(19) Europäisches Patentamt / European Patent Office / Office européen des brevets

(11) **EP 4 365 308 A1**

(12) **DEMANDE DE BREVET EUROPEEN**

(43) Date de publication:
**08.05.2024 Bulletin 2024/19**

(21) Numéro de dépôt: **22205313.4**

(22) Date de dépôt: **03.11.2022**

(51) Classification Internationale des Brevets (IPC):
***C12Q 1/6883*** (2018.01)

(52) Classification Coopérative des Brevets (CPC):
**C12Q 1/6883;** C12Q 2600/158

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(71) Demandeur: **BIOMÉRIEUX**
**69280 Marcy L'Etoile (FR)**

(72) Inventeurs:
• **MOMMERT, Marine**
  **69003 Lyon (FR)**

• **BRENGEL PESCE, Karen**
  **38490 Les Abrets en Dauphine (FR)**
• **ORIOL, Guy**
  **42400 Saint Chamond (FR)**
• **GUICHARD, Audrey**
  **38440 Moidieu-Detourbe (FR)**

(74) Mandataire: **bioMérieux PI Groupement mandataires**
**bioMérieux**
**69280 Marcy l'Etoile (FR)**

(54) **METHODE POUR DETERMINER LA NATURE VIRALE OU BACTERIENNE D'UNE INFECTION**

(57) La présente invention a pour objet des méthodes in vitro ou ex vivo et des kits pour déterminer à partir d'un échantillon biologique d'un sujet la nature d'une infection, à savoir virale ou bactérienne, par l'intermédiaire de l'identification de la variation du niveau d'expression de plusieurs biomarqueurs. En particulier, la méthode comprenant les étapes de (a) mesure de l'expression d'au moins un gène cible viral et d'au moins un gène cible bactérien choisi parmi OLAH, FAM20A, IL1R2, MMP8, RETN et SLC1A2, de (b) comparaison des expressions mesurées à l'étape (a) avec des valeurs d'expression de références desdits gènes cibles, et de (c) conclusion quant à la nature virale ou bactérienne de l'infection sur la base des résultats de comparaison.

**EP 4 365 308 A1**

**Description**

La présente invention concerne le domaine technique des méthodes et kits de diagnostic *in vitro*. En particulier, l'invention a pour objet des méthodes et kits pour déterminer à partir d'un échantillon biologique d'un sujet la nature d'une infection, à savoir virale ou bactérienne, par l'intermédiaire de l'identification de la variation du niveau d'expression de plusieurs biomarqueurs.

**TECHNIQUE ANTERIEURE**

Les virus et les bactéries interagissent avec différents récepteurs de reconnaissance à la surface des leucocytes présents dans le sang circulant. Cette interaction entraine chez l'hôte des évènements transcriptionnels spécifiques qui régulent la réponse immunitaire (Takeuchi et al. 2010). Par conséquent, l'activation différentielle des programmes transcriptionnels de l'hôte génère des signatures transcriptomiques uniques qui peuvent permettre de distinguer les causes virales des causes bactériennes.

Dans ce contexte, l'analyse du profil transcriptomique de l'hôte peut fournir une approche indirecte de la détection de la nature d'une infection, complétant ainsi les approches directes, telles que la culture ou l'amplification des acides nucléiques (Ramilo et al. 2009). Cela représente également un intérêt particulier car les infections virales sont fréquemment la cause de fièvre sans source apparente, notamment chez les jeunes enfants.

De plus, en considérant la pratique courante dans les hôpitaux à administrer systématiquement, à titre préventif, des antibiotiques aux patients fébriles jusqu'à l'obtention des résultats des tests de culture implique malheureusement que de nombreuses infections virales sont traitées à tort par des médicaments antimicrobiens.

En outre, il a été démontré que l'administration excessive d'antibiotiques conduit à une augmentation de la résistance bactérienne, non seulement au niveau individuel mais aussi à un niveau plus global. Par conséquent, le mauvais usage et la surutilisation des antibiotiques disponibles contribuent activement au développement des résistances antimicrobiennes (Fauci et al. 2014). De surcroit, en raison des difficultés à distinguer les étiologies virales, bactériennes ou non infectieuses, un certain nombre de patients sont traités de manière inappropriée avec des antibiotiques à des taux élevés.

Une distinction précoce entre les patients présentant une infection virale et ceux présentant une infection bactérienne pourrait donc permettre une prise en charge plus précise et plus fine desdits patients, tout en réduisant de manière significative l'utilisation inutile d'antibiotique.

Depuis quelques années, la découverte de biomarqueurs capables de distinguer, à partir de l'expression génétique du sang total, les infections virales des infections bactériennes présentant des phénotypes cliniques initiaux similaires, suscite un intérêt croissant. Des auteurs ont par exemple décrit que les profils transcriptionnels des enfants fébriles viropositifs et des enfants fébriles atteints d'infection bactérienne aiguë diffèrent des profils des enfants non fébriles viropositifs et négatifs (Hu et al. 2013).

Des efforts croissants sont ainsi déployés pour développer des biomarqueurs de l'hôte permettant de distinguer les infections virales des infections bactériennes, notamment chez les enfants fébriles (D. Brown et al. 2016).

Cet intérêt découle non seulement de la nécessité de distinguer les infections bactériennes potentiellement mortelles des infections virales, mais aussi d'éviter la prescription inutile d'une antibiothérapie empirique, quelle que soit la gravité de l'infection. Comme mentionné précédemment, la surconsommation d'antibiotiques dans le monde entier accélère la résistance aux antimicrobiens (AMR), qui est reconnue par l'Organisation Mondiale de la Santé comme l'une des plus grandes menaces pour la santé humaine dans les années à venir.

Par exemple, le document WO2018011316 décrit une méthode pour identifier un sujet présentant une infection bactérienne. En particulier, la méthode consiste à détecter dans un échantillon d'ARNm la modulation de l'expression de 2 à 10 gènes choisis parmi la signature génique suivante : IFI44L, FAM89A, IFI27L, IFTI1, RSAD2, IFIT3, OTOF, IFIT2, EPSTI1, SERPING1, OAS1, IFI6, HLA-DRB6, HBZ, HS.386275, EIF2AK2, IFIT1L, FCER1A, C21ORF7, GYPE, GYPB, HBM, EIF1AY, LOC649143, HBD, FBX07, KCNMA1, MERTK, EBI3, UPB1, EMR1, PTPN20, TMEM119, SLPI, S100P et PI3.

La discrimination entre une infection virale et une bactérienne à partir de d'une signature transcriptomique a également été décrite par d'autres auteurs. En particulier, à partir d'une cohorte indépendante de 623 patients, lesquels présentaient une infection bactérienne, une infection virale, une co-infection ou une maladie non infectieuse, une signature de 45 ARNm a été identifiée pour discriminer entre une infection virale ou bactérienne (E L.Tsalik et al., 2021).

Cependant, des efforts supplémentaires sont nécessaires pour évaluer la précision et l'utilité diagnostique des biomarqueurs, notamment transcriptomiques, avant qu'ils puissent être transformés en test cliniquement applicable pour déterminer l'origine virale ou bactérienne d'une infection.

Ainsi, bien que des solutions existent, il demeure toujours un besoin de développer de nouvelles signatures transcriptomiques permettant d'identifier la nature d'une infection et de discriminer ainsi les étiologies virales et bactériennes. Cela revêt une importance toute particulière afin d'améliorer la prise en charge des patients, notamment des enfants fébriles, de réduire l'utilisation inappropriée d'antibiotiques et participer à la lutte contre le développement de la résistance aux antibiotiques.

**Résumé**

La présente invention est basée sur l'identification de signatures transcriptomiques associées aux infections virales et bactériennes. La méthode selon l'invention permet d'identifier chez un sujet diagnostiqué comme ayant une infection, ou suspecté d'avoir une infection, la nature de ladite infection, afin de sélectionner la prise en charge la plus adaptée. De manière tout à fait avantageuse, la méthode selon l'invention permet d'exclure la présence d'une infection bactérienne et de limiter l'utilisation inappropriée d'antibiotiques.

Ainsi, un premier aspect de l'invention concerne une méthode *in vitro* ou *ex vivo* pour déterminer la nature d'une infection chez un sujet par la mesure, à partir d'un échantillon biologique dudit sujet, de la variation du niveau d'expression d'au moins un gène cible viral et d'au moins un gène cible bactérien choisi parmi OLAH, FAM20A, IL1R2, MMP8, RETN et SLC1A2.

La méthode selon l'invention, à partir d'un échantillon biologique d'un sujet infecté ou susceptible d'être infecté, comprend ainsi les étapes suivantes:

(a) mesure de l'expression d'au moins un gène cible viral et d'au moins un gène cible bactérien choisi parmi les gènes cibles bactériens OLAH, FAM20A, IL1R2, MMP8, RETN et SLC1A2,

(b) comparaison des expressions mesurées à l'étape (a) avec des valeurs d'expression de références prédéterminées desdits gènes cibles, et

(c) conclusion quant à la nature virale ou bactérienne de l'infection sur la base des résultats de comparaison.

Les avantages de la méthode selon l'invention sont nombreux, notamment :

(i) Différencier de façon précise la nature virale ou bactérienne de l'infection avec un niveau de performance élevée, la majorité des combinaisons de biomarqueurs ayant une performance en termes d'aire sous la courbe (AUC) d'au moins 0,85, voire d'au moins 0,90 parfois,

(ii) Exclure la présence d'une infection bactérienne, .

(iii) Identifier rapidement des variations significatives du niveau d'expression des gènes, notamment par la mise en œuvre de systèmes automatisés ou par l'intermédiaire des kits de détection selon l'invention,

(iv) Eviter l'identification de faux positifs correspondant à la présence de bactéries ou de virus non pathogéniques naturellement présents chez les sujets,

(v) Permettre une identification de la nature de l'infection lorsque le pathogène n'est pas identifiable ou lorsqu'il est inaccessible. En effet, la présente méthode ne nécessite pas un accès direct au pathogène, seul un échantillon biologique du sujet est nécessaire.

Après de nombreuses recherches, il est ainsi du mérite des inventeurs d'avoir identifié une signature transcriptomique dont l'expression est caractéristique de la nature virale ou bactérienne de l'infection et d'utiliser la mesure de la variation de cette expression pour identifier la source de l'infection. Cela est d'autant plus remarquable qu'il est particulièrement difficile d'identifier des gènes cibles bactériens performants qui permettent notamment, lorsqu'ils sont combinés avec des gènes cibles viraux, d'exclure la présence d'une infection bactérienne.

L'identification de la nature d'une infection présente un intérêt certain en permettant notamment d'accompagner le clinicien dans une prise en charge plus adaptée et personnalisée, notamment pour éviter la prescription inutile d'antibiotique lorsque l'infection est de nature virale ou lorsque la présence d'une infection bactérienne est exclue.

De préférence, l'étape (a) de mesure comprend ou consiste en la mesure d'au moins un gène cible viral choisi parmi les gènes viraux de l'hôte impliqués dans la voie des cytokines pro-inflammatoires, dans la voie de l'interféron, dans la voie de signalisation des récepteurs Toll-like (« Toll-like receptors » ou TLR), dans la voie de signalisation des récepteurs RIG-I-like (« RIG-I-like receptors » ou RLR) et dans la voie de la présentation antigénique médiée par le Complexe Majeur d'Histocompatibilité (CMH) de classe II, et d'au moins un gène cible bactérien choisi parmi OLAH, FAM20A, IL1R2, MMP8, RETN et SLC1A2.

De préférence, l'étape (a) de mesure comprend ou consiste en la mesure d'au moins un gène cible viral choisi parmi les gènes viraux de l'hôte impliqués dans la voie de l'interféron et de la présentation antigénique médiée par le CMH de classe II, et d'au moins un gène cible bactérien choisi parmi OLAH, FAM20A, IL1R2, MMP8, RETN et SLC1A2.

De préférence, l'étape (a) de mesure comprend ou consiste en la mesure d'au moins un gène cible viral choisi parmi les gènes viraux de l'hôte impliqués dans la voie de la voie de l'interféron, et en particulier, lesdits gènes sont choisis parmi les gènes cibles viraux IFI27, IFN-$\alpha$, IFN-$\beta$, IFN-$\epsilon$, IFN-$\kappa$, IFN-$\omega$, IFN-$\gamma$, IFNL1, IFNL2, IFNL3, ADAR1, IFIT1, IFIT2, IFIT3, IFIT5, IFI44L, ISG15, ISG20, MDA5, OAS1, OAS2, OAS3, OASL, PARP12, PKR, RIG-I, RNaseL, TRIM25, ZAP, ZCCHC3, ZNFX1, RBBP6, SHFL, TRIM22, TRIM25, TRIM32, TRIM69, Viperin, HERC1, HERC2, HERC3, HERC4, HERC5, HERC6 et leurs combinaisons, et d'au moins un gène cible bactérien choisi parmi OLAH, FAM20A, IL1R2, MMP8, RETN et SLC1A2.

De préférence, l'étape (a) de mesure comprend ou consiste en la mesure d'au moins un gène cible viral choisi parmi RSAD2, IFI27, OAS1, IFIT1, IFI44L, SIGLEC1, ISG15 et HERC6, et d'au moins un gène cible bactérien choisi parmi OLAH, FAM20A, IL1R2, MMP8, RETN et SLC1A2.

De préférence, l'étape (a) de mesure comprend ou consiste en la mesure d'au moins un gène cible viral choisi parmi SIGLEC1, ISG15 et HERC6, et d'au moins un gène cible bactérien choisi parmi OLAH, FAM20A, IL1R2, MMP8, RETN et SLC1A2.

De préférence, l'étape (a) comprend ou consiste en la mesure d'au moins un gène cible viral choisi parmi IFI27, SIGLEC1, ISG15, HERC6, RSAD2, OAS1, IFIT1, IFI44L et d'au moins un gène cible bactérien choisi parmi OLAH, FAM20A, IL1R2, MMP8, RETN et SLC1A2,

De préférence, l'étape (a) comprend la mesure de l'expression du gène cible viral IFI27 et du gène cible bactérien OLAH, et de préférence encore, également la mesure de l'expression du gène cible bactérien FAM20A.

De préférence, l'étape (a) de mesure comprend ou consiste en la mesure de l'expression de deux gènes cibles viraux choisis parmi IFI27, SIGLEC1, ISG15, HERC6, RSAD2, OAS1, IFIT1 et IFI44L et de deux gènes cibles bactériens choisis parmi OLAH, FAM20A, IL1R2, MMP8, RETN et SLC1A2, et de préférence choisis parmi OLAH, FAM20A, IL1R2 et MMP8.

De préférence, l'étape (a) de mesure comprend ou consiste en la mesure de l'expression de deux à cinq gènes cibles viraux choisis parmi SIGLEC1, ISG15, HERC6, RSAD2, IFI27, OAS1, IFIT1 et IFI44L et de trois ou quatre gènes cibles bactériens choisis parmi OLAH, FAM20A, IL1R2, MMP8, RETN et SLC1A2, et de préférence choisis parmi OLAH, FAM20A, IL1R2 et MMP8.

De préférence, l'étape (a) de mesure comprend ou consiste en la mesure de l'expression du gène cible viral IFI27, et éventuellement d'au moins un autre gène viral choisi parmi SIGLEC1, ISG15, HERC6, RSAD2, OAS1, IFIT1, IFI44L, et d'au moins deux gènes cibles bactériens choisis parmi OLAH, FAM20A, IL1R2, MMP8, RETN et SLC1A2, et de préférence choisis parmi OLAH, FAM20A, IL1R2 et MMP8.

De préférence, l'étape (a) comprend ou consiste en la mesure d'au moins un gène cible viral choisi parmi SIGLEC1, ISG15 et HERC6, et d'au moins un gène cible bactérien choisi parmi OLAH, FAM20A, IL1R2, MMP8, RETN et SLC1A2, de préférence parmi IL1R2, OLAH et FAM20A.

De préférence, l'étape (a) comprend ou consiste en la mesure d'au moins un gène cible viral choisi parmi IFI27, SIGLEC1, ISG15, HERC6, RSAD2, OAS1, IFIT1 et IFI44L, du gène cible bactérien OLAH, et d'au moins un autre gène cible bactérien choisi parmi FAM20A, IL1R2, MMP8, RETN et SLC1A2, de préférence parmi FAM20A, IL1R2 et MMP8.

De préférence, l'étape (a) comprend ou consiste en la mesure d'au moins un gène cible viral choisi parmi IFI27, SIGLEC1, ISG15, HERC6, RSAD2, OAS1, IFIT1 et IFI44L, du gène cible bactérien FAM20A, et d'au moins un autre gène cible bactérien choisi parmi OLAH, IL1R2, MMP8, RETN et SLC1A2, de préférence parmi OLAH, IL1R2 et MMP8.

De préférence, l'étape (a) comprend ou consiste en la mesure de l'expression du gène cible viral IFI27, d'un autre gène cible viral choisi parmi HERC6, SIGLEC1, IFI44L et ISG15, du gène cible bactérien FAM20A et d'un autre gène cible bactérien choisi parmi OLAH et IL1R2.

De préférence, l'étape (a) comprend ou consiste en la mesure de l'expression du gène cible viral IFI27, de deux autres gènes cibles viraux choisis parmi SIGLEC1, HERC6, OAS1 et IFIT1, du gène cible bactérien FAM20A et d'un autre gène cible bactérien choisi parmi OLAH, IL1R2 et MMP8.

De préférence, l'étape (a) comprend ou consiste en la mesure de l'expression des gènes cibles viraux IFI27 et SIGLEC1, d'un autre gène cible viral choisi parmi IFIT1, HERC6, ISG15 et ISG15, des gènes cibles bactériens FAM20A et MMP8, et d'un autre gène cible bactérien choisi parmi OLAH et IL1R2.

De préférence, l'étape (a) comprend ou consiste en la mesure de l'expression des gènes cibles viraux IFI27 et SIGLEC1, et de deux autres gènes cibles viraux choisis parmi IFIT1, OAS1, HERC6 et ISG15, des gènes cibles bactériens FAM20A et MMP8, et d'un autre gène cible bactérien choisi parmi OLAH et IL1R2.

De préférence, l'étape (a) consiste en la mesure d'une combinaison de gène cibles viraux et bactériens choisie parmi les combinaisons de gènes cibles listés dans les tableaux 6 à 12 et qui présentent une AUC du modèle pour la détermination de la nature virale ou bactérienne de l'infection d'au moins 0,90.

De préférence, dans la méthode selon l'invention, la valeur d'expression de référence des gènes cibles correspond à l'expression respectives desdits gènes cibles dans un échantillon biologique de référence obtenu chez un sujet ayant une infection bactérienne. Ainsi, il pourra être conclu à une infection de nature virale lorsque la comparaison du niveau d'expression des gènes cibles au niveau des transcrits ARNm par rapport aux valeurs de référence respectives, met en évidence au moins une variation d'expression choisie parmi:

- une surexpression de SIGLEC1,

- une surexpression de ISG15,

- une surexpression de HERC6,

- une surexpression de RSAD2,

- une surexpression de IFI27,

- une surexpression de OAS1,

- une surexpression de IFIT1, et

- une surexpression de IFI44L,

et au moins une autre variation d'expression choisie parmi :

- une sous expression de SLC1A2,

- une sous expression de IL1R2,

- une sous expression de FAM20A,

- une sous expression de OLAH,

- une sous expression de RETN, et

- une sous expression de MMP8.

De préférence encore, dans la méthode selon l'invention la valeur d'expression de référence desdits gènes cibles correspond à l'expression respectives desdits gènes cibles dans un échantillon biologique de référence obtenu chez un sujet ayant une infection virale. Ainsi, il peut être conclu à une infection de nature bactérienne lorsque les résultats de comparaison d'expression des gènes cibles met en évidence au moins deux variations choisies parmi les variations suivantes :

- une sous expression de SIGLEC1,

- une sous expression de ISG15,

- une sous expression de HERC6,

- une sous expression de RSAD2,

- une sous expression de IFI27,

- une sous expression de OAS1,

- une sous expression de IFIT1, et

- une sous expression de IFI44L,

et au moins une autre variation d'expression choisie parmi :

- une surexpression de SLC1A2,

- une surexpression de IL1R2,

- une surexpression de FAM20A,

- une surexpression de OLAH,

- une surexpression de RETN, et

- une surexpression de MMP8.

De préférence, la méthode selon l'invention comprend en outre une étape (a') de mesure de l'expression d'au moins un gène cible additionnel choisi parmi PI3, EBI3, ADGRE1 et S100P, une étape (b') de comparaison des expressions mesurées à l'étape (a') avec des valeurs d'expression de références desdits gènes cibles additionnels et une étape (c') de conclusion quant à la nature virale ou bactérienne de l'infection sur la base des résultats comparaisons.

De préférence, dans la méthode selon l'invention, la mesure de l'expression des gènes cibles est réalisée au niveau des ARNm.

De préférence, dans la méthode selon l'invention, la mesure de la variation d'expression est réalisée par amplification via une RT-PCR, de préférence une RT-PCR quantitative, ou une PCR nichée.

De préférence, dans la méthode selon l'invention, l'expression est normalisée par rapport à l'expression d'un ou plusieurs gènes de ménage choisis parmi DECR1, HPRT1, PPIB, GAPDH, et ACTB.

De préférence, dans la méthode selon l'invention le sujet est un enfant, de préférence un enfant de moins de 4 ans, et de préférence encore, un enfant de moins de 4 ans.

De préférence, dans la méthode selon l'invention, l'échantillon biologique est un échantillon de sang, de préférence un échantillon de sang total.

L'invention concerne également un kit de mesure *in* vitro ou *ex vivo* de l'expression d'au moins un gène cible viral choisi parmi SIGLEC1, ISG15, HERC6, RSAD2, IFI27, OAS1, IFIT1, IFI44L, et d'au moins un gène cible bactérien choisi parmi OLAH, FAM20A, IL1R2, MMP8, RETN et SLC1A2, ledit kit comprenant des réactifs spécifiques des produits d'expression desdits gènes cibles, lesdits réactifs étant de préférence des amorces ou des sondes. Le kit est particulièrement adapté pour la mise en œuvre de la méthode selon l'invention et permet ainsi de déterminer la nature virale ou bactérienne d'une infection à partir d'un échantillon biologique d'un sujet.

**BREVE DESCRIPTION DES FIGURES**

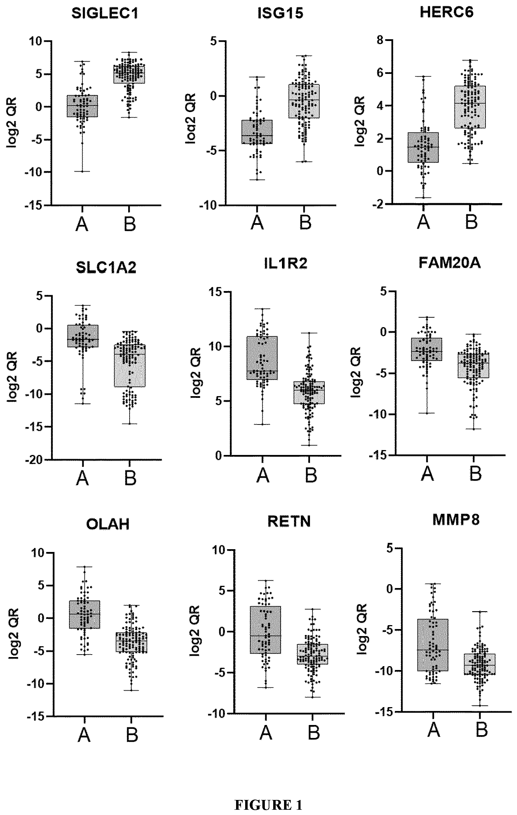

La Figure 1 représente les box plot de l'expression des gènes cibles SIGLEC1, ISG15, HERC6, SLC1A2, IL1R2, FAM20A, OLAH, RETN et MMP8 obtenue à partir des valeurs brutes de RT-PCR normalisées avec les gènes de ménage DECR1, HPRT1 et PPIB ; A : Infection bactérienne (n=68) ; B : Infection virale (n=123).

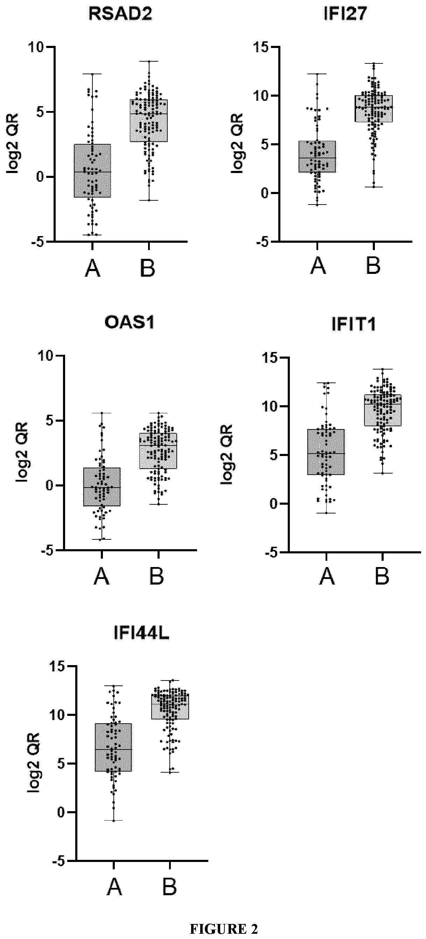

La Figure 2 représente les box plot de l'expression des gènes cibles RSAD2, IFI27, OAS1, IFIT1 et IFI44L obtenue à partir des valeurs brutes de RT-PCR normalisées avec les gènes de ménage DECR1, HPRT1 et PPIB ; A : Infection bactérienne (n=68) ; B : Infection virale (n=123).

**DESCRIPTION DETAILLEE**

Le transcriptome est l'ensemble des ARNs issus de la transcription du génome. L'analyse transcriptomique peut caractériser le transcriptome entier ou partiel, d'un tissu particulier, d'un type cellulaire, ou comparer les transcriptomes entre différentes conditions expérimentales ou cliniques. Les agents pathogènes tels que les virus et les bactéries interagissent chez l'hôte avec différents récepteurs de reconnaissance à la surface des leucocytes présents dans le sang circulant. Comme mentionné précédemment, cette interaction entraine des évènements transcriptionnels spécifiques qui régulent la réponse immunitaire, générant ainsi des signatures transcriptomiques spécifiques.

Les signatures transcriptomiques peuvent donc être des outils d'aide à la décision basées sur une analyse de transcrits de gènes préalablement sélectionnés.

Un premier objet de l'invention concerne une méthode *in vitro* ou *ex vivo* pour déterminer la nature virale ou bactérienne d'une infection chez un sujet. Plus particulièrement, les inventeurs ont identifié que la mise en évidence, à partir d'un échantillon biologique d'un sujet infecté ou susceptible d'être infecté, de la variation du niveau d'expression de plusieurs gènes cibles permettait de caractériser la nature virale ou bactérienne de l'infection. En d'autres termes, la méthode selon l'invention permet d'exclure la présence d'une infection bactérienne.

Ainsi, la méthode selon l'invention comprend les étapes suivantes de :

(a) mesure, à partir de l'échantillon biologique d'un sujet infecté, de l'expression d'au moins un gène cible viral et d'au moins un gène cible bactérien choisi parmi les gènes cibles bactériens OLAH, FAM20A, IL1R2, MMP8, RETN et SLC1A2,

(b) comparaison des expressions mesurées à l'étape (a) avec des valeurs d'expression de références prédéterminées desdits gènes cibles, et

(c) conclusion quant à la nature virale ou bactérienne de l'infection dudit sujet sur la base des résultats de comparaison.

L'expression « gène cible viral », fait référence à un gène de l'hôte dont l'expression est dérégulée en présence d'une infection virale, c'est-à-dire un gène dont l'expression est significativement augmentée ou diminuée en présence de ladite infection.

L'expression « gène cible bactérien », fait référence à un gène de l'hôte dont l'expression est dérégulée en présence d'une infection bactérienne, c'est-à-dire un gène dont l'expression est significativement augmentée ou diminuée en présence de ladite infection.

Au sens de la présente description, l'expression « gène cible » fait indifféremment référence à un gène cible viral ou un gène cible bactérien à moins que le contexte ne permette d'identifier clairement qu'il s'agisse d'un gène cible viral ou d'un gène cible bactérien.

Les termes « biomarqueur » ou « marqueur », font référence à une caractéristique biologique mesurable objectivement qui représente un indicateur des processus biologiques normaux ou pathologiques suite à la présence d'une infection. Ainsi, Au sens de la présente invention, les biomarqueurs sont les gènes cibles viraux et les gènes cibles bactériens, et tout particulièrement les transcrits desdits gènes cibles.

Pour l'ensemble de la présente description, lorsqu'il est fait référence à la mesure de l'expression « d'au moins un gène cible bactérien choisi parmi OLAH, FAM20A, IL1R2, MMP8, RETN et SLC1A2», cela inclut la possibilité de la mesure de l'expression de plusieurs gènes cibles, englobant ainsi de facto toutes les combinaisons possibles de 2 à 6 gènes cibles bactériens sans qu'il soit nécessaire d'en donner la liste exhaustive. Ceci s'applique pour toutes les listes de gènes cibles introduites dans la présente description par l'expression « au moins » et concerne donc également la mesure de l'expression des gènes cibles viraux.

L'expression « sujet infecté », fait référence à un sujet ayant une infection, en d'autres termes, un sujet ayant été diagnostiqué comme ayant une infection. Le diagnostic de la présence de l'infection peut être réalisé par n'importe quelle méthode connue de la personne du métier permettant d'apporter une telle conclusion. Le diagnostic peut ainsi être réalisé par une méthode moléculaire, comme par exemple un dosage protéique de la procalcitonine (PCT), ou directement par le clinicien sur la base de(s) symptôme(s) ou signe(s) clinique(s) du sujet.

Les symptômes ou signes cliniques associés à la présence d'une infection sont également bien connus de la personne du métier, et on citera à titre d'exemple les maux de tête, des douleurs dans une partie spécifique du corps (l'abdomen par exemple), de la fièvre (>38°C°) avec ou pas des frissons, une température corporelle inférieure à 35,5°C, un symptôme respiratoire choisi dans le groupe comprenant la toux, l'expectoration, la dyspnée, la tachypnée et la douleur pleurétique; une constatation à l'auscultation, une nausée avec ou sans vomissement, une pression artérielle systolique supérieure à 90 mmHg, une fréquence cardiaque supérieure à 120 battements/min, un symptôme d'infection d'un organe ou d'un système organique choisi dans le groupe constitué par les infections des voies respiratoires, les infections des voies digestives, les infections vaginales, les méningites, les septicémies, les érysipèles, les péritonites, les cholangites, les cholécystites et les ostéomyélites.

Dès lors, la méthode selon l'invention permet d'identifier la nature de l'infection dudit sujet.

L'expression « nature de l'infection» fait référence uniquement à l'étiologie de ladite infection, à savoir une infection de nature virale ou une infection de nature bactérienne. La méthode selon l'invention permet ainsi de distinguer une infection virale d'une infection bactérienne, ou inversement. En d'autres termes, la méthode selon l'invention permet d'exclure la présence d'une infection bactérienne.

Au sens de la présente description, le terme « sujet » désigne un être humain et de préférence, le sujet est un patient. Par définition, le patient est une personne entrée en contact avec un professionnel de santé, notamment un médecin, une structure médicale ou un établissement de santé.

Les gènes cibles impliqués dans la méthode selon l'invention sont bien connus de la personne du métier mais il est du mérite des inventeurs d'avoir identifié que des signatures transcriptomiques basées sur la variation d'expression desdits gènes pouvaient permettre de déterminer de manière efficace la nature virale ou bactérienne d'une infection.

En effet, la personne du métier est en mesure d'identifier les gènes cibles viraux, pour la mise en œuvre de la méthode selon l'invention. Il en est de même pour les positions chromosomiques desdits gènes cibles qui sont accessibles dans les bases de données publiques, comme par exemple dans la base Ensembl (assemblage GRCh38/hg38).

Ainsi, n'importe quel gène cible viral, dont l'expression est significativement augmentée ou diminuée en présence d'une infection peut être utilisé dans la méthode selon l'invention. De tels gènes sont connus de l'homme du métier.

Par conséquent, les gènes cibles viraux peuvent être choisis parmi les gènes de la voie de l'interféron, de la voie des cytokines pro-inflammatoires, de la voie de signalisation des récepteurs Toll-like (« Toll-like receptors » ou TLR), de la voie de signalisation des récepteurs RIG-I-like (« RIG-I-like receptors » ou RLR), ou encore, de la voie de la présentation antigénique médiée par le CMH de classe II.

Les gènes cibles viraux de la voie de l'interféron, notamment les gènes stimulés par les interférons, dits gènes ISG, sont bien connus de la personne du métier (Schneider et al, 2014 ; Yang et al. 2020). A titre d'exemple de ces gènes, on peut notamment citer IFN-$\alpha$, IFN-$\beta$, IFN-$\epsilon$, IFN-$\kappa$, IFN-$\omega$, IFN-$\gamma$, IFNL1, IFNL2, IFNL3, ADAR1, IFIT1, IFIT2, IFIT3, IFIT5, IFI27, IFI44L, ISG15, ISG20, MDA5, OAS1, OAS2, OAS3, OASL, PARP12, PKR, RIG-I, RNaseL, RSAD2, RBBP6, SIGLEC1, SHFL, TRIM22, TRIM25, TRIM32, TRIM69, Viperin, ZAP, ZCCHC3, et ZNFX1.

Les gènes cibles viraux de la voie des cytokines pro-inflammatoires sont également bien connus de la personne du métier (Mogensen et al. 2001). A titre d'exemple de ces gènes, on citera notamment TNF$\alpha$, IL-1$\beta$, IL-6, IL-IRa, IL-8, GM-CSF, MIP-1$\alpha$, MIP-1$\beta$, IL-10, IL-1$\alpha$, TGF-$\beta$, TGF-$\beta$1, IL-2, IL-4, IL-13, IL-15, IL-3, IL-5, IP10, et SCM-1.

Ainsi, selon un mode de réalisation particulier, l'étape (a) de mesure comprend ou consiste en la mesure d'au moins un gène cible viral choisi parmi les gènes viraux de l'hôte impliqués dans la voie des cytokines pro-inflammatoires, et en particulier choisis parmi les gènes cibles viraux TNF$\alpha$, IL-1$\beta$, IL-6, IL-IRa, IL-8, GM-CSF, MIP-1$\alpha$, MIP-1$\beta$, IL-10, IL-1$\alpha$, TGF-$\beta$, TGF-$\beta$1, IL-2, IL-4, IL-13, IL-15, IL-3, IL-5, IP10, SCM-1 et leurs combinaisons, et d'au moins un gène cible bactérien choisi parmi OLAH, FAM20A, IL1R2, MMP8, RETN et SLC1A2.

Les gènes cibles viraux de la voie des récepteurs Toll-like (« Toll-like receptor ») sont également bien connus de la personne du métier (Kawasaki et al., 2014) et comprennent notamment les gènes TLR natifs, les gènes co-récepteurs des TLR (« TLR co-receptor genes »), les gènes régulateurs des TLR (« TLR regulator genes »), les gènes inhibiteurs de la signalisation des TLR (« TLR signaling inhibitors »), les gènes adaptateurs des TLR (« TLR adaptator genes »), et les gènes de signalisation des TLR (« TLR signaling genes »).

A titre d'exemple de gènes TLR natifs, on citera notamment les gènes TLR3, TLR7, TLR8 et TLR9.

A titre d'exemple de gènes co-récepteurs des TLR, on citera notamment les gènes CD14, LBP, MD1 et MD2.

A titre d'exemple de gènes régulateurs des TLR, on citera notamment les gènes CD300LF, GRP94, PRAT4A, PRAT4B et UNC93B1.

A titre d'exemple de gènes inhibiteurs de la signalisation des TLR, on citera notamment les gènes ATF3, AXL, BAMBI, BCL3, BPI, CENTB1, DAK, FLII, HSP10, IRAKM(IRAK3), LGP2(DHX58), MSK1, MSK2, NLRP12(NALP12), NFKBIA, NFKBIB, NFKBIE, PECAM1, PIN1, PPP3CA, PPP3CB, PPP3R1, PTPN6, RNF125, RP105, SHIP1, SIGIRR, SIKE, TNFAIP3, TNIP1, TNIP2, TNIP3, TOLLIP, TRAFD1, TRIAD3, TYRO3 et ZCCHC11.

A titre d'exemple de gènes adaptateurs des TLR, on citera notamment les gènes MyD88, RAC1, SARM1, TANK, TIRAP, TRAM(TICAM2) et TRIF(TICAM1).

A titre d'exemple de gènes de signalisation des TLR, on citera notamment les gènes AAMP, ACT1(TRAF3IP2), AGER, AKT1, BECN1, BTK, CARD6, CARD11, DDX3X, FADD, IKK$\alpha$, IKK$\beta$, IKK$\epsilon$, IPS1, IRAK1, IRAK4, IRF1, IRF3, IRF5, IRF7, IRF8, IRF9, ITCH, LRRC59, LRRFIP2, MAP2K2, MAP3K7(TAK1), MAPK1, MDA5(IFIH1), NAIP5, NAP1, NEMO, NIBP, OTUD5, PKR, PRKRA, RIG-1, RIPK1, RIPK2, RIPK3, STAP2, SUGT1, SYK, TAB1, TAB2, TAB3, TBK1, TBKBP1, TIFA, TRADD, TRAF3, TRAF6, TRIL et WDFY1.

Ainsi, selon un mode de réalisation particulier, l'étape (a) de mesure comprend ou consiste en la mesure d'au moins un gène cible viral choisi parmi les gènes viraux de l'hôte impliqués dans la voie de signalisation des récepteurs TLR, et en particulier choisis parmi TLR3, TLR7, TLR8, TLR9, CD14, LBP, MD1, MD2, CD300LF, GRP94, PRAT4A, PRAT4B, UNC93B1 ATF3, AXL, BAMBI, BCL3, BPI, CENTB1, DAK, FLII, HSP10, IRAKM(IRAK3), LGP2(DHX58), MSK1, MSK2, NLRP12(NALP12), NFKBIA, NFKBIB, NFKBIE, PECAM1, PIN1, PPP3CA, PPP3CB, PPP3R1, PTPN6, RNF125, RP105, SHIP1, SIGIRR, SIKE, TNFAIP3, TNIP1, TNIP2, TNIP3, TOLLIP, TRAFD1, TRIAD3, TYRO3, ZCCHC11, MyD88, RAC1, SARM1, TANK, TIRAP, TRAM(TICAM2), TRIF(TICAM1), AAMP, ACT1(TRAF3IP2), AGER, AKT1, BECN1, BTK, CARD6, CARD11, DDX3X, FADD, IKK$\alpha$, IKK$\beta$, IKK$\epsilon$, IPS1, IRAK1, IRAK4, IRF1, IRF3, IRF5, IRF7, IRF8, IRF9, ITCH, LRRC59, LRRFIP2, MAP2K2, MAP3K7(TAK1), MAPK1, MDA5(IFIH1), NAIP5, NAP1, NEMO, NIBP, OTUD5, PKR, PRKRA, RIG-1, RIPK1, RIPK2, RIPK3, STAP2, SUGT1, SYK, TAB1, TAB2, TAB3, TBK1, TBKBP1, TIFA, TRADD, TRAF3, TRAF6, TRIL, WDFY1 et leurs combinaisons, et d'au moins un gène cible bactérien choisi parmi OLAH, FAM20A, IL1R2, MMP8, RETN et SLC1A2.

Les gènes cibles viraux de la voie de signalisation des récepteurs RIG-I-like sont également connus de la personne du métier. A titre d'exemple, on peut citer les gènes LGP2(DHX58), MDA5(IFIH1), RIG-I(DDX58), TRAFD1, CYLD, DAK, DDX60, MFN2, MUL1, OTUD5, PIN1, RNF125, SIKE, TNFAIP3, IPS1, IPS1-HA, PARP13, STING, TOMM70A, TRIM13, TRIM26, TRIM32 et ZDHHC1.

Ainsi, selon un mode de réalisation particulier, l'étape (a) de mesure comprend ou consiste en la mesure d'au moins un gène cible viral choisi parmi les gènes viraux de l'hôte impliqués dans la voie de signalisation des récepteurs RLR, et en particulier, lesdits gènes sont choisis parmi les gènes cibles viraux LGP2(DHX58), MDA5(IFIH1), RIG-I(DDX58), TRAFD1, CYLD, DAK, DDX60, MFN2, MUL1, OTUD5, PIN1, RNF125, SIKE, TNFAIP3, IPS1, IPS1-HA, PARP13, STING, TOMM70A, TRIM13, TRIM26, TRIM32, ZDHHC1 et leurs combinaisons, et d'au moins un gène cible bactérien choisi parmi OLAH, FAM20A, IL1R2, MMP8, RETN et SLC1A2.

Enfin, les gènes cibles viraux de la voie de la présentation antigénique médiée par le CMH de classe II sont également bien connus de la personne du métier. A titre d'exemple, on peut citer les gènes cibles de la famille des ubiquitine ligases, notamment HERC1, HERC2, HERC3, HERC4, HERC5 et HERC6 (Hochrainer et al., 2005).

Ainsi, selon un mode de réalisation particulier, les gènes cibles viraux dont l'expression est mesurée à l'étape (a) sont choisis parmi les gènes cibles viraux de la voie des cytokines pro-inflammatoires, de la voie de l'interféron, de la voie de signalisation des récepteurs Toll-like (« Toll-like receptors » ou TLR), de la voie de signalisation des récepteurs RIG-I-like (« RIG-I-like receptors » ou RLR), et de la voie de la présentation antigénique médiée par le CMH de classe II.

Selon un mode de réalisation particulier, les gènes cibles viraux dont l'expression est mesurée à l'étape (a) sont choisis parmi TNFα, IL-1β, IL-6, IL-IRa, IL-8, GM-CSF, MIP-1α, MIP-1β, IL-10, IL-1α, TGF-β, TGF-β1, IL-2, IL-4, IL-13, IL-15, IL-3, IL-5, IP10, SCM-1, IFN-α, IFN-β, IFN-ε, IFN-κ, IFN-ω, IFN-γ, IFNL1, IFNL2, IFNL3, ADAR1, IFIT1, IFIT2, IFIT3, IFIT5, IFI27, IFI44L, ISG15, ISG20, MDA5, OAS1, OAS2, OAS3, OASL, PARP12, PKR, RIG-I, RNaseL, ZAP, ZCCHC3, ZNFX1, RBBP6, SHFL, TRIM22, TRIM25, TRIM32, TRIM69, Viperin, TLR3, TLR7, TLR8, TLR9, CD14, LBP, MD1, MD2, CD300LF, GRP94, PRAT4A, PRAT4B, UNC93B1 ATF3, AXL, BAMBI, BCL3, BPI, CENTB1, DAK, FLII, HSP10, IRAKM(IRAK3), LGP2(DHX58), MSK1, MSK2, NLRP12(NALP12), NFKBIA, NFKBIB, NFKBIE, PECAM1, PIN1, PPP3CA, PPP3CB, PPP3R1, PTPN6, RNF125, RP105, SHIP1, SIGIRR, SIKE, TNFAIP3, TNIP1, TNIP2, TNIP3, TOLLIP, TRAFD1, TRIAD3, TYRO3, ZCCHC11, MyD88, RAC1, SARM1, TANK, TIRAP, TRAM(TICAM2), TRIF(TICAM1), AAMP, ACT1(TRAF3IP2), AGER, AKT1, BECN1, BTK, CARD6, CARD11, DDX3X, FADD, IKKα, IKKβ, IKKε, IPS1, IRAK1, IRAK4, IRF1, IRF3, IRF5, IRF7, IRF8, IRF9, ITCH, LRRC59, LRRFIP2, MAP2K2, MAP3K7(TAK1), MAPK1, MDA5(IFIH1), NAIP5, NAP1, NEMO, NIBP, OTUD5, PKR, PRKRA, RIG-1, RIPK1, RIPK2, RIPK3, STAP2, SUGT1, SYK, TAB1, TAB2, TAB3, TBK1, TBKBP1, TIFA, TRADD, TRAF3, TRAF6, TRIL, WDFY1, LGP2(DHX58), MDA5(IFIH1), RIG-I(DDX58), TRAFD1, CYLD, DAK, DDX60, MFN2, MUL1, OTUD5, PIN1, RNF125, SIKE, TNFAIP3, IPS1, IPS1-HA, PARP13, STING, TOMM70A, TRIM13, TRIM26, TRIM32, ZDHHC1, HERC1, HERC2, HERC3, HERC4, HERC5, HERC6 et leurs combinaisons.

Selon un mode de réalisation préféré, les gènes cibles viraux dont l'expression est mesurée à l'étape (a) sont choisis parmi les gènes cibles viraux de la voie de l'interféron et de la voie de la présentation antigénique médiée par le CMH de classe II.

Selon un autre mode de réalisation préféré, l'étape (a) de mesure comprend ou consiste en la mesure d'au moins un gène cible viral choisi parmi IFN-α, IFN-β, IFN-ε, IFN-κ, IFN-ω, IFN-γ, IFNL1, IFNL2, IFNL3, ADAR1, IFIT1, IFIT2, IFIT3, IFIT5, IFI27, IFI44L, ISG15, ISG20, MDA5, OAS1, OAS2, OAS3, OASL, PARP12, PKR, RIG-I, RNaseL, RSAD2, RBBP6, SIGLEC1, SHFL, TRIM22, TRIM25, TRIM32, TRIM69, Viperin, ZAP, ZCCHC3, ZNFX1, HERC1, HERC2, HERC3, HERC4, HERC5, HERC6 et leurs combinaisons, et d'au moins un gène cible bactérien choisi parmi OLAH, FAM20A, IL1R2, MMP8, RETN et SLC1A2.

Selon un autre mode de réalisation préféré, l'étape (a) de mesure comprend ou consiste en la mesure d'au moins un gène cible viral choisi parmi IFI27, IFN-γ, IFIT1, IFIT2, IFI44L, ISG15, OAS1, OAS2, OAS3, RSAD2, TRIM25, SIGLEC1, TRIM22, TRIM32, HERC5, HERC6 et leurs combinaisons, et d'au moins un gène cible bactérien choisi parmi OLAH, FAM20A, IL1R2, MMP8, RETN et SLC1A2.

Selon un autre mode de réalisation particulièrement préféré, l'étape (a) de mesure comprend ou consiste en la mesure d'au moins un gène cible viral choisi parmi SIGLEC1, RSAD2, IFI27, OAS1, IFIT1, IFI44L, ISG15 et HERC6, et d'au moins un gène cible bactérien choisi parmi OLAH, FAM20A, IL1R2, MMP8, RETN et SLC1A2.

Les positions chromosomiques des gènes cibles selon ce mode de réalisation préféré sont notamment données dans le tableau 1 ci-dessous :

[Tableau 1]

| Gène | Localisation chromosomique (GRCh38/hg38) | Nom Anglais |
|---|---|---|
| SIGLEC1 | Chr20: 3,686,970-3,712,600 | Sialic Acid Binding Ig Like Lectin 1 |
| ISG15 | Chr1: 1,001,138-1,014,540 | ISG15 Ubiquitin Like Modifier |
| HERC6 | Chr4: 88378739-88443097 | HECT And RLD Domain Containing E3 Ubiquitin Protein Ligase Family Member 6 |

(suite)

| Gène | Localisation chromosomique (GRCh38/hg38) | Nom Anglais |
|---|---|---|
| SLC1A2 | Chr11: 35251205-35420063 | Solute Carrier Family 1 Member 2 |
| IL1R2 | Chr2: 101991960-102028544 | Interleukin 1 Receptor Type 2 |
| FAM20A | Chr17: 68535113-68601367 | Golgi Associated Secretory Pathway Pseudokinase |
| OLAH | Chr10: 15032227-15073853 | Oleoyl-ACP Hydrolase |
| RETN | Chrl9: 7669049-7670455 | Resistin |
| MMP8 | Chr11: 102711796-102727050 | Matrix Metallopeptidase 8 |
| RSAD2 | Chr2: 6865557-6898239 | Radical S-Adenosyl Methionine Domain Containing 2 |
| IFI27 | Chr14: 94104836-94116698 | Interferon Alpha Inducible Protein 27 |
| OAS1 | Chr12: 112905856-112933219 | 2'-5'-Oligoadenylate Synthetase 1 |
| IFIT1 | Chr10: 89392546-89406487 | Interferon Induced Protein With Tetratricopeptide Repeats 1 |
| IFI44L | Chr1: 78619902-78646145 | Interferon Induced Protein 44 Like |

Le gène SIGLEC1 code pour un membre de la superfamille des immunoglobulines. La protéine codée est une molécule d'adhésion de type lectine qui se lie aux ligands glycoconjugués sur les surfaces cellulaires de manière dépendante de l'acide sialique. Il s'agit d'une protéine transmembranaire de type I exprimée uniquement par une sous-population de macrophages et qui est impliquée dans la médiation des interactions cellule-cellule.

Le gène ISG15 code pour une protéine de type ubiquitine qui est conjuguée à des protéines cibles intracellulaires lors de l'activation par l'interféron-alpha et l'interféron-bêta. Plusieurs fonctions ont été attribuées à la protéine codée, notamment l'activité chimiotactique envers les neutrophiles, la direction des protéines cibles conjuguées aux filaments intermédiaires, la signalisation intercellulaire et l'activité antivirale pendant les infections virales.

Le gène HERC6 appartient à la famille HERC des ubiquitine ligases, qui contiennent toutes a minima un domaine HECT de 350 acides aminés catalysant la formation d'un thioester avec l'ubiquitine avant de la transférer à un substrat.

Le gène SLC1A2 code pour un membre d'une famille de protéines transporteuses de solutés. Cette protéine liée à la membrane est le principal transporteur qui élimine le glutamate, un neurotransmetteur excitateur, de l'espace extracellulaire au niveau des synapses du système nerveux central. La clairance du glutamate est nécessaire pour une activation synaptique correcte et pour prévenir les dommages neuronaux dus à une activation excessive des récepteurs du glutamate.

Le gène IL1R2 code pour une protéine récepteur de cytokine qui appartient à la famille des récepteurs de l'interleukine 1. Cette protéine lie l'interleukine alpha (IL1(A), l'interleukine bêta (IL1B) et le récepteur de l'interleukine 1 de type I (IL1R1/IL1R(A), et agit comme un récepteur leurre qui inhibe l'activité de ses ligands. L'interleukine 4 (IL4) antagoniserait l'activité de l'interleukine 1 en induisant l'expression et la libération de cette cytokine. Ce gène et trois autres gènes forment un groupe de gènes de récepteurs de cytokines sur le chromosome 2q12. L'épissage alternatif donne lieu à de multiples variantes de transcription et isoformes de protéines membranaires et solubles.

Le gène FAM20A code pour une protéine vraisemblablement sécrétée qui pourrait jouer un rôle dans l'hématopoïèse. Une mutation à ce locus a été associée à l'amélogénèse imparfaite et au syndrome d'hyperplasie gingivale.

Le gène OLAH permet l'activité hydrolase du dodécanoyl-[porteuse d'acyle-protéine] ; l'activité hydrolase du myristoyl-[porteuse d'acyle-protéine] ; et l'activité hydrolase du palmitoyl-[porteuse d'acyle-protéine]. Il intervient dans le processus de biosynthèse des acides gras à chaîne moyenne.

Le gène RETN code pour une protéine ayant un rôle antimicrobien au niveau de la peau via une activité antibactérienne contre les bactéries à Gram positif et à Gram négatif.

Le gène MMP8 code pour un membre de la famille des protéines de la matrice métalloprotéinase (MMP). Ces protéines sont impliquées dans la dégradation de la matrice extracellulaire au cours du développement embryonnaire, de la reproduction et du remodelage des tissus, ainsi que dans les processus pathologiques, tels que l'arthrite et les métastases. La protéolyse à différents sites sur cette protéine donne lieu à plusieurs formes actives de l'enzyme avec des extrémités N-terminales distinctes. Cette protéine fonctionne dans la dégradation des collagènes de type I, II et III.

Le gène RASAD2 code pour une protéine antivirale inductible par l'interféron qui appartient à la superfamille des enzymes S-adénosyl-L-méthionine (SAM) qui joue un rôle dans la réponse antivirale cellulaire et la signalisation immunitaire innée. Les effets antiviraux résultent notamment de l'inhibition de la réplication de l'ARN viral, de l'interférence dans la voie de sécrétion, de la liaison aux protéines virales et de la dérégulation du métabolisme des lipides cellulaires.

Le gène IFI27 est notamment impliqué dans le processus métabolique des protéines cellulaires, la réponse de défense à d'autres organismes et la voie de signalisation apoptotique extrinsèque. Ce gène agit également en amont ou dans la régulation négative de la transcription par l'ARN polymérase II et la régulation de l'exportation des protéines du noyau.

Le gène OAS1 code pour une protéine synthétisant les 2',5'-oligoadénylates (2-5As) et jouant un rôle clé dans la réponse antivirale cellulaire innée.

Le gène IFIT1 code une protéine contenant des répétitions tetratricopeptidiques identifiée à l'origine comme étant induite par un traitement à l'interféron. La protéine codée peut inhiber la réplication virale et l'initiation de la traduction

Le gène IFI44L est associé à l'activité de liaison au GTP et impliqué dans la réponse de défense aux virus.

Selon la présente description, l'identification ou la mesure du niveau d'expression génique consiste à mettre en évidence une variation de l'expression au niveau transcriptomique desdits gènes, ladite variation étant mise en évidence par rapport à une expression de référence desdits gènes. Pour cette raison, on parlera notamment de signature transcriptomique.

Les transcrits des différents gènes cibles utiles pour la mise en œuvre de la méthode selon la présente description sont également connus de la personne du métier et leurs séquences sont mises à disposition dans les bases de données NCBI ou Ensembl.

Des exemples de transcris sont présentés dans le tableau 2 ci-après pour certains gènes cibles viraux et bactériens préférés :

[Tableau 2]

| Gène | Référence transcrit (base de données NCBI) |
|---|---|
| SIGLEC1 | NM_023068.4; NM_001367089.1 |
| ISG15 | NM_005101.4 |
| HERC6 | NM_017912.4 <br> NM_001165136.2 |
| SLC1A2 | NM_004171.4; NM_001195728.3; NM_001252652.2 |
| IL1R2 | NM_004633.4; NM_001261419.2 |
| FAM20A | NM_017565.4; NM_001243746.2 |
| OLAH | NM_001039702.3; NM_018324.3 |
| RETN | NM_020415.4; NM_001385725.1; NM_001385726.1; NM_001385727.1; NM_001193374.2 |
| MMP8 | NM_002424.3; NM_001304442.2; NM_001304441.2 |
| RSAD2 | NM_080657.5; NM_001410702.1 |
| IFI27 | NM_001366994.1; NM_001366993.1; NM_001288952.2; NM_001130080.3; NM_001288956.2; NM_001288959.2 |
| OAS1 | NM_0013201512; NM_016816.4; NM_001032409.3; NM_002534.4; NM_001406027.1; NM_001406030.1; NM_001406025.1; NM_001406024.1; NM_001406029.1; NM_001406020.1; NM_001406026.1; NM_001406023.1; NM_001406021.1; NM_001406022.1 |
| IFIT1 | NM_001270930.2; NM_001270927.2; NM_001270928.2; NM_001270929.2; NM_001548.5 |
| IFI44L | NM_001375646.1; NM_001375650.1; NM_006820.4; NM_001375647.1; NM_001375649.1; NM_001375648.1 |

Selon un mode de réalisation particulier, la mesure de l'expression des gènes cibles viraux et bactériens est réalisée au niveau des transcrits ARNm, et de préférence au niveau des transcrits choisis parmi les transcrits du tableau 2.

Selon un mode de réalisation préféré, la mesure de l'expression des gènes cibles est réalisée au niveau des transcrits ARNm, et de préférence au niveau des transcrits choisis parmi les transcrits (base de données NCBI) NM_023068.4, NM_001367089.1, NM_005101.4, NM_017912.4, NM_001165136.2, NM_017565.4, NM_001243746.2, NM_002424.3, NM_001304442.2, NM_001304441.2, NM_001039702.3, NM_018324.3, NM_004633.4, NM_001261419.2, NM_003256.4, NM_004171.4, NM_001195728.3, NM_001252652.2, NM_020415.4, NM_001385725.1, NM_001385726.1, NM_001385727.1, NM_001193374.2 et leurs combinaisons.

N'importe quelle méthode connue de la personne du métier permettant de mesurer le niveau d'expression génique, ou une variation d'expression génique, au niveau transcriptionnel peut être utilisée dans le cadre de la méthode selon l'invention.

Ainsi, la mesure peut être réalisée via une méthode directe permettant de déterminer la présence dudit transcrit dans l'échantillon biologique, ou par détection indirecte du transcrit après transformation de ce dernier en ADN.

Les techniques couramment utilisées pour mesurer simultanément la concentration d'un grand nombre de types différents d'ARN messagers sont connues de la personne du métier et il n'est pas nécessaire d'en donner le détail. A titre d'exemple, on citera notamment les puces à ADN, le CAGE, le SAGE et, plus récemment, le séquençage d'ARN à haut débit dit RNA-Seq.

Selon la présente description, l'expression des gènes au niveau transcriptionnel peut être mesurée par toute méthode connue de détection moléculaire. Ainsi, la variation de l'expression génique peut être mesurée par amplification via notamment une Reverse Transcription-Polymerase Chain Reaction ou RT-PCR, par séquençage (de préférence par séquençage haut débit) ou par des techniques d'hybridation (par exemple avec des micropuces d'hybridation ou par des techniques du type NanoString® nCounter®). Toutes ces méthodes sont également bien connues de la personne du métier et il n'est pas nécessaire de les détailler ici.

Selon un mode de réalisation particulier, la détermination de l'expression des gènes peut être réalisée de la manière suivante :

(1) extraction des ARN totaux d'un échantillon sanguin ou des PBMC et réalisation d'une étape de transcription inverse afin d'obtenir les différents ADN complémentaires des différents ARN messagers initialement présents dans l'échantillon ou les PBMC (ou ADNc),

(2) amplification spécifique des ADNc. Dans ce cas, le réactif spécifique utilisé comprend au moins une amorce d'amplification spécifique du gène. Cette étape peut être réalisée par une réaction d'amplification de type PCR ou par tout autre technique d'amplification appropriée,

(3) détermination de l'expression du gène en quantifiant les ADNc.

Selon un mode de réalisation préféré, la mesure de l'expression génique est réalisée par RT-PCR, de préférence RT-PCR quantitative ou semi-quantitative, ou par PCR nichée également appelée « Nested » PCR, par exemple en utilisant la technologie FilmArray® (Poritz et al. 2011) ou la plateforme Biomark™ de Fluidigm. Selon ce mode de réalisation, l'expression est mesurée au niveau des transcrits ARNm des gènes cibles.

La personne du métier est tout en fait en mesure de déterminer les séquences des amorces ou couples d'amorces nécessaires à l'amplification des transcrits des gènes cibles, et éventuellement des gènes cibles additionnels définis plus loin dans la description, pour déterminer leurs niveaux d'expressions respectifs. En effet, de nombreux outils sont disponibles, par exemple Geneious ou Primer 3, lesdites séquences pouvant ensuite être ajustées si besoin par la personne du métier.

La mesure du niveau d'expression permet de déterminer la quantité des transcrits dans l'échantillon biologique ou également d'en donner une valeur dérivée.

Selon un mode de réalisation particulier, le niveau d'expression des gènes cibles de la signature est une valeur dérivée ou normalisée de la quantité des transcrits, notamment ARNm, desdits gènes cibles.

Selon un mode de réalisation particulier, l'expression génique est normalisée par rapport à l'expression d'un ou plusieurs gènes de ménage (ou gènes de référence) selon les méthodes connues de la personne du métier. Ainsi, l'expression est normalisée en utilisant un ou plusieurs des gènes de ménage suivants : DECR1 (localisation chromosomique : chr8, 90001352-90053633), HPRT1 (localisation chromosomique: chrX, 134452842-134520513) et PPIB (localisation chromosomique: chr15:64155812-64163205), RPLP0 (localisation chromosomique: chr12, 120196699-120201111), PPIA (localisation chromosomique: chr7, 44795960-44803117), GLYR1 (localisation chromosomique: chr16, 4803203-4847288), RANBP3 (localisation chromosomique : chr19 , 5916139-5978140), B2M (localisation chromosomique : chr15, 44711492-44718145), TBP (localisation chromosomique : chr6, 170554369-170572859), GAPDH (localisation chromosomique : chr12, 6534517-6538371) et ACTB (localisation chromosomique: chr14, 5527148-5530601). Les localisations chromosomiques sont données selon le GRCh38/hg38. De préférence, l'expression est normalisée en utilisant un ou plusieurs gènes de ménage choisis parmi : DECR1, HPRT1, PPIB, GAPDH, ACTB et leurs combinaisons, de préférence encore, choisis parmi DECR1, HPRT1, PPIB et leurs combinaisons.

Dans un tel cas, le niveau de référence utilisé est également normalisé au préalable, de la même manière. La normalisation, que ce soit pour le niveau de référence ou pour le niveau de transcrits de l'échantillon biologique à tester, est réalisée avant la comparaison, notamment avant le calcul d'un rapport entre le niveau de transcrits dudit échantillon à tester et le niveau de référence. Lorsqu'une valeur seuil différente d'un niveau de référence est utilisée pour émettre une conclusion, il pourra être tenu compte de cette normalisation, pour le choix de la valeur seuil.

Dans le cas où le niveau de(s) transcrits des gènes est normalisé par rapport au niveau de transcrits d'un ou plusieurs gènes de ménage, bien entendu, cela implique que la présente méthode inclut la détermination du niveau de transcrits du ou des gènes de ménage utilisé(s) pour la normalisation.

D'une manière générale, selon la méthode de la présente invention, et quels que soient ses modes de réalisations, le niveau d'expression d'un gène cible (de préférence l'expression normalisée) dans l'échantillon biologique du sujet est comparé à une valeur d'expression prédéterminée de ce même gène (de préférence l'expression normalisée) dans un échantillon biologique de référence. Cette comparaison permet d'obtenir la variation de l'expression dudit gène cible mesurée selon la présente méthode.

Pour un gène cible donné, la valeur d'expression de référence correspond à un niveau d'expression de transcrits dudit gène obtenu à partir d'un échantillon biologique de référence issu d'un sujet ayant une infection de nature déterminée. L'échantillon biologique de référence est de même nature que l'échantillon biologique à tester ou tout du moins d'une nature compatible pour constituer une référence quant à la détermination du niveau d'expression des gènes cibles Avantageusement, et notamment pour les modes de réalisation ci-après, la valeur de référence pour un gène donné correspond à la moyenne du niveau de transcrits ARNm dudit gène obtenue à partir d'échantillons biologiques de références issus d'une population de sujets présentant une infection.

Au sens de la présente description, l'expression « valeur de référence » ou « valeur de référence prédéterminée », est synonyme des expressions « valeur contrôle » ou « valeur seuil », et sert de point de comparaison pour déterminer si le niveau d'expression d'un gène cible est diminué ou augmenté.

Selon un mode de réalisation particulier, la valeur de référence des gènes cibles correspond au niveau d'expression des transcrits ARNm desdits gènes obtenus à partir d'un échantillon biologique de référence issu d'un sujet ayant une infection bactérienne.

Selon un mode de réalisation particulier, la valeur de référence des gènes cibles correspond au niveau d'expression des transcrits ARNm desdits gènes obtenus à partir d'un échantillon biologique de référence issu d'un sujet ayant une infection virale.

La comparaison peut être effectuée par toutes méthodes connues de la personne du métier et peut par exemple impliquer le calcul d'un rapport ou d'une différence. Avantageusement, dans le cadre de la présente méthode, la(les) comparaison(s) et l'émission d'une conclusion quant à la nature de l'infection chez le sujet dont est issu l'échantillon biologique, est effectuée par une technique automatisée, réalisée par un ordinateur ou assistée par ordinateur.

Ainsi, la nature virale ou bactérienne de l'infection du sujet est déterminée lorsque les comparaisons des niveaux d'expression des gènes cibles avec leurs valeurs de références respectives permet d'identifier une différence statistiquement significative, en d'autres termes, une variation dudit niveau d'expression. Par conséquent, on parlera de surexpression lorsqu'une expression significativement augmentée est mise en évidence, et à l'inverse de sous-expression, lorsqu'une expression significativement diminuée est mise en évidence.

La personne du métier est en mesure de déterminer le test statistique à utiliser pour déterminer cette valeur de référence avec laquelle le niveau d'expression des gènes cibles doit être comparé. Les exemples de réalisation présentent une des méthodes possibles.

Selon une première variante de réalisation de l'invention, la valeur d'expression de référence de chaque gène cible est déterminée à partir d'un échantillon biologique de référence issu d'un pool d'échantillons biologiques de sujets atteints d'une infection bactérienne. De préférence, dans la méthode selon l'invention, la valeur d'expression de référence des gènes cibles correspond à l'expression respectives desdits gènes cibles dans un échantillon biologique de référence obtenu chez un sujet ayant une infection bactérienne. Ainsi, il pourra être conclu à une infection de nature virale lorsque la comparaison du niveau d'expression des gènes cibles au niveau des transcrits ARNm par rapport aux valeurs de référence respectives, met en évidence au moins une variation d'expression choisie parmi:

- une surexpression de SIGLEC1,

- une surexpression de ISG15,

- une surexpression de HERC6,

- une surexpression de RSAD2,

- une surexpression de IFI27,

- une surexpression de OAS1,

- une surexpression de IFIT1, et

- une surexpression de IFI44L,

et au moins une autre variation d'expression choisie parmi :

- une sous expression de SLC1A2,

- une sous expression de IL1R2,

- une sous expression de FAM20A,

- une sous expression de OLAH,

- une sous expression de RETN, et

- une sous expression de MMP8.

Selon une deuxième variante de réalisation de l'invention, la valeur de référence de chaque gène cible est déterminée à partir d'un échantillon biologique de référence issu d'un pool d'échantillons biologiques de sujets atteints d'une infection virale. Ainsi, il peut être conclu à une infection de nature bactérienne lorsque les résultats de comparaison d'expression des gènes cibles met en évidence au moins deux variations choisies parmi les variations suivantes :

- une sous expression de SIGLEC1,

- une sous expression de ISG15,

- une sous expression de HERC6,

- une sous expression de RSAD2,

- une sous expression de IFI27,

- une sous expression de OAS1,

- une sous expression de IFIT1, et

- une sous expression de IFI44L,

et au moins une autre variation d'expression choisie parmi :

- une surexpression de SLC1A2,

- une surexpression de IL1R2,

- une surexpression de FAM20A,

- une surexpression de OLAH,

- une surexpression de RETN, et

- une surexpression de MMP8.

La personne du métier est en mesure d'opter pour l'une ou l'autre des variantes en fonction des échantillons biologiques de référence à disposition. L'échantillon biologique de référence est avantageusement être un pool d'échantillons biologiques de sujets ayant une infection bactérienne ou un pool d'échantillons biologiques de sujets ayant une infection virale.

Pour tous les modes de réalisation particuliers ci-après, la conclusion quant à la nature de l'infection est réalisée en tenant compte des surexpression/sous expression des différents gènes cibles mises en évidence à partir de l'échantillon biologique du sujet, et telles que définies précédemment dans l'une ou l'autre des deux variantes de réalisation.

Selon un mode de réalisation particulier, l'étape (a) comprend ou consiste en la mesure de l'expression d'au moins un gène cible viral choisi parmi SIGLEC1, ISG15, HERC6, RSAD2, IFI27, OAS1, IFIT1 et IFI44L, et d'au moins un gène cible bactérien choisi parmi OLAH, FAM20A, IL1R2, MMP8, RETN et SLC1A2.

Selon un autre mode de réalisation particulier, l'étape (a) comprend ou consiste en la mesure de l'expression d'un seul gène cible viral choisi parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2, et de préférence, de deux ou trois gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression de deux gènes cibles viraux choisis parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2, et de préférence, de deux ou trois gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression de trois gènes cibles viraux choisis parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression de quatre gènes cibles viraux choisis parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression de cinq gènes cibles viraux choisis parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression de six gènes cibles viraux choisis parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression de sept gènes cibles viraux choisis parmi SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des huit gènes cibles viraux SIGLEC1, ISG15, HERC6, RSAD2, IFI27, OAS1, IFIT1 et IFI44L, et de deux, trois, quatre, cinq ou six gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression du gène cible viral SIGLEC1 et d'un ou plusieurs gènes cibles bactériens choisi parmi OLAH, FAM20A, IL1R2, MMP8, RETN et SLC1A2, de préférence parmi SLC1A2, IL1R2, OLAH, FAM20A et RETN, et de préférence encore parmi SLC1A2, IL1R2, OLAH et FAM20A.

Selon une variante préférée de ce mode de réalisation, l'étape (a) comprend ou consiste en la mesure de l'expression du gène cible viral SIGLEC1 et d'au moins trois gènes cibles bactériens choisi SLC1A2, IL1R2, OLAH, FAM20A et RETN.

Selon un autre mode de réalisation particulier, l'étape (a) comprend ou consiste en la mesure de l'expression du gène cible viral ISG15 et d'un ou plusieurs gènes cibles bactériens choisi parmi, OLAH, FAM20A, IL1R2, MMP8, RETN et SLC1A2, de préférence parmi SLC1A2, IL1R2, OLAH, FAM20A et RETN, et de préférence encore parmi SLC1A2, IL1R2, OLAH et FAM20A.

Selon un autre mode de réalisation particulier, l'étape de mesure comprend ou consiste en la mesure de l'expression du gène viral HERC6 et d'un ou plusieurs gènes cibles bactériens choisi parmi OLAH, FAM20A, IL1R2, MMP8, RETN et SLC1A2, de préférence parmi SLC1A2, IL1R2, OLAH, FAM20A et RETN, et de préférence encore parmi SLC1A2, IL1R2, OLAH et FAM20A.

Selon un autre mode de réalisation particulier, l'étape (a) comprend ou consiste en la mesure de l'expression du gène cible viral IFIT1 et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression du gène cible viral IFI44L et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression du gène cible viral RSAD2 et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression du gène cible viral OAS1 et d'un ou plusieurs gènes cibles bactériens choisis parmi OLAH, FAM20A, IL1R2, MMP8, RETN et SLC1A2.

Selon un mode de réalisation préféré, l'étape (a) comprend ou consiste en la mesure de l'expression du gène cible viral IFI27 et d'un ou plusieurs gènes cibles bactériens choisis parmi OLAH, FAM20A, IL1R2, MMP8, RETN et SLC1A2, et de préférence parmi IL1R2, OLAH, FAM20 et RETN.

Selon un autre mode de réalisation préféré, l'étape (a) comprend ou consiste en la mesure de l'expression du gène cible viral IFI27, et éventuellement d'au moins un autre gène viral choisi parmi SIGLEC1, ISG15, HERC6, RSAD2, OAS1, IFIT1, IFI44L, et de deux gènes cibles bactériens choisis parmi OLAH, FAM20A, IL1R2, MMP8, RETN et SLC1A2, et de préférence choisis parmi OLAH, FAM20A, IL1R2 et MMP8.

Selon un autre mode de réalisation préféré, l'étape (a) comprend ou consiste en la mesure de l'expression du gène cible viral IFI27, du gène cible bactérien OLAH et d'au moins un autre gène cible bactérien choisi parmi SLC1A2, IL1R2, FAM20A, RETN et MMP8.

Selon un autre mode de réalisation particulier, l'étape (a) comprend ou consiste en la mesure de l'expression d'une combinaison de deux gènes cibles choisie parmi les combinaisons du tableau 6, et de préférence celles qui présentent une performance en termes d'aire sous la courbe ROC du modèle d'au moins 0,86, et tout particulièrement, d'au moins 0,88.

Selon une variante préférée de ce mode de réalisation particulier, l'étape (a) comprend ou consiste en la mesure de l'expression d'une combinaison d'un gène cible viral et d'un gène cible bactérien, ladite combinaison étant choisie parmi les combinaisons suivantes : SIGLEC1_IL1R2, IFI44L_OLAH et IFI27_1L1R2.

Selon un autre mode de réalisation, l'étape (a) comprend ou consiste en la mesure de l'expression d'une combinaison de deux gènes cibles choisie parmi les combinaisons suivantes : SIGLEC1_IL1R2 ; SIGLEC1_FAM20A ; HERC6_IL1R2 ; SIGLEC1_OLAH ; HERC6_OLAH ; ISG15_IL1R2 ; SIGLEC1_RETN ; HERC6_FAM20A ; SLC1A2_OLAH ; HERC6_RETN ; SIGLEC1_SLC1A2 ; ISG15_OLAH ; SIGLEC1_MMP8 ; HERC6MMP8, IL1R2_OLAH ; IL1R2FAM20A ; SIGLEC1_HERC6 ; HERC6_SLC1A2 ; SIGLEC1_ISG15 ; FAM20A_OLAH ; ISG15FAM20A ; SLC1A2_IL1R2 ; ISG15RETN ; ISG15_MMP8 et ISG15_SLC1A2, et de préférence choisie parmi les combinaisons de gènes suivantes : SIGLEC1_IL1R2 ; SIGLEC1_FAM20A ; HERC6_IL1R2 ; SIGLEC1_OLAH et HERC6_OLAH.

Selon un autre mode de réalisation particulier, l'étape (a) comprend ou consiste en la mesure d'au moins un gène cible viral choisi parmi IFI27, SIGLEC1, ISG15, HERC6, RSAD2, OAS1, IFIT1 et IFI44L, du gène cible bactérien OLAH, et d'au moins un autre gène cible bactérien choisi parmi FAM20A, IL1R2, MMP8, RETN et SLC1A2, de préférence parmi FAM20A, IL1R2 et MMP8.

Selon un autre mode de réalisation particulier, l'étape (a) l'étape (a) comprend ou consiste en la mesure d'au moins un gène cible viral choisi parmi IFI27, SIGLEC1, ISG15, HERC6, RSAD2, OAS1, IFIT1 et IFI44L, du gène cible bactérien FAM20A, et d'au moins un autre gène cible bactérien choisi parmi OLAH, IL1R2, MMP8, RETN et SLC1A2, de préférence parmi OLAH, IL1R2 et MMP8.

Selon un autre mode de réalisation particulier, l'étape (a) comprend ou consiste en la mesure de l'expression des gènes cibles viraux SIGLEC1 et ISG15, et d'un ou plusieurs gènes cibles bactériens choisis parmi OLAH, FAM20A, IL1R2, MMP8, RETN et SLC1A2.

Selon un mode de réalisation particulier, l'étape (a) comprend ou consiste en la mesure de l'expression des gènes cibles viraux SIGLEC1 et HERC6, et d'un ou plusieurs gènes cibles bactériens choisis parmi OLAH, FAM20A, IL1R2, MMP8, RETN et SLC1A2.

Selon un mode de réalisation particulier, l'étape (a) comprend ou consiste en la mesure de l'expression des gènes cibles viraux SIGLEC1 et RSAD2, et d'un ou plusieurs gènes cibles bactériens choisis parmi OLAH, FAM20A, IL1R2, MMP8, RETN et SLC1A2.

Selon un mode de réalisation préféré, l'étape (a) comprend ou consiste en la mesure de l'expression des gènes cibles viraux SIGLEC1 et IFI27, et d'un ou plusieurs gènes cibles bactériens choisis parmi OLAH, FAM20A, IL1R2, MMP8, RETN et SLC1A2.

Selon une variante de ce mode de réalisation préféré, l'étape (a) comprend ou consiste en la mesure de l'expression des gènes cibles viraux SIGLEC1 et IFI27, et de deux à quatre gènes cibles bactériens choisis parmi OLAH, FAM20A, IL1R2 et MMP8.

Selon un autre mode de réalisation particulier, l'étape (a) comprend ou consiste en la mesure de l'expression des gènes cibles viraux SIGLEC1 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1 et IFIT1, et d'un ou plusieurs gènes cibles bactériens choisis parmi OLAH, FAM20A, IL1R2, MMP8, RETN et SLC1A2, et de préférence, choisis parmi OLAH et FAM20A.

Selon un autre mode de réalisation particulier, l'étape (a) comprend ou consiste en la mesure de l'expression des gènes cibles viraux SIGLEC1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15 et HERC6, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15 et RSAD2, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15 et IFI27, et d'un ou plusieurs gènes cibles bactériens choisis parmi OLAH, FAM20A, IL1R2, MMP8, RETN et SLC1A2, et de préférence, choisis parmi OLAH et FAM20A.

Selon un autre mode de réalisation particulier, l'étape (a) comprend ou consiste en la mesure de l'expression des gènes cibles viraux ISG15 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6 et RSAD2, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6 et IFI27, et d'un ou plusieurs gènes cibles bactériens choisis parmi OLAH, FAM20A, IL1R2, MMP8, RETN et SLC1A2, et de préférence choisis, parmi OLAH et FAM20A.

Selon un autre mode de réalisation particulier, l'étape (a) comprend ou consiste en la mesure de l'expression des gènes cibles viraux HERC6 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2 et OAS1, et d'un ou plusieurs gènes cibles bactériens choisis parmi OLAH, FAM20A, IL1R2, MMP8, RETN et SLC1A2.

Selon un mode de réalisation particulier, l'étape (a) comprend ou consiste en la mesure de l'expression des gènes cibles viraux RSAD2 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI27 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI27 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI27 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux OAS1 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux OAS1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI44L et IFIT1, et d'un ou plusieurs gènes cibles bactériens choisis parmi OLAH, FAM20A, IL1R2, MMP8, RETN et SLC1A2.

Selon un autre mode de réalisation particulier, l'étape de mesure comprend ou consiste en la mesure d'au moins un gène cible viral choisi parmi SIGLEC1, ISG15 et HERC6, et d'au moins un gène cible bactérien choisi parmi OLAH, FAM20A, IL1R2, MMP8, RETN et SLC1A2. De préférence, selon ce mode de réalisation, l'étape de mesure comprend ou consiste en la mesure d'au moins un gène cible viral choisi parmi SIGLEC1, ISG15 et HERC6, et d'au moins un gène cible choisi parmi IL1R2, OLAH, et FAM20A.

Selon un autre mode de réalisation particulier, l'étape de mesure comprend ou consiste en la mesure d'au moins un gène cible viral choisi parmi RSAD2, IFI27, OAS1, IFIT1 et IFI44L, et d'au moins un gène cible bactérien choisi parmi OLAH, FAM20A, IL1R2, MMP8, RETN et SLC1A2. De préférence, selon ce mode de réalisation, l'étape de mesure comprend ou consiste en la mesure d'au moins un gène cible viral choisi parmi RSAD2, IFI27, OAS1, IFIT1 et IFI44L, et d'au moins un gène cible bactérien choisi parmi IL1R2, OLAH, et FAM20A.

Selon un autre mode de réalisation préféré, l'étape de mesure comprend ou consiste en la mesure d'au moins un gène cible viral choisi parmi SIGLEC1, ISG15 et HERC6, et d'au moins deux gènes cibles bactériens choisis parmi SLC1A2, IL1R2, OLAH, FAM20A et RETN et MMP8, et de préférence IL1R2, OLAH et FAM20A.

Selon une variante de ce mode de réalisation particulier, l'étape (a) comprend ou consiste en la mesure de l'expression du gène cible viral SIGLEC1 et d'au moins deux gènes cibles bactériens choisis parmi le groupe constitué de OLAH, SLC1A2, IL1R2, FAM20A, RETN et MMP8.

Selon une autre variante de ce mode de réalisation particulier, l'étape (a) comprend ou consiste en la mesure de l'expression du gène cible viral HERC6 et d'au moins deux gènes cibles bactériens choisis parmi le groupe constitué de OLAH, SLC1A2, IL1R2, FAM20A, RETN et MMP8.

Selon une autre variante de ce mode de réalisation particulier, l'étape (a) comprend ou consiste en la mesure de l'expression du gène cible viral ISG15 et d'au moins deux gènes cibles bactériens choisis parmi le groupe constitué de OLAH, SLC1A2, IL1R2, FAM20A, RETN et MMP8.

Selon une autre variante de ce mode de réalisation particulier, l'étape (a) comprend ou consiste en la mesure de l'expression du gène cible viral SIGLEC1 et du gène cible bactérien OLAH, et d'au moins un autre gène cible choisi parmi le groupe constitué de ISG15, HERC6, SLC1A2, IL1R2, FAM20A, RETN et MMP8.

Selon une autre variante de ce mode de réalisation particulier, l'étape (a) comprend ou consiste en la mesure de l'expression du gène cible viral SIGLEC1 et du gène cible bactérien IL1R2, et d'au moins un autre gène cible choisi parmi ISG15, HERC6, SLC1A2, OLAH, FAM20A, RETN et MMP8, et de préférence choisi parmi SLC1A2, OLAH, FAM20A, RETN et MMP8.

Selon un autre mode de réalisation particulier, l'étape (a) comprend ou consiste en la mesure de l'expression des gènes cibles viraux SIGLEC1, ISG15 et HERC6, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, ISG15 et RSAD2, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, ISG15 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, ISG15 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, ISG15 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, ISG15 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, HERC6 et RSAD2, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, HERC6 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, HERC6 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, HERC6 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, HERC6 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, RSAD2 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, RSAD2 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, RSAD2 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, RSAD2 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, IFI27 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, IFI27 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, IFI27 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, IFIT1 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, OAS1 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux SIGLEC1, IFI27 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, HERC6 et RSAD2, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, HERC6 et IFI27, et d'un ou plusieurs gènes cibles bactériens choisis parmi OLAH, FAM20A, ILIR2, MMP8, RETN et SLC1A2.

Selon un autre mode de réalisation particulier, l'étape (a) comprend ou consiste en la mesure de l'expression des gènes cibles viraux ISG15, HERC6 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, HERC6 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, HERC6 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, RSAD2 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, RSAD2 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, RSAD2 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, RSAD2 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, IFI27 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, IFI27 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, IFI27 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, OAS1 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, OAS1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux ISG15, IFIT1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, RSAD2 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, RSAD2 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, RSAD2 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, RSAD2 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, IFI27 et OAS1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, IFI27 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, IFI27 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, OAS1 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, OAS1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux HERC6, IFI44L et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2, OAS1 et IFI27, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2, IFI27 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2, IFI27 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2, OAS1 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2, OAS1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux RSAD2, IFIT1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI27, OAS1 et IFIT1, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI27, OAS1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux IFI27, IFIT1 et IFI44L et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression des gènes cibles viraux OAS1, IFIT1 et IFI44L, et d'un ou plusieurs gènes cibles bactériens choisis parmi OLAH, FAM20A, IL1R2, MMP8, RETN et SLC1A2.

Selon un autre mode de réalisation particulier, l'étape (a) comprend ou consiste en la mesure de l'expression d'une combinaison de trois gènes cibles choisie parmi celles listées dans le tableau 7, et de préférence celles qui présentent une performance en termes d'aire sous la courbe ROC du modèle d'au moins 0,86, d'au moins 0,88, et tout particulièrement, d'au moins 0,90.

Selon un autre variante de ce mode de réalisation particulier, l'étape de mesure comprend ou consiste en la mesure de l'expression d'une combinaison de trois gènes cibles choisie parmi les combinaisons suivantes : SIGLEC1_IL1R2_FAM20A, HERC6_IL1R2_FAM20A, SIGLEC1_SLC1A2_OLAH, SIGLEC1_HERC6_IL1R2, SIGLEC1_FAM20A_OLAH, SIGLEC1_IL1R2_MMP8, SIGLEC1_ISG15_IL1R2, SIGLEC1_SLC1A2_IL1R2, SIGLEC1_IL1R2_OLAH, SIGLEC1_SLC1A2_FAM20A, HERC6_FAM20A_OLAH, SIGLEC1_FAM20A_RETN, SIGLEC1_HERC6 FAM20A, SIGLEC1_FAM20A _MMP8, SIGLEC1_IL1R2_RETN, ISG15_IL1R2_FAM20A, SIGLEC1_ISG15_FAM20A, ISG15_HERC6_IL1R2, HERC6_IL1R2_OLAH, SIGLEC1_HERC6_OLAH, HERC6_SLC1A2_OLAH, HERC6_IL1R2_RETN, HERC6_IL1R2_MMP8, SIGLEC1_OLAH_MMP8, SIGLEC1_ISG15_OLAH, SIGLEC1_OLAH_RETN, HERC6_SLC1A2_IL1R2, ISG15_SLC1A2_OLAH, HERC6_FAM20A_RETN, HERC6_OLAH _RETN, ISG15_SLC1A2_IL1R2, HERC6_OLAH_MMP8, SIGLEC1_HERC6_RETN, ISG15_IL1R2_OLAH, ISG15_HERC6_OLAH, SIGLEC1_SLC1A2_RETN, ISG15_IL1R2_RETN, ISG15_FAM20A_OLAH, ISG15_IL1R2_MMP8, SIGLEC1_SLC1A2_MMP8, ISG15_HERC6_RETN, SIGLEC1_ISG15_RETN, HERC6_SLC1A2FAM20A, ISG15_HERC6_FAM20A, HERC6_FAM20A_MMP8, SLC1A2_FAM20A_OLAH, SIGLEC1_RETN_MMP8, HERC6_SLC1A2 RETN, SIGLEC1_HERC6_SLC1A2, SLC1A2_IL1R2_OLAH, SIGLEC1_HERC6_MMP8, ISG15_OLAH_RETN, SIGLEC1_ISG15_SLC1A2, ISG15_OLAH_MMP8, SLC1A2_OLAH_MMP8, SLC1A2_OLAH_RETN, HERC6_RETN_MMP8, HERC6_SLC1A2_MMP8, SIGLEC1_ISG15_MMP8, IL1R2_FAM20A_OLAH, ISG15_HERC6_MMP8, ISG15_SLC1A2_FAM20A, ISG15_FAM20A_RETN, IL1R2_OLAH_MMP8, SLC1A2_IL1R2_FAM20A, IL1R2_OLAH_RETN, SIGLEC1_ISG15_HERC6, FAM20A_OLAH_MMP8, ISG15_SLC1A2_RETN, IL1R2_FAM20A_MMP8, ISG15_HERC6_SLC1A2, ISG15_SLC1A2_MMP8, ISG15_FAM20A_MMP8, IL1R2_FAM20A_RETN, SLC1A2_IL1R2_RETN, FAM20A_OLAH_RETN, OLAH_RETN_MMP8, SLC1A2IL1R2_MMP8, ISG15_RETN_MMP8, IL1R2_RETN_MMP8, SLC1A2_FAM20A_RETN, SLC1A2_FAM20_MMP8, SLC1A2_RETN_MMP8, et FAM20A_RETN_MMP8. De préférence, selon cette variante, l'étape de mesure comprend ou consiste en la mesure de l'expression d'une combinaison de trois gènes cibles choisis parmi les combinaisons suivantes : S1GLEC1_IL1R2_FAM20A, HERC6_IL1R2_FAM20A, SIGLEC1_SLC1A2_OLAH, SIGLEC1_HERC6_IL1R2, SIGLEC1_FAM20A_OLAH, SIGLEC1_IL1R2_MMP8, SIGLEC1_ISG15_ILIR2, SIGLEC1_SLC1A2_ILIR2, SIGLEC1IL1R2_OLAH, SIGLEC1_SLC1A2_FAM20A et HERC6_FAM20A_OLAH, et de préférence encore parmi SIGLEC1_IL1R2_FAM20A, HERC6_IL1R2_FAM20A et SIGLEC1_SLC1A2_OLAH.

Selon une autre variante de mode de réalisation particulier, l'étape (a) comprend ou consiste en la mesure de l'expression d'une combinaison d'une combinaison de trois gènes cibles choisie parmi les combinaisons suivantes: IFI27_OLAH_FAM20A, IFI27_IL1R2_FAM20A, IFI27_OLAH _MMP8, IFI27HERC6_OLAH, IFI27_IL1R2_OLAH, IFI27_ISG15_OLAH, IFI27_OLAH _RETN, IFI27_SLC1A2_OLAH, IFI27_SIGLEC1_OLAH, IFI27_SIGLEC1_IL1R2, IFI27_IFI44L_OLAH, IFI27_IFIT1_OLAH, IFI27_OAS_OLAH, IFI27_HERC6_IL1R2, RSAD2_IFI27_OLAH, IFI27_IL1R2_RETN, IFI27_IFI44L_ILIR2, IFI27_IFIT1_IL1R2, IF127_ISG15_IL1R2 et RSAD2_IFI27_IL1R2.

Selon un autre mode de réalisation particulier, l'étape (a) comprend ou consiste en la mesure de l'expression d'au moins quatre gènes cibles.

Selon une variante de ce mode de réalisation, l'étape de mesure comprend ou consiste en la mesure d'au moins deux gènes cibles choisis parmi SIGLEC1, ISG15et HERC6, et d'au moins deux gènes cibles choisis parmi OLAH, FAM20A, IL1R2, MMP8, RETN et SLC1A2, et de préférence IL1R2, OLAH et FAM20A.

Selon une autre variante de ce mode de réalisation, l'étape de mesure comprend ou consiste en la mesure de SIGLEC1 et d'au moins trois gènes cibles choisis parmi SLC1A2, ILIR2, OLAH, FAM20A et RETN.

Selon une autre variante de ce mode de réalisation, l'étape de mesure comprend ou consiste en la mesure de l'expression des gènes cibles SIGLEC1 et ILIR2, et d'au moins deux autres gènes cibles choisis parmi ISG15, HERC6, SLC1A2, OLAH, FAM20A, RETN et MMP8, de préférence parmi SLC1A2, OLAH et FAM20A.

Selon une autre variante de ce mode de réalisation, l'étape de mesure comprend ou consiste en la mesure de l'expression des gènes cibles SIGLEC1 et HERC6, et d'au moins deux autres gènes cibles choisis parmi ISG15, IL1R2, SLC1A2, OLAH, FAM20A, RETN et MMP8. Selon une autre variante de ce mode de réalisation particulier, l'étape de mesure comprend ou consiste en la mesure de l'expression des gènes cibles SIGLEC1, IL1R2et FAM20A, et d'au moins un autre gène cible choisi parmi ISG15, HERC6, SLC1A2, OLAH, RETN et MMP8.

Selon une autre variante de ce mode de réalisation, l'étape de mesure comprend ou consiste en la mesure de l'expression des gènes cibles SIGLEC1 et SLC1A2, et d'au moins deux autres gènes cibles choisis parmi ISG15, IL1R2, HERC6, OLAH, FAM20A, RETN et MMP8, et de préférence IL1R2, OLAH, FAM20A et MMP8.

Selon une autre variante préférée de ce mode de réalisation, l'étape (a) comprend ou consiste en la mesure de l'expression du gène cible viral IFI27, d'un autre gène cible viral choisi parmi HERC6, SIGLEC1, IFI44L et ISG15, du gène cible bactérien FAM20A et d'un autre gène cible bactérien choisi parmi OLAH et IL1R2.

Selon une autre variante de ce mode de réalisation particulier, l'étape (a) comprend ou consiste en la mesure de l'expression d'une combinaison de quatre gènes cibles choisie parmi celles listées dans le tableau 8, et de préférence celles de ce tableau qui présentent une performance en termes d'aire sous la courbe ROC du modèle d'au moins 0,86, d'au moins 0,88, et tout particulièrement, d'au moins 0,90.

Selon une autre variante de ce mode de réalisation particulier, l'étape de mesure comprend ou consiste en la mesure de l'expression d'une combinaison de quatre gènes cibles choisie parmi les combinaisons présentées dans le tableau 3 ci-dessous :

[Tableau 3]

| Combinaisons préférées de 4 gènes | |
|---|---|
| SIGLEC1_IL1R2FAM20A_MMP8 | ISG15HERC6 SLC1A2IL1R2 |
| SIGLEC1_HERC6 IL1R2FAM20A | HERC6 SLC1A2OLAH RETN |
| SIGLEC1_ISG15IL1R2FAM20A | ISG15SLC1A2IL1R2OLAH |
| SIGLEC1_IL1R2FAM20A OLAH | HERC6 IL1R2OLAH RETN |
| SIGLEC1_IL1R2FAM20A RETN | HERC6 IL1R2OLAH MMP8 |
| SIGLEC1_SLC1A2IL1R2FAM20A | HERC6 IL1R2RETN MMP8 |
| SIGLEC1_SLC1A2FAM20A OLAH | SIGLEC1_ISG15OLAH MMP8 |
| ISG15HERC6 IL1R2FAM20A | SIGLEC1_ISG15OLAH RETN |
| SIGLEC1_FAM20A OLAH MMP8 | ISG15HERC6 OLAH RETN |
| HERC6 IL1R2FAM20A OLAH | HERC6 SLC1A2IL1R2RETN |

(suite)

| Combinaisons préférées de 4 gènes | |
|---|---|
| SIGLEC1_SLC1A2OLAH MMP8 | HERC6 SLC1A2FAM20A RETN |
| SIGLEC1_ISG15SLC1A2OLAH | HERC6 OLAH RETN MMP8 |
| SIGLEC1_HERC6 FAM20A OLAH | HERC6 SLC1A2IL1R2MMP8 |
| SIGLEC1_SLC1A2IL1R2OLAH | SIGLEC1_HERC6 SLC1A2RETN |
| SIGLEC1_ISG15FAM20A OLAH | ISG15SLC1A2OLAH MMP8 |
| HERC6 IL1R2FAM20A MMP8 | ISG15SLC1A2OLAH RETN |
| SIGLEC1_HERC6 SLC1A2OLAH | SIGLEC1_ISG15HERC6 RETN |
| SIGLEC1_HERC6 IL1R2MMP8 | ISG15SLC1A2IL1R2RETN |
| HERC6 IL1R2FAM20A RETN | ISG15HERC6 OLAH MMP8 |
| SIGLEC1_FAM20A RETN MMP8 | HERC6 FAM20A RETN MMP8 |
| SIGLEC1_HERC6 FAM20A RETN | SIGLEC1_ISG15SLC1A2RETN |
| SIGLEC1_SLC1A2OLAH RETN | SIGLEC1_HERC6 RETN MMP8 |
| SIGLEC1_ISG15HERC6 IL1R2 | ISG15SLC1A2IL1R2MMP8 |
| SIGLEC1_FAM20A OLAH RETN | ISG15IL1R2OLAH MMP8 |
| SIGLEC1_HERC6 IL1R2OLAH | SIGLEC1_HERC6 SLC1A2MMP8 |
| SIGLEC1_IL1R2OLAH MMP8 | HERC6 SLC1A2FAM20A MMP8 |
| HERC6 SLC1A2IL1R2FAM20A | ISG15IL1R2OLAH RETN |
| SIGLEC1_SLC1A2IL1R2MMP8 | SIGLEC1_SLC1A2RETN MMP8 |
| SIGLEC1_ISG15SLC1A2IL1R2 | ISG15HERC6 SLC1A2FAM20A |
| SIGLEC1_HERC6 SLC1A2IL1R2 | ISG15HERC6 FAM20A MMP8 |
| SIGLEC1_SLC1A2IL1R2RETN | ISG15HERC6 SLC1A2RETN |
| HERC6 SLC1A2FAM20A OLAH | ISG15IL1R2RETN MMP8 |
| SIGLEC1_ISG15FAM20A RETN | SIGLEC1_ISG15SLC1A2MMP8 |
| SIGLEC1_ISG15IL1R2MMP8 | SLC1A2IL1R2FAM20A OLAH |
| SIGLEC1_SLC1A2FAM20A MMP8 | ISG15FAM20A OLAH MMP8 |
| SIGLEC1_ISG15IL1R2OLAH | SLC1A2FAM20A OLAH MMP8 |
| SIGLEC1_ISG15SLC1A2FAM20A | ISG15FAM20A OLAH RETN |
| SIGLEC1_HERC6 IL1R2RETN | ISG15HERC6 RETN MMP8 |
| SIGLEC1_SLC1A2FAM20A RETN | SIGLEC1_ISG15RETN MMP8 |
| SIGLEC1_HERC6 SLC1A2FAM20A | SLC1A2FAM20A OLAH RETN |
| HERC6 FAM20A OLAH RETN | SIGLEC1_ISG15HERC6 SLC1A2 |
| SIGLEC1_IL1R2RETN MMP8 | ISG15OLAH RETN MMP8 |
| HERC6 FAM20A OLAH MMP8 | HERC6 SLC1A2RETN MMP8 |
| ISG15HERC6 FAM20A OLAH | ISG15HERC6 SLC1A2MMP8 |
| ISG15SLC1A2IL1R2FAM20A | SLC1A2IL1R2OLAH RETN |
| SIGLEC1_ISG15HERC6 FAM20A | SIGLEC1_ISG15HERC6 MMP8 |
| ISG15IL1R2FAM20A OLAH | SLC1A2IL1R2OLAH MMP8 |
| SIGLEC1_ISG15FAM20A MMP8 | ISG15SLC1A2FAM20A RETN |

(suite)

| Combinaisons préférées de 4 gènes | |
|---|---|
| SIGLEC1_ISG15IL1R2RETN | SLC1A2OLAH RETN MMP8 |
| SIGLEC1_IL1R2OLAH RETN | IL1R2FAM20A OLAH MMP8 |
| SIGLEC1_HERC6 FAM20A MMP8 | ISG15SLC1A2FAM20A MMP8 |
| ISG15HERC6 FAM20A RETN | IL1R2FAM20A OLAH RETN |
| ISG15IL1R2FAM20A MMP8 | ISG15FAM20A RETN MMP8 |
| ISG15HERC6 IL1R2RETN | SLC1A2IL1R2FAM20A MMP8 |
| ISG15SLC1A2FAM20A OLAH | SLC1A2IL1R2FAM20A RETN |
| SIGLEC1_HERC6 OLAH MMP8 | ISG15SLC1A2RETN MMP8 |
| ISG15HERC6 IL1R2MMP8 | IL1R2OLAH RETN MMP8 |
| ISG15HERC6 IL1R2OLAH | FAM20A OLAH RETN MMP8 |
| SIGLEC1_HERC6 OLAH RETN | IL1R2FAM20A RETN MMP8 |
| ISG15HERC6 SLC1A2OLAH | SLC1A2IL1R2RETN MMP8 |
| ISG15IL1R2FAM20A RETN | HERC6 SLC1A2OLAH MMP8 |
| SIGLEC1_OLAH RETN MMP8 | SIGLEC1_ISG15HERC6 OLAH |
| HERC6 SLC1A2IL1R2OLAH | |

De préférence, selon cette variante, l'étape de mesure comprend ou consiste en la mesure de l'expression d'une combinaison de quatre gènes cibles choisie parmi les combinaisons suivantes : SIGLEC1_IL1R2_FAM20A_MMP8, SIGLEC1_HERC6__IL1R2_FAM20A, SIGLEC1_ISG15_IL1R2_FAM20A, SIGLEC1_IL1R2_FAM20A_OLAH, SIGLEC1_IL1R2_FAM20A_RETN, SIGLEC1_SLC1A2_IL1R2 FAM20A, SIGLEC1_SLC1A2_FAM20AOLAH, ISG15_HERC6_IL1R2_FAM20A, SIGLEC1_FAM20A_OLAH_MMP8, et HERC6_IL1R2_FAM20A_OLAH.

Selon un autre mode de réalisation particulier, l'étape (a) comprend ou consiste en la mesure de l'expression d'au moins cinq gènes cibles.

Selon une variante de ce mode de réalisation, l'étape de mesure comprend ou consiste en la mesure de l'expression d'au moins deux gènes cibles choisis parmi SIGLEC1, ISG15et HERC6 et d'au moins trois autres gènes cibles choisis parmi IL1R2, SLC1A2, OLAH, FAM20A, RETN et MMP8.

Selon une autre variante de ce mode de réalisation, l'étape de mesure comprend ou consiste en la mesure de l'expression des gènes cibles SIGLEC1, ISG15et HERC6 et d'au moins deux autres gènes cibles choisis parmi IL1R2, SLC1A2, OLAH, FAM20A, RETN et MMP8, de préférence parmi IL1R2, OLAH et FAM20A.

Selon une variante de ce mode de réalisation, l'étape de mesure comprend ou consiste en la mesure de l'expression des gènes cibles SIGLEC1 et ISG15et d'au moins trois autres gènes cibles choisis parmi HERC6, IL1R2, SLC1A2, OLAH, FAM20A, RETN et MMP8, de préférence parmi IL1R2, OLAH, FAM20A et MMP8.

Selon une variante de ce mode de réalisation, l'étape de mesure comprend ou consiste en la mesure de l'expression des gènes cibles SIGLEC1 et IL1R2et d'au moins trois autres gènes cibles choisis parmi HERC6, ISG15, SLC1A2, OLAH, FAM20A, RETN et MMP8, de préférence parmi OLAH, SLC1A2, FAM20A et MMP8.

Selon une variante de ce mode de réalisation, l'étape de mesure comprend ou consiste en la mesure de l'expression des gènes cibles SIGLEC1 et MMP8 et d'au moins trois autres gènes cibles choisis parmi HERC6, ISG15, SLC1A2, OLAH, FAM20A, RETN et IL1R2, de préférence parmi OLAH, SLC1A2, FAM20A et IL1R2.

Selon une variante de ce mode de réalisation, l'étape de mesure comprend ou consiste en la mesure de l'expression des gènes cibles SIGLEC1 et HERC6 et d'au moins trois autres gènes cibles choisis parmi ISG15, ILIR2, SLC1A2, OLAH, FAM20A, RETN et _MMP8, de préférence parmi IL1R2, OLAH, FAM20A et MMP8.

Selon une autre variante de ce mode de réalisation, l'étape de mesure comprend ou consiste en la mesure de l'expression des gènes cibles SIGLEC1, FAM20A et MMP8, et d'au moins deux autres gènes cibles choisis parmi ILIR2, ISG15, HERC6, SLC1A2, OLAH, et RETN, de préférence parmi ILIR2, ISG15, SLC1A2et OLAH.

Selon une variante de ce mode de réalisation, l'étape de mesure comprend ou consiste en la mesure de l'expression des gènes cibles SIGLEC1, IL1R2et HERC6 et d'au moins deux autres gènes cibles choisis parmi ISG15, SLC1A2, OLAH, FAM20A, RETN et MMP8.

Selon une autre variante de ce mode de réalisation, l'étape de mesure comprend ou consiste en la mesure de l'expression des gènes cibles SIGLEC1, IL1R2et ISG15et d'au moins deux autres gènes cibles choisis parmi HERC6, SLC1A2, OLAH, FAM20A, RETN et MMP8.

Selon une autre variante de ce mode de réalisation, l'étape de mesure comprend ou consiste en la mesure de l'expression d'une combinaison de cinq gènes cibles choisie parmi les combinaisons présentées dans le tableau 4 ci-dessous :

[Tableau 4]

| Combinaisons préférées de 5 gènes | |
|---|---|
| SIGLEC1_HERC6 IL1R2FAM20A MMP8 | SIGLEC1_ISG15SLC1A2IL1R2RETN |
| SIGLEC1_IL1R2FAM20A OLAH MMP8 | SIGLEC1_ISG15SLC1A2FAM20A RETN |
| SIGLEC1_ISG15IL1R2FAM20A MMP8 | SIGLEC1_HERC6 SLC1A2IL1R2RETN |
| SIGLEC1_IL1R2FAM20A RETN MMP8 | HERC6 SLC1A2IL1R2FAM20A RETN |
| SIGLEC1_SLC1A2IL1R2FAM20A MMP8 | SIGLEC1_HERC6 SLC1A2OLAH RETN |
| SIGLEC1_ISG15HERC6 IL1R2FAM20A | SIGLEC1_SLC1A2IL1R2RETN MMP8 |
| SIGLEC1_HERC6 IL1R2FAM20A OLAH | SIGLEC1_ISG15IL1R2RETN MMP8 |
| SIGLEC1_SLC1A2FAM20A OLAH MMP8 | SIGLEC1_HERC6 SLC1A2FAM20A MMP8 |
| SIGLEC1_ISG15IL1R2FAM20A OLAH | HERC6 SLC1A2IL1R2FAM20A MMP8 |
| SIGLEC1_ISG15IL1R2FAM20A RETN | HERC6 FAM20A OLAH RETN MMP8 |
| SIGLEC1_HERC6 IL1R2FAM20A RETN | SIGLEC1_ISG15HERC6 SLC1A2FAM20A |
| SIGLEC1_HERC6 SLC1A2IL1R2FAM20A | HERC6 SLC1A2FAM20A OLAH RETN |
| SIGLEC1_SLC1A2IL1R2FAM20A OLAH | ISG15HERC6 FAM20A OLAH RETN |
| SIGLEC1_IL1R2FAM20A OLAH RETN | SIGLEC1_IL1R2OLAH RETN MMP8 |
| SIGLEC1_SLC1A2IL1R2FAM20A RETN | SIGLEC1_ISG15HERC6 IL1R2RETN |
| SIGLEC1_ISG15SLC1A2IL1R2FAM20A | SIGLEC1_HERC6 IL1R2OLAH RETN |
| SIGLEC1_ISG15SLC1A2FAM20A OLAH | ISG15HERC6 FAM20A OLAH MMP8 |
| SIGLEC1_HERC6 SLC1A2FAM20A OLAH | SIGLEC1_ISG15HERC6 FAM20A MMP8 |
| SIGLEC1_HERC6 FAM20A OLAH MMP8 | SIGLEC1_ISG15IL1R2OLAH RETN |
| SIGLEC1_FAM20A OLAH RETN MMP8 | SIGLEC1_HERC6 OLAH RETN MMP8 |
| SIGLEC1_ISG15FAM20A OLAH MMP8 | ISG15IL1R2FAM20A OLAH MMP8 |
| ISG15HERC6 IL1R2FAM20A OLAH | ISG15SLC1A2IL1R2FAM20A RETN |
| SIGLEC1_SLC1A2FAM20A OLAH RETN | ISG15HERC6 IL1R2RETN MMP8 |
| ISG15HERC6 IL1R2FAM20A MMP8 | ISG15SLC1A2IL1R2FAM20A MMP8 |
| ISG15HERC6 IL1R2FAM20A RETN | SIGLEC1_ISG15HERC6 OLAH RETN |
| HERC6 IL1R2FAM20A OLAH MMP8 | ISG15HERC6 SLC1A2IL1R2OLAH |
| SIGLEC1_ISG15HERC6 FAM20A OLAH | ISG15HERC6 SLC1A2OLAH MMP8 |
| SIGLEC1_HERC6 FAM20A RETN MMP8 | ISG15HERC6 SLC1A2FAM20A RETN |
| SIGLEC1_ISG15SLC1A2OLAH MMP8 | SIGLEC1_ISG15HERC6 OLAH MMP8 |
| SIGLEC1_SLC1A2IL1R2OLAH MMP8 | ISG15HERC6 SLC1A2OLAH RETN |
| SIGLEC1_ISG15SLC1A2IL1R2OLAH | SIGLEC1_ISG15OLAH RETN MMP8 |
| SIGLEC1_HERC6 FAM20A OLAH RETN | ISG15IL1R2FAM20A OLAH RETN |
| SIGLEC1_HERC6 SLC1A2OLAH MMP8 | ISG15SLC1A2FAM20A OLAH MMP8 |

(suite)

| Combinaisons préférées de 5 gènes | |
|---|---|
| HERC6 IL1R2FAM20A OLAH RETN | HERC6 SLC1A2IL1R2OLAH RETN |
| SIGLEC1_ISG15HERC6 FAM20A RETN | ISG15HERC6 IL1R2OLAH MMP8 |
| SIGLEC1_ISG15FAM20A RETN MMP8 | ISG15HERC6 IL1R2OLAH RETN |
| SIGLEC1_SLC1A2IL1R2OLAH RETN | ISG15IL1R2FAM20A RETN MMP8 |
| SIGLEC1_HERC6 IL1R2OLAH MMP8 | HERC6 IL1R2OLAH RETN MMP8 |
| SIGLEC1_HERC6 SLC1A2IL1R2OLAH | HERC6 SLC1A2OLAH RETN MMP8 |
| SIGLEC1_ISG15HERC6 SLC1A2OLAH | ISG15HERC6 SLC1A2IL1R2RETN |
| SIGLEC1_ISG15HERC6 IL1R2MMP8 | HERC6 SLC1A2IL1R2OLAH MMP8 |
| ISG15HERC6 SLC1A2IL1R2FAM20A | ISG15HERC6 FAM20A RETN MMP8 |
| SIGLEC1_SLC1A2FAM20A RETN MMP8 | ISG15HERC6 SLC1A2IL1R2MMP8 |
| HERC6 SLC1A2IL1R2FAM20A OLAH | ISG15SLC1A2FAM20A OLAH RETN |
| SIGLEC1_ISG15FAM20A OLAH RETN | ISG15HERC6 OLAH RETN MMP8 |
| SIGLEC1_ISG15SLC1A2OLAH RETN | ISG15SLC1A2IL1R2OLAH RETN |
| SIGLEC1_HERC6 IL1R2RETN MMP8 | ISG15SLC1A2IL1R2OLAH MMP8 |
| HERC6 IL1R2FAM20A RETN MMP8 | SIGLEC1_ISG15HERC6 SLC1A2RETN |
| SIGLEC1_HERC6 SLC1A2IL1R2MMP8 | HERC6 SLC1A2IL1R2RETN MMP8 |
| SIGLEC1_SLC1A2OLAH RETN MMP8 | HERC6 SLC1A2FAM20A RETN MMP8 |
| ISG15SLC1A2IL1R2FAM20A OLAH | SIGLEC1_HERC6 SLC1A2RETN MMP8 |
| SIGLEC1_HERC6 SLC1A2FAM20A RETN | SIGLEC1_ISG15HERC6 RETN MMP8 |
| SIGLEC1_ISG15HERC6 IL1R2OLAH | ISG15HERC6 SLC1A2FAM20A MMP8 |
| SIGLEC1_ISG15HERC6 SLC1A2IL1R2 | ISG15SLC1A2OLAH RETN MMP8 |
| ISG15HERC6 SLC1A2FAM20A OLAH | SIGLEC1_ISG15SLC1A2RETN MMP8 |
| SIGLEC1_ISG15SLC1A2IL1R2MMP8 | SIGLEC1_ISG15HERC6 SLC1A2MMP8 |
| SIGLEC1_ISG15IL1R2OLAH MMP8 | ISG15SLC1A2IL1R2RETN MMP8 |
| SIGLEC1_ISG15SLC1A2FAM20A MMP8 | ISG15IL1R2OLAH RETN MMP8 |
| HERC6 SLC1A2FAM20A OLAH MMP8 | ISG15FAM20A OLAH RETN MMP8 |
| SLC1A2FAM20A OLAH RETN MMP8 | SLC1A2IL1R2FAM20A OLAH MMP8 |
| SLC1A2IL1R2OLAH RETN MMP8 | ISG15HERC6 SLC1A2RETN MMP8 |
| ISG15SLC1A2FAM20A RETN MMP8 | SLC1A2IL1R2FAM20A OLAH RETN |
| IL1R2FAM20A OLAH RETN MMP8 | SLC1A2IL1R2FAM20A RETN MMP8 |

De préférence, selon cette variante, l'étape de mesure comprend ou consiste en la mesure de l'expression d'une combinaison de cinq gènes cibles choisie parmi les combinaisons suivantes : SIGLEC1_HERC6_IL1R2_FAM20A_MMP8, SIGLEC1_IL1R2_FAM20A_OLAH MMP8 ; SIGLEC1_ISG15_IL1R2_FAM20A_MMP8 ; SIGLEC1_IL1R2_FAM20A_RETN_MMP8 ; SIGLEC1_SLC1A2_IL1R2_FAM20A_MMP8 ; SIGLEC1_ISG15_HERC6_IL1R2_FAM20A ; SIGLEC1_HERC6_IL1R2_FAM20A_OLAH ; SIGLEC1_SLC1A2_FAM20A_OLAH_MMP8 et SIGLEC1_ISG15_IL1R2_FAM20A_OLAH.

Selon une variante préférée de ce mode de réalisation, l'étape (a) comprend ou consiste en la mesure de l'expression du gène cible viral IFI27, de deux autres gènes cibles viraux choisis parmi SIGLEC1, HERC6, OAS1 et IFIT1, du gène cible bactérien FAM20A et d'un autre gène cible bactérien choisi parmi OLAH, IL1R2 et MMP8.

Selon une autre variante préférée de ce mode de réalisation, l'étape (a) comprend ou consiste en la mesure de l'expression d'une combinaison de cinq gènes cibles choisie parmi celles listées dans le tableau 9, et de préférence celles de ce tableau qui présentent une performance en termes d'aire sous la courbe ROC du modèle d'au moins 0,86, d'au moins 0,88, et tout particulièrement, d'au moins 0,90.

Selon une autre variante préférée de ce mode de réalisation, l'étape (a) comprend ou consiste en la mesure de l'expression des gènes cibles viraux IFI27 et SIGLEC1, et des gènes cibles bactériens FAM20A, IL1R2 et MMP8.

Selon un autre mode de réalisation particulier, l'étape (a) comprend ou consiste en la mesure de l'expression d'au moins six gènes cibles.

Selon une variante de ce mode de réalisation particulier, l'étape de mesure comprend ou consiste en la mesure de l'expression des gènes cibles SIGLEC1, ISG15 et HERC6 et d'au moins trois autres gènes cibles choisis parmi IL1R2, SLC1A2, OLAH, FAM20A, RETN et MMP8.

Selon une autre variante de ce mode de réalisation particulier, l'étape de mesure comprend ou consiste en la mesure de l'expression des gènes cibles SIGLEC1, FAM20A et MMP8 et d'au moins trois autres gènes cibles choisis parmi ISG15, HERC6, IL1R2, SLC1A2, OLAH, et RETN.

Selon une autre variante de ce mode de réalisation particulier, l'étape de mesure comprend ou consiste en la mesure de l'expression des gènes cibles SIGLEC1, ISG15 et SLC1A2, et d'au moins trois autres gènes cibles choisis parmi HERC6, IL1R2, OLAH, FAM20A, MMP8 et RETN.

Selon une autre variante de ce mode de réalisation particulier, l'étape de mesure comprend ou consiste en la mesure de l'expression des gènes cibles SIGLEC1 et IL1R2, et d'au moins quatre autres gènes cibles choisis parmi, HERC6, SLC1A2, OLAH, FAM20A, RETN et MMP8.

Selon une autre variante de ce mode de réalisation particulier, l'étape de mesure comprend ou consiste en la mesure de l'expression des gènes cibles SIGLEC1 et HERC6, et d'au moins quatre autres gènes cibles choisis parmi ISG15, IL1R2, SLC1A2, OLAH, FAM20A, RETN et MMP8.

Selon une autre variante de ce mode de réalisation particulier, l'étape de mesure comprend ou consiste en la mesure de l'expression des gènes cibles SIGLEC1 et MMP8, et d'au moins quatre autres gènes cibles choisis parmi HERC6, ISG15, IL1R2, SLC1A2, OLAH, FAM20A, et RETN.

Selon une autre variante de ce mode de réalisation particulier, l'étape de mesure comprend ou consiste en la mesure de l'expression des gènes cibles SIGLEC1 et OLAH, et d'au moins quatre autres gènes cibles choisis parmi HERC6, ISG15, IL1R2, SLC1A2, MMP8, FAM20A, et RETN.

Selon une autre variante de ce mode de réalisation particulier, l'étape de mesure comprend ou consiste en la mesure de l'expression d'une combinaison de six gènes cibles choisis parmi les combinaisons suivantes :

SIGLEC1_ISG15_IL1R2_FAM20A_OLAH_MMP8 ;
SIGLEC1_HERC6_IL1R2_FAM20A_OLAH_MMP8 ;
SIGLEC1_ISG15_HERC6_IL1R2_FAM20A_MMP8 ;
SIGLEC1_SLC1A2_IL1R2_FAM20A_OLAH_MMP8 ;
SIGLEC1_HERC6_IL1R2_FAM20A_RETN_MMP8 ;
SIGLEC1_ISG15_IL1R2_FAM20A_RETN_MMP8 ;
SIGLEC1_IL1R2_FAM20A_OLAH_RETN_MMP8 ;
SIGLEC1_ISG15_SLC1A2_IL1R2_FAM20A_MMP8;
SIGLEC1_HERC6_SLC1A2_IL1R2_FAM20A_MMP8 ;
SIGLEC1_SLC1A2_IL1R2_FAM20A_RETN_MMP8 ;
SIGLEC1_ISG15_SLC1A2_FAM20A_OLAH_MMP8 ;
SIGLEC1_ISG15_HERC6_IL1R2_FAM20A_OLAH;
SIGLEC1_HERC6_SLC1A2_FAM20A_OLAH_MMP8 ;
SIGLEC1_ISG15_IL1R2_FAM20A_OLAH_RETN;
SIGLEC1_HERC6_SLC1A2_IL1R2_FAM20A_OLAH;
SIGLEC1_SLC1A2_IL1R2_FAM20A_OLAH_RETN ;
SIGLEC1_SLC1A2_FAM20A_OLAH_RETN_MMP8 ;
SIGLEC1_ISG15_SLC1A2_IL1R2_FAM20A_OLAH ;
SIGLEC1_ISG15_HERC6_IL1R2_FAM20A_RETN;
SIGLEC1_HERC6_IL1R2_FAM20A_OLAH_RETN ;
SIGLEC1_ISG15_HERC6_SLC1A2_IL1R2_FAM20A ;
SIGLEC1_ISG15_SLC1A2_IL1R2_FAM20A_RETN ;
SIGLEC1_HERC6_FAM20A_OLAH_RETN_MMP8 ;
SIGLEC1_ISG15_HERC6_FAM20A_OLAH_MMP8 ;
SIGLEC1_ISG15_HERC6_SLC1A2_FAM20A_OLAH ;
SIGLEC1_HERC6_SLC1A2_IL1R2_FAM20A_RETN ;

SIGLEC1_ISG15_FAM20A_OLAH_RETN_MMP8 ;
SIGLEC1_ISG15_SLC1A2_FAM20A_OLAH_RETN ;
ISG15_HERC6_ILIR2_FAM20A_OLAH_MMP8 ;
SIGLEC1_ISG15_HERC6_FAM20A_RETN_MMP8 ;
ISG15_HERC6_ILIR2_FAM20A_RETNMMP8 ;
SIGLEC1_HERC6_SLC1A2_FAM20A_OLAH_RETN ;
ISG15_HERC6_IL1R2_FAM20A_OLAH_RETN ;
SIGLEC1_ISG15_HERC6_FAM20A_OLAH_RETN;
SIGLEC1_ISG15_SLC1A2_IL1R2_OLAH MMP8 ;
SIGLEC1_HERC6_SLC1A2_IL1R2_OLAH_MMP8 ;
SIGLEC1_ISG15_HERC6_SLC1A2_OLAH_MMP8 ;
HERC6_IL1R2_FAM20A_OLAH_RETN_MMP8 ;
SIGLEC1_HERC6_SLC1A2_FAM20A_RETN_MMP8 ;
ISG15_HERC6_SLC1A2_IL1R2_FAM20A_OLAH ;
SIGLEC1_ISG15_HERC6_SLC1A2_IL1R2_OLAH ;
SIGLEC1_SLC1A2_IL1R2_OLAH_RETN_MMP8 ;
SIGLEC1_ISG15_SLC1A2_FAM20A_RETN_MMP8 ;
SIGLEC1_ISG15_SLC1A2_IL1R2_OLAH_RETN ;
HERC6_SLC1A2_IL1R2_FAM20A_OLAH_MMP8 ;
SIGLEC1_ISG15_HERC6_SLC1A2_FAM20A_RETN ;
ISG15_HERC6_SLC1A2_IL1R2_FAM20A_RETN ;
SIGLEC1_ISG15_HERC6_IL1R2_RETN_MMP8 ;
SIGLEC1_ISG15_SLC1A2_OLAH_RETN_MMP8 ;
SIGLEC1_HERC6_IL1R2_OLAH_RETN_MMP8 ;
SIGLEC1_ISG15HERC6_IL1R2_OLAH_MMP8 ;
SIGLEC1_HERC6_SLC1A2_OLAH_RETN_MMP8 ;
HERC6_SLC1A2_IL1R2_FAM20A_OLAH_RETN ;
ISG15_HERC6_SLC1A2_IL1R2_FAM20A_MMP8 ;
SIGLEC1_ISG15_HERC6_SLC1A2_IL1R2_MMP8 ;
SIGLEC1_HERC6_SLC1A2_IL1R2_OLAHRETN ;
ISG15_HERC6_FAM20A_OLAH_RETN_MMP8 ;
SIGLEC1_ISG15_HERC6_SLC1A2_OLAH_RETN;
SIGLEC1_HERC6_SLC1A2_IL1R2_RETN_MMP8 ;
ISG15_HERC6_SLC1A2_FAM20A_OLAH_RETN ;
HERC6_SLC1A2_FAM20A_OLAH_RETN_MMP8 ;
SIGLEC1_ISG15_HERC6_SLC1A2_FAM20A_MMP8 ;
ISG15_HERC6_SLC1A2_FAM20A_OLAH_MMP8 ;
SIGLEC1_ISG15_SLC1A2_IL1R2_RETN_MMP8 ;
HERC6_SLC1A2_IL1R2_FAM20_RETN_MMP8 ;
SIGLEC1_ISG15_HERC6_SLC1A2_IL1R2_RETN ;
SIGLEC1_ISG15_IL1R2_OLAH_RETN_MMP8;
ISG15_SLC1A2_IL1R2_FAM20A_OLAH_MMP8 ;
SIGLEC1_ISG15_HERC6_IL1R2_OLAHRETN ;
ISG15_SLC1A2_IL1R2_FAM20A_OLAH_RETN ;
SIGLEC1_ISG15_HERC6_OLAH_RETN_MMP8;
ISG15_HERC6_IL1R2_OLAH_RETN_MMP8 ;
ISG15_HERC6_SLC1A2_IL1R2_OLAH_RETN ;
ISG15_HERC6_SLC1A2_OLAH_RETN_MMP8 ;
ISG15_HERC6_SLC1A2_IL1R2_OLAH_MMP8 ;
HERC6_SLC1A2_IL1R2_OLAH_RETN_MMP8 ;
ISG15_SLC1A2_IL1R2_FAM20A_RETN_MMP8 ;
ISG15_IL1R2_FAM20A_OLAH_RETN_MMP8 ;
ISG15_HERC6_SLC1A2_FAM20A_RETN_MMP8 ;
ISG15_HERC6_SLC1A2_IL1R2_RETN_MMP8 ;
ISG15_SLC1A2_FAM20A_OLAH_RETN_MMP8 ;
SIGLEC1_ISG15_HERC6_SLC1A2_RETN_MMP8 ;
ISG15_SLC1A2_IL1R2_OLAH_RETN_MMP8 ; et SLC1A2_IL1R2_FAM20A_OLAH_RETN_MMP8.

De préférence, selon cette variante, l'étape de mesure comprend ou consiste en la mesure de l'expression d'une combinaison de six gènes cibles choisie parmi les combinaisons suivantes :

SIGLEC1_ISG15_IL1R2_FAM20A_OLAH_MMP8 ;
SIGLEC1_HERC6_IL1R2_FAM20A_OLAH_MMP8 ;
SIGLEC1_ISG15_HERC6_IL1R2_FAM20_MMP8 ;
S1GLEC1_SLC1A2_IL1R2_FAM20A_OLAH_MMP8;
SIGLEC1_HERC6_IL1R2_FAM20A_RETN_MMP8 ;
SIGLEC1_ISG15_IL1R2_FAM20A_RETN_MMP8 ; et
S1GLEC1_IL1R2_FAM20A_OLAH_RETN_MMP8

Selon une variante préférée de ce mode de réalisation, l'étape (a) comprend ou consiste en la mesure de l'expression des gènes cibles viraux IFI27 et SIGLEC1, d'un autre gène cible viral choisi parmi IFIT1, HERC6, ISG15 et ISG15, des gènes cibles bactériens FAM20A et MMP8, et d'un autre gène cible bactérien choisi parmi OLAH et IL1R2.

Selon une autre variante préférée de ce mode de réalisation, l'étape (a) comprend ou consiste en la mesure de l'expression d'une combinaison de six gènes cibles choisie parmi celles listées dans le tableau 10, et de préférence celles de ce tableau qui présentent une performance en termes d'aire sous la courbe ROC du modèle d'au moins 0,86, d'au moins 0,88, et tout particulièrement, d'au moins 0,90.

Selon un autre mode de réalisation particulier, l'étape (a) comprend ou consiste en la mesure de l'expression d'au moins sept gènes cibles.

Selon une variante de ce mode de réalisation particulier, l'étape (a) comprend ou consiste en la mesure de l'expression du gène cible SIGLEC1 et de six autres gènes cibles choisis parmi ISG15, HERC6, OLAH, FAM20A, IL1R2, MMP8, RETN et SLC1A2.

Selon une autre variante de ce mode de réalisation particulier, l'étape (a) comprend ou consiste en la mesure de l'expression du gène cible MMP8 et de six autres gènes cibles choisis parmi ISG15, HERC6, SLC1A2, IL1R2, FAM20A, OLAH, RETN et SIGLEC1.

Selon une autre variante de ce mode de réalisation particulier, l'étape de mesure comprend ou consiste en la mesure de l'expression des gènes cibles SIGLEC1 et MMP8, et d'au moins quatre autres gènes cibles choisis parmi ISG15, HERC6, IL1R2, SLC1A2, OLAH, FAM20A, et RETN, de préférence parmi ISG15, HERC6, IL1R2, OLAH, FAM20A, et RETN.

Selon une autre variante de ce mode de réalisation particulier, l'étape de mesure comprend ou consiste en la mesure de l'expression des gènes cibles SIGLEC1 et ISG15, et de cinq autres gènes cibles choisis parmi HERC6, SLC1A2, IL1R2, FAM20A, OLAH, RETN et MMP8.

Selon une autre variante de ce mode de réalisation particulier, l'étape de mesure comprend ou consiste en la mesure de l'expression des gènes cibles SIGLEC1, ISG15 et HERC6, et de quatre autres gènes cibles choisis parmi SLC1A2, IL1R2, FAM20A, OLAH, RETN et MMP8.

Selon une autre variante de ce mode de réalisation particulier, l'étape de mesure comprend ou consiste en la mesure de l'expression des gènes cibles SIGLEC1, FAM20A et MMP8, et d'au moins trois autres gènes cibles choisis parmi ISG15, HERC6, IL1R2, SLC1A2, OLAH et RETN, et de préférence parmi ISG15, HERC6, IL1R2, OLAH et RETN.

Selon une autre variante de ce mode de réalisation particulier, l'étape de mesure comprend ou consiste en la mesure de l'expression des gènes cibles SIGLEC1, FAM20A, IL1R2 et MMP8, et d'au moins deux autres gènes cibles choisis parmi ISG15, HERC6, SLC1A2, OLAH et RETN, et de préférence parmi ISG15, HERC6, OLAH et RETN.

Selon une autre variante de ce mode de réalisation particulier, l'étape de mesure comprend ou consiste en la mesure de l'expression de sept gènes cibles choisis parmi les combinaisons suivantes :

SIGLEC1_ISG15_HERC6_IL1R2_FAM20A_OLAH_MMP8 ;
SIGLEC1_ISG15_SLC1A2_IL1R2_FAM20A_OLAH_MMP8 ;
IGLEC1_HERC6_SLC1A2_IL1R2_FAM20A_OLAH_MMP8 ;
SIGLEC1_ISG15_IL1R2_FAM20A_OLAH_RETN_MMP8 ;
SIGLEC1_ISG15_HERC6_IL1R2_FAM20A_RETN_MMP8 ;
SIGLEC1_HERC6_IL1R2_FAM20A_OLAH_RETN_MMP8 ;
SIGLEC1_ISG15_HERC6_SLC1A2_IL1R2_FAM20A_MMP8 ;
SIGLEC1_ISG15_SLC1A2_IL1R2-FAM20A_RETN_MMP8 ;
SIGLEC1_SLC1A2_IL1R2_FAM20A_OLAH_RETN_MMP8 ;
SIGLEC1_HERC6_SLC1A2_IL1R2_FAM20A_RETN_MMP8 ;
SIGLEC1_ISG15_HERC6_SLC1A2_FAM20_OLAHMMP8,
SIGLEC1_ISG15_SLC1A2_FAM20A_OLAH_RETN_MMP8 ;
SIGLEC1_ISG15_SLC1A2_IL1R2_FAM20A_OLAH_RETN ;

SIGLEC1_HERC6_SLC1A2_FAM20A_OLAH_RETN_MMP8 ;
SIGLEC1_ISG15HERC6_IL1R2_FAM20A_OLAH_RETN ;
SIGLEC1_ISG15_HERC6_SLC1A2_IL1R2_FAM20A_OLAH ;
SIGLEC1_ISG15_HERC6_FAM20A_OLAH_RETN_MMP8 ;
SIGLEC1_HERC6_SLC1A2_IL1R2_FAM20A_OLAH_RETN ;
SIGLEC1_ISG15_HERC6_SLC1A2_IL1R2_FAM20N ;
SIGLEC1_ISG15_HERC6_SLC1A2_FAM20A_OLAH_RETN ;
ISG15HERC6_ILIR2_FAM20A_OLAH_RETN_MMP8 ;
SIGLEC1_ISG15HERC6_SLC1A2_FAM20A_RETN_MMP8 ;
SIGLEC1_ISG15_HERC6_SLC1A2_IL1R2_OLAH_MMP8 ;
SIGLEC1_ISG15_SLC1A2_IL1R2_OLAH_RETN_MMP8 ;
ISG15_HERC6_SLC1A2_IL1R2_FAM20A_RETN_MMP8 ;
ISG15_HERC6_SLC1A2_IL1R2_FAM20A_OLAH_MMP8 ;
SIGLEC1_HERC6_SLC1A2_IL1R2_OLAH_RETN_MMP8 ;
ISG15_HERC6_SLC1A2_IL1R2_FAM20A_OLAH_RETN ;
HERC6_SLC1A2_IL1R2_FAM20A_OLAH_RETN_MMP8 ;
SIGLEC1_ISG15_HERC6_IL1R2_OLAH_RETN_MMP8 ;
SIGLEC1_ISG15_HERC6_SLC1A2_OLAH_RETN_MMP8 ;
ISG15_HERC6_SLC1A2_FAM20A_OLAH_RETN_MMP8 ;
SIGLEC1_ISG15_HERC6_SLC1A2_IL1R2_OLAH_RETN ;
SIGLEC1_ISG15_HERC6_SLC1A2_IL1R2_RETN_MMP8 ;
ISG15_SLC1A2_IL1R2_FAM20A_OLAH_RETN_MMP8 ;
et ISG15_HERC6_SLC1A2_IL1R2_OLAH_RETN_MMP8.

De préférence, selon cette variante, l'étape de mesure comprend ou consiste en la mesure de l'expression de sept gènes cibles choisis parmi les combinaisons suivantes :

SIGLEC1_ISG15_SLC1A2_IL1R2_FAM20A_OLAH_MMP8 ;
IGLEC1_HERC6_SLC1A2_IL1R2_FAM20A_OLAH_MMP8 ;
SIGLEC1_ISG15_IL1R2_FAM20A_OLAH_RETN_MMP8 ;
SIGLEC1_ISG15_HERC6_IL1R2_FAM20A_RETN_MMP8 ;
SIGLEC1_HERC6_IL1R2_FAM20A_OLAH_RETN_MMP8 ;
SIGLEC1_ISG15_HERC6_SLC1A2_IL1R2_FAM20A_MMP8 ; et
SIGLEC1_ISG15_SLC1A2_IL1R2_FAM20A_RETN_MMP8.

Selon une variante préférée de ce mode de réalisation particulier, l'étape (a) comprend ou consiste en la mesure de l'expression des gènes cibles viraux IFI27 et SIGLEC1, et de deux autres gènes cibles viraux choisis parmi IFIT1, OAS1, HERC6 et ISG15, des gènes cibles bactériens FAM20A et MMP8, et d'un autre gène cible bactérien choisi parmi OLAH et IL1R2

Selon une autre variante préférée de ce mode de réalisation particulier, l'étape (a) comprend ou consiste en la mesure de l'expression d'une combinaison de sept gènes cibles choisie parmi celles listées dans le tableau 11, et de préférence celles de ce tableau qui présentent une performance en termes d'aire sous la courbe ROC du modèle d'au moins 0,86, d'au moins 0,88, et tout particulièrement, d'au moins 0,90.

Selon un autre mode de réalisation particulier, l'étape (a) comprend ou consiste en la mesure de l'expression d'au moins huit gènes cibles.

Selon une variante de ce mode de réalisation particulier, l'étape de mesure comprend ou consiste en la mesure de l'expression de SIGLEC1 et ISG15, et d'au moins six autres gènes cibles choisis parmi HERC6, SLC1A2, IL1R2, FAM20A, OLAH, RETN et MMP8.

Selon une autre variante de ce mode de réalisation particulier, l'étape de mesure comprend ou consiste en la mesure de l'expression de SIGLEC1, MMP8 et d'au moins six autres gènes cibles choisis parmi HERC6, ISG15, SLC1A2, IL1R2, FAM20A, OLAH, et RETN.

Selon une autre variante de ce mode de réalisation particulier, l'étape de mesure comprend ou consiste en la mesure de l'expression de SIGLEC1, MMP8 et ISG15, et d'au moins cinq autres gènes cibles choisis parmi HERC6, SLC1A2, IL1R2, FAM20A, OLAH, et RETN.

Selon une autre variante de ce mode de réalisation particulier, l'étape de mesure comprend ou consiste en la mesure de l'expression d'une combinaison de huit gènes cibles choisie parmi les combinaisons suivantes :
SIGLEC1_ISG15HERC6_IL1R2_FAM20A_OLAH_RETN_MMP8 ;

SIGLEC1_ISG15_HERC6_SLC1A2_IL1R2_FAM20A_OLAH_MMP8;
SIGLEC1_ISG15_SLC1A2_IL1R2_FAM20A_OLAH_RETN_MMP8 ;
SIGLEC1_ISG15HERC6_SLC1A2_IL1R2_FAM20A_RETN_MMP8 ;
SIGLEC1_HERC6_SLC1A2_IL1R2_FAM20A_OLAH_RETN_MMP8 ;
SIGLEC1_ISG15_HERC6_SLC1A2_FAM20A_OLAH_MMP8 ;
SIGLEC1_ISG15_HERC6_SLC1A2_IL1R2_FAM20A_OLAH_RETN ;
ISG15_HERC6_SLC1A2_IL1R2_FAM20A_OLAH_RETN_MMP8, et
SIGLEC1_ISG15_HERC6_SLC1A2_IL1R2_OLAH_RETN_MMP8.

Selon une autre variante de ce mode de réalisation particulier, l'étape (a) comprend ou consiste en la mesure de l'expression d'une combinaison de huit gènes cibles choisie parmi celles listées dans le tableau 12, et de préférence, celles qui présentent une performance en termes d'aire sous la courbe ROC du modèle d'au moins 0,87, d'au moins 0,88, et tout particulièrement, d'au moins 0,89.

Selon un autre mode de réalisation particulier, la méthode comprend une étape de mesure de la variation de l'expression d'au moins neuf gènes cibles.

Selon une autre variante de ce mode de réalisation particulier, l'étape (a) comprend ou consiste en la mesure de l'expression d'une combinaison de neuf gènes cibles choisie parmi celles listées dans le tableau 13, et de préférence celles qui présentent une performance en termes d'aire sous la courbe ROC du modèle d'au moins 0,87, d'au moins 0,88, et tout particulièrement, d'au moins 0,89.

Selon un autre mode de réalisation particulier, la méthode comprend une étape de mesure de la variation de l'expression des dix gènes cibles.

Selon une autre variante de ce mode de réalisation particulier, l'étape (a) comprend ou consiste en la mesure de l'expression d'une combinaison de dix gènes cibles choisie parmi celles listées dans le tableau 14, et de préférence celles qui présentent une performance en termes d'aire sous la courbe ROC du modèle d'au moins 0,87, d'au moins 0,88, et tout particulièrement, d'au moins 0,89.

Selon un autre mode de réalisation particulier, la méthode comprend une étape de mesure de la variation de l'expression d'au moins onze gènes cibles.

Selon une autre variante de ce mode de réalisation particulier, l'étape (a) comprend ou consiste en la mesure de l'expression d'une combinaison de onze gènes cibles choisie parmi celles listées dans le tableau 15, et de préférence celles qui présentent une performance en termes d'aire sous la courbe ROC du modèle d'au moins 0,86, et tout particulièrement, d'au moins 0,87.

Selon un autre mode de réalisation particulier, la méthode comprend une étape de mesure de la variation de l'expression d'au moins douze gènes cibles.

Selon une autre variante de ce mode de réalisation particulier, l'étape (a) comprend ou consiste en la mesure de l'expression d'une combinaison de douze gènes cibles choisie parmi celles listées dans le tableau 16, et de préférence celles qui présentent une performance en termes d'aire sous la courbe ROC du modèle d'au moins 0,86, et tout particulièrement, d'au moins 0,87.

Selon un autre mode de réalisation particulier, la méthode comprend une étape de mesure de la variation de l'expression d'au moins treize gènes cibles.

Selon une autre variante de ce mode de réalisation particulier, l'étape (a) comprend ou consiste en la mesure de l'expression d'une combinaison de treize gènes cibles choisie parmi celles listées dans le tableau 17.

Par « échantillon biologique », on se réfère dans la présente description à tout échantillon provenant d'un sujet et pouvant être de différentes natures, comme le sang ou ses dérivés, les expectorations, l'urine, les selles, la peau, le liquide céphalo-rachidien, le liquide de lavage broncho-alvéolaire, le liquide de ponction de la cavité abdominale, la salive, les sécrétions gastriques, le sperme, le liquide séminal, les larmes, la moelle épinière, ganglion du nerf trijumeau, tissu adipeux, tissu lymphoïde, tissu placentaire, tissu du tractus gastro-intestinal, tissu du tractus génital, ou le tissu du système nerveux central.

L'expression « 'échantillon biologique de référence », ou «échantillon contrôle, fait référence à l'échantillon biologique à partir duquel les niveaux d'expressions des gènes cibles, et éventuellement ceux des gènes additionnels, dans l'échantillon biologique à tester sont comparés. L'échantillon biologique de référence est de même nature que l'échantillon biologique à tester ou tout du moins d'une nature compatible pour constituer une référence quant à la détermination du niveau d'expression des gènes cibles. Cet échantillon est issu de d'un sujet présentant une infection bactérienne ou virale, ou d'un pool d'échantillons biologiques tous issus de patients présentant une infection de même nature, à savoir virale ou bactérienne.

Selon un mode de réalisation particulier, l'échantillon biologique de référence est calibré pour contenir la quantité de transcrits d'au moins deux gènes cibles, et éventuellement d'un ou plusieurs gènes additionnels, correspondant à la quantité ou à la concentration représentative du niveau d'expression mesurée dans un pool d'échantillons de sujets présentant une infection bactérienne ou une infection virale. En d'autres termes, l'échantillon biologique de référence est calibré pour contenir la quantité moyenne des transcrits desdits gènes cibles obtenue à partir d'un pool d'échantillons de sujets ayant une infection bactérienne ou de sujets ayant une infection virale.

En particulier, l'échantillon biologique peut être un fluide biologique, comme un échantillon de sang ou un échantillon dérivé du sang, qui peut notamment être choisi parmi le sang total (tel que collecté par voie veineuse, c'est-à-dire contenant les cellules blanches et rouges, les plaquettes et le plasm(a), le plasma, le sérum, ainsi que tous les types de cellules extraites à partir du sang, comme par exemple les cellules mononuclées sanguines périphériques (ou PBMC, contenant les lymphocytes B, les lymphocytes T, les cellules NK, les cellules dendritiques et les monocytes), des sous-populations de lymphocytes B et T, des monocytes purifiés, ou encore des neutrophiles.

Selon un mode de réalisation préféré, l'échantillon biologique mis en œuvre dans la méthode selon l'invention est un échantillon de sang, de préférence un échantillon de sang total.

Selon un autre mode de réalisation particulier, le sujet est un patient au sein d'un hôpital, de préférence au sein du service des urgences, du service de réanimation, en unité de soins intensifs (USI) ou en unité de soins continus, et tout particulièrement, un patient au sein du service des urgences.

Selon un mode de réalisation préféré, le sujet est un patient âgé de moins de 6 ans, de préférence de moins de 4 ans, et de préférence encore, de moins de 2 ans. Selon ce mode de réalisation, le patient peut être au sein du service des urgences, et notamment en urgence pédiatrique.

Selon un mode de réalisation particulier, la méthode permet d'identifier la nature de l'infection avec une sensibilité d'au moins 80%, 85%, 90% ou encore d'au moins 95%. La sensibilité correspond à la probabilité d'identifier correctement la nature virale ou bactérienne de l'infection lorsque le sujet est infecté et peut être définie par la formule suivante :

$$Sensibilité = \frac{nombre\ de\ vrais\ positifs}{nombre\ de\ vrais\ positifs + nombre\ de\ faux\ négatifs}$$

Selon un mode de réalisation particulier, la méthode permet d'identifier la nature de l'infection avec une spécificité d'au moins 80%, 85%, 90% ou encore d'au moins 95%.

La spécificité correspond à la probabilité d'identifier de manière erronée la nature virale ou bactérienne de l'infection. En d'autres termes, d'identifier la présence d'une infection virale alors qu'il s'agit d'une infection bactérienne, ou inversement. La spécificité peut être définie par la formule suivante :

$$Spécificité = \frac{nombre\ de\ vrais\ négatifs}{nombre\ de\ vrais\ négatifs + nombre\ de\ faux\ positifs}$$

La sensibilité et la spécificité d'un test diagnostic, telle que la méthode selon la présente invention, peuvent également être résumées par l'intermédiaire d'une courbe ROC (de l'anglais « Receiver Operating Characteristics), en particulier de l'aire sous la courbe ROC, qui est bien connue de la personne du métier.

Ainsi, la méthode selon la présente description permet d'identifier la nature virale ou bactérienne d'une infection avec une performance reflétée par une aire sous la courbe ROC d'au moins 0,85, d'au moins 0,86, d'au moins 0,87, d'au moins 0,88, d'au moins 0,89, et tout particulièrement d'au moins 0,90 pour les signatures transcriptomiques les plus performantes.

Bien que les performances de la méthode selon l'invention soient tout à fait satisfaisantes, dans certaines situations, la mesure de la variation d'expression des gènes cibles peut être complétée par la mesure de la variation d'expression de gènes additionnels.

Ainsi, la méthode selon l'invention, dans tous ses modes de réalisations, peut en outre comprendre la mesure de la variation d'au moins un gène additionnel choisi parmi les gènes suivants : PI3, EBI3, ADGRE1 et S100P.

Ces gènes additionnels sont également connus de la personne du métier et les positions chromosomiques sont données dans le tableau 5 ci-dessous :

[Tableau 5] :

| Biomarqueur additionnel | Localisation chromosomique (GRCh38/hg38) | Nom anglais |
|---|---|---|
| PI3 | Chr20: 45174902-45176544 | Peptidase Inhibitor 3 |
| EBI3 | Chrl9: 4229523-4237528 | Epstein-Barr Virus Induced 3 |
| ADGRE1 | Chrl9: 6887566-6940459 | Adhesion G Protein-Coupled Receptor EI |

(suite)

| Biomarqueur additionnel | Localisation chromosomique (GRCh38/hg38) | Nom anglais |
|---|---|---|
| S100P | Chr4: 6693878-6697170 | S100 Calcium Binding Protein P |

De la même manière que précédemment, pour un gène additionnel donné, la valeur de référence correspond à un niveau d'expression de transcrits dudit gène obtenu à partir d'un échantillon biologique de référence issu d'un sujet présentant une infection. Avantageusement, et notamment pour les variantes définies ci-après, la valeur de référence pour un gène additionnel donné correspond à la moyenne du niveau de transcrits ARNm dudit gène obtenue à partir d'échantillons biologiques de références issus d'une population de sujets présentant une infection.

Selon une première variante, la valeur de référence de chaque gène additionnel est déterminée à partir d'un échantillon biologique de référence issu d'un sujet atteint d'une infection bactérienne. Ainsi, il pourra être conclu à la présence d'une infection de nature virale lorsque la comparaison du niveau d'expression des gènes additionnels au niveau des transcrits ARNm par rapport aux valeurs de référence respectives met en évidence au moins une variation d'expression choisi parmi :

- une sous expression de PI3,

- une sous expression de EBI3,

- une sous expression de ADGRE1, et

- une sous expression de S100P.

Selon une deuxième variante, la valeur de référence de chaque gène additionnel est déterminée à partir d'un échantillon biologique de référence issu de sujets atteints d'une infection virale. Ainsi, il pourra être conclu à la présence d'une infection de nature bactérienne lorsque la comparaison du niveau d'expression des gènes additionnels au niveau des transcrits ARNm par rapport aux valeurs de référence respectives met en évidence au moins une variation d'expression choisi parmi :

- une surexpression de PI3,

- une surexpression de EBI3,

- une surexpression de ADGRE1, et

- une surexpression de S100P.

Selon un mode de réalisation particulier, la méthode selon l'invention comprend la mesure de la variation de l'expression de l'ensemble des gènes cibles et des gènes additionnels suivants : SIGLEC1, ISG15, HERC6, SLC1A2, IL1R2, FAM20A, OLAH, RETN, MMP8, RSAD2, IFI27, OAS1, IFIT1, IFI44L, PI3, EBI3, ADGRE1 et S100P.

La conclusion quant à la nature virale ou bactérienne de l'infection est faite telle que définie précédemment sur la base de la surexpression ou de la sous expression desdits gènes en fonction des valeurs de références déterminées.

Ainsi, selon un mode de réalisation particulier, la méthode comprend les étapes :

- obtention d'un échantillon biologique de sang, de préférence de sang total, d'un sujet présentant une infection,

- mise en contact dudit échantillon biologique avec des réactifs spécifiques des produits d'expression d'un ou plusieurs gènes viraux selon la présente description, et d'au moins un gène cible bactérien choisi parmi OLAH, FAM20A, IL1R2, MMP8, RETN et SLC1A2, et éventuellement d'un ou plusieurs gènes additionnels choisis parmi PI3, EBI3, ADGRE1 et S100P,

- mesure de l'expression desdits gènes cibles, et éventuellement du ou des gènes additionnels.

Selon un mode de réalisation préféré, la méthode comprend les étapes :

- obtention d'un échantillon biologique de sang, de préférence de sang total, d'un sujet présentant une infection,

- mise en contact dudit échantillon biologique avec des réactifs spécifiques des produits d'expression d'au moins un gène cible viral choisi parmi SIGLEC1, ISG15, HERC6, RSAD2, IFI27, OAS1, IFIT1, IFI44L, et d'au moins un gène bactérien choisi parmi SLC1A2, IL1R2, FAM20A, OLAH, RETN et MMP8, et éventuellement d'un ou plusieurs gènes additionnels choisis parmi, PI3, EBI3, ADGRE1 et S100P,

- mesure de l'expression desdits gènes cibles, et éventuellement du ou des gènes additionnels.

Un autre objet de l'invention concerne un kit de mesure *in* vitro ou *ex vivo* de l'expression d'au moins un gène cible viral choisi parmi SIGLEC1, ISG15, HERC6, RSAD2, IFI27, OAS1, IFIT1, IFI44L, et d'au moins un gène cible bactérien choisi parmi OLAH, FAM20A, IL1R2, MMP8, RETN et SLC1A2, ledit kit comprenant des réactifs spécifiques des produits d'expression desdits gènes cibles.

Selon un mode de réalisation particulier, le kit comprend des réactifs spécifiques des produits d'expression d'au moins trois gènes cibles, quatre gènes cibles, cinq gènes cibles, six gènes cibles, sept gènes cibles, huit gènes cibles ou de l'ensemble des gènes cibles.

Tout particulièrement, le kit permet la mise en œuvre de la méthode selon l'invention. Le kit selon l'invention peut donc être avantageusement utilisé pour la détermination de nature virale ou bactérienne d'une infection.

Par conséquent, tous les modes de réalisations particuliers et préférés décrits pour la méthode selon l'invention, notamment sur les combinaisons de gènes cibles dont l'expression est mesurée, s'appliquent également pour le kit.

Les réactifs spécifiques permettant de mesure l'expression des gènes sont connus de la personne du métier et selon un mode de réalisation particulier, il s'agit des amorces d'amplification et/ou des sondes d'hybridation.

De tels réactifs permettent de déterminer quantitativement le niveau de transcrits dudit gène sélectionné dans l'échantillon biologique test. Comme expliqué précédemment, le niveau de transcrits déterminé peut ne pas correspondre directement à la quantité de transcrits présents dans l'échantillon biologique, mais être une valeur dérivée représentative de la quantité de transcrits. En effet, de manière classique, la détermination quantitative peut inclure une étape d'amplification et/ou de normalisation et/ou de calcul d'un rapport...

Par kit, on entend un ensemble de produits et/ou outils à utiliser ensemble pour obtenir, en particulier, la détermination du niveau de transcrits des gènes cibles définis dans le cadre de l'invention. Les réactifs nécessaires peuvent ou non être rassemblés au sein d'un même kit ou d'un même dispositif.

On entend par « amorce d'amplification » ou « amorce », un fragment nucléotidique pouvant être constitué de 5 à 100 nucléotides, de préférence de 15 à 30 nucléotides, et possédant une spécificité d'hybridation avec une séquence nucléotidique cible, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplification de plusieurs biomarqueurs (e.g des gènes ou des ARNm desdits gènes) différents, plusieurs couples d'amorces différents sont de préférence utilisés, ayant préférentiellement chacun une capacité à s'hybrider spécifiquement avec un biomarqueur différent.

La personne du métier est tout à fait en mesure à partir de la séquence d'un gène, et notamment de la séquence des transcrits correspondants, par exemple ceux listés dans le tableau 2, de déterminer les séquences nucléotidiques des amorces afin de permettre une amplification des transcrits.

Selon un mode de réalisation particulier, des amorces sont présentes dans le kit, lesdites amorces comprenant, ou consistant en, une séquence nucléotidique complémentaire d'au moins une partie d'une séquence des transcrits tels que présentés dans le tableau 2 précédemment.

On entend par « sonde » ou « sonde d'hybridation », un fragment nucléotidique constitué typiquement de 5 à 100 nucléotides, de préférence de 15 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, la séquence nucléotidique cible peut être une séquence nucléotidique comprise dans un ARN messager (ARNm) ou une séquence nucléotidique comprise dans un ADN complémentaire (ADNc) obtenu par transcription inverse dudit ARNm. Lorsque l'on souhaite cibler plusieurs biomarqueurs (e.g. des gènes ou des ARNm desdits gènes) différents, plusieurs sondes différentes sont de préférence utilisées, ayant préférentiellement chacune une capacité à s'hybrider spécifiquement avec un biomarqueur différent.

Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques.

Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence.

La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par la personne du métier.

En général, selon la longueur des sondes d'hybridation utilisés, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Selon un mode de réalisation préféré, le kit comprend un échantillon contrôle calibré pour contenir la quantité de transcrits d'au moins deux gènes cibles, et éventuellement d'un ou plusieurs gènes additionnels, correspondant à la quantité ou à la concentration représentative du niveau d'expression mesurée dans un pool d'échantillons de sujets présentant une infection bactérienne et/ou un échantillon contrôle calibré pour contenir la quantité de transcrits d'au moins deux gènes cibles, et éventuellement d'un ou plusieurs gènes additionnels, correspondant à la quantité ou à la concentration représentative du niveau d'expression mesurée dans un pool d'échantillons de sujets présentant une infection virale.

En d'autres termes, l'échantillon contrôle est calibré pour contenir la quantité moyenne des transcrits desdits gènes cibles obtenue à partir d'un pool d'échantillons de sujets ayant une infection bactérienne ou de sujets ayant une infection virale.

Selon un mode de réalisation, les réactifs spécifiques de l'expression des gènes cibles, plus précisément, des produits d'amplification et/ou produits de détection, tels que par exemple des amorces ou des sondes, peuvent être liés à un même support solide. Le kit peut donc comprendre un support solide comprenant un ou plusieurs oligonucléotide(s) adapté(s) à la détermination du niveau de transcrits du ou de chaque gène cible, voire un ou plusieurs oligonucléotide(s) adapté(s) à la détermination du niveau de transcrits du gène ou de chaque gène de ménage sélectionné, voire un ou plusieurs oligonucléotide(s) adapté(s) à la détection d'au moins un gène cible, comme précédemment décrit. De tels supports solides sont bien connus de la personne du métier, et notamment décrits dans les demandes WO2008/140568 et WO2017/093672 auxquelles on pourra se référer pour plus de détails.

Le kit, dans tous ses modes de réalisation, peut également comprendre en outre des réactifs spécifiques des produits d'expression d'un ou plusieurs gènes additionnels choisis parmi PI3, EBI3, ADGRE1 et S100P et leurs combinaisons.

Le kit peut également comprendre, de la même manière que définie précédemment, une valeur du niveau d'expression des gènes additionnels et/ou des échantillons contrôles (positifs ou négatifs) desdits gènes additionnels.

Le kit, dans tous ses modes de réalisation, peut également comprendre en outre des réactifs spécifiques des produits d'expression d'un ou plusieurs gènes de ménage. Là encore, le kit selon l'invention peut comprendre des réactifs spécifiques des produits d'expression de chaque gène de ménage sélectionné.

Selon un mode de réalisation particulier, le kit se présente sous la forme d'un consommable intégrant la purification des acides nucléiques, la PCR nichée multiplexe et la détection en un seul consommable sous la forme d'une cassette ou poche FilmArray®. Un tel consommable est avantageusement destiné à être utilisé avec les systèmes BioFire® FilmArray® V2.0 et Torch.

Un autre objet concerne l'utilisation d'un kit tel que défini précédemment pour déterminer la nature virale ou bactérienne d'une infection chez un sujet.

Un autre objet de la présente description concerne une méthode comprenant la mesure quantitative, notamment par RT-qPCR, des ARNm d'au moins un gène cible viral choisi parmi IFI27, SIGLEC1, ISG15, HERC6, RSAD2, OAS1, IFIT1, IFI44L, et d'au moins un gène cible bactérien choisi parmi OLAH, FAM20A, IL1R2, MMP8, RETN et SLC1A2, et éventuellement d'un ou plusieurs gènes additionnels tels que définis précédemment, dans un échantillon biologique de sang d'un sujet présentant une infection.

La description concerne également les méthodes de détermination de la nature virale ou bactérienne d'une infection chez un sujet infecté telles que définies précédemment, qui comprennent en outre une étape de traitement de l'infection virale ou bactérienne.

Selon un mode de réalisation particulier, lorsqu'une infection virale est identifiée, le traitement comprend l'administration d'au moins un agent antiviral. La personne du métier est en mesure de choisir l'agent antiviral le plus adapté parmi les agents connus. A titre d'exemple, l'agent antiviral peut être choisi parmi les agents suivants : amantadine, rimantadine, ritonavir, cobicistat, interferon alfa-2b/ribavirin, ombitasvir/paritaprevir/ritonavir, peginterferon alfa-2a, peginterferon alfa-2b, maraviroc, raltegravir, dolutegravir, elvitegravir, sofosbuvir, enfuvirtide, foscarnet, fomivirsen, zanamivir, oseltamivir, peramivir, nevirapine, etravirine, efavirenz, rilpivirine, delavirdine, nevirapine, daclatasvir, entacavir, lamivudine, adefovir, didanosine, tenofovir, abacavir, lamivudine, zidovudine, stavudine, emtricitabine, zalcitabine, telbivudine, didanosine, boceprevir, simeprevir, telaprevir, lopinavir, fosamprenavir, darunavir, ritonavir, tipranavir, atazanavir, nelfinavir, amprenavir, indinavir, saquinavir, ribavirin, valacyclovir, famciclovir, acyclovir, ganciclovir, valganciclovir et cidofovir.

Selon un mode de réalisation particulier, lorsqu'une infection virale est identifiée, le traitement comprend l'administration d'au moins un antibiotique. La personne du métier est en mesure de choisir l'antibiotique le plus adapté parmi les antibiotiques connus. A titre d'exemple, l'antibiotique peut être choisi parmi les antibiotiques suivants : erythromycine, clindamucine, gentamicine, tetracycline, l'amoxicilline, amikacine, aztréonam, chloramphénicol, ceftazidime, clindamycine, céfalotine, ciprofloxacine, ,colistine, céfotétan, céfotaxime, érythromycine, acide fusidique, fosfomycine, céfoxitine, furanes, gentamicine, imipénème, kanamycine, lincomycine, céfamandole, minocycline, latamoxef, métronidazole, acide nalidixique, nétilmicine, oxacilline, benzylpénicilline, péfloxacine, pipéracilline, pristinamycine, rifampicine, spiramycine, sulfamides, streptomycine, triméthoprime, sulfamétoxazole, tétracycline, téicoplanine, ticarcilline, tobramycine, triméthoprime, vancomycine, meclocycline, sulfacetamide, ceftobiprole, ceftaroline, dalbavancine, daptomycine, linezolide, mupirocine, oritavancine, telavancine, tigecycline, vancomycine, aminoglycosides, carbapenemes, ceftazidime, cefepime, ceftobiprole, fluorquinolones, piperacilline/tazobactame, ticarcilline/acide clavulanic, linezolide, streptogramines, daptomycine, amikacine, kanamycine, neomycine, netilmicine, tobramycine, paromomycine, , spectinomycine, geldanamycine, herbimycine et rifaximine.

La présente invention est illustrée de manière non limitative à partir des exemples suivants.

## EXEMPLES

### 1. Matériel et méthode

### 1.1 Caractéristiques cliniques des patients & échantillons

Des échantillons cliniques de sang total ont été collectés dans des tubes PAXgène® lors de l'admission du patient au sein service des urgences (J0) en suivant les recommandations du fabriquant. Au total, 191 échantillons ont été collectés avec la répartition suivantes : 68 échantillons issus d'une infection bactérienne caractérisée et 123 échantillons issus d'une infection virale caractérisée.

L'origine et la répartition des échantillons sont résumées ci-dessous :

Patients : Femme - 82 (42,9%) ; Homme 109(57,1%)

Age moyen : 3,4 ans

Infection bactérienne (GRAM-), n(%) : 28 (14,6)

Infection bactérienne (GRAM+), n(%) : 24 (12,6)

Infection bactérienne probable, n(%) : 16 (8,4)

Infection virale, n(%) : 123 (64,4)

### 1.2. Identification et validation des biomarqueurs

Une première sélection de biomarqueurs transcriptomiques caractéristiques des infections virales et bactériennes a été effectuée par l'intermédiaire de la base de données interne de la Demanderesse. Plusieurs centaines de biomarqueurs ont ainsi été identifiés.

Pour chacun d'entre eux, plusieurs scores ont alors été attribués afin d'évaluer une multitudes de critères, à savoir notamment la capacité d'expression à partir des données de RNAseq issus de banque d'échantillons, la capacité de discrimination entre une infection virale et bactérienne à partir de données de micro-array issues des bases de données publiques, ou encore la possibilité de mettre au point des paires d'amorces pour l'amplification dans un système de PCR automatisé (FilmArray® par exemple).

En tenant compte des différents scores obtenus pour chaque biomarqueur, un score global a été calculé afin de permettre une sélection optimale et l'identification des biomarqueurs les plus prometteurs pour l'identification de la nature de l'infection.

Ensuite, pour finalement valider les biomarqueurs identifiés, les échantillons des patients ont été divisés en deux groupes de données. Le premier groupe, dit set « TRAIN » (n=128), permet d'entrainer le modèle d'apprentissage sur les données, tandis que le second groupe, dit set « TEST » (n=63), est utilisé pour la validation indépendante des performances.

Pour évaluer les performances individuelles des biomarqueurs, l'aire sous la courbe ROC a été calculée avec un indice de confiance à 95% (IC95%). Concernant les performances des combinaisons, des analyses ont été effectuées à l'aide de classificateurs complexes basés sur l'apprentissage automatique en utilisant la version 3.6.1 de R.

Deux méthodes ont particulièrement été utilisées pour évaluer l'importance des variables. La première est la fonction "explain" du package FastShap qui est dérivée de la théorie des jeux. Une récompense (poids) est donnée aux biomarqueurs qui apportent le plus dans le modèle d'apprentissage automatique pour classer les patients et permettre l'identification de la nature de l'infection (virale ou bactérienne).

La deuxième méthode est la fonction "FeatureImp" du paquet IML qui renvoie le facteur par lequel l'erreur de prédiction du modèle augmente lorsqu'une caractéristique est mélangée.

En définitive, la sélection a permis d'identifier des biomarqueurs viraux, et notamment des biomarqueurs impliqués dans la voie de l'interféron et de la présentation antigénique médiée par le CMH de classe II, mais également des biomarqueurs bactériens, notamment les biomarqueurs SLC1A2, IL1R2, FAM20A, OLAH, RETN et MMP8.

Les performances ont également été évaluées et confirmées sur deux autres modèles d'apprentissage automatique, à savoir le Random Forest et la régression « Partial Least Squares-Discriminant » (PLS).

## 2. Résultats

### 2.1 BoxPlot

L'analyse des BoxPlot révèle les surexpression/sous-expression des différents gènes cibles selon la nature de l'infection. Ainsi, lorsque l'on compare les expressions des gènes cibles entre les infections virales et bactérienne, on constate que les gènes SLC1A2, IL1R2, FAM20A, OLAH, RETN et MMP sont surexprimés lors d'une infection bactérienne alors que les gènes SIGLEC1, ISG15, HERC6, IFI44L, RSAD2, IFI27, OAS1 et IFIT1 sont surexprimés lors d'une infection virale.

### 2.2 Analyse des performances des biomarqueurs selon l'invention

Les performances des gènes cibles de la méthode selon l'invention pour déterminer la nature virale ou bactérienne d'une infection sont présentées dans les tableaux ci-dessous. Pour chaque modèle, les performances ont été évaluée plusieurs fois et les AUC du modèle correspondent donc à la moyenne des différentes AUC obtenues.

Performances obtenues pour l'identification de la nature virale ou bactérienne de l'infection sur la base des combinaisons à 2 gènes :

[Tableau 6]

| Modèle | AUC (modèle) | IC 95% | | AUC (Train) | IC 95% (Train) | | AUC (Test) | IC 95% (Test) | |
|---|---|---|---|---|---|---|---|---|---|
| IFI27_OLAH | 0,910 | 0,902 | 0,918 | 0,916 | 0,859 | 0,974 | 0,937 | 0,876 | 0,997 |
| IFI27_IL1R2 | 0,903 | 0,896 | 0,910 | 0,913 | 0,864 | 0,962 | 0,910 | 0,837 | 0,982 |
| IFI27_RETN | 0,892 | 0,882 | 0,901 | 0,900 | 0,836 | 0,963 | 0,933 | 0,858 | 1 |
| SIGLEC1_IL1R2 | 0,890 | 0,882 | 0,898 | 0,897 | 0,835 | 0,959 | 0,960 | 0,920 | 1 |
| SIGLEC1_FAM20A | 0,888 | 0,878 | 0,897 | 0,890 | 0,817 | 0,963 | 0,955 | 0,893 | 1 |
| HERC6_IL1R2 | 0,885 | 0,877 | 0,893 | 0,895 | 0,836 | 0,954 | 0,927 | 0,865 | 0,990 |
| SIGLEC1_OLAH | 0,882 | 0,873 | 0,890 | 0,890 | 0,821 | 0,959 | 0,949 | 0,900 | 0,998 |
| IFIT1_IL1R2 | 0,880 | 0,871 | 0,888 | 0,890 | 0,824 | 0,956 | 0,953 | 0,909 | 0,998 |
| IFI27_FAM20A | 0,878 | 0,867 | 0,888 | 0,878 | 0,800 | 0,957 | 0,923 | 0,839 | 1 |
| HERC6_OLAH | 0,876 | 0,867 | 0,885 | 0,886 | 0,819 | 0,953 | 0,941 | 0,889 | 0,993 |
| IFI44L_IL1R2 | 0,876 | 0,868 | 0,884 | 0,886 | 0,825 | 0,947 | 0,941 | 0,889 | 0,993 |
| OAS1_IL1R2 | 0,875 | 0,866 | 0,883 | 0,886 | 0,821 | 0,952 | 0,926 | 0,863 | 0,989 |
| ISG15_IL1R2 | 0,874 | 0,866 | 0,882 | 0,885 | 0,824 | 0,945 | 0,933 | 0,865 | 1 |
| RSAD2_IL1R2 | 0,872 | 0,863 | 0,880 | 0,881 | 0,814 | 0,948 | 0,943 | 0,893 | 0,994 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| OAS1_OLAH | 0,870 | 0,861 | 0,879 | 0,880 | 0,808 | 0,952 | 0,946 | 0,892 | 0,999 |
| SIGLEC1_RETN | 0,869 | 0,859 | 0,879 | 0,875 | 0,797 | 0,953 | 0,948 | 0,898 | 0,997 |
| IFI27_MMP8 | 0,867 | 0,857 | 0,877 | 0,877 | 0,809 | 0,945 | 0,882 | 0,793 | 0,970 |
| HERC6_FAM20A | 0,866 | 0,856 | 0,876 | 0,874 | 0,802 | 0,945 | 0,925 | 0,838 | 1 |
| IFI27_SIGLEC1 | 0,865 | 0,855 | 0,875 | 0,877 | 0,810 | 0,943 | 0,949 | 0,898 | 0,999 |
| IFI44L_OLAH | 0,865 | 0,856 | 0,874 | 0,871 | 0,798 | 0,943 | 0,938 | 0,883 | 0,993 |
| RSAD2_OLAH | 0,865 | 0,855 | 0,874 | 0,875 | 0,802 | 0,947 | 0,950 | 0,901 | 0,999 |
| SLC1A2_OLAH | 0,865 | 0,855 | 0,874 | 0,880 | 0,817 | 0,942 | 0,845 | 0,737 | 0,952 |
| IFIT1_OLAH | 0,864 | 0,854 | 0,874 | 0,873 | 0,798 | 0,947 | 0,949 | 0,901 | 0,997 |
| HERC6_RETN | 0,864 | 0,853 | 0,874 | 0,872 | 0,795 | 0,950 | 0,928 | 0,867 | 0,990 |
| SIGLEC1_SLC1A2 | 0,864 | 0,855 | 0,872 | 0,877 | 0,811 | 0,944 | 0,957 | 0,914 | 0,999 |
| ISG15_OLAH | 0,863 | 0,854 | 0,872 | 0,873 | 0,805 | 0,941 | 0,940 | 0,886 | 0,994 |
| SIGLEC1_MMP8 | 0,858 | 0,848 | 0,868 | 0,863 | 0,786 | 0,940 | 0,926 | 0,866 | 0,987 |
| IFI27_SLC1A2 | 0,857 | 0,847 | 0,867 | 0,863 | 0,785 | 0,940 | 0,895 | 0,808 | 0,981 |
| HERC6_MMP8 | 0,851 | 0,841 | 0,862 | 0,859 | 0,784 | 0,935 | 0,877 | 0,791 | 0,964 |
| IL1R2_OLAH | 0,851 | 0,842 | 0,860 | 0,862 | 0,796 | 0,928 | 0,843 | 0,733 | 0,954 |
| IFI44L_FAM20A | 0,850 | 0,840 | 0,860 | 0,853 | 0,771 | 0,935 | 0,934 | 0,855 | 1 |
| IL1R2_FAM20A | 0,847 | 0,839 | 0,856 | 0,863 | 0,797 | 0,929 | 0,849 | 0,736 | 0,962 |
| SIGLEC1_HERC6 | 0,847 | 0,837 | 0,857 | 0,854 | 0,778 | 0,930 | 0,946 | 0,896 | 0,995 |
| HERC6_SLC1A2 | 0,842 | 0,833 | 0,851 | 0,857 | 0,787 | 0,926 | 0,902 | 0,820 | 0,984 |
| SIGLEC1_ISG15 | 0,842 | 0,832 | 0,852 | 0,849 | 0,772 | 0,927 | 0,945 | 0,895 | 0,994 |
| FAM20A_OLAH | 0,839 | 0,829 | 0,849 | 0,852 | 0,782 | 0,922 | 0,871 | 0,776 | 0,965 |
| ISG15_FAM20A | 0,838 | 0,828 | 0,848 | 0,846 | 0,769 | 0,923 | 0,924 | 0,844 | 1 |
| SLC1A2_IL1R2 | 0,838 | 0,829 | 0,846 | 0,852 | 0,785 | 0,918 | 0,826 | 0,709 | 0,943 |
| ISG15_RETN | 0,836 | 0,827 | 0,846 | 0,851 | 0,777 | 0,926 | 0,949 | 0,896 | 1 |
| OLAH_MMP8 | 0,836 | 0,826 | 0,845 | 0,847 | 0,778 | 0,916 | 0,861 | 0,761 | 0,960 |
| OLAH_RETN | 0,834 | 0,824 | 0,844 | 0,850 | 0,780 | 0,921 | 0,848 | 0,738 | 0,958 |
| ISG15_HERC6 | 0,829 | 0,819 | 0,840 | 0,839 | 0,763 | 0,914 | 0,921 | 0,856 | 0,986 |
| IL1R2_MMP8 | 0,828 | 0,820 | 0,836 | 0,849 | 0,781 | 0,916 | 0,812 | 0,696 | 0,928 |
| IL1R2_RETN | 0,828 | 0,819 | 0,837 | 0,850 | 0,782 | 0,918 | 0,820 | 0,705 | 0,935 |
| ISG15_MMP8 | 0,819 | 0,810 | 0,829 | 0,832 | 0,754 | 0,910 | 0,898 | 0,823 | 0,972 |
| ISG15_SLC1A2_ | 0,803 | 0,793 | 0,813 | 0,820 | 0,744 | 0,895 | 0,911 | 0,839 | 0,983 |

Performances obtenues pour l'identification de la nature virale ou bactérienne de l'infection sur la base des combinaisons à trois gènes :

[Tableau 7]

| Modèle | AUC (modèle) | IC 95% | | AUC (Train) | IC 95% (Train) | | AUC (Test) | IC 95% (Test) | |
|---|---|---|---|---|---|---|---|---|---|
| IFI27_OLAH_FAM20A | 0,912 | 0,903 | 0,920 | 0,922 | 0,862 | 0,981 | 0,929 | 0,857 | 1 |
| IFI27_IL1R2_FAM20A | 0,908 | 0,901 | 0,916 | 0,920 | 0,867 | 0,972 | 0,910 | 0,819 | 1 |
| IFI27 _OLAH_MMP8 | 0,907 | 0,899 | 0,915 | 0,916 | 0,859 | 0,974 | 0,937 | 0,876 | 0,997 |
| IFI27_HERC6_OLAH | 0,907 | 0,899 | 0,915 | 0,919 | 0,862 | 0,975 | 0,942 | 0,888 | 0,997 |
| IFI27_IL1R2_OLAH | 0,907 | 0,899 | 0,914 | 0,921 | 0,868 | 0,974 | 0,936 | 0,875 | 0,996 |
| IFI27_ISG15_OLAH | 0,906 | 0,899 | 0,914 | 0,916 | 0,859 | 0,973 | 0,934 | 0,871 | 0,996 |
| IFI27_OLAH_RETN | 0,905 | 0,897 | 0,913 | 0,916 | 0,857 | 0,974 | 0,940 | 0,876 | 1 |
| IFI27_SLC1A2_OLAH | 0,905 | 0,897 | 0,913 | 0,926 | 0,874 | 0,978 | 0,929 | 0,862 | 0,997 |
| IFI27_SIGLEC1_OLAH | 0,905 | 0,897 | 0,913 | 0,917 | 0,859 | 0,975 | 0,945 | 0,890 | 0,999 |
| IFI27_SIGLEC1_IL1R2 | 0,905 | 0,898 | 0,912 | 0,920 | 0,871 | 0,969 | 0,951 | 0,905 | 0,998 |
| IFI27_IFI44L_OLAH | 0,905 | 0,896 | 0,913 | 0,916 | 0,859 | 0,974 | 0,937 | 0,876 | 0,997 |
| IFI27_IFIT1_OLAH | 0,904 | 0,896 | 0,912 | 0,916 | 0,858 | 0,974 | 0,939 | 0,881 | 0,998 |
| IFI27_OAS1_OLAH | 0,903 | 0,896 | 0,911 | 0,916 | 0,858 | 0,974 | 0,938 | 0,879 | 0,997 |
| IFI27_HERC6_IL1R2 | 0,903 | 0,896 | 0,910 | 0,918 | 0,870 | 0,966 | 0,929 | 0,870 | 0,988 |
| RSAD2_IFI27_OLAH | 0,903 | 0,895 | 0,911 | 0,916 | 0,858 | 0,974 | 0,939 | 0,881 | 0,998 |
| IFI27_IL1R2_RETN | 0,902 | 0,895 | 0,910 | 0,921 | 0,871 | 0,971 | 0,924 | 0,852 | 0,995 |
| IFI27_IFI44L_IL1R2 | 0,901 | 0,894 | 0,908 | 0,913 | 0,863 | 0,963 | 0,932 | 0,872 | 0,991 |
| IFI27_IFIT1_IL1R2 | 0,901 | 0,893 | 0,908 | 0,915 | 0,864 | 0,966 | 0,940 | 0,887 | 0,994 |
| IFI27_ISG15_IL1R2 | 0,900 | 0,893 | 0,906 | 0,914 | 0,864 | 0,963 | 0,929 | 0,868 | 0,991 |
| RSAD2_IFI27_IL1R2 | 0,900 | 0,892 | 0,907 | 0,916 | 0,866 | 0,966 | 0,935 | 0,878 | 0,992 |
| IFI27_OAS1_IL1R2 | 0,899 | 0,892 | 0,906 | 0,914 | 0,864 | 0,965 | 0,932 | 0,873 | 0,990 |
| IFI27_ILIR2_MMP8 | 0,898 | 0,891 | 0,905 | 0,913 | 0,863 | 0,962 | 0,914 | 0,844 | 0,984 |
| IFI27_SIGLEC1_FAM20A | 0,896 | 0,887 | 0,905 | 0,905 | 0,837 | 0,974 | 0,950 | 0,874 | 1 |
| SIGLEC1_IL1R2_FAM20A | 0,896 | 0,888 | 0,905 | 0,909 | 0,846 | 0,973 | 0,963 | 0,912 | 1 |
| IFI27_SLC1A2_IL1R2 | 0,895 | 0,888 | 0,903 | 0,917 | 0,871 | 0,963 | 0,903 | 0,819 | 0,988 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| IFI27_HERC6_RETN | 0,895 | 0,885 | 0,904 | 0,908 | 0,845 | 0,971 | 0,951 | 0,893 | 1 |
| IFI27_FAM20A_RETN | 0,894 | 0,884 | 0,904 | 0,906 | 0,844 | 0,968 | 0,923 | 0,832 | 1 |
| HERC6_IL1R2_FAM20A | 0,892 | 0,883 | 0,900 | 0,907 | 0,847 | 0,966 | 0,922 | 0,833 | 1 |
| SIGLEC1_SLC1A2_OLAH | 0,892 | 0,883 | 0,900 | 0,912 | 0,857 | 0,968 | 0,951 | 0,904 | 0,998 |
| IFI27_SIGLEC1_RETN | 0,891 | 0,882 | 0,901 | 0,904 | 0,840 | 0,969 | 0,962 | 0,912 | 1 |
| IFIT1_SIGLEC1_FAM20A | 0,890 | 0,881 | 0,899 | 0,897 | 0,826 | 0,968 | 0,939 | 0,873 | 1 |
| IFI27_IFI44L_RETN | 0,890 | 0,880 | 0,899 | 0,906 | 0,842 | 0,970 | 0,955 | 0,901 | 1 |
| SIGLEC1_HERC6_IL1R2 | 0,889 | 0,881 | 0,897 | 0,902 | 0,841 | 0,962 | 0,953 | 0,909 | 0,998 |
| SIGLEC1_OLAH_FAM20A | 0,889 | 0,879 | 0,898 | 0,897 | 0,825 | 0,969 | 0,962 | 0,918 | 1 |
| IFI27_HERC6_FAM20A | 0,887 | 0,878 | 0,897 | 0,902 | 0,839 | 0,965 | 0,934 | 0,851 | 1 |
| IFI27_ISG15_RETN | 0,887 | 0,878 | 0,897 | 0,903 | 0,841 | 0,966 | 0,947 | 0,883 | 1 |
| SIGLEC1_IL1R2_MMP8 | 0,887 | 0,879 | 0,895 | 0,901 | 0,842 | 0,960 | 0,962 | 0,923 | 1 |
| IFI27_IFIT1_RETN | 0,887 | 0,877 | 0,896 | 0,904 | 0,840 | 0,968 | 0,952 | 0,892 | 1 |
| IFI44L_SLC1A2_OLAH | 0,887 | 0,878 | 0,895 | 0,905 | 0,848 | 0,963 | 0,930 | 0,873 | 0,988 |
| IFI44L_SIGLEC1_IL1R2 | 0,886 | 0,878 | 0,894 | 0,898 | 0,836 | 0,960 | 0,958 | 0,916 | 0,999 |
| SIGLEC1_ISG15_IL1R2 | 0,886 | 0,878 | 0,894 | 0,899 | 0,837 | 0,961 | 0,962 | 0,923 | 1 |
| SIGLEC1_SLC1A2_IL1R2 | 0,886 | 0,878 | 0,894 | 0,908 | 0,853 | 0,964 | 0,964 | 0,927 | 1 |
| SIGLEC1_IL1R2_OLAH | 0,885 | 0,877 | 0,894 | 0,901 | 0,840 | 0,963 | 0,960 | 0,918 | 1 |
| SIGLEC1_SLC1A2_FAM20A | 0,885 | 0,876 | 0,894 | 0,902 | 0,839 | 0,966 | 0,949 | 0,877 | 1 |
| RSAD2_SIGLEC1_IL1R2 | 0,885 | 0,877 | 0,893 | 0,897 | 0,833 | 0,961 | 0,961 | 0,922 | 1,000 |
| IFIT1_SIGLEC1_IL1R2 | 0,885 | 0,876 | 0,893 | 0,898 | 0,836 | 0,960 | 0,958 | 0,916 | 0,999 |
| OAS1_SIGLEC1_IL1R2 | 0,884 | 0,876 | 0,893 | 0,898 | 0,835 | 0,961 | 0,959 | 0,918 | 0,999 |
| HERC6_OLAH_FAM20A | 0,884 | 0,875 | 0,894 | 0,898 | 0,833 | 0,963 | 0,942 | 0,875 | 1 |
| SIGLEC1_FAM20A_RETN | 0,884 | 0,875 | 0,894 | 0,893 | 0,821 | 0,966 | 0,954 | 0,895 | 1 |
| OAS1_IL1R2_FAM20A | 0,884 | 0,876 | 0,893 | 0,898 | 0,831 | 0,964 | 0,935 | 0,856 | 1 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| SIGLEC1_HERC6_FAM20A | 0,884 | 0,874 | 0,894 | 0,893 | 0,821 | 0,965 | 0,952 | 0,879 | 1 |
| IFI44L_SIGLEC1_FAM20A | 0,884 | 0,874 | 0,893 | 0,893 | 0,822 | 0,965 | 0,958 | 0,898 | 1 |
| IFI44L_IL1R2_FAM20A | 0,884 | 0,875 | 0,892 | 0,896 | 0,832 | 0,960 | 0,943 | 0,874 | 1 |
| IFI27_OAS1_RETN | 0,884 | 0,874 | 0,893 | 0,903 | 0,839 | 0,966 | 0,948 | 0,889 | 1 |
| RSAD2_IFI27_RETN | 0,883 | 0,874 | 0,893 | 0,901 | 0,837 | 0,966 | 0,952 | 0,894 | 1 |
| SIGLEC1_FAM20A_MMP8 | 0,883 | 0,873 | 0,893 | 0,890 | 0,817 | 0,964 | 0,959 | 0,897 | 1 |
| SIGLEC1_IL1R2_RETN | 0,883 | 0,874 | 0,891 | 0,896 | 0,832 | 0,961 | 0,960 | 0,918 | 1 |
| ISG15_IL1R2_FAM20A | 0,882 | 0,874 | 0,891 | 0,900 | 0,838 | 0,961 | 0,926 | 0,844 | 1 |
| SIGLEC1_ISG15_FAM20A | 0,882 | 0,873 | 0,892 | 0,890 | 0,816 | 0,963 | 0,955 | 0,893 | 1 |
| RSAD2_SIGLEC1_FAM20A | 0,882 | 0,873 | 0,892 | 0,895 | 0,824 | 0,965 | 0,952 | 0,890 | 1 |
| IFIT1_HERC6_IL1R2 | 0,882 | 0,874 | 0,890 | 0,895 | 0,833 | 0,958 | 0,942 | 0,891 | 0,994 |
| IFI27_IFI44L_FAM20A | 0,882 | 0,872 | 0,891 | 0,895 | 0,825 | 0,964 | 0,939 | 0,860 | 1 |
| ISG15_HERC6_IL1R2 | 0,882 | 0,874 | 0,890 | 0,895 | 0,836 | 0,954 | 0,941 | 0,888 | 0,995 |
| OAS1_SIGLEC1_FAM20A | 0,882 | 0,872 | 0,891 | 0,892 | 0,820 | 0,964 | 0,955 | 0,895 | 1 |
| IFIT1_SLC1A2_OLAH | 0,881 | 0,872 | 0,890 | 0,903 | 0,844 | 0,962 | 0,943 | 0,893 | 0,994 |
| IFIT1_IL1R2_FAM20A | 0,881 | 0,872 | 0,890 | 0,899 | 0,835 | 0,964 | 0,950 | 0,884 | 1 |
| IF127_RETN_MMP8 | 0,881 | 0,871 | 0,891 | 0,899 | 0,836 | 0,962 | 0,923 | 0,842 | 1 |
| IFI44L_HERC6_IL1R2 | 0,881 | 0,873 | 0,889 | 0,897 | 0,838 | 0,956 | 0,941 | 0,889 | 0,994 |
| RSAD2_IL1R2_FAM20A | 0,880 | 0,872 | 0,889 | 0,894 | 0,827 | 0,961 | 0,942 | 0,867 | 1 |
| IFI27_SLC1A2_RETN | 0,880 | 0,870 | 0,889 | 0,893 | 0,827 | 0,960 | 0,925 | 0,843 | 1 |
| OAS1_SLC1A2_OLAH | 0,879 | 0,870 | 0,888 | 0,906 | 0,849 | 0,963 | 0,930 | 0,869 | 0,992 |
| HERC6_IL1R2_OLAH | 0,879 | 0,870 | 0,888 | 0,898 | 0,839 | 0,957 | 0,935 | 0,878 | 0,991 |
| SIGLEC1_HERC6_OLAH | 0,879 | 0,870 | 0,888 | 0,893 | 0,825 | 0,961 | 0,957 | 0,914 | 0,999 |
| OAS1_HERC6_IL1R2 | 0,879 | 0,870 | 0,887 | 0,893 | 0,831 | 0,955 | 0,932 | 0,873 | 0,990 |
| HERC6_SLC1A2_OLAH | 0,879 | 0,870 | 0,888 | 0,902 | 0,844 | 0,960 | 0,937 | 0,880 | 0,993 |

EP 4 365 308 A1

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2_HERC6_IL1R2 | 0,878 | 0,870 | 0,887 | 0,894 | 0,832 | 0,955 | 0,943 | 0,892 | 0,995 |
| RSAD2_SLC1A2_OLAH | 0,878 | 0,869 | 0,887 | 0,901 | 0,843 | 0,960 | 0,935 | 0,878 | 0,991 |
| HERC6_IL1R2_RETN | 0,878 | 0,869 | 0,887 | 0,893 | 0,829 | 0,958 | 0,929 | 0,869 | 0,990 |
| OAS1_OLAH_FAM20A | 0,878 | 0,868 | 0,887 | 0,891 | 0,819 | 0,963 | 0,949 | 0,886 | 1 |
| IFIT1_SIGLEC1_OLAH | 0,878 | 0,869 | 0,886 | 0,893 | 0,825 | 0,960 | 0,943 | 0,890 | 0,997 |
| IFI27_ISG15_FAM20A | 0,878 | 0,867 | 0,888 | 0,886 | 0,813 | 0,960 | 0,930 | 0,850 | 1 |
| IFIT1_SLC1A2_IL1R2 | 0,878 | 0,869 | 0,886 | 0,900 | 0,841 | 0,959 | 0,947 | 0,896 | 0,997 |
| IFI44L_SIGLEC1_OLAH | 0,878 | 0,869 | 0,886 | 0,891 | 0,822 | 0,960 | 0,947 | 0,896 | 0,998 |
| IFI27_HERC6_MMP8 | 0,877 | 0,868 | 0,887 | 0,891 | 0,826 | 0,956 | 0,905 | 0,829 | 0,982 |
| HERC6_IL1R2_MMP8 | 0,877 | 0,869 | 0,886 | 0,896 | 0,837 | 0,955 | 0,926 | 0,865 | 0,988 |
| SIGLEC 1_OLAH_MMP8 | 0,877 | 0,868 | 0,887 | 0,890 | 0,822 | 0,958 | 0,954 | 0,910 | 0,999 |
| IFI44L_SLC1A2_IL1R2 | 0,877 | 0,869 | 0,885 | 0,896 | 0,838 | 0,953 | 0,934 | 0,871 | 0,997 |
| OAS1_SIGLEC1_OLAH | 0,876 | 0,867 | 0,886 | 0,889 | 0,819 | 0,959 | 0,948 | 0,898 | 0,998 |
| SIGLEC1_ISG15_OLAH | 0,876 | 0,867 | 0,885 | 0,890 | 0,821 | 0,959 | 0,949 | 0,900 | 0,998 |
| SIGLEC1_OLAHRETN | 0,876 | 0,866 | 0,885 | 0,890 | 0,819 | 0,961 | 0,945 | 0,890 | 0,999 |
| HERC6_SLC1A2_IL1R2 | 0,876 | 0,867 | 0,885 | 0,901 | 0,847 | 0,956 | 0,914 | 0,835 | 0,993 |
| IFIT1_IL1R2_MMP8 | 0,875 | 0,867 | 0,884 | 0,892 | 0,829 | 0,956 | 0,959 | 0,918 | 0,999 |
| OAS1_IFIT1_IL1R2 | 0,875 | 0,866 | 0,884 | 0,888 | 0,821 | 0,956 | 0,945 | 0,895 | 0,994 |
| ISG15_SLC1A2_OLAH | 0,875 | 0,866 | 0,883 | 0,895 | 0,835 | 0,954 | 0,922 | 0,854 | 0,989 |
| IFIT1_ISG15_IL1R2 | 0,875 | 0,866 | 0,883 | 0,894 | 0,830 | 0,958 | 0,958 | 0,916 | 0,999 |
| IFI27_FAM20A_MMP8 | 0,875 | 0,865 | 0,885 | 0,887 | 0,818 | 0,957 | 0,907 | 0,814 | 0,999 |
| IFI27_IFIT1_FAM20A | 0,875 | 0,864 | 0,885 | 0,888 | 0,817 | 0,959 | 0,941 | 0,862 | 1 |
| RSAD2_SIGLEC1_OLAH | 0,875 | 0,865 | 0,884 | 0,890 | 0,821 | 0,959 | 0,950 | 0,902 | 0,998 |
| HERC6_FAM20A_RETN | 0,875 | 0,864 | 0,885 | 0,887 | 0,816 | 0,958 | 0,939 | 0,861 | 1 |
| IFI44L_OLAH_FAM20A | 0,874 | 0,865 | 0,884 | 0,889 | 0,818 | 0,960 | 0,957 | 0,909 | 1 |

| Modèle | AUC (modèle) | IC 95% | | AUC (Train) | IC 95% (Train) | | AUC (Test) | IC 95% (Test) | |
|---|---|---|---|---|---|---|---|---|---|
| HERC6_OLAH_RETN | 0,874 | 0,864 | 0,884 | 0,889 | 0,818 | 0,959 | 0,942 | 0,891 | 0,993 |
| OAS1_IL1R2_OLAH | 0,873 | 0,864 | 0,882 | 0,894 | 0,828 | 0,960 | 0,938 | 0,879 | 0,997 |
| IFIT1_IFI44L_IL1R2 | 0,873 | 0,864 | 0,882 | 0,890 | 0,824 | 0,956 | 0,953 | 0,909 | 0,997 |
| IFI27_SIGLEC1_MMP8 | 0,873 | 0,863 | 0,882 | 0,883 | 0,813 | 0,954 | 0,916 | 0,844 | 0,988 |
| IFI44L_ISG15_IL1R2 | 0,872 | 0,864 | 0,880 | 0,889 | 0,829 | 0,949 | 0,954 | 0,910 | 0,999 |
| IFI27_OAS1_FAM20A | 0,872 | 0,862 | 0,882 | 0,883 | 0,811 | 0,956 | 0,942 | 0,864 | 1 |
| ISG15_SLC1A2_IL1R2 | 0,872 | 0,864 | 0,880 | 0,894 | 0,836 | 0,952 | 0,917 | 0,838 | 0,997 |
| IFI27_SLC1A2_FAM20A | 0,872 | 0,861 | 0,882 | 0,884 | 0,812 | 0,956 | 0,915 | 0,827 | 1 |
| HERC6_OLAH_MMP8 | 0,872 | 0,862 | 0,881 | 0,887 | 0,820 | 0,955 | 0,941 | 0,889 | 0,994 |
| IFIT1_IL1R2_OLAH | 0,872 | 0,863 | 0,881 | 0,891 | 0,825 | 0,957 | 0,954 | 0,910 | 0,998 |
| IFI44L_HERC6_OLAH | 0,871 | 0,862 | 0,881 | 0,887 | 0,820 | 0,954 | 0,941 | 0,889 | 0,993 |
| RSAD2_SLC1A2_IL1R2 | 0,871 | 0,863 | 0,879 | 0,895 | 0,837 | 0,954 | 0,937 | 0,874 | 1 |
| SIGLEC1_HERC6_RETN | 0,871 | 0,861 | 0,881 | 0,878 | 0,799 | 0,958 | 0,959 | 0,916 | 1 |
| IFI44L_IL1R2_OLAH | 0,871 | 0,862 | 0,880 | 0,889 | 0,827 | 0,952 | 0,942 | 0,889 | 0,995 |
| RSAD2_IFIT1_IL1R2 | 0,871 | 0,862 | 0,880 | 0,888 | 0,820 | 0,956 | 0,953 | 0,909 | 0,997 |
| ISG15_IL1R2_OLAH | 0,871 | 0,862 | 0,879 | 0,889 | 0,827 | 0,950 | 0,938 | 0,876 | 1,000 |
| IFIT1_HERC6_OLAH | 0,871 | 0,861 | 0,880 | 0,886 | 0,819 | 0,953 | 0,940 | 0,888 | 0,993 |
| IFIT1_IL1R2_RETN | 0,870 | 0,861 | 0,880 | 0,888 | 0,819 | 0,957 | 0,954 | 0,910 | 0,998 |
| OAS1_HERC6_OLAH | 0,870 | 0,861 | 0,880 | 0,886 | 0,819 | 0,953 | 0,941 | 0,889 | 0,993 |
| ISG15_HERC6_OLAH | 0,870 | 0,861 | 0,880 | 0,885 | 0,818 | 0,952 | 0,950 | 0,904 | 0,996 |
| RSAD2_IFI27_FAM20A | 0,870 | 0,860 | 0,880 | 0,882 | 0,810 | 0,955 | 0,945 | 0,866 | 1 |
| OAS1_SLC1A2_IL1R2 | 0,870 | 0,861 | 0,878 | 0,896 | 0,837 | 0,954 | 0,917 | 0,844 | 0,991 |
| OAS1_IFI44L_IL1R2 | 0,870 | 0,861 | 0,879 | 0,886 | 0,821 | 0,952 | 0,935 | 0,878 | 0,991 |
| RSAD2_OLAH_FAM20A | 0,870 | 0,860 | 0,879 | 0,884 | 0,811 | 0,958 | 0,959 | 0,905 | 1 |
| SIGLEC1_SLC1A2_RETN | 0,870 | 0,861 | 0,879 | 0,893 | 0,829 | 0,957 | 0,950 | 0,902 | 0,998 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2_HERC6_OLAH | 0,870 | 0,860 | 0,879 | 0,886 | 0,820 | 0,952 | 0,939 | 0,886 | 0,992 |
| RSAD2_IL1R2_OLAH | 0,870 | 0,861 | 0,879 | 0,890 | 0,824 | 0,955 | 0,949 | 0,900 | 0,998 |
| IFI27_SIGLEC1_SLC1A2 | 0,869 | 0,860 | 0,878 | 0,889 | 0,826 | 0,952 | 0,964 | 0,922 | 1 |
| RSAD2_OAS1_IL1R2 | 0,869 | 0,860 | 0,878 | 0,886 | 0,820 | 0,952 | 0,934 | 0,877 | 0,991 |
| IFI44L_IL1R2_MMP8 | 0,868 | 0,860 | 0,876 | 0,883 | 0,821 | 0,945 | 0,949 | 0,900 | 0,997 |
| IFI27_IFI44L_SIGLEC1 | 0,868 | 0,859 | 0,877 | 0,886 | 0,821 | 0,950 | 0,945 | 0,893 | 0,996 |
| IFI27_SLC1A2_MMP8 | 0,868 | 0,858 | 0,877 | 0,886 | 0,821 | 0,951 | 0,892 | 0,805 | 0,980 |
| OAS1_IL1R2_MMP8 | 0,868 | 0,859 | 0,876 | 0,886 | 0,821 | 0,952 | 0,928 | 0,867 | 0,990 |
| RSAD2_IFI27_SIGLEC1 | 0,868 | 0,858 | 0,877 | 0,884 | 0,822 | 0,947 | 0,940 | 0,885 | 0,996 |
| OAS1_ISG15_IL1R2 | 0,868 | 0,859 | 0,876 | 0,888 | 0,823 | 0,952 | 0,932 | 0,871 | 0,992 |
| ISG15_IL1R2_RETN | 0,867 | 0,859 | 0,876 | 0,889 | 0,827 | 0,952 | 0,935 | 0,869 | 1 |
| IFI27_IFI44L_MMP8 | 0,867 | 0,857 | 0,877 | 0,878 | 0,809 | 0,948 | 0,902 | 0,823 | 0,981 |
| ISG15_OLAH_FAM20A | 0,867 | 0,857 | 0,877 | 0,887 | 0,820 | 0,954 | 0,945 | 0,892 | 0,997 |
| RSAD2_IFI44L_IL1R2 | 0,867 | 0,858 | 0,876 | 0,882 | 0,815 | 0,949 | 0,945 | 0,895 | 0,994 |
| IFI27_OAS1_SIGLEC1 | 0,867 | 0,857 | 0,876 | 0,882 | 0,818 | 0,946 | 0,945 | 0,892 | 0,997 |
| IFIT1_OLAH_FAM20A | 0,867 | 0,857 | 0,877 | 0,881 | 0,807 | 0,954 | 0,958 | 0,909 | 1 |
| IFIT1_SIGLEC1_RETN | 0,866 | 0,857 | 0,876 | 0,878 | 0,804 | 0,953 | 0,942 | 0,889 | 0,996 |
| IFI27_IFIT1_SIGLEC1 | 0,866 | 0,857 | 0,876 | 0,885 | 0,823 | 0,947 | 0,941 | 0,886 | 0,996 |
| RSAD2_ISG15_IL1R2 | 0,866 | 0,858 | 0,875 | 0,883 | 0,818 | 0,949 | 0,953 | 0,908 | 0,998 |
| ISG15_IL1R2_MMP8 | 0,866 | 0,858 | 0,874 | 0,884 | 0,824 | 0,944 | 0,933 | 0,865 | 1,000 |
| OAS1_IL1R2_RETN | 0,866 | 0,857 | 0,876 | 0,885 | 0,817 | 0,954 | 0,932 | 0,871 | 0,992 |
| IFI44L_IL1R2_RETN | 0,866 | 0,857 | 0,875 | 0,885 | 0,818 | 0,951 | 0,946 | 0,897 | 0,995 |
| RSAD2_OAS1_OLAH | 0,866 | 0,856 | 0,875 | 0,880 | 0,808 | 0,952 | 0,943 | 0,889 | 0,998 |
| RSAD2_IL1R2_MMP8 | 0,866 | 0,857 | 0,874 | 0,883 | 0,818 | 0,949 | 0,948 | 0,899 | 0,996 |
| SIGLEC1_SLC1A2_MMP8 | 0,866 | 0,857 | 0,874 | 0,884 | 0,819 | 0,949 | 0,937 | 0,882 | 0,992 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| OAS1_OLAH_RETN | 0,866 | 0,856 | 0,875 | 0,884 | 0,811 | 0,958 | 0,945 | 0,891 | 0,998 |
| ISG15_HERC6_RETN | 0,865 | 0,855 | 0,876 | 0,878 | 0,801 | 0,955 | 0,954 | 0,908 | 1 |
| RSAD2_IL1R2_RETN | 0,865 | 0,856 | 0,874 | 0,883 | 0,813 | 0,953 | 0,946 | 0,896 | 0,995 |
| OAS1_IFIT1_OLAH | 0,865 | 0,856 | 0,875 | 0,880 | 0,806 | 0,953 | 0,943 | 0,891 | 0,996 |
| IFI27_ISG15_MMP8 | 0,865 | 0,856 | 0,874 | 0,878 | 0,808 | 0,948 | 0,899 | 0,818 | 0,979 |
| IFI27_IFIT1_MMP8 | 0,865 | 0,855 | 0,875 | 0,876 | 0,805 | 0,946 | 0,913 | 0,839 | 0,987 |
| SIGLEC1_ISG15_RETN | 0,864 | 0,854 | 0,874 | 0,875 | 0,798 | 0,952 | 0,953 | 0,907 | 0,999 |
| OAS1_OLAH_MMP8 | 0,864 | 0,855 | 0,874 | 0,879 | 0,806 | 0,951 | 0,949 | 0,899 | 0,999 |
| IFI44L_SIGLEC1_RETN | 0,864 | 0,854 | 0,874 | 0,876 | 0,799 | 0,953 | 0,948 | 0,899 | 0,997 |
| HERC6_SLC1A2_FAM20A | 0,864 | 0,854 | 0,874 | 0,886 | 0,824 | 0,949 | 0,928 | 0,843 | 1 |
| ISG15_HERC6_FAM20A | 0,864 | 0,854 | 0,874 | 0,875 | 0,803 | 0,947 | 0,930 | 0,845 | 1 |
| OAS 1_SIGLEC1_RETN | 0,864 | 0,853 | 0,874 | 0,876 | 0,799 | 0,953 | 0,946 | 0,894 | 0,997 |
| HERC6_FAM20A_MMP8 | 0,863 | 0,853 | 0,874 | 0,878 | 0,808 | 0,949 | 0,927 | 0,845 | 1 |
| RSAD2_SIGLEC1_RETN | 0,863 | 0,853 | 0,874 | 0,876 | 0,799 | 0,953 | 0,945 | 0,893 | 0,996 |
| OAS1_IFI44L_OLAH | 0,863 | 0,853 | 0,872 | 0,882 | 0,810 | 0,953 | 0,941 | 0,887 | 0,996 |
| SLC1A2_OLAH_FAM20A | 0,863 | 0,853 | 0,872 | 0,884 | 0,822 | 0,945 | 0,859 | 0,754 | 0,963 |
| IFI27_SIGLEC1_ISG15 | 0,863 | 0,853 | 0,873 | 0,880 | 0,815 | 0,945 | 0,950 | 0,899 | 1 |
| IFI27_OAS1_MMP8 | 0,862 | 0,852 | 0,872 | 0,875 | 0,804 | 0,946 | 0,901 | 0,822 | 0,980 |
| OAS1_ISG15_OLAH | 0,862 | 0,852 | 0,871 | 0,880 | 0,808 | 0,952 | 0,943 | 0,890 | 0,997 |
| RSAD2_IFI27_MMP8 | 0,862 | 0,852 | 0,871 | 0,877 | 0,806 | 0,947 | 0,908 | 0,830 | 0,985 |
| IFI44L_SLC1A2_FAM20A | 0,861 | 0,852 | 0,871 | 0,879 | 0,810 | 0,948 | 0,925 | 0,843 | 1 |
| IFI44L_OLAH_MMP8 | 0,861 | 0,852 | 0,871 | 0,872 | 0,800 | 0,944 | 0,937 | 0,881 | 0,993 |
| SIGLEC 1_RETN_MMP8 | 0,861 | 0,851 | 0,872 | 0,873 | 0,796 | 0,951 | 0,953 | 0,906 | 1 |
| HERC6_SLC1A2_RETN | 0,861 | 0,851 | 0,871 | 0,885 | 0,818 | 0,953 | 0,933 | 0,866 | 0,999 |
| IFIT1_SIGLEC1_SLC1A2 | 0,861 | 0,852 | 0,869 | 0,878 | 0,813 | 0,943 | 0,950 | 0,904 | 0,996 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| IFI44L_ISG15_OLAH | 0,861 | 0,851 | 0,870 | 0,876 | 0,805 | 0,946 | 0,942 | 0,891 | 0,994 |
| RSAD2_OLAH_RETN | 0,861 | 0,851 | 0,871 | 0,877 | 0,800 | 0,953 | 0,958 | 0,914 | 1 |
| IFI27_SIGLEC1_HERC6 | 0,860 | 0,850 | 0,871 | 0,878 | 0,812 | 0,944 | 0,947 | 0,897 | 0,997 |
| SIGLEC1_HERC6_SLC1A2 | 0,860 | 0,852 | 0,869 | 0,877 | 0,810 | 0,944 | 0,954 | 0,908 | 1 |
| CAS1_SIGLEC1_SLC1A2 | 0,860 | 0,851 | 0,869 | 0,876 | 0,811 | 0,942 | 0,951 | 0,906 | 0,997 |
| IFI44L_HERC6_RETN | 0,860 | 0,850 | 0,870 | 0,871 | 0,792 | 0,950 | 0,937 | 0,879 | 0,995 |
| RSAD2_OLAH_MMP8 | 0,860 | 0,850 | 0,869 | 0,874 | 0,801 | 0,947 | 0,950 | 0,902 | 0,998 |
| IFIT1_HERC6_RETN | 0,860 | 0,849 | 0,870 | 0,873 | 0,796 | 0,950 | 0,927 | 0,865 | 0,989 |
| IFI44L_OLAH_RETN | 0,860 | 0,849 | 0,870 | 0,877 | 0,801 | 0,952 | 0,939 | 0,883 | 0,995 |
| IFIT1_HERC6_FAM20A | 0,860 | 0,849 | 0,870 | 0,873 | 0,801 | 0,945 | 0,928 | 0,843 | 1 |
| RSAD2_SIGLEC1_SLC1A2 | 0,859 | 0,851 | 0,868 | 0,877 | 0,811 | 0,943 | 0,948 | 0,900 | 0,996 |
| SLC1A2_IL1R2_OLAH | 0,859 | 0,850 | 0,868 | 0,877 | 0,815 | 0,939 | 0,842 | 0,733 | 0,952 |
| RSAD2_IFI27_HERC6 | 0,859 | 0,850 | 0,869 | 0,880 | 0,820 | 0,940 | 0,886 | 0,807 | 0,965 |
| SIGLEC 1_HERC6_MMP8 | 0,859 | 0,849 | 0,869 | 0,871 | 0,795 | 0,947 | 0,914 | 0,848 | 0,981 |
| IFIT1_ISG15_OLAH | 0,859 | 0,850 | 0,869 | 0,875 | 0,803 | 0,948 | 0,957 | 0,913 | 1,000 |
| IFIT1_OLAH_RETN | 0,859 | 0,849 | 0,869 | 0,878 | 0,803 | 0,953 | 0,953 | 0,908 | 0,998 |
| IFIT1_OLAH_MMP8 | 0,859 | 0,849 | 0,869 | 0,870 | 0,796 | 0,945 | 0,953 | 0,909 | 0,998 |
| IFI44L_SIGLEC1_SLC1A2 | 0,859 | 0,851 | 0,867 | 0,880 | 0,814 | 0,945 | 0,955 | 0,913 | 0,998 |
| IFI44L_HERC6_FAM20A | 0,859 | 0,849 | 0,869 | 0,870 | 0,796 | 0,945 | 0,934 | 0,850 | 1 |
| ISG15_OLAH_RETN | 0,859 | 0,849 | 0,868 | 0,879 | 0,810 | 0,948 | 0,948 | 0,894 | 1 |
| IFI27_HERC6_SLC1A2 | 0,859 | 0,849 | 0,869 | 0,880 | 0,817 | 0,943 | 0,921 | 0,847 | 0,994 |
| IFI27_OAS1_HERC6 | 0,859 | 0,849 | 0,869 | 0,880 | 0,819 | 0,940 | 0,901 | 0,829 | 0,973 |
| IFIT1_IFI44L_OLAH | 0,859 | 0,849 | 0,868 | 0,876 | 0,803 | 0,948 | 0,941 | 0,888 | 0,994 |
| OAS 1_FAM20A_RETN | 0,858 | 0,848 | 0,869 | 0,880 | 0,806 | 0,954 | 0,934 | 0,852 | 1 |
| RSAD2_IFIT1_OLAH | 0,858 | 0,849 | 0,868 | 0,875 | 0,802 | 0,948 | 0,951 | 0,904 | 0,998 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| OAS1_HERC6_RETN | 0,858 | 0,848 | 0,868 | 0,870 | 0,793 | 0,948 | 0,925 | 0,861 | 0,989 |
| IFI27_IFI44L_SLC1A2 | 0,858 | 0,849 | 0,868 | 0,877 | 0,809 | 0,945 | 0,927 | 0,863 | 0,991 |
| SIGLEC1_ISG15_SLC1A2 | 0,858 | 0,850 | 0,866 | 0,878 | 0,811 | 0,944 | 0,954 | 0,910 | 0,998 |
| IFI27_ISG15_HERC6 | 0,858 | 0,847 | 0,869 | 0,878 | 0,815 | 0,941 | 0,911 | 0,843 | 0,979 |
| IFI27_IFI44L_HERC6 | 0,858 | 0,848 | 0,868 | 0,876 | 0,813 | 0,939 | 0,907 | 0,837 | 0,976 |
| ISG15_OLAH_MMP8 | 0,858 | 0,849 | 0,867 | 0,875 | 0,807 | 0,942 | 0,943 | 0,892 | 0,995 |
| SLC1A2_OLAH_MMP8 | 0,858 | 0,849 | 0,867 | 0,879 | 0,817 | 0,941 | 0,855 | 0,754 | 0,957 |
| RSAD2_ISG15_OLAH | 0,858 | 0,848 | 0,867 | 0,877 | 0,805 | 0,948 | 0,952 | 0,906 | 0,998 |
| SLC1A2_OLAH_RETN | 0,858 | 0,848 | 0,867 | 0,878 | 0,815 | 0,941 | 0,842 | 0,733 | 0,952 |
| RSAD2_HERC6_FAM20A | 0,858 | 0,848 | 0,868 | 0,874 | 0,804 | 0,945 | 0,924 | 0,837 | 1 |
| RSAD2_HERC6_RETN | 0,858 | 0,848 | 0,868 | 0,871 | 0,794 | 0,948 | 0,922 | 0,857 | 0,987 |
| IFI27_ISG15_SLC1A2 | 0,857 | 0,848 | 0,867 | 0,875 | 0,803 | 0,946 | 0,918 | 0,845 | 0,992 |
| RSAD2_IFI44L_OLAH | 0,857 | 0,848 | 0,867 | 0,876 | 0,803 | 0,948 | 0,946 | 0,896 | 0,996 |
| IFI27_IFIT1_SLC1A2 | 0,857 | 0,847 | 0,867 | 0,877 | 0,809 | 0,945 | 0,949 | 0,893 | 1 |
| HERC6_RETN_MMP8 | 0,857 | 0,846 | 0,867 | 0,870 | 0,792 | 0,948 | 0,916 | 0,850 | 0,983 |
| OAS1_HERC6_FAM20A | 0,857 | 0,846 | 0,867 | 0,870 | 0,797 | 0,944 | 0,933 | 0,848 | 1 |
| RSAD2_SIGLEC1_HERC6 | 0,856 | 0,846 | 0,865 | 0,869 | 0,803 | 0,936 | 0,911 | 0,843 | 0,979 |
| IFIT1_SIGLEC1_HERC6 | 0,855 | 0,846 | 0,864 | 0,867 | 0,798 | 0,937 | 0,912 | 0,845 | 0,979 |
| IFIT1_SIGLEC1_MMP8 | 0,855 | 0,845 | 0,865 | 0,867 | 0,793 | 0,942 | 0,913 | 0,846 | 0,980 |
| HERC6_SLC1A2_MMP8 | 0,855 | 0,845 | 0,864 | 0,875 | 0,807 | 0,943 | 0,892 | 0,809 | 0,976 |
| OAS1_SIGLEC1_HERC6 | 0,854 | 0,845 | 0,864 | 0,866 | 0,798 | 0,935 | 0,941 | 0,890 | 0,993 |
| IFI27_IFIT1_HERC6 | 0,854 | 0,843 | 0,864 | 0,877 | 0,813 | 0,940 | 0,917 | 0,852 | 0,982 |
| IFI44L_SIGLEC1_MMP8 | 0,853 | 0,843 | 0,863 | 0,863 | 0,787 | 0,940 | 0,924 | 0,863 | 0,985 |
| IFI44L_FAM20A_RETN | 0,853 | 0,842 | 0,863 | 0,867 | 0,790 | 0,944 | 0,937 | 0,867 | 1 |
| IFI27_OAS1_SLC1A2 | 0,853 | 0,843 | 0,863 | 0,871 | 0,800 | 0,942 | 0,920 | 0,844 | 0,995 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2_IFI27_SLC1A2 | 0,853 | 0,843 | 0,863 | 0,871 | 0,800 | 0,942 | 0,921 | 0,846 | 0,996 |
| IFI44L_SLC1A2_RETN | 0,853 | 0,842 | 0,863 | 0,876 | 0,805 | 0,946 | 0,925 | 0,863 | 0,987 |
| IFIT1_SLC1A2_FAM20A | 0,852 | 0,842 | 0,863 | 0,869 | 0,797 | 0,942 | 0,933 | 0,851 | 1 |
| OAS1_SIGLEC1_MMP8 | 0,852 | 0,842 | 0,862 | 0,864 | 0,788 | 0,940 | 0,923 | 0,861 | 0,985 |
| IFI44L_ISG15_FAM20A | 0,852 | 0,842 | 0,861 | 0,860 | 0,779 | 0,941 | 0,940 | 0,864 | 1 |
| IFI44L_SIGLEC1_HERC6 | 0,852 | 0,842 | 0,861 | 0,862 | 0,790 | 0,935 | 0,938 | 0,885 | 0,991 |
| SIGLEC1_ISG15_MMP8 | 0,851 | 0,841 | 0,861 | 0,865 | 0,788 | 0,941 | 0,926 | 0,866 | 0,986 |
| RSAD2_SIGLEC1_MMP8 | 0,851 | 0,841 | 0,861 | 0,864 | 0,788 | 0,940 | 0,921 | 0,858 | 0,984 |

Performances obtenues pour l'identification de la nature virale ou bactérienne de l'infection sur la base des combinaisons à 4 gènes :

[Tableau 8]

| Modèle | AUC modèle | IC 95% | | AUC Train | IC 95% Train | | AUC Test | IC 95% Test | |
|---|---|---|---|---|---|---|---|---|---|
| IFI27_SIGLEC1_IL1R2_FAM20A | 0,909 | 0,901 | 0,917 | 0,926 | 0,871 | 0,981 | 0,949 | 0,881 | 1 |
| IFI27_IL1R2_OLAH_FAM20A | 0,909 | 0,901 | 0,917 | 0,926 | 0,870 | 0,981 | 0,926 | 0,852 | 1,000 |
| IFI27_HERC6_IL1R2_FAM20A | 0,909 | 0,901 | 0,917 | 0,927 | 0,877 | 0,978 | 0,927 | 0,845 | 1 |
| IFI27_OLAH_FAM20A_MMP8 | 0,909 | 0,900 | 0,917 | 0,922 | 0,864 | 0,980 | 0,929 | 0,858 | 1 |
| IFI27_HERC6_OLAH_FAM20A | 0,909 | 0,901 | 0,917 | 0,922 | 0,862 | 0,982 | 0,942 | 0,879 | 1 |
| IFI27_ISG15_OLAH_FAM20A | 0,908 | 0,900 | 0,916 | 0,922 | 0,863 | 0,980 | 0,925 | 0,851 | 0,999 |
| IFI27_IFI44L_IL1R2_FAM20A | 0,907 | 0,899 | 0,915 | 0,924 | 0,870 | 0,978 | 0,927 | 0,845 | 1 |
| IFI27_SIGLEC1_OLAH_FAM20A | 0,906 | 0,897 | 0,915 | 0,918 | 0,855 | 0,981 | 0,952 | 0,893 | 1 |
| IFI27_OLAH_FAM20A_RETN | 0,906 | 0,897 | 0,914 | 0,921 | 0,862 | 0,981 | 0,934 | 0,863 | 1 |
| IFI27_IFI44L_OLAH_FAM20A | 0,906 | 0,897 | 0,914 | 0,920 | 0,858 | 0,981 | 0,938 | 0,872 | 1 |
| IFI27_IFIT1_OLAH_FAM20A | 0,905 | 0,897 | 0,913 | 0,919 | 0,858 | 0,981 | 0,937 | 0,869 | 1 |
| RSAD2_IFI27_OLAH_FAM20A | 0,905 | 0,896 | 0,913 | 0,920 | 0,859 | 0,981 | 0,937 | 0,869 | 1 |
| IFI27_IFIT1_IL1R2_FAM20A | 0,905 | 0,896 | 0,913 | 0,923 | 0,869 | 0,977 | 0,929 | 0,850 | 1 |
| IFI27_OAS1_OLAH_FAM20A | 0,904 | 0,896 | 0,913 | 0,920 | 0,860 | 0,981 | 0,937 | 0,870 | 1 |
| IFI27_IL1R2_FAM20A_MMP8 | 0,904 | 0,896 | 0,912 | 0,921 | 0,870 | 0,973 | 0,908 | 0,815 | 1 |
| RSAD2_IFI27_HERC6_OLAH | 0,904 | 0,896 | 0,912 | 0,923 | 0,871 | 0,976 | 0,937 | 0,879 | 0,995 |
| IFI27_IFI44L_HERC6_OLAH | 0,904 | 0,896 | 0,912 | 0,924 | 0,872 | 0,977 | 0,937 | 0,880 | 0,994 |
| IFI27_OAS1_HERC6_OLAH | 0,904 | 0,896 | 0,912 | 0,923 | 0,871 | 0,975 | 0,935 | 0,876 | 0,994 |
| IFI27_OAS1_IL1R2_FAM20A | 0,904 | 0,896 | 0,912 | 0,925 | 0,872 | 0,977 | 0,932 | 0,852 | 1 |
| IFI27_ISG15_IL1R2_FAM20A | 0,904 | 0,896 | 0,911 | 0,921 | 0,867 | 0,974 | 0,914 | 0,826 | 1 |
| RSAD2_IFI27_IL1R2_FAM20A | 0,904 | 0,896 | 0,912 | 0,925 | 0,872 | 0,977 | 0,927 | 0,844 | 1 |
| IFI27_ISG15_HERC6_OLAH | 0,904 | 0,896 | 0,912 | 0,919 | 0,863 | 0,975 | 0,938 | 0,880 | 0,996 |
| IFI27_SLC1A2_OLAH_FAM20A | 0,904 | 0,895 | 0,912 | 0,925 | 0,870 | 0,981 | 0,924 | 0,846 | 1 |
| IFI27_HERC6_IL1R2_OLAH | 0,903 | 0,896 | 0,911 | 0,924 | 0,873 | 0,975 | 0,943 | 0,890 | 0,997 |
| IFI27_IL1R2_OLAH_MMP8 | 0,903 | 0,896 | 0,911 | 0,922 | 0,870 | 0,974 | 0,937 | 0,878 | 0,996 |

(suite)

| Modèle | AUC modèle | IC 95% | | AUC Train | IC 95% Train | | AUC Test | IC 95% Test | |
|---|---|---|---|---|---|---|---|---|---|
| IFI27_ IFIT1_ HERC6_ OLAH | 0,903 | 0,895 | 0,911 | 0,922 | 0,868 | 0,976 | 0,939 | 0,882 | 0,996 |
| IFI27_ OLAH_ RETN_ MMP8 | 0,903 | 0,894 | 0,912 | 0,920 | 0,861 | 0,978 | 0,948 | 0,889 | 1 |
| IFI27_ ISG15_ IL1R2_ OLAH | 0,903 | 0,895 | 0,910 | 0,922 | 0,870 | 0,975 | 0,935 | 0,873 | 0,997 |
| IFI27_ IL1R2_ FAM20A_ RETN | 0,903 | 0,895 | 0,911 | 0,922 | 0,870 | 0,974 | 0,912 | 0,822 | 1 |
| IFI27_ ISG15_ OLAH_ MMP8 | 0,902 | 0,894 | 0,910 | 0,916 | 0,859 | 0,973 | 0,934 | 0,871 | 0,996 |
| IFI27_ IFI44L_ SIGLEC1_ OLAH | 0,902 | 0,894 | 0,910 | 0,920 | 0,864 | 0,976 | 0,948 | 0,896 | 0,999 |
| IFI27_ HERC6_ OLAH_ MMP8 | 0,902 | 0,894 | 0,910 | 0,918 | 0,861 | 0,975 | 0,941 | 0,886 | 0,997 |
| SIGLEC1_ IL1R2_ FAM20A_ MMP8 | 0,902 | 0,893 | 0,910 | 0,917 | 0,858 | 0,976 | 0,961 | 0,900 | 1 |
| IFI27_ HERC6_ FAM20A_ RETN | 0,902 | 0,893 | 0,911 | 0,919 | 0,859 | 0,979 | 0,940 | 0,862 | 1 |
| IFI27_ SIGLEC1_ IL1R2_ OLAH | 0,902 | 0,894 | 0,909 | 0,921 | 0,869 | 0,973 | 0,942 | 0,888 | 0,997 |
| IFI27_ SIGLEC1_ HERC6_ OLAH | 0,901 | 0,893 | 0,910 | 0,917 | 0,859 | 0,975 | 0,942 | 0,887 | 0,997 |
| IFI27_ IFI44L_ IL1R2_ OLAH | 0,901 | 0,893 | 0,909 | 0,922 | 0,869 | 0,974 | 0,938 | 0,879 | 0,997 |
| IFI27_ HERC6_ OLAH_ RETN | 0,901 | 0,893 | 0,909 | 0,917 | 0,858 | 0,976 | 0,949 | 0,895 | 1 |
| IFI27_ SIGLEC1_ SLC1A2_ OLAH | 0,901 | 0,893 | 0,909 | 0,927 | 0,875 | 0,979 | 0,940 | 0,881 | 1,000 |
| IFI27_ SIGLEC1_ OLAH_ MMP8 | 0,901 | 0,893 | 0,909 | 0,917 | 0,859 | 0,975 | 0,945 | 0,890 | 0,999 |
| IFI27_ SLC1A2_ OLAH_ MMP8 | 0,901 | 0,892 | 0,909 | 0,926 | 0,874 | 0,978 | 0,930 | 0,864 | 0,997 |
| IFI27_ SIGLEC1_ ISG15_ OLAH | 0,901 | 0,893 | 0,909 | 0,915 | 0,857 | 0,974 | 0,942 | 0,886 | 0,999 |
| IFI27_ OAS1_ HERC6_ RETN | 0,901 | 0,892 | 0,910 | 0,915 | 0,854 | 0,976 | 0,932 | 0,860 | 1 |
| IFI27_ IFIT1_ SIGLEC1_ FAM20A | 0,901 | 0,892 | 0,909 | 0,914 | 0,849 | 0,978 | 0,948 | 0,878 | 1 |
| IFI27_ IFI44L_ OLAH_ MMP8 | 0,901 | 0,892 | 0,909 | 0,916 | 0,859 | 0,974 | 0,937 | 0,876 | 0,997 |
| IFI27_ ISG15_ SLC1A2_ OLAH | 0,901 | 0,893 | 0,909 | 0,926 | 0,874 | 0,977 | 0,923 | 0,850 | 0,995 |
| IFI27_ ISG15_ OLAH_ RETN | 0,901 | 0,892 | 0,909 | 0,917 | 0,859 | 0,975 | 0,939 | 0,874 | 1 |
| IFI27_ IFIT1_ SIGLEC1_ OLAH | 0,901 | 0,893 | 0,909 | 0,918 | 0,861 | 0,974 | 0,941 | 0,885 | 0,997 |
| IFI27_ SLC1A2_ IL1R2_ OLAH | 0,901 | 0,893 | 0,908 | 0,927 | 0,877 | 0,977 | 0,926 | 0,856 | 0,996 |
| IFI27_ IFIT1_ IL1R2_ OLAH | 0,900 | 0,892 | 0,908 | 0,921 | 0,869 | 0,974 | 0,941 | 0,886 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27_ IFI44L_ SIGLEC1_ IL1R2 | 0,900 | 0,893 | 0,907 | 0,919 | 0,869 | 0,968 | 0,943 | 0,893 | 0,994 |
| IFI27_ IL1R2_ OLAH_ RETN | 0,900 | 0,892 | 0,908 | 0,918 | 0,863 | 0,974 | 0,941 | 0,879 | 1 |
| IFI27_ IFI44L_ SLC1A2_ OLAH | 0,900 | 0,892 | 0,909 | 0,927 | 0,875 | 0,978 | 0,932 | 0,866 | 0,997 |
| RSAD2_ IFI27_ SLC1A2_ OLAH | 0,900 | 0,892 | 0,908 | 0,926 | 0,875 | 0,978 | 0,932 | 0,866 | 0,997 |
| IFI27_ IFIT1_ SLC1A2_ OLAH | 0,900 | 0,892 | 0,908 | 0,926 | 0,874 | 0,979 | 0,935 | 0,871 | 0,998 |
| RSAD2_ IFI27_ IL1R2_ OLAH | 0,900 | 0,892 | 0,908 | 0,921 | 0,868 | 0,974 | 0,941 | 0,886 | 0,997 |
| IFI27_ IFIT1_ OLAH_ MMP8 | 0,900 | 0,891 | 0,908 | 0,916 | 0,858 | 0,974 | 0,939 | 0,881 | 0,998 |
| IFI27_ OAS1_ IL1R2_ OLAH | 0,900 | 0,892 | 0,908 | 0,922 | 0,869 | 0,974 | 0,941 | 0,887 | 0,996 |
| IFI27_ HERC6_ SLC1A2_ OLAH | 0,900 | 0,891 | 0,908 | 0,928 | 0,878 | 0,979 | 0,936 | 0,874 | 0,998 |
| IFI27_ SIGLEC1_ IL1R2_ RETN | 0,899 | 0,892 | 0,907 | 0,920 | 0,866 | 0,973 | 0,958 | 0,913 | 1 |
| IFI27_ OAS1_ IFIT1_ OLAH | 0,899 | 0,891 | 0,907 | 0,916 | 0,858 | 0,974 | 0,938 | 0,877 | 0,999 |
| IFI27_ IFI44L_ HERC6_ IL1R2 | 0,899 | 0,892 | 0,907 | 0,918 | 0,870 | 0,966 | 0,929 | 0,870 | 0,988 |
| IFI27_ OAS1_ SIGLEC1_ IL1R2 | 0,899 | 0,892 | 0,907 | 0,919 | 0,870 | 0,968 | 0,950 | 0,903 | 0,997 |
| IFI27_ SIGLEC1_ HERC6_ IL1R2 | 0,899 | 0,892 | 0,907 | 0,921 | 0,872 | 0,969 | 0,949 | 0,902 | 0,996 |
| IFI27_ IFI44L_ ISG15_ OLAH | 0,899 | 0,891 | 0,907 | 0,917 | 0,859 | 0,974 | 0,934 | 0,871 | 0,996 |
| IFI27_ OAS1_ SIGLEC1_ OLAH | 0,899 | 0,891 | 0,907 | 0,918 | 0,861 | 0,974 | 0,943 | 0,888 | 0,999 |
| IFI27_ SIGLEC1_ IL1R2_ MMP8 | 0,899 | 0,892 | 0,906 | 0,921 | 0,873 | 0,969 | 0,950 | 0,903 | 0,997 |
| RSAD2_ IFI27_ SIGLEC1_ OLAH | 0,899 | 0,891 | 0,907 | 0,919 | 0,863 | 0,975 | 0,941 | 0,885 | 0,998 |
| IFI27_ IFIT1_ ISG15_ OLAH | 0,899 | 0,891 | 0,907 | 0,914 | 0,854 | 0,973 | 0,935 | 0,873 | 0,996 |
| IFI27_ OAS1_ OLAH_ MMP8 | 0,899 | 0,891 | 0,907 | 0,916 | 0,858 | 0,974 | 0,937 | 0,877 | 0,997 |
| IFI27_ OAS1_ HERC6_ IL1R2 | 0,899 | 0,892 | 0,906 | 0,918 | 0,870 | 0,966 | 0,929 | 0,870 | 0,988 |
| IFI27_ SIGLEC1_ OLAH_ RETN | 0,899 | 0,890 | 0,907 | 0,916 | 0,856 | 0,976 | 0,951 | 0,896 | 1 |
| IFI27_ IFI44L_ OLAH_ RETN | 0,899 | 0,890 | 0,907 | 0,914 | 0,855 | 0,973 | 0,941 | 0,878 | 1 |
| RSAD2_ IFI27_ OLAH_ MMP8 | 0,899 | 0,891 | 0,907 | 0,916 | 0,857 | 0,974 | 0,938 | 0,879 | 0,997 |
| IFI27_ IFIT1_ SIGLEC1_ IL1R2 | 0,899 | 0,891 | 0,906 | 0,919 | 0,870 | 0,968 | 0,950 | 0,903 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27_ OAS1_ SLC1A2_ OLAH | 0,899 | 0,890 | 0,907 | 0,926 | 0,874 | 0,978 | 0,928 | 0,860 | 0,997 |
| RSAD2_ IFI27_ SIGLEC1_ IL1R2 | 0,899 | 0,891 | 0,906 | 0,919 | 0,870 | 0,968 | 0,948 | 0,899 | 0,997 |
| IFI27_ IFIT1_ OLAH_ RETN | 0,899 | 0,890 | 0,907 | 0,915 | 0,855 | 0,974 | 0,945 | 0,884 | 1 |
| IFI27_ SIGLEC1_ ISG15_ IL1R2 | 0,899 | 0,892 | 0,906 | 0,920 | 0,871 | 0,969 | 0,947 | 0,897 | 0,996 |
| IFI27_ HERC6_ IL1R2_ RETN | 0,899 | 0,891 | 0,906 | 0,919 | 0,866 | 0,973 | 0,938 | 0,880 | 0,996 |
| IFI27_ SLC1A2_ OLAH_ RETN | 0,898 | 0,890 | 0,907 | 0,925 | 0,873 | 0,977 | 0,933 | 0,864 | 1 |
| RSAD2_ IFI27_ HERC6_ IL1R2 | 0,898 | 0,891 | 0,906 | 0,918 | 0,869 | 0,966 | 0,932 | 0,873 | 0,990 |
| IFI27_ OAS1_ OLAH_ RETN | 0,898 | 0,890 | 0,906 | 0,915 | 0,856 | 0,974 | 0,942 | 0,882 | 1 |
| IFI27_ SIGLEC1_ FAM20A_ RETN | 0,898 | 0,889 | 0,907 | 0,913 | 0,850 | 0,976 | 0,952 | 0,880 | 1 |
| IFI27_ IFI44L_ IL1R2_ RETN | 0,898 | 0,890 | 0,906 | 0,920 | 0,868 | 0,972 | 0,938 | 0,877 | 0,999 |
| RSAD2_ IFI27_ SIGLEC1_ FAM20A | 0,898 | 0,889 | 0,907 | 0,914 | 0,850 | 0,978 | 0,948 | 0,878 | 1 |
| RSAD2_ IFI27_ IFIT1_ OLAH | 0,898 | 0,890 | 0,906 | 0,916 | 0,857 | 0,974 | 0,938 | 0,877 | 0,999 |
| RSAD2_ IFI27_ OAS1_ OLAH | 0,898 | 0,890 | 0,906 | 0,917 | 0,859 | 0,974 | 0,938 | 0,879 | 0,997 |
| IFI27_ SLC1A2_ IL1R2_ FAM20A | 0,898 | 0,889 | 0,906 | 0,922 | 0,874 | 0,971 | 0,905 | 0,810 | 1 |
| IFI27_ IFIT1_ HERC6_ IL1R2 | 0,898 | 0,890 | 0,905 | 0,915 | 0,865 | 0,965 | 0,938 | 0,884 | 0,992 |
| IFI27_ IFIT1_ IFI44L_ OLAH | 0,897 | 0,889 | 0,906 | 0,916 | 0,858 | 0,975 | 0,939 | 0,881 | 0,998 |
| RSAD2_ IFI27_ ISG15_ OLAH | 0,897 | 0,889 | 0,905 | 0,915 | 0,856 | 0,974 | 0,936 | 0,875 | 0,996 |
| IFI27_ OAS1_ SIGLEC1_ FAM20A | 0,897 | 0,888 | 0,906 | 0,910 | 0,845 | 0,976 | 0,948 | 0,879 | 1 |
| IFI27_ OAS1_ ISG15_ OLAH | 0,897 | 0,889 | 0,905 | 0,916 | 0,858 | 0,974 | 0,936 | 0,877 | 0,995 |
| RSAD2_ IFI27_ OLAH_ RETN | 0,897 | 0,889 | 0,905 | 0,914 | 0,854 | 0,973 | 0,945 | 0,885 | 1 |
| IFI27_ IL1R2_ RETN_ MMP8 | 0,897 | 0,889 | 0,905 | 0,921 | 0,870 | 0,972 | 0,921 | 0,846 | 0,995 |
| IFI27_ ISG15_ HERC6_ IL1R2 | 0,897 | 0,889 | 0,904 | 0,918 | 0,869 | 0,966 | 0,928 | 0,869 | 0,988 |
| IFI27_ IFIT1_ IL1R2_ RETN | 0,897 | 0,889 | 0,904 | 0,917 | 0,864 | 0,971 | 0,946 | 0,892 | 0,999 |
| IFI27_ OAS1_ IFI44L_ OLAH | 0,896 | 0,888 | 0,905 | 0,918 | 0,861 | 0,975 | 0,938 | 0,879 | 0,997 |
| IFIT1_ SIGLEC1_ HERC6_ FAM20A | 0,896 | 0,888 | 0,905 | 0,911 | 0,849 | 0,972 | 0,937 | 0,865 | 1 |

| Modèle | AUC modèle | IC 95% | | AUC Train | IC 95% Train | | AUC Test | IC 95% Test | |
|---|---|---|---|---|---|---|---|---|---|
| IFI27_ HERC6_ IL1R2_ MMP8 | 0,896 | 0,889 | 0,904 | 0,916 | 0,867 | 0,965 | 0,933 | 0,875 | 0,990 |
| IFI27_ ISG15_ IL1R2_ RETN | 0,896 | 0,888 | 0,904 | 0,919 | 0,866 | 0,971 | 0,932 | 0,865 | 0,998 |
| RSAD2_ IFI27_ IFI44L_ OLAH | 0,896 | 0,888 | 0,904 | 0,917 | 0,860 | 0,974 | 0,940 | 0,883 | 0,998 |
| RSAD2_ IFI27_ HERC6_ RETN | 0,896 | 0,887 | 0,905 | 0,913 | 0,853 | 0,974 | 0,940 | 0,878 | 1 |
| IFI27_ IFIT1_ IL1R2_ MMP8 | 0,896 | 0,888 | 0,903 | 0,916 | 0,865 | 0,966 | 0,942 | 0,890 | 0,995 |
| IFI27_ IFI44L_ IL1R2_ MMP8 | 0,896 | 0,888 | 0,903 | 0,912 | 0,861 | 0,963 | 0,932 | 0,873 | 0,991 |
| IFI27_ OAS1_ IFIT1_ IL1R2 | 0,896 | 0,888 | 0,903 | 0,915 | 0,864 | 0,966 | 0,943 | 0,890 | 0,997 |
| IFI27_ IFIT1_ IFI44L_ IL1R2 | 0,895 | 0,888 | 0,903 | 0,915 | 0,863 | 0,967 | 0,938 | 0,883 | 0,993 |
| IFI27_ OAS1_ IL1R2_ RETN | 0,895 | 0,888 | 0,903 | 0,918 | 0,866 | 0,971 | 0,938 | 0,879 | 0,997 |
| RSAD2_ IFI27_ IL1R2_ RETN | 0,895 | 0,887 | 0,903 | 0,919 | 0,865 | 0,972 | 0,940 | 0,882 | 0,998 |
| RSAD2_ IFI27_ IFIT1_ IL1R2 | 0,895 | 0,887 | 0,902 | 0,916 | 0,865 | 0,966 | 0,940 | 0,887 | 0,994 |
| IFI27_ SIGLEC1_ SLC1A2_ IL1R2 | 0,895 | 0,887 | 0,902 | 0,924 | 0,878 | 0,970 | 0,957 | 0,912 | 1 |
| SIGLEC1_ HERC6_ IL1R2_ FAM20A | 0,895 | 0,886 | 0,903 | 0,912 | 0,849 | 0,974 | 0,958 | 0,897 | 1 |
| IFI27_ IFI44L_ SLC1A2_ IL1R2 | 0,894 | 0,887 | 0,902 | 0,922 | 0,876 | 0,967 | 0,932 | 0,867 | 0,996 |
| IFI27_ IFI44L_ ISG15_ IL1R2 | 0,894 | 0,887 | 0,902 | 0,915 | 0,866 | 0,965 | 0,937 | 0,880 | 0,994 |
| IFI27_ IFI44L_ SIGLEC1_ FAM20A | 0,894 | 0,885 | 0,903 | 0,910 | 0,844 | 0,975 | 0,948 | 0,873 | 1 |
| IFI27_ ISG15_ IL1R2_ MMP8 | 0,894 | 0,887 | 0,901 | 0,913 | 0,863 | 0,963 | 0,929 | 0,869 | 0,990 |
| RSAD2_ IFI27_ OAS1_ IL1R2 | 0,894 | 0,886 | 0,901 | 0,915 | 0,865 | 0,966 | 0,939 | 0,884 | 0,994 |
| IFI27_ SIGLEC1_ ISG15_ FAM20A | 0,894 | 0,884 | 0,903 | 0,904 | 0,835 | 0,973 | 0,948 | 0,873 | 1 |
| RSAD2_ IFI27_ IL1R2_ MMP8 | 0,894 | 0,886 | 0,901 | 0,916 | 0,865 | 0,966 | 0,937 | 0,881 | 0,993 |
| IFI27_ IFIT1_ ISG15_ IL1R2 | 0,893 | 0,886 | 0,901 | 0,915 | 0,864 | 0,966 | 0,941 | 0,888 | 0,994 |
| IFI27_ IFIT1_ HERC6_ RETN | 0,893 | 0,884 | 0,902 | 0,907 | 0,844 | 0,971 | 0,941 | 0,878 | 1 |
| IFI27_ IFI44L_ FAM20A_ RETN | 0,893 | 0,883 | 0,903 | 0,910 | 0,846 | 0,974 | 0,943 | 0,866 | 1 |
| IFI27_ IFI44L_ HERC6_ RETN | 0,893 | 0,884 | 0,902 | 0,910 | 0,848 | 0,971 | 0,939 | 0,873 | 1 |
| IFIT1_ SIGLEC1_ IL1R2_ FAM20A | 0,893 | 0,884 | 0,901 | 0,913 | 0,850 | 0,975 | 0,962 | 0,914 | 1 |

| Modèle | AUC modèle | IC 95% | | AUC Train | IC 95% Train | | AUC Test | IC 95% Test | |
|---|---|---|---|---|---|---|---|---|---|
| RSAD2_ IFI27_ IFI44L_ IL1R2 | 0,893 | 0,885 | 0,900 | 0,915 | 0,865 | 0,966 | 0,935 | 0,878 | 0,991 |
| IFI27_ OAS1_ IL1R2_ MMP8 | 0,893 | 0,885 | 0,900 | 0,915 | 0,864 | 0,965 | 0,930 | 0,871 | 0,989 |
| IFI27_ OAS1_ IFI44L_ IL1R2 | 0,893 | 0,885 | 0,900 | 0,914 | 0,864 | 0,965 | 0,932 | 0,873 | 0,990 |
| IFI27_ OAS1_ SIGLEC1_ RETN | 0,893 | 0,884 | 0,901 | 0,909 | 0,848 | 0,971 | 0,953 | 0,892 | 1 |
| IFI44L_ SIGLEC1_ IL1R2_ FAM20A | 0,893 | 0,884 | 0,901 | 0,910 | 0,847 | 0,973 | 0,962 | 0,911 | 1 |
| OAS1_ SIGLEC1_ IL1R2_ FAM20A | 0,892 | 0,883 | 0,901 | 0,910 | 0,847 | 0,973 | 0,962 | 0,910 | 1 |
| IFI27_ SLC1A2_ IL1R2_ RETN | 0,892 | 0,884 | 0,900 | 0,921 | 0,873 | 0,969 | 0,916 | 0,835 | 0,998 |
| RSAD2_ SIGLEC1_ IL1R2_ FAM20A | 0,892 | 0,883 | 0,900 | 0,909 | 0,846 | 0,973 | 0,963 | 0,912 | 1 |
| SIGLEC1_ ISG15_ IL1R2_ FAM20A | 0,892 | 0,883 | 0,900 | 0,908 | 0,845 | 0,972 | 0,963 | 0,912 | 1 |
| SIGLEC1_ IL1R2_ OLAH_ FAM20A | 0,892 | 0,883 | 0,900 | 0,910 | 0,847 | 0,973 | 0,963 | 0,912 | 1 |
| IFI27_ IFIT1_ SLC1A2_ IL1R2 | 0,892 | 0,884 | 0,899 | 0,922 | 0,876 | 0,968 | 0,936 | 0,875 | 0,997 |
| IFI27_ IFIT1_ SIGLEC1_ RETN | 0,891 | 0,882 | 0,900 | 0,908 | 0,846 | 0,971 | 0,955 | 0,900 | 1 |
| IFI27_ SIGLEC1_ HERC6_ FAM20A | 0,891 | 0,882 | 0,901 | 0,905 | 0,837 | 0,973 | 0,951 | 0,876 | 1 |
| RSAD2_ IFI27_ ISG15_ IL1R2 | 0,891 | 0,884 | 0,898 | 0,916 | 0,866 | 0,966 | 0,934 | 0,876 | 0,991 |
| IFI27_ OAS1_ ISG15_ IL1R2 | 0,891 | 0,883 | 0,898 | 0,914 | 0,863 | 0,964 | 0,932 | 0,873 | 0,990 |
| RSAD2_ IFI27_ SLC1A2_ IL1R2 | 0,891 | 0,883 | 0,898 | 0,921 | 0,875 | 0,967 | 0,932 | 0,867 | 0,996 |
| SIGLEC1_ IL1R2_ FAM20A_ RETN | 0,891 | 0,882 | 0,900 | 0,907 | 0,844 | 0,971 | 0,962 | 0,907 | 1 |
| IFI27_ SIGLEC1_ FAM20A_ MMP8 | 0,890 | 0,881 | 0,900 | 0,904 | 0,836 | 0,972 | 0,946 | 0,867 | 1 |
| IFI27_ ISG15_ FAM20A_ RETN | 0,890 | 0,881 | 0,900 | 0,909 | 0,848 | 0,969 | 0,934 | 0,853 | 1 |
| RSAD2_ IFI27_ SIGLEC1_ RETN | 0,890 | 0,881 | 0,899 | 0,909 | 0,848 | 0,970 | 0,953 | 0,896 | 1 |
| IFI27_ HERC6_ SLC1A2_ IL1R2 | 0,890 | 0,882 | 0,898 | 0,920 | 0,875 | 0,966 | 0,918 | 0,845 | 0,992 |
| IFI27_ ISG15_ HERC6_ RETN | 0,890 | 0,880 | 0,899 | 0,907 | 0,844 | 0,971 | 0,951 | 0,893 | 1 |
| IFI27_ SIGLEC1_ HERC6_ RETN | 0,890 | 0,880 | 0,899 | 0,905 | 0,840 | 0,971 | 0,963 | 0,917 | 1 |
| IFI27_ IFIT1_ FAM20A_ RETN | 0,890 | 0,880 | 0,899 | 0,910 | 0,848 | 0,972 | 0,942 | 0,865 | 1 |
| IFI27_ IFI44L_ SIGLEC1_ RETN | 0,889 | 0,881 | 0,898 | 0,907 | 0,844 | 0,970 | 0,954 | 0,898 | 1 |

EP 4 365 308 A1

| Modèle | AUC modèle | IC 95% | | AUC Train | IC 95% Train | | AUC Test | IC 95% Test | |
|---|---|---|---|---|---|---|---|---|---|
| IFIT1_ IFI44L_ SIGLEC1_ FAM20A | 0,889 | 0,881 | 0,898 | 0,901 | 0,832 | 0,970 | 0,939 | 0,875 | 1 |
| SIGLEC1_ SLC1A2_ IL1R2_ FAM20A | 0,889 | 0,881 | 0,898 | 0,916 | 0,862 | 0,970 | 0,954 | 0,890 | 1 |
| IFI27_ OAS1_ SLC1A2_ IL1R2 | 0,889 | 0,881 | 0,897 | 0,922 | 0,876 | 0,967 | 0,923 | 0,853 | 0,992 |
| SIGLEC1 - SLC1A2_ OLAH_ FAM20A | 0,889 | 0,880 | 0,898 | 0,913 | 0,855 | 0,971 | 0,950 | 0,890 | 1 |
| ISG15_ HERC6_ IL1R2_ FAM20A | 0,889 | 0,880 | 0,898 | 0,907 | 0,847 | 0,966 | 0,932 | 0,849 | 1 |
| IFI27_ ISG15_ SLC1A2_ IL1R2 | 0,889 | 0,881 | 0,896 | 0,918 | 0,871 | 0,965 | 0,918 | 0,844 | 0,993 |
| IFI27_ SLC1A2_ IL1R2_ MMP8 | 0,888 | 0,881 | 0,896 | 0,919 | 0,874 | 0,965 | 0,905 | 0,822 | 0,989 |
| OAS1_ IFIT1_ SIGLEC1_ FAM20A | 0,888 | 0,879 | 0,897 | 0,899 | 0,829 | 0,970 | 0,947 | 0,880 | 1 |
| IFI27_ OAS1_ FAM20A_ RETN | 0,888 | 0,878 | 0,897 | 0,910 | 0,848 | 0,971 | 0,942 | 0,865 | 1 |
| IFI44L_ SIGLEC1_ SLC1A2_ OLAH | 0,888 | 0,879 | 0,896 | 0,912 | 0,856 | 0,967 | 0,950 | 0,902 | 0,998 |
| IFIT1_ SIGLEC1_ OLAH_ FAM20A | 0,887 | 0,878 | 0,896 | 0,901 | 0,832 | 0,970 | 0,948 | 0,893 | 1 |
| SIGLEC1_ OLAH_ FAM20A_ MMP8 | 0,887 | 0,877 | 0,897 | 0,901 | 0,832 | 0,970 | 0,965 | 0,920 | 1 |
| IFI44L_ HERC6_ IL1R2_ FAM20A | 0,887 | 0,878 | 0,896 | 0,906 | 0,845 | 0,967 | 0,936 | 0,856 | 1 |
| IFI27_ SIGLEC1_ ISG15_ RETN | 0,887 | 0,877 | 0,896 | 0,904 | 0,838 | 0,969 | 0,957 | 0,903 | 1 |
| IFIT1_ SIGLEC1_ SLC1A2_ OLAH | 0,887 | 0,878 | 0,895 | 0,913 | 0,857 | 0,968 | 0,947 | 0,897 | 0,997 |
| IFI27_ HERC6_ RETN_ MMP8 | 0,886 | 0,877 | 0,896 | 0,905 | 0,841 | 0,969 | 0,947 | 0,886 | 1 |
| HERC6_ IL1R2_ OLAH_ FAM20A | 0,886 | 0,877 | 0,895 | 0,907 | 0,847 | 0,966 | 0,930 | 0,853 | 1 |
| IFI27_ HERC6_ FAM20A_ MMP8 | 0,886 | 0,877 | 0,896 | 0,906 | 0,844 | 0,968 | 0,932 | 0,851 | 1 |
| RSAD2_ IFI27_ FAM20A_ RETN | 0,886 | 0,877 | 0,896 | 0,908 | 0,846 | 0,971 | 0,942 | 0,864 | 1 |
| OAS1_ HERC6_ IL1R2_ FAM20A | 0,886 | 0,877 | 0,895 | 0,905 | 0,843 | 0,967 | 0,930 | 0,847 | 1 |
| IFIT1_ HERC6_ IL1R2_ FAM20A | 0,886 | 0,877 | 0,895 | 0,903 | 0,840 | 0,965 | 0,936 | 0,854 | 1 |
| SIGLEC1_ SLC1A2_ OLAH_ MMP8 | 0,886 | 0,877 | 0,895 | 0,912 | 0,856 | 0,967 | 0,957 | 0,914 | 0,999 |
| OAS1_ SIGLEC1_ SLC1A2_ OLAH | 0,886 | 0,877 | 0,895 | 0,912 | 0,857 | 0,968 | 0,952 | 0,906 | 0,999 |
| RSAD2_ SIGLEC1_ SLC1A2_ OLAH | 0,886 | 0,877 | 0,894 | 0,912 | 0,856 | 0,968 | 0,953 | 0,908 | 0,998 |
| IFIT1_ SIGLEC1_ ISG15_ FAM20A | 0,886 | 0,877 | 0,894 | 0,896 | 0,826 | 0,967 | 0,939 | 0,873 | 1 |

| Modèle | AUC modèle | IC 95% | | AUC Train | IC 95% Train | | AUC Test | IC 95% Test | |
|---|---|---|---|---|---|---|---|---|---|
| IFI27_ SIGLEC1_ SLC1A2_ FAM20A | 0,886 | 0,876 | 0,895 | 0,911 | 0,848 | 0,973 | 0,950 | 0,874 | 1 |
| SIGLEC1_ ISG15_ SLC1A2_ OLAH | 0,886 | 0,877 | 0,894 | 0,912 | 0,856 | 0,967 | 0,948 | 0,899 | 0,997 |
| IFI44L_ SIGLEC1_ HERC6_ IL1R2 | 0,886 | 0,877 | 0,894 | 0,902 | 0,841 | 0,962 | 0,957 | 0,914 | 0,999 |
| IFI44L_ SLC1A2_ OLAH_ FAM20A | 0,885 | 0,876 | 0,895 | 0,908 | 0,849 | 0,968 | 0,927 | 0,853 | 1 |
| IFIT1_ SIGLEC1_ HERC6_ IL1R2 | 0,885 | 0,877 | 0,894 | 0,904 | 0,845 | 0,962 | 0,953 | 0,909 | 0,998 |
| RSAD2_ IFIT1_ SIGLEC1_ FAM20A | 0,885 | 0,876 | 0,894 | 0,899 | 0,829 | 0,969 | 0,943 | 0,879 | 1 |
| SIGLEC1_ HERC6_ OLAH_ FAM20A | 0,885 | 0,875 | 0,895 | 0,902 | 0,834 | 0,970 | 0,960 | 0,907 | 1 |
| RSAD2_ SIGLEC1_ HERC6_ IL1R2 | 0,885 | 0,876 | 0,893 | 0,903 | 0,843 | 0,962 | 0,949 | 0,902 | 0,996 |
| IFIT1_ SIGLEC1_ FAM20A_ MMP8 | 0,885 | 0,876 | 0,894 | 0,899 | 0,829 | 0,969 | 0,940 | 0,872 | 1 |
| OAS1_ SIGLEC1_ HERC6_ IL1R2 | 0,885 | 0,876 | 0,893 | 0,903 | 0,845 | 0,962 | 0,957 | 0,914 | 0,999 |
| RSAD2_ HERC6_ IL1R2_ FAM20A | 0,885 | 0,876 | 0,894 | 0,905 | 0,843 | 0,967 | 0,930 | 0,846 | 1 |
| SIGLEC1_ SLC1A2_ IL1R2_ OLAH | 0,885 | 0,876 | 0,893 | 0,913 | 0,858 | 0,968 | 0,963 | 0,925 | 1 |
| SIGLEC1_ ISG15_ OLAH_ FAM20A | 0,884 | 0,875 | 0,894 | 0,897 | 0,825 | 0,968 | 0,962 | 0,918 | 1 |
| HERC6_ IL1R2_ FAM20A_ MMP8 | 0,884 | 0,876 | 0,893 | 0,909 | 0,852 | 0,966 | 0,920 | 0,828 | 1 |
| IFI44L_ SIGLEC1_ OLAH_ FAM20A | 0,884 | 0,875 | 0,894 | 0,896 | 0,824 | 0,969 | 0,962 | 0,918 | 1 |
| SIGLEC1_ HERC6_ SLC1A2_ OLAH | 0,884 | 0,875 | 0,894 | 0,912 | 0,856 | 0,968 | 0,952 | 0,907 | 0,997 |
| IFI27_ SIGLEC1_ SLC1A2_ RETN | 0,884 | 0,875 | 0,893 | 0,909 | 0,848 | 0,971 | 0,960 | 0,907 | 1 |
| SIGLEC1_ HERC6_ IL1R2_ MMP8 | 0,884 | 0,875 | 0,892 | 0,903 | 0,844 | 0,962 | 0,964 | 0,927 | 1 |
| IFI27_ SIGLEC1_ RETN_ MMP8 | 0,884 | 0,874 | 0,893 | 0,904 | 0,840 | 0,969 | 0,962 | 0,912 | 1 |
| IFIT1_ SIGLEC1_ FAM20A_ RETN | 0,884 | 0,874 | 0,893 | 0,898 | 0,828 | 0,968 | 0,941 | 0,878 | 1 |
| HERC6_ IL1R2_ FAM20A_ RETN | 0,884 | 0,874 | 0,893 | 0,907 | 0,846 | 0,967 | 0,926 | 0,840 | 1 |
| OAS1_ SIGLEC1_ OLAH_ FAM20A | 0,883 | 0,874 | 0,893 | 0,896 | 0,824 | 0,968 | 0,962 | 0,918 | 1 |
| IFIT1_ SIGLEC1_ SLC1A2_ FAM20A | 0,883 | 0,874 | 0,892 | 0,907 | 0,847 | 0,968 | 0,938 | 0,865 | 1 |
| IFI44L_ SLC1A2_ IL1R2 _ FAM20A | 0,883 | 0,875 | 0,892 | 0,908 | 0,853 | 0,962 | 0,925 | 0,838 | 1 |
| SIGLEC1_ FAM20A_ RETN_ MMP8 | 0,883 | 0,873 | 0,893 | 0,900 | 0,829 | 0,970 | 0,958 | 0,896 | 1 |

(suite)

| Modèle | AUC modèle | IC 95% | | AUC Train | IC 95% Train | | AUC Test | IC 95% Test | |
|---|---|---|---|---|---|---|---|---|---|
| SIGLEC1_HERC6_ FAM20A_RETN | 0,883 | 0,873 | 0,893 | 0,898 | 0,827 | 0,968 | 0,955 | 0,890 | 1 |
| IFI27_ ISG15_ HERC6_ FAM20A | 0,883 | 0,873 | 0,893 | 0,901 | 0,837 | 0,965 | 0,936 | 0,856 | 1 |
| IFI27_ FAM20A_ RETN_ MMP8 | 0,883 | 0,873 | 0,894 | 0,906 | 0,844 | 0,968 | 0,923 | 0,832 | 1 |
| IFI44L_ SIGLEC1_IL1R2_ MMP8 | 0,883 | 0,875 | 0,891 | 0,902 | 0,843 | 0,961 | 0,955 | 0,912 | 0,999 |
| SIGLEC1_ SLC1A2_ OLAH_ RETN | 0,883 | 0,874 | 0,892 | 0,913 | 0,857 | 0,969 | 0,949 | 0,899 | 0,998 |
| IFI27_ IFIT1_ HERC6_ FAM20A | 0,883 | 0,873 | 0,893 | 0,903 | 0,841 | 0,966 | 0,934 | 0,851 | 1 |
| IFI27_ HERC6_ SLC1A2_ RETN | 0,883 | 0,873 | 0,892 | 0,910 | 0,849 | 0,971 | 0,942 | 0,874 | 1 |
| IFI27_ IFI44L_ ISG15_ RETN | 0,883 | 0,873 | 0,892 | 0,905 | 0,841 | 0,969 | 0,957 | 0,901 | 1 |
| SIGLEC1_ ISG15_ HERC6_ IL1R2 | 0,883 | 0,874 | 0,891 | 0,902 | 0,841 | 0,963 | 0,953 | 0,909 | 0,998 |
| RSAD2_ IFI27_ HERC6_ FAM20A | 0,883 | 0,873 | 0,892 | 0,905 | 0,844 | 0,966 | 0,928 | 0,845 | 1 |
| IFI27_ IFI44L_ HERC6_ FAM20A | 0,882 | 0,873 | 0,892 | 0,898 | 0,832 | 0,964 | 0,937 | 0,857 | 1 |
| RSAD2_ SIGLEC1_ OLAH_ FAM20A | 0,882 | 0,873 | 0,892 | 0,896 | 0,825 | 0,968 | 0,962 | 0,919 | 1 |
| IFI27_ OAS1_ IFIT1_ RETN | 0,882 | 0,873 | 0,892 | 0,905 | 0,841 | 0,968 | 0,950 | 0,888 | 1 |
| SIGLEC1_ OLAH_ FAM20A_ RETN | 0,882 | 0,872 | 0,892 | 0,896 | 0,823 | 0,968 | 0,963 | 0,920 | 1 |
| RSAD2_ SIGLEC1_ HERC6_ FAM20A | 0,882 | 0,873 | 0,891 | 0,899 | 0,834 | 0,965 | 0,941 | 0,872 | 1 |
| SIGLEC1_ HERC6_ IL1R2_ OLAH | 0,882 | 0,873 | 0,891 | 0,903 | 0,842 | 0,964 | 0,959 | 0,918 | 1,000 |
| IFI27_ OAS1_ HERC6_ FAM20A | 0,882 | 0,872 | 0,892 | 0,903 | 0,840 | 0,965 | 0,932 | 0,848 | 1 |
| IFI44L_ SIGLEC1_ SLC1A2_ IL1R2 | 0,882 | 0,874 | 0,890 | 0,907 | 0,851 | 0,963 | 0,964 | 0,927 | 1 |
| SIGLEC1_ IL1R2_ OLAH_ MMP8 | 0,882 | 0,873 | 0,890 | 0,905 | 0,847 | 0,963 | 0,963 | 0,925 | 1 |
| IFI44L_ ISG15_ IL1R2_ FAM20A | 0,882 | 0,873 | 0,890 | 0,901 | 0,839 | 0,964 | 0,941 | 0,871 | 1 |
| OAS1_ IL1R2_ OLAH_ FAM20A | 0,881 | 0,873 | 0,890 | 0,901 | 0,835 | 0,967 | 0,936 | 0,860 | 1 |
| HERC6_ SLC1A2_ IL1R2_ FAM20A | 0,881 | 0,872 | 0,891 | 0,912 | 0,860 | 0,965 | 0,916 | 0,818 | 1 |
| SIGLEC1_ SLC1A2_ IL1R2_ MMP8 | 0,881 | 0,873 | 0,889 | 0,910 | 0,855 | 0,964 | 0,966 | 0,930 | 1 |
| SIGLEC1_ ISG15_ SLC1A2_ IL1R2 | 0,881 | 0,873 | 0,889 | 0,908 | 0,853 | 0,964 | 0,964 | 0,927 | 1 |
| SIGLEC1_ HERC6_ SLC1A2_ IL1R2 | 0,881 | 0,873 | 0,890 | 0,908 | 0,853 | 0,963 | 0,961 | 0,922 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_ IFIT1_ ISG15_ RETN | 0,881 | 0,872 | 0,891 | 0,904 | 0,841 | 0,968 | 0,953 | 0,896 | 1 |
| RSAD2_ SIGLEC1_ IL1R2_ MMP8 | 0,881 | 0,873 | 0,890 | 0,903 | 0,844 | 0,962 | 0,965 | 0,929 | 1 |
| IFI44L_ SIGLEC1_ IL1R2_ OLAH | 0,881 | 0,873 | 0,890 | 0,901 | 0,840 | 0,962 | 0,958 | 0,915 | 1 |
| IFI27_ SLC1A2_ FAM20A_ RETN | 0,881 | 0,871 | 0,891 | 0,899 | 0,835 | 0,964 | 0,913 | 0,817 | 1 |
| IFIT1_ IL1R2_ FAM20A_ MMP8 | 0,881 | 0,872 | 0,890 | 0,904 | 0,841 | 0,967 | 0,941 | 0,867 | 1 |
| IFI44L_ SIGLEC1_ ISG15_ IL1R2 | 0,881 | 0,873 | 0,889 | 0,900 | 0,839 | 0,961 | 0,960 | 0,919 | 1 |
| OAS1_ IFIT1_ SIGLEC1_ IL1R2 | 0,881 | 0,873 | 0,890 | 0,899 | 0,836 | 0,961 | 0,957 | 0,915 | 0,998 |
| IFI27_ IFI44L_ FAM20A_ MMP8 | 0,881 | 0,871 | 0,891 | 0,899 | 0,832 | 0,966 | 0,935 | 0,856 | 1 |
| IFIT1_ SIGLEC1_ SLC1A2_ IL1R2 | 0,881 | 0,873 | 0,889 | 0,908 | 0,853 | 0,963 | 0,964 | 0,927 | 1 |
| SIGLEC1_ SLC1A2_ IL1R2_ RETN | 0,881 | 0,873 | 0,889 | 0,910 | 0,855 | 0,965 | 0,962 | 0,922 | 1 |
| IFI27_ OAS1_ IFI44L_ RETN | 0,881 | 0,871 | 0,890 | 0,906 | 0,843 | 0,970 | 0,954 | 0,896 | 1 |
| OAS1_ SIGLEC1_ IL1R2_ MMP8 | 0,881 | 0,872 | 0,889 | 0,902 | 0,843 | 0,962 | 0,963 | 0,925 | 1 |
| OAS1_ IFIT1_ IL1R2_ FAM20A | 0,881 | 0,872 | 0,890 | 0,901 | 0,836 | 0,967 | 0,942 | 0,868 | 1 |
| RSAD2_ SIGLEC1_ SLC1A2_ IL1R2 | 0,881 | 0,872 | 0,889 | 0,909 | 0,853 | 0,964 | 0,965 | 0,929 | 1 |
| IFIT1_ SIGLEC1_ HERC6_ OLAH | 0,881 | 0,872 | 0,890 | 0,900 | 0,838 | 0,962 | 0,938 | 0,885 | 0,992 |
| HERC6_ SLC1A2_ OLAH_ FAM20A | 0,881 | 0,871 | 0,890 | 0,911 | 0,856 | 0,967 | 0,929 | 0,849 | 1 |
| RSAD2_ OAS1_ SIGLEC1_ FAM20A | 0,881 | 0,871 | 0,890 | 0,897 | 0,826 | 0,968 | 0,952 | 0,889 | 1 |
| OAS1_ IL1R2_ FAM20A_ MMP8 | 0,881 | 0,872 | 0,889 | 0,903 | 0,841 | 0,966 | 0,934 | 0,853 | 1 |
| SIGLEC1_ ISG15_ FAM20A_ RETN | 0,881 | 0,871 | 0,890 | 0,893 | 0,821 | 0,966 | 0,954 | 0,895 | 1 |
| SIGLEC1_ ISG15_ IL1R2_ MMP8 | 0,881 | 0,873 | 0,889 | 0,903 | 0,844 | 0,962 | 0,962 | 0,923 | 1 |
| IFI27_ IFI44L_ RETN_ MMP8 | 0,880 | 0,871 | 0,890 | 0,905 | 0,842 | 0,969 | 0,950 | 0,892 | 1 |
| IFI44L_ SIGLEC1_ SLC1A2_ FAM20A | 0,880 | 0,871 | 0,890 | 0,901 | 0,837 | 0,966 | 0,949 | 0,877 | 1 |
| IFI44L_ SLC1A2_ OLAH_ MMP8 | 0,880 | 0,871 | 0,889 | 0,906 | 0,848 | 0,964 | 0,937 | 0,882 | 0,991 |
| SIGLEC1_ SLC1A2_ FAM20A_ MMP8 | 0,880 | 0,870 | 0,890 | 0,902 | 0,838 | 0,966 | 0,951 | 0,877 | 1 |
| IFIT1_ SIGLEC1_ IL1R2_ MMP8 | 0,880 | 0,872 | 0,889 | 0,901 | 0,842 | 0,960 | 0,962 | 0,923 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1_ SIGLEC1_ HERC6_ RETN | 0,880 | 0,871 | 0,890 | 0,893 | 0,823 | 0,963 | 0,925 | 0,865 | 0,985 |
| IFI44L_ SIGLEC1_ HERC6_ FAM20A | 0,880 | 0,870 | 0,890 | 0,899 | 0,832 | 0,967 | 0,952 | 0,881 | 1 |
| SIGLEC1_ ISG15_ IL1R2_ OLAH | 0,880 | 0,872 | 0,888 | 0,902 | 0,841 | 0,963 | 0,961 | 0,920 | 1 |
| SIGLEC1_ ISG15_ SLC1A2_ FAM20A | 0,880 | 0,871 | 0,889 | 0,901 | 0,837 | 0,965 | 0,949 | 0,877 | 1 |
| OAS1_ IFI44L_ SIGLEC1_ IL1R2 | 0,880 | 0,871 | 0,889 | 0,899 | 0,836 | 0,963 | 0,957 | 0,915 | 0,998 |
| RSAD2_ OAS1_ IL1R2_ FAM20A | 0,880 | 0,871 | 0,889 | 0,898 | 0,832 | 0,965 | 0,936 | 0,858 | 1 |
| OAS1_ SIGLEC1_ IL1R2_ OLAH | 0,880 | 0,871 | 0,889 | 0,900 | 0,837 | 0,963 | 0,955 | 0,912 | 0,999 |
| IFI44L_ IL1R2_ FAM20A_ MMP8 | 0,880 | 0,871 | 0,888 | 0,901 | 0,840 | 0,963 | 0,936 | 0,855 | 1 |
| IFI27_ IFIT1_ IFI44L_ RETN | 0,880 | 0,870 | 0,890 | 0,903 | 0,839 | 0,968 | 0,952 | 0,892 | 1 |
| OAS1_ SLC1A2_ OLAH_ FAM20A | 0,880 | 0,870 | 0,889 | 0,912 | 0,855 | 0,970 | 0,939 | 0,868 | 1 |
| OAS1_ SIGLEC1_ SLC1A2_ IL1R2 | 0,880 | 0,871 | 0,888 | 0,908 | 0,853 | 0,964 | 0,964 | 0,927 | 1 |
| SIGLEC1_ HERC6_ IL1R2 RETN | 0,880 | 0,870 | 0,889 | 0,897 | 0,831 | 0,964 | 0,960 | 0,919 | 1 |
| IFI44L_ SLC1A2_ IL1R2_ OLAH | 0,880 | 0,871 | 0,889 | 0,907 | 0,851 | 0,963 | 0,929 | 0,868 | 0,991 |
| SIGLEC1_ SLC1A2_ FAM20A_ RETN | 0,880 | 0,870 | 0,889 | 0,902 | 0,838 | 0,966 | 0,948 | 0,875 | 1 |
| RSAD2_ IFI27_ IFIT1_ RETN | 0,880 | 0,870 | 0,889 | 0,905 | 0,842 | 0,969 | 0,951 | 0,891 | 1 |
| RSAD2_ SIGLEC1_ SLC1A2_ FAM20A | 0,880 | 0,870 | 0,889 | 0,901 | 0,838 | 0,964 | 0,947 | 0,877 | 1 |
| SIGLEC1_ HERC6_ SLC1A2_ FAM20A | 0,880 | 0,870 | 0,889 | 0,901 | 0,837 | 0,964 | 0,954 | 0,881 | 1 |
| HERC6_ OLAH_ FAM20A_ RETN | 0,879 | 0,870 | 0,889 | 0,903 | 0,839 | 0,967 | 0,945 | 0,879 | 1 |
| RSAD2_ OAS1_ SIGLEC1_ IL1R2 | 0,879 | 0,871 | 0,888 | 0,895 | 0,831 | 0,959 | 0,962 | 0,924 | 1 |
| IFI27_ OAS1_ HERC6_ MMP8 | 0,879 | 0,871 | 0,888 | 0,899 | 0,840 | 0,957 | 0,891 | 0,809 | 0,973 |
| IFI44L_ SIGLEC1_ FAM20A_ RETN | 0,879 | 0,870 | 0,889 | 0,894 | 0,823 | 0,966 | 0,955 | 0,898 | 1 |
| IFI27_ CAS1_ ISG15_ RETN | 0,879 | 0,870 | 0,889 | 0,905 | 0,843 | 0,968 | 0,952 | 0,896 | 1 |
| SIGLEC1_ IL1R2_ RETN_ MMP8 | 0,879 | 0,870 | 0,888 | 0,899 | 0,835 | 0,963 | 0,968 | 0,935 | 1 |
| IFIT1_ HERC6_ OLAH_ FAM20A | 0,879 | 0,869 | 0,889 | 0,899 | 0,835 | 0,964 | 0,941 | 0,872 | 1 |
| IFIT1_ SIGLEC1_ IL1R2_ OLAH | 0,879 | 0,871 | 0,888 | 0,901 | 0,841 | 0,962 | 0,960 | 0,918 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L_ IL1R2_ OLAH_ FAM20A | 0,879 | 0,870 | 0,888 | 0,897 | 0,832 | 0,962 | 0,951 | 0,896 | 1 |
| HERC6_ OLAH_ FAM20A_ MMP8 | 0,879 | 0,869 | 0,889 | 0,899 | 0,835 | 0,963 | 0,942 | 0,875 | 1 |
| OAS1_ IFI44L_ IL1R2_ FAM20A | 0,879 | 0,870 | 0,888 | 0,900 | 0,834 | 0,965 | 0,940 | 0,866 | 1 |
| RSAD2_ IFI27_ IFI44L_ RETN | 0,879 | 0,869 | 0,888 | 0,906 | 0,842 | 0,970 | 0,957 | 0,902 | 1 |
| IFIT1_ SIGLEC1_ ISG15_ IL1R2 | 0,879 | 0,870 | 0,887 | 0,900 | 0,840 | 0,961 | 0,961 | 0,921 | 1 |
| IFI27_ IFIT1_ RETN_ MMP8 | 0,879 | 0,869 | 0,889 | 0,904 | 0,840 | 0,968 | 0,950 | 0,889 | 1 |
| IFIT1_ ISG15_ IL1R2_ FAM20A | 0,879 | 0,870 | 0,888 | 0,903 | 0,840 | 0,965 | 0,951 | 0,887 | 1 |
| IFI27_ IFI44L_ SLC1A2_ RETN | 0,879 | 0,869 | 0,888 | 0,903 | 0,840 | 0,967 | 0,946 | 0,880 | 1 |
| RSAD2_ IFI27_ OAS1_ RETN | 0,879 | 0,869 | 0,888 | 0,901 | 0,836 | 0,966 | 0,950 | 0,889 | 1 |
| IFI44L_ SLC1A2_ OLAH_ RETN | 0,879 | 0,869 | 0,888 | 0,907 | 0,848 | 0,965 | 0,929 | 0,870 | 0,988 |
| ISG15_ HERC6_ OLAH_ FAM20A | 0,879 | 0,869 | 0,888 | 0,899 | 0,835 | 0,964 | 0,946 | 0,881 | 1 |
| OAS1_ SIGLEC1_ HERC6_ FAM20A | 0,879 | 0,869 | 0,888 | 0,896 | 0,829 | 0,964 | 0,949 | 0,885 | 1 |
| RSAD2_ SIGLEC1_ IL1R2_ OLAH | 0,879 | 0,869 | 0,888 | 0,899 | 0,836 | 0,963 | 0,960 | 0,919 | 1 |
| ISG15_ SLC1A2_ IL1R2_ FAM20A | 0,879 | 0,870 | 0,887 | 0,905 | 0,849 | 0,962 | 0,916 | 0,826 | 1 |
| OAS1_ SIGLEC1_ FAM20A_ RETN | 0,879 | 0,869 | 0,888 | 0,894 | 0,822 | 0,966 | 0,955 | 0,898 | 1 |
| IFI44L_ HERC6_ OLAH_ FAM20A | 0,879 | 0,869 | 0,888 | 0,900 | 0,834 | 0,965 | 0,947 | 0,882 | 1 |
| IFI27_ ISG15_ RETN_ MMP8 | 0,879 | 0,869 | 0,888 | 0,903 | 0,840 | 0,965 | 0,945 | 0,879 | 1 |
| RSAD2_ IFIT1_ SIGLEC1_ IL1R2 | 0,878 | 0,870 | 0,887 | 0,897 | 0,835 | 0,960 | 0,961 | 0,922 | 1 |
| RSAD2_ SLC1A2_ OLAH_ FAM20A | 0,878 | 0,869 | 0,888 | 0,908 | 0,849 | 0,966 | 0,932 | 0,858 | 1 |
| IFI44L_ SIGLEC1_ ISG15_ FAM20A | 0,878 | 0,869 | 0,888 | 0,893 | 0,821 | 0,965 | 0,958 | 0,898 | 1 |
| IFI44L_ SIGLEC1_ IL1R2_ RETN | 0,878 | 0,870 | 0,887 | 0,897 | 0,833 | 0,961 | 0,959 | 0,916 | 1 |
| RSAD2_ IFI44L_ SIGLEC1_ IL1R2 | 0,878 | 0,870 | 0,887 | 0,896 | 0,831 | 0,961 | 0,960 | 0,920 | 1,000 |
| IFIT1_ IFI44L_ SIGLEC1_ IL1R2 | 0,878 | 0,870 | 0,887 | 0,897 | 0,834 | 0,960 | 0,958 | 0,916 | 0,999 |
| IFI44L_ ISG15_ SLC1A2_ OLAH | 0,878 | 0,869 | 0,887 | 0,906 | 0,848 | 0,963 | 0,933 | 0,876 | 0,989 |
| OAS1_ ISG15_ IL1R2_ FAM20A | 0,878 | 0,869 | 0,887 | 0,896 | 0,829 | 0,962 | 0,936 | 0,857 | 1 |

| Modèle | AUC modèle | IC 95% | | AUC Train | IC 95% Train | | AUC Test | IC 95% Test | |
|---|---|---|---|---|---|---|---|---|---|
| OAS1_ SIGLEC1_ SLC1A2_ FAM20A | 0,878 | 0,869 | 0,888 | 0,901 | 0,838 | 0,965 | 0,948 | 0,877 | 1 |
| IFIT1_ SLC1A2_ IL1R2_ FAM20A | 0,878 | 0,869 | 0,887 | 0,904 | 0,846 | 0,962 | 0,937 | 0,860 | 1 |
| IFI44L_ SIGLEC1_ FAM20A_ MMP8 | 0,878 | 0,868 | 0,888 | 0,892 | 0,820 | 0,964 | 0,957 | 0,895 | 1 |
| SIGLEC1_ ISG15_ HERC6_ FAM20A | 0,878 | 0,868 | 0,888 | 0,892 | 0,820 | 0,964 | 0,952 | 0,879 | 1 |
| ISG15_ IL1R2_ OLAH_ FAM20A | 0,878 | 0,869 | 0,887 | 0,901 | 0,839 | 0,963 | 0,927 | 0,851 | 1 |
| SIGLEC1_ ISG15_ FAM20A_ MMP8 | 0,878 | 0,868 | 0,888 | 0,889 | 0,816 | 0,963 | 0,958 | 0,896 | 1 |
| RSAD2_ SIGLEC1_ ISG15_ IL1R2 | 0,878 | 0,869 | 0,886 | 0,897 | 0,834 | 0,960 | 0,962 | 0,924 | 1 |
| SIGLEC1_ ISG15_ IL1R2_ RETN | 0,878 | 0,869 | 0,886 | 0,898 | 0,833 | 0,963 | 0,965 | 0,926 | 1 |
| SIGLEC1_ IL1R2_ OLAH_ RETN | 0,878 | 0,869 | 0,887 | 0,898 | 0,833 | 0,963 | 0,960 | 0,918 | 1 |
| RSAD2_ IFI27_ IFI44L_ FAM20A | 0,878 | 0,867 | 0,888 | 0,899 | 0,830 | 0,968 | 0,942 | 0,867 | 1 |
| RSAD2_ IL1R2_ OLAH_ FAM20A | 0,877 | 0,868 | 0,887 | 0,900 | 0,834 | 0,967 | 0,947 | 0,879 | 1 |
| OAS1_ SIGLEC1_ ISG15_ IL1R2 | 0,877 | 0,869 | 0,886 | 0,898 | 0,835 | 0,960 | 0,961 | 0,922 | 1,000 |
| OAS1_ IFIT1_ SIGLEC1_ OLAH | 0,877 | 0,868 | 0,886 | 0,894 | 0,826 | 0,963 | 0,942 | 0,889 | 0,996 |
| RSAD2_ SIGLEC1_ FAM20A_ RETN | 0,877 | 0,868 | 0,887 | 0,894 | 0,823 | 0,965 | 0,953 | 0,895 | 1 |
| RSAD2_ SIGLEC1_ FAM20A_ MMP8 | 0,877 | 0,868 | 0,887 | 0,893 | 0,822 | 0,964 | 0,952 | 0,887 | 1 |
| IFI27_ IFIT1_ HERC6_ MMP8 | 0,877 | 0,868 | 0,886 | 0,897 | 0,837 | 0,957 | 0,892 | 0,810 | 0,975 |
| SIGLEC1_ HERC6_ FAM20A_ MMP8 | 0,877 | 0,867 | 0,887 | 0,893 | 0,821 | 0,965 | 0,952 | 0,879 | 1 |
| RSAD2_ IFI27_ ISG15_ RETN | 0,877 | 0,868 | 0,887 | 0,904 | 0,840 | 0,968 | 0,950 | 0,890 | 1 |
| IFIT1_ IFI44L_ SLC1A2_ OLAH | 0,877 | 0,868 | 0,886 | 0,907 | 0,849 | 0,964 | 0,934 | 0,878 | 0,990 |
| RSAD2_ HERC6_ OLAH_ FAM20A | 0,877 | 0,868 | 0,887 | 0,900 | 0,836 | 0,964 | 0,942 | 0,875 | 1 |
| IFIT1_ SIGLEC1_ IL1R2_ RETN | 0,877 | 0,868 | 0,886 | 0,897 | 0,833 | 0,961 | 0,958 | 0,914 | 1 |
| IFI27_ IFIT1_ SLC1A2_ RETN | 0,877 | 0,867 | 0,886 | 0,902 | 0,838 | 0,966 | 0,945 | 0,875 | 1 |
| OAS1_ HERC6_ OLAH_ FAM20A | 0,877 | 0,867 | 0,886 | 0,898 | 0,833 | 0,963 | 0,942 | 0,875 | 1 |
| IFI27_ IFI44L_ ISG15_ FAM20A | 0,877 | 0,867 | 0,886 | 0,894 | 0,824 | 0,963 | 0,942 | 0,863 | 1 |
| OAS1_ IL1R2_ FAM20A_ RETN | 0,877 | 0,867 | 0,886 | 0,899 | 0,833 | 0,965 | 0,933 | 0,853 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1_ SLC1A2_ IL1R2_ FAM20A | 0,877 | 0,868 | 0,885 | 0,905 | 0,848 | 0,962 | 0,926 | 0,839 | 1 |
| RSAD2_ SIGLEC1_ ISG15_ FAM20A | 0,876 | 0,867 | 0,886 | 0,895 | 0,824 | 0,965 | 0,951 | 0,889 | 1 |
| IFI44L_ SIGLEC1_ HERC6_ OLAH | 0,876 | 0,867 | 0,886 | 0,894 | 0,828 | 0,960 | 0,952 | 0,907 | 0,997 |
| IFIT1_ SLC1A2_ OLAH_ FAM20A | 0,876 | 0,867 | 0,886 | 0,903 | 0,842 | 0,964 | 0,938 | 0,868 | 1 |
| RSAD2_ SLC1A2_ IL1R2_ FAM20A | 0,876 | 0,868 | 0,885 | 0,901 | 0,843 | 0,958 | 0,930 | 0,842 | 1 |
| OAS1_ SIGLEC1_ FAM20A_ MMP8 | 0,876 | 0,866 | 0,886 | 0,892 | 0,821 | 0,964 | 0,955 | 0,893 | 1 |
| OAS1_ SIGLEC1_ IL1R2_ RETN | 0,876 | 0,867 | 0,885 | 0,897 | 0,831 | 0,963 | 0,957 | 0,913 | 1,000 |
| RSAD2_ ISG15_ IL1R2_ FAM20A | 0,876 | 0,867 | 0,885 | 0,897 | 0,832 | 0,963 | 0,941 | 0,867 | 1 |
| OAS1_ IFIT1_ HERC6_ IL1R2 | 0,876 | 0,867 | 0,885 | 0,895 | 0,833 | 0,958 | 0,941 | 0,889 | 0,993 |
| IFI27_ HERC6_ SLC1A2_ FAM20A | 0,876 | 0,866 | 0,886 | 0,904 | 0,846 | 0,963 | 0,933 | 0,850 | 1 |
| IFI27_ ISG15_ SLC1A2_ RETN | 0,876 | 0,867 | 0,886 | 0,898 | 0,834 | 0,962 | 0,936 | 0,860 | 1 |
| RSAD2_ IFI27_ HERC6_ MMP8 | 0,876 | 0,867 | 0,885 | 0,896 | 0,837 | 0,956 | 0,893 | 0,813 | 0,974 |
| OAS1_ IFI44L_ SIGLEC1_ FAM20A | 0,876 | 0,866 | 0,886 | 0,893 | 0,820 | 0,965 | 0,955 | 0,895 | 1 |
| IFI44L_ HERC6_ SLC1A2_ OLAH | 0,876 | 0,866 | 0,885 | 0,906 | 0,848 | 0,963 | 0,939 | 0,885 | 0,994 |
| IFIT1_ SLC1A2_ OLAH_ MMP8 | 0,876 | 0,867 | 0,885 | 0,904 | 0,846 | 0,962 | 0,946 | 0,897 | 0,995 |
| IFIT1_ IFI44L_ IL1R2_ FAM20A | 0,876 | 0,867 | 0,885 | 0,896 | 0,831 | 0,962 | 0,949 | 0,883 | 1 |
| RSAD2_ IFI27_ SLC1A2_ RETN | 0,876 | 0,866 | 0,885 | 0,899 | 0,834 | 0,964 | 0,938 | 0,864 | 1 |
| RSAD2_ SIGLEC1_ IL1R2_ RETN | 0,876 | 0,866 | 0,885 | 0,896 | 0,830 | 0,962 | 0,963 | 0,923 | 1 |
| RSAD2_ IL1R2_ FAM20A_ MMP8 | 0,876 | 0,867 | 0,884 | 0,898 | 0,834 | 0,961 | 0,935 | 0,852 | 1 |
| IFI27_ OAS1_ SLC1A2_ RETN | 0,876 | 0,866 | 0,885 | 0,898 | 0,833 | 0,963 | 0,938 | 0,865 | 1 |
| OAS1_ SIGLEC1_ ISG15_ FAM20A | 0,876 | 0,866 | 0,886 | 0,891 | 0,819 | 0,963 | 0,955 | 0,895 | 1 |
| OAS1_ SIGLEC1_ HERC6_ RETN | 0,875 | 0,866 | 0,885 | 0,891 | 0,820 | 0,963 | 0,947 | 0,897 | 0,997 |
| IFI27_ IFI44L_ HERC6_ MMP8 | 0,875 | 0,867 | 0,884 | 0,893 | 0,831 | 0,955 | 0,892 | 0,811 | 0,974 |
| RSAD2_ IFI44L_ SIGLEC1_ FAM20A | 0,875 | 0,866 | 0,885 | 0,893 | 0,822 | 0,964 | 0,950 | 0,889 | 1 |
| RSAD2_ IFIT1_ IL1R2_ FAM20A | 0,875 | 0,866 | 0,884 | 0,898 | 0,832 | 0,964 | 0,948 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1_ SLC1A2_ IL1R2_ OLAH | 0,875 | 0,867 | 0,884 | 0,908 | 0,851 | 0,965 | 0,945 | 0,894 | 0,995 |
| IFIT1_ HERC6_ IL1R2_ MMP8 | 0,875 | 0,867 | 0,884 | 0,897 | 0,835 | 0,958 | 0,949 | 0,902 | 0,996 |
| IFI27_ IFIT1_ IFI44L_ FAM20A | 0,875 | 0,865 | 0,885 | 0,895 | 0,826 | 0,964 | 0,939 | 0,860 | 1 |
| ISG15_ HERC6_ FAM20A_ RETN | 0,875 | 0,865 | 0,885 | 0,895 | 0,827 | 0,964 | 0,942 | 0,867 | 1 |
| IFI27_ IFI44L_ SLC1A2_ FAM20A | 0,875 | 0,865 | 0,885 | 0,897 | 0,831 | 0,963 | 0,943 | 0,866 | 1 |
| IFI44L_ ISG15_ HERC6_ IL1R2 | 0,875 | 0,867 | 0,883 | 0,896 | 0,838 | 0,955 | 0,943 | 0,892 | 0,995 |
| IFIT1_ ISG15_ HERC6_ IL1R2 | 0,875 | 0,866 | 0,883 | 0,897 | 0,837 | 0,957 | 0,947 | 0,897 | 0,996 |
| ISG15_ IL1R2_ FAM20A_ MMP8 | 0,875 | 0,866 | 0,883 | 0,901 | 0,842 | 0,959 | 0,917 | 0,831 | 1 |
| IFI27_ IFIT1_ FAM20A_ MMP8 | 0,875 | 0,864 | 0,885 | 0,896 | 0,829 | 0,963 | 0,934 | 0,853 | 1 |
| IFI27_ OAS1_ RETN_ MMP8 | 0,875 | 0,865 | 0,885 | 0,900 | 0,836 | 0,964 | 0,940 | 0,878 | 1 |
| IFIT1_ IL1R2_ OLAH_ FAM20A | 0,875 | 0,865 | 0,884 | 0,896 | 0,830 | 0,962 | 0,952 | 0,895 | 1 |
| RSAD2_ IFI27_ RETN_ MMP8 | 0,875 | 0,865 | 0,884 | 0,899 | 0,833 | 0,964 | 0,942 | 0,879 | 1 |
| IFIT1_ IFI44L_ HERC6_ IL1R2 | 0,875 | 0,866 | 0,883 | 0,896 | 0,834 | 0,959 | 0,942 | 0,891 | 0,994 |
| OAS1_ SIGLEC1_ HERC6_ OLAH | 0,875 | 0,865 | 0,884 | 0,893 | 0,828 | 0,957 | 0,953 | 0,909 | 0,998 |
| OAS1_ IFIT1_ SLC1A2_ OLAH | 0,874 | 0,865 | 0,884 | 0,905 | 0,848 | 0,963 | 0,941 | 0,889 | 0,993 |
| IFI44L_ IL1R2_ FAM20A_ RETN | 0,874 | 0,865 | 0,883 | 0,895 | 0,831 | 0,959 | 0,939 | 0,866 | 1 |
| IFIT1_ HERC6_ IL1R2_ RETN | 0,874 | 0,865 | 0,884 | 0,893 | 0,826 | 0,960 | 0,948 | 0,900 | 0,996 |
| IFIT1_ IL1R2_ FAM20A_ RETN | 0,874 | 0,865 | 0,884 | 0,897 | 0,833 | 0,962 | 0,947 | 0,877 | 1 |
| ISG15_ HERC6_ IL1R2_ RETN | 0,874 | 0,865 | 0,884 | 0,896 | 0,832 | 0,960 | 0,948 | 0,898 | 0,997 |
| OAS1_ HERC6_ SLC1A2_ OLAH | 0,874 | 0,865 | 0,884 | 0,902 | 0,844 | 0,960 | 0,936 | 0,879 | 0,993 |
| RSAD2_ SIGLEC1_ HERC6_ OLAH | 0,874 | 0,865 | 0,884 | 0,897 | 0,834 | 0,960 | 0,948 | 0,900 | 0,996 |
| IFIT1_ HERC6_ SLC1A2_ OLAH | 0,874 | 0,865 | 0,884 | 0,903 | 0,845 | 0,961 | 0,941 | 0,887 | 0,996 |
| RSAD2_ IFI44L_ IL1R2_ FAM20A | 0,874 | 0,866 | 0,883 | 0,895 | 0,830 | 0,961 | 0,946 | 0,876 | 1 |
| IFI44L_ HERC6_ IL1R2_ OLAH | 0,874 | 0,865 | 0,883 | 0,899 | 0,839 | 0,958 | 0,948 | 0,899 | 0,996 |
| IFI44L_ HERC6_ IL1R2_ RETN | 0,874 | 0,865 | 0,883 | 0,894 | 0,827 | 0,960 | 0,942 | 0,891 | 0,994 |

| Modèle | AUC modèle | IC 95% | | AUC Train | IC 95% Train | | AUC Test | IC 95% Test | |
|---|---|---|---|---|---|---|---|---|---|
| RSAD2_ IFIT1_ HERC6_ IL1R2 | 0,874 | 0,865 | 0,883 | 0,895 | 0,833 | 0,957 | 0,941 | 0,889 | 0,993 |
| IFIT1_ HERC6_ SLC1A2_ IL1R2 | 0,874 | 0,865 | 0,883 | 0,901 | 0,844 | 0,959 | 0,946 | 0,893 | 0,998 |
| RSAD2_ HERC6_ SLC1A2_ OLAH | 0,874 | 0,865 | 0,883 | 0,901 | 0,843 | 0,960 | 0,938 | 0,882 | 0,994 |
| IFI44L_ SIGLEC1_ OLAH_ MMP8 | 0,874 | 0,865 | 0,883 | 0,892 | 0,824 | 0,959 | 0,954 | 0,909 | 0,999 |
| ISG15_ SLC1A2_ OLAH_ FAM20A | 0,874 | 0,865 | 0,883 | 0,901 | 0,842 | 0,961 | 0,912 | 0,830 | 0,994 |
| SIGLEC1_ HERC6_ OLAH_ MMP8 | 0,874 | 0,864 | 0,884 | 0,893 | 0,826 | 0,961 | 0,957 | 0,914 | 0,999 |
| ISG15_ HERC6_ IL1R2_ MMP8 | 0,874 | 0,865 | 0,883 | 0,897 | 0,838 | 0,955 | 0,941 | 0,889 | 0,994 |
| OAS1_ IFI44L_ SLC1A2_ OLAH | 0,874 | 0,864 | 0,884 | 0,907 | 0,850 | 0,964 | 0,934 | 0,878 | 0,990 |
| ISG15_ HERC6_ IL1R2_ OLAH | 0,874 | 0,865 | 0,883 | 0,899 | 0,838 | 0,959 | 0,943 | 0,893 | 0,994 |
| RSAD2_ SIGLEC1_ HERC6_ RETN | 0,874 | 0,864 | 0,883 | 0,891 | 0,820 | 0,962 | 0,936 | 0,882 | 0,990 |
| SIGLEC1_ HERC6_ OLAH_ RETN | 0,874 | 0,864 | 0,884 | 0,893 | 0,821 | 0,964 | 0,957 | 0,914 | 0,999 |
| IFIT1_ ISG15_ SLC1A2_ OLAH | 0,874 | 0,865 | 0,883 | 0,903 | 0,844 | 0,962 | 0,945 | 0,895 | 0,995 |
| IFI27_ ISG15_ FAM20A_ MMP8 | 0,874 | 0,864 | 0,884 | 0,892 | 0,824 | 0,961 | 0,927 | 0,846 | 1 |
| ISG15_ HERC6_ SLC1A2_ OLAH | 0,874 | 0,864 | 0,883 | 0,902 | 0,844 | 0,960 | 0,940 | 0,886 | 0,995 |
| IFI27_ IFI44L_ SIGLEC1_ MMP8 | 0,874 | 0,864 | 0,883 | 0,890 | 0,824 | 0,957 | 0,920 | 0,849 | 0,990 |
| RSAD2_ OAS1_ OLAH_ FAM20A | 0,873 | 0,864 | 0,883 | 0,893 | 0,822 | 0,965 | 0,946 | 0,881 | 1 |
| IFI27_ SIGLEC1_ SLC1A2_ MMP8 | 0,873 | 0,864 | 0,882 | 0,899 | 0,838 | 0,959 | 0,935 | 0,870 | 0,999 |
| RSAD2_ ISG15_ HERC6_ IL1R2 | 0,873 | 0,865 | 0,882 | 0,896 | 0,838 | 0,955 | 0,943 | 0,892 | 0,995 |
| IFIT1_ SIGLEC1_ OLAH_ MMP8 | 0,873 | 0,864 | 0,883 | 0,892 | 0,825 | 0,958 | 0,947 | 0,897 | 0,997 |
| IFI44L_ HERC6_ IL1R2_ MMP8 | 0,873 | 0,865 | 0,882 | 0,897 | 0,837 | 0,956 | 0,940 | 0,887 | 0,993 |
| OAS1_ IFIT1_ OLAH_ FAM20A | 0,873 | 0,863 | 0,883 | 0,891 | 0,819 | 0,963 | 0,949 | 0,886 | 1 |
| RSAD2_ IFI44L_ SLC1A2_ OLAH | 0,873 | 0,864 | 0,882 | 0,906 | 0,849 | 0,964 | 0,934 | 0,878 | 0,990 |
| RSAD2_ SLC1A2_ IL1R2_ OLAH | 0,873 | 0,864 | 0,882 | 0,904 | 0,846 | 0,962 | 0,937 | 0,878 | 0,996 |
| IFIT1_ HERC6_ IL1R2_ OLAH | 0,873 | 0,864 | 0,882 | 0,899 | 0,838 | 0,960 | 0,946 | 0,896 | 0,995 |
| OAS1_ ISG15_ HERC6_ IL1R2 | 0,873 | 0,864 | 0,882 | 0,896 | 0,837 | 0,954 | 0,941 | 0,888 | 0,995 |

EP 4 365 308 A1

65

(suite)

| Modèle | AUC modèle | IC 95% | | AUC Train | IC 95% Train | | AUC Test | IC 95% Test | |
|---|---|---|---|---|---|---|---|---|---|
| IFI27_ OAS1_ FAM20A_ MMP8 | 0,873 | 0,863 | 0,883 | 0,893 | 0,825 | 0,962 | 0,935 | 0,854 | 1 |
| RSAD2_ HERC6_ IL1R2_ OLAH | 0,873 | 0,864 | 0,882 | 0,898 | 0,838 | 0,959 | 0,945 | 0,894 | 0,995 |
| IFI27_ ISG15_ HERC6_ MMP8 | 0,873 | 0,864 | 0,882 | 0,891 | 0,826 | 0,956 | 0,907 | 0,830 | 0,983 |
| IFI27_ IFIT1_ SIGLEC1_ MMP8 | 0,873 | 0,863 | 0,882 | 0,888 | 0,820 | 0,955 | 0,911 | 0,835 | 0,987 |
| OAS1_ HERC6_ IL1R2_ OLAH | 0,873 | 0,863 | 0,882 | 0,900 | 0,840 | 0,960 | 0,937 | 0,881 | 0,993 |
| ISG15_ IL1R2_ FAM20A_ RETN | 0,873 | 0,864 | 0,882 | 0,900 | 0,838 | 0,961 | 0,925 | 0,842 | 1 |
| RSAD2_ OAS1_ HERC6_ IL1R2 | 0,873 | 0,864 | 0,882 | 0,894 | 0,832 | 0,955 | 0,943 | 0,892 | 0,995 |
| SIGLEC1_ OLAH_ RETN_ MMP8 | 0,873 | 0,863 | 0,883 | 0,892 | 0,821 | 0,962 | 0,964 | 0,925 | 1 |
| HERC6_ SLC1A2_ IL1R2_ OLAH | 0,873 | 0,864 | 0,881 | 0,902 | 0,845 | 0,959 | 0,932 | 0,869 | 0,994 |
| HERC6_ SLC1A2_ OLAH_ MMP8 | 0,872 | 0,863 | 0,882 | 0,902 | 0,844 | 0,960 | 0,936 | 0,878 | 0,993 |
| IFIT1_ SLC1A2_ OLAH_ RETN | 0,872 | 0,863 | 0,882 | 0,906 | 0,847 | 0,965 | 0,948 | 0,899 | 0,996 |
| IFIT1_ SLC1A2_ IL1R2_ MMP8 | 0,872 | 0,864 | 0,881 | 0,904 | 0,847 | 0,960 | 0,953 | 0,908 | 0,999 |
| RSAD2_ IFIT1_ SIGLEC1_ OLAH | 0,872 | 0,863 | 0,882 | 0,894 | 0,827 | 0,962 | 0,947 | 0,896 | 0,998 |
| IFI27_ OAS1_ IFI44L_ FAM20A | 0,872 | 0,862 | 0,882 | 0,894 | 0,825 | 0,964 | 0,942 | 0,863 | 1 |
| OAS1_ SLC1A2_ IL1R2_ OLAH | 0,872 | 0,863 | 0,881 | 0,908 | 0,850 | 0,965 | 0,925 | 0,859 | 0,991 |
| IFI27_ OAS1_ SIGLEC1_ MMP8 | 0,872 | 0,863 | 0,881 | 0,890 | 0,824 | 0,956 | 0,921 | 0,848 | 0,993 |
| RSAD2_ SLC1A2_ OLAH_ MMP8 | 0,872 | 0,863 | 0,881 | 0,901 | 0,842 | 0,959 | 0,936 | 0,880 | 0,992 |
| SIGLEC1_ ISG15_ HERC6_ OLAH | 0,872 | 0,863 | 0,881 | 0,891 | 0,822 | 0,960 | 0,954 | 0,911 | 0,998 |
| ISG15_ HERC6_ SLC1A2_ IL1R2 | 0,872 | 0,863 | 0,881 | 0,901 | 0,846 | 0,957 | 0,936 | 0,871 | 1 |
| IFIT1_ ISG15_ SLC1A2_ IL1R2 | 0,872 | 0,864 | 0,880 | 0,903 | 0,845 | 0,960 | 0,948 | 0,898 | 0,998 |
| OAS1_ SLC1A2_ OLAH_ MMP8 | 0,872 | 0,863 | 0,881 | 0,902 | 0,844 | 0,960 | 0,932 | 0,872 | 0,991 |
| OAS1_ HERC6_ IL1R2_ RETN | 0,872 | 0,863 | 0,881 | 0,894 | 0,828 | 0,959 | 0,939 | 0,885 | 0,993 |
| RSAD2_ IFI27_ FAM20A_ MMP8 | 0,872 | 0,862 | 0,882 | 0,894 | 0,826 | 0,962 | 0,934 | 0,853 | 1 |
| OAS1_ HERC6_ IL1R2_ MMP8 | 0,872 | 0,863 | 0,881 | 0,893 | 0,831 | 0,955 | 0,932 | 0,873 | 0,990 |
| RSAD2_ IL1R2_ FAM20A_ RETN | 0,872 | 0,863 | 0,881 | 0,893 | 0,826 | 0,961 | 0,943 | 0,868 | 1 |

66

(suite)

| Modèle | AUC modèle | IC 95% | | AUC Train | IC 95% Train | | AUC Test | IC 95% Test | |
|---|---|---|---|---|---|---|---|---|---|
| IFI27_ SIGLEC1_ HERC6_ MMP8 | 0,872 | 0,863 | 0,881 | 0,889 | 0,823 | 0,956 | 0,918 | 0,846 | 0,991 |
| IFI27_ OAS1_ SIGLEC1_ SLC1A2 | 0,872 | 0,863 | 0,881 | 0,896 | 0,837 | 0,956 | 0,957 | 0,908 | 1 |
| HERC6_ SLC1A2_ OLAH_ RETN | 0,872 | 0,862 | 0,881 | 0,903 | 0,844 | 0,962 | 0,935 | 0,874 | 0,995 |
| ISG15_ SLC1A2_ IL1R2_ OLAH | 0,872 | 0,863 | 0,880 | 0,897 | 0,839 | 0,956 | 0,921 | 0,847 | 0,994 |
| RSAD2_ IFI27_ SIGLEC1_ HERC6 | 0,872 | 0,863 | 0,881 | 0,896 | 0,840 | 0,951 | 0,922 | 0,859 | 0,985 |
| RSAD2_ OAS1_ SIGLEC1_ OLAH | 0,872 | 0,862 | 0,881 | 0,891 | 0,822 | 0,960 | 0,945 | 0,893 | 0,996 |
| IFI44L_ HERC6_ SLC1A2_ IL1R2_ | 0,872 | 0,863 | 0,881 | 0,900 | 0,844 | 0,956 | 0,935 | 0,874 | 0,996 |
| IFI44L_ ISG15_ SLC1A2_ IL1R2_ | 0,872 | 0,864 | 0,880 | 0,900 | 0,844 | 0,955 | 0,937 | 0,874 | 1,000 |
| IFIT1_ IFI44L_ SIGLEC1_ OLAH | 0,872 | 0,863 | 0,881 | 0,893 | 0,826 | 0,960 | 0,938 | 0,881 | 0,995 |
| OAS1_ IFI44L_ HERC6_ IL1R2_ | 0,872 | 0,863 | 0,880 | 0,895 | 0,833 | 0,956 | 0,941 | 0,889 | 0,994 |
| RSAD2_ IFIT1_ SLC1A2_ OLAH | 0,872 | 0,862 | 0,881 | 0,902 | 0,844 | 0,961 | 0,945 | 0,895 | 0,995 |
| OAS1_ OLAH_ FAM20A_ MMP8 | 0,872 | 0,862 | 0,881 | 0,894 | 0,824 | 0,965 | 0,950 | 0,886 | 1 |
| IFI27_ OAS1_ SIGLEC1_ HERC6 | 0,872 | 0,862 | 0,881 | 0,896 | 0,841 | 0,952 | 0,937 | 0,883 | 0,991 |
| IFI27_ SLC1A2_ RETN_ MMP8 | 0,872 | 0,862 | 0,881 | 0,897 | 0,834 | 0,961 | 0,914 | 0,827 | 1 |
| IFIT1_ SIGLEC1_ ISG15_ OLAH | 0,872 | 0,863 | 0,880 | 0,894 | 0,827 | 0,961 | 0,946 | 0,893 | 0,998 |
| RSAD2_ OAS1_ SLC1A2_ OLAH | 0,871 | 0,862 | 0,881 | 0,902 | 0,844 | 0,961 | 0,934 | 0,876 | 0,991 |
| HERC6_ IL1R2_ OLAH_ RETN | 0,871 | 0,862 | 0,881 | 0,896 | 0,832 | 0,961 | 0,940 | 0,887 | 0,993 |
| IFIT1_ SIGLEC1_ OLAH_ RETN | 0,871 | 0,862 | 0,881 | 0,892 | 0,823 | 0,962 | 0,941 | 0,884 | 0,998 |
| OAS1_ SIGLEC1_ OLAH_ MMP8 | 0,871 | 0,862 | 0,881 | 0,890 | 0,821 | 0,958 | 0,954 | 0,910 | 0,999 |
| OAS1_ IFIT1_ SLC1A2_ IL1R2 | 0,871 | 0,863 | 0,880 | 0,903 | 0,845 | 0,961 | 0,946 | 0,895 | 0,997 |
| RSAD2_ HERC6_ IL1R2_ RETN | 0,871 | 0,862 | 0,881 | 0,894 | 0,828 | 0,960 | 0,941 | 0,889 | 0,994 |
| HERC6_ IL1R2_ OLAH_ MMP8 | 0,871 | 0,862 | 0,880 | 0,897 | 0,838 | 0,956 | 0,935 | 0,877 | 0,992 |
| IFI44L_ SIGLEC1_ OLAH_ RETN | 0,871 | 0,862 | 0,881 | 0,891 | 0,821 | 0,961 | 0,943 | 0,888 | 0,998 |
| RSAD2_ IFI44L_ HERC6_ IL1R2 | 0,871 | 0,862 | 0,880 | 0,894 | 0,833 | 0,955 | 0,942 | 0,891 | 0,994 |
| HERC6_ IL1R2_ RETN_ MMP8 | 0,871 | 0,862 | 0,881 | 0,893 | 0,828 | 0,958 | 0,930 | 0,867 | 0,994 |

| Modèle | AUC modèle | IC 95% | | AUC Train | IC 95% Train | | AUC Test | IC 95% Test | |
|---|---|---|---|---|---|---|---|---|---|
| RSAD2_ HERC6_ IL1R2_ MMP8 | 0,871 | 0,862 | 0,880 | 0,895 | 0,833 | 0,956 | 0,943 | 0,892 | 0,995 |
| OAS1_ HERC6_ SLC1A2_ IL1R2 | 0,871 | 0,862 | 0,880 | 0,900 | 0,844 | 0,957 | 0,926 | 0,859 | 0,994 |
| RSAD2_ SLC1A2_ OLAH_ RETN | 0,871 | 0,862 | 0,880 | 0,902 | 0,843 | 0,961 | 0,940 | 0,886 | 0,994 |
| IFI44L_ SIGLEC1_ ISG15_ OLAH | 0,871 | 0,862 | 0,880 | 0,891 | 0,822 | 0,959 | 0,947 | 0,896 | 0,998 |
| SIGLEC1_ ISG15_ OLAH_ MMP8 | 0,871 | 0,861 | 0,880 | 0,890 | 0,822 | 0,958 | 0,953 | 0,908 | 0,999 |
| RSAD2_ HERC6_ SLC1A2_ IL1R2 | 0,871 | 0,862 | 0,880 | 0,899 | 0,842 | 0,956 | 0,932 | 0,865 | 0,998 |
| IFI44L_ HERC6_ FAM20A_ RETN | 0,871 | 0,861 | 0,881 | 0,892 | 0,821 | 0,963 | 0,941 | 0,864 | 1 |
| IFI27_ HERC6_ SLC1A2_ MMP8 | 0,871 | 0,861 | 0,880 | 0,900 | 0,843 | 0,957 | 0,904 | 0,824 | 0,985 |
| OAS1_ IFI44L_ SIGLEC1_ OLAH | 0,871 | 0,861 | 0,880 | 0,892 | 0,824 | 0,960 | 0,948 | 0,899 | 0,997 |
| OAS1_ ISG15_ OLAH_ FAM20A | 0,871 | 0,861 | 0,881 | 0,891 | 0,819 | 0,963 | 0,951 | 0,891 | 1 |
| IFI44L_ ISG15_ OLAH_ FAM20A | 0,871 | 0,861 | 0,880 | 0,890 | 0,818 | 0,961 | 0,958 | 0,912 | 1 |
| SIGLEC1_ ISG15_ OLAH_ RETN | 0,871 | 0,861 | 0,880 | 0,891 | 0,820 | 0,962 | 0,949 | 0,897 | 1 |
| IFI27_ IFIT1_ ISG15_ FAM20A | 0,870 | 0,860 | 0,881 | 0,887 | 0,816 | 0,958 | 0,942 | 0,864 | 1 |
| IFIT1_ IFI44L_ SLC1A2_ IL1R2 | 0,870 | 0,862 | 0,879 | 0,900 | 0,841 | 0,959 | 0,948 | 0,898 | 0,997 |
| OAS1_ IFI44L_ OLAH_ FAM20A | 0,870 | 0,860 | 0,880 | 0,890 | 0,817 | 0,963 | 0,953 | 0,898 | 1 |
| OAS1_ ISG15_ SLC1A2_ OLAH | 0,870 | 0,861 | 0,880 | 0,906 | 0,849 | 0,963 | 0,930 | 0,869 | 0,992 |
| RSAD2_ SIGLEC1_ OLAH_ MMP8 | 0,870 | 0,861 | 0,880 | 0,890 | 0,822 | 0,958 | 0,953 | 0,908 | 0,999 |
| OAS1_ OLAH_ FAM20A_ RETN | 0,870 | 0,860 | 0,880 | 0,893 | 0,822 | 0,965 | 0,950 | 0,888 | 1 |
| IFI44L_ SLC1A2_ IL1R2_ RETN | 0,870 | 0,861 | 0,879 | 0,899 | 0,842 | 0,956 | 0,938 | 0,879 | 0,997 |
| IFI44L_ OLAH_ FAM20A_ MMP8 | 0,870 | 0,860 | 0,880 | 0,888 | 0,817 | 0,959 | 0,958 | 0,911 | 1 |
| OAS1_ SIGLEC1_ OLAH_ RETN | 0,870 | 0,860 | 0,880 | 0,890 | 0,819 | 0,961 | 0,949 | 0,898 | 1 |
| IFIT1_ SLC1A2_ IL1R2_ RETN | 0,870 | 0,861 | 0,879 | 0,899 | 0,839 | 0,959 | 0,950 | 0,902 | 0,998 |
| OAS1_ SLC1A2_ OLAH_ RETN | 0,870 | 0,861 | 0,879 | 0,903 | 0,845 | 0,961 | 0,932 | 0,871 | 0,992 |
| RSAD2_ IFI27_ SIGLEC1_ MMP8 | 0,870 | 0,860 | 0,880 | 0,888 | 0,821 | 0,955 | 0,915 | 0,842 | 0,988 |
| RSAD2_ IFIT1_ SLC1A2_ IL1R2 | 0,870 | 0,862 | 0,878 | 0,899 | 0,840 | 0,958 | 0,949 | 0,899 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2_ ISG15_ SLC1A2_ OLAH | 0,870 | 0,861 | 0,879 | 0,901 | 0,843 | 0,960 | 0,935 | 0,878 | 0,991 |
| ISG15_ HERC6_ OLAH_ RETN | 0,870 | 0,860 | 0,880 | 0,892 | 0,822 | 0,962 | 0,953 | 0,909 | 0,998 |
| IFI44L_ SIGLEC1_ HERC6_ RETN | 0,869 | 0,860 | 0,879 | 0,883 | 0,808 | 0,959 | 0,943 | 0,894 | 0,993 |
| IFI44L_ SLC1A2_ IL1R2_ MMP8 | 0,869 | 0,861 | 0,877 | 0,897 | 0,842 | 0,953 | 0,936 | 0,871 | 1 |
| RSAD2_ SIGLEC1_ OLAH_ RETN | 0,869 | 0,860 | 0,879 | 0,890 | 0,819 | 0,961 | 0,948 | 0,896 | 1,000 |
| OAS1_ IFIT1_ IL1R2_ OLAH | 0,869 | 0,860 | 0,879 | 0,893 | 0,826 | 0,961 | 0,946 | 0,896 | 0,995 |
| HERC6_ SLC1A2_ IL1R2_ RETN | 0,869 | 0,860 | 0,879 | 0,899 | 0,841 | 0,958 | 0,915 | 0,839 | 0,992 |
| IFI27_ ISG15_ SLC1A2_ FAM20A | 0,869 | 0,859 | 0,879 | 0,888 | 0,817 | 0,960 | 0,926 | 0,843 | 1 |
| IFIT1_ HERC6_ FAM20A_ RETN | 0,869 | 0,859 | 0,879 | 0,887 | 0,816 | 0,958 | 0,937 | 0,859 | 1 |
| IFI27_ IFI44L_ SLC1A2_ MMP8 | 0,869 | 0,860 | 0,879 | 0,893 | 0,831 | 0,955 | 0,912 | 0,837 | 0,987 |
| OAS1_ IFIT1_ IL1R2_ MMP8 | 0,869 | 0,860 | 0,878 | 0,892 | 0,828 | 0,956 | 0,954 | 0,911 | 0,998 |
| HERC6_ SLC1A2_ FAM20A_ RETN | 0,869 | 0,859 | 0,879 | 0,896 | 0,833 | 0,960 | 0,934 | 0,851 | 1 |
| HERC6_ OLAH_ RETN_ MMP8 | 0,869 | 0,859 | 0,879 | 0,892 | 0,822 | 0,962 | 0,957 | 0,915 | 0,998 |
| IFI27_ IFIT1_ SIGLEC1_ SLC1A2 | 0,869 | 0,860 | 0,878 | 0,894 | 0,834 | 0,954 | 0,954 | 0,907 | 1 |
| HERC6_ SLC1A2_ IL1R2_ MMP8 | 0,869 | 0,860 | 0,878 | 0,900 | 0,845 | 0,955 | 0,910 | 0,828 | 0,991 |
| RSAD2_ IFI27_ OAS1_ FAM20A | 0,869 | 0,859 | 0,879 | 0,884 | 0,811 | 0,957 | 0,938 | 0,858 | 1 |
| IFI27_ SIGLEC1_ ISG15_ MMP8 | 0,869 | 0,859 | 0,878 | 0,884 | 0,814 | 0,954 | 0,917 | 0,845 | 0,989 |
| OAS1_ HERC6_ OLAH_ RETN | 0,869 | 0,859 | 0,878 | 0,888 | 0,819 | 0,958 | 0,942 | 0,891 | 0,993 |
| IFI44L_ HERC6_ OLAH_ RETN | 0,869 | 0,859 | 0,879 | 0,889 | 0,817 | 0,960 | 0,945 | 0,895 | 0,994 |
| IFIT1_ HERC6_ OLAH_ RETN | 0,869 | 0,859 | 0,878 | 0,889 | 0,820 | 0,959 | 0,940 | 0,888 | 0,993 |
| IFI27_ OAS1_ ISG15_ FAM20A | 0,869 | 0,858 | 0,879 | 0,883 | 0,810 | 0,956 | 0,942 | 0,864 | 1 |
| IFIT1_ IFI44L_ IL1R2_ MMP8 | 0,868 | 0,859 | 0,877 | 0,892 | 0,828 | 0,955 | 0,957 | 0,914 | 0,999 |
| OAS1_ IFIT1_ IFI44L_ IL1R2 | 0,868 | 0,859 | 0,877 | 0,888 | 0,820 | 0,957 | 0,945 | 0,895 | 0,994 |
| RSAD2_ HERC6_ OLAH_ RETN | 0,868 | 0,858 | 0,878 | 0,888 | 0,818 | 0,958 | 0,941 | 0,890 | 0,993 |
| IFIT1_ IFI44L_ ISG15_ IL1R2 | 0,868 | 0,859 | 0,877 | 0,894 | 0,831 | 0,958 | 0,957 | 0,914 | 0,999 |

(suite)

| Modèle | AUC modèle | IC 95% | | AUC Train | IC 95% Train | | AUC Test | IC 95% Test | |
|---|---|---|---|---|---|---|---|---|---|
| OAS1_ SIGLEC1_ ISG15_ OLAH | 0,868 | 0,858 | 0,878 | 0,889 | 0,819 | 0,958 | 0,945 | 0,892 | 0,997 |
| SIGLEC1_ HERC6_ SLC1A2_ RETN | 0,868 | 0,858 | 0,877 | 0,893 | 0,827 | 0,960 | 0,955 | 0,910 | 1 |
| IFIT1_ IL1R2_ OLAH_ MMP8 | 0,868 | 0,858 | 0,877 | 0,890 | 0,826 | 0,954 | 0,961 | 0,922 | 1 |
| CAS1_ ISG15_ HERC6_ RETN | 0,868 | 0,858 | 0,878 | 0,890 | 0,821 | 0,959 | 0,941 | 0,886 | 0,996 |
| ISG15_ SLC1A2_ OLAH_ MMP8 | 0,868 | 0,858 | 0,877 | 0,894 | 0,835 | 0,954 | 0,922 | 0,856 | 0,988 |
| ISG15_ SLC1A2_ OLAH_ RETN | 0,868 | 0,858 | 0,877 | 0,897 | 0,838 | 0,956 | 0,924 | 0,856 | 0,992 |
| RSAD2_ IFI27_ SIGLEC1_ SLC1A2 | 0,868 | 0,858 | 0,877 | 0,895 | 0,836 | 0,955 | 0,951 | 0,902 | 1 |
| OAS1_ IFIT1_ ISG15_ IL1R2 | 0,868 | 0,859 | 0,877 | 0,891 | 0,825 | 0,957 | 0,949 | 0,902 | 0,996 |
| OAS1_ HERC6_ FAM20A_ RETN | 0,868 | 0,858 | 0,878 | 0,887 | 0,816 | 0,959 | 0,939 | 0,861 | 1 |
| IFI27_ SLC1A2_ FAM20A_ MMP8 | 0,868 | 0,857 | 0,878 | 0,891 | 0,825 | 0,958 | 0,907 | 0,815 | 0,998 |
| IFI27_ OAS1_ IFIT1_ FAM20A | 0,868 | 0,857 | 0,878 | 0,885 | 0,813 | 0,956 | 0,943 | 0,865 | 1 |
| IFIT1_ ISG15_ IL1R2_ MMP8 | 0,868 | 0,859 | 0,876 | 0,893 | 0,831 | 0,955 | 0,963 | 0,925 | 1 |
| RSAD2_ OAS1_ IFIT1_ IL1R2 | 0,868 | 0,859 | 0,877 | 0,888 | 0,820 | 0,956 | 0,947 | 0,899 | 0,995 |
| RSAD2_ OAS1_ IL1R2_ OLAH | 0,867 | 0,858 | 0,877 | 0,893 | 0,827 | 0,960 | 0,945 | 0,891 | 0,998 |
| IFI27_ IFI44L_ SIGLEC1_ HERC6 | 0,867 | 0,858 | 0,877 | 0,891 | 0,830 | 0,952 | 0,932 | 0,875 | 0,988 |
| RSAD2_ IFI44L_ SIGLEC1_ OLAH | 0,867 | 0,858 | 0,877 | 0,891 | 0,822 | 0,960 | 0,952 | 0,905 | 0,999 |
| IFI27_ IFIT1_ SLC1A2_ FAM20A | 0,867 | 0,857 | 0,878 | 0,890 | 0,821 | 0,958 | 0,941 | 0,862 | 1 |
| RSAD2_ IFI27_ IFIT1_ FAM20A | 0,867 | 0,857 | 0,878 | 0,888 | 0,817 | 0,958 | 0,940 | 0,861 | 1 |
| IFI27_ IFI44L_ SIGLEC1_ SLC1A2 | 0,867 | 0,858 | 0,876 | 0,893 | 0,831 | 0,954 | 0,960 | 0,914 | 1 |
| RSAD2_ SIGLEC1_ ISG15_ OLAH | 0,867 | 0,858 | 0,876 | 0,890 | 0,821 | 0,959 | 0,950 | 0,902 | 0,998 |
| IFI27_ SIGLEC1_ ISG15_ SLC1A2 | 0,867 | 0,858 | 0,876 | 0,892 | 0,831 | 0,952 | 0,959 | 0,912 | 1 |
| RSAD2_ HERC6_ FAM20A_ RETN | 0,867 | 0,857 | 0,877 | 0,886 | 0,815 | 0,958 | 0,938 | 0,860 | 1 |
| IFI44L_ ISG15_ IL1R2_ OLAH | 0,867 | 0,858 | 0,876 | 0,892 | 0,830 | 0,954 | 0,948 | 0,899 | 0,996 |
| RSAD2_ IFI27_ ISG15_ FAM20A | 0,867 | 0,857 | 0,877 | 0,885 | 0,814 | 0,957 | 0,945 | 0,866 | 1 |
| RSAD2_ IFIT1_ IL1R2_ MMP8 | 0,867 | 0,858 | 0,876 | 0,891 | 0,827 | 0,955 | 0,960 | 0,920 | 1,000 |

(suite)

| Modèle | AUC modèle | IC 95% | | AUC Train | IC 95% Train | | AUC Test | IC 95% Test | |
|---|---|---|---|---|---|---|---|---|---|
| IFIT1_ IFI44L_ OLAH_ FAM20A | 0,867 | 0,857 | 0,877 | 0,889 | 0,818 | 0,960 | 0,957 | 0,909 | 1 |
| IFIT1_ HERC6_ OLAH_ MMP8 | 0,867 | 0,857 | 0,876 | 0,887 | 0,819 | 0,954 | 0,940 | 0,887 | 0,993 |
| IFIT1_ ISG15_ IL1R2_ OLAH | 0,867 | 0,857 | 0,876 | 0,895 | 0,830 | 0,959 | 0,959 | 0,918 | 1,000 |
| SIGLEC1_ ISG15_ HERC6_ RETN | 0,866 | 0,856 | 0,877 | 0,879 | 0,801 | 0,958 | 0,959 | 0,916 | 1 |
| OAS1_ IFIT1_ IL1R2_ RETN | 0,866 | 0,857 | 0,876 | 0,888 | 0,817 | 0,958 | 0,950 | 0,904 | 0,996 |
| IFI44L_ HERC6_ OLAH_ MMP8 | 0,866 | 0,857 | 0,876 | 0,887 | 0,819 | 0,955 | 0,941 | 0,889 | 0,993 |
| IFI44L_ OLAH_ FAM20A_ RETN | 0,866 | 0,856 | 0,876 | 0,889 | 0,818 | 0,960 | 0,957 | 0,909 | 1 |
| IFIT1_ ISG15_ IL1R2_ RETN | 0,866 | 0,857 | 0,876 | 0,890 | 0,824 | 0,957 | 0,955 | 0,912 | 0,999 |
| IFI27_ OAS1_ SLC1A2_ FAM20A | 0,866 | 0,856 | 0,877 | 0,888 | 0,820 | 0,957 | 0,938 | 0,857 | 1 |
| OAS1_ IFI44L_ IL1R2_ OLAH | 0,866 | 0,857 | 0,876 | 0,893 | 0,827 | 0,959 | 0,940 | 0,885 | 0,995 |
| OAS1_ SIGLEC1_ SLC1A2_ RETN | 0,866 | 0,857 | 0,875 | 0,893 | 0,829 | 0,957 | 0,951 | 0,903 | 0,999 |
| OAS1_ IL1R2_ OLAH_ MMP8 | 0,866 | 0,857 | 0,875 | 0,894 | 0,831 | 0,957 | 0,939 | 0,883 | 0,996 |
| RSAD2_ OAS1_ HERC6_ OLAH | 0,866 | 0,856 | 0,875 | 0,887 | 0,821 | 0,953 | 0,937 | 0,882 | 0,992 |
| IFI44L_ IL1R2_ OLAH_ MMP8 | 0,866 | 0,857 | 0,875 | 0,892 | 0,832 | 0,951 | 0,946 | 0,895 | 0,996 |
| OAS1_ ISG15_ IL1R2_ OLAH | 0,866 | 0,857 | 0,875 | 0,893 | 0,826 | 0,959 | 0,942 | 0,888 | 0,997 |
| IFIT1_ SIGLEC1_ SLC1A2_ RETN | 0,866 | 0,857 | 0,875 | 0,894 | 0,832 | 0,956 | 0,947 | 0,897 | 0,996 |
| IFIT1_ IL1R2_ RETN_ MMP8 | 0,866 | 0,856 | 0,876 | 0,890 | 0,820 | 0,959 | 0,965 | 0,929 | 1 |
| ISG15_ SLC1A2_ IL1R2_ RETN | 0,866 | 0,857 | 0,874 | 0,898 | 0,840 | 0,956 | 0,915 | 0,834 | 0,996 |
| ISG15_ HERC6_ OLAH_ MMP8 | 0,866 | 0,856 | 0,875 | 0,888 | 0,820 | 0,955 | 0,946 | 0,897 | 0,994 |
| RSAD2_ IFI27_ SLC1A2_ FAM20A | 0,865 | 0,855 | 0,876 | 0,886 | 0,817 | 0,956 | 0,939 | 0,858 | 1 |
| HERC6_ FAM20A_ RETN_ MMP8 | 0,865 | 0,855 | 0,876 | 0,888 | 0,817 | 0,959 | 0,941 | 0,864 | 1 |
| OAS1_ HERC6_ OLAH_ MMP8 | 0,865 | 0,856 | 0,875 | 0,887 | 0,820 | 0,954 | 0,940 | 0,887 | 0,993 |
| RSAD2_ ISG15_ SLC1A2_ IL1R2 | 0,865 | 0,857 | 0,873 | 0,896 | 0,838 | 0,954 | 0,939 | 0,877 | 1 |
| RSAD2_ IFIT1_ ISG15_ IL1R2 | 0,865 | 0,856 | 0,874 | 0,891 | 0,826 | 0,956 | 0,955 | 0,913 | 0,998 |
| IFI27_ IFIT1_ SLC1A2_ MMP8 | 0,865 | 0,855 | 0,875 | 0,889 | 0,826 | 0,953 | 0,920 | 0,847 | 0,992 |

(suite)

| Modèle | AUC modèle | IC 95% | | AUC Train | IC 95% Train | | AUC Test | IC 95% Test | |
|---|---|---|---|---|---|---|---|---|---|
| RSAD2_IFI27_SLC1A2_MMP8 | 0,865 | 0,855 | 0,874 | 0,888 | 0,824 | 0,952 | 0,911 | 0,832 | 0,990 |
| OAS1_IL1R2_OLAH_RETN | 0,865 | 0,855 | 0,874 | 0,895 | 0,827 | 0,962 | 0,942 | 0,888 | 0,997 |
| IFI44L_ISG15_IL1R2_MMP8 | 0,865 | 0,857 | 0,873 | 0,892 | 0,834 | 0,950 | 0,954 | 0,909 | 1 |
| IFIT1_ISG15_OLAH_FAM20A | 0,865 | 0,855 | 0,875 | 0,888 | 0,816 | 0,960 | 0,955 | 0,906 | 1 |
| RSAD2_OLAH_FAM20A_MMP8 | 0,865 | 0,855 | 0,875 | 0,886 | 0,814 | 0,958 | 0,959 | 0,907 | 1 |
| IFI44L_ISG15_HERC6_OLAH | 0,865 | 0,855 | 0,874 | 0,885 | 0,818 | 0,952 | 0,950 | 0,904 | 0,996 |
| IFIT1_IFI44L_HERC6_OLAH | 0,865 | 0,855 | 0,874 | 0,887 | 0,820 | 0,953 | 0,939 | 0,885 | 0,993 |
| IFIT1_IFI44L_IL1R2_OLAH | 0,865 | 0,855 | 0,874 | 0,891 | 0,826 | 0,956 | 0,953 | 0,908 | 0,998 |
| OAS1_ISG15_HERC6_OLAH | 0,865 | 0,855 | 0,874 | 0,885 | 0,819 | 0,950 | 0,951 | 0,905 | 0,997 |
| SIGLEC1_ISG15_SLC1A2_RETN | 0,865 | 0,856 | 0,874 | 0,893 | 0,830 | 0,957 | 0,951 | 0,903 | 0,999 |
| RSAD2_IFIT1_IFI44L_IL1R2 | 0,865 | 0,855 | 0,874 | 0,887 | 0,819 | 0,955 | 0,955 | 0,913 | 0,998 |
| RSAD2_HERC6_OLAH_MMP8 | 0,864 | 0,855 | 0,874 | 0,888 | 0,822 | 0,954 | 0,939 | 0,885 | 0,993 |
| IFIT1_ISG15_HERC6_OLAH | 0,864 | 0,855 | 0,874 | 0,885 | 0,818 | 0,951 | 0,948 | 0,900 | 0,995 |
| RSAD2_ISG15_HERC6_RETN | 0,864 | 0,854 | 0,875 | 0,890 | 0,821 | 0,958 | 0,938 | 0,883 | 0,993 |
| IFI44L_ISG15_IL1R2_RETN | 0,864 | 0,855 | 0,873 | 0,888 | 0,823 | 0,953 | 0,959 | 0,917 | 1 |
| OAS1_IFIT1_HERC6_OLAH | 0,864 | 0,855 | 0,874 | 0,886 | 0,819 | 0,953 | 0,941 | 0,889 | 0,993 |
| IFI44L_SIGLEC1_SLC1A2_RETN | 0,864 | 0,856 | 0,873 | 0,894 | 0,830 | 0,958 | 0,951 | 0,903 | 0,999 |
| OAS1_ISG15_SLC1A2_IL1R2 | 0,864 | 0,856 | 0,873 | 0,897 | 0,840 | 0,955 | 0,922 | 0,852 | 0,992 |
| RSAD2_IFI44L_SLC1A2_IL1R2 | 0,864 | 0,856 | 0,873 | 0,896 | 0,838 | 0,954 | 0,941 | 0,883 | 1,000 |
| RSAD2_IFI44L_OLAH_FAM20A | 0,864 | 0,854 | 0,875 | 0,888 | 0,816 | 0,960 | 0,955 | 0,906 | 1 |
| RSAD2_OAS1_SLC1A2_IL1R2 | 0,864 | 0,856 | 0,873 | 0,896 | 0,838 | 0,954 | 0,936 | 0,873 | 0,999 |
| RSAD2_SLC1A2_IL1R2_MMP8 | 0,864 | 0,856 | 0,873 | 0,896 | 0,838 | 0,954 | 0,938 | 0,874 | 1 |
| RSAD2_SLC1A2_IL1R2_RETN | 0,864 | 0,855 | 0,873 | 0,897 | 0,838 | 0,956 | 0,938 | 0,874 | 1 |
| OAS1_SIGLEC1_HERC6_SLC1A2 | 0,864 | 0,855 | 0,873 | 0,886 | 0,824 | 0,949 | 0,947 | 0,898 | 0,996 |
| RSAD2_SIGLEC1_SLC1A2_RETN | 0,864 | 0,855 | 0,873 | 0,893 | 0,830 | 0,957 | 0,951 | 0,904 | 0,998 |

| Modèle | AUC modèle | IC 95% | | AUC Train | IC 95% Train | | AUC Test | IC 95% Test | |
|---|---|---|---|---|---|---|---|---|---|
| OAS1_ IFI44L_ SLC1A2_ IL1R2 | 0,864 | 0,855 | 0,873 | 0,898 | 0,841 | 0,954 | 0,934 | 0,873 | 0,994 |
| SIGLEC1_ HERC6_ RETN_ MMP8 | 0,864 | 0,854 | 0,874 | 0,879 | 0,799 | 0,958 | 0,955 | 0,911 | 0,999 |
| RSAD2_ ISG15_ HERC6_ OLAH | 0,864 | 0,854 | 0,874 | 0,885 | 0,820 | 0,950 | 0,946 | 0,897 | 0,994 |
| RSAD2_ IFIT1_ IL1R2_ OLAH | 0,864 | 0,855 | 0,873 | 0,891 | 0,825 | 0,957 | 0,951 | 0,905 | 0,997 |
| RSAD2_ IL1R2_ OLAH_ MMP8 | 0,864 | 0,855 | 0,873 | 0,891 | 0,828 | 0,954 | 0,957 | 0,914 | 0,999 |
| IFI27_ IFIT1_ SIGLEC1_ HERC6 | 0,864 | 0,854 | 0,873 | 0,890 | 0,831 | 0,949 | 0,927 | 0,866 | 0,989 |
| OAS1_ IFIT1_ SIGLEC1_ RETN | 0,864 | 0,854 | 0,874 | 0,878 | 0,803 | 0,953 | 0,940 | 0,887 | 0,994 |
| IFI27_ ISG15_ SLC1A2_ MMP8 | 0,864 | 0,854 | 0,873 | 0,886 | 0,821 | 0,951 | 0,903 | 0,820 | 0,986 |
| OAS1_ IFI44L_ HERC6_ OLAH | 0,864 | 0,854 | 0,873 | 0,886 | 0,820 | 0,953 | 0,941 | 0,889 | 0,993 |
| IFI27_ OAS1_ SLC1A2_ MMP8 | 0,863 | 0,854 | 0,873 | 0,887 | 0,823 | 0,951 | 0,908 | 0,828 | 0,987 |
| IFIT1_ IFI44L_ IL1R2_ RETN | 0,863 | 0,854 | 0,873 | 0,886 | 0,817 | 0,956 | 0,957 | 0,914 | 0,999 |
| IFIT1_ SIGLEC1_ HERC6_ MMP8 | 0,863 | 0,854 | 0,873 | 0,881 | 0,813 | 0,950 | 0,887 | 0,805 | 0,969 |
| RSAD2_ SIGLEC1_ HERC6_ SLC1A2 | 0,863 | 0,855 | 0,872 | 0,887 | 0,824 | 0,949 | 0,933 | 0,876 | 0,989 |
| RSAD2_ IFIT1_ IL1R2_ RETN | 0,863 | 0,854 | 0,873 | 0,884 | 0,813 | 0,955 | 0,955 | 0,913 | 0,998 |
| IFI27_ SIGLEC1_ HERC6_ SLC1A2 | 0,863 | 0,854 | 0,873 | 0,890 | 0,828 | 0,952 | 0,965 | 0,924 | 1 |
| RSAD2_ ISG15_ OLAH_ FAM20A | 0,863 | 0,853 | 0,873 | 0,885 | 0,812 | 0,958 | 0,959 | 0,907 | 1 |
| RSAD2_ ISG15_ IL1R2_ OLAH | 0,863 | 0,854 | 0,872 | 0,890 | 0,824 | 0,956 | 0,953 | 0,908 | 0,999 |
| RSAD2_ OAS1_ IFI44L_ IL1R2 | 0,863 | 0,854 | 0,872 | 0,884 | 0,818 | 0,951 | 0,937 | 0,882 | 0,992 |
| RSAD2_ OLAH_ FAM20A_ RETN | 0,863 | 0,853 | 0,873 | 0,886 | 0,813 | 0,959 | 0,958 | 0,904 | 1 |
| RSAD2_ IFI44L_ IL1R2_ OLAH | 0,863 | 0,854 | 0,872 | 0,891 | 0,826 | 0,955 | 0,946 | 0,895 | 0,997 |
| ISG15_ SLC1A2_ IL1R2_ MMP8 | 0,863 | 0,855 | 0,871 | 0,894 | 0,837 | 0,951 | 0,917 | 0,837 | 0,998 |
| RSAD2_ IFI44L_ HERC6_ OLAH | 0,863 | 0,853 | 0,872 | 0,887 | 0,821 | 0,953 | 0,938 | 0,884 | 0,992 |
| ISG15_ IL1R2_ OLAH_ MMP8 | 0,863 | 0,854 | 0,872 | 0,889 | 0,830 | 0,949 | 0,938 | 0,877 | 0,999 |
| SIGLEC1_ HERC6_ SLC1A2_ MMP8 | 0,863 | 0,854 | 0,872 | 0,887 | 0,822 | 0,952 | 0,934 | 0,877 | 0,991 |
| OAS1_ SLC1A2_ IL1R2_ MMP8 | 0,863 | 0,854 | 0,871 | 0,897 | 0,839 | 0,955 | 0,918 | 0,846 | 0,991 |

(suite)

| Modèle | AUC modèle | IC 95% | | AUC Train | IC 95% Train | | AUC Test | IC 95% Test | |
|---|---|---|---|---|---|---|---|---|---|
| RSAD2_IFIT1_OLAH_FAM20A | 0,863 | 0,853 | 0,873 | 0,886 | 0,812 | 0,959 | 0,957 | 0,904 | 1 |
| HERC6_SLC1A2_FAM20A_MMP8 | 0,863 | 0,853 | 0,873 | 0,888 | 0,824 | 0,952 | 0,922 | 0,836 | 1 |
| OAS1_SLC1A2_IL1R2_RETN | 0,862 | 0,853 | 0,871 | 0,893 | 0,834 | 0,953 | 0,918 | 0,847 | 0,990 |
| OAS1_IFI44L_ISG15_IL1R2 | 0,862 | 0,853 | 0,871 | 0,887 | 0,822 | 0,952 | 0,941 | 0,889 | 0,993 |
| RSAD2_IL1R2_OLAH_RETN | 0,862 | 0,853 | 0,872 | 0,887 | 0,819 | 0,956 | 0,951 | 0,904 | 0,998 |
| IFI44L_SLC1A2_FAM20A_RETN | 0,862 | 0,852 | 0,872 | 0,890 | 0,824 | 0,956 | 0,927 | 0,848 | 1 |
| IFIT1_IL1R2_OLAH_RETN | 0,862 | 0,853 | 0,872 | 0,888 | 0,819 | 0,956 | 0,955 | 0,912 | 0,999 |
| RSAD2_IFIT1_HERC6_OLAH | 0,862 | 0,853 | 0,872 | 0,886 | 0,819 | 0,952 | 0,939 | 0,886 | 0,992 |
| ISG15_IL1R2_OLAH_RETN | 0,862 | 0,853 | 0,871 | 0,889 | 0,826 | 0,953 | 0,940 | 0,878 | 1 |
| IFIT1_OLAH_FAM20A_MMP8 | 0,862 | 0,852 | 0,872 | 0,882 | 0,810 | 0,955 | 0,958 | 0,908 | 1 |
| OAS1_IFI44L_IL1R2_MMP8 | 0,862 | 0,853 | 0,871 | 0,886 | 0,822 | 0,951 | 0,938 | 0,883 | 0,993 |
| IFIT1_SIGLEC1_HERC6_SLC1A2 | 0,862 | 0,853 | 0,871 | 0,887 | 0,825 | 0,948 | 0,935 | 0,879 | 0,990 |
| IFI44L_SIGLEC1_SLC1A2_MMP8 | 0,862 | 0,853 | 0,870 | 0,884 | 0,819 | 0,949 | 0,937 | 0,882 | 0,992 |
| IFI27_IFI44L_SIGLEC1_ISG15 | 0,862 | 0,852 | 0,871 | 0,886 | 0,822 | 0,949 | 0,941 | 0,887 | 0,996 |
| RSAD2_ISG15_HERC6_FAM20A | 0,862 | 0,852 | 0,872 | 0,882 | 0,815 | 0,949 | 0,922 | 0,834 | 1 |
| SIGLEC1_SLC1A2_RETN_MMP8 | 0,862 | 0,852 | 0,871 | 0,892 | 0,828 | 0,957 | 0,951 | 0,904 | 0,998 |
| ISG15_HERC6_SLC1A2_FAM20A | 0,862 | 0,852 | 0,871 | 0,886 | 0,822 | 0,950 | 0,933 | 0,850 | 1 |
| IFIT1_ISG15_HERC6_RETN | 0,862 | 0,851 | 0,872 | 0,883 | 0,811 | 0,955 | 0,938 | 0,881 | 0,995 |
| IFI44L_IL1R2_OLAH_RETN | 0,862 | 0,852 | 0,871 | 0,890 | 0,826 | 0,955 | 0,945 | 0,893 | 0,996 |
| IFIT1_SIGLEC1_SLC1A2_MMP8 | 0,862 | 0,853 | 0,870 | 0,885 | 0,821 | 0,949 | 0,932 | 0,874 | 0,989 |
| IFIT1_SIGLEC1_ISG15_RETN | 0,862 | 0,852 | 0,871 | 0,881 | 0,807 | 0,954 | 0,947 | 0,895 | 0,998 |
| RSAD2_OAS1_IL1R2_RETN | 0,861 | 0,852 | 0,871 | 0,885 | 0,816 | 0,954 | 0,941 | 0,889 | 0,994 |
| IFI27_IFI44L_ISG15_MMP8 | 0,861 | 0,852 | 0,871 | 0,880 | 0,810 | 0,950 | 0,905 | 0,829 | 0,982 |
| RSAD2_IFIT1_SIGLEC1_RETN | 0,861 | 0,851 | 0,871 | 0,879 | 0,804 | 0,954 | 0,941 | 0,887 | 0,996 |
| OAS1_IFIT1_OLAH_RETN | 0,861 | 0,851 | 0,871 | 0,883 | 0,808 | 0,958 | 0,948 | 0,898 | 0,998 |

(suite)

| Modèle | AUC modèle | IC 95% | | AUC Train | IC 95% Train | | AUC Test | IC 95% Test | |
|---|---|---|---|---|---|---|---|---|---|
| RSAD2_ OAS1_ IL1R2_ MMP8 | 0,861 | 0,852 | 0,870 | 0,887 | 0,822 | 0,951 | 0,936 | 0,881 | 0,991 |
| IFI27_ OAS1_ IFIT1_ SIGLEC1 | 0,861 | 0,852 | 0,871 | 0,883 | 0,819 | 0,946 | 0,940 | 0,885 | 0,995 |
| RSAD2_ OAS1_ ISG15_ IL1R2 | 0,861 | 0,852 | 0,870 | 0,886 | 0,821 | 0,952 | 0,940 | 0,887 | 0,993 |
| OAS1_ IFI44L_ IL1R2_ RETN | 0,861 | 0,852 | 0,871 | 0,884 | 0,815 | 0,953 | 0,940 | 0,887 | 0,994 |
| OAS1_ ISG15_ IL1R2_ RETN | 0,861 | 0,852 | 0,870 | 0,888 | 0,821 | 0,956 | 0,941 | 0,888 | 0,994 |
| OAS1_ SIGLEC1_ SLC1A2_ MMP8 | 0,861 | 0,852 | 0,870 | 0,884 | 0,820 | 0,949 | 0,937 | 0,882 | 0,992 |
| RSAD2_ IFI27_ IFI44L_ SIGLEC1 | 0,861 | 0,851 | 0,871 | 0,886 | 0,824 | 0,947 | 0,941 | 0,887 | 0,996 |
| IFI27_ IFIT1_ SIGLEC1_ ISG15 | 0,861 | 0,851 | 0,871 | 0,884 | 0,822 | 0,946 | 0,939 | 0,882 | 0,996 |
| IFI27_ IFIT1_ ISG15_ MMP8 | 0,861 | 0,851 | 0,870 | 0,877 | 0,807 | 0,948 | 0,912 | 0,838 | 0,986 |
| RSAD2_ IFI44L_ ISG15_ IL1R2 | 0,861 | 0,852 | 0,870 | 0,885 | 0,821 | 0,949 | 0,958 | 0,915 | 1,000 |
| ISG15_ HERC6_ FAM20A_ MMP8 | 0,861 | 0,850 | 0,871 | 0,882 | 0,812 | 0,953 | 0,929 | 0,848 | 1 |
| RSAD2_ OAS1_ OLAH_ RETN | 0,861 | 0,851 | 0,871 | 0,884 | 0,809 | 0,958 | 0,947 | 0,896 | 0,998 |
| RSAD2_ IFI27_ SIGLEC1_ ISG15 | 0,861 | 0,851 | 0,870 | 0,884 | 0,822 | 0,947 | 0,940 | 0,885 | 0,996 |
| OAS1_ ISG15_ IL1R2_ MMP8 | 0,860 | 0,852 | 0,869 | 0,886 | 0,822 | 0,950 | 0,930 | 0,871 | 0,990 |
| OAS1_ IL1R2_ RETN_ MMP8 | 0,860 | 0,851 | 0,870 | 0,886 | 0,818 | 0,955 | 0,946 | 0,897 | 0,995 |
| IFI27_ OAS1_ IFI44L_ SIGLEC1 | 0,860 | 0,851 | 0,870 | 0,885 | 0,821 | 0,948 | 0,948 | 0,897 | 0,999 |
| IFI44L_ HERC6_ SLC1A2_ FAM20A | 0,860 | 0,850 | 0,870 | 0,885 | 0,820 | 0,950 | 0,935 | 0,854 | 1 |
| RSAD2_ OAS1_ OLAH_ MMP8 | 0,860 | 0,851 | 0,870 | 0,879 | 0,807 | 0,952 | 0,945 | 0,890 | 0,999 |
| RSAD2_ SIGLEC1_ SLC1A2_ MMP8 | 0,860 | 0,851 | 0,869 | 0,885 | 0,821 | 0,949 | 0,936 | 0,881 | 0,991 |
| RSAD2_IFI27_OAS1_SIGLEC1 | 0,860 | 0,850 | 0,870 | 0,885 | 0,822 | 0,947 | 0,943 | 0,889 | 0,998 |
| ISG15_HERC6_SLC1A2_RETN | 0,860 | 0,851 | 0,870 | 0,886 | 0,819 | 0,954 | 0,940 | 0,877 | 1 |
| RSAD2_ISG15_IL1R2_RETN | 0,860 | 0,851 | 0,869 | 0,887 | 0,818 | 0,955 | 0,955 | 0,912 | 0,999 |
| IFIT1_ISG15_HERC6_FAM20A | 0,860 | 0,850 | 0,870 | 0,880 | 0,811 | 0,949 | 0,926 | 0,839 | 1 |
| IFI44L_IL1R2_RETN_MMP8 | 0,860 | 0,850 | 0,870 | 0,886 | 0,819 | 0,953 | 0,963 | 0,925 | 1 |
| ISG15_IL1R2_RETN_MMP8 | 0,860 | 0,851 | 0,869 | 0,888 | 0,825 | 0,950 | 0,935 | 0,871 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2_IFI44L_IL1R2_MMP8 | 0,860 | 0,851 | 0,869 | 0,884 | 0,818 | 0,949 | 0,950 | 0,904 | 0,996 |
| IFI44L_ISG15_HERC6_RETN | 0,860 | 0,850 | 0,870 | 0,879 | 0,803 | 0,955 | 0,951 | 0,901 | 1 |
| SIGLEC1_ISG15_SLC1A2_MMP8 | 0,860 | 0,851 | 0,869 | 0,884 | 0,818 | 0,949 | 0,936 | 0,881 | 0,991 |
| IFIT1_IFI44L_SIGLEC1_RETN | 0,860 | 0,850 | 0,870 | 0,877 | 0,803 | 0,952 | 0,936 | 0,878 | 0,994 |
| IFI27_OAS1_SIGLEC1_ISG15 | 0,860 | 0,850 | 0,869 | 0,882 | 0,818 | 0,946 | 0,942 | 0,889 | 0,996 |
| RSAD2_IFI44L_IL1R2_RETN | 0,860 | 0,850 | 0,869 | 0,883 | 0,814 | 0,952 | 0,953 | 0,909 | 0,998 |
| IFI27_IFIT1_IFI44L_SIGLEC1 | 0,860 | 0,850 | 0,869 | 0,885 | 0,821 | 0,950 | 0,946 | 0,894 | 0,997 |
| IFI44L_SIGLEC1_ISG15_RETN | 0,860 | 0,849 | 0,870 | 0,877 | 0,801 | 0,953 | 0,952 | 0,906 | 0,998 |
| RSAD2_ISG15_IL1R2_MMP8 | 0,860 | 0,851 | 0,868 | 0,883 | 0,818 | 0,948 | 0,951 | 0,904 | 0,999 |
| SLC1A2_IL1R2_OLAH_FAM20A | 0,859 | 0,850 | 0,869 | 0,887 | 0,827 | 0,947 | 0,858 | 0,752 | 0,963 |
| ISG15_OLAH_FAM20A_MMP8 | 0,859 | 0,850 | 0,869 | 0,886 | 0,820 | 0,952 | 0,947 | 0,894 | 0,999 |
| IFIT1_OLAH_FAM20A_RETN | 0,859 | 0,849 | 0,870 | 0,881 | 0,808 | 0,955 | 0,958 | 0,909 | 1 |
| RSAD2_IFI27_IFIT1_SIGLEC1 | 0,859 | 0,849 | 0,869 | 0,884 | 0,821 | 0,946 | 0,942 | 0,888 | 0,997 |
| OAS1_IFIT1_OLAH_MMP8 | 0,859 | 0,849 | 0,869 | 0,879 | 0,806 | 0,952 | 0,945 | 0,893 | 0,997 |
| RSAD2_OAS1_SIGLEC1_RETN | 0,859 | 0,849 | 0,869 | 0,874 | 0,797 | 0,951 | 0,945 | 0,893 | 0,996 |
| IFI44L_ISG15_SLC1A2_FAM20A | 0,859 | 0,849 | 0,869 | 0,882 | 0,813 | 0,952 | 0,932 | 0,853 | 1 |
| OAS1_OLAH_RETN_MMP8 | 0,859 | 0,849 | 0,869 | 0,883 | 0,809 | 0,958 | 0,959 | 0,917 | 1 |
| SLC1A2_OLAH_FAM20A_MMP8 | 0,859 | 0,849 | 0,869 | 0,890 | 0,832 | 0,949 | 0,873 | 0,777 | 0,969 |
| RSAD2_OAS1_IFIT1_OLAH | 0,859 | 0,849 | 0,869 | 0,880 | 0,806 | 0,953 | 0,941 | 0,887 | 0,996 |
| IFIT1_HERC6_FAM20A_MMP8 | 0,859 | 0,848 | 0,869 | 0,879 | 0,809 | 0,949 | 0,925 | 0,842 | 1 |
| ISG15_OLAH_FAM20A_RETN | 0,859 | 0,849 | 0,868 | 0,887 | 0,819 | 0,955 | 0,945 | 0,891 | 0,998 |
| IFI27_OAS1_ISG15_HERC6 | 0,859 | 0,849 | 0,869 | 0,882 | 0,823 | 0,942 | 0,904 | 0,834 | 0,975 |
| IFI27_OAS1_IFIT1_MMP8 | 0,859 | 0,849 | 0,868 | 0,874 | 0,803 | 0,946 | 0,913 | 0,839 | 0,987 |
| RSAD2_IL1R2_RETN_MMP8 | 0,858 | 0,849 | 0,868 | 0,882 | 0,813 | 0,952 | 0,961 | 0,921 | 1 |
| OAS1_SIGLEC1_ISG15_RETN | 0,858 | 0,848 | 0,869 | 0,877 | 0,801 | 0,952 | 0,952 | 0,904 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1_IFI44L_HERC6_FAM20A | 0,858 | 0,848 | 0,869 | 0,877 | 0,805 | 0,949 | 0,928 | 0,845 | 1 |
| OAS1_ISG15_HERC6_FAM20A | 0,858 | 0,848 | 0,868 | 0,876 | 0,806 | 0,947 | 0,926 | 0,839 | 1 |
| OAS1_IFI44L_OLAH_RETN | 0,858 | 0,848 | 0,869 | 0,881 | 0,806 | 0,956 | 0,948 | 0,900 | 0,996 |
| OAS1_ISG15_OLAH_RETN | 0,858 | 0,848 | 0,868 | 0,884 | 0,810 | 0,957 | 0,953 | 0,907 | 0,999 |
| RSAD2_IFI27_IFI44L_MMP8 | 0,858 | 0,848 | 0,868 | 0,878 | 0,808 | 0,948 | 0,909 | 0,833 | 0,985 |
| IFI27_OAS1_ISG15_MMP8 | 0,858 | 0,848 | 0,868 | 0,876 | 0,806 | 0,947 | 0,903 | 0,825 | 0,981 |
| IFI27_IFIT1_IFI44L_MMP8 | 0,858 | 0,848 | 0,868 | 0,877 | 0,807 | 0,948 | 0,912 | 0,838 | 0,986 |
| IFIT1_SIGLEC1_RETN_MMP8 | 0,858 | 0,848 | 0,868 | 0,878 | 0,804 | 0,953 | 0,947 | 0,895 | 0,998 |
| RSAD2_IFI44L_HERC6_RETN | 0,858 | 0,848 | 0,868 | 0,877 | 0,800 | 0,954 | 0,926 | 0,866 | 0,986 |
| OAS1_IFI44L_SIGLEC1_RETN | 0,858 | 0,848 | 0,868 | 0,877 | 0,800 | 0,954 | 0,947 | 0,896 | 0,998 |
| IFI44L_OLAH_RETN_MMP8 | 0,858 | 0,848 | 0,868 | 0,880 | 0,806 | 0,953 | 0,952 | 0,905 | 0,999 |
| IFI44L_ISG15_HERC6_FAM20A | 0,858 | 0,848 | 0,868 | 0,873 | 0,800 | 0,946 | 0,934 | 0,850 | 1 |
| IFI27_OAS1_IFI44L_MMP8 | 0,858 | 0,848 | 0,868 | 0,877 | 0,806 | 0,947 | 0,908 | 0,831 | 0,984 |
| RSAD2_SIGLEC1_ISG15_RETN | 0,858 | 0,847 | 0,868 | 0,878 | 0,803 | 0,953 | 0,949 | 0,899 | 0,998 |
| IFI44L_SIGLEC1_HERC6_SLC1A2 | 0,857 | 0,849 | 0,866 | 0,880 | 0,814 | 0,945 | 0,951 | 0,903 | 0,999 |
| IFI44L_ISG15_OLAH_RETN | 0,857 | 0,848 | 0,867 | 0,881 | 0,807 | 0,954 | 0,948 | 0,898 | 0,997 |
| IFIT1_HERC6_SLC1A2_FAM20A | 0,857 | 0,847 | 0,867 | 0,882 | 0,817 | 0,947 | 0,929 | 0,845 | 1 |
| ISG15_HERC6_RETN_MMP8 | 0,857 | 0,847 | 0,868 | 0,877 | 0,801 | 0,954 | 0,945 | 0,893 | 0,996 |
| OAS1_IFIT1_ISG15_OLAH | 0,857 | 0,848 | 0,867 | 0,879 | 0,806 | 0,952 | 0,945 | 0,893 | 0,997 |
| IFI44L_SIGLEC1_RETN_MMP8 | 0,857 | 0,847 | 0,868 | 0,874 | 0,797 | 0,951 | 0,952 | 0,905 | 1,000 |
| OAS1_HERC6_SLC1A2_FAM20A | 0,857 | 0,847 | 0,867 | 0,884 | 0,819 | 0,949 | 0,930 | 0,846 | 1 |
| RSAD2_OAS1_IFI44L_OLAH | 0,857 | 0,848 | 0,867 | 0,883 | 0,811 | 0,954 | 0,939 | 0,882 | 0,996 |
| OAS1_IFIT1_IFI44L_OLAH | 0,857 | 0,848 | 0,867 | 0,882 | 0,810 | 0,954 | 0,941 | 0,887 | 0,996 |
| RSAD2_HERC6_SLC1A2_FAM20A | 0,857 | 0,847 | 0,867 | 0,886 | 0,823 | 0,949 | 0,930 | 0,846 | 1 |
| IFI44L_SLC1A2_FAM20A_MMP8 | 0,857 | 0,847 | 0,867 | 0,882 | 0,814 | 0,951 | 0,925 | 0,844 | 1 |

(suite)

| Modèle | AUC modèle | IC 95% | | AUC Train | IC 95% Train | | AUC Test | IC 95% Test | |
|---|---|---|---|---|---|---|---|---|---|
| SIGLEC1_ISG15_RETN_MMP8 | 0,857 | 0,847 | 0,867 | 0,874 | 0,797 | 0,952 | 0,958 | 0,914 | 1 |
| OAS1_IFI44L_HERC6_RETN | 0,857 | 0,847 | 0,867 | 0,876 | 0,798 | 0,953 | 0,939 | 0,885 | 0,994 |
| IFI44L_HERC6_SLC1A2_RETN | 0,857 | 0,847 | 0,867 | 0,884 | 0,814 | 0,953 | 0,938 | 0,874 | 1 |
| OAS1_IFI44L_OLAH_MMP8 | 0,857 | 0,847 | 0,867 | 0,880 | 0,807 | 0,952 | 0,941 | 0,887 | 0,995 |
| RSAD2_OAS1_ISG15_OLAH | 0,857 | 0,847 | 0,866 | 0,881 | 0,808 | 0,953 | 0,945 | 0,891 | 0,998 |
| RSAD2_IFI27_OAS1_MMP8 | 0,857 | 0,847 | 0,867 | 0,877 | 0,806 | 0,947 | 0,908 | 0,830 | 0,985 |
| IFI27_SIGLEC1_ISG15_HERC6 | 0,857 | 0,846 | 0,867 | 0,882 | 0,818 | 0,946 | 0,948 | 0,898 | 0,997 |
| IFI44L_ISG15_FAM20A_RETN | 0,857 | 0,846 | 0,867 | 0,874 | 0,800 | 0,949 | 0,942 | 0,874 | 1 |
| IFI44L_ISG15_OLAH_MMP8 | 0,856 | 0,847 | 0,866 | 0,876 | 0,805 | 0,946 | 0,943 | 0,893 | 0,994 |
| RSAD2_IFI44L_SIGLEC1_RETN | 0,856 | 0,846 | 0,867 | 0,875 | 0,798 | 0,952 | 0,945 | 0,893 | 0,996 |
| IFI44L_SIGLEC1_HERC6_MMP8 | 0,856 | 0,846 | 0,866 | 0,874 | 0,800 | 0,947 | 0,914 | 0,846 | 0,982 |
| RSAD2_IFI27_ISG15_MMP8 | 0,856 | 0,846 | 0,866 | 0,877 | 0,807 | 0,948 | 0,908 | 0,831 | 0,985 |
| SLC1A2_OLAH_FAM20A_RETN | 0,856 | 0,846 | 0,866 | 0,883 | 0,821 | 0,945 | 0,862 | 0,759 | 0,965 |
| OAS1_IFIT1_SIGLEC1_SLC1A2 | 0,856 | 0,847 | 0,865 | 0,877 | 0,812 | 0,942 | 0,949 | 0,902 | 0,996 |
| OAS1_ISG15_OLAH_MMP8 | 0,856 | 0,846 | 0,866 | 0,880 | 0,807 | 0,952 | 0,948 | 0,897 | 0,999 |
| RSAD2_OAS1_HERC6_FAM20A | 0,856 | 0,846 | 0,866 | 0,875 | 0,802 | 0,947 | 0,921 | 0,833 | 1 |
| RSAD2_HERC6_FAM20A_MMP8 | 0,856 | 0,845 | 0,866 | 0,879 | 0,809 | 0,950 | 0,924 | 0,841 | 1 |
| OAS1_SIGLEC1_RETN_MMP8 | 0,856 | 0,845 | 0,866 | 0,874 | 0,798 | 0,951 | 0,951 | 0,902 | 1 |
| RSAD2_IFI44L_HERC6_FAM20A | 0,856 | 0,845 | 0,866 | 0,880 | 0,810 | 0,950 | 0,926 | 0,847 | 1 |
| IFI27_OAS1_HERC6_SLC1A2 | 0,856 | 0,846 | 0,865 | 0,882 | 0,820 | 0,943 | 0,915 | 0,840 | 0,991 |
| IFI44L_HERC6_FAM20A_MMP8 | 0,856 | 0,845 | 0,866 | 0,876 | 0,804 | 0,948 | 0,932 | 0,851 | 1 |
| IFIT1_IFI44L_HERC6_RETN | 0,856 | 0,845 | 0,866 | 0,874 | 0,795 | 0,952 | 0,927 | 0,865 | 0,989 |
| OAS1_HERC6_SLC1A2_RETN | 0,856 | 0,846 | 0,865 | 0,884 | 0,817 | 0,952 | 0,925 | 0,855 | 0,995 |
| IFIT1_ISG15_OLAH_RETN | 0,856 | 0,846 | 0,866 | 0,884 | 0,811 | 0,956 | 0,962 | 0,921 | 1 |
| RSAD2_SIGLEC1_HERC6_MMP8 | 0,855 | 0,846 | 0,865 | 0,875 | 0,805 | 0,945 | 0,901 | 0,829 | 0,973 |

| Modèle | AUC modèle | IC 95% | | AUC Train | IC 95% Train | | AUC Test | IC 95% Test | |
|---|---|---|---|---|---|---|---|---|---|
| RSAD2_IFI27_ISG15_HERC6 | 0,855 | 0,846 | 0,865 | 0,880 | 0,820 | 0,940 | 0,882 | 0,801 | 0,962 |
| IFIT1_HERC6_SLC1A2_RETN | 0,855 | 0,845 | 0,865 | 0,884 | 0,816 | 0,953 | 0,937 | 0,872 | 1 |
| OAS1_ISG15_FAM20A_RETN | 0,855 | 0,845 | 0,866 | 0,882 | 0,809 | 0,955 | 0,940 | 0,863 | 1 |
| RSAD2_IFI27_HERC6_SLC1A2 | 0,855 | 0,846 | 0,865 | 0,880 | 0,818 | 0,942 | 0,912 | 0,835 | 0,988 |
| RSAD2_SIGLEC1_RETN_MMP8 | 0,855 | 0,844 | 0,866 | 0,876 | 0,800 | 0,952 | 0,951 | 0,903 | 0,999 |
| RSAD2_IFI27_IFIT1_MMP8 | 0,855 | 0,845 | 0,865 | 0,875 | 0,804 | 0,946 | 0,911 | 0,836 | 0,986 |
| IFI27_IFI44L_HERC6_SLC1A2 | 0,855 | 0,845 | 0,864 | 0,882 | 0,820 | 0,945 | 0,923 | 0,853 | 0,993 |
| RSAD2_IFI27_IFI44L_HERC6 | 0,855 | 0,845 | 0,865 | 0,880 | 0,820 | 0,939 | 0,891 | 0,816 | 0,967 |
| OAS1_IFIT1_SIGLEC1_MMP8 | 0,855 | 0,845 | 0,864 | 0,871 | 0,798 | 0,945 | 0,905 | 0,835 | 0,976 |
| OAS1_IFIT1_HERC6_RETN | 0,854 | 0,844 | 0,865 | 0,872 | 0,795 | 0,949 | 0,934 | 0,873 | 0,994 |
| IFIT1_SIGLEC1_ISG15_SLC1A2 | 0,854 | 0,846 | 0,863 | 0,878 | 0,813 | 0,943 | 0,950 | 0,904 | 0,996 |
| RSAD2_OLAH_RETN_MMP8 | 0,854 | 0,844 | 0,865 | 0,877 | 0,801 | 0,952 | 0,965 | 0,927 | 1 |
| RSAD2_OAS1_FAM20A_RETN | 0,854 | 0,844 | 0,865 | 0,880 | 0,807 | 0,954 | 0,929 | 0,847 | 1 |
| OAS1_HERC6_FAM20A_MMP8 | 0,854 | 0,844 | 0,865 | 0,878 | 0,807 | 0,950 | 0,927 | 0,845 | 1 |
| RSAD2_ISG15_OLAH_RETN | 0,854 | 0,844 | 0,864 | 0,877 | 0,801 | 0,952 | 0,966 | 0,929 | 1 |
| OAS1_SIGLEC1_HERC6_MMP8 | 0,854 | 0,845 | 0,864 | 0,875 | 0,806 | 0,944 | 0,914 | 0,847 | 0,981 |
| RSAD2_HERC6_SLC1A2_RETN | 0,854 | 0,845 | 0,864 | 0,885 | 0,817 | 0,952 | 0,930 | 0,863 | 0,997 |
| SIGLEC1_ISG15_HERC6_SLC1A2 | 0,854 | 0,846 | 0,863 | 0,880 | 0,814 | 0,946 | 0,950 | 0,901 | 0,999 |
| IFIT1_OLAH_RETN_MMP8 | 0,854 | 0,844 | 0,865 | 0,876 | 0,799 | 0,952 | 0,966 | 0,929 | 1 |
| IFIT1_IFI44L_OLAH_MMP8 | 0,854 | 0,844 | 0,864 | 0,874 | 0,801 | 0,947 | 0,946 | 0,895 | 0,996 |
| RSAD2_IFIT1_OLAH_RETN | 0,854 | 0,844 | 0,864 | 0,881 | 0,805 | 0,957 | 0,958 | 0,914 | 1 |
| RSAD2_OAS1_HERC6_RETN | 0,854 | 0,844 | 0,864 | 0,871 | 0,793 | 0,948 | 0,927 | 0,864 | 0,990 |
| OAS1_IFI44L_ISG15_OLAH | 0,854 | 0,844 | 0,864 | 0,882 | 0,811 | 0,953 | 0,945 | 0,893 | 0,996 |
| IFI27_ISG15_HERC6_SLC1A2 | 0,854 | 0,844 | 0,864 | 0,882 | 0,819 | 0,945 | 0,921 | 0,847 | 0,994 |
| OAS1_SLC1A2_FAM20A_RETN | 0,854 | 0,844 | 0,864 | 0,881 | 0,811 | 0,951 | 0,930 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1_IFI44L_SIGLEC1_SLC1A2 | 0,854 | 0,845 | 0,863 | 0,879 | 0,814 | 0,944 | 0,947 | 0,899 | 0,995 |
| IFI27_IFIT1_HERC6_SLC1A2 | 0,854 | 0,844 | 0,864 | 0,883 | 0,819 | 0,946 | 0,939 | 0,876 | 1 |
| RSAD2_IFI27_IFIT1_HERC6 | 0,854 | 0,843 | 0,864 | 0,882 | 0,822 | 0,943 | 0,912 | 0,844 | 0,980 |
| RSAD2_IFIT1_SIGLEC1_SLC1A2 | 0,854 | 0,845 | 0,863 | 0,878 | 0,812 | 0,943 | 0,950 | 0,904 | 0,996 |
| IFIT1_IFI44L_OLAH_RETN | 0,854 | 0,843 | 0,864 | 0,879 | 0,803 | 0,954 | 0,947 | 0,897 | 0,997 |
| IFIT1_IFI44L_ISG15_OLAH | 0,853 | 0,844 | 0,863 | 0,877 | 0,806 | 0,949 | 0,946 | 0,896 | 0,995 |
| OAS1_SIGLEC1_ISG15_SLC1A2 | 0,853 | 0,845 | 0,862 | 0,877 | 0,812 | 0,942 | 0,952 | 0,907 | 0,997 |
| RSAD2_OAS1_SIGLEC1_SLC1A2 | 0,853 | 0,844 | 0,863 | 0,876 | 0,810 | 0,941 | 0,948 | 0,900 | 0,996 |
| IFI27_OAS1_IFI44L_HERC6 | 0,853 | 0,843 | 0,864 | 0,879 | 0,818 | 0,940 | 0,904 | 0,834 | 0,975 |
| IFIT1_ISG15_OLAH_MMP8 | 0,853 | 0,844 | 0,863 | 0,874 | 0,801 | 0,947 | 0,961 | 0,921 | 1 |
| ISG15_OLAH_RETN_MMP8 | 0,853 | 0,843 | 0,863 | 0,879 | 0,811 | 0,948 | 0,957 | 0,912 | 1 |
| IFI27_OAS1_IFIT1_HERC6 | 0,853 | 0,843 | 0,863 | 0,880 | 0,818 | 0,942 | 0,925 | 0,862 | 0,988 |
| OAS1_IFI44L_SIGLEC1_SLC1A2 | 0,853 | 0,844 | 0,862 | 0,876 | 0,811 | 0,942 | 0,952 | 0,907 | 0,997 |
| HERC6_SLC1A2_RETN_MMP8 | 0,853 | 0,843 | 0,863 | 0,882 | 0,814 | 0,950 | 0,923 | 0,853 | 0,993 |
| RSAD2_IFI27_OAS1_HERC6 | 0,853 | 0,843 | 0,863 | 0,879 | 0,819 | 0,940 | 0,884 | 0,804 | 0,963 |
| OAS1_IFIT1_FAM20A_RETN | 0,853 | 0,843 | 0,863 | 0,877 | 0,801 | 0,952 | 0,935 | 0,853 | 1 |
| RSAD2_IFI44L_SIGLEC1_SLC1A2 | 0,853 | 0,844 | 0,862 | 0,878 | 0,813 | 0,943 | 0,946 | 0,897 | 0,995 |
| RSAD2_IFIT1_HERC6_RETN | 0,853 | 0,843 | 0,863 | 0,872 | 0,794 | 0,949 | 0,926 | 0,863 | 0,989 |
| IFI27_OAS1_IFIT1_SLC1A2 | 0,853 | 0,843 | 0,863 | 0,874 | 0,805 | 0,944 | 0,949 | 0,891 | 1 |
| RSAD2_SIGLEC1_ISG15_SLC1A2 | 0,853 | 0,844 | 0,861 | 0,878 | 0,813 | 0,943 | 0,948 | 0,900 | 0,995 |
| IFIT1_SLC1A2_FAM20A_RETN | 0,853 | 0,843 | 0,863 | 0,879 | 0,807 | 0,951 | 0,932 | 0,852 | 1 |
| IFI44L_SIGLEC1_ISG15_SLC1A2 | 0,853 | 0,844 | 0,861 | 0,879 | 0,813 | 0,945 | 0,952 | 0,907 | 0,997 |
| OAS1_IFI44L_HERC6_FAM20A | 0,853 | 0,842 | 0,863 | 0,872 | 0,799 | 0,945 | 0,935 | 0,852 | 1 |
| RSAD2_ISG15_OLAH_MMP8 | 0,853 | 0,843 | 0,862 | 0,877 | 0,805 | 0,948 | 0,953 | 0,908 | 0,998 |
| RSAD2_IFIT1_OLAH_MMP8 | 0,852 | 0,843 | 0,862 | 0,875 | 0,802 | 0,948 | 0,951 | 0,905 | 0,997 |

EP 4 365 308 A1

80

| Modèle | AUC modèle | IC 95% | | AUC Train | IC 95% Train | | AUC Test | IC 95% Test | |
|---|---|---|---|---|---|---|---|---|---|
| IFI44L_HERC6_RETN_MMP8 | 0,852 | 0,842 | 0,863 | 0,873 | 0,793 | 0,952 | 0,921 | 0,856 | 0,985 |
| RSAD2_IFI44L_OLAH_RETN | 0,852 | 0,842 | 0,863 | 0,879 | 0,804 | 0,954 | 0,953 | 0,908 | 0,999 |
| ISG15_HERC6_SLC1A2_MMP8 | 0,852 | 0,843 | 0,861 | 0,881 | 0,814 | 0,947 | 0,905 | 0,828 | 0,983 |
| RSAD2_IFI44L_OLAH_MMP8 | 0,852 | 0,842 | 0,862 | 0,874 | 0,801 | 0,947 | 0,946 | 0,895 | 0,996 |
| IFIT1_ IFI44L_ SLC1A2_ FAM20A | 0,852 | 0,842 | 0,862 | 0,878 | 0,809 | 0,947 | 0,923 | 0,840 | 1 |
| SLC1A2_ IL1R2_ OLAH_ RETN | 0,852 | 0,843 | 0,861 | 0,879 | 0,818 | 0,941 | 0,840 | 0,729 | 0,951 |
| IFI27_ IFI44L_ ISG15_ SLC1A2 | 0,852 | 0,842 | 0,861 | 0,879 | 0,812 | 0,946 | 0,929 | 0,866 | 0,992 |
| SIGLEC1_ ISG15_ HERC6_ MMP8 | 0,852 | 0,842 | 0,862 | 0,871 | 0,795 | 0,946 | 0,915 | 0,849 | 0,981 |
| RSAD2_ IFIT1_ SIGLEC1_ MMP8 | 0,852 | 0,841 | 0,862 | 0,871 | 0,798 | 0,945 | 0,907 | 0,836 | 0,977 |
| RSAD2_ IFIT1_ ISG15_ OLAH | 0,852 | 0,842 | 0,861 | 0,877 | 0,804 | 0,949 | 0,957 | 0,914 | 0,999 |
| IFIT1_ HERC6_ RETN_ MMP8 | 0,852 | 0,841 | 0,862 | 0,872 | 0,795 | 0,949 | 0,914 | 0,846 | 0,982 |
| OAS1_ IFIT1_ HERC6_ FAM20A | 0,852 | 0,841 | 0,862 | 0,872 | 0,799 | 0,945 | 0,932 | 0,847 | 1 |
| SLC1A2_ IL1R2_ OLAH_ MMP8 | 0,852 | 0,843 | 0,861 | 0,879 | 0,818 | 0,940 | 0,850 | 0,745 | 0,955 |
| OAS1_ IFIT1_ SIGLEC1_ HERC6 | 0,852 | 0,842 | 0,861 | 0,869 | 0,802 | 0,936 | 0,911 | 0,844 | 0,978 |
| RSAD2_ IFI44L_ SLC1A2_ FAM20A | 0,851 | 0,841 | 0,862 | 0,880 | 0,812 | 0,949 | 0,920 | 0,838 | 1 |
| ISG15_ SLC1A2_ FAM20A_ RETN | 0,851 | 0,841 | 0,861 | 0,880 | 0,812 | 0,948 | 0,921 | 0,837 | 1 |
| IFI44L_ HERC6_ SLC1A2_ MMP8 | 0,851 | 0,842 | 0,860 | 0,877 | 0,809 | 0,944 | 0,904 | 0,828 | 0,981 |
| OAS1_ IFI44L_ FAM20A_ RETN | 0,851 | 0,841 | 0,862 | 0,875 | 0,800 | 0,951 | 0,939 | 0,863 | 1 |
| RSAD2_ IFI27_ IFI44L_ SLC1A2 | 0,851 | 0,841 | 0,861 | 0,877 | 0,809 | 0,944 | 0,925 | 0,862 | 0,988 |
| IFIT1_ ISG15_ SLC1A2_ FAM20A | 0,851 | 0,840 | 0,861 | 0,873 | 0,800 | 0,946 | 0,937 | 0,857 | 1 |
| IFI27_ IFI44L_ ISG15_ HERC6 | 0,851 | 0,840 | 0,861 | 0,877 | 0,815 | 0,940 | 0,909 | 0,840 | 0,977 |
| IFI27_ IFIT1_ ISG15_ SLC1A2 | 0,851 | 0,841 | 0,861 | 0,877 | 0,808 | 0,945 | 0,949 | 0,893 | 1 |
| RSAD2_ IFI44L_ ISG15_ OLAH | 0,850 | 0,841 | 0,860 | 0,877 | 0,805 | 0,948 | 0,943 | 0,893 | 0,994 |
| OAS1_ IFI44L_ SLC1A2_ FAM20A | 0,850 | 0,840 | 0,861 | 0,880 | 0,811 | 0,949 | 0,929 | 0,847 | 1 |
| RSAD2_ IFIT1_ IFI44L_ OLAH | 0,850 | 0,840 | 0,860 | 0,877 | 0,804 | 0,949 | 0,947 | 0,897 | 0,996 |

(suite)

| Modèle | AUC modèle | IC 95% | | AUC Train | IC 95% Train | | AUC Test | IC 95% Test | |
|---|---|---|---|---|---|---|---|---|---|
| IFIT1_ HERC6_ SLC1A2_ MMP8 | 0,850 | 0,841 | 0,859 | 0,876 | 0,808 | 0,944 | 0,899 | 0,818 | 0,980 |
| SLC1A2_ OLAH_ RETN_ MMP8 | 0,850 | 0,840 | 0,860 | 0,881 | 0,819 | 0,942 | 0,858 | 0,757 | 0,958 |
| OAS1_ HERC6_ RETN_ MMP8 | 0,850 | 0,840 | 0,861 | 0,873 | 0,797 | 0,949 | 0,917 | 0,851 | 0,984 |
| RSAD2_ IFI27_ IFIT1_ SLC1A2 | 0,850 | 0,840 | 0,860 | 0,875 | 0,806 | 0,944 | 0,948 | 0,890 | 1 |

Performances obtenues pour l'identification de la nature virale ou bactérienne de l'infection sur la base des combinaisons à 5 gènes :

[Tableau 9]

| Modèle | AUC modèle | IC 95% | | AUC Train | IC 95% Train | | AUC Test | IC 95% Test | |
|---|---|---|---|---|---|---|---|---|---|
| IFI27_ SIGLEC1_ IL1R2_ FAM20A_ MMP8 | 0,910 | 0,902 | 0,918 | 0,930 | 0,877 | 0,983 | 0,957 | 0,889 | 1 |
| IFI27_ IL1R2_ OLAH_ FAM20A_ MMP8 | 0,907 | 0,899 | 0,916 | 0,927 | 0,875 | 0,980 | 0,924 | 0,847 | 1 |
| IFI27_ OAS1_ HERC6_ OLAH_ FAM20A | 0,907 | 0,899 | 0,916 | 0,927 | 0,873 | 0,982 | 0,929 | 0,857 | 1 |
| RSAD2_ IFI27_ HERC6_ OLAH_ FAM20A | 0,907 | 0,899 | 0,916 | 0,927 | 0,872 | 0,981 | 0,930 | 0,860 | 1 |
| IFI27_ IFIT1_ SIGLEC1_ IL1R2_ FAM20A | 0,907 | 0,899 | 0,915 | 0,926 | 0,870 | 0,982 | 0,949 | 0,886 | 1 |
| IFI27_ IFIT1_ SIGLEC1_ OLAH_ FAM20A | 0,907 | 0,898 | 0,915 | 0,925 | 0,866 | 0,984 | 0,953 | 0,898 | 1 |
| IFI27_ IFIT1_ HERC6_ OLAH_ FAM20A | 0,907 | 0,898 | 0,915 | 0,925 | 0,869 | 0,981 | 0,934 | 0,866 | 1 |
| IFI27_ ISG15_ HERC6_ OLAH_ FAM20A | 0,906 | 0,898 | 0,915 | 0,925 | 0,868 | 0,982 | 0,936 | 0,870 | 1 |
| IFI27_ HERC6_ IL1R2_ OLAH_ FAM20A | 0,906 | 0,898 | 0,914 | 0,926 | 0,872 | 0,981 | 0,936 | 0,865 | 1 |
| IFI27_ IFI44L_ SIGLEC1_ IL1R2_ FAM20A | 0,906 | 0,898 | 0,914 | 0,927 | 0,873 | 0,982 | 0,950 | 0,884 | 1 |
| IFI27_ OAS1_ SIGLEC1_ IL1R2_ FAM20A | 0,906 | 0,898 | 0,914 | 0,928 | 0,872 | 0,983 | 0,951 | 0,888 | 1 |
| RSAD2_ IFI27_ SIGLEC1_ IL1R2_ FAM20A | 0,906 | 0,898 | 0,914 | 0,929 | 0,874 | 0,984 | 0,947 | 0,879 | 1 |
| IFI27_ SIGLEC1_ HERC6_ IL1R2_ FAM20A | 0,906 | 0,897 | 0,914 | 0,926 | 0,870 | 0,982 | 0,951 | 0,884 | 1 |
| IFI27_ IFI44L_ IL1R2_ FAM20A_ MMP8 | 0,905 | 0,897 | 0,913 | 0,927 | 0,874 | 0,979 | 0,927 | 0,844 | 1 |
| IFI27_ SIGLEC1_ ISG15_ IL1R2_ FAM20A | 0,905 | 0,897 | 0,913 | 0,927 | 0,873 | 0,982 | 0,950 | 0,883 | 1 |
| IFI27_ ISG15_ OLAH_ FAM20A_ MMP8 | 0,905 | 0,896 | 0,914 | 0,921 | 0,862 | 0,979 | 0,928 | 0,856 | 1 |
| IFI27_ IFI44L_ HERC6_ OLAH_ FAM20A | 0,905 | 0,896 | 0,914 | 0,923 | 0,865 | 0,981 | 0,941 | 0,877 | 1 |
| IFI27_ SIGLEC1_ ISG15_ OLAH_ FAM20A | 0,905 | 0,896 | 0,914 | 0,922 | 0,861 | 0,983 | 0,950 | 0,891 | 1 |
| IFI27_ HERC6_ OLAH_ FAM20A_ MMP8 | 0,905 | 0,896 | 0,914 | 0,923 | 0,864 | 0,981 | 0,941 | 0,877 | 1 |
| IFI27_ OAS1_ SIGLEC1_ OLAH_ FAM20A | 0,905 | 0,896 | 0,913 | 0,923 | 0,862 | 0,983 | 0,950 | 0,892 | 1 |
| IFI27_ ISG15_ IL1R2_ OLAH_ FAM20A | 0,905 | 0,897 | 0,913 | 0,924 | 0,868 | 0,981 | 0,925 | 0,850 | 1,000 |
| IFI27_ SIGLEC1_ OLAH_ FAM20A_ MMP8 | 0,905 | 0,896 | 0,913 | 0,921 | 0,859 | 0,983 | 0,953 | 0,895 | 1 |
| RSAD2_ IFI27_ HERC6_ IL1R2_ FAM20A | 0,905 | 0,897 | 0,913 | 0,928 | 0,878 | 0,978 | 0,927 | 0,845 | 1 |
| IFI27_ OAS1_ HERC6_ IL1R2_ FAM20A | 0,904 | 0,897 | 0,912 | 0,928 | 0,878 | 0,978 | 0,922 | 0,836 | 1 |
| IFI27_ HERC6_ IL1R2_ FAM20A_ MMP8 | 0,904 | 0,896 | 0,912 | 0,929 | 0,879 | 0,979 | 0,925 | 0,840 | 1 |

| Modèle | AUC modèle | IC 95% | | AUC Train | IC 95% Train | | AUC Test | IC 95% Test | |
|---|---|---|---|---|---|---|---|---|---|
| IFI27_ IFI44L_ HERC6_ IL1R2_ FAM20A | 0,904 | 0,896 | 0,912 | 0,927 | 0,875 | 0,978 | 0,930 | 0,849 | 1 |
| IFI27_ IFI44L_ SIGLEC1_ OLAH_ FAM20A | 0,904 | 0,896 | 0,913 | 0,921 | 0,860 | 0,982 | 0,953 | 0,894 | 1 |
| IFI27_ SIGLEC1_ IL1R2_ OLAH_ FAM20A | 0,904 | 0,896 | 0,912 | 0,924 | 0,866 | 0,982 | 0,950 | 0,890 | 1 |
| RSAD2_ IFI27_ SIGLEC1_ OLAH_ FAM20A | 0,904 | 0,895 | 0,912 | 0,926 | 0,867 | 0,985 | 0,950 | 0,891 | 1 |
| IFI27_ IFIT1_ HERC6_ IL1R2_ FAM20A | 0,904 | 0,896 | 0,912 | 0,927 | 0,876 | 0,978 | 0,932 | 0,852 | 1 |
| IFI27_ SIGLEC1_ HERC6_ OLAH_ FAM20A | 0,903 | 0,895 | 0,912 | 0,919 | 0,856 | 0,981 | 0,949 | 0,888 | 1 |
| IFI27_ HERC6_ IL1R2_ FAM20A_ RETN | 0,903 | 0,895 | 0,912 | 0,931 | 0,880 | 0,982 | 0,929 | 0,849 | 1 |
| IFI27_ ISG15_ HERC6_ IL1R2_ FAM20A | 0,903 | 0,895 | 0,911 | 0,927 | 0,876 | 0,978 | 0,927 | 0,845 | 1 |
| IFI27_ IFI44L_ IL1R2_ OLAH_ FAM20A | 0,903 | 0,895 | 0,911 | 0,924 | 0,866 | 0,981 | 0,930 | 0,858 | 1 |
| IFI27_ IFI44L_ OLAH_ FAM20A_ MMP8 | 0,903 | 0,894 | 0,912 | 0,922 | 0,862 | 0,981 | 0,939 | 0,875 | 1 |
| IFI27_ SIGLEC1_ IL1R2_ FAM20A_ RETN | 0,903 | 0,895 | 0,911 | 0,926 | 0,871 | 0,982 | 0,949 | 0,881 | 1 |
| IFI27_ IFIT1_ IL1R2_ FAM20A_ MMP8 | 0,903 | 0,895 | 0,911 | 0,926 | 0,874 | 0,978 | 0,935 | 0,857 | 1 |
| IFI27_ HERC6_ OLAH_ FAM20A_ RETN | 0,902 | 0,894 | 0,911 | 0,922 | 0,863 | 0,982 | 0,942 | 0,878 | 1 |
| IFI27_ IFIT1_ OLAH_ FAM20A_ MMP8 | 0,902 | 0,893 | 0,911 | 0,920 | 0,860 | 0,981 | 0,941 | 0,879 | 1 |
| IFI27_ OLAH_ FAM20A_ RETN_ MMP8 | 0,902 | 0,893 | 0,911 | 0,923 | 0,865 | 0,981 | 0,929 | 0,857 | 1 |
| IFI27_ IFI44L_ ISG15_ OLAH_ FAM20A | 0,902 | 0,894 | 0,911 | 0,920 | 0,859 | 0,981 | 0,932 | 0,862 | 1 |
| IFI27_ IFIT1_ IL1R2_ OLAH_ FAM20A | 0,902 | 0,894 | 0,910 | 0,924 | 0,867 | 0,981 | 0,933 | 0,863 | 1 |
| IFI27_ IL1R2_ OLAH_ FAM20A_ RETN | 0,902 | 0,894 | 0,910 | 0,924 | 0,869 | 0,980 | 0,928 | 0,855 | 1 |
| IFI27_ OAS1_ SIGLEC1_ HERC6_ RETN | 0,902 | 0,893 | 0,910 | 0,919 | 0,859 | 0,978 | 0,948 | 0,887 | 1 |
| RSAD2_ IFI27_ OLAH_ FAM20A_ MMP8 | 0,902 | 0,893 | 0,910 | 0,922 | 0,863 | 0,980 | 0,935 | 0,867 | 1 |
| RSAD2_ IFI27_ IL1R2_ OLAH_ FAM20A | 0,902 | 0,893 | 0,910 | 0,924 | 0,868 | 0,980 | 0,930 | 0,858 | 1 |
| IFI27_ IFIT1_ SIGLEC1_ HERC6_ FAM20A | 0,902 | 0,894 | 0,910 | 0,920 | 0,861 | 0,980 | 0,941 | 0,866 | 1 |
| IFI27_ OAS1_ OLAH_ FAM20A_ MMP8 | 0,902 | 0,893 | 0,910 | 0,921 | 0,862 | 0,980 | 0,938 | 0,874 | 1 |
| IFI27_ OAS1_ IL1R2_ OLAH_ FAM20A | 0,901 | 0,893 | 0,909 | 0,923 | 0,866 | 0,980 | 0,933 | 0,862 | 1 |
| IFI27_ IFIT1_ IFI44L_ OLAH_ FAM20A | 0,901 | 0,893 | 0,910 | 0,920 | 0,859 | 0,981 | 0,939 | 0,873 | 1 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| IFI27_ ISG15_ OLAH_ FAM20A_ RETN | 0,901 | 0,893 | 0,910 | 0,921 | 0,863 | 0,980 | 0,934 | 0,864 | 1 |
| IFI27_ IFIT1_ SIGLEC1_ FAM20A_ RETN | 0,901 | 0,893 | 0,910 | 0,919 | 0,859 | 0,979 | 0,948 | 0,880 | 1 |
| RSAD2_ IFI27_ IL1R2_ FAM20A_ MMP8 | 0,901 | 0,893 | 0,909 | 0,926 | 0,875 | 0,978 | 0,927 | 0,841 | 1 |
| IFI27_ OAS1_ IL1R2_ FAM20A_ MMP8 | 0,901 | 0,893 | 0,909 | 0,925 | 0,873 | 0,977 | 0,929 | 0,846 | 1 |
| IFI27_ ISG15_ IL1R2_ FAM20A_ MMP8 | 0,901 | 0,893 | 0,909 | 0,922 | 0,870 | 0,975 | 0,917 | 0,829 | 1 |
| IFI44L_ SIGLEC1_ HERC6_ OLAH | 0,901 | 0,893 | 0,909 | 0,925 | 0,872 | 0,978 | 0,946 | 0,895 | 0,997 |
| IFI44L_ ISG15_ IL1R2_ FAM20A | 0,901 | 0,893 | 0,909 | 0,924 | 0,870 | 0,978 | 0,927 | 0,845 | 1 |
| IFI44L_ IL1R2_ FAM20A_ RETN | 0,901 | 0,892 | 0,909 | 0,924 | 0,871 | 0,978 | 0,927 | 0,846 | 1 |
| IFI27_ OAS1_ ISG15_ OLAH_ FAM20A | 0,901 | 0,892 | 0,909 | 0,920 | 0,860 | 0,980 | 0,934 | 0,866 | 1 |
| IFI27_ IFIT1_ ISG15_ OLAH_ FAM20A | 0,901 | 0,892 | 0,909 | 0,920 | 0,860 | 0,980 | 0,935 | 0,867 | 1 |
| IFI27_ SLC1A2_ OLAH_ FAM20A_ MMP8 | 0,900 | 0,891 | 0,909 | 0,926 | 0,871 | 0,980 | 0,923 | 0,844 | 1 |
| IFI27_ IFIT1_ IFI44L_ IL1R2_ FAM20A | 0,900 | 0,892 | 0,908 | 0,922 | 0,867 | 0,976 | 0,930 | 0,851 | 1 |
| RSAD2_ IFI27_ HERC6_ IL1R2_ OLAH | 0,900 | 0,892 | 0,908 | 0,926 | 0,877 | 0,975 | 0,938 | 0,882 | 0,994 |
| IFI27_ OAS1_ IFI44L_ OLAH_ FAM20A | 0,900 | 0,891 | 0,909 | 0,920 | 0,860 | 0,981 | 0,936 | 0,867 | 1 |
| IFI27_ SIGLEC1_ OLAH_ FAM20A_ RETN | 0,900 | 0,891 | 0,909 | 0,918 | 0,856 | 0,981 | 0,951 | 0,891 | 1 |
| IFI27_ OAS1_ IFIT1_ OLAH_ FAM20A | 0,900 | 0,891 | 0,908 | 0,920 | 0,859 | 0,981 | 0,936 | 0,867 | 1 |
| IFI27_ OAS1_ HERC6_ FAM20A_ RETN | 0,900 | 0,891 | 0,909 | 0,923 | 0,865 | 0,981 | 0,934 | 0,853 | 1 |
| IFI27_ ISG15_ SLC1A2_ OLAH_ FAM20A | 0,900 | 0,891 | 0,909 | 0,927 | 0,873 | 0,981 | 0,917 | 0,836 | 0,998 |
| IFI27_ OAS1_ HERC6_ OLAH_ RETN | 0,900 | 0,891 | 0,908 | 0,923 | 0,867 | 0,978 | 0,939 | 0,878 | 1,000 |
| RSAD2_ IFI27_ SIGLEC1_ HERC6_ OLAH | 0,900 | 0,891 | 0,908 | 0,923 | 0,871 | 0,975 | 0,939 | 0,884 | 0,994 |
| IFI27_ SLC1A2_ IL1R2_ OLAH_ FAM20A | 0,900 | 0,891 | 0,908 | 0,927 | 0,875 | 0,980 | 0,918 | 0,835 | 1 |
| RSAD2_ IFI27_ HERC6_ OLAH_ MMP8 | 0,900 | 0,892 | 0,908 | 0,922 | 0,870 | 0,975 | 0,935 | 0,876 | 0,993 |
| IFI27_ OAS1_ HERC6_ OLAH_ MMP8 | 0,900 | 0,891 | 0,908 | 0,923 | 0,871 | 0,975 | 0,935 | 0,876 | 0,994 |
| RSAD2_ IFI27_ ISG15_ OLAH_ FAM20A | 0,900 | 0,891 | 0,908 | 0,918 | 0,857 | 0,979 | 0,934 | 0,866 | 1 |
| IFI27_ OAS1_ HERC6_ IL1R2_ OLAH | 0,900 | 0,892 | 0,907 | 0,927 | 0,879 | 0,976 | 0,938 | 0,880 | 0,996 |

| Modèle | AUC modèle | IC 95% | | AUC Train | IC 95% Train | | AUC Test | IC 95% Test | |
|---|---|---|---|---|---|---|---|---|---|
| RSAD2_ IFI27_ OAS1_ OLAH_ FAM20A | 0,899 | 0,891 | 0,908 | 0,921 | 0,861 | 0,981 | 0,937 | 0,870 | 1 |
| IFI27_ IFI44L_ HERC6_ IL1R2_ OLAH | 0,899 | 0,891 | 0,908 | 0,927 | 0,878 | 0,976 | 0,937 | 0,880 | 0,993 |
| IFI27_ IFI44L_ HERC6_ OLAH_ MMP8 | 0,899 | 0,891 | 0,908 | 0,923 | 0,869 | 0,976 | 0,935 | 0,876 | 0,994 |
| IFI27_ IFIT1_ HERC6_ OLAH_ RETN | 0,899 | 0,891 | 0,908 | 0,920 | 0,863 | 0,977 | 0,942 | 0,885 | 1,000 |
| IFI27_ OAS1_ SIGLEC1_ HERC6_ OLAH | 0,899 | 0,891 | 0,907 | 0,922 | 0,869 | 0,975 | 0,940 | 0,885 | 0,996 |
| IFI27_ OAS1_ SIGLEC1_ HERC6_ IL1R2 | 0,899 | 0,892 | 0,907 | 0,922 | 0,876 | 0,969 | 0,949 | 0,902 | 0,996 |
| RSAD2_ IFI27_ HERC6_ FAM20A_ RETN | 0,899 | 0,890 | 0,908 | 0,922 | 0,864 | 0,979 | 0,934 | 0,854 | 1 |
| RSAD2_ IFI27_ IFI44L_ OLAH_ FAM20A | 0,899 | 0,890 | 0,908 | 0,923 | 0,863 | 0,983 | 0,934 | 0,864 | 1 |
| IFI27_ OAS1_ IFIT1_ HERC6_ OLAH | 0,899 | 0,891 | 0,907 | 0,924 | 0,871 | 0,976 | 0,935 | 0,876 | 0,994 |
| RSAD2_ IFI27_ IFIT1_ OLAH_ FAM20A | 0,899 | 0,890 | 0,907 | 0,920 | 0,860 | 0,980 | 0,937 | 0,870 | 1 |
| RSAD2_ IFI27_ OAS1_ HERC6_ OLAH | 0,899 | 0,891 | 0,907 | 0,923 | 0,872 | 0,975 | 0,933 | 0,873 | 0,992 |
| IFI27_ OAS1_ IFIT1_ IL1R2_ FAM20A | 0,899 | 0,891 | 0,907 | 0,923 | 0,870 | 0,977 | 0,929 | 0,850 | 1 |
| RSAD2_ IFI27_ IFIT1_ IL1R2_ FAM20A | 0,899 | 0,891 | 0,907 | 0,923 | 0,869 | 0,977 | 0,929 | 0,850 | 1 |
| IFIT1_ SIGLEC1_ IL1R2_ FAM20A_ MMP8 | 0,899 | 0,891 | 0,907 | 0,921 | 0,864 | 0,977 | 0,965 | 0,913 | 1 |
| IFI27_ OAS1_ IFI44L_ IL1R2_ FAM20A | 0,899 | 0,890 | 0,907 | 0,923 | 0,869 | 0,977 | 0,930 | 0,851 | 1 |
| IFI27_ IFIT1_ HERC6_ OLAH_ MMP8 | 0,899 | 0,890 | 0,907 | 0,923 | 0,870 | 0,976 | 0,939 | 0,882 | 0,996 |
| IFI27_ IFI44L_ SIGLEC1_ IL1R2_ OLAH | 0,899 | 0,891 | 0,906 | 0,923 | 0,872 | 0,974 | 0,947 | 0,896 | 0,997 |
| IFI27_ HERC6_ OLAH_ RETN_ MMP8 | 0,899 | 0,890 | 0,908 | 0,919 | 0,858 | 0,979 | 0,958 | 0,910 | 1 |
| IFI27_ IFIT1_ HERC6_ FAM20A_ RETN | 0,899 | 0,890 | 0,908 | 0,922 | 0,864 | 0,980 | 0,933 | 0,851 | 1 |
| IFI27_ IFI44L_ OLAH_ FAM20A_ RETN | 0,899 | 0,890 | 0,907 | 0,919 | 0,857 | 0,980 | 0,936 | 0,866 | 1 |
| IFI27_ IFI44L_ ISG15_ HERC6_ OLAH | 0,899 | 0,890 | 0,907 | 0,924 | 0,872 | 0,976 | 0,934 | 0,874 | 0,993 |
| IFI27_ IFI44L_ HERC6_ OLAH_ RETN | 0,899 | 0,890 | 0,907 | 0,921 | 0,865 | 0,977 | 0,945 | 0,890 | 0,999 |
| RSAD2_ IFI27_ IFI44L_ IL1R2_ FAM20A | 0,899 | 0,890 | 0,907 | 0,923 | 0,869 | 0,978 | 0,929 | 0,849 | 1 |
| IFI27_ IFIT1_ OLAH_ FAM20A_ RETN | 0,899 | 0,890 | 0,907 | 0,918 | 0,856 | 0,979 | 0,936 | 0,866 | 1 |
| IFI27_ IFIT1_ SIGLEC1_ HERC6_ OLAH | 0,899 | 0,890 | 0,907 | 0,922 | 0,868 | 0,976 | 0,943 | 0,892 | 0,995 |

| Modèle | AUC modèle | IC 95% | | AUC Train | IC 95% Train | | AUC Test | IC 95% Test | |
|---|---|---|---|---|---|---|---|---|---|
| IFI27_ OAS1_ OLAH_ FAM20A_ RETN | 0,898 | 0,890 | 0,907 | 0,918 | 0,856 | 0,979 | 0,937 | 0,869 | 1 |
| IFI27_ SIGLEC1_ ISG15_ SLC1A2_ OLAH | 0,898 | 0,890 | 0,906 | 0,929 | 0,878 | 0,980 | 0,939 | 0,879 | 0,999 |
| IFI27_ ISG15_ IL1R2_ OLAH_ MMP8 | 0,898 | 0,891 | 0,906 | 0,922 | 0,871 | 0,973 | 0,937 | 0,878 | 0,996 |
| RSAD2_ IFI27_ HERC6_ OLAH_ RETN | 0,898 | 0,890 | 0,907 | 0,922 | 0,867 | 0,977 | 0,939 | 0,879 | 0,999 |
| IFI27_ SIGLEC1_ SLC1A2_ OLAH_ FAM20A | 0,898 | 0,889 | 0,907 | 0,925 | 0,867 | 0,983 | 0,945 | 0,877 | 1 |
| IFI27_ OAS1_ SIGLEC1_ FAM20A_ RETN | 0,898 | 0,889 | 0,907 | 0,917 | 0,855 | 0,979 | 0,943 | 0,872 | 1 |
| IFI27_ HERC6_ SLC1A2_ OLAH_ FAM20A | 0,898 | 0,889 | 0,907 | 0,926 | 0,871 | 0,981 | 0,926 | 0,847 | 1 |
| IFIT1_ SIGLEC1_ HERC6_ IL1R2_ FAM20A | 0,898 | 0,890 | 0,907 | 0,919 | 0,862 | 0,976 | 0,953 | 0,897 | 1 |
| RSAD2_ IFI27_ SIGLEC1_ HERC6_ FAM20A | 0,898 | 0,889 | 0,907 | 0,918 | 0,858 | 0,978 | 0,941 | 0,869 | 1 |
| IFI27_ ISG15_ HERC6_ IL1R2_ OLAH | 0,898 | 0,890 | 0,906 | 0,924 | 0,874 | 0,975 | 0,942 | 0,888 | 0,997 |
| IFI27_ SIGLEC1_ IL1R2_ OLAH_ MMP8 | 0,898 | 0,890 | 0,906 | 0,922 | 0,871 | 0,973 | 0,951 | 0,904 | 0,999 |
| IFI27_ ISG15_ HERC6_ OLAH_ MMP8 | 0,898 | 0,890 | 0,906 | 0,919 | 0,864 | 0,975 | 0,937 | 0,879 | 0,995 |
| RSAD2_ IFI27_ IFIT1_ HERC6_ OLAH | 0,898 | 0,890 | 0,906 | 0,922 | 0,870 | 0,975 | 0,936 | 0,878 | 0,994 |
| IFI27_ OAS1_ ISG15_ HERC6_ RETN | 0,898 | 0,889 | 0,907 | 0,917 | 0,858 | 0,976 | 0,932 | 0,863 | 1,000 |
| IFI27_ SIGLEC1_ ISG15_ IL1R2_ OLAH | 0,898 | 0,890 | 0,905 | 0,921 | 0,869 | 0,973 | 0,945 | 0,892 | 0,998 |
| IFI27_ OAS1_ IFI44L_ HERC6_ OLAH | 0,898 | 0,889 | 0,906 | 0,926 | 0,875 | 0,977 | 0,937 | 0,879 | 0,995 |
| IFI27_ ISG15_ OLAH_ RETN_ MMP8 | 0,898 | 0,889 | 0,907 | 0,920 | 0,861 | 0,978 | 0,948 | 0,889 | 1 |
| RSAD2_ IFI27_ SLC1A2_ OLAH_ FAM20A | 0,898 | 0,889 | 0,906 | 0,923 | 0,867 | 0,980 | 0,921 | 0,839 | 1 |
| IFI27_ SIGLEC1_ HERC6_ IL1R2_ OLAH | 0,898 | 0,890 | 0,906 | 0,924 | 0,873 | 0,975 | 0,945 | 0,892 | 0,997 |
| RSAD2_ IFI27_ OAS1_ IL1R2_ FAM20A | 0,898 | 0,889 | 0,906 | 0,925 | 0,873 | 0,978 | 0,929 | 0,848 | 1 |
| IFI27_ ISG15_ IL1R2_ FAM20A_ RETN | 0,898 | 0,890 | 0,906 | 0,923 | 0,871 | 0,976 | 0,916 | 0,829 | 1 |
| IFI27_ IFI44L_ SLC1A2_ OLAH_ FAM20A | 0,898 | 0,889 | 0,907 | 0,924 | 0,867 | 0,982 | 0,926 | 0,850 | 1 |
| RSAD2_ IFI27_ OLAH_ FAM20A_ RETN | 0,898 | 0,889 | 0,906 | 0,918 | 0,858 | 0,979 | 0,937 | 0,869 | 1 |
| IFI27_ OAS1_ HERC6_ IL1R2_ RETN | 0,898 | 0,890 | 0,906 | 0,922 | 0,869 | 0,974 | 0,938 | 0,878 | 0,998 |
| IFI44L_ SIGLEC1_ IL1R2_ FAM20A_ MMP8 | 0,898 | 0,889 | 0,906 | 0,919 | 0,861 | 0,976 | 0,961 | 0,903 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_ HERC6_ IL1R2_ OLAH_ MMP8 | 0,898 | 0,890 | 0,906 | 0,924 | 0,873 | 0,974 | 0,943 | 0,890 | 0,997 |
| IFI27_ IFIT1_ ISG15_ IL1R2_ FAM20A | 0,898 | 0,889 | 0,906 | 0,924 | 0,871 | 0,978 | 0,928 | 0,848 | 1 |
| IFI27_ IFIT1_ HERC6_ IL1R2_ OLAH | 0,898 | 0,889 | 0,906 | 0,925 | 0,875 | 0,975 | 0,942 | 0,888 | 0,997 |
| IFI27_ IFI44L_ SIGLEC1_ HERC6_ IL1R2 | 0,898 | 0,890 | 0,905 | 0,922 | 0,875 | 0,970 | 0,937 | 0,883 | 0,991 |
| IFI27_ OAS1_ ISG15_ HERC6_ OLAH | 0,897 | 0,889 | 0,906 | 0,922 | 0,870 | 0,975 | 0,936 | 0,877 | 0,995 |
| IFI27_ SIGLEC1_ SLC1A2_ IL1R2_ FAM20A | 0,897 | 0,889 | 0,906 | 0,928 | 0,877 | 0,979 | 0,947 | 0,875 | 1 |
| IFI27_ IFI44L_ SIGLEC1_ ISG15_ OLAH | 0,897 | 0,890 | 0,905 | 0,919 | 0,863 | 0,974 | 0,941 | 0,884 | 0,999 |
| IFI27_ IFIT1_ IL1R2_ FAM20A_ RETN | 0,897 | 0,889 | 0,906 | 0,924 | 0,871 | 0,978 | 0,929 | 0,850 | 1 |
| IFI27_ IL1R2_ OLAH_ RETN_ MMP8 | 0,897 | 0,889 | 0,906 | 0,923 | 0,868 | 0,977 | 0,948 | 0,890 | 1 |
| IFI27_ ISG15_ HERC6_ OLAH_ RETN | 0,897 | 0,889 | 0,906 | 0,918 | 0,860 | 0,976 | 0,943 | 0,887 | 1,000 |
| IFI27_ IFI44L_ SIGLEC1_ OLAH_ MMP8 | 0,897 | 0,889 | 0,905 | 0,920 | 0,864 | 0,976 | 0,949 | 0,898 | 1,000 |
| IFI27_ IFI44L_ IL1R2_ OLAH_ MMP8 | 0,897 | 0,889 | 0,905 | 0,922 | 0,871 | 0,974 | 0,940 | 0,884 | 0,996 |
| IFI27_ IFIT1_ ISG15_ HERC6_ OLAH | 0,897 | 0,889 | 0,905 | 0,920 | 0,865 | 0,975 | 0,938 | 0,880 | 0,996 |
| IFI27_ OAS1_ ISG15_ IL1R2_ FAM20A | 0,897 | 0,889 | 0,905 | 0,925 | 0,873 | 0,978 | 0,928 | 0,848 | 1 |
| IFI27_ OAS1_ IL1R2_ FAM20A_ RETN | 0,897 | 0,889 | 0,905 | 0,927 | 0,874 | 0,979 | 0,928 | 0,848 | 1 |
| IFI27_ SIGLEC1_ ISG15_ HERC6_ OLAH | 0,897 | 0,889 | 0,906 | 0,919 | 0,862 | 0,975 | 0,941 | 0,886 | 0,996 |
| RSAD2_ IFI27_ IFI44L_ HERC6_ OLAH | 0,897 | 0,889 | 0,905 | 0,926 | 0,874 | 0,977 | 0,938 | 0,882 | 0,995 |
| IFI27_ IFI44L_ HERC6_ FAM20A_ RETN | 0,897 | 0,888 | 0,906 | 0,920 | 0,861 | 0,979 | 0,938 | 0,860 | 1 |
| IFI27_ IFIT1_ IFI44L_ SIGLEC1_ FAM20A | 0,897 | 0,888 | 0,906 | 0,914 | 0,848 | 0,979 | 0,946 | 0,875 | 1 |
| IFI27_ IFI44L_ SIGLEC1_ FAM20A_ RETN | 0,897 | 0,888 | 0,906 | 0,919 | 0,860 | 0,979 | 0,946 | 0,873 | 1 |
| IFI27_ IFIT1_ SIGLEC1_ ISG15_ FAM20A | 0,897 | 0,889 | 0,905 | 0,916 | 0,853 | 0,980 | 0,942 | 0,870 | 1 |
| RSAD2_ IFI27_ SIGLEC1_ FAM20A_ RETN | 0,897 | 0,888 | 0,906 | 0,918 | 0,858 | 0,979 | 0,949 | 0,882 | 1 |
| IFI27_ ISG15_ HERC6_ FAM20A_ RETN | 0,897 | 0,888 | 0,906 | 0,920 | 0,861 | 0,979 | 0,941 | 0,864 | 1 |
| IFI27_ OAS1_ SIGLEC1_ HERC6_ FAM20A | 0,897 | 0,888 | 0,906 | 0,915 | 0,854 | 0,976 | 0,945 | 0,874 | 1 |
| IFI27_ IL1R2_ FAM20A_ RETN_ MMP8 | 0,897 | 0,888 | 0,905 | 0,924 | 0,873 | 0,975 | 0,911 | 0,819 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2_ IFI27_ IL1R2_ FAM20A_ RETN | 0,897 | 0,889 | 0,905 | 0,924 | 0,872 | 0,977 | 0,929 | 0,850 | 1 |
| IFI27_ IFIT1_ SLC1A2_ OLAH_ FAM20A | 0,897 | 0,888 | 0,906 | 0,922 | 0,865 | 0,980 | 0,925 | 0,847 | 1 |
| IFI27_ SIGLEC1_ HERC6_ OLAH_ MMP8 | 0,897 | 0,888 | 0,905 | 0,918 | 0,860 | 0,975 | 0,941 | 0,886 | 0,997 |
| IFI27_ SLC1A2_ OLAH_ FAM20A_ RETN | 0,897 | 0,888 | 0,906 | 0,925 | 0,869 | 0,980 | 0,921 | 0,839 | 1 |
| SIGLEC1_ HERC6_ IL1R2_ FAM20A_ MMP8 | 0,897 | 0,888 | 0,905 | 0,917 | 0,858 | 0,976 | 0,961 | 0,900 | 1 |
| RSAD2_ IFI27_ SIGLEC1_ HERC6_ IL1R2 | 0,897 | 0,889 | 0,904 | 0,920 | 0,872 | 0,968 | 0,949 | 0,902 | 0,996 |
| IFI27_ IFIT1_ SIGLEC1_ IL1R2_ OLAH | 0,897 | 0,889 | 0,904 | 0,920 | 0,868 | 0,973 | 0,941 | 0,887 | 0,996 |
| IFI27_ IFIT1_ IFI44L_ HERC6_ OLAH | 0,897 | 0,888 | 0,905 | 0,925 | 0,872 | 0,978 | 0,938 | 0,881 | 0,995 |
| RSAD2_ IFI27_ ISG15_ HERC6_ OLAH | 0,897 | 0,888 | 0,905 | 0,924 | 0,872 | 0,975 | 0,936 | 0,877 | 0,995 |
| IFI27_ SIGLEC1_ ISG15_ OLAH_ MMP8 | 0,897 | 0,888 | 0,905 | 0,916 | 0,859 | 0,974 | 0,942 | 0,886 | 0,999 |
| RSAD2_ SIGLEC1_ IL1R2_ FAM20A_ MMP8 | 0,897 | 0,888 | 0,905 | 0,919 | 0,862 | 0,976 | 0,963 | 0,908 | 1 |
| IFI27_ IFI44L_ SIGLEC1_ SLC1A2_ OLAH | 0,896 | 0,888 | 0,905 | 0,927 | 0,876 | 0,978 | 0,946 | 0,890 | 1 |
| RSAD2_ IFI27_ ISG15_ IL1R2_ FAM20A | 0,896 | 0,888 | 0,905 | 0,924 | 0,871 | 0,977 | 0,926 | 0,843 | 1 |
| IFI27_ OAS1_ SLC1A2_ OLAH_ FAM20A | 0,896 | 0,888 | 0,905 | 0,924 | 0,868 | 0,981 | 0,922 | 0,842 | 1 |
| SIGLEC1_ IL1R2_ OLAH_ FAM20A_ MMP8 | 0,896 | 0,888 | 0,905 | 0,918 | 0,859 | 0,976 | 0,964 | 0,909 | 1 |
| IFI27_ OAS1_ IFIT1_ HERC6_ RETN | 0,896 | 0,887 | 0,905 | 0,915 | 0,853 | 0,976 | 0,937 | 0,867 | 1 |
| RSAD2_ IFI27_ OAS1_ HERC6_ RETN | 0,896 | 0,887 | 0,905 | 0,915 | 0,853 | 0,977 | 0,933 | 0,858 | 1 |
| IFI27_ OAS1_ IFI44L_ HERC6_ RETN | 0,896 | 0,887 | 0,905 | 0,915 | 0,854 | 0,976 | 0,936 | 0,867 | 1 |
| IFI27_ IFI44L_ SIGLEC1_ OLAH_ RETN | 0,896 | 0,888 | 0,904 | 0,916 | 0,857 | 0,975 | 0,949 | 0,890 | 1 |
| RSAD2_ IFI27_ SIGLEC1_ IL1R2_ OLAH | 0,896 | 0,888 | 0,904 | 0,922 | 0,870 | 0,974 | 0,942 | 0,888 | 0,996 |
| IFI27_ SIGLEC1_ SLC1A2_ OLAH_ MMP8 | 0,896 | 0,888 | 0,905 | 0,927 | 0,876 | 0,979 | 0,941 | 0,883 | 1 |
| IFI27_ OAS1_ SIGLEC1_ IL1R2_ OLAH | 0,896 | 0,888 | 0,904 | 0,920 | 0,868 | 0,973 | 0,943 | 0,890 | 0,997 |
| IFI27_ OAS1_ HERC6_ SLC1A2_ OLAH | 0,896 | 0,887 | 0,905 | 0,928 | 0,879 | 0,978 | 0,933 | 0,868 | 0,997 |
| IFI27_ IFI44L_ OLAH_ RETN_ MMP8 | 0,896 | 0,887 | 0,905 | 0,920 | 0,861 | 0,979 | 0,952 | 0,897 | 1 |
| IFI27_ IFIT1_ IL1R2_ OLAH_ MMP8 | 0,896 | 0,888 | 0,904 | 0,922 | 0,870 | 0,973 | 0,945 | 0,892 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27_ IFI44L_ SLC1A2_ IL1R2_ FAM20A | 0,896 | 0,888 | 0,904 | 0,927 | 0,878 | 0,976 | 0,927 | 0,843 | 1 |
| RSAD2_ IFI27_ SIGLEC1_ HERC6_ RETN | 0,896 | 0,887 | 0,905 | 0,914 | 0,855 | 0,973 | 0,949 | 0,897 | 1 |
| IFI27_ OAS1_ SIGLEC1_ SLC1A2_ OLAH | 0,896 | 0,888 | 0,904 | 0,927 | 0,875 | 0,979 | 0,945 | 0,888 | 1 |
| IFI27_ SLC1A2_ IL1R2_ OLAH_ MMP8 | 0,896 | 0,888 | 0,904 | 0,927 | 0,877 | 0,976 | 0,928 | 0,860 | 0,996 |
| IFI27_ OAS1_ IL1R2_ OLAH_ MMP8 | 0,896 | 0,888 | 0,904 | 0,921 | 0,869 | 0,973 | 0,943 | 0,890 | 0,997 |
| IFI27_ IFIT1_ SIGLEC1_ OLAH_ MMP8 | 0,896 | 0,888 | 0,904 | 0,918 | 0,862 | 0,974 | 0,941 | 0,885 | 0,997 |
| IFI27_ SIGLEC1_ OLAH_ RETN_ MMP8 | 0,896 | 0,887 | 0,905 | 0,918 | 0,857 | 0,978 | 0,963 | 0,914 | 1 |
| OAS1_ SIGLEC1_ IL1R2_ FAM20A_ MMP8 | 0,896 | 0,887 | 0,904 | 0,916 | 0,858 | 0,974 | 0,964 | 0,910 | 1 |
| RSAD2_ IFI27_ IL1R2_ OLAH_ MMP8 | 0,896 | 0,888 | 0,904 | 0,921 | 0,869 | 0,973 | 0,943 | 0,890 | 0,996 |
| IFI27_ IFIT1_ SIGLEC1_ SLC1A2_ OLAH | 0,896 | 0,887 | 0,904 | 0,927 | 0,876 | 0,979 | 0,942 | 0,884 | 1 |
| IFI27_ IFI44L_ HERC6_ IL1R2_ RETN | 0,896 | 0,888 | 0,904 | 0,919 | 0,866 | 0,973 | 0,934 | 0,872 | 0,995 |
| IFI27_ IFIT1_ ISG15_ SLC1A2_ OLAH | 0,896 | 0,887 | 0,904 | 0,926 | 0,874 | 0,978 | 0,928 | 0,861 | 0,996 |
| IFI27_ IFI44L_ SIGLEC1_ IL1R2_ RETN | 0,896 | 0,888 | 0,903 | 0,921 | 0,868 | 0,973 | 0,951 | 0,901 | 1 |
| IFI27_ OAS1_ IFIT1_ SIGLEC1_ OLAH | 0,896 | 0,888 | 0,904 | 0,919 | 0,864 | 0,974 | 0,941 | 0,885 | 0,997 |
| IFI27_ ISG15_ SLC1A2_ OLAH_ MMP8 | 0,896 | 0,887 | 0,904 | 0,926 | 0,874 | 0,977 | 0,924 | 0,852 | 0,996 |
| IFI27_ IFIT1_ OLAH_ RETN_ MMP8 | 0,896 | 0,887 | 0,905 | 0,918 | 0,857 | 0,978 | 0,952 | 0,896 | 1 |
| SIGLEC1_ ISG15_ IL1R2_ FAM20A_ MMP8 | 0,896 | 0,887 | 0,904 | 0,915 | 0,855 | 0,974 | 0,961 | 0,900 | 1 |
| IFI27_ SIGLEC1_ ISG15_ FAM20A_ RETN | 0,895 | 0,886 | 0,905 | 0,911 | 0,847 | 0,976 | 0,949 | 0,876 | 1 |
| IFI27_ IFI44L_ SIGLEC1_ IL1R2_ MMP8 | 0,895 | 0,888 | 0,903 | 0,919 | 0,870 | 0,967 | 0,945 | 0,895 | 0,994 |
| RSAD2_ IFI27_ SIGLEC1_ SLC1A2_ OLAH | 0,895 | 0,887 | 0,904 | 0,928 | 0,877 | 0,979 | 0,942 | 0,884 | 1 |
| IFI27_ ISG15_ IL1R2_ OLAH_ RETN | 0,895 | 0,887 | 0,904 | 0,919 | 0,864 | 0,975 | 0,940 | 0,878 | 1 |
| IFI27_ IFIT1_ SLC1A2_ OLAH_ MMP8 | 0,895 | 0,887 | 0,904 | 0,927 | 0,874 | 0,979 | 0,935 | 0,871 | 0,998 |
| IFI27_ HERC6_ IL1R2_ OLAH_ RETN | 0,895 | 0,887 | 0,904 | 0,918 | 0,860 | 0,975 | 0,947 | 0,892 | 1 |
| IFI27_ IFI44L_ SLC1A2_ OLAH_ MMP8 | 0,895 | 0,887 | 0,904 | 0,927 | 0,876 | 0,978 | 0,932 | 0,866 | 0,997 |
| IFI27_ SIGLEC1_ HERC6_ OLAH_ RETN | 0,895 | 0,887 | 0,904 | 0,918 | 0,858 | 0,977 | 0,948 | 0,893 | 1 |

91

| Modèle | AUC modèle | IC 95% | | AUC Train | IC 95% Train | | AUC Test | IC 95% Test | |
|---|---|---|---|---|---|---|---|---|---|
| RSAD2_ IFI27_ HERC6_ SLC1A2_ OLAH | 0,895 | 0,887 | 0,904 | 0,927 | 0,877 | 0,978 | 0,927 | 0,860 | 0,994 |
| IFI27_ IFIT1_ SIGLEC1_ OLAH_ RETN | 0,895 | 0,887 | 0,904 | 0,916 | 0,858 | 0,975 | 0,950 | 0,893 | 1 |
| IFI27_ IFI44L_ ISG15_ IL1R2_ OLAH | 0,895 | 0,887 | 0,903 | 0,921 | 0,869 | 0,974 | 0,939 | 0,881 | 0,997 |
| IFI27_ SIGLEC1_ HERC6_ FAM20A_ RETN | 0,895 | 0,886 | 0,905 | 0,917 | 0,855 | 0,979 | 0,950 | 0,874 | 1 |
| IFI27_ IFI44L_ ISG15_ OLAH_ MMP8 | 0,895 | 0,887 | 0,903 | 0,917 | 0,859 | 0,974 | 0,934 | 0,871 | 0,996 |
| IFI27_ ISG15_ SLC1A2_ IL1R2_ OLAH | 0,895 | 0,887 | 0,903 | 0,928 | 0,879 | 0,977 | 0,924 | 0,852 | 0,996 |
| IFI27_ ISG15_ HERC6_ SLC1A2_ OLAH | 0,895 | 0,887 | 0,904 | 0,928 | 0,878 | 0,978 | 0,924 | 0,855 | 0,993 |
| RSAD2_ IFI27_ HERC6_ IL1R2_ RETN | 0,895 | 0,887 | 0,903 | 0,920 | 0,867 | 0,973 | 0,939 | 0,882 | 0,996 |
| IFI27_ OAS1_ OLAH_ RETN_ MMP8 | 0,895 | 0,886 | 0,904 | 0,919 | 0,860 | 0,979 | 0,951 | 0,894 | 1 |
| IFI27_ IFI44L_ ISG15_ SLC1A2_ OLAH | 0,895 | 0,887 | 0,903 | 0,926 | 0,875 | 0,978 | 0,925 | 0,855 | 0,995 |
| IFI27_ IFIT1_ SIGLEC1_ HERC6_ RETN | 0,895 | 0,886 | 0,904 | 0,912 | 0,850 | 0,973 | 0,947 | 0,894 | 0,999 |
| IFI27_ IFIT1_ SIGLEC1_ HERC6_ IL1R2 | 0,895 | 0,887 | 0,902 | 0,922 | 0,875 | 0,969 | 0,948 | 0,900 | 0,996 |
| IFI27_ OAS1_ IFIT1_ IL1R2_ OLAH | 0,895 | 0,887 | 0,903 | 0,920 | 0,868 | 0,973 | 0,941 | 0,885 | 0,997 |
| IFI27_ SIGLEC1_ IL1R2_ RETN_ MMP8 | 0,895 | 0,887 | 0,903 | 0,920 | 0,865 | 0,974 | 0,964 | 0,925 | 1 |
| IFI27_ IFI44L_ SLC1A2_ IL1R2_ OLAH | 0,895 | 0,886 | 0,903 | 0,927 | 0,877 | 0,977 | 0,927 | 0,858 | 0,996 |
| IFI27_ IFIT1_ SIGLEC1_ FAM20A_ MMP8 | 0,895 | 0,886 | 0,904 | 0,914 | 0,849 | 0,978 | 0,947 | 0,878 | 1 |
| IFI27_ OAS1_ SIGLEC1_ OLAH_ MMP8 | 0,895 | 0,886 | 0,903 | 0,918 | 0,862 | 0,974 | 0,945 | 0,890 | 0,999 |
| RSAD2_ IFI27_ SIGLEC1_ OLAH_ MMP8 | 0,895 | 0,886 | 0,903 | 0,919 | 0,863 | 0,975 | 0,942 | 0,887 | 0,998 |
| IFI27_ IFIT1_ SIGLEC1_ ISG15_ OLAH | 0,895 | 0,887 | 0,903 | 0,918 | 0,862 | 0,974 | 0,941 | 0,885 | 0,998 |
| RSAD2_ IFI27_ SLC1A2_ OLAH_ MMP8 | 0,895 | 0,886 | 0,903 | 0,926 | 0,875 | 0,978 | 0,932 | 0,866 | 0,997 |
| IFI27_ SIGLEC1_ ISG15_ OLAH_ RETN | 0,895 | 0,886 | 0,903 | 0,917 | 0,859 | 0,975 | 0,948 | 0,891 | 1 |
| IFI27_ OAS1_ SIGLEC1_ IL1R2_ RETN | 0,895 | 0,887 | 0,903 | 0,920 | 0,867 | 0,973 | 0,953 | 0,901 | 1 |
| IFIT1_ SIGLEC1_ HERC6_ OLAH_ FAM20A | 0,895 | 0,886 | 0,904 | 0,913 | 0,852 | 0,973 | 0,949 | 0,893 | 1 |
| IFI27_ SIGLEC1_ HERC6_ SLC1A2_ OLAH | 0,895 | 0,886 | 0,904 | 0,927 | 0,876 | 0,979 | 0,940 | 0,881 | 0,999 |
| IFI27_ SIGLEC1_ SLC1A2_ IL1R2_ OLAH | 0,895 | 0,886 | 0,903 | 0,927 | 0,877 | 0,977 | 0,942 | 0,886 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27_ OAS1_ SLC1A2_ OLAH_ MMP8 | 0,895 | 0,886 | 0,903 | 0,926 | 0,875 | 0,978 | 0,929 | 0,862 | 0,997 |
| IFI27_ OAS1_ IFIT1_ SIGLEC1_ FAM20A | 0,894 | 0,886 | 0,903 | 0,913 | 0,848 | 0,978 | 0,948 | 0,880 | 1 |
| IFI27_ IFIT1_ IFI44L_ SIGLEC1_ OLAH | 0,894 | 0,886 | 0,903 | 0,919 | 0,863 | 0,976 | 0,949 | 0,898 | 1,000 |
| IFI27_ HERC6_ SLC1A2_ IL1R2_ FAM20A | 0,894 | 0,886 | 0,903 | 0,930 | 0,883 | 0,977 | 0,923 | 0,835 | 1 |
| IFI27_ SIGLEC1_ FAM20A_ RETN_ MMP8 | 0,894 | 0,885 | 0,904 | 0,916 | 0,852 | 0,979 | 0,954 | 0,882 | 1 |
| IFI27_ OAS1_ IFIT1_ HERC6_ IL1R2 | 0,894 | 0,887 | 0,902 | 0,917 | 0,868 | 0,966 | 0,939 | 0,884 | 0,994 |
| IFI27_ IFIT1_ ISG15_ OLAH_ MMP8 | 0,894 | 0,886 | 0,903 | 0,914 | 0,855 | 0,973 | 0,935 | 0,873 | 0,996 |
| IFI27_ IFIT1_ SIGLEC1_ IL1R2_ RETN | 0,894 | 0,886 | 0,902 | 0,920 | 0,866 | 0,973 | 0,959 | 0,915 | 1 |
| RSAD2_ IFI27_ OLAH_ RETN_ MMP8 | 0,894 | 0,885 | 0,903 | 0,918 | 0,858 | 0,978 | 0,951 | 0,894 | 1 |
| IFI27_ IFI44L_ SIGLEC1_ ISG15_ IL1R2 | 0,894 | 0,887 | 0,901 | 0,918 | 0,869 | 0,968 | 0,942 | 0,891 | 0,994 |
| IFI27_ SLC1A2_ OLAH_ RETN_ MMP8 | 0,894 | 0,885 | 0,903 | 0,926 | 0,873 | 0,978 | 0,941 | 0,878 | 1 |
| RSAD2_ IFI27_ IFIT1_ IL1R2_ OLAH | 0,894 | 0,886 | 0,902 | 0,921 | 0,869 | 0,974 | 0,941 | 0,885 | 0,997 |
| IFI27_ OAS1_ IFIT1_ OLAH_ MMP8 | 0,894 | 0,886 | 0,903 | 0,916 | 0,858 | 0,974 | 0,938 | 0,877 | 0,999 |
| IFI27_ HERC6_ SLC1A2_ OLAH_ MMP8 | 0,894 | 0,885 | 0,903 | 0,929 | 0,878 | 0,979 | 0,936 | 0,874 | 0,998 |
| IFI27_ IFIT1_ ISG15_ IL1R2_ OLAH | 0,894 | 0,886 | 0,902 | 0,919 | 0,865 | 0,972 | 0,941 | 0,885 | 0,998 |
| IFI27_ OAS1_ SIGLEC1_ OLAH_ RETN | 0,894 | 0,886 | 0,902 | 0,915 | 0,857 | 0,974 | 0,949 | 0,889 | 1 |
| RSAD2_ IFI27_ OAS1_ IL1R2_ OLAH | 0,894 | 0,886 | 0,902 | 0,921 | 0,868 | 0,974 | 0,941 | 0,886 | 0,997 |
| RSAD2_ IFI27_ ISG15_ SLC1A2_ OLAH | 0,894 | 0,886 | 0,902 | 0,927 | 0,875 | 0,979 | 0,928 | 0,860 | 0,996 |
| IFI27_ SIGLEC1_ IL1R2_ OLAH_ RETN | 0,894 | 0,886 | 0,902 | 0,917 | 0,860 | 0,975 | 0,950 | 0,896 | 1 |
| IFI27_ OAS1_ IFI44L_ SIGLEC1_ OLAH | 0,894 | 0,886 | 0,902 | 0,919 | 0,863 | 0,976 | 0,948 | 0,896 | 0,999 |
| RSAD2_ IFI27_ SLC1A2_ IL1R2_ OLAH | 0,894 | 0,885 | 0,902 | 0,926 | 0,876 | 0,976 | 0,933 | 0,867 | 0,998 |
| IFI27_ IFIT1_ HERC6_ SLC1A2_ OLAH | 0,894 | 0,885 | 0,903 | 0,929 | 0,879 | 0,980 | 0,936 | 0,874 | 0,997 |
| RSAD2_ IFI27_ IFIT1_ SIGLEC1_ FAM20A | 0,894 | 0,885 | 0,903 | 0,915 | 0,850 | 0,979 | 0,946 | 0,877 | 1 |
| IFI27_ OAS1_ SIGLEC1_ ISG15_ OLAH | 0,894 | 0,886 | 0,902 | 0,917 | 0,861 | 0,974 | 0,942 | 0,886 | 0,999 |
| IFI27_ OAS1_ IFIT1_ SLC1A2_ OLAH | 0,894 | 0,885 | 0,902 | 0,927 | 0,875 | 0,978 | 0,939 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_ OAS1_ SIGLEC1_ IL1R2_ MMP8 | 0,894 | 0,886 | 0,901 | 0,919 | 0,871 | 0,967 | 0,950 | 0,903 | 0,997 |
| IFI27_ HERC6_ SLC1A2_ IL1R2_ OLAH | 0,894 | 0,885 | 0,902 | 0,926 | 0,876 | 0,976 | 0,934 | 0,870 | 0,997 |
| IFI27_ OAS1_ IFIT1_ OLAH_ RETN | 0,894 | 0,885 | 0,902 | 0,915 | 0,855 | 0,975 | 0,943 | 0,881 | 1 |
| IFI27_ IFIT1_ SLC1A2_ IL1R2_ OLAH | 0,894 | 0,885 | 0,902 | 0,926 | 0,876 | 0,976 | 0,932 | 0,866 | 0,997 |
| SIGLEC1_ IL1R2_ FAM20A_ RETN_ MMP8 | 0,894 | 0,885 | 0,903 | 0,917 | 0,858 | 0,976 | 0,961 | 0,901 | 1 |
| IFI27_ IFI44L_ IL1R2_ OLAH_ RETN | 0,894 | 0,885 | 0,902 | 0,918 | 0,862 | 0,974 | 0,942 | 0,882 | 1 |
| RSAD2_ IFI27_ IFI44L_ SIGLEC1_ OLAH | 0,894 | 0,886 | 0,902 | 0,918 | 0,861 | 0,975 | 0,948 | 0,896 | 0,999 |
| IFI27_ IFIT1_ HERC6_ IL1R2_ RETN | 0,893 | 0,886 | 0,901 | 0,919 | 0,866 | 0,973 | 0,938 | 0,880 | 0,996 |
| IFI27_ IFIT1_ IFI44L_ SIGLEC1_ IL1R2 | 0,893 | 0,886 | 0,901 | 0,921 | 0,872 | 0,970 | 0,947 | 0,898 | 0,995 |
| RSAD2_ IFI27_ OAS1_ SIGLEC1_ OLAH | 0,893 | 0,885 | 0,902 | 0,919 | 0,863 | 0,974 | 0,941 | 0,885 | 0,998 |
| RSAD2_ IFI27_ SIGLEC1_ ISG15_ OLAH | 0,893 | 0,885 | 0,902 | 0,918 | 0,862 | 0,974 | 0,941 | 0,884 | 0,999 |
| IFIT1_ SIGLEC1_ HERC6_ FAM20A_ RETN | 0,893 | 0,885 | 0,902 | 0,914 | 0,853 | 0,975 | 0,940 | 0,873 | 1 |
| RSAD2_ IFI27_ IFIT1_ SIGLEC1_ OLAH | 0,893 | 0,885 | 0,902 | 0,919 | 0,863 | 0,975 | 0,940 | 0,884 | 0,997 |
| IFI27_ SIGLEC1_ HERC6_ IL1R2_ RETN | 0,893 | 0,885 | 0,902 | 0,922 | 0,869 | 0,975 | 0,951 | 0,903 | 0,999 |
| IFI27_ HERC6_ IL1R2_ RETN_ MMP8 | 0,893 | 0,885 | 0,902 | 0,922 | 0,868 | 0,975 | 0,939 | 0,881 | 0,998 |
| IFI27_ OAS1_ HERC6_ IL1R2_ MMP8 | 0,893 | 0,886 | 0,901 | 0,916 | 0,868 | 0,965 | 0,932 | 0,873 | 0,990 |
| RSAD2_ IFI27_ SIGLEC1_ IL1R2_ RETN | 0,893 | 0,885 | 0,902 | 0,921 | 0,869 | 0,974 | 0,957 | 0,911 | 1 |
| RSAD2_ IFI27_ SIGLEC1_ OLAH_ RETN | 0,893 | 0,885 | 0,902 | 0,916 | 0,857 | 0,975 | 0,947 | 0,889 | 1 |
| IFI27_ HERC6_ FAM20A_ RETN_ MMP8 | 0,893 | 0,883 | 0,903 | 0,920 | 0,861 | 0,980 | 0,940 | 0,862 | 1 |
| IFI27_ IFIT1_ IL1R2_ OLAH_ RETN | 0,893 | 0,885 | 0,902 | 0,918 | 0,861 | 0,974 | 0,943 | 0,885 | 1 |
| RSAD2_ IFI27_ SIGLEC1_ IL1R2_ MMP8 | 0,893 | 0,886 | 0,901 | 0,920 | 0,872 | 0,968 | 0,951 | 0,905 | 0,997 |
| RSAD2_ IFI27_ OAS1_ OLAH_ MMP8 | 0,893 | 0,885 | 0,902 | 0,916 | 0,859 | 0,974 | 0,937 | 0,877 | 0,997 |
| IFI27_ IFI44L_ ISG15_ OLAH_ RETN | 0,893 | 0,885 | 0,902 | 0,916 | 0,858 | 0,974 | 0,939 | 0,874 | 1 |
| RSAD2_ IFI27_ IL1R2_ OLAH_ RETN | 0,893 | 0,885 | 0,902 | 0,919 | 0,863 | 0,974 | 0,943 | 0,885 | 1 |
| IFI27_ OAS1_ IL1R2_ OLAH_ RETN | 0,893 | 0,885 | 0,901 | 0,918 | 0,862 | 0,974 | 0,943 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2_IFI27_IFIT1_SLC1A2_OLAH | 0,893 | 0,885 | 0,902 | 0,926 | 0,873 | 0,978 | 0,935 | 0,871 | 0,999 |
| IFI27_ISG15_SLC1A2_OLAH_RETN | 0,893 | 0,885 | 0,902 | 0,926 | 0,874 | 0,977 | 0,929 | 0,859 | 1,000 |
| IFI27_SIGLEC1_ISG15_IL1R2_MMP8 | 0,893 | 0,886 | 0,900 | 0,920 | 0,871 | 0,968 | 0,950 | 0,903 | 0,997 |
| RSAD2_IFI27_OAS1_HERC6_IL1R2 | 0,893 | 0,885 | 0,901 | 0,918 | 0,870 | 0,966 | 0,938 | 0,882 | 0,994 |
| IFI27_OAS1_ISG15_IL1R2_OLAH | 0,893 | 0,885 | 0,901 | 0,924 | 0,872 | 0,975 | 0,938 | 0,881 | 0,995 |
| IFI27_SLC1A2_IL1R2_OLAH_RETN | 0,893 | 0,885 | 0,902 | 0,926 | 0,874 | 0,977 | 0,929 | 0,858 | 1 |
| IFI27_OAS1_ISG15_OLAH_MMP8 | 0,893 | 0,885 | 0,901 | 0,916 | 0,858 | 0,974 | 0,936 | 0,877 | 0,995 |
| IFI27_SIGLEC1_HERC6_IL1R2_MMP8 | 0,893 | 0,886 | 0,901 | 0,920 | 0,872 | 0,969 | 0,949 | 0,902 | 0,996 |
| RSAD2_IFI27_OAS1_SLC1A2_OLAH | 0,893 | 0,884 | 0,902 | 0,926 | 0,873 | 0,978 | 0,935 | 0,869 | 1 |
| IFI27_IFIT1_IFI44L_OLAH_MMP8 | 0,893 | 0,884 | 0,902 | 0,917 | 0,859 | 0,975 | 0,940 | 0,882 | 0,998 |
| SIGLEC1_SLC1A2_IL1R2_FAM20A_MMP8 | 0,893 | 0,884 | 0,902 | 0,922 | 0,872 | 0,972 | 0,957 | 0,887 | 1 |
| IFI27_IFI44L_HERC6_IL1R2_MMP8 | 0,893 | 0,885 | 0,900 | 0,916 | 0,866 | 0,965 | 0,930 | 0,871 | 0,989 |
| IFI27_OAS1_IFIT1_SIGLEC1_IL1R2 | 0,893 | 0,885 | 0,901 | 0,920 | 0,871 | 0,969 | 0,951 | 0,904 | 0,998 |
| IFI27_OAS1_IFIT1_ISG15_OLAH | 0,893 | 0,885 | 0,901 | 0,914 | 0,854 | 0,973 | 0,935 | 0,873 | 0,996 |
| RSAD2_IFI27_ISG15_IL1R2_OLAH | 0,893 | 0,885 | 0,901 | 0,920 | 0,868 | 0,973 | 0,942 | 0,887 | 0,997 |
| RSAD2_IFI27_OAS1_SIGLEC1_FAM20A | 0,893 | 0,884 | 0,902 | 0,915 | 0,851 | 0,979 | 0,949 | 0,879 | 1 |
| IFI27_IFIT1_IFI44L_IL1R2_OLAH | 0,893 | 0,885 | 0,901 | 0,921 | 0,868 | 0,974 | 0,940 | 0,885 | 0,995 |
| IFI27_OAS1_SLC1A2_IL1R2_OLAH | 0,893 | 0,885 | 0,901 | 0,926 | 0,876 | 0,976 | 0,929 | 0,862 | 0,997 |
| IFI27_IFI44L_IL1R2_RETN_MMP8 | 0,893 | 0,885 | 0,901 | 0,919 | 0,866 | 0,972 | 0,940 | 0,879 | 1 |
| IFI27_SIGLEC1_SLC1A2_OLAH_RETN | 0,893 | 0,884 | 0,902 | 0,926 | 0,874 | 0,979 | 0,947 | 0,888 | 1 |
| IFI27_SIGLEC1_ISG15_IL1R2_RETN | 0,893 | 0,885 | 0,901 | 0,922 | 0,869 | 0,974 | 0,953 | 0,906 | 1 |
| RSAD2_IFI27_OAS1_SIGLEC1_IL1R2 | 0,893 | 0,885 | 0,901 | 0,920 | 0,871 | 0,968 | 0,958 | 0,914 | 1 |
| IFI27_ISG15_HERC6_IL1R2_RETN | 0,893 | 0,885 | 0,901 | 0,919 | 0,866 | 0,973 | 0,942 | 0,887 | 0,997 |
| IFI27_IFI44L_SIGLEC1_HERC6_RETN | 0,893 | 0,884 | 0,902 | 0,912 | 0,851 | 0,974 | 0,946 | 0,891 | 1 |
| IFI27_OAS1_ISG15_SLC1A2_OLAH | 0,893 | 0,884 | 0,901 | 0,927 | 0,875 | 0,978 | 0,921 | 0,849 | 0,993 |

| Modèle | AUC modèle | IC 95% | | AUC Train | IC 95% Train | | AUC Test | IC 95% Test | |
|---|---|---|---|---|---|---|---|---|---|
| IFI27_ IFIT1_ ISG15_ OLAH_ RETN | 0,893 | 0,884 | 0,901 | 0,916 | 0,857 | 0,975 | 0,940 | 0,878 | 1 |
| RSAD2_ IFI27_ SLC1A2_ IL1R2_ FAM20A | 0,893 | 0,884 | 0,901 | 0,926 | 0,878 | 0,975 | 0,926 | 0,840 | 1 |
| RSAD2_ IFI27_ IFIT1_ OLAH_ MMP8 | 0,893 | 0,884 | 0,901 | 0,916 | 0,858 | 0,974 | 0,938 | 0,877 | 0,999 |
| IFI27_ IFIT1_ SIGLEC1_ IL1R2_ MMP8 | 0,893 | 0,885 | 0,900 | 0,919 | 0,870 | 0,968 | 0,951 | 0,905 | 0,997 |
| RSAD2_ IFI27_ IFIT1_ HERC6_ IL1R2 | 0,893 | 0,885 | 0,900 | 0,918 | 0,869 | 0,967 | 0,936 | 0,880 | 0,992 |
| IFI27_ SIGLEC1_ ISG15_ HERC6_ IL1R2 | 0,893 | 0,885 | 0,900 | 0,919 | 0,870 | 0,968 | 0,949 | 0,902 | 0,996 |
| RSAD2_ IFI27_ SIGLEC1_ ISG15_ FAM20A | 0,893 | 0,884 | 0,902 | 0,914 | 0,851 | 0,978 | 0,947 | 0,877 | 1 |
| IFI27_ SLC1A2_ IL1R2_ FAM20A_ MMP8 | 0,893 | 0,884 | 0,901 | 0,925 | 0,878 | 0,972 | 0,905 | 0,810 | 1 |
| IFI27_ OAS1_ IFI44L_ SIGLEC1_ IL1R2 | 0,892 | 0,885 | 0,900 | 0,919 | 0,869 | 0,968 | 0,942 | 0,891 | 0,994 |
| IFI27_ IFI44L_ SLC1A2_ OLAH_ RETN | 0,892 | 0,884 | 0,901 | 0,927 | 0,876 | 0,978 | 0,935 | 0,868 | 1 |
| IFI27_ OAS1_ IFI44L_ IL1R2_ OLAH | 0,892 | 0,884 | 0,901 | 0,922 | 0,869 | 0,974 | 0,939 | 0,883 | 0,995 |
| IFI27_ HERC6_ SLC1A2_ OLAH_ RETN | 0,892 | 0,883 | 0,901 | 0,925 | 0,872 | 0,978 | 0,940 | 0,878 | 1 |
| RSAD2_ IFI27_ IFI44L_ IL1R2_ OLAH | 0,892 | 0,884 | 0,900 | 0,921 | 0,867 | 0,974 | 0,941 | 0,886 | 0,997 |
| IFI27_ IFIT1_ SLC1A2_ IL1R2_ FAM20A | 0,892 | 0,884 | 0,901 | 0,924 | 0,873 | 0,975 | 0,927 | 0,842 | 1 |
| IFI27_ OAS1_ IFIT1_ IL1R2_ RETN | 0,892 | 0,884 | 0,900 | 0,918 | 0,864 | 0,971 | 0,949 | 0,896 | 1 |
| IFI27_ OAS1_ IFIT1_ IFI44L_ OLAH | 0,892 | 0,884 | 0,901 | 0,916 | 0,858 | 0,974 | 0,939 | 0,881 | 0,998 |
| IFI27_ IFI44L_ HERC6_ SLC1A2_ OLAH | 0,892 | 0,883 | 0,902 | 0,928 | 0,877 | 0,978 | 0,933 | 0,870 | 0,995 |
| RSAD2_ IFI27_ OAS1_ IFIT1_ OLAH | 0,892 | 0,884 | 0,900 | 0,916 | 0,858 | 0,974 | 0,938 | 0,879 | 0,997 |
| IFI27_ IFIT1_ SIGLEC1_ ISG15_ IL1R2 | 0,892 | 0,885 | 0,900 | 0,920 | 0,871 | 0,968 | 0,947 | 0,897 | 0,996 |
| IFI27_ IFIT1_ IFI44L_ OLAH_ RETN | 0,892 | 0,884 | 0,901 | 0,914 | 0,855 | 0,974 | 0,945 | 0,884 | 1 |
| IFI27_ ISG15_ IL1R2_ RETN_ MMP8 | 0,892 | 0,884 | 0,900 | 0,924 | 0,873 | 0,976 | 0,933 | 0,866 | 0,999 |
| IFI27_ IFIT1_ IFI44L_ ISG15_ OLAH | 0,892 | 0,884 | 0,900 | 0,915 | 0,857 | 0,974 | 0,936 | 0,876 | 0,996 |
| RSAD2_ IFI27_ IFI44L_ SIGLEC1_ IL1R2 | 0,892 | 0,884 | 0,900 | 0,920 | 0,870 | 0,970 | 0,946 | 0,897 | 0,995 |
| IFI27_ OAS1_ IFI44L_ OLAH_ MMP8 | 0,892 | 0,883 | 0,900 | 0,917 | 0,860 | 0,974 | 0,937 | 0,878 | 0,996 |
| IFI27_ OAS1_ IFI44L_ HERC6_ IL1R2 | 0,892 | 0,884 | 0,899 | 0,919 | 0,871 | 0,966 | 0,929 | 0,870 | 0,988 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| RSAD2_ IFI27_ HERC6_ IL1R2_ MMP8 | 0,892 | 0,884 | 0,900 | 0,915 | 0,866 | 0,965 | 0,934 | 0,876 | 0,991 |
| RSAD2_ IFI27_ OAS1_ ISG15_ OLAH | 0,892 | 0,884 | 0,900 | 0,916 | 0,858 | 0,974 | 0,935 | 0,874 | 0,995 |
| RSAD2_ IFI27_ ISG15_ OLAH_ MMP8 | 0,892 | 0,883 | 0,900 | 0,915 | 0,856 | 0,974 | 0,935 | 0,874 | 0,996 |
| RSAD2_ IFI27_ OAS1_ OLAH_ RETN | 0,892 | 0,883 | 0,900 | 0,914 | 0,853 | 0,974 | 0,943 | 0,882 | 1 |
| IFI27_ IFIT1_ SLC1A2_ OLAH_ RETN | 0,892 | 0,883 | 0,900 | 0,925 | 0,874 | 0,977 | 0,938 | 0,873 | 1 |
| IFI27_ IFIT1_ IL1R2_ RETN_ MMP8 | 0,892 | 0,883 | 0,900 | 0,918 | 0,863 | 0,973 | 0,949 | 0,897 | 1 |
| IFI27_ OAS1_ SIGLEC1_ ISG15_ FAM20A | 0,892 | 0,882 | 0,901 | 0,911 | 0,846 | 0,976 | 0,946 | 0,875 | 1 |
| OAS1_ IFIT1_ SIGLEC1_ IL1R2_ FAM20A | 0,892 | 0,883 | 0,900 | 0,914 | 0,851 | 0,976 | 0,962 | 0,913 | 1 |
| IFI27_ IFI44L_ SIGLEC1_ ISG15_ FAM20A | 0,892 | 0,883 | 0,901 | 0,910 | 0,844 | 0,976 | 0,943 | 0,868 | 1 |
| IFI27_ IFIT1_ IFI44L_ HERC6_ IL1R2 | 0,892 | 0,884 | 0,899 | 0,918 | 0,868 | 0,969 | 0,938 | 0,883 | 0,993 |
| IFI27_ OAS1_ ISG15_ HERC6_ IL1R2 | 0,892 | 0,884 | 0,899 | 0,920 | 0,873 | 0,967 | 0,932 | 0,873 | 0,990 |
| RSAD2_ IFI27_ SLC1A2_ OLAH_ RETN | 0,892 | 0,883 | 0,900 | 0,925 | 0,873 | 0,977 | 0,934 | 0,866 | 1 |
| RSAD2_ IFI27_ IFI44L_ SIGLEC1_ FAM20A | 0,892 | 0,883 | 0,901 | 0,914 | 0,849 | 0,978 | 0,948 | 0,878 | 1 |
| IFIT1_ SIGLEC1_ ISG15_ HERC6_ FAM20A | 0,892 | 0,883 | 0,900 | 0,911 | 0,849 | 0,972 | 0,938 | 0,865 | 1 |
| IFI44L_ SIGLEC1_ HERC6_ IL1R2_ FAM20A | 0,892 | 0,883 | 0,900 | 0,913 | 0,852 | 0,974 | 0,957 | 0,896 | 1 |
| IFI27_ OAS1_ ISG15_ OLAH_ RETN | 0,892 | 0,883 | 0,900 | 0,916 | 0,858 | 0,975 | 0,938 | 0,875 | 1 |
| IFI27_ OAS1_ SLC1A2_ OLAH_ RETN | 0,892 | 0,883 | 0,900 | 0,925 | 0,874 | 0,977 | 0,935 | 0,868 | 1 |
| RSAD2_ IFI27_ IFIT1_ ISG15_ OLAH | 0,891 | 0,883 | 0,900 | 0,914 | 0,856 | 0,973 | 0,935 | 0,873 | 0,996 |
| IFI27_ IFIT1_ IFI44L_ SLC1A2_ OLAH | 0,891 | 0,883 | 0,900 | 0,925 | 0,873 | 0,978 | 0,936 | 0,873 | 0,999 |
| RSAD2_ IFI27_ IFI44L_ HERC6_ IL1R2 | 0,891 | 0,884 | 0,899 | 0,918 | 0,870 | 0,967 | 0,932 | 0,874 | 0,989 |
| IFI27_ OAS1_ IFI44L_ ISG15_ OLAH | 0,891 | 0,883 | 0,900 | 0,916 | 0,859 | 0,974 | 0,934 | 0,874 | 0,994 |
| RSAD2_ IFI27_ IFIT1_ SIGLEC1_ IL1R2 | 0,891 | 0,884 | 0,899 | 0,919 | 0,870 | 0,968 | 0,949 | 0,901 | 0,997 |
| IFI27_ IFIT1_ HERC6_ IL1R2_ MMP8 | 0,891 | 0,884 | 0,899 | 0,915 | 0,864 | 0,965 | 0,939 | 0,886 | 0,993 |
| RSAD2_ IFI27_ IFI44L_ OLAH_ MMP8 | 0,891 | 0,883 | 0,900 | 0,918 | 0,861 | 0,975 | 0,939 | 0,881 | 0,997 |
| RSAD2_ IFI27_ IFIT1_ OLAH_ RETN | 0,891 | 0,883 | 0,900 | 0,914 | 0,855 | 0,974 | 0,945 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_ OAS1_ IFI44L_ OLAH_ RETN | 0,891 | 0,883 | 0,900 | 0,915 | 0,856 | 0,974 | 0,942 | 0,882 | 1 |
| RSAD2_ IFI27_ IFI44L_ SLC1A2_ OLAH | 0,891 | 0,883 | 0,900 | 0,927 | 0,875 | 0,978 | 0,932 | 0,866 | 0,997 |
| IFI27_ SLC1A2_ IL1R2_ FAM20A_ RETN | 0,891 | 0,883 | 0,900 | 0,927 | 0,880 | 0,974 | 0,907 | 0,810 | 1 |
| IFI27_ IFI44L_ ISG15_ HERC6_ IL1R2 | 0,891 | 0,884 | 0,899 | 0,920 | 0,873 | 0,968 | 0,928 | 0,869 | 0,988 |
| IFI27_ OAS1_ SLC1A2_ IL1R2_ FAM20A | 0,891 | 0,883 | 0,900 | 0,928 | 0,880 | 0,977 | 0,925 | 0,839 | 1 |
| RSAD2_ SIGLEC1_ HERC6_ IL1R2 _ FAM20A | 0,891 | 0,882 | 0,900 | 0,916 | 0,857 | 0,974 | 0,955 | 0,898 | 1 |
| IFI27_ ISG15_ SLC1A2_ IL1R2_ FAM20A | 0,891 | 0,883 | 0,899 | 0,923 | 0,873 | 0,973 | 0,910 | 0,816 | 1 |
| IFIT1_ IFI44L_ SIGLEC1_ HERC6_ FAM20A | 0,891 | 0,882 | 0,900 | 0,912 | 0,850 | 0,973 | 0,937 | 0,867 | 1 |
| IFI27_ IFIT1_ IFI44L_ IL1R2_ RETN | 0,891 | 0,883 | 0,899 | 0,920 | 0,866 | 0,973 | 0,941 | 0,883 | 0,999 |
| IFI27_ OAS1_ HERC6_ RETN_ MMP8 | 0,891 | 0,882 | 0,900 | 0,916 | 0,857 | 0,976 | 0,929 | 0,857 | 1 |
| RSAD2_ IFI27_ SIGLEC1_ ISG15_ IL1R2 | 0,891 | 0,883 | 0,899 | 0,920 | 0,872 | 0,969 | 0,948 | 0,899 | 0,997 |
| RSAD2_ IFI27_ OAS1_ IFI44L_ OLAH | 0,891 | 0,883 | 0,899 | 0,918 | 0,861 | 0,975 | 0,939 | 0,881 | 0,997 |
| IFI27_ IFI44L_ SIGLEC1_ SLC1A2_ IL1R2 | 0,891 | 0,883 | 0,899 | 0,924 | 0,878 | 0,970 | 0,947 | 0,897 | 0,996 |
| IFI27_ OAS1_ SIGLEC1_ ISG15_ IL1R2 | 0,891 | 0,883 | 0,898 | 0,919 | 0,870 | 0,968 | 0,950 | 0,903 | 0,997 |
| RSAD2_ IFI27_ IFIT1_ IL1R2_ RETN | 0,891 | 0,883 | 0,899 | 0,917 | 0,864 | 0,971 | 0,946 | 0,892 | 0,999 |
| RSAD2_ IFI27_ ISG15_ OLAH_ RETN | 0,891 | 0,882 | 0,899 | 0,916 | 0,857 | 0,975 | 0,939 | 0,876 | 1 |
| IFI27_ OAS1_ SIGLEC1_ SLC1A2_ IL1R2 | 0,891 | 0,883 | 0,899 | 0,924 | 0,878 | 0,970 | 0,950 | 0,901 | 0,999 |
| IFI27_ OAS1_ IFI44L_ SLC1A2_ OLAH | 0,891 | 0,882 | 0,899 | 0,926 | 0,875 | 0,978 | 0,935 | 0,871 | 0,999 |
| RSAD2_ IFI27_ SIGLEC1_ FAM20A_ MMP8 | 0,890 | 0,881 | 0,900 | 0,913 | 0,849 | 0,978 | 0,948 | 0,878 | 1 |
| RSAD2_ IFI27_ IFI44L_ ISG15_ OLAH | 0,890 | 0,882 | 0,899 | 0,916 | 0,858 | 0,975 | 0,937 | 0,878 | 0,996 |
| RSAD2_ IFI27_ IFIT1_ IFI44L_ OLAH | 0,890 | 0,882 | 0,899 | 0,916 | 0,858 | 0,974 | 0,939 | 0,881 | 0,998 |
| IFI27_ SIGLEC1_ SLC1A2_ IL1R2_ RETN | 0,890 | 0,882 | 0,898 | 0,921 | 0,871 | 0,971 | 0,957 | 0,910 | 1 |
| RSAD2_ IFI27_ ISG15_ HERC6_ IL1R2 | 0,890 | 0,883 | 0,898 | 0,917 | 0,868 | 0,966 | 0,929 | 0,870 | 0,988 |
| RSAD2_ IFI27_ IFI44L_ OLAH_ RETN | 0,890 | 0,882 | 0,899 | 0,915 | 0,856 | 0,974 | 0,942 | 0,881 | 1 |
| IFI27_ OAS1_ IFI44L_ SIGLEC1_ FAM20A | 0,890 | 0,881 | 0,899 | 0,910 | 0,844 | 0,975 | 0,947 | 0,876 | 1 |

| Modèle | AUC modèle | IC 95% | | AUC Train | IC 95% Train | | AUC Test | IC 95% Test | |
|---|---|---|---|---|---|---|---|---|---|
| RSAD2_ IFI27_ IFI44L_ HERC6_ RETN | 0,890 | 0,881 | 0,899 | 0,913 | 0,853 | 0,974 | 0,940 | 0,880 | 1 |
| IFI27_ OAS1_ SIGLEC1_ FAM20A_ MMP8 | 0,890 | 0,881 | 0,900 | 0,911 | 0,847 | 0,976 | 0,948 | 0,879 | 1 |
| OAS1_ SIGLEC1_ HERC6_ IL1R2_ FAM20A | 0,890 | 0,881 | 0,899 | 0,913 | 0,853 | 0,972 | 0,959 | 0,905 | 1 |
| IFI27_ SIGLEC1_ ISG15_ SLC1A2_ IL1R2 | 0,890 | 0,883 | 0,897 | 0,924 | 0,878 | 0,969 | 0,949 | 0,900 | 0,998 |
| IFIT1_ SIGLEC1_ HERC6_ FAM20A_ MMP8 | 0,890 | 0,881 | 0,899 | 0,910 | 0,848 | 0,972 | 0,936 | 0,863 | 1 |
| RSAD2_ IFI27_ SIGLEC1_ SLC1A2_ IL1R2 | 0,890 | 0,882 | 0,898 | 0,924 | 0,879 | 0,970 | 0,953 | 0,906 | 1 |
| IFI27_ IFI44L_ SIGLEC1_ HERC6_ FAM20A | 0,890 | 0,881 | 0,899 | 0,911 | 0,848 | 0,974 | 0,947 | 0,870 | 1 |
| RSAD2_ IFI27_ ISG15_ HERC6_ RETN | 0,890 | 0,881 | 0,899 | 0,914 | 0,855 | 0,973 | 0,941 | 0,884 | 0,999 |
| IFIT1_ IFI44L_ SIGLEC1_ IL1R2_ FAM20A | 0,890 | 0,881 | 0,898 | 0,909 | 0,845 | 0,973 | 0,958 | 0,907 | 1 |
| IFI27_ ISG15_ HERC6_ IL1R2_ MMP8 | 0,890 | 0,882 | 0,897 | 0,916 | 0,866 | 0,965 | 0,933 | 0,875 | 0,990 |
| IFI27_ IFI44L_ ISG15_ IL1R2_ RETN | 0,890 | 0,882 | 0,898 | 0,920 | 0,867 | 0,972 | 0,942 | 0,886 | 0,999 |
| RSAD2_ IFI27_ OAS1_ IL1R2_ RETN | 0,890 | 0,882 | 0,898 | 0,918 | 0,864 | 0,972 | 0,943 | 0,886 | 1 |
| IFI27_ OAS1_ IFIT1_ IL1R2_ MMP8 | 0,890 | 0,882 | 0,897 | 0,916 | 0,865 | 0,966 | 0,943 | 0,891 | 0,996 |
| IFI27_ IFIT1_ ISG15_ HERC6_ IL1R2 | 0,890 | 0,882 | 0,897 | 0,915 | 0,865 | 0,965 | 0,938 | 0,884 | 0,992 |
| IFI27_ OAS1_ IL1R2_ RETN_ MMP8 | 0,890 | 0,881 | 0,898 | 0,918 | 0,864 | 0,972 | 0,940 | 0,881 | 0,999 |
| RSAD2_ IFI27_ IL1R2_ RETN_ MMP8 | 0,890 | 0,881 | 0,898 | 0,919 | 0,865 | 0,973 | 0,941 | 0,882 | 1 |
| IFI27_ IFIT1_ IFI44L_ IL1R2_ MMP8 | 0,889 | 0,882 | 0,897 | 0,917 | 0,865 | 0,968 | 0,937 | 0,881 | 0,993 |
| RSAD2_ IFI27_ IFIT1_ HERC6_ RETN | 0,889 | 0,880 | 0,898 | 0,912 | 0,852 | 0,973 | 0,941 | 0,881 | 1 |
| RSAD2_ IFIT1_ SIGLEC1_ IL1R2_ FAM20A | 0,889 | 0,881 | 0,898 | 0,911 | 0,847 | 0,974 | 0,963 | 0,914 | 1 |
| IFI27_ IFIT1_ SIGLEC1_ SLC1A2_ FAM20A | 0,889 | 0,881 | 0,898 | 0,919 | 0,862 | 0,976 | 0,946 | 0,871 | 1 |
| OAS1_ IFIT1_ SIGLEC1_ HERC6_ FAM20A | 0,889 | 0,880 | 0,898 | 0,910 | 0,849 | 0,971 | 0,938 | 0,868 | 1 |
| RSAD2_ IFI27_ OAS1_ IFIT1_ IL1R2 | 0,889 | 0,881 | 0,897 | 0,914 | 0,863 | 0,965 | 0,945 | 0,892 | 0,997 |
| RSAD2_ IFI27_ IFIT1_ IL1R2_ MMP8 | 0,889 | 0,881 | 0,897 | 0,916 | 0,865 | 0,966 | 0,942 | 0,890 | 0,995 |
| IFI27_ OAS1_ IFI44L_ IL1R2_ RETN | 0,889 | 0,881 | 0,897 | 0,918 | 0,865 | 0,971 | 0,939 | 0,881 | 0,997 |
| IFI27_ SIGLEC1_ ISG15_ HERC6_ FAM20A | 0,889 | 0,880 | 0,899 | 0,906 | 0,837 | 0,974 | 0,948 | 0,872 | 1 |

| Modèle | AUC modèle | IC 95% | | AUC Train | IC 95% Train | | AUC Test | IC 95% Test | |
|---|---|---|---|---|---|---|---|---|---|
| SIGLEC1_ ISG15_ HERC6_ IL1R2_ FAM20A | 0,889 | 0,880 | 0,898 | 0,912 | 0,850 | 0,974 | 0,959 | 0,900 | 1 |
| IFI27_ IFIT1_ ISG15_ IL1R2_ RETN | 0,889 | 0,881 | 0,897 | 0,917 | 0,864 | 0,971 | 0,947 | 0,894 | 1,000 |
| IFI27_ OAS1_ IFIT1_ IFI44L_ IL1R2 | 0,889 | 0,881 | 0,897 | 0,916 | 0,864 | 0,968 | 0,938 | 0,883 | 0,993 |
| IFI27_ IFI44L_ SLC1A2_ IL1R2_ RETN | 0,889 | 0,881 | 0,897 | 0,924 | 0,875 | 0,972 | 0,933 | 0,866 | 0,999 |
| IFI27_ OAS1_ IFIT1_ SIGLEC1_ RETN | 0,889 | 0,880 | 0,898 | 0,909 | 0,847 | 0,970 | 0,954 | 0,896 | 1 |
| IFI27_ IFIT1_ SIGLEC1_ SLC1A2_ IL1R2 | 0,889 | 0,881 | 0,897 | 0,924 | 0,878 | 0,970 | 0,954 | 0,909 | 1,000 |
| IFIT1_ SIGLEC1_ IL1R2_ OLAH_ FAM20A | 0,889 | 0,880 | 0,898 | 0,913 | 0,851 | 0,975 | 0,962 | 0,914 | 1 |
| IFI27_ SIGLEC1_ SLC1A2_ IL1R2_ MMP8 | 0,889 | 0,881 | 0,897 | 0,924 | 0,878 | 0,970 | 0,955 | 0,911 | 1 |
| SIGLEC1_ HERC6_ IL1R2_ OLAH_ FAM20A | 0,889 | 0,880 | 0,898 | 0,911 | 0,848 | 0,974 | 0,959 | 0,900 | 1 |
| SIGLEC1_ SLC1A2_ OLAH_ FAM20A_ MMP8 | 0,889 | 0,879 | 0,898 | 0,919 | 0,863 | 0,974 | 0,951 | 0,888 | 1 |
| IFI27_ IFI44L_ ISG15_ IL1R2_ MMP8 | 0,889 | 0,881 | 0,896 | 0,915 | 0,865 | 0,966 | 0,937 | 0,880 | 0,994 |
| IFI27_ IFIT1_ IFI44L_ ISG15_ IL1R2 | 0,889 | 0,880 | 0,897 | 0,916 | 0,864 | 0,968 | 0,939 | 0,885 | 0,994 |
| OAS1_ IFIT1_ SIGLEC1_ OLAH_ FAM20A | 0,889 | 0,879 | 0,898 | 0,906 | 0,837 | 0,975 | 0,955 | 0,906 | 1 |
| RSAD2_ IFIT1_ SIGLEC1_ HERC6_ FAM20A | 0,889 | 0,880 | 0,897 | 0,911 | 0,849 | 0,972 | 0,937 | 0,865 | 1 |
| RSAD2_ IFI27_ IFIT1_ IFI44L_ IL1R2 | 0,888 | 0,881 | 0,896 | 0,915 | 0,863 | 0,968 | 0,938 | 0,883 | 0,993 |
| IFI27_ IFI44L_ SIGLEC1_ FAM20A_ MMP8 | 0,888 | 0,879 | 0,898 | 0,911 | 0,846 | 0,976 | 0,947 | 0,872 | 1 |
| IFI27_ SIGLEC1_ SLC1A2_ FAM20A_ RETN | 0,888 | 0,879 | 0,898 | 0,919 | 0,860 | 0,979 | 0,948 | 0,871 | 1 |
| IFIT1_ SIGLEC1_ OLAH_ FAM20A_ MMP8 | 0,888 | 0,879 | 0,898 | 0,906 | 0,841 | 0,972 | 0,955 | 0,907 | 1 |
| RSAD2_ IFI27_ IFI44L_ IL1R2_ RETN | 0,888 | 0,880 | 0,896 | 0,918 | 0,863 | 0,972 | 0,938 | 0,878 | 0,998 |
| IFI27_ HERC6_ SLC1A2_ IL1R2_ RETN | 0,888 | 0,879 | 0,896 | 0,923 | 0,873 | 0,973 | 0,935 | 0,871 | 0,999 |
| IFI27_ SIGLEC1_ ISG15_ FAM20A_ MMP8 | 0,888 | 0,878 | 0,898 | 0,905 | 0,837 | 0,974 | 0,943 | 0,868 | 1 |
| IFI44L_ SIGLEC1_ ISG15_ IL1R2_ FAM20A | 0,888 | 0,879 | 0,897 | 0,910 | 0,848 | 0,973 | 0,962 | 0,911 | 1 |
| IFI27_ IFI44L_ ISG15_ FAM20A_ RETN | 0,888 | 0,878 | 0,897 | 0,911 | 0,848 | 0,974 | 0,945 | 0,868 | 1 |
| IFI27_ IFIT1_ ISG15_ HERC6_ RETN | 0,888 | 0,878 | 0,897 | 0,910 | 0,848 | 0,971 | 0,943 | 0,882 | 1 |
| IFI27_ SIGLEC1_ HERC6_ SLC1A2_ IL1R2 | 0,888 | 0,880 | 0,896 | 0,924 | 0,878 | 0,970 | 0,957 | 0,912 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L_ SIGLEC1_ IL1R2_ OLAH_ FAM20A | 0,888 | 0,879 | 0,897 | 0,910 | 0,848 | 0,973 | 0,963 | 0,912 | 1 |
| IFI27_ IFIT1_ ISG15_ IL1R2_ MMP8 | 0,888 | 0,880 | 0,895 | 0,915 | 0,864 | 0,966 | 0,941 | 0,888 | 0,994 |
| OAS1_ IFI44L_ SIGLEC1_ IL1R2_ FAM20A | 0,887 | 0,879 | 0,896 | 0,910 | 0,846 | 0,973 | 0,964 | 0,914 | 1 |
| IFIT1_ SIGLEC1_ ISG15_ IL1R2_ FAM20A | 0,887 | 0,879 | 0,896 | 0,913 | 0,850 | 0,975 | 0,962 | 0,914 | 1 |
| RSAD2_ IFI27_ IFI44L_ IL1R2_ MMP8 | 0,887 | 0,880 | 0,895 | 0,915 | 0,863 | 0,966 | 0,936 | 0,880 | 0,992 |
| IFIT1_ IFI44L_ SIGLEC1_ OLAH_ FAM20A | 0,887 | 0,878 | 0,896 | 0,905 | 0,836 | 0,973 | 0,943 | 0,887 | 1 |
| IFI27_ IFIT1_ IFI44L_ HERC6_ RETN | 0,887 | 0,878 | 0,897 | 0,910 | 0,847 | 0,972 | 0,940 | 0,875 | 1 |
| IFIT1_ SIGLEC1_ IL1R2_ FAM20A_ RETN | 0,887 | 0,878 | 0,896 | 0,909 | 0,846 | 0,972 | 0,961 | 0,910 | 1 |
| RSAD2_ IFI27_ OAS1_ IFI44L_ IL1R2 | 0,887 | 0,880 | 0,895 | 0,914 | 0,863 | 0,966 | 0,940 | 0,886 | 0,994 |
| RSAD2_ IFI27_ OAS1_ SIGLEC1_ RETN | 0,887 | 0,878 | 0,896 | 0,909 | 0,847 | 0,971 | 0,957 | 0,897 | 1 |
| OAS1_ SIGLEC1_ IL1R2_ OLAH_ FAM20A | 0,887 | 0,878 | 0,896 | 0,910 | 0,847 | 0,973 | 0,962 | 0,910 | 1 |
| RSAD2_ IFI27_ OAS1_ IL1R2_ MMP8 | 0,887 | 0,879 | 0,895 | 0,915 | 0,865 | 0,966 | 0,939 | 0,884 | 0,994 |
| RSAD2_ IFI27_ ISG15_ IL1R2_ RETN | 0,887 | 0,879 | 0,895 | 0,918 | 0,865 | 0,972 | 0,940 | 0,882 | 0,998 |
| IFIT1_ SIGLEC1_ HERC6_ SLC1A2_ FAM20A | 0,887 | 0,878 | 0,896 | 0,918 | 0,864 | 0,971 | 0,938 | 0,862 | 1 |
| RSAD2_ OAS1_ SIGLEC1_ IL1R2_ FAM20A | 0,887 | 0,878 | 0,896 | 0,910 | 0,847 | 0,973 | 0,963 | 0,912 | 1 |
| IFI27_ IFI44L_ FAM20A_ RETN_ MMP8 | 0,887 | 0,877 | 0,897 | 0,914 | 0,852 | 0,977 | 0,946 | 0,870 | 1 |
| IFI27_ IFI44L_ SLC1A2_ IL1R2_ MMP8 | 0,887 | 0,879 | 0,895 | 0,922 | 0,876 | 0,968 | 0,929 | 0,864 | 0,995 |
| RSAD2_ IFI27_ HERC6_ RETN_ MMP8 | 0,887 | 0,878 | 0,896 | 0,911 | 0,850 | 0,972 | 0,933 | 0,868 | 0,997 |
| IFI27_ OAS1_ ISG15_ IL1R2_ RETN | 0,887 | 0,879 | 0,895 | 0,919 | 0,866 | 0,971 | 0,940 | 0,882 | 0,998 |
| IFI27_ IFIT1_ IFI44L_ FAM20A_ RETN | 0,887 | 0,877 | 0,897 | 0,910 | 0,846 | 0,974 | 0,943 | 0,866 | 1 |
| IFI27_ SIGLEC1_ HERC6_ FAM20A_ MMP8 | 0,887 | 0,877 | 0,897 | 0,904 | 0,836 | 0,973 | 0,943 | 0,865 | 1 |
| IFI27_ IFIT1_ HERC6_ RETN_ MMP8 | 0,887 | 0,877 | 0,896 | 0,908 | 0,845 | 0,970 | 0,930 | 0,863 | 0,998 |
| IFI27_ IFIT1_ SLC1A2_ IL1R2_ RETN | 0,887 | 0,878 | 0,895 | 0,919 | 0,867 | 0,971 | 0,939 | 0,878 | 1 |
| IFI27_ OAS1_ IFIT1_ ISG15_ IL1R2 | 0,887 | 0,879 | 0,895 | 0,915 | 0,864 | 0,966 | 0,943 | 0,890 | 0,997 |
| IFI27_ HERC6_ SLC1A2_ FAM20A_ RETN | 0,887 | 0,877 | 0,896 | 0,919 | 0,861 | 0,976 | 0,940 | 0,860 | 1 |

| Modèle | AUC modèle | IC 95% | | AUC Train | IC 95% Train | | AUC Test | IC 95% Test | |
|---|---|---|---|---|---|---|---|---|---|
| RSAD2_ IFI27_ IFIT1_ ISG15_ IL1R2 | 0,887 | 0,879 | 0,894 | 0,915 | 0,864 | 0,966 | 0,941 | 0,888 | 0,994 |
| SIGLEC1_ ISG15_ IL1R2_ OLAH_ FAM20A | 0,887 | 0,878 | 0,895 | 0,908 | 0,845 | 0,972 | 0,963 | 0,912 | 1 |
| IFI27_ IFI44L_ ISG15_ HERC6_ RETN | 0,886 | 0,877 | 0,896 | 0,908 | 0,846 | 0,971 | 0,939 | 0,873 | 1 |
| RSAD2_ SIGLEC1_ IL1R2_ OLAH_ FAM20A | 0,886 | 0,877 | 0,895 | 0,909 | 0,846 | 0,972 | 0,963 | 0,912 | 1 |
| IFI27_ OAS1_ IFIT1_ SLC1A2_ IL1R2 | 0,886 | 0,878 | 0,895 | 0,922 | 0,876 | 0,968 | 0,937 | 0,875 | 0,999 |
| IFI27_ OAS1_ IFI44L_ IL1R2_ MMP8 | 0,886 | 0,879 | 0,894 | 0,915 | 0,864 | 0,965 | 0,930 | 0,871 | 0,989 |
| IFI27_ OAS1_ IFI44L_ SIGLEC1_ RETN | 0,886 | 0,877 | 0,895 | 0,910 | 0,849 | 0,970 | 0,954 | 0,894 | 1 |
| IFI44L_ SIGLEC1_ IL1R2_ FAM20A_ RETN | 0,886 | 0,877 | 0,895 | 0,907 | 0,844 | 0,971 | 0,962 | 0,909 | 1 |
| IFI27_ IFIT1_ SIGLEC1_ ISG15_ RETN | 0,886 | 0,877 | 0,895 | 0,908 | 0,846 | 0,970 | 0,955 | 0,900 | 1 |
| IFI27_ OAS1_ HERC6_ SLC1A2_ RETN | 0,886 | 0,877 | 0,895 | 0,915 | 0,858 | 0,973 | 0,932 | 0,857 | 1 |
| SIGLEC1_ ISG15_ IL1R2_ FAM20A_ RETN | 0,886 | 0,877 | 0,895 | 0,906 | 0,842 | 0,971 | 0,962 | 0,907 | 1 |
| IFI27_ OAS1_ HERC6_ SLC1A2_ IL1R2 | 0,886 | 0,878 | 0,894 | 0,920 | 0,875 | 0,966 | 0,918 | 0,845 | 0,992 |
| SIGLEC1_ HERC6_ IL1R2_ FAM20A_ RETN | 0,886 | 0,877 | 0,895 | 0,911 | 0,848 | 0,973 | 0,959 | 0,898 | 1 |
| IFI27_ OAS1_ SIGLEC1_ ISG15_ RETN | 0,886 | 0,877 | 0,895 | 0,910 | 0,848 | 0,971 | 0,953 | 0,892 | 1 |
| IFI27_ OAS1_ SIGLEC1_ SLC1A2_ RETN | 0,886 | 0,877 | 0,894 | 0,915 | 0,857 | 0,972 | 0,948 | 0,880 | 1 |
| RSAD2_ IFI44L_ SIGLEC1_ IL1R2_ FAM20A | 0,886 | 0,877 | 0,895 | 0,911 | 0,848 | 0,973 | 0,964 | 0,914 | 1 |
| RSAD2_ IFI27_ HERC6_ SLC1A2_ IL1R2 | 0,886 | 0,878 | 0,894 | 0,920 | 0,875 | 0,966 | 0,929 | 0,863 | 0,995 |
| IFI27_ OAS1_ SIGLEC1_ SLC1A2_ FAM20A | 0,886 | 0,876 | 0,895 | 0,918 | 0,859 | 0,977 | 0,946 | 0,872 | 1 |
| IFIT1_ SIGLEC1_ SLC1A2_ IL1R2_ FAM20A | 0,886 | 0,877 | 0,894 | 0,917 | 0,863 | 0,971 | 0,951 | 0,891 | 1 |
| RSAD2_ IFI27_ SLC1A2_ IL1R2_ RETN | 0,886 | 0,877 | 0,894 | 0,922 | 0,870 | 0,973 | 0,929 | 0,860 | 0,998 |
| IFIT1_ SIGLEC1_ SLC1A2_ OLAH_ FAM20A | 0,886 | 0,876 | 0,895 | 0,919 | 0,864 | 0,974 | 0,945 | 0,885 | 1 |
| SIGLEC1_ HERC6_ SLC1A2_ IL1R2_ FAM20A | 0,886 | 0,876 | 0,895 | 0,917 | 0,864 | 0,971 | 0,948 | 0,875 | 1 |
| IFI44L_ SIGLEC1_ SLC1A2_ IL1R2_ FAM20A | 0,886 | 0,877 | 0,894 | 0,916 | 0,862 | 0,970 | 0,953 | 0,886 | 1 |
| IFI27_ OAS1_ SLC1A2_ IL1R2_ RETN | 0,886 | 0,877 | 0,894 | 0,922 | 0,872 | 0,972 | 0,926 | 0,856 | 0,997 |
| IFI27_ IFI44L_ ISG15_ SLC1A2_ IL1R2 | 0,886 | 0,878 | 0,893 | 0,922 | 0,877 | 0,968 | 0,933 | 0,868 | 0,997 |

| Modèle | AUC modèle | IC 95% | | AUC Train | IC 95% Train | | AUC Test | IC 95% Test | |
|---|---|---|---|---|---|---|---|---|---|
| IFI44L_ SIGLEC1_ SLC1A2_ OLAH_ FAM20A | 0,886 | 0,876 | 0,895 | 0,912 | 0,853 | 0,971 | 0,949 | 0,887 | 1 |
| RSAD2_ IFI27_ SIGLEC1_ SLC1A2_ FAM20A | 0,886 | 0,876 | 0,895 | 0,919 | 0,859 | 0,978 | 0,947 | 0,873 | 1 |
| IFIT1_ IFI44L_ SIGLEC1_ ISG15_ FAM20A | 0,885 | 0,877 | 0,894 | 0,899 | 0,829 | 0,969 | 0,940 | 0,878 | 1 |
| SIGLEC1_ SLC1A2_ IL1R2_ OLAH_ FAM20A | 0,885 | 0,877 | 0,894 | 0,915 | 0,860 | 0,970 | 0,954 | 0,893 | 1 |
| IFI27_ IFI44L_ HERC6_ RETN_ MMP8 | 0,885 | 0,876 | 0,895 | 0,907 | 0,844 | 0,970 | 0,932 | 0,863 | 1 |
| IFI27_ IFIT1_ SLC1A2_ IL1R2_ MMP8 | 0,885 | 0,877 | 0,893 | 0,922 | 0,876 | 0,968 | 0,936 | 0,875 | 0,997 |
| RSAD2_ SIGLEC1_ IL1R2_ FAM20A_ RETN | 0,885 | 0,876 | 0,895 | 0,908 | 0,845 | 0,972 | 0,962 | 0,907 | 1 |
| SIGLEC1_ IL1R2_ OLAH_ FAM20A_ RETN | 0,885 | 0,876 | 0,894 | 0,907 | 0,843 | 0,971 | 0,962 | 0,907 | 1 |
| RSAD2_ SIGLEC1_ SLC1A2_ IL1R2_ FAM20A | 0,885 | 0,876 | 0,894 | 0,916 | 0,862 | 0,970 | 0,954 | 0,890 | 1 |
| RSAD2_ IFIT1_ SIGLEC1_ OLAH_ FAM20A | 0,885 | 0,876 | 0,894 | 0,904 | 0,834 | 0,973 | 0,953 | 0,902 | 1 |
| OAS1_ SIGLEC1_ IL1R2_ FAM20A_ RETN | 0,885 | 0,876 | 0,894 | 0,907 | 0,844 | 0,971 | 0,962 | 0,907 | 1 |
| IFI27_ IFIT1_ IFI44L_ SIGLEC1_ RETN | 0,885 | 0,876 | 0,894 | 0,908 | 0,846 | 0,970 | 0,951 | 0,894 | 1 |
| OAS1_ SIGLEC1_ ISG15_ IL1R2_ FAM20A | 0,885 | 0,876 | 0,894 | 0,909 | 0,846 | 0,973 | 0,964 | 0,914 | 1 |
| RSAD2_ IFI27_ IFIT1_ SIGLEC1_ RETN | 0,885 | 0,876 | 0,894 | 0,908 | 0,846 | 0,970 | 0,955 | 0,900 | 1 |
| RSAD2_ IFI27_ IFIT1_ SLC1A2_ IL1R2 | 0,885 | 0,877 | 0,893 | 0,922 | 0,876 | 0,968 | 0,936 | 0,875 | 0,997 |
| IFI27_ IFI44L_ SIGLEC1_ ISG15_ RETN | 0,885 | 0,876 | 0,894 | 0,906 | 0,844 | 0,969 | 0,952 | 0,895 | 1 |
| RSAD2_ IFI27_ ISG15_ IL1R2_ MMP8 | 0,885 | 0,877 | 0,893 | 0,915 | 0,864 | 0,966 | 0,936 | 0,879 | 0,992 |
| IFI27_ SIGLEC1_ ISG15_ HERC6_ RETN | 0,885 | 0,875 | 0,895 | 0,906 | 0,841 | 0,971 | 0,963 | 0,916 | 1 |
| IFI27_ IFIT1_ ISG15_ FAM20A_ RETN | 0,885 | 0,875 | 0,895 | 0,911 | 0,849 | 0,972 | 0,942 | 0,865 | 1 |
| RSAD2_ IFI27_ IFI44L_ ISG15_ IL1R2 | 0,885 | 0,877 | 0,892 | 0,915 | 0,864 | 0,966 | 0,936 | 0,880 | 0,992 |
| RSAD2_ SIGLEC1_ ISG15_ IL1R2_ FAM20A | 0,885 | 0,876 | 0,894 | 0,909 | 0,845 | 0,972 | 0,963 | 0,912 | 1 |
| IFI27_ OAS1_ SIGLEC1_ RETN_ MMP8 | 0,885 | 0,875 | 0,894 | 0,911 | 0,850 | 0,972 | 0,957 | 0,897 | 1 |
| SIGLEC1_ SLC1A2_ IL1R2_ FAM20A_ RETN | 0,885 | 0,876 | 0,894 | 0,916 | 0,862 | 0,969 | 0,953 | 0,885 | 1 |
| RSAD2_ IFI27_ OAS1_ SLC1A2_ IL1R2 | 0,885 | 0,877 | 0,893 | 0,921 | 0,875 | 0,967 | 0,930 | 0,862 | 0,999 |
| IFI27_ ISG15_ SLC1A2_ IL1R2_ RETN | 0,885 | 0,877 | 0,893 | 0,919 | 0,868 | 0,970 | 0,918 | 0,839 | 0,998 |

| Modèle | AUC modèle | IC 95% | | AUC Train | IC 95% Train | | AUC Test | IC 95% Test | |
|---|---|---|---|---|---|---|---|---|---|
| IFI27_ IFIT1_ HERC6_ SLC1A2_ IL1R2 | 0,885 | 0,876 | 0,893 | 0,922 | 0,876 | 0,968 | 0,938 | 0,878 | 0,998 |
| OAS1_ IFIT1_ IFI44L_ SIGLEC1_ FAM20A | 0,885 | 0,875 | 0,894 | 0,902 | 0,832 | 0,972 | 0,938 | 0,870 | 1 |
| SIGLEC1_ ISG15_ SLC1A2_ IL1R2_ FAM20A | 0,885 | 0,876 | 0,893 | 0,914 | 0,859 | 0,969 | 0,953 | 0,888 | 1 |
| IFI27_ SLC1A2_ IL1R2_ RETN_ MMP8 | 0,884 | 0,876 | 0,893 | 0,921 | 0,872 | 0,970 | 0,915 | 0,832 | 0,998 |
| RSAD2_ IFI27_ OAS1_ ISG15_ IL1R2 | 0,884 | 0,877 | 0,892 | 0,915 | 0,865 | 0,966 | 0,939 | 0,884 | 0,994 |
| RSAD2_ IFI27_ IFI44L_ FAM20A_ RETN | 0,884 | 0,874 | 0,894 | 0,910 | 0,846 | 0,974 | 0,942 | 0,865 | 1 |
| IFI27_ IFIT1_ ISG15_ SLC1A2_ IL1R2 | 0,884 | 0,876 | 0,893 | 0,922 | 0,876 | 0,968 | 0,937 | 0,877 | 0,997 |
| RSAD2_ IFI27_ SLC1A2_ IL1R2_ MMP8 | 0,884 | 0,877 | 0,892 | 0,921 | 0,874 | 0,969 | 0,934 | 0,870 | 0,997 |
| IFI27_ OAS1_ IFI44L_ ISG15_ IL1R2 | 0,884 | 0,877 | 0,892 | 0,914 | 0,863 | 0,964 | 0,932 | 0,873 | 0,990 |
| IFI27_ IFI44L_ HERC6_ SLC1A2_ IL1R2 | 0,884 | 0,876 | 0,893 | 0,921 | 0,875 | 0,966 | 0,929 | 0,864 | 0,995 |
| IFI27_ IFIT1_ HERC6_ FAM20A_ MMP8 | 0,884 | 0,875 | 0,894 | 0,911 | 0,851 | 0,970 | 0,926 | 0,842 | 1 |
| IFI27_ IFIT1_ IFI44L_ SLC1A2_ IL1R2 | 0,884 | 0,876 | 0,892 | 0,922 | 0,875 | 0,968 | 0,939 | 0,880 | 0,998 |
| IFI27_ OAS1_ ISG15_ IL1R2_ MMP8 | 0,884 | 0,876 | 0,892 | 0,914 | 0,864 | 0,965 | 0,932 | 0,873 | 0,990 |
| OAS1_ IFIT1_ SIGLEC1_ FAM20A_ RETN | 0,884 | 0,875 | 0,893 | 0,903 | 0,834 | 0,972 | 0,949 | 0,889 | 1 |
| OAS1_ SIGLEC1_ SLC1A2_ IL1R2_ FAM20A | 0,884 | 0,875 | 0,893 | 0,916 | 0,861 | 0,970 | 0,953 | 0,890 | 1 |
| RSAD2_ IFI27_ SIGLEC1_ ISG15_ RETN | 0,884 | 0,875 | 0,893 | 0,909 | 0,847 | 0,970 | 0,953 | 0,896 | 1 |
| OAS1_ SIGLEC1_ SLC1A2_ OLAH_ FAM20A | 0,884 | 0,874 | 0,893 | 0,915 | 0,857 | 0,972 | 0,951 | 0,895 | 1 |
| IFI44L_ SIGLEC1_ OLAH_ FAM20A_ MMP8 | 0,884 | 0,874 | 0,893 | 0,902 | 0,834 | 0,970 | 0,966 | 0,923 | 1 |
| SIGLEC1_ ISG15_ SLC1A2_ OLAH_ FAM20A | 0,884 | 0,874 | 0,893 | 0,913 | 0,854 | 0,971 | 0,950 | 0,891 | 1 |
| SIGLEC1_ HERC6_ SLC1A2_ OLAH_ FAM20A | 0,883 | 0,874 | 0,893 | 0,914 | 0,857 | 0,972 | 0,947 | 0,874 | 1 |
| IFIT1_ IFI44L_ SIGLEC1_ FAM20A_ MMP8 | 0,883 | 0,874 | 0,893 | 0,901 | 0,832 | 0,970 | 0,938 | 0,869 | 1 |
| IFI27_ OAS1_ IFI44L_ FAM20A_ RETN | 0,883 | 0,874 | 0,893 | 0,911 | 0,848 | 0,974 | 0,946 | 0,869 | 1 |
| IFI27_ IFIT1_ SIGLEC1_ RETN_ MMP8 | 0,883 | 0,874 | 0,893 | 0,909 | 0,847 | 0,971 | 0,957 | 0,902 | 1 |
| RSAD2_ SIGLEC1_ SLC1A2_ OLAH_ FAM20A | 0,883 | 0,874 | 0,893 | 0,915 | 0,858 | 0,972 | 0,950 | 0,891 | 1 |
| RSAD2_ IFI27_ IFI44L_ HERC6_ FAM20A | 0,883 | 0,873 | 0,893 | 0,907 | 0,843 | 0,970 | 0,930 | 0,851 | 1 |

104

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| RSAD2_ IFI27_ IFI44L_ SIGLEC1_ RETN | 0,883 | 0,874 | 0,892 | 0,908 | 0,846 | 0,969 | 0,951 | 0,893 | 1 |
| IFI27_ SIGLEC1_ ISG15_ SLC1A2_ FAM20A | 0,883 | 0,874 | 0,893 | 0,911 | 0,850 | 0,971 | 0,948 | 0,872 | 1 |
| OAS1_ IFIT1_ SIGLEC1_ ISG15_ FAM20A | 0,883 | 0,874 | 0,893 | 0,900 | 0,829 | 0,970 | 0,946 | 0,879 | 1 |
| IFI44L_ SLC1A2_ OLAH_ FAM20A_ MMP8 | 0,883 | 0,874 | 0,892 | 0,911 | 0,854 | 0,969 | 0,929 | 0,855 | 1 |
| RSAD2_ IFI27_ ISG15_ SLC1A2_ IL1R2 | 0,883 | 0,875 | 0,891 | 0,922 | 0,876 | 0,968 | 0,929 | 0,863 | 0,996 |
| IFI27_ ISG15_ HERC6_ SLC1A2_ IL1R2 | 0,883 | 0,875 | 0,891 | 0,922 | 0,877 | 0,967 | 0,925 | 0,858 | 0,992 |
| IFI27_ IFIT1_ FAM20A_ RETN_ MMP8 | 0,883 | 0,873 | 0,893 | 0,912 | 0,850 | 0,974 | 0,943 | 0,866 | 1 |
| IFI44L_ ISG15_ HERC6_ IL1R2_ FAM20A | 0,883 | 0,874 | 0,892 | 0,907 | 0,846 | 0,967 | 0,940 | 0,865 | 1 |
| IFIT1_ IFI44L_ SIGLEC1_ FAM20A_ RETN | 0,883 | 0,874 | 0,892 | 0,900 | 0,831 | 0,970 | 0,941 | 0,880 | 1 |
| IFI27_ IFI44L_ SIGLEC1_ SLC1A2_ FAM20A | 0,883 | 0,873 | 0,892 | 0,911 | 0,851 | 0,972 | 0,947 | 0,870 | 1 |
| RSAD2_ OAS1_ IFIT1_ SIGLEC1_ FAM20A | 0,883 | 0,873 | 0,892 | 0,899 | 0,828 | 0,970 | 0,947 | 0,880 | 1 |
| IFI27_ OAS1_ ISG15_ FAM20A_ RETN | 0,883 | 0,873 | 0,892 | 0,911 | 0,849 | 0,972 | 0,943 | 0,866 | 1 |
| IFIT1_ IFI44L_ SIGLEC1_ SLC1A2_ FAM20A | 0,883 | 0,874 | 0,892 | 0,911 | 0,852 | 0,970 | 0,936 | 0,861 | 1 |
| RSAD2_ IFI27_ HERC6_ FAM20A_ MMP8 | 0,883 | 0,873 | 0,892 | 0,911 | 0,852 | 0,969 | 0,927 | 0,846 | 1 |
| RSAD2_ IFI27_ IFI44L_ SLC1A2_ IL1R2 | 0,883 | 0,875 | 0,890 | 0,921 | 0,875 | 0,967 | 0,933 | 0,868 | 0,997 |
| SIGLEC1_ HERC6_ OLAH_ FAM20A_ MMP8 | 0,883 | 0,872 | 0,893 | 0,902 | 0,835 | 0,970 | 0,963 | 0,914 | 1 |
| SIGLEC1_ OLAH_ FAM20A_ RETN_ MMP8 | 0,882 | 0,872 | 0,893 | 0,905 | 0,838 | 0,973 | 0,970 | 0,929 | 1 |
| IFIT1_ SIGLEC1_ ISG15_ OLAH_ FAM20A | 0,882 | 0,873 | 0,892 | 0,901 | 0,832 | 0,970 | 0,948 | 0,893 | 1 |
| SIGLEC1_ ISG15_ OLAH_ FAM20A_ MMP8 | 0,882 | 0,873 | 0,892 | 0,902 | 0,834 | 0,970 | 0,963 | 0,919 | 1 |
| RSAD2_ SIGLEC1_ HERC6_ OLAH_ FAM20A | 0,882 | 0,873 | 0,892 | 0,907 | 0,844 | 0,969 | 0,946 | 0,888 | 1 |
| IFI27_ ISG15_ SLC1A2_ IL1R2_ MMP8 | 0,882 | 0,875 | 0,890 | 0,919 | 0,872 | 0,966 | 0,921 | 0,847 | 0,994 |
| RSAD2_ IFI27_ SIGLEC1_ RETN_ MMP8 | 0,882 | 0,873 | 0,892 | 0,912 | 0,852 | 0,973 | 0,957 | 0,902 | 1 |
| ISG15_ HERC6_ IL1R2_ OLAH_ FAM20A | 0,882 | 0,873 | 0,892 | 0,906 | 0,846 | 0,966 | 0,935 | 0,858 | 1 |
| IFIT1_ ISG15_ HERC6_ IL1R2_ FAM20A | 0,882 | 0,873 | 0,891 | 0,905 | 0,844 | 0,965 | 0,933 | 0,851 | 1 |
| SIGLEC1_ SLC1A2_ OLAH_ FAM20A_ RETN | 0,882 | 0,873 | 0,892 | 0,913 | 0,854 | 0,972 | 0,950 | 0,890 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_ OAS1_ HERC6_ FAM20A_ MMP8 | 0,882 | 0,872 | 0,892 | 0,910 | 0,851 | 0,969 | 0,927 | 0,846 | 1 |
| IFI44L_ SIGLEC1_ HERC6_ OLAH_ FAM20A | 0,882 | 0,872 | 0,892 | 0,903 | 0,836 | 0,969 | 0,955 | 0,896 | 1 |
| IFI44L_ SIGLEC1_ SLC1A2_ OLAH_ MMP8 | 0,882 | 0,874 | 0,891 | 0,912 | 0,856 | 0,967 | 0,957 | 0,914 | 0,999 |
| IFIT1_ SIGLEC1_ FAM20A_ RETN_ MMP8 | 0,882 | 0,873 | 0,891 | 0,906 | 0,840 | 0,972 | 0,947 | 0,885 | 1 |
| IFI27_ HERC6_ SLC1A2_ IL1R2_ MMP8 | 0,882 | 0,874 | 0,890 | 0,920 | 0,874 | 0,967 | 0,925 | 0,857 | 0,993 |
| OAS1_ IFIT1_ SIGLEC1_ FAM20A_ MMP8 | 0,882 | 0,873 | 0,892 | 0,901 | 0,832 | 0,971 | 0,948 | 0,881 | 1 |
| RSAD2_ IFI27_ OAS1_ FAM20A_ RETN | 0,882 | 0,873 | 0,892 | 0,909 | 0,847 | 0,970 | 0,937 | 0,858 | 1 |
| RSAD2_ IFI27_ HERC6_ SLC1A2_ RETN | 0,882 | 0,873 | 0,891 | 0,912 | 0,853 | 0,971 | 0,934 | 0,864 | 1 |
| IFI27_ IFIT1_ SIGLEC1_ SLC1A2_ RETN | 0,882 | 0,873 | 0,891 | 0,915 | 0,859 | 0,971 | 0,955 | 0,900 | 1 |
| IFI27_ IFI44L_ SIGLEC1_ RETN_ MMP8 | 0,882 | 0,873 | 0,891 | 0,907 | 0,844 | 0,969 | 0,953 | 0,897 | 1 |
| OAS1_ ISG15_ HERC6_ IL1R2_ FAM20A | 0,882 | 0,873 | 0,891 | 0,904 | 0,843 | 0,965 | 0,933 | 0,850 | 1 |
| IFI27_OAS1_IFIT1_FAM20A_RETN | 0,882 | 0,872 | 0,892 | 0,909 | 0,846 | 0,971 | 0,942 | 0,864 | 1 |
| IFI27_ ISG15_ HERC6_ RETN_ MMP8 | 0,882 | 0,872 | 0,892 | 0,905 | 0,840 | 0,969 | 0,947 | 0,886 | 1 |
| RSAD2_ ISG15_ HERC6_ IL1R2_ FAM20A | 0,882 | 0,872 | 0,891 | 0,906 | 0,846 | 0,966 | 0,932 | 0,848 | 1 |
| ISG15_ HERC6_ IL1R2_ FAM20A_ MMP8 | 0,882 | 0,873 | 0,891 | 0,907 | 0,848 | 0,965 | 0,927 | 0,841 | 1 |
| IFI27_ OAS1_ SLC1A2_ IL1R2_ MMP8 | 0,882 | 0,874 | 0,890 | 0,920 | 0,874 | 0,967 | 0,924 | 0,856 | 0,992 |
| IFI27_ OAS1_ IFI44L_ SLC1A2_ IL1R2 | 0,882 | 0,873 | 0,890 | 0,922 | 0,877 | 0,967 | 0,929 | 0,865 | 0,994 |
| IFI27_ IFI44L_ SLCIA2_ FAM20A_ RETN | 0,882 | 0,872 | 0,891 | 0,911 | 0,850 | 0,973 | 0,941 | 0,861 | 1 |
| IFI44L_ SLC1A2_ IL1R2_ OLAH_ FAM20A | 0,882 | 0,873 | 0,891 | 0,912 | 0,855 | 0,968 | 0,928 | 0,852 | 1 |
| RSAD2_ IFIT1_ IFI44L_ SIGLEC1_ FAM20A | 0,882 | 0,873 | 0,891 | 0,899 | 0,829 | 0,970 | 0,939 | 0,875 | 1 |
| IFI27_ SIGLEC1_ HERC6_ RETN_ MMP8 | 0,882 | 0,872 | 0,891 | 0,904 | 0,838 | 0,969 | 0,961 | 0,912 | 1 |
| IFI44L_ HERC6_ IL1R2_ OLAH_ FAM20A | 0,882 | 0,872 | 0,891 | 0,905 | 0,844 | 0,967 | 0,938 | 0,864 | 1 |
| ISG15_ HERC6_ IL1R2_ FAM20A_ RETN | 0,882 | 0,872 | 0,891 | 0,907 | 0,847 | 0,967 | 0,933 | 0,849 | 1 |
| HERC6_ IL1R2_ OLAH_ FAM20A_ MMP8 | 0,881 | 0,872 | 0,890 | 0,908 | 0,851 | 0,965 | 0,932 | 0,851 | 1 |
| RSAD2_ SIGLEC1_ OLAH_ FAM20A_ MMP8 | 0,881 | 0,871 | 0,891 | 0,904 | 0,838 | 0,971 | 0,964 | 0,924 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_SIGLEC1_SLC1A2_FAM20A_MMP8 | 0,881 | 0,872 | 0,891 | 0,909 | 0,846 | 0,972 | 0,947 | 0,870 | 1 |
| RSAD2_IFI27_SIGLEC1_SLC1A2_RETN | 0,881 | 0,872 | 0,890 | 0,914 | 0,857 | 0,971 | 0,951 | 0,893 | 1 |
| RSAD2_IFI27_IFIT1_FAM20A_RETN | 0,881 | 0,871 | 0,891 | 0,911 | 0,849 | 0,972 | 0,942 | 0,865 | 1 |
| IFIT1_SIGLEC1_OLAH_FAM20A_RETN | 0,881 | 0,872 | 0,890 | 0,903 | 0,833 | 0,972 | 0,948 | 0,893 | 1 |
| IFIT1_SIGLEC1_SLC1A2_OLAH_MMP8 | 0,881 | 0,872 | 0,890 | 0,911 | 0,856 | 0,967 | 0,954 | 0,911 | 0,998 |
| IFI27_OAS1_ISG15_SLC1A2_IL1R2 | 0,881 | 0,873 | 0,889 | 0,922 | 0,876 | 0,967 | 0,923 | 0,853 | 0,992 |
| RSAD2_IFI27_ISG15_FAM20A_RETN | 0,881 | 0,871 | 0,891 | 0,910 | 0,848 | 0,971 | 0,942 | 0,864 | 1 |
| SIGLEC1_ISG15_HERC6_OLAH_FAM20A | 0,881 | 0,871 | 0,891 | 0,902 | 0,835 | 0,970 | 0,957 | 0,901 | 1 |
| IFI27_IFI44L_HERC6_FAM20A_MMP8 | 0,881 | 0,871 | 0,891 | 0,906 | 0,844 | 0,969 | 0,932 | 0,851 | 1 |
| IFI44L_SIGLEC1_HERC6_IL1R2_MMP8 | 0,881 | 0,873 | 0,889 | 0,902 | 0,843 | 0,961 | 0,961 | 0,921 | 1 |
| IFI27_ISG15_HERC6_FAM20A_MMP8 | 0,881 | 0,871 | 0,891 | 0,907 | 0,844 | 0,969 | 0,932 | 0,851 | 1 |
| OAS1_IFIT1_SIGLEC1_HERC6_IL1R2 | 0,881 | 0,872 | 0,889 | 0,903 | 0,844 | 0,962 | 0,952 | 0,907 | 0,997 |
| OAS1_IFIT1_SIGLEC1_SLC1A2_FAM20A | 0,881 | 0,871 | 0,890 | 0,907 | 0,846 | 0,968 | 0,945 | 0,873 | 1 |
| IFI27_IFI44L_SIGLEC1_SLC1A2_RETN | 0,881 | 0,872 | 0,889 | 0,915 | 0,858 | 0,972 | 0,953 | 0,896 | 1 |
| IFI27_ISG15_FAM20A_RETN_MMP8 | 0,881 | 0,870 | 0,891 | 0,909 | 0,848 | 0,970 | 0,933 | 0,850 | 1 |
| IFI44L_HERC6_IL1R2_FAM20A_MMP8 | 0,881 | 0,872 | 0,890 | 0,908 | 0,848 | 0,968 | 0,934 | 0,851 | 1 |
| RSAD2_OAS1_HERC6_IL1R2_FAM20A | 0,881 | 0,871 | 0,890 | 0,904 | 0,842 | 0,967 | 0,929 | 0,845 | 1 |
| IFI44L_SIGLEC1_SLC1A2_IL1R2_OLAH | 0,881 | 0,872 | 0,889 | 0,913 | 0,858 | 0,968 | 0,963 | 0,925 | 1 |
| IFI27_SIGLEC1_HERC6_SLC1A2_FAM20A | 0,881 | 0,871 | 0,890 | 0,910 | 0,847 | 0,972 | 0,949 | 0,873 | 1 |
| SIGLEC1_HERC6_FAM20A_RETN_MMP8 | 0,881 | 0,870 | 0,891 | 0,905 | 0,836 | 0,973 | 0,957 | 0,891 | 1 |
| IFI27_IFIT1_SIGLEC1_HERC6_MMP8 | 0,881 | 0,872 | 0,889 | 0,905 | 0,847 | 0,963 | 0,909 | 0,834 | 0,984 |
| IFIT1_IFI44L_HERC6_IL1R2_FAM20A | 0,881 | 0,872 | 0,889 | 0,906 | 0,845 | 0,967 | 0,935 | 0,855 | 1 |
| OAS1_HERC6_IL1R2_FAM20A_MMP8 | 0,880 | 0,872 | 0,889 | 0,905 | 0,843 | 0,966 | 0,934 | 0,853 | 1 |
| OAS1_SIGLEC1_SLC1A2_OLAH_MMP8 | 0,880 | 0,871 | 0,890 | 0,911 | 0,855 | 0,967 | 0,958 | 0,916 | 0,999 |
| IFIT1_SIGLEC1_HERC6_SLC1A2_OLAH | 0,880 | 0,871 | 0,890 | 0,912 | 0,857 | 0,967 | 0,943 | 0,893 | 0,994 |

| Modèle | AUC modèle | IC 95% | | AUC Train | IC 95% Train | | AUC Test | IC 95% Test | |
|---|---|---|---|---|---|---|---|---|---|
| OAS1_ SIGLEC1_ HERC6_ OLAH_ FAM20A | 0,880 | 0,871 | 0,890 | 0,905 | 0,841 | 0,969 | 0,954 | 0,901 | 1 |
| RSAD2_ SIGLEC1_ SLC1A2_ OLAH_ MMP8 | 0,880 | 0,871 | 0,889 | 0,912 | 0,857 | 0,968 | 0,957 | 0,915 | 0,998 |
| RSAD2_ IFIT1_ SIGLEC1_ ISG15_ FAM20A | 0,880 | 0,871 | 0,889 | 0,900 | 0,830 | 0,970 | 0,945 | 0,881 | 1 |
| SIGLEC1_ ISG15_ SLC1A2_ OLAH_ MMP8 | 0,880 | 0,872 | 0,889 | 0,912 | 0,857 | 0,968 | 0,952 | 0,907 | 0,997 |
| SIGLEC1_ SLC1A2_ IL1R2_ OLAH_ MMP8 | 0,880 | 0,872 | 0,889 | 0,912 | 0,858 | 0,967 | 0,966 | 0,931 | 1 |
| OAS1_ SIGLEC1_ OLAH_ FAM20A_ MMP8 | 0,880 | 0,870 | 0,890 | 0,901 | 0,833 | 0,969 | 0,963 | 0,920 | 1 |
| IFI44L_ SIGLEC1_ ISG15_ SLC1A2_ OLAH | 0,880 | 0,872 | 0,889 | 0,911 | 0,855 | 0,967 | 0,949 | 0,901 | 0,997 |
| IFI27_ IFIT1_ HERC6_ SLC1A2_ RETN | 0,880 | 0,870 | 0,890 | 0,910 | 0,849 | 0,971 | 0,938 | 0,869 | 1 |
| IFIT1_ HERC6_ IL1R2_ FAM20A_ MMP8 | 0,880 | 0,871 | 0,889 | 0,904 | 0,843 | 0,965 | 0,937 | 0,859 | 1 |
| RSAD2_ IFIT1_ SIGLEC1_ FAM20A_ RETN | 0,880 | 0,871 | 0,890 | 0,902 | 0,833 | 0,971 | 0,947 | 0,886 | 1 |
| RSAD2_ SIGLEC1_ HERC6_ IL1R2_ MMP8 | 0,880 | 0,871 | 0,889 | 0,904 | 0,845 | 0,962 | 0,961 | 0,921 | 1 |
| OAS1_ IFIT1_ SIGLEC1_ SLC1A2_ OLAH | 0,880 | 0,871 | 0,889 | 0,911 | 0,855 | 0,967 | 0,946 | 0,896 | 0,995 |
| IFIT1_ IFI44L_ SIGLEC1_ SLC1A2_ OLAH | 0,880 | 0,871 | 0,889 | 0,913 | 0,857 | 0,969 | 0,943 | 0,892 | 0,995 |
| RSAD2_ OAS1_ SIGLEC1_ OLAH_ FAM20A | 0,880 | 0,870 | 0,890 | 0,899 | 0,828 | 0,970 | 0,959 | 0,911 | 1 |
| RSAD2_ IFIT1_ SIGLEC1_ SLC1A2_ FAM20A | 0,880 | 0,871 | 0,889 | 0,907 | 0,846 | 0,968 | 0,945 | 0,873 | 1 |
| IFIT1_ SIGLEC1_ ISG15_ FAM20A_ RETN | 0,880 | 0,871 | 0,889 | 0,899 | 0,829 | 0,968 | 0,942 | 0,880 | 1 |
| IFI27_ OAS1_ FAM20A_ RETN_ MMP8 | 0,880 | 0,870 | 0,890 | 0,908 | 0,846 | 0,971 | 0,943 | 0,866 | 1 |
| IFI44L_ SLC1A2_ IL1R2_ FAM20A_ MMP8 | 0,880 | 0,872 | 0,888 | 0,910 | 0,858 | 0,962 | 0,928 | 0,840 | 1 |
| IFI44L_ ISG15_ SLC1A2_ OLAH_ FAM20A | 0,880 | 0,870 | 0,889 | 0,909 | 0,849 | 0,968 | 0,927 | 0,853 | 1 |
| OAS1_ SIGLEC1_ HERC6_ IL1R2_ MMP8 | 0,880 | 0,871 | 0,888 | 0,903 | 0,845 | 0,962 | 0,960 | 0,919 | 1 |
| OAS1_ IFIT1_ HERC6_ IL1R2_ FAM20A | 0,880 | 0,871 | 0,889 | 0,904 | 0,842 | 0,967 | 0,936 | 0,855 | 1 |
| OAS1_ SIGLEC1_ HERC6_ SLC1A2_ OLAH | 0,880 | 0,870 | 0,889 | 0,912 | 0,857 | 0,967 | 0,951 | 0,904 | 0,998 |
| IFI27_ OAS1_ SIGLEC1_ HERC6_ MMP8 | 0,880 | 0,871 | 0,888 | 0,906 | 0,850 | 0,962 | 0,917 | 0,845 | 0,990 |
| IFIT1_ SIGLEC1_ HERC6_ IL1R2_ MMP8 | 0,880 | 0,871 | 0,888 | 0,903 | 0,845 | 0,962 | 0,957 | 0,914 | 0,999 |
| RSAD2_ OAS1_ SIGLEC1_ HERC6_ IL1R2 | 0,880 | 0,871 | 0,889 | 0,904 | 0,846 | 0,962 | 0,957 | 0,914 | 0,999 |

(suite)

| Modèle | AUC modèle | IC 95% | | AUC Train | IC 95% Train | | AUC Test | IC 95% Test | |
|---|---|---|---|---|---|---|---|---|---|
| OAS1_ HERC6_ IL1R2_ OLAH _FAM20A | 0,880 | 0,870 | 0,889 | 0,906 | 0,845 | 0,968 | 0,938 | 0,864 | 1 |
| RSAD2_ HERC6_ IL1R2_ OLAH _FAM20A | 0,880 | 0,870 | 0,889 | 0,906 | 0,845 | 0,967 | 0,934 | 0,857 | 1 |
| IFI44L_ SIGLEC1_ ISG15_ OLAH _FAM20A | 0,880 | 0,870 | 0,889 | 0,897 | 0,825 | 0,968 | 0,961 | 0,916 | 1 |
| IFIT1_ SIGLEC1_ ISG15_ FAM20A_ MMP8 | 0,880 | 0,870 | 0,889 | 0,899 | 0,830 | 0,969 | 0,940 | 0,872 | 1 |
| IFI27_ SIGLEC1_ ISG15_ SLC1A2_ RETN | 0,880 | 0,870 | 0,889 | 0,910 | 0,849 | 0,970 | 0,957 | 0,904 | 1 |
| IFI27_ IFIT1_ IFI44L_ HERC6_ FAM20A | 0,879 | 0,870 | 0,889 | 0,899 | 0,833 | 0,964 | 0,927 | 0,845 | 1 |
| RSAD2_ SIGLEC1_ SLC1A2_ IL1R2_ OLAH | 0,879 | 0,870 | 0,888 | 0,912 | 0,856 | 0,967 | 0,961 | 0,921 | 1 |
| IFIT1_ SIGLEC1_ ISG15_ SLC1A2_ OLAH | 0,879 | 0,871 | 0,888 | 0,912 | 0,857 | 0,968 | 0,945 | 0,893 | 0,996 |
| IFIT1_ HERC6_ IL1R2_ OLAH _FAM20A | 0,879 | 0,870 | 0,889 | 0,905 | 0,845 | 0,966 | 0,936 | 0,861 | 1 |
| RSAD2_ SIGLEC1_ HERC6_ FAM20A_ RETN | 0,879 | 0,870 | 0,888 | 0,907 | 0,844 | 0,970 | 0,943 | 0,876 | 1 |
| OAS1_ IFI44L_ HERC6_ IL1R2_ FAM20A | 0,879 | 0,870 | 0,888 | 0,905 | 0,843 | 0,967 | 0,937 | 0,858 | 1 |
| IFIT1_ IFI44L_ SIGLEC1_ HERC6_ IL1R2 | 0,879 | 0,871 | 0,888 | 0,903 | 0,843 | 0,962 | 0,954 | 0,911 | 0,998 |
| IFI44L_ ISG15_ SLC1A2_ IL1R2_ FAM20A | 0,879 | 0,870 | 0,888 | 0,907 | 0,852 | 0,963 | 0,926 | 0,839 | 1 |
| IFI27_ SIGLEC1_ HERC6_ SLC1A2_ RETN | 0,879 | 0,869 | 0,889 | 0,909 | 0,847 | 0,971 | 0,953 | 0,895 | 1 |
| IFIT1_ SIGLEC1_ ISG15_ SLC1A2_ FAM20A | 0,879 | 0,870 | 0,888 | 0,907 | 0,846 | 0,967 | 0,938 | 0,865 | 1 |
| RSAD2_ IFI27_ FAM20A_ RETN_ MMP8 | 0,879 | 0,869 | 0,889 | 0,910 | 0,847 | 0,973 | 0,943 | 0,865 | 1 |
| OAS1_ IFI44L_ SIGLEC1_ HERC6_ IL1R2 | 0,879 | 0,870 | 0,888 | 0,903 | 0,843 | 0,963 | 0,955 | 0,912 | 0,999 |
| IFIT1_ SIGLEC1_ SLC1A2_ FAM20A_ MMP8 | 0,879 | 0,870 | 0,888 | 0,905 | 0,844 | 0,967 | 0,940 | 0,867 | 1 |
| IFIT1_ SIGLEC1_ HERC6_ IL1R2_ RETN | 0,879 | 0,870 | 0,888 | 0,904 | 0,841 | 0,967 | 0,952 | 0,907 | 0,997 |
| SIGLEC1_ ISG15_ SLC1A2_ IL1R2_ OLAH | 0,879 | 0,870 | 0,887 | 0,913 | 0,858 | 0,968 | 0,962 | 0,924 | 1 |
| IFIT1_ SIGLEC1_ SLC1A2_ IL1R2_ OLAH | 0,879 | 0,870 | 0,888 | 0,914 | 0,859 | 0,969 | 0,965 | 0,929 | 1 |
| RSAD2_ IFIT1_ SIGLEC1_ FAM20A_ MMP8 | 0,879 | 0,869 | 0,888 | 0,900 | 0,830 | 0,970 | 0,943 | 0,877 | 1 |
| RSAD2_ SIGLEC1_ ISG15_ HERC6_ FAM20A | 0,879 | 0,870 | 0,888 | 0,902 | 0,838 | 0,967 | 0,943 | 0,875 | 1 |
| IFI44L_ SIGLEC1_ HERC6_ FAM20A_ RETN | 0,879 | 0,869 | 0,889 | 0,903 | 0,836 | 0,970 | 0,954 | 0,888 | 1 |
| RSAD2_ SIGLEC1_ HERC6_ SLC1A2_ OLAH | 0,879 | 0,869 | 0,888 | 0,912 | 0,856 | 0,967 | 0,947 | 0,898 | 0,995 |

| Modèle | AUC modèle | IC 95% | | AUC Train | IC 95% Train | | AUC Test | IC 95% Test | |
|---|---|---|---|---|---|---|---|---|---|
| RSAD2_ IFIT1_ SIGLEC1_ SLC1A2_ OLAH | 0,879 | 0,870 | 0,888 | 0,913 | 0,857 | 0,969 | 0,948 | 0,899 | 0,997 |
| IFI44L_ HERC6_ IL1R2_ FAM20A_ RETN | 0,879 | 0,870 | 0,888 | 0,907 | 0,845 | 0,968 | 0,937 | 0,857 | 1 |
| IFI44L_ SIGLEC1_ SLC1A2_ OLAH_ RETN | 0,879 | 0,870 | 0,888 | 0,914 | 0,857 | 0,970 | 0,950 | 0,901 | 0,999 |
| RSAD2_ HERC6_ IL1R2_ FAM20A_ MMP8 | 0,879 | 0,870 | 0,888 | 0,904 | 0,844 | 0,965 | 0,928 | 0,842 | 1 |
| IFI44L_ SIGLEC1_ ISG15_ HERC6_ IL1R2 | 0,879 | 0,870 | 0,887 | 0,902 | 0,842 | 0,962 | 0,955 | 0,912 | 0,999 |
| SIGLEC1_ HERC6_ OLAH_ FAM20A_ RETN | 0,879 | 0,869 | 0,889 | 0,902 | 0,834 | 0,970 | 0,960 | 0,905 | 1 |
| RSAD2_ OAS1_ SIGLEC1_ SLC1A2_ OLAH | 0,879 | 0,869 | 0,888 | 0,911 | 0,855 | 0,967 | 0,950 | 0,901 | 0,999 |
| OAS1_ IL1R2_ OLAH_ FAM20A_ MMP8 | 0,879 | 0,870 | 0,888 | 0,905 | 0,843 | 0,967 | 0,937 | 0,859 | 1 |
| IFIT1_ SIGLEC1_ ISG15_ HERC6_ IL1R2 | 0,879 | 0,870 | 0,887 | 0,905 | 0,846 | 0,963 | 0,952 | 0,907 | 0,998 |
| RSAD2_ IFI27_ ISG15_ HERC6_ FAM20A | 0,879 | 0,869 | 0,888 | 0,908 | 0,848 | 0,968 | 0,925 | 0,841 | 1 |
| IFI27_ SIGLEC1_ ISG15_ RETN_ MMP8 | 0,879 | 0,869 | 0,889 | 0,904 | 0,839 | 0,969 | 0,958 | 0,905 | 1 |
| OAS1_ SIGLEC1_ HERC6_ FAM20A_ RETN | 0,878 | 0,869 | 0,888 | 0,902 | 0,838 | 0,967 | 0,948 | 0,882 | 1 |
| SIGLEC1_ HERC6_ SLC1A2_ OLAH_ MMP8 | 0,878 | 0,869 | 0,888 | 0,911 | 0,855 | 0,967 | 0,955 | 0,913 | 0,998 |
| OAS1_ IFIT1_ IL1R2_ FAM20A_ MMP8 | 0,878 | 0,870 | 0,887 | 0,905 | 0,843 | 0,968 | 0,943 | 0,870 | 1 |
| IFI27_ OAS1_ ISG15_ HERC6_ FAM20A | 0,878 | 0,868 | 0,889 | 0,905 | 0,844 | 0,966 | 0,926 | 0,843 | 1 |
| IFI27_ IFI44L_ HERC6_ SLC1A2_ RETN | 0,878 | 0,869 | 0,888 | 0,910 | 0,849 | 0,970 | 0,938 | 0,868 | 1 |
| RSAD2_ IFIT1_ SIGLEC1_ HERC6_ IL1R2 | 0,878 | 0,870 | 0,887 | 0,903 | 0,844 | 0,962 | 0,952 | 0,907 | 0,997 |
| IFI44L_ SIGLEC1_ HERC6_ IL1R2_ OLAH | 0,878 | 0,870 | 0,887 | 0,904 | 0,843 | 0,964 | 0,959 | 0,917 | 1 |
| HERC6_ IL1R2_ OLAH_ FAM20A_ RETN | 0,878 | 0,869 | 0,888 | 0,904 | 0,843 | 0,966 | 0,932 | 0,855 | 1 |
| RSAD2_ IFI44L_ HERC6_ IL1R2_ FAM20A | 0,878 | 0,869 | 0,887 | 0,907 | 0,847 | 0,967 | 0,935 | 0,855 | 1 |
| OAS1_ SIGLEC1_ SLC1A2_ IL1R2_ OLAH | 0,878 | 0,869 | 0,887 | 0,913 | 0,858 | 0,968 | 0,963 | 0,925 | 1 |
| IFI27_ ISG15_ HERC6_ SLC1A2_ RETN | 0,878 | 0,869 | 0,888 | 0,910 | 0,848 | 0,971 | 0,943 | 0,878 | 1 |
| SIGLEC1_ ISG15_ HERC6_ FAM20A_ RETN | 0,878 | 0,868 | 0,888 | 0,897 | 0,827 | 0,968 | 0,955 | 0,890 | 1 |
| SIGLEC1_ ISG15_ FAM20A_ RETN_ MMP8 | 0,878 | 0,868 | 0,888 | 0,900 | 0,829 | 0,970 | 0,958 | 0,896 | 1 |
| IFIT1_ IFI44L_ SLC1A2_ OLAH_ FAM20A | 0,878 | 0,869 | 0,887 | 0,909 | 0,850 | 0,968 | 0,920 | 0,843 | 0,997 |

| Modèle | AUC modèle | IC 95% | | AUC Train | IC 95% Train | | AUC Test | IC 95% Test | |
|---|---|---|---|---|---|---|---|---|---|
| RSAD2_ SIGLEC1_ HERC6_ SLC1A2_ FAM20A | 0,878 | 0,868 | 0,888 | 0,909 | 0,852 | 0,966 | 0,941 | 0,866 | 1 |
| RSAD2_ IFI44L_ SIGLEC1_ HERC6_ IL1R2 | 0,878 | 0,869 | 0,887 | 0,900 | 0,837 | 0,962 | 0,959 | 0,918 | 0,999 |
| OAS1_ IFIT1_ SIGLEC1_ HERC6_ RETN | 0,878 | 0,869 | 0,887 | 0,897 | 0,829 | 0,964 | 0,921 | 0,858 | 0,983 |
| SIGLEC1_ SLC1A2_ IL1R2_ OLAH_ RETN | 0,878 | 0,869 | 0,887 | 0,913 | 0,857 | 0,968 | 0,961 | 0,920 | 1 |
| OAS1_ IFI44L_ SIGLEC1_ OLAH_ FAM20A | 0,878 | 0,868 | 0,888 | 0,896 | 0,824 | 0,968 | 0,963 | 0,920 | 1 |
| IFI44L_ HERC6_ SLC1A2_ IL1R2_ FAM20A | 0,878 | 0,868 | 0,887 | 0,912 | 0,860 | 0,965 | 0,925 | 0,836 | 1 |
| IFI27_ IFIT1_ ISG15_ HERC6_ FAM20A | 0,878 | 0,868 | 0,888 | 0,904 | 0,842 | 0,966 | 0,929 | 0,846 | 1 |
| IFIT1_ SIGLEC1_ SLC1A2_ OLAH_ RETN | 0,878 | 0,869 | 0,887 | 0,915 | 0,860 | 0,970 | 0,948 | 0,897 | 0,999 |
| IFIT1_ HERC6_ IL1R2_ FAM20A_ RETN | 0,878 | 0,868 | 0,887 | 0,904 | 0,842 | 0,966 | 0,938 | 0,857 | 1 |
| IFI44L_ SLC1A2_ OLAH_ FAM20A_ RETN | 0,878 | 0,868 | 0,887 | 0,908 | 0,848 | 0,968 | 0,927 | 0,853 | 1 |
| IFI44L_ HERC6_ SLC1A2_ OLAH_ FAM20A | 0,878 | 0,868 | 0,888 | 0,912 | 0,855 | 0,969 | 0,932 | 0,853 | 1 |
| RSAD2_ SIGLEC1_ ISG15_ HERC6_ IL1R2 | 0,878 | 0,869 | 0,886 | 0,903 | 0,843 | 0,962 | 0,951 | 0,905 | 0,997 |
| IFI44L_ SIGLEC1_ FAM20A_ RETN_ MMP8 | 0,878 | 0,868 | 0,888 | 0,902 | 0,833 | 0,971 | 0,959 | 0,897 | 1 |
| SIGLEC1_ HERC6_ IL1R2_ OLAH_ MMP8 | 0,878 | 0,869 | 0,887 | 0,905 | 0,846 | 0,964 | 0,966 | 0,931 | 1 |
| RSAD2_ IFIT1_ HERC6_ IL1R2_ FAM20A | 0,878 | 0,868 | 0,887 | 0,904 | 0,842 | 0,966 | 0,935 | 0,853 | 1 |
| OAS1_ SIGLEC1_ HERC6_ IL1R2_ RETN | 0,878 | 0,868 | 0,887 | 0,901 | 0,837 | 0,965 | 0,957 | 0,913 | 1,000 |
| SIGLEC1_ HERC6_ SLC1A2_ IL1R2_ OLAH | 0,878 | 0,868 | 0,887 | 0,912 | 0,856 | 0,967 | 0,959 | 0,917 | 1 |
| SIGLEC1_ ISG15_ HERC6_ SLC1A2_ OLAH | 0,878 | 0,868 | 0,887 | 0,910 | 0,853 | 0,966 | 0,950 | 0,903 | 0,997 |
| OAS1_ SIGLEC1_ ISG15_ SLC1A2_ OLAH | 0,878 | 0,868 | 0,887 | 0,912 | 0,856 | 0,968 | 0,948 | 0,899 | 0,997 |
| RSAD2_ OAS1_ SIGLEC1_ HERC6_ FAM20A | 0,878 | 0,868 | 0,887 | 0,898 | 0,832 | 0,964 | 0,941 | 0,871 | 1 |
| IFI44L_ SIGLEC1_ OLAH_ FAM20A_ RETN | 0,877 | 0,868 | 0,887 | 0,896 | 0,823 | 0,968 | 0,962 | 0,918 | 1 |
| IFIT1_ SIGLEC1_ HERC6_ IL1R2_ OLAH | 0,877 | 0,869 | 0,886 | 0,907 | 0,848 | 0,965 | 0,950 | 0,904 | 0,996 |
| IFI44L_ SIGLEC1_ IL1R2_ OLAH_ MMP8 | 0,877 | 0,869 | 0,886 | 0,905 | 0,848 | 0,962 | 0,957 | 0,914 | 0,999 |
| OAS1_ IFI44L_ SIGLEC1_ SLC1A2_ OLAH | 0,877 | 0,868 | 0,887 | 0,914 | 0,859 | 0,969 | 0,949 | 0,900 | 0,998 |
| IFIT1_ SIGLEC1_ SLC1A2_ FAM20A_ RETN | 0,877 | 0,868 | 0,887 | 0,909 | 0,849 | 0,968 | 0,939 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1_IFI44L_HERC6_OLAH_FAM20A | 0,877 | 0,868 | 0,887 | 0,902 | 0,838 | 0,966 | 0,938 | 0,871 | 1 |
| SIGLEC1_ISG15_HERC6_IL1R2_MMP8 | 0,877 | 0,869 | 0,886 | 0,903 | 0,844 | 0,963 | 0,964 | 0,927 | 1 |
| OAS1_HERC6_IL1R2_FAM20A_RETN | 0,877 | 0,868 | 0,887 | 0,904 | 0,841 | 0,967 | 0,935 | 0,853 | 1 |
| OAS1_SIGLEC1_HERC6_SLC1A2_IL1R2 | 0,877 | 0,868 | 0,886 | 0,910 | 0,857 | 0,964 | 0,959 | 0,918 | 1,000 |
| IFIT1_SIGLEC1_HERC6_SLC1A2_IL1R2 | 0,877 | 0,869 | 0,886 | 0,908 | 0,854 | 0,963 | 0,961 | 0,922 | 1 |
| RSAD2_IFI27_IFIT1_HERC6_FAM20A | 0,877 | 0,867 | 0,887 | 0,907 | 0,846 | 0,967 | 0,934 | 0,853 | 1 |
| OAS1_SIGLEC1_ISG15_OLAH_FAM20A | 0,877 | 0,867 | 0,887 | 0,896 | 0,825 | 0,968 | 0,962 | 0,917 | 1 |
| IFI27_OAS1_SLC1A2_FAM20A_RETN | 0,877 | 0,868 | 0,887 | 0,910 | 0,848 | 0,971 | 0,938 | 0,856 | 1 |
| RSAD2_OAS1_IL1R2_OLAH_FAM20A | 0,877 | 0,868 | 0,886 | 0,901 | 0,835 | 0,967 | 0,940 | 0,866 | 1 |
| IFI44L_SIGLEC1_HERC6_IL1R2_RETN | 0,877 | 0,868 | 0,886 | 0,901 | 0,838 | 0,965 | 0,959 | 0,918 | 1,000 |
| IFI27_OAS1_IFIT1_ISG15_RETN | 0,877 | 0,868 | 0,887 | 0,904 | 0,841 | 0,968 | 0,950 | 0,887 | 1 |
| RSAD2_IFI27_SIGLEC1_HERC6_MMP8 | 0,877 | 0,868 | 0,886 | 0,901 | 0,842 | 0,959 | 0,909 | 0,834 | 0,983 |
| ISG15_HERC6_SLC1A2_IL1R2_FAM20A | 0,877 | 0,867 | 0,887 | 0,912 | 0,859 | 0,964 | 0,922 | 0,829 | 1 |
| SIGLEC1_SLC1A2_FAM20A_RETN_MMP8 | 0,877 | 0,867 | 0,887 | 0,903 | 0,839 | 0,967 | 0,953 | 0,882 | 1 |
| RSAD2_SIGLEC1_HERC6_IL1R2_OLAH | 0,877 | 0,868 | 0,886 | 0,906 | 0,847 | 0,965 | 0,955 | 0,912 | 0,999 |
| IFI27_IFI44L_ISG15_HERC6_FAM20A | 0,877 | 0,867 | 0,887 | 0,900 | 0,835 | 0,965 | 0,937 | 0,857 | 1 |
| IFI44L_IL1R2_OLAH_FAM20A_MMP8 | 0,877 | 0,868 | 0,886 | 0,903 | 0,842 | 0,964 | 0,947 | 0,883 | 1 |
| IFI27_SIGLEC1_SLC1A2_RETN_MMP8 | 0,877 | 0,867 | 0,887 | 0,910 | 0,849 | 0,970 | 0,955 | 0,901 | 1 |
| HERC6_SLC1A2_IL1R2_OLAH_FAM20A | 0,877 | 0,868 | 0,886 | 0,914 | 0,861 | 0,967 | 0,922 | 0,835 | 1 |
| OAS1_SIGLEC1_SLC1A2_OLAH_RETN | 0,877 | 0,867 | 0,887 | 0,913 | 0,857 | 0,969 | 0,949 | 0,898 | 0,999 |
| RSAD2_SIGLEC1_HERC6_SLC1A2_IL1R2 | 0,877 | 0,868 | 0,886 | 0,909 | 0,855 | 0,964 | 0,960 | 0,919 | 1 |
| IFI27_IFIT1_SLC1A2_FAM20A_RETN | 0,877 | 0,867 | 0,887 | 0,908 | 0,847 | 0,969 | 0,939 | 0,858 | 1 |
| IFI44L_SIGLEC1_SLC1A2_IL1R2_MMP8 | 0,877 | 0,869 | 0,885 | 0,909 | 0,855 | 0,963 | 0,966 | 0,930 | 1 |
| IFI27_OAS1_IFI44L_HERC6_FAM20A | 0,877 | 0,867 | 0,887 | 0,902 | 0,837 | 0,966 | 0,934 | 0,853 | 1 |
| RSAD2_SIGLEC1_ISG15_OLAH_FAM20A | 0,877 | 0,867 | 0,887 | 0,896 | 0,825 | 0,968 | 0,961 | 0,917 | 1 |

(suite)

| Modèle | AUC modèle | IC 95% | | AUC Train | IC 95% Train | | AUC Test | IC 95% Test | |
|---|---|---|---|---|---|---|---|---|---|
| SIGLEC1_ISG15_OLAH_FAM20A_RETN | 0,877 | 0,867 | 0,887 | 0,896 | 0,825 | 0,968 | 0,962 | 0,919 | 1 |
| SIGLEC1_ISG15_SLC1A2_OLAH_RETN | 0,877 | 0,868 | 0,886 | 0,914 | 0,858 | 0,970 | 0,950 | 0,901 | 0,999 |
| RSAD2_IFI27_OAS1_HERC6_FAM20A | 0,877 | 0,867 | 0,887 | 0,904 | 0,843 | 0,965 | 0,929 | 0,846 | 1 |
| SIGLEC1_HERC6_IL1R2_RETN_MMP8 | 0,877 | 0,867 | 0,886 | 0,901 | 0,835 | 0,966 | 0,970 | 0,937 | 1 |
| RSAD2_SIGLEC1_SLC1A2_OLAH_RETN | 0,877 | 0,868 | 0,886 | 0,913 | 0,856 | 0,969 | 0,950 | 0,901 | 0,999 |
| RSAD2_SIGLEC1_ISG15_SLC1A2_OLAH | 0,877 | 0,868 | 0,886 | 0,912 | 0,856 | 0,968 | 0,950 | 0,903 | 0,997 |
| IFI44L_SIGLEC1_HERC6_SLC1A2_OLAH | 0,877 | 0,867 | 0,886 | 0,912 | 0,856 | 0,967 | 0,953 | 0,909 | 0,998 |
| IFI27_IFI44L_SIGLEC1_HERC6_MMP8 | 0,877 | 0,868 | 0,886 | 0,897 | 0,836 | 0,959 | 0,908 | 0,833 | 0,982 |
| OAS1_SIGLEC1_HERC6_IL1R2_OLAH | 0,877 | 0,868 | 0,886 | 0,907 | 0,849 | 0,965 | 0,958 | 0,915 | 1,000 |
| RSAD2_SIGLEC1_HERC6_IL1R2_RETN | 0,877 | 0,867 | 0,886 | 0,901 | 0,836 | 0,965 | 0,954 | 0,911 | 0,998 |
| OAS1_SIGLEC1_FAM20A_RETN_MMP8 | 0,877 | 0,867 | 0,887 | 0,901 | 0,832 | 0,971 | 0,958 | 0,898 | 1 |
| IFI44L_ISG15_IL1R2_OLAH_FAM20A | 0,877 | 0,868 | 0,886 | 0,901 | 0,837 | 0,965 | 0,953 | 0,898 | 1 |
| OAS1_SIGLEC1_ISG15_HERC6_IL1R2 | 0,877 | 0,868 | 0,887 | 0,904 | 0,846 | 0,961 | 0,959 | 0,917 | 1 |
| IFI27_ISG15_SLC1A2_FAM20A_RETN | 0,877 | 0,867 | 0,886 | 0,904 | 0,841 | 0,968 | 0,926 | 0,839 | 1 |
| IFIT1_SIGLEC1_HERC6_OLAH_RETN | 0,877 | 0,867 | 0,886 | 0,900 | 0,835 | 0,965 | 0,938 | 0,885 | 0,991 |
| RSAD2_HERC6_IL1R2_FAM20A_RETN | 0,877 | 0,868 | 0,886 | 0,905 | 0,842 | 0,967 | 0,932 | 0,848 | 1 |
| HERC6_IL1R2_FAM20A_RETN_MMP8 | 0,877 | 0,867 | 0,886 | 0,905 | 0,844 | 0,967 | 0,925 | 0,836 | 1 |
| RSAD2_IFI27_SLC1A2_FAM20A_RETN | 0,877 | 0,867 | 0,886 | 0,910 | 0,848 | 0,971 | 0,937 | 0,855 | 1 |
| IFIT1_IL1R2_OLAH_FAM20A_MMP8 | 0,876 | 0,867 | 0,886 | 0,905 | 0,844 | 0,967 | 0,949 | 0,883 | 1 |
| IFI44L_SIGLEC1_SLC1A2_IL1R2_RETN | 0,876 | 0,868 | 0,885 | 0,910 | 0,855 | 0,965 | 0,962 | 0,922 | 1 |
| RSAD2_SLC1A2_IL1R2_OLAH_FAM20A | 0,876 | 0,868 | 0,885 | 0,911 | 0,855 | 0,968 | 0,932 | 0,851 | 1 |
| RSAD2_SIGLEC1_HERC6_FAM20A_MMP8 | 0,876 | 0,867 | 0,886 | 0,898 | 0,832 | 0,964 | 0,942 | 0,873 | 1 |
| OAS1_SIGLEC1_OLAH_FAM20A_RETN | 0,876 | 0,866 | 0,886 | 0,896 | 0,824 | 0,968 | 0,962 | 0,918 | 1 |
| SIGLEC1_HERC6_SLC1A2_IL1R2_MMP8 | 0,876 | 0,868 | 0,885 | 0,907 | 0,852 | 0,962 | 0,963 | 0,925 | 1 |
| OAS1_IFIT1_IL1R2_OLAH_FAM20A | 0,876 | 0,867 | 0,885 | 0,901 | 0,835 | 0,967 | 0,945 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_ HERC6_ SLC1A2_ RETN_ MMP8 | 0,876 | 0,866 | 0,886 | 0,908 | 0,847 | 0,970 | 0,935 | 0,864 | 1 |
| IFI44L_ SIGLEC1_ ISG15_ IL1R2_ MMP8 | 0,876 | 0,868 | 0,884 | 0,904 | 0,846 | 0,961 | 0,957 | 0,914 | 0,999 |
| IFI44L_ SIGLEC1_ ISG15_ SLC1A2_ IL1R2 | 0,876 | 0,868 | 0,884 | 0,907 | 0,851 | 0,963 | 0,963 | 0,925 | 1 |
| IFIT1_ SLC1A2_ IL1R2_ FAM20A_ MMP8 | 0,876 | 0,867 | 0,885 | 0,909 | 0,854 | 0,964 | 0,936 | 0,857 | 1 |
| RSAD2_ SIGLEC1_ FAM20A_ RETN_ MMP8 | 0,876 | 0,866 | 0,886 | 0,902 | 0,835 | 0,970 | 0,957 | 0,896 | 1 |
| IFIT1_ HERC6_ SLC1A2_ IL1R2_ FAM20A | 0,876 | 0,866 | 0,885 | 0,912 | 0,858 | 0,966 | 0,932 | 0,848 | 1 |
| RSAD2_ SIGLEC1_ SLC1A2_ IL1R2_ MMP8 | 0,876 | 0,867 | 0,884 | 0,909 | 0,854 | 0,963 | 0,967 | 0,933 | 1 |
| RSAD2_ IFI44L_SIGLEC1_OLAH_ FAM20A | 0,876 | 0,866 | 0,886 | 0,897 | 0,826 | 0,968 | 0,963 | 0,921 | 1 |
| OAS1_ISG15_IL1R2_OLAH_FAM20A | 0,876 | 0,867 | 0,885 | 0,901 | 0,835 | 0,967 | 0,940 | 0,866 | 1 |
| IFI27_IFIT1_IFI44L_FAM20A_MMP8 | 0,876 | 0,866 | 0,886 | 0,899 | 0,832 | 0,966 | 0,934 | 0,854 | 1 |
| SIGLEC1_SLC1A2_OLAH_RETN_MMP8 | 0,876 | 0,866 | 0,885 | 0,909 | 0,851 | 0,967 | 0,962 | 0,923 | 1 |
| RSAD2_IFI44L_SLC1A2_OLAH_FAM20A | 0,876 | 0,866 | 0,885 | 0,908 | 0,849 | 0,968 | 0,926 | 0,852 | 1 |
| OAS1_HERC6_SLC1A2_IL1R2_FAM20A | 0,876 | 0,867 | 0,885 | 0,911 | 0,857 | 0,965 | 0,923 | 0,832 | 1 |
| RSAD2_HERC6_SLC1A2_IL1R2_FAM20A | 0,876 | 0,867 | 0,885 | 0,911 | 0,857 | 0,964 | 0,927 | 0,837 | 1 |
| OAS1_IFIT1_SIGLEC1_IL1R2_OLAH | 0,876 | 0,867 | 0,885 | 0,901 | 0,838 | 0,965 | 0,957 | 0,914 | 0,999 |
| OAS1_SLC1A2_IL1R2_OLAH_FAM20A | 0,876 | 0,867 | 0,885 | 0,911 | 0,854 | 0,968 | 0,927 | 0,844 | 1 |
| IFI27_OAS1_IFIT1_HERC6_FAM20A | 0,876 | 0,866 | 0,886 | 0,903 | 0,841 | 0,965 | 0,935 | 0,854 | 1 |
| IFIT1_SIGLEC1_HERC6_OLAH_MMP8 | 0,876 | 0,866 | 0,885 | 0,899 | 0,837 | 0,961 | 0,936 | 0,881 | 0,991 |
| OAS1_SIGLEC1_HERC6_ SLC1A2_ FAM20A | 0,876 | 0,866 | 0,885 | 0,908 | 0,852 | 0,965 | 0,943 | 0,870 | 1 |
| OAS1_HERC6_SLC1A2_OLAH_FAM20A | 0,876 | 0,866 | 0,885 | 0,910 | 0,854 | 0,966 | 0,928 | 0,846 | 1 |
| IFIT1_SIGLEC1_ISG15_SLC1A2_IL1R2 | 0,876 | 0,868 | 0,884 | 0,908 | 0,853 | 0,964 | 0,964 | 0,927 | 1 |
| ISG15_SLC1A2_IL1R2_OLAH_FAM20A | 0,876 | 0,866 | 0,885 | 0,908 | 0,850 | 0,965 | 0,924 | 0,844 | 1 |
| IFI44L_ SIGLEC1_SLC1A2_FAM20A_ MMP8 | 0,876 | 0,866 | 0,885 | 0,901 | 0,837 | 0,966 | 0,952 | 0,880 | 1 |
| RSAD2_IFI44L_SIGLEC1_SLC1A2_OLAH | 0,876 | 0,866 | 0,885 | 0,912 | 0,857 | 0,968 | 0,949 | 0,901 | 0,997 |
| IFI44L_SIGLEC1_ISG15_FAM20A_RETN | 0,876 | 0,866 | 0,885 | 0,894 | 0,823 | 0,966 | 0,955 | 0,898 | 1 |

EP 4 365 308 A1

114

| Modèle | AUC modèle | IC 95% | | AUC Train | IC 95% Train | | AUC Test | IC 95% Test | |
|---|---|---|---|---|---|---|---|---|---|
| SIGLEC1_HERC6_SLC1A2_FAM20A_ RETN | 0,875 | 0,865 | 0,886 | 0,903 | 0,839 | 0,966 | 0,950 | 0,876 | 1 |
| IFI44L_SIGLEC1_HERC6_SLC1A2_ FAM20A | 0,875 | 0,866 | 0,885 | 0,904 | 0,842 | 0,966 | 0,953 | 0,880 | 1 |
| SIGLEC1_ISG15_HERC6_IL1R2_OLAH | 0,875 | 0,866 | 0,885 | 0,903 | 0,842 | 0,964 | 0,959 | 0,918 | 1,000 |
| SIGLEC1_ISG15_HERC6_SLC1A2_IL1R2 | 0,875 | 0,867 | 0,884 | 0,908 | 0,853 | 0,963 | 0,962 | 0,923 | 1 |
| OAS1_IFI44L_SLC1A2_OLAH_FAM20A | 0,875 | 0,866 | 0,885 | 0,909 | 0,849 | 0,969 | 0,927 | 0,852 | 1 |
| RSAD2_ HERC6_ SLC1A2_OLAH_ FAM20A | 0,875 | 0,865 | 0,885 | 0,910 | 0,854 | 0,966 | 0,929 | 0,848 | 1 |
| IFIT1_ISG15_IL1R2_FAM20A_MMP8 | 0,875 | 0,866 | 0,885 | 0,907 | 0,846 | 0,969 | 0,941 | 0,867 | 1 |
| ISG15_HERC6_SLC1A2_OLAH_FAM20A | 0,875 | 0,866 | 0,885 | 0,911 | 0,855 | 0,967 | 0,932 | 0,852 | 1 |
| IFIT1_SIGLEC1_IL1R2_OLAH_MMP8 | 0,875 | 0,867 | 0,884 | 0,905 | 0,848 | 0,962 | 0,963 | 0,925 | 1 |
| SIGLEC1_ISG15_SLC1A2_IL1R2_MMP8 | 0,875 | 0,868 | 0,883 | 0,910 | 0,856 | 0,964 | 0,966 | 0,930 | 1 |
| IFIT1_SIGLEC1_SLC1A2_IL1R2_MMP8 | 0,875 | 0,867 | 0,884 | 0,910 | 0,855 | 0,964 | 0,966 | 0,930 | 1 |
| RSAD2_IFI27_OAS1_IFI44L_RETN | 0,875 | 0,866 | 0,885 | 0,905 | 0,840 | 0,969 | 0,953 | 0,892 | 1 |
| IFI27_OAS1_IFI44L_ISG15_RETN | 0,875 | 0,866 | 0,885 | 0,906 | 0,843 | 0,969 | 0,954 | 0,896 | 1 |
| IFI44L_ISG15_ IL1R2_ FAM20A_MMP8 | 0,875 | 0,867 | 0,884 | 0,904 | 0,844 | 0,964 | 0,937 | 0,858 | 1 |
| IFI27_IFI44L_ISG15_FAM20A_MMP8 | 0,875 | 0,865 | 0,885 | 0,898 | 0,831 | 0,965 | 0,938 | 0,860 | 1 |
| RSAD2_ OAS1_ IL1R2_FAM20A_MMP8 | 0,875 | 0,866 | 0,884 | 0,903 | 0,840 | 0,965 | 0,934 | 0,853 | 1 |
| IFIT1_ISG15_SLC1A2_IL1R2_FAM20A | 0,875 | 0,866 | 0,884 | 0,904 | 0,846 | 0,963 | 0,935 | 0,857 | 1 |
| RSAD2_OAS1_SIGLEC1_SLC1A2_ FAM20A | 0,875 | 0,866 | 0,885 | 0,903 | 0,840 | 0,966 | 0,947 | 0,874 | 1 |
| RSAD2_OAS1_SIGLEC1_FAM20A_RETN | 0,875 | 0,866 | 0,885 | 0,895 | 0,824 | 0,966 | 0,955 | 0,897 | 1 |
| SIGLEC1_ISG15_IL1R2_OLAH_MMP8 | 0,875 | 0,867 | 0,884 | 0,905 | 0,847 | 0,963 | 0,963 | 0,925 | 1 |
| RSAD2_IFI27_OAS1_IFI44L_FAM20A | 0,875 | 0,865 | 0,885 | 0,901 | 0,835 | 0,968 | 0,938 | 0,862 | 1 |
| RSAD2_SIGLEC1_SLC1A2_FAM20A_ MMP8 | 0,875 | 0,866 | 0,885 | 0,902 | 0,839 | 0,965 | 0,949 | 0,878 | 1 |
| IFI44L_SIGLEC1_IL1R2_RETN_MMP8 | 0,875 | 0,866 | 0,885 | 0,902 | 0,839 | 0,964 | 0,962 | 0,924 | 1 |
| OAS1_IFIT1_SIGLEC1_HERC6_OLAH | 0,875 | 0,866 | 0,884 | 0,900 | 0,839 | 0,962 | 0,937 | 0,883 | 0,991 |
| IFI44L_SIGLEC1_ISG15_IL1R2_OLAH | 0,875 | 0,867 | 0,884 | 0,902 | 0,841 | 0,963 | 0,958 | 0,915 | 1 |

| Modèle | AUC modèle | IC 95% | | AUC Train | IC 95% Train | | AUC Test | IC 95% Test | |
|---|---|---|---|---|---|---|---|---|---|
| OAS1_IFI44L_SIGLEC1_IL1R2_MMP8 | 0,875 | 0,867 | 0,884 | 0,902 | 0,842 | 0,962 | 0,957 | 0,914 | 0,999 |
| SIGLEC1_ISG15_SLC1A2_FAM20A_ MMP8 | 0,875 | 0,865 | 0,885 | 0,900 | 0,836 | 0,964 | 0,952 | 0,880 | 1 |
| HERC6_SLC1A2_OLAH_FAM20A_MMP8 | 0,875 | 0,866 | 0,885 | 0,911 | 0,856 | 0,966 | 0,928 | 0,846 | 1 |
| IFI27_OAS1_ISG15_HERC6_MMP8 | 0,875 | 0,867 | 0,884 | 0,901 | 0,844 | 0,958 | 0,900 | 0,822 | 0,978 |
| OAS1_IFIT1_IFI44L_SIGLEC1_IL1R2 | 0,875 | 0,866 | 0,884 | 0,899 | 0,835 | 0,962 | 0,955 | 0,913 | 0,998 |
| RSAD2_IFI27_OAS1_IFIT1_RETN | 0,875 | 0,866 | 0,885 | 0,905 | 0,841 | 0,968 | 0,949 | 0,885 | 1 |
| OAS1_IFIT1_SIGLEC1_IL1R2_MMP8 | 0,875 | 0,866 | 0,884 | 0,901 | 0,842 | 0,961 | 0,963 | 0,925 | 1 |
| RSAD2_SIGLEC1_IL1R2_OLAH_MMP8 | 0,875 | 0,866 | 0,884 | 0,905 | 0,847 | 0,963 | 0,963 | 0,925 | 1 |
| SIGLEC1_ISG15_SLC1A2_IL1R2_RETN | 0,875 | 0,867 | 0,883 | 0,909 | 0,854 | 0,964 | 0,965 | 0,927 | 1 |
| OAS1_IFIT1_SIGLEC1_SLC1A2_IL1R2 | 0,875 | 0,866 | 0,884 | 0,908 | 0,853 | 0,963 | 0,963 | 0,925 | 1 |
| SIGLEC1_ISG15_SLC1A2_FAM20A_ RETN | 0,875 | 0,865 | 0,885 | 0,902 | 0,839 | 0,966 | 0,948 | 0,875 | 1 |
| IFI44L_SIGLEC1_ISG15_SLC1A2_ FAM20A | 0,875 | 0,866 | 0,884 | 0,901 | 0,837 | 0,965 | 0,949 | 0,877 | 1 |
| RSAD2_IFI27_IFI44L_ISG15_FAM20A | 0,875 | 0,865 | 0,885 | 0,903 | 0,836 | 0,970 | 0,943 | 0,870 | 1 |
| IFIT1_IFI44L_SIGLEC1_IL1R2_MMP8 | 0,875 | 0,866 | 0,884 | 0,901 | 0,842 | 0,961 | 0,960 | 0,919 | 1 |
| IFI27_OAS1_IFIT1_IFI44L_RETN | 0,875 | 0,865 | 0,885 | 0,906 | 0,842 | 0,969 | 0,948 | 0,882 | 1 |
| IFIT1_SIGLEC1_SLC1A2_IL1R2_RETN | 0,875 | 0,866 | 0,884 | 0,910 | 0,855 | 0,965 | 0,962 | 0,922 | 1 |
| RSAD2_SIGLEC1_OLAH_FAM20A_RETN | 0,875 | 0,865 | 0,885 | 0,897 | 0,825 | 0,969 | 0,961 | 0,917 | 1 |
| IFI27_IFI44L_ISG15_RETN_MMP8 | 0,875 | 0,865 | 0,885 | 0,904 | 0,841 | 0,967 | 0,953 | 0,896 | 1 |
| RSAD2_OAS1_SIGLEC1_FAM20A_MMP8 | 0,875 | 0,865 | 0,885 | 0,895 | 0,824 | 0,966 | 0,950 | 0,885 | 1 |
| RSAD2_IFI44L_SIGLEC1_IL1R2_MMP8 | 0,875 | 0,866 | 0,883 | 0,900 | 0,839 | 0,962 | 0,960 | 0,919 | 1 |
| SIGLEC1_HERC6_SLC1A2_IL1R2_RETN | 0,875 | 0,866 | 0,884 | 0,909 | 0,853 | 0,965 | 0,960 | 0,919 | 1 |
| IFI44L_SIGLEC1_ISG15_HERC6_FAM20A | 0,875 | 0,865 | 0,885 | 0,897 | 0,830 | 0,965 | 0,953 | 0,882 | 1 |
| OAS1_SIGLEC1_SLC1A2_IL1R2_MMP8 | 0,875 | 0,866 | 0,883 | 0,910 | 0,857 | 0,964 | 0,966 | 0,930 | 1 |
| RSAD2_IFI44L_SIGLEC1_HERC6_ FAM20A | 0,875 | 0,865 | 0,884 | 0,900 | 0,835 | 0,965 | 0,941 | 0,871 | 1 |
| OAS1_IFIT1_ISG15_IL1R2_FAM20A | 0,875 | 0,865 | 0,884 | 0,900 | 0,835 | 0,965 | 0,942 | 0,868 | 1 |

EP 4 365 308 A1

| Modèle | AUC modèle | IC 95% | | AUC Train | IC 95% Train | | AUC Test | IC 95% Test | |
|---|---|---|---|---|---|---|---|---|---|
| IFI44L_SLC1A2_IL1R2_FAM20A_RETN | 0,875 | 0,866 | 0,884 | 0,909 | 0,855 | 0,963 | 0,926 | 0,840 | 1 |
| IFIT1_HERC6_SLC1A2_OLAH_FAM20A | 0,875 | 0,865 | 0,885 | 0,910 | 0,854 | 0,966 | 0,930 | 0,849 | 1 |
| IFIT1_SIGLEC1_ISG15_HERC6_RETN | 0,875 | 0,865 | 0,884 | 0,896 | 0,828 | 0,963 | 0,935 | 0,880 | 0,989 |
| IFI44L_SIGLEC1_HERC6_SLC1A2_IL1R2 | 0,875 | 0,866 | 0,884 | 0,908 | 0,853 | 0,963 | 0,960 | 0,920 | 1,000 |
| RSAD2_SIGLEC1_SLC1A2_IL1R2_RETN | 0,875 | 0,866 | 0,884 | 0,910 | 0,855 | 0,965 | 0,965 | 0,928 | 1 |
| HERC6_SLC1A2_IL1R2_FAM20A_RETN | 0,875 | 0,865 | 0,884 | 0,912 | 0,858 | 0,966 | 0,918 | 0,824 | 1 |
| RSAD2_OAS1_SIGLEC1_IL1R2_MMP8 | 0,875 | 0,866 | 0,884 | 0,903 | 0,844 | 0,962 | 0,965 | 0,929 | 1 |
| SIGLEC1_HERC6_SLC1A2_OLAH_RETN | 0,875 | 0,865 | 0,885 | 0,910 | 0,851 | 0,968 | 0,958 | 0,915 | 1,000 |
| RSAD2_OAS1_SIGLEC1_ISG15_FAM20A | 0,875 | 0,865 | 0,884 | 0,897 | 0,826 | 0,968 | 0,952 | 0,889 | 1 |
| SIGLEC1_SLC1A2_IL1R2_RETN_MMP8 | 0,875 | 0,866 | 0,883 | 0,912 | 0,858 | 0,966 | 0,968 | 0,935 | 1 |
| IFIT1_IFI44L_SLC1A2_IL1R2_FAM20A | 0,874 | 0,866 | 0,883 | 0,909 | 0,854 | 0,964 | 0,924 | 0,837 | 1 |
| OAS1_SIGLEC1_IL1R2_OLAH_MMP8 | 0,874 | 0,866 | 0,883 | 0,903 | 0,844 | 0,962 | 0,964 | 0,927 | 1 |
| OAS1_IFI44L_IL1R2_FAM20A_MMP8 | 0,874 | 0,865 | 0,884 | 0,904 | 0,842 | 0,966 | 0,938 | 0,858 | 1 |
| IFI27_HERC6_SLC1A2_FAM20A_MMP8 | 0,874 | 0,864 | 0,885 | 0,908 | 0,849 | 0,966 | 0,929 | 0,846 | 1 |
| IFIT1_HERC6_OLAH_FAM20A_RETN | 0,874 | 0,865 | 0,884 | 0,903 | 0,840 | 0,966 | 0,943 | 0,877 | 1 |
| OAS1_IFI44L_IL1R2_OLAH_FAM20A | 0,874 | 0,865 | 0,884 | 0,900 | 0,834 | 0,967 | 0,948 | 0,880 | 1 |
| RSAD2_SIGLEC1_ISG15_SLC1A2_IL1R2 | 0,874 | 0,866 | 0,883 | 0,909 | 0,854 | 0,964 | 0,964 | 0,927 | 1 |
| RSAD2_IL1R2_OLAH_FAM20A_MMP8 | 0,874 | 0,865 | 0,883 | 0,902 | 0,839 | 0,964 | 0,941 | 0,868 | 1 |
| RSAD2_IFIT1_SIGLEC1_SLC1A2_IL1R2 | 0,874 | 0,866 | 0,883 | 0,906 | 0,850 | 0,962 | 0,964 | 0,927 | 1 |
| IFIT1_IFI44L_IL1R2_FAM20A_MMP8 | 0,874 | 0,865 | 0,883 | 0,903 | 0,840 | 0,966 | 0,943 | 0,869 | 1 |
| IFI44L_SIGLEC1_SLC1A2_FAM20A_ RETN | 0,874 | 0,865 | 0,884 | 0,903 | 0,838 | 0,967 | 0,948 | 0,875 | 1 |
| IFI27_IFIT1_IFI44L_ISG15_RETN | 0,874 | 0,864 | 0,884 | 0,904 | 0,840 | 0,967 | 0,953 | 0,896 | 1 |
| SIGLEC1_ISG15_IL1R2_RETN_MMP8 | 0,874 | 0,865 | 0,883 | 0,901 | 0,837 | 0,964 | 0,971 | 0,938 | 1 |
| RSAD2_OAS1_IFI44L_IL1R2_FAM20A | 0,874 | 0,865 | 0,883 | 0,901 | 0,836 | 0,965 | 0,941 | 0,867 | 1 |
| IFI27_OAS1_SIGLEC1_SLC1A2_MMP8 | 0,874 | 0,865 | 0,883 | 0,904 | 0,845 | 0,963 | 0,934 | 0,868 | 1,000 |

(suite)

| Modèle | AUC modèle | IC 95% | | AUC Train | IC 95% Train | | AUC Test | IC 95% Test | |
|---|---|---|---|---|---|---|---|---|---|
| OAS1_SIGLEC1_SLC1A2_IL1R2_RETN | 0,874 | 0,865 | 0,883 | 0,910 | 0,855 | 0,965 | 0,962 | 0,922 | 1 |
| RSAD2_IFI27_IFI44L_FAM20A_MMP8 | 0,874 | 0,864 | 0,885 | 0,899 | 0,832 | 0,966 | 0,935 | 0,857 | 1 |
| RSAD2_OAS1_IFIT1_IL1R2_FAM20A | 0,874 | 0,865 | 0,883 | 0,901 | 0,836 | 0,966 | 0,941 | 0,867 | 1 |
| RSAD2_SIGLEC1_ISG15_SLC1A2_ FAM20A | 0,874 | 0,865 | 0,884 | 0,902 | 0,839 | 0,965 | 0,947 | 0,877 | 1 |
| SIGLEC1_HERC6_SLC1A2_FAM20A_ MMP8 | 0,874 | 0,864 | 0,884 | 0,901 | 0,838 | 0,964 | 0,954 | 0,881 | 1 |
| HERC6_SLC1A2_IL1R2_FAM20A_MMP8 | 0,874 | 0,865 | 0,883 | 0,913 | 0,862 | 0,964 | 0,917 | 0,819 | 1 |
| OAS1_IFI44L_SIGLEC1_IL1R2_OLAH | 0,874 | 0,865 | 0,883 | 0,901 | 0,837 | 0,965 | 0,951 | 0,905 | 0,997 |
| IFIT1_SIGLEC1_ISG15_HERC6_OLAH | 0,874 | 0,865 | 0,883 | 0,901 | 0,839 | 0,963 | 0,941 | 0,890 | 0,993 |
| OAS1_SIGLEC1_ISG15_HERC6_FAM20A | 0,874 | 0,865 | 0,884 | 0,897 | 0,831 | 0,964 | 0,948 | 0,884 | 1 |
| IFIT1_IFI44L_SIGLEC1_HERC6_OLAH | 0,874 | 0,865 | 0,883 | 0,899 | 0,837 | 0,961 | 0,937 | 0,883 | 0,991 |
| HERC6_OLAH_FAM20A_RETN_MMP8 | 0,874 | 0,864 | 0,884 | 0,900 | 0,835 | 0,965 | 0,949 | 0,885 | 1 |
| SIGLEC1_ISG15_HERC6_SLC1A2_ FAM20A | 0,874 | 0,864 | 0,884 | 0,901 | 0,838 | 0,964 | 0,952 | 0,879 | 1 |
| IFI44L_ SIGLEC1_HERC6_FAM20A_ MMP8 | 0,874 | 0,864 | 0,884 | 0,898 | 0,831 | 0,966 | 0,953 | 0,882 | 1 |
| IFIT1_SLC1A2_OLAH_FAM20A_MMP8 | 0,874 | 0,865 | 0,883 | 0,906 | 0,847 | 0,965 | 0,939 | 0,869 | 1 |
| OAS1_SIGLEC1_ISG15_SLC1A2_IL1R2 | 0,874 | 0,865 | 0,883 | 0,908 | 0,853 | 0,964 | 0,964 | 0,927 | 1 |
| IFIT1_ISG15_HERC6_OLAH_FAM20A | 0,874 | 0,864 | 0,884 | 0,900 | 0,837 | 0,964 | 0,941 | 0,872 | 1 |
| IFIT1_IFI44L_SIGLEC1_HERC6_RETN | 0,874 | 0,865 | 0,883 | 0,894 | 0,824 | 0,964 | 0,925 | 0,865 | 0,985 |
| RSAD2_OAS1_SIGLEC1_SLC1A2_IL1R2 | 0,874 | 0,865 | 0,883 | 0,908 | 0,852 | 0,963 | 0,965 | 0,926 | 1 |
| RSAD2_OAS1_SLC1A2_OLAH_FAM20A | 0,874 | 0,864 | 0,883 | 0,911 | 0,853 | 0,969 | 0,935 | 0,862 | 1 |
| IFI27_OAS1_IFIT1_HERC6_MMP8 | 0,874 | 0,865 | 0,883 | 0,899 | 0,840 | 0,957 | 0,892 | 0,811 | 0,974 |
| HERC6_SLC1A2_OLAH_FAM20A_RETN | 0,874 | 0,864 | 0,884 | 0,910 | 0,854 | 0,966 | 0,928 | 0,847 | 1 |
| IFI27_IFI44L_SLC1A2_FAM20A_MMP8 | 0,874 | 0,864 | 0,884 | 0,900 | 0,836 | 0,965 | 0,937 | 0,857 | 1 |
| RSAD2_OAS1_SIGLEC1_IL1R2_OLAH | 0,874 | 0,864 | 0,883 | 0,900 | 0,837 | 0,963 | 0,960 | 0,919 | 1 |
| OAS1_ IL1R2_OLAH_FAM20A_RETN | 0,874 | 0,864 | 0,883 | 0,900 | 0,834 | 0,965 | 0,932 | 0,853 | 1 |
| OAS1_SLC1A2_OLAH_FAM20A_MMP8 | 0,874 | 0,864 | 0,883 | 0,909 | 0,852 | 0,966 | 0,934 | 0,858 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1_IFIT1_IFI44L_IL1R2_FAM20A | 0,874 | 0,865 | 0,883 | 0,899 | 0,834 | 0,965 | 0,941 | 0,867 | 1 |
| OAS1_IFIT1_SIGLEC1_ISG15_IL1R2 | 0,874 | 0,865 | 0,882 | 0,899 | 0,836 | 0,962 | 0,955 | 0,913 | 0,998 |
| IFIT1_HERC6_OLAH_FAM20A_MMP8 | 0,874 | 0,864 | 0,884 | 0,899 | 0,835 | 0,963 | 0,943 | 0,876 | 1 |
| RSAD2_OAS1_ISG15_IL1R2_FAM20A | 0,874 | 0,864 | 0,883 | 0,897 | 0,831 | 0,963 | 0,936 | 0,857 | 1 |
| IFIT1_ISG15_HERC6_FAM20A_RETN | 0,874 | 0,864 | 0,883 | 0,899 | 0,834 | 0,963 | 0,940 | 0,864 | 1 |
| OAS1_SIGLEC1_ISG15_FAM20A_RETN | 0,874 | 0,864 | 0,884 | 0,894 | 0,822 | 0,966 | 0,955 | 0,898 | 1 |
| OAS1_SIGLEC1_ISG15_HERC6_RETN | 0,874 | 0,863 | 0,884 | 0,896 | 0,829 | 0,964 | 0,953 | 0,907 | 1,000 |
| RSAD2_IFIT1_IFI44L_SIGLEC1_IL1R2 | 0,874 | 0,865 | 0,882 | 0,896 | 0,831 | 0,961 | 0,955 | 0,913 | 0,998 |
| IFI44L_HERC6_OLAH_FAM20A_MMP8 | 0,874 | 0,864 | 0,884 | 0,899 | 0,833 | 0,964 | 0,947 | 0,881 | 1 |
| ISG15_HERC6_OLAH_FAM20A_RETN | 0,874 | 0,864 | 0,883 | 0,899 | 0,833 | 0,965 | 0,950 | 0,887 | 1 |
| RSAD2_OAS1_IFI44L_SIGLEC1_FAM20A | 0,873 | 0,864 | 0,883 | 0,897 | 0,826 | 0,968 | 0,953 | 0,892 | 1 |
| IFI44L_SIGLEC1_IL1R2_OLAH_RETN | 0,873 | 0,864 | 0,883 | 0,899 | 0,834 | 0,963 | 0,958 | 0,914 | 1 |
| IFIT1_SIGLEC1_ISG15_IL1R2_OLAH | 0,873 | 0,865 | 0,882 | 0,903 | 0,842 | 0,964 | 0,960 | 0,918 | 1 |
| RSAD2_SLC1A2_OLAH_FAM20A_MMP8 | 0,873 | 0,864 | 0,883 | 0,905 | 0,847 | 0,964 | 0,935 | 0,863 | 1 |
| SIGLEC1_IL1R2_OLAH_RETN_MMP8 | 0,873 | 0,864 | 0,883 | 0,901 | 0,838 | 0,964 | 0,968 | 0,935 | 1 |
| RSAD2_OAS1_IFIT1_SIGLEC1_IL1R2 | 0,873 | 0,864 | 0,882 | 0,897 | 0,835 | 0,960 | 0,961 | 0,922 | 1 |
| RSAD2_IFIT1_SIGLEC1_HERC6_RETN | 0,873 | 0,864 | 0,883 | 0,893 | 0,824 | 0,963 | 0,923 | 0,862 | 0,984 |
| RSAD2_IFIT1_IL1R2_FAM20A_MMP8 | 0,873 | 0,864 | 0,882 | 0,905 | 0,843 | 0,968 | 0,943 | 0,870 | 1 |
| OAS1_IFIT1_SLC1A2_IL1R2_FAM20A | 0,873 | 0,864 | 0,882 | 0,907 | 0,850 | 0,965 | 0,936 | 0,855 | 1 |
| IFI44L_HERC6_OLAH_FAM20A_RETN | 0,873 | 0,864 | 0,883 | 0,901 | 0,835 | 0,967 | 0,948 | 0,884 | 1 |
| IFI44L_SIGLEC1_ISG15_IL1R2_RETN | 0,873 | 0,865 | 0,882 | 0,900 | 0,837 | 0,963 | 0,960 | 0,917 | 1 |
| IFIT1_IFI44L_SIGLEC1_ISG15_IL1R2 | 0,873 | 0,865 | 0,882 | 0,901 | 0,840 | 0,962 | 0,959 | 0,918 | 1,000 |
| IFIT1_ISG15_IL1R2_OLAH_FAM20A | 0,873 | 0,864 | 0,883 | 0,900 | 0,835 | 0,964 | 0,953 | 0,896 | 1 |
| IFI44L_ISG15_HERC6_OLAH_FAM20A | 0,873 | 0,863 | 0,883 | 0,897 | 0,830 | 0,963 | 0,947 | 0,882 | 1 |
| SIGLEC1_ISG15_HERC6_IL1R2_RETN | 0,873 | 0,864 | 0,883 | 0,898 | 0,831 | 0,964 | 0,962 | 0,923 | 1 |

| Modèle | AUC modèle | IC 95% | | AUC Train | IC 95% Train | | AUC Test | IC 95% Test | |
|---|---|---|---|---|---|---|---|---|---|
| OAS1_IFIT1_IL1R2_FAM20A_RETN | 0,873 | 0,864 | 0,883 | 0,900 | 0,835 | 0,966 | 0,945 | 0,871 | 1 |
| OAS1_IFIT1_SLC1A2_OLAH_FAM20A | 0,873 | 0,864 | 0,883 | 0,909 | 0,850 | 0,968 | 0,935 | 0,861 | 1 |
| RSAD2_SIGLEC1_IL1R2_RETN_MMP8 | 0,873 | 0,864 | 0,883 | 0,899 | 0,835 | 0,964 | 0,972 | 0,940 | 1 |
| OAS1_IFI44L_SLC1A2_IL1R2_FAM20A | 0,873 | 0,864 | 0,882 | 0,908 | 0,851 | 0,964 | 0,925 | 0,837 | 1 |
| RSAD2_IFIT1_SIGLEC1_IL1R2_MMP8 | 0,873 | 0,864 | 0,882 | 0,901 | 0,841 | 0,961 | 0,966 | 0,931 | 1 |
| IFI27_OAS1_IFIT1_RETN_MMP8 | 0,873 | 0,863 | 0,883 | 0,904 | 0,841 | 0,968 | 0,949 | 0,887 | 1 |
| IFIT1_IFI44L_SIGLEC1_SLC1A2_IL1R2 | 0,873 | 0,865 | 0,882 | 0,907 | 0,851 | 0,963 | 0,964 | 0,927 | 1 |
| OAS1_IFIT1_HERC6_OLAH_FAM20A | 0,873 | 0,863 | 0,883 | 0,898 | 0,833 | 0,963 | 0,940 | 0,871 | 1 |
| OAS1_SIGLEC1_IL1R2_RETN_MMP8 | 0,873 | 0,864 | 0,883 | 0,899 | 0,834 | 0,963 | 0,970 | 0,936 | 1 |
| IFI44L_SIGLEC1_ISG15_FAM20A_MMP8 | 0,873 | 0,863 | 0,883 | 0,890 | 0,818 | 0,963 | 0,957 | 0,895 | 1 |
| SIGLEC1_HERC6_IL1R2_OLAH_RETN | 0,873 | 0,863 | 0,883 | 0,900 | 0,835 | 0,966 | 0,960 | 0,919 | 1 |
| OAS1_ ISG15_IL1R2_FAM20A_MMP8 | 0,873 | 0,864 | 0,882 | 0,903 | 0,841 | 0,966 | 0,934 | 0,853 | 1 |
| OAS1_IFIT1_SIGLEC1_IL1R2_RETN | 0,873 | 0,864 | 0,882 | 0,896 | 0,829 | 0,962 | 0,958 | 0,916 | 0,999 |
| RSAD2_IFI44L_SIGLEC1_IL1R2_OLAH | 0,873 | 0,864 | 0,882 | 0,899 | 0,834 | 0,964 | 0,961 | 0,921 | 1 |
| OAS1_ IFI44L_SIGLEC1_SLC1A2_IL1R2 | 0,873 | 0,864 | 0,882 | 0,907 | 0,852 | 0,963 | 0,963 | 0,925 | 1 |
| RSAD2_OAS1_HERC6_OLAH_FAM20A | 0,873 | 0,863 | 0,883 | 0,901 | 0,836 | 0,965 | 0,938 | 0,870 | 1 |
| RSAD2_IFI27_IFIT1_ISG15_RETN | 0,873 | 0,864 | 0,882 | 0,904 | 0,841 | 0,968 | 0,953 | 0,895 | 1 |
| RSAD2_IFI44L_SIGLEC1_SLC1A2_ FAM20A | 0,873 | 0,863 | 0,883 | 0,903 | 0,840 | 0,965 | 0,943 | 0,871 | 1 |
| RSAD2_IFIT1_SIGLEC1_IL1R2_OLAH | 0,873 | 0,864 | 0,882 | 0,899 | 0,836 | 0,963 | 0,959 | 0,917 | 1 |
| OAS1_SIGLEC1_SLC1A2_FAM20A_ MMP8 | 0,873 | 0,863 | 0,883 | 0,903 | 0,839 | 0,966 | 0,949 | 0,878 | 1 |
| RSAD2_ISG15_ IL1R2_OLAH_FAM20A | 0,873 | 0,864 | 0,882 | 0,901 | 0,835 | 0,967 | 0,947 | 0,880 | 1 |
| IFIT1_IFI44L_ISG15_IL1R2_FAM20A | 0,873 | 0,864 | 0,882 | 0,900 | 0,836 | 0,963 | 0,948 | 0,883 | 1 |
| ISG15_HERC6_OLAH_FAM20A_MMP8 | 0,873 | 0,863 | 0,883 | 0,899 | 0,834 | 0,963 | 0,945 | 0,878 | 1 |
| IFIT1_SIGLEC1_IL1R2_RETN_MMP8 | 0,873 | 0,863 | 0,883 | 0,900 | 0,837 | 0,964 | 0,967 | 0,933 | 1 |
| IFI27_IFIT1_IFI44L_RETN_MMP8 | 0,873 | 0,862 | 0,883 | 0,904 | 0,841 | 0,968 | 0,950 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1_IFI44L_SIGLEC1_HERC6_FAM20A | 0,873 | 0,863 | 0,882 | 0,898 | 0,832 | 0,964 | 0,954 | 0,892 | 1 |
| RSAD2_ OAS1_ IFI44L_ SIGLEC1_ IL1R2 | 0,873 | 0,864 | 0,882 | 0,895 | 0,829 | 0,960 | 0,961 | 0,922 | 1 |
| RSAD2_ IFI27_ OAS1_ HERC6_ MMP8 | 0,873 | 0,864 | 0,882 | 0,899 | 0,839 | 0,958 | 0,900 | 0,821 | 0,979 |
| IFI27_ IFI44L_ ISG15_ SLC1A2_ RETN | 0,873 | 0,863 | 0,882 | 0,905 | 0,842 | 0,968 | 0,945 | 0,879 | 1 |
| RSAD2_ SIGLEC1_ SLC1A2_ FAM20A_ RETN | 0,873 | 0,863 | 0,883 | 0,903 | 0,840 | 0,966 | 0,947 | 0,874 | 1 |
| IFI27_ IFIT1_ ISG15_ RETN_ MMP8 | 0,873 | 0,863 | 0,883 | 0,902 | 0,838 | 0,966 | 0,952 | 0,894 | 1 |
| IFI27_ IFIT1_ ISG15_ HERC6_ MMP8 | 0,873 | 0,864 | 0,882 | 0,897 | 0,837 | 0,957 | 0,895 | 0,813 | 0,976 |
| OAS1_ IFI44L_ ISG15_ IL1R2_ FAM20A | 0,873 | 0,863 | 0,882 | 0,900 | 0,836 | 0,965 | 0,943 | 0,869 | 1 |
| OAS1_ SIGLEC1_ ISG15_ SLC1A2_ FAM20A | 0,873 | 0,863 | 0,883 | 0,902 | 0,840 | 0,965 | 0,949 | 0,878 | 1 |
| IFI27_ IFIT1_ SIGLEC1_ SLC1A2_ MMP8 | 0,873 | 0,864 | 0,882 | 0,903 | 0,843 | 0,962 | 0,925 | 0,855 | 0,995 |
| OAS1_ IFI44L_ SIGLEC1_ ISG15_ IL1R2 | 0,873 | 0,864 | 0,881 | 0,900 | 0,836 | 0,963 | 0,957 | 0,915 | 0,998 |
| RSAD2_ IFI44L_ SLC1A2_ IL1R2_ FAM20A | 0,873 | 0,864 | 0,882 | 0,907 | 0,851 | 0,962 | 0,927 | 0,839 | 1 |
| OAS1_ SIGLEC1_ SLC1A2_ FAM20A_ RETN | 0,873 | 0,863 | 0,883 | 0,901 | 0,838 | 0,965 | 0,947 | 0,874 | 1 |
| OAS1_ SIGLEC1_ HERC6_ SLC1A2_ RETN | 0,873 | 0,863 | 0,882 | 0,904 | 0,843 | 0,965 | 0,951 | 0,901 | 1 |
| IFIT1_ SIGLEC1_ ISG15_ IL1R2_ MMP8 | 0,873 | 0,864 | 0,881 | 0,902 | 0,843 | 0,961 | 0,962 | 0,923 | 1 |
| IFIT1_ SLC1A2_ IL1R2_ OLAH_ FAM20A | 0,873 | 0,864 | 0,882 | 0,907 | 0,849 | 0,964 | 0,932 | 0,856 | 1 |
| OAS1_ IL1R2_ FAM20A_ RETN_ MMP8 | 0,873 | 0,863 | 0,882 | 0,904 | 0,842 | 0,967 | 0,937 | 0,858 | 1 |
| IFIT1_ IFI44L_ SIGLEC1_ IL1R2_ OLAH | 0,873 | 0,864 | 0,881 | 0,901 | 0,839 | 0,963 | 0,958 | 0,915 | 1 |
| RSAD2_ IFI44L_ SIGLEC1_ ISG15_ IL1R2 | 0,873 | 0,864 | 0,881 | 0,896 | 0,830 | 0,961 | 0,958 | 0,916 | 0,999 |
| RSAD2_ IFI27_ IFI44L_ ISG15_ RETN | 0,872 | 0,863 | 0,882 | 0,905 | 0,842 | 0,969 | 0,957 | 0,900 | 1 |
| OAS1_ ISG15_ SLC1A2_ OLAH_ FAM20A | 0,872 | 0,863 | 0,882 | 0,911 | 0,854 | 0,969 | 0,938 | 0,866 | 1 |
| IFI27_ OAS1_ IFIT1_ SLC1A2_ RETN | 0,872 | 0,863 | 0,882 | 0,901 | 0,837 | 0,966 | 0,942 | 0,870 | 1 |
| RSAD2_ IFI27_ OAS1_ ISG15_RETN | 0,872 | 0,863 | 0,882 | 0,904 | 0,841 | 0,968 | 0,949 | 0,886 | 1 |
| OAS1_ SIGLEC1_ HERC6_ FAM20A_ MMP8 | 0,872 | 0,863 | 0,882 | 0,896 | 0,828 | 0,963 | 0,949 | 0,885 | 1 |
| RSAD2_ SIGLEC1_ ISG15_ FAM20A_ RETN | 0,872 | 0,863 | 0,882 | 0,895 | 0,825 | 0,966 | 0,953 | 0,895 | 1 |

| Modèle | AUC modèle | IC 95% | | AUC Train | IC 95% Train | | AUC Test | IC 95% Test | |
|---|---|---|---|---|---|---|---|---|---|
| IFI27_ IFIT1_ ISG15_ SLC1A2_ RETN | 0,872 | 0,863 | 0,882 | 0,901 | 0,836 | 0,965 | 0,943 | 0,875 | 1 |
| IFI44L_ ISG15_ SLC1A2_ OLAH_ MMP8 | 0,872 | 0,863 | 0,881 | 0,906 | 0,848 | 0,964 | 0,937 | 0,882 | 0,991 |
| RSAD2_ IFI27_ IFIT1_ IFI44L_ RETN | 0,872 | 0,863 | 0,882 | 0,906 | 0,843 | 0,969 | 0,951 | 0,891 | 1 |
| OAS1_ SIGLEC1_ ISG15_ IL1R2_ OLAH | 0,872 | 0,863 | 0,881 | 0,900 | 0,837 | 0,963 | 0,955 | 0,912 | 0,999 |
| IFI44L_ SLC1A2_ IL1R2_ OLAH_ MMP8 | 0,872 | 0,863 | 0,881 | 0,904 | 0,847 | 0,960 | 0,932 | 0,870 | 0,993 |
| OAS1_ SLC1A2_ IL1R2_ FAM20A_ MMP8 | 0,872 | 0,864 | 0,881 | 0,911 | 0,858 | 0,964 | 0,926 | 0,835 | 1 |
| IFI44L_ SLC1A2_ IL1R2_ OLAH_ RETN | 0,872 | 0,863 | 0,882 | 0,909 | 0,852 | 0,966 | 0,930 | 0,871 | 0,990 |
| IFI27_ OAS1_ IFI44L_ SLC1A2_ RETN | 0,872 | 0,863 | 0,882 | 0,903 | 0,840 | 0,967 | 0,943 | 0,875 | 1 |
| IFIT1_ SIGLEC1_ HERC6_ RETN_ MMP8 | 0,872 | 0,862 | 0,882 | 0,893 | 0,823 | 0,963 | 0,925 | 0,865 | 0,985 |
| RSAD2_ OAS1_ SIGLEC1_ ISG15_ IL1R2 | 0,872 | 0,863 | 0,881 | 0,896 | 0,833 | 0,960 | 0,962 | 0,924 | 1 |
| IFI44L_ ISG15_ SLC1A2_ IL1R2_ OLAH | 0,872 | 0,863 | 0,881 | 0,906 | 0,850 | 0,963 | 0,936 | 0,879 | 0,993 |
| IFI27_ OAS1_ IFI44L_ HERC6_ MMP8 | 0,872 | 0,863 | 0,881 | 0,900 | 0,842 | 0,958 | 0,897 | 0,817 | 0,976 |
| RSAD2_ SIGLEC1_ ISG15_ IL1R2_ MMP8 | 0,872 | 0,863 | 0,881 | 0,903 | 0,844 | 0,962 | 0,965 | 0,929 | 1 |
| RSAD2_ IFI44L_ SIGLEC1_ SLC1A2_ IL1R2 | 0,872 | 0,863 | 0,881 | 0,909 | 0,853 | 0,965 | 0,966 | 0,931 | 1 |
| OAS1_ HERC6_ OLAH_ FAM20A_ RETN | 0,872 | 0,862 | 0,882 | 0,902 | 0,838 | 0,966 | 0,945 | 0,879 | 1 |
| RSAD2_ OAS1_ IL1R2_ FAM20A_ RETN | 0,872 | 0,863 | 0,881 | 0,898 | 0,832 | 0,964 | 0,934 | 0,854 | 1 |
| OAS1_ SLC1A2_ OLAH_FAM20A_ RETN | 0,872 | 0,862 | 0,882 | 0,912 | 0,855 | 0,970 | 0,938 | 0,866 | 1 |
| RSAD2_ HERC6_ OLAH_ FAM20A_ RETN | 0,872 | 0,862 | 0,882 | 0,902 | 0,838 | 0,966 | 0,943 | 0,878 | 1 |
| IFI27_ SLC1A2_ FAM20A_ RETN_ MMP8 | 0,872 | 0,862 | 0,882 | 0,899 | 0,835 | 0,964 | 0,913 | 0,817 | 1 |
| RSAD2_ OAS1_ SLC1A2_ IL1R2_ FAM20A | 0,872 | 0,863 | 0,881 | 0,905 | 0,848 | 0,961 | 0,932 | 0,846 | 1 |
| RSAD2_ IFI44L_ HERC6_ OLAH_ FAM20A | 0,872 | 0,862 | 0,882 | 0,901 | 0,837 | 0,965 | 0,940 | 0,874 | 1 |
| OAS1_ IFI44L_ SIGLEC1_ IL1R2_ RETN | 0,872 | 0,863 | 0,881 | 0,897 | 0,832 | 0,963 | 0,954 | 0,911 | 0,998 |
| RSAD2_ SIGLEC1_ ISG15_ FAM20A_ MMP8 | 0,872 | 0,862 | 0,881 | 0,895 | 0,824 | 0,965 | 0,952 | 0,889 | 1 |
| OAS1_ IFI44L_ SIGLEC1_ FAM20A_ RETN | 0,872 | 0,862 | 0,882 | 0,894 | 0,822 | 0,966 | 0,954 | 0,895 | 1 |
| RSAD2_ OAS1_ SIGLEC1_ IL1R2_ RETN | 0,872 | 0,862 | 0,881 | 0,897 | 0,831 | 0,962 | 0,964 | 0,925 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2_ IFIT1_ SIGLEC1_ HERC6_ OLAH | 0,872 | 0,862 | 0,881 | 0,900 | 0,838 | 0,962 | 0,937 | 0,883 | 0,991 |
| IFIT1_IL1R2_FAM20A_RETN_MMP8 | 0,872 | 0,862 | 0,881 | 0,902 | 0,838 | 0,967 | 0,945 | 0,872 | 1 |
| OAS1_ IFI44L_ HERC6_ OLAH_ FAM20A | 0,872 | 0,862 | 0,881 | 0,902 | 0,838 | 0,966 | 0,947 | 0,885 | 1 |
| RSAD2_ ISG15_ HERC6_ OLAH_ FAM20A | 0,872 | 0,862 | 0,881 | 0,901 | 0,839 | 0,963 | 0,938 | 0,868 | 1 |
| IFI27_ IFI44L_ SLC1A2_ RETN_ MMP8 | 0,872 | 0,862 | 0,882 | 0,902 | 0,839 | 0,965 | 0,934 | 0,861 | 1 |
| IFI27_ OAS1_ IFI44L_ RETN_ MMP8 | 0,872 | 0,862 | 0,881 | 0,905 | 0,842 | 0,969 | 0,949 | 0,890 | 1 |
| SIGLEC1_ ISG15_ HERC6_ FAM20A_ MMP8 | 0,872 | 0,861 | 0,882 | 0,891 | 0,819 | 0,963 | 0,952 | 0,881 | 1 |
| OAS1_ ISG15_ SLC1A2_ IL1R2_ FAM20A | 0,872 | 0,862 | 0,881 | 0,905 | 0,848 | 0,962 | 0,927 | 0,841 | 1 |
| IFI44L_ ISG15_ IL1R2_ FAM20A_ RETN | 0,871 | 0,862 | 0,881 | 0,900 | 0,837 | 0,963 | 0,938 | 0,865 | 1 |
| OAS1_ SIGLEC1_ IL1R2_ OLAH_ RETN | 0,871 | 0,862 | 0,881 | 0,899 | 0,834 | 0,964 | 0,958 | 0,915 | 1 |
| OAS1_ ISG15_ IL1R2_ FAM20A_ RETN | 0,871 | 0,862 | 0,881 | 0,898 | 0,833 | 0,963 | 0,935 | 0,856 | 1 |
| OAS1_SIGLEC1_ISG15_IL1R2_MMP8 | 0,871 | 0,863 | 0,880 | 0,902 | 0,843 | 0,962 | 0,963 | 0,925 | 1 |
| IFIT1_ IFI44L_ SIGLEC1_ IL1R2_ RETN | 0,871 | 0,862 | 0,881 | 0,898 | 0,834 | 0,962 | 0,959 | 0,916 | 1 |
| RSAD2_ IFIT1_ SLC1A2_ IL1R2_ FAM20A | 0,871 | 0,863 | 0,880 | 0,904 | 0,845 | 0,962 | 0,935 | 0,854 | 1 |
| IFI27_ OAS1_ IFI44L_ FAM20A_ MMP8 | 0,871 | 0,861 | 0,882 | 0,899 | 0,832 | 0,966 | 0,937 | 0,858 | 1 |
| IFI27_ ISG15_ HERC6_ SLC1A2_ FAM20A | 0,871 | 0,861 | 0,882 | 0,905 | 0,846 | 0,964 | 0,933 | 0,850 | 1 |
| RSAD2_ SLC1A2_ OLAH_ FAM20A_ RETN | 0,871 | 0,862 | 0,881 | 0,908 | 0,849 | 0,967 | 0,932 | 0,856 | 1 |
| RSAD2_ SIGLEC1_ ISG15_ IL1R2_ OLAH | 0,871 | 0,862 | 0,880 | 0,899 | 0,836 | 0,962 | 0,960 | 0,919 | 1 |
| IFIT1_ SIGLEC1_ HERC6_ SLC1A2_ RETN | 0,871 | 0,862 | 0,880 | 0,904 | 0,844 | 0,963 | 0,929 | 0,871 | 0,987 |
| IFIT1_ SIGLEC1_ ISG15_ IL1R2_ RETN | 0,871 | 0,862 | 0,880 | 0,898 | 0,834 | 0,962 | 0,963 | 0,922 | 1 |
| RSAD2_ ISG15_ SLC1A2_ OLAH_ FAM20A | 0,871 | 0,862 | 0,881 | 0,908 | 0,849 | 0,967 | 0,932 | 0,858 | 1 |
| IFIT1_ ISG15_ SLC1A2_ OLAH_ FAM20A | 0,871 | 0,862 | 0,881 | 0,903 | 0,841 | 0,964 | 0,938 | 0,868 | 1 |
| OAS1_ ISG15_ HERC6_ OLAH_ FAM20A | 0,871 | 0,861 | 0,881 | 0,900 | 0,836 | 0,963 | 0,945 | 0,878 | 1 |
| SIGLEC1_ ISG15_ IL1R2_ OLAH_ RETN | 0,871 | 0,862 | 0,880 | 0,900 | 0,836 | 0,964 | 0,964 | 0,924 | 1 |
| IFI44L_ ISG15_ SLC1A2_ OLAH_ RETN | 0,871 | 0,862 | 0,881 | 0,908 | 0,850 | 0,967 | 0,933 | 0,876 | 0,990 |

| Modèle | AUC modèle | IC 95% | | AUC Train | IC 95% Train | | AUC Test | IC 95% Test | |
|---|---|---|---|---|---|---|---|---|---|
| OAS1_ IFIT1_ SIGLEC1_ OLAH_ RETN | 0,871 | 0,862 | 0,881 | 0,895 | 0,825 | 0,964 | 0,942 | 0,889 | 0,995 |
| OAS1_ IFI44L_ SIGLEC1_ SLC1A2_ FAM20A | 0,871 | 0,861 | 0,881 | 0,903 | 0,841 | 0,966 | 0,948 | 0,878 | 1 |
| RSAD2_ HERC6_ OLAH_ FAM20A_ MMP8 | 0,871 | 0,862 | 0,881 | 0,898 | 0,834 | 0,962 | 0,941 | 0,873 | 1 |
| SIGLEC1_ HERC6_ OLAH_ RETN_ MMP8 | 0,871 | 0,861 | 0,882 | 0,892 | 0,820 | 0,965 | 0,961 | 0,922 | 1,000 |
| IFI27_ OAS1_ HERC6_ SLC1A2_ FAM20A | 0,871 | 0,861 | 0,881 | 0,904 | 0,846 | 0,963 | 0,933 | 0,850 | 1 |
| RSAD2_ IFI27_ HERC6_ SLC1A2_ FAM20A | 0,871 | 0,861 | 0,881 | 0,904 | 0,845 | 0,962 | 0,932 | 0,848 | 1 |
| IFIT1_ IFI44L_ SLC1A2_ OLAH_ MMP8 | 0,871 | 0,862 | 0,880 | 0,905 | 0,847 | 0,963 | 0,937 | 0,883 | 0,991 |
| OAS1_ IFIT1_ SIGLEC1_ OLAH_ MMP8 | 0,871 | 0,862 | 0,880 | 0,893 | 0,825 | 0,961 | 0,946 | 0,895 | 0,996 |
| RSAD2_ ISG15_ SLC1A2_ IL1R2_ FAM20A | 0,871 | 0,862 | 0,880 | 0,902 | 0,845 | 0,959 | 0,930 | 0,842 | 1 |
| RSAD2_ SLC1A2_ IL1R2_ FAM20A_ MMP8 | 0,871 | 0,863 | 0,879 | 0,906 | 0,851 | 0,960 | 0,926 | 0,832 | 1 |
| RSAD2_ ISG15_ HERC6_ FAM20A_ RETN | 0,871 | 0,861 | 0,881 | 0,900 | 0,837 | 0,963 | 0,938 | 0,862 | 1 |
| IFIT1_ SIGLEC1_ IL1R2_ OLAH_ RETN | 0,871 | 0,862 | 0,880 | 0,899 | 0,834 | 0,963 | 0,958 | 0,914 | 1 |
| IFIT1_ SLC1A2_ IL1R2_ FAM20A_ RETN | 0,871 | 0,861 | 0,880 | 0,906 | 0,849 | 0,963 | 0,934 | 0,855 | 1 |
| IFI27_ IFI44L_ HERC6_ SLC1A2_ FAM20A | 0,871 | 0,861 | 0,881 | 0,902 | 0,841 | 0,963 | 0,938 | 0,856 | 1 |
| RSAD2_ IFI44L_ SIGLEC1_ IL1R2_ RETN | 0,871 | 0,862 | 0,880 | 0,894 | 0,827 | 0,962 | 0,960 | 0,920 | 1,000 |
| RSAD2_ IFI44L_ SIGLEC1_ ISG15_ FAM20A | 0,871 | 0,861 | 0,881 | 0,894 | 0,823 | 0,965 | 0,951 | 0,892 | 1 |
| OAS1_ HERC6_ OLAH_ FAM20A_ MMP8 | 0,871 | 0,861 | 0,881 | 0,899 | 0,835 | 0,963 | 0,943 | 0,876 | 1 |
| IFI44L_ SIGLEC1_ HERC6_ OLAH_ RETN | 0,871 | 0,861 | 0,881 | 0,893 | 0,823 | 0,963 | 0,950 | 0,903 | 0,997 |
| RSAD2_ IFIT1_ SIGLEC1_ ISG15_ IL1R2 | 0,871 | 0,862 | 0,880 | 0,898 | 0,835 | 0,961 | 0,961 | 0,922 | 1 |
| IFI44L_ SIGLEC1_ HERC6_ OLAH_ MMP8 | 0,871 | 0,861 | 0,880 | 0,894 | 0,828 | 0,960 | 0,953 | 0,909 | 0,998 |
| RSAD2_ IFI27_ IFIT1_ IFI44L_ FAM20A | 0,871 | 0,861 | 0,881 | 0,900 | 0,831 | 0,968 | 0,941 | 0,865 | 1 |
| OAS1_ IFI44L_ SIGLEC1_ ISG15_ FAM20A | 0,871 | 0,861 | 0,881 | 0,893 | 0,821 | 0,965 | 0,957 | 0,896 | 1 |
| RSAD2_ SIGLEC1_ ISG15_ HERC6_ RETN | 0,871 | 0,861 | 0,881 | 0,896 | 0,828 | 0,965 | 0,939 | 0,886 | 0,992 |
| RSAD2_ IFIT1_ SLC1A2_ OLAH_ FAM20A | 0,871 | 0,861 | 0,880 | 0,906 | 0,846 | 0,966 | 0,938 | 0,866 | 1 |
| RSAD2_ IFI27_ ISG15_ HERC6_ MMP8 | 0,871 | 0,862 | 0,879 | 0,896 | 0,838 | 0,955 | 0,896 | 0,817 | 0,975 |

(suite)

| Modèle | AUC modèle | IC 95% | | AUC Train | IC 95% Train | | AUC Test | IC 95% Test | |
|---|---|---|---|---|---|---|---|---|---|
| RSAD2_ IFIT1_ ISG15_ IL1R2_ FAM20A | 0,871 | 0,861 | 0,880 | 0,900 | 0,835 | 0,965 | 0,950 | 0,883 | 1 |
| IFI27_ OAS1_ HERC6_ SLC1A2_ MMP8 | 0,871 | 0,862 | 0,880 | 0,904 | 0,849 | 0,959 | 0,897 | 0,813 | 0,980 |
| RSAD2_ IFI27_ SIGLEC1_ SLC1A2_ MMP8 | 0,871 | 0,861 | 0,880 | 0,901 | 0,841 | 0,961 | 0,932 | 0,865 | 0,998 |
| IFIT1_ IFI44L_ IL1R2_ OLAH_ FAM20A | 0,871 | 0,861 | 0,880 | 0,898 | 0,833 | 0,963 | 0,955 | 0,901 | 1 |
| RSAD2_ IFIT1_ HERC6_ OLAH_ FAM20A | 0,871 | 0,861 | 0,880 | 0,900 | 0,836 | 0,964 | 0,942 | 0,875 | 1 |
| IFI44L_ SLC1A2_ OLAH_ RETN_ MMP8 | 0,871 | 0,861 | 0,880 | 0,906 | 0,846 | 0,967 | 0,943 | 0,893 | 0,994 |
| OAS1_ SIGLEC1_ HERC6_ OLAH_ RETN | 0,871 | 0,861 | 0,880 | 0,894 | 0,826 | 0,961 | 0,954 | 0,910 | 0,999 |
| IFI27_ OAS1_ SIGLEC1_ HERC6_ SLC1A2_ | 0,870 | 0,862 | 0,879 | 0,900 | 0,844 | 0,956 | 0,953 | 0,905 | 1 |
| IFI27_ IFI44L_ SIGLEC1_ SLC1A2_ MMP8 | 0,870 | 0,862 | 0,879 | 0,900 | 0,840 | 0,961 | 0,929 | 0,860 | 0,999 |
| IFI27_ SIGLEC1_ ISG15_ SLC1A2_ MMP8 | 0,870 | 0,861 | 0,880 | 0,903 | 0,844 | 0,961 | 0,930 | 0,863 | 0,997 |
| RSAD2_ IFIT1_ SIGLEC1_ IL1R2_ RETN | 0,870 | 0,861 | 0,880 | 0,897 | 0,832 | 0,963 | 0,960 | 0,919 | 1 |
| OAS1_ IFI44L_ IL1R2_ FAM20A_ RETN | 0,870 | 0,861 | 0,880 | 0,900 | 0,835 | 0,964 | 0,938 | 0,862 | 1 |
| RSAD2_ IFI27_ IFI44L_ SLC1A2_ FAM20A | 0,870 | 0,860 | 0,881 | 0,902 | 0,837 | 0,966 | 0,940 | 0,861 | 1 |
| IFI27_ IFIT1_ IFI44L_ ISG15_ FAM20A | 0,870 | 0,860 | 0,880 | 0,895 | 0,826 | 0,964 | 0,940 | 0,863 | 1 |
| IFI27_ IFIT1_ IFI44L_ HERC6_ MMP8 | 0,870 | 0,861 | 0,880 | 0,896 | 0,837 | 0,956 | 0,890 | 0,807 | 0,973 |
| RSAD2_ IFI27_ IFI44L_ SLC1A2_ RETN | 0,870 | 0,861 | 0,880 | 0,902 | 0,838 | 0,966 | 0,946 | 0,880 | 1 |
| OAS1_ IFI44L_ SIGLEC1_ FAM20A_ MMP8 | 0,870 | 0,860 | 0,881 | 0,892 | 0,820 | 0,964 | 0,955 | 0,893 | 1 |
| IFI27_ OAS1_ ISG15_ SLC1A2_ RETN | 0,870 | 0,861 | 0,880 | 0,901 | 0,837 | 0,965 | 0,940 | 0,870 | 1 |
| IFI44L_ ISG15_ HERC6_ FAM20A_ RETN | 0,870 | 0,860 | 0,880 | 0,896 | 0,827 | 0,965 | 0,945 | 0,870 | 1 |
| RSAD2_ IFI27_ OAS1_ RETN_ MMP8 | 0,870 | 0,860 | 0,880 | 0,901 | 0,836 | 0,966 | 0,943 | 0,880 | 1 |
| IFI27_ IFI44L_ ISG15_ SLC1A2_ FAM20A | 0,870 | 0,860 | 0,880 | 0,897 | 0,831 | 0,963 | 0,943 | 0,866 | 1 |
| IFIT1_ ISG15_ IL1R2_ FAM20A_ RETN | 0,870 | 0,860 | 0,880 | 0,901 | 0,838 | 0,964 | 0,949 | 0,884 | 1 |
| OAS1_ ISG15_ HERC6_ IL1R2_ RETN | 0,870 | 0,861 | 0,880 | 0,897 | 0,835 | 0,960 | 0,949 | 0,899 | 0,999 |
| RSAD2_ OAS1_ SIGLEC1_ HERC6_ RETN | 0,870 | 0,860 | 0,880 | 0,890 | 0,820 | 0,961 | 0,939 | 0,886 | 0,992 |
| IFIT1_ IFI44L_ HERC6_ SLC1A2_ OLAH | 0,870 | 0,860 | 0,880 | 0,905 | 0,848 | 0,963 | 0,940 | 0,886 | 0,994 |

(suite)

| Modèle | AUC modèle | IC 95% | | AUC Train | IC 95% Train | | AUC Test | IC 95% Test | |
|---|---|---|---|---|---|---|---|---|---|
| IFI44L_ IL1R2_ FAM20A_ RETN_ MMP8 | 0,870 | 0,861 | 0,879 | 0,899 | 0,836 | 0,962 | 0,938 | 0,860 | 1 |
| IFI27_ OAS1_ ISG15_ RETN_ MMP8 | 0,870 | 0,860 | 0,880 | 0,903 | 0,840 | 0,966 | 0,948 | 0,888 | 1 |
| RSAD2_ IFI44L_ IL1R2_ OLAH_ FAM20A | 0,870 | 0,860 | 0,880 | 0,898 | 0,832 | 0,964 | 0,950 | 0,888 | 1 |
| RSAD2_ IFI44L_ SIGLEC1_ FAM20A_ MMP8 | 0,870 | 0,860 | 0,880 | 0,894 | 0,824 | 0,964 | 0,947 | 0,881 | 1 |
| ISG15_ IL1R2_ OLAH_ FAM20A_ MMP8 | 0,870 | 0,861 | 0,879 | 0,900 | 0,840 | 0,960 | 0,930 | 0,855 | 1 |
| IFIT1_ SLC1A2_ OLAH_ FAM20A_ RETN | 0,870 | 0,860 | 0,880 | 0,903 | 0,841 | 0,964 | 0,937 | 0,866 | 1 |
| RSAD2_ IFIT1_ IL1R2_ OLAH_ FAM20A | 0,870 | 0,861 | 0,879 | 0,899 | 0,831 | 0,966 | 0,952 | 0,890 | 1 |
| IFI27_ IFIT1_ ISG15_ FAM20A_ MMP8 | 0,870 | 0,860 | 0,880 | 0,896 | 0,829 | 0,963 | 0,935 | 0,854 | 1 |
| RSAD2_ IFI27_ OAS1_ SLC1A2_ RETN | 0,870 | 0,860 | 0,880 | 0,901 | 0,836 | 0,966 | 0,938 | 0,863 | 1 |
| IFI44L_ ISG15_ HERC6_ SLC1A2_ OLAH | 0,870 | 0,860 | 0,880 | 0,904 | 0,846 | 0,962 | 0,939 | 0,885 | 0,994 |
| RSAD2_ HERC6_ SLC1A2_ IL1R2_ OLAH | 0,870 | 0,861 | 0,879 | 0,905 | 0,847 | 0,962 | 0,942 | 0,887 | 0,997 |
| RSAD2_ IFI27_ IFI44L_ HERC6_ MMP8 | 0,870 | 0,861 | 0,879 | 0,897 | 0,837 | 0,956 | 0,892 | 0,811 | 0,974 |
| RSAD2_ SIGLEC1_ IL1R2_ OLAH_ RETN | 0,870 | 0,860 | 0,879 | 0,898 | 0,832 | 0,963 | 0,961 | 0,919 | 1 |
| RSAD2_ ISG15_ IL1R2_ FAM20A_ MMP8 | 0,870 | 0,861 | 0,879 | 0,900 | 0,837 | 0,962 | 0,935 | 0,852 | 1 |
| OAS1_ IFIT1_ IFI44L_ SIGLEC1_ OLAH | 0,870 | 0,861 | 0,879 | 0,893 | 0,825 | 0,962 | 0,942 | 0,889 | 0,996 |
| IFI27_ IFIT1_ HERC6_ SLC1A2_ FAM20A | 0,870 | 0,860 | 0,880 | 0,903 | 0,844 | 0,962 | 0,933 | 0,850 | 1 |
| RSAD2_ IFI27_ IFI44L_ RETN_ MMP8 | 0,870 | 0,860 | 0,880 | 0,903 | 0,839 | 0,967 | 0,951 | 0,895 | 1 |
| OAS1_ ISG15_ HERC6_ FAM20A_ RETN | 0,870 | 0,860 | 0,880 | 0,898 | 0,833 | 0,963 | 0,938 | 0,862 | 1 |
| IFI44L_ HERC6_ SLC1A2_ OLAH_ MMP8 | 0,870 | 0,860 | 0,880 | 0,904 | 0,846 | 0,962 | 0,939 | 0,885 | 0,994 |
| IFI44L_ HERC6_ SLC1A2_ IL1R2_ OLAH | 0,870 | 0,860 | 0,880 | 0,907 | 0,851 | 0,963 | 0,942 | 0,888 | 0,997 |
| OAS1_ SLC1A2_ IL1R2_ FAM20A_ RETN | 0,870 | 0,861 | 0,879 | 0,906 | 0,849 | 0,962 | 0,926 | 0,839 | 1 |
| IFI44L_ IL1R2_ OLAH_ FAM20A_ RETN | 0,870 | 0,860 | 0,879 | 0,898 | 0,834 | 0,962 | 0,945 | 0,882 | 1 |
| OAS1_ SIGLEC1_ ISG15_ FAM20A_ MMP8 | 0,870 | 0,859 | 0,880 | 0,892 | 0,820 | 0,964 | 0,955 | 0,893 | 1 |
| IFIT1_ SLC1A2_ IL1R2_ OLAH_ MMP8 | 0,870 | 0,861 | 0,878 | 0,902 | 0,845 | 0,960 | 0,949 | 0,902 | 0,996 |
| IFI27_ IFIT1_ IFI44L_ SLC1A2_ RETN | 0,870 | 0,860 | 0,879 | 0,901 | 0,836 | 0,965 | 0,945 | 0,878 | 1 |

| Modèle | AUC modèle | IC 95% | | AUC Train | IC 95% Train | | AUC Test | IC 95% Test | |
|---|---|---|---|---|---|---|---|---|---|
| IFI27_ IFIT1_ SLC1A2_ RETN_ MMP8 | 0,870 | 0,860 | 0,880 | 0,904 | 0,841 | 0,966 | 0,943 | 0,877 | 1 |
| RSAD2_ IFI27_ IFIT1_ RETN_ MMP8 | 0,869 | 0,860 | 0,879 | 0,905 | 0,842 | 0,968 | 0,950 | 0,889 | 1 |
| RSAD2_ OAS1_ IFIT1_ SIGLEC1_ OLAH | 0,869 | 0,860 | 0,879 | 0,894 | 0,825 | 0,962 | 0,946 | 0,894 | 0,997 |
| RSAD2_ SIGLEC1_ HERC6_ SLC1A2_ RETN | 0,869 | 0,860 | 0,879 | 0,902 | 0,840 | 0,964 | 0,940 | 0,887 | 0,993 |
| RSAD2_ IFI44L_ IL1R2_ FAM20A_ MMP8 | 0,869 | 0,860 | 0,879 | 0,900 | 0,837 | 0,962 | 0,936 | 0,855 | 1 |
| RSAD2_ OAS1_ SIGLEC1_ HERC6_ OLAH | 0,869 | 0,860 | 0,879 | 0,896 | 0,833 | 0,959 | 0,949 | 0,902 | 0,996 |
| OAS1_ IFIT1_ HERC6_ IL1R2_ MMP8 | 0,869 | 0,860 | 0,878 | 0,896 | 0,834 | 0,958 | 0,949 | 0,902 | 0,996 |
| RSAD2_ IL1R2_ OLAH_ FAM20A_ RETN | 0,869 | 0,860 | 0,879 | 0,897 | 0,830 | 0,963 | 0,945 | 0,874 | 1 |
| RSAD2_ IFI44L_ ISG15_ IL1R2_ FAM20A | 0,869 | 0,860 | 0,879 | 0,898 | 0,833 | 0,963 | 0,943 | 0,873 | 1 |
| OAS1_ SIGLEC1_ ISG15_ IL1R2_ RETN | 0,869 | 0,860 | 0,879 | 0,898 | 0,833 | 0,963 | 0,961 | 0,920 | 1 |
| RSAD2_ IFI27_ IFIT1_ SLC1A2_ RETN | 0,869 | 0,860 | 0,879 | 0,900 | 0,835 | 0,965 | 0,943 | 0,873 | 1 |
| RSAD2_ SIGLEC1_ ISG15_ IL1R2_ RETN | 0,869 | 0,860 | 0,879 | 0,897 | 0,832 | 0,963 | 0,963 | 0,923 | 1 |
| OAS1_ IFI44L_ SIGLEC1_ HERC6_ OLAH | 0,869 | 0,860 | 0,879 | 0,895 | 0,831 | 0,958 | 0,951 | 0,905 | 0,997 |
| IFI27_ IFI44L_ ISG15_ HERC6_ MMP8 | 0,869 | 0,860 | 0,878 | 0,894 | 0,832 | 0,956 | 0,895 | 0,814 | 0,975 |
| IFI44L_ SIGLEC1_ ISG15_ HERC6_ OLAH | 0,869 | 0,860 | 0,879 | 0,894 | 0,828 | 0,960 | 0,951 | 0,905 | 0,997 |
| RSAD2_ IFI44L_ SIGLEC1_ FAM20A_ RETN | 0,869 | 0,859 | 0,879 | 0,894 | 0,823 | 0,965 | 0,951 | 0,892 | 1 |
| RSAD2_ SIGLEC1_ HERC6_ OLAH_ RETN | 0,869 | 0,860 | 0,879 | 0,895 | 0,828 | 0,963 | 0,948 | 0,900 | 0,996 |
| ISG15_ SLC1A2_ IL1R2_ FAM20A_ RETN | 0,869 | 0,860 | 0,879 | 0,906 | 0,850 | 0,963 | 0,916 | 0,826 | 1 |
| IFI27_ IFIT1_ IFI44L_ SLC1A2_ FAM20A | 0,869 | 0,859 | 0,879 | 0,899 | 0,834 | 0,965 | 0,942 | 0,865 | 1 |
| IFIT1_ IFI44L_ HERC6_ FAM20A_ RETN | 0,869 | 0,859 | 0,879 | 0,896 | 0,827 | 0,964 | 0,928 | 0,848 | 1 |
| OAS1_ IFIT1_ ISG15_ HERC6_ IL1R2 | 0,869 | 0,860 | 0,878 | 0,897 | 0,839 | 0,956 | 0,947 | 0,897 | 0,997 |
| RSAD2_ SLC1A2_ IL1R2_ FAM20A_ RETN | 0,869 | 0,860 | 0,878 | 0,901 | 0,843 | 0,958 | 0,929 | 0,840 | 1 |
| IFIT1_ IFI44L_ ISG15_ SLC1A2_ OLAH | 0,869 | 0,860 | 0,878 | 0,907 | 0,849 | 0,964 | 0,934 | 0,878 | 0,990 |
| OAS1_ IFIT1_ HERC6_ IL1R2_ RETN | 0,869 | 0,859 | 0,879 | 0,893 | 0,827 | 0,960 | 0,949 | 0,902 | 0,996 |
| OAS1_ HERC6_ SLC1A2_ IL1R2_ OLAH | 0,869 | 0,860 | 0,878 | 0,904 | 0,847 | 0,961 | 0,939 | 0,883 | 0,995 |

| Modèle | AUC modèle | IC 95% | | AUC Train | IC 95% Train | | AUC Test | IC 95% Test | |
|---|---|---|---|---|---|---|---|---|---|
| OAS1_ IFIT1_ SLC1A2_ IL1R2_ OLAH | 0,869 | 0,859 | 0,878 | 0,907 | 0,849 | 0,965 | 0,943 | 0,892 | 0,995 |
| RSAD2_ SIGLEC1_ HERC6_ OLAH_ MMP8 | 0,869 | 0,859 | 0,878 | 0,897 | 0,834 | 0,960 | 0,949 | 0,902 | 0,996 |
| OAS1_ IFIT1_ SIGLEC1_ ISG15_ OLAH | 0,869 | 0,860 | 0,878 | 0,894 | 0,826 | 0,962 | 0,941 | 0,888 | 0,995 |
| RSAD2_ OAS1_ IFIT1_ HERC6_ IL1R2 | 0,869 | 0,860 | 0,878 | 0,895 | 0,833 | 0,957 | 0,942 | 0,891 | 0,994 |
| RSAD2_ OAS1_ OLAH_ FAM20A_ MMP8 | 0,869 | 0,859 | 0,879 | 0,893 | 0,823 | 0,963 | 0,946 | 0,881 | 1 |
| IFIT1_ IFI44L_ SLC1A2_ OLAH_ RETN | 0,869 | 0,859 | 0,878 | 0,908 | 0,850 | 0,967 | 0,933 | 0,875 | 0,990 |
| RSAD2_ IFI27_ ISG15_ SLC1A2_ RETN | 0,869 | 0,859 | 0,878 | 0,901 | 0,837 | 0,965 | 0,939 | 0,868 | 1 |
| OAS1_ IFI44L_ SIGLEC1_ HERC6_ RETN | 0,869 | 0,859 | 0,878 | 0,889 | 0,817 | 0,961 | 0,946 | 0,896 | 0,995 |
| IFIT1_ HERC6_ IL1R2_ RETN_ MMP8 | 0,869 | 0,859 | 0,878 | 0,894 | 0,826 | 0,962 | 0,959 | 0,918 | 1,000 |
| RSAD2_ IFI44L_ HERC6_ SLC1A2_ OLAH | 0,869 | 0,859 | 0,878 | 0,905 | 0,849 | 0,962 | 0,934 | 0,876 | 0,991 |
| RSAD2_ IFI27_ ISG15_ RETN_ MMP8 | 0,869 | 0,859 | 0,878 | 0,901 | 0,837 | 0,965 | 0,951 | 0,894 | 1 |
| ISG15_ HERC6_ IL1R2_ RETN_ MMP8 | 0,869 | 0,859 | 0,878 | 0,897 | 0,833 | 0,960 | 0,948 | 0,895 | 1 |
| RSAD2_ IFIT1_ ISG15_ HERC6_ IL1R2 | 0,869 | 0,860 | 0,877 | 0,898 | 0,838 | 0,957 | 0,946 | 0,895 | 0,996 |
| ISG15_ SLC1A2_ IL1R2_ FAM20A_ MMP8 | 0,869 | 0,860 | 0,877 | 0,906 | 0,852 | 0,961 | 0,914 | 0,822 | 1 |
| IFIT1_ HERC6_ SLC1A2_ IL1R2_ OLAH | 0,869 | 0,859 | 0,878 | 0,904 | 0,846 | 0,961 | 0,942 | 0,889 | 0,996 |
| OAS1_ IFI44L_ HERC6_ SLC1A2_ OLAH | 0,869 | 0,858 | 0,879 | 0,905 | 0,848 | 0,962 | 0,938 | 0,883 | 0,994 |
| OAS1_ IFIT1_ HERC6_ SLC1A2_ IL1R2 | 0,869 | 0,859 | 0,878 | 0,902 | 0,845 | 0,959 | 0,946 | 0,893 | 0,998 |
| OAS1_ SIGLEC1_ HERC6_ OLAH_ MMP8 | 0,869 | 0,859 | 0,878 | 0,893 | 0,828 | 0,957 | 0,953 | 0,909 | 0,998 |
| IFIT1_ ISG15_ SLC1A2_ IL1R2_ OLAH | 0,869 | 0,859 | 0,878 | 0,907 | 0,849 | 0,965 | 0,948 | 0,899 | 0,996 |
| IFIT1_ HERC6_ SLC1A2_ OLAH_ MMP8 | 0,868 | 0,859 | 0,878 | 0,902 | 0,844 | 0,960 | 0,943 | 0,891 | 0,996 |
| IFI27_ OAS1_ IFI44L_ ISG15_ FAM20A | 0,868 | 0,858 | 0,879 | 0,893 | 0,824 | 0,963 | 0,942 | 0,863 | 1 |
| IFI44L_ HERC6_ SLC1A2_ OLAH_ RETN | 0,868 | 0,858 | 0,879 | 0,906 | 0,848 | 0,965 | 0,942 | 0,889 | 0,995 |
| RSAD2_ OAS1_ HERC6_ SLC1A2_ OLAH | 0,868 | 0,859 | 0,878 | 0,902 | 0,844 | 0,960 | 0,945 | 0,892 | 0,998 |
| IFI44L_ ISG15_ HERC6_ IL1R2_ RETN | 0,868 | 0,859 | 0,878 | 0,896 | 0,832 | 0,960 | 0,949 | 0,900 | 0,998 |
| SIGLEC1_ ISG15_ HERC6_ OLAH_ RETN | 0,868 | 0,858 | 0,879 | 0,892 | 0,820 | 0,964 | 0,957 | 0,914 | 0,999 |

(suite)

| Modèle | AUC modèle | IC 95% | | AUC Train | IC 95% Train | | AUC Test | IC 95% Test | |
|---|---|---|---|---|---|---|---|---|---|
| OAS1_IFIT1_SLC1A2_OLAH_MMP8 | 0,868 | 0,859 | 0,878 | 0,905 | 0,847 | 0,962 | 0,945 | 0,895 | 0,994 |
| IFIT1_SIGLEC1_OLAH_RETN_MMP8 | 0,868 | 0,858 | 0,878 | 0,894 | 0,826 | 0,962 | 0,959 | 0,915 | 1 |
| IFI27_ISG15_SLC1A2_RETN_MMP8 | 0,868 | 0,858 | 0,878 | 0,900 | 0,838 | 0,962 | 0,929 | 0,851 | 1 |
| IFIT1_ISG15_HERC6_IL1R2_MMP8 | 0,868 | 0,859 | 0,877 | 0,896 | 0,836 | 0,957 | 0,948 | 0,899 | 0,996 |
| IFI27_OAS1_IFIT1_SIGLEC1_MMP8 | 0,868 | 0,859 | 0,878 | 0,888 | 0,821 | 0,956 | 0,913 | 0,837 | 0,989 |
| IFIT1_IFI44L_SLC1A2_IL1R2_OLAH | 0,868 | 0,859 | 0,877 | 0,910 | 0,854 | 0,966 | 0,946 | 0,896 | 0,996 |
| IFI27_OAS1_IFIT1_FAM20A_MMP8 | 0,868 | 0,858 | 0,878 | 0,894 | 0,826 | 0,962 | 0,937 | 0,857 | 1 |
| IFIT1_ISG15_HERC6_IL1R2_RETN | 0,868 | 0,859 | 0,878 | 0,896 | 0,832 | 0,960 | 0,948 | 0,898 | 0,997 |
| IFI27_IFIT1_SIGLEC1_ISG15_MMP8 | 0,868 | 0,859 | 0,878 | 0,888 | 0,821 | 0,956 | 0,911 | 0,835 | 0,987 |
| IFI44L_SIGLEC1_OLAH_RETN_MMP8 | 0,868 | 0,858 | 0,878 | 0,891 | 0,821 | 0,961 | 0,962 | 0,921 | 1 |
| IFI27_OAS1_ISG15_FAM20A_MMP8 | 0,868 | 0,858 | 0,878 | 0,893 | 0,824 | 0,961 | 0,936 | 0,856 | 1 |
| ISG15_HERC6_SLC1A2_IL1R2_OLAH | 0,868 | 0,859 | 0,877 | 0,904 | 0,848 | 0,961 | 0,936 | 0,878 | 0,994 |
| IFIT1_HERC6_SLC1A2_IL1R2_MMP8 | 0,868 | 0,859 | 0,877 | 0,903 | 0,847 | 0,960 | 0,951 | 0,903 | 1,000 |
| IFIT1_IFI44L_ISG15_HERC6_IL1R2 | 0,868 | 0,859 | 0,877 | 0,896 | 0,836 | 0,957 | 0,946 | 0,895 | 0,996 |
| IFI27_IFI44L_SIGLEC1_ISG15_MMP8 | 0,868 | 0,859 | 0,877 | 0,889 | 0,823 | 0,956 | 0,916 | 0,844 | 0,988 |
| RSAD2_IFI27_OAS1_FAM20A_MMP8 | 0,868 | 0,858 | 0,878 | 0,893 | 0,824 | 0,961 | 0,937 | 0,857 | 1 |
| OAS1_IFIT1_HERC6_SLC1A2_OLAH | 0,868 | 0,858 | 0,878 | 0,902 | 0,844 | 0,960 | 0,945 | 0,893 | 0,996 |
| IFI27_SIGLEC1_HERC6_SLC1A2_MMP8 | 0,868 | 0,859 | 0,877 | 0,902 | 0,844 | 0,960 | 0,927 | 0,859 | 0,995 |
| RSAD2_ISG15_HERC6_IL1R2_RETN | 0,868 | 0,859 | 0,877 | 0,897 | 0,834 | 0,960 | 0,948 | 0,898 | 0,997 |
| IFI44L_ISG15_HERC6_IL1R2_MMP8 | 0,868 | 0,859 | 0,877 | 0,895 | 0,836 | 0,954 | 0,943 | 0,893 | 0,994 |
| IFIT1_IFI44L_HERC6_IL1R2_MMP8 | 0,868 | 0,859 | 0,877 | 0,896 | 0,833 | 0,958 | 0,950 | 0,903 | 0,997 |
| OAS1_HERC6_SLC1A2_OLAH_MMP8 | 0,868 | 0,858 | 0,877 | 0,902 | 0,844 | 0,960 | 0,935 | 0,877 | 0,993 |
| OAS1_SIGLEC1_HERC6_RETN_MMP8 | 0,868 | 0,858 | 0,878 | 0,892 | 0,820 | 0,963 | 0,947 | 0,897 | 0,997 |
| IFI27_IFIT1_IFI44L_SIGLEC1_MMP8 | 0,868 | 0,858 | 0,877 | 0,891 | 0,825 | 0,957 | 0,913 | 0,838 | 0,988 |
| RSAD2_OAS1_SIGLEC1_OLAH_MMP8 | 0,868 | 0,858 | 0,877 | 0,892 | 0,825 | 0,960 | 0,948 | 0,899 | 0,997 |

| Modèle | AUC modèle | IC 95% | | AUC Train | IC 95% Train | | AUC Test | IC 95% Test | |
|---|---|---|---|---|---|---|---|---|---|
| IFIT1_ IFI44L_ IL1R2_ FAM20A_ RETN | 0,868 | 0,858 | 0,877 | 0,896 | 0,831 | 0,960 | 0,947 | 0,881 | 1 |
| ISG15_ HERC6_ SLC1A2_ OLAH_ MMP8 | 0,868 | 0,858 | 0,877 | 0,903 | 0,845 | 0,960 | 0,940 | 0,886 | 0,995 |
| RSAD2_ IFI27_ IFIT1_ HERC6_ MMP8 | 0,868 | 0,859 | 0,877 | 0,897 | 0,838 | 0,956 | 0,892 | 0,812 | 0,973 |
| RSAD2_ ISG15_ IL1R2_ FAM20A_ RETN | 0,868 | 0,858 | 0,877 | 0,895 | 0,829 | 0,961 | 0,942 | 0,868 | 1 |
| ISG15_ HERC6_ SLC1A2_ FAM20A_ RETN | 0,868 | 0,858 | 0,878 | 0,899 | 0,835 | 0,963 | 0,940 | 0,860 | 1 |
| OAS1_ IFIT1_ IFI44L_ SLC1A2_ OLAH | 0,868 | 0,858 | 0,878 | 0,908 | 0,851 | 0,965 | 0,936 | 0,881 | 0,990 |
| IFIT1_ ISG15_ HERC6_ SLC1A2_ IL1R2 | 0,868 | 0,859 | 0,877 | 0,903 | 0,846 | 0,959 | 0,951 | 0,902 | 1 |
| IFI44L_ ISG15_ HERC6_ IL1R2_ OLAH | 0,868 | 0,859 | 0,877 | 0,899 | 0,839 | 0,959 | 0,947 | 0,898 | 0,996 |
| RSAD2_ HERC6_ SLC1A2_ OLAH_ MMP8 | 0,868 | 0,858 | 0,877 | 0,901 | 0,843 | 0,960 | 0,939 | 0,883 | 0,995 |
| OAS1_ IFIT1_ IFI44L_ HERC6_ IL1R2 | 0,868 | 0,859 | 0,877 | 0,896 | 0,833 | 0,958 | 0,942 | 0,891 | 0,994 |
| SIGLEC1_ ISG15_ HERC6_ OLAH_ MMP8 | 0,868 | 0,858 | 0,877 | 0,891 | 0,823 | 0,959 | 0,954 | 0,911 | 0,998 |
| IFIT1_ ISG15_ HERC6_ SLC1A2_ OLAH | 0,868 | 0,858 | 0,877 | 0,903 | 0,844 | 0,961 | 0,943 | 0,891 | 0,996 |
| RSAD2_ OAS1_ HERC6_ IL1R2_ OLAH | 0,868 | 0,858 | 0,877 | 0,899 | 0,838 | 0,959 | 0,939 | 0,885 | 0,993 |
| IFIT1_ ISG15_ SLC1A2_ OLAH_ MMP8 | 0,868 | 0,858 | 0,877 | 0,904 | 0,845 | 0,962 | 0,946 | 0,897 | 0,995 |
| IFI27_ OAS1_ SLC1A2_ RETN_ MMP8 | 0,868 | 0,858 | 0,877 | 0,900 | 0,837 | 0,962 | 0,926 | 0,848 | 1 |
| IFIT1_ IL1R2_ OLAH_ FAM20A_ RETN | 0,868 | 0,858 | 0,877 | 0,895 | 0,831 | 0,960 | 0,951 | 0,891 | 1 |
| RSAD2_ IFI27_ SLC1A2_ RETN_ MMP8 | 0,868 | 0,858 | 0,877 | 0,901 | 0,839 | 0,964 | 0,932 | 0,858 | 1 |
| RSAD2_ IFIT1_ IFI44L_ IL1R2_ FAM20A | 0,868 | 0,859 | 0,877 | 0,895 | 0,829 | 0,961 | 0,949 | 0,881 | 1 |
| IFIT1_ IFI44L_ HERC6_ IL1R2_ RETN | 0,868 | 0,858 | 0,877 | 0,893 | 0,826 | 0,960 | 0,946 | 0,897 | 0,995 |
| OAS1_ ISG15_ HERC6_ SLC1A2_ OLAH | 0,868 | 0,858 | 0,877 | 0,902 | 0,844 | 0,959 | 0,941 | 0,888 | 0,995 |
| IFI27_ OAS1_ IFIT1_ SIGLEC1_ SLC1A2 | 0,867 | 0,858 | 0,877 | 0,897 | 0,837 | 0,956 | 0,953 | 0,903 | 1 |
| ISG15_ HERC6_ SLC1A2_ OLAH_ RETN | 0,867 | 0,858 | 0,877 | 0,903 | 0,844 | 0,963 | 0,943 | 0,888 | 0,999 |
| IFI27_ SIGLEC1_ ISG15_ HERC6_ MMP8 | 0,867 | 0,858 | 0,877 | 0,892 | 0,826 | 0,957 | 0,914 | 0,839 | 0,989 |
| RSAD2_ SIGLEC1_ ISG15_ HERC6_ OLAH | 0,867 | 0,858 | 0,877 | 0,897 | 0,834 | 0,960 | 0,948 | 0,900 | 0,995 |
| IFI27_ IFIT1_ HERC6_ SLC1A2_ MMP8 | 0,867 | 0,858 | 0,877 | 0,901 | 0,844 | 0,958 | 0,899 | 0,815 | 0,983 |

EP 4 365 308 A1

| Modèle | AUC modèle | IC 95% | | AUC Train | IC 95% Train | | AUC Test | IC 95% Test | |
|---|---|---|---|---|---|---|---|---|---|
| OAS1_ IFI44L_ SLC1A2_ OLAH_ MMP8 | 0,867 | 0,858 | 0,877 | 0,905 | 0,847 | 0,962 | 0,938 | 0,884 | 0,992 |
| IFI27_ OAS1_ IFIT1_ IFI44L_ FAM20A | 0,867 | 0,857 | 0,878 | 0,894 | 0,825 | 0,964 | 0,941 | 0,862 | 1 |
| SIGLEC1_ ISG15_ OLAH_ RETN_ MMP8 | 0,867 | 0,857 | 0,877 | 0,891 | 0,820 | 0,961 | 0,964 | 0,925 | 1 |
| RSAD2_ IFI44L_ SLC1A2_ OLAH_ MMP8 | 0,867 | 0,858 | 0,877 | 0,905 | 0,847 | 0,963 | 0,937 | 0,882 | 0,991 |
| RSAD2_ IFIT1_ IL1R2_ FAM20A_ RETN | 0,867 | 0,858 | 0,877 | 0,897 | 0,831 | 0,963 | 0,946 | 0,874 | 1 |
| OAS1_ SIGLEC1_ ISG15_ HERC6_ OLAH | 0,867 | 0,858 | 0,877 | 0,892 | 0,827 | 0,956 | 0,954 | 0,911 | 0,998 |
| IFIT1_ SLC1A2_ IL1R2_ OLAH_ RETN | 0,867 | 0,858 | 0,877 | 0,908 | 0,850 | 0,965 | 0,943 | 0,892 | 0,995 |
| IFIT1_ IFI44L_ SIGLEC1_ OLAH_ MMP8 | 0,867 | 0,858 | 0,877 | 0,891 | 0,824 | 0,958 | 0,943 | 0,890 | 0,997 |
| RSAD2_ ISG15_ HERC6_ SLC1A2_ OLAH | 0,867 | 0,858 | 0,876 | 0,901 | 0,843 | 0,959 | 0,940 | 0,885 | 0,995 |
| RSAD2_ IFIT1_ SLC1A2_ IL1R2_ OLAH | 0,867 | 0,858 | 0,876 | 0,905 | 0,847 | 0,963 | 0,947 | 0,897 | 0,997 |
| OAS1_ IFIT1_ OLAH_ FAM20A | 0,867 | 0,857 | 0,877 | 0,893 | 0,822 | 0,964 | 0,945 | 0,879 | 1 |
| IFI27_ HERC6_ SLC1A2_ MMP8 | 0,867 | 0,858 | 0,876 | 0,900 | 0,842 | 0,957 | 0,900 | 0,817 | 0,983 |
| HERC6_ SLC1A2_ OLAH_ RETN | 0,867 | 0,857 | 0,877 | 0,904 | 0,846 | 0,963 | 0,936 | 0,876 | 0,996 |
| ISG15_ IL1R2_ OLAH_ FAM20A_ RETN | 0,867 | 0,857 | 0,877 | 0,901 | 0,839 | 0,962 | 0,927 | 0,851 | 1 |
| IFI27_ OAS1_ IFI44L_ SIGLEC1_ MMP8 | 0,867 | 0,858 | 0,876 | 0,889 | 0,822 | 0,956 | 0,920 | 0,848 | 0,991 |
| OAS1_ HERC6_ SLC1A2_ OLAH_ RETN | 0,867 | 0,857 | 0,877 | 0,902 | 0,843 | 0,961 | 0,933 | 0,871 | 0,994 |
| OAS1_ ISG15_ OLAH_ FAM20A | 0,867 | 0,857 | 0,877 | 0,892 | 0,821 | 0,964 | 0,949 | 0,885 | 1 |
| RSAD2_ IFIT1_ HERC6_ SLC1A2_ IL1R2 | 0,867 | 0,858 | 0,876 | 0,902 | 0,844 | 0,959 | 0,946 | 0,893 | 0,998 |
| IFI44L_ HERC6_ IL1R2_ RETN_ MMP8 | 0,867 | 0,857 | 0,877 | 0,895 | 0,829 | 0,961 | 0,950 | 0,903 | 0,997 |
| RSAD2_ ISG15_ HERC6_ IL1R2_ OLAH | 0,867 | 0,858 | 0,876 | 0,899 | 0,839 | 0,959 | 0,945 | 0,895 | 0,994 |
| ISG15_ SLC1A2_ OLAH_ FAM20A_ MMP8 | 0,867 | 0,857 | 0,877 | 0,901 | 0,843 | 0,960 | 0,921 | 0,842 | 0,999 |
| HERC6_ SLC1A2_ IL1R2_ OLAH_ RETN | 0,867 | 0,858 | 0,876 | 0,904 | 0,846 | 0,961 | 0,932 | 0,867 | 0,996 |
| RSAD2_ OAS1_ IFI44L_ OLAH_ FAM20A | 0,867 | 0,857 | 0,877 | 0,892 | 0,821 | 0,963 | 0,945 | 0,885 | 1 |
| RSAD2_ IFI44L_ SIGLEC1_ HERC6_ OLAH | 0,867 | 0,857 | 0,876 | 0,896 | 0,833 | 0,960 | 0,949 | 0,902 | 0,996 |
| OAS1_ HERC6_ IL1R2_ RETN_ MMP8 | 0,867 | 0,857 | 0,876 | 0,893 | 0,827 | 0,959 | 0,941 | 0,887 | 0,996 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| RSAD2_IFI27_IFIT1_FAM20A_MMP8 | 0,867 | 0,856 | 0,877 | 0,894 | 0,827 | 0,961 | 0,934 | 0,853 | 1 |
| IFI44L_SIGLEC1_ISG15_OLAH_MMP8 | 0,867 | 0,857 | 0,876 | 0,891 | 0,824 | 0,958 | 0,954 | 0,909 | 0,999 |
| OAS1_IFI44L_SLC1A2_OLAH_RETN | 0,867 | 0,857 | 0,877 | 0,909 | 0,851 | 0,966 | 0,936 | 0,881 | 0,991 |
| ISG15_HERC6_IL1R2_OLAH_MMP8 | 0,867 | 0,857 | 0,876 | 0,900 | 0,840 | 0,959 | 0,949 | 0,902 | 0,996 |
| RSAD2_IFIT1_HERC6_IL1R2_MMP8 | 0,867 | 0,858 | 0,876 | 0,896 | 0,834 | 0,958 | 0,949 | 0,902 | 0,996 |
| ISG15_HERC6_IL1R2_OLAH_RETN | 0,867 | 0,857 | 0,877 | 0,899 | 0,835 | 0,963 | 0,951 | 0,905 | 0,997 |
| RSAD2_IFIT1_SIGLEC1_OLAH_RETN | 0,867 | 0,857 | 0,876 | 0,895 | 0,826 | 0,964 | 0,949 | 0,897 | 1 |
| RSAD2_SLC1A2_IL1R2_OLAH_MMP8 | 0,867 | 0,858 | 0,876 | 0,903 | 0,846 | 0,960 | 0,939 | 0,880 | 0,998 |
| OAS1_ISG15_HERC6_IL1R2_MMP8 | 0,867 | 0,858 | 0,876 | 0,896 | 0,837 | 0,955 | 0,941 | 0,889 | 0,994 |
| OAS1_IFIT1_OLAH_FAM20A_MMP8 | 0,867 | 0,857 | 0,877 | 0,893 | 0,823 | 0,964 | 0,953 | 0,895 | 1 |
| OAS1_IFIT1_HERC6_IL1R2_OLAH | 0,867 | 0,857 | 0,876 | 0,899 | 0,837 | 0,960 | 0,946 | 0,896 | 0,995 |
| IFI44L_HERC6_IL1R2_OLAH_MMP8 | 0,867 | 0,857 | 0,876 | 0,899 | 0,840 | 0,958 | 0,951 | 0,904 | 0,998 |
| IFI27_OAS1_IFI44L_SLC1A2_FAM20A | 0,867 | 0,856 | 0,877 | 0,898 | 0,833 | 0,964 | 0,943 | 0,866 | 1 |
| RSAD2_IFIT1_HERC6_SLC1A2_OLAH | 0,867 | 0,857 | 0,876 | 0,902 | 0,844 | 0,960 | 0,940 | 0,885 | 0,995 |
| IFIT1_HERC6_IL1R2_OLAH_MMP8 | 0,867 | 0,857 | 0,876 | 0,899 | 0,839 | 0,959 | 0,955 | 0,912 | 0,999 |
| RSAD2_IFI27_ISG15_FAM20A_MMP8 | 0,867 | 0,856 | 0,877 | 0,894 | 0,826 | 0,962 | 0,934 | 0,853 | 1 |
| OAS1_IFIT1_OLAH_FAM20A_RETN | 0,867 | 0,857 | 0,876 | 0,893 | 0,822 | 0,965 | 0,949 | 0,886 | 1 |
| OAS1_SIGLEC1_OLAH_RETN_MMP8 | 0,867 | 0,856 | 0,877 | 0,892 | 0,821 | 0,962 | 0,964 | 0,925 | 1 |
| OAS1_HERC6_IL1R2_OLAH_MMP8 | 0,867 | 0,857 | 0,876 | 0,900 | 0,840 | 0,959 | 0,940 | 0,886 | 0,995 |
| OAS1_IFIT1_ISG15_OLAH_FAM20A | 0,867 | 0,857 | 0,876 | 0,891 | 0,819 | 0,963 | 0,951 | 0,891 | 1 |
| IFIT1_ISG15_HERC6_IL1R2_OLAH | 0,866 | 0,857 | 0,876 | 0,899 | 0,839 | 0,960 | 0,952 | 0,907 | 0,997 |
| RSAD2_OAS1_OLAH_FAM20A_RETN | 0,866 | 0,856 | 0,877 | 0,894 | 0,823 | 0,965 | 0,948 | 0,885 | 1 |
| ISG15_IL1R2_FAM20A_RETN_MMP8 | 0,866 | 0,858 | 0,875 | 0,901 | 0,842 | 0,960 | 0,921 | 0,836 | 1 |
| IFIT1_HERC6_SLC1A2_OLAH_RETN | 0,866 | 0,856 | 0,876 | 0,903 | 0,843 | 0,962 | 0,937 | 0,877 | 0,997 |
| IFI27_IFI44L_HERC6_SLC1A2_MMP8 | 0,866 | 0,857 | 0,876 | 0,899 | 0,842 | 0,957 | 0,899 | 0,815 | 0,983 |

(suite)

| Modèle | AUC modèle | IC 95% | | AUC Train | IC 95% Train | | AUC Test | IC 95% Test | |
|---|---|---|---|---|---|---|---|---|---|
| HERC6_ IL1R2_ OLAH_ RETN_ MMP8 | 0,866 | 0,856 | 0,877 | 0,899 | 0,836 | 0,962 | 0,953 | 0,909 | 0,997 |
| RSAD2_ IL1R2_ FAM20A_ RETN_ MMP8 | 0,866 | 0,857 | 0,876 | 0,899 | 0,835 | 0,963 | 0,937 | 0,856 | 1 |
| OAS1_ IFIT1_ ISG15_ SLC1A2_ OLAH | 0,866 | 0,857 | 0,876 | 0,905 | 0,847 | 0,962 | 0,941 | 0,889 | 0,993 |
| HERC6_ SLC1A2_ OLAH_ RETN_ MMP8 | 0,866 | 0,857 | 0,876 | 0,902 | 0,842 | 0,962 | 0,943 | 0,890 | 0,997 |
| OAS1_ ISG15_ HERC6_ SLC1A2_ IL1R2 | 0,866 | 0,857 | 0,876 | 0,902 | 0,846 | 0,957 | 0,933 | 0,867 | 0,998 |
| RSAD2_ IFI27_ SIGLEC1_ HERC6_ SLC1A2 | 0,866 | 0,857 | 0,875 | 0,897 | 0,841 | 0,953 | 0,945 | 0,892 | 0,997 |
| RSAD2_ IFIT1_ HERC6_ IL1R2_ RETN | 0,866 | 0,857 | 0,876 | 0,893 | 0,826 | 0,960 | 0,948 | 0,900 | 0,996 |
| IFIT1_ ISG15_ SLC1A2_ OLAH_ RETN | 0,866 | 0,857 | 0,876 | 0,907 | 0,848 | 0,965 | 0,951 | 0,905 | 0,997 |
| ISG15_ HERC6_ SLC1A2_ IL1R2_ RETN | 0,866 | 0,857 | 0,876 | 0,901 | 0,843 | 0,959 | 0,936 | 0,871 | 1 |
| RSAD2_ ISG15_ HERC6_ IL1R2_ MMP8 | 0,866 | 0,857 | 0,875 | 0,898 | 0,840 | 0,957 | 0,941 | 0,889 | 0,994 |
| IFI44L_ HERC6_ IL1R2 _ OLAH_ RETN | 0,866 | 0,856 | 0,876 | 0,898 | 0,833 | 0,963 | 0,948 | 0,899 | 0,996 |
| OAS1_ IFI44L_ HERC6_ IL1R2_ RETN | 0,866 | 0,857 | 0,876 | 0,894 | 0,827 | 0,960 | 0,942 | 0,891 | 0,994 |
| RSAD2_ OAS1_ IFI44L_ SLC1A2_ OLAH | 0,866 | 0,856 | 0,876 | 0,907 | 0,850 | 0,964 | 0,934 | 0,878 | 0,990 |
| IFIT1_ HERC6_ SLC1A2_ IL1R2_ RETN | 0,866 | 0,856 | 0,876 | 0,901 | 0,842 | 0,960 | 0,940 | 0,883 | 0,998 |
| RSAD2_ OAS1_ HERC6_ IL1R2_ RETN | 0,866 | 0,857 | 0,876 | 0,894 | 0,827 | 0,960 | 0,942 | 0,891 | 0,994 |
| RSAD2_ IFI44L_ SLC1A2_ OLAH_ RETN | 0,866 | 0,856 | 0,876 | 0,909 | 0,851 | 0,967 | 0,936 | 0,881 | 0,991 |
| OAS1_ IFIT1_ SLC1A2_ OLAH_ RETN | 0,866 | 0,856 | 0,876 | 0,908 | 0,850 | 0,966 | 0,943 | 0,893 | 0,994 |
| IFI44L_ HERC6_ SLC1A2_ IL1R2_ RETN | 0,866 | 0,857 | 0,876 | 0,901 | 0,843 | 0,960 | 0,933 | 0,867 | 0,998 |
| RSAD2_ IFI44L_ SLC1A2_ IL1R2_ OLAH | 0,866 | 0,856 | 0,876 | 0,905 | 0,848 | 0,963 | 0,941 | 0,887 | 0,995 |
| IFI27_ OAS1_ SIGLEC1_ ISG15_ HERC6 | 0,866 | 0,856 | 0,876 | 0,895 | 0,839 | 0,951 | 0,936 | 0,881 | 0,991 |
| IFIT1_ IFI44L_ SIGLEC1_ OLAH_ RETN | 0,866 | 0,857 | 0,875 | 0,891 | 0,821 | 0,961 | 0,939 | 0,881 | 0,998 |
| RSAD2_ OAS1_ SLC1A2_ IL1R2_ OLAH | 0,866 | 0,857 | 0,875 | 0,904 | 0,847 | 0,962 | 0,938 | 0,880 | 0,996 |
| IFIT1_ SIGLEC1_ ISG15_ OLAH_ MMP8 | 0,866 | 0,857 | 0,875 | 0,891 | 0,825 | 0,958 | 0,950 | 0,902 | 0,998 |
| RSAD2_ IFIT1_ IFI44L_ HERC6_ IL1R2 | 0,866 | 0,857 | 0,875 | 0,896 | 0,833 | 0,958 | 0,943 | 0,893 | 0,994 |
| HERC6_ SLC1A2_ IL1R2_ OLAH_ MMP8 | 0,866 | 0,857 | 0,875 | 0,901 | 0,844 | 0,958 | 0,935 | 0,874 | 0,995 |

133

(suite)

| Modèle | AUC modèle | IC 95% | | AUC Train | IC 95% Train | | AUC Test | IC 95% Test | |
|---|---|---|---|---|---|---|---|---|---|
| RSAD2_ IFIT1_ SIGLEC1_ OLAH_ MMP8 | 0,866 | 0,857 | 0,875 | 0,893 | 0,826 | 0,961 | 0,951 | 0,903 | 0,999 |
| RSAD2_ IFIT1_ SLC1A2_ OLAH_ MMP8 | 0,866 | 0,857 | 0,875 | 0,904 | 0,846 | 0,962 | 0,945 | 0,895 | 0,994 |
| IFI27_ IFIT1_ SLC1A2_ FAM20A_ MMP8 | 0,866 | 0,855 | 0,876 | 0,897 | 0,832 | 0,961 | 0,933 | 0,851 | 1 |
| RSAD2_ IFI44L_ SIGLEC1_ HERC6_ RETN | 0,866 | 0,856 | 0,875 | 0,890 | 0,819 | 0,962 | 0,935 | 0,880 | 0,989 |
| IFIT1_ IFI44L_ SIGLEC1_ ISG15_ OLAH | 0,866 | 0,857 | 0,875 | 0,894 | 0,828 | 0,961 | 0,941 | 0,886 | 0,996 |
| RSAD2_ OAS1_ HERC6_ IL1R2_ MMP8 | 0,866 | 0,857 | 0,875 | 0,895 | 0,833 | 0,956 | 0,942 | 0,891 | 0,994 |
| OAS1_ IFIT1_ SLC1A2_ IL1R2_ MMP8 | 0,866 | 0,857 | 0,875 | 0,904 | 0,847 | 0,961 | 0,951 | 0,904 | 0,998 |
| ISG15_ HERC6_ FAM20A_ RETN_ MMP8 | 0,866 | 0,855 | 0,876 | 0,895 | 0,826 | 0,963 | 0,942 | 0,869 | 1 |
| RSAD2_ OAS1_ IFIT1_ SLC1A2_ OLAH | 0,866 | 0,856 | 0,876 | 0,904 | 0,846 | 0,962 | 0,941 | 0,889 | 0,993 |
| RSAD2_ OAS1_ ISG15_ HERC6_ IL1R2 | 0,866 | 0,857 | 0,875 | 0,897 | 0,839 | 0,955 | 0,943 | 0,891 | 0,996 |
| OAS1_ ISG15_ HERC6_ OLAH_ RETN | 0,866 | 0,856 | 0,876 | 0,895 | 0,830 | 0,960 | 0,954 | 0,910 | 0,999 |
| RSAD2_ IFIT1_ IFI44L_ SLC1A2_ OLAH | 0,866 | 0,856 | 0,875 | 0,907 | 0,850 | 0,965 | 0,935 | 0,880 | 0,990 |
| OAS1_ IFI44L_ SLC1A2_ IL1R2_ OLAH | 0,866 | 0,856 | 0,876 | 0,907 | 0,850 | 0,964 | 0,939 | 0,883 | 0,995 |
| RSAD2_ ISG15_ HERC6_ SLC1A2_ IL1R2 | 0,866 | 0,857 | 0,875 | 0,899 | 0,843 | 0,956 | 0,937 | 0,874 | 1 |
| OAS1_ IFI44L_ ISG15_ SLC1A2 _ OLAH | 0,866 | 0,856 | 0,876 | 0,907 | 0,850 | 0,964 | 0,934 | 0,878 | 0,990 |
| IFIT1_ IFI44L_ HERC6_ IL1R2_ OLAH | 0,866 | 0,857 | 0,875 | 0,899 | 0,838 | 0,960 | 0,946 | 0,896 | 0,995 |
| RSAD2_ HERC6_ IL1R2_ OLAH_ MMP8 | 0,866 | 0,856 | 0,875 | 0,898 | 0,839 | 0,958 | 0,947 | 0,897 | 0,996 |
| RSAD2_ SLC1A2_ IL1R2_ OLAH_ RETN | 0,866 | 0,856 | 0,875 | 0,905 | 0,847 | 0,962 | 0,937 | 0,877 | 0,997 |
| RSAD2_ OAS1_ SIGLEC1_ OLAH_ RETN | 0,866 | 0,856 | 0,876 | 0,890 | 0,820 | 0,961 | 0,948 | 0,898 | 0,998 |
| OAS1_ IFI44L_ HERC6_ IL1R2 _ OLAH | 0,866 | 0,856 | 0,875 | 0,899 | 0,838 | 0,959 | 0,946 | 0,895 | 0,996 |
| RSAD2_ HERC6_ IL1R2_ RETN_ MMP8 | 0,866 | 0,856 | 0,875 | 0,894 | 0,827 | 0,960 | 0,945 | 0,891 | 0,998 |
| RSAD2_ HERC6_ IL1R2_ OLAH_ RETN | 0,866 | 0,856 | 0,875 | 0,896 | 0,831 | 0,961 | 0,945 | 0,894 | 0,995 |
| OAS1_ IFI44L_ HERC6_ IL1R2_ MMP8 | 0,866 | 0,857 | 0,874 | 0,895 | 0,833 | 0,956 | 0,941 | 0,889 | 0,994 |
| RSAD2_ IFI27_ OAS1_ SIGLEC1_ HERC6 | 0,866 | 0,856 | 0,875 | 0,897 | 0,842 | 0,951 | 0,921 | 0,857 | 0,984 |
| OAS1_ ISG15_ HERC6_ IL1R2_ OLAH | 0,866 | 0,856 | 0,875 | 0,899 | 0,838 | 0,959 | 0,945 | 0,895 | 0,994 |

(suite)

| Modèle | AUC modèle | IC 95% | | AUC Train | IC 95% Train | | AUC Test | IC 95% Test | |
|---|---|---|---|---|---|---|---|---|---|
| IFI44L_ ISG15_ HERC6_ SLC1A2_ IL1R2 | 0,866 | 0,857 | 0,875 | 0,901 | 0,845 | 0,956 | 0,939 | 0,880 | 0,998 |
| OAS1_ IFI44L_ SIGLEC1_ OLAH_ MMP8 | 0,866 | 0,856 | 0,875 | 0,892 | 0,823 | 0,960 | 0,955 | 0,912 | 0,999 |
| IFIT1_ HERC6_ IL1R2_ OLAH_ RETN | 0,866 | 0,856 | 0,875 | 0,897 | 0,833 | 0,962 | 0,947 | 0,897 | 0,996 |
| IFI44L_ ISG15_ OLAH_ FAM20A_ MMP8 | 0,865 | 0,856 | 0,875 | 0,890 | 0,818 | 0,961 | 0,958 | 0,910 | 1 |
| RSAD2_ OAS1_ SLC1A2_ OLAH_ MMP8 | 0,865 | 0,856 | 0,875 | 0,900 | 0,842 | 0,959 | 0,939 | 0,885 | 0,994 |
| IFI27_ OAS1_ SIGLEC1_ ISG15_ MMP8 | 0,865 | 0,856 | 0,875 | 0,890 | 0,824 | 0,956 | 0,921 | 0,848 | 0,993 |
| IFIT1_ ISG15_ HERC6_ OLAH_ RETN | 0,865 | 0,856 | 0,875 | 0,893 | 0,825 | 0,961 | 0,945 | 0,895 | 0,994 |
| OAS1_ SLC1A2_ IL1R2_ OLAH_ MMP8 | 0,865 | 0,856 | 0,875 | 0,905 | 0,848 | 0,962 | 0,930 | 0,868 | 0,993 |
| IFI27_ OAS1_ SIGLEC1_ ISG15_ SLC1A2 | 0,865 | 0,856 | 0,875 | 0,896 | 0,837 | 0,956 | 0,957 | 0,908 | 1 |
| OAS1_ IFIT1_ ISG15_ SLC1A2_ IL1R2 | 0,865 | 0,856 | 0,874 | 0,903 | 0,846 | 0,961 | 0,948 | 0,898 | 0,998 |
| RSAD2_ IFI44L_ ISG15_ SLC1A2_ OLAH | 0,865 | 0,856 | 0,875 | 0,906 | 0,849 | 0,964 | 0,935 | 0,879 | 0,990 |
| IFIT1_ IFI44L_ SLC1A2_ IL1R2_ MMP8 | 0,865 | 0,857 | 0,874 | 0,904 | 0,848 | 0,960 | 0,953 | 0,907 | 0,999 |
| RSAD2_ SIGLEC1_ HERC6_ RETN_ MMP8 | 0,865 | 0,855 | 0,875 | 0,889 | 0,817 | 0,962 | 0,938 | 0,885 | 0,991 |
| OAS1_ ISG15_ SLC1A2_ IL1R2_ OLAH | 0,865 | 0,856 | 0,875 | 0,907 | 0,850 | 0,965 | 0,928 | 0,865 | 0,992 |
| IFIT1_ IFI44L_ HERC6_ SLC1A2_ IL1R2 | 0,865 | 0,856 | 0,874 | 0,902 | 0,845 | 0,960 | 0,947 | 0,896 | 0,998 |
| RSAD2_ ISG15_ SLC1A2_ IL1R2_ OLAH | 0,865 | 0,856 | 0,874 | 0,904 | 0,846 | 0,962 | 0,938 | 0,880 | 0,996 |
| IFI44L_ ISG15_ SLC1A2_ IL1R2_ RETN | 0,865 | 0,856 | 0,874 | 0,902 | 0,846 | 0,958 | 0,943 | 0,889 | 0,998 |
| OAS1_ HERC6_ IL1R2_ OLAH_ RETN | 0,865 | 0,855 | 0,875 | 0,896 | 0,831 | 0,961 | 0,942 | 0,891 | 0,994 |
| RSAD2_ IFIT1_ HERC6_ IL1R2 _ OLAH | 0,865 | 0,856 | 0,875 | 0,898 | 0,837 | 0,959 | 0,946 | 0,896 | 0,995 |
| RSAD2_ IFI44L_ ISG15_ HERC6_ IL1R2 | 0,865 | 0,856 | 0,874 | 0,896 | 0,838 | 0,955 | 0,943 | 0,892 | 0,995 |
| IFIT1_ SIGLEC1_ ISG15_ OLAH_ RETN | 0,865 | 0,856 | 0,875 | 0,892 | 0,822 | 0,961 | 0,946 | 0,891 | 1 |
| IFI27_ ISG15_ HERC6_ SLC1A2_ MMP8 | 0,865 | 0,856 | 0,874 | 0,900 | 0,843 | 0,957 | 0,907 | 0,828 | 0,985 |
| IFI44L_ SIGLEC1_ ISG15_ HERC6_ RETN | 0,865 | 0,855 | 0,875 | 0,884 | 0,808 | 0,959 | 0,951 | 0,904 | 0,998 |
| IFIT1_ SLC1A2_ OLAH_ RETN_ MMP8 | 0,865 | 0,855 | 0,875 | 0,901 | 0,840 | 0,962 | 0,953 | 0,909 | 0,997 |
| RSAD2_ IFI27_ OAS1_ SIGLEC1_ MMP8 | 0,865 | 0,855 | 0,875 | 0,890 | 0,825 | 0,956 | 0,921 | 0,848 | 0,993 |

(suite)

| Modèle | AUC modèle | IC 95% | | AUC Train | IC 95% Train | | AUC Test | IC 95% Test | |
|---|---|---|---|---|---|---|---|---|---|
| RSAD2_ OAS1_ IFI44L_ HERC6_ IL1R2 | 0,865 | 0,856 | 0,874 | 0,894 | 0,832 | 0,955 | 0,942 | 0,891 | 0,994 |
| RSAD2_ IFI27_ OAS1_ ISG15_ FAM20A | 0,865 | 0,855 | 0,875 | 0,883 | 0,809 | 0,957 | 0,940 | 0,861 | 1 |
| IFI27_ OAS1_ SLC1A2_ FAM20A_ MMP8 | 0,865 | 0,855 | 0,875 | 0,896 | 0,831 | 0,961 | 0,929 | 0,846 | 1 |
| RSAD2_ IFI44L_ HERC6_ IL1R2_ RETN | 0,865 | 0,855 | 0,874 | 0,893 | 0,827 | 0,960 | 0,943 | 0,893 | 0,994 |
| IFI44L_ HERC6_ SLC1A2_ FAM20A_ RETN | 0,865 | 0,855 | 0,875 | 0,894 | 0,828 | 0,960 | 0,939 | 0,861 | 1 |
| OAS1_ IFI44L_ ISG15_ HERC6_ IL1R2 | 0,865 | 0,856 | 0,874 | 0,897 | 0,839 | 0,955 | 0,946 | 0,895 | 0,996 |
| OAS1_ IFIT1_ IFI44L_ OLAH_ FAM20A | 0,865 | 0,855 | 0,875 | 0,890 | 0,818 | 0,963 | 0,949 | 0,893 | 1 |
| IFIT1_ ISG15_ SLC1A2_ IL1R2_ RETN | 0,865 | 0,856 | 0,874 | 0,899 | 0,839 | 0,959 | 0,951 | 0,904 | 0,998 |
| IFI27_ OAS1_ IFIT1_ SIGLEC1_ HERC6 | 0,865 | 0,856 | 0,874 | 0,898 | 0,843 | 0,953 | 0,933 | 0,876 | 0,989 |
| ISG15_ HERC6_ SLC1A2_ IL1R2_ MMP8 | 0,865 | 0,856 | 0,874 | 0,901 | 0,845 | 0,956 | 0,932 | 0,866 | 0,997 |
| IFI44L_ SIGLEC1_ ISG15_ OLAH_ RETN | 0,865 | 0,855 | 0,875 | 0,890 | 0,820 | 0,961 | 0,948 | 0,896 | 1,000 |
| IFIT1_ ISG15_ SLC1A2_ IL1R2_ MMP8 | 0,865 | 0,857 | 0,873 | 0,904 | 0,848 | 0,961 | 0,952 | 0,906 | 0,999 |
| ISG15_ SLC1A2_ OLAH_ FAM20A_ RETN | 0,865 | 0,855 | 0,875 | 0,903 | 0,844 | 0,962 | 0,913 | 0,831 | 0,995 |
| RSAD2_ IFI27_ SLC1A2_ FAM20A_ MMP8 | 0,865 | 0,855 | 0,875 | 0,896 | 0,831 | 0,960 | 0,932 | 0,850 | 1 |
| RSAD2_ SIGLEC1_ OLAH_ RETN_ MMP8 | 0,865 | 0,855 | 0,875 | 0,892 | 0,822 | 0,962 | 0,963 | 0,922 | 1 |
| RSAD2_ IFIT1_ IFI44L_ SIGLEC1_ OLAH | 0,865 | 0,856 | 0,874 | 0,895 | 0,827 | 0,962 | 0,948 | 0,897 | 0,999 |
| RSAD2_ OAS1_ HERC6_ SLC1A2_ IL1R2 | 0,865 | 0,856 | 0,874 | 0,898 | 0,841 | 0,955 | 0,935 | 0,870 | 1,000 |
| IFIT1_ IFI44L_ HERC6_ OLAH_ RETN | 0,865 | 0,855 | 0,874 | 0,892 | 0,823 | 0,961 | 0,938 | 0,884 | 0,992 |
| RSAD2_ IFI27_ OAS1_ IFIT1_ FAM20A | 0,865 | 0,855 | 0,875 | 0,887 | 0,816 | 0,958 | 0,942 | 0,864 | 1 |
| IFI44L_ HERC6_ SLC1A2_ IL1R2_ MMP8 | 0,865 | 0,856 | 0,874 | 0,900 | 0,844 | 0,955 | 0,933 | 0,871 | 0,994 |
| RSAD2_ IFI27_ IFI44L_ SIGLEC1_ MMP8 | 0,865 | 0,855 | 0,874 | 0,891 | 0,825 | 0,957 | 0,915 | 0,842 | 0,988 |
| OAS1_ OLAH_ FAM20A_ RETN_ MMP8 | 0,865 | 0,854 | 0,875 | 0,891 | 0,820 | 0,963 | 0,957 | 0,901 | 1 |
| RSAD2_ IFI27_ SIGLEC1_ ISG15_ HERC6 | 0,865 | 0,855 | 0,874 | 0,894 | 0,838 | 0,950 | 0,920 | 0,854 | 0,985 |
| IFI27_ ISG15_ SLC1A2_ FAM20A_ MMP8 | 0,865 | 0,855 | 0,875 | 0,896 | 0,830 | 0,962 | 0,925 | 0,842 | 1 |
| RSAD2_ IFI44L_ HERC6_ IL1R2_ OLAH | 0,865 | 0,855 | 0,874 | 0,897 | 0,836 | 0,958 | 0,947 | 0,897 | 0,996 |

136

(suite)

| Modèle | AUC modèle | IC 95% | | AUC Train | IC 95% Train | | AUC Test | IC 95% Test | |
|---|---|---|---|---|---|---|---|---|---|
| ISG15_ HERC6_ OLAH_ RETN_ MMP8 | 0,865 | 0,854 | 0,875 | 0,892 | 0,822 | 0,962 | 0,961 | 0,922 | 1,000 |
| RSAD2_ ISG15_ HERC6_ OLAH_ RETN | 0,865 | 0,855 | 0,874 | 0,894 | 0,828 | 0,961 | 0,946 | 0,897 | 0,994 |
| IFIT1_IFI44L_ISG15_SLC1A2_IL1R2 | 0,865 | 0,856 | 0,873 | 0,900 | 0,842 | 0,958 | 0,948 | 0,898 | 0,998 |
| IFI27_OAS1_IFI44L_SIGLEC1_SLC1A2 | 0,865 | 0,856 | 0,873 | 0,896 | 0,836 | 0,955 | 0,953 | 0,903 | 1 |
| IFI44L_ISG15_HERC6_OLAH_RETN | 0,864 | 0,855 | 0,874 | 0,891 | 0,820 | 0,961 | 0,953 | 0,909 | 0,998 |
| RSAD2_IFIT1_SLC1A2_IL1R2_MMP8 | 0,864 | 0,856 | 0,873 | 0,902 | 0,844 | 0,960 | 0,954 | 0,909 | 1,000 |
| IFIT1_HERC6_OLAH_RETN_MMP8 | 0,864 | 0,854 | 0,875 | 0,892 | 0,822 | 0,961 | 0,958 | 0,917 | 0,999 |
| IFI44L_HERC6_OLAH_RETN_MMP8 | 0,864 | 0,854 | 0,875 | 0,890 | 0,818 | 0,961 | 0,958 | 0,916 | 0,999 |
| IFI27_OAS1_IFI44L_SIGLEC1_HERC6 | 0,864 | 0,855 | 0,873 | 0,897 | 0,842 | 0,953 | 0,932 | 0,874 | 0,989 |
| OAS1_SLC1A2_IL1R2_OLAH_RETN | 0,864 | 0,855 | 0,874 | 0,907 | 0,849 | 0,964 | 0,932 | 0,870 | 0,993 |
| OAS1_HERC6_SLC1A2_IL1R2_MMP8 | 0,864 | 0,855 | 0,873 | 0,900 | 0,843 | 0,957 | 0,925 | 0,857 | 0,993 |
| RSAD2_HERC6_SLC1A2_IL1R2_MMP8 | 0,864 | 0,855 | 0,873 | 0,898 | 0,840 | 0,956 | 0,933 | 0,867 | 0,999 |
| OAS1_HERC6_SLC1A2_IL1R2_RETN | 0,864 | 0,855 | 0,874 | 0,899 | 0,840 | 0,958 | 0,922 | 0,849 | 0,994 |
| OAS1_IFI44L_SIGLEC1_OLAH_RETN | 0,864 | 0,854 | 0,874 | 0,891 | 0,820 | 0,961 | 0,952 | 0,905 | 1,000 |
| OAS1_IFI44L_OLAH_FAM20A_MMP8 | 0,864 | 0,854 | 0,875 | 0,891 | 0,819 | 0,962 | 0,953 | 0,898 | 1 |
| RSAD2_IFI44L_IL1R2_FAM20A_RETN | 0,864 | 0,855 | 0,874 | 0,894 | 0,828 | 0,960 | 0,945 | 0,873 | 1 |
| RSAD2_IFIT1_SIGLEC1_ISG15_OLAH | 0,864 | 0,855 | 0,873 | 0,894 | 0,827 | 0,962 | 0,947 | 0,896 | 0,998 |
| ISG15_SLC1A2_IL1R2_OLAH_RETN | 0,864 | 0,855 | 0,873 | 0,899 | 0,840 | 0,957 | 0,921 | 0,846 | 0,995 |
| OAS 1_HERC6_OLAH_RETN_MMP8 | 0,864 | 0,854 | 0,874 | 0,892 | 0,822 | 0,961 | 0,955 | 0,913 | 0,998 |
| IFIT1_SIGLEC1_HERC6_ SLC1A2_ MMP8 | 0,864 | 0,855 | 0,873 | 0,893 | 0,833 | 0,954 | 0,909 | 0,838 | 0,980 |
| OAS1_ISG15_OLAH_FAM20A_RETN | 0,864 | 0,854 | 0,874 | 0,893 | 0,822 | 0,964 | 0,952 | 0,894 | 1 |
| OAS1_IFI44L_HERC6_FAM20A_RETN | 0,864 | 0,854 | 0,874 | 0,892 | 0,822 | 0,962 | 0,942 | 0,867 | 1 |
| RSAD2_IFI27_IFIT1_ISG15_FAM20A | 0,864 | 0,854 | 0,874 | 0,890 | 0,820 | 0,960 | 0,940 | 0,861 | 1 |
| RSAD2_IFI44L_HERC6_IL1R2_MMP8 | 0,864 | 0,855 | 0,873 | 0,894 | 0,833 | 0,955 | 0,942 | 0,891 | 0,994 |
| OAS1_ISG15_OLAH_FAM20A_MMP8 | 0,864 | 0,854 | 0,874 | 0,894 | 0,824 | 0,965 | 0,950 | 0,886 | 1 |

| Modèle | AUC modèle | IC 95% | | AUC Train | IC 95% Train | | AUC Test | IC 95% Test | |
|---|---|---|---|---|---|---|---|---|---|
| OAS1_ISG15_SLC1A2_OLAH_MMP8 | 0,864 | 0,855 | 0,873 | 0,902 | 0,844 | 0,960 | 0,932 | 0,872 | 0,991 |
| OAS1_IFIT1_SLC1A2_IL1R2_RETN | 0,864 | 0,855 | 0,873 | 0,899 | 0,839 | 0,959 | 0,949 | 0,900 | 0,997 |
| RSAD2_IFIT1_SLC1A2_OLAH_RETN | 0,864 | 0,854 | 0,873 | 0,905 | 0,846 | 0,965 | 0,945 | 0,894 | 0,995 |
| RSAD2_OAS1_HERC6_FAM20A_RETN | 0,864 | 0,854 | 0,874 | 0,886 | 0,815 | 0,957 | 0,934 | 0,855 | 1 |
| RSAD2_IFI44L_HERC6_FAM20A_RETN | 0,864 | 0,854 | 0,874 | 0,895 | 0,828 | 0,962 | 0,929 | 0,850 | 1 |
| OAS1_IFIT1_HERC6_FAM20A_RETN | 0,864 | 0,854 | 0,874 | 0,889 | 0,818 | 0,960 | 0,936 | 0,857 | 1 |
| RSAD2_HERC6_OLAH_RETN_MMP8 | 0,864 | 0,854 | 0,874 | 0,890 | 0,821 | 0,960 | 0,954 | 0,911 | 0,998 |
| OAS1_IFIT1_HERC6_OLAH_RETN | 0,864 | 0,854 | 0,874 | 0,890 | 0,820 | 0,959 | 0,940 | 0,888 | 0,993 |
| RSAD2_IFI27_OAS1_SIGLEC1_SLC1A2 | 0,864 | 0,854 | 0,873 | 0,896 | 0,837 | 0,955 | 0,951 | 0,900 | 1 |
| RSAD2_IFIT1_ISG15_SLC1A2_OLAH | 0,864 | 0,854 | 0,873 | 0,902 | 0,843 | 0,961 | 0,943 | 0,893 | 0,994 |
| IFI27_IFIT1_SIGLEC1_ISG15_SLC1A2 | 0,864 | 0,855 | 0,873 | 0,895 | 0,836 | 0,954 | 0,953 | 0,905 | 1 |
| RSAD2_HERC6_ SLC1A2_ IL1R2_ RETN | 0,864 | 0,854 | 0,873 | 0,899 | 0,840 | 0,958 | 0,932 | 0,865 | 0,998 |
| RSAD2_IFIT1_ISG15_SLC1A2_IL1R2 | 0,864 | 0,855 | 0,872 | 0,900 | 0,842 | 0,959 | 0,948 | 0,898 | 0,998 |
| OAS1_IFI44L_HERC6_SLC1A2_IL1R2 | 0,864 | 0,854 | 0,873 | 0,900 | 0,844 | 0,956 | 0,935 | 0,874 | 0,996 |
| RSAD2_OAS1_HERC6_OLAH_RETN | 0,863 | 0,854 | 0,873 | 0,889 | 0,819 | 0,959 | 0,942 | 0,891 | 0,993 |
| RSAD2_ISG15_SLC1A2_OLAH_MMP8 | 0,863 | 0,854 | 0,873 | 0,900 | 0,842 | 0,959 | 0,936 | 0,880 | 0,992 |
| IFIT1_IFI44L_SLC1A2_IL1R2_RETN | 0,863 | 0,854 | 0,873 | 0,899 | 0,840 | 0,959 | 0,945 | 0,893 | 0,996 |
| RSAD2_IFI27_IFIT1_SIGLEC1_MMP8 | 0,863 | 0,854 | 0,873 | 0,889 | 0,822 | 0,956 | 0,911 | 0,835 | 0,987 |
| RSAD2_OAS1_IFIT1_SLC1A2_IL1R2 | 0,863 | 0,854 | 0,872 | 0,900 | 0,841 | 0,959 | 0,949 | 0,899 | 0,999 |
| IFI27_OAS1_IFIT1_ISG15_FAM20A | 0,863 | 0,853 | 0,874 | 0,886 | 0,815 | 0,957 | 0,945 | 0,866 | 1 |
| IFIT1_IFI44L_OLAH_FAM20A_MMP8 | 0,863 | 0,853 | 0,873 | 0,888 | 0,817 | 0,959 | 0,958 | 0,911 | 1 |
| RSAD2_OAS1_SLC1A2_OLAH_RETN | 0,863 | 0,854 | 0,873 | 0,903 | 0,844 | 0,962 | 0,940 | 0,886 | 0,994 |
| IFI44L_ISG15_SLC1A2_IL1R2_MMP8 | 0,863 | 0,855 | 0,871 | 0,899 | 0,843 | 0,954 | 0,939 | 0,878 | 1 |
| RSAD2_OAS1_SIGLEC1_ISG15_OLAH | 0,863 | 0,854 | 0,873 | 0,892 | 0,823 | 0,960 | 0,945 | 0,893 | 0,996 |
| OAS1_IFI44L_ISG15_OLAH_FAM20A | 0,863 | 0,853 | 0,873 | 0,890 | 0,817 | 0,963 | 0,953 | 0,898 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_IFIT1_ISG15_SLC1A2_FAM20A | 0,863 | 0,853 | 0,874 | 0,889 | 0,820 | 0,958 | 0,941 | 0,862 | 1 |
| IFI44L_SIGLEC1_HERC6_SLC1A2_RETN | 0,863 | 0,854 | 0,872 | 0,893 | 0,827 | 0,960 | 0,951 | 0,903 | 0,999 |
| IFI27_IFI44L_SIGLEC1_ISG15_SLC1A2 | 0,863 | 0,854 | 0,872 | 0,895 | 0,835 | 0,955 | 0,953 | 0,904 | 1 |
| IFI44L_ISG15_OLAH_FAM20A_RETN | 0,863 | 0,853 | 0,873 | 0,891 | 0,821 | 0,962 | 0,958 | 0,912 | 1 |
| RSAD2_ISG15_SLC1A2_OLAH_RETN | 0,863 | 0,853 | 0,873 | 0,903 | 0,844 | 0,961 | 0,941 | 0,888 | 0,995 |
| OAS1_ISG15_SLC1A2_OLAH_RETN | 0,863 | 0,853 | 0,873 | 0,903 | 0,845 | 0,962 | 0,937 | 0,880 | 0,994 |
| RSAD2_IFI27_IFI44L_SIGLEC1_HERC6 | 0,863 | 0,854 | 0,872 | 0,896 | 0,840 | 0,951 | 0,912 | 0,843 | 0,981 |
| IFIT1_HERC6_SLC1A2_FAM20A_RETN | 0,863 | 0,853 | 0,873 | 0,897 | 0,832 | 0,961 | 0,939 | 0,858 | 1 |
| RSAD2_IFI44L_HERC6_SLC1A2_IL1R2 | 0,863 | 0,854 | 0,872 | 0,900 | 0,843 | 0,956 | 0,936 | 0,874 | 0,998 |
| RSAD2_IFI44L_SIGLEC1_OLAH_MMP8 | 0,863 | 0,853 | 0,873 | 0,891 | 0,823 | 0,959 | 0,957 | 0,914 | 0,999 |
| OAS1_IFIT1_IL1R2_OLAH_MMP8 | 0,863 | 0,853 | 0,872 | 0,894 | 0,831 | 0,958 | 0,960 | 0,920 | 1,000 |
| RSAD2_OAS1_ISG15_SLC1A2_OLAH | 0,863 | 0,853 | 0,872 | 0,903 | 0,845 | 0,961 | 0,934 | 0,876 | 0,992 |
| RSAD2_IFI27_SIGLEC1_ISG15_MMP8 | 0,863 | 0,853 | 0,872 | 0,888 | 0,821 | 0,955 | 0,914 | 0,840 | 0,988 |
| IFI27_IFIT1_SIGLEC1_HERC6_SLC1A2 | 0,863 | 0,854 | 0,872 | 0,898 | 0,840 | 0,955 | 0,950 | 0,902 | 0,998 |
| RSAD2_SLC1A2_OLAH_RETN_MMP8 | 0,863 | 0,853 | 0,872 | 0,900 | 0,840 | 0,960 | 0,946 | 0,895 | 0,996 |
| ISG15_SLC1A2_IL1R2_OLAH_MMP8 | 0,863 | 0,854 | 0,872 | 0,896 | 0,838 | 0,954 | 0,926 | 0,856 | 0,997 |
| RSAD2_OAS1_IFI44L_SIGLEC1_OLAH | 0,863 | 0,853 | 0,872 | 0,892 | 0,824 | 0,961 | 0,946 | 0,895 | 0,996 |
| IFIT1_SLC1A2_IL1R2_RETN_MMP8 | 0,863 | 0,853 | 0,872 | 0,903 | 0,845 | 0,961 | 0,959 | 0,917 | 1 |
| OAS1_IFI44L_SIGLEC1_ISG15_OLAH | 0,863 | 0,853 | 0,873 | 0,892 | 0,824 | 0,961 | 0,943 | 0,892 | 0,995 |
| OAS1_SIGLEC1_ISG15_OLAH_RETN | 0,863 | 0,852 | 0,872 | 0,890 | 0,820 | 0,961 | 0,949 | 0,898 | 1 |
| OAS1_IFIT1_IFI44L_SLC1A2_IL1R2 | 0,863 | 0,853 | 0,873 | 0,901 | 0,843 | 0,959 | 0,948 | 0,898 | 0,997 |
| IFI44L_OLAH_FAM20A_RETN_MMP8 | 0,863 | 0,852 | 0,872 | 0,894 | 0,826 | 0,962 | 0,960 | 0,917 | 1 |
| SIGLEC1_ISG15_HERC6_SLC1A2_RETN | 0,862 | 0,853 | 0,872 | 0,893 | 0,826 | 0,960 | 0,960 | 0,916 | 1 |
| OAS1_SIGLEC1_ISG15_OLAH_MMP8 | 0,862 | 0,853 | 0,872 | 0,890 | 0,821 | 0,959 | 0,951 | 0,904 | 0,998 |
| OAS1_IFI44L_HERC6_OLAH_RETN | 0,862 | 0,852 | 0,872 | 0,890 | 0,820 | 0,960 | 0,946 | 0,897 | 0,995 |

139

| Modèle | AUC modèle | IC 95% | | AUC Train | IC 95% Train | | AUC Test | IC 95% Test | |
|---|---|---|---|---|---|---|---|---|---|
| RSAD2_SIGLEC1_ISG15_OLAH_MMP8 | 0,862 | 0,853 | 0,872 | 0,891 | 0,823 | 0,959 | 0,952 | 0,906 | 0,998 |
| HERC6_SLC1A2_IL1R2_RETN_MMP8 | 0,862 | 0,853 | 0,872 | 0,899 | 0,841 | 0,957 | 0,922 | 0,847 | 0,997 |
| RSAD2_OAS1_IFIT1_IL1R2_OLAH | 0,862 | 0,853 | 0,872 | 0,893 | 0,826 | 0,960 | 0,946 | 0,896 | 0,995 |
| RSAD2_OAS1_IFIT1_IL1R2_MMP8 | 0,862 | 0,853 | 0,872 | 0,892 | 0,827 | 0,956 | 0,954 | 0,911 | 0,998 |
| OAS1_HERC6_SLC1A2_FAM20A_RETN | 0,862 | 0,852 | 0,872 | 0,896 | 0,832 | 0,961 | 0,937 | 0,856 | 1 |
| IFI27_OAS1_ISG15_SLC1A2_FAM20A | 0,862 | 0,852 | 0,873 | 0,887 | 0,817 | 0,957 | 0,936 | 0,854 | 1 |
| IFI27_IFI44L_ISG15_SLC1A2_MMP8 | 0,862 | 0,853 | 0,872 | 0,894 | 0,832 | 0,956 | 0,915 | 0,843 | 0,987 |
| IFIT1_IFI44L_ISG15_OLAH_FAM20A | 0,862 | 0,852 | 0,872 | 0,889 | 0,817 | 0,961 | 0,957 | 0,910 | 1 |
| RSAD2_IFI27_SIGLEC1_ISG15_SLC1A2 | 0,862 | 0,853 | 0,871 | 0,895 | 0,836 | 0,955 | 0,952 | 0,903 | 1 |
| OAS1_IFI44L_OLAH_FAM20A_RETN | 0,862 | 0,852 | 0,872 | 0,891 | 0,818 | 0,963 | 0,954 | 0,901 | 1 |
| RSAD2_SIGLEC1_ISG15_OLAH_RETN | 0,862 | 0,852 | 0,872 | 0,890 | 0,819 | 0,961 | 0,950 | 0,899 | 1 |
| RSAD2_IFI44L_SIGLEC1_OLAH_RETN | 0,862 | 0,852 | 0,872 | 0,890 | 0,819 | 0,961 | 0,950 | 0,901 | 0,999 |
| RSAD2_IFIT1_SLC1A2_IL1R2_RETN | 0,862 | 0,853 | 0,871 | 0,896 | 0,835 | 0,957 | 0,946 | 0,893 | 0,998 |
| OAS1_IFIT1_IFI44L_IL1R2_MMP8 | 0,862 | 0,853 | 0,871 | 0,892 | 0,828 | 0,957 | 0,954 | 0,911 | 0,998 |
| OAS1_IFIT1_SIGLEC1_SLC1A2_RETN | 0,862 | 0,853 | 0,871 | 0,894 | 0,832 | 0,957 | 0,947 | 0,897 | 0,996 |
| IFI44L_SLC1A2_IL1R2_RETN_MMP8 | 0,862 | 0,853 | 0,871 | 0,901 | 0,846 | 0,957 | 0,943 | 0,885 | 1 |
| OAS1_SLC1A2_OLAH_RETN_MMP8 | 0,862 | 0,852 | 0,871 | 0,903 | 0,844 | 0,962 | 0,940 | 0,886 | 0,994 |
| RSAD2_IFI27_IFI44L_SIGLEC1_SLC1A2 | 0,862 | 0,853 | 0,871 | 0,897 | 0,838 | 0,955 | 0,952 | 0,904 | 1 |
| OAS1_IFI44L_ISG15_HERC6_RETN | 0,862 | 0,851 | 0,872 | 0,891 | 0,823 | 0,960 | 0,936 | 0,877 | 0,995 |
| RSAD2_HERC6_SLC1A2_FAM20A_RETN | 0,862 | 0,852 | 0,872 | 0,897 | 0,832 | 0,962 | 0,939 | 0,858 | 1 |
| OAS1_IFIT1_ISG15_HERC6_RETN | 0,862 | 0,851 | 0,872 | 0,890 | 0,822 | 0,959 | 0,939 | 0,883 | 0,995 |
| OAS1_ISG15_HERC6_SLC1A2_RETN | 0,862 | 0,852 | 0,872 | 0,895 | 0,831 | 0,958 | 0,936 | 0,871 | 1 |
| OAS1_IFIT1_ISG15_IL1R2_OLAH | 0,862 | 0,852 | 0,871 | 0,893 | 0,826 | 0,961 | 0,946 | 0,896 | 0,995 |
| IFI44L_HERC6_FAM20A_RETN_MMP8 | 0,862 | 0,851 | 0,872 | 0,893 | 0,822 | 0,965 | 0,943 | 0,868 | 1 |
| RSAD2_IFI27_IFIT1_SIGLEC1_HERC6 | 0,862 | 0,852 | 0,871 | 0,896 | 0,840 | 0,951 | 0,917 | 0,852 | 0,982 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| IFIT1_ISG15_IL1R2_OLAH_MMP8 | 0,862 | 0,852 | 0,871 | 0,893 | 0,831 | 0,955 | 0,965 | 0,929 | 1 |
| OAS1_SIGLEC1_HERC6_SLC1A2_MMP8 | 0,862 | 0,853 | 0,871 | 0,889 | 0,825 | 0,952 | 0,936 | 0,879 | 0,993 |
| RSAD2_IFI27_ISG15_SLC1A2_FAM20A | 0,862 | 0,851 | 0,872 | 0,887 | 0,817 | 0,957 | 0,938 | 0,858 | 1 |
| RSAD2_IFIT1_IFI44L_SLC1A2_IL1R2 | 0,861 | 0,853 | 0,870 | 0,900 | 0,841 | 0,958 | 0,947 | 0,896 | 0,997 |
| IFIT1_IFI44L_IL1R2_OLAH_MMP8 | 0,861 | 0,852 | 0,871 | 0,890 | 0,826 | 0,954 | 0,961 | 0,922 | 1 |
| RSAD2_IFI44L_HERC6_OLAH_RETN | 0,861 | 0,851 | 0,871 | 0,891 | 0,822 | 0,960 | 0,941 | 0,890 | 0,993 |
| RSAD2_IFIT1_HERC6_FAM20A_RETN | 0,861 | 0,851 | 0,871 | 0,886 | 0,815 | 0,958 | 0,938 | 0,860 | 1 |
| RSAD2_SIGLEC1_HERC6_ SLC1A2_ MMP8 | 0,861 | 0,852 | 0,870 | 0,890 | 0,826 | 0,953 | 0,927 | 0,867 | 0,987 |
| RSAD2_IFI27_IFIT1_SLC1A2_FAM20A | 0,861 | 0,851 | 0,872 | 0,889 | 0,820 | 0,958 | 0,940 | 0,861 | 1 |
| RSAD2_OAS1_IFIT1_IFI44L_IL1R2 | 0,861 | 0,852 | 0,871 | 0,889 | 0,821 | 0,956 | 0,948 | 0,901 | 0,995 |
| IFIT1_IFI44L_HERC6_OLAH_MMP8 | 0,861 | 0,851 | 0,871 | 0,889 | 0,823 | 0,955 | 0,939 | 0,885 | 0,993 |
| OAS1_IFIT1_IL1R2_OLAH_RETN | 0,861 | 0,851 | 0,871 | 0,892 | 0,823 | 0,961 | 0,949 | 0,901 | 0,996 |
| IFI44L_SIGLEC1_HERC6_RETN_MMP8 | 0,861 | 0,851 | 0,871 | 0,883 | 0,808 | 0,959 | 0,943 | 0,894 | 0,993 |
| RSAD2_IFI27_IFIT1_SIGLEC1_SLC1A2 | 0,861 | 0,852 | 0,870 | 0,896 | 0,836 | 0,955 | 0,950 | 0,900 | 1,000 |
| OAS1_IFIT1_IFI44L_IL1R2_OLAH | 0,861 | 0,851 | 0,870 | 0,894 | 0,827 | 0,961 | 0,946 | 0,896 | 0,995 |
| IFI44L_ISG15_IL1R2_OLAH_MMP8 | 0,861 | 0,852 | 0,870 | 0,893 | 0,835 | 0,952 | 0,958 | 0,915 | 1,000 |
| RSAD2_OAS1_IL1R2_OLAH_MMP8 | 0,861 | 0,851 | 0,870 | 0,894 | 0,831 | 0,957 | 0,941 | 0,887 | 0,996 |
| RSAD2_IFIT1_HERC6_OLAH_RETN | 0,861 | 0,851 | 0,870 | 0,889 | 0,820 | 0,959 | 0,940 | 0,888 | 0,993 |
| IFI27_IFI44L_SIGLEC1_HERC6_SLC1A2 | 0,861 | 0,852 | 0,870 | 0,893 | 0,832 | 0,954 | 0,953 | 0,904 | 1 |
| RSAD2_OAS1_ISG15_HERC6_RETN | 0,861 | 0,851 | 0,871 | 0,890 | 0,821 | 0,958 | 0,941 | 0,887 | 0,996 |
| HERC6_SLC1A2_FAM20A_RETN_MMP8 | 0,861 | 0,851 | 0,871 | 0,896 | 0,832 | 0,960 | 0,936 | 0,854 | 1 |
| RSAD2_IFI44L_HERC6_ SLC1A2_ FAM20A | 0,861 | 0,851 | 0,871 | 0,895 | 0,834 | 0,955 | 0,922 | 0,838 | 1 |
| IFIT1_IFI44L_ISG15_IL1R2_MMP8 | 0,861 | 0,852 | 0,870 | 0,894 | 0,832 | 0,956 | 0,962 | 0,923 | 1 |
| RSAD2_IFI44L_SIGLEC1_ISG15_OLAH | 0,861 | 0,851 | 0,870 | 0,892 | 0,823 | 0,960 | 0,950 | 0,902 | 0,998 |
| SIGLEC1_HERC6_SLC1A2_RETN_MMP8 | 0,861 | 0,851 | 0,870 | 0,893 | 0,827 | 0,960 | 0,955 | 0,910 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27_OAS1_IFI44L_SLC1A2_MMP8 | 0,861 | 0,851 | 0,870 | 0,893 | 0,832 | 0,955 | 0,912 | 0,838 | 0,986 |
| OAS1_IFIT1_ISG15_IL1R2_RETN | 0,861 | 0,851 | 0,870 | 0,890 | 0,821 | 0,959 | 0,954 | 0,911 | 0,998 |
| RSAD2_OAS1_HERC6_OLAH_MMP8 | 0,861 | 0,851 | 0,870 | 0,888 | 0,821 | 0,954 | 0,936 | 0,880 | 0,991 |
| RSAD2_IFI44L_ISG15_HERC6_RETN | 0,861 | 0,850 | 0,871 | 0,892 | 0,824 | 0,960 | 0,933 | 0,875 | 0,991 |
| IFI44L_ISG15_SLC1A2_FAM20A_RETN | 0,861 | 0,851 | 0,871 | 0,893 | 0,827 | 0,959 | 0,935 | 0,860 | 1 |
| IFIT1_ISG15_HERC6_OLAH_MMP8 | 0,861 | 0,851 | 0,871 | 0,888 | 0,822 | 0,954 | 0,943 | 0,894 | 0,993 |
| RSAD2_IFI44L_ISG15_HERC6_FAM20A | 0,861 | 0,851 | 0,871 | 0,890 | 0,825 | 0,955 | 0,924 | 0,844 | 1 |
| IFI27_OAS1_IFIT1_SLC1A2_FAM20A | 0,860 | 0,850 | 0,871 | 0,888 | 0,819 | 0,957 | 0,941 | 0,862 | 1 |
| OAS1_IFIT1_IL1R2_RETN_MMP8 | 0,860 | 0,851 | 0,870 | 0,889 | 0,819 | 0,958 | 0,963 | 0,925 | 1 |
| RSAD2_IFI27_OAS1_SLC1A2_FAM20A | 0,860 | 0,850 | 0,871 | 0,889 | 0,820 | 0,958 | 0,936 | 0,854 | 1 |
| OAS1_IFIT1_HERC6_OLAH_MMP8 | 0,860 | 0,851 | 0,870 | 0,887 | 0,819 | 0,954 | 0,940 | 0,887 | 0,993 |
| RSAD2_ OAS1_ ISG15_ IL1R2_ OLAH | 0,860 | 0,851 | 0,870 | 0,895 | 0,829 | 0,961 | 0,948 | 0,898 | 0,997 |
| OAS1_ IFIT1_ ISG15_ ILIR2_ MMP8 | 0,860 | 0,851 | 0,869 | 0,890 | 0,826 | 0,954 | 0,959 | 0,918 | 0,999 |
| OAS1_ IFIT1_ SIGLEC1_ HERC6_ SLC1A2 | 0,860 | 0,851 | 0,869 | 0,889 | 0,828 | 0,949 | 0,930 | 0,873 | 0,988 |
| IFI27_ SIGLEC1_ ISG15_ HERC6_ SLC1A2 | 0,860 | 0,851 | 0,870 | 0,891 | 0,830 | 0,952 | 0,959 | 0,912 | 1 |
| IFIT1_ IFI44L_ SIGLEC1_ SLC1A2_ RETN | 0,860 | 0,851 | 0,869 | 0,895 | 0,832 | 0,957 | 0,940 | 0,886 | 0,994 |
| RSAD2_ OAS1_ IL1R2_ OLAH_ RETN | 0,860 | 0,851 | 0,870 | 0,893 | 0,825 | 0,961 | 0,943 | 0,891 | 0,996 |
| OAS1_ IFIT1_ IFI44L_ ISG15_ IL1R2 | 0,860 | 0,851 | 0,869 | 0,891 | 0,825 | 0,957 | 0,949 | 0,902 | 0,996 |
| RSAD2_ OAS1_ IFIT1_ IL1R2_ RETN | 0,860 | 0,851 | 0,870 | 0,887 | 0,816 | 0,958 | 0,951 | 0,906 | 0,997 |
| RSAD2_ OAS1_ IFIT1_ ISG15_ IL1R2 | 0,860 | 0,851 | 0,869 | 0,891 | 0,825 | 0,957 | 0,949 | 0,902 | 0,996 |
| IFIT1_ IL1R2_ OLAH_ RETN_ MMP8 | 0,860 | 0,850 | 0,870 | 0,893 | 0,827 | 0,960 | 0,964 | 0,927 | 1 |
| IFI44L_ ISG15_ HERC6_ OLAH_ MMP8 | 0,860 | 0,850 | 0,870 | 0,888 | 0,820 | 0,955 | 0,947 | 0,898 | 0,995 |
| IFIT1_ IFI44L_ ISG15_ IL1R2_ OLAH | 0,860 | 0,851 | 0,869 | 0,894 | 0,830 | 0,958 | 0,959 | 0,918 | 1,000 |
| IFIT1_ HERC6_ FAM20A_ RETN_ MMP8 | 0,860 | 0,849 | 0,870 | 0,888 | 0,818 | 0,959 | 0,940 | 0,863 | 1 |
| OAS1_ IFIT1_ IFI44L_ IL1R2_ RETN | 0,860 | 0,850 | 0,870 | 0,888 | 0,817 | 0,958 | 0,950 | 0,904 | 0,996 |

| Modèle | AUC modèle | IC 95% | | AUC Train | IC 95% Train | | AUC Test | IC 95% Test | |
|---|---|---|---|---|---|---|---|---|---|
| RSAD2_ IFI27_ IFI44L_ SLC1A2_ MMP8 | 0,860 | 0,850 | 0,870 | 0,893 | 0,831 | 0,955 | 0,915 | 0,842 | 0,988 |
| IFIT1_ SIGLEC1_ ISG15_ SLC1A2_ RETN | 0,860 | 0,851 | 0,869 | 0,895 | 0,833 | 0,957 | 0,949 | 0,900 | 0,998 |
| IFI27_ IFIT1_ ISG15_ SLC1A2_ MMP8 | 0,860 | 0,850 | 0,869 | 0,889 | 0,826 | 0,953 | 0,920 | 0,847 | 0,992 |
| RSAD2_ IFIT1_ IL1R2_ OLAH_ MMP8 | 0,860 | 0,850 | 0,869 | 0,889 | 0,825 | 0,953 | 0,963 | 0,925 | 1 |
| OAS1_ SIGLEC1_ ISG15_ SLC1A2_ RETN | 0,860 | 0,850 | 0,869 | 0,893 | 0,830 | 0,957 | 0,951 | 0,902 | 1 |
| IFI27_ IFIT1_ IFI44L_ SIGLEC1_ SLC1A2 | 0,860 | 0,850 | 0,869 | 0,893 | 0,831 | 0,954 | 0,955 | 0,908 | 1 |
| RSAD2_ OAS1_ IFI44L_ IL1R2_ OLAH | 0,860 | 0,850 | 0,869 | 0,894 | 0,829 | 0,960 | 0,941 | 0,887 | 0,995 |
| RSAD2_ IFI44L_ ISG15_ OLAH_ FAM20A | 0,860 | 0,849 | 0,870 | 0,890 | 0,818 | 0,961 | 0,958 | 0,910 | 1 |
| IFI27_ IFI44L_ SIGLEC1_ ISG15_ HERC6 | 0,859 | 0,850 | 0,869 | 0,892 | 0,832 | 0,952 | 0,926 | 0,866 | 0,986 |
| RSAD2_ ISG15_ SLC1A2_ IL1R2_ RETN | 0,859 | 0,850 | 0,868 | 0,896 | 0,837 | 0,955 | 0,945 | 0,886 | 1 |
| IFI27_ OAS1_ IFIT1_ SLC1A2_ MMP8 | 0,859 | 0,850 | 0,869 | 0,889 | 0,826 | 0,953 | 0,920 | 0,846 | 0,993 |
| RSAD2_ OAS1_ ISG15_ HERC6_ OLAH | 0,859 | 0,850 | 0,869 | 0,886 | 0,821 | 0,951 | 0,945 | 0,895 | 0,994 |
| SIGLEC1_ ISG15_ HERC6_ RETN_ MMP8 | 0,859 | 0,849 | 0,870 | 0,879 | 0,800 | 0,958 | 0,959 | 0,916 | 1 |
| RSAD2_ IFIT1_ IFI44L_ IL1R2_ MMP8 | 0,859 | 0,850 | 0,868 | 0,890 | 0,826 | 0,955 | 0,958 | 0,916 | 0,999 |
| IFIT1_ IFI44L_ IL1R2_ RETN_ MMP8 | 0,859 | 0,849 | 0,869 | 0,889 | 0,820 | 0,959 | 0,965 | 0,929 | 1 |
| OAS1_ IFI44L_ SIGLEC1_ SLC1A2_ RETN | 0,859 | 0,850 | 0,868 | 0,893 | 0,830 | 0,957 | 0,948 | 0,897 | 0,999 |
| OAS1_ IFI44L_ IL1R2_ OLAH_ MMP8 | 0,859 | 0,850 | 0,868 | 0,895 | 0,832 | 0,957 | 0,946 | 0,894 | 0,997 |
| RSAD2_ IFI44L_ ISG15_ SLC1A2_ IL1R2 | 0,859 | 0,851 | 0,868 | 0,897 | 0,839 | 0,954 | 0,943 | 0,888 | 0,999 |
| OAS1_ ISG15_ IL1R2_ OLAH_ MMP8 | 0,859 | 0,850 | 0,868 | 0,894 | 0,831 | 0,957 | 0,939 | 0,883 | 0,996 |
| RSAD2_ IFI27_ OAS1_ SLC1A2_ MMP8 | 0,859 | 0,849 | 0,869 | 0,889 | 0,826 | 0,953 | 0,913 | 0,835 | 0,991 |
| RSAD2_ OAS1_ SIGLEC1_ SLC1A2_ RETN | 0,859 | 0,849 | 0,869 | 0,893 | 0,829 | 0,956 | 0,953 | 0,906 | 1 |
| IFIT1_ SIGLEC1_ ISG15_ HERC6_ MMP8 | 0,859 | 0,850 | 0,868 | 0,886 | 0,820 | 0,952 | 0,895 | 0,816 | 0,973 |
| IFI27_ IFIT1_ IFI44L_ SLC1A2_ MMP8 | 0,859 | 0,849 | 0,869 | 0,891 | 0,828 | 0,953 | 0,917 | 0,846 | 0,989 |
| OAS1_ IL1R2_ OLAH_ RETN_ MMP8 | 0,859 | 0,849 | 0,869 | 0,893 | 0,826 | 0,960 | 0,949 | 0,900 | 0,997 |
| RSAD2_ IFIT1_ SIGLEC1_ SLC1A2_ RETN | 0,859 | 0,850 | 0,868 | 0,895 | 0,832 | 0,957 | 0,945 | 0,893 | 0,996 |

| Modèle | AUC modèle | IC 95% | | AUC Train | IC 95% Train | | AUC Test | IC 95% Test | |
|---|---|---|---|---|---|---|---|---|---|
| RSAD2_ IFI44L_ OLAH_ FAM20A_ MMP8 | 0,859 | 0,848 | 0,869 | 0,888 | 0,817 | 0,959 | 0,957 | 0,909 | 1 |
| OAS1_ ISG15_ HERC6_ OLAH_ MMP8 | 0,859 | 0,849 | 0,869 | 0,888 | 0,822 | 0,954 | 0,949 | 0,902 | 0,996 |
| RSAD2_ IFIT1_ ISG15_ IL1R2_ MMP8 | 0,859 | 0,850 | 0,868 | 0,892 | 0,829 | 0,954 | 0,960 | 0,920 | 1,000 |
| IFIT1_ ISG15_ IL1R2_ RETN_ MMP8 | 0,859 | 0,849 | 0,869 | 0,892 | 0,825 | 0,959 | 0,967 | 0,933 | 1 |
| OAS1_ IFIT1_ IFI44L_ HERC6_ OLAH | 0,859 | 0,849 | 0,869 | 0,887 | 0,821 | 0,953 | 0,941 | 0,889 | 0,994 |
| IFI44L_ SIGLEC1_ ISG15_ SLC1A2_ RETN | 0,859 | 0,850 | 0,868 | 0,894 | 0,830 | 0,957 | 0,951 | 0,903 | 0,999 |
| IFIT1_ IFI44L_ ISG15_ HERC6_ OLAH | 0,859 | 0,849 | 0,868 | 0,886 | 0,820 | 0,952 | 0,945 | 0,894 | 0,995 |
| OAS1_ IFI44L_ HERC6_ OLAH_ MMP8 | 0,859 | 0,849 | 0,869 | 0,888 | 0,821 | 0,954 | 0,941 | 0,889 | 0,993 |
| IFIT1_ IFI44L_ ISG15_ IL1R2_ RETN | 0,859 | 0,849 | 0,868 | 0,888 | 0,821 | 0,956 | 0,957 | 0,914 | 0,999 |
| IFIT1_ ISG15_ OLAH_ FAM20A_ RETN | 0,859 | 0,848 | 0,869 | 0,887 | 0,815 | 0,960 | 0,958 | 0,910 | 1 |
| OAS1_ IFI44L_ ISG15_ SLC1A2_ IL1R2 | 0,859 | 0,850 | 0,868 | 0,899 | 0,843 | 0,955 | 0,939 | 0,883 | 0,995 |
| ISG15_ HERC6_ SLC1A2_ FAM20A_ MMP8 | 0,859 | 0,848 | 0,869 | 0,890 | 0,826 | 0,955 | 0,925 | 0,840 | 1 |
| RSAD2_ IFIT1_ IFI44L_ ISG15_ IL1R2 | 0,859 | 0,849 | 0,868 | 0,890 | 0,823 | 0,956 | 0,958 | 0,916 | 0,999 |
| RSAD2_ ISG15_ HERC6_ OLAH_ MMP8 | 0,859 | 0,849 | 0,868 | 0,889 | 0,824 | 0,953 | 0,945 | 0,895 | 0,994 |
| OAS1_ IFI44L_ ISG15_ IL1R2_ OLAH | 0,859 | 0,849 | 0,868 | 0,893 | 0,826 | 0,959 | 0,942 | 0,890 | 0,995 |
| OAS1_ IFI44L_ IL1R2_ OLAH_ RETN | 0,859 | 0,849 | 0,868 | 0,893 | 0,824 | 0,961 | 0,946 | 0,895 | 0,996 |
| IFIT1_ IFI44L_ OLAH_ FAM20A_ RETN | 0,859 | 0,848 | 0,869 | 0,888 | 0,816 | 0,959 | 0,957 | 0,909 | 1 |
| IFI27_ IFIT1_ IFI44L_ SIGLEC1_ HERC6 | 0,859 | 0,849 | 0,868 | 0,893 | 0,834 | 0,952 | 0,920 | 0,856 | 0,984 |
| RSAD2_ ISG15_ OLAH_ FAM20A_ MMP8 | 0,859 | 0,848 | 0,869 | 0,887 | 0,815 | 0,959 | 0,960 | 0,910 | 1 |
| RSAD2_ OAS1_ IFIT1_ HERC6_ OLAH | 0,859 | 0,849 | 0,868 | 0,886 | 0,819 | 0,953 | 0,937 | 0,882 | 0,992 |
| OAS1_ IFIT1_ ISG15_ HERC6_ OLAH | 0,858 | 0,848 | 0,868 | 0,885 | 0,819 | 0,950 | 0,951 | 0,905 | 0,997 |
| IFI44L_ ISG15_ IL1R2_ OLAH_ RETN | 0,858 | 0,849 | 0,868 | 0,891 | 0,826 | 0,957 | 0,954 | 0,909 | 1,000 |
| IFI44L_ SIGLEC1_ HERC6_ SLC1A2_ MMP8 | 0,858 | 0,849 | 0,867 | 0,886 | 0,821 | 0,951 | 0,936 | 0,879 | 0,993 |
| RSAD2_ OLAH_ FAM20A_ RETN_ MMP8 | 0,858 | 0,848 | 0,869 | 0,888 | 0,817 | 0,960 | 0,962 | 0,913 | 1 |
| RSAD2_ OAS1_ IFI44L_ SLC1A2_ IL1R2 | 0,858 | 0,849 | 0,867 | 0,897 | 0,839 | 0,954 | 0,940 | 0,883 | 0,998 |

| Modèle | AUC modèle | IC 95% | | AUC Train | IC 95% Train | | AUC Test | IC 95% Test | |
|---|---|---|---|---|---|---|---|---|---|
| RSAD2_ IFIT1_ ISG15_ HERC6_ RETN | 0,858 | 0,848 | 0,868 | 0,890 | 0,821 | 0,958 | 0,937 | 0,881 | 0,993 |
| ISG15_ SLC1A2_ OLAH_ RETN_ MMP8 | 0,858 | 0,849 | 0,868 | 0,898 | 0,839 | 0,957 | 0,935 | 0,873 | 0,996 |
| RSAD2_ OAS1_ IFI44L_ HERC6_ OLAH | 0,858 | 0,848 | 0,868 | 0,887 | 0,821 | 0,953 | 0,937 | 0,882 | 0,992 |
| OAS1_ ISG15_ HERC6_ RETN_ MMP8 | 0,858 | 0,848 | 0,868 | 0,890 | 0,822 | 0,958 | 0,941 | 0,886 | 0,996 |
| RSAD2_ IFI44L_ SLC1A2_ IL1R2_ RETN | 0,858 | 0,849 | 0,867 | 0,897 | 0,838 | 0,955 | 0,941 | 0,884 | 0,999 |
| IFIT1_ ISG15_ HERC6_ FAM20A_ MMP8 | 0,858 | 0,848 | 0,868 | 0,885 | 0,817 | 0,952 | 0,927 | 0,845 | 1 |
| OAS1_ ISG15_ IL1R2_ OLAH_ RETN | 0,858 | 0,848 | 0,868 | 0,894 | 0,826 | 0,962 | 0,942 | 0,889 | 0,996 |
| RSAD2_ IFI27_ ISG15_ SLC1A2_ MMP8 | 0,858 | 0,848 | 0,867 | 0,888 | 0,824 | 0,952 | 0,911 | 0,833 | 0,989 |
| RSAD2_ OAS1_ ISG15_ SLC1A2_ IL1R2 | 0,858 | 0,849 | 0,867 | 0,895 | 0,837 | 0,953 | 0,938 | 0,875 | 1 |
| RSAD2_ IFIT1_ ISG15_ IL1R2_ OLAH | 0,858 | 0,848 | 0,867 | 0,893 | 0,827 | 0,958 | 0,952 | 0,907 | 0,997 |
| RSAD2_ IFI44L_ SLC1A2_ IL1R2_ MMP8 | 0,858 | 0,849 | 0,866 | 0,896 | 0,838 | 0,953 | 0,945 | 0,887 | 1 |
| IFI44L_ IL1R2_ OLAH_ RETN_ MMP8 | 0,858 | 0,848 | 0,868 | 0,889 | 0,824 | 0,954 | 0,957 | 0,913 | 1,000 |
| IFI27_ IFIT1_ SIGLEC1_ ISG15_ HERC6 | 0,858 | 0,848 | 0,868 | 0,889 | 0,830 | 0,949 | 0,924 | 0,861 | 0,987 |
| IFIT1_ ISG15_ OLAH_ FAM20A_ MMP8 | 0,858 | 0,847 | 0,868 | 0,884 | 0,811 | 0,956 | 0,959 | 0,910 | 1 |
| RSAD2_ IL1R2_ OLAH_ RETN_ MMP8 | 0,858 | 0,848 | 0,868 | 0,890 | 0,823 | 0,957 | 0,964 | 0,927 | 1 |
| RSAD2_ ISG15_ SLC1A2_ IL1R2_ MMP8 | 0,858 | 0,850 | 0,866 | 0,894 | 0,836 | 0,953 | 0,940 | 0,878 | 1 |
| OAS1_ISG15_SLC1A2_IL1R2_RETN | 0,858 | 0,849 | 0,867 | 0,899 | 0,841 | 0,956 | 0,930 | 0,867 | 0,994 |
| RSAD2_ OAS1_ ISG15_ HERC6_ FAM20A | 0,858 | 0,848 | 0,868 | 0,883 | 0,816 | 0,951 | 0,923 | 0,837 | 1 |
| RSAD2_ SIGLEC1_ ISG15_ SLC1A2_ RETN | 0,858 | 0,848 | 0,867 | 0,895 | 0,832 | 0,958 | 0,947 | 0,897 | 0,997 |
| RSAD2_ IFIT1_ IFI44L_ OLAH_ FAM20A | 0,858 | 0,848 | 0,868 | 0,887 | 0,815 | 0,959 | 0,955 | 0,906 | 1 |
| RSAD2_ IFIT1_ OLAH_ FAM20A_ MMP8 | 0,858 | 0,848 | 0,868 | 0,884 | 0,811 | 0,957 | 0,960 | 0,910 | 1 |
| OAS1_ SIGLEC1_ SLC1A2_ RETN_ MMP8 | 0,858 | 0,848 | 0,867 | 0,893 | 0,829 | 0,956 | 0,955 | 0,911 | 1 |
| RSAD2_ IFIT1_ ISG15_ IL1R2_ RETN | 0,858 | 0,848 | 0,867 | 0,886 | 0,817 | 0,956 | 0,957 | 0,914 | 0,999 |
| OAS1_ IFIT1_ SIGLEC1_ SLC1A2_ MMP8 | 0,858 | 0,848 | 0,867 | 0,886 | 0,823 | 0,950 | 0,928 | 0,869 | 0,988 |
| IFIT1_ ISG15_ IL1R2_ OLAH_ RETN | 0,858 | 0,848 | 0,867 | 0,892 | 0,826 | 0,958 | 0,962 | 0,923 | 1 |

| Modèle | AUC modèle | IC 95% | | AUC Train | IC 95% Train | | AUC Test | IC 95% Test | |
|---|---|---|---|---|---|---|---|---|---|
| OAS1_ IFI44L_ SLC1A2_ IL1R2_ RETN | 0,858 | 0,848 | 0,867 | 0,898 | 0,840 | 0,956 | 0,937 | 0,880 | 0,993 |
| RSAD2_ ISG15_ HERC6_ FAM20A_ MMP8 | 0,858 | 0,847 | 0,868 | 0,888 | 0,822 | 0,954 | 0,925 | 0,843 | 1 |
| OAS1_ SIGLEC1_ ISG15_ HERC6_ SLC1A2 | 0,858 | 0,848 | 0,867 | 0,885 | 0,823 | 0,948 | 0,947 | 0,897 | 0,996 |
| RSAD2_ IFI44L_ HERC6_ OLAH_ MMP8 | 0,858 | 0,848 | 0,867 | 0,888 | 0,822 | 0,954 | 0,937 | 0,882 | 0,992 |
| RSAD2_ IFIT1_ HERC6_ OLAH_ MMP8 | 0,858 | 0,848 | 0,867 | 0,887 | 0,820 | 0,954 | 0,941 | 0,889 | 0,994 |
| RSAD2_ ISG15_ IL1R2_ OLAH_ MMP8 | 0,857 | 0,848 | 0,867 | 0,890 | 0,826 | 0,953 | 0,958 | 0,916 | 1,000 |
| RSAD2_ HERC6_ FAM20A_ RETN_ MMP8 | 0,857 | 0,847 | 0,868 | 0,888 | 0,818 | 0,959 | 0,940 | 0,863 | 1 |
| RSAD2_ ISG15_ HERC6_ SLC1A2_ FAM20A | 0,857 | 0,847 | 0,867 | 0,887 | 0,825 | 0,950 | 0,929 | 0,846 | 1 |
| IFI44L_ HERC6_ SLC1A2_ FAM20A_ MMP8 | 0,857 | 0,847 | 0,868 | 0,886 | 0,821 | 0,952 | 0,928 | 0,845 | 1 |
| IFIT1_ IFI44L_ HERC6_ FAM20A_ MMP8 | 0,857 | 0,847 | 0,868 | 0,885 | 0,816 | 0,953 | 0,922 | 0,840 | 1 |
| RSAD2_ OAS1_ SLC1A2_ IL1R2_ RETN | 0,857 | 0,848 | 0,867 | 0,896 | 0,836 | 0,955 | 0,937 | 0,874 | 1 |
| OAS1_ IFIT1_ SIGLEC1_ HERC6_ MMP8 | 0,857 | 0,848 | 0,867 | 0,882 | 0,815 | 0,949 | 0,888 | 0,807 | 0,969 |
| RSAD2_ IFIT1_ SIGLEC1_ HERC6_ SLC1A2 | 0,857 | 0,848 | 0,866 | 0,890 | 0,829 | 0,951 | 0,925 | 0,865 | 0,985 |
| IFI27_ OAS1_ ISG15_ SLC1A2_ MMP8 | 0,857 | 0,848 | 0,867 | 0,887 | 0,823 | 0,951 | 0,910 | 0,832 | 0,988 |
| OAS1_ HERC6_ FAM20A_ RETN_ MMP8 | 0,857 | 0,847 | 0,868 | 0,889 | 0,818 | 0,960 | 0,939 | 0,861 | 1 |
| RSAD2_ IFIT1_ IL1R2_ RETN_ MMP8 | 0,857 | 0,847 | 0,867 | 0,890 | 0,821 | 0,959 | 0,965 | 0,929 | 1 |
| RSAD2_ ISG15_ OLAH_ FAM20A_ RETN | 0,857 | 0,847 | 0,867 | 0,886 | 0,813 | 0,960 | 0,960 | 0,908 | 1 |
| RSAD2_ IFI44L_ IL1R2_ OLAH_ MMP8 | 0,857 | 0,848 | 0,867 | 0,890 | 0,827 | 0,952 | 0,953 | 0,908 | 0,999 |
| RSAD2_ OAS1_ IFIT1_ SIGLEC1_ RETN | 0,857 | 0,847 | 0,867 | 0,880 | 0,805 | 0,954 | 0,941 | 0,887 | 0,996 |
| IFIT1_ SIGLEC1_ SLC1A2_ RETN_ MMP8 | 0,857 | 0,848 | 0,866 | 0,895 | 0,833 | 0,957 | 0,950 | 0,903 | 0,997 |
| OAS1_ IFI44L_ SLC1A2_ IL1R2_ MMP8 | 0,857 | 0,848 | 0,866 | 0,897 | 0,840 | 0,954 | 0,934 | 0,874 | 0,993 |
| RSAD2_ IFIT1_ IFI44L_ IL1R2_ OLAH | 0,857 | 0,847 | 0,867 | 0,891 | 0,825 | 0,957 | 0,952 | 0,907 | 0,997 |
| RSAD2_ IFIT1_ ISG15_ OLAH_ FAM20A | 0,857 | 0,847 | 0,867 | 0,885 | 0,810 | 0,959 | 0,960 | 0,911 | 1 |
| RSAD2_ IFI44L_ SIGLEC1_ SLC1A2_ RETN | 0,857 | 0,848 | 0,866 | 0,894 | 0,831 | 0,958 | 0,950 | 0,902 | 0,998 |
| RSAD2_ IFI27_ IFIT1_ SLC1A2_ MMP8 | 0,857 | 0,847 | 0,867 | 0,888 | 0,824 | 0,952 | 0,920 | 0,847 | 0,992 |

| Modèle | AUC modèle | IC 95% | | AUC Train | IC 95% Train | | AUC Test | IC 95% Test | |
|---|---|---|---|---|---|---|---|---|---|
| OAS1_ IFI44L_ ISG15_ HERC6_ OLAH | 0,857 | 0,847 | 0,867 | 0,885 | 0,820 | 0,951 | 0,950 | 0,904 | 0,996 |
| RSAD2_ OAS1_ SIGLEC1_ HERC6_ SLC1A2 | 0,857 | 0,848 | 0,866 | 0,887 | 0,825 | 0,949 | 0,938 | 0,885 | 0,991 |
| IFIT1_ IFI44L_ SIGLEC1_ HERC6_ MMP8 | 0,857 | 0,847 | 0,866 | 0,882 | 0,814 | 0,950 | 0,886 | 0,803 | 0,968 |
| IFI44L_ ISG15_ IL1R2_ RETN_ MMP8 | 0,857 | 0,847 | 0,866 | 0,890 | 0,827 | 0,954 | 0,966 | 0,931 | 1 |
| RSAD2_ IFIT1_ IFI44L_ HERC6_ OLAH | 0,857 | 0,847 | 0,866 | 0,888 | 0,823 | 0,953 | 0,938 | 0,884 | 0,992 |
| RSAD2_ OAS1_ SLC1A2_ IL1R2_ MMP8 | 0,857 | 0,848 | 0,866 | 0,896 | 0,837 | 0,954 | 0,939 | 0,878 | 1,000 |
| RSAD2_ IFI44L_ ISG15_ HERC6_ OLAH | 0,857 | 0,847 | 0,867 | 0,888 | 0,824 | 0,952 | 0,942 | 0,892 | 0,993 |
| IFIT1_ IFI44L_ ISG15_ HERC6_ FAM20A | 0,857 | 0,847 | 0,867 | 0,880 | 0,810 | 0,950 | 0,928 | 0,843 | 1 |
| OAS1_ IFI44L_ HERC6_ SLC1A2_ RETN | 0,857 | 0,847 | 0,867 | 0,888 | 0,821 | 0,956 | 0,936 | 0,874 | 0,997 |
| RSAD2_ ISG15_ HERC6_ SLC1A2_ RETN | 0,857 | 0,847 | 0,866 | 0,892 | 0,827 | 0,956 | 0,935 | 0,871 | 0,998 |
| RSAD2_ SLC1A2_ IL1R2_ RETN_ MMP8 | 0,857 | 0,848 | 0,866 | 0,895 | 0,836 | 0,955 | 0,941 | 0,880 | 1 |
| RSAD2_ ISG15_ HERC6_ RETN_ MMP8 | 0,857 | 0,846 | 0,867 | 0,890 | 0,822 | 0,958 | 0,936 | 0,879 | 0,992 |
| RSAD2_ IFI44L_ ISG15_ IL1R2_ OLAH | 0,857 | 0,847 | 0,866 | 0,889 | 0,824 | 0,955 | 0,952 | 0,906 | 0,999 |
| RSAD2_ IFIT1_ IFI44L_ IL1R2_ RETN | 0,857 | 0,847 | 0,866 | 0,885 | 0,814 | 0,956 | 0,955 | 0,913 | 0,998 |
| OAS1_ IFIT1_ SIGLEC1_ ISG15_ RETN | 0,857 | 0,847 | 0,866 | 0,880 | 0,806 | 0,954 | 0,945 | 0,892 | 0,997 |
| RSAD2_ ISG15_ IL1R2_ OLAH_ RETN | 0,856 | 0,847 | 0,866 | 0,889 | 0,822 | 0,957 | 0,958 | 0,915 | 1 |
| RSAD2_ IFIT1_ ISG15_ HERC6_ OLAH | 0,856 | 0,847 | 0,866 | 0,886 | 0,821 | 0,951 | 0,945 | 0,895 | 0,994 |
| RSAD2_ IFIT1_ IL1R2_ OLAH_ RETN | 0,856 | 0,847 | 0,866 | 0,889 | 0,821 | 0,957 | 0,953 | 0,909 | 0,998 |
| OAS1_ ISG15_ HERC6_ SLC1A2_ FAM20A | 0,856 | 0,846 | 0,866 | 0,888 | 0,825 | 0,950 | 0,930 | 0,847 | 1 |
| OAS1_ ISG15_ SLC1A2_ IL1R2_ MMP8 | 0,856 | 0,848 | 0,865 | 0,898 | 0,840 | 0,955 | 0,925 | 0,858 | 0,992 |
| OAS1_ IFI44L_ SIGLEC1_ HERC6_ SLC1A2 | 0,856 | 0,847 | 0,865 | 0,887 | 0,824 | 0,950 | 0,945 | 0,894 | 0,995 |
| IFI44L_ SIGLEC1_ SLC1A2_ RETN_ MMP8 | 0,856 | 0,847 | 0,865 | 0,892 | 0,828 | 0,957 | 0,953 | 0,907 | 0,999 |
| RSAD2_ SIGLEC1_ SLC1A2_ RETN_ MMP8 | 0,856 | 0,846 | 0,866 | 0,893 | 0,829 | 0,957 | 0,953 | 0,908 | 0,999 |
| SIGLEC1_ ISG15_ SLC1A2_ RETN_ MMP8 | 0,856 | 0,847 | 0,866 | 0,893 | 0,829 | 0,958 | 0,953 | 0,906 | 1 |
| RSAD2_ IFI44L_ OLAH_ FAM20A_ RETN | 0,856 | 0,846 | 0,867 | 0,888 | 0,816 | 0,960 | 0,954 | 0,903 | 1 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| SIGLEC1_ ISG15_ HERC6_ SLC1A2_ MMP8 | 0,856 | 0,847 | 0,865 | 0,887 | 0,822 | 0,952 | 0,934 | 0,877 | 0,991 |
| IFI44L_ ISG15_ HERC6_ SLC1A2_ FAM20A | 0,856 | 0,846 | 0,866 | 0,886 | 0,821 | 0,952 | 0,935 | 0,854 | 1 |
| RSAD2_ OAS1_ IFI44L_ HERC6_ FAM20A | 0,856 | 0,846 | 0,866 | 0,882 | 0,811 | 0,952 | 0,923 | 0,841 | 1 |
| RSAD2_ IFIT1_ SIGLEC1_ SLC1A2_ MMP8 | 0,856 | 0,847 | 0,865 | 0,886 | 0,822 | 0,949 | 0,928 | 0,869 | 0,988 |
| OAS1_ IFIT1_ IFI44L_ SIGLEC1_ RETN | 0,856 | 0,845 | 0,866 | 0,876 | 0,800 | 0,951 | 0,935 | 0,877 | 0,993 |
| ISG15_ SLC1A2_ IL1R2_ RETN_ MMP8 | 0,856 | 0,847 | 0,865 | 0,897 | 0,840 | 0,954 | 0,918 | 0,838 | 0,999 |
| IFIT1_ IFI44L_ SIGLEC1_ SLC1A2_ MMP8 | 0,856 | 0,847 | 0,865 | 0,888 | 0,825 | 0,951 | 0,921 | 0,858 | 0,984 |
| IFIT1_ IFI44L_ HERC6_ SLC1A2_ FAM20A | 0,856 | 0,846 | 0,865 | 0,892 | 0,831 | 0,953 | 0,926 | 0,842 | 1 |
| IFIT1_ OLAH_ FAM20A_ RETN_ MMP8 | 0,856 | 0,845 | 0,866 | 0,887 | 0,816 | 0,958 | 0,964 | 0,921 | 1 |
| RSAD2_ IFI44L_ IL1R2_ OLAH_ RETN | 0,856 | 0,846 | 0,865 | 0,890 | 0,822 | 0,957 | 0,957 | 0,913 | 1,000 |
| RSAD2_ OAS1_ IFI44L_ ISG15_ IL1R2 | 0,856 | 0,846 | 0,865 | 0,888 | 0,823 | 0,952 | 0,941 | 0,889 | 0,993 |
| IFIT1_ IFI44L_ SIGLEC1_ ISG15_ RETN | 0,856 | 0,846 | 0,865 | 0,881 | 0,808 | 0,954 | 0,939 | 0,884 | 0,995 |
| IFIT1_ IFI44L_ IL1R2_ OLAH_ RETN | 0,855 | 0,846 | 0,865 | 0,890 | 0,823 | 0,957 | 0,954 | 0,910 | 0,999 |
| RSAD2_ SIGLEC1_ ISG15_ HERC6_ SLC1A2 | 0,855 | 0,847 | 0,864 | 0,887 | 0,825 | 0,949 | 0,938 | 0,884 | 0,992 |
| RSAD2_ OAS1_ IFI44L_ IL1R2_ RETN | 0,855 | 0,846 | 0,865 | 0,883 | 0,813 | 0,953 | 0,942 | 0,891 | 0,994 |
| OAS1_ IFI44L_ ISG15_ IL1R2_ RETN | 0,855 | 0,846 | 0,865 | 0,888 | 0,820 | 0,956 | 0,952 | 0,908 | 0,997 |
| RSAD2_ IFIT1_ OLAH_ FAM20A_ RETN | 0,855 | 0,845 | 0,866 | 0,885 | 0,811 | 0,958 | 0,958 | 0,907 | 1 |
| RSAD2_ IFIT1_ SIGLEC1_ HERC6_ MMP8 | 0,855 | 0,846 | 0,865 | 0,881 | 0,813 | 0,949 | 0,887 | 0,805 | 0,969 |
| RSAD2_ OAS1_ ISG15_ IL1R2_ RETN | 0,855 | 0,846 | 0,865 | 0,886 | 0,817 | 0,954 | 0,947 | 0,898 | 0,995 |
| RSAD2_ OAS1_ IFI44L_ IL1R2_ MMP8 | 0,855 | 0,846 | 0,865 | 0,887 | 0,822 | 0,951 | 0,943 | 0,893 | 0,994 |
| IFIT1_ HERC6_ SLC1A2_ FAM20A_ MMP8 | 0,855 | 0,845 | 0,865 | 0,886 | 0,820 | 0,951 | 0,925 | 0,840 | 1 |
| RSAD2_ IFI44L_ SIGLEC1_ HERC6_ SLC1A2 | 0,855 | 0,846 | 0,864 | 0,886 | 0,824 | 0,948 | 0,934 | 0,878 | 0,989 |
| IFI44L_ SIGLEC1_ ISG15_ SLC1A2_ MMP8 | 0,855 | 0,847 | 0,864 | 0,884 | 0,819 | 0,949 | 0,937 | 0,882 | 0,991 |
| CAS1_ SLC1A2_ IL1R2_ RETN_ MMP8 | 0,855 | 0,846 | 0,864 | 0,895 | 0,836 | 0,955 | 0,928 | 0,863 | 0,994 |
| RSAD2_ HERC6_ SLC1A2_ FAM20A_ MMP8 | 0,855 | 0,845 | 0,865 | 0,888 | 0,823 | 0,952 | 0,924 | 0,839 | 1 |

(suite)

| Modèle | AUC modèle | IC 95% | | AUC Train | IC 95% Train | | AUC Test | IC 95% Test | |
|---|---|---|---|---|---|---|---|---|---|
| ISG15_ IL1R2_ OLAH_ RETN_ MMP8 | 0,855 | 0,846 | 0,865 | 0,892 | 0,830 | 0,954 | 0,943 | 0,886 | 1 |
| IFI44L_ SLC1A2_ FAM20A_ RETN_ MMP8 | 0,855 | 0,845 | 0,865 | 0,891 | 0,825 | 0,957 | 0,930 | 0,852 | 1 |
| IFIT1_ SIGLEC1_ ISG15_ SLC1A2_ MMP8 | 0,855 | 0,846 | 0,864 | 0,889 | 0,827 | 0,952 | 0,930 | 0,872 | 0,989 |
| RSAD2_ OAS1_ IFIT1_ OLAH_ RETN | 0,855 | 0,845 | 0,865 | 0,884 | 0,809 | 0,958 | 0,947 | 0,896 | 0,998 |
| IFI44L_ ISG15_ SLC1A2_ FAM20A_ MMP8 | 0,855 | 0,845 | 0,865 | 0,884 | 0,815 | 0,953 | 0,934 | 0,858 | 1 |
| CAS1_ ISG15_ HERC6_ FAM20A_ MMP8 | 0,855 | 0,844 | 0,865 | 0,885 | 0,818 | 0,953 | 0,932 | 0,851 | 1 |
| OAS1_ IFIT1_ SIGLEC1_ RETN_ MMP8 | 0,855 | 0,844 | 0,865 | 0,878 | 0,803 | 0,954 | 0,946 | 0,894 | 0,997 |
| RSAD2_ IFIT1_ IFI44L_ SIGLEC1_ RETN | 0,855 | 0,844 | 0,865 | 0,878 | 0,803 | 0,953 | 0,936 | 0,878 | 0,994 |
| RSAD2_ OAS1_ IL1R2_ RETN_ MMP8 | 0,855 | 0,845 | 0,864 | 0,885 | 0,815 | 0,954 | 0,953 | 0,909 | 0,997 |
| RSAD2_ IFI44L_ ISG15_ IL1R2_ RETN | 0,855 | 0,845 | 0,864 | 0,884 | 0,815 | 0,953 | 0,958 | 0,916 | 0,999 |
| RSAD2_ IFIT1_ SIGLEC1_ ISG15_ RETN | 0,854 | 0,845 | 0,864 | 0,880 | 0,806 | 0,954 | 0,943 | 0,890 | 0,997 |
| OAS1_ IFI44L_ HERC6_ SLC1A2_ FAM20A | 0,854 | 0,844 | 0,865 | 0,889 | 0,827 | 0,952 | 0,935 | 0,854 | 1 |
| RSAD2_ OAS1_ SIGLEC1_ SLC1A2_ MMP8 | 0,854 | 0,845 | 0,864 | 0,883 | 0,818 | 0,948 | 0,938 | 0,883 | 0,993 |
| OAS1_ IFI44L_ ISG15_ IL1R2_ MMP8 | 0,854 | 0,845 | 0,863 | 0,887 | 0,823 | 0,950 | 0,946 | 0,897 | 0,995 |
| ISG15_ OLAH_ FAM20A_ RETN_ MMP8 | 0,854 | 0,844 | 0,865 | 0,891 | 0,826 | 0,956 | 0,950 | 0,899 | 1 |
| IFIT1_ ISG15_ HERC6_ SLC1A2_ FAM20A | 0,854 | 0,844 | 0,865 | 0,886 | 0,822 | 0,950 | 0,933 | 0,850 | 1 |
| OAS1_ HERC6_ SLC1A2_FAM20A_ MMP8 | 0,854 | 0,844 | 0,864 | 0,887 | 0,822 | 0,952 | 0,921 | 0,835 | 1 |
| RSAD2_ OAS1_ OLAH_ RETN_ MMP8 | 0,854 | 0,844 | 0,865 | 0,883 | 0,809 | 0,958 | 0,959 | 0,917 | 1 |
| OAS1_ ISG15_ IL1R2_ RETN_ MMP8 | 0,854 | 0,845 | 0,864 | 0,889 | 0,823 | 0,955 | 0,953 | 0,909 | 0,997 |
| IFI27_ OAS1_ IFIT1_ SIGLEC1_ ISG15 | 0,854 | 0,845 | 0,864 | 0,882 | 0,818 | 0,946 | 0,941 | 0,887 | 0,995 |
| OAS1_ IFIT1_ ISG15_ OLAH_ RETN | 0,854 | 0,844 | 0,864 | 0,884 | 0,810 | 0,959 | 0,955 | 0,911 | 1,000 |
| RSAD2_ OAS1_ ISG15_ IL1R2_ MMP8 | 0,854 | 0,845 | 0,863 | 0,886 | 0,821 | 0,951 | 0,945 | 0,895 | 0,994 |
| IFIT1_ IFI44L_ SIGLEC1_ HERC6_ SLC1A2 | 0,854 | 0,846 | 0,863 | 0,886 | 0,825 | 0,948 | 0,934 | 0,878 | 0,990 |
| SLC1A2_ IL1R2_ OLAH_ FAM20A_ MMP8 | 0,854 | 0,845 | 0,864 | 0,891 | 0,835 | 0,947 | 0,877 | 0,782 | 0,973 |
| RSAD2_ IFI44L_ HERC6_ SLC1A2_ RETN | 0,854 | 0,844 | 0,864 | 0,890 | 0,823 | 0,957 | 0,929 | 0,867 | 0,992 |

(suite)

| Modèle | AUC modèle | IC 95% | | AUC Train | IC 95% Train | | AUC Test | IC 95% Test | |
|---|---|---|---|---|---|---|---|---|---|
| OAS1_ IFI44L_ SIGLEC1_ SLC1A2_ MMP8 | 0,854 | 0,845 | 0,863 | 0,884 | 0,819 | 0,949 | 0,937 | 0,882 | 0,992 |
| OAS1_ IFIT1_ OLAH_ RETN_ MMP8 | 0,854 | 0,844 | 0,864 | 0,882 | 0,807 | 0,957 | 0,963 | 0,925 | 1 |
| RSAD2_ OAS1_ IFI44L_ HERC6_ RETN | 0,854 | 0,843 | 0,864 | 0,877 | 0,801 | 0,954 | 0,929 | 0,870 | 0,988 |
| IFI44L_ ISG15_ HERC6_ FAM20A_ MMP8 | 0,854 | 0,843 | 0,865 | 0,878 | 0,806 | 0,950 | 0,928 | 0,847 | 1 |
| IFIT1_ IFI44L_ ISG15_ HERC6_ RETN | 0,854 | 0,844 | 0,864 | 0,884 | 0,812 | 0,956 | 0,935 | 0,877 | 0,992 |
| IFIT1_ SIGLEC1_ ISG15_ HERC6_ SLC1A2 | 0,854 | 0,845 | 0,863 | 0,886 | 0,825 | 0,948 | 0,936 | 0,881 | 0,991 |
| RSAD2_ IFI27_ OAS1_ SIGLEC1_ ISG15 | 0,854 | 0,844 | 0,864 | 0,884 | 0,822 | 0,946 | 0,943 | 0,889 | 0,998 |
| OAS1_ SIGLEC1_ ISG15_ SLC1A2_ MMP8 | 0,854 | 0,844 | 0,863 | 0,885 | 0,821 | 0,949 | 0,939 | 0,885 | 0,993 |
| RSAD2_ IFIT1_ ISG15_ HERC6_ FAM20A | 0,854 | 0,844 | 0,864 | 0,883 | 0,816 | 0,950 | 0,925 | 0,840 | 1 |
| RSAD2_ IFI27_ OAS1_ IFI44L_ SIGLEC1 | 0,854 | 0,844 | 0,864 | 0,885 | 0,823 | 0,947 | 0,946 | 0,894 | 0,997 |
| IFIT1_ ISG15_ SLC1A2_ FAM20A_ RETN | 0,854 | 0,843 | 0,864 | 0,885 | 0,816 | 0,955 | 0,936 | 0,862 | 1 |
| IFI44L_ ISG15_ OLAH_ RETN_ MMP8 | 0,854 | 0,844 | 0,864 | 0,885 | 0,813 | 0,956 | 0,959 | 0,916 | 1 |
| OAS1_ IFI44L_ IL1R2_ RETN_ MMP8 | 0,854 | 0,844 | 0,863 | 0,886 | 0,818 | 0,955 | 0,964 | 0,927 | 1 |
| IFI27_ OAS1_ IFIT1_ ISG15_ MMP8 | 0,853 | 0,843 | 0,863 | 0,878 | 0,807 | 0,948 | 0,914 | 0,842 | 0,986 |
| IFI27_ OAS1_ IFIT1_ HERC6_ SLC1A2 | 0,853 | 0,843 | 0,863 | 0,888 | 0,827 | 0,949 | 0,940 | 0,881 | 1,000 |
| IFIT1_ ISG15_ HERC6_ SLC1A2_ RETN | 0,853 | 0,844 | 0,863 | 0,888 | 0,821 | 0,954 | 0,940 | 0,879 | 1 |
| IFI27_ OAS1_ IFI44L_ SIGLEC1_ ISG15 | 0,853 | 0,844 | 0,863 | 0,886 | 0,823 | 0,948 | 0,948 | 0,897 | 0,999 |
| IFI27_ OAS1_ IFIT1_ IFI44L_ SIGLEC1 | 0,853 | 0,843 | 0,863 | 0,884 | 0,821 | 0,948 | 0,951 | 0,902 | 1 |
| RSAD2_ OAS1_ IFIT1_ OLAH_ MMP8 | 0,853 | 0,843 | 0,863 | 0,880 | 0,807 | 0,953 | 0,946 | 0,895 | 0,997 |
| OAS1_ IFIT1_ IFI44L_ OLAH_ RETN | 0,853 | 0,843 | 0,864 | 0,882 | 0,807 | 0,956 | 0,946 | 0,895 | 0,996 |
| RSAD2_ OAS1_ ISG15_ OLAH_ RETN | 0,853 | 0,843 | 0,863 | 0,883 | 0,809 | 0,957 | 0,953 | 0,907 | 0,999 |
| RSAD2_ IFI27_ IFI44L_ SIGLEC1_ ISG15 | 0,853 | 0,843 | 0,863 | 0,886 | 0,825 | 0,948 | 0,942 | 0,888 | 0,996 |
| IFI27_ OAS1_ IFI44L_ ISG15_ MMP8 | 0,853 | 0,843 | 0,863 | 0,880 | 0,810 | 0,949 | 0,904 | 0,827 | 0,981 |
| RSAD2_ ISG15_ IL1R2_ RETN_ MMP8 | 0,853 | 0,844 | 0,863 | 0,886 | 0,818 | 0,954 | 0,962 | 0,922 | 1 |
| RSAD2_ OAS1_ SIGLEC1_ ISG15_ RETN | 0,853 | 0,843 | 0,863 | 0,878 | 0,803 | 0,953 | 0,949 | 0,899 | 0,999 |

| Modèle | AUC modèle | IC 95% | | AUC Train | IC 95% Train | | AUC Test | IC 95% Test | |
|---|---|---|---|---|---|---|---|---|---|
| RSAD2_ IFI27_ OAS1_ IFIT1_ SIGLEC1 | 0,853 | 0,843 | 0,863 | 0,884 | 0,822 | 0,947 | 0,942 | 0,888 | 0,997 |
| RSAD2_ IFI27_ OAS1_ IFI44L_ MMP8 | 0,853 | 0,843 | 0,863 | 0,877 | 0,807 | 0,948 | 0,909 | 0,833 | 0,985 |
| IFI27_ OAS1_ IFI44L_ HERC6_ SLC1A2 | 0,853 | 0,843 | 0,863 | 0,884 | 0,823 | 0,945 | 0,921 | 0,853 | 0,989 |
| OAS1_ IFIT1_ IFI44L_ HERC6_ RETN | 0,853 | 0,842 | 0,864 | 0,876 | 0,799 | 0,953 | 0,929 | 0,870 | 0,988 |
| IFI27_ IFIT1_ IFI44L_ ISG15_ MMP8 | 0,853 | 0,843 | 0,863 | 0,878 | 0,808 | 0,949 | 0,911 | 0,837 | 0,985 |
| IFIT1_ IFI44L_ SIGLEC1_ RETN_ MMP8 | 0,853 | 0,842 | 0,863 | 0,878 | 0,804 | 0,953 | 0,939 | 0,882 | 0,996 |
| IFI27_ IFIT1_ IFI44L_ SIGLEC1_ ISG15 | 0,853 | 0,843 | 0,863 | 0,885 | 0,821 | 0,949 | 0,941 | 0,887 | 0,996 |
| RSAD2_ IFI44L_ ISG15_ SLC1A2_ FAM20A | 0,853 | 0,842 | 0,863 | 0,888 | 0,821 | 0,955 | 0,924 | 0,845 | 1 |
| RSAD2_ OAS1_ HERC6_ SLC1A2_ FAM20A | 0,853 | 0,843 | 0,863 | 0,881 | 0,816 | 0,947 | 0,923 | 0,837 | 1 |
| RSAD2_ IFI27_ IFIT1_ SIGLEC1_ ISG15 | 0,853 | 0,843 | 0,863 | 0,883 | 0,820 | 0,946 | 0,942 | 0,888 | 0,997 |
| OAS1_ IFI44L_ OLAH_ RETN_ MMP8 | 0,853 | 0,842 | 0,863 | 0,882 | 0,807 | 0,957 | 0,959 | 0,918 | 1,000 |
| IFIT1_ ISG15_ HERC6_ RETN_ MMP8 | 0,853 | 0,842 | 0,863 | 0,884 | 0,813 | 0,955 | 0,927 | 0,866 | 0,989 |
| ISG15_ HERC6_ SLC1A2_ RETN_ MMP8 | 0,853 | 0,843 | 0,863 | 0,886 | 0,818 | 0,954 | 0,935 | 0,870 | 0,999 |
| RSAD2_ SIGLEC1_ ISG15_ SLC1A2_ MMP8 | 0,853 | 0,843 | 0,862 | 0,885 | 0,820 | 0,949 | 0,938 | 0,884 | 0,992 |
| RSAD2_ IFI44L_ ISG15_ IL1R2_ MMP8 | 0,853 | 0,844 | 0,862 | 0,886 | 0,823 | 0,949 | 0,961 | 0,921 | 1 |
| IFI44L_ ISG15_ HERC6_ SLC1A2_ RETN | 0,853 | 0,843 | 0,862 | 0,886 | 0,818 | 0,954 | 0,942 | 0,880 | 1 |
| OAS1_ IFIT1_ ISG15_ HERC6_ FAM20A | 0,852 | 0,842 | 0,863 | 0,879 | 0,810 | 0,948 | 0,926 | 0,839 | 1 |
| IFIT1_ IFI44L_ SLC1A2_ FAM20A_ RETN | 0,852 | 0,842 | 0,862 | 0,889 | 0,823 | 0,955 | 0,927 | 0,848 | 1 |
| OAS1_ IFI44L_ SLC1A2_ FAM20A_ RETN | 0,852 | 0,842 | 0,863 | 0,892 | 0,826 | 0,958 | 0,932 | 0,852 | 1 |
| SLC1A2_ IL1R2_ OLAH_ FAM20A_ RETN | 0,852 | 0,843 | 0,862 | 0,884 | 0,823 | 0,945 | 0,862 | 0,759 | 0,965 |
| RSAD2_ IFIT1_ IFI44L_ HERC6_ RETN | 0,852 | 0,842 | 0,863 | 0,876 | 0,799 | 0,953 | 0,927 | 0,867 | 0,987 |
| IFIT1_ SIGLEC1_ ISG15_ RETN_ MMP8 | 0,852 | 0,842 | 0,862 | 0,880 | 0,806 | 0,953 | 0,949 | 0,898 | 0,999 |
| RSAD2_ OAS1_ IFI44L_ SLC1A2_ FAM20A | 0,852 | 0,841 | 0,863 | 0,889 | 0,822 | 0,956 | 0,914 | 0,829 | 1,000 |
| IFI27_ OAS1_ ISG15_ HERC6_ SLC1A2 | 0,852 | 0,842 | 0,862 | 0,884 | 0,823 | 0,944 | 0,912 | 0,837 | 0,987 |
| RSAD2_ IFI44L_ HERC6_ FAM20A_ MMP8 | 0,852 | 0,841 | 0,863 | 0,879 | 0,808 | 0,950 | 0,922 | 0,840 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1_IFI44L_SIGLEC1_ISG15_RETN | 0,852 | 0,842 | 0,862 | 0,877 | 0,801 | 0,953 | 0,952 | 0,904 | 1 |
| OAS1_ISG15_SLC1A2_FAM20A_RETN | 0,852 | 0,842 | 0,862 | 0,886 | 0,818 | 0,954 | 0,934 | 0,853 | 1 |
| OAS1_IFI44L_ISG15_HERC6_FAM20A | 0,852 | 0,842 | 0,862 | 0,878 | 0,807 | 0,948 | 0,933 | 0,853 | 1 |
| RSAD2_IFI44L_SIGLEC1_SLC1A2_MMP8 | 0,852 | 0,843 | 0,861 | 0,885 | 0,821 | 0,950 | 0,933 | 0,876 | 0,990 |
| RSAD2_OAS1_IFI44L_OLAH_MMP8 | 0,852 | 0,842 | 0,862 | 0,881 | 0,809 | 0,952 | 0,940 | 0,885 | 0,996 |
| RSAD2_OAS1_IFI44L_OLAH_RETN | 0,852 | 0,842 | 0,862 | 0,883 | 0,809 | 0,957 | 0,940 | 0,885 | 0,995 |
| RSAD2_IFI27_IFI44L_ISG15_MMP8 | 0,852 | 0,842 | 0,862 | 0,878 | 0,808 | 0,949 | 0,904 | 0,827 | 0,981 |
| RSAD2_IFI44L_IL1R2_RETN_MMP8 | 0,852 | 0,842 | 0,862 | 0,884 | 0,814 | 0,953 | 0,963 | 0,925 | 1 |
| RSAD2_OAS1_ISG15_FAM20A_RETN | 0,852 | 0,841 | 0,862 | 0,886 | 0,815 | 0,958 | 0,935 | 0,855 | 1 |
| OAS1_ISG15_OLAH_RETN_MMP8 | 0,852 | 0,841 | 0,862 | 0,883 | 0,810 | 0,957 | 0,963 | 0,925 | 1 |
| IFI44L_SIGLEC1_ISG15_RETN_MMP8 | 0,852 | 0,841 | 0,862 | 0,876 | 0,799 | 0,952 | 0,959 | 0,916 | 1 |
| OAS1_IFIT1_IFI44L_OLAH_MMP8 | 0,852 | 0,842 | 0,862 | 0,880 | 0,807 | 0,952 | 0,941 | 0,887 | 0,995 |
| RSAD2_IFI27_OAS1_ISG15_MMP8 | 0,852 | 0,841 | 0,862 | 0,877 | 0,807 | 0,948 | 0,908 | 0,831 | 0,985 |
| RSAD2_OAS1_IFI44L_SIGLEC1_RETN | 0,852 | 0,841 | 0,862 | 0,874 | 0,797 | 0,951 | 0,945 | 0,893 | 0,996 |
| RSAD2_OAS1_HERC6_FAM20A_MMP8 | 0,851 | 0,841 | 0,862 | 0,879 | 0,808 | 0,950 | 0,923 | 0,840 | 1 |
| RSAD2_IFI27_IFI44L_HERC6_SLC1A2 | 0,851 | 0,842 | 0,861 | 0,880 | 0,818 | 0,941 | 0,912 | 0,842 | 0,982 |
| RSAD2_OAS1_ISG15_OLAH_MMP8 | 0,851 | 0,842 | 0,861 | 0,880 | 0,808 | 0,952 | 0,948 | 0,897 | 0,999 |
| RSAD2_OAS1_IFIT1_ISG15_OLAH | 0,851 | 0,841 | 0,861 | 0,880 | 0,808 | 0,952 | 0,943 | 0,891 | 0,996 |
| IFI27_OAS1_IFIT1_IFI44L_MMP8 | 0,851 | 0,841 | 0,861 | 0,877 | 0,807 | 0,948 | 0,911 | 0,836 | 0,986 |
| RSAD2_IFIT1_SIGLEC1_RETN_MMP8 | 0,851 | 0,841 | 0,862 | 0,880 | 0,805 | 0,955 | 0,942 | 0,889 | 0,996 |
| OAS1_IFIT1_HERC6_FAM20A_MMP8 | 0,851 | 0,841 | 0,862 | 0,881 | 0,812 | 0,951 | 0,921 | 0,836 | 1 |
| RSAD2_IFI44L_SLC1A2_FAM20A_RETN | 0,851 | 0,841 | 0,862 | 0,889 | 0,823 | 0,955 | 0,927 | 0,848 | 1 |
| IFI27_OAS1_IFIT1_ISG15_HERC6 | 0,851 | 0,841 | 0,862 | 0,881 | 0,820 | 0,942 | 0,920 | 0,856 | 0,983 |
| OAS1_IFI44L_ISG15_OLAH_RETN | 0,851 | 0,841 | 0,861 | 0,882 | 0,808 | 0,957 | 0,955 | 0,912 | 0,999 |
| RSAD2_IFIT1_IFI44L_HERC6_FAM20A | 0,851 | 0,840 | 0,862 | 0,881 | 0,811 | 0,951 | 0,918 | 0,837 | 1 |

| Modèle | AUC modèle | IC 95% | | AUC Train | IC 95% Train | | AUC Test | IC 95% Test | |
|---|---|---|---|---|---|---|---|---|---|
| OAS1_ IFIT1_ HERC6_ SLC1A2_ RETN | 0,851 | 0,841 | 0,861 | 0,882 | 0,812 | 0,952 | 0,938 | 0,873 | 1 |
| IFI44L_ISG15_ HERC6_ RETN_ MMP8 | 0,851 | 0,840 | 0,861 | 0,880 | 0,804 | 0,955 | 0,941 | 0,889 | 0,994 |
| OAS1_ IFIT1_ ISG15_ OLAH_ MMP8 | 0,851 | 0,841 | 0,861 | 0,879 | 0,807 | 0,952 | 0,946 | 0,895 | 0,997 |
| RSAD2_ IFI27_ IFI44L_ISG15_ HERC6 | 0,851 | 0,841 | 0,861 | 0,881 | 0,822 | 0,941 | 0,890 | 0,814 | 0,966 |
| RSAD2_ OAS1_ IFIT1_ IFI44L_ OLAH | 0,851 | 0,841 | 0,860 | 0,882 | 0,810 | 0,953 | 0,940 | 0,884 | 0,996 |
| RSAD2_IFI27_IFIT1_ IFI44L_ SIGLEC1 | 0,851 | 0,840 | 0,861 | 0,888 | 0,828 | 0,949 | 0,942 | 0,888 | 0,996 |
| IFI44L_ SIGLEC1_ ISG15_ HERC6_ MMP8 | 0,851 | 0,841 | 0,861 | 0,875 | 0,802 | 0,948 | 0,916 | 0,850 | 0,982 |
| IFI44L_ SIGLEC1_ ISG15_ HERC6_ SLC1A2 | 0,850 | 0,842 | 0,859 | 0,881 | 0,815 | 0,946 | 0,949 | 0,900 | 0,997 |
| RSAD2_ IFIT1_ HERC6_ FAM20A_ MMP8 | 0,850 | 0,840 | 0,861 | 0,879 | 0,809 | 0,949 | 0,925 | 0,842 | 1 |
| RSAD2_ IFI27_ OAS1_ ISG15_ HERC6 | 0,850 | 0,840 | 0,860 | 0,881 | 0,821 | 0,941 | 0,888 | 0,810 | 0,966 |
| RSAD2_ SIGLEC1_ ISG15_ HERC6_ MMP8 | 0,850 | 0,840 | 0,860 | 0,880 | 0,813 | 0,946 | 0,912 | 0,844 | 0,980 |
| IFIT1_ IFI44L_ OLAH_ RETN_ MMP8 | 0,850 | 0,840 | 0,861 | 0,879 | 0,804 | 0,954 | 0,962 | 0,922 | 1 |
| OAS1_ IFIT1_ ISG15_ FAM20A_ RETN | 0,850 | 0,840 | 0,861 | 0,881 | 0,807 | 0,955 | 0,941 | 0,866 | 1 |
| OAS1_ IFI44L_ SIGLEC1_ RETN_ MMP8 | 0,850 | 0,839 | 0,861 | 0,874 | 0,798 | 0,951 | 0,951 | 0,902 | 1 |
| RSAD2_ IFI27_ OAS1_ IFIT1_ MMP8 | 0,850 | 0,840 | 0,860 | 0,876 | 0,804 | 0,947 | 0,912 | 0,837 | 0,987 |
| OAS1_ IFIT1_ HERC6_ SLC1A2_ FAM20A | 0,850 | 0,840 | 0,860 | 0,882 | 0,816 | 0,948 | 0,929 | 0,845 | 1 |
| IFI27_ OAS1_ IFI44L_ ISG15_ HERC6 | 0,850 | 0,840 | 0,861 | 0,879 | 0,818 | 0,939 | 0,907 | 0,837 | 0,976 |
| IFIT1_ IFI44L_ ISG15_ OLAH_ RETN | 0,850 | 0,840 | 0,860 | 0,880 | 0,807 | 0,954 | 0,950 | 0,902 | 0,998 |
| RSAD2_ OAS1_ SIGLEC1_ RETN_ MMP8 | 0,850 | 0,839 | 0,861 | 0,876 | 0,800 | 0,952 | 0,949 | 0,900 | 0,998 |
| RSAD2_ OAS1_ HERC6_ SLC1A2_ RETN | 0,850 | 0,840 | 0,860 | 0,884 | 0,816 | 0,952 | 0,937 | 0,871 | 1 |

Performances obtenues pour l'identification de la nature virale ou bactérienne de l'infection sur la base des combinaisons à 6 gènes :

[Tableau 10]

| Modèle | AUC modèle | IC 95% | | AUC Train | IC 95% Train | | AUC Test | IC 95% Test | |
|---|---|---|---|---|---|---|---|---|---|
| IFI27 IFIT1 SIGLEC1 HERC6 OLAH FAM20A | 0,908 | 0,899 | 0,916 | 0,930 | 0,875 | 0,986 | 0,946 | 0,884 | 1 |
| IFI27 IFI44L SIGLEC1 IL1R2 FAM20A MMP8 | 0,907 | 0,899 | 0,914 | 0,931 | 0,880 | 0,983 | 0,952 | 0,885 | 1 |
| IFI27 IFIT1 SIGLEC1 IL1R2 FAM20A MMP8 | 0,906 | 0,898 | 0,914 | 0,932 | 0,880 | 0,984 | 0,957 | 0,894 | 1 |
| IFI27 OAS1 SIGLEC1 IL1R2 FAM20A MMP8 | 0,906 | 0,898 | 0,914 | 0,932 | 0,879 | 0,984 | 0,952 | 0,888 | 1 |
| RSAD2 IFI27 SIGLEC1 IL1R2 FAM20A MMP8 | 0,906 | 0,898 | 0,914 | 0,933 | 0,882 | 0,985 | 0,957 | 0,892 | 1 |
| IFI27 SIGLEC1 IL1R2 OLAH FAM20A MMP8 | 0,906 | 0,898 | 0,914 | 0,930 | 0,876 | 0,983 | 0,953 | 0,893 | 1 |
| IFI27 IFIT1 SIGLEC1 HERC6 IL1R2 FAM20A | 0,906 | 0,898 | 0,913 | 0,932 | 0,881 | 0,983 | 0,948 | 0,880 | 1 |
| IFI27 SIGLEC1 ISG15 IL1R2 FAM20A MMP8 | 0,905 | 0,897 | 0,913 | 0,932 | 0,880 | 0,984 | 0,953 | 0,885 | 1 |
| IFI27 IFIT1 SIGLEC1 OLAH FAM20A MMP8 | 0,905 | 0,896 | 0,914 | 0,927 | 0,869 | 0,985 | 0,957 | 0,902 | 1 |
| IFI27 OAS1 SIGLEC1 HERC6 OLAH FAM20A | 0,905 | 0,896 | 0,914 | 0,927 | 0,872 | 0,983 | 0,938 | 0,872 | 1 |
| IFI27 OAS1 SIGLEC1 HERC6 IL1R2 FAM20A | 0,905 | 0,896 | 0,913 | 0,930 | 0,878 | 0,983 | 0,948 | 0,881 | 1 |
| IFI27 SIGLEC1 HERC6 IL1R2 FAM20A MMP8 | 0,905 | 0,896 | 0,913 | 0,930 | 0,877 | 0,983 | 0,954 | 0,886 | 1 |
| RSAD2 IFI27 SIGLEC1 HERC6 IL1R2 FAM20A | 0,904 | 0,896 | 0,912 | 0,931 | 0,877 | 0,984 | 0,948 | 0,882 | 1 |
| IFI27 SIGLEC1 ISG15 OLAH FAM20A MMP8 | 0,904 | 0,895 | 0,913 | 0,924 | 0,864 | 0,984 | 0,950 | 0,891 | 1 |
| RSAD2 IFI27 HERC6 OLAH FAM20A MMP8 | 0,904 | 0,895 | 0,912 | 0,929 | 0,876 | 0,982 | 0,934 | 0,865 | 1 |
| IFI27 HERC6 IL1R2 OLAH FAM20A MMP8 | 0,904 | 0,895 | 0,912 | 0,932 | 0,880 | 0,983 | 0,935 | 0,863 | 1 |
| IFI27 IFI44L SIGLEC1 HERC6 IL1R2 FAM20A | 0,904 | 0,895 | 0,912 | 0,929 | 0,876 | 0,983 | 0,946 | 0,876 | 1 |
| RSAD2 IFI27 SIGLEC1 HERC6 OLAH FAM20A | 0,904 | 0,895 | 0,912 | 0,930 | 0,875 | 0,986 | 0,942 | 0,878 | 1 |
| IFI27 IFIT1 SIGLEC1 HERC6 FAM20A RETN | 0,903 | 0,895 | 0,912 | 0,928 | 0,873 | 0,983 | 0,940 | 0,867 | 1 |
| IFI27 IFIT1 SIGLEC1 IL1R2 OLAH FAM20A | 0,903 | 0,895 | 0,911 | 0,927 | 0,871 | 0,984 | 0,948 | 0,886 | 1 |
| IFI27 IFI44L IL1R2 OLAH FAM20A MMP8 | 0,903 | 0,895 | 0,912 | 0,929 | 0,874 | 0,983 | 0,932 | 0,859 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 SIGLEC1 IL1R2 FAM20A RETN MMP8 | 0,903 | 0,894 | 0,912 | 0,932 | 0,879 | 0,986 | 0,957 | 0,889 | 1 |
| IFI27 OAS1 HERC6 OLAH FAM20A MMP8 | 0,903 | 0,894 | 0,912 | 0,927 | 0,873 | 0,981 | 0,933 | 0,863 | 1 |
| IFI27 IFI44L SIGLEC1 OLAH FAM20A MMP8 | 0,903 | 0,894 | 0,912 | 0,924 | 0,864 | 0,984 | 0,955 | 0,898 | 1 |
| RSAD2 IFI27 SIGLEC1 OLAH FAM20A MMP8 | 0,903 | 0,894 | 0,912 | 0,927 | 0,869 | 0,985 | 0,951 | 0,895 | 1 |
| IFI27 IFIT1 SIGLEC1 ISG15 OLAH FAM20A | 0,903 | 0,894 | 0,911 | 0,926 | 0,868 | 0,985 | 0,941 | 0,879 | 1 |
| RSAD2 IFI27 HERC6 IL1R2 OLAH FAM20A | 0,903 | 0,895 | 0,911 | 0,929 | 0,877 | 0,982 | 0,929 | 0,857 | 1 |
| IFI27 IFIT1 IFI44L SIGLEC1 OLAH FAM20A | 0,903 | 0,894 | 0,911 | 0,925 | 0,866 | 0,984 | 0,952 | 0,897 | 1 |
| IFI27 ISG15 IL1R2 OLAH FAM20A MMP8 | 0,903 | 0,895 | 0,911 | 0,927 | 0,875 | 0,980 | 0,922 | 0,844 | 0,999 |
| IFI27 OAS1 HERC6 IL1R2 OLAH FAM20A | 0,903 | 0,894 | 0,911 | 0,930 | 0,878 | 0,981 | 0,927 | 0,852 | 1 |
| IFI27 ISG15 HERC6 OLAH FAM20A MMP8 | 0,902 | 0,893 | 0,911 | 0,925 | 0,868 | 0,982 | 0,937 | 0,872 | 1 |
| IFI27 IFI44L SIGLEC1 ISG15 OLAH FAM20A | 0,902 | 0,894 | 0,911 | 0,924 | 0,865 | 0,983 | 0,945 | 0,884 | 1 |
| IFI27 OAS1 SIGLEC1 OLAH FAM20A MMP8 | 0,902 | 0,893 | 0,911 | 0,923 | 0,862 | 0,984 | 0,950 | 0,893 | 1 |
| IFI27 OAS1 IFIT1 HERC6 OLAH FAM20A | 0,902 | 0,893 | 0,911 | 0,927 | 0,873 | 0,982 | 0,930 | 0,859 | 1 |
| RSAD2 IFI27 OAS1 HERC6 OLAH FAM20A | 0,902 | 0,893 | 0,911 | 0,927 | 0,873 | 0,981 | 0,930 | 0,859 | 1 |
| IFI27 ISG15 HERC6 IL1R2 OLAH FAM20A | 0,902 | 0,894 | 0,910 | 0,929 | 0,875 | 0,982 | 0,928 | 0,855 | 1 |
| IFI27 IFI44L SIGLEC1 ISG15 IL1R2 FAM20A | 0,902 | 0,894 | 0,910 | 0,928 | 0,874 | 0,982 | 0,947 | 0,880 | 1 |
| IFI27 IFI44L SIGLEC1 IL1R2 OLAH FAM20A | 0,902 | 0,894 | 0,910 | 0,926 | 0,870 | 0,982 | 0,951 | 0,889 | 1 |
| IFI27 IFIT1 IFI44L SIGLEC1 IL1R2 FAM20A | 0,902 | 0,894 | 0,910 | 0,927 | 0,872 | 0,982 | 0,949 | 0,886 | 1 |
| IFI27 IFIT1 HERC6 IL1R2 OLAH FAM20A | 0,902 | 0,894 | 0,910 | 0,929 | 0,877 | 0,982 | 0,928 | 0,853 | 1 |
| IFI27 OAS1 IFIT1 SIGLEC1 OLAH FAM20A | 0,902 | 0,893 | 0,910 | 0,923 | 0,863 | 0,983 | 0,950 | 0,891 | 1 |
| IFI27 OAS1 IFI44L HERC6 OLAH FAM20A | 0,902 | 0,893 | 0,911 | 0,926 | 0,870 | 0,982 | 0,933 | 0,862 | 1 |
| IFI27 IFIT1 IL1R2 OLAH FAM20A MMP8 | 0,902 | 0,893 | 0,910 | 0,929 | 0,876 | 0,982 | 0,935 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFI44L SIGLEC1 HERC6 OLAH FAM20A | 0,902 | 0,893 | 0,911 | 0,922 | 0,862 | 0,982 | 0,950 | 0,886 | 1 |
| IFI27 IFIT1 IFI44L HERC6 OLAH FAM20A | 0,902 | 0,893 | 0,911 | 0,925 | 0,869 | 0,982 | 0,933 | 0,863 | 1 |
| RSAD2 IFI27 IFI44L HERC6 OLAH FAM20A | 0,901 | 0,892 | 0,910 | 0,929 | 0,875 | 0,983 | 0,932 | 0,863 | 1,000 |
| IFI27 IFIT1 SIGLEC1 ISG15 IL1R2 FAM20A | 0,901 | 0,894 | 0,909 | 0,927 | 0,872 | 0,983 | 0,948 | 0,883 | 1 |
| IFI27 SIGLEC1 ISG15 IL1R2 OLAH FAM20A | 0,901 | 0,893 | 0,910 | 0,928 | 0,872 | 0,983 | 0,945 | 0,881 | 1 |
| IFI27 SIGLEC1 ISG15 HERC6 OLAH FAM20A | 0,901 | 0,892 | 0,911 | 0,923 | 0,863 | 0,983 | 0,945 | 0,882 | 1 |
| IFI27 IFIT1 HERC6 OLAH FAM20A MMP8 | 0,901 | 0,892 | 0,910 | 0,924 | 0,867 | 0,981 | 0,938 | 0,873 | 1 |
| IFI27 IFI44L ISG15 HERC6 OLAH FAM20A | 0,901 | 0,892 | 0,910 | 0,925 | 0,868 | 0,982 | 0,935 | 0,868 | 1 |
| IFI27 IFI44L HERC6 IL1R2 OLAH FAM20A | 0,901 | 0,893 | 0,910 | 0,928 | 0,874 | 0,982 | 0,935 | 0,863 | 1 |
| IFI27 OAS1 SIGLEC1 IL1R2 OLAH FAM20A | 0,901 | 0,893 | 0,910 | 0,924 | 0,866 | 0,982 | 0,948 | 0,886 | 1 |
| IFI27 OAS1 ISG15 HERC6 OLAH FAM20A | 0,901 | 0,892 | 0,910 | 0,929 | 0,874 | 0,983 | 0,927 | 0,854 | 1 |
| IFI27 OAS1 IFIT1 SIGLEC1 IL1R2 FAM20A | 0,901 | 0,893 | 0,909 | 0,927 | 0,871 | 0,983 | 0,950 | 0,889 | 1 |
| RSAD2 IFI27 IL1R2 OLAH FAM20A MMP8 | 0,901 | 0,893 | 0,909 | 0,929 | 0,876 | 0,982 | 0,930 | 0,857 | 1 |
| RSAD2 IFI27 IFIT1 HERC6 OLAH FAM20A | 0,901 | 0,892 | 0,910 | 0,927 | 0,872 | 0,981 | 0,932 | 0,862 | 1 |
| IFI27 OAS1 SIGLEC1 HERC6 FAM20A RETN | 0,901 | 0,892 | 0,910 | 0,928 | 0,873 | 0,983 | 0,938 | 0,864 | 1 |
| IFI27 OAS1 SIGLEC1 ISG15 OLAH FAM20A | 0,901 | 0,892 | 0,910 | 0,923 | 0,863 | 0,983 | 0,947 | 0,887 | 1 |
| IFI27 SIGLEC1 HERC6 IL1R2 OLAH FAM20A | 0,901 | 0,892 | 0,909 | 0,925 | 0,867 | 0,982 | 0,950 | 0,889 | 1 |
| IFI27 OAS1 IL1R2 OLAH FAM20A MMP8 | 0,901 | 0,892 | 0,909 | 0,929 | 0,876 | 0,982 | 0,932 | 0,859 | 1 |
| IFI27 IFIT1 ISG15 HERC6 OLAH FAM20A | 0,901 | 0,892 | 0,910 | 0,926 | 0,869 | 0,982 | 0,935 | 0,868 | 1 |
| IFI27 IFIT1 SIGLEC1 IL1R2 FAM20A RETN | 0,901 | 0,892 | 0,909 | 0,927 | 0,871 | 0,982 | 0,949 | 0,886 | 1 |
| IFI27 IFIT1 SIGLEC1 OLAH FAM20A RETN | 0,900 | 0,892 | 0,909 | 0,923 | 0,863 | 0,983 | 0,950 | 0,891 | 1 |
| RSAD2 IFI27 ISG15 HERC6 OLAH FAM20A | 0,900 | 0,892 | 0,909 | 0,927 | 0,873 | 0,982 | 0,929 | 0,859 | 1,000 |

(suite)

| Modèle | AUC modèle | IC 95% | | AUC Train | IC 95% Train | | AUC Test | IC 95% Test | |
|---|---|---|---|---|---|---|---|---|---|
| IFI27 SIGLEC1 ISG15 HERC6 IL1R2 FAM20A | 0,900 | 0,892 | 0,909 | 0,927 | 0,873 | 0,982 | 0,949 | 0,881 | 1 |
| IFI27 SIGLEC1 HERC6 OLAH FAM20A MMP8 | 0,900 | 0,891 | 0,910 | 0,921 | 0,859 | 0,984 | 0,953 | 0,895 | 1 |
| RSAD2 IFI27 IFIT1 SIGLEC1 OLAH FAM20A | 0,900 | 0,892 | 0,909 | 0,924 | 0,865 | 0,984 | 0,951 | 0,893 | 1 |
| IFI27 OAS1 HERC6 OLAH FAM20A RETN | 0,900 | 0,891 | 0,909 | 0,931 | 0,880 | 0,983 | 0,935 | 0,865 | 1 |
| RSAD2 IFI27 SIGLEC1 IL1R2 OLAH FAM20A | 0,900 | 0,892 | 0,909 | 0,927 | 0,869 | 0,984 | 0,948 | 0,885 | 1 |
| RSAD2 IFI27 OAS1 SIGLEC1 IL1R2 FAM20A | 0,900 | 0,892 | 0,908 | 0,927 | 0,871 | 0,984 | 0,951 | 0,888 | 1 |
| IFI27 OAS1 IFI44L SIGLEC1 IL1R2 FAM20A | 0,900 | 0,892 | 0,908 | 0,928 | 0,874 | 0,982 | 0,950 | 0,887 | 1 |
| IFI27 ISG15 HERC6 OLAH FAM20A RETN | 0,900 | 0,891 | 0,909 | 0,925 | 0,868 | 0,982 | 0,936 | 0,869 | 1 |
| IFI27 IFI44L ISG15 OLAH FAM20A MMP8 | 0,900 | 0,891 | 0,909 | 0,922 | 0,863 | 0,982 | 0,936 | 0,870 | 1 |
| IFI27 IFI44L SIGLEC1 IL1R2 FAM20A RETN | 0,900 | 0,891 | 0,908 | 0,927 | 0,872 | 0,982 | 0,948 | 0,881 | 1 |
| IFI27 OAS1 HERC6 IL1R2 FAM20A MMP8 | 0,900 | 0,891 | 0,908 | 0,930 | 0,880 | 0,979 | 0,924 | 0,837 | 1 |
| IFI27 IFI44L HERC6 OLAH FAM20A MMP8 | 0,900 | 0,890 | 0,909 | 0,924 | 0,866 | 0,981 | 0,940 | 0,876 | 1 |
| IFI27 OAS1 IFI44L SIGLEC1 OLAH FAM20A | 0,900 | 0,891 | 0,908 | 0,923 | 0,863 | 0,982 | 0,951 | 0,892 | 1 |
| IFI27 IFI44L HERC6 IL1R2 FAM20A MMP8 | 0,900 | 0,891 | 0,908 | 0,929 | 0,879 | 0,980 | 0,927 | 0,841 | 1 |
| RSAD2 IFI27 HERC6 IL1R2 FAM20A MMP8 | 0,900 | 0,891 | 0,908 | 0,929 | 0,879 | 0,979 | 0,926 | 0,841 | 1 |
| RSAD2 IFI27 HERC6 OLAH FAM20A RETN | 0,899 | 0,891 | 0,908 | 0,929 | 0,876 | 0,982 | 0,936 | 0,868 | 1 |
| IFI27 OAS1 SIGLEC1 ISG15 IL1R2 FAM20A | 0,899 | 0,891 | 0,908 | 0,927 | 0,872 | 0,983 | 0,949 | 0,882 | 1 |
| IFI27 IFIT1 HERC6 OLAH FAM20A RETN | 0,899 | 0,891 | 0,908 | 0,927 | 0,872 | 0,982 | 0,934 | 0,863 | 1 |
| RSAD2 IFI27 SIGLEC1 HERC6 FAM20A RETN | 0,899 | 0,890 | 0,908 | 0,929 | 0,876 | 0,983 | 0,946 | 0,876 | 1 |
| RSAD2 IFI27 SIGLEC1 ISG15 OLAH FAM20A | 0,899 | 0,890 | 0,908 | 0,924 | 0,865 | 0,983 | 0,945 | 0,883 | 1 |
| RSAD2 IFI27 OAS1 SIGLEC1 OLAH FAM20A | 0,899 | 0,890 | 0,908 | 0,924 | 0,864 | 0,984 | 0,951 | 0,893 | 1 |
| RSAD2 IFI27 IFIT1 SIGLEC1 IL1R2 FAM20A | 0,899 | 0,891 | 0,907 | 0,926 | 0,870 | 0,982 | 0,949 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 IFI44L SIGLEC1 IL1R2 FAM20A | 0,899 | 0,891 | 0,907 | 0,928 | 0,873 | 0,982 | 0,950 | 0,886 | 1 |
| IFI27 OAS1 SIGLEC1 IL1R2 FAM20A RETN | 0,899 | 0,890 | 0,908 | 0,928 | 0,872 | 0,984 | 0,950 | 0,886 | 1 |
| IFI27 SIGLEC1 ISG15 OLAH FAM20A RETN | 0,899 | 0,890 | 0,908 | 0,921 | 0,860 | 0,982 | 0,949 | 0,889 | 1 |
| IFI27 SIGLEC1 OLAH FAM20A RETN MMP8 | 0,899 | 0,889 | 0,908 | 0,924 | 0,865 | 0,984 | 0,957 | 0,899 | 1 |
| IFI27 HERC6 OLAH FAM20A RETN MMP8 | 0,899 | 0,889 | 0,908 | 0,927 | 0,869 | 0,984 | 0,943 | 0,878 | 1 |
| RSAD2 IFI27 SIGLEC1 IL1R2 FAM20A RETN | 0,899 | 0,890 | 0,907 | 0,929 | 0,874 | 0,984 | 0,948 | 0,881 | 1 |
| IFI27 OAS1 IFI44L HERC6 IL1R2 FAM20A | 0,899 | 0,891 | 0,907 | 0,926 | 0,875 | 0,978 | 0,926 | 0,842 | 1 |
| RSAD2 IFI27 SIGLEC1 ISG15 IL1R2 FAM20A | 0,899 | 0,890 | 0,907 | 0,928 | 0,873 | 0,983 | 0,946 | 0,878 | 1 |
| IFI27 IFI44L ISG15 HERC6 IL1R2 FAM20A | 0,899 | 0,890 | 0,907 | 0,927 | 0,876 | 0,978 | 0,930 | 0,849 | 1 |
| IFI27 IFI44L ISG15 IL1R2 OLAH FAM20A | 0,899 | 0,890 | 0,907 | 0,923 | 0,866 | 0,980 | 0,929 | 0,858 | 1 |
| IFI27 SIGLEC1 ISG15 IL1R2 FAM20A RETN | 0,898 | 0,890 | 0,907 | 0,928 | 0,873 | 0,983 | 0,950 | 0,883 | 1 |
| IFI27 IFIT1 HERC6 IL1R2 FAM20A MMP8 | 0,898 | 0,890 | 0,907 | 0,929 | 0,879 | 0,980 | 0,934 | 0,854 | 1 |
| IFI27 IFIT1 IFI44L HERC6 IL1R2 FAM20A | 0,898 | 0,890 | 0,907 | 0,927 | 0,876 | 0,978 | 0,932 | 0,851 | 1 |
| IFI27 IL1R2 OLAH FAM20A RETN MMP8 | 0,898 | 0,890 | 0,907 | 0,929 | 0,875 | 0,982 | 0,925 | 0,849 | 1 |
| RSAD2 IFI27 IFI44L SIGLEC1 OLAH FAM20A | 0,898 | 0,890 | 0,907 | 0,925 | 0,867 | 0,984 | 0,951 | 0,892 | 1 |
| IFI27 OAS1 SIGLEC1 OLAH FAM20A RETN | 0,898 | 0,889 | 0,907 | 0,924 | 0,865 | 0,983 | 0,948 | 0,886 | 1 |
| IFI27 IFI44L ISG15 IL1R2 FAM20A MMP8 | 0,898 | 0,890 | 0,906 | 0,927 | 0,874 | 0,979 | 0,926 | 0,841 | 1 |
| IFI27 OAS1 ISG15 HERC6 IL1R2 FAM20A | 0,898 | 0,890 | 0,906 | 0,927 | 0,877 | 0,978 | 0,923 | 0,837 | 1 |
| RSAD2 IFI27 IFI44L HERC6 IL1R2 FAM20A | 0,898 | 0,890 | 0,907 | 0,928 | 0,877 | 0,979 | 0,929 | 0,847 | 1 |
| IFI27 ISG15 HERC6 IL1R2 FAM20A MMP8 | 0,898 | 0,889 | 0,907 | 0,929 | 0,879 | 0,979 | 0,926 | 0,841 | 1 |
| IFI27 IFIT1 SIGLEC1 SLC1A2 OLAH FAM20A | 0,898 | 0,889 | 0,907 | 0,930 | 0,876 | 0,983 | 0,942 | 0,878 | 1 |
| IFI27 OAS1 HERC6 IL1R2 FAM20A RETN | 0,898 | 0,890 | 0,906 | 0,932 | 0,881 | 0,982 | 0,928 | 0,847 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 HERC6 IL1R2 FAM20A RETN | 0,898 | 0,889 | 0,906 | 0,931 | 0,880 | 0,982 | 0,930 | 0,850 | 1 |
| RSAD2 IFI27 OAS1 HERC6 IL1R2 FAM20A | 0,898 | 0,890 | 0,906 | 0,930 | 0,880 | 0,979 | 0,923 | 0,837 | 1 |
| IFI27 IFIT1 IFI44L OLAH FAM20A MMP8 | 0,898 | 0,889 | 0,907 | 0,921 | 0,861 | 0,981 | 0,938 | 0,873 | 1 |
| IFI27 OAS1 IFIT1 HERC6 IL1R2 FAM20A | 0,898 | 0,889 | 0,906 | 0,927 | 0,876 | 0,979 | 0,926 | 0,843 | 1 |
| IFI27 HERC6 IL1R2 OLAH FAM20A RETN | 0,898 | 0,889 | 0,906 | 0,926 | 0,871 | 0,982 | 0,939 | 0,870 | 1 |
| IFI27 SIGLEC1 ISG15 SLC1A2 OLAH FAM20A | 0,898 | 0,889 | 0,907 | 0,930 | 0,878 | 0,983 | 0,937 | 0,868 | 1 |
| IFI27 IFIT1 ISG15 OLAH FAM20A MMP8 | 0,898 | 0,889 | 0,907 | 0,921 | 0,861 | 0,981 | 0,936 | 0,871 | 1 |
| IFI27 IFI44L SIGLEC1 OLAH FAM20A RETN | 0,898 | 0,889 | 0,906 | 0,920 | 0,859 | 0,982 | 0,953 | 0,895 | 1 |
| IFI27 HERC6 IL1R2 FAM20A RETN MMP8 | 0,898 | 0,889 | 0,907 | 0,931 | 0,879 | 0,982 | 0,932 | 0,851 | 1 |
| RSAD2 IFI27 SIGLEC1 OLAH FAM20A RETN | 0,898 | 0,889 | 0,907 | 0,924 | 0,865 | 0,984 | 0,950 | 0,891 | 1 |
| IFI27 SIGLEC1 SLC1A2 IL1R2 FAM20A MMP8 | 0,898 | 0,889 | 0,906 | 0,936 | 0,891 | 0,981 | 0,954 | 0,885 | 1 |
| IFI27 IFIT1 SIGLEC1 ISG15 FAM20A RETN | 0,898 | 0,889 | 0,906 | 0,920 | 0,861 | 0,980 | 0,946 | 0,879 | 1 |
| IFI27 IFI44L HERC6 OLAH FAM20A RETN | 0,898 | 0,889 | 0,907 | 0,923 | 0,866 | 0,981 | 0,936 | 0,866 | 1 |
| IFI27 IFI44L HERC6 IL1R2 FAM20A RETN | 0,898 | 0,889 | 0,906 | 0,931 | 0,880 | 0,982 | 0,929 | 0,849 | 1 |
| IFI27 IFIT1 IFI44L IL1R2 FAM20A MMP8 | 0,898 | 0,889 | 0,906 | 0,926 | 0,873 | 0,978 | 0,936 | 0,858 | 1 |
| IFI27 ISG15 OLAH FAM20A RETN MMP8 | 0,898 | 0,888 | 0,907 | 0,924 | 0,868 | 0,981 | 0,930 | 0,858 | 1 |
| IFI27 SIGLEC1 IL1R2 OLAH FAM20A RETN | 0,897 | 0,889 | 0,906 | 0,924 | 0,866 | 0,982 | 0,950 | 0,890 | 1 |
| IFI27 ISG15 IL1R2 OLAH FAM20A RETN | 0,897 | 0,889 | 0,906 | 0,926 | 0,871 | 0,981 | 0,925 | 0,849 | 1 |
| RSAD2 IFI27 IFIT1 HERC6 IL1R2 FAM20A | 0,897 | 0,889 | 0,906 | 0,928 | 0,877 | 0,979 | 0,930 | 0,850 | 1 |
| IFI27 SIGLEC1 HERC6 IL1R2 FAM20A RETN | 0,897 | 0,888 | 0,906 | 0,927 | 0,872 | 0,982 | 0,950 | 0,883 | 1 |
| IFI27 OAS1 IFIT1 SIGLEC1 HERC6 RETN | 0,897 | 0,889 | 0,906 | 0,919 | 0,862 | 0,977 | 0,942 | 0,883 | 1 |
| IFI27 IFIT1 IFI44L SIGLEC1 FAM20A RETN | 0,897 | 0,888 | 0,906 | 0,920 | 0,860 | 0,979 | 0,947 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 ISG15 HERC6 IL1R2 FAM20A RETN | 0,897 | 0,889 | 0,906 | 0,931 | 0,880 | 0,982 | 0,929 | 0,849 | 1 |
| IFI27 IFIT1 IFI44L IL1R2 OLAH FAM20A | 0,897 | 0,889 | 0,906 | 0,923 | 0,866 | 0,981 | 0,932 | 0,860 | 1 |
| RSAD2 IFI27 OAS1 OLAH FAM20A MMP8 | 0,897 | 0,888 | 0,906 | 0,922 | 0,863 | 0,980 | 0,935 | 0,869 | 1 |
| IFI27 SLC1A2 IL1R2 OLAH FAM20A MMP8 | 0,897 | 0,888 | 0,906 | 0,930 | 0,881 | 0,979 | 0,920 | 0,839 | 1 |
| IFI27 IFIT1 SIGLEC1 FAM20A RETN MMP8 | 0,897 | 0,888 | 0,906 | 0,923 | 0,864 | 0,982 | 0,948 | 0,876 | 1 |
| IFI27 OAS1 ISG15 OLAH FAM20A MMP8 | 0,897 | 0,888 | 0,906 | 0,920 | 0,861 | 0,980 | 0,934 | 0,867 | 1 |
| IFI27 IFI44L IL1R2 FAM20A RETN MMP8 | 0,897 | 0,888 | 0,906 | 0,928 | 0,875 | 0,981 | 0,929 | 0,848 | 1 |
| IFI27 OAS1 IFIT1 IL1R2 FAM20A MMP8 | 0,897 | 0,889 | 0,905 | 0,925 | 0,873 | 0,978 | 0,933 | 0,854 | 1 |
| IFI27 SIGLEC1 SLC1A2 OLAH FAM20A MMP8 | 0,897 | 0,888 | 0,906 | 0,927 | 0,872 | 0,983 | 0,952 | 0,890 | 1 |
| IFIT1 SIGLEC1 HERC6 IL1R2 FAM20A MMP8 | 0,897 | 0,888 | 0,905 | 0,923 | 0,869 | 0,978 | 0,963 | 0,907 | 1 |
| IFI27 IFIT1 SIGLEC1 ISG15 HERC6 FAM20A | 0,897 | 0,888 | 0,905 | 0,921 | 0,861 | 0,981 | 0,942 | 0,867 | 1 |
| IFI27 OAS1 IFIT1 OLAH FAM20A MMP8 | 0,897 | 0,888 | 0,905 | 0,921 | 0,862 | 0,981 | 0,940 | 0,877 | 1 |
| IFI27 OAS1 SIGLEC1 SLC1A2 OLAH FAM20A | 0,897 | 0,888 | 0,906 | 0,928 | 0,872 | 0,984 | 0,941 | 0,878 | 1 |
| RSAD2 IFI27 ISG15 HERC6 IL1R2 FAM20A | 0,897 | 0,888 | 0,905 | 0,927 | 0,877 | 0,978 | 0,926 | 0,843 | 1 |
| IFI27 OAS1 IFIT1 IL1R2 OLAH FAM20A | 0,896 | 0,888 | 0,905 | 0,924 | 0,867 | 0,981 | 0,933 | 0,863 | 1 |
| IFI27 IFIT1 ISG15 IL1R2 OLAH FAM20A | 0,896 | 0,888 | 0,905 | 0,924 | 0,867 | 0,981 | 0,932 | 0,861 | 1 |
| IFI27 IFI44L SIGLEC1 HERC6 FAM20A RETN | 0,896 | 0,888 | 0,905 | 0,924 | 0,867 | 0,982 | 0,945 | 0,869 | 1 |
| IFI27 OAS1 HERC6 OLAH RETN MMP8 | 0,896 | 0,888 | 0,905 | 0,924 | 0,868 | 0,980 | 0,943 | 0,884 | 1 |
| IFI27 IFIT1 HERC6 IL1R2 FAM20A RETN | 0,896 | 0,888 | 0,905 | 0,931 | 0,880 | 0,982 | 0,929 | 0,849 | 1 |
| IFI27 IFI44L SIGLEC1 HERC6 IL1R2 OLAH | 0,896 | 0,888 | 0,904 | 0,927 | 0,879 | 0,976 | 0,942 | 0,890 | 0,994 |
| IFI27 SIGLEC1 HERC6 OLAH FAM20A RETN | 0,896 | 0,887 | 0,906 | 0,919 | 0,858 | 0,981 | 0,950 | 0,890 | 1 |
| IFI27 IFI44L OLAH FAM20A RETN MMP8 | 0,896 | 0,887 | 0,906 | 0,924 | 0,864 | 0,983 | 0,941 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 HERC6 SLC1A2 OLAH FAM20A | 0,896 | 0,887 | 0,905 | 0,929 | 0,878 | 0,980 | 0,918 | 0,835 | 1 |
| IFI27 IFI44L SLC1A2 OLAH FAM20A MMP8 | 0,896 | 0,887 | 0,905 | 0,926 | 0,870 | 0,981 | 0,928 | 0,852 | 1 |
| RSAD2 IFI27 IFIT1 IL1R2 FAM20A MMP8 | 0,896 | 0,888 | 0,904 | 0,926 | 0,874 | 0,978 | 0,933 | 0,854 | 1 |
| IFI27 OAS1 SIGLEC1 ISG15 HERC6 RETN | 0,896 | 0,887 | 0,905 | 0,921 | 0,863 | 0,979 | 0,945 | 0,882 | 1 |
| IFI27 IFIT1 SIGLEC1 HERC6 FAM20A MMP8 | 0,896 | 0,888 | 0,905 | 0,921 | 0,861 | 0,980 | 0,939 | 0,864 | 1 |
| RSAD2 IFI27 OAS1 IL1R2 OLAH FAM20A | 0,896 | 0,888 | 0,905 | 0,923 | 0,866 | 0,980 | 0,930 | 0,858 | 1 |
| IFI27 IFIT1 ISG15 HERC6 IL1R2 FAM20A | 0,896 | 0,888 | 0,905 | 0,927 | 0,876 | 0,978 | 0,933 | 0,853 | 1 |
| IFI27 OAS1 SIGLEC1 HERC6 OLAH RETN | 0,896 | 0,888 | 0,905 | 0,923 | 0,867 | 0,978 | 0,946 | 0,890 | 1 |
| RSAD2 IFI27 IFI44L OLAH FAM20A MMP8 | 0,896 | 0,886 | 0,906 | 0,922 | 0,863 | 0,982 | 0,935 | 0,868 | 1 |
| RSAD2 IFI27 ISG15 OLAH FAM20A MMP8 | 0,896 | 0,887 | 0,905 | 0,921 | 0,861 | 0,981 | 0,933 | 0,865 | 1 |
| IFI27 OAS1 ISG15 IL1R2 OLAH FAM20A | 0,896 | 0,887 | 0,905 | 0,925 | 0,869 | 0,981 | 0,927 | 0,855 | 0,999 |
| IFI27 IFI44L SIGLEC1 HERC6 OLAH RETN | 0,896 | 0,887 | 0,905 | 0,922 | 0,866 | 0,978 | 0,948 | 0,896 | 1,000 |
| IFI27 IFIT1 IFI44L ISG15 OLAH FAM20A | 0,896 | 0,887 | 0,905 | 0,920 | 0,859 | 0,981 | 0,935 | 0,867 | 1 |
| IFI27 OAS1 ISG15 HERC6 FAM20A RETN | 0,896 | 0,887 | 0,905 | 0,924 | 0,867 | 0,980 | 0,935 | 0,857 | 1 |
| IFI27 OAS1 IFI44L OLAH FAM20A MMP8 | 0,896 | 0,886 | 0,905 | 0,922 | 0,862 | 0,982 | 0,935 | 0,867 | 1 |
| IFI27 IFIT1 IFI44L SIGLEC1 HERC6 FAM20A | 0,896 | 0,888 | 0,904 | 0,920 | 0,861 | 0,980 | 0,941 | 0,866 | 1 |
| RSAD2 IFI27 IFIT1 OLAH FAM20A MMP8 | 0,896 | 0,887 | 0,905 | 0,922 | 0,864 | 0,981 | 0,940 | 0,876 | 1 |
| RSAD2 IFI27 ISG15 IL1R2 OLAH FAM20A | 0,896 | 0,887 | 0,904 | 0,924 | 0,867 | 0,980 | 0,927 | 0,853 | 1 |
| IFI27 IFI44L ISG15 SLC1A2 OLAH FAM20A | 0,896 | 0,887 | 0,905 | 0,929 | 0,875 | 0,983 | 0,917 | 0,836 | 0,999 |
| RSAD2 IFI27 HERC6 SLC1A2 OLAH FAM20A | 0,896 | 0,887 | 0,905 | 0,928 | 0,875 | 0,980 | 0,922 | 0,841 | 1 |
| IFI27 IFI44L IL1R2 OLAH FAM20A RETN | 0,896 | 0,887 | 0,904 | 0,924 | 0,866 | 0,981 | 0,930 | 0,858 | 1 |
| IFI27 ISG15 SLC1A2 OLAH FAM20A MMP8 | 0,896 | 0,887 | 0,905 | 0,927 | 0,873 | 0,980 | 0,922 | 0,843 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 HERC6 OLAH RETN | 0,896 | 0,887 | 0,904 | 0,922 | 0,867 | 0,978 | 0,939 | 0,878 | 1,000 |
| IFI27 OAS1 IFIT1 SIGLEC1 HERC6 FAM20A | 0,896 | 0,887 | 0,904 | 0,923 | 0,866 | 0,980 | 0,938 | 0,865 | 1 |
| RSAD2 IFI27 OAS1 SIGLEC1 HERC6 RETN | 0,896 | 0,887 | 0,904 | 0,918 | 0,859 | 0,978 | 0,949 | 0,888 | 1 |
| IFI27 OAS1 SIGLEC1 HERC6 IL1R2 RETN | 0,896 | 0,888 | 0,904 | 0,924 | 0,872 | 0,977 | 0,949 | 0,894 | 1 |
| IFI27 IFIT1 SIGLEC1 HERC6 OLAH RETN | 0,896 | 0,887 | 0,904 | 0,923 | 0,869 | 0,978 | 0,947 | 0,894 | 0,999 |
| IFI27 ISG15 HERC6 SLC1A2 OLAH FAM20A | 0,896 | 0,886 | 0,905 | 0,931 | 0,880 | 0,982 | 0,915 | 0,832 | 0,998 |
| IFI27 IFI44L SIGLEC1 ISG15 HERC6 OLAH | 0,895 | 0,887 | 0,904 | 0,925 | 0,873 | 0,977 | 0,945 | 0,892 | 0,997 |
| RSAD2 IFI27 HERC6 OLAH RETN MMP8 | 0,895 | 0,887 | 0,904 | 0,924 | 0,867 | 0,980 | 0,945 | 0,889 | 1 |
| IFI27 OAS1 IFI44L IL1R2 FAM20A MMP8 | 0,895 | 0,887 | 0,904 | 0,926 | 0,873 | 0,979 | 0,930 | 0,847 | 1 |
| IFI27 OAS1 IFI44L SIGLEC1 HERC6 RETN | 0,895 | 0,887 | 0,903 | 0,919 | 0,861 | 0,977 | 0,943 | 0,883 | 1 |
| IFI27 IFI44L ISG15 OLAH FAM20A RETN | 0,895 | 0,886 | 0,904 | 0,921 | 0,861 | 0,981 | 0,935 | 0,865 | 1 |
| RSAD2 IFI27 IFI44L IL1R2 FAM20A MMP8 | 0,895 | 0,887 | 0,904 | 0,926 | 0,874 | 0,979 | 0,928 | 0,845 | 1 |
| RSAD2 IFI27 SIGLEC1 SLC1A2 OLAH FAM20A | 0,895 | 0,886 | 0,904 | 0,927 | 0,871 | 0,984 | 0,943 | 0,879 | 1 |
| IFI27 IFI44L SIGLEC1 SLC1A2 OLAH FAM20A | 0,895 | 0,886 | 0,904 | 0,925 | 0,867 | 0,983 | 0,946 | 0,879 | 1 |
| IFI27 IFIT1 ISG15 IL1R2 FAM20A MMP8 | 0,895 | 0,887 | 0,904 | 0,927 | 0,875 | 0,979 | 0,933 | 0,854 | 1 |
| IFI27 IFIT1 OLAH FAM20A RETN MMP8 | 0,895 | 0,886 | 0,905 | 0,923 | 0,864 | 0,982 | 0,943 | 0,879 | 1 |
| IFI27 IFIT1 IL1R2 FAM20A RETN MMP8 | 0,895 | 0,886 | 0,904 | 0,929 | 0,875 | 0,982 | 0,937 | 0,860 | 1 |
| IFI27 OAS1 IFIT1 IFI44L OLAH FAM20A | 0,895 | 0,886 | 0,904 | 0,920 | 0,859 | 0,981 | 0,937 | 0,869 | 1 |
| IFI27 OAS1 SIGLEC1 FAM20A RETN MMP8 | 0,895 | 0,885 | 0,905 | 0,922 | 0,861 | 0,982 | 0,947 | 0,875 | 1 |
| RSAD2 IFI27 IFIT1 IL1R2 OLAH FAM20A | 0,895 | 0,887 | 0,904 | 0,924 | 0,867 | 0,981 | 0,933 | 0,863 | 1 |
| RSAD2 IFI27 SIGLEC1 HERC6 IL1R2 OLAH | 0,895 | 0,887 | 0,903 | 0,926 | 0,877 | 0,975 | 0,941 | 0,888 | 0,995 |
| IFI27 OAS1 IFI44L IL1R2 OLAH FAM20A | 0,895 | 0,886 | 0,904 | 0,923 | 0,866 | 0,981 | 0,929 | 0,856 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 HERC6 OLAH RETN MMP8 | 0,895 | 0,886 | 0,904 | 0,922 | 0,864 | 0,980 | 0,949 | 0,896 | 1 |
| IFI27 OAS1 IFIT1 SIGLEC1 FAM20A RETN | 0,895 | 0,886 | 0,904 | 0,919 | 0,860 | 0,979 | 0,946 | 0,878 | 1 |
| IFI27 IFI44L SIGLEC1 HERC6 OLAH MMP8 | 0,895 | 0,887 | 0,903 | 0,924 | 0,871 | 0,978 | 0,946 | 0,895 | 0,997 |
| IFI27 OAS1 SIGLEC1 HERC6 IL1R2 OLAH | 0,895 | 0,887 | 0,903 | 0,924 | 0,875 | 0,974 | 0,943 | 0,891 | 0,996 |
| IFI27 IFIT1 SIGLEC1 SLC1A2 IL1R2 FAM20A | 0,895 | 0,886 | 0,904 | 0,934 | 0,887 | 0,981 | 0,943 | 0,874 | 1 |
| RSAD2 IFI27 SLC1A2 OLAH FAM20A MMP8 | 0,895 | 0,886 | 0,904 | 0,924 | 0,869 | 0,980 | 0,925 | 0,848 | 1 |
| RSAD2 IFI27 IFIT1 SIGLEC1 HERC6 FAM20A | 0,895 | 0,886 | 0,904 | 0,922 | 0,865 | 0,979 | 0,941 | 0,871 | 1 |
| RSAD2 IFI27 SIGLEC1 HERC6 OLAH MMP8 | 0,895 | 0,887 | 0,903 | 0,922 | 0,869 | 0,974 | 0,937 | 0,881 | 0,993 |
| IFI27 OAS1 IFI44L ISG15 OLAH FAM20A | 0,895 | 0,886 | 0,904 | 0,920 | 0,860 | 0,981 | 0,935 | 0,868 | 1 |
| IFI27 IFI44L SIGLEC1 SLC1A2 IL1R2 FAM20A | 0,895 | 0,886 | 0,904 | 0,928 | 0,878 | 0,979 | 0,948 | 0,879 | 1 |
| IFI27 OAS1 OLAH FAM20A RETN MMP8 | 0,895 | 0,886 | 0,904 | 0,924 | 0,866 | 0,982 | 0,939 | 0,872 | 1 |
| IFI27 OAS1 SIGLEC1 SLC1A2 IL1R2 FAM20A | 0,895 | 0,886 | 0,904 | 0,932 | 0,884 | 0,980 | 0,943 | 0,873 | 1 |
| IFI27 OAS1 HERC6 IL1R2 OLAH MMP8 | 0,895 | 0,887 | 0,903 | 0,927 | 0,879 | 0,976 | 0,938 | 0,880 | 0,996 |
| IFI27 IFI44L SIGLEC1 IL1R2 OLAH MMP8 | 0,895 | 0,887 | 0,903 | 0,924 | 0,873 | 0,974 | 0,947 | 0,896 | 0,998 |
| IFI27 IFIT1 IFI44L HERC6 FAM20A RETN | 0,895 | 0,886 | 0,904 | 0,921 | 0,862 | 0,979 | 0,928 | 0,845 | 1 |
| IFI27 OAS1 IFIT1 SIGLEC1 HERC6 OLAH | 0,895 | 0,887 | 0,903 | 0,924 | 0,872 | 0,975 | 0,940 | 0,885 | 0,995 |
| IFI27 OAS1 IFI44L HERC6 FAM20A RETN | 0,895 | 0,886 | 0,904 | 0,923 | 0,864 | 0,981 | 0,935 | 0,856 | 1 |
| IFI27 OAS1 IFIT1 ISG15 OLAH FAM20A | 0,895 | 0,886 | 0,904 | 0,921 | 0,861 | 0,981 | 0,934 | 0,866 | 1 |
| IFI27 IFI44L HERC6 OLAH RETN MMP8 | 0,895 | 0,886 | 0,904 | 0,922 | 0,864 | 0,980 | 0,948 | 0,895 | 1 |
| IFI27 ISG15 SLC1A2 IL1R2 OLAH FAM20A | 0,895 | 0,886 | 0,903 | 0,929 | 0,877 | 0,980 | 0,916 | 0,833 | 0,999 |
| IFI27 IFIT1 IL1R2 OLAH FAM20A RETN | 0,895 | 0,886 | 0,903 | 0,924 | 0,868 | 0,981 | 0,932 | 0,860 | 1 |
| RSAD2 IFI27 IFI44L IL1R2 OLAH FAM20A | 0,895 | 0,886 | 0,904 | 0,924 | 0,866 | 0,981 | 0,929 | 0,856 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 SLC1A2 OLAH FAM20A MMP8 | 0,895 | 0,885 | 0,904 | 0,924 | 0,868 | 0,980 | 0,928 | 0,854 | 1 |
| RSAD2 IFI27 IL1R2 OLAH FAM20A RETN | 0,895 | 0,886 | 0,903 | 0,924 | 0,867 | 0,980 | 0,930 | 0,858 | 1 |
| IFI27 OAS1 SIGLEC1 HERC6 OLAH MMP8 | 0,895 | 0,886 | 0,903 | 0,923 | 0,871 | 0,976 | 0,939 | 0,883 | 0,996 |
| IFI27 IFI44L SIGLEC1 ISG15 IL1R2 OLAH | 0,894 | 0,887 | 0,902 | 0,923 | 0,873 | 0,974 | 0,943 | 0,891 | 0,996 |
| RSAD2 IFI27 OAS1 HERC6 OLAH RETN | 0,894 | 0,886 | 0,903 | 0,922 | 0,866 | 0,979 | 0,942 | 0,883 | 1 |
| IFI27 OAS1 IFIT1 HERC6 OLAH MMP8 | 0,894 | 0,886 | 0,903 | 0,923 | 0,871 | 0,975 | 0,935 | 0,876 | 0,994 |
| IFI27 IFIT1 IFI44L OLAH FAM20A RETN | 0,894 | 0,886 | 0,903 | 0,919 | 0,858 | 0,981 | 0,938 | 0,870 | 1 |
| RSAD2 IFI27 OAS1 IFI44L OLAH FAM20A | 0,894 | 0,885 | 0,903 | 0,921 | 0,861 | 0,981 | 0,933 | 0,863 | 1 |
| RSAD2 IFI27 HERC6 IL1R2 OLAH MMP8 | 0,894 | 0,886 | 0,902 | 0,927 | 0,878 | 0,975 | 0,938 | 0,882 | 0,994 |
| RSAD2 IFI27 IFIT1 IFI44L OLAH FAM20A | 0,894 | 0,885 | 0,903 | 0,923 | 0,863 | 0,983 | 0,933 | 0,862 | 1 |
| RSAD2 IFI27 OAS1 IL1R2 FAM20A MMP8 | 0,894 | 0,886 | 0,903 | 0,926 | 0,875 | 0,978 | 0,928 | 0,843 | 1 |
| RSAD2 IFI27 OAS1 ISG15 OLAH FAM20A | 0,894 | 0,885 | 0,903 | 0,920 | 0,860 | 0,980 | 0,934 | 0,866 | 1 |
| IFI27 OAS1 IFI44L SIGLEC1 HERC6 OLAH | 0,894 | 0,886 | 0,902 | 0,926 | 0,875 | 0,977 | 0,945 | 0,892 | 0,997 |
| RSAD2 IFI27 SIGLEC1 SLC1A2 IL1R2 FAM20A | 0,894 | 0,885 | 0,903 | 0,931 | 0,881 | 0,982 | 0,948 | 0,881 | 1 |
| IFI27 IFIT1 SIGLEC1 HERC6 OLAH MMP8 | 0,894 | 0,886 | 0,903 | 0,921 | 0,867 | 0,975 | 0,940 | 0,886 | 0,994 |
| IFI27 OAS1 IL1R2 OLAH FAM20A RETN | 0,894 | 0,886 | 0,903 | 0,924 | 0,868 | 0,981 | 0,930 | 0,858 | 1 |
| IFI27 IFI44L SLC1A2 IL1R2 OLAH FAM20A | 0,894 | 0,886 | 0,903 | 0,928 | 0,875 | 0,982 | 0,923 | 0,842 | 1 |
| RSAD2 IFI27 SIGLEC1 FAM20A RETN MMP8 | 0,894 | 0,884 | 0,904 | 0,926 | 0,868 | 0,984 | 0,947 | 0,875 | 1 |
| RSAD2 IFI27 IFI44L SIGLEC1 HERC6 OLAH | 0,894 | 0,886 | 0,902 | 0,924 | 0,872 | 0,977 | 0,947 | 0,897 | 0,997 |
| RSAD2 IFI27 OLAH FAM20A RETN MMP8 | 0,894 | 0,885 | 0,904 | 0,923 | 0,864 | 0,982 | 0,940 | 0,874 | 1 |
| IFI27 IFIT1 ISG15 OLAH FAM20A RETN | 0,894 | 0,885 | 0,903 | 0,920 | 0,861 | 0,980 | 0,933 | 0,862 | 1 |
| IFI27 IFI44L SIGLEC1 ISG15 FAM20A RETN | 0,894 | 0,885 | 0,903 | 0,920 | 0,860 | 0,979 | 0,946 | 0,874 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 HERC6 SLC1A2 OLAH FAM20A MMP8 | 0,894 | 0,885 | 0,903 | 0,927 | 0,873 | 0,981 | 0,924 | 0,844 | 1 |
| IFI27 SIGLEC1 SLC1A2 IL1R2 OLAH FAM20A | 0,894 | 0,885 | 0,903 | 0,926 | 0,871 | 0,981 | 0,946 | 0,880 | 1 |
| IFI27 OAS1 SIGLEC1 HERC6 RETN MMP8 | 0,894 | 0,885 | 0,903 | 0,919 | 0,859 | 0,979 | 0,950 | 0,890 | 1 |
| RSAD2 IFI27 IFI44L ISG15 OLAH FAM20A | 0,894 | 0,885 | 0,903 | 0,920 | 0,859 | 0,981 | 0,933 | 0,863 | 1 |
| IFI27 OAS1 IL1R2 FAM20A RETN MMP8 | 0,894 | 0,885 | 0,902 | 0,929 | 0,877 | 0,981 | 0,934 | 0,855 | 1 |
| IFI27 SIGLEC1 HERC6 SLC1A2 OLAH FAM20A | 0,894 | 0,884 | 0,903 | 0,926 | 0,869 | 0,983 | 0,945 | 0,877 | 1 |
| RSAD2 IFI27 SIGLEC1 HERC6 OLAH RETN | 0,894 | 0,885 | 0,902 | 0,922 | 0,868 | 0,977 | 0,942 | 0,886 | 0,999 |
| RSAD2 IFI27 OAS1 SIGLEC1 HERC6 OLAH | 0,894 | 0,885 | 0,902 | 0,923 | 0,871 | 0,975 | 0,938 | 0,881 | 0,995 |
| RSAD2 IFI27 OAS1 IFIT1 OLAH FAM20A | 0,894 | 0,885 | 0,903 | 0,920 | 0,860 | 0,981 | 0,937 | 0,869 | 1 |
| IFI27 SIGLEC1 ISG15 SLC1A2 IL1R2 FAM20A | 0,894 | 0,885 | 0,903 | 0,932 | 0,885 | 0,980 | 0,947 | 0,877 | 1 |
| IFI27 IFI44L ISG15 IL1R2 FAM20A RETN | 0,894 | 0,885 | 0,902 | 0,924 | 0,871 | 0,978 | 0,927 | 0,846 | 1 |
| IFI27 IFIT1 IFI44L SIGLEC1 HERC6 OLAH | 0,894 | 0,886 | 0,902 | 0,925 | 0,872 | 0,978 | 0,947 | 0,897 | 0,997 |
| IFI27 IFI44L HERC6 IL1R2 OLAH MMP8 | 0,894 | 0,886 | 0,902 | 0,927 | 0,878 | 0,976 | 0,937 | 0,880 | 0,993 |
| IFI27 IFIT1 SIGLEC1 HERC6 IL1R2 OLAH | 0,894 | 0,886 | 0,902 | 0,926 | 0,876 | 0,975 | 0,941 | 0,888 | 0,995 |
| RSAD2 IFI27 OAS1 HERC6 OLAH MMP8 | 0,894 | 0,885 | 0,902 | 0,923 | 0,871 | 0,975 | 0,934 | 0,874 | 0,993 |
| IFI27 OAS1 IFIT1 HERC6 FAM20A RETN | 0,894 | 0,884 | 0,903 | 0,923 | 0,865 | 0,981 | 0,934 | 0,853 | 1 |
| RSAD2 IFI27 OAS1 HERC6 IL1R2 OLAH | 0,894 | 0,885 | 0,902 | 0,928 | 0,880 | 0,976 | 0,938 | 0,880 | 0,996 |
| IFI27 IFI44L SIGLEC1 HERC6 IL1R2 RETN | 0,894 | 0,886 | 0,902 | 0,924 | 0,871 | 0,977 | 0,945 | 0,893 | 0,996 |
| IFI27 IFIT1 HERC6 SLC1A2 OLAH FAM20A | 0,894 | 0,884 | 0,903 | 0,928 | 0,875 | 0,980 | 0,924 | 0,844 | 1 |
| OAS1 IFIT1 SIGLEC1 IL1R2 FAM20A MMP8 | 0,894 | 0,885 | 0,902 | 0,920 | 0,863 | 0,977 | 0,963 | 0,909 | 1 |
| IFIT1 SIGLEC1 IL1R2 OLAH FAM20A MMP8 | 0,894 | 0,885 | 0,902 | 0,922 | 0,866 | 0,978 | 0,966 | 0,916 | 1 |
| IFI27 ISG15 HERC6 OLAH RETN MMP8 | 0,894 | 0,884 | 0,903 | 0,920 | 0,861 | 0,979 | 0,951 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 IFI44L SLC1A2 OLAH FAM20A | 0,894 | 0,885 | 0,902 | 0,926 | 0,871 | 0,982 | 0,921 | 0,839 | 1 |
| RSAD2 IFI27 IFIT1 ISG15 OLAH FAM20A | 0,894 | 0,885 | 0,903 | 0,920 | 0,860 | 0,980 | 0,935 | 0,867 | 1 |
| IFI27 OAS1 IFI44L HERC6 OLAH MMP8 | 0,894 | 0,885 | 0,902 | 0,925 | 0,874 | 0,976 | 0,934 | 0,874 | 0,994 |
| RSAD2 IFI27 IFIT1 HERC6 OLAH RETN | 0,894 | 0,885 | 0,902 | 0,923 | 0,868 | 0,978 | 0,937 | 0,876 | 0,998 |
| IFI27 IFI44L ISG15 HERC6 OLAH MMP8 | 0,894 | 0,885 | 0,902 | 0,923 | 0,871 | 0,976 | 0,932 | 0,871 | 0,992 |
| IFI27 IFIT1 IFI44L SIGLEC1 ISG15 FAM20A | 0,894 | 0,885 | 0,902 | 0,915 | 0,851 | 0,980 | 0,942 | 0,870 | 1 |
| RSAD2 IFI27 ISG15 SLC1A2 OLAH FAM20A | 0,894 | 0,885 | 0,903 | 0,927 | 0,872 | 0,981 | 0,916 | 0,834 | 0,999 |
| IFI27 OAS1 HERC6 IL1R2 OLAH RETN | 0,894 | 0,885 | 0,902 | 0,922 | 0,867 | 0,978 | 0,939 | 0,878 | 1,000 |
| RSAD2 IFI27 ISG15 HERC6 FAM20A RETN | 0,894 | 0,885 | 0,903 | 0,924 | 0,869 | 0,980 | 0,933 | 0,852 | 1 |
| IFI27 OAS1 SLC1A2 OLAH FAM20A MMP8 | 0,894 | 0,885 | 0,903 | 0,925 | 0,870 | 0,980 | 0,926 | 0,850 | 1 |
| IFI27 HERC6 SLC1A2 IL1R2 OLAH FAM20A | 0,894 | 0,885 | 0,902 | 0,930 | 0,879 | 0,980 | 0,928 | 0,849 | 1 |
| IFI27 OAS1 ISG15 HERC6 OLAH RETN | 0,894 | 0,885 | 0,902 | 0,922 | 0,867 | 0,978 | 0,940 | 0,880 | 1 |
| IFI27 IFIT1 ISG15 HERC6 FAM20A RETN | 0,894 | 0,884 | 0,903 | 0,923 | 0,866 | 0,980 | 0,934 | 0,854 | 1 |
| IFI27 IFIT1 IFI44L IL1R2 FAM20A RETN | 0,893 | 0,885 | 0,902 | 0,923 | 0,869 | 0,977 | 0,930 | 0,852 | 1 |
| IFI27 OAS1 ISG15 OLAH FAM20A RETN | 0,893 | 0,885 | 0,902 | 0,920 | 0,861 | 0,980 | 0,934 | 0,866 | 1 |
| IFI27 OAS1 IFIT1 IFI44L IL1R2 FAM20A | 0,893 | 0,885 | 0,902 | 0,922 | 0,868 | 0,976 | 0,928 | 0,848 | 1 |
| RSAD2 IFI27 IL1R2 FAM20A RETN MMP8 | 0,893 | 0,885 | 0,902 | 0,927 | 0,874 | 0,980 | 0,932 | 0,851 | 1 |
| IFI27 OAS1 IFIT1 HERC6 IL1R2 OLAH | 0,893 | 0,885 | 0,902 | 0,925 | 0,875 | 0,974 | 0,939 | 0,882 | 0,996 |
| IFI27 OAS1 IFIT1 OLAH FAM20A RETN | 0,893 | 0,884 | 0,902 | 0,918 | 0,857 | 0,979 | 0,935 | 0,864 | 1 |
| RSAD2 IFI27 OAS1 HERC6 FAM20A RETN | 0,893 | 0,884 | 0,903 | 0,923 | 0,865 | 0,981 | 0,935 | 0,855 | 1 |
| IFI27 ISG15 IL1R2 FAM20A RETN MMP8 | 0,893 | 0,885 | 0,902 | 0,926 | 0,875 | 0,976 | 0,920 | 0,833 | 1 |
| IFI27 OAS1 SIGLEC1 ISG15 FAM20A RETN | 0,893 | 0,884 | 0,903 | 0,917 | 0,855 | 0,979 | 0,946 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFI44L HERC6 OLAH RETN | 0,893 | 0,885 | 0,902 | 0,923 | 0,868 | 0,977 | 0,935 | 0,872 | 0,998 |
| IFI27 SIGLEC1 HERC6 OLAH RETN MMP8 | 0,893 | 0,884 | 0,903 | 0,918 | 0,858 | 0,979 | 0,961 | 0,915 | 1 |
| IFI27 IFI44L ISG15 HERC6 IL1R2 OLAH | 0,893 | 0,885 | 0,901 | 0,927 | 0,877 | 0,976 | 0,934 | 0,874 | 0,993 |
| IFI27 IFI44L ISG15 HERC6 OLAH RETN | 0,893 | 0,884 | 0,902 | 0,921 | 0,864 | 0,977 | 0,941 | 0,885 | 0,997 |
| RSAD2 SIGLEC1 HERC6 IL1R2 FAM20A MMP8 | 0,893 | 0,885 | 0,902 | 0,920 | 0,864 | 0,976 | 0,961 | 0,902 | 1 |
| IFI27 SIGLEC1 ISG15 SLC1A2 OLAH MMP8 | 0,893 | 0,885 | 0,902 | 0,930 | 0,879 | 0,980 | 0,939 | 0,879 | 0,999 |
| IFI27 IFIT1 SIGLEC1 ISG15 HERC6 OLAH | 0,893 | 0,885 | 0,901 | 0,923 | 0,870 | 0,976 | 0,942 | 0,889 | 0,996 |
| IFI27 IFIT1 IFI44L HERC6 OLAH RETN | 0,893 | 0,884 | 0,902 | 0,920 | 0,864 | 0,977 | 0,943 | 0,888 | 0,999 |
| IFI27 IFIT1 ISG15 SLC1A2 OLAH FAM20A | 0,893 | 0,884 | 0,902 | 0,924 | 0,868 | 0,980 | 0,923 | 0,846 | 1,000 |
| IFI44L SIGLEC1 HERC6 IL1R2 FAM20A MMP8 | 0,893 | 0,884 | 0,902 | 0,919 | 0,862 | 0,976 | 0,959 | 0,897 | 1 |
| IFI27 OAS1 SIGLEC1 HERC6 IL1R2 MMP8 | 0,893 | 0,885 | 0,901 | 0,922 | 0,875 | 0,968 | 0,948 | 0,900 | 0,996 |
| RSAD2 IFI27 IFIT1 IFI44L IL1R2 FAM20A | 0,893 | 0,885 | 0,901 | 0,922 | 0,867 | 0,976 | 0,928 | 0,848 | 1 |
| IFI27 OAS1 ISG15 IL1R2 FAM20A MMP8 | 0,893 | 0,885 | 0,901 | 0,927 | 0,875 | 0,978 | 0,930 | 0,847 | 1 |
| IFI27 SIGLEC1 ISG15 IL1R2 OLAH MMP8 | 0,893 | 0,885 | 0,901 | 0,923 | 0,872 | 0,974 | 0,943 | 0,892 | 0,995 |
| IFI27 IFIT1 IFI44L ISG15 IL1R2 FAM20A | 0,893 | 0,884 | 0,901 | 0,923 | 0,869 | 0,977 | 0,928 | 0,848 | 1 |
| IFI27 OAS1 IFIT1 ISG15 HERC6 RETN | 0,893 | 0,884 | 0,902 | 0,917 | 0,858 | 0,977 | 0,932 | 0,863 | 1,000 |
| IFI27 OAS1 SIGLEC1 HERC6 SLC1A2 OLAH | 0,893 | 0,884 | 0,902 | 0,928 | 0,878 | 0,978 | 0,941 | 0,882 | 1 |
| IFI27 IFIT1 ISG15 HERC6 OLAH RETN | 0,893 | 0,884 | 0,901 | 0,920 | 0,864 | 0,977 | 0,940 | 0,881 | 0,999 |
| IFI27 IFI44L SIGLEC1 ISG15 SLC1A2 OLAH | 0,893 | 0,885 | 0,901 | 0,929 | 0,879 | 0,979 | 0,945 | 0,887 | 1 |
| RSAD2 IFI27 HERC6 IL1R2 OLAH RETN | 0,893 | 0,884 | 0,901 | 0,922 | 0,867 | 0,977 | 0,939 | 0,879 | 0,999 |
| IFIT1 IFI44L SIGLEC1 IL1R2 FAM20A MMP8 | 0,893 | 0,884 | 0,901 | 0,920 | 0,864 | 0,977 | 0,966 | 0,915 | 1 |
| IFI27 OAS1 ISG15 SLC1A2 OLAH FAM20A | 0,893 | 0,884 | 0,902 | 0,927 | 0,873 | 0,981 | 0,917 | 0,837 | 0,998 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 ISG15 OLAH FAM20A RETN | 0,893 | 0,884 | 0,902 | 0,919 | 0,859 | 0,980 | 0,933 | 0,863 | 1 |
| IFI27 ISG15 IL1R2 OLAH RETN MMP8 | 0,893 | 0,884 | 0,901 | 0,923 | 0,868 | 0,978 | 0,948 | 0,890 | 1 |
| RSAD2 IFI27 IFIT1 HERC6 IL1R2 OLAH | 0,893 | 0,884 | 0,901 | 0,925 | 0,875 | 0,975 | 0,939 | 0,884 | 0,995 |
| IFI27 IFI44L SLC1A2 IL1R2 FAM20A MMP8 | 0,893 | 0,884 | 0,901 | 0,929 | 0,882 | 0,976 | 0,926 | 0,839 | 1 |
| RSAD2 IFI27 IFI44L HERC6 IL1R2 OLAH | 0,893 | 0,884 | 0,901 | 0,926 | 0,877 | 0,975 | 0,936 | 0,878 | 0,993 |
| IFI27 SLC1A2 IL1R2 OLAH FAM20A RETN | 0,893 | 0,884 | 0,902 | 0,927 | 0,874 | 0,980 | 0,920 | 0,837 | 1 |
| RSAD2 IFI27 SIGLEC1 HERC6 IL1R2 RETN | 0,893 | 0,884 | 0,901 | 0,922 | 0,870 | 0,975 | 0,949 | 0,900 | 0,998 |
| RSAD2 IFI27 OAS1 IFIT1 IL1R2 FAM20A | 0,893 | 0,884 | 0,901 | 0,923 | 0,869 | 0,977 | 0,929 | 0,850 | 1 |
| IFI27 IFI44L SIGLEC1 OLAH RETN MMP8 | 0,893 | 0,884 | 0,902 | 0,920 | 0,862 | 0,978 | 0,959 | 0,908 | 1 |
| IFI27 SIGLEC1 ISG15 HERC6 IL1R2 OLAH | 0,893 | 0,884 | 0,901 | 0,924 | 0,874 | 0,975 | 0,943 | 0,891 | 0,996 |
| IFIT1 IFI44L SIGLEC1 HERC6 IL1R2 FAM20A | 0,893 | 0,884 | 0,901 | 0,918 | 0,861 | 0,976 | 0,952 | 0,895 | 1 |
| RSAD2 IFI27 IFI44L HERC6 OLAH MMP8 | 0,893 | 0,884 | 0,901 | 0,926 | 0,874 | 0,977 | 0,934 | 0,875 | 0,993 |
| RSAD2 IFI27 OAS1 SIGLEC1 HERC6 FAM20A | 0,893 | 0,883 | 0,902 | 0,918 | 0,858 | 0,977 | 0,940 | 0,868 | 1 |
| IFI27 OAS1 ISG15 HERC6 OLAH MMP8 | 0,893 | 0,884 | 0,901 | 0,923 | 0,871 | 0,975 | 0,937 | 0,879 | 0,995 |
| IFI27 ISG15 SLC1A2 OLAH FAM20A RETN | 0,893 | 0,884 | 0,901 | 0,926 | 0,872 | 0,980 | 0,916 | 0,834 | 0,998 |
| IFI27 OAS1 IFI44L OLAH FAM20A RETN | 0,893 | 0,883 | 0,902 | 0,920 | 0,860 | 0,981 | 0,938 | 0,870 | 1 |
| RSAD2 IFI27 IFIT1 SIGLEC1 HERC6 OLAH | 0,893 | 0,884 | 0,901 | 0,924 | 0,873 | 0,976 | 0,939 | 0,885 | 0,994 |
| RSAD2 IFI27 ISG15 IL1R2 FAM20A MMP8 | 0,893 | 0,884 | 0,901 | 0,926 | 0,875 | 0,978 | 0,928 | 0,843 | 1 |
| IFI27 OAS1 SIGLEC1 ISG15 HERC6 OLAH | 0,892 | 0,884 | 0,901 | 0,923 | 0,870 | 0,976 | 0,939 | 0,883 | 0,995 |
| RSAD2 IFI27 IFI44L HERC6 OLAH RETN | 0,892 | 0,884 | 0,901 | 0,923 | 0,869 | 0,977 | 0,935 | 0,874 | 0,996 |
| IFI27 OAS1 IFIT1 HERC6 IL1R2 RETN | 0,892 | 0,884 | 0,901 | 0,922 | 0,870 | 0,975 | 0,945 | 0,888 | 1 |
| RSAD2 IFI27 IFIT1 SIGLEC1 FAM20A RETN | 0,892 | 0,883 | 0,901 | 0,920 | 0,860 | 0,979 | 0,947 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 SIGLEC1 HERC6 IL1R2 FAM20A MMP8 | 0,892 | 0,884 | 0,901 | 0,918 | 0,861 | 0,975 | 0,964 | 0,910 | 1 |
| IFI27 IFIT1 SIGLEC1 ISG15 SLC1A2 OLAH | 0,892 | 0,884 | 0,901 | 0,929 | 0,879 | 0,979 | 0,938 | 0,878 | 0,998 |
| RSAD2 IFI27 SLC1A2 IL1R2 OLAH FAM20A | 0,892 | 0,884 | 0,901 | 0,927 | 0,873 | 0,981 | 0,921 | 0,838 | 1 |
| IFI27 OAS1 IFIT1 SIGLEC1 HERC6 IL1R2 | 0,892 | 0,885 | 0,900 | 0,922 | 0,876 | 0,969 | 0,949 | 0,902 | 0,996 |
| IFI27 SIGLEC1 ISG15 HERC6 OLAH MMP8 | 0,892 | 0,884 | 0,901 | 0,918 | 0,862 | 0,975 | 0,941 | 0,886 | 0,996 |
| IFI27 IFI44L SIGLEC1 FAM20A RETN MMP8 | 0,892 | 0,883 | 0,902 | 0,920 | 0,861 | 0,979 | 0,950 | 0,878 | 1 |
| RSAD2 IFI27 OAS1 IFI44L IL1R2 FAM20A | 0,892 | 0,884 | 0,901 | 0,923 | 0,869 | 0,977 | 0,932 | 0,852 | 1 |
| RSAD2 IFI27 OAS1 IFIT1 HERC6 OLAH | 0,892 | 0,884 | 0,901 | 0,922 | 0,870 | 0,975 | 0,934 | 0,874 | 0,993 |
| IFI27 OAS1 IFIT1 IL1R2 FAM20A RETN | 0,892 | 0,884 | 0,901 | 0,925 | 0,872 | 0,978 | 0,929 | 0,850 | 1 |
| IFI27 SLC1A2 OLAH FAM20A RETN MMP8 | 0,892 | 0,883 | 0,902 | 0,927 | 0,873 | 0,981 | 0,924 | 0,845 | 1 |
| IFI27 IFI44L HERC6 IL1R2 OLAH RETN | 0,892 | 0,884 | 0,901 | 0,923 | 0,868 | 0,977 | 0,943 | 0,888 | 0,999 |
| IFI27 HERC6 IL1R2 OLAH RETN MMP8 | 0,892 | 0,883 | 0,901 | 0,921 | 0,863 | 0,979 | 0,954 | 0,904 | 1 |
| IFI27 OAS1 IFI44L ISG15 HERC6 RETN | 0,892 | 0,883 | 0,901 | 0,917 | 0,857 | 0,977 | 0,935 | 0,868 | 1 |
| IFI27 OAS1 ISG15 HERC6 IL1R2 OLAH | 0,892 | 0,884 | 0,901 | 0,926 | 0,877 | 0,975 | 0,939 | 0,883 | 0,996 |
| IFI27 OAS1 HERC6 IL1R2 RETN MMP8 | 0,892 | 0,884 | 0,901 | 0,922 | 0,869 | 0,975 | 0,936 | 0,873 | 0,999 |
| IFI27 OAS1 IFIT1 IFI44L HERC6 OLAH | 0,892 | 0,884 | 0,901 | 0,926 | 0,874 | 0,977 | 0,937 | 0,880 | 0,994 |
| IFI27 SIGLEC1 HERC6 IL1R2 OLAH MMP8 | 0,892 | 0,884 | 0,900 | 0,923 | 0,872 | 0,974 | 0,949 | 0,900 | 0,998 |
| IFI27 IFI44L SIGLEC1 ISG15 OLAH MMP8 | 0,892 | 0,884 | 0,900 | 0,920 | 0,865 | 0,975 | 0,942 | 0,887 | 0,998 |
| IFI27 OAS1 IFI44L SLC1A2 OLAH FAM20A | 0,892 | 0,883 | 0,901 | 0,929 | 0,873 | 0,984 | 0,923 | 0,843 | 1 |
| IFI27 OAS1 IFI44L ISG15 IL1R2 FAM20A | 0,892 | 0,884 | 0,901 | 0,923 | 0,869 | 0,977 | 0,930 | 0,851 | 1 |
| IFI27 IFIT1 HERC6 IL1R2 OLAH RETN | 0,892 | 0,883 | 0,901 | 0,920 | 0,864 | 0,977 | 0,943 | 0,887 | 1,000 |
| RSAD2 IFI27 IFIT1 HERC6 OLAH MMP8 | 0,892 | 0,884 | 0,901 | 0,923 | 0,870 | 0,975 | 0,936 | 0,878 | 0,994 |

(suite)

| Modèle | AUC modèle | IC 95% | | AUC Train | IC 95% Train | | AUC Test | IC 95% Test | |
|---|---|---|---|---|---|---|---|---|---|
| IFI27 ISG15 HERC6 IL1R2 OLAH MMP8 | 0,892 | 0,884 | 0,900 | 0,924 | 0,874 | 0,974 | 0,942 | 0,888 | 0,997 |
| IFI27 IFIT1 SIGLEC1 IL1R2 OLAH MMP8 | 0,892 | 0,884 | 0,900 | 0,923 | 0,872 | 0,974 | 0,951 | 0,904 | 0,998 |
| RSAD2 IFI27 OAS1 OLAH FAM20A RETN | 0,892 | 0,883 | 0,901 | 0,919 | 0,858 | 0,980 | 0,937 | 0,869 | 1 |
| IFI27 SIGLEC1 HERC6 SLC1A2 IL1R2 FAM20A | 0,892 | 0,883 | 0,901 | 0,928 | 0,877 | 0,979 | 0,948 | 0,877 | 1 |
| RSAD2 IFI27 OAS1 SIGLEC1 HERC6 IL1R2 | 0,892 | 0,884 | 0,900 | 0,922 | 0,876 | 0,969 | 0,951 | 0,905 | 0,997 |
| IFI27 IFIT1 SIGLEC1 OLAH RETN MMP8 | 0,892 | 0,883 | 0,901 | 0,919 | 0,860 | 0,978 | 0,962 | 0,911 | 1 |
| IFI27 IFIT1 HERC6 IL1R2 OLAH MMP8 | 0,892 | 0,884 | 0,900 | 0,924 | 0,874 | 0,975 | 0,942 | 0,888 | 0,997 |
| RSAD2 IFI27 IFIT1 HERC6 FAM20A RETN | 0,892 | 0,883 | 0,901 | 0,922 | 0,864 | 0,979 | 0,933 | 0,853 | 1 |
| RSAD2 IFI27 IFI44L SLC1A2 OLAH FAM20A | 0,892 | 0,883 | 0,901 | 0,925 | 0,869 | 0,982 | 0,923 | 0,844 | 1 |
| IFI27 IFIT1 SIGLEC1 HERC6 IL1R2 RETN | 0,892 | 0,884 | 0,900 | 0,924 | 0,871 | 0,976 | 0,951 | 0,905 | 0,998 |
| IFI27 IFIT1 SLC1A2 IL1R2 OLAH FAM20A | 0,892 | 0,883 | 0,901 | 0,925 | 0,871 | 0,980 | 0,923 | 0,842 | 1 |
| RSAD2 IFI27 OAS1 SIGLEC1 FAM20A RETN | 0,892 | 0,882 | 0,901 | 0,918 | 0,857 | 0,979 | 0,949 | 0,883 | 1 |
| IFI27 OAS1 IFI44L SIGLEC1 HERC6 IL1R2 | 0,892 | 0,884 | 0,899 | 0,922 | 0,876 | 0,968 | 0,939 | 0,886 | 0,992 |
| RSAD2 IFI27 SIGLEC1 ISG15 HERC6 FAM20A | 0,892 | 0,883 | 0,901 | 0,918 | 0,858 | 0,978 | 0,941 | 0,869 | 1 |
| RSAD2 IFI27 OAS1 HERC6 IL1R2 RETN | 0,892 | 0,884 | 0,900 | 0,920 | 0,865 | 0,975 | 0,940 | 0,877 | 1 |
| OAS1 IFIT1 SIGLEC1 HERC6 IL1R2 FAM20A | 0,892 | 0,883 | 0,900 | 0,919 | 0,862 | 0,976 | 0,953 | 0,897 | 1 |
| IFIT1 SIGLEC1 ISG15 HERC6 IL1R2 FAM20A | 0,892 | 0,883 | 0,900 | 0,918 | 0,860 | 0,975 | 0,953 | 0,897 | 1 |
| RSAD2 IFI27 SIGLEC1 ISG15 HERC6 OLAH | 0,892 | 0,883 | 0,900 | 0,923 | 0,871 | 0,975 | 0,939 | 0,884 | 0,994 |
| IFI27 OAS1 SIGLEC1 IL1R2 OLAH MMP8 | 0,892 | 0,884 | 0,900 | 0,922 | 0,871 | 0,973 | 0,947 | 0,896 | 0,998 |
| RSAD2 IFI27 SIGLEC1 IL1R2 OLAH MMP8 | 0,892 | 0,884 | 0,900 | 0,922 | 0,872 | 0,973 | 0,949 | 0,900 | 0,998 |
| IFI27 SIGLEC1 ISG15 SLC1A2 IL1R2 OLAH | 0,892 | 0,884 | 0,900 | 0,930 | 0,881 | 0,980 | 0,939 | 0,880 | 0,998 |
| RSAD2 IFI27 ISG15 HERC6 IL1R2 OLAH | 0,892 | 0,884 | 0,900 | 0,924 | 0,873 | 0,974 | 0,937 | 0,880 | 0,994 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 IFI44L ISG15 IL1R2 FAM20A | 0,892 | 0,883 | 0,900 | 0,924 | 0,870 | 0,978 | 0,928 | 0,848 | 1 |
| IFI27 IFIT1 IFI44L HERC6 OLAH MMP8 | 0,892 | 0,883 | 0,900 | 0,924 | 0,871 | 0,977 | 0,936 | 0,877 | 0,994 |
| RSAD2 IFI27 IFIT1 OLAH FAM20A RETN | 0,892 | 0,883 | 0,901 | 0,918 | 0,857 | 0,979 | 0,935 | 0,864 | 1 |
| RSAD2 IFI27 IFI44L HERC6 FAM20A RETN | 0,892 | 0,882 | 0,901 | 0,924 | 0,867 | 0,982 | 0,932 | 0,851 | 1 |
| RSAD2 IFI27 OAS1 SLC1A2 OLAH FAM20A | 0,892 | 0,883 | 0,901 | 0,924 | 0,867 | 0,980 | 0,923 | 0,843 | 1 |
| IFI27 IFI44L HERC6 SLC1A2 OLAH FAM20A | 0,892 | 0,882 | 0,902 | 0,926 | 0,870 | 0,981 | 0,925 | 0,845 | 1 |
| IFI44L SIGLEC1 IL1R2 OLAH FAM20A MMP8 | 0,892 | 0,883 | 0,900 | 0,918 | 0,861 | 0,975 | 0,962 | 0,906 | 1 |
| IFI27 IFIT1 ISG15 HERC6 OLAH MMP8 | 0,892 | 0,883 | 0,900 | 0,921 | 0,866 | 0,975 | 0,936 | 0,877 | 0,995 |
| IFI27 OAS1 IFI44L HERC6 IL1R2 OLAH | 0,892 | 0,883 | 0,900 | 0,928 | 0,880 | 0,976 | 0,936 | 0,878 | 0,994 |
| IFI27 IFIT1 IFI44L ISG15 HERC6 OLAH | 0,892 | 0,883 | 0,900 | 0,924 | 0,871 | 0,977 | 0,935 | 0,876 | 0,993 |
| RSAD2 IFI27 IFIT1 SLC1A2 OLAH FAM20A | 0,892 | 0,883 | 0,901 | 0,923 | 0,865 | 0,980 | 0,927 | 0,851 | 1 |
| IFIT1 SIGLEC1 HERC6 IL1R2 OLAH FAM20A | 0,892 | 0,883 | 0,900 | 0,919 | 0,861 | 0,977 | 0,952 | 0,895 | 1 |
| IFI27 OAS1 ISG15 HERC6 IL1R2 RETN | 0,892 | 0,883 | 0,900 | 0,922 | 0,870 | 0,975 | 0,938 | 0,877 | 0,999 |
| IFI27 OAS1 IFI44L IL1R2 FAM20A RETN | 0,892 | 0,883 | 0,900 | 0,925 | 0,872 | 0,978 | 0,929 | 0,850 | 1 |
| IFI27 OAS1 SIGLEC1 SLC1A2 OLAH MMP8 | 0,892 | 0,883 | 0,900 | 0,927 | 0,875 | 0,978 | 0,945 | 0,888 | 1 |
| RSAD2 IFI27 ISG15 HERC6 OLAH RETN | 0,892 | 0,883 | 0,900 | 0,923 | 0,868 | 0,978 | 0,938 | 0,877 | 0,999 |
| IFI27 SIGLEC1 ISG15 HERC6 FAM20A RETN | 0,892 | 0,882 | 0,901 | 0,915 | 0,852 | 0,978 | 0,947 | 0,872 | 1 |
| RSAD2 SIGLEC1 IL1R2 OLAH FAM20A MMP8 | 0,892 | 0,883 | 0,900 | 0,919 | 0,863 | 0,976 | 0,964 | 0,911 | 1 |
| IFI27 SIGLEC1 ISG15 OLAH RETN MMP8 | 0,892 | 0,882 | 0,901 | 0,917 | 0,857 | 0,978 | 0,960 | 0,910 | 1 |
| IFI27 SIGLEC1 SLC1A2 OLAH FAM20A RETN | 0,892 | 0,882 | 0,901 | 0,924 | 0,865 | 0,982 | 0,943 | 0,876 | 1 |
| RSAD2 IFI27 OAS1 ISG15 HERC6 OLAH | 0,892 | 0,883 | 0,900 | 0,923 | 0,871 | 0,975 | 0,936 | 0,877 | 0,995 |
| RSAD2 IFIT1 SIGLEC1 IL1R2 FAM20A MMP8 | 0,892 | 0,883 | 0,900 | 0,922 | 0,865 | 0,978 | 0,967 | 0,916 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 ISG15 HERC6 IL1R2 OLAH | 0,892 | 0,883 | 0,900 | 0,924 | 0,874 | 0,974 | 0,942 | 0,888 | 0,997 |
| IFI27 OAS1 IFIT1 ISG15 HERC6 OLAH | 0,892 | 0,883 | 0,900 | 0,923 | 0,871 | 0,975 | 0,938 | 0,880 | 0,996 |
| RSAD2 IFI27 OAS1 IFI44L HERC6 OLAH | 0,891 | 0,883 | 0,900 | 0,926 | 0,875 | 0,977 | 0,937 | 0,879 | 0,995 |
| SIGLEC1 ISG15 IL1R2 OLAH FAM20A MMP8 | 0,891 | 0,883 | 0,900 | 0,916 | 0,857 | 0,974 | 0,964 | 0,909 | 1 |
| IFI27 IFIT1 SIGLEC1 ISG15 IL1R2 OLAH | 0,891 | 0,884 | 0,899 | 0,921 | 0,868 | 0,973 | 0,945 | 0,891 | 0,998 |
| IFI27 OAS1 HERC6 SLC1A2 OLAH MMP8 | 0,891 | 0,883 | 0,900 | 0,928 | 0,878 | 0,977 | 0,930 | 0,864 | 0,997 |
| IFI27 OAS1 IFIT1 SLC1A2 OLAH FAM20A | 0,891 | 0,882 | 0,901 | 0,924 | 0,868 | 0,981 | 0,926 | 0,850 | 1 |
| IFI27 IFI44L SIGLEC1 SLC1A2 OLAH MMP8 | 0,891 | 0,883 | 0,900 | 0,927 | 0,876 | 0,978 | 0,949 | 0,895 | 1 |
| IFI27 SIGLEC1 ISG15 HERC6 OLAH RETN | 0,891 | 0,882 | 0,900 | 0,917 | 0,858 | 0,976 | 0,945 | 0,888 | 1 |
| IFI27 IFI44L SIGLEC1 HERC6 IL1R2 MMP8 | 0,891 | 0,884 | 0,899 | 0,922 | 0,875 | 0,970 | 0,937 | 0,883 | 0,991 |
| IFI27 IFI44L IL1R2 OLAH RETN MMP8 | 0,891 | 0,882 | 0,900 | 0,923 | 0,868 | 0,978 | 0,951 | 0,897 | 1 |
| RSAD2 IFI27 OAS1 ISG15 HERC6 RETN | 0,891 | 0,882 | 0,900 | 0,919 | 0,860 | 0,979 | 0,937 | 0,869 | 1 |
| IFI44L SIGLEC1 ISG15 IL1R2 FAM20A MMP8 | 0,891 | 0,883 | 0,900 | 0,916 | 0,858 | 0,975 | 0,961 | 0,903 | 1 |
| IFI27 OAS1 IFI44L HERC6 IL1R2 RETN | 0,891 | 0,883 | 0,899 | 0,922 | 0,869 | 0,974 | 0,938 | 0,878 | 0,998 |
| IFI27 OAS1 HERC6 SLC1A2 OLAH RETN | 0,891 | 0,883 | 0,900 | 0,927 | 0,877 | 0,978 | 0,932 | 0,863 | 1 |
| IFI27 SIGLEC1 ISG15 HERC6 SLC1A2 OLAH | 0,891 | 0,882 | 0,900 | 0,930 | 0,879 | 0,980 | 0,939 | 0,880 | 0,998 |
| IFI27 OAS1 IFI44L SIGLEC1 FAM20A RETN | 0,891 | 0,882 | 0,900 | 0,919 | 0,859 | 0,979 | 0,943 | 0,873 | 1 |
| SIGLEC1 HERC6 IL1R2 OLAH FAM20A MMP8 | 0,891 | 0,882 | 0,900 | 0,918 | 0,859 | 0,976 | 0,964 | 0,909 | 1 |
| IFI27 IFI44L ISG15 HERC6 FAM20A RETN | 0,891 | 0,882 | 0,901 | 0,920 | 0,861 | 0,979 | 0,940 | 0,862 | 1 |
| IFI27 HERC6 SLC1A2 OLAH FAM20A RETN | 0,891 | 0,882 | 0,900 | 0,927 | 0,872 | 0,983 | 0,926 | 0,847 | 1 |
| IFI27 SIGLEC1 IL1R2 OLAH RETN MMP8 | 0,891 | 0,882 | 0,900 | 0,920 | 0,863 | 0,978 | 0,961 | 0,913 | 1 |
| IFI27 IFI44L SIGLEC1 IL1R2 OLAH RETN | 0,891 | 0,883 | 0,899 | 0,919 | 0,864 | 0,975 | 0,952 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 SIGLEC1 IL1R2 OLAH | 0,891 | 0,883 | 0,899 | 0,921 | 0,869 | 0,973 | 0,942 | 0,888 | 0,996 |
| RSAD2 IFI27 IFI44L OLAH FAM20A RETN | 0,891 | 0,882 | 0,901 | 0,921 | 0,860 | 0,981 | 0,937 | 0,869 | 1 |
| IFI27 IFIT1 IFI44L SIGLEC1 IL1R2 OLAH | 0,891 | 0,883 | 0,899 | 0,922 | 0,870 | 0,973 | 0,948 | 0,897 | 0,998 |
| IFI27 OAS1 SLC1A2 IL1R2 OLAH FAM20A | 0,891 | 0,883 | 0,900 | 0,928 | 0,875 | 0,981 | 0,920 | 0,836 | 1 |
| IFI27 OAS1 SIGLEC1 OLAH RETN MMP8 | 0,891 | 0,882 | 0,900 | 0,920 | 0,862 | 0,979 | 0,962 | 0,909 | 1 |
| IFI27 OAS1 IFI44L ISG15 HERC6 OLAH | 0,891 | 0,882 | 0,900 | 0,924 | 0,873 | 0,976 | 0,935 | 0,876 | 0,994 |
| IFI27 OAS1 IFI44L SIGLEC1 IL1R2 OLAH | 0,891 | 0,883 | 0,899 | 0,923 | 0,872 | 0,974 | 0,947 | 0,896 | 0,997 |
| RSAD2 IFI27 ISG15 HERC6 OLAH MMP8 | 0,891 | 0,883 | 0,900 | 0,923 | 0,871 | 0,976 | 0,936 | 0,877 | 0,995 |
| IFI27 OAS1 IFIT1 ISG15 IL1R2 FAM20A | 0,891 | 0,882 | 0,900 | 0,924 | 0,871 | 0,978 | 0,929 | 0,850 | 1 |
| IFI27 IFI44L ISG15 IL1R2 OLAH MMP8 | 0,891 | 0,883 | 0,899 | 0,922 | 0,871 | 0,974 | 0,939 | 0,882 | 0,996 |
| RSAD2 OAS1 SIGLEC1 IL1R2 FAM20A MMP8 | 0,891 | 0,882 | 0,900 | 0,919 | 0,862 | 0,976 | 0,962 | 0,905 | 1 |
| IFI27 ISG15 HERC6 IL1R2 OLAH RETN | 0,891 | 0,882 | 0,900 | 0,921 | 0,865 | 0,977 | 0,943 | 0,887 | 1 |
| IFIT1 SIGLEC1 ISG15 IL1R2 FAM20A MMP8 | 0,891 | 0,883 | 0,900 | 0,919 | 0,861 | 0,976 | 0,966 | 0,915 | 1 |
| IFI27 IFIT1 IFI44L SIGLEC1 HERC6 RETN | 0,891 | 0,882 | 0,900 | 0,911 | 0,850 | 0,973 | 0,941 | 0,887 | 0,995 |
| IFI27 IFIT1 IFI44L SIGLEC1 FAM20A MMP8 | 0,891 | 0,882 | 0,900 | 0,913 | 0,848 | 0,978 | 0,946 | 0,877 | 1 |
| RSAD2 IFI27 SIGLEC1 HERC6 SLC1A2 OLAH | 0,891 | 0,882 | 0,900 | 0,928 | 0,877 | 0,978 | 0,937 | 0,878 | 0,996 |
| IFI27 OAS1 IFIT1 IFI44L SIGLEC1 FAM20A | 0,891 | 0,882 | 0,900 | 0,913 | 0,848 | 0,979 | 0,947 | 0,878 | 1 |
| RSAD2 IFI27 SIGLEC1 ISG15 FAM20A RETN | 0,891 | 0,881 | 0,900 | 0,918 | 0,858 | 0,979 | 0,948 | 0,881 | 1 |
| IFI27 OAS1 ISG15 IL1R2 FAM20A RETN | 0,891 | 0,882 | 0,899 | 0,926 | 0,873 | 0,978 | 0,930 | 0,851 | 1 |
| IFI27 OAS1 IFIT1 SIGLEC1 OLAH RETN | 0,891 | 0,882 | 0,899 | 0,916 | 0,858 | 0,974 | 0,949 | 0,892 | 1 |
| IFIT1 SIGLEC1 HERC6 OLAH FAM20A MMP8 | 0,891 | 0,881 | 0,900 | 0,918 | 0,860 | 0,976 | 0,954 | 0,900 | 1 |
| IFI27 IFI44L SIGLEC1 IL1R2 RETN MMP8 | 0,891 | 0,883 | 0,899 | 0,923 | 0,871 | 0,976 | 0,961 | 0,920 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 IFIT1 ISG15 IL1R2 FAM20A | 0,891 | 0,882 | 0,899 | 0,923 | 0,870 | 0,977 | 0,929 | 0,850 | 1 |
| RSAD2 IFIT1 SIGLEC1 HERC6 IL1R2 FAM20A | 0,891 | 0,882 | 0,899 | 0,918 | 0,860 | 0,976 | 0,954 | 0,898 | 1 |
| IFI27 IFIT1 IL1R2 OLAH RETN MMP8 | 0,891 | 0,882 | 0,900 | 0,921 | 0,864 | 0,977 | 0,952 | 0,900 | 1 |
| RSAD2 IFI27 OAS1 ISG15 IL1R2 FAM20A | 0,891 | 0,882 | 0,899 | 0,925 | 0,872 | 0,978 | 0,932 | 0,852 | 1 |
| IFI27 IFI44L SIGLEC1 SLC1A2 IL1R2 OLAH | 0,891 | 0,882 | 0,899 | 0,927 | 0,877 | 0,978 | 0,943 | 0,888 | 0,999 |
| RSAD2 IFI27 IFIT1 IL1R2 FAM20A RETN | 0,891 | 0,882 | 0,899 | 0,924 | 0,871 | 0,978 | 0,929 | 0,850 | 1 |
| RSAD2 IFI27 SIGLEC1 HERC6 FAM20A MMP8 | 0,891 | 0,881 | 0,900 | 0,918 | 0,858 | 0,978 | 0,940 | 0,868 | 1 |
| SIGLEC1 ISG15 HERC6 IL1R2 FAM20A MMP8 | 0,891 | 0,882 | 0,900 | 0,915 | 0,855 | 0,974 | 0,962 | 0,903 | 1 |
| IFI27 OAS1 SIGLEC1 ISG15 HERC6 FAM20A | 0,891 | 0,881 | 0,900 | 0,914 | 0,853 | 0,976 | 0,942 | 0,870 | 1 |
| IFI27 OAS1 SIGLEC1 IL1R2 RETN MMP8 | 0,891 | 0,882 | 0,899 | 0,923 | 0,871 | 0,976 | 0,963 | 0,918 | 1 |
| RSAD2 IFI27 IFI44L SIGLEC1 HERC6 FAM20A | 0,891 | 0,882 | 0,899 | 0,918 | 0,858 | 0,977 | 0,943 | 0,871 | 1 |
| IFI27 IFIT1 SIGLEC1 SLC1A2 OLAH MMP8 | 0,891 | 0,882 | 0,900 | 0,928 | 0,877 | 0,979 | 0,942 | 0,884 | 1 |
| IFI27 IFIT1 ISG15 IL1R2 FAM20A RETN | 0,891 | 0,882 | 0,899 | 0,926 | 0,873 | 0,979 | 0,929 | 0,850 | 1 |
| IFI27 IFI44L SIGLEC1 SLC1A2 OLAH RETN | 0,891 | 0,882 | 0,899 | 0,926 | 0,875 | 0,978 | 0,951 | 0,895 | 1 |
| RSAD2 IFI27 SIGLEC1 ISG15 SLC1A2 OLAH | 0,891 | 0,882 | 0,899 | 0,929 | 0,878 | 0,980 | 0,938 | 0,878 | 0,998 |
| IFI27 IFIT1 SIGLEC1 ISG15 OLAH MMP8 | 0,891 | 0,882 | 0,899 | 0,919 | 0,862 | 0,975 | 0,941 | 0,885 | 0,998 |
| IFI27 OAS1 ISG15 HERC6 SLC1A2 OLAH | 0,891 | 0,882 | 0,900 | 0,929 | 0,879 | 0,978 | 0,924 | 0,855 | 0,993 |
| IFI27 OAS1 IFIT1 OLAH RETN MMP8 | 0,891 | 0,881 | 0,900 | 0,918 | 0,858 | 0,978 | 0,952 | 0,896 | 1 |
| IFI27 IFIT1 IFI44L HERC6 IL1R2 OLAH | 0,891 | 0,882 | 0,899 | 0,926 | 0,876 | 0,976 | 0,936 | 0,879 | 0,993 |
| RSAD2 IFI27 SIGLEC1 SLC1A2 OLAH MMP8 | 0,891 | 0,882 | 0,899 | 0,927 | 0,876 | 0,979 | 0,943 | 0,886 | 1 |
| IFI27 IFIT1 ISG15 SLC1A2 OLAH MMP8 | 0,891 | 0,882 | 0,899 | 0,926 | 0,874 | 0,978 | 0,929 | 0,862 | 0,997 |
| IFI27 ISG15 SLC1A2 IL1R2 OLAH MMP8 | 0,891 | 0,883 | 0,899 | 0,927 | 0,878 | 0,976 | 0,925 | 0,854 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 IFI44L SIGLEC1 FAM20A RETN | 0,891 | 0,881 | 0,900 | 0,918 | 0,858 | 0,979 | 0,948 | 0,881 | 1 |
| IFI27 OAS1 IL1R2 OLAH RETN MMP8 | 0,891 | 0,882 | 0,899 | 0,922 | 0,866 | 0,977 | 0,953 | 0,902 | 1 |
| IFI27 IFI44L SIGLEC1 ISG15 HERC6 IL1R2 | 0,890 | 0,883 | 0,898 | 0,923 | 0,876 | 0,970 | 0,938 | 0,885 | 0,991 |
| RSAD2 IFI27 IFIT1 IFI44L HERC6 OLAH | 0,890 | 0,882 | 0,899 | 0,924 | 0,872 | 0,976 | 0,935 | 0,876 | 0,993 |
| IFIT1 IFI44L SIGLEC1 HERC6 OLAH FAM20A | 0,890 | 0,881 | 0,900 | 0,917 | 0,857 | 0,977 | 0,946 | 0,887 | 1 |
| RSAD2 IFI27 IFI44L SIGLEC1 IL1R2 OLAH | 0,890 | 0,883 | 0,898 | 0,924 | 0,873 | 0,975 | 0,950 | 0,902 | 0,998 |
| IFI27 IFI44L SIGLEC1 ISG15 OLAH RETN | 0,890 | 0,882 | 0,899 | 0,919 | 0,862 | 0,975 | 0,947 | 0,888 | 1 |
| RSAD2 IFI27 SIGLEC1 HERC6 IL1R2 MMP8 | 0,890 | 0,883 | 0,898 | 0,921 | 0,873 | 0,968 | 0,949 | 0,902 | 0,996 |
| IFI27 IFIT1 IFI44L SIGLEC1 HERC6 IL1R2 | 0,890 | 0,883 | 0,898 | 0,922 | 0,874 | 0,970 | 0,941 | 0,890 | 0,993 |
| IFI27 OAS1 SIGLEC1 ISG15 SLC1A2 OLAH | 0,890 | 0,882 | 0,899 | 0,928 | 0,877 | 0,979 | 0,939 | 0,880 | 0,998 |
| RSAD2 IFI27 OAS1 IL1R2 FAM20A RETN | 0,890 | 0,882 | 0,899 | 0,925 | 0,873 | 0,978 | 0,929 | 0,850 | 1 |
| IFI27 OAS1 HERC6 FAM20A RETN MMP8 | 0,890 | 0,881 | 0,900 | 0,923 | 0,865 | 0,981 | 0,934 | 0,853 | 1 |
| IFI27 SIGLEC1 SLC1A2 IL1R2 FAM20A RETN | 0,890 | 0,881 | 0,900 | 0,929 | 0,878 | 0,980 | 0,948 | 0,877 | 1 |
| IFI27 OAS1 IFIT1 HERC6 SLC1A2 OLAH | 0,890 | 0,881 | 0,900 | 0,928 | 0,878 | 0,978 | 0,933 | 0,867 | 0,998 |
| IFI27 OAS1 HERC6 SLC1A2 IL1R2 FAM20A | 0,890 | 0,881 | 0,899 | 0,930 | 0,884 | 0,977 | 0,923 | 0,835 | 1 |
| IFI27 OAS1 IFIT1 SIGLEC1 ISG15 FAM20A | 0,890 | 0,881 | 0,899 | 0,916 | 0,853 | 0,980 | 0,942 | 0,870 | 1 |
| RSAD2 IFI27 HERC6 FAM20A RETN MMP8 | 0,890 | 0,881 | 0,900 | 0,923 | 0,865 | 0,980 | 0,934 | 0,854 | 1 |
| RSAD2 IFI27 IFIT1 SIGLEC1 HERC6 RETN | 0,890 | 0,882 | 0,899 | 0,915 | 0,855 | 0,974 | 0,941 | 0,886 | 0,997 |
| RSAD2 IFI27 IFI44L ISG15 HERC6 OLAH | 0,890 | 0,882 | 0,899 | 0,923 | 0,871 | 0,975 | 0,934 | 0,875 | 0,993 |
| IFI27 OAS1 SIGLEC1 HERC6 FAM20A MMP8 | 0,890 | 0,881 | 0,900 | 0,915 | 0,855 | 0,976 | 0,940 | 0,868 | 1 |
| IFI27 SIGLEC1 HERC6 FAM20A RETN MMP8 | 0,890 | 0,880 | 0,901 | 0,918 | 0,855 | 0,981 | 0,953 | 0,881 | 1 |
| IFI27 OAS1 SIGLEC1 SLC1A2 IL1R2 OLAH | 0,890 | 0,882 | 0,899 | 0,927 | 0,877 | 0,977 | 0,947 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 SIGLEC1 ISG15 FAM20A RETN MMP8 | 0,890 | 0,880 | 0,900 | 0,917 | 0,853 | 0,981 | 0,951 | 0,878 | 1 |
| RSAD2 IFI27 OAS1 IFI44L HERC6 RETN | 0,890 | 0,881 | 0,899 | 0,915 | 0,853 | 0,977 | 0,934 | 0,861 | 1 |
| IFI27 IFI44L ISG15 OLAH RETN MMP8 | 0,890 | 0,881 | 0,899 | 0,919 | 0,860 | 0,978 | 0,950 | 0,894 | 1 |
| IFI27 IFI44L HERC6 IL1R2 RETN MMP8 | 0,890 | 0,882 | 0,899 | 0,922 | 0,868 | 0,975 | 0,933 | 0,869 | 0,996 |
| IFI27 OAS1 HERC6 SLC1A2 IL1R2 OLAH | 0,890 | 0,881 | 0,899 | 0,927 | 0,877 | 0,976 | 0,929 | 0,862 | 0,997 |
| IFI27 IFI44L SLC1A2 OLAH FAM20A RETN | 0,890 | 0,881 | 0,899 | 0,924 | 0,867 | 0,982 | 0,924 | 0,844 | 1 |
| IFI27 IFIT1 SIGLEC1 ISG15 FAM20A MMP8 | 0,890 | 0,881 | 0,899 | 0,916 | 0,853 | 0,980 | 0,942 | 0,871 | 1 |
| RSAD2 IFI27 IL1R2 OLAH RETN MMP8 | 0,890 | 0,881 | 0,899 | 0,921 | 0,865 | 0,977 | 0,953 | 0,900 | 1 |
| RSAD2 IFI27 OAS1 SIGLEC1 IL1R2 OLAH | 0,890 | 0,882 | 0,898 | 0,920 | 0,868 | 0,973 | 0,943 | 0,890 | 0,997 |
| IFI27 OAS1 IFIT1 SIGLEC1 OLAH MMP8 | 0,890 | 0,882 | 0,899 | 0,919 | 0,864 | 0,974 | 0,945 | 0,890 | 0,999 |
| RSAD2 IFI27 SIGLEC1 OLAH RETN MMP8 | 0,890 | 0,881 | 0,899 | 0,920 | 0,862 | 0,979 | 0,961 | 0,908 | 1 |
| RSAD2 IFI27 IFI44L IL1R2 FAM20A RETN | 0,890 | 0,881 | 0,899 | 0,924 | 0,870 | 0,978 | 0,928 | 0,848 | 1 |
| IFI27 IFIT1 SIGLEC1 IL1R2 OLAH RETN | 0,890 | 0,882 | 0,898 | 0,919 | 0,863 | 0,975 | 0,948 | 0,892 | 1 |
| RSAD2 IFI27 SIGLEC1 SLC1A2 IL1R2 OLAH | 0,890 | 0,881 | 0,899 | 0,928 | 0,878 | 0,977 | 0,942 | 0,885 | 0,999 |
| RSAD2 IFI27 HERC6 SLC1A2 OLAH MMP8 | 0,890 | 0,881 | 0,899 | 0,927 | 0,876 | 0,977 | 0,927 | 0,860 | 0,994 |
| IFI27 IFIT1 SIGLEC1 HERC6 SLC1A2 OLAH | 0,890 | 0,881 | 0,899 | 0,930 | 0,879 | 0,980 | 0,941 | 0,886 | 0,996 |
| IFI27 IFIT1 IFI44L SIGLEC1 ISG15 OLAH | 0,890 | 0,882 | 0,898 | 0,919 | 0,863 | 0,975 | 0,940 | 0,882 | 0,998 |
| RSAD2 IFI27 HERC6 SLC1A2 IL1R2 FAM20A | 0,890 | 0,881 | 0,899 | 0,930 | 0,883 | 0,977 | 0,925 | 0,838 | 1 |
| IFI27 IFIT1 SLC1A2 OLAH FAM20A RETN | 0,890 | 0,881 | 0,899 | 0,924 | 0,868 | 0,981 | 0,926 | 0,849 | 1 |
| RSAD2 IFI27 IFIT1 ISG15 HERC6 OLAH | 0,890 | 0,881 | 0,899 | 0,923 | 0,870 | 0,975 | 0,935 | 0,876 | 0,994 |
| IFI27 IFI44L ISG15 SLC1A2 OLAH MMP8 | 0,890 | 0,881 | 0,899 | 0,926 | 0,874 | 0,978 | 0,925 | 0,855 | 0,995 |
| IFI27 IFI44L SIGLEC1 ISG15 IL1R2 RETN | 0,890 | 0,882 | 0,898 | 0,921 | 0,869 | 0,974 | 0,950 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 IFI44L SIGLEC1 HERC6 IL1R2 | 0,890 | 0,882 | 0,898 | 0,923 | 0,874 | 0,971 | 0,940 | 0,888 | 0,992 |
| IFI27 SIGLEC1 ISG15 SLC1A2 OLAH RETN | 0,890 | 0,881 | 0,899 | 0,927 | 0,876 | 0,978 | 0,941 | 0,881 | 1 |
| IFI27 IFIT1 HERC6 FAM20A RETN MMP8 | 0,890 | 0,880 | 0,900 | 0,922 | 0,863 | 0,981 | 0,933 | 0,851 | 1 |
| RSAD2 IFI27 ISG15 IL1R2 FAM20A RETN | 0,890 | 0,881 | 0,899 | 0,925 | 0,872 | 0,977 | 0,928 | 0,849 | 1 |
| IFIT1 SIGLEC1 HERC6 FAM20A RETN MMP8 | 0,890 | 0,881 | 0,899 | 0,917 | 0,856 | 0,978 | 0,945 | 0,878 | 1 |
| IFI27 SIGLEC1 SLC1A2 IL1R2 OLAH MMP8 | 0,890 | 0,881 | 0,898 | 0,927 | 0,877 | 0,977 | 0,947 | 0,894 | 0,999 |
| IFI27 IFIT1 IFI44L SIGLEC1 OLAH RETN | 0,890 | 0,881 | 0,898 | 0,916 | 0,857 | 0,975 | 0,948 | 0,888 | 1 |
| IFI27 OAS1 IFIT1 IL1R2 OLAH MMP8 | 0,890 | 0,882 | 0,898 | 0,922 | 0,870 | 0,973 | 0,943 | 0,890 | 0,997 |
| IFI27 IFIT1 SLC1A2 IL1R2 FAM20A MMP8 | 0,890 | 0,881 | 0,899 | 0,927 | 0,878 | 0,976 | 0,929 | 0,845 | 1 |
| RSAD2 IFI27 SLC1A2 OLAH FAM20A RETN | 0,890 | 0,881 | 0,899 | 0,924 | 0,868 | 0,981 | 0,922 | 0,841 | 1 |
| IFI27 IFIT1 SIGLEC1 ISG15 HERC6 RETN | 0,890 | 0,881 | 0,899 | 0,910 | 0,848 | 0,972 | 0,949 | 0,897 | 1 |
| OAS1 SIGLEC1 IL1R2 OLAH FAM20A MMP8 | 0,890 | 0,881 | 0,899 | 0,918 | 0,861 | 0,975 | 0,964 | 0,911 | 1 |
| IFI27 SIGLEC1 ISG15 IL1R2 OLAH RETN | 0,890 | 0,881 | 0,898 | 0,919 | 0,863 | 0,975 | 0,947 | 0,891 | 1 |
| IFI27 OAS1 SIGLEC1 HERC6 SLC1A2 RETN | 0,890 | 0,881 | 0,899 | 0,922 | 0,867 | 0,976 | 0,947 | 0,885 | 1 |
| IFI27 OAS1 SIGLEC1 IL1R2 OLAH RETN | 0,890 | 0,881 | 0,898 | 0,919 | 0,863 | 0,974 | 0,952 | 0,898 | 1 |
| RSAD2 IFI27 IFIT1 IFI44L SIGLEC1 FAM20A | 0,890 | 0,881 | 0,899 | 0,915 | 0,850 | 0,980 | 0,948 | 0,880 | 1 |
| IFI27 OAS1 SIGLEC1 ISG15 IL1R2 OLAH | 0,890 | 0,882 | 0,898 | 0,921 | 0,869 | 0,973 | 0,943 | 0,889 | 0,998 |
| IFI27 IFIT1 SIGLEC1 SLC1A2 IL1R2 OLAH | 0,890 | 0,881 | 0,898 | 0,928 | 0,878 | 0,978 | 0,946 | 0,892 | 0,999 |
| IFI27 IFI44L SLC1A2 IL1R2 OLAH MMP8 | 0,890 | 0,881 | 0,898 | 0,927 | 0,878 | 0,977 | 0,934 | 0,869 | 0,998 |
| RSAD2 IFI27 IFIT1 SIGLEC1 IL1R2 OLAH | 0,890 | 0,882 | 0,898 | 0,921 | 0,869 | 0,973 | 0,942 | 0,888 | 0,996 |
| IFI27 OAS1 IFIT1 SIGLEC1 SLC1A2 OLAH | 0,890 | 0,881 | 0,898 | 0,927 | 0,876 | 0,979 | 0,945 | 0,888 | 1 |
| IFI27 IFIT1 IFI44L SIGLEC1 OLAH MMP8 | 0,890 | 0,881 | 0,898 | 0,919 | 0,863 | 0,976 | 0,949 | 0,898 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 HERC6 SLC1A2 IL1R2 OLAH | 0,890 | 0,881 | 0,898 | 0,927 | 0,877 | 0,976 | 0,928 | 0,862 | 0,994 |
| OAS1 IFI44L SIGLEC1 IL1R2 FAM20A MMP8 | 0,890 | 0,881 | 0,898 | 0,918 | 0,861 | 0,975 | 0,963 | 0,908 | 1 |
| IFI27 OAS1 ISG15 HERC6 RETN MMP8 | 0,890 | 0,881 | 0,899 | 0,917 | 0,858 | 0,976 | 0,935 | 0,868 | 1 |
| RSAD2 IFI27 OAS1 IFIT1 HERC6 RETN | 0,890 | 0,880 | 0,899 | 0,916 | 0,854 | 0,978 | 0,935 | 0,862 | 1 |
| IFI27 OAS1 IFI44L SIGLEC1 ISG15 OLAH | 0,890 | 0,881 | 0,898 | 0,919 | 0,863 | 0,975 | 0,941 | 0,887 | 0,996 |
| RSAD2 IFI27 HERC6 IL1R2 RETN MMP8 | 0,890 | 0,881 | 0,898 | 0,921 | 0,868 | 0,974 | 0,938 | 0,878 | 0,998 |
| IFI27 IFIT1 IFI44L SIGLEC1 IL1R2 RETN | 0,890 | 0,882 | 0,898 | 0,921 | 0,868 | 0,974 | 0,949 | 0,897 | 1 |
| IFI27 IFIT1 ISG15 OLAH RETN MMP8 | 0,890 | 0,880 | 0,899 | 0,918 | 0,858 | 0,978 | 0,951 | 0,895 | 1 |
| IFI27 IFIT1 IFI44L IL1R2 OLAH MMP8 | 0,890 | 0,881 | 0,898 | 0,922 | 0,870 | 0,973 | 0,942 | 0,889 | 0,995 |
| IFI27 IFIT1 SIGLEC1 IL1R2 RETN MMP8 | 0,890 | 0,881 | 0,898 | 0,921 | 0,867 | 0,975 | 0,963 | 0,923 | 1 |
| RSAD2 IFI27 SIGLEC1 ISG15 IL1R2 OLAH | 0,890 | 0,881 | 0,898 | 0,920 | 0,868 | 0,973 | 0,945 | 0,892 | 0,998 |
| IFI27 OAS1 SLC1A2 OLAH FAM20A RETN | 0,890 | 0,881 | 0,899 | 0,925 | 0,870 | 0,981 | 0,922 | 0,841 | 1 |
| IFIT1 SIGLEC1 IL1R2 FAM20A RETN MMP8 | 0,890 | 0,881 | 0,898 | 0,920 | 0,864 | 0,977 | 0,968 | 0,918 | 1 |
| IFI27 OAS1 IFI44L SIGLEC1 OLAH MMP8 | 0,890 | 0,881 | 0,898 | 0,920 | 0,864 | 0,976 | 0,949 | 0,898 | 1,000 |
| IFI27 OAS1 IFI44L SIGLEC1 HERC6 FAM20A | 0,890 | 0,881 | 0,898 | 0,915 | 0,855 | 0,975 | 0,946 | 0,872 | 1 |
| IFI27 IFIT1 SIGLEC1 ISG15 OLAH RETN | 0,890 | 0,881 | 0,898 | 0,917 | 0,860 | 0,975 | 0,946 | 0,888 | 1 |
| IFI27 OAS1 SIGLEC1 ISG15 HERC6 IL1R2 | 0,890 | 0,882 | 0,897 | 0,921 | 0,874 | 0,968 | 0,950 | 0,904 | 0,996 |
| IFI27 IFIT1 ISG15 IL1R2 OLAH MMP8 | 0,890 | 0,881 | 0,898 | 0,921 | 0,870 | 0,973 | 0,945 | 0,892 | 0,997 |
| RSAD2 IFI27 OAS1 IL1R2 OLAH MMP8 | 0,890 | 0,881 | 0,898 | 0,920 | 0,868 | 0,973 | 0,943 | 0,890 | 0,996 |
| IFI27 OAS1 SIGLEC1 ISG15 OLAH MMP8 | 0,889 | 0,881 | 0,898 | 0,918 | 0,861 | 0,974 | 0,943 | 0,887 | 1,000 |
| IFI27 SIGLEC1 HERC6 SLC1A2 OLAH MMP8 | 0,889 | 0,880 | 0,899 | 0,927 | 0,876 | 0,979 | 0,941 | 0,883 | 0,999 |
| IFI27 SIGLEC1 HERC6 IL1R2 RETN MMP8 | 0,889 | 0,881 | 0,898 | 0,922 | 0,868 | 0,977 | 0,963 | 0,923 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 SIGLEC1 SLC1A2 FAM20A RETN | 0,889 | 0,880 | 0,899 | 0,924 | 0,868 | 0,980 | 0,941 | 0,865 | 1 |
| RSAD2 IFI27 IFI44L SIGLEC1 HERC6 RETN | 0,889 | 0,881 | 0,898 | 0,916 | 0,857 | 0,975 | 0,942 | 0,887 | 0,997 |
| RSAD2 IFI27 OAS1 OLAH RETN MMP8 | 0,889 | 0,880 | 0,899 | 0,919 | 0,860 | 0,979 | 0,951 | 0,894 | 1 |
| IFI27 IFIT1 SIGLEC1 SLC1A2 OLAH RETN | 0,889 | 0,880 | 0,898 | 0,927 | 0,875 | 0,979 | 0,949 | 0,892 | 1 |
| IFI27 OAS1 IFIT1 IFI44L SIGLEC1 OLAH | 0,889 | 0,881 | 0,897 | 0,920 | 0,863 | 0,976 | 0,949 | 0,898 | 1,000 |
| RSAD2 IFI27 IFIT1 IL1R2 OLAH MMP8 | 0,889 | 0,881 | 0,898 | 0,922 | 0,870 | 0,974 | 0,946 | 0,894 | 0,997 |
| RSAD2 IFI27 IFIT1 SIGLEC1 HERC6 IL1R2 | 0,889 | 0,882 | 0,897 | 0,922 | 0,874 | 0,969 | 0,946 | 0,897 | 0,995 |
| RSAD2 IFI27 OAS1 SIGLEC1 SLC1A2 OLAH | 0,889 | 0,881 | 0,898 | 0,928 | 0,876 | 0,980 | 0,948 | 0,892 | 1 |
| RSAD2 IFI27 OAS1 HERC6 SLC1A2 OLAH | 0,889 | 0,880 | 0,898 | 0,929 | 0,879 | 0,979 | 0,932 | 0,864 | 0,999 |
| IFI27 OAS1 IFIT1 SIGLEC1 ISG15 OLAH | 0,889 | 0,881 | 0,898 | 0,918 | 0,862 | 0,974 | 0,941 | 0,885 | 0,998 |
| RSAD2 IFI27 IFIT1 SIGLEC1 ISG15 FAM20A | 0,889 | 0,880 | 0,898 | 0,916 | 0,852 | 0,980 | 0,941 | 0,870 | 1 |
| RSAD2 IFI27 OAS1 IFIT1 SIGLEC1 FAM20A | 0,889 | 0,880 | 0,899 | 0,915 | 0,850 | 0,979 | 0,947 | 0,877 | 1 |
| RSAD2 IFI27 SIGLEC1 IL1R2 RETN MMP8 | 0,889 | 0,881 | 0,898 | 0,922 | 0,868 | 0,975 | 0,962 | 0,921 | 1 |
| IFI27 IFI44L SIGLEC1 ISG15 IL1R2 MMP8 | 0,889 | 0,882 | 0,896 | 0,918 | 0,869 | 0,967 | 0,942 | 0,891 | 0,994 |
| RSAD2 IFI27 IFIT1 HERC6 IL1R2 RETN | 0,889 | 0,881 | 0,897 | 0,920 | 0,867 | 0,973 | 0,941 | 0,886 | 0,996 |
| IFI27 IFI44L ISG15 HERC6 IL1R2 RETN | 0,889 | 0,881 | 0,897 | 0,921 | 0,868 | 0,974 | 0,936 | 0,876 | 0,995 |
| RSAD2 IFI27 HERC6 SLC1A2 OLAH RETN | 0,889 | 0,880 | 0,898 | 0,926 | 0,876 | 0,977 | 0,935 | 0,870 | 1 |
| IFI27 OAS1 SIGLEC1 SLC1A2 OLAH RETN | 0,889 | 0,880 | 0,898 | 0,925 | 0,873 | 0,978 | 0,948 | 0,886 | 1 |
| IFI27 HERC6 SLC1A2 IL1R2 FAM20A RETN | 0,889 | 0,880 | 0,898 | 0,933 | 0,885 | 0,981 | 0,925 | 0,840 | 1 |
| IFI27 IFIT1 ISG15 HERC6 SLC1A2 OLAH | 0,889 | 0,880 | 0,898 | 0,928 | 0,877 | 0,978 | 0,928 | 0,861 | 0,995 |
| RSAD2 IFI27 SIGLEC1 ISG15 HERC6 RETN | 0,889 | 0,880 | 0,898 | 0,918 | 0,860 | 0,976 | 0,951 | 0,901 | 1 |
| IFIT1 SIGLEC1 HERC6 IL1R2 FAM20A RETN | 0,889 | 0,880 | 0,898 | 0,919 | 0,861 | 0,976 | 0,953 | 0,897 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 IFI44L HERC6 RETN | 0,889 | 0,880 | 0,898 | 0,916 | 0,855 | 0,977 | 0,935 | 0,865 | 1 |
| IFI27 OAS1 IFI44L SIGLEC1 OLAH RETN | 0,889 | 0,881 | 0,897 | 0,916 | 0,857 | 0,975 | 0,948 | 0,888 | 1 |
| RSAD2 IFI44L SIGLEC1 IL1R2 FAM20A MMP8 | 0,889 | 0,880 | 0,898 | 0,919 | 0,862 | 0,975 | 0,963 | 0,908 | 1 |
| IFI27 IFI44L SLC1A2 IL1R2 FAM20A RETN | 0,889 | 0,880 | 0,898 | 0,926 | 0,875 | 0,977 | 0,925 | 0,840 | 1 |
| IFI27 ISG15 HERC6 SLC1A2 OLAH MMP8 | 0,889 | 0,880 | 0,898 | 0,928 | 0,877 | 0,978 | 0,926 | 0,859 | 0,993 |
| IFI27 SIGLEC1 ISG15 IL1R2 RETN MMP8 | 0,889 | 0,881 | 0,897 | 0,920 | 0,866 | 0,975 | 0,965 | 0,927 | 1 |
| RSAD2 IFI27 SLC1A2 IL1R2 OLAH MMP8 | 0,889 | 0,881 | 0,897 | 0,925 | 0,875 | 0,976 | 0,935 | 0,871 | 0,998 |
| RSAD2 IFI27 IFI44L SIGLEC1 OLAH MMP8 | 0,889 | 0,881 | 0,897 | 0,919 | 0,862 | 0,975 | 0,949 | 0,898 | 1,000 |
| IFI27 IFI44L ISG15 SLC1A2 IL1R2 FAM20A | 0,889 | 0,880 | 0,897 | 0,930 | 0,882 | 0,977 | 0,926 | 0,842 | 1 |
| RSAD2 IFI27 SLC1A2 IL1R2 FAM20A MMP8 | 0,889 | 0,881 | 0,897 | 0,929 | 0,882 | 0,976 | 0,926 | 0,839 | 1 |
| IFI27 IFI44L SIGLEC1 HERC6 SLC1A2 OLAH | 0,889 | 0,880 | 0,898 | 0,930 | 0,879 | 0,980 | 0,945 | 0,890 | 0,999 |
| IFI27 OAS1 ISG15 IL1R2 OLAH MMP8 | 0,889 | 0,881 | 0,897 | 0,923 | 0,871 | 0,974 | 0,939 | 0,883 | 0,995 |
| IFI27 IFIT1 SLC1A2 IL1R2 OLAH MMP8 | 0,889 | 0,880 | 0,898 | 0,926 | 0,876 | 0,976 | 0,935 | 0,872 | 0,998 |
| RSAD2 IFI27 IFIT1 SIGLEC1 SLC1A2 OLAH | 0,889 | 0,880 | 0,897 | 0,927 | 0,875 | 0,978 | 0,941 | 0,883 | 1,000 |
| RSAD2 IFI27 OAS1 SIGLEC1 OLAH MMP8 | 0,889 | 0,880 | 0,897 | 0,918 | 0,862 | 0,974 | 0,942 | 0,887 | 0,998 |
| RSAD2 IFI27 IFIT1 HERC6 SLC1A2 OLAH | 0,889 | 0,880 | 0,898 | 0,928 | 0,877 | 0,978 | 0,930 | 0,865 | 0,995 |
| RSAD2 IFI27 IFI44L HERC6 IL1R2 RETN | 0,889 | 0,881 | 0,897 | 0,921 | 0,868 | 0,974 | 0,936 | 0,876 | 0,996 |
| RSAD2 IFI27 IFIT1 OLAH RETN MMP8 | 0,889 | 0,880 | 0,898 | 0,919 | 0,859 | 0,978 | 0,952 | 0,896 | 1 |
| IFI27 IFI44L HERC6 SLC1A2 IL1R2 FAM20A | 0,889 | 0,880 | 0,898 | 0,929 | 0,881 | 0,977 | 0,928 | 0,843 | 1 |
| IFI27 OAS1 ISG15 OLAH RETN MMP8 | 0,889 | 0,880 | 0,898 | 0,918 | 0,859 | 0,978 | 0,949 | 0,893 | 1 |
| RSAD2 IFI27 IFIT1 SIGLEC1 OLAH MMP8 | 0,889 | 0,880 | 0,897 | 0,919 | 0,864 | 0,975 | 0,942 | 0,887 | 0,997 |
| IFI27 IFIT1 ISG15 SLC1A2 IL1R2 OLAH | 0,889 | 0,880 | 0,897 | 0,928 | 0,878 | 0,978 | 0,926 | 0,858 | 0,994 |

(suite)

| Modèle | AUC modèle | IC 95% | | AUC Train | IC 95% Train | | AUC Test | IC 95% Test | |
|---|---|---|---|---|---|---|---|---|---|
| IFI27 ISG15 HERC6 FAM20A RETN MMP8 | 0,889 | 0,879 | 0,899 | 0,920 | 0,861 | 0,980 | 0,941 | 0,864 | 1 |
| IFI27 IFIT1 IFI44L OLAH RETN MMP8 | 0,889 | 0,880 | 0,898 | 0,920 | 0,862 | 0,979 | 0,951 | 0,893 | 1 |
| RSAD2 IFI27 IFI44L SIGLEC1 ISG15 OLAH | 0,889 | 0,881 | 0,897 | 0,919 | 0,862 | 0,976 | 0,942 | 0,887 | 0,997 |
| RSAD2 IFI27 ISG15 HERC6 SLC1A2 OLAH | 0,889 | 0,880 | 0,897 | 0,928 | 0,878 | 0,978 | 0,924 | 0,855 | 0,993 |
| IFI27 OAS1 IFIT1 SLC1A2 OLAH MMP8 | 0,889 | 0,880 | 0,898 | 0,928 | 0,876 | 0,979 | 0,940 | 0,879 | 1 |
| IFI44L SIGLEC1 SLC1A2 IL1R2 FAM20A MMP8 | 0,889 | 0,880 | 0,897 | 0,924 | 0,876 | 0,973 | 0,957 | 0,889 | 1 |
| IFI27 OAS1 IFI44L SIGLEC1 IL1R2 RETN | 0,889 | 0,881 | 0,897 | 0,920 | 0,867 | 0,973 | 0,950 | 0,899 | 1 |
| OAS1 IFIT1 SIGLEC1 HERC6 OLAH FAM20A | 0,889 | 0,879 | 0,898 | 0,913 | 0,852 | 0,974 | 0,948 | 0,892 | 1 |
| IFIT1 SIGLEC1 ISG15 HERC6 OLAH FAM20A | 0,889 | 0,879 | 0,898 | 0,912 | 0,852 | 0,973 | 0,951 | 0,896 | 1 |
| IFI27 IFI44L ISG15 SLC1A2 IL1R2 OLAH | 0,889 | 0,880 | 0,897 | 0,927 | 0,878 | 0,977 | 0,925 | 0,854 | 0,996 |
| IFI27 IFIT1 HERC6 IL1R2 RETN MMP8 | 0,889 | 0,880 | 0,897 | 0,922 | 0,868 | 0,976 | 0,945 | 0,890 | 0,999 |
| IFI44L SIGLEC1 IL1R2 FAM20A RETN MMP8 | 0,889 | 0,880 | 0,898 | 0,918 | 0,860 | 0,976 | 0,962 | 0,905 | 1 |
| RSAD2 IFI27 OAS1 IFIT1 IL1R2 OLAH | 0,889 | 0,880 | 0,897 | 0,921 | 0,868 | 0,973 | 0,941 | 0,885 | 0,997 |
| RSAD2 SIGLEC1 ISG15 IL1R2 FAM20A MMP8 | 0,889 | 0,880 | 0,897 | 0,917 | 0,860 | 0,975 | 0,962 | 0,905 | 1 |
| IFI27 OAS1 IFIT1 SIGLEC1 IL1R2 RETN | 0,889 | 0,881 | 0,897 | 0,921 | 0,868 | 0,974 | 0,953 | 0,902 | 1 |
| RSAD2 IFI27 SIGLEC1 ISG15 OLAH MMP8 | 0,889 | 0,880 | 0,897 | 0,918 | 0,862 | 0,974 | 0,941 | 0,884 | 0,998 |
| IFIT1 SIGLEC1 ISG15 HERC6 FAM20A RETN | 0,889 | 0,880 | 0,898 | 0,916 | 0,855 | 0,976 | 0,942 | 0,875 | 1 |
| IFI27 IFIT1 IFI44L SIGLEC1 SLC1A2 OLAH | 0,889 | 0,880 | 0,897 | 0,926 | 0,875 | 0,978 | 0,946 | 0,890 | 1 |
| IFI27 OAS1 IFIT1 IL1R2 OLAH RETN | 0,889 | 0,880 | 0,897 | 0,918 | 0,861 | 0,974 | 0,945 | 0,886 | 1 |
| IFI27 IFIT1 SIGLEC1 HERC6 IL1R2 MMP8 | 0,889 | 0,881 | 0,896 | 0,921 | 0,874 | 0,969 | 0,948 | 0,900 | 0,996 |
| IFI27 SIGLEC1 HERC6 IL1R2 OLAH RETN | 0,889 | 0,880 | 0,897 | 0,918 | 0,861 | 0,976 | 0,948 | 0,893 | 1 |
| IFIT1 SIGLEC1 SLC1A2 IL1R2 FAM20A MMP8 | 0,889 | 0,880 | 0,897 | 0,927 | 0,880 | 0,974 | 0,960 | 0,898 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 SLC1A2 IL1R2 OLAH RETN MMP8 | 0,889 | 0,879 | 0,898 | 0,926 | 0,875 | 0,978 | 0,938 | 0,871 | 1 |
| IFI27 IFIT1 IFI44L HERC6 IL1R2 RETN | 0,889 | 0,880 | 0,897 | 0,920 | 0,866 | 0,973 | 0,935 | 0,874 | 0,995 |
| IFI27 IFIT1 SIGLEC1 HERC6 SLC1A2 FAM20A | 0,889 | 0,880 | 0,897 | 0,926 | 0,874 | 0,978 | 0,941 | 0,864 | 1 |
| RSAD2 IFI27 SIGLEC1 IL1R2 OLAH RETN | 0,889 | 0,880 | 0,897 | 0,919 | 0,862 | 0,975 | 0,948 | 0,892 | 1 |
| IFI27 OAS1 IFIT1 SIGLEC1 FAM20A MMP8 | 0,888 | 0,879 | 0,898 | 0,914 | 0,849 | 0,978 | 0,948 | 0,881 | 1 |
| IFI27 ISG15 HERC6 SLC1A2 IL1R2 OLAH | 0,888 | 0,880 | 0,897 | 0,928 | 0,879 | 0,977 | 0,926 | 0,858 | 0,994 |
| IFI27 SIGLEC1 SLC1A2 OLAH RETN MMP8 | 0,888 | 0,879 | 0,898 | 0,924 | 0,869 | 0,979 | 0,957 | 0,906 | 1 |
| IFI27 HERC6 SLC1A2 IL1R2 FAM20A MMP8 | 0,888 | 0,880 | 0,897 | 0,931 | 0,885 | 0,977 | 0,925 | 0,839 | 1 |
| IFI27 IFI44L HERC6 FAM20A RETN MMP8 | 0,888 | 0,878 | 0,898 | 0,920 | 0,860 | 0,980 | 0,940 | 0,862 | 1 |
| RSAD2 SIGLEC1 SLC1A2 IL1R2 FAM20A MMP8 | 0,888 | 0,879 | 0,897 | 0,924 | 0,875 | 0,972 | 0,959 | 0,895 | 1 |
| IFI27 IFIT1 IFI44L SIGLEC1 ISG15 IL1R2 | 0,888 | 0,881 | 0,896 | 0,920 | 0,871 | 0,969 | 0,945 | 0,894 | 0,995 |
| IFIT1 IFI44L SIGLEC1 HERC6 FAM20A RETN | 0,888 | 0,880 | 0,897 | 0,914 | 0,853 | 0,976 | 0,936 | 0,869 | 1 |
| IFI27 OAS1 IFI44L SIGLEC1 SLC1A2 OLAH | 0,888 | 0,880 | 0,897 | 0,927 | 0,876 | 0,978 | 0,948 | 0,893 | 1 |
| IFI27 IFI44L SLC1A2 OLAH RETN MMP8 | 0,888 | 0,879 | 0,897 | 0,925 | 0,871 | 0,980 | 0,943 | 0,881 | 1 |
| SIGLEC1 SLC1A2 IL1R2 OLAH FAM20A MMP8 | 0,888 | 0,879 | 0,897 | 0,922 | 0,872 | 0,972 | 0,957 | 0,890 | 1 |
| OAS1 SIGLEC1 ISG15 IL1R2 FAM20A MMP8 | 0,888 | 0,879 | 0,897 | 0,915 | 0,857 | 0,973 | 0,963 | 0,906 | 1 |
| RSAD2 IFI27 ISG15 SLC1A2 OLAH MMP8 | 0,888 | 0,880 | 0,897 | 0,927 | 0,875 | 0,979 | 0,928 | 0,860 | 0,996 |
| RSAD2 IFI27 OAS1 SLC1A2 OLAH MMP8 | 0,888 | 0,879 | 0,897 | 0,926 | 0,874 | 0,978 | 0,935 | 0,869 | 1 |
| IFI27 OAS1 ISG15 SLC1A2 OLAH MMP8 | 0,888 | 0,880 | 0,897 | 0,927 | 0,875 | 0,979 | 0,922 | 0,850 | 0,993 |
| IFI27 HERC6 SLC1A2 OLAH RETN MMP8 | 0,888 | 0,879 | 0,897 | 0,924 | 0,870 | 0,979 | 0,946 | 0,887 | 1 |
| RSAD2 IFI27 ISG15 IL1R2 OLAH MMP8 | 0,888 | 0,880 | 0,896 | 0,922 | 0,869 | 0,974 | 0,943 | 0,890 | 0,997 |
| RSAD2 IFI27 IFIT1 SLC1A2 OLAH MMP8 | 0,888 | 0,880 | 0,897 | 0,927 | 0,875 | 0,978 | 0,936 | 0,873 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 IFIT1 SIGLEC1 OLAH RETN | 0,888 | 0,880 | 0,897 | 0,916 | 0,857 | 0,974 | 0,950 | 0,893 | 1 |
| IFI27 SIGLEC1 HERC6 SLC1A2 IL1R2 OLAH | 0,888 | 0,879 | 0,897 | 0,927 | 0,877 | 0,978 | 0,940 | 0,883 | 0,998 |
| IFI27 OAS1 IFIT1 ISG15 SLC1A2 OLAH | 0,888 | 0,879 | 0,897 | 0,927 | 0,875 | 0,978 | 0,932 | 0,865 | 0,998 |
| RSAD2 IFI27 OAS1 IFI44L SIGLEC1 OLAH | 0,888 | 0,880 | 0,896 | 0,918 | 0,861 | 0,975 | 0,950 | 0,900 | 1 |
| RSAD2 IFI27 IFI44L SIGLEC1 OLAH RETN | 0,888 | 0,880 | 0,897 | 0,916 | 0,856 | 0,976 | 0,951 | 0,896 | 1 |
| RSAD2 IFI27 OAS1 SIGLEC1 IL1R2 RETN | 0,888 | 0,880 | 0,896 | 0,919 | 0,865 | 0,973 | 0,955 | 0,901 | 1 |
| IFI27 IFIT1 HERC6 SLC1A2 OLAH MMP8 | 0,888 | 0,879 | 0,897 | 0,928 | 0,878 | 0,979 | 0,935 | 0,873 | 0,997 |
| IFI27 IFIT1 HERC6 SLC1A2 IL1R2 FAM20A | 0,888 | 0,879 | 0,897 | 0,930 | 0,882 | 0,977 | 0,927 | 0,842 | 1 |
| IFI27 IFIT1 IFI44L SLC1A2 IL1R2 FAM20A | 0,888 | 0,879 | 0,897 | 0,924 | 0,874 | 0,975 | 0,927 | 0,843 | 1 |
| IFI27 OAS1 IFIT1 HERC6 IL1R2 MMP8 | 0,888 | 0,880 | 0,896 | 0,917 | 0,868 | 0,966 | 0,937 | 0,881 | 0,993 |
| IFI27 OAS1 IFI44L IL1R2 OLAH MMP8 | 0,888 | 0,880 | 0,896 | 0,922 | 0,870 | 0,974 | 0,941 | 0,886 | 0,996 |
| IFI27 IFIT1 IFI44L SIGLEC1 IL1R2 MMP8 | 0,888 | 0,880 | 0,896 | 0,922 | 0,873 | 0,970 | 0,946 | 0,897 | 0,995 |
| RSAD2 IFI27 OAS1 IFIT1 SIGLEC1 OLAH | 0,888 | 0,880 | 0,896 | 0,919 | 0,864 | 0,975 | 0,940 | 0,884 | 0,997 |
| IFI27 OAS1 SIGLEC1 ISG15 OLAH RETN | 0,888 | 0,879 | 0,897 | 0,917 | 0,859 | 0,975 | 0,948 | 0,890 | 1 |
| RSAD2 IFI27 ISG15 SLC1A2 IL1R2 OLAH | 0,888 | 0,880 | 0,896 | 0,927 | 0,877 | 0,978 | 0,928 | 0,860 | 0,996 |
| IFI27 OAS1 IFIT1 SLC1A2 IL1R2 OLAH | 0,888 | 0,879 | 0,897 | 0,927 | 0,876 | 0,977 | 0,933 | 0,866 | 1,000 |
| IFI27 IFI44L ISG15 IL1R2 OLAH RETN | 0,888 | 0,880 | 0,896 | 0,918 | 0,862 | 0,974 | 0,940 | 0,879 | 1 |
| IFI27 ISG15 HERC6 SLC1A2 OLAH RETN | 0,888 | 0,879 | 0,897 | 0,926 | 0,875 | 0,977 | 0,934 | 0,869 | 0,999 |
| RSAD2 IFI27 IFI44L IL1R2 OLAH MMP8 | 0,888 | 0,880 | 0,896 | 0,922 | 0,870 | 0,974 | 0,945 | 0,892 | 0,998 |
| IFI27 ISG15 SLC1A2 OLAH RETN MMP8 | 0,888 | 0,879 | 0,897 | 0,927 | 0,874 | 0,979 | 0,937 | 0,870 | 1 |
| RSAD2 IFI27 OAS1 SIGLEC1 OLAH RETN | 0,888 | 0,879 | 0,897 | 0,916 | 0,858 | 0,974 | 0,949 | 0,888 | 1 |
| IFI27 OAS1 SLC1A2 IL1R2 OLAH MMP8 | 0,888 | 0,880 | 0,896 | 0,926 | 0,876 | 0,976 | 0,930 | 0,864 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27 HERC6 SLC1A2 IL1R2 OLAH MMP8 | 0,888 | 0,879 | 0,896 | 0,927 | 0,878 | 0,977 | 0,933 | 0,869 | 0,996 |
| IFI27 ISG15 SLC1A2 IL1R2 OLAH RETN | 0,888 | 0,879 | 0,896 | 0,926 | 0,876 | 0,977 | 0,928 | 0,857 | 1,000 |
| IFI27 OAS1 IFI44L OLAH RETN MMP8 | 0,888 | 0,879 | 0,897 | 0,918 | 0,859 | 0,978 | 0,950 | 0,894 | 1 |
| IFI27 OAS1 IFIT1 ISG15 OLAH MMP8 | 0,888 | 0,879 | 0,897 | 0,914 | 0,855 | 0,973 | 0,935 | 0,873 | 0,996 |
| RSAD2 IFI27 SIGLEC1 ISG15 HERC6 IL1R2 | 0,888 | 0,880 | 0,896 | 0,921 | 0,874 | 0,968 | 0,948 | 0,900 | 0,996 |
| RSAD2 SIGLEC1 IL1R2 FAM20A RETN MMP8 | 0,888 | 0,879 | 0,897 | 0,920 | 0,863 | 0,977 | 0,964 | 0,912 | 1 |
| IFI27 IFIT1 HERC6 SLC1A2 OLAH RETN | 0,888 | 0,878 | 0,897 | 0,925 | 0,874 | 0,977 | 0,936 | 0,871 | 1 |
| RSAD2 IFI27 OAS1 IL1R2 OLAH RETN | 0,888 | 0,879 | 0,896 | 0,918 | 0,862 | 0,974 | 0,945 | 0,887 | 1 |
| IFI27 IFIT1 SIGLEC1 HERC6 RETN MMP8 | 0,888 | 0,879 | 0,897 | 0,910 | 0,848 | 0,972 | 0,941 | 0,885 | 0,997 |
| RSAD2 IFI27 SIGLEC1 HERC6 RETN MMP8 | 0,888 | 0,878 | 0,897 | 0,915 | 0,856 | 0,975 | 0,952 | 0,902 | 1 |
| IFI27 OAS1 IFIT1 ISG15 IL1R2 OLAH | 0,888 | 0,879 | 0,896 | 0,919 | 0,865 | 0,972 | 0,941 | 0,885 | 0,998 |
| IFI27 OAS1 SIGLEC1 SLC1A2 FAM20A RETN | 0,888 | 0,878 | 0,897 | 0,922 | 0,864 | 0,979 | 0,939 | 0,862 | 1 |
| IFI27 ISG15 HERC6 SLC1A2 IL1R2 FAM20A | 0,888 | 0,878 | 0,897 | 0,930 | 0,883 | 0,977 | 0,923 | 0,835 | 1 |
| RSAD2 IFI27 IFI44L SIGLEC1 SLC1A2 OLAH | 0,888 | 0,879 | 0,896 | 0,926 | 0,875 | 0,978 | 0,946 | 0,890 | 1 |
| RSAD2 IFI27 IFIT1 IFI44L SIGLEC1 OLAH | 0,888 | 0,879 | 0,896 | 0,919 | 0,862 | 0,975 | 0,949 | 0,898 | 1,000 |
| IFI27 IFIT1 SIGLEC1 ISG15 IL1R2 RETN | 0,888 | 0,880 | 0,896 | 0,920 | 0,867 | 0,973 | 0,957 | 0,912 | 1 |
| IFI27 IFIT1 IFI44L ISG15 OLAH MMP8 | 0,888 | 0,879 | 0,896 | 0,915 | 0,857 | 0,974 | 0,936 | 0,876 | 0,995 |
| RSAD2 IFI27 ISG15 HERC6 IL1R2 RETN | 0,888 | 0,879 | 0,896 | 0,920 | 0,868 | 0,973 | 0,938 | 0,880 | 0,996 |
| IFI27 OAS1 IFIT1 ISG15 OLAH RETN | 0,888 | 0,879 | 0,896 | 0,916 | 0,857 | 0,975 | 0,940 | 0,878 | 1 |
| RSAD2 IFI27 OAS1 IFIT1 HERC6 IL1R2 | 0,888 | 0,880 | 0,896 | 0,917 | 0,868 | 0,966 | 0,941 | 0,888 | 0,995 |
| RSAD2 IFI27 OAS1 ISG15 IL1R2 OLAH | 0,888 | 0,879 | 0,896 | 0,920 | 0,867 | 0,973 | 0,943 | 0,890 | 0,997 |
| SIGLEC1 HERC6 IL1R2 FAM20A RETN MMP8 | 0,888 | 0,878 | 0,897 | 0,918 | 0,859 | 0,976 | 0,960 | 0,898 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFI44L HERC6 SLC1A2 OLAH MMP8 | 0,888 | 0,878 | 0,897 | 0,927 | 0,877 | 0,978 | 0,932 | 0,868 | 0,995 |
| OAS1 IFIT1 SIGLEC1 IL1R2 OLAH FAM20A | 0,888 | 0,879 | 0,896 | 0,914 | 0,852 | 0,977 | 0,963 | 0,914 | 1 |
| IFI27 IFI44L SLC1A2 IL1R2 OLAH RETN | 0,888 | 0,879 | 0,896 | 0,926 | 0,875 | 0,977 | 0,935 | 0,868 | 1 |
| IFI27 IFIT1 ISG15 IL1R2 OLAH RETN | 0,888 | 0,879 | 0,896 | 0,918 | 0,862 | 0,975 | 0,941 | 0,882 | 1 |
| RSAD2 IFI27 SIGLEC1 SLC1A2 OLAH RETN | 0,888 | 0,879 | 0,896 | 0,927 | 0,875 | 0,979 | 0,949 | 0,891 | 1 |
| IFIT1 SIGLEC1 SLC1A2 OLAH FAM20A MMP8 | 0,888 | 0,878 | 0,897 | 0,923 | 0,870 | 0,975 | 0,952 | 0,895 | 1 |
| RSAD2 IFI27 OAS1 SLC1A2 IL1R2 OLAH | 0,888 | 0,879 | 0,896 | 0,926 | 0,876 | 0,976 | 0,930 | 0,862 | 0,999 |
| SIGLEC1 ISG15 IL1R2 FAM20A RETN MMP8 | 0,888 | 0,879 | 0,896 | 0,915 | 0,855 | 0,975 | 0,962 | 0,902 | 1 |
| IFI27 OAS1 IFIT1 IFI44L SIGLEC1 IL1R2 | 0,888 | 0,880 | 0,895 | 0,921 | 0,873 | 0,970 | 0,948 | 0,900 | 0,996 |
| SIGLEC1 IL1R2 OLAH FAM20A RETN MMP8 | 0,887 | 0,878 | 0,897 | 0,918 | 0,860 | 0,977 | 0,963 | 0,909 | 1 |
| RSAD2 IFI27 OAS1 SIGLEC1 ISG15 OLAH | 0,887 | 0,879 | 0,896 | 0,918 | 0,862 | 0,974 | 0,941 | 0,884 | 0,999 |
| SIGLEC1 ISG15 SLC1A2 IL1R2 FAM20A MMP8 | 0,887 | 0,879 | 0,896 | 0,924 | 0,877 | 0,972 | 0,958 | 0,890 | 1 |
| IFI27 IFIT1 SLC1A2 OLAH RETN MMP8 | 0,887 | 0,878 | 0,897 | 0,924 | 0,869 | 0,979 | 0,947 | 0,887 | 1 |
| IFIT1 SIGLEC1 HERC6 OLAH FAM20A RETN | 0,887 | 0,878 | 0,897 | 0,915 | 0,854 | 0,976 | 0,946 | 0,886 | 1 |
| IFI27 SIGLEC1 ISG15 HERC6 IL1R2 RETN | 0,887 | 0,879 | 0,896 | 0,923 | 0,870 | 0,975 | 0,952 | 0,905 | 1,000 |
| IFI27 OAS1 IFI44L SIGLEC1 IL1R2 MMP8 | 0,887 | 0,880 | 0,895 | 0,919 | 0,870 | 0,968 | 0,943 | 0,894 | 0,993 |
| SIGLEC1 HERC6 SLC1A2 IL1R2 FAM20A MMP8 | 0,887 | 0,878 | 0,897 | 0,923 | 0,873 | 0,973 | 0,959 | 0,893 | 1 |
| OAS1 SIGLEC1 IL1R2 FAM20A RETN MMP8 | 0,887 | 0,878 | 0,896 | 0,918 | 0,859 | 0,976 | 0,964 | 0,912 | 1 |
| RSAD2 IFI27 IFIT1 ISG15 SLC1A2 OLAH | 0,887 | 0,879 | 0,896 | 0,926 | 0,874 | 0,978 | 0,928 | 0,861 | 0,996 |
| RSAD2 IFI27 IFIT1 SLC1A2 IL1R2 OLAH | 0,887 | 0,879 | 0,896 | 0,927 | 0,877 | 0,977 | 0,933 | 0,868 | 0,998 |
| RSAD2 IFI27 OAS1 IFIT1 OLAH MMP8 | 0,887 | 0,879 | 0,896 | 0,915 | 0,857 | 0,973 | 0,938 | 0,879 | 0,997 |
| RSAD2 IFI27 OAS1 ISG15 OLAH MMP8 | 0,887 | 0,879 | 0,896 | 0,916 | 0,858 | 0,974 | 0,936 | 0,877 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 SIGLEC1 HERC6 OLAH FAM20A | 0,887 | 0,878 | 0,897 | 0,912 | 0,851 | 0,973 | 0,951 | 0,896 | 1 |
| IFI27 IFI44L ISG15 HERC6 SLC1A2 OLAH | 0,887 | 0,878 | 0,896 | 0,928 | 0,878 | 0,978 | 0,924 | 0,855 | 0,993 |
| OAS1 SIGLEC1 SLC1A2 IL1R2 FAM20A MMP8 | 0,887 | 0,878 | 0,896 | 0,923 | 0,874 | 0,971 | 0,961 | 0,899 | 1 |
| IFI27 IFIT1 IFI44L ISG15 SLC1A2 OLAH | 0,887 | 0,879 | 0,896 | 0,926 | 0,874 | 0,978 | 0,927 | 0,858 | 0,996 |
| IFI27 IFIT1 IFI44L ISG15 IL1R2 OLAH | 0,887 | 0,879 | 0,895 | 0,918 | 0,864 | 0,973 | 0,938 | 0,881 | 0,995 |
| IFI27 IFIT1 ISG15 SLC1A2 OLAH RETN | 0,887 | 0,879 | 0,896 | 0,928 | 0,877 | 0,979 | 0,934 | 0,866 | 1 |
| RSAD2 IFI27 OAS1 HERC6 RETN MMP8 | 0,887 | 0,878 | 0,897 | 0,915 | 0,853 | 0,977 | 0,934 | 0,862 | 1 |
| IFI27 IFIT1 SIGLEC1 ISG15 HERC6 IL1R2 | 0,887 | 0,880 | 0,895 | 0,922 | 0,875 | 0,969 | 0,950 | 0,903 | 0,997 |
| IFI27 OAS1 IFIT1 SIGLEC1 IL1R2 MMP8 | 0,887 | 0,879 | 0,895 | 0,919 | 0,870 | 0,967 | 0,952 | 0,906 | 0,998 |
| IFI27 SIGLEC1 ISG15 HERC6 IL1R2 MMP8 | 0,887 | 0,880 | 0,895 | 0,920 | 0,872 | 0,968 | 0,949 | 0,902 | 0,996 |
| IFI27 SIGLEC1 SLC1A2 IL1R2 OLAH RETN | 0,887 | 0,878 | 0,896 | 0,926 | 0,874 | 0,977 | 0,949 | 0,894 | 1 |
| IFI27 OAS1 IFIT1 IFI44L IL1R2 OLAH | 0,887 | 0,879 | 0,895 | 0,921 | 0,868 | 0,975 | 0,940 | 0,885 | 0,995 |
| IFI27 ISG15 HERC6 IL1R2 RETN MMP8 | 0,887 | 0,879 | 0,896 | 0,921 | 0,867 | 0,975 | 0,946 | 0,892 | 0,999 |
| IFI27 OAS1 SLC1A2 IL1R2 FAM20A MMP8 | 0,887 | 0,879 | 0,896 | 0,927 | 0,878 | 0,976 | 0,927 | 0,841 | 1 |
| RSAD2 IFI27 IFI44L SIGLEC1 IL1R2 RETN | 0,887 | 0,879 | 0,895 | 0,919 | 0,864 | 0,974 | 0,950 | 0,900 | 1 |
| RSAD2 IFI27 ISG15 OLAH RETN MMP8 | 0,887 | 0,878 | 0,896 | 0,918 | 0,858 | 0,978 | 0,949 | 0,892 | 1 |
| RSAD2 IFI27 IFIT1 SIGLEC1 ISG15 OLAH | 0,887 | 0,879 | 0,895 | 0,918 | 0,862 | 0,974 | 0,941 | 0,885 | 0,998 |
| IFI27 IFI44L SIGLEC1 SLC1A2 IL1R2 RETN | 0,887 | 0,879 | 0,895 | 0,923 | 0,874 | 0,973 | 0,949 | 0,896 | 1 |
| IFI27 OAS1 IFIT1 IFI44L OLAH RETN | 0,887 | 0,878 | 0,896 | 0,914 | 0,855 | 0,974 | 0,945 | 0,884 | 1 |
| IFI27 IFI44L HERC6 SLC1A2 OLAH RETN | 0,887 | 0,878 | 0,896 | 0,924 | 0,871 | 0,977 | 0,936 | 0,871 | 1 |
| IFI27 OAS1 IFI44L HERC6 SLC1A2 OLAH | 0,887 | 0,878 | 0,897 | 0,929 | 0,880 | 0,978 | 0,929 | 0,861 | 0,997 |
| IFI27 IFI44L SIGLEC1 ISG15 HERC6 RETN | 0,887 | 0,878 | 0,896 | 0,911 | 0,849 | 0,973 | 0,942 | 0,885 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 OAS1 IFIT1 SLC1A2 OLAH | 0,887 | 0,878 | 0,896 | 0,926 | 0,875 | 0,978 | 0,940 | 0,877 | 1 |
| IFI27 SLC1A2 IL1R2 FAM20A RETN MMP8 | 0,887 | 0,878 | 0,896 | 0,925 | 0,877 | 0,974 | 0,909 | 0,814 | 1 |
| IFI27 ISG15 SLC1A2 IL1R2 FAM20A MMP8 | 0,887 | 0,879 | 0,895 | 0,926 | 0,877 | 0,975 | 0,916 | 0,825 | 1 |
| RSAD2 IFI27 IFI44L SLC1A2 IL1R2 FAM20A | 0,887 | 0,878 | 0,896 | 0,927 | 0,878 | 0,976 | 0,928 | 0,844 | 1 |
| IFI27 IFI44L ISG15 SLC1A2 OLAH RETN | 0,887 | 0,878 | 0,896 | 0,929 | 0,878 | 0,979 | 0,933 | 0,865 | 1 |
| RSAD2 IFI27 OAS1 HERC6 IL1R2 MMP8 | 0,887 | 0,879 | 0,895 | 0,919 | 0,871 | 0,967 | 0,933 | 0,875 | 0,990 |
| IFI27 OAS1 SIGLEC1 HERC6 SLC1A2 IL1R2 | 0,887 | 0,879 | 0,895 | 0,927 | 0,882 | 0,971 | 0,949 | 0,899 | 0,999 |
| IFI27 SIGLEC1 HERC6 SLC1A2 OLAH RETN | 0,887 | 0,877 | 0,896 | 0,926 | 0,873 | 0,978 | 0,946 | 0,888 | 1 |
| RSAD2 IFI27 IFI44L SLC1A2 OLAH MMP8 | 0,887 | 0,878 | 0,896 | 0,927 | 0,875 | 0,978 | 0,932 | 0,866 | 0,997 |
| IFI27 OAS1 SLC1A2 OLAH RETN MMP8 | 0,887 | 0,878 | 0,896 | 0,924 | 0,870 | 0,978 | 0,941 | 0,878 | 1 |
| IFI27 IFI44L SIGLEC1 ISG15 HERC6 FAM20A | 0,887 | 0,878 | 0,896 | 0,913 | 0,850 | 0,976 | 0,943 | 0,866 | 1 |
| IFI27 OAS1 SIGLEC1 ISG15 IL1R2 RETN | 0,887 | 0,879 | 0,895 | 0,921 | 0,868 | 0,974 | 0,954 | 0,904 | 1 |
| RSAD2 IFI27 SLC1A2 OLAH RETN MMP8 | 0,887 | 0,878 | 0,896 | 0,925 | 0,871 | 0,979 | 0,942 | 0,879 | 1 |
| IFI27 IFIT1 IFI44L SLC1A2 OLAH MMP8 | 0,887 | 0,878 | 0,896 | 0,926 | 0,874 | 0,978 | 0,936 | 0,873 | 0,999 |
| RSAD2 IFI27 OAS1 SIGLEC1 ISG15 FAM20A | 0,887 | 0,877 | 0,897 | 0,915 | 0,851 | 0,979 | 0,947 | 0,876 | 1 |
| IFI27 OAS1 IFI44L ISG15 OLAH MMP8 | 0,887 | 0,878 | 0,895 | 0,917 | 0,859 | 0,975 | 0,934 | 0,874 | 0,994 |
| OAS1 IFIT1 SIGLEC1 HERC6 FAM20A RETN | 0,887 | 0,878 | 0,896 | 0,914 | 0,853 | 0,975 | 0,940 | 0,873 | 1 |
| IFIT1 IFI44L SIGLEC1 OLAH FAM20A MMP8 | 0,887 | 0,877 | 0,896 | 0,908 | 0,843 | 0,974 | 0,954 | 0,904 | 1 |
| RSAD2 IFI27 IFI44L SIGLEC1 IL1R2 MMP8 | 0,887 | 0,879 | 0,895 | 0,919 | 0,869 | 0,970 | 0,943 | 0,893 | 0,994 |
| IFI27 OAS1 IFI44L SLC1A2 IL1R2 FAM20A | 0,887 | 0,878 | 0,896 | 0,929 | 0,881 | 0,976 | 0,927 | 0,843 | 1 |
| IFI27 IFIT1 HERC6 SLC1A2 IL1R2 OLAH | 0,887 | 0,878 | 0,896 | 0,926 | 0,876 | 0,976 | 0,934 | 0,870 | 0,997 |
| IFI27 OAS1 IFIT1 IFI44L HERC6 IL1R2 | 0,887 | 0,879 | 0,895 | 0,922 | 0,873 | 0,970 | 0,938 | 0,883 | 0,993 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 SLC1A2 IL1R2 OLAH RETN | 0,887 | 0,878 | 0,896 | 0,923 | 0,871 | 0,975 | 0,934 | 0,866 | 1 |
| RSAD2 IFI27 OAS1 IFI44L SIGLEC1 FAM20A | 0,887 | 0,877 | 0,896 | 0,914 | 0,850 | 0,979 | 0,949 | 0,879 | 1 |
| IFI27 OAS1 IFIT1 IFI44L OLAH MMP8 | 0,887 | 0,878 | 0,895 | 0,916 | 0,858 | 0,974 | 0,941 | 0,884 | 0,999 |
| RSAD2 IFI27 IFI44L ISG15 SLC1A2 OLAH | 0,887 | 0,878 | 0,895 | 0,927 | 0,875 | 0,979 | 0,928 | 0,860 | 0,996 |
| IFI27 OAS1 SIGLEC1 SLC1A2 IL1R2 RETN | 0,887 | 0,878 | 0,895 | 0,924 | 0,876 | 0,973 | 0,952 | 0,897 | 1 |
| IFI27 IFIT1 IFI44L IL1R2 OLAH RETN | 0,887 | 0,878 | 0,895 | 0,916 | 0,860 | 0,973 | 0,942 | 0,883 | 1 |
| IFI27 IFIT1 IFI44L SIGLEC1 SLC1A2 FAM20A | 0,887 | 0,878 | 0,896 | 0,921 | 0,863 | 0,978 | 0,941 | 0,865 | 1 |
| RSAD2 IFI27 IFI44L ISG15 IL1R2 OLAH | 0,887 | 0,879 | 0,895 | 0,922 | 0,869 | 0,975 | 0,941 | 0,885 | 0,997 |
| IFI27 IFIT1 SLC1A2 IL1R2 OLAH RETN | 0,887 | 0,878 | 0,895 | 0,923 | 0,870 | 0,975 | 0,934 | 0,866 | 1 |
| OAS1 IFIT1 IFI44L SIGLEC1 IL1R2 FAM20A | 0,887 | 0,878 | 0,896 | 0,913 | 0,850 | 0,976 | 0,959 | 0,907 | 1 |
| IFI27 OAS1 ISG15 IL1R2 OLAH RETN | 0,887 | 0,878 | 0,895 | 0,919 | 0,863 | 0,974 | 0,939 | 0,879 | 0,999 |
| IFI27 HERC6 SLC1A2 IL1R2 OLAH RETN | 0,887 | 0,878 | 0,896 | 0,924 | 0,871 | 0,976 | 0,938 | 0,874 | 1 |
| IFI27 OAS1 IFIT1 SLC1A2 IL1R2 FAM20A | 0,887 | 0,878 | 0,896 | 0,925 | 0,875 | 0,975 | 0,925 | 0,839 | 1 |
| RSAD2 IFI27 OAS1 IFIT1 ISG15 OLAH | 0,887 | 0,878 | 0,895 | 0,915 | 0,856 | 0,973 | 0,934 | 0,872 | 0,995 |
| RSAD2 IFI27 IFIT1 SLC1A2 IL1R2 FAM20A | 0,887 | 0,878 | 0,895 | 0,924 | 0,873 | 0,975 | 0,927 | 0,842 | 1 |
| RSAD2 IFI27 SIGLEC1 ISG15 OLAH RETN | 0,887 | 0,878 | 0,895 | 0,917 | 0,860 | 0,975 | 0,947 | 0,889 | 1 |
| IFI27 OAS1 IFI44L ISG15 IL1R2 OLAH | 0,887 | 0,878 | 0,895 | 0,923 | 0,871 | 0,975 | 0,936 | 0,878 | 0,994 |
| RSAD2 IFI27 IFIT1 ISG15 IL1R2 OLAH | 0,887 | 0,879 | 0,895 | 0,919 | 0,865 | 0,972 | 0,941 | 0,885 | 0,998 |
| IFI27 IFIT1 ISG15 HERC6 IL1R2 RETN | 0,887 | 0,878 | 0,895 | 0,919 | 0,865 | 0,973 | 0,940 | 0,884 | 0,997 |
| RSAD2 IFI27 IFIT1 SIGLEC1 FAM20A MMP8 | 0,887 | 0,877 | 0,896 | 0,913 | 0,848 | 0,978 | 0,946 | 0,879 | 1 |
| RSAD2 IFI27 OAS1 IFI44L SIGLEC1 IL1R2 | 0,887 | 0,879 | 0,894 | 0,921 | 0,871 | 0,971 | 0,951 | 0,905 | 0,997 |
| IFI27 IFIT1 IFI44L ISG15 OLAH RETN | 0,887 | 0,878 | 0,895 | 0,915 | 0,856 | 0,975 | 0,941 | 0,879 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 IL1R2 RETN MMP8 | 0,887 | 0,878 | 0,895 | 0,918 | 0,863 | 0,974 | 0,954 | 0,904 | 1 |
| IFI27 OAS1 IFI44L HERC6 IL1R2 MMP8 | 0,887 | 0,879 | 0,894 | 0,916 | 0,867 | 0,965 | 0,930 | 0,872 | 0,989 |
| IFI27 IFIT1 IFI44L IL1R2 RETN MMP8 | 0,886 | 0,878 | 0,895 | 0,918 | 0,863 | 0,974 | 0,946 | 0,891 | 1 |
| RSAD2 IFI27 IFIT1 SIGLEC1 IL1R2 RETN | 0,886 | 0,878 | 0,895 | 0,920 | 0,866 | 0,973 | 0,959 | 0,915 | 1 |
| IFI27 OAS1 IFI44L HERC6 RETN MMP8 | 0,886 | 0,877 | 0,896 | 0,914 | 0,853 | 0,976 | 0,937 | 0,870 | 1 |
| RSAD2 IFI27 IFI44L SIGLEC1 ISG15 FAM20A | 0,886 | 0,877 | 0,896 | 0,914 | 0,849 | 0,978 | 0,945 | 0,872 | 1 |
| RSAD2 IFI27 IFI44L OLAH RETN MMP8 | 0,886 | 0,877 | 0,895 | 0,918 | 0,859 | 0,978 | 0,951 | 0,894 | 1 |
| RSAD2 IFI27 OAS1 IFI44L IL1R2 OLAH | 0,886 | 0,878 | 0,895 | 0,921 | 0,868 | 0,974 | 0,941 | 0,886 | 0,997 |
| IFI27 OAS1 IFIT1 HERC6 RETN MMP8 | 0,886 | 0,877 | 0,896 | 0,916 | 0,855 | 0,976 | 0,932 | 0,860 | 1 |
| IFI27 OAS1 ISG15 SLC1A2 IL1R2 OLAH | 0,886 | 0,878 | 0,895 | 0,926 | 0,875 | 0,977 | 0,920 | 0,848 | 0,991 |
| RSAD2 IFI27 IFIT1 IL1R2 OLAH RETN | 0,886 | 0,878 | 0,895 | 0,918 | 0,861 | 0,974 | 0,943 | 0,885 | 1 |
| RSAD2 IFI27 OAS1 ISG15 SLC1A2 OLAH | 0,886 | 0,878 | 0,895 | 0,926 | 0,874 | 0,979 | 0,930 | 0,862 | 0,999 |
| RSAD2 IFI27 OAS1 IFIT1 OLAH RETN | 0,886 | 0,878 | 0,895 | 0,914 | 0,853 | 0,974 | 0,947 | 0,887 | 1 |
| IFIT1 IFI44L SIGLEC1 ISG15 HERC6 FAM20A | 0,886 | 0,878 | 0,895 | 0,914 | 0,853 | 0,975 | 0,937 | 0,865 | 1 |
| IFI27 OAS1 SLC1A2 IL1R2 OLAH RETN | 0,886 | 0,878 | 0,895 | 0,926 | 0,874 | 0,977 | 0,933 | 0,865 | 1 |
| RSAD2 IFI27 OAS1 IFI44L HERC6 IL1R2 | 0,886 | 0,878 | 0,894 | 0,919 | 0,871 | 0,968 | 0,934 | 0,876 | 0,992 |
| IFI27 OAS1 HERC6 SLC1A2 IL1R2 RETN | 0,886 | 0,878 | 0,895 | 0,924 | 0,876 | 0,973 | 0,928 | 0,858 | 0,999 |
| IFI27 OAS1 IFI44L IL1R2 OLAH RETN | 0,886 | 0,878 | 0,895 | 0,918 | 0,862 | 0,974 | 0,941 | 0,883 | 1,000 |
| RSAD2 IFI27 IFI44L HERC6 SLC1A2 OLAH | 0,886 | 0,877 | 0,895 | 0,927 | 0,877 | 0,978 | 0,928 | 0,862 | 0,994 |
| RSAD2 IFI27 ISG15 IL1R2 OLAH RETN | 0,886 | 0,878 | 0,895 | 0,919 | 0,862 | 0,975 | 0,940 | 0,881 | 1,000 |
| RSAD2 IFI27 IFIT1 IFI44L IL1R2 OLAH | 0,886 | 0,878 | 0,894 | 0,921 | 0,867 | 0,974 | 0,940 | 0,885 | 0,995 |
| IFI27 OAS1 IFIT1 SLC1A2 OLAH RETN | 0,886 | 0,877 | 0,895 | 0,926 | 0,874 | 0,978 | 0,939 | 0,871 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFI44L HERC6 SLC1A2 IL1R2 OLAH | 0,886 | 0,877 | 0,896 | 0,926 | 0,877 | 0,976 | 0,930 | 0,866 | 0,995 |
| IFI44L SIGLEC1 ISG15 HERC6 IL1R2 FAM20A | 0,886 | 0,877 | 0,895 | 0,914 | 0,853 | 0,975 | 0,954 | 0,892 | 1 |
| IFI27 OAS1 IFI44L SLC1A2 OLAH MMP8 | 0,886 | 0,877 | 0,895 | 0,927 | 0,875 | 0,978 | 0,935 | 0,871 | 0,999 |
| IFI27 OAS1 IFI44L SIGLEC1 ISG15 FAM20A | 0,886 | 0,877 | 0,895 | 0,912 | 0,848 | 0,976 | 0,945 | 0,873 | 1 |
| IFIT1 SIGLEC1 HERC6 SLC1A2 IL1R2 FAM20A | 0,886 | 0,877 | 0,895 | 0,924 | 0,875 | 0,973 | 0,946 | 0,874 | 1 |
| RSAD2 IFI27 IFIT1 HERC6 IL1R2 MMP8 | 0,886 | 0,878 | 0,894 | 0,916 | 0,866 | 0,966 | 0,937 | 0,881 | 0,993 |
| RSAD2 IFI27 IFIT1 IFI44L SIGLEC1 IL1R2 | 0,886 | 0,878 | 0,894 | 0,919 | 0,868 | 0,969 | 0,946 | 0,897 | 0,995 |
| IFI27 OAS1 ISG15 HERC6 IL1R2 MMP8 | 0,886 | 0,878 | 0,894 | 0,918 | 0,870 | 0,967 | 0,928 | 0,868 | 0,988 |
| RSAD2 IFI27 OAS1 SIGLEC1 IL1R2 MMP8 | 0,886 | 0,877 | 0,894 | 0,919 | 0,871 | 0,968 | 0,957 | 0,912 | 1 |
| IFI27 OAS1 IFIT1 IFI44L ISG15 OLAH | 0,886 | 0,877 | 0,894 | 0,915 | 0,856 | 0,974 | 0,935 | 0,875 | 0,994 |
| IFIT1 SIGLEC1 HERC6 SLC1A2 OLAH FAM20A | 0,886 | 0,876 | 0,896 | 0,921 | 0,868 | 0,974 | 0,937 | 0,864 | 1 |
| RSAD2 IFI27 SLC1A2 IL1R2 FAM20A RETN | 0,886 | 0,877 | 0,895 | 0,929 | 0,880 | 0,978 | 0,925 | 0,838 | 1 |
| RSAD2 IFI27 IFI44L HERC6 IL1R2 MMP8 | 0,886 | 0,878 | 0,894 | 0,915 | 0,865 | 0,966 | 0,936 | 0,880 | 0,992 |
| RSAD2 IFI27 OAS1 IFIT1 SIGLEC1 IL1R2 | 0,886 | 0,878 | 0,894 | 0,920 | 0,871 | 0,968 | 0,958 | 0,914 | 1 |
| RSAD2 IFI27 IFIT1 ISG15 OLAH MMP8 | 0,886 | 0,877 | 0,895 | 0,915 | 0,856 | 0,973 | 0,935 | 0,873 | 0,996 |
| RSAD2 IFI27 OAS1 IFI44L OLAH MMP8 | 0,886 | 0,877 | 0,894 | 0,918 | 0,860 | 0,975 | 0,938 | 0,879 | 0,997 |
| OAS1 IFIT1 SIGLEC1 OLAH FAM20A MMP8 | 0,886 | 0,876 | 0,895 | 0,912 | 0,848 | 0,977 | 0,960 | 0,911 | 1 |
| IFI27 IFIT1 IFI44L HERC6 SLC1A2 OLAH | 0,886 | 0,877 | 0,895 | 0,928 | 0,877 | 0,978 | 0,934 | 0,872 | 0,995 |
| IFI27 OAS1 IFI44L ISG15 SLC1A2 OLAH | 0,886 | 0,877 | 0,895 | 0,927 | 0,876 | 0,979 | 0,924 | 0,853 | 0,995 |
| RSAD2 IFI27 IFI44L ISG15 OLAH MMP8 | 0,886 | 0,877 | 0,894 | 0,916 | 0,858 | 0,974 | 0,937 | 0,878 | 0,996 |
| IFI27 IFIT1 IFI44L HERC6 IL1R2 MMP8 | 0,886 | 0,878 | 0,894 | 0,918 | 0,868 | 0,969 | 0,936 | 0,880 | 0,992 |
| RSAD2 IFI27 OAS1 SLC1A2 OLAH RETN | 0,886 | 0,877 | 0,895 | 0,927 | 0,875 | 0,979 | 0,937 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 SIGLEC1 ISG15 IL1R2 RETN | 0,886 | 0,877 | 0,894 | 0,922 | 0,869 | 0,974 | 0,954 | 0,908 | 1 |
| IFI27 IFIT1 SIGLEC1 ISG15 ILIR2 MMP8 | 0,886 | 0,878 | 0,893 | 0,919 | 0,870 | 0,967 | 0,948 | 0,899 | 0,996 |
| RSAD2 IFIT1 SIGLEC1 IL1R2 OLAH FAM20A | 0,886 | 0,877 | 0,894 | 0,910 | 0,846 | 0,974 | 0,963 | 0,914 | 1 |
| IFI27 IFIT1 SIGLEC1 SLC1A2 IL1R2 RETN | 0,886 | 0,877 | 0,894 | 0,926 | 0,877 | 0,974 | 0,953 | 0,903 | 1 |
| RSAD2 IFI27 OAS1 IFI44L ISG15 OLAH | 0,886 | 0,877 | 0,894 | 0,917 | 0,859 | 0,975 | 0,935 | 0,876 | 0,994 |
| IFIT1 IFI44L SIGLEC1 IL1R2 OLAH FAM20A | 0,886 | 0,877 | 0,894 | 0,909 | 0,845 | 0,973 | 0,957 | 0,905 | 1 |
| IFI27 IFIT1 SLC1A2 IL1R2 FAM20A RETN | 0,886 | 0,877 | 0,895 | 0,927 | 0,877 | 0,977 | 0,929 | 0,847 | 1 |
| RSAD2 IFI27 OAS1 SIGLEC1 FAM20A MMP8 | 0,886 | 0,876 | 0,896 | 0,913 | 0,849 | 0,978 | 0,948 | 0,878 | 1 |
| RSAD2 IFI27 ISG15 SLC1A2 OLAH RETN | 0,886 | 0,877 | 0,894 | 0,928 | 0,877 | 0,979 | 0,933 | 0,866 | 1,000 |
| RSAD2 IFI27 OAS1 ISG15 OLAH RETN | 0,886 | 0,877 | 0,894 | 0,916 | 0,856 | 0,975 | 0,940 | 0,877 | 1 |
| RSAD2 IFI27 ISG15 SLC1A2 IL1R2 FAM20A | 0,886 | 0,877 | 0,894 | 0,927 | 0,879 | 0,975 | 0,925 | 0,839 | 1 |
| SIGLEC1 SLC1A2 IL1R2 FAM20A RETN MMP8 | 0,886 | 0,876 | 0,895 | 0,922 | 0,872 | 0,973 | 0,957 | 0,887 | 1 |
| IFI27 OAS1 ISG15 SLC1A2 OLAH RETN | 0,886 | 0,877 | 0,894 | 0,928 | 0,877 | 0,979 | 0,929 | 0,859 | 0,999 |
| IFI27 IFIT1 SIGLEC1 ISG15 SLC1A2 FAM20A | 0,886 | 0,877 | 0,895 | 0,922 | 0,866 | 0,977 | 0,942 | 0,867 | 1 |
| IFI27 IFI44L SIGLEC1 ISG15 SLC1A2 IL1R2 | 0,885 | 0,878 | 0,893 | 0,924 | 0,879 | 0,970 | 0,946 | 0,895 | 0,996 |
| RSAD2 IFI27 SIGLEC1 SLC1A2 FAM20A RETN | 0,885 | 0,876 | 0,895 | 0,923 | 0,866 | 0,979 | 0,945 | 0,871 | 1 |
| RSAD2 IFI27 IFIT1 IFI44L HERC6 IL1R2 | 0,885 | 0,878 | 0,893 | 0,918 | 0,868 | 0,969 | 0,938 | 0,883 | 0,993 |
| OAS1 IFIT1 SIGLEC1 ISG15 IL1R2 FAM20A | 0,885 | 0,876 | 0,894 | 0,913 | 0,850 | 0,976 | 0,961 | 0,910 | 1 |
| RSAD2 IFI27 IFIT1 ISG15 OLAH RETN | 0,885 | 0,877 | 0,894 | 0,916 | 0,857 | 0,975 | 0,940 | 0,878 | 1 |
| IFI27 OAS1 IFIT1 IFI44L SLC1A2 OLAH | 0,885 | 0,877 | 0,894 | 0,927 | 0,875 | 0,978 | 0,938 | 0,876 | 1 |
| RSAD2 OAS1 SIGLEC1 HERC6 IL1R2 FAM20A | 0,885 | 0,876 | 0,895 | 0,914 | 0,855 | 0,973 | 0,955 | 0,899 | 1 |
| IFI27 OAS1 IFIT1 IFI44L IL1R2 RETN | 0,885 | 0,877 | 0,894 | 0,918 | 0,865 | 0,972 | 0,941 | 0,883 | 0,999 |

(suite)

| Modèle | AUC modèle | IC 95% | | AUC Train | IC 95% Train | | AUC Test | IC 95% Test | |
|---|---|---|---|---|---|---|---|---|---|
| IFI27 IFI44L ISG15 IL1R2 RETN MMP8 | 0,885 | 0,877 | 0,894 | 0,922 | 0,869 | 0,974 | 0,940 | 0,880 | 1 |
| RSAD2 IFI27 IFIT1 IFI44L OLAH MMP8 | 0,885 | 0,877 | 0,894 | 0,915 | 0,856 | 0,973 | 0,941 | 0,884 | 0,999 |
| RSAD2 IFI27 OAS1 IFIT1 IL1R2 RETN | 0,885 | 0,877 | 0,894 | 0,916 | 0,862 | 0,971 | 0,950 | 0,897 | 1 |
| IFI27 IFIT1 IFI44L SLC1A2 OLAH RETN | 0,885 | 0,876 | 0,894 | 0,923 | 0,871 | 0,976 | 0,939 | 0,875 | 1 |
| IFI27 OAS1 IFIT1 SIGLEC1 ISG15 IL1R2 | 0,885 | 0,877 | 0,893 | 0,920 | 0,871 | 0,969 | 0,952 | 0,906 | 0,998 |
| IFI27 IFIT1 ISG15 SLC1A2 IL1R2 FAM20A | 0,885 | 0,876 | 0,894 | 0,923 | 0,873 | 0,974 | 0,925 | 0,840 | 1 |
| RSAD2 IFI27 OAS1 IFIT1 IFI44L OLAH | 0,885 | 0,877 | 0,894 | 0,916 | 0,858 | 0,974 | 0,939 | 0,881 | 0,998 |
| IFI27 OAS1 IFI44L SIGLEC1 ISG15 IL1R2 | 0,885 | 0,878 | 0,893 | 0,918 | 0,868 | 0,968 | 0,939 | 0,886 | 0,992 |
| IFI27 IFIT1 IFI44L SLC1A2 IL1R2 OLAH | 0,885 | 0,877 | 0,894 | 0,925 | 0,874 | 0,976 | 0,933 | 0,868 | 0,997 |
| RSAD2 IFI27 SIGLEC1 ISG15 FAM20A MMP8 | 0,885 | 0,876 | 0,895 | 0,915 | 0,851 | 0,979 | 0,946 | 0,875 | 1 |
| RSAD2 IFI27 SIGLEC1 SLC1A2 IL1R2 RETN | 0,885 | 0,877 | 0,894 | 0,923 | 0,874 | 0,973 | 0,953 | 0,903 | 1 |
| RSAD2 IFI27 OAS1 IFI44L SLC1A2 OLAH | 0,885 | 0,876 | 0,894 | 0,924 | 0,872 | 0,977 | 0,937 | 0,872 | 1 |
| IFI27 OAS1 IFIT1 ISG15 HERC6 IL1R2 | 0,885 | 0,877 | 0,893 | 0,918 | 0,869 | 0,966 | 0,939 | 0,884 | 0,995 |
| IFI27 IFI44L SIGLEC1 HERC6 RETN MMP8 | 0,885 | 0,876 | 0,895 | 0,912 | 0,851 | 0,974 | 0,942 | 0,885 | 1,000 |
| IFI27 IFI44L SIGLEC1 ISG15 FAM20A MMP8 | 0,885 | 0,876 | 0,894 | 0,910 | 0,844 | 0,976 | 0,943 | 0,869 | 1 |
| RSAD2 IFI27 OAS1 SLC1A2 IL1R2 FAM20A | 0,885 | 0,876 | 0,894 | 0,926 | 0,877 | 0,974 | 0,927 | 0,841 | 1 |
| RSAD2 IFIT1 SIGLEC1 HERC6 FAM20A RETN | 0,885 | 0,876 | 0,894 | 0,914 | 0,852 | 0,975 | 0,941 | 0,875 | 1 |
| RSAD2 IFI27 IFI44L SIGLEC1 ISG15 IL1R2 | 0,885 | 0,877 | 0,893 | 0,918 | 0,866 | 0,969 | 0,942 | 0,892 | 0,993 |
| IFI27 IFI44L ISG15 HERC6 IL1R2 MMP8 | 0,885 | 0,877 | 0,893 | 0,918 | 0,869 | 0,966 | 0,929 | 0,870 | 0,988 |
| IFI44L SIGLEC1 HERC6 IL1R2 OLAH FAM20A | 0,885 | 0,876 | 0,894 | 0,914 | 0,853 | 0,975 | 0,957 | 0,896 | 1 |
| RSAD2 IFI27 IFIT1 SLC1A2 OLAH RETN | 0,885 | 0,876 | 0,894 | 0,924 | 0,872 | 0,977 | 0,940 | 0,877 | 1 |
| RSAD2 IFI27 IFIT1 SIGLEC1 IL1R2 MMP8 | 0,885 | 0,877 | 0,893 | 0,920 | 0,872 | 0,968 | 0,950 | 0,903 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 IFI44L ISG15 IL1R2 RETN | 0,885 | 0,877 | 0,893 | 0,919 | 0,866 | 0,973 | 0,940 | 0,882 | 0,999 |
| IFI27 OAS1 SIGLEC1 ISG15 IL1R2 MMP8 | 0,885 | 0,877 | 0,893 | 0,919 | 0,871 | 0,967 | 0,949 | 0,901 | 0,997 |
| RSAD2 IFI27 IFI44L IL1R2 OLAH RETN | 0,885 | 0,876 | 0,894 | 0,918 | 0,861 | 0,975 | 0,942 | 0,883 | 1 |
| IFIT1 IFI44L SIGLEC1 SLC1A2 OLAH FAM20A | 0,885 | 0,876 | 0,894 | 0,920 | 0,864 | 0,975 | 0,934 | 0,868 | 1,000 |
| RSAD2 IFI27 OAS1 IFI44L OLAH RETN | 0,885 | 0,876 | 0,894 | 0,915 | 0,856 | 0,975 | 0,943 | 0,882 | 1 |
| RSAD2 IFI27 IFIT1 IL1R2 RETN MMP8 | 0,885 | 0,876 | 0,894 | 0,920 | 0,865 | 0,974 | 0,950 | 0,899 | 1 |
| IFI27 IFI44L SIGLEC1 SLC1A2 IL1R2 MMP8 | 0,885 | 0,877 | 0,893 | 0,925 | 0,879 | 0,970 | 0,948 | 0,900 | 0,996 |
| IFIT1 SIGLEC1 ISG15 HERC6 FAM20A MMP8 | 0,885 | 0,876 | 0,894 | 0,911 | 0,849 | 0,973 | 0,938 | 0,865 | 1 |
| IFI27 OAS1 ISG15 SLC1A2 ILIR2 FAM20A | 0,885 | 0,876 | 0,894 | 0,928 | 0,880 | 0,976 | 0,924 | 0,837 | 1 |
| RSAD2 IFI27 IFI44L SLC1A2 IL1R2 OLAH | 0,885 | 0,876 | 0,894 | 0,926 | 0,876 | 0,976 | 0,933 | 0,867 | 0,998 |
| IFI27 OAS1 HERC6 SLC1A2 FAM20A RETN | 0,885 | 0,875 | 0,894 | 0,923 | 0,868 | 0,979 | 0,932 | 0,849 | 1 |
| IFI27 IFI44L SIGLEC1 HERC6 FAM20A MMP8 | 0,885 | 0,875 | 0,894 | 0,912 | 0,849 | 0,974 | 0,941 | 0,862 | 1 |
| IFI27 SIGLEC1 ISG15 SLC1A2 IL1R2 RETN | 0,885 | 0,877 | 0,893 | 0,924 | 0,875 | 0,973 | 0,957 | 0,910 | 1 |
| IFI27 OAS1 IFI44L ISG15 OLAH RETN | 0,885 | 0,876 | 0,893 | 0,917 | 0,859 | 0,975 | 0,939 | 0,878 | 1 |
| RSAD2 OAS1 IFIT1 SIGLEC1 IL1R2 FAM20A | 0,885 | 0,876 | 0,894 | 0,912 | 0,849 | 0,975 | 0,965 | 0,917 | 1 |
| RSAD2 IFI27 OAS1 ISG15 HERC6 IL1R2 | 0,885 | 0,877 | 0,893 | 0,921 | 0,874 | 0,968 | 0,933 | 0,874 | 0,991 |
| IFI27 IFI44L SIGLEC1 SLC1A2 FAM20A RETN | 0,885 | 0,875 | 0,894 | 0,920 | 0,863 | 0,978 | 0,945 | 0,869 | 1 |
| IFIT1 IFI44L SIGLEC1 HERC6 FAM20A MMP8 | 0,885 | 0,875 | 0,894 | 0,911 | 0,850 | 0,973 | 0,935 | 0,864 | 1 |
| RSAD2 IFI27 IFIT1 IFI44L OLAH RETN | 0,885 | 0,876 | 0,893 | 0,916 | 0,857 | 0,975 | 0,945 | 0,884 | 1 |
| RSAD2 IFI27 IFIT1 ISG15 HERC6 IL1R2 | 0,885 | 0,877 | 0,892 | 0,918 | 0,869 | 0,967 | 0,937 | 0,882 | 0,992 |
| RSAD2 IFI27 SIGLEC1 ISG15 IL1R2 MMP8 | 0,885 | 0,877 | 0,893 | 0,919 | 0,871 | 0,968 | 0,948 | 0,899 | 0,996 |
| IFI27 SIGLEC1 ISG15 SLC1A2 FAM20A RETN | 0,885 | 0,875 | 0,894 | 0,922 | 0,865 | 0,978 | 0,943 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L SIGLEC1 SLC1A2 OLAH FAM20A MMP8 | 0,885 | 0,875 | 0,894 | 0,918 | 0,863 | 0,974 | 0,951 | 0,888 | 1 |
| RSAD2 SIGLEC1 ISG15 HERC6 IL1R2 FAM20A | 0,885 | 0,875 | 0,894 | 0,916 | 0,858 | 0,975 | 0,955 | 0,897 | 1 |
| IFIT1 IFI44L SIGLEC1 ISG15 IL1R2 FAM20A | 0,885 | 0,876 | 0,893 | 0,910 | 0,846 | 0,974 | 0,958 | 0,907 | 1 |
| RSAD2 IFI27 OAS1 SIGLEC1 SLC1A2 IL1R2 | 0,885 | 0,876 | 0,893 | 0,924 | 0,878 | 0,971 | 0,954 | 0,905 | 1 |
| OAS1 IFI44L SIGLEC1 HERC6 IL1R2 FAM20A | 0,885 | 0,876 | 0,893 | 0,915 | 0,857 | 0,974 | 0,959 | 0,905 | 1 |
| IFI27 OAS1 IFIT1 SIGLEC1 SLC1A2 IL1R2 | 0,884 | 0,876 | 0,893 | 0,926 | 0,880 | 0,971 | 0,951 | 0,902 | 1 |
| IFI27 OAS1 IFI44L SLC1A2 OLAH RETN | 0,884 | 0,875 | 0,893 | 0,928 | 0,877 | 0,979 | 0,935 | 0,866 | 1 |
| RSAD2 IFI27 IFI44L SLC1A2 OLAH RETN | 0,884 | 0,876 | 0,893 | 0,926 | 0,874 | 0,977 | 0,935 | 0,868 | 1 |
| IFI27 OAS1 SIGLEC1 ISG15 FAM20A MMP8 | 0,884 | 0,874 | 0,895 | 0,912 | 0,848 | 0,976 | 0,946 | 0,874 | 1 |
| RSAD2 IFI27 IFIT1 IFI44L SLC1A2 OLAH | 0,884 | 0,876 | 0,893 | 0,926 | 0,874 | 0,978 | 0,935 | 0,871 | 0,998 |
| IFI27 IFIT1 ISG15 IL1R2 RETN MMP8 | 0,884 | 0,876 | 0,893 | 0,918 | 0,863 | 0,974 | 0,949 | 0,897 | 1 |
| IFI27 ISG15 SLC1A2 IL1R2 FAM20A RETN | 0,884 | 0,876 | 0,893 | 0,924 | 0,874 | 0,974 | 0,912 | 0,819 | 1 |
| IFI27 OAS1 SLC1A2 IL1R2 FAM20A RETN | 0,884 | 0,876 | 0,893 | 0,929 | 0,880 | 0,978 | 0,924 | 0,837 | 1 |
| IFI27 IFIT1 SIGLEC1 SLC1A2 FAM20A MMP8 | 0,884 | 0,875 | 0,893 | 0,919 | 0,861 | 0,977 | 0,945 | 0,869 | 1 |
| IFI27 SIGLEC1 SLC1A2 IL1R2 RETN MMP8 | 0,884 | 0,876 | 0,893 | 0,926 | 0,878 | 0,974 | 0,966 | 0,927 | 1 |
| RSAD2 IFI44L SIGLEC1 HERC6 IL1R2 FAM20A | 0,884 | 0,875 | 0,893 | 0,915 | 0,856 | 0,974 | 0,958 | 0,901 | 1 |
| RSAD2 SIGLEC1 HERC6 IL1R2 OLAH FAM20A | 0,884 | 0,875 | 0,894 | 0,915 | 0,856 | 0,974 | 0,955 | 0,898 | 1 |
| IFI27 IFIT1 IFI44L ISG15 HERC6 IL1R2 | 0,884 | 0,876 | 0,892 | 0,919 | 0,869 | 0,969 | 0,936 | 0,879 | 0,993 |
| RSAD2 IFI27 IFIT1 ISG15 HERC6 RETN | 0,884 | 0,875 | 0,893 | 0,914 | 0,855 | 0,973 | 0,940 | 0,882 | 0,998 |
| RSAD2 IFI27 IFI44L ISG15 OLAH RETN | 0,884 | 0,876 | 0,893 | 0,916 | 0,857 | 0,976 | 0,938 | 0,874 | 1 |
| RSAD2 IFI27 OAS1 SIGLEC1 ISG15 IL1R2 | 0,884 | 0,876 | 0,892 | 0,921 | 0,873 | 0,969 | 0,955 | 0,911 | 1,000 |
| OAS1 IFIT1 SIGLEC1 IL1R2 FAM20A RETN | 0,884 | 0,875 | 0,893 | 0,912 | 0,849 | 0,974 | 0,961 | 0,908 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 SIGLEC1 SLC1A2 IL1R2 MMP8 | 0,884 | 0,876 | 0,892 | 0,924 | 0,878 | 0,970 | 0,953 | 0,906 | 1 |
| RSAD2 IFIT1 IFI44L SIGLEC1 IL1R2 FAM20A | 0,884 | 0,875 | 0,893 | 0,912 | 0,848 | 0,975 | 0,962 | 0,913 | 1 |
| RSAD2 IFI27 SIGLEC1 HERC6 SLC1A2 IL1R2 | 0,884 | 0,876 | 0,892 | 0,925 | 0,880 | 0,970 | 0,949 | 0,900 | 0,998 |
| RSAD2 IFI27 IFIT1 IFI44L IL1R2 RETN | 0,884 | 0,876 | 0,892 | 0,916 | 0,860 | 0,971 | 0,940 | 0,882 | 0,999 |
| IFI27 SIGLEC1 SLC1A2 FAM20A RETN MMP8 | 0,884 | 0,874 | 0,894 | 0,922 | 0,863 | 0,981 | 0,950 | 0,874 | 1 |
| RSAD2 IFI27 IFI44L SIGLEC1 FAM20A MMP8 | 0,884 | 0,874 | 0,893 | 0,913 | 0,848 | 0,978 | 0,948 | 0,878 | 1 |
| IFI27 OAS1 SIGLEC1 SLC1A2 IL1R2 MMP8 | 0,884 | 0,876 | 0,892 | 0,925 | 0,880 | 0,970 | 0,950 | 0,901 | 0,999 |
| RSAD2 SIGLEC1 SLC1A2 OLAH FAM20A MMP8 | 0,884 | 0,874 | 0,893 | 0,919 | 0,864 | 0,973 | 0,948 | 0,886 | 1 |
| IFI27 SIGLEC1 ISG15 SLC1A2 IL1R2 MMP8 | 0,884 | 0,876 | 0,892 | 0,924 | 0,879 | 0,970 | 0,950 | 0,901 | 0,999 |
| RSAD2 OAS1 IFIT1 SIGLEC1 HERC6 FAM20A | 0,884 | 0,875 | 0,893 | 0,911 | 0,850 | 0,972 | 0,937 | 0,865 | 1 |
| RSAD2 IFI27 IFIT1 IFI44L ISG15 OLAH | 0,884 | 0,875 | 0,892 | 0,915 | 0,857 | 0,974 | 0,936 | 0,876 | 0,996 |
| IFIT1 IFI44L SIGLEC1 IL1R2 FAM20A RETN | 0,884 | 0,875 | 0,893 | 0,909 | 0,847 | 0,972 | 0,960 | 0,908 | 1 |
| RSAD2 IFI27 OAS1 IL1R2 RETN MMP8 | 0,884 | 0,875 | 0,893 | 0,918 | 0,864 | 0,973 | 0,939 | 0,877 | 1 |
| IFI27 OAS1 IFI44L ISG15 HERC6 IL1R2 | 0,884 | 0,876 | 0,892 | 0,920 | 0,873 | 0,968 | 0,930 | 0,871 | 0,989 |
| RSAD2 IFI27 IFIT1 SIGLEC1 ISG15 IL1R2 | 0,884 | 0,876 | 0,892 | 0,920 | 0,871 | 0,968 | 0,947 | 0,897 | 0,996 |
| IFI27 OAS1 IFIT1 ISG15 IL1R2 RETN | 0,884 | 0,875 | 0,892 | 0,918 | 0,865 | 0,972 | 0,949 | 0,896 | 1 |
| SIGLEC1 ISG15 SLC1A2 OLAH FAM20A MMP8 | 0,884 | 0,874 | 0,893 | 0,916 | 0,860 | 0,971 | 0,951 | 0,889 | 1 |
| IFI27 IFIT1 SIGLEC1 ISG15 SLC1A2 IL1R2 | 0,884 | 0,876 | 0,892 | 0,924 | 0,878 | 0,969 | 0,949 | 0,900 | 0,998 |
| OAS1 IFIT1 IFI44L SIGLEC1 HERC6 FAM20A | 0,884 | 0,875 | 0,892 | 0,909 | 0,847 | 0,971 | 0,936 | 0,866 | 1 |
| RSAD2 IFIT1 SIGLEC1 ISG15 HERC6 FAM20A | 0,884 | 0,875 | 0,892 | 0,911 | 0,850 | 0,972 | 0,936 | 0,862 | 1 |
| RSAD2 IFIT1 SIGLEC1 ISG15 IL1R2 FAM20A | 0,884 | 0,875 | 0,892 | 0,911 | 0,847 | 0,975 | 0,963 | 0,914 | 1 |
| RSAD2 IFI27 IFI44L ISG15 HERC6 RETN | 0,883 | 0,875 | 0,892 | 0,914 | 0,855 | 0,973 | 0,941 | 0,884 | 0,998 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFI44L SLC1A2 IL1R2 OLAH | 0,883 | 0,874 | 0,893 | 0,927 | 0,878 | 0,977 | 0,929 | 0,861 | 0,998 |
| IFI27 IFIT1 SIGLEC1 SLC1A2 IL1R2 MMP8 | 0,883 | 0,875 | 0,892 | 0,924 | 0,878 | 0,970 | 0,955 | 0,911 | 1 |
| OAS1 IFIT1 IFI44L SIGLEC1 OLAH FAM20A | 0,883 | 0,874 | 0,893 | 0,905 | 0,835 | 0,974 | 0,948 | 0,893 | 1 |
| IFI27 OAS1 IFIT1 SIGLEC1 SLC1A2 FAM20A | 0,883 | 0,874 | 0,893 | 0,920 | 0,863 | 0,977 | 0,943 | 0,871 | 1 |
| IFI27 IFIT1 SIGLEC1 HERC6 SLC1A2 IL1R2 | 0,883 | 0,875 | 0,892 | 0,925 | 0,880 | 0,971 | 0,951 | 0,904 | 0,998 |
| OAS1 IFIT1 SIGLEC1 HERC6 FAM20A MMP8 | 0,883 | 0,874 | 0,893 | 0,910 | 0,848 | 0,972 | 0,938 | 0,868 | 1 |
| IFIT1 SIGLEC1 ISG15 IL1R2 OLAH FAM20A | 0,883 | 0,874 | 0,892 | 0,913 | 0,850 | 0,975 | 0,962 | 0,914 | 1 |
| RSAD2 IFI27 HERC6 SLC1A2 FAM20A RETN | 0,883 | 0,874 | 0,893 | 0,921 | 0,865 | 0,977 | 0,932 | 0,849 | 1 |
| RSAD2 IFI27 OAS1 IFI44L IL1R2 RETN | 0,883 | 0,875 | 0,892 | 0,916 | 0,862 | 0,971 | 0,941 | 0,883 | 1,000 |
| SIGLEC1 ISG15 HERC6 IL1R2 OLAH FAM20A | 0,883 | 0,874 | 0,893 | 0,911 | 0,849 | 0,974 | 0,958 | 0,899 | 1 |
| RSAD2 IFI27 IFI44L ISG15 HERC6 IL1R2 | 0,883 | 0,875 | 0,891 | 0,919 | 0,870 | 0,968 | 0,932 | 0,874 | 0,989 |
| RSAD2 IFI27 IFIT1 IFI44L HERC6 RETN | 0,883 | 0,874 | 0,892 | 0,912 | 0,852 | 0,973 | 0,940 | 0,879 | 1 |
| RSAD2 IFI27 OAS1 IFIT1 IFI44L IL1R2 | 0,883 | 0,875 | 0,891 | 0,915 | 0,863 | 0,968 | 0,940 | 0,886 | 0,994 |
| IFI27 IFI44L HERC6 SLC1A2 IL1R2 RETN | 0,883 | 0,874 | 0,892 | 0,923 | 0,874 | 0,973 | 0,934 | 0,868 | 0,999 |
| OAS1 IFIT1 SIGLEC1 ISG15 HERC6 FAM20A | 0,883 | 0,874 | 0,892 | 0,910 | 0,849 | 0,971 | 0,941 | 0,873 | 1 |
| OAS1 SIGLEC1 HERC6 IL1R2 OLAH FAM20A | 0,883 | 0,874 | 0,893 | 0,913 | 0,853 | 0,972 | 0,959 | 0,905 | 1 |
| RSAD2 IFI27 IFIT1 SIGLEC1 SLC1A2 FAM20A | 0,883 | 0,874 | 0,892 | 0,921 | 0,865 | 0,977 | 0,945 | 0,869 | 1 |
| RSAD2 IFI27 OAS1 IFIT1 IL1R2 MMP8 | 0,883 | 0,875 | 0,892 | 0,915 | 0,864 | 0,965 | 0,943 | 0,891 | 0,996 |
| IFIT1 SIGLEC1 ISG15 OLAH FAM20A MMP8 | 0,883 | 0,874 | 0,893 | 0,907 | 0,841 | 0,972 | 0,957 | 0,909 | 1 |
| RSAD2 IFI27 HERC6 SLC1A2 IL1R2 RETN | 0,883 | 0,875 | 0,892 | 0,923 | 0,874 | 0,973 | 0,935 | 0,871 | 0,999 |
| OAS1 SIGLEC1 SLC1A2 OLAH FAM20A MMP8 | 0,883 | 0,873 | 0,893 | 0,917 | 0,861 | 0,972 | 0,951 | 0,890 | 1 |
| RSAD2 IFI27 ISG15 HERC6 IL1R2 MMP8 | 0,883 | 0,875 | 0,891 | 0,916 | 0,866 | 0,965 | 0,933 | 0,875 | 0,990 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 SIGLEC1 ISG15 OLAH FAM20A | 0,883 | 0,874 | 0,892 | 0,905 | 0,836 | 0,974 | 0,949 | 0,896 | 1 |
| OAS1 SIGLEC1 ISG15 HERC6 IL1R2 FAM20A | 0,883 | 0,874 | 0,892 | 0,914 | 0,855 | 0,972 | 0,959 | 0,905 | 1 |
| IFI27 OAS1 SIGLEC1 HERC6 SLC1A2 FAM20A | 0,883 | 0,873 | 0,893 | 0,921 | 0,866 | 0,975 | 0,945 | 0,869 | 1 |
| IFI27 OAS1 IFI44L IL1R2 RETN MMP8 | 0,883 | 0,875 | 0,891 | 0,918 | 0,864 | 0,972 | 0,939 | 0,879 | 0,999 |
| IFI27 IFIT1 ISG15 HERC6 IL1R2 MMP8 | 0,883 | 0,875 | 0,891 | 0,915 | 0,865 | 0,965 | 0,938 | 0,884 | 0,993 |
| RSAD2 IFIT1 SIGLEC1 OLAH FAM20A MMP8 | 0,883 | 0,874 | 0,892 | 0,908 | 0,843 | 0,974 | 0,959 | 0,911 | 1 |
| IFI27 OAS1 IFI44L SIGLEC1 FAM20A MMP8 | 0,883 | 0,873 | 0,893 | 0,912 | 0,847 | 0,976 | 0,946 | 0,875 | 1 |
| IFI27 OAS1 SIGLEC1 ISG15 SLC1A2 IL1R2 | 0,883 | 0,875 | 0,891 | 0,925 | 0,880 | 0,970 | 0,950 | 0,901 | 0,999 |
| IFI27 OAS1 ISG15 HERC6 SLC1A2 RETN | 0,883 | 0,874 | 0,892 | 0,917 | 0,860 | 0,974 | 0,932 | 0,859 | 1 |
| RSAD2 IFI27 SIGLEC1 HERC6 SLC1A2 RETN | 0,883 | 0,874 | 0,892 | 0,918 | 0,863 | 0,973 | 0,948 | 0,891 | 1 |
| IFIT1 IFI44L SIGLEC1 ISG15 OLAH FAM20A | 0,883 | 0,874 | 0,892 | 0,905 | 0,836 | 0,974 | 0,945 | 0,888 | 1 |
| IFIT1 IFI44L SIGLEC1 HERC6 SLC1A2 FAM20A | 0,883 | 0,874 | 0,892 | 0,919 | 0,866 | 0,972 | 0,934 | 0,856 | 1 |
| OAS1 IFIT1 SIGLEC1 SLC1A2 OLAH FAM20A | 0,883 | 0,874 | 0,892 | 0,919 | 0,863 | 0,974 | 0,945 | 0,881 | 1 |
| IFI44L SIGLEC1 ISG15 IL1R2 OLAH FAM20A | 0,883 | 0,874 | 0,892 | 0,910 | 0,848 | 0,973 | 0,961 | 0,909 | 1 |
| RSAD2 IFIT1 IFI44L SIGLEC1 HERC6 FAM20A | 0,883 | 0,874 | 0,891 | 0,910 | 0,849 | 0,971 | 0,932 | 0,860 | 1 |
| RSAD2 IFI27 OAS1 HERC6 SLC1A2 RETN | 0,883 | 0,873 | 0,892 | 0,914 | 0,854 | 0,974 | 0,929 | 0,851 | 1 |
| IFI27 IFI44L SIGLEC1 HERC6 SLC1A2 IL1R2 | 0,883 | 0,874 | 0,891 | 0,927 | 0,883 | 0,972 | 0,946 | 0,896 | 0,995 |
| RSAD2 IFI27 SIGLEC1 ISG15 SLC1A2 IL1R2 | 0,883 | 0,875 | 0,891 | 0,924 | 0,879 | 0,970 | 0,949 | 0,900 | 0,998 |
| IFIT1 SIGLEC1 IL1R2 OLAH FAM20A RETN | 0,883 | 0,874 | 0,892 | 0,910 | 0,847 | 0,972 | 0,960 | 0,908 | 1 |
| IFIT1 SIGLEC1 HERC6 SLC1A2 FAM20A RETN | 0,883 | 0,873 | 0,892 | 0,919 | 0,865 | 0,972 | 0,936 | 0,859 | 1 |
| RSAD2 IFI27 SIGLEC1 HERC6 SLC1A2 FAM20A | 0,883 | 0,873 | 0,892 | 0,921 | 0,866 | 0,976 | 0,940 | 0,864 | 1 |
| RSAD2 IFI27 IFIT1 IFI44L IL1R2 MMP8 | 0,883 | 0,875 | 0,890 | 0,915 | 0,863 | 0,968 | 0,937 | 0,881 | 0,993 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 HERC6 SLC1A2 FAM20A RETN | 0,883 | 0,873 | 0,892 | 0,919 | 0,861 | 0,976 | 0,933 | 0,851 | 1 |
| RSAD2 IFI27 IFIT1 ISG15 IL1R2 RETN | 0,882 | 0,874 | 0,891 | 0,917 | 0,864 | 0,971 | 0,947 | 0,894 | 1,000 |
| IFI27 SIGLEC1 HERC6 SLC1A2 IL1R2 RETN | 0,882 | 0,874 | 0,891 | 0,924 | 0,874 | 0,973 | 0,954 | 0,906 | 1 |
| IFIT1 IFI44L SIGLEC1 SLC1A2 IL1R2 FAM20A | 0,882 | 0,874 | 0,891 | 0,920 | 0,869 | 0,972 | 0,948 | 0,888 | 1 |
| IFI27 IFIT1 IFI44L ISG15 HERC6 RETN | 0,882 | 0,873 | 0,892 | 0,911 | 0,849 | 0,972 | 0,939 | 0,874 | 1 |
| RSAD2 IFI27 IFI44L IL1R2 RETN MMP8 | 0,882 | 0,874 | 0,891 | 0,919 | 0,864 | 0,973 | 0,943 | 0,887 | 1,000 |
| IFI27 SIGLEC1 HERC6 SLC1A2 FAM20A RETN | 0,882 | 0,872 | 0,892 | 0,920 | 0,862 | 0,978 | 0,949 | 0,873 | 1 |
| IFI44L SIGLEC1 HERC6 IL1R2 FAM20A RETN | 0,882 | 0,873 | 0,892 | 0,912 | 0,852 | 0,973 | 0,955 | 0,893 | 1 |
| IFI27 IFIT1 IFI44L SIGLEC1 SLC1A2 IL1R2 | 0,882 | 0,874 | 0,891 | 0,924 | 0,877 | 0,970 | 0,950 | 0,903 | 0,997 |
| RSAD2 IFIT1 SIGLEC1 HERC6 FAM20A MMP8 | 0,882 | 0,873 | 0,892 | 0,911 | 0,849 | 0,972 | 0,937 | 0,865 | 1 |
| IFI27 OAS1 IFIT1 IFI44L IL1R2 MMP8 | 0,882 | 0,874 | 0,890 | 0,917 | 0,865 | 0,969 | 0,939 | 0,885 | 0,993 |
| IFI27 IFIT1 SIGLEC1 HERC6 SLC1A2 RETN | 0,882 | 0,873 | 0,891 | 0,919 | 0,864 | 0,973 | 0,950 | 0,897 | 1 |
| RSAD2 OAS1 IFIT1 SIGLEC1 OLAH FAM20A | 0,882 | 0,873 | 0,892 | 0,905 | 0,836 | 0,975 | 0,955 | 0,906 | 1 |
| IFI27 OAS1 IFI44L HERC6 SLC1A2 RETN | 0,882 | 0,873 | 0,891 | 0,914 | 0,855 | 0,973 | 0,930 | 0,857 | 1 |
| IFI27 OAS1 IFIT1 SIGLEC1 ISG15 RETN | 0,882 | 0,873 | 0,891 | 0,909 | 0,848 | 0,970 | 0,954 | 0,896 | 1 |
| IFI27 SIGLEC1 ISG15 HERC6 FAM20A MMP8 | 0,882 | 0,872 | 0,892 | 0,905 | 0,837 | 0,972 | 0,942 | 0,863 | 1 |
| SIGLEC1 HERC6 SLC1A2 OLAH FAM20A MMP8 | 0,882 | 0,872 | 0,892 | 0,918 | 0,863 | 0,973 | 0,951 | 0,883 | 1 |
| RSAD2 IFI27 ISG15 HERC6 RETN MMP8 | 0,882 | 0,873 | 0,891 | 0,914 | 0,855 | 0,973 | 0,940 | 0,883 | 0,998 |
| RSAD2 OAS1 SIGLEC1 IL1R2 OLAH FAM20A | 0,882 | 0,873 | 0,891 | 0,910 | 0,847 | 0,973 | 0,963 | 0,912 | 1 |
| RSAD2 IFI27 IFIT1 SIGLEC1 SLC1A2 IL1R2 | 0,882 | 0,874 | 0,890 | 0,924 | 0,878 | 0,970 | 0,952 | 0,905 | 1,000 |
| RSAD2 IFI27 OAS1 IFIT1 SIGLEC1 RETN | 0,882 | 0,873 | 0,891 | 0,909 | 0,846 | 0,971 | 0,952 | 0,892 | 1 |
| IFI27 IFI44L SLC1A2 IL1R2 RETN MMP8 | 0,882 | 0,873 | 0,891 | 0,924 | 0,875 | 0,972 | 0,936 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 SIGLEC1 SLC1A2 IL1R2 FAM20A | 0,882 | 0,873 | 0,891 | 0,917 | 0,862 | 0,972 | 0,954 | 0,892 | 1 |
| RSAD2 SIGLEC1 HERC6 SLC1A2 IL1R2 FAM20A | 0,882 | 0,872 | 0,892 | 0,919 | 0,867 | 0,971 | 0,947 | 0,874 | 1 |
| IFIT1 SIGLEC1 SLC1A2 IL1R2 OLAH FAM20A | 0,882 | 0,873 | 0,891 | 0,916 | 0,862 | 0,971 | 0,952 | 0,894 | 1 |
| IFI27 IFIT1 IFI44L ISG15 IL1R2 MMP8 | 0,882 | 0,873 | 0,890 | 0,918 | 0,866 | 0,969 | 0,937 | 0,881 | 0,993 |
| RSAD2 IFIT1 SIGLEC1 IL1R2 FAM20A RETN | 0,882 | 0,873 | 0,891 | 0,911 | 0,847 | 0,974 | 0,963 | 0,913 | 1 |
| RSAD2 SIGLEC1 HERC6 IL1R2 FAM20A RETN | 0,882 | 0,872 | 0,891 | 0,913 | 0,854 | 0,973 | 0,955 | 0,894 | 1 |
| IFIT1 SIGLEC1 HERC6 SLC1A2 FAM20A MMP8 | 0,882 | 0,872 | 0,891 | 0,917 | 0,862 | 0,971 | 0,940 | 0,864 | 1 |
| IFIT1 SIGLEC1 ISG15 IL1R2 FAM20A RETN | 0,882 | 0,873 | 0,891 | 0,909 | 0,846 | 0,972 | 0,961 | 0,910 | 1 |
| RSAD2 IFIT1 SIGLEC1 SLC1A2 OLAH FAM20A | 0,882 | 0,873 | 0,891 | 0,920 | 0,865 | 0,975 | 0,950 | 0,890 | 1 |
| OAS1 IFIT1 SIGLEC1 SLC1A2 IL1R2 FAM20A | 0,882 | 0,873 | 0,891 | 0,914 | 0,857 | 0,971 | 0,951 | 0,888 | 1 |
| OAS1 SIGLEC1 HERC6 SLC1A2 IL1R2 FAM20A | 0,882 | 0,872 | 0,891 | 0,919 | 0,868 | 0,970 | 0,949 | 0,878 | 1 |
| IFI27 IFIT1 IFI44L HERC6 FAM20A MMP8 | 0,882 | 0,872 | 0,891 | 0,910 | 0,849 | 0,971 | 0,924 | 0,842 | 1 |
| IFI27 SIGLEC1 ISG15 HERC6 SLC1A2 IL1R2 | 0,882 | 0,873 | 0,890 | 0,924 | 0,879 | 0,970 | 0,949 | 0,900 | 0,998 |
| IFI27 OAS1 IFIT1 HERC6 SLC1A2 RETN | 0,882 | 0,872 | 0,891 | 0,913 | 0,854 | 0,972 | 0,936 | 0,863 | 1 |
| IFI27 OAS1 IFI44L ISG15 IL1R2 RETN | 0,882 | 0,873 | 0,890 | 0,919 | 0,867 | 0,972 | 0,938 | 0,878 | 0,998 |
| IFI27 IFIT1 IFI44L ISG15 FAM20A RETN | 0,882 | 0,872 | 0,891 | 0,910 | 0,846 | 0,974 | 0,943 | 0,866 | 1 |
| IFI27 OAS1 IFI44L SIGLEC1 SLC1A2 IL1R2 | 0,882 | 0,873 | 0,890 | 0,923 | 0,877 | 0,969 | 0,945 | 0,893 | 0,996 |
| IFI44L SIGLEC1 HERC6 SLC1A2 IL1R2 FAM20A | 0,882 | 0,872 | 0,891 | 0,918 | 0,865 | 0,972 | 0,951 | 0,879 | 1 |
| IFIT1 SIGLEC1 OLAH FAM20A RETN MMP8 | 0,882 | 0,872 | 0,891 | 0,912 | 0,850 | 0,975 | 0,958 | 0,909 | 1 |
| OAS1 IFI44L SIGLEC1 IL1R2 OLAH FAM20A | 0,881 | 0,872 | 0,891 | 0,910 | 0,847 | 0,973 | 0,963 | 0,912 | 1 |
| IFI44L SIGLEC1 SLC1A2 IL1R2 OLAH FAM20A | 0,881 | 0,873 | 0,890 | 0,916 | 0,861 | 0,971 | 0,954 | 0,893 | 1 |
| IFI27 OAS1 IFIT1 IFI44L SIGLEC1 RETN | 0,881 | 0,872 | 0,890 | 0,910 | 0,849 | 0,970 | 0,955 | 0,897 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 ISG15 HERC6 SLC1A2 FAM20A RETN | 0,881 | 0,872 | 0,891 | 0,918 | 0,861 | 0,976 | 0,941 | 0,862 | 1 |
| IFI27 OAS1 IFIT1 SLC1A2 IL1R2 RETN | 0,881 | 0,873 | 0,890 | 0,920 | 0,868 | 0,971 | 0,941 | 0,878 | 1 |
| IFIT1 SIGLEC1 ISG15 HERC6 SLC1A2 FAM20A | 0,881 | 0,872 | 0,891 | 0,918 | 0,864 | 0,972 | 0,940 | 0,864 | 1 |
| OAS1 IFIT1 SIGLEC1 OLAH FAM20A RETN | 0,881 | 0,872 | 0,891 | 0,905 | 0,835 | 0,974 | 0,955 | 0,906 | 1 |
| IFI27 SIGLEC1 HERC6 SLC1A2 IL1R2 MMP8 | 0,881 | 0,873 | 0,890 | 0,924 | 0,878 | 0,970 | 0,955 | 0,911 | 1 |
| IFI27 OAS1 IFIT1 HERC6 SLC1A2 IL1R2 | 0,881 | 0,873 | 0,890 | 0,922 | 0,876 | 0,968 | 0,937 | 0,874 | 1 |
| IFI27 IFI44L HERC6 SLC1A2 FAM20A RETN | 0,881 | 0,872 | 0,891 | 0,919 | 0,862 | 0,976 | 0,941 | 0,862 | 1 |
| RSAD2 IFI27 IFI44L ISG15 IL1R2 RETN | 0,881 | 0,873 | 0,889 | 0,918 | 0,864 | 0,972 | 0,938 | 0,878 | 0,998 |
| IFI27 IFIT1 HERC6 SLC1A2 IL1R2 RETN | 0,881 | 0,873 | 0,890 | 0,922 | 0,872 | 0,972 | 0,936 | 0,872 | 1,000 |
| SIGLEC1 ISG15 IL1R2 OLAH FAM20A RETN | 0,881 | 0,872 | 0,890 | 0,906 | 0,841 | 0,970 | 0,963 | 0,910 | 1 |
| OAS1 SIGLEC1 HERC6 IL1R2 FAM20A RETN | 0,881 | 0,872 | 0,890 | 0,912 | 0,852 | 0,971 | 0,960 | 0,905 | 1 |
| IFI44L SIGLEC1 ISG15 SLC1A2 IL1R2 FAM20A | 0,881 | 0,872 | 0,890 | 0,915 | 0,860 | 0,969 | 0,953 | 0,888 | 1 |
| IFI27 OAS1 ISG15 IL1R2 RETN MMP8 | 0,881 | 0,873 | 0,889 | 0,919 | 0,866 | 0,972 | 0,940 | 0,881 | 0,999 |
| RSAD2 IFI27 ISG15 IL1R2 RETN MMP8 | 0,881 | 0,872 | 0,890 | 0,919 | 0,864 | 0,973 | 0,942 | 0,884 | 1 |
| IFI27 OAS1 IFIT1 SIGLEC1 SLC1A2 RETN | 0,881 | 0,872 | 0,890 | 0,915 | 0,859 | 0,971 | 0,947 | 0,881 | 1 |
| IFI27 IFIT1 IFI44L HERC6 RETN MMP8 | 0,881 | 0,871 | 0,891 | 0,908 | 0,846 | 0,971 | 0,929 | 0,859 | 0,999 |
| IFI27 OAS1 IFIT1 IFI44L ISG15 IL1R2 | 0,881 | 0,873 | 0,889 | 0,916 | 0,864 | 0,968 | 0,939 | 0,885 | 0,994 |
| IFI27 IFI44L ISG15 SLC1A2 IL1R2 RETN | 0,881 | 0,873 | 0,889 | 0,922 | 0,872 | 0,972 | 0,933 | 0,866 | 0,999 |
| RSAD2 IFI27 OAS1 IFI44L IL1R2 MMP8 | 0,881 | 0,873 | 0,889 | 0,914 | 0,862 | 0,966 | 0,937 | 0,881 | 0,993 |
| IFI27 IFI44L ISG15 FAM20A RETN MMP8 | 0,881 | 0,870 | 0,891 | 0,912 | 0,849 | 0,975 | 0,948 | 0,874 | 1 |
| IFI27 IFIT1 SLC1A2 IL1R2 RETN MMP8 | 0,881 | 0,872 | 0,890 | 0,922 | 0,872 | 0,973 | 0,945 | 0,886 | 1 |
| IFI44L SIGLEC1 ISG15 IL1R2 FAM20A RETN | 0,881 | 0,872 | 0,890 | 0,907 | 0,842 | 0,971 | 0,962 | 0,909 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 SLC1A2 IL1R2 OLAH FAM20A | 0,881 | 0,871 | 0,890 | 0,915 | 0,860 | 0,970 | 0,954 | 0,893 | 1 |
| IFI27 OAS1 SIGLEC1 ISG15 SLC1A2 FAM20A | 0,881 | 0,871 | 0,891 | 0,916 | 0,857 | 0,974 | 0,946 | 0,872 | 1 |
| IFI27 HERC6 SLC1A2 IL1R2 RETN MMP8 | 0,881 | 0,872 | 0,890 | 0,923 | 0,873 | 0,973 | 0,935 | 0,868 | 1 |
| RSAD2 IFI27 OAS1 HERC6 SLC1A2 IL1R2 | 0,881 | 0,872 | 0,889 | 0,923 | 0,878 | 0,968 | 0,932 | 0,866 | 0,997 |
| RSAD2 IFI27 OAS1 ISG15 IL1R2 RETN | 0,881 | 0,872 | 0,889 | 0,917 | 0,863 | 0,971 | 0,945 | 0,888 | 1 |
| IFI44L SIGLEC1 IL1R2 OLAH FAM20A RETN | 0,881 | 0,871 | 0,890 | 0,907 | 0,843 | 0,971 | 0,963 | 0,912 | 1 |
| SIGLEC1 HERC6 SLC1A2 IL1R2 OLAH FAM20A | 0,881 | 0,871 | 0,890 | 0,919 | 0,865 | 0,972 | 0,947 | 0,874 | 1 |
| IFIT1 IFI44L SIGLEC1 OLAH FAM20A RETN | 0,881 | 0,871 | 0,890 | 0,905 | 0,836 | 0,973 | 0,943 | 0,887 | 1 |
| IFI27 ISG15 HERC6 SLC1A2 IL1R2 RETN | 0,881 | 0,872 | 0,889 | 0,923 | 0,873 | 0,973 | 0,936 | 0,872 | 1,000 |
| RSAD2 IFI27 OAS1 SIGLEC1 SLC1A2 FAM20A | 0,881 | 0,871 | 0,890 | 0,919 | 0,860 | 0,977 | 0,949 | 0,878 | 1 |
| RSAD2 IFIT1 IFI44L SIGLEC1 OLAH FAM20A | 0,881 | 0,871 | 0,890 | 0,905 | 0,836 | 0,974 | 0,947 | 0,892 | 1 |
| RSAD2 IFI27 OAS1 IFI44L SIGLEC1 RETN | 0,880 | 0,872 | 0,889 | 0,908 | 0,846 | 0,971 | 0,952 | 0,889 | 1 |
| RSAD2 IFI27 SIGLEC1 ISG15 SLC1A2 FAM20A | 0,880 | 0,871 | 0,890 | 0,917 | 0,858 | 0,976 | 0,946 | 0,872 | 1 |
| RSAD2 IFI27 SIGLEC1 SLC1A2 FAM20A MMP8 | 0,880 | 0,871 | 0,890 | 0,919 | 0,860 | 0,978 | 0,947 | 0,873 | 1 |
| IFI27 OAS1 SIGLEC1 SLC1A2 FAM20A MMP8 | 0,880 | 0,871 | 0,890 | 0,918 | 0,859 | 0,976 | 0,945 | 0,870 | 1 |
| IFI27 OAS1 IFI44L SIGLEC1 ISG15 RETN | 0,880 | 0,872 | 0,889 | 0,910 | 0,848 | 0,971 | 0,954 | 0,894 | 1 |
| OAS1 SIGLEC1 ISG15 IL1R2 OLAH FAM20A | 0,880 | 0,871 | 0,890 | 0,909 | 0,845 | 0,973 | 0,963 | 0,912 | 1 |
| RSAD2 IFI27 IFI44L ISG15 HERC6 FAM20A | 0,880 | 0,871 | 0,890 | 0,913 | 0,851 | 0,974 | 0,926 | 0,845 | 1 |
| IFI27 IFIT1 IFI44L SLC1A2 IL1R2 RETN | 0,880 | 0,872 | 0,889 | 0,919 | 0,866 | 0,972 | 0,938 | 0,875 | 1 |
| RSAD2 OAS1 IFI44L SIGLEC1 IL1R2 FAM20A | 0,880 | 0,871 | 0,890 | 0,910 | 0,846 | 0,973 | 0,963 | 0,912 | 1 |
| IFI27 IFIT1 IFI44L SIGLEC1 ISG15 RETN | 0,880 | 0,871 | 0,890 | 0,908 | 0,846 | 0,970 | 0,950 | 0,892 | 1 |
| RSAD2 IFI27 IFI44L SIGLEC1 SLC1A2 IL1R2 | 0,880 | 0,872 | 0,889 | 0,925 | 0,878 | 0,971 | 0,951 | 0,905 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L SIGLEC1 IL1R2 OLAH FAM20A | 0,880 | 0,871 | 0,889 | 0,910 | 0,847 | 0,973 | 0,964 | 0,914 | 1 |
| RSAD2 IFIT1 SIGLEC1 HERC6 SLC1A2 FAM20A | 0,880 | 0,871 | 0,889 | 0,917 | 0,863 | 0,971 | 0,937 | 0,860 | 1 |
| RSAD2 OAS1 SIGLEC1 IL1R2 FAM20A RETN | 0,880 | 0,871 | 0,890 | 0,908 | 0,844 | 0,972 | 0,961 | 0,906 | 1 |
| OAS1 SIGLEC1 SLC1A2 IL1R2 OLAH FAM20A | 0,880 | 0,871 | 0,890 | 0,915 | 0,860 | 0,970 | 0,955 | 0,896 | 1 |
| IFI27 IFIT1 ISG15 HERC6 RETN MMP8 | 0,880 | 0,871 | 0,890 | 0,909 | 0,848 | 0,971 | 0,935 | 0,870 | 1,000 |
| RSAD2 IFI27 IFI44L HERC6 RETN MMP8 | 0,880 | 0,871 | 0,889 | 0,911 | 0,850 | 0,972 | 0,933 | 0,868 | 0,997 |
| RSAD2 IFI27 IFIT1 HERC6 RETN MMP8 | 0,880 | 0,871 | 0,890 | 0,911 | 0,850 | 0,972 | 0,933 | 0,868 | 0,997 |
| OAS1 IFI44L SIGLEC1 ISG15 IL1R2 FAM20A | 0,880 | 0,871 | 0,889 | 0,909 | 0,845 | 0,973 | 0,962 | 0,911 | 1 |
| SIGLEC1 SLC1A2 IL1R2 OLAH FAM20A RETN | 0,880 | 0,871 | 0,889 | 0,916 | 0,863 | 0,970 | 0,957 | 0,896 | 1 |
| IFI27 OAS1 IFIT1 SIGLEC1 RETN MMP8 | 0,880 | 0,871 | 0,890 | 0,910 | 0,849 | 0,971 | 0,955 | 0,897 | 1 |
| IFI44L SIGLEC1 SLC1A2 IL1R2 FAM20A RETN | 0,880 | 0,871 | 0,889 | 0,915 | 0,862 | 0,969 | 0,953 | 0,885 | 1 |
| SIGLEC1 SLC1A2 OLAH FAM20A RETN MMP8 | 0,880 | 0,870 | 0,890 | 0,916 | 0,858 | 0,973 | 0,948 | 0,879 | 1 |
| RSAD2 IFI27 IFIT1 ISG15 IL1R2 MMP8 | 0,880 | 0,872 | 0,888 | 0,915 | 0,864 | 0,966 | 0,942 | 0,890 | 0,995 |
| RSAD2 IFI27 IFIT1 IFI44L ISG15 IL1R2 | 0,880 | 0,872 | 0,888 | 0,917 | 0,865 | 0,969 | 0,939 | 0,885 | 0,994 |
| IFIT1 SIGLEC1 ISG15 SLC1A2 OLAH FAM20A | 0,880 | 0,871 | 0,889 | 0,918 | 0,863 | 0,973 | 0,943 | 0,882 | 1 |
| IFI44L SIGLEC1 ISG15 SLC1A2 OLAH FAM20A | 0,880 | 0,871 | 0,889 | 0,911 | 0,852 | 0,970 | 0,945 | 0,878 | 1 |
| SIGLEC1 ISG15 SLC1A2 IL1R2 OLAH FAM20A | 0,880 | 0,871 | 0,889 | 0,915 | 0,861 | 0,970 | 0,954 | 0,893 | 1 |
| RSAD2 OAS1 SIGLEC1 ISG15 IL1R2 FAM20A | 0,880 | 0,871 | 0,889 | 0,910 | 0,846 | 0,973 | 0,964 | 0,914 | 1 |
| OAS1 IFIT1 SIGLEC1 FAM20A RETN MMP8 | 0,880 | 0,870 | 0,889 | 0,907 | 0,841 | 0,974 | 0,950 | 0,886 | 1 |
| IFIT1 IFI44L SIGLEC1 FAM20A RETN MMP8 | 0,880 | 0,870 | 0,889 | 0,908 | 0,842 | 0,975 | 0,943 | 0,881 | 1 |
| SIGLEC1 ISG15 HERC6 IL1R2 FAM20A RETN | 0,880 | 0,870 | 0,889 | 0,910 | 0,848 | 0,972 | 0,959 | 0,898 | 1 |
| RSAD2 IFI27 IFI44L SIGLEC1 SLC1A2 FAM20A | 0,880 | 0,870 | 0,890 | 0,918 | 0,859 | 0,978 | 0,947 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 SIGLEC1 HERC6 SLC1A2 FAM20A | 0,880 | 0,871 | 0,889 | 0,916 | 0,862 | 0,971 | 0,937 | 0,860 | 1 |
| IFI27 IFI44L ISG15 HERC6 RETN MMP8 | 0,880 | 0,870 | 0,889 | 0,908 | 0,846 | 0,970 | 0,935 | 0,867 | 1 |
| RSAD2 IFI27 OAS1 SIGLEC1 ISG15 RETN | 0,880 | 0,871 | 0,889 | 0,909 | 0,847 | 0,971 | 0,955 | 0,896 | 1 |
| IFIT1 SIGLEC1 SLC1A2 IL1R2 FAM20A RETN | 0,880 | 0,871 | 0,889 | 0,918 | 0,865 | 0,971 | 0,955 | 0,898 | 1 |
| IFIT1 SIGLEC1 ISG15 SLC1A2 IL1R2 FAM20A | 0,880 | 0,871 | 0,889 | 0,917 | 0,863 | 0,971 | 0,951 | 0,891 | 1 |
| SIGLEC1 HERC6 IL1R2 OLAH FAM20A RETN | 0,880 | 0,870 | 0,889 | 0,910 | 0,846 | 0,973 | 0,960 | 0,901 | 1 |
| IFI27 OAS1 IFIT1 SLC1A2 IL1R2 MMP8 | 0,880 | 0,871 | 0,888 | 0,922 | 0,875 | 0,968 | 0,936 | 0,873 | 0,999 |
| IFI27 OAS1 IFIT1 ISG15 IL1R2 MMP8 | 0,880 | 0,871 | 0,888 | 0,916 | 0,866 | 0,966 | 0,943 | 0,891 | 0,996 |
| RSAD2 IFI27 IFIT1 SLC1A2 IL1R2 RETN | 0,880 | 0,871 | 0,888 | 0,919 | 0,867 | 0,971 | 0,938 | 0,876 | 1 |
| RSAD2 IFI27 OAS1 IFIT1 ISG15 IL1R2 | 0,880 | 0,871 | 0,888 | 0,914 | 0,863 | 0,965 | 0,947 | 0,896 | 0,998 |
| IFI27 IFIT1 IFI44L FAM20A RETN MMP8 | 0,880 | 0,869 | 0,890 | 0,915 | 0,852 | 0,977 | 0,946 | 0,870 | 1 |
| RSAD2 IFI27 OAS1 SLC1A2 IL1R2 RETN | 0,880 | 0,871 | 0,888 | 0,920 | 0,868 | 0,973 | 0,930 | 0,856 | 1 |
| IFI27 IFI44L SIGLEC1 ISG15 SLC1A2 FAM20A | 0,880 | 0,870 | 0,889 | 0,914 | 0,856 | 0,973 | 0,946 | 0,869 | 1 |
| OAS1 SIGLEC1 IL1R2 OLAH FAM20A RETN | 0,880 | 0,870 | 0,889 | 0,907 | 0,843 | 0,971 | 0,962 | 0,907 | 1 |
| RSAD2 IFIT1 SIGLEC1 ISG15 OLAH FAM20A | 0,880 | 0,870 | 0,889 | 0,904 | 0,834 | 0,974 | 0,953 | 0,902 | 1 |
| RSAD2 SIGLEC1 IL1R2 OLAH FAM20A RETN | 0,880 | 0,870 | 0,889 | 0,908 | 0,844 | 0,971 | 0,963 | 0,910 | 1 |
| IFI44L SLC1A2 IL1R2 OLAH FAM20A MMP8 | 0,880 | 0,871 | 0,888 | 0,917 | 0,866 | 0,969 | 0,928 | 0,847 | 1 |
| IFI27 OAS1 HERC6 SLC1A2 IL1R2 MMP8 | 0,880 | 0,871 | 0,888 | 0,922 | 0,876 | 0,967 | 0,924 | 0,855 | 0,993 |
| RSAD2 SIGLEC1 SLC1A2 IL1R2 FAM20A RETN | 0,880 | 0,870 | 0,889 | 0,915 | 0,862 | 0,969 | 0,954 | 0,888 | 1 |
| RSAD2 OAS1 SIGLEC1 OLAH FAM20A MMP8 | 0,880 | 0,870 | 0,889 | 0,905 | 0,839 | 0,971 | 0,958 | 0,910 | 1 |
| RSAD2 SIGLEC1 ISG15 IL1R2 OLAH FAM20A | 0,880 | 0,870 | 0,889 | 0,909 | 0,845 | 0,972 | 0,963 | 0,912 | 1 |
| SIGLEC1 ISG15 HERC6 SLC1A2 IL1R2 FAM20A | 0,879 | 0,870 | 0,889 | 0,919 | 0,866 | 0,972 | 0,949 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFI44L ISG15 SLC1A2 IL1R2 MMP8 | 0,879 | 0,872 | 0,887 | 0,922 | 0,875 | 0,968 | 0,930 | 0,866 | 0,995 |
| SIGLEC1 ISG15 SLC1A2 IL1R2 FAM20A RETN | 0,879 | 0,870 | 0,888 | 0,915 | 0,862 | 0,969 | 0,953 | 0,885 | 1 |
| RSAD2 IFI27 IFIT1 HERC6 SLC1A2 IL1R2 | 0,879 | 0,871 | 0,888 | 0,923 | 0,878 | 0,968 | 0,938 | 0,878 | 0,998 |
| IFI27 OAS1 ISG15 HERC6 SLC1A2 IL1R2 | 0,879 | 0,871 | 0,888 | 0,922 | 0,877 | 0,967 | 0,925 | 0,856 | 0,994 |
| RSAD2 IFI27 OAS1 IFIT1 SLC1A2 IL1R2 | 0,879 | 0,871 | 0,888 | 0,920 | 0,873 | 0,968 | 0,936 | 0,871 | 1 |
| RSAD2 IFI27 OAS1 IFI44L FAM20A RETN | 0,879 | 0,869 | 0,889 | 0,912 | 0,850 | 0,975 | 0,945 | 0,869 | 1 |
| IFI27 IFIT1 ISG15 SLC1A2 IL1R2 RETN | 0,879 | 0,871 | 0,888 | 0,920 | 0,869 | 0,971 | 0,937 | 0,874 | 1 |
| OAS1 SIGLEC1 HERC6 SLC1A2 OLAH FAM20A | 0,879 | 0,869 | 0,889 | 0,915 | 0,860 | 0,970 | 0,943 | 0,875 | 1 |
| RSAD2 IFI27 SLC1A2 IL1R2 RETN MMP8 | 0,879 | 0,871 | 0,888 | 0,923 | 0,873 | 0,973 | 0,929 | 0,859 | 1 |
| OAS1 IFI44L SIGLEC1 IL1R2 FAM20A RETN | 0,879 | 0,870 | 0,889 | 0,905 | 0,841 | 0,970 | 0,961 | 0,906 | 1 |
| IFI27 IFI44L SIGLEC1 HERC6 SLC1A2 RETN | 0,879 | 0,870 | 0,888 | 0,916 | 0,859 | 0,973 | 0,947 | 0,890 | 1 |
| IFI27 OAS1 IFIT1 IFI44L FAM20A RETN | 0,879 | 0,869 | 0,889 | 0,910 | 0,846 | 0,973 | 0,945 | 0,868 | 1 |
| RSAD2 SIGLEC1 HERC6 OLAH FAM20A MMP8 | 0,879 | 0,870 | 0,889 | 0,910 | 0,850 | 0,970 | 0,953 | 0,898 | 1 |
| IFIT1 IFI44L SIGLEC1 ISG15 FAM20A RETN | 0,879 | 0,870 | 0,888 | 0,900 | 0,830 | 0,970 | 0,945 | 0,886 | 1 |
| RSAD2 IFI27 IFI44L ISG15 FAM20A RETN | 0,879 | 0,869 | 0,889 | 0,910 | 0,846 | 0,974 | 0,942 | 0,865 | 1 |
| RSAD2 IFI27 OAS1 SIGLEC1 SLC1A2 RETN | 0,879 | 0,870 | 0,888 | 0,915 | 0,856 | 0,973 | 0,950 | 0,881 | 1 |
| RSAD2 IFI27 HERC6 SLC1A2 IL1R2 MMP8 | 0,879 | 0,871 | 0,887 | 0,921 | 0,875 | 0,967 | 0,930 | 0,866 | 0,995 |
| RSAD2 IFI27 IFIT1 SIGLEC1 ISG15 RETN | 0,879 | 0,870 | 0,888 | 0,908 | 0,847 | 0,970 | 0,955 | 0,900 | 1 |
| IFIT1 IFI44L SIGLEC1 ISG15 FAM20A MMP8 | 0,879 | 0,870 | 0,888 | 0,901 | 0,831 | 0,970 | 0,938 | 0,869 | 1 |
| IFI27 OAS1 SIGLEC1 ISG15 SLC1A2 RETN | 0,879 | 0,870 | 0,888 | 0,915 | 0,858 | 0,972 | 0,948 | 0,880 | 1 |
| IFI27 OAS1 IFI44L SIGLEC1 SLC1A2 FAM20A | 0,879 | 0,869 | 0,889 | 0,918 | 0,860 | 0,977 | 0,948 | 0,874 | 1 |
| IFI27 HERC6 SLC1A2 FAM20A RETN MMP8 | 0,879 | 0,869 | 0,889 | 0,919 | 0,861 | 0,977 | 0,939 | 0,859 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1 SIGLEC1 SLC1A2 OLAH FAM20A RETN | 0,879 | 0,870 | 0,888 | 0,919 | 0,864 | 0,974 | 0,943 | 0,882 | 1 |
| IFI44L SIGLEC1 ISG15 OLAH FAM20A MMP8 | 0,879 | 0,869 | 0,889 | 0,904 | 0,837 | 0,971 | 0,961 | 0,915 | 1 |
| IFI27 OAS1 IFI44L SIGLEC1 SLC1A2 RETN | 0,879 | 0,871 | 0,887 | 0,915 | 0,859 | 0,972 | 0,949 | 0,883 | 1 |
| RSAD2 IFI27 IFIT1 SLC1A2 IL1R2 MMP8 | 0,879 | 0,871 | 0,887 | 0,922 | 0,877 | 0,968 | 0,937 | 0,876 | 0,998 |
| RSAD2 IFI27 IFI44L HERC6 FAM20A MMP8 | 0,879 | 0,869 | 0,889 | 0,912 | 0,851 | 0,972 | 0,925 | 0,844 | 1 |
| RSAD2 IFI27 IFIT1 IFI44L SIGLEC1 RETN | 0,879 | 0,870 | 0,888 | 0,908 | 0,847 | 0,970 | 0,950 | 0,892 | 1 |
| RSAD2 SIGLEC1 HERC6 FAM20A RETN MMP8 | 0,879 | 0,869 | 0,888 | 0,909 | 0,846 | 0,973 | 0,948 | 0,882 | 1 |
| RSAD2 SIGLEC1 ISG15 SLC1A2 IL1R2 FAM20A | 0,879 | 0,869 | 0,888 | 0,914 | 0,859 | 0,969 | 0,951 | 0,882 | 1 |
| IFI27 OAS1 HERC6 SLC1A2 RETN MMP8 | 0,879 | 0,869 | 0,888 | 0,915 | 0,858 | 0,972 | 0,926 | 0,850 | 1 |
| RSAD2 IFI27 IFIT1 IFI44L FAM20A RETN | 0,879 | 0,869 | 0,889 | 0,910 | 0,847 | 0,974 | 0,942 | 0,865 | 1 |
| RSAD2 SIGLEC1 HERC6 SLC1A2 OLAH FAM20A | 0,879 | 0,868 | 0,889 | 0,916 | 0,862 | 0,971 | 0,940 | 0,869 | 1 |
| IFI27 ISG15 SLC1A2 IL1R2 RETN MMP8 | 0,879 | 0,870 | 0,887 | 0,922 | 0,871 | 0,972 | 0,923 | 0,845 | 1 |
| IFI44L SIGLEC1 SLC1A2 OLAH FAM20A RETN | 0,879 | 0,869 | 0,888 | 0,914 | 0,855 | 0,972 | 0,948 | 0,884 | 1 |
| IFI27 IFIT1 ISG15 HERC6 FAM20A MMP8 | 0,879 | 0,869 | 0,889 | 0,909 | 0,849 | 0,969 | 0,924 | 0,839 | 1 |
| RSAD2 IFI44L SIGLEC1 ISG15 IL1R2 FAM20A | 0,879 | 0,869 | 0,888 | 0,910 | 0,846 | 0,973 | 0,963 | 0,912 | 1 |
| RSAD2 IFI27 OAS1 SIGLEC1 RETN MMP8 | 0,879 | 0,869 | 0,889 | 0,912 | 0,851 | 0,973 | 0,958 | 0,897 | 1 |
| IFI27 OAS1 IFI44L SLC1A2 IL1R2 RETN | 0,879 | 0,870 | 0,887 | 0,924 | 0,875 | 0,972 | 0,932 | 0,865 | 0,998 |
| SIGLEC1 HERC6 OLAH FAM20A RETN MMP8 | 0,878 | 0,868 | 0,889 | 0,909 | 0,842 | 0,975 | 0,963 | 0,910 | 1 |
| IFI27 OAS1 IFIT1 ISG15 SLC1A2 IL1R2 | 0,878 | 0,870 | 0,887 | 0,921 | 0,875 | 0,968 | 0,936 | 0,874 | 0,998 |
| IFI27 OAS1 IFI44L ISG15 FAM20A RETN | 0,878 | 0,869 | 0,888 | 0,911 | 0,848 | 0,974 | 0,946 | 0,869 | 1 |
| RSAD2 IFI27 IFI44L ISG15 IL1R2 MMP8 | 0,878 | 0,871 | 0,886 | 0,914 | 0,862 | 0,966 | 0,936 | 0,880 | 0,992 |
| OAS1 IFIT1 IFI44L SIGLEC1 FAM20A RETN | 0,878 | 0,869 | 0,888 | 0,902 | 0,832 | 0,972 | 0,943 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1 IFI44L SIGLEC1 ISG15 SLC1A2 FAM20A | 0,878 | 0,869 | 0,887 | 0,911 | 0,851 | 0,970 | 0,936 | 0,861 | 1 |
| IFI27 OAS1 SIGLEC1 ISG15 RETN MMP8 | 0,878 | 0,869 | 0,888 | 0,912 | 0,851 | 0,973 | 0,957 | 0,897 | 1 |
| IFI44L SIGLEC1 HERC6 OLAH FAM20A MMP8 | 0,878 | 0,868 | 0,888 | 0,905 | 0,840 | 0,970 | 0,961 | 0,906 | 1 |
| RSAD2 IFI27 IFI44L SLC1A2 IL1R2 RETN | 0,878 | 0,870 | 0,886 | 0,922 | 0,871 | 0,973 | 0,929 | 0,860 | 0,998 |
| OAS1 SIGLEC1 ISG15 IL1R2 FAM20A RETN | 0,878 | 0,869 | 0,888 | 0,906 | 0,842 | 0,971 | 0,961 | 0,904 | 1 |
| RSAD2 IFI27 OAS1 IFI44L ISG15 IL1R2 | 0,878 | 0,870 | 0,886 | 0,914 | 0,863 | 0,966 | 0,940 | 0,886 | 0,994 |
| IFI27 IFI44L SIGLEC1 HERC6 SLC1A2 FAM20A | 0,878 | 0,869 | 0,888 | 0,914 | 0,856 | 0,972 | 0,947 | 0,870 | 1 |
| IFI44L SIGLEC1 HERC6 SLC1A2 OLAH FAM20A | 0,878 | 0,868 | 0,888 | 0,914 | 0,857 | 0,972 | 0,946 | 0,872 | 1 |
| RSAD2 SIGLEC1 ISG15 IL1R2 FAM20A RETN | 0,878 | 0,869 | 0,888 | 0,906 | 0,842 | 0,971 | 0,962 | 0,907 | 1 |
| IFI27 IFIT1 HERC6 SLC1A2 IL1R2 MMP8 | 0,878 | 0,870 | 0,887 | 0,921 | 0,875 | 0,967 | 0,937 | 0,877 | 0,997 |
| IFI27 SIGLEC1 ISG15 SLC1A2 FAM20A MMP8 | 0,878 | 0,868 | 0,888 | 0,912 | 0,851 | 0,973 | 0,945 | 0,868 | 1 |
| IFIT1 IFI44L SIGLEC1 SLC1A2 FAM20A MMP8 | 0,878 | 0,869 | 0,887 | 0,911 | 0,852 | 0,970 | 0,938 | 0,864 | 1 |
| IFI44L SIGLEC1 OLAH FAM20A RETN MMP8 | 0,878 | 0,868 | 0,888 | 0,905 | 0,838 | 0,972 | 0,966 | 0,923 | 1 |
| IFI27 IFIT1 ISG15 SLC1A2 IL1R2 MMP8 | 0,878 | 0,870 | 0,886 | 0,922 | 0,877 | 0,968 | 0,937 | 0,877 | 0,997 |
| OAS1 SIGLEC1 SLC1A2 IL1R2 FAM20A RETN | 0,878 | 0,869 | 0,887 | 0,915 | 0,861 | 0,969 | 0,955 | 0,891 | 1 |
| RSAD2 IFIT1 SIGLEC1 OLAH FAM20A RETN | 0,878 | 0,869 | 0,888 | 0,903 | 0,834 | 0,973 | 0,953 | 0,902 | 1 |
| OAS1 IFIT1 SIGLEC1 ISG15 FAM20A RETN | 0,878 | 0,869 | 0,888 | 0,903 | 0,833 | 0,972 | 0,949 | 0,889 | 1 |
| OAS1 IFIT1 IFI44L SIGLEC1 ISG15 FAM20A | 0,878 | 0,869 | 0,888 | 0,902 | 0,832 | 0,972 | 0,938 | 0,870 | 1 |
| RSAD2 IFI27 OAS1 IFI44L HERC6 FAM20A | 0,878 | 0,868 | 0,888 | 0,910 | 0,848 | 0,972 | 0,930 | 0,851 | 1 |
| IFI27 OAS1 SLC1A2 IL1R2 RETN MMP8 | 0,878 | 0,869 | 0,887 | 0,924 | 0,875 | 0,973 | 0,932 | 0,864 | 0,999 |
| IFI27 OAS1 SIGLEC1 SLC1A2 RETN MMP8 | 0,878 | 0,869 | 0,887 | 0,915 | 0,857 | 0,972 | 0,948 | 0,880 | 1 |
| RSAD2 OAS1 IFIT1 IFI44L SIGLEC1 FAM20A | 0,878 | 0,869 | 0,887 | 0,902 | 0,832 | 0,973 | 0,939 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 OAS1 ISG15 IL1R2 MMP8 | 0,878 | 0,870 | 0,886 | 0,916 | 0,865 | 0,966 | 0,939 | 0,884 | 0,994 |
| SIGLEC1 ISG15 HERC6 OLAH FAM20A MMP8 | 0,878 | 0,868 | 0,888 | 0,903 | 0,835 | 0,970 | 0,964 | 0,916 | 1 |
| RSAD2 IFI27 ISG15 SLC1A2 IL1R2 RETN | 0,878 | 0,870 | 0,886 | 0,922 | 0,871 | 0,973 | 0,930 | 0,862 | 0,999 |
| RSAD2 IFI27 IFI44L FAM20A RETN MMP8 | 0,878 | 0,867 | 0,888 | 0,915 | 0,852 | 0,977 | 0,945 | 0,869 | 1 |
| IFI27 IFIT1 IFI44L SLC1A2 IL1R2 MMP8 | 0,878 | 0,870 | 0,886 | 0,921 | 0,874 | 0,968 | 0,939 | 0,880 | 0,998 |
| IFI27 OAS1 IFIT1 ISG15 FAM20A RETN | 0,878 | 0,868 | 0,887 | 0,909 | 0,847 | 0,972 | 0,943 | 0,865 | 1 |
| IFI27 IFI44L HERC6 SLC1A2 IL1R2 MMP8 | 0,878 | 0,869 | 0,887 | 0,920 | 0,874 | 0,966 | 0,927 | 0,861 | 0,993 |
| RSAD2 OAS1 SIGLEC1 SLC1A2 IL1R2 FAM20A | 0,878 | 0,868 | 0,887 | 0,915 | 0,860 | 0,970 | 0,955 | 0,894 | 1 |
| IFI27 OAS1 IFI44L ISG15 IL1R2 MMP8 | 0,878 | 0,870 | 0,886 | 0,914 | 0,863 | 0,965 | 0,932 | 0,873 | 0,990 |
| IFI27 OAS1 IFIT1 SIGLEC1 HERC6 MMP8 | 0,878 | 0,869 | 0,886 | 0,907 | 0,852 | 0,962 | 0,904 | 0,828 | 0,981 |
| SIGLEC1 ISG15 HERC6 SLC1A2 OLAH FAM20A | 0,878 | 0,868 | 0,888 | 0,914 | 0,857 | 0,970 | 0,946 | 0,872 | 1 |
| RSAD2 IFI27 ISG15 HERC6 SLC1A2 IL1R2 | 0,878 | 0,870 | 0,886 | 0,921 | 0,876 | 0,967 | 0,928 | 0,861 | 0,995 |
| RSAD2 IFI44L SIGLEC1 SLC1A2 IL1R2 FAM20A | 0,878 | 0,869 | 0,887 | 0,916 | 0,862 | 0,971 | 0,954 | 0,890 | 1 |
| IFI44L ISG15 HERC6 IL1R2 OLAH FAM20A | 0,878 | 0,868 | 0,887 | 0,905 | 0,844 | 0,967 | 0,939 | 0,867 | 1 |
| IFI27 IFI44L SIGLEC1 SLC1A2 FAM20A MMP8 | 0,878 | 0,868 | 0,887 | 0,911 | 0,849 | 0,972 | 0,946 | 0,869 | 1 |
| SIGLEC1 HERC6 SLC1A2 IL1R2 FAM20A RETN | 0,878 | 0,868 | 0,887 | 0,918 | 0,865 | 0,971 | 0,948 | 0,875 | 1 |
| RSAD2 IFI27 IFI44L HERC6 SLC1A2 IL1R2 | 0,878 | 0,869 | 0,886 | 0,919 | 0,873 | 0,966 | 0,928 | 0,861 | 0,995 |
| OAS1 IFIT1 IFI44L SIGLEC1 FAM20A MMP8 | 0,878 | 0,868 | 0,887 | 0,902 | 0,832 | 0,972 | 0,939 | 0,870 | 1 |
| OAS1 IFI44L SIGLEC1 SLC1A2 OLAH FAM20A | 0,878 | 0,868 | 0,887 | 0,913 | 0,854 | 0,971 | 0,948 | 0,890 | 1 |
| RSAD2 OAS1 SIGLEC1 SLC1A2 OLAH FAM20A | 0,878 | 0,868 | 0,887 | 0,915 | 0,857 | 0,972 | 0,950 | 0,892 | 1 |
| IFI27 OAS1 IFI44L HERC6 SLC1A2 IL1R2 | 0,878 | 0,869 | 0,886 | 0,921 | 0,876 | 0,967 | 0,926 | 0,859 | 0,993 |
| RSAD2 IFI27 IFIT1 ISG15 SLC1A2 IL1R2 | 0,878 | 0,870 | 0,886 | 0,922 | 0,876 | 0,968 | 0,936 | 0,875 | 0,997 |

(suite)

| Modèle | AUC modèle | IC 95% | | AUC Train | IC 95% Train | | AUC Test | IC 95% Test | |
|---|---|---|---|---|---|---|---|---|---|
| OAS1 IFI44L SIGLEC1 SLC1A2 IL1R2 FAM20A | 0,878 | 0,868 | 0,887 | 0,916 | 0,861 | 0,970 | 0,954 | 0,891 | 1 |
| IFI27 IFIT1 IFI44L ISG15 SLC1A2 IL1R2 | 0,877 | 0,869 | 0,886 | 0,923 | 0,876 | 0,969 | 0,934 | 0,873 | 0,994 |
| IFI27 IFIT1 IFI44L SIGLEC1 RETN MMP8 | 0,877 | 0,868 | 0,887 | 0,909 | 0,848 | 0,971 | 0,953 | 0,897 | 1 |
| RSAD2 IFI44L SIGLEC1 IL1R2 FAM20A RETN | 0,877 | 0,868 | 0,887 | 0,906 | 0,842 | 0,970 | 0,961 | 0,906 | 1 |
| IFI44L SIGLEC1 ISG15 HERC6 OLAH FAM20A | 0,877 | 0,868 | 0,887 | 0,903 | 0,836 | 0,970 | 0,953 | 0,894 | 1 |
| IFI27 IFIT1 ISG15 FAM20A RETN MMP8 | 0,877 | 0,867 | 0,888 | 0,912 | 0,850 | 0,975 | 0,943 | 0,867 | 1 |
| RSAD2 IFI27 OAS1 SLC1A2 IL1R2 MMP8 | 0,877 | 0,869 | 0,886 | 0,920 | 0,872 | 0,967 | 0,930 | 0,863 | 0,998 |
| IFI27 OAS1 ISG15 SLC1A2 IL1R2 RETN | 0,877 | 0,869 | 0,886 | 0,922 | 0,871 | 0,972 | 0,926 | 0,856 | 0,997 |
| IFI27 IFIT1 SIGLEC1 ISG15 RETN MMP8 | 0,877 | 0,868 | 0,887 | 0,908 | 0,846 | 0,970 | 0,957 | 0,902 | 1 |
| IFI27 SIGLEC1 ISG15 HERC6 SLC1A2 FAM20A | 0,877 | 0,867 | 0,887 | 0,911 | 0,851 | 0,972 | 0,947 | 0,870 | 1 |
| OAS1 SIGLEC1 ISG15 SLC1A2 OLAH FAM20A | 0,877 | 0,868 | 0,887 | 0,912 | 0,854 | 0,971 | 0,950 | 0,891 | 1 |
| IFI27 OAS1 IFI44L SIGLEC1 RETN MMP8 | 0,877 | 0,868 | 0,887 | 0,911 | 0,850 | 0,972 | 0,953 | 0,893 | 1 |
| OAS1 SIGLEC1 ISG15 SLC1A2 IL1R2 FAM20A | 0,877 | 0,868 | 0,887 | 0,914 | 0,859 | 0,969 | 0,953 | 0,888 | 1 |
| IFI27 IFIT1 ISG15 HERC6 SLC1A2 IL1R2 | 0,877 | 0,869 | 0,886 | 0,923 | 0,877 | 0,968 | 0,937 | 0,877 | 0,997 |
| IFI44L ISG15 SLC1A2 OLAH FAM20A MMP8 | 0,877 | 0,868 | 0,887 | 0,911 | 0,854 | 0,969 | 0,925 | 0,850 | 1 |
| RSAD2 IFI27 IFIT1 IFI44L HERC6 FAM20A | 0,877 | 0,867 | 0,887 | 0,907 | 0,844 | 0,970 | 0,925 | 0,844 | 1 |
| IFI27 OAS1 IFIT1 IFI44L SLC1A2 IL1R2 | 0,877 | 0,869 | 0,886 | 0,921 | 0,874 | 0,968 | 0,937 | 0,877 | 0,997 |
| RSAD2 IFI27 IFIT1 HERC6 FAM20A MMP8 | 0,877 | 0,868 | 0,887 | 0,911 | 0,852 | 0,969 | 0,926 | 0,842 | 1 |
| RSAD2 IFI27 ISG15 HERC6 FAM20A MMP8 | 0,877 | 0,867 | 0,887 | 0,912 | 0,854 | 0,970 | 0,926 | 0,845 | 1 |
| RSAD2 IFI27 OAS1 ISG15 FAM20A RETN | 0,877 | 0,867 | 0,887 | 0,910 | 0,849 | 0,971 | 0,940 | 0,862 | 1 |
| IFI27 OAS1 ISG15 HERC6 FAM20A MMP8 | 0,877 | 0,867 | 0,887 | 0,913 | 0,856 | 0,970 | 0,927 | 0,846 | 1 |
| IFIT1 IFI44L SLC1A2 OLAH FAM20A MMP8 | 0,877 | 0,868 | 0,886 | 0,913 | 0,856 | 0,970 | 0,924 | 0,848 | 1,000 |

(suite)

| Modèle | AUC modèle | IC 95% | | AUC Train | IC 95% Train | | AUC Test | IC 95% Test | |
|---|---|---|---|---|---|---|---|---|---|
| SIGLEC1 ISG15 OLAH FAM20A RETN MMP8 | 0,877 | 0,867 | 0,887 | 0,904 | 0,837 | 0,972 | 0,970 | 0,929 | 1 |
| IFI27 OAS1 IFIT1 HERC6 FAM20A MMP8 | 0,877 | 0,867 | 0,887 | 0,911 | 0,853 | 0,970 | 0,925 | 0,842 | 1 |
| IFI27 OAS1 IFI44L HERC6 FAM20A MMP8 | 0,877 | 0,867 | 0,887 | 0,910 | 0,850 | 0,971 | 0,926 | 0,845 | 1 |
| RSAD2 IFI27 ISG15 SLC1A2 IL1R2 MMP8 | 0,877 | 0,869 | 0,885 | 0,921 | 0,874 | 0,968 | 0,930 | 0,866 | 0,995 |
| RSAD2 OAS1 IFIT1 SIGLEC1 FAM20A RETN | 0,877 | 0,867 | 0,887 | 0,903 | 0,834 | 0,972 | 0,949 | 0,889 | 1 |
| OAS1 IFIT1 IFI44L SIGLEC1 SLC1A2 FAM20A | 0,877 | 0,868 | 0,886 | 0,912 | 0,853 | 0,971 | 0,936 | 0,861 | 1 |
| RSAD2 IFI44L SIGLEC1 SLC1A2 OLAH FAM20A | 0,877 | 0,867 | 0,887 | 0,915 | 0,857 | 0,973 | 0,945 | 0,885 | 1 |
| RSAD2 IFI27 IFI44L SIGLEC1 ISG15 RETN | 0,877 | 0,868 | 0,886 | 0,908 | 0,846 | 0,969 | 0,950 | 0,892 | 1 |
| RSAD2 OAS1 SIGLEC1 HERC6 OLAH FAM20A | 0,877 | 0,867 | 0,887 | 0,906 | 0,844 | 0,969 | 0,948 | 0,891 | 1 |
| OAS1 IFIT1 SIGLEC1 ISG15 FAM20A MMP8 | 0,877 | 0,867 | 0,886 | 0,901 | 0,832 | 0,971 | 0,946 | 0,877 | 1 |
| RSAD2 SIGLEC1 ISG15 HERC6 FAM20A RETN | 0,877 | 0,867 | 0,886 | 0,910 | 0,849 | 0,971 | 0,947 | 0,881 | 1 |
| OAS1 SIGLEC1 HERC6 FAM20A RETN MMP8 | 0,877 | 0,867 | 0,886 | 0,908 | 0,842 | 0,973 | 0,952 | 0,889 | 1 |
| IFI27 OAS1 IFI44L FAM20A RETN MMP8 | 0,877 | 0,866 | 0,887 | 0,914 | 0,851 | 0,976 | 0,947 | 0,871 | 1 |
| IFI27 IFIT1 IFI44L HERC6 SLC1A2 IL1R2 | 0,877 | 0,868 | 0,885 | 0,923 | 0,877 | 0,969 | 0,936 | 0,876 | 0,995 |
| RSAD2 IFI27 OAS1 IFI44L SLC1A2 IL1R2 | 0,877 | 0,868 | 0,885 | 0,921 | 0,874 | 0,967 | 0,933 | 0,866 | 0,999 |
| RSAD2 OAS1 IFIT1 SIGLEC1 ISG15 FAM20A | 0,877 | 0,867 | 0,886 | 0,900 | 0,829 | 0,970 | 0,946 | 0,879 | 1 |
| IFI44L ISG15 SLC1A2 IL1R2 OLAH FAM20A | 0,877 | 0,868 | 0,886 | 0,913 | 0,857 | 0,969 | 0,928 | 0,852 | 1 |
| IFI27 SIGLEC1 HERC6 SLC1A2 FAM20A MMP8 | 0,877 | 0,867 | 0,887 | 0,912 | 0,851 | 0,973 | 0,946 | 0,869 | 1 |
| RSAD2 IFI27 IFIT1 IFI44L SLC1A2 IL1R2 | 0,877 | 0,869 | 0,885 | 0,921 | 0,874 | 0,968 | 0,938 | 0,879 | 0,997 |
| RSAD2 IFI27 OAS1 IFIT1 FAM20A RETN | 0,877 | 0,867 | 0,886 | 0,911 | 0,849 | 0,972 | 0,943 | 0,867 | 1 |
| SIGLEC1 ISG15 SLC1A2 OLAH FAM20A RETN | 0,877 | 0,867 | 0,886 | 0,914 | 0,856 | 0,971 | 0,949 | 0,887 | 1 |
| RSAD2 OAS1 IFIT1 SIGLEC1 FAM20A MMP8 | 0,877 | 0,867 | 0,886 | 0,901 | 0,832 | 0,971 | 0,949 | 0,883 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1 SIGLEC1 ISG15 FAM20A RETN MMP8 | 0,877 | 0,867 | 0,886 | 0,906 | 0,840 | 0,972 | 0,947 | 0,885 | 1 |
| RSAD2 SIGLEC1 ISG15 SLC1A2 OLAH FAM20A | 0,877 | 0,867 | 0,886 | 0,912 | 0,854 | 0,971 | 0,950 | 0,891 | 1 |
| IFI44L HERC6 IL1R2 OLAH FAM20A MMP8 | 0,877 | 0,867 | 0,886 | 0,909 | 0,850 | 0,968 | 0,940 | 0,865 | 1 |
| IFI27 IFI44L ISG15 SLC1A2 FAM20A RETN | 0,877 | 0,867 | 0,886 | 0,911 | 0,850 | 0,973 | 0,941 | 0,861 | 1 |
| IFI27 SIGLEC1 ISG15 HERC6 RETN MMP8 | 0,877 | 0,866 | 0,887 | 0,905 | 0,840 | 0,970 | 0,960 | 0,910 | 1 |
| RSAD2 IFI27 IFIT1 ISG15 FAM20A RETN | 0,877 | 0,867 | 0,886 | 0,911 | 0,849 | 0,972 | 0,942 | 0,865 | 1 |
| IFIT1 SIGLEC1 ISG15 OLAH FAM20A RETN | 0,876 | 0,867 | 0,886 | 0,903 | 0,833 | 0,972 | 0,948 | 0,893 | 1 |
| IFI27 IFI44L ISG15 HERC6 SLC1A2 IL1R2 | 0,876 | 0,868 | 0,885 | 0,921 | 0,876 | 0,967 | 0,929 | 0,864 | 0,995 |
| IFI27 IFI44L SIGLEC1 ISG15 RETN MMP8 | 0,876 | 0,867 | 0,886 | 0,906 | 0,843 | 0,969 | 0,951 | 0,894 | 1 |
| RSAD2 SIGLEC1 ISG15 HERC6 OLAH FAM20A | 0,876 | 0,867 | 0,886 | 0,909 | 0,847 | 0,971 | 0,949 | 0,892 | 1 |
| RSAD2 IFIT1 IFI44L SIGLEC1 ISG15 FAM20A | 0,876 | 0,867 | 0,885 | 0,899 | 0,828 | 0,969 | 0,940 | 0,877 | 1 |
| IFI27 IFIT1 SIGLEC1 ISG15 SLC1A2 RETN | 0,876 | 0,868 | 0,885 | 0,915 | 0,860 | 0,971 | 0,954 | 0,897 | 1 |
| RSAD2 IFI27 IFI44L SLC1A2 IL1R2 MMP8 | 0,876 | 0,868 | 0,884 | 0,921 | 0,873 | 0,968 | 0,933 | 0,868 | 0,997 |
| RSAD2 IFI27 OAS1 HERC6 FAM20A MMP8 | 0,876 | 0,866 | 0,886 | 0,912 | 0,853 | 0,970 | 0,927 | 0,846 | 1 |
| IFI27 ISG15 HERC6 SLC1A2 IL1R2 MMP8 | 0,876 | 0,868 | 0,885 | 0,922 | 0,876 | 0,967 | 0,928 | 0,863 | 0,994 |
| OAS1 SIGLEC1 SLC1A2 OLAH FAM20A RETN | 0,876 | 0,866 | 0,886 | 0,913 | 0,855 | 0,972 | 0,952 | 0,896 | 1 |
| ISG15 HERC6 IL1R2 OLAH FAM20A MMP8 | 0,876 | 0,867 | 0,886 | 0,908 | 0,850 | 0,966 | 0,935 | 0,855 | 1 |
| IFI44L ISG15 HERC6 IL1R2 FAM20A MMP8 | 0,876 | 0,867 | 0,885 | 0,910 | 0,850 | 0,969 | 0,932 | 0,847 | 1 |
| RSAD2 SIGLEC1 ISG15 OLAH FAM20A MMP8 | 0,876 | 0,866 | 0,886 | 0,904 | 0,838 | 0,971 | 0,962 | 0,920 | 1 |
| RSAD2 OAS1 ISG15 HERC6 IL1R2 FAM20A | 0,876 | 0,867 | 0,886 | 0,904 | 0,844 | 0,965 | 0,933 | 0,850 | 1 |
| IFIT1 IFI44L HERC6 SLC1A2 OLAH FAM20A | 0,876 | 0,866 | 0,886 | 0,916 | 0,862 | 0,969 | 0,916 | 0,830 | 1 |
| RSAD2 IFIT1 IFI44L SIGLEC1 SLC1A2 FAM20A | 0,876 | 0,867 | 0,885 | 0,910 | 0,851 | 0,970 | 0,936 | 0,861 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 OAS1 ISG15 SLC1A2 IL1R2 | 0,876 | 0,868 | 0,884 | 0,922 | 0,876 | 0,968 | 0,929 | 0,861 | 0,998 |
| RSAD2 SIGLEC1 SLC1A2 OLAH FAM20A RETN | 0,876 | 0,866 | 0,886 | 0,914 | 0,856 | 0,972 | 0,951 | 0,894 | 1 |
| IFIT1 IFI44L SIGLEC1 SLC1A2 OLAH MMP8 | 0,876 | 0,867 | 0,885 | 0,911 | 0,855 | 0,967 | 0,953 | 0,909 | 0,998 |
| IFI27 IFI44L SLC1A2 FAM20A RETN MMP8 | 0,876 | 0,866 | 0,886 | 0,912 | 0,851 | 0,974 | 0,941 | 0,862 | 1 |
| OAS1 HERC6 IL1R2 OLAH FAM20A MMP8 | 0,876 | 0,866 | 0,885 | 0,909 | 0,849 | 0,968 | 0,937 | 0,859 | 1 |
| OAS1 SIGLEC1 ISG15 HERC6 FAM20A RETN | 0,876 | 0,866 | 0,886 | 0,907 | 0,845 | 0,969 | 0,952 | 0,889 | 1 |
| OAS1 IFIT1 ISG15 HERC6 IL1R2 FAM20A | 0,876 | 0,866 | 0,885 | 0,904 | 0,843 | 0,965 | 0,933 | 0,851 | 1 |
| RSAD2 IFI27 ISG15 HERC6 SLC1A2 RETN | 0,876 | 0,866 | 0,885 | 0,914 | 0,857 | 0,972 | 0,937 | 0,870 | 1 |
| RSAD2 IFI27 IFI44L HERC6 SLC1A2 RETN | 0,876 | 0,866 | 0,885 | 0,914 | 0,855 | 0,972 | 0,936 | 0,868 | 1 |
| OAS1 SIGLEC1 HERC6 OLAH FAM20A MMP8 | 0,876 | 0,866 | 0,886 | 0,906 | 0,843 | 0,969 | 0,958 | 0,907 | 1 |
| IFIT1 SIGLEC1 HERC6 IL1R2 RETN MMP8 | 0,876 | 0,866 | 0,885 | 0,904 | 0,841 | 0,966 | 0,963 | 0,925 | 1 |
| RSAD2 IFI27 IFIT1 SIGLEC1 SLC1A2 RETN | 0,876 | 0,867 | 0,884 | 0,915 | 0,858 | 0,971 | 0,953 | 0,896 | 1 |
| IFIT1 IFI44L ISG15 HERC6 IL1R2 FAM20A | 0,876 | 0,867 | 0,885 | 0,908 | 0,848 | 0,968 | 0,932 | 0,851 | 1 |
| OAS1 IFIT1 SIGLEC1 ISG15 SLC1A2 FAM20A | 0,876 | 0,866 | 0,885 | 0,907 | 0,846 | 0,968 | 0,943 | 0,871 | 1 |
| OAS1 IFI44L SIGLEC1 OLAH FAM20A MMP8 | 0,876 | 0,865 | 0,886 | 0,901 | 0,834 | 0,969 | 0,963 | 0,922 | 1 |
| OAS1 IFIT1 HERC6 IL1R2 FAM20A MMP8 | 0,876 | 0,866 | 0,885 | 0,905 | 0,844 | 0,967 | 0,939 | 0,860 | 1 |
| SIGLEC1 ISG15 HERC6 FAM20A RETN MMP8 | 0,875 | 0,865 | 0,886 | 0,904 | 0,835 | 0,972 | 0,957 | 0,891 | 1 |
| RSAD2 IFI27 IFI44L ISG15 SLC1A2 IL1R2 | 0,875 | 0,868 | 0,883 | 0,922 | 0,876 | 0,968 | 0,930 | 0,865 | 0,996 |
| RSAD2 OAS1 HERC6 IL1R2 FAM20A MMP8 | 0,875 | 0,866 | 0,885 | 0,904 | 0,843 | 0,966 | 0,934 | 0,853 | 1 |
| IFI44L SIGLEC1 SLC1A2 IL1R2 OLAH MMP8 | 0,875 | 0,867 | 0,884 | 0,912 | 0,858 | 0,967 | 0,966 | 0,931 | 1 |
| RSAD2 OAS1 HERC6 IL1R2 OLAH FAM20A | 0,875 | 0,866 | 0,885 | 0,906 | 0,845 | 0,968 | 0,938 | 0,864 | 1 |
| IFI27 IFI44L SIGLEC1 ISG15 SLC1A2 RETN | 0,875 | 0,867 | 0,884 | 0,914 | 0,857 | 0,971 | 0,951 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 IFI44L SIGLEC1 SLC1A2 RETN | 0,875 | 0,867 | 0,884 | 0,916 | 0,860 | 0,972 | 0,952 | 0,894 | 1 |
| IFI44L SIGLEC1 HERC6 FAM20A RETN MMP8 | 0,875 | 0,865 | 0,885 | 0,907 | 0,840 | 0,974 | 0,955 | 0,888 | 1 |
| RSAD2 IFI27 SIGLEC1 ISG15 RETN MMP8 | 0,875 | 0,866 | 0,885 | 0,912 | 0,852 | 0,973 | 0,957 | 0,902 | 1 |
| IFI44L ISG15 HERC6 IL1R2 FAM20A RETN | 0,875 | 0,866 | 0,885 | 0,908 | 0,848 | 0,969 | 0,942 | 0,868 | 1 |
| RSAD2 IFI27 IFIT1 SIGLEC1 RETN MMP8 | 0,875 | 0,866 | 0,885 | 0,910 | 0,849 | 0,971 | 0,955 | 0,900 | 1 |
| IFIT1 IFI44L SIGLEC1 SLC1A2 FAM20A RETN | 0,875 | 0,866 | 0,885 | 0,913 | 0,854 | 0,972 | 0,936 | 0,863 | 1 |
| IFIT1 ISG15 HERC6 IL1R2 OLAH FAM20A | 0,875 | 0,866 | 0,885 | 0,906 | 0,846 | 0,966 | 0,935 | 0,858 | 1 |
| IFI44L SIGLEC1 ISG15 SLC1A2 OLAH MMP8 | 0,875 | 0,867 | 0,884 | 0,912 | 0,857 | 0,968 | 0,952 | 0,907 | 0,997 |
| IFIT1 IFI44L HERC6 IL1R2 OLAH FAM20A | 0,875 | 0,866 | 0,884 | 0,906 | 0,845 | 0,966 | 0,934 | 0,856 | 1 |
| IFI27 IFIT1 IFI44L SLC1A2 FAM20A RETN | 0,875 | 0,865 | 0,885 | 0,911 | 0,850 | 0,973 | 0,937 | 0,855 | 1 |
| RSAD2 IFI44L SIGLEC1 HERC6 OLAH FAM20A | 0,875 | 0,866 | 0,885 | 0,907 | 0,844 | 0,970 | 0,946 | 0,888 | 1 |
| RSAD2 IFIT1 SIGLEC1 FAM20A RETN MMP8 | 0,875 | 0,866 | 0,885 | 0,904 | 0,837 | 0,972 | 0,948 | 0,886 | 1 |
| RSAD2 IFIT1 IFI44L SIGLEC1 FAM20A RETN | 0,875 | 0,866 | 0,885 | 0,901 | 0,831 | 0,971 | 0,945 | 0,885 | 1 |
| IFIT1 HERC6 IL1R2 OLAH FAM20A MMP8 | 0,875 | 0,866 | 0,885 | 0,907 | 0,847 | 0,966 | 0,946 | 0,875 | 1 |
| RSAD2 IFI27 IFIT1 HERC6 SLC1A2 RETN | 0,875 | 0,866 | 0,885 | 0,912 | 0,854 | 0,971 | 0,938 | 0,871 | 1 |
| RSAD2 OAS1 IL1R2 OLAH FAM20A MMP8 | 0,875 | 0,866 | 0,884 | 0,906 | 0,844 | 0,967 | 0,935 | 0,856 | 1 |
| RSAD2 ISG15 HERC6 IL1R2 OLAH FAM20A | 0,875 | 0,865 | 0,885 | 0,905 | 0,846 | 0,965 | 0,934 | 0,857 | 1 |
| RSAD2 IFI44L ISG15 HERC6 IL1R2 FAM20A | 0,875 | 0,866 | 0,884 | 0,909 | 0,851 | 0,967 | 0,934 | 0,855 | 1 |
| IFI27 OAS1 IFIT1 FAM20A RETN MMP8 | 0,875 | 0,865 | 0,885 | 0,911 | 0,849 | 0,974 | 0,945 | 0,869 | 1 |
| IFI27 OAS1 IFI44L SLC1A2 IL1R2 MMP8 | 0,875 | 0,867 | 0,883 | 0,922 | 0,876 | 0,968 | 0,927 | 0,860 | 0,994 |
| RSAD2 OAS1 IFIT1 SIGLEC1 SLC1A2 FAM20A | 0,875 | 0,865 | 0,885 | 0,907 | 0,846 | 0,968 | 0,943 | 0,871 | 1 |
| RSAD2 IFI44L SIGLEC1 OLAH FAM20A MMP8 | 0,875 | 0,865 | 0,885 | 0,904 | 0,838 | 0,971 | 0,964 | 0,924 | 1 |

(suite)

| Modèle | AUC modèle | IC 95% | | AUC Train | IC 95% Train | | AUC Test | IC 95% Test | |
|---|---|---|---|---|---|---|---|---|---|
| ISG15 HERC6 IL1R2 FAM20A RETN MMP8 | 0,875 | 0,866 | 0,885 | 0,910 | 0,850 | 0,969 | 0,932 | 0,848 | 1 |
| RSAD2 IFI27 OAS1 FAM20A RETN MMP8 | 0,875 | 0,865 | 0,885 | 0,908 | 0,845 | 0,970 | 0,942 | 0,865 | 1 |
| RSAD2 SIGLEC1 HERC6 OLAH FAM20A RETN | 0,875 | 0,865 | 0,885 | 0,907 | 0,844 | 0,969 | 0,947 | 0,889 | 1 |
| IFI27 OAS1 SIGLEC1 HERC6 SLC1A2 MMP8 | 0,875 | 0,866 | 0,884 | 0,911 | 0,857 | 0,965 | 0,925 | 0,856 | 0,994 |
| IFI27 IFIT1 IFI44L SIGLEC1 HERC6 MMP8 | 0,875 | 0,866 | 0,884 | 0,905 | 0,847 | 0,963 | 0,908 | 0,833 | 0,982 |
| RSAD2 IFIT1 IFI44L SIGLEC1 FAM20A MMP8 | 0,875 | 0,865 | 0,885 | 0,901 | 0,832 | 0,971 | 0,938 | 0,869 | 1 |
| OAS1 IFIT1 SIGLEC1 SLC1A2 FAM20A MMP8 | 0,875 | 0,865 | 0,884 | 0,908 | 0,847 | 0,969 | 0,946 | 0,872 | 1 |
| OAS1 SIGLEC1 OLAH FAM20A RETN MMP8 | 0,875 | 0,865 | 0,885 | 0,907 | 0,842 | 0,972 | 0,967 | 0,928 | 1 |
| IFI27 IFIT1 SIGLEC1 ISG15 HERC6 MMP8 | 0,875 | 0,866 | 0,884 | 0,905 | 0,847 | 0,963 | 0,908 | 0,832 | 0,983 |
| IFIT1 ISG15 HERC6 IL1R2 FAM20A RETN | 0,875 | 0,865 | 0,884 | 0,906 | 0,846 | 0,966 | 0,934 | 0,851 | 1 |
| IFI44L SIGLEC1 ISG15 HERC6 FAM20A RETN | 0,875 | 0,865 | 0,885 | 0,904 | 0,837 | 0,970 | 0,955 | 0,890 | 1 |
| RSAD2 IFI27 IFI44L SIGLEC1 SLC1A2 RETN | 0,875 | 0,866 | 0,884 | 0,914 | 0,858 | 0,970 | 0,952 | 0,896 | 1 |
| OAS1 IFIT1 SIGLEC1 HERC6 IL1R2 RETN | 0,875 | 0,866 | 0,884 | 0,905 | 0,843 | 0,968 | 0,952 | 0,907 | 0,997 |
| OAS1 SIGLEC1 HERC6 IL1R2 RETN MMP8 | 0,875 | 0,865 | 0,884 | 0,902 | 0,838 | 0,966 | 0,967 | 0,933 | 1 |
| OAS1 ISG15 HERC6 IL1R2 FAM20A MMP8 | 0,875 | 0,865 | 0,884 | 0,907 | 0,848 | 0,967 | 0,932 | 0,849 | 1 |
| RSAD2 HERC6 IL1R2 OLAH FAM20A MMP8 | 0,875 | 0,865 | 0,884 | 0,906 | 0,846 | 0,966 | 0,934 | 0,854 | 1 |
| IFI27 IFIT1 IFI44L ISG15 HERC6 FAM20A | 0,875 | 0,865 | 0,885 | 0,902 | 0,838 | 0,966 | 0,924 | 0,840 | 1 |
| IFI27 IFI44L ISG15 HERC6 FAM20A MMP8 | 0,875 | 0,865 | 0,885 | 0,907 | 0,845 | 0,969 | 0,932 | 0,851 | 1 |
| SIGLEC1 HERC6 SLC1A2 OLAH FAM20A RETN | 0,875 | 0,864 | 0,885 | 0,914 | 0,856 | 0,971 | 0,947 | 0,874 | 1 |
| RSAD2 IFIT1 SIGLEC1 ISG15 FAM20A RETN | 0,875 | 0,865 | 0,884 | 0,902 | 0,833 | 0,971 | 0,948 | 0,888 | 1 |
| IFI44L HERC6 SLC1A2 IL1R2 OLAH FAM20A | 0,875 | 0,865 | 0,885 | 0,914 | 0,860 | 0,968 | 0,929 | 0,846 | 1 |
| IFIT1 SIGLEC1 HERC6 SLC1A2 OLAH MMP8 | 0,875 | 0,865 | 0,885 | 0,913 | 0,858 | 0,968 | 0,947 | 0,898 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 SLC1A2 IL1R2 OLAH MMP8 | 0,875 | 0,866 | 0,884 | 0,912 | 0,858 | 0,966 | 0,966 | 0,931 | 1 |
| IFI27 OAS1 ISG15 SLC1A2 IL1R2 MMP8 | 0,875 | 0,867 | 0,883 | 0,921 | 0,875 | 0,967 | 0,923 | 0,853 | 0,992 |
| OAS1 IFIT1 SIGLEC1 SLC1A2 FAM20A RETN | 0,875 | 0,865 | 0,884 | 0,910 | 0,849 | 0,970 | 0,943 | 0,870 | 1 |
| OAS1 SIGLEC1 ISG15 HERC6 OLAH FAM20A | 0,875 | 0,865 | 0,885 | 0,906 | 0,843 | 0,969 | 0,955 | 0,902 | 1 |
| IFI44L SIGLEC1 HERC6 OLAH FAM20A RETN | 0,875 | 0,865 | 0,885 | 0,904 | 0,837 | 0,970 | 0,954 | 0,893 | 1 |
| RSAD2 ISG15 HERC6 IL1R2 FAM20A RETN | 0,875 | 0,865 | 0,884 | 0,906 | 0,846 | 0,966 | 0,934 | 0,852 | 1 |
| OAS1 IFI44L SIGLEC1 HERC6 OLAH FAM20A | 0,875 | 0,865 | 0,884 | 0,905 | 0,841 | 0,969 | 0,954 | 0,899 | 1 |
| OAS1 SIGLEC1 ISG15 OLAH FAM20A MMP8 | 0,875 | 0,864 | 0,885 | 0,901 | 0,833 | 0,970 | 0,964 | 0,922 | 1 |
| ISG15 HERC6 IL1R2 OLAH FAM20A RETN | 0,874 | 0,865 | 0,884 | 0,906 | 0,845 | 0,967 | 0,937 | 0,862 | 1 |
| IFI27 SIGLEC1 ISG15 HERC6 SLC1A2 RETN | 0,874 | 0,865 | 0,884 | 0,910 | 0,848 | 0,972 | 0,952 | 0,895 | 1 |
| RSAD2 IFIT1 ISG15 HERC6 IL1R2 FAM20A | 0,874 | 0,865 | 0,884 | 0,906 | 0,846 | 0,966 | 0,934 | 0,855 | 1 |
| IFIT1 SIGLEC1 SLC1A2 FAM20A RETN MMP8 | 0,874 | 0,865 | 0,884 | 0,914 | 0,856 | 0,971 | 0,948 | 0,878 | 1 |
| IFIT1 IFI44L HERC6 IL1R2 FAM20A MMP8 | 0,874 | 0,865 | 0,883 | 0,908 | 0,849 | 0,968 | 0,934 | 0,851 | 1 |
| OAS1 IFI44L ISG15 HERC6 IL1R2 FAM20A | 0,874 | 0,865 | 0,884 | 0,907 | 0,847 | 0,967 | 0,938 | 0,861 | 1 |
| RSAD2 IFI27 IFI44L SIGLEC1 RETN MMP8 | 0,874 | 0,865 | 0,884 | 0,910 | 0,849 | 0,971 | 0,952 | 0,895 | 1 |
| RSAD2 SIGLEC1 OLAH FAM20A RETN MMP8 | 0,874 | 0,865 | 0,884 | 0,908 | 0,844 | 0,972 | 0,963 | 0,920 | 1 |
| OAS1 ISG15 HERC6 IL1R2 FAM20A RETN | 0,874 | 0,864 | 0,884 | 0,907 | 0,847 | 0,967 | 0,934 | 0,852 | 1 |
| RSAD2 IFI27 IFIT1 FAM20A RETN MMP8 | 0,874 | 0,864 | 0,884 | 0,912 | 0,850 | 0,975 | 0,943 | 0,866 | 1 |
| RSAD2 IFI27 SIGLEC1 ISG15 SLC1A2 RETN | 0,874 | 0,865 | 0,883 | 0,914 | 0,857 | 0,971 | 0,951 | 0,893 | 1 |
| RSAD2 IFIT1 SIGLEC1 ISG15 SLC1A2 FAM20A | 0,874 | 0,865 | 0,884 | 0,907 | 0,846 | 0,968 | 0,946 | 0,874 | 1 |
| IFI27 IFIT1 SIGLEC1 SLC1A2 RETN MMP8 | 0,874 | 0,865 | 0,883 | 0,915 | 0,859 | 0,971 | 0,955 | 0,900 | 1 |
| RSAD2 SIGLEC1 ISG15 HERC6 SLC1A2 FAM20A | 0,874 | 0,864 | 0,884 | 0,910 | 0,853 | 0,967 | 0,940 | 0,864 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 SIGLEC1 HERC6 IL1R2 MMP8 | 0,874 | 0,866 | 0,883 | 0,904 | 0,846 | 0,962 | 0,958 | 0,916 | 1,000 |
| OAS1 IFIT1 SIGLEC1 HERC6 SLC1A2 OLAH | 0,874 | 0,865 | 0,884 | 0,912 | 0,857 | 0,968 | 0,945 | 0,895 | 0,995 |
| OAS1 IFIT1 SIGLEC1 ISG15 HERC6 RETN | 0,874 | 0,865 | 0,884 | 0,902 | 0,839 | 0,966 | 0,932 | 0,873 | 0,990 |
| IFI27 OAS1 IFI44L ISG15 SLC1A2 IL1R2 | 0,874 | 0,866 | 0,883 | 0,922 | 0,877 | 0,968 | 0,930 | 0,866 | 0,994 |
| IFI27 OAS1 ISG15 FAM20A RETN MMP8 | 0,874 | 0,864 | 0,884 | 0,910 | 0,848 | 0,972 | 0,943 | 0,866 | 1 |
| IFIT1 ISG15 HERC6 IL1R2 FAM20A MMP8 | 0,874 | 0,865 | 0,884 | 0,906 | 0,846 | 0,966 | 0,937 | 0,859 | 1 |
| IFI44L SIGLEC1 HERC6 IL1R2 RETN MMP8 | 0,874 | 0,865 | 0,884 | 0,901 | 0,837 | 0,965 | 0,963 | 0,925 | 1 |
| RSAD2 OAS1 SIGLEC1 ISG15 OLAH FAM20A | 0,874 | 0,864 | 0,884 | 0,898 | 0,827 | 0,969 | 0,959 | 0,911 | 1 |
| IFIT1 SIGLEC1 ISG15 SLC1A2 OLAH MMP8 | 0,874 | 0,865 | 0,883 | 0,913 | 0,857 | 0,968 | 0,952 | 0,907 | 0,997 |
| IFI27 OAS1 IFI44L SIGLEC1 HERC6 MMP8 | 0,874 | 0,866 | 0,883 | 0,906 | 0,850 | 0,962 | 0,910 | 0,835 | 0,985 |
| RSAD2 OAS1 IFI44L HERC6 IL1R2 FAM20A | 0,874 | 0,865 | 0,883 | 0,907 | 0,846 | 0,968 | 0,936 | 0,857 | 1 |
| OAS1 ISG15 HERC6 IL1R2 OLAH FAM20A | 0,874 | 0,864 | 0,884 | 0,906 | 0,846 | 0,966 | 0,936 | 0,860 | 1 |
| OAS1 IFI44L HERC6 IL1R2 FAM20A MMP8 | 0,874 | 0,865 | 0,883 | 0,909 | 0,849 | 0,969 | 0,934 | 0,851 | 1 |
| SIGLEC1 ISG15 HERC6 OLAH FAM20A RETN | 0,874 | 0,864 | 0,884 | 0,900 | 0,833 | 0,968 | 0,958 | 0,902 | 1 |
| SIGLEC1 ISG15 SLC1A2 IL1R2 OLAH MMP8 | 0,874 | 0,866 | 0,883 | 0,913 | 0,859 | 0,966 | 0,965 | 0,929 | 1 |
| RSAD2 SIGLEC1 HERC6 IL1R2 RETN MMP8 | 0,874 | 0,864 | 0,884 | 0,902 | 0,838 | 0,966 | 0,961 | 0,921 | 1 |
| IFI44L HERC6 SLC1A2 OLAH FAM20A MMP8 | 0,874 | 0,864 | 0,884 | 0,912 | 0,855 | 0,969 | 0,934 | 0,859 | 1 |
| RSAD2 ISG15 HERC6 IL1R2 FAM20A MMP8 | 0,874 | 0,864 | 0,884 | 0,906 | 0,847 | 0,966 | 0,930 | 0,846 | 1 |
| OAS1 IFIT1 IFI44L SIGLEC1 HERC6 IL1R2 | 0,874 | 0,865 | 0,883 | 0,903 | 0,843 | 0,962 | 0,955 | 0,912 | 0,999 |
| RSAD2 OAS1 SIGLEC1 HERC6 FAM20A RETN | 0,874 | 0,865 | 0,883 | 0,907 | 0,844 | 0,969 | 0,943 | 0,876 | 1 |
| IFIT1 SIGLEC1 SLC1A2 IL1R2 OLAH MMP8 | 0,874 | 0,865 | 0,883 | 0,911 | 0,856 | 0,966 | 0,965 | 0,929 | 1 |
| IFIT1 SIGLEC1 HERC6 OLAH RETN MMP8 | 0,874 | 0,864 | 0,884 | 0,901 | 0,836 | 0,967 | 0,958 | 0,917 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 SIGLEC1 ISG15 HERC6 FAM20A | 0,874 | 0,864 | 0,883 | 0,902 | 0,838 | 0,967 | 0,940 | 0,869 | 1 |
| IFIT1 SIGLEC1 ISG15 SLC1A2 FAM20A MMP8 | 0,874 | 0,864 | 0,883 | 0,905 | 0,844 | 0,966 | 0,940 | 0,867 | 1 |
| IFIT1 IFI44L SIGLEC1 HERC6 IL1R2 MMP8 | 0,874 | 0,865 | 0,883 | 0,902 | 0,843 | 0,961 | 0,961 | 0,921 | 1 |
| RSAD2 IFI27 HERC6 SLC1A2 RETN MMP8 | 0,874 | 0,864 | 0,883 | 0,911 | 0,852 | 0,970 | 0,929 | 0,858 | 1 |
| IFI27 IFIT1 ISG15 HERC6 SLC1A2 RETN | 0,874 | 0,864 | 0,883 | 0,911 | 0,852 | 0,971 | 0,939 | 0,870 | 1 |
| OAS1 IFI44L SIGLEC1 HERC6 IL1R2 MMP8 | 0,874 | 0,865 | 0,882 | 0,902 | 0,843 | 0,961 | 0,961 | 0,921 | 1 |
| IFI27 OAS1 IFI44L SLC1A2 FAM20A RETN | 0,874 | 0,864 | 0,884 | 0,911 | 0,848 | 0,973 | 0,939 | 0,857 | 1 |
| RSAD2 SIGLEC1 HERC6 SLC1A2 IL1R2 OLAH | 0,874 | 0,864 | 0,883 | 0,911 | 0,855 | 0,966 | 0,955 | 0,912 | 0,999 |
| SIGLEC1 HERC6 SLC1A2 IL1R2 OLAH MMP8 | 0,874 | 0,864 | 0,883 | 0,911 | 0,857 | 0,966 | 0,965 | 0,929 | 1 |
| IFI44L SLC1A2 IL1R2 OLAH FAM20A RETN | 0,874 | 0,864 | 0,883 | 0,913 | 0,858 | 0,968 | 0,927 | 0,849 | 1 |
| IFI44L SIGLEC1 ISG15 SLC1A2 IL1R2 OLAH | 0,874 | 0,865 | 0,882 | 0,914 | 0,859 | 0,968 | 0,962 | 0,924 | 1 |
| OAS1 SIGLEC1 HERC6 SLC1A2 OLAH MMP8 | 0,874 | 0,864 | 0,883 | 0,911 | 0,856 | 0,967 | 0,954 | 0,910 | 0,999 |
| RSAD2 IFIT1 SIGLEC1 ISG15 FAM20A MMP8 | 0,874 | 0,864 | 0,883 | 0,899 | 0,830 | 0,969 | 0,943 | 0,877 | 1 |
| IFIT1 SLC1A2 IL1R2 OLAH FAM20A MMP8 | 0,874 | 0,865 | 0,882 | 0,914 | 0,861 | 0,967 | 0,935 | 0,858 | 1 |
| OAS1 IFIT1 IL1R2 OLAH FAM20A MMP8 | 0,874 | 0,865 | 0,883 | 0,906 | 0,844 | 0,968 | 0,942 | 0,870 | 1 |
| RSAD2 OAS1 SIGLEC1 HERC6 IL1R2 MMP8 | 0,874 | 0,864 | 0,883 | 0,904 | 0,845 | 0,962 | 0,963 | 0,925 | 1 |
| IFIT1 IFI44L SIGLEC1 HERC6 IL1R2 RETN | 0,874 | 0,864 | 0,883 | 0,905 | 0,842 | 0,967 | 0,952 | 0,907 | 0,997 |
| OAS1 IFIT1 SIGLEC1 SLC1A2 OLAH MMP8 | 0,874 | 0,864 | 0,883 | 0,911 | 0,855 | 0,967 | 0,952 | 0,907 | 0,997 |
| OAS1 SIGLEC1 HERC6 OLAH FAM20A RETN | 0,874 | 0,864 | 0,883 | 0,906 | 0,842 | 0,969 | 0,954 | 0,900 | 1 |
| RSAD2 IFIT1 SIGLEC1 SLC1A2 FAM20A MMP8 | 0,874 | 0,864 | 0,883 | 0,907 | 0,846 | 0,967 | 0,946 | 0,874 | 1 |
| RSAD2 OAS1 SIGLEC1 SLC1A2 OLAH MMP8 | 0,873 | 0,864 | 0,883 | 0,911 | 0,855 | 0,966 | 0,957 | 0,913 | 1,000 |
| OAS1 SIGLEC1 SLC1A2 IL1R2 OLAH MMP8 | 0,873 | 0,864 | 0,883 | 0,912 | 0,858 | 0,966 | 0,966 | 0,931 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 IFIT1 HERC6 SLC1A2 RETN MMP8 | 0,873 | 0,864 | 0,883 | 0,911 | 0,851 | 0,971 | 0,927 | 0,853 | 1 |
| IFIT1 SIGLEC1 ISG15 HERC6 SLC1A2 OLAH | 0,873 | 0,864 | 0,883 | 0,912 | 0,857 | 0,967 | 0,943 | 0,893 | 0,994 |
| RSAD2 IFI27 SIGLEC1 SLC1A2 RETN MMP8 | 0,873 | 0,864 | 0,883 | 0,914 | 0,857 | 0,971 | 0,954 | 0,898 | 1 |
| IFIT1 SIGLEC1 ISG15 HERC6 IL1R2 RETN | 0,873 | 0,864 | 0,882 | 0,905 | 0,843 | 0,967 | 0,952 | 0,907 | 0,998 |
| OAS1 IFIT1 HERC6 IL1R2 OLAH FAM20A | 0,873 | 0,864 | 0,883 | 0,906 | 0,844 | 0,967 | 0,938 | 0,864 | 1 |
| IFI44L SIGLEC1 HERC6 IL1R2 OLAH MMP8 | 0,873 | 0,864 | 0,882 | 0,903 | 0,844 | 0,962 | 0,963 | 0,924 | 1 |
| RSAD2 OAS1 SIGLEC1 HERC6 SLC1A2 FAM20A | 0,873 | 0,864 | 0,883 | 0,909 | 0,852 | 0,965 | 0,939 | 0,863 | 1 |
| IFI44L HERC6 IL1R2 OLAH FAM20A RETN | 0,873 | 0,864 | 0,883 | 0,905 | 0,844 | 0,967 | 0,938 | 0,864 | 1 |
| IFI27 OAS1 IFI44L ISG15 HERC6 FAM20A | 0,873 | 0,863 | 0,883 | 0,904 | 0,841 | 0,968 | 0,927 | 0,844 | 1 |
| IFIT1 IFI44L SIGLEC1 ISG15 HERC6 IL1R2 | 0,873 | 0,865 | 0,882 | 0,904 | 0,845 | 0,962 | 0,954 | 0,910 | 0,999 |
| RSAD2 OAS1 IFIT1 HERC6 IL1R2 FAM20A | 0,873 | 0,864 | 0,883 | 0,904 | 0,841 | 0,966 | 0,936 | 0,855 | 1 |
| RSAD2 OAS1 IFIT1 SIGLEC1 HERC6 IL1R2 | 0,873 | 0,864 | 0,882 | 0,904 | 0,847 | 0,962 | 0,958 | 0,916 | 0,999 |
| RSAD2 IFI44L SLC1A2 OLAH FAM20A MMP8 | 0,873 | 0,864 | 0,883 | 0,914 | 0,857 | 0,970 | 0,925 | 0,851 | 0,999 |
| IFIT1 SIGLEC1 HERC6 SLC1A2 IL1R2 OLAH | 0,873 | 0,864 | 0,883 | 0,913 | 0,858 | 0,967 | 0,952 | 0,908 | 0,997 |
| IFI27 OAS1 SIGLEC1 ISG15 HERC6 MMP8 | 0,873 | 0,865 | 0,882 | 0,908 | 0,853 | 0,963 | 0,917 | 0,846 | 0,989 |
| IFI44L SLC1A2 OLAH FAM20A RETN MMP8 | 0,873 | 0,864 | 0,883 | 0,914 | 0,856 | 0,971 | 0,932 | 0,859 | 1 |
| RSAD2 IFI27 OAS1 ISG15 HERC6 FAM20A | 0,873 | 0,863 | 0,883 | 0,908 | 0,847 | 0,968 | 0,928 | 0,846 | 1 |
| RSAD2 IFI27 IFI44L SLC1A2 FAM20A RETN | 0,873 | 0,863 | 0,883 | 0,910 | 0,847 | 0,973 | 0,938 | 0,857 | 1 |
| RSAD2 OAS1 IFIT1 IL1R2 FAM20A MMP8 | 0,873 | 0,864 | 0,882 | 0,907 | 0,845 | 0,969 | 0,946 | 0,873 | 1 |
| IFIT1 IFI44L SIGLEC1 HERC6 SLC1A2 OLAH | 0,873 | 0,864 | 0,883 | 0,912 | 0,857 | 0,967 | 0,940 | 0,888 | 0,993 |
| RSAD2 IFIT1 HERC6 IL1R2 FAM20A MMP8 | 0,873 | 0,864 | 0,882 | 0,904 | 0,843 | 0,965 | 0,937 | 0,859 | 1 |
| RSAD2 IFIT1 SIGLEC1 HERC6 SLC1A2 OLAH | 0,873 | 0,864 | 0,883 | 0,912 | 0,857 | 0,967 | 0,943 | 0,893 | 0,994 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 HERC6 SLC1A2 FAM20A RETN | 0,873 | 0,863 | 0,883 | 0,911 | 0,854 | 0,969 | 0,940 | 0,864 | 1 |
| IFIT1 SIGLEC1 ISG15 SLC1A2 IL1R2 OLAH | 0,873 | 0,864 | 0,882 | 0,913 | 0,858 | 0,968 | 0,962 | 0,924 | 1 |
| RSAD2 OAS1 IFI44L SIGLEC1 OLAH FAM20A | 0,873 | 0,863 | 0,883 | 0,898 | 0,827 | 0,970 | 0,957 | 0,908 | 1 |
| IFIT1 HERC6 IL1R2 FAM20A RETN MMP8 | 0,873 | 0,864 | 0,883 | 0,907 | 0,845 | 0,970 | 0,940 | 0,860 | 1 |
| RSAD2 IFI44L SIGLEC1 HERC6 IL1R2 MMP8 | 0,873 | 0,864 | 0,882 | 0,902 | 0,842 | 0,962 | 0,960 | 0,919 | 1 |
| RSAD2 IFI27 OAS1 SIGLEC1 HERC6 MMP8 | 0,873 | 0,864 | 0,882 | 0,905 | 0,849 | 0,962 | 0,918 | 0,846 | 0,991 |
| IFI44L ISG15 HERC6 SLC1A2 IL1R2 FAM20A | 0,873 | 0,863 | 0,883 | 0,912 | 0,859 | 0,965 | 0,928 | 0,839 | 1 |
| OAS1 IFIT1 IFI44L SIGLEC1 SLC1A2 OLAH | 0,873 | 0,864 | 0,882 | 0,912 | 0,856 | 0,968 | 0,943 | 0,892 | 0,995 |
| IFIT1 SIGLEC1 ISG15 SLC1A2 FAM20A RETN | 0,873 | 0,864 | 0,882 | 0,908 | 0,848 | 0,968 | 0,940 | 0,868 | 1 |
| SIGLEC1 ISG15 HERC6 SLC1A2 OLAH MMP8 | 0,873 | 0,863 | 0,882 | 0,910 | 0,853 | 0,966 | 0,951 | 0,905 | 0,997 |
| IFIT1 SIGLEC1 HERC6 SLC1A2 OLAH RETN | 0,873 | 0,863 | 0,883 | 0,914 | 0,858 | 0,969 | 0,943 | 0,893 | 0,994 |
| OAS1 IFI44L HERC6 IL1R2 OLAH FAM20A | 0,873 | 0,863 | 0,882 | 0,906 | 0,845 | 0,967 | 0,939 | 0,867 | 1 |
| RSAD2 OAS1 SIGLEC1 FAM20A RETN MMP8 | 0,873 | 0,863 | 0,883 | 0,903 | 0,836 | 0,971 | 0,955 | 0,893 | 1 |
| IFIT1 IFI44L SIGLEC1 ISG15 SLC1A2 OLAH | 0,873 | 0,864 | 0,882 | 0,912 | 0,856 | 0,968 | 0,943 | 0,892 | 0,995 |
| HERC6 IL1R2 OLAH FAM20A RETN MMP8 | 0,873 | 0,863 | 0,883 | 0,909 | 0,850 | 0,968 | 0,939 | 0,862 | 1 |
| IFI44L SIGLEC1 ISG15 HERC6 IL1R2 MMP8 | 0,873 | 0,864 | 0,881 | 0,902 | 0,843 | 0,961 | 0,960 | 0,919 | 1 |
| IFI27 IFIT1 IFI44L HERC6 SLC1A2 RETN | 0,873 | 0,863 | 0,883 | 0,910 | 0,849 | 0,971 | 0,938 | 0,869 | 1 |
| OAS1 SIGLEC1 HERC6 SLC1A2 IL1R2 OLAH | 0,873 | 0,863 | 0,883 | 0,912 | 0,858 | 0,966 | 0,959 | 0,917 | 1 |
| SIGLEC1 HERC6 SLC1A2 FAM20A RETN MMP8 | 0,873 | 0,863 | 0,883 | 0,904 | 0,840 | 0,968 | 0,953 | 0,880 | 1 |
| IFI44L ISG15 SLC1A2 IL1R2 FAM20A MMP8 | 0,873 | 0,864 | 0,881 | 0,911 | 0,859 | 0,963 | 0,928 | 0,840 | 1 |
| RSAD2 OAS1 IFIT1 SIGLEC1 SLC1A2 OLAH | 0,873 | 0,863 | 0,882 | 0,911 | 0,855 | 0,968 | 0,952 | 0,905 | 0,999 |
| RSAD2 IFIT1 SIGLEC1 SLC1A2 OLAH MMP8 | 0,873 | 0,864 | 0,882 | 0,911 | 0,855 | 0,967 | 0,954 | 0,910 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27 IFI44L HERC6 SLC1A2 RETN MMP8 | 0,873 | 0,863 | 0,882 | 0,909 | 0,848 | 0,970 | 0,930 | 0,857 | 1 |
| RSAD2 IFI27 IFIT1 ISG15 HERC6 FAM20A | 0,873 | 0,863 | 0,883 | 0,907 | 0,847 | 0,968 | 0,926 | 0,843 | 1 |
| IFI27 OAS1 ISG15 SLC1A2 FAM20A RETN | 0,873 | 0,863 | 0,883 | 0,910 | 0,849 | 0,971 | 0,940 | 0,860 | 1 |
| OAS1 IFIT1 IFI44L HERC6 IL1R2 FAM20A | 0,873 | 0,864 | 0,882 | 0,906 | 0,844 | 0,967 | 0,936 | 0,856 | 1 |
| IFIT1 SIGLEC1 ISG15 HERC6 IL1R2 MMP8 | 0,873 | 0,864 | 0,881 | 0,904 | 0,847 | 0,962 | 0,957 | 0,913 | 1 |
| OAS1 IFIT1 SIGLEC1 ISG15 HERC6 IL1R2 | 0,873 | 0,864 | 0,882 | 0,905 | 0,849 | 0,962 | 0,951 | 0,904 | 0,998 |
| RSAD2 HERC6 SLC1A2 IL1R2 OLAH FAM20A | 0,873 | 0,863 | 0,883 | 0,914 | 0,859 | 0,968 | 0,928 | 0,844 | 1 |
| ISG15 HERC6 SLC1A2 IL1R2 OLAH FAM20A | 0,873 | 0,863 | 0,883 | 0,911 | 0,857 | 0,966 | 0,926 | 0,842 | 1 |
| IFI27 IFIT1 ISG15 SLC1A2 FAM20A RETN | 0,873 | 0,863 | 0,883 | 0,908 | 0,847 | 0,969 | 0,940 | 0,859 | 1 |
| OAS1 ISG15 HERC6 SLC1A2 IL1R2 FAM20A | 0,873 | 0,863 | 0,883 | 0,911 | 0,858 | 0,965 | 0,925 | 0,835 | 1 |
| IFI44L SIGLEC1 SLC1A2 IL1R2 OLAH RETN | 0,873 | 0,864 | 0,882 | 0,913 | 0,858 | 0,968 | 0,962 | 0,922 | 1 |
| RSAD2 SIGLEC1 HERC6 SLC1A2 OLAH MMP8 | 0,873 | 0,863 | 0,882 | 0,912 | 0,857 | 0,967 | 0,950 | 0,904 | 0,996 |
| RSAD2 IFI27 ISG15 FAM20A RETN MMP8 | 0,873 | 0,862 | 0,883 | 0,909 | 0,847 | 0,971 | 0,945 | 0,867 | 1 |
| RSAD2 OAS1 SIGLEC1 HERC6 SLC1A2 OLAH | 0,873 | 0,863 | 0,882 | 0,912 | 0,856 | 0,967 | 0,951 | 0,904 | 0,998 |
| RSAD2 IFIT1 SIGLEC1 SLC1A2 FAM20A RETN | 0,873 | 0,863 | 0,882 | 0,908 | 0,848 | 0,969 | 0,946 | 0,876 | 1 |
| RSAD2 IFI44L HERC6 IL1R2 FAM20A MMP8 | 0,873 | 0,863 | 0,882 | 0,909 | 0,850 | 0,968 | 0,932 | 0,846 | 1 |
| IFI27 OAS1 IFIT1 IFI44L HERC6 FAM20A | 0,873 | 0,863 | 0,882 | 0,903 | 0,838 | 0,968 | 0,927 | 0,845 | 1 |
| IFI27 IFI44L ISG15 HERC6 SLC1A2 RETN | 0,873 | 0,863 | 0,882 | 0,910 | 0,849 | 0,971 | 0,939 | 0,870 | 1 |
| RSAD2 OAS1 SIGLEC1 OLAH FAM20A RETN | 0,873 | 0,863 | 0,883 | 0,896 | 0,824 | 0,969 | 0,958 | 0,911 | 1 |
| RSAD2 OAS1 SIGLEC1 HERC6 IL1R2 RETN | 0,873 | 0,863 | 0,882 | 0,901 | 0,837 | 0,965 | 0,960 | 0,919 | 1 |
| IFI44L ISG15 HERC6 SLC1A2 OLAH FAM20A | 0,873 | 0,863 | 0,883 | 0,912 | 0,855 | 0,969 | 0,929 | 0,851 | 1 |
| IFIT1 IFI44L SLC1A2 IL1R2 OLAH FAM20A | 0,873 | 0,863 | 0,882 | 0,912 | 0,855 | 0,968 | 0,926 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 HERC6 SLC1A2 FAM20A MMP8 | 0,873 | 0,863 | 0,882 | 0,908 | 0,851 | 0,965 | 0,943 | 0,868 | 1 |
| IFI44L HERC6 IL1R2 FAM20A RETN MMP8 | 0,872 | 0,863 | 0,882 | 0,909 | 0,848 | 0,970 | 0,938 | 0,857 | 1 |
| RSAD2 IFI44L HERC6 IL1R2 OLAH FAM20A | 0,872 | 0,863 | 0,882 | 0,906 | 0,846 | 0,966 | 0,937 | 0,863 | 1 |
| IFIT1 IFI44L HERC6 OLAH FAM20A MMP8 | 0,872 | 0,863 | 0,882 | 0,901 | 0,837 | 0,965 | 0,937 | 0,869 | 1 |
| IFI44L HERC6 SLC1A2 IL1R2 FAM20A MMP8 | 0,872 | 0,863 | 0,882 | 0,916 | 0,866 | 0,967 | 0,923 | 0,827 | 1 |
| IFIT1 IFI44L HERC6 IL1R2 FAM20A RETN | 0,872 | 0,863 | 0,882 | 0,906 | 0,844 | 0,968 | 0,936 | 0,855 | 1 |
| IFIT1 SIGLEC1 HERC6 IL1R2 OLAH MMP8 | 0,872 | 0,863 | 0,881 | 0,906 | 0,849 | 0,964 | 0,958 | 0,915 | 1,000 |
| IFIT1 IFI44L SIGLEC1 SLC1A2 OLAH RETN | 0,872 | 0,863 | 0,881 | 0,915 | 0,860 | 0,971 | 0,939 | 0,883 | 0,995 |
| RSAD2 OAS1 SIGLEC1 SLC1A2 IL1R2 OLAH | 0,872 | 0,863 | 0,882 | 0,913 | 0,857 | 0,968 | 0,966 | 0,929 | 1 |
| IFI44L SIGLEC1 ISG15 FAM20A RETN MMP8 | 0,872 | 0,862 | 0,882 | 0,901 | 0,832 | 0,971 | 0,959 | 0,897 | 1 |
| RSAD2 IFIT1 IFI44L SIGLEC1 HERC6 IL1R2 | 0,872 | 0,864 | 0,881 | 0,900 | 0,839 | 0,962 | 0,955 | 0,913 | 0,998 |
| RSAD2 OAS1 IFI44L SIGLEC1 HERC6 IL1R2 | 0,872 | 0,863 | 0,881 | 0,901 | 0,840 | 0,962 | 0,961 | 0,921 | 1 |
| IFIT1 SIGLEC1 HERC6 SLC1A2 IL1R2 RETN | 0,872 | 0,863 | 0,881 | 0,910 | 0,855 | 0,965 | 0,951 | 0,906 | 0,996 |
| IFI44L SIGLEC1 ISG15 SLC1A2 OLAH RETN | 0,872 | 0,863 | 0,881 | 0,912 | 0,856 | 0,969 | 0,948 | 0,898 | 0,998 |
| IFIT1 IFI44L ISG15 SLC1A2 OLAH FAM20A | 0,872 | 0,863 | 0,882 | 0,909 | 0,850 | 0,968 | 0,920 | 0,843 | 0,997 |
| OAS1 SIGLEC1 ISG15 HERC6 SLC1A2 OLAH | 0,872 | 0,863 | 0,882 | 0,912 | 0,857 | 0,967 | 0,951 | 0,904 | 0,998 |
| OAS1 IFIT1 SIGLEC1 SLC1A2 IL1R2 OLAH | 0,872 | 0,863 | 0,882 | 0,912 | 0,857 | 0,967 | 0,962 | 0,923 | 1 |
| RSAD2 IFIT1 SIGLEC1 HERC6 IL1R2 MMP8 | 0,872 | 0,863 | 0,881 | 0,903 | 0,844 | 0,962 | 0,958 | 0,916 | 0,999 |
| IFIT1 IFI44L HERC6 OLAH FAM20A RETN | 0,872 | 0,863 | 0,882 | 0,903 | 0,840 | 0,967 | 0,935 | 0,864 | 1 |
| OAS1 HERC6 IL1R2 FAM20A RETN MMP8 | 0,872 | 0,863 | 0,882 | 0,906 | 0,845 | 0,968 | 0,938 | 0,857 | 1 |
| OAS1 IFIT1 SIGLEC1 ISG15 SLC1A2 OLAH | 0,872 | 0,863 | 0,882 | 0,912 | 0,856 | 0,968 | 0,945 | 0,894 | 0,995 |
| IFI27 IFI44L SIGLEC1 SLC1A2 RETN MMP8 | 0,872 | 0,863 | 0,881 | 0,915 | 0,858 | 0,972 | 0,954 | 0,898 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 ISG15 HERC6 FAM20A MMP8 | 0,872 | 0,863 | 0,882 | 0,903 | 0,838 | 0,967 | 0,943 | 0,875 | 1 |
| OAS1 IFIT1 SIGLEC1 HERC6 OLAH RETN | 0,872 | 0,863 | 0,882 | 0,900 | 0,836 | 0,965 | 0,941 | 0,890 | 0,993 |
| IFIT1 SIGLEC1 ISG15 HERC6 IL1R2 OLAH | 0,872 | 0,863 | 0,881 | 0,908 | 0,850 | 0,966 | 0,950 | 0,904 | 0,996 |
| IFI27 SIGLEC1 HERC6 SLC1A2 RETN MMP8 | 0,872 | 0,862 | 0,882 | 0,910 | 0,847 | 0,972 | 0,953 | 0,896 | 1 |
| OAS1 SIGLEC1 HERC6 SLC1A2 FAM20A RETN | 0,872 | 0,862 | 0,882 | 0,910 | 0,852 | 0,967 | 0,943 | 0,871 | 1 |
| RSAD2 SIGLEC1 HERC6 IL1R2 OLAH MMP8 | 0,872 | 0,863 | 0,882 | 0,905 | 0,847 | 0,963 | 0,960 | 0,919 | 1 |
| RSAD2 IFIT1 SIGLEC1 SLC1A2 IL1R2 OLAH | 0,872 | 0,863 | 0,881 | 0,911 | 0,855 | 0,967 | 0,963 | 0,925 | 1 |
| OAS1 IFIT1 SIGLEC1 HERC6 SLC1A2 IL1R2 | 0,872 | 0,863 | 0,881 | 0,910 | 0,857 | 0,963 | 0,960 | 0,920 | 1,000 |
| RSAD2 OAS1 HERC6 IL1R2 FAM20A RETN | 0,872 | 0,862 | 0,882 | 0,905 | 0,842 | 0,967 | 0,935 | 0,853 | 1 |
| IFIT1 SIGLEC1 ISG15 HERC6 OLAH RETN | 0,872 | 0,863 | 0,882 | 0,901 | 0,837 | 0,966 | 0,948 | 0,900 | 0,995 |
| IFI27 OAS1 IFIT1 SLC1A2 FAM20A RETN | 0,872 | 0,862 | 0,882 | 0,908 | 0,848 | 0,969 | 0,939 | 0,858 | 1 |
| RSAD2 SIGLEC1 ISG15 SLC1A2 OLAH MMP8 | 0,872 | 0,863 | 0,881 | 0,912 | 0,857 | 0,968 | 0,953 | 0,909 | 0,998 |
| IFI44L SIGLEC1 SLC1A2 FAM20A RETN MMP8 | 0,872 | 0,862 | 0,882 | 0,903 | 0,839 | 0,967 | 0,953 | 0,882 | 1 |
| RSAD2 IFIT1 SIGLEC1 HERC6 IL1R2 RETN | 0,872 | 0,863 | 0,881 | 0,904 | 0,840 | 0,967 | 0,953 | 0,909 | 0,997 |
| IFI44L SIGLEC1 ISG15 OLAH FAM20A RETN | 0,872 | 0,862 | 0,882 | 0,897 | 0,826 | 0,969 | 0,961 | 0,916 | 1 |
| OAS1 SIGLEC1 ISG15 SLC1A2 OLAH MMP8 | 0,872 | 0,863 | 0,881 | 0,913 | 0,857 | 0,968 | 0,955 | 0,913 | 0,998 |
| OAS1 SIGLEC1 HERC6 IL1R2 OLAH MMP8 | 0,872 | 0,863 | 0,881 | 0,904 | 0,846 | 0,962 | 0,962 | 0,922 | 1 |
| OAS1 IFIT1 SIGLEC1 SLC1A2 OLAH RETN | 0,872 | 0,862 | 0,882 | 0,914 | 0,858 | 0,970 | 0,945 | 0,893 | 0,996 |
| OAS1 IFI44L SIGLEC1 HERC6 FAM20A RETN | 0,872 | 0,862 | 0,882 | 0,904 | 0,840 | 0,968 | 0,949 | 0,885 | 1 |
| RSAD2 IFI27 IFI44L HERC6 SLC1A2 FAM20A | 0,872 | 0,861 | 0,882 | 0,914 | 0,857 | 0,970 | 0,928 | 0,847 | 1 |
| IFIT1 IFI44L SLC1A2 IL1R2 FAM20A MMP8 | 0,872 | 0,863 | 0,881 | 0,912 | 0,859 | 0,964 | 0,930 | 0,843 | 1 |
| OAS1 SIGLEC1 ISG15 HERC6 IL1R2 RETN | 0,872 | 0,862 | 0,882 | 0,903 | 0,842 | 0,965 | 0,962 | 0,921 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFI44L SLC1A2 OLAH FAM20A MMP8 | 0,872 | 0,862 | 0,882 | 0,912 | 0,855 | 0,970 | 0,928 | 0,854 | 1 |
| SIGLEC1 ISG15 HERC6 SLC1A2 IL1R2 OLAH | 0,872 | 0,863 | 0,881 | 0,912 | 0,857 | 0,966 | 0,957 | 0,914 | 0,999 |
| OAS1 IFIT1 SIGLEC1 HERC6 IL1R2 OLAH | 0,872 | 0,863 | 0,881 | 0,907 | 0,848 | 0,965 | 0,951 | 0,905 | 0,997 |
| RSAD2 SIGLEC1 ISG15 SLC1A2 IL1R2 OLAH | 0,872 | 0,863 | 0,881 | 0,912 | 0,857 | 0,968 | 0,961 | 0,922 | 1 |
| OAS1 IFI44L SIGLEC1 ISG15 OLAH FAM20A | 0,872 | 0,862 | 0,882 | 0,898 | 0,826 | 0,970 | 0,961 | 0,915 | 1 |
| RSAD2 SLC1A2 IL1R2 OLAH FAM20A MMP8 | 0,872 | 0,863 | 0,881 | 0,914 | 0,862 | 0,966 | 0,929 | 0,844 | 1 |
| OAS1 SLC1A2 IL1R2 OLAH FAM20A MMP8 | 0,872 | 0,863 | 0,881 | 0,913 | 0,860 | 0,967 | 0,930 | 0,847 | 1 |
| SIGLEC1 SLC1A2 IL1R2 OLAH RETN MMP8 | 0,872 | 0,862 | 0,881 | 0,914 | 0,859 | 0,968 | 0,968 | 0,935 | 1 |
| IFI44L SIGLEC1 SLC1A2 OLAH RETN MMP8 | 0,872 | 0,862 | 0,881 | 0,908 | 0,850 | 0,967 | 0,962 | 0,923 | 1 |
| SIGLEC1 ISG15 SLC1A2 FAM20A RETN MMP8 | 0,872 | 0,862 | 0,882 | 0,903 | 0,840 | 0,967 | 0,953 | 0,882 | 1 |
| OAS1 IFI44L HERC6 SLC1A2 OLAH FAM20A | 0,872 | 0,861 | 0,882 | 0,911 | 0,854 | 0,967 | 0,924 | 0,844 | 1 |
| IFIT1 IFI44L ISG15 HERC6 OLAH FAM20A | 0,872 | 0,862 | 0,881 | 0,903 | 0,839 | 0,966 | 0,935 | 0,864 | 1 |
| RSAD2 ISG15 HERC6 SLC1A2 IL1R2 FAM20A | 0,872 | 0,862 | 0,881 | 0,909 | 0,855 | 0,963 | 0,924 | 0,832 | 1 |
| OAS1 IFI44L SIGLEC1 HERC6 IL1R2 RETN | 0,872 | 0,862 | 0,881 | 0,903 | 0,840 | 0,965 | 0,957 | 0,914 | 0,999 |
| IFI27 SIGLEC1 ISG15 SLC1A2 RETN MMP8 | 0,872 | 0,862 | 0,881 | 0,910 | 0,850 | 0,970 | 0,957 | 0,904 | 1 |
| OAS1 SIGLEC1 HERC6 SLC1A2 IL1R2 RETN | 0,872 | 0,862 | 0,881 | 0,911 | 0,857 | 0,966 | 0,957 | 0,913 | 1 |
| OAS1 IFI44L SIGLEC1 SLC1A2 OLAH MMP8 | 0,872 | 0,862 | 0,881 | 0,910 | 0,854 | 0,966 | 0,957 | 0,914 | 0,999 |
| OAS1 IFIT1 IFI44L SIGLEC1 HERC6 RETN | 0,872 | 0,862 | 0,881 | 0,900 | 0,833 | 0,967 | 0,923 | 0,862 | 0,984 |
| IFIT1 HERC6 IL1R2 OLAH FAM20A RETN | 0,872 | 0,862 | 0,881 | 0,905 | 0,844 | 0,967 | 0,936 | 0,861 | 1 |
| RSAD2 OAS1 SIGLEC1 HERC6 FAM20A MMP8 | 0,872 | 0,862 | 0,881 | 0,899 | 0,833 | 0,965 | 0,943 | 0,873 | 1 |
| RSAD2 IFI27 IFIT1 SIGLEC1 HERC6 MMP8 | 0,872 | 0,863 | 0,881 | 0,905 | 0,848 | 0,962 | 0,904 | 0,828 | 0,981 |
| IFI44L ISG15 SLC1A2 OLAH FAM20A RETN | 0,872 | 0,862 | 0,882 | 0,909 | 0,849 | 0,968 | 0,927 | 0,853 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L SIGLEC1 HERC6 SLC1A2 FAM20A RETN | 0,872 | 0,862 | 0,882 | 0,904 | 0,841 | 0,967 | 0,950 | 0,876 | 1 |
| IFI27 IFIT1 SIGLEC1 HERC6 SLC1A2 MMP8 | 0,872 | 0,863 | 0,881 | 0,910 | 0,856 | 0,964 | 0,917 | 0,848 | 0,987 |
| RSAD2 OAS1 IFIT1 SIGLEC1 HERC6 RETN | 0,872 | 0,862 | 0,881 | 0,899 | 0,832 | 0,966 | 0,927 | 0,868 | 0,987 |
| RSAD2 IFI27 OAS1 IFI44L ISG15 FAM20A | 0,872 | 0,862 | 0,882 | 0,902 | 0,835 | 0,969 | 0,940 | 0,864 | 1 |
| IFIT1 HERC6 SLC1A2 IL1R2 FAM20A MMP8 | 0,872 | 0,862 | 0,881 | 0,915 | 0,863 | 0,967 | 0,929 | 0,842 | 1 |
| IFIT1 IFI44L HERC6 SLC1A2 IL1R2 FAM20A | 0,872 | 0,862 | 0,881 | 0,913 | 0,861 | 0,965 | 0,926 | 0,836 | 1 |
| OAS1 HERC6 SLC1A2 IL1R2 OLAH FAM20A | 0,872 | 0,862 | 0,881 | 0,913 | 0,858 | 0,967 | 0,927 | 0,842 | 1 |
| RSAD2 IFI44L SLC1A2 IL1R2 OLAH FAM20A | 0,872 | 0,862 | 0,881 | 0,912 | 0,856 | 0,968 | 0,929 | 0,852 | 1 |
| RSAD2 HERC6 IL1R2 OLAH FAM20A RETN | 0,872 | 0,862 | 0,881 | 0,907 | 0,845 | 0,968 | 0,937 | 0,863 | 1 |
| IFI44L SIGLEC1 ISG15 HERC6 IL1R2 OLAH | 0,872 | 0,862 | 0,881 | 0,902 | 0,841 | 0,963 | 0,960 | 0,919 | 1 |
| OAS1 SIGLEC1 ISG15 HERC6 SLC1A2 IL1R2 | 0,872 | 0,862 | 0,881 | 0,910 | 0,857 | 0,963 | 0,965 | 0,927 | 1 |
| OAS1 SIGLEC1 HERC6 SLC1A2 IL1R2 MMP8 | 0,872 | 0,863 | 0,880 | 0,910 | 0,857 | 0,963 | 0,964 | 0,927 | 1 |
| RSAD2 SIGLEC1 ISG15 HERC6 IL1R2 MMP8 | 0,872 | 0,863 | 0,881 | 0,904 | 0,847 | 0,962 | 0,961 | 0,921 | 1 |
| SIGLEC1 ISG15 SLC1A2 IL1R2 OLAH RETN | 0,872 | 0,863 | 0,881 | 0,913 | 0,857 | 0,968 | 0,961 | 0,920 | 1 |
| RSAD2 OAS1 SIGLEC1 HERC6 IL1R2 OLAH | 0,872 | 0,862 | 0,881 | 0,907 | 0,849 | 0,965 | 0,958 | 0,915 | 1,000 |
| HERC6 SLC1A2 IL1R2 OLAH FAM20A MMP8 | 0,872 | 0,862 | 0,881 | 0,912 | 0,861 | 0,964 | 0,921 | 0,830 | 1 |
| IFI44L SIGLEC1 ISG15 HERC6 IL1R2 RETN | 0,872 | 0,862 | 0,881 | 0,901 | 0,838 | 0,965 | 0,960 | 0,919 | 1 |
| IFIT1 ISG15 HERC6 SLC1A2 IL1R2 FAM20A | 0,871 | 0,862 | 0,881 | 0,911 | 0,857 | 0,965 | 0,928 | 0,842 | 1 |
| OAS1 HERC6 IL1R2 OLAH FAM20A RETN | 0,871 | 0,861 | 0,881 | 0,906 | 0,845 | 0,967 | 0,937 | 0,863 | 1 |
| OAS1 IFIT1 HERC6 IL1R2 FAM20A RETN | 0,871 | 0,862 | 0,881 | 0,905 | 0,842 | 0,968 | 0,937 | 0,856 | 1 |
| IFI27 OAS1 IFIT1 ISG15 HERC6 FAM20A | 0,871 | 0,861 | 0,882 | 0,905 | 0,844 | 0,967 | 0,926 | 0,843 | 1 |
| IFI27 ISG15 HERC6 SLC1A2 RETN MMP8 | 0,871 | 0,862 | 0,881 | 0,907 | 0,846 | 0,969 | 0,938 | 0,870 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 SLC1A2 IL1R2 OLAH RETN | 0,871 | 0,862 | 0,881 | 0,914 | 0,858 | 0,969 | 0,964 | 0,927 | 1 |
| IFIT1 IFI44L SIGLEC1 HERC6 OLAH RETN | 0,871 | 0,862 | 0,881 | 0,901 | 0,836 | 0,966 | 0,937 | 0,883 | 0,991 |
| IFI44L ISG15 IL1R2 OLAH FAM20A MMP8 | 0,871 | 0,862 | 0,880 | 0,905 | 0,844 | 0,965 | 0,948 | 0,884 | 1 |
| RSAD2 SIGLEC1 SLC1A2 FAM20A RETN MMP8 | 0,871 | 0,861 | 0,881 | 0,908 | 0,848 | 0,969 | 0,953 | 0,885 | 1 |
| RSAD2 IFIT1 HERC6 IL1R2 OLAH FAM20A | 0,871 | 0,862 | 0,881 | 0,906 | 0,845 | 0,967 | 0,937 | 0,863 | 1 |
| RSAD2 OAS1 ISG15 IL1R2 OLAH FAM20A | 0,871 | 0,862 | 0,881 | 0,900 | 0,834 | 0,966 | 0,940 | 0,866 | 1 |
| RSAD2 OAS1 SIGLEC1 ISG15 HERC6 IL1R2 | 0,871 | 0,862 | 0,880 | 0,904 | 0,846 | 0,961 | 0,959 | 0,917 | 1 |
| IFIT1 SIGLEC1 SLC1A2 OLAH RETN MMP8 | 0,871 | 0,862 | 0,881 | 0,912 | 0,856 | 0,968 | 0,959 | 0,917 | 1 |
| IFIT1 SIGLEC1 HERC6 SLC1A2 IL1R2 MMP8 | 0,871 | 0,862 | 0,880 | 0,909 | 0,856 | 0,963 | 0,964 | 0,927 | 1 |
| RSAD2 IFI44L SIGLEC1 SLC1A2 OLAH MMP8 | 0,871 | 0,862 | 0,881 | 0,911 | 0,856 | 0,967 | 0,955 | 0,913 | 0,998 |
| RSAD2 IFI44L SIGLEC1 ISG15 HERC6 FAM20A | 0,871 | 0,862 | 0,881 | 0,902 | 0,838 | 0,966 | 0,943 | 0,875 | 1 |
| RSAD2 IFI44L HERC6 SLC1A2 OLAH FAM20A | 0,871 | 0,861 | 0,882 | 0,913 | 0,858 | 0,969 | 0,924 | 0,843 | 1 |
| RSAD2 IFI27 OAS1 IFIT1 HERC6 FAM20A | 0,871 | 0,861 | 0,882 | 0,904 | 0,843 | 0,965 | 0,932 | 0,849 | 1 |
| RSAD2 SIGLEC1 ISG15 HERC6 SLC1A2 OLAH | 0,871 | 0,862 | 0,880 | 0,910 | 0,854 | 0,966 | 0,947 | 0,898 | 0,995 |
| OAS1 ISG15 IL1R2 OLAH FAM20A MMP8 | 0,871 | 0,862 | 0,881 | 0,904 | 0,841 | 0,966 | 0,937 | 0,859 | 1 |
| RSAD2 OAS1 SIGLEC1 HERC6 SLC1A2 IL1R2 | 0,871 | 0,862 | 0,881 | 0,909 | 0,855 | 0,963 | 0,966 | 0,929 | 1 |
| OAS1 SIGLEC1 HERC6 SLC1A2 OLAH RETN | 0,871 | 0,861 | 0,881 | 0,912 | 0,856 | 0,968 | 0,948 | 0,897 | 0,999 |
| OAS1 SIGLEC1 SLC1A2 OLAH RETN MMP8 | 0,871 | 0,861 | 0,881 | 0,912 | 0,854 | 0,969 | 0,960 | 0,918 | 1 |
| RSAD2 IFIT1 SIGLEC1 ISG15 HERC6 IL1R2 | 0,871 | 0,862 | 0,880 | 0,904 | 0,846 | 0,963 | 0,952 | 0,907 | 0,998 |
| IFIT1 SIGLEC1 HERC6 IL1R2 OLAH RETN | 0,871 | 0,862 | 0,880 | 0,904 | 0,842 | 0,967 | 0,949 | 0,902 | 0,996 |
| RSAD2 SIGLEC1 ISG15 FAM20A RETN MMP8 | 0,871 | 0,861 | 0,881 | 0,903 | 0,836 | 0,970 | 0,957 | 0,898 | 1 |
| RSAD2 IFI27 OAS1 SLC1A2 FAM20A RETN | 0,871 | 0,861 | 0,881 | 0,912 | 0,851 | 0,972 | 0,941 | 0,861 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 HERC6 SLC1A2 IL1R2 MMP8 | 0,871 | 0,862 | 0,880 | 0,908 | 0,854 | 0,962 | 0,963 | 0,925 | 1 |
| OAS1 SIGLEC1 ISG15 SLC1A2 IL1R2 OLAH | 0,871 | 0,862 | 0,880 | 0,912 | 0,857 | 0,967 | 0,962 | 0,924 | 1 |
| OAS1 IFI44L SIGLEC1 ISG15 HERC6 IL1R2 | 0,871 | 0,862 | 0,880 | 0,904 | 0,845 | 0,962 | 0,955 | 0,911 | 1,000 |
| OAS1 SIGLEC1 ISG15 HERC6 IL1R2 MMP8 | 0,871 | 0,862 | 0,880 | 0,904 | 0,847 | 0,961 | 0,965 | 0,928 | 1 |
| IFI27 IFIT1 SLC1A2 FAM20A RETN MMP8 | 0,871 | 0,861 | 0,882 | 0,908 | 0,845 | 0,971 | 0,941 | 0,861 | 1 |
| SIGLEC1 ISG15 HERC6 SLC1A2 FAM20A RETN | 0,871 | 0,861 | 0,881 | 0,901 | 0,838 | 0,965 | 0,950 | 0,876 | 1 |
| RSAD2 IFIT1 IFI44L HERC6 IL1R2 FAM20A | 0,871 | 0,862 | 0,880 | 0,907 | 0,847 | 0,967 | 0,934 | 0,853 | 1 |
| OAS1 IFIT1 SLC1A2 IL1R2 FAM20A MMP8 | 0,871 | 0,862 | 0,880 | 0,912 | 0,858 | 0,966 | 0,934 | 0,852 | 1 |
| OAS1 SIGLEC1 SLC1A2 FAM20A RETN MMP8 | 0,871 | 0,861 | 0,881 | 0,904 | 0,842 | 0,967 | 0,950 | 0,880 | 1 |
| IFI44L SIGLEC1 HERC6 SLC1A2 OLAH MMP8 | 0,871 | 0,861 | 0,881 | 0,911 | 0,855 | 0,967 | 0,957 | 0,915 | 0,998 |
| OAS1 IFIT1 IFI44L HERC6 OLAH FAM20A | 0,871 | 0,861 | 0,881 | 0,901 | 0,837 | 0,965 | 0,937 | 0,869 | 1 |
| OAS1 IFI44L IL1R2 OLAH FAM20A MMP8 | 0,871 | 0,861 | 0,881 | 0,905 | 0,844 | 0,967 | 0,943 | 0,872 | 1 |
| IFIT1 SIGLEC1 ISG15 SLC1A2 OLAH RETN | 0,871 | 0,862 | 0,880 | 0,914 | 0,859 | 0,970 | 0,948 | 0,897 | 0,999 |
| IFIT1 IFI44L SIGLEC1 HERC6 IL1R2 OLAH | 0,871 | 0,862 | 0,880 | 0,905 | 0,846 | 0,964 | 0,952 | 0,907 | 0,997 |
| IFI44L SIGLEC1 HERC6 SLC1A2 IL1R2 OLAH | 0,871 | 0,861 | 0,881 | 0,910 | 0,855 | 0,966 | 0,960 | 0,919 | 1 |
| IFIT1 ISG15 HERC6 OLAH FAM20A RETN | 0,871 | 0,861 | 0,881 | 0,901 | 0,838 | 0,965 | 0,943 | 0,877 | 1 |
| IFI27 IFI44L SIGLEC1 ISG15 HERC6 MMP8 | 0,871 | 0,862 | 0,880 | 0,897 | 0,835 | 0,959 | 0,910 | 0,836 | 0,983 |
| RSAD2 IFI27 IFI44L SIGLEC1 HERC6 MMP8 | 0,871 | 0,862 | 0,880 | 0,901 | 0,843 | 0,959 | 0,907 | 0,831 | 0,982 |
| IFIT1 SIGLEC1 SLC1A2 IL1R2 OLAH RETN | 0,871 | 0,862 | 0,880 | 0,914 | 0,858 | 0,969 | 0,958 | 0,914 | 1 |
| ISG15 HERC6 SLC1A2 IL1R2 FAM20A RETN | 0,871 | 0,861 | 0,881 | 0,912 | 0,858 | 0,966 | 0,926 | 0,837 | 1 |
| RSAD2 IFI44L SIGLEC1 ISG15 HERC6 IL1R2 | 0,871 | 0,862 | 0,880 | 0,900 | 0,838 | 0,962 | 0,958 | 0,916 | 0,999 |
| SIGLEC1 ISG15 HERC6 IL1R2 RETN MMP8 | 0,871 | 0,861 | 0,880 | 0,901 | 0,836 | 0,966 | 0,970 | 0,937 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L SIGLEC1 HERC6 FAM20A RETN | 0,871 | 0,861 | 0,880 | 0,907 | 0,844 | 0,970 | 0,943 | 0,876 | 1 |
| IFIT1 HERC6 SLC1A2 IL1R2 OLAH FAM20A | 0,871 | 0,861 | 0,881 | 0,912 | 0,857 | 0,967 | 0,927 | 0,843 | 1 |
| RSAD2 IFI44L SIGLEC1 HERC6 SLC1A2 FAM20A | 0,871 | 0,861 | 0,880 | 0,909 | 0,852 | 0,966 | 0,939 | 0,863 | 1 |
| RSAD2 IFI44L SIGLEC1 ISG15 OLAH FAM20A | 0,871 | 0,861 | 0,881 | 0,897 | 0,826 | 0,968 | 0,963 | 0,921 | 1 |
| IFI44L SIGLEC1 ISG15 HERC6 SLC1A2 OLAH | 0,871 | 0,861 | 0,880 | 0,910 | 0,854 | 0,966 | 0,950 | 0,903 | 0,997 |
| OAS1 SIGLEC1 ISG15 HERC6 SLC1A2 FAM20A | 0,871 | 0,861 | 0,881 | 0,908 | 0,851 | 0,965 | 0,943 | 0,871 | 1 |
| OAS1 IFI44L HERC6 IL1R2 FAM20A RETN | 0,871 | 0,861 | 0,880 | 0,907 | 0,845 | 0,968 | 0,937 | 0,857 | 1 |
| RSAD2 IFIT1 SIGLEC1 HERC6 IL1R2 OLAH | 0,871 | 0,861 | 0,880 | 0,907 | 0,849 | 0,966 | 0,952 | 0,907 | 0,997 |
| RSAD2 OAS1 SLC1A2 IL1R2 OLAH FAM20A | 0,871 | 0,861 | 0,880 | 0,912 | 0,857 | 0,968 | 0,933 | 0,850 | 1 |
| SIGLEC1 ISG15 SLC1A2 OLAH RETN MMP8 | 0,871 | 0,861 | 0,880 | 0,910 | 0,852 | 0,968 | 0,959 | 0,917 | 1,000 |
| OAS1 ISG15 HERC6 SLC1A2 OLAH FAM20A | 0,871 | 0,860 | 0,881 | 0,912 | 0,856 | 0,967 | 0,929 | 0,849 | 1 |
| IFIT1 SIGLEC1 ISG15 HERC6 SLC1A2 IL1R2 | 0,871 | 0,862 | 0,879 | 0,909 | 0,855 | 0,963 | 0,962 | 0,923 | 1 |
| RSAD2 SIGLEC1 HERC6 SLC1A2 IL1R2 RETN | 0,871 | 0,861 | 0,880 | 0,910 | 0,854 | 0,965 | 0,958 | 0,916 | 1,000 |
| RSAD2 IFI27 IFIT1 SLC1A2 FAM20A RETN | 0,871 | 0,861 | 0,881 | 0,908 | 0,847 | 0,969 | 0,940 | 0,859 | 1 |
| IFIT1 IFI44L IL1R2 OLAH FAM20A MMP8 | 0,871 | 0,861 | 0,880 | 0,906 | 0,845 | 0,967 | 0,950 | 0,888 | 1 |
| SIGLEC1 HERC6 IL1R2 OLAH RETN MMP8 | 0,871 | 0,861 | 0,881 | 0,901 | 0,836 | 0,965 | 0,968 | 0,935 | 1 |
| SIGLEC1 ISG15 HERC6 IL1R2 OLAH MMP8 | 0,871 | 0,861 | 0,880 | 0,904 | 0,845 | 0,963 | 0,964 | 0,927 | 1 |
| RSAD2 IFI44L HERC6 SLC1A2 IL1R2 FAM20A | 0,871 | 0,861 | 0,880 | 0,913 | 0,860 | 0,965 | 0,924 | 0,834 | 1 |
| OAS1 SIGLEC1 SLC1A2 IL1R2 OLAH RETN | 0,871 | 0,861 | 0,880 | 0,913 | 0,857 | 0,968 | 0,962 | 0,922 | 1 |
| IFI44L SIGLEC1 ISG15 HERC6 SLC1A2 FAM20A | 0,871 | 0,861 | 0,880 | 0,902 | 0,840 | 0,964 | 0,952 | 0,879 | 1 |
| IFI44L ISG15 SLC1A2 IL1R2 FAM20A RETN | 0,871 | 0,861 | 0,880 | 0,908 | 0,854 | 0,963 | 0,928 | 0,842 | 1 |
| IFI44L SLC1A2 IL1R2 FAM20A RETN MMP8 | 0,871 | 0,862 | 0,879 | 0,913 | 0,861 | 0,965 | 0,927 | 0,836 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 IFIT1 IL1R2 OLAH FAM20A | 0,871 | 0,861 | 0,880 | 0,901 | 0,835 | 0,968 | 0,945 | 0,873 | 1 |
| SIGLEC1 HERC6 SLC1A2 OLAH RETN MMP8 | 0,871 | 0,860 | 0,881 | 0,909 | 0,850 | 0,968 | 0,964 | 0,927 | 1 |
| OAS1 HERC6 SLC1A2 IL1R2 FAM20A MMP8 | 0,871 | 0,861 | 0,880 | 0,914 | 0,862 | 0,966 | 0,924 | 0,831 | 1 |
| RSAD2 OAS1 HERC6 SLC1A2 IL1R2 FAM20A | 0,870 | 0,861 | 0,880 | 0,910 | 0,856 | 0,964 | 0,927 | 0,837 | 1 |
| RSAD2 HERC6 IL1R2 FAM20A RETN MMP8 | 0,870 | 0,861 | 0,880 | 0,906 | 0,844 | 0,968 | 0,937 | 0,854 | 1 |
| RSAD2 IFI27 ISG15 SLC1A2 FAM20A RETN | 0,870 | 0,860 | 0,881 | 0,909 | 0,848 | 0,971 | 0,936 | 0,854 | 1 |
| IFIT1 IFI44L SLC1A2 OLAH FAM20A RETN | 0,870 | 0,861 | 0,880 | 0,910 | 0,851 | 0,968 | 0,918 | 0,841 | 0,996 |
| RSAD2 OAS1 IFI44L IL1R2 OLAH FAM20A | 0,870 | 0,861 | 0,880 | 0,901 | 0,835 | 0,966 | 0,946 | 0,878 | 1 |
| OAS1 IFI44L HERC6 SLC1A2 IL1R2 FAM20A | 0,870 | 0,861 | 0,880 | 0,912 | 0,860 | 0,965 | 0,926 | 0,836 | 1 |
| RSAD2 SIGLEC1 ISG15 HERC6 IL1R2 RETN | 0,870 | 0,861 | 0,880 | 0,904 | 0,842 | 0,965 | 0,955 | 0,912 | 0,999 |
| RSAD2 OAS1 HERC6 SLC1A2 OLAH FAM20A | 0,870 | 0,860 | 0,880 | 0,910 | 0,854 | 0,966 | 0,930 | 0,850 | 1 |
| OAS1 IFI44L SIGLEC1 HERC6 IL1R2 OLAH | 0,870 | 0,861 | 0,880 | 0,904 | 0,844 | 0,964 | 0,958 | 0,916 | 1,000 |
| OAS1 IFIT1 SIGLEC1 HERC6 SLC1A2 RETN | 0,870 | 0,861 | 0,880 | 0,906 | 0,847 | 0,965 | 0,927 | 0,867 | 0,987 |
| IFI27 IFIT1 IFI44L ISG15 FAM20A MMP8 | 0,870 | 0,860 | 0,881 | 0,900 | 0,833 | 0,966 | 0,933 | 0,853 | 1 |
| HERC6 SLC1A2 IL1R2 OLAH FAM20A RETN | 0,870 | 0,860 | 0,880 | 0,913 | 0,859 | 0,967 | 0,922 | 0,834 | 1 |
| RSAD2 IFIT1 IFI44L SIGLEC1 SLC1A2 OLAH | 0,870 | 0,861 | 0,879 | 0,913 | 0,856 | 0,969 | 0,947 | 0,897 | 0,996 |
| RSAD2 SIGLEC1 ISG15 HERC6 SLC1A2 IL1R2 | 0,870 | 0,862 | 0,879 | 0,909 | 0,855 | 0,964 | 0,960 | 0,919 | 1 |
| IFIT1 HERC6 SLC1A2 OLAH FAM20A MMP8 | 0,870 | 0,860 | 0,881 | 0,910 | 0,854 | 0,966 | 0,932 | 0,853 | 1 |
| RSAD2 IFI27 HERC6 SLC1A2 FAM20A MMP8 | 0,870 | 0,860 | 0,880 | 0,911 | 0,855 | 0,967 | 0,928 | 0,845 | 1 |
| RSAD2 SIGLEC1 HERC6 SLC1A2 OLAH RETN | 0,870 | 0,860 | 0,880 | 0,913 | 0,857 | 0,970 | 0,946 | 0,896 | 0,995 |
| IFI44L SIGLEC1 ISG15 SLC1A2 FAM20A RETN | 0,870 | 0,861 | 0,880 | 0,901 | 0,837 | 0,965 | 0,948 | 0,875 | 1 |
| OAS1 IFIT1 ISG15 IL1R2 FAM20A MMP8 | 0,870 | 0,861 | 0,880 | 0,905 | 0,843 | 0,968 | 0,943 | 0,870 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 SIGLEC1 ISG15 SLC1A2 OLAH | 0,870 | 0,861 | 0,879 | 0,912 | 0,855 | 0,968 | 0,946 | 0,895 | 0,996 |
| RSAD2 OAS1 IFI44L IL1R2 FAM20A MMP8 | 0,870 | 0,861 | 0,880 | 0,905 | 0,845 | 0,966 | 0,937 | 0,859 | 1 |
| IFI44L SIGLEC1 ISG15 SLC1A2 IL1R2 MMP8 | 0,870 | 0,862 | 0,878 | 0,908 | 0,854 | 0,962 | 0,966 | 0,930 | 1 |
| IFI27 IFIT1 HERC6 SLC1A2 FAM20A MMP8 | 0,870 | 0,860 | 0,880 | 0,911 | 0,855 | 0,967 | 0,927 | 0,843 | 1 |
| RSAD2 SIGLEC1 SLC1A2 OLAH RETN MMP8 | 0,870 | 0,861 | 0,880 | 0,910 | 0,853 | 0,968 | 0,960 | 0,919 | 1 |
| IFI44L SIGLEC1 ISG15 IL1R2 OLAH MMP8 | 0,870 | 0,862 | 0,879 | 0,905 | 0,848 | 0,962 | 0,957 | 0,914 | 0,999 |
| RSAD2 IFIT1 SLC1A2 IL1R2 FAM20A MMP8 | 0,870 | 0,861 | 0,879 | 0,910 | 0,855 | 0,965 | 0,932 | 0,849 | 1 |
| OAS1 IFIT1 ISG15 IL1R2 OLAH FAM20A | 0,870 | 0,861 | 0,880 | 0,901 | 0,835 | 0,967 | 0,945 | 0,873 | 1 |
| OAS1 SIGLEC1 ISG15 FAM20A RETN MMP8 | 0,870 | 0,860 | 0,881 | 0,901 | 0,832 | 0,971 | 0,958 | 0,898 | 1 |
| OAS1 SIGLEC1 HERC6 SLC1A2 FAM20A MMP8 | 0,870 | 0,860 | 0,880 | 0,908 | 0,851 | 0,965 | 0,943 | 0,870 | 1 |
| OAS1 IFI44L SIGLEC1 HERC6 SLC1A2 OLAH | 0,870 | 0,860 | 0,880 | 0,912 | 0,856 | 0,967 | 0,951 | 0,904 | 0,998 |
| OAS1 IFI44L SLC1A2 IL1R2 OLAH FAM20A | 0,870 | 0,860 | 0,880 | 0,913 | 0,857 | 0,969 | 0,930 | 0,852 | 1 |
| RSAD2 IFI44L SIGLEC1 HERC6 IL1R2 RETN | 0,870 | 0,861 | 0,879 | 0,901 | 0,837 | 0,965 | 0,960 | 0,920 | 1 |
| RSAD2 OAS1 SIGLEC1 SLC1A2 FAM20A MMP8 | 0,870 | 0,860 | 0,880 | 0,903 | 0,840 | 0,966 | 0,946 | 0,873 | 1 |
| RSAD2 OAS1 SIGLEC1 ISG15 SLC1A2 OLAH | 0,870 | 0,860 | 0,880 | 0,910 | 0,854 | 0,967 | 0,948 | 0,898 | 0,998 |
| IFI27 OAS1 HERC6 SLC1A2 FAM20A MMP8 | 0,870 | 0,860 | 0,880 | 0,915 | 0,860 | 0,969 | 0,925 | 0,840 | 1 |
| RSAD2 ISG15 HERC6 SLC1A2 OLAH FAM20A | 0,870 | 0,860 | 0,880 | 0,911 | 0,855 | 0,967 | 0,932 | 0,852 | 1 |
| IFIT1 IFI44L SIGLEC1 SLC1A2 IL1R2 OLAH | 0,870 | 0,861 | 0,879 | 0,914 | 0,859 | 0,968 | 0,963 | 0,925 | 1 |
| RSAD2 OAS1 SIGLEC1 ISG15 FAM20A RETN | 0,870 | 0,860 | 0,880 | 0,896 | 0,826 | 0,967 | 0,954 | 0,895 | 1 |
| OAS1 IFIT1 HERC6 SLC1A2 IL1R2 FAM20A | 0,870 | 0,860 | 0,880 | 0,913 | 0,859 | 0,966 | 0,932 | 0,848 | 1 |
| OAS1 HERC6 SLC1A2 OLAH FAM20A MMP8 | 0,870 | 0,860 | 0,880 | 0,912 | 0,857 | 0,967 | 0,932 | 0,852 | 1 |
| RSAD2 IFIT1 SIGLEC1 HERC6 SLC1A2 IL1R2 | 0,870 | 0,861 | 0,879 | 0,908 | 0,853 | 0,963 | 0,963 | 0,925 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 SIGLEC1 IL1R2 OLAH MMP8 | 0,870 | 0,861 | 0,879 | 0,905 | 0,847 | 0,964 | 0,960 | 0,920 | 0,999 |
| OAS1 IFIT1 IFI44L IL1R2 FAM20A MMP8 | 0,870 | 0,861 | 0,879 | 0,904 | 0,841 | 0,967 | 0,943 | 0,868 | 1 |
| IFI44L SIGLEC1 ISG15 SLC1A2 IL1R2 RETN | 0,870 | 0,862 | 0,878 | 0,910 | 0,855 | 0,965 | 0,965 | 0,927 | 1 |
| RSAD2 SIGLEC1 ISG15 HERC6 IL1R2 OLAH | 0,870 | 0,861 | 0,879 | 0,905 | 0,845 | 0,964 | 0,958 | 0,916 | 0,999 |
| RSAD2 HERC6 SLC1A2 OLAH FAM20A MMP8 | 0,870 | 0,860 | 0,880 | 0,912 | 0,857 | 0,967 | 0,933 | 0,853 | 1 |
| IFIT1 ISG15 IL1R2 OLAH FAM20A MMP8 | 0,870 | 0,860 | 0,880 | 0,906 | 0,845 | 0,967 | 0,949 | 0,883 | 1 |
| OAS1 IFI44L SIGLEC1 OLAH FAM20A RETN | 0,870 | 0,860 | 0,880 | 0,896 | 0,823 | 0,968 | 0,964 | 0,921 | 1 |
| RSAD2 IFIT1 IL1R2 OLAH FAM20A MMP8 | 0,870 | 0,861 | 0,879 | 0,905 | 0,843 | 0,967 | 0,946 | 0,876 | 1 |
| RSAD2 OAS1 IFI44L SIGLEC1 HERC6 FAM20A | 0,870 | 0,860 | 0,879 | 0,899 | 0,833 | 0,964 | 0,941 | 0,871 | 1 |
| IFIT1 IFI44L ISG15 SLC1A2 IL1R2 FAM20A | 0,870 | 0,861 | 0,879 | 0,908 | 0,853 | 0,964 | 0,924 | 0,836 | 1 |
| RSAD2 IFI44L SIGLEC1 HERC6 IL1R2 OLAH | 0,870 | 0,860 | 0,879 | 0,902 | 0,841 | 0,963 | 0,959 | 0,918 | 1,000 |
| IFIT1 IFI44L ISG15 HERC6 FAM20A RETN | 0,870 | 0,860 | 0,879 | 0,904 | 0,840 | 0,968 | 0,935 | 0,855 | 1 |
| IFI44L SIGLEC1 HERC6 IL1R2 OLAH RETN | 0,870 | 0,860 | 0,879 | 0,900 | 0,836 | 0,965 | 0,958 | 0,916 | 0,999 |
| OAS1 IFIT1 IFI44L SIGLEC1 IL1R2 MMP8 | 0,870 | 0,861 | 0,879 | 0,900 | 0,840 | 0,961 | 0,958 | 0,916 | 1,000 |
| ISG15 HERC6 SLC1A2 IL1R2 FAM20A MMP8 | 0,870 | 0,860 | 0,880 | 0,915 | 0,864 | 0,966 | 0,921 | 0,824 | 1 |
| IFI27 IFI44L HERC6 SLC1A2 FAM20A MMP8 | 0,870 | 0,859 | 0,880 | 0,906 | 0,846 | 0,966 | 0,930 | 0,847 | 1 |
| SIGLEC1 ISG15 HERC6 SLC1A2 IL1R2 MMP8 | 0,870 | 0,861 | 0,878 | 0,907 | 0,852 | 0,962 | 0,963 | 0,925 | 1 |
| RSAD2 IFIT1 SIGLEC1 SLC1A2 OLAH RETN | 0,870 | 0,860 | 0,879 | 0,914 | 0,858 | 0,970 | 0,952 | 0,905 | 0,999 |
| RSAD2 OAS1 SIGLEC1 SLC1A2 OLAH RETN | 0,870 | 0,860 | 0,879 | 0,912 | 0,856 | 0,969 | 0,958 | 0,910 | 1 |
| RSAD2 IFIT1 HERC6 IL1R2 FAM20A RETN | 0,870 | 0,860 | 0,879 | 0,904 | 0,841 | 0,966 | 0,938 | 0,857 | 1 |
| OAS1 IFI44L SIGLEC1 SLC1A2 IL1R2 OLAH | 0,870 | 0,860 | 0,879 | 0,913 | 0,858 | 0,968 | 0,963 | 0,925 | 1 |
| OAS1 IFI44L SIGLEC1 SLC1A2 OLAH RETN | 0,870 | 0,860 | 0,880 | 0,914 | 0,858 | 0,970 | 0,950 | 0,900 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 SIGLEC1 ISG15 HERC6 MMP8 | 0,870 | 0,861 | 0,879 | 0,902 | 0,844 | 0,959 | 0,909 | 0,834 | 0,983 |
| SIGLEC1 HERC6 SLC1A2 IL1R2 OLAH RETN | 0,870 | 0,860 | 0,880 | 0,914 | 0,860 | 0,969 | 0,959 | 0,917 | 1 |
| IFI27 OAS1 IFIT1 IFI44L ISG15 RETN | 0,870 | 0,860 | 0,880 | 0,906 | 0,843 | 0,969 | 0,952 | 0,890 | 1 |
| OAS1 IFI44L SIGLEC1 ISG15 SLC1A2 OLAH | 0,870 | 0,860 | 0,879 | 0,913 | 0,857 | 0,968 | 0,947 | 0,897 | 0,997 |
| RSAD2 OAS1 SIGLEC1 ISG15 SLC1A2 FAM20A | 0,870 | 0,860 | 0,880 | 0,902 | 0,839 | 0,965 | 0,947 | 0,874 | 1 |
| IFI44L SIGLEC1 HERC6 SLC1A2 IL1R2 MMP8 | 0,870 | 0,861 | 0,879 | 0,907 | 0,853 | 0,962 | 0,964 | 0,927 | 1 |
| RSAD2 IFI44L SIGLEC1 SLC1A2 IL1R2 OLAH | 0,870 | 0,860 | 0,879 | 0,912 | 0,857 | 0,968 | 0,965 | 0,929 | 1 |
| RSAD2 IFI44L SIGLEC1 HERC6 SLC1A2 OLAH | 0,870 | 0,860 | 0,879 | 0,912 | 0,857 | 0,968 | 0,947 | 0,898 | 0,995 |
| RSAD2 IFI27 IFI44L ISG15 FAM20A MMP8 | 0,870 | 0,859 | 0,880 | 0,899 | 0,832 | 0,965 | 0,937 | 0,860 | 1 |
| IFI44L HERC6 SLC1A2 OLAH FAM20A RETN | 0,870 | 0,859 | 0,880 | 0,913 | 0,857 | 0,970 | 0,934 | 0,856 | 1 |
| IFI27 OAS1 IFIT1 SIGLEC1 SLC1A2 MMP8 | 0,870 | 0,861 | 0,879 | 0,904 | 0,846 | 0,963 | 0,929 | 0,861 | 0,997 |
| IFI44L SIGLEC1 ISG15 SLC1A2 FAM20A MMP8 | 0,870 | 0,860 | 0,879 | 0,901 | 0,838 | 0,965 | 0,951 | 0,879 | 1 |
| OAS1 IFIT1 SIGLEC1 HERC6 OLAH MMP8 | 0,870 | 0,860 | 0,879 | 0,900 | 0,839 | 0,961 | 0,939 | 0,886 | 0,992 |
| RSAD2 HERC6 SLC1A2 IL1R2 FAM20A MMP8 | 0,870 | 0,860 | 0,879 | 0,914 | 0,862 | 0,965 | 0,925 | 0,832 | 1 |
| IFI44L HERC6 SLC1A2 IL1R2 FAM20A RETN | 0,870 | 0,860 | 0,880 | 0,913 | 0,860 | 0,966 | 0,925 | 0,836 | 1 |
| IFI44L HERC6 OLAH FAM20A RETN MMP8 | 0,870 | 0,859 | 0,880 | 0,902 | 0,836 | 0,969 | 0,948 | 0,884 | 1 |
| IFIT1 HERC6 OLAH FAM20A RETN MMP8 | 0,869 | 0,859 | 0,880 | 0,901 | 0,836 | 0,965 | 0,948 | 0,882 | 1 |
| IFIT1 ISG15 SLC1A2 IL1R2 OLAH FAM20A | 0,869 | 0,860 | 0,879 | 0,907 | 0,849 | 0,965 | 0,933 | 0,858 | 1 |
| OAS1 ISG15 SLC1A2 IL1R2 OLAH FAM20A | 0,869 | 0,860 | 0,879 | 0,911 | 0,854 | 0,968 | 0,927 | 0,844 | 1 |
| OAS1 SIGLEC1 ISG15 OLAH FAM20A RETN | 0,869 | 0,859 | 0,880 | 0,896 | 0,825 | 0,968 | 0,962 | 0,918 | 1 |
| OAS1 IFI44L ISG15 SLC1A2 OLAH FAM20A | 0,869 | 0,859 | 0,880 | 0,909 | 0,849 | 0,968 | 0,927 | 0,852 | 1 |
| IFI27 IFIT1 IFI44L HERC6 SLC1A2 FAM20A | 0,869 | 0,859 | 0,880 | 0,905 | 0,846 | 0,964 | 0,930 | 0,847 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 ISG15 SLC1A2 IL1R2 OLAH FAM20A | 0,869 | 0,860 | 0,879 | 0,912 | 0,856 | 0,968 | 0,932 | 0,851 | 1 |
| RSAD2 IFI27 SIGLEC1 HERC6 SLC1A2 MMP8 | 0,869 | 0,860 | 0,878 | 0,903 | 0,846 | 0,959 | 0,926 | 0,859 | 0,993 |
| OAS1 IFIT1 SLC1A2 IL1R2 OLAH FAM20A | 0,869 | 0,860 | 0,879 | 0,911 | 0,853 | 0,968 | 0,935 | 0,856 | 1 |
| IFI27 OAS1 SLC1A2 FAM20A RETN MMP8 | 0,869 | 0,859 | 0,880 | 0,911 | 0,849 | 0,972 | 0,940 | 0,858 | 1 |
| OAS1 IFI44L SIGLEC1 HERC6 SLC1A2 FAM20A | 0,869 | 0,860 | 0,879 | 0,907 | 0,850 | 0,964 | 0,943 | 0,870 | 1 |
| RSAD2 IFI44L ISG15 SLC1A2 OLAH FAM20A | 0,869 | 0,860 | 0,879 | 0,908 | 0,849 | 0,968 | 0,926 | 0,852 | 1 |
| IFIT1 IFI44L SIGLEC1 HERC6 OLAH MMP8 | 0,869 | 0,860 | 0,879 | 0,899 | 0,837 | 0,961 | 0,937 | 0,882 | 0,992 |
| ISG15 HERC6 OLAH FAM20A RETN MMP8 | 0,869 | 0,859 | 0,880 | 0,903 | 0,838 | 0,968 | 0,951 | 0,888 | 1 |
| OAS1 IFIT1 SIGLEC1 SLC1A2 IL1R2 RETN | 0,869 | 0,860 | 0,879 | 0,910 | 0,855 | 0,965 | 0,962 | 0,922 | 1 |
| SIGLEC1 ISG15 HERC6 SLC1A2 OLAH RETN | 0,869 | 0,859 | 0,879 | 0,909 | 0,850 | 0,967 | 0,954 | 0,910 | 0,999 |
| RSAD2 IFI27 OAS1 IFIT1 ISG15 RETN | 0,869 | 0,860 | 0,879 | 0,904 | 0,841 | 0,967 | 0,950 | 0,887 | 1 |
| OAS1 IFIT1 SIGLEC1 HERC6 RETN MMP8 | 0,869 | 0,860 | 0,879 | 0,898 | 0,831 | 0,965 | 0,924 | 0,863 | 0,984 |
| SIGLEC1 HERC6 SLC1A2 IL1R2 RETN MMP8 | 0,869 | 0,860 | 0,879 | 0,909 | 0,854 | 0,965 | 0,971 | 0,939 | 1 |
| RSAD2 IFI27 SLC1A2 FAM20A RETN MMP8 | 0,869 | 0,859 | 0,879 | 0,910 | 0,848 | 0,971 | 0,938 | 0,857 | 1 |
| IFI44L SIGLEC1 SLC1A2 IL1R2 RETN MMP8 | 0,869 | 0,861 | 0,878 | 0,912 | 0,858 | 0,966 | 0,966 | 0,930 | 1 |
| OAS1 SIGLEC1 ISG15 HERC6 IL1R2 OLAH | 0,869 | 0,860 | 0,879 | 0,906 | 0,848 | 0,964 | 0,963 | 0,924 | 1 |
| RSAD2 IFIT1 SIGLEC1 HERC6 OLAH RETN | 0,869 | 0,860 | 0,879 | 0,900 | 0,835 | 0,965 | 0,938 | 0,885 | 0,991 |
| IFI44L SIGLEC1 HERC6 SLC1A2 IL1R2 RETN | 0,869 | 0,860 | 0,878 | 0,908 | 0,852 | 0,964 | 0,955 | 0,912 | 0,999 |
| IFIT1 ISG15 SLC1A2 IL1R2 FAM20A MMP8 | 0,869 | 0,860 | 0,878 | 0,909 | 0,854 | 0,965 | 0,935 | 0,855 | 1 |
| RSAD2 SIGLEC1 ISG15 SLC1A2 FAM20A MMP8 | 0,869 | 0,859 | 0,879 | 0,902 | 0,839 | 0,964 | 0,949 | 0,878 | 1 |
| IFIT1 SIGLEC1 ISG15 HERC6 OLAH MMP8 | 0,869 | 0,860 | 0,879 | 0,899 | 0,837 | 0,962 | 0,943 | 0,893 | 0,994 |
| RSAD2 IFI27 OAS1 IFI44L FAM20A MMP8 | 0,869 | 0,859 | 0,880 | 0,900 | 0,833 | 0,966 | 0,934 | 0,855 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 SIGLEC1 ISG15 SLC1A2 OLAH RETN | 0,869 | 0,860 | 0,879 | 0,912 | 0,856 | 0,968 | 0,951 | 0,903 | 0,999 |
| RSAD2 SIGLEC1 ISG15 OLAH FAM20A RETN | 0,869 | 0,859 | 0,879 | 0,897 | 0,826 | 0,969 | 0,961 | 0,917 | 1 |
| IFI44L SIGLEC1 HERC6 SLC1A2 FAM20A MMP8 | 0,869 | 0,859 | 0,879 | 0,904 | 0,842 | 0,966 | 0,953 | 0,880 | 1 |
| ISG15 HERC6 SLC1A2 OLAH FAM20A RETN | 0,869 | 0,859 | 0,879 | 0,911 | 0,855 | 0,968 | 0,932 | 0,851 | 1 |
| RSAD2 IFIT1 IFI44L SLC1A2 OLAH FAM20A | 0,869 | 0,860 | 0,878 | 0,909 | 0,850 | 0,968 | 0,922 | 0,845 | 0,998 |
| IFIT1 SIGLEC1 ISG15 SLC1A2 IL1R2 RETN | 0,869 | 0,861 | 0,878 | 0,909 | 0,854 | 0,964 | 0,965 | 0,927 | 1 |
| RSAD2 OAS1 SIGLEC1 IL1R2 OLAH MMP8 | 0,869 | 0,859 | 0,879 | 0,904 | 0,845 | 0,963 | 0,962 | 0,924 | 1 |
| IFIT1 IFI44L SIGLEC1 HERC6 SLC1A2 IL1R2 | 0,869 | 0,860 | 0,878 | 0,908 | 0,853 | 0,963 | 0,959 | 0,918 | 1,000 |
| IFIT1 ISG15 HERC6 SLC1A2 OLAH FAM20A | 0,869 | 0,859 | 0,879 | 0,911 | 0,854 | 0,967 | 0,932 | 0,852 | 1 |
| HERC6 SLC1A2 OLAH FAM20A RETN MMP8 | 0,869 | 0,859 | 0,879 | 0,911 | 0,854 | 0,967 | 0,934 | 0,855 | 1 |
| IFIT1 IFI44L SIGLEC1 IL1R2 OLAH MMP8 | 0,869 | 0,860 | 0,878 | 0,904 | 0,847 | 0,962 | 0,959 | 0,917 | 1 |
| OAS1 SIGLEC1 HERC6 IL1R2 OLAH RETN | 0,869 | 0,859 | 0,879 | 0,903 | 0,840 | 0,966 | 0,955 | 0,911 | 1,000 |
| OAS1 IFI44L ISG15 IL1R2 OLAH FAM20A | 0,869 | 0,859 | 0,879 | 0,901 | 0,836 | 0,967 | 0,948 | 0,880 | 1 |
| OAS1 IFIT1 HERC6 SLC1A2 OLAH FAM20A | 0,869 | 0,859 | 0,879 | 0,910 | 0,854 | 0,966 | 0,930 | 0,849 | 1 |
| RSAD2 OAS1 IFI44L SIGLEC1 SLC1A2 OLAH | 0,869 | 0,859 | 0,879 | 0,912 | 0,856 | 0,968 | 0,951 | 0,903 | 1,000 |
| OAS1 IFIT1 SIGLEC1 SLC1A2 IL1R2 MMP8 | 0,869 | 0,860 | 0,878 | 0,910 | 0,856 | 0,964 | 0,967 | 0,932 | 1 |
| OAS1 IFIT1 SIGLEC1 IL1R2 RETN MMP8 | 0,869 | 0,859 | 0,879 | 0,900 | 0,836 | 0,964 | 0,965 | 0,929 | 1 |
| SIGLEC1 ISG15 HERC6 SLC1A2 FAM20A MMP8 | 0,869 | 0,859 | 0,879 | 0,900 | 0,838 | 0,963 | 0,952 | 0,879 | 1 |
| OAS1 IFIT1 IFI44L SLC1A2 OLAH FAM20A | 0,869 | 0,859 | 0,879 | 0,910 | 0,852 | 0,968 | 0,923 | 0,845 | 1 |
| RSAD2 OAS1 IL1R2 OLAH FAM20A RETN | 0,869 | 0,859 | 0,879 | 0,900 | 0,834 | 0,965 | 0,930 | 0,852 | 1 |
| RSAD2 IFIT1 IFI44L HERC6 OLAH FAM20A | 0,869 | 0,859 | 0,879 | 0,903 | 0,840 | 0,965 | 0,934 | 0,864 | 1 |
| IFI44L SIGLEC1 IL1R2 OLAH RETN MMP8 | 0,869 | 0,859 | 0,878 | 0,902 | 0,840 | 0,964 | 0,963 | 0,925 | 1 |

(suite)

| Modèle | AUC modèle | IC 95% | | AUC Train | IC 95% Train | | AUC Test | IC 95% Test | |
|---|---|---|---|---|---|---|---|---|---|
| ISG15 HERC6 SLC1A2 OLAH FAM20A MMP8 | 0,869 | 0,859 | 0,879 | 0,912 | 0,857 | 0,966 | 0,929 | 0,847 | 1 |
| RSAD2 OAS1 SLC1A2 OLAH FAM20A MMP8 | 0,869 | 0,859 | 0,878 | 0,909 | 0,852 | 0,966 | 0,934 | 0,858 | 1 |
| RSAD2 IFI44L SIGLEC1 SLC1A2 FAM20A MMP8 | 0,869 | 0,859 | 0,879 | 0,903 | 0,841 | 0,966 | 0,945 | 0,873 | 1 |
| IFI44L ISG15 HERC6 OLAH FAM20A RETN | 0,869 | 0,859 | 0,879 | 0,900 | 0,833 | 0,967 | 0,948 | 0,884 | 1 |
| OAS1 SIGLEC1 ISG15 SLC1A2 OLAH RETN | 0,869 | 0,859 | 0,879 | 0,914 | 0,858 | 0,969 | 0,948 | 0,897 | 0,999 |
| IFI27 OAS1 IFIT1 ISG15 HERC6 MMP8 | 0,869 | 0,860 | 0,877 | 0,900 | 0,843 | 0,957 | 0,900 | 0,823 | 0,977 |
| IFIT1 IFI44L SIGLEC1 ISG15 HERC6 RETN | 0,869 | 0,859 | 0,878 | 0,896 | 0,828 | 0,963 | 0,933 | 0,877 | 0,988 |
| OAS1 IFIT1 IL1R2 FAM20A RETN MMP8 | 0,869 | 0,859 | 0,878 | 0,905 | 0,842 | 0,969 | 0,948 | 0,874 | 1 |
| OAS 1 IL1R2 OLAH FAM20A RETN MMP8 | 0,869 | 0,859 | 0,879 | 0,907 | 0,845 | 0,969 | 0,940 | 0,863 | 1 |
| OAS1 IFIT1 IFI44L SIGLEC1 IL1R2 OLAH | 0,869 | 0,860 | 0,878 | 0,901 | 0,837 | 0,965 | 0,954 | 0,911 | 0,998 |
| RSAD2 IFI44L SLC1A2 IL1R2 FAM20A MMP8 | 0,869 | 0,860 | 0,878 | 0,911 | 0,859 | 0,963 | 0,927 | 0,838 | 1 |
| RSAD2 IFI44L HERC6 IL1R2 FAM20A RETN | 0,869 | 0,859 | 0,879 | 0,907 | 0,846 | 0,968 | 0,934 | 0,853 | 1 |
| OAS1 IFI44L SIGLEC1 FAM20A RETN MMP8 | 0,869 | 0,859 | 0,879 | 0,902 | 0,833 | 0,971 | 0,959 | 0,899 | 1 |
| OAS1 IFI44L SIGLEC1 IL1R2 OLAH MMP8 | 0,869 | 0,860 | 0,878 | 0,903 | 0,843 | 0,963 | 0,957 | 0,914 | 0,999 |
| IFI44L SIGLEC1 HERC6 SLC1A2 OLAH RETN | 0,869 | 0,859 | 0,879 | 0,909 | 0,851 | 0,968 | 0,957 | 0,913 | 1 |
| IFIT1 SIGLEC1 SLC1A2 IL1R2 RETN MMP8 | 0,869 | 0,860 | 0,878 | 0,911 | 0,857 | 0,966 | 0,970 | 0,937 | 1 |
| OAS1 IFI44L SIGLEC1 HERC6 SLC1A2 IL1R2 | 0,869 | 0,859 | 0,878 | 0,910 | 0,856 | 0,963 | 0,959 | 0,918 | 1,000 |
| IFIT1 SIGLEC1 ISG15 SLC1A2 IL1R2 MMP8 | 0,869 | 0,860 | 0,877 | 0,910 | 0,856 | 0,964 | 0,966 | 0,930 | 1 |
| OAS1 SIGLEC1 ISG15 HERC6 SLC1A2 RETN | 0,869 | 0,859 | 0,879 | 0,905 | 0,844 | 0,965 | 0,952 | 0,902 | 1 |
| IFI27 ISG15 SLC1A2 FAM20A RETN MMP8 | 0,869 | 0,858 | 0,879 | 0,905 | 0,842 | 0,968 | 0,928 | 0,842 | 1 |
| OAS1 IFIT1 SIGLEC1 ISG15 SLC1A2 IL1R2 | 0,869 | 0,860 | 0,878 | 0,908 | 0,853 | 0,964 | 0,964 | 0,927 | 1 |
| IFI27 ISG15 HERC6 SLC1A2 FAM20A MMP8 | 0,869 | 0,858 | 0,879 | 0,907 | 0,848 | 0,966 | 0,929 | 0,846 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L SIGLEC1 ISG15 IL1R2 RETN MMP8 | 0,869 | 0,859 | 0,878 | 0,902 | 0,840 | 0,965 | 0,963 | 0,925 | 1 |
| RSAD2 SLC1A2 IL1R2 OLAH FAM20A RETN | 0,869 | 0,859 | 0,878 | 0,910 | 0,854 | 0,967 | 0,930 | 0,849 | 1 |
| RSAD2 HERC6 SLC1A2 OLAH FAM20A RETN | 0,869 | 0,859 | 0,879 | 0,910 | 0,853 | 0,967 | 0,933 | 0,852 | 1 |
| RSAD2 OAS1 IFI44L SLC1A2 OLAH FAM20A | 0,869 | 0,859 | 0,879 | 0,910 | 0,852 | 0,969 | 0,924 | 0,848 | 1,000 |
| IFI27 IFIT1 SIGLEC1 ISG15 SLC1A2 MMP8 | 0,869 | 0,860 | 0,878 | 0,904 | 0,845 | 0,962 | 0,924 | 0,854 | 0,994 |
| RSAD2 IFI27 OAS1 IFIT1 IFI44L FAM20A | 0,869 | 0,858 | 0,879 | 0,901 | 0,835 | 0,968 | 0,938 | 0,862 | 1 |
| RSAD2 SIGLEC1 HERC6 IL1R2 OLAH RETN | 0,869 | 0,859 | 0,878 | 0,902 | 0,838 | 0,966 | 0,953 | 0,909 | 0,998 |
| RSAD2 OAS1 IFIT1 IFI44L IL1R2 FAM20A | 0,869 | 0,859 | 0,878 | 0,901 | 0,836 | 0,966 | 0,940 | 0,866 | 1 |
| OAS1 SLC1A2 IL1R2 OLAH FAM20A RETN | 0,869 | 0,859 | 0,878 | 0,912 | 0,856 | 0,969 | 0,926 | 0,842 | 1 |
| OAS1 IFIT1 IFI44L IL1R2 OLAH FAM20A | 0,869 | 0,859 | 0,878 | 0,900 | 0,834 | 0,967 | 0,947 | 0,879 | 1 |
| RSAD2 IFI27 OAS1 IFI44L ISG15 RETN | 0,869 | 0,859 | 0,878 | 0,906 | 0,843 | 0,969 | 0,952 | 0,890 | 1 |
| IFIT1 HERC6 SLC1A2 IL1R2 FAM20A RETN | 0,869 | 0,859 | 0,879 | 0,911 | 0,857 | 0,965 | 0,930 | 0,846 | 1 |
| OAS1 IFIT1 IFI44L SIGLEC1 HERC6 OLAH | 0,868 | 0,859 | 0,878 | 0,899 | 0,838 | 0,961 | 0,936 | 0,881 | 0,991 |
| OAS1 HERC6 SLC1A2 IL1R2 FAM20A RETN | 0,868 | 0,859 | 0,878 | 0,912 | 0,858 | 0,966 | 0,925 | 0,836 | 1 |
| OAS1 IFIT1 SIGLEC1 ISG15 IL1R2 OLAH | 0,868 | 0,860 | 0,877 | 0,901 | 0,839 | 0,964 | 0,957 | 0,914 | 0,999 |
| OAS1 ISG15 HERC6 OLAH FAM20A RETN | 0,868 | 0,858 | 0,878 | 0,904 | 0,842 | 0,967 | 0,947 | 0,882 | 1 |
| RSAD2 HERC6 SLC1A2 IL1R2 FAM20A RETN | 0,868 | 0,859 | 0,878 | 0,911 | 0,856 | 0,965 | 0,929 | 0,840 | 1 |
| OAS1 IFIT1 IFI44L SIGLEC1 ISG15 IL1R2 | 0,868 | 0,860 | 0,877 | 0,899 | 0,836 | 0,963 | 0,957 | 0,915 | 0,998 |
| RSAD2 IFIT1 SIGLEC1 SLC1A2 IL1R2 MMP8 | 0,868 | 0,860 | 0,877 | 0,909 | 0,854 | 0,963 | 0,965 | 0,929 | 1 |
| RSAD2 OAS1 ISG15 IL1R2 FAM20A MMP8 | 0,868 | 0,859 | 0,878 | 0,903 | 0,841 | 0,966 | 0,934 | 0,853 | 1 |
| RSAD2 OAS1 IFIT1 ISG15 IL1R2 FAM20A | 0,868 | 0,859 | 0,878 | 0,901 | 0,836 | 0,966 | 0,942 | 0,868 | 1 |
| SIGLEC1 ISG15 SLC1A2 IL1R2 RETN MMP8 | 0,868 | 0,860 | 0,877 | 0,912 | 0,858 | 0,966 | 0,970 | 0,937 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L SIGLEC1 ISG15 HERC6 SLC1A2 IL1R2 | 0,868 | 0,860 | 0,877 | 0,909 | 0,854 | 0,964 | 0,961 | 0,921 | 1 |
| HERC6 SLC1A2 IL1R2 FAM20A RETN MMP8 | 0,868 | 0,859 | 0,878 | 0,913 | 0,860 | 0,966 | 0,924 | 0,831 | 1 |
| OAS1 IFIT1 SLC1A2 OLAH FAM20A MMP8 | 0,868 | 0,858 | 0,878 | 0,909 | 0,850 | 0,967 | 0,940 | 0,870 | 1 |
| RSAD2 OAS1 SIGLEC1 ISG15 FAM20A MMP8 | 0,868 | 0,858 | 0,879 | 0,895 | 0,824 | 0,966 | 0,950 | 0,885 | 1 |
| IFIT1 IFI44L SIGLEC1 ISG15 HERC6 OLAH | 0,868 | 0,859 | 0,878 | 0,900 | 0,839 | 0,962 | 0,942 | 0,892 | 0,993 |
| IFIT1 IFI44L SIGLEC1 ISG15 SLC1A2 IL1R2 | 0,868 | 0,860 | 0,877 | 0,907 | 0,852 | 0,963 | 0,965 | 0,929 | 1 |
| RSAD2 IFI44L SIGLEC1 HERC6 FAM20A MMP8 | 0,868 | 0,858 | 0,878 | 0,899 | 0,833 | 0,964 | 0,942 | 0,873 | 1 |
| OAS1 IFI44L SLC1A2 IL1R2 FAM20A MMP8 | 0,868 | 0,859 | 0,878 | 0,913 | 0,862 | 0,965 | 0,927 | 0,834 | 1 |
| IFI44L SIGLEC1 ISG15 HERC6 FAM20A MMP8 | 0,868 | 0,858 | 0,878 | 0,898 | 0,831 | 0,966 | 0,953 | 0,882 | 1 |
| OAS1 IFI44L SIGLEC1 ISG15 HERC6 FAM20A | 0,868 | 0,858 | 0,878 | 0,899 | 0,834 | 0,965 | 0,953 | 0,891 | 1 |
| IFIT1 HERC6 SLC1A2 OLAH FAM20A RETN | 0,868 | 0,858 | 0,878 | 0,911 | 0,854 | 0,967 | 0,930 | 0,850 | 1 |
| IFIT1 IFI44L ISG15 IL1R2 FAM20A MMP8 | 0,868 | 0,859 | 0,878 | 0,904 | 0,841 | 0,966 | 0,942 | 0,867 | 1 |
| RSAD2 IFI27 OAS1 IFIT1 IFI44L RETN | 0,868 | 0,858 | 0,878 | 0,906 | 0,842 | 0,969 | 0,949 | 0,884 | 1 |
| RSAD2 OAS1 IFIT1 SIGLEC1 IL1R2 OLAH | 0,868 | 0,859 | 0,877 | 0,899 | 0,836 | 0,963 | 0,959 | 0,917 | 1 |
| RSAD2 OAS1 IFIT1 SIGLEC1 SLC1A2 IL1R2 | 0,868 | 0,859 | 0,877 | 0,907 | 0,852 | 0,963 | 0,968 | 0,932 | 1 |
| RSAD2 OAS1 IFI44L ISG15 IL1R2 FAM20A | 0,868 | 0,858 | 0,878 | 0,903 | 0,840 | 0,966 | 0,939 | 0,864 | 1 |
| RSAD2 OAS1 IFI44L SIGLEC1 SLC1A2 FAM20A | 0,868 | 0,858 | 0,878 | 0,904 | 0,842 | 0,966 | 0,945 | 0,873 | 1 |
| IFIT1 IFI44L SIGLEC1 IL1R2 RETN MMP8 | 0,868 | 0,858 | 0,878 | 0,900 | 0,837 | 0,964 | 0,962 | 0,923 | 1 |
| RSAD2 SIGLEC1 ISG15 SLC1A2 FAM20A RETN | 0,868 | 0,858 | 0,878 | 0,903 | 0,841 | 0,966 | 0,947 | 0,874 | 1 |
| RSAD2 IFIT1 SLC1A2 IL1R2 OLAH FAM20A | 0,868 | 0,859 | 0,877 | 0,908 | 0,851 | 0,966 | 0,933 | 0,855 | 1 |
| RSAD2 IFI27 IFIT1 IFI44L FAM20A MMP8 | 0,868 | 0,858 | 0,879 | 0,899 | 0,833 | 0,966 | 0,934 | 0,856 | 1 |
| RSAD2 OAS1 SIGLEC1 SLC1A2 IL1R2 RETN | 0,868 | 0,859 | 0,877 | 0,909 | 0,854 | 0,965 | 0,968 | 0,931 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 HERC6 OLAH FAM20A RETN | 0,868 | 0,858 | 0,878 | 0,901 | 0,836 | 0,966 | 0,945 | 0,878 | 1 |
| OAS1 SIGLEC1 ISG15 SLC1A2 FAM20A RETN | 0,868 | 0,858 | 0,878 | 0,901 | 0,838 | 0,965 | 0,947 | 0,874 | 1 |
| RSAD2 OAS1 SIGLEC1 ISG15 HERC6 RETN | 0,868 | 0,858 | 0,878 | 0,897 | 0,831 | 0,964 | 0,949 | 0,901 | 0,997 |
| RSAD2 IFI27 IFIT1 IFI44L ISG15 FAM20A | 0,868 | 0,858 | 0,878 | 0,903 | 0,835 | 0,970 | 0,943 | 0,870 | 1 |
| IFIT1 IFI44L SIGLEC1 ISG15 IL1R2 MMP8 | 0,868 | 0,859 | 0,877 | 0,901 | 0,842 | 0,961 | 0,960 | 0,919 | 1 |
| SIGLEC1 ISG15 HERC6 SLC1A2 IL1R2 RETN | 0,868 | 0,859 | 0,877 | 0,909 | 0,853 | 0,965 | 0,960 | 0,919 | 1 |
| RSAD2 IFI44L SIGLEC1 SLC1A2 OLAH RETN | 0,868 | 0,859 | 0,878 | 0,913 | 0,857 | 0,969 | 0,950 | 0,901 | 0,999 |
| RSAD2 OAS1 SIGLEC1 SLC1A2 FAM20A RETN | 0,868 | 0,858 | 0,878 | 0,903 | 0,840 | 0,966 | 0,947 | 0,874 | 1 |
| OAS1 IFIT1 IL1R2 OLAH FAM20A RETN | 0,868 | 0,858 | 0,878 | 0,900 | 0,834 | 0,966 | 0,941 | 0,868 | 1 |
| RSAD2 IFIT1 SIGLEC1 ISG15 HERC6 RETN | 0,868 | 0,859 | 0,877 | 0,898 | 0,833 | 0,963 | 0,929 | 0,872 | 0,987 |
| RSAD2 IFIT1 IFI44L SIGLEC1 IL1R2 MMP8 | 0,868 | 0,859 | 0,877 | 0,900 | 0,839 | 0,962 | 0,959 | 0,917 | 1 |
| RSAD2 IFI44L SIGLEC1 ISG15 SLC1A2 OLAH | 0,868 | 0,859 | 0,877 | 0,911 | 0,855 | 0,967 | 0,949 | 0,901 | 0,997 |
| RSAD2 SIGLEC1 SLC1A2 IL1R2 RETN MMP8 | 0,868 | 0,859 | 0,877 | 0,913 | 0,859 | 0,966 | 0,973 | 0,943 | 1 |
| IFI27 OAS1 SIGLEC1 ISG15 SLC1A2 MMP8 | 0,868 | 0,859 | 0,877 | 0,905 | 0,847 | 0,963 | 0,932 | 0,864 | 0,999 |
| RSAD2 IFI44L SIGLEC1 ISG15 SLC1A2 FAM20A | 0,868 | 0,858 | 0,878 | 0,903 | 0,840 | 0,965 | 0,942 | 0,870 | 1 |
| OAS1 HERC6 SLC1A2 OLAH FAM20A RETN | 0,868 | 0,858 | 0,878 | 0,911 | 0,854 | 0,967 | 0,928 | 0,847 | 1 |
| IFIT1 SLC1A2 IL1R2 FAM20A RETN MMP8 | 0,868 | 0,859 | 0,877 | 0,911 | 0,855 | 0,966 | 0,938 | 0,861 | 1 |
| RSAD2 IFI44L SIGLEC1 HERC6 SLC1A2 IL1R2 | 0,868 | 0,859 | 0,877 | 0,908 | 0,853 | 0,964 | 0,960 | 0,919 | 1 |
| RSAD2 IFI27 OAS1 SIGLEC1 SLC1A2 MMP8 | 0,868 | 0,859 | 0,877 | 0,903 | 0,844 | 0,963 | 0,939 | 0,875 | 1 |
| RSAD2 OAS1 IFI44L SIGLEC1 IL1R2 OLAH | 0,868 | 0,859 | 0,877 | 0,898 | 0,833 | 0,963 | 0,961 | 0,921 | 1 |
| IFIT1 ISG15 HERC6 OLAH FAM20A MMP8 | 0,868 | 0,858 | 0,878 | 0,900 | 0,837 | 0,963 | 0,942 | 0,873 | 1 |
| OAS1 IFIT1 ISG15 SLC1A2 IL1R2 FAM20A | 0,868 | 0,859 | 0,877 | 0,908 | 0,851 | 0,965 | 0,936 | 0,855 | 1 |

(suite)

| Modèle | AUC modèle | IC 95% | | AUC Train | IC 95% Train | | AUC Test | IC 95% Test | |
|---|---|---|---|---|---|---|---|---|---|
| SIGLEC1 ISG15 IL1R2 OLAH RETN MMP8 | 0,868 | 0,858 | 0,877 | 0,902 | 0,839 | 0,965 | 0,970 | 0,937 | 1 |
| RSAD2 IFI44L SIGLEC1 IL1R2 OLAH MMP8 | 0,868 | 0,859 | 0,877 | 0,901 | 0,841 | 0,962 | 0,960 | 0,919 | 1 |
| OAS1 IFI44L SIGLEC1 IL1R2 RETN MMP8 | 0,868 | 0,858 | 0,877 | 0,902 | 0,839 | 0,965 | 0,960 | 0,920 | 1,000 |
| OAS1 SIGLEC1 SLC1A2 IL1R2 RETN MMP8 | 0,868 | 0,859 | 0,877 | 0,912 | 0,859 | 0,966 | 0,971 | 0,939 | 1 |
| RSAD2 OAS1 IFI44L HERC6 OLAH FAM20A | 0,868 | 0,858 | 0,878 | 0,901 | 0,838 | 0,965 | 0,938 | 0,871 | 1 |
| IFIT1 IFI44L SIGLEC1 SLC1A2 IL1R2 MMP8 | 0,868 | 0,859 | 0,876 | 0,908 | 0,854 | 0,962 | 0,964 | 0,926 | 1 |
| RSAD2 IFIT1 HERC6 SLC1A2 IL1R2 FAM20A | 0,868 | 0,858 | 0,877 | 0,913 | 0,859 | 0,966 | 0,930 | 0,845 | 1 |
| OAS1 SIGLEC1 ISG15 SLC1A2 IL1R2 RETN | 0,868 | 0,859 | 0,877 | 0,910 | 0,854 | 0,965 | 0,965 | 0,927 | 1 |
| IFI27 IFIT1 IFI44L SLC1A2 FAM20A MMP8 | 0,868 | 0,858 | 0,878 | 0,901 | 0,836 | 0,965 | 0,932 | 0,850 | 1 |
| IFI27 IFI44L ISG15 SLC1A2 FAM20A MMP8 | 0,868 | 0,857 | 0,878 | 0,900 | 0,836 | 0,965 | 0,937 | 0,857 | 1 |
| IFIT1 IFI44L SIGLEC1 SLC1A2 IL1R2 RETN | 0,868 | 0,859 | 0,877 | 0,910 | 0,855 | 0,965 | 0,962 | 0,922 | 1 |
| RSAD2 IFI44L SIGLEC1 FAM20A RETN MMP8 | 0,868 | 0,858 | 0,878 | 0,901 | 0,833 | 0,969 | 0,954 | 0,895 | 1 |
| RSAD2 OAS1 IFI44L SIGLEC1 ISG15 FAM20A | 0,868 | 0,858 | 0,878 | 0,898 | 0,827 | 0,968 | 0,952 | 0,891 | 1 |
| RSAD2 OAS1 SIGLEC1 IL1R2 RETN MMP8 | 0,868 | 0,858 | 0,878 | 0,899 | 0,835 | 0,964 | 0,971 | 0,938 | 1 |
| IFIT1 SIGLEC1 IL1R2 OLAH RETN MMP8 | 0,868 | 0,858 | 0,877 | 0,903 | 0,841 | 0,964 | 0,965 | 0,929 | 1 |
| RSAD2 OAS1 IFIT1 IFI44L SIGLEC1 IL1R2 | 0,868 | 0,858 | 0,877 | 0,897 | 0,831 | 0,962 | 0,957 | 0,915 | 0,998 |
| RSAD2 IFI27 OAS1 ISG15 HERC6 MMP8 | 0,868 | 0,859 | 0,877 | 0,901 | 0,843 | 0,958 | 0,903 | 0,827 | 0,980 |
| OAS1 SIGLEC1 HERC6 OLAH RETN MMP8 | 0,868 | 0,857 | 0,878 | 0,896 | 0,828 | 0,963 | 0,964 | 0,926 | 1 |
| RSAD2 IFIT1 IFI44L SIGLEC1 IL1R2 OLAH | 0,868 | 0,859 | 0,877 | 0,899 | 0,834 | 0,964 | 0,958 | 0,916 | 0,999 |
| RSAD2 SIGLEC1 ISG15 SLC1A2 IL1R2 MMP8 | 0,868 | 0,859 | 0,876 | 0,909 | 0,854 | 0,964 | 0,966 | 0,931 | 1 |
| RSAD2 ISG15 IL1R2 OLAH FAM20A MMP8 | 0,868 | 0,858 | 0,877 | 0,902 | 0,839 | 0,964 | 0,941 | 0,868 | 1 |
| RSAD2 ISG15 HERC6 OLAH FAM20A RETN | 0,868 | 0,858 | 0,878 | 0,905 | 0,844 | 0,967 | 0,942 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 HERC6 OLAH FAM20A MMP8 | 0,868 | 0,858 | 0,878 | 0,899 | 0,834 | 0,963 | 0,941 | 0,873 | 1 |
| IFI27 OAS1 IFI44L HERC6 SLC1A2 FAM20A | 0,868 | 0,857 | 0,878 | 0,910 | 0,852 | 0,967 | 0,933 | 0,851 | 1 |
| RSAD2 OAS1 HERC6 OLAH FAM20A MMP8 | 0,868 | 0,858 | 0,877 | 0,899 | 0,835 | 0,963 | 0,941 | 0,874 | 1 |
| OAS1 ISG15 IL1R2 OLAH FAM20A RETN | 0,868 | 0,858 | 0,877 | 0,900 | 0,834 | 0,965 | 0,930 | 0,852 | 1 |
| IFI44L ISG15 HERC6 OLAH FAM20A MMP8 | 0,868 | 0,858 | 0,878 | 0,899 | 0,833 | 0,964 | 0,948 | 0,882 | 1 |
| RSAD2 IFIT1 HERC6 SLC1A2 OLAH FAM20A | 0,868 | 0,857 | 0,878 | 0,910 | 0,854 | 0,966 | 0,930 | 0,849 | 1 |
| OAS1 SIGLEC1 ISG15 SLC1A2 FAM20A MMP8 | 0,868 | 0,857 | 0,878 | 0,902 | 0,839 | 0,965 | 0,949 | 0,878 | 1 |
| OAS1 IFIT1 SIGLEC1 IL1R2 OLAH RETN | 0,868 | 0,858 | 0,877 | 0,900 | 0,834 | 0,965 | 0,955 | 0,912 | 0,999 |
| IFI27 OAS1 ISG15 HERC6 SLC1A2 FAM20A | 0,868 | 0,857 | 0,878 | 0,905 | 0,846 | 0,964 | 0,929 | 0,845 | 1 |
| RSAD2 IFIT1 SIGLEC1 SLC1A2 IL1R2 RETN | 0,868 | 0,858 | 0,877 | 0,908 | 0,852 | 0,964 | 0,965 | 0,928 | 1 |
| IFIT1 SIGLEC1 ISG15 IL1R2 OLAH MMP8 | 0,868 | 0,859 | 0,876 | 0,905 | 0,848 | 0,962 | 0,963 | 0,925 | 1 |
| RSAD2 OAS1 SIGLEC1 SLC1A2 IL1R2 MMP8 | 0,867 | 0,858 | 0,877 | 0,910 | 0,856 | 0,963 | 0,968 | 0,934 | 1 |
| OAS1 IFI44L SIGLEC1 SLC1A2 IL1R2 MMP8 | 0,867 | 0,859 | 0,876 | 0,908 | 0,854 | 0,963 | 0,965 | 0,928 | 1 |
| IFI27 OAS1 IFI44L ISG15 HERC6 MMP8 | 0,867 | 0,859 | 0,876 | 0,901 | 0,845 | 0,958 | 0,900 | 0,822 | 0,978 |
| IFI27 OAS1 IFIT1 ISG15 SLC1A2 RETN | 0,867 | 0,858 | 0,877 | 0,899 | 0,834 | 0,964 | 0,941 | 0,869 | 1 |
| RSAD2 IFIT1 SIGLEC1 IL1R2 OLAH MMP8 | 0,867 | 0,858 | 0,877 | 0,905 | 0,846 | 0,963 | 0,964 | 0,927 | 1 |
| IFI27 OAS1 IFIT1 IFI44L FAM20A MMP8 | 0,867 | 0,857 | 0,878 | 0,897 | 0,830 | 0,965 | 0,934 | 0,854 | 1 |
| RSAD2 OAS1 IFI44L SIGLEC1 FAM20A MMP8 | 0,867 | 0,857 | 0,878 | 0,896 | 0,826 | 0,967 | 0,951 | 0,888 | 1 |
| RSAD2 IFI44L ISG15 SLC1A2 IL1R2 FAM20A | 0,867 | 0,858 | 0,877 | 0,907 | 0,851 | 0,963 | 0,930 | 0,844 | 1 |
| IFIT1 IFI44L ISG15 IL1R2 OLAH FAM20A | 0,867 | 0,858 | 0,877 | 0,900 | 0,835 | 0,965 | 0,952 | 0,895 | 1 |
| RSAD2 OAS1 HERC6 OLAH FAM20A RETN | 0,867 | 0,858 | 0,877 | 0,902 | 0,837 | 0,966 | 0,939 | 0,871 | 1 |
| RSAD2 OAS1 IFI44L SIGLEC1 IL1R2 MMP8 | 0,867 | 0,858 | 0,876 | 0,901 | 0,840 | 0,962 | 0,961 | 0,921 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L SIGLEC1 HERC6 OLAH RETN MMP8 | 0,867 | 0,857 | 0,878 | 0,896 | 0,827 | 0,965 | 0,962 | 0,924 | 1 |
| IFIT1 ISG15 SLC1A2 IL1R2 FAM20A RETN | 0,867 | 0,858 | 0,877 | 0,905 | 0,848 | 0,962 | 0,932 | 0,851 | 1 |
| RSAD2 OAS1 SIGLEC1 ISG15 SLC1A2 IL1R2 | 0,867 | 0,858 | 0,877 | 0,908 | 0,852 | 0,963 | 0,966 | 0,928 | 1 |
| OAS1 SIGLEC1 IL1R2 OLAH RETN MMP8 | 0,867 | 0,857 | 0,877 | 0,900 | 0,836 | 0,964 | 0,970 | 0,936 | 1 |
| OAS 1 HERC6 OLAH FAM20A RETN MMP8 | 0,867 | 0,857 | 0,877 | 0,900 | 0,835 | 0,965 | 0,950 | 0,886 | 1 |
| RSAD2 SIGLEC1 ISG15 SLC1A2 IL1R2 RETN | 0,867 | 0,858 | 0,876 | 0,910 | 0,855 | 0,965 | 0,964 | 0,926 | 1 |
| OAS1 SIGLEC1 ISG15 SLC1A2 IL1R2 MMP8 | 0,867 | 0,859 | 0,876 | 0,910 | 0,856 | 0,964 | 0,966 | 0,930 | 1 |
| OAS1 IFIT1 ISG15 HERC6 OLAH FAM20A | 0,867 | 0,857 | 0,877 | 0,900 | 0,837 | 0,963 | 0,941 | 0,872 | 1 |
| RSAD2 IFI44L SLC1A2 OLAH FAM20A RETN | 0,867 | 0,857 | 0,877 | 0,909 | 0,850 | 0,968 | 0,926 | 0,852 | 1 |
| RSAD2 OAS1 IFI44L SIGLEC1 FAM20A RETN | 0,867 | 0,857 | 0,877 | 0,896 | 0,825 | 0,966 | 0,953 | 0,894 | 1 |
| OAS1 IFIT1 ISG15 HERC6 FAM20A RETN | 0,867 | 0,857 | 0,877 | 0,898 | 0,834 | 0,962 | 0,937 | 0,860 | 1 |
| OAS1 IFIT1 IFI44L SLC1A2 IL1R2 FAM20A | 0,867 | 0,858 | 0,876 | 0,906 | 0,849 | 0,963 | 0,925 | 0,837 | 1 |
| OAS1 IFIT1 ISG15 IL1R2 FAM20A RETN | 0,867 | 0,857 | 0,877 | 0,900 | 0,835 | 0,965 | 0,945 | 0,871 | 1 |
| OAS1 IFIT1 IFI44L SIGLEC1 IL1R2 RETN | 0,867 | 0,858 | 0,877 | 0,898 | 0,832 | 0,964 | 0,957 | 0,915 | 0,998 |
| RSAD2 OAS1 ISG15 HERC6 OLAH FAM20A | 0,867 | 0,857 | 0,877 | 0,900 | 0,837 | 0,963 | 0,938 | 0,868 | 1 |
| RSAD2 IFIT1 SIGLEC1 ISG15 SLC1A2 IL1R2 | 0,867 | 0,859 | 0,876 | 0,905 | 0,850 | 0,961 | 0,964 | 0,927 | 1 |
| OAS1 IFIT1 IFI44L ISG15 IL1R2 FAM20A | 0,867 | 0,858 | 0,877 | 0,900 | 0,835 | 0,965 | 0,945 | 0,871 | 1 |
| IFI27 OAS1 IFIT1 ISG15 RETN MMP8 | 0,867 | 0,857 | 0,877 | 0,903 | 0,839 | 0,967 | 0,950 | 0,887 | 1 |
| RSAD2 IFI44L SIGLEC1 OLAH FAM20A RETN | 0,867 | 0,857 | 0,877 | 0,898 | 0,827 | 0,969 | 0,961 | 0,917 | 1 |
| RSAD2 IFIT1 SLC1A2 OLAH FAM20A MMP8 | 0,867 | 0,857 | 0,877 | 0,905 | 0,846 | 0,965 | 0,939 | 0,869 | 1 |
| OAS1 IFIT1 SIGLEC1 ISG15 HERC6 OLAH | 0,867 | 0,858 | 0,877 | 0,900 | 0,839 | 0,962 | 0,941 | 0,890 | 0,993 |
| RSAD2 IFI27 ISG15 HERC6 SLC1A2 FAM20A | 0,867 | 0,857 | 0,877 | 0,908 | 0,850 | 0,965 | 0,927 | 0,843 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 IFI44L SIGLEC1 HERC6 RETN | 0,867 | 0,858 | 0,876 | 0,895 | 0,826 | 0,964 | 0,922 | 0,860 | 0,983 |
| RSAD2 IFI44L ISG15 HERC6 OLAH FAM20A | 0,867 | 0,857 | 0,877 | 0,903 | 0,841 | 0,965 | 0,941 | 0,876 | 1 |
| OAS1 IFI44L SIGLEC1 ISG15 FAM20A RETN | 0,867 | 0,857 | 0,877 | 0,895 | 0,823 | 0,966 | 0,955 | 0,898 | 1 |
| IFI27 IFI44L SIGLEC1 ISG15 SLC1A2 MMP8 | 0,867 | 0,858 | 0,876 | 0,901 | 0,841 | 0,960 | 0,925 | 0,853 | 0,997 |
| IFIT1 SIGLEC1 ISG15 IL1R2 RETN MMP8 | 0,867 | 0,857 | 0,877 | 0,902 | 0,839 | 0,964 | 0,968 | 0,935 | 1 |
| OAS1 IFIT1 IFI44L SIGLEC1 SLC1A2 IL1R2 | 0,867 | 0,858 | 0,876 | 0,907 | 0,851 | 0,963 | 0,963 | 0,925 | 1 |
| RSAD2 OAS1 IL1R2 FAM20A RETN MMP8 | 0,867 | 0,857 | 0,877 | 0,904 | 0,841 | 0,967 | 0,938 | 0,859 | 1 |
| RSAD2 IFI44L ISG15 HERC6 FAM20A RETN | 0,867 | 0,857 | 0,877 | 0,905 | 0,844 | 0,966 | 0,935 | 0,859 | 1 |
| RSAD2 IFI44L SIGLEC1 SLC1A2 IL1R2 MMP8 | 0,867 | 0,858 | 0,876 | 0,907 | 0,852 | 0,962 | 0,968 | 0,934 | 1 |
| IFIT1 SLC1A2 IL1R2 OLAH FAM20A RETN | 0,867 | 0,857 | 0,877 | 0,907 | 0,850 | 0,964 | 0,929 | 0,852 | 1 |
| RSAD2 OAS1 IFIT1 HERC6 OLAH FAM20A | 0,867 | 0,857 | 0,877 | 0,901 | 0,837 | 0,965 | 0,936 | 0,865 | 1 |
| OAS1 IFI44L SLC1A2 OLAH FAM20A RETN | 0,867 | 0,857 | 0,877 | 0,908 | 0,849 | 0,968 | 0,927 | 0,852 | 1 |
| RSAD2 IFIT1 IFI44L SIGLEC1 ISG15 IL1R2 | 0,867 | 0,858 | 0,876 | 0,896 | 0,831 | 0,961 | 0,955 | 0,913 | 0,998 |
| RSAD2 IFI44L SIGLEC1 SLC1A2 IL1R2 RETN | 0,867 | 0,858 | 0,876 | 0,907 | 0,850 | 0,963 | 0,970 | 0,936 | 1 |
| RSAD2 IFI44L SIGLEC1 ISG15 IL1R2 MMP8 | 0,867 | 0,858 | 0,876 | 0,900 | 0,838 | 0,962 | 0,960 | 0,919 | 1 |
| ISG15 SLC1A2 IL1R2 OLAH FAM20A MMP8 | 0,867 | 0,858 | 0,876 | 0,910 | 0,856 | 0,964 | 0,923 | 0,840 | 1 |
| RSAD2 IFI44L IL1R2 OLAH FAM20A MMP8 | 0,867 | 0,857 | 0,876 | 0,904 | 0,842 | 0,965 | 0,948 | 0,884 | 1 |
| IFIT1 IFI44L SLC1A2 IL1R2 FAM20A RETN | 0,867 | 0,858 | 0,876 | 0,909 | 0,854 | 0,963 | 0,925 | 0,838 | 1 |
| IFI27 IFIT1 IFI44L ISG15 RETN MMP8 | 0,867 | 0,856 | 0,877 | 0,903 | 0,839 | 0,967 | 0,953 | 0,897 | 1 |
| RSAD2 OAS1 IFI44L SLC1A2 IL1R2 FAM20A | 0,867 | 0,857 | 0,876 | 0,907 | 0,851 | 0,964 | 0,925 | 0,837 | 1 |
| OAS1 IFI44L SIGLEC1 ISG15 IL1R2 OLAH | 0,867 | 0,858 | 0,876 | 0,901 | 0,837 | 0,965 | 0,951 | 0,905 | 0,997 |
| OAS1 IFI44L ISG15 SLC1A2 IL1R2 FAM20A | 0,867 | 0,857 | 0,876 | 0,907 | 0,851 | 0,964 | 0,925 | 0,837 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 IFIT1 SIGLEC1 HERC6 OLAH | 0,867 | 0,857 | 0,876 | 0,901 | 0,840 | 0,962 | 0,937 | 0,883 | 0,991 |
| IFIT1 IFI44L SIGLEC1 HERC6 RETN MMP8 | 0,867 | 0,857 | 0,876 | 0,894 | 0,824 | 0,964 | 0,925 | 0,865 | 0,985 |
| OAS1 SIGLEC1 ISG15 HERC6 FAM20A MMP8 | 0,867 | 0,857 | 0,877 | 0,898 | 0,832 | 0,964 | 0,949 | 0,885 | 1 |
| IFI27 OAS1 IFI44L ISG15 RETN MMP8 | 0,867 | 0,857 | 0,877 | 0,905 | 0,842 | 0,968 | 0,953 | 0,895 | 1 |
| IFI44L SIGLEC1 ISG15 IL1R2 OLAH RETN | 0,867 | 0,858 | 0,876 | 0,900 | 0,837 | 0,964 | 0,960 | 0,917 | 1 |
| OAS1 IFI44L SIGLEC1 ISG15 IL1R2 MMP8 | 0,867 | 0,858 | 0,875 | 0,901 | 0,841 | 0,961 | 0,955 | 0,912 | 0,999 |
| OAS1 IFI44L SIGLEC1 SLC1A2 IL1R2 RETN | 0,867 | 0,857 | 0,876 | 0,910 | 0,855 | 0,965 | 0,962 | 0,922 | 1 |
| RSAD2 OAS1 IFIT1 SIGLEC1 IL1R2 MMP8 | 0,867 | 0,857 | 0,876 | 0,901 | 0,842 | 0,961 | 0,966 | 0,931 | 1 |
| IFI44L ISG15 IL1R2 OLAH FAM20A RETN | 0,867 | 0,857 | 0,876 | 0,901 | 0,838 | 0,965 | 0,943 | 0,879 | 1 |
| RSAD2 IFIT1 ISG15 IL1R2 FAM20A MMP8 | 0,867 | 0,857 | 0,876 | 0,905 | 0,843 | 0,967 | 0,943 | 0,870 | 1 |
| RSAD2 IFIT1 HERC6 OLAH FAM20A RETN | 0,867 | 0,857 | 0,877 | 0,902 | 0,839 | 0,966 | 0,943 | 0,877 | 1 |
| RSAD2 HERC6 OLAH FAM20A RETN MMP8 | 0,867 | 0,857 | 0,877 | 0,900 | 0,836 | 0,965 | 0,947 | 0,881 | 1 |
| RSAD2 OAS1 SIGLEC1 ISG15 IL1R2 OLAH | 0,867 | 0,857 | 0,876 | 0,899 | 0,836 | 0,962 | 0,959 | 0,917 | 1 |
| OAS1 IFI44L ISG15 IL1R2 FAM20A MMP8 | 0,867 | 0,857 | 0,876 | 0,904 | 0,842 | 0,966 | 0,938 | 0,858 | 1 |
| RSAD2 IFIT1 ISG15 SLC1A2 IL1R2 FAM20A | 0,867 | 0,858 | 0,875 | 0,904 | 0,845 | 0,963 | 0,934 | 0,852 | 1 |
| IFIT1 IFI44L SIGLEC1 ISG15 IL1R2 OLAH | 0,867 | 0,858 | 0,876 | 0,902 | 0,841 | 0,963 | 0,958 | 0,915 | 1 |
| RSAD2 OAS1 ISG15 HERC6 FAM20A RETN | 0,867 | 0,857 | 0,876 | 0,901 | 0,838 | 0,964 | 0,938 | 0,862 | 1 |
| OAS1 IFI44L SIGLEC1 SLC1A2 FAM20A MMP8 | 0,867 | 0,857 | 0,876 | 0,903 | 0,841 | 0,966 | 0,948 | 0,877 | 1 |
| OAS1 IFI44L SIGLEC1 ISG15 SLC1A2 IL1R2 | 0,867 | 0,858 | 0,876 | 0,907 | 0,852 | 0,963 | 0,964 | 0,927 | 1 |
| RSAD2 OAS1 IFI44L SIGLEC1 ISG15 IL1R2 | 0,867 | 0,857 | 0,876 | 0,895 | 0,829 | 0,961 | 0,960 | 0,920 | 1,000 |
| OAS1 IFI44L SIGLEC1 HERC6 FAM20A MMP8 | 0,867 | 0,857 | 0,876 | 0,899 | 0,833 | 0,965 | 0,953 | 0,891 | 1 |
| OAS1 SIGLEC1 ISG15 IL1R2 OLAH MMP8 | 0,867 | 0,857 | 0,876 | 0,904 | 0,845 | 0,963 | 0,963 | 0,926 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 SLC1A2 IL1R2 FAM20A RETN | 0,866 | 0,857 | 0,876 | 0,908 | 0,851 | 0,964 | 0,936 | 0,855 | 1 |
| RSAD2 IFI27 OAS1 IFI44L HERC6 MMP8 | 0,866 | 0,857 | 0,876 | 0,897 | 0,837 | 0,957 | 0,898 | 0,818 | 0,978 |
| RSAD2 IFI27 IFIT1 IFI44L ISG15 RETN | 0,866 | 0,857 | 0,876 | 0,903 | 0,839 | 0,967 | 0,953 | 0,895 | 1 |
| OAS1 ISG15 SLC1A2 OLAH FAM20A MMP8 | 0,866 | 0,857 | 0,876 | 0,910 | 0,853 | 0,967 | 0,936 | 0,861 | 1 |
| OAS1 IFIT1 SIGLEC1 ISG15 IL1R2 MMP8 | 0,866 | 0,858 | 0,875 | 0,901 | 0,842 | 0,961 | 0,963 | 0,925 | 1 |
| RSAD2 OAS1 IFIT1 SLC1A2 IL1R2 FAM20A | 0,866 | 0,858 | 0,875 | 0,906 | 0,848 | 0,964 | 0,936 | 0,855 | 1 |
| IFI27 OAS1 IFI44L ISG15 FAM20A MMP8 | 0,866 | 0,855 | 0,877 | 0,898 | 0,831 | 0,965 | 0,938 | 0,860 | 1 |
| RSAD2 IFIT1 IFI44L SIGLEC1 SLC1A2 IL1R2 | 0,866 | 0,858 | 0,875 | 0,907 | 0,850 | 0,963 | 0,965 | 0,929 | 1 |
| RSAD2 IFIT1 SIGLEC1 HERC6 OLAH MMP8 | 0,866 | 0,857 | 0,876 | 0,899 | 0,837 | 0,961 | 0,936 | 0,881 | 0,991 |
| OAS1 IFI44L HERC6 OLAH FAM20A RETN | 0,866 | 0,856 | 0,876 | 0,904 | 0,840 | 0,968 | 0,943 | 0,879 | 1 |
| RSAD2 IFI27 OAS1 HERC6 SLC1A2 MMP8 | 0,866 | 0,857 | 0,876 | 0,907 | 0,852 | 0,962 | 0,900 | 0,817 | 0,983 |
| IFI27 OAS1 IFIT1 IFI44L RETN MMP8 | 0,866 | 0,856 | 0,877 | 0,904 | 0,841 | 0,968 | 0,949 | 0,885 | 1 |
| RSAD2 SIGLEC1 ISG15 IL1R2 OLAH MMP8 | 0,866 | 0,857 | 0,876 | 0,903 | 0,844 | 0,962 | 0,966 | 0,931 | 1 |
| RSAD2 IFI44L SIGLEC1 ISG15 SLC1A2 IL1R2 | 0,866 | 0,858 | 0,875 | 0,906 | 0,849 | 0,963 | 0,967 | 0,933 | 1 |
| IFI27 IFI44L ISG15 HERC6 SLC1A2 FAM20A | 0,866 | 0,856 | 0,877 | 0,902 | 0,841 | 0,963 | 0,938 | 0,856 | 1 |
| RSAD2 IFI44L SIGLEC1 IL1R2 RETN MMP8 | 0,866 | 0,857 | 0,876 | 0,900 | 0,836 | 0,964 | 0,967 | 0,933 | 1 |
| RSAD2 OAS1 IFIT1 IL1R2 FAM20A RETN | 0,866 | 0,857 | 0,876 | 0,901 | 0,837 | 0,966 | 0,943 | 0,869 | 1 |
| RSAD2 OAS1 ISG15 SLC1A2 OLAH FAM20A | 0,866 | 0,856 | 0,876 | 0,910 | 0,852 | 0,968 | 0,935 | 0,862 | 1 |
| SIGLEC1 ISG15 HERC6 IL1R2 OLAH RETN | 0,866 | 0,856 | 0,876 | 0,900 | 0,835 | 0,966 | 0,959 | 0,918 | 1,000 |
| IFI27 OAS1 IFI44L SIGLEC1 SLC1A2 MMP8 | 0,866 | 0,857 | 0,875 | 0,903 | 0,845 | 0,962 | 0,932 | 0,864 | 0,999 |
| ISG15 SLC1A2 IL1R2 OLAH FAM20A RETN | 0,866 | 0,857 | 0,876 | 0,908 | 0,851 | 0,965 | 0,924 | 0,844 | 1 |
| RSAD2 SIGLEC1 IL1R2 OLAH RETN MMP8 | 0,866 | 0,856 | 0,876 | 0,900 | 0,837 | 0,964 | 0,970 | 0,937 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L IL1R2 OLAH FAM20A RETN MMP8 | 0,866 | 0,857 | 0,876 | 0,902 | 0,840 | 0,964 | 0,947 | 0,885 | 1 |
| IFIT1 SIGLEC1 ISG15 HERC6 RETN MMP8 | 0,866 | 0,856 | 0,876 | 0,896 | 0,829 | 0,963 | 0,934 | 0,878 | 0,989 |
| RSAD2 OAS1 IFIT1 SIGLEC1 IL1R2 RETN | 0,866 | 0,857 | 0,876 | 0,896 | 0,830 | 0,962 | 0,960 | 0,918 | 1 |
| OAS1 IFIT1 SIGLEC1 ISG15 IL1R2 RETN | 0,866 | 0,857 | 0,875 | 0,897 | 0,831 | 0,962 | 0,960 | 0,920 | 1,000 |
| RSAD2 IFIT1 SIGLEC1 IL1R2 RETN MMP8 | 0,866 | 0,857 | 0,876 | 0,902 | 0,839 | 0,965 | 0,968 | 0,935 | 1 |
| IFIT1 ISG15 SLC1A2 OLAH FAM20A MMP8 | 0,866 | 0,856 | 0,876 | 0,907 | 0,847 | 0,966 | 0,939 | 0,869 | 1 |
| IFIT1 IL1R2 OLAH FAM20A RETN MMP8 | 0,866 | 0,856 | 0,876 | 0,905 | 0,844 | 0,967 | 0,948 | 0,882 | 1 |
| RSAD2 IFI27 IFI44L SLC1A2 FAM20A MMP8 | 0,866 | 0,856 | 0,877 | 0,903 | 0,839 | 0,966 | 0,936 | 0,855 | 1 |
| RSAD2 OAS1 SLC1A2 IL1R2 FAM20A MMP8 | 0,866 | 0,857 | 0,875 | 0,910 | 0,856 | 0,963 | 0,927 | 0,835 | 1 |
| IFIT1 ISG15 IL1R2 FAM20A RETN MMP8 | 0,866 | 0,856 | 0,876 | 0,905 | 0,842 | 0,968 | 0,942 | 0,869 | 1 |
| IFIT1 IFI44L SIGLEC1 ISG15 IL1R2 RETN | 0,866 | 0,857 | 0,875 | 0,900 | 0,837 | 0,963 | 0,960 | 0,917 | 1 |
| RSAD2 SIGLEC1 HERC6 OLAH RETN MMP8 | 0,866 | 0,856 | 0,876 | 0,898 | 0,831 | 0,966 | 0,958 | 0,916 | 0,999 |
| RSAD2 IFI27 IFI44L ISG15 SLC1A2 FAM20A | 0,866 | 0,855 | 0,877 | 0,901 | 0,836 | 0,966 | 0,939 | 0,860 | 1 |
| RSAD2 OAS1 SIGLEC1 HERC6 SLC1A2 RETN | 0,866 | 0,856 | 0,876 | 0,904 | 0,842 | 0,965 | 0,951 | 0,901 | 1 |
| IFI27 IFIT1 IFI44L SIGLEC1 SLC1A2 MMP8 | 0,866 | 0,857 | 0,875 | 0,902 | 0,842 | 0,961 | 0,927 | 0,858 | 0,996 |
| OAS1 SIGLEC1 ISG15 HERC6 RETN MMP8 | 0,866 | 0,856 | 0,876 | 0,897 | 0,829 | 0,964 | 0,952 | 0,905 | 0,999 |
| RSAD2 OAS1 IFI44L SIGLEC1 SLC1A2 IL1R2 | 0,866 | 0,857 | 0,875 | 0,907 | 0,851 | 0,964 | 0,968 | 0,932 | 1 |
| RSAD2 IFI44L HERC6 OLAH FAM20A MMP8 | 0,866 | 0,856 | 0,876 | 0,901 | 0,839 | 0,964 | 0,945 | 0,882 | 1 |
| RSAD2 OAS1 SLC1A2 OLAH FAM20A RETN | 0,866 | 0,856 | 0,876 | 0,910 | 0,852 | 0,968 | 0,935 | 0,862 | 1 |
| RSAD2 IFIT1 IFI44L SIGLEC1 IL1R2 RETN | 0,866 | 0,857 | 0,875 | 0,897 | 0,831 | 0,964 | 0,961 | 0,922 | 1,000 |
| OAS1 IFI44L ISG15 HERC6 OLAH FAM20A | 0,866 | 0,856 | 0,876 | 0,900 | 0,836 | 0,963 | 0,947 | 0,885 | 1 |
| IFI44L ISG15 IL1R2 FAM20A RETN MMP8 | 0,866 | 0,857 | 0,875 | 0,903 | 0,842 | 0,964 | 0,937 | 0,859 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 IFI44L HERC6 MMP8 | 0,866 | 0,857 | 0,875 | 0,900 | 0,842 | 0,957 | 0,897 | 0,817 | 0,976 |
| OAS1 IFI44L SIGLEC1 ISG15 SLC1A2 FAM20A | 0,866 | 0,856 | 0,876 | 0,902 | 0,839 | 0,965 | 0,948 | 0,878 | 1 |
| RSAD2 OAS1 ISG15 IL1R2 FAM20A RETN | 0,866 | 0,856 | 0,876 | 0,898 | 0,833 | 0,964 | 0,935 | 0,856 | 1 |
| IFI27 IFI44L SIGLEC1 HERC6 SLC1A2 MMP8 | 0,866 | 0,857 | 0,875 | 0,902 | 0,845 | 0,960 | 0,924 | 0,854 | 0,993 |
| RSAD2 IFIT1 IFI44L SLC1A2 IL1R2 FAM20A | 0,866 | 0,857 | 0,875 | 0,907 | 0,851 | 0,963 | 0,925 | 0,837 | 1 |
| IFI27 OAS1 IFIT1 IFI44L SLC1A2 RETN | 0,866 | 0,856 | 0,876 | 0,903 | 0,839 | 0,966 | 0,945 | 0,875 | 1 |
| RSAD2 OAS1 ISG15 SLC1A2 IL1R2 FAM20A | 0,866 | 0,856 | 0,875 | 0,904 | 0,848 | 0,961 | 0,932 | 0,846 | 1 |
| OAS1 IFIT1 ISG15 SLC1A2 OLAH FAM20A | 0,866 | 0,856 | 0,876 | 0,908 | 0,849 | 0,967 | 0,936 | 0,862 | 1 |
| RSAD2 IFIT1 SIGLEC1 ISG15 IL1R2 OLAH | 0,866 | 0,857 | 0,875 | 0,899 | 0,836 | 0,963 | 0,959 | 0,917 | 1 |
| RSAD2 SIGLEC1 ISG15 IL1R2 RETN MMP8 | 0,866 | 0,856 | 0,876 | 0,900 | 0,837 | 0,964 | 0,971 | 0,938 | 1 |
| RSAD2 IFI44L SIGLEC1 ISG15 IL1R2 OLAH | 0,866 | 0,857 | 0,875 | 0,896 | 0,831 | 0,962 | 0,958 | 0,916 | 0,999 |
| RSAD2 OAS1 IFI44L SIGLEC1 IL1R2 RETN | 0,866 | 0,856 | 0,875 | 0,893 | 0,826 | 0,961 | 0,963 | 0,924 | 1 |
| OAS1 SIGLEC1 ISG15 HERC6 OLAH RETN | 0,866 | 0,856 | 0,876 | 0,898 | 0,832 | 0,964 | 0,959 | 0,916 | 1 |
| IFI27 OAS1 IFI44L ISG15 SLC1A2 RETN | 0,866 | 0,856 | 0,875 | 0,903 | 0,840 | 0,966 | 0,945 | 0,877 | 1 |
| IFI27 OAS1 ISG15 HERC6 SLC1A2 MMP8 | 0,866 | 0,857 | 0,875 | 0,906 | 0,852 | 0,960 | 0,901 | 0,822 | 0,980 |
| RSAD2 IFIT1 HERC6 OLAH FAM20A MMP8 | 0,866 | 0,856 | 0,876 | 0,899 | 0,835 | 0,963 | 0,942 | 0,875 | 1 |
| RSAD2 IFIT1 ISG15 HERC6 FAM20A RETN | 0,866 | 0,856 | 0,876 | 0,900 | 0,837 | 0,963 | 0,935 | 0,858 | 1 |
| RSAD2 OAS1 SIGLEC1 IL1R2 OLAH RETN | 0,866 | 0,856 | 0,876 | 0,898 | 0,832 | 0,963 | 0,961 | 0,919 | 1 |
| RSAD2 IFI44L SIGLEC1 ISG15 FAM20A RETN | 0,866 | 0,856 | 0,876 | 0,897 | 0,827 | 0,967 | 0,952 | 0,893 | 1 |
| OAS1 IFIT1 SIGLEC1 OLAH RETN MMP8 | 0,866 | 0,856 | 0,876 | 0,896 | 0,829 | 0,964 | 0,953 | 0,909 | 0,998 |
| OAS1 ISG15 IL1R2 FAM20A RETN MMP8 | 0,866 | 0,856 | 0,875 | 0,905 | 0,843 | 0,967 | 0,939 | 0,860 | 1 |
| RSAD2 OAS1 IFIT1 SIGLEC1 ISG15 IL1R2 | 0,866 | 0,857 | 0,875 | 0,898 | 0,835 | 0,961 | 0,962 | 0,923 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 IFIT1 SLC1A2 OLAH FAM20A | 0,866 | 0,856 | 0,875 | 0,909 | 0,849 | 0,968 | 0,934 | 0,859 | 1 |
| RSAD2 IFI27 OAS1 IFI44L SLC1A2 RETN | 0,866 | 0,856 | 0,875 | 0,905 | 0,843 | 0,968 | 0,940 | 0,868 | 1 |
| OAS1 IFI44L IL1R2 OLAH FAM20A RETN | 0,866 | 0,856 | 0,875 | 0,900 | 0,835 | 0,966 | 0,943 | 0,874 | 1 |
| OAS1 IFI44L SIGLEC1 SLC1A2 FAM20A RETN | 0,866 | 0,856 | 0,875 | 0,901 | 0,838 | 0,965 | 0,947 | 0,875 | 1 |
| IFI27 IFI44L ISG15 SLC1A2 RETN MMP8 | 0,865 | 0,855 | 0,876 | 0,904 | 0,841 | 0,966 | 0,939 | 0,870 | 1 |
| OAS1 SIGLEC1 HERC6 SLC1A2 RETN MMP8 | 0,865 | 0,856 | 0,875 | 0,903 | 0,840 | 0,965 | 0,950 | 0,900 | 1,000 |
| RSAD2 IFI44L SIGLEC1 ISG15 FAM20A MMP8 | 0,865 | 0,855 | 0,876 | 0,894 | 0,823 | 0,964 | 0,948 | 0,883 | 1 |
| RSAD2 IFI44L ISG15 IL1R2 OLAH FAM20A | 0,865 | 0,856 | 0,875 | 0,900 | 0,835 | 0,965 | 0,951 | 0,890 | 1 |
| OAS1 IFI44L SIGLEC1 IL1R2 OLAH RETN | 0,865 | 0,856 | 0,875 | 0,899 | 0,834 | 0,964 | 0,958 | 0,916 | 0,999 |
| OAS1 IFI44L HERC6 OLAH FAM20A MMP8 | 0,865 | 0,855 | 0,876 | 0,899 | 0,835 | 0,964 | 0,947 | 0,884 | 1 |
| OAS1 SIGLEC1 ISG15 IL1R2 RETN MMP8 | 0,865 | 0,856 | 0,875 | 0,901 | 0,837 | 0,964 | 0,971 | 0,938 | 1 |
| IFI27 OAS1 IFIT1 SLC1A2 RETN MMP8 | 0,865 | 0,856 | 0,875 | 0,900 | 0,836 | 0,965 | 0,939 | 0,865 | 1 |
| RSAD2 IFI27 OAS1 IFIT1 HERC6 MMP8 | 0,865 | 0,856 | 0,874 | 0,900 | 0,842 | 0,959 | 0,899 | 0,819 | 0,978 |
| IFI27 IFIT1 IFI44L ISG15 HERC6 MMP8 | 0,865 | 0,856 | 0,875 | 0,897 | 0,838 | 0,957 | 0,895 | 0,814 | 0,976 |
| RSAD2 IFIT1 ISG15 HERC6 OLAH FAM20A | 0,865 | 0,855 | 0,875 | 0,900 | 0,837 | 0,962 | 0,938 | 0,868 | 1 |
| RSAD2 ISG15 HERC6 OLAH FAM20A MMP8 | 0,865 | 0,855 | 0,875 | 0,901 | 0,839 | 0,962 | 0,939 | 0,869 | 1 |
| RSAD2 OAS1 SIGLEC1 ISG15 IL1R2 MMP8 | 0,865 | 0,856 | 0,875 | 0,903 | 0,844 | 0,962 | 0,965 | 0,929 | 1 |
| OAS1 IFI44L SIGLEC1 ISG15 HERC6 RETN | 0,865 | 0,855 | 0,875 | 0,896 | 0,828 | 0,964 | 0,951 | 0,903 | 0,999 |
| IFI27 OAS1 IFI44L SLC1A2 FAM20A MMP8 | 0,865 | 0,855 | 0,876 | 0,902 | 0,837 | 0,966 | 0,936 | 0,855 | 1 |
| RSAD2 IFI27 IFIT1 SIGLEC1 SLC1A2 MMP8 | 0,865 | 0,856 | 0,874 | 0,902 | 0,842 | 0,961 | 0,926 | 0,857 | 0,995 |
| RSAD2 IFI27 OAS1 IFI44L RETN MMP8 | 0,865 | 0,855 | 0,875 | 0,905 | 0,841 | 0,968 | 0,946 | 0,881 | 1 |
| RSAD2 IFIT1 SIGLEC1 HERC6 SLC1A2 RETN | 0,865 | 0,856 | 0,874 | 0,903 | 0,843 | 0,963 | 0,929 | 0,871 | 0,987 |

(suite)

| Modèle | AUC modèle | IC 95% | | AUC Train | IC 95% Train | | AUC Test | IC 95% Test | |
|---|---|---|---|---|---|---|---|---|---|
| OAS1 ISG15 SLC1A2 IL1R2 FAM20A MMP8 | 0,865 | 0,856 | 0,875 | 0,912 | 0,860 | 0,965 | 0,926 | 0,835 | 1 |
| RSAD2 IFI27 OAS1 IFIT1 RETN MMP8 | 0,865 | 0,855 | 0,875 | 0,905 | 0,841 | 0,969 | 0,948 | 0,884 | 1 |
| RSAD2 IL1R2 OLAH FAM20A RETN MMP8 | 0,865 | 0,855 | 0,875 | 0,903 | 0,840 | 0,966 | 0,942 | 0,870 | 1 |
| RSAD2 IFI44L HERC6 OLAH FAM20A RETN | 0,865 | 0,855 | 0,875 | 0,904 | 0,841 | 0,967 | 0,938 | 0,871 | 1 |
| RSAD2 IFIT1 ISG15 IL1R2 OLAH FAM20A | 0,865 | 0,856 | 0,874 | 0,899 | 0,833 | 0,965 | 0,952 | 0,890 | 1 |
| RSAD2 IFIT1 IFI44L SIGLEC1 HERC6 OLAH | 0,865 | 0,856 | 0,874 | 0,900 | 0,838 | 0,962 | 0,937 | 0,883 | 0,991 |
| IFIT1 IFI44L IL1R2 FAM20A RETN MMP8 | 0,865 | 0,855 | 0,875 | 0,903 | 0,840 | 0,967 | 0,946 | 0,873 | 1 |
| IFI44L SIGLEC1 ISG15 HERC6 OLAH RETN | 0,865 | 0,855 | 0,875 | 0,894 | 0,824 | 0,964 | 0,953 | 0,909 | 0,998 |
| OAS1 IFIT1 SLC1A2 OLAH FAM20A RETN | 0,865 | 0,855 | 0,875 | 0,909 | 0,851 | 0,968 | 0,935 | 0,861 | 1 |
| RSAD2 IFI44L SIGLEC1 SLC1A2 FAM20A RETN | 0,865 | 0,855 | 0,875 | 0,904 | 0,842 | 0,966 | 0,946 | 0,874 | 1 |
| IFIT1 SLC1A2 OLAH FAM20A RETN MMP8 | 0,865 | 0,855 | 0,875 | 0,906 | 0,846 | 0,966 | 0,941 | 0,872 | 1 |
| OAS1 IFI44L SLC1A2 IL1R2 FAM20A RETN | 0,865 | 0,855 | 0,875 | 0,908 | 0,852 | 0,963 | 0,925 | 0,837 | 1 |
| OAS1 IFI44L ISG15 HERC6 FAM20A RETN | 0,865 | 0,855 | 0,875 | 0,899 | 0,834 | 0,964 | 0,942 | 0,868 | 1 |
| RSAD2 ISG15 SLC1A2 OLAH FAM20A MMP8 | 0,865 | 0,855 | 0,874 | 0,906 | 0,848 | 0,965 | 0,932 | 0,857 | 1 |
| IFI44L ISG15 SLC1A2 IL1R2 OLAH RETN | 0,865 | 0,855 | 0,874 | 0,910 | 0,854 | 0,966 | 0,938 | 0,883 | 0,993 |
| IFI44L ISG15 SLC1A2 IL1R2 OLAH MMP8 | 0,865 | 0,856 | 0,874 | 0,904 | 0,847 | 0,960 | 0,935 | 0,875 | 0,994 |
| IFIT1 IFI44L SIGLEC1 IL1R2 OLAH RETN | 0,865 | 0,856 | 0,874 | 0,899 | 0,834 | 0,963 | 0,958 | 0,914 | 1 |
| RSAD2 IFI44L SIGLEC1 IL1R2 OLAH RETN | 0,865 | 0,855 | 0,874 | 0,897 | 0,831 | 0,964 | 0,962 | 0,922 | 1 |
| RSAD2 IFIT1 IFI44L IL1R2 FAM20A MMP8 | 0,865 | 0,856 | 0,874 | 0,904 | 0,841 | 0,966 | 0,943 | 0,869 | 1 |
| SIGLEC1 ISG15 HERC6 OLAH RETN MMP8 | 0,865 | 0,854 | 0,875 | 0,892 | 0,819 | 0,964 | 0,961 | 0,922 | 1,000 |
| RSAD2 IFIT1 SIGLEC1 ISG15 HERC6 OLAH | 0,865 | 0,855 | 0,875 | 0,901 | 0,839 | 0,962 | 0,940 | 0,888 | 0,992 |
| OAS1 IFI44L SIGLEC1 HERC6 OLAH RETN | 0,865 | 0,855 | 0,875 | 0,896 | 0,829 | 0,963 | 0,950 | 0,903 | 0,997 |

(suite)

| Modèle | AUC modèle | IC 95% | | AUC Train | IC 95% Train | | AUC Test | IC 95% Test | |
|---|---|---|---|---|---|---|---|---|---|
| OAS1 IFI44L SIGLEC1 ISG15 IL1R2 RETN | 0,865 | 0,856 | 0,874 | 0,898 | 0,833 | 0,963 | 0,959 | 0,918 | 0,999 |
| IFI27 IFIT1 IFI44L ISG15 SLC1A2 RETN | 0,865 | 0,855 | 0,875 | 0,902 | 0,838 | 0,966 | 0,945 | 0,878 | 1 |
| IFI27 OAS1 IFI44L HERC6 SLC1A2 MMP8 | 0,865 | 0,856 | 0,874 | 0,904 | 0,849 | 0,959 | 0,907 | 0,831 | 0,982 |
| IFIT1 SIGLEC1 ISG15 IL1R2 OLAH RETN | 0,865 | 0,856 | 0,874 | 0,900 | 0,836 | 0,963 | 0,961 | 0,919 | 1 |
| OAS1 ISG15 SLC1A2 OLAH FAM20A RETN | 0,865 | 0,855 | 0,875 | 0,912 | 0,855 | 0,969 | 0,937 | 0,865 | 1 |
| OAS1 IFI44L IL1R2 FAM20A RETN MMP8 | 0,865 | 0,855 | 0,874 | 0,907 | 0,845 | 0,969 | 0,943 | 0,868 | 1 |
| RSAD2 OAS1 SLC1A2 IL1R2 FAM20A RETN | 0,865 | 0,856 | 0,874 | 0,905 | 0,849 | 0,962 | 0,930 | 0,844 | 1 |
| RSAD2 IFI44L SIGLEC1 ISG15 IL1R2 RETN | 0,865 | 0,856 | 0,874 | 0,895 | 0,828 | 0,962 | 0,959 | 0,918 | 0,999 |
| OAS1 ISG15 HERC6 OLAH FAM20A MMP8 | 0,865 | 0,855 | 0,875 | 0,898 | 0,834 | 0,962 | 0,946 | 0,879 | 1 |
| IFI44L SLC1A2 IL1R2 OLAH RETN MMP8 | 0,865 | 0,855 | 0,874 | 0,906 | 0,849 | 0,963 | 0,942 | 0,890 | 0,995 |
| OAS1 IFIT1 ISG15 HERC6 IL1R2 RETN | 0,865 | 0,855 | 0,875 | 0,896 | 0,833 | 0,960 | 0,949 | 0,899 | 0,998 |
| OAS1 IFIT1 IFI44L IL1R2 FAM20A RETN | 0,865 | 0,855 | 0,874 | 0,900 | 0,835 | 0,965 | 0,939 | 0,863 | 1 |
| RSAD2 IFI27 OAS1 IFIT1 SLC1A2 RETN | 0,865 | 0,855 | 0,874 | 0,901 | 0,836 | 0,966 | 0,941 | 0,866 | 1 |
| IFIT1 ISG15 SLC1A2 OLAH FAM20A RETN | 0,865 | 0,854 | 0,875 | 0,902 | 0,840 | 0,964 | 0,938 | 0,868 | 1 |
| RSAD2 IFIT1 SIGLEC1 IL1R2 OLAH RETN | 0,865 | 0,855 | 0,874 | 0,898 | 0,832 | 0,964 | 0,959 | 0,916 | 1 |
| IFI27 OAS1 SIGLEC1 ISG15 HERC6 SLC1A2 | 0,865 | 0,856 | 0,874 | 0,900 | 0,844 | 0,956 | 0,951 | 0,900 | 1 |
| RSAD2 OAS1 SIGLEC1 HERC6 OLAH RETN | 0,865 | 0,855 | 0,875 | 0,895 | 0,827 | 0,962 | 0,950 | 0,903 | 0,997 |
| IFI27 SIGLEC1 ISG15 HERC6 SLC1A2 MMP8 | 0,865 | 0,855 | 0,874 | 0,903 | 0,845 | 0,961 | 0,925 | 0,857 | 0,993 |
| RSAD2 SLC1A2 OLAH FAM20A RETN MMP8 | 0,865 | 0,855 | 0,874 | 0,906 | 0,847 | 0,966 | 0,941 | 0,871 | 1 |
| RSAD2 IFI27 SIGLEC1 ISG15 SLC1A2 MMP8 | 0,865 | 0,855 | 0,874 | 0,903 | 0,844 | 0,961 | 0,930 | 0,863 | 0,997 |
| RSAD2 SIGLEC1 ISG15 HERC6 OLAH RETN | 0,864 | 0,855 | 0,874 | 0,896 | 0,830 | 0,963 | 0,954 | 0,910 | 0,998 |
| RSAD2 IFIT1 SIGLEC1 ISG15 IL1R2 MMP8 | 0,864 | 0,856 | 0,873 | 0,901 | 0,841 | 0,961 | 0,966 | 0,931 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFI44L SLC1A2 RETN MMP8 | 0,864 | 0,855 | 0,874 | 0,903 | 0,840 | 0,966 | 0,936 | 0,866 | 1 |
| IFIT1 ISG15 IL1R2 OLAH FAM20A RETN | 0,864 | 0,854 | 0,875 | 0,898 | 0,834 | 0,962 | 0,952 | 0,891 | 1 |
| RSAD2 OAS1 IFI44L IL1R2 FAM20A RETN | 0,864 | 0,854 | 0,874 | 0,900 | 0,836 | 0,965 | 0,938 | 0,862 | 1 |
| IFI27 IFIT1 ISG15 HERC6 SLC1A2 FAM20A | 0,864 | 0,854 | 0,875 | 0,905 | 0,846 | 0,964 | 0,933 | 0,850 | 1 |
| RSAD2 IFI27 OAS1 IFI44L SLC1A2 FAM20A | 0,864 | 0,853 | 0,875 | 0,903 | 0,839 | 0,967 | 0,935 | 0,855 | 1 |
| IFIT1 SIGLEC1 ISG15 HERC6 SLC1A2 RETN | 0,864 | 0,855 | 0,874 | 0,902 | 0,842 | 0,962 | 0,938 | 0,884 | 0,992 |
| IFI27 OAS1 IFIT1 HERC6 SLC1A2 MMP8 | 0,864 | 0,855 | 0,873 | 0,905 | 0,850 | 0,959 | 0,902 | 0,823 | 0,981 |
| OAS1 SLC1A2 IL1R2 FAM20A RETN MMP8 | 0,864 | 0,855 | 0,874 | 0,912 | 0,857 | 0,966 | 0,929 | 0,841 | 1 |
| RSAD2 IFIT1 SLC1A2 IL1R2 FAM20A RETN | 0,864 | 0,855 | 0,873 | 0,907 | 0,851 | 0,964 | 0,935 | 0,853 | 1 |
| RSAD2 IFI44L SLC1A2 IL1R2 FAM20A RETN | 0,864 | 0,855 | 0,874 | 0,907 | 0,852 | 0,962 | 0,927 | 0,839 | 1 |
| OAS1 ISG15 HERC6 SLC1A2 FAM20A RETN | 0,864 | 0,854 | 0,875 | 0,902 | 0,840 | 0,964 | 0,936 | 0,855 | 1 |
| RSAD2 IFI27 OAS1 ISG15 RETN MMP8 | 0,864 | 0,854 | 0,874 | 0,901 | 0,837 | 0,966 | 0,952 | 0,894 | 1 |
| RSAD2 ISG15 SLC1A2 IL1R2 FAM20A RETN | 0,864 | 0,855 | 0,874 | 0,903 | 0,846 | 0,959 | 0,929 | 0,840 | 1 |
| OAS1 IFI44L SIGLEC1 HERC6 SLC1A2 RETN | 0,864 | 0,855 | 0,874 | 0,904 | 0,842 | 0,965 | 0,951 | 0,901 | 1 |
| RSAD2 ISG15 SLC1A2 IL1R2 FAM20A MMP8 | 0,864 | 0,855 | 0,873 | 0,907 | 0,853 | 0,961 | 0,926 | 0,832 | 1 |
| OAS1 IFI44L SIGLEC1 ISG15 FAM20A MMP8 | 0,864 | 0,853 | 0,875 | 0,891 | 0,818 | 0,963 | 0,955 | 0,893 | 1 |
| RSAD2 IFI27 OAS1 HERC6 SLC1A2 FAM20A | 0,864 | 0,854 | 0,875 | 0,904 | 0,846 | 0,963 | 0,932 | 0,848 | 1 |
| RSAD2 IFI27 IFIT1 IFI44L RETN MMP8 | 0,864 | 0,854 | 0,874 | 0,905 | 0,842 | 0,969 | 0,951 | 0,892 | 1 |
| RSAD2 ISG15 HERC6 SLC1A2 IL1R2 OLAH | 0,864 | 0,855 | 0,873 | 0,905 | 0,848 | 0,962 | 0,943 | 0,889 | 0,998 |
| OAS1 ISG15 SLC1A2 IL1R2 FAM20A RETN | 0,864 | 0,854 | 0,874 | 0,905 | 0,849 | 0,962 | 0,927 | 0,841 | 1 |
| OAS1 SLC1A2 OLAH FAM20A RETN MMP8 | 0,864 | 0,854 | 0,874 | 0,907 | 0,848 | 0,967 | 0,938 | 0,864 | 1 |
| RSAD2 IFI27 IFIT1 IFI44L SLC1A2 FAM20A | 0,864 | 0,853 | 0,875 | 0,901 | 0,836 | 0,966 | 0,940 | 0,861 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1 SIGLEC1 HERC6 SLC1A2 RETN MMP8 | 0,864 | 0,855 | 0,873 | 0,904 | 0,844 | 0,963 | 0,932 | 0,875 | 0,988 |
| RSAD2 OAS1 SIGLEC1 HERC6 OLAH MMP8 | 0,864 | 0,854 | 0,874 | 0,896 | 0,833 | 0,959 | 0,949 | 0,902 | 0,996 |
| OAS1 ISG15 HERC6 IL1R2 RETN MMP8 | 0,864 | 0,854 | 0,874 | 0,898 | 0,836 | 0,961 | 0,948 | 0,895 | 1 |
| IFIT1 IFI44L HERC6 SLC1A2 OLAH RETN | 0,864 | 0,854 | 0,874 | 0,907 | 0,849 | 0,965 | 0,934 | 0,875 | 0,993 |
| IFI27 IFIT1 ISG15 SLC1A2 RETN MMP8 | 0,864 | 0,854 | 0,874 | 0,903 | 0,841 | 0,966 | 0,943 | 0,876 | 1 |
| OAS1 SIGLEC1 ISG15 IL1R2 OLAH RETN | 0,864 | 0,854 | 0,874 | 0,900 | 0,836 | 0,965 | 0,959 | 0,917 | 1 |
| IFI27 OAS1 IFIT1 SIGLEC1 HERC6 SLC1A2 | 0,864 | 0,855 | 0,873 | 0,901 | 0,846 | 0,957 | 0,948 | 0,897 | 0,999 |
| IFI44L ISG15 HERC6 SLC1A2 OLAH MMP8 | 0,864 | 0,854 | 0,874 | 0,904 | 0,846 | 0,962 | 0,939 | 0,885 | 0,994 |
| RSAD2 IFI27 OAS1 ISG15 SLC1A2 RETN | 0,864 | 0,854 | 0,873 | 0,901 | 0,836 | 0,965 | 0,937 | 0,861 | 1 |
| RSAD2 OAS1 IFIT1 SIGLEC1 OLAH RETN | 0,864 | 0,854 | 0,873 | 0,895 | 0,826 | 0,964 | 0,946 | 0,893 | 0,998 |
| OAS1 IFIT1 IFI44L SIGLEC1 OLAH MMP8 | 0,864 | 0,854 | 0,873 | 0,893 | 0,825 | 0,961 | 0,946 | 0,895 | 0,996 |
| RSAD2 SIGLEC1 ISG15 HERC6 SLC1A2 RETN | 0,864 | 0,854 | 0,873 | 0,904 | 0,844 | 0,965 | 0,948 | 0,898 | 0,998 |
| RSAD2 IFI27 IFIT1 ISG15 SLC1A2 RETN | 0,864 | 0,854 | 0,873 | 0,900 | 0,836 | 0,964 | 0,945 | 0,876 | 1 |
| OAS1 IFI44L ISG15 IL1R2 FAM20A RETN | 0,864 | 0,854 | 0,874 | 0,900 | 0,836 | 0,965 | 0,941 | 0,866 | 1 |
| IFIT1 IFI44L ISG15 IL1R2 FAM20A RETN | 0,864 | 0,854 | 0,873 | 0,900 | 0,837 | 0,963 | 0,945 | 0,878 | 1 |
| IFI44L SIGLEC1 ISG15 HERC6 OLAH MMP8 | 0,864 | 0,854 | 0,873 | 0,894 | 0,828 | 0,960 | 0,952 | 0,907 | 0,997 |
| RSAD2 IFI27 IFIT1 HERC6 SLC1A2 FAM20A | 0,864 | 0,853 | 0,874 | 0,906 | 0,848 | 0,964 | 0,933 | 0,850 | 1 |
| IFIT1 IFI44L HERC6 SLC1A2 OLAH MMP8 | 0,864 | 0,854 | 0,874 | 0,905 | 0,848 | 0,963 | 0,940 | 0,886 | 0,994 |
| IFIT1 IFI44L SIGLEC1 HERC6 SLC1A2 RETN | 0,864 | 0,855 | 0,873 | 0,902 | 0,842 | 0,962 | 0,929 | 0,871 | 0,987 |
| OAS1 IFIT1 IFI44L SIGLEC1 OLAH RETN | 0,864 | 0,854 | 0,873 | 0,894 | 0,825 | 0,964 | 0,938 | 0,882 | 0,994 |
| RSAD2 IFI27 IFI44L ISG15 RETN MMP8 | 0,864 | 0,853 | 0,874 | 0,904 | 0,841 | 0,968 | 0,953 | 0,897 | 1 |
| IFIT1 IFI44L ISG15 SLC1A2 OLAH MMP8 | 0,863 | 0,854 | 0,873 | 0,905 | 0,847 | 0,963 | 0,938 | 0,884 | 0,992 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 SIGLEC1 ISG15 IL1R2 RETN | 0,863 | 0,854 | 0,873 | 0,897 | 0,831 | 0,962 | 0,967 | 0,930 | 1 |
| RSAD2 IFIT1 IL1R2 FAM20A RETN MMP8 | 0,863 | 0,854 | 0,873 | 0,904 | 0,840 | 0,968 | 0,945 | 0,872 | 1 |
| RSAD2 ISG15 SLC1A2 OLAH FAM20A RETN | 0,863 | 0,854 | 0,873 | 0,908 | 0,849 | 0,967 | 0,930 | 0,853 | 1 |
| RSAD2 OAS1 ISG15 HERC6 IL1R2 RETN | 0,863 | 0,854 | 0,873 | 0,896 | 0,833 | 0,959 | 0,948 | 0,896 | 0,999 |
| IFI27 OAS1 IFIT1 HERC6 SLC1A2 FAM20A | 0,863 | 0,853 | 0,874 | 0,905 | 0,847 | 0,963 | 0,933 | 0,850 | 1 |
| IFI44L ISG15 SLC1A2 OLAH RETN MMP8 | 0,863 | 0,854 | 0,873 | 0,907 | 0,847 | 0,967 | 0,943 | 0,893 | 0,994 |
| RSAD2 ISG15 HERC6 SLC1A2 FAM20A RETN | 0,863 | 0,853 | 0,874 | 0,902 | 0,840 | 0,964 | 0,937 | 0,858 | 1 |
| IFI44L HERC6 SLC1A2 IL1R2 OLAH MMP8 | 0,863 | 0,854 | 0,873 | 0,908 | 0,852 | 0,963 | 0,943 | 0,891 | 0,996 |
| RSAD2 IFI27 IFI44L ISG15 SLC1A2 RETN | 0,863 | 0,854 | 0,873 | 0,904 | 0,840 | 0,967 | 0,946 | 0,881 | 1 |
| RSAD2 IFI27 IFI44L ISG15 HERC6 MMP8 | 0,863 | 0,854 | 0,872 | 0,896 | 0,838 | 0,955 | 0,896 | 0,817 | 0,975 |
| OAS1 IFI44L SIGLEC1 HERC6 OLAH MMP8 | 0,863 | 0,854 | 0,873 | 0,895 | 0,831 | 0,958 | 0,950 | 0,904 | 0,996 |
| RSAD2 ISG15 IL1R2 OLAH FAM20A RETN | 0,863 | 0,853 | 0,873 | 0,898 | 0,832 | 0,964 | 0,943 | 0,873 | 1 |
| RSAD2 IFIT1 SLC1A2 OLAH FAM20A RETN | 0,863 | 0,854 | 0,873 | 0,907 | 0,848 | 0,967 | 0,938 | 0,866 | 1 |
| RSAD2 OAS1 HERC6 SLC1A2 IL1R2 OLAH | 0,863 | 0,854 | 0,873 | 0,904 | 0,846 | 0,961 | 0,941 | 0,884 | 0,999 |
| RSAD2 IFI27 IFIT1 ISG15 RETN MMP8 | 0,863 | 0,853 | 0,873 | 0,903 | 0,839 | 0,966 | 0,953 | 0,895 | 1 |
| IFIT1 IFI44L SIGLEC1 OLAH RETN MMP8 | 0,863 | 0,853 | 0,873 | 0,896 | 0,828 | 0,964 | 0,959 | 0,915 | 1 |
| OAS1 IFIT1 HERC6 SLC1A2 IL1R2 OLAH | 0,863 | 0,853 | 0,873 | 0,903 | 0,846 | 0,961 | 0,943 | 0,891 | 0,996 |
| IFI27 IFIT1 IFI44L ISG15 SLC1A2 FAM20A | 0,863 | 0,853 | 0,874 | 0,899 | 0,834 | 0,965 | 0,941 | 0,863 | 1 |
| RSAD2 HERC6 SLC1A2 IL1R2 OLAH MMP8 | 0,863 | 0,854 | 0,872 | 0,904 | 0,847 | 0,962 | 0,945 | 0,892 | 0,998 |
| RSAD2 IFIT1 ISG15 SLC1A2 OLAH FAM20A | 0,863 | 0,854 | 0,873 | 0,907 | 0,847 | 0,967 | 0,937 | 0,865 | 1 |
| RSAD2 IFIT1 SIGLEC1 ISG15 IL1R2 RETN | 0,863 | 0,854 | 0,872 | 0,897 | 0,831 | 0,963 | 0,960 | 0,919 | 1 |
| IFIT1 HERC6 SLC1A2 IL1R2 OLAH MMP8 | 0,863 | 0,854 | 0,873 | 0,904 | 0,847 | 0,962 | 0,950 | 0,903 | 0,997 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 HERC6 IL1R2 RETN MMP8 | 0,863 | 0,853 | 0,873 | 0,895 | 0,827 | 0,962 | 0,961 | 0,921 | 1 |
| OAS1 IFIT1 SIGLEC1 ISG15 OLAH RETN | 0,863 | 0,853 | 0,873 | 0,895 | 0,825 | 0,964 | 0,942 | 0,889 | 0,995 |
| OAS1 IFI44L HERC6 SLC1A2 OLAH RETN | 0,863 | 0,853 | 0,874 | 0,907 | 0,849 | 0,966 | 0,935 | 0,875 | 0,994 |
| RSAD2 OAS1 IFIT1 SIGLEC1 OLAH MMP8 | 0,863 | 0,854 | 0,873 | 0,893 | 0,825 | 0,961 | 0,946 | 0,895 | 0,996 |
| RSAD2 IFIT1 IFI44L IL1R2 OLAH FAM20A | 0,863 | 0,854 | 0,872 | 0,898 | 0,832 | 0,964 | 0,950 | 0,888 | 1 |
| IFI44L HERC6 SLC1A2 IL1R2 OLAH RETN | 0,863 | 0,853 | 0,873 | 0,907 | 0,850 | 0,964 | 0,941 | 0,886 | 0,997 |
| RSAD2 IFIT1 SIGLEC1 HERC6 RETN MMP8 | 0,863 | 0,853 | 0,873 | 0,894 | 0,824 | 0,964 | 0,924 | 0,863 | 0,984 |
| RSAD2 IFI27 IFI44L SIGLEC1 SLC1A2 MMP8 | 0,863 | 0,854 | 0,872 | 0,901 | 0,841 | 0,960 | 0,929 | 0,861 | 0,998 |
| RSAD2 SLC1A2 IL1R2 FAM20A RETN MMP8 | 0,863 | 0,854 | 0,872 | 0,909 | 0,855 | 0,963 | 0,926 | 0,832 | 1 |
| IFI44L ISG15 HERC6 SLC1A2 IL1R2 OLAH | 0,863 | 0,853 | 0,873 | 0,906 | 0,850 | 0,963 | 0,943 | 0,890 | 0,997 |
| OAS1 IFI44L ISG15 HERC6 IL1R2 RETN | 0,863 | 0,853 | 0,873 | 0,898 | 0,835 | 0,961 | 0,952 | 0,904 | 1 |
| IFI27 OAS1 IFIT1 ISG15 FAM20A MMP8 | 0,863 | 0,852 | 0,874 | 0,895 | 0,827 | 0,963 | 0,937 | 0,857 | 1 |
| RSAD2 IFI27 OAS1 SLC1A2 RETN MMP8 | 0,863 | 0,853 | 0,873 | 0,901 | 0,837 | 0,965 | 0,929 | 0,850 | 1 |
| RSAD2 IFIT1 HERC6 SLC1A2 IL1R2 OLAH | 0,863 | 0,853 | 0,873 | 0,904 | 0,846 | 0,961 | 0,942 | 0,889 | 0,996 |
| RSAD2 IFI27 OAS1 ISG15 FAM20A MMP8 | 0,863 | 0,853 | 0,873 | 0,893 | 0,824 | 0,961 | 0,938 | 0,858 | 1 |
| RSAD2 ISG15 HERC6 FAM20A RETN MMP8 | 0,863 | 0,853 | 0,873 | 0,904 | 0,842 | 0,967 | 0,936 | 0,859 | 1 |
| OAS1 IFIT1 HERC6 SLC1A2 IL1R2 MMP8 | 0,863 | 0,854 | 0,872 | 0,903 | 0,846 | 0,959 | 0,951 | 0,903 | 1,000 |
| RSAD2 IFI27 OAS1 IFIT1 FAM20A MMP8 | 0,863 | 0,853 | 0,873 | 0,896 | 0,829 | 0,963 | 0,936 | 0,856 | 1 |
| RSAD2 IFI27 OAS1 SIGLEC1 HERC6 SLC1A2 | 0,863 | 0,854 | 0,872 | 0,901 | 0,845 | 0,956 | 0,949 | 0,898 | 0,999 |
| IFIT1 IFI44L ISG15 HERC6 SLC1A2 OLAH | 0,863 | 0,853 | 0,873 | 0,906 | 0,849 | 0,963 | 0,938 | 0,883 | 0,993 |
| RSAD2 OAS1 SIGLEC1 ISG15 HERC6 OLAH | 0,863 | 0,853 | 0,873 | 0,896 | 0,833 | 0,959 | 0,950 | 0,904 | 0,996 |
| RSAD2 IFIT1 IFI44L ISG15 IL1R2 FAM20A | 0,863 | 0,854 | 0,872 | 0,898 | 0,833 | 0,962 | 0,948 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L HERC6 SLC1A2 OLAH RETN MMP8 | 0,863 | 0,853 | 0,873 | 0,906 | 0,847 | 0,965 | 0,945 | 0,895 | 0,995 |
| IFIT1 ISG15 HERC6 SLC1A2 IL1R2 OLAH | 0,863 | 0,854 | 0,872 | 0,904 | 0,846 | 0,961 | 0,942 | 0,889 | 0,996 |
| OAS1 IFIT1 IFI44L HERC6 FAM20A RETN | 0,863 | 0,853 | 0,873 | 0,895 | 0,827 | 0,963 | 0,930 | 0,851 | 1 |
| IFI27 IFIT1 IFI44L SLC1A2 RETN MMP8 | 0,863 | 0,853 | 0,873 | 0,902 | 0,839 | 0,966 | 0,942 | 0,875 | 1 |
| IFI44L ISG15 HERC6 SLC1A2 OLAH RETN | 0,863 | 0,853 | 0,873 | 0,905 | 0,846 | 0,965 | 0,943 | 0,891 | 0,996 |
| IFI27 OAS1 IFIT1 IFI44L ISG15 FAM20A | 0,863 | 0,852 | 0,873 | 0,895 | 0,826 | 0,964 | 0,940 | 0,861 | 1 |
| IFI44L ISG15 HERC6 SLC1A2 FAM20A RETN | 0,863 | 0,852 | 0,873 | 0,898 | 0,832 | 0,963 | 0,946 | 0,868 | 1 |
| RSAD2 IFI44L ISG15 IL1R2 FAM20A MMP8 | 0,863 | 0,853 | 0,872 | 0,903 | 0,842 | 0,963 | 0,936 | 0,855 | 1 |
| IFI27 OAS1 ISG15 SLC1A2 RETN MMP8 | 0,863 | 0,853 | 0,873 | 0,900 | 0,838 | 0,963 | 0,935 | 0,862 | 1 |
| RSAD2 IFI44L SIGLEC1 ISG15 HERC6 RETN | 0,863 | 0,853 | 0,872 | 0,896 | 0,828 | 0,965 | 0,940 | 0,888 | 0,992 |
| OAS1 ISG15 HERC6 SLC1A2 IL1R2 OLAH | 0,863 | 0,853 | 0,872 | 0,905 | 0,849 | 0,962 | 0,941 | 0,887 | 0,995 |
| OAS1 IFIT1 IFI44L SIGLEC1 ISG15 OLAH | 0,863 | 0,853 | 0,872 | 0,894 | 0,826 | 0,962 | 0,942 | 0,889 | 0,996 |
| RSAD2 SIGLEC1 ISG15 IL1R2 OLAH RETN | 0,863 | 0,853 | 0,872 | 0,897 | 0,832 | 0,963 | 0,963 | 0,923 | 1 |
| RSAD2 SIGLEC1 ISG15 HERC6 RETN MMP8 | 0,863 | 0,852 | 0,873 | 0,896 | 0,828 | 0,965 | 0,945 | 0,895 | 0,994 |
| OAS1 IFIT1 ISG15 HERC6 SLC1A2 IL1R2 | 0,862 | 0,853 | 0,872 | 0,902 | 0,846 | 0,959 | 0,951 | 0,902 | 1 |
| RSAD2 IFI44L HERC6 SLC1A2 IL1R2 OLAH | 0,862 | 0,852 | 0,873 | 0,907 | 0,851 | 0,963 | 0,941 | 0,886 | 0,996 |
| OAS1 IFIT1 IFI44L HERC6 SLC1A2 OLAH | 0,862 | 0,852 | 0,873 | 0,905 | 0,848 | 0,962 | 0,934 | 0,875 | 0,992 |
| IFIT1 IFI44L SLC1A2 IL1R2 OLAH MMP8 | 0,862 | 0,853 | 0,872 | 0,904 | 0,847 | 0,961 | 0,949 | 0,901 | 0,997 |
| RSAD2 IFI27 IFIT1 ISG15 HERC6 MMP8 | 0,862 | 0,854 | 0,871 | 0,898 | 0,839 | 0,956 | 0,893 | 0,813 | 0,974 |
| IFIT1 IFI44L ISG15 HERC6 IL1R2 RETN | 0,862 | 0,853 | 0,872 | 0,896 | 0,832 | 0,960 | 0,949 | 0,900 | 0,998 |
| OAS1 IFIT1 SLC1A2 IL1R2 OLAH MMP8 | 0,862 | 0,853 | 0,872 | 0,903 | 0,845 | 0,960 | 0,950 | 0,903 | 0,997 |
| IFI27 OAS1 IFI44L ISG15 SLC1A2 FAM20A | 0,862 | 0,852 | 0,873 | 0,898 | 0,833 | 0,964 | 0,942 | 0,865 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1 ISG15 SLC1A2 IL1R2 OLAH MMP8 | 0,862 | 0,854 | 0,871 | 0,902 | 0,844 | 0,960 | 0,949 | 0,902 | 0,996 |
| OAS1 ISG15 HERC6 SLC1A2 OLAH RETN | 0,862 | 0,852 | 0,873 | 0,904 | 0,846 | 0,963 | 0,940 | 0,880 | 1 |
| OAS1 HERC6 SLC1A2 IL1R2 OLAH MMP8 | 0,862 | 0,853 | 0,872 | 0,905 | 0,848 | 0,962 | 0,939 | 0,883 | 0,995 |
| IFIT1 ISG15 HERC6 SLC1A2 FAM20A RETN | 0,862 | 0,852 | 0,873 | 0,901 | 0,838 | 0,964 | 0,939 | 0,860 | 1 |
| IFIT1 IFI44L ISG15 SLC1A2 OLAH RETN | 0,862 | 0,853 | 0,872 | 0,908 | 0,850 | 0,966 | 0,934 | 0,877 | 0,990 |
| RSAD2 OAS1 IFI44L HERC6 SLC1A2 OLAH | 0,862 | 0,852 | 0,872 | 0,905 | 0,848 | 0,962 | 0,938 | 0,883 | 0,994 |
| RSAD2 IFIT1 ISG15 HERC6 IL1R2 RETN | 0,862 | 0,853 | 0,872 | 0,897 | 0,833 | 0,961 | 0,949 | 0,900 | 0,998 |
| RSAD2 IFI44L SIGLEC1 HERC6 OLAH RETN | 0,862 | 0,853 | 0,872 | 0,896 | 0,829 | 0,963 | 0,943 | 0,893 | 0,994 |
| IFIT1 ISG15 HERC6 FAM20A RETN MMP8 | 0,862 | 0,852 | 0,872 | 0,901 | 0,837 | 0,966 | 0,938 | 0,862 | 1 |
| RSAD2 OAS1 SIGLEC1 OLAH RETN MMP8 | 0,862 | 0,852 | 0,872 | 0,892 | 0,822 | 0,962 | 0,959 | 0,917 | 1,000 |
| IFI27 IFIT1 IFI44L SIGLEC1 ISG15 MMP8 | 0,862 | 0,853 | 0,872 | 0,890 | 0,824 | 0,957 | 0,911 | 0,836 | 0,986 |
| ISG15 HERC6 IL1R2 OLAH RETN MMP8 | 0,862 | 0,852 | 0,872 | 0,900 | 0,837 | 0,964 | 0,961 | 0,922 | 1 |
| RSAD2 OAS1 OLAH FAM20A RETN MMP8 | 0,862 | 0,852 | 0,872 | 0,895 | 0,826 | 0,964 | 0,952 | 0,892 | 1 |
| RSAD2 OAS1 IFI44L SIGLEC1 HERC6 RETN | 0,862 | 0,852 | 0,872 | 0,889 | 0,817 | 0,961 | 0,945 | 0,895 | 0,995 |
| RSAD2 IFIT1 ISG15 IL1R2 FAM20A RETN | 0,862 | 0,852 | 0,872 | 0,898 | 0,832 | 0,963 | 0,947 | 0,878 | 1 |
| OAS1 IFI44L ISG15 HERC6 SLC1A2 OLAH | 0,862 | 0,852 | 0,872 | 0,906 | 0,849 | 0,963 | 0,937 | 0,881 | 0,993 |
| RSAD2 OAS1 IFIT1 OLAH FAM20A MMP8 | 0,862 | 0,852 | 0,872 | 0,893 | 0,823 | 0,963 | 0,951 | 0,890 | 1 |
| RSAD2 OAS1 IFI44L OLAH FAM20A MMP8 | 0,862 | 0,852 | 0,873 | 0,897 | 0,829 | 0,965 | 0,947 | 0,888 | 1 |
| RSAD2 IFI44L HERC6 SLC1A2 OLAH RETN | 0,862 | 0,852 | 0,872 | 0,908 | 0,850 | 0,966 | 0,934 | 0,876 | 0,992 |
| OAS1 HERC6 SLC1A2 OLAH RETN MMP8 | 0,862 | 0,852 | 0,872 | 0,902 | 0,843 | 0,962 | 0,941 | 0,886 | 0,997 |
| RSAD2 IFI27 IFIT1 IFI44L SLC1A2 RETN | 0,862 | 0,852 | 0,872 | 0,901 | 0,837 | 0,965 | 0,942 | 0,873 | 1 |
| OAS1 IFIT1 ISG15 SLC1A2 IL1R2 OLAH | 0,862 | 0,852 | 0,872 | 0,908 | 0,851 | 0,965 | 0,943 | 0,892 | 0,995 |

(suite)

| Modèle | AUC modèle | IC 95% | | AUC Train | IC 95% Train | | AUC Test | IC 95% Test | |
|---|---|---|---|---|---|---|---|---|---|
| OAS1 IFI44L HERC6 SLC1A2 OLAH MMP8 | 0,862 | 0,852 | 0,872 | 0,905 | 0,848 | 0,962 | 0,939 | 0,884 | 0,994 |
| RSAD2 SIGLEC1 ISG15 HERC6 OLAH MMP8 | 0,862 | 0,852 | 0,872 | 0,897 | 0,834 | 0,960 | 0,949 | 0,902 | 0,996 |
| RSAD2 OAS1 HERC6 SLC1A2 OLAH RETN | 0,862 | 0,852 | 0,872 | 0,903 | 0,843 | 0,962 | 0,939 | 0,877 | 1 |
| IFIT1 IFI44L ISG15 SLC1A2 IL1R2 OLAH | 0,862 | 0,853 | 0,871 | 0,910 | 0,853 | 0,966 | 0,946 | 0,896 | 0,996 |
| RSAD2 ISG15 HERC6 IL1R2 RETN MMP8 | 0,862 | 0,852 | 0,872 | 0,897 | 0,834 | 0,960 | 0,948 | 0,895 | 1 |
| IFI44L SIGLEC1 ISG15 OLAH RETN MMP8 | 0,862 | 0,852 | 0,872 | 0,892 | 0,822 | 0,962 | 0,962 | 0,921 | 1 |
| OAS1 IFI44L SIGLEC1 ISG15 HERC6 OLAH | 0,862 | 0,852 | 0,872 | 0,896 | 0,833 | 0,959 | 0,953 | 0,909 | 0,998 |
| OAS1 ISG15 HERC6 SLC1A2 IL1R2 RETN | 0,862 | 0,852 | 0,872 | 0,902 | 0,844 | 0,959 | 0,930 | 0,859 | 1 |
| IFIT1 IFI44L SLC1A2 OLAH RETN MMP8 | 0,862 | 0,852 | 0,872 | 0,905 | 0,845 | 0,966 | 0,947 | 0,898 | 0,995 |
| RSAD2 ISG15 IL1R2 FAM20A RETN MMP8 | 0,862 | 0,852 | 0,871 | 0,901 | 0,838 | 0,964 | 0,936 | 0,854 | 1 |
| IFIT1 SIGLEC1 ISG15 OLAH RETN MMP8 | 0,862 | 0,852 | 0,872 | 0,895 | 0,827 | 0,962 | 0,962 | 0,920 | 1 |
| RSAD2 IFIT1 SLC1A2 IL1R2 OLAH MMP8 | 0,862 | 0,853 | 0,871 | 0,903 | 0,846 | 0,960 | 0,951 | 0,905 | 0,997 |
| RSAD2 IFI27 IFI44L SLC1A2 RETN MMP8 | 0,862 | 0,852 | 0,872 | 0,902 | 0,839 | 0,965 | 0,935 | 0,863 | 1 |
| RSAD2 SIGLEC1 HERC6 SLC1A2 RETN MMP8 | 0,862 | 0,852 | 0,871 | 0,903 | 0,841 | 0,965 | 0,940 | 0,887 | 0,993 |
| RSAD2 IFIT1 ISG15 HERC6 SLC1A2 IL1R2 | 0,862 | 0,853 | 0,871 | 0,903 | 0,846 | 0,959 | 0,951 | 0,902 | 1 |
| OAS1 IFIT1 SIGLEC1 ISG15 OLAH MMP8 | 0,862 | 0,852 | 0,871 | 0,893 | 0,825 | 0,961 | 0,943 | 0,892 | 0,995 |
| IFI27 OAS1 IFIT1 SIGLEC1 ISG15 MMP8 | 0,862 | 0,852 | 0,871 | 0,889 | 0,821 | 0,956 | 0,913 | 0,837 | 0,989 |
| RSAD2 HERC6 SLC1A2 IL1R2 OLAH RETN | 0,862 | 0,852 | 0,872 | 0,905 | 0,848 | 0,963 | 0,937 | 0,877 | 0,997 |
| IFIT1 ISG15 HERC6 IL1R2 RETN MMP8 | 0,862 | 0,852 | 0,872 | 0,896 | 0,830 | 0,961 | 0,959 | 0,917 | 1 |
| RSAD2 OAS1 IFIT1 HERC6 IL1R2 MMP8 | 0,862 | 0,853 | 0,871 | 0,897 | 0,835 | 0,959 | 0,949 | 0,902 | 0,996 |
| IFIT1 IFI44L IL1R2 OLAH FAM20A RETN | 0,862 | 0,852 | 0,871 | 0,899 | 0,835 | 0,964 | 0,950 | 0,892 | 1 |
| IFI44L HERC6 IL1R2 OLAH RETN MMP8 | 0,862 | 0,852 | 0,872 | 0,900 | 0,836 | 0,963 | 0,960 | 0,920 | 0,999 |

(suite)

| Modèle | AUC modèle | IC 95% | | AUC Train | IC 95% Train | | AUC Test | IC 95% Test | |
|---|---|---|---|---|---|---|---|---|---|
| IFIT1 IFI44L SLC1A2 IL1R2 OLAH RETN | 0,862 | 0,852 | 0,871 | 0,910 | 0,854 | 0,966 | 0,945 | 0,894 | 0,995 |
| RSAD2 OAS1 IFIT1 ISG15 HERC6 IL1R2 | 0,862 | 0,852 | 0,871 | 0,897 | 0,838 | 0,956 | 0,947 | 0,897 | 0,997 |
| RSAD2 IFI44L ISG15 HERC6 SLC1A2 OLAH | 0,862 | 0,852 | 0,872 | 0,906 | 0,849 | 0,963 | 0,934 | 0,877 | 0,990 |
| IFI44L ISG15 HERC6 IL1R2 RETN MMP8 | 0,862 | 0,852 | 0,871 | 0,897 | 0,833 | 0,962 | 0,955 | 0,910 | 1 |
| OAS1 IFIT1 ISG15 HERC6 IL1R2 MMP8 | 0,862 | 0,852 | 0,871 | 0,898 | 0,840 | 0,957 | 0,949 | 0,900 | 0,997 |
| RSAD2 ISG15 HERC6 IL1R2 OLAH RETN | 0,862 | 0,852 | 0,872 | 0,899 | 0,836 | 0,962 | 0,952 | 0,907 | 0,998 |
| RSAD2 OAS1 SIGLEC1 HERC6 RETN MMP8 | 0,862 | 0,852 | 0,872 | 0,892 | 0,820 | 0,963 | 0,943 | 0,893 | 0,994 |
| IFI44L ISG15 HERC6 IL1R2 OLAH RETN | 0,862 | 0,852 | 0,871 | 0,900 | 0,836 | 0,964 | 0,951 | 0,905 | 0,997 |
| OAS1 ISG15 HERC6 IL1R2 OLAH RETN | 0,862 | 0,851 | 0,872 | 0,900 | 0,837 | 0,962 | 0,948 | 0,899 | 0,997 |
| RSAD2 IFIT1 IFI44L HERC6 SLC1A2 OLAH | 0,862 | 0,852 | 0,871 | 0,905 | 0,848 | 0,962 | 0,934 | 0,876 | 0,991 |
| RSAD2 IFI27 IFIT1 ISG15 FAM20A MMP8 | 0,862 | 0,851 | 0,872 | 0,895 | 0,828 | 0,962 | 0,935 | 0,854 | 1 |
| OAS1 IFIT1 IFI44L HERC6 IL1R2 RETN | 0,862 | 0,852 | 0,871 | 0,895 | 0,828 | 0,961 | 0,948 | 0,900 | 0,996 |
| RSAD2 IFI44L ISG15 HERC6 IL1R2 RETN | 0,862 | 0,852 | 0,871 | 0,900 | 0,837 | 0,962 | 0,952 | 0,905 | 0,999 |
| OAS1 SIGLEC1 ISG15 HERC6 OLAH MMP8 | 0,862 | 0,852 | 0,871 | 0,892 | 0,827 | 0,956 | 0,955 | 0,912 | 0,999 |
| RSAD2 IFIT1 SIGLEC1 OLAH RETN MMP8 | 0,862 | 0,852 | 0,871 | 0,896 | 0,828 | 0,964 | 0,959 | 0,915 | 1 |
| OAS1 IFIT1 IFI44L HERC6 IL1R2 MMP8 | 0,862 | 0,852 | 0,871 | 0,895 | 0,832 | 0,958 | 0,949 | 0,902 | 0,996 |
| IFI27 OAS1 IFIT1 IFI44L SIGLEC1 MMP8 | 0,861 | 0,852 | 0,871 | 0,889 | 0,823 | 0,956 | 0,916 | 0,842 | 0,990 |
| IFIT1 HERC6 SLC1A2 OLAH RETN MMP8 | 0,861 | 0,851 | 0,872 | 0,903 | 0,843 | 0,963 | 0,950 | 0,902 | 0,998 |
| RSAD2 ISG15 HERC6 SLC1A2 OLAH RETN | 0,861 | 0,852 | 0,871 | 0,904 | 0,845 | 0,963 | 0,943 | 0,888 | 0,999 |
| RSAD2 IFI27 IFIT1 IFI44L HERC6 MMP8 | 0,861 | 0,852 | 0,871 | 0,897 | 0,838 | 0,956 | 0,892 | 0,812 | 0,973 |
| RSAD2 HERC6 SLC1A2 OLAH RETN MMP8 | 0,861 | 0,852 | 0,871 | 0,903 | 0,843 | 0,963 | 0,943 | 0,890 | 0,997 |
| OAS1 IFI44L HERC6 SLC1A2 IL1R2 OLAH | 0,861 | 0,851 | 0,872 | 0,907 | 0,851 | 0,963 | 0,939 | 0,882 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L HERC6 SLC1A2 OLAH MMP8 | 0,861 | 0,851 | 0,871 | 0,905 | 0,848 | 0,962 | 0,932 | 0,873 | 0,990 |
| IFIT1 IFI44L SIGLEC1 ISG15 OLAH MMP8 | 0,861 | 0,852 | 0,871 | 0,891 | 0,824 | 0,958 | 0,946 | 0,894 | 0,998 |
| IFI27 IFIT1 IFI44L HERC6 SLC1A2 MMP8 | 0,861 | 0,852 | 0,871 | 0,900 | 0,842 | 0,957 | 0,902 | 0,820 | 0,984 |
| ISG15 HERC6 SLC1A2 IL1R2 OLAH RETN | 0,861 | 0,852 | 0,871 | 0,907 | 0,851 | 0,964 | 0,941 | 0,884 | 0,999 |
| RSAD2 OAS1 IFIT1 HERC6 SLC1A2 IL1R2 | 0,861 | 0,852 | 0,871 | 0,900 | 0,843 | 0,958 | 0,950 | 0,899 | 1 |
| OAS1 HERC6 SLC1A2 IL1R2 OLAH RETN | 0,861 | 0,851 | 0,871 | 0,904 | 0,846 | 0,961 | 0,933 | 0,869 | 0,996 |
| RSAD2 IFI27 IFIT1 SLC1A2 RETN MMP8 | 0,861 | 0,852 | 0,871 | 0,902 | 0,839 | 0,965 | 0,942 | 0,874 | 1 |
| IFI27 OAS1 IFI44L SIGLEC1 HERC6 SLC1A2 | 0,861 | 0,853 | 0,870 | 0,901 | 0,846 | 0,957 | 0,947 | 0,895 | 0,999 |
| OAS1 IFIT1 HERC6 SLC1A2 OLAH MMP8 | 0,861 | 0,851 | 0,872 | 0,902 | 0,844 | 0,960 | 0,946 | 0,894 | 0,997 |
| RSAD2 IFI27 ISG15 SLC1A2 RETN MMP8 | 0,861 | 0,851 | 0,871 | 0,901 | 0,839 | 0,964 | 0,934 | 0,860 | 1 |
| IFIT1 HERC6 IL1R2 OLAH RETN MMP8 | 0,861 | 0,851 | 0,871 | 0,899 | 0,835 | 0,963 | 0,962 | 0,924 | 1 |
| OAS1 IFIT1 IFI44L SLC1A2 OLAH MMP8 | 0,861 | 0,851 | 0,871 | 0,905 | 0,847 | 0,963 | 0,938 | 0,884 | 0,992 |
| RSAD2 OAS1 IFIT1 HERC6 IL1R2 RETN | 0,861 | 0,852 | 0,871 | 0,894 | 0,827 | 0,961 | 0,947 | 0,898 | 0,996 |
| IFIT1 HERC6 SLC1A2 IL1R2 OLAH RETN | 0,861 | 0,851 | 0,871 | 0,904 | 0,846 | 0,962 | 0,940 | 0,883 | 0,997 |
| RSAD2 OAS1 IFIT1 IFI44L SIGLEC1 OLAH | 0,861 | 0,852 | 0,871 | 0,893 | 0,825 | 0,962 | 0,946 | 0,894 | 0,997 |
| IFIT1 IFI44L ISG15 HERC6 IL1R2 MMP8 | 0,861 | 0,852 | 0,870 | 0,896 | 0,836 | 0,957 | 0,947 | 0,897 | 0,996 |
| RSAD2 OAS1 IFI44L SIGLEC1 HERC6 OLAH | 0,861 | 0,852 | 0,871 | 0,896 | 0,833 | 0,959 | 0,949 | 0,902 | 0,996 |
| RSAD2 IFIT1 IL1R2 OLAH FAM20A RETN | 0,861 | 0,852 | 0,871 | 0,898 | 0,831 | 0,964 | 0,947 | 0,880 | 1 |
| RSAD2 OAS1 ISG15 OLAH FAM20A MMP8 | 0,861 | 0,851 | 0,871 | 0,893 | 0,824 | 0,963 | 0,947 | 0,882 | 1 |
| ISG15 HERC6 SLC1A2 OLAH RETN MMP8 | 0,861 | 0,851 | 0,871 | 0,905 | 0,846 | 0,964 | 0,952 | 0,905 | 0,999 |
| IFIT1 SLC1A2 IL1R2 OLAH RETN MMP8 | 0,861 | 0,852 | 0,871 | 0,906 | 0,849 | 0,964 | 0,957 | 0,914 | 0,999 |
| OAS1 HERC6 IL1R2 OLAH RETN MMP8 | 0,861 | 0,851 | 0,871 | 0,899 | 0,835 | 0,963 | 0,958 | 0,916 | 0,999 |

(suite)

| Modèle | AUC modèle | IC 95% | | AUC Train | IC 95% Train | | AUC Test | IC 95% Test | |
|---|---|---|---|---|---|---|---|---|---|
| IFIT1 ISG15 SLC1A2 IL1R2 OLAH RETN | 0,861 | 0,852 | 0,871 | 0,907 | 0,849 | 0,965 | 0,948 | 0,899 | 0,997 |
| ISG15 HERC6 SLC1A2 IL1R2 OLAH MMP8 | 0,861 | 0,852 | 0,870 | 0,904 | 0,848 | 0,961 | 0,940 | 0,885 | 0,996 |
| RSAD2 OAS1 IFI44L ISG15 OLAH FAM20A | 0,861 | 0,851 | 0,872 | 0,894 | 0,823 | 0,965 | 0,945 | 0,885 | 1 |
| RSAD2 IFIT1 HERC6 SLC1A2 IL1R2 MMP8 | 0,861 | 0,852 | 0,870 | 0,903 | 0,846 | 0,960 | 0,950 | 0,901 | 0,999 |
| IFI27 OAS1 IFIT1 IFI44L SLC1A2 FAM20A | 0,861 | 0,850 | 0,872 | 0,899 | 0,833 | 0,964 | 0,942 | 0,865 | 1 |
| OAS1 ISG15 HERC6 FAM20A RETN MMP8 | 0,861 | 0,851 | 0,871 | 0,899 | 0,834 | 0,964 | 0,939 | 0,863 | 1 |
| RSAD2 OAS1 HERC6 IL1R2 OLAH MMP8 | 0,861 | 0,852 | 0,871 | 0,898 | 0,838 | 0,958 | 0,946 | 0,895 | 0,997 |
| IFIT1 IFI44L HERC6 SLC1A2 IL1R2 OLAH | 0,861 | 0,851 | 0,871 | 0,907 | 0,850 | 0,963 | 0,943 | 0,890 | 0,997 |
| OAS1 IFIT1 HERC6 SLC1A2 IL1R2 RETN | 0,861 | 0,851 | 0,871 | 0,901 | 0,843 | 0,960 | 0,939 | 0,880 | 0,999 |
| IFIT1 ISG15 HERC6 SLC1A2 OLAH MMP8 | 0,861 | 0,851 | 0,871 | 0,902 | 0,844 | 0,960 | 0,943 | 0,891 | 0,996 |
| IFI27 IFIT1 ISG15 SLC1A2 FAM20A MMP8 | 0,861 | 0,850 | 0,872 | 0,896 | 0,831 | 0,961 | 0,933 | 0,851 | 1 |
| RSAD2 OAS1 HERC6 SLC1A2 OLAH MMP8 | 0,861 | 0,851 | 0,871 | 0,902 | 0,844 | 0,960 | 0,942 | 0,888 | 0,997 |
| RSAD2 OAS1 IFIT1 ISG15 OLAH FAM20A | 0,861 | 0,851 | 0,871 | 0,893 | 0,822 | 0,964 | 0,949 | 0,886 | 1 |
| IFI27 IFIT1 ISG15 HERC6 SLC1A2 MMP8 | 0,861 | 0,852 | 0,870 | 0,899 | 0,842 | 0,957 | 0,899 | 0,815 | 0,982 |
| RSAD2 IFI44L SIGLEC1 HERC6 OLAH MMP8 | 0,861 | 0,851 | 0,871 | 0,897 | 0,833 | 0,960 | 0,949 | 0,902 | 0,996 |
| IFIT1 IFI44L HERC6 SLC1A2 FAM20A RETN | 0,861 | 0,851 | 0,871 | 0,900 | 0,838 | 0,963 | 0,926 | 0,844 | 1 |
| OAS1 IFIT1 ISG15 HERC6 SLC1A2 OLAH | 0,861 | 0,851 | 0,871 | 0,903 | 0,845 | 0,960 | 0,945 | 0,893 | 0,996 |
| RSAD2 IFIT1 HERC6 IL1R2 RETN MMP8 | 0,861 | 0,851 | 0,871 | 0,894 | 0,826 | 0,963 | 0,959 | 0,918 | 1,000 |
| RSAD2 IFI27 IFI44L HERC6 SLC1A2 MMP8 | 0,861 | 0,852 | 0,870 | 0,899 | 0,842 | 0,957 | 0,902 | 0,823 | 0,982 |
| IFIT1 IFI44L HERC6 FAM20A RETN MMP8 | 0,861 | 0,851 | 0,871 | 0,898 | 0,830 | 0,966 | 0,932 | 0,852 | 1 |
| IFIT1 IFI44L HERC6 IL1R2 RETN MMP8 | 0,861 | 0,851 | 0,871 | 0,894 | 0,826 | 0,962 | 0,959 | 0,918 | 1,000 |
| OAS1 IFI44L SIGLEC1 OLAH RETN MMP8 | 0,861 | 0,851 | 0,871 | 0,892 | 0,822 | 0,963 | 0,965 | 0,928 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 HERC6 SLC1A2 OLAH RETN | 0,861 | 0,851 | 0,871 | 0,901 | 0,841 | 0,960 | 0,939 | 0,880 | 0,998 |
| RSAD2 OAS1 HERC6 IL1R2 OLAH RETN | 0,861 | 0,851 | 0,871 | 0,898 | 0,834 | 0,962 | 0,947 | 0,897 | 0,996 |
| OAS1 IFIT1 SLC1A2 IL1R2 OLAH RETN | 0,861 | 0,851 | 0,871 | 0,908 | 0,850 | 0,965 | 0,946 | 0,895 | 0,996 |
| RSAD2 OAS1 IFIT1 SLC1A2 IL1R2 OLAH | 0,861 | 0,851 | 0,871 | 0,905 | 0,847 | 0,963 | 0,948 | 0,899 | 0,997 |
| IFIT1 ISG15 HERC6 SLC1A2 IL1R2 MMP8 | 0,861 | 0,851 | 0,870 | 0,902 | 0,845 | 0,959 | 0,953 | 0,906 | 1 |
| RSAD2 OAS1 IFIT1 SIGLEC1 ISG15 OLAH | 0,861 | 0,851 | 0,870 | 0,894 | 0,826 | 0,962 | 0,945 | 0,893 | 0,996 |
| OAS1 ISG15 HERC6 SLC1A2 OLAH MMP8 | 0,861 | 0,851 | 0,871 | 0,902 | 0,845 | 0,960 | 0,940 | 0,886 | 0,995 |
| OAS1 ISG15 HERC6 OLAH RETN MMP8 | 0,861 | 0,850 | 0,871 | 0,895 | 0,828 | 0,962 | 0,961 | 0,921 | 1 |
| RSAD2 IFIT1 ISG15 HERC6 IL1R2 MMP8 | 0,861 | 0,851 | 0,870 | 0,897 | 0,837 | 0,957 | 0,947 | 0,897 | 0,996 |
| RSAD2 IFI44L SIGLEC1 HERC6 SLC1A2 RETN | 0,861 | 0,852 | 0,870 | 0,902 | 0,840 | 0,964 | 0,939 | 0,886 | 0,993 |
| HERC6 SLC1A2 IL1R2 OLAH RETN MMP8 | 0,861 | 0,851 | 0,870 | 0,904 | 0,846 | 0,962 | 0,939 | 0,881 | 0,997 |
| OAS1 IFIT1 ISG15 HERC6 OLAH RETN | 0,861 | 0,851 | 0,871 | 0,896 | 0,831 | 0,961 | 0,951 | 0,905 | 0,997 |
| IFIT1 ISG15 HERC6 IL1R2 OLAH RETN | 0,861 | 0,851 | 0,870 | 0,900 | 0,836 | 0,963 | 0,951 | 0,905 | 0,997 |
| RSAD2 IFIT1 IFI44L HERC6 FAM20A RETN | 0,861 | 0,851 | 0,871 | 0,898 | 0,832 | 0,964 | 0,925 | 0,844 | 1 |
| RSAD2 IFI44L ISG15 HERC6 SLC1A2 FAM20A | 0,861 | 0,850 | 0,871 | 0,899 | 0,839 | 0,959 | 0,925 | 0,842 | 1 |
| RSAD2 IFIT1 IFI44L SLC1A2 OLAH MMP8 | 0,861 | 0,851 | 0,870 | 0,905 | 0,847 | 0,964 | 0,937 | 0,883 | 0,991 |
| RSAD2 OAS1 HERC6 IL1R2 RETN MMP8 | 0,861 | 0,851 | 0,870 | 0,894 | 0,828 | 0,961 | 0,943 | 0,889 | 0,998 |
| OAS1 IFIT1 IFI44L OLAH FAM20A MMP8 | 0,861 | 0,850 | 0,871 | 0,893 | 0,822 | 0,963 | 0,951 | 0,895 | 1 |
| IFI27 OAS1 IFI44L SIGLEC1 ISG15 MMP8 | 0,860 | 0,851 | 0,870 | 0,890 | 0,824 | 0,957 | 0,917 | 0,845 | 0,990 |
| RSAD2 OAS1 IFIT1 HERC6 IL1R2 OLAH | 0,860 | 0,851 | 0,870 | 0,899 | 0,837 | 0,960 | 0,946 | 0,896 | 0,995 |
| RSAD2 OAS1 IFIT1 SLC1A2 OLAH MMP8 | 0,860 | 0,851 | 0,870 | 0,904 | 0,846 | 0,962 | 0,946 | 0,897 | 0,995 |
| OAS1 IFIT1 ISG15 OLAH FAM20A RETN | 0,860 | 0,850 | 0,871 | 0,893 | 0,822 | 0,964 | 0,952 | 0,894 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 IFI44L SLC1A2 OLAH MMP8 | 0,860 | 0,851 | 0,870 | 0,905 | 0,848 | 0,963 | 0,935 | 0,879 | 0,991 |
| RSAD2 HERC6 IL1R2 OLAH RETN MMP8 | 0,860 | 0,850 | 0,871 | 0,899 | 0,835 | 0,962 | 0,959 | 0,918 | 0,999 |
| OAS1 IFIT1 HERC6 IL1R2 OLAH MMP8 | 0,860 | 0,851 | 0,870 | 0,899 | 0,838 | 0,959 | 0,954 | 0,911 | 0,998 |
| RSAD2 OAS1 IFIT1 IFI44L HERC6 IL1R2 | 0,860 | 0,851 | 0,870 | 0,895 | 0,833 | 0,958 | 0,946 | 0,896 | 0,995 |
| IFIT1 ISG15 HERC6 SLC1A2 OLAH RETN | 0,860 | 0,850 | 0,870 | 0,904 | 0,845 | 0,963 | 0,943 | 0,888 | 0,999 |
| ISG15 SLC1A2 IL1R2 FAM20A RETN MMP8 | 0,860 | 0,851 | 0,869 | 0,906 | 0,851 | 0,961 | 0,916 | 0,826 | 1 |
| OAS1 IFIT1 IFI44L SLC1A2 OLAH RETN | 0,860 | 0,850 | 0,871 | 0,909 | 0,851 | 0,967 | 0,937 | 0,882 | 0,991 |
| RSAD2 IFIT1 HERC6 SLC1A2 OLAH MMP8 | 0,860 | 0,851 | 0,870 | 0,901 | 0,843 | 0,960 | 0,943 | 0,891 | 0,996 |
| IFIT1 HERC6 SLC1A2 IL1R2 RETN MMP8 | 0,860 | 0,851 | 0,870 | 0,903 | 0,845 | 0,962 | 0,950 | 0,899 | 1 |
| RSAD2 IFI44L IL1R2 OLAH FAM20A RETN | 0,860 | 0,850 | 0,870 | 0,897 | 0,832 | 0,963 | 0,949 | 0,885 | 1 |
| IFI27 OAS1 IFIT1 SIGLEC1 ISG15 SLC1A2 | 0,860 | 0,851 | 0,869 | 0,897 | 0,837 | 0,956 | 0,954 | 0,905 | 1 |
| RSAD2 OAS1 IFIT1 IFI44L OLAH FAM20A | 0,860 | 0,851 | 0,870 | 0,894 | 0,824 | 0,964 | 0,943 | 0,885 | 1 |
| RSAD2 OAS1 ISG15 HERC6 SLC1A2 OLAH | 0,860 | 0,850 | 0,870 | 0,902 | 0,845 | 0,959 | 0,942 | 0,889 | 0,996 |
| OAS1 IFI44L HERC6 IL1R2 RETN MMP8 | 0,860 | 0,851 | 0,870 | 0,895 | 0,829 | 0,961 | 0,950 | 0,903 | 0,997 |
| RSAD2 IFI27 OAS1 IFIT1 ISG15 FAM20A | 0,860 | 0,850 | 0,871 | 0,888 | 0,817 | 0,959 | 0,941 | 0,862 | 1 |
| ISG15 IL1R2 OLAH FAM20A RETN MMP8 | 0,860 | 0,851 | 0,870 | 0,901 | 0,842 | 0,961 | 0,935 | 0,864 | 1 |
| RSAD2 OAS1 IFI44L HERC6 FAM20A RETN | 0,860 | 0,850 | 0,870 | 0,895 | 0,828 | 0,962 | 0,927 | 0,847 | 1 |
| RSAD2 OAS1 IFIT1 HERC6 SLC1A2 OLAH | 0,860 | 0,850 | 0,870 | 0,903 | 0,845 | 0,960 | 0,949 | 0,898 | 1,000 |
| IFI44L ISG15 HERC6 IL1R2 OLAH MMP8 | 0,860 | 0,851 | 0,870 | 0,899 | 0,840 | 0,958 | 0,953 | 0,909 | 0,998 |
| RSAD2 IFI27 OAS1 IFI44L SIGLEC1 MMP8 | 0,860 | 0,851 | 0,870 | 0,890 | 0,823 | 0,957 | 0,924 | 0,854 | 0,994 |
| IFIT1 IFI44L ISG15 HERC6 OLAH RETN | 0,860 | 0,851 | 0,870 | 0,895 | 0,827 | 0,962 | 0,942 | 0,891 | 0,994 |
| RSAD2 OAS1 ISG15 OLAH FAM20A RETN | 0,860 | 0,850 | 0,870 | 0,894 | 0,823 | 0,965 | 0,951 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI27 ISG15 HERC6 SLC1A2 MMP8 | 0,860 | 0,851 | 0,869 | 0,901 | 0,845 | 0,957 | 0,902 | 0,823 | 0,982 |
| IFIT1 ISG15 HERC6 IL1R2 OLAH MMP8 | 0,860 | 0,851 | 0,870 | 0,900 | 0,840 | 0,959 | 0,959 | 0,918 | 1,000 |
| OAS1 IFIT1 IFI44L ISG15 SLC1A2 OLAH | 0,860 | 0,850 | 0,870 | 0,909 | 0,852 | 0,966 | 0,936 | 0,881 | 0,990 |
| OAS1 ISG15 HERC6 IL1R2 OLAH MMP8 | 0,860 | 0,850 | 0,870 | 0,900 | 0,841 | 0,959 | 0,947 | 0,898 | 0,995 |
| RSAD2 IFIT1 SIGLEC1 ISG15 OLAH RETN | 0,860 | 0,850 | 0,870 | 0,895 | 0,826 | 0,964 | 0,949 | 0,897 | 1 |
| IFIT1 IFI44L SIGLEC1 ISG15 OLAH RETN | 0,860 | 0,851 | 0,870 | 0,891 | 0,822 | 0,960 | 0,941 | 0,884 | 0,998 |
| IFIT1 IFI44L HERC6 OLAH RETN MMP8 | 0,860 | 0,850 | 0,870 | 0,892 | 0,823 | 0,962 | 0,953 | 0,909 | 0,997 |
| RSAD2 OAS1 ISG15 HERC6 IL1R2 OLAH | 0,860 | 0,850 | 0,870 | 0,898 | 0,838 | 0,959 | 0,945 | 0,895 | 0,995 |
| IFI44L ISG15 HERC6 SLC1A2 IL1R2 RETN | 0,860 | 0,850 | 0,870 | 0,906 | 0,849 | 0,962 | 0,942 | 0,882 | 1 |
| OAS1 IFIT1 IFI44L ISG15 HERC6 IL1R2 | 0,860 | 0,851 | 0,869 | 0,897 | 0,838 | 0,956 | 0,947 | 0,897 | 0,997 |
| ISG15 HERC6 SLC1A2 FAM20A RETN MMP8 | 0,860 | 0,850 | 0,870 | 0,900 | 0,836 | 0,965 | 0,940 | 0,862 | 1 |
| IFIT1 ISG15 HERC6 SLC1A2 IL1R2 RETN | 0,860 | 0,850 | 0,870 | 0,905 | 0,848 | 0,962 | 0,943 | 0,887 | 1 |
| RSAD2 IFI44L SLC1A2 IL1R2 OLAH MMP8 | 0,860 | 0,851 | 0,869 | 0,904 | 0,848 | 0,961 | 0,942 | 0,888 | 0,997 |
| IFIT1 IFI44L HERC6 SLC1A2 IL1R2 MMP8 | 0,860 | 0,851 | 0,869 | 0,903 | 0,846 | 0,960 | 0,951 | 0,902 | 1,000 |
| RSAD2 IFI44L ISG15 IL1R2 FAM20A RETN | 0,860 | 0,850 | 0,870 | 0,897 | 0,833 | 0,962 | 0,942 | 0,871 | 1 |
| RSAD2 IFIT1 HERC6 SLC1A2 OLAH RETN | 0,860 | 0,850 | 0,870 | 0,904 | 0,845 | 0,963 | 0,937 | 0,877 | 0,997 |
| RSAD2 ISG15 HERC6 IL1R2 OLAH MMP8 | 0,860 | 0,850 | 0,869 | 0,899 | 0,840 | 0,958 | 0,949 | 0,902 | 0,996 |
| IFIT1 IFI44L HERC6 IL1R2 OLAH MMP8 | 0,860 | 0,850 | 0,869 | 0,899 | 0,838 | 0,959 | 0,955 | 0,912 | 0,999 |
| RSAD2 IFIT1 IFI44L SIGLEC1 OLAH RETN | 0,860 | 0,850 | 0,869 | 0,895 | 0,825 | 0,964 | 0,948 | 0,896 | 1,000 |
| RSAD2 IFIT1 IFI44L ISG15 HERC6 IL1R2 | 0,860 | 0,851 | 0,869 | 0,898 | 0,838 | 0,957 | 0,947 | 0,897 | 0,997 |
| RSAD2 IFI27 OAS1 SLC1A2 FAM20A MMP8 | 0,860 | 0,849 | 0,870 | 0,895 | 0,830 | 0,960 | 0,928 | 0,844 | 1 |
| OAS1 IFI44L SIGLEC1 HERC6 RETN MMP8 | 0,860 | 0,850 | 0,869 | 0,889 | 0,818 | 0,961 | 0,949 | 0,901 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 ISG15 SLC1A2 FAM20A MMP8 | 0,860 | 0,849 | 0,870 | 0,895 | 0,829 | 0,960 | 0,929 | 0,846 | 1 |
| RSAD2 OAS1 ISG15 HERC6 OLAH RETN | 0,860 | 0,850 | 0,870 | 0,895 | 0,830 | 0,960 | 0,953 | 0,909 | 0,998 |
| RSAD2 OAS1 IFI44L HERC6 IL1R2 OLAH | 0,860 | 0,850 | 0,869 | 0,899 | 0,838 | 0,959 | 0,946 | 0,895 | 0,996 |
| OAS1 ISG15 HERC6 SLC1A2 IL1R2 MMP8 | 0,860 | 0,850 | 0,869 | 0,902 | 0,847 | 0,957 | 0,933 | 0,867 | 0,998 |
| RSAD2 OAS1 IFIT1 OLAH FAM20A RETN | 0,860 | 0,850 | 0,870 | 0,894 | 0,823 | 0,964 | 0,948 | 0,885 | 1 |
| RSAD2 ISG15 HERC6 SLC1A2 IL1R2 RETN | 0,860 | 0,850 | 0,869 | 0,902 | 0,844 | 0,959 | 0,940 | 0,878 | 1 |
| RSAD2 IFIT1 ISG15 SLC1A2 IL1R2 OLAH | 0,860 | 0,850 | 0,869 | 0,905 | 0,847 | 0,964 | 0,947 | 0,897 | 0,997 |
| RSAD2 OAS1 IFI44L HERC6 IL1R2 RETN | 0,860 | 0,850 | 0,869 | 0,893 | 0,827 | 0,960 | 0,945 | 0,894 | 0,995 |
| OAS1 IFIT1 HERC6 IL1R2 OLAH RETN | 0,860 | 0,849 | 0,870 | 0,897 | 0,833 | 0,962 | 0,947 | 0,897 | 0,996 |
| RSAD2 OAS1 ISG15 HERC6 IL1R2 MMP8 | 0,860 | 0,850 | 0,869 | 0,898 | 0,839 | 0,956 | 0,946 | 0,895 | 0,996 |
| RSAD2 IFI27 SIGLEC1 ISG15 HERC6 SLC1A2 | 0,860 | 0,851 | 0,868 | 0,898 | 0,841 | 0,954 | 0,942 | 0,889 | 0,996 |
| IFI44L ISG15 HERC6 FAM20A RETN MMP8 | 0,860 | 0,849 | 0,870 | 0,895 | 0,826 | 0,965 | 0,947 | 0,874 | 1 |
| RSAD2 OAS1 SLC1A2 IL1R2 OLAH MMP8 | 0,859 | 0,850 | 0,869 | 0,904 | 0,846 | 0,961 | 0,939 | 0,882 | 0,996 |
| RSAD2 IFIT1 IFI44L HERC6 IL1R2 MMP8 | 0,859 | 0,850 | 0,869 | 0,895 | 0,832 | 0,958 | 0,950 | 0,903 | 0,997 |
| RSAD2 IFIT1 ISG15 HERC6 IL1R2 OLAH | 0,859 | 0,850 | 0,869 | 0,900 | 0,839 | 0,960 | 0,950 | 0,904 | 0,996 |
| RSAD2 IFI27 ISG15 SLC1A2 FAM20A MMP8 | 0,859 | 0,849 | 0,870 | 0,895 | 0,830 | 0,960 | 0,932 | 0,850 | 1 |
| OAS1 IFIT1 ISG15 SLC1A2 OLAH MMP8 | 0,859 | 0,850 | 0,869 | 0,903 | 0,845 | 0,961 | 0,941 | 0,889 | 0,993 |
| OAS1 IFI44L ISG15 HERC6 OLAH RETN | 0,859 | 0,849 | 0,870 | 0,895 | 0,829 | 0,960 | 0,958 | 0,916 | 1,000 |
| OAS1 IFI44L ISG15 SLC1A2 OLAH RETN | 0,859 | 0,849 | 0,869 | 0,909 | 0,851 | 0,967 | 0,935 | 0,879 | 0,990 |
| RSAD2 IFI27 OAS1 IFIT1 SIGLEC1 MMP8 | 0,859 | 0,850 | 0,869 | 0,889 | 0,822 | 0,956 | 0,916 | 0,841 | 0,991 |
| RSAD2 ISG15 HERC6 SLC1A2 OLAH MMP8 | 0,859 | 0,850 | 0,869 | 0,901 | 0,843 | 0,960 | 0,940 | 0,885 | 0,995 |
| IFIT1 ISG15 HERC6 OLAH RETN MMP8 | 0,859 | 0,849 | 0,870 | 0,893 | 0,825 | 0,961 | 0,955 | 0,913 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 SLC1A2 FAM20A MMP8 | 0,859 | 0,849 | 0,870 | 0,897 | 0,832 | 0,961 | 0,933 | 0,852 | 1 |
| OAS1 IFI44L ISG15 SLC1A2 OLAH MMP8 | 0,859 | 0,850 | 0,869 | 0,905 | 0,847 | 0,963 | 0,936 | 0,881 | 0,991 |
| RSAD2 IFIT1 IFI44L SIGLEC1 OLAH MMP8 | 0,859 | 0,850 | 0,869 | 0,894 | 0,827 | 0,961 | 0,953 | 0,907 | 0,999 |
| OAS1 IFIT1 ISG15 SLC1A2 OLAH RETN | 0,859 | 0,849 | 0,869 | 0,907 | 0,849 | 0,965 | 0,945 | 0,895 | 0,994 |
| RSAD2 OAS1 IFIT1 IFI44L SLC1A2 OLAH | 0,859 | 0,850 | 0,869 | 0,909 | 0,852 | 0,966 | 0,935 | 0,880 | 0,990 |
| IFIT1 IFI44L ISG15 HERC6 IL1R2 OLAH | 0,859 | 0,850 | 0,869 | 0,899 | 0,838 | 0,959 | 0,949 | 0,902 | 0,996 |
| RSAD2 IFI27 IFIT1 HERC6 SLC1A2 MMP8 | 0,859 | 0,850 | 0,869 | 0,900 | 0,843 | 0,957 | 0,900 | 0,817 | 0,983 |
| RSAD2 ISG15 HERC6 OLAH RETN MMP8 | 0,859 | 0,849 | 0,870 | 0,895 | 0,828 | 0,962 | 0,957 | 0,915 | 0,998 |
| RSAD2 IFI27 OAS1 SIGLEC1 ISG15 HERC6 | 0,859 | 0,850 | 0,869 | 0,895 | 0,840 | 0,951 | 0,921 | 0,858 | 0,984 |
| RSAD2 IFI44L SIGLEC1 ISG15 HERC6 OLAH | 0,859 | 0,850 | 0,869 | 0,899 | 0,836 | 0,961 | 0,950 | 0,904 | 0,996 |
| OAS1 IFI44L HERC6 SLC1A2 IL1R2 RETN | 0,859 | 0,849 | 0,869 | 0,902 | 0,844 | 0,960 | 0,936 | 0,873 | 0,999 |
| RSAD2 OAS1 HERC6 OLAH RETN MMP8 | 0,859 | 0,849 | 0,869 | 0,890 | 0,821 | 0,960 | 0,954 | 0,911 | 0,998 |
| OAS1 IFIT1 ISG15 HERC6 IL1R2 OLAH | 0,859 | 0,849 | 0,869 | 0,899 | 0,838 | 0,959 | 0,952 | 0,907 | 0,997 |
| RSAD2 IFI44L HERC6 SLC1A2 FAM20A RETN | 0,859 | 0,849 | 0,869 | 0,901 | 0,838 | 0,963 | 0,929 | 0,850 | 1 |
| OAS1 IFI44L SLC1A2 OLAH RETN MMP8 | 0,859 | 0,849 | 0,869 | 0,905 | 0,845 | 0,966 | 0,941 | 0,889 | 0,993 |
| RSAD2 IFI27 IFIT1 IFI44L SIGLEC1 MMP8 | 0,859 | 0,850 | 0,869 | 0,891 | 0,825 | 0,957 | 0,914 | 0,840 | 0,989 |
| OAS1 IFIT1 IFI44L HERC6 OLAH RETN | 0,859 | 0,849 | 0,869 | 0,893 | 0,824 | 0,962 | 0,940 | 0,888 | 0,993 |
| RSAD2 IFIT1 ISG15 HERC6 SLC1A2 OLAH | 0,859 | 0,850 | 0,869 | 0,904 | 0,846 | 0,961 | 0,943 | 0,891 | 0,996 |
| RSAD2 ISG15 SLC1A2 IL1R2 OLAH MMP8 | 0,859 | 0,850 | 0,868 | 0,903 | 0,845 | 0,960 | 0,937 | 0,877 | 0,997 |
| OAS1 SIGLEC1 ISG15 OLAH RETN MMP8 | 0,859 | 0,849 | 0,869 | 0,892 | 0,821 | 0,962 | 0,963 | 0,922 | 1 |
| RSAD2 OAS1 IFI44L OLAH FAM20A RETN | 0,859 | 0,849 | 0,869 | 0,892 | 0,821 | 0,963 | 0,945 | 0,885 | 1 |
| OAS1 IFIT1 IFI44L HERC6 SLC1A2 IL1R2 | 0,859 | 0,849 | 0,869 | 0,903 | 0,847 | 0,960 | 0,949 | 0,899 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 IFI44L HERC6 IL1R2 RETN | 0,859 | 0,850 | 0,869 | 0,893 | 0,826 | 0,960 | 0,947 | 0,898 | 0,995 |
| RSAD2 IFI27 IFIT1 SLC1A2 FAM20A MMP8 | 0,859 | 0,849 | 0,869 | 0,896 | 0,832 | 0,961 | 0,933 | 0,851 | 1 |
| RSAD2 IFI44L ISG15 SLC1A2 IL1R2 OLAH | 0,859 | 0,850 | 0,869 | 0,906 | 0,849 | 0,963 | 0,941 | 0,887 | 0,995 |
| OAS1 IFIT1 IFI44L SLC1A2 IL1R2 OLAH | 0,859 | 0,849 | 0,869 | 0,910 | 0,854 | 0,966 | 0,945 | 0,893 | 0,996 |
| OAS1 IFI44L ISG15 SLC1A2 IL1R2 OLAH | 0,859 | 0,849 | 0,869 | 0,907 | 0,850 | 0,964 | 0,940 | 0,885 | 0,995 |
| RSAD2 OAS1 ISG15 HERC6 SLC1A2 IL1R2 | 0,859 | 0,849 | 0,869 | 0,900 | 0,844 | 0,956 | 0,942 | 0,879 | 1 |
| OAS1 IFI44L HERC6 IL1R2 OLAH RETN | 0,859 | 0,849 | 0,869 | 0,898 | 0,834 | 0,963 | 0,947 | 0,897 | 0,996 |
| IFIT1 IFI44L ISG15 HERC6 SLC1A2 IL1R2 | 0,859 | 0,850 | 0,868 | 0,902 | 0,845 | 0,959 | 0,950 | 0,900 | 1,000 |
| OAS1 IFIT1 SIGLEC1 HERC6 SLC1A2 MMP8 | 0,859 | 0,850 | 0,868 | 0,892 | 0,831 | 0,953 | 0,913 | 0,845 | 0,981 |
| RSAD2 OAS1 IFI44L SLC1A2 OLAH RETN | 0,859 | 0,849 | 0,869 | 0,909 | 0,851 | 0,967 | 0,936 | 0,881 | 0,991 |
| OAS 1 IFIT1 OLAH FAM20A RETN MMP8 | 0,859 | 0,849 | 0,869 | 0,890 | 0,818 | 0,962 | 0,958 | 0,904 | 1 |
| OAS1 IFIT1 HERC6 OLAH RETN MMP8 | 0,859 | 0,849 | 0,869 | 0,891 | 0,822 | 0,960 | 0,955 | 0,913 | 0,998 |
| RSAD2 OAS1 SLC1A2 IL1R2 OLAH RETN | 0,859 | 0,849 | 0,869 | 0,905 | 0,848 | 0,963 | 0,939 | 0,882 | 0,996 |
| RSAD2 IFI44L SLC1A2 IL1R2 OLAH RETN | 0,859 | 0,849 | 0,869 | 0,907 | 0,850 | 0,963 | 0,939 | 0,884 | 0,994 |
| RSAD2 IFIT1 SLC1A2 IL1R2 OLAH RETN | 0,859 | 0,849 | 0,868 | 0,907 | 0,849 | 0,964 | 0,946 | 0,895 | 0,996 |
| ISG15 HERC6 SLC1A2 IL1R2 RETN MMP8 | 0,859 | 0,849 | 0,868 | 0,902 | 0,845 | 0,959 | 0,942 | 0,881 | 1 |
| RSAD2 IFI44L IL1R2 FAM20A RETN MMP8 | 0,859 | 0,849 | 0,869 | 0,902 | 0,839 | 0,964 | 0,940 | 0,862 | 1 |
| RSAD2 IFI44L ISG15 SLC1A2 OLAH MMP8 | 0,859 | 0,849 | 0,868 | 0,905 | 0,848 | 0,963 | 0,937 | 0,882 | 0,991 |
| IFI44L HERC6 SLC1A2 IL1R2 RETN MMP8 | 0,859 | 0,849 | 0,869 | 0,902 | 0,844 | 0,960 | 0,943 | 0,885 | 1 |
| RSAD2 OAS1 ISG15 SLC1A2 IL1R2 OLAH | 0,859 | 0,849 | 0,868 | 0,904 | 0,847 | 0,962 | 0,938 | 0,880 | 0,996 |
| IFIT1 IFI44L HERC6 SLC1A2 IL1R2 RETN | 0,859 | 0,849 | 0,868 | 0,901 | 0,842 | 0,960 | 0,939 | 0,881 | 0,998 |
| OAS1 IFIT1 IFI44L HERC6 IL1R2 OLAH | 0,859 | 0,849 | 0,868 | 0,898 | 0,837 | 0,959 | 0,946 | 0,896 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 HERC6 SLC1A2 IL1R2 RETN | 0,859 | 0,849 | 0,868 | 0,901 | 0,842 | 0,960 | 0,942 | 0,886 | 0,999 |
| RSAD2 IFI44L ISG15 SLC1A2 OLAH RETN | 0,859 | 0,849 | 0,869 | 0,909 | 0,851 | 0,967 | 0,934 | 0,877 | 0,990 |
| RSAD2 OAS1 IFI44L SLC1A2 IL1R2 OLAH | 0,859 | 0,849 | 0,869 | 0,906 | 0,849 | 0,963 | 0,940 | 0,885 | 0,995 |
| OAS1 IFI44L HERC6 SLC1A2 FAM20A RETN | 0,859 | 0,849 | 0,869 | 0,897 | 0,833 | 0,961 | 0,939 | 0,862 | 1 |
| ISG15 SLC1A2 OLAH FAM20A RETN MMP8 | 0,859 | 0,849 | 0,869 | 0,904 | 0,848 | 0,961 | 0,923 | 0,847 | 0,999 |
| IFI44L ISG15 OLAH FAM20A RETN MMP8 | 0,859 | 0,849 | 0,869 | 0,896 | 0,828 | 0,964 | 0,962 | 0,920 | 1 |
| IFI27 IFI44L ISG15 HERC6 SLC1A2 MMP8 | 0,859 | 0,850 | 0,868 | 0,899 | 0,842 | 0,957 | 0,903 | 0,821 | 0,986 |
| IFIT1 IFI44L HERC6 IL1R2 OLAH RETN | 0,859 | 0,849 | 0,868 | 0,898 | 0,833 | 0,963 | 0,948 | 0,899 | 0,996 |
| RSAD2 SLC1A2 IL1R2 OLAH RETN MMP8 | 0,859 | 0,849 | 0,868 | 0,905 | 0,848 | 0,963 | 0,941 | 0,885 | 0,998 |
| OAS1 IFI44L HERC6 IL1R2 OLAH MMP8 | 0,859 | 0,849 | 0,868 | 0,900 | 0,840 | 0,959 | 0,951 | 0,904 | 0,998 |
| RSAD2 IFIT1 IFI44L SLC1A2 OLAH RETN | 0,859 | 0,849 | 0,868 | 0,908 | 0,851 | 0,966 | 0,937 | 0,883 | 0,991 |
| RSAD2 IFI44L SLC1A2 OLAH RETN MMP8 | 0,859 | 0,849 | 0,868 | 0,905 | 0,845 | 0,966 | 0,942 | 0,891 | 0,994 |
| OAS1 IFI44L ISG15 HERC6 IL1R2 MMP8 | 0,859 | 0,849 | 0,868 | 0,896 | 0,838 | 0,954 | 0,945 | 0,894 | 0,995 |
| OAS1 IFI44L SLC1A2 IL1R2 OLAH MMP8 | 0,859 | 0,849 | 0,868 | 0,905 | 0,849 | 0,962 | 0,941 | 0,888 | 0,995 |
| IFI27 OAS1 IFIT1 SIGLEC1 ISG15 HERC6 | 0,859 | 0,849 | 0,868 | 0,898 | 0,843 | 0,953 | 0,930 | 0,873 | 0,988 |
| RSAD2 IFIT1 IFI44L SLC1A2 IL1R2 OLAH | 0,859 | 0,849 | 0,868 | 0,909 | 0,852 | 0,966 | 0,946 | 0,895 | 0,996 |
| RSAD2 OAS1 HERC6 SLC1A2 IL1R2 RETN | 0,859 | 0,849 | 0,868 | 0,899 | 0,840 | 0,958 | 0,934 | 0,865 | 1 |
| RSAD2 OAS1 IFI44L HERC6 IL1R2 MMP8 | 0,859 | 0,849 | 0,868 | 0,894 | 0,833 | 0,955 | 0,942 | 0,891 | 0,994 |
| OAS1 IFI44L SLC1A2 IL1R2 OLAH RETN | 0,859 | 0,848 | 0,869 | 0,908 | 0,851 | 0,965 | 0,938 | 0,883 | 0,993 |
| RSAD2 IFI27 IFI44L SIGLEC1 HERC6 SLC1A2 | 0,858 | 0,850 | 0,867 | 0,899 | 0,843 | 0,954 | 0,940 | 0,885 | 0,995 |
| RSAD2 OAS1 IFIT1 HERC6 FAM20A RETN | 0,858 | 0,848 | 0,869 | 0,887 | 0,816 | 0,958 | 0,934 | 0,855 | 1 |
| RSAD2 IFI44L ISG15 HERC6 SLC1A2 IL1R2 | 0,858 | 0,849 | 0,868 | 0,902 | 0,846 | 0,957 | 0,940 | 0,882 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L ISG15 HERC6 IL1R2 OLAH | 0,858 | 0,849 | 0,868 | 0,900 | 0,841 | 0,959 | 0,946 | 0,896 | 0,995 |
| RSAD2 IFIT1 HERC6 IL1R2 OLAH MMP8 | 0,858 | 0,848 | 0,868 | 0,899 | 0,838 | 0,959 | 0,957 | 0,914 | 0,999 |
| IFI44L ISG15 HERC6 OLAH RETN MMP8 | 0,858 | 0,848 | 0,869 | 0,892 | 0,821 | 0,962 | 0,961 | 0,922 | 1,000 |
| IFI27 OAS1 IFI44L SIGLEC1 ISG15 SLC1A2 | 0,858 | 0,849 | 0,867 | 0,896 | 0,837 | 0,956 | 0,953 | 0,903 | 1 |
| OAS1 IFIT1 ISG15 OLAH FAM20A MMP8 | 0,858 | 0,848 | 0,869 | 0,893 | 0,823 | 0,964 | 0,952 | 0,892 | 1 |
| RSAD2 IFI27 OAS1 SIGLEC1 ISG15 MMP8 | 0,858 | 0,848 | 0,868 | 0,890 | 0,824 | 0,956 | 0,921 | 0,848 | 0,993 |
| OAS1 IFIT1 ISG15 SLC1A2 IL1R2 RETN | 0,858 | 0,849 | 0,868 | 0,899 | 0,838 | 0,959 | 0,949 | 0,900 | 0,998 |
| RSAD2 IFIT1 SIGLEC1 ISG15 OLAH MMP8 | 0,858 | 0,849 | 0,868 | 0,893 | 0,826 | 0,961 | 0,949 | 0,900 | 0,998 |
| RSAD2 ISG15 SLC1A2 IL1R2 OLAH RETN | 0,858 | 0,849 | 0,868 | 0,906 | 0,849 | 0,963 | 0,939 | 0,881 | 0,998 |
| RSAD2 IFI44L ISG15 HERC6 OLAH RETN | 0,858 | 0,848 | 0,868 | 0,897 | 0,832 | 0,962 | 0,947 | 0,899 | 0,995 |
| RSAD2 OAS1 IFI44L ISG15 SLC1A2 OLAH | 0,858 | 0,848 | 0,868 | 0,907 | 0,850 | 0,964 | 0,934 | 0,878 | 0,990 |
| RSAD2 OAS1 SIGLEC1 ISG15 OLAH MMP8 | 0,858 | 0,848 | 0,868 | 0,891 | 0,823 | 0,959 | 0,947 | 0,897 | 0,996 |
| RSAD2 IFI27 IFIT1 SIGLEC1 HERC6 SLC1A2 | 0,858 | 0,849 | 0,867 | 0,900 | 0,845 | 0,955 | 0,942 | 0,889 | 0,995 |
| IFI27 OAS1 IFI44L SIGLEC1 ISG15 HERC6 | 0,858 | 0,849 | 0,867 | 0,897 | 0,842 | 0,953 | 0,932 | 0,874 | 0,989 |
| RSAD2 IFI44L HERC6 IL1R2 RETN MMP8 | 0,858 | 0,848 | 0,868 | 0,894 | 0,827 | 0,961 | 0,950 | 0,902 | 0,998 |
| RSAD2 IFI44L HERC6 IL1R2 OLAH RETN | 0,858 | 0,848 | 0,868 | 0,899 | 0,834 | 0,963 | 0,948 | 0,899 | 0,996 |
| OAS1 IFIT1 IFI44L ISG15 OLAH FAM20A | 0,858 | 0,848 | 0,868 | 0,890 | 0,818 | 0,963 | 0,950 | 0,895 | 1 |
| RSAD2 IFI44L HERC6 IL1R2 OLAH MMP8 | 0,858 | 0,848 | 0,868 | 0,899 | 0,839 | 0,959 | 0,952 | 0,906 | 0,999 |
| RSAD2 IFIT1 ISG15 HERC6 OLAH RETN | 0,858 | 0,848 | 0,868 | 0,894 | 0,828 | 0,960 | 0,942 | 0,892 | 0,993 |
| RSAD2 ISG15 HERC6 SLC1A2 IL1R2 MMP8 | 0,858 | 0,849 | 0,867 | 0,900 | 0,843 | 0,957 | 0,936 | 0,872 | 1,000 |
| RSAD2 IFIT1 IFI44L IL1R2 FAM20A RETN | 0,858 | 0,848 | 0,868 | 0,895 | 0,830 | 0,960 | 0,946 | 0,874 | 1 |
| OAS1 IFIT1 SLC1A2 OLAH RETN MMP8 | 0,858 | 0,848 | 0,868 | 0,899 | 0,838 | 0,961 | 0,954 | 0,911 | 0,998 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 IFI44L ISG15 SLC1A2 OLAH | 0,858 | 0,849 | 0,867 | 0,907 | 0,850 | 0,965 | 0,935 | 0,880 | 0,990 |
| OAS1 IFI44L ISG15 HERC6 IL1R2 OLAH | 0,858 | 0,848 | 0,868 | 0,900 | 0,841 | 0,959 | 0,948 | 0,900 | 0,996 |
| RSAD2 OAS1 SIGLEC1 ISG15 OLAH RETN | 0,858 | 0,848 | 0,868 | 0,892 | 0,821 | 0,963 | 0,949 | 0,899 | 0,999 |
| RSAD2 OAS1 IFI44L ISG15 HERC6 IL1R2 | 0,858 | 0,848 | 0,867 | 0,897 | 0,840 | 0,955 | 0,947 | 0,896 | 0,997 |
| RSAD2 OAS1 IFI44L SIGLEC1 OLAH MMP8 | 0,858 | 0,848 | 0,868 | 0,893 | 0,826 | 0,961 | 0,948 | 0,899 | 0,997 |
| RSAD2 IFI27 IFI44L SIGLEC1 ISG15 MMP8 | 0,858 | 0,848 | 0,868 | 0,889 | 0,822 | 0,956 | 0,916 | 0,844 | 0,988 |
| RSAD2 OAS1 IFIT1 SLC1A2 IL1R2 MMP8 | 0,858 | 0,849 | 0,867 | 0,902 | 0,844 | 0,960 | 0,954 | 0,909 | 1,000 |
| RSAD2 IFI44L ISG15 HERC6 IL1R2 MMP8 | 0,858 | 0,849 | 0,867 | 0,895 | 0,836 | 0,954 | 0,942 | 0,891 | 0,994 |
| RSAD2 OAS1 IFI44L ISG15 HERC6 FAM20A | 0,858 | 0,848 | 0,868 | 0,890 | 0,824 | 0,956 | 0,922 | 0,841 | 1 |
| RSAD2 IFIT1 IFI44L HERC6 SLC1A2 IL1R2 | 0,858 | 0,849 | 0,867 | 0,902 | 0,844 | 0,960 | 0,947 | 0,896 | 0,998 |
| OAS1 IFI44L ISG15 OLAH FAM20A MMP8 | 0,858 | 0,847 | 0,869 | 0,890 | 0,818 | 0,962 | 0,953 | 0,898 | 1 |
| OAS1 ISG15 SLC1A2 IL1R2 OLAH MMP8 | 0,858 | 0,849 | 0,867 | 0,905 | 0,848 | 0,962 | 0,930 | 0,868 | 0,993 |
| IFI27 OAS1 IFIT1 IFI44L SIGLEC1 SLC1A2 | 0,858 | 0,849 | 0,867 | 0,896 | 0,836 | 0,955 | 0,955 | 0,906 | 1 |
| IFIT1 IFI44L ISG15 OLAH FAM20A MMP8 | 0,858 | 0,848 | 0,868 | 0,889 | 0,818 | 0,960 | 0,958 | 0,911 | 1 |
| OAS1 ISG15 SLC1A2 IL1R2 OLAH RETN | 0,858 | 0,848 | 0,868 | 0,907 | 0,849 | 0,964 | 0,934 | 0,874 | 0,994 |
| RSAD2 IFIT1 IFI44L HERC6 IL1R2 OLAH | 0,858 | 0,848 | 0,867 | 0,899 | 0,838 | 0,960 | 0,946 | 0,896 | 0,995 |
| IFI44L ISG15 HERC6 SLC1A2 IL1R2 MMP8 | 0,858 | 0,849 | 0,867 | 0,901 | 0,846 | 0,957 | 0,939 | 0,880 | 0,998 |
| OAS1 IFI44L HERC6 OLAH RETN MMP8 | 0,858 | 0,847 | 0,868 | 0,891 | 0,822 | 0,960 | 0,954 | 0,911 | 0,998 |
| OAS1 IFIT1 ISG15 SLC1A2 IL1R2 MMP8 | 0,858 | 0,849 | 0,867 | 0,903 | 0,846 | 0,960 | 0,952 | 0,906 | 0,999 |
| RSAD2 OAS1 IFI44L SIGLEC1 OLAH RETN | 0,858 | 0,848 | 0,868 | 0,891 | 0,820 | 0,961 | 0,953 | 0,907 | 0,999 |
| OAS1 IFIT1 IFI44L OLAH FAM20A RETN | 0,858 | 0,848 | 0,868 | 0,889 | 0,817 | 0,962 | 0,949 | 0,893 | 1 |
| OAS1 ISG15 OLAH FAM20A RETN MMP8 | 0,858 | 0,847 | 0,868 | 0,892 | 0,821 | 0,963 | 0,958 | 0,902 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFI44L ISG15 HERC6 SLC1A2 IL1R2 | 0,858 | 0,848 | 0,868 | 0,900 | 0,845 | 0,956 | 0,940 | 0,879 | 1 |
| OAS1 IFIT1 IFI44L ISG15 HERC6 RETN | 0,858 | 0,848 | 0,868 | 0,892 | 0,825 | 0,959 | 0,924 | 0,859 | 0,988 |
| RSAD2 IFIT1 IFI44L SIGLEC1 ISG15 OLAH | 0,858 | 0,848 | 0,867 | 0,895 | 0,828 | 0,962 | 0,947 | 0,895 | 0,999 |
| RSAD2 SIGLEC1 ISG15 OLAH RETN MMP8 | 0,858 | 0,847 | 0,868 | 0,892 | 0,822 | 0,963 | 0,963 | 0,922 | 1 |
| RSAD2 OAS1 IFIT1 SLC1A2 OLAH RETN | 0,858 | 0,848 | 0,867 | 0,906 | 0,847 | 0,964 | 0,945 | 0,894 | 0,995 |
| IFIT1 IFI44L ISG15 SLC1A2 IL1R2 RETN | 0,858 | 0,848 | 0,867 | 0,899 | 0,840 | 0,958 | 0,949 | 0,900 | 0,997 |
| RSAD2 IFI27 IFIT1 SIGLEC1 ISG15 MMP8 | 0,858 | 0,848 | 0,867 | 0,889 | 0,822 | 0,957 | 0,911 | 0,835 | 0,987 |
| OAS1 HERC6 SLC1A2 IL1R2 RETN MMP8 | 0,857 | 0,848 | 0,867 | 0,900 | 0,841 | 0,958 | 0,928 | 0,860 | 0,997 |
| RSAD2 IFI44L SIGLEC1 OLAH RETN MMP8 | 0,857 | 0,847 | 0,868 | 0,891 | 0,821 | 0,961 | 0,962 | 0,921 | 1 |
| IFI44L HERC6 SLC1A2 FAM20A RETN MMP8 | 0,857 | 0,847 | 0,868 | 0,896 | 0,830 | 0,963 | 0,939 | 0,860 | 1 |
| RSAD2 IFIT1 HERC6 IL1R2 OLAH RETN | 0,857 | 0,847 | 0,867 | 0,897 | 0,833 | 0,962 | 0,946 | 0,896 | 0,996 |
| IFIT1 SIGLEC1 ISG15 HERC6 SLC1A2 MMP8 | 0,857 | 0,848 | 0,866 | 0,894 | 0,834 | 0,954 | 0,914 | 0,846 | 0,982 |
| RSAD2 IFI27 OAS1 SIGLEC1 ISG15 SLC1A2 | 0,857 | 0,848 | 0,867 | 0,896 | 0,837 | 0,955 | 0,951 | 0,900 | 1 |
| RSAD2 IFIT1 ISG15 SLC1A2 OLAH MMP8 | 0,857 | 0,848 | 0,867 | 0,903 | 0,844 | 0,961 | 0,945 | 0,895 | 0,994 |
| RSAD2 OAS1 HERC6 SLC1A2 IL1R2 MMP8 | 0,857 | 0,848 | 0,867 | 0,898 | 0,841 | 0,956 | 0,934 | 0,868 | 0,999 |
| IFIT1 IFI44L ISG15 SLC1A2 IL1R2 MMP8 | 0,857 | 0,849 | 0,866 | 0,904 | 0,847 | 0,960 | 0,953 | 0,907 | 0,999 |
| RSAD2 OAS1 ISG15 SLC1A2 OLAH MMP8 | 0,857 | 0,848 | 0,867 | 0,901 | 0,842 | 0,959 | 0,937 | 0,881 | 0,993 |
| OAS1 IFI44L SIGLEC1 ISG15 OLAH RETN | 0,857 | 0,847 | 0,867 | 0,891 | 0,821 | 0,962 | 0,951 | 0,902 | 1,000 |
| RSAD2 OAS1 IFIT1 HERC6 OLAH RETN | 0,857 | 0,847 | 0,867 | 0,890 | 0,821 | 0,959 | 0,940 | 0,888 | 0,993 |
| RSAD2 IFI44L HERC6 SLC1A2 IL1R2 RETN | 0,857 | 0,848 | 0,867 | 0,901 | 0,843 | 0,960 | 0,934 | 0,869 | 0,998 |
| OAS1 SLC1A2 IL1R2 OLAH RETN MMP8 | 0,857 | 0,847 | 0,867 | 0,906 | 0,849 | 0,963 | 0,936 | 0,878 | 0,993 |
| OAS1 IFI44L SIGLEC1 ISG15 OLAH MMP8 | 0,857 | 0,847 | 0,867 | 0,892 | 0,824 | 0,960 | 0,952 | 0,907 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27 OAS1 IFIT1 ISG15 SLC1A2 FAM20A | 0,857 | 0,846 | 0,868 | 0,888 | 0,819 | 0,957 | 0,942 | 0,864 | 1 |
| OAS1 IFI44L ISG15 HERC6 SLC1A2 RETN | 0,857 | 0,847 | 0,867 | 0,899 | 0,836 | 0,961 | 0,934 | 0,869 | 0,998 |
| RSAD2 IFI27 OAS1 ISG15 SLC1A2 FAM20A | 0,857 | 0,846 | 0,868 | 0,889 | 0,820 | 0,958 | 0,936 | 0,854 | 1 |
| RSAD2 OAS1 IFIT1 IL1R2 OLAH MMP8 | 0,857 | 0,847 | 0,867 | 0,895 | 0,832 | 0,958 | 0,955 | 0,913 | 0,998 |
| RSAD2 OAS1 IFIT1 ISG15 SLC1A2 OLAH | 0,857 | 0,847 | 0,867 | 0,903 | 0,845 | 0,961 | 0,941 | 0,889 | 0,993 |
| RSAD2 IFIT1 SIGLEC1 HERC6 SLC1A2 MMP8 | 0,857 | 0,848 | 0,866 | 0,894 | 0,833 | 0,954 | 0,911 | 0,842 | 0,980 |
| IFIT1 ISG15 SLC1A2 OLAH RETN MMP8 | 0,857 | 0,847 | 0,867 | 0,902 | 0,842 | 0,962 | 0,954 | 0,911 | 0,998 |
| IFI44L SIGLEC1 ISG15 HERC6 SLC1A2 RETN | 0,857 | 0,847 | 0,866 | 0,894 | 0,828 | 0,960 | 0,955 | 0,909 | 1 |
| RSAD2 OAS1 IFI44L ISG15 HERC6 RETN | 0,857 | 0,846 | 0,867 | 0,893 | 0,825 | 0,961 | 0,938 | 0,882 | 0,994 |
| RSAD2 IFI27 OAS1 IFI44L SIGLEC1 HERC6 | 0,857 | 0,848 | 0,866 | 0,898 | 0,843 | 0,953 | 0,920 | 0,855 | 0,984 |
| RSAD2 OAS1 IFI44L HERC6 SLC1A2 IL1R2 | 0,857 | 0,847 | 0,867 | 0,898 | 0,841 | 0,955 | 0,934 | 0,869 | 0,998 |
| IFIT1 IFI44L SIGLEC1 HERC6 SLC1A2 MMP8 | 0,857 | 0,848 | 0,866 | 0,895 | 0,835 | 0,955 | 0,908 | 0,836 | 0,979 |
| RSAD2 IFIT1 ISG15 SLC1A2 IL1R2 MMP8 | 0,857 | 0,848 | 0,865 | 0,903 | 0,845 | 0,960 | 0,954 | 0,909 | 1,000 |
| OAS1 IFIT1 IFI44L SLC1A2 IL1R2 MMP8 | 0,857 | 0,848 | 0,866 | 0,903 | 0,846 | 0,960 | 0,951 | 0,904 | 0,998 |
| RSAD2 OAS1 HERC6 SLC1A2 FAM20A RETN | 0,857 | 0,847 | 0,867 | 0,896 | 0,831 | 0,961 | 0,936 | 0,855 | 1 |
| RSAD2 HERC6 SLC1A2 IL1R2 RETN MMP8 | 0,857 | 0,847 | 0,866 | 0,901 | 0,842 | 0,959 | 0,936 | 0,869 | 1 |
| OAS1 IFI44L HERC6 SLC1A2 IL1R2 MMP8 | 0,857 | 0,847 | 0,866 | 0,900 | 0,844 | 0,956 | 0,934 | 0,872 | 0,995 |
| RSAD2 IFI27 OAS1 IFI44L SIGLEC1 SLC1A2 | 0,857 | 0,847 | 0,866 | 0,896 | 0,837 | 0,955 | 0,952 | 0,902 | 1 |
| IFI27 IFIT1 SIGLEC1 ISG15 HERC6 SLC1A2 | 0,857 | 0,848 | 0,866 | 0,896 | 0,838 | 0,954 | 0,952 | 0,904 | 1 |
| RSAD2 OAS1 IFIT1 ISG15 SLC1A2 IL1R2 | 0,857 | 0,848 | 0,866 | 0,899 | 0,840 | 0,958 | 0,948 | 0,898 | 0,998 |
| RSAD2 IFIT1 ISG15 SLC1A2 OLAH RETN | 0,857 | 0,847 | 0,866 | 0,906 | 0,847 | 0,965 | 0,947 | 0,898 | 0,996 |
| RSAD2 IFIT1 IFI44L SLC1A2 IL1R2 MMP8 | 0,857 | 0,848 | 0,865 | 0,902 | 0,845 | 0,960 | 0,954 | 0,909 | 1,000 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 HERC6 SLC1A2 FAM20A RETN | 0,857 | 0,846 | 0,867 | 0,897 | 0,832 | 0,961 | 0,939 | 0,858 | 1 |
| RSAD2 IFIT1 HERC6 OLAH RETN MMP8 | 0,857 | 0,846 | 0,867 | 0,890 | 0,821 | 0,960 | 0,955 | 0,913 | 0,998 |
| OAS1 IFI44L OLAH FAM20A RETN MMP8 | 0,857 | 0,846 | 0,867 | 0,896 | 0,825 | 0,966 | 0,955 | 0,904 | 1 |
| RSAD2 IFI44L SIGLEC1 HERC6 RETN MMP8 | 0,857 | 0,847 | 0,867 | 0,890 | 0,819 | 0,962 | 0,937 | 0,883 | 0,990 |
| RSAD2 IFI27 IFIT1 ISG15 SLC1A2 FAM20A | 0,857 | 0,846 | 0,867 | 0,889 | 0,820 | 0,958 | 0,939 | 0,860 | 1 |
| OAS1 IFIT1 SLC1A2 IL1R2 RETN MMP8 | 0,856 | 0,847 | 0,866 | 0,902 | 0,843 | 0,961 | 0,958 | 0,915 | 1 |
| RSAD2 IFI27 OAS1 IFIT1 SIGLEC1 SLC1A2 | 0,856 | 0,847 | 0,866 | 0,896 | 0,837 | 0,955 | 0,951 | 0,900 | 1 |
| RSAD2 OAS1 IFI44L HERC6 OLAH RETN | 0,856 | 0,846 | 0,866 | 0,891 | 0,822 | 0,960 | 0,940 | 0,888 | 0,992 |
| RSAD2 IFIT1 SLC1A2 OLAH RETN MMP8 | 0,856 | 0,847 | 0,866 | 0,900 | 0,839 | 0,961 | 0,953 | 0,909 | 0,998 |
| OAS1 IFI44L ISG15 OLAH FAM20A RETN | 0,856 | 0,846 | 0,867 | 0,890 | 0,817 | 0,963 | 0,954 | 0,901 | 1 |
| IFIT1 IFI44L HERC6 SLC1A2 FAM20A MMP8 | 0,856 | 0,846 | 0,866 | 0,895 | 0,834 | 0,955 | 0,917 | 0,830 | 1 |
| RSAD2 IFI27 OAS1 IFIT1 SIGLEC1 HERC6 | 0,856 | 0,847 | 0,866 | 0,896 | 0,842 | 0,951 | 0,916 | 0,851 | 0,982 |
| RSAD2 IFI44L HERC6 SLC1A2 IL1R2 MMP8 | 0,856 | 0,847 | 0,866 | 0,900 | 0,843 | 0,956 | 0,936 | 0,874 | 0,998 |
| RSAD2 IFI27 IFIT1 SIGLEC1 ISG15 HERC6 | 0,856 | 0,847 | 0,866 | 0,893 | 0,837 | 0,950 | 0,914 | 0,847 | 0,981 |
| IFI44L SIGLEC1 ISG15 HERC6 RETN MMP8 | 0,856 | 0,846 | 0,867 | 0,884 | 0,808 | 0,959 | 0,951 | 0,904 | 0,998 |
| IFIT1 IFI44L ISG15 HERC6 FAM20A MMP8 | 0,856 | 0,846 | 0,866 | 0,891 | 0,825 | 0,957 | 0,922 | 0,837 | 1 |
| IFI27 IFI44L SIGLEC1 ISG15 HERC6 SLC1A2 | 0,856 | 0,848 | 0,865 | 0,894 | 0,835 | 0,953 | 0,949 | 0,897 | 1 |
| RSAD2 IFI44L HERC6 FAM20A RETN MMP8 | 0,856 | 0,846 | 0,866 | 0,895 | 0,826 | 0,963 | 0,935 | 0,858 | 1 |
| RSAD2 IFIT1 ISG15 SLC1A2 IL1R2 RETN | 0,856 | 0,847 | 0,865 | 0,897 | 0,836 | 0,957 | 0,946 | 0,893 | 0,998 |
| RSAD2 OAS1 IFI44L SIGLEC1 ISG15 OLAH | 0,856 | 0,846 | 0,866 | 0,893 | 0,824 | 0,961 | 0,943 | 0,892 | 0,995 |
| RSAD2 IFIT1 IFI44L HERC6 OLAH RETN | 0,856 | 0,846 | 0,866 | 0,892 | 0,824 | 0,961 | 0,936 | 0,881 | 0,991 |
| RSAD2 IFI27 IFI44L SIGLEC1 ISG15 SLC1A2 | 0,856 | 0,847 | 0,865 | 0,896 | 0,837 | 0,955 | 0,953 | 0,906 | 1 |

**EP 4 365 308 A1**

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 ISG15 SLC1A2 OLAH RETN | 0,856 | 0,846 | 0,866 | 0,902 | 0,843 | 0,961 | 0,942 | 0,890 | 0,995 |
| IFI27 OAS1 IFIT1 IFI44L SIGLEC1 HERC6 | 0,856 | 0,846 | 0,865 | 0,897 | 0,842 | 0,953 | 0,932 | 0,874 | 0,989 |
| RSAD2 IFI27 OAS1 IFIT1 SLC1A2 FAM20A | 0,856 | 0,845 | 0,866 | 0,890 | 0,821 | 0,959 | 0,938 | 0,857 | 1 |
| OAS1 IFIT1 IFI44L SLC1A2 IL1R2 RETN | 0,856 | 0,846 | 0,865 | 0,899 | 0,839 | 0,959 | 0,945 | 0,893 | 0,996 |
| OAS1 IFIT1 IFI44L ISG15 SLC1A2 IL1R2 | 0,856 | 0,846 | 0,865 | 0,901 | 0,844 | 0,959 | 0,947 | 0,897 | 0,997 |
| RSAD2 OAS1 IFI44L HERC6 SLC1A2 FAM20A | 0,856 | 0,845 | 0,866 | 0,895 | 0,833 | 0,956 | 0,917 | 0,834 | 1 |
| RSAD2 OAS1 IFIT1 SLC1A2 IL1R2 RETN | 0,856 | 0,846 | 0,865 | 0,896 | 0,836 | 0,957 | 0,948 | 0,896 | 1,000 |
| RSAD2 IFI44L SIGLEC1 ISG15 OLAH RETN | 0,856 | 0,846 | 0,866 | 0,891 | 0,821 | 0,962 | 0,951 | 0,902 | 1 |
| OAS1 IFIT1 IL1R2 OLAH RETN MMP8 | 0,856 | 0,846 | 0,866 | 0,893 | 0,826 | 0,960 | 0,957 | 0,914 | 0,999 |
| OAS1 SIGLEC1 ISG15 HERC6 SLC1A2 MMP8 | 0,856 | 0,846 | 0,865 | 0,893 | 0,832 | 0,955 | 0,936 | 0,878 | 0,994 |
| IFI44L ISG15 SLC1A2 IL1R2 RETN MMP8 | 0,856 | 0,846 | 0,865 | 0,901 | 0,845 | 0,957 | 0,950 | 0,897 | 1 |
| RSAD2 OAS1 SLC1A2 OLAH RETN MMP8 | 0,856 | 0,846 | 0,865 | 0,901 | 0,842 | 0,961 | 0,947 | 0,897 | 0,996 |
| IFIT1 IFI44L SLC1A2 IL1R2 RETN MMP8 | 0,856 | 0,846 | 0,865 | 0,903 | 0,845 | 0,961 | 0,959 | 0,917 | 1,000 |
| RSAD2 IFIT1 IFI44L ISG15 SLC1A2 IL1R2 | 0,856 | 0,847 | 0,864 | 0,900 | 0,842 | 0,958 | 0,948 | 0,898 | 0,998 |
| IFIT1 ISG15 SLC1A2 IL1R2 RETN MMP8 | 0,856 | 0,846 | 0,865 | 0,904 | 0,846 | 0,961 | 0,959 | 0,918 | 1,000 |
| RSAD2 ISG15 SLC1A2 OLAH RETN MMP8 | 0,856 | 0,846 | 0,865 | 0,900 | 0,840 | 0,960 | 0,952 | 0,906 | 0,998 |
| IFI44L SIGLEC1 HERC6 SLC1A2 RETN MMP8 | 0,856 | 0,846 | 0,865 | 0,893 | 0,826 | 0,959 | 0,952 | 0,904 | 1,000 |
| RSAD2 IFI27 IFIT1 SIGLEC1 ISG15 SLC1A2 | 0,855 | 0,846 | 0,865 | 0,895 | 0,836 | 0,955 | 0,952 | 0,903 | 1 |
| RSAD2 IFI44L ISG15 HERC6 FAM20A MMP8 | 0,855 | 0,845 | 0,866 | 0,890 | 0,825 | 0,956 | 0,926 | 0,849 | 1 |
| RSAD2 IFI44L HERC6 OLAH RETN MMP8 | 0,855 | 0,845 | 0,866 | 0,892 | 0,823 | 0,962 | 0,949 | 0,902 | 0,996 |
| RSAD2 OAS1 IFIT1 IFI44L IL1R2 MMP8 | 0,855 | 0,846 | 0,865 | 0,893 | 0,828 | 0,957 | 0,954 | 0,911 | 0,998 |
| IFIT1 IFI44L OLAH FAM20A RETN MMP8 | 0,855 | 0,845 | 0,866 | 0,894 | 0,826 | 0,963 | 0,961 | 0,918 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L SIGLEC1 ISG15 OLAH MMP8 | 0,855 | 0,845 | 0,865 | 0,892 | 0,824 | 0,960 | 0,954 | 0,909 | 0,999 |
| RSAD2 OAS1 SIGLEC1 HERC6 SLC1A2 MMP8 | 0,855 | 0,846 | 0,865 | 0,889 | 0,825 | 0,952 | 0,936 | 0,879 | 0,993 |
| RSAD2 IFIT1 IFI44L ISG15 HERC6 FAM20A | 0,855 | 0,845 | 0,866 | 0,892 | 0,827 | 0,956 | 0,918 | 0,836 | 1 |
| OAS1 IFI44L HERC6 FAM20A RETN MMP8 | 0,855 | 0,845 | 0,866 | 0,896 | 0,826 | 0,965 | 0,943 | 0,868 | 1 |
| IFIT1 HERC6 SLC1A2 FAM20A RETN MMP8 | 0,855 | 0,845 | 0,866 | 0,895 | 0,829 | 0,960 | 0,940 | 0,862 | 1 |
| IFIT1 IFI44L ISG15 OLAH FAM20A RETN | 0,855 | 0,845 | 0,865 | 0,892 | 0,821 | 0,962 | 0,955 | 0,907 | 1 |
| OAS1 IFIT1 ISG15 IL1R2 OLAH MMP8 | 0,855 | 0,846 | 0,865 | 0,894 | 0,830 | 0,957 | 0,960 | 0,920 | 1,000 |
| SIGLEC1 ISG15 HERC6 SLC1A2 RETN MMP8 | 0,855 | 0,845 | 0,865 | 0,893 | 0,826 | 0,960 | 0,960 | 0,916 | 1 |
| RSAD2 IFIT1 SLC1A2 IL1R2 RETN MMP8 | 0,855 | 0,846 | 0,864 | 0,902 | 0,843 | 0,960 | 0,959 | 0,917 | 1 |
| RSAD2 OAS1 IFIT1 IFI44L SLC1A2 IL1R2 | 0,855 | 0,846 | 0,864 | 0,899 | 0,840 | 0,958 | 0,949 | 0,899 | 0,999 |
| RSAD2 OAS1 IFIT1 IL1R2 OLAH RETN | 0,855 | 0,845 | 0,865 | 0,891 | 0,822 | 0,960 | 0,950 | 0,904 | 0,996 |
| RSAD2 IFIT1 HERC6 SLC1A2 FAM20A RETN | 0,855 | 0,845 | 0,865 | 0,897 | 0,832 | 0,961 | 0,937 | 0,856 | 1 |
| RSAD2 IFI27 IFI44L SIGLEC1 ISG15 HERC6 | 0,855 | 0,846 | 0,864 | 0,895 | 0,838 | 0,951 | 0,912 | 0,844 | 0,980 |
| IFIT1 IFI44L ISG15 HERC6 OLAH MMP8 | 0,855 | 0,845 | 0,865 | 0,888 | 0,822 | 0,954 | 0,939 | 0,886 | 0,992 |
| OAS1 ISG15 SLC1A2 OLAH RETN MMP8 | 0,855 | 0,845 | 0,865 | 0,903 | 0,844 | 0,961 | 0,943 | 0,892 | 0,995 |
| RSAD2 SIGLEC1 ISG15 HERC6 SLC1A2 MMP8 | 0,855 | 0,846 | 0,864 | 0,891 | 0,828 | 0,954 | 0,928 | 0,868 | 0,988 |
| OAS1 IFIT1 IFI44L SIGLEC1 SLC1A2 RETN | 0,855 | 0,846 | 0,864 | 0,895 | 0,833 | 0,957 | 0,940 | 0,886 | 0,995 |
| IFIT1 IFI44L ISG15 IL1R2 OLAH MMP8 | 0,855 | 0,845 | 0,865 | 0,893 | 0,832 | 0,955 | 0,965 | 0,929 | 1 |
| RSAD2 IFIT1 IFI44L SLC1A2 IL1R2 RETN | 0,855 | 0,846 | 0,864 | 0,898 | 0,838 | 0,958 | 0,949 | 0,899 | 0,999 |
| OAS1 IFIT1 IFI44L IL1R2 OLAH MMP8 | 0,855 | 0,845 | 0,864 | 0,894 | 0,831 | 0,958 | 0,959 | 0,918 | 0,999 |
| IFI27 IFIT1 IFI44L SIGLEC1 ISG15 SLC1A2 | 0,855 | 0,846 | 0,864 | 0,895 | 0,835 | 0,955 | 0,954 | 0,905 | 1 |
| RSAD2 OAS1 IFIT1 ISG15 IL1R2 OLAH | 0,855 | 0,845 | 0,864 | 0,893 | 0,826 | 0,960 | 0,946 | 0,896 | 0,995 |

(suite)

| Modèle | AUC modèle | IC 95% | | AUC Train | IC 95% Train | | AUC Test | IC 95% Test | |
|---|---|---|---|---|---|---|---|---|---|
| OAS1 IFIT1 ISG15 HERC6 SLC1A2 RETN | 0,855 | 0,844 | 0,865 | 0,896 | 0,833 | 0,959 | 0,939 | 0,876 | 1 |
| OAS1 IFIT1 SIGLEC1 ISG15 SLC1A2 RETN | 0,855 | 0,845 | 0,864 | 0,896 | 0,834 | 0,958 | 0,948 | 0,898 | 0,998 |
| RSAD2 IFI44L HERC6 SLC1A2 FAM20A MMP8 | 0,855 | 0,844 | 0,865 | 0,895 | 0,834 | 0,956 | 0,915 | 0,830 | 1 |
| RSAD2 OAS1 IFIT1 IFI44L IL1R2 OLAH | 0,854 | 0,845 | 0,864 | 0,894 | 0,827 | 0,961 | 0,946 | 0,896 | 0,995 |
| RSAD2 OAS1 IFIT1 ISG15 HERC6 RETN | 0,854 | 0,844 | 0,865 | 0,890 | 0,822 | 0,958 | 0,939 | 0,883 | 0,995 |
| OAS1 HERC6 SLC1A2 FAM20A RETN MMP8 | 0,854 | 0,844 | 0,865 | 0,894 | 0,829 | 0,960 | 0,936 | 0,854 | 1 |
| IFI27 OAS1 IFI44L ISG15 SLC1A2 MMP8 | 0,854 | 0,845 | 0,864 | 0,894 | 0,832 | 0,956 | 0,914 | 0,841 | 0,988 |
| ISG15 SLC1A2 IL1R2 OLAH RETN MMP8 | 0,854 | 0,845 | 0,864 | 0,900 | 0,843 | 0,958 | 0,930 | 0,862 | 0,999 |
| RSAD2 IFI27 OAS1 IFI44L SLC1A2 MMP8 | 0,854 | 0,844 | 0,864 | 0,893 | 0,831 | 0,956 | 0,914 | 0,838 | 0,991 |
| RSAD2 OAS1 HERC6 FAM20A RETN MMP8 | 0,854 | 0,844 | 0,865 | 0,888 | 0,818 | 0,959 | 0,937 | 0,859 | 1 |
| OAS1 IFIT1 ISG15 IL1R2 OLAH RETN | 0,854 | 0,844 | 0,864 | 0,892 | 0,824 | 0,960 | 0,951 | 0,905 | 0,997 |
| IFI27 IFIT1 IFI44L SIGLEC1 HERC6 SLC1A2 | 0,854 | 0,845 | 0,863 | 0,896 | 0,837 | 0,954 | 0,949 | 0,899 | 0,999 |
| OAS1 IFIT1 HERC6 FAM20A RETN MMP8 | 0,854 | 0,844 | 0,864 | 0,889 | 0,818 | 0,960 | 0,938 | 0,860 | 1 |
| OAS1 IFIT1 IFI44L HERC6 OLAH MMP8 | 0,854 | 0,844 | 0,864 | 0,887 | 0,821 | 0,953 | 0,937 | 0,882 | 0,992 |
| RSAD2 OAS1 IFIT1 SIGLEC1 SLC1A2 RETN | 0,854 | 0,844 | 0,864 | 0,896 | 0,835 | 0,958 | 0,947 | 0,896 | 0,997 |
| RSAD2 OAS1 IL1R2 OLAH RETN MMP8 | 0,854 | 0,844 | 0,864 | 0,892 | 0,824 | 0,959 | 0,954 | 0,910 | 0,998 |
| RSAD2 OAS1 IFIT1 IFI44L IL1R2 RETN | 0,854 | 0,844 | 0,864 | 0,887 | 0,817 | 0,958 | 0,950 | 0,904 | 0,996 |
| RSAD2 OAS1 ISG15 HERC6 SLC1A2 RETN | 0,854 | 0,844 | 0,864 | 0,895 | 0,831 | 0,958 | 0,937 | 0,872 | 1 |
| RSAD2 IFI44L ISG15 HERC6 SLC1A2 RETN | 0,854 | 0,844 | 0,864 | 0,898 | 0,835 | 0,961 | 0,929 | 0,867 | 0,991 |
| RSAD2 HERC6 SLC1A2 FAM20A RETN MMP8 | 0,854 | 0,844 | 0,864 | 0,896 | 0,831 | 0,960 | 0,937 | 0,855 | 1 |
| RSAD2 ISG15 HERC6 SLC1A2 FAM20A MMP8 | 0,854 | 0,843 | 0,864 | 0,895 | 0,833 | 0,957 | 0,924 | 0,839 | 1 |
| RSAD2 OAS1 IFIT1 IL1R2 RETN MMP8 | 0,854 | 0,844 | 0,864 | 0,890 | 0,820 | 0,959 | 0,960 | 0,920 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L ISG15 OLAH FAM20A MMP8 | 0,854 | 0,843 | 0,864 | 0,889 | 0,819 | 0,960 | 0,959 | 0,914 | 1 |
| RSAD2 IFIT1 IFI44L ISG15 HERC6 RETN | 0,854 | 0,844 | 0,864 | 0,888 | 0,819 | 0,957 | 0,928 | 0,868 | 0,988 |
| RSAD2 OAS1 ISG15 IL1R2 OLAH MMP8 | 0,854 | 0,844 | 0,863 | 0,894 | 0,831 | 0,957 | 0,943 | 0,891 | 0,996 |
| RSAD2 IFI27 IFIT1 IFI44L SIGLEC1 HERC6 | 0,854 | 0,844 | 0,863 | 0,896 | 0,840 | 0,951 | 0,913 | 0,844 | 0,982 |
| IFI44L ISG15 IL1R2 OLAH RETN MMP8 | 0,854 | 0,844 | 0,863 | 0,893 | 0,829 | 0,956 | 0,964 | 0,926 | 1 |
| RSAD2 IFI44L ISG15 SLC1A2 IL1R2 RETN | 0,854 | 0,844 | 0,863 | 0,901 | 0,844 | 0,958 | 0,946 | 0,892 | 0,999 |
| RSAD2 OAS1 ISG15 HERC6 OLAH MMP8 | 0,853 | 0,844 | 0,863 | 0,889 | 0,824 | 0,954 | 0,945 | 0,895 | 0,994 |
| RSAD2 OAS1 IFIT1 ISG15 IL1R2 MMP8 | 0,853 | 0,844 | 0,863 | 0,892 | 0,828 | 0,956 | 0,958 | 0,916 | 0,999 |
| RSAD2 IFIT1 IFI44L OLAH FAM20A MMP8 | 0,853 | 0,843 | 0,864 | 0,888 | 0,817 | 0,959 | 0,957 | 0,909 | 1 |
| OAS1 IFIT1 IFI44L ISG15 IL1R2 MMP8 | 0,853 | 0,844 | 0,863 | 0,892 | 0,827 | 0,956 | 0,957 | 0,915 | 0,998 |
| RSAD2 IFIT1 IFI44L HERC6 SLC1A2 FAM20A | 0,853 | 0,843 | 0,864 | 0,898 | 0,840 | 0,957 | 0,918 | 0,834 | 1 |
| OAS1 IFI44L SIGLEC1 HERC6 SLC1A2 MMP8 | 0,853 | 0,844 | 0,863 | 0,888 | 0,824 | 0,951 | 0,935 | 0,877 | 0,993 |
| RSAD2 OAS1 IFIT1 HERC6 OLAH MMP8 | 0,853 | 0,843 | 0,863 | 0,887 | 0,820 | 0,954 | 0,936 | 0,881 | 0,991 |
| IFIT1 ISG15 IL1R2 OLAH RETN MMP8 | 0,853 | 0,843 | 0,864 | 0,892 | 0,827 | 0,958 | 0,966 | 0,931 | 1 |
| OAS1 IFIT1 IFI44L IL1R2 RETN MMP8 | 0,853 | 0,843 | 0,863 | 0,890 | 0,821 | 0,959 | 0,961 | 0,922 | 1,000 |
| IFI27 OAS1 IFIT1 ISG15 SLC1A2 MMP8 | 0,853 | 0,844 | 0,863 | 0,889 | 0,825 | 0,952 | 0,920 | 0,846 | 0,993 |
| OAS1 ISG15 HERC6 SLC1A2 RETN MMP8 | 0,853 | 0,843 | 0,863 | 0,894 | 0,829 | 0,958 | 0,937 | 0,872 | 1 |
| RSAD2 IFI27 IFIT1 IFI44L SIGLEC1 SLC1A2 | 0,853 | 0,844 | 0,862 | 0,896 | 0,837 | 0,955 | 0,952 | 0,904 | 1 |
| OAS1 IFIT1 SIGLEC1 SLC1A2 RETN MMP8 | 0,853 | 0,844 | 0,863 | 0,896 | 0,834 | 0,958 | 0,951 | 0,904 | 0,998 |
| IFIT1 IFI44L IL1R2 OLAH RETN MMP8 | 0,853 | 0,843 | 0,863 | 0,893 | 0,827 | 0,960 | 0,964 | 0,927 | 1 |
| RSAD2 OAS1 IFIT1 IFI44L ISG15 IL1R2 | 0,853 | 0,844 | 0,863 | 0,891 | 0,825 | 0,957 | 0,949 | 0,902 | 0,996 |
| OAS1 IFI44L SIGLEC1 ISG15 SLC1A2 RETN | 0,853 | 0,844 | 0,863 | 0,896 | 0,834 | 0,958 | 0,950 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L ISG15 HERC6 SLC1A2 FAM20A MMP8 | 0,853 | 0,843 | 0,864 | 0,889 | 0,824 | 0,955 | 0,928 | 0,844 | 1 |
| OAS1 IFIT1 ISG15 HERC6 OLAH MMP8 | 0,853 | 0,843 | 0,863 | 0,888 | 0,822 | 0,953 | 0,948 | 0,900 | 0,995 |
| IFIT1 IFI44L SIGLEC1 ISG15 SLC1A2 RETN | 0,853 | 0,844 | 0,862 | 0,897 | 0,835 | 0,958 | 0,942 | 0,890 | 0,995 |
| RSAD2 OAS1 IFI44L HERC6 OLAH MMP8 | 0,853 | 0,843 | 0,863 | 0,889 | 0,822 | 0,955 | 0,933 | 0,875 | 0,990 |
| OAS1 IFIT1 IFI44L IL1R2 OLAH RETN | 0,853 | 0,843 | 0,863 | 0,892 | 0,823 | 0,960 | 0,947 | 0,897 | 0,996 |
| RSAD2 OAS1 IFI44L IL1R2 OLAH MMP8 | 0,853 | 0,843 | 0,863 | 0,895 | 0,832 | 0,958 | 0,946 | 0,894 | 0,997 |
| OAS1 ISG15 HERC6 SLC1A2 FAM20A MMP8 | 0,853 | 0,843 | 0,863 | 0,894 | 0,832 | 0,956 | 0,925 | 0,840 | 1 |
| RSAD2 ISG15 OLAH FAM20A RETN MMP8 | 0,853 | 0,843 | 0,863 | 0,889 | 0,818 | 0,960 | 0,964 | 0,917 | 1 |
| RSAD2 IFIT1 ISG15 IL1R2 OLAH MMP8 | 0,853 | 0,843 | 0,863 | 0,891 | 0,828 | 0,955 | 0,963 | 0,925 | 1 |
| RSAD2 IFIT1 IFI44L SIGLEC1 SLC1A2 RETN | 0,853 | 0,844 | 0,862 | 0,894 | 0,832 | 0,956 | 0,940 | 0,886 | 0,994 |
| OAS1 IFIT1 IFI44L ISG15 IL1R2 OLAH | 0,853 | 0,843 | 0,862 | 0,892 | 0,825 | 0,960 | 0,947 | 0,898 | 0,996 |
| RSAD2 OAS1 ISG15 IL1R2 OLAH RETN | 0,853 | 0,843 | 0,863 | 0,892 | 0,824 | 0,960 | 0,947 | 0,897 | 0,997 |
| RSAD2 OAS1 IFIT1 IFI44L HERC6 OLAH | 0,853 | 0,843 | 0,863 | 0,886 | 0,820 | 0,953 | 0,934 | 0,876 | 0,992 |
| RSAD2 OAS1 IFIT1 ISG15 IL1R2 RETN | 0,853 | 0,843 | 0,863 | 0,890 | 0,821 | 0,959 | 0,955 | 0,913 | 0,998 |
| OAS1 IFIT1 ISG15 IL1R2 RETNMMP8 | 0,853 | 0,843 | 0,863 | 0,891 | 0,823 | 0,959 | 0,967 | 0,932 | 1 |
| RSAD2 IFI44L SIGLEC1 HERC6 SLC1A2 MMP8 | 0,853 | 0,843 | 0,862 | 0,890 | 0,826 | 0,953 | 0,927 | 0,867 | 0,988 |
| OAS1 IFI44L ISG15 SLC1A2 IL1R2 RETN | 0,853 | 0,843 | 0,862 | 0,902 | 0,846 | 0,959 | 0,942 | 0,889 | 0,996 |
| IFI44L ISG15 SLC1A2 FAM20A RETN MMP8 | 0,853 | 0,843 | 0,863 | 0,893 | 0,828 | 0,959 | 0,935 | 0,859 | 1 |
| IFIT1 ISG15 OLAH FAM20A RETN MMP8 | 0,853 | 0,842 | 0,863 | 0,888 | 0,818 | 0,959 | 0,965 | 0,924 | 1 |
| RSAD2 OAS1 ISG15 HERC6 FAM20A MMP8 | 0,853 | 0,842 | 0,863 | 0,886 | 0,819 | 0,953 | 0,926 | 0,844 | 1 |
| RSAD2 OAS1 IFI44L IL1R2 OLAH RETN | 0,853 | 0,843 | 0,863 | 0,892 | 0,824 | 0,960 | 0,945 | 0,893 | 0,996 |
| IFI27 IFIT1 IFI44L ISG15 SLC1A2 MMP8 | 0,853 | 0,843 | 0,863 | 0,892 | 0,830 | 0,954 | 0,920 | 0,849 | 0,991 |

(suite)

| Modèle | AUC modèle | IC 95% | | AUC Train | IC 95% Train | | AUC Test | IC 95% Test | |
|---|---|---|---|---|---|---|---|---|---|
| RSAD2 OAS1 ISG15 HERC6 SLC1A2 FAM20A | 0,853 | 0,842 | 0,863 | 0,887 | 0,824 | 0,950 | 0,928 | 0,844 | 1 |
| RSAD2 IFI27 IFI44L ISG15 SLC1A2 MMP8 | 0,853 | 0,843 | 0,863 | 0,893 | 0,831 | 0,955 | 0,916 | 0,843 | 0,989 |
| IFIT1 IFI44L SIGLEC1 ISG15 HERC6 MMP8 | 0,853 | 0,843 | 0,862 | 0,885 | 0,819 | 0,951 | 0,895 | 0,816 | 0,973 |
| IFI27 OAS1 IFIT1 IFI44L SLC1A2 MMP8 | 0,853 | 0,843 | 0,863 | 0,892 | 0,829 | 0,954 | 0,916 | 0,842 | 0,991 |
| OAS1 IFI44L ISG15 HERC6 RETN MMP8 | 0,853 | 0,842 | 0,863 | 0,893 | 0,824 | 0,961 | 0,936 | 0,877 | 0,994 |
| OAS1 IFI44L ISG15 HERC6 SLC1A2 FAM20A | 0,853 | 0,842 | 0,863 | 0,890 | 0,827 | 0,953 | 0,935 | 0,854 | 1 |
| OAS1 IFI44L IL1R2 OLAH RETN MMP8 | 0,852 | 0,842 | 0,863 | 0,892 | 0,825 | 0,959 | 0,955 | 0,912 | 0,999 |
| RSAD2 OAS1 SIGLEC1 ISG15 SLC1A2 RETN | 0,852 | 0,843 | 0,862 | 0,894 | 0,831 | 0,957 | 0,953 | 0,904 | 1 |
| RSAD2 IFIT1 IFI44L ISG15 OLAH FAM20A | 0,852 | 0,842 | 0,863 | 0,889 | 0,818 | 0,960 | 0,957 | 0,908 | 1 |
| RSAD2 OAS1 IFI44L ISG15 IL1R2 OLAH | 0,852 | 0,843 | 0,862 | 0,894 | 0,828 | 0,959 | 0,942 | 0,890 | 0,995 |
| OAS1 ISG15 IL1R2 OLAH RETN MMP8 | 0,852 | 0,842 | 0,862 | 0,894 | 0,828 | 0,960 | 0,957 | 0,914 | 0,999 |
| RSAD2 IFIT1 IFI44L IL1R2 OLAH MMP8 | 0,852 | 0,842 | 0,862 | 0,889 | 0,825 | 0,954 | 0,963 | 0,925 | 1 |
| IFIT1 IFI44L ISG15 IL1R2 RETN MMP8 | 0,852 | 0,842 | 0,862 | 0,892 | 0,825 | 0,959 | 0,967 | 0,933 | 1 |
| IFIT1 ISG15 HERC6 SLC1A2 FAM20A MMP8 | 0,852 | 0,842 | 0,862 | 0,892 | 0,828 | 0,955 | 0,922 | 0,836 | 1 |
| OAS1 IFIT1 IFI44L ISG15 IL1R2 RETN | 0,852 | 0,842 | 0,862 | 0,890 | 0,821 | 0,959 | 0,955 | 0,913 | 0,998 |
| RSAD2 IFI27 OAS1 IFIT1 SLC1A2 MMP8 | 0,852 | 0,842 | 0,862 | 0,890 | 0,827 | 0,954 | 0,918 | 0,843 | 0,994 |
| RSAD2 IFIT1 HERC6 FAM20A RETN MMP8 | 0,852 | 0,842 | 0,863 | 0,889 | 0,818 | 0,960 | 0,939 | 0,861 | 1 |
| RSAD2 OAS1 IFIT1 ISG15 HERC6 OLAH | 0,852 | 0,842 | 0,862 | 0,885 | 0,820 | 0,951 | 0,945 | 0,895 | 0,994 |
| RSAD2 OAS1 ISG15 HERC6 RETN MMP8 | 0,852 | 0,842 | 0,862 | 0,889 | 0,821 | 0,957 | 0,940 | 0,885 | 0,995 |
| RSAD2 IFI27 OAS1 ISG15 SLC1A2 MMP8 | 0,852 | 0,842 | 0,862 | 0,890 | 0,827 | 0,954 | 0,914 | 0,836 | 0,992 |
| OAS1 IFIT1 IFI44L ISG15 HERC6 OLAH | 0,852 | 0,842 | 0,862 | 0,886 | 0,821 | 0,951 | 0,950 | 0,904 | 0,996 |
| RSAD2 OAS1 ISG15 SLC1A2 IL1R2 RETN | 0,852 | 0,842 | 0,862 | 0,896 | 0,836 | 0,955 | 0,945 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFI44L ISG15 OLAH FAM20A RETN | 0,852 | 0,841 | 0,863 | 0,890 | 0,820 | 0,961 | 0,958 | 0,912 | 1 |
| OAS1 SIGLEC1 ISG15 SLC1A2 RETN MMP8 | 0,852 | 0,842 | 0,862 | 0,894 | 0,832 | 0,957 | 0,957 | 0,911 | 1 |
| IFI27 IFIT1 IFI44L SIGLEC1 ISG15 HERC6 | 0,852 | 0,842 | 0,861 | 0,893 | 0,834 | 0,952 | 0,921 | 0,857 | 0,984 |
| RSAD2 IFIT1 ISG15 OLAH FAM20A MMP8 | 0,852 | 0,841 | 0,862 | 0,884 | 0,811 | 0,956 | 0,961 | 0,914 | 1 |
| OAS1 IFIT1 IFI44L SIGLEC1 HERC6 SLC1A2 | 0,852 | 0,843 | 0,861 | 0,890 | 0,830 | 0,950 | 0,927 | 0,868 | 0,986 |
| IFIT1 IFI44L SIGLEC1 SLC1A2 RETN MMP8 | 0,852 | 0,842 | 0,861 | 0,895 | 0,833 | 0,957 | 0,941 | 0,888 | 0,994 |
| OAS1 IFI44L HERC6 SLC1A2 FAM20A MMP8 | 0,852 | 0,841 | 0,862 | 0,892 | 0,829 | 0,954 | 0,927 | 0,845 | 1 |
| RSAD2 IFI44L ISG15 HERC6 RETN MMP8 | 0,852 | 0,841 | 0,862 | 0,892 | 0,824 | 0,960 | 0,933 | 0,875 | 0,991 |
| OAS1 IFIT1 ISG15 HERC6 RETN MMP8 | 0,852 | 0,841 | 0,862 | 0,889 | 0,821 | 0,957 | 0,938 | 0,882 | 0,995 |
| RSAD2 IFIT1 IFI44L ISG15 IL1R2 MMP8 | 0,852 | 0,842 | 0,861 | 0,891 | 0,827 | 0,955 | 0,961 | 0,922 | 1 |
| RSAD2 IFIT1 IFI44L HERC6 OLAH MMP8 | 0,852 | 0,842 | 0,861 | 0,887 | 0,821 | 0,953 | 0,935 | 0,879 | 0,991 |
| IFI44L SIGLEC1 ISG15 HERC6 SLC1A2 MMP8 | 0,851 | 0,842 | 0,861 | 0,887 | 0,822 | 0,952 | 0,937 | 0,880 | 0,994 |
| RSAD2 IFIT1 SIGLEC1 ISG15 SLC1A2 RETN | 0,851 | 0,842 | 0,861 | 0,896 | 0,834 | 0,958 | 0,949 | 0,900 | 0,998 |
| OAS1 IFI44L ISG15 HERC6 OLAH MMP8 | 0,851 | 0,841 | 0,862 | 0,888 | 0,823 | 0,954 | 0,948 | 0,900 | 0,996 |
| RSAD2 OAS1 IFI44L ISG15 SLC1A2 IL1R2 | 0,851 | 0,842 | 0,861 | 0,897 | 0,840 | 0,954 | 0,943 | 0,888 | 0,999 |
| RSAD2 OAS1 IFI44L ISG15 HERC6 OLAH | 0,851 | 0,841 | 0,861 | 0,889 | 0,825 | 0,953 | 0,941 | 0,890 | 0,993 |
| RSAD2 IFIT1 IL1R2 OLAH RETN MMP8 | 0,851 | 0,841 | 0,862 | 0,892 | 0,825 | 0,959 | 0,962 | 0,923 | 1 |
| RSAD2 IFIT1 ISG15 HERC6 OLAH MMP8 | 0,851 | 0,841 | 0,862 | 0,889 | 0,824 | 0,953 | 0,945 | 0,895 | 0,994 |
| OAS1 IFIT1 SIGLEC1 ISG15 HERC6 MMP8 | 0,851 | 0,842 | 0,861 | 0,886 | 0,823 | 0,949 | 0,896 | 0,817 | 0,974 |
| RSAD2 IFI44L ISG15 SLC1A2 IL1R2 MMP8 | 0,851 | 0,843 | 0,860 | 0,897 | 0,840 | 0,954 | 0,942 | 0,885 | 0,999 |
| RSAD2 OAS1 IFIT1 IFI44L HERC6 FAM20A | 0,851 | 0,841 | 0,862 | 0,884 | 0,816 | 0,953 | 0,923 | 0,841 | 1 |
| RSAD2 ISG15 IL1R2 OLAH RETN MMP8 | 0,851 | 0,841 | 0,861 | 0,892 | 0,826 | 0,958 | 0,968 | 0,933 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1 IFI44L ISG15 IL1R2 OLAH MMP8 | 0,851 | 0,842 | 0,861 | 0,895 | 0,832 | 0,957 | 0,947 | 0,897 | 0,997 |
| RSAD2 OAS1 IFI44L SLC1A2 IL1R2 RETN | 0,851 | 0,842 | 0,861 | 0,896 | 0,837 | 0,955 | 0,941 | 0,884 | 0,999 |
| RSAD2 IFIT1 ISG15 HERC6 FAM20A MMP8 | 0,851 | 0,841 | 0,862 | 0,887 | 0,822 | 0,953 | 0,923 | 0,840 | 1 |
| RSAD2 OAS1 IFI44L SIGLEC1 SLC1A2 RETN | 0,851 | 0,842 | 0,861 | 0,893 | 0,829 | 0,956 | 0,948 | 0,897 | 0,999 |
| RSAD2 OAS1 IFIT1 SIGLEC1 HERC6 SLC1A2 | 0,851 | 0,842 | 0,860 | 0,889 | 0,829 | 0,950 | 0,929 | 0,872 | 0,987 |
| OAS1 IFIT1 SIGLEC1 ISG15 HERC6 SLC1A2 | 0,851 | 0,842 | 0,860 | 0,890 | 0,830 | 0,950 | 0,933 | 0,876 | 0,989 |
| IFIT1 IFI44L ISG15 HERC6 SLC1A2 FAM20A | 0,851 | 0,841 | 0,861 | 0,892 | 0,830 | 0,953 | 0,925 | 0,837 | 1 |
| RSAD2 ISG15 SLC1A2 IL1R2 RETN MMP8 | 0,851 | 0,842 | 0,860 | 0,897 | 0,838 | 0,956 | 0,946 | 0,889 | 1 |
| OAS1 IFIT1 ISG15 HERC6 FAM20A MMP8 | 0,851 | 0,840 | 0,862 | 0,887 | 0,821 | 0,953 | 0,926 | 0,844 | 1 |
| OAS1 IFI44L ISG15 IL1R2 OLAH RETN | 0,851 | 0,841 | 0,861 | 0,892 | 0,825 | 0,960 | 0,949 | 0,900 | 0,998 |
| RSAD2 IFI44L ISG15 HERC6 OLAH MMP8 | 0,851 | 0,841 | 0,861 | 0,890 | 0,826 | 0,954 | 0,938 | 0,885 | 0,991 |
| RSAD2 IFIT1 ISG15 OLAH FAM20A RETN | 0,851 | 0,841 | 0,861 | 0,888 | 0,814 | 0,961 | 0,959 | 0,909 | 1 |
| RSAD2 IFI44L OLAH FAM20A RETN MMP8 | 0,851 | 0,840 | 0,862 | 0,895 | 0,826 | 0,963 | 0,959 | 0,913 | 1 |
| RSAD2 IFI44L SIGLEC1 ISG15 SLC1A2 RETN | 0,851 | 0,841 | 0,860 | 0,896 | 0,833 | 0,958 | 0,948 | 0,898 | 0,997 |
| OAS1 IFIT1 IFI44L SIGLEC1 HERC6 MMP8 | 0,851 | 0,841 | 0,860 | 0,883 | 0,817 | 0,949 | 0,888 | 0,807 | 0,969 |
| RSAD2 IFIT1 ISG15 IL1R2 RETN MMP8 | 0,851 | 0,841 | 0,861 | 0,891 | 0,824 | 0,959 | 0,967 | 0,933 | 1 |
| OAS1 IFI44L SIGLEC1 SLC1A2 RETN MMP8 | 0,851 | 0,841 | 0,860 | 0,893 | 0,829 | 0,956 | 0,951 | 0,901 | 1 |
| OAS1 IFI44L ISG15 SLC1A2 IL1R2 MMP8 | 0,851 | 0,842 | 0,860 | 0,898 | 0,841 | 0,954 | 0,940 | 0,883 | 0,997 |
| IFIT1 IFI44L ISG15 IL1R2 OLAH RETN | 0,851 | 0,841 | 0,861 | 0,892 | 0,826 | 0,958 | 0,963 | 0,924 | 1 |
| RSAD2 IFIT1 IFI44L ISG15 IL1R2 OLAH | 0,851 | 0,841 | 0,860 | 0,892 | 0,827 | 0,958 | 0,953 | 0,909 | 0,998 |
| RSAD2 IFIT1 IFI44L ISG15 IL1R2 RETN | 0,851 | 0,841 | 0,860 | 0,886 | 0,817 | 0,955 | 0,957 | 0,914 | 0,999 |
| RSAD2 IFI27 IFIT1 IFI44L SLC1A2 MMP8 | 0,851 | 0,841 | 0,860 | 0,891 | 0,829 | 0,954 | 0,917 | 0,846 | 0,989 |

(suite)

| Modèle | AUC modèle | IC 95% | | AUC Train | IC 95% Train | | AUC Test | IC 95% Test | |
|---|---|---|---|---|---|---|---|---|---|
| RSAD2 IFIT1 ISG15 IL1R2 OLAH RETN | 0,850 | 0,841 | 0,860 | 0,890 | 0,823 | 0,958 | 0,962 | 0,923 | 1 |
| RSAD2 IFI27 IFIT1 ISG15 SLC1A2 MMP8 | 0,850 | 0,841 | 0,860 | 0,889 | 0,825 | 0,952 | 0,920 | 0,847 | 0,992 |
| RSAD2 IFIT1 IFI44L IL1R2 RETN MMP8 | 0,850 | 0,840 | 0,861 | 0,889 | 0,820 | 0,959 | 0,964 | 0,927 | 1 |
| RSAD2 OAS1 IFI44L ISG15 SLC1A2 FAM20A | 0,850 | 0,839 | 0,861 | 0,893 | 0,828 | 0,959 | 0,916 | 0,831 | 1 |
| RSAD2 OAS1 IFI44L HERC6 SLC1A2 RETN | 0,850 | 0,840 | 0,861 | 0,889 | 0,822 | 0,956 | 0,936 | 0,875 | 0,996 |
| RSAD2 IFIT1 OLAH FAM20A RETN MMP8 | 0,850 | 0,840 | 0,861 | 0,886 | 0,814 | 0,959 | 0,964 | 0,920 | 1 |
| OAS1 IFIT1 IFI44L HERC6 FAM20A MMP8 | 0,850 | 0,840 | 0,861 | 0,885 | 0,817 | 0,954 | 0,922 | 0,840 | 1 |
| RSAD2 IFI44L IL1R2 OLAH RETN MMP8 | 0,850 | 0,840 | 0,860 | 0,890 | 0,824 | 0,956 | 0,964 | 0,927 | 1 |
| IFIT1 SIGLEC1 ISG15 SLC1A2 RETN MMP8 | 0,850 | 0,841 | 0,860 | 0,895 | 0,833 | 0,957 | 0,951 | 0,904 | 0,998 |
| RSAD2 OAS1 IFIT1 SIGLEC1 SLC1A2 MMP8 | 0,850 | 0,841 | 0,860 | 0,887 | 0,824 | 0,950 | 0,934 | 0,877 | 0,991 |
| RSAD2 IFIT1 ISG15 HERC6 SLC1A2 RETN | 0,850 | 0,840 | 0,860 | 0,891 | 0,826 | 0,956 | 0,936 | 0,873 | 0,999 |
| RSAD2 IFIT1 IFI44L HERC6 FAM20A MMP8 | 0,850 | 0,840 | 0,861 | 0,886 | 0,818 | 0,954 | 0,916 | 0,833 | 0,999 |
| RSAD2 OAS1 IFIT1 ISG15 HERC6 FAM20A | 0,850 | 0,840 | 0,860 | 0,881 | 0,813 | 0,949 | 0,924 | 0,839 | 1 |
| RSAD2 IFIT1 IFI44L ISG15 HERC6 OLAH | 0,850 | 0,840 | 0,860 | 0,889 | 0,826 | 0,953 | 0,943 | 0,894 | 0,993 |
| RSAD2 IFIT1 IFI44L SIGLEC1 HERC6 SLC1A2 | 0,850 | 0,841 | 0,859 | 0,890 | 0,830 | 0,951 | 0,923 | 0,862 | 0,984 |

Performances obtenues pour l'identification de la nature virale ou bactérienne de l'infection sur la base des combinaisons à 7 gènes :

[Tableau 11]

| Modèle | AUC modèle | IC 95% | | AUC Train | IC 95% Train | | AUC Test | IC 95% Test | |
|---|---|---|---|---|---|---|---|---|---|
| IFI27, SIGLEC1, ISG15, IL1R2, OLAH, FAM20A, MMP8 | 0,904 | 0,895 | 0,912 | 0,932 | 0,879 | 0,985 | 0,948 | 0,882 | 1 |
| IFI27, IFIT1, SIGLEC1, HERC6, OLAH, FAM20A, MMP8 | 0,903 | 0,894 | 0,912 | 0,931 | 0,876 | 0,986 | 0,953 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, IL1R2, OLAH, FAM20A, MMP8 | 0,903 | 0,895 | 0,911 | 0,934 | 0,883 | 0,985 | 0,958 | 0,900 | 1 |
| IFI27, OAS1, SIGLEC1, HERC6, IL1R2, FAM20A, MMP8 | 0,903 | 0,894 | 0,912 | 0,933 | 0,882 | 0,984 | 0,951 | 0,887 | 1 |
| IFI27, OAS1, IFIT1, SIGLEC1, HERC6, OLAH, FAM20A | 0,903 | 0,894 | 0,912 | 0,931 | 0,878 | 0,984 | 0,939 | 0,875 | 1 |
| IFI27, IFI44L, SIGLEC1, IL1R2, OLAH, FAM20A, MMP8 | 0,903 | 0,895 | 0,911 | 0,934 | 0,884 | 0,984 | 0,954 | 0,895 | 1 |
| IFI27, IFIT1, SIGLEC1, HERC6, IL1R2, FAM20A, MMP8 | 0,903 | 0,895 | 0,911 | 0,933 | 0,883 | 0,983 | 0,953 | 0,887 | 1 |
| RSAD2, IFI27, SIGLEC1, IL1R2, OLAH, FAM20A, MMP8 | 0,903 | 0,894 | 0,911 | 0,935 | 0,884 | 0,986 | 0,952 | 0,892 | 1 |
| IFI27, IFIT1, IFI44L, SIGLEC1, HERC6, OLAH, FAM20A | 0,903 | 0,894 | 0,911 | 0,931 | 0,876 | 0,986 | 0,943 | 0,881 | 1 |
| RSAD2, IFI27, SIGLEC1, HERC6, OLAH, FAM20A, MMP8 | 0,902 | 0,893 | 0,911 | 0,934 | 0,880 | 0,987 | 0,950 | 0,891 | 1 |
| IFI27, OAS1, SIGLEC1, IL1R2, OLAH, FAM20A, MMP8 | 0,902 | 0,894 | 0,911 | 0,934 | 0,883 | 0,986 | 0,951 | 0,892 | 1 |
| RSAD2, IFI27, SIGLEC1, HERC6, IL1R2, FAM20A, MMP8 | 0,902 | 0,893 | 0,910 | 0,934 | 0,883 | 0,985 | 0,953 | 0,886 | 1 |
| IFI27, IFIT1, SIGLEC1, HERC6, IL1R2, OLAH, FAM20A | 0,902 | 0,893 | 0,910 | 0,932 | 0,879 | 0,985 | 0,943 | 0,879 | 1 |
| IFI27, IFIT1, SIGLEC1, ISG15, HERC6, OLAH, FAM20A | 0,901 | 0,893 | 0,910 | 0,932 | 0,877 | 0,986 | 0,941 | 0,878 | 1 |
| IFI27, IFIT1, IFI44L, SIGLEC1, IL1R2, FAM20A, MMP8 | 0,901 | 0,893 | 0,909 | 0,931 | 0,880 | 0,983 | 0,955 | 0,893 | 1 |
| IFI27, IFI44L, SIGLEC1, ISG15, IL1R2, FAM20A, MMP8 | 0,901 | 0,894 | 0,909 | 0,933 | 0,882 | 0,983 | 0,950 | 0,882 | 1 |
| IFI27, IFI44L, SIGLEC1, HERC6, IL1R2, FAM20A, MMP8 | 0,901 | 0,893 | 0,910 | 0,931 | 0,879 | 0,983 | 0,952 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, ISG15, OLAH, FAM20A, MMP8 | 0,901 | 0,892 | 0,910 | 0,929 | 0,871 | 0,986 | 0,951 | 0,894 | 1 |
| IFI27, OAS1, SIGLEC1, HERC6, OLAH, FAM20A, MMP8 | 0,901 | 0,892 | 0,910 | 0,928 | 0,871 | 0,985 | 0,948 | 0,888 | 1 |
| IFI27, SIGLEC1, HERC6, IL1R2, OLAH, FAM20A, MMP8 | 0,901 | 0,892 | 0,910 | 0,930 | 0,876 | 0,984 | 0,955 | 0,897 | 1 |
| RSAD2, IFI27, IFIT1, SIGLEC1, HERC6, OLAH, FAM20A | 0,901 | 0,892 | 0,909 | 0,932 | 0,879 | 0,985 | 0,940 | 0,877 | 1 |
| RSAD2, IFI27, HERC6, IL1R2, OLAH, FAM20A, MMP8 | 0,901 | 0,892 | 0,909 | 0,933 | 0,883 | 0,983 | 0,927 | 0,850 | 1 |
| IFI27, IFI44L, SIGLEC1, ISG15, OLAH, FAM20A, MMP8 | 0,901 | 0,892 | 0,909 | 0,927 | 0,870 | 0,985 | 0,948 | 0,888 | 1 |
| IFI27, OAS1, IFIT1, SIGLEC1, IL1R2, FAM20A, MMP8 | 0,901 | 0,892 | 0,909 | 0,932 | 0,880 | 0,984 | 0,955 | 0,894 | 1 |
| IFI27, IFIT1, IFI44L, SIGLEC1, HERC6, IL1R2, FAM20A | 0,900 | 0,892 | 0,908 | 0,932 | 0,881 | 0,983 | 0,948 | 0,880 | 1 |
| IFI27, IFIT1, SIGLEC1, HERC6, OLAH, FAM20A, RETN | 0,900 | 0,892 | 0,909 | 0,931 | 0,875 | 0,986 | 0,943 | 0,881 | 1 |
| IFI27, OAS1, HERC6, IL1R2, OLAH, FAM20A, MMP8 | 0,900 | 0,892 | 0,909 | 0,933 | 0,883 | 0,983 | 0,928 | 0,853 | 1 |
| IFI27, OAS1, IFIT1, SIGLEC1, OLAH, FAM20A, MMP8 | 0,900 | 0,891 | 0,909 | 0,926 | 0,868 | 0,985 | 0,954 | 0,898 | 1 |
| IFI27, OAS1, SIGLEC1, HERC6, IL1R2, OLAH, FAM20A | 0,900 | 0,892 | 0,909 | 0,929 | 0,875 | 0,983 | 0,941 | 0,875 | 1 |
| IFI27, OAS1, IFIT1, SIGLEC1, HERC6, IL1R2, FAM20A | 0,900 | 0,892 | 0,908 | 0,933 | 0,883 | 0,984 | 0,946 | 0,879 | 1 |
| IFI27, IFIT1, SIGLEC1, ISG15, IL1R2, FAM20A, MMP8 | 0,900 | 0,892 | 0,908 | 0,932 | 0,880 | 0,983 | 0,954 | 0,888 | 1 |
| IFI27, SIGLEC1, ISG15, HERC6, IL1R2, FAM20A, MMP8 | 0,900 | 0,891 | 0,908 | 0,930 | 0,877 | 0,984 | 0,953 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, IL1R2, FAM20A, MMP8 | 0,900 | 0,892 | 0,908 | 0,931 | 0,880 | 0,983 | 0,958 | 0,897 | 1 |
| IFI27, IFIT1, IFI44L, SIGLEC1, HERC6, FAM20A, RETN | 0,900 | 0,891 | 0,908 | 0,928 | 0,873 | 0,984 | 0,940 | 0,867 | 1 |
| IFI27, IFIT1, SIGLEC1, ISG15, HERC6, IL1R2, FAM20A | 0,900 | 0,892 | 0,908 | 0,933 | 0,881 | 0,984 | 0,948 | 0,880 | 1 |
| RSAD2, IFI27, OAS1, SIGLEC1, HERC6, OLAH, FAM20A | 0,900 | 0,891 | 0,908 | 0,930 | 0,875 | 0,984 | 0,938 | 0,872 | 1 |
| IFI27, IFIT1, SIGLEC1, IL1R2, FAM20A, RETN, MMP8 | 0,900 | 0,891 | 0,908 | 0,934 | 0,882 | 0,986 | 0,957 | 0,893 | 1 |
| IFI27, IFIT1, IFI44L, SIGLEC1, OLAH, FAM20A, MMP8 | 0,900 | 0,891 | 0,908 | 0,927 | 0,870 | 0,985 | 0,955 | 0,901 | 1 |
| IFI27, OAS1, SIGLEC1, IL1R2, FAM20A, RETN, MMP8 | 0,899 | 0,890 | 0,908 | 0,931 | 0,877 | 0,985 | 0,953 | 0,889 | 1 |
| IFI27, IFI44L, SIGLEC1, HERC6, IL1R2, OLAH, FAM20A | 0,899 | 0,891 | 0,908 | 0,927 | 0,873 | 0,982 | 0,947 | 0,881 | 1 |
| RSAD2, IFI27, SIGLEC1, IL1R2, FAM20A, RETN, MMP8 | 0,899 | 0,890 | 0,908 | 0,935 | 0,883 | 0,987 | 0,950 | 0,883 | 1 |
| IFI27, OAS1, IFI44L, SIGLEC1, IL1R2, FAM20A, MMP8 | 0,899 | 0,891 | 0,908 | 0,933 | 0,882 | 0,984 | 0,953 | 0,889 | 1 |
| IFI27, ISG15, HERC6, IL1R2, OLAH, FAM20A, MMP8 | 0,899 | 0,891 | 0,908 | 0,930 | 0,879 | 0,982 | 0,927 | 0,852 | 1 |
| IFI27, IFI44L, SIGLEC1, IL1R2, FAM20A, RETN, MMP8 | 0,899 | 0,891 | 0,908 | 0,934 | 0,883 | 0,986 | 0,953 | 0,885 | 1 |
| RSAD2, IFI27, OAS1, SIGLEC1, IL1R2, FAM20A, MMP8 | 0,899 | 0,891 | 0,908 | 0,933 | 0,880 | 0,985 | 0,954 | 0,891 | 1 |
| IFI27, SIGLEC1, ISG15, HERC6, OLAH, FAM20A, MMP8 | 0,899 | 0,889 | 0,909 | 0,924 | 0,864 | 0,984 | 0,951 | 0,892 | 1 |
| IFI27, IFIT1, SIGLEC1, HERC6, IL1R2, FAM20A, RETN | 0,899 | 0,891 | 0,907 | 0,932 | 0,879 | 0,985 | 0,948 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, HERC6, IL1R2, OLAH, FAM20A | 0,899 | 0,890 | 0,908 | 0,930 | 0,876 | 0,985 | 0,947 | 0,884 | 1 |
| IFI27, IFI44L, SIGLEC1, ISG15, HERC6, OLAH, FAM20A | 0,899 | 0,890 | 0,908 | 0,929 | 0,872 | 0,985 | 0,943 | 0,878 | 1 |
| RSAD2, IFI27, SIGLEC1, ISG15, OLAH, FAM20A, MMP8 | 0,899 | 0,890 | 0,908 | 0,931 | 0,874 | 0,988 | 0,943 | 0,880 | 1 |
| IFI27, IFI44L, ISG15, IL1R2, OLAH, FAM20A, MMP8 | 0,899 | 0,890 | 0,907 | 0,930 | 0,876 | 0,983 | 0,928 | 0,856 | 1 |
| IFI27, OAS1, IFI44L, SIGLEC1, HERC6, IL1R2, FAM20A | 0,899 | 0,891 | 0,907 | 0,933 | 0,884 | 0,983 | 0,946 | 0,879 | 1 |
| IFI27, OAS1, SIGLEC1, ISG15, OLAH, FAM20A, MMP8 | 0,899 | 0,890 | 0,908 | 0,925 | 0,865 | 0,985 | 0,948 | 0,888 | 1 |
| IFI27, OAS1, SIGLEC1, ISG15, HERC6, OLAH, FAM20A | 0,899 | 0,890 | 0,908 | 0,928 | 0,872 | 0,984 | 0,939 | 0,873 | 1 |
| IFI27, IFIT1, IFI44L, SIGLEC1, ISG15, OLAH, FAM20A | 0,899 | 0,890 | 0,907 | 0,926 | 0,868 | 0,984 | 0,943 | 0,884 | 1 |
| IFI27, OAS1, IFI44L, SIGLEC1, HERC6, OLAH, FAM20A | 0,899 | 0,890 | 0,907 | 0,930 | 0,876 | 0,984 | 0,940 | 0,874 | 1 |
| IFI27, IFI44L, SIGLEC1, ISG15, IL1R2, OLAH, FAM20A | 0,899 | 0,890 | 0,907 | 0,928 | 0,873 | 0,983 | 0,946 | 0,882 | 1 |
| RSAD2, IFI27, IFI44L, SIGLEC1, IL1R2, FAM20A, MMP8 | 0,899 | 0,890 | 0,907 | 0,931 | 0,880 | 0,983 | 0,952 | 0,886 | 1 |
| IFI27, IFI44L, SIGLEC1, ISG15, HERC6, IL1R2, FAM20A | 0,899 | 0,890 | 0,907 | 0,929 | 0,875 | 0,982 | 0,941 | 0,870 | 1 |
| IFI27, OAS1, IFIT1, SIGLEC1, HERC6, FAM20A, RETN | 0,898 | 0,890 | 0,907 | 0,930 | 0,877 | 0,984 | 0,936 | 0,864 | 1 |
| IFI27, IFI44L, HERC6, IL1R2, OLAH, FAM20A, MMP8 | 0,898 | 0,890 | 0,907 | 0,931 | 0,880 | 0,983 | 0,936 | 0,864 | 1 |
| RSAD2, IFI27, IFIT1, SIGLEC1, OLAH, FAM20A, MMP8 | 0,898 | 0,890 | 0,907 | 0,928 | 0,870 | 0,985 | 0,954 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, HERC6, IL1R2, FAM20A | 0,898 | 0,890 | 0,907 | 0,932 | 0,881 | 0,983 | 0,949 | 0,883 | 1 |
| IFI27, IFIT1, SIGLEC1, HERC6, FAM20A, RETN, MMP8 | 0,898 | 0,889 | 0,907 | 0,926 | 0,869 | 0,984 | 0,949 | 0,878 | 1 |
| RSAD2, IFI27, OAS1, SIGLEC1, HERC6, IL1R2, FAM20A | 0,898 | 0,890 | 0,907 | 0,930 | 0,878 | 0,983 | 0,948 | 0,882 | 1 |
| RSAD2, IFI27, SIGLEC1, ISG15, IL1R2, FAM20A, MMP8 | 0,898 | 0,890 | 0,907 | 0,934 | 0,883 | 0,985 | 0,952 | 0,885 | 1 |
| IFI27, IFIT1, HERC6, IL1R2, OLAH, FAM20A, MMP8 | 0,898 | 0,890 | 0,907 | 0,932 | 0,880 | 0,983 | 0,937 | 0,867 | 1 |
| IFI27, IFIT1, SIGLEC1, ISG15, IL1R2, OLAH, FAM20A | 0,898 | 0,890 | 0,906 | 0,928 | 0,872 | 0,985 | 0,943 | 0,879 | 1 |
| IFI27, SIGLEC1, ISG15, IL1R2, FAM20A, RETN, MMP8 | 0,898 | 0,889 | 0,907 | 0,933 | 0,879 | 0,986 | 0,954 | 0,886 | 1 |
| IFI27, OAS1, SIGLEC1, HERC6, OLAH, FAM20A, RETN | 0,898 | 0,889 | 0,907 | 0,930 | 0,875 | 0,984 | 0,939 | 0,874 | 1 |
| RSAD2, IFI27, OAS1, SIGLEC1, OLAH, FAM20A, MMP8 | 0,898 | 0,889 | 0,907 | 0,927 | 0,869 | 0,985 | 0,951 | 0,895 | 1 |
| RSAD2, IFI27, IFI44L, SIGLEC1, HERC6, IL1R2, FAM20A | 0,898 | 0,890 | 0,906 | 0,930 | 0,877 | 0,982 | 0,945 | 0,876 | 1 |
| IFI27, OAS1, IFIT1, IFI44L, SIGLEC1, OLAH, FAM20A | 0,898 | 0,890 | 0,907 | 0,923 | 0,863 | 0,983 | 0,951 | 0,892 | 1 |
| RSAD2, IFI27, OAS1, HERC6, OLAH, FAM20A, MMP8 | 0,898 | 0,889 | 0,907 | 0,929 | 0,876 | 0,982 | 0,932 | 0,861 | 1 |
| IFI27, IFIT1, IFI44L, SIGLEC1, IL1R2, OLAH, FAM20A | 0,898 | 0,890 | 0,906 | 0,927 | 0,870 | 0,983 | 0,947 | 0,884 | 1 |
| IFI27, OAS1, IFIT1, SIGLEC1, IL1R2, OLAH, FAM20A | 0,898 | 0,890 | 0,906 | 0,926 | 0,869 | 0,983 | 0,949 | 0,887 | 1 |
| IFI27, IFI44L, SIGLEC1, HERC6, OLAH, FAM20A, MMP8 | 0,898 | 0,889 | 0,907 | 0,925 | 0,866 | 0,984 | 0,952 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, SIGLEC1, IL1R2, OLAH, FAM20A, RETN, MMP8 | 0,898 | 0,889 | 0,907 | 0,932 | 0,877 | 0,986 | 0,954 | 0,893 | 1 |
| IFI27, OAS1, SIGLEC1, ISG15, IL1R2, FAM20A, MMP8 | 0,898 | 0,889 | 0,907 | 0,931 | 0,880 | 0,983 | 0,951 | 0,885 | 1 |
| IFI27, SIGLEC1, ISG15, SLC1A2, OLAH, FAM20A, MMP8 | 0,898 | 0,888 | 0,907 | 0,935 | 0,886 | 0,985 | 0,941 | 0,874 | 1 |
| RSAD2, IFI27, OAS1, HERC6, IL1R2, OLAH, FAM20A | 0,898 | 0,889 | 0,906 | 0,929 | 0,877 | 0,981 | 0,927 | 0,853 | 1 |
| IFI27, IFIT1, SIGLEC1, ISG15, HERC6, FAM20A, RETN | 0,898 | 0,889 | 0,906 | 0,928 | 0,872 | 0,983 | 0,941 | 0,869 | 1 |
| IFI27, IFIT1, SIGLEC1, OLAH, FAM20A, RETN, MMP8 | 0,897 | 0,888 | 0,907 | 0,929 | 0,870 | 0,987 | 0,960 | 0,907 | 1 |
| RSAD2, IFI27, SIGLEC1, ISG15, HERC6, OLAH, FAM20A | 0,897 | 0,888 | 0,907 | 0,930 | 0,875 | 0,985 | 0,940 | 0,875 | 1 |
| RSAD2, IFI27, IFI44L, SIGLEC1, HERC6, OLAH, FAM20A | 0,897 | 0,889 | 0,906 | 0,931 | 0,876 | 0,985 | 0,943 | 0,880 | 1 |
| IFI27, OAS1, IFIT1, HERC6, OLAH, FAM20A, MMP8 | 0,897 | 0,888 | 0,907 | 0,927 | 0,872 | 0,982 | 0,935 | 0,867 | 1 |
| IFI27, OAS1, IFIT1, IFI44L, HERC6, OLAH, FAM20A | 0,897 | 0,888 | 0,906 | 0,928 | 0,873 | 0,983 | 0,932 | 0,860 | 1 |
| IFI27, SIGLEC1, ISG15, OLAH, FAM20A, RETN, MMP8 | 0,897 | 0,887 | 0,907 | 0,927 | 0,868 | 0,985 | 0,957 | 0,899 | 1 |
| IFI27, OAS1, SIGLEC1, HERC6, IL1R2, FAM20A, RETN | 0,897 | 0,888 | 0,906 | 0,932 | 0,881 | 0,983 | 0,945 | 0,877 | 1 |
| RSAD2, IFI27, IFI44L, HERC6, OLAH, FAM20A, MMP8 | 0,897 | 0,888 | 0,907 | 0,929 | 0,875 | 0,984 | 0,934 | 0,866 | 1 |
| IFI27, SIGLEC1, ISG15, HERC6, IL1R2, OLAH, FAM20A | 0,897 | 0,888 | 0,906 | 0,928 | 0,873 | 0,983 | 0,948 | 0,886 | 1 |
| IFI27, OAS1, IFIT1, SIGLEC1, ISG15, OLAH, FAM20A | 0,897 | 0,888 | 0,906 | 0,926 | 0,868 | 0,984 | 0,943 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, HERC6, IL1R2, OLAH, FAM20A | 0,897 | 0,888 | 0,906 | 0,929 | 0,875 | 0,982 | 0,928 | 0,853 | 1 |
| RSAD2, IFI27, SIGLEC1, HERC6, OLAH, FAM20A, RETN | 0,897 | 0,888 | 0,906 | 0,929 | 0,874 | 0,984 | 0,938 | 0,872 | 1 |
| IFI27, OAS1, SIGLEC1, ISG15, HERC6, IL1R2, FAM20A | 0,897 | 0,888 | 0,906 | 0,930 | 0,877 | 0,982 | 0,949 | 0,883 | 1 |
| IFI27, OAS1, SIGLEC1, HERC6, FAM20A, RETN, MMP8 | 0,897 | 0,887 | 0,907 | 0,929 | 0,872 | 0,986 | 0,943 | 0,872 | 1 |
| IFI27, OAS1, IFIT1, HERC6, IL1R2, OLAH, FAM20A | 0,897 | 0,888 | 0,906 | 0,930 | 0,878 | 0,981 | 0,928 | 0,854 | 1 |
| IFI27, IFIT1, SIGLEC1, ISG15, OLAH, FAM20A, RETN | 0,897 | 0,888 | 0,906 | 0,926 | 0,868 | 0,985 | 0,943 | 0,882 | 1 |
| IFI27, IFIT1, IFI44L, SIGLEC1, OLAH, FAM20A, RETN | 0,897 | 0,888 | 0,905 | 0,924 | 0,864 | 0,984 | 0,950 | 0,891 | 1 |
| IFI27, OAS1, SIGLEC1, OLAH, FAM20A, RETN, MMP8 | 0,897 | 0,887 | 0,906 | 0,928 | 0,870 | 0,986 | 0,952 | 0,894 | 1 |
| RSAD2, IFI27, SIGLEC1, HERC6, IL1R2, FAM20A, RETN | 0,897 | 0,888 | 0,906 | 0,932 | 0,880 | 0,984 | 0,946 | 0,879 | 1 |
| IFI27, IFI44L, ISG15, HERC6, IL1R2, OLAH, FAM20A | 0,897 | 0,888 | 0,905 | 0,929 | 0,875 | 0,982 | 0,928 | 0,855 | 1 |
| IFI27, IFIT1, IFI44L, IL1R2, OLAH, FAM20A, MMP8 | 0,897 | 0,888 | 0,905 | 0,929 | 0,876 | 0,983 | 0,936 | 0,868 | 1 |
| RSAD2, IFI27, IFIT1, HERC6, OLAH, FAM20A, MMP8 | 0,896 | 0,887 | 0,905 | 0,929 | 0,876 | 0,982 | 0,934 | 0,865 | 1 |
| IFI27, OAS1, ISG15, HERC6, OLAH, FAM20A, MMP8 | 0,896 | 0,887 | 0,906 | 0,926 | 0,872 | 0,981 | 0,932 | 0,862 | 1 |
| IFI27, IFIT1, SIGLEC1, IL1R2, OLAH, FAM20A, RETN | 0,896 | 0,888 | 0,905 | 0,927 | 0,871 | 0,984 | 0,948 | 0,886 | 1 |
| IFI27, IFIT1, IFI44L, SIGLEC1, ISG15, IL1R2, FAM20A | 0,896 | 0,888 | 0,904 | 0,928 | 0,873 | 0,983 | 0,949 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, ISG15, HERC6, IL1R2, OLAH, FAM20A | 0,896 | 0,888 | 0,905 | 0,929 | 0,876 | 0,981 | 0,926 | 0,852 | 1 |
| IFI27, OAS1, IFI44L, HERC6, IL1R2, OLAH, FAM20A | 0,896 | 0,887 | 0,905 | 0,930 | 0,877 | 0,982 | 0,926 | 0,850 | 1 |
| IFI27, SIGLEC1, HERC6, IL1R2, FAM20A, RETN, MMP8 | 0,896 | 0,887 | 0,906 | 0,931 | 0,877 | 0,985 | 0,955 | 0,888 | 1 |
| RSAD2, IFI27, SIGLEC1, ISG15, HERC6, IL1R2, FAM20A | 0,896 | 0,887 | 0,905 | 0,930 | 0,876 | 0,983 | 0,947 | 0,880 | 1 |
| IFI27, IFIT1, IFI44L, HERC6, OLAH, FAM20A, MMP8 | 0,896 | 0,887 | 0,905 | 0,925 | 0,869 | 0,982 | 0,937 | 0,871 | 1 |
| IFI27, OAS1, IFI44L, SIGLEC1, OLAH, FAM20A, MMP8 | 0,896 | 0,887 | 0,905 | 0,924 | 0,864 | 0,984 | 0,953 | 0,898 | 1 |
| RSAD2, IFI27, OAS1, IFI44L, HERC6, OLAH, FAM20A | 0,896 | 0,887 | 0,905 | 0,929 | 0,875 | 0,983 | 0,930 | 0,859 | 1 |
| RSAD2, IFI27, IFIT1, SIGLEC1, IL1R2, OLAH, FAM20A | 0,896 | 0,888 | 0,904 | 0,928 | 0,871 | 0,984 | 0,948 | 0,886 | 1 |
| RSAD2, IFI27, SIGLEC1, OLAH, FAM20A, RETN, MMP8 | 0,896 | 0,887 | 0,905 | 0,928 | 0,870 | 0,986 | 0,958 | 0,905 | 1 |
| IFI27, IFIT1, ISG15, HERC6, IL1R2, OLAH, FAM20A | 0,896 | 0,887 | 0,905 | 0,929 | 0,876 | 0,983 | 0,927 | 0,853 | 1 |
| IFI27, OAS1, IFI44L, HERC6, OLAH, FAM20A, MMP8 | 0,896 | 0,886 | 0,906 | 0,927 | 0,872 | 0,983 | 0,935 | 0,867 | 1 |
| IFI27, OAS1, SIGLEC1, ISG15, IL1R2, OLAH, FAM20A | 0,896 | 0,887 | 0,905 | 0,926 | 0,869 | 0,983 | 0,946 | 0,882 | 1 |
| IFI27, IFI44L, ISG15, HERC6, OLAH, FAM20A, MMP8 | 0,896 | 0,886 | 0,906 | 0,925 | 0,868 | 0,982 | 0,938 | 0,873 | 1 |
| IFI27, IFIT1, ISG15, IL1R2, OLAH, FAM20A, MMP8 | 0,896 | 0,887 | 0,905 | 0,928 | 0,875 | 0,982 | 0,934 | 0,864 | 1 |
| IFI27, ISG15, HERC6, OLAH, FAM20A, RETN, MMP8 | 0,896 | 0,886 | 0,906 | 0,927 | 0,870 | 0,984 | 0,937 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, ISG15, HERC6, IL1R2, OLAH, FAM20A | 0,896 | 0,887 | 0,905 | 0,930 | 0,878 | 0,982 | 0,929 | 0,857 | 1 |
| IFI27, IFIT1, SIGLEC1, SLC1A2, OLAH, FAM20A, MMP8 | 0,896 | 0,887 | 0,905 | 0,933 | 0,881 | 0,985 | 0,951 | 0,889 | 1 |
| RSAD2, IFI27, IFI44L, SIGLEC1, OLAH, FAM20A, MMP8 | 0,896 | 0,887 | 0,905 | 0,927 | 0,869 | 0,985 | 0,954 | 0,900 | 1 |
| RSAD2, IFI27, HERC6, OLAH, FAM20A, RETN, MMP8 | 0,896 | 0,887 | 0,905 | 0,931 | 0,877 | 0,985 | 0,930 | 0,858 | 1 |
| IFI27, IFI44L, SIGLEC1, HERC6, IL1R2, FAM20A, RETN | 0,896 | 0,887 | 0,905 | 0,930 | 0,877 | 0,983 | 0,942 | 0,870 | 1 |
| RSAD2, IFI27, ISG15, HERC6, OLAH, FAM20A, MMP8 | 0,896 | 0,887 | 0,905 | 0,927 | 0,872 | 0,982 | 0,930 | 0,861 | 1,000 |
| IFI27, IFIT1, IFI44L, ISG15, HERC6, OLAH, FAM20A | 0,896 | 0,887 | 0,905 | 0,926 | 0,869 | 0,982 | 0,933 | 0,864 | 1 |
| IFI27, OAS1, IFIT1, IL1R2, OLAH, FAM20A, MMP8 | 0,896 | 0,887 | 0,905 | 0,927 | 0,873 | 0,982 | 0,935 | 0,867 | 1 |
| IFI27, OAS1, SIGLEC1, SLC1A2, OLAH, FAM20A, MMP8 | 0,896 | 0,887 | 0,905 | 0,935 | 0,884 | 0,986 | 0,942 | 0,877 | 1 |
| IFI27, OAS1, IFIT1, IFI44L, SIGLEC1, IL1R2, FAM20A | 0,896 | 0,888 | 0,904 | 0,927 | 0,872 | 0,983 | 0,949 | 0,886 | 1 |
| RSAD2, IFI27, IFIT1, IFI44L, HERC6, OLAH, FAM20A | 0,896 | 0,887 | 0,905 | 0,929 | 0,874 | 0,983 | 0,928 | 0,858 | 0,998 |
| IFI27, OAS1, IFI44L, SIGLEC1, IL1R2, OLAH, FAM20A | 0,896 | 0,887 | 0,904 | 0,926 | 0,869 | 0,982 | 0,952 | 0,892 | 1 |
| RSAD2, IFI27, IFI44L, HERC6, IL1R2, OLAH, FAM20A | 0,896 | 0,887 | 0,905 | 0,929 | 0,876 | 0,983 | 0,928 | 0,856 | 1 |
| IFI27, OAS1, IFIT1, SIGLEC1, OLAH, FAM20A, RETN | 0,896 | 0,887 | 0,905 | 0,924 | 0,865 | 0,983 | 0,951 | 0,891 | 1 |
| RSAD2, IFI27, OAS1, IL1R2, OLAH, FAM20A, MMP8 | 0,896 | 0,887 | 0,905 | 0,929 | 0,876 | 0,982 | 0,933 | 0,861 | 1 |
| IFI27, IFIT1, IFI44L, SIGLEC1, IL1R2, FAM20A, RETN | 0,896 | 0,887 | 0,904 | 0,927 | 0,872 | 0,982 | 0,949 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, OLAH, FAM20A, RETN, MMP8 | 0,896 | 0,886 | 0,905 | 0,928 | 0,871 | 0,985 | 0,953 | 0,894 | 1 |
| RSAD2, IFI27, IFIT1, SIGLEC1, HERC6, FAM20A, RETN | 0,896 | 0,887 | 0,904 | 0,930 | 0,876 | 0,983 | 0,935 | 0,861 | 1 |
| IFI27, IFIT1, ISG15, HERC6, OLAH, FAM20A, MMP8 | 0,896 | 0,886 | 0,905 | 0,926 | 0,870 | 0,982 | 0,935 | 0,868 | 1 |
| RSAD2, IFI27, IFIT1, HERC6, IL1R2, OLAH, FAM20A | 0,896 | 0,887 | 0,904 | 0,929 | 0,876 | 0,982 | 0,929 | 0,857 | 1 |
| IFI27, OAS1, HERC6, OLAH, FAM20A, RETN, MMP8 | 0,896 | 0,886 | 0,905 | 0,931 | 0,877 | 0,986 | 0,929 | 0,854 | 1 |
| IFI27, OAS1, IFI44L, ISG15, HERC6, OLAH, FAM20A | 0,896 | 0,886 | 0,905 | 0,928 | 0,873 | 0,983 | 0,930 | 0,859 | 1 |
| RSAD2, IFI27, SIGLEC1, HERC6, FAM20A, RETN, MMP8 | 0,895 | 0,886 | 0,905 | 0,932 | 0,877 | 0,986 | 0,946 | 0,872 | 1 |
| RSAD2, IFI27, IFIT1, IL1R2, OLAH, FAM20A, MMP8 | 0,895 | 0,887 | 0,904 | 0,928 | 0,875 | 0,982 | 0,935 | 0,867 | 1 |
| IFI27, OAS1, IFI44L, SIGLEC1, HERC6, FAM20A, RETN | 0,895 | 0,887 | 0,904 | 0,928 | 0,873 | 0,982 | 0,938 | 0,864 | 1 |
| IFI27, OAS1, SIGLEC1, ISG15, HERC6, FAM20A, RETN | 0,895 | 0,886 | 0,904 | 0,929 | 0,873 | 0,984 | 0,938 | 0,864 | 1 |
| RSAD2, IFI27, IFIT1, IFI44L, SIGLEC1, OLAH, FAM20A | 0,895 | 0,887 | 0,904 | 0,925 | 0,866 | 0,984 | 0,950 | 0,893 | 1 |
| IFI27, IFI44L, SIGLEC1, ISG15, OLAH, FAM20A, RETN | 0,895 | 0,886 | 0,904 | 0,925 | 0,868 | 0,983 | 0,946 | 0,884 | 1 |
| IFI27, SIGLEC1, ISG15, IL1R2, OLAH, FAM20A, RETN | 0,895 | 0,887 | 0,904 | 0,926 | 0,869 | 0,983 | 0,946 | 0,883 | 1 |
| RSAD2, IFI27, SIGLEC1, SLC1A2, IL1R2, FAM20A, MMP8 | 0,895 | 0,886 | 0,904 | 0,935 | 0,889 | 0,981 | 0,954 | 0,886 | 1 |
| RSAD2, IFI27, OAS1, IFIT1, HERC6, OLAH, FAM20A | 0,895 | 0,886 | 0,904 | 0,928 | 0,874 | 0,982 | 0,929 | 0,857 | 1 |
| IFI27, OAS1, SIGLEC1, SLC1A2, IL1R2, FAM20A, MMP8 | 0,895 | 0,886 | 0,904 | 0,935 | 0,889 | 0,982 | 0,950 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, HERC6, OLAH, FAM20A, RETN | 0,895 | 0,886 | 0,904 | 0,931 | 0,879 | 0,983 | 0,933 | 0,862 | 1 |
| IFI27, OAS1, IFI44L, SIGLEC1, ISG15, OLAH, FAM20A | 0,895 | 0,886 | 0,904 | 0,924 | 0,865 | 0,983 | 0,947 | 0,886 | 1 |
| IFI27, IFIT1, SIGLEC1, ISG15, SLC1A2, OLAH, FAM20A | 0,895 | 0,886 | 0,904 | 0,935 | 0,885 | 0,985 | 0,938 | 0,871 | 1 |
| IFI27, IFI44L, SIGLEC1, IL1R2, OLAH, FAM20A, RETN | 0,895 | 0,887 | 0,904 | 0,926 | 0,870 | 0,982 | 0,951 | 0,889 | 1 |
| RSAD2, IFI27, OAS1, IFIT1, SIGLEC1, OLAH, FAM20A | 0,895 | 0,886 | 0,904 | 0,923 | 0,864 | 0,983 | 0,950 | 0,891 | 1 |
| IFI27, OAS1, SIGLEC1, IL1R2, OLAH, FAM20A, RETN | 0,895 | 0,886 | 0,904 | 0,924 | 0,866 | 0,982 | 0,948 | 0,886 | 1 |
| RSAD2, IFI27, OAS1, ISG15, HERC6, OLAH, FAM20A | 0,895 | 0,886 | 0,904 | 0,928 | 0,874 | 0,982 | 0,927 | 0,855 | 1,000 |
| RSAD2, IFI27, IFIT1, SIGLEC1, ISG15, OLAH, FAM20A | 0,895 | 0,886 | 0,904 | 0,926 | 0,868 | 0,985 | 0,941 | 0,879 | 1 |
| IFI27, OAS1, ISG15, IL1R2, OLAH, FAM20A, MMP8 | 0,895 | 0,886 | 0,904 | 0,929 | 0,875 | 0,982 | 0,933 | 0,864 | 1 |
| IFI27, IFI44L, SIGLEC1, HERC6, OLAH, FAM20A, RETN | 0,895 | 0,886 | 0,904 | 0,924 | 0,866 | 0,982 | 0,947 | 0,879 | 1 |
| IFI27, IFIT1, HERC6, OLAH, FAM20A, RETN, MMP8 | 0,895 | 0,885 | 0,904 | 0,928 | 0,871 | 0,984 | 0,938 | 0,870 | 1 |
| RSAD2, IFI27, OAS1, SIGLEC1, IL1R2, OLAH, FAM20A | 0,895 | 0,886 | 0,904 | 0,925 | 0,867 | 0,983 | 0,947 | 0,883 | 1 |
| RSAD2, IFI27, HERC6, IL1R2, OLAH, FAM20A, RETN | 0,895 | 0,886 | 0,904 | 0,929 | 0,877 | 0,982 | 0,928 | 0,855 | 1 |
| IFI27, IFIT1, SIGLEC1, HERC6, SLC1A2, OLAH, FAM20A | 0,895 | 0,885 | 0,904 | 0,934 | 0,884 | 0,984 | 0,945 | 0,882 | 1 |
| IFI27, IFIT1, IFI44L, HERC6, OLAH, FAM20A, RETN | 0,895 | 0,886 | 0,904 | 0,927 | 0,872 | 0,982 | 0,934 | 0,863 | 1 |
| IFI27, HERC6, IL1R2, OLAH, FAM20A, RETN, MMP8 | 0,895 | 0,886 | 0,904 | 0,932 | 0,879 | 0,985 | 0,934 | 0,861 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, SLC1A2, OLAH, FAM20A, MMP8 | 0,895 | 0,885 | 0,904 | 0,933 | 0,880 | 0,985 | 0,947 | 0,884 | 1 |
| IFI27, IFI44L, SIGLEC1, ISG15, IL1R2, FAM20A, RETN | 0,895 | 0,886 | 0,903 | 0,929 | 0,874 | 0,983 | 0,947 | 0,880 | 1 |
| IFI27, OAS1, IFIT1, ISG15, HERC6, OLAH, FAM20A | 0,895 | 0,886 | 0,904 | 0,928 | 0,874 | 0,983 | 0,928 | 0,856 | 1 |
| IFI27, IFIT1, SIGLEC1, ISG15, IL1R2, FAM20A, RETN | 0,895 | 0,886 | 0,903 | 0,927 | 0,873 | 0,982 | 0,948 | 0,883 | 1 |
| IFI27, SIGLEC1, ISG15, HERC6, OLAH, FAM20A, RETN | 0,895 | 0,885 | 0,904 | 0,922 | 0,863 | 0,982 | 0,946 | 0,884 | 1 |
| IFI27, IFIT1, IFI44L, SIGLEC1, SLC1A2, OLAH, FAM20A | 0,895 | 0,886 | 0,903 | 0,931 | 0,878 | 0,984 | 0,943 | 0,879 | 1 |
| RSAD2, IFI27, IFI44L, IL1R2, OLAH, FAM20A, MMP8 | 0,895 | 0,885 | 0,904 | 0,928 | 0,873 | 0,983 | 0,932 | 0,859 | 1 |
| IFI27, IFI44L, IL1R2, OLAH, FAM20A, RETN, MMP8 | 0,895 | 0,885 | 0,904 | 0,930 | 0,876 | 0,984 | 0,933 | 0,863 | 1 |
| RSAD2, IFI27, SIGLEC1, ISG15, IL1R2, OLAH, FAM20A | 0,894 | 0,886 | 0,903 | 0,926 | 0,869 | 0,983 | 0,943 | 0,879 | 1 |
| RSAD2, IFI27, IFI44L, ISG15, HERC6, OLAH, FAM20A | 0,894 | 0,885 | 0,904 | 0,930 | 0,876 | 0,984 | 0,928 | 0,858 | 0,999 |
| IFI27, ISG15, HERC6, IL1R2, OLAH, FAM20A, RETN | 0,894 | 0,886 | 0,903 | 0,928 | 0,874 | 0,982 | 0,932 | 0,860 | 1 |
| IFI27, OAS1, ISG15, HERC6, OLAH, FAM20A, RETN | 0,894 | 0,885 | 0,904 | 0,930 | 0,878 | 0,983 | 0,935 | 0,866 | 1 |
| IFI27, OAS1, SIGLEC1, HERC6, SLC1A2, OLAH, FAM20A | 0,894 | 0,885 | 0,904 | 0,931 | 0,880 | 0,983 | 0,935 | 0,866 | 1 |
| IFI27, IFI44L, SIGLEC1, SLC1A2, OLAH, FAM20A, MMP8 | 0,894 | 0,885 | 0,903 | 0,927 | 0,873 | 0,982 | 0,953 | 0,892 | 1 |
| RSAD2, IFI27, IFI44L, SIGLEC1, IL1R2, OLAH, FAM20A | 0,894 | 0,886 | 0,903 | 0,926 | 0,870 | 0,983 | 0,950 | 0,888 | 1 |
| IFI27, OAS1, IFIT1, SIGLEC1, IL1R2, FAM20A, RETN | 0,894 | 0,886 | 0,903 | 0,927 | 0,872 | 0,983 | 0,949 | 0,887 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, ISG15, HERC6, OLAH, FAM20A, RETN | 0,894 | 0,885 | 0,903 | 0,926 | 0,871 | 0,981 | 0,933 | 0,863 | 1 |
| RSAD2, IFI27, OAS1, SIGLEC1, ISG15, OLAH, FAM20A | 0,894 | 0,885 | 0,903 | 0,924 | 0,865 | 0,983 | 0,946 | 0,886 | 1 |
| IFI27, OAS1, HERC6, IL1R2, OLAH, FAM20A, RETN | 0,894 | 0,885 | 0,903 | 0,931 | 0,879 | 0,982 | 0,929 | 0,855 | 1 |
| IFI27, OAS1, IFI44L, SIGLEC1, ISG15, IL1R2, FAM20A | 0,894 | 0,886 | 0,903 | 0,929 | 0,876 | 0,982 | 0,948 | 0,883 | 1 |
| IFI27, OAS1, IFIT1, SIGLEC1, ISG15, IL1R2, FAM20A | 0,894 | 0,886 | 0,903 | 0,927 | 0,871 | 0,982 | 0,949 | 0,886 | 1 |
| IFI27, OAS1, IFIT1, HERC6, IL1R2, FAM20A, MMP8 | 0,894 | 0,885 | 0,903 | 0,930 | 0,879 | 0,980 | 0,929 | 0,848 | 1 |
| RSAD2, IFI27, ISG15, IL1R2, OLAH, FAM20A, MMP8 | 0,894 | 0,885 | 0,903 | 0,929 | 0,876 | 0,982 | 0,927 | 0,854 | 1,000 |
| IFI27, IFI44L, SIGLEC1, SLC1A2, IL1R2, FAM20A, MMP8 | 0,894 | 0,886 | 0,903 | 0,935 | 0,890 | 0,981 | 0,953 | 0,885 | 1 |
| IFI27, OAS1, IFI44L, HERC6, OLAH, FAM20A, RETN | 0,894 | 0,885 | 0,903 | 0,930 | 0,876 | 0,983 | 0,934 | 0,864 | 1 |
| IFI27, SIGLEC1, ISG15, SLC1A2, IL1R2, FAM20A, MMP8 | 0,894 | 0,885 | 0,903 | 0,937 | 0,894 | 0,980 | 0,955 | 0,885 | 1 |
| IFI27, IFIT1, SIGLEC1, SLC1A2, IL1R2, FAM20A, MMP8 | 0,894 | 0,885 | 0,903 | 0,936 | 0,891 | 0,981 | 0,954 | 0,886 | 1 |
| RSAD2, IFI27, OAS1, SIGLEC1, HERC6, FAM20A, RETN | 0,894 | 0,885 | 0,903 | 0,929 | 0,874 | 0,984 | 0,938 | 0,864 | 1 |
| IFI27, SIGLEC1, SLC1A2, IL1R2, OLAH, FAM20A, MMP8 | 0,894 | 0,885 | 0,903 | 0,933 | 0,885 | 0,982 | 0,947 | 0,878 | 1 |
| IFI27, OAS1, IFI44L, IL1R2, OLAH, FAM20A, MMP8 | 0,894 | 0,884 | 0,903 | 0,929 | 0,874 | 0,983 | 0,932 | 0,859 | 1 |
| IFI27, ISG15, IL1R2, OLAH, FAM20A, RETN, MMP8 | 0,894 | 0,885 | 0,903 | 0,929 | 0,875 | 0,982 | 0,925 | 0,849 | 1 |
| RSAD2, IFI27, SIGLEC1, IL1R2, OLAH, FAM20A, RETN | 0,894 | 0,885 | 0,903 | 0,927 | 0,869 | 0,984 | 0,948 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, ISG15, HERC6, OLAH, FAM20A, RETN | 0,894 | 0,884 | 0,903 | 0,925 | 0,869 | 0,981 | 0,935 | 0,866 | 1 |
| IFI27, OAS1, IFI44L, HERC6, IL1R2, FAM20A, MMP8 | 0,894 | 0,885 | 0,903 | 0,930 | 0,879 | 0,980 | 0,926 | 0,840 | 1 |
| RSAD2, IFI27, OAS1, IFI44L, SIGLEC1, OLAH, FAM20A | 0,894 | 0,885 | 0,903 | 0,924 | 0,865 | 0,983 | 0,950 | 0,891 | 1 |
| IFI27, IFI44L, ISG15, SLC1A2, OLAH, FAM20A, MMP8 | 0,894 | 0,884 | 0,903 | 0,929 | 0,876 | 0,981 | 0,923 | 0,846 | 1,000 |
| IFI27, IFI44L, SLC1A2, IL1R2, OLAH, FAM20A, MMP8 | 0,894 | 0,885 | 0,902 | 0,932 | 0,882 | 0,982 | 0,924 | 0,844 | 1 |
| RSAD2, IFI27, IFIT1, IFI44L, SIGLEC1, IL1R2, FAM20A | 0,894 | 0,885 | 0,902 | 0,927 | 0,872 | 0,982 | 0,950 | 0,887 | 1 |
| RSAD2, IFI27, SIGLEC1, ISG15, HERC6, FAM20A, RETN | 0,894 | 0,884 | 0,903 | 0,930 | 0,876 | 0,983 | 0,946 | 0,876 | 1 |
| IFI27, OAS1, HERC6, IL1R2, FAM20A, RETN, MMP8 | 0,894 | 0,885 | 0,902 | 0,932 | 0,880 | 0,983 | 0,929 | 0,847 | 1 |
| IFI27, OAS1, SIGLEC1, ISG15, OLAH, FAM20A, RETN | 0,894 | 0,884 | 0,903 | 0,923 | 0,863 | 0,983 | 0,947 | 0,886 | 1 |
| IFI27, IFIT1, IFI44L, SIGLEC1, ISG15, FAM20A, RETN | 0,893 | 0,885 | 0,902 | 0,920 | 0,861 | 0,980 | 0,945 | 0,876 | 1 |
| RSAD2, IFI27, OAS1, HERC6, OLAH, FAM20A, RETN | 0,893 | 0,884 | 0,903 | 0,931 | 0,879 | 0,983 | 0,934 | 0,864 | 1 |
| IFI27, IFIT1, IFI44L, HERC6, IL1R2, FAM20A, MMP8 | 0,893 | 0,885 | 0,902 | 0,930 | 0,879 | 0,980 | 0,934 | 0,853 | 1 |
| RSAD2, IFI27, IFI44L, SIGLEC1, ISG15, OLAH, FAM20A | 0,893 | 0,884 | 0,902 | 0,924 | 0,865 | 0,982 | 0,943 | 0,881 | 1 |
| RSAD2, IFI27, IFI44L, HERC6, IL1R2, FAM20A, MMP8 | 0,893 | 0,885 | 0,902 | 0,928 | 0,876 | 0,979 | 0,926 | 0,840 | 1 |
| RSAD2, IFI27, IFIT1, SIGLEC1, OLAH, FAM20A, RETN | 0,893 | 0,884 | 0,902 | 0,923 | 0,864 | 0,983 | 0,948 | 0,887 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, SLC1A2, OLAH, FAM20A | 0,893 | 0,885 | 0,902 | 0,931 | 0,880 | 0,983 | 0,939 | 0,871 | 1 |
| RSAD2, IFI27, IFIT1, HERC6, OLAH, FAM20A, RETN | 0,893 | 0,884 | 0,902 | 0,929 | 0,876 | 0,982 | 0,935 | 0,867 | 1 |
| RSAD2, IFI27, HERC6, IL1R2, FAM20A, RETN, MMP8 | 0,893 | 0,884 | 0,902 | 0,932 | 0,881 | 0,983 | 0,932 | 0,851 | 1 |
| RSAD2, IFI27, OAS1, IFI44L, SIGLEC1, IL1R2, FAM20A | 0,893 | 0,885 | 0,902 | 0,927 | 0,873 | 0,982 | 0,949 | 0,885 | 1 |
| RSAD2, IFI27, IFIT1, ISG15, HERC6, OLAH, FAM20A | 0,893 | 0,884 | 0,903 | 0,928 | 0,873 | 0,982 | 0,929 | 0,859 | 1,000 |
| IFI27, IFI44L, ISG15, HERC6, IL1R2, FAM20A, MMP8 | 0,893 | 0,884 | 0,902 | 0,930 | 0,880 | 0,980 | 0,927 | 0,841 | 1 |
| IFI27, SIGLEC1, HERC6, OLAH, FAM20A, RETN, MMP8 | 0,893 | 0,883 | 0,904 | 0,925 | 0,867 | 0,984 | 0,953 | 0,895 | 1 |
| IFI27, IFIT1, IFI44L, ISG15, OLAH, FAM20A, MMP8 | 0,893 | 0,884 | 0,902 | 0,921 | 0,861 | 0,981 | 0,936 | 0,870 | 1 |
| RSAD2, IFI27, ISG15, HERC6, OLAH, FAM20A, RETN | 0,893 | 0,884 | 0,902 | 0,929 | 0,875 | 0,982 | 0,935 | 0,867 | 1 |
| IFI27, OAS1, IFI44L, SIGLEC1, IL1R2, FAM20A, RETN | 0,893 | 0,884 | 0,902 | 0,929 | 0,876 | 0,983 | 0,948 | 0,883 | 1 |
| RSAD2, IFI27, IFI44L, HERC6, OLAH, FAM20A, RETN | 0,893 | 0,884 | 0,902 | 0,930 | 0,877 | 0,983 | 0,932 | 0,862 | 1 |
| IFI27, IFIT1, IL1R2, OLAH, FAM20A, RETN, MMP8 | 0,893 | 0,884 | 0,902 | 0,929 | 0,875 | 0,983 | 0,933 | 0,863 | 1 |
| RSAD2, IFI27, OAS1, IFIT1, SIGLEC1, IL1R2, FAM20A | 0,893 | 0,884 | 0,901 | 0,927 | 0,872 | 0,982 | 0,951 | 0,890 | 1 |
| RSAD2, IFI27, OAS1, SIGLEC1, ISG15, IL1R2, FAM20A | 0,893 | 0,884 | 0,902 | 0,927 | 0,872 | 0,983 | 0,949 | 0,882 | 1 |
| RSAD2, IFI27, SIGLEC1, ISG15, OLAH, FAM20A, RETN | 0,893 | 0,884 | 0,902 | 0,924 | 0,865 | 0,983 | 0,945 | 0,883 | 1 |
| IFI27, OAS1, SIGLEC1, ISG15, IL1R2, FAM20A, RETN | 0,893 | 0,884 | 0,902 | 0,928 | 0,872 | 0,983 | 0,949 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, ISG15, SLC1A2, OLAH, FAM20A | 0,893 | 0,883 | 0,902 | 0,933 | 0,882 | 0,985 | 0,937 | 0,869 | 1 |
| IFI27, IFI44L, ISG15, OLAH, FAM20A, RETN, MMP8 | 0,893 | 0,883 | 0,902 | 0,924 | 0,865 | 0,983 | 0,940 | 0,874 | 1 |
| IFI27, IFIT1, SIGLEC1, SLC1A2, IL1R2, OLAH, FAM20A | 0,893 | 0,884 | 0,901 | 0,933 | 0,882 | 0,983 | 0,943 | 0,879 | 1 |
| IFI27, IFI44L, HERC6, OLAH, FAM20A, RETN, MMP8 | 0,893 | 0,883 | 0,903 | 0,926 | 0,869 | 0,984 | 0,941 | 0,874 | 1 |
| IFI27, IFIT1, HERC6, IL1R2, OLAH, FAM20A, RETN | 0,893 | 0,884 | 0,902 | 0,929 | 0,875 | 0,982 | 0,929 | 0,855 | 1 |
| IFI27, OAS1, IFIT1, IFI44L, HERC6, IL1R2, FAM20A | 0,893 | 0,884 | 0,901 | 0,927 | 0,875 | 0,978 | 0,927 | 0,844 | 1 |
| IFI27, OAS1, IFI44L, ISG15, HERC6, IL1R2, FAM20A | 0,893 | 0,884 | 0,901 | 0,927 | 0,876 | 0,979 | 0,925 | 0,840 | 1 |
| RSAD2, IFI27, IFIT1, HERC6, IL1R2, FAM20A, MMP8 | 0,892 | 0,884 | 0,901 | 0,930 | 0,879 | 0,980 | 0,933 | 0,852 | 1 |
| RSAD2, IFI27, OAS1, SIGLEC1, OLAH, FAM20A, RETN | 0,892 | 0,883 | 0,902 | 0,923 | 0,863 | 0,984 | 0,950 | 0,891 | 1 |
| IFI27, OAS1, IFIT1, SIGLEC1, HERC6, OLAH, RETN | 0,892 | 0,884 | 0,901 | 0,925 | 0,872 | 0,978 | 0,947 | 0,893 | 1 |
| IFI27, ISG15, HERC6, IL1R2, FAM20A, RETN, MMP8 | 0,892 | 0,883 | 0,902 | 0,931 | 0,880 | 0,983 | 0,932 | 0,851 | 1 |
| IFI27, OAS1, HERC6, SLC1A2, OLAH, FAM20A, MMP8 | 0,892 | 0,883 | 0,902 | 0,930 | 0,879 | 0,980 | 0,921 | 0,839 | 1 |
| RSAD2, IFI27, IFIT1, SIGLEC1, IL1R2, FAM20A, RETN | 0,892 | 0,884 | 0,901 | 0,927 | 0,871 | 0,982 | 0,949 | 0,886 | 1 |
| IFI27, IFI44L, HERC6, IL1R2, OLAH, FAM20A, RETN | 0,892 | 0,883 | 0,902 | 0,927 | 0,873 | 0,981 | 0,934 | 0,862 | 1 |
| RSAD2, IFI27, IFIT1, SIGLEC1, ISG15, IL1R2, FAM20A | 0,892 | 0,884 | 0,901 | 0,928 | 0,873 | 0,983 | 0,949 | 0,884 | 1 |
| IFI27, OAS1, ISG15, HERC6, IL1R2, FAM20A, MMP8 | 0,892 | 0,884 | 0,901 | 0,929 | 0,879 | 0,979 | 0,923 | 0,835 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, HERC6, SLC1A2, OLAH, FAM20A, MMP8 | 0,892 | 0,883 | 0,902 | 0,931 | 0,881 | 0,982 | 0,923 | 0,843 | 1 |
| IFI27, OAS1, IL1R2, OLAH, FAM20A, RETN, MMP8 | 0,892 | 0,883 | 0,901 | 0,929 | 0,875 | 0,984 | 0,930 | 0,858 | 1 |
| IFI27, OAS1, IFI44L, SIGLEC1, OLAH, FAM20A, RETN | 0,892 | 0,883 | 0,901 | 0,923 | 0,864 | 0,983 | 0,950 | 0,891 | 1 |
| RSAD2, IFI27, SIGLEC1, ISG15, IL1R2, FAM20A, RETN | 0,892 | 0,883 | 0,901 | 0,928 | 0,873 | 0,983 | 0,946 | 0,878 | 1 |
| IFI27, OAS1, IFIT1, SIGLEC1, SLC1A2, OLAH, FAM20A | 0,892 | 0,883 | 0,902 | 0,932 | 0,878 | 0,985 | 0,943 | 0,879 | 1 |
| RSAD2, IFI27, IL1R2, OLAH, FAM20A, RETN, MMP8 | 0,892 | 0,883 | 0,901 | 0,930 | 0,876 | 0,983 | 0,930 | 0,858 | 1 |
| IFI27, OAS1, SIGLEC1, HERC6, OLAH, RETN, MMP8 | 0,892 | 0,883 | 0,901 | 0,926 | 0,871 | 0,981 | 0,957 | 0,905 | 1 |
| RSAD2, IFI27, IFI44L, SIGLEC1, ISG15, IL1R2, FAM20A | 0,892 | 0,884 | 0,901 | 0,928 | 0,874 | 0,982 | 0,947 | 0,880 | 1 |
| IFI27, IFI44L, SIGLEC1, ISG15, HERC6, FAM20A, RETN | 0,892 | 0,883 | 0,901 | 0,923 | 0,866 | 0,980 | 0,942 | 0,866 | 1 |
| RSAD2, IFI27, OAS1, SIGLEC1, IL1R2, FAM20A, RETN | 0,892 | 0,883 | 0,901 | 0,928 | 0,872 | 0,984 | 0,950 | 0,886 | 1 |
| RSAD2, IFI27, IFI44L, SIGLEC1, HERC6, FAM20A, RETN | 0,892 | 0,883 | 0,901 | 0,928 | 0,874 | 0,982 | 0,939 | 0,864 | 1 |
| RSAD2, IFI27, OAS1, HERC6, IL1R2, FAM20A, MMP8 | 0,892 | 0,883 | 0,901 | 0,931 | 0,881 | 0,980 | 0,923 | 0,835 | 1 |
| RSAD2, IFI27, SIGLEC1, ISG15, SLC1A2, OLAH, FAM20A | 0,892 | 0,883 | 0,901 | 0,929 | 0,876 | 0,982 | 0,936 | 0,866 | 1 |
| RSAD2, IFI27, OAS1, IFI44L, HERC6, IL1R2, FAM20A | 0,892 | 0,884 | 0,901 | 0,927 | 0,876 | 0,978 | 0,926 | 0,842 | 1 |
| IFI27, OAS1, IFI44L, HERC6, IL1R2, FAM20A, RETN | 0,892 | 0,883 | 0,901 | 0,932 | 0,882 | 0,982 | 0,929 | 0,848 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, HERC6, IL1R2, FAM20A, RETN, MMP8 | 0,892 | 0,883 | 0,901 | 0,931 | 0,879 | 0,983 | 0,936 | 0,857 | 1 |
| IFI27, SIGLEC1, ISG15, HERC6, IL1R2, FAM20A, RETN | 0,892 | 0,883 | 0,901 | 0,928 | 0,874 | 0,983 | 0,949 | 0,881 | 1 |
| IFI27, SIGLEC1, ISG15, HERC6, SLC1A2, OLAH, FAM20A | 0,892 | 0,882 | 0,902 | 0,931 | 0,879 | 0,983 | 0,939 | 0,872 | 1 |
| IFI27, OAS1, SIGLEC1, HERC6, SLC1A2, IL1R2, FAM20A | 0,892 | 0,883 | 0,901 | 0,933 | 0,886 | 0,980 | 0,943 | 0,874 | 1 |
| RSAD2, IFI27, IFIT1, IFI44L, HERC6, IL1R2, FAM20A | 0,892 | 0,884 | 0,900 | 0,927 | 0,875 | 0,978 | 0,930 | 0,849 | 1 |
| IFI27, OAS1, ISG15, HERC6, IL1R2, FAM20A, RETN | 0,892 | 0,883 | 0,901 | 0,932 | 0,882 | 0,982 | 0,925 | 0,842 | 1 |
| IFI27, IFIT1, SIGLEC1, ISG15, FAM20A, RETN, MMP8 | 0,892 | 0,882 | 0,901 | 0,923 | 0,864 | 0,983 | 0,946 | 0,872 | 1 |
| IFI27, IFIT1, IFI44L, HERC6, IL1R2, FAM20A, RETN | 0,892 | 0,883 | 0,900 | 0,931 | 0,880 | 0,982 | 0,929 | 0,849 | 1 |
| IFI27, ISG15, SLC1A2, IL1R2, OLAH, FAM20A, MMP8 | 0,892 | 0,883 | 0,900 | 0,931 | 0,883 | 0,979 | 0,920 | 0,838 | 1 |
| RSAD2, IFI27, OAS1, ISG15, OLAH, FAM20A, MMP8 | 0,892 | 0,882 | 0,901 | 0,921 | 0,863 | 0,980 | 0,933 | 0,866 | 1,000 |
| IFI27, SIGLEC1, HERC6, SLC1A2, OLAH, FAM20A, MMP8 | 0,892 | 0,882 | 0,902 | 0,927 | 0,871 | 0,983 | 0,952 | 0,891 | 1 |
| IFI27, IFI44L, ISG15, IL1R2, OLAH, FAM20A, RETN | 0,892 | 0,883 | 0,901 | 0,923 | 0,865 | 0,980 | 0,930 | 0,859 | 1 |
| IFI27, OAS1, IFIT1, HERC6, IL1R2, FAM20A, RETN | 0,892 | 0,883 | 0,901 | 0,931 | 0,880 | 0,982 | 0,929 | 0,848 | 1 |
| RSAD2, IFI27, IFI44L, ISG15, HERC6, IL1R2, FAM20A | 0,892 | 0,883 | 0,900 | 0,928 | 0,877 | 0,979 | 0,928 | 0,845 | 1 |
| IFI27, IFIT1, HERC6, IL1R2, FAM20A, RETN, MMP8 | 0,892 | 0,882 | 0,901 | 0,931 | 0,878 | 0,983 | 0,936 | 0,857 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, HERC6, IL1R2, RETN, MMP8 | 0,892 | 0,883 | 0,900 | 0,924 | 0,871 | 0,978 | 0,959 | 0,911 | 1 |
| IFI27, IFIT1, IFI44L, SIGLEC1, ISG15, HERC6, FAM20A | 0,892 | 0,883 | 0,900 | 0,920 | 0,860 | 0,980 | 0,942 | 0,867 | 1 |
| IFI27, OAS1, IFIT1, SIGLEC1, ISG15, HERC6, RETN | 0,892 | 0,883 | 0,900 | 0,920 | 0,863 | 0,976 | 0,942 | 0,884 | 1 |
| IFI27, OAS1, IFIT1, ISG15, OLAH, FAM20A, MMP8 | 0,892 | 0,882 | 0,901 | 0,921 | 0,861 | 0,981 | 0,936 | 0,871 | 1 |
| RSAD2, IFI27, OAS1, IFI44L, OLAH, FAM20A, MMP8 | 0,892 | 0,882 | 0,902 | 0,923 | 0,864 | 0,982 | 0,935 | 0,869 | 1 |
| RSAD2, IFI27, OAS1, HERC6, IL1R2, FAM20A, RETN | 0,892 | 0,883 | 0,900 | 0,930 | 0,879 | 0,981 | 0,929 | 0,848 | 1 |
| IFIT1, IFI44L, SIGLEC1, HERC6, IL1R2, FAM20A, MMP8 | 0,892 | 0,883 | 0,900 | 0,924 | 0,870 | 0,978 | 0,962 | 0,906 | 1 |
| IFI27, OAS1, SIGLEC1, SLC1A2, IL1R2, OLAH, FAM20A | 0,891 | 0,882 | 0,900 | 0,930 | 0,877 | 0,982 | 0,941 | 0,876 | 1 |
| IFI27, SIGLEC1, HERC6, SLC1A2, IL1R2, FAM20A, MMP8 | 0,891 | 0,882 | 0,901 | 0,932 | 0,884 | 0,980 | 0,955 | 0,887 | 1 |
| IFI27, IFIT1, IFI44L, SIGLEC1, FAM20A, RETN, MMP8 | 0,891 | 0,882 | 0,901 | 0,923 | 0,864 | 0,982 | 0,949 | 0,878 | 1 |
| IFI27, OAS1, IFI44L, ISG15, OLAH, FAM20A, MMP8 | 0,891 | 0,882 | 0,901 | 0,921 | 0,862 | 0,981 | 0,936 | 0,870 | 1 |
| OAS1, IFIT1, SIGLEC1, HERC6, IL1R2, FAM20A, MMP8 | 0,891 | 0,883 | 0,900 | 0,923 | 0,868 | 0,977 | 0,962 | 0,906 | 1 |
| IFI27, SIGLEC1, ISG15, SLC1A2, IL1R2, OLAH, FAM20A | 0,891 | 0,882 | 0,900 | 0,933 | 0,884 | 0,982 | 0,938 | 0,870 | 1 |
| IFI27, IFI44L, SIGLEC1, HERC6, FAM20A, RETN, MMP8 | 0,891 | 0,882 | 0,901 | 0,924 | 0,866 | 0,982 | 0,950 | 0,877 | 1 |
| IFI27, OAS1, IFIT1, SIGLEC1, FAM20A, RETN, MMP8 | 0,891 | 0,882 | 0,901 | 0,923 | 0,864 | 0,982 | 0,948 | 0,879 | 1 |
| IFI27, IFI44L, ISG15, HERC6, IL1R2, FAM20A, RETN | 0,891 | 0,882 | 0,900 | 0,931 | 0,880 | 0,982 | 0,929 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, HERC6, OLAH, RETN, MMP8 | 0,891 | 0,882 | 0,900 | 0,924 | 0,868 | 0,980 | 0,953 | 0,902 | 1 |
| RSAD2, IFI27, SIGLEC1, HERC6, SLC1A2, OLAH, FAM20A | 0,891 | 0,882 | 0,901 | 0,931 | 0,878 | 0,983 | 0,938 | 0,870 | 1 |
| RSAD2, IFI27, OAS1, IFIT1, OLAH, FAM20A, MMP8 | 0,891 | 0,882 | 0,901 | 0,922 | 0,863 | 0,981 | 0,939 | 0,876 | 1 |
| IFI27, SIGLEC1, HERC6, IL1R2, OLAH, FAM20A, RETN | 0,891 | 0,882 | 0,901 | 0,925 | 0,868 | 0,982 | 0,949 | 0,886 | 1 |
| IFI27, OAS1, IFIT1, HERC6, OLAH, RETN, MMP8 | 0,891 | 0,882 | 0,900 | 0,924 | 0,867 | 0,980 | 0,945 | 0,886 | 1 |
| IFI27, IFIT1, SIGLEC1, HERC6, OLAH, RETN, MMP8 | 0,891 | 0,882 | 0,900 | 0,926 | 0,870 | 0,981 | 0,957 | 0,909 | 1 |
| IFI27, IFIT1, SIGLEC1, HERC6, SLC1A2, IL1R2, FAM20A | 0,891 | 0,882 | 0,900 | 0,936 | 0,890 | 0,981 | 0,946 | 0,876 | 1 |
| IFI27, IFIT1, IFI44L, ISG15, HERC6, IL1R2, FAM20A | 0,891 | 0,883 | 0,900 | 0,927 | 0,876 | 0,978 | 0,932 | 0,851 | 1 |
| IFI27, IFIT1, SLC1A2, IL1R2, OLAH, FAM20A, MMP8 | 0,891 | 0,882 | 0,900 | 0,931 | 0,881 | 0,981 | 0,923 | 0,842 | 1 |
| IFI27, IFI44L, SIGLEC1, ISG15, SLC1A2, IL1R2, FAM20A | 0,891 | 0,883 | 0,900 | 0,932 | 0,884 | 0,979 | 0,947 | 0,877 | 1 |
| IFI27, OAS1, IFIT1, IFI44L, OLAH, FAM20A, MMP8 | 0,891 | 0,882 | 0,901 | 0,921 | 0,861 | 0,981 | 0,937 | 0,871 | 1 |
| IFI27, IFIT1, IFI44L, SIGLEC1, HERC6, OLAH, RETN | 0,891 | 0,883 | 0,900 | 0,926 | 0,872 | 0,979 | 0,946 | 0,893 | 0,998 |
| IFI27, SIGLEC1, SLC1A2, IL1R2, FAM20A, RETN, MMP8 | 0,891 | 0,882 | 0,900 | 0,935 | 0,886 | 0,983 | 0,955 | 0,888 | 1 |
| IFI27, IFIT1, IFI44L, ISG15, IL1R2, OLAH, FAM20A | 0,891 | 0,883 | 0,900 | 0,924 | 0,867 | 0,981 | 0,932 | 0,861 | 1 |
| RSAD2, IFI27, ISG15, HERC6, IL1R2, FAM20A, MMP8 | 0,891 | 0,882 | 0,900 | 0,929 | 0,879 | 0,979 | 0,926 | 0,841 | 1 |
| IFI27, OAS1, IFIT1, ISG15, HERC6, IL1R2, FAM20A | 0,891 | 0,882 | 0,900 | 0,928 | 0,877 | 0,979 | 0,925 | 0,842 | 1 |
| IFI27, OAS1, IFIT1, IFI44L, SIGLEC1, FAM20A, RETN | 0,891 | 0,882 | 0,900 | 0,919 | 0,858 | 0,979 | 0,946 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, SLC1A2, IL1R2, OLAH, FAM20A | 0,891 | 0,882 | 0,900 | 0,930 | 0,878 | 0,982 | 0,947 | 0,882 | 1 |
| RSAD2, IFI27, IFI44L, SIGLEC1, IL1R2, FAM20A, RETN | 0,891 | 0,882 | 0,900 | 0,929 | 0,874 | 0,983 | 0,950 | 0,886 | 1 |
| RSAD2, IFI27, IFIT1, SIGLEC1, SLC1A2, OLAH, FAM20A | 0,891 | 0,882 | 0,900 | 0,930 | 0,877 | 0,983 | 0,942 | 0,878 | 1 |
| IFI27, IFIT1, IFI44L, SLC1A2, OLAH, FAM20A, MMP8 | 0,891 | 0,882 | 0,900 | 0,927 | 0,872 | 0,981 | 0,928 | 0,852 | 1 |
| IFI27, OAS1, ISG15, HERC6, SLC1A2, OLAH, FAM20A | 0,891 | 0,881 | 0,901 | 0,933 | 0,884 | 0,982 | 0,912 | 0,825 | 0,999 |
| IFI27, OAS1, IFIT1, SIGLEC1, HERC6, IL1R2, RETN | 0,891 | 0,883 | 0,899 | 0,925 | 0,873 | 0,977 | 0,945 | 0,889 | 1,000 |
| RSAD2, IFI27, IFIT1, IFI44L, OLAH, FAM20A, MMP8 | 0,891 | 0,881 | 0,900 | 0,922 | 0,862 | 0,982 | 0,936 | 0,870 | 1 |
| RSAD2, IFI27, OAS1, ISG15, IL1R2, OLAH, FAM20A | 0,891 | 0,882 | 0,900 | 0,924 | 0,869 | 0,980 | 0,926 | 0,853 | 0,999 |
| RSAD2, IFI27, OAS1, IFIT1, HERC6, IL1R2, FAM20A | 0,891 | 0,882 | 0,900 | 0,929 | 0,878 | 0,979 | 0,925 | 0,842 | 1 |
| IFI27, OAS1, IFI44L, SIGLEC1, SLC1A2, OLAH, FAM20A | 0,891 | 0,882 | 0,900 | 0,928 | 0,873 | 0,984 | 0,941 | 0,878 | 1 |
| IFI27, IFIT1, SIGLEC1, SLC1A2, OLAH, FAM20A, RETN | 0,891 | 0,882 | 0,900 | 0,929 | 0,875 | 0,984 | 0,943 | 0,879 | 1 |
| RSAD2, IFI27, OAS1, HERC6, OLAH, RETN, MMP8 | 0,891 | 0,882 | 0,900 | 0,923 | 0,867 | 0,980 | 0,945 | 0,886 | 1 |
| RSAD2, IFI27, OAS1, IFIT1, SIGLEC1, HERC6, RETN | 0,891 | 0,882 | 0,899 | 0,919 | 0,860 | 0,978 | 0,947 | 0,889 | 1 |
| IFI27, IFI44L, SIGLEC1, HERC6, IL1R2, OLAH, MMP8 | 0,891 | 0,883 | 0,899 | 0,926 | 0,878 | 0,975 | 0,943 | 0,892 | 0,995 |
| RSAD2, IFI27, IFIT1, IFI44L, SIGLEC1, HERC6, FAM20A | 0,891 | 0,882 | 0,899 | 0,923 | 0,865 | 0,981 | 0,942 | 0,872 | 1 |
| IFI27, OAS1, IFI44L, ISG15, IL1R2, OLAH, FAM20A | 0,891 | 0,882 | 0,900 | 0,924 | 0,867 | 0,981 | 0,928 | 0,856 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, ISG15, HERC6, SLC1A2, OLAH, FAM20A, MMP8 | 0,891 | 0,881 | 0,900 | 0,931 | 0,881 | 0,980 | 0,918 | 0,835 | 1 |
| RSAD2, IFI27, SLC1A2, IL1R2, OLAH, FAM20A, MMP8 | 0,891 | 0,882 | 0,899 | 0,930 | 0,879 | 0,980 | 0,922 | 0,841 | 1 |
| RSAD2, IFI27, IFI44L, ISG15, OLAH, FAM20A, MMP8 | 0,891 | 0,881 | 0,901 | 0,923 | 0,864 | 0,982 | 0,934 | 0,867 | 1 |
| IFI27, OAS1, IFIT1, ISG15, IL1R2, OLAH, FAM20A | 0,891 | 0,882 | 0,899 | 0,924 | 0,868 | 0,981 | 0,929 | 0,859 | 1,000 |
| IFI27, OAS1, IFIT1, IFI44L, IL1R2, OLAH, FAM20A | 0,891 | 0,882 | 0,899 | 0,925 | 0,867 | 0,982 | 0,932 | 0,860 | 1 |
| IFI27, IFIT1, IFI44L, OLAH, FAM20A, RETN, MMP8 | 0,891 | 0,881 | 0,900 | 0,924 | 0,864 | 0,983 | 0,941 | 0,875 | 1 |
| RSAD2, IFI27, OAS1, HERC6, SLC1A2, OLAH, FAM20A | 0,891 | 0,881 | 0,900 | 0,930 | 0,879 | 0,980 | 0,922 | 0,840 | 1 |
| IFI27, OAS1, IFI44L, SIGLEC1, HERC6, OLAH, RETN | 0,891 | 0,882 | 0,899 | 0,924 | 0,872 | 0,977 | 0,941 | 0,884 | 0,999 |
| RSAD2, IFI27, OAS1, ISG15, HERC6, IL1R2, FAM20A | 0,891 | 0,882 | 0,899 | 0,929 | 0,879 | 0,978 | 0,922 | 0,836 | 1 |
| IFIT1, SIGLEC1, HERC6, IL1R2, OLAH, FAM20A, MMP8 | 0,891 | 0,882 | 0,899 | 0,924 | 0,870 | 0,978 | 0,962 | 0,906 | 1 |
| RSAD2, IFI27, SIGLEC1, HERC6, OLAH, RETN, MMP8 | 0,891 | 0,882 | 0,900 | 0,924 | 0,869 | 0,980 | 0,955 | 0,906 | 1 |
| IFI27, IFIT1, IFI44L, SIGLEC1, SLC1A2, IL1R2, FAM20A | 0,891 | 0,882 | 0,899 | 0,934 | 0,887 | 0,981 | 0,943 | 0,874 | 1 |
| IFI27, IFIT1, ISG15, SLC1A2, OLAH, FAM20A, MMP8 | 0,891 | 0,881 | 0,900 | 0,926 | 0,872 | 0,981 | 0,924 | 0,847 | 1 |
| IFI27, OAS1, IFIT1, SIGLEC1, ISG15, FAM20A, RETN | 0,891 | 0,881 | 0,900 | 0,920 | 0,861 | 0,980 | 0,943 | 0,875 | 1 |
| RSAD2, IFI27, HERC6, SLC1A2, IL1R2, OLAH, FAM20A | 0,891 | 0,882 | 0,899 | 0,930 | 0,880 | 0,980 | 0,922 | 0,841 | 1 |
| IFI27, OAS1, IFIT1, SIGLEC1, HERC6, FAM20A, MMP8 | 0,890 | 0,882 | 0,899 | 0,923 | 0,866 | 0,980 | 0,938 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, IL1R2, FAM20A, RETN, MMP8 | 0,890 | 0,881 | 0,900 | 0,929 | 0,875 | 0,982 | 0,937 | 0,860 | 1 |
| IFI27, IFIT1, ISG15, OLAH, FAM20A, RETN, MMP8 | 0,890 | 0,881 | 0,900 | 0,921 | 0,861 | 0,981 | 0,940 | 0,874 | 1 |
| IFI27, OAS1, IFIT1, IFI44L, SIGLEC1, HERC6, RETN | 0,890 | 0,882 | 0,899 | 0,919 | 0,862 | 0,977 | 0,941 | 0,882 | 1 |
| RSAD2, IFI27, ISG15, HERC6, IL1R2, FAM20A, RETN | 0,890 | 0,882 | 0,899 | 0,931 | 0,881 | 0,982 | 0,930 | 0,850 | 1 |
| IFI27, IFIT1, IFI44L, SIGLEC1, HERC6, FAM20A, MMP8 | 0,890 | 0,882 | 0,899 | 0,921 | 0,862 | 0,980 | 0,939 | 0,864 | 1 |
| RSAD2, IFI27, SIGLEC1, SLC1A2, IL1R2, OLAH, FAM20A | 0,890 | 0,881 | 0,900 | 0,929 | 0,876 | 0,982 | 0,946 | 0,882 | 1 |
| IFI27, OAS1, IFIT1, IFI44L, IL1R2, FAM20A, MMP8 | 0,890 | 0,882 | 0,899 | 0,925 | 0,872 | 0,977 | 0,933 | 0,854 | 1 |
| IFI27, IFI44L, ISG15, SLC1A2, IL1R2, OLAH, FAM20A | 0,890 | 0,882 | 0,899 | 0,931 | 0,881 | 0,982 | 0,918 | 0,837 | 1,000 |
| IFI27, OAS1, IFIT1, IFI44L, SIGLEC1, HERC6, FAM20A | 0,890 | 0,882 | 0,899 | 0,922 | 0,865 | 0,980 | 0,939 | 0,868 | 1 |
| IFI27, SIGLEC1, ISG15, SLC1A2, OLAH, FAM20A, RETN | 0,890 | 0,881 | 0,900 | 0,930 | 0,877 | 0,983 | 0,936 | 0,866 | 1 |
| IFI27, OAS1, IFIT1, IFI44L, ISG15, OLAH, FAM20A | 0,890 | 0,881 | 0,899 | 0,920 | 0,860 | 0,981 | 0,935 | 0,867 | 1 |
| IFI27, IFIT1, SIGLEC1, ISG15, SLC1A2, IL1R2, FAM20A | 0,890 | 0,882 | 0,899 | 0,933 | 0,886 | 0,979 | 0,943 | 0,874 | 1 |
| IFI27, IFI44L, SIGLEC1, ISG15, HERC6, IL1R2, OLAH | 0,890 | 0,882 | 0,898 | 0,926 | 0,877 | 0,975 | 0,941 | 0,888 | 0,995 |
| IFI27, IFIT1, SIGLEC1, ISG15, HERC6, FAM20A, MMP8 | 0,890 | 0,882 | 0,899 | 0,920 | 0,860 | 0,979 | 0,939 | 0,864 | 1 |
| IFI27, OAS1, IFI44L, SIGLEC1, ISG15, HERC6, RETN | 0,890 | 0,882 | 0,898 | 0,920 | 0,863 | 0,978 | 0,943 | 0,883 | 1 |
| IFI27, OAS1, HERC6, IL1R2, OLAH, RETN, MMP8 | 0,890 | 0,881 | 0,899 | 0,923 | 0,868 | 0,979 | 0,945 | 0,886 | 1 |
| RSAD2, IFI27, IFI44L, SIGLEC1, OLAH, FAM20A, RETN | 0,890 | 0,881 | 0,900 | 0,924 | 0,864 | 0,983 | 0,950 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, OLAH, RETN | 0,890 | 0,882 | 0,899 | 0,923 | 0,867 | 0,978 | 0,946 | 0,893 | 0,999 |
| RSAD2, IFI27, IFIT1, IFI44L, IL1R2, FAM20A, MMP8 | 0,890 | 0,882 | 0,899 | 0,926 | 0,873 | 0,979 | 0,935 | 0,857 | 1 |
| IFI27, IFI44L, ISG15, IL1R2, FAM20A, RETN, MMP8 | 0,890 | 0,881 | 0,899 | 0,928 | 0,875 | 0,981 | 0,929 | 0,848 | 1 |
| RSAD2, IFI27, OAS1, IFIT1, IL1R2, FAM20A, MMP8 | 0,890 | 0,881 | 0,899 | 0,926 | 0,873 | 0,978 | 0,933 | 0,854 | 1 |
| RSAD2, IFI27, ISG15, SLC1A2, OLAH, FAM20A, MMP8 | 0,890 | 0,881 | 0,899 | 0,928 | 0,875 | 0,980 | 0,918 | 0,837 | 1 |
| RSAD2, IFI27, OAS1, IFIT1, IL1R2, OLAH, FAM20A | 0,890 | 0,882 | 0,899 | 0,924 | 0,867 | 0,981 | 0,933 | 0,863 | 1 |
| RSAD2, IFI27, SIGLEC1, HERC6, SLC1A2, IL1R2, FAM20A | 0,890 | 0,881 | 0,900 | 0,933 | 0,884 | 0,981 | 0,946 | 0,876 | 1 |
| RSAD2, IFI27, IFIT1, ISG15, OLAH, FAM20A, MMP8 | 0,890 | 0,881 | 0,900 | 0,921 | 0,861 | 0,981 | 0,937 | 0,872 | 1 |
| IFI27, HERC6, SLC1A2, IL1R2, OLAH, FAM20A, MMP8 | 0,890 | 0,881 | 0,899 | 0,933 | 0,885 | 0,982 | 0,925 | 0,845 | 1 |
| IFI27, IFIT1, ISG15, HERC6, IL1R2, FAM20A, MMP8 | 0,890 | 0,881 | 0,899 | 0,929 | 0,878 | 0,979 | 0,935 | 0,855 | 1 |
| IFI27, OAS1, SIGLEC1, HERC6, IL1R2, OLAH, MMP8 | 0,890 | 0,882 | 0,898 | 0,924 | 0,874 | 0,973 | 0,945 | 0,893 | 0,996 |
| RSAD2, IFI27, IFIT1, HERC6, IL1R2, FAM20A, RETN | 0,890 | 0,881 | 0,899 | 0,932 | 0,881 | 0,983 | 0,929 | 0,849 | 1 |
| IFI27, OAS1, IFIT1, SIGLEC1, HERC6, RETN, MMP8 | 0,890 | 0,881 | 0,899 | 0,919 | 0,861 | 0,977 | 0,946 | 0,888 | 1 |
| IFI27, OAS1, IFIT1, HERC6, SLC1A2, OLAH, FAM20A | 0,890 | 0,880 | 0,900 | 0,929 | 0,878 | 0,980 | 0,920 | 0,838 | 1 |
| IFI27, OAS1, IFIT1, IL1R2, FAM20A, RETN, MMP8 | 0,890 | 0,881 | 0,899 | 0,929 | 0,875 | 0,982 | 0,936 | 0,859 | 1 |
| IFI27, IFIT1, ISG15, IL1R2, OLAH, FAM20A, RETN | 0,890 | 0,881 | 0,899 | 0,923 | 0,866 | 0,980 | 0,932 | 0,861 | 1 |
| IFIT1, SIGLEC1, ISG15, HERC6, IL1R2, FAM20A, MMP8 | 0,890 | 0,881 | 0,899 | 0,922 | 0,868 | 0,977 | 0,962 | 0,906 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, SLC1A2, OLAH, FAM20A | 0,890 | 0,881 | 0,899 | 0,929 | 0,874 | 0,984 | 0,941 | 0,877 | 1 |
| IFI27, IFIT1, IFI44L, HERC6, SLC1A2, OLAH, FAM20A | 0,890 | 0,880 | 0,899 | 0,930 | 0,877 | 0,982 | 0,923 | 0,842 | 1 |
| IFI27, OAS1, IFIT1, SIGLEC1, HERC6, OLAH, MMP8 | 0,890 | 0,881 | 0,898 | 0,923 | 0,871 | 0,974 | 0,938 | 0,881 | 0,995 |
| RSAD2, IFI27, IFI44L, SIGLEC1, SLC1A2, OLAH, FAM20A | 0,890 | 0,881 | 0,899 | 0,927 | 0,870 | 0,983 | 0,943 | 0,879 | 1 |
| RSAD2, IFI27, ISG15, HERC6, SLC1A2, OLAH, FAM20A | 0,890 | 0,880 | 0,899 | 0,931 | 0,881 | 0,982 | 0,915 | 0,832 | 0,999 |
| IFI27, OAS1, HERC6, SLC1A2, IL1R2, OLAH, FAM20A | 0,890 | 0,881 | 0,899 | 0,932 | 0,884 | 0,981 | 0,916 | 0,830 | 1 |
| RSAD2, IFI27, IFI44L, ISG15, IL1R2, OLAH, FAM20A | 0,890 | 0,881 | 0,899 | 0,923 | 0,866 | 0,980 | 0,928 | 0,856 | 1 |
| IFI27, OAS1, ISG15, OLAH, FAM20A, RETN, MMP8 | 0,890 | 0,880 | 0,899 | 0,923 | 0,864 | 0,982 | 0,939 | 0,872 | 1 |
| IFI27, OAS1, SIGLEC1, SLC1A2, OLAH, FAM20A, RETN | 0,890 | 0,881 | 0,899 | 0,927 | 0,871 | 0,983 | 0,940 | 0,875 | 1 |
| IFI27, OAS1, IFIT1, IFI44L, HERC6, FAM20A, RETN | 0,890 | 0,881 | 0,899 | 0,923 | 0,865 | 0,981 | 0,932 | 0,852 | 1 |
| IFI27, OAS1, IFI44L, HERC6, SLC1A2, OLAH, FAM20A | 0,890 | 0,880 | 0,900 | 0,930 | 0,879 | 0,981 | 0,922 | 0,842 | 1 |
| IFI27, OAS1, ISG15, IL1R2, OLAH, FAM20A, RETN | 0,890 | 0,881 | 0,899 | 0,925 | 0,870 | 0,981 | 0,927 | 0,855 | 0,999 |
| IFI27, IFI44L, SIGLEC1, HERC6, SLC1A2, OLAH, FAM20A | 0,890 | 0,880 | 0,899 | 0,928 | 0,873 | 0,983 | 0,949 | 0,885 | 1 |
| IFI27, OAS1, IFI44L, ISG15, HERC6, FAM20A, RETN | 0,890 | 0,880 | 0,899 | 0,925 | 0,868 | 0,982 | 0,934 | 0,855 | 1 |
| IFI27, IFIT1, SIGLEC1, ISG15, HERC6, OLAH, RETN | 0,890 | 0,881 | 0,899 | 0,923 | 0,869 | 0,978 | 0,946 | 0,893 | 0,999 |
| IFI27, OAS1, IFIT1, SIGLEC1, SLC1A2, IL1R2, FAM20A | 0,890 | 0,881 | 0,899 | 0,932 | 0,883 | 0,980 | 0,943 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, ISG15, HERC6, OLAH, RETN, MMP8 | 0,890 | 0,881 | 0,899 | 0,924 | 0,869 | 0,980 | 0,945 | 0,886 | 1 |
| RSAD2, IFIT1, SIGLEC1, HERC6, IL1R2, FAM20A, MMP8 | 0,890 | 0,881 | 0,898 | 0,923 | 0,869 | 0,977 | 0,962 | 0,906 | 1 |
| RSAD2, IFI27, HERC6, IL1R2, OLAH, RETN, MMP8 | 0,890 | 0,881 | 0,899 | 0,925 | 0,870 | 0,980 | 0,943 | 0,887 | 1 |
| IFI27, IFIT1, ISG15, HERC6, IL1R2, FAM20A, RETN | 0,890 | 0,881 | 0,899 | 0,931 | 0,880 | 0,982 | 0,929 | 0,849 | 1 |
| RSAD2, IFI27, IFI44L, HERC6, IL1R2, FAM20A, RETN | 0,890 | 0,880 | 0,899 | 0,932 | 0,882 | 0,983 | 0,929 | 0,849 | 1 |
| RSAD2, IFI27, OAS1, SIGLEC1, HERC6, OLAH, RETN | 0,890 | 0,881 | 0,898 | 0,924 | 0,868 | 0,979 | 0,946 | 0,889 | 1 |
| IFI27, OAS1, IFI44L, SIGLEC1, HERC6, IL1R2, RETN | 0,890 | 0,882 | 0,898 | 0,925 | 0,874 | 0,976 | 0,940 | 0,885 | 0,996 |
| RSAD2, IFI27, OAS1, OLAH, FAM20A, RETN, MMP8 | 0,890 | 0,880 | 0,899 | 0,922 | 0,864 | 0,981 | 0,940 | 0,874 | 1 |
| RSAD2, IFI27, OAS1, IFIT1, IFI44L, OLAH, FAM20A | 0,890 | 0,880 | 0,899 | 0,921 | 0,861 | 0,981 | 0,933 | 0,863 | 1 |
| IFI27, OAS1, IFIT1, OLAH, FAM20A, RETN, MMP8 | 0,890 | 0,880 | 0,899 | 0,923 | 0,863 | 0,982 | 0,942 | 0,877 | 1 |
| IFI27, OAS1, SIGLEC1, ISG15, HERC6, RETN, MMP8 | 0,890 | 0,880 | 0,899 | 0,922 | 0,863 | 0,980 | 0,949 | 0,889 | 1 |
| RSAD2, IFI27, OAS1, SLC1A2, OLAH, FAM20A, MMP8 | 0,890 | 0,880 | 0,899 | 0,925 | 0,869 | 0,981 | 0,923 | 0,843 | 1 |
| RSAD2, IFI27, IFIT1, IFI44L, IL1R2, OLAH, FAM20A | 0,890 | 0,881 | 0,898 | 0,925 | 0,868 | 0,982 | 0,930 | 0,858 | 1 |
| RSAD2, IFI27, OAS1, SIGLEC1, HERC6, IL1R2, RETN | 0,890 | 0,881 | 0,898 | 0,923 | 0,869 | 0,977 | 0,954 | 0,901 | 1 |
| RSAD2, IFI27, IFI44L, SLC1A2, OLAH, FAM20A, MMP8 | 0,890 | 0,880 | 0,899 | 0,930 | 0,876 | 0,983 | 0,928 | 0,854 | 1 |
| IFI27, IFIT1, IFI44L, ISG15, OLAH, FAM20A, RETN | 0,890 | 0,881 | 0,899 | 0,920 | 0,860 | 0,981 | 0,935 | 0,865 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, IL1R2, OLAH, FAM20A, RETN | 0,890 | 0,881 | 0,898 | 0,924 | 0,867 | 0,981 | 0,930 | 0,858 | 1 |
| RSAD2, IFI27, IFIT1, SIGLEC1, ISG15, HERC6, FAM20A | 0,890 | 0,881 | 0,898 | 0,922 | 0,865 | 0,980 | 0,941 | 0,871 | 1 |
| IFI27, OAS1, SIGLEC1, HERC6, IL1R2, OLAH, RETN | 0,889 | 0,881 | 0,898 | 0,923 | 0,868 | 0,978 | 0,945 | 0,889 | 1 |
| RSAD2, IFI27, IFI44L, SIGLEC1, HERC6, IL1R2, OLAH | 0,889 | 0,881 | 0,898 | 0,927 | 0,879 | 0,976 | 0,942 | 0,890 | 0,994 |
| IFI27, IFI44L, ISG15, HERC6, SLC1A2, OLAH, FAM20A | 0,889 | 0,880 | 0,899 | 0,930 | 0,879 | 0,982 | 0,918 | 0,837 | 1 |
| IFI27, OAS1, SLC1A2, IL1R2, OLAH, FAM20A, MMP8 | 0,889 | 0,881 | 0,898 | 0,931 | 0,881 | 0,980 | 0,924 | 0,845 | 1 |
| RSAD2, IFI27, OAS1, SIGLEC1, HERC6, IL1R2, OLAH | 0,889 | 0,881 | 0,898 | 0,924 | 0,875 | 0,974 | 0,942 | 0,890 | 0,995 |
| RSAD2, IFI27, IFIT1, HERC6, OLAH, RETN, MMP8 | 0,889 | 0,880 | 0,899 | 0,925 | 0,869 | 0,981 | 0,943 | 0,886 | 1 |
| RSAD2, IFI27, SIGLEC1, HERC6, IL1R2, OLAH, MMP8 | 0,889 | 0,881 | 0,898 | 0,926 | 0,877 | 0,975 | 0,942 | 0,890 | 0,995 |
| IFI27, OAS1, IFIT1, ISG15, HERC6, FAM20A, RETN | 0,889 | 0,880 | 0,899 | 0,923 | 0,867 | 0,980 | 0,935 | 0,857 | 1 |
| IFI27, IFIT1, IFI44L, ISG15, SLC1A2, OLAH, FAM20A | 0,889 | 0,880 | 0,898 | 0,928 | 0,874 | 0,982 | 0,916 | 0,834 | 0,999 |
| RSAD2, IFI27, OAS1, SIGLEC1, FAM20A, RETN, MMP8 | 0,889 | 0,879 | 0,900 | 0,925 | 0,867 | 0,984 | 0,947 | 0,875 | 1 |
| RSAD2, IFI27, OAS1, SIGLEC1, ISG15, HERC6, RETN | 0,889 | 0,881 | 0,898 | 0,920 | 0,862 | 0,979 | 0,948 | 0,887 | 1 |
| IFI27, OAS1, SIGLEC1, ISG15, HERC6, OLAH, RETN | 0,889 | 0,880 | 0,898 | 0,923 | 0,867 | 0,978 | 0,945 | 0,887 | 1 |
| IFI27, OAS1, IFI44L, SIGLEC1, SLC1A2, IL1R2, FAM20A | 0,889 | 0,880 | 0,898 | 0,931 | 0,881 | 0,980 | 0,942 | 0,872 | 1 |
| RSAD2, IFI27, OAS1, IFIT1, SIGLEC1, HERC6, FAM20A | 0,889 | 0,881 | 0,898 | 0,923 | 0,866 | 0,980 | 0,939 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, ISG15, HERC6, FAM20A | 0,889 | 0,881 | 0,898 | 0,923 | 0,867 | 0,980 | 0,938 | 0,865 | 1 |
| RSAD2, IFI27, ISG15, IL1R2, OLAH, FAM20A, RETN | 0,889 | 0,880 | 0,898 | 0,924 | 0,868 | 0,981 | 0,927 | 0,853 | 1 |
| IFI27, IFI44L, SIGLEC1, ISG15, HERC6, OLAH, MMP8 | 0,889 | 0,881 | 0,898 | 0,925 | 0,873 | 0,977 | 0,945 | 0,892 | 0,997 |
| IFI27, OAS1, IFI44L, SIGLEC1, HERC6, IL1R2, OLAH | 0,889 | 0,881 | 0,898 | 0,927 | 0,878 | 0,975 | 0,946 | 0,895 | 0,996 |
| RSAD2, IFI27, IFI44L, SIGLEC1, HERC6, OLAH, RETN | 0,889 | 0,881 | 0,898 | 0,923 | 0,869 | 0,977 | 0,948 | 0,896 | 1,000 |
| IFI27, IFIT1, IFI44L, ISG15, HERC6, FAM20A, RETN | 0,889 | 0,880 | 0,898 | 0,921 | 0,863 | 0,979 | 0,933 | 0,853 | 1 |
| IFI27, OAS1, IFI44L, SIGLEC1, HERC6, OLAH, MMP8 | 0,889 | 0,881 | 0,898 | 0,926 | 0,875 | 0,977 | 0,943 | 0,891 | 0,996 |
| RSAD2, IFI27, IFIT1, ISG15, HERC6, IL1R2, FAM20A | 0,889 | 0,881 | 0,898 | 0,927 | 0,876 | 0,978 | 0,930 | 0,850 | 1 |
| IFI27, IFI44L, SIGLEC1, HERC6, SLC1A2, IL1R2, FAM20A | 0,889 | 0,880 | 0,898 | 0,931 | 0,882 | 0,980 | 0,947 | 0,876 | 1 |
| IFI27, OAS1, IFIT1, ISG15, HERC6, OLAH, RETN | 0,889 | 0,880 | 0,898 | 0,921 | 0,866 | 0,977 | 0,938 | 0,876 | 1,000 |
| IFI27, IFI44L, ISG15, HERC6, OLAH, RETN, MMP8 | 0,889 | 0,880 | 0,898 | 0,922 | 0,864 | 0,980 | 0,948 | 0,895 | 1 |
| RSAD2, IFI27, OAS1, SIGLEC1, HERC6, OLAH, MMP8 | 0,889 | 0,880 | 0,898 | 0,923 | 0,870 | 0,975 | 0,938 | 0,881 | 0,995 |
| RSAD2, IFI27, IFIT1, ISG15, IL1R2, OLAH, FAM20A | 0,889 | 0,880 | 0,898 | 0,924 | 0,867 | 0,981 | 0,932 | 0,861 | 1 |
| IFI27, SLC1A2, IL1R2, OLAH, FAM20A, RETN, MMP8 | 0,889 | 0,880 | 0,899 | 0,933 | 0,883 | 0,982 | 0,922 | 0,842 | 1 |
| IFI27, OAS1, ISG15, SLC1A2, OLAH, FAM20A, MMP8 | 0,889 | 0,880 | 0,898 | 0,927 | 0,875 | 0,980 | 0,920 | 0,841 | 0,998 |
| OAS1, IFIT1, SIGLEC1, IL1R2, OLAH, FAM20A, MMP8 | 0,889 | 0,881 | 0,898 | 0,919 | 0,861 | 0,977 | 0,965 | 0,913 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, IL1R2, OLAH, FAM20A | 0,889 | 0,880 | 0,898 | 0,924 | 0,867 | 0,982 | 0,930 | 0,858 | 1 |
| IFI27, OAS1, SIGLEC1, ISG15, FAM20A, RETN, MMP8 | 0,889 | 0,879 | 0,899 | 0,923 | 0,863 | 0,984 | 0,945 | 0,871 | 1 |
| RSAD2, IFI27, OAS1, IFIT1, HERC6, OLAH, RETN | 0,889 | 0,880 | 0,898 | 0,923 | 0,867 | 0,979 | 0,940 | 0,880 | 1 |
| RSAD2, IFI27, IFIT1, SLC1A2, OLAH, FAM20A, MMP8 | 0,889 | 0,880 | 0,898 | 0,926 | 0,870 | 0,981 | 0,929 | 0,857 | 1 |
| IFI27, IFIT1, IFI44L, ISG15, IL1R2, FAM20A, MMP8 | 0,889 | 0,880 | 0,898 | 0,926 | 0,873 | 0,978 | 0,933 | 0,854 | 1 |
| IFI27, OAS1, IFIT1, SIGLEC1, HERC6, IL1R2, OLAH | 0,889 | 0,881 | 0,897 | 0,924 | 0,875 | 0,974 | 0,942 | 0,890 | 0,995 |
| IFI27, IFIT1, IFI44L, HERC6, OLAH, RETN, MMP8 | 0,889 | 0,880 | 0,898 | 0,922 | 0,864 | 0,979 | 0,949 | 0,897 | 1 |
| IFI27, OAS1, IFIT1, IL1R2, OLAH, FAM20A, RETN | 0,889 | 0,880 | 0,898 | 0,925 | 0,869 | 0,981 | 0,932 | 0,861 | 1 |
| IFI27, ISG15, HERC6, SLC1A2, IL1R2, OLAH, FAM20A | 0,889 | 0,880 | 0,898 | 0,932 | 0,883 | 0,981 | 0,917 | 0,834 | 1 |
| IFI27, SIGLEC1, HERC6, SLC1A2, IL1R2, OLAH, FAM20A | 0,889 | 0,880 | 0,898 | 0,927 | 0,872 | 0,981 | 0,943 | 0,875 | 1 |
| IFI27, IFI44L, SIGLEC1, HERC6, IL1R2, OLAH, RETN | 0,889 | 0,880 | 0,898 | 0,923 | 0,870 | 0,977 | 0,948 | 0,896 | 0,999 |
| RSAD2, IFI27, IFIT1, OLAH, FAM20A, RETN, MMP8 | 0,889 | 0,879 | 0,899 | 0,923 | 0,864 | 0,982 | 0,940 | 0,875 | 1 |
| IFI27, IFIT1, ISG15, HERC6, SLC1A2, OLAH, FAM20A | 0,889 | 0,879 | 0,899 | 0,930 | 0,879 | 0,982 | 0,916 | 0,834 | 0,999 |
| RSAD2, IFI27, IFIT1, SIGLEC1, HERC6, OLAH, RETN | 0,889 | 0,880 | 0,898 | 0,925 | 0,872 | 0,978 | 0,942 | 0,887 | 0,998 |
| IFI27, IFI44L, SIGLEC1, ISG15, IL1R2, OLAH, MMP8 | 0,889 | 0,881 | 0,897 | 0,924 | 0,875 | 0,974 | 0,943 | 0,891 | 0,996 |
| RSAD2, IFI27, IFI44L, HERC6, SLC1A2, OLAH, FAM20A | 0,889 | 0,879 | 0,899 | 0,930 | 0,878 | 0,981 | 0,923 | 0,844 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, HERC6, OLAH, MMP8 | 0,889 | 0,881 | 0,897 | 0,924 | 0,871 | 0,977 | 0,947 | 0,897 | 0,997 |
| RSAD2, IFI27, OAS1, IFI44L, IL1R2, FAM20A, MMP8 | 0,889 | 0,880 | 0,898 | 0,926 | 0,874 | 0,979 | 0,930 | 0,847 | 1 |
| IFI27, OAS1, SIGLEC1, ISG15, HERC6, IL1R2, RETN | 0,889 | 0,880 | 0,897 | 0,925 | 0,873 | 0,977 | 0,953 | 0,900 | 1 |
| IFI27, OAS1, IFIT1, SLC1A2, OLAH, FAM20A, MMP8 | 0,889 | 0,879 | 0,898 | 0,925 | 0,869 | 0,981 | 0,926 | 0,850 | 1 |
| IFI27, OAS1, IFI44L, SLC1A2, OLAH, FAM20A, MMP8 | 0,889 | 0,879 | 0,899 | 0,932 | 0,879 | 0,985 | 0,925 | 0,848 | 1 |
| IFI27, IFIT1, HERC6, SLC1A2, OLAH, FAM20A, MMP8 | 0,889 | 0,879 | 0,899 | 0,927 | 0,873 | 0,980 | 0,923 | 0,842 | 1 |
| IFI27, OAS1, IFI44L, IL1R2, OLAH, FAM20A, RETN | 0,889 | 0,880 | 0,898 | 0,924 | 0,866 | 0,981 | 0,929 | 0,856 | 1 |
| RSAD2, IFI27, OAS1, IFI44L, ISG15, OLAH, FAM20A | 0,889 | 0,880 | 0,898 | 0,921 | 0,861 | 0,981 | 0,930 | 0,861 | 1,000 |
| IFI27, OAS1, IFIT1, IFI44L, SIGLEC1, HERC6, OLAH | 0,889 | 0,880 | 0,897 | 0,926 | 0,875 | 0,977 | 0,946 | 0,894 | 0,998 |
| IFI27, SIGLEC1, SLC1A2, OLAH, FAM20A, RETN, MMP8 | 0,889 | 0,879 | 0,899 | 0,927 | 0,871 | 0,983 | 0,947 | 0,879 | 1 |
| RSAD2, IFI27, OAS1, IFI44L, SIGLEC1, HERC6, RETN | 0,889 | 0,880 | 0,897 | 0,919 | 0,860 | 0,977 | 0,941 | 0,875 | 1 |
| IFI27, OAS1, IFIT1, IFI44L, HERC6, OLAH, RETN | 0,889 | 0,880 | 0,897 | 0,923 | 0,868 | 0,978 | 0,938 | 0,877 | 0,999 |
| IFI27, OAS1, IFIT1, HERC6, IL1R2, OLAH, RETN | 0,889 | 0,880 | 0,897 | 0,922 | 0,867 | 0,978 | 0,939 | 0,878 | 1,000 |
| RSAD2, IFI27, IFIT1, IL1R2, FAM20A, RETN, MMP8 | 0,889 | 0,880 | 0,898 | 0,930 | 0,877 | 0,982 | 0,934 | 0,856 | 1 |
| IFI27, OAS1, HERC6, SLC1A2, OLAH, FAM20A, RETN | 0,889 | 0,879 | 0,898 | 0,932 | 0,883 | 0,981 | 0,916 | 0,830 | 1 |
| IFI27, IFIT1, IFI44L, SIGLEC1, ISG15, HERC6, OLAH | 0,889 | 0,880 | 0,897 | 0,925 | 0,873 | 0,978 | 0,945 | 0,892 | 0,997 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, HERC6, OLAH, RETN, MMP8 | 0,889 | 0,879 | 0,898 | 0,923 | 0,867 | 0,980 | 0,941 | 0,883 | 1,000 |
| RSAD2, IFI27, OAS1, IL1R2, OLAH, FAM20A, RETN | 0,889 | 0,880 | 0,898 | 0,924 | 0,867 | 0,981 | 0,930 | 0,858 | 1 |
| IFI27, IFIT1, SIGLEC1, HERC6, IL1R2, OLAH, RETN | 0,889 | 0,880 | 0,897 | 0,923 | 0,869 | 0,978 | 0,948 | 0,896 | 1 |
| RSAD2, IFI27, IFIT1, IFI44L, ISG15, OLAH, FAM20A | 0,889 | 0,879 | 0,898 | 0,920 | 0,860 | 0,981 | 0,933 | 0,863 | 1 |
| RSAD2, IFI27, ISG15, OLAH, FAM20A, RETN, MMP8 | 0,889 | 0,879 | 0,898 | 0,922 | 0,863 | 0,981 | 0,940 | 0,874 | 1 |
| RSAD2, IFI27, SIGLEC1, ISG15, SLC1A2, IL1R2, FAM20A | 0,889 | 0,879 | 0,898 | 0,933 | 0,885 | 0,980 | 0,945 | 0,874 | 1 |
| RSAD2, IFI27, OAS1, HERC6, IL1R2, OLAH, RETN | 0,889 | 0,880 | 0,897 | 0,922 | 0,866 | 0,978 | 0,942 | 0,883 | 1 |
| IFI27, IFI44L, HERC6, IL1R2, OLAH, RETN, MMP8 | 0,888 | 0,880 | 0,897 | 0,922 | 0,866 | 0,979 | 0,947 | 0,893 | 1 |
| IFI27, OAS1, IFI44L, ISG15, SLC1A2, OLAH, FAM20A | 0,888 | 0,879 | 0,898 | 0,930 | 0,877 | 0,983 | 0,918 | 0,837 | 0,999 |
| RSAD2, IFI27, OAS1, HERC6, IL1R2, OLAH, MMP8 | 0,888 | 0,880 | 0,897 | 0,927 | 0,879 | 0,976 | 0,938 | 0,880 | 0,996 |
| RSAD2, IFI27, IFIT1, SIGLEC1, FAM20A, RETN, MMP8 | 0,888 | 0,879 | 0,898 | 0,924 | 0,866 | 0,982 | 0,947 | 0,877 | 1 |
| IFI27, IFIT1, SIGLEC1, SLC1A2, IL1R2, FAM20A, RETN | 0,888 | 0,879 | 0,897 | 0,934 | 0,886 | 0,981 | 0,943 | 0,874 | 1 |
| RSAD2, IFI27, IFIT1, HERC6, SLC1A2, OLAH, FAM20A | 0,888 | 0,879 | 0,898 | 0,928 | 0,876 | 0,980 | 0,922 | 0,840 | 1 |
| IFI27, OAS1, SIGLEC1, ISG15, HERC6, IL1R2, OLAH | 0,888 | 0,880 | 0,897 | 0,924 | 0,874 | 0,974 | 0,940 | 0,885 | 0,996 |
| IFI27, IFIT1, IFI44L, SIGLEC1, HERC6, IL1R2, OLAH | 0,888 | 0,880 | 0,897 | 0,927 | 0,878 | 0,976 | 0,943 | 0,892 | 0,995 |
| IFI27, OAS1, IFIT1, IFI44L, SLC1A2, OLAH, FAM20A | 0,888 | 0,879 | 0,898 | 0,929 | 0,874 | 0,984 | 0,924 | 0,846 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, ISG15, SLC1A2, IL1R2, FAM20A | 0,888 | 0,879 | 0,898 | 0,933 | 0,886 | 0,980 | 0,945 | 0,876 | 1 |
| RSAD2, IFI27, IFIT1, SIGLEC1, SLC1A2, IL1R2, FAM20A | 0,888 | 0,879 | 0,897 | 0,934 | 0,887 | 0,981 | 0,943 | 0,874 | 1 |
| RSAD2, IFI27, OAS1, IFIT1, ISG15, OLAH, FAM20A | 0,888 | 0,879 | 0,897 | 0,921 | 0,861 | 0,981 | 0,933 | 0,865 | 1 |
| IFI27, OAS1, IFIT1, IFI44L, OLAH, FAM20A, RETN | 0,888 | 0,879 | 0,898 | 0,920 | 0,860 | 0,981 | 0,938 | 0,870 | 1 |
| RSAD2, IFI27, OAS1, ISG15, HERC6, FAM20A, RETN | 0,888 | 0,879 | 0,898 | 0,924 | 0,868 | 0,980 | 0,935 | 0,857 | 1 |
| RSAD2, IFI27, IFIT1, IFI44L, SIGLEC1, FAM20A, RETN | 0,888 | 0,879 | 0,897 | 0,919 | 0,858 | 0,979 | 0,945 | 0,877 | 1 |
| RSAD2, IFI27, HERC6, SLC1A2, OLAH, FAM20A, RETN | 0,888 | 0,879 | 0,897 | 0,931 | 0,879 | 0,982 | 0,918 | 0,834 | 1 |
| IFI27, IFIT1, HERC6, IL1R2, OLAH, RETN, MMP8 | 0,888 | 0,879 | 0,897 | 0,922 | 0,865 | 0,979 | 0,948 | 0,895 | 1 |
| IFI27, IFI44L, SIGLEC1, SLC1A2, OLAH, FAM20A, RETN | 0,888 | 0,879 | 0,897 | 0,926 | 0,869 | 0,982 | 0,946 | 0,879 | 1 |
| RSAD2, IFI27, IFI44L, HERC6, OLAH, RETN, MMP8 | 0,888 | 0,879 | 0,897 | 0,923 | 0,867 | 0,980 | 0,943 | 0,887 | 1,000 |
| RSAD2, IFI27, ISG15, SLC1A2, IL1R2, OLAH, FAM20A | 0,888 | 0,879 | 0,897 | 0,931 | 0,882 | 0,981 | 0,916 | 0,833 | 1,000 |
| IFIT1, SIGLEC1, HERC6, IL1R2, FAM20A, RETN, MMP8 | 0,888 | 0,879 | 0,897 | 0,922 | 0,866 | 0,978 | 0,961 | 0,901 | 1 |
| IFI27, IFIT1, SIGLEC1, HERC6, SLC1A2, FAM20A, RETN | 0,888 | 0,879 | 0,897 | 0,930 | 0,878 | 0,981 | 0,940 | 0,866 | 1 |
| RSAD2, IFI27, IFI44L, ISG15, SLC1A2, OLAH, FAM20A | 0,888 | 0,879 | 0,898 | 0,927 | 0,873 | 0,982 | 0,916 | 0,834 | 0,999 |
| IFI27, IFIT1, SIGLEC1, ISG15, HERC6, IL1R2, OLAH | 0,888 | 0,880 | 0,896 | 0,926 | 0,876 | 0,976 | 0,941 | 0,888 | 0,995 |
| RSAD2, IFI27, OAS1, IFI44L, SIGLEC1, HERC6, OLAH | 0,888 | 0,880 | 0,896 | 0,927 | 0,876 | 0,977 | 0,946 | 0,894 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, IL1R2, OLAH, FAM20A, MMP8 | 0,888 | 0,879 | 0,897 | 0,922 | 0,866 | 0,978 | 0,965 | 0,913 | 1 |
| RSAD2, IFI27, SIGLEC1, SLC1A2, OLAH, FAM20A, RETN | 0,888 | 0,879 | 0,897 | 0,927 | 0,870 | 0,983 | 0,942 | 0,877 | 1 |
| IFI27, IFIT1, ISG15, HERC6, OLAH, RETN, MMP8 | 0,888 | 0,879 | 0,897 | 0,922 | 0,865 | 0,980 | 0,947 | 0,892 | 1 |
| IFI27, IFIT1, IFI44L, SIGLEC1, HERC6, OLAH, MMP8 | 0,888 | 0,880 | 0,897 | 0,924 | 0,871 | 0,977 | 0,947 | 0,897 | 0,997 |
| IFI27, OAS1, IFI44L, IL1R2, FAM20A, RETN, MMP8 | 0,888 | 0,879 | 0,897 | 0,929 | 0,876 | 0,981 | 0,935 | 0,856 | 1 |
| IFI27, OAS1, IFIT1, ISG15, OLAH, FAM20A, RETN | 0,888 | 0,879 | 0,897 | 0,920 | 0,860 | 0,980 | 0,935 | 0,867 | 1 |
| IFI27, OAS1, ISG15, HERC6, IL1R2, OLAH, RETN | 0,888 | 0,879 | 0,897 | 0,922 | 0,867 | 0,978 | 0,940 | 0,880 | 1 |
| RSAD2, IFI27, IFIT1, SIGLEC1, HERC6, FAM20A, MMP8 | 0,888 | 0,879 | 0,897 | 0,922 | 0,864 | 0,980 | 0,940 | 0,869 | 1 |
| IFI27, ISG15, SLC1A2, IL1R2, OLAH, FAM20A, RETN | 0,888 | 0,879 | 0,897 | 0,928 | 0,877 | 0,980 | 0,916 | 0,832 | 1 |
| IFI27, IFIT1, IFI44L, SIGLEC1, HERC6, IL1R2, RETN | 0,888 | 0,880 | 0,896 | 0,923 | 0,870 | 0,976 | 0,945 | 0,893 | 0,996 |
| IFI27, IFIT1, ISG15, IL1R2, FAM20A, RETN, MMP8 | 0,888 | 0,879 | 0,897 | 0,930 | 0,877 | 0,983 | 0,937 | 0,860 | 1 |
| RSAD2, IFI27, IFIT1, IFI44L, HERC6, FAM20A, RETN | 0,888 | 0,879 | 0,897 | 0,924 | 0,867 | 0,981 | 0,929 | 0,848 | 1 |
| RSAD2, IFI27, OAS1, ISG15, HERC6, OLAH, RETN | 0,888 | 0,879 | 0,897 | 0,923 | 0,867 | 0,979 | 0,943 | 0,885 | 1 |
| RSAD2, IFI27, SIGLEC1, HERC6, IL1R2, RETN, MMP8 | 0,888 | 0,879 | 0,897 | 0,922 | 0,869 | 0,976 | 0,961 | 0,920 | 1 |
| RSAD2, IFI27, ISG15, HERC6, OLAH, RETN, MMP8 | 0,888 | 0,879 | 0,897 | 0,924 | 0,867 | 0,980 | 0,945 | 0,889 | 1 |
| IFI27, IFI44L, HERC6, SLC1A2, OLAH, FAM20A, MMP8 | 0,888 | 0,878 | 0,898 | 0,927 | 0,873 | 0,981 | 0,926 | 0,848 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, HERC6, SLC1A2, IL1R2, OLAH, FAM20A | 0,888 | 0,879 | 0,897 | 0,930 | 0,880 | 0,981 | 0,927 | 0,848 | 1 |
| IFI27, IFI44L, SLC1A2, OLAH, FAM20A, RETN, MMP8 | 0,888 | 0,878 | 0,898 | 0,928 | 0,873 | 0,982 | 0,927 | 0,848 | 1 |
| RSAD2, IFI27, OAS1, SIGLEC1, SLC1A2, IL1R2, FAM20A | 0,888 | 0,879 | 0,897 | 0,932 | 0,884 | 0,980 | 0,942 | 0,871 | 1 |
| IFI27, OAS1, SIGLEC1, SLC1A2, IL1R2, FAM20A, RETN | 0,888 | 0,878 | 0,897 | 0,931 | 0,882 | 0,981 | 0,942 | 0,872 | 1 |
| IFI27, OAS1, IFI44L, OLAH, FAM20A, RETN, MMP8 | 0,888 | 0,878 | 0,898 | 0,923 | 0,863 | 0,983 | 0,942 | 0,877 | 1 |
| IFI27, ISG15, HERC6, SLC1A2, OLAH, FAM20A, RETN | 0,888 | 0,878 | 0,897 | 0,928 | 0,875 | 0,981 | 0,916 | 0,832 | 1 |
| IFI27, OAS1, IFIT1, HERC6, IL1R2, OLAH, MMP8 | 0,888 | 0,879 | 0,897 | 0,925 | 0,876 | 0,974 | 0,939 | 0,882 | 0,996 |
| RSAD2, IFI27, IFIT1, SIGLEC1, ISG15, FAM20A, RETN | 0,888 | 0,879 | 0,897 | 0,920 | 0,860 | 0,980 | 0,946 | 0,879 | 1 |
| IFI27, IFI44L, SIGLEC1, IL1R2, OLAH, RETN, MMP8 | 0,888 | 0,879 | 0,896 | 0,924 | 0,870 | 0,979 | 0,958 | 0,908 | 1 |
| RSAD2, IFI27, IFI44L, OLAH, FAM20A, RETN, MMP8 | 0,888 | 0,878 | 0,898 | 0,923 | 0,863 | 0,983 | 0,939 | 0,873 | 1 |
| IFI27, OAS1, IFIT1, ISG15, IL1R2, FAM20A, MMP8 | 0,888 | 0,879 | 0,897 | 0,926 | 0,874 | 0,978 | 0,933 | 0,854 | 1 |
| RSAD2, IFI27, OAS1, IFIT1, SIGLEC1, HERC6, OLAH | 0,888 | 0,879 | 0,896 | 0,924 | 0,873 | 0,975 | 0,940 | 0,885 | 0,995 |
| IFI27, OAS1, ISG15, HERC6, FAM20A, RETN, MMP8 | 0,888 | 0,878 | 0,898 | 0,925 | 0,868 | 0,982 | 0,935 | 0,857 | 1 |
| RSAD2, OAS1, SIGLEC1, HERC6, IL1R2, FAM20A, MMP8 | 0,888 | 0,879 | 0,897 | 0,920 | 0,865 | 0,976 | 0,961 | 0,902 | 1 |
| IFI27, IFI44L, SIGLEC1, SLC1A2, IL1R2, FAM20A, RETN | 0,888 | 0,879 | 0,897 | 0,930 | 0,879 | 0,980 | 0,947 | 0,877 | 1 |
| IFI27, IFIT1, SIGLEC1, ISG15, HERC6, OLAH, MMP8 | 0,888 | 0,879 | 0,896 | 0,922 | 0,869 | 0,975 | 0,941 | 0,887 | 0,995 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, ISG15, SLC1A2, OLAH, FAM20A, RETN | 0,888 | 0,878 | 0,897 | 0,928 | 0,874 | 0,982 | 0,917 | 0,836 | 0,999 |
| IFI27, IFI44L, ISG15, HERC6, IL1R2, OLAH, MMP8 | 0,888 | 0,879 | 0,896 | 0,927 | 0,877 | 0,976 | 0,934 | 0,874 | 0,993 |
| RSAD2, IFI27, SIGLEC1, HERC6, IL1R2, OLAH, RETN | 0,888 | 0,879 | 0,896 | 0,923 | 0,869 | 0,978 | 0,946 | 0,890 | 1 |
| IFI27, IFIT1, SIGLEC1, HERC6, IL1R2, OLAH, MMP8 | 0,888 | 0,879 | 0,896 | 0,926 | 0,876 | 0,975 | 0,943 | 0,892 | 0,995 |
| IFI27, IFI44L, SIGLEC1, HERC6, IL1R2, RETN, MMP8 | 0,888 | 0,879 | 0,896 | 0,924 | 0,871 | 0,977 | 0,952 | 0,906 | 0,998 |
| IFI27, OAS1, IFI44L, HERC6, IL1R2, OLAH, MMP8 | 0,888 | 0,879 | 0,896 | 0,928 | 0,880 | 0,976 | 0,935 | 0,876 | 0,994 |
| IFI27, IFIT1, ISG15, SLC1A2, IL1R2, OLAH, FAM20A | 0,888 | 0,879 | 0,897 | 0,929 | 0,878 | 0,980 | 0,920 | 0,839 | 1 |
| RSAD2, IFI27, OAS1, ISG15, SLC1A2, OLAH, FAM20A | 0,888 | 0,878 | 0,897 | 0,926 | 0,872 | 0,980 | 0,916 | 0,833 | 1,000 |
| IFI27, SIGLEC1, ISG15, HERC6, IL1R2, OLAH, MMP8 | 0,888 | 0,879 | 0,896 | 0,924 | 0,874 | 0,974 | 0,946 | 0,895 | 0,996 |
| IFI27, SIGLEC1, ISG15, HERC6, OLAH, RETN, MMP8 | 0,888 | 0,878 | 0,897 | 0,919 | 0,860 | 0,978 | 0,957 | 0,908 | 1 |
| RSAD2, IFI27, OAS1, IFIT1, SIGLEC1, FAM20A, RETN | 0,888 | 0,878 | 0,897 | 0,919 | 0,860 | 0,979 | 0,946 | 0,878 | 1 |
| IFI27, OAS1, SIGLEC1, HERC6, SLC1A2, OLAH, MMP8 | 0,887 | 0,878 | 0,897 | 0,928 | 0,878 | 0,978 | 0,941 | 0,882 | 1 |
| IFI27, OAS1, IFI44L, SIGLEC1, ISG15, HERC6, OLAH | 0,887 | 0,879 | 0,896 | 0,926 | 0,875 | 0,977 | 0,943 | 0,890 | 0,997 |
| RSAD2, IFI27, IFIT1, IL1R2, OLAH, FAM20A, RETN | 0,887 | 0,879 | 0,896 | 0,924 | 0,868 | 0,981 | 0,933 | 0,863 | 1 |
| IFI27, OAS1, SIGLEC1, HERC6, SLC1A2, OLAH, RETN | 0,887 | 0,878 | 0,897 | 0,929 | 0,879 | 0,979 | 0,941 | 0,878 | 1 |
| IFI27, OAS1, ISG15, HERC6, IL1R2, OLAH, MMP8 | 0,887 | 0,879 | 0,896 | 0,926 | 0,877 | 0,975 | 0,938 | 0,880 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, ISG15, FAM20A, RETN, MMP8 | 0,887 | 0,877 | 0,898 | 0,925 | 0,867 | 0,984 | 0,945 | 0,872 | 1 |
| IFI27, IFIT1, IFI44L, SLC1A2, IL1R2, OLAH, FAM20A | 0,887 | 0,879 | 0,896 | 0,928 | 0,874 | 0,982 | 0,921 | 0,838 | 1 |
| IFI27, IFI44L, SIGLEC1, ISG15, FAM20A, RETN, MMP8 | 0,887 | 0,877 | 0,897 | 0,922 | 0,862 | 0,981 | 0,948 | 0,875 | 1 |
| RSAD2, IFI27, IFI44L, SIGLEC1, SLC1A2, IL1R2, FAM20A | 0,887 | 0,878 | 0,897 | 0,930 | 0,880 | 0,980 | 0,945 | 0,875 | 1 |
| RSAD2, IFI27, OAS1, SIGLEC1, HERC6, RETN, MMP8 | 0,887 | 0,878 | 0,897 | 0,919 | 0,859 | 0,979 | 0,950 | 0,890 | 1 |
| IFI27, OAS1, IFI44L, SIGLEC1, HERC6, RETN, MMP8 | 0,887 | 0,879 | 0,896 | 0,919 | 0,862 | 0,977 | 0,945 | 0,884 | 1 |
| RSAD2, IFI27, IFIT1, SIGLEC1, HERC6, IL1R2, OLAH | 0,887 | 0,879 | 0,896 | 0,926 | 0,877 | 0,975 | 0,940 | 0,887 | 0,994 |
| IFI27, IFIT1, IFI44L, ISG15, HERC6, OLAH, RETN | 0,887 | 0,879 | 0,896 | 0,920 | 0,864 | 0,977 | 0,941 | 0,885 | 0,997 |
| IFI27, ISG15, SLC1A2, OLAH, FAM20A, RETN, MMP8 | 0,887 | 0,878 | 0,897 | 0,928 | 0,875 | 0,981 | 0,924 | 0,845 | 1 |
| IFI27, OAS1, SIGLEC1, ISG15, HERC6, OLAH, MMP8 | 0,887 | 0,879 | 0,896 | 0,922 | 0,869 | 0,976 | 0,938 | 0,881 | 0,995 |
| RSAD2, IFI27, OAS1, IFI44L, HERC6, OLAH, RETN | 0,887 | 0,879 | 0,896 | 0,922 | 0,865 | 0,978 | 0,941 | 0,882 | 1 |
| RSAD2, IFI27, SIGLEC1, ISG15, HERC6, IL1R2, OLAH | 0,887 | 0,879 | 0,896 | 0,925 | 0,875 | 0,975 | 0,938 | 0,882 | 0,994 |
| IFI27, ISG15, HERC6, IL1R2, OLAH, RETN, MMP8 | 0,887 | 0,878 | 0,896 | 0,922 | 0,865 | 0,979 | 0,948 | 0,894 | 1 |
| RSAD2, IFI27, OAS1, IFIT1, HERC6, OLAH, MMP8 | 0,887 | 0,879 | 0,896 | 0,923 | 0,871 | 0,975 | 0,935 | 0,876 | 0,994 |
| IFI27, SIGLEC1, ISG15, HERC6, SLC1A2, IL1R2, FAM20A | 0,887 | 0,878 | 0,897 | 0,932 | 0,884 | 0,980 | 0,947 | 0,877 | 1 |
| IFI27, OAS1, IFI44L, ISG15, OLAH, FAM20A, RETN | 0,887 | 0,878 | 0,897 | 0,920 | 0,860 | 0,981 | 0,934 | 0,864 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, ISG15, HERC6, OLAH | 0,887 | 0,879 | 0,896 | 0,923 | 0,871 | 0,975 | 0,938 | 0,881 | 0,995 |
| IFI27, IFIT1, IFI44L, SIGLEC1, IL1R2, OLAH, MMP8 | 0,887 | 0,879 | 0,895 | 0,924 | 0,874 | 0,974 | 0,949 | 0,900 | 0,998 |
| RSAD2, IFI27, SIGLEC1, SLC1A2, ILIR2, FAM20A, RETN | 0,887 | 0,878 | 0,897 | 0,931 | 0,882 | 0,981 | 0,947 | 0,880 | 1 |
| RSAD2, IFI27, IFIT1, IFI44L, HERC6, OLAH, RETN | 0,887 | 0,879 | 0,896 | 0,923 | 0,868 | 0,977 | 0,936 | 0,875 | 0,996 |
| IFI27, IFI44L, SIGLEC1, ISG15, HERC6, IL1R2, RETN | 0,887 | 0,879 | 0,895 | 0,924 | 0,871 | 0,977 | 0,945 | 0,893 | 0,996 |
| IFI27, IFI44L, SIGLEC1, ISG15, SLC1A2, OLAH, MMP8 | 0,887 | 0,879 | 0,896 | 0,930 | 0,880 | 0,979 | 0,945 | 0,887 | 1 |
| RSAD2, SIGLEC1, HERC6, IL1R2, OLAH, FAM20A, MMP8 | 0,887 | 0,878 | 0,896 | 0,919 | 0,863 | 0,975 | 0,962 | 0,905 | 1 |
| IFI27, OAS1, IFI44L, SIGLEC1, ISG15, FAM20A, RETN | 0,887 | 0,878 | 0,897 | 0,920 | 0,861 | 0,980 | 0,946 | 0,877 | 1 |
| IFI27, OAS1, SIGLEC1, ISG15, HERC6, SLC1A2, OLAH | 0,887 | 0,878 | 0,896 | 0,930 | 0,881 | 0,980 | 0,937 | 0,877 | 0,997 |
| IFI27, OAS1, IFI44L, ISG15, HERC6, OLAH, RETN | 0,887 | 0,878 | 0,896 | 0,922 | 0,867 | 0,977 | 0,938 | 0,877 | 0,999 |
| RSAD2, IFI27, IFI44L, HERC6, IL1R2, OLAH, MMP8 | 0,887 | 0,879 | 0,896 | 0,926 | 0,877 | 0,976 | 0,936 | 0,878 | 0,993 |
| RSAD2, IFI27, IFIT1, ISG15, ILIR2, FAM20A, MMP8 | 0,887 | 0,878 | 0,896 | 0,925 | 0,873 | 0,978 | 0,933 | 0,854 | 1 |
| RSAD2, IFI27, IFIT1, ISG15, SLC1A2, OLAH, FAM20A | 0,887 | 0,878 | 0,896 | 0,925 | 0,870 | 0,980 | 0,921 | 0,841 | 1 |
| IFI27, OAS1, IFIT1, HERC6, IL1R2, RETN, MMP8 | 0,887 | 0,878 | 0,896 | 0,924 | 0,871 | 0,977 | 0,942 | 0,882 | 1 |
| IFI27, OAS1, IFIT1, ISG15, SLC1A2, OLAH, FAM20A | 0,887 | 0,878 | 0,896 | 0,925 | 0,869 | 0,980 | 0,924 | 0,848 | 0,999 |
| IFI27, OAS1, IFIT1, IFI44L, HERC6, OLAH, MMP8 | 0,887 | 0,878 | 0,896 | 0,926 | 0,874 | 0,977 | 0,935 | 0,876 | 0,994 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, HERC6, OLAH, MMP8 | 0,887 | 0,878 | 0,896 | 0,923 | 0,871 | 0,975 | 0,937 | 0,881 | 0,993 |
| RSAD2, IFI27, OAS1, IFIT1, IFI44L, IL1R2, FAM20A | 0,887 | 0,878 | 0,896 | 0,922 | 0,867 | 0,976 | 0,928 | 0,848 | 1 |
| OAS1, IFIT1, IFI44L, SIGLEC1, IL1R2, FAM20A, MMP8 | 0,887 | 0,878 | 0,896 | 0,920 | 0,863 | 0,978 | 0,965 | 0,912 | 1 |
| IFI44L, SIGLEC1, ISG15, HERC6, IL1R2, FAM20A, MMP8 | 0,887 | 0,878 | 0,896 | 0,918 | 0,860 | 0,975 | 0,959 | 0,897 | 1 |
| IFI27, OAS1, IFIT1, IFI44L, IL1R2, FAM20A, RETN | 0,887 | 0,878 | 0,896 | 0,923 | 0,869 | 0,977 | 0,930 | 0,852 | 1 |
| IFI27, OAS1, IFI44L, ISG15, ILIR2, FAM20A, MMP8 | 0,887 | 0,878 | 0,896 | 0,925 | 0,873 | 0,978 | 0,930 | 0,847 | 1 |
| IFIT1, IFI44L, SIGLEC1, SLC1A2, OLAH, FAM20A, MMP8 | 0,887 | 0,878 | 0,896 | 0,926 | 0,872 | 0,979 | 0,947 | 0,886 | 1 |
| IFI27, OAS1, IFIT1, IFI44L, SIGLEC1, ISG15, FAM20A | 0,887 | 0,878 | 0,896 | 0,915 | 0,850 | 0,979 | 0,943 | 0,871 | 1 |
| IFI27, OAS1, IFIT1, IFI44L, ISG15, HERC6, RETN | 0,887 | 0,878 | 0,896 | 0,916 | 0,856 | 0,976 | 0,935 | 0,869 | 1 |
| RSAD2, IFI27, IFIT1, HERC6, ILIR2, OLAH, RETN | 0,887 | 0,878 | 0,896 | 0,923 | 0,868 | 0,978 | 0,937 | 0,876 | 0,998 |
| IFI27, IFI44L, HERC6, SLC1A2, IL1R2, OLAH, FAM20A | 0,887 | 0,877 | 0,897 | 0,929 | 0,878 | 0,980 | 0,928 | 0,849 | 1 |
| IFI27, SIGLEC1, SLC1A2, IL1R2, OLAH, FAM20A, RETN | 0,887 | 0,878 | 0,896 | 0,927 | 0,872 | 0,981 | 0,945 | 0,877 | 1 |
| IFI27, IFIT1, SIGLEC1, HERC6, IL1R2, RETN, MMP8 | 0,887 | 0,878 | 0,895 | 0,921 | 0,867 | 0,976 | 0,957 | 0,914 | 0,999 |
| RSAD2, IFI27, IFI44L, SIGLEC1, HERC6, IL1R2, RETN | 0,887 | 0,879 | 0,895 | 0,924 | 0,871 | 0,977 | 0,945 | 0,893 | 0,996 |
| IFI27, IFI44L, ISG15, HERC6, IL1R2, OLAH, RETN | 0,887 | 0,878 | 0,895 | 0,923 | 0,869 | 0,977 | 0,941 | 0,885 | 0,997 |
| RSAD2, IFI27, IFI44L, IL1R2, OLAH, FAM20A, RETN | 0,887 | 0,877 | 0,897 | 0,924 | 0,866 | 0,981 | 0,929 | 0,856 | 1 |
| OAS1, IFI44L, SIGLEC1, HERC6, IL1R2, FAM20A, MMP8 | 0,887 | 0,878 | 0,896 | 0,919 | 0,863 | 0,976 | 0,960 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, HERC6, FAM20A, RETN | 0,887 | 0,877 | 0,897 | 0,923 | 0,866 | 0,981 | 0,934 | 0,853 | 1 |
| IFI27, OAS1, HERC6, SLC1A2, OLAH, RETN, MMP8 | 0,887 | 0,878 | 0,896 | 0,928 | 0,878 | 0,978 | 0,938 | 0,874 | 1 |
| RSAD2, IFI27, IFIT1, ISG15, OLAH, FAM20A, RETN | 0,887 | 0,878 | 0,896 | 0,920 | 0,860 | 0,980 | 0,933 | 0,862 | 1 |
| RSAD2, IFI27, OAS1, IL1R2, FAM20A, RETN, MMP8 | 0,887 | 0,878 | 0,896 | 0,930 | 0,878 | 0,982 | 0,935 | 0,856 | 1 |
| RSAD2, IFI27, IFIT1, ISG15, HERC6, FAM20A, RETN | 0,887 | 0,878 | 0,896 | 0,924 | 0,869 | 0,980 | 0,933 | 0,852 | 1 |
| RSAD2, IFI27, IFI44L, IL1R2, FAM20A, RETN, MMP8 | 0,887 | 0,877 | 0,896 | 0,928 | 0,875 | 0,981 | 0,932 | 0,851 | 1 |
| RSAD2, IFI27, IFIT1, IFI44L, SLC1A2, OLAH, FAM20A | 0,887 | 0,878 | 0,896 | 0,926 | 0,870 | 0,982 | 0,923 | 0,844 | 1 |
| RSAD2, IFI27, OAS1, IFIT1, HERC6, IL1R2, OLAH | 0,887 | 0,878 | 0,896 | 0,925 | 0,876 | 0,974 | 0,939 | 0,882 | 0,996 |
| RSAD2, IFI27, IFI44L, ISG15, ILIR2, FAM20A, MMP8 | 0,887 | 0,877 | 0,896 | 0,926 | 0,874 | 0,978 | 0,929 | 0,846 | 1 |
| IFI27, SIGLEC1, ISG15, SLC1A2, IL1R2, FAM20A, RETN | 0,887 | 0,878 | 0,896 | 0,932 | 0,884 | 0,980 | 0,946 | 0,876 | 1 |
| IFIT1, IFI44L, SIGLEC1, ISG15, HERC6, IL1R2, FAM20A | 0,887 | 0,878 | 0,895 | 0,918 | 0,861 | 0,976 | 0,953 | 0,897 | 1 |
| IFI27, OAS1, ISG15, SLC1A2, IL1R2, OLAH, FAM20A | 0,887 | 0,878 | 0,896 | 0,930 | 0,879 | 0,981 | 0,916 | 0,834 | 0,999 |
| RSAD2, IFI27, IFIT1, IFI44L, SIGLEC1, HERC6, OLAH | 0,887 | 0,878 | 0,895 | 0,924 | 0,872 | 0,977 | 0,947 | 0,897 | 0,997 |
| RSAD2, IFIT1, SIGLEC1, IL1R2, OLAH, FAM20A, MMP8 | 0,887 | 0,878 | 0,895 | 0,921 | 0,864 | 0,977 | 0,966 | 0,915 | 1 |
| IFI27, SIGLEC1, HERC6, ILIR2, OLAH, RETN, MMP8 | 0,887 | 0,878 | 0,896 | 0,920 | 0,862 | 0,978 | 0,960 | 0,912 | 1 |
| RSAD2, IFI27, IFI44L, ISG15, HERC6, FAM20A, RETN | 0,887 | 0,877 | 0,896 | 0,926 | 0,871 | 0,981 | 0,937 | 0,860 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, HERC6, IL1R2, OLAH, FAM20A, MMP8 | 0,887 | 0,878 | 0,896 | 0,918 | 0,861 | 0,976 | 0,961 | 0,903 | 1 |
| RSAD2, OAS1, IFIT1, SIGLEC1, IL1R2, FAM20A, MMP8 | 0,887 | 0,878 | 0,895 | 0,920 | 0,863 | 0,977 | 0,963 | 0,909 | 1 |
| IFI27, IFIT1, IFI44L, ISG15, IL1R2, FAM20A, RETN | 0,887 | 0,878 | 0,896 | 0,924 | 0,871 | 0,978 | 0,930 | 0,852 | 1 |
| IFI27, OAS1, IFI44L, SIGLEC1, FAM20A, RETN, MMP8 | 0,887 | 0,877 | 0,896 | 0,922 | 0,863 | 0,981 | 0,947 | 0,875 | 1 |
| RSAD2, IFI27, OAS1, ISG15, OLAH, FAM20A, RETN | 0,887 | 0,877 | 0,896 | 0,920 | 0,861 | 0,980 | 0,934 | 0,866 | 1 |
| RSAD2, IFI27, IFIT1, ISG15, HERC6, OLAH, RETN | 0,887 | 0,878 | 0,895 | 0,922 | 0,866 | 0,977 | 0,937 | 0,876 | 0,998 |
| RSAD2, OAS1, SIGLEC1, IL1R2, OLAH, FAM20A, MMP8 | 0,887 | 0,877 | 0,896 | 0,919 | 0,862 | 0,975 | 0,963 | 0,908 | 1 |
| RSAD2, IFI27, SIGLEC1, ISG15, HERC6, OLAH, MMP8 | 0,887 | 0,878 | 0,896 | 0,923 | 0,870 | 0,975 | 0,937 | 0,880 | 0,994 |
| IFI27, IFI44L, SLC1A2, IL1R2, OLAH, FAM20A, RETN | 0,887 | 0,877 | 0,896 | 0,927 | 0,873 | 0,982 | 0,921 | 0,838 | 1 |
| IFI27, OAS1, IFI44L, SIGLEC1, HERC6, IL1R2, MMP8 | 0,887 | 0,879 | 0,894 | 0,922 | 0,876 | 0,969 | 0,940 | 0,888 | 0,993 |
| IFI27, OAS1, SIGLEC1, HERC6, SLC1A2, FAM20A, RETN | 0,887 | 0,877 | 0,896 | 0,926 | 0,873 | 0,980 | 0,937 | 0,863 | 1 |
| IFIT1, IFI44L, SIGLEC1, HERC6, OLAH, FAM20A, MMP8 | 0,887 | 0,877 | 0,896 | 0,918 | 0,859 | 0,976 | 0,952 | 0,896 | 1 |
| IFI27, IFI44L, SIGLEC1, ISG15, SLC1A2, IL1R2, OLAH | 0,887 | 0,878 | 0,895 | 0,928 | 0,878 | 0,978 | 0,943 | 0,888 | 0,999 |
| IFIT1, SIGLEC1, ISG15, IL1R2, OLAH, FAM20A, MMP8 | 0,887 | 0,878 | 0,895 | 0,920 | 0,864 | 0,977 | 0,966 | 0,916 | 1 |
| IFI27, OAS1, IFIT1, SIGLEC1, OLAH, RETN, MMP8 | 0,887 | 0,877 | 0,896 | 0,920 | 0,861 | 0,978 | 0,962 | 0,911 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, OLAH | 0,887 | 0,878 | 0,895 | 0,925 | 0,873 | 0,977 | 0,946 | 0,894 | 0,998 |
| IFI27, IFIT1, SIGLEC1, ISG15, SLC1A2, OLAH, MMP8 | 0,887 | 0,878 | 0,895 | 0,930 | 0,881 | 0,980 | 0,938 | 0,878 | 0,998 |
| IFI27, IFIT1, IFI44L, SIGLEC1, ISG15, IL1R2, OLAH | 0,887 | 0,878 | 0,895 | 0,924 | 0,874 | 0,975 | 0,945 | 0,893 | 0,996 |
| RSAD2, IFI27, IFIT1, IFI44L, OLAH, FAM20A, RETN | 0,887 | 0,877 | 0,896 | 0,921 | 0,860 | 0,981 | 0,937 | 0,869 | 1 |
| IFIT1, IFI44L, SIGLEC1, HERC6, IL1R2, OLAH, FAM20A | 0,887 | 0,878 | 0,895 | 0,918 | 0,860 | 0,976 | 0,951 | 0,894 | 1 |
| IFI27, IFIT1, IFI44L, SIGLEC1, ISG15, FAM20A, MMP8 | 0,887 | 0,877 | 0,896 | 0,915 | 0,851 | 0,980 | 0,943 | 0,873 | 1 |
| RSAD2, IFI27, OAS1, IFIT1, HERC6, FAM20A, RETN | 0,887 | 0,877 | 0,896 | 0,923 | 0,865 | 0,981 | 0,934 | 0,853 | 1 |
| IFI27, SIGLEC1, ISG15, HERC6, FAM20A, RETN, MMP8 | 0,887 | 0,876 | 0,897 | 0,919 | 0,857 | 0,982 | 0,953 | 0,881 | 1 |
| RSAD2, IFI27, ISG15, HERC6, IL1R2, OLAH, RETN | 0,887 | 0,878 | 0,895 | 0,923 | 0,868 | 0,978 | 0,938 | 0,877 | 0,999 |
| IFI27, OAS1, IFI44L, SIGLEC1, IL1R2, OLAH, MMP8 | 0,886 | 0,878 | 0,895 | 0,923 | 0,872 | 0,974 | 0,949 | 0,900 | 0,997 |
| RSAD2, IFI27, OAS1, ISG15, HERC6, OLAH, MMP8 | 0,886 | 0,878 | 0,895 | 0,923 | 0,871 | 0,975 | 0,937 | 0,879 | 0,995 |
| RSAD2, IFI27, OAS1, SLC1A2, IL1R2, OLAH, FAM20A | 0,886 | 0,878 | 0,895 | 0,928 | 0,875 | 0,981 | 0,918 | 0,834 | 1 |
| RSAD2, IFI27, IFI44L, SIGLEC1, IL1R2, OLAH, MMP8 | 0,886 | 0,878 | 0,895 | 0,924 | 0,874 | 0,974 | 0,950 | 0,902 | 0,998 |
| RSAD2, IFI27, OAS1, IFI44L, HERC6, IL1R2, OLAH | 0,886 | 0,878 | 0,895 | 0,928 | 0,879 | 0,976 | 0,936 | 0,877 | 0,995 |
| IFI27, HERC6, SLC1A2, OLAH, FAM20A, RETN, MMP8 | 0,886 | 0,877 | 0,896 | 0,929 | 0,876 | 0,982 | 0,925 | 0,844 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, HERC6, IL1R2, OLAH, RETN | 0,886 | 0,878 | 0,895 | 0,922 | 0,867 | 0,977 | 0,935 | 0,872 | 0,998 |
| IFI27, OAS1, IFIT1, SIGLEC1, HERC6, IL1R2, MMP8 | 0,886 | 0,879 | 0,894 | 0,922 | 0,875 | 0,968 | 0,948 | 0,900 | 0,996 |
| IFI27, SIGLEC1, ISG15, SLC1A2, IL1R2, OLAH, MMP8 | 0,886 | 0,878 | 0,895 | 0,930 | 0,881 | 0,978 | 0,941 | 0,885 | 0,997 |
| RSAD2, IFI27, OAS1, IFI44L, HERC6, OLAH, MMP8 | 0,886 | 0,878 | 0,895 | 0,925 | 0,874 | 0,977 | 0,934 | 0,874 | 0,994 |
| IFI27, OAS1, IFIT1, SLC1A2, IL1R2, OLAH, FAM20A | 0,886 | 0,877 | 0,895 | 0,926 | 0,872 | 0,980 | 0,923 | 0,842 | 1 |
| RSAD2, IFI27, OAS1, IFIT1, HERC6, IL1R2, RETN | 0,886 | 0,878 | 0,895 | 0,922 | 0,868 | 0,976 | 0,945 | 0,885 | 1 |
| RSAD2, IFI27, SIGLEC1, ISG15, HERC6, OLAH, RETN | 0,886 | 0,878 | 0,895 | 0,921 | 0,866 | 0,977 | 0,942 | 0,886 | 0,999 |
| RSAD2, IFI27, IFI44L, SIGLEC1, FAM20A, RETN, MMP8 | 0,886 | 0,876 | 0,896 | 0,924 | 0,866 | 0,983 | 0,948 | 0,876 | 1 |
| RSAD2, IFI27, IFI44L, ISG15, OLAH, FAM20A, RETN | 0,886 | 0,877 | 0,896 | 0,921 | 0,861 | 0,981 | 0,934 | 0,864 | 1 |
| RSAD2, IFI27, IFIT1, HERC6, IL1R2, OLAH, MMP8 | 0,886 | 0,878 | 0,895 | 0,925 | 0,876 | 0,975 | 0,940 | 0,885 | 0,995 |
| IFI27, OAS1, SIGLEC1, IL1R2, OLAH, RETN, MMP8 | 0,886 | 0,877 | 0,895 | 0,922 | 0,867 | 0,978 | 0,961 | 0,910 | 1 |
| IFI27, HERC6, SLC1A2, IL1R2, OLAH, FAM20A, RETN | 0,886 | 0,877 | 0,895 | 0,929 | 0,876 | 0,981 | 0,927 | 0,848 | 1 |
| RSAD2, IFI27, OAS1, ISG15, HERC6, IL1R2, OLAH | 0,886 | 0,878 | 0,895 | 0,925 | 0,876 | 0,975 | 0,938 | 0,880 | 0,996 |
| IFI27, OAS1, SIGLEC1, HERC6, SLC1A2, IL1R2, OLAH | 0,886 | 0,877 | 0,895 | 0,928 | 0,879 | 0,977 | 0,941 | 0,882 | 1 |
| RSAD2, IFI27, OAS1, IFIT1, IFI44L, SIGLEC1, FAM20A | 0,886 | 0,877 | 0,895 | 0,915 | 0,851 | 0,980 | 0,946 | 0,877 | 1 |
| IFI27, OAS1, IFI44L, ISG15, HERC6, OLAH, MMP8 | 0,886 | 0,877 | 0,895 | 0,924 | 0,872 | 0,976 | 0,932 | 0,870 | 0,993 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, ISG15, HERC6, OLAH, MMP8 | 0,886 | 0,877 | 0,895 | 0,923 | 0,871 | 0,975 | 0,937 | 0,879 | 0,995 |
| OAS1, IFIT1, IFI44L, SIGLEC1, HERC6, IL1R2, FAM20A | 0,886 | 0,878 | 0,895 | 0,918 | 0,861 | 0,976 | 0,950 | 0,893 | 1 |
| RSAD2, IFI27, IFI44L, SLC1A2, IL1R2, OLAH, FAM20A | 0,886 | 0,877 | 0,896 | 0,927 | 0,873 | 0,982 | 0,922 | 0,841 | 1 |
| IFI27, OAS1, ISG15, IL1R2, FAM20A, RETN, MMP8 | 0,886 | 0,877 | 0,895 | 0,929 | 0,877 | 0,980 | 0,934 | 0,855 | 1 |
| RSAD2, IFI27, OAS1, IFI44L, OLAH, FAM20A, RETN | 0,886 | 0,877 | 0,896 | 0,921 | 0,861 | 0,982 | 0,936 | 0,867 | 1 |
| IFI27, IFI44L, SIGLEC1, ISG15, OLAH, RETN, MMP8 | 0,886 | 0,877 | 0,895 | 0,921 | 0,865 | 0,978 | 0,959 | 0,908 | 1 |
| RSAD2, IFI27, OAS1, HERC6, IL1R2, RETN, MMP8 | 0,886 | 0,878 | 0,895 | 0,920 | 0,865 | 0,975 | 0,940 | 0,878 | 1 |
| RSAD2, IFI27, OAS1, IFIT1, OLAH, FAM20A, RETN | 0,886 | 0,877 | 0,895 | 0,919 | 0,858 | 0,980 | 0,937 | 0,869 | 1 |
| RSAD2, IFI27, OAS1, SIGLEC1, ISG15, FAM20A, RETN | 0,886 | 0,876 | 0,896 | 0,919 | 0,858 | 0,979 | 0,948 | 0,881 | 1 |
| IFI27, IFIT1, SIGLEC1, IL1R2, OLAH, RETN, MMP8 | 0,886 | 0,877 | 0,895 | 0,921 | 0,864 | 0,978 | 0,961 | 0,913 | 1 |
| IFI27, OAS1, IFIT1, SIGLEC1, IL1R2, OLAH, MMP8 | 0,886 | 0,878 | 0,894 | 0,922 | 0,871 | 0,973 | 0,947 | 0,896 | 0,998 |
| RSAD2, IFI27, OAS1, SIGLEC1, ISG15, HERC6, OLAH | 0,886 | 0,877 | 0,895 | 0,923 | 0,871 | 0,976 | 0,938 | 0,881 | 0,995 |
| IFI27, IFIT1, SIGLEC1, ISG15, SLC1A2, IL1R2, OLAH | 0,886 | 0,878 | 0,895 | 0,929 | 0,879 | 0,978 | 0,939 | 0,880 | 0,998 |
| IFI27, IFIT1, SIGLEC1, ISG15, OLAH, RETN, MMP8 | 0,886 | 0,877 | 0,895 | 0,920 | 0,861 | 0,979 | 0,958 | 0,905 | 1 |
| RSAD2, IFI27, ISG15, HERC6, IL1R2, OLAH, MMP8 | 0,886 | 0,878 | 0,895 | 0,924 | 0,874 | 0,975 | 0,939 | 0,884 | 0,995 |
| IFI27, OAS1, IFIT1, IFI44L, ISG15, IL1R2, FAM20A | 0,886 | 0,877 | 0,895 | 0,923 | 0,869 | 0,977 | 0,928 | 0,848 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SLC1A2, IL1R2, OLAH, FAM20A | 0,886 | 0,877 | 0,895 | 0,926 | 0,872 | 0,980 | 0,922 | 0,841 | 1 |
| IFI27, OAS1, IFIT1, SIGLEC1, HERC6, SLC1A2, OLAH | 0,886 | 0,877 | 0,895 | 0,927 | 0,877 | 0,978 | 0,941 | 0,883 | 1,000 |
| RSAD2, IFI27, IFI44L, HERC6, IL1R2, OLAH, RETN | 0,886 | 0,877 | 0,895 | 0,924 | 0,870 | 0,978 | 0,936 | 0,876 | 0,996 |
| IFI27, IFIT1, IFI44L, ISG15, HERC6, OLAH, MMP8 | 0,886 | 0,877 | 0,895 | 0,922 | 0,868 | 0,976 | 0,935 | 0,876 | 0,993 |
| IFI27, IFIT1, ISG15, SLC1A2, OLAH, FAM20A, RETN | 0,886 | 0,877 | 0,895 | 0,925 | 0,869 | 0,981 | 0,922 | 0,844 | 1,000 |
| IFIT1, IFI44L, SIGLEC1, ISG15, IL1R2, FAM20A, MMP8 | 0,886 | 0,877 | 0,895 | 0,920 | 0,864 | 0,977 | 0,967 | 0,916 | 1 |
| RSAD2, IFI27, SLC1A2, OLAH, FAM20A, RETN, MMP8 | 0,886 | 0,876 | 0,896 | 0,927 | 0,872 | 0,982 | 0,924 | 0,844 | 1 |
| RSAD2, IFI27, OAS1, IFIT1, SLC1A2, OLAH, FAM20A | 0,886 | 0,877 | 0,895 | 0,924 | 0,867 | 0,981 | 0,922 | 0,841 | 1 |
| IFI27, IFIT1, SLC1A2, OLAH, FAM20A, RETN, MMP8 | 0,886 | 0,876 | 0,896 | 0,926 | 0,870 | 0,981 | 0,929 | 0,854 | 1 |
| RSAD2, IFI27, OAS1, IFIT1, ISG15, HERC6, RETN | 0,886 | 0,877 | 0,895 | 0,919 | 0,859 | 0,979 | 0,934 | 0,865 | 1 |
| IFI27, IFIT1, SIGLEC1, ISG15, IL1R2, OLAH, MMP8 | 0,886 | 0,878 | 0,894 | 0,923 | 0,872 | 0,974 | 0,943 | 0,892 | 0,995 |
| IFI27, IFIT1, HERC6, SLC1A2, OLAH, FAM20A, RETN | 0,886 | 0,876 | 0,896 | 0,928 | 0,875 | 0,982 | 0,922 | 0,839 | 1 |
| IFI27, IFI44L, SIGLEC1, ISG15, IL1R2, OLAH, RETN | 0,886 | 0,878 | 0,894 | 0,921 | 0,867 | 0,975 | 0,952 | 0,900 | 1 |
| IFIT1, SIGLEC1, ISG15, HERC6, IL1R2, OLAH, FAM20A | 0,886 | 0,877 | 0,895 | 0,919 | 0,862 | 0,977 | 0,952 | 0,895 | 1 |
| IFI27, OAS1, IFIT1, IFI44L, SIGLEC1, HERC6, IL1R2 | 0,886 | 0,878 | 0,894 | 0,923 | 0,876 | 0,970 | 0,939 | 0,886 | 0,992 |
| IFI27, SIGLEC1, ISG15, IL1R2, OLAH, RETN, MMP8 | 0,886 | 0,877 | 0,895 | 0,921 | 0,865 | 0,978 | 0,960 | 0,911 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, ISG15, SLC1A2, IL1R2, FAM20A, MMP8 | 0,886 | 0,877 | 0,895 | 0,930 | 0,883 | 0,976 | 0,926 | 0,839 | 1 |
| RSAD2, IFI27, OAS1, SIGLEC1, ISG15, HERC6, FAM20A | 0,886 | 0,876 | 0,895 | 0,918 | 0,858 | 0,977 | 0,940 | 0,868 | 1 |
| RSAD2, IFI27, OAS1, SIGLEC1, HERC6, FAM20A, MMP8 | 0,886 | 0,876 | 0,896 | 0,916 | 0,856 | 0,976 | 0,941 | 0,871 | 1 |
| RSAD2, IFI27, IFIT1, SIGLEC1, HERC6, IL1R2, RETN | 0,886 | 0,878 | 0,894 | 0,923 | 0,871 | 0,975 | 0,949 | 0,901 | 0,997 |
| RSAD2, IFI27, OAS1, IFI44L, SLC1A2, OLAH, FAM20A | 0,886 | 0,876 | 0,895 | 0,929 | 0,874 | 0,984 | 0,923 | 0,843 | 1 |
| OAS1, IFIT1, SIGLEC1, ISG15, IL1R2, FAM20A, MMP8 | 0,886 | 0,877 | 0,895 | 0,918 | 0,860 | 0,976 | 0,965 | 0,910 | 1 |
| IFI27, OAS1, IFI44L, SIGLEC1, ISG15, IL1R2, OLAH | 0,886 | 0,878 | 0,894 | 0,923 | 0,872 | 0,974 | 0,942 | 0,889 | 0,996 |
| IFI27, OAS1, SIGLEC1, ISG15, SLC1A2, OLAH, MMP8 | 0,886 | 0,877 | 0,894 | 0,929 | 0,878 | 0,979 | 0,941 | 0,883 | 0,999 |
| IFI27, IFIT1, IFI44L, HERC6, FAM20A, RETN, MMP8 | 0,886 | 0,876 | 0,896 | 0,921 | 0,862 | 0,980 | 0,932 | 0,849 | 1 |
| OAS1, IFIT1, SIGLEC1, HERC6, IL1R2, OLAH, FAM20A | 0,886 | 0,877 | 0,895 | 0,919 | 0,861 | 0,976 | 0,952 | 0,895 | 1 |
| IFI27, IFIT1, IFI44L, SIGLEC1, OLAH, RETN, MMP8 | 0,886 | 0,876 | 0,895 | 0,921 | 0,863 | 0,979 | 0,959 | 0,908 | 1 |
| IFI27, OAS1, IFIT1, ISG15, HERC6, IL1R2, RETN | 0,886 | 0,877 | 0,894 | 0,924 | 0,872 | 0,976 | 0,941 | 0,881 | 1 |
| RSAD2, IFI27, ISG15, SLC1A2, OLAH, FAM20A, RETN | 0,886 | 0,876 | 0,895 | 0,927 | 0,873 | 0,981 | 0,916 | 0,833 | 1,000 |
| IFI27, IFIT1, IFI44L, SLC1A2, OLAH, FAM20A, RETN | 0,886 | 0,876 | 0,895 | 0,925 | 0,868 | 0,982 | 0,922 | 0,841 | 1 |
| RSAD2, IFI27, OAS1, SIGLEC1, HERC6, SLC1A2, OLAH | 0,886 | 0,876 | 0,895 | 0,928 | 0,878 | 0,978 | 0,941 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, HERC6, IL1R2 | 0,886 | 0,878 | 0,894 | 0,925 | 0,878 | 0,972 | 0,946 | 0,896 | 0,995 |
| IFI27, IFI44L, SIGLEC1, ISG15, SLC1A2, OLAH, RETN | 0,886 | 0,877 | 0,894 | 0,928 | 0,877 | 0,979 | 0,947 | 0,889 | 1 |
| RSAD2, IFI44L, SIGLEC1, HERC6, IL1R2, FAM20A, MMP8 | 0,886 | 0,877 | 0,894 | 0,919 | 0,863 | 0,975 | 0,960 | 0,900 | 1 |
| IFI27, IFIT1, ISG15, HERC6, IL1R2, OLAH, RETN | 0,886 | 0,877 | 0,894 | 0,921 | 0,865 | 0,977 | 0,941 | 0,884 | 0,999 |
| RSAD2, IFI27, ISG15, HERC6, FAM20A, RETN, MMP8 | 0,886 | 0,876 | 0,895 | 0,927 | 0,871 | 0,982 | 0,934 | 0,854 | 1 |
| IFI27, SIGLEC1, HERC6, SLC1A2, OLAH, FAM20A, RETN | 0,886 | 0,876 | 0,895 | 0,926 | 0,869 | 0,983 | 0,943 | 0,876 | 1 |
| IFI27, OAS1, IFI44L, HERC6, FAM20A, RETN, MMP8 | 0,886 | 0,876 | 0,896 | 0,924 | 0,865 | 0,983 | 0,935 | 0,855 | 1 |
| IFI27, OAS1, IFI44L, ISG15, IL1R2, FAM20A, RETN | 0,886 | 0,877 | 0,895 | 0,925 | 0,872 | 0,978 | 0,929 | 0,850 | 1 |
| IFI27, IFIT1, SIGLEC1, ISG15, HERC6, SLC1A2, OLAH | 0,886 | 0,877 | 0,895 | 0,930 | 0,880 | 0,980 | 0,939 | 0,881 | 0,997 |
| IFI27, OAS1, IFI44L, ISG15, HERC6, IL1R2, OLAH | 0,886 | 0,877 | 0,894 | 0,927 | 0,878 | 0,976 | 0,934 | 0,874 | 0,993 |
| IFI27, OAS1, IFI44L, SIGLEC1, OLAH, RETN, MMP8 | 0,886 | 0,877 | 0,894 | 0,920 | 0,863 | 0,978 | 0,959 | 0,908 | 1 |
| RSAD2, IFI27, OAS1, ISG15, IL1R2, FAM20A, MMP8 | 0,886 | 0,877 | 0,895 | 0,927 | 0,875 | 0,978 | 0,928 | 0,843 | 1 |
| IFI27, IFI44L, SLC1A2, IL1R2, FAM20A, RETN, MMP8 | 0,886 | 0,877 | 0,895 | 0,930 | 0,880 | 0,980 | 0,924 | 0,836 | 1 |
| IFI27, OAS1, IFIT1, ISG15, IL1R2, FAM20A, RETN | 0,886 | 0,877 | 0,895 | 0,926 | 0,873 | 0,978 | 0,930 | 0,852 | 1 |
| IFIT1, SIGLEC1, HERC6, OLAH, FAM20A, RETN, MMP8 | 0,886 | 0,876 | 0,895 | 0,922 | 0,864 | 0,980 | 0,954 | 0,898 | 1 |
| IFI44L, SIGLEC1, ISG15, IL1R2, OLAH, FAM20A, MMP8 | 0,886 | 0,877 | 0,894 | 0,917 | 0,860 | 0,974 | 0,963 | 0,908 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SLC1A2, IL1R2, OLAH, FAM20A | 0,886 | 0,876 | 0,895 | 0,929 | 0,876 | 0,983 | 0,918 | 0,836 | 1 |
| RSAD2, IFI27, OAS1, IFI44L, ISG15, IL1R2, FAM20A | 0,886 | 0,877 | 0,894 | 0,923 | 0,869 | 0,977 | 0,930 | 0,851 | 1 |
| IFI27, OAS1, IFIT1, SIGLEC1, HERC6, SLC1A2, RETN | 0,886 | 0,877 | 0,894 | 0,926 | 0,874 | 0,978 | 0,940 | 0,880 | 1 |
| RSAD2, IFI27, IFIT1, IFI44L, ISG15, IL1R2, FAM20A | 0,885 | 0,877 | 0,894 | 0,922 | 0,868 | 0,977 | 0,928 | 0,848 | 1 |
| IFI27, OAS1, ISG15, SLC1A2, OLAH, FAM20A, RETN | 0,885 | 0,876 | 0,895 | 0,927 | 0,873 | 0,981 | 0,916 | 0,834 | 0,999 |
| RSAD2, IFI27, IFI44L, ISG15, HERC6, OLAH, RETN | 0,885 | 0,877 | 0,894 | 0,923 | 0,868 | 0,977 | 0,935 | 0,874 | 0,996 |
| OAS1, SIGLEC1, HERC6, IL1R2, OLAH, FAM20A, MMP8 | 0,885 | 0,876 | 0,895 | 0,919 | 0,863 | 0,975 | 0,963 | 0,908 | 1 |
| IFI27, IFIT1, IFI44L, SIGLEC1, ISG15, HERC6, RETN | 0,885 | 0,877 | 0,894 | 0,912 | 0,851 | 0,973 | 0,940 | 0,886 | 0,995 |
| RSAD2, IFI27, OAS1, SIGLEC1, HERC6, IL1R2, MMP8 | 0,885 | 0,877 | 0,894 | 0,922 | 0,874 | 0,969 | 0,951 | 0,905 | 0,997 |
| IFI27, OAS1, IFIT1, IFI44L, HERC6, IL1R2, OLAH | 0,885 | 0,877 | 0,894 | 0,926 | 0,877 | 0,975 | 0,939 | 0,883 | 0,995 |
| RSAD2, IFIT1, SIGLEC1, ISG15, IL1R2, FAM20A, MMP8 | 0,885 | 0,877 | 0,894 | 0,920 | 0,862 | 0,977 | 0,966 | 0,913 | 1 |
| IFI27, OAS1, IFIT1, IFI44L, SIGLEC1, IL1R2, OLAH | 0,885 | 0,877 | 0,893 | 0,922 | 0,871 | 0,973 | 0,948 | 0,897 | 0,998 |
| IFI27, SIGLEC1, ISG15, HERC6, SLC1A2, OLAH, MMP8 | 0,885 | 0,876 | 0,895 | 0,930 | 0,880 | 0,980 | 0,939 | 0,880 | 0,998 |
| IFI27, OAS1, IFIT1, SIGLEC1, IL1R2, OLAH, RETN | 0,885 | 0,877 | 0,894 | 0,919 | 0,864 | 0,975 | 0,948 | 0,892 | 1 |
| RSAD2, IFI27, OAS1, HERC6, FAM20A, RETN, MMP8 | 0,885 | 0,875 | 0,895 | 0,923 | 0,865 | 0,981 | 0,934 | 0,853 | 1 |
| RSAD2, IFI27, SIGLEC1, IL1R2, OLAH, RETN, MMP8 | 0,885 | 0,876 | 0,894 | 0,922 | 0,867 | 0,978 | 0,959 | 0,908 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, SLC1A2, FAM20A, RETN, MMP8 | 0,885 | 0,876 | 0,895 | 0,925 | 0,870 | 0,981 | 0,946 | 0,869 | 1 |
| SIGLEC1, ISG15, HERC6, IL1R2, OLAH, FAM20A, MMP8 | 0,885 | 0,876 | 0,895 | 0,916 | 0,858 | 0,974 | 0,964 | 0,909 | 1 |
| RSAD2, IFI27, IFI44L, SIGLEC1, ISG15, IL1R2, OLAH | 0,885 | 0,877 | 0,893 | 0,925 | 0,874 | 0,975 | 0,943 | 0,891 | 0,996 |
| IFI27, OAS1, ISG15, HERC6, IL1R2, RETN, MMP8 | 0,885 | 0,876 | 0,894 | 0,924 | 0,871 | 0,977 | 0,937 | 0,874 | 1,000 |
| RSAD2, IFI27, IFIT1, SIGLEC1, ISG15, HERC6, OLAH | 0,885 | 0,877 | 0,894 | 0,924 | 0,873 | 0,976 | 0,939 | 0,884 | 0,994 |
| IFI27, IFIT1, IFI44L, HERC6, IL1R2, OLAH, RETN | 0,885 | 0,877 | 0,894 | 0,922 | 0,868 | 0,977 | 0,943 | 0,888 | 0,999 |
| RSAD2, IFI27, OAS1, IFI44L, SIGLEC1, HERC6, FAM20A | 0,885 | 0,876 | 0,894 | 0,917 | 0,857 | 0,977 | 0,942 | 0,870 | 1 |
| IFI27, OAS1, SIGLEC1, SLC1A2, IL1R2, OLAH, MMP8 | 0,885 | 0,877 | 0,894 | 0,927 | 0,878 | 0,976 | 0,947 | 0,892 | 1 |
| IFI27, OAS1, HERC6, SLC1A2, IL1R2, OLAH, MMP8 | 0,885 | 0,877 | 0,894 | 0,927 | 0,878 | 0,976 | 0,929 | 0,862 | 0,997 |
| RSAD2, IFI27, OAS1, ISG15, HERC6, IL1R2, RETN | 0,885 | 0,877 | 0,894 | 0,922 | 0,868 | 0,976 | 0,942 | 0,881 | 1 |
| RSAD2, IFI27, SLC1A2, IL1R2, OLAH, FAM20A, RETN | 0,885 | 0,876 | 0,894 | 0,927 | 0,873 | 0,981 | 0,920 | 0,836 | 1 |
| RSAD2, IFI27, OAS1, IFIT1, IL1R2, FAM20A, RETN | 0,885 | 0,876 | 0,894 | 0,924 | 0,870 | 0,977 | 0,929 | 0,850 | 1 |
| RSAD2, SIGLEC1, ISG15, HERC6, IL1R2, FAM20A, MMP8 | 0,885 | 0,876 | 0,894 | 0,919 | 0,862 | 0,975 | 0,961 | 0,902 | 1 |
| RSAD2, IFI27, OAS1, IFI44L, ISG15, HERC6, OLAH | 0,885 | 0,876 | 0,894 | 0,925 | 0,873 | 0,976 | 0,937 | 0,879 | 0,995 |
| IFIT1, IFI44L, SIGLEC1, HERC6, FAM20A, RETN, MMP8 | 0,885 | 0,876 | 0,894 | 0,919 | 0,858 | 0,980 | 0,945 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, FAM20A, RETN | 0,885 | 0,876 | 0,895 | 0,919 | 0,860 | 0,979 | 0,948 | 0,881 | 1 |
| IFI27, IFI44L, SIGLEC1, SLC1A2, IL1R2, OLAH, MMP8 | 0,885 | 0,877 | 0,894 | 0,927 | 0,877 | 0,977 | 0,951 | 0,901 | 1 |
| RSAD2, IFI27, IFIT1, IFI44L, SIGLEC1, ISG15, FAM20A | 0,885 | 0,876 | 0,894 | 0,915 | 0,850 | 0,980 | 0,945 | 0,874 | 1 |
| IFI27, OAS1, IFI44L, HERC6, IL1R2, RETN, MMP8 | 0,885 | 0,877 | 0,894 | 0,922 | 0,869 | 0,975 | 0,936 | 0,873 | 0,999 |
| IFI27, OAS1, IFI44L, ISG15, HERC6, IL1R2, RETN | 0,885 | 0,877 | 0,894 | 0,922 | 0,870 | 0,974 | 0,938 | 0,877 | 0,999 |
| IFI27, OAS1, IFIT1, IFI44L, ISG15, HERC6, OLAH | 0,885 | 0,876 | 0,894 | 0,925 | 0,873 | 0,976 | 0,934 | 0,874 | 0,994 |
| RSAD2, IFI27, OAS1, IFIT1, IFI44L, HERC6, OLAH | 0,885 | 0,876 | 0,894 | 0,926 | 0,874 | 0,977 | 0,937 | 0,880 | 0,994 |
| RSAD2, IFI27, OAS1, IFI44L, SIGLEC1, FAM20A, RETN | 0,885 | 0,876 | 0,895 | 0,918 | 0,858 | 0,979 | 0,947 | 0,879 | 1 |
| RSAD2, IFI27, OAS1, HERC6, SLC1A2, OLAH, RETN | 0,885 | 0,876 | 0,894 | 0,927 | 0,877 | 0,978 | 0,932 | 0,860 | 1 |
| IFI27, OAS1, IFIT1, IFI44L, SIGLEC1, OLAH, RETN | 0,885 | 0,877 | 0,894 | 0,916 | 0,856 | 0,975 | 0,949 | 0,889 | 1 |
| IFI27, OAS1, SLC1A2, OLAH, FAM20A, RETN, MMP8 | 0,885 | 0,875 | 0,895 | 0,927 | 0,873 | 0,981 | 0,925 | 0,846 | 1 |
| RSAD2, IFI27, OAS1, IFI44L, HERC6, IL1R2, RETN | 0,885 | 0,877 | 0,893 | 0,920 | 0,866 | 0,975 | 0,940 | 0,877 | 1 |
| RSAD2, IFI27, IFIT1, IFI44L, HERC6, OLAH, MMP8 | 0,885 | 0,877 | 0,894 | 0,925 | 0,873 | 0,977 | 0,933 | 0,873 | 0,992 |
| IFI27, IFIT1, IFI44L, SIGLEC1, ISG15, OLAH, MMP8 | 0,885 | 0,876 | 0,894 | 0,920 | 0,865 | 0,976 | 0,942 | 0,887 | 0,998 |
| RSAD2, IFI27, OAS1, IFIT1, ISG15, HERC6, OLAH | 0,885 | 0,876 | 0,894 | 0,923 | 0,872 | 0,975 | 0,935 | 0,876 | 0,994 |
| IFI27, OAS1, IFIT1, IFI44L, HERC6, IL1R2, RETN | 0,885 | 0,877 | 0,893 | 0,923 | 0,871 | 0,975 | 0,938 | 0,878 | 0,998 |
| IFIT1, IFI44L, SIGLEC1, IL1R2, FAM20A, RETN, MMP8 | 0,885 | 0,876 | 0,894 | 0,922 | 0,866 | 0,978 | 0,968 | 0,918 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, ISG15, HERC6, IL1R2, OLAH | 0,885 | 0,877 | 0,894 | 0,924 | 0,874 | 0,974 | 0,938 | 0,882 | 0,994 |
| RSAD2, IFI27, OAS1, SIGLEC1, IL1R2, OLAH, MMP8 | 0,885 | 0,876 | 0,894 | 0,922 | 0,871 | 0,973 | 0,947 | 0,896 | 0,998 |
| OAS1, IFIT1, SIGLEC1, IL1R2, FAM20A, RETN, MMP8 | 0,885 | 0,876 | 0,894 | 0,922 | 0,865 | 0,978 | 0,968 | 0,918 | 1 |
| IFI27, OAS1, IFIT1, IL1R2, OLAH, RETN, MMP8 | 0,885 | 0,876 | 0,894 | 0,921 | 0,864 | 0,977 | 0,954 | 0,902 | 1 |
| IFI27, IFIT1, SIGLEC1, ISG15, IL1R2, OLAH, RETN | 0,885 | 0,877 | 0,893 | 0,919 | 0,863 | 0,974 | 0,947 | 0,891 | 1 |
| IFI27, IFIT1, ISG15, HERC6, IL1R2, OLAH, MMP8 | 0,885 | 0,877 | 0,893 | 0,924 | 0,875 | 0,974 | 0,942 | 0,888 | 0,997 |
| IFI27, IFIT1, IFI44L, SLC1A2, IL1R2, FAM20A, MMP8 | 0,885 | 0,876 | 0,894 | 0,928 | 0,880 | 0,976 | 0,930 | 0,846 | 1 |
| RSAD2, OAS1, IFIT1, SIGLEC1, HERC6, IL1R2, FAM20A | 0,885 | 0,876 | 0,894 | 0,918 | 0,860 | 0,976 | 0,952 | 0,895 | 1 |
| RSAD2, IFI27, OAS1, SIGLEC1, OLAH, RETN, MMP8 | 0,885 | 0,876 | 0,894 | 0,920 | 0,861 | 0,978 | 0,961 | 0,908 | 1 |
| IFI27, IFIT1, IFI44L, HERC6, IL1R2, OLAH, MMP8 | 0,885 | 0,877 | 0,893 | 0,927 | 0,877 | 0,977 | 0,936 | 0,879 | 0,993 |
| IFI27, IFI44L, SIGLEC1, ISG15, IL1R2, RETN, MMP8 | 0,885 | 0,877 | 0,893 | 0,923 | 0,871 | 0,976 | 0,962 | 0,922 | 1 |
| RSAD2, IFI27, IFIT1, IFI44L, SIGLEC1, HERC6, RETN | 0,885 | 0,877 | 0,893 | 0,915 | 0,856 | 0,974 | 0,937 | 0,880 | 0,994 |
| RSAD2, IFI27, ISG15, IL1R2, FAM20A, RETN, MMP8 | 0,885 | 0,876 | 0,894 | 0,927 | 0,874 | 0,980 | 0,933 | 0,853 | 1 |
| IFIT1, SIGLEC1, HERC6, SLC1A2, IL1R2, FAM20A, MMP8 | 0,885 | 0,876 | 0,894 | 0,928 | 0,882 | 0,974 | 0,959 | 0,895 | 1 |
| RSAD2, IFI27, SIGLEC1, ISG15, HERC6, SLC1A2, OLAH | 0,885 | 0,876 | 0,894 | 0,929 | 0,879 | 0,979 | 0,938 | 0,879 | 0,997 |
| IFI27, OAS1, IFIT1, ISG15, HERC6, IL1R2, OLAH | 0,885 | 0,876 | 0,894 | 0,923 | 0,873 | 0,974 | 0,939 | 0,882 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, HERC6, SLC1A2, OLAH, MMP8 | 0,885 | 0,875 | 0,894 | 0,928 | 0,877 | 0,978 | 0,938 | 0,880 | 0,996 |
| RSAD2, IFI27, HERC6, SLC1A2, OLAH, RETN, MMP8 | 0,885 | 0,876 | 0,894 | 0,928 | 0,877 | 0,979 | 0,941 | 0,881 | 1 |
| IFI27, IFIT1, SIGLEC1, ISG15, SLC1A2, FAM20A, RETN | 0,885 | 0,876 | 0,894 | 0,926 | 0,871 | 0,981 | 0,938 | 0,862 | 1 |
| IFI27, OAS1, SIGLEC1, ISG15, IL1R2, OLAH, MMP8 | 0,885 | 0,876 | 0,893 | 0,923 | 0,872 | 0,974 | 0,946 | 0,895 | 0,996 |
| RSAD2, IFI27, SIGLEC1, SLC1A2, IL1R2, OLAH, MMP8 | 0,885 | 0,876 | 0,894 | 0,927 | 0,878 | 0,977 | 0,946 | 0,891 | 1,000 |
| IFI27, IFIT1, IFI44L, ISG15, HERC6, IL1R2, OLAH | 0,885 | 0,877 | 0,893 | 0,925 | 0,874 | 0,976 | 0,935 | 0,876 | 0,993 |
| IFIT1, IFI44L, SIGLEC1, ISG15, HERC6, OLAH, FAM20A | 0,885 | 0,875 | 0,895 | 0,916 | 0,856 | 0,976 | 0,946 | 0,887 | 1 |
| RSAD2, IFI27, IFI44L, ISG15, HERC6, IL1R2, OLAH | 0,885 | 0,876 | 0,893 | 0,926 | 0,877 | 0,976 | 0,937 | 0,879 | 0,995 |
| IFI27, IFIT1, SLC1A2, IL1R2, OLAH, FAM20A, RETN | 0,885 | 0,876 | 0,894 | 0,926 | 0,871 | 0,980 | 0,922 | 0,841 | 1 |
| OAS1, IFIT1, SIGLEC1, ISG15, HERC6, IL1R2, FAM20A | 0,885 | 0,876 | 0,894 | 0,919 | 0,863 | 0,976 | 0,953 | 0,897 | 1 |
| IFI27, OAS1, IFIT1, SIGLEC1, ISG15, SLC1A2, OLAH | 0,885 | 0,876 | 0,894 | 0,928 | 0,877 | 0,979 | 0,940 | 0,882 | 0,999 |
| RSAD2, IFI27, IFI44L, ISG15, HERC6, OLAH, MMP8 | 0,885 | 0,876 | 0,894 | 0,923 | 0,871 | 0,976 | 0,932 | 0,871 | 0,992 |
| RSAD2, IFI27, SIGLEC1, ISG15, SLC1A2, OLAH, MMP8 | 0,885 | 0,876 | 0,894 | 0,929 | 0,879 | 0,979 | 0,939 | 0,880 | 0,999 |
| OAS1, SIGLEC1, ISG15, HERC6, IL1R2, FAM20A, MMP8 | 0,885 | 0,876 | 0,894 | 0,916 | 0,858 | 0,974 | 0,961 | 0,902 | 1 |
| IFI27, SIGLEC1, ISG15, HERC6, IL1R2, OLAH, RETN | 0,885 | 0,876 | 0,894 | 0,920 | 0,863 | 0,977 | 0,946 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, HERC6, SLC1A2, IL1R2, RETN | 0,885 | 0,876 | 0,893 | 0,928 | 0,881 | 0,975 | 0,945 | 0,887 | 1 |
| OAS1, IFIT1, SIGLEC1, HERC6, OLAH, FAM20A, MMP8 | 0,885 | 0,875 | 0,894 | 0,916 | 0,858 | 0,975 | 0,953 | 0,899 | 1 |
| IFI27, OAS1, IFIT1, HERC6, SLC1A2, OLAH, RETN | 0,885 | 0,875 | 0,894 | 0,926 | 0,876 | 0,977 | 0,935 | 0,869 | 1 |
| RSAD2, IFIT1, IFI44L, SIGLEC1, IL1R2, FAM20A, MMP8 | 0,885 | 0,876 | 0,894 | 0,922 | 0,866 | 0,978 | 0,966 | 0,915 | 1 |
| IFI27, OAS1, IFIT1, SIGLEC1, SLC1A2, OLAH, MMP8 | 0,885 | 0,876 | 0,894 | 0,927 | 0,876 | 0,979 | 0,945 | 0,888 | 1 |
| IFI27, IFIT1, ISG15, HERC6, FAM20A, RETN, MMP8 | 0,885 | 0,875 | 0,895 | 0,923 | 0,866 | 0,980 | 0,936 | 0,857 | 1 |
| IFI27, OAS1, HERC6, SLC1A2, IL1R2, OLAH, RETN | 0,885 | 0,876 | 0,894 | 0,930 | 0,880 | 0,980 | 0,930 | 0,862 | 0,999 |
| RSAD2, IFI27, IFI44L, SIGLEC1, HERC6, IL1R2, MMP8 | 0,885 | 0,877 | 0,892 | 0,923 | 0,874 | 0,971 | 0,940 | 0,888 | 0,992 |
| IFI27, IFI44L, HERC6, SLC1A2, OLAH, FAM20A, RETN | 0,885 | 0,875 | 0,894 | 0,928 | 0,873 | 0,983 | 0,926 | 0,847 | 1 |
| IFI27, IFIT1, SIGLEC1, ISG15, HERC6, IL1R2, RETN | 0,885 | 0,877 | 0,893 | 0,922 | 0,869 | 0,975 | 0,951 | 0,905 | 0,998 |
| IFI27, OAS1, SIGLEC1, ISG15, OLAH, RETN, MMP8 | 0,885 | 0,875 | 0,894 | 0,920 | 0,861 | 0,979 | 0,960 | 0,907 | 1 |
| IFIT1, SIGLEC1, IL1R2, OLAH, FAM20A, RETN, MMP8 | 0,885 | 0,876 | 0,894 | 0,921 | 0,865 | 0,978 | 0,970 | 0,921 | 1 |
| IFI27, IFIT1, IFI44L, SIGLEC1, IL1R2, RETN, MMP8 | 0,885 | 0,876 | 0,893 | 0,925 | 0,873 | 0,977 | 0,961 | 0,920 | 1 |
| IFI27, IFIT1, SIGLEC1, SLC1A2, IL1R2, OLAH, MMP8 | 0,885 | 0,876 | 0,894 | 0,927 | 0,877 | 0,977 | 0,948 | 0,895 | 1 |
| IFI27, OAS1, ISG15, HERC6, SLC1A2, OLAH, RETN | 0,885 | 0,875 | 0,894 | 0,928 | 0,878 | 0,978 | 0,932 | 0,863 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, ISG15, SLC1A2, OLAH, RETN | 0,885 | 0,876 | 0,893 | 0,929 | 0,878 | 0,980 | 0,940 | 0,879 | 1 |
| RSAD2, IFI27, IFIT1, IFI44L, HERC6, IL1R2, OLAH | 0,885 | 0,876 | 0,893 | 0,927 | 0,877 | 0,977 | 0,937 | 0,881 | 0,993 |
| IFI27, IFI44L, ISG15, IL1R2, OLAH, RETN, MMP8 | 0,885 | 0,876 | 0,894 | 0,923 | 0,868 | 0,977 | 0,951 | 0,897 | 1 |
| IFI27, OAS1, SIGLEC1, SLC1A2, OLAH, RETN, MMP8 | 0,885 | 0,875 | 0,894 | 0,927 | 0,874 | 0,979 | 0,957 | 0,901 | 1 |
| IFI27, OAS1, ISG15, HERC6, SLC1A2, OLAH, MMP8 | 0,885 | 0,875 | 0,894 | 0,928 | 0,879 | 0,978 | 0,923 | 0,853 | 0,993 |
| IFI27, OAS1, IFIT1, HERC6, FAM20A, RETN, MMP8 | 0,885 | 0,875 | 0,895 | 0,924 | 0,866 | 0,982 | 0,934 | 0,853 | 1 |
| IFI27, IFIT1, IFI44L, SIGLEC1, ISG15, SLC1A2, OLAH | 0,885 | 0,876 | 0,893 | 0,930 | 0,880 | 0,980 | 0,941 | 0,882 | 1 |
| RSAD2, IFI27, OAS1, IFI44L, SIGLEC1, IL1R2, OLAH | 0,885 | 0,876 | 0,893 | 0,922 | 0,870 | 0,974 | 0,949 | 0,899 | 0,999 |
| RSAD2, IFIT1, IFI44L, SIGLEC1, HERC6, IL1R2, FAM20A | 0,885 | 0,876 | 0,893 | 0,919 | 0,861 | 0,976 | 0,952 | 0,895 | 1 |
| IFI27, OAS1, HERC6, SLC1A2, IL1R2, FAM20A, MMP8 | 0,885 | 0,875 | 0,894 | 0,931 | 0,884 | 0,977 | 0,925 | 0,839 | 1 |
| IFI27, IFIT1, IFI44L, SIGLEC1, SLC1A2, FAM20A, RETN | 0,885 | 0,875 | 0,894 | 0,923 | 0,868 | 0,979 | 0,940 | 0,865 | 1 |
| RSAD2, IFI27, OAS1, IFIT1, SIGLEC1, HERC6, IL1R2 | 0,885 | 0,876 | 0,893 | 0,922 | 0,876 | 0,969 | 0,952 | 0,907 | 0,997 |
| IFIT1, SIGLEC1, ISG15, HERC6, OLAH, FAM20A, MMP8 | 0,885 | 0,875 | 0,894 | 0,918 | 0,859 | 0,976 | 0,951 | 0,895 | 1 |
| IFI27, OAS1, IFIT1, SIGLEC1, IL1R2, RETN, MMP8 | 0,885 | 0,876 | 0,893 | 0,923 | 0,870 | 0,976 | 0,963 | 0,918 | 1 |
| RSAD2, IFI27, SIGLEC1, HERC6, SLC1A2, IL1R2, OLAH | 0,885 | 0,875 | 0,894 | 0,927 | 0,878 | 0,977 | 0,941 | 0,885 | 0,998 |
| RSAD2, IFI27, HERC6, SLC1A2, IL1R2, FAM20A, MMP8 | 0,885 | 0,876 | 0,893 | 0,930 | 0,884 | 0,977 | 0,926 | 0,840 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, HERC6, IL1R2, OLAH, FAM20A | 0,884 | 0,875 | 0,894 | 0,918 | 0,859 | 0,977 | 0,952 | 0,897 | 1 |
| IFI27, OAS1, IFI44L, SIGLEC1, IL1R2, OLAH, RETN | 0,884 | 0,876 | 0,893 | 0,920 | 0,865 | 0,975 | 0,951 | 0,899 | 1 |
| IFI27, IFIT1, SIGLEC1, HERC6, SLC1A2, FAM20A, MMP8 | 0,884 | 0,876 | 0,893 | 0,926 | 0,875 | 0,978 | 0,938 | 0,860 | 1 |
| IFI27, IFI44L, SIGLEC1, SLC1A2, OLAH, RETN, MMP8 | 0,884 | 0,875 | 0,894 | 0,928 | 0,875 | 0,981 | 0,959 | 0,908 | 1 |
| RSAD2, IFI27, SIGLEC1, ISG15, HERC6, IL1R2, RETN | 0,884 | 0,876 | 0,893 | 0,921 | 0,868 | 0,974 | 0,950 | 0,901 | 0,999 |
| RSAD2, IFIT1, SIGLEC1, ISG15, HERC6, IL1R2, FAM20A | 0,884 | 0,876 | 0,893 | 0,918 | 0,860 | 0,976 | 0,954 | 0,898 | 1 |
| IFIT1, SIGLEC1, SLC1A2, IL1R2, OLAH, FAM20A, MMP8 | 0,884 | 0,876 | 0,893 | 0,926 | 0,878 | 0,975 | 0,962 | 0,906 | 1 |
| RSAD2, IFI27, OAS1, HERC6, SLC1A2, OLAH, MMP8 | 0,884 | 0,875 | 0,894 | 0,928 | 0,878 | 0,978 | 0,932 | 0,864 | 0,999 |
| IFI27, IFI44L, ISG15, SLC1A2, IL1R2, OLAH, MMP8 | 0,884 | 0,876 | 0,893 | 0,927 | 0,878 | 0,977 | 0,927 | 0,858 | 0,997 |
| IFI27, IFI44L, SIGLEC1, SLC1A2, IL1R2, OLAH, RETN | 0,884 | 0,876 | 0,893 | 0,926 | 0,874 | 0,977 | 0,950 | 0,894 | 1 |
| RSAD2, IFI27, OAS1, IFI44L, ISG15, HERC6, RETN | 0,884 | 0,875 | 0,893 | 0,919 | 0,859 | 0,979 | 0,937 | 0,869 | 1 |
| IFI27, OAS1, IFIT1, SLC1A2, OLAH, FAM20A, RETN | 0,884 | 0,875 | 0,894 | 0,924 | 0,867 | 0,981 | 0,925 | 0,847 | 1 |
| IFI27, OAS1, IFI44L, SIGLEC1, ISG15, OLAH, MMP8 | 0,884 | 0,876 | 0,893 | 0,919 | 0,863 | 0,976 | 0,939 | 0,884 | 0,995 |
| RSAD2, IFI27, OAS1, IFIT1, ISG15, IL1R2, FAM20A | 0,884 | 0,875 | 0,893 | 0,923 | 0,870 | 0,977 | 0,929 | 0,850 | 1 |
| RSAD2, IFI27, SIGLEC1, ISG15, SLC1A2, IL1R2, OLAH | 0,884 | 0,876 | 0,893 | 0,929 | 0,880 | 0,979 | 0,939 | 0,880 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, HERC6, SLC1A2, OLAH, RETN, MMP8 | 0,884 | 0,875 | 0,894 | 0,927 | 0,874 | 0,980 | 0,942 | 0,883 | 1 |
| IFI27, IFIT1, IFI44L, SIGLEC1, ISG15, OLAH, RETN | 0,884 | 0,876 | 0,893 | 0,920 | 0,863 | 0,977 | 0,946 | 0,885 | 1 |
| IFIT1, IFI44L, SIGLEC1, HERC6, IL1R2, FAM20A, RETN | 0,884 | 0,876 | 0,893 | 0,918 | 0,861 | 0,976 | 0,952 | 0,895 | 1 |
| IFI27, OAS1, IFIT1, HERC6, SLC1A2, OLAH, MMP8 | 0,884 | 0,875 | 0,894 | 0,929 | 0,879 | 0,978 | 0,933 | 0,867 | 0,998 |
| IFI27, OAS1, IFIT1, SIGLEC1, ISG15, IL1R2, OLAH | 0,884 | 0,876 | 0,892 | 0,921 | 0,869 | 0,973 | 0,943 | 0,889 | 0,998 |
| RSAD2, IFI27, SIGLEC1, ISG15, HERC6, FAM20A, MMP8 | 0,884 | 0,875 | 0,894 | 0,918 | 0,857 | 0,978 | 0,940 | 0,868 | 1 |
| RSAD2, IFI27, IFIT1, SIGLEC1, OLAH, RETN, MMP8 | 0,884 | 0,875 | 0,893 | 0,919 | 0,860 | 0,978 | 0,962 | 0,911 | 1 |
| RSAD2, IFI27, HERC6, SLC1A2, IL1R2, OLAH, MMP8 | 0,884 | 0,875 | 0,893 | 0,926 | 0,877 | 0,976 | 0,929 | 0,864 | 0,995 |
| IFI27, IFIT1, IFI44L, SIGLEC1, IL1R2, OLAH, RETN | 0,884 | 0,876 | 0,893 | 0,920 | 0,865 | 0,975 | 0,952 | 0,899 | 1 |
| IFI27, IFIT1, IFI44L, SIGLEC1, HERC6, IL1R2, MMP8 | 0,884 | 0,876 | 0,892 | 0,922 | 0,874 | 0,970 | 0,940 | 0,888 | 0,992 |
| RSAD2, IFI27, SIGLEC1, ISG15, IL1R2, OLAH, MMP8 | 0,884 | 0,876 | 0,893 | 0,923 | 0,872 | 0,974 | 0,945 | 0,894 | 0,995 |
| IFI27, OAS1, SIGLEC1, SLC1A2, FAM20A, RETN, MMP8 | 0,884 | 0,874 | 0,894 | 0,923 | 0,865 | 0,980 | 0,942 | 0,868 | 1 |
| IFI27, OAS1, IFIT1, SIGLEC1, ISG15, OLAH, RETN | 0,884 | 0,876 | 0,893 | 0,917 | 0,860 | 0,975 | 0,947 | 0,890 | 1 |
| IFI27, IFIT1, IFI44L, SIGLEC1, ISG15, HERC6, IL1R2 | 0,884 | 0,876 | 0,892 | 0,922 | 0,874 | 0,970 | 0,942 | 0,891 | 0,993 |
| IFI27, SIGLEC1, ISG15, HERC6, SLC1A2, IL1R2, OLAH | 0,884 | 0,875 | 0,893 | 0,929 | 0,880 | 0,979 | 0,937 | 0,877 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, HERC6, SLC1A2, OLAH, RETN | 0,884 | 0,875 | 0,893 | 0,928 | 0,878 | 0,978 | 0,946 | 0,890 | 1 |
| IFI27, OAS1, IFIT1, IFI44L, SIGLEC1, FAM20A, MMP8 | 0,884 | 0,875 | 0,894 | 0,914 | 0,848 | 0,979 | 0,947 | 0,880 | 1 |
| IFI27, IFI44L, SIGLEC1, ISG15, HERC6, SLC1A2, OLAH | 0,884 | 0,875 | 0,893 | 0,930 | 0,880 | 0,980 | 0,940 | 0,883 | 0,997 |
| RSAD2, IFI27, IFIT1, IFI44L, IL1R2, FAM20A, RETN | 0,884 | 0,875 | 0,894 | 0,923 | 0,869 | 0,977 | 0,930 | 0,852 | 1 |
| IFI27, OAS1, IFIT1, IFI44L, SIGLEC1, IL1R2, RETN | 0,884 | 0,876 | 0,892 | 0,922 | 0,869 | 0,974 | 0,950 | 0,899 | 1 |
| IFI27, OAS1, SIGLEC1, SLC1A2, IL1R2, OLAH, RETN | 0,884 | 0,875 | 0,893 | 0,927 | 0,877 | 0,978 | 0,950 | 0,890 | 1 |
| IFI27, OAS1, SLC1A2, IL1R2, OLAH, FAM20A, RETN | 0,884 | 0,875 | 0,893 | 0,927 | 0,873 | 0,981 | 0,920 | 0,836 | 1 |
| RSAD2, SIGLEC1, HERC6, SLC1A2, IL1R2, FAM20A, MMP8 | 0,884 | 0,874 | 0,894 | 0,925 | 0,877 | 0,972 | 0,959 | 0,895 | 1 |
| IFI27, SIGLEC1, ISG15, SLC1A2, IL1R2, OLAH, RETN | 0,884 | 0,875 | 0,893 | 0,928 | 0,877 | 0,979 | 0,941 | 0,882 | 1 |
| IFI27, IFIT1, ISG15, IL1R2, OLAH, RETN, MMP8 | 0,884 | 0,875 | 0,893 | 0,920 | 0,864 | 0,976 | 0,951 | 0,898 | 1 |
| RSAD2, IFI27, OAS1, IFIT1, OLAH, RETN, MMP8 | 0,884 | 0,875 | 0,893 | 0,919 | 0,859 | 0,978 | 0,952 | 0,896 | 1 |
| RSAD2, IFI27, IFIT1, SIGLEC1, IL1R2, OLAH, MMP8 | 0,884 | 0,876 | 0,893 | 0,922 | 0,872 | 0,973 | 0,949 | 0,900 | 0,998 |
| RSAD2, IFI27, OAS1, IFIT1, SIGLEC1, ISG15, FAM20A | 0,884 | 0,875 | 0,893 | 0,916 | 0,852 | 0,980 | 0,941 | 0,869 | 1 |
| IFI27, OAS1, IFIT1, SIGLEC1, HERC6, SLC1A2, FAM20A | 0,884 | 0,875 | 0,893 | 0,928 | 0,879 | 0,977 | 0,936 | 0,859 | 1 |
| IFI27, OAS1, IFI44L, SLC1A2, OLAH, FAM20A, RETN | 0,884 | 0,875 | 0,894 | 0,927 | 0,870 | 0,984 | 0,923 | 0,843 | 1 |
| OAS1, IFIT1, IFI44L, SIGLEC1, HERC6, OLAH, FAM20A | 0,884 | 0,875 | 0,893 | 0,916 | 0,856 | 0,975 | 0,942 | 0,882 | 1 |
| IFI44L, SIGLEC1, SLC1A2, IL1R2, OLAH, FAM20A, MMP8 | 0,884 | 0,875 | 0,893 | 0,923 | 0,874 | 0,972 | 0,959 | 0,897 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, SLC1A2, IL1R2, FAM20A, MMP8 | 0,884 | 0,875 | 0,893 | 0,927 | 0,880 | 0,974 | 0,961 | 0,899 | 1 |
| IFI27, HERC6, SLC1A2, IL1R2, FAM20A, RETN, MMP8 | 0,884 | 0,875 | 0,893 | 0,934 | 0,886 | 0,982 | 0,928 | 0,843 | 1 |
| IFI27, IFIT1, IFI44L, SIGLEC1, ISG15, IL1R2, RETN | 0,884 | 0,876 | 0,892 | 0,922 | 0,869 | 0,974 | 0,947 | 0,894 | 0,999 |
| IFI27, OAS1, IFIT1, SIGLEC1, SLC1A2, IL1R2, OLAH | 0,884 | 0,875 | 0,893 | 0,927 | 0,877 | 0,977 | 0,946 | 0,889 | 1 |
| RSAD2, IFI27, IFIT1, SIGLEC1, ISG15, HERC6, RETN | 0,884 | 0,876 | 0,892 | 0,916 | 0,858 | 0,974 | 0,946 | 0,893 | 0,998 |
| IFI27, OAS1, IFIT1, IFI44L, SIGLEC1, ISG15, OLAH | 0,884 | 0,876 | 0,893 | 0,918 | 0,861 | 0,975 | 0,938 | 0,880 | 0,996 |
| IFIT1, SIGLEC1, ISG15, HERC6, FAM20A, RETN, MMP8 | 0,884 | 0,875 | 0,893 | 0,918 | 0,857 | 0,978 | 0,950 | 0,885 | 1 |
| IFI27, OAS1, IFIT1, ISG15, HERC6, SLC1A2, OLAH | 0,884 | 0,874 | 0,894 | 0,929 | 0,879 | 0,978 | 0,930 | 0,864 | 0,997 |
| IFIT1, IFI44L, SIGLEC1, ISG15, HERC6, FAM20A, RETN | 0,884 | 0,875 | 0,893 | 0,917 | 0,856 | 0,978 | 0,943 | 0,881 | 1 |
| RSAD2, IFI27, IFIT1, ISG15, IL1R2, FAM20A, RETN | 0,884 | 0,875 | 0,893 | 0,924 | 0,871 | 0,978 | 0,929 | 0,850 | 1 |
| IFI27, OAS1, IFI44L, HERC6, SLC1A2, IL1R2, FAM20A | 0,884 | 0,874 | 0,894 | 0,931 | 0,885 | 0,976 | 0,925 | 0,837 | 1 |
| IFI27, OAS1, IFIT1, HERC6, SLC1A2, IL1R2, FAM20A | 0,884 | 0,874 | 0,893 | 0,930 | 0,883 | 0,977 | 0,924 | 0,838 | 1 |
| IFI27, IFI44L, SIGLEC1, ISG15, HERC6, IL1R2, MMP8 | 0,884 | 0,877 | 0,891 | 0,923 | 0,876 | 0,970 | 0,938 | 0,885 | 0,991 |
| IFI27, OAS1, HERC6, SLC1A2, IL1R2, FAM20A, RETN | 0,884 | 0,875 | 0,893 | 0,934 | 0,886 | 0,981 | 0,924 | 0,838 | 1 |
| RSAD2, IFI27, SIGLEC1, HERC6, SLC1A2, FAM20A, RETN | 0,884 | 0,874 | 0,894 | 0,926 | 0,873 | 0,979 | 0,945 | 0,871 | 1 |
| RSAD2, IFI27, OAS1, IL1R2, OLAH, RETN, MMP8 | 0,884 | 0,875 | 0,893 | 0,921 | 0,865 | 0,977 | 0,953 | 0,902 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, FAM20A | 0,884 | 0,875 | 0,893 | 0,918 | 0,858 | 0,977 | 0,943 | 0,871 | 1 |
| RSAD2, IFI27, OAS1, IFI44L, IL1R2, FAM20A, RETN | 0,884 | 0,875 | 0,893 | 0,924 | 0,871 | 0,978 | 0,930 | 0,851 | 1 |
| IFI27, OAS1, SIGLEC1, ISG15, SLC1A2, IL1R2, OLAH | 0,884 | 0,875 | 0,893 | 0,930 | 0,881 | 0,978 | 0,941 | 0,883 | 1,000 |
| RSAD2, IFI27, IFI44L, HERC6, SLC1A2, IL1R2, FAM20A | 0,884 | 0,875 | 0,893 | 0,928 | 0,880 | 0,976 | 0,925 | 0,838 | 1 |
| IFI27, SIGLEC1, ISG15, SLC1A2, OLAH, RETN, MMP8 | 0,884 | 0,875 | 0,893 | 0,928 | 0,875 | 0,980 | 0,953 | 0,900 | 1 |
| RSAD2, IFI27, OAS1, SIGLEC1, ISG15, IL1R2, OLAH | 0,884 | 0,875 | 0,892 | 0,921 | 0,869 | 0,973 | 0,945 | 0,891 | 0,998 |
| IFI27, OAS1, ISG15, HERC6, SLC1A2, IL1R2, FAM20A | 0,884 | 0,874 | 0,893 | 0,931 | 0,885 | 0,977 | 0,921 | 0,833 | 1 |
| IFI27, OAS1, IFIT1, IFI44L, SIGLEC1, OLAH, MMP8 | 0,884 | 0,875 | 0,892 | 0,920 | 0,864 | 0,976 | 0,950 | 0,900 | 1,000 |
| IFI27, OAS1, IFI44L, ISG15, HERC6, RETN, MMP8 | 0,884 | 0,875 | 0,893 | 0,918 | 0,858 | 0,977 | 0,935 | 0,868 | 1 |
| IFI27, IFIT1, IFI44L, IL1R2, OLAH, RETN, MMP8 | 0,884 | 0,875 | 0,893 | 0,922 | 0,866 | 0,977 | 0,950 | 0,895 | 1 |
| RSAD2, IFI27, IFI44L, ISG15, IL1R2, FAM20A, RETN | 0,884 | 0,874 | 0,893 | 0,924 | 0,871 | 0,978 | 0,926 | 0,845 | 1 |
| RSAD2, IFI27, IFIT1, IFI44L, SIGLEC1, IL1R2, OLAH | 0,884 | 0,876 | 0,892 | 0,922 | 0,870 | 0,974 | 0,948 | 0,898 | 0,998 |
| OAS1, SIGLEC1, HERC6, SLC1A2, IL1R2, FAM20A, MMP8 | 0,884 | 0,874 | 0,893 | 0,923 | 0,874 | 0,971 | 0,960 | 0,896 | 1 |
| IFI27, OAS1, IFIT1, SIGLEC1, ISG15, OLAH, MMP8 | 0,884 | 0,875 | 0,892 | 0,919 | 0,863 | 0,975 | 0,941 | 0,885 | 0,998 |
| IFI27, OAS1, IFIT1, ISG15, HERC6, RETN, MMP8 | 0,884 | 0,874 | 0,893 | 0,917 | 0,858 | 0,976 | 0,935 | 0,868 | 1 |
| RSAD2, IFI27, OAS1, SIGLEC1, SLC1A2, OLAH, MMP8 | 0,884 | 0,875 | 0,893 | 0,928 | 0,876 | 0,980 | 0,948 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, HERC6, SLC1A2, OLAH, RETN | 0,884 | 0,874 | 0,893 | 0,924 | 0,871 | 0,977 | 0,947 | 0,892 | 1 |
| IFI27, IFIT1, SLC1A2, IL1R2, FAM20A, RETN, MMP8 | 0,884 | 0,875 | 0,893 | 0,932 | 0,883 | 0,981 | 0,934 | 0,853 | 1 |
| RSAD2, IFI27, IFIT1, SLC1A2, OLAH, FAM20A, RETN | 0,884 | 0,875 | 0,893 | 0,923 | 0,866 | 0,981 | 0,927 | 0,851 | 1 |
| IFI27, IFIT1, SIGLEC1, HERC6, SLC1A2, OLAH, RETN | 0,884 | 0,874 | 0,893 | 0,927 | 0,876 | 0,978 | 0,948 | 0,894 | 1 |
| IFI27, OAS1, IFIT1, ISG15, OLAH, RETN, MMP8 | 0,884 | 0,874 | 0,893 | 0,918 | 0,858 | 0,977 | 0,950 | 0,893 | 1 |
| IFI27, IFIT1, HERC6, SLC1A2, IL1R2, FAM20A, MMP8 | 0,884 | 0,874 | 0,893 | 0,929 | 0,881 | 0,976 | 0,929 | 0,845 | 1 |
| RSAD2, IFIT1, SIGLEC1, HERC6, OLAH, FAM20A, MMP8 | 0,884 | 0,874 | 0,893 | 0,916 | 0,858 | 0,975 | 0,954 | 0,900 | 1 |
| IFI27, IFIT1, SIGLEC1, HERC6, SLC1A2, OLAH, MMP8 | 0,884 | 0,874 | 0,893 | 0,929 | 0,879 | 0,980 | 0,940 | 0,884 | 0,996 |
| IFI27, IFIT1, SIGLEC1, SLC1A2, OLAH, RETN, MMP8 | 0,884 | 0,874 | 0,893 | 0,928 | 0,877 | 0,980 | 0,955 | 0,901 | 1 |
| IFI27, IFIT1, IFI44L, SIGLEC1, HERC6, SLC1A2, FAM20A | 0,884 | 0,875 | 0,892 | 0,927 | 0,875 | 0,979 | 0,941 | 0,864 | 1 |
| RSAD2, IFI27, IFI44L, SLC1A2, IL1R2, FAM20A, MMP8 | 0,884 | 0,875 | 0,893 | 0,929 | 0,882 | 0,976 | 0,926 | 0,839 | 1 |
| IFI27, SIGLEC1, HERC6, SLC1A2, OLAH, RETN, MMP8 | 0,884 | 0,874 | 0,893 | 0,924 | 0,868 | 0,979 | 0,953 | 0,900 | 1 |
| RSAD2, IFI27, IFIT1, ISG15, HERC6, OLAH, MMP8 | 0,884 | 0,875 | 0,893 | 0,923 | 0,871 | 0,976 | 0,935 | 0,876 | 0,994 |
| IFI27, SIGLEC1, HERC6, SLC1A2, IL1R2, FAM20A, RETN | 0,884 | 0,874 | 0,893 | 0,930 | 0,879 | 0,980 | 0,947 | 0,875 | 1 |
| RSAD2, IFI27, OAS1, SLC1A2, OLAH, FAM20A, RETN | 0,884 | 0,874 | 0,893 | 0,925 | 0,869 | 0,982 | 0,920 | 0,836 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, HERC6, SLC1A2, IL1R2, FAM20A, RETN | 0,884 | 0,875 | 0,893 | 0,933 | 0,885 | 0,981 | 0,925 | 0,840 | 1 |
| RSAD2, IFI27, OAS1, IFIT1, SIGLEC1, IL1R2, OLAH | 0,884 | 0,875 | 0,892 | 0,920 | 0,868 | 0,973 | 0,942 | 0,888 | 0,996 |
| RSAD2, IFI27, IFIT1, SLC1A2, IL1R2, FAM20A, MMP8 | 0,884 | 0,875 | 0,892 | 0,927 | 0,878 | 0,976 | 0,926 | 0,841 | 1 |
| IFIT1, SIGLEC1, HERC6, SLC1A2, OLAH, FAM20A, MMP8 | 0,884 | 0,873 | 0,894 | 0,926 | 0,877 | 0,976 | 0,948 | 0,879 | 1 |
| IFI27, OAS1, IFIT1, SIGLEC1, SLC1A2, FAM20A, RETN | 0,884 | 0,874 | 0,893 | 0,924 | 0,868 | 0,980 | 0,941 | 0,868 | 1 |
| IFI27, OAS1, SIGLEC1, ISG15, HERC6, FAM20A, MMP8 | 0,884 | 0,874 | 0,894 | 0,915 | 0,855 | 0,976 | 0,940 | 0,868 | 1 |
| RSAD2, IFI27, OAS1, IFIT1, IL1R2, OLAH, MMP8 | 0,884 | 0,875 | 0,892 | 0,922 | 0,870 | 0,973 | 0,946 | 0,894 | 0,997 |
| RSAD2, IFI27, OAS1, ISG15, IL1R2, FAM20A, RETN | 0,884 | 0,875 | 0,893 | 0,926 | 0,874 | 0,978 | 0,929 | 0,850 | 1 |
| RSAD2, IFI27, OAS1, SIGLEC1, IL1R2, OLAH, RETN | 0,884 | 0,875 | 0,892 | 0,918 | 0,862 | 0,974 | 0,951 | 0,895 | 1 |
| OAS1, SIGLEC1, HERC6, IL1R2, FAM20A, RETN, MMP8 | 0,884 | 0,874 | 0,893 | 0,919 | 0,861 | 0,976 | 0,963 | 0,907 | 1 |
| RSAD2, IFI27, IFI44L, SIGLEC1, OLAH, RETN, MMP8 | 0,884 | 0,875 | 0,892 | 0,920 | 0,862 | 0,978 | 0,959 | 0,908 | 1 |
| IFI27, OAS1, SIGLEC1, ISG15, HERC6, IL1R2, MMP8 | 0,884 | 0,875 | 0,892 | 0,921 | 0,874 | 0,968 | 0,949 | 0,902 | 0,996 |
| OAS1, IFIT1, SIGLEC1, HERC6, FAM20A, RETN, MMP8 | 0,884 | 0,875 | 0,892 | 0,918 | 0,858 | 0,978 | 0,941 | 0,873 | 1 |
| RSAD2, IFI27, OAS1, IFI44L, SIGLEC1, ISG15, OLAH | 0,884 | 0,875 | 0,892 | 0,919 | 0,862 | 0,976 | 0,942 | 0,887 | 0,997 |
| IFI27, ISG15, SLC1A2, IL1R2, OLAH, RETN, MMP8 | 0,884 | 0,875 | 0,892 | 0,928 | 0,877 | 0,978 | 0,936 | 0,869 | 1 |
| RSAD2, IFI27, OAS1, SIGLEC1, HERC6, SLC1A2, RETN | 0,884 | 0,875 | 0,892 | 0,922 | 0,866 | 0,977 | 0,945 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, HERC6, SLC1A2, OLAH | 0,884 | 0,874 | 0,893 | 0,927 | 0,876 | 0,977 | 0,942 | 0,885 | 0,999 |
| IFI44L, SIGLEC1, HERC6, IL1R2, FAM20A, RETN, MMP8 | 0,884 | 0,874 | 0,893 | 0,918 | 0,861 | 0,976 | 0,959 | 0,896 | 1 |
| RSAD2, IFI27, OAS1, SIGLEC1, ISG15, SLC1A2, OLAH | 0,884 | 0,875 | 0,892 | 0,929 | 0,878 | 0,980 | 0,943 | 0,886 | 1 |
| RSAD2, IFI27, OAS1, SIGLEC1, SLC1A2, IL1R2, OLAH | 0,884 | 0,875 | 0,892 | 0,925 | 0,874 | 0,976 | 0,950 | 0,894 | 1 |
| IFIT1, IFI44L, SIGLEC1, HERC6, OLAH, FAM20A, RETN | 0,884 | 0,874 | 0,893 | 0,916 | 0,856 | 0,977 | 0,945 | 0,886 | 1 |
| RSAD2, IFI27, OAS1, IFI44L, SIGLEC1, OLAH, MMP8 | 0,884 | 0,875 | 0,892 | 0,918 | 0,861 | 0,975 | 0,951 | 0,901 | 1 |
| RSAD2, IFI44L, SIGLEC1, IL1R2, OLAH, FAM20A, MMP8 | 0,884 | 0,874 | 0,893 | 0,918 | 0,861 | 0,975 | 0,963 | 0,908 | 1 |
| IFI27, OAS1, IFIT1, SIGLEC1, ISG15, HERC6, IL1R2 | 0,884 | 0,875 | 0,892 | 0,922 | 0,875 | 0,968 | 0,950 | 0,904 | 0,996 |
| IFI27, OAS1, IFI44L, SIGLEC1, ISG15, HERC6, FAM20A | 0,884 | 0,875 | 0,892 | 0,915 | 0,856 | 0,975 | 0,946 | 0,872 | 1 |
| RSAD2, IFI27, IFI44L, HERC6, FAM20A, RETN, MMP8 | 0,884 | 0,873 | 0,894 | 0,925 | 0,867 | 0,983 | 0,935 | 0,855 | 1 |
| IFI27, OAS1, SIGLEC1, ISG15, SLC1A2, OLAH, RETN | 0,884 | 0,875 | 0,892 | 0,929 | 0,879 | 0,980 | 0,943 | 0,883 | 1 |
| RSAD2, IFI27, OAS1, HERC6, SLC1A2, IL1R2, OLAH | 0,884 | 0,874 | 0,893 | 0,929 | 0,881 | 0,977 | 0,935 | 0,869 | 1 |
| IFI27, OAS1, ISG15, IL1R2, OLAH, RETN, MMP8 | 0,884 | 0,874 | 0,893 | 0,922 | 0,867 | 0,978 | 0,948 | 0,894 | 1 |
| IFI27, OAS1, IFI44L, IL1R2, OLAH, RETN, MMP8 | 0,883 | 0,874 | 0,893 | 0,922 | 0,867 | 0,978 | 0,950 | 0,896 | 1 |
| IFI27, OAS1, SIGLEC1, ISG15, HERC6, SLC1A2, RETN | 0,883 | 0,875 | 0,892 | 0,920 | 0,865 | 0,976 | 0,946 | 0,884 | 1 |
| IFI27, OAS1, IFI44L, SIGLEC1, ISG15, HERC6, IL1R2 | 0,883 | 0,876 | 0,891 | 0,922 | 0,876 | 0,968 | 0,939 | 0,886 | 0,992 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, HERC6, IL1R2 | 0,883 | 0,876 | 0,891 | 0,921 | 0,872 | 0,970 | 0,941 | 0,890 | 0,993 |
| IFI27, OAS1, IFIT1, IFI44L, OLAH, RETN, MMP8 | 0,883 | 0,874 | 0,893 | 0,921 | 0,862 | 0,980 | 0,951 | 0,893 | 1 |
| IFI27, OAS1, SIGLEC1, HERC6, SLC1A2, RETN, MMP8 | 0,883 | 0,875 | 0,892 | 0,923 | 0,868 | 0,977 | 0,947 | 0,885 | 1 |
| IFI27, OAS1, IFI44L, SIGLEC1, IL1R2, RETN, MMP8 | 0,883 | 0,875 | 0,892 | 0,923 | 0,871 | 0,975 | 0,961 | 0,919 | 1 |
| IFI27, SIGLEC1, HERC6, SLC1A2, IL1R2, OLAH, MMP8 | 0,883 | 0,874 | 0,893 | 0,927 | 0,877 | 0,977 | 0,947 | 0,894 | 0,999 |
| IFI27, IFIT1, IFI44L, HERC6, IL1R2, RETN, MMP8 | 0,883 | 0,875 | 0,892 | 0,921 | 0,867 | 0,975 | 0,942 | 0,885 | 0,999 |
| IFI27, IFIT1, SIGLEC1, SLC1A2, IL1R2, OLAH, RETN | 0,883 | 0,874 | 0,892 | 0,926 | 0,874 | 0,977 | 0,950 | 0,894 | 1 |
| IFI27, OAS1, IFIT1, HERC6, SLC1A2, IL1R2, OLAH | 0,883 | 0,874 | 0,893 | 0,927 | 0,879 | 0,976 | 0,934 | 0,868 | 0,999 |
| RSAD2, IFI27, OAS1, HERC6, SLC1A2, IL1R2, FAM20A | 0,883 | 0,874 | 0,893 | 0,931 | 0,886 | 0,976 | 0,921 | 0,832 | 1 |
| RSAD2, IFI27, IFI44L, SIGLEC1, ISG15, OLAH, MMP8 | 0,883 | 0,875 | 0,892 | 0,919 | 0,862 | 0,975 | 0,942 | 0,887 | 0,997 |
| RSAD2, IFI27, IFI44L, SIGLEC1, IL1R2, OLAH, RETN | 0,883 | 0,875 | 0,892 | 0,920 | 0,865 | 0,975 | 0,953 | 0,901 | 1 |
| IFI27, IFIT1, ISG15, SLC1A2, IL1R2, OLAH, MMP8 | 0,883 | 0,875 | 0,892 | 0,927 | 0,878 | 0,977 | 0,932 | 0,868 | 0,995 |
| IFI27, OAS1, IFI44L, SIGLEC1, ISG15, SLC1A2, OLAH | 0,883 | 0,875 | 0,892 | 0,929 | 0,879 | 0,979 | 0,945 | 0,887 | 1 |
| IFI27, OAS1, IFIT1, SIGLEC1, SLC1A2, OLAH, RETN | 0,883 | 0,874 | 0,892 | 0,926 | 0,873 | 0,978 | 0,949 | 0,889 | 1 |
| IFI27, IFIT1, IFI44L, SIGLEC1, SLC1A2, OLAH, RETN | 0,883 | 0,875 | 0,892 | 0,926 | 0,874 | 0,978 | 0,949 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, HERC6, RETN, MMP8 | 0,883 | 0,874 | 0,893 | 0,919 | 0,860 | 0,979 | 0,936 | 0,868 | 1 |
| IFI27, IFI44L, HERC6, SLC1A2, IL1R2, FAM20A, MMP8 | 0,883 | 0,874 | 0,893 | 0,930 | 0,884 | 0,976 | 0,927 | 0,840 | 1 |
| IFI27, IFIT1, IFI44L, SIGLEC1, HERC6, RETN, MMP8 | 0,883 | 0,874 | 0,892 | 0,912 | 0,850 | 0,973 | 0,940 | 0,885 | 0,995 |
| RSAD2, IFI27, IFIT1, HERC6, FAM20A, RETN, MMP8 | 0,883 | 0,874 | 0,893 | 0,923 | 0,866 | 0,981 | 0,934 | 0,854 | 1 |
| IFI27, OAS1, IFIT1, SLC1A2, IL1R2, FAM20A, MMP8 | 0,883 | 0,874 | 0,892 | 0,926 | 0,878 | 0,975 | 0,927 | 0,843 | 1 |
| RSAD2, IFI27, IFIT1, SIGLEC1, HERC6, SLC1A2, OLAH | 0,883 | 0,874 | 0,893 | 0,927 | 0,877 | 0,977 | 0,939 | 0,882 | 0,996 |
| RSAD2, IFI27, SIGLEC1, SLC1A2, FAM20A, RETN, MMP8 | 0,883 | 0,873 | 0,893 | 0,926 | 0,870 | 0,982 | 0,949 | 0,877 | 1 |
| RSAD2, IFI27, IFIT1, SIGLEC1, ISG15, SLC1A2, OLAH | 0,883 | 0,875 | 0,892 | 0,929 | 0,879 | 0,979 | 0,938 | 0,878 | 0,998 |
| IFI27, OAS1, IFI44L, SIGLEC1, ISG15, OLAH, RETN | 0,883 | 0,875 | 0,892 | 0,919 | 0,862 | 0,976 | 0,948 | 0,892 | 1 |
| IFI27, IFI44L, ISG15, HERC6, FAM20A, RETN, MMP8 | 0,883 | 0,873 | 0,894 | 0,921 | 0,861 | 0,980 | 0,941 | 0,864 | 1 |
| OAS1, IFIT1, SIGLEC1, SLC1A2, IL1R2, FAM20A, MMP8 | 0,883 | 0,874 | 0,892 | 0,927 | 0,880 | 0,974 | 0,960 | 0,898 | 1 |
| RSAD2, IFI27, SIGLEC1, SLC1A2, OLAH, RETN, MMP8 | 0,883 | 0,874 | 0,893 | 0,927 | 0,874 | 0,980 | 0,957 | 0,903 | 1 |
| RSAD2, IFI27, SIGLEC1, ISG15, OLAH, RETN, MMP8 | 0,883 | 0,874 | 0,892 | 0,920 | 0,861 | 0,979 | 0,957 | 0,903 | 1 |
| RSAD2, IFI27, IFIT1, HERC6, IL1R2, RETN, MMP8 | 0,883 | 0,874 | 0,892 | 0,920 | 0,866 | 0,974 | 0,943 | 0,889 | 0,998 |
| RSAD2, IFI27, HERC6, SLC1A2, IL1R2, OLAH, RETN | 0,883 | 0,874 | 0,893 | 0,927 | 0,876 | 0,977 | 0,935 | 0,870 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, HERC6, RETN | 0,883 | 0,874 | 0,892 | 0,916 | 0,854 | 0,977 | 0,934 | 0,861 | 1 |
| RSAD2, IFI27, OAS1, IFIT1, SIGLEC1, SLC1A2, OLAH | 0,883 | 0,874 | 0,892 | 0,928 | 0,876 | 0,979 | 0,947 | 0,891 | 1 |
| RSAD2, IFI27, OAS1, IFIT1, SIGLEC1, OLAH, RETN | 0,883 | 0,874 | 0,892 | 0,916 | 0,859 | 0,974 | 0,950 | 0,892 | 1 |
| RSAD2, SIGLEC1, SLC1A2, IL1R2, OLAH, FAM20A, MMP8 | 0,883 | 0,874 | 0,892 | 0,924 | 0,875 | 0,973 | 0,960 | 0,900 | 1 |
| IFI27, IFIT1, IFI44L, SIGLEC1, SLC1A2, OLAH, MMP8 | 0,883 | 0,874 | 0,892 | 0,927 | 0,875 | 0,978 | 0,948 | 0,894 | 1 |
| RSAD2, SIGLEC1, ISG15, IL1R2, OLAH, FAM20A, MMP8 | 0,883 | 0,874 | 0,892 | 0,918 | 0,861 | 0,975 | 0,964 | 0,909 | 1 |
| RSAD2, IFI27, IFIT1, IFI44L, ISG15, HERC6, OLAH | 0,883 | 0,874 | 0,892 | 0,924 | 0,871 | 0,976 | 0,934 | 0,875 | 0,993 |
| IFI27, OAS1, IFIT1, SIGLEC1, ISG15, FAM20A, MMP8 | 0,883 | 0,873 | 0,893 | 0,916 | 0,853 | 0,980 | 0,942 | 0,871 | 1 |
| RSAD2, OAS1, IFI44L, SIGLEC1, IL1R2, FAM20A, MMP8 | 0,883 | 0,874 | 0,892 | 0,918 | 0,861 | 0,975 | 0,961 | 0,903 | 1 |
| RSAD2, SIGLEC1, HERC6, IL1R2, FAM20A, RETN, MMP8 | 0,883 | 0,874 | 0,892 | 0,921 | 0,865 | 0,977 | 0,959 | 0,898 | 1 |
| IFI27, IFI44L, ISG15, HERC6, IL1R2, RETN, MMP8 | 0,883 | 0,875 | 0,892 | 0,923 | 0,870 | 0,975 | 0,938 | 0,878 | 0,998 |
| RSAD2, IFI27, IFIT1, SIGLEC1, SLC1A2, OLAH, MMP8 | 0,883 | 0,874 | 0,892 | 0,927 | 0,876 | 0,978 | 0,941 | 0,883 | 0,999 |
| RSAD2, IFI27, OAS1, IFIT1, HERC6, SLC1A2, OLAH | 0,883 | 0,874 | 0,892 | 0,928 | 0,878 | 0,978 | 0,937 | 0,873 | 1 |
| IFI27, ISG15, HERC6, SLC1A2, OLAH, RETN, MMP8 | 0,883 | 0,874 | 0,892 | 0,927 | 0,876 | 0,979 | 0,941 | 0,881 | 1 |
| IFI27, IFIT1, SIGLEC1, ISG15, HERC6, SLC1A2, FAM20A | 0,883 | 0,874 | 0,892 | 0,927 | 0,876 | 0,977 | 0,943 | 0,867 | 1 |
| IFI27, OAS1, IFI44L, SIGLEC1, HERC6, SLC1A2, RETN | 0,883 | 0,875 | 0,892 | 0,924 | 0,870 | 0,977 | 0,941 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, SIGLEC1, ISG15, HERC6, IL1R2, FAM20A, RETN | 0,883 | 0,874 | 0,892 | 0,918 | 0,860 | 0,975 | 0,953 | 0,897 | 1 |
| IFI27, IFIT1, SIGLEC1, HERC6, SLC1A2, IL1R2, OLAH | 0,883 | 0,874 | 0,892 | 0,928 | 0,878 | 0,977 | 0,943 | 0,889 | 0,998 |
| OAS1, IFIT1, SIGLEC1, HERC6, IL1R2, FAM20A, RETN | 0,883 | 0,874 | 0,892 | 0,919 | 0,861 | 0,976 | 0,953 | 0,897 | 1 |
| IFI27, OAS1, SIGLEC1, ISG15, IL1R2, OLAH, RETN | 0,883 | 0,874 | 0,892 | 0,919 | 0,864 | 0,975 | 0,950 | 0,895 | 1 |
| IFI27, IFIT1, IFI44L, ISG15, IL1R2, OLAH, MMP8 | 0,883 | 0,874 | 0,892 | 0,920 | 0,867 | 0,972 | 0,939 | 0,883 | 0,995 |
| RSAD2, IFI27, IFI44L, SIGLEC1, HERC6, SLC1A2, OLAH | 0,883 | 0,874 | 0,892 | 0,926 | 0,875 | 0,977 | 0,942 | 0,886 | 0,999 |
| RSAD2, IFI27, IFIT1, IL1R2, OLAH, RETN, MMP8 | 0,883 | 0,874 | 0,892 | 0,921 | 0,866 | 0,977 | 0,952 | 0,900 | 1 |
| RSAD2, IFI27, IFI44L, SLC1A2, OLAH, FAM20A, RETN | 0,883 | 0,874 | 0,892 | 0,925 | 0,868 | 0,982 | 0,924 | 0,845 | 1 |
| IFI27, OAS1, IFI44L, SIGLEC1, HERC6, FAM20A, MMP8 | 0,883 | 0,874 | 0,892 | 0,914 | 0,854 | 0,973 | 0,943 | 0,868 | 1 |
| OAS1, SIGLEC1, ISG15, IL1R2, OLAH, FAM20A, MMP8 | 0,883 | 0,874 | 0,892 | 0,916 | 0,858 | 0,974 | 0,964 | 0,909 | 1 |
| RSAD2, IFI27, SLC1A2, IL1R2, FAM20A, RETN, MMP8 | 0,883 | 0,874 | 0,892 | 0,929 | 0,881 | 0,978 | 0,928 | 0,843 | 1 |
| IFI27, OAS1, IFIT1, ISG15, SLC1A2, OLAH, MMP8 | 0,883 | 0,874 | 0,892 | 0,926 | 0,874 | 0,978 | 0,930 | 0,863 | 0,998 |
| IFI27, IFIT1, IFI44L, HERC6, SLC1A2, IL1R2, FAM20A | 0,883 | 0,874 | 0,892 | 0,929 | 0,881 | 0,977 | 0,927 | 0,842 | 1 |
| IFI27, IFI44L, HERC6, SLC1A2, OLAH, RETN, MMP8 | 0,883 | 0,873 | 0,892 | 0,924 | 0,870 | 0,978 | 0,940 | 0,878 | 1 |
| RSAD2, IFI27, IFI44L, SIGLEC1, ISG15, SLC1A2, OLAH | 0,883 | 0,875 | 0,891 | 0,931 | 0,881 | 0,981 | 0,943 | 0,886 | 1 |
| RSAD2, IFI27, IFIT1, SLC1A2, IL1R2, OLAH, MMP8 | 0,883 | 0,874 | 0,892 | 0,927 | 0,877 | 0,976 | 0,933 | 0,869 | 0,996 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, HERC6, SLC1A2, OLAH, MMP8 | 0,883 | 0,874 | 0,892 | 0,929 | 0,879 | 0,979 | 0,945 | 0,890 | 0,999 |
| IFI27, OAS1, IFI44L, HERC6, SLC1A2, OLAH, RETN | 0,883 | 0,874 | 0,892 | 0,928 | 0,878 | 0,978 | 0,930 | 0,861 | 1,000 |
| IFIT1, SIGLEC1, HERC6, IL1R2, OLAH, FAM20A, RETN | 0,883 | 0,874 | 0,892 | 0,919 | 0,861 | 0,976 | 0,952 | 0,895 | 1 |
| RSAD2, IFI27, IFI44L, SIGLEC1, HERC6, FAM20A, MMP8 | 0,883 | 0,874 | 0,892 | 0,918 | 0,859 | 0,977 | 0,941 | 0,869 | 1 |
| IFI27, IFI44L, SLC1A2, IL1R2, OLAH, RETN, MMP8 | 0,883 | 0,874 | 0,892 | 0,926 | 0,874 | 0,978 | 0,941 | 0,878 | 1 |
| RSAD2, OAS1, SIGLEC1, ISG15, IL1R2, FAM20A, MMP8 | 0,883 | 0,874 | 0,892 | 0,918 | 0,860 | 0,975 | 0,962 | 0,903 | 1 |
| RSAD2, OAS1, SIGLEC1, IL1R2, FAM20A, RETN, MMP8 | 0,883 | 0,874 | 0,892 | 0,920 | 0,863 | 0,977 | 0,962 | 0,907 | 1 |
| IFI27, OAS1, ISG15, HERC6, SLC1A2, IL1R2, OLAH | 0,883 | 0,874 | 0,892 | 0,927 | 0,878 | 0,977 | 0,928 | 0,862 | 0,995 |
| RSAD2, IFI27, ISG15, HERC6, SLC1A2, IL1R2, FAM20A | 0,883 | 0,874 | 0,892 | 0,930 | 0,883 | 0,978 | 0,924 | 0,836 | 1 |
| IFIT1, IFI44L, SIGLEC1, HERC6, SLC1A2, OLAH, FAM20A | 0,883 | 0,873 | 0,893 | 0,924 | 0,873 | 0,976 | 0,930 | 0,858 | 1 |
| RSAD2, IFI27, OAS1, IFI44L, SIGLEC1, OLAH, RETN | 0,883 | 0,874 | 0,891 | 0,916 | 0,856 | 0,975 | 0,954 | 0,897 | 1 |
| RSAD2, IFI27, OAS1, SIGLEC1, ISG15, OLAH, MMP8 | 0,883 | 0,874 | 0,892 | 0,919 | 0,863 | 0,974 | 0,941 | 0,884 | 0,998 |
| RSAD2, IFI27, OAS1, SIGLEC1, ISG15, HERC6, IL1R2 | 0,883 | 0,874 | 0,891 | 0,922 | 0,875 | 0,969 | 0,953 | 0,909 | 0,998 |
| IFI44L, SIGLEC1, ISG15, IL1R2, FAM20A, RETN, MMP8 | 0,883 | 0,874 | 0,892 | 0,916 | 0,857 | 0,975 | 0,963 | 0,908 | 1 |
| RSAD2, IFI27, SIGLEC1, SLC1A2, IL1R2, OLAH, RETN | 0,883 | 0,874 | 0,892 | 0,927 | 0,875 | 0,978 | 0,947 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, ISG15, SLC1A2, IL1R2, FAM20A, MMP8 | 0,883 | 0,874 | 0,891 | 0,925 | 0,877 | 0,973 | 0,959 | 0,893 | 1 |
| RSAD2, IFI27, IFIT1, IFI44L, SIGLEC1, OLAH, RETN | 0,883 | 0,874 | 0,891 | 0,916 | 0,857 | 0,976 | 0,951 | 0,896 | 1 |
| IFI27, SIGLEC1, ISG15, HERC6, SLC1A2, OLAH, RETN | 0,883 | 0,873 | 0,892 | 0,928 | 0,877 | 0,979 | 0,939 | 0,877 | 1 |
| IFI27, OAS1, IFI44L, SIGLEC1, SLC1A2, OLAH, MMP8 | 0,883 | 0,874 | 0,891 | 0,927 | 0,876 | 0,978 | 0,948 | 0,893 | 1 |
| IFI27, OAS1, SIGLEC1, ISG15, SLC1A2, FAM20A, RETN | 0,883 | 0,873 | 0,892 | 0,923 | 0,866 | 0,979 | 0,940 | 0,863 | 1 |
| RSAD2, IFI27, OAS1, SIGLEC1, IL1R2, RETN, MMP8 | 0,883 | 0,873 | 0,892 | 0,922 | 0,868 | 0,976 | 0,965 | 0,920 | 1 |
| SIGLEC1, ISG15, SLC1A2, IL1R2, OLAH, FAM20A, MMP8 | 0,883 | 0,874 | 0,892 | 0,924 | 0,875 | 0,973 | 0,958 | 0,892 | 1 |
| IFIT1, SIGLEC1, ISG15, IL1R2, FAM20A, RETN, MMP8 | 0,883 | 0,874 | 0,892 | 0,922 | 0,866 | 0,979 | 0,967 | 0,916 | 1 |
| RSAD2, IFI27, IFIT1, HERC6, SLC1A2, IL1R2, FAM20A | 0,883 | 0,873 | 0,892 | 0,931 | 0,883 | 0,978 | 0,927 | 0,842 | 1 |
| RSAD2,IFI27,OAS1,IFIT1, SIGLEC1, OLAH, MMP8 | 0,883 | 0,874 | 0,891 | 0,919 | 0,863 | 0,974 | 0,942 | 0,887 | 0,997 |
| RSAD2, IFI27, ISG15, HERC6, SLC1A2, OLAH, RETN | 0,883 | 0,874 | 0,892 | 0,927 | 0,876 | 0,978 | 0,933 | 0,866 | 0,999 |
| RSAD2, IFI27, SIGLEC1, ISG15, SLC1A2, OLAH, RETN | 0,883 | 0,874 | 0,891 | 0,929 | 0,878 | 0,980 | 0,941 | 0,881 | 1 |
| RSAD2, IFI27, IFIT1, SIGLEC1, IL1R2, OLAH, RETN | 0,883 | 0,874 | 0,891 | 0,919 | 0,863 | 0,975 | 0,948 | 0,892 | 1 |
| RSAD2, IFI27, ISG15, HERC6, SLC1A2, IL1R2, OLAH | 0,883 | 0,874 | 0,891 | 0,929 | 0,879 | 0,978 | 0,927 | 0,860 | 0,995 |
| IFI27, OAS1, IFIT1, IFI44L, SIGLEC1, SLC1A2, OLAH | 0,883 | 0,874 | 0,891 | 0,927 | 0,876 | 0,978 | 0,948 | 0,892 | 1 |
| RSAD2, IFI27, IFI44L, SIGLEC1, ISG15, OLAH, RETN | 0,883 | 0,874 | 0,891 | 0,920 | 0,863 | 0,978 | 0,948 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, ISG15, OLAH, RETN, MMP8 | 0,883 | 0,873 | 0,892 | 0,919 | 0,859 | 0,978 | 0,949 | 0,890 | 1 |
| IFI27, OAS1, IFIT1, IFI44L, SIGLEC1, IL1R2, MMP8 | 0,883 | 0,875 | 0,890 | 0,923 | 0,875 | 0,970 | 0,947 | 0,898 | 0,995 |
| RSAD2, IFI27, IFIT1, SIGLEC1, SLC1A2, IL1R2, OLAH | 0,883 | 0,874 | 0,891 | 0,927 | 0,877 | 0,978 | 0,945 | 0,889 | 1,000 |
| IFI27, IFIT1, IFI44L, SIGLEC1, HERC6, SLC1A2, OLAH | 0,883 | 0,873 | 0,892 | 0,927 | 0,877 | 0,978 | 0,942 | 0,887 | 0,998 |
| RSAD2, IFIT1, SIGLEC1, IL1R2, FAM20A, RETN, MMP8 | 0,883 | 0,874 | 0,891 | 0,922 | 0,866 | 0,979 | 0,968 | 0,918 | 1 |
| RSAD2, IFI27, OAS1, ISG15, HERC6, SLC1A2, OLAH | 0,883 | 0,873 | 0,892 | 0,929 | 0,879 | 0,979 | 0,932 | 0,864 | 0,999 |
| IFI27, IFI44L, ISG15, SLC1A2, OLAH, RETN, MMP8 | 0,883 | 0,873 | 0,892 | 0,926 | 0,873 | 0,979 | 0,939 | 0,875 | 1 |
| IFI27, OAS1, IFI44L, SIGLEC1, SLC1A2, OLAH, RETN | 0,883 | 0,874 | 0,891 | 0,926 | 0,874 | 0,978 | 0,947 | 0,884 | 1 |
| OAS1, IFI44L, SIGLEC1, IL1R2, OLAH, FAM20A, MMP8 | 0,883 | 0,873 | 0,892 | 0,918 | 0,861 | 0,975 | 0,963 | 0,908 | 1 |
| IFI27, IFIT1, ISG15, HERC6, SLC1A2, OLAH, MMP8 | 0,883 | 0,873 | 0,892 | 0,928 | 0,877 | 0,978 | 0,928 | 0,861 | 0,995 |
| SIGLEC1, HERC6, SLC1A2, IL1R2, OLAH, FAM20A, MMP8 | 0,883 | 0,873 | 0,892 | 0,922 | 0,872 | 0,972 | 0,960 | 0,898 | 1 |
| RSAD2, IFI27, OAS1, SIGLEC1, SLC1A2, OLAH, RETN | 0,883 | 0,874 | 0,891 | 0,925 | 0,872 | 0,978 | 0,949 | 0,887 | 1 |
| RSAD2, IFI27, IFIT1, IFI44L, HERC6, IL1R2, RETN | 0,883 | 0,874 | 0,891 | 0,920 | 0,867 | 0,973 | 0,936 | 0,876 | 0,995 |
| IFI27, OAS1, IFIT1, IFI44L, IL1R2, OLAH, MMP8 | 0,883 | 0,874 | 0,891 | 0,921 | 0,869 | 0,973 | 0,942 | 0,889 | 0,995 |
| IFI27, ISG15, HERC6, SLC1A2, IL1R2, OLAH, MMP8 | 0,883 | 0,874 | 0,891 | 0,928 | 0,879 | 0,977 | 0,927 | 0,860 | 0,994 |
| IFI27, IFI44L, ISG15, HERC6, SLC1A2, IL1R2, FAM20A | 0,882 | 0,873 | 0,892 | 0,929 | 0,882 | 0,976 | 0,928 | 0,843 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, OLAH, MMP8 | 0,882 | 0,874 | 0,891 | 0,919 | 0,862 | 0,975 | 0,949 | 0,898 | 1,000 |
| RSAD2, IFI27, ISG15, SLC1A2, IL1R2, OLAH, MMP8 | 0,882 | 0,874 | 0,891 | 0,929 | 0,879 | 0,978 | 0,928 | 0,861 | 0,996 |
| IFI27, OAS1, IFI44L, SLC1A2, IL1R2, FAM20A, MMP8 | 0,882 | 0,873 | 0,892 | 0,929 | 0,882 | 0,976 | 0,926 | 0,839 | 1 |
| IFI27, IFIT1, ISG15, SLC1A2, IL1R2, FAM20A, MMP8 | 0,882 | 0,873 | 0,891 | 0,928 | 0,880 | 0,976 | 0,927 | 0,842 | 1 |
| IFI27, OAS1, IFIT1, SLC1A2, IL1R2, OLAH, MMP8 | 0,882 | 0,874 | 0,891 | 0,927 | 0,878 | 0,976 | 0,936 | 0,872 | 1 |
| IFIT1, SIGLEC1, ISG15, HERC6, OLAH, FAM20A, RETN | 0,882 | 0,873 | 0,892 | 0,916 | 0,855 | 0,976 | 0,949 | 0,892 | 1 |
| RSAD2, IFI27, IFI44L, HERC6, IL1R2, RETN, MMP8 | 0,882 | 0,874 | 0,891 | 0,920 | 0,867 | 0,974 | 0,933 | 0,869 | 0,996 |
| IFI27, SIGLEC1, ISG15, HERC6, IL1R2, RETN, MMP8 | 0,882 | 0,874 | 0,891 | 0,921 | 0,866 | 0,976 | 0,963 | 0,923 | 1 |
| RSAD2, IFIT1, SIGLEC1, SLC1A2, IL1R2, FAM20A, MMP8 | 0,882 | 0,873 | 0,891 | 0,928 | 0,882 | 0,974 | 0,960 | 0,898 | 1 |
| RSAD2, IFI27, IFIT1, SIGLEC1, ISG15, IL1R2, OLAH | 0,882 | 0,874 | 0,891 | 0,921 | 0,868 | 0,973 | 0,945 | 0,891 | 0,998 |
| RSAD2, IFI27, IFIT1, IFI44L, IL1R2, OLAH, MMP8 | 0,882 | 0,874 | 0,891 | 0,922 | 0,870 | 0,973 | 0,942 | 0,889 | 0,995 |
| RSAD2, IFI27, OAS1, IFI44L, SIGLEC1, IL1R2, RETN | 0,882 | 0,874 | 0,891 | 0,916 | 0,861 | 0,972 | 0,958 | 0,908 | 1 |
| IFI27, IFIT1, IFI44L, SIGLEC1, ISG15, SLC1A2, FAM20A | 0,882 | 0,873 | 0,891 | 0,922 | 0,866 | 0,978 | 0,940 | 0,864 | 1 |
| RSAD2, IFI27, IFI44L, SIGLEC1, IL1R2, RETN, MMP8 | 0,882 | 0,874 | 0,891 | 0,922 | 0,869 | 0,976 | 0,960 | 0,918 | 1 |
| RSAD2, IFI27, ISG15, HERC6, SLC1A2, OLAH, MMP8 | 0,882 | 0,873 | 0,891 | 0,928 | 0,878 | 0,978 | 0,925 | 0,857 | 0,993 |

(suite)

| Modèle | AUC modèle | IC 95% | | AUC Train | IC 95% Train | | AUC Test | IC 95% Test | |
|---|---|---|---|---|---|---|---|---|---|
| IFI27, SIGLEC1, SLC1A2, IL1R2, OLAH, RETN, MMP8 | 0,882 | 0,873 | 0,892 | 0,926 | 0,874 | 0,978 | 0,958 | 0,909 | 1 |
| RSAD2, IFI27, IFIT1, HERC6, SLC1A2, OLAH, RETN | 0,882 | 0,873 | 0,892 | 0,925 | 0,875 | 0,976 | 0,935 | 0,870 | 1 |
| IFI27, IFI44L, SIGLEC1, HERC6, SLC1A2, IL1R2, OLAH | 0,882 | 0,873 | 0,892 | 0,927 | 0,877 | 0,977 | 0,946 | 0,893 | 0,998 |
| RSAD2, IFI27, OAS1, IFI44L, SIGLEC1, SLC1A2, OLAH | 0,882 | 0,874 | 0,891 | 0,926 | 0,874 | 0,978 | 0,953 | 0,900 | 1 |
| IFI27, IFIT1, ISG15, HERC6, SLC1A2, IL1R2, OLAH | 0,882 | 0,873 | 0,891 | 0,927 | 0,877 | 0,976 | 0,927 | 0,860 | 0,994 |
| SIGLEC1, ISG15, IL1R2, OLAH, FAM20A, RETN, MMP8 | 0,882 | 0,873 | 0,892 | 0,915 | 0,855 | 0,975 | 0,965 | 0,911 | 1 |
| IFI27, IFI44L, ISG15, SLC1A2, IL1R2, FAM20A, RETN | 0,882 | 0,873 | 0,891 | 0,927 | 0,877 | 0,977 | 0,924 | 0,839 | 1 |
| RSAD2, IFI27, IFIT1, IFI44L, SIGLEC1, FAM20A, MMP8 | 0,882 | 0,873 | 0,892 | 0,915 | 0,849 | 0,980 | 0,946 | 0,877 | 1 |
| OAS1, SIGLEC1, SLC1A2, IL1R2, OLAH, FAM20A, MMP8 | 0,882 | 0,873 | 0,891 | 0,925 | 0,876 | 0,974 | 0,959 | 0,898 | 1 |
| IFI27, OAS1, IFI44L, HERC6, SLC1A2, OLAH, MMP8 | 0,882 | 0,873 | 0,892 | 0,928 | 0,878 | 0,977 | 0,929 | 0,861 | 0,997 |
| IFI27, OAS1, IFIT1, ISG15, IL1R2, OLAH, MMP8 | 0,882 | 0,874 | 0,891 | 0,921 | 0,870 | 0,972 | 0,946 | 0,894 | 0,998 |
| RSAD2, IFI27, IFIT1, ISG15, SLC1A2, OLAH, MMP8 | 0,882 | 0,873 | 0,891 | 0,926 | 0,874 | 0,978 | 0,929 | 0,863 | 0,996 |
| IFI27, IFIT1, IFI44L, ISG15, SLC1A2, OLAH, MMP8 | 0,882 | 0,873 | 0,891 | 0,927 | 0,875 | 0,979 | 0,927 | 0,859 | 0,996 |
| IFI27, OAS1, IFI44L, ISG15, OLAH, RETN, MMP8 | 0,882 | 0,873 | 0,891 | 0,919 | 0,860 | 0,978 | 0,948 | 0,891 | 1 |
| RSAD2, IFI27, OAS1, IFIT1, IFI44L, SIGLEC1, OLAH | 0,882 | 0,874 | 0,891 | 0,919 | 0,862 | 0,975 | 0,951 | 0,901 | 1 |
| IFIT1, SIGLEC1, ISG15, SLC1A2, OLAH, FAM20A, MMP8 | 0,882 | 0,873 | 0,891 | 0,921 | 0,868 | 0,974 | 0,947 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, OLAH, RETN, MMP8 | 0,882 | 0,873 | 0,891 | 0,918 | 0,858 | 0,978 | 0,950 | 0,894 | 1 |
| RSAD2, IFI27, IFIT1, SIGLEC1, HERC6, SLC1A2, FAM20A | 0,882 | 0,873 | 0,891 | 0,927 | 0,877 | 0,978 | 0,938 | 0,860 | 1 |
| RSAD2, IFI27, IFIT1, SIGLEC1, SLC1A2, FAM20A, RETN | 0,882 | 0,873 | 0,891 | 0,924 | 0,869 | 0,980 | 0,941 | 0,865 | 1 |
| IFI27, IFIT1, SIGLEC1, ISG15, HERC6, RETN, MMP8 | 0,882 | 0,873 | 0,891 | 0,910 | 0,848 | 0,972 | 0,946 | 0,892 | 0,999 |
| IFI27, IFIT1, HERC6, SLC1A2, IL1R2, OLAH, MMP8 | 0,882 | 0,873 | 0,891 | 0,927 | 0,877 | 0,976 | 0,934 | 0,871 | 0,997 |
| RSAD2, IFI27, IFI44L, HERC6, SLC1A2, OLAH, MMP8 | 0,882 | 0,873 | 0,891 | 0,927 | 0,877 | 0,978 | 0,928 | 0,862 | 0,995 |
| OAS1, IFIT1, SIGLEC1, ISG15, HERC6, FAM20A, RETN | 0,882 | 0,873 | 0,891 | 0,916 | 0,858 | 0,975 | 0,941 | 0,875 | 1 |
| SIGLEC1, ISG15, HERC6, IL1R2, FAM20A, RETN, MMP8 | 0,882 | 0,873 | 0,891 | 0,915 | 0,855 | 0,975 | 0,961 | 0,900 | 1 |
| RSAD2, IFI27, ISG15, IL1R2, OLAH, RETN, MMP8 | 0,882 | 0,873 | 0,891 | 0,921 | 0,865 | 0,978 | 0,953 | 0,900 | 1 |
| RSAD2, IFI27, IFIT1, ISG15, HERC6, SLC1A2, OLAH | 0,882 | 0,873 | 0,891 | 0,927 | 0,877 | 0,978 | 0,927 | 0,860 | 0,994 |
| RSAD2, IFI27, ISG15, HERC6, IL1R2, RETN, MMP8 | 0,882 | 0,873 | 0,891 | 0,922 | 0,868 | 0,975 | 0,941 | 0,883 | 1,000 |
| RSAD2, IFI27, IFI44L, SIGLEC1, SLC1A2, OLAH, MMP8 | 0,882 | 0,873 | 0,891 | 0,926 | 0,875 | 0,977 | 0,948 | 0,894 | 1 |
| IFI27, IFI44L, ISG15, HERC6, SLC1A2, OLAH, RETN | 0,882 | 0,873 | 0,891 | 0,926 | 0,875 | 0,977 | 0,936 | 0,871 | 1 |
| IFI27, IFI44L, ISG15, HERC6, SLC1A2, OLAH, MMP8 | 0,882 | 0,873 | 0,891 | 0,928 | 0,878 | 0,978 | 0,926 | 0,859 | 0,993 |
| RSAD2, IFI27, OAS1, IFI44L, OLAH, RETN, MMP8 | 0,882 | 0,873 | 0,891 | 0,919 | 0,859 | 0,978 | 0,951 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, ISG15, IL1R2, RETN, MMP8 | 0,882 | 0,873 | 0,891 | 0,923 | 0,871 | 0,976 | 0,963 | 0,918 | 1 |
| IFI27, OAS1, IFI44L, SIGLEC1, ISG15, IL1R2, RETN | 0,882 | 0,874 | 0,890 | 0,921 | 0,868 | 0,973 | 0,945 | 0,891 | 0,998 |
| RSAD2, OAS1, IFIT1, SIGLEC1, HERC6, OLAH, FAM20A | 0,882 | 0,873 | 0,891 | 0,912 | 0,851 | 0,973 | 0,949 | 0,893 | 1 |
| RSAD2, IFI27, IFI44L, HERC6, SLC1A2, OLAH, RETN | 0,882 | 0,873 | 0,891 | 0,926 | 0,875 | 0,977 | 0,935 | 0,870 | 0,999 |
| RSAD2, IFI27, IFIT1, HERC6, SLC1A2, OLAH, MMP8 | 0,882 | 0,873 | 0,891 | 0,927 | 0,876 | 0,978 | 0,930 | 0,865 | 0,995 |
| IFI27, IFIT1, SIGLEC1, ISG15, IL1R2, RETN, MMP8 | 0,882 | 0,873 | 0,891 | 0,921 | 0,867 | 0,975 | 0,964 | 0,925 | 1 |
| IFI44L, SIGLEC1, IL1R2, OLAH, FAM20A, RETN, MMP8 | 0,882 | 0,873 | 0,891 | 0,918 | 0,860 | 0,976 | 0,963 | 0,908 | 1 |
| RSAD2, IFIT1, IFI44L, SIGLEC1, HERC6, OLAH, FAM20A | 0,882 | 0,873 | 0,891 | 0,915 | 0,855 | 0,975 | 0,943 | 0,884 | 1 |
| IFI27, IFIT1, IFI44L, SIGLEC1, SLC1A2, IL1R2, OLAH | 0,882 | 0,873 | 0,891 | 0,927 | 0,877 | 0,978 | 0,945 | 0,889 | 1,000 |
| IFI27, IFIT1, SLC1A2, IL1R2, OLAH, RETN, MMP8 | 0,882 | 0,873 | 0,891 | 0,925 | 0,872 | 0,977 | 0,946 | 0,887 | 1 |
| RSAD2, IFI27, IFI44L, SIGLEC1, ISG15, HERC6, RETN | 0,882 | 0,873 | 0,891 | 0,917 | 0,859 | 0,975 | 0,943 | 0,889 | 0,998 |
| IFIT1, IFI44L, SIGLEC1, ISG15, OLAH, FAM20A, MMP8 | 0,882 | 0,872 | 0,892 | 0,909 | 0,844 | 0,974 | 0,954 | 0,904 | 1 |
| RSAD2, IFI27, IFIT1, SIGLEC1, HERC6, IL1R2, MMP8 | 0,882 | 0,874 | 0,890 | 0,922 | 0,875 | 0,969 | 0,947 | 0,898 | 0,995 |
| IFI27, ISG15, HERC6, SLC1A2, IL1R2, OLAH, RETN | 0,882 | 0,873 | 0,891 | 0,926 | 0,875 | 0,977 | 0,933 | 0,867 | 0,998 |
| IFI27, ISG15, HERC6, SLC1A2, IL1R2, FAM20A, RETN | 0,882 | 0,872 | 0,891 | 0,933 | 0,885 | 0,981 | 0,925 | 0,840 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, IL1R2, OLAH, MMP8 | 0,882 | 0,873 | 0,890 | 0,922 | 0,870 | 0,974 | 0,943 | 0,890 | 0,997 |
| RSAD2, IFI27, OAS1, ISG15, SLC1A2, OLAH, MMP8 | 0,882 | 0,873 | 0,891 | 0,926 | 0,873 | 0,979 | 0,929 | 0,861 | 0,998 |
| IFI27, IFIT1, ISG15, SLC1A2, OLAH, RETN, MMP8 | 0,882 | 0,872 | 0,891 | 0,927 | 0,873 | 0,980 | 0,940 | 0,878 | 1 |
| RSAD2, IFI27, IFI44L, SIGLEC1, SLC1A2, OLAH, RETN | 0,882 | 0,873 | 0,890 | 0,927 | 0,875 | 0,978 | 0,951 | 0,895 | 1 |
| RSAD2, IFI27, IFIT1, HERC6, SLC1A2, IL1R2, OLAH | 0,882 | 0,873 | 0,891 | 0,926 | 0,876 | 0,975 | 0,930 | 0,866 | 0,995 |
| IFI27, HERC6, SLC1A2, IL1R2, OLAH, RETN, MMP8 | 0,882 | 0,872 | 0,891 | 0,927 | 0,875 | 0,979 | 0,946 | 0,886 | 1 |
| IFI27, OAS1, IFIT1, SLC1A2, OLAH, RETN, MMP8 | 0,882 | 0,872 | 0,891 | 0,922 | 0,866 | 0,978 | 0,941 | 0,877 | 1 |
| RSAD2, IFI27, IFIT1, SIGLEC1, IL1R2, RETN, MMP8 | 0,882 | 0,873 | 0,890 | 0,922 | 0,868 | 0,975 | 0,962 | 0,921 | 1 |
| RSAD2, IFI27, IFIT1, SIGLEC1, ISG15, FAM20A, MMP8 | 0,882 | 0,872 | 0,891 | 0,916 | 0,852 | 0,981 | 0,940 | 0,868 | 1 |
| RSAD2, IFI27, IFI44L, IL1R2, OLAH, RETN, MMP8 | 0,882 | 0,873 | 0,891 | 0,923 | 0,868 | 0,979 | 0,951 | 0,897 | 1 |
| IFI27, IFIT1, IFI44L, ISG15, HERC6, IL1R2, RETN | 0,882 | 0,873 | 0,890 | 0,920 | 0,867 | 0,973 | 0,936 | 0,876 | 0,995 |
| RSAD2, IFI27, IFIT1, IFI44L, OLAH, RETN, MMP8 | 0,882 | 0,873 | 0,891 | 0,921 | 0,862 | 0,980 | 0,951 | 0,893 | 1 |
| IFI27, OAS1, IFIT1, ISG15, IL1R2, OLAH, RETN | 0,882 | 0,873 | 0,890 | 0,918 | 0,862 | 0,975 | 0,941 | 0,882 | 1 |
| IFI44L, SIGLEC1, HERC6, SLC1A2, IL1R2, FAM20A, MMP8 | 0,882 | 0,872 | 0,891 | 0,924 | 0,875 | 0,973 | 0,959 | 0,895 | 1 |
| OAS1, IFIT1, SIGLEC1, HERC6, OLAH, FAM20A, RETN | 0,882 | 0,873 | 0,891 | 0,915 | 0,855 | 0,975 | 0,946 | 0,886 | 1 |
| IFI27, OAS1, IFI44L, ISG15, IL1R2, OLAH, MMP8 | 0,882 | 0,873 | 0,890 | 0,923 | 0,872 | 0,974 | 0,934 | 0,874 | 0,993 |

(suite)

| Modèle | AUC modèle | IC 95% | | AUC Train | IC 95% Train | | AUC Test | IC 95% Test | |
|---|---|---|---|---|---|---|---|---|---|
| IFIT1, SIGLEC1, ISG15, SLC1A2, IL1R2, FAM20A, MMP8 | 0,882 | 0,873 | 0,891 | 0,927 | 0,881 | 0,973 | 0,959 | 0,895 | 1 |
| OAS1, IFIT1, SIGLEC1, ISG15, HERC6, OLAH, FAM20A | 0,882 | 0,872 | 0,891 | 0,913 | 0,853 | 0,973 | 0,949 | 0,893 | 1 |
| IFI27, IFIT1, ISG15, HERC6, SLC1A2, OLAH, RETN | 0,882 | 0,872 | 0,891 | 0,926 | 0,875 | 0,978 | 0,934 | 0,868 | 0,999 |
| RSAD2, IFI27, IFIT1, ISG15, HERC6, IL1R2, RETN | 0,882 | 0,873 | 0,890 | 0,920 | 0,868 | 0,973 | 0,943 | 0,889 | 0,998 |
| RSAD2, IFI27, IFIT1, IFI44L, SIGLEC1, IL1R2, RETN | 0,882 | 0,873 | 0,890 | 0,919 | 0,864 | 0,974 | 0,951 | 0,902 | 1 |
| RSAD2, IFI27, OAS1, IFI44L, IL1R2, OLAH, MMP8 | 0,882 | 0,873 | 0,890 | 0,922 | 0,869 | 0,974 | 0,945 | 0,892 | 0,998 |
| OAS1, IFI44L, SIGLEC1, ISG15, IL1R2, FAM20A, MMP8 | 0,882 | 0,872 | 0,891 | 0,917 | 0,859 | 0,975 | 0,961 | 0,902 | 1 |
| IFI27, IFIT1, HERC6, SLC1A2, IL1R2, OLAH, RETN | 0,882 | 0,872 | 0,891 | 0,925 | 0,874 | 0,977 | 0,936 | 0,871 | 1 |
| IFI27, OAS1, SLC1A2, IL1R2, OLAH, RETN, MMP8 | 0,882 | 0,872 | 0,891 | 0,925 | 0,873 | 0,977 | 0,942 | 0,880 | 1 |
| RSAD2, IFI27, OAS1, SLC1A2, IL1R2, OLAH, MMP8 | 0,882 | 0,873 | 0,890 | 0,926 | 0,876 | 0,976 | 0,930 | 0,862 | 0,999 |
| IFI27, ISG15, HERC6, SLC1A2, IL1R2, FAM20A, MMP8 | 0,882 | 0,872 | 0,891 | 0,930 | 0,884 | 0,977 | 0,925 | 0,839 | 1 |
| IFI27, IFIT1, IFI44L, HERC6, SLC1A2, OLAH, RETN | 0,882 | 0,872 | 0,891 | 0,924 | 0,871 | 0,976 | 0,935 | 0,869 | 1 |
| RSAD2,IFI27,OAS1,IFIT1, HERC6, IL1R2, MMP8 | 0,882 | 0,873 | 0,890 | 0,919 | 0,871 | 0,967 | 0,939 | 0,885 | 0,993 |
| OAS1, IFIT1, IFI44L, SIGLEC1, IL1R2, OLAH, FAM20A | 0,882 | 0,873 | 0,891 | 0,912 | 0,849 | 0,975 | 0,958 | 0,906 | 1 |
| RSAD2, IFI27, SIGLEC1, ISG15, IL1R2, OLAH, RETN | 0,882 | 0,873 | 0,890 | 0,919 | 0,863 | 0,975 | 0,948 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, SLC1A2, IL1R2, RETN | 0,882 | 0,874 | 0,890 | 0,924 | 0,876 | 0,973 | 0,943 | 0,887 | 1,000 |
| RSAD2, IFI27, IFI44L, SIGLEC1, SLC1A2, IL1R2, OLAH | 0,882 | 0,873 | 0,890 | 0,927 | 0,877 | 0,978 | 0,945 | 0,890 | 1,000 |
| IFI27, IFI44L, HERC6, SLC1A2, IL1R2, FAM20A, RETN | 0,882 | 0,872 | 0,891 | 0,931 | 0,883 | 0,980 | 0,927 | 0,844 | 1 |
| RSAD2, IFI27, IFIT1, SIGLEC1, SLC1A2, OLAH, RETN | 0,882 | 0,873 | 0,890 | 0,926 | 0,874 | 0,978 | 0,949 | 0,892 | 1 |
| RSAD2, IFI27, IFI44L, ISG15, HERC6, IL1R2, RETN | 0,882 | 0,873 | 0,890 | 0,921 | 0,868 | 0,974 | 0,936 | 0,876 | 0,995 |
| RSAD2,IFI27,OAS1,IFIT1, IFI44L, IL1R2, OLAH | 0,882 | 0,873 | 0,890 | 0,920 | 0,867 | 0,974 | 0,940 | 0,884 | 0,996 |
| OAS1, IFIT1, IFI44L, SIGLEC1, HERC6, FAM20A, RETN | 0,882 | 0,873 | 0,890 | 0,915 | 0,855 | 0,975 | 0,937 | 0,872 | 1 |
| IFI27, IFIT1, IFI44L, SLC1A2, IL1R2, FAM20A, RETN | 0,882 | 0,872 | 0,891 | 0,926 | 0,876 | 0,977 | 0,924 | 0,837 | 1 |
| IFI27, IFIT1, IFI44L, SIGLEC1, ISG15, IL1R2, MMP8 | 0,882 | 0,874 | 0,889 | 0,922 | 0,874 | 0,970 | 0,942 | 0,892 | 0,993 |
| RSAD2, IFI27, IFIT1, IFI44L, SIGLEC1, ISG15, OLAH | 0,882 | 0,873 | 0,890 | 0,919 | 0,863 | 0,976 | 0,941 | 0,885 | 0,997 |
| IFI27, IFI44L, ISG15, SLC1A2, IL1R2, OLAH, RETN | 0,882 | 0,873 | 0,890 | 0,929 | 0,878 | 0,979 | 0,932 | 0,863 | 1 |
| OAS1, IFIT1, SIGLEC1, SLC1A2, OLAH, FAM20A, MMP8 | 0,882 | 0,872 | 0,891 | 0,922 | 0,869 | 0,976 | 0,952 | 0,892 | 1 |
| IFI27, OAS1, IFIT1, IFI44L, HERC6, SLC1A2, OLAH | 0,882 | 0,872 | 0,891 | 0,929 | 0,879 | 0,978 | 0,934 | 0,868 | 0,999 |
| RSAD2,IFI27,OAS1,IFIT1, SIGLEC1, ISG15, OLAH | 0,882 | 0,873 | 0,890 | 0,919 | 0,863 | 0,975 | 0,941 | 0,885 | 0,998 |
| RSAD2, IFI27, IFI44L, SIGLEC1, ISG15, HERC6, IL1R2 | 0,881 | 0,874 | 0,889 | 0,920 | 0,870 | 0,970 | 0,940 | 0,888 | 0,992 |
| RSAD2, IFI27, OAS1, ISG15, IL1R2, OLAH, RETN | 0,881 | 0,873 | 0,890 | 0,919 | 0,863 | 0,976 | 0,942 | 0,883 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, ISG15, OLAH, RETN, MMP8 | 0,881 | 0,872 | 0,891 | 0,919 | 0,859 | 0,978 | 0,950 | 0,893 | 1 |
| RSAD2, IFI27, IFIT1, SIGLEC1, HERC6, RETN, MMP8 | 0,881 | 0,872 | 0,890 | 0,915 | 0,856 | 0,974 | 0,943 | 0,890 | 0,997 |
| RSAD2, IFI27, IFIT1, SIGLEC1, ISG15, OLAH, RETN | 0,881 | 0,873 | 0,890 | 0,918 | 0,861 | 0,976 | 0,947 | 0,890 | 1 |
| RSAD2, IFI27, IFI44L, ISG15, HERC6, SLC1A2, OLAH | 0,881 | 0,872 | 0,891 | 0,928 | 0,878 | 0,978 | 0,925 | 0,857 | 0,993 |
| RSAD2, IFI27, OAS1, IFIT1, SLC1A2, OLAH, MMP8 | 0,881 | 0,873 | 0,890 | 0,926 | 0,875 | 0,978 | 0,940 | 0,877 | 1 |
| RSAD2, IFI27, SLC1A2, IL1R2, OLAH, RETN, MMP8 | 0,881 | 0,872 | 0,891 | 0,924 | 0,872 | 0,977 | 0,941 | 0,878 | 1 |
| OAS1, IFIT1, SIGLEC1, ISG15, IL1R2, OLAH, FAM20A | 0,881 | 0,872 | 0,890 | 0,913 | 0,850 | 0,976 | 0,961 | 0,910 | 1 |
| IFI27, IFIT1, IFI44L, SIGLEC1, SLC1A2, FAM20A, MMP8 | 0,881 | 0,872 | 0,890 | 0,920 | 0,863 | 0,978 | 0,942 | 0,868 | 1 |
| RSAD2, IFI27, ISG15, SLC1A2, IL1R2, FAM20A, MMP8 | 0,881 | 0,873 | 0,890 | 0,929 | 0,883 | 0,976 | 0,927 | 0,840 | 1 |
| IFI27, OAS1, ISG15, SLC1A2, IL1R2, OLAH, MMP8 | 0,881 | 0,873 | 0,890 | 0,927 | 0,877 | 0,977 | 0,922 | 0,852 | 0,992 |
| IFI27, IFIT1, ISG15, SLC1A2, IL1R2, OLAH, RETN | 0,881 | 0,873 | 0,890 | 0,927 | 0,877 | 0,978 | 0,936 | 0,870 | 1 |
| IFI27, OAS1, IFIT1, IFI44L, IL1R2, OLAH, RETN | 0,881 | 0,873 | 0,890 | 0,916 | 0,860 | 0,973 | 0,942 | 0,883 | 1 |
| RSAD2, IFI27, OAS1, IFIT1, ISG15, IL1R2, OLAH | 0,881 | 0,873 | 0,890 | 0,919 | 0,865 | 0,972 | 0,941 | 0,885 | 0,998 |
| RSAD2, IFI27, IFIT1, SLC1A2, OLAH, RETN, MMP8 | 0,881 | 0,872 | 0,891 | 0,923 | 0,868 | 0,979 | 0,945 | 0,884 | 1 |
| OAS1, IFIT1, IFI44L, SIGLEC1, OLAH, FAM20A, MMP8 | 0,881 | 0,871 | 0,891 | 0,910 | 0,844 | 0,975 | 0,957 | 0,907 | 1 |
| RSAD2, IFI27, OAS1, ISG15, SLC1A2, IL1R2, OLAH | 0,881 | 0,872 | 0,890 | 0,930 | 0,880 | 0,979 | 0,932 | 0,864 | 0,999 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, HERC6, SLC1A2, FAM20A, RETN | 0,881 | 0,872 | 0,891 | 0,925 | 0,870 | 0,980 | 0,945 | 0,866 | 1 |
| SIGLEC1, HERC6, IL1R2, OLAH, FAM20A, RETN, MMP8 | 0,881 | 0,871 | 0,891 | 0,918 | 0,859 | 0,976 | 0,965 | 0,911 | 1 |
| RSAD2, IFI27, IFI44L, ISG15, IL1R2, OLAH, MMP8 | 0,881 | 0,873 | 0,890 | 0,922 | 0,870 | 0,974 | 0,940 | 0,884 | 0,996 |
| RSAD2, IFI27, OAS1, IFI44L, ISG15, IL1R2, OLAH | 0,881 | 0,873 | 0,890 | 0,922 | 0,869 | 0,975 | 0,942 | 0,887 | 0,997 |
| RSAD2, IFIT1, SIGLEC1, ISG15, HERC6, OLAH, FAM20A | 0,881 | 0,872 | 0,891 | 0,912 | 0,852 | 0,973 | 0,951 | 0,896 | 1 |
| IFI27, IFI44L, SIGLEC1, ISG15, SLC1A2, FAM20A, RETN | 0,881 | 0,872 | 0,891 | 0,923 | 0,867 | 0,980 | 0,941 | 0,864 | 1 |
| RSAD2, SIGLEC1, IL1R2, OLAH, FAM20A, RETN, MMP8 | 0,881 | 0,872 | 0,891 | 0,919 | 0,862 | 0,976 | 0,964 | 0,913 | 1 |
| IFI27, OAS1, IFIT1, SIGLEC1, ISG15, IL1R2, RETN | 0,881 | 0,873 | 0,890 | 0,921 | 0,868 | 0,974 | 0,953 | 0,902 | 1 |
| RSAD2, IFI27, IFIT1, SIGLEC1, ISG15, OLAH, MMP8 | 0,881 | 0,873 | 0,890 | 0,919 | 0,863 | 0,975 | 0,941 | 0,885 | 0,998 |
| RSAD2, IFI27, SIGLEC1, ISG15, HERC6, IL1R2, MMP8 | 0,881 | 0,873 | 0,889 | 0,921 | 0,874 | 0,968 | 0,948 | 0,900 | 0,996 |
| IFI27, OAS1, IFIT1, IFI44L, SIGLEC1, SLC1A2, FAM20A | 0,881 | 0,872 | 0,890 | 0,922 | 0,864 | 0,979 | 0,940 | 0,867 | 1 |
| IFI44L, SIGLEC1, SLC1A2, IL1R2, FAM20A, RETN, MMP8 | 0,881 | 0,872 | 0,890 | 0,924 | 0,874 | 0,973 | 0,960 | 0,896 | 1 |
| RSAD2, IFI27, OAS1, SIGLEC1, ISG15, OLAH, RETN | 0,881 | 0,872 | 0,890 | 0,916 | 0,858 | 0,975 | 0,945 | 0,882 | 1 |
| IFIT1, IFI44L, SIGLEC1, HERC6, SLC1A2, IL1R2, FAM20A | 0,881 | 0,872 | 0,890 | 0,926 | 0,878 | 0,974 | 0,940 | 0,868 | 1 |
| IFI27, OAS1, IFI44L, ISG15, HERC6, SLC1A2, OLAH | 0,881 | 0,871 | 0,891 | 0,929 | 0,880 | 0,979 | 0,924 | 0,854 | 0,994 |
| RSAD2, IFI27, OAS1, IFIT1, SIGLEC1, FAM20A, MMP8 | 0,881 | 0,871 | 0,891 | 0,914 | 0,849 | 0,979 | 0,946 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SLC1A2, IL1R2, OLAH | 0,881 | 0,872 | 0,890 | 0,925 | 0,874 | 0,976 | 0,936 | 0,869 | 1 |
| RSAD2, IFI27, OAS1, IFI44L, SIGLEC1, ISG15, FAM20A | 0,881 | 0,872 | 0,891 | 0,915 | 0,851 | 0,979 | 0,943 | 0,871 | 1 |
| RSAD2, IFI27, IFIT1, ISG15, SLC1A2, IL1R2, OLAH | 0,881 | 0,872 | 0,890 | 0,927 | 0,878 | 0,977 | 0,926 | 0,858 | 0,994 |
| IFI27, IFIT1, HERC6, SLC1A2, IL1R2, FAM20A, RETN | 0,881 | 0,872 | 0,891 | 0,933 | 0,886 | 0,981 | 0,926 | 0,842 | 1 |
| RSAD2, IFI27, OAS1, IFIT1, IL1R2, OLAH, RETN | 0,881 | 0,872 | 0,890 | 0,917 | 0,860 | 0,974 | 0,945 | 0,886 | 1 |
| RSAD2, IFI27, IFI44L, ISG15, SLC1A2, OLAH, MMP8 | 0,881 | 0,872 | 0,890 | 0,927 | 0,875 | 0,979 | 0,928 | 0,860 | 0,996 |
| IFI27, OAS1, IFIT1, IFI44L, SLC1A2, IL1R2, FAM20A | 0,881 | 0,872 | 0,890 | 0,926 | 0,876 | 0,975 | 0,926 | 0,842 | 1 |
| IFI27, OAS1, IFIT1, SLC1A2, IL1R2, OLAH, RETN | 0,881 | 0,872 | 0,890 | 0,926 | 0,874 | 0,977 | 0,938 | 0,871 | 1 |
| IFI27, OAS1, IFIT1, ISG15, SLC1A2, IL1R2, OLAH | 0,881 | 0,872 | 0,890 | 0,928 | 0,878 | 0,977 | 0,929 | 0,862 | 0,996 |
| RSAD2, IFI27, IFIT1, IFI44L, SIGLEC1, SLC1A2, OLAH | 0,881 | 0,873 | 0,890 | 0,926 | 0,874 | 0,977 | 0,945 | 0,889 | 1 |
| IFI27, IFIT1, SIGLEC1, ISG15, HERC6, IL1R2, MMP8 | 0,881 | 0,873 | 0,889 | 0,921 | 0,873 | 0,969 | 0,950 | 0,903 | 0,997 |
| IFI27, OAS1, IFIT1, IFI44L, ISG15, OLAH, MMP8 | 0,881 | 0,872 | 0,890 | 0,916 | 0,858 | 0,974 | 0,935 | 0,875 | 0,994 |
| RSAD2, IFIT1, SIGLEC1, HERC6, IL1R2, FAM20A, RETN | 0,881 | 0,872 | 0,890 | 0,918 | 0,860 | 0,976 | 0,954 | 0,898 | 1 |
| IFI27, IFI44L, SIGLEC1, ISG15, HERC6, FAM20A, MMP8 | 0,881 | 0,871 | 0,891 | 0,911 | 0,848 | 0,974 | 0,941 | 0,862 | 1 |
| IFI27, OAS1, SLC1A2, IL1R2, FAM20A, RETN, MMP8 | 0,881 | 0,872 | 0,890 | 0,929 | 0,879 | 0,978 | 0,928 | 0,843 | 1 |
| IFI27, IFIT1, IFI44L, ISG15, IL1R2, OLAH, RETN | 0,881 | 0,872 | 0,889 | 0,917 | 0,861 | 0,974 | 0,939 | 0,879 | 1,000 |
| RSAD2, IFI27, OAS1, SLC1A2, IL1R2, FAM20A, MMP8 | 0,881 | 0,872 | 0,890 | 0,928 | 0,880 | 0,975 | 0,927 | 0,840 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, HERC6, RETN, MMP8 | 0,881 | 0,872 | 0,890 | 0,915 | 0,854 | 0,976 | 0,935 | 0,866 | 1 |
| RSAD2, IFI27, IFIT1, ISG15, IL1R2, OLAH, MMP8 | 0,881 | 0,872 | 0,890 | 0,922 | 0,870 | 0,973 | 0,946 | 0,894 | 0,998 |
| IFIT1, SIGLEC1, ISG15, HERC6, SLC1A2, OLAH, FAM20A | 0,881 | 0,871 | 0,891 | 0,921 | 0,868 | 0,974 | 0,937 | 0,864 | 1 |
| RSAD2, IFI44L, SIGLEC1, ISG15, IL1R2, FAM20A, MMP8 | 0,881 | 0,872 | 0,890 | 0,916 | 0,858 | 0,975 | 0,961 | 0,903 | 1 |
| IFI27, IFI44L, HERC6, SLC1A2, IL1R2, OLAH, RETN | 0,881 | 0,871 | 0,890 | 0,924 | 0,872 | 0,976 | 0,936 | 0,871 | 1 |
| RSAD2, IFI27, OAS1, SLC1A2, IL1R2, OLAH, RETN | 0,881 | 0,872 | 0,890 | 0,926 | 0,874 | 0,978 | 0,937 | 0,867 | 1 |
| IFI27, OAS1, IFI44L, SIGLEC1, SLC1A2, IL1R2, OLAH | 0,881 | 0,872 | 0,890 | 0,926 | 0,876 | 0,977 | 0,947 | 0,892 | 1 |
| RSAD2, IFI27, IFIT1, SIGLEC1, ISG15, HERC6, IL1R2 | 0,881 | 0,873 | 0,889 | 0,922 | 0,875 | 0,969 | 0,946 | 0,897 | 0,995 |
| RSAD2, OAS1, IFIT1, SIGLEC1, IL1R2, OLAH, FAM20A | 0,881 | 0,872 | 0,890 | 0,912 | 0,849 | 0,975 | 0,965 | 0,917 | 1 |
| RSAD2, OAS1, SIGLEC1, SLC1A2, IL1R2, FAM20A, MMP8 | 0,881 | 0,871 | 0,890 | 0,922 | 0,874 | 0,971 | 0,960 | 0,896 | 1 |
| IFI27, OAS1, IFIT1, SIGLEC1, SLC1A2, IL1R2, RETN | 0,881 | 0,872 | 0,890 | 0,925 | 0,876 | 0,974 | 0,952 | 0,895 | 1 |
| RSAD2, IFI27, OAS1, IFIT1, SIGLEC1, IL1R2, RETN | 0,881 | 0,872 | 0,889 | 0,919 | 0,864 | 0,973 | 0,958 | 0,907 | 1 |
| SIGLEC1, ISG15, HERC6, SLC1A2, IL1R2, FAM20A, MMP8 | 0,881 | 0,871 | 0,890 | 0,924 | 0,876 | 0,972 | 0,959 | 0,893 | 1 |
| IFIT1, SIGLEC1, HERC6, SLC1A2, IL1R2, OLAH, FAM20A | 0,881 | 0,871 | 0,891 | 0,924 | 0,874 | 0,974 | 0,943 | 0,872 | 1 |
| RSAD2, IFI27, OAS1, IFIT1, ISG15, OLAH, MMP8 | 0,881 | 0,872 | 0,890 | 0,915 | 0,857 | 0,973 | 0,934 | 0,872 | 0,995 |
| RSAD2, IFI27, SIGLEC1, ISG15, HERC6, RETN, MMP8 | 0,881 | 0,871 | 0,890 | 0,918 | 0,860 | 0,976 | 0,954 | 0,907 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, HERC6, SLC1A2, IL1R2, RETN | 0,881 | 0,872 | 0,890 | 0,925 | 0,878 | 0,973 | 0,950 | 0,900 | 1 |
| RSAD2, IFI27, OAS1, IFIT1, IFI44L, SIGLEC1, IL1R2 | 0,881 | 0,873 | 0,889 | 0,919 | 0,867 | 0,970 | 0,949 | 0,901 | 0,997 |
| RSAD2, IFI27, IFI44L, SIGLEC1, ISG15, IL1R2, RETN | 0,881 | 0,872 | 0,889 | 0,920 | 0,865 | 0,974 | 0,947 | 0,896 | 0,998 |
| RSAD2, IFI27, IFIT1, IFI44L, SLC1A2, IL1R2, FAM20A | 0,881 | 0,872 | 0,890 | 0,924 | 0,874 | 0,975 | 0,927 | 0,843 | 1 |
| IFI27, OAS1, IFIT1, IFI44L, ISG15, IL1R2, OLAH | 0,881 | 0,872 | 0,889 | 0,918 | 0,863 | 0,972 | 0,938 | 0,881 | 0,995 |
| RSAD2, SIGLEC1, ISG15, SLC1A2, IL1R2, FAM20A, MMP8 | 0,881 | 0,871 | 0,890 | 0,926 | 0,878 | 0,973 | 0,959 | 0,893 | 1 |
| OAS1, IFIT1, SIGLEC1, ISG15, OLAH, FAM20A, MMP8 | 0,881 | 0,871 | 0,890 | 0,912 | 0,848 | 0,977 | 0,960 | 0,911 | 1 |
| IFI27, OAS1, SIGLEC1, SLC1A2, IL1R2, RETN, MMP8 | 0,881 | 0,872 | 0,889 | 0,927 | 0,880 | 0,974 | 0,963 | 0,916 | 1 |
| IFI27, IFIT1, ISG15, HERC6, SLC1A2, IL1R2, FAM20A | 0,881 | 0,871 | 0,890 | 0,929 | 0,882 | 0,977 | 0,925 | 0,840 | 1 |
| IFI27, ISG15, SLC1A2, IL1R2, FAM20A, RETN, MMP8 | 0,881 | 0,872 | 0,890 | 0,927 | 0,878 | 0,976 | 0,913 | 0,821 | 1 |
| RSAD2, IFI27, IFIT1, IFI44L, SIGLEC1, SLC1A2, FAM20A | 0,881 | 0,872 | 0,890 | 0,922 | 0,865 | 0,979 | 0,940 | 0,866 | 1 |
| RSAD2, OAS1, IFIT1, SIGLEC1, OLAH, FAM20A, MMP8 | 0,881 | 0,871 | 0,890 | 0,912 | 0,848 | 0,977 | 0,959 | 0,910 | 1 |
| RSAD2, IFI27, OAS1, IFI44L, HERC6, SLC1A2, OLAH | 0,881 | 0,871 | 0,890 | 0,929 | 0,879 | 0,978 | 0,932 | 0,864 | 0,999 |
| RSAD2, IFI27, IFI44L, SIGLEC1, HERC6, RETN, MMP8 | 0,881 | 0,872 | 0,890 | 0,916 | 0,857 | 0,974 | 0,943 | 0,889 | 0,998 |
| RSAD2, IFI27, OAS1, IFIT1, ISG15, SLC1A2, OLAH | 0,881 | 0,872 | 0,890 | 0,926 | 0,873 | 0,978 | 0,933 | 0,866 | 0,999 |
| IFI27, OAS1, IFIT1, IFI44L, HERC6, IL1R2, MMP8 | 0,881 | 0,873 | 0,889 | 0,921 | 0,872 | 0,970 | 0,938 | 0,883 | 0,993 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, HERC6, IL1R2, MMP8 | 0,881 | 0,873 | 0,889 | 0,919 | 0,871 | 0,968 | 0,935 | 0,877 | 0,992 |
| IFI27, IFI44L, ISG15, HERC6, SLC1A2, IL1R2, OLAH | 0,881 | 0,872 | 0,890 | 0,928 | 0,879 | 0,977 | 0,926 | 0,858 | 0,994 |
| IFI27, IFIT1, IFI44L, ISG15, SLC1A2, IL1R2, FAM20A | 0,881 | 0,871 | 0,890 | 0,925 | 0,876 | 0,975 | 0,927 | 0,842 | 1 |
| RSAD2, IFI27, OAS1, IFI44L, ISG15, SLC1A2, OLAH | 0,881 | 0,872 | 0,890 | 0,926 | 0,873 | 0,978 | 0,929 | 0,861 | 0,998 |
| RSAD2, IFI27, OAS1, IFI44L, ISG15, OLAH, MMP8 | 0,881 | 0,872 | 0,889 | 0,917 | 0,860 | 0,975 | 0,935 | 0,876 | 0,994 |
| OAS1, SIGLEC1, IL1R2, OLAH, FAM20A, RETN, MMP8 | 0,881 | 0,871 | 0,890 | 0,918 | 0,860 | 0,976 | 0,965 | 0,913 | 1 |
| OAS1, IFIT1, SIGLEC1, HERC6, SLC1A2, IL1R2, FAM20A | 0,881 | 0,871 | 0,890 | 0,925 | 0,877 | 0,973 | 0,945 | 0,873 | 1 |
| IFIT1, SIGLEC1, ISG15, HERC6, SLC1A2, IL1R2, FAM20A | 0,881 | 0,871 | 0,890 | 0,924 | 0,875 | 0,973 | 0,946 | 0,874 | 1 |
| OAS1, SIGLEC1, ISG15, IL1R2, FAM20A, RETN, MMP8 | 0,881 | 0,871 | 0,890 | 0,917 | 0,859 | 0,976 | 0,964 | 0,910 | 1 |
| RSAD2, IFI44L, SIGLEC1, SLC1A2, IL1R2, FAM20A, MMP8 | 0,881 | 0,871 | 0,890 | 0,923 | 0,874 | 0,972 | 0,959 | 0,895 | 1 |
| IFI27, OAS1, IFIT1, ISG15, SLC1A2, OLAH, RETN | 0,881 | 0,872 | 0,890 | 0,926 | 0,874 | 0,978 | 0,938 | 0,872 | 1 |
| OAS1, IFI44L, SIGLEC1, IL1R2, FAM20A, RETN, MMP8 | 0,881 | 0,871 | 0,890 | 0,918 | 0,860 | 0,976 | 0,963 | 0,908 | 1 |
| RSAD2, IFI27, SIGLEC1, ISG15, IL1R2, RETN, MMP8 | 0,881 | 0,872 | 0,889 | 0,922 | 0,869 | 0,975 | 0,963 | 0,922 | 1 |
| IFIT1, SIGLEC1, SLC1A2, IL1R2, FAM20A, RETN, MMP8 | 0,881 | 0,871 | 0,890 | 0,928 | 0,881 | 0,975 | 0,959 | 0,893 | 1 |
| IFI27, OAS1, ISG15, SLC1A2, OLAH, RETN, MMP8 | 0,881 | 0,871 | 0,890 | 0,927 | 0,874 | 0,979 | 0,937 | 0,871 | 1 |
| IFI27, OAS1, IFI44L, ISG15, IL1R2, OLAH, RETN | 0,880 | 0,872 | 0,889 | 0,919 | 0,863 | 0,975 | 0,940 | 0,881 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SLC1A2, OLAH, RETN, MMP8 | 0,880 | 0,871 | 0,890 | 0,925 | 0,871 | 0,980 | 0,940 | 0,875 | 1 |
| IFI27, IFI44L, SIGLEC1, HERC6, SLC1A2, IL1R2, RETN | 0,880 | 0,872 | 0,889 | 0,926 | 0,876 | 0,976 | 0,946 | 0,894 | 0,998 |
| IFI27, OAS1, IFIT1, SLC1A2, IL1R2, FAM20A, RETN | 0,880 | 0,871 | 0,890 | 0,927 | 0,877 | 0,978 | 0,924 | 0,839 | 1 |
| IFI27, IFI44L, SIGLEC1, SLC1A2, IL1R2, RETN, MMP8 | 0,880 | 0,872 | 0,889 | 0,929 | 0,882 | 0,975 | 0,963 | 0,922 | 1 |
| RSAD2, IFIT1, SIGLEC1, HERC6, FAM20A, RETN, MMP8 | 0,880 | 0,871 | 0,889 | 0,917 | 0,857 | 0,977 | 0,940 | 0,872 | 1 |
| RSAD2, OAS1, IFIT1, SIGLEC1, HERC6, FAM20A, RETN | 0,880 | 0,871 | 0,890 | 0,916 | 0,856 | 0,975 | 0,942 | 0,876 | 1 |
| IFI27, OAS1, IFI44L, ISG15, SLC1A2, OLAH, MMP8 | 0,880 | 0,871 | 0,890 | 0,927 | 0,875 | 0,979 | 0,924 | 0,853 | 0,995 |
| RSAD2, IFI27, ISG15, SLC1A2, OLAH, RETN, MMP8 | 0,880 | 0,871 | 0,890 | 0,927 | 0,873 | 0,980 | 0,938 | 0,873 | 1 |
| RSAD2, IFI27, OAS1, IFIT1, IFI44L, OLAH, MMP8 | 0,880 | 0,872 | 0,889 | 0,916 | 0,858 | 0,974 | 0,941 | 0,884 | 0,999 |
| RSAD2, IFI27, IFI44L, SLC1A2, OLAH, RETN, MMP8 | 0,880 | 0,871 | 0,890 | 0,926 | 0,872 | 0,980 | 0,942 | 0,880 | 1 |
| IFI27, IFIT1, IFI44L, HERC6, SLC1A2, OLAH, MMP8 | 0,880 | 0,871 | 0,890 | 0,927 | 0,877 | 0,978 | 0,932 | 0,868 | 0,995 |
| RSAD2, IFI27, IFIT1, SLC1A2, IL1R2, OLAH, RETN | 0,880 | 0,872 | 0,889 | 0,923 | 0,871 | 0,976 | 0,934 | 0,866 | 1 |
| IFI27, OAS1, IFI44L, SIGLEC1, SLC1A2, FAM20A, RETN | 0,880 | 0,871 | 0,890 | 0,922 | 0,865 | 0,979 | 0,938 | 0,860 | 1 |
| RSAD2, IFIT1, SIGLEC1, SLC1A2, OLAH, FAM20A, MMP8 | 0,880 | 0,871 | 0,890 | 0,923 | 0,870 | 0,976 | 0,951 | 0,892 | 1 |
| RSAD2, IFI27, IFI44L, ISG15, SLC1A2, IL1R2, FAM20A | 0,880 | 0,871 | 0,890 | 0,929 | 0,882 | 0,977 | 0,927 | 0,843 | 1 |
| IFI27, IFIT1, IFI44L, SLC1A2, IL1R2, OLAH, MMP8 | 0,880 | 0,872 | 0,889 | 0,925 | 0,875 | 0,975 | 0,937 | 0,877 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, SIGLEC1, ISG15, IL1R2, FAM20A, RETN, MMP8 | 0,880 | 0,871 | 0,890 | 0,918 | 0,861 | 0,976 | 0,963 | 0,909 | 1 |
| RSAD2,IFI27,OAS1,IFIT1, HERC6, RETN, MMP8 | 0,880 | 0,871 | 0,890 | 0,915 | 0,853 | 0,977 | 0,934 | 0,862 | 1 |
| IFIT1, IFI44L, SIGLEC1, ISG15 IL1R2, OLAH, FAM20A | 0,880 | 0,872 | 0,889 | 0,909 | 0,845 | 0,973 | 0,958 | 0,907 | 1 |
| IFI27, OAS1, IFIT1, IFI44L, SLC1A2, OLAH, MMP8 | 0,880 | 0,871 | 0,890 | 0,927 | 0,875 | 0,978 | 0,939 | 0,877 | 1 |
| IFI44L, SIGLEC1, ISG15, HERC6, IL1R2, OLAH, FAM20A | 0,880 | 0,871 | 0,890 | 0,914 | 0,852 | 0,975 | 0,957 | 0,898 | 1 |
| IFI27, IFIT1, IFI44L, ISG15, HERC6, SLC1A2, OLAH | 0,880 | 0,871 | 0,889 | 0,928 | 0,877 | 0,978 | 0,929 | 0,865 | 0,994 |
| RSAD2,IFI27,OAS1,IFIT1, IFI44L, HERC6, IL1R2 | 0,880 | 0,872 | 0,888 | 0,919 | 0,870 | 0,969 | 0,937 | 0,881 | 0,993 |
| IFI27, OAS1, IFIT1, SIGLEC1, HERC6, SLC1A2, IL1R2 | 0,880 | 0,872 | 0,889 | 0,926 | 0,881 | 0,971 | 0,950 | 0,900 | 1,000 |
| IFI27, IFI44L, HERC6, SLC1A2, IL1R2, OLAH, MMP8 | 0,880 | 0,871 | 0,890 | 0,927 | 0,877 | 0,976 | 0,930 | 0,866 | 0,995 |
| RSAD2, IFI27, OAS1, IFI44L, SIGLEC1, IL1R2, MMP8 | 0,880 | 0,872 | 0,889 | 0,920 | 0,870 | 0,970 | 0,952 | 0,907 | 0,997 |
| IFI27, OAS1, ISG15, HERC6,SLC1A2,FAM20A, RETN | 0,880 | 0,870 | 0,890 | 0,922 | 0,867 | 0,978 | 0,930 | 0,849 | 1 |
| RSAD2, IFI27, IFIT1, IFI44L, SIGLEC1, IL1R2, MMP8 | 0,880 | 0,872 | 0,888 | 0,920 | 0,871 | 0,970 | 0,946 | 0,897 | 0,994 |
| OAS1, IFIT1, SIGLEC1, IL1R2, OLAH, FAM20A, RETN | 0,880 | 0,871 | 0,889 | 0,911 | 0,848 | 0,974 | 0,962 | 0,911 | 1 |
| RSAD2, IFI27, IFI44L, ISG15, OLAH, RETN, MMP8 | 0,880 | 0,871 | 0,889 | 0,918 | 0,859 | 0,978 | 0,948 | 0,890 | 1 |
| SIGLEC1, ISG15, SLC1A2, IL1R2, FAM20A, RETN, MMP8 | 0,880 | 0,871 | 0,889 | 0,924 | 0,875 | 0,973 | 0,955 | 0,885 | 1 |
| OAS1, IFIT1, IFI44L, SIGLEC1, ISG15, IL1R2, FAM20A | 0,880 | 0,871 | 0,889 | 0,912 | 0,849 | 0,975 | 0,959 | 0,907 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, HERC6, SLC1A2, IL1R2, RETN | 0,880 | 0,871 | 0,889 | 0,927 | 0,881 | 0,974 | 0,952 | 0,905 | 1,000 |
| IFI27, IFIT1, IFI44L, SLC1A2, OLAH, RETN, MMP8 | 0,880 | 0,871 | 0,890 | 0,923 | 0,867 | 0,979 | 0,943 | 0,882 | 1 |
| RSAD2, IFI27, OAS1, HERC6, SLC1A2, IL1R2, RETN | 0,880 | 0,871 | 0,889 | 0,924 | 0,874 | 0,974 | 0,932 | 0,858 | 1 |
| RSAD2, IFI27, OAS1, IFIT1, ISG15, OLAH, RETN | 0,880 | 0,871 | 0,889 | 0,915 | 0,855 | 0,974 | 0,941 | 0,879 | 1 |
| IFI27, OAS1, IFIT1, IFI44L, SIGLEC1, ISG15, IL1R2 | 0,880 | 0,872 | 0,888 | 0,920 | 0,871 | 0,969 | 0,946 | 0,896 | 0,995 |
| RSAD2, IFI27, OAS1, IFIT1, IFI44L, OLAH, RETN | 0,880 | 0,871 | 0,889 | 0,915 | 0,855 | 0,974 | 0,946 | 0,884 | 1 |
| IFI27, OAS1, IFIT1, IFI44L, ISG15, OLAH, RETN | 0,880 | 0,871 | 0,889 | 0,916 | 0,857 | 0,975 | 0,940 | 0,878 | 1 |
| IFI27, IFIT1, IFI44L, ISG15, SLC1A2, IL1R2, OLAH | 0,880 | 0,871 | 0,889 | 0,927 | 0,878 | 0,977 | 0,927 | 0,860 | 0,994 |
| RSAD2, IFI27, ISG15, SLC1A2, IL1R2, OLAH, RETN | 0,880 | 0,871 | 0,889 | 0,926 | 0,875 | 0,978 | 0,932 | 0,863 | 1,000 |
| RSAD2, SIGLEC1, SLC1A2, IL1R2, FAM20A, RETN, MMP8 | 0,880 | 0,871 | 0,890 | 0,924 | 0,875 | 0,973 | 0,958 | 0,894 | 1 |
| RSAD2, IFI27, OAS1, SIGLEC1, HERC6, SLC1A2, IL1R2 | 0,880 | 0,871 | 0,889 | 0,927 | 0,882 | 0,972 | 0,953 | 0,905 | 1 |
| IFI27, SIGLEC1, HERC6, SLC1A2, IL1R2, OLAH, RETN | 0,880 | 0,871 | 0,889 | 0,925 | 0,872 | 0,977 | 0,946 | 0,889 | 1 |
| IFI27, OAS1, SIGLEC1, HERC6, SLC1A2, IL1R2, MMP8 | 0,880 | 0,871 | 0,889 | 0,927 | 0,882 | 0,971 | 0,948 | 0,897 | 0,999 |
| RSAD2, IFI27, OAS1, SIGLEC1, SLC1A2, FAM20A, RETN | 0,880 | 0,870 | 0,890 | 0,923 | 0,866 | 0,980 | 0,939 | 0,862 | 1 |
| OAS1, IFI44L, SIGLEC1, SLC1A2, IL1R2, FAM20A, MMP8 | 0,880 | 0,871 | 0,889 | 0,923 | 0,874 | 0,971 | 0,961 | 0,901 | 1 |
| RSAD2, IFIT1, SIGLEC1, ISG15, HERC6, FAM20A, RETN | 0,880 | 0,871 | 0,889 | 0,916 | 0,857 | 0,976 | 0,942 | 0,875 | 1 |
| RSAD2, IFI27, OAS1, IFI44L, HERC6, RETN, MMP8 | 0,880 | 0,871 | 0,889 | 0,915 | 0,853 | 0,977 | 0,934 | 0,862 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SLC1A2, IL1R2, OLAH, MMP8 | 0,880 | 0,871 | 0,889 | 0,925 | 0,875 | 0,976 | 0,935 | 0,871 | 0,998 |
| IFI27, IFIT1, IFI44L, ISG15, SLC1A2, OLAH, RETN | 0,880 | 0,871 | 0,889 | 0,927 | 0,876 | 0,979 | 0,934 | 0,866 | 1 |
| RSAD2, IFI27, OAS1, IFI44L, SLC1A2, IL1R2, FAM20A | 0,880 | 0,871 | 0,889 | 0,927 | 0,879 | 0,975 | 0,927 | 0,843 | 1 |
| IFI27, OAS1, IFI44L, ISG15, SLC1A2, IL1R2, FAM20A | 0,880 | 0,871 | 0,889 | 0,930 | 0,882 | 0,977 | 0,926 | 0,842 | 1 |
| RSAD2, IFI27, OAS1, SIGLEC1, ISG15, IL1R2, RETN | 0,880 | 0,871 | 0,889 | 0,920 | 0,866 | 0,973 | 0,955 | 0,902 | 1 |
| RSAD2, IFI27, IFI44L, ISG15, IL1R2, OLAH, RETN | 0,880 | 0,871 | 0,889 | 0,918 | 0,860 | 0,975 | 0,939 | 0,878 | 1 |
| RSAD2, IFIT1, SIGLEC1, HERC6, SLC1A2, IL1R2, FAM20A | 0,880 | 0,871 | 0,889 | 0,925 | 0,877 | 0,974 | 0,946 | 0,874 | 1 |
| IFI27, IFIT1, IFI44L, SIGLEC1, SLC1A2, IL1R2, RETN | 0,880 | 0,871 | 0,888 | 0,923 | 0,872 | 0,973 | 0,950 | 0,897 | 1 |
| RSAD2, IFIT1, SIGLEC1, HERC6, SLC1A2, OLAH, FAM20A | 0,880 | 0,870 | 0,890 | 0,921 | 0,868 | 0,974 | 0,939 | 0,866 | 1 |
| RSAD2, IFIT1, IFI44L, SIGLEC1, IL1R2, OLAH, FAM20A | 0,880 | 0,871 | 0,889 | 0,911 | 0,847 | 0,974 | 0,962 | 0,913 | 1 |
| IFI27, IFIT1, ISG15, HERC6, IL1R2, RETN, MMP8 | 0,880 | 0,871 | 0,889 | 0,922 | 0,868 | 0,975 | 0,946 | 0,892 | 0,999 |
| IFI27, IFI44L, SIGLEC1, SLC1A2, FAM20A, RETN, MMP8 | 0,880 | 0,870 | 0,890 | 0,923 | 0,867 | 0,980 | 0,950 | 0,875 | 1 |
| IFI27, OAS1, ISG15, SLC1A2, IL1R2, FAM20A, MMP8 | 0,880 | 0,871 | 0,889 | 0,927 | 0,878 | 0,975 | 0,927 | 0,841 | 1 |
| RSAD2, IFI27, IFI44L, ISG15, SLC1A2, IL1R2, OLAH | 0,880 | 0,871 | 0,889 | 0,927 | 0,877 | 0,977 | 0,929 | 0,863 | 0,996 |
| RSAD2, IFI27, IFIT1, ISG15, IL1R2, OLAH, RETN | 0,880 | 0,871 | 0,888 | 0,919 | 0,863 | 0,975 | 0,941 | 0,882 | 1 |
| RSAD2, IFI27, OAS1, IFIT1, IL1R2, RETN, MMP8 | 0,880 | 0,871 | 0,889 | 0,920 | 0,865 | 0,974 | 0,953 | 0,902 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, ISG15, IL1R2, OLAH | 0,880 | 0,872 | 0,888 | 0,919 | 0,865 | 0,973 | 0,938 | 0,881 | 0,995 |
| IFI27, OAS1, IFIT1, HERC6, SLC1A2, IL1R2, RETN | 0,880 | 0,871 | 0,889 | 0,924 | 0,875 | 0,973 | 0,938 | 0,873 | 1 |
| IFI27, OAS1, IFI44L, HERC6, SLC1A2, FAM20A, RETN | 0,880 | 0,870 | 0,889 | 0,923 | 0,868 | 0,979 | 0,935 | 0,853 | 1 |
| RSAD2, IFI27, OAS1, IFI44L, IL1R2, OLAH, RETN | 0,880 | 0,871 | 0,889 | 0,918 | 0,862 | 0,975 | 0,945 | 0,886 | 1 |
| IFI27, OAS1, ISG15, SLC1A2, IL1R2, OLAH, RETN | 0,880 | 0,871 | 0,889 | 0,927 | 0,876 | 0,977 | 0,930 | 0,861 | 1,000 |
| RSAD2, IFIT1, SIGLEC1, HERC6, OLAH, FAM20A, RETN | 0,880 | 0,871 | 0,889 | 0,915 | 0,854 | 0,976 | 0,948 | 0,889 | 1 |
| IFI27, OAS1, IFI44L, HERC6, SLC1A2, IL1R2, OLAH | 0,880 | 0,870 | 0,890 | 0,928 | 0,879 | 0,977 | 0,928 | 0,860 | 0,997 |
| IFI27, OAS1, IFIT1, ISG15, SLC1A2, IL1R2, FAM20A | 0,880 | 0,870 | 0,889 | 0,924 | 0,874 | 0,974 | 0,925 | 0,840 | 1 |
| RSAD2, IFI27, IFIT1, IFI44L, HERC6, SLC1A2, OLAH | 0,880 | 0,871 | 0,889 | 0,927 | 0,877 | 0,978 | 0,930 | 0,865 | 0,995 |
| OAS1, IFIT1, SIGLEC1, HERC6, SLC1A2, OLAH, FAM20A | 0,880 | 0,870 | 0,890 | 0,922 | 0,870 | 0,973 | 0,937 | 0,867 | 1 |
| RSAD2, IFI27, SIGLEC1, ISG15, SLC1A2, FAM20A, RETN | 0,880 | 0,870 | 0,890 | 0,922 | 0,866 | 0,979 | 0,941 | 0,866 | 1 |
| RSAD2, IFI27, OAS1, IFI44L, SLC1A2, OLAH, MMP8 | 0,880 | 0,870 | 0,889 | 0,924 | 0,872 | 0,977 | 0,937 | 0,872 | 1 |
| IFI27, IFIT1, SIGLEC1, ISG15, SLC1A2, FAM20A, MMP8 | 0,880 | 0,870 | 0,889 | 0,922 | 0,865 | 0,978 | 0,941 | 0,865 | 1 |
| RSAD2, IFI27, SIGLEC1, SLC1A2, IL1R2, RETN, MMP8 | 0,880 | 0,871 | 0,888 | 0,928 | 0,881 | 0,975 | 0,964 | 0,922 | 1 |
| RSAD2, IFI27, OAS1, IFIT1, SLC1A2, OLAH, RETN | 0,880 | 0,871 | 0,889 | 0,927 | 0,874 | 0,979 | 0,940 | 0,873 | 1 |
| RSAD2, IFI27, IFI44L, HERC6, SLC1A2, IL1R2, OLAH | 0,880 | 0,870 | 0,889 | 0,926 | 0,876 | 0,976 | 0,929 | 0,864 | 0,995 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, SLC1A2, IL1R2, RETN, MMP8 | 0,880 | 0,871 | 0,888 | 0,926 | 0,878 | 0,973 | 0,967 | 0,929 | 1 |
| IFI27, OAS1, HERC6, SLC1A2, IL1R2, RETN, MMP8 | 0,880 | 0,871 | 0,888 | 0,925 | 0,876 | 0,974 | 0,926 | 0,854 | 0,998 |
| IFIT1, IFI44L, SIGLEC1, ISG15, SLC1A2, OLAH, FAM20A | 0,880 | 0,870 | 0,889 | 0,920 | 0,865 | 0,975 | 0,934 | 0,868 | 1,000 |
| IFI27, IFIT1, SIGLEC1, ISG15, SLC1A2, IL1R2, RETN | 0,880 | 0,871 | 0,888 | 0,925 | 0,877 | 0,973 | 0,952 | 0,901 | 1 |
| IFI27, OAS1, IFI44L, SLC1A2, IL1R2, FAM20A, RETN | 0,880 | 0,870 | 0,889 | 0,926 | 0,875 | 0,977 | 0,925 | 0,840 | 1 |
| IFI27, OAS1, IFI44L, SLC1A2, OLAH, RETN, MMP8 | 0,879 | 0,870 | 0,889 | 0,926 | 0,872 | 0,979 | 0,942 | 0,880 | 1 |
| IFI27, OAS1, IFI44L, SIGLEC1, ISG15, IL1R2, MMP8 | 0,879 | 0,872 | 0,887 | 0,919 | 0,870 | 0,968 | 0,940 | 0,888 | 0,992 |
| IFI27, IFIT1, IFI44L, SLC1A2, IL1R2, OLAH, RETN | 0,879 | 0,871 | 0,888 | 0,922 | 0,870 | 0,975 | 0,935 | 0,869 | 1 |
| IFIT1, IFI44L, SIGLEC1, ISG15, HERC6, FAM20A, MMP8 | 0,879 | 0,870 | 0,889 | 0,913 | 0,851 | 0,975 | 0,935 | 0,863 | 1 |
| IFI27, IFI44L, SIGLEC1, ISG15, SLC1A2, IL1R2, MMP8 | 0,879 | 0,872 | 0,887 | 0,924 | 0,879 | 0,970 | 0,945 | 0,893 | 0,996 |
| RSAD2, IFI27, IFIT1, IFI44L, IL1R2, OLAH, RETN | 0,879 | 0,871 | 0,888 | 0,916 | 0,859 | 0,974 | 0,942 | 0,883 | 1 |
| IFI27, OAS1, IFIT1, IFI44L, IL1R2, RETN, MMP8 | 0,879 | 0,871 | 0,888 | 0,919 | 0,864 | 0,975 | 0,945 | 0,889 | 1 |
| RSAD2, IFI27, IFIT1, IFI44L, SLC1A2, OLAH, MMP8 | 0,879 | 0,871 | 0,888 | 0,926 | 0,875 | 0,978 | 0,935 | 0,871 | 0,998 |
| RSAD2, IFI27, OAS1, SIGLEC1, SLC1A2, IL1R2, RETN | 0,879 | 0,871 | 0,888 | 0,922 | 0,872 | 0,973 | 0,954 | 0,896 | 1 |
| IFI27, IFIT1, ISG15, SLC1A2, IL1R2, FAM20A, RETN | 0,879 | 0,870 | 0,889 | 0,927 | 0,878 | 0,977 | 0,926 | 0,842 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, SIGLEC1, ISG15, SLC1A2, FAM20A, RETN, MMP8 | 0,879 | 0,869 | 0,890 | 0,922 | 0,864 | 0,980 | 0,952 | 0,878 | 1 |
| IFIT1, SIGLEC1, HERC6, SLC1A2, FAM20A, RETN, MMP8 | 0,879 | 0,870 | 0,889 | 0,920 | 0,864 | 0,975 | 0,948 | 0,877 | 1 |
| RSAD2, IFI27, IFI44L, SLC1A2, IL1R2, OLAH, RETN | 0,879 | 0,871 | 0,888 | 0,923 | 0,871 | 0,976 | 0,934 | 0,866 | 1 |
| RSAD2, IFI27, IFIT1, SLC1A2, IL1R2, FAM20A, RETN | 0,879 | 0,870 | 0,888 | 0,926 | 0,876 | 0,977 | 0,929 | 0,847 | 1 |
| SIGLEC1, SLC1A2, IL1R2, OLAH, FAM20A, RETN, MMP8 | 0,879 | 0,870 | 0,889 | 0,923 | 0,872 | 0,973 | 0,958 | 0,892 | 1 |
| RSAD2, IFI27, OAS1, IFIT1, SLC1A2, IL1R2, FAM20A | 0,879 | 0,870 | 0,888 | 0,925 | 0,876 | 0,975 | 0,924 | 0,838 | 1 |
| RSAD2, IFIT1, SIGLEC1, ISG15, IL1R2, OLAH, FAM20A | 0,879 | 0,870 | 0,888 | 0,910 | 0,846 | 0,974 | 0,961 | 0,911 | 1 |
| RSAD2, IFI27, IFIT1, ISG15, SLC1A2, OLAH, RETN | 0,879 | 0,871 | 0,888 | 0,927 | 0,876 | 0,979 | 0,934 | 0,866 | 1 |
| IFI27, OAS1, IFIT1, SIGLEC1, ISG15, SLC1A2, FAM20A | 0,879 | 0,870 | 0,889 | 0,922 | 0,867 | 0,977 | 0,941 | 0,867 | 1 |
| RSAD2, IFI27, ISG15, SLC1A2, IL1R2, FAM20A, RETN | 0,879 | 0,870 | 0,888 | 0,929 | 0,881 | 0,977 | 0,923 | 0,836 | 1 |
| RSAD2, IFI27, IFI44L, ISG15, SLC1A2, OLAH, RETN | 0,879 | 0,871 | 0,888 | 0,928 | 0,877 | 0,979 | 0,933 | 0,866 | 1,000 |
| RSAD2, IFI27, IFI44L, SLC1A2, IL1R2, FAM20A, RETN | 0,879 | 0,870 | 0,889 | 0,927 | 0,877 | 0,977 | 0,925 | 0,840 | 1 |
| RSAD2, IFI27, IFI44L, SIGLEC1, ISG15, IL1R2, MMP8 | 0,879 | 0,871 | 0,887 | 0,919 | 0,869 | 0,970 | 0,940 | 0,888 | 0,992 |
| RSAD2, IFI27, OAS1, IFIT1, IFI44L, SLC1A2, OLAH | 0,879 | 0,870 | 0,888 | 0,927 | 0,875 | 0,979 | 0,941 | 0,879 | 1 |
| RSAD2, IFI27, IFIT1, IFI44L, HERC6, IL1R2, MMP8 | 0,879 | 0,871 | 0,887 | 0,918 | 0,868 | 0,969 | 0,937 | 0,882 | 0,992 |
| RSAD2, IFI27, OAS1, ISG15, SLC1A2, OLAH, RETN | 0,879 | 0,870 | 0,888 | 0,927 | 0,875 | 0,979 | 0,932 | 0,860 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, IL1R2 | 0,879 | 0,871 | 0,887 | 0,918 | 0,866 | 0,969 | 0,943 | 0,893 | 0,994 |
| IFI27, OAS1, SIGLEC1, ISG15, HERC6, SLC1A2, IL1R2 | 0,879 | 0,871 | 0,888 | 0,927 | 0,882 | 0,971 | 0,949 | 0,899 | 0,999 |
| RSAD2, IFI27, IFIT1, IFI44L, ISG15, OLAH, MMP8 | 0,879 | 0,870 | 0,888 | 0,916 | 0,858 | 0,974 | 0,936 | 0,876 | 0,996 |
| IFI27, OAS1, IFIT1, IFI44L, ISG15, SLC1A2, OLAH | 0,879 | 0,870 | 0,888 | 0,927 | 0,875 | 0,978 | 0,929 | 0,862 | 0,997 |
| IFI27, IFI44L, SIGLEC1, ISG15, HERC6, RETN, MMP8 | 0,879 | 0,870 | 0,889 | 0,911 | 0,849 | 0,973 | 0,941 | 0,884 | 0,999 |
| OAS1, SIGLEC1, ISG15, SLC1A2, IL1R2, FAM20A, MMP8 | 0,879 | 0,870 | 0,889 | 0,924 | 0,876 | 0,972 | 0,959 | 0,893 | 1 |
| RSAD2, IFI27, IFIT1, ISG15, SLC1A2, IL1R2, FAM20A | 0,879 | 0,870 | 0,888 | 0,924 | 0,874 | 0,974 | 0,926 | 0,841 | 1 |
| IFI27, OAS1, ISG15, HERC6, SLC1A2, IL1R2, RETN | 0,879 | 0,870 | 0,888 | 0,926 | 0,877 | 0,975 | 0,933 | 0,863 | 1 |
| RSAD2, OAS1, IFIT1, IFI44L, SIGLEC1, IL1R2, FAM20A | 0,879 | 0,870 | 0,888 | 0,912 | 0,850 | 0,975 | 0,963 | 0,914 | 1 |
| IFI27, IFIT1, IFI44L, ISG15, IL1R2, RETN, MMP8 | 0,879 | 0,870 | 0,888 | 0,919 | 0,863 | 0,974 | 0,943 | 0,887 | 1,000 |
| IFI27, OAS1, IFI44L, SIGLEC1, SLC1A2, IL1R2, RETN | 0,879 | 0,871 | 0,888 | 0,924 | 0,875 | 0,973 | 0,949 | 0,894 | 1 |
| IFI44L, SIGLEC1, ISG15, SLC1A2, OLAH, FAM20A, MMP8 | 0,879 | 0,870 | 0,888 | 0,916 | 0,860 | 0,971 | 0,949 | 0,885 | 1 |
| OAS1, IFIT1, SIGLEC1, OLAH, FAM20A, RETN, MMP8 | 0,879 | 0,869 | 0,889 | 0,912 | 0,848 | 0,977 | 0,960 | 0,911 | 1 |
| IFI27, OAS1, IFIT1, ISG15, HERC6, IL1R2, MMP8 | 0,879 | 0,871 | 0,887 | 0,917 | 0,868 | 0,966 | 0,939 | 0,885 | 0,993 |
| IFI27, OAS1, IFIT1, SIGLEC1, ISG15, IL1R2, MMP8 | 0,879 | 0,871 | 0,887 | 0,920 | 0,872 | 0,968 | 0,952 | 0,906 | 0,998 |
| IFI27, OAS1, SIGLEC1, ISG15, SLC1A2, IL1R2, RETN | 0,879 | 0,871 | 0,888 | 0,925 | 0,877 | 0,974 | 0,951 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, IL1R2 | 0,879 | 0,871 | 0,887 | 0,920 | 0,870 | 0,971 | 0,948 | 0,900 | 0,996 |
| RSAD2, OAS1, SIGLEC1, ISG15, HERC6, IL1R2, FAM20A | 0,879 | 0,870 | 0,888 | 0,915 | 0,857 | 0,973 | 0,957 | 0,901 | 1 |
| RSAD2, IFI27, OAS1, IFI44L, ISG15, OLAH, RETN | 0,879 | 0,870 | 0,888 | 0,916 | 0,856 | 0,976 | 0,938 | 0,874 | 1 |
| RSAD2, IFI27, OAS1, IFIT1, IFI44L, ISG15, OLAH | 0,879 | 0,870 | 0,888 | 0,915 | 0,856 | 0,974 | 0,935 | 0,875 | 0,994 |
| RSAD2, OAS1, IFIT1, SIGLEC1, ISG15, IL1R2, FAM20A | 0,879 | 0,870 | 0,888 | 0,912 | 0,848 | 0,975 | 0,964 | 0,916 | 1 |
| RSAD2, OAS1, SIGLEC1, SLC1A2, OLAH, FAM20A, MMP8 | 0,879 | 0,869 | 0,889 | 0,918 | 0,862 | 0,973 | 0,952 | 0,892 | 1 |
| IFI27, IFIT1, IFI44L, HERC6, SLC1A2, FAM20A, RETN | 0,879 | 0,869 | 0,889 | 0,922 | 0,865 | 0,978 | 0,930 | 0,847 | 1 |
| IFI27, OAS1, IFI44L, SLC1A2, IL1R2, OLAH, MMP8 | 0,879 | 0,870 | 0,888 | 0,928 | 0,879 | 0,977 | 0,932 | 0,864 | 0,999 |
| OAS1, IFI44L, SIGLEC1, ISG15, HERC6, IL1R2, FAM20A | 0,879 | 0,870 | 0,888 | 0,914 | 0,855 | 0,973 | 0,958 | 0,904 | 1 |
| IFI27, OAS1, IFIT1, IFI44L, SLC1A2, OLAH, RETN | 0,879 | 0,870 | 0,888 | 0,926 | 0,874 | 0,977 | 0,938 | 0,869 | 1 |
| IFIT1, IFI44L, SIGLEC1, SLC1A2, OLAH, FAM20A, RETN | 0,879 | 0,870 | 0,888 | 0,919 | 0,863 | 0,975 | 0,937 | 0,874 | 1 |
| OAS1, SIGLEC1, SLC1A2, IL1R2, FAM20A, RETN, MMP8 | 0,879 | 0,869 | 0,888 | 0,923 | 0,873 | 0,973 | 0,958 | 0,894 | 1 |
| RSAD2, IFI27, OAS1, IFIT1, ISG15, HERC6, IL1R2 | 0,879 | 0,871 | 0,887 | 0,918 | 0,870 | 0,966 | 0,942 | 0,890 | 0,995 |
| IFI27, OAS1, IFIT1, SIGLEC1, SLC1A2, FAM20A, MMP8 | 0,879 | 0,869 | 0,888 | 0,921 | 0,864 | 0,978 | 0,942 | 0,870 | 1 |
| RSAD2, IFI27, IFIT1, IFI44L, ISG15, OLAH, RETN | 0,879 | 0,870 | 0,887 | 0,915 | 0,856 | 0,975 | 0,939 | 0,875 | 1 |
| RSAD2, IFI27, OAS1, IFI44L, SIGLEC1, FAM20A, MMP8 | 0,879 | 0,869 | 0,889 | 0,913 | 0,849 | 0,978 | 0,948 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, OLAH, FAM20A, RETN, MMP8 | 0,879 | 0,869 | 0,888 | 0,915 | 0,853 | 0,978 | 0,957 | 0,907 | 1 |
| IFIT1, IFI44L, SIGLEC1, IL1R2, OLAH, FAM20A, RETN | 0,879 | 0,870 | 0,888 | 0,908 | 0,844 | 0,971 | 0,959 | 0,907 | 1 |
| IFI27, SIGLEC1, ISG15, SLC1A2, IL1R2, RETN, MMP8 | 0,879 | 0,870 | 0,887 | 0,926 | 0,878 | 0,973 | 0,965 | 0,925 | 1 |
| RSAD2, IFI27, OAS1, IFIT1, IFI44L, IL1R2, RETN | 0,879 | 0,870 | 0,887 | 0,915 | 0,860 | 0,970 | 0,945 | 0,887 | 1 |
| RSAD2, IFI27, OAS1, SIGLEC1, ISG15, FAM20A, MMP8 | 0,879 | 0,868 | 0,889 | 0,915 | 0,851 | 0,979 | 0,947 | 0,876 | 1 |
| RSAD2, IFI27, IFI44L, SIGLEC1, SLC1A2, FAM20A, RETN | 0,879 | 0,869 | 0,888 | 0,922 | 0,865 | 0,979 | 0,945 | 0,871 | 1 |
| RSAD2, IFI27, OAS1, IFIT1, SIGLEC1, IL1R2, MMP8 | 0,879 | 0,870 | 0,887 | 0,920 | 0,872 | 0,968 | 0,957 | 0,912 | 1 |
| RSAD2, IFIT1, IFI44L, SIGLEC1, HERC6, FAM20A, RETN | 0,879 | 0,870 | 0,888 | 0,915 | 0,855 | 0,976 | 0,934 | 0,866 | 1 |
| OAS1, IFIT1, IFI44L, SIGLEC1, SLC1A2, OLAH, FAM20A | 0,879 | 0,869 | 0,888 | 0,920 | 0,865 | 0,975 | 0,934 | 0,868 | 0,999 |
| RSAD2, IFI27, IFIT1, SIGLEC1, ISG15, IL1R2, RETN | 0,879 | 0,870 | 0,887 | 0,920 | 0,866 | 0,973 | 0,955 | 0,910 | 1 |
| RSAD2, OAS1, SIGLEC1, HERC6, IL1R2, OLAH, FAM20A | 0,879 | 0,869 | 0,888 | 0,914 | 0,856 | 0,973 | 0,958 | 0,903 | 1 |
| RSAD2, IFI44L, SIGLEC1, IL1R2, FAM20A, RETN, MMP8 | 0,879 | 0,869 | 0,888 | 0,919 | 0,862 | 0,976 | 0,964 | 0,911 | 1 |
| IFI27, OAS1, ISG15, SLC1A2, IL1R2, FAM20A, RETN | 0,879 | 0,870 | 0,888 | 0,929 | 0,880 | 0,978 | 0,923 | 0,836 | 1 |
| OAS1, IFIT1, IFI44L, SIGLEC1, IL1R2, FAM20A, RETN | 0,879 | 0,869 | 0,888 | 0,910 | 0,847 | 0,973 | 0,961 | 0,908 | 1 |
| RSAD2, IFIT1, IFI44L, SIGLEC1, OLAH, FAM20A, MMP8 | 0,879 | 0,869 | 0,888 | 0,908 | 0,842 | 0,974 | 0,955 | 0,906 | 1 |
| RSAD2, IFI27, IFIT1, IFI44L, ISG15, SLC1A2, OLAH | 0,879 | 0,870 | 0,887 | 0,926 | 0,873 | 0,978 | 0,928 | 0,861 | 0,996 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, SLC1A2, IL1R2, OLAH, FAM20A | 0,879 | 0,869 | 0,888 | 0,920 | 0,867 | 0,974 | 0,946 | 0,886 | 1 |
| IFI27, SIGLEC1, HERC6, SLC1A2, FAM20A, RETN, MMP8 | 0,878 | 0,868 | 0,889 | 0,924 | 0,866 | 0,982 | 0,951 | 0,875 | 1 |
| RSAD2, IFI27, OAS1, ISG15, SLC1A2, IL1R2, FAM20A | 0,878 | 0,869 | 0,888 | 0,927 | 0,879 | 0,974 | 0,926 | 0,840 | 1 |
| RSAD2, SIGLEC1, HERC6, SLC1A2, OLAH, FAM20A, MMP8 | 0,878 | 0,868 | 0,888 | 0,922 | 0,871 | 0,973 | 0,945 | 0,878 | 1 |
| SIGLEC1, HERC6, SLC1A2, IL1R2, FAM20A, RETN, MMP8 | 0,878 | 0,869 | 0,888 | 0,922 | 0,872 | 0,973 | 0,957 | 0,889 | 1 |
| IFI27, SIGLEC1, ISG15, HERC6, SLC1A2, FAM20A, RETN | 0,878 | 0,868 | 0,889 | 0,920 | 0,862 | 0,978 | 0,943 | 0,866 | 1 |
| IFI27, OAS1, IFI44L, HERC6, SLC1A2, IL1R2, RETN | 0,878 | 0,870 | 0,887 | 0,924 | 0,876 | 0,973 | 0,935 | 0,869 | 1 |
| RSAD2, IFI27, OAS1, HERC6, SLC1A2, FAM20A, RETN | 0,878 | 0,868 | 0,888 | 0,923 | 0,868 | 0,979 | 0,933 | 0,849 | 1 |
| RSAD2, IFI27, OAS1, ISG15, HERC6, IL1R2, MMP8 | 0,878 | 0,870 | 0,887 | 0,921 | 0,873 | 0,968 | 0,934 | 0,876 | 0,992 |
| IFIT1, SIGLEC1, HERC6, SLC1A2, OLAH, FAM20A, RETN | 0,878 | 0,868 | 0,888 | 0,919 | 0,866 | 0,973 | 0,935 | 0,861 | 1 |
| RSAD2, IFI27, OAS1, SLC1A2, IL1R2, FAM20A, RETN | 0,878 | 0,869 | 0,887 | 0,927 | 0,879 | 0,976 | 0,922 | 0,834 | 1 |
| IFIT1, SIGLEC1, HERC6, SLC1A2, IL1R2, FAM20A, RETN | 0,878 | 0,869 | 0,888 | 0,924 | 0,875 | 0,973 | 0,946 | 0,874 | 1 |
| RSAD2, IFI27, IFIT1, SIGLEC1, ISG15, SLC1A2, FAM20A | 0,878 | 0,869 | 0,887 | 0,922 | 0,867 | 0,978 | 0,940 | 0,864 | 1 |
| IFI27, OAS1, IFI44L, ISG15, HERC6, IL1R2, MMP8 | 0,878 | 0,870 | 0,886 | 0,918 | 0,870 | 0,967 | 0,928 | 0,868 | 0,988 |
| IFI27, IFIT1, IFI44L, HERC6, SLC1A2, IL1R2, OLAH | 0,878 | 0,869 | 0,888 | 0,925 | 0,875 | 0,975 | 0,933 | 0,870 | 0,995 |
| IFI27, OAS1, IFI44L, ISG15, SLC1A2, OLAH, RETN | 0,878 | 0,869 | 0,887 | 0,929 | 0,878 | 0,979 | 0,933 | 0,865 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, HERC6, SLC1A2, FAM20A, MMP8 | 0,878 | 0,868 | 0,888 | 0,920 | 0,866 | 0,974 | 0,941 | 0,865 | 1 |
| RSAD2, IFI27, OAS1, IFI44L, SLC1A2, IL1R2, OLAH | 0,878 | 0,869 | 0,887 | 0,927 | 0,877 | 0,977 | 0,932 | 0,863 | 1,000 |
| RSAD2, IFIT1, IFI44L, SIGLEC1, SLC1A2, OLAH, FAM20A | 0,878 | 0,869 | 0,887 | 0,921 | 0,866 | 0,976 | 0,938 | 0,875 | 1 |
| RSAD2, IFI27, OAS1, IFI44L, SLC1A2, OLAH, RETN | 0,878 | 0,869 | 0,887 | 0,926 | 0,873 | 0,979 | 0,938 | 0,870 | 1 |
| IFI27, IFIT1, ISG15, HERC6, SLC1A2, FAM20A, RETN | 0,878 | 0,868 | 0,888 | 0,920 | 0,864 | 0,977 | 0,936 | 0,855 | 1 |
| RSAD2, OAS1, IFI44L, SIGLEC1, HERC6, IL1R2, FAM20A | 0,878 | 0,869 | 0,887 | 0,916 | 0,857 | 0,974 | 0,959 | 0,905 | 1 |
| RSAD2, OAS1, IFIT1, SIGLEC1, ISG15, HERC6, FAM20A | 0,878 | 0,869 | 0,887 | 0,910 | 0,849 | 0,971 | 0,938 | 0,868 | 1 |
| IFI27, OAS1, IFIT1, IFI44L, SLC1A2, IL1R2, OLAH | 0,878 | 0,869 | 0,887 | 0,926 | 0,875 | 0,976 | 0,932 | 0,864 | 0,999 |
| IFI27, OAS1, IFIT1, SIGLEC1, SLC1A2, IL1R2, MMP8 | 0,878 | 0,870 | 0,886 | 0,926 | 0,882 | 0,971 | 0,953 | 0,905 | 1 |
| IFI27, OAS1, IFIT1, HERC6, SLC1A2, FAM20A, RETN | 0,878 | 0,868 | 0,888 | 0,923 | 0,866 | 0,979 | 0,934 | 0,851 | 1 |
| RSAD2, IFI27, IFIT1, IFI44L, IL1R2, RETN, MMP8 | 0,878 | 0,869 | 0,887 | 0,918 | 0,862 | 0,973 | 0,943 | 0,887 | 1,000 |
| RSAD2, IFI27, IFI44L, SIGLEC1, ISG15, FAM20A, MMP8 | 0,878 | 0,868 | 0,888 | 0,913 | 0,849 | 0,977 | 0,943 | 0,870 | 1 |
| RSAD2, IFIT1, IFI44L, SIGLEC1, ISG15, IL1R2, FAM20A | 0,878 | 0,869 | 0,887 | 0,911 | 0,847 | 0,974 | 0,963 | 0,914 | 1 |
| IFI27, OAS1, IFIT1, IFI44L, ISG15, IL1R2, RETN | 0,878 | 0,869 | 0,887 | 0,919 | 0,865 | 0,972 | 0,941 | 0,883 | 0,999 |
| IFIT1, IFI44L, SIGLEC1, ISG15, ILIR2, FAM20A, RETN | 0,878 | 0,869 | 0,887 | 0,910 | 0,847 | 0,972 | 0,960 | 0,909 | 1 |
| RSAD2, IFI27, IFIT1, IFI44L, ISG15, HERC6, RETN | 0,878 | 0,869 | 0,887 | 0,914 | 0,855 | 0,973 | 0,940 | 0,883 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SLC1A2, IL1R2, OLAH, RETN | 0,878 | 0,869 | 0,887 | 0,926 | 0,875 | 0,978 | 0,935 | 0,867 | 1 |
| RSAD2, OAS1, IFIT1, IFI44L, SIGLEC1, HERC6, FAM20A | 0,878 | 0,869 | 0,887 | 0,910 | 0,848 | 0,971 | 0,932 | 0,860 | 1 |
| OAS1, IFIT1, SIGLEC1, ISG15, IL1R2, FAM20A, RETN | 0,878 | 0,869 | 0,887 | 0,912 | 0,850 | 0,974 | 0,961 | 0,908 | 1 |
| RSAD2, IFI27, IFI44L, SIGLEC1, SLC1A2, IL1R2, RETN | 0,878 | 0,870 | 0,886 | 0,923 | 0,873 | 0,974 | 0,949 | 0,896 | 1 |
| RSAD2, IFI27, ISG15, HERC6, SLC1A2, FAM20A, RETN | 0,878 | 0,868 | 0,888 | 0,922 | 0,867 | 0,978 | 0,934 | 0,852 | 1 |
| IFI27, IFIT1, IFI44L, SIGLEC1, ISG15, SLC1A2, IL1R2 | 0,878 | 0,870 | 0,886 | 0,925 | 0,879 | 0,970 | 0,946 | 0,895 | 0,997 |
| RSAD2, IFI44L, SIGLEC1, ISG15, HERC6, IL1R2, FAM20A | 0,878 | 0,868 | 0,887 | 0,915 | 0,856 | 0,974 | 0,958 | 0,901 | 1 |
| RSAD2, IFI27, SIGLEC1, HERC6, SLC1A2, IL1R2, MMP8 | 0,878 | 0,869 | 0,887 | 0,924 | 0,879 | 0,970 | 0,949 | 0,900 | 0,998 |
| OAS1, IFIT1, SIGLEC1, SLC1A2, IL1R2, OLAH, FAM20A | 0,878 | 0,868 | 0,887 | 0,918 | 0,863 | 0,972 | 0,952 | 0,891 | 1 |
| RSAD2, IFIT1, IFI44L, SIGLEC1, ISG15, HERC6, FAM20A | 0,878 | 0,869 | 0,887 | 0,911 | 0,850 | 0,972 | 0,935 | 0,866 | 1 |
| RSAD2, IFI27, IFIT1, IFI44L, SLC1A2, OLAH, RETN | 0,878 | 0,869 | 0,887 | 0,923 | 0,871 | 0,976 | 0,939 | 0,875 | 1 |
| RSAD2, IFI27, IFIT1, IFI44L, SLC1A2, IL1R2, OLAH | 0,878 | 0,869 | 0,887 | 0,925 | 0,874 | 0,976 | 0,933 | 0,868 | 0,997 |
| IFI27, OAS1, IFIT1, IFI44L, ISG15, HERC6, IL1R2 | 0,878 | 0,869 | 0,886 | 0,921 | 0,873 | 0,970 | 0,936 | 0,879 | 0,992 |
| RSAD2, IFI27, OAS1, SIGLEC1, HERC6, SLC1A2, FAM20A | 0,878 | 0,868 | 0,888 | 0,920 | 0,866 | 0,975 | 0,940 | 0,864 | 1 |
| RSAD2, SIGLEC1, ISG15, HERC6, IL1R2, OLAH, FAM20A | 0,878 | 0,868 | 0,887 | 0,916 | 0,858 | 0,974 | 0,955 | 0,898 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, ISG15, FAM20A, MMP8 | 0,878 | 0,867 | 0,888 | 0,911 | 0,847 | 0,975 | 0,946 | 0,874 | 1 |
| OAS1, SIGLEC1, HERC6, SLC1A2, OLAH, FAM20A, MMP8 | 0,878 | 0,868 | 0,888 | 0,919 | 0,866 | 0,972 | 0,943 | 0,876 | 1 |
| RSAD2, IFI44L, SIGLEC1, HERC6, IL1R2, OLAH, FAM20A | 0,878 | 0,868 | 0,887 | 0,914 | 0,855 | 0,974 | 0,958 | 0,901 | 1 |
| IFIT1, SIGLEC1, SLC1A2, OLAH, FAM20A, RETN, MMP8 | 0,878 | 0,868 | 0,887 | 0,922 | 0,868 | 0,975 | 0,952 | 0,894 | 1 |
| IFI27, OAS1, IFIT1, ISG15, HERC6, SLC1A2, RETN | 0,878 | 0,868 | 0,887 | 0,915 | 0,857 | 0,973 | 0,933 | 0,858 | 1 |
| RSAD2, IFI27, IFIT1, SIGLEC1, HERC6, SLC1A2, RETN | 0,878 | 0,869 | 0,886 | 0,921 | 0,869 | 0,974 | 0,940 | 0,883 | 0,998 |
| IFIT1, IFI44L, SIGLEC1, HERC6, SLC1A2, FAM20A, RETN | 0,878 | 0,869 | 0,887 | 0,923 | 0,871 | 0,974 | 0,933 | 0,857 | 1 |
| OAS1, IFIT1, IFI44L, SIGLEC1, ISG15, OLAH, FAM20A | 0,878 | 0,868 | 0,887 | 0,905 | 0,836 | 0,974 | 0,947 | 0,891 | 1 |
| RSAD2, IFI27, OAS1, IFIT1, SIGLEC1, SLC1A2, FAM20A | 0,878 | 0,868 | 0,887 | 0,920 | 0,863 | 0,977 | 0,946 | 0,874 | 1 |
| RSAD2, IFI27, OAS1, ISG15, HERC6, SLC1A2, RETN | 0,878 | 0,868 | 0,887 | 0,915 | 0,856 | 0,975 | 0,928 | 0,850 | 1 |
| RSAD2, IFI27, SIGLEC1, HERC6, SLC1A2, FAM20A, MMP8 | 0,878 | 0,868 | 0,888 | 0,921 | 0,866 | 0,976 | 0,940 | 0,864 | 1 |
| RSAD2, IFI27, IFIT1, SIGLEC1, SLC1A2, IL1R2, RETN | 0,878 | 0,869 | 0,886 | 0,925 | 0,877 | 0,973 | 0,953 | 0,903 | 1 |
| IFI27, OAS1, IFIT1, SIGLEC1, ISG15, SLC1A2, IL1R2 | 0,878 | 0,869 | 0,886 | 0,925 | 0,880 | 0,970 | 0,950 | 0,901 | 0,999 |
| IFIT1, IFI44L, SIGLEC1, ISG15, HERC6, SLC1A2, FAM20A | 0,878 | 0,868 | 0,887 | 0,920 | 0,867 | 0,973 | 0,934 | 0,856 | 1 |
| RSAD2, SIGLEC1, ISG15, SLC1A2, OLAH, FAM20A, MMP8 | 0,877 | 0,868 | 0,887 | 0,917 | 0,863 | 0,971 | 0,951 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, SIGLEC1, HERC6, SLC1A2, IL1R2, RETN, MMP8 | 0,877 | 0,868 | 0,887 | 0,925 | 0,877 | 0,973 | 0,965 | 0,926 | 1 |
| RSAD2, IFI27, IFI44L, ISG15, HERC6, IL1R2, MMP8 | 0,877 | 0,870 | 0,885 | 0,916 | 0,866 | 0,966 | 0,936 | 0,880 | 0,992 |
| RSAD2, OAS1, IFIT1, SIGLEC1, HERC6, FAM20A, MMP8 | 0,877 | 0,868 | 0,887 | 0,911 | 0,849 | 0,972 | 0,937 | 0,865 | 1 |
| IFI27, OAS1, IFIT1, ISG15, IL1R2, RETN, MMP8 | 0,877 | 0,868 | 0,887 | 0,920 | 0,865 | 0,974 | 0,951 | 0,899 | 1 |
| IFI27, OAS1, IFI44L, SIGLEC1, HERC6, SLC1A2, FAM20A | 0,877 | 0,868 | 0,887 | 0,920 | 0,865 | 0,974 | 0,942 | 0,866 | 1 |
| RSAD2, IFI27, OAS1, IFI44L, ISG15, HERC6, IL1R2 | 0,877 | 0,869 | 0,886 | 0,922 | 0,874 | 0,969 | 0,930 | 0,871 | 0,990 |
| IFI27, OAS1, IFI44L, ISG15, SLC1A2, IL1R2, OLAH | 0,877 | 0,868 | 0,887 | 0,928 | 0,878 | 0,977 | 0,923 | 0,851 | 0,994 |
| RSAD2, IFI27, IFIT1, SIGLEC1, SLC1A2, FAM20A, MMP8 | 0,877 | 0,868 | 0,887 | 0,921 | 0,864 | 0,977 | 0,943 | 0,870 | 1 |
| IFIT1, IFI44L, SIGLEC1, HERC6, SLC1A2, FAM20A, MMP8 | 0,877 | 0,868 | 0,887 | 0,919 | 0,866 | 0,972 | 0,934 | 0,856 | 1 |
| RSAD2, IFI27, SIGLEC1, ISG15, SLC1A2, IL1R2, RETN | 0,877 | 0,869 | 0,886 | 0,924 | 0,875 | 0,973 | 0,953 | 0,904 | 1 |
| RSAD2, IFIT1, SIGLEC1, ISG15, OLAH, FAM20A, MMP8 | 0,877 | 0,868 | 0,887 | 0,908 | 0,841 | 0,974 | 0,954 | 0,902 | 1 |
| IFI27, OAS1, IFI44L, SIGLEC1, HERC6, SLC1A2, IL1R2 | 0,877 | 0,868 | 0,886 | 0,926 | 0,882 | 0,970 | 0,948 | 0,899 | 0,997 |
| RSAD2, IFIT1, SIGLEC1, SLC1A2, IL1R2, OLAH, FAM20A | 0,877 | 0,868 | 0,886 | 0,919 | 0,864 | 0,973 | 0,954 | 0,892 | 1 |
| IFI27, IFIT1, SIGLEC1, HERC6, SLC1A2, IL1R2, MMP8 | 0,877 | 0,869 | 0,886 | 0,925 | 0,879 | 0,971 | 0,951 | 0,904 | 0,998 |
| RSAD2, OAS1, IFIT1, SIGLEC1, IL1R2, FAM20A, RETN | 0,877 | 0,868 | 0,886 | 0,911 | 0,848 | 0,975 | 0,963 | 0,911 | 1 |
| RSAD2, IFI27, OAS1, IFIT1, SIGLEC1, ISG15, IL1R2 | 0,877 | 0,869 | 0,886 | 0,921 | 0,872 | 0,969 | 0,955 | 0,911 | 1,000 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, ISG15, HERC6, SLC1A2, RETN | 0,877 | 0,868 | 0,887 | 0,915 | 0,857 | 0,973 | 0,933 | 0,858 | 1 |
| RSAD2, IFIT1, SIGLEC1, IL1R2, OLAH, FAM20A, RETN | 0,877 | 0,868 | 0,886 | 0,911 | 0,848 | 0,974 | 0,964 | 0,916 | 1 |
| OAS1, IFIT1, IFI44L, SIGLEC1, HERC6, FAM20A, MMP8 | 0,877 | 0,868 | 0,887 | 0,910 | 0,848 | 0,971 | 0,936 | 0,867 | 1 |
| IFI27, OAS1, SIGLEC1, ISG15, HERC6, SLC1A2, FAM20A | 0,877 | 0,867 | 0,887 | 0,920 | 0,866 | 0,975 | 0,945 | 0,869 | 1 |
| OAS1, IFIT1, IFI44L, SIGLEC1, ISG15, HERC6, FAM20A | 0,877 | 0,868 | 0,886 | 0,913 | 0,853 | 0,973 | 0,939 | 0,872 | 1 |
| IFIT1, IFI44L, SIGLEC1, ISG15, SLC1A2, IL1R2, FAM20A | 0,877 | 0,868 | 0,886 | 0,921 | 0,869 | 0,972 | 0,948 | 0,888 | 1 |
| SIGLEC1, ISG15, HERC6, SLC1A2, OLAH, FAM20A, MMP8 | 0,877 | 0,867 | 0,887 | 0,919 | 0,865 | 0,973 | 0,947 | 0,876 | 1 |
| RSAD2, IFI27, OAS1, SIGLEC1, ISG15, IL1R2, MMP8 | 0,877 | 0,868 | 0,886 | 0,921 | 0,873 | 0,969 | 0,957 | 0,913 | 1 |
| OAS1, IFI44L, SIGLEC1, HERC6, IL1R2, OLAH, FAM20A | 0,877 | 0,868 | 0,887 | 0,914 | 0,856 | 0,973 | 0,959 | 0,905 | 1 |
| IFIT1, SIGLEC1, ISG15, IL1R2, OLAH, FAM20A, RETN | 0,877 | 0,868 | 0,886 | 0,910 | 0,848 | 0,972 | 0,960 | 0,908 | 1 |
| IFI27, IFIT1, SIGLEC1, ISG15, SLC1A2, IL1R2, MMP8 | 0,877 | 0,869 | 0,885 | 0,924 | 0,878 | 0,969 | 0,950 | 0,901 | 0,999 |
| RSAD2, IFI27, OAS1, IFIT1, SIGLEC1, SLC1A2, IL1R2 | 0,877 | 0,869 | 0,885 | 0,924 | 0,878 | 0,971 | 0,954 | 0,905 | 1 |
| RSAD2, IFI27, IFI44L, SIGLEC1, HERC6, SLC1A2, FAM20A | 0,877 | 0,867 | 0,887 | 0,921 | 0,867 | 0,976 | 0,940 | 0,864 | 1 |
| RSAD2, IFI27, IFIT1, SIGLEC1, HERC6, SLC1A2, IL1R2 | 0,877 | 0,869 | 0,885 | 0,926 | 0,880 | 0,971 | 0,950 | 0,902 | 0,998 |
| IFI27, IFIT1, IFI44L, SIGLEC1, SLC1A2, IL1R2, MMP8 | 0,877 | 0,869 | 0,885 | 0,926 | 0,880 | 0,971 | 0,951 | 0,905 | 0,997 |
| RSAD2, IFI27, IFIT1, IFI44L, ISG15, HERC6, IL1R2 | 0,877 | 0,869 | 0,885 | 0,919 | 0,868 | 0,969 | 0,935 | 0,878 | 0,992 |

(suite)

| Modèle | AUC modèle | IC 95% | | AUC Train | IC 95% Train | | AUC Test | IC 95% Test | |
|---|---|---|---|---|---|---|---|---|---|
| IFIT1, SIGLEC1, ISG15, HERC6, SLC1A2, FAM20A, RETN | 0,877 | 0,867 | 0,887 | 0,919 | 0,866 | 0,973 | 0,939 | 0,863 | 1 |
| OAS1, SIGLEC1, ISG15, SLC1A2, OLAH, FAM20A, MMP8 | 0,877 | 0,867 | 0,887 | 0,917 | 0,862 | 0,971 | 0,951 | 0,892 | 1 |
| RSAD2, IFI27, OAS1, IFI44L, HERC6, SLC1A2, RETN | 0,877 | 0,868 | 0,886 | 0,915 | 0,855 | 0,975 | 0,929 | 0,852 | 1 |
| IFI27, IFIT1, IFI44L, ISG15, HERC6, IL1R2, MMP8 | 0,877 | 0,869 | 0,885 | 0,919 | 0,869 | 0,969 | 0,936 | 0,879 | 0,993 |
| RSAD2, OAS1, IFIT1, SIGLEC1, SLC1A2, OLAH, FAM20A | 0,877 | 0,867 | 0,886 | 0,920 | 0,865 | 0,975 | 0,950 | 0,890 | 1 |
| RSAD2, IFI44L, SIGLEC1, SLC1A2, OLAH, FAM20A, MMP8 | 0,877 | 0,867 | 0,887 | 0,919 | 0,864 | 0,974 | 0,946 | 0,882 | 1 |
| RSAD2, IFI27, SIGLEC1, ISG15, HERC6, SLC1A2, FAM20A | 0,877 | 0,867 | 0,887 | 0,921 | 0,867 | 0,976 | 0,940 | 0,864 | 1 |
| IFI27, SIGLEC1, ISG15, HERC6, SLC1A2, IL1R2, RETN | 0,877 | 0,868 | 0,885 | 0,925 | 0,877 | 0,974 | 0,954 | 0,907 | 1 |
| OAS1, IFIT1, IFI44L, SIGLEC1, HERC6, SLC1A2, FAM20A | 0,877 | 0,868 | 0,886 | 0,920 | 0,868 | 0,972 | 0,932 | 0,855 | 1 |
| RSAD2, IFI27, OAS1, IFIT1, ISG15, IL1R2, RETN | 0,877 | 0,868 | 0,886 | 0,916 | 0,862 | 0,971 | 0,950 | 0,897 | 1 |
| OAS1, IFI44L, SIGLEC1, SLC1A2, OLAH, FAM20A, MMP8 | 0,877 | 0,867 | 0,887 | 0,917 | 0,861 | 0,973 | 0,948 | 0,886 | 1 |
| OAS1, SIGLEC1, ISG15, HERC6, IL1R2, OLAH, FAM20A | 0,877 | 0,867 | 0,886 | 0,913 | 0,854 | 0,972 | 0,959 | 0,905 | 1 |
| RSAD2, IFI27, IFIT1, ISG15, HERC6, IL1R2, MMP8 | 0,877 | 0,868 | 0,885 | 0,916 | 0,866 | 0,966 | 0,939 | 0,885 | 0,994 |
| RSAD2, IFI27, OAS1, SIGLEC1, ISG15, SLC1A2, IL1R2 | 0,877 | 0,868 | 0,885 | 0,925 | 0,880 | 0,971 | 0,954 | 0,905 | 1 |
| IFIT1, IFI44L, SIGLEC1, SLC1A2, IL1R2, FAM20A, RETN | 0,877 | 0,868 | 0,886 | 0,919 | 0,866 | 0,971 | 0,950 | 0,891 | 1 |
| IFIT1, SIGLEC1, SLC1A2, IL1R2, OLAH, FAM20A, RETN | 0,877 | 0,867 | 0,886 | 0,920 | 0,867 | 0,972 | 0,952 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, SIGLEC1, ISG15, HERC6, FAM20A, MMP8 | 0,877 | 0,867 | 0,886 | 0,910 | 0,849 | 0,971 | 0,941 | 0,873 | 1 |
| RSAD2, OAS1, IFIT1, IFI44L, SIGLEC1, OLAH, FAM20A | 0,877 | 0,867 | 0,886 | 0,905 | 0,835 | 0,974 | 0,948 | 0,893 | 1 |
| IFI27, IFIT1, IFI44L, SIGLEC1, HERC6, SLC1A2, RETN | 0,877 | 0,868 | 0,885 | 0,918 | 0,863 | 0,973 | 0,946 | 0,892 | 1,000 |
| RSAD2, IFI27, OAS1, IFIT1, IFI44L, IL1R2, MMP8 | 0,877 | 0,868 | 0,885 | 0,916 | 0,865 | 0,968 | 0,940 | 0,886 | 0,994 |
| IFI27, OAS1, HERC6, SLC1A2, FAM20A, RETN, MMP8 | 0,877 | 0,867 | 0,887 | 0,923 | 0,866 | 0,980 | 0,934 | 0,851 | 1 |
| OAS1, IFIT1, IFI44L, SIGLEC1, SLC1A2, IL1R2, FAM20A | 0,877 | 0,868 | 0,886 | 0,920 | 0,867 | 0,973 | 0,950 | 0,890 | 1 |
| RSAD2, IFI27, IFIT1, IFI44L, ISG15, IL1R2, RETN | 0,877 | 0,868 | 0,885 | 0,916 | 0,861 | 0,971 | 0,939 | 0,880 | 0,998 |
| RSAD2, OAS1, IFIT1, SIGLEC1, ISG15, OLAH, FAM20A | 0,877 | 0,867 | 0,886 | 0,905 | 0,836 | 0,975 | 0,952 | 0,901 | 1 |
| OAS1, IFIT1, SIGLEC1, ISG15, SLC1A2, OLAH, FAM20A | 0,877 | 0,867 | 0,886 | 0,918 | 0,863 | 0,974 | 0,945 | 0,882 | 1 |
| RSAD2, IFIT1, SIGLEC1, ISG15, HERC6, FAM20A, MMP8 | 0,877 | 0,867 | 0,886 | 0,911 | 0,850 | 0,972 | 0,936 | 0,862 | 1 |
| IFI27, IFI44L, ISG15, HERC6, SLC1A2, FAM20A, RETN | 0,876 | 0,866 | 0,886 | 0,919 | 0,862 | 0,976 | 0,941 | 0,862 | 1 |
| IFI27, OAS1, SIGLEC1, ISG15, SLC1A2, IL1R2, MMP8 | 0,876 | 0,868 | 0,885 | 0,926 | 0,881 | 0,971 | 0,950 | 0,901 | 0,999 |
| RSAD2, IFI27, IFIT1, SIGLEC1, ISG15, IL1R2, MMP8 | 0,876 | 0,868 | 0,885 | 0,919 | 0,871 | 0,968 | 0,948 | 0,899 | 0,996 |
| RSAD2, IFI27, IFI44L, HERC6, SLC1A2, FAM20A, RETN | 0,876 | 0,867 | 0,886 | 0,923 | 0,868 | 0,978 | 0,930 | 0,848 | 1 |
| RSAD2, IFI27, HERC6, SLC1A2, IL1R2, RETN, MMP8 | 0,876 | 0,868 | 0,885 | 0,924 | 0,874 | 0,974 | 0,935 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, SLC1A2, IL1R2, MMP8 | 0,876 | 0,868 | 0,885 | 0,924 | 0,878 | 0,971 | 0,953 | 0,903 | 1 |
| IFI27, IFI44L, SIGLEC1, HERC6, SLC1A2, IL1R2, MMP8 | 0,876 | 0,868 | 0,885 | 0,927 | 0,883 | 0,972 | 0,946 | 0,896 | 0,995 |
| IFI27, IFI44L, SIGLEC1, ISG15, HERC6, SLC1A2, IL1R2 | 0,876 | 0,868 | 0,885 | 0,926 | 0,881 | 0,970 | 0,943 | 0,892 | 0,995 |
| RSAD2, IFI27, SIGLEC1, ISG15, SLC1A2, IL1R2, MMP8 | 0,876 | 0,868 | 0,884 | 0,924 | 0,878 | 0,969 | 0,950 | 0,901 | 0,999 |
| IFI44L, SIGLEC1, ISG15, HERC6, IL1R2, FAM20A, RETN | 0,876 | 0,867 | 0,886 | 0,912 | 0,852 | 0,973 | 0,955 | 0,895 | 1 |
| RSAD2, IFIT1, IFI44L, SIGLEC1, HERC6, SLC1A2, FAM20A | 0,876 | 0,867 | 0,886 | 0,921 | 0,869 | 0,973 | 0,933 | 0,856 | 1 |
| RSAD2, IFI27, OAS1, IFI44L, IL1R2, RETN, MMP8 | 0,876 | 0,867 | 0,885 | 0,918 | 0,863 | 0,973 | 0,942 | 0,883 | 1 |
| IFI27, IFI44L, HERC6, SLC1A2, IL1R2, RETN, MMP8 | 0,876 | 0,867 | 0,885 | 0,924 | 0,874 | 0,973 | 0,935 | 0,868 | 1 |
| IFI44L, SIGLEC1, ISG15, SLC1A2, IL1R2, OLAH, FAM20A | 0,876 | 0,867 | 0,885 | 0,917 | 0,863 | 0,971 | 0,954 | 0,893 | 1 |
| RSAD2, IFI27, OAS1, IFIT1, HERC6, SLC1A2, RETN | 0,876 | 0,867 | 0,886 | 0,914 | 0,853 | 0,974 | 0,930 | 0,852 | 1 |
| IFIT1, IFI44L, SIGLEC1, ISG15, OLAH, FAM20A, RETN | 0,876 | 0,867 | 0,885 | 0,904 | 0,835 | 0,973 | 0,945 | 0,888 | 1 |
| IFI27, IFIT1, SIGLEC1, ISG15, HERC6, SLC1A2, IL1R2 | 0,876 | 0,868 | 0,885 | 0,926 | 0,880 | 0,971 | 0,950 | 0,902 | 0,998 |
| RSAD2, IFI27, IFIT1, HERC6, SLC1A2, FAM20A, RETN | 0,876 | 0,867 | 0,886 | 0,921 | 0,865 | 0,977 | 0,932 | 0,849 | 1 |
| IFI27, OAS1, IFIT1, IFI44L, SIGLEC1, SLC1A2, IL1R2 | 0,876 | 0,868 | 0,885 | 0,924 | 0,878 | 0,970 | 0,946 | 0,894 | 0,998 |
| RSAD2, IFIT1, SIGLEC1, ISG15, IL1R2, FAM20A, RETN | 0,876 | 0,867 | 0,885 | 0,911 | 0,848 | 0,973 | 0,964 | 0,916 | 1 |
| OAS1, IFIT1, SIGLEC1, ISG15, OLAH, FAM20A, RETN | 0,876 | 0,867 | 0,886 | 0,904 | 0,834 | 0,974 | 0,949 | 0,896 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, SLC1A2, IL1R2 | 0,876 | 0,868 | 0,885 | 0,926 | 0,879 | 0,972 | 0,952 | 0,902 | 1 |
| IFIT1, SIGLEC1, ISG15, SLC1A2, IL1R2, OLAH, FAM20A | 0,876 | 0,867 | 0,885 | 0,917 | 0,862 | 0,972 | 0,950 | 0,890 | 1 |
| RSAD2, OAS1, SIGLEC1, HERC6, IL1R2, FAM20A, RETN | 0,876 | 0,866 | 0,886 | 0,912 | 0,852 | 0,971 | 0,958 | 0,902 | 1 |
| IFI44L, SIGLEC1, HERC6, SLC1A2, OLAH, FAM20A, MMP8 | 0,876 | 0,866 | 0,886 | 0,918 | 0,862 | 0,973 | 0,950 | 0,880 | 1 |
| OAS1, IFIT1, IFI44L, SIGLEC1, OLAH, FAM20A, RETN | 0,876 | 0,867 | 0,886 | 0,905 | 0,835 | 0,974 | 0,948 | 0,893 | 1 |
| RSAD2, SIGLEC1, HERC6, SLC1A2, IL1R2, OLAH, FAM20A | 0,876 | 0,866 | 0,886 | 0,918 | 0,865 | 0,971 | 0,943 | 0,870 | 1 |
| RSAD2, IFI27, IFIT1, SIGLEC1, SLC1A2, IL1R2, MMP8 | 0,876 | 0,868 | 0,884 | 0,925 | 0,879 | 0,970 | 0,953 | 0,906 | 1 |
| IFI44L, SIGLEC1, ISG15, HERC6, SLC1A2, IL1R2, FAM20A | 0,876 | 0,866 | 0,886 | 0,919 | 0,866 | 0,972 | 0,950 | 0,878 | 1 |
| IFI44L, SIGLEC1, HERC6, SLC1A2, IL1R2, OLAH, FAM20A | 0,876 | 0,866 | 0,886 | 0,918 | 0,865 | 0,972 | 0,950 | 0,877 | 1 |
| RSAD2, IFI27, SIGLEC1, ISG15, HERC6, SLC1A2, IL1R2 | 0,876 | 0,868 | 0,884 | 0,924 | 0,878 | 0,969 | 0,949 | 0,900 | 0,998 |
| IFI44L, SIGLEC1, ISG15, IL1R2, OLAH, FAM20A, RETN | 0,876 | 0,867 | 0,885 | 0,906 | 0,842 | 0,970 | 0,963 | 0,912 | 1 |
| IFI27, OAS1, ISG15, HERC6, SLC1A2, RETN, MMP8 | 0,876 | 0,866 | 0,885 | 0,915 | 0,858 | 0,972 | 0,930 | 0,857 | 1 |
| OAS1, IFIT1, SIGLEC1, ISG15, SLC1A2, IL1R2, FAM20A | 0,876 | 0,866 | 0,885 | 0,916 | 0,861 | 0,971 | 0,951 | 0,888 | 1 |
| RSAD2, IFIT1, IFI44L, SIGLEC1, HERC6, FAM20A, MMP8 | 0,876 | 0,867 | 0,885 | 0,910 | 0,848 | 0,971 | 0,932 | 0,860 | 1 |
| IFI44L, SIGLEC1, HERC6, IL1R2, OLAH, FAM20A, RETN | 0,876 | 0,866 | 0,885 | 0,912 | 0,851 | 0,973 | 0,957 | 0,896 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, SIGLEC1, ISG15, HERC6, SLC1A2, IL1R2, FAM20A | 0,876 | 0,866 | 0,886 | 0,919 | 0,869 | 0,970 | 0,949 | 0,878 | 1 |
| RSAD2, IFI27, IFI44L, SIGLEC1, HERC6, SLC1A2, IL1R2 | 0,876 | 0,867 | 0,884 | 0,926 | 0,880 | 0,972 | 0,948 | 0,900 | 0,996 |
| IFI27, IFIT1, IFI44L, ISG15, HERC6, FAM20A, MMP8 | 0,876 | 0,866 | 0,886 | 0,910 | 0,849 | 0,971 | 0,925 | 0,843 | 1 |
| IFI27, IFIT1, IFI44L, SIGLEC1, HERC6, SLC1A2, IL1R2 | 0,876 | 0,867 | 0,884 | 0,926 | 0,881 | 0,971 | 0,947 | 0,897 | 0,996 |
| RSAD2, IFI27, OAS1, IFIT1, IFI44L, SIGLEC1, RETN | 0,876 | 0,867 | 0,885 | 0,907 | 0,844 | 0,970 | 0,952 | 0,889 | 1 |
| IFIT1, SIGLEC1, ISG15, OLAH, FAM20A, RETN, MMP8 | 0,876 | 0,866 | 0,886 | 0,912 | 0,849 | 0,974 | 0,958 | 0,909 | 1 |
| RSAD2, IFI27, IFIT1, HERC6, SLC1A2, IL1R2, RETN | 0,876 | 0,867 | 0,884 | 0,922 | 0,872 | 0,972 | 0,936 | 0,872 | 1,000 |
| SIGLEC1, ISG15, SLC1A2, OLAH, FAM20A, RETN, MMP8 | 0,876 | 0,866 | 0,886 | 0,915 | 0,858 | 0,972 | 0,949 | 0,882 | 1 |
| IFI27, OAS1, IFIT1, IFI44L, SIGLEC1, ISG15, RETN | 0,876 | 0,867 | 0,885 | 0,909 | 0,848 | 0,970 | 0,955 | 0,897 | 1 |
| OAS1, SIGLEC1, HERC6, SLC1A2, IL1R2, OLAH, FAM20A | 0,876 | 0,865 | 0,886 | 0,920 | 0,869 | 0,971 | 0,946 | 0,878 | 1 |
| OAS1, IFIT1, SIGLEC1, HERC6, SLC1A2, FAM20A, RETN | 0,876 | 0,866 | 0,885 | 0,922 | 0,870 | 0,973 | 0,934 | 0,859 | 1 |
| IFI27, IFIT1, HERC6, SLC1A2, IL1R2, RETN, MMP8 | 0,876 | 0,867 | 0,884 | 0,925 | 0,875 | 0,974 | 0,943 | 0,885 | 1 |
| IFI27, SIGLEC1, ISG15, HERC6, SLC1A2, IL1R2, MMP8 | 0,876 | 0,867 | 0,884 | 0,924 | 0,879 | 0,970 | 0,950 | 0,901 | 0,999 |
| OAS1, IFIT1, SIGLEC1, SLC1A2, OLAH, FAM20A, RETN | 0,876 | 0,866 | 0,885 | 0,918 | 0,861 | 0,974 | 0,945 | 0,882 | 1 |
| RSAD2, IFIT1, IFI44L, SIGLEC1, IL1R2, FAM20A, RETN | 0,876 | 0,866 | 0,885 | 0,910 | 0,846 | 0,973 | 0,963 | 0,913 | 1 |
| RSAD2, IFIT1, SIGLEC1, ISG15, SLC1A2, IL1R2, FAM20A | 0,876 | 0,867 | 0,885 | 0,918 | 0,865 | 0,972 | 0,955 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, SLC1A2, IL1R2, OLAH, FAM20A, RETN | 0,876 | 0,866 | 0,885 | 0,916 | 0,863 | 0,970 | 0,957 | 0,896 | 1 |
| IFI27, OAS1, IFI44L, SIGLEC1, SLC1A2, IL1R2, MMP8 | 0,876 | 0,867 | 0,884 | 0,924 | 0,878 | 0,970 | 0,945 | 0,893 | 0,996 |
| RSAD2, IFIT1, IFI44L, SIGLEC1, SLC1A2, IL1R2, FAM20A | 0,875 | 0,866 | 0,884 | 0,919 | 0,865 | 0,972 | 0,952 | 0,893 | 1 |
| RSAD2, IFI27, OAS1, IFI44L, ISG15, IL1R2, RETN | 0,875 | 0,867 | 0,884 | 0,918 | 0,864 | 0,971 | 0,941 | 0,883 | 1,000 |
| RSAD2, OAS1, SIGLEC1, ISG15, IL1R2, OLAH, FAM20A | 0,875 | 0,866 | 0,885 | 0,909 | 0,846 | 0,973 | 0,964 | 0,914 | 1 |
| RSAD2, IFIT1, SIGLEC1, ISG15, SLC1A2, OLAH, FAM20A | 0,875 | 0,866 | 0,885 | 0,919 | 0,863 | 0,974 | 0,946 | 0,883 | 1 |
| IFI27, OAS1, IFIT1, SLC1A2, IL1R2, RETN, MMP8 | 0,875 | 0,867 | 0,884 | 0,922 | 0,871 | 0,972 | 0,945 | 0,884 | 1 |
| RSAD2, IFI27, IFI44L, HERC6, SLC1A2, IL1R2, RETN | 0,875 | 0,867 | 0,884 | 0,924 | 0,874 | 0,973 | 0,933 | 0,866 | 0,999 |
| RSAD2, IFI27, IFI44L, SIGLEC1, HERC6, SLC1A2, RETN | 0,875 | 0,867 | 0,884 | 0,921 | 0,868 | 0,975 | 0,939 | 0,880 | 0,998 |
| RSAD2, SIGLEC1, ISG15, HERC6, IL1R2, FAM20A, RETN | 0,875 | 0,866 | 0,885 | 0,914 | 0,855 | 0,973 | 0,955 | 0,894 | 1 |
| RSAD2, IFI27, ISG15, HERC6, SLC1A2, IL1R2, RETN | 0,875 | 0,867 | 0,884 | 0,923 | 0,873 | 0,973 | 0,936 | 0,872 | 1,000 |
| IFI27, OAS1, IFI44L, SIGLEC1, ISG15, SLC1A2, IL1R2 | 0,875 | 0,867 | 0,884 | 0,924 | 0,878 | 0,970 | 0,946 | 0,895 | 0,996 |
| IFI44L, SIGLEC1, SLC1A2, OLAH, FAM20A, RETN, MMP8 | 0,875 | 0,866 | 0,885 | 0,916 | 0,858 | 0,974 | 0,949 | 0,880 | 1 |
| RSAD2, IFI27, IFIT1, IFI44L, HERC6, FAM20A, MMP8 | 0,875 | 0,866 | 0,885 | 0,913 | 0,853 | 0,973 | 0,921 | 0,838 | 1 |
| IFI27, OAS1, IFIT1, HERC6, SLC1A2, IL1R2, MMP8 | 0,875 | 0,866 | 0,884 | 0,921 | 0,875 | 0,967 | 0,940 | 0,879 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, HERC6, OLAH, FAM20A, MMP8 | 0,875 | 0,865 | 0,885 | 0,910 | 0,850 | 0,971 | 0,954 | 0,899 | 1 |
| RSAD2, IFI27, IFIT1, ISG15, IL1R2, RETN, MMP8 | 0,875 | 0,866 | 0,884 | 0,920 | 0,865 | 0,974 | 0,951 | 0,901 | 1 |
| RSAD2, OAS1, IFI44L, SIGLEC1, IL1R2, OLAH, FAM20A | 0,875 | 0,866 | 0,885 | 0,911 | 0,847 | 0,974 | 0,963 | 0,912 | 1 |
| RSAD2, OAS1, IFIT1, SIGLEC1, SLC1A2, IL1R2, FAM20A | 0,875 | 0,866 | 0,884 | 0,918 | 0,863 | 0,972 | 0,955 | 0,893 | 1 |
| RSAD2, SIGLEC1, ISG15, HERC6, SLC1A2, IL1R2, FAM20A | 0,875 | 0,865 | 0,885 | 0,918 | 0,866 | 0,970 | 0,948 | 0,875 | 1 |
| IFI44L, SIGLEC1, ISG15, SLC1A2, IL1R2, FAM20A, RETN | 0,875 | 0,866 | 0,884 | 0,915 | 0,862 | 0,968 | 0,953 | 0,885 | 1 |
| RSAD2, IFI27, OAS1, IFI44L, SIGLEC1, SLC1A2, FAM20A | 0,875 | 0,865 | 0,885 | 0,918 | 0,859 | 0,976 | 0,947 | 0,875 | 1 |
| RSAD2, IFIT1, SIGLEC1, HERC6, SLC1A2, FAM20A, RETN | 0,875 | 0,866 | 0,885 | 0,919 | 0,865 | 0,972 | 0,936 | 0,859 | 1 |
| RSAD2, IFIT1, IFI44L, SIGLEC1, ISG15, OLAH, FAM20A | 0,875 | 0,866 | 0,885 | 0,905 | 0,836 | 0,974 | 0,947 | 0,892 | 1 |
| RSAD2, IFI27, HERC6, SLC1A2, FAM20A, RETN, MMP8 | 0,875 | 0,865 | 0,885 | 0,923 | 0,868 | 0,979 | 0,935 | 0,853 | 1 |
| IFI27, OAS1, IFI44L, ISG15, IL1R2, RETN, MMP8 | 0,875 | 0,867 | 0,884 | 0,920 | 0,867 | 0,973 | 0,941 | 0,883 | 1,000 |
| IFI27, IFIT1, SIGLEC1, ISG15, HERC6, SLC1A2, RETN | 0,875 | 0,866 | 0,884 | 0,919 | 0,865 | 0,974 | 0,949 | 0,896 | 1 |
| OAS1, IFIT1, SIGLEC1, SLC1A2, IL1R2, FAM20A, RETN | 0,875 | 0,866 | 0,884 | 0,919 | 0,866 | 0,972 | 0,954 | 0,892 | 1 |
| IFI27, OAS1, IFIT1, IFI44L, HERC6, SLC1A2, RETN | 0,875 | 0,866 | 0,885 | 0,914 | 0,854 | 0,973 | 0,935 | 0,863 | 1 |
| IFI27, IFIT1, IFI44L, ISG15, HERC6, RETN, MMP8 | 0,875 | 0,866 | 0,885 | 0,910 | 0,848 | 0,971 | 0,936 | 0,871 | 1 |
| IFI27, IFI44L, ISG15, HERC6, SLC1A2, IL1R2, RETN | 0,875 | 0,867 | 0,884 | 0,923 | 0,874 | 0,973 | 0,933 | 0,866 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, HERC6, SLC1A2, RETN, MMP8 | 0,875 | 0,866 | 0,884 | 0,920 | 0,866 | 0,974 | 0,950 | 0,896 | 1 |
| RSAD2, IFI27, SIGLEC1, ISG15, HERC6, SLC1A2, RETN | 0,875 | 0,866 | 0,884 | 0,919 | 0,865 | 0,974 | 0,945 | 0,887 | 1 |
| RSAD2, OAS1, SIGLEC1, IL1R2, OLAH, FAM20A, RETN | 0,875 | 0,865 | 0,885 | 0,907 | 0,843 | 0,971 | 0,961 | 0,906 | 1 |
| RSAD2, IFI27, IFI44L, ISG15, HERC6, RETN, MMP8 | 0,875 | 0,866 | 0,884 | 0,914 | 0,855 | 0,973 | 0,940 | 0,883 | 0,998 |
| RSAD2, IFI27, IFI44L, SIGLEC1, SLC1A2, IL1R2, MMP8 | 0,875 | 0,867 | 0,883 | 0,925 | 0,879 | 0,972 | 0,951 | 0,905 | 0,997 |
| RSAD2, SIGLEC1, ISG15, HERC6, FAM20A, RETN, MMP8 | 0,875 | 0,866 | 0,884 | 0,913 | 0,853 | 0,974 | 0,951 | 0,888 | 1 |
| OAS1, IFI44L, SIGLEC1, HERC6, IL1R2, FAM20A, RETN | 0,875 | 0,866 | 0,884 | 0,914 | 0,854 | 0,973 | 0,961 | 0,908 | 1 |
| OAS1, IFI44L, SIGLEC1, ISG15, IL1R2, OLAH, FAM20A | 0,875 | 0,866 | 0,885 | 0,910 | 0,846 | 0,973 | 0,962 | 0,911 | 1 |
| IFI27, IFIT1, IFI44L, HERC6, SLC1A2, IL1R2, RETN | 0,875 | 0,866 | 0,884 | 0,923 | 0,873 | 0,973 | 0,933 | 0,866 | 0,999 |
| OAS1, IFI44L, SIGLEC1, HERC6, SLC1A2, IL1R2, FAM20A | 0,875 | 0,865 | 0,885 | 0,919 | 0,869 | 0,970 | 0,949 | 0,878 | 1 |
| RSAD2, IFI27, OAS1, IFIT1, SIGLEC1, ISG15, RETN | 0,875 | 0,866 | 0,884 | 0,909 | 0,847 | 0,971 | 0,952 | 0,892 | 1 |
| IFIT1, IFI44L, SIGLEC1, ISG15, FAM20A, RETN, MMP8 | 0,875 | 0,865 | 0,885 | 0,908 | 0,841 | 0,975 | 0,943 | 0,881 | 1 |
| RSAD2, OAS1, SIGLEC1, HERC6, SLC1A2, IL1R2, FAM20A | 0,875 | 0,865 | 0,885 | 0,919 | 0,868 | 0,969 | 0,951 | 0,881 | 1 |
| RSAD2, IFI27, IFIT1, SIGLEC1, ISG15, SLC1A2, IL1R2 | 0,875 | 0,867 | 0,883 | 0,924 | 0,878 | 0,969 | 0,949 | 0,900 | 0,998 |
| RSAD2, IFI27, OAS1, IFIT1, HERC6, SLC1A2, IL1R2 | 0,875 | 0,866 | 0,884 | 0,922 | 0,875 | 0,968 | 0,938 | 0,875 | 1 |
| IFI27, IFIT1, HERC6, SLC1A2, FAM20A, RETN, MMP8 | 0,875 | 0,865 | 0,885 | 0,920 | 0,863 | 0,977 | 0,936 | 0,855 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, HERC6, SLC1A2, FAM20A | 0,875 | 0,866 | 0,884 | 0,918 | 0,864 | 0,971 | 0,938 | 0,862 | 1 |
| IFI27, IFIT1, SIGLEC1, HERC6, SLC1A2, RETN, MMP8 | 0,875 | 0,866 | 0,884 | 0,919 | 0,865 | 0,974 | 0,940 | 0,880 | 1 |
| IFIT1, SIGLEC1, ISG15, HERC6, SLC1A2, FAM20A, MMP8 | 0,875 | 0,865 | 0,885 | 0,918 | 0,864 | 0,972 | 0,940 | 0,864 | 1 |
| RSAD2, IFI27, IFI44L, SIGLEC1, ISG15, SLC1A2, IL1R2 | 0,875 | 0,867 | 0,883 | 0,925 | 0,879 | 0,971 | 0,950 | 0,903 | 0,997 |
| RSAD2, OAS1, IFIT1, SIGLEC1, OLAH, FAM20A, RETN | 0,875 | 0,865 | 0,885 | 0,905 | 0,836 | 0,974 | 0,955 | 0,906 | 1 |
| RSAD2, SIGLEC1, HERC6, OLAH, FAM20A, RETN, MMP8 | 0,875 | 0,865 | 0,884 | 0,916 | 0,856 | 0,976 | 0,951 | 0,893 | 1 |
| RSAD2, IFI27, IFIT1, ISG15, HERC6, RETN, MMP8 | 0,875 | 0,865 | 0,884 | 0,914 | 0,855 | 0,973 | 0,937 | 0,877 | 0,997 |
| OAS1, SIGLEC1, ISG15, HERC6, IL1R2, FAM20A, RETN | 0,875 | 0,865 | 0,885 | 0,912 | 0,853 | 0,972 | 0,961 | 0,908 | 1 |
| RSAD2, IFI27, IFI44L, ISG15, IL1R2, RETN, MMP8 | 0,875 | 0,866 | 0,883 | 0,919 | 0,865 | 0,973 | 0,943 | 0,886 | 1 |
| RSAD2, IFI27, SIGLEC1, ISG15, SLC1A2, FAM20A, MMP8 | 0,875 | 0,865 | 0,885 | 0,917 | 0,858 | 0,976 | 0,946 | 0,872 | 1 |
| RSAD2, IFI27, OAS1, IFIT1, SLC1A2, IL1R2, RETN | 0,875 | 0,866 | 0,883 | 0,919 | 0,867 | 0,971 | 0,937 | 0,868 | 1 |
| RSAD2, IFIT1, SIGLEC1, HERC6, SLC1A2, FAM20A, MMP8 | 0,875 | 0,865 | 0,884 | 0,916 | 0,861 | 0,971 | 0,938 | 0,861 | 1 |
| IFI27, OAS1, IFIT1, IFI44L, HERC6, FAM20A, MMP8 | 0,875 | 0,865 | 0,885 | 0,912 | 0,853 | 0,971 | 0,920 | 0,837 | 1 |
| SIGLEC1, ISG15, SLC1A2, IL1R2, OLAH, FAM20A, RETN | 0,875 | 0,865 | 0,884 | 0,918 | 0,865 | 0,970 | 0,955 | 0,894 | 1 |
| RSAD2, IFI27, OAS1, SIGLEC1, ISG15, SLC1A2, FAM20A | 0,875 | 0,865 | 0,885 | 0,916 | 0,858 | 0,975 | 0,947 | 0,874 | 1 |
| RSAD2, OAS1, IFIT1, SIGLEC1, HERC6, SLC1A2, FAM20A | 0,875 | 0,865 | 0,884 | 0,916 | 0,862 | 0,970 | 0,936 | 0,859 | 1 |

**EP 4 365 308 A1**

(suite)

| Modèle | AUC modèle | IC 95% | | AUC Train | IC 95% Train | | AUC Test | IC 95% Test | |
|---|---|---|---|---|---|---|---|---|---|
| IFI27, ISG15, HERC6, SLC1A2, IL1R2, RETN, MMP8 | 0,875 | 0,866 | 0,884 | 0,925 | 0,876 | 0,975 | 0,936 | 0,870 | 1 |
| SIGLEC1, HERC6, SLC1A2, OLAH, FAM20A, RETN, MMP8 | 0,875 | 0,864 | 0,885 | 0,917 | 0,860 | 0,974 | 0,949 | 0,877 | 1 |
| RSAD2, IFIT1, SIGLEC1, SLC1A2, IL1R2, FAM20A, RETN | 0,875 | 0,865 | 0,884 | 0,920 | 0,868 | 0,972 | 0,957 | 0,895 | 1 |
| RSAD2, SIGLEC1, HERC6, IL1R2, OLAH, FAM20A, RETN | 0,875 | 0,865 | 0,885 | 0,912 | 0,852 | 0,972 | 0,955 | 0,897 | 1 |
| RSAD2, SIGLEC1, SLC1A2, IL1R2, OLAH, FAM20A, RETN | 0,875 | 0,865 | 0,884 | 0,916 | 0,862 | 0,970 | 0,955 | 0,894 | 1 |
| RSAD2, IFIT1, SIGLEC1, OLAH, FAM20A, RETN, MMP8 | 0,875 | 0,865 | 0,884 | 0,913 | 0,849 | 0,976 | 0,960 | 0,913 | 1 |
| RSAD2, IFI27, OAS1, HERC6, SLC1A2, RETN, MMP8 | 0,875 | 0,865 | 0,884 | 0,913 | 0,853 | 0,974 | 0,927 | 0,848 | 1 |
| IFI27, IFI44L, SIGLEC1, ISG15, SLC1A2, FAM20A, MMP8 | 0,875 | 0,865 | 0,884 | 0,915 | 0,856 | 0,974 | 0,943 | 0,866 | 1 |
| OAS1, IFIT1, SIGLEC1, HERC6, SLC1A2, FAM20A, MMP8 | 0,875 | 0,865 | 0,884 | 0,915 | 0,860 | 0,970 | 0,937 | 0,860 | 1 |
| RSAD2, IFI27, OAS1, SIGLEC1, SLC1A2, FAM20A, MMP8 | 0,875 | 0,864 | 0,885 | 0,919 | 0,860 | 0,977 | 0,949 | 0,878 | 1 |
| OAS1, IFIT1, IFI44L, SIGLEC1, FAM20A, RETN, MMP8 | 0,875 | 0,865 | 0,884 | 0,909 | 0,842 | 0,975 | 0,946 | 0,884 | 1 |
| RSAD2, IFI27, OAS1, IFI44L, SIGLEC1, ISG15, RETN | 0,875 | 0,865 | 0,884 | 0,909 | 0,846 | 0,971 | 0,953 | 0,891 | 1 |
| RSAD2, IFI27, OAS1, ISG15, IL1R2, RETN, MMP8 | 0,875 | 0,865 | 0,884 | 0,918 | 0,863 | 0,973 | 0,942 | 0,883 | 1 |
| RSAD2, IFI27, IFI44L, SIGLEC1, ISG15, SLC1A2, FAM20A | 0,874 | 0,865 | 0,884 | 0,918 | 0,860 | 0,976 | 0,947 | 0,874 | 1 |
| IFI27, OAS1, IFIT1, ISG15, HERC6, SLC1A2, IL1R2 | 0,874 | 0,865 | 0,883 | 0,922 | 0,876 | 0,968 | 0,937 | 0,874 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, SLC1A2, FAM20A, MMP8 | 0,874 | 0,864 | 0,884 | 0,918 | 0,859 | 0,978 | 0,947 | 0,873 | 1 |
| SIGLEC1, ISG15, HERC6, IL1R2, OLAH, FAM20A, RETN | 0,874 | 0,865 | 0,884 | 0,908 | 0,845 | 0,971 | 0,960 | 0,901 | 1 |
| RSAD2, IFI27, OAS1, IFI44L, ISG15, FAM20A, RETN | 0,874 | 0,864 | 0,884 | 0,910 | 0,846 | 0,974 | 0,945 | 0,869 | 1 |
| RSAD2, IFIT1, SIGLEC1, SLC1A2, OLAH, FAM20A, RETN | 0,874 | 0,865 | 0,884 | 0,919 | 0,863 | 0,975 | 0,950 | 0,890 | 1 |
| IFI27, OAS1, IFIT1, SIGLEC1, ISG15, SLC1A2, RETN | 0,874 | 0,866 | 0,883 | 0,915 | 0,859 | 0,971 | 0,947 | 0,881 | 1 |
| IFI27, OAS1, SIGLEC1, ISG15, SLC1A2, FAM20A, MMP8 | 0,874 | 0,864 | 0,885 | 0,916 | 0,857 | 0,975 | 0,946 | 0,872 | 1 |
| RSAD2, IFI27, IFIT1, IFI44L, ISG15, HERC6, FAM20A | 0,874 | 0,864 | 0,884 | 0,911 | 0,849 | 0,973 | 0,921 | 0,837 | 1 |
| IFI27, IFI44L, SIGLEC1, ISG15, HERC6, SLC1A2, FAM20A | 0,874 | 0,865 | 0,884 | 0,916 | 0,859 | 0,973 | 0,943 | 0,866 | 1 |
| OAS1, IFIT1, SIGLEC1, ISG15, HERC6, SLC1A2, FAM20A | 0,874 | 0,865 | 0,884 | 0,918 | 0,865 | 0,971 | 0,938 | 0,862 | 1 |
| OAS1, SIGLEC1, SLC1A2, OLAH, FAM20A, RETN, MMP8 | 0,874 | 0,864 | 0,884 | 0,915 | 0,859 | 0,972 | 0,951 | 0,891 | 1 |
| SIGLEC1, ISG15, HERC6, SLC1A2, IL1R2, OLAH, FAM20A | 0,874 | 0,864 | 0,884 | 0,917 | 0,863 | 0,971 | 0,948 | 0,875 | 1 |
| IFI27, IFI44L, ISG15, SLC1A2, IL1R2, RETN, MMP8 | 0,874 | 0,866 | 0,883 | 0,923 | 0,874 | 0,972 | 0,936 | 0,869 | 1 |
| RSAD2, IFI27, OAS1, IFIT1, IFI44L, ISG15, IL1R2 | 0,874 | 0,866 | 0,883 | 0,916 | 0,864 | 0,969 | 0,941 | 0,888 | 0,995 |
| IFI27, OAS1, IFI44L, SIGLEC1, ISG15, SLC1A2, FAM20A | 0,874 | 0,864 | 0,884 | 0,916 | 0,858 | 0,974 | 0,947 | 0,873 | 1 |
| RSAD2, IFI44L, SIGLEC1, HERC6, SLC1A2, IL1R2, FAM20A | 0,874 | 0,864 | 0,884 | 0,919 | 0,867 | 0,971 | 0,948 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, HERC6, OLAH, FAM20A, RETN, MMP8 | 0,874 | 0,864 | 0,884 | 0,911 | 0,846 | 0,976 | 0,958 | 0,899 | 1 |
| IFIT1, SIGLEC1, ISG15, SLC1A2, IL1R2, FAM20A, RETN | 0,874 | 0,865 | 0,883 | 0,920 | 0,869 | 0,972 | 0,955 | 0,898 | 1 |
| RSAD2, IFI27, IFIT1, IFI44L, HERC6, RETN, MMP8 | 0,874 | 0,865 | 0,884 | 0,911 | 0,850 | 0,972 | 0,933 | 0,868 | 0,997 |
| OAS1, IFIT1, SIGLEC1, ISG15, FAM20A, RETN, MMP8 | 0,874 | 0,864 | 0,884 | 0,907 | 0,840 | 0,974 | 0,950 | 0,886 | 1 |
| IFI27, IFIT1, IFI44L, SLC1A2, IL1R2, RETN, MMP8 | 0,874 | 0,865 | 0,883 | 0,922 | 0,870 | 0,973 | 0,941 | 0,881 | 1 |
| IFI27, OAS1, IFIT1, ISG15, SLC1A2, IL1R2, RETN | 0,874 | 0,865 | 0,883 | 0,920 | 0,868 | 0,971 | 0,941 | 0,878 | 1 |
| RSAD2, OAS1, SIGLEC1, HERC6, FAM20A, RETN, MMP8 | 0,874 | 0,865 | 0,884 | 0,910 | 0,847 | 0,973 | 0,948 | 0,881 | 1 |
| RSAD2, IFI27, IFIT1, SLC1A2, IL1R2, RETN, MMP8 | 0,874 | 0,865 | 0,883 | 0,922 | 0,871 | 0,973 | 0,947 | 0,890 | 1 |
| RSAD2, IFI27, IFI44L, ISG15, HERC6, FAM20A, MMP8 | 0,874 | 0,864 | 0,885 | 0,913 | 0,852 | 0,974 | 0,925 | 0,844 | 1 |
| RSAD2, IFI27, IFIT1, IFI44L, SIGLEC1, SLC1A2, IL1R2 | 0,874 | 0,866 | 0,882 | 0,925 | 0,878 | 0,971 | 0,951 | 0,905 | 0,997 |
| RSAD2, IFI44L, SIGLEC1, ISG15, IL1R2, OLAH, FAM20A | 0,874 | 0,864 | 0,883 | 0,910 | 0,846 | 0,973 | 0,963 | 0,912 | 1 |
| RSAD2, OAS1, SIGLEC1, SLC1A2, IL1R2, OLAH, FAM20A | 0,874 | 0,864 | 0,884 | 0,916 | 0,861 | 0,971 | 0,957 | 0,897 | 1 |
| IFI27, OAS1, IFIT1, HERC6, SLC1A2, RETN, MMP8 | 0,874 | 0,864 | 0,883 | 0,913 | 0,854 | 0,972 | 0,930 | 0,857 | 1 |
| IFIT1, SIGLEC1, ISG15, SLC1A2, OLAH, FAM20A, RETN | 0,874 | 0,864 | 0,883 | 0,919 | 0,863 | 0,974 | 0,942 | 0,881 | 1 |
| RSAD2, IFI44L, SIGLEC1, HERC6, IL1R2, FAM20A, RETN | 0,874 | 0,864 | 0,884 | 0,914 | 0,855 | 0,973 | 0,954 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, HERC6, SLC1A2, RETN, MMP8 | 0,874 | 0,865 | 0,883 | 0,914 | 0,855 | 0,972 | 0,929 | 0,855 | 1 |
| RSAD2, IFI27, OAS1, HERC6, SLC1A2, IL1R2, MMP8 | 0,874 | 0,865 | 0,883 | 0,922 | 0,876 | 0,969 | 0,932 | 0,865 | 0,998 |
| RSAD2, SIGLEC1, SLC1A2, OLAH, FAM20A, RETN, MMP8 | 0,874 | 0,864 | 0,884 | 0,917 | 0,862 | 0,972 | 0,951 | 0,891 | 1 |
| IFI27, IFI44L, HERC6, SLC1A2, FAM20A, RETN, MMP8 | 0,874 | 0,863 | 0,884 | 0,920 | 0,862 | 0,978 | 0,942 | 0,863 | 1 |
| OAS1, SIGLEC1, HERC6, IL1R2, OLAH, FAM20A, RETN | 0,874 | 0,864 | 0,884 | 0,911 | 0,851 | 0,971 | 0,960 | 0,907 | 1 |
| RSAD2, IFI27, IFIT1, IFI44L, ISG15, IL1R2, MMP8 | 0,874 | 0,865 | 0,882 | 0,916 | 0,864 | 0,969 | 0,937 | 0,881 | 0,993 |
| SIGLEC1, ISG15, HERC6, OLAH, FAM20A, RETN, MMP8 | 0,874 | 0,863 | 0,884 | 0,907 | 0,840 | 0,975 | 0,962 | 0,910 | 1 |
| IFI27, ISG15, HERC6, SLC1A2, FAM20A, RETN, MMP8 | 0,874 | 0,863 | 0,884 | 0,919 | 0,861 | 0,977 | 0,940 | 0,860 | 1 |
| RSAD2, IFI27, OAS1, IFI44L, ISG15, HERC6, FAM20A | 0,874 | 0,863 | 0,884 | 0,913 | 0,851 | 0,974 | 0,926 | 0,845 | 1 |
| IFI27, OAS1, IFIT1, IFI44L, SLC1A2, IL1R2, RETN | 0,874 | 0,865 | 0,882 | 0,920 | 0,868 | 0,972 | 0,940 | 0,877 | 1 |
| IFI27, IFIT1, ISG15, HERC6, SLC1A2, IL1R2, RETN | 0,874 | 0,865 | 0,882 | 0,922 | 0,872 | 0,972 | 0,936 | 0,872 | 1,000 |
| OAS1, SIGLEC1, HERC6, OLAH, FAM20A, RETN, MMP8 | 0,874 | 0,864 | 0,884 | 0,913 | 0,851 | 0,974 | 0,961 | 0,911 | 1 |
| IFI27, OAS1, IFIT1, IFI44L, SIGLEC1, SLC1A2, RETN | 0,874 | 0,865 | 0,882 | 0,914 | 0,858 | 0,971 | 0,949 | 0,883 | 1 |
| RSAD2, SIGLEC1, HERC6, SLC1A2, IL1R2, FAM20A, RETN | 0,874 | 0,863 | 0,884 | 0,919 | 0,868 | 0,970 | 0,946 | 0,873 | 1 |
| IFI27, OAS1, IFI44L, SIGLEC1, SLC1A2, FAM20A, MMP8 | 0,874 | 0,864 | 0,884 | 0,918 | 0,859 | 0,977 | 0,946 | 0,872 | 1 |
| IFI44L, SIGLEC1, ISG15, HERC6, OLAH, FAM20A, MMP8 | 0,874 | 0,863 | 0,884 | 0,905 | 0,840 | 0,971 | 0,959 | 0,904 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, SLC1A2, RETN | 0,874 | 0,865 | 0,883 | 0,917 | 0,860 | 0,973 | 0,943 | 0,885 | 1 |
| OAS1, SIGLEC1, ISG15, IL1R2, OLAH, FAM20A, RETN | 0,874 | 0,864 | 0,883 | 0,906 | 0,842 | 0,971 | 0,961 | 0,904 | 1 |
| OAS1, SIGLEC1, ISG15, HERC6, FAM20A, RETN, MMP8 | 0,874 | 0,864 | 0,883 | 0,911 | 0,848 | 0,974 | 0,952 | 0,889 | 1 |
| IFI27, IFIT1, IFI44L, ISG15, SLC1A2, IL1R2, RETN | 0,874 | 0,865 | 0,882 | 0,919 | 0,867 | 0,971 | 0,938 | 0,875 | 1 |
| OAS1, SIGLEC1, ISG15, SLC1A2, IL1R2, OLAH, FAM20A | 0,874 | 0,864 | 0,883 | 0,916 | 0,861 | 0,970 | 0,954 | 0,893 | 1 |
| OAS1, IFI44L, SIGLEC1, IL1R2, OLAH, FAM20A, RETN | 0,874 | 0,864 | 0,883 | 0,905 | 0,840 | 0,970 | 0,961 | 0,906 | 1 |
| RSAD2, SIGLEC1, ISG15, SLC1A2, IL1R2, OLAH, FAM20A | 0,873 | 0,864 | 0,883 | 0,915 | 0,860 | 0,970 | 0,955 | 0,894 | 1 |
| IFI27, IFI44L, SIGLEC1, HERC6, SLC1A2, FAM20A, MMP8 | 0,873 | 0,864 | 0,883 | 0,913 | 0,855 | 0,972 | 0,945 | 0,866 | 1 |
| OAS1, SIGLEC1, SLC1A2, IL1R2, OLAH, FAM20A, RETN | 0,873 | 0,864 | 0,883 | 0,916 | 0,862 | 0,970 | 0,957 | 0,897 | 1 |
| IFI27, OAS1, IFIT1, IFI44L, ISG15, IL1R2, MMP8 | 0,873 | 0,865 | 0,882 | 0,917 | 0,865 | 0,969 | 0,937 | 0,881 | 0,993 |
| RSAD2, IFI27, OAS1, IFIT1, ISG15, IL1R2, MMP8 | 0,873 | 0,865 | 0,882 | 0,914 | 0,862 | 0,965 | 0,945 | 0,892 | 0,997 |
| IFI27, OAS1, IFIT1, SIGLEC1, ISG15, RETN, MMP8 | 0,873 | 0,864 | 0,883 | 0,909 | 0,848 | 0,970 | 0,955 | 0,897 | 1 |
| IFI27, IFIT1, ISG15, SLC1A2, IL1R2, RETN, MMP8 | 0,873 | 0,864 | 0,882 | 0,922 | 0,871 | 0,973 | 0,946 | 0,887 | 1 |
| IFI27, OAS1, IFIT1, IFI44L, SIGLEC1, RETN, MMP8 | 0,873 | 0,864 | 0,883 | 0,911 | 0,850 | 0,972 | 0,954 | 0,896 | 1 |
| RSAD2, SIGLEC1, ISG15, IL1R2, OLAH, FAM20A, RETN | 0,873 | 0,864 | 0,883 | 0,906 | 0,841 | 0,970 | 0,963 | 0,910 | 1 |
| IFI44L, ISG15, SLC1A2, IL1R2, OLAH, FAM20A, MMP8 | 0,873 | 0,865 | 0,882 | 0,917 | 0,865 | 0,969 | 0,925 | 0,841 | 1 |
| RSAD2, IFI27, OAS1, IFIT1, SIGLEC1, SLC1A2, RETN | 0,873 | 0,865 | 0,882 | 0,915 | 0,856 | 0,973 | 0,949 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, SLC1A2, RETN, MMP8 | 0,873 | 0,864 | 0,883 | 0,916 | 0,860 | 0,972 | 0,949 | 0,883 | 1 |
| RSAD2,IFI27,OAS1,IFIT1, IFI44L, FAM20A, RETN | 0,873 | 0,863 | 0,883 | 0,912 | 0,850 | 0,975 | 0,945 | 0,869 | 1 |
| RSAD2, IFI27, IFIT1, IFI44L, ISG15, FAM20A, RETN | 0,873 | 0,863 | 0,883 | 0,911 | 0,847 | 0,974 | 0,942 | 0,865 | 1 |
| RSAD2, SIGLEC1, ISG15, HERC6, OLAH, FAM20A, MMP8 | 0,873 | 0,863 | 0,883 | 0,910 | 0,850 | 0,970 | 0,953 | 0,898 | 1 |
| RSAD2, OAS1, SIGLEC1, ISG15, IL1R2, FAM20A, RETN | 0,873 | 0,863 | 0,883 | 0,907 | 0,843 | 0,971 | 0,961 | 0,904 | 1 |
| OAS1, SIGLEC1, HERC6, SLC1A2, IL1R2, FAM20A, RETN | 0,873 | 0,863 | 0,883 | 0,920 | 0,869 | 0,970 | 0,951 | 0,883 | 1 |
| IFI27, OAS1, IFIT1, IFI44L, ISG15, FAM20A, RETN | 0,873 | 0,863 | 0,883 | 0,910 | 0,847 | 0,973 | 0,945 | 0,868 | 1 |
| RSAD2, IFI27, OAS1, ISG15, HERC6, SLC1A2, IL1R2 | 0,873 | 0,865 | 0,882 | 0,924 | 0,879 | 0,969 | 0,933 | 0,867 | 0,998 |
| RSAD2, OAS1, IFI44L, SIGLEC1, ISG15, IL1R2, FAM20A | 0,873 | 0,863 | 0,883 | 0,910 | 0,846 | 0,973 | 0,963 | 0,912 | 1 |
| RSAD2, IFI27, IFIT1, IFI44L, SIGLEC1, ISG15, RETN | 0,873 | 0,864 | 0,882 | 0,909 | 0,848 | 0,970 | 0,950 | 0,892 | 1 |
| IFIT1, IFI44L, SIGLEC1, ISG15, SLC1A2, FAM20A, MMP8 | 0,873 | 0,864 | 0,882 | 0,911 | 0,851 | 0,970 | 0,938 | 0,864 | 1 |
| OAS1, SIGLEC1, ISG15, HERC6, SLC1A2, OLAH, FAM20A | 0,873 | 0,863 | 0,883 | 0,915 | 0,860 | 0,970 | 0,943 | 0,874 | 1 |
| OAS1, IFI44L, SIGLEC1, ISG15, IL1R2, FAM20A, RETN | 0,873 | 0,863 | 0,883 | 0,908 | 0,844 | 0,971 | 0,960 | 0,903 | 1 |
| IFI27, IFIT1, IFI44L, ISG15, FAM20A, RETN, MMP8 | 0,873 | 0,862 | 0,884 | 0,912 | 0,849 | 0,975 | 0,948 | 0,874 | 1 |
| IFI44L, SIGLEC1, ISG15, HERC6, SLC1A2, OLAH, FAM20A | 0,873 | 0,863 | 0,883 | 0,914 | 0,858 | 0,971 | 0,946 | 0,872 | 1 |
| RSAD2, IFI27, IFIT1, HERC6, SLC1A2, IL1R2, MMP8 | 0,873 | 0,864 | 0,882 | 0,923 | 0,877 | 0,968 | 0,937 | 0,877 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, HERC6, SLC1A2, OLAH, FAM20A | 0,873 | 0,862 | 0,883 | 0,916 | 0,862 | 0,971 | 0,943 | 0,875 | 1 |
| OAS1, IFIT1, IFI44L, SIGLEC1, ISG15, FAM20A, RETN | 0,873 | 0,863 | 0,883 | 0,902 | 0,832 | 0,972 | 0,942 | 0,881 | 1 |
| IFI27, OAS1, IFIT1, IFI44L, HERC6, SLC1A2, IL1R2 | 0,873 | 0,864 | 0,882 | 0,922 | 0,876 | 0,968 | 0,936 | 0,874 | 0,998 |
| IFI44L, SIGLEC1, ISG15, SLC1A2, OLAH, FAM20A, RETN | 0,873 | 0,863 | 0,882 | 0,914 | 0,856 | 0,971 | 0,946 | 0,879 | 1 |
| RSAD2, IFI27, OAS1, IFIT1, SIGLEC1, RETN, MMP8 | 0,873 | 0,863 | 0,883 | 0,910 | 0,848 | 0,972 | 0,954 | 0,895 | 1 |
| RSAD2, SIGLEC1, ISG15, SLC1A2, IL1R2, FAM20A, RETN | 0,873 | 0,863 | 0,883 | 0,915 | 0,862 | 0,968 | 0,952 | 0,882 | 1 |
| RSAD2, SIGLEC1, HERC6, SLC1A2, FAM20A, RETN, MMP8 | 0,873 | 0,863 | 0,883 | 0,912 | 0,854 | 0,971 | 0,949 | 0,877 | 1 |
| IFI44L, SIGLEC1, HERC6, SLC1A2, IL1R2, FAM20A, RETN | 0,873 | 0,863 | 0,883 | 0,920 | 0,868 | 0,971 | 0,952 | 0,880 | 1 |
| RSAD2, IFIT1, IFI44L, SIGLEC1, OLAH, FAM20A, RETN | 0,873 | 0,863 | 0,882 | 0,905 | 0,836 | 0,974 | 0,947 | 0,892 | 1 |
| RSAD2, IFI27, OAS1, SLC1A2, IL1R2, RETN, MMP8 | 0,873 | 0,864 | 0,881 | 0,924 | 0,874 | 0,974 | 0,934 | 0,863 | 1 |
| RSAD2, OAS1, IFIT1, SIGLEC1, FAM20A, RETN, MMP8 | 0,873 | 0,863 | 0,882 | 0,909 | 0,843 | 0,974 | 0,952 | 0,889 | 1 |
| RSAD2, OAS1, SIGLEC1, ISG15, OLAH, FAM20A, MMP8 | 0,873 | 0,862 | 0,883 | 0,905 | 0,840 | 0,971 | 0,960 | 0,910 | 1 |
| SIGLEC1, HERC6, SLC1A2, IL1R2, OLAH, FAM20A, RETN | 0,873 | 0,862 | 0,883 | 0,918 | 0,864 | 0,971 | 0,947 | 0,874 | 1 |
| RSAD2, SIGLEC1, ISG15, HERC6, SLC1A2, OLAH, FAM20A | 0,872 | 0,862 | 0,883 | 0,915 | 0,860 | 0,970 | 0,941 | 0,871 | 1 |
| RSAD2, IFI27, OAS1, IFI44L, HERC6, SLC1A2, IL1R2 | 0,872 | 0,863 | 0,881 | 0,923 | 0,878 | 0,968 | 0,930 | 0,864 | 0,997 |
| RSAD2, IFI44L, SIGLEC1, SLC1A2, IL1R2, OLAH, FAM20A | 0,872 | 0,862 | 0,882 | 0,916 | 0,861 | 0,970 | 0,954 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, ISG15, SLC1A2, RETN | 0,872 | 0,864 | 0,881 | 0,915 | 0,858 | 0,971 | 0,948 | 0,882 | 1 |
| RSAD2, IFI27, OAS1, IFI44L, SIGLEC1, SLC1A2, RETN | 0,872 | 0,864 | 0,881 | 0,916 | 0,858 | 0,974 | 0,948 | 0,876 | 1 |
| RSAD2, OAS1, IFI44L, SIGLEC1, OLAH, FAM20A, MMP8 | 0,872 | 0,862 | 0,882 | 0,906 | 0,840 | 0,972 | 0,958 | 0,910 | 1 |
| IFI27, OAS1, ISG15, HERC6, SLC1A2, IL1R2, MMP8 | 0,872 | 0,864 | 0,881 | 0,923 | 0,878 | 0,968 | 0,927 | 0,860 | 0,994 |
| RSAD2, IFI27, OAS1, IFIT1, IFI44L, HERC6, FAM20A | 0,872 | 0,862 | 0,882 | 0,910 | 0,848 | 0,972 | 0,926 | 0,845 | 1 |
| RSAD2, IFIT1, SIGLEC1, ISG15, OLAH, FAM20A, RETN | 0,872 | 0,863 | 0,882 | 0,904 | 0,834 | 0,974 | 0,953 | 0,902 | 1 |
| RSAD2, OAS1, SIGLEC1, OLAH, FAM20A, RETN, MMP8 | 0,872 | 0,862 | 0,882 | 0,908 | 0,843 | 0,973 | 0,966 | 0,924 | 1 |
| OAS1, IFI44L, SIGLEC1, SLC1A2, IL1R2, OLAH, FAM20A | 0,872 | 0,863 | 0,882 | 0,916 | 0,862 | 0,970 | 0,954 | 0,895 | 1 |
| RSAD2, IFI27, OAS1, IFI44L, HERC6, FAM20A, MMP8 | 0,872 | 0,862 | 0,883 | 0,911 | 0,851 | 0,971 | 0,925 | 0,844 | 1 |
| IFI27, SIGLEC1, ISG15, HERC6, SLC1A2, FAM20A, MMP8 | 0,872 | 0,862 | 0,883 | 0,912 | 0,852 | 0,972 | 0,943 | 0,866 | 1 |
| IFI27, OAS1, IFI44L, SIGLEC1, ISG15, RETN, MMP8 | 0,872 | 0,862 | 0,882 | 0,911 | 0,850 | 0,972 | 0,953 | 0,893 | 1 |
| IFI44L, SIGLEC1, ISG15, OLAH, FAM20A, RETN, MMP8 | 0,872 | 0,862 | 0,882 | 0,907 | 0,840 | 0,973 | 0,967 | 0,925 | 1 |
| RSAD2, IFI44L, SIGLEC1, IL1R2, OLAH, FAM20A, RETN | 0,872 | 0,862 | 0,882 | 0,906 | 0,842 | 0,970 | 0,963 | 0,912 | 1 |
| IFIT1, IFI44L, SIGLEC1, SLC1A2, FAM20A, RETN, MMP8 | 0,872 | 0,863 | 0,882 | 0,918 | 0,861 | 0,974 | 0,939 | 0,868 | 1 |
| RSAD2, OAS1, IFIT1, IFI44L, SIGLEC1, ISG15, FAM20A | 0,872 | 0,863 | 0,881 | 0,902 | 0,832 | 0,973 | 0,939 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SLC1A2, IL1R2, RETN | 0,872 | 0,864 | 0,880 | 0,920 | 0,868 | 0,972 | 0,929 | 0,854 | 1 |
| OAS1, IFI44L, SIGLEC1, HERC6, SLC1A2, OLAH, FAM20A | 0,872 | 0,862 | 0,882 | 0,916 | 0,861 | 0,971 | 0,941 | 0,872 | 1 |
| RSAD2, IFI27, IFIT1, ISG15, HERC6, SLC1A2, IL1R2 | 0,872 | 0,864 | 0,880 | 0,923 | 0,878 | 0,968 | 0,937 | 0,877 | 0,997 |
| RSAD2, OAS1, HERC6, IL1R2, OLAH, FAM20A, MMP8 | 0,872 | 0,862 | 0,882 | 0,908 | 0,849 | 0,968 | 0,936 | 0,858 | 1 |
| RSAD2, OAS1, IFIT1, SIGLEC1, ISG15, FAM20A, RETN | 0,872 | 0,862 | 0,882 | 0,902 | 0,833 | 0,972 | 0,950 | 0,891 | 1 |
| OAS1, IFI44L, SIGLEC1, ISG15, SLC1A2, IL1R2, FAM20A | 0,872 | 0,862 | 0,881 | 0,915 | 0,860 | 0,969 | 0,953 | 0,890 | 1 |
| RSAD2, IFI27, OAS1, IFIT1, SLC1A2, IL1R2, MMP8 | 0,872 | 0,863 | 0,881 | 0,920 | 0,872 | 0,968 | 0,936 | 0,871 | 1 |
| RSAD2, OAS1, IFI44L, SIGLEC1, IL1R2, FAM20A, RETN | 0,872 | 0,862 | 0,882 | 0,906 | 0,842 | 0,971 | 0,961 | 0,906 | 1 |
| RSAD2, IFI27, ISG15, SLC1A2, IL1R2, RETN, MMP8 | 0,872 | 0,863 | 0,880 | 0,922 | 0,872 | 0,972 | 0,930 | 0,860 | 1 |
| RSAD2, IFI27, OAS1, IFI44L, FAM20A, RETN, MMP8 | 0,872 | 0,861 | 0,883 | 0,913 | 0,850 | 0,976 | 0,946 | 0,872 | 1 |
| OAS1, IFIT1, SIGLEC1, HERC6, IL1R2, RETN, MMP8 | 0,872 | 0,862 | 0,881 | 0,904 | 0,842 | 0,967 | 0,963 | 0,925 | 1 |
| RSAD2, IFI27, IFIT1, ISG15, SLC1A2, IL1R2, RETN | 0,872 | 0,863 | 0,880 | 0,921 | 0,870 | 0,972 | 0,936 | 0,872 | 0,999 |
| IFI27, OAS1, IFIT1, ISG15, HERC6, FAM20A, MMP8 | 0,872 | 0,862 | 0,882 | 0,912 | 0,854 | 0,969 | 0,925 | 0,844 | 1 |
| RSAD2, IFI44L, SIGLEC1, SLC1A2, IL1R2, FAM20A, RETN | 0,872 | 0,862 | 0,881 | 0,915 | 0,862 | 0,969 | 0,954 | 0,888 | 1 |
| OAS1, IFI44L, SIGLEC1, ISG15, SLC1A2, OLAH, FAM20A | 0,872 | 0,862 | 0,882 | 0,912 | 0,854 | 0,971 | 0,947 | 0,885 | 1 |
| RSAD2, IFI27, OAS1, IFIT1, ISG15, FAM20A, RETN | 0,872 | 0,862 | 0,882 | 0,911 | 0,850 | 0,971 | 0,943 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SLC1A2, IL1R2, RETN | 0,872 | 0,863 | 0,880 | 0,919 | 0,867 | 0,972 | 0,936 | 0,871 | 1 |
| IFI27, IFIT1, IFI44L, SIGLEC1, ISG15, RETN, MMP8 | 0,872 | 0,862 | 0,882 | 0,910 | 0,848 | 0,971 | 0,950 | 0,892 | 1 |
| RSAD2, OAS1, SIGLEC1, ISG15, SLC1A2, IL1R2, FAM20A | 0,872 | 0,862 | 0,882 | 0,915 | 0,861 | 0,969 | 0,954 | 0,891 | 1 |
| RSAD2, IFI27, IFIT1, ISG15, SLC1A2, IL1R2, MMP8 | 0,872 | 0,863 | 0,880 | 0,922 | 0,876 | 0,968 | 0,936 | 0,875 | 0,997 |
| SIGLEC1, ISG15, HERC6, SLC1A2, IL1R2, FAM20A, RETN | 0,872 | 0,861 | 0,882 | 0,918 | 0,866 | 0,970 | 0,950 | 0,878 | 1 |
| RSAD2, IFI27, IFI44L, SLC1A2, IL1R2, RETN, MMP8 | 0,872 | 0,863 | 0,880 | 0,923 | 0,874 | 0,973 | 0,930 | 0,860 | 1 |
| RSAD2, IFI27, OAS1, IFI44L, ISG15, IL1R2, MMP8 | 0,872 | 0,863 | 0,880 | 0,914 | 0,862 | 0,966 | 0,937 | 0,881 | 0,993 |
| RSAD2, IFI27, OAS1, SIGLEC1, ISG15, SLC1A2, RETN | 0,872 | 0,862 | 0,881 | 0,914 | 0,855 | 0,972 | 0,950 | 0,881 | 1 |
| IFI27, OAS1, IFI44L, ISG15, HERC6, FAM20A, MMP8 | 0,872 | 0,861 | 0,882 | 0,909 | 0,848 | 0,970 | 0,930 | 0,852 | 1 |
| OAS1, IFIT1, IFI44L, SIGLEC1, ISG15, FAM20A, MMP8 | 0,872 | 0,861 | 0,882 | 0,901 | 0,831 | 0,972 | 0,939 | 0,870 | 1 |
| IFI27, IFI44L, SIGLEC1, HERC6, SLC1A2, RETN, MMP8 | 0,872 | 0,862 | 0,881 | 0,916 | 0,858 | 0,973 | 0,943 | 0,885 | 1 |
| RSAD2, IFI27, ISG15, HERC6, SLC1A2, IL1R2, MMP8 | 0,872 | 0,863 | 0,880 | 0,921 | 0,875 | 0,967 | 0,928 | 0,862 | 0,995 |
| IFI44L, ISG15, HERC6, IL1R2, OLAH, FAM20A, MMP8 | 0,872 | 0,862 | 0,881 | 0,909 | 0,849 | 0,968 | 0,940 | 0,865 | 1 |
| RSAD2, OAS1, SIGLEC1, ISG15, HERC6, FAM20A, RETN | 0,872 | 0,862 | 0,881 | 0,911 | 0,851 | 0,972 | 0,946 | 0,879 | 1 |
| OAS1, IFIT1, IFI44L, SIGLEC1, SLC1A2, FAM20A, MMP8 | 0,872 | 0,862 | 0,881 | 0,911 | 0,852 | 0,970 | 0,937 | 0,862 | 1 |
| IFI27, OAS1, IFIT1, ISG15, SLC1A2, IL1R2, MMP8 | 0,871 | 0,863 | 0,880 | 0,921 | 0,874 | 0,968 | 0,936 | 0,874 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, ISG15, SLC1A2, IL1R2, MMP8 | 0,871 | 0,863 | 0,880 | 0,922 | 0,875 | 0,968 | 0,937 | 0,878 | 0,996 |
| RSAD2, IFI27, IFIT1, ISG15, HERC6, FAM20A, MMP8 | 0,871 | 0,861 | 0,881 | 0,912 | 0,853 | 0,970 | 0,925 | 0,844 | 1 |
| RSAD2, IFI27, OAS1, IFIT1, ISG15, SLC1A2, IL1R2 | 0,871 | 0,863 | 0,880 | 0,921 | 0,874 | 0,968 | 0,937 | 0,872 | 1 |
| IFI44L, HERC6, SLC1A2, IL1R2, OLAH, FAM20A, MMP8 | 0,871 | 0,862 | 0,881 | 0,918 | 0,867 | 0,969 | 0,930 | 0,849 | 1 |
| RSAD2, IFI44L, SIGLEC1, HERC6, SLC1A2, OLAH, FAM20A | 0,871 | 0,861 | 0,882 | 0,917 | 0,862 | 0,972 | 0,939 | 0,868 | 1 |
| RSAD2, IFI27, OAS1, ISG15, SLC1A2, IL1R2, RETN | 0,871 | 0,863 | 0,880 | 0,920 | 0,868 | 0,973 | 0,930 | 0,856 | 1 |
| RSAD2, OAS1, SIGLEC1, SLC1A2, IL1R2, FAM20A, RETN | 0,871 | 0,862 | 0,881 | 0,915 | 0,861 | 0,969 | 0,953 | 0,888 | 1 |
| IFI27, OAS1, SIGLEC1, ISG15, SLC1A2, RETN, MMP8 | 0,871 | 0,862 | 0,881 | 0,915 | 0,857 | 0,972 | 0,948 | 0,880 | 1 |
| RSAD2, IFI27, IFIT1, IFI44L, HERC6, SLC1A2, IL1R2 | 0,871 | 0,863 | 0,880 | 0,923 | 0,877 | 0,969 | 0,936 | 0,876 | 0,995 |
| RSAD2, IFI27, IFI44L, ISG15, SLC1A2, IL1R2, RETN | 0,871 | 0,863 | 0,879 | 0,921 | 0,870 | 0,972 | 0,928 | 0,857 | 0,999 |
| RSAD2, IFI44L, SIGLEC1, HERC6, OLAH, FAM20A, MMP8 | 0,871 | 0,861 | 0,881 | 0,910 | 0,850 | 0,970 | 0,953 | 0,898 | 1 |
| RSAD2, IFI44L, SIGLEC1, ISG15, IL1R2, FAM20A, RETN | 0,871 | 0,861 | 0,881 | 0,906 | 0,842 | 0,970 | 0,963 | 0,910 | 1 |
| RSAD2, IFI27, OAS1, IFIT1, IFI44L, SLC1A2, IL1R2 | 0,871 | 0,863 | 0,880 | 0,921 | 0,874 | 0,969 | 0,940 | 0,879 | 1 |
| IFI27, OAS1, IFIT1, SIGLEC1, ISG15, HERC6, MMP8 | 0,871 | 0,863 | 0,880 | 0,908 | 0,855 | 0,962 | 0,912 | 0,839 | 0,985 |
| RSAD2, IFI27, OAS1, IFI44L, SIGLEC1, RETN, MMP8 | 0,871 | 0,861 | 0,881 | 0,910 | 0,848 | 0,972 | 0,953 | 0,890 | 1 |
| RSAD2, OAS1, IFIT1, IFI44L, SIGLEC1, FAM20A, MMP8 | 0,871 | 0,861 | 0,881 | 0,904 | 0,834 | 0,973 | 0,939 | 0,870 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, FAM20A, RETN, MMP8 | 0,871 | 0,860 | 0,882 | 0,914 | 0,851 | 0,977 | 0,943 | 0,868 | 1 |
| OAS1, SIGLEC1, ISG15, SLC1A2, IL1R2, FAM20A, RETN | 0,871 | 0,861 | 0,881 | 0,915 | 0,861 | 0,968 | 0,952 | 0,884 | 1 |
| RSAD2, IFI27, OAS1, SIGLEC1, ISG15, RETN, MMP8 | 0,871 | 0,861 | 0,881 | 0,913 | 0,852 | 0,974 | 0,957 | 0,895 | 1 |
| RSAD2, OAS1, SIGLEC1, ISG15, SLC1A2, OLAH, FAM20A | 0,871 | 0,861 | 0,881 | 0,912 | 0,854 | 0,971 | 0,950 | 0,891 | 1 |
| IFI27, IFIT1, IFI44L, HERC6, SLC1A2, IL1R2, MMP8 | 0,871 | 0,862 | 0,880 | 0,923 | 0,878 | 0,969 | 0,935 | 0,875 | 0,995 |
| OAS1, IFI44L, SIGLEC1, SLC1A2, IL1R2, FAM20A, RETN | 0,871 | 0,862 | 0,881 | 0,915 | 0,861 | 0,969 | 0,955 | 0,893 | 1 |
| IFI27, OAS1, IFIT1, IFI44L, FAM20A, RETN, MMP8 | 0,871 | 0,860 | 0,882 | 0,913 | 0,851 | 0,976 | 0,947 | 0,871 | 1 |
| RSAD2, IFI44L, SIGLEC1, ISG15, SLC1A2, IL1R2, FAM20A | 0,871 | 0,862 | 0,881 | 0,914 | 0,860 | 0,969 | 0,951 | 0,882 | 1 |
| IFI44L, SIGLEC1, ISG15, HERC6, FAM20A, RETN, MMP8 | 0,871 | 0,861 | 0,881 | 0,907 | 0,840 | 0,974 | 0,955 | 0,888 | 1 |
| OAS1, SIGLEC1, HERC6, SLC1A2, FAM20A, RETN, MMP8 | 0,871 | 0,861 | 0,881 | 0,912 | 0,854 | 0,971 | 0,950 | 0,881 | 1 |
| RSAD2, IFI27, OAS1, ISG15, HERC6, FAM20A, MMP8 | 0,871 | 0,861 | 0,881 | 0,912 | 0,854 | 0,970 | 0,927 | 0,847 | 1 |
| IFI27, OAS1, IFIT1, IFI44L, SLC1A2, IL1R2, MMP8 | 0,871 | 0,862 | 0,880 | 0,919 | 0,872 | 0,967 | 0,937 | 0,877 | 0,997 |
| RSAD2, IFI27, OAS1, SIGLEC1, SLC1A2, RETN, MMP8 | 0,871 | 0,861 | 0,881 | 0,914 | 0,855 | 0,973 | 0,950 | 0,881 | 1 |
| IFI27, OAS1, IFI44L, SLC1A2, IL1R2, RETN, MMP8 | 0,871 | 0,862 | 0,880 | 0,924 | 0,875 | 0,973 | 0,932 | 0,862 | 1 |
| RSAD2, OAS1, IFI44L, SIGLEC1, SLC1A2, IL1R2, FAM20A | 0,871 | 0,861 | 0,880 | 0,915 | 0,860 | 0,970 | 0,955 | 0,894 | 1 |
| IFIT1, IFI44L, HERC6, SLC1A2, OLAH, FAM20A, MMP8 | 0,871 | 0,861 | 0,881 | 0,918 | 0,864 | 0,971 | 0,922 | 0,839 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, ISG15, SLC1A2, FAM20A | 0,871 | 0,861 | 0,880 | 0,913 | 0,854 | 0,972 | 0,937 | 0,863 | 1 |
| RSAD2, IFI27, IFI44L, HERC6, SLC1A2, IL1R2, MMP8 | 0,871 | 0,862 | 0,880 | 0,921 | 0,875 | 0,968 | 0,926 | 0,859 | 0,993 |
| IFIT1, IFI44L, SLC1A2, IL1R2, OLAH, FAM20A, MMP8 | 0,871 | 0,862 | 0,880 | 0,917 | 0,866 | 0,969 | 0,930 | 0,852 | 1 |
| OAS1, SIGLEC1, HERC6, SLC1A2, OLAH, FAM20A, RETN | 0,871 | 0,860 | 0,881 | 0,914 | 0,859 | 0,969 | 0,943 | 0,875 | 1 |
| RSAD2, IFI27, IFI44L, ISG15, FAM20A, RETN, MMP8 | 0,871 | 0,860 | 0,882 | 0,914 | 0,850 | 0,977 | 0,942 | 0,867 | 1 |
| RSAD2, OAS1, IFIT1, IFI44L, SIGLEC1, FAM20A, RETN | 0,871 | 0,861 | 0,880 | 0,905 | 0,836 | 0,974 | 0,942 | 0,881 | 1 |
| IFI27, OAS1, ISG15, SLC1A2, IL1R2, RETN, MMP8 | 0,871 | 0,862 | 0,879 | 0,923 | 0,873 | 0,972 | 0,930 | 0,862 | 0,999 |
| OAS1, IFIT1, SIGLEC1, SLC1A2, FAM20A, RETN, MMP8 | 0,871 | 0,861 | 0,881 | 0,915 | 0,857 | 0,973 | 0,952 | 0,884 | 1 |
| IFIT1, IFI44L, SIGLEC1, ISG15, SLC1A2, FAM20A, RETN | 0,871 | 0,861 | 0,880 | 0,912 | 0,854 | 0,971 | 0,936 | 0,863 | 1 |
| IFI27, OAS1, IFI44L, ISG15, SLC1A2, IL1R2, RETN | 0,871 | 0,862 | 0,879 | 0,923 | 0,873 | 0,973 | 0,933 | 0,866 | 0,999 |
| IFIT1, IFI44L, ISG15, SLC1A2, OLAH, FAM20A, MMP8 | 0,871 | 0,862 | 0,880 | 0,913 | 0,856 | 0,970 | 0,923 | 0,847 | 0,999 |
| RSAD2, OAS1, SIGLEC1, ISG15, HERC6, OLAH, FAM20A | 0,871 | 0,861 | 0,881 | 0,907 | 0,845 | 0,970 | 0,950 | 0,893 | 1 |
| IFI27, OAS1, IFI44L, HERC6, SLC1A2, IL1R2, MMP8 | 0,871 | 0,862 | 0,880 | 0,920 | 0,875 | 0,966 | 0,929 | 0,863 | 0,995 |
| IFIT1, IFI44L, ISG15, HERC6, SLC1A2, OLAH, FAM20A | 0,871 | 0,861 | 0,881 | 0,916 | 0,862 | 0,970 | 0,916 | 0,830 | 1 |
| RSAD2, SIGLEC1, HERC6, SLC1A2, OLAH, FAM20A, RETN | 0,871 | 0,860 | 0,881 | 0,917 | 0,862 | 0,972 | 0,940 | 0,869 | 1 |
| IFI27, OAS1, IFI44L, ISG15, HERC6, SLC1A2, IL1R2 | 0,871 | 0,861 | 0,880 | 0,922 | 0,877 | 0,967 | 0,930 | 0,864 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, ISG15, SLC1A2, OLAH, FAM20A | 0,871 | 0,861 | 0,880 | 0,912 | 0,853 | 0,971 | 0,945 | 0,881 | 1 |
| IFI27, IFIT1, ISG15, HERC6, SLC1A2, IL1R2, MMP8 | 0,870 | 0,862 | 0,879 | 0,922 | 0,876 | 0,967 | 0,936 | 0,876 | 0,996 |
| RSAD2, OAS1, IFIT1, HERC6, IL1R2, FAM20A, MMP8 | 0,870 | 0,861 | 0,880 | 0,907 | 0,846 | 0,968 | 0,939 | 0,861 | 1 |
| RSAD2, IFI27, OAS1, IFIT1, HERC6, FAM20A, MMP8 | 0,870 | 0,860 | 0,880 | 0,912 | 0,854 | 0,970 | 0,927 | 0,846 | 1 |
| RSAD2, IFI44L, SIGLEC1, ISG15, HERC6, OLAH, FAM20A | 0,870 | 0,860 | 0,881 | 0,909 | 0,847 | 0,971 | 0,948 | 0,890 | 1 |
| IFI27, OAS1, IFI44L, ISG15, FAM20A, RETN, MMP8 | 0,870 | 0,859 | 0,882 | 0,912 | 0,849 | 0,975 | 0,948 | 0,874 | 1 |
| RSAD2, IFIT1, IFI44L, SIGLEC1, SLC1A2, FAM20A, MMP8 | 0,870 | 0,861 | 0,880 | 0,912 | 0,853 | 0,970 | 0,937 | 0,863 | 1 |
| RSAD2, OAS1, SIGLEC1, SLC1A2, OLAH, FAM20A, RETN | 0,870 | 0,860 | 0,881 | 0,914 | 0,856 | 0,972 | 0,951 | 0,894 | 1 |
| IFIT1, IFI44L, HERC6, IL1R2, OLAH, FAM20A, MMP8 | 0,870 | 0,861 | 0,880 | 0,909 | 0,850 | 0,968 | 0,937 | 0,860 | 1 |
| RSAD2, OAS1, IFIT1, IFI44L, SIGLEC1, SLC1A2, FAM20A | 0,870 | 0,861 | 0,880 | 0,912 | 0,852 | 0,971 | 0,936 | 0,862 | 1 |
| IFI27, OAS1, IFIT1, IFI44L, SIGLEC1, HERC6, MMP8 | 0,870 | 0,862 | 0,879 | 0,907 | 0,853 | 0,962 | 0,903 | 0,826 | 0,980 |
| IFI27, OAS1, IFIT1, IFI44L, ISG15, SLC1A2, IL1R2 | 0,870 | 0,862 | 0,879 | 0,922 | 0,875 | 0,969 | 0,937 | 0,877 | 0,997 |
| IFI44L, SIGLEC1, ISG15, HERC6, OLAH, FAM20A, RETN | 0,870 | 0,860 | 0,880 | 0,903 | 0,837 | 0,970 | 0,954 | 0,893 | 1 |
| IFI27, IFI44L, ISG15, SLC1A2, FAM20A, RETN, MMP8 | 0,870 | 0,860 | 0,881 | 0,913 | 0,851 | 0,974 | 0,941 | 0,862 | 1 |
| OAS1, SIGLEC1, ISG15, HERC6, OLAH, FAM20A, MMP8 | 0,870 | 0,860 | 0,881 | 0,905 | 0,842 | 0,968 | 0,958 | 0,907 | 1 |
| RSAD2, OAS1, IFIT1, SIGLEC1, ISG15, FAM20A, MMP8 | 0,870 | 0,860 | 0,880 | 0,902 | 0,832 | 0,971 | 0,946 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, HERC6, SLC1A2, MMP8 | 0,870 | 0,862 | 0,879 | 0,912 | 0,859 | 0,966 | 0,917 | 0,847 | 0,988 |
| RSAD2, IFIT1, IFI44L, SIGLEC1, ISG15, SLC1A2, FAM20A | 0,870 | 0,861 | 0,879 | 0,910 | 0,851 | 0,970 | 0,936 | 0,861 | 1 |
| IFI27, IFI44L, ISG15, HERC6, SLC1A2, IL1R2, MMP8 | 0,870 | 0,862 | 0,879 | 0,922 | 0,876 | 0,967 | 0,929 | 0,864 | 0,995 |
| IFI27, OAS1, IFI44L, SIGLEC1, SLC1A2, RETN, MMP8 | 0,870 | 0,861 | 0,879 | 0,915 | 0,858 | 0,972 | 0,950 | 0,885 | 1 |
| RSAD2, IFI27, IFIT1, IFI44L, SLC1A2, IL1R2, MMP8 | 0,870 | 0,862 | 0,878 | 0,922 | 0,875 | 0,968 | 0,940 | 0,882 | 0,999 |
| RSAD2, OAS1, IFI44L, SIGLEC1, SLC1A2, OLAH, FAM20A | 0,870 | 0,860 | 0,880 | 0,915 | 0,857 | 0,973 | 0,946 | 0,886 | 1 |
| IFIT1, IFI44L, ISG15, HERC6, IL1R2, OLAH, FAM20A | 0,870 | 0,861 | 0,879 | 0,909 | 0,850 | 0,968 | 0,933 | 0,853 | 1 |
| OAS1, SIGLEC1, ISG15, SLC1A2, OLAH, FAM20A, RETN | 0,870 | 0,860 | 0,880 | 0,914 | 0,857 | 0,972 | 0,948 | 0,886 | 1 |
| IFI27, IFIT1, IFI44L, ISG15, SLC1A2, FAM20A, RETN | 0,870 | 0,860 | 0,880 | 0,911 | 0,849 | 0,973 | 0,938 | 0,856 | 1 |
| IFI27, IFIT1, IFI44L, ISG15, HERC6, SLC1A2, IL1R2 | 0,870 | 0,861 | 0,879 | 0,923 | 0,877 | 0,969 | 0,934 | 0,873 | 0,994 |
| RSAD2, IFI27, IFI44L, ISG15, HERC6, SLC1A2, IL1R2 | 0,870 | 0,861 | 0,879 | 0,920 | 0,873 | 0,966 | 0,927 | 0,860 | 0,995 |
| RSAD2, IFIT1, IFI44L, SIGLEC1, ISG15, FAM20A, RETN | 0,870 | 0,861 | 0,879 | 0,900 | 0,830 | 0,970 | 0,943 | 0,884 | 1 |
| OAS1, IFI44L, SIGLEC1, SLC1A2, OLAH, FAM20A, RETN | 0,870 | 0,860 | 0,880 | 0,914 | 0,855 | 0,972 | 0,949 | 0,892 | 1 |
| RSAD2, IFIT1, IFI44L, SIGLEC1, FAM20A, RETN, MMP8 | 0,870 | 0,860 | 0,880 | 0,908 | 0,841 | 0,975 | 0,945 | 0,883 | 1 |
| OAS1, IFIT1, HERC6, IL1R2, OLAH, FAM20A, MMP8 | 0,870 | 0,860 | 0,880 | 0,905 | 0,845 | 0,966 | 0,942 | 0,868 | 1 |
| RSAD2, IFI27, IFIT1, ISG15, HERC6, SLC1A2, RETN | 0,870 | 0,860 | 0,880 | 0,915 | 0,858 | 0,972 | 0,936 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SLC1A2, IL1R2, MMP8 | 0,870 | 0,861 | 0,878 | 0,920 | 0,872 | 0,967 | 0,930 | 0,863 | 0,998 |
| RSAD2, IFIT1, IFI44L, SIGLEC1, ISG15, FAM20A, MMP8 | 0,870 | 0,860 | 0,880 | 0,900 | 0,831 | 0,970 | 0,938 | 0,869 | 1 |
| IFI44L, SLC1A2, IL1R2, OLAH, FAM20A, RETN, MMP8 | 0,870 | 0,861 | 0,879 | 0,917 | 0,864 | 0,970 | 0,935 | 0,859 | 1 |
| RSAD2, OAS1, ISG15, HERC6, IL1R2, OLAH, FAM20A | 0,870 | 0,860 | 0,880 | 0,906 | 0,846 | 0,966 | 0,937 | 0,862 | 1 |
| RSAD2, OAS1, IFIT1, IL1R2, OLAH, FAM20A, MMP8 | 0,870 | 0,861 | 0,879 | 0,908 | 0,847 | 0,970 | 0,945 | 0,873 | 1 |
| IFI44L, ISG15, HERC6, SLC1A2, IL1R2, OLAH, FAM20A | 0,870 | 0,860 | 0,880 | 0,914 | 0,860 | 0,968 | 0,929 | 0,846 | 1 |
| IFI27, IFIT1, IFI44L, SIGLEC1, ISG15, SLC1A2, RETN | 0,870 | 0,861 | 0,879 | 0,914 | 0,858 | 0,971 | 0,950 | 0,891 | 1 |
| IFIT1, IFI44L, SIGLEC1, HERC6, IL1R2, RETN, MMP8 | 0,870 | 0,860 | 0,879 | 0,902 | 0,839 | 0,966 | 0,965 | 0,928 | 1 |
| RSAD2, OAS1, IFI44L, HERC6, IL1R2, FAM20A, MMP8 | 0,870 | 0,860 | 0,879 | 0,912 | 0,854 | 0,970 | 0,930 | 0,846 | 1 |
| RSAD2, IFI44L, SIGLEC1, SLC1A2, OLAH, FAM20A, RETN | 0,870 | 0,860 | 0,880 | 0,915 | 0,857 | 0,973 | 0,945 | 0,884 | 1 |
| OAS1, IFI44L, SIGLEC1, HERC6, OLAH, FAM20A, MMP8 | 0,870 | 0,859 | 0,880 | 0,908 | 0,846 | 0,970 | 0,958 | 0,904 | 1 |
| RSAD2, SIGLEC1, ISG15, HERC6, OLAH, FAM20A, RETN | 0,870 | 0,860 | 0,880 | 0,908 | 0,847 | 0,970 | 0,949 | 0,892 | 1 |
| SIGLEC1, ISG15, HERC6, SLC1A2, OLAH, FAM20A, RETN | 0,870 | 0,859 | 0,880 | 0,912 | 0,855 | 0,970 | 0,945 | 0,871 | 1 |
| RSAD2, IFI27, IFIT1, SIGLEC1, ISG15, SLC1A2, RETN | 0,870 | 0,861 | 0,878 | 0,915 | 0,859 | 0,971 | 0,953 | 0,896 | 1 |
| OAS1, IFIT1, IFI44L, SIGLEC1, SLC1A2, FAM20A, RETN | 0,870 | 0,860 | 0,879 | 0,914 | 0,855 | 0,972 | 0,936 | 0,863 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, ISG15, HERC6, SLC1A2, RETN | 0,870 | 0,860 | 0,879 | 0,914 | 0,857 | 0,971 | 0,938 | 0,872 | 1 |
| RSAD2, IFI27, OAS1, ISG15, SLC1A2, IL1R2, MMP8 | 0,870 | 0,861 | 0,878 | 0,920 | 0,873 | 0,968 | 0,932 | 0,864 | 0,999 |
| RSAD2, SIGLEC1, ISG15, SLC1A2, OLAH, FAM20A, RETN | 0,870 | 0,860 | 0,880 | 0,915 | 0,857 | 0,972 | 0,949 | 0,888 | 1 |
| OAS1, IFI44L, SIGLEC1, HERC6, FAM20A, RETN, MMP8 | 0,870 | 0,860 | 0,879 | 0,907 | 0,842 | 0,972 | 0,951 | 0,886 | 1 |
| RSAD2, IFI27, IFIT1, IFI44L, SIGLEC1, SLC1A2, RETN | 0,869 | 0,861 | 0,878 | 0,916 | 0,860 | 0,972 | 0,952 | 0,895 | 1 |
| OAS1, IFI44L, SIGLEC1, ISG15, OLAH, FAM20A, MMP8 | 0,869 | 0,859 | 0,880 | 0,904 | 0,837 | 0,971 | 0,961 | 0,915 | 1 |
| RSAD2, IFI27, IFIT1, IFI44L, ISG15, SLC1A2, IL1R2 | 0,869 | 0,861 | 0,878 | 0,922 | 0,876 | 0,969 | 0,933 | 0,872 | 0,994 |
| RSAD2, IFI27, OAS1, IFIT1, FAM20A, RETN, MMP8 | 0,869 | 0,859 | 0,880 | 0,912 | 0,850 | 0,973 | 0,943 | 0,867 | 1 |
| IFI27, OAS1, IFIT1, IFI44L, ISG15, HERC6, FAM20A | 0,869 | 0,859 | 0,879 | 0,907 | 0,844 | 0,970 | 0,925 | 0,841 | 1 |
| IFI27, OAS1, IFIT1, ISG15, FAM20A, RETN, MMP8 | 0,869 | 0,859 | 0,880 | 0,911 | 0,848 | 0,973 | 0,945 | 0,869 | 1 |
| OAS1, IFIT1, IFI44L, HERC6, SLC1A2, OLAH, FAM20A | 0,869 | 0,859 | 0,880 | 0,917 | 0,863 | 0,970 | 0,913 | 0,826 | 1 |
| RSAD2, IFIT1, SIGLEC1, ISG15, FAM20A, RETN, MMP8 | 0,869 | 0,860 | 0,879 | 0,904 | 0,837 | 0,972 | 0,948 | 0,886 | 1 |
| IFI27, IFIT1, IFI44L, HERC6, SLC1A2, FAM20A, MMP8 | 0,869 | 0,859 | 0,879 | 0,913 | 0,857 | 0,970 | 0,914 | 0,825 | 1 |
| IFI44L, ISG15, HERC6, IL1R2, OLAH, FAM20A, RETN | 0,869 | 0,860 | 0,879 | 0,907 | 0,846 | 0,968 | 0,940 | 0,868 | 1 |
| RSAD2, OAS1, IFI44L, SIGLEC1, HERC6, OLAH, FAM20A | 0,869 | 0,859 | 0,879 | 0,906 | 0,844 | 0,969 | 0,947 | 0,889 | 1 |
| RSAD2, IFI44L, SIGLEC1, ISG15, OLAH, FAM20A, MMP8 | 0,869 | 0,859 | 0,879 | 0,904 | 0,837 | 0,970 | 0,962 | 0,920 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, ISG15, SLC1A2, IL1R2, MMP8 | 0,869 | 0,861 | 0,877 | 0,920 | 0,873 | 0,968 | 0,928 | 0,861 | 0,996 |
| RSAD2, IFI27, OAS1, IFIT1, SIGLEC1, HERC6, MMP8 | 0,869 | 0,860 | 0,878 | 0,908 | 0,851 | 0,964 | 0,904 | 0,826 | 0,983 |
| IFIT1, SIGLEC1, ISG15, HERC6, IL1R2, RETN, MMP8 | 0,869 | 0,860 | 0,879 | 0,904 | 0,841 | 0,967 | 0,964 | 0,927 | 1 |
| OAS1, SIGLEC1, ISG15, OLAH, FAM20A, RETN, MMP8 | 0,869 | 0,859 | 0,880 | 0,905 | 0,838 | 0,972 | 0,967 | 0,928 | 1 |
| RSAD2, IFI44L, ISG15, HERC6, IL1R2, OLAH, FAM20A | 0,869 | 0,859 | 0,879 | 0,909 | 0,850 | 0,968 | 0,936 | 0,863 | 1 |
| RSAD2, ISG15, HERC6, IL1R2, OLAH, FAM20A, MMP8 | 0,869 | 0,859 | 0,879 | 0,908 | 0,850 | 0,967 | 0,936 | 0,857 | 1 |
| RSAD2, OAS1, ISG15, HERC6, IL1R2, FAM20A, MMP8 | 0,869 | 0,859 | 0,879 | 0,908 | 0,849 | 0,967 | 0,932 | 0,849 | 1 |
| RSAD2, OAS1, SIGLEC1, HERC6, OLAH, FAM20A, RETN | 0,869 | 0,859 | 0,879 | 0,906 | 0,843 | 0,969 | 0,951 | 0,895 | 1 |
| IFI44L, SIGLEC1, HERC6, SLC1A2, OLAH, FAM20A, RETN | 0,869 | 0,859 | 0,880 | 0,914 | 0,857 | 0,972 | 0,947 | 0,874 | 1 |
| RSAD2, OAS1, SIGLEC1, HERC6, IL1R2, RETN, MMP8 | 0,869 | 0,859 | 0,879 | 0,902 | 0,838 | 0,966 | 0,966 | 0,931 | 1 |
| OAS1, IFIT1, SIGLEC1, ISG15, SLC1A2, FAM20A, RETN | 0,869 | 0,859 | 0,879 | 0,910 | 0,849 | 0,970 | 0,942 | 0,868 | 1 |
| RSAD2, IFI27, OAS1, IFI44L, ISG15, SLC1A2, IL1R2 | 0,869 | 0,861 | 0,877 | 0,922 | 0,876 | 0,968 | 0,929 | 0,861 | 0,998 |
| RSAD2, OAS1, IFIT1, SIGLEC1, ISG15, SLC1A2, FAM20A | 0,869 | 0,859 | 0,879 | 0,907 | 0,845 | 0,968 | 0,943 | 0,871 | 1 |
| IFIT1, ISG15, HERC6, IL1R2, OLAH, FAM20A, MMP8 | 0,869 | 0,860 | 0,879 | 0,907 | 0,848 | 0,966 | 0,945 | 0,872 | 1 |
| OAS1, IFI44L, SIGLEC1, ISG15, HERC6, OLAH, FAM20A | 0,869 | 0,859 | 0,879 | 0,905 | 0,841 | 0,969 | 0,954 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, SIGLEC1, ISG15, HERC6, SLC1A2, FAM20A, RETN | 0,869 | 0,859 | 0,880 | 0,911 | 0,853 | 0,968 | 0,946 | 0,876 | 1 |
| OAS1, IFI44L, SIGLEC1, HERC6, IL1R2, RETN, MMP8 | 0,869 | 0,860 | 0,879 | 0,903 | 0,840 | 0,966 | 0,962 | 0,923 | 1 |
| RSAD2, OAS1, SIGLEC1, ISG15, HERC6, SLC1A2, FAM20A | 0,869 | 0,859 | 0,879 | 0,910 | 0,854 | 0,967 | 0,939 | 0,863 | 1 |
| IFIT1, IFI44L, ISG15, HERC6, IL1R2, FAM20A, RETN | 0,869 | 0,860 | 0,878 | 0,911 | 0,852 | 0,970 | 0,934 | 0,855 | 1 |
| OAS1, IFIT1, SIGLEC1, ISG15, HERC6, IL1R2, RETN | 0,869 | 0,860 | 0,879 | 0,906 | 0,847 | 0,966 | 0,954 | 0,910 | 0,999 |
| OAS1, IFIT1, ISG15, HERC6, IL1R2, FAM20A, RETN | 0,869 | 0,859 | 0,879 | 0,907 | 0,847 | 0,967 | 0,935 | 0,853 | 1 |
| RSAD2, SIGLEC1, ISG15, HERC6, SLC1A2, FAM20A, RETN | 0,869 | 0,859 | 0,879 | 0,912 | 0,855 | 0,970 | 0,942 | 0,869 | 1 |
| RSAD2, IFI44L, SLC1A2, IL1R2, OLAH, FAM20A, MMP8 | 0,869 | 0,860 | 0,879 | 0,918 | 0,867 | 0,969 | 0,927 | 0,846 | 1 |
| IFIT1, IFI44L, SIGLEC1, ISG15, SLC1A2, OLAH, MMP8 | 0,869 | 0,860 | 0,878 | 0,912 | 0,857 | 0,968 | 0,950 | 0,903 | 0,997 |
| OAS1, ISG15, HERC6, IL1R2, FAM20A, RETN, MMP8 | 0,869 | 0,859 | 0,879 | 0,910 | 0,851 | 0,969 | 0,934 | 0,852 | 1 |
| IFIT1, SIGLEC1, HERC6, SLC1A2, OLAH, RETN, MMP8 | 0,869 | 0,859 | 0,879 | 0,914 | 0,859 | 0,969 | 0,951 | 0,906 | 0,997 |
| OAS1, ISG15, HERC6, IL1R2, OLAH, FAM20A, MMP8 | 0,869 | 0,859 | 0,879 | 0,909 | 0,851 | 0,968 | 0,938 | 0,860 | 1 |
| RSAD2, IFI27, IFIT1, IFI44L, SIGLEC1, RETN, MMP8 | 0,869 | 0,859 | 0,879 | 0,910 | 0,849 | 0,971 | 0,951 | 0,893 | 1 |
| RSAD2, OAS1, IFIT1, SIGLEC1, HERC6, IL1R2, RETN | 0,869 | 0,859 | 0,878 | 0,905 | 0,842 | 0,967 | 0,952 | 0,906 | 0,998 |
| OAS1, IFIT1, IFI44L, SIGLEC1, HERC6, IL1R2, RETN | 0,869 | 0,860 | 0,878 | 0,905 | 0,842 | 0,967 | 0,951 | 0,905 | 0,997 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, SIGLEC1, ISG15, SLC1A2, FAM20A, MMP8 | 0,869 | 0,859 | 0,878 | 0,909 | 0,848 | 0,969 | 0,946 | 0,872 | 1 |
| IFI27, OAS1, IFIT1, IFI44L, SLC1A2, FAM20A, RETN | 0,869 | 0,859 | 0,879 | 0,911 | 0,849 | 0,973 | 0,936 | 0,853 | 1 |
| RSAD2, SIGLEC1, ISG15, OLAH, FAM20A, RETN, MMP8 | 0,869 | 0,859 | 0,879 | 0,908 | 0,844 | 0,972 | 0,963 | 0,920 | 1 |
| RSAD2, OAS1, IFIT1, ISG15, HERC6, IL1R2, FAM20A | 0,869 | 0,859 | 0,878 | 0,906 | 0,845 | 0,966 | 0,935 | 0,855 | 1 |
| IFIT1, IFI44L, HERC6, SLC1A2, IL1R2, OLAH, FAM20A | 0,869 | 0,859 | 0,879 | 0,918 | 0,866 | 0,969 | 0,920 | 0,832 | 1 |
| RSAD2, OAS1, IFI44L, ISG15, HERC6, IL1R2, FAM20A | 0,869 | 0,859 | 0,878 | 0,909 | 0,850 | 0,968 | 0,936 | 0,860 | 1 |
| RSAD2, IFI27, IFIT1, SIGLEC1, ISG15, RETN, MMP8 | 0,869 | 0,859 | 0,879 | 0,910 | 0,849 | 0,971 | 0,955 | 0,900 | 1 |
| OAS1, SIGLEC1, ISG15, HERC6, IL1R2, RETN, MMP8 | 0,869 | 0,859 | 0,878 | 0,906 | 0,845 | 0,967 | 0,972 | 0,940 | 1 |
| OAS1, SIGLEC1, ISG15, HERC6, OLAH, FAM20A, RETN | 0,869 | 0,859 | 0,879 | 0,907 | 0,845 | 0,970 | 0,955 | 0,902 | 1 |
| OAS1, IFIT1, ISG15, HERC6, IL1R2, FAM20A, MMP8 | 0,869 | 0,859 | 0,878 | 0,907 | 0,846 | 0,967 | 0,937 | 0,859 | 1 |
| RSAD2, IFI27, OAS1, SIGLEC1, HERC6, SLC1A2, MMP8 | 0,869 | 0,859 | 0,878 | 0,913 | 0,859 | 0,966 | 0,929 | 0,862 | 0,997 |
| IFIT1, IFI44L, ISG15, HERC6, IL1R2, FAM20A, MMP8 | 0,869 | 0,859 | 0,878 | 0,910 | 0,850 | 0,969 | 0,932 | 0,847 | 1 |
| RSAD2, IFI44L, HERC6, SLC1A2, OLAH, FAM20A, MMP8 | 0,869 | 0,858 | 0,879 | 0,916 | 0,862 | 0,970 | 0,922 | 0,843 | 1 |
| IFIT1, IFI44L, SIGLEC1, HERC6, SLC1A2, OLAH, MMP8 | 0,869 | 0,859 | 0,879 | 0,913 | 0,858 | 0,968 | 0,941 | 0,889 | 0,993 |
| IFI27, OAS1, IFI44L, ISG15, SLC1A2, FAM20A, RETN | 0,869 | 0,858 | 0,879 | 0,911 | 0,848 | 0,973 | 0,938 | 0,856 | 1 |
| OAS1, IFI44L, SIGLEC1, OLAH, FAM20A, RETN, MMP8 | 0,869 | 0,858 | 0,879 | 0,906 | 0,840 | 0,972 | 0,967 | 0,927 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, SIGLEC1, HERC6, IL1R2, OLAH, RETN, MMP8 | 0,869 | 0,859 | 0,878 | 0,904 | 0,842 | 0,967 | 0,964 | 0,927 | 1 |
| ISG15, HERC6, IL1R2, OLAH, FAM20A, RETN, MMP8 | 0,869 | 0,859 | 0,879 | 0,909 | 0,850 | 0,969 | 0,939 | 0,863 | 1 |
| IFI27, IFIT1, IFI44L, SIGLEC1, ISG15, HERC6, MMP8 | 0,869 | 0,860 | 0,877 | 0,906 | 0,849 | 0,963 | 0,908 | 0,833 | 0,982 |
| IFI44L, ISG15, SLC1A2, IL1R2, OLAH, FAM20A, RETN | 0,869 | 0,859 | 0,878 | 0,913 | 0,858 | 0,968 | 0,927 | 0,849 | 1 |
| RSAD2, IFI44L, SIGLEC1, HERC6, FAM20A, RETN, MMP8 | 0,869 | 0,859 | 0,878 | 0,909 | 0,846 | 0,972 | 0,949 | 0,882 | 1 |
| IFI27, IFIT1, SIGLEC1, ISG15, SLC1A2, RETN, MMP8 | 0,869 | 0,859 | 0,878 | 0,915 | 0,860 | 0,971 | 0,953 | 0,896 | 1 |
| RSAD2, IFI27, IFIT1, IFI44L, HERC6, SLC1A2, RETN | 0,869 | 0,859 | 0,878 | 0,913 | 0,854 | 0,971 | 0,937 | 0,869 | 1 |
| IFI44L, ISG15, HERC6, IL1R2, FAM20A, RETN, MMP8 | 0,869 | 0,859 | 0,878 | 0,910 | 0,849 | 0,970 | 0,941 | 0,864 | 1 |
| RSAD2, IFI27, ISG15, HERC6, SLC1A2, RETN, MMP8 | 0,869 | 0,859 | 0,878 | 0,915 | 0,858 | 0,971 | 0,936 | 0,868 | 1 |
| OAS1, IFI44L, HERC6, IL1R2, OLAH, FAM20A, MMP8 | 0,869 | 0,859 | 0,878 | 0,909 | 0,849 | 0,968 | 0,940 | 0,865 | 1 |
| IFIT1, IFI44L, HERC6, SLC1A2, OLAH, FAM20A, RETN | 0,868 | 0,858 | 0,879 | 0,917 | 0,863 | 0,970 | 0,917 | 0,832 | 1 |
| RSAD2, ISG15, HERC6, IL1R2, FAM20A, RETN, MMP8 | 0,868 | 0,859 | 0,878 | 0,910 | 0,850 | 0,969 | 0,932 | 0,848 | 1 |
| RSAD2, IFI44L, SIGLEC1, ISG15, HERC6, FAM20A, RETN | 0,868 | 0,859 | 0,878 | 0,910 | 0,849 | 0,971 | 0,947 | 0,881 | 1 |
| RSAD2, OAS1, IFI44L, HERC6, IL1R2, OLAH, FAM20A | 0,868 | 0,858 | 0,878 | 0,907 | 0,846 | 0,968 | 0,935 | 0,860 | 1 |
| IFIT1, SIGLEC1, HERC6, SLC1A2, IL1R2, OLAH, MMP8 | 0,868 | 0,859 | 0,878 | 0,912 | 0,859 | 0,966 | 0,962 | 0,924 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, FAM20A, RETN, MMP8 | 0,868 | 0,858 | 0,879 | 0,910 | 0,848 | 0,971 | 0,942 | 0,865 | 1 |
| RSAD2, SIGLEC1, HERC6, SLC1A2, IL1R2, OLAH, MMP8 | 0,868 | 0,859 | 0,878 | 0,912 | 0,859 | 0,966 | 0,963 | 0,925 | 1 |
| IFIT1, SIGLEC1, ISG15, SLC1A2, FAM20A, RETN, MMP8 | 0,868 | 0,859 | 0,878 | 0,914 | 0,856 | 0,971 | 0,948 | 0,878 | 1 |
| IFI44L, SIGLEC1, ISG15, SLC1A2, IL1R2, OLAH, MMP8 | 0,868 | 0,860 | 0,877 | 0,913 | 0,860 | 0,966 | 0,965 | 0,929 | 1 |
| RSAD2, IFI27, OAS1, IFI44L, SIGLEC1, HERC6, MMP8 | 0,868 | 0,860 | 0,877 | 0,906 | 0,849 | 0,963 | 0,913 | 0,839 | 0,987 |
| OAS1, IFI44L, SIGLEC1, ISG15, HERC6, FAM20A, RETN | 0,868 | 0,859 | 0,878 | 0,907 | 0,845 | 0,969 | 0,949 | 0,885 | 1 |
| OAS1, IFIT1, SIGLEC1, HERC6, OLAH, RETN, MMP8 | 0,868 | 0,858 | 0,878 | 0,901 | 0,836 | 0,967 | 0,953 | 0,909 | 0,997 |
| RSAD2, OAS1, IFI44L, IL1R2, OLAH, FAM20A, MMP8 | 0,868 | 0,858 | 0,878 | 0,907 | 0,846 | 0,969 | 0,942 | 0,873 | 1 |
| IFI44L, HERC6, IL1R2, OLAH, FAM20A, RETN, MMP8 | 0,868 | 0,858 | 0,878 | 0,912 | 0,852 | 0,973 | 0,946 | 0,874 | 1 |
| IFIT1, ISG15, HERC6, IL1R2, FAM20A, RETN, MMP8 | 0,868 | 0,859 | 0,878 | 0,910 | 0,850 | 0,970 | 0,938 | 0,859 | 1 |
| OAS1, IFI44L, ISG15, HERC6, IL1R2, OLAH, FAM20A | 0,868 | 0,858 | 0,878 | 0,906 | 0,845 | 0,966 | 0,938 | 0,864 | 1 |
| RSAD2, OAS1, ISG15, HERC6, IL1R2, FAM20A, RETN | 0,868 | 0,858 | 0,878 | 0,907 | 0,847 | 0,967 | 0,933 | 0,849 | 1 |
| OAS1, SIGLEC1, HERC6, SLC1A2, IL1R2, OLAH, MMP8 | 0,868 | 0,859 | 0,878 | 0,912 | 0,859 | 0,966 | 0,961 | 0,922 | 1 |
| RSAD2, SIGLEC1, ISG15, HERC6, SLC1A2, FAM20A, MMP8 | 0,868 | 0,858 | 0,878 | 0,908 | 0,850 | 0,966 | 0,942 | 0,867 | 1 |
| IFI44L, ISG15, HERC6, SLC1A2, OLAH, FAM20A, MMP8 | 0,868 | 0,858 | 0,878 | 0,914 | 0,858 | 0,969 | 0,927 | 0,850 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, RETN, MMP8 | 0,868 | 0,858 | 0,878 | 0,911 | 0,850 | 0,971 | 0,952 | 0,895 | 1 |
| RSAD2, IFIT1, IFI44L, HERC6, SLC1A2, OLAH, FAM20A | 0,868 | 0,858 | 0,878 | 0,916 | 0,863 | 0,970 | 0,914 | 0,828 | 1 |
| IFI27, IFIT1, IFI44L, SLC1A2, FAM20A, RETN, MMP8 | 0,868 | 0,858 | 0,879 | 0,914 | 0,852 | 0,975 | 0,940 | 0,861 | 1 |
| IFIT1, SIGLEC1, ISG15, HERC6, SLC1A2, OLAH, MMP8 | 0,868 | 0,858 | 0,878 | 0,913 | 0,858 | 0,968 | 0,947 | 0,898 | 0,996 |
| OAS1, IFIT1, IFI44L, SIGLEC1, HERC6, IL1R2, MMP8 | 0,868 | 0,859 | 0,877 | 0,902 | 0,843 | 0,961 | 0,960 | 0,919 | 1 |
| OAS1, IFIT1, SIGLEC1, HERC6, SLC1A2, OLAH, MMP8 | 0,868 | 0,858 | 0,878 | 0,913 | 0,858 | 0,968 | 0,947 | 0,898 | 0,995 |
| IFI27, OAS1, SIGLEC1, ISG15, HERC6, SLC1A2, MMP8 | 0,868 | 0,859 | 0,877 | 0,911 | 0,857 | 0,964 | 0,923 | 0,853 | 0,992 |
| OAS1, IFIT1, HERC6, IL1R2, FAM20A, RETN, MMP8 | 0,868 | 0,858 | 0,878 | 0,908 | 0,845 | 0,970 | 0,941 | 0,862 | 1 |
| RSAD2, OAS1, IFIT1, SIGLEC1, SLC1A2, FAM20A, MMP8 | 0,868 | 0,858 | 0,878 | 0,908 | 0,847 | 0,969 | 0,946 | 0,872 | 1 |
| OAS1, IFIT1, ISG15, HERC6, IL1R2, OLAH, FAM20A | 0,868 | 0,858 | 0,878 | 0,906 | 0,846 | 0,966 | 0,936 | 0,860 | 1 |
| IFIT1, SIGLEC1, ISG15, HERC6, OLAH, RETN, MMP8 | 0,868 | 0,858 | 0,878 | 0,904 | 0,841 | 0,968 | 0,960 | 0,920 | 1,000 |
| RSAD2, ISG15, HERC6, IL1R2, OLAH, FAM20A, RETN | 0,868 | 0,858 | 0,878 | 0,906 | 0,846 | 0,966 | 0,937 | 0,862 | 1 |
| IFIT1, IFI44L, SIGLEC1, ISG15, HERC6, IL1R2, RETN | 0,868 | 0,859 | 0,877 | 0,904 | 0,843 | 0,966 | 0,955 | 0,912 | 0,999 |
| OAS1, IFIT1, IFI44L, HERC6, IL1R2, OLAH, FAM20A | 0,868 | 0,859 | 0,877 | 0,906 | 0,845 | 0,967 | 0,934 | 0,856 | 1 |
| OAS1, SIGLEC1, HERC6, SLC1A2, OLAH, RETN, MMP8 | 0,868 | 0,858 | 0,878 | 0,913 | 0,856 | 0,970 | 0,962 | 0,922 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, SLC1A2, RETN | 0,868 | 0,859 | 0,877 | 0,914 | 0,858 | 0,971 | 0,950 | 0,890 | 1 |
| RSAD2, IFIT1, SIGLEC1, SLC1A2, FAM20A, RETN, MMP8 | 0,868 | 0,858 | 0,878 | 0,913 | 0,855 | 0,971 | 0,950 | 0,881 | 1 |
| IFIT1, ISG15, HERC6, IL1R2, OLAH, FAM20A, RETN | 0,868 | 0,858 | 0,878 | 0,906 | 0,846 | 0,967 | 0,936 | 0,860 | 1 |
| IFIT1, HERC6, SLC1A2, IL1R2, OLAH, FAM20A, MMP8 | 0,868 | 0,858 | 0,878 | 0,916 | 0,865 | 0,968 | 0,932 | 0,850 | 1 |
| RSAD2, IFI27, IFIT1, IFI44L, SLC1A2, FAM20A, RETN | 0,868 | 0,858 | 0,878 | 0,910 | 0,847 | 0,972 | 0,938 | 0,857 | 1 |
| RSAD2, IFI44L, HERC6, IL1R2, OLAH, FAM20A, MMP8 | 0,868 | 0,858 | 0,878 | 0,910 | 0,851 | 0,968 | 0,933 | 0,854 | 1 |
| RSAD2, IFIT1, IFI44L, SLC1A2, OLAH, FAM20A, MMP8 | 0,868 | 0,858 | 0,877 | 0,913 | 0,856 | 0,970 | 0,924 | 0,849 | 0,998 |
| IFI44L, ISG15, SLC1A2, OLAH, FAM20A, RETN, MMP8 | 0,868 | 0,858 | 0,878 | 0,915 | 0,858 | 0,971 | 0,928 | 0,855 | 1 |
| OAS1, HERC6, IL1R2, OLAH, FAM20A, RETN, MMP8 | 0,868 | 0,858 | 0,878 | 0,908 | 0,848 | 0,969 | 0,942 | 0,868 | 1 |
| IFI44L, SIGLEC1, HERC6, SLC1A2, FAM20A, RETN, MMP8 | 0,868 | 0,858 | 0,878 | 0,906 | 0,843 | 0,969 | 0,954 | 0,881 | 1 |
| OAS1, IFIT1, IFI44L, HERC6, IL1R2, FAM20A, MMP8 | 0,868 | 0,859 | 0,877 | 0,909 | 0,849 | 0,969 | 0,934 | 0,851 | 1 |
| RSAD2, IFIT1, IFI44L, SIGLEC1, SLC1A2, FAM20A, RETN | 0,868 | 0,858 | 0,877 | 0,913 | 0,854 | 0,972 | 0,936 | 0,863 | 1 |
| RSAD2, IFI27, IFIT1, ISG15, FAM20A, RETN, MMP8 | 0,868 | 0,857 | 0,878 | 0,911 | 0,849 | 0,974 | 0,943 | 0,867 | 1 |
| OAS1, IFIT1, IFI44L, ISG15, HERC6, IL1R2, FAM20A | 0,868 | 0,858 | 0,877 | 0,908 | 0,850 | 0,967 | 0,934 | 0,854 | 1 |
| RSAD2, OAS1, IFIT1, HERC6, IL1R2, OLAH, FAM20A | 0,868 | 0,858 | 0,878 | 0,905 | 0,844 | 0,967 | 0,938 | 0,864 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, HERC6, SLC1A2, RETN, MMP8 | 0,868 | 0,858 | 0,877 | 0,911 | 0,851 | 0,971 | 0,927 | 0,853 | 1 |
| RSAD2, IFIT1, ISG15, HERC6, IL1R2, FAM20A, MMP8 | 0,868 | 0,858 | 0,877 | 0,908 | 0,849 | 0,967 | 0,936 | 0,858 | 1 |
| IFIT1, IFI44L, ISG15, HERC6, OLAH, FAM20A, RETN | 0,868 | 0,858 | 0,877 | 0,905 | 0,843 | 0,968 | 0,937 | 0,868 | 1 |
| RSAD2, IFIT1, ISG15, HERC6, IL1R2, FAM20A, RETN | 0,868 | 0,858 | 0,877 | 0,908 | 0,848 | 0,967 | 0,935 | 0,853 | 1 |
| RSAD2, IFIT1, SIGLEC1, HERC6, IL1R2, RETN, MMP8 | 0,868 | 0,858 | 0,877 | 0,904 | 0,841 | 0,966 | 0,963 | 0,925 | 1 |
| IFIT1, HERC6, IL1R2, OLAH, FAM20A, RETN, MMP8 | 0,868 | 0,858 | 0,878 | 0,908 | 0,846 | 0,969 | 0,942 | 0,868 | 1 |
| RSAD2, OAS1, SIGLEC1, ISG15, HERC6, FAM20A, MMP8 | 0,868 | 0,858 | 0,878 | 0,902 | 0,838 | 0,967 | 0,940 | 0,869 | 1 |
| RSAD2, HERC6, SLC1A2, IL1R2, OLAH, FAM20A, MMP8 | 0,868 | 0,858 | 0,877 | 0,914 | 0,861 | 0,966 | 0,926 | 0,837 | 1 |
| RSAD2, OAS1, IFIT1, SIGLEC1, SLC1A2, FAM20A, RETN | 0,868 | 0,858 | 0,878 | 0,909 | 0,849 | 0,970 | 0,945 | 0,873 | 1 |
| IFIT1, SIGLEC1, ISG15, SLC1A2, IL1R2, OLAH, MMP8 | 0,868 | 0,859 | 0,877 | 0,913 | 0,859 | 0,966 | 0,966 | 0,931 | 1 |
| IFI27, IFIT1, ISG15, HERC6, SLC1A2, RETN, MMP8 | 0,868 | 0,858 | 0,878 | 0,912 | 0,854 | 0,971 | 0,928 | 0,855 | 1 |
| IFIT1, IFI44L, SIGLEC1, HERC6, OLAH, RETN, MMP8 | 0,868 | 0,858 | 0,878 | 0,901 | 0,835 | 0,966 | 0,958 | 0,917 | 0,999 |
| OAS1, IFI44L, SIGLEC1, HERC6, OLAH, FAM20A, RETN | 0,868 | 0,858 | 0,877 | 0,906 | 0,843 | 0,970 | 0,954 | 0,899 | 1 |
| RSAD2, IFIT1, IFI44L, ISG15, HERC6, IL1R2, FAM20A | 0,868 | 0,858 | 0,877 | 0,909 | 0,851 | 0,967 | 0,930 | 0,849 | 1 |
| RSAD2, OAS1, HERC6, IL1R2, FAM20A, RETN, MMP8 | 0,868 | 0,858 | 0,877 | 0,906 | 0,844 | 0,968 | 0,938 | 0,857 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, HERC6, IL1R2, OLAH, FAM20A, RETN, MMP8 | 0,868 | 0,858 | 0,878 | 0,906 | 0,845 | 0,967 | 0,941 | 0,866 | 1 |
| OAS1, HERC6, SLC1A2, IL1R2, OLAH, FAM20A, MMP8 | 0,867 | 0,858 | 0,877 | 0,914 | 0,862 | 0,966 | 0,925 | 0,838 | 1 |
| RSAD2, IFIT1, SIGLEC1, ISG15, SLC1A2, FAM20A, MMP8 | 0,867 | 0,858 | 0,877 | 0,906 | 0,845 | 0,967 | 0,946 | 0,874 | 1 |
| RSAD2, OAS1, HERC6, SLC1A2, IL1R2, OLAH, FAM20A | 0,867 | 0,857 | 0,877 | 0,913 | 0,858 | 0,967 | 0,926 | 0,840 | 1 |
| RSAD2, OAS1, SIGLEC1, HERC6, SLC1A2, FAM20A, MMP8 | 0,867 | 0,857 | 0,877 | 0,908 | 0,851 | 0,965 | 0,941 | 0,866 | 1 |
| RSAD2, IFI27, IFIT1, SIGLEC1, SLC1A2, RETN, MMP8 | 0,867 | 0,858 | 0,877 | 0,915 | 0,859 | 0,971 | 0,953 | 0,896 | 1 |
| RSAD2, OAS1, IFIT1, SIGLEC1, HERC6, SLC1A2, OLAH | 0,867 | 0,858 | 0,877 | 0,912 | 0,857 | 0,967 | 0,951 | 0,905 | 0,998 |
| RSAD2, IFI44L, HERC6, SLC1A2, IL1R2, OLAH, FAM20A | 0,867 | 0,857 | 0,878 | 0,915 | 0,861 | 0,968 | 0,924 | 0,840 | 1 |
| OAS1, ISG15, HERC6, IL1R2, OLAH, FAM20A, RETN | 0,867 | 0,857 | 0,878 | 0,906 | 0,846 | 0,967 | 0,937 | 0,862 | 1 |
| OAS1, ISG15, HERC6, SLC1A2, IL1R2, OLAH, FAM20A | 0,867 | 0,857 | 0,878 | 0,912 | 0,857 | 0,966 | 0,927 | 0,842 | 1 |
| OAS1, IFIT1, IFI44L, SIGLEC1, HERC6, SLC1A2, OLAH | 0,867 | 0,858 | 0,877 | 0,912 | 0,858 | 0,967 | 0,939 | 0,886 | 0,993 |
| IFI27, OAS1, IFI44L, SIGLEC1, ISG15, HERC6, MMP8 | 0,867 | 0,859 | 0,876 | 0,908 | 0,854 | 0,963 | 0,911 | 0,836 | 0,986 |
| RSAD2, IFIT1, HERC6, IL1R2, OLAH, FAM20A, MMP8 | 0,867 | 0,858 | 0,877 | 0,905 | 0,846 | 0,965 | 0,946 | 0,875 | 1 |
| IFI44L, SIGLEC1, ISG15, HERC6, IL1R2, RETN, MMP8 | 0,867 | 0,858 | 0,877 | 0,901 | 0,837 | 0,965 | 0,964 | 0,927 | 1 |
| RSAD2, SIGLEC1, ISG15, HERC6, IL1R2, RETN, MMP8 | 0,867 | 0,858 | 0,877 | 0,906 | 0,844 | 0,968 | 0,963 | 0,925 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, ISG15, HERC6, IL1R2, OLAH, FAM20A | 0,867 | 0,858 | 0,877 | 0,907 | 0,847 | 0,967 | 0,935 | 0,858 | 1 |
| SIGLEC1, ISG15, HERC6, SLC1A2, FAM20A, RETN, MMP8 | 0,867 | 0,857 | 0,878 | 0,904 | 0,840 | 0,968 | 0,953 | 0,880 | 1 |
| RSAD2, IFIT1, SIGLEC1, ISG15, SLC1A2, FAM20A, RETN | 0,867 | 0,858 | 0,877 | 0,909 | 0,849 | 0,970 | 0,946 | 0,876 | 1 |
| RSAD2, OAS1, IFI44L, SIGLEC1, ISG15, OLAH, FAM20A | 0,867 | 0,857 | 0,877 | 0,898 | 0,827 | 0,969 | 0,959 | 0,911 | 1 |
| OAS1, IFIT1, SIGLEC1, HERC6, SLC1A2, OLAH, RETN | 0,867 | 0,857 | 0,877 | 0,915 | 0,860 | 0,969 | 0,940 | 0,887 | 0,993 |
| OAS1, IFIT1, SLC1A2, IL1R2, OLAH, FAM20A, MMP8 | 0,867 | 0,858 | 0,876 | 0,914 | 0,862 | 0,967 | 0,937 | 0,859 | 1 |
| IFI27, OAS1, IFI44L, SLC1A2, FAM20A, RETN, MMP8 | 0,867 | 0,857 | 0,878 | 0,914 | 0,853 | 0,975 | 0,939 | 0,859 | 1 |
| IFI27, IFIT1, IFI44L, ISG15, HERC6, SLC1A2, RETN | 0,867 | 0,858 | 0,877 | 0,911 | 0,852 | 0,971 | 0,939 | 0,870 | 1 |
| IFIT1, IFI44L, SIGLEC1, ISG15, HERC6, SLC1A2, OLAH | 0,867 | 0,858 | 0,877 | 0,913 | 0,858 | 0,968 | 0,940 | 0,888 | 0,993 |
| RSAD2, IFIT1, SIGLEC1, HERC6, SLC1A2, OLAH, MMP8 | 0,867 | 0,857 | 0,877 | 0,912 | 0,857 | 0,967 | 0,947 | 0,898 | 0,996 |
| IFIT1, IFI44L, HERC6, OLAH, FAM20A, RETN, MMP8 | 0,867 | 0,857 | 0,877 | 0,906 | 0,842 | 0,971 | 0,940 | 0,871 | 1 |
| SIGLEC1, ISG15, HERC6, SLC1A2, IL1R2, OLAH, MMP8 | 0,867 | 0,858 | 0,876 | 0,912 | 0,859 | 0,966 | 0,963 | 0,926 | 1 |
| IFIT1, IFI44L, SIGLEC1, ISG15, HERC6, IL1R2, MMP8 | 0,867 | 0,858 | 0,876 | 0,904 | 0,845 | 0,962 | 0,960 | 0,919 | 1 |
| IFI27, IFI44L, SIGLEC1, ISG15, SLC1A2, RETN, MMP8 | 0,867 | 0,858 | 0,877 | 0,914 | 0,856 | 0,971 | 0,951 | 0,893 | 1 |
| RSAD2, OAS1, IFIT1, IFI44L, HERC6, IL1R2, FAM20A | 0,867 | 0,858 | 0,876 | 0,908 | 0,848 | 0,969 | 0,936 | 0,857 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, HERC6, SLC1A2, OLAH, RETN | 0,867 | 0,857 | 0,877 | 0,914 | 0,858 | 0,969 | 0,939 | 0,886 | 0,992 |
| IFI27, OAS1, IFIT1, ISG15, SLC1A2, FAM20A, RETN | 0,867 | 0,857 | 0,877 | 0,908 | 0,847 | 0,969 | 0,939 | 0,858 | 1 |
| RSAD2, IFI27, OAS1, IFI44L, SLC1A2, FAM20A, RETN | 0,867 | 0,857 | 0,877 | 0,910 | 0,848 | 0,972 | 0,940 | 0,861 | 1 |
| RSAD2, IFI27, IFI44L, ISG15, HERC6, SLC1A2, FAM20A | 0,867 | 0,856 | 0,878 | 0,913 | 0,855 | 0,971 | 0,926 | 0,844 | 1 |
| RSAD2, OAS1, ISG15, IL1R2, OLAH, FAM20A, MMP8 | 0,867 | 0,857 | 0,877 | 0,905 | 0,843 | 0,967 | 0,933 | 0,854 | 1 |
| RSAD2, IFI44L, ISG15, HERC6, IL1R2, FAM20A, MMP8 | 0,867 | 0,857 | 0,877 | 0,912 | 0,854 | 0,970 | 0,930 | 0,847 | 1 |
| RSAD2, IFI27, IFI44L, ISG15, SLC1A2, FAM20A, RETN | 0,867 | 0,857 | 0,877 | 0,910 | 0,848 | 0,972 | 0,939 | 0,859 | 1 |
| RSAD2, IFIT1, SLC1A2, IL1R2, OLAH, FAM20A, MMP8 | 0,867 | 0,858 | 0,876 | 0,914 | 0,861 | 0,966 | 0,936 | 0,857 | 1 |
| RSAD2, OAS1, SIGLEC1, ISG15, FAM20A, RETN, MMP8 | 0,867 | 0,857 | 0,877 | 0,904 | 0,837 | 0,971 | 0,955 | 0,893 | 1 |
| OAS1, IFIT1, SIGLEC1, ISG15, HERC6, OLAH, RETN | 0,867 | 0,857 | 0,877 | 0,904 | 0,842 | 0,966 | 0,946 | 0,896 | 0,995 |
| IFIT1, IFI44L, HERC6, IL1R2, FAM20A, RETN, MMP8 | 0,867 | 0,857 | 0,877 | 0,908 | 0,847 | 0,969 | 0,941 | 0,862 | 1 |
| IFIT1, IFI44L, ISG15, SLC1A2, IL1R2, OLAH, FAM20A | 0,867 | 0,857 | 0,876 | 0,913 | 0,857 | 0,969 | 0,929 | 0,853 | 1 |
| IFIT1, SIGLEC1, ISG15, HERC6, SLC1A2, IL1R2, OLAH | 0,867 | 0,858 | 0,876 | 0,913 | 0,858 | 0,967 | 0,952 | 0,908 | 0,997 |
| IFI44L, HERC6, SLC1A2, OLAH, FAM20A, RETN, MMP8 | 0,867 | 0,857 | 0,877 | 0,914 | 0,856 | 0,972 | 0,937 | 0,861 | 1 |
| OAS1, IFIT1, SIGLEC1, HERC6, SLC1A2, IL1R2, RETN | 0,867 | 0,857 | 0,876 | 0,911 | 0,857 | 0,966 | 0,951 | 0,905 | 0,997 |
| IFI27, OAS1, IFI44L, ISG15, SLC1A2, IL1R2, MMP8 | 0,867 | 0,859 | 0,875 | 0,922 | 0,876 | 0,968 | 0,928 | 0,863 | 0,994 |

(suite)

| Modèle | AUC modèle | IC 95% | | AUC Train | IC 95% Train | | AUC Test | IC 95% Test | |
|---|---|---|---|---|---|---|---|---|---|
| OAS1, IFI44L, ISG15, HERC6, IL1R2, FAM20A, MMP8 | 0,867 | 0,857 | 0,877 | 0,910 | 0,850 | 0,969 | 0,933 | 0,850 | 1 |
| RSAD2, OAS1, IFIT1, SIGLEC1, ISG15, HERC6, RETN | 0,867 | 0,857 | 0,877 | 0,903 | 0,839 | 0,967 | 0,933 | 0,875 | 0,990 |
| RSAD2, OAS1, SIGLEC1, HERC6, SLC1A2, IL1R2, OLAH | 0,867 | 0,857 | 0,877 | 0,911 | 0,857 | 0,966 | 0,962 | 0,922 | 1 |
| OAS1, SIGLEC1, ISG15, HERC6, SLC1A2, IL1R2, OLAH | 0,867 | 0,857 | 0,877 | 0,911 | 0,857 | 0,966 | 0,959 | 0,917 | 1 |
| RSAD2, ISG15, HERC6, SLC1A2, IL1R2, OLAH, FAM20A | 0,867 | 0,857 | 0,877 | 0,913 | 0,859 | 0,967 | 0,927 | 0,842 | 1 |
| OAS1, SIGLEC1, HERC6, SLC1A2, IL1R2, RETN, MMP8 | 0,867 | 0,858 | 0,876 | 0,914 | 0,859 | 0,968 | 0,967 | 0,932 | 1 |
| IFIT1, IFI44L, HERC6, SLC1A2, IL1R2, FAM20A, MMP8 | 0,867 | 0,857 | 0,877 | 0,916 | 0,865 | 0,967 | 0,924 | 0,830 | 1 |
| IFI27, IFIT1, IFI44L, SIGLEC1, SLC1A2, RETN, MMP8 | 0,867 | 0,858 | 0,876 | 0,916 | 0,860 | 0,972 | 0,952 | 0,894 | 1 |
| RSAD2, IFI44L, SIGLEC1, HERC6, OLAH, FAM20A, RETN | 0,867 | 0,857 | 0,877 | 0,907 | 0,845 | 0,970 | 0,946 | 0,888 | 1 |
| IFI27, SIGLEC1, ISG15, HERC6, SLC1A2, RETN, MMP8 | 0,867 | 0,857 | 0,877 | 0,911 | 0,849 | 0,972 | 0,950 | 0,891 | 1 |
| OAS1, IFI44L, HERC6, SLC1A2, OLAH, FAM20A, MMP8 | 0,867 | 0,856 | 0,878 | 0,914 | 0,859 | 0,969 | 0,927 | 0,850 | 1 |
| OAS1, IFIT1, SIGLEC1, HERC6, SLC1A2, IL1R2, OLAH | 0,867 | 0,857 | 0,877 | 0,913 | 0,859 | 0,967 | 0,953 | 0,909 | 0,997 |
| RSAD2, OAS1, IFI44L, SLC1A2, OLAH, FAM20A, MMP8 | 0,867 | 0,857 | 0,877 | 0,915 | 0,859 | 0,971 | 0,924 | 0,849 | 0,999 |
| RSAD2, IFIT1, SIGLEC1, HERC6, SLC1A2, IL1R2, OLAH | 0,867 | 0,857 | 0,876 | 0,914 | 0,859 | 0,968 | 0,954 | 0,911 | 0,998 |
| OAS1, SIGLEC1, HERC6, IL1R2, OLAH, RETN, MMP8 | 0,867 | 0,857 | 0,877 | 0,904 | 0,841 | 0,968 | 0,967 | 0,932 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SLC1A2, FAM20A, RETN, MMP8 | 0,867 | 0,856 | 0,877 | 0,914 | 0,854 | 0,975 | 0,940 | 0,861 | 1 |
| OAS1, IFI44L, SLC1A2, IL1R2, OLAH, FAM20A, MMP8 | 0,867 | 0,857 | 0,877 | 0,917 | 0,865 | 0,969 | 0,929 | 0,848 | 1 |
| OAS1, IFIT1, IFI44L, SIGLEC1, ISG15, HERC6, RETN | 0,867 | 0,857 | 0,876 | 0,904 | 0,841 | 0,967 | 0,932 | 0,874 | 0,989 |
| OAS1, IFI44L, ISG15, HERC6, SLC1A2, OLAH, FAM20A | 0,867 | 0,856 | 0,877 | 0,911 | 0,855 | 0,968 | 0,925 | 0,845 | 1 |
| IFI44L, SIGLEC1, SLC1A2, IL1R2, OLAH, RETN, MMP8 | 0,867 | 0,857 | 0,876 | 0,914 | 0,859 | 0,968 | 0,970 | 0,937 | 1 |
| IFIT1, IFI44L, SIGLEC1, SLC1A2, OLAH, RETN, MMP8 | 0,867 | 0,857 | 0,876 | 0,913 | 0,857 | 0,969 | 0,955 | 0,911 | 1 |
| OAS1, IFIT1, SIGLEC1, HERC6, IL1R2, OLAH, MMP8 | 0,867 | 0,858 | 0,876 | 0,905 | 0,848 | 0,963 | 0,958 | 0,915 | 1,000 |
| OAS1, IFIT1, IFI44L, SIGLEC1, SLC1A2, OLAH, MMP8 | 0,867 | 0,857 | 0,876 | 0,911 | 0,855 | 0,967 | 0,952 | 0,907 | 0,998 |
| RSAD2, IFI44L, ISG15, HERC6, IL1R2, FAM20A, RETN | 0,867 | 0,857 | 0,877 | 0,911 | 0,852 | 0,969 | 0,936 | 0,860 | 1 |
| IFIT1, SIGLEC1, HERC6, SLC1A2, IL1R2, RETN, MMP8 | 0,867 | 0,857 | 0,876 | 0,911 | 0,857 | 0,966 | 0,963 | 0,925 | 1 |
| RSAD2, IFI27, IFI44L, HERC6, SLC1A2, RETN, MMP8 | 0,867 | 0,857 | 0,876 | 0,912 | 0,853 | 0,971 | 0,933 | 0,864 | 1 |
| RSAD2, OAS1, SLC1A2, IL1R2, OLAH, FAM20A, MMP8 | 0,867 | 0,858 | 0,876 | 0,914 | 0,861 | 0,967 | 0,932 | 0,849 | 1 |
| RSAD2, IFI27, IFI44L, HERC6,SLC1A2,FAM20A, MMP8 | 0,867 | 0,856 | 0,877 | 0,913 | 0,857 | 0,970 | 0,925 | 0,842 | 1 |
| IFIT1, IFI44L, SLC1A2, OLAH, FAM20A, RETN, MMP8 | 0,867 | 0,857 | 0,876 | 0,913 | 0,856 | 0,970 | 0,927 | 0,852 | 1 |
| OAS1, IFIT1, IFI44L, SLC1A2, OLAH, FAM20A, MMP8 | 0,867 | 0,857 | 0,877 | 0,913 | 0,856 | 0,969 | 0,925 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, SIGLEC1, ISG15, HERC6, SLC1A2, OLAH, MMP8 | 0,867 | 0,857 | 0,877 | 0,911 | 0,855 | 0,966 | 0,954 | 0,910 | 0,999 |
| RSAD2, IFI44L, SIGLEC1, ISG15, HERC6, SLC1A2, FAM20A | 0,867 | 0,857 | 0,877 | 0,910 | 0,853 | 0,967 | 0,939 | 0,863 | 1 |
| RSAD2, IFI44L, SIGLEC1, OLAH, FAM20A, RETN, MMP8 | 0,867 | 0,856 | 0,877 | 0,908 | 0,844 | 0,972 | 0,962 | 0,919 | 1 |
| RSAD2, OAS1, IFIT1, IFI44L, SIGLEC1, HERC6, IL1R2 | 0,867 | 0,858 | 0,876 | 0,901 | 0,841 | 0,962 | 0,961 | 0,921 | 1 |
| IFI44L, SIGLEC1, HERC6, IL1R2, OLAH, RETN, MMP8 | 0,867 | 0,857 | 0,877 | 0,902 | 0,838 | 0,966 | 0,962 | 0,922 | 1 |
| RSAD2, IFI27, SIGLEC1, ISG15, SLC1A2, RETN, MMP8 | 0,867 | 0,857 | 0,876 | 0,914 | 0,856 | 0,971 | 0,952 | 0,895 | 1 |
| RSAD2, OAS1, SIGLEC1, HERC6, SLC1A2, FAM20A, RETN | 0,867 | 0,856 | 0,877 | 0,912 | 0,855 | 0,968 | 0,942 | 0,870 | 1 |
| IFIT1, IFI44L, ISG15, HERC6, OLAH, FAM20A, MMP8 | 0,867 | 0,857 | 0,877 | 0,901 | 0,838 | 0,965 | 0,937 | 0,868 | 1 |
| IFIT1, ISG15, HERC6, SLC1A2, IL1R2, OLAH, FAM20A | 0,867 | 0,857 | 0,876 | 0,912 | 0,858 | 0,967 | 0,928 | 0,844 | 1 |
| OAS1, IFI44L, ISG15, HERC6, IL1R2, FAM20A, RETN | 0,867 | 0,856 | 0,877 | 0,908 | 0,848 | 0,968 | 0,939 | 0,864 | 1 |
| RSAD2, OAS1, IFI44L, SIGLEC1, ISG15, HERC6, FAM20A | 0,867 | 0,857 | 0,876 | 0,902 | 0,838 | 0,967 | 0,940 | 0,869 | 1 |
| IFI44L, SIGLEC1, ISG15, HERC6, SLC1A2, FAM20A, RETN | 0,866 | 0,856 | 0,877 | 0,904 | 0,841 | 0,966 | 0,950 | 0,876 | 1 |
| RSAD2, OAS1, SIGLEC1, HERC6, SLC1A2, OLAH, MMP8 | 0,866 | 0,856 | 0,877 | 0,911 | 0,856 | 0,967 | 0,954 | 0,910 | 0,999 |
| OAS1, IFI44L, HERC6, SLC1A2, IL1R2, OLAH, FAM20A | 0,866 | 0,856 | 0,877 | 0,914 | 0,860 | 0,967 | 0,923 | 0,838 | 1 |
| RSAD2, IFIT1, IFI44L, HERC6, IL1R2, OLAH, FAM20A | 0,866 | 0,857 | 0,876 | 0,908 | 0,849 | 0,967 | 0,933 | 0,855 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, ISG15, HERC6, SLC1A2, RETN, MMP8 | 0,866 | 0,857 | 0,876 | 0,909 | 0,848 | 0,970 | 0,934 | 0,862 | 1 |
| IFI44L, SIGLEC1, HERC6, SLC1A2, IL1R2, OLAH, MMP8 | 0,866 | 0,857 | 0,876 | 0,911 | 0,857 | 0,965 | 0,965 | 0,929 | 1 |
| IFI44L, HERC6, SLC1A2, IL1R2, OLAH, FAM20A, RETN | 0,866 | 0,856 | 0,877 | 0,914 | 0,860 | 0,968 | 0,929 | 0,846 | 1 |
| OAS1, IFIT1, SIGLEC1, HERC6, IL1R2, OLAH, RETN | 0,866 | 0,857 | 0,876 | 0,905 | 0,843 | 0,967 | 0,945 | 0,895 | 0,994 |
| RSAD2, IFIT1, IFI44L, SIGLEC1, HERC6, IL1R2, MMP8 | 0,866 | 0,858 | 0,875 | 0,903 | 0,844 | 0,963 | 0,959 | 0,917 | 1 |
| OAS1, SIGLEC1, ISG15, HERC6, SLC1A2, IL1R2, RETN | 0,866 | 0,857 | 0,876 | 0,909 | 0,853 | 0,965 | 0,958 | 0,912 | 1 |
| IFIT1, IFI44L, SIGLEC1, ISG15, HERC6, OLAH, RETN | 0,866 | 0,857 | 0,876 | 0,902 | 0,837 | 0,967 | 0,948 | 0,900 | 0,995 |
| IFIT1, SIGLEC1, ISG15, HERC6, IL1R2, OLAH, RETN | 0,866 | 0,857 | 0,876 | 0,905 | 0,844 | 0,967 | 0,950 | 0,904 | 0,996 |
| RSAD2, OAS1, IFIT1, SIGLEC1, SLC1A2, OLAH, MMP8 | 0,866 | 0,857 | 0,876 | 0,911 | 0,855 | 0,967 | 0,957 | 0,913 | 1,000 |
| IFIT1, SIGLEC1, ISG15, HERC6, SLC1A2, OLAH, RETN | 0,866 | 0,856 | 0,876 | 0,912 | 0,856 | 0,968 | 0,945 | 0,895 | 0,994 |
| OAS1, IFIT1, IFI44L, SIGLEC1, HERC6, OLAH, RETN | 0,866 | 0,857 | 0,876 | 0,901 | 0,836 | 0,965 | 0,938 | 0,884 | 0,992 |
| OAS1, IFIT1, IFI44L, SIGLEC1, ISG15, HERC6, IL1R2 | 0,866 | 0,857 | 0,875 | 0,904 | 0,847 | 0,962 | 0,954 | 0,909 | 0,999 |
| RSAD2, OAS1, SIGLEC1, ISG15, HERC6, IL1R2, RETN | 0,866 | 0,856 | 0,876 | 0,903 | 0,842 | 0,965 | 0,962 | 0,921 | 1 |
| IFIT1, ISG15, SLC1A2, IL1R2, OLAH, FAM20A, MMP8 | 0,866 | 0,857 | 0,876 | 0,913 | 0,859 | 0,966 | 0,934 | 0,855 | 1 |
| RSAD2, OAS1, IFIT1, IFI44L, IL1R2, FAM20A, MMP8 | 0,866 | 0,857 | 0,876 | 0,909 | 0,848 | 0,970 | 0,942 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, HERC6, SLC1A2, MMP8 | 0,866 | 0,857 | 0,875 | 0,911 | 0,857 | 0,964 | 0,921 | 0,850 | 0,992 |
| IFIT1, IFI44L, HERC6, IL1R2, OLAH, FAM20A, RETN | 0,866 | 0,857 | 0,876 | 0,905 | 0,844 | 0,967 | 0,934 | 0,857 | 1 |
| RSAD2, IFI27, OAS1, SIGLEC1, ISG15, HERC6, MMP8 | 0,866 | 0,857 | 0,875 | 0,908 | 0,852 | 0,963 | 0,917 | 0,846 | 0,989 |
| RSAD2, OAS1, IFIT1, SIGLEC1, HERC6, IL1R2, MMP8 | 0,866 | 0,857 | 0,876 | 0,905 | 0,848 | 0,962 | 0,961 | 0,921 | 1 |
| OAS1, IFIT1, HERC6, SLC1A2, IL1R2, FAM20A, MMP8 | 0,866 | 0,857 | 0,876 | 0,916 | 0,863 | 0,968 | 0,929 | 0,842 | 1 |
| RSAD2, OAS1, HERC6, IL1R2, OLAH, FAM20A, RETN | 0,866 | 0,856 | 0,877 | 0,906 | 0,845 | 0,967 | 0,937 | 0,863 | 1 |
| RSAD2, OAS1, ISG15, HERC6, SLC1A2, IL1R2, FAM20A | 0,866 | 0,856 | 0,876 | 0,910 | 0,857 | 0,963 | 0,925 | 0,833 | 1 |
| RSAD2, IFI27, IFIT1, IFI44L, HERC6, SLC1A2, FAM20A | 0,866 | 0,856 | 0,877 | 0,914 | 0,858 | 0,970 | 0,923 | 0,839 | 1 |
| RSAD2, IFI27, OAS1, IFIT1, ISG15, HERC6, FAM20A | 0,866 | 0,856 | 0,877 | 0,907 | 0,846 | 0,967 | 0,930 | 0,849 | 1 |
| RSAD2, OAS1, IFI44L, SIGLEC1, HERC6, SLC1A2, FAM20A | 0,866 | 0,856 | 0,876 | 0,910 | 0,853 | 0,966 | 0,938 | 0,862 | 1 |
| OAS1, IFIT1, SIGLEC1, SLC1A2, IL1R2, OLAH, MMP8 | 0,866 | 0,857 | 0,875 | 0,912 | 0,857 | 0,966 | 0,966 | 0,931 | 1 |
| RSAD2, IFIT1, SIGLEC1, SLC1A2, IL1R2, OLAH, MMP8 | 0,866 | 0,857 | 0,875 | 0,912 | 0,858 | 0,967 | 0,965 | 0,929 | 1 |
| RSAD2, SIGLEC1, HERC6, IL1R2, OLAH, RETN, MMP8 | 0,866 | 0,856 | 0,876 | 0,906 | 0,844 | 0,968 | 0,962 | 0,923 | 1 |
| OAS1, IFIT1, SIGLEC1, ISG15, HERC6, SLC1A2, OLAH | 0,866 | 0,856 | 0,876 | 0,913 | 0,858 | 0,968 | 0,946 | 0,897 | 0,995 |
| IFIT1, IFI44L, SIGLEC1, HERC6, IL1R2, OLAH, MMP8 | 0,866 | 0,857 | 0,875 | 0,907 | 0,849 | 0,964 | 0,963 | 0,924 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, HERC6, SLC1A2, RETN, MMP8 | 0,866 | 0,857 | 0,876 | 0,912 | 0,853 | 0,971 | 0,934 | 0,865 | 1 |
| OAS1, IFI44L, ISG15, HERC6, SLC1A2, IL1R2, FAM20A | 0,866 | 0,856 | 0,877 | 0,914 | 0,862 | 0,965 | 0,926 | 0,836 | 1 |
| RSAD2, IFI44L, ISG15, SLC1A2, OLAH, FAM20A, MMP8 | 0,866 | 0,856 | 0,876 | 0,912 | 0,854 | 0,970 | 0,922 | 0,845 | 0,998 |
| IFI44L, SIGLEC1, ISG15, SLC1A2, IL1R2, OLAH, RETN | 0,866 | 0,857 | 0,875 | 0,914 | 0,859 | 0,969 | 0,962 | 0,922 | 1 |
| RSAD2, SIGLEC1, ISG15, HERC6, SLC1A2, IL1R2, OLAH | 0,866 | 0,857 | 0,876 | 0,912 | 0,857 | 0,966 | 0,955 | 0,912 | 0,999 |
| OAS1, IFI44L, SIGLEC1, HERC6, IL1R2, OLAH, MMP8 | 0,866 | 0,857 | 0,875 | 0,903 | 0,844 | 0,962 | 0,963 | 0,924 | 1 |
| RSAD2, OAS1, SIGLEC1, SLC1A2, IL1R2, OLAH, MMP8 | 0,866 | 0,856 | 0,876 | 0,912 | 0,858 | 0,966 | 0,967 | 0,932 | 1 |
| RSAD2, SIGLEC1, ISG15, SLC1A2, IL1R2, OLAH, MMP8 | 0,866 | 0,857 | 0,875 | 0,912 | 0,858 | 0,966 | 0,965 | 0,929 | 1 |
| IFIT1, SIGLEC1, ISG15, HERC6, IL1R2, OLAH, MMP8 | 0,866 | 0,857 | 0,875 | 0,907 | 0,850 | 0,964 | 0,961 | 0,921 | 1 |
| OAS1, IFIT1, IFI44L, SIGLEC1, HERC6, IL1R2, OLAH | 0,866 | 0,857 | 0,875 | 0,905 | 0,846 | 0,964 | 0,952 | 0,907 | 0,997 |
| RSAD2, IFIT1, SIGLEC1, ISG15, HERC6, IL1R2, RETN | 0,866 | 0,857 | 0,875 | 0,905 | 0,843 | 0,967 | 0,952 | 0,907 | 0,998 |
| RSAD2, IFI44L, SIGLEC1, HERC6, IL1R2, RETN, MMP8 | 0,866 | 0,856 | 0,876 | 0,901 | 0,837 | 0,965 | 0,963 | 0,925 | 1 |
| OAS1, IFIT1, SIGLEC1, ISG15, HERC6, IL1R2, MMP8 | 0,866 | 0,857 | 0,875 | 0,906 | 0,850 | 0,962 | 0,958 | 0,914 | 1 |
| IFIT1, IFI44L, ISG15, HERC6, SLC1A2, IL1R2, FAM20A | 0,866 | 0,856 | 0,876 | 0,915 | 0,864 | 0,966 | 0,926 | 0,835 | 1 |
| RSAD2, OAS1, SIGLEC1, SLC1A2, FAM20A, RETN, MMP8 | 0,866 | 0,856 | 0,876 | 0,909 | 0,849 | 0,969 | 0,955 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, SLC1A2, IL1R2, OLAH, MMP8 | 0,866 | 0,857 | 0,875 | 0,912 | 0,858 | 0,966 | 0,966 | 0,931 | 1 |
| RSAD2, OAS1, IFI44L, SIGLEC1, HERC6, IL1R2, MMP8 | 0,866 | 0,857 | 0,875 | 0,902 | 0,843 | 0,962 | 0,962 | 0,922 | 1 |
| SIGLEC1, ISG15, SLC1A2, IL1R2, OLAH, RETN, MMP8 | 0,866 | 0,857 | 0,875 | 0,912 | 0,857 | 0,967 | 0,966 | 0,931 | 1 |
| IFI44L, ISG15, HERC6, SLC1A2, IL1R2, FAM20A, MMP8 | 0,866 | 0,856 | 0,876 | 0,917 | 0,867 | 0,967 | 0,924 | 0,830 | 1 |
| OAS1, IFIT1, IFI44L, IL1R2, OLAH, FAM20A, MMP8 | 0,866 | 0,857 | 0,875 | 0,905 | 0,843 | 0,967 | 0,945 | 0,875 | 1 |
| RSAD2, OAS1, IFIT1, SIGLEC1, SLC1A2, IL1R2, OLAH | 0,866 | 0,857 | 0,875 | 0,913 | 0,857 | 0,968 | 0,967 | 0,931 | 1 |
| IFI44L, SIGLEC1, ISG15, SLC1A2, FAM20A, RETN, MMP8 | 0,866 | 0,856 | 0,876 | 0,904 | 0,841 | 0,967 | 0,953 | 0,882 | 1 |
| RSAD2, IFI44L, ISG15, HERC6, SLC1A2, IL1R2, FAM20A | 0,866 | 0,856 | 0,876 | 0,914 | 0,862 | 0,966 | 0,926 | 0,837 | 1 |
| ISG15, HERC6, SLC1A2, IL1R2, FAM20A, RETN, MMP8 | 0,866 | 0,856 | 0,875 | 0,917 | 0,864 | 0,970 | 0,927 | 0,838 | 1 |
| RSAD2, OAS1, SIGLEC1, ISG15, OLAH, FAM20A, RETN | 0,866 | 0,856 | 0,876 | 0,896 | 0,822 | 0,969 | 0,960 | 0,914 | 1 |
| OAS1, IFIT1, SIGLEC1, ISG15, SLC1A2, OLAH, MMP8 | 0,866 | 0,856 | 0,875 | 0,912 | 0,857 | 0,968 | 0,951 | 0,906 | 0,997 |
| OAS1, IFIT1, ISG15, HERC6, SLC1A2, IL1R2, FAM20A | 0,866 | 0,856 | 0,876 | 0,911 | 0,857 | 0,965 | 0,928 | 0,842 | 1 |
| RSAD2, IFIT1, IFI44L, SIGLEC1, SLC1A2, OLAH, MMP8 | 0,866 | 0,857 | 0,875 | 0,911 | 0,855 | 0,967 | 0,954 | 0,910 | 0,998 |
| RSAD2, OAS1, SIGLEC1, HERC6, IL1R2, OLAH, MMP8 | 0,866 | 0,856 | 0,876 | 0,904 | 0,847 | 0,962 | 0,961 | 0,920 | 1 |
| IFIT1, IFI44L, SIGLEC1, ISG15, SLC1A2, OLAH, RETN | 0,866 | 0,857 | 0,875 | 0,915 | 0,860 | 0,971 | 0,940 | 0,885 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, HERC6, FAM20A, RETN | 0,866 | 0,856 | 0,875 | 0,907 | 0,844 | 0,970 | 0,943 | 0,876 | 1 |
| IFIT1, SIGLEC1, SLC1A2, IL1R2, OLAH, RETN, MMP8 | 0,866 | 0,856 | 0,875 | 0,915 | 0,861 | 0,968 | 0,967 | 0,933 | 1 |
| RSAD2, OAS1, IFI44L, HERC6, SLC1A2, OLAH, FAM20A | 0,866 | 0,855 | 0,876 | 0,912 | 0,856 | 0,968 | 0,924 | 0,845 | 1 |
| RSAD2, IFIT1, IFI44L, SIGLEC1, HERC6, IL1R2, RETN | 0,866 | 0,857 | 0,875 | 0,904 | 0,842 | 0,967 | 0,953 | 0,909 | 0,997 |
| OAS1, IFI44L, ISG15, SLC1A2, OLAH, FAM20A, MMP8 | 0,866 | 0,855 | 0,876 | 0,911 | 0,853 | 0,969 | 0,925 | 0,849 | 1 |
| OAS1, IFIT1, SIGLEC1, ISG15, SLC1A2, IL1R2, OLAH | 0,866 | 0,856 | 0,875 | 0,912 | 0,857 | 0,967 | 0,961 | 0,922 | 1 |
| RSAD2, OAS1, IFIT1, SIGLEC1, HERC6, IL1R2, OLAH | 0,866 | 0,856 | 0,875 | 0,906 | 0,848 | 0,964 | 0,954 | 0,910 | 0,998 |
| IFIT1, SIGLEC1, HERC6, SLC1A2, IL1R2, OLAH, RETN | 0,866 | 0,856 | 0,876 | 0,916 | 0,862 | 0,969 | 0,949 | 0,902 | 0,996 |
| OAS1, IFI44L, HERC6, IL1R2, FAM20A, RETN, MMP8 | 0,866 | 0,856 | 0,875 | 0,909 | 0,848 | 0,970 | 0,938 | 0,857 | 1 |
| RSAD2, IFIT1, HERC6, SLC1A2, IL1R2, FAM20A, MMP8 | 0,866 | 0,856 | 0,875 | 0,915 | 0,863 | 0,967 | 0,929 | 0,842 | 1 |
| RSAD2, IFIT1, IFI44L, SIGLEC1, HERC6, SLC1A2, OLAH | 0,866 | 0,856 | 0,875 | 0,912 | 0,857 | 0,967 | 0,940 | 0,888 | 0,993 |
| RSAD2, OAS1, IFI44L, SIGLEC1, HERC6, IL1R2, RETN | 0,866 | 0,856 | 0,875 | 0,900 | 0,835 | 0,964 | 0,957 | 0,913 | 1 |
| OAS1, SIGLEC1, ISG15, SLC1A2, IL1R2, OLAH, MMP8 | 0,866 | 0,856 | 0,875 | 0,913 | 0,859 | 0,966 | 0,965 | 0,929 | 1 |
| OAS1, IFIT1, HERC6, IL1R2, OLAH, FAM20A, RETN | 0,866 | 0,856 | 0,876 | 0,905 | 0,844 | 0,967 | 0,937 | 0,863 | 1 |
| RSAD2, OAS1, SIGLEC1, HERC6, SLC1A2, IL1R2, RETN | 0,866 | 0,856 | 0,875 | 0,912 | 0,857 | 0,967 | 0,962 | 0,919 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, HERC6, IL1R2, FAM20A, MMP8 | 0,866 | 0,856 | 0,875 | 0,908 | 0,848 | 0,968 | 0,935 | 0,853 | 1 |
| IFIT1, HERC6, SLC1A2, IL1R2, FAM20A, RETN, MMP8 | 0,866 | 0,856 | 0,875 | 0,915 | 0,860 | 0,969 | 0,930 | 0,845 | 1 |
| RSAD2, IFI44L, ISG15, HERC6, SLC1A2, OLAH, FAM20A | 0,866 | 0,855 | 0,876 | 0,913 | 0,858 | 0,969 | 0,925 | 0,844 | 1 |
| RSAD2, IFI27, IFI44L, SIGLEC1, SLC1A2, RETN, MMP8 | 0,866 | 0,856 | 0,875 | 0,915 | 0,859 | 0,971 | 0,954 | 0,899 | 1 |
| OAS1, IFIT1, ISG15, IL1R2, OLAH, FAM20A, MMP8 | 0,866 | 0,856 | 0,875 | 0,907 | 0,845 | 0,969 | 0,943 | 0,871 | 1 |
| OAS1, IFIT1, SIGLEC1, HERC6, SLC1A2, IL1R2, MMP8 | 0,866 | 0,856 | 0,875 | 0,910 | 0,858 | 0,963 | 0,964 | 0,927 | 1 |
| IFI27, OAS1, IFI44L, HERC6, SLC1A2, FAM20A, MMP8 | 0,866 | 0,855 | 0,876 | 0,912 | 0,856 | 0,969 | 0,926 | 0,844 | 1 |
| OAS1, IFIT1, SIGLEC1, SLC1A2, IL1R2, OLAH, RETN | 0,866 | 0,856 | 0,875 | 0,913 | 0,858 | 0,968 | 0,958 | 0,915 | 1 |
| ISG15, HERC6, SLC1A2, IL1R2, OLAH, FAM20A, MMP8 | 0,866 | 0,856 | 0,875 | 0,915 | 0,865 | 0,966 | 0,926 | 0,839 | 1 |
| IFI44L, SIGLEC1, ISG15, HERC6, IL1R2, OLAH, MMP8 | 0,866 | 0,857 | 0,875 | 0,903 | 0,844 | 0,962 | 0,963 | 0,924 | 1 |
| RSAD2, IFIT1, IFI44L, SIGLEC1, ISG15, HERC6, IL1R2 | 0,866 | 0,857 | 0,874 | 0,901 | 0,840 | 0,962 | 0,952 | 0,907 | 0,997 |
| RSAD2, IFI27, OAS1, ISG15, SLC1A2, FAM20A, RETN | 0,866 | 0,855 | 0,876 | 0,911 | 0,850 | 0,972 | 0,941 | 0,861 | 1 |
| OAS1, IFIT1, SIGLEC1, SLC1A2, OLAH, RETN, MMP8 | 0,866 | 0,856 | 0,875 | 0,912 | 0,856 | 0,968 | 0,959 | 0,917 | 1 |
| RSAD2, SIGLEC1, HERC6, SLC1A2, IL1R2, OLAH, RETN | 0,866 | 0,855 | 0,876 | 0,914 | 0,860 | 0,968 | 0,955 | 0,912 | 0,999 |
| SIGLEC1, HERC6, SLC1A2, IL1R2, OLAH, RETN, MMP8 | 0,865 | 0,856 | 0,875 | 0,913 | 0,858 | 0,968 | 0,967 | 0,933 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFI44L, SIGLEC1, ISG15, HERC6, IL1R2, RETN | 0,865 | 0,856 | 0,875 | 0,904 | 0,843 | 0,965 | 0,954 | 0,909 | 1,000 |
| OAS1, ISG15, HERC6, SLC1A2, IL1R2, FAM20A, RETN | 0,865 | 0,855 | 0,876 | 0,912 | 0,857 | 0,966 | 0,927 | 0,839 | 1 |
| RSAD2, IFIT1, SIGLEC1, HERC6, SLC1A2, OLAH, RETN | 0,865 | 0,856 | 0,875 | 0,912 | 0,856 | 0,968 | 0,943 | 0,893 | 0,994 |
| IFI27, OAS1, IFIT1, SLC1A2, FAM20A, RETN, MMP8 | 0,865 | 0,855 | 0,876 | 0,908 | 0,845 | 0,971 | 0,941 | 0,861 | 1 |
| RSAD2, OAS1, IL1R2, OLAH, FAM20A, RETN, MMP8 | 0,865 | 0,855 | 0,875 | 0,907 | 0,845 | 0,968 | 0,938 | 0,861 | 1 |
| RSAD2, IFI27, IFIT1, ISG15, SLC1A2, FAM20A, RETN | 0,865 | 0,855 | 0,876 | 0,908 | 0,847 | 0,969 | 0,940 | 0,859 | 1 |
| RSAD2, IFIT1, HERC6, IL1R2, FAM20A, RETN, MMP8 | 0,865 | 0,856 | 0,875 | 0,906 | 0,844 | 0,969 | 0,941 | 0,863 | 1 |
| IFI44L, SIGLEC1, ISG15, HERC6, SLC1A2, OLAH, MMP8 | 0,865 | 0,856 | 0,875 | 0,911 | 0,856 | 0,967 | 0,951 | 0,905 | 0,997 |
| RSAD2, IFIT1, SIGLEC1, ISG15, SLC1A2, OLAH, MMP8 | 0,865 | 0,856 | 0,875 | 0,912 | 0,857 | 0,968 | 0,952 | 0,907 | 0,997 |
| RSAD2, IFIT1, SIGLEC1, ISG15, HERC6, SLC1A2, OLAH | 0,865 | 0,856 | 0,875 | 0,913 | 0,858 | 0,968 | 0,945 | 0,895 | 0,994 |
| RSAD2, IFI44L, HERC6, SLC1A2, IL1R2, FAM20A, MMP8 | 0,865 | 0,856 | 0,875 | 0,919 | 0,869 | 0,968 | 0,924 | 0,830 | 1 |
| OAS1, IFI44L, HERC6, SLC1A2, IL1R2, FAM20A, MMP8 | 0,865 | 0,855 | 0,875 | 0,916 | 0,866 | 0,966 | 0,923 | 0,827 | 1 |
| IFIT1, IFI44L, SIGLEC1, ISG15, HERC6, IL1R2, OLAH | 0,865 | 0,856 | 0,874 | 0,906 | 0,848 | 0,964 | 0,954 | 0,910 | 0,998 |
| RSAD2, SIGLEC1, HERC6, SLC1A2, IL1R2, RETN, MMP8 | 0,865 | 0,856 | 0,875 | 0,910 | 0,854 | 0,965 | 0,964 | 0,926 | 1 |
| OAS1, IFIT1, IFI44L, SIGLEC1, SLC1A2, OLAH, RETN | 0,865 | 0,856 | 0,875 | 0,915 | 0,860 | 0,971 | 0,940 | 0,885 | 0,995 |

(suite)

| Modèle | AUC modèle | IC 95% | | AUC Train | IC 95% Train | | AUC Test | IC 95% Test | |
|---|---|---|---|---|---|---|---|---|---|
| ISG15, HERC6, SLC1A2, IL1R2, OLAH, FAM20A, RETN | 0,865 | 0,855 | 0,876 | 0,913 | 0,858 | 0,968 | 0,926 | 0,840 | 1 |
| OAS1, SIGLEC1, ISG15, HERC6, SLC1A2, FAM20A, MMP8 | 0,865 | 0,855 | 0,876 | 0,908 | 0,850 | 0,965 | 0,942 | 0,868 | 1 |
| OAS1, SIGLEC1, HERC6, SLC1A2, IL1R2, OLAH, RETN | 0,865 | 0,855 | 0,876 | 0,915 | 0,862 | 0,969 | 0,957 | 0,913 | 1,000 |
| OAS1, IFI44L, SIGLEC1, HERC6, SLC1A2, FAM20A, RETN | 0,865 | 0,855 | 0,875 | 0,910 | 0,852 | 0,967 | 0,943 | 0,871 | 1 |
| RSAD2, SIGLEC1, HERC6, SLC1A2, OLAH, RETN, MMP8 | 0,865 | 0,855 | 0,875 | 0,910 | 0,853 | 0,968 | 0,952 | 0,907 | 0,997 |
| IFIT1, SIGLEC1, ISG15, HERC6, SLC1A2, IL1R2, RETN | 0,865 | 0,856 | 0,875 | 0,909 | 0,854 | 0,964 | 0,955 | 0,912 | 0,998 |
| OAS1, SIGLEC1, ISG15, SLC1A2, FAM20A, RETN, MMP8 | 0,865 | 0,855 | 0,876 | 0,904 | 0,840 | 0,967 | 0,950 | 0,880 | 1 |
| RSAD2, SIGLEC1, ISG15, SLC1A2, FAM20A, RETN, MMP8 | 0,865 | 0,855 | 0,875 | 0,908 | 0,848 | 0,968 | 0,953 | 0,885 | 1 |
| RSAD2, SIGLEC1, ISG15, HERC6, SLC1A2, OLAH, MMP8 | 0,865 | 0,856 | 0,875 | 0,911 | 0,856 | 0,967 | 0,948 | 0,900 | 0,995 |
| RSAD2, SIGLEC1, SLC1A2, IL1R2, OLAH, RETN, MMP8 | 0,865 | 0,856 | 0,875 | 0,914 | 0,859 | 0,968 | 0,968 | 0,935 | 1 |
| OAS1, SIGLEC1, ISG15, HERC6, SLC1A2, OLAH, RETN | 0,865 | 0,855 | 0,875 | 0,913 | 0,856 | 0,969 | 0,950 | 0,900 | 1 |
| RSAD2, OAS1, SIGLEC1, ISG15, HERC6, SLC1A2, OLAH | 0,865 | 0,855 | 0,875 | 0,912 | 0,856 | 0,967 | 0,951 | 0,904 | 0,998 |
| IFI27, IFIT1, ISG15, SLC1A2, FAM20A, RETN, MMP8 | 0,865 | 0,854 | 0,876 | 0,908 | 0,845 | 0,971 | 0,941 | 0,861 | 1 |
| RSAD2, HERC6, SLC1A2, IL1R2, OLAH, FAM20A, RETN | 0,865 | 0,855 | 0,875 | 0,913 | 0,858 | 0,967 | 0,927 | 0,842 | 1 |
| IFI27, OAS1, ISG15, HERC6, SLC1A2, FAM20A, MMP8 | 0,865 | 0,855 | 0,876 | 0,915 | 0,861 | 0,969 | 0,924 | 0,839 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, SLC1A2, IL1R2, OLAH | 0,865 | 0,856 | 0,874 | 0,911 | 0,856 | 0,967 | 0,963 | 0,925 | 1 |
| IFIT1, IFI44L, SLC1A2, IL1R2, OLAH, FAM20A, RETN | 0,865 | 0,855 | 0,875 | 0,913 | 0,858 | 0,968 | 0,926 | 0,846 | 1 |
| IFIT1, SLC1A2, IL1R2, OLAH, FAM20A, RETN, MMP8 | 0,865 | 0,855 | 0,875 | 0,913 | 0,859 | 0,967 | 0,938 | 0,864 | 1 |
| IFI44L, SIGLEC1, ISG15, SLC1A2, OLAH, RETN, MMP8 | 0,865 | 0,856 | 0,875 | 0,910 | 0,852 | 0,967 | 0,959 | 0,917 | 1,000 |
| IFI44L, ISG15, HERC6, SLC1A2, OLAH, FAM20A, RETN | 0,865 | 0,855 | 0,876 | 0,913 | 0,856 | 0,969 | 0,932 | 0,854 | 1 |
| RSAD2, OAS1, SIGLEC1, ISG15, HERC6, SLC1A2, IL1R2 | 0,865 | 0,856 | 0,875 | 0,909 | 0,855 | 0,963 | 0,966 | 0,929 | 1 |
| HERC6, SLC1A2, IL1R2, OLAH, FAM20A, RETN, MMP8 | 0,865 | 0,855 | 0,875 | 0,914 | 0,862 | 0,967 | 0,925 | 0,838 | 1 |
| IFIT1, IFI44L, SIGLEC1, ISG15, SLC1A2, IL1R2, OLAH | 0,865 | 0,856 | 0,874 | 0,914 | 0,859 | 0,968 | 0,961 | 0,922 | 1 |
| OAS1, IFIT1, IFI44L, SIGLEC1, ISG15, SLC1A2, OLAH | 0,865 | 0,856 | 0,875 | 0,912 | 0,856 | 0,968 | 0,945 | 0,894 | 0,995 |
| OAS1, SIGLEC1, SLC1A2, IL1R2, OLAH, RETN, MMP8 | 0,865 | 0,855 | 0,875 | 0,914 | 0,860 | 0,968 | 0,966 | 0,931 | 1 |
| IFI44L, HERC6, SLC1A2, IL1R2, FAM20A, RETN, MMP8 | 0,865 | 0,855 | 0,875 | 0,917 | 0,865 | 0,969 | 0,928 | 0,837 | 1 |
| OAS1, ISG15, HERC6, SLC1A2, IL1R2, FAM20A, MMP8 | 0,865 | 0,855 | 0,875 | 0,914 | 0,862 | 0,966 | 0,924 | 0,831 | 1 |
| RSAD2, IFI44L, ISG15, SLC1A2, IL1R2, OLAH, FAM20A | 0,865 | 0,855 | 0,875 | 0,912 | 0,856 | 0,968 | 0,929 | 0,852 | 1 |
| RSAD2, OAS1, IFIT1, HERC6, IL1R2, FAM20A, RETN | 0,865 | 0,855 | 0,875 | 0,905 | 0,842 | 0,967 | 0,937 | 0,856 | 1 |
| IFIT1, IFI44L, ISG15, SLC1A2, OLAH, FAM20A, RETN | 0,865 | 0,855 | 0,875 | 0,910 | 0,851 | 0,969 | 0,918 | 0,841 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, HERC6, IL1R2, OLAH | 0,865 | 0,855 | 0,875 | 0,903 | 0,843 | 0,964 | 0,959 | 0,917 | 1 |
| OAS1, IFI44L, SIGLEC1, ISG15, HERC6, IL1R2, MMP8 | 0,865 | 0,856 | 0,874 | 0,902 | 0,843 | 0,961 | 0,960 | 0,919 | 1 |
| RSAD2, OAS1, IFIT1, IFI44L, HERC6, OLAH, FAM20A | 0,865 | 0,855 | 0,875 | 0,903 | 0,841 | 0,966 | 0,933 | 0,863 | 1 |
| RSAD2, OAS1, IFI44L, SIGLEC1, OLAH, FAM20A, RETN | 0,865 | 0,855 | 0,875 | 0,896 | 0,824 | 0,969 | 0,958 | 0,911 | 1 |
| RSAD2, IFIT1, IFI44L, SIGLEC1, HERC6, IL1R2, OLAH | 0,865 | 0,856 | 0,874 | 0,905 | 0,845 | 0,965 | 0,954 | 0,910 | 0,998 |
| IFIT1, ISG15, HERC6, OLAH, FAM20A, RETN, MMP8 | 0,865 | 0,855 | 0,875 | 0,904 | 0,840 | 0,967 | 0,948 | 0,883 | 1 |
| IFI27, IFIT1, ISG15, HERC6, SLC1A2, FAM20A, MMP8 | 0,865 | 0,855 | 0,875 | 0,910 | 0,853 | 0,967 | 0,927 | 0,843 | 1 |
| RSAD2, OAS1, IFIT1, ISG15, IL1R2, FAM20A, MMP8 | 0,865 | 0,855 | 0,875 | 0,907 | 0,845 | 0,969 | 0,945 | 0,872 | 1 |
| IFI27, IFIT1, SIGLEC1, ISG15, HERC6, SLC1A2, MMP8 | 0,865 | 0,856 | 0,874 | 0,910 | 0,856 | 0,964 | 0,917 | 0,848 | 0,987 |
| RSAD2, IFI44L, SIGLEC1, HERC6, IL1R2, OLAH, MMP8 | 0,865 | 0,856 | 0,874 | 0,902 | 0,842 | 0,962 | 0,962 | 0,922 | 1 |
| RSAD2, OAS1, IFI44L, SIGLEC1, ISG15, HERC6, IL1R2 | 0,865 | 0,856 | 0,874 | 0,902 | 0,842 | 0,962 | 0,960 | 0,919 | 1 |
| RSAD2, OAS1, IFIT1, SIGLEC1, HERC6, OLAH, RETN | 0,865 | 0,855 | 0,875 | 0,902 | 0,838 | 0,966 | 0,940 | 0,888 | 0,993 |
| RSAD2, OAS1, IFIT1, SIGLEC1, ISG15, HERC6, IL1R2 | 0,865 | 0,856 | 0,874 | 0,905 | 0,848 | 0,962 | 0,957 | 0,913 | 1 |
| IFIT1, SIGLEC1, ISG15, SLC1A2, OLAH, RETN, MMP8 | 0,865 | 0,855 | 0,874 | 0,911 | 0,855 | 0,968 | 0,959 | 0,917 | 1 |
| RSAD2, IFI44L, SIGLEC1, HERC6, SLC1A2, FAM20A, RETN | 0,865 | 0,855 | 0,875 | 0,912 | 0,855 | 0,969 | 0,943 | 0,870 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, SIGLEC1, ISG15, HERC6, SLC1A2, IL1R2 | 0,865 | 0,855 | 0,874 | 0,910 | 0,858 | 0,963 | 0,959 | 0,918 | 1,000 |
| RSAD2, IFI44L, SIGLEC1, HERC6, SLC1A2, FAM20A, MMP8 | 0,865 | 0,855 | 0,875 | 0,909 | 0,852 | 0,966 | 0,937 | 0,860 | 1 |
| IFIT1, IFI44L, SIGLEC1, HERC6, SLC1A2, IL1R2, OLAH | 0,865 | 0,855 | 0,874 | 0,913 | 0,859 | 0,967 | 0,953 | 0,909 | 0,997 |
| RSAD2, IFI44L, SIGLEC1, SLC1A2, IL1R2, OLAH, MMP8 | 0,865 | 0,856 | 0,874 | 0,912 | 0,858 | 0,966 | 0,967 | 0,933 | 1 |
| OAS1, IFIT1, IFI44L, HERC6, OLAH, FAM20A, RETN | 0,865 | 0,855 | 0,874 | 0,903 | 0,840 | 0,967 | 0,935 | 0,865 | 1 |
| RSAD2, IFI27, IFIT1, SIGLEC1, ISG15, HERC6, MMP8 | 0,865 | 0,856 | 0,873 | 0,906 | 0,850 | 0,962 | 0,905 | 0,830 | 0,981 |
| IFIT1, IFI44L, SIGLEC1, HERC6, IL1R2, OLAH, RETN | 0,865 | 0,855 | 0,874 | 0,905 | 0,843 | 0,967 | 0,949 | 0,902 | 0,996 |
| OAS1, IFIT1, IFI44L, HERC6, OLAH, FAM20A, MMP8 | 0,865 | 0,855 | 0,875 | 0,902 | 0,839 | 0,965 | 0,938 | 0,870 | 1 |
| OAS1, IFIT1, SIGLEC1, ISG15, HERC6, RETN, MMP8 | 0,865 | 0,855 | 0,875 | 0,903 | 0,839 | 0,966 | 0,934 | 0,877 | 0,991 |
| OAS1, ISG15, HERC6, SLC1A2, OLAH, FAM20A, RETN | 0,865 | 0,854 | 0,875 | 0,911 | 0,854 | 0,968 | 0,934 | 0,855 | 1 |
| RSAD2, OAS1, IFIT1, IFI44L, SIGLEC1, HERC6, RETN | 0,865 | 0,855 | 0,874 | 0,900 | 0,833 | 0,967 | 0,925 | 0,865 | 0,985 |
| OAS1, ISG15, SLC1A2, IL1R2, OLAH, FAM20A, MMP8 | 0,865 | 0,855 | 0,874 | 0,915 | 0,862 | 0,968 | 0,926 | 0,842 | 1 |
| RSAD2, OAS1, SIGLEC1, ISG15, HERC6, IL1R2, MMP8 | 0,865 | 0,855 | 0,874 | 0,904 | 0,847 | 0,961 | 0,965 | 0,928 | 1 |
| OAS1, IFIT1, ISG15, HERC6, OLAH, FAM20A, RETN | 0,865 | 0,855 | 0,875 | 0,903 | 0,840 | 0,965 | 0,942 | 0,875 | 1 |
| RSAD2, IFIT1, SIGLEC1, HERC6, IL1R2, OLAH, RETN | 0,865 | 0,855 | 0,874 | 0,904 | 0,841 | 0,967 | 0,949 | 0,902 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, HERC6, IL1R2, OLAH, MMP8 | 0,865 | 0,855 | 0,874 | 0,906 | 0,849 | 0,964 | 0,959 | 0,917 | 1 |
| IFI44L, ISG15, HERC6, SLC1A2, IL1R2, FAM20A, RETN | 0,865 | 0,855 | 0,875 | 0,913 | 0,859 | 0,967 | 0,928 | 0,841 | 1 |
| RSAD2, OAS1, IFI44L, HERC6, SLC1A2, IL1R2, FAM20A | 0,865 | 0,855 | 0,875 | 0,913 | 0,860 | 0,965 | 0,924 | 0,834 | 1 |
| IFI44L, SIGLEC1, ISG15, HERC6, SLC1A2, IL1R2, OLAH | 0,865 | 0,855 | 0,874 | 0,911 | 0,856 | 0,966 | 0,955 | 0,912 | 0,998 |
| RSAD2, OAS1, SIGLEC1, HERC6, SLC1A2, OLAH, RETN | 0,865 | 0,855 | 0,875 | 0,913 | 0,856 | 0,970 | 0,952 | 0,903 | 1 |
| OAS1, IFI44L, HERC6, IL1R2, OLAH, FAM20A, RETN | 0,865 | 0,855 | 0,875 | 0,905 | 0,844 | 0,967 | 0,939 | 0,867 | 1 |
| OAS1, IFIT1, IFI44L, HERC6, IL1R2, FAM20A, RETN | 0,865 | 0,855 | 0,874 | 0,906 | 0,844 | 0,968 | 0,936 | 0,855 | 1 |
| RSAD2, OAS1, SIGLEC1, SLC1A2, IL1R2, OLAH, RETN | 0,865 | 0,855 | 0,874 | 0,913 | 0,857 | 0,968 | 0,967 | 0,929 | 1 |
| RSAD2, OAS1, HERC6, SLC1A2, IL1R2, FAM20A, MMP8 | 0,865 | 0,855 | 0,874 | 0,914 | 0,862 | 0,966 | 0,925 | 0,832 | 1 |
| OAS1, IFIT1, SIGLEC1, ISG15, HERC6, IL1R2, OLAH | 0,865 | 0,855 | 0,874 | 0,907 | 0,850 | 0,965 | 0,953 | 0,909 | 0,998 |
| RSAD2, IFI27, OAS1, IFIT1, SLC1A2, FAM20A, RETN | 0,865 | 0,855 | 0,875 | 0,908 | 0,847 | 0,969 | 0,940 | 0,860 | 1 |
| RSAD2, IFI44L, SIGLEC1, ISG15, HERC6, FAM20A, MMP8 | 0,865 | 0,855 | 0,875 | 0,903 | 0,838 | 0,967 | 0,943 | 0,875 | 1 |
| OAS1, IFI44L, SIGLEC1, ISG15, OLAH, FAM20A, RETN | 0,865 | 0,854 | 0,875 | 0,896 | 0,824 | 0,968 | 0,961 | 0,915 | 1 |
| IFIT1, SIGLEC1, ISG15, SLC1A2, IL1R2, OLAH, RETN | 0,865 | 0,855 | 0,874 | 0,913 | 0,858 | 0,968 | 0,959 | 0,916 | 1 |
| IFI44L, ISG15, SLC1A2, IL1R2, FAM20A, RETN, MMP8 | 0,865 | 0,856 | 0,874 | 0,913 | 0,861 | 0,965 | 0,927 | 0,836 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, HERC6, MMP8 | 0,865 | 0,856 | 0,873 | 0,906 | 0,850 | 0,962 | 0,904 | 0,829 | 0,980 |
| RSAD2, IFI27, IFIT1, SIGLEC1, HERC6, SLC1A2, MMP8 | 0,865 | 0,856 | 0,873 | 0,909 | 0,854 | 0,964 | 0,916 | 0,846 | 0,986 |
| RSAD2, OAS1, HERC6, SLC1A2, OLAH, FAM20A, MMP8 | 0,865 | 0,855 | 0,874 | 0,912 | 0,857 | 0,967 | 0,932 | 0,852 | 1 |
| RSAD2, IFI27, OAS1, IFI44L, HERC6, SLC1A2, FAM20A | 0,865 | 0,854 | 0,876 | 0,914 | 0,857 | 0,971 | 0,926 | 0,843 | 1 |
| RSAD2, IFIT1, ISG15, HERC6, SLC1A2, IL1R2, FAM20A | 0,865 | 0,855 | 0,874 | 0,912 | 0,858 | 0,965 | 0,928 | 0,842 | 1 |
| OAS1, IFIT1, SIGLEC1, ISG15, HERC6, SLC1A2, RETN | 0,864 | 0,855 | 0,874 | 0,912 | 0,856 | 0,968 | 0,929 | 0,869 | 0,990 |
| SIGLEC1, ISG15, HERC6, IL1R2, OLAH, RETN, MMP8 | 0,864 | 0,855 | 0,874 | 0,901 | 0,836 | 0,965 | 0,968 | 0,935 | 1 |
| RSAD2, OAS1, IFIT1, IFI44L, SIGLEC1, SLC1A2, OLAH | 0,864 | 0,855 | 0,874 | 0,912 | 0,856 | 0,968 | 0,952 | 0,904 | 1 |
| OAS1, SIGLEC1, ISG15, HERC6, SLC1A2, IL1R2, MMP8 | 0,864 | 0,855 | 0,874 | 0,910 | 0,858 | 0,963 | 0,966 | 0,930 | 1 |
| IFIT1, ISG15, HERC6, SLC1A2, IL1R2, FAM20A, RETN | 0,864 | 0,854 | 0,875 | 0,911 | 0,855 | 0,966 | 0,930 | 0,845 | 1 |
| RSAD2, SIGLEC1, ISG15, HERC6, IL1R2, OLAH, MMP8 | 0,864 | 0,855 | 0,874 | 0,905 | 0,847 | 0,963 | 0,960 | 0,919 | 1 |
| RSAD2, IFIT1, SIGLEC1, HERC6, OLAH, RETN, MMP8 | 0,864 | 0,854 | 0,874 | 0,901 | 0,836 | 0,967 | 0,957 | 0,915 | 0,998 |
| RSAD2, OAS1, IFIT1, SIGLEC1, HERC6, SLC1A2, IL1R2 | 0,864 | 0,855 | 0,874 | 0,908 | 0,853 | 0,962 | 0,965 | 0,928 | 1 |
| RSAD2, IFI27, IFIT1, SLC1A2, FAM20A, RETN, MMP8 | 0,864 | 0,854 | 0,875 | 0,908 | 0,845 | 0,971 | 0,941 | 0,861 | 1 |
| RSAD2, ISG15, HERC6, SLC1A2, IL1R2, FAM20A, RETN | 0,864 | 0,854 | 0,875 | 0,911 | 0,857 | 0,966 | 0,929 | 0,841 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, HERC6, SLC1A2, IL1R2, RETN | 0,864 | 0,855 | 0,874 | 0,909 | 0,853 | 0,965 | 0,951 | 0,906 | 0,997 |
| IFI44L, SIGLEC1, ISG15, HERC6, SLC1A2, FAM20A, MMP8 | 0,864 | 0,854 | 0,874 | 0,902 | 0,840 | 0,965 | 0,952 | 0,879 | 1 |
| OAS1, IFIT1, HERC6, SLC1A2, OLAH, FAM20A, MMP8 | 0,864 | 0,854 | 0,875 | 0,909 | 0,853 | 0,965 | 0,932 | 0,853 | 1 |
| OAS1, IFI44L, SIGLEC1, HERC6, SLC1A2, OLAH, MMP8 | 0,864 | 0,854 | 0,874 | 0,911 | 0,855 | 0,967 | 0,954 | 0,910 | 0,999 |
| RSAD2, OAS1, IFI44L, SLC1A2, IL1R2, OLAH, FAM20A | 0,864 | 0,854 | 0,875 | 0,913 | 0,857 | 0,969 | 0,930 | 0,852 | 1 |
| OAS1, SIGLEC1, ISG15, SLC1A2, OLAH, RETN, MMP8 | 0,864 | 0,854 | 0,874 | 0,911 | 0,854 | 0,968 | 0,960 | 0,919 | 1 |
| OAS1, IFIT1, SIGLEC1, ISG15, SLC1A2, OLAH, RETN | 0,864 | 0,854 | 0,874 | 0,914 | 0,858 | 0,969 | 0,943 | 0,892 | 0,995 |
| RSAD2, SIGLEC1, ISG15, SLC1A2, IL1R2, OLAH, RETN | 0,864 | 0,855 | 0,874 | 0,914 | 0,859 | 0,969 | 0,960 | 0,919 | 1 |
| RSAD2, OAS1, SIGLEC1, ISG15, SLC1A2, OLAH, MMP8 | 0,864 | 0,855 | 0,874 | 0,911 | 0,856 | 0,967 | 0,953 | 0,908 | 0,998 |
| OAS1, IFI44L, SIGLEC1, ISG15, HERC6, SLC1A2, FAM20A | 0,864 | 0,854 | 0,874 | 0,909 | 0,852 | 0,966 | 0,942 | 0,868 | 1 |
| RSAD2, OAS1, IFIT1, SIGLEC1, ISG15, SLC1A2, OLAH | 0,864 | 0,855 | 0,874 | 0,911 | 0,855 | 0,968 | 0,949 | 0,900 | 0,998 |
| RSAD2, OAS1, IFIT1, SLC1A2, IL1R2, FAM20A, MMP8 | 0,864 | 0,855 | 0,873 | 0,914 | 0,860 | 0,967 | 0,936 | 0,855 | 1 |
| OAS1, IFI44L, ISG15, IL1R2, OLAH, FAM20A, MMP8 | 0,864 | 0,854 | 0,874 | 0,905 | 0,843 | 0,968 | 0,943 | 0,872 | 1 |
| RSAD2, OAS1, SIGLEC1, ISG15, SLC1A2, IL1R2, OLAH | 0,864 | 0,855 | 0,874 | 0,912 | 0,856 | 0,967 | 0,966 | 0,929 | 1 |
| IFI44L, SIGLEC1, HERC6, SLC1A2, IL1R2, OLAH, RETN | 0,864 | 0,854 | 0,874 | 0,914 | 0,859 | 0,969 | 0,959 | 0,917 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, HERC6, IL1R2, OLAH | 0,864 | 0,854 | 0,874 | 0,907 | 0,849 | 0,964 | 0,963 | 0,924 | 1 |
| RSAD2, IFI44L, SIGLEC1, ISG15, HERC6, IL1R2, MMP8 | 0,864 | 0,855 | 0,873 | 0,902 | 0,842 | 0,962 | 0,960 | 0,919 | 1 |
| IFI44L, SIGLEC1, HERC6, SLC1A2, IL1R2, RETN, MMP8 | 0,864 | 0,855 | 0,873 | 0,910 | 0,854 | 0,965 | 0,966 | 0,931 | 1 |
| RSAD2, OAS1, ISG15, HERC6, SLC1A2, OLAH, FAM20A | 0,864 | 0,854 | 0,874 | 0,911 | 0,855 | 0,967 | 0,929 | 0,849 | 1 |
| IFIT1, HERC6, SLC1A2, OLAH, FAM20A, RETN, MMP8 | 0,864 | 0,854 | 0,875 | 0,908 | 0,850 | 0,967 | 0,933 | 0,854 | 1 |
| OAS1, SIGLEC1, ISG15, HERC6, IL1R2, OLAH, MMP8 | 0,864 | 0,855 | 0,874 | 0,905 | 0,847 | 0,962 | 0,963 | 0,924 | 1 |
| OAS1, IFIT1, IL1R2, OLAH, FAM20A, RETN, MMP8 | 0,864 | 0,854 | 0,874 | 0,905 | 0,841 | 0,968 | 0,946 | 0,875 | 1 |
| RSAD2, IFI27, ISG15, HERC6, SLC1A2, FAM20A, MMP8 | 0,864 | 0,854 | 0,875 | 0,913 | 0,858 | 0,969 | 0,927 | 0,843 | 1 |
| RSAD2, OAS1, IFI44L, SIGLEC1, FAM20A, RETN, MMP8 | 0,864 | 0,854 | 0,875 | 0,903 | 0,835 | 0,970 | 0,953 | 0,889 | 1 |
| SIGLEC1, ISG15, HERC6, SLC1A2, OLAH, RETN, MMP8 | 0,864 | 0,854 | 0,874 | 0,910 | 0,850 | 0,969 | 0,958 | 0,916 | 0,999 |
| OAS1, IFIT1, ISG15, SLC1A2, IL1R2, OLAH, FAM20A | 0,864 | 0,854 | 0,874 | 0,911 | 0,853 | 0,968 | 0,935 | 0,856 | 1 |
| OAS1, IFI44L, HERC6, SLC1A2, OLAH, FAM20A, RETN | 0,864 | 0,853 | 0,875 | 0,912 | 0,857 | 0,968 | 0,924 | 0,843 | 1 |
| RSAD2, IFI27, OAS1, IFI44L, ISG15, FAM20A, MMP8 | 0,864 | 0,853 | 0,875 | 0,901 | 0,834 | 0,967 | 0,934 | 0,855 | 1 |
| RSAD2, IFIT1, IFI44L, SLC1A2, IL1R2, OLAH, FAM20A | 0,864 | 0,855 | 0,873 | 0,913 | 0,858 | 0,969 | 0,928 | 0,850 | 1 |
| OAS1, IFI44L, SIGLEC1, ISG15, HERC6, SLC1A2, OLAH | 0,864 | 0,854 | 0,874 | 0,912 | 0,857 | 0,968 | 0,951 | 0,904 | 0,998 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, HERC6, SLC1A2, IL1R2, OLAH, FAM20A | 0,864 | 0,854 | 0,874 | 0,912 | 0,857 | 0,967 | 0,927 | 0,843 | 1 |
| OAS1, IFI44L, ISG15, SLC1A2, IL1R2, OLAH, FAM20A | 0,864 | 0,854 | 0,874 | 0,913 | 0,857 | 0,969 | 0,929 | 0,851 | 1 |
| OAS1, IFIT1, IFI44L, HERC6, SLC1A2, IL1R2, FAM20A | 0,864 | 0,854 | 0,874 | 0,913 | 0,861 | 0,965 | 0,924 | 0,832 | 1 |
| IFI27, OAS1, ISG15, SLC1A2, FAM20A, RETN, MMP8 | 0,864 | 0,853 | 0,875 | 0,911 | 0,850 | 0,972 | 0,942 | 0,863 | 1 |
| RSAD2, IFI44L, HERC6, IL1R2, OLAH, FAM20A, RETN | 0,864 | 0,854 | 0,874 | 0,906 | 0,846 | 0,967 | 0,938 | 0,865 | 1 |
| IFIT1, IFI44L, ISG15, IL1R2, OLAH, FAM20A, MMP8 | 0,864 | 0,855 | 0,873 | 0,906 | 0,845 | 0,967 | 0,950 | 0,888 | 1 |
| OAS1, IFI44L, SIGLEC1, SLC1A2, FAM20A, RETN, MMP8 | 0,864 | 0,854 | 0,874 | 0,905 | 0,843 | 0,968 | 0,950 | 0,880 | 1 |
| RSAD2, OAS1, IFI44L, HERC6, IL1R2, FAM20A, RETN | 0,864 | 0,854 | 0,874 | 0,908 | 0,847 | 0,969 | 0,936 | 0,858 | 1 |
| RSAD2, ISG15, SLC1A2, IL1R2, OLAH, FAM20A, MMP8 | 0,864 | 0,855 | 0,873 | 0,914 | 0,862 | 0,967 | 0,929 | 0,844 | 1 |
| RSAD2, OAS1, SIGLEC1, SLC1A2, OLAH, RETN, MMP8 | 0,864 | 0,854 | 0,874 | 0,912 | 0,854 | 0,969 | 0,962 | 0,921 | 1 |
| IFIT1, IFI44L, ISG15, SLC1A2, IL1R2, FAM20A, MMP8 | 0,864 | 0,855 | 0,873 | 0,912 | 0,859 | 0,964 | 0,930 | 0,843 | 1 |
| IFIT1, IFI44L, SIGLEC1, HERC6, SLC1A2, IL1R2, MMP8 | 0,864 | 0,855 | 0,873 | 0,908 | 0,855 | 0,962 | 0,964 | 0,927 | 1 |
| RSAD2, OAS1, IFIT1, ISG15, IL1R2, OLAH, FAM20A | 0,864 | 0,854 | 0,874 | 0,901 | 0,835 | 0,968 | 0,945 | 0,873 | 1 |
| IFI27, OAS1, IFIT1, HERC6, SLC1A2, FAM20A, MMP8 | 0,864 | 0,854 | 0,874 | 0,914 | 0,859 | 0,969 | 0,925 | 0,840 | 1 |
| RSAD2, IFI44L, SIGLEC1, ISG15, HERC6, IL1R2, RETN | 0,864 | 0,854 | 0,874 | 0,901 | 0,838 | 0,964 | 0,958 | 0,916 | 1,000 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, SIGLEC1, HERC6, SLC1A2, MMP8 | 0,864 | 0,855 | 0,873 | 0,909 | 0,854 | 0,963 | 0,920 | 0,850 | 0,989 |
| IFIT1, ISG15, HERC6, SLC1A2, IL1R2, FAM20A, MMP8 | 0,864 | 0,854 | 0,874 | 0,915 | 0,863 | 0,967 | 0,929 | 0,842 | 1 |
| RSAD2, IFIT1, SIGLEC1, SLC1A2, OLAH, RETN, MMP8 | 0,864 | 0,854 | 0,874 | 0,913 | 0,857 | 0,969 | 0,960 | 0,919 | 1 |
| IFIT1, IFI44L, SIGLEC1, HERC6, SLC1A2, IL1R2, RETN | 0,864 | 0,855 | 0,873 | 0,910 | 0,855 | 0,965 | 0,950 | 0,904 | 0,996 |
| RSAD2, OAS1, ISG15, SLC1A2, IL1R2, OLAH, FAM20A | 0,864 | 0,854 | 0,874 | 0,911 | 0,855 | 0,968 | 0,933 | 0,850 | 1 |
| RSAD2, IFIT1, SIGLEC1, ISG15, HERC6, IL1R2, MMP8 | 0,864 | 0,855 | 0,873 | 0,904 | 0,847 | 0,962 | 0,957 | 0,913 | 1 |
| RSAD2, IFIT1, SIGLEC1, SLC1A2, IL1R2, OLAH, RETN | 0,864 | 0,855 | 0,873 | 0,913 | 0,857 | 0,969 | 0,961 | 0,920 | 1 |
| RSAD2, OAS1, IFIT1, SIGLEC1, SLC1A2, OLAH, RETN | 0,864 | 0,854 | 0,874 | 0,912 | 0,856 | 0,968 | 0,958 | 0,910 | 1 |
| OAS1, IFIT1, IFI44L, ISG15, HERC6, FAM20A, RETN | 0,864 | 0,854 | 0,874 | 0,906 | 0,844 | 0,969 | 0,928 | 0,848 | 1 |
| OAS1, HERC6, SLC1A2, IL1R2, OLAH, FAM20A, RETN | 0,864 | 0,853 | 0,874 | 0,912 | 0,856 | 0,967 | 0,927 | 0,842 | 1 |
| RSAD2, HERC6, SLC1A2, OLAH, FAM20A, RETN, MMP8 | 0,864 | 0,854 | 0,874 | 0,910 | 0,852 | 0,967 | 0,934 | 0,854 | 1 |
| IFI44L, ISG15, HERC6, OLAH, FAM20A, RETN, MMP8 | 0,864 | 0,853 | 0,874 | 0,903 | 0,836 | 0,969 | 0,950 | 0,886 | 1 |
| OAS1, IFIT1, IFI44L, SLC1A2, IL1R2, OLAH, FAM20A | 0,864 | 0,854 | 0,874 | 0,914 | 0,859 | 0,969 | 0,927 | 0,846 | 1 |
| RSAD2, IFIT1, SIGLEC1, HERC6, SLC1A2, IL1R2, MMP8 | 0,864 | 0,855 | 0,873 | 0,908 | 0,854 | 0,963 | 0,964 | 0,927 | 1 |
| OAS1, ISG15, HERC6, OLAH, FAM20A, RETN, MMP8 | 0,864 | 0,854 | 0,874 | 0,904 | 0,841 | 0,967 | 0,948 | 0,883 | 1 |

(suite)

| Modèle | AUC modèle | IC 95% | | AUC Train | IC 95% Train | | AUC Test | IC 95% Test | |
|---|---|---|---|---|---|---|---|---|---|
| ISG15, HERC6, SLC1A2, OLAH, FAM20A, RETN, MMP8 | 0,864 | 0,854 | 0,874 | 0,911 | 0,854 | 0,968 | 0,935 | 0,856 | 1 |
| RSAD2, OAS1, IFI44L, ISG15, IL1R2, OLAH, FAM20A | 0,864 | 0,854 | 0,874 | 0,901 | 0,836 | 0,966 | 0,948 | 0,882 | 1 |
| RSAD2, IFI27, OAS1, IFIT1, IFI44L, ISG15, FAM20A | 0,864 | 0,853 | 0,874 | 0,902 | 0,835 | 0,969 | 0,940 | 0,864 | 1 |
| OAS1, IFIT1, IFI44L, ISG15, HERC6, OLAH, FAM20A | 0,864 | 0,853 | 0,874 | 0,902 | 0,841 | 0,964 | 0,933 | 0,861 | 1 |
| RSAD2, ISG15, HERC6, OLAH, FAM20A, RETN, MMP8 | 0,864 | 0,854 | 0,874 | 0,905 | 0,843 | 0,967 | 0,947 | 0,882 | 1 |
| OAS1, SIGLEC1, ISG15, HERC6, IL1R2, OLAH, RETN | 0,864 | 0,854 | 0,874 | 0,905 | 0,844 | 0,966 | 0,958 | 0,915 | 1 |
| RSAD2, SIGLEC1, ISG15, HERC6, SLC1A2, IL1R2, RETN | 0,864 | 0,854 | 0,873 | 0,910 | 0,854 | 0,965 | 0,958 | 0,916 | 1,000 |
| OAS1, IFI44L, SIGLEC1, ISG15, SLC1A2, OLAH, MMP8 | 0,864 | 0,854 | 0,873 | 0,912 | 0,856 | 0,968 | 0,952 | 0,907 | 0,998 |
| OAS1, IFI44L, SIGLEC1, HERC6, SLC1A2, IL1R2, RETN | 0,864 | 0,854 | 0,873 | 0,911 | 0,856 | 0,966 | 0,952 | 0,906 | 0,998 |
| RSAD2, OAS1, SIGLEC1, HERC6, SLC1A2, IL1R2, MMP8 | 0,864 | 0,854 | 0,873 | 0,911 | 0,858 | 0,963 | 0,968 | 0,933 | 1 |
| IFI44L, SIGLEC1, HERC6, SLC1A2, OLAH, RETN, MMP8 | 0,864 | 0,854 | 0,874 | 0,910 | 0,851 | 0,968 | 0,962 | 0,923 | 1 |
| OAS1, IFI44L, SIGLEC1, SLC1A2, IL1R2, OLAH, MMP8 | 0,864 | 0,854 | 0,873 | 0,912 | 0,858 | 0,966 | 0,967 | 0,933 | 1 |
| OAS1, HERC6, SLC1A2, OLAH, FAM20A, RETN, MMP8 | 0,864 | 0,854 | 0,874 | 0,910 | 0,853 | 0,967 | 0,933 | 0,852 | 1 |
| RSAD2, OAS1, IFI44L, SIGLEC1, SLC1A2, OLAH, MMP8 | 0,864 | 0,854 | 0,874 | 0,911 | 0,855 | 0,967 | 0,957 | 0,914 | 0,999 |
| RSAD2, OAS1, SIGLEC1, HERC6, IL1R2, OLAH, RETN | 0,864 | 0,854 | 0,874 | 0,902 | 0,838 | 0,965 | 0,959 | 0,916 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, HERC6, FAM20A, MMP8 | 0,864 | 0,854 | 0,874 | 0,899 | 0,833 | 0,965 | 0,943 | 0,873 | 1 |
| RSAD2, IFIT1, SIGLEC1, ISG15, HERC6, IL1R2, OLAH | 0,864 | 0,854 | 0,873 | 0,908 | 0,850 | 0,966 | 0,951 | 0,905 | 0,997 |
| OAS1, SIGLEC1, ISG15, SLC1A2, IL1R2, OLAH, RETN | 0,864 | 0,854 | 0,873 | 0,912 | 0,857 | 0,968 | 0,960 | 0,919 | 1 |
| RSAD2, IFIT1, HERC6, SLC1A2, OLAH, FAM20A, MMP8 | 0,864 | 0,854 | 0,874 | 0,909 | 0,853 | 0,965 | 0,929 | 0,849 | 1 |
| RSAD2, IFI44L, SIGLEC1, SLC1A2, FAM20A, RETN, MMP8 | 0,864 | 0,854 | 0,874 | 0,910 | 0,849 | 0,970 | 0,950 | 0,883 | 1 |
| RSAD2, IFIT1, HERC6, SLC1A2, IL1R2, OLAH, FAM20A | 0,864 | 0,854 | 0,874 | 0,913 | 0,859 | 0,967 | 0,928 | 0,844 | 1 |
| RSAD2, OAS1, SIGLEC1, ISG15, SLC1A2, FAM20A, MMP8 | 0,864 | 0,853 | 0,874 | 0,902 | 0,840 | 0,965 | 0,945 | 0,870 | 1 |
| RSAD2, ISG15, HERC6, SLC1A2, IL1R2, FAM20A, MMP8 | 0,864 | 0,854 | 0,873 | 0,913 | 0,862 | 0,965 | 0,922 | 0,826 | 1 |
| RSAD2, IFI44L, SIGLEC1, HERC6, SLC1A2, IL1R2, OLAH | 0,864 | 0,854 | 0,874 | 0,910 | 0,855 | 0,966 | 0,958 | 0,916 | 0,999 |
| OAS1, IFIT1, IFI44L, SIGLEC1, HERC6, SLC1A2, IL1R2 | 0,864 | 0,854 | 0,873 | 0,910 | 0,857 | 0,963 | 0,959 | 0,918 | 1,000 |
| RSAD2, IFIT1, IFI44L, ISG15, HERC6, OLAH, FAM20A | 0,864 | 0,853 | 0,874 | 0,905 | 0,844 | 0,965 | 0,932 | 0,861 | 1 |
| RSAD2, OAS1, IFI44L, SIGLEC1, HERC6, SLC1A2, OLAH | 0,864 | 0,854 | 0,874 | 0,912 | 0,856 | 0,968 | 0,951 | 0,904 | 0,998 |
| OAS1, IFIT1, ISG15, HERC6, SLC1A2, OLAH, FAM20A | 0,864 | 0,853 | 0,874 | 0,911 | 0,855 | 0,967 | 0,930 | 0,851 | 1 |
| RSAD2, IFIT1, SIGLEC1, ISG15, HERC6, OLAH, RETN | 0,864 | 0,854 | 0,873 | 0,901 | 0,837 | 0,966 | 0,948 | 0,900 | 0,995 |
| RSAD2, IFIT1, IFI44L, HERC6, SLC1A2, IL1R2, FAM20A | 0,864 | 0,854 | 0,873 | 0,914 | 0,862 | 0,965 | 0,924 | 0,833 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, IL1R2, OLAH, MMP8 | 0,864 | 0,854 | 0,873 | 0,903 | 0,843 | 0,963 | 0,958 | 0,916 | 1,000 |
| OAS1, IFIT1, SIGLEC1, IL1R2, OLAH, RETN, MMP8 | 0,864 | 0,854 | 0,873 | 0,902 | 0,840 | 0,964 | 0,962 | 0,924 | 1 |
| RSAD2, IFI44L, SLC1A2, IL1R2, OLAH, FAM20A, RETN | 0,864 | 0,853 | 0,874 | 0,911 | 0,855 | 0,967 | 0,926 | 0,845 | 1 |
| IFI27, OAS1, IFIT1, IFI44L, HERC6, SLC1A2, FAM20A | 0,864 | 0,853 | 0,874 | 0,912 | 0,856 | 0,968 | 0,928 | 0,844 | 1 |
| OAS1, IFIT1, IFI44L, ISG15, SLC1A2, OLAH, FAM20A | 0,864 | 0,854 | 0,874 | 0,911 | 0,852 | 0,969 | 0,922 | 0,844 | 1,000 |
| RSAD2, IFI27, OAS1, IFIT1, IFI44L, FAM20A, MMP8 | 0,864 | 0,853 | 0,874 | 0,900 | 0,834 | 0,967 | 0,933 | 0,853 | 1 |
| RSAD2, IFI44L, HERC6, SLC1A2, OLAH, FAM20A, RETN | 0,864 | 0,853 | 0,874 | 0,914 | 0,859 | 0,969 | 0,924 | 0,843 | 1 |
| SIGLEC1, ISG15, HERC6, SLC1A2, IL1R2, OLAH, RETN | 0,864 | 0,854 | 0,874 | 0,915 | 0,860 | 0,969 | 0,959 | 0,917 | 1 |
| RSAD2, OAS1, IFIT1, IFI44L, IL1R2, OLAH, FAM20A | 0,864 | 0,854 | 0,873 | 0,901 | 0,835 | 0,966 | 0,946 | 0,878 | 1 |
| OAS1, IFIT1, IFI44L, SIGLEC1, HERC6, SLC1A2, RETN | 0,864 | 0,854 | 0,873 | 0,907 | 0,849 | 0,966 | 0,925 | 0,864 | 0,986 |
| OAS1, IFI44L, SIGLEC1, ISG15, HERC6, IL1R2, OLAH | 0,864 | 0,854 | 0,873 | 0,904 | 0,845 | 0,964 | 0,960 | 0,919 | 1 |
| OAS1, IFI44L, SIGLEC1, HERC6, SLC1A2, FAM20A, MMP8 | 0,864 | 0,854 | 0,874 | 0,907 | 0,850 | 0,964 | 0,942 | 0,867 | 1 |
| IFIT1, SIGLEC1, ISG15, HERC6, SLC1A2, IL1R2, MMP8 | 0,864 | 0,855 | 0,872 | 0,910 | 0,856 | 0,963 | 0,965 | 0,929 | 1 |
| RSAD2, IFIT1, SIGLEC1, ISG15, HERC6, SLC1A2, IL1R2 | 0,864 | 0,855 | 0,872 | 0,908 | 0,854 | 0,963 | 0,963 | 0,925 | 1 |
| RSAD2, IFI44L, SIGLEC1, HERC6, SLC1A2, OLAH, MMP8 | 0,864 | 0,854 | 0,874 | 0,914 | 0,859 | 0,968 | 0,949 | 0,902 | 0,996 |
| OAS1, IFIT1, IFI44L, SIGLEC1, IL1R2, RETN, MMP8 | 0,864 | 0,854 | 0,873 | 0,901 | 0,838 | 0,965 | 0,961 | 0,922 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, HERC6, SLC1A2, IL1R2, FAM20A, RETN, MMP8 | 0,864 | 0,854 | 0,873 | 0,914 | 0,861 | 0,968 | 0,928 | 0,839 | 1 |
| OAS1, ISG15, HERC6, SLC1A2, OLAH, FAM20A, MMP8 | 0,863 | 0,853 | 0,874 | 0,912 | 0,857 | 0,967 | 0,933 | 0,853 | 1 |
| IFIT1, HERC6, SLC1A2, IL1R2, OLAH, FAM20A, RETN | 0,863 | 0,853 | 0,874 | 0,912 | 0,857 | 0,967 | 0,927 | 0,843 | 1 |
| OAS1, IFI44L, SIGLEC1, HERC6, SLC1A2, IL1R2, MMP8 | 0,863 | 0,854 | 0,873 | 0,909 | 0,856 | 0,962 | 0,963 | 0,924 | 1 |
| OAS1, IFIT1, HERC6, OLAH, FAM20A, RETN, MMP8 | 0,863 | 0,853 | 0,873 | 0,901 | 0,837 | 0,966 | 0,950 | 0,886 | 1 |
| IFIT1, IFI44L, HERC6, SLC1A2, IL1R2, FAM20A, RETN | 0,863 | 0,854 | 0,873 | 0,915 | 0,863 | 0,966 | 0,923 | 0,834 | 1 |
| IFIT1, IFI44L, SLC1A2, IL1R2, FAM20A, RETN, MMP8 | 0,863 | 0,854 | 0,873 | 0,912 | 0,859 | 0,966 | 0,932 | 0,848 | 1 |
| RSAD2, ISG15, HERC6, SLC1A2, OLAH, FAM20A, MMP8 | 0,863 | 0,854 | 0,873 | 0,912 | 0,857 | 0,967 | 0,933 | 0,853 | 1 |
| RSAD2, IFI44L, HERC6, IL1R2, FAM20A, RETN, MMP8 | 0,863 | 0,853 | 0,874 | 0,910 | 0,849 | 0,970 | 0,936 | 0,853 | 1 |
| RSAD2, SIGLEC1, ISG15, HERC6, SLC1A2, IL1R2, MMP8 | 0,863 | 0,854 | 0,872 | 0,907 | 0,853 | 0,962 | 0,963 | 0,925 | 1 |
| RSAD2, IFIT1, IFI44L, HERC6, OLAH, FAM20A, MMP8 | 0,863 | 0,853 | 0,873 | 0,903 | 0,841 | 0,965 | 0,936 | 0,867 | 1 |
| IFI27, OAS1, IFI44L, ISG15, HERC6, SLC1A2, FAM20A | 0,863 | 0,853 | 0,874 | 0,910 | 0,853 | 0,968 | 0,932 | 0,849 | 1 |
| RSAD2, IFI27, OAS1, SLC1A2, FAM20A, RETN, MMP8 | 0,863 | 0,853 | 0,874 | 0,911 | 0,850 | 0,972 | 0,941 | 0,861 | 1 |
| IFI44L, SIGLEC1, ISG15, HERC6, SLC1A2, OLAH, RETN | 0,863 | 0,853 | 0,873 | 0,908 | 0,850 | 0,967 | 0,954 | 0,910 | 0,999 |
| RSAD2, SIGLEC1, ISG15, SLC1A2, OLAH, RETN, MMP8 | 0,863 | 0,854 | 0,873 | 0,911 | 0,853 | 0,968 | 0,958 | 0,916 | 0,999 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, SLC1A2, IL1R2, OLAH | 0,863 | 0,854 | 0,873 | 0,914 | 0,859 | 0,968 | 0,963 | 0,925 | 1 |
| RSAD2, HERC6, SLC1A2, IL1R2, FAM20A, RETN, MMP8 | 0,863 | 0,854 | 0,873 | 0,915 | 0,862 | 0,968 | 0,929 | 0,840 | 1 |
| IFI44L, SIGLEC1, ISG15, HERC6, IL1R2, OLAH, RETN | 0,863 | 0,854 | 0,873 | 0,903 | 0,839 | 0,966 | 0,959 | 0,917 | 1,000 |
| OAS1, IFI44L, SIGLEC1, HERC6, SLC1A2, IL1R2, OLAH | 0,863 | 0,853 | 0,874 | 0,912 | 0,858 | 0,966 | 0,958 | 0,916 | 1,000 |
| RSAD2, OAS1, IFI44L, SIGLEC1, SLC1A2, FAM20A, MMP8 | 0,863 | 0,853 | 0,873 | 0,903 | 0,841 | 0,966 | 0,943 | 0,870 | 1 |
| IFI27, IFIT1, IFI44L, ISG15, HERC6, SLC1A2, FAM20A | 0,863 | 0,853 | 0,874 | 0,905 | 0,847 | 0,964 | 0,932 | 0,848 | 1 |
| RSAD2, IFIT1, IFI44L, SLC1A2, IL1R2, FAM20A, MMP8 | 0,863 | 0,854 | 0,873 | 0,911 | 0,858 | 0,964 | 0,928 | 0,842 | 1 |
| RSAD2, SLC1A2, IL1R2, OLAH, FAM20A, RETN, MMP8 | 0,863 | 0,854 | 0,873 | 0,914 | 0,861 | 0,967 | 0,933 | 0,851 | 1 |
| OAS1, IFI44L, SIGLEC1, SLC1A2, OLAH, RETN, MMP8 | 0,863 | 0,853 | 0,873 | 0,911 | 0,853 | 0,969 | 0,959 | 0,915 | 1 |
| RSAD2, OAS1, SIGLEC1, ISG15, SLC1A2, FAM20A, RETN | 0,863 | 0,853 | 0,874 | 0,903 | 0,839 | 0,966 | 0,947 | 0,874 | 1 |
| RSAD2, ISG15, HERC6, SLC1A2, OLAH, FAM20A, RETN | 0,863 | 0,853 | 0,873 | 0,911 | 0,854 | 0,968 | 0,930 | 0,849 | 1 |
| RSAD2, IFIT1, HERC6, IL1R2, OLAH, FAM20A, RETN | 0,863 | 0,853 | 0,873 | 0,906 | 0,845 | 0,967 | 0,938 | 0,864 | 1 |
| RSAD2, OAS1, IFI44L, ISG15, SLC1A2, OLAH, FAM20A | 0,863 | 0,853 | 0,874 | 0,910 | 0,851 | 0,969 | 0,922 | 0,844 | 1,000 |
| RSAD2, IFI27, IFIT1, HERC6, SLC1A2, FAM20A, MMP8 | 0,863 | 0,853 | 0,873 | 0,911 | 0,855 | 0,967 | 0,928 | 0,845 | 1 |
| OAS1, IFI44L, SIGLEC1, HERC6, SLC1A2, OLAH, RETN | 0,863 | 0,853 | 0,873 | 0,913 | 0,857 | 0,969 | 0,950 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, ISG15, HERC6, IL1R2, OLAH | 0,863 | 0,854 | 0,873 | 0,902 | 0,841 | 0,963 | 0,959 | 0,918 | 1,000 |
| IFI27, IFI44L, ISG15, HERC6, SLC1A2, FAM20A, MMP8 | 0,863 | 0,852 | 0,874 | 0,906 | 0,846 | 0,966 | 0,930 | 0,847 | 1 |
| RSAD2, OAS1, ISG15, HERC6, OLAH, FAM20A, RETN | 0,863 | 0,853 | 0,873 | 0,905 | 0,843 | 0,967 | 0,942 | 0,875 | 1 |
| RSAD2, IFIT1, IFI44L, ISG15, HERC6, FAM20A, RETN | 0,863 | 0,853 | 0,873 | 0,906 | 0,845 | 0,967 | 0,922 | 0,842 | 1 |
| RSAD2, IFIT1, IFI44L, SIGLEC1, SLC1A2, IL1R2, OLAH | 0,863 | 0,854 | 0,872 | 0,911 | 0,855 | 0,967 | 0,965 | 0,929 | 1 |
| OAS1, IFIT1, IFI44L, SIGLEC1, HERC6, RETN, MMP8 | 0,863 | 0,854 | 0,873 | 0,900 | 0,833 | 0,966 | 0,923 | 0,862 | 0,984 |
| RSAD2, SIGLEC1, ISG15, HERC6, SLC1A2, OLAH, RETN | 0,863 | 0,853 | 0,873 | 0,912 | 0,855 | 0,969 | 0,948 | 0,900 | 0,995 |
| RSAD2, OAS1, IFI44L, SLC1A2, IL1R2, FAM20A, MMP8 | 0,863 | 0,853 | 0,873 | 0,915 | 0,863 | 0,966 | 0,927 | 0,837 | 1 |
| IFI44L, SIGLEC1, ISG15, SLC1A2, IL1R2, RETN, MMP8 | 0,863 | 0,855 | 0,872 | 0,912 | 0,857 | 0,966 | 0,968 | 0,934 | 1 |
| RSAD2, IFI44L, SLC1A2, OLAH, FAM20A, RETN, MMP8 | 0,863 | 0,853 | 0,873 | 0,913 | 0,856 | 0,971 | 0,930 | 0,859 | 1 |
| RSAD2, OAS1, IFIT1, SLC1A2, IL1R2, OLAH, FAM20A | 0,863 | 0,854 | 0,873 | 0,911 | 0,854 | 0,968 | 0,933 | 0,853 | 1 |
| IFIT1, IFI44L, SIGLEC1, ISG15, HERC6, OLAH, MMP8 | 0,863 | 0,853 | 0,873 | 0,900 | 0,837 | 0,962 | 0,945 | 0,895 | 0,994 |
| IFI44L, SIGLEC1, ISG15, HERC6, SLC1A2, IL1R2, RETN | 0,863 | 0,854 | 0,872 | 0,909 | 0,853 | 0,964 | 0,958 | 0,915 | 1 |
| IFI27, OAS1, IFIT1, SIGLEC1, ISG15, SLC1A2, MMP8 | 0,863 | 0,854 | 0,872 | 0,905 | 0,847 | 0,963 | 0,929 | 0,862 | 0,997 |
| OAS1, IFIT1, IFI44L, SLC1A2, IL1R2, FAM20A, MMP8 | 0,863 | 0,853 | 0,873 | 0,914 | 0,862 | 0,966 | 0,929 | 0,841 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SLC1A2, IL1R2, OLAH, FAM20A, RETN | 0,863 | 0,853 | 0,873 | 0,911 | 0,855 | 0,968 | 0,930 | 0,847 | 1 |
| OAS1, IFI44L, SIGLEC1, ISG15, FAM20A, RETN, MMP8 | 0,863 | 0,852 | 0,874 | 0,902 | 0,833 | 0,971 | 0,959 | 0,899 | 1 |
| RSAD2, OAS1, IFI44L, HERC6, OLAH, FAM20A, MMP8 | 0,863 | 0,852 | 0,874 | 0,904 | 0,842 | 0,966 | 0,941 | 0,877 | 1 |
| RSAD2, OAS1, IFIT1, IL1R2, FAM20A, RETN, MMP8 | 0,863 | 0,853 | 0,873 | 0,907 | 0,845 | 0,970 | 0,947 | 0,875 | 1 |
| IFIT1, ISG15, HERC6, SLC1A2, OLAH, FAM20A, MMP8 | 0,863 | 0,853 | 0,873 | 0,910 | 0,854 | 0,966 | 0,932 | 0,853 | 1 |
| OAS1, IFIT1, HERC6, SLC1A2, IL1R2, FAM20A, RETN | 0,863 | 0,853 | 0,873 | 0,911 | 0,857 | 0,966 | 0,930 | 0,846 | 1 |
| RSAD2, OAS1, HERC6, OLAH, FAM20A, RETN, MMP8 | 0,863 | 0,853 | 0,873 | 0,904 | 0,841 | 0,967 | 0,945 | 0,878 | 1 |
| RSAD2, OAS1, IFIT1, HERC6, SLC1A2, OLAH, FAM20A | 0,863 | 0,853 | 0,873 | 0,910 | 0,854 | 0,966 | 0,932 | 0,852 | 1 |
| IFIT1, ISG15, HERC6, SLC1A2, OLAH, FAM20A, RETN | 0,863 | 0,853 | 0,873 | 0,911 | 0,854 | 0,968 | 0,930 | 0,849 | 1 |
| IFI44L, SIGLEC1, ISG15, HERC6, SLC1A2, IL1R2, MMP8 | 0,863 | 0,854 | 0,872 | 0,907 | 0,853 | 0,962 | 0,964 | 0,927 | 1 |
| RSAD2, IFIT1, IFI44L, SIGLEC1, HERC6, OLAH, RETN | 0,863 | 0,854 | 0,872 | 0,901 | 0,836 | 0,966 | 0,938 | 0,885 | 0,991 |
| RSAD2, IFI27, ISG15, SLC1A2, FAM20A, RETN, MMP8 | 0,863 | 0,852 | 0,873 | 0,910 | 0,849 | 0,972 | 0,939 | 0,858 | 1 |
| IFIT1, IFI44L, SIGLEC1, SLC1A2, IL1R2, OLAH, RETN | 0,863 | 0,854 | 0,872 | 0,915 | 0,860 | 0,970 | 0,959 | 0,917 | 1 |
| RSAD2, IFI44L, SIGLEC1, ISG15, FAM20A, RETN, MMP8 | 0,863 | 0,853 | 0,873 | 0,903 | 0,836 | 0,970 | 0,953 | 0,894 | 1 |
| RSAD2, OAS1, IFI44L, SIGLEC1, SLC1A2, IL1R2, OLAH | 0,863 | 0,853 | 0,873 | 0,912 | 0,856 | 0,968 | 0,967 | 0,930 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, ISG15, RETN | 0,863 | 0,853 | 0,873 | 0,905 | 0,842 | 0,968 | 0,953 | 0,892 | 1 |
| RSAD2, IFI44L, SIGLEC1, ISG15, HERC6, SLC1A2, OLAH | 0,863 | 0,853 | 0,872 | 0,911 | 0,855 | 0,967 | 0,946 | 0,896 | 0,995 |
| RSAD2, OAS1, HERC6, SLC1A2, OLAH, FAM20A, RETN | 0,863 | 0,853 | 0,873 | 0,911 | 0,854 | 0,967 | 0,933 | 0,852 | 1 |
| RSAD2, SIGLEC1, ISG15, HERC6, IL1R2, OLAH, RETN | 0,863 | 0,853 | 0,873 | 0,904 | 0,842 | 0,966 | 0,958 | 0,915 | 1,000 |
| OAS1, IFIT1, SIGLEC1, SLC1A2, IL1R2, RETN, MMP8 | 0,863 | 0,853 | 0,872 | 0,912 | 0,858 | 0,966 | 0,970 | 0,937 | 1 |
| SIGLEC1, ISG15, HERC6, SLC1A2, IL1R2, RETN, MMP8 | 0,863 | 0,854 | 0,872 | 0,909 | 0,853 | 0,965 | 0,970 | 0,937 | 1 |
| RSAD2, IFIT1, ISG15, HERC6, OLAH, FAM20A, RETN | 0,863 | 0,853 | 0,873 | 0,905 | 0,843 | 0,966 | 0,942 | 0,875 | 1 |
| RSAD2, IFIT1, IFI44L, SIGLEC1, ISG15, SLC1A2, OLAH | 0,863 | 0,854 | 0,872 | 0,913 | 0,857 | 0,969 | 0,947 | 0,897 | 0,996 |
| RSAD2, IFI27, OAS1, HERC6, SLC1A2, FAM20A, MMP8 | 0,863 | 0,852 | 0,873 | 0,913 | 0,858 | 0,968 | 0,924 | 0,839 | 1 |
| OAS1, IFIT1, IFI44L, SIGLEC1, HERC6, OLAH, MMP8 | 0,863 | 0,853 | 0,872 | 0,899 | 0,838 | 0,961 | 0,939 | 0,886 | 0,992 |
| RSAD2, OAS1, IFIT1, SIGLEC1, HERC6, SLC1A2, RETN | 0,863 | 0,853 | 0,872 | 0,909 | 0,850 | 0,967 | 0,934 | 0,876 | 0,992 |
| RSAD2, IFI44L, SIGLEC1, ISG15, SLC1A2, FAM20A, MMP8 | 0,863 | 0,853 | 0,873 | 0,902 | 0,839 | 0,965 | 0,945 | 0,873 | 1 |
| RSAD2, OAS1, IFI44L, ISG15, HERC6, OLAH, FAM20A | 0,863 | 0,852 | 0,873 | 0,904 | 0,843 | 0,966 | 0,941 | 0,877 | 1 |
| RSAD2, OAS1, IFIT1, HERC6, SLC1A2, IL1R2, FAM20A | 0,863 | 0,853 | 0,872 | 0,913 | 0,859 | 0,966 | 0,930 | 0,845 | 1 |
| RSAD2, OAS1, IFI44L, SIGLEC1, ISG15, FAM20A, RETN | 0,863 | 0,852 | 0,873 | 0,897 | 0,826 | 0,967 | 0,953 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, ISG15, HERC6, SLC1A2, IL1R2 | 0,863 | 0,854 | 0,872 | 0,908 | 0,854 | 0,963 | 0,960 | 0,919 | 1 |
| IFI44L, SIGLEC1, ISG15, IL1R2, OLAH, RETN, MMP8 | 0,863 | 0,853 | 0,872 | 0,902 | 0,840 | 0,964 | 0,965 | 0,929 | 1 |
| RSAD2, OAS1, IFI44L, ISG15, IL1R2, FAM20A, MMP8 | 0,863 | 0,852 | 0,873 | 0,906 | 0,845 | 0,967 | 0,937 | 0,858 | 1 |
| RSAD2, OAS1, IFIT1, SLC1A2, OLAH, FAM20A, MMP8 | 0,863 | 0,853 | 0,872 | 0,909 | 0,851 | 0,967 | 0,941 | 0,872 | 1 |
| RSAD2, IFIT1, IFI44L, HERC6, OLAH, FAM20A, RETN | 0,863 | 0,853 | 0,872 | 0,906 | 0,844 | 0,968 | 0,933 | 0,863 | 1 |
| OAS1, IFI44L, SLC1A2, IL1R2, OLAH, FAM20A, RETN | 0,863 | 0,852 | 0,873 | 0,912 | 0,856 | 0,967 | 0,928 | 0,848 | 1 |
| RSAD2, IFI27, IFIT1, IFI44L, ISG15, FAM20A, MMP8 | 0,863 | 0,852 | 0,873 | 0,899 | 0,832 | 0,966 | 0,937 | 0,860 | 1 |
| RSAD2, OAS1, IFIT1, IFI44L, SIGLEC1, IL1R2, OLAH | 0,863 | 0,853 | 0,872 | 0,898 | 0,832 | 0,963 | 0,958 | 0,916 | 0,999 |
| OAS1, IFIT1, ISG15, SLC1A2, IL1R2, FAM20A, MMP8 | 0,863 | 0,853 | 0,872 | 0,913 | 0,859 | 0,967 | 0,934 | 0,852 | 1 |
| RSAD2, OAS1, IFI44L, SIGLEC1, HERC6, SLC1A2, IL1R2 | 0,863 | 0,853 | 0,872 | 0,908 | 0,853 | 0,963 | 0,963 | 0,923 | 1 |
| RSAD2, OAS1, SIGLEC1, HERC6, OLAH, RETN, MMP8 | 0,863 | 0,852 | 0,873 | 0,898 | 0,831 | 0,965 | 0,959 | 0,918 | 1,000 |
| RSAD2, IFIT1, IFI44L, ISG15, SLC1A2, OLAH, FAM20A | 0,863 | 0,853 | 0,872 | 0,909 | 0,850 | 0,968 | 0,922 | 0,845 | 0,998 |
| OAS1, IFI44L, SIGLEC1, ISG15, SLC1A2, IL1R2, OLAH | 0,863 | 0,853 | 0,872 | 0,913 | 0,858 | 0,968 | 0,962 | 0,924 | 1 |
| OAS1, IFIT1, SIGLEC1, ISG15, SLC1A2, IL1R2, RETN | 0,863 | 0,853 | 0,872 | 0,909 | 0,854 | 0,964 | 0,965 | 0,927 | 1 |
| OAS1, IFIT1, SIGLEC1, HERC6, SLC1A2, RETN, MMP8 | 0,862 | 0,853 | 0,872 | 0,905 | 0,845 | 0,965 | 0,929 | 0,871 | 0,988 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, SLC1A2, FAM20A | 0,862 | 0,852 | 0,873 | 0,904 | 0,842 | 0,967 | 0,945 | 0,873 | 1 |
| RSAD2, OAS1, ISG15, IL1R2, OLAH, FAM20A, RETN | 0,862 | 0,853 | 0,872 | 0,900 | 0,834 | 0,965 | 0,932 | 0,854 | 1 |
| OAS1, IFI44L, SIGLEC1, ISG15, SLC1A2, OLAH, RETN | 0,862 | 0,853 | 0,872 | 0,913 | 0,857 | 0,969 | 0,950 | 0,900 | 1,000 |
| OAS1, ISG15, IL1R2, OLAH, FAM20A, RETN, MMP8 | 0,862 | 0,853 | 0,872 | 0,907 | 0,845 | 0,969 | 0,939 | 0,862 | 1 |
| RSAD2, IFIT1, IFI44L, SIGLEC1, SLC1A2, OLAH, RETN | 0,862 | 0,853 | 0,872 | 0,913 | 0,857 | 0,970 | 0,947 | 0,896 | 0,998 |
| RSAD2, IFIT1, ISG15, IL1R2, OLAH, FAM20A, MMP8 | 0,862 | 0,853 | 0,872 | 0,905 | 0,843 | 0,967 | 0,945 | 0,874 | 1 |
| RSAD2, OAS1, IFI44L, ISG15, HERC6, FAM20A, RETN | 0,862 | 0,852 | 0,873 | 0,905 | 0,843 | 0,966 | 0,935 | 0,859 | 1 |
| OAS1, IFI44L, SIGLEC1, HERC6, IL1R2, OLAH, RETN | 0,862 | 0,852 | 0,873 | 0,904 | 0,842 | 0,966 | 0,955 | 0,911 | 0,999 |
| OAS1, IFI44L, SIGLEC1, ISG15, HERC6, SLC1A2, IL1R2 | 0,862 | 0,853 | 0,872 | 0,909 | 0,856 | 0,963 | 0,961 | 0,921 | 1 |
| RSAD2, IFI27, IFI44L, SIGLEC1, ISG15, HERC6, MMP8 | 0,862 | 0,854 | 0,871 | 0,901 | 0,843 | 0,960 | 0,905 | 0,830 | 0,981 |
| RSAD2, IFI44L, ISG15, HERC6, OLAH, FAM20A, RETN | 0,862 | 0,852 | 0,873 | 0,906 | 0,844 | 0,967 | 0,942 | 0,878 | 1 |
| RSAD2, IFI44L, SIGLEC1, HERC6, SLC1A2, IL1R2, MMP8 | 0,862 | 0,853 | 0,871 | 0,906 | 0,851 | 0,962 | 0,964 | 0,927 | 1 |
| RSAD2, IFIT1, ISG15, SLC1A2, IL1R2, FAM20A, MMP8 | 0,862 | 0,853 | 0,871 | 0,910 | 0,855 | 0,965 | 0,933 | 0,851 | 1 |
| OAS1, SLC1A2, IL1R2, OLAH, FAM20A, RETN, MMP8 | 0,862 | 0,853 | 0,872 | 0,915 | 0,863 | 0,968 | 0,932 | 0,850 | 1 |
| RSAD2, IFIT1, SIGLEC1, ISG15, SLC1A2, OLAH, RETN | 0,862 | 0,853 | 0,872 | 0,914 | 0,857 | 0,970 | 0,953 | 0,907 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, SLC1A2, IL1R2, OLAH, RETN | 0,862 | 0,853 | 0,872 | 0,913 | 0,857 | 0,968 | 0,965 | 0,928 | 1 |
| OAS1, SIGLEC1, ISG15, HERC6, OLAH, RETN, MMP8 | 0,862 | 0,852 | 0,873 | 0,900 | 0,835 | 0,966 | 0,970 | 0,935 | 1 |
| RSAD2, IFI44L, SIGLEC1, ISG15, SLC1A2, OLAH, MMP8 | 0,862 | 0,853 | 0,872 | 0,912 | 0,857 | 0,968 | 0,951 | 0,905 | 0,997 |
| RSAD2, OAS1, IFIT1, IFI44L, SLC1A2, OLAH, FAM20A | 0,862 | 0,853 | 0,872 | 0,911 | 0,853 | 0,969 | 0,921 | 0,842 | 0,999 |
| RSAD2, IFIT1, IFI44L, SIGLEC1, IL1R2, OLAH, MMP8 | 0,862 | 0,853 | 0,871 | 0,902 | 0,841 | 0,962 | 0,960 | 0,919 | 1 |
| IFI27, IFIT1, IFI44L, ISG15, SLC1A2, FAM20A, MMP8 | 0,862 | 0,852 | 0,873 | 0,901 | 0,837 | 0,966 | 0,933 | 0,851 | 1 |
| OAS1, IFIT1, IFI44L, ISG15, IL1R2, OLAH, FAM20A | 0,862 | 0,853 | 0,872 | 0,901 | 0,835 | 0,967 | 0,948 | 0,880 | 1 |
| OAS1, IFI44L, SLC1A2, OLAH, FAM20A, RETN, MMP8 | 0,862 | 0,852 | 0,873 | 0,914 | 0,857 | 0,971 | 0,932 | 0,859 | 1 |
| OAS1, IFIT1, SIGLEC1, ISG15, HERC6, OLAH, MMP8 | 0,862 | 0,852 | 0,872 | 0,900 | 0,839 | 0,961 | 0,943 | 0,893 | 0,994 |
| IFIT1, IFI44L, SIGLEC1, ISG15, IL1R2, OLAH, MMP8 | 0,862 | 0,853 | 0,871 | 0,904 | 0,847 | 0,962 | 0,959 | 0,917 | 1 |
| RSAD2, IFIT1, IFI44L, HERC6, IL1R2, FAM20A, RETN | 0,862 | 0,853 | 0,872 | 0,907 | 0,846 | 0,968 | 0,936 | 0,855 | 1 |
| IFIT1, IFI44L, ISG15, SLC1A2, IL1R2, FAM20A, RETN | 0,862 | 0,853 | 0,872 | 0,908 | 0,853 | 0,963 | 0,926 | 0,839 | 1 |
| IFIT1, ISG15, SLC1A2, IL1R2, OLAH, FAM20A, RETN | 0,862 | 0,852 | 0,872 | 0,908 | 0,851 | 0,965 | 0,932 | 0,857 | 1 |
| RSAD2, IFIT1, IFI44L, IL1R2, OLAH, FAM20A, MMP8 | 0,862 | 0,853 | 0,872 | 0,905 | 0,844 | 0,967 | 0,952 | 0,892 | 1 |
| RSAD2, IFI44L, SIGLEC1, HERC6, SLC1A2, OLAH, RETN | 0,862 | 0,852 | 0,872 | 0,913 | 0,856 | 0,969 | 0,946 | 0,896 | 0,995 |
| OAS1, IFIT1, ISG15, IL1R2, OLAH, FAM20A, RETN | 0,862 | 0,852 | 0,872 | 0,900 | 0,833 | 0,966 | 0,941 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, SLC1A2, IL1R2, OLAH, FAM20A, RETN | 0,862 | 0,852 | 0,872 | 0,911 | 0,854 | 0,968 | 0,935 | 0,855 | 1 |
| RSAD2, OAS1, HERC6, SLC1A2, IL1R2, FAM20A, RETN | 0,862 | 0,852 | 0,872 | 0,911 | 0,856 | 0,965 | 0,929 | 0,840 | 1 |
| OAS1, IFIT1, SLC1A2, IL1R2, FAM20A, RETN, MMP8 | 0,862 | 0,852 | 0,872 | 0,911 | 0,856 | 0,967 | 0,937 | 0,858 | 1 |
| RSAD2, OAS1, SIGLEC1, IL1R2, OLAH, RETN, MMP8 | 0,862 | 0,852 | 0,873 | 0,901 | 0,837 | 0,964 | 0,968 | 0,935 | 1 |
| OAS1, IFI44L, SIGLEC1, SLC1A2, IL1R2, OLAH, RETN | 0,862 | 0,852 | 0,872 | 0,914 | 0,858 | 0,969 | 0,962 | 0,922 | 1 |
| RSAD2, IFI44L, SIGLEC1, ISG15, SLC1A2, IL1R2, OLAH | 0,862 | 0,853 | 0,871 | 0,911 | 0,856 | 0,967 | 0,963 | 0,925 | 1 |
| RSAD2, OAS1, IFIT1, HERC6, OLAH, FAM20A, MMP8 | 0,862 | 0,852 | 0,872 | 0,899 | 0,835 | 0,962 | 0,941 | 0,874 | 1 |
| RSAD2, IFI44L, SIGLEC1, ISG15, OLAH, FAM20A, RETN | 0,862 | 0,852 | 0,872 | 0,897 | 0,826 | 0,969 | 0,962 | 0,919 | 1 |
| RSAD2, OAS1, IFIT1, IL1R2, OLAH, FAM20A, RETN | 0,862 | 0,852 | 0,872 | 0,901 | 0,836 | 0,966 | 0,940 | 0,867 | 1 |
| OAS1, IFI44L, HERC6, SLC1A2, IL1R2, FAM20A, RETN | 0,862 | 0,852 | 0,872 | 0,915 | 0,864 | 0,967 | 0,927 | 0,840 | 1 |
| RSAD2, IFI27, OAS1, IFIT1, SIGLEC1, SLC1A2, MMP8 | 0,862 | 0,853 | 0,871 | 0,904 | 0,846 | 0,963 | 0,932 | 0,861 | 1 |
| OAS1, IFI44L, ISG15, HERC6, OLAH, FAM20A, RETN | 0,862 | 0,852 | 0,872 | 0,905 | 0,843 | 0,967 | 0,946 | 0,884 | 1 |
| OAS1, IFIT1, SIGLEC1, ISG15, SLC1A2, IL1R2, MMP8 | 0,862 | 0,853 | 0,871 | 0,910 | 0,856 | 0,964 | 0,967 | 0,932 | 1 |
| RSAD2, IFIT1, SIGLEC1, SLC1A2, IL1R2, RETN, MMP8 | 0,862 | 0,853 | 0,871 | 0,912 | 0,858 | 0,966 | 0,971 | 0,939 | 1 |
| IFIT1, IFI44L, SIGLEC1, ISG15, SLC1A2, IL1R2, RETN | 0,862 | 0,853 | 0,871 | 0,910 | 0,854 | 0,965 | 0,965 | 0,927 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, ISG15, IL1R2, FAM20A | 0,862 | 0,852 | 0,872 | 0,902 | 0,839 | 0,965 | 0,941 | 0,867 | 1 |
| OAS1, IFIT1, ISG15, IL1R2, FAM20A, RETN, MMP8 | 0,862 | 0,852 | 0,872 | 0,905 | 0,842 | 0,969 | 0,948 | 0,874 | 1 |
| RSAD2, IFI44L, SIGLEC1, HERC6, IL1R2, OLAH, RETN | 0,862 | 0,852 | 0,872 | 0,900 | 0,836 | 0,965 | 0,958 | 0,916 | 0,999 |
| RSAD2, OAS1, IFI44L, SIGLEC1, IL1R2, OLAH, MMP8 | 0,862 | 0,852 | 0,872 | 0,901 | 0,841 | 0,962 | 0,960 | 0,919 | 1 |
| RSAD2, IFIT1, ISG15, HERC6, SLC1A2, OLAH, FAM20A | 0,862 | 0,852 | 0,872 | 0,911 | 0,854 | 0,967 | 0,932 | 0,852 | 1 |
| RSAD2, IFI44L, SIGLEC1, HERC6, SLC1A2, IL1R2, RETN | 0,862 | 0,853 | 0,871 | 0,908 | 0,853 | 0,964 | 0,955 | 0,912 | 0,999 |
| RSAD2, IFI27, SIGLEC1, ISG15, HERC6, SLC1A2, MMP8 | 0,862 | 0,853 | 0,871 | 0,903 | 0,846 | 0,959 | 0,926 | 0,859 | 0,993 |
| IFIT1, SIGLEC1, ISG15, SLC1A2, IL1R2, RETN, MMP8 | 0,862 | 0,853 | 0,871 | 0,912 | 0,858 | 0,966 | 0,971 | 0,939 | 1 |
| IFIT1, IFI44L, SIGLEC1, IL1R2, OLAH, RETN, MMP8 | 0,862 | 0,852 | 0,872 | 0,902 | 0,840 | 0,964 | 0,964 | 0,927 | 1 |
| IFI27, OAS1, IFIT1, IFI44L, SIGLEC1, SLC1A2, MMP8 | 0,862 | 0,853 | 0,871 | 0,905 | 0,846 | 0,963 | 0,930 | 0,864 | 0,997 |
| IFI27, OAS1, IFIT1, IFI44L, ISG15, FAM20A, MMP8 | 0,862 | 0,851 | 0,873 | 0,898 | 0,831 | 0,965 | 0,934 | 0,854 | 1 |
| IFI44L, ISG15, IL1R2, OLAH, FAM20A, RETN, MMP8 | 0,862 | 0,852 | 0,871 | 0,905 | 0,843 | 0,966 | 0,948 | 0,886 | 1 |
| OAS1, IFIT1, IFI44L, SIGLEC1, SLC1A2, IL1R2, RETN | 0,862 | 0,853 | 0,871 | 0,910 | 0,855 | 0,965 | 0,962 | 0,922 | 1 |
| RSAD2, OAS1, SIGLEC1, ISG15, SLC1A2, OLAH, RETN | 0,862 | 0,852 | 0,872 | 0,912 | 0,855 | 0,968 | 0,955 | 0,906 | 1 |
| OAS1, IFI44L, SIGLEC1, IL1R2, OLAH, RETN, MMP8 | 0,862 | 0,852 | 0,872 | 0,903 | 0,841 | 0,965 | 0,963 | 0,925 | 1 |
| RSAD2, OAS1, IFIT1, HERC6, OLAH, FAM20A, RETN | 0,862 | 0,852 | 0,872 | 0,901 | 0,837 | 0,966 | 0,943 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, SLC1A2, OLAH, RETN, MMP8 | 0,862 | 0,852 | 0,871 | 0,912 | 0,854 | 0,969 | 0,957 | 0,914 | 0,999 |
| RSAD2, OAS1, IFI44L, SIGLEC1, ISG15, SLC1A2, OLAH | 0,862 | 0,852 | 0,871 | 0,911 | 0,855 | 0,967 | 0,948 | 0,898 | 0,998 |
| OAS1, SIGLEC1, ISG15, HERC6, SLC1A2, RETN, MMP8 | 0,862 | 0,852 | 0,872 | 0,905 | 0,844 | 0,965 | 0,952 | 0,903 | 1 |
| RSAD2, IFIT1, ISG15, SLC1A2, IL1R2, OLAH, FAM20A | 0,862 | 0,853 | 0,871 | 0,908 | 0,850 | 0,966 | 0,933 | 0,855 | 1 |
| OAS1, IFIT1, IFI44L, SIGLEC1, ISG15, IL1R2, OLAH | 0,862 | 0,852 | 0,871 | 0,901 | 0,838 | 0,965 | 0,953 | 0,909 | 0,997 |
| RSAD2, OAS1, IFIT1, SIGLEC1, IL1R2, OLAH, MMP8 | 0,862 | 0,852 | 0,871 | 0,904 | 0,845 | 0,963 | 0,963 | 0,925 | 1 |
| OAS1, IFI44L, HERC6, OLAH, FAM20A, RETN, MMP8 | 0,862 | 0,851 | 0,872 | 0,904 | 0,839 | 0,968 | 0,948 | 0,885 | 1 |
| OAS1, IFIT1, IFI44L, SIGLEC1, SLC1A2, IL1R2, MMP8 | 0,862 | 0,853 | 0,870 | 0,908 | 0,854 | 0,962 | 0,964 | 0,926 | 1 |
| RSAD2, OAS1, IFIT1, SIGLEC1, HERC6, OLAH, MMP8 | 0,862 | 0,852 | 0,871 | 0,899 | 0,837 | 0,961 | 0,940 | 0,888 | 0,993 |
| IFI27, OAS1, IFIT1, IFI44L, ISG15, HERC6, MMP8 | 0,862 | 0,853 | 0,870 | 0,901 | 0,844 | 0,957 | 0,893 | 0,813 | 0,974 |
| RSAD2, OAS1, IFIT1, SIGLEC1, HERC6, RETN, MMP8 | 0,862 | 0,852 | 0,872 | 0,899 | 0,832 | 0,966 | 0,926 | 0,866 | 0,986 |
| RSAD2, IFIT1, IFI44L, SIGLEC1, HERC6, SLC1A2, IL1R2 | 0,862 | 0,853 | 0,870 | 0,906 | 0,851 | 0,962 | 0,960 | 0,920 | 1,000 |
| RSAD2, IFIT1, SLC1A2, IL1R2, FAM20A, RETN, MMP8 | 0,862 | 0,852 | 0,871 | 0,911 | 0,855 | 0,966 | 0,937 | 0,859 | 1 |
| IFIT1, IFI44L, SIGLEC1, ISG15, IL1R2, RETN, MMP8 | 0,862 | 0,852 | 0,871 | 0,902 | 0,839 | 0,965 | 0,965 | 0,929 | 1 |
| IFIT1, IFI44L, SIGLEC1, SLC1A2, IL1R2, RETN, MMP8 | 0,862 | 0,853 | 0,871 | 0,912 | 0,858 | 0,966 | 0,967 | 0,932 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, ISG15, SLC1A2, IL1R2, OLAH, FAM20A, RETN | 0,862 | 0,851 | 0,872 | 0,912 | 0,856 | 0,968 | 0,926 | 0,842 | 1 |
| IFIT1, ISG15, SLC1A2, IL1R2, FAM20A, RETN, MMP8 | 0,862 | 0,852 | 0,871 | 0,911 | 0,856 | 0,966 | 0,938 | 0,861 | 1 |
| RSAD2, IFI27, OAS1, SIGLEC1, ISG15, SLC1A2, MMP8 | 0,862 | 0,852 | 0,871 | 0,905 | 0,846 | 0,964 | 0,937 | 0,872 | 1 |
| RSAD2, IFI44L, SIGLEC1, ISG15, HERC6, SLC1A2, IL1R2 | 0,862 | 0,852 | 0,871 | 0,908 | 0,853 | 0,963 | 0,959 | 0,918 | 1,000 |
| RSAD2, OAS1, IFIT1, SIGLEC1, ISG15, SLC1A2, IL1R2 | 0,861 | 0,852 | 0,871 | 0,907 | 0,851 | 0,963 | 0,968 | 0,932 | 1 |
| RSAD2, OAS1, IFIT1, IFI44L, SIGLEC1, IL1R2, MMP8 | 0,861 | 0,852 | 0,871 | 0,901 | 0,840 | 0,962 | 0,961 | 0,921 | 1 |
| OAS1, IFI44L, SIGLEC1, HERC6, OLAH, RETN, MMP8 | 0,861 | 0,851 | 0,872 | 0,898 | 0,831 | 0,965 | 0,962 | 0,924 | 1 |
| RSAD2, ISG15, SLC1A2, IL1R2, OLAH, FAM20A, RETN | 0,861 | 0,852 | 0,871 | 0,911 | 0,855 | 0,967 | 0,932 | 0,850 | 1 |
| OAS1, IFIT1, IFI44L, SIGLEC1, ISG15, IL1R2, MMP8 | 0,861 | 0,852 | 0,870 | 0,900 | 0,839 | 0,961 | 0,958 | 0,916 | 1,000 |
| RSAD2, IFI44L, SIGLEC1, ISG15, SLC1A2, FAM20A, RETN | 0,861 | 0,851 | 0,871 | 0,904 | 0,842 | 0,966 | 0,946 | 0,874 | 1 |
| RSAD2, IFI44L, HERC6, SLC1A2, IL1R2, FAM20A, RETN | 0,861 | 0,851 | 0,872 | 0,915 | 0,864 | 0,967 | 0,924 | 0,836 | 1 |
| OAS1, IFI44L, ISG15, SLC1A2, OLAH, FAM20A, RETN | 0,861 | 0,851 | 0,872 | 0,908 | 0,849 | 0,968 | 0,927 | 0,852 | 1 |
| IFIT1, IFI44L, SIGLEC1, ISG15, SLC1A2, IL1R2, MMP8 | 0,861 | 0,853 | 0,870 | 0,908 | 0,854 | 0,962 | 0,964 | 0,926 | 1 |
| RSAD2, OAS1, IFI44L, SIGLEC1, SLC1A2, OLAH, RETN | 0,861 | 0,852 | 0,871 | 0,911 | 0,854 | 0,968 | 0,955 | 0,906 | 1 |
| IFI27, IFIT1, IFI44L, SIGLEC1, ISG15, SLC1A2, MMP8 | 0,861 | 0,852 | 0,871 | 0,902 | 0,843 | 0,961 | 0,926 | 0,857 | 0,996 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, ISG15, SLC1A2, IL1R2, FAM20A, RETN | 0,861 | 0,851 | 0,871 | 0,908 | 0,851 | 0,964 | 0,936 | 0,855 | 1 |
| RSAD2, OAS1, IFIT1, SIGLEC1, IL1R2, RETN, MMP8 | 0,861 | 0,851 | 0,871 | 0,902 | 0,839 | 0,965 | 0,968 | 0,935 | 1 |
| IFI27, OAS1, IFIT1, IFI44L, ISG15, RETN, MMP8 | 0,861 | 0,851 | 0,872 | 0,904 | 0,840 | 0,967 | 0,951 | 0,888 | 1 |
| OAS1, IFIT1, IFI44L, ISG15, IL1R2, FAM20A, MMP8 | 0,861 | 0,851 | 0,871 | 0,904 | 0,841 | 0,967 | 0,943 | 0,868 | 1 |
| RSAD2, IFI27, IFI44L, SIGLEC1, HERC6, SLC1A2, MMP8 | 0,861 | 0,852 | 0,870 | 0,906 | 0,851 | 0,961 | 0,923 | 0,854 | 0,991 |
| IFIT1, SIGLEC1, ISG15, IL1R2, OLAH, RETN, MMP8 | 0,861 | 0,851 | 0,871 | 0,903 | 0,841 | 0,965 | 0,968 | 0,935 | 1 |
| OAS1, IFIT1, HERC6, SLC1A2, OLAH, FAM20A, RETN | 0,861 | 0,851 | 0,872 | 0,910 | 0,854 | 0,967 | 0,930 | 0,850 | 1 |
| RSAD2, OAS1, IFIT1, IFI44L, SIGLEC1, IL1R2, RETN | 0,861 | 0,852 | 0,871 | 0,897 | 0,830 | 0,963 | 0,961 | 0,921 | 1 |
| OAS1, IFI44L, SIGLEC1, ISG15, SLC1A2, FAM20A, RETN | 0,861 | 0,851 | 0,871 | 0,901 | 0,837 | 0,965 | 0,947 | 0,875 | 1 |
| IFI27, IFI44L, SIGLEC1, ISG15, HERC6, SLC1A2, MMP8 | 0,861 | 0,852 | 0,870 | 0,902 | 0,845 | 0,959 | 0,924 | 0,855 | 0,993 |
| OAS1, IFIT1, IFI44L, SLC1A2, OLAH, FAM20A, RETN | 0,861 | 0,851 | 0,871 | 0,911 | 0,852 | 0,969 | 0,924 | 0,848 | 1,000 |
| OAS1, IFIT1, IFI44L, SIGLEC1, ISG15, HERC6, OLAH | 0,861 | 0,851 | 0,871 | 0,900 | 0,839 | 0,962 | 0,941 | 0,890 | 0,993 |
| RSAD2, IFI44L, SIGLEC1, ISG15, SLC1A2, OLAH, RETN | 0,861 | 0,852 | 0,871 | 0,913 | 0,856 | 0,969 | 0,948 | 0,898 | 0,998 |
| IFI44L, SIGLEC1, ISG15, HERC6, OLAH, RETN, MMP8 | 0,861 | 0,851 | 0,871 | 0,896 | 0,827 | 0,965 | 0,963 | 0,925 | 1 |
| OAS1, SIGLEC1, ISG15, SLC1A2, IL1R2, RETN, MMP8 | 0,861 | 0,852 | 0,870 | 0,912 | 0,858 | 0,966 | 0,971 | 0,938 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, ISG15, SLC1A2, OLAH, FAM20A, MMP8 | 0,861 | 0,851 | 0,871 | 0,909 | 0,851 | 0,967 | 0,936 | 0,861 | 1 |
| RSAD2, OAS1, SIGLEC1, ISG15, IL1R2, OLAH, MMP8 | 0,861 | 0,851 | 0,871 | 0,904 | 0,846 | 0,963 | 0,963 | 0,926 | 1 |
| OAS1, IFIT1, IFI44L, ISG15, SLC1A2, IL1R2, FAM20A | 0,861 | 0,851 | 0,871 | 0,907 | 0,850 | 0,964 | 0,926 | 0,839 | 1 |
| RSAD2, OAS1, IFI44L, SIGLEC1, ISG15, FAM20A, MMP8 | 0,861 | 0,850 | 0,872 | 0,897 | 0,828 | 0,967 | 0,951 | 0,888 | 1 |
| RSAD2, IFIT1, IFI44L, SLC1A2, OLAH, FAM20A, RETN | 0,861 | 0,851 | 0,871 | 0,909 | 0,850 | 0,968 | 0,921 | 0,844 | 0,997 |
| RSAD2, OAS1, IFI44L, SIGLEC1, ISG15, IL1R2, OLAH | 0,861 | 0,851 | 0,870 | 0,897 | 0,831 | 0,963 | 0,958 | 0,916 | 1,000 |
| RSAD2, OAS1, IFI44L, HERC6, OLAH, FAM20A, RETN | 0,861 | 0,851 | 0,871 | 0,903 | 0,839 | 0,966 | 0,938 | 0,871 | 1 |
| RSAD2, OAS1, ISG15, HERC6, OLAH, FAM20A, MMP8 | 0,861 | 0,851 | 0,871 | 0,901 | 0,840 | 0,963 | 0,941 | 0,872 | 1 |
| OAS1, IFIT1, SIGLEC1, ISG15, IL1R2, OLAH, MMP8 | 0,861 | 0,852 | 0,870 | 0,905 | 0,846 | 0,964 | 0,957 | 0,915 | 0,998 |
| RSAD2, IFIT1, IFI44L, SIGLEC1, ISG15, HERC6, RETN | 0,861 | 0,851 | 0,870 | 0,899 | 0,834 | 0,964 | 0,926 | 0,867 | 0,986 |
| RSAD2, IFIT1, IFI44L, SIGLEC1, IL1R2, RETN, MMP8 | 0,861 | 0,851 | 0,870 | 0,900 | 0,835 | 0,964 | 0,967 | 0,933 | 1 |
| OAS1, IFI44L, IL1R2, OLAH, FAM20A, RETN, MMP8 | 0,861 | 0,851 | 0,871 | 0,905 | 0,842 | 0,968 | 0,947 | 0,878 | 1 |
| OAS1, IFIT1, IFI44L, IL1R2, FAM20A, RETN, MMP8 | 0,861 | 0,851 | 0,871 | 0,907 | 0,845 | 0,970 | 0,946 | 0,871 | 1 |
| RSAD2, OAS1, IFIT1, SIGLEC1, SLC1A2, IL1R2, RETN | 0,861 | 0,851 | 0,870 | 0,909 | 0,853 | 0,965 | 0,970 | 0,933 | 1 |
| OAS1, IFIT1, SIGLEC1, ISG15, IL1R2, RETN, MMP8 | 0,861 | 0,851 | 0,871 | 0,901 | 0,838 | 0,965 | 0,968 | 0,935 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, SLC1A2, IL1R2, RETN, MMP8 | 0,861 | 0,851 | 0,870 | 0,912 | 0,859 | 0,966 | 0,973 | 0,941 | 1 |
| OAS1, IFIT1, IFI44L, SIGLEC1, ISG15, SLC1A2, IL1R2 | 0,861 | 0,852 | 0,870 | 0,907 | 0,851 | 0,963 | 0,964 | 0,927 | 1 |
| RSAD2, OAS1, IFIT1, ISG15, HERC6, FAM20A, RETN | 0,861 | 0,851 | 0,871 | 0,900 | 0,837 | 0,963 | 0,935 | 0,858 | 1 |
| RSAD2, IFI44L, HERC6, OLAH, FAM20A, RETN, MMP8 | 0,861 | 0,851 | 0,871 | 0,906 | 0,843 | 0,969 | 0,941 | 0,876 | 1 |
| OAS1, IFI44L, ISG15, SLC1A2, IL1R2, FAM20A, MMP8 | 0,861 | 0,851 | 0,871 | 0,913 | 0,862 | 0,965 | 0,927 | 0,834 | 1 |
| OAS1, IFIT1, ISG15, HERC6, OLAH, FAM20A, MMP8 | 0,861 | 0,851 | 0,871 | 0,900 | 0,837 | 0,963 | 0,942 | 0,873 | 1 |
| RSAD2, OAS1, IFIT1, ISG15, SLC1A2, IL1R2, FAM20A | 0,861 | 0,851 | 0,870 | 0,906 | 0,848 | 0,964 | 0,936 | 0,855 | 1 |
| RSAD2, IFI44L, ISG15, SLC1A2, IL1R2, FAM20A, MMP8 | 0,861 | 0,851 | 0,871 | 0,912 | 0,860 | 0,963 | 0,927 | 0,838 | 1 |
| RSAD2, IFIT1, SLC1A2, IL1R2, OLAH, FAM20A, RETN | 0,861 | 0,851 | 0,871 | 0,909 | 0,852 | 0,966 | 0,935 | 0,859 | 1 |
| RSAD2, OAS1, IFIT1, SIGLEC1, ISG15, IL1R2, OLAH | 0,861 | 0,851 | 0,870 | 0,900 | 0,836 | 0,963 | 0,959 | 0,917 | 1 |
| IFIT1, IFI44L, IL1R2, OLAH, FAM20A, RETN, MMP8 | 0,861 | 0,851 | 0,871 | 0,906 | 0,844 | 0,968 | 0,950 | 0,888 | 1 |
| RSAD2, OAS1, SIGLEC1, ISG15, HERC6, SLC1A2, RETN | 0,861 | 0,851 | 0,871 | 0,905 | 0,844 | 0,965 | 0,952 | 0,902 | 1 |
| RSAD2, OAS1, IFI44L, SLC1A2, OLAH, FAM20A, RETN | 0,861 | 0,850 | 0,871 | 0,911 | 0,852 | 0,969 | 0,923 | 0,847 | 0,999 |
| RSAD2, IFI44L, ISG15, IL1R2, OLAH, FAM20A, MMP8 | 0,861 | 0,851 | 0,871 | 0,904 | 0,843 | 0,965 | 0,948 | 0,884 | 1 |
| RSAD2, IFI27, OAS1, IFI44L, ISG15, HERC6, MMP8 | 0,861 | 0,852 | 0,870 | 0,900 | 0,842 | 0,958 | 0,903 | 0,827 | 0,980 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SIGLEC1, ISG15, IL1R2 | 0,861 | 0,852 | 0,870 | 0,896 | 0,831 | 0,961 | 0,958 | 0,917 | 0,999 |
| OAS1, IFIT1, ISG15, SLC1A2, OLAH, FAM20A, MMP8 | 0,861 | 0,851 | 0,871 | 0,908 | 0,850 | 0,967 | 0,938 | 0,866 | 1 |
| RSAD2, IFIT1, HERC6, OLAH, FAM20A, RETN, MMP8 | 0,861 | 0,851 | 0,871 | 0,900 | 0,836 | 0,964 | 0,948 | 0,884 | 1 |
| RSAD2, IFI27, IFIT1, IFI44L, SLC1A2, FAM20A, MMP8 | 0,861 | 0,850 | 0,871 | 0,903 | 0,839 | 0,967 | 0,934 | 0,853 | 1 |
| RSAD2, IFI27, IFI44L, ISG15, SLC1A2, FAM20A, MMP8 | 0,861 | 0,850 | 0,872 | 0,903 | 0,839 | 0,967 | 0,938 | 0,858 | 1 |
| RSAD2, IFI44L, ISG15, HERC6, OLAH, FAM20A, MMP8 | 0,861 | 0,850 | 0,871 | 0,902 | 0,840 | 0,964 | 0,940 | 0,876 | 1 |
| IFI27, OAS1, IFIT1, IFI44L, ISG15, SLC1A2, RETN | 0,861 | 0,851 | 0,870 | 0,904 | 0,841 | 0,967 | 0,945 | 0,874 | 1 |
| IFI27, OAS1, IFI44L, SIGLEC1, ISG15, SLC1A2, MMP8 | 0,861 | 0,851 | 0,870 | 0,905 | 0,846 | 0,963 | 0,934 | 0,867 | 1 |
| RSAD2, IFIT1, HERC6, SLC1A2, IL1R2, FAM20A, RETN | 0,861 | 0,851 | 0,871 | 0,912 | 0,858 | 0,967 | 0,932 | 0,846 | 1 |
| IFIT1, IFI44L, SIGLEC1, ISG15, HERC6, RETN, MMP8 | 0,861 | 0,851 | 0,871 | 0,896 | 0,829 | 0,964 | 0,932 | 0,875 | 0,988 |
| RSAD2, OAS1, IFIT1, SIGLEC1, IL1R2, OLAH, RETN | 0,861 | 0,851 | 0,870 | 0,897 | 0,831 | 0,964 | 0,959 | 0,916 | 1 |
| OAS1, IFI44L, SIGLEC1, ISG15, IL1R2, OLAH, MMP8 | 0,861 | 0,851 | 0,870 | 0,903 | 0,844 | 0,963 | 0,957 | 0,914 | 0,999 |
| RSAD2, IFI27, OAS1, IFI44L, SIGLEC1, SLC1A2, MMP8 | 0,861 | 0,851 | 0,870 | 0,904 | 0,844 | 0,963 | 0,933 | 0,863 | 1 |
| RSAD2, OAS1, IFI44L, IL1R2, OLAH, FAM20A, RETN | 0,861 | 0,850 | 0,871 | 0,900 | 0,835 | 0,966 | 0,941 | 0,870 | 1 |
| RSAD2, OAS1, IFIT1, ISG15, HERC6, OLAH, FAM20A | 0,861 | 0,850 | 0,871 | 0,901 | 0,839 | 0,964 | 0,937 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SLC1A2, FAM20A, MMP8 | 0,861 | 0,850 | 0,872 | 0,903 | 0,838 | 0,967 | 0,935 | 0,854 | 1 |
| RSAD2, IFI27, OAS1, ISG15, HERC6, SLC1A2, FAM20A | 0,861 | 0,850 | 0,871 | 0,908 | 0,850 | 0,966 | 0,927 | 0,843 | 1 |
| RSAD2, OAS1, IFIT1, SIGLEC1, SLC1A2, IL1R2, MMP8 | 0,861 | 0,851 | 0,870 | 0,908 | 0,854 | 0,962 | 0,971 | 0,938 | 1 |
| RSAD2, OAS1, SIGLEC1, ISG15, SLC1A2, IL1R2, RETN | 0,861 | 0,851 | 0,870 | 0,909 | 0,853 | 0,965 | 0,968 | 0,931 | 1 |
| RSAD2, IFIT1, IFI44L, SIGLEC1, SLC1A2, IL1R2, MMP8 | 0,861 | 0,852 | 0,869 | 0,908 | 0,853 | 0,963 | 0,968 | 0,934 | 1 |
| RSAD2, IFIT1, SIGLEC1, IL1R2, OLAH, RETN, MMP8 | 0,861 | 0,851 | 0,870 | 0,901 | 0,838 | 0,964 | 0,967 | 0,933 | 1 |
| RSAD2, OAS1, IFIT1, IFI44L, SLC1A2, IL1R2, FAM20A | 0,861 | 0,851 | 0,870 | 0,906 | 0,849 | 0,963 | 0,927 | 0,842 | 1 |
| OAS1, IFI44L, SIGLEC1, SLC1A2, IL1R2, RETN, MMP8 | 0,861 | 0,851 | 0,870 | 0,912 | 0,857 | 0,966 | 0,968 | 0,934 | 1 |
| RSAD2, IFI44L, ISG15, SLC1A2, OLAH, FAM20A, RETN | 0,861 | 0,850 | 0,871 | 0,909 | 0,850 | 0,968 | 0,926 | 0,852 | 1 |
| RSAD2, IFIT1, IFI44L, SIGLEC1, ISG15, IL1R2, MMP8 | 0,861 | 0,852 | 0,869 | 0,900 | 0,838 | 0,962 | 0,960 | 0,919 | 1 |
| RSAD2, IFIT1, HERC6, SLC1A2, OLAH, FAM20A, RETN | 0,860 | 0,850 | 0,871 | 0,910 | 0,853 | 0,967 | 0,932 | 0,850 | 1 |
| RSAD2, IFI44L, SIGLEC1, ISG15, IL1R2, OLAH, MMP8 | 0,860 | 0,851 | 0,870 | 0,900 | 0,839 | 0,962 | 0,959 | 0,917 | 1 |
| RSAD2, OAS1, SIGLEC1, ISG15, HERC6, OLAH, RETN | 0,860 | 0,850 | 0,871 | 0,899 | 0,835 | 0,964 | 0,952 | 0,906 | 0,998 |
| OAS1, IFIT1, SIGLEC1, ISG15, IL1R2, OLAH, RETN | 0,860 | 0,851 | 0,870 | 0,900 | 0,836 | 0,965 | 0,954 | 0,910 | 0,999 |
| IFI27, OAS1, IFIT1, IFI44L, SLC1A2, FAM20A, MMP8 | 0,860 | 0,850 | 0,871 | 0,901 | 0,837 | 0,966 | 0,932 | 0,850 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, ISG15, IL1R2, OLAH | 0,860 | 0,851 | 0,869 | 0,898 | 0,832 | 0,964 | 0,957 | 0,914 | 0,999 |
| RSAD2,IFI27, OAS1,IFIT1, ISG15, HERC6, MMP8 | 0,860 | 0,851 | 0,869 | 0,901 | 0,843 | 0,958 | 0,901 | 0,823 | 0,979 |
| RSAD2, IFIT1, SIGLEC1, ISG15, SLC1A2, IL1R2, MMP8 | 0,860 | 0,852 | 0,869 | 0,908 | 0,853 | 0,963 | 0,965 | 0,929 | 1 |
| OAS1, IFI44L, SIGLEC1, ISG15, HERC6, FAM20A, MMP8 | 0,860 | 0,850 | 0,871 | 0,899 | 0,834 | 0,965 | 0,953 | 0,891 | 1 |
| IFIT1,ISG15,IL1R2,OLAH, FAM20A, RETN, MMP8 | 0,860 | 0,850 | 0,870 | 0,906 | 0,845 | 0,967 | 0,948 | 0,882 | 1 |
| OAS1, IFIT1, IFI44L, SIGLEC1, IL1R2, OLAH, RETN | 0,860 | 0,851 | 0,870 | 0,900 | 0,836 | 0,965 | 0,955 | 0,912 | 0,999 |
| RSAD2, IFIT1, SIGLEC1, ISG15, SLC1A2, IL1R2, RETN | 0,860 | 0,851 | 0,869 | 0,908 | 0,852 | 0,964 | 0,965 | 0,928 | 1 |
| RSAD2, IFI44L, SIGLEC1, IL1R2, OLAH, RETN, MMP8 | 0,860 | 0,851 | 0,870 | 0,901 | 0,838 | 0,965 | 0,967 | 0,932 | 1 |
| OAS1, IFI44L, SIGLEC1, ISG15, SLC1A2, IL1R2, RETN | 0,860 | 0,851 | 0,869 | 0,910 | 0,855 | 0,965 | 0,965 | 0,927 | 1 |
| RSAD2, SIGLEC1, ISG15, SLC1A2, IL1R2, RETN, MMP8 | 0,860 | 0,851 | 0,869 | 0,913 | 0,859 | 0,966 | 0,972 | 0,941 | 1 |
| RSAD2, OAS1, SIGLEC1, ISG15, HERC6, RETN, MMP8 | 0,860 | 0,850 | 0,870 | 0,899 | 0,832 | 0,965 | 0,951 | 0,904 | 0,998 |
| RSAD2, IFIT1, IFI44L, SIGLEC1, ISG15, SLC1A2, IL1R2 | 0,860 | 0,851 | 0,869 | 0,905 | 0,848 | 0,962 | 0,968 | 0,935 | 1 |
| OAS1, IFIT1, IFI44L, SIGLEC1, ISG15, IL1R2, RETN | 0,860 | 0,851 | 0,870 | 0,899 | 0,833 | 0,964 | 0,959 | 0,918 | 0,999 |
| RSAD2, OAS1, IFI44L, SIGLEC1, SLC1A2, FAM20A, RETN | 0,860 | 0,850 | 0,870 | 0,905 | 0,843 | 0,967 | 0,945 | 0,873 | 1 |
| RSAD2, OAS1, SIGLEC1, ISG15, SLC1A2, IL1R2, MMP8 | 0,860 | 0,851 | 0,869 | 0,909 | 0,855 | 0,963 | 0,968 | 0,934 | 1 |
| RSAD2, OAS1, IFIT1, IFI44L, SIGLEC1, SLC1A2, IL1R2 | 0,860 | 0,851 | 0,869 | 0,905 | 0,849 | 0,962 | 0,970 | 0,935 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, SLC1A2, IL1R2, RETN | 0,860 | 0,851 | 0,869 | 0,906 | 0,849 | 0,963 | 0,967 | 0,929 | 1 |
| IFIT1, IFI44L, ISG15, IL1R2, FAM20A, RETN, MMP8 | 0,860 | 0,850 | 0,870 | 0,903 | 0,839 | 0,966 | 0,946 | 0,873 | 1 |
| RSAD2, OAS1, IFIT1, ISG15, IL1R2, FAM20A, RETN | 0,860 | 0,850 | 0,870 | 0,902 | 0,838 | 0,966 | 0,943 | 0,869 | 1 |
| RSAD2, IFIT1, IFI44L, SIGLEC1, SLC1A2, IL1R2, RETN | 0,860 | 0,851 | 0,869 | 0,907 | 0,850 | 0,963 | 0,965 | 0,928 | 1 |
| OAS1, IFIT1, IFI44L, SIGLEC1, OLAH, RETN, MMP8 | 0,860 | 0,850 | 0,870 | 0,897 | 0,830 | 0,964 | 0,955 | 0,912 | 0,999 |
| RSAD2, SIGLEC1, ISG15, HERC6, OLAH, RETN, MMP8 | 0,860 | 0,850 | 0,870 | 0,900 | 0,835 | 0,966 | 0,958 | 0,916 | 0,999 |
| RSAD2, IFI44L, SIGLEC1, ISG15, SLC1A2, IL1R2, RETN | 0,860 | 0,851 | 0,869 | 0,907 | 0,850 | 0,963 | 0,970 | 0,937 | 1 |
| OAS1, IFI44L, SIGLEC1, ISG15, IL1R2, RETN, MMP8 | 0,860 | 0,850 | 0,870 | 0,901 | 0,837 | 0,965 | 0,962 | 0,923 | 1 |
| RSAD2, IFIT1, IFI44L, ISG15, SLC1A2, IL1R2, FAM20A | 0,860 | 0,851 | 0,869 | 0,906 | 0,850 | 0,962 | 0,927 | 0,840 | 1 |
| RSAD2, IFI27, OAS1, IFI44L, ISG15, SLC1A2, FAM20A | 0,860 | 0,849 | 0,871 | 0,902 | 0,838 | 0,966 | 0,935 | 0,855 | 1 |
| RSAD2, IFI44L, ISG15, HERC6, FAM20A, RETN, MMP8 | 0,860 | 0,850 | 0,870 | 0,908 | 0,846 | 0,969 | 0,934 | 0,858 | 1 |
| RSAD2, OAS1, IFI44L, SIGLEC1, IL1R2, OLAH, RETN | 0,860 | 0,850 | 0,870 | 0,895 | 0,829 | 0,962 | 0,962 | 0,922 | 1 |
| RSAD2, OAS1, IFI44L, SIGLEC1, IL1R2, RETN, MMP8 | 0,860 | 0,850 | 0,870 | 0,900 | 0,836 | 0,964 | 0,970 | 0,937 | 1 |
| OAS1, SIGLEC1, ISG15, IL1R2, OLAH, RETN, MMP8 | 0,860 | 0,850 | 0,870 | 0,900 | 0,837 | 0,964 | 0,970 | 0,936 | 1 |
| RSAD2, IFIT1, IFI44L, SIGLEC1, IL1R2, OLAH, RETN | 0,860 | 0,851 | 0,869 | 0,898 | 0,831 | 0,964 | 0,961 | 0,921 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFI44L, SIGLEC1, ISG15, HERC6, OLAH, RETN | 0,860 | 0,850 | 0,870 | 0,898 | 0,833 | 0,964 | 0,952 | 0,906 | 0,998 |
| RSAD2, IFIT1, IFI44L, SIGLEC1, HERC6, OLAH, MMP8 | 0,860 | 0,850 | 0,869 | 0,899 | 0,836 | 0,961 | 0,936 | 0,881 | 0,991 |
| IFIT1, IFI44L, ISG15, HERC6, FAM20A, RETN, MMP8 | 0,860 | 0,850 | 0,870 | 0,905 | 0,840 | 0,969 | 0,937 | 0,860 | 1 |
| IFI27, OAS1, IFI44L, ISG15, SLC1A2, FAM20A, MMP8 | 0,860 | 0,848 | 0,871 | 0,901 | 0,836 | 0,966 | 0,937 | 0,856 | 1 |
| OAS1, IFI44L, SIGLEC1, ISG15, HERC6, SLC1A2, RETN | 0,860 | 0,850 | 0,870 | 0,905 | 0,844 | 0,965 | 0,952 | 0,902 | 1 |
| OAS1, IFI44L, SIGLEC1, ISG15, SLC1A2, FAM20A, MMP8 | 0,860 | 0,849 | 0,870 | 0,903 | 0,840 | 0,965 | 0,948 | 0,877 | 1 |
| RSAD2, OAS1, IFI44L, ISG15, SLC1A2, IL1R2, FAM20A | 0,860 | 0,850 | 0,870 | 0,908 | 0,851 | 0,964 | 0,926 | 0,839 | 1 |
| RSAD2, IFI27, OAS1, IFIT1, HERC6, SLC1A2, MMP8 | 0,860 | 0,850 | 0,869 | 0,907 | 0,852 | 0,962 | 0,898 | 0,815 | 0,981 |
| RSAD2, OAS1, IFI44L, SIGLEC1, SLC1A2, IL1R2, MMP8 | 0,860 | 0,851 | 0,869 | 0,907 | 0,852 | 0,962 | 0,972 | 0,940 | 1 |
| RSAD2, OAS1, IFI44L, SIGLEC1, ISG15, SLC1A2, IL1R2 | 0,860 | 0,850 | 0,869 | 0,905 | 0,848 | 0,962 | 0,967 | 0,930 | 1 |
| RSAD2, OAS1, IFIT1, IFI44L, SIGLEC1, HERC6, OLAH | 0,860 | 0,850 | 0,869 | 0,900 | 0,838 | 0,961 | 0,939 | 0,886 | 0,992 |
| RSAD2, OAS1, IFIT1, SLC1A2, IL1R2, FAM20A, RETN | 0,860 | 0,850 | 0,869 | 0,907 | 0,851 | 0,964 | 0,936 | 0,855 | 1 |
| IFI27, OAS1, IFIT1, ISG15, HERC6, SLC1A2, FAM20A | 0,860 | 0,849 | 0,870 | 0,904 | 0,845 | 0,963 | 0,930 | 0,847 | 1 |
| RSAD2, IFI27, OAS1, ISG15, HERC6, SLC1A2, MMP8 | 0,860 | 0,850 | 0,869 | 0,906 | 0,851 | 0,961 | 0,901 | 0,820 | 0,982 |
| RSAD2, IFI44L, SIGLEC1, SLC1A2, IL1R2, RETN, MMP8 | 0,860 | 0,850 | 0,869 | 0,910 | 0,855 | 0,965 | 0,971 | 0,939 | 1 |
| RSAD2, IFI27, OAS1, IFI44L, ISG15, SLC1A2, RETN | 0,860 | 0,850 | 0,869 | 0,904 | 0,842 | 0,967 | 0,943 | 0,874 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, ISG15, IL1R2, RETN, MMP8 | 0,860 | 0,850 | 0,869 | 0,900 | 0,835 | 0,964 | 0,967 | 0,933 | 1 |
| RSAD2, OAS1, IFI44L, SIGLEC1, ISG15, IL1R2, MMP8 | 0,859 | 0,850 | 0,869 | 0,900 | 0,838 | 0,961 | 0,960 | 0,919 | 1 |
| RSAD2, OAS1, ISG15, IL1R2, FAM20A, RETN, MMP8 | 0,859 | 0,849 | 0,870 | 0,905 | 0,843 | 0,968 | 0,939 | 0,860 | 1 |
| RSAD2, IFI27, OAS1, IFIT1, IFI44L, RETN, MMP8 | 0,859 | 0,849 | 0,870 | 0,905 | 0,842 | 0,968 | 0,949 | 0,884 | 1 |
| OAS1, IFI44L, ISG15, IL1R2, OLAH, FAM20A, RETN | 0,859 | 0,849 | 0,870 | 0,900 | 0,834 | 0,965 | 0,943 | 0,874 | 1 |
| OAS1, IFIT1, SLC1A2, OLAH, FAM20A, RETN, MMP8 | 0,859 | 0,849 | 0,870 | 0,906 | 0,845 | 0,967 | 0,939 | 0,866 | 1 |
| OAS1, IFI44L, SLC1A2, IL1R2, FAM20A, RETN, MMP8 | 0,859 | 0,850 | 0,869 | 0,912 | 0,859 | 0,965 | 0,925 | 0,832 | 1 |
| RSAD2, IFI44L, ISG15, HERC6, SLC1A2, FAM20A, RETN | 0,859 | 0,849 | 0,870 | 0,905 | 0,844 | 0,966 | 0,930 | 0,851 | 1 |
| RSAD2, IFI27, OAS1, IFIT1, ISG15, RETN, MMP8 | 0,859 | 0,849 | 0,870 | 0,904 | 0,841 | 0,968 | 0,950 | 0,886 | 1 |
| OAS1, IFI44L, SIGLEC1, ISG15, SLC1A2, IL1R2, MMP8 | 0,859 | 0,851 | 0,868 | 0,908 | 0,853 | 0,962 | 0,965 | 0,928 | 1 |
| RSAD2, SIGLEC1, ISG15, IL1R2, OLAH, RETN, MMP8 | 0,859 | 0,849 | 0,870 | 0,901 | 0,838 | 0,964 | 0,971 | 0,938 | 1 |
| RSAD2, OAS1, IFI44L, SIGLEC1, ISG15, HERC6, RETN | 0,859 | 0,849 | 0,869 | 0,898 | 0,832 | 0,964 | 0,949 | 0,901 | 0,997 |
| RSAD2, IFI27, OAS1, IFI44L, ISG15, RETN, MMP8 | 0,859 | 0,849 | 0,869 | 0,905 | 0,842 | 0,969 | 0,950 | 0,887 | 1 |
| RSAD2, IFIT1, ISG15, HERC6, OLAH, FAM20A, MMP8 | 0,859 | 0,849 | 0,869 | 0,901 | 0,840 | 0,962 | 0,939 | 0,869 | 1 |
| OAS1, IFIT1, IFI44L, IL1R2, OLAH, FAM20A, RETN | 0,859 | 0,850 | 0,869 | 0,900 | 0,834 | 0,966 | 0,942 | 0,872 | 1 |
| IFI27, OAS1, IFIT1, ISG15, SLC1A2, RETN, MMP8 | 0,859 | 0,849 | 0,869 | 0,900 | 0,836 | 0,965 | 0,941 | 0,870 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, ISG15, SLC1A2, IL1R2, FAM20A, RETN | 0,859 | 0,849 | 0,869 | 0,908 | 0,853 | 0,963 | 0,928 | 0,842 | 1 |
| RSAD2, OAS1, SIGLEC1, ISG15, IL1R2, RETN, MMP8 | 0,859 | 0,849 | 0,870 | 0,900 | 0,836 | 0,963 | 0,970 | 0,936 | 1 |
| RSAD2, OAS1, ISG15, SLC1A2, IL1R2, FAM20A, MMP8 | 0,859 | 0,850 | 0,869 | 0,910 | 0,856 | 0,963 | 0,927 | 0,835 | 1 |
| RSAD2, IFIT1, IFI44L, SIGLEC1, ISG15, IL1R2, RETN | 0,859 | 0,850 | 0,868 | 0,897 | 0,831 | 0,964 | 0,961 | 0,922 | 1,000 |
| RSAD2, IFI27, OAS1, IFI44L, HERC6, SLC1A2, MMP8 | 0,859 | 0,850 | 0,869 | 0,907 | 0,851 | 0,962 | 0,903 | 0,822 | 0,984 |
| RSAD2, IFIT1, ISG15, SLC1A2, OLAH, FAM20A, MMP8 | 0,859 | 0,850 | 0,869 | 0,905 | 0,845 | 0,965 | 0,939 | 0,869 | 1 |
| RSAD2, IFIT1, IL1R2, OLAH, FAM20A, RETN, MMP8 | 0,859 | 0,849 | 0,869 | 0,904 | 0,842 | 0,967 | 0,948 | 0,881 | 1 |
| RSAD2, OAS1, IFI44L, IL1R2, FAM20A, RETN, MMP8 | 0,859 | 0,849 | 0,870 | 0,907 | 0,846 | 0,968 | 0,941 | 0,865 | 1 |
| RSAD2, IFI27, OAS1, IFIT1, ISG15, SLC1A2, RETN | 0,859 | 0,849 | 0,869 | 0,901 | 0,836 | 0,966 | 0,942 | 0,869 | 1 |
| RSAD2, IFI27, IFIT1, SIGLEC1, ISG15, SLC1A2, MMP8 | 0,859 | 0,850 | 0,868 | 0,904 | 0,845 | 0,963 | 0,925 | 0,855 | 0,995 |
| RSAD2, IFI27, IFIT1, ISG15, HERC6, SLC1A2, FAM20A | 0,859 | 0,848 | 0,870 | 0,908 | 0,850 | 0,966 | 0,930 | 0,847 | 1 |
| RSAD2, IFIT1, ISG15, SLC1A2, IL1R2, FAM20A, RETN | 0,859 | 0,850 | 0,868 | 0,907 | 0,850 | 0,964 | 0,935 | 0,853 | 1 |
| IFIT1, IFI44L, SIGLEC1, ISG15, IL1R2, OLAH, RETN | 0,859 | 0,850 | 0,868 | 0,900 | 0,837 | 0,964 | 0,961 | 0,919 | 1 |
| RSAD2, IFI44L, SIGLEC1, ISG15, SLC1A2, IL1R2, MMP8 | 0,859 | 0,850 | 0,868 | 0,906 | 0,851 | 0,962 | 0,968 | 0,935 | 1 |
| RSAD2, OAS1, IFIT1, SIGLEC1, ISG15, HERC6, OLAH | 0,859 | 0,849 | 0,869 | 0,900 | 0,839 | 0,962 | 0,941 | 0,890 | 0,993 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SLC1A2, OLAH, FAM20A, RETN, MMP8 | 0,859 | 0,849 | 0,869 | 0,908 | 0,849 | 0,967 | 0,938 | 0,864 | 1 |
| RSAD2, IFI44L, SLC1A2, IL1R2, FAM20A, RETN, MMP8 | 0,859 | 0,849 | 0,868 | 0,914 | 0,862 | 0,966 | 0,926 | 0,835 | 1 |
| RSAD2, OAS1, IFI44L, SIGLEC1, ISG15, IL1R2, RETN | 0,859 | 0,849 | 0,868 | 0,893 | 0,826 | 0,961 | 0,962 | 0,923 | 1 |
| RSAD2, OAS1, SLC1A2, IL1R2, FAM20A, RETN, MMP8 | 0,859 | 0,850 | 0,868 | 0,913 | 0,859 | 0,966 | 0,928 | 0,837 | 1 |
| RSAD2, OAS1, IFI44L, SLC1A2, IL1R2, FAM20A, RETN | 0,859 | 0,849 | 0,869 | 0,907 | 0,851 | 0,963 | 0,923 | 0,834 | 1 |
| OAS1, IFIT1, IFI44L, SLC1A2, IL1R2, FAM20A, RETN | 0,859 | 0,849 | 0,869 | 0,908 | 0,852 | 0,963 | 0,924 | 0,835 | 1 |
| IFIT1, IFI44L, ISG15, HERC6, SLC1A2, FAM20A, RETN | 0,859 | 0,849 | 0,869 | 0,907 | 0,848 | 0,967 | 0,934 | 0,853 | 1 |
| RSAD2, IFI27, IFIT1, IFI44L, ISG15, SLC1A2, FAM20A | 0,859 | 0,848 | 0,869 | 0,901 | 0,836 | 0,966 | 0,939 | 0,860 | 1 |
| IFI27, OAS1, IFI44L, ISG15, SLC1A2, RETN, MMP8 | 0,859 | 0,849 | 0,869 | 0,904 | 0,842 | 0,966 | 0,943 | 0,877 | 1 |
| IFI27, OAS1, IFIT1, IFI44L, SLC1A2, RETN, MMP8 | 0,859 | 0,849 | 0,869 | 0,903 | 0,839 | 0,966 | 0,940 | 0,867 | 1 |
| RSAD2, IFIT1, SIGLEC1, ISG15, HERC6, OLAH, MMP8 | 0,859 | 0,849 | 0,869 | 0,900 | 0,838 | 0,962 | 0,943 | 0,893 | 0,994 |
| RSAD2, IFI27, OAS1, IFIT1, IFI44L, SLC1A2, FAM20A | 0,859 | 0,848 | 0,870 | 0,903 | 0,839 | 0,967 | 0,935 | 0,855 | 1 |
| OAS1, IFI44L, ISG15, SLC1A2, IL1R2, FAM20A, RETN | 0,859 | 0,848 | 0,869 | 0,908 | 0,852 | 0,963 | 0,925 | 0,837 | 1 |
| RSAD2, OAS1, IFIT1, IFI44L, IL1R2, FAM20A, RETN | 0,859 | 0,849 | 0,869 | 0,901 | 0,837 | 0,966 | 0,939 | 0,864 | 1 |
| RSAD2, IFI27, OAS1, IFIT1, IFI44L, HERC6, MMP8 | 0,859 | 0,849 | 0,868 | 0,900 | 0,841 | 0,958 | 0,899 | 0,819 | 0,978 |
| RSAD2, IFIT1, SIGLEC1, ISG15, IL1R2, OLAH, MMP8 | 0,859 | 0,849 | 0,868 | 0,904 | 0,846 | 0,963 | 0,965 | 0,929 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, ISG15, SLC1A2, OLAH, FAM20A, RETN, MMP8 | 0,859 | 0,848 | 0,869 | 0,906 | 0,847 | 0,966 | 0,942 | 0,875 | 1 |
| OAS1, IFIT1, ISG15, HERC6, SLC1A2, FAM20A, RETN | 0,859 | 0,848 | 0,869 | 0,902 | 0,840 | 0,964 | 0,936 | 0,855 | 1 |
| RSAD2, OAS1, ISG15, SLC1A2, OLAH, FAM20A, RETN | 0,859 | 0,848 | 0,869 | 0,910 | 0,851 | 0,968 | 0,934 | 0,859 | 1 |
| OAS1, IFIT1, ISG15, SLC1A2, OLAH, FAM20A, RETN | 0,859 | 0,848 | 0,869 | 0,908 | 0,849 | 0,967 | 0,936 | 0,862 | 1 |
| RSAD2, OAS1, IFIT1, ISG15, SLC1A2, OLAH, FAM20A | 0,859 | 0,849 | 0,869 | 0,908 | 0,849 | 0,967 | 0,935 | 0,861 | 1 |
| OAS1, ISG15, SLC1A2, IL1R2, FAM20A, RETN, MMP8 | 0,859 | 0,849 | 0,868 | 0,913 | 0,859 | 0,967 | 0,929 | 0,841 | 1 |
| OAS1, IFI44L, ISG15, HERC6, OLAH, FAM20A, MMP8 | 0,859 | 0,848 | 0,869 | 0,898 | 0,834 | 0,962 | 0,947 | 0,885 | 1 |
| OAS1, IFI44L, SIGLEC1, ISG15, IL1R2, OLAH, RETN | 0,859 | 0,849 | 0,868 | 0,899 | 0,834 | 0,964 | 0,957 | 0,914 | 0,999 |
| RSAD2, OAS1, ISG15, HERC6, FAM20A, RETN, MMP8 | 0,859 | 0,848 | 0,869 | 0,904 | 0,842 | 0,967 | 0,936 | 0,859 | 1 |
| RSAD2, IFIT1, SIGLEC1, ISG15, HERC6, RETN, MMP8 | 0,859 | 0,849 | 0,868 | 0,898 | 0,832 | 0,964 | 0,930 | 0,873 | 0,988 |
| RSAD2, IFIT1, SLC1A2, OLAH, FAM20A, RETN, MMP8 | 0,858 | 0,849 | 0,868 | 0,906 | 0,846 | 0,967 | 0,941 | 0,871 | 1 |
| OAS1, IFIT1, IFI44L, HERC6, SLC1A2, OLAH, RETN | 0,858 | 0,848 | 0,869 | 0,907 | 0,849 | 0,966 | 0,928 | 0,865 | 0,992 |
| RSAD2, IFI27, OAS1, IFIT1, IFI44L, SLC1A2, RETN | 0,858 | 0,849 | 0,868 | 0,903 | 0,839 | 0,968 | 0,943 | 0,872 | 1 |
| OAS1, IFI44L, ISG15, HERC6, SLC1A2, FAM20A, RETN | 0,858 | 0,848 | 0,869 | 0,904 | 0,844 | 0,965 | 0,934 | 0,854 | 1 |
| IFI27, OAS1, IFI44L, ISG15, HERC6, SLC1A2, MMP8 | 0,858 | 0,849 | 0,868 | 0,906 | 0,852 | 0,960 | 0,900 | 0,821 | 0,979 |
| IFI27, OAS1, IFIT1, ISG15, HERC6, SLC1A2, MMP8 | 0,858 | 0,849 | 0,868 | 0,904 | 0,849 | 0,959 | 0,902 | 0,823 | 0,981 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, HERC6, SLC1A2, RETN | 0,858 | 0,849 | 0,868 | 0,904 | 0,844 | 0,963 | 0,934 | 0,877 | 0,990 |
| RSAD2, ISG15, IL1R2, OLAH, FAM20A, RETN, MMP8 | 0,858 | 0,848 | 0,868 | 0,902 | 0,839 | 0,965 | 0,941 | 0,868 | 1 |
| OAS1, IFI44L, HERC6, SLC1A2, OLAH, RETN, MMP8 | 0,858 | 0,848 | 0,869 | 0,907 | 0,848 | 0,966 | 0,939 | 0,884 | 0,994 |
| IFI27, OAS1, IFIT1, IFI44L, HERC6, SLC1A2, MMP8 | 0,858 | 0,849 | 0,868 | 0,904 | 0,849 | 0,959 | 0,908 | 0,832 | 0,983 |
| RSAD2, IFIT1, SIGLEC1, ISG15, IL1R2, RETN, MMP8 | 0,858 | 0,849 | 0,868 | 0,901 | 0,838 | 0,965 | 0,972 | 0,940 | 1 |
| OAS1, IFIT1, SIGLEC1, ISG15, OLAH, RETN, MMP8 | 0,858 | 0,848 | 0,868 | 0,897 | 0,830 | 0,964 | 0,952 | 0,906 | 0,998 |
| OAS1, ISG15, SLC1A2, OLAH, FAM20A, RETN, MMP8 | 0,858 | 0,848 | 0,868 | 0,908 | 0,848 | 0,967 | 0,937 | 0,863 | 1 |
| RSAD2, OAS1, IFIT1, SIGLEC1, ISG15, IL1R2, RETN | 0,858 | 0,849 | 0,868 | 0,897 | 0,831 | 0,963 | 0,962 | 0,921 | 1 |
| RSAD2, IFIT1, IFI44L, ISG15, IL1R2, FAM20A, MMP8 | 0,858 | 0,848 | 0,868 | 0,904 | 0,842 | 0,966 | 0,942 | 0,867 | 1 |
| RSAD2, OAS1, ISG15, SLC1A2, IL1R2, FAM20A, RETN | 0,858 | 0,848 | 0,868 | 0,905 | 0,849 | 0,962 | 0,930 | 0,844 | 1 |
| RSAD2, ISG15, SLC1A2, OLAH, FAM20A, RETN, MMP8 | 0,858 | 0,848 | 0,868 | 0,907 | 0,847 | 0,966 | 0,941 | 0,871 | 1 |
| RSAD2, OAS1, IFIT1, IFI44L, OLAH, FAM20A, MMP8 | 0,858 | 0,848 | 0,868 | 0,898 | 0,831 | 0,966 | 0,946 | 0,886 | 1 |
| IFI44L, HERC6, SLC1A2, IL1R2, OLAH, RETN, MMP8 | 0,858 | 0,848 | 0,868 | 0,908 | 0,849 | 0,966 | 0,947 | 0,897 | 0,997 |
| IFIT1, IFI44L, HERC6, SLC1A2, OLAH, RETN, MMP8 | 0,858 | 0,848 | 0,868 | 0,906 | 0,847 | 0,965 | 0,940 | 0,887 | 0,993 |
| RSAD2, IFIT1, IFI44L, SLC1A2, IL1R2, FAM20A, RETN | 0,858 | 0,849 | 0,867 | 0,907 | 0,853 | 0,962 | 0,924 | 0,835 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, IL1R2, OLAH, RETN | 0,858 | 0,848 | 0,868 | 0,898 | 0,832 | 0,963 | 0,963 | 0,923 | 1 |
| RSAD2, IFI27, OAS1, IFIT1, SLC1A2, RETN, MMP8 | 0,858 | 0,848 | 0,868 | 0,902 | 0,838 | 0,966 | 0,940 | 0,866 | 1 |
| RSAD2, OAS1, IFI44L, ISG15, IL1R2, FAM20A, RETN | 0,858 | 0,848 | 0,868 | 0,902 | 0,838 | 0,965 | 0,939 | 0,863 | 1 |
| IFIT1, IFI44L, ISG15, IL1R2, OLAH, FAM20A, RETN | 0,858 | 0,848 | 0,868 | 0,901 | 0,837 | 0,965 | 0,949 | 0,890 | 1 |
| RSAD2, IFI27, IFIT1, IFI44L, SIGLEC1, SLC1A2, MMP8 | 0,858 | 0,849 | 0,867 | 0,902 | 0,842 | 0,961 | 0,927 | 0,858 | 0,996 |
| OAS1, IFIT1, IFI44L, ISG15, IL1R2, FAM20A, RETN | 0,858 | 0,848 | 0,868 | 0,900 | 0,836 | 0,965 | 0,941 | 0,866 | 1 |
| RSAD2, OAS1, ISG15, HERC6, SLC1A2, FAM20A, RETN | 0,858 | 0,847 | 0,868 | 0,902 | 0,841 | 0,964 | 0,936 | 0,855 | 1 |
| RSAD2, OAS1, SIGLEC1, HERC6, SLC1A2, RETN, MMP8 | 0,858 | 0,848 | 0,868 | 0,903 | 0,840 | 0,965 | 0,950 | 0,900 | 1,000 |
| RSAD2, IFI27, OAS1, IFI44L, SLC1A2, RETN, MMP8 | 0,858 | 0,848 | 0,868 | 0,905 | 0,842 | 0,968 | 0,933 | 0,858 | 1 |
| RSAD2, OAS1, IFIT1, SIGLEC1, ISG15, IL1R2, MMP8 | 0,858 | 0,848 | 0,867 | 0,901 | 0,842 | 0,961 | 0,966 | 0,931 | 1 |
| RSAD2, IFI44L, SIGLEC1, HERC6, OLAH, RETN, MMP8 | 0,858 | 0,848 | 0,868 | 0,899 | 0,832 | 0,966 | 0,958 | 0,916 | 0,999 |
| OAS1, IFIT1, ISG15, HERC6, IL1R2, RETN, MMP8 | 0,858 | 0,848 | 0,868 | 0,897 | 0,832 | 0,961 | 0,960 | 0,918 | 1 |
| RSAD2, OAS1, IFIT1, ISG15, HERC6, IL1R2, RETN | 0,858 | 0,848 | 0,868 | 0,896 | 0,833 | 0,960 | 0,948 | 0,898 | 0,998 |
| RSAD2, IFIT1, SIGLEC1, ISG15, IL1R2, OLAH, RETN | 0,858 | 0,848 | 0,867 | 0,898 | 0,831 | 0,964 | 0,960 | 0,918 | 1 |
| RSAD2, IFI44L, SIGLEC1, ISG15, IL1R2, OLAH, RETN | 0,858 | 0,848 | 0,867 | 0,896 | 0,830 | 0,963 | 0,960 | 0,918 | 1 |
| IFI27, IFIT1, IFI44L, ISG15, SLC1A2, RETN, MMP8 | 0,858 | 0,847 | 0,868 | 0,902 | 0,839 | 0,965 | 0,942 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| ISG15, SLC1A2, IL1R2, OLAH, FAM20A, RETN, MMP8 | 0,858 | 0,848 | 0,867 | 0,911 | 0,857 | 0,964 | 0,923 | 0,842 | 1 |
| RSAD2, OAS1, IFIT1, SIGLEC1, OLAH, RETN, MMP8 | 0,858 | 0,847 | 0,868 | 0,896 | 0,829 | 0,964 | 0,954 | 0,910 | 0,999 |
| RSAD2, IFIT1, IFI44L, ISG15, IL1R2, OLAH, FAM20A | 0,858 | 0,848 | 0,867 | 0,900 | 0,835 | 0,965 | 0,952 | 0,892 | 1 |
| RSAD2, IFI27, IFI44L, SIGLEC1, ISG15, SLC1A2, MMP8 | 0,858 | 0,848 | 0,867 | 0,901 | 0,842 | 0,961 | 0,928 | 0,859 | 0,998 |
| OAS1, IFI44L, ISG15, HERC6, SLC1A2, OLAH, RETN | 0,858 | 0,847 | 0,868 | 0,909 | 0,852 | 0,966 | 0,940 | 0,882 | 0,998 |
| RSAD2, IFIT1, IFI44L, SIGLEC1, HERC6, SLC1A2, RETN | 0,858 | 0,849 | 0,866 | 0,903 | 0,843 | 0,963 | 0,923 | 0,862 | 0,984 |
| RSAD2, IFI27, OAS1, ISG15, SLC1A2, RETN, MMP8 | 0,858 | 0,847 | 0,868 | 0,901 | 0,837 | 0,965 | 0,932 | 0,853 | 1 |
| RSAD2, SIGLEC1, ISG15, HERC6, SLC1A2, RETN, MMP8 | 0,857 | 0,848 | 0,867 | 0,906 | 0,846 | 0,966 | 0,946 | 0,895 | 0,996 |
| RSAD2, OAS1, IFI44L, SIGLEC1, HERC6, OLAH, RETN | 0,857 | 0,848 | 0,867 | 0,896 | 0,829 | 0,963 | 0,949 | 0,901 | 0,996 |
| IFI27, OAS1, IFIT1, SIGLEC1, ISG15, HERC6, SLC1A2 | 0,857 | 0,848 | 0,867 | 0,903 | 0,848 | 0,958 | 0,945 | 0,892 | 0,997 |
| RSAD2, OAS1, IFIT1, SLC1A2, OLAH, FAM20A, RETN | 0,857 | 0,847 | 0,868 | 0,908 | 0,849 | 0,968 | 0,934 | 0,859 | 1 |
| RSAD2, IFIT1, IFI44L, SIGLEC1, ISG15, HERC6, OLAH | 0,857 | 0,848 | 0,867 | 0,900 | 0,839 | 0,962 | 0,940 | 0,888 | 0,992 |
| RSAD2, IFI27, IFIT1, IFI44L, ISG15, RETN, MMP8 | 0,857 | 0,847 | 0,868 | 0,903 | 0,839 | 0,967 | 0,954 | 0,897 | 1 |
| RSAD2, IFIT1, ISG15, IL1R2, FAM20A, RETN, MMP8 | 0,857 | 0,848 | 0,867 | 0,905 | 0,842 | 0,969 | 0,945 | 0,872 | 1 |
| RSAD2, OAS1, SIGLEC1, ISG15, HERC6, OLAH, MMP8 | 0,857 | 0,847 | 0,867 | 0,897 | 0,834 | 0,960 | 0,951 | 0,906 | 0,996 |

(suite)

| Modèle | AUC modèle | IC 95% | | AUC Train | IC 95% Train | | AUC Test | IC 95% Test | |
|---|---|---|---|---|---|---|---|---|---|
| OAS1, IFI44L, ISG15, IL1R2, FAM20A, RETN, MMP8 | 0,857 | 0,847 | 0,868 | 0,908 | 0,846 | 0,969 | 0,943 | 0,868 | 1 |
| IFIT1, SIGLEC1, ISG15, HERC6, SLC1A2, RETN, MMP8 | 0,857 | 0,848 | 0,867 | 0,902 | 0,842 | 0,962 | 0,938 | 0,884 | 0,992 |
| RSAD2, OAS1, IFIT1, IFI44L, HERC6, FAM20A, RETN | 0,857 | 0,847 | 0,867 | 0,899 | 0,834 | 0,964 | 0,925 | 0,844 | 1 |
| IFIT1, IFI44L, SIGLEC1, ISG15, HERC6, SLC1A2, RETN | 0,857 | 0,848 | 0,866 | 0,901 | 0,842 | 0,961 | 0,932 | 0,874 | 0,989 |
| IFIT1, IFI44L, SIGLEC1, ISG15, OLAH, RETN, MMP8 | 0,857 | 0,847 | 0,867 | 0,896 | 0,828 | 0,963 | 0,960 | 0,915 | 1 |
| RSAD2, ISG15, SLC1A2, IL1R2, FAM20A, RETN, MMP8 | 0,857 | 0,848 | 0,867 | 0,910 | 0,857 | 0,963 | 0,926 | 0,832 | 1 |
| RSAD2, IFI27, OAS1, IFIT1, HERC6, SLC1A2, FAM20A | 0,857 | 0,846 | 0,868 | 0,905 | 0,846 | 0,964 | 0,934 | 0,851 | 1 |
| RSAD2, OAS1, ISG15, HERC6, IL1R2, RETN, MMP8 | 0,857 | 0,847 | 0,867 | 0,900 | 0,838 | 0,963 | 0,949 | 0,898 | 1 |
| RSAD2, IFI27, IFIT1, IFI44L, ISG15, SLC1A2, RETN | 0,857 | 0,847 | 0,867 | 0,901 | 0,837 | 0,965 | 0,947 | 0,882 | 1 |
| RSAD2, IFIT1, ISG15, HERC6, SLC1A2, IL1R2, OLAH | 0,857 | 0,848 | 0,866 | 0,904 | 0,846 | 0,962 | 0,943 | 0,890 | 0,996 |
| OAS1, IFI44L, SIGLEC1, HERC6, SLC1A2, RETN, MMP8 | 0,857 | 0,847 | 0,867 | 0,903 | 0,841 | 0,965 | 0,950 | 0,900 | 1,000 |
| OAS1, IFIT1, HERC6, SLC1A2, IL1R2, OLAH, MMP8 | 0,857 | 0,847 | 0,867 | 0,903 | 0,845 | 0,960 | 0,951 | 0,904 | 0,998 |
| OAS1, ISG15, HERC6, IL1R2, OLAH, RETN, MMP8 | 0,857 | 0,847 | 0,867 | 0,902 | 0,840 | 0,964 | 0,959 | 0,918 | 1,000 |
| RSAD2, IFI27, OAS1, IFIT1, ISG15, FAM20A, MMP8 | 0,857 | 0,846 | 0,868 | 0,896 | 0,829 | 0,963 | 0,936 | 0,856 | 1 |
| OAS1, IFI44L, SIGLEC1, ISG15, HERC6, RETN, MMP8 | 0,857 | 0,847 | 0,867 | 0,897 | 0,829 | 0,964 | 0,953 | 0,907 | 1,000 |
| IFIT1, ISG15, HERC6, SLC1A2, IL1R2, OLAH, MMP8 | 0,857 | 0,847 | 0,866 | 0,904 | 0,847 | 0,962 | 0,950 | 0,903 | 0,997 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, ISG15, HERC6, SLC1A2, IL1R2, RETN | 0,857 | 0,846 | 0,867 | 0,904 | 0,846 | 0,961 | 0,943 | 0,887 | 1,000 |
| RSAD2, IFIT1, ISG15, HERC6, FAM20A, RETN, MMP8 | 0,857 | 0,847 | 0,867 | 0,904 | 0,841 | 0,966 | 0,936 | 0,859 | 1 |
| OAS1, IFIT1, IFI44L, ISG15, HERC6, IL1R2, RETN | 0,857 | 0,847 | 0,867 | 0,898 | 0,835 | 0,961 | 0,952 | 0,904 | 1 |
| RSAD2, OAS1, IFI44L, HERC6, SLC1A2, OLAH, RETN | 0,857 | 0,846 | 0,867 | 0,907 | 0,849 | 0,966 | 0,933 | 0,870 | 0,995 |
| IFI44L, ISG15, HERC6, SLC1A2, IL1R2, OLAH, RETN | 0,857 | 0,847 | 0,867 | 0,909 | 0,852 | 0,965 | 0,942 | 0,888 | 0,997 |
| RSAD2, IFI44L, SIGLEC1, ISG15, HERC6, OLAH, RETN | 0,857 | 0,847 | 0,867 | 0,897 | 0,830 | 0,964 | 0,950 | 0,903 | 0,997 |
| RSAD2, IFIT1, IFI44L, SIGLEC1, HERC6, RETN, MMP8 | 0,857 | 0,847 | 0,866 | 0,895 | 0,826 | 0,964 | 0,922 | 0,860 | 0,983 |
| RSAD2, OAS1, HERC6, IL1R2, OLAH, RETN, MMP8 | 0,857 | 0,846 | 0,867 | 0,899 | 0,835 | 0,963 | 0,958 | 0,916 | 0,999 |
| OAS1, IFIT1, ISG15, HERC6, FAM20A, RETN, MMP8 | 0,857 | 0,847 | 0,867 | 0,903 | 0,840 | 0,966 | 0,936 | 0,859 | 1 |
| RSAD2, OAS1, ISG15, HERC6, SLC1A2, IL1R2, OLAH | 0,857 | 0,847 | 0,867 | 0,905 | 0,849 | 0,962 | 0,943 | 0,887 | 1,000 |
| RSAD2, ISG15, HERC6, IL1R2, OLAH, RETN, MMP8 | 0,857 | 0,846 | 0,867 | 0,901 | 0,838 | 0,963 | 0,960 | 0,920 | 1,000 |
| OAS1, IFIT1, IFI44L, SIGLEC1, ISG15, OLAH, RETN | 0,857 | 0,847 | 0,866 | 0,894 | 0,825 | 0,964 | 0,938 | 0,882 | 0,994 |
| OAS1, ISG15, HERC6, SLC1A2, FAM20A, RETN, MMP8 | 0,857 | 0,846 | 0,867 | 0,903 | 0,842 | 0,965 | 0,936 | 0,857 | 1 |
| IFI44L, ISG15, SLC1A2, IL1R2, OLAH, RETN, MMP8 | 0,857 | 0,847 | 0,866 | 0,907 | 0,850 | 0,963 | 0,948 | 0,899 | 0,997 |
| RSAD2, IFI27, IFIT1, IFI44L, ISG15, HERC6, MMP8 | 0,857 | 0,847 | 0,866 | 0,898 | 0,840 | 0,956 | 0,893 | 0,813 | 0,974 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, HERC6, SLC1A2, RETN | 0,857 | 0,847 | 0,866 | 0,903 | 0,842 | 0,965 | 0,952 | 0,903 | 1 |
| OAS1, IFIT1, ISG15, HERC6, SLC1A2, IL1R2, OLAH | 0,856 | 0,846 | 0,867 | 0,903 | 0,846 | 0,960 | 0,947 | 0,896 | 0,997 |
| RSAD2, IFIT1, SIGLEC1, HERC6, SLC1A2, RETN, MMP8 | 0,856 | 0,847 | 0,866 | 0,903 | 0,842 | 0,964 | 0,932 | 0,875 | 0,988 |
| IFIT1, IFI44L, ISG15, HERC6, SLC1A2, OLAH, RETN | 0,856 | 0,846 | 0,866 | 0,908 | 0,851 | 0,966 | 0,934 | 0,874 | 0,994 |
| IFIT1, IFI44L, ISG15, HERC6, SLC1A2, OLAH, MMP8 | 0,856 | 0,846 | 0,866 | 0,906 | 0,849 | 0,963 | 0,938 | 0,883 | 0,993 |
| IFIT1, IFI44L, SIGLEC1, HERC6, SLC1A2, RETN, MMP8 | 0,856 | 0,847 | 0,866 | 0,902 | 0,842 | 0,962 | 0,930 | 0,873 | 0,988 |
| RSAD2, OAS1, IFIT1, HERC6, SLC1A2, IL1R2, OLAH | 0,856 | 0,846 | 0,867 | 0,904 | 0,846 | 0,961 | 0,947 | 0,895 | 0,999 |
| RSAD2, IFIT1, ISG15, HERC6, SLC1A2, FAM20A, RETN | 0,856 | 0,846 | 0,867 | 0,902 | 0,840 | 0,964 | 0,937 | 0,858 | 1 |
| RSAD2, IFIT1, ISG15, SLC1A2, OLAH, FAM20A, RETN | 0,856 | 0,847 | 0,866 | 0,908 | 0,848 | 0,967 | 0,938 | 0,866 | 1 |
| OAS1, IFIT1, ISG15, HERC6, SLC1A2, OLAH, RETN | 0,856 | 0,846 | 0,867 | 0,904 | 0,846 | 0,963 | 0,945 | 0,888 | 1 |
| IFIT1, HERC6, SLC1A2, IL1R2, OLAH, RETN, MMP8 | 0,856 | 0,846 | 0,866 | 0,906 | 0,849 | 0,964 | 0,955 | 0,911 | 1,000 |
| RSAD2, HERC6, SLC1A2, IL1R2, OLAH, RETN, MMP8 | 0,856 | 0,847 | 0,866 | 0,906 | 0,848 | 0,963 | 0,943 | 0,890 | 0,997 |
| RSAD2, ISG15, HERC6, SLC1A2, IL1R2, OLAH, RETN | 0,856 | 0,846 | 0,866 | 0,906 | 0,849 | 0,963 | 0,942 | 0,886 | 0,999 |
| RSAD2, IFIT1, HERC6, SLC1A2, IL1R2, OLAH, MMP8 | 0,856 | 0,846 | 0,866 | 0,904 | 0,847 | 0,962 | 0,950 | 0,903 | 0,997 |
| RSAD2, IFI44L, HERC6, SLC1A2, IL1R2, OLAH, RETN | 0,856 | 0,846 | 0,866 | 0,905 | 0,848 | 0,963 | 0,936 | 0,876 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, ISG15, HERC6, IL1R2, RETN | 0,856 | 0,846 | 0,866 | 0,898 | 0,835 | 0,961 | 0,952 | 0,904 | 1 |
| OAS1, ISG15, HERC6, SLC1A2, IL1R2, OLAH, RETN | 0,856 | 0,846 | 0,867 | 0,906 | 0,849 | 0,963 | 0,936 | 0,871 | 1 |
| OAS1, IFIT1, HERC6, SLC1A2, OLAH, RETN, MMP8 | 0,856 | 0,846 | 0,866 | 0,901 | 0,840 | 0,962 | 0,951 | 0,904 | 0,998 |
| IFIT1, IFI44L, ISG15, SLC1A2, IL1R2, OLAH, MMP8 | 0,856 | 0,847 | 0,865 | 0,904 | 0,847 | 0,961 | 0,948 | 0,900 | 0,996 |
| RSAD2, OAS1, HERC6, SLC1A2, IL1R2, OLAH, MMP8 | 0,856 | 0,847 | 0,866 | 0,904 | 0,846 | 0,961 | 0,946 | 0,892 | 0,999 |
| IFI44L, ISG15, HERC6, SLC1A2, IL1R2, OLAH, MMP8 | 0,856 | 0,847 | 0,866 | 0,908 | 0,852 | 0,964 | 0,945 | 0,892 | 0,997 |
| RSAD2, OAS1, IFI44L, SIGLEC1, HERC6, OLAH, MMP8 | 0,856 | 0,846 | 0,866 | 0,897 | 0,834 | 0,960 | 0,948 | 0,900 | 0,995 |
| OAS1, ISG15, HERC6, SLC1A2, OLAH, RETN, MMP8 | 0,856 | 0,846 | 0,866 | 0,905 | 0,846 | 0,963 | 0,953 | 0,906 | 1 |
| IFIT1, IFI44L, HERC6, SLC1A2, IL1R2, OLAH, MMP8 | 0,856 | 0,846 | 0,866 | 0,907 | 0,850 | 0,963 | 0,948 | 0,898 | 0,998 |
| RSAD2, OAS1, ISG15, HERC6, IL1R2, OLAH, RETN | 0,856 | 0,846 | 0,866 | 0,900 | 0,837 | 0,962 | 0,952 | 0,905 | 0,999 |
| OAS1, IFIT1, IFI44L, SIGLEC1, ISG15, OLAH, MMP8 | 0,856 | 0,846 | 0,866 | 0,893 | 0,825 | 0,961 | 0,943 | 0,892 | 0,995 |
| IFI44L, ISG15, HERC6, SLC1A2, OLAH, RETN, MMP8 | 0,856 | 0,846 | 0,866 | 0,907 | 0,848 | 0,966 | 0,949 | 0,902 | 0,996 |
| OAS1, IFI44L, ISG15, HERC6, IL1R2, RETN, MMP8 | 0,856 | 0,846 | 0,866 | 0,898 | 0,835 | 0,961 | 0,954 | 0,908 | 1 |
| OAS1, IFIT1, IFI44L, HERC6, SLC1A2, OLAH, MMP8 | 0,856 | 0,846 | 0,866 | 0,905 | 0,848 | 0,962 | 0,935 | 0,877 | 0,992 |
| IFI44L, ISG15, HERC6, IL1R2, OLAH, RETN, MMP8 | 0,856 | 0,846 | 0,866 | 0,900 | 0,836 | 0,963 | 0,963 | 0,925 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SIGLEC1, OLAH, RETN | 0,856 | 0,846 | 0,866 | 0,895 | 0,825 | 0,964 | 0,945 | 0,891 | 0,998 |
| RSAD2, IFI44L, IL1R2, OLAH, FAM20A, RETN, MMP8 | 0,856 | 0,846 | 0,866 | 0,906 | 0,844 | 0,967 | 0,953 | 0,893 | 1 |
| OAS1, IFIT1, IFI44L, ISG15, HERC6, SLC1A2, OLAH | 0,856 | 0,846 | 0,866 | 0,906 | 0,849 | 0,963 | 0,936 | 0,879 | 0,993 |
| RSAD2, OAS1, HERC6, SLC1A2, IL1R2, OLAH, RETN | 0,856 | 0,846 | 0,866 | 0,903 | 0,845 | 0,962 | 0,939 | 0,876 | 1 |
| RSAD2, IFIT1, IFI44L, HERC6, SLC1A2, OLAH, RETN | 0,856 | 0,846 | 0,866 | 0,908 | 0,851 | 0,965 | 0,928 | 0,867 | 0,990 |
| OAS1, HERC6, SLC1A2, IL1R2, OLAH, RETN, MMP8 | 0,856 | 0,846 | 0,866 | 0,907 | 0,850 | 0,964 | 0,939 | 0,882 | 0,996 |
| RSAD2, IFI27, IFIT1, ISG15, SLC1A2, RETN, MMP8 | 0,856 | 0,846 | 0,866 | 0,901 | 0,838 | 0,964 | 0,942 | 0,874 | 1 |
| OAS1, IFIT1, IFI44L, HERC6, IL1R2, RETN, MMP8 | 0,856 | 0,846 | 0,866 | 0,895 | 0,827 | 0,962 | 0,959 | 0,918 | 1,000 |
| RSAD2, IFI27, IFI44L, ISG15, SLC1A2, RETN, MMP8 | 0,856 | 0,846 | 0,866 | 0,903 | 0,841 | 0,966 | 0,940 | 0,872 | 1 |
| RSAD2, OAS1, ISG15, HERC6, SLC1A2, OLAH, RETN | 0,856 | 0,846 | 0,866 | 0,905 | 0,847 | 0,964 | 0,939 | 0,876 | 1 |
| RSAD2, IFIT1, ISG15, IL1R2, OLAH, FAM20A, RETN | 0,856 | 0,846 | 0,866 | 0,898 | 0,832 | 0,964 | 0,949 | 0,884 | 1 |
| OAS1, IFIT1, HERC6, SLC1A2, IL1R2, OLAH, RETN | 0,856 | 0,845 | 0,866 | 0,904 | 0,846 | 0,962 | 0,940 | 0,883 | 0,997 |
| RSAD2, OAS1, IFIT1, SLC1A2, IL1R2, OLAH, MMP8 | 0,856 | 0,846 | 0,865 | 0,903 | 0,846 | 0,960 | 0,951 | 0,905 | 0,997 |
| RSAD2, IFI27, OAS1, SIGLEC1, ISG15, HERC6, SLC1A2 | 0,856 | 0,846 | 0,865 | 0,902 | 0,847 | 0,957 | 0,945 | 0,892 | 0,997 |
| RSAD2, OAS1, IFIT1, HERC6, IL1R2, RETN, MMP8 | 0,856 | 0,846 | 0,866 | 0,895 | 0,827 | 0,962 | 0,960 | 0,919 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, HERC6, SLC1A2, IL1R2, OLAH | 0,856 | 0,845 | 0,866 | 0,907 | 0,851 | 0,963 | 0,941 | 0,885 | 0,997 |
| IFI27, OAS1, IFIT1, IFI44L, ISG15, SLC1A2, FAM20A | 0,856 | 0,845 | 0,866 | 0,900 | 0,834 | 0,965 | 0,941 | 0,863 | 1 |
| OAS1, IFI44L, ISG15, HERC6, FAM20A, RETN, MMP8 | 0,856 | 0,845 | 0,866 | 0,903 | 0,839 | 0,968 | 0,942 | 0,870 | 1 |
| RSAD2, OAS1, HERC6, SLC1A2, OLAH, RETN, MMP8 | 0,856 | 0,846 | 0,865 | 0,902 | 0,842 | 0,963 | 0,947 | 0,894 | 0,999 |
| OAS1, IFI44L, SIGLEC1, ISG15, HERC6, OLAH, MMP8 | 0,856 | 0,846 | 0,866 | 0,896 | 0,833 | 0,959 | 0,953 | 0,909 | 0,998 |
| RSAD2, OAS1, IFIT1, SIGLEC1, ISG15, OLAH, RETN | 0,856 | 0,846 | 0,865 | 0,895 | 0,826 | 0,964 | 0,946 | 0,893 | 0,998 |
| OAS1, IFIT1, ISG15, HERC6, IL1R2, OLAH, RETN | 0,856 | 0,845 | 0,866 | 0,900 | 0,837 | 0,962 | 0,950 | 0,902 | 0,998 |
| RSAD2, IFI44L, ISG15, HERC6, SLC1A2, IL1R2, OLAH | 0,856 | 0,845 | 0,866 | 0,907 | 0,851 | 0,963 | 0,942 | 0,889 | 0,996 |
| RSAD2, IFIT1, IFI44L, ISG15, HERC6, IL1R2, RETN | 0,856 | 0,846 | 0,865 | 0,900 | 0,837 | 0,962 | 0,949 | 0,900 | 0,997 |
| IFI27, OAS1, IFI44L, SIGLEC1, ISG15, HERC6, SLC1A2 | 0,856 | 0,847 | 0,864 | 0,902 | 0,847 | 0,957 | 0,947 | 0,895 | 0,998 |
| OAS1, IFI44L, HERC6, SLC1A2, IL1R2, OLAH, RETN | 0,856 | 0,845 | 0,866 | 0,906 | 0,849 | 0,964 | 0,934 | 0,873 | 0,995 |
| RSAD2, IFI44L, ISG15, HERC6, SLC1A2, OLAH, RETN | 0,856 | 0,845 | 0,866 | 0,909 | 0,852 | 0,966 | 0,939 | 0,885 | 0,994 |
| OAS1, IFIT1, HERC6, IL1R2, OLAH, RETN, MMP8 | 0,856 | 0,845 | 0,866 | 0,899 | 0,835 | 0,963 | 0,962 | 0,924 | 1 |
| RSAD2, IFIT1, ISG15, HERC6, IL1R2, RETN, MMP8 | 0,856 | 0,845 | 0,866 | 0,897 | 0,832 | 0,962 | 0,957 | 0,913 | 1,000 |
| IFIT1, IFI44L, ISG15, HERC6, SLC1A2, IL1R2, OLAH | 0,855 | 0,846 | 0,865 | 0,907 | 0,851 | 0,963 | 0,945 | 0,892 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, ISG15, HERC6, SLC1A2, IL1R2, OLAH, MMP8 | 0,855 | 0,846 | 0,865 | 0,906 | 0,849 | 0,963 | 0,946 | 0,893 | 0,998 |
| RSAD2, OAS1, IFI44L, ISG15, HERC6, SLC1A2, FAM20A | 0,855 | 0,845 | 0,866 | 0,900 | 0,839 | 0,960 | 0,924 | 0,841 | 1 |
| RSAD2, OAS1, IFIT1, IFI44L, SIGLEC1, OLAH, MMP8 | 0,855 | 0,846 | 0,865 | 0,893 | 0,825 | 0,961 | 0,946 | 0,895 | 0,996 |
| OAS1, IFIT1, IFI44L, ISG15, HERC6, OLAH, RETN | 0,855 | 0,845 | 0,865 | 0,897 | 0,832 | 0,961 | 0,946 | 0,896 | 0,995 |
| RSAD2, ISG15, HERC6, SLC1A2, FAM20A, RETN, MMP8 | 0,855 | 0,845 | 0,866 | 0,903 | 0,841 | 0,965 | 0,935 | 0,856 | 1 |
| IFIT1, IFI44L, ISG15, HERC6, IL1R2, RETN, MMP8 | 0,855 | 0,846 | 0,865 | 0,895 | 0,830 | 0,961 | 0,960 | 0,919 | 1 |
| IFIT1, ISG15, HERC6, SLC1A2, IL1R2, OLAH, RETN | 0,855 | 0,845 | 0,865 | 0,906 | 0,849 | 0,964 | 0,943 | 0,888 | 0,999 |
| OAS1, ISG15, HERC6, SLC1A2, IL1R2, OLAH, MMP8 | 0,855 | 0,846 | 0,865 | 0,905 | 0,849 | 0,962 | 0,942 | 0,888 | 0,996 |
| RSAD2, IFIT1, IFI44L, SIGLEC1, OLAH, RETN, MMP8 | 0,855 | 0,845 | 0,865 | 0,896 | 0,828 | 0,963 | 0,959 | 0,916 | 1 |
| RSAD2, IFI44L, HERC6, SLC1A2, IL1R2, OLAH, MMP8 | 0,855 | 0,845 | 0,865 | 0,908 | 0,852 | 0,963 | 0,945 | 0,893 | 0,996 |
| RSAD2, OAS1, IFI44L, ISG15, OLAH, FAM20A, MMP8 | 0,855 | 0,844 | 0,866 | 0,898 | 0,830 | 0,966 | 0,948 | 0,889 | 1 |
| OAS1, IFI44L, ISG15, HERC6, SLC1A2, IL1R2, OLAH | 0,855 | 0,845 | 0,866 | 0,907 | 0,852 | 0,962 | 0,939 | 0,881 | 0,997 |
| RSAD2, OAS1, ISG15, HERC6, OLAH, RETN, MMP8 | 0,855 | 0,845 | 0,866 | 0,896 | 0,830 | 0,962 | 0,959 | 0,918 | 0,999 |
| RSAD2, IFI44L, ISG15, IL1R2, OLAH, FAM20A, RETN | 0,855 | 0,845 | 0,866 | 0,900 | 0,835 | 0,965 | 0,948 | 0,885 | 1 |
| IFIT1, IFI44L, ISG15, SLC1A2, IL1R2, OLAH, RETN | 0,855 | 0,845 | 0,865 | 0,910 | 0,854 | 0,966 | 0,946 | 0,896 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, HERC6, SLC1A2, OLAH, MMP8 | 0,855 | 0,845 | 0,866 | 0,905 | 0,849 | 0,962 | 0,938 | 0,883 | 0,993 |
| IFIT1, ISG15, HERC6, IL1R2, OLAH, RETN, MMP8 | 0,855 | 0,845 | 0,865 | 0,900 | 0,837 | 0,963 | 0,962 | 0,924 | 1 |
| IFIT1, IFI44L, HERC6, SLC1A2, IL1R2, OLAH, RETN | 0,855 | 0,845 | 0,865 | 0,907 | 0,850 | 0,964 | 0,936 | 0,876 | 0,995 |
| OAS1, IFI44L, ISG15, HERC6, SLC1A2, IL1R2, RETN | 0,855 | 0,845 | 0,865 | 0,906 | 0,850 | 0,962 | 0,942 | 0,883 | 1 |
| OAS1, IFIT1, HERC6, SLC1A2, IL1R2, RETN, MMP8 | 0,855 | 0,845 | 0,865 | 0,904 | 0,846 | 0,963 | 0,952 | 0,903 | 1 |
| OAS1, IFI44L, ISG15, HERC6, IL1R2, OLAH, RETN | 0,855 | 0,845 | 0,865 | 0,899 | 0,836 | 0,962 | 0,952 | 0,905 | 0,999 |
| IFI27, OAS1, IFIT1, IFI44L, SIGLEC1, ISG15, MMP8 | 0,855 | 0,845 | 0,865 | 0,890 | 0,824 | 0,956 | 0,912 | 0,837 | 0,987 |
| RSAD2, IFIT1, IFI44L, IL1R2, FAM20A, RETN, MMP8 | 0,855 | 0,845 | 0,865 | 0,904 | 0,841 | 0,967 | 0,946 | 0,873 | 1 |
| OAS1, IFI44L, HERC6, SLC1A2, IL1R2, OLAH, MMP8 | 0,855 | 0,845 | 0,865 | 0,908 | 0,853 | 0,963 | 0,942 | 0,888 | 0,997 |
| OAS1, IFIT1, ISG15, SLC1A2, IL1R2, OLAH, MMP8 | 0,855 | 0,846 | 0,864 | 0,904 | 0,846 | 0,961 | 0,948 | 0,899 | 0,996 |
| RSAD2, IFI44L, SIGLEC1, ISG15, HERC6, SLC1A2, RETN | 0,855 | 0,846 | 0,864 | 0,907 | 0,847 | 0,967 | 0,945 | 0,893 | 0,996 |
| RSAD2, IFI44L, HERC6, SLC1A2, OLAH, RETN, MMP8 | 0,855 | 0,845 | 0,865 | 0,905 | 0,846 | 0,965 | 0,937 | 0,882 | 0,992 |
| OAS1, IFIT1, ISG15, HERC6, SLC1A2, IL1R2, MMP8 | 0,855 | 0,845 | 0,865 | 0,903 | 0,846 | 0,959 | 0,952 | 0,903 | 1 |
| RSAD2, OAS1, IFIT1, HERC6, SLC1A2, IL1R2, MMP8 | 0,855 | 0,845 | 0,864 | 0,902 | 0,845 | 0,959 | 0,951 | 0,902 | 1 |
| RSAD2, OAS1, IFIT1, ISG15, HERC6, SLC1A2, IL1R2 | 0,855 | 0,845 | 0,864 | 0,901 | 0,844 | 0,958 | 0,951 | 0,901 | 1 |

(suite)

| Modèle | AUC modèle | IC 95% | | AUC Train | IC 95% Train | | AUC Test | IC 95% Test | |
|---|---|---|---|---|---|---|---|---|---|
| ISG15, HERC6, SLC1A2, IL1R2, OLAH, RETN, MMP8 | 0,855 | 0,845 | 0,864 | 0,908 | 0,853 | 0,964 | 0,951 | 0,902 | 1 |
| OAS1, IFIT1, SLC1A2, IL1R2, OLAH, RETN, MMP8 | 0,855 | 0,845 | 0,865 | 0,905 | 0,847 | 0,963 | 0,957 | 0,914 | 0,999 |
| RSAD2, OAS1, ISG15, OLAH, FAM20A, RETN, MMP8 | 0,855 | 0,844 | 0,865 | 0,897 | 0,829 | 0,966 | 0,952 | 0,894 | 1 |
| RSAD2, IFIT1, IFI44L, HERC6, SLC1A2, OLAH, MMP8 | 0,855 | 0,845 | 0,865 | 0,905 | 0,848 | 0,962 | 0,932 | 0,873 | 0,990 |
| IFIT1, IFI44L, SLC1A2, IL1R2, OLAH, RETN, MMP8 | 0,855 | 0,845 | 0,864 | 0,906 | 0,848 | 0,964 | 0,954 | 0,911 | 0,998 |
| IFIT1, IFI44L, ISG15, SLC1A2, OLAH, RETN, MMP8 | 0,855 | 0,845 | 0,864 | 0,906 | 0,846 | 0,967 | 0,948 | 0,900 | 0,995 |
| RSAD2, IFI27, OAS1, IFIT1, SIGLEC1, HERC6, SLC1A2 | 0,855 | 0,845 | 0,864 | 0,902 | 0,847 | 0,957 | 0,945 | 0,892 | 0,997 |
| RSAD2, OAS1, IFIT1, IFI44L, HERC6, SLC1A2, OLAH | 0,855 | 0,845 | 0,865 | 0,905 | 0,848 | 0,962 | 0,933 | 0,873 | 0,992 |
| OAS1, IFI44L, ISG15, HERC6, SLC1A2, OLAH, MMP8 | 0,855 | 0,844 | 0,865 | 0,905 | 0,848 | 0,963 | 0,936 | 0,879 | 0,993 |
| RSAD2, IFIT1, IFI44L, HERC6, SLC1A2, IL1R2, OLAH | 0,855 | 0,845 | 0,865 | 0,905 | 0,849 | 0,962 | 0,943 | 0,891 | 0,996 |
| RSAD2, OAS1, IFIT1, IFI44L, ISG15, OLAH, FAM20A | 0,855 | 0,844 | 0,865 | 0,895 | 0,825 | 0,965 | 0,945 | 0,886 | 1 |
| RSAD2, OAS1, IFI44L, ISG15, HERC6, SLC1A2, OLAH | 0,855 | 0,844 | 0,865 | 0,907 | 0,850 | 0,963 | 0,937 | 0,881 | 0,993 |
| OAS1, IFIT1, ISG15, SLC1A2, IL1R2, OLAH, RETN | 0,855 | 0,844 | 0,865 | 0,908 | 0,850 | 0,965 | 0,946 | 0,895 | 0,996 |
| OAS1, IFIT1, IFI44L, HERC6, SLC1A2, FAM20A, RETN | 0,855 | 0,845 | 0,865 | 0,902 | 0,840 | 0,964 | 0,926 | 0,843 | 1 |
| RSAD2, IFI27, OAS1, IFIT1, IFI44L, SIGLEC1, MMP8 | 0,855 | 0,845 | 0,864 | 0,891 | 0,824 | 0,957 | 0,917 | 0,843 | 0,992 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, HERC6, IL1R2, OLAH, RETN, MMP8 | 0,855 | 0,844 | 0,865 | 0,899 | 0,835 | 0,963 | 0,962 | 0,924 | 1 |
| RSAD2, OAS1, IFIT1, ISG15, OLAH, FAM20A, MMP8 | 0,855 | 0,844 | 0,865 | 0,892 | 0,822 | 0,962 | 0,951 | 0,890 | 1 |
| RSAD2, OAS1, IFIT1, OLAH, FAM20A, RETN, MMP8 | 0,854 | 0,844 | 0,865 | 0,893 | 0,823 | 0,963 | 0,953 | 0,896 | 1 |
| RSAD2, OAS1, ISG15, HERC6, SLC1A2, IL1R2, RETN | 0,854 | 0,844 | 0,864 | 0,901 | 0,843 | 0,958 | 0,938 | 0,873 | 1 |
| RSAD2, OAS1, ISG15, HERC6, IL1R2, OLAH, MMP8 | 0,854 | 0,845 | 0,864 | 0,899 | 0,840 | 0,958 | 0,948 | 0,900 | 0,996 |
| IFIT1, ISG15, HERC6, SLC1A2, OLAH, RETN, MMP8 | 0,854 | 0,844 | 0,865 | 0,907 | 0,848 | 0,965 | 0,954 | 0,909 | 1,000 |
| OAS1, IFIT1, ISG15, HERC6, OLAH, RETN, MMP8 | 0,854 | 0,844 | 0,865 | 0,895 | 0,828 | 0,962 | 0,960 | 0,920 | 1,000 |
| RSAD2, IFI27, IFIT1, IFI44L, SLC1A2, RETN, MMP8 | 0,854 | 0,844 | 0,864 | 0,902 | 0,838 | 0,965 | 0,941 | 0,873 | 1 |
| RSAD2, IFIT1, IFI44L, ISG15, HERC6, SLC1A2, OLAH | 0,854 | 0,845 | 0,864 | 0,907 | 0,851 | 0,964 | 0,936 | 0,880 | 0,992 |
| RSAD2, IFIT1, ISG15, SLC1A2, IL1R2, OLAH, MMP8 | 0,854 | 0,845 | 0,863 | 0,904 | 0,846 | 0,961 | 0,950 | 0,903 | 0,997 |
| RSAD2, OAS1, IFIT1, IFI44L, SLC1A2, OLAH, MMP8 | 0,854 | 0,844 | 0,864 | 0,906 | 0,848 | 0,964 | 0,938 | 0,884 | 0,992 |
| IFI27, IFIT1, IFI44L, ISG15, HERC6, SLC1A2, MMP8 | 0,854 | 0,845 | 0,864 | 0,900 | 0,842 | 0,957 | 0,899 | 0,816 | 0,982 |
| RSAD2, IFIT1, ISG15, HERC6, SLC1A2, IL1R2, RETN | 0,854 | 0,845 | 0,864 | 0,904 | 0,846 | 0,962 | 0,946 | 0,891 | 1,000 |
| RSAD2, OAS1, IFIT1, IFI44L, SIGLEC1, ISG15, OLAH | 0,854 | 0,845 | 0,864 | 0,894 | 0,826 | 0,962 | 0,945 | 0,893 | 0,996 |
| RSAD2, OAS1, IFIT1, ISG15, OLAH, FAM20A, RETN | 0,854 | 0,844 | 0,865 | 0,895 | 0,824 | 0,965 | 0,950 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, SLC1A2, FAM20A, MMP8 | 0,854 | 0,844 | 0,865 | 0,895 | 0,830 | 0,960 | 0,929 | 0,846 | 1 |
| RSAD2, OAS1, IFIT1, HERC6, SLC1A2, OLAH, RETN | 0,854 | 0,844 | 0,864 | 0,901 | 0,841 | 0,961 | 0,940 | 0,879 | 1 |
| RSAD2, OAS1, IFIT1, ISG15, HERC6, IL1R2, MMP8 | 0,854 | 0,845 | 0,864 | 0,897 | 0,838 | 0,957 | 0,949 | 0,900 | 0,997 |
| RSAD2, IFIT1, HERC6, SLC1A2, IL1R2, OLAH, RETN | 0,854 | 0,844 | 0,864 | 0,904 | 0,846 | 0,962 | 0,940 | 0,883 | 0,997 |
| IFI27, OAS1, IFIT1, ISG15, SLC1A2, FAM20A, MMP8 | 0,854 | 0,843 | 0,865 | 0,897 | 0,832 | 0,962 | 0,933 | 0,852 | 1 |
| RSAD2, ISG15, HERC6, SLC1A2, OLAH, RETN, MMP8 | 0,854 | 0,844 | 0,864 | 0,904 | 0,846 | 0,963 | 0,950 | 0,902 | 0,998 |
| RSAD2, IFIT1, SIGLEC1, ISG15, OLAH, RETN, MMP8 | 0,854 | 0,844 | 0,864 | 0,896 | 0,828 | 0,964 | 0,963 | 0,922 | 1 |
| RSAD2, OAS1, IFIT1, IFI44L, HERC6, IL1R2, MMP8 | 0,854 | 0,845 | 0,864 | 0,895 | 0,833 | 0,958 | 0,949 | 0,902 | 0,996 |
| IFI27, OAS1, IFIT1, IFI44L, SIGLEC1, HERC6, SLC1A2 | 0,854 | 0,845 | 0,863 | 0,901 | 0,846 | 0,957 | 0,946 | 0,893 | 0,998 |
| RSAD2, IFIT1, IFI44L, HERC6, SLC1A2, FAM20A, RETN | 0,854 | 0,844 | 0,864 | 0,903 | 0,842 | 0,963 | 0,923 | 0,840 | 1 |
| OAS1, IFIT1, IFI44L, HERC6, FAM20A, RETN, MMP8 | 0,854 | 0,844 | 0,864 | 0,897 | 0,829 | 0,966 | 0,933 | 0,853 | 1 |
| RSAD2, OAS1, IFIT1, HERC6, IL1R2, OLAH, MMP8 | 0,854 | 0,844 | 0,864 | 0,899 | 0,839 | 0,959 | 0,954 | 0,911 | 0,998 |
| RSAD2, OAS1, IFIT1, IFI44L, HERC6, IL1R2, RETN | 0,854 | 0,844 | 0,864 | 0,893 | 0,827 | 0,960 | 0,946 | 0,896 | 0,995 |
| IFIT1, IFI44L, ISG15, HERC6, OLAH, RETN, MMP8 | 0,854 | 0,844 | 0,864 | 0,895 | 0,826 | 0,964 | 0,955 | 0,913 | 0,998 |
| RSAD2, IFIT1, HERC6, SLC1A2, OLAH, RETN, MMP8 | 0,854 | 0,844 | 0,864 | 0,902 | 0,841 | 0,963 | 0,950 | 0,903 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, ISG15, HERC6, SLC1A2, FAM20A | 0,854 | 0,844 | 0,864 | 0,903 | 0,844 | 0,961 | 0,914 | 0,825 | 1 |
| RSAD2, IFI44L, ISG15, HERC6, IL1R2, RETN, MMP8 | 0,854 | 0,844 | 0,864 | 0,899 | 0,836 | 0,962 | 0,955 | 0,911 | 1 |
| IFIT1, IFI44L, ISG15, HERC6, IL1R2, OLAH, RETN | 0,854 | 0,844 | 0,864 | 0,900 | 0,836 | 0,964 | 0,948 | 0,899 | 0,996 |
| RSAD2, IFI44L, ISG15, HERC6, IL1R2, OLAH, RETN | 0,854 | 0,844 | 0,864 | 0,901 | 0,839 | 0,964 | 0,950 | 0,903 | 0,997 |
| RSAD2, OAS1, IFIT1, HERC6, SLC1A2, OLAH, MMP8 | 0,854 | 0,844 | 0,864 | 0,903 | 0,845 | 0,960 | 0,949 | 0,898 | 1,000 |
| OAS1, IFIT1, IFI44L, HERC6, OLAH, RETN, MMP8 | 0,854 | 0,844 | 0,864 | 0,893 | 0,824 | 0,962 | 0,955 | 0,913 | 0,998 |
| RSAD2, IFI44L, ISG15, HERC6, SLC1A2, FAM20A, MMP8 | 0,854 | 0,843 | 0,865 | 0,902 | 0,843 | 0,962 | 0,923 | 0,840 | 1 |
| RSAD2, IFIT1, ISG15, HERC6, SLC1A2, IL1R2, MMP8 | 0,854 | 0,844 | 0,863 | 0,902 | 0,845 | 0,959 | 0,952 | 0,904 | 1 |
| RSAD2, OAS1, IFIT1, HERC6, SLC1A2, IL1R2, RETN | 0,854 | 0,844 | 0,864 | 0,901 | 0,843 | 0,960 | 0,939 | 0,878 | 1 |
| RSAD2, IFI27, IFIT1, ISG15, SLC1A2, FAM20A, MMP8 | 0,854 | 0,843 | 0,864 | 0,896 | 0,831 | 0,961 | 0,934 | 0,853 | 1 |
| OAS1, IFI44L, HERC6, IL1R2, OLAH, RETN, MMP8 | 0,854 | 0,844 | 0,864 | 0,899 | 0,836 | 0,963 | 0,960 | 0,920 | 0,999 |
| OAS1, ISG15, HERC6, SLC1A2, IL1R2, RETN, MMP8 | 0,854 | 0,844 | 0,864 | 0,904 | 0,847 | 0,960 | 0,934 | 0,864 | 1 |
| RSAD2, OAS1, IFI44L, HERC6, IL1R2, OLAH, RETN | 0,854 | 0,844 | 0,864 | 0,898 | 0,833 | 0,963 | 0,948 | 0,899 | 0,997 |
| RSAD2, OAS1, IFI44L, SIGLEC1, ISG15, HERC6, OLAH | 0,854 | 0,844 | 0,864 | 0,898 | 0,835 | 0,961 | 0,952 | 0,907 | 0,997 |
| RSAD2, OAS1, IFIT1, SIGLEC1, ISG15, OLAH, MMP8 | 0,854 | 0,844 | 0,864 | 0,894 | 0,826 | 0,962 | 0,943 | 0,892 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, HERC6, SLC1A2, FAM20A, RETN | 0,854 | 0,843 | 0,864 | 0,901 | 0,839 | 0,963 | 0,929 | 0,850 | 1 |
| RSAD2, IFIT1, IFI44L, HERC6, IL1R2, RETN, MMP8 | 0,854 | 0,844 | 0,864 | 0,894 | 0,826 | 0,962 | 0,960 | 0,920 | 1,000 |
| OAS1, IFIT1, IFI44L, HERC6, SLC1A2, IL1R2, OLAH | 0,854 | 0,843 | 0,864 | 0,907 | 0,851 | 0,963 | 0,945 | 0,892 | 0,997 |
| RSAD2, IFI44L, ISG15, HERC6, SLC1A2, IL1R2, RETN | 0,854 | 0,844 | 0,863 | 0,907 | 0,852 | 0,963 | 0,943 | 0,886 | 1 |
| RSAD2, IFI27, OAS1, IFI44L, SIGLEC1, HERC6, SLC1A2 | 0,854 | 0,845 | 0,863 | 0,901 | 0,846 | 0,957 | 0,947 | 0,895 | 0,999 |
| RSAD2, OAS1, IFI44L, OLAH, FAM20A, RETN, MMP8 | 0,854 | 0,843 | 0,864 | 0,901 | 0,835 | 0,967 | 0,946 | 0,887 | 1 |
| RSAD2, IFIT1, IFI44L, SLC1A2, IL1R2, OLAH, MMP8 | 0,854 | 0,844 | 0,863 | 0,905 | 0,849 | 0,961 | 0,950 | 0,903 | 0,997 |
| IFIT1, ISG15, SLC1A2, IL1R2, OLAH, RETN, MMP8 | 0,854 | 0,844 | 0,863 | 0,907 | 0,849 | 0,964 | 0,957 | 0,914 | 0,999 |
| OAS1, IFIT1, IFI44L, HERC6, SLC1A2, IL1R2, MMP8 | 0,854 | 0,844 | 0,863 | 0,903 | 0,847 | 0,959 | 0,951 | 0,903 | 1,000 |
| IFIT1, IFI44L, HERC6, SLC1A2, FAM20A, RETN, MMP8 | 0,854 | 0,843 | 0,864 | 0,901 | 0,838 | 0,964 | 0,930 | 0,849 | 1 |
| RSAD2, IFIT1, IFI44L, ISG15, IL1R2, FAM20A, RETN | 0,854 | 0,844 | 0,864 | 0,897 | 0,832 | 0,962 | 0,945 | 0,875 | 1 |
| RSAD2, IFIT1, IFI44L, ISG15, HERC6, SLC1A2, IL1R2 | 0,854 | 0,844 | 0,863 | 0,901 | 0,845 | 0,958 | 0,950 | 0,900 | 1,000 |
| RSAD2, IFIT1, ISG15, HERC6, IL1R2, OLAH, RETN | 0,854 | 0,844 | 0,864 | 0,899 | 0,836 | 0,962 | 0,951 | 0,905 | 0,997 |
| RSAD2, OAS1, IFI44L, SIGLEC1, OLAH, RETN, MMP8 | 0,854 | 0,843 | 0,864 | 0,893 | 0,824 | 0,963 | 0,958 | 0,916 | 0,999 |
| OAS1, IFIT1, IFI44L, ISG15, SLC1A2, OLAH, MMP8 | 0,854 | 0,844 | 0,863 | 0,904 | 0,845 | 0,962 | 0,938 | 0,884 | 0,992 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, ISG15, HERC6, IL1R2, OLAH | 0,854 | 0,844 | 0,863 | 0,899 | 0,839 | 0,959 | 0,951 | 0,905 | 0,997 |
| RSAD2, IFI27, IFI44L, ISG15, HERC6, SLC1A2, MMP8 | 0,854 | 0,844 | 0,863 | 0,900 | 0,843 | 0,957 | 0,904 | 0,827 | 0,982 |
| IFIT1, ISG15, HERC6, SLC1A2, FAM20A, RETN, MMP8 | 0,854 | 0,843 | 0,864 | 0,901 | 0,838 | 0,964 | 0,940 | 0,864 | 1 |
| RSAD2, IFIT1, ISG15, HERC6, SLC1A2, OLAH, RETN | 0,853 | 0,843 | 0,864 | 0,904 | 0,845 | 0,963 | 0,943 | 0,888 | 0,999 |
| RSAD2, IFI27, OAS1, IFIT1, SLC1A2, FAM20A, MMP8 | 0,853 | 0,843 | 0,864 | 0,897 | 0,832 | 0,962 | 0,933 | 0,852 | 1 |
| RSAD2, IFI27, IFIT1, IFI44L, HERC6, SLC1A2, MMP8 | 0,853 | 0,844 | 0,863 | 0,901 | 0,844 | 0,958 | 0,899 | 0,818 | 0,980 |
| OAS1, IFIT1, IFI44L, SLC1A2, IL1R2, OLAH, MMP8 | 0,853 | 0,844 | 0,863 | 0,904 | 0,847 | 0,961 | 0,950 | 0,903 | 0,997 |
| RSAD2, IFI44L, ISG15, HERC6, SLC1A2, OLAH, MMP8 | 0,853 | 0,843 | 0,863 | 0,906 | 0,849 | 0,963 | 0,936 | 0,880 | 0,991 |
| RSAD2, OAS1, IFI44L, ISG15, HERC6, OLAH, RETN | 0,853 | 0,843 | 0,864 | 0,897 | 0,833 | 0,962 | 0,949 | 0,902 | 0,996 |
| RSAD2, IFI44L, SIGLEC1, ISG15, HERC6, OLAH, MMP8 | 0,853 | 0,844 | 0,863 | 0,899 | 0,836 | 0,961 | 0,952 | 0,907 | 0,997 |
| OAS1, IFIT1, ISG15, HERC6, SLC1A2, OLAH, MMP8 | 0,853 | 0,843 | 0,864 | 0,903 | 0,845 | 0,960 | 0,945 | 0,893 | 0,996 |
| RSAD2, OAS1, SIGLEC1, ISG15, OLAH, RETN, MMP8 | 0,853 | 0,843 | 0,864 | 0,892 | 0,822 | 0,962 | 0,960 | 0,919 | 1 |
| RSAD2, OAS1, IFI44L, ISG15, OLAH, FAM20A, RETN | 0,853 | 0,843 | 0,864 | 0,892 | 0,821 | 0,964 | 0,946 | 0,887 | 1 |
| OAS1, IFIT1, IFI44L, ISG15, SLC1A2, OLAH, RETN | 0,853 | 0,843 | 0,863 | 0,909 | 0,851 | 0,967 | 0,936 | 0,881 | 0,991 |
| RSAD2, IFI27, OAS1, IFI44L, SIGLEC1, ISG15, MMP8 | 0,853 | 0,843 | 0,863 | 0,890 | 0,824 | 0,957 | 0,920 | 0,848 | 0,991 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, ISG15, HERC6, OLAH, RETN | 0,853 | 0,843 | 0,863 | 0,897 | 0,832 | 0,961 | 0,952 | 0,906 | 0,998 |
| RSAD2, OAS1, IFIT1, SLC1A2, IL1R2, OLAH, RETN | 0,853 | 0,843 | 0,863 | 0,907 | 0,850 | 0,965 | 0,947 | 0,896 | 0,997 |
| RSAD2, OAS1, IFI44L, HERC6, FAM20A, RETN, MMP8 | 0,853 | 0,843 | 0,864 | 0,896 | 0,828 | 0,963 | 0,930 | 0,852 | 1 |
| RSAD2, OAS1, IFIT1, ISG15, SLC1A2, IL1R2, OLAH | 0,853 | 0,843 | 0,863 | 0,905 | 0,847 | 0,963 | 0,947 | 0,897 | 0,997 |
| RSAD2, IFIT1, HERC6, IL1R2, OLAH, RETN, MMP8 | 0,853 | 0,843 | 0,863 | 0,898 | 0,834 | 0,963 | 0,961 | 0,922 | 1,000 |
| OAS1, IFIT1, IFI44L, HERC6, SLC1A2, IL1R2, RETN | 0,853 | 0,843 | 0,863 | 0,902 | 0,843 | 0,961 | 0,942 | 0,886 | 0,999 |
| IFI44L, ISG15, HERC6, SLC1A2, FAM20A, RETN, MMP8 | 0,853 | 0,843 | 0,864 | 0,898 | 0,832 | 0,964 | 0,945 | 0,867 | 1 |
| RSAD2, OAS1, IFIT1, HERC6, IL1R2, OLAH, RETN | 0,853 | 0,843 | 0,863 | 0,898 | 0,834 | 0,962 | 0,947 | 0,897 | 0,996 |
| OAS1, IFIT1, IFI44L, ISG15, SLC1A2, IL1R2, OLAH | 0,853 | 0,843 | 0,863 | 0,910 | 0,854 | 0,966 | 0,945 | 0,893 | 0,996 |
| IFIT1, IFI44L, HERC6, SLC1A2, IL1R2, RETN, MMP8 | 0,853 | 0,844 | 0,863 | 0,903 | 0,845 | 0,962 | 0,950 | 0,900 | 1,000 |
| OAS1, IFIT1, IFI44L, ISG15, HERC6, SLC1A2, IL1R2 | 0,853 | 0,843 | 0,863 | 0,903 | 0,847 | 0,959 | 0,950 | 0,900 | 1 |
| OAS1, IFI44L, ISG15, HERC6, OLAH, RETN, MMP8 | 0,853 | 0,843 | 0,864 | 0,895 | 0,828 | 0,962 | 0,964 | 0,927 | 1 |
| OAS1, IFIT1, IFI44L, HERC6, IL1R2, OLAH, MMP8 | 0,853 | 0,843 | 0,863 | 0,899 | 0,838 | 0,959 | 0,954 | 0,911 | 0,998 |
| RSAD2, IFI44L, ISG15, IL1R2, FAM20A, RETN, MMP8 | 0,853 | 0,843 | 0,864 | 0,901 | 0,839 | 0,964 | 0,939 | 0,861 | 1 |
| RSAD2, IFI44L, HERC6, SLC1A2, FAM20A, RETN, MMP8 | 0,853 | 0,843 | 0,864 | 0,899 | 0,835 | 0,963 | 0,933 | 0,854 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, ISG15, HERC6, SLC1A2, IL1R2, RETN | 0,853 | 0,844 | 0,863 | 0,905 | 0,848 | 0,962 | 0,943 | 0,887 | 1,000 |
| IFIT1, IFI44L, ISG15, HERC6, IL1R2, OLAH, MMP8 | 0,853 | 0,843 | 0,863 | 0,900 | 0,841 | 0,959 | 0,959 | 0,918 | 1,000 |
| RSAD2, OAS1, IFI44L, HERC6, IL1R2, RETN, MMP8 | 0,853 | 0,843 | 0,863 | 0,895 | 0,828 | 0,961 | 0,947 | 0,895 | 0,998 |
| RSAD2, OAS1, IFIT1, IFI44L, ISG15, HERC6, FAM20A | 0,853 | 0,843 | 0,864 | 0,893 | 0,828 | 0,958 | 0,916 | 0,833 | 1,000 |
| RSAD2, IFIT1, IFI44L, HERC6, FAM20A, RETN, MMP8 | 0,853 | 0,843 | 0,863 | 0,899 | 0,832 | 0,966 | 0,933 | 0,855 | 1 |
| RSAD2, OAS1, IFIT1, ISG15, HERC6, SLC1A2, OLAH | 0,853 | 0,843 | 0,863 | 0,903 | 0,846 | 0,961 | 0,946 | 0,894 | 0,997 |
| RSAD2, IFIT1, IFI44L, IL1R2, OLAH, FAM20A, RETN | 0,853 | 0,843 | 0,863 | 0,899 | 0,833 | 0,964 | 0,950 | 0,887 | 1 |
| RSAD2, OAS1, ISG15, HERC6, SLC1A2, OLAH, MMP8 | 0,853 | 0,843 | 0,863 | 0,903 | 0,845 | 0,960 | 0,940 | 0,885 | 0,996 |
| RSAD2, OAS1, IFI44L, HERC6, IL1R2, OLAH, MMP8 | 0,853 | 0,843 | 0,863 | 0,900 | 0,841 | 0,959 | 0,951 | 0,904 | 0,999 |
| RSAD2, IFI27, OAS1, IFIT1, SIGLEC1, ISG15, MMP8 | 0,853 | 0,843 | 0,863 | 0,888 | 0,820 | 0,955 | 0,917 | 0,843 | 0,992 |
| OAS1, IFIT1, IFI44L, OLAH, FAM20A, RETN, MMP8 | 0,853 | 0,843 | 0,863 | 0,896 | 0,826 | 0,966 | 0,955 | 0,904 | 1 |
| RSAD2, IFIT1, ISG15, HERC6, OLAH, RETN, MMP8 | 0,853 | 0,843 | 0,863 | 0,895 | 0,828 | 0,962 | 0,957 | 0,915 | 0,998 |
| RSAD2, IFI44L, SIGLEC1, ISG15, HERC6, RETN, MMP8 | 0,853 | 0,843 | 0,863 | 0,896 | 0,827 | 0,965 | 0,946 | 0,897 | 0,995 |
| RSAD2, IFI44L, SLC1A2, IL1R2, OLAH, RETN, MMP8 | 0,853 | 0,843 | 0,863 | 0,906 | 0,849 | 0,964 | 0,949 | 0,901 | 0,997 |
| RSAD2, IFIT1, IFI44L, SIGLEC1, ISG15, OLAH, RETN | 0,853 | 0,843 | 0,862 | 0,895 | 0,826 | 0,964 | 0,948 | 0,896 | 1,000 |
| OAS1, IFIT1, ISG15, HERC6, IL1R2, OLAH, MMP8 | 0,853 | 0,843 | 0,863 | 0,900 | 0,842 | 0,959 | 0,960 | 0,920 | 1,000 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SLC1A2, OLAH, RETN, MMP8 | 0,853 | 0,843 | 0,863 | 0,905 | 0,845 | 0,966 | 0,947 | 0,898 | 0,995 |
| OAS1, IFIT1, IFI44L, ISG15, OLAH, FAM20A, MMP8 | 0,853 | 0,842 | 0,863 | 0,892 | 0,821 | 0,963 | 0,951 | 0,895 | 1 |
| RSAD2, IFI27, IFIT1, IFI44L, SIGLEC1, ISG15, MMP8 | 0,853 | 0,843 | 0,862 | 0,890 | 0,822 | 0,957 | 0,913 | 0,838 | 0,988 |
| RSAD2, IFIT1, IFI44L, ISG15, SLC1A2, OLAH, MMP8 | 0,853 | 0,843 | 0,862 | 0,905 | 0,847 | 0,963 | 0,938 | 0,884 | 0,992 |
| OAS1, IFI44L, SIGLEC1, ISG15, OLAH, RETN, MMP8 | 0,853 | 0,842 | 0,863 | 0,893 | 0,823 | 0,963 | 0,966 | 0,929 | 1 |
| RSAD2, IFIT1, IFI44L, ISG15, HERC6, OLAH, RETN | 0,853 | 0,843 | 0,862 | 0,896 | 0,831 | 0,961 | 0,943 | 0,893 | 0,994 |
| RSAD2, IFIT1, IFI44L, ISG15, HERC6, IL1R2, MMP8 | 0,853 | 0,843 | 0,862 | 0,897 | 0,837 | 0,957 | 0,946 | 0,896 | 0,996 |
| RSAD2, IFI44L, HERC6, IL1R2, OLAH, RETN, MMP8 | 0,853 | 0,842 | 0,863 | 0,900 | 0,836 | 0,963 | 0,959 | 0,918 | 0,999 |
| RSAD2, IFI44L, ISG15, SLC1A2, IL1R2, OLAH, RETN | 0,853 | 0,843 | 0,862 | 0,908 | 0,851 | 0,964 | 0,945 | 0,893 | 0,996 |
| RSAD2, IFI27, IFIT1, ISG15, HERC6, SLC1A2, MMP8 | 0,853 | 0,843 | 0,862 | 0,900 | 0,844 | 0,957 | 0,902 | 0,821 | 0,983 |
| OAS1, IFIT1, IFI44L, SLC1A2, IL1R2, OLAH, RETN | 0,853 | 0,842 | 0,863 | 0,910 | 0,854 | 0,966 | 0,942 | 0,891 | 0,994 |
| RSAD2, IFIT1, SLC1A2, IL1R2, OLAH, RETN, MMP8 | 0,853 | 0,843 | 0,862 | 0,907 | 0,849 | 0,964 | 0,957 | 0,914 | 0,999 |
| OAS1, IFIT1, IFI44L, ISG15, HERC6, IL1R2, MMP8 | 0,853 | 0,843 | 0,862 | 0,898 | 0,839 | 0,957 | 0,948 | 0,899 | 0,997 |
| RSAD2, IFIT1, IFI44L, HERC6, SLC1A2, IL1R2, MMP8 | 0,852 | 0,843 | 0,862 | 0,903 | 0,846 | 0,960 | 0,951 | 0,902 | 1,000 |
| RSAD2, IFIT1, IFI44L, ISG15, HERC6, FAM20A, MMP8 | 0,852 | 0,842 | 0,863 | 0,894 | 0,830 | 0,957 | 0,911 | 0,826 | 0,995 |

(suite)

| Modèle | AUC modèle | IC 95% | | AUC Train | IC 95% Train | | AUC Test | IC 95% Test | |
|---|---|---|---|---|---|---|---|---|---|
| IFIT1, ISG15, HERC6, SLC1A2, IL1R2, RETN, MMP8 | 0,852 | 0,843 | 0,862 | 0,903 | 0,845 | 0,962 | 0,952 | 0,903 | 1 |
| RSAD2, OAS1, IFIT1, IFI44L, HERC6, IL1R2, OLAH | 0,852 | 0,843 | 0,862 | 0,899 | 0,839 | 0,960 | 0,947 | 0,898 | 0,995 |
| RSAD2, OAS1, IFIT1, IFI44L, HERC6, SLC1A2, IL1R2 | 0,852 | 0,843 | 0,862 | 0,903 | 0,847 | 0,959 | 0,951 | 0,901 | 1 |
| RSAD2, IFIT1, ISG15, HERC6, IL1R2, OLAH, MMP8 | 0,852 | 0,842 | 0,862 | 0,900 | 0,842 | 0,959 | 0,959 | 0,918 | 1,000 |
| RSAD2, OAS1, IFIT1, IFI44L, ISG15, HERC6, IL1R2 | 0,852 | 0,843 | 0,862 | 0,897 | 0,838 | 0,956 | 0,947 | 0,897 | 0,997 |
| RSAD2, OAS1, IFIT1, HERC6, OLAH, RETN, MMP8 | 0,852 | 0,842 | 0,863 | 0,890 | 0,820 | 0,960 | 0,955 | 0,913 | 0,998 |
| RSAD2, IFIT1, HERC6, SLC1A2, IL1R2, RETN, MMP8 | 0,852 | 0,843 | 0,862 | 0,903 | 0,844 | 0,962 | 0,952 | 0,904 | 1,000 |
| OAS1, IFIT1, SIGLEC1, ISG15, HERC6, SLC1A2, MMP8 | 0,852 | 0,843 | 0,862 | 0,896 | 0,837 | 0,955 | 0,924 | 0,861 | 0,987 |
| RSAD2, OAS1, IFI44L, SIGLEC1, HERC6, RETN, MMP8 | 0,852 | 0,842 | 0,862 | 0,890 | 0,819 | 0,962 | 0,946 | 0,896 | 0,995 |
| OAS1, IFI44L, SLC1A2, IL1R2, OLAH, RETN, MMP8 | 0,852 | 0,842 | 0,862 | 0,907 | 0,849 | 0,964 | 0,945 | 0,894 | 0,995 |
| IFIT1, IFI44L, ISG15, HERC6, SLC1A2, IL1R2, MMP8 | 0,852 | 0,843 | 0,862 | 0,901 | 0,844 | 0,958 | 0,952 | 0,904 | 1 |
| RSAD2, OAS1, IFI44L, SLC1A2, IL1R2, OLAH, RETN | 0,852 | 0,842 | 0,863 | 0,907 | 0,850 | 0,963 | 0,939 | 0,884 | 0,994 |
| RSAD2, IFI44L, SIGLEC1, HERC6, SLC1A2, RETN, MMP8 | 0,852 | 0,843 | 0,862 | 0,903 | 0,840 | 0,965 | 0,939 | 0,885 | 0,993 |
| RSAD2, OAS1, IFI44L, SLC1A2, IL1R2, OLAH, MMP8 | 0,852 | 0,842 | 0,862 | 0,905 | 0,849 | 0,961 | 0,941 | 0,888 | 0,995 |
| RSAD2, OAS1, IFIT1, IFI44L, OLAH, FAM20A, RETN | 0,852 | 0,842 | 0,862 | 0,896 | 0,826 | 0,965 | 0,943 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFI44L, HERC6, SLC1A2, FAM20A, RETN, MMP8 | 0,852 | 0,842 | 0,863 | 0,901 | 0,837 | 0,965 | 0,936 | 0,857 | 1 |
| IFI44L, ISG15, HERC6, SLC1A2, IL1R2, RETN, MMP8 | 0,852 | 0,843 | 0,862 | 0,905 | 0,849 | 0,962 | 0,946 | 0,890 | 1 |
| OAS1, IFIT1, IFI44L, HERC6, IL1R2, OLAH, RETN | 0,852 | 0,842 | 0,862 | 0,898 | 0,834 | 0,963 | 0,947 | 0,897 | 0,996 |
| RSAD2, IFI44L, ISG15, SLC1A2, IL1R2, OLAH, MMP8 | 0,852 | 0,843 | 0,862 | 0,904 | 0,847 | 0,961 | 0,945 | 0,892 | 0,998 |
| RSAD2, OAS1, IFIT1, IFI44L, HERC6, OLAH, RETN | 0,852 | 0,842 | 0,862 | 0,892 | 0,824 | 0,961 | 0,938 | 0,884 | 0,992 |
| RSAD2, IFIT1, IFI44L, ISG15, SLC1A2, IL1R2, OLAH | 0,852 | 0,843 | 0,862 | 0,909 | 0,852 | 0,965 | 0,945 | 0,893 | 0,996 |
| OAS1, IFI44L, ISG15, SLC1A2, OLAH, RETN, MMP8 | 0,852 | 0,842 | 0,862 | 0,906 | 0,845 | 0,966 | 0,943 | 0,893 | 0,994 |
| IFIT1, IFI44L, ISG15, HERC6, SLC1A2, FAM20A, MMP8 | 0,852 | 0,842 | 0,862 | 0,899 | 0,840 | 0,958 | 0,913 | 0,823 | 1 |
| OAS1, IFIT1, IFI44L, SIGLEC1, HERC6, SLC1A2, MMP8 | 0,852 | 0,843 | 0,861 | 0,893 | 0,833 | 0,953 | 0,912 | 0,843 | 0,981 |
| RSAD2, OAS1, IFI44L, HERC6, SLC1A2, IL1R2, RETN | 0,852 | 0,842 | 0,862 | 0,901 | 0,843 | 0,960 | 0,941 | 0,880 | 1 |
| RSAD2, OAS1, IFIT1, SIGLEC1, HERC6, SLC1A2, MMP8 | 0,852 | 0,843 | 0,861 | 0,895 | 0,835 | 0,955 | 0,918 | 0,852 | 0,985 |
| RSAD2, OAS1, ISG15, SLC1A2, IL1R2, OLAH, RETN | 0,852 | 0,842 | 0,862 | 0,906 | 0,849 | 0,963 | 0,940 | 0,884 | 0,997 |
| OAS1, IFI44L, ISG15, SLC1A2, IL1R2, OLAH, MMP8 | 0,852 | 0,842 | 0,862 | 0,905 | 0,849 | 0,962 | 0,941 | 0,888 | 0,995 |
| OAS1, IFI44L, ISG15, SLC1A2, IL1R2, OLAH, RETN | 0,852 | 0,842 | 0,862 | 0,909 | 0,853 | 0,966 | 0,942 | 0,890 | 0,995 |
| RSAD2, OAS1, IFIT1, IFI44L, SLC1A2, IL1R2, OLAH | 0,852 | 0,842 | 0,862 | 0,909 | 0,852 | 0,965 | 0,946 | 0,895 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SLC1A2, OLAH, RETN | 0,852 | 0,842 | 0,862 | 0,909 | 0,851 | 0,967 | 0,937 | 0,883 | 0,991 |
| RSAD2, OAS1, IFI44L, HERC6, OLAH, RETN, MMP8 | 0,852 | 0,842 | 0,862 | 0,893 | 0,825 | 0,962 | 0,948 | 0,900 | 0,995 |
| RSAD2, OAS1, ISG15, SLC1A2, IL1R2, OLAH, MMP8 | 0,852 | 0,843 | 0,861 | 0,903 | 0,846 | 0,961 | 0,939 | 0,882 | 0,996 |
| RSAD2, OAS1, IFI44L, ISG15, HERC6, IL1R2, OLAH | 0,852 | 0,842 | 0,862 | 0,900 | 0,841 | 0,959 | 0,947 | 0,898 | 0,995 |
| RSAD2, IFIT1, IFI44L, SIGLEC1, ISG15, OLAH, MMP8 | 0,852 | 0,842 | 0,862 | 0,894 | 0,827 | 0,961 | 0,951 | 0,903 | 0,999 |
| RSAD2, IFIT1, IFI44L, SLC1A2, IL1R2, OLAH, RETN | 0,852 | 0,842 | 0,862 | 0,908 | 0,852 | 0,965 | 0,945 | 0,894 | 0,995 |
| RSAD2, IFIT1, IFI44L, SLC1A2, OLAH, RETN, MMP8 | 0,852 | 0,842 | 0,862 | 0,905 | 0,844 | 0,966 | 0,947 | 0,898 | 0,995 |
| RSAD2, IFIT1, ISG15, HERC6, SLC1A2, OLAH, MMP8 | 0,852 | 0,842 | 0,862 | 0,902 | 0,844 | 0,960 | 0,943 | 0,891 | 0,996 |
| RSAD2, IFI44L, ISG15, SLC1A2, OLAH, RETN, MMP8 | 0,852 | 0,842 | 0,862 | 0,907 | 0,847 | 0,967 | 0,945 | 0,895 | 0,995 |
| OAS1, IFI44L, HERC6, SLC1A2, IL1R2, RETN, MMP8 | 0,852 | 0,842 | 0,862 | 0,903 | 0,845 | 0,961 | 0,943 | 0,885 | 1 |
| RSAD2, OAS1, SLC1A2, IL1R2, OLAH, RETN, MMP8 | 0,852 | 0,842 | 0,862 | 0,906 | 0,849 | 0,964 | 0,942 | 0,888 | 0,996 |
| RSAD2, IFI44L, ISG15, HERC6, OLAH, RETN, MMP8 | 0,852 | 0,841 | 0,862 | 0,899 | 0,833 | 0,965 | 0,954 | 0,911 | 0,998 |
| OAS1, IFIT1, ISG15, OLAH, FAM20A, RETN, MMP8 | 0,852 | 0,841 | 0,862 | 0,891 | 0,820 | 0,962 | 0,959 | 0,905 | 1 |
| RSAD2, IFIT1, ISG15, SLC1A2, IL1R2, OLAH, RETN | 0,852 | 0,842 | 0,861 | 0,907 | 0,849 | 0,965 | 0,946 | 0,895 | 0,996 |
| RSAD2, ISG15, HERC6, SLC1A2, IL1R2, RETN, MMP8 | 0,852 | 0,842 | 0,861 | 0,901 | 0,842 | 0,959 | 0,941 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, ISG15, SLC1A2, OLAH, RETN | 0,852 | 0,842 | 0,861 | 0,909 | 0,851 | 0,967 | 0,934 | 0,877 | 0,990 |
| RSAD2, IFI27, OAS1, IFIT1, ISG15, SLC1A2, FAM20A | 0,852 | 0,841 | 0,862 | 0,892 | 0,824 | 0,960 | 0,938 | 0,858 | 1 |
| OAS1, IFIT1, IFI44L, ISG15, OLAH, FAM20A, RETN | 0,852 | 0,841 | 0,862 | 0,889 | 0,817 | 0,961 | 0,952 | 0,898 | 1 |
| RSAD2, OAS1, IFI44L, ISG15, SLC1A2, IL1R2, OLAH | 0,852 | 0,841 | 0,862 | 0,906 | 0,850 | 0,963 | 0,940 | 0,885 | 0,995 |
| RSAD2, OAS1, IFI44L, ISG15, HERC6, FAM20A, MMP8 | 0,852 | 0,841 | 0,862 | 0,891 | 0,826 | 0,957 | 0,925 | 0,845 | 1 |
| RSAD2, OAS1, IFI44L, ISG15, SLC1A2, OLAH, MMP8 | 0,852 | 0,842 | 0,862 | 0,905 | 0,847 | 0,963 | 0,933 | 0,875 | 0,990 |
| RSAD2, OAS1, IFI44L, ISG15, HERC6, SLC1A2, IL1R2 | 0,852 | 0,842 | 0,861 | 0,900 | 0,845 | 0,955 | 0,945 | 0,885 | 1 |
| RSAD2, IFI27, IFI44L, SIGLEC1, ISG15, HERC6, SLC1A2 | 0,851 | 0,843 | 0,860 | 0,898 | 0,842 | 0,954 | 0,940 | 0,885 | 0,995 |
| RSAD2, OAS1, HERC6, SLC1A2, IL1R2, RETN, MMP8 | 0,851 | 0,842 | 0,861 | 0,902 | 0,844 | 0,959 | 0,939 | 0,872 | 1 |
| OAS1, IFI44L, ISG15, HERC6, IL1R2, OLAH, MMP8 | 0,851 | 0,842 | 0,861 | 0,899 | 0,841 | 0,958 | 0,954 | 0,910 | 0,998 |
| RSAD2, IFIT1, IFI44L, HERC6, IL1R2, OLAH, MMP8 | 0,851 | 0,842 | 0,861 | 0,899 | 0,839 | 0,959 | 0,955 | 0,912 | 0,999 |
| RSAD2, OAS1, ISG15, HERC6, SLC1A2, IL1R2, MMP8 | 0,851 | 0,842 | 0,861 | 0,899 | 0,842 | 0,955 | 0,936 | 0,869 | 1 |
| RSAD2, OAS1, IFI44L, ISG15, SLC1A2, OLAH, RETN | 0,851 | 0,841 | 0,862 | 0,909 | 0,851 | 0,967 | 0,935 | 0,879 | 0,991 |
| RSAD2, OAS1, IFI44L, SLC1A2, OLAH, RETN, MMP8 | 0,851 | 0,841 | 0,861 | 0,906 | 0,846 | 0,966 | 0,943 | 0,893 | 0,994 |
| OAS1, IFI44L, ISG15, HERC6, SLC1A2, IL1R2, MMP8 | 0,851 | 0,841 | 0,861 | 0,900 | 0,845 | 0,956 | 0,939 | 0,879 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, ISG15, HERC6, SLC1A2 | 0,851 | 0,842 | 0,860 | 0,901 | 0,846 | 0,956 | 0,941 | 0,888 | 0,995 |
| RSAD2, IFIT1, IFI44L, HERC6, SLC1A2, IL1R2, RETN | 0,851 | 0,842 | 0,861 | 0,901 | 0,842 | 0,960 | 0,939 | 0,881 | 0,998 |
| RSAD2, ISG15, SLC1A2, IL1R2, OLAH, RETN, MMP8 | 0,851 | 0,842 | 0,861 | 0,905 | 0,848 | 0,963 | 0,943 | 0,888 | 0,999 |
| RSAD2, OAS1, IFI44L, ISG15, HERC6, IL1R2, MMP8 | 0,851 | 0,841 | 0,861 | 0,895 | 0,837 | 0,954 | 0,946 | 0,895 | 0,996 |
| RSAD2, OAS1, IFI44L, ISG15, HERC6, SLC1A2, RETN | 0,851 | 0,841 | 0,862 | 0,898 | 0,835 | 0,961 | 0,933 | 0,870 | 0,995 |
| RSAD2, OAS1, IFIT1, IFI44L, ISG15, HERC6, RETN | 0,851 | 0,841 | 0,861 | 0,892 | 0,825 | 0,959 | 0,926 | 0,864 | 0,988 |
| RSAD2, OAS1, IFIT1, IFI44L, ISG15, SLC1A2, OLAH | 0,851 | 0,841 | 0,861 | 0,909 | 0,852 | 0,966 | 0,935 | 0,880 | 0,990 |
| OAS1, IFIT1, IFI44L, ISG15, HERC6, IL1R2, OLAH | 0,851 | 0,841 | 0,861 | 0,899 | 0,840 | 0,959 | 0,952 | 0,907 | 0,997 |
| RSAD2, IFIT1, IFI44L, ISG15, HERC6, IL1R2, OLAH | 0,851 | 0,841 | 0,861 | 0,900 | 0,840 | 0,960 | 0,948 | 0,900 | 0,996 |
| RSAD2, IFIT1, IFI44L, HERC6, OLAH, RETN, MMP8 | 0,851 | 0,841 | 0,861 | 0,894 | 0,825 | 0,962 | 0,952 | 0,907 | 0,997 |
| OAS1, IFI44L, ISG15, OLAH, FAM20A, RETN, MMP8 | 0,851 | 0,840 | 0,861 | 0,895 | 0,825 | 0,966 | 0,957 | 0,905 | 1 |
| RSAD2, IFI44L, ISG15, HERC6, IL1R2, OLAH, MMP8 | 0,851 | 0,841 | 0,861 | 0,901 | 0,843 | 0,959 | 0,950 | 0,903 | 0,997 |
| RSAD2, IFI27, OAS1, IFI44L, SIGLEC1, ISG15, SLC1A2 | 0,851 | 0,842 | 0,860 | 0,896 | 0,837 | 0,956 | 0,952 | 0,902 | 1 |
| OAS1, IFIT1, ISG15, SLC1A2, OLAH, RETN, MMP8 | 0,851 | 0,840 | 0,861 | 0,900 | 0,839 | 0,961 | 0,954 | 0,911 | 0,998 |
| IFI27, OAS1, IFIT1, IFI44L, SIGLEC1, ISG15, SLC1A2 | 0,851 | 0,841 | 0,860 | 0,896 | 0,836 | 0,955 | 0,954 | 0,905 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, OLAH, RETN | 0,851 | 0,840 | 0,861 | 0,891 | 0,821 | 0,962 | 0,950 | 0,901 | 0,999 |
| RSAD2, OAS1, IFIT1, ISG15, SLC1A2, OLAH, MMP8 | 0,851 | 0,841 | 0,861 | 0,902 | 0,844 | 0,961 | 0,942 | 0,891 | 0,994 |
| IFIT1, IFI44L, ISG15, OLAH, FAM20A, RETN, MMP8 | 0,851 | 0,840 | 0,861 | 0,896 | 0,828 | 0,964 | 0,962 | 0,920 | 1 |
| RSAD2, IFI44L, ISG15, HERC6, SLC1A2, IL1R2, MMP8 | 0,851 | 0,841 | 0,860 | 0,901 | 0,846 | 0,957 | 0,940 | 0,882 | 0,999 |
| RSAD2, OAS1, IFIT1, HERC6, SLC1A2, FAM20A, RETN | 0,850 | 0,840 | 0,861 | 0,896 | 0,831 | 0,961 | 0,935 | 0,854 | 1 |
| RSAD2, IFIT1, IFI44L, HERC6, IL1R2, OLAH, RETN | 0,850 | 0,841 | 0,860 | 0,898 | 0,834 | 0,963 | 0,947 | 0,898 | 0,996 |
| RSAD2, OAS1, IFIT1, ISG15, SLC1A2, OLAH, RETN | 0,850 | 0,840 | 0,860 | 0,906 | 0,847 | 0,964 | 0,945 | 0,894 | 0,995 |
| RSAD2, OAS1, IFIT1, IFI44L, IL1R2, OLAH, MMP8 | 0,850 | 0,840 | 0,860 | 0,895 | 0,832 | 0,958 | 0,955 | 0,913 | 0,998 |
| OAS1, IFIT1, IFI44L, ISG15, HERC6, SLC1A2, RETN | 0,850 | 0,840 | 0,860 | 0,898 | 0,836 | 0,960 | 0,921 | 0,851 | 0,990 |
| RSAD2, IFI27, IFIT1, IFI44L, SIGLEC1, HERC6, SLC1A2 | 0,850 | 0,841 | 0,859 | 0,899 | 0,844 | 0,955 | 0,940 | 0,885 | 0,995 |
| RSAD2, IFIT1, IFI44L, SIGLEC1, HERC6, SLC1A2, MMP8 | 0,850 | 0,841 | 0,859 | 0,896 | 0,837 | 0,956 | 0,908 | 0,836 | 0,979 |
| RSAD2, OAS1, IFI44L, SIGLEC1, ISG15, OLAH, MMP8 | 0,850 | 0,840 | 0,860 | 0,893 | 0,825 | 0,960 | 0,949 | 0,901 | 0,997 |

Performances obtenues pour l'identification de la nature virale ou bactérienne de l'infection sur la base des combinaisons à 8 gènes :

[Tableau 12]

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, IL1R2, OLAH, FAM20A, MMP8 | 0,899 | 0,891 | 0,907 | 0,935 | 0,885 | 0,985 | 0,952 | 0,892 | 1 |
| IFI27, IFIT1, SIGLEC1, HERC6, IL1R2, OLAH, FAM20A, MMP8 | 0,899 | 0,891 | 0,908 | 0,935 | 0,885 | 0,986 | 0,954 | 0,893 | 1 |
| RSAD2, IFI27, SIGLEC1, HERC6, IL1R2, OLAH, FAM20A, MMP8 | 0,899 | 0,890 | 0,908 | 0,935 | 0,885 | 0,986 | 0,951 | 0,891 | 1 |

| | | | | | | | | |
|---|---|---|---|---|---|---|---|---|
| IFI27, OAS1, IFIT1, SIGLEC1, HERC6, OLAH, FAM20A, MMP8 | 0,899 | 0,890 | 0,908 | 0,933 | 0,880 | 0,986 | 0,945 | 0,881 | 1 |
| IFI27, OAS1, SIGLEC1, HERC6, IL1R2, OLAH, FAM20A, MMP8 | 0,899 | 0,890 | 0,908 | 0,936 | 0,885 | 0,986 | 0,950 | 0,890 | 1 |
| IFI27, IFIT1, SIGLEC1, ISG15, IL1R2, OLAH, FAM20A, MMP8 | 0,899 | 0,890 | 0,907 | 0,937 | 0,886 | 0,987 | 0,952 | 0,889 | 1 |
| IFI27, OAS1, IFIT1, SIGLEC1, IL1R2, OLAH, FAM20A, MMP8 | 0,899 | 0,890 | 0,907 | 0,935 | 0,885 | 0,986 | 0,955 | 0,899 | 1 |
| IFI27, IFIT1, IFI44L, SIGLEC1, IL1R2, OLAH, FAM20A, MMP8 | 0,898 | 0,890 | 0,906 | 0,936 | 0,886 | 0,986 | 0,955 | 0,895 | 1 |
| IFI27, OAS1, IFIT1, SIGLEC1, HERC6, IL1R2, FAM20A, MMP8 | 0,898 | 0,890 | 0,907 | 0,933 | 0,883 | 0,983 | 0,951 | 0,885 | 1 |
| IFI27, OAS1, IFIT1, IFI44L, SIGLEC1, HERC6, OLAH, FAM20A | 0,898 | 0,889 | 0,907 | 0,932 | 0,879 | 0,985 | 0,940 | 0,876 | 1 |
| IFI27, SIGLEC1, ISG15, HERC6, IL1R2, OLAH, FAM20A, MMP8 | 0,898 | 0,889 | 0,907 | 0,933 | 0,881 | 0,986 | 0,950 | 0,886 | 1 |
| IFI27, IFIT1, SIGLEC1, ISG15, HERC6, OLAH, FAM20A, MMP8 | 0,898 | 0,888 | 0,907 | 0,931 | 0,877 | 0,986 | 0,951 | 0,892 | 1 |
| IFI27, IFI44L, SIGLEC1, HERC6, IL1R2, OLAH, FAM20A, MMP8 | 0,898 | 0,889 | 0,906 | 0,936 | 0,886 | 0,985 | 0,953 | 0,892 | 1 |
| RSAD2, IFI27, OAS1, SIGLEC1, HERC6, OLAH, FAM20A, MMP8 | 0,898 | 0,888 | 0,907 | 0,934 | 0,881 | 0,986 | 0,947 | 0,887 | 1 |
| IFI27, IFIT1, IFI44L, SIGLEC1, HERC6, IL1R2, FAM20A, MMP8 | 0,897 | 0,889 | 0,906 | 0,933 | 0,882 | 0,984 | 0,952 | 0,885 | 1 |
| IFI27, IFIT1, IFI44L, SIGLEC1, HERC6, OLAH, FAM20A, MMP8 | 0,897 | 0,889 | 0,906 | 0,931 | 0,877 | 0,986 | 0,955 | 0,896 | 1 |
| RSAD2, IFI27, IFIT1, SIGLEC1, HERC6, OLAH, FAM20A, MMP8 | 0,897 | 0,888 | 0,906 | 0,934 | 0,882 | 0,986 | 0,948 | 0,887 | 1 |
| RSAD2, IFI27, IFIT1, SIGLEC1, IL1R2, OLAH, FAM20A, MMP8 | 0,897 | 0,889 | 0,906 | 0,935 | 0,884 | 0,986 | 0,954 | 0,897 | 1 |
| RSAD2, IFI27, OAS1, SIGLEC1, IL1R2, OLAH, FAM20A, MMP8 | 0,897 | 0,888 | 0,906 | 0,935 | 0,884 | 0,986 | 0,952 | 0,892 | 1 |
| RSAD2, IFI27, IFIT1, SIGLEC1, HERC6, IL1R2, FAM20A, MMP8 | 0,897 | 0,889 | 0,905 | 0,933 | 0,883 | 0,983 | 0,951 | 0,884 | 1 |
| IFI27, IFIT1, IFI44L, SIGLEC1, HERC6, IL1R2, OLAH, FAM20A | 0,897 | 0,888 | 0,905 | 0,932 | 0,879 | 0,985 | 0,945 | 0,881 | 1 |
| IFI27, IFIT1, IFI44L, SIGLEC1, ISG15, HERC6, OLAH, FAM20A | 0,897 | 0,888 | 0,906 | 0,932 | 0,878 | 0,986 | 0,941 | 0,878 | 1 |
| IFI27, OAS1, SIGLEC1, ISG15, IL1R2, OLAH, FAM20A, MMP8 | 0,897 | 0,888 | 0,906 | 0,936 | 0,884 | 0,987 | 0,950 | 0,886 | 1 |
| IFI27, IFIT1, SIGLEC1, ISG15, HERC6, IL1R2, FAM20A, MMP8 | 0,897 | 0,888 | 0,905 | 0,932 | 0,880 | 0,983 | 0,953 | 0,885 | 1 |
| IFI27, OAS1, IFIT1, SIGLEC1, HERC6, IL1R2, OLAH, FAM20A | 0,896 | 0,888 | 0,905 | 0,932 | 0,880 | 0,984 | 0,941 | 0,878 | 1 |
| IFI27, OAS1, SIGLEC1, HERC6, OLAH, FAM20A, RETN, MMP8 | 0,896 | 0,886 | 0,906 | 0,933 | 0,877 | 0,988 | 0,948 | 0,886 | 1 |
| IFI27, OAS1, IFI44L, SIGLEC1, HERC6, IL1R2, FAM20A, MMP8 | 0,896 | 0,888 | 0,905 | 0,934 | 0,884 | 0,983 | 0,948 | 0,881 | 1 |
| IFI27, IFI44L, SIGLEC1, ISG15, HERC6, IL1R2, FAM20A, MMP8 | 0,896 | 0,888 | 0,904 | 0,933 | 0,882 | 0,983 | 0,950 | 0,882 | 1 |
| IFI27, OAS1, IFIT1, SIGLEC1, ISG15, HERC6, OLAH, FAM20A | 0,896 | 0,887 | 0,905 | 0,933 | 0,880 | 0,985 | 0,938 | 0,873 | 1 |
| IFI27, OAS1, SIGLEC1, HERC6, IL1R2, FAM20A, RETN, MMP8 | 0,896 | 0,887 | 0,905 | 0,934 | 0,881 | 0,987 | 0,950 | 0,882 | 1 |
| IFI27, OAS1, IFIT1, SIGLEC1, HERC6, OLAH, FAM20A, RETN | 0,896 | 0,887 | 0,905 | 0,933 | 0,880 | 0,985 | 0,939 | 0,874 | 1 |

| | | | | | | | | |
|---|---|---|---|---|---|---|---|---|
| RSAD2, IFI27, OAS1, IFIT1, SIGLEC1, HERC6, OLAH, FAM20A | 0,896 | 0,887 | 0,905 | 0,931 | 0,878 | 0,985 | 0,939 | 0,875 | 1 |
| IFI27, IFIT1, SIGLEC1, HERC6, IL1R2, FAM20A, RETN, MMP8 | 0,896 | 0,887 | 0,905 | 0,936 | 0,885 | 0,988 | 0,955 | 0,888 | 1 |
| RSAD2, IFI27, SIGLEC1, ISG15, IL1R2, OLAH, FAM20A, MMP8 | 0,896 | 0,887 | 0,905 | 0,936 | 0,885 | 0,987 | 0,952 | 0,892 | 1 |
| IFI27, OAS1, IFIT1, SIGLEC1, ISG15, OLAH, FAM20A, MMP8 | 0,896 | 0,887 | 0,905 | 0,928 | 0,870 | 0,986 | 0,953 | 0,898 | 1 |
| RSAD2, IFI27, SIGLEC1, HERC6, OLAH, FAM20A, RETN, MMP8 | 0,896 | 0,886 | 0,905 | 0,935 | 0,882 | 0,989 | 0,951 | 0,892 | 1 |
| IFI27, IFIT1, SIGLEC1, HERC6, OLAH, FAM20A, RETN, MMP8 | 0,896 | 0,886 | 0,905 | 0,933 | 0,878 | 0,988 | 0,957 | 0,898 | 1 |
| IFI27, SIGLEC1, ISG15, IL1R2, OLAH, FAM20A, RETN, MMP8 | 0,896 | 0,886 | 0,905 | 0,933 | 0,879 | 0,987 | 0,953 | 0,891 | 1 |
| RSAD2, IFI27, SIGLEC1, ISG15, HERC6, OLAH, FAM20A, MMP8 | 0,895 | 0,886 | 0,905 | 0,934 | 0,881 | 0,988 | 0,947 | 0,887 | 1 |
| IFI27, OAS1, SIGLEC1, ISG15, HERC6, OLAH, FAM20A, MMP8 | 0,895 | 0,886 | 0,905 | 0,929 | 0,873 | 0,986 | 0,946 | 0,884 | 1 |
| IFI27, IFIT1, IFI44L, SIGLEC1, HERC6, OLAH, FAM20A, RETN | 0,895 | 0,887 | 0,904 | 0,930 | 0,875 | 0,985 | 0,942 | 0,879 | 1 |
| RSAD2, IFI27, IFIT1, IFI44L, SIGLEC1, HERC6, OLAH, FAM20A | 0,895 | 0,887 | 0,904 | 0,932 | 0,879 | 0,985 | 0,940 | 0,877 | 1 |
| RSAD2, IFI27, IFI44L, SIGLEC1, HERC6, IL1R2, FAM20A, MMP8 | 0,895 | 0,887 | 0,904 | 0,933 | 0,883 | 0,983 | 0,950 | 0,880 | 1 |
| RSAD2, IFI27, OAS1, SIGLEC1, HERC6, IL1R2, FAM20A, MMP8 | 0,895 | 0,886 | 0,904 | 0,935 | 0,884 | 0,985 | 0,949 | 0,881 | 1 |
| IFI27, IFIT1, IFI44L, SIGLEC1, ISG15, OLAH, FAM20A, MMP8 | 0,895 | 0,886 | 0,904 | 0,930 | 0,874 | 0,986 | 0,951 | 0,894 | 1 |
| IFI27, IFIT1, SIGLEC1, ISG15, HERC6, IL1R2, OLAH, FAM20A | 0,895 | 0,887 | 0,904 | 0,933 | 0,881 | 0,986 | 0,941 | 0,877 | 1 |
| IFI27, OAS1, IFIT1, IFI44L, SIGLEC1, IL1R2, FAM20A, MMP8 | 0,895 | 0,887 | 0,903 | 0,931 | 0,880 | 0,982 | 0,957 | 0,896 | 1 |
| RSAD2, IFI27, OAS1, HERC6, IL1R2, OLAH, FAM20A, MMP8 | 0,895 | 0,886 | 0,904 | 0,933 | 0,884 | 0,983 | 0,927 | 0,850 | 1 |
| RSAD2, IFI27, SIGLEC1, HERC6, IL1R2, FAM20A, RETN, MMP8 | 0,895 | 0,886 | 0,904 | 0,935 | 0,883 | 0,987 | 0,949 | 0,880 | 1 |
| RSAD2, IFI27, IFIT1, SIGLEC1, HERC6, IL1R2, OLAH, FAM20A | 0,895 | 0,887 | 0,903 | 0,934 | 0,883 | 0,986 | 0,939 | 0,874 | 1 |
| IFI27, IFI44L, SIGLEC1, ISG15, HERC6, OLAH, FAM20A, MMP8 | 0,895 | 0,886 | 0,904 | 0,928 | 0,871 | 0,985 | 0,945 | 0,881 | 1 |
| IFI27, IFIT1, SIGLEC1, ISG15, HERC6, OLAH, FAM20A, RETN | 0,895 | 0,886 | 0,904 | 0,931 | 0,876 | 0,987 | 0,942 | 0,879 | 1 |
| IFI27, IFIT1, IFI44L, SIGLEC1, IL1R2, FAM20A, RETN, MMP8 | 0,895 | 0,886 | 0,903 | 0,934 | 0,882 | 0,986 | 0,953 | 0,888 | 1 |
| IFI27, IFIT1, IFI44L, SIGLEC1, ISG15, IL1R2, FAM20A, MMP8 | 0,895 | 0,887 | 0,903 | 0,932 | 0,881 | 0,983 | 0,952 | 0,885 | 1 |
| IFI27, OAS1, IFI44L, SIGLEC1, IL1R2, OLAH, FAM20A, MMP8 | 0,895 | 0,886 | 0,904 | 0,935 | 0,885 | 0,985 | 0,954 | 0,896 | 1 |
| IFI27, IFI44L, SIGLEC1, ISG15, HERC6, IL1R2, OLAH, FAM20A | 0,895 | 0,886 | 0,903 | 0,930 | 0,876 | 0,983 | 0,942 | 0,877 | 1 |
| IFI27, OAS1, IFIT1, IFI44L, SIGLEC1, HERC6, IL1R2, FAM20A | 0,895 | 0,887 | 0,903 | 0,934 | 0,884 | 0,984 | 0,946 | 0,879 | 1 |
| RSAD2, IFI27, OAS1, SIGLEC1, HERC6, IL1R2, OLAH, FAM20A | 0,895 | 0,886 | 0,904 | 0,931 | 0,878 | 0,984 | 0,940 | 0,874 | 1 |
| IFI27, IFIT1, SIGLEC1, IL1R2, OLAH, FAM20A, RETN, MMP8 | 0,895 | 0,886 | 0,903 | 0,934 | 0,881 | 0,988 | 0,958 | 0,899 | 1 |

| Genes | | | | | | | | |
|---|---|---|---|---|---|---|---|---|
| IFI27, OAS1, IFIT1, SIGLEC1, HERC6, FAM20A, RETN, MMP8 | 0,895 | 0,885 | 0,904 | 0,933 | 0,879 | 0,987 | 0,942 | 0,873 | 1 |
| IFI27, OAS1, IFIT1, IFI44L, SIGLEC1, OLAH, FAM20A, MMP8 | 0,894 | 0,885 | 0,904 | 0,927 | 0,869 | 0,985 | 0,957 | 0,902 | 1 |
| IFI27, OAS1, IFIT1, SIGLEC1, IL1R2, FAM20A, RETN, MMP8 | 0,894 | 0,886 | 0,903 | 0,934 | 0,882 | 0,986 | 0,954 | 0,891 | 1 |
| IFI27, IFIT1, IFI44L, SIGLEC1, ISG15, HERC6, FAM20A, RETN | 0,894 | 0,886 | 0,903 | 0,928 | 0,873 | 0,983 | 0,940 | 0,867 | 1 |
| RSAD2, IFI27, IFI44L, SIGLEC1, IL1R2, OLAH, FAM20A, MMP8 | 0,894 | 0,886 | 0,903 | 0,935 | 0,885 | 0,985 | 0,952 | 0,893 | 1 |
| RSAD2, IFI27, SIGLEC1, ISG15, HERC6, IL1R2, FAM20A, MMP8 | 0,894 | 0,885 | 0,903 | 0,934 | 0,883 | 0,984 | 0,952 | 0,885 | 1 |
| IFI27, OAS1, IFI44L, SIGLEC1, HERC6, OLAH, FAM20A, MMP8 | 0,894 | 0,885 | 0,903 | 0,933 | 0,880 | 0,985 | 0,949 | 0,886 | 1 |
| IFI27, IFIT1, IFI44L, SIGLEC1, ISG15, HERC6, IL1R2, FAM20A | 0,894 | 0,886 | 0,902 | 0,933 | 0,882 | 0,983 | 0,943 | 0,875 | 1 |
| IFI27, OAS1, IFIT1, HERC6, IL1R2, OLAH, FAM20A, MMP8 | 0,894 | 0,885 | 0,903 | 0,932 | 0,881 | 0,984 | 0,930 | 0,857 | 1 |
| RSAD2, IFI27, IFI44L, HERC6, IL1R2, OLAH, FAM20A, MMP8 | 0,894 | 0,885 | 0,903 | 0,932 | 0,881 | 0,983 | 0,930 | 0,857 | 1 |
| IFI27, IFI44L, SIGLEC1, ISG15, IL1R2, FAM20A, RETN, MMP8 | 0,894 | 0,885 | 0,903 | 0,934 | 0,883 | 0,986 | 0,951 | 0,882 | 1 |
| IFI27, OAS1, IFIT1, SIGLEC1, HERC6, IL1R2, FAM20A, RETN | 0,894 | 0,885 | 0,903 | 0,935 | 0,885 | 0,985 | 0,943 | 0,876 | 1 |
| RSAD2, IFI27, SIGLEC1, IL1R2, OLAH, FAM20A, RETN, MMP8 | 0,894 | 0,885 | 0,903 | 0,934 | 0,881 | 0,988 | 0,951 | 0,890 | 1 |
| IFI27, OAS1, SIGLEC1, ISG15, HERC6, IL1R2, FAM20A, MMP8 | 0,894 | 0,885 | 0,903 | 0,932 | 0,881 | 0,983 | 0,950 | 0,882 | 1 |
| IFI27, OAS1, SIGLEC1, IL1R2, OLAH, FAM20A, RETN, MMP8 | 0,894 | 0,884 | 0,903 | 0,933 | 0,878 | 0,987 | 0,952 | 0,893 | 1 |
| RSAD2, IFI27, IFI44L, SIGLEC1, HERC6, OLAH, FAM20A, MMP8 | 0,894 | 0,885 | 0,903 | 0,934 | 0,881 | 0,986 | 0,950 | 0,889 | 1 |
| RSAD2, IFI27, IFIT1, SIGLEC1, ISG15, OLAH, FAM20A, MMP8 | 0,894 | 0,885 | 0,903 | 0,929 | 0,872 | 0,986 | 0,950 | 0,893 | 1 |
| IFI27, OAS1, SIGLEC1, ISG15, HERC6, IL1R2, OLAH, FAM20A | 0,894 | 0,885 | 0,903 | 0,930 | 0,876 | 0,984 | 0,942 | 0,877 | 1 |
| IFI27, IFI44L, SIGLEC1, HERC6, IL1R2, FAM20A, RETN, MMP8 | 0,894 | 0,885 | 0,903 | 0,935 | 0,884 | 0,986 | 0,952 | 0,883 | 1 |
| IFI27, OAS1, IFIT1, IFI44L, SIGLEC1, HERC6, FAM20A, RETN | 0,894 | 0,885 | 0,902 | 0,930 | 0,877 | 0,984 | 0,936 | 0,864 | 1 |
| RSAD2, IFI27, OAS1, IFIT1, SIGLEC1, OLAH, FAM20A, MMP8 | 0,894 | 0,885 | 0,903 | 0,928 | 0,871 | 0,985 | 0,954 | 0,899 | 1 |
| IFI27, IFIT1, IFI44L, SIGLEC1, HERC6, IL1R2, FAM20A, RETN | 0,894 | 0,885 | 0,902 | 0,931 | 0,878 | 0,985 | 0,948 | 0,880 | 1 |
| IFI27, IFIT1, SIGLEC1, ISG15, SLC1A2, OLAH, FAM20A, MMP8 | 0,894 | 0,884 | 0,903 | 0,939 | 0,892 | 0,987 | 0,948 | 0,885 | 1 |
| IFI27, OAS1, IFI44L, SIGLEC1, HERC6, IL1R2, OLAH, FAM20A | 0,894 | 0,885 | 0,902 | 0,931 | 0,879 | 0,983 | 0,942 | 0,874 | 1 |
| IFI27, OAS1, ISG15, HERC6, IL1R2, OLAH, FAM20A, MMP8 | 0,894 | 0,884 | 0,903 | 0,930 | 0,878 | 0,982 | 0,925 | 0,848 | 1 |
| IFI27, IFI44L, SIGLEC1, IL1R2, OLAH, FAM20A, RETN, MMP8 | 0,894 | 0,885 | 0,902 | 0,934 | 0,881 | 0,987 | 0,952 | 0,889 | 1 |
| RSAD2, IFI27, IFIT1, SIGLEC1, ISG15, HERC6, OLAH, FAM20A | 0,894 | 0,884 | 0,903 | 0,931 | 0,877 | 0,985 | 0,939 | 0,875 | 1 |
| IFI27, IFI44L, ISG15, HERC6, IL1R2, OLAH, FAM20A, MMP8 | 0,893 | 0,884 | 0,903 | 0,930 | 0,877 | 0,983 | 0,930 | 0,858 | 1 |

| | | | | | | | | | |
|---|---|---|---|---|---|---|---|---|---|
| RSAD2, IFI27, IFIT1, IFI44L, SIGLEC1, IL1R2, FAM20A, MMP8 | 0,893 | 0,885 | 0,901 | 0,932 | 0,881 | 0,982 | 0,958 | 0,897 | 1 |
| IFI27, IFIT1, IFI44L, HERC6, IL1R2, OLAH, FAM20A, MMP8 | 0,893 | 0,884 | 0,902 | 0,931 | 0,880 | 0,983 | 0,936 | 0,864 | 1 |
| IFI27, SIGLEC1, ISG15, SLC1A2, IL1R2, OLAH, FAM20A, MMP8 | 0,893 | 0,884 | 0,902 | 0,940 | 0,895 | 0,985 | 0,945 | 0,875 | 1 |
| RSAD2, IFI27, IFIT1, SIGLEC1, HERC6, OLAH, FAM20A, RETN | 0,893 | 0,884 | 0,902 | 0,931 | 0,877 | 0,985 | 0,939 | 0,875 | 1 |
| RSAD2, IFI27, IFIT1, HERC6, IL1R2, OLAH, FAM20A, MMP8 | 0,893 | 0,884 | 0,902 | 0,931 | 0,880 | 0,983 | 0,934 | 0,863 | 1 |
| IFI27, OAS1, IFIT1, SIGLEC1, ISG15, HERC6, IL1R2, FAM20A | 0,893 | 0,885 | 0,902 | 0,934 | 0,884 | 0,983 | 0,947 | 0,881 | 1 |
| IFI27, OAS1, IFIT1, SIGLEC1, OLAH, FAM20A, RETN, MMP8 | 0,893 | 0,884 | 0,903 | 0,928 | 0,870 | 0,986 | 0,957 | 0,902 | 1 |
| IFI27, IFIT1, SIGLEC1, ISG15, IL1R2, FAM20A, RETN, MMP8 | 0,893 | 0,884 | 0,902 | 0,934 | 0,883 | 0,986 | 0,955 | 0,891 | 1 |
| IFI27, OAS1, IFI44L, HERC6, IL1R2, OLAH, FAM20A, MMP8 | 0,893 | 0,883 | 0,903 | 0,931 | 0,879 | 0,983 | 0,927 | 0,851 | 1 |
| IFI27, OAS1, IFIT1, SIGLEC1, ISG15, HERC6, FAM20A, RETN | 0,893 | 0,884 | 0,902 | 0,931 | 0,878 | 0,984 | 0,936 | 0,864 | 1 |
| IFI27, IFIT1, SIGLEC1, ISG15, OLAH, FAM20A, RETN, MMP8 | 0,893 | 0,883 | 0,902 | 0,930 | 0,873 | 0,987 | 0,955 | 0,901 | 1 |
| IFI27, OAS1, IFIT1, SIGLEC1, ISG15, IL1R2, FAM20A, MMP8 | 0,893 | 0,884 | 0,902 | 0,933 | 0,882 | 0,983 | 0,954 | 0,893 | 1 |
| IFI27, IFIT1, IFI44L, SIGLEC1, HERC6, FAM20A, RETN, MMP8 | 0,893 | 0,884 | 0,902 | 0,926 | 0,869 | 0,983 | 0,947 | 0,875 | 1 |
| RSAD2, IFI27, SIGLEC1, ISG15, HERC6, IL1R2, OLAH, FAM20A | 0,893 | 0,884 | 0,902 | 0,930 | 0,876 | 0,985 | 0,942 | 0,879 | 1 |
| IFI27, OAS1, IFIT1, IFI44L, SIGLEC1, IL1R2, OLAH, FAM20A | 0,893 | 0,884 | 0,901 | 0,927 | 0,870 | 0,983 | 0,948 | 0,885 | 1 |
| RSAD2, IFI27, OAS1, IFIT1, SIGLEC1, IL1R2, FAM20A, MMP8 | 0,893 | 0,884 | 0,901 | 0,932 | 0,880 | 0,983 | 0,955 | 0,894 | 1 |
| IFI27, IFIT1, IFI44L, SIGLEC1, ISG15, IL1R2, OLAH, FAM20A | 0,893 | 0,884 | 0,901 | 0,928 | 0,872 | 0,984 | 0,946 | 0,883 | 1 |
| RSAD2, IFI27, OAS1, IFIT1, SIGLEC1, HERC6, IL1R2, FAM20A | 0,893 | 0,884 | 0,901 | 0,934 | 0,885 | 0,983 | 0,946 | 0,879 | 1 |
| IFI27, IFIT1, SIGLEC1, HERC6, IL1R2, OLAH, FAM20A, RETN | 0,893 | 0,884 | 0,902 | 0,932 | 0,879 | 0,986 | 0,942 | 0,878 | 1 |
| RSAD2, IFI27, OAS1, IFI44L, SIGLEC1, IL1R2, FAM20A, MMP8 | 0,893 | 0,884 | 0,902 | 0,932 | 0,881 | 0,983 | 0,953 | 0,889 | 1 |
| RSAD2, IFI27, IFIT1, IFI44L, SIGLEC1, HERC6, IL1R2, FAM20A | 0,893 | 0,884 | 0,901 | 0,933 | 0,882 | 0,983 | 0,946 | 0,878 | 1 |
| RSAD2, IFI27, OAS1, SIGLEC1, ISG15, OLAH, FAM20A, MMP8 | 0,893 | 0,883 | 0,902 | 0,930 | 0,873 | 0,987 | 0,946 | 0,884 | 1 |
| RSAD2, IFI27, ISG15, HERC6, IL1R2, OLAH, FAM20A, MMP8 | 0,893 | 0,883 | 0,902 | 0,930 | 0,879 | 0,982 | 0,925 | 0,849 | 1 |
| IFI27, OAS1, IFI44L, SIGLEC1, ISG15, OLAH, FAM20A, MMP8 | 0,892 | 0,883 | 0,902 | 0,927 | 0,870 | 0,985 | 0,949 | 0,891 | 1 |
| IFI27, OAS1, IFI44L, SIGLEC1, ISG15, HERC6, OLAH, FAM20A | 0,892 | 0,883 | 0,902 | 0,930 | 0,876 | 0,985 | 0,940 | 0,874 | 1 |
| RSAD2, IFI27, IFIT1, SIGLEC1, ISG15, IL1R2, FAM20A, MMP8 | 0,892 | 0,884 | 0,901 | 0,933 | 0,883 | 0,983 | 0,954 | 0,890 | 1 |
| IFI27, IFI44L, SIGLEC1, ISG15, SLC1A2, OLAH, FAM20A, MMP8 | 0,892 | 0,883 | 0,901 | 0,936 | 0,887 | 0,984 | 0,947 | 0,882 | 1 |
| IFI27, OAS1, SIGLEC1, ISG15, SLC1A2, OLAH, FAM20A, MMP8 | 0,892 | 0,883 | 0,902 | 0,938 | 0,889 | 0,987 | 0,941 | 0,875 | 1 |

| Genes | | | | | | | | | |
|---|---|---|---|---|---|---|---|---|---|
| RSAD2, IFI27, IFI44L, SIGLEC1, HERC6, IL1R2, OLAH, FAM20A | 0,892 | 0,884 | 0,901 | 0,933 | 0,880 | 0,985 | 0,947 | 0,883 | 1 |
| RSAD2, IFI27, OAS1, SIGLEC1, ISG15, HERC6, OLAH, FAM20A | 0,892 | 0,883 | 0,902 | 0,931 | 0,877 | 0,986 | 0,937 | 0,869 | 1 |
| IFI27, OAS1, IFI44L, SIGLEC1, ISG15, IL1R2, FAM20A, MMP8 | 0,892 | 0,883 | 0,901 | 0,934 | 0,884 | 0,984 | 0,950 | 0,882 | 1 |
| RSAD2, IFI27, OAS1, IFI44L, SIGLEC1, HERC6, OLAH, FAM20A | 0,892 | 0,883 | 0,901 | 0,931 | 0,877 | 0,984 | 0,940 | 0,875 | 1 |
| IFI27, OAS1, SIGLEC1, ISG15, HERC6, OLAH, FAM20A, RETN | 0,892 | 0,883 | 0,902 | 0,930 | 0,876 | 0,985 | 0,940 | 0,874 | 1 |
| IFI27, OAS1, IFIT1, IFI44L, SIGLEC1, ISG15, OLAH, FAM20A | 0,892 | 0,884 | 0,901 | 0,926 | 0,868 | 0,984 | 0,943 | 0,884 | 1 |
| IFI27, IFI44L, SIGLEC1, ISG15, OLAH, FAM20A, RETN, MMP8 | 0,892 | 0,883 | 0,902 | 0,929 | 0,872 | 0,986 | 0,951 | 0,891 | 1 |
| IFI27, IFIT1, IFI44L, SIGLEC1, SLC1A2, OLAH, FAM20A, MMP8 | 0,892 | 0,883 | 0,901 | 0,934 | 0,883 | 0,986 | 0,952 | 0,890 | 1 |
| IFI27, IFIT1, SIGLEC1, SLC1A2, IL1R2, OLAH, FAM20A, MMP8 | 0,892 | 0,883 | 0,901 | 0,939 | 0,894 | 0,985 | 0,954 | 0,893 | 1 |
| RSAD2, IFI27, IFIT1, SIGLEC1, IL1R2, FAM20A, RETN, MMP8 | 0,892 | 0,883 | 0,901 | 0,935 | 0,883 | 0,987 | 0,952 | 0,888 | 1 |
| RSAD2, IFI27, OAS1, SIGLEC1, IL1R2, FAM20A, RETN, MMP8 | 0,892 | 0,882 | 0,902 | 0,934 | 0,882 | 0,986 | 0,953 | 0,889 | 1 |
| IFI27, IFIT1, IFI44L, SIGLEC1, OLAH, FAM20A, RETN, MMP8 | 0,892 | 0,882 | 0,901 | 0,930 | 0,874 | 0,987 | 0,959 | 0,906 | 1 |
| IFI27, OAS1, SIGLEC1, ISG15, IL1R2, FAM20A, RETN, MMP8 | 0,892 | 0,883 | 0,901 | 0,933 | 0,879 | 0,986 | 0,951 | 0,885 | 1 |
| RSAD2, IFI27, IFIT1, SIGLEC1, HERC6, FAM20A, RETN, MMP8 | 0,892 | 0,883 | 0,901 | 0,931 | 0,878 | 0,985 | 0,942 | 0,870 | 1 |
| IFI27, OAS1, IFI44L, SIGLEC1, IL1R2, FAM20A, RETN, MMP8 | 0,892 | 0,883 | 0,901 | 0,933 | 0,881 | 0,985 | 0,952 | 0,888 | 1 |
| IFI27, IFIT1, SIGLEC1, ISG15, HERC6, FAM20A, RETN, MMP8 | 0,892 | 0,883 | 0,901 | 0,927 | 0,870 | 0,984 | 0,949 | 0,878 | 1 |
| IFI27, IFIT1, SIGLEC1, ISG15, IL1R2, OLAH, FAM20A, RETN | 0,892 | 0,883 | 0,900 | 0,929 | 0,872 | 0,986 | 0,946 | 0,884 | 1 |
| RSAD2, IFI27, IFIT1, IFI44L, SIGLEC1, OLAH, FAM20A, MMP8 | 0,892 | 0,883 | 0,901 | 0,928 | 0,871 | 0,985 | 0,954 | 0,899 | 1 |
| IFI27, OAS1, HERC6, IL1R2, OLAH, FAM20A, RETN, MMP8 | 0,892 | 0,883 | 0,901 | 0,935 | 0,883 | 0,986 | 0,929 | 0,855 | 1 |
| RSAD2, IFI27, HERC6, IL1R2, OLAH, FAM20A, RETN, MMP8 | 0,892 | 0,882 | 0,901 | 0,934 | 0,882 | 0,986 | 0,932 | 0,858 | 1 |
| IFI27, OAS1, IFIT1, IFI44L, HERC6, OLAH, FAM20A, MMP8 | 0,892 | 0,882 | 0,901 | 0,929 | 0,874 | 0,983 | 0,934 | 0,865 | 1 |
| IFI27, IFIT1, ISG15, HERC6, IL1R2, OLAH, FAM20A, MMP8 | 0,892 | 0,883 | 0,901 | 0,930 | 0,877 | 0,982 | 0,929 | 0,857 | 1 |
| RSAD2, IFI27, SIGLEC1, SLC1A2, IL1R2, OLAH, FAM20A, MMP8 | 0,892 | 0,883 | 0,901 | 0,937 | 0,890 | 0,984 | 0,950 | 0,888 | 1 |
| IFI27, OAS1, SIGLEC1, HERC6, SLC1A2, OLAH, FAM20A, MMP8 | 0,892 | 0,882 | 0,901 | 0,934 | 0,884 | 0,984 | 0,941 | 0,877 | 1 |
| RSAD2, IFI27, SIGLEC1, ISG15, IL1R2, FAM20A, RETN, MMP8 | 0,892 | 0,882 | 0,901 | 0,935 | 0,883 | 0,987 | 0,949 | 0,880 | 1 |
| IFI27, IFIT1, IFI44L, SIGLEC1, ISG15, OLAH, FAM20A, RETN | 0,892 | 0,883 | 0,900 | 0,927 | 0,870 | 0,985 | 0,942 | 0,880 | 1 |
| IFI27, OAS1, IFIT1, SIGLEC1, IL1R2, OLAH, FAM20A, RETN | 0,892 | 0,883 | 0,900 | 0,926 | 0,869 | 0,983 | 0,949 | 0,887 | 1 |
| IFI27, OAS1, SIGLEC1, ISG15, OLAH, FAM20A, RETN, MMP8 | 0,892 | 0,881 | 0,902 | 0,928 | 0,870 | 0,986 | 0,951 | 0,893 | 1 |

| | | | | | | | | |
|---|---|---|---|---|---|---|---|---|
| IFI27, SIGLEC1, ISG15, HERC6, OLAH, FAM20A, RETN, MMP8 | 0,892 | 0,881 | 0,902 | 0,928 | 0,870 | 0,986 | 0,957 | 0,899 | 1 |
| RSAD2, IFI27, OAS1, IFI44L, HERC6, OLAH, FAM20A, MMP8 | 0,892 | 0,882 | 0,901 | 0,930 | 0,876 | 0,984 | 0,934 | 0,866 | 1 |
| IFI27, OAS1, IFI44L, SIGLEC1, ISG15, HERC6, IL1R2, FAM20A | 0,891 | 0,883 | 0,900 | 0,931 | 0,881 | 0,982 | 0,946 | 0,879 | 1 |
| IFI27, OAS1, SIGLEC1, HERC6, IL1R2, OLAH, FAM20A, RETN | 0,891 | 0,882 | 0,901 | 0,930 | 0,876 | 0,983 | 0,939 | 0,871 | 1 |
| IFI27, IFIT1, SIGLEC1, ISG15, HERC6, IL1R2, FAM20A, RETN | 0,891 | 0,882 | 0,900 | 0,933 | 0,880 | 0,986 | 0,950 | 0,883 | 1 |
| IFI27, IFIT1, IFI44L, SIGLEC1, IL1R2, OLAH, FAM20A, RETN | 0,891 | 0,883 | 0,900 | 0,927 | 0,870 | 0,983 | 0,947 | 0,884 | 1 |
| IFI27, ISG15, HERC6, IL1R2, OLAH, FAM20A, RETN, MMP8 | 0,891 | 0,882 | 0,901 | 0,932 | 0,879 | 0,985 | 0,932 | 0,859 | 1 |
| IFI27, OAS1, IFIT1, SIGLEC1, ISG15, IL1R2, OLAH, FAM20A | 0,891 | 0,883 | 0,900 | 0,928 | 0,871 | 0,985 | 0,943 | 0,881 | 1 |
| RSAD2, IFI27, IFIT1, IFI44L, HERC6, OLAH, FAM20A, MMP8 | 0,891 | 0,882 | 0,901 | 0,930 | 0,875 | 0,984 | 0,934 | 0,866 | 1 |
| IFI27, SIGLEC1, ISG15, HERC6, IL1R2, FAM20A, RETN, MMP8 | 0,891 | 0,882 | 0,901 | 0,933 | 0,880 | 0,986 | 0,953 | 0,885 | 1 |
| IFI27, IFI44L, SIGLEC1, ISG15, IL1R2, OLAH, FAM20A, RETN | 0,891 | 0,883 | 0,900 | 0,928 | 0,872 | 0,983 | 0,946 | 0,882 | 1 |
| IFI27, OAS1, IFI44L, SIGLEC1, HERC6, IL1R2, FAM20A, RETN | 0,891 | 0,883 | 0,900 | 0,934 | 0,884 | 0,984 | 0,943 | 0,876 | 1 |
| IFI27, OAS1, IFI44L, SIGLEC1, HERC6, OLAH, FAM20A, RETN | 0,891 | 0,882 | 0,900 | 0,931 | 0,878 | 0,984 | 0,939 | 0,871 | 1 |
| RSAD2, IFI27, IFIT1, SIGLEC1, ISG15, HERC6, IL1R2, FAM20A | 0,891 | 0,883 | 0,900 | 0,934 | 0,884 | 0,985 | 0,948 | 0,882 | 1 |
| IFI27, SIGLEC1, ISG15, HERC6, SLC1A2, OLAH, FAM20A, MMP8 | 0,891 | 0,882 | 0,901 | 0,935 | 0,885 | 0,986 | 0,945 | 0,878 | 1 |
| RSAD2, IFI27, OAS1, IFIT1, IFI44L, HERC6, OLAH, FAM20A | 0,891 | 0,882 | 0,900 | 0,930 | 0,875 | 0,984 | 0,930 | 0,859 | 1 |
| RSAD2, IFI27, OAS1, SIGLEC1, HERC6, OLAH, FAM20A, RETN | 0,891 | 0,882 | 0,901 | 0,930 | 0,876 | 0,985 | 0,936 | 0,867 | 1 |
| RSAD2, IFI27, IFI44L, SIGLEC1, ISG15, IL1R2, FAM20A, MMP8 | 0,891 | 0,882 | 0,900 | 0,933 | 0,883 | 0,984 | 0,950 | 0,882 | 1 |
| IFI27, OAS1, IFIT1, IFI44L, SIGLEC1, OLAH, FAM20A, RETN | 0,891 | 0,882 | 0,900 | 0,924 | 0,865 | 0,983 | 0,951 | 0,891 | 1 |
| RSAD2, IFI27, OAS1, IFIT1, SIGLEC1, HERC6, FAM20A, RETN | 0,891 | 0,882 | 0,900 | 0,930 | 0,877 | 0,984 | 0,934 | 0,861 | 1 |
| RSAD2, IFI27, OAS1, SIGLEC1, ISG15, IL1R2, FAM20A, MMP8 | 0,891 | 0,882 | 0,901 | 0,934 | 0,883 | 0,985 | 0,951 | 0,883 | 1 |
| RSAD2, IFI27, OAS1, IFIT1, HERC6, OLAH, FAM20A, MMP8 | 0,891 | 0,882 | 0,901 | 0,930 | 0,877 | 0,983 | 0,934 | 0,865 | 1 |
| IFI27, IFI44L, SIGLEC1, SLC1A2, IL1R2, OLAH, FAM20A, MMP8 | 0,891 | 0,882 | 0,900 | 0,935 | 0,888 | 0,983 | 0,950 | 0,884 | 1 |
| RSAD2, IFI27, OAS1, IFI44L, SIGLEC1, HERC6, IL1R2, FAM20A | 0,891 | 0,883 | 0,900 | 0,933 | 0,884 | 0,983 | 0,946 | 0,880 | 1 |
| IFI27, IFI44L, SIGLEC1, HERC6, OLAH, FAM20A, RETN, MMP8 | 0,891 | 0,881 | 0,901 | 0,928 | 0,872 | 0,984 | 0,950 | 0,885 | 1 |
| RSAD2, IFI27, SIGLEC1, ISG15, SLC1A2, OLAH, FAM20A, MMP8 | 0,891 | 0,882 | 0,901 | 0,938 | 0,890 | 0,986 | 0,941 | 0,875 | 1 |
| IFI27, OAS1, IFIT1, IFI44L, HERC6, IL1R2, OLAH, FAM20A | 0,891 | 0,882 | 0,900 | 0,930 | 0,878 | 0,983 | 0,925 | 0,848 | 1 |
| RSAD2, IFI27, IFIT1, SIGLEC1, HERC6, IL1R2, FAM20A, RETN | 0,891 | 0,882 | 0,900 | 0,933 | 0,880 | 0,985 | 0,948 | 0,880 | 1 |

| | | | | | | | | | |
|---|---|---|---|---|---|---|---|---|---|
| IFI27, OAS1, ISG15, HERC6, OLAH, FAM20A, RETN, MMP8 | 0,891 | 0,882 | 0,901 | 0,931 | 0,876 | 0,986 | 0,930 | 0,856 | 1 |
| RSAD2, IFI27, IFI44L, SIGLEC1, ISG15, HERC6, OLAH, FAM20A | 0,891 | 0,882 | 0,900 | 0,928 | 0,873 | 0,984 | 0,941 | 0,877 | 1 |
| RSAD2, IFI27, OAS1, IFI44L, SIGLEC1, OLAH, FAM20A, MMP8 | 0,891 | 0,881 | 0,901 | 0,927 | 0,869 | 0,985 | 0,954 | 0,900 | 1 |
| IFI27, OAS1, IFIT1, SIGLEC1, SLC1A2, OLAH, FAM20A, MMP8 | 0,891 | 0,882 | 0,900 | 0,937 | 0,887 | 0,986 | 0,946 | 0,883 | 1 |
| IFI27, OAS1, SIGLEC1, SLC1A2, IL1R2, OLAH, FAM20A, MMP8 | 0,891 | 0,882 | 0,900 | 0,937 | 0,889 | 0,984 | 0,945 | 0,879 | 1 |
| RSAD2, IFI27, OAS1, SIGLEC1, OLAH, FAM20A, RETN, MMP8 | 0,891 | 0,881 | 0,901 | 0,928 | 0,870 | 0,986 | 0,958 | 0,905 | 1 |
| RSAD2, IFI27, SIGLEC1, ISG15, OLAH, FAM20A, RETN, MMP8 | 0,891 | 0,881 | 0,901 | 0,930 | 0,872 | 0,987 | 0,950 | 0,893 | 1 |
| RSAD2, IFI27, OAS1, HERC6, OLAH, FAM20A, RETN, MMP8 | 0,891 | 0,881 | 0,900 | 0,931 | 0,876 | 0,985 | 0,929 | 0,855 | 1 |
| RSAD2, IFI27, IFI44L, SIGLEC1, IL1R2, FAM20A, RETN, MMP8 | 0,891 | 0,881 | 0,900 | 0,934 | 0,882 | 0,986 | 0,951 | 0,883 | 1 |
| IFI27, OAS1, IFIT1, SIGLEC1, HERC6, SLC1A2, OLAH, FAM20A | 0,891 | 0,881 | 0,901 | 0,937 | 0,890 | 0,984 | 0,938 | 0,871 | 1 |
| IFI27, IFI44L, SIGLEC1, ISG15, HERC6, OLAH, FAM20A, RETN | 0,891 | 0,881 | 0,900 | 0,926 | 0,870 | 0,983 | 0,943 | 0,878 | 1 |
| RSAD2, IFI27, IFIT1, IFI44L, SIGLEC1, HERC6, FAM20A, RETN | 0,891 | 0,882 | 0,900 | 0,929 | 0,875 | 0,983 | 0,935 | 0,861 | 1 |
| IFI27, OAS1, IFIT1, HERC6, OLAH, FAM20A, RETN, MMP8 | 0,891 | 0,881 | 0,901 | 0,932 | 0,878 | 0,987 | 0,932 | 0,859 | 1 |
| RSAD2, IFI27, SIGLEC1, HERC6, IL1R2, OLAH, FAM20A, RETN | 0,891 | 0,882 | 0,900 | 0,931 | 0,877 | 0,985 | 0,945 | 0,881 | 1 |
| RSAD2, IFI27, OAS1, IFIT1, IFI44L, SIGLEC1, OLAH, FAM20A | 0,891 | 0,882 | 0,900 | 0,923 | 0,864 | 0,983 | 0,950 | 0,891 | 1 |
| IFI27, OAS1, SIGLEC1, ISG15, HERC6, FAM20A, RETN, MMP8 | 0,891 | 0,881 | 0,901 | 0,929 | 0,872 | 0,986 | 0,943 | 0,872 | 1 |
| IFI27, OAS1, IFI44L, ISG15, HERC6, IL1R2, OLAH, FAM20A | 0,891 | 0,881 | 0,900 | 0,929 | 0,876 | 0,982 | 0,927 | 0,853 | 1 |
| RSAD2, IFI27, OAS1, SIGLEC1, HERC6, FAM20A, RETN, MMP8 | 0,891 | 0,881 | 0,900 | 0,933 | 0,878 | 0,987 | 0,942 | 0,872 | 1 |
| IFI27, IFIT1, SIGLEC1, HERC6, SLC1A2, OLAH, FAM20A, MMP8 | 0,891 | 0,881 | 0,900 | 0,935 | 0,886 | 0,985 | 0,953 | 0,893 | 1 |
| IFI27, SIGLEC1, HERC6, IL1R2, OLAH, FAM20A, RETN, MMP8 | 0,891 | 0,881 | 0,901 | 0,931 | 0,876 | 0,986 | 0,957 | 0,898 | 1 |
| IFI27, OAS1, IFI44L, SIGLEC1, ISG15, IL1R2, OLAH, FAM20A | 0,891 | 0,882 | 0,899 | 0,927 | 0,871 | 0,983 | 0,947 | 0,885 | 1 |
| RSAD2, IFI27, OAS1, IFI44L, HERC6, IL1R2, OLAH, FAM20A | 0,891 | 0,881 | 0,900 | 0,931 | 0,878 | 0,983 | 0,929 | 0,856 | 1 |
| RSAD2, IFI27, IFIT1, SIGLEC1, OLAH, FAM20A, RETN, MMP8 | 0,891 | 0,881 | 0,900 | 0,929 | 0,872 | 0,987 | 0,955 | 0,902 | 1 |
| IFI27, IFI44L, ISG15, SLC1A2, IL1R2, OLAH, FAM20A, MMP8 | 0,891 | 0,882 | 0,899 | 0,935 | 0,888 | 0,982 | 0,923 | 0,844 | 1 |
| RSAD2, IFI27, OAS1, ISG15, HERC6, OLAH, FAM20A, MMP8 | 0,890 | 0,881 | 0,900 | 0,927 | 0,873 | 0,982 | 0,932 | 0,862 | 1 |
| IFI27, IFIT1, IFI44L, ISG15, HERC6, OLAH, FAM20A, MMP8 | 0,890 | 0,881 | 0,900 | 0,926 | 0,870 | 0,982 | 0,936 | 0,870 | 1 |
| RSAD2, IFI27, OAS1, IFIT1, IL1R2, OLAH, FAM20A, MMP8 | 0,890 | 0,881 | 0,899 | 0,929 | 0,874 | 0,983 | 0,936 | 0,868 | 1 |
| RSAD2, IFI27, IFI44L, SIGLEC1, ISG15, OLAH, FAM20A, MMP8 | 0,890 | 0,881 | 0,900 | 0,929 | 0,872 | 0,985 | 0,950 | 0,893 | 1 |

| | | | | | | | | |
|---|---|---|---|---|---|---|---|---|
| RSAD2, IFI27, IFIT1, IFI44L, SIGLEC1, IL1R2, OLAH, FAM20A | 0,890 | 0,882 | 0,899 | 0,927 | 0,871 | 0,984 | 0,947 | 0,884 | 1 |
| RSAD2, IFI27, SIGLEC1, ISG15, HERC6, OLAH, FAM20A, RETN | 0,890 | 0,881 | 0,900 | 0,931 | 0,877 | 0,985 | 0,941 | 0,878 | 1 |
| IFI27, OAS1, IFIT1, SIGLEC1, ISG15, OLAH, FAM20A, RETN | 0,890 | 0,881 | 0,899 | 0,926 | 0,867 | 0,984 | 0,943 | 0,882 | 1 |
| IFI27, IFIT1, IFI44L, SIGLEC1, ISG15, SLC1A2, OLAH, FAM20A | 0,890 | 0,881 | 0,899 | 0,935 | 0,885 | 0,985 | 0,939 | 0,873 | 1 |
| RSAD2, IFI27, OAS1, SIGLEC1, HERC6, IL1R2, FAM20A, RETN | 0,890 | 0,881 | 0,900 | 0,931 | 0,880 | 0,983 | 0,945 | 0,877 | 1 |
| RSAD2, IFI27, IFIT1, SIGLEC1, SLC1A2, OLAH, FAM20A, MMP8 | 0,890 | 0,881 | 0,900 | 0,935 | 0,885 | 0,986 | 0,948 | 0,885 | 1 |
| RSAD2, IFI27, OAS1, IFIT1, SIGLEC1, IL1R2, OLAH, FAM20A | 0,890 | 0,882 | 0,899 | 0,926 | 0,869 | 0,983 | 0,949 | 0,886 | 1 |
| IFI27, OAS1, SIGLEC1, HERC6, SLC1A2, IL1R2, FAM20A, MMP8 | 0,890 | 0,881 | 0,900 | 0,936 | 0,891 | 0,981 | 0,948 | 0,879 | 1 |
| IFI27, OAS1, IFI44L, SIGLEC1, HERC6, FAM20A, RETN, MMP8 | 0,890 | 0,881 | 0,900 | 0,931 | 0,876 | 0,985 | 0,943 | 0,871 | 1 |
| RSAD2, IFI27, SIGLEC1, HERC6, SLC1A2, OLAH, FAM20A, MMP8 | 0,890 | 0,881 | 0,900 | 0,936 | 0,887 | 0,985 | 0,943 | 0,878 | 1 |
| RSAD2, IFI27, OAS1, IFIT1, SIGLEC1, ISG15, OLAH, FAM20A | 0,890 | 0,881 | 0,899 | 0,926 | 0,867 | 0,984 | 0,945 | 0,884 | 1 |
| RSAD2, IFI27, OAS1, SIGLEC1, ISG15, HERC6, IL1R2, FAM20A | 0,890 | 0,881 | 0,899 | 0,931 | 0,878 | 0,983 | 0,948 | 0,882 | 1 |
| IFI27, OAS1, IFIT1, IFI44L, HERC6, OLAH, FAM20A, RETN | 0,890 | 0,881 | 0,899 | 0,931 | 0,879 | 0,984 | 0,928 | 0,855 | 1 |
| RSAD2, IFI27, IFI44L, SIGLEC1, ISG15, HERC6, IL1R2, FAM20A | 0,890 | 0,881 | 0,899 | 0,930 | 0,878 | 0,982 | 0,942 | 0,873 | 1 |
| IFI27, IFIT1, SIGLEC1, ISG15, HERC6, SLC1A2, OLAH, FAM20A | 0,890 | 0,880 | 0,900 | 0,938 | 0,892 | 0,984 | 0,936 | 0,868 | 1 |
| RSAD2, IFI27, OAS1, IFIT1, HERC6, IL1R2, OLAH, FAM20A | 0,890 | 0,881 | 0,899 | 0,929 | 0,877 | 0,981 | 0,928 | 0,854 | 1 |
| RSAD2, IFI27, IFIT1, SIGLEC1, ISG15, HERC6, FAM20A, RETN | 0,890 | 0,881 | 0,899 | 0,929 | 0,875 | 0,983 | 0,935 | 0,861 | 1 |
| IFI27, IFIT1, HERC6, IL1R2, OLAH, FAM20A, RETN, MMP8 | 0,890 | 0,881 | 0,899 | 0,931 | 0,878 | 0,985 | 0,934 | 0,861 | 1 |
| IFI27, OAS1, IFIT1, ISG15, HERC6, IL1R2, OLAH, FAM20A | 0,890 | 0,881 | 0,899 | 0,929 | 0,876 | 0,981 | 0,926 | 0,851 | 1 |
| RSAD2, IFI27, OAS1, ISG15, HERC6, IL1R2, OLAH, FAM20A | 0,890 | 0,881 | 0,899 | 0,929 | 0,876 | 0,981 | 0,926 | 0,852 | 1 |
| IFI27, IFI44L, SIGLEC1, HERC6, IL1R2, OLAH, FAM20A, RETN | 0,890 | 0,881 | 0,899 | 0,927 | 0,872 | 0,982 | 0,947 | 0,881 | 1 |
| IFI27, IFIT1, IFI44L, ISG15, HERC6, IL1R2, OLAH, FAM20A | 0,890 | 0,881 | 0,899 | 0,929 | 0,875 | 0,983 | 0,928 | 0,855 | 1 |
| RSAD2, IFI27, IFIT1, HERC6, OLAH, FAM20A, RETN, MMP8 | 0,890 | 0,880 | 0,899 | 0,929 | 0,874 | 0,985 | 0,932 | 0,859 | 1 |
| IFI27, OAS1, IFI44L, SIGLEC1, ISG15, HERC6, FAM20A, RETN | 0,890 | 0,881 | 0,899 | 0,928 | 0,873 | 0,982 | 0,938 | 0,864 | 1 |
| IFI27, OAS1, IFI44L, ISG15, HERC6, OLAH, FAM20A, MMP8 | 0,890 | 0,880 | 0,900 | 0,928 | 0,873 | 0,983 | 0,930 | 0,860 | 1 |
| IFI27, IFIT1, IFI44L, ISG15, IL1R2, OLAH, FAM20A, MMP8 | 0,890 | 0,881 | 0,899 | 0,928 | 0,874 | 0,982 | 0,934 | 0,864 | 1 |
| RSAD2, IFI27, ISG15, HERC6, OLAH, FAM20A, RETN, MMP8 | 0,890 | 0,880 | 0,899 | 0,931 | 0,877 | 0,985 | 0,932 | 0,859 | 1 |
| RSAD2, IFI27, IFIT1, IFI44L, HERC6, IL1R2, OLAH, FAM20A | 0,890 | 0,881 | 0,899 | 0,929 | 0,875 | 0,983 | 0,928 | 0,855 | 1 |

| | | | | | | | | |
|---|---|---|---|---|---|---|---|---|
| IFI27, OAS1, IFIT1, IFI44L, ISG15, HERC6, OLAH, FAM20A | 0,890 | 0,880 | 0,900 | 0,928 | 0,873 | 0,983 | 0,927 | 0,854 | 1 |
| RSAD2, IFI27, IFIT1, SIGLEC1, ISG15, IL1R2, OLAH, FAM20A | 0,890 | 0,881 | 0,898 | 0,929 | 0,873 | 0,985 | 0,945 | 0,881 | 1 |
| IFI27, IFI44L, SIGLEC1, ISG15, SLC1A2, IL1R2, FAM20A, MMP8 | 0,890 | 0,881 | 0,898 | 0,938 | 0,895 | 0,981 | 0,952 | 0,883 | 1 |
| RSAD2, IFI27, SIGLEC1, ISG15, HERC6, FAM20A, RETN, MMP8 | 0,890 | 0,880 | 0,899 | 0,931 | 0,877 | 0,986 | 0,945 | 0,870 | 1 |
| IFI27, IFIT1, IFI44L, SIGLEC1, HERC6, SLC1A2, OLAH, FAM20A | 0,890 | 0,880 | 0,899 | 0,934 | 0,885 | 0,984 | 0,945 | 0,882 | 1 |
| RSAD2, IFI27, IFIT1, IFI44L, SIGLEC1, ISG15, OLAH, FAM20A | 0,890 | 0,881 | 0,898 | 0,927 | 0,869 | 0,984 | 0,945 | 0,886 | 1 |
| RSAD2, IFI27, OAS1, SIGLEC1, SLC1A2, OLAH, FAM20A, MMP8 | 0,890 | 0,880 | 0,899 | 0,934 | 0,883 | 0,986 | 0,945 | 0,880 | 1 |
| IFI27, IFIT1, IFI44L, SIGLEC1, ISG15, IL1R2, FAM20A, RETN | 0,890 | 0,881 | 0,898 | 0,929 | 0,874 | 0,983 | 0,949 | 0,886 | 1 |
| IFI27, OAS1, SIGLEC1, ISG15, IL1R2, OLAH, FAM20A, RETN | 0,889 | 0,880 | 0,899 | 0,926 | 0,869 | 0,983 | 0,946 | 0,882 | 1 |
| RSAD2, IFI27, IFI44L, SIGLEC1, HERC6, OLAH, FAM20A, RETN | 0,889 | 0,880 | 0,899 | 0,930 | 0,876 | 0,985 | 0,940 | 0,874 | 1 |
| IFI27, OAS1, IFIT1, IFI44L, SIGLEC1, IL1R2, FAM20A, RETN | 0,889 | 0,881 | 0,898 | 0,927 | 0,872 | 0,983 | 0,949 | 0,887 | 1 |
| IFI27, OAS1, IFIT1, ISG15, HERC6, OLAH, FAM20A, MMP8 | 0,889 | 0,880 | 0,899 | 0,926 | 0,870 | 0,981 | 0,933 | 0,863 | 1 |
| IFI27, OAS1, SIGLEC1, ISG15, HERC6, IL1R2, FAM20A, RETN | 0,889 | 0,880 | 0,899 | 0,932 | 0,880 | 0,983 | 0,945 | 0,877 | 1 |
| IFI27, SIGLEC1, ISG15, HERC6, IL1R2, OLAH, FAM20A, RETN | 0,889 | 0,880 | 0,899 | 0,928 | 0,873 | 0,983 | 0,948 | 0,886 | 1 |
| RSAD2, IFI27, IFI44L, ISG15, HERC6, OLAH, FAM20A, MMP8 | 0,889 | 0,879 | 0,899 | 0,930 | 0,876 | 0,984 | 0,933 | 0,865 | 1 |
| IFI27, OAS1, IFIT1, IFI44L, IL1R2, OLAH, FAM20A, MMP8 | 0,889 | 0,880 | 0,899 | 0,927 | 0,873 | 0,982 | 0,933 | 0,863 | 1 |
| IFI27, OAS1, IFIT1, ISG15, IL1R2, OLAH, FAM20A, MMP8 | 0,889 | 0,880 | 0,899 | 0,928 | 0,875 | 0,981 | 0,934 | 0,864 | 1 |
| IFI27, OAS1, IFIT1, SIGLEC1, SLC1A2, IL1R2, FAM20A, MMP8 | 0,889 | 0,880 | 0,898 | 0,935 | 0,889 | 0,980 | 0,951 | 0,884 | 1 |
| RSAD2, IFI27, SIGLEC1, HERC6, SLC1A2, IL1R2, FAM20A, MMP8 | 0,889 | 0,880 | 0,899 | 0,936 | 0,890 | 0,982 | 0,954 | 0,886 | 1 |
| IFI27, IFI44L, ISG15, IL1R2, OLAH, FAM20A, RETN, MMP8 | 0,889 | 0,880 | 0,899 | 0,930 | 0,875 | 0,984 | 0,934 | 0,865 | 1 |
| RSAD2, IFI27, OAS1, IFI44L, ISG15, HERC6, OLAH, FAM20A | 0,889 | 0,879 | 0,899 | 0,929 | 0,875 | 0,984 | 0,928 | 0,857 | 1,000 |
| RSAD2, IFI27, OAS1, SIGLEC1, ISG15, IL1R2, OLAH, FAM20A | 0,889 | 0,880 | 0,898 | 0,926 | 0,868 | 0,983 | 0,946 | 0,882 | 1 |
| IFI27, OAS1, IFIT1, ISG15, HERC6, OLAH, FAM20A, RETN | 0,889 | 0,880 | 0,898 | 0,931 | 0,879 | 0,983 | 0,934 | 0,864 | 1 |
| IFI27, OAS1, IFI44L, ISG15, IL1R2, OLAH, FAM20A, MMP8 | 0,889 | 0,879 | 0,899 | 0,929 | 0,876 | 0,982 | 0,929 | 0,858 | 1 |
| IFI27, IFIT1, IFI44L, ISG15, HERC6, OLAH, FAM20A, RETN | 0,889 | 0,880 | 0,898 | 0,927 | 0,873 | 0,982 | 0,933 | 0,863 | 1 |
| IFI27, IFI44L, SIGLEC1, ISG15, HERC6, IL1R2, FAM20A, RETN | 0,889 | 0,880 | 0,898 | 0,931 | 0,878 | 0,984 | 0,942 | 0,872 | 1 |
| IFI27, IFIT1, IFI44L, SIGLEC1, SLC1A2, IL1R2, FAM20A, MMP8 | 0,889 | 0,880 | 0,898 | 0,935 | 0,890 | 0,981 | 0,954 | 0,886 | 1 |
| RSAD2, IFI27, IFIT1, IFI44L, IL1R2, OLAH, FAM20A, MMP8 | 0,889 | 0,880 | 0,898 | 0,929 | 0,875 | 0,983 | 0,933 | 0,863 | 1 |

| | | | | | | | | |
|---|---|---|---|---|---|---|---|---|
| IFI27, OAS1, IFIT1, IFI44L, SIGLEC1, SLC1A2, OLAH, FAM20A | 0,889 | 0,880 | 0,898 | 0,931 | 0,877 | 0,985 | 0,941 | 0,877 | 1 |
| RSAD2, IFI27, IFI44L, SIGLEC1, HERC6, IL1R2, FAM20A, RETN | 0,889 | 0,879 | 0,899 | 0,932 | 0,880 | 0,984 | 0,943 | 0,875 | 1 |
| RSAD2, IFI27, IFI44L, SIGLEC1, ISG15, IL1R2, OLAH, FAM20A | 0,889 | 0,880 | 0,898 | 0,928 | 0,873 | 0,983 | 0,946 | 0,882 | 1 |
| RSAD2, IFI27, OAS1, IFI44L, SIGLEC1, IL1R2, OLAH, FAM20A | 0,889 | 0,880 | 0,898 | 0,926 | 0,869 | 0,982 | 0,952 | 0,892 | 1 |
| IFI27, OAS1, IFI44L, SIGLEC1, SLC1A2, OLAH, FAM20A, MMP8 | 0,889 | 0,879 | 0,899 | 0,935 | 0,884 | 0,986 | 0,942 | 0,877 | 1 |
| RSAD2, IFI27, IFI44L, ISG15, HERC6, IL1R2, OLAH, FAM20A | 0,889 | 0,880 | 0,898 | 0,929 | 0,875 | 0,983 | 0,929 | 0,857 | 1 |
| IFI27, IFI44L, ISG15, HERC6, OLAH, FAM20A, RETN, MMP8 | 0,889 | 0,879 | 0,899 | 0,927 | 0,871 | 0,984 | 0,938 | 0,871 | 1 |
| RSAD2, IFI27, IFIT1, SIGLEC1, IL1R2, OLAH, FAM20A, RETN | 0,889 | 0,880 | 0,898 | 0,928 | 0,871 | 0,984 | 0,948 | 0,886 | 1 |
| IFI27, OAS1, IFI44L, SIGLEC1, IL1R2, OLAH, FAM20A, RETN | 0,889 | 0,880 | 0,898 | 0,926 | 0,869 | 0,982 | 0,952 | 0,892 | 1 |
| RSAD2, IFI27, OAS1, IFI44L, IL1R2, OLAH, FAM20A, MMP8 | 0,889 | 0,879 | 0,899 | 0,928 | 0,874 | 0,983 | 0,929 | 0,857 | 1 |
| RSAD2, IFI27, SIGLEC1, ISG15, IL1R2, OLAH, FAM20A, RETN | 0,889 | 0,880 | 0,898 | 0,926 | 0,869 | 0,983 | 0,946 | 0,882 | 1 |
| RSAD2, IFI27, IFIT1, ISG15, HERC6, OLAH, FAM20A, MMP8 | 0,889 | 0,879 | 0,899 | 0,928 | 0,874 | 0,982 | 0,934 | 0,866 | 1 |
| IFI27, IFI44L, HERC6, IL1R2, OLAH, FAM20A, RETN, MMP8 | 0,889 | 0,879 | 0,899 | 0,932 | 0,879 | 0,985 | 0,934 | 0,861 | 1 |
| IFI27, IFIT1, ISG15, HERC6, OLAH, FAM20A, RETN, MMP8 | 0,889 | 0,879 | 0,899 | 0,928 | 0,871 | 0,984 | 0,936 | 0,867 | 1 |
| IFI27, OAS1, IFI44L, HERC6, OLAH, FAM20A, RETN, MMP8 | 0,889 | 0,879 | 0,899 | 0,931 | 0,877 | 0,986 | 0,932 | 0,859 | 1 |
| IFI27, OAS1, IFI44L, SIGLEC1, ISG15, OLAH, FAM20A, RETN | 0,889 | 0,880 | 0,898 | 0,924 | 0,865 | 0,982 | 0,948 | 0,888 | 1 |
| RSAD2, IFI27, IFIT1, SIGLEC1, SLC1A2, IL1R2, FAM20A, MMP8 | 0,889 | 0,880 | 0,897 | 0,936 | 0,891 | 0,981 | 0,953 | 0,885 | 1 |
| RSAD2, IFI27, SIGLEC1, ISG15, HERC6, IL1R2, FAM20A, RETN | 0,889 | 0,879 | 0,898 | 0,931 | 0,879 | 0,983 | 0,946 | 0,879 | 1 |
| IFI27, SIGLEC1, HERC6, SLC1A2, IL1R2, OLAH, FAM20A, MMP8 | 0,889 | 0,879 | 0,898 | 0,935 | 0,886 | 0,983 | 0,949 | 0,883 | 1 |
| IFI27, OAS1, IFI44L, SIGLEC1, OLAH, FAM20A, RETN, MMP8 | 0,889 | 0,879 | 0,899 | 0,927 | 0,870 | 0,985 | 0,957 | 0,902 | 1 |
| IFI27, IFIT1, IFI44L, HERC6, OLAH, FAM20A, RETN, MMP8 | 0,889 | 0,879 | 0,899 | 0,928 | 0,872 | 0,985 | 0,938 | 0,871 | 1 |
| RSAD2, IFI27, SIGLEC1, ISG15, SLC1A2, IL1R2, FAM20A, MMP8 | 0,889 | 0,879 | 0,898 | 0,938 | 0,895 | 0,981 | 0,952 | 0,883 | 1 |
| RSAD2, IFI27, ISG15, HERC6, IL1R2, OLAH, FAM20A, RETN | 0,889 | 0,880 | 0,898 | 0,929 | 0,876 | 0,982 | 0,926 | 0,851 | 1 |
| RSAD2, IFI27, SIGLEC1, SLC1A2, IL1R2, FAM20A, RETN, MMP8 | 0,889 | 0,879 | 0,898 | 0,937 | 0,890 | 0,985 | 0,952 | 0,883 | 1 |
| IFI27, OAS1, SIGLEC1, ISG15, HERC6, SLC1A2, OLAH, FAM20A | 0,889 | 0,879 | 0,899 | 0,934 | 0,885 | 0,984 | 0,936 | 0,866 | 1 |
| RSAD2, IFI27, OAS1, SIGLEC1, ISG15, HERC6, FAM20A, RETN | 0,888 | 0,879 | 0,898 | 0,929 | 0,875 | 0,983 | 0,937 | 0,863 | 1 |
| IFI27, IFIT1, SIGLEC1, ISG15, SLC1A2, IL1R2, FAM20A, MMP8 | 0,888 | 0,880 | 0,897 | 0,938 | 0,896 | 0,981 | 0,954 | 0,886 | 1 |
| IFI27, OAS1, IFIT1, IFI44L, SIGLEC1, ISG15, IL1R2, FAM20A | 0,888 | 0,880 | 0,897 | 0,928 | 0,874 | 0,983 | 0,949 | 0,886 | 1 |

| | | | | | | | | | |
|---|---|---|---|---|---|---|---|---|---|
| IFI27, OAS1, SIGLEC1, SLC1A2, IL1R2, FAM20A, RETN, MMP8 | 0,888 | 0,879 | 0,898 | 0,936 | 0,890 | 0,982 | 0,951 | 0,883 | 1 |
| RSAD2, IFI27, IFIT1, ISG15, HERC6, IL1R2, OLAH, FAM20A | 0,888 | 0,879 | 0,897 | 0,930 | 0,877 | 0,982 | 0,929 | 0,857 | 1 |
| IFI27, OAS1, IFIT1, HERC6, IL1R2, OLAH, FAM20A, RETN | 0,888 | 0,879 | 0,898 | 0,931 | 0,879 | 0,982 | 0,929 | 0,855 | 1 |
| RSAD2, IFI27, OAS1, IFIT1, ISG15, HERC6, OLAH, FAM20A | 0,888 | 0,879 | 0,898 | 0,928 | 0,874 | 0,983 | 0,928 | 0,856 | 1 |
| IFI27, IFI44L, SIGLEC1, ISG15, SLC1A2, IL1R2, OLAH, FAM20A | 0,888 | 0,880 | 0,897 | 0,936 | 0,889 | 0,983 | 0,940 | 0,872 | 1 |
| RSAD2, IFI27, IFI44L, ISG15, IL1R2, OLAH, FAM20A, MMP8 | 0,888 | 0,879 | 0,898 | 0,929 | 0,876 | 0,982 | 0,928 | 0,856 | 1 |
| RSAD2, IFI27, OAS1, IFI44L, SIGLEC1, ISG15, OLAH, FAM20A | 0,888 | 0,879 | 0,897 | 0,924 | 0,865 | 0,983 | 0,947 | 0,886 | 1 |
| IFI27, IFIT1, IFI44L, SIGLEC1, SLC1A2, IL1R2, OLAH, FAM20A | 0,888 | 0,880 | 0,897 | 0,932 | 0,881 | 0,983 | 0,942 | 0,878 | 1 |
| IFI27, SIGLEC1, ISG15, SLC1A2, OLAH, FAM20A, RETN, MMP8 | 0,888 | 0,879 | 0,898 | 0,934 | 0,884 | 0,984 | 0,947 | 0,878 | 1 |
| IFI27, IFIT1, SIGLEC1, ISG15, SLC1A2, IL1R2, OLAH, FAM20A | 0,888 | 0,879 | 0,897 | 0,937 | 0,889 | 0,984 | 0,937 | 0,869 | 1 |
| IFI27, OAS1, IFI44L, SIGLEC1, SLC1A2, IL1R2, FAM20A, MMP8 | 0,888 | 0,879 | 0,897 | 0,936 | 0,890 | 0,981 | 0,949 | 0,882 | 1 |
| IFI27, OAS1, IFI44L, HERC6, IL1R2, OLAH, FAM20A, RETN | 0,888 | 0,879 | 0,897 | 0,931 | 0,879 | 0,983 | 0,930 | 0,858 | 1 |
| IFI27, IFIT1, SIGLEC1, HERC6, SLC1A2, IL1R2, FAM20A, MMP8 | 0,888 | 0,879 | 0,897 | 0,937 | 0,893 | 0,981 | 0,955 | 0,888 | 1 |
| RSAD2, IFI27, IFIT1, IFI44L, ISG15, HERC6, OLAH, FAM20A | 0,888 | 0,878 | 0,898 | 0,930 | 0,875 | 0,984 | 0,927 | 0,856 | 0,998 |
| IFI27, IFI44L, ISG15, HERC6, IL1R2, OLAH, FAM20A, RETN | 0,888 | 0,879 | 0,897 | 0,928 | 0,874 | 0,982 | 0,930 | 0,858 | 1 |
| IFI27, OAS1, IFIT1, SIGLEC1, ISG15, SLC1A2, OLAH, FAM20A | 0,888 | 0,879 | 0,898 | 0,934 | 0,884 | 0,984 | 0,939 | 0,871 | 1 |
| IFI27, OAS1, IFI44L, ISG15, HERC6, OLAH, FAM20A, RETN | 0,888 | 0,879 | 0,898 | 0,930 | 0,877 | 0,983 | 0,934 | 0,864 | 1 |
| RSAD2, IFI27, OAS1, HERC6, IL1R2, OLAH, FAM20A, RETN | 0,888 | 0,879 | 0,897 | 0,931 | 0,879 | 0,982 | 0,929 | 0,855 | 1 |
| RSAD2, IFI27, IFI44L, HERC6, OLAH, FAM20A, RETN, MMP8 | 0,888 | 0,878 | 0,898 | 0,931 | 0,876 | 0,985 | 0,929 | 0,857 | 1 |
| IFI27, IFIT1, IFI44L, IL1R2, OLAH, FAM20A, RETN, MMP8 | 0,888 | 0,879 | 0,897 | 0,929 | 0,875 | 0,983 | 0,933 | 0,863 | 1 |
| RSAD2, IFI27, IFIT1, ISG15, IL1R2, OLAH, FAM20A, MMP8 | 0,888 | 0,879 | 0,897 | 0,929 | 0,876 | 0,982 | 0,935 | 0,866 | 1 |
| RSAD2, IFI27, IFIT1, IFI44L, HERC6, OLAH, FAM20A, RETN | 0,888 | 0,879 | 0,897 | 0,930 | 0,878 | 0,983 | 0,928 | 0,857 | 1,000 |
| IFI27, OAS1, ISG15, HERC6, IL1R2, OLAH, FAM20A, RETN | 0,888 | 0,879 | 0,897 | 0,931 | 0,879 | 0,982 | 0,927 | 0,853 | 1 |
| RSAD2, IFI27, OAS1, SIGLEC1, IL1R2, OLAH, FAM20A, RETN | 0,888 | 0,879 | 0,897 | 0,925 | 0,868 | 0,983 | 0,947 | 0,883 | 1 |
| RSAD2, IFI27, OAS1, ISG15, IL1R2, OLAH, FAM20A, MMP8 | 0,888 | 0,878 | 0,898 | 0,929 | 0,875 | 0,982 | 0,933 | 0,864 | 1 |
| IFI27, OAS1, SIGLEC1, HERC6, SLC1A2, IL1R2, OLAH, FAM20A | 0,888 | 0,878 | 0,898 | 0,931 | 0,881 | 0,982 | 0,936 | 0,867 | 1 |
| RSAD2, IFI27, IFIT1, SIGLEC1, HERC6, SLC1A2, OLAH, FAM20A | 0,888 | 0,878 | 0,898 | 0,935 | 0,887 | 0,983 | 0,938 | 0,872 | 1 |
| RSAD2, IFI27, IFIT1, IFI44L, SIGLEC1, OLAH, FAM20A, RETN | 0,888 | 0,879 | 0,897 | 0,923 | 0,864 | 0,983 | 0,948 | 0,887 | 1 |

| | | | | | | | | |
|---|---|---|---|---|---|---|---|---|
| IFI27, IFIT1, SIGLEC1, HERC6, SLC1A2, IL1R2, OLAH, FAM20A | 0,888 | 0,879 | 0,897 | 0,936 | 0,888 | 0,983 | 0,941 | 0,876 | 1 |
| IFI27, OAS1, IFIT1, HERC6, IL1R2, FAM20A, RETN, MMP8 | 0,888 | 0,878 | 0,897 | 0,930 | 0,877 | 0,983 | 0,928 | 0,847 | 1 |
| RSAD2, IFI27, OAS1, IFIT1, SIGLEC1, OLAH, FAM20A, RETN | 0,888 | 0,879 | 0,897 | 0,924 | 0,865 | 0,983 | 0,951 | 0,891 | 1 |
| RSAD2, IFI27, IFI44L, SIGLEC1, OLAH, FAM20A, RETN, MMP8 | 0,888 | 0,878 | 0,898 | 0,929 | 0,871 | 0,986 | 0,955 | 0,900 | 1 |
| RSAD2, IFI27, IFI44L, SIGLEC1, SLC1A2, IL1R2, FAM20A, MMP8 | 0,888 | 0,879 | 0,897 | 0,935 | 0,890 | 0,981 | 0,953 | 0,885 | 1 |
| IFI27, IFIT1, SIGLEC1, SLC1A2, IL1R2, FAM20A, RETN, MMP8 | 0,888 | 0,879 | 0,897 | 0,939 | 0,893 | 0,985 | 0,954 | 0,886 | 1 |
| IFI27, OAS1, IFIT1, IFI44L, HERC6, IL1R2, FAM20A, MMP8 | 0,888 | 0,879 | 0,897 | 0,929 | 0,878 | 0,980 | 0,928 | 0,844 | 1 |
| RSAD2, IFI27, IFI44L, SIGLEC1, SLC1A2, OLAH, FAM20A, MMP8 | 0,888 | 0,878 | 0,898 | 0,933 | 0,880 | 0,985 | 0,947 | 0,884 | 1 |
| RSAD2, IFI27, IFIT1, IFI44L, SIGLEC1, SLC1A2, OLAH, FAM20A | 0,888 | 0,879 | 0,897 | 0,930 | 0,876 | 0,984 | 0,941 | 0,876 | 1 |
| IFI27, IFIT1, ISG15, IL1R2, OLAH, FAM20A, RETN, MMP8 | 0,888 | 0,878 | 0,897 | 0,927 | 0,871 | 0,982 | 0,934 | 0,864 | 1 |
| IFI27, IFI44L, SIGLEC1, ISG15, HERC6, SLC1A2, OLAH, FAM20A | 0,888 | 0,878 | 0,897 | 0,934 | 0,885 | 0,983 | 0,938 | 0,870 | 1 |
| RSAD2, IFI27, IFIT1, SIGLEC1, ISG15, OLAH, FAM20A, RETN | 0,888 | 0,879 | 0,897 | 0,926 | 0,868 | 0,985 | 0,943 | 0,882 | 1 |
| IFI27, OAS1, IFIT1, SIGLEC1, HERC6, OLAH, RETN, MMP8 | 0,888 | 0,878 | 0,897 | 0,927 | 0,873 | 0,981 | 0,954 | 0,903 | 1 |
| RSAD2, IFI27, HERC6, SLC1A2, IL1R2, OLAH, FAM20A, MMP8 | 0,887 | 0,878 | 0,897 | 0,934 | 0,887 | 0,982 | 0,922 | 0,840 | 1 |
| IFI27, OAS1, IFIT1, SIGLEC1, HERC6, SLC1A2, IL1R2, FAM20A | 0,887 | 0,878 | 0,897 | 0,936 | 0,891 | 0,980 | 0,941 | 0,872 | 1 |
| RSAD2, IFI27, OAS1, SIGLEC1, SLC1A2, IL1R2, FAM20A, MMP8 | 0,887 | 0,878 | 0,897 | 0,935 | 0,889 | 0,982 | 0,949 | 0,882 | 1 |
| RSAD2, IFI27, OAS1, IFIT1, HERC6, OLAH, FAM20A, RETN | 0,887 | 0,878 | 0,897 | 0,931 | 0,880 | 0,983 | 0,933 | 0,862 | 1 |
| IFI27, IFI44L, SIGLEC1, SLC1A2, IL1R2, FAM20A, RETN, MMP8 | 0,887 | 0,878 | 0,896 | 0,935 | 0,886 | 0,983 | 0,955 | 0,887 | 1 |
| IFI27, SIGLEC1, ISG15, HERC6, SLC1A2, IL1R2, FAM20A, MMP8 | 0,887 | 0,878 | 0,897 | 0,937 | 0,894 | 0,981 | 0,955 | 0,887 | 1 |
| IFI27, OAS1, IFI44L, HERC6, IL1R2, FAM20A, RETN, MMP8 | 0,887 | 0,878 | 0,897 | 0,931 | 0,878 | 0,983 | 0,932 | 0,850 | 1 |
| RSAD2, IFI27, OAS1, IFIT1, IFI44L, SIGLEC1, IL1R2, FAM20A | 0,887 | 0,879 | 0,896 | 0,929 | 0,874 | 0,983 | 0,951 | 0,890 | 1 |
| RSAD2, IFI27, OAS1, SIGLEC1, ISG15, OLAH, FAM20A, RETN | 0,887 | 0,878 | 0,897 | 0,924 | 0,865 | 0,983 | 0,945 | 0,883 | 1 |
| IFI27, IFIT1, IFI44L, HERC6, IL1R2, OLAH, FAM20A, RETN | 0,887 | 0,878 | 0,897 | 0,929 | 0,875 | 0,983 | 0,928 | 0,853 | 1 |
| IFI27, OAS1, SIGLEC1, SLC1A2, OLAH, FAM20A, RETN, MMP8 | 0,887 | 0,877 | 0,897 | 0,932 | 0,879 | 0,985 | 0,951 | 0,892 | 1 |
| IFI27, IFIT1, IFI44L, ISG15, SLC1A2, OLAH, FAM20A, MMP8 | 0,887 | 0,878 | 0,897 | 0,928 | 0,875 | 0,981 | 0,924 | 0,847 | 1 |
| IFI27, IFIT1, ISG15, HERC6, IL1R2, OLAH, FAM20A, RETN | 0,887 | 0,878 | 0,896 | 0,927 | 0,874 | 0,981 | 0,928 | 0,854 | 1 |
| IFI27, IFIT1, SIGLEC1, ISG15, SLC1A2, OLAH, FAM20A, RETN | 0,887 | 0,878 | 0,896 | 0,935 | 0,885 | 0,985 | 0,938 | 0,871 | 1 |
| IFI27, OAS1, ISG15, IL1R2, OLAH, FAM20A, RETN, MMP8 | 0,887 | 0,878 | 0,897 | 0,929 | 0,874 | 0,983 | 0,930 | 0,860 | 1 |

| | | | | | | | | |
|---|---|---|---|---|---|---|---|---|
| IFI27, OAS1, IFIT1, SIGLEC1, SLC1A2, IL1R2, OLAH, FAM20A | 0,887 | 0,878 | 0,896 | 0,932 | 0,881 | 0,983 | 0,941 | 0,875 | 1 |
| IFI27, OAS1, IFIT1, SIGLEC1, ISG15, IL1R2, FAM20A, RETN | 0,887 | 0,878 | 0,896 | 0,927 | 0,872 | 0,982 | 0,948 | 0,883 | 1 |
| RSAD2, IFI27, OAS1, IFI44L, HERC6, OLAH, FAM20A, RETN | 0,887 | 0,878 | 0,897 | 0,930 | 0,877 | 0,982 | 0,933 | 0,862 | 1 |
| RSAD2, IFI27, IFIT1, SIGLEC1, ISG15, SLC1A2, OLAH, FAM20A | 0,887 | 0,878 | 0,896 | 0,935 | 0,885 | 0,985 | 0,938 | 0,871 | 1 |
| RSAD2, IFI27, IFIT1, IFI44L, HERC6, IL1R2, FAM20A, MMP8 | 0,887 | 0,878 | 0,896 | 0,928 | 0,876 | 0,979 | 0,932 | 0,850 | 1 |
| IFI27, OAS1, SIGLEC1, ISG15, SLC1A2, IL1R2, FAM20A, MMP8 | 0,887 | 0,878 | 0,897 | 0,937 | 0,893 | 0,980 | 0,948 | 0,879 | 1 |
| RSAD2, IFI27, IFIT1, HERC6, IL1R2, OLAH, FAM20A, RETN | 0,887 | 0,878 | 0,896 | 0,929 | 0,877 | 0,982 | 0,928 | 0,855 | 1 |
| RSAD2, IFI27, OAS1, ISG15, HERC6, OLAH, FAM20A, RETN | 0,887 | 0,878 | 0,896 | 0,931 | 0,878 | 0,983 | 0,935 | 0,866 | 1 |
| RSAD2, IFI27, OAS1, HERC6, SLC1A2, OLAH, FAM20A, MMP8 | 0,887 | 0,877 | 0,897 | 0,930 | 0,879 | 0,980 | 0,922 | 0,841 | 1 |
| IFI27, OAS1, IFI44L, SIGLEC1, ISG15, IL1R2, FAM20A, RETN | 0,887 | 0,878 | 0,896 | 0,930 | 0,877 | 0,983 | 0,948 | 0,883 | 1 |
| IFI27, OAS1, IFI44L, SIGLEC1, ISG15, SLC1A2, OLAH, FAM20A | 0,887 | 0,877 | 0,897 | 0,932 | 0,881 | 0,983 | 0,938 | 0,870 | 1 |
| RSAD2, IFI27, OAS1, IFI44L, SIGLEC1, HERC6, FAM20A, RETN | 0,887 | 0,878 | 0,896 | 0,929 | 0,874 | 0,983 | 0,939 | 0,866 | 1 |
| RSAD2, IFI27, SIGLEC1, SLC1A2, OLAH, FAM20A, RETN, MMP8 | 0,887 | 0,877 | 0,897 | 0,935 | 0,885 | 0,986 | 0,948 | 0,884 | 1 |
| IFI27, OAS1, HERC6, SLC1A2, IL1R2, OLAH, FAM20A, MMP8 | 0,887 | 0,878 | 0,896 | 0,935 | 0,889 | 0,982 | 0,921 | 0,838 | 1 |
| IFI27, OAS1, IFI44L, SIGLEC1, HERC6, SLC1A2, OLAH, FAM20A | 0,887 | 0,877 | 0,897 | 0,932 | 0,882 | 0,983 | 0,936 | 0,867 | 1 |
| IFI27, IFIT1, SIGLEC1, SLC1A2, OLAH, FAM20A, RETN, MMP8 | 0,887 | 0,877 | 0,897 | 0,937 | 0,887 | 0,986 | 0,953 | 0,892 | 1 |
| RSAD2, IFI27, IFI44L, HERC6, IL1R2, OLAH, FAM20A, RETN | 0,887 | 0,877 | 0,897 | 0,929 | 0,876 | 0,982 | 0,929 | 0,857 | 1 |
| IFI27, OAS1, ISG15, HERC6, IL1R2, FAM20A, RETN, MMP8 | 0,887 | 0,878 | 0,896 | 0,933 | 0,882 | 0,984 | 0,926 | 0,843 | 1 |
| RSAD2, IFI27, IFI44L, ISG15, HERC6, OLAH, FAM20A, RETN | 0,887 | 0,877 | 0,896 | 0,930 | 0,878 | 0,983 | 0,935 | 0,867 | 1 |
| RSAD2, IFI27, IFIT1, ISG15, HERC6, OLAH, FAM20A, RETN | 0,887 | 0,877 | 0,896 | 0,929 | 0,876 | 0,982 | 0,934 | 0,865 | 1 |
| IFI27, OAS1, IFIT1, IFI44L, HERC6, IL1R2, FAM20A, RETN | 0,887 | 0,878 | 0,896 | 0,932 | 0,882 | 0,982 | 0,929 | 0,848 | 1 |
| RSAD2, IFI27, SIGLEC1, ISG15, HERC6, SLC1A2, OLAH, FAM20A | 0,887 | 0,877 | 0,897 | 0,931 | 0,880 | 0,982 | 0,935 | 0,866 | 1 |
| IFI27, IFIT1, IFI44L, HERC6, IL1R2, FAM20A, RETN, MMP8 | 0,887 | 0,877 | 0,896 | 0,931 | 0,878 | 0,983 | 0,936 | 0,857 | 1 |
| RSAD2, IFI27, OAS1, IL1R2, OLAH, FAM20A, RETN, MMP8 | 0,887 | 0,877 | 0,896 | 0,929 | 0,876 | 0,983 | 0,932 | 0,861 | 1 |
| RSAD2, IFI27, IFI44L, SIGLEC1, IL1R2, OLAH, FAM20A, RETN | 0,887 | 0,877 | 0,896 | 0,927 | 0,871 | 0,983 | 0,950 | 0,888 | 1 |
| RSAD2, IFI27, SIGLEC1, HERC6, SLC1A2, IL1R2, OLAH, FAM20A | 0,887 | 0,877 | 0,896 | 0,931 | 0,880 | 0,982 | 0,941 | 0,874 | 1 |
| IFI27, OAS1, SIGLEC1, HERC6, IL1R2, OLAH, RETN, MMP8 | 0,887 | 0,877 | 0,896 | 0,927 | 0,873 | 0,981 | 0,955 | 0,905 | 1 |
| IFI27, SIGLEC1, ISG15, SLC1A2, IL1R2, FAM20A, RETN, MMP8 | 0,887 | 0,878 | 0,896 | 0,935 | 0,888 | 0,983 | 0,953 | 0,881 | 1 |

| | | | | | | | | |
|---|---|---|---|---|---|---|---|---|
| RSAD2, IFI27, OAS1, HERC6, IL1R2, FAM20A, RETN, MMP8 | 0,887 | 0,877 | 0,896 | 0,932 | 0,881 | 0,984 | 0,929 | 0,846 | 1 |
| IFI27, IFI44L, SIGLEC1, HERC6, SLC1A2, IL1R2, FAM20A, MMP8 | 0,887 | 0,877 | 0,896 | 0,934 | 0,887 | 0,980 | 0,954 | 0,886 | 1 |
| RSAD2, IFI27, IFIT1, IFI44L, SIGLEC1, ISG15, IL1R2, FAM20A | 0,887 | 0,878 | 0,895 | 0,927 | 0,873 | 0,982 | 0,948 | 0,883 | 1 |
| RSAD2, IFI27, OAS1, SIGLEC1, HERC6, SLC1A2, OLAH, FAM20A | 0,887 | 0,877 | 0,896 | 0,931 | 0,880 | 0,983 | 0,935 | 0,866 | 1 |
| RSAD2, IFI27, OAS1, IFI44L, HERC6, IL1R2, FAM20A, MMP8 | 0,887 | 0,877 | 0,896 | 0,930 | 0,879 | 0,980 | 0,926 | 0,840 | 1 |
| RSAD2, IFI27, OAS1, IFI44L, SIGLEC1, ISG15, IL1R2, FAM20A | 0,887 | 0,878 | 0,895 | 0,927 | 0,873 | 0,982 | 0,949 | 0,884 | 1 |
| IFI27, IFI44L, SIGLEC1, HERC6, SLC1A2, OLAH, FAM20A, MMP8 | 0,886 | 0,877 | 0,896 | 0,928 | 0,874 | 0,982 | 0,953 | 0,892 | 1 |
| RSAD2, IFI27, IFI44L, SIGLEC1, ISG15, OLAH, FAM20A, RETN | 0,886 | 0,877 | 0,896 | 0,924 | 0,865 | 0,982 | 0,945 | 0,882 | 1 |
| IFI27, IFIT1, IFI44L, SIGLEC1, ISG15, FAM20A, RETN, MMP8 | 0,886 | 0,877 | 0,896 | 0,923 | 0,864 | 0,983 | 0,946 | 0,872 | 1 |
| IFI27, OAS1, IFIT1, IFI44L, HERC6, SLC1A2, OLAH, FAM20A | 0,886 | 0,877 | 0,896 | 0,932 | 0,882 | 0,982 | 0,921 | 0,838 | 1 |
| RSAD2, IFI27, IFI44L, SIGLEC1, HERC6, FAM20A, RETN, MMP8 | 0,886 | 0,877 | 0,896 | 0,930 | 0,875 | 0,984 | 0,946 | 0,872 | 1 |
| IFI27, OAS1, IFIT1, IL1R2, OLAH, FAM20A, RETN, MMP8 | 0,886 | 0,877 | 0,896 | 0,929 | 0,874 | 0,983 | 0,932 | 0,861 | 1 |
| IFI27, OAS1, IFIT1, IFI44L, SIGLEC1, ISG15, HERC6, RETN | 0,886 | 0,878 | 0,895 | 0,920 | 0,863 | 0,977 | 0,941 | 0,883 | 1,000 |
| IFI27, OAS1, SIGLEC1, ISG15, SLC1A2, IL1R2, OLAH, FAM20A | 0,886 | 0,877 | 0,896 | 0,933 | 0,883 | 0,983 | 0,939 | 0,871 | 1 |
| RSAD2, IFI27, OAS1, SIGLEC1, ISG15, SLC1A2, OLAH, FAM20A | 0,886 | 0,877 | 0,896 | 0,932 | 0,880 | 0,984 | 0,940 | 0,873 | 1 |
| RSAD2, IFI27, IFIT1, IL1R2, OLAH, FAM20A, RETN, MMP8 | 0,886 | 0,877 | 0,896 | 0,930 | 0,876 | 0,984 | 0,932 | 0,861 | 1 |
| IFI27, OAS1, SIGLEC1, HERC6, SLC1A2, OLAH, FAM20A, RETN | 0,886 | 0,876 | 0,896 | 0,931 | 0,881 | 0,982 | 0,935 | 0,864 | 1 |
| IFI27, IFI44L, ISG15, HERC6, IL1R2, FAM20A, RETN, MMP8 | 0,886 | 0,876 | 0,896 | 0,931 | 0,878 | 0,983 | 0,936 | 0,857 | 1 |
| IFI27, OAS1, IFI44L, ISG15, HERC6, IL1R2, FAM20A, MMP8 | 0,886 | 0,877 | 0,896 | 0,930 | 0,879 | 0,980 | 0,926 | 0,840 | 1 |
| RSAD2, IFI27, OAS1, IFIT1, HERC6, IL1R2, FAM20A, MMP8 | 0,886 | 0,877 | 0,895 | 0,930 | 0,880 | 0,980 | 0,930 | 0,850 | 1 |
| IFI27, OAS1, IFI44L, SIGLEC1, HERC6, OLAH, RETN, MMP8 | 0,886 | 0,877 | 0,895 | 0,926 | 0,872 | 0,980 | 0,950 | 0,897 | 1 |
| IFI27, OAS1, ISG15, HERC6, SLC1A2, OLAH, FAM20A, MMP8 | 0,886 | 0,876 | 0,896 | 0,931 | 0,882 | 0,981 | 0,918 | 0,836 | 1 |
| IFI27, IFIT1, IFI44L, SIGLEC1, SLC1A2, OLAH, FAM20A, RETN | 0,886 | 0,877 | 0,895 | 0,930 | 0,876 | 0,984 | 0,942 | 0,877 | 1 |
| IFI27, IFIT1, IFI44L, SIGLEC1, HERC6, SLC1A2, IL1R2, FAM20A | 0,886 | 0,877 | 0,895 | 0,936 | 0,890 | 0,981 | 0,946 | 0,876 | 1 |
| RSAD2, IFI27, OAS1, IFIT1, SIGLEC1, SLC1A2, OLAH, FAM20A | 0,886 | 0,877 | 0,895 | 0,932 | 0,878 | 0,985 | 0,943 | 0,878 | 1 |
| RSAD2, IFI27, OAS1, IFIT1, SIGLEC1, IL1R2, FAM20A, RETN | 0,886 | 0,877 | 0,895 | 0,927 | 0,871 | 0,982 | 0,950 | 0,888 | 1 |
| IFI27, OAS1, IFIT1, IFI44L, SIGLEC1, HERC6, OLAH, RETN | 0,886 | 0,878 | 0,895 | 0,924 | 0,872 | 0,977 | 0,940 | 0,882 | 0,998 |
| IFI27, SIGLEC1, SLC1A2, IL1R2, OLAH, FAM20A, RETN, MMP8 | 0,886 | 0,877 | 0,896 | 0,934 | 0,885 | 0,984 | 0,948 | 0,880 | 1 |

| Genes | | | | | | | | | |
|---|---|---|---|---|---|---|---|---|---|
| IFI27, OAS1, IFIT1, IFI44L, SIGLEC1, ISG15, FAM20A, RETN | 0,886 | 0,877 | 0,895 | 0,920 | 0,860 | 0,980 | 0,943 | 0,874 | 1 |
| RSAD2, IFI27, OAS1, IFIT1, IFI44L, OLAH, FAM20A, MMP8 | 0,886 | 0,876 | 0,896 | 0,924 | 0,865 | 0,982 | 0,938 | 0,874 | 1 |
| RSAD2, IFI27, IFI44L, SIGLEC1, ISG15, SLC1A2, OLAH, FAM20A | 0,886 | 0,877 | 0,895 | 0,931 | 0,879 | 0,983 | 0,938 | 0,869 | 1 |
| IFI27, IFI44L, SIGLEC1, ISG15, SLC1A2, OLAH, FAM20A, RETN | 0,886 | 0,877 | 0,895 | 0,932 | 0,881 | 0,983 | 0,939 | 0,871 | 1 |
| IFI27, IFIT1, ISG15, SLC1A2, IL1R2, OLAH, FAM20A, MMP8 | 0,886 | 0,877 | 0,895 | 0,934 | 0,886 | 0,981 | 0,926 | 0,849 | 1 |
| RSAD2, IFI27, OAS1, IFIT1, SIGLEC1, ISG15, IL1R2, FAM20A | 0,886 | 0,877 | 0,895 | 0,927 | 0,873 | 0,982 | 0,950 | 0,887 | 1 |
| IFI27, OAS1, SIGLEC1, ISG15, SLC1A2, OLAH, FAM20A, RETN | 0,886 | 0,876 | 0,895 | 0,933 | 0,881 | 0,985 | 0,937 | 0,869 | 1 |
| RSAD2, IFI27, OAS1, IFIT1, IFI44L, HERC6, IL1R2, FAM20A | 0,886 | 0,877 | 0,895 | 0,927 | 0,875 | 0,978 | 0,926 | 0,843 | 1 |
| RSAD2, IFI27, OAS1, SIGLEC1, ISG15, IL1R2, FAM20A, RETN | 0,886 | 0,876 | 0,895 | 0,928 | 0,872 | 0,983 | 0,949 | 0,882 | 1 |
| IFI27, IFI44L, ISG15, HERC6, SLC1A2, OLAH, FAM20A, MMP8 | 0,886 | 0,876 | 0,896 | 0,931 | 0,880 | 0,983 | 0,922 | 0,841 | 1 |
| RSAD2, IFI27, ISG15, HERC6, IL1R2, FAM20A, RETN, MMP8 | 0,886 | 0,877 | 0,895 | 0,932 | 0,881 | 0,983 | 0,930 | 0,850 | 1 |
| IFI27, IFIT1, IFI44L, SLC1A2, IL1R2, OLAH, FAM20A, MMP8 | 0,886 | 0,877 | 0,895 | 0,932 | 0,882 | 0,982 | 0,924 | 0,844 | 1 |
| RSAD2, IFI27, IFIT1, SIGLEC1, SLC1A2, IL1R2, OLAH, FAM20A | 0,886 | 0,877 | 0,895 | 0,933 | 0,882 | 0,983 | 0,943 | 0,879 | 1 |
| IFI27, OAS1, IFI44L, ISG15, HERC6, IL1R2, FAM20A, RETN | 0,886 | 0,877 | 0,895 | 0,932 | 0,881 | 0,982 | 0,927 | 0,844 | 1 |
| RSAD2, IFI27, OAS1, SIGLEC1, HERC6, OLAH, RETN, MMP8 | 0,886 | 0,877 | 0,895 | 0,925 | 0,870 | 0,980 | 0,954 | 0,903 | 1 |
| RSAD2, IFI27, IFIT1, HERC6, IL1R2, FAM20A, RETN, MMP8 | 0,886 | 0,876 | 0,895 | 0,931 | 0,878 | 0,983 | 0,935 | 0,857 | 1 |
| IFI27, OAS1, IFIT1, HERC6, SLC1A2, OLAH, FAM20A, MMP8 | 0,886 | 0,876 | 0,896 | 0,929 | 0,877 | 0,981 | 0,923 | 0,843 | 1 |
| RSAD2, IFI27, OAS1, IFIT1, ISG15, OLAH, FAM20A, MMP8 | 0,886 | 0,876 | 0,895 | 0,922 | 0,863 | 0,982 | 0,936 | 0,871 | 1 |
| RSAD2, IFI27, ISG15, HERC6, SLC1A2, OLAH, FAM20A, MMP8 | 0,886 | 0,876 | 0,896 | 0,931 | 0,881 | 0,981 | 0,918 | 0,835 | 1 |
| RSAD2, IFI27, ISG15, IL1R2, OLAH, FAM20A, RETN, MMP8 | 0,886 | 0,876 | 0,896 | 0,928 | 0,873 | 0,982 | 0,930 | 0,858 | 1 |
| RSAD2, IFI27, IFI44L, ISG15, HERC6, IL1R2, FAM20A, MMP8 | 0,886 | 0,876 | 0,895 | 0,928 | 0,876 | 0,979 | 0,926 | 0,840 | 1 |
| IFI27, IFIT1, IFI44L, ISG15, OLAH, FAM20A, RETN, MMP8 | 0,886 | 0,876 | 0,896 | 0,922 | 0,862 | 0,982 | 0,941 | 0,875 | 1 |
| IFI27, OAS1, IFIT1, SIGLEC1, HERC6, IL1R2, RETN, MMP8 | 0,886 | 0,877 | 0,894 | 0,925 | 0,872 | 0,978 | 0,958 | 0,910 | 1 |
| IFI27, OAS1, IFIT1, IFI44L, ISG15, OLAH, FAM20A, MMP8 | 0,886 | 0,876 | 0,896 | 0,921 | 0,861 | 0,981 | 0,936 | 0,870 | 1 |
| RSAD2, IFI27, IFI44L, SIGLEC1, ISG15, HERC6, FAM20A, RETN | 0,886 | 0,876 | 0,895 | 0,928 | 0,874 | 0,982 | 0,939 | 0,864 | 1 |
| OAS1, IFIT1, SIGLEC1, HERC6, IL1R2, OLAH, FAM20A, MMP8 | 0,886 | 0,877 | 0,895 | 0,924 | 0,870 | 0,978 | 0,962 | 0,906 | 1 |
| IFI27, IFIT1, SIGLEC1, ISG15, HERC6, SLC1A2, IL1R2, FAM20A | 0,886 | 0,876 | 0,895 | 0,936 | 0,890 | 0,981 | 0,948 | 0,879 | 1 |
| RSAD2, IFI27, HERC6, SLC1A2, OLAH, FAM20A, RETN, MMP8 | 0,886 | 0,876 | 0,895 | 0,933 | 0,884 | 0,983 | 0,924 | 0,844 | 1 |

| | | | | | | | | | |
|---|---|---|---|---|---|---|---|---|---|
| IFI27, OAS1, IFIT1, IFI44L, ISG15, HERC6, IL1R2, FAM20A | 0,886 | 0,877 | 0,894 | 0,928 | 0,877 | 0,979 | 0,925 | 0,841 | 1 |
| RSAD2, IFI27, SIGLEC1, ISG15, SLC1A2, IL1R2, OLAH, FAM20A | 0,886 | 0,876 | 0,895 | 0,934 | 0,885 | 0,983 | 0,938 | 0,869 | 1 |
| IFI27, IFI44L, SIGLEC1, SLC1A2, OLAH, FAM20A, RETN, MMP8 | 0,886 | 0,876 | 0,895 | 0,931 | 0,878 | 0,984 | 0,949 | 0,883 | 1 |
| RSAD2, IFI27, OAS1, IFI44L, ISG15, HERC6, IL1R2, FAM20A | 0,886 | 0,877 | 0,894 | 0,927 | 0,876 | 0,979 | 0,925 | 0,840 | 1 |
| IFI27, IFIT1, SIGLEC1, SLC1A2, IL1R2, OLAH, FAM20A, RETN | 0,885 | 0,876 | 0,895 | 0,933 | 0,883 | 0,983 | 0,943 | 0,879 | 1 |
| IFI27, OAS1, IFIT1, SIGLEC1, HERC6, IL1R2, OLAH, RETN | 0,885 | 0,877 | 0,894 | 0,924 | 0,870 | 0,978 | 0,946 | 0,892 | 1,000 |
| IFI27, OAS1, IFIT1, IFI44L, ISG15, HERC6, FAM20A, RETN | 0,885 | 0,876 | 0,895 | 0,923 | 0,866 | 0,981 | 0,929 | 0,850 | 1 |
| IFI27, OAS1, SIGLEC1, ISG15, HERC6, OLAH, RETN, MMP8 | 0,885 | 0,876 | 0,895 | 0,926 | 0,871 | 0,981 | 0,957 | 0,905 | 1 |
| RSAD2, IFI27, IFI44L, SIGLEC1, HERC6, SLC1A2, OLAH, FAM20A | 0,885 | 0,876 | 0,895 | 0,931 | 0,880 | 0,983 | 0,939 | 0,871 | 1 |
| IFI27, IFIT1, IFI44L, SIGLEC1, ISG15, SLC1A2, IL1R2, FAM20A | 0,885 | 0,877 | 0,894 | 0,933 | 0,885 | 0,980 | 0,943 | 0,874 | 1 |
| OAS1, IFIT1, IFI44L, SIGLEC1, HERC6, IL1R2, FAM20A, MMP8 | 0,885 | 0,877 | 0,894 | 0,924 | 0,870 | 0,978 | 0,962 | 0,906 | 1 |
| IFI27, IFIT1, IFI44L, SIGLEC1, HERC6, OLAH, RETN, MMP8 | 0,885 | 0,876 | 0,894 | 0,926 | 0,872 | 0,981 | 0,955 | 0,907 | 1 |
| IFI27, OAS1, IFIT1, SIGLEC1, ISG15, HERC6, OLAH, RETN | 0,885 | 0,876 | 0,894 | 0,925 | 0,872 | 0,978 | 0,947 | 0,892 | 1 |
| RSAD2, IFI27, OAS1, IFI44L, SIGLEC1, OLAH, FAM20A, RETN | 0,885 | 0,876 | 0,895 | 0,922 | 0,862 | 0,982 | 0,951 | 0,893 | 1 |
| IFI27, OAS1, IFIT1, SIGLEC1, SLC1A2, OLAH, FAM20A, RETN | 0,885 | 0,876 | 0,895 | 0,931 | 0,878 | 0,984 | 0,942 | 0,878 | 1 |
| RSAD2, IFI27, IFIT1, IFI44L, SIGLEC1, IL1R2, FAM20A, RETN | 0,885 | 0,876 | 0,895 | 0,927 | 0,871 | 0,982 | 0,949 | 0,886 | 1 |
| RSAD2, IFI27, OAS1, IFI44L, ISG15, OLAH, FAM20A, MMP8 | 0,885 | 0,875 | 0,896 | 0,923 | 0,865 | 0,982 | 0,936 | 0,870 | 1 |
| IFI27, OAS1, IFIT1, ISG15, HERC6, IL1R2, FAM20A, RETN | 0,885 | 0,876 | 0,894 | 0,932 | 0,881 | 0,982 | 0,927 | 0,845 | 1 |
| RSAD2, IFI27, OAS1, HERC6, SLC1A2, IL1R2, OLAH, FAM20A | 0,885 | 0,876 | 0,895 | 0,934 | 0,886 | 0,981 | 0,918 | 0,833 | 1 |
| IFI27, SIGLEC1, ISG15, HERC6, SLC1A2, IL1R2, OLAH, FAM20A | 0,885 | 0,876 | 0,895 | 0,933 | 0,885 | 0,982 | 0,938 | 0,870 | 1 |
| IFI27, ISG15, HERC6, SLC1A2, IL1R2, OLAH, FAM20A, MMP8 | 0,885 | 0,876 | 0,895 | 0,935 | 0,889 | 0,981 | 0,922 | 0,841 | 1 |
| RSAD2, IFI27, IFIT1, SIGLEC1, ISG15, IL1R2, FAM20A, RETN | 0,885 | 0,876 | 0,894 | 0,928 | 0,873 | 0,983 | 0,949 | 0,884 | 1 |
| IFI27, IFI44L, ISG15, SLC1A2, OLAH, FAM20A, RETN, MMP8 | 0,885 | 0,875 | 0,895 | 0,930 | 0,877 | 0,983 | 0,924 | 0,844 | 1 |
| IFI27, IFIT1, IFI44L, HERC6, SLC1A2, OLAH, FAM20A, MMP8 | 0,885 | 0,876 | 0,895 | 0,931 | 0,880 | 0,982 | 0,925 | 0,844 | 1 |
| RSAD2, IFI27, IFIT1, SLC1A2, IL1R2, OLAH, FAM20A, MMP8 | 0,885 | 0,876 | 0,894 | 0,931 | 0,882 | 0,981 | 0,924 | 0,845 | 1 |
| IFI27, IFIT1, SIGLEC1, HERC6, SLC1A2, OLAH, FAM20A, RETN | 0,885 | 0,875 | 0,895 | 0,936 | 0,887 | 0,985 | 0,942 | 0,877 | 1 |
| IFI27, IFI44L, SIGLEC1, ISG15, HERC6, FAM20A, RETN, MMP8 | 0,885 | 0,875 | 0,895 | 0,926 | 0,868 | 0,984 | 0,949 | 0,876 | 1 |
| IFI27, OAS1, IFI44L, SIGLEC1, HERC6, SLC1A2, IL1R2, FAM20A | 0,885 | 0,876 | 0,895 | 0,934 | 0,889 | 0,980 | 0,943 | 0,874 | 1 |

| | | | | | | | | |
|---|---|---|---|---|---|---|---|---|
| RSAD2, IFI27, SIGLEC1, HERC6, IL1R2, OLAH, RETN, MMP8 | 0,885 | 0,876 | 0,894 | 0,925 | 0,871 | 0,979 | 0,954 | 0,905 | 1 |
| IFI27, IFI44L, SIGLEC1, HERC6, SLC1A2, IL1R2, OLAH, FAM20A | 0,885 | 0,876 | 0,895 | 0,933 | 0,882 | 0,983 | 0,947 | 0,881 | 1 |
| RSAD2, IFI27, IFI44L, ISG15, SLC1A2, OLAH, FAM20A, MMP8 | 0,885 | 0,875 | 0,895 | 0,931 | 0,878 | 0,983 | 0,924 | 0,848 | 1,000 |
| IFI27, SIGLEC1, ISG15, SLC1A2, IL1R2, OLAH, FAM20A, RETN | 0,885 | 0,876 | 0,894 | 0,933 | 0,884 | 0,982 | 0,938 | 0,870 | 1 |
| IFI27, IFI44L, SIGLEC1, HERC6, IL1R2, OLAH, RETN, MMP8 | 0,885 | 0,876 | 0,894 | 0,926 | 0,872 | 0,979 | 0,953 | 0,904 | 1 |
| IFI27, OAS1, IFIT1, ISG15, HERC6, IL1R2, FAM20A, MMP8 | 0,885 | 0,876 | 0,894 | 0,929 | 0,878 | 0,980 | 0,929 | 0,848 | 1 |
| IFI27, OAS1, IFIT1, SIGLEC1, HERC6, SLC1A2, FAM20A, RETN | 0,885 | 0,876 | 0,894 | 0,931 | 0,882 | 0,981 | 0,937 | 0,866 | 1 |
| IFI27, OAS1, IFIT1, SIGLEC1, ISG15, FAM20A, RETN, MMP8 | 0,885 | 0,875 | 0,895 | 0,923 | 0,863 | 0,982 | 0,946 | 0,875 | 1 |
| IFI27, OAS1, IFI44L, ISG15, HERC6, SLC1A2, OLAH, FAM20A | 0,885 | 0,875 | 0,895 | 0,933 | 0,884 | 0,982 | 0,918 | 0,836 | 1 |
| IFI27, OAS1, IFI44L, HERC6, SLC1A2, OLAH, FAM20A, MMP8 | 0,885 | 0,875 | 0,895 | 0,930 | 0,878 | 0,981 | 0,923 | 0,843 | 1 |
| RSAD2, IFI27, ISG15, SLC1A2, IL1R2, OLAH, FAM20A, MMP8 | 0,885 | 0,876 | 0,894 | 0,936 | 0,890 | 0,982 | 0,917 | 0,834 | 1 |
| IFI27, IFI44L, SLC1A2, IL1R2, OLAH, FAM20A, RETN, MMP8 | 0,885 | 0,875 | 0,894 | 0,935 | 0,886 | 0,984 | 0,925 | 0,846 | 1 |
| RSAD2, IFI27, IFI44L, SLC1A2, IL1R2, OLAH, FAM20A, MMP8 | 0,885 | 0,875 | 0,895 | 0,933 | 0,883 | 0,983 | 0,928 | 0,853 | 1 |
| IFI27, IFIT1, IFI44L, SIGLEC1, ISG15, HERC6, OLAH, RETN | 0,885 | 0,876 | 0,894 | 0,924 | 0,870 | 0,978 | 0,946 | 0,893 | 0,998 |
| IFI27, OAS1, HERC6, SLC1A2, OLAH, FAM20A, RETN, MMP8 | 0,885 | 0,875 | 0,895 | 0,933 | 0,884 | 0,982 | 0,921 | 0,837 | 1 |
| RSAD2, IFI27, IFI44L, SIGLEC1, ISG15, IL1R2, FAM20A, RETN | 0,885 | 0,875 | 0,894 | 0,928 | 0,874 | 0,982 | 0,947 | 0,880 | 1 |
| RSAD2, IFI27, IFI44L, IL1R2, OLAH, FAM20A, RETN, MMP8 | 0,885 | 0,875 | 0,895 | 0,930 | 0,877 | 0,984 | 0,930 | 0,859 | 1 |
| IFI27, OAS1, IFIT1, IFI44L, SIGLEC1, SLC1A2, IL1R2, FAM20A | 0,885 | 0,876 | 0,894 | 0,933 | 0,885 | 0,981 | 0,945 | 0,877 | 1 |
| RSAD2, IFI27, OAS1, IFI44L, SIGLEC1, IL1R2, FAM20A, RETN | 0,885 | 0,875 | 0,894 | 0,928 | 0,875 | 0,982 | 0,948 | 0,883 | 1 |
| IFI27, OAS1, IFIT1, IFI44L, SIGLEC1, FAM20A, RETN, MMP8 | 0,885 | 0,875 | 0,895 | 0,923 | 0,863 | 0,982 | 0,948 | 0,881 | 1 |
| IFI27, IFI44L, SIGLEC1, ISG15, HERC6, OLAH, RETN, MMP8 | 0,885 | 0,876 | 0,894 | 0,924 | 0,868 | 0,979 | 0,949 | 0,896 | 1 |
| RSAD2, IFI27, IFIT1, IFI44L, ISG15, HERC6, IL1R2, FAM20A | 0,885 | 0,876 | 0,894 | 0,927 | 0,876 | 0,978 | 0,929 | 0,847 | 1 |
| RSAD2, IFI27, OAS1, SIGLEC1, SLC1A2, IL1R2, OLAH, FAM20A | 0,885 | 0,875 | 0,894 | 0,930 | 0,878 | 0,982 | 0,941 | 0,876 | 1 |
| RSAD2, IFI27, OAS1, IFIT1, SIGLEC1, ISG15, HERC6, RETN | 0,885 | 0,876 | 0,893 | 0,920 | 0,862 | 0,978 | 0,943 | 0,885 | 1 |
| IFI27, OAS1, IFIT1, SIGLEC1, ISG15, HERC6, RETN, MMP8 | 0,885 | 0,876 | 0,893 | 0,920 | 0,863 | 0,978 | 0,945 | 0,887 | 1 |
| RSAD2, IFI27, OAS1, ISG15, HERC6, IL1R2, FAM20A, MMP8 | 0,885 | 0,876 | 0,894 | 0,931 | 0,881 | 0,980 | 0,923 | 0,835 | 1 |
| IFI27, IFIT1, IFI44L, ISG15, HERC6, IL1R2, FAM20A, MMP8 | 0,885 | 0,875 | 0,894 | 0,930 | 0,879 | 0,980 | 0,935 | 0,855 | 1 |
| RSAD2, IFI27, IFIT1, IFI44L, SIGLEC1, ISG15, HERC6, FAM20A | 0,885 | 0,876 | 0,893 | 0,922 | 0,863 | 0,981 | 0,942 | 0,872 | 1 |

503

| | | | | | | | | | |
|---|---|---|---|---|---|---|---|---|---|
| RSAD2, IFI27, IFI44L, HERC6, SLC1A2, OLAH, FAM20A, MMP8 | 0,885 | 0,875 | 0,895 | 0,933 | 0,884 | 0,983 | 0,925 | 0,848 | 1 |
| RSAD2, IFI27, IFIT1, SIGLEC1, HERC6, SLC1A2, IL1R2, FAM20A | 0,885 | 0,875 | 0,894 | 0,935 | 0,890 | 0,981 | 0,946 | 0,876 | 1 |
| IFI27, IFIT1, IFI44L, SIGLEC1, ISG15, HERC6, FAM20A, MMP8 | 0,885 | 0,876 | 0,894 | 0,920 | 0,861 | 0,980 | 0,937 | 0,862 | 1 |
| IFI27, OAS1, IFI44L, IL1R2, OLAH, FAM20A, RETN, MMP8 | 0,885 | 0,875 | 0,895 | 0,931 | 0,877 | 0,984 | 0,934 | 0,864 | 1 |
| IFI27, OAS1, IFI44L, ISG15, SLC1A2, OLAH, FAM20A, MMP8 | 0,885 | 0,874 | 0,895 | 0,930 | 0,877 | 0,983 | 0,924 | 0,847 | 1 |
| RSAD2, IFI27, IFIT1, SIGLEC1, HERC6, OLAH, RETN, MMP8 | 0,885 | 0,876 | 0,894 | 0,924 | 0,869 | 0,980 | 0,951 | 0,900 | 1 |
| IFI27, OAS1, IFIT1, SLC1A2, IL1R2, OLAH, FAM20A, MMP8 | 0,885 | 0,875 | 0,894 | 0,931 | 0,881 | 0,982 | 0,924 | 0,845 | 1 |
| IFI27, OAS1, IFIT1, HERC6, IL1R2, OLAH, RETN, MMP8 | 0,885 | 0,876 | 0,894 | 0,924 | 0,868 | 0,979 | 0,945 | 0,885 | 1 |
| RSAD2, IFI27, OAS1, SLC1A2, IL1R2, OLAH, FAM20A, MMP8 | 0,885 | 0,876 | 0,894 | 0,930 | 0,881 | 0,980 | 0,924 | 0,844 | 1 |
| RSAD2, IFI27, IFI44L, HERC6, IL1R2, FAM20A, RETN, MMP8 | 0,885 | 0,874 | 0,895 | 0,929 | 0,877 | 0,982 | 0,935 | 0,856 | 1 |
| RSAD2, IFI27, IFI44L, SIGLEC1, HERC6, OLAH, RETN, MMP8 | 0,885 | 0,876 | 0,894 | 0,926 | 0,871 | 0,980 | 0,952 | 0,901 | 1 |
| IFIT1, IFI44L, SIGLEC1, ISG15, HERC6, IL1R2, FAM20A, MMP8 | 0,885 | 0,876 | 0,894 | 0,923 | 0,868 | 0,978 | 0,963 | 0,907 | 1 |
| IFI27, OAS1, IFI44L, SIGLEC1, SLC1A2, IL1R2, OLAH, FAM20A | 0,885 | 0,875 | 0,894 | 0,930 | 0,877 | 0,982 | 0,943 | 0,879 | 1 |
| IFI27, OAS1, IFIT1, IFI44L, SLC1A2, OLAH, FAM20A, MMP8 | 0,885 | 0,875 | 0,894 | 0,932 | 0,879 | 0,985 | 0,925 | 0,848 | 1 |
| IFI27, OAS1, IFIT1, IFI44L, ISG15, IL1R2, OLAH, FAM20A | 0,885 | 0,876 | 0,893 | 0,924 | 0,868 | 0,981 | 0,933 | 0,863 | 1 |
| IFI27, IFIT1, HERC6, SLC1A2, IL1R2, OLAH, FAM20A, MMP8 | 0,885 | 0,875 | 0,894 | 0,933 | 0,884 | 0,981 | 0,924 | 0,843 | 1 |
| IFI27, OAS1, SIGLEC1, ISG15, HERC6, SLC1A2, IL1R2, FAM20A | 0,885 | 0,875 | 0,894 | 0,935 | 0,889 | 0,980 | 0,942 | 0,873 | 1 |
| RSAD2, IFI27, OAS1, SIGLEC1, HERC6, SLC1A2, IL1R2, FAM20A | 0,885 | 0,875 | 0,894 | 0,933 | 0,886 | 0,979 | 0,943 | 0,874 | 1 |
| RSAD2, IFI27, OAS1, ISG15, HERC6, SLC1A2, OLAH, FAM20A | 0,885 | 0,875 | 0,894 | 0,932 | 0,883 | 0,981 | 0,915 | 0,829 | 1 |
| IFI27, OAS1, IFIT1, IFI44L, SIGLEC1, ISG15, HERC6, FAM20A | 0,885 | 0,876 | 0,893 | 0,922 | 0,865 | 0,980 | 0,939 | 0,868 | 1 |
| IFIT1, IFI44L, SIGLEC1, HERC6, IL1R2, OLAH, FAM20A, MMP8 | 0,885 | 0,875 | 0,894 | 0,924 | 0,870 | 0,978 | 0,963 | 0,909 | 1 |
| IFI27, IFI44L, SIGLEC1, ISG15, HERC6, SLC1A2, IL1R2, FAM20A | 0,885 | 0,875 | 0,894 | 0,931 | 0,884 | 0,979 | 0,948 | 0,879 | 1 |
| IFI27, IFIT1, IFI44L, ISG15, HERC6, SLC1A2, OLAH, FAM20A | 0,885 | 0,875 | 0,894 | 0,933 | 0,883 | 0,983 | 0,917 | 0,835 | 1,000 |
| RSAD2, IFI27, OAS1, IFIT1, HERC6, OLAH, RETN, MMP8 | 0,885 | 0,875 | 0,894 | 0,924 | 0,867 | 0,980 | 0,945 | 0,886 | 1 |
| RSAD2, IFI27, OAS1, IFI44L, ISG15, IL1R2, OLAH, FAM20A | 0,885 | 0,875 | 0,894 | 0,924 | 0,867 | 0,981 | 0,928 | 0,856 | 1 |
| IFI27, IFIT1, IFI44L, ISG15, IL1R2, OLAH, FAM20A, RETN | 0,884 | 0,876 | 0,893 | 0,924 | 0,867 | 0,981 | 0,932 | 0,861 | 1 |
| RSAD2, IFI27, OAS1, IFIT1, SIGLEC1, HERC6, OLAH, RETN | 0,884 | 0,876 | 0,893 | 0,926 | 0,873 | 0,979 | 0,947 | 0,891 | 1 |
| RSAD2, IFI27, IFIT1, HERC6, SLC1A2, OLAH, FAM20A, MMP8 | 0,884 | 0,875 | 0,894 | 0,930 | 0,879 | 0,981 | 0,925 | 0,848 | 1 |

| Genes | | | | | | | | |
|---|---|---|---|---|---|---|---|---|
| RSAD2, IFI27, SIGLEC1, ISG15, SLC1A2, OLAH, FAM20A, RETN | 0,884 | 0,875 | 0,894 | 0,929 | 0,876 | 0,982 | 0,935 | 0,865 | 1 |
| RSAD2, IFI27, OAS1, IFIT1, IFI44L, SIGLEC1, HERC6, FAM20A | 0,884 | 0,876 | 0,893 | 0,924 | 0,866 | 0,981 | 0,939 | 0,868 | 1 |
| IFI27, OAS1, IFIT1, ISG15, SLC1A2, OLAH, FAM20A, MMP8 | 0,884 | 0,875 | 0,894 | 0,927 | 0,873 | 0,981 | 0,925 | 0,849 | 1 |
| RSAD2, OAS1, IFIT1, SIGLEC1, HERC6, IL1R2, FAM20A, MMP8 | 0,884 | 0,876 | 0,893 | 0,923 | 0,869 | 0,977 | 0,962 | 0,906 | 1 |
| IFI27, OAS1, ISG15, HERC6, IL1R2, OLAH, RETN, MMP8 | 0,884 | 0,875 | 0,894 | 0,924 | 0,869 | 0,980 | 0,945 | 0,885 | 1 |
| IFI27, IFIT1, SIGLEC1, ISG15, HERC6, OLAH, RETN, MMP8 | 0,884 | 0,875 | 0,894 | 0,924 | 0,869 | 0,980 | 0,954 | 0,906 | 1 |
| IFI27, IFIT1, IFI44L, ISG15, HERC6, IL1R2, FAM20A, RETN | 0,884 | 0,875 | 0,893 | 0,931 | 0,880 | 0,982 | 0,929 | 0,849 | 1 |
| IFI27, IFIT1, SIGLEC1, HERC6, SLC1A2, FAM20A, RETN, MMP8 | 0,884 | 0,875 | 0,894 | 0,929 | 0,875 | 0,982 | 0,950 | 0,878 | 1 |
| RSAD2, IFI27, IFIT1, IFI44L, ISG15, OLAH, FAM20A, MMP8 | 0,884 | 0,874 | 0,894 | 0,922 | 0,863 | 0,982 | 0,937 | 0,872 | 1 |
| RSAD2, IFI27, OAS1, IFI44L, SIGLEC1, HERC6, OLAH, RETN | 0,884 | 0,876 | 0,893 | 0,924 | 0,870 | 0,978 | 0,942 | 0,883 | 1 |
| RSAD2, IFI27, OAS1, IFIT1, IFI44L, IL1R2, OLAH, FAM20A | 0,884 | 0,875 | 0,893 | 0,926 | 0,869 | 0,983 | 0,930 | 0,858 | 1 |
| IFI27, OAS1, IFI44L, SIGLEC1, HERC6, IL1R2, OLAH, MMP8 | 0,884 | 0,876 | 0,893 | 0,928 | 0,880 | 0,975 | 0,946 | 0,895 | 0,996 |
| IFI27, OAS1, IFIT1, IFI44L, SIGLEC1, HERC6, FAM20A, MMP8 | 0,884 | 0,876 | 0,893 | 0,923 | 0,867 | 0,980 | 0,937 | 0,865 | 1 |
| IFI27, OAS1, IFIT1, ISG15, HERC6, OLAH, RETN, MMP8 | 0,884 | 0,875 | 0,894 | 0,924 | 0,868 | 0,980 | 0,945 | 0,886 | 1 |
| RSAD2, IFI27, IFIT1, ISG15, SLC1A2, OLAH, FAM20A, MMP8 | 0,884 | 0,875 | 0,894 | 0,927 | 0,872 | 0,981 | 0,926 | 0,851 | 1 |
| IFI27, IFIT1, ISG15, HERC6, SLC1A2, OLAH, FAM20A, MMP8 | 0,884 | 0,874 | 0,894 | 0,930 | 0,879 | 0,981 | 0,922 | 0,842 | 1 |
| RSAD2, IFI27, OAS1, IFI44L, SIGLEC1, SLC1A2, OLAH, FAM20A | 0,884 | 0,875 | 0,894 | 0,929 | 0,873 | 0,984 | 0,941 | 0,877 | 1 |
| IFI27, OAS1, IFIT1, ISG15, IL1R2, OLAH, FAM20A, RETN | 0,884 | 0,875 | 0,893 | 0,924 | 0,868 | 0,981 | 0,929 | 0,859 | 1,000 |
| IFI27, IFI44L, HERC6, SLC1A2, IL1R2, OLAH, FAM20A, MMP8 | 0,884 | 0,874 | 0,894 | 0,933 | 0,885 | 0,982 | 0,927 | 0,848 | 1 |
| IFI27, IFI44L, SIGLEC1, SLC1A2, IL1R2, OLAH, FAM20A, RETN | 0,884 | 0,875 | 0,894 | 0,930 | 0,878 | 0,982 | 0,947 | 0,882 | 1 |
| RSAD2, IFI27, OAS1, IFIT1, HERC6, IL1R2, FAM20A, RETN | 0,884 | 0,875 | 0,893 | 0,930 | 0,879 | 0,981 | 0,929 | 0,848 | 1 |
| IFI27, OAS1, IFIT1, ISG15, HERC6, SLC1A2, OLAH, FAM20A | 0,884 | 0,874 | 0,894 | 0,932 | 0,882 | 0,981 | 0,917 | 0,835 | 1,000 |
| RSAD2, IFI27, OAS1, IFIT1, IFI44L, SIGLEC1, HERC6, RETN | 0,884 | 0,876 | 0,893 | 0,920 | 0,861 | 0,978 | 0,942 | 0,881 | 1 |
| IFI27, IFIT1, SIGLEC1, HERC6, IL1R2, OLAH, RETN, MMP8 | 0,884 | 0,875 | 0,893 | 0,926 | 0,872 | 0,980 | 0,955 | 0,907 | 1 |
| RSAD2, IFI27, OAS1, IFIT1, ISG15, IL1R2, OLAH, FAM20A | 0,884 | 0,875 | 0,893 | 0,924 | 0,868 | 0,981 | 0,929 | 0,859 | 1,000 |
| RSAD2, IFI27, IFIT1, IFI44L, SLC1A2, OLAH, FAM20A, MMP8 | 0,884 | 0,875 | 0,894 | 0,930 | 0,876 | 0,983 | 0,928 | 0,854 | 1 |
| RSAD2, IFI27, OAS1, IFI44L, HERC6, IL1R2, FAM20A, RETN | 0,884 | 0,874 | 0,894 | 0,931 | 0,880 | 0,981 | 0,929 | 0,848 | 1 |
| RSAD2, IFI27, OAS1, IFIT1, OLAH, FAM20A, RETN, MMP8 | 0,884 | 0,874 | 0,894 | 0,924 | 0,866 | 0,983 | 0,941 | 0,877 | 1 |

| | | | | | | | | |
|---|---|---|---|---|---|---|---|---|
| IFIT1, SIGLEC1, ISG15, HERC6, IL1R2, OLAH, FAM20A, MMP8 | 0,884 | 0,875 | 0,893 | 0,922 | 0,868 | 0,977 | 0,962 | 0,906 | 1 |
| IFI27, IFIT1, IFI44L, SIGLEC1, HERC6, SLC1A2, FAM20A, RETN | 0,884 | 0,875 | 0,893 | 0,929 | 0,878 | 0,981 | 0,941 | 0,867 | 1 |
| RSAD2, IFI27, IFIT1, IFI44L, HERC6, IL1R2, FAM20A, RETN | 0,884 | 0,874 | 0,894 | 0,932 | 0,882 | 0,983 | 0,929 | 0,849 | 1 |
| IFI27, OAS1, IFIT1, IFI44L, SIGLEC1, HERC6, IL1R2, RETN | 0,884 | 0,876 | 0,892 | 0,925 | 0,873 | 0,977 | 0,939 | 0,883 | 0,995 |
| IFI27, OAS1, SIGLEC1, HERC6, SLC1A2, FAM20A, RETN, MMP8 | 0,884 | 0,874 | 0,894 | 0,929 | 0,875 | 0,983 | 0,945 | 0,874 | 1 |
| RSAD2, IFI27, OAS1, IFIT1, HERC6, SLC1A2, OLAH, FAM20A | 0,884 | 0,874 | 0,894 | 0,929 | 0,878 | 0,981 | 0,920 | 0,837 | 1 |
| RSAD2, IFIT1, SIGLEC1, HERC6, IL1R2, OLAH, FAM20A, MMP8 | 0,884 | 0,875 | 0,893 | 0,924 | 0,871 | 0,978 | 0,962 | 0,906 | 1 |
| IFI27, OAS1, IFI44L, ISG15, IL1R2, OLAH, FAM20A, RETN | 0,884 | 0,875 | 0,893 | 0,924 | 0,867 | 0,981 | 0,928 | 0,856 | 1 |
| IFI27, OAS1, SIGLEC1, SLC1A2, IL1R2, OLAH, FAM20A, RETN | 0,884 | 0,874 | 0,894 | 0,930 | 0,877 | 0,982 | 0,941 | 0,876 | 1 |
| RSAD2, IFI27, OAS1, HERC6, IL1R2, OLAH, RETN, MMP8 | 0,884 | 0,875 | 0,893 | 0,924 | 0,868 | 0,979 | 0,945 | 0,886 | 1 |
| RSAD2, IFI27, OAS1, ISG15, HERC6, IL1R2, FAM20A, RETN | 0,884 | 0,875 | 0,893 | 0,930 | 0,880 | 0,981 | 0,927 | 0,844 | 1 |
| IFI27, OAS1, ISG15, SLC1A2, IL1R2, OLAH, FAM20A, MMP8 | 0,884 | 0,875 | 0,893 | 0,935 | 0,888 | 0,981 | 0,920 | 0,839 | 1,000 |
| IFI27, IFI44L, SIGLEC1, ISG15, HERC6, IL1R2, OLAH, MMP8 | 0,884 | 0,876 | 0,892 | 0,926 | 0,877 | 0,975 | 0,940 | 0,886 | 0,994 |
| RSAD2, IFI27, ISG15, HERC6, SLC1A2, IL1R2, OLAH, FAM20A | 0,884 | 0,875 | 0,893 | 0,932 | 0,883 | 0,981 | 0,917 | 0,834 | 1 |
| IFI27, OAS1, ISG15, HERC6, SLC1A2, IL1R2, OLAH, FAM20A | 0,884 | 0,874 | 0,894 | 0,933 | 0,886 | 0,981 | 0,914 | 0,828 | 1 |
| RSAD2, IFI27, OAS1, SIGLEC1, HERC6, IL1R2, RETN, MMP8 | 0,884 | 0,875 | 0,893 | 0,924 | 0,871 | 0,978 | 0,960 | 0,912 | 1 |
| RSAD2, IFI27, IFIT1, IFI44L, SIGLEC1, HERC6, OLAH, RETN | 0,884 | 0,875 | 0,892 | 0,925 | 0,872 | 0,978 | 0,948 | 0,897 | 0,999 |
| RSAD2, IFI27, OAS1, IFI44L, HERC6, SLC1A2, OLAH, FAM20A | 0,884 | 0,874 | 0,894 | 0,930 | 0,879 | 0,981 | 0,922 | 0,842 | 1 |
| IFI27, OAS1, IFI44L, SIGLEC1, HERC6, IL1R2, RETN, MMP8 | 0,884 | 0,875 | 0,893 | 0,928 | 0,877 | 0,978 | 0,951 | 0,903 | 0,999 |
| IFI27, OAS1, SIGLEC1, ISG15, HERC6, IL1R2, OLAH, RETN | 0,884 | 0,875 | 0,893 | 0,923 | 0,867 | 0,978 | 0,946 | 0,890 | 1 |
| IFI27, IFI44L, ISG15, SLC1A2, IL1R2, OLAH, FAM20A, RETN | 0,884 | 0,875 | 0,893 | 0,931 | 0,881 | 0,982 | 0,918 | 0,837 | 1,000 |
| IFI27, IFIT1, ISG15, HERC6, IL1R2, FAM20A, RETN, MMP8 | 0,884 | 0,874 | 0,893 | 0,930 | 0,878 | 0,983 | 0,935 | 0,856 | 1 |
| RSAD2, IFI27, OAS1, ISG15, HERC6, OLAH, RETN, MMP8 | 0,884 | 0,875 | 0,893 | 0,924 | 0,869 | 0,980 | 0,946 | 0,888 | 1 |
| RSAD2, IFI27, SIGLEC1, SLC1A2, IL1R2, OLAH, FAM20A, RETN | 0,884 | 0,874 | 0,894 | 0,929 | 0,876 | 0,982 | 0,946 | 0,882 | 1 |
| IFI27, OAS1, IFI44L, SIGLEC1, ISG15, HERC6, OLAH, RETN | 0,884 | 0,875 | 0,893 | 0,924 | 0,872 | 0,977 | 0,941 | 0,884 | 0,999 |
| IFI27, OAS1, IFI44L, SIGLEC1, HERC6, IL1R2, OLAH, RETN | 0,884 | 0,875 | 0,892 | 0,924 | 0,872 | 0,977 | 0,942 | 0,885 | 1,000 |
| IFI27, OAS1, IFIT1, IFI44L, IL1R2, OLAH, FAM20A, RETN | 0,884 | 0,875 | 0,893 | 0,924 | 0,867 | 0,982 | 0,930 | 0,858 | 1 |
| IFI27, IFIT1, IFI44L, SIGLEC1, SLC1A2, IL1R2, FAM20A, RETN | 0,884 | 0,875 | 0,893 | 0,934 | 0,886 | 0,981 | 0,943 | 0,874 | 1 |

| Genes | | | | | | | | |
|---|---|---|---|---|---|---|---|---|
| RSAD2, IFI27, OAS1, IFI44L, SIGLEC1, HERC6, IL1R2, OLAH | 0,884 | 0,875 | 0,892 | 0,927 | 0,879 | 0,975 | 0,947 | 0,897 | 0,997 |
| RSAD2, IFI27, SIGLEC1, HERC6, SLC1A2, OLAH, FAM20A, RETN | 0,884 | 0,874 | 0,894 | 0,932 | 0,881 | 0,983 | 0,939 | 0,871 | 1 |
| IFI27, OAS1, IFI44L, SIGLEC1, SLC1A2, OLAH, FAM20A, RETN | 0,884 | 0,874 | 0,893 | 0,927 | 0,871 | 0,983 | 0,940 | 0,875 | 1 |
| IFI27, OAS1, SIGLEC1, HERC6, SLC1A2, IL1R2, FAM20A, RETN | 0,884 | 0,874 | 0,894 | 0,935 | 0,889 | 0,980 | 0,940 | 0,869 | 1 |
| IFI27, OAS1, IFIT1, IFI44L, HERC6, OLAH, RETN, MMP8 | 0,884 | 0,874 | 0,893 | 0,924 | 0,867 | 0,981 | 0,945 | 0,887 | 1 |
| IFI27, IFIT1, SIGLEC1, HERC6, SLC1A2, IL1R2, FAM20A, RETN | 0,884 | 0,874 | 0,893 | 0,936 | 0,890 | 0,982 | 0,945 | 0,875 | 1 |
| IFI27, OAS1, IFIT1, ISG15, OLAH, FAM20A, RETN, MMP8 | 0,884 | 0,874 | 0,894 | 0,921 | 0,861 | 0,981 | 0,940 | 0,874 | 1 |
| RSAD2, IFI27, IFIT1, SIGLEC1, SLC1A2, OLAH, FAM20A, RETN | 0,884 | 0,874 | 0,893 | 0,930 | 0,875 | 0,984 | 0,942 | 0,877 | 1 |
| IFI27, OAS1, IFI44L, SIGLEC1, ISG15, SLC1A2, IL1R2, FAM20A | 0,884 | 0,874 | 0,893 | 0,932 | 0,884 | 0,980 | 0,941 | 0,871 | 1 |
| RSAD2, IFI27, OAS1, IFIT1, SIGLEC1, FAM20A, RETN, MMP8 | 0,884 | 0,874 | 0,894 | 0,924 | 0,865 | 0,982 | 0,947 | 0,877 | 1 |
| RSAD2, IFI27, IFI44L, SIGLEC1, HERC6, IL1R2, OLAH, MMP8 | 0,884 | 0,876 | 0,892 | 0,926 | 0,878 | 0,975 | 0,943 | 0,892 | 0,995 |
| RSAD2, IFI27, IFIT1, IFI44L, HERC6, SLC1A2, OLAH, FAM20A | 0,884 | 0,874 | 0,893 | 0,932 | 0,883 | 0,982 | 0,921 | 0,841 | 1,000 |
| IFI27, IFI44L, SIGLEC1, ISG15, HERC6, IL1R2, OLAH, RETN | 0,884 | 0,875 | 0,892 | 0,925 | 0,872 | 0,978 | 0,946 | 0,893 | 0,998 |
| IFI27, IFIT1, IFI44L, SIGLEC1, HERC6, IL1R2, OLAH, RETN | 0,884 | 0,875 | 0,892 | 0,926 | 0,874 | 0,979 | 0,947 | 0,895 | 0,999 |
| RSAD2, IFI27, OAS1, ISG15, SLC1A2, OLAH, FAM20A, MMP8 | 0,884 | 0,874 | 0,893 | 0,928 | 0,875 | 0,980 | 0,918 | 0,837 | 1 |
| IFI27, IFI44L, ISG15, HERC6, SLC1A2, IL1R2, OLAH, FAM20A | 0,884 | 0,874 | 0,893 | 0,934 | 0,885 | 0,982 | 0,918 | 0,836 | 1 |
| RSAD2, IFI27, OAS1, ISG15, OLAH, FAM20A, RETN, MMP8 | 0,884 | 0,874 | 0,894 | 0,923 | 0,864 | 0,982 | 0,937 | 0,869 | 1 |
| RSAD2, IFI27, OAS1, SIGLEC1, HERC6, IL1R2, OLAH, RETN | 0,884 | 0,875 | 0,893 | 0,924 | 0,869 | 0,979 | 0,946 | 0,889 | 1 |
| IFI27, OAS1, IFIT1, IFI44L, OLAH, FAM20A, RETN, MMP8 | 0,884 | 0,873 | 0,894 | 0,923 | 0,863 | 0,983 | 0,941 | 0,876 | 1 |
| IFI27, OAS1, SIGLEC1, HERC6, SLC1A2, OLAH, RETN, MMP8 | 0,884 | 0,874 | 0,893 | 0,932 | 0,883 | 0,981 | 0,948 | 0,888 | 1 |
| IFI27, OAS1, IFIT1, HERC6, SLC1A2, IL1R2, OLAH, FAM20A | 0,884 | 0,874 | 0,893 | 0,932 | 0,883 | 0,981 | 0,921 | 0,840 | 1 |
| RSAD2, IFI27, SIGLEC1, ISG15, HERC6, SLC1A2, IL1R2, FAM20A | 0,884 | 0,874 | 0,893 | 0,935 | 0,888 | 0,981 | 0,945 | 0,874 | 1 |
| RSAD2, IFI27, OAS1, IFIT1, IFI44L, IL1R2, FAM20A, MMP8 | 0,884 | 0,874 | 0,893 | 0,926 | 0,873 | 0,978 | 0,935 | 0,857 | 1 |
| RSAD2, IFI27, OAS1, IFIT1, ISG15, HERC6, IL1R2, FAM20A | 0,884 | 0,875 | 0,893 | 0,929 | 0,878 | 0,979 | 0,925 | 0,842 | 1 |
| IFI27, IFI44L, SIGLEC1, ISG15, SLC1A2, IL1R2, FAM20A, RETN | 0,884 | 0,874 | 0,893 | 0,932 | 0,885 | 0,980 | 0,945 | 0,874 | 1 |
| IFI27, SIGLEC1, HERC6, SLC1A2, IL1R2, FAM20A, RETN, MMP8 | 0,884 | 0,874 | 0,893 | 0,935 | 0,887 | 0,983 | 0,955 | 0,888 | 1 |
| RSAD2, IFI27, OAS1, IFI44L, SLC1A2, OLAH, FAM20A, MMP8 | 0,884 | 0,873 | 0,894 | 0,931 | 0,879 | 0,984 | 0,926 | 0,850 | 1 |
| RSAD2, IFI27, IFI44L, ISG15, HERC6, SLC1A2, OLAH, FAM20A | 0,884 | 0,873 | 0,894 | 0,934 | 0,884 | 0,983 | 0,918 | 0,837 | 1,000 |

| | | | | | | | | |
|---|---|---|---|---|---|---|---|---|
| IFI27, OAS1, IFIT1, IFI44L, IL1R2, FAM20A, RETN, MMP8 | 0,884 | 0,874 | 0,893 | 0,929 | 0,876 | 0,982 | 0,935 | 0,858 | 1 |
| IFI27, IFIT1, SIGLEC1, ISG15, SLC1A2, IL1R2, FAM20A, RETN | 0,884 | 0,874 | 0,893 | 0,933 | 0,886 | 0,980 | 0,943 | 0,874 | 1 |
| IFI27, OAS1, IFIT1, SIGLEC1, ISG15, HERC6, FAM20A, MMP8 | 0,883 | 0,874 | 0,893 | 0,924 | 0,867 | 0,980 | 0,939 | 0,868 | 1 |
| RSAD2, IFI27, OAS1, ISG15, IL1R2, OLAH, FAM20A, RETN | 0,883 | 0,874 | 0,893 | 0,924 | 0,868 | 0,980 | 0,925 | 0,850 | 1,000 |
| IFI27, OAS1, IFIT1, SIGLEC1, HERC6, IL1R2, OLAH, MMP8 | 0,883 | 0,875 | 0,892 | 0,924 | 0,875 | 0,974 | 0,942 | 0,890 | 0,995 |
| IFI27, ISG15, SLC1A2, IL1R2, OLAH, FAM20A, RETN, MMP8 | 0,883 | 0,874 | 0,893 | 0,933 | 0,883 | 0,982 | 0,921 | 0,840 | 1 |
| RSAD2, IFI27, IFIT1, ISG15, HERC6, IL1R2, FAM20A, MMP8 | 0,883 | 0,874 | 0,892 | 0,929 | 0,878 | 0,979 | 0,934 | 0,854 | 1 |
| IFI27, OAS1, IFI44L, HERC6, SLC1A2, IL1R2, OLAH, FAM20A | 0,883 | 0,873 | 0,894 | 0,931 | 0,881 | 0,982 | 0,921 | 0,839 | 1 |
| OAS1, IFIT1, SIGLEC1, HERC6, IL1R2, FAM20A, RETN, MMP8 | 0,883 | 0,874 | 0,892 | 0,923 | 0,868 | 0,979 | 0,962 | 0,905 | 1 |
| RSAD2, IFIT1, IFI44L, SIGLEC1, HERC6, IL1R2, FAM20A, MMP8 | 0,883 | 0,875 | 0,892 | 0,924 | 0,870 | 0,978 | 0,962 | 0,906 | 1 |
| OAS1, IFIT1, SIGLEC1, ISG15, HERC6, IL1R2, FAM20A, MMP8 | 0,883 | 0,874 | 0,892 | 0,923 | 0,868 | 0,978 | 0,963 | 0,907 | 1 |
| IFI27, SIGLEC1, ISG15, HERC6, SLC1A2, OLAH, FAM20A, RETN | 0,883 | 0,873 | 0,893 | 0,931 | 0,878 | 0,983 | 0,939 | 0,872 | 1 |
| IFI27, OAS1, IFIT1, SIGLEC1, SLC1A2, IL1R2, FAM20A, RETN | 0,883 | 0,874 | 0,893 | 0,933 | 0,886 | 0,981 | 0,943 | 0,875 | 1 |
| RSAD2, IFI27, OAS1, IFIT1, SIGLEC1, HERC6, IL1R2, RETN | 0,883 | 0,875 | 0,892 | 0,925 | 0,872 | 0,978 | 0,951 | 0,897 | 1 |
| RSAD2, IFI27, OAS1, IFIT1, SLC1A2, OLAH, FAM20A, MMP8 | 0,883 | 0,874 | 0,893 | 0,924 | 0,867 | 0,980 | 0,926 | 0,850 | 1 |
| RSAD2, IFI27, IFIT1, IFI44L, SIGLEC1, HERC6, FAM20A, MMP8 | 0,883 | 0,874 | 0,892 | 0,923 | 0,864 | 0,981 | 0,940 | 0,869 | 1 |
| IFI27, OAS1, ISG15, HERC6, SLC1A2, OLAH, FAM20A, RETN | 0,883 | 0,874 | 0,893 | 0,932 | 0,883 | 0,982 | 0,915 | 0,829 | 1 |
| IFI27, OAS1, IFIT1, IFI44L, SIGLEC1, HERC6, OLAH, MMP8 | 0,883 | 0,875 | 0,892 | 0,926 | 0,875 | 0,977 | 0,943 | 0,890 | 0,997 |
| IFI27, SIGLEC1, HERC6, SLC1A2, OLAH, FAM20A, RETN, MMP8 | 0,883 | 0,873 | 0,894 | 0,927 | 0,871 | 0,983 | 0,948 | 0,881 | 1 |
| IFIT1, IFI44L, SIGLEC1, HERC6, IL1R2, FAM20A, RETN, MMP8 | 0,883 | 0,874 | 0,892 | 0,925 | 0,870 | 0,980 | 0,964 | 0,909 | 1 |
| IFI27, OAS1, IFIT1, SIGLEC1, ISG15, SLC1A2, IL1R2, FAM20A | 0,883 | 0,874 | 0,893 | 0,933 | 0,886 | 0,980 | 0,943 | 0,875 | 1 |
| IFI27, IFIT1, IFI44L, SIGLEC1, ISG15, HERC6, IL1R2, OLAH | 0,883 | 0,875 | 0,891 | 0,927 | 0,878 | 0,976 | 0,942 | 0,889 | 0,996 |
| RSAD2, IFI27, IFIT1, IFI44L, SIGLEC1, ISG15, FAM20A, RETN | 0,883 | 0,874 | 0,892 | 0,920 | 0,861 | 0,980 | 0,943 | 0,874 | 1 |
| IFI27, OAS1, IFI44L, ISG15, OLAH, FAM20A, RETN, MMP8 | 0,883 | 0,873 | 0,894 | 0,923 | 0,864 | 0,983 | 0,941 | 0,875 | 1 |
| IFI27, OAS1, IFIT1, SIGLEC1, ISG15, HERC6, IL1R2, RETN | 0,883 | 0,875 | 0,892 | 0,924 | 0,872 | 0,976 | 0,947 | 0,891 | 1 |
| RSAD2, IFI27, OAS1, IFI44L, SIGLEC1, HERC6, IL1R2, RETN | 0,883 | 0,875 | 0,891 | 0,924 | 0,872 | 0,977 | 0,954 | 0,904 | 1 |
| RSAD2, IFI27, IFI44L, SIGLEC1, HERC6, SLC1A2, IL1R2, FAM20A | 0,883 | 0,873 | 0,893 | 0,932 | 0,883 | 0,980 | 0,948 | 0,879 | 1 |
| IFI27, OAS1, IFI44L, SIGLEC1, ISG15, HERC6, IL1R2, RETN | 0,883 | 0,875 | 0,891 | 0,927 | 0,877 | 0,977 | 0,939 | 0,883 | 0,995 |

| | | | | | | | | |
|---|---|---|---|---|---|---|---|---|
| RSAD2, IFI27, OAS1, IFIT1, SIGLEC1, HERC6, FAM20A, MMP8 | 0,883 | 0,874 | 0,892 | 0,923 | 0,866 | 0,980 | 0,938 | 0,867 | 1 |
| IFI27, ISG15, HERC6, SLC1A2, OLAH, FAM20A, RETN, MMP8 | 0,883 | 0,873 | 0,893 | 0,931 | 0,880 | 0,982 | 0,922 | 0,839 | 1 |
| IFI27, OAS1, SIGLEC1, ISG15, HERC6, IL1R2, RETN, MMP8 | 0,883 | 0,874 | 0,892 | 0,926 | 0,873 | 0,979 | 0,957 | 0,907 | 1 |
| RSAD2, IFI27, IFI44L, ISG15, HERC6, IL1R2, FAM20A, RETN | 0,883 | 0,873 | 0,893 | 0,932 | 0,881 | 0,982 | 0,929 | 0,849 | 1 |
| IFI27, IFIT1, IFI44L, HERC6, SLC1A2, IL1R2, OLAH, FAM20A | 0,883 | 0,874 | 0,892 | 0,931 | 0,882 | 0,981 | 0,925 | 0,845 | 1 |
| IFI27, OAS1, SIGLEC1, ISG15, HERC6, IL1R2, OLAH, MMP8 | 0,883 | 0,874 | 0,892 | 0,924 | 0,875 | 0,973 | 0,943 | 0,891 | 0,996 |
| RSAD2, IFI27, IFIT1, IFI44L, ISG15, IL1R2, OLAH, FAM20A | 0,883 | 0,874 | 0,892 | 0,924 | 0,867 | 0,981 | 0,932 | 0,861 | 1 |
| IFI27, OAS1, IFIT1, HERC6, SLC1A2, OLAH, FAM20A, RETN | 0,883 | 0,873 | 0,893 | 0,932 | 0,882 | 0,982 | 0,916 | 0,830 | 1 |
| RSAD2, IFI27, OAS1, IFIT1, IFI44L, ISG15, OLAH, FAM20A | 0,883 | 0,874 | 0,892 | 0,921 | 0,861 | 0,981 | 0,932 | 0,863 | 1 |
| RSAD2, IFI27, IFIT1, SIGLEC1, ISG15, FAM20A, RETN, MMP8 | 0,883 | 0,873 | 0,893 | 0,923 | 0,863 | 0,982 | 0,946 | 0,874 | 1 |
| RSAD2, IFI27, IFI44L, SIGLEC1, HERC6, IL1R2, OLAH, RETN | 0,883 | 0,874 | 0,892 | 0,924 | 0,872 | 0,977 | 0,947 | 0,895 | 0,999 |
| RSAD2, IFI27, OAS1, IFI44L, HERC6, OLAH, RETN, MMP8 | 0,883 | 0,874 | 0,892 | 0,924 | 0,867 | 0,980 | 0,941 | 0,882 | 1 |
| IFIT1, SIGLEC1, HERC6, IL1R2, OLAH, FAM20A, RETN, MMP8 | 0,883 | 0,874 | 0,892 | 0,925 | 0,870 | 0,980 | 0,961 | 0,904 | 1 |
| IFI27, OAS1, IFIT1, IFI44L, SIGLEC1, HERC6, IL1R2, OLAH | 0,883 | 0,875 | 0,891 | 0,926 | 0,877 | 0,975 | 0,946 | 0,895 | 0,996 |
| IFI27, OAS1, IFI44L, SLC1A2, IL1R2, OLAH, FAM20A, MMP8 | 0,883 | 0,873 | 0,893 | 0,934 | 0,884 | 0,983 | 0,927 | 0,851 | 1 |
| IFI27, IFIT1, SIGLEC1, ISG15, HERC6, IL1R2, OLAH, RETN | 0,883 | 0,874 | 0,892 | 0,923 | 0,869 | 0,978 | 0,947 | 0,894 | 0,999 |
| IFI27, OAS1, IFI44L, SIGLEC1, ISG15, HERC6, IL1R2, OLAH | 0,883 | 0,874 | 0,891 | 0,926 | 0,877 | 0,975 | 0,941 | 0,888 | 0,995 |
| RSAD2, IFI27, IFIT1, ISG15, HERC6, IL1R2, FAM20A, RETN | 0,883 | 0,874 | 0,892 | 0,932 | 0,881 | 0,983 | 0,929 | 0,849 | 1 |
| RSAD2, IFI27, OAS1, SIGLEC1, HERC6, IL1R2, OLAH, MMP8 | 0,883 | 0,874 | 0,892 | 0,924 | 0,874 | 0,973 | 0,943 | 0,891 | 0,996 |
| IFI27, OAS1, IFI44L, HERC6, IL1R2, OLAH, RETN, MMP8 | 0,883 | 0,874 | 0,892 | 0,924 | 0,869 | 0,979 | 0,940 | 0,882 | 0,999 |
| IFI27, OAS1, IFI44L, ISG15, HERC6, OLAH, RETN, MMP8 | 0,883 | 0,874 | 0,892 | 0,925 | 0,869 | 0,980 | 0,942 | 0,883 | 1 |
| RSAD2, IFI27, OAS1, IFIT1, IFI44L, SIGLEC1, FAM20A, RETN | 0,883 | 0,873 | 0,892 | 0,918 | 0,858 | 0,979 | 0,945 | 0,876 | 1 |
| IFI27, OAS1, IFI44L, SIGLEC1, ISG15, HERC6, RETN, MMP8 | 0,883 | 0,874 | 0,892 | 0,920 | 0,863 | 0,978 | 0,945 | 0,884 | 1 |
| RSAD2, IFI27, OAS1, IFIT1, SIGLEC1, ISG15, HERC6, FAM20A | 0,883 | 0,874 | 0,892 | 0,923 | 0,866 | 0,980 | 0,939 | 0,868 | 1 |
| RSAD2, IFI27, IFIT1, HERC6, IL1R2, OLAH, RETN, MMP8 | 0,883 | 0,874 | 0,892 | 0,924 | 0,869 | 0,980 | 0,945 | 0,889 | 1 |
| RSAD2, IFI27, OAS1, IFI44L, SIGLEC1, ISG15, HERC6, RETN | 0,883 | 0,874 | 0,891 | 0,920 | 0,863 | 0,978 | 0,942 | 0,878 | 1 |
| RSAD2, IFI27, OAS1, IFI44L, SIGLEC1, HERC6, OLAH, MMP8 | 0,883 | 0,874 | 0,891 | 0,926 | 0,875 | 0,977 | 0,945 | 0,892 | 0,997 |
| RSAD2, IFI27, IFI44L, HERC6, SLC1A2, IL1R2, OLAH, FAM20A | 0,883 | 0,873 | 0,893 | 0,931 | 0,881 | 0,981 | 0,925 | 0,847 | 1 |

| | | | | | | | | | |
|---|---|---|---|---|---|---|---|---|---|
| RSAD2, IFI27, OAS1, IFI44L, ISG15, HERC6, FAM20A, RETN | 0,883 | 0,873 | 0,893 | 0,925 | 0,869 | 0,981 | 0,934 | 0,856 | 1 |
| IFI27, IFIT1, SLC1A2, IL1R2, OLAH, FAM20A, RETN, MMP8 | 0,883 | 0,873 | 0,892 | 0,932 | 0,881 | 0,982 | 0,923 | 0,843 | 1 |
| IFI27, IFI44L, ISG15, HERC6, IL1R2, OLAH, RETN, MMP8 | 0,883 | 0,874 | 0,892 | 0,923 | 0,867 | 0,979 | 0,945 | 0,890 | 0,999 |
| IFI27, OAS1, IFIT1, IFI44L, ISG15, OLAH, FAM20A, RETN | 0,883 | 0,873 | 0,892 | 0,920 | 0,859 | 0,980 | 0,934 | 0,864 | 1 |
| RSAD2, IFI27, SIGLEC1, ISG15, HERC6, OLAH, RETN, MMP8 | 0,883 | 0,873 | 0,892 | 0,924 | 0,868 | 0,980 | 0,954 | 0,905 | 1 |
| RSAD2, IFI27, OAS1, IFIT1, SIGLEC1, HERC6, RETN, MMP8 | 0,883 | 0,874 | 0,892 | 0,919 | 0,860 | 0,978 | 0,947 | 0,889 | 1 |
| RSAD2, IFI27, OAS1, SIGLEC1, ISG15, HERC6, OLAH, RETN | 0,883 | 0,874 | 0,892 | 0,924 | 0,869 | 0,979 | 0,946 | 0,889 | 1 |
| RSAD2, IFI27, OAS1, IFIT1, HERC6, IL1R2, OLAH, RETN | 0,883 | 0,874 | 0,892 | 0,923 | 0,867 | 0,979 | 0,940 | 0,880 | 1 |
| RSAD2, IFI27, IFI44L, SIGLEC1, SLC1A2, IL1R2, OLAH, FAM20A | 0,883 | 0,873 | 0,893 | 0,929 | 0,876 | 0,982 | 0,946 | 0,881 | 1 |
| RSAD2, IFI27, IFIT1, IFI44L, ISG15, HERC6, FAM20A, RETN | 0,883 | 0,873 | 0,892 | 0,925 | 0,870 | 0,980 | 0,932 | 0,853 | 1 |
| IFI27, OAS1, IFIT1, IFI44L, ISG15, SLC1A2, OLAH, FAM20A | 0,883 | 0,873 | 0,892 | 0,930 | 0,877 | 0,983 | 0,920 | 0,840 | 0,999 |
| IFI27, OAS1, IFIT1, IFI44L, SIGLEC1, HERC6, RETN, MMP8 | 0,883 | 0,874 | 0,891 | 0,920 | 0,863 | 0,977 | 0,943 | 0,884 | 1 |
| IFI27, IFIT1, HERC6, SLC1A2, OLAH, FAM20A, RETN, MMP8 | 0,883 | 0,873 | 0,893 | 0,931 | 0,880 | 0,982 | 0,924 | 0,842 | 1 |
| IFI27, OAS1, IFI44L, SIGLEC1, SLC1A2, IL1R2, FAM20A, RETN | 0,883 | 0,873 | 0,892 | 0,932 | 0,883 | 0,981 | 0,941 | 0,870 | 1 |
| RSAD2, IFIT1, SIGLEC1, ISG15, HERC6, IL1R2, FAM20A, MMP8 | 0,883 | 0,874 | 0,891 | 0,922 | 0,867 | 0,977 | 0,963 | 0,907 | 1 |
| IFIT1, SIGLEC1, ISG15, HERC6, IL1R2, FAM20A, RETN, MMP8 | 0,883 | 0,874 | 0,891 | 0,923 | 0,867 | 0,979 | 0,961 | 0,901 | 1 |
| RSAD2, IFI27, OAS1, IFIT1, SIGLEC1, ISG15, FAM20A, RETN | 0,883 | 0,873 | 0,892 | 0,920 | 0,861 | 0,980 | 0,943 | 0,875 | 1 |
| IFI27, IFIT1, IFI44L, ISG15, IL1R2, FAM20A, RETN, MMP8 | 0,883 | 0,873 | 0,892 | 0,929 | 0,876 | 0,982 | 0,937 | 0,860 | 1 |
| RSAD2, IFI27, IFIT1, ISG15, HERC6, SLC1A2, OLAH, FAM20A | 0,883 | 0,873 | 0,893 | 0,931 | 0,880 | 0,981 | 0,917 | 0,835 | 0,999 |
| RSAD2, IFI27, IFIT1, IFI44L, OLAH, FAM20A, RETN, MMP8 | 0,883 | 0,872 | 0,893 | 0,922 | 0,862 | 0,982 | 0,938 | 0,871 | 1 |
| RSAD2, IFI27, HERC6, SLC1A2, IL1R2, OLAH, FAM20A, RETN | 0,882 | 0,873 | 0,892 | 0,931 | 0,881 | 0,981 | 0,920 | 0,835 | 1 |
| RSAD2, IFI27, IFIT1, HERC6, SLC1A2, IL1R2, OLAH, FAM20A | 0,882 | 0,873 | 0,892 | 0,931 | 0,881 | 0,980 | 0,923 | 0,842 | 1 |
| RSAD2, IFI27, ISG15, HERC6, SLC1A2, OLAH, FAM20A, RETN | 0,882 | 0,873 | 0,892 | 0,930 | 0,878 | 0,981 | 0,914 | 0,829 | 1,000 |
| RSAD2, IFI27, OAS1, IFIT1, IFI44L, HERC6, FAM20A, RETN | 0,882 | 0,873 | 0,892 | 0,924 | 0,866 | 0,981 | 0,933 | 0,853 | 1 |
| RSAD2, IFI27, IFIT1, IFI44L, SIGLEC1, SLC1A2, IL1R2, FAM20A | 0,882 | 0,873 | 0,891 | 0,935 | 0,888 | 0,982 | 0,943 | 0,874 | 1 |
| RSAD2, IFI27, IFIT1, IFI44L, HERC6, OLAH, RETN, MMP8 | 0,882 | 0,873 | 0,891 | 0,923 | 0,867 | 0,980 | 0,949 | 0,895 | 1 |
| RSAD2, IFI27, IFIT1, ISG15, OLAH, FAM20A, RETN, MMP8 | 0,882 | 0,872 | 0,893 | 0,921 | 0,860 | 0,981 | 0,940 | 0,875 | 1 |
| RSAD2, IFI27, OAS1, IFIT1, IFI44L, HERC6, OLAH, RETN | 0,882 | 0,874 | 0,891 | 0,921 | 0,864 | 0,978 | 0,940 | 0,881 | 1,000 |

| Genes | | | | | | | | |
|---|---|---|---|---|---|---|---|---|
| RSAD2, IFI27, IFIT1, IFI44L, SIGLEC1, FAM20A, RETN, MMP8 | 0,882 | 0,872 | 0,892 | 0,923 | 0,864 | 0,982 | 0,947 | 0,877 | 1 |
| IFI27, OAS1, IFIT1, SIGLEC1, ISG15, HERC6, OLAH, MMP8 | 0,882 | 0,874 | 0,891 | 0,923 | 0,871 | 0,975 | 0,936 | 0,878 | 0,994 |
| RSAD2, IFI27, IFI44L, ISG15, IL1R2, OLAH, FAM20A, RETN | 0,882 | 0,873 | 0,892 | 0,923 | 0,866 | 0,980 | 0,928 | 0,856 | 1 |
| IFI27, IFIT1, IFI44L, ISG15, SLC1A2, IL1R2, OLAH, FAM20A | 0,882 | 0,873 | 0,891 | 0,931 | 0,880 | 0,982 | 0,917 | 0,836 | 0,999 |
| IFI27, OAS1, IFIT1, ISG15, HERC6, IL1R2, OLAH, RETN | 0,882 | 0,874 | 0,891 | 0,922 | 0,866 | 0,977 | 0,939 | 0,878 | 1,000 |
| RSAD2, IFI27, OAS1, ISG15, HERC6, IL1R2, OLAH, RETN | 0,882 | 0,873 | 0,891 | 0,923 | 0,867 | 0,979 | 0,943 | 0,885 | 1 |
| RSAD2, IFI27, OAS1, HERC6, SLC1A2, OLAH, FAM20A, RETN | 0,882 | 0,873 | 0,892 | 0,932 | 0,883 | 0,982 | 0,917 | 0,831 | 1 |
| RSAD2, IFI27, SLC1A2, IL1R2, OLAH, FAM20A, RETN, MMP8 | 0,882 | 0,873 | 0,892 | 0,933 | 0,883 | 0,982 | 0,922 | 0,840 | 1 |
| RSAD2, IFI27, IFIT1, ISG15, IL1R2, OLAH, FAM20A, RETN | 0,882 | 0,873 | 0,891 | 0,924 | 0,867 | 0,981 | 0,932 | 0,861 | 1 |
| IFI27, OAS1, IFIT1, SIGLEC1, ISG15, HERC6, IL1R2, OLAH | 0,882 | 0,874 | 0,891 | 0,924 | 0,874 | 0,974 | 0,942 | 0,889 | 0,995 |
| RSAD2, IFI27, OAS1, SIGLEC1, ISG15, FAM20A, RETN, MMP8 | 0,882 | 0,872 | 0,893 | 0,925 | 0,866 | 0,983 | 0,945 | 0,872 | 1 |
| IFI27, IFIT1, ISG15, HERC6, SLC1A2, IL1R2, OLAH, FAM20A | 0,882 | 0,873 | 0,892 | 0,932 | 0,883 | 0,981 | 0,920 | 0,838 | 1 |
| RSAD2, IFI27, IFI44L, HERC6, IL1R2, OLAH, RETN, MMP8 | 0,882 | 0,873 | 0,891 | 0,924 | 0,869 | 0,980 | 0,945 | 0,889 | 1 |
| RSAD2, IFI27, OAS1, IFI44L, OLAH, FAM20A, RETN, MMP8 | 0,882 | 0,872 | 0,893 | 0,923 | 0,864 | 0,982 | 0,938 | 0,870 | 1 |
| RSAD2, IFI27, IFIT1, IFI44L, SIGLEC1, HERC6, IL1R2, OLAH | 0,882 | 0,874 | 0,891 | 0,928 | 0,879 | 0,976 | 0,943 | 0,892 | 0,995 |
| RSAD2, IFI27, OAS1, SIGLEC1, ISG15, HERC6, IL1R2, OLAH | 0,882 | 0,873 | 0,891 | 0,924 | 0,874 | 0,974 | 0,940 | 0,885 | 0,996 |
| RSAD2, IFI27, IFIT1, SIGLEC1, ISG15, SLC1A2, IL1R2, FAM20A | 0,882 | 0,873 | 0,891 | 0,934 | 0,888 | 0,980 | 0,945 | 0,875 | 1 |
| RSAD2, IFI27, OAS1, SIGLEC1, SLC1A2, OLAH, FAM20A, RETN | 0,882 | 0,873 | 0,892 | 0,927 | 0,871 | 0,983 | 0,939 | 0,872 | 1 |
| IFI27, HERC6, SLC1A2, IL1R2, OLAH, FAM20A, RETN, MMP8 | 0,882 | 0,872 | 0,892 | 0,933 | 0,883 | 0,982 | 0,928 | 0,851 | 1 |
| IFI27, IFIT1, IFI44L, ISG15, HERC6, OLAH, RETN, MMP8 | 0,882 | 0,873 | 0,891 | 0,922 | 0,864 | 0,979 | 0,947 | 0,894 | 1,000 |
| RSAD2, IFI27, OAS1, IFIT1, IL1R2, FAM20A, RETN, MMP8 | 0,882 | 0,873 | 0,892 | 0,930 | 0,877 | 0,982 | 0,934 | 0,856 | 1 |
| IFI27, IFIT1, IFI44L, SIGLEC1, ISG15, HERC6, OLAH, MMP8 | 0,882 | 0,873 | 0,891 | 0,925 | 0,873 | 0,977 | 0,945 | 0,892 | 0,997 |
| IFI27, IFIT1, IFI44L, SIGLEC1, HERC6, IL1R2, OLAH, MMP8 | 0,882 | 0,874 | 0,890 | 0,926 | 0,877 | 0,975 | 0,945 | 0,894 | 0,995 |
| RSAD2, IFI27, IFI44L, SIGLEC1, ISG15, HERC6, OLAH, RETN | 0,882 | 0,874 | 0,891 | 0,924 | 0,870 | 0,978 | 0,946 | 0,893 | 0,998 |
| RSAD2, IFI27, IFIT1, SIGLEC1, ISG15, HERC6, FAM20A, MMP8 | 0,882 | 0,873 | 0,891 | 0,922 | 0,864 | 0,980 | 0,940 | 0,869 | 1 |
| RSAD2, IFI27, IFI44L, ISG15, OLAH, FAM20A, RETN, MMP8 | 0,882 | 0,872 | 0,893 | 0,923 | 0,864 | 0,983 | 0,939 | 0,872 | 1 |
| RSAD2, IFI27, ISG15, HERC6, IL1R2, OLAH, RETN, MMP8 | 0,882 | 0,873 | 0,891 | 0,925 | 0,870 | 0,980 | 0,943 | 0,887 | 1 |
| RSAD2, IFI27, IFIT1, SIGLEC1, ISG15, HERC6, OLAH, RETN | 0,882 | 0,873 | 0,891 | 0,926 | 0,873 | 0,978 | 0,942 | 0,887 | 0,998 |

| | | | | | | | | |
|---|---|---|---|---|---|---|---|---|
| RSAD2, IFI27, OAS1, IFIT1, ISG15, HERC6, FAM20A, RETN | 0,882 | 0,872 | 0,892 | 0,923 | 0,867 | 0,980 | 0,935 | 0,857 | 1 |
| RSAD2, IFI27, OAS1, IFIT1, IL1R2, OLAH, FAM20A, RETN | 0,882 | 0,873 | 0,891 | 0,924 | 0,868 | 0,981 | 0,933 | 0,863 | 1 |
| RSAD2, OAS1, IFIT1, SIGLEC1, IL1R2, OLAH, FAM20A, MMP8 | 0,882 | 0,873 | 0,891 | 0,919 | 0,862 | 0,977 | 0,964 | 0,910 | 1 |
| RSAD2, IFI27, OAS1, IFIT1, ISG15, HERC6, OLAH, RETN | 0,882 | 0,873 | 0,891 | 0,923 | 0,868 | 0,978 | 0,941 | 0,882 | 1 |
| RSAD2, IFI27, IFIT1, SIGLEC1, HERC6, IL1R2, OLAH, RETN | 0,882 | 0,873 | 0,891 | 0,926 | 0,873 | 0,978 | 0,942 | 0,887 | 0,998 |
| IFI27, OAS1, IFI44L, HERC6, SLC1A2, OLAH, FAM20A, RETN | 0,882 | 0,872 | 0,892 | 0,932 | 0,882 | 0,982 | 0,920 | 0,837 | 1 |
| IFI27, OAS1, IFI44L, ISG15, SLC1A2, IL1R2, OLAH, FAM20A | 0,882 | 0,872 | 0,892 | 0,931 | 0,880 | 0,983 | 0,916 | 0,833 | 1,000 |
| IFI27, OAS1, IFI44L, ISG15, HERC6, FAM20A, RETN, MMP8 | 0,882 | 0,871 | 0,892 | 0,927 | 0,869 | 0,984 | 0,937 | 0,859 | 1 |
| RSAD2, IFI27, OAS1, ISG15, SLC1A2, IL1R2, OLAH, FAM20A | 0,882 | 0,872 | 0,891 | 0,932 | 0,883 | 0,981 | 0,916 | 0,832 | 1 |
| RSAD2, IFI27, IFIT1, SIGLEC1, HERC6, SLC1A2, FAM20A, RETN | 0,882 | 0,873 | 0,891 | 0,929 | 0,878 | 0,981 | 0,938 | 0,865 | 1 |
| RSAD2, IFI27, OAS1, IFIT1, SIGLEC1, HERC6, OLAH, MMP8 | 0,882 | 0,873 | 0,891 | 0,923 | 0,872 | 0,974 | 0,938 | 0,881 | 0,995 |
| IFI27, IFIT1, IFI44L, ISG15, SLC1A2, OLAH, FAM20A, RETN | 0,882 | 0,873 | 0,891 | 0,928 | 0,874 | 0,982 | 0,916 | 0,834 | 0,999 |
| RSAD2, IFI27, IFI44L, ISG15, SLC1A2, IL1R2, OLAH, FAM20A | 0,882 | 0,872 | 0,892 | 0,931 | 0,880 | 0,982 | 0,918 | 0,837 | 1,000 |
| IFI27, IFIT1, IFI44L, SIGLEC1, HERC6, IL1R2, RETN, MMP8 | 0,882 | 0,873 | 0,890 | 0,924 | 0,872 | 0,977 | 0,953 | 0,908 | 0,999 |
| RSAD2, IFI27, IFI44L, SIGLEC1, ISG15, HERC6, IL1R2, OLAH | 0,882 | 0,873 | 0,890 | 0,926 | 0,876 | 0,976 | 0,942 | 0,889 | 0,996 |
| RSAD2, IFI27, IFIT1, IFI44L, ISG15, SLC1A2, OLAH, FAM20A | 0,882 | 0,873 | 0,891 | 0,928 | 0,874 | 0,982 | 0,916 | 0,834 | 0,999 |
| IFI27, OAS1, IFIT1, IFI44L, SIGLEC1, ISG15, HERC6, OLAH | 0,882 | 0,873 | 0,890 | 0,927 | 0,876 | 0,978 | 0,942 | 0,888 | 0,997 |
| IFI27, OAS1, IFIT1, ISG15, IL1R2, FAM20A, RETN, MMP8 | 0,882 | 0,872 | 0,892 | 0,930 | 0,877 | 0,983 | 0,934 | 0,856 | 1 |
| RSAD2, IFI27, SIGLEC1, HERC6, SLC1A2, IL1R2, FAM20A, RETN | 0,882 | 0,872 | 0,892 | 0,934 | 0,886 | 0,982 | 0,946 | 0,876 | 1 |
| RSAD2, OAS1, SIGLEC1, HERC6, IL1R2, OLAH, FAM20A, MMP8 | 0,882 | 0,872 | 0,891 | 0,920 | 0,864 | 0,976 | 0,961 | 0,903 | 1 |
| IFI27, OAS1, IFIT1, IFI44L, ISG15, HERC6, OLAH, RETN | 0,882 | 0,873 | 0,891 | 0,923 | 0,868 | 0,978 | 0,937 | 0,875 | 0,998 |
| RSAD2, IFI27, OAS1, IFIT1, SIGLEC1, SLC1A2, IL1R2, FAM20A | 0,882 | 0,873 | 0,891 | 0,933 | 0,886 | 0,981 | 0,945 | 0,878 | 1 |
| IFI27, IFIT1, SIGLEC1, ISG15, HERC6, SLC1A2, FAM20A, RETN | 0,882 | 0,872 | 0,891 | 0,930 | 0,878 | 0,981 | 0,941 | 0,869 | 1 |
| RSAD2, IFI27, OAS1, SIGLEC1, ISG15, HERC6, RETN, MMP8 | 0,882 | 0,872 | 0,891 | 0,922 | 0,863 | 0,980 | 0,949 | 0,889 | 1 |
| IFI27, OAS1, HERC6, SLC1A2, IL1R2, OLAH, FAM20A, RETN | 0,882 | 0,872 | 0,891 | 0,932 | 0,883 | 0,981 | 0,916 | 0,830 | 1 |
| IFI27, OAS1, IFIT1, SIGLEC1, HERC6, SLC1A2, OLAH, RETN | 0,882 | 0,872 | 0,891 | 0,931 | 0,883 | 0,979 | 0,941 | 0,879 | 1 |
| IFI27, OAS1, SIGLEC1, ISG15, SLC1A2, IL1R2, FAM20A, RETN | 0,882 | 0,872 | 0,891 | 0,933 | 0,885 | 0,980 | 0,945 | 0,876 | 1 |
| IFI27, IFI44L, ISG15, HERC6, SLC1A2, OLAH, FAM20A, RETN | 0,882 | 0,872 | 0,891 | 0,930 | 0,877 | 0,982 | 0,917 | 0,834 | 1 |

512

| | | | | | | | | | |
|---|---|---|---|---|---|---|---|---|---|
| IFI27, IFI44L, SIGLEC1, HERC6, SLC1A2, OLAH, FAM20A, RETN | 0,882 | 0,872 | 0,892 | 0,930 | 0,877 | 0,983 | 0,946 | 0,878 | 1 |
| IFI27, OAS1, IFI44L, SIGLEC1, ISG15, HERC6, OLAH, MMP8 | 0,882 | 0,873 | 0,890 | 0,926 | 0,875 | 0,977 | 0,941 | 0,886 | 0,996 |
| RSAD2, IFI27, OAS1, IFIT1, SIGLEC1, HERC6, IL1R2, OLAH | 0,882 | 0,873 | 0,890 | 0,924 | 0,875 | 0,974 | 0,942 | 0,890 | 0,995 |
| IFI27, IFIT1, IFI44L, SLC1A2, OLAH, FAM20A, RETN, MMP8 | 0,882 | 0,872 | 0,892 | 0,928 | 0,873 | 0,983 | 0,928 | 0,850 | 1 |
| RSAD2, IFI27, OAS1, SIGLEC1, ISG15, HERC6, IL1R2, RETN | 0,882 | 0,873 | 0,890 | 0,924 | 0,872 | 0,977 | 0,953 | 0,900 | 1 |
| OAS1, IFIT1, SIGLEC1, ISG15, IL1R2, OLAH, FAM20A, MMP8 | 0,882 | 0,873 | 0,891 | 0,920 | 0,863 | 0,977 | 0,966 | 0,913 | 1 |
| RSAD2, IFI27, IFI44L, SIGLEC1, ISG15, SLC1A2, IL1R2, FAM20A | 0,882 | 0,872 | 0,891 | 0,932 | 0,885 | 0,980 | 0,945 | 0,874 | 1 |
| RSAD2, IFI27, IFIT1, IFI44L, IL1R2, FAM20A, RETN, MMP8 | 0,882 | 0,872 | 0,891 | 0,929 | 0,875 | 0,982 | 0,934 | 0,856 | 1 |
| IFI27, IFIT1, SIGLEC1, ISG15, HERC6, IL1R2, OLAH, MMP8 | 0,882 | 0,873 | 0,890 | 0,925 | 0,876 | 0,975 | 0,945 | 0,893 | 0,996 |
| RSAD2, IFI27, OAS1, IFI44L, IL1R2, OLAH, FAM20A, RETN | 0,881 | 0,872 | 0,891 | 0,925 | 0,868 | 0,982 | 0,930 | 0,858 | 1 |
| RSAD2, IFI27, IFI44L, SIGLEC1, SLC1A2, OLAH, FAM20A, RETN | 0,881 | 0,872 | 0,891 | 0,927 | 0,871 | 0,983 | 0,943 | 0,878 | 1 |
| OAS1, IFIT1, IFI44L, SIGLEC1, IL1R2, OLAH, FAM20A, MMP8 | 0,881 | 0,872 | 0,891 | 0,920 | 0,863 | 0,977 | 0,966 | 0,915 | 1 |
| RSAD2, IFI27, IFIT1, ISG15, HERC6, OLAH, RETN, MMP8 | 0,881 | 0,872 | 0,891 | 0,924 | 0,868 | 0,981 | 0,945 | 0,889 | 1 |
| RSAD2, IFI27, ISG15, SLC1A2, IL1R2, OLAH, FAM20A, RETN | 0,881 | 0,872 | 0,891 | 0,930 | 0,880 | 0,981 | 0,916 | 0,833 | 1,000 |
| RSAD2, IFI27, SIGLEC1, HERC6, SLC1A2, FAM20A, RETN, MMP8 | 0,881 | 0,871 | 0,892 | 0,929 | 0,875 | 0,982 | 0,946 | 0,872 | 1 |
| RSAD2, IFI27, OAS1, ISG15, HERC6, FAM20A, RETN, MMP8 | 0,881 | 0,871 | 0,892 | 0,926 | 0,870 | 0,983 | 0,935 | 0,857 | 1 |
| RSAD2, IFI27, OAS1, IFI44L, ISG15, SLC1A2, OLAH, FAM20A | 0,881 | 0,872 | 0,891 | 0,930 | 0,877 | 0,983 | 0,918 | 0,836 | 1 |
| IFI27, OAS1, IFI44L, ISG15, HERC6, IL1R2, OLAH, RETN | 0,881 | 0,873 | 0,890 | 0,922 | 0,867 | 0,977 | 0,938 | 0,877 | 0,999 |
| RSAD2, IFI27, OAS1, IFIT1, IFI44L, OLAH, FAM20A, RETN | 0,881 | 0,872 | 0,891 | 0,922 | 0,861 | 0,982 | 0,936 | 0,867 | 1 |
| IFI27, IFIT1, IFI44L, HERC6, SLC1A2, OLAH, FAM20A, RETN | 0,881 | 0,872 | 0,891 | 0,931 | 0,880 | 0,983 | 0,925 | 0,845 | 1 |
| IFI27, IFI44L, SIGLEC1, ISG15, IL1R2, OLAH, RETN, MMP8 | 0,881 | 0,872 | 0,890 | 0,924 | 0,870 | 0,978 | 0,954 | 0,902 | 1 |
| RSAD2, IFI27, OAS1, IFI44L, ISG15, HERC6, OLAH, RETN | 0,881 | 0,872 | 0,890 | 0,922 | 0,866 | 0,978 | 0,941 | 0,882 | 1 |
| RSAD2, IFI27, SIGLEC1, ISG15, HERC6, IL1R2, OLAH, MMP8 | 0,881 | 0,873 | 0,890 | 0,925 | 0,876 | 0,974 | 0,943 | 0,892 | 0,995 |
| IFI27, OAS1, IFIT1, IFI44L, HERC6, IL1R2, OLAH, RETN | 0,881 | 0,872 | 0,890 | 0,923 | 0,869 | 0,977 | 0,938 | 0,877 | 0,999 |
| IFI27, IFIT1, ISG15, HERC6, SLC1A2, OLAH, FAM20A, RETN | 0,881 | 0,871 | 0,891 | 0,928 | 0,875 | 0,981 | 0,915 | 0,829 | 1 |
| RSAD2, IFI27, OAS1, SIGLEC1, ISG15, HERC6, OLAH, MMP8 | 0,881 | 0,872 | 0,890 | 0,923 | 0,871 | 0,975 | 0,937 | 0,879 | 0,995 |
| IFI27, IFIT1, IFI44L, SIGLEC1, ISG15, HERC6, IL1R2, RETN | 0,881 | 0,873 | 0,890 | 0,924 | 0,871 | 0,977 | 0,945 | 0,893 | 0,996 |
| RSAD2, IFI27, OAS1, SIGLEC1, ISG15, SLC1A2, IL1R2, FAM20A | 0,881 | 0,872 | 0,891 | 0,933 | 0,886 | 0,980 | 0,945 | 0,875 | 1 |

| | | | | | | | | | |
|---|---|---|---|---|---|---|---|---|---|
| IFI27, ISG15, HERC6, SLC1A2, IL1R2, OLAH, FAM20A, RETN | 0,881 | 0,872 | 0,891 | 0,931 | 0,882 | 0,981 | 0,916 | 0,832 | 1 |
| IFI27, OAS1, IFIT1, IFI44L, HERC6, FAM20A, RETN, MMP8 | 0,881 | 0,871 | 0,891 | 0,923 | 0,865 | 0,982 | 0,934 | 0,853 | 1 |
| IFI27, IFI44L, HERC6, SLC1A2, OLAH, FAM20A, RETN, MMP8 | 0,881 | 0,871 | 0,891 | 0,929 | 0,875 | 0,983 | 0,927 | 0,848 | 1 |
| RSAD2, IFI27, IFIT1, SIGLEC1, SLC1A2, IL1R2, FAM20A, RETN | 0,881 | 0,872 | 0,890 | 0,934 | 0,886 | 0,981 | 0,943 | 0,874 | 1 |
| IFI27, OAS1, SLC1A2, IL1R2, OLAH, FAM20A, RETN, MMP8 | 0,881 | 0,872 | 0,891 | 0,933 | 0,884 | 0,983 | 0,923 | 0,843 | 1 |
| RSAD2, IFI27, OAS1, IFI44L, ISG15, OLAH, FAM20A, RETN | 0,881 | 0,872 | 0,891 | 0,922 | 0,862 | 0,981 | 0,935 | 0,867 | 1 |
| RSAD2, IFI27, SIGLEC1, ISG15, HERC6, IL1R2, OLAH, RETN | 0,881 | 0,872 | 0,890 | 0,922 | 0,868 | 0,977 | 0,943 | 0,887 | 1,000 |
| RSAD2, IFI27, OAS1, IFIT1, IFI44L, SIGLEC1, HERC6, OLAH | 0,881 | 0,873 | 0,890 | 0,927 | 0,876 | 0,977 | 0,947 | 0,895 | 0,998 |
| RSAD2, IFI27, OAS1, ISG15, HERC6, IL1R2, OLAH, MMP8 | 0,881 | 0,872 | 0,890 | 0,925 | 0,876 | 0,975 | 0,938 | 0,880 | 0,996 |
| RSAD2, IFI27, OAS1, IFIT1, ISG15, OLAH, FAM20A, RETN | 0,881 | 0,872 | 0,891 | 0,920 | 0,860 | 0,980 | 0,934 | 0,866 | 1 |
| RSAD2, IFI27, IFIT1, IFI44L, SIGLEC1, HERC6, OLAH, MMP8 | 0,881 | 0,873 | 0,890 | 0,924 | 0,871 | 0,977 | 0,947 | 0,897 | 0,997 |
| IFI27, IFIT1, IFI44L, HERC6, IL1R2, OLAH, RETN, MMP8 | 0,881 | 0,872 | 0,890 | 0,922 | 0,864 | 0,979 | 0,947 | 0,892 | 1 |
| IFIT1, IFI44L, SIGLEC1, ISG15, SLC1A2, OLAH, FAM20A, MMP8 | 0,881 | 0,872 | 0,890 | 0,924 | 0,872 | 0,977 | 0,941 | 0,877 | 1 |
| RSAD2, IFI27, IFIT1, IFI44L, ISG15, OLAH, FAM20A, RETN | 0,881 | 0,872 | 0,891 | 0,920 | 0,860 | 0,981 | 0,935 | 0,865 | 1 |
| RSAD2, IFI27, SIGLEC1, HERC6, SLC1A2, OLAH, RETN, MMP8 | 0,881 | 0,872 | 0,891 | 0,932 | 0,883 | 0,981 | 0,952 | 0,900 | 1 |
| RSAD2, IFI27, OAS1, IFIT1, IFI44L, SLC1A2, OLAH, FAM20A | 0,881 | 0,872 | 0,891 | 0,929 | 0,874 | 0,983 | 0,925 | 0,847 | 1 |
| RSAD2, IFI27, OAS1, IFI44L, HERC6, IL1R2, OLAH, RETN | 0,881 | 0,872 | 0,890 | 0,923 | 0,867 | 0,978 | 0,939 | 0,879 | 0,999 |
| IFI27, SIGLEC1, ISG15, HERC6, IL1R2, OLAH, RETN, MMP8 | 0,881 | 0,872 | 0,890 | 0,922 | 0,865 | 0,978 | 0,958 | 0,909 | 1 |
| RSAD2, IFI27, SIGLEC1, ISG15, SLC1A2, IL1R2, FAM20A, RETN | 0,881 | 0,871 | 0,891 | 0,933 | 0,886 | 0,980 | 0,943 | 0,873 | 1 |
| RSAD2, IFI27, OAS1, IFI44L, SIGLEC1, ISG15, HERC6, OLAH | 0,881 | 0,872 | 0,890 | 0,926 | 0,874 | 0,978 | 0,943 | 0,890 | 0,997 |
| IFI27, OAS1, IFIT1, HERC6, SLC1A2, OLAH, RETN, MMP8 | 0,881 | 0,871 | 0,891 | 0,929 | 0,878 | 0,979 | 0,940 | 0,876 | 1 |
| IFIT1, IFI44L, SIGLEC1, ISG15, HERC6, IL1R2, OLAH, FAM20A | 0,881 | 0,872 | 0,890 | 0,918 | 0,860 | 0,976 | 0,951 | 0,894 | 1 |
| RSAD2, IFI27, IFIT1, SIGLEC1, HERC6, IL1R2, OLAH, MMP8 | 0,881 | 0,872 | 0,890 | 0,926 | 0,877 | 0,975 | 0,942 | 0,890 | 0,995 |
| RSAD2, IFI27, IFI44L, SIGLEC1, HERC6, IL1R2, RETN, MMP8 | 0,881 | 0,872 | 0,890 | 0,924 | 0,872 | 0,977 | 0,952 | 0,906 | 0,998 |
| IFIT1, IFI44L, SIGLEC1, ISG15, IL1R2, OLAH, FAM20A, MMP8 | 0,881 | 0,872 | 0,890 | 0,920 | 0,864 | 0,976 | 0,965 | 0,914 | 1 |
| IFI27, OAS1, IFIT1, SIGLEC1, IL1R2, OLAH, RETN, MMP8 | 0,881 | 0,872 | 0,890 | 0,921 | 0,865 | 0,978 | 0,963 | 0,914 | 1 |
| IFI27, IFIT1, ISG15, HERC6, IL1R2, OLAH, RETN, MMP8 | 0,881 | 0,872 | 0,890 | 0,922 | 0,865 | 0,979 | 0,947 | 0,892 | 1 |
| RSAD2, IFI27, IFIT1, IFI44L, SIGLEC1, HERC6, IL1R2, RETN | 0,881 | 0,873 | 0,889 | 0,923 | 0,870 | 0,976 | 0,945 | 0,893 | 0,996 |

| | | | | | | | | |
|---|---|---|---|---|---|---|---|---|
| RSAD2, IFI27, OAS1, IFI44L, SIGLEC1, FAM20A, RETN, MMP8 | 0,881 | 0,870 | 0,891 | 0,925 | 0,867 | 0,983 | 0,948 | 0,876 | 1 |
| RSAD2, IFI27, IFI44L, HERC6, SLC1A2, OLAH, FAM20A, RETN | 0,881 | 0,871 | 0,891 | 0,931 | 0,881 | 0,982 | 0,922 | 0,842 | 1 |
| RSAD2, IFI27, OAS1, SIGLEC1, HERC6, SLC1A2, OLAH, RETN | 0,881 | 0,872 | 0,890 | 0,929 | 0,878 | 0,980 | 0,945 | 0,883 | 1 |
| IFI27, OAS1, IFIT1, IFI44L, ISG15, IL1R2, FAM20A, MMP8 | 0,881 | 0,871 | 0,891 | 0,926 | 0,873 | 0,978 | 0,933 | 0,854 | 1 |
| IFI27, IFIT1, IFI44L, SIGLEC1, ISG15, IL1R2, OLAH, MMP8 | 0,881 | 0,872 | 0,889 | 0,924 | 0,875 | 0,974 | 0,945 | 0,893 | 0,996 |
| IFI27, OAS1, IFIT1, IFI44L, SIGLEC1, OLAH, RETN, MMP8 | 0,881 | 0,872 | 0,890 | 0,921 | 0,863 | 0,979 | 0,955 | 0,899 | 1 |
| RSAD2, IFI27, OAS1, SIGLEC1, SLC1A2, IL1R2, FAM20A, RETN | 0,881 | 0,871 | 0,891 | 0,930 | 0,881 | 0,980 | 0,942 | 0,871 | 1 |
| IFI27, OAS1, SIGLEC1, HERC6, SLC1A2, IL1R2, OLAH, MMP8 | 0,881 | 0,872 | 0,890 | 0,929 | 0,881 | 0,977 | 0,943 | 0,887 | 1 |
| IFI27, OAS1, IFIT1, ISG15, HERC6, FAM20A, RETN, MMP8 | 0,881 | 0,871 | 0,891 | 0,926 | 0,869 | 0,982 | 0,935 | 0,857 | 1 |
| IFI44L, SIGLEC1, ISG15, HERC6, IL1R2, OLAH, FAM20A, MMP8 | 0,881 | 0,872 | 0,890 | 0,916 | 0,858 | 0,974 | 0,961 | 0,903 | 1 |
| RSAD2, IFI27, IFI44L, ISG15, HERC6, OLAH, RETN, MMP8 | 0,881 | 0,872 | 0,890 | 0,923 | 0,866 | 0,980 | 0,943 | 0,887 | 1,000 |
| RSAD2, IFI27, OAS1, IFI44L, HERC6, IL1R2, OLAH, MMP8 | 0,881 | 0,872 | 0,890 | 0,928 | 0,879 | 0,977 | 0,935 | 0,875 | 0,995 |
| IFI27, IFIT1, ISG15, SLC1A2, IL1R2, OLAH, FAM20A, RETN | 0,881 | 0,871 | 0,890 | 0,929 | 0,878 | 0,980 | 0,920 | 0,839 | 1 |
| IFI27, OAS1, IFIT1, ISG15, SLC1A2, IL1R2, OLAH, FAM20A | 0,881 | 0,871 | 0,890 | 0,929 | 0,877 | 0,980 | 0,921 | 0,841 | 1 |
| RSAD2, IFI27, IFIT1, ISG15, SLC1A2, IL1R2, OLAH, FAM20A | 0,881 | 0,872 | 0,890 | 0,929 | 0,878 | 0,980 | 0,918 | 0,837 | 1,000 |
| IFI27, IFI44L, SIGLEC1, ISG15, SLC1A2, IL1R2, OLAH, MMP8 | 0,881 | 0,872 | 0,889 | 0,930 | 0,882 | 0,978 | 0,946 | 0,892 | 0,999 |
| RSAD2, IFI27, ISG15, SLC1A2, OLAH, FAM20A, RETN, MMP8 | 0,881 | 0,871 | 0,891 | 0,928 | 0,874 | 0,982 | 0,924 | 0,843 | 1 |
| RSAD2, IFI27, OAS1, IFIT1, ISG15, SLC1A2, OLAH, FAM20A | 0,881 | 0,871 | 0,890 | 0,925 | 0,870 | 0,980 | 0,923 | 0,843 | 1 |
| IFI27, IFIT1, IFI44L, SIGLEC1, IL1R2, OLAH, RETN, MMP8 | 0,881 | 0,872 | 0,890 | 0,924 | 0,870 | 0,979 | 0,955 | 0,904 | 1 |
| IFI27, IFI44L, SIGLEC1, HERC6, SLC1A2, IL1R2, FAM20A, RETN | 0,881 | 0,871 | 0,890 | 0,931 | 0,882 | 0,980 | 0,948 | 0,879 | 1 |
| RSAD2, IFI27, IFI44L, SIGLEC1, ISG15, HERC6, OLAH, MMP8 | 0,881 | 0,872 | 0,889 | 0,925 | 0,873 | 0,978 | 0,946 | 0,894 | 0,998 |
| IFI27, IFIT1, ISG15, SLC1A2, OLAH, FAM20A, RETN, MMP8 | 0,881 | 0,870 | 0,891 | 0,927 | 0,872 | 0,981 | 0,925 | 0,849 | 1 |
| RSAD2, IFI27, IFIT1, IFI44L, IL1R2, OLAH, FAM20A, RETN | 0,881 | 0,871 | 0,890 | 0,925 | 0,868 | 0,982 | 0,932 | 0,860 | 1 |
| IFI27, OAS1, IFI44L, ISG15, HERC6, IL1R2, OLAH, MMP8 | 0,881 | 0,872 | 0,889 | 0,927 | 0,878 | 0,976 | 0,934 | 0,874 | 0,993 |
| IFI27, OAS1, IFI44L, ISG15, SLC1A2, OLAH, FAM20A, RETN | 0,881 | 0,871 | 0,890 | 0,930 | 0,877 | 0,983 | 0,917 | 0,835 | 1,000 |
| RSAD2, IFI27, OAS1, IFIT1, IFI44L, SIGLEC1, ISG15, FAM20A | 0,881 | 0,872 | 0,890 | 0,915 | 0,850 | 0,979 | 0,943 | 0,873 | 1 |
| IFI27, OAS1, SIGLEC1, ISG15, HERC6, SLC1A2, OLAH, MMP8 | 0,881 | 0,871 | 0,890 | 0,930 | 0,881 | 0,980 | 0,937 | 0,877 | 0,997 |
| RSAD2, IFI27, OAS1, IFIT1, HERC6, IL1R2, OLAH, MMP8 | 0,881 | 0,872 | 0,890 | 0,925 | 0,876 | 0,974 | 0,939 | 0,882 | 0,996 |

| | | | | | | | | |
|---|---|---|---|---|---|---|---|---|
| RSAD2, IFI27, IFIT1, SLC1A2, OLAH, FAM20A, RETN, MMP8 | 0,881 | 0,871 | 0,890 | 0,926 | 0,871 | 0,981 | 0,927 | 0,852 | 1 |
| RSAD2, IFIT1, SIGLEC1, HERC6, IL1R2, FAM20A, RETN, MMP8 | 0,881 | 0,872 | 0,889 | 0,923 | 0,868 | 0,979 | 0,960 | 0,900 | 1 |
| IFI27, IFIT1, IFI44L, SIGLEC1, ISG15, SLC1A2, FAM20A, RETN | 0,881 | 0,871 | 0,890 | 0,925 | 0,870 | 0,980 | 0,938 | 0,862 | 1 |
| IFI27, OAS1, IFIT1, SIGLEC1, ISG15, HERC6, SLC1A2, OLAH | 0,881 | 0,871 | 0,890 | 0,930 | 0,880 | 0,979 | 0,937 | 0,877 | 0,997 |
| RSAD2, IFI27, OAS1, IFI44L, SIGLEC1, HERC6, RETN, MMP8 | 0,881 | 0,871 | 0,890 | 0,919 | 0,861 | 0,978 | 0,941 | 0,875 | 1 |
| RSAD2, IFI27, IFIT1, HERC6, SLC1A2, OLAH, FAM20A, RETN | 0,881 | 0,871 | 0,890 | 0,930 | 0,879 | 0,982 | 0,918 | 0,834 | 1 |
| RSAD2, IFI27, IFIT1, IFI44L, ISG15, IL1R2, FAM20A, MMP8 | 0,881 | 0,871 | 0,890 | 0,926 | 0,873 | 0,978 | 0,933 | 0,854 | 1 |
| RSAD2, IFI27, IFIT1, SIGLEC1, HERC6, IL1R2, RETN, MMP8 | 0,880 | 0,872 | 0,889 | 0,924 | 0,871 | 0,976 | 0,959 | 0,916 | 1 |
| IFI27, OAS1, IFI44L, SIGLEC1, ISG15, FAM20A, RETN, MMP8 | 0,880 | 0,870 | 0,891 | 0,923 | 0,864 | 0,982 | 0,947 | 0,875 | 1 |
| IFI27, OAS1, SIGLEC1, ISG15, HERC6, SLC1A2, OLAH, RETN | 0,880 | 0,871 | 0,890 | 0,928 | 0,877 | 0,978 | 0,938 | 0,874 | 1 |
| IFI27, OAS1, IFI44L, SIGLEC1, HERC6, SLC1A2, FAM20A, RETN | 0,880 | 0,871 | 0,890 | 0,928 | 0,875 | 0,980 | 0,938 | 0,864 | 1 |
| IFI27, OAS1, IFIT1, SLC1A2, OLAH, FAM20A, RETN, MMP8 | 0,880 | 0,870 | 0,891 | 0,926 | 0,870 | 0,981 | 0,927 | 0,852 | 1 |
| RSAD2, IFI27, IFIT1, ISG15, IL1R2, FAM20A, RETN, MMP8 | 0,880 | 0,871 | 0,890 | 0,930 | 0,877 | 0,982 | 0,934 | 0,856 | 1 |
| RSAD2, OAS1, IFI44L, SIGLEC1, HERC6, IL1R2, FAM20A, MMP8 | 0,880 | 0,871 | 0,890 | 0,919 | 0,862 | 0,975 | 0,960 | 0,900 | 1 |
| IFI27, OAS1, SIGLEC1, HERC6, SLC1A2, IL1R2, OLAH, RETN | 0,880 | 0,871 | 0,890 | 0,928 | 0,877 | 0,979 | 0,942 | 0,880 | 1 |
| RSAD2, IFI27, OAS1, IFIT1, ISG15, IL1R2, FAM20A, MMP8 | 0,880 | 0,871 | 0,890 | 0,926 | 0,873 | 0,978 | 0,934 | 0,856 | 1 |
| IFIT1, IFI44L, SIGLEC1, HERC6, SLC1A2, OLAH, FAM20A, MMP8 | 0,880 | 0,870 | 0,891 | 0,929 | 0,880 | 0,979 | 0,941 | 0,872 | 1 |
| IFI27, OAS1, IFIT1, ISG15, SLC1A2, OLAH, FAM20A, RETN | 0,880 | 0,871 | 0,890 | 0,925 | 0,869 | 0,981 | 0,925 | 0,849 | 1 |
| IFI27, IFI44L, SIGLEC1, ISG15, HERC6, IL1R2, RETN, MMP8 | 0,880 | 0,872 | 0,889 | 0,924 | 0,871 | 0,977 | 0,951 | 0,905 | 0,998 |
| IFI27, OAS1, IFI44L, SIGLEC1, IL1R2, OLAH, RETN, MMP8 | 0,880 | 0,871 | 0,889 | 0,924 | 0,869 | 0,979 | 0,958 | 0,908 | 1 |
| IFI27, OAS1, IFIT1, IFI44L, SIGLEC1, IL1R2, OLAH, MMP8 | 0,880 | 0,872 | 0,889 | 0,924 | 0,874 | 0,974 | 0,949 | 0,900 | 0,998 |
| IFIT1, IFI44L, SIGLEC1, HERC6, OLAH, FAM20A, RETN, MMP8 | 0,880 | 0,870 | 0,890 | 0,922 | 0,864 | 0,980 | 0,952 | 0,895 | 1 |
| IFI27, OAS1, ISG15, SLC1A2, IL1R2, OLAH, FAM20A, RETN | 0,880 | 0,871 | 0,890 | 0,929 | 0,878 | 0,980 | 0,916 | 0,834 | 0,999 |
| RSAD2, IFI27, IFIT1, IFI44L, SLC1A2, IL1R2, OLAH, FAM20A | 0,880 | 0,871 | 0,889 | 0,926 | 0,872 | 0,981 | 0,922 | 0,841 | 1 |
| RSAD2, IFI27, IFI44L, ISG15, HERC6, IL1R2, OLAH, RETN | 0,880 | 0,872 | 0,889 | 0,924 | 0,871 | 0,978 | 0,937 | 0,878 | 0,996 |
| IFIT1, IFI44L, SIGLEC1, SLC1A2, IL1R2, OLAH, FAM20A, MMP8 | 0,880 | 0,872 | 0,889 | 0,928 | 0,880 | 0,976 | 0,963 | 0,908 | 1 |
| IFI27, OAS1, IFIT1, IFI44L, SLC1A2, IL1R2, OLAH, FAM20A | 0,880 | 0,871 | 0,890 | 0,930 | 0,876 | 0,983 | 0,921 | 0,839 | 1 |
| OAS1, IFIT1, IFI44L, SIGLEC1, HERC6, IL1R2, OLAH, FAM20A | 0,880 | 0,871 | 0,889 | 0,916 | 0,858 | 0,975 | 0,951 | 0,894 | 1 |

| | | | | | | | | | |
|---|---|---|---|---|---|---|---|---|---|
| OAS1, IFIT1, SIGLEC1, HERC6, SLC1A2, IL1R2, FAM20A, MMP8 | 0,880 | 0,871 | 0,890 | 0,928 | 0,882 | 0,974 | 0,959 | 0,895 | 1 |
| RSAD2, IFI27, OAS1, SLC1A2, OLAH, FAM20A, RETN, MMP8 | 0,880 | 0,870 | 0,890 | 0,927 | 0,872 | 0,982 | 0,924 | 0,844 | 1 |
| IFI27, OAS1, SIGLEC1, ISG15, HERC6, SLC1A2, IL1R2, OLAH | 0,880 | 0,871 | 0,890 | 0,928 | 0,879 | 0,978 | 0,939 | 0,880 | 0,998 |
| IFI27, IFIT1, IFI44L, ISG15, HERC6, FAM20A, RETN, MMP8 | 0,880 | 0,870 | 0,890 | 0,921 | 0,863 | 0,980 | 0,935 | 0,856 | 1 |
| IFI27, OAS1, SIGLEC1, ISG15, HERC6, SLC1A2, FAM20A, RETN | 0,880 | 0,870 | 0,890 | 0,927 | 0,874 | 0,980 | 0,937 | 0,863 | 1 |
| RSAD2, IFIT1, IFI44L, SIGLEC1, IL1R2, OLAH, FAM20A, MMP8 | 0,880 | 0,871 | 0,889 | 0,921 | 0,864 | 0,978 | 0,966 | 0,915 | 1 |
| RSAD2, IFI27, OAS1, IFI44L, ISG15, IL1R2, FAM20A, MMP8 | 0,880 | 0,870 | 0,890 | 0,925 | 0,872 | 0,978 | 0,930 | 0,847 | 1 |
| IFI27, IFIT1, IFI44L, SLC1A2, IL1R2, OLAH, FAM20A, RETN | 0,880 | 0,871 | 0,889 | 0,928 | 0,874 | 0,982 | 0,922 | 0,841 | 1 |
| IFI27, OAS1, IFI44L, SIGLEC1, ISG15, IL1R2, OLAH, MMP8 | 0,880 | 0,872 | 0,888 | 0,925 | 0,876 | 0,975 | 0,941 | 0,888 | 0,995 |
| RSAD2, IFI27, OAS1, HERC6, SLC1A2, OLAH, RETN, MMP8 | 0,880 | 0,871 | 0,890 | 0,930 | 0,880 | 0,981 | 0,941 | 0,877 | 1 |
| IFI27, OAS1, IFIT1, SIGLEC1, SLC1A2, FAM20A, RETN, MMP8 | 0,880 | 0,870 | 0,890 | 0,927 | 0,872 | 0,982 | 0,942 | 0,866 | 1 |
| RSAD2, IFI27, OAS1, IFI44L, SIGLEC1, ISG15, FAM20A, RETN | 0,880 | 0,870 | 0,890 | 0,918 | 0,858 | 0,978 | 0,947 | 0,879 | 1 |
| OAS1, IFIT1, SIGLEC1, HERC6, OLAH, FAM20A, RETN, MMP8 | 0,880 | 0,871 | 0,889 | 0,920 | 0,862 | 0,979 | 0,952 | 0,895 | 1 |
| IFI27, OAS1, HERC6, SLC1A2, IL1R2, FAM20A, RETN, MMP8 | 0,880 | 0,871 | 0,889 | 0,935 | 0,887 | 0,982 | 0,924 | 0,837 | 1 |
| RSAD2, IFI27, IFIT1, ISG15, HERC6, IL1R2, OLAH, RETN | 0,880 | 0,871 | 0,889 | 0,922 | 0,867 | 0,977 | 0,937 | 0,876 | 0,998 |
| RSAD2, IFI27, OAS1, IFIT1, IFI44L, HERC6, OLAH, MMP8 | 0,880 | 0,871 | 0,889 | 0,925 | 0,873 | 0,976 | 0,935 | 0,876 | 0,994 |
| RSAD2, IFIT1, SIGLEC1, ISG15, IL1R2, OLAH, FAM20A, MMP8 | 0,880 | 0,871 | 0,889 | 0,919 | 0,862 | 0,976 | 0,967 | 0,916 | 1 |
| IFI27, OAS1, HERC6, SLC1A2, IL1R2, OLAH, RETN, MMP8 | 0,880 | 0,871 | 0,889 | 0,928 | 0,878 | 0,979 | 0,938 | 0,874 | 1 |
| IFI27, OAS1, IFIT1, IFI44L, SIGLEC1, HERC6, SLC1A2, FAM20A | 0,880 | 0,871 | 0,889 | 0,930 | 0,881 | 0,979 | 0,937 | 0,862 | 1 |
| RSAD2, IFI27, OAS1, IFIT1, SIGLEC1, ISG15, HERC6, OLAH | 0,880 | 0,871 | 0,889 | 0,924 | 0,873 | 0,976 | 0,938 | 0,881 | 0,995 |
| IFI27, OAS1, IFI44L, ISG15, IL1R2, FAM20A, RETN, MMP8 | 0,880 | 0,870 | 0,890 | 0,929 | 0,877 | 0,982 | 0,935 | 0,856 | 1 |
| RSAD2, IFI27, OAS1, IFIT1, SLC1A2, IL1R2, OLAH, FAM20A | 0,880 | 0,871 | 0,889 | 0,927 | 0,873 | 0,981 | 0,922 | 0,840 | 1 |
| IFI27, OAS1, IFIT1, SIGLEC1, HERC6, SLC1A2, OLAH, MMP8 | 0,880 | 0,870 | 0,890 | 0,928 | 0,878 | 0,978 | 0,939 | 0,880 | 0,999 |
| RSAD2, IFI27, IFI44L, SIGLEC1, SLC1A2, IL1R2, FAM20A, RETN | 0,880 | 0,870 | 0,890 | 0,931 | 0,881 | 0,981 | 0,945 | 0,875 | 1 |
| IFI27, IFIT1, SIGLEC1, ISG15, IL1R2, OLAH, RETN, MMP8 | 0,880 | 0,871 | 0,889 | 0,922 | 0,866 | 0,978 | 0,960 | 0,911 | 1 |
| IFIT1, SIGLEC1, HERC6, SLC1A2, IL1R2, OLAH, FAM20A, MMP8 | 0,880 | 0,870 | 0,890 | 0,927 | 0,879 | 0,974 | 0,960 | 0,896 | 1 |
| RSAD2, IFI27, IFI44L, SIGLEC1, ISG15, FAM20A, RETN, MMP8 | 0,880 | 0,869 | 0,890 | 0,924 | 0,866 | 0,982 | 0,947 | 0,875 | 1 |
| IFI27, OAS1, IFIT1, IFI44L, SLC1A2, OLAH, FAM20A, RETN | 0,880 | 0,870 | 0,889 | 0,927 | 0,870 | 0,983 | 0,923 | 0,843 | 1 |

| | | | | | | | | |
|---|---|---|---|---|---|---|---|---|
| RSAD2, IFI27, IFIT1, SIGLEC1, ISG15, HERC6, IL1R2, OLAH | 0,880 | 0,871 | 0,888 | 0,925 | 0,875 | 0,975 | 0,939 | 0,885 | 0,993 |
| OAS1, IFIT1, SIGLEC1, IL1R2, OLAH, FAM20A, RETN, MMP8 | 0,880 | 0,871 | 0,889 | 0,921 | 0,864 | 0,978 | 0,967 | 0,916 | 1 |
| RSAD2, IFI27, OAS1, SIGLEC1, HERC6, SLC1A2, OLAH, MMP8 | 0,880 | 0,870 | 0,889 | 0,929 | 0,879 | 0,979 | 0,941 | 0,882 | 1 |
| RSAD2, IFI27, IFI44L, SLC1A2, OLAH, FAM20A, RETN, MMP8 | 0,880 | 0,870 | 0,890 | 0,929 | 0,875 | 0,983 | 0,928 | 0,853 | 1 |
| RSAD2, IFI27, OAS1, IFI44L, SIGLEC1, SLC1A2, IL1R2, FAM20A | 0,880 | 0,870 | 0,890 | 0,931 | 0,882 | 0,979 | 0,943 | 0,873 | 1 |
| RSAD2, IFI27, OAS1, IFIT1, HERC6, IL1R2, RETN, MMP8 | 0,880 | 0,871 | 0,889 | 0,923 | 0,868 | 0,978 | 0,945 | 0,886 | 1 |
| IFI27, OAS1, IFIT1, IFI44L, ISG15, IL1R2, FAM20A, RETN | 0,880 | 0,871 | 0,889 | 0,924 | 0,871 | 0,977 | 0,930 | 0,852 | 1 |
| IFI27, OAS1, IFI44L, SIGLEC1, HERC6, SLC1A2, OLAH, RETN | 0,880 | 0,870 | 0,889 | 0,931 | 0,884 | 0,979 | 0,938 | 0,874 | 1 |
| IFI27, IFIT1, SIGLEC1, ISG15, SLC1A2, IL1R2, OLAH, MMP8 | 0,880 | 0,871 | 0,888 | 0,930 | 0,881 | 0,979 | 0,942 | 0,887 | 0,998 |
| RSAD2, IFI27, OAS1, IFI44L, ISG15, HERC6, OLAH, MMP8 | 0,880 | 0,871 | 0,889 | 0,924 | 0,872 | 0,976 | 0,935 | 0,875 | 0,994 |
| RSAD2, IFI27, IFI44L, ISG15, SLC1A2, OLAH, FAM20A, RETN | 0,880 | 0,870 | 0,889 | 0,929 | 0,875 | 0,982 | 0,916 | 0,834 | 0,999 |
| RSAD2, OAS1, IFIT1, IFI44L, SIGLEC1, IL1R2, FAM20A, MMP8 | 0,880 | 0,871 | 0,889 | 0,920 | 0,863 | 0,977 | 0,964 | 0,910 | 1 |
| IFI27, OAS1, IFIT1, SIGLEC1, HERC6, SLC1A2, IL1R2, OLAH | 0,880 | 0,870 | 0,889 | 0,928 | 0,879 | 0,977 | 0,939 | 0,879 | 0,999 |
| IFI27, OAS1, IFIT1, IFI44L, SIGLEC1, HERC6, IL1R2, MMP8 | 0,880 | 0,872 | 0,888 | 0,924 | 0,878 | 0,970 | 0,940 | 0,888 | 0,993 |
| IFI27, OAS1, IFIT1, IFI44L, HERC6, IL1R2, OLAH, MMP8 | 0,880 | 0,871 | 0,888 | 0,926 | 0,876 | 0,975 | 0,941 | 0,887 | 0,995 |
| IFI27, IFIT1, HERC6, SLC1A2, IL1R2, OLAH, FAM20A, RETN | 0,880 | 0,870 | 0,890 | 0,929 | 0,877 | 0,981 | 0,922 | 0,839 | 1 |
| RSAD2, IFI27, IFIT1, IFI44L, ISG15, HERC6, OLAH, RETN | 0,880 | 0,871 | 0,888 | 0,922 | 0,867 | 0,977 | 0,936 | 0,876 | 0,996 |
| IFI27, OAS1, SIGLEC1, HERC6, SLC1A2, IL1R2, RETN, MMP8 | 0,880 | 0,871 | 0,889 | 0,928 | 0,881 | 0,976 | 0,955 | 0,903 | 1 |
| IFI27, OAS1, IFIT1, IFI44L, SIGLEC1, SLC1A2, FAM20A, RETN | 0,880 | 0,870 | 0,889 | 0,926 | 0,871 | 0,981 | 0,940 | 0,868 | 1 |
| RSAD2, IFI27, IFI44L, ISG15, HERC6, IL1R2, OLAH, MMP8 | 0,880 | 0,871 | 0,888 | 0,926 | 0,877 | 0,976 | 0,937 | 0,879 | 0,995 |
| RSAD2, IFI27, OAS1, IFI44L, SIGLEC1, IL1R2, OLAH, MMP8 | 0,880 | 0,871 | 0,888 | 0,924 | 0,873 | 0,974 | 0,948 | 0,898 | 0,998 |
| RSAD2, IFI27, OAS1, IFI44L, ISG15, HERC6, IL1R2, OLAH | 0,880 | 0,871 | 0,888 | 0,927 | 0,877 | 0,976 | 0,937 | 0,879 | 0,995 |
| IFI27, IFIT1, IFI44L, SIGLEC1, SLC1A2, FAM20A, RETN, MMP8 | 0,880 | 0,870 | 0,889 | 0,926 | 0,870 | 0,981 | 0,945 | 0,868 | 1 |
| IFI27, OAS1, IFIT1, SIGLEC1, ISG15, OLAH, RETN, MMP8 | 0,880 | 0,870 | 0,889 | 0,920 | 0,861 | 0,979 | 0,961 | 0,910 | 1 |
| RSAD2, IFI27, OAS1, IFIT1, IFI44L, ISG15, HERC6, RETN | 0,880 | 0,871 | 0,889 | 0,919 | 0,859 | 0,978 | 0,934 | 0,865 | 1 |
| IFI27, IFI44L, SIGLEC1, HERC6, SLC1A2, OLAH, RETN, MMP8 | 0,880 | 0,870 | 0,889 | 0,926 | 0,873 | 0,979 | 0,957 | 0,908 | 1 |
| IFI27, SIGLEC1, HERC6, SLC1A2, IL1R2, OLAH, FAM20A, RETN | 0,880 | 0,870 | 0,890 | 0,927 | 0,873 | 0,982 | 0,943 | 0,875 | 1 |
| IFIT1, SIGLEC1, ISG15, HERC6, OLAH, FAM20A, RETN, MMP8 | 0,880 | 0,870 | 0,889 | 0,922 | 0,864 | 0,980 | 0,954 | 0,898 | 1 |

| | | | | | | | | |
|---|---|---|---|---|---|---|---|---|
| RSAD2, IFI27, IFI44L, SIGLEC1, ISG15, IL1R2, OLAH, MMP8 | 0,880 | 0,871 | 0,888 | 0,927 | 0,877 | 0,976 | 0,941 | 0,888 | 0,995 |
| RSAD2, IFI27, IFIT1, IFI44L, HERC6, IL1R2, OLAH, MMP8 | 0,880 | 0,871 | 0,888 | 0,927 | 0,878 | 0,976 | 0,937 | 0,881 | 0,993 |
| RSAD2, IFI27, OAS1, IFIT1, SIGLEC1, HERC6, SLC1A2, RETN | 0,880 | 0,871 | 0,888 | 0,924 | 0,870 | 0,979 | 0,942 | 0,880 | 1 |
| IFI27, OAS1, ISG15, SLC1A2, OLAH, FAM20A, RETN, MMP8 | 0,880 | 0,869 | 0,890 | 0,929 | 0,876 | 0,982 | 0,922 | 0,842 | 1 |
| RSAD2, OAS1, SIGLEC1, ISG15, HERC6, IL1R2, FAM20A, MMP8 | 0,880 | 0,870 | 0,889 | 0,918 | 0,861 | 0,975 | 0,961 | 0,902 | 1 |
| IFI27, IFIT1, IFI44L, SIGLEC1, ISG15, SLC1A2, OLAH, MMP8 | 0,880 | 0,871 | 0,888 | 0,930 | 0,881 | 0,980 | 0,942 | 0,884 | 1 |
| IFI27, OAS1, IFIT1, HERC6, SLC1A2, IL1R2, FAM20A, MMP8 | 0,880 | 0,870 | 0,889 | 0,930 | 0,883 | 0,976 | 0,927 | 0,842 | 1 |
| RSAD2, IFI27, SIGLEC1, ISG15, HERC6, IL1R2, RETN, MMP8 | 0,880 | 0,871 | 0,888 | 0,924 | 0,871 | 0,977 | 0,961 | 0,920 | 1 |
| IFI27, OAS1, IFIT1, ISG15, HERC6, IL1R2, OLAH, MMP8 | 0,880 | 0,871 | 0,888 | 0,923 | 0,872 | 0,973 | 0,939 | 0,882 | 0,996 |
| RSAD2, IFI27, OAS1, IFIT1, ISG15, HERC6, IL1R2, RETN | 0,880 | 0,871 | 0,888 | 0,923 | 0,869 | 0,976 | 0,942 | 0,881 | 1 |
| IFIT1, IFI44L, SIGLEC1, ISG15, HERC6, OLAH, FAM20A, MMP8 | 0,880 | 0,869 | 0,890 | 0,917 | 0,858 | 0,976 | 0,951 | 0,895 | 1 |
| RSAD2, IFI27, OAS1, IFI44L, SLC1A2, IL1R2, OLAH, FAM20A | 0,880 | 0,870 | 0,889 | 0,930 | 0,876 | 0,983 | 0,920 | 0,837 | 1 |
| IFI27, IFI44L, ISG15, SLC1A2, IL1R2, FAM20A, RETN, MMP8 | 0,880 | 0,870 | 0,889 | 0,931 | 0,883 | 0,979 | 0,923 | 0,835 | 1 |
| RSAD2, IFI27, OAS1, IFIT1, IFI44L, HERC6, IL1R2, OLAH | 0,880 | 0,871 | 0,888 | 0,925 | 0,875 | 0,975 | 0,940 | 0,884 | 0,996 |
| IFI27, IFIT1, SIGLEC1, ISG15, SLC1A2, FAM20A, RETN, MMP8 | 0,880 | 0,870 | 0,889 | 0,926 | 0,870 | 0,981 | 0,942 | 0,865 | 1 |
| RSAD2, IFI27, IFIT1, IFI44L, SIGLEC1, IL1R2, OLAH, MMP8 | 0,879 | 0,871 | 0,888 | 0,924 | 0,874 | 0,975 | 0,949 | 0,900 | 0,998 |
| IFI27, OAS1, IFIT1, IFI44L, SIGLEC1, ISG15, IL1R2, OLAH | 0,879 | 0,871 | 0,888 | 0,924 | 0,874 | 0,974 | 0,941 | 0,888 | 0,995 |
| RSAD2, IFI27, IFI44L, SIGLEC1, IL1R2, OLAH, RETN, MMP8 | 0,879 | 0,870 | 0,889 | 0,924 | 0,869 | 0,978 | 0,957 | 0,906 | 1 |
| OAS1, IFIT1, IFI44L, SIGLEC1, ISG15, HERC6, IL1R2, FAM20A | 0,879 | 0,870 | 0,889 | 0,919 | 0,863 | 0,976 | 0,955 | 0,900 | 1 |
| IFI27, OAS1, IFIT1, SIGLEC1, HERC6, SLC1A2, FAM20A, MMP8 | 0,879 | 0,870 | 0,889 | 0,930 | 0,882 | 0,979 | 0,936 | 0,860 | 1 |
| IFI27, OAS1, IFIT1, SLC1A2, IL1R2, OLAH, FAM20A, RETN | 0,879 | 0,870 | 0,889 | 0,927 | 0,874 | 0,981 | 0,922 | 0,840 | 1 |
| IFI27, IFIT1, IFI44L, SIGLEC1, HERC6, SLC1A2, FAM20A, MMP8 | 0,879 | 0,871 | 0,888 | 0,926 | 0,874 | 0,978 | 0,938 | 0,860 | 1 |
| RSAD2, OAS1, IFIT1, SIGLEC1, ISG15, IL1R2, FAM20A, MMP8 | 0,879 | 0,870 | 0,888 | 0,919 | 0,862 | 0,977 | 0,967 | 0,914 | 1 |
| RSAD2, IFI27, OAS1, IFI44L, IL1R2, FAM20A, RETN, MMP8 | 0,879 | 0,869 | 0,890 | 0,929 | 0,877 | 0,982 | 0,935 | 0,856 | 1 |
| IFI27, OAS1, ISG15, HERC6, SLC1A2, OLAH, RETN, MMP8 | 0,879 | 0,870 | 0,889 | 0,929 | 0,879 | 0,978 | 0,938 | 0,874 | 1 |
| IFIT1, IFI44L, SIGLEC1, HERC6, SLC1A2, IL1R2, FAM20A, MMP8 | 0,879 | 0,870 | 0,889 | 0,930 | 0,885 | 0,975 | 0,961 | 0,898 | 1 |
| RSAD2, IFI27, OAS1, IFIT1, ISG15, HERC6, IL1R2, OLAH | 0,879 | 0,871 | 0,888 | 0,924 | 0,874 | 0,974 | 0,939 | 0,882 | 0,996 |
| OAS1, IFIT1, IFI44L, SIGLEC1, HERC6, OLAH, FAM20A, MMP8 | 0,879 | 0,869 | 0,889 | 0,917 | 0,858 | 0,976 | 0,948 | 0,890 | 1 |

519

| | | | | | | | | |
|---|---|---|---|---|---|---|---|---|
| OAS1, IFIT1, SIGLEC1, ISG15, HERC6, IL1R2, OLAH, FAM20A | 0,879 | 0,870 | 0,889 | 0,919 | 0,862 | 0,977 | 0,953 | 0,898 | 1 |
| IFI27, IFI44L, SIGLEC1, ISG15, SLC1A2, OLAH, RETN, MMP8 | 0,879 | 0,870 | 0,888 | 0,930 | 0,880 | 0,981 | 0,952 | 0,897 | 1 |
| IFI27, IFIT1, SIGLEC1, HERC6, SLC1A2, OLAH, RETN, MMP8 | 0,879 | 0,870 | 0,889 | 0,930 | 0,879 | 0,980 | 0,955 | 0,906 | 1 |
| IFI27, OAS1, IFIT1, SIGLEC1, ISG15, SLC1A2, OLAH, MMP8 | 0,879 | 0,870 | 0,888 | 0,929 | 0,878 | 0,979 | 0,942 | 0,885 | 1,000 |
| RSAD2, IFI27, HERC6, SLC1A2, IL1R2, OLAH, RETN, MMP8 | 0,879 | 0,870 | 0,889 | 0,928 | 0,877 | 0,979 | 0,941 | 0,881 | 1 |
| RSAD2, IFI27, IFIT1, IFI44L, HERC6, IL1R2, OLAH, RETN | 0,879 | 0,870 | 0,888 | 0,923 | 0,869 | 0,978 | 0,939 | 0,882 | 0,997 |
| RSAD2, IFI27, OAS1, IFI44L, SIGLEC1, ISG15, IL1R2, OLAH | 0,879 | 0,871 | 0,888 | 0,924 | 0,874 | 0,975 | 0,945 | 0,892 | 0,997 |
| IFI27, IFIT1, SIGLEC1, ISG15, HERC6, SLC1A2, OLAH, MMP8 | 0,879 | 0,870 | 0,889 | 0,931 | 0,881 | 0,980 | 0,940 | 0,883 | 0,997 |
| IFI27, OAS1, IFIT1, IFI44L, HERC6, IL1R2, RETN, MMP8 | 0,879 | 0,870 | 0,888 | 0,923 | 0,870 | 0,976 | 0,941 | 0,881 | 1 |
| IFI27, IFI44L, SIGLEC1, ISG15, SLC1A2, IL1R2, OLAH, RETN | 0,879 | 0,871 | 0,888 | 0,928 | 0,878 | 0,979 | 0,948 | 0,891 | 1 |
| RSAD2, IFI27, IFIT1, IFI44L, SIGLEC1, ISG15, HERC6, OLAH | 0,879 | 0,871 | 0,888 | 0,925 | 0,873 | 0,978 | 0,943 | 0,890 | 0,997 |
| IFI27, OAS1, IFIT1, IFI44L, ISG15, HERC6, OLAH, MMP8 | 0,879 | 0,870 | 0,888 | 0,925 | 0,873 | 0,977 | 0,936 | 0,877 | 0,994 |
| RSAD2, IFI27, OAS1, IFI44L, SIGLEC1, HERC6, IL1R2, MMP8 | 0,879 | 0,871 | 0,887 | 0,924 | 0,877 | 0,971 | 0,941 | 0,889 | 0,993 |
| RSAD2, IFI27, IFIT1, HERC6, SLC1A2, OLAH, RETN, MMP8 | 0,879 | 0,870 | 0,889 | 0,929 | 0,878 | 0,980 | 0,942 | 0,883 | 1 |
| RSAD2, IFI27, OAS1, SIGLEC1, IL1R2, OLAH, RETN, MMP8 | 0,879 | 0,869 | 0,889 | 0,922 | 0,867 | 0,978 | 0,962 | 0,913 | 1 |
| OAS1, IFIT1, IFI44L, SIGLEC1, IL1R2, FAM20A, RETN, MMP8 | 0,879 | 0,870 | 0,888 | 0,923 | 0,867 | 0,979 | 0,968 | 0,918 | 1 |
| IFI27, OAS1, SIGLEC1, ISG15, IL1R2, OLAH, RETN, MMP8 | 0,879 | 0,870 | 0,889 | 0,923 | 0,868 | 0,978 | 0,960 | 0,911 | 1 |
| IFI27, IFIT1, IFI44L, SIGLEC1, ISG15, OLAH, RETN, MMP8 | 0,879 | 0,870 | 0,888 | 0,920 | 0,862 | 0,978 | 0,952 | 0,896 | 1 |
| RSAD2, IFI27, HERC6, SLC1A2, IL1R2, FAM20A, RETN, MMP8 | 0,879 | 0,870 | 0,888 | 0,934 | 0,886 | 0,981 | 0,929 | 0,844 | 1 |
| RSAD2, IFI27, OAS1, IFIT1, ISG15, HERC6, OLAH, MMP8 | 0,879 | 0,870 | 0,888 | 0,923 | 0,871 | 0,976 | 0,936 | 0,877 | 0,995 |
| RSAD2, IFI27, OAS1, SIGLEC1, HERC6, SLC1A2, IL1R2, OLAH | 0,879 | 0,870 | 0,889 | 0,928 | 0,880 | 0,977 | 0,946 | 0,889 | 1 |
| RSAD2, IFI27, SIGLEC1, HERC6, SLC1A2, IL1R2, OLAH, MMP8 | 0,879 | 0,870 | 0,889 | 0,928 | 0,879 | 0,977 | 0,945 | 0,891 | 0,998 |
| IFIT1, IFI44L, SIGLEC1, ISG15, HERC6, FAM20A, RETN, MMP8 | 0,879 | 0,870 | 0,888 | 0,919 | 0,859 | 0,979 | 0,947 | 0,880 | 1 |
| RSAD2, IFI27, OAS1, SIGLEC1, ISG15, HERC6, SLC1A2, OLAH | 0,879 | 0,870 | 0,888 | 0,930 | 0,880 | 0,980 | 0,939 | 0,880 | 0,999 |
| IFI27, IFI44L, HERC6, SLC1A2, IL1R2, OLAH, FAM20A, RETN | 0,879 | 0,869 | 0,889 | 0,929 | 0,877 | 0,982 | 0,927 | 0,848 | 1 |
| RSAD2, SIGLEC1, ISG15, HERC6, IL1R2, OLAH, FAM20A, MMP8 | 0,879 | 0,870 | 0,889 | 0,919 | 0,863 | 0,975 | 0,962 | 0,905 | 1 |
| IFI27, OAS1, SIGLEC1, SLC1A2, IL1R2, OLAH, RETN, MMP8 | 0,879 | 0,870 | 0,889 | 0,928 | 0,878 | 0,978 | 0,955 | 0,900 | 1 |
| RSAD2, OAS1, IFIT1, SIGLEC1, HERC6, OLAH, FAM20A, MMP8 | 0,879 | 0,869 | 0,889 | 0,917 | 0,858 | 0,975 | 0,953 | 0,897 | 1 |

| | | | | | | | | | |
|---|---|---|---|---|---|---|---|---|---|
| RSAD2, OAS1, IFIT1, IFI44L, SIGLEC1, HERC6, IL1R2, FAM20A | 0,879 | 0,870 | 0,888 | 0,919 | 0,861 | 0,976 | 0,953 | 0,898 | 1 |
| RSAD2, IFI27, OAS1, IFIT1, IFI44L, HERC6, IL1R2, RETN | 0,879 | 0,871 | 0,888 | 0,922 | 0,869 | 0,976 | 0,938 | 0,874 | 1 |
| IFI27, IFIT1, SIGLEC1, ISG15, HERC6, SLC1A2, IL1R2, OLAH | 0,879 | 0,870 | 0,888 | 0,929 | 0,879 | 0,978 | 0,940 | 0,883 | 0,998 |
| RSAD2, OAS1, IFIT1, SIGLEC1, ISG15, HERC6, IL1R2, FAM20A | 0,879 | 0,870 | 0,888 | 0,919 | 0,861 | 0,976 | 0,953 | 0,898 | 1 |
| RSAD2, IFI27, IFIT1, ISG15, SLC1A2, OLAH, FAM20A, RETN | 0,879 | 0,870 | 0,888 | 0,926 | 0,870 | 0,981 | 0,922 | 0,843 | 1 |
| IFI27, IFIT1, IFI44L, SIGLEC1, ISG15, IL1R2, OLAH, RETN | 0,879 | 0,871 | 0,887 | 0,921 | 0,867 | 0,975 | 0,950 | 0,897 | 1 |
| RSAD2, IFI27, IFIT1, SIGLEC1, IL1R2, OLAH, RETN, MMP8 | 0,879 | 0,870 | 0,888 | 0,923 | 0,868 | 0,978 | 0,961 | 0,911 | 1 |
| RSAD2, IFI27, IFIT1, IFI44L, SIGLEC1, ISG15, HERC6, RETN | 0,879 | 0,871 | 0,887 | 0,917 | 0,859 | 0,975 | 0,941 | 0,887 | 0,996 |
| IFI27, OAS1, IFIT1, SIGLEC1, HERC6, SLC1A2, RETN, MMP8 | 0,879 | 0,870 | 0,888 | 0,926 | 0,874 | 0,978 | 0,946 | 0,888 | 1 |
| IFI27, IFIT1, IFI44L, ISG15, HERC6, IL1R2, OLAH, RETN | 0,879 | 0,870 | 0,888 | 0,922 | 0,868 | 0,977 | 0,939 | 0,882 | 0,996 |
| RSAD2, IFI27, OAS1, IFIT1, IFI44L, ISG15, IL1R2, FAM20A | 0,879 | 0,870 | 0,888 | 0,923 | 0,869 | 0,977 | 0,928 | 0,848 | 1 |
| IFI27, OAS1, IFIT1, IFI44L, SIGLEC1, IL1R2, OLAH, RETN | 0,879 | 0,870 | 0,888 | 0,920 | 0,865 | 0,975 | 0,953 | 0,900 | 1 |
| IFI27, OAS1, IFIT1, IFI44L, SIGLEC1, ISG15, FAM20A, MMP8 | 0,879 | 0,869 | 0,889 | 0,915 | 0,850 | 0,980 | 0,943 | 0,873 | 1 |
| RSAD2, IFI27, OAS1, IFIT1, SIGLEC1, HERC6, SLC1A2, OLAH | 0,879 | 0,870 | 0,888 | 0,928 | 0,878 | 0,978 | 0,942 | 0,884 | 1 |
| RSAD2, IFI27, IFIT1, ISG15, HERC6, FAM20A, RETN, MMP8 | 0,879 | 0,869 | 0,889 | 0,927 | 0,871 | 0,982 | 0,934 | 0,854 | 1 |
| IFI27, IFIT1, IFI44L, SIGLEC1, ISG15, HERC6, SLC1A2, FAM20A | 0,879 | 0,870 | 0,888 | 0,927 | 0,876 | 0,978 | 0,941 | 0,864 | 1 |
| IFIT1, IFI44L, SIGLEC1, IL1R2, OLAH, FAM20A, RETN, MMP8 | 0,879 | 0,870 | 0,888 | 0,919 | 0,862 | 0,977 | 0,967 | 0,917 | 1 |
| IFI27, OAS1, IFIT1, SIGLEC1, HERC6, SLC1A2, IL1R2, RETN | 0,879 | 0,870 | 0,888 | 0,928 | 0,881 | 0,975 | 0,945 | 0,888 | 1 |
| RSAD2, IFI27, IFIT1, SIGLEC1, ISG15, HERC6, OLAH, MMP8 | 0,879 | 0,870 | 0,888 | 0,924 | 0,872 | 0,975 | 0,937 | 0,880 | 0,994 |
| IFI27, OAS1, IFIT1, SIGLEC1, ISG15, HERC6, SLC1A2, RETN | 0,879 | 0,870 | 0,888 | 0,927 | 0,876 | 0,978 | 0,942 | 0,884 | 1 |
| RSAD2, IFI44L, SIGLEC1, HERC6, IL1R2, OLAH, FAM20A, MMP8 | 0,879 | 0,869 | 0,888 | 0,921 | 0,866 | 0,976 | 0,961 | 0,903 | 1 |
| RSAD2, IFI27, IFI44L, ISG15, IL1R2, FAM20A, RETN, MMP8 | 0,879 | 0,869 | 0,889 | 0,927 | 0,875 | 0,980 | 0,932 | 0,851 | 1 |
| RSAD2, IFI27, OAS1, SLC1A2, IL1R2, OLAH, FAM20A, RETN | 0,879 | 0,869 | 0,888 | 0,927 | 0,875 | 0,980 | 0,918 | 0,834 | 1 |
| RSAD2, IFIT1, IFI44L, SIGLEC1, HERC6, IL1R2, OLAH, FAM20A | 0,879 | 0,870 | 0,888 | 0,918 | 0,860 | 0,976 | 0,952 | 0,895 | 1 |
| OAS1, SIGLEC1, ISG15, HERC6, IL1R2, OLAH, FAM20A, MMP8 | 0,879 | 0,869 | 0,888 | 0,918 | 0,861 | 0,975 | 0,963 | 0,908 | 1 |
| RSAD2, IFI27, IFIT1, SLC1A2, IL1R2, OLAH, FAM20A, RETN | 0,879 | 0,870 | 0,888 | 0,927 | 0,872 | 0,981 | 0,921 | 0,839 | 1 |
| RSAD2, IFI27, IFIT1, SIGLEC1, SLC1A2, FAM20A, RETN, MMP8 | 0,879 | 0,869 | 0,888 | 0,926 | 0,871 | 0,982 | 0,946 | 0,873 | 1 |
| IFI27, IFIT1, SIGLEC1, ISG15, SLC1A2, IL1R2, OLAH, RETN | 0,879 | 0,870 | 0,888 | 0,929 | 0,879 | 0,980 | 0,941 | 0,881 | 1 |

| Genes | | | | | | | | | |
|---|---|---|---|---|---|---|---|---|---|
| RSAD2, OAS1, IFIT1, SIGLEC1, HERC6, IL1R2, OLAH, FAM20A | 0,879 | 0,870 | 0,888 | 0,918 | 0,860 | 0,976 | 0,955 | 0,901 | 1 |
| IFI27, OAS1, IFIT1, HERC6, SLC1A2, IL1R2, OLAH, RETN | 0,879 | 0,869 | 0,888 | 0,927 | 0,876 | 0,978 | 0,934 | 0,867 | 1 |
| OAS1, IFI44L, SIGLEC1, HERC6, IL1R2, OLAH, FAM20A, MMP8 | 0,879 | 0,869 | 0,888 | 0,920 | 0,863 | 0,976 | 0,962 | 0,906 | 1 |
| RSAD2, IFI27, IFIT1, IFI44L, HERC6, FAM20A, RETN, MMP8 | 0,879 | 0,869 | 0,889 | 0,925 | 0,868 | 0,982 | 0,934 | 0,853 | 1 |
| IFI27, OAS1, IFI44L, SIGLEC1, ISG15, IL1R2, OLAH, RETN | 0,879 | 0,870 | 0,887 | 0,921 | 0,866 | 0,976 | 0,947 | 0,894 | 0,999 |
| IFI27, OAS1, SIGLEC1, ISG15, SLC1A2, IL1R2, OLAH, MMP8 | 0,879 | 0,870 | 0,888 | 0,930 | 0,882 | 0,979 | 0,940 | 0,882 | 0,999 |
| RSAD2, IFI27, IFIT1, IFI44L, SIGLEC1, HERC6, SLC1A2, FAM20A | 0,879 | 0,870 | 0,888 | 0,927 | 0,875 | 0,978 | 0,937 | 0,860 | 1 |
| IFI27, OAS1, IFIT1, SIGLEC1, ISG15, IL1R2, OLAH, RETN | 0,879 | 0,870 | 0,887 | 0,919 | 0,864 | 0,975 | 0,947 | 0,890 | 1 |
| RSAD2, IFI27, IFI44L, ISG15, HERC6, FAM20A, RETN, MMP8 | 0,879 | 0,868 | 0,889 | 0,925 | 0,868 | 0,982 | 0,937 | 0,858 | 1 |
| RSAD2, IFI27, OAS1, IFI44L, ISG15, HERC6, IL1R2, RETN | 0,879 | 0,870 | 0,887 | 0,922 | 0,868 | 0,975 | 0,938 | 0,873 | 1 |
| IFI27, OAS1, IFIT1, ISG15, HERC6, IL1R2, RETN, MMP8 | 0,879 | 0,870 | 0,888 | 0,924 | 0,871 | 0,977 | 0,945 | 0,886 | 1 |
| RSAD2, IFI27, OAS1, IFI44L, HERC6, FAM20A, RETN, MMP8 | 0,879 | 0,868 | 0,889 | 0,925 | 0,867 | 0,983 | 0,935 | 0,855 | 1 |
| IFIT1, IFI44L, SIGLEC1, ISG15, HERC6, OLAH, FAM20A, RETN | 0,879 | 0,869 | 0,888 | 0,918 | 0,858 | 0,978 | 0,945 | 0,886 | 1 |
| RSAD2, IFI27, OAS1, ISG15, SLC1A2, OLAH, FAM20A, RETN | 0,879 | 0,869 | 0,888 | 0,927 | 0,874 | 0,981 | 0,917 | 0,833 | 1 |
| RSAD2, IFI27, OAS1, IFIT1, SIGLEC1, OLAH, RETN, MMP8 | 0,879 | 0,869 | 0,888 | 0,920 | 0,861 | 0,978 | 0,962 | 0,911 | 1 |
| RSAD2, IFI27, SIGLEC1, ISG15, HERC6, SLC1A2, OLAH, MMP8 | 0,879 | 0,869 | 0,888 | 0,931 | 0,881 | 0,980 | 0,939 | 0,881 | 0,998 |
| OAS1, IFI44L, SIGLEC1, ISG15, HERC6, IL1R2, FAM20A, MMP8 | 0,879 | 0,869 | 0,888 | 0,917 | 0,859 | 0,974 | 0,960 | 0,900 | 1 |
| OAS1, IFIT1, IFI44L, SIGLEC1, ISG15, IL1R2, FAM20A, MMP8 | 0,879 | 0,869 | 0,888 | 0,919 | 0,861 | 0,976 | 0,966 | 0,913 | 1 |
| IFI27, SIGLEC1, ISG15, HERC6, SLC1A2, IL1R2, FAM20A, RETN | 0,879 | 0,869 | 0,889 | 0,932 | 0,884 | 0,980 | 0,947 | 0,877 | 1 |
| RSAD2, IFI27, IFIT1, IFI44L, SIGLEC1, OLAH, RETN, MMP8 | 0,879 | 0,870 | 0,888 | 0,921 | 0,863 | 0,979 | 0,955 | 0,900 | 1 |
| IFI27, OAS1, IFI44L, HERC6, SLC1A2, IL1R2, FAM20A, MMP8 | 0,879 | 0,868 | 0,889 | 0,930 | 0,884 | 0,976 | 0,923 | 0,834 | 1 |
| RSAD2, IFI27, OAS1, IFIT1, IFI44L, SIGLEC1, IL1R2, OLAH | 0,879 | 0,870 | 0,887 | 0,921 | 0,869 | 0,973 | 0,951 | 0,902 | 1 |
| RSAD2, OAS1, SIGLEC1, ISG15, IL1R2, OLAH, FAM20A, MMP8 | 0,879 | 0,869 | 0,888 | 0,918 | 0,861 | 0,976 | 0,963 | 0,906 | 1 |
| RSAD2, IFIT1, SIGLEC1, ISG15, HERC6, IL1R2, OLAH, FAM20A | 0,879 | 0,869 | 0,888 | 0,918 | 0,859 | 0,977 | 0,952 | 0,897 | 1 |
| RSAD2, IFI27, OAS1, ISG15, HERC6, IL1R2, RETN, MMP8 | 0,879 | 0,870 | 0,887 | 0,923 | 0,869 | 0,977 | 0,942 | 0,880 | 1 |
| RSAD2, IFI27, OAS1, SIGLEC1, HERC6, SLC1A2, FAM20A, RETN | 0,879 | 0,869 | 0,889 | 0,925 | 0,872 | 0,979 | 0,937 | 0,863 | 1 |
| IFI27, SIGLEC1, ISG15, HERC6, SLC1A2, IL1R2, OLAH, MMP8 | 0,879 | 0,870 | 0,888 | 0,929 | 0,880 | 0,978 | 0,942 | 0,888 | 0,997 |
| RSAD2, IFI27, OAS1, IFI44L, SIGLEC1, OLAH, RETN, MMP8 | 0,879 | 0,869 | 0,888 | 0,921 | 0,863 | 0,978 | 0,960 | 0,908 | 1 |

| | | | | | | | | |
|---|---|---|---|---|---|---|---|---|
| IFI27, IFIT1, IFI44L, HERC6, SLC1A2, IL1R2, FAM20A, MMP8 | 0,879 | 0,869 | 0,888 | 0,930 | 0,884 | 0,977 | 0,929 | 0,845 | 1 |
| IFI27, OAS1, IFIT1, IFI44L, SIGLEC1, HERC6, SLC1A2, RETN | 0,879 | 0,870 | 0,887 | 0,926 | 0,874 | 0,977 | 0,941 | 0,882 | 1 |
| IFI44L, SIGLEC1, ISG15, SLC1A2, IL1R2, OLAH, FAM20A, MMP8 | 0,878 | 0,870 | 0,887 | 0,923 | 0,875 | 0,972 | 0,959 | 0,895 | 1 |
| IFI27, OAS1, ISG15, HERC6, SLC1A2, IL1R2, FAM20A, RETN | 0,878 | 0,869 | 0,888 | 0,933 | 0,885 | 0,980 | 0,923 | 0,836 | 1 |
| IFI27, OAS1, IFIT1, ISG15, HERC6, SLC1A2, OLAH, RETN | 0,878 | 0,869 | 0,888 | 0,928 | 0,878 | 0,978 | 0,935 | 0,869 | 1 |
| OAS1, IFIT1, SIGLEC1, SLC1A2, IL1R2, OLAH, FAM20A, MMP8 | 0,878 | 0,869 | 0,888 | 0,926 | 0,878 | 0,975 | 0,962 | 0,906 | 1 |
| IFI27, OAS1, IFI44L, ISG15, HERC6, SLC1A2, IL1R2, FAM20A | 0,878 | 0,868 | 0,889 | 0,930 | 0,884 | 0,976 | 0,925 | 0,837 | 1 |
| RSAD2, IFI27, OAS1, HERC6, SLC1A2, IL1R2, OLAH, RETN | 0,878 | 0,869 | 0,888 | 0,928 | 0,876 | 0,979 | 0,932 | 0,860 | 1 |
| IFI27, OAS1, ISG15, HERC6, SLC1A2, IL1R2, OLAH, RETN | 0,878 | 0,869 | 0,888 | 0,930 | 0,880 | 0,979 | 0,930 | 0,862 | 0,999 |
| IFI27, OAS1, IFIT1, IFI44L, ISG15, HERC6, IL1R2, RETN | 0,878 | 0,870 | 0,887 | 0,924 | 0,873 | 0,976 | 0,938 | 0,877 | 1,000 |
| RSAD2, IFI27, SIGLEC1, ISG15, SLC1A2, IL1R2, OLAH, MMP8 | 0,878 | 0,870 | 0,887 | 0,929 | 0,880 | 0,978 | 0,942 | 0,887 | 0,998 |
| RSAD2, IFI27, OAS1, SIGLEC1, HERC6, SLC1A2, IL1R2, RETN | 0,878 | 0,870 | 0,887 | 0,926 | 0,877 | 0,975 | 0,948 | 0,888 | 1 |
| IFI27, IFIT1, IFI44L, SLC1A2, IL1R2, FAM20A, RETN, MMP8 | 0,878 | 0,869 | 0,888 | 0,932 | 0,883 | 0,981 | 0,932 | 0,849 | 1 |
| RSAD2, IFI27, OAS1, IFIT1, IFI44L, ISG15, HERC6, OLAH | 0,878 | 0,869 | 0,887 | 0,924 | 0,873 | 0,976 | 0,934 | 0,874 | 0,994 |
| IFI27, OAS1, IFIT1, SIGLEC1, ISG15, SLC1A2, FAM20A, RETN | 0,878 | 0,869 | 0,888 | 0,924 | 0,868 | 0,980 | 0,938 | 0,862 | 1 |
| IFI27, IFIT1, SIGLEC1, ISG15, HERC6, SLC1A2, OLAH, RETN | 0,878 | 0,869 | 0,888 | 0,928 | 0,877 | 0,979 | 0,941 | 0,884 | 0,999 |
| IFI27, IFIT1, IFI44L, SIGLEC1, ISG15, IL1R2, RETN, MMP8 | 0,878 | 0,870 | 0,887 | 0,925 | 0,873 | 0,977 | 0,959 | 0,916 | 1 |
| IFI44L, SIGLEC1, ISG15, HERC6, IL1R2, FAM20A, RETN, MMP8 | 0,878 | 0,869 | 0,888 | 0,917 | 0,858 | 0,975 | 0,958 | 0,894 | 1 |
| IFI27, OAS1, IFIT1, IFI44L, SIGLEC1, IL1R2, RETN, MMP8 | 0,878 | 0,870 | 0,887 | 0,924 | 0,872 | 0,976 | 0,958 | 0,913 | 1 |
| IFI27, OAS1, IFI44L, HERC6, SLC1A2, OLAH, RETN, MMP8 | 0,878 | 0,869 | 0,888 | 0,930 | 0,881 | 0,980 | 0,938 | 0,872 | 1 |
| IFI27, OAS1, IFI44L, SIGLEC1, ISG15, OLAH, RETN, MMP8 | 0,878 | 0,869 | 0,888 | 0,922 | 0,866 | 0,979 | 0,957 | 0,905 | 1 |
| IFI27, OAS1, IFI44L, SLC1A2, OLAH, FAM20A, RETN, MMP8 | 0,878 | 0,868 | 0,889 | 0,929 | 0,874 | 0,984 | 0,928 | 0,851 | 1 |
| IFI27, OAS1, IFIT1, IFI44L, SIGLEC1, ISG15, OLAH, RETN | 0,878 | 0,870 | 0,887 | 0,919 | 0,862 | 0,977 | 0,948 | 0,891 | 1 |
| RSAD2, IFI27, OAS1, IFI44L, HERC6, IL1R2, RETN, MMP8 | 0,878 | 0,869 | 0,887 | 0,921 | 0,866 | 0,976 | 0,937 | 0,872 | 1 |
| IFI27, SIGLEC1, ISG15, SLC1A2, IL1R2, OLAH, RETN, MMP8 | 0,878 | 0,869 | 0,888 | 0,929 | 0,880 | 0,979 | 0,957 | 0,906 | 1 |
| RSAD2, IFI27, IFI44L, HERC6, SLC1A2, IL1R2, FAM20A, MMP8 | 0,878 | 0,869 | 0,888 | 0,930 | 0,884 | 0,976 | 0,927 | 0,840 | 1 |
| IFI27, IFIT1, IFI44L, SIGLEC1, HERC6, SLC1A2, OLAH, RETN | 0,878 | 0,869 | 0,888 | 0,926 | 0,876 | 0,977 | 0,945 | 0,889 | 1 |
| IFI27, OAS1, IFI44L, ISG15, HERC6, IL1R2, RETN, MMP8 | 0,878 | 0,869 | 0,887 | 0,924 | 0,871 | 0,977 | 0,937 | 0,874 | 1,000 |

| | 0,878 | 0,869 | 0,888 | 0,928 | 0,882 | 0,974 | 0,959 | 0,895 | 1 |
|---|---|---|---|---|---|---|---|---|---|
| RSAD2, IFIT1, SIGLEC1, HERC6, SLC1A2, IL1R2, FAM20A, MMP8 | 0,878 | 0,869 | 0,888 | 0,928 | 0,882 | 0,974 | 0,959 | 0,895 | 1 |
| IFI27, OAS1, IFIT1, HERC6, SLC1A2, IL1R2, OLAH, MMP8 | 0,878 | 0,869 | 0,888 | 0,928 | 0,879 | 0,976 | 0,935 | 0,870 | 1 |
| IFIT1, IFI44L, SIGLEC1, ISG15, HERC6, IL1R2, FAM20A, RETN | 0,878 | 0,869 | 0,887 | 0,918 | 0,861 | 0,976 | 0,953 | 0,897 | 1 |
| IFIT1, SIGLEC1, ISG15, HERC6, SLC1A2, OLAH, FAM20A, MMP8 | 0,878 | 0,868 | 0,889 | 0,925 | 0,876 | 0,975 | 0,947 | 0,877 | 1 |
| RSAD2, IFI27, IFI44L, SIGLEC1, ISG15, HERC6, IL1R2, RETN | 0,878 | 0,870 | 0,887 | 0,923 | 0,869 | 0,976 | 0,945 | 0,893 | 0,996 |
| IFI27, OAS1, IFIT1, SIGLEC1, ISG15, SLC1A2, IL1R2, OLAH | 0,878 | 0,869 | 0,887 | 0,930 | 0,881 | 0,979 | 0,941 | 0,883 | 1,000 |
| IFI27, OAS1, IFIT1, IFI44L, ISG15, HERC6, RETN, MMP8 | 0,878 | 0,869 | 0,888 | 0,916 | 0,857 | 0,976 | 0,934 | 0,867 | 1 |
| RSAD2, IFI27, OAS1, SIGLEC1, ISG15, SLC1A2, OLAH, MMP8 | 0,878 | 0,869 | 0,887 | 0,929 | 0,878 | 0,980 | 0,943 | 0,886 | 1 |
| IFI27, IFIT1, HERC6, SLC1A2, IL1R2, FAM20A, RETN, MMP8 | 0,878 | 0,869 | 0,888 | 0,933 | 0,884 | 0,982 | 0,930 | 0,847 | 1 |
| IFI27, IFI44L, SIGLEC1, ISG15, HERC6, SLC1A2, OLAH, RETN | 0,878 | 0,869 | 0,887 | 0,927 | 0,875 | 0,978 | 0,943 | 0,887 | 1 |
| OAS1, IFIT1, SIGLEC1, ISG15, IL1R2, FAM20A, RETN, MMP8 | 0,878 | 0,869 | 0,887 | 0,922 | 0,865 | 0,979 | 0,967 | 0,914 | 1 |
| IFI27, IFIT1, SIGLEC1, HERC6, SLC1A2, IL1R2, OLAH, RETN | 0,878 | 0,869 | 0,888 | 0,927 | 0,876 | 0,978 | 0,948 | 0,894 | 1 |
| RSAD2, IFI27, SIGLEC1, HERC6, SLC1A2, IL1R2, OLAH, RETN | 0,878 | 0,869 | 0,888 | 0,928 | 0,878 | 0,978 | 0,946 | 0,890 | 1 |
| RSAD2, OAS1, SIGLEC1, HERC6, IL1R2, FAM20A, RETN, MMP8 | 0,878 | 0,869 | 0,888 | 0,921 | 0,865 | 0,977 | 0,959 | 0,898 | 1 |
| RSAD2, IFI27, SIGLEC1, ISG15, HERC6, SLC1A2, IL1R2, OLAH | 0,878 | 0,869 | 0,887 | 0,930 | 0,881 | 0,979 | 0,940 | 0,883 | 0,998 |
| IFI27, OAS1, IFIT1, IFI44L, ISG15, HERC6, IL1R2, OLAH | 0,878 | 0,870 | 0,887 | 0,924 | 0,874 | 0,975 | 0,936 | 0,878 | 0,994 |
| IFI27, IFIT1, IFI44L, ISG15, HERC6, IL1R2, OLAH, MMP8 | 0,878 | 0,870 | 0,887 | 0,925 | 0,875 | 0,975 | 0,937 | 0,880 | 0,993 |
| OAS1, IFIT1, IFI44L, SIGLEC1, SLC1A2, OLAH, FAM20A, MMP8 | 0,878 | 0,868 | 0,888 | 0,924 | 0,870 | 0,978 | 0,943 | 0,881 | 1 |
| IFI27, OAS1, IFI44L, SIGLEC1, ISG15, HERC6, SLC1A2, OLAH | 0,878 | 0,869 | 0,888 | 0,929 | 0,879 | 0,979 | 0,939 | 0,881 | 0,997 |
| OAS1, IFIT1, SIGLEC1, HERC6, SLC1A2, OLAH, FAM20A, MMP8 | 0,878 | 0,868 | 0,888 | 0,926 | 0,877 | 0,975 | 0,942 | 0,871 | 1 |
| IFI27, OAS1, IFI44L, SIGLEC1, HERC6, SLC1A2, OLAH, MMP8 | 0,878 | 0,869 | 0,888 | 0,927 | 0,877 | 0,977 | 0,941 | 0,884 | 0,999 |
| IFI27, OAS1, IFIT1, IFI44L, SIGLEC1, ISG15, OLAH, MMP8 | 0,878 | 0,869 | 0,887 | 0,920 | 0,863 | 0,976 | 0,939 | 0,882 | 0,996 |
| RSAD2, IFI27, OAS1, IFIT1, HERC6, SLC1A2, OLAH, RETN | 0,878 | 0,869 | 0,888 | 0,927 | 0,876 | 0,978 | 0,932 | 0,860 | 1 |
| RSAD2, IFI27, OAS1, IFIT1, SIGLEC1, IL1R2, OLAH, MMP8 | 0,878 | 0,869 | 0,887 | 0,922 | 0,871 | 0,973 | 0,947 | 0,896 | 0,998 |
| RSAD2, IFI27, OAS1, IFI44L, SIGLEC1, IL1R2, OLAH, RETN | 0,878 | 0,869 | 0,887 | 0,920 | 0,865 | 0,975 | 0,953 | 0,897 | 1 |
| RSAD2, IFI27, OAS1, IFIT1, HERC6, FAM20A, RETN, MMP8 | 0,878 | 0,868 | 0,888 | 0,924 | 0,866 | 0,982 | 0,934 | 0,853 | 1 |
| IFI27, IFI44L, SIGLEC1, SLC1A2, IL1R2, OLAH, RETN, MMP8 | 0,878 | 0,869 | 0,887 | 0,930 | 0,881 | 0,980 | 0,958 | 0,908 | 1 |
| IFI27, OAS1, IFIT1, SIGLEC1, SLC1A2, OLAH, RETN, MMP8 | 0,878 | 0,868 | 0,888 | 0,927 | 0,875 | 0,980 | 0,957 | 0,901 | 1 |

| | | | | | | | | |
|---|---|---|---|---|---|---|---|---|
| IFI27, IFIT1, SIGLEC1, ISG15, HERC6, IL1R2, RETN, MMP8 | 0,878 | 0,869 | 0,887 | 0,921 | 0,867 | 0,976 | 0,957 | 0,914 | 0,999 |
| RSAD2, IFI27, IFI44L, SLC1A2, IL1R2, OLAH, FAM20A, RETN | 0,878 | 0,868 | 0,888 | 0,927 | 0,872 | 0,981 | 0,923 | 0,843 | 1 |
| RSAD2, IFI27, IFIT1, HERC6, SLC1A2, IL1R2, FAM20A, MMP8 | 0,878 | 0,869 | 0,888 | 0,931 | 0,884 | 0,978 | 0,929 | 0,845 | 1 |
| RSAD2, IFI27, IFI44L, SIGLEC1, HERC6, SLC1A2, OLAH, RETN | 0,878 | 0,869 | 0,887 | 0,926 | 0,876 | 0,976 | 0,942 | 0,884 | 1 |
| IFI27, OAS1, IFIT1, IFI44L, HERC6, SLC1A2, IL1R2, FAM20A | 0,878 | 0,869 | 0,888 | 0,932 | 0,886 | 0,977 | 0,926 | 0,840 | 1 |
| IFI27, OAS1, IFIT1, SIGLEC1, SLC1A2, IL1R2, OLAH, RETN | 0,878 | 0,869 | 0,887 | 0,927 | 0,876 | 0,978 | 0,949 | 0,889 | 1 |
| RSAD2, IFI27, OAS1, IFIT1, IFI44L, IL1R2, FAM20A, RETN | 0,878 | 0,868 | 0,888 | 0,923 | 0,869 | 0,977 | 0,930 | 0,852 | 1 |
| IFI27, IFI44L, SIGLEC1, ISG15, HERC6, SLC1A2, OLAH, MMP8 | 0,878 | 0,869 | 0,887 | 0,930 | 0,880 | 0,980 | 0,941 | 0,885 | 0,998 |
| RSAD2, IFIT1, IFI44L, SIGLEC1, SLC1A2, OLAH, FAM20A, MMP8 | 0,878 | 0,868 | 0,888 | 0,926 | 0,873 | 0,978 | 0,942 | 0,879 | 1 |
| IFI27, OAS1, IFIT1, IFI44L, SIGLEC1, HERC6, SLC1A2, OLAH | 0,878 | 0,869 | 0,888 | 0,927 | 0,877 | 0,977 | 0,942 | 0,885 | 0,999 |
| IFI27, IFIT1, SIGLEC1, ISG15, SLC1A2, OLAH, RETN, MMP8 | 0,878 | 0,869 | 0,888 | 0,929 | 0,877 | 0,980 | 0,954 | 0,900 | 1 |
| IFI27, SIGLEC1, ISG15, HERC6, SLC1A2, OLAH, RETN, MMP8 | 0,878 | 0,868 | 0,888 | 0,927 | 0,875 | 0,980 | 0,953 | 0,900 | 1 |
| RSAD2, IFI27, OAS1, SIGLEC1, ISG15, HERC6, FAM20A, MMP8 | 0,878 | 0,868 | 0,888 | 0,916 | 0,856 | 0,976 | 0,941 | 0,871 | 1 |
| OAS1, IFIT1, IFI44L, SIGLEC1, HERC6, FAM20A, RETN, MMP8 | 0,878 | 0,869 | 0,887 | 0,919 | 0,859 | 0,978 | 0,947 | 0,883 | 1 |
| RSAD2, IFI27, OAS1, IFIT1, IFI44L, SIGLEC1, HERC6, IL1R2 | 0,878 | 0,870 | 0,886 | 0,924 | 0,876 | 0,971 | 0,947 | 0,898 | 0,996 |
| RSAD2, IFI27, OAS1, IFI44L, SIGLEC1, ISG15, OLAH, MMP8 | 0,878 | 0,870 | 0,887 | 0,919 | 0,862 | 0,975 | 0,942 | 0,887 | 0,997 |
| IFI27, OAS1, IFIT1, SIGLEC1, ISG15, IL1R2, OLAH, MMP8 | 0,878 | 0,870 | 0,887 | 0,923 | 0,873 | 0,974 | 0,947 | 0,897 | 0,997 |
| RSAD2, IFI27, IFI44L, SIGLEC1, ISG15, IL1R2, OLAH, RETN | 0,878 | 0,870 | 0,887 | 0,922 | 0,867 | 0,977 | 0,951 | 0,900 | 1 |
| RSAD2, OAS1, IFI44L, SIGLEC1, IL1R2, OLAH, FAM20A, MMP8 | 0,878 | 0,868 | 0,888 | 0,919 | 0,862 | 0,976 | 0,962 | 0,906 | 1 |
| IFIT1, IFI44L, SIGLEC1, ISG15, IL1R2, FAM20A, RETN, MMP8 | 0,878 | 0,869 | 0,887 | 0,922 | 0,866 | 0,979 | 0,968 | 0,918 | 1 |
| IFIT1, SIGLEC1, ISG15, IL1R2, OLAH, FAM20A, RETN, MMP8 | 0,878 | 0,869 | 0,887 | 0,921 | 0,864 | 0,978 | 0,966 | 0,915 | 1 |
| IFI27, IFI44L, HERC6, SLC1A2, IL1R2, FAM20A, RETN, MMP8 | 0,878 | 0,868 | 0,888 | 0,932 | 0,883 | 0,981 | 0,930 | 0,847 | 1 |
| IFI27, IFIT1, IFI44L, SIGLEC1, ISG15, SLC1A2, OLAH, RETN | 0,878 | 0,869 | 0,887 | 0,928 | 0,877 | 0,979 | 0,947 | 0,889 | 1 |
| IFI27, OAS1, ISG15, HERC6, SLC1A2, IL1R2, OLAH, MMP8 | 0,878 | 0,869 | 0,887 | 0,927 | 0,878 | 0,976 | 0,928 | 0,862 | 0,995 |
| RSAD2, IFI27, IFIT1, SIGLEC1, ISG15, HERC6, IL1R2, RETN | 0,878 | 0,870 | 0,886 | 0,924 | 0,872 | 0,975 | 0,949 | 0,901 | 0,997 |
| IFIT1, IFI44L, SIGLEC1, ISG15, HERC6, SLC1A2, OLAH, FAM20A | 0,878 | 0,868 | 0,888 | 0,924 | 0,873 | 0,976 | 0,930 | 0,858 | 1 |
| IFI27, IFIT1, HERC6, SLC1A2, IL1R2, OLAH, RETN, MMP8 | 0,878 | 0,869 | 0,887 | 0,927 | 0,876 | 0,979 | 0,941 | 0,881 | 1 |
| RSAD2, IFI27, ISG15, HERC6, SLC1A2, OLAH, RETN, MMP8 | 0,878 | 0,869 | 0,887 | 0,928 | 0,878 | 0,979 | 0,941 | 0,881 | 1 |

| | | | | | | | | |
|---|---|---|---|---|---|---|---|---|
| IFI27, OAS1, SIGLEC1, ISG15, HERC6, SLC1A2, IL1R2, RETN | 0,878 | 0,869 | 0,887 | 0,928 | 0,881 | 0,975 | 0,948 | 0,891 | 1 |
| IFI27, OAS1, IFIT1, SIGLEC1, SLC1A2, IL1R2, OLAH, MMP8 | 0,878 | 0,869 | 0,887 | 0,927 | 0,878 | 0,976 | 0,949 | 0,894 | 1 |
| RSAD2, IFI27, OAS1, ISG15, IL1R2, FAM20A, RETN, MMP8 | 0,878 | 0,868 | 0,888 | 0,929 | 0,877 | 0,981 | 0,935 | 0,856 | 1 |
| RSAD2, IFI27, SIGLEC1, ISG15, IL1R2, OLAH, RETN, MMP8 | 0,878 | 0,869 | 0,887 | 0,922 | 0,867 | 0,978 | 0,957 | 0,907 | 1 |
| RSAD2, IFI27, IFIT1, IFI44L, ISG15, IL1R2, FAM20A, RETN | 0,878 | 0,868 | 0,888 | 0,924 | 0,871 | 0,977 | 0,930 | 0,852 | 1 |
| IFI27, OAS1, IFIT1, IFI44L, IL1R2, OLAH, RETN, MMP8 | 0,878 | 0,869 | 0,887 | 0,922 | 0,866 | 0,977 | 0,950 | 0,895 | 1 |
| IFI27, OAS1, IFI44L, SLC1A2, IL1R2, OLAH, FAM20A, RETN | 0,878 | 0,868 | 0,888 | 0,929 | 0,875 | 0,983 | 0,921 | 0,839 | 1 |
| OAS1, IFIT1, SIGLEC1, ISG15, HERC6, OLAH, FAM20A, MMP8 | 0,878 | 0,868 | 0,888 | 0,917 | 0,858 | 0,975 | 0,953 | 0,899 | 1 |
| OAS1, IFIT1, SIGLEC1, ISG15, HERC6, FAM20A, RETN, MMP8 | 0,878 | 0,869 | 0,887 | 0,921 | 0,863 | 0,979 | 0,947 | 0,882 | 1 |
| RSAD2, SIGLEC1, HERC6, SLC1A2, IL1R2, OLAH, FAM20A, MMP8 | 0,878 | 0,868 | 0,888 | 0,926 | 0,878 | 0,973 | 0,960 | 0,900 | 1 |
| IFIT1, SIGLEC1, ISG15, SLC1A2, IL1R2, OLAH, FAM20A, MMP8 | 0,878 | 0,869 | 0,887 | 0,927 | 0,880 | 0,974 | 0,963 | 0,905 | 1 |
| IFI27, OAS1, IFIT1, ISG15, IL1R2, OLAH, RETN, MMP8 | 0,878 | 0,869 | 0,887 | 0,920 | 0,864 | 0,977 | 0,951 | 0,898 | 1 |
| RSAD2, IFI27, OAS1, ISG15, HERC6, SLC1A2, OLAH, RETN | 0,878 | 0,869 | 0,887 | 0,928 | 0,877 | 0,979 | 0,932 | 0,860 | 1 |
| RSAD2, IFI27, IFIT1, ISG15, HERC6, IL1R2, OLAH, MMP8 | 0,878 | 0,869 | 0,887 | 0,924 | 0,874 | 0,974 | 0,939 | 0,884 | 0,994 |
| RSAD2, IFIT1, SIGLEC1, HERC6, SLC1A2, OLAH, FAM20A, MMP8 | 0,878 | 0,868 | 0,888 | 0,927 | 0,878 | 0,976 | 0,946 | 0,876 | 1 |
| IFI27, OAS1, IFIT1, ISG15, HERC6, SLC1A2, OLAH, MMP8 | 0,878 | 0,868 | 0,888 | 0,929 | 0,879 | 0,978 | 0,930 | 0,864 | 0,997 |
| RSAD2, IFI27, IFIT1, IFI44L, SIGLEC1, IL1R2, OLAH, RETN | 0,878 | 0,869 | 0,886 | 0,920 | 0,865 | 0,975 | 0,952 | 0,900 | 1 |
| OAS1, IFIT1, SIGLEC1, HERC6, IL1R2, OLAH, FAM20A, RETN | 0,878 | 0,869 | 0,887 | 0,919 | 0,861 | 0,977 | 0,952 | 0,895 | 1 |
| RSAD2, IFI27, OAS1, IFIT1, IFI44L, SIGLEC1, OLAH, RETN | 0,878 | 0,869 | 0,886 | 0,916 | 0,856 | 0,975 | 0,955 | 0,899 | 1 |
| IFI27, OAS1, IFI44L, SIGLEC1, ISG15, SLC1A2, OLAH, MMP8 | 0,878 | 0,869 | 0,887 | 0,929 | 0,879 | 0,979 | 0,945 | 0,887 | 1 |
| RSAD2, IFI27, IFIT1, SIGLEC1, ISG15, SLC1A2, OLAH, MMP8 | 0,878 | 0,869 | 0,887 | 0,930 | 0,881 | 0,980 | 0,939 | 0,880 | 0,999 |
| IFI27, IFIT1, SIGLEC1, ISG15, HERC6, SLC1A2, FAM20A, MMP8 | 0,878 | 0,868 | 0,887 | 0,928 | 0,877 | 0,978 | 0,937 | 0,859 | 1 |
| IFI27, IFIT1, IFI44L, ISG15, SLC1A2, IL1R2, FAM20A, MMP8 | 0,878 | 0,869 | 0,887 | 0,928 | 0,880 | 0,976 | 0,927 | 0,840 | 1 |
| IFI27, OAS1, SIGLEC1, ISG15, SLC1A2, OLAH, RETN, MMP8 | 0,878 | 0,868 | 0,887 | 0,929 | 0,878 | 0,981 | 0,954 | 0,897 | 1 |
| RSAD2, IFI27, OAS1, SIGLEC1, ISG15, SLC1A2, IL1R2, OLAH | 0,878 | 0,869 | 0,887 | 0,929 | 0,879 | 0,979 | 0,942 | 0,884 | 1 |
| IFI27, IFIT1, SIGLEC1, HERC6, SLC1A2, IL1R2, OLAH, MMP8 | 0,878 | 0,868 | 0,887 | 0,927 | 0,878 | 0,977 | 0,945 | 0,890 | 0,999 |
| RSAD2, IFIT1, SIGLEC1, HERC6, OLAH, FAM20A, RETN, MMP8 | 0,878 | 0,868 | 0,887 | 0,921 | 0,864 | 0,978 | 0,952 | 0,896 | 1 |
| RSAD2, IFI27, OAS1, IFI44L, SIGLEC1, HERC6, FAM20A, MMP8 | 0,878 | 0,868 | 0,887 | 0,916 | 0,857 | 0,976 | 0,943 | 0,873 | 1 |

| | 0,878 | 0,868 | 0,887 | 0,922 | 0,866 | 0,977 | 0,952 | 0,900 | 1 |
|---|---|---|---|---|---|---|---|---|---|
| RSAD2, IFI27, OAS1, IFIT1, IL1R2, OLAH, RETN, MMP8 | 0,878 | 0,868 | 0,887 | 0,922 | 0,866 | 0,977 | 0,952 | 0,900 | 1 |
| RSAD2, IFI27, OAS1, IFI44L, SIGLEC1, ISG15, HERC6, FAM20A | 0,878 | 0,868 | 0,887 | 0,916 | 0,857 | 0,976 | 0,942 | 0,870 | 1 |
| IFI27, IFIT1, IFI44L, SIGLEC1, ISG15, SLC1A2, IL1R2, OLAH | 0,878 | 0,869 | 0,886 | 0,928 | 0,878 | 0,978 | 0,940 | 0,883 | 0,998 |
| IFIT1, IFI44L, SIGLEC1, HERC6, IL1R2, OLAH, FAM20A, RETN | 0,878 | 0,869 | 0,887 | 0,917 | 0,859 | 0,975 | 0,951 | 0,894 | 1 |
| IFIT1, SIGLEC1, ISG15, HERC6, SLC1A2, IL1R2, FAM20A, MMP8 | 0,878 | 0,868 | 0,887 | 0,928 | 0,882 | 0,974 | 0,958 | 0,890 | 1 |
| RSAD2, IFI27, OAS1, SIGLEC1, ISG15, IL1R2, OLAH, MMP8 | 0,878 | 0,869 | 0,887 | 0,923 | 0,872 | 0,974 | 0,945 | 0,894 | 0,995 |
| RSAD2, IFIT1, IFI44L, SIGLEC1, HERC6, OLAH, FAM20A, MMP8 | 0,878 | 0,868 | 0,887 | 0,917 | 0,859 | 0,975 | 0,949 | 0,891 | 1 |
| RSAD2, IFI27, OAS1, IFI44L, SIGLEC1, ISG15, SLC1A2, OLAH | 0,878 | 0,869 | 0,886 | 0,930 | 0,880 | 0,981 | 0,948 | 0,892 | 1 |
| RSAD2, IFI27, IFIT1, IFI44L, SIGLEC1, ISG15, IL1R2, OLAH | 0,878 | 0,869 | 0,886 | 0,926 | 0,875 | 0,976 | 0,942 | 0,889 | 0,996 |
| RSAD2, OAS1, IFIT1, SIGLEC1, IL1R2, FAM20A, RETN, MMP8 | 0,878 | 0,868 | 0,887 | 0,922 | 0,865 | 0,978 | 0,968 | 0,918 | 1 |
| IFI27, OAS1, IFIT1, IFI44L, SIGLEC1, ISG15, HERC6, IL1R2 | 0,878 | 0,869 | 0,886 | 0,924 | 0,878 | 0,970 | 0,939 | 0,886 | 0,992 |
| IFI27, OAS1, IFIT1, SIGLEC1, ISG15, HERC6, SLC1A2, FAM20A | 0,878 | 0,868 | 0,887 | 0,929 | 0,880 | 0,977 | 0,936 | 0,859 | 1 |
| IFI27, IFIT1, IFI44L, SIGLEC1, ISG15, HERC6, SLC1A2, OLAH | 0,878 | 0,869 | 0,887 | 0,930 | 0,881 | 0,980 | 0,940 | 0,883 | 0,998 |
| IFIT1, SIGLEC1, HERC6, SLC1A2, IL1R2, FAM20A, RETN, MMP8 | 0,878 | 0,868 | 0,887 | 0,928 | 0,880 | 0,975 | 0,958 | 0,890 | 1 |
| IFI27, OAS1, IFI44L, SIGLEC1, HERC6, SLC1A2, IL1R2, RETN | 0,878 | 0,869 | 0,886 | 0,928 | 0,881 | 0,975 | 0,939 | 0,881 | 0,997 |
| RSAD2, IFIT1, IFI44L, SIGLEC1, ISG15, HERC6, IL1R2, FAM20A | 0,878 | 0,869 | 0,886 | 0,918 | 0,860 | 0,976 | 0,953 | 0,897 | 1 |
| RSAD2, IFI27, IFIT1, IFI44L, SLC1A2, IL1R2, FAM20A, MMP8 | 0,878 | 0,868 | 0,887 | 0,928 | 0,879 | 0,977 | 0,927 | 0,842 | 1 |
| RSAD2, IFI27, OAS1, IFIT1, ISG15, IL1R2, FAM20A, RETN | 0,878 | 0,868 | 0,887 | 0,924 | 0,871 | 0,978 | 0,929 | 0,850 | 1 |
| IFI27, OAS1, SIGLEC1, ISG15, SLC1A2, IL1R2, OLAH, RETN | 0,878 | 0,869 | 0,886 | 0,928 | 0,877 | 0,979 | 0,942 | 0,881 | 1 |
| RSAD2, IFI27, OAS1, IFIT1, SIGLEC1, HERC6, SLC1A2, FAM20A | 0,878 | 0,868 | 0,887 | 0,928 | 0,879 | 0,977 | 0,936 | 0,859 | 1 |
| RSAD2, IFIT1, SIGLEC1, SLC1A2, IL1R2, OLAH, FAM20A, MMP8 | 0,878 | 0,868 | 0,887 | 0,927 | 0,879 | 0,975 | 0,962 | 0,906 | 1 |
| IFI27, OAS1, IFIT1, IFI44L, HERC6, SLC1A2, OLAH, RETN | 0,878 | 0,868 | 0,887 | 0,926 | 0,875 | 0,977 | 0,934 | 0,867 | 1 |
| RSAD2, IFI27, OAS1, SIGLEC1, ISG15, OLAH, RETN, MMP8 | 0,877 | 0,868 | 0,887 | 0,920 | 0,861 | 0,979 | 0,959 | 0,905 | 1 |
| RSAD2, IFIT1, IFI44L, SIGLEC1, ISG15, IL1R2, FAM20A, MMP8 | 0,877 | 0,868 | 0,887 | 0,920 | 0,862 | 0,977 | 0,966 | 0,913 | 1 |
| IFI27, OAS1, IFIT1, SIGLEC1, ISG15, SLC1A2, OLAH, RETN | 0,877 | 0,868 | 0,886 | 0,929 | 0,878 | 0,979 | 0,943 | 0,883 | 1 |
| IFI27, OAS1, SIGLEC1, ISG15, SLC1A2, FAM20A, RETN, MMP8 | 0,877 | 0,867 | 0,888 | 0,923 | 0,866 | 0,980 | 0,940 | 0,863 | 1 |
| RSAD2, IFI27, IFIT1, SIGLEC1, ISG15, HERC6, SLC1A2, OLAH | 0,877 | 0,868 | 0,887 | 0,930 | 0,880 | 0,980 | 0,939 | 0,881 | 0,997 |
| RSAD2, IFI27, IFI44L, SIGLEC1, HERC6, SLC1A2, OLAH, MMP8 | 0,877 | 0,868 | 0,887 | 0,926 | 0,875 | 0,977 | 0,943 | 0,888 | 0,999 |

| | | | | | | | | |
|---|---|---|---|---|---|---|---|---|
| OAS1, IFIT1, IFI44L, SIGLEC1, SLC1A2, IL1R2, FAM20A, MMP8 | 0,877 | 0,868 | 0,887 | 0,926 | 0,879 | 0,973 | 0,963 | 0,904 | 1 |
| IFI27, IFIT1, IFI44L, SIGLEC1, ISG15, HERC6, RETN, MMP8 | 0,877 | 0,868 | 0,887 | 0,912 | 0,851 | 0,973 | 0,939 | 0,884 | 0,995 |
| RSAD2, IFI27, IFI44L, HERC6, SLC1A2, OLAH, RETN, MMP8 | 0,877 | 0,868 | 0,887 | 0,928 | 0,877 | 0,979 | 0,941 | 0,881 | 1 |
| RSAD2, IFI27, OAS1, IFI44L, ISG15, IL1R2, FAM20A, RETN | 0,877 | 0,868 | 0,887 | 0,925 | 0,872 | 0,978 | 0,928 | 0,848 | 1 |
| RSAD2, IFI27, OAS1, IFIT1, ISG15, OLAH, RETN, MMP8 | 0,877 | 0,868 | 0,887 | 0,919 | 0,859 | 0,978 | 0,950 | 0,893 | 1 |
| RSAD2, IFI27, IFIT1, SLC1A2, IL1R2, FAM20A, RETN, MMP8 | 0,877 | 0,868 | 0,887 | 0,932 | 0,883 | 0,981 | 0,930 | 0,850 | 1 |
| RSAD2, IFI27, OAS1, HERC6, SLC1A2, IL1R2, OLAH, MMP8 | 0,877 | 0,868 | 0,887 | 0,929 | 0,880 | 0,977 | 0,935 | 0,869 | 1 |
| RSAD2, IFI27, SIGLEC1, ISG15, HERC6, SLC1A2, OLAH, RETN | 0,877 | 0,868 | 0,887 | 0,929 | 0,879 | 0,979 | 0,941 | 0,883 | 0,999 |
| RSAD2, IFI27, IFI44L, SIGLEC1, ISG15, OLAH, RETN, MMP8 | 0,877 | 0,868 | 0,886 | 0,921 | 0,864 | 0,979 | 0,957 | 0,905 | 1 |
| IFI27, IFI44L, ISG15, HERC6, SLC1A2, OLAH, RETN, MMP8 | 0,877 | 0,868 | 0,887 | 0,927 | 0,876 | 0,979 | 0,937 | 0,874 | 1 |
| RSAD2, IFI27, IFIT1, IFI44L, SLC1A2, OLAH, FAM20A, RETN | 0,877 | 0,868 | 0,887 | 0,926 | 0,870 | 0,983 | 0,922 | 0,841 | 1 |
| RSAD2, IFI27, OAS1, HERC6, SLC1A2, IL1R2, FAM20A, MMP8 | 0,877 | 0,868 | 0,887 | 0,931 | 0,886 | 0,976 | 0,921 | 0,829 | 1 |
| IFI27, OAS1, IFIT1, SLC1A2, IL1R2, FAM20A, RETN, MMP8 | 0,877 | 0,868 | 0,887 | 0,932 | 0,883 | 0,981 | 0,928 | 0,847 | 1 |
| RSAD2, IFI27, OAS1, IFIT1, SIGLEC1, IL1R2, OLAH, RETN | 0,877 | 0,868 | 0,886 | 0,919 | 0,863 | 0,974 | 0,950 | 0,893 | 1 |
| IFI27, IFIT1, IFI44L, ISG15, IL1R2, OLAH, RETN, MMP8 | 0,877 | 0,868 | 0,887 | 0,918 | 0,861 | 0,975 | 0,948 | 0,894 | 1 |
| RSAD2, IFI27, OAS1, IFIT1, SLC1A2, OLAH, FAM20A, RETN | 0,877 | 0,868 | 0,887 | 0,924 | 0,867 | 0,981 | 0,924 | 0,844 | 1 |
| IFI27, OAS1, IFIT1, IFI44L, SLC1A2, IL1R2, FAM20A, MMP8 | 0,877 | 0,868 | 0,887 | 0,928 | 0,880 | 0,976 | 0,926 | 0,840 | 1 |
| SIGLEC1, ISG15, HERC6, IL1R2, OLAH, FAM20A, RETN, MMP8 | 0,877 | 0,868 | 0,887 | 0,916 | 0,857 | 0,976 | 0,965 | 0,911 | 1 |
| RSAD2, SIGLEC1, ISG15, HERC6, IL1R2, FAM20A, RETN, MMP8 | 0,877 | 0,868 | 0,887 | 0,920 | 0,863 | 0,977 | 0,958 | 0,896 | 1 |
| IFI27, OAS1, IFI44L, SIGLEC1, ISG15, HERC6, IL1R2, MMP8 | 0,877 | 0,869 | 0,885 | 0,922 | 0,876 | 0,969 | 0,940 | 0,888 | 0,993 |
| IFIT1, IFI44L, SIGLEC1, ISG15, SLC1A2, IL1R2, FAM20A, MMP8 | 0,877 | 0,868 | 0,886 | 0,926 | 0,880 | 0,973 | 0,961 | 0,897 | 1 |
| IFI27, OAS1, IFI44L, ISG15, IL1R2, OLAH, RETN, MMP8 | 0,877 | 0,868 | 0,886 | 0,924 | 0,868 | 0,979 | 0,946 | 0,890 | 1 |
| RSAD2, IFI27, IFIT1, SIGLEC1, ISG15, OLAH, RETN, MMP8 | 0,877 | 0,868 | 0,887 | 0,920 | 0,860 | 0,979 | 0,958 | 0,905 | 1 |
| IFI27, IFIT1, SIGLEC1, SLC1A2, IL1R2, OLAH, RETN, MMP8 | 0,877 | 0,868 | 0,887 | 0,929 | 0,878 | 0,979 | 0,959 | 0,909 | 1 |
| IFI27, IFI44L, ISG15, HERC6, SLC1A2, IL1R2, FAM20A, MMP8 | 0,877 | 0,867 | 0,887 | 0,930 | 0,883 | 0,976 | 0,927 | 0,840 | 1 |
| OAS1, SIGLEC1, HERC6, SLC1A2, IL1R2, OLAH, FAM20A, MMP8 | 0,877 | 0,867 | 0,887 | 0,923 | 0,874 | 0,972 | 0,960 | 0,900 | 1 |
| RSAD2, IFI27, IFIT1, SIGLEC1, SLC1A2, IL1R2, OLAH, MMP8 | 0,877 | 0,868 | 0,886 | 0,927 | 0,878 | 0,977 | 0,947 | 0,893 | 1 |
| RSAD2, IFI27, IFIT1, IFI44L, HERC6, SLC1A2, IL1R2, FAM20A | 0,877 | 0,868 | 0,887 | 0,928 | 0,879 | 0,976 | 0,925 | 0,838 | 1 |

| Genes | | | | | | | | |
|---|---|---|---|---|---|---|---|---|
| IFI27, IFIT1, IFI44L, SIGLEC1, SLC1A2, IL1R2, OLAH, MMP8 | 0,877 | 0,869 | 0,886 | 0,927 | 0,877 | 0,976 | 0,950 | 0,899 | 1 |
| RSAD2, IFI27, IFIT1, SIGLEC1, HERC6, SLC1A2, OLAH, RETN | 0,877 | 0,868 | 0,887 | 0,929 | 0,879 | 0,978 | 0,945 | 0,889 | 1 |
| OAS1, IFI44L, SIGLEC1, HERC6, IL1R2, FAM20A, RETN, MMP8 | 0,877 | 0,868 | 0,887 | 0,919 | 0,862 | 0,976 | 0,962 | 0,905 | 1 |
| RSAD2, IFI27, OAS1, IFI44L, HERC6, SLC1A2, IL1R2, FAM20A | 0,877 | 0,867 | 0,887 | 0,930 | 0,884 | 0,975 | 0,926 | 0,839 | 1 |
| RSAD2, IFI27, OAS1, IFIT1, IFI44L, SIGLEC1, FAM20A, MMP8 | 0,877 | 0,867 | 0,887 | 0,914 | 0,849 | 0,980 | 0,946 | 0,877 | 1 |
| RSAD2, IFI27, IFI44L, SIGLEC1, ISG15, HERC6, SLC1A2, OLAH | 0,877 | 0,868 | 0,886 | 0,931 | 0,881 | 0,980 | 0,940 | 0,883 | 0,998 |
| RSAD2, IFI27, OAS1, IFI44L, SIGLEC1, HERC6, SLC1A2, OLAH | 0,877 | 0,868 | 0,886 | 0,929 | 0,879 | 0,979 | 0,947 | 0,892 | 1 |
| RSAD2, IFI27, OAS1, IFIT1, SIGLEC1, SLC1A2, IL1R2, OLAH | 0,877 | 0,868 | 0,886 | 0,925 | 0,875 | 0,976 | 0,951 | 0,897 | 1 |
| IFI27, OAS1, IFIT1, HERC6, SLC1A2, IL1R2, FAM20A, RETN | 0,877 | 0,867 | 0,887 | 0,932 | 0,884 | 0,980 | 0,926 | 0,842 | 1 |
| IFI27, OAS1, ISG15, HERC6, SLC1A2, IL1R2, FAM20A, MMP8 | 0,877 | 0,867 | 0,887 | 0,930 | 0,884 | 0,977 | 0,925 | 0,839 | 1 |
| RSAD2, IFI27, IFIT1, IFI44L, ISG15, HERC6, IL1R2, OLAH | 0,877 | 0,869 | 0,886 | 0,925 | 0,874 | 0,976 | 0,935 | 0,876 | 0,993 |
| RSAD2, IFI27, OAS1, IFI44L, SIGLEC1, ISG15, OLAH, RETN | 0,877 | 0,868 | 0,886 | 0,918 | 0,860 | 0,977 | 0,946 | 0,885 | 1 |
| IFI27, ISG15, HERC6, SLC1A2, IL1R2, FAM20A, RETN, MMP8 | 0,877 | 0,867 | 0,887 | 0,934 | 0,886 | 0,982 | 0,929 | 0,844 | 1 |
| IFIT1, SIGLEC1, ISG15, HERC6, IL1R2, OLAH, FAM20A, RETN | 0,877 | 0,868 | 0,886 | 0,919 | 0,862 | 0,977 | 0,952 | 0,895 | 1 |
| OAS1, SIGLEC1, ISG15, HERC6, IL1R2, FAM20A, RETN, MMP8 | 0,877 | 0,868 | 0,886 | 0,917 | 0,859 | 0,975 | 0,962 | 0,904 | 1 |
| RSAD2, IFI27, IFIT1, SIGLEC1, HERC6, SLC1A2, FAM20A, MMP8 | 0,877 | 0,868 | 0,886 | 0,927 | 0,877 | 0,978 | 0,935 | 0,856 | 1 |
| RSAD2, IFI27, SIGLEC1, SLC1A2, IL1R2, OLAH, RETN, MMP8 | 0,877 | 0,868 | 0,887 | 0,929 | 0,879 | 0,979 | 0,958 | 0,906 | 1 |
| RSAD2, OAS1, SIGLEC1, HERC6, SLC1A2, IL1R2, FAM20A, MMP8 | 0,877 | 0,867 | 0,887 | 0,924 | 0,876 | 0,972 | 0,959 | 0,895 | 1 |
| IFI27, OAS1, IFIT1, ISG15, HERC6, SLC1A2, IL1R2, FAM20A | 0,877 | 0,867 | 0,887 | 0,930 | 0,883 | 0,977 | 0,924 | 0,838 | 1 |
| RSAD2, IFI27, OAS1, IFIT1, SIGLEC1, ISG15, IL1R2, OLAH | 0,877 | 0,869 | 0,886 | 0,921 | 0,869 | 0,973 | 0,946 | 0,892 | 0,999 |
| IFI27, OAS1, IFI44L, SIGLEC1, SLC1A2, OLAH, RETN, MMP8 | 0,877 | 0,868 | 0,887 | 0,928 | 0,876 | 0,980 | 0,957 | 0,901 | 1 |
| RSAD2, IFI27, OAS1, IFIT1, SIGLEC1, HERC6, IL1R2, MMP8 | 0,877 | 0,869 | 0,886 | 0,921 | 0,874 | 0,969 | 0,951 | 0,905 | 0,997 |
| IFI27, IFI44L, SIGLEC1, HERC6, SLC1A2, IL1R2, OLAH, RETN | 0,877 | 0,868 | 0,887 | 0,924 | 0,873 | 0,976 | 0,948 | 0,893 | 1 |
| RSAD2, OAS1, IFIT1, IFI44L, SIGLEC1, HERC6, OLAH, FAM20A | 0,877 | 0,868 | 0,886 | 0,915 | 0,856 | 0,975 | 0,943 | 0,884 | 1 |
| RSAD2, IFI27, OAS1, HERC6, SLC1A2, IL1R2, FAM20A, RETN | 0,877 | 0,868 | 0,886 | 0,932 | 0,885 | 0,980 | 0,923 | 0,834 | 1 |
| IFI27, OAS1, IFI44L, HERC6, SLC1A2, IL1R2, FAM20A, RETN | 0,877 | 0,867 | 0,887 | 0,932 | 0,884 | 0,980 | 0,925 | 0,840 | 1 |
| IFI27, IFI44L, ISG15, SLC1A2, IL1R2, OLAH, RETN, MMP8 | 0,877 | 0,868 | 0,886 | 0,927 | 0,876 | 0,979 | 0,936 | 0,870 | 1 |
| RSAD2, OAS1, SIGLEC1, SLC1A2, IL1R2, OLAH, FAM20A, MMP8 | 0,877 | 0,867 | 0,887 | 0,924 | 0,875 | 0,973 | 0,959 | 0,898 | 1 |

| | | | | | | | | |
|---|---|---|---|---|---|---|---|---|
| RSAD2, IFI27, OAS1, IFIT1, SIGLEC1, ISG15, SLC1A2, OLAH | 0,877 | 0,868 | 0,886 | 0,929 | 0,878 | 0,980 | 0,943 | 0,886 | 1 |
| IFI27, IFIT1, IFI44L, SIGLEC1, SLC1A2, OLAH, RETN, MMP8 | 0,877 | 0,868 | 0,887 | 0,928 | 0,876 | 0,980 | 0,959 | 0,908 | 1 |
| IFI27, IFI44L, SIGLEC1, ISG15, HERC6, SLC1A2, IL1R2, OLAH | 0,877 | 0,868 | 0,886 | 0,929 | 0,880 | 0,977 | 0,942 | 0,887 | 0,997 |
| RSAD2, IFI27, OAS1, SIGLEC1, SLC1A2, IL1R2, OLAH, RETN | 0,877 | 0,868 | 0,886 | 0,928 | 0,877 | 0,979 | 0,950 | 0,888 | 1 |
| RSAD2, IFI27, OAS1, ISG15, HERC6, SLC1A2, OLAH, MMP8 | 0,877 | 0,868 | 0,886 | 0,929 | 0,878 | 0,979 | 0,930 | 0,863 | 0,998 |
| RSAD2, IFI27, OAS1, IFI44L, HERC6, SLC1A2, OLAH, RETN | 0,877 | 0,868 | 0,886 | 0,927 | 0,875 | 0,979 | 0,933 | 0,861 | 1 |
| IFI44L, SIGLEC1, HERC6, SLC1A2, IL1R2, OLAH, FAM20A, MMP8 | 0,877 | 0,867 | 0,887 | 0,923 | 0,874 | 0,973 | 0,959 | 0,897 | 1 |
| IFI27, OAS1, IFIT1, IFI44L, SIGLEC1, ISG15, SLC1A2, OLAH | 0,877 | 0,868 | 0,886 | 0,929 | 0,879 | 0,979 | 0,945 | 0,887 | 1 |
| IFIT1, IFI44L, SIGLEC1, SLC1A2, OLAH, FAM20A, RETN, MMP8 | 0,877 | 0,867 | 0,887 | 0,924 | 0,872 | 0,977 | 0,949 | 0,890 | 1 |
| RSAD2, IFI27, OAS1, IFIT1, IFI44L, SIGLEC1, OLAH, MMP8 | 0,877 | 0,869 | 0,885 | 0,919 | 0,862 | 0,975 | 0,951 | 0,901 | 1 |
| OAS1, IFIT1, IFI44L, SIGLEC1, HERC6, OLAH, FAM20A, RETN | 0,877 | 0,868 | 0,886 | 0,915 | 0,855 | 0,976 | 0,941 | 0,881 | 1 |
| OAS1, IFIT1, IFI44L, SIGLEC1, HERC6, IL1R2, FAM20A, RETN | 0,877 | 0,868 | 0,886 | 0,918 | 0,861 | 0,976 | 0,950 | 0,893 | 1 |
| RSAD2, IFIT1, SIGLEC1, ISG15, HERC6, OLAH, FAM20A, MMP8 | 0,877 | 0,867 | 0,887 | 0,916 | 0,858 | 0,975 | 0,953 | 0,899 | 1 |
| RSAD2, IFI27, IFIT1, IFI44L, ISG15, HERC6, OLAH, MMP8 | 0,877 | 0,868 | 0,886 | 0,924 | 0,871 | 0,976 | 0,934 | 0,874 | 0,993 |
| IFI27, IFIT1, ISG15, HERC6, SLC1A2, OLAH, RETN, MMP8 | 0,877 | 0,867 | 0,887 | 0,927 | 0,876 | 0,979 | 0,941 | 0,881 | 1 |
| RSAD2, IFI27, OAS1, SIGLEC1, SLC1A2, IL1R2, OLAH, MMP8 | 0,877 | 0,868 | 0,886 | 0,926 | 0,877 | 0,976 | 0,951 | 0,897 | 1 |
| IFI27, OAS1, IFIT1, ISG15, HERC6, SLC1A2, IL1R2, OLAH | 0,877 | 0,867 | 0,887 | 0,927 | 0,878 | 0,977 | 0,929 | 0,862 | 0,997 |
| IFI27, OAS1, IFI44L, SIGLEC1, ISG15, HERC6, SLC1A2, RETN | 0,877 | 0,868 | 0,886 | 0,923 | 0,870 | 0,977 | 0,939 | 0,875 | 1 |
| RSAD2, IFI27, IFIT1, SIGLEC1, ISG15, SLC1A2, FAM20A, RETN | 0,877 | 0,868 | 0,886 | 0,926 | 0,871 | 0,981 | 0,938 | 0,862 | 1 |
| IFI27, ISG15, HERC6, SLC1A2, IL1R2, OLAH, RETN, MMP8 | 0,877 | 0,868 | 0,886 | 0,929 | 0,879 | 0,979 | 0,941 | 0,881 | 1 |
| RSAD2, IFI27, IFI44L, SIGLEC1, HERC6, SLC1A2, FAM20A, RETN | 0,877 | 0,867 | 0,887 | 0,927 | 0,875 | 0,980 | 0,943 | 0,870 | 1 |
| RSAD2, IFI44L, SIGLEC1, ISG15, HERC6, IL1R2, FAM20A, MMP8 | 0,877 | 0,868 | 0,886 | 0,918 | 0,861 | 0,975 | 0,960 | 0,900 | 1 |
| RSAD2, IFI27, IFIT1, IFI44L, SIGLEC1, SLC1A2, FAM20A, RETN | 0,877 | 0,867 | 0,887 | 0,924 | 0,869 | 0,980 | 0,940 | 0,868 | 1 |
| IFI44L, SIGLEC1, ISG15, IL1R2, OLAH, FAM20A, RETN, MMP8 | 0,877 | 0,868 | 0,886 | 0,917 | 0,858 | 0,975 | 0,964 | 0,911 | 1 |
| RSAD2, IFI27, OAS1, SIGLEC1, SLC1A2, OLAH, RETN, MMP8 | 0,877 | 0,867 | 0,886 | 0,927 | 0,874 | 0,980 | 0,957 | 0,900 | 1 |
| IFI27, OAS1, IFIT1, SIGLEC1, ISG15, HERC6, IL1R2, MMP8 | 0,877 | 0,869 | 0,885 | 0,921 | 0,874 | 0,968 | 0,949 | 0,902 | 0,996 |
| RSAD2, IFI27, OAS1, IFIT1, IFI44L, IL1R2, OLAH, MMP8 | 0,877 | 0,868 | 0,886 | 0,922 | 0,870 | 0,974 | 0,942 | 0,889 | 0,995 |
| IFIT1, SIGLEC1, HERC6, SLC1A2, OLAH, FAM20A, RETN, MMP8 | 0,877 | 0,867 | 0,887 | 0,927 | 0,877 | 0,978 | 0,952 | 0,884 | 1 |

| | | | | | | | | | |
|---|---|---|---|---|---|---|---|---|---|
| IFI44L, SIGLEC1, HERC6, IL1R2, OLAH, FAM20A, RETN, MMP8 | 0,877 | 0,867 | 0,887 | 0,920 | 0,863 | 0,977 | 0,960 | 0,900 | 1 |
| IFI27, OAS1, IFIT1, IFI44L, SIGLEC1, SLC1A2, OLAH, RETN | 0,877 | 0,868 | 0,886 | 0,926 | 0,874 | 0,978 | 0,948 | 0,886 | 1 |
| RSAD2, IFI27, IFI44L, SIGLEC1, ISG15, SLC1A2, OLAH, MMP8 | 0,877 | 0,868 | 0,885 | 0,930 | 0,880 | 0,980 | 0,942 | 0,885 | 1 |
| IFI27, IFI44L, HERC6, SLC1A2, IL1R2, OLAH, RETN, MMP8 | 0,877 | 0,867 | 0,886 | 0,926 | 0,874 | 0,978 | 0,937 | 0,873 | 1 |
| IFI27, IFI44L, SIGLEC1, HERC6, SLC1A2, IL1R2, OLAH, MMP8 | 0,877 | 0,868 | 0,886 | 0,927 | 0,878 | 0,976 | 0,951 | 0,902 | 1 |
| RSAD2, IFIT1, SIGLEC1, IL1R2, OLAH, FAM20A, RETN, MMP8 | 0,877 | 0,868 | 0,886 | 0,921 | 0,864 | 0,978 | 0,968 | 0,918 | 1 |
| RSAD2, IFI27, OAS1, IFIT1, IFI44L, SIGLEC1, SLC1A2, OLAH | 0,877 | 0,868 | 0,885 | 0,926 | 0,874 | 0,978 | 0,953 | 0,900 | 1 |
| IFI27, SIGLEC1, HERC6, SLC1A2, IL1R2, OLAH, RETN, MMP8 | 0,877 | 0,867 | 0,886 | 0,927 | 0,876 | 0,979 | 0,959 | 0,910 | 1 |
| RSAD2, IFI27, IFIT1, SIGLEC1, ISG15, SLC1A2, IL1R2, OLAH | 0,877 | 0,868 | 0,886 | 0,929 | 0,879 | 0,978 | 0,939 | 0,880 | 0,998 |
| RSAD2, IFI27, IFIT1, IFI44L, SIGLEC1, HERC6, IL1R2, MMP8 | 0,877 | 0,869 | 0,885 | 0,923 | 0,875 | 0,971 | 0,941 | 0,890 | 0,993 |
| IFI27, OAS1, IFI44L, ISG15, HERC6, SLC1A2, OLAH, RETN | 0,877 | 0,867 | 0,886 | 0,928 | 0,878 | 0,978 | 0,930 | 0,861 | 1,000 |
| RSAD2, IFI27, OAS1, IFI44L, SLC1A2, OLAH, FAM20A, RETN | 0,877 | 0,867 | 0,886 | 0,927 | 0,870 | 0,984 | 0,924 | 0,845 | 1 |
| IFI27, OAS1, SIGLEC1, ISG15, HERC6, SLC1A2, RETN, MMP8 | 0,877 | 0,867 | 0,886 | 0,922 | 0,866 | 0,977 | 0,948 | 0,885 | 1 |
| IFI27, IFIT1, IFI44L, HERC6, SLC1A2, OLAH, RETN, MMP8 | 0,877 | 0,867 | 0,886 | 0,924 | 0,870 | 0,978 | 0,940 | 0,879 | 1 |
| RSAD2, IFI27, SIGLEC1, ISG15, HERC6, SLC1A2, FAM20A, RETN | 0,877 | 0,867 | 0,887 | 0,925 | 0,871 | 0,979 | 0,943 | 0,870 | 1 |
| RSAD2, IFI27, IFI44L, ISG15, SLC1A2, IL1R2, FAM20A, MMP8 | 0,877 | 0,867 | 0,887 | 0,930 | 0,883 | 0,976 | 0,926 | 0,839 | 1 |
| RSAD2, IFI27, OAS1, IFIT1, HERC6, SLC1A2, OLAH, MMP8 | 0,877 | 0,867 | 0,886 | 0,927 | 0,877 | 0,978 | 0,935 | 0,869 | 1 |
| RSAD2, IFI27, SIGLEC1, ISG15, SLC1A2, OLAH, RETN, MMP8 | 0,877 | 0,867 | 0,886 | 0,928 | 0,876 | 0,980 | 0,953 | 0,898 | 1 |
| IFI27, IFIT1, IFI44L, SIGLEC1, ISG15, SLC1A2, FAM20A, MMP8 | 0,877 | 0,867 | 0,886 | 0,922 | 0,865 | 0,978 | 0,941 | 0,867 | 1 |
| IFI27, IFIT1, IFI44L, SIGLEC1, ISG15, HERC6, IL1R2, MMP8 | 0,877 | 0,869 | 0,885 | 0,923 | 0,875 | 0,971 | 0,941 | 0,890 | 0,993 |
| RSAD2, IFI27, ISG15, HERC6, SLC1A2, IL1R2, OLAH, MMP8 | 0,877 | 0,868 | 0,886 | 0,929 | 0,879 | 0,978 | 0,928 | 0,861 | 0,995 |
| RSAD2, IFI27, OAS1, SIGLEC1, ISG15, IL1R2, OLAH, RETN | 0,877 | 0,868 | 0,886 | 0,919 | 0,863 | 0,975 | 0,951 | 0,896 | 1 |
| RSAD2, IFI27, OAS1, IFI44L, IL1R2, OLAH, RETN, MMP8 | 0,877 | 0,867 | 0,886 | 0,923 | 0,867 | 0,978 | 0,951 | 0,897 | 1 |
| IFI27, SIGLEC1, ISG15, HERC6, SLC1A2, IL1R2, OLAH, RETN | 0,877 | 0,867 | 0,886 | 0,929 | 0,879 | 0,979 | 0,939 | 0,877 | 1 |
| IFI27, OAS1, IFI44L, SIGLEC1, ISG15, SLC1A2, OLAH, RETN | 0,877 | 0,868 | 0,886 | 0,929 | 0,879 | 0,980 | 0,946 | 0,885 | 1 |
| RSAD2, IFI27, IFIT1, SIGLEC1, HERC6, SLC1A2, OLAH, MMP8 | 0,877 | 0,867 | 0,886 | 0,927 | 0,877 | 0,978 | 0,939 | 0,882 | 0,996 |
| RSAD2, IFI27, IFIT1, HERC6, SLC1A2, IL1R2, OLAH, MMP8 | 0,877 | 0,867 | 0,886 | 0,927 | 0,878 | 0,976 | 0,932 | 0,867 | 0,996 |
| RSAD2, IFI27, IFI44L, SIGLEC1, ISG15, SLC1A2, OLAH, RETN | 0,877 | 0,868 | 0,885 | 0,928 | 0,877 | 0,979 | 0,946 | 0,887 | 1 |

| | | | | | | | | |
|---|---|---|---|---|---|---|---|---|
| RSAD2, IFI27, OAS1, IFI44L, SIGLEC1, ISG15, HERC6, IL1R2 | 0,877 | 0,868 | 0,885 | 0,924 | 0,877 | 0,972 | 0,943 | 0,893 | 0,994 |
| OAS1, IFIT1, IFI44L, SIGLEC1, ISG15, HERC6, FAM20A, RETN | 0,877 | 0,867 | 0,886 | 0,919 | 0,862 | 0,977 | 0,941 | 0,877 | 1 |
| RSAD2, IFI27, IFIT1, SIGLEC1, SLC1A2, OLAH, RETN, MMP8 | 0,877 | 0,867 | 0,886 | 0,929 | 0,878 | 0,981 | 0,957 | 0,903 | 1 |
| RSAD2, IFI27, SIGLEC1, ISG15, SLC1A2, IL1R2, OLAH, RETN | 0,877 | 0,868 | 0,886 | 0,929 | 0,878 | 0,979 | 0,942 | 0,883 | 1 |
| IFI27, OAS1, IFIT1, IFI44L, ISG15, OLAH, RETN, MMP8 | 0,877 | 0,867 | 0,886 | 0,919 | 0,859 | 0,978 | 0,950 | 0,893 | 1 |
| RSAD2, SIGLEC1, HERC6, IL1R2, OLAH, FAM20A, RETN, MMP8 | 0,877 | 0,867 | 0,886 | 0,920 | 0,864 | 0,977 | 0,961 | 0,905 | 1 |
| RSAD2, IFI27, ISG15, HERC6, SLC1A2, IL1R2, FAM20A, MMP8 | 0,877 | 0,867 | 0,886 | 0,931 | 0,885 | 0,977 | 0,926 | 0,839 | 1 |
| RSAD2, IFI27, OAS1, IFIT1, SIGLEC1, SLC1A2, OLAH, MMP8 | 0,877 | 0,868 | 0,886 | 0,928 | 0,876 | 0,980 | 0,947 | 0,891 | 1 |
| RSAD2, IFI27, OAS1, ISG15, HERC6, SLC1A2, IL1R2, FAM20A | 0,877 | 0,867 | 0,886 | 0,931 | 0,886 | 0,976 | 0,921 | 0,832 | 1 |
| RSAD2, IFI27, IFIT1, SIGLEC1, ISG15, HERC6, SLC1A2, FAM20A | 0,877 | 0,867 | 0,886 | 0,927 | 0,878 | 0,977 | 0,936 | 0,857 | 1 |
| RSAD2, IFI27, OAS1, IFIT1, IFI44L, SIGLEC1, ISG15, OLAH | 0,877 | 0,868 | 0,885 | 0,919 | 0,863 | 0,976 | 0,941 | 0,885 | 0,997 |
| RSAD2, IFI27, OAS1, IFI44L, ISG15, HERC6, RETN, MMP8 | 0,877 | 0,867 | 0,886 | 0,919 | 0,860 | 0,979 | 0,936 | 0,868 | 1 |
| IFI27, OAS1, IFIT1, IFI44L, SIGLEC1, SLC1A2, OLAH, MMP8 | 0,877 | 0,867 | 0,886 | 0,927 | 0,877 | 0,978 | 0,948 | 0,892 | 1 |
| IFI27, IFIT1, ISG15, HERC6, SLC1A2, IL1R2, OLAH, MMP8 | 0,877 | 0,867 | 0,886 | 0,928 | 0,879 | 0,977 | 0,929 | 0,864 | 0,995 |
| RSAD2, IFI27, IFI44L, ISG15, HERC6, SLC1A2, IL1R2, FAM20A | 0,877 | 0,867 | 0,886 | 0,929 | 0,881 | 0,976 | 0,925 | 0,838 | 1 |
| RSAD2, IFI27, OAS1, IFIT1, HERC6, SLC1A2, IL1R2, OLAH | 0,877 | 0,867 | 0,886 | 0,929 | 0,880 | 0,977 | 0,937 | 0,873 | 1 |
| IFI27, OAS1, IFI44L, SIGLEC1, ISG15, IL1R2, RETN, MMP8 | 0,877 | 0,868 | 0,885 | 0,924 | 0,872 | 0,976 | 0,962 | 0,922 | 1 |
| OAS1, IFIT1, SIGLEC1, ISG15, HERC6, IL1R2, FAM20A, RETN | 0,877 | 0,867 | 0,886 | 0,919 | 0,863 | 0,976 | 0,953 | 0,897 | 1 |
| OAS1, IFIT1, IFI44L, SIGLEC1, HERC6, SLC1A2, OLAH, FAM20A | 0,876 | 0,866 | 0,887 | 0,925 | 0,875 | 0,975 | 0,928 | 0,856 | 1 |
| RSAD2, IFI27, IFIT1, IFI44L, IL1R2, OLAH, RETN, MMP8 | 0,876 | 0,867 | 0,886 | 0,922 | 0,867 | 0,977 | 0,950 | 0,895 | 1 |
| RSAD2, IFI27, OAS1, ISG15, HERC6, SLC1A2, IL1R2, OLAH | 0,876 | 0,867 | 0,886 | 0,929 | 0,880 | 0,977 | 0,928 | 0,859 | 0,997 |
| RSAD2, IFI27, OAS1, IFI44L, SIGLEC1, HERC6, SLC1A2, RETN | 0,876 | 0,868 | 0,885 | 0,922 | 0,867 | 0,977 | 0,942 | 0,875 | 1 |
| RSAD2, IFI27, IFIT1, SIGLEC1, HERC6, SLC1A2, IL1R2, OLAH | 0,876 | 0,867 | 0,886 | 0,928 | 0,879 | 0,977 | 0,941 | 0,886 | 0,997 |
| RSAD2, IFI27, IFIT1, SIGLEC1, SLC1A2, IL1R2, OLAH, RETN | 0,876 | 0,867 | 0,886 | 0,925 | 0,874 | 0,977 | 0,950 | 0,894 | 1 |
| RSAD2, IFI27, IFI44L, SIGLEC1, SLC1A2, OLAH, RETN, MMP8 | 0,876 | 0,867 | 0,886 | 0,929 | 0,877 | 0,980 | 0,959 | 0,907 | 1 |
| RSAD2, IFI27, ISG15, HERC6, SLC1A2, IL1R2, FAM20A, RETN | 0,876 | 0,867 | 0,886 | 0,933 | 0,885 | 0,981 | 0,925 | 0,840 | 1 |
| RSAD2, IFI27, SIGLEC1, ISG15, SLC1A2, FAM20A, RETN, MMP8 | 0,876 | 0,866 | 0,887 | 0,926 | 0,870 | 0,982 | 0,943 | 0,868 | 1 |
| IFI27, OAS1, IFIT1, SLC1A2, IL1R2, OLAH, RETN, MMP8 | 0,876 | 0,867 | 0,886 | 0,926 | 0,874 | 0,977 | 0,941 | 0,879 | 1 |

| | | | | | | | | |
|---|---|---|---|---|---|---|---|---|
| RSAD2, SIGLEC1, ISG15, HERC6, SLC1A2, IL1R2, FAM20A, MMP8 | 0,876 | 0,867 | 0,886 | 0,926 | 0,880 | 0,973 | 0,959 | 0,893 | 1 |
| RSAD2, IFI27, OAS1, IFI44L, SLC1A2, IL1R2, FAM20A, MMP8 | 0,876 | 0,867 | 0,886 | 0,928 | 0,881 | 0,975 | 0,926 | 0,839 | 1 |
| IFI44L, SIGLEC1, ISG15, HERC6, SLC1A2, IL1R2, FAM20A, MMP8 | 0,876 | 0,867 | 0,886 | 0,925 | 0,877 | 0,973 | 0,959 | 0,893 | 1 |
| RSAD2, IFIT1, SIGLEC1, ISG15, IL1R2, FAM20A, RETN, MMP8 | 0,876 | 0,867 | 0,885 | 0,921 | 0,864 | 0,978 | 0,967 | 0,916 | 1 |
| RSAD2, IFI27, OAS1, IFIT1, IFI44L, OLAH, RETN, MMP8 | 0,876 | 0,867 | 0,886 | 0,921 | 0,862 | 0,980 | 0,951 | 0,893 | 1 |
| RSAD2, IFI27, OAS1, SIGLEC1, SLC1A2, FAM20A, RETN, MMP8 | 0,876 | 0,866 | 0,887 | 0,924 | 0,867 | 0,982 | 0,946 | 0,874 | 1 |
| RSAD2, IFI27, OAS1, IFIT1, SIGLEC1, ISG15, OLAH, MMP8 | 0,876 | 0,867 | 0,885 | 0,919 | 0,862 | 0,975 | 0,941 | 0,885 | 0,998 |
| RSAD2, IFI27, OAS1, IFIT1, IFI44L, SIGLEC1, IL1R2, RETN | 0,876 | 0,868 | 0,885 | 0,917 | 0,862 | 0,973 | 0,957 | 0,907 | 1 |
| RSAD2, IFI27, IFI44L, SIGLEC1, HERC6, SLC1A2, IL1R2, OLAH | 0,876 | 0,867 | 0,886 | 0,927 | 0,877 | 0,976 | 0,946 | 0,893 | 0,998 |
| IFI27, OAS1, IFI44L, SIGLEC1, HERC6, SLC1A2, IL1R2, OLAH | 0,876 | 0,867 | 0,886 | 0,928 | 0,879 | 0,976 | 0,941 | 0,884 | 0,998 |
| RSAD2, IFI27, IFIT1, HERC6, SLC1A2, IL1R2, OLAH, RETN | 0,876 | 0,867 | 0,886 | 0,925 | 0,874 | 0,976 | 0,935 | 0,870 | 1 |
| RSAD2, IFI27, OAS1, ISG15, IL1R2, OLAH, RETN, MMP8 | 0,876 | 0,867 | 0,886 | 0,922 | 0,866 | 0,978 | 0,951 | 0,897 | 1 |
| IFI27, IFIT1, IFI44L, SIGLEC1, HERC6, SLC1A2, OLAH, MMP8 | 0,876 | 0,867 | 0,886 | 0,927 | 0,877 | 0,978 | 0,942 | 0,887 | 0,998 |
| RSAD2, IFI27, IFIT1, ISG15, SLC1A2, IL1R2, FAM20A, MMP8 | 0,876 | 0,867 | 0,885 | 0,928 | 0,880 | 0,976 | 0,926 | 0,840 | 1 |
| RSAD2, IFI27, IFIT1, IFI44L, SIGLEC1, ISG15, FAM20A, MMP8 | 0,876 | 0,867 | 0,886 | 0,914 | 0,849 | 0,979 | 0,945 | 0,876 | 1 |
| RSAD2, IFI27, OAS1, IFIT1, SIGLEC1, IL1R2, RETN, MMP8 | 0,876 | 0,867 | 0,885 | 0,921 | 0,867 | 0,975 | 0,965 | 0,920 | 1 |
| IFI27, IFI44L, SIGLEC1, HERC6, SLC1A2, FAM20A, RETN, MMP8 | 0,876 | 0,866 | 0,887 | 0,927 | 0,872 | 0,982 | 0,948 | 0,871 | 1 |
| SIGLEC1, ISG15, HERC6, SLC1A2, IL1R2, OLAH, FAM20A, MMP8 | 0,876 | 0,867 | 0,886 | 0,924 | 0,876 | 0,973 | 0,959 | 0,895 | 1 |
| RSAD2, IFI27, IFI44L, SIGLEC1, SLC1A2, IL1R2, OLAH, MMP8 | 0,876 | 0,868 | 0,885 | 0,927 | 0,877 | 0,977 | 0,950 | 0,899 | 1 |
| RSAD2, IFI27, IFIT1, SIGLEC1, ISG15, IL1R2, OLAH, MMP8 | 0,876 | 0,868 | 0,885 | 0,923 | 0,872 | 0,974 | 0,945 | 0,894 | 0,995 |
| IFI27, OAS1, IFI44L, SIGLEC1, ISG15, SLC1A2, IL1R2, OLAH | 0,876 | 0,867 | 0,885 | 0,928 | 0,878 | 0,978 | 0,942 | 0,885 | 0,999 |
| RSAD2, IFI27, OAS1, SIGLEC1, ISG15, SLC1A2, OLAH, RETN | 0,876 | 0,867 | 0,885 | 0,929 | 0,876 | 0,981 | 0,947 | 0,883 | 1 |
| RSAD2, IFI27, IFI44L, SIGLEC1, ISG15, SLC1A2, IL1R2, OLAH | 0,876 | 0,868 | 0,885 | 0,929 | 0,878 | 0,979 | 0,943 | 0,889 | 0,998 |
| OAS1, IFIT1, SIGLEC1, ISG15, HERC6, OLAH, FAM20A, RETN | 0,876 | 0,867 | 0,886 | 0,915 | 0,855 | 0,975 | 0,947 | 0,889 | 1 |
| RSAD2, IFI27, IFIT1, IFI44L, SIGLEC1, ISG15, OLAH, RETN | 0,876 | 0,868 | 0,885 | 0,920 | 0,863 | 0,978 | 0,948 | 0,891 | 1 |
| IFI27, IFI44L, SIGLEC1, ISG15, HERC6, SLC1A2, FAM20A, RETN | 0,876 | 0,866 | 0,886 | 0,923 | 0,868 | 0,979 | 0,942 | 0,863 | 1 |
| RSAD2, IFI27, ISG15, HERC6, SLC1A2, IL1R2, OLAH, RETN | 0,876 | 0,867 | 0,885 | 0,926 | 0,876 | 0,977 | 0,933 | 0,867 | 0,998 |
| IFI27, IFIT1, IFI44L, SIGLEC1, SLC1A2, IL1R2, OLAH, RETN | 0,876 | 0,867 | 0,885 | 0,926 | 0,875 | 0,977 | 0,950 | 0,894 | 1 |

| | | | | | | | | |
|---|---|---|---|---|---|---|---|---|
| RSAD2, IFI27, OAS1, IFIT1, ISG15, HERC6, RETN, MMP8 | 0,876 | 0,867 | 0,886 | 0,918 | 0,858 | 0,978 | 0,932 | 0,861 | 1 |
| RSAD2, IFI27, OAS1, IFIT1, SLC1A2, IL1R2, FAM20A, MMP8 | 0,876 | 0,867 | 0,885 | 0,926 | 0,877 | 0,975 | 0,927 | 0,843 | 1 |
| RSAD2, IFI27, OAS1, IFIT1, SIGLEC1, SLC1A2, OLAH, RETN | 0,876 | 0,867 | 0,885 | 0,928 | 0,876 | 0,980 | 0,950 | 0,888 | 1 |
| IFI27, IFIT1, ISG15, SLC1A2, IL1R2, FAM20A, RETN, MMP8 | 0,876 | 0,867 | 0,886 | 0,933 | 0,884 | 0,982 | 0,930 | 0,848 | 1 |
| OAS1, SIGLEC1, HERC6, IL1R2, OLAH, FAM20A, RETN, MMP8 | 0,876 | 0,866 | 0,886 | 0,920 | 0,863 | 0,977 | 0,964 | 0,912 | 1 |
| RSAD2, IFI27, IFI44L, SIGLEC1, SLC1A2, IL1R2, OLAH, RETN | 0,876 | 0,867 | 0,885 | 0,926 | 0,875 | 0,977 | 0,949 | 0,892 | 1 |
| IFI27, OAS1, IFIT1, IFI44L, SIGLEC1, ISG15, IL1R2, RETN | 0,876 | 0,868 | 0,885 | 0,922 | 0,870 | 0,975 | 0,948 | 0,896 | 1,000 |
| OAS1, IFIT1, SIGLEC1, ISG15, SLC1A2, OLAH, FAM20A, MMP8 | 0,876 | 0,866 | 0,886 | 0,922 | 0,868 | 0,975 | 0,952 | 0,891 | 1 |
| RSAD2, OAS1, SIGLEC1, IL1R2, OLAH, FAM20A, RETN, MMP8 | 0,876 | 0,866 | 0,886 | 0,920 | 0,863 | 0,977 | 0,962 | 0,906 | 1 |
| RSAD2, IFI27, OAS1, IFIT1, ISG15, HERC6, SLC1A2, OLAH | 0,876 | 0,867 | 0,886 | 0,927 | 0,876 | 0,978 | 0,933 | 0,866 | 0,999 |
| RSAD2, IFI27, IFI44L, SIGLEC1, ISG15, HERC6, FAM20A, MMP8 | 0,876 | 0,866 | 0,886 | 0,918 | 0,858 | 0,977 | 0,941 | 0,869 | 1 |
| IFI27, IFIT1, IFI44L, HERC6, SLC1A2, IL1R2, FAM20A, RETN | 0,876 | 0,867 | 0,886 | 0,930 | 0,881 | 0,979 | 0,927 | 0,844 | 1 |
| IFI27, OAS1, IFIT1, SIGLEC1, ISG15, IL1R2, RETN, MMP8 | 0,876 | 0,867 | 0,885 | 0,923 | 0,870 | 0,976 | 0,963 | 0,918 | 1 |
| IFI27, OAS1, IFIT1, IFI44L, HERC6, SLC1A2, OLAH, MMP8 | 0,876 | 0,866 | 0,886 | 0,929 | 0,879 | 0,978 | 0,934 | 0,868 | 0,999 |
| RSAD2, IFI27, OAS1, IFI44L, SIGLEC1, SLC1A2, OLAH, RETN | 0,876 | 0,867 | 0,885 | 0,926 | 0,873 | 0,979 | 0,951 | 0,889 | 1 |
| RSAD2, IFI27, IFI44L, ISG15, HERC6, SLC1A2, OLAH, RETN | 0,876 | 0,867 | 0,885 | 0,927 | 0,876 | 0,977 | 0,935 | 0,870 | 0,999 |
| IFI27, OAS1, IFIT1, IFI44L, ISG15, IL1R2, OLAH, MMP8 | 0,876 | 0,867 | 0,885 | 0,921 | 0,869 | 0,973 | 0,940 | 0,886 | 0,995 |
| RSAD2, SIGLEC1, ISG15, SLC1A2, IL1R2, OLAH, FAM20A, MMP8 | 0,876 | 0,866 | 0,886 | 0,924 | 0,877 | 0,972 | 0,961 | 0,901 | 1 |
| OAS1, IFI44L, SIGLEC1, ISG15, IL1R2, OLAH, FAM20A, MMP8 | 0,876 | 0,866 | 0,886 | 0,916 | 0,858 | 0,974 | 0,963 | 0,908 | 1 |
| RSAD2, IFI27, OAS1, SIGLEC1, HERC6, SLC1A2, RETN, MMP8 | 0,876 | 0,867 | 0,885 | 0,923 | 0,869 | 0,978 | 0,949 | 0,887 | 1 |
| RSAD2, IFI27, OAS1, IFIT1, ISG15, IL1R2, OLAH, MMP8 | 0,876 | 0,867 | 0,885 | 0,922 | 0,871 | 0,973 | 0,946 | 0,894 | 0,998 |
| IFI27, IFIT1, ISG15, SLC1A2, IL1R2, OLAH, RETN, MMP8 | 0,876 | 0,866 | 0,886 | 0,926 | 0,873 | 0,978 | 0,940 | 0,879 | 1 |
| IFI27, OAS1, IFIT1, IFI44L, SIGLEC1, ISG15, SLC1A2, FAM20A | 0,876 | 0,867 | 0,885 | 0,922 | 0,866 | 0,979 | 0,940 | 0,867 | 1 |
| IFI27, OAS1, IFI44L, SIGLEC1, SLC1A2, IL1R2, OLAH, MMP8 | 0,876 | 0,867 | 0,885 | 0,927 | 0,878 | 0,976 | 0,951 | 0,900 | 1 |
| RSAD2, IFI27, OAS1, IFIT1, HERC6, SLC1A2, IL1R2, FAM20A | 0,876 | 0,866 | 0,885 | 0,931 | 0,886 | 0,977 | 0,925 | 0,839 | 1 |
| RSAD2, IFI27, IFIT1, IFI44L, SIGLEC1, IL1R2, RETN, MMP8 | 0,876 | 0,867 | 0,885 | 0,925 | 0,872 | 0,977 | 0,961 | 0,920 | 1 |
| OAS1, IFIT1, IFI44L, SIGLEC1, ISG15, HERC6, OLAH, FAM20A | 0,876 | 0,866 | 0,886 | 0,916 | 0,857 | 0,975 | 0,946 | 0,888 | 1 |
| IFI27, OAS1, IFI44L, SIGLEC1, SLC1A2, IL1R2, OLAH, RETN | 0,876 | 0,867 | 0,885 | 0,926 | 0,875 | 0,978 | 0,952 | 0,894 | 1 |

| | | | | | | | | |
|---|---|---|---|---|---|---|---|---|
| IFIT1, IFI44L, SIGLEC1, HERC6, SLC1A2, IL1R2, OLAH, FAM20A | 0,876 | 0,866 | 0,886 | 0,926 | 0,876 | 0,975 | 0,938 | 0,868 | 1 |
| RSAD2, IFI27, OAS1, IFIT1, ISG15, SLC1A2, OLAH, MMP8 | 0,876 | 0,867 | 0,885 | 0,926 | 0,873 | 0,978 | 0,933 | 0,866 | 0,999 |
| IFI27, IFI44L, ISG15, HERC6, SLC1A2, IL1R2, FAM20A, RETN | 0,876 | 0,866 | 0,886 | 0,931 | 0,882 | 0,979 | 0,926 | 0,842 | 1 |
| RSAD2, IFI27, IFIT1, IFI44L, SIGLEC1, HERC6, SLC1A2, OLAH | 0,876 | 0,867 | 0,885 | 0,926 | 0,876 | 0,977 | 0,941 | 0,885 | 0,997 |
| RSAD2, IFI27, IFIT1, SIGLEC1, ISG15, SLC1A2, OLAH, RETN | 0,876 | 0,867 | 0,885 | 0,929 | 0,878 | 0,980 | 0,939 | 0,878 | 1 |
| RSAD2, IFI27, OAS1, IFIT1, SIGLEC1, SLC1A2, FAM20A, RETN | 0,876 | 0,866 | 0,885 | 0,925 | 0,869 | 0,981 | 0,941 | 0,869 | 1 |
| RSAD2, IFI27, IFIT1, IFI44L, HERC6, SLC1A2, OLAH, RETN | 0,876 | 0,867 | 0,885 | 0,926 | 0,875 | 0,977 | 0,935 | 0,870 | 0,999 |
| RSAD2, IFI44L, SIGLEC1, ISG15, IL1R2, OLAH, FAM20A, MMP8 | 0,876 | 0,866 | 0,885 | 0,917 | 0,860 | 0,974 | 0,962 | 0,906 | 1 |
| OAS1, SIGLEC1, ISG15, HERC6, SLC1A2, IL1R2, FAM20A, MMP8 | 0,876 | 0,866 | 0,886 | 0,924 | 0,876 | 0,972 | 0,958 | 0,892 | 1 |
| RSAD2, IFI27, IFIT1, IFI44L, SIGLEC1, ISG15, OLAH, MMP8 | 0,876 | 0,867 | 0,885 | 0,920 | 0,863 | 0,976 | 0,939 | 0,882 | 0,997 |
| RSAD2, IFI27, IFIT1, IFI44L, HERC6, IL1R2, RETN, MMP8 | 0,876 | 0,867 | 0,885 | 0,921 | 0,867 | 0,975 | 0,941 | 0,883 | 1,000 |
| RSAD2, IFIT1, IFI44L, SIGLEC1, SLC1A2, IL1R2, FAM20A, MMP8 | 0,876 | 0,867 | 0,885 | 0,927 | 0,880 | 0,974 | 0,962 | 0,902 | 1 |
| RSAD2, IFI27, IFI44L, SLC1A2, IL1R2, FAM20A, RETN, MMP8 | 0,876 | 0,866 | 0,885 | 0,930 | 0,880 | 0,980 | 0,924 | 0,836 | 1 |
| RSAD2, IFI27, IFI44L, HERC6, SLC1A2, IL1R2, OLAH, RETN | 0,876 | 0,866 | 0,885 | 0,925 | 0,874 | 0,976 | 0,935 | 0,870 | 0,999 |
| RSAD2, IFI27, OAS1, IFI44L, SIGLEC1, IL1R2, RETN, MMP8 | 0,876 | 0,867 | 0,885 | 0,920 | 0,865 | 0,975 | 0,962 | 0,916 | 1 |
| RSAD2, OAS1, IFIT1, SIGLEC1, HERC6, IL1R2, FAM20A, RETN | 0,876 | 0,867 | 0,885 | 0,919 | 0,860 | 0,977 | 0,952 | 0,895 | 1 |
| RSAD2, IFI27, IFI44L, HERC6, SLC1A2, IL1R2, FAM20A, RETN | 0,876 | 0,866 | 0,886 | 0,930 | 0,882 | 0,979 | 0,926 | 0,842 | 1 |
| RSAD2, IFI27, OAS1, IFIT1, SIGLEC1, ISG15, OLAH, RETN | 0,876 | 0,867 | 0,885 | 0,918 | 0,860 | 0,976 | 0,948 | 0,889 | 1 |
| IFIT1, IFI44L, SIGLEC1, SLC1A2, IL1R2, FAM20A, RETN, MMP8 | 0,876 | 0,866 | 0,885 | 0,929 | 0,882 | 0,975 | 0,961 | 0,900 | 1 |
| RSAD2, IFI27, IFIT1, ISG15, SLC1A2, IL1R2, OLAH, MMP8 | 0,876 | 0,867 | 0,885 | 0,927 | 0,878 | 0,976 | 0,932 | 0,868 | 0,995 |
| RSAD2, IFI27, OAS1, IFI44L, HERC6, SLC1A2, OLAH, MMP8 | 0,876 | 0,866 | 0,885 | 0,928 | 0,878 | 0,978 | 0,932 | 0,864 | 0,999 |
| RSAD2, IFI27, OAS1, IFI44L, SIGLEC1, SLC1A2, IL1R2, OLAH | 0,876 | 0,867 | 0,885 | 0,927 | 0,876 | 0,977 | 0,948 | 0,890 | 1 |
| RSAD2, IFI27, IFIT1, IFI44L, SIGLEC1, SLC1A2, OLAH, RETN | 0,876 | 0,867 | 0,884 | 0,926 | 0,874 | 0,978 | 0,950 | 0,894 | 1 |
| RSAD2, IFI27, IFIT1, SIGLEC1, ISG15, IL1R2, OLAH, RETN | 0,876 | 0,867 | 0,884 | 0,919 | 0,863 | 0,975 | 0,947 | 0,891 | 1 |
| OAS1, IFI44L, SIGLEC1, HERC6, SLC1A2, IL1R2, FAM20A, MMP8 | 0,876 | 0,866 | 0,886 | 0,923 | 0,875 | 0,971 | 0,959 | 0,893 | 1 |
| RSAD2, IFI27, IFIT1, SLC1A2, IL1R2, OLAH, RETN, MMP8 | 0,876 | 0,866 | 0,885 | 0,925 | 0,873 | 0,977 | 0,943 | 0,883 | 1 |
| RSAD2, IFI27, OAS1, SIGLEC1, ISG15, HERC6, SLC1A2, RETN | 0,876 | 0,867 | 0,885 | 0,921 | 0,866 | 0,977 | 0,947 | 0,884 | 1 |
| RSAD2, IFI27, IFIT1, IFI44L, SIGLEC1, HERC6, RETN, MMP8 | 0,876 | 0,867 | 0,885 | 0,915 | 0,856 | 0,975 | 0,939 | 0,883 | 0,995 |

535

| Gene set | | | | | | | | |
|---|---|---|---|---|---|---|---|---|
| RSAD2, IFI27, IFIT1, ISG15, IL1R2, OLAH, RETN, MMP8 | 0,876 | 0,866 | 0,885 | 0,921 | 0,865 | 0,977 | 0,950 | 0,897 | 1 |
| RSAD2, IFI27, OAS1, IFI44L, SIGLEC1, ISG15, IL1R2, RETN | 0,876 | 0,867 | 0,884 | 0,918 | 0,862 | 0,974 | 0,952 | 0,900 | 1 |
| RSAD2, OAS1, IFIT1, SIGLEC1, HERC6, FAM20A, RETN, MMP8 | 0,876 | 0,866 | 0,885 | 0,918 | 0,859 | 0,978 | 0,941 | 0,873 | 1 |
| RSAD2, OAS1, IFIT1, SIGLEC1, SLC1A2, IL1R2, FAM20A, MMP8 | 0,876 | 0,866 | 0,885 | 0,928 | 0,882 | 0,974 | 0,962 | 0,901 | 1 |
| RSAD2, IFI27, OAS1, IFIT1, SIGLEC1, ISG15, HERC6, IL1R2 | 0,876 | 0,867 | 0,884 | 0,922 | 0,875 | 0,969 | 0,952 | 0,907 | 0,997 |
| RSAD2, IFI27, OAS1, IFI44L, SIGLEC1, SLC1A2, OLAH, MMP8 | 0,876 | 0,867 | 0,885 | 0,926 | 0,874 | 0,978 | 0,954 | 0,902 | 1 |
| RSAD2, IFI27, IFIT1, IFI44L, SIGLEC1, ISG15, HERC6, IL1R2 | 0,876 | 0,867 | 0,884 | 0,919 | 0,870 | 0,969 | 0,940 | 0,888 | 0,992 |
| RSAD2, IFI44L, SIGLEC1, SLC1A2, IL1R2, OLAH, FAM20A, MMP8 | 0,876 | 0,866 | 0,885 | 0,924 | 0,875 | 0,973 | 0,959 | 0,898 | 1 |
| IFI27, OAS1, IFI44L, SIGLEC1, ISG15, HERC6, FAM20A, MMP8 | 0,876 | 0,865 | 0,886 | 0,914 | 0,854 | 0,973 | 0,945 | 0,871 | 1 |
| RSAD2, IFIT1, IFI44L, SIGLEC1, HERC6, SLC1A2, OLAH, FAM20A | 0,876 | 0,866 | 0,885 | 0,925 | 0,875 | 0,975 | 0,927 | 0,853 | 1 |
| RSAD2, IFI27, IFI44L, ISG15, IL1R2, OLAH, RETN, MMP8 | 0,876 | 0,866 | 0,885 | 0,922 | 0,867 | 0,978 | 0,952 | 0,898 | 1 |
| RSAD2, OAS1, IFIT1, SIGLEC1, ISG15, HERC6, FAM20A, RETN | 0,876 | 0,866 | 0,885 | 0,917 | 0,858 | 0,976 | 0,943 | 0,879 | 1 |
| RSAD2, IFI27, IFIT1, ISG15, HERC6, SLC1A2, IL1R2, OLAH | 0,875 | 0,866 | 0,885 | 0,926 | 0,877 | 0,976 | 0,926 | 0,858 | 0,994 |
| RSAD2, IFIT1, SIGLEC1, ISG15, SLC1A2, IL1R2, FAM20A, MMP8 | 0,875 | 0,866 | 0,885 | 0,927 | 0,880 | 0,974 | 0,960 | 0,894 | 1 |
| IFI27, IFI44L, ISG15, HERC6, SLC1A2, IL1R2, OLAH, RETN | 0,875 | 0,866 | 0,885 | 0,926 | 0,875 | 0,977 | 0,936 | 0,871 | 1 |
| RSAD2, IFI27, IFI44L, ISG15, HERC6, IL1R2, RETN, MMP8 | 0,875 | 0,867 | 0,884 | 0,923 | 0,870 | 0,976 | 0,937 | 0,876 | 0,998 |
| IFI27, OAS1, IFI44L, SLC1A2, IL1R2, FAM20A, RETN, MMP8 | 0,875 | 0,866 | 0,885 | 0,930 | 0,881 | 0,979 | 0,922 | 0,833 | 1 |
| RSAD2, IFI27, OAS1, IFI44L, ISG15, IL1R2, OLAH, MMP8 | 0,875 | 0,867 | 0,884 | 0,923 | 0,871 | 0,974 | 0,939 | 0,883 | 0,996 |
| RSAD2, IFI27, OAS1, SIGLEC1, ISG15, HERC6, IL1R2, MMP8 | 0,875 | 0,867 | 0,884 | 0,922 | 0,875 | 0,968 | 0,951 | 0,905 | 0,997 |
| IFI27, OAS1, IFI44L, SIGLEC1, ISG15, SLC1A2, FAM20A, RETN | 0,875 | 0,866 | 0,885 | 0,923 | 0,867 | 0,980 | 0,940 | 0,864 | 1 |
| RSAD2, IFI27, IFI44L, SIGLEC1, ISG15, HERC6, IL1R2, MMP8 | 0,875 | 0,868 | 0,883 | 0,920 | 0,870 | 0,970 | 0,940 | 0,888 | 0,992 |
| OAS1, SIGLEC1, ISG15, IL1R2, OLAH, FAM20A, RETN, MMP8 | 0,875 | 0,866 | 0,885 | 0,914 | 0,854 | 0,974 | 0,966 | 0,914 | 1 |
| OAS1, IFIT1, IFI44L, SIGLEC1, ISG15, IL1R2, OLAH, FAM20A | 0,875 | 0,866 | 0,885 | 0,913 | 0,850 | 0,976 | 0,959 | 0,907 | 1 |
| RSAD2, IFI27, IFIT1, HERC6, SLC1A2, IL1R2, FAM20A, RETN | 0,875 | 0,866 | 0,885 | 0,933 | 0,885 | 0,981 | 0,927 | 0,845 | 1 |
| RSAD2, IFI27, IFIT1, ISG15, HERC6, SLC1A2, OLAH, RETN | 0,875 | 0,866 | 0,885 | 0,927 | 0,876 | 0,978 | 0,933 | 0,866 | 0,999 |
| IFI27, IFIT1, ISG15, HERC6, SLC1A2, IL1R2, OLAH, RETN | 0,875 | 0,866 | 0,885 | 0,926 | 0,875 | 0,978 | 0,934 | 0,868 | 0,999 |
| IFI27, OAS1, IFIT1, IFI44L, SIGLEC1, SLC1A2, IL1R2, OLAH | 0,875 | 0,866 | 0,884 | 0,927 | 0,877 | 0,977 | 0,949 | 0,895 | 1 |
| RSAD2, OAS1, IFIT1, IFI44L, SIGLEC1, OLAH, FAM20A, MMP8 | 0,875 | 0,865 | 0,885 | 0,911 | 0,846 | 0,975 | 0,954 | 0,903 | 1 |

| | | | | | | | | | |
|---|---|---|---|---|---|---|---|---|---|
| IFI27, IFIT1, ISG15, HERC6, SLC1A2, IL1R2, FAM20A, MMP8 | 0,875 | 0,866 | 0,885 | 0,929 | 0,882 | 0,976 | 0,928 | 0,842 | 1 |
| RSAD2, OAS1, SIGLEC1, ISG15, IL1R2, FAM20A, RETN, MMP8 | 0,875 | 0,866 | 0,885 | 0,919 | 0,861 | 0,976 | 0,961 | 0,902 | 1 |
| RSAD2, IFI44L, SIGLEC1, HERC6, SLC1A2, IL1R2, FAM20A, MMP8 | 0,875 | 0,866 | 0,885 | 0,925 | 0,877 | 0,972 | 0,959 | 0,895 | 1 |
| RSAD2, OAS1, IFIT1, SIGLEC1, SLC1A2, OLAH, FAM20A, MMP8 | 0,875 | 0,866 | 0,885 | 0,923 | 0,869 | 0,976 | 0,952 | 0,892 | 1 |
| IFI27, IFIT1, IFI44L, ISG15, HERC6, SLC1A2, IL1R2, FAM20A | 0,875 | 0,866 | 0,885 | 0,929 | 0,882 | 0,976 | 0,927 | 0,842 | 1 |
| RSAD2, IFI27, IFIT1, IFI44L, ISG15, IL1R2, OLAH, MMP8 | 0,875 | 0,867 | 0,884 | 0,919 | 0,866 | 0,972 | 0,942 | 0,889 | 0,995 |
| RSAD2, IFIT1, SIGLEC1, ISG15, HERC6, FAM20A, RETN, MMP8 | 0,875 | 0,866 | 0,884 | 0,918 | 0,860 | 0,977 | 0,945 | 0,877 | 1 |
| IFI27, OAS1, IFI44L, SIGLEC1, SLC1A2, FAM20A, RETN, MMP8 | 0,875 | 0,865 | 0,886 | 0,923 | 0,866 | 0,980 | 0,942 | 0,868 | 1 |
| RSAD2, IFIT1, IFI44L, SIGLEC1, ISG15, HERC6, OLAH, FAM20 | 0,875 | 0,865 | 0,885 | 0,916 | 0,857 | 0,975 | 0,947 | 0,890 | 1 |
| RSAD2, IFI27, IFIT1, IFI44L, SIGLEC1, SLC1A2, OLAH, MMP8 | 0,875 | 0,867 | 0,884 | 0,926 | 0,875 | 0,978 | 0,948 | 0,894 | 1 |
| OAS1, IFIT1, IFI44L, SIGLEC1, HERC6, SLC1A2, IL1R2, FAM20 | 0,875 | 0,866 | 0,885 | 0,927 | 0,881 | 0,974 | 0,940 | 0,869 | 1 |
| RSAD2, IFI27, IFIT1, SIGLEC1, ISG15, HERC6, RETN, MMP8 | 0,875 | 0,866 | 0,884 | 0,918 | 0,860 | 0,975 | 0,949 | 0,900 | 0,998 |
| RSAD2, IFI27, OAS1, IFIT1, IFI44L, SIGLEC1, SLC1A2, FAM20 | 0,875 | 0,866 | 0,885 | 0,922 | 0,866 | 0,979 | 0,941 | 0,868 | 1 |
| OAS1, IFIT1, IFI44L, SIGLEC1, ISG15, OLAH, FAM20A, MMP8 | 0,875 | 0,865 | 0,886 | 0,912 | 0,847 | 0,977 | 0,954 | 0,902 | 1 |
| RSAD2, OAS1, IFIT1, SIGLEC1, ISG15, HERC6, OLAH, FAM20 | 0,875 | 0,865 | 0,885 | 0,914 | 0,853 | 0,974 | 0,950 | 0,895 | 1 |
| RSAD2, IFI27, IFIT1, IFI44L, ISG15, HERC6, IL1R2, RETN | 0,875 | 0,867 | 0,884 | 0,921 | 0,869 | 0,974 | 0,936 | 0,876 | 0,995 |
| IFI27, OAS1, IFIT1, IFI44L, SIGLEC1, SLC1A2, FAM20A, MMP8 | 0,875 | 0,866 | 0,885 | 0,921 | 0,864 | 0,979 | 0,939 | 0,865 | 1 |
| RSAD2, IFIT1, SIGLEC1, HERC6, IL1R2, OLAH, FAM20A, RETN | 0,875 | 0,866 | 0,885 | 0,918 | 0,860 | 0,977 | 0,952 | 0,897 | 1 |
| IFI27, OAS1, IFIT1, ISG15, SLC1A2, IL1R2, OLAH, MMP8 | 0,875 | 0,866 | 0,884 | 0,927 | 0,878 | 0,976 | 0,933 | 0,868 | 0,997 |
| RSAD2, IFI27, IFI44L, SIGLEC1, SLC1A2, FAM20A, RETN, MMP8 | 0,875 | 0,865 | 0,885 | 0,926 | 0,871 | 0,982 | 0,949 | 0,877 | 1 |
| RSAD2, IFIT1, SIGLEC1, ISG15, HERC6, IL1R2, FAM20A, RETN | 0,875 | 0,866 | 0,884 | 0,918 | 0,860 | 0,976 | 0,954 | 0,898 | 1 |
| IFIT1, SIGLEC1, SLC1A2, IL1R2, OLAH, FAM20A, RETN, MMP8 | 0,875 | 0,866 | 0,885 | 0,927 | 0,878 | 0,975 | 0,961 | 0,903 | 1 |
| IFI27, OAS1, IFIT1, IFI44L, HERC6, SLC1A2, FAM20A, RETN | 0,875 | 0,865 | 0,885 | 0,922 | 0,866 | 0,978 | 0,934 | 0,852 | 1 |
| RSAD2, IFI44L, SIGLEC1, HERC6, IL1R2, FAM20A, RETN, MMP8 | 0,875 | 0,866 | 0,885 | 0,921 | 0,865 | 0,977 | 0,959 | 0,897 | 1 |
| RSAD2, OAS1, IFIT1, IFI44L, SIGLEC1, IL1R2, OLAH, FAM20 | 0,875 | 0,866 | 0,884 | 0,913 | 0,849 | 0,976 | 0,962 | 0,913 | 1 |
| IFI27, OAS1, IFI44L, ISG15, HERC6, SLC1A2, OLAH, MMP8 | 0,875 | 0,865 | 0,885 | 0,929 | 0,880 | 0,978 | 0,924 | 0,854 | 0,994 |
| RSAD2, IFI27, IFIT1, IFI44L, SIGLEC1, ISG15, SLC1A2, OLAH | 0,875 | 0,867 | 0,884 | 0,931 | 0,881 | 0,981 | 0,942 | 0,884 | 1 |
| OAS1, IFIT1, SIGLEC1, ISG15, SLC1A2, IL1R2, FAM20A, MMP8 | 0,875 | 0,866 | 0,885 | 0,927 | 0,881 | 0,973 | 0,959 | 0,893 | 1 |

| | | | | | | | | |
|---|---|---|---|---|---|---|---|---|
| RSAD2, IFI27, SIGLEC1, HERC6, SLC1A2, IL1R2, RETN, MMP8 | 0,875 | 0,866 | 0,884 | 0,927 | 0,880 | 0,974 | 0,963 | 0,923 | 1 |
| IFIT1, IFI44L, SIGLEC1, ISG15, HERC6, SLC1A2, IL1R2, FAM20A | 0,875 | 0,866 | 0,884 | 0,925 | 0,877 | 0,974 | 0,941 | 0,869 | 1 |
| RSAD2, IFI27, OAS1, IFIT1, SIGLEC1, ISG15, FAM20A, MMP8 | 0,875 | 0,865 | 0,885 | 0,916 | 0,851 | 0,980 | 0,940 | 0,867 | 1 |
| IFI27, IFIT1, IFI44L, ISG15, HERC6, SLC1A2, OLAH, RETN | 0,875 | 0,866 | 0,884 | 0,926 | 0,874 | 0,977 | 0,935 | 0,870 | 1,000 |
| OAS1, SIGLEC1, ISG15, SLC1A2, IL1R2, OLAH, FAM20A, MMP8 | 0,875 | 0,866 | 0,885 | 0,923 | 0,875 | 0,972 | 0,961 | 0,901 | 1 |
| IFI44L, SIGLEC1, ISG15, SLC1A2, IL1R2, FAM20A, RETN, MMP8 | 0,875 | 0,866 | 0,884 | 0,925 | 0,876 | 0,973 | 0,957 | 0,889 | 1 |
| RSAD2, IFI27, OAS1, SLC1A2, IL1R2, OLAH, RETN, MMP8 | 0,875 | 0,866 | 0,885 | 0,929 | 0,877 | 0,980 | 0,939 | 0,873 | 1 |
| IFI27, OAS1, IFIT1, ISG15, SLC1A2, IL1R2, FAM20A, MMP8 | 0,875 | 0,865 | 0,885 | 0,927 | 0,879 | 0,975 | 0,926 | 0,841 | 1 |
| IFI27, OAS1, IFIT1, ISG15, SLC1A2, OLAH, RETN, MMP8 | 0,875 | 0,865 | 0,885 | 0,926 | 0,872 | 0,979 | 0,941 | 0,877 | 1 |
| IFI27, IFI44L, SIGLEC1, HERC6, SLC1A2, IL1R2, RETN, MMP8 | 0,875 | 0,866 | 0,884 | 0,927 | 0,879 | 0,975 | 0,955 | 0,909 | 1 |
| IFI27, OAS1, IFIT1, IFI44L, ISG15, HERC6, SLC1A2, OLAH | 0,875 | 0,865 | 0,885 | 0,928 | 0,878 | 0,978 | 0,928 | 0,862 | 0,995 |
| IFI27, OAS1, IFI44L, HERC6, SLC1A2, OLAH, RETN | 0,875 | 0,865 | 0,885 | 0,928 | 0,878 | 0,979 | 0,932 | 0,864 | 0,999 |
| IFI27, OAS1, IFI44L, HERC6, SLC1A2, IL1R2, OLAH, MMP8 | 0,875 | 0,865 | 0,885 | 0,928 | 0,879 | 0,977 | 0,928 | 0,860 | 0,997 |
| IFI27, OAS1, IFI44L, SIGLEC1, HERC6, SLC1A2, RETN, MMP8 | 0,875 | 0,866 | 0,884 | 0,925 | 0,872 | 0,978 | 0,942 | 0,880 | 1 |
| OAS1, IFIT1, SIGLEC1, HERC6, SLC1A2, IL1R2, OLAH, FAM20A | 0,875 | 0,865 | 0,885 | 0,924 | 0,875 | 0,973 | 0,943 | 0,875 | 1 |
| IFI27, IFIT1, SIGLEC1, HERC6, SLC1A2, IL1R2, RETN, MMP8 | 0,875 | 0,866 | 0,884 | 0,927 | 0,881 | 0,974 | 0,963 | 0,923 | 1 |
| IFI27, IFIT1, ISG15, HERC6, SLC1A2, IL1R2, FAM20A, RETN | 0,875 | 0,865 | 0,885 | 0,933 | 0,886 | 0,981 | 0,927 | 0,845 | 1 |
| RSAD2, IFI27, ISG15, SLC1A2, IL1R2, FAM20A, RETN, MMP8 | 0,875 | 0,866 | 0,884 | 0,930 | 0,881 | 0,978 | 0,927 | 0,841 | 1 |
| RSAD2, IFI27, IFI44L, HERC6, SLC1A2, IL1R2, OLAH, MMP8 | 0,875 | 0,866 | 0,884 | 0,926 | 0,877 | 0,976 | 0,929 | 0,864 | 0,995 |
| RSAD2, IFI27, OAS1, IFI44L, ISG15, IL1R2, OLAH, RETN | 0,875 | 0,866 | 0,884 | 0,917 | 0,860 | 0,974 | 0,938 | 0,876 | 1 |
| RSAD2, IFIT1, IFI44L, SIGLEC1, IL1R2, FAM20A, RETN, MMP8 | 0,875 | 0,866 | 0,884 | 0,922 | 0,865 | 0,978 | 0,968 | 0,918 | 1 |
| IFI27, IFIT1, IFI44L, ISG15, SLC1A2, IL1R2, OLAH, MMP8 | 0,875 | 0,866 | 0,884 | 0,929 | 0,880 | 0,977 | 0,934 | 0,872 | 0,995 |
| RSAD2, IFI27, OAS1, IFI44L, ISG15, HERC6, SLC1A2, OLAH | 0,875 | 0,865 | 0,885 | 0,929 | 0,879 | 0,979 | 0,932 | 0,864 | 0,999 |
| IFI27, OAS1, IFIT1, SIGLEC1, SLC1A2, IL1R2, RETN, MMP8 | 0,875 | 0,866 | 0,884 | 0,926 | 0,878 | 0,974 | 0,962 | 0,913 | 1 |
| IFIT1, SIGLEC1, ISG15, HERC6, SLC1A2, IL1R2, OLAH, FAM20A | 0,875 | 0,865 | 0,885 | 0,924 | 0,875 | 0,974 | 0,945 | 0,873 | 1 |
| RSAD2, IFI27, IFIT1, ISG15, HERC6, SLC1A2, IL1R2, FAM20A | 0,875 | 0,865 | 0,884 | 0,930 | 0,883 | 0,977 | 0,925 | 0,840 | 1 |
| RSAD2, IFI27, IFIT1, ISG15, HERC6, SLC1A2, OLAH, MMP8 | 0,875 | 0,865 | 0,885 | 0,927 | 0,877 | 0,978 | 0,928 | 0,862 | 0,995 |
| IFIT1, IFI44L, SIGLEC1, HERC6, SLC1A2, OLAH, FAM20A, RETN | 0,875 | 0,865 | 0,885 | 0,925 | 0,874 | 0,976 | 0,933 | 0,861 | 1 |

| | | | | | | | | | |
|---|---|---|---|---|---|---|---|---|---|
| RSAD2, IFI27, OAS1, ISG15, SLC1A2, IL1R2, OLAH, MMP8 | 0,875 | 0,866 | 0,884 | 0,929 | 0,880 | 0,978 | 0,930 | 0,862 | 0,998 |
| IFI27, OAS1, IFIT1, ISG15, SLC1A2, IL1R2, OLAH, RETN | 0,875 | 0,866 | 0,884 | 0,926 | 0,875 | 0,978 | 0,933 | 0,864 | 1 |
| RSAD2, OAS1, IFIT1, SIGLEC1, ISG15, IL1R2, OLAH, FAM20A | 0,875 | 0,866 | 0,884 | 0,912 | 0,849 | 0,975 | 0,963 | 0,914 | 1 |
| IFI27, OAS1, IFIT1, ISG15, HERC6, SLC1A2, FAM20A, RETN | 0,875 | 0,865 | 0,885 | 0,922 | 0,866 | 0,978 | 0,930 | 0,849 | 1 |
| IFI27, IFI44L, ISG15, HERC6, SLC1A2, IL1R2, OLAH, MMP8 | 0,875 | 0,866 | 0,884 | 0,928 | 0,879 | 0,977 | 0,927 | 0,860 | 0,994 |
| IFI27, IFIT1, IFI44L, ISG15, HERC6, IL1R2, RETN, MMP8 | 0,875 | 0,866 | 0,884 | 0,921 | 0,867 | 0,975 | 0,942 | 0,885 | 0,999 |
| RSAD2, IFI27, ISG15, SLC1A2, IL1R2, OLAH, RETN, MMP8 | 0,875 | 0,865 | 0,884 | 0,927 | 0,875 | 0,978 | 0,939 | 0,876 | 1 |
| IFI27, OAS1, IFIT1, IFI44L, SLC1A2, IL1R2, FAM20A, RETN | 0,875 | 0,865 | 0,884 | 0,925 | 0,874 | 0,976 | 0,924 | 0,839 | 1 |
| RSAD2, IFI27, IFIT1, IFI44L, ISG15, OLAH, RETN, MMP8 | 0,875 | 0,865 | 0,884 | 0,918 | 0,859 | 0,978 | 0,949 | 0,890 | 1 |
| IFI27, OAS1, IFI44L, ISG15, SLC1A2, IL1R2, FAM20A, MMP8 | 0,875 | 0,865 | 0,885 | 0,930 | 0,884 | 0,977 | 0,926 | 0,839 | 1 |
| RSAD2, IFIT1, SIGLEC1, HERC6, SLC1A2, IL1R2, OLAH, FAM20A | 0,875 | 0,865 | 0,885 | 0,923 | 0,872 | 0,974 | 0,946 | 0,876 | 1 |
| RSAD2, IFI27, IFIT1, IFI44L, SIGLEC1, SLC1A2, IL1R2, OLAH | 0,875 | 0,866 | 0,883 | 0,927 | 0,877 | 0,978 | 0,946 | 0,891 | 1 |
| RSAD2, IFI27, IFI44L, SIGLEC1, ISG15, IL1R2, RETN, MMP8 | 0,875 | 0,866 | 0,884 | 0,922 | 0,868 | 0,976 | 0,958 | 0,914 | 1 |
| RSAD2, IFI27, OAS1, IFIT1, ISG15, IL1R2, OLAH, RETN | 0,875 | 0,866 | 0,884 | 0,919 | 0,863 | 0,975 | 0,942 | 0,883 | 1 |
| RSAD2, OAS1, IFI44L, SIGLEC1, ISG15, IL1R2, FAM20A, MMP8 | 0,875 | 0,865 | 0,884 | 0,917 | 0,859 | 0,975 | 0,961 | 0,902 | 1 |
| RSAD2, IFI27, OAS1, IFIT1, SLC1A2, IL1R2, OLAH, MMP8 | 0,875 | 0,866 | 0,884 | 0,926 | 0,876 | 0,976 | 0,938 | 0,874 | 1 |
| RSAD2, IFI27, IFIT1, IFI44L, HERC6, SLC1A2, OLAH, MMP8 | 0,875 | 0,865 | 0,884 | 0,927 | 0,877 | 0,978 | 0,930 | 0,865 | 0,995 |
| IFI27, OAS1, ISG15, SLC1A2, IL1R2, OLAH, RETN, MMP8 | 0,875 | 0,865 | 0,884 | 0,926 | 0,875 | 0,978 | 0,934 | 0,868 | 0,999 |
| IFI27, IFIT1, IFI44L, ISG15, HERC6, SLC1A2, OLAH, MMP8 | 0,875 | 0,865 | 0,884 | 0,928 | 0,878 | 0,979 | 0,929 | 0,865 | 0,994 |
| RSAD2, IFI27, IFI44L, ISG15, HERC6, SLC1A2, OLAH, MMP8 | 0,875 | 0,865 | 0,884 | 0,928 | 0,878 | 0,978 | 0,926 | 0,859 | 0,993 |
| RSAD2, IFI27, OAS1, IFIT1, IFI44L, IL1R2, OLAH, RETN | 0,875 | 0,866 | 0,884 | 0,917 | 0,860 | 0,974 | 0,946 | 0,886 | 1 |
| RSAD2, IFIT1, SIGLEC1, ISG15, SLC1A2, OLAH, FAM20A, MMP8 | 0,875 | 0,865 | 0,884 | 0,922 | 0,869 | 0,975 | 0,949 | 0,887 | 1 |
| OAS1, IFI44L, SIGLEC1, SLC1A2, IL1R2, OLAH, FAM20A, MMP8 | 0,875 | 0,865 | 0,884 | 0,924 | 0,876 | 0,973 | 0,958 | 0,897 | 1 |
| RSAD2, OAS1, IFIT1, SIGLEC1, ISG15, OLAH, FAM20A, MMP8 | 0,875 | 0,865 | 0,885 | 0,912 | 0,848 | 0,976 | 0,959 | 0,910 | 1 |
| SIGLEC1, ISG15, SLC1A2, IL1R2, OLAH, FAM20A, RETN, MMP8 | 0,875 | 0,865 | 0,884 | 0,923 | 0,874 | 0,973 | 0,957 | 0,890 | 1 |
| RSAD2, SIGLEC1, HERC6, SLC1A2, IL1R2, FAM20A, RETN, MMP8 | 0,875 | 0,865 | 0,885 | 0,925 | 0,876 | 0,974 | 0,957 | 0,890 | 1 |
| RSAD2, IFI27, OAS1, IFIT1, SLC1A2, OLAH, RETN, MMP8 | 0,875 | 0,865 | 0,884 | 0,923 | 0,868 | 0,978 | 0,941 | 0,877 | 1 |
| RSAD2, IFI27, OAS1, IFIT1, IFI44L, ISG15, OLAH, MMP8 | 0,875 | 0,865 | 0,884 | 0,916 | 0,858 | 0,974 | 0,935 | 0,875 | 0,994 |

| | 0,875 | 0,865 | 0,884 | 0,927 | 0,877 | 0,977 | 0,946 | 0,893 | 0,998 |
|---|---|---|---|---|---|---|---|---|---|
| IFI27, IFIT1, IFI44L, SIGLEC1, HERC6, SLC1A2, IL1R2, OLAH | 0,875 | 0,865 | 0,884 | 0,927 | 0,877 | 0,977 | 0,946 | 0,893 | 0,998 |
| RSAD2, IFI27, OAS1, IFIT1, IFI44L, SIGLEC1, IL1R2, MMP8 | 0,875 | 0,866 | 0,883 | 0,919 | 0,868 | 0,969 | 0,951 | 0,905 | 0,997 |
| RSAD2, IFI27, OAS1, SIGLEC1, ISG15, SLC1A2, FAM20A, RETN | 0,875 | 0,865 | 0,885 | 0,923 | 0,866 | 0,979 | 0,943 | 0,869 | 1 |
| RSAD2, IFI27, OAS1, IFIT1, IFI44L, ISG15, IL1R2, OLAH | 0,875 | 0,866 | 0,883 | 0,918 | 0,864 | 0,973 | 0,938 | 0,881 | 0,995 |
| RSAD2, OAS1, IFIT1, SIGLEC1, HERC6, OLAH, FAM20A, RETN | 0,875 | 0,865 | 0,884 | 0,914 | 0,854 | 0,975 | 0,946 | 0,886 | 1 |
| RSAD2, IFI27, OAS1, IFI44L, ISG15, OLAH, RETN, MMP8 | 0,875 | 0,865 | 0,884 | 0,918 | 0,859 | 0,978 | 0,950 | 0,894 | 1 |
| IFI27, IFI44L, SIGLEC1, ISG15, HERC6, SLC1A2, IL1R2, RETN | 0,875 | 0,866 | 0,883 | 0,928 | 0,880 | 0,976 | 0,943 | 0,890 | 0,997 |
| RSAD2, SIGLEC1, ISG15, IL1R2, OLAH, FAM20A, RETN, MMP8 | 0,875 | 0,865 | 0,884 | 0,918 | 0,860 | 0,976 | 0,965 | 0,913 | 1 |
| RSAD2, IFI27, IFIT1, ISG15, HERC6, IL1R2, RETN, MMP8 | 0,875 | 0,866 | 0,883 | 0,920 | 0,866 | 0,974 | 0,948 | 0,896 | 1,000 |
| IFI27, IFIT1, IFI44L, ISG15, SLC1A2, IL1R2, FAM20A, RETN | 0,875 | 0,865 | 0,884 | 0,926 | 0,876 | 0,976 | 0,924 | 0,837 | 1 |
| IFI27, IFIT1, IFI44L, SIGLEC1, HERC6, SLC1A2, IL1R2, RETN | 0,874 | 0,866 | 0,883 | 0,927 | 0,878 | 0,976 | 0,946 | 0,895 | 0,997 |
| RSAD2, IFI27, IFIT1, ISG15, SLC1A2, IL1R2, OLAH, RETN | 0,874 | 0,866 | 0,883 | 0,928 | 0,878 | 0,979 | 0,935 | 0,869 | 1 |
| RSAD2, IFI27, IFIT1, IFI44L, SIGLEC1, ISG15, IL1R2, RETN | 0,874 | 0,866 | 0,883 | 0,920 | 0,865 | 0,974 | 0,947 | 0,896 | 0,998 |
| OAS1, IFIT1, SIGLEC1, SLC1A2, IL1R2, FAM20A, RETN, MMP8 | 0,874 | 0,865 | 0,884 | 0,928 | 0,881 | 0,975 | 0,959 | 0,893 | 1 |
| IFIT1, IFI44L, SIGLEC1, HERC6, SLC1A2, FAM20A, RETN, MMP8 | 0,874 | 0,865 | 0,884 | 0,923 | 0,870 | 0,976 | 0,945 | 0,873 | 1 |
| RSAD2, IFI27, OAS1, IFI44L, ISG15, SLC1A2, OLAH, MMP8 | 0,874 | 0,865 | 0,884 | 0,925 | 0,873 | 0,978 | 0,929 | 0,861 | 0,998 |
| RSAD2, OAS1, IFIT1, SIGLEC1, HERC6, SLC1A2, OLAH, FAM20A | 0,874 | 0,865 | 0,884 | 0,922 | 0,871 | 0,974 | 0,942 | 0,876 | 1 |
| RSAD2, IFI27, OAS1, IFIT1, ISG15, SLC1A2, IL1R2, OLAH | 0,874 | 0,865 | 0,883 | 0,929 | 0,880 | 0,978 | 0,930 | 0,863 | 0,998 |
| RSAD2, IFI27, OAS1, IFIT1, IFI44L, HERC6, SLC1A2, OLAH | 0,874 | 0,865 | 0,884 | 0,928 | 0,878 | 0,978 | 0,936 | 0,871 | 1 |
| RSAD2, OAS1, IFIT1, IFI44L, SIGLEC1, HERC6, FAM20A, RETN | 0,874 | 0,865 | 0,884 | 0,916 | 0,856 | 0,976 | 0,937 | 0,872 | 1 |
| RSAD2, IFI27, IFIT1, IFI44L, SIGLEC1, ISG15, SLC1A2, FAM20A | 0,874 | 0,865 | 0,884 | 0,922 | 0,865 | 0,978 | 0,939 | 0,864 | 1 |
| RSAD2, IFI27, OAS1, ISG15, SLC1A2, IL1R2, OLAH, RETN | 0,874 | 0,865 | 0,883 | 0,928 | 0,876 | 0,979 | 0,930 | 0,858 | 1 |
| RSAD2, IFI27, IFIT1, ISG15, SLC1A2, OLAH, RETN, MMP8 | 0,874 | 0,865 | 0,884 | 0,926 | 0,872 | 0,980 | 0,940 | 0,878 | 1 |
| RSAD2, IFI27, OAS1, IFI44L, HERC6, SLC1A2, IL1R2, OLAH | 0,874 | 0,864 | 0,884 | 0,929 | 0,881 | 0,977 | 0,934 | 0,868 | 1,000 |
| RSAD2, IFI27, IFI44L, ISG15, SLC1A2, OLAH, RETN, MMP8 | 0,874 | 0,865 | 0,884 | 0,927 | 0,875 | 0,980 | 0,938 | 0,874 | 1 |
| OAS1, SIGLEC1, HERC6, SLC1A2, IL1R2, FAM20A, RETN, MMP8 | 0,874 | 0,864 | 0,884 | 0,927 | 0,879 | 0,975 | 0,959 | 0,895 | 1 |
| RSAD2, IFI27, IFI44L, ISG15, SLC1A2, IL1R2, OLAH, MMP8 | 0,874 | 0,865 | 0,883 | 0,929 | 0,879 | 0,978 | 0,930 | 0,865 | 0,996 |
| OAS1, IFIT1, SIGLEC1, ISG15, HERC6, SLC1A2, IL1R2, FAM20A | 0,874 | 0,865 | 0,884 | 0,924 | 0,876 | 0,973 | 0,945 | 0,873 | 1 |

| | | | | | | | | |
|---|---|---|---|---|---|---|---|---|
| IFI27, IFIT1, IFI44L, ISG15, SLC1A2, OLAH, RETN, MMP8 | 0,874 | 0,865 | 0,884 | 0,925 | 0,870 | 0,979 | 0,942 | 0,881 | 1 |
| IFI27, OAS1, IFIT1, IFI44L, ISG15, IL1R2, OLAH, RETN | 0,874 | 0,865 | 0,883 | 0,917 | 0,861 | 0,974 | 0,937 | 0,875 | 0,999 |
| RSAD2, IFI27, OAS1, SLC1A2, IL1R2, FAM20A, RETN, MMP8 | 0,874 | 0,865 | 0,883 | 0,929 | 0,880 | 0,978 | 0,927 | 0,842 | 1 |
| RSAD2, IFI27, IFI44L, SLC1A2, IL1R2, OLAH, RETN, MMP8 | 0,874 | 0,865 | 0,884 | 0,925 | 0,873 | 0,978 | 0,941 | 0,878 | 1 |
| IFI27, IFIT1, IFI44L, SIGLEC1, ISG15, SLC1A2, IL1R2, RETN | 0,874 | 0,866 | 0,883 | 0,925 | 0,876 | 0,974 | 0,945 | 0,889 | 1 |
| RSAD2, IFI27, IFIT1, IFI44L, SLC1A2, OLAH, RETN, MMP8 | 0,874 | 0,865 | 0,884 | 0,923 | 0,867 | 0,979 | 0,945 | 0,884 | 1 |
| RSAD2, IFI27, OAS1, IFIT1, IFI44L, HERC6, RETN, MMP8 | 0,874 | 0,865 | 0,884 | 0,915 | 0,853 | 0,977 | 0,933 | 0,860 | 1 |
| RSAD2, IFI27, OAS1, IFIT1, IFI44L, HERC6, IL1R2, MMP8 | 0,874 | 0,866 | 0,883 | 0,919 | 0,870 | 0,969 | 0,938 | 0,882 | 0,994 |
| IFI27, OAS1, IFIT1, IFI44L, SLC1A2, OLAH, RETN, MMP8 | 0,874 | 0,864 | 0,884 | 0,922 | 0,866 | 0,977 | 0,941 | 0,877 | 1 |
| RSAD2, IFIT1, SIGLEC1, ISG15, HERC6, SLC1A2, IL1R2, FAM20A | 0,874 | 0,865 | 0,884 | 0,926 | 0,877 | 0,974 | 0,946 | 0,874 | 1 |
| IFI27, IFI44L, SIGLEC1, ISG15, SLC1A2, IL1R2, RETN, MMP8 | 0,874 | 0,866 | 0,883 | 0,929 | 0,882 | 0,975 | 0,961 | 0,918 | 1 |
| RSAD2, IFIT1, IFI44L, SIGLEC1, ISG15, HERC6, FAM20A, RETN | 0,874 | 0,865 | 0,883 | 0,918 | 0,859 | 0,976 | 0,940 | 0,876 | 1 |
| IFI27, OAS1, IFIT1, IFI44L, SIGLEC1, SLC1A2, IL1R2, RETN | 0,874 | 0,865 | 0,883 | 0,923 | 0,873 | 0,973 | 0,950 | 0,895 | 1 |
| IFI27, OAS1, IFIT1, IFI44L, ISG15, SLC1A2, OLAH, MMP8 | 0,874 | 0,865 | 0,884 | 0,927 | 0,875 | 0,979 | 0,929 | 0,862 | 0,997 |
| RSAD2, IFI27, IFIT1, SIGLEC1, HERC6, SLC1A2, IL1R2, RETN | 0,874 | 0,865 | 0,883 | 0,927 | 0,881 | 0,974 | 0,951 | 0,903 | 0,999 |
| RSAD2, IFI27, OAS1, IFIT1, IFI44L, SLC1A2, OLAH, MMP8 | 0,874 | 0,865 | 0,883 | 0,927 | 0,875 | 0,979 | 0,941 | 0,879 | 1 |
| RSAD2, IFI27, OAS1, IFIT1, SLC1A2, IL1R2, OLAH, RETN | 0,874 | 0,865 | 0,883 | 0,926 | 0,874 | 0,978 | 0,938 | 0,869 | 1 |
| RSAD2, IFI27, IFIT1, IFI44L, ISG15, IL1R2, OLAH, RETN | 0,874 | 0,865 | 0,883 | 0,916 | 0,859 | 0,974 | 0,938 | 0,877 | 1,000 |
| RSAD2, IFI27, IFIT1, IFI44L, SIGLEC1, SLC1A2, FAM20A, MMP8 | 0,874 | 0,865 | 0,884 | 0,923 | 0,866 | 0,979 | 0,940 | 0,866 | 1 |
| IFI27, IFIT1, IFI44L, SLC1A2, IL1R2, OLAH, RETN, MMP8 | 0,874 | 0,864 | 0,884 | 0,924 | 0,871 | 0,977 | 0,946 | 0,888 | 1 |
| IFI27, IFIT1, IFI44L, HERC6, SLC1A2, IL1R2, OLAH, RETN | 0,874 | 0,865 | 0,883 | 0,924 | 0,871 | 0,976 | 0,935 | 0,869 | 1 |
| IFI27, OAS1, IFI44L, ISG15, HERC6, SLC1A2, FAM20A, RETN | 0,874 | 0,864 | 0,884 | 0,923 | 0,868 | 0,979 | 0,935 | 0,854 | 1 |
| IFI27, IFI44L, SIGLEC1, ISG15, SLC1A2, FAM20A, RETN, MMP8 | 0,874 | 0,863 | 0,885 | 0,924 | 0,868 | 0,980 | 0,948 | 0,872 | 1 |
| IFI44L, SIGLEC1, SLC1A2, IL1R2, OLAH, FAM20A, RETN, MMP8 | 0,874 | 0,864 | 0,883 | 0,923 | 0,872 | 0,973 | 0,957 | 0,891 | 1 |
| RSAD2, IFIT1, IFI44L, SIGLEC1, HERC6, OLAH, FAM20A, RETN | 0,874 | 0,865 | 0,883 | 0,916 | 0,856 | 0,977 | 0,943 | 0,884 | 1 |
| IFI27, OAS1, ISG15, SLC1A2, IL1R2, FAM20A, RETN, MMP8 | 0,874 | 0,865 | 0,883 | 0,928 | 0,878 | 0,978 | 0,927 | 0,841 | 1 |
| RSAD2, IFIT1, SIGLEC1, ISG15, HERC6, OLAH, FAM20A, RETN | 0,874 | 0,865 | 0,883 | 0,916 | 0,856 | 0,976 | 0,948 | 0,889 | 1 |
| RSAD2, IFIT1, IFI44L, SIGLEC1, ISG15, IL1R2, OLAH, FAM20A | 0,874 | 0,865 | 0,883 | 0,911 | 0,847 | 0,975 | 0,962 | 0,913 | 1 |

| | | | | | | | | | |
|---|---|---|---|---|---|---|---|---|---|
| OAS1, IFIT1, SIGLEC1, ISG15, HERC6, SLC1A2, OLAH, FAM20A | 0,874 | 0,864 | 0,884 | 0,922 | 0,870 | 0,973 | 0,937 | 0,867 | 1 |
| OAS1, IFIT1, SIGLEC1, ISG15, IL1R2, OLAH, FAM20A, RETN | 0,874 | 0,865 | 0,883 | 0,911 | 0,849 | 0,974 | 0,961 | 0,910 | 1 |
| OAS1, IFIT1, IFI44L, SIGLEC1, IL1R2, OLAH, FAM20A, RETN | 0,874 | 0,865 | 0,883 | 0,910 | 0,847 | 0,974 | 0,960 | 0,907 | 1 |
| IFI27, OAS1, IFI44L, ISG15, HERC6, SLC1A2, IL1R2, OLAH | 0,874 | 0,864 | 0,884 | 0,927 | 0,878 | 0,976 | 0,928 | 0,861 | 0,995 |
| RSAD2, IFI27, IFI44L, SIGLEC1, HERC6, SLC1A2, IL1R2, RETN | 0,874 | 0,865 | 0,882 | 0,927 | 0,878 | 0,976 | 0,945 | 0,892 | 0,997 |
| RSAD2, IFIT1, SIGLEC1, ISG15, HERC6, SLC1A2, OLAH, FAM20A | 0,874 | 0,864 | 0,884 | 0,922 | 0,869 | 0,975 | 0,936 | 0,862 | 1 |
| IFI27, OAS1, IFIT1, HERC6, SLC1A2, IL1R2, RETN, MMP8 | 0,874 | 0,865 | 0,883 | 0,923 | 0,873 | 0,974 | 0,938 | 0,873 | 1 |
| IFI27, OAS1, IFIT1, ISG15, SLC1A2, IL1R2, FAM20A, RETN | 0,874 | 0,864 | 0,883 | 0,927 | 0,878 | 0,977 | 0,925 | 0,841 | 1 |
| OAS1, IFIT1, SIGLEC1, HERC6, SLC1A2, FAM20A, RETN, MMP8 | 0,874 | 0,864 | 0,883 | 0,920 | 0,866 | 0,974 | 0,942 | 0,870 | 1 |
| OAS1, IFIT1, IFI44L, SIGLEC1, OLAH, FAM20A, RETN, MMP8 | 0,874 | 0,864 | 0,884 | 0,915 | 0,852 | 0,979 | 0,962 | 0,916 | 1 |
| RSAD2, IFIT1, IFI44L, SIGLEC1, HERC6, FAM20A, RETN, MMP8 | 0,874 | 0,865 | 0,883 | 0,919 | 0,860 | 0,979 | 0,942 | 0,874 | 1 |
| RSAD2, IFIT1, IFI44L, SIGLEC1, HERC6, IL1R2, FAM20A, RETN | 0,874 | 0,865 | 0,883 | 0,919 | 0,861 | 0,976 | 0,952 | 0,895 | 1 |
| RSAD2, IFIT1, SIGLEC1, SLC1A2, IL1R2, FAM20A, RETN, MMP8 | 0,874 | 0,865 | 0,883 | 0,927 | 0,879 | 0,975 | 0,959 | 0,893 | 1 |
| IFI27, OAS1, IFIT1, IFI44L, ISG15, SLC1A2, IL1R2, FAM20A | 0,874 | 0,864 | 0,883 | 0,927 | 0,878 | 0,975 | 0,926 | 0,842 | 1 |
| RSAD2, IFI27, IFI44L, ISG15, HERC6, SLC1A2, IL1R2, OLAH | 0,874 | 0,864 | 0,883 | 0,929 | 0,880 | 0,978 | 0,925 | 0,856 | 0,994 |
| RSAD2, IFI27, OAS1, SIGLEC1, ISG15, IL1R2, RETN, MMP8 | 0,874 | 0,864 | 0,883 | 0,922 | 0,868 | 0,976 | 0,965 | 0,920 | 1 |
| IFI27, OAS1, IFIT1, IFI44L, SIGLEC1, ISG15, IL1R2, MMP8 | 0,874 | 0,865 | 0,882 | 0,922 | 0,874 | 0,970 | 0,945 | 0,895 | 0,995 |
| RSAD2, IFI27, OAS1, IFIT1, IFI44L, SIGLEC1, ISG15, IL1R2 | 0,874 | 0,865 | 0,882 | 0,918 | 0,867 | 0,970 | 0,948 | 0,900 | 0,996 |
| IFI27, OAS1, IFIT1, SIGLEC1, HERC6, SLC1A2, IL1R2, MMP8 | 0,874 | 0,865 | 0,883 | 0,926 | 0,882 | 0,971 | 0,949 | 0,899 | 0,999 |
| IFI27, IFIT1, IFI44L, ISG15, SLC1A2, IL1R2, OLAH, RETN | 0,874 | 0,865 | 0,883 | 0,927 | 0,876 | 0,978 | 0,935 | 0,870 | 1 |
| RSAD2, IFI27, IFI44L, SIGLEC1, ISG15, HERC6, RETN, MMP8 | 0,874 | 0,864 | 0,883 | 0,917 | 0,859 | 0,975 | 0,945 | 0,890 | 0,999 |
| IFIT1, SIGLEC1, ISG15, SLC1A2, IL1R2, FAM20A, RETN, MMP8 | 0,874 | 0,864 | 0,883 | 0,927 | 0,881 | 0,974 | 0,958 | 0,892 | 1 |
| RSAD2, IFIT1, IFI44L, SIGLEC1, HERC6, SLC1A2, IL1R2, FAM20A | 0,874 | 0,864 | 0,883 | 0,926 | 0,877 | 0,974 | 0,941 | 0,869 | 1 |
| RSAD2, IFI27, IFIT1, IFI44L, ISG15, SLC1A2, OLAH, MMP8 | 0,874 | 0,865 | 0,883 | 0,926 | 0,874 | 0,978 | 0,929 | 0,863 | 0,996 |
| IFI27, IFIT1, IFI44L, SIGLEC1, SLC1A2, IL1R2, RETN, MMP8 | 0,874 | 0,865 | 0,882 | 0,926 | 0,878 | 0,975 | 0,958 | 0,912 | 1 |
| OAS1, IFI44L, SIGLEC1, ISG15, IL1R2, FAM20A, RETN, MMP8 | 0,874 | 0,864 | 0,883 | 0,916 | 0,857 | 0,975 | 0,961 | 0,902 | 1 |
| RSAD2, SIGLEC1, SLC1A2, IL1R2, OLAH, FAM20A, RETN, MMP8 | 0,874 | 0,864 | 0,883 | 0,924 | 0,875 | 0,973 | 0,961 | 0,903 | 1 |
| RSAD2, IFI27, IFIT1, IFI44L, ISG15, HERC6, SLC1A2, OLAH | 0,874 | 0,864 | 0,883 | 0,928 | 0,877 | 0,978 | 0,929 | 0,865 | 0,994 |

542

| | | | | | | | | | |
|---|---|---|---|---|---|---|---|---|---|
| SIGLEC1, ISG15, HERC6, SLC1A2, IL1R2, FAM20A, RETN, MMP8 | 0,873 | 0,864 | 0,883 | 0,925 | 0,876 | 0,974 | 0,955 | 0,885 | 1 |
| IFIT1, IFI44L, SIGLEC1, ISG15, OLAH, FAM20A, RETN, MMP8 | 0,873 | 0,864 | 0,883 | 0,914 | 0,851 | 0,977 | 0,957 | 0,907 | 1 |
| RSAD2, IFI27, OAS1, ISG15, SLC1A2, OLAH, RETN, MMP8 | 0,873 | 0,864 | 0,883 | 0,927 | 0,873 | 0,980 | 0,938 | 0,872 | 1 |
| IFI27, OAS1, IFI44L, ISG15, SLC1A2, IL1R2, FAM20A, RETN | 0,873 | 0,864 | 0,883 | 0,927 | 0,877 | 0,977 | 0,924 | 0,839 | 1 |
| IFI27, OAS1, IFIT1, SIGLEC1, ISG15, SLC1A2, IL1R2, RETN | 0,873 | 0,865 | 0,882 | 0,925 | 0,877 | 0,974 | 0,953 | 0,898 | 1 |
| RSAD2, IFI27, OAS1, IFIT1, IFI44L, SLC1A2, IL1R2, FAM20A | 0,873 | 0,864 | 0,883 | 0,926 | 0,878 | 0,975 | 0,926 | 0,842 | 1 |
| RSAD2, IFI27, IFIT1, SIGLEC1, ISG15, HERC6, IL1R2, MMP8 | 0,873 | 0,865 | 0,882 | 0,922 | 0,875 | 0,969 | 0,946 | 0,897 | 0,995 |
| RSAD2, IFI27, IFIT1, IFI44L, SLC1A2, IL1R2, FAM20A, RETN | 0,873 | 0,864 | 0,883 | 0,926 | 0,875 | 0,977 | 0,925 | 0,840 | 1 |
| RSAD2, IFI27, IFIT1, IFI44L, SLC1A2, IL1R2, OLAH, MMP8 | 0,873 | 0,864 | 0,882 | 0,926 | 0,876 | 0,976 | 0,936 | 0,875 | 0,996 |
| RSAD2, IFI27, IFIT1, SIGLEC1, ISG15, IL1R2, RETN, MMP8 | 0,873 | 0,864 | 0,882 | 0,922 | 0,868 | 0,975 | 0,963 | 0,922 | 1 |
| IFI27, IFIT1, IFI44L, ISG15, HERC6, SLC1A2, FAM20A, RETN | 0,873 | 0,863 | 0,883 | 0,921 | 0,864 | 0,978 | 0,935 | 0,853 | 1 |
| IFI27, OAS1, IFIT1, ISG15, HERC6, SLC1A2, IL1R2, RETN | 0,873 | 0,864 | 0,882 | 0,924 | 0,874 | 0,974 | 0,939 | 0,876 | 1 |
| IFIT1, IFI44L, SIGLEC1, ISG15, IL1R2, OLAH, FAM20A, RETN | 0,873 | 0,864 | 0,882 | 0,908 | 0,844 | 0,971 | 0,958 | 0,906 | 1 |
| IFI27, OAS1, IFI44L, ISG15, SLC1A2, OLAH, RETN, MMP8 | 0,873 | 0,864 | 0,883 | 0,926 | 0,873 | 0,979 | 0,939 | 0,875 | 1 |
| OAS1, SIGLEC1, ISG15, SLC1A2, IL1R2, FAM20A, RETN, MMP8 | 0,873 | 0,864 | 0,883 | 0,925 | 0,877 | 0,973 | 0,958 | 0,893 | 1 |
| RSAD2, IFI27, IFI44L, ISG15, SLC1A2, IL1R2, OLAH, RETN | 0,873 | 0,864 | 0,882 | 0,927 | 0,876 | 0,978 | 0,932 | 0,863 | 1,000 |
| RSAD2, OAS1, IFI44L, SIGLEC1, SLC1A2, IL1R2, FAM20A, MMP8 | 0,873 | 0,864 | 0,883 | 0,923 | 0,874 | 0,971 | 0,961 | 0,899 | 1 |
| RSAD2, IFI27, OAS1, IFIT1, SIGLEC1, ISG15, IL1R2, RETN | 0,873 | 0,865 | 0,882 | 0,918 | 0,864 | 0,973 | 0,954 | 0,902 | 1 |
| IFI27, OAS1, IFIT1, SIGLEC1, ISG15, SLC1A2, FAM20A, MMP8 | 0,873 | 0,863 | 0,883 | 0,921 | 0,865 | 0,977 | 0,941 | 0,869 | 1 |
| RSAD2, OAS1, SIGLEC1, ISG15, SLC1A2, IL1R2, FAM20A, MMP8 | 0,873 | 0,863 | 0,883 | 0,924 | 0,876 | 0,972 | 0,959 | 0,893 | 1 |
| RSAD2, OAS1, IFI44L, SIGLEC1, IL1R2, FAM20A, RETN, MMP8 | 0,873 | 0,863 | 0,883 | 0,919 | 0,862 | 0,976 | 0,962 | 0,906 | 1 |
| RSAD2, IFI27, IFI44L, SIGLEC1, ISG15, SLC1A2, FAM20A, RETN | 0,873 | 0,863 | 0,883 | 0,922 | 0,866 | 0,978 | 0,942 | 0,867 | 1 |
| IFI27, OAS1, IFIT1, IFI44L, HERC6, SLC1A2, IL1R2, OLAH | 0,873 | 0,863 | 0,883 | 0,926 | 0,877 | 0,975 | 0,935 | 0,870 | 1 |
| RSAD2, IFI27, OAS1, IFI44L, ISG15, SLC1A2, IL1R2, FAM20A | 0,873 | 0,864 | 0,883 | 0,927 | 0,879 | 0,975 | 0,928 | 0,844 | 1 |
| RSAD2, IFI27, OAS1, IFIT1, IFI44L, ISG15, OLAH, RETN | 0,873 | 0,864 | 0,882 | 0,915 | 0,856 | 0,975 | 0,940 | 0,876 | 1 |
| IFIT1, IFI44L, SIGLEC1, ISG15, SLC1A2, OLAH, FAM20A, RETN | 0,873 | 0,864 | 0,882 | 0,919 | 0,863 | 0,975 | 0,935 | 0,871 | 0,999 |
| RSAD2, OAS1, SIGLEC1, HERC6, SLC1A2, OLAH, FAM20A, MMP8 | 0,873 | 0,863 | 0,883 | 0,920 | 0,868 | 0,972 | 0,945 | 0,878 | 1 |
| RSAD2, OAS1, IFIT1, SIGLEC1, HERC6, SLC1A2, IL1R2, FAM20A | 0,873 | 0,864 | 0,883 | 0,926 | 0,877 | 0,974 | 0,949 | 0,884 | 1 |

| | | | | | | | | | |
|---|---|---|---|---|---|---|---|---|---|
| OAS1, IFIT1, SIGLEC1, ISG15, OLAH, FAM20A, RETN, MMP8 | 0,873 | 0,863 | 0,883 | 0,912 | 0,848 | 0,977 | 0,961 | 0,912 | 1 |
| RSAD2, IFI27, IFIT1, IFI44L, ISG15, SLC1A2, IL1R2, FAM20A | 0,873 | 0,864 | 0,883 | 0,925 | 0,876 | 0,975 | 0,927 | 0,842 | 1 |
| OAS1, SIGLEC1, SLC1A2, IL1R2, OLAH, FAM20A, RETN, MMP8 | 0,873 | 0,863 | 0,883 | 0,924 | 0,874 | 0,973 | 0,960 | 0,902 | 1 |
| IFI27, IFIT1, IFI44L, HERC6, SLC1A2, IL1R2, OLAH, MMP8 | 0,873 | 0,864 | 0,882 | 0,924 | 0,874 | 0,975 | 0,933 | 0,870 | 0,995 |
| RSAD2, IFI27, OAS1, IFI44L, SIGLEC1, SLC1A2, FAM20A, RETN | 0,873 | 0,863 | 0,883 | 0,923 | 0,867 | 0,980 | 0,939 | 0,862 | 1 |
| IFI27, OAS1, IFIT1, IFI44L, SLC1A2, IL1R2, OLAH, MMP8 | 0,873 | 0,864 | 0,882 | 0,926 | 0,876 | 0,976 | 0,937 | 0,872 | 1 |
| RSAD2, IFI27, OAS1, ISG15, HERC6, SLC1A2, FAM20A, RETN | 0,873 | 0,863 | 0,883 | 0,923 | 0,867 | 0,979 | 0,932 | 0,850 | 1 |
| OAS1, IFI44L, SIGLEC1, IL1R2, OLAH, FAM20A, RETN, MMP8 | 0,873 | 0,863 | 0,883 | 0,919 | 0,861 | 0,976 | 0,964 | 0,911 | 1 |
| IFIT1, SIGLEC1, ISG15, HERC6, SLC1A2, IL1R2, FAM20A, RETN | 0,873 | 0,863 | 0,883 | 0,924 | 0,875 | 0,973 | 0,946 | 0,874 | 1 |
| RSAD2, IFI27, OAS1, IFI44L, ISG15, SLC1A2, IL1R2, OLAH | 0,873 | 0,864 | 0,882 | 0,930 | 0,880 | 0,979 | 0,932 | 0,864 | 0,999 |
| IFIT1, IFI44L, SIGLEC1, ISG15, SLC1A2, IL1R2, OLAH, FAM20A | 0,873 | 0,864 | 0,882 | 0,920 | 0,866 | 0,973 | 0,946 | 0,886 | 1 |
| RSAD2, IFI27, OAS1, IFIT1, HERC6, SLC1A2, IL1R2, RETN | 0,873 | 0,864 | 0,882 | 0,925 | 0,875 | 0,975 | 0,934 | 0,862 | 1 |
| IFI27, OAS1, IFI44L, SIGLEC1, SLC1A2, IL1R2, RETN, MMP8 | 0,873 | 0,864 | 0,882 | 0,928 | 0,882 | 0,974 | 0,959 | 0,910 | 1 |
| RSAD2, SIGLEC1, ISG15, SLC1A2, IL1R2, FAM20A, RETN, MMP8 | 0,873 | 0,863 | 0,883 | 0,927 | 0,879 | 0,974 | 0,959 | 0,895 | 1 |
| RSAD2, IFI27, IFIT1, IFI44L, HERC6, SLC1A2, IL1R2, OLAH | 0,873 | 0,863 | 0,882 | 0,925 | 0,875 | 0,975 | 0,932 | 0,868 | 0,995 |
| IFI27, SIGLEC1, ISG15, HERC6, SLC1A2, FAM20A, RETN, MMP8 | 0,873 | 0,862 | 0,884 | 0,924 | 0,867 | 0,982 | 0,950 | 0,874 | 1 |
| RSAD2, OAS1, IFIT1, IFI44L, SIGLEC1, ISG15, IL1R2, FAM20A | 0,873 | 0,864 | 0,882 | 0,912 | 0,849 | 0,975 | 0,963 | 0,914 | 1 |
| IFI27, OAS1, IFIT1, IFI44L, ISG15, SLC1A2, OLAH, RETN | 0,873 | 0,864 | 0,882 | 0,925 | 0,873 | 0,978 | 0,936 | 0,868 | 1 |
| IFI27, IFIT1, IFI44L, ISG15, HERC6, SLC1A2, IL1R2, OLAH | 0,873 | 0,864 | 0,882 | 0,927 | 0,878 | 0,977 | 0,929 | 0,864 | 0,995 |
| IFI44L, SIGLEC1, HERC6, SLC1A2, IL1R2, FAM20A, RETN, MMP8 | 0,873 | 0,863 | 0,883 | 0,923 | 0,873 | 0,973 | 0,959 | 0,893 | 1 |
| IFI27, IFIT1, SIGLEC1, ISG15, HERC6, SLC1A2, IL1R2, RETN | 0,873 | 0,864 | 0,882 | 0,927 | 0,880 | 0,974 | 0,954 | 0,908 | 1 |
| RSAD2, IFI27, OAS1, IFIT1, SIGLEC1, SLC1A2, IL1R2, RETN | 0,873 | 0,864 | 0,881 | 0,923 | 0,873 | 0,973 | 0,957 | 0,900 | 1 |
| RSAD2, IFI27, OAS1, IFI44L, HERC6, SLC1A2, IL1R2, RETN | 0,873 | 0,864 | 0,882 | 0,924 | 0,873 | 0,974 | 0,932 | 0,858 | 1 |
| IFI27, OAS1, IFI44L, SLC1A2, IL1R2, OLAH, RETN, MMP8 | 0,873 | 0,863 | 0,883 | 0,927 | 0,876 | 0,979 | 0,942 | 0,880 | 1 |
| RSAD2, IFI27, OAS1, ISG15, HERC6, SLC1A2, IL1R2, RETN | 0,873 | 0,864 | 0,882 | 0,924 | 0,873 | 0,975 | 0,933 | 0,860 | 1 |
| RSAD2, IFI27, OAS1, IFIT1, SIGLEC1, HERC6, SLC1A2, IL1R2 | 0,873 | 0,864 | 0,882 | 0,927 | 0,882 | 0,972 | 0,954 | 0,907 | 1 |
| RSAD2, IFI27, OAS1, HERC6, SLC1A2, IL1R2, RETN, MMP8 | 0,873 | 0,864 | 0,882 | 0,923 | 0,872 | 0,974 | 0,933 | 0,860 | 1 |
| RSAD2, IFI27, OAS1, IFIT1, IFI44L, IL1R2, RETN, MMP8 | 0,873 | 0,864 | 0,882 | 0,918 | 0,863 | 0,974 | 0,943 | 0,884 | 1 |

544

| | | | | | | | | | |
|---|---|---|---|---|---|---|---|---|---|
| RSAD2, IFI27, IFIT1, ISG15, SLC1A2, IL1R2, FAM20A, RETN | 0,873 | 0,864 | 0,882 | 0,926 | 0,876 | 0,976 | 0,927 | 0,844 | 1 |
| IFIT1, SIGLEC1, ISG15, HERC6, SLC1A2, FAM20A, RETN, MMP8 | 0,873 | 0,863 | 0,883 | 0,920 | 0,865 | 0,975 | 0,948 | 0,877 | 1 |
| IFIT1, IFI44L, SIGLEC1, HERC6, SLC1A2, IL1R2, FAM20A, RETN | 0,873 | 0,863 | 0,882 | 0,926 | 0,878 | 0,974 | 0,941 | 0,869 | 1 |
| RSAD2, IFI27, OAS1, ISG15, SLC1A2, IL1R2, FAM20A, MMP8 | 0,873 | 0,863 | 0,882 | 0,929 | 0,883 | 0,976 | 0,927 | 0,840 | 1 |
| RSAD2, IFI27, OAS1, IFIT1, ISG15, SLC1A2, OLAH, RETN | 0,873 | 0,864 | 0,882 | 0,926 | 0,873 | 0,979 | 0,935 | 0,865 | 1 |
| RSAD2, IFI27, IFIT1, IFI44L, ISG15, SLC1A2, OLAH, RETN | 0,873 | 0,864 | 0,881 | 0,927 | 0,875 | 0,979 | 0,935 | 0,868 | 1 |
| IFIT1, SIGLEC1, HERC6, SLC1A2, IL1R2, OLAH, FAM20A, RETN | 0,873 | 0,863 | 0,883 | 0,924 | 0,875 | 0,974 | 0,943 | 0,872 | 1 |
| RSAD2, OAS1, IFIT1, SIGLEC1, IL1R2, OLAH, FAM20A, RETN | 0,873 | 0,863 | 0,882 | 0,911 | 0,847 | 0,974 | 0,964 | 0,916 | 1 |
| RSAD2, IFI27, IFIT1, SIGLEC1, ISG15, SLC1A2, FAM20A, MMP8 | 0,873 | 0,863 | 0,882 | 0,922 | 0,866 | 0,978 | 0,940 | 0,864 | 1 |
| RSAD2, IFI27, OAS1, IFI44L, SLC1A2, IL1R2, OLAH, MMP8 | 0,873 | 0,863 | 0,882 | 0,927 | 0,877 | 0,977 | 0,930 | 0,862 | 0,999 |
| RSAD2, IFI27, OAS1, IFI44L, ISG15, SLC1A2, OLAH, RETN | 0,873 | 0,864 | 0,882 | 0,928 | 0,876 | 0,980 | 0,933 | 0,862 | 1 |
| RSAD2, IFI27, IFIT1, SIGLEC1, SLC1A2, IL1R2, RETN, MMP8 | 0,873 | 0,864 | 0,881 | 0,927 | 0,879 | 0,974 | 0,964 | 0,922 | 1 |
| IFI27, OAS1, ISG15, HERC6, SLC1A2, IL1R2, RETN, MMP8 | 0,873 | 0,864 | 0,882 | 0,926 | 0,877 | 0,975 | 0,933 | 0,863 | 1 |
| RSAD2, IFI27, SIGLEC1, ISG15, HERC6, SLC1A2, IL1R2, RETN | 0,873 | 0,864 | 0,881 | 0,926 | 0,878 | 0,973 | 0,951 | 0,901 | 1 |
| RSAD2, IFI27, OAS1, IFI44L, SIGLEC1, SLC1A2, IL1R2, RETN | 0,873 | 0,864 | 0,881 | 0,924 | 0,875 | 0,974 | 0,951 | 0,892 | 1 |
| RSAD2, IFI27, OAS1, IFIT1, IFI44L, ISG15, SLC1A2, OLAH | 0,873 | 0,863 | 0,882 | 0,926 | 0,873 | 0,979 | 0,933 | 0,866 | 1,000 |
| IFIT1, SIGLEC1, ISG15, HERC6, SLC1A2, OLAH, FAM20A, RETN | 0,873 | 0,863 | 0,883 | 0,919 | 0,866 | 0,973 | 0,935 | 0,861 | 1 |
| RSAD2, IFI27, OAS1, IFIT1, IFI44L, SLC1A2, OLAH, RETN | 0,873 | 0,864 | 0,881 | 0,926 | 0,874 | 0,979 | 0,940 | 0,873 | 1 |
| IFI27, IFIT1, SIGLEC1, ISG15, SLC1A2, IL1R2, RETN, MMP8 | 0,873 | 0,864 | 0,881 | 0,925 | 0,878 | 0,973 | 0,966 | 0,927 | 1 |
| RSAD2, OAS1, SIGLEC1, ISG15, HERC6, IL1R2, OLAH, FAM20A | 0,873 | 0,863 | 0,882 | 0,916 | 0,858 | 0,974 | 0,958 | 0,903 | 1 |
| OAS1, IFIT1, IFI44L, SIGLEC1, ISG15, SLC1A2, OLAH, FAM20A | 0,873 | 0,863 | 0,882 | 0,919 | 0,864 | 0,975 | 0,934 | 0,868 | 0,999 |
| RSAD2, IFI27, OAS1, IFIT1, SIGLEC1, ISG15, SLC1A2, FAM20A | 0,872 | 0,863 | 0,882 | 0,922 | 0,867 | 0,978 | 0,941 | 0,867 | 1 |
| IFI27, OAS1, IFI44L, SIGLEC1, ISG15, SLC1A2, IL1R2, RETN | 0,872 | 0,864 | 0,881 | 0,926 | 0,878 | 0,974 | 0,948 | 0,893 | 1 |
| RSAD2, IFI27, OAS1, SIGLEC1, HERC6, SLC1A2, IL1R2, MMP8 | 0,872 | 0,863 | 0,882 | 0,926 | 0,880 | 0,971 | 0,950 | 0,899 | 1 |
| IFI27, OAS1, IFIT1, IFI44L, SLC1A2, IL1R2, OLAH, RETN | 0,872 | 0,863 | 0,882 | 0,926 | 0,874 | 0,977 | 0,937 | 0,869 | 1 |
| IFIT1, IFI44L, SIGLEC1, SLC1A2, IL1R2, OLAH, FAM20A, RETN | 0,872 | 0,863 | 0,882 | 0,921 | 0,868 | 0,974 | 0,946 | 0,885 | 1 |
| RSAD2, IFI27, OAS1, IFIT1, SLC1A2, IL1R2, FAM20A, RETN | 0,872 | 0,863 | 0,882 | 0,927 | 0,877 | 0,977 | 0,920 | 0,831 | 1 |
| RSAD2, IFI27, OAS1, IFI44L, SIGLEC1, ISG15, IL1R2, MMP8 | 0,872 | 0,864 | 0,881 | 0,920 | 0,870 | 0,971 | 0,949 | 0,902 | 0,996 |

| | | | | | | | | |
|---|---|---|---|---|---|---|---|---|
| SIGLEC1, HERC6, SLC1A2, IL1R2, OLAH, FAM20A, RETN, MMP8 | 0,872 | 0,862 | 0,883 | 0,923 | 0,872 | 0,973 | 0,958 | 0,892 | 1 |
| IFI27, OAS1, IFI44L, ISG15, SLC1A2, IL1R2, OLAH, MMP8 | 0,872 | 0,863 | 0,882 | 0,928 | 0,878 | 0,977 | 0,925 | 0,855 | 0,995 |
| RSAD2, IFI44L, SIGLEC1, ISG15, SLC1A2, IL1R2, FAM20A, MMP8 | 0,872 | 0,863 | 0,882 | 0,925 | 0,878 | 0,973 | 0,959 | 0,893 | 1 |
| OAS1, IFIT1, SIGLEC1, HERC6, SLC1A2, IL1R2, FAM20A, RETN | 0,872 | 0,863 | 0,882 | 0,924 | 0,876 | 0,973 | 0,945 | 0,873 | 1 |
| OAS1, IFIT1, IFI44L, SIGLEC1, ISG15, IL1R2, FAM20A, RETN | 0,872 | 0,863 | 0,882 | 0,910 | 0,847 | 0,972 | 0,963 | 0,911 | 1 |
| RSAD2, IFI27, IFIT1, IFI44L, SIGLEC1, ISG15, IL1R2, MMP8 | 0,872 | 0,864 | 0,880 | 0,919 | 0,869 | 0,969 | 0,943 | 0,893 | 0,994 |
| IFI27, OAS1, SIGLEC1, ISG15, SLC1A2, IL1R2, RETN, MMP8 | 0,872 | 0,864 | 0,881 | 0,927 | 0,880 | 0,974 | 0,963 | 0,916 | 1 |
| RSAD2, IFI27, OAS1, SIGLEC1, SLC1A2, IL1R2, RETN, MMP8 | 0,872 | 0,863 | 0,881 | 0,925 | 0,877 | 0,973 | 0,958 | 0,903 | 1 |
| RSAD2, IFI27, IFI44L, SIGLEC1, SLC1A2, IL1R2, RETN, MMP8 | 0,872 | 0,864 | 0,881 | 0,928 | 0,880 | 0,975 | 0,962 | 0,921 | 1 |
| RSAD2, IFI27, IFIT1, IFI44L, HERC6, SLC1A2, FAM20A, RETN | 0,872 | 0,863 | 0,882 | 0,922 | 0,866 | 0,977 | 0,929 | 0,846 | 1 |
| RSAD2, OAS1, SIGLEC1, SLC1A2, IL1R2, FAM20A, RETN, MMP8 | 0,872 | 0,862 | 0,882 | 0,924 | 0,874 | 0,973 | 0,958 | 0,894 | 1 |
| OAS1, IFI44L, SIGLEC1, ISG15, SLC1A2, IL1R2, FAM20A, MMP8 | 0,872 | 0,863 | 0,882 | 0,924 | 0,876 | 0,972 | 0,959 | 0,893 | 1 |
| RSAD2, IFI27, OAS1, SIGLEC1, ISG15, SLC1A2, IL1R2, RETN | 0,872 | 0,864 | 0,881 | 0,923 | 0,873 | 0,972 | 0,955 | 0,898 | 1 |
| IFI27, OAS1, ISG15, HERC6, SLC1A2, FAM20A, RETN, MMP8 | 0,872 | 0,862 | 0,883 | 0,922 | 0,866 | 0,979 | 0,934 | 0,853 | 1 |
| RSAD2, IFI27, OAS1, IFI44L, SIGLEC1, HERC6, SLC1A2, IL1R2 | 0,872 | 0,863 | 0,881 | 0,926 | 0,880 | 0,972 | 0,955 | 0,909 | 1 |
| RSAD2, IFI27, IFI44L, ISG15, SLC1A2, IL1R2, FAM20A, RETN | 0,872 | 0,862 | 0,882 | 0,927 | 0,878 | 0,977 | 0,923 | 0,836 | 1 |
| RSAD2, IFI27, OAS1, SIGLEC1, HERC6, SLC1A2, FAM20A, MMP8 | 0,872 | 0,862 | 0,883 | 0,919 | 0,865 | 0,974 | 0,939 | 0,863 | 1 |
| RSAD2, IFI27, OAS1, IFI44L, SLC1A2, OLAH, RETN, MMP8 | 0,872 | 0,862 | 0,882 | 0,925 | 0,871 | 0,980 | 0,940 | 0,875 | 1 |
| OAS1, IFIT1, SIGLEC1, HERC6, SLC1A2, OLAH, FAM20A, RETN | 0,872 | 0,862 | 0,882 | 0,921 | 0,869 | 0,973 | 0,936 | 0,865 | 1 |
| RSAD2, OAS1, IFIT1, IFI44L, SIGLEC1, ISG15, HERC6, FAM20A | 0,872 | 0,863 | 0,881 | 0,911 | 0,850 | 0,972 | 0,935 | 0,866 | 1 |
| OAS1, IFIT1, IFI44L, SIGLEC1, HERC6, SLC1A2, FAM20A, RETN | 0,872 | 0,863 | 0,881 | 0,925 | 0,875 | 0,975 | 0,935 | 0,862 | 1 |
| RSAD2, IFI44L, SIGLEC1, IL1R2, OLAH, FAM20A, RETN, MMP8 | 0,872 | 0,862 | 0,882 | 0,919 | 0,862 | 0,976 | 0,964 | 0,911 | 1 |
| IFIT1, IFI44L, SIGLEC1, ISG15, HERC6, SLC1A2, FAM20A, RETN | 0,872 | 0,863 | 0,881 | 0,923 | 0,872 | 0,975 | 0,935 | 0,859 | 1 |
| RSAD2, IFI44L, SIGLEC1, ISG15, HERC6, IL1R2, OLAH, FAM20A | 0,872 | 0,862 | 0,882 | 0,915 | 0,856 | 0,974 | 0,958 | 0,901 | 1 |
| RSAD2, IFI27, OAS1, IFIT1, IFI44L, SLC1A2, IL1R2, OLAH | 0,872 | 0,863 | 0,881 | 0,926 | 0,875 | 0,977 | 0,938 | 0,872 | 1 |
| RSAD2, IFI27, IFIT1, IFI44L, SLC1A2, IL1R2, OLAH, RETN | 0,872 | 0,863 | 0,881 | 0,922 | 0,869 | 0,975 | 0,935 | 0,869 | 1 |
| RSAD2, IFI27, OAS1, IFIT1, ISG15, SLC1A2, IL1R2, FAM20A | 0,872 | 0,863 | 0,882 | 0,925 | 0,876 | 0,974 | 0,923 | 0,836 | 1 |
| RSAD2, IFIT1, IFI44L, SIGLEC1, ISG15, OLAH, FAM20A, MMP8 | 0,872 | 0,862 | 0,882 | 0,907 | 0,841 | 0,974 | 0,954 | 0,903 | 1 |

| | | | | | | | | |
|---|---|---|---|---|---|---|---|---|
| OAS1, IFIT1, IFI44L, SIGLEC1, SLC1A2, IL1R2, OLAH, FAM20A | 0,872 | 0,863 | 0,882 | 0,921 | 0,868 | 0,974 | 0,946 | 0,885 | 1 |
| IFI27, OAS1, IFIT1, IFI44L, SIGLEC1, HERC6, SLC1A2, IL1R2 | 0,872 | 0,863 | 0,881 | 0,925 | 0,880 | 0,970 | 0,949 | 0,901 | 0,997 |
| RSAD2, IFI27, OAS1, IFI44L, SLC1A2, IL1R2, OLAH, RETN | 0,872 | 0,863 | 0,881 | 0,926 | 0,874 | 0,979 | 0,936 | 0,866 | 1 |
| RSAD2, IFI27, IFIT1, IFI44L, ISG15, SLC1A2, IL1R2, OLAH | 0,872 | 0,863 | 0,881 | 0,928 | 0,878 | 0,977 | 0,929 | 0,864 | 0,995 |
| IFI27, OAS1, IFIT1, SIGLEC1, ISG15, HERC6, SLC1A2, IL1R2 | 0,872 | 0,863 | 0,881 | 0,926 | 0,882 | 0,971 | 0,951 | 0,902 | 1 |
| RSAD2, IFI27, OAS1, IFI44L, HERC6, SLC1A2, FAM20A, RETN | 0,872 | 0,862 | 0,882 | 0,924 | 0,868 | 0,980 | 0,935 | 0,853 | 1 |
| IFI27, OAS1, SIGLEC1, ISG15, HERC6, SLC1A2, IL1R2, MMP8 | 0,872 | 0,863 | 0,881 | 0,926 | 0,882 | 0,971 | 0,948 | 0,897 | 0,999 |
| RSAD2, OAS1, SIGLEC1, ISG15, SLC1A2, OLAH, FAM20A, MMP8 | 0,872 | 0,862 | 0,882 | 0,919 | 0,866 | 0,973 | 0,951 | 0,889 | 1 |
| RSAD2, IFIT1, SIGLEC1, ISG15, IL1R2, OLAH, FAM20A, RETN | 0,872 | 0,863 | 0,881 | 0,912 | 0,850 | 0,974 | 0,964 | 0,916 | 1 |
| RSAD2, IFIT1, SIGLEC1, HERC6, SLC1A2, OLAH, FAM20A, RETN | 0,872 | 0,862 | 0,882 | 0,920 | 0,867 | 0,974 | 0,938 | 0,865 | 1 |
| RSAD2, IFI27, OAS1, IFI44L, SIGLEC1, HERC6, SLC1A2, FAM20A | 0,872 | 0,862 | 0,882 | 0,921 | 0,867 | 0,975 | 0,941 | 0,866 | 1 |
| RSAD2, IFI27, OAS1, SIGLEC1, ISG15, HERC6, SLC1A2, IL1R2 | 0,872 | 0,863 | 0,881 | 0,926 | 0,881 | 0,971 | 0,953 | 0,905 | 1 |
| OAS1, IFIT1, SIGLEC1, SLC1A2, IL1R2, OLAH, FAM20A, RETN | 0,872 | 0,862 | 0,881 | 0,920 | 0,867 | 0,973 | 0,954 | 0,895 | 1 |
| IFIT1, SIGLEC1, ISG15, SLC1A2, OLAH, FAM20A, RETN, MMP8 | 0,872 | 0,862 | 0,882 | 0,920 | 0,867 | 0,974 | 0,950 | 0,890 | 1 |
| RSAD2, IFI27, IFIT1, ISG15, HERC6, SLC1A2, FAM20A, RETN | 0,872 | 0,862 | 0,882 | 0,922 | 0,867 | 0,977 | 0,934 | 0,852 | 1 |
| RSAD2, IFI27, OAS1, IFIT1, SIGLEC1, SLC1A2, FAM20A, MMP8 | 0,872 | 0,862 | 0,882 | 0,921 | 0,864 | 0,978 | 0,942 | 0,870 | 1 |
| RSAD2, IFI44L, SIGLEC1, ISG15, IL1R2, FAM20A, RETN, MMP8 | 0,872 | 0,862 | 0,882 | 0,918 | 0,860 | 0,976 | 0,963 | 0,908 | 1 |
| RSAD2, IFI27, OAS1, IFIT1, ISG15, HERC6, IL1R2, MMP8 | 0,872 | 0,863 | 0,880 | 0,918 | 0,870 | 0,967 | 0,941 | 0,888 | 0,994 |
| OAS1, IFIT1, SIGLEC1, SLC1A2, OLAH, FAM20A, RETN, MMP8 | 0,872 | 0,862 | 0,882 | 0,923 | 0,870 | 0,976 | 0,953 | 0,896 | 1 |
| RSAD2, OAS1, IFIT1, IFI44L, SIGLEC1, SLC1A2, OLAH, FAM20A | 0,872 | 0,862 | 0,881 | 0,920 | 0,865 | 0,975 | 0,939 | 0,876 | 1 |
| RSAD2, IFI27, OAS1, IFI44L, SIGLEC1, ISG15, FAM20A, MMP8 | 0,872 | 0,861 | 0,883 | 0,915 | 0,851 | 0,978 | 0,943 | 0,871 | 1 |
| IFI27, OAS1, IFI44L, ISG15, SLC1A2, IL1R2, OLAH, RETN | 0,872 | 0,862 | 0,881 | 0,929 | 0,878 | 0,979 | 0,932 | 0,863 | 1 |
| RSAD2, IFIT1, SIGLEC1, HERC6, SLC1A2, IL1R2, FAM20A, RETN | 0,872 | 0,862 | 0,881 | 0,925 | 0,876 | 0,974 | 0,946 | 0,874 | 1 |
| RSAD2, OAS1, IFI44L, SIGLEC1, ISG15, HERC6, IL1R2, FAM20A | 0,872 | 0,862 | 0,881 | 0,915 | 0,856 | 0,974 | 0,958 | 0,904 | 1 |
| RSAD2, OAS1, IFIT1, SIGLEC1, OLAH, FAM20A, RETN, MMP8 | 0,872 | 0,862 | 0,881 | 0,914 | 0,851 | 0,977 | 0,961 | 0,913 | 1 |
| RSAD2, IFI27, OAS1, IFIT1, IFI44L, ISG15, HERC6, IL1R2 | 0,872 | 0,863 | 0,880 | 0,919 | 0,870 | 0,969 | 0,937 | 0,881 | 0,993 |
| RSAD2, SIGLEC1, ISG15, HERC6, SLC1A2, OLAH, FAM20A, MMP8 | 0,872 | 0,862 | 0,882 | 0,921 | 0,869 | 0,972 | 0,946 | 0,877 | 1 |
| RSAD2, IFIT1, SIGLEC1, HERC6, SLC1A2, FAM20A, RETN, MMP8 | 0,872 | 0,862 | 0,881 | 0,920 | 0,866 | 0,975 | 0,942 | 0,870 | 1 |

| | | | | | | | | |
|---|---|---|---|---|---|---|---|---|
| IFI27, IFIT1, IFI44L, HERC6, SLC1A2, FAM20A, RETN, MMP8 | 0,872 | 0,861 | 0,882 | 0,922 | 0,864 | 0,979 | 0,935 | 0,852 | 1 |
| OAS1, SIGLEC1, ISG15, HERC6, SLC1A2, OLAH, FAM20A, MMP8 | 0,872 | 0,861 | 0,882 | 0,919 | 0,866 | 0,971 | 0,942 | 0,874 | 1 |
| RSAD2, IFIT1, IFI44L, SIGLEC1, ISG15, SLC1A2, OLAH, FAM20A | 0,872 | 0,862 | 0,881 | 0,920 | 0,866 | 0,975 | 0,939 | 0,876 | 1 |
| RSAD2, OAS1, IFI44L, SIGLEC1, SLC1A2, OLAH, FAM20A, MMP8 | 0,872 | 0,861 | 0,882 | 0,918 | 0,863 | 0,973 | 0,947 | 0,883 | 1 |
| RSAD2, OAS1, IFI44L, SIGLEC1, HERC6, IL1R2, OLAH, FAM20A | 0,872 | 0,862 | 0,881 | 0,914 | 0,856 | 0,973 | 0,958 | 0,904 | 1 |
| IFIT1, IFI44L, SIGLEC1, ISG15, HERC6, SLC1A2, FAM20A, MMP8 | 0,872 | 0,862 | 0,881 | 0,920 | 0,867 | 0,973 | 0,934 | 0,856 | 1 |
| IFI27, OAS1, IFI44L, SIGLEC1, HERC6, SLC1A2, FAM20A, MMP8 | 0,872 | 0,862 | 0,881 | 0,922 | 0,869 | 0,975 | 0,942 | 0,866 | 1 |
| OAS1, SIGLEC1, HERC6, SLC1A2, OLAH, FAM20A, RETN, MMP8 | 0,872 | 0,861 | 0,882 | 0,918 | 0,862 | 0,973 | 0,947 | 0,882 | 1 |
| IFI27, OAS1, IFI44L, ISG15, HERC6, SLC1A2, IL1R2, RETN | 0,872 | 0,863 | 0,880 | 0,924 | 0,874 | 0,973 | 0,936 | 0,869 | 1 |
| RSAD2, SIGLEC1, HERC6, SLC1A2, OLAH, FAM20A, RETN, MMP8 | 0,872 | 0,861 | 0,882 | 0,920 | 0,867 | 0,974 | 0,945 | 0,874 | 1 |
| IFI27, OAS1, IFIT1, IFI44L, ISG15, IL1R2, RETN, MMP8 | 0,872 | 0,862 | 0,881 | 0,919 | 0,864 | 0,975 | 0,945 | 0,889 | 1 |
| IFI27, OAS1, IFIT1, IFI44L, ISG15, HERC6, SLC1A2, RETN | 0,872 | 0,862 | 0,881 | 0,915 | 0,857 | 0,973 | 0,932 | 0,857 | 1 |
| RSAD2, IFI27, OAS1, IFIT1, HERC6, SLC1A2, FAM20A, RETN | 0,872 | 0,862 | 0,881 | 0,923 | 0,867 | 0,979 | 0,933 | 0,849 | 1 |
| RSAD2, OAS1, IFIT1, SIGLEC1, ISG15, IL1R2, FAM20A, RETN | 0,872 | 0,862 | 0,881 | 0,911 | 0,847 | 0,974 | 0,962 | 0,910 | 1 |
| RSAD2, IFI27, SIGLEC1, ISG15, HERC6, SLC1A2, FAM20A, MMP8 | 0,871 | 0,861 | 0,882 | 0,921 | 0,867 | 0,976 | 0,939 | 0,863 | 1 |
| OAS1, IFIT1, IFI44L, SIGLEC1, SLC1A2, OLAH, FAM20A, RETN | 0,871 | 0,862 | 0,881 | 0,920 | 0,864 | 0,975 | 0,937 | 0,874 | 1 |
| IFI27, OAS1, IFI44L, HERC6, SLC1A2, FAM20A, RETN, MMP8 | 0,871 | 0,861 | 0,882 | 0,923 | 0,866 | 0,981 | 0,937 | 0,856 | 1 |
| OAS1, IFI44L, SIGLEC1, ISG15, HERC6, IL1R2, OLAH, FAM20A | 0,871 | 0,862 | 0,881 | 0,914 | 0,856 | 0,973 | 0,959 | 0,905 | 1 |
| OAS1, IFIT1, IFI44L, SIGLEC1, HERC6, SLC1A2, FAM20A, MMP8 | 0,871 | 0,862 | 0,881 | 0,920 | 0,867 | 0,972 | 0,934 | 0,857 | 1 |
| RSAD2, IFI27, OAS1, SIGLEC1, ISG15, HERC6, SLC1A2, FAM20A | 0,871 | 0,861 | 0,882 | 0,920 | 0,866 | 0,974 | 0,940 | 0,864 | 1 |
| RSAD2, OAS1, IFIT1, SIGLEC1, SLC1A2, IL1R2, OLAH, FAM20A | 0,871 | 0,862 | 0,881 | 0,919 | 0,865 | 0,973 | 0,955 | 0,897 | 1 |
| RSAD2, IFI27, OAS1, IFIT1, ISG15, HERC6, SLC1A2, RETN | 0,871 | 0,862 | 0,881 | 0,915 | 0,856 | 0,975 | 0,928 | 0,850 | 1 |
| RSAD2, IFI27, OAS1, ISG15, SLC1A2, IL1R2, FAM20A, RETN | 0,871 | 0,862 | 0,881 | 0,926 | 0,877 | 0,975 | 0,921 | 0,833 | 1 |
| RSAD2, IFI27, OAS1, IFI44L, SLC1A2, IL1R2, FAM20A, RETN | 0,871 | 0,862 | 0,881 | 0,924 | 0,873 | 0,976 | 0,924 | 0,838 | 1 |
| RSAD2, IFI27, SIGLEC1, ISG15, SLC1A2, IL1R2, RETN, MMP8 | 0,871 | 0,863 | 0,880 | 0,927 | 0,880 | 0,974 | 0,963 | 0,920 | 1 |
| IFI27, OAS1, IFIT1, IFI44L, ISG15, SLC1A2, IL1R2, OLAH | 0,871 | 0,862 | 0,881 | 0,927 | 0,878 | 0,977 | 0,929 | 0,863 | 0,996 |
| IFI27, OAS1, IFIT1, IFI44L, HERC6, SLC1A2, IL1R2, RETN | 0,871 | 0,862 | 0,880 | 0,924 | 0,874 | 0,973 | 0,938 | 0,873 | 1 |
| IFI27, OAS1, IFI44L, SIGLEC1, HERC6, SLC1A2, IL1R2, MMP8 | 0,871 | 0,862 | 0,880 | 0,926 | 0,882 | 0,970 | 0,948 | 0,899 | 0,997 |

| | | | | | | | | |
|---|---|---|---|---|---|---|---|---|
| RSAD2, IFIT1, IFI44L, SIGLEC1, SLC1A2, IL1R2, OLAH, FAM20A | 0,871 | 0,862 | 0,881 | 0,922 | 0,869 | 0,974 | 0,950 | 0,892 | 1 |
| RSAD2, IFI27, OAS1, IFI44L, ISG15, HERC6, IL1R2, MMP8 | 0,871 | 0,863 | 0,880 | 0,922 | 0,875 | 0,969 | 0,932 | 0,873 | 0,990 |
| RSAD2, IFI44L, SIGLEC1, SLC1A2, IL1R2, FAM20A, RETN, MMP8 | 0,871 | 0,861 | 0,881 | 0,924 | 0,874 | 0,973 | 0,958 | 0,894 | 1 |
| OAS1, IFI44L, SIGLEC1, SLC1A2, IL1R2, FAM20A, RETN, MMP8 | 0,871 | 0,861 | 0,881 | 0,923 | 0,873 | 0,973 | 0,958 | 0,894 | 1 |
| OAS1, IFI44L, SIGLEC1, ISG15, SLC1A2, OLAH, FAM20A, MMP8 | 0,871 | 0,861 | 0,881 | 0,917 | 0,861 | 0,972 | 0,946 | 0,882 | 1 |
| IFI27, OAS1, IFI44L, HERC6, SLC1A2, IL1R2, RETN, MMP8 | 0,871 | 0,862 | 0,880 | 0,924 | 0,875 | 0,974 | 0,935 | 0,867 | 1 |
| IFI27, IFIT1, IFI44L, SIGLEC1, ISG15, SLC1A2, IL1R2, MMP8 | 0,871 | 0,863 | 0,879 | 0,925 | 0,880 | 0,970 | 0,947 | 0,897 | 0,997 |
| RSAD2, IFIT1, IFI44L, SIGLEC1, IL1R2, OLAH, FAM20A, RETN | 0,871 | 0,862 | 0,881 | 0,912 | 0,848 | 0,975 | 0,964 | 0,916 | 1 |
| IFI44L, SIGLEC1, ISG15, HERC6, SLC1A2, OLAH, FAM20A, MMP8 | 0,871 | 0,861 | 0,881 | 0,919 | 0,865 | 0,973 | 0,946 | 0,873 | 1 |
| IFI27, OAS1, SIGLEC1, ISG15, HERC6, SLC1A2, FAM20A, MMP8 | 0,871 | 0,861 | 0,882 | 0,920 | 0,866 | 0,974 | 0,941 | 0,865 | 1 |
| RSAD2, OAS1, IFIT1, IFI44L, SIGLEC1, HERC6, SLC1A2, FAM20A | 0,871 | 0,862 | 0,880 | 0,921 | 0,869 | 0,973 | 0,933 | 0,856 | 1 |
| RSAD2, IFI27, IFI44L, SIGLEC1, HERC6, SLC1A2, FAM20A, MMP8 | 0,871 | 0,861 | 0,881 | 0,921 | 0,867 | 0,976 | 0,940 | 0,864 | 1 |
| RSAD2, IFI27, IFIT1, IFI44L, SIGLEC1, HERC6, SLC1A2, RETN | 0,871 | 0,863 | 0,879 | 0,922 | 0,870 | 0,975 | 0,936 | 0,875 | 0,997 |
| RSAD2, IFI27, OAS1, IFIT1, IFI44L, ISG15, IL1R2, RETN | 0,871 | 0,862 | 0,880 | 0,915 | 0,860 | 0,970 | 0,942 | 0,884 | 1 |
| RSAD2, IFIT1, SIGLEC1, SLC1A2, IL1R2, OLAH, FAM20A, RETN | 0,871 | 0,862 | 0,881 | 0,921 | 0,868 | 0,974 | 0,957 | 0,898 | 1 |
| OAS1, IFIT1, SIGLEC1, ISG15, SLC1A2, IL1R2, OLAH, FAM20A | 0,871 | 0,861 | 0,881 | 0,918 | 0,864 | 0,973 | 0,952 | 0,891 | 1 |
| RSAD2, IFI27, IFI44L, SIGLEC1, ISG15, SLC1A2, IL1R2, RETN | 0,871 | 0,863 | 0,879 | 0,925 | 0,875 | 0,975 | 0,948 | 0,896 | 1 |
| RSAD2, IFI27, IFIT1, IFI44L, ISG15, HERC6, FAM20A, MMP8 | 0,871 | 0,861 | 0,881 | 0,914 | 0,855 | 0,974 | 0,916 | 0,835 | 0,997 |
| IFI44L, SIGLEC1, ISG15, HERC6, IL1R2, OLAH, FAM20A, RETN | 0,871 | 0,861 | 0,881 | 0,911 | 0,850 | 0,972 | 0,958 | 0,899 | 1 |
| RSAD2, IFIT1, IFI44L, SIGLEC1, ISG15, HERC6, FAM20A, MMP8 | 0,871 | 0,861 | 0,880 | 0,911 | 0,850 | 0,972 | 0,935 | 0,866 | 1 |
| RSAD2, OAS1, IFIT1, SIGLEC1, ISG15, HERC6, FAM20A, MMP8 | 0,871 | 0,861 | 0,880 | 0,910 | 0,849 | 0,971 | 0,939 | 0,870 | 1 |
| RSAD2, IFI27, IFI44L, SIGLEC1, ISG15, HERC6, SLC1A2, FAM20A | 0,871 | 0,861 | 0,881 | 0,922 | 0,867 | 0,976 | 0,941 | 0,864 | 1 |
| RSAD2, IFI27, IFIT1, IFI44L, SIGLEC1, SLC1A2, IL1R2, RETN | 0,871 | 0,862 | 0,879 | 0,923 | 0,873 | 0,974 | 0,949 | 0,896 | 1 |
| RSAD2, IFI27, ISG15, HERC6, SLC1A2, FAM20A, RETN, MMP8 | 0,871 | 0,860 | 0,881 | 0,925 | 0,870 | 0,980 | 0,934 | 0,852 | 1 |
| RSAD2, OAS1, SIGLEC1, ISG15, HERC6, FAM20A, RETN, MMP8 | 0,871 | 0,861 | 0,880 | 0,913 | 0,852 | 0,974 | 0,951 | 0,888 | 1 |
| RSAD2, OAS1, IFIT1, IFI44L, SIGLEC1, ISG15, OLAH, FAM20A | 0,871 | 0,861 | 0,880 | 0,905 | 0,836 | 0,975 | 0,947 | 0,891 | 1 |
| RSAD2, IFIT1, IFI44L, SIGLEC1, ISG15, HERC6, SLC1A2, FAM20A | 0,871 | 0,861 | 0,880 | 0,920 | 0,868 | 0,972 | 0,929 | 0,852 | 1 |
| IFI27, SIGLEC1, ISG15, HERC6, SLC1A2, IL1R2, RETN, MMP8 | 0,871 | 0,862 | 0,879 | 0,927 | 0,879 | 0,974 | 0,965 | 0,926 | 1 |

| Genes | | | | | | | | |
|---|---|---|---|---|---|---|---|---|
| RSAD2, IFI44L, SIGLEC1, ISG15, SLC1A2, OLAH, FAM20A, MMP8 | 0,871 | 0,861 | 0,880 | 0,918 | 0,862 | 0,973 | 0,943 | 0,879 | 1 |
| RSAD2, IFI27, IFIT1, SIGLEC1, HERC6, SLC1A2, IL1R2, MMP8 | 0,871 | 0,862 | 0,879 | 0,926 | 0,880 | 0,971 | 0,950 | 0,902 | 0,998 |
| RSAD2, OAS1, SIGLEC1, HERC6, OLAH, FAM20A, RETN, MMP8 | 0,871 | 0,861 | 0,880 | 0,916 | 0,856 | 0,976 | 0,951 | 0,893 | 1 |
| OAS1, IFIT1, IFI44L, SIGLEC1, ISG15, OLAH, FAM20A, RETN | 0,871 | 0,861 | 0,880 | 0,904 | 0,834 | 0,974 | 0,947 | 0,891 | 1 |
| RSAD2, IFIT1, SIGLEC1, ISG15, SLC1A2, IL1R2, OLAH, FAM20A | 0,871 | 0,861 | 0,880 | 0,918 | 0,864 | 0,973 | 0,957 | 0,895 | 1 |
| OAS1, IFIT1, SIGLEC1, ISG15, HERC6, SLC1A2, FAM20A, RETN | 0,871 | 0,861 | 0,880 | 0,920 | 0,868 | 0,972 | 0,938 | 0,865 | 1 |
| IFI27, OAS1, IFI44L, SIGLEC1, ISG15, HERC6, SLC1A2, IL1R2 | 0,870 | 0,862 | 0,879 | 0,926 | 0,882 | 0,970 | 0,949 | 0,901 | 0,997 |
| RSAD2, IFI27, OAS1, IFI44L, ISG15, HERC6, SLC1A2, RETN | 0,870 | 0,861 | 0,880 | 0,917 | 0,858 | 0,976 | 0,930 | 0,853 | 1 |
| RSAD2, IFIT1, IFI44L, SIGLEC1, SLC1A2, OLAH, FAM20A, RETN | 0,870 | 0,861 | 0,880 | 0,920 | 0,864 | 0,976 | 0,938 | 0,875 | 1 |
| IFI27, OAS1, IFIT1, IFI44L, SIGLEC1, SLC1A2, IL1R2, MMP8 | 0,870 | 0,862 | 0,879 | 0,925 | 0,880 | 0,971 | 0,951 | 0,902 | 1,000 |
| RSAD2, IFI27, OAS1, IFIT1, ISG15, IL1R2, RETN, MMP8 | 0,870 | 0,861 | 0,880 | 0,919 | 0,865 | 0,974 | 0,952 | 0,900 | 1 |
| IFIT1, IFI44L, SIGLEC1, ISG15, SLC1A2, IL1R2, FAM20A, RETN | 0,870 | 0,861 | 0,879 | 0,919 | 0,867 | 0,971 | 0,950 | 0,891 | 1 |
| RSAD2, OAS1, IFIT1, IFI44L, SIGLEC1, IL1R2, FAM20A, RETN | 0,870 | 0,861 | 0,880 | 0,911 | 0,847 | 0,974 | 0,963 | 0,911 | 1 |
| RSAD2, IFI44L, SIGLEC1, HERC6, SLC1A2, OLAH, FAM20A, MMP8 | 0,870 | 0,860 | 0,881 | 0,921 | 0,868 | 0,974 | 0,943 | 0,876 | 1 |
| IFI27, OAS1, IFIT1, IFI44L, SIGLEC1, ISG15, SLC1A2, IL1R2 | 0,870 | 0,862 | 0,879 | 0,924 | 0,878 | 0,970 | 0,945 | 0,893 | 0,996 |
| RSAD2, IFI27, IFI44L, ISG15, HERC6, SLC1A2, FAM20A, RETN | 0,870 | 0,860 | 0,881 | 0,923 | 0,868 | 0,978 | 0,936 | 0,856 | 1 |
| OAS1, IFIT1, IFI44L, SIGLEC1, ISG15, SLC1A2, IL1R2, FAM20A | 0,870 | 0,861 | 0,880 | 0,919 | 0,867 | 0,972 | 0,950 | 0,890 | 1 |
| RSAD2, IFI27, IFIT1, SIGLEC1, ISG15, SLC1A2, IL1R2, RETN | 0,870 | 0,862 | 0,879 | 0,926 | 0,878 | 0,974 | 0,953 | 0,903 | 1 |
| OAS1, IFI44L, SIGLEC1, HERC6, SLC1A2, OLAH, FAM20A, MMP8 | 0,870 | 0,860 | 0,881 | 0,918 | 0,864 | 0,971 | 0,945 | 0,879 | 1 |
| IFI44L, SIGLEC1, ISG15, HERC6, SLC1A2, IL1R2, OLAH, FAM20A | 0,870 | 0,860 | 0,880 | 0,918 | 0,864 | 0,972 | 0,950 | 0,877 | 1 |
| IFI27, OAS1, IFIT1, ISG15, HERC6, SLC1A2, RETN, MMP8 | 0,870 | 0,860 | 0,880 | 0,915 | 0,857 | 0,973 | 0,932 | 0,856 | 1 |
| IFI27, OAS1, IFI44L, SIGLEC1, ISG15, HERC6, SLC1A2, FAM20A | 0,870 | 0,861 | 0,880 | 0,919 | 0,865 | 0,974 | 0,943 | 0,868 | 1 |
| RSAD2, OAS1, SIGLEC1, ISG15, HERC6, IL1R2, FAM20A, RETN | 0,870 | 0,860 | 0,880 | 0,913 | 0,854 | 0,972 | 0,958 | 0,902 | 1 |
| IFIT1, SIGLEC1, ISG15, SLC1A2, IL1R2, OLAH, FAM20A, RETN | 0,870 | 0,861 | 0,880 | 0,921 | 0,869 | 0,973 | 0,952 | 0,895 | 1 |
| OAS1, IFIT1, IFI44L, SIGLEC1, ISG15, HERC6, SLC1A2, FAM20A | 0,870 | 0,861 | 0,880 | 0,921 | 0,870 | 0,973 | 0,930 | 0,854 | 1 |
| IFI27, OAS1, IFIT1, IFI44L, ISG15, HERC6, FAM20A, MMP8 | 0,870 | 0,860 | 0,881 | 0,911 | 0,851 | 0,971 | 0,918 | 0,837 | 1 |
| RSAD2, OAS1, IFIT1, IFI44L, SIGLEC1, HERC6, FAM20A, MMP8 | 0,870 | 0,861 | 0,880 | 0,909 | 0,847 | 0,971 | 0,932 | 0,860 | 1 |
| OAS1, IFIT1, IFI44L, SIGLEC1, ISG15, HERC6, FAM20A, MMP8 | 0,870 | 0,860 | 0,880 | 0,912 | 0,852 | 0,972 | 0,939 | 0,872 | 1 |

| | | | | | | | | |
|---|---|---|---|---|---|---|---|---|
| RSAD2, OAS1, IFIT1, SIGLEC1, ISG15, SLC1A2, OLAH, FAM20A | 0,870 | 0,861 | 0,880 | 0,919 | 0,863 | 0,974 | 0,946 | 0,883 | 1 |
| IFI27, OAS1, IFIT1, SIGLEC1, ISG15, SLC1A2, IL1R2, MMP8 | 0,870 | 0,862 | 0,879 | 0,924 | 0,879 | 0,970 | 0,951 | 0,902 | 1,000 |
| RSAD2, IFIT1, SIGLEC1, SLC1A2, OLAH, FAM20A, RETN, MMP8 | 0,870 | 0,860 | 0,880 | 0,922 | 0,869 | 0,975 | 0,953 | 0,896 | 1 |
| RSAD2, IFI27, IFIT1, HERC6, SLC1A2, IL1R2, RETN, MMP8 | 0,870 | 0,861 | 0,879 | 0,923 | 0,873 | 0,973 | 0,943 | 0,885 | 1 |
| RSAD2, IFIT1, IFI44L, SIGLEC1, HERC6, SLC1A2, FAM20A, RETN | 0,870 | 0,861 | 0,879 | 0,924 | 0,874 | 0,975 | 0,932 | 0,855 | 1 |
| IFI27, OAS1, IFIT1, HERC6, SLC1A2, FAM20A, RETN, MMP8 | 0,870 | 0,860 | 0,880 | 0,924 | 0,867 | 0,980 | 0,932 | 0,848 | 1 |
| IFI44L, SIGLEC1, ISG15, SLC1A2, OLAH, FAM20A, RETN, MMP8 | 0,870 | 0,860 | 0,880 | 0,915 | 0,859 | 0,972 | 0,949 | 0,880 | 1 |
| RSAD2, IFI27, OAS1, HERC6, SLC1A2, FAM20A, RETN, MMP8 | 0,870 | 0,860 | 0,880 | 0,923 | 0,867 | 0,980 | 0,932 | 0,848 | 1 |
| IFI44L, SIGLEC1, ISG15, SLC1A2, IL1R2, OLAH, FAM20A, RETN | 0,870 | 0,860 | 0,879 | 0,917 | 0,864 | 0,969 | 0,954 | 0,891 | 1 |
| OAS1, SIGLEC1, ISG15, HERC6, SLC1A2, IL1R2, OLAH, FAM20A | 0,870 | 0,859 | 0,880 | 0,920 | 0,869 | 0,971 | 0,946 | 0,878 | 1 |
| IFI27, IFI44L, SIGLEC1, ISG15, HERC6, SLC1A2, IL1R2, MMP8 | 0,870 | 0,862 | 0,878 | 0,926 | 0,881 | 0,970 | 0,943 | 0,892 | 0,995 |
| SIGLEC1, ISG15, HERC6, SLC1A2, OLAH, FAM20A, RETN, MMP8 | 0,870 | 0,859 | 0,880 | 0,918 | 0,862 | 0,974 | 0,948 | 0,875 | 1 |
| IFI27, IFIT1, IFI44L, SIGLEC1, HERC6, SLC1A2, IL1R2, MMP8 | 0,870 | 0,861 | 0,879 | 0,926 | 0,881 | 0,971 | 0,948 | 0,900 | 0,996 |
| RSAD2, SIGLEC1, ISG15, HERC6, OLAH, FAM20A, RETN, MMP8 | 0,870 | 0,860 | 0,880 | 0,916 | 0,856 | 0,975 | 0,952 | 0,895 | 1 |
| RSAD2, OAS1, IFIT1, SIGLEC1, HERC6, SLC1A2, FAM20A, RETN | 0,870 | 0,860 | 0,879 | 0,921 | 0,869 | 0,973 | 0,938 | 0,866 | 1 |
| RSAD2, IFI27, IFI44L, SIGLEC1, HERC6, SLC1A2, IL1R2, MMP8 | 0,870 | 0,861 | 0,878 | 0,926 | 0,880 | 0,972 | 0,948 | 0,900 | 0,996 |
| IFI27, OAS1, IFIT1, IFI44L, ISG15, HERC6, IL1R2, MMP8 | 0,870 | 0,861 | 0,879 | 0,920 | 0,872 | 0,969 | 0,936 | 0,879 | 0,992 |
| OAS1, IFIT1, IFI44L, SIGLEC1, SLC1A2, IL1R2, FAM20A, RETN | 0,870 | 0,860 | 0,879 | 0,919 | 0,867 | 0,971 | 0,952 | 0,893 | 1 |
| RSAD2, IFIT1, IFI44L, SIGLEC1, HERC6, SLC1A2, FAM20A, MMP8 | 0,870 | 0,860 | 0,879 | 0,921 | 0,870 | 0,973 | 0,933 | 0,856 | 1 |
| RSAD2, IFI27, IFIT1, IFI44L, ISG15, IL1R2, RETN, MMP8 | 0,870 | 0,860 | 0,879 | 0,918 | 0,863 | 0,974 | 0,943 | 0,887 | 1,000 |
| RSAD2, IFI27, OAS1, IFIT1, SIGLEC1, ISG15, SLC1A2, IL1R2 | 0,870 | 0,861 | 0,878 | 0,925 | 0,880 | 0,971 | 0,954 | 0,905 | 1 |
| IFI27, OAS1, IFI44L, ISG15, HERC6, SLC1A2, RETN, MMP8 | 0,870 | 0,860 | 0,879 | 0,915 | 0,857 | 0,972 | 0,933 | 0,859 | 1 |
| RSAD2, IFI27, OAS1, IFIT1, IFI44L, SIGLEC1, ISG15, RETN | 0,870 | 0,861 | 0,879 | 0,908 | 0,845 | 0,971 | 0,953 | 0,891 | 1 |
| RSAD2, IFI27, OAS1, IFIT1, SIGLEC1, ISG15, IL1R2, MMP8 | 0,870 | 0,861 | 0,878 | 0,921 | 0,873 | 0,969 | 0,957 | 0,913 | 1 |
| RSAD2, OAS1, SIGLEC1, HERC6, IL1R2, OLAH, FAM20A, RETN | 0,870 | 0,860 | 0,880 | 0,912 | 0,853 | 0,972 | 0,958 | 0,902 | 1 |
| RSAD2, IFIT1, SIGLEC1, ISG15, HERC6, SLC1A2, FAM20A, RETN | 0,870 | 0,860 | 0,879 | 0,919 | 0,866 | 0,972 | 0,939 | 0,865 | 1 |
| RSAD2, IFI27, OAS1, IFIT1, IFI44L, HERC6, SLC1A2, RETN | 0,870 | 0,860 | 0,879 | 0,915 | 0,855 | 0,975 | 0,929 | 0,852 | 1 |
| IFI27, IFIT1, IFI44L, SIGLEC1, ISG15, HERC6, SLC1A2, RETN | 0,870 | 0,861 | 0,878 | 0,918 | 0,863 | 0,973 | 0,947 | 0,893 | 1 |

| | | | | | | | | | |
|---|---|---|---|---|---|---|---|---|---|
| OAS1, SIGLEC1, ISG15, HERC6, OLAH, FAM20A, RETN, MMP8 | 0,870 | 0,860 | 0,880 | 0,913 | 0,852 | 0,974 | 0,961 | 0,911 | 1 |
| RSAD2, IFI27, IFIT1, IFI44L, ISG15, HERC6, IL1R2, MMP8 | 0,870 | 0,861 | 0,878 | 0,919 | 0,868 | 0,969 | 0,935 | 0,878 | 0,992 |
| OAS1, IFIT1, SIGLEC1, ISG15, SLC1A2, OLAH, FAM20A, RETN | 0,870 | 0,860 | 0,880 | 0,919 | 0,864 | 0,974 | 0,945 | 0,882 | 1 |
| RSAD2, IFI27, IFIT1, SIGLEC1, ISG15, HERC6, SLC1A2, RETN | 0,870 | 0,861 | 0,878 | 0,922 | 0,869 | 0,974 | 0,939 | 0,881 | 0,997 |
| RSAD2, IFI27, IFI44L, SIGLEC1, ISG15, SLC1A2, IL1R2, MMP8 | 0,870 | 0,862 | 0,878 | 0,925 | 0,879 | 0,971 | 0,950 | 0,903 | 0,997 |
| IFI27, IFIT1, IFI44L, SIGLEC1, ISG15, HERC6, SLC1A2, IL1R2 | 0,870 | 0,861 | 0,878 | 0,924 | 0,878 | 0,970 | 0,947 | 0,897 | 0,997 |
| RSAD2, IFI27, OAS1, IFIT1, IFI44L, SIGLEC1, SLC1A2, IL1R2 | 0,870 | 0,861 | 0,878 | 0,926 | 0,880 | 0,972 | 0,950 | 0,898 | 1 |
| RSAD2, SIGLEC1, ISG15, HERC6, SLC1A2, IL1R2, OLAH, FAM20A | 0,870 | 0,859 | 0,880 | 0,919 | 0,867 | 0,971 | 0,946 | 0,873 | 1 |
| RSAD2, IFI27, OAS1, IFI44L, SIGLEC1, ISG15, SLC1A2, IL1R2 | 0,870 | 0,861 | 0,878 | 0,927 | 0,881 | 0,973 | 0,955 | 0,910 | 1 |
| IFI27, IFIT1, ISG15, HERC6, SLC1A2, FAM20A, RETN, MMP8 | 0,870 | 0,859 | 0,880 | 0,920 | 0,864 | 0,977 | 0,937 | 0,856 | 1 |
| RSAD2, OAS1, SIGLEC1, ISG15, HERC6, SLC1A2, IL1R2, FAM20A | 0,869 | 0,859 | 0,880 | 0,919 | 0,868 | 0,969 | 0,951 | 0,881 | 1 |
| RSAD2, IFI27, SIGLEC1, ISG15, HERC6, SLC1A2, IL1R2, MMP8 | 0,869 | 0,861 | 0,878 | 0,923 | 0,878 | 0,969 | 0,948 | 0,898 | 0,997 |
| RSAD2, IFI27, IFIT1, IFI44L, SIGLEC1, HERC6, SLC1A2, IL1R2 | 0,869 | 0,861 | 0,878 | 0,926 | 0,880 | 0,971 | 0,948 | 0,900 | 0,996 |
| RSAD2, IFIT1, IFI44L, SIGLEC1, ISG15, SLC1A2, IL1R2, FAM20A | 0,869 | 0,860 | 0,878 | 0,920 | 0,868 | 0,973 | 0,955 | 0,897 | 1 |
| RSAD2, IFI27, IFIT1, SIGLEC1, HERC6, SLC1A2, RETN, MMP8 | 0,869 | 0,861 | 0,878 | 0,921 | 0,868 | 0,974 | 0,942 | 0,887 | 0,998 |
| RSAD2, OAS1, SIGLEC1, HERC6, SLC1A2, IL1R2, OLAH, FAM20A | 0,869 | 0,859 | 0,880 | 0,919 | 0,868 | 0,971 | 0,946 | 0,878 | 1 |
| RSAD2, IFI27, IFIT1, SIGLEC1, ISG15, HERC6, SLC1A2, IL1R2 | 0,869 | 0,861 | 0,878 | 0,925 | 0,880 | 0,970 | 0,949 | 0,900 | 0,998 |
| IFI27, IFIT1, SIGLEC1, ISG15, HERC6, SLC1A2, IL1R2, MMP8 | 0,869 | 0,861 | 0,878 | 0,925 | 0,880 | 0,970 | 0,950 | 0,902 | 0,998 |
| OAS1, IFI44L, SIGLEC1, ISG15, HERC6, SLC1A2, IL1R2, FAM20A | 0,869 | 0,859 | 0,879 | 0,920 | 0,869 | 0,970 | 0,949 | 0,878 | 1 |
| IFI27, IFI44L, ISG15, HERC6, SLC1A2, IL1R2, RETN, MMP8 | 0,869 | 0,861 | 0,878 | 0,924 | 0,874 | 0,974 | 0,935 | 0,868 | 1 |
| RSAD2, IFI27, IFI44L, SIGLEC1, ISG15, HERC6, SLC1A2, IL1R2 | 0,869 | 0,861 | 0,878 | 0,925 | 0,879 | 0,971 | 0,950 | 0,903 | 0,997 |
| RSAD2, IFI27, IFIT1, IFI44L, ISG15, HERC6, RETN, MMP8 | 0,869 | 0,860 | 0,879 | 0,915 | 0,856 | 0,973 | 0,938 | 0,879 | 0,997 |
| RSAD2, IFI27, OAS1, IFI44L, SIGLEC1, SLC1A2, IL1R2, MMP8 | 0,869 | 0,861 | 0,878 | 0,926 | 0,879 | 0,972 | 0,952 | 0,902 | 1 |
| RSAD2, IFIT1, IFI44L, SIGLEC1, ISG15, IL1R2, FAM20A, RETN | 0,869 | 0,860 | 0,879 | 0,911 | 0,848 | 0,974 | 0,963 | 0,913 | 1 |
| RSAD2, OAS1, SIGLEC1, ISG15, HERC6, OLAH, FAM20A, MMP8 | 0,869 | 0,859 | 0,880 | 0,910 | 0,849 | 0,971 | 0,953 | 0,898 | 1 |
| RSAD2, OAS1, IFIT1, SIGLEC1, ISG15, SLC1A2, IL1R2, FAM20A | 0,869 | 0,860 | 0,879 | 0,918 | 0,865 | 0,972 | 0,957 | 0,895 | 1 |
| IFI27, IFI44L, SIGLEC1, ISG15, HERC6, SLC1A2, FAM20A, MMP8 | 0,869 | 0,859 | 0,879 | 0,916 | 0,860 | 0,973 | 0,942 | 0,865 | 1 |
| RSAD2, OAS1, IFIT1, SIGLEC1, ISG15, HERC6, SLC1A2, FAM20A | 0,869 | 0,860 | 0,879 | 0,918 | 0,864 | 0,971 | 0,937 | 0,860 | 1 |

| | | | | | | | | |
|---|---|---|---|---|---|---|---|---|
| IFI27, IFIT1, IFI44L, HERC6, SLC1A2, IL1R2, RETN, MMP8 | 0,869 | 0,860 | 0,878 | 0,923 | 0,872 | 0,974 | 0,940 | 0,879 | 1 |
| RSAD2, OAS1, IFIT1, SIGLEC1, SLC1A2, OLAH, FAM20A, RETN | 0,869 | 0,859 | 0,879 | 0,919 | 0,863 | 0,975 | 0,951 | 0,893 | 1 |
| RSAD2, IFI27, ISG15, HERC6, SLC1A2, IL1R2, RETN, MMP8 | 0,869 | 0,860 | 0,878 | 0,925 | 0,876 | 0,974 | 0,936 | 0,870 | 1 |
| RSAD2, OAS1, IFIT1, IFI44L, SIGLEC1, SLC1A2, IL1R2, FAM20A | 0,869 | 0,860 | 0,879 | 0,920 | 0,868 | 0,973 | 0,952 | 0,892 | 1 |
| OAS1, SIGLEC1, ISG15, SLC1A2, OLAH, FAM20A, RETN, MMP8 | 0,869 | 0,859 | 0,879 | 0,915 | 0,858 | 0,971 | 0,950 | 0,888 | 1 |
| RSAD2, IFI27, OAS1, ISG15, HERC6, SLC1A2, RETN, MMP8 | 0,869 | 0,859 | 0,879 | 0,917 | 0,859 | 0,976 | 0,927 | 0,850 | 1 |
| RSAD2, SIGLEC1, ISG15, HERC6, IL1R2, OLAH, FAM20A, RETN | 0,869 | 0,859 | 0,879 | 0,913 | 0,853 | 0,972 | 0,957 | 0,899 | 1 |
| RSAD2, IFI27, IFI44L, SIGLEC1, ISG15, HERC6, SLC1A2, RETN | 0,869 | 0,860 | 0,878 | 0,921 | 0,867 | 0,975 | 0,941 | 0,883 | 1,000 |
| IFI27, OAS1, IFI44L, SIGLEC1, ISG15, SLC1A2, IL1R2, MMP8 | 0,869 | 0,861 | 0,877 | 0,924 | 0,878 | 0,970 | 0,945 | 0,893 | 0,996 |
| RSAD2, IFI27, IFI44L, HERC6, SLC1A2, FAM20A, RETN, MMP8 | 0,869 | 0,859 | 0,880 | 0,924 | 0,868 | 0,980 | 0,937 | 0,857 | 1 |
| RSAD2, OAS1, SIGLEC1, SLC1A2, OLAH, FAM20A, RETN, MMP8 | 0,869 | 0,859 | 0,879 | 0,918 | 0,862 | 0,973 | 0,951 | 0,891 | 1 |
| RSAD2, OAS1, IFIT1, IFI44L, SIGLEC1, OLAH, FAM20A, RETN | 0,869 | 0,859 | 0,879 | 0,905 | 0,835 | 0,974 | 0,948 | 0,893 | 1 |
| RSAD2, IFI27, IFIT1, HERC6, SLC1A2, FAM20A, RETN, MMP8 | 0,869 | 0,859 | 0,879 | 0,923 | 0,867 | 0,980 | 0,934 | 0,852 | 1 |
| OAS1, IFIT1, SIGLEC1, ISG15, SLC1A2, IL1R2, FAM20A, RETN | 0,869 | 0,859 | 0,879 | 0,922 | 0,871 | 0,972 | 0,951 | 0,883 | 1 |
| IFI27, IFIT1, IFI44L, SIGLEC1, HERC6, SLC1A2, RETN, MMP8 | 0,869 | 0,860 | 0,878 | 0,920 | 0,866 | 0,974 | 0,942 | 0,886 | 0,999 |
| RSAD2, OAS1, IFIT1, SIGLEC1, HERC6, SLC1A2, FAM20A, MMP8 | 0,869 | 0,859 | 0,878 | 0,916 | 0,861 | 0,971 | 0,937 | 0,860 | 1 |
| RSAD2, IFI27, OAS1, IFIT1, IFI44L, ISG15, HERC6, FAM20A | 0,869 | 0,858 | 0,879 | 0,911 | 0,849 | 0,973 | 0,921 | 0,838 | 1 |
| RSAD2, OAS1, IFI44L, SIGLEC1, ISG15, IL1R2, OLAH, FAM20A | 0,869 | 0,859 | 0,879 | 0,910 | 0,846 | 0,973 | 0,963 | 0,912 | 1 |
| RSAD2, IFIT1, SIGLEC1, ISG15, SLC1A2, IL1R2, FAM20A, RETN | 0,869 | 0,859 | 0,878 | 0,922 | 0,871 | 0,973 | 0,955 | 0,891 | 1 |
| RSAD2, IFI27, OAS1, IFIT1, IFI44L, HERC6, FAM20A, MMP8 | 0,869 | 0,859 | 0,879 | 0,912 | 0,853 | 0,972 | 0,920 | 0,837 | 1 |
| RSAD2, IFI27, OAS1, IFI44L, SIGLEC1, ISG15, SLC1A2, FAM20A | 0,869 | 0,858 | 0,879 | 0,916 | 0,858 | 0,975 | 0,946 | 0,872 | 1 |
| IFI27, OAS1, IFIT1, IFI44L, HERC6, SLC1A2, RETN, MMP8 | 0,869 | 0,859 | 0,878 | 0,914 | 0,855 | 0,972 | 0,932 | 0,859 | 1 |
| RSAD2, IFIT1, SIGLEC1, ISG15, OLAH, FAM20A, RETN, MMP8 | 0,869 | 0,859 | 0,878 | 0,912 | 0,847 | 0,976 | 0,959 | 0,911 | 1 |
| RSAD2, IFIT1, IFI44L, SIGLEC1, OLAH, FAM20A, RETN, MMP8 | 0,869 | 0,859 | 0,879 | 0,915 | 0,853 | 0,978 | 0,959 | 0,911 | 1 |
| RSAD2, IFI27, OAS1, IFIT1, SIGLEC1, SLC1A2, IL1R2, MMP8 | 0,869 | 0,860 | 0,877 | 0,924 | 0,877 | 0,971 | 0,953 | 0,903 | 1 |
| OAS1, IFI44L, SIGLEC1, ISG15, HERC6, IL1R2, FAM20A, RETN | 0,869 | 0,859 | 0,879 | 0,914 | 0,855 | 0,973 | 0,961 | 0,908 | 1 |
| RSAD2, IFI44L, SIGLEC1, HERC6, SLC1A2, IL1R2, OLAH, FAM20A | 0,869 | 0,858 | 0,879 | 0,918 | 0,866 | 0,971 | 0,945 | 0,871 | 1 |
| RSAD2, OAS1, IFIT1, SIGLEC1, ISG15, OLAH, FAM20A, RETN | 0,869 | 0,859 | 0,878 | 0,904 | 0,834 | 0,974 | 0,952 | 0,901 | 1 |

| | | | | | | | | | |
|---|---|---|---|---|---|---|---|---|---|
| RSAD2, OAS1, SIGLEC1, ISG15, IL1R2, OLAH, FAM20A, RETN | 0,869 | 0,859 | 0,879 | 0,906 | 0,842 | 0,971 | 0,961 | 0,904 | 1 |
| RSAD2, IFI27, OAS1, SIGLEC1, ISG15, SLC1A2, IL1R2, MMP8 | 0,868 | 0,860 | 0,877 | 0,926 | 0,880 | 0,971 | 0,954 | 0,905 | 1 |
| RSAD2, IFIT1, IFI44L, SIGLEC1, SLC1A2, IL1R2, FAM20A, RETN | 0,868 | 0,859 | 0,878 | 0,920 | 0,869 | 0,972 | 0,954 | 0,895 | 1 |
| RSAD2, IFIT1, SIGLEC1, ISG15, SLC1A2, OLAH, FAM20A, RETN | 0,868 | 0,859 | 0,878 | 0,919 | 0,864 | 0,974 | 0,947 | 0,885 | 1 |
| RSAD2, IFI27, IFIT1, IFI44L, SIGLEC1, SLC1A2, IL1R2, MMP8 | 0,868 | 0,860 | 0,877 | 0,925 | 0,879 | 0,971 | 0,950 | 0,903 | 0,997 |
| IFI44L, SIGLEC1, HERC6, SLC1A2, OLAH, FAM20A, RETN, MMP8 | 0,868 | 0,858 | 0,879 | 0,917 | 0,860 | 0,974 | 0,951 | 0,879 | 1 |
| RSAD2, IFI27, IFIT1, SIGLEC1, ISG15, SLC1A2, IL1R2, MMP8 | 0,868 | 0,860 | 0,877 | 0,924 | 0,878 | 0,969 | 0,950 | 0,901 | 0,999 |
| RSAD2, OAS1, IFI44L, SIGLEC1, HERC6, IL1R2, FAM20A, RETN | 0,868 | 0,859 | 0,878 | 0,913 | 0,854 | 0,973 | 0,959 | 0,904 | 1 |
| RSAD2, IFI27, IFIT1, IFI44L, HERC6, SLC1A2, IL1R2, RETN | 0,868 | 0,860 | 0,877 | 0,923 | 0,872 | 0,973 | 0,933 | 0,867 | 0,998 |
| RSAD2, IFI27, IFIT1, ISG15, HERC6, SLC1A2, IL1R2, RETN | 0,868 | 0,860 | 0,877 | 0,922 | 0,871 | 0,972 | 0,936 | 0,872 | 1,000 |
| IFI44L, SIGLEC1, ISG15, HERC6, OLAH, FAM20A, RETN, MMP8 | 0,868 | 0,858 | 0,879 | 0,911 | 0,846 | 0,975 | 0,961 | 0,907 | 1 |
| OAS1, SIGLEC1, ISG15, HERC6, IL1R2, OLAH, FAM20A, RETN | 0,868 | 0,858 | 0,879 | 0,911 | 0,851 | 0,971 | 0,960 | 0,907 | 1 |
| RSAD2, IFI27, OAS1, IFI44L, HERC6, SLC1A2, RETN, MMP8 | 0,868 | 0,859 | 0,878 | 0,914 | 0,853 | 0,974 | 0,930 | 0,854 | 1 |
| RSAD2, IFI44L, SIGLEC1, ISG15, HERC6, SLC1A2, IL1R2, FAM20 | 0,868 | 0,858 | 0,878 | 0,918 | 0,866 | 0,970 | 0,948 | 0,875 | 1 |
| IFI27, IFI44L, ISG15, HERC6, SLC1A2, FAM20A, RETN, MMP8 | 0,868 | 0,857 | 0,879 | 0,919 | 0,861 | 0,978 | 0,942 | 0,863 | 1 |
| RSAD2, OAS1, IFIT1, SIGLEC1, SLC1A2, IL1R2, FAM20A, RETN | 0,868 | 0,859 | 0,878 | 0,920 | 0,869 | 0,972 | 0,957 | 0,896 | 1 |
| OAS1, IFI44L, SIGLEC1, HERC6, SLC1A2, IL1R2, OLAH, FAM20 | 0,868 | 0,858 | 0,879 | 0,920 | 0,869 | 0,971 | 0,946 | 0,878 | 1 |
| RSAD2, IFI27, OAS1, IFIT1, HERC6, SLC1A2, IL1R2, MMP8 | 0,868 | 0,859 | 0,877 | 0,923 | 0,877 | 0,969 | 0,937 | 0,873 | 1 |
| IFI27, IFIT1, SIGLEC1, ISG15, HERC6, SLC1A2, RETN, MMP8 | 0,868 | 0,859 | 0,877 | 0,919 | 0,865 | 0,974 | 0,940 | 0,880 | 1 |
| RSAD2, IFI27, OAS1, SIGLEC1, ISG15, SLC1A2, FAM20A, MMP8 | 0,868 | 0,857 | 0,879 | 0,916 | 0,858 | 0,975 | 0,948 | 0,877 | 1 |
| RSAD2, SIGLEC1, ISG15, SLC1A2, OLAH, FAM20A, RETN, MMP8 | 0,868 | 0,858 | 0,878 | 0,916 | 0,861 | 0,972 | 0,948 | 0,885 | 1 |
| RSAD2, IFI27, OAS1, IFIT1, IFI44L, ISG15, FAM20A, RETN | 0,868 | 0,858 | 0,878 | 0,910 | 0,846 | 0,974 | 0,945 | 0,869 | 1 |
| RSAD2, IFI27, IFIT1, IFI44L, SIGLEC1, ISG15, SLC1A2, IL1R2 | 0,868 | 0,860 | 0,876 | 0,925 | 0,878 | 0,971 | 0,949 | 0,900 | 0,997 |
| OAS1, IFIT1, IFI44L, SIGLEC1, ISG15, FAM20A, RETN, MMP8 | 0,868 | 0,858 | 0,878 | 0,909 | 0,842 | 0,975 | 0,946 | 0,884 | 1 |
| OAS1, IFI44L, SIGLEC1, HERC6, IL1R2, OLAH, FAM20A, RETN | 0,868 | 0,858 | 0,878 | 0,914 | 0,854 | 0,973 | 0,961 | 0,908 | 1 |
| RSAD2, IFIT1, SIGLEC1, ISG15, HERC6, SLC1A2, FAM20A, MMP8 | 0,868 | 0,858 | 0,878 | 0,917 | 0,863 | 0,971 | 0,937 | 0,860 | 1 |
| RSAD2, IFI27, IFI44L, ISG15, HERC6, SLC1A2, IL1R2, RETN | 0,868 | 0,860 | 0,876 | 0,923 | 0,873 | 0,973 | 0,933 | 0,866 | 0,999 |
| RSAD2, IFI27, OAS1, IFIT1, SLC1A2, IL1R2, RETN, MMP8 | 0,868 | 0,859 | 0,877 | 0,921 | 0,869 | 0,972 | 0,941 | 0,876 | 1 |

| | | | | | | | | |
|---|---|---|---|---|---|---|---|---|
| RSAD2, IFI27, IFI44L, SIGLEC1, ISG15, SLC1A2, FAM20A, MMP8 | 0,868 | 0,857 | 0,878 | 0,918 | 0,860 | 0,976 | 0,947 | 0,874 | 1 |
| RSAD2, IFI27, IFI44L, HERC6, SLC1A2, IL1R2, RETN, MMP8 | 0,868 | 0,859 | 0,877 | 0,923 | 0,874 | 0,973 | 0,935 | 0,868 | 1 |
| RSAD2, IFI27, OAS1, IFI44L, ISG15, HERC6, FAM20A, MMP8 | 0,868 | 0,857 | 0,879 | 0,911 | 0,850 | 0,972 | 0,926 | 0,847 | 1 |
| RSAD2, OAS1, IFI44L, SIGLEC1, HERC6, OLAH, FAM20A, MMP8 | 0,868 | 0,858 | 0,878 | 0,910 | 0,850 | 0,970 | 0,952 | 0,895 | 1 |
| RSAD2, SIGLEC1, HERC6, SLC1A2, IL1R2, OLAH, FAM20A, RETN | 0,868 | 0,857 | 0,878 | 0,919 | 0,868 | 0,971 | 0,947 | 0,877 | 1 |
| RSAD2, OAS1, IFI44L, SIGLEC1, HERC6, SLC1A2, IL1R2, FAM20A | 0,868 | 0,858 | 0,878 | 0,920 | 0,870 | 0,970 | 0,952 | 0,884 | 1 |
| RSAD2, SIGLEC1, ISG15, HERC6, SLC1A2, FAM20A, RETN, MMP8 | 0,868 | 0,858 | 0,878 | 0,913 | 0,855 | 0,971 | 0,950 | 0,880 | 1 |
| RSAD2, IFI27, OAS1, IFI44L, SIGLEC1, SLC1A2, FAM20A, MMP8 | 0,868 | 0,857 | 0,878 | 0,918 | 0,859 | 0,977 | 0,947 | 0,875 | 1 |
| RSAD2, OAS1, SIGLEC1, SLC1A2, IL1R2, OLAH, FAM20A, RETN | 0,868 | 0,858 | 0,878 | 0,917 | 0,863 | 0,970 | 0,958 | 0,900 | 1 |
| OAS1, IFIT1, SIGLEC1, ISG15, HERC6, SLC1A2, FAM20A, MMP8 | 0,868 | 0,858 | 0,878 | 0,918 | 0,864 | 0,971 | 0,936 | 0,859 | 1 |
| IFI27, OAS1, IFIT1, IFI44L, SLC1A2, IL1R2, RETN, MMP8 | 0,868 | 0,858 | 0,877 | 0,921 | 0,869 | 0,972 | 0,939 | 0,876 | 1 |
| IFI27, IFIT1, ISG15, HERC6, SLC1A2, IL1R2, RETN, MMP8 | 0,868 | 0,859 | 0,877 | 0,925 | 0,875 | 0,975 | 0,945 | 0,887 | 1 |
| OAS1, IFI44L, SIGLEC1, ISG15, IL1R2, OLAH, FAM20A, RETN | 0,868 | 0,858 | 0,878 | 0,906 | 0,842 | 0,970 | 0,962 | 0,907 | 1 |
| RSAD2, SIGLEC1, ISG15, SLC1A2, IL1R2, OLAH, FAM20A, RETN | 0,868 | 0,858 | 0,878 | 0,917 | 0,864 | 0,969 | 0,954 | 0,891 | 1 |
| RSAD2, IFI27, OAS1, IFI44L, ISG15, IL1R2, RETN, MMP8 | 0,868 | 0,858 | 0,877 | 0,918 | 0,864 | 0,973 | 0,942 | 0,883 | 1 |
| RSAD2, IFI27, OAS1, IFIT1, HERC6, SLC1A2, RETN, MMP8 | 0,868 | 0,858 | 0,877 | 0,914 | 0,853 | 0,974 | 0,927 | 0,848 | 1 |
| IFI27, IFIT1, IFI44L, ISG15, HERC6, SLC1A2, IL1R2, RETN | 0,867 | 0,859 | 0,876 | 0,924 | 0,874 | 0,974 | 0,933 | 0,866 | 0,999 |
| IFI27, OAS1, IFIT1, ISG15, SLC1A2, IL1R2, RETN, MMP8 | 0,867 | 0,858 | 0,877 | 0,921 | 0,870 | 0,972 | 0,945 | 0,884 | 1 |
| OAS1, IFI44L, SIGLEC1, SLC1A2, OLAH, FAM20A, RETN, MMP8 | 0,867 | 0,857 | 0,878 | 0,914 | 0,856 | 0,971 | 0,950 | 0,892 | 1 |
| RSAD2, IFI44L, SIGLEC1, HERC6, IL1R2, OLAH, FAM20A, RETN | 0,867 | 0,857 | 0,877 | 0,914 | 0,855 | 0,973 | 0,957 | 0,899 | 1 |
| RSAD2, IFI27, SIGLEC1, ISG15, HERC6, SLC1A2, RETN, MMP8 | 0,867 | 0,858 | 0,877 | 0,921 | 0,867 | 0,975 | 0,948 | 0,894 | 1 |
| OAS1, IFI44L, SIGLEC1, ISG15, HERC6, SLC1A2, OLAH, FAM20A | 0,867 | 0,857 | 0,878 | 0,916 | 0,861 | 0,971 | 0,941 | 0,872 | 1 |
| OAS1, SIGLEC1, ISG15, HERC6, SLC1A2, IL1R2, FAM20A, RETN | 0,867 | 0,857 | 0,878 | 0,920 | 0,869 | 0,970 | 0,950 | 0,881 | 1 |
| IFI27, OAS1, IFI44L, SIGLEC1, ISG15, SLC1A2, FAM20A, MMP8 | 0,867 | 0,857 | 0,878 | 0,916 | 0,858 | 0,975 | 0,946 | 0,872 | 1 |
| RSAD2, OAS1, SIGLEC1, ISG15, SLC1A2, IL1R2, OLAH, FAM20A | 0,867 | 0,857 | 0,877 | 0,916 | 0,861 | 0,970 | 0,955 | 0,894 | 1 |
| RSAD2, IFIT1, IFI44L, SIGLEC1, ISG15, OLAH, FAM20A, RETN | 0,867 | 0,858 | 0,877 | 0,904 | 0,835 | 0,974 | 0,947 | 0,892 | 1 |
| RSAD2, IFI27, OAS1, IFIT1, ISG15, HERC6, SLC1A2, IL1R2 | 0,867 | 0,858 | 0,876 | 0,922 | 0,875 | 0,968 | 0,939 | 0,877 | 1 |
| IFI27, IFIT1, IFI44L, ISG15, SLC1A2, IL1R2, RETN, MMP8 | 0,867 | 0,858 | 0,876 | 0,922 | 0,871 | 0,973 | 0,940 | 0,880 | 1 |

| | | | | | | | | |
|---|---|---|---|---|---|---|---|---|
| RSAD2, OAS1, SIGLEC1, HERC6, SLC1A2, IL1R2, FAM20A, RETN | 0,867 | 0,857 | 0,877 | 0,918 | 0,868 | 0,969 | 0,952 | 0,884 | 1 |
| RSAD2, OAS1, SIGLEC1, ISG15, HERC6, SLC1A2, OLAH, FAM20A | 0,867 | 0,857 | 0,878 | 0,916 | 0,862 | 0,971 | 0,943 | 0,875 | 1 |
| IFI44L, SIGLEC1, HERC6, SLC1A2, IL1R2, OLAH, FAM20A, RETN | 0,867 | 0,857 | 0,877 | 0,917 | 0,864 | 0,971 | 0,951 | 0,879 | 1 |
| RSAD2, IFI44L, SIGLEC1, ISG15, HERC6, IL1R2, FAM20A, RETN | 0,867 | 0,857 | 0,877 | 0,914 | 0,855 | 0,973 | 0,954 | 0,893 | 1 |
| IFI27, OAS1, IFIT1, IFI44L, SIGLEC1, ISG15, RETN, MMP8 | 0,867 | 0,857 | 0,877 | 0,911 | 0,850 | 0,972 | 0,954 | 0,896 | 1 |
| IFI44L, SIGLEC1, ISG15, HERC6, SLC1A2, IL1R2, FAM20A, RETN | 0,867 | 0,857 | 0,877 | 0,920 | 0,868 | 0,971 | 0,951 | 0,879 | 1 |
| RSAD2, OAS1, IFI44L, SIGLEC1, IL1R2, OLAH, FAM20A, RETN | 0,867 | 0,857 | 0,877 | 0,904 | 0,839 | 0,970 | 0,961 | 0,906 | 1 |
| OAS1, SIGLEC1, HERC6, SLC1A2, IL1R2, OLAH, FAM20A, RETN | 0,867 | 0,856 | 0,878 | 0,921 | 0,871 | 0,971 | 0,949 | 0,882 | 1 |
| RSAD2, IFI27, OAS1, IFI44L, HERC6, SLC1A2, IL1R2, MMP8 | 0,867 | 0,858 | 0,876 | 0,923 | 0,877 | 0,969 | 0,928 | 0,857 | 1,000 |
| IFI27, OAS1, IFIT1, ISG15, HERC6, SLC1A2, IL1R2, MMP8 | 0,867 | 0,858 | 0,876 | 0,922 | 0,876 | 0,968 | 0,939 | 0,877 | 1 |
| IFI27, OAS1, IFIT1, IFI44L, HERC6, SLC1A2, IL1R2, MMP8 | 0,867 | 0,858 | 0,876 | 0,923 | 0,877 | 0,969 | 0,936 | 0,875 | 0,997 |
| IFI27, OAS1, IFIT1, IFI44L, SIGLEC1, ISG15, SLC1A2, RETN | 0,867 | 0,858 | 0,876 | 0,914 | 0,858 | 0,971 | 0,949 | 0,883 | 1 |
| RSAD2, OAS1, SIGLEC1, HERC6, SLC1A2, FAM20A, RETN, MMP8 | 0,867 | 0,857 | 0,877 | 0,913 | 0,855 | 0,971 | 0,949 | 0,878 | 1 |
| OAS1, SIGLEC1, ISG15, SLC1A2, IL1R2, OLAH, FAM20A, RETN | 0,867 | 0,857 | 0,877 | 0,918 | 0,865 | 0,970 | 0,955 | 0,894 | 1 |
| OAS1, IFI44L, SIGLEC1, HERC6, OLAH, FAM20A, RETN, MMP8 | 0,867 | 0,857 | 0,877 | 0,913 | 0,852 | 0,975 | 0,958 | 0,904 | 1 |
| RSAD2, IFI27, OAS1, IFIT1, IFI44L, HERC6, SLC1A2, IL1R2 | 0,867 | 0,858 | 0,876 | 0,922 | 0,875 | 0,968 | 0,938 | 0,876 | 1,000 |
| RSAD2, IFI27, OAS1, IFIT1, IFI44L, ISG15, IL1R2, MMP8 | 0,867 | 0,858 | 0,876 | 0,916 | 0,864 | 0,968 | 0,939 | 0,885 | 0,994 |
| IFI27, OAS1, IFIT1, IFI44L, ISG15, SLC1A2, IL1R2, RETN | 0,867 | 0,858 | 0,876 | 0,919 | 0,867 | 0,972 | 0,941 | 0,879 | 1 |
| OAS1, SIGLEC1, ISG15, HERC6, SLC1A2, FAM20A, RETN, MMP8 | 0,867 | 0,856 | 0,877 | 0,914 | 0,856 | 0,972 | 0,950 | 0,883 | 1 |
| RSAD2, IFI27, OAS1, IFIT1, ISG15, SLC1A2, IL1R2, RETN | 0,867 | 0,858 | 0,875 | 0,921 | 0,870 | 0,972 | 0,934 | 0,861 | 1 |
| RSAD2, SIGLEC1, ISG15, HERC6, SLC1A2, IL1R2, FAM20A, RETN | 0,867 | 0,856 | 0,877 | 0,918 | 0,866 | 0,969 | 0,948 | 0,875 | 1 |
| RSAD2, IFI27, OAS1, IFIT1, SIGLEC1, ISG15, SLC1A2, RETN | 0,867 | 0,858 | 0,875 | 0,915 | 0,857 | 0,973 | 0,948 | 0,879 | 1 |
| IFI27, OAS1, IFIT1, SIGLEC1, ISG15, SLC1A2, RETN, MMP8 | 0,867 | 0,857 | 0,876 | 0,916 | 0,860 | 0,972 | 0,949 | 0,883 | 1 |
| OAS1, IFI44L, SIGLEC1, HERC6, SLC1A2, IL1R2, FAM20A, RETN | 0,867 | 0,856 | 0,877 | 0,920 | 0,870 | 0,970 | 0,950 | 0,881 | 1 |
| RSAD2, IFI27, IFI44L, SIGLEC1, HERC6, SLC1A2, RETN, MMP8 | 0,866 | 0,858 | 0,875 | 0,922 | 0,868 | 0,975 | 0,941 | 0,883 | 0,999 |
| RSAD2, IFI27, OAS1, IFIT1, IFI44L, SLC1A2, IL1R2, RETN | 0,866 | 0,858 | 0,875 | 0,919 | 0,867 | 0,972 | 0,930 | 0,856 | 1 |
| RSAD2, IFI27, IFIT1, ISG15, SLC1A2, IL1R2, RETN, MMP8 | 0,866 | 0,858 | 0,875 | 0,922 | 0,871 | 0,972 | 0,946 | 0,887 | 1 |
| RSAD2, IFI44L, SIGLEC1, SLC1A2, OLAH, FAM20A, RETN, MMP8 | 0,866 | 0,856 | 0,877 | 0,915 | 0,858 | 0,972 | 0,949 | 0,888 | 1 |

| | | | | | | | | |
|---|---|---|---|---|---|---|---|---|
| RSAD2, IFI44L, SIGLEC1, ISG15, HERC6, FAM20A, RETN, MMP8 | 0,866 | 0,857 | 0,876 | 0,913 | 0,852 | 0,974 | 0,950 | 0,885 | 1 |
| RSAD2, IFI44L, SIGLEC1, ISG15, HERC6, SLC1A2, OLAH, FAM20A | 0,866 | 0,856 | 0,877 | 0,917 | 0,862 | 0,972 | 0,938 | 0,867 | 1 |
| OAS1, IFIT1, IFI44L, SIGLEC1, SLC1A2, FAM20A, RETN, MMP8 | 0,866 | 0,856 | 0,876 | 0,918 | 0,862 | 0,974 | 0,939 | 0,868 | 1 |
| OAS1, IFI44L, SIGLEC1, ISG15, SLC1A2, IL1R2, OLAH, FAM20A | 0,866 | 0,856 | 0,876 | 0,917 | 0,864 | 0,971 | 0,954 | 0,893 | 1 |
| RSAD2, IFI27, OAS1, IFIT1, IFI44L, SIGLEC1, SLC1A2, RETN | 0,866 | 0,858 | 0,875 | 0,916 | 0,859 | 0,974 | 0,948 | 0,878 | 1 |
| RSAD2, OAS1, IFIT1, SIGLEC1, ISG15, FAM20A, RETN, MMP8 | 0,866 | 0,856 | 0,876 | 0,909 | 0,843 | 0,974 | 0,952 | 0,889 | 1 |
| RSAD2, IFI44L, SIGLEC1, ISG15, IL1R2, OLAH, FAM20A, RETN | 0,866 | 0,856 | 0,876 | 0,906 | 0,843 | 0,970 | 0,962 | 0,909 | 1 |
| RSAD2, OAS1, SIGLEC1, ISG15, OLAH, FAM20A, RETN, MMP8 | 0,866 | 0,856 | 0,876 | 0,907 | 0,842 | 0,973 | 0,965 | 0,922 | 1 |
| SIGLEC1, ISG15, HERC6, SLC1A2, IL1R2, OLAH, FAM20A, RETN | 0,866 | 0,856 | 0,876 | 0,918 | 0,865 | 0,971 | 0,948 | 0,875 | 1 |
| RSAD2, IFI27, OAS1, ISG15, HERC6, SLC1A2, IL1R2, MMP8 | 0,866 | 0,857 | 0,875 | 0,923 | 0,877 | 0,970 | 0,930 | 0,863 | 0,998 |
| RSAD2, IFI44L, SIGLEC1, SLC1A2, IL1R2, OLAH, FAM20A, RETN | 0,866 | 0,856 | 0,876 | 0,917 | 0,863 | 0,971 | 0,954 | 0,893 | 1 |
| RSAD2, IFI44L, SIGLEC1, HERC6, OLAH, FAM20A, RETN, MMP8 | 0,866 | 0,856 | 0,876 | 0,915 | 0,855 | 0,975 | 0,952 | 0,895 | 1 |
| RSAD2, IFI27, IFIT1, IFI44L, SLC1A2, IL1R2, RETN, MMP8 | 0,866 | 0,857 | 0,875 | 0,921 | 0,870 | 0,973 | 0,941 | 0,881 | 1 |
| RSAD2, IFI27, OAS1, IFI44L, SLC1A2, IL1R2, RETN, MMP8 | 0,866 | 0,857 | 0,875 | 0,924 | 0,875 | 0,974 | 0,935 | 0,865 | 1 |
| RSAD2, IFI27, OAS1, IFIT1, IFI44L, SIGLEC1, RETN, MMP8 | 0,866 | 0,856 | 0,876 | 0,910 | 0,848 | 0,972 | 0,952 | 0,889 | 1 |
| RSAD2, OAS1, IFIT1, IFI44L, SIGLEC1, FAM20A, RETN, MMP8 | 0,866 | 0,856 | 0,876 | 0,911 | 0,845 | 0,976 | 0,941 | 0,874 | 1 |
| RSAD2, OAS1, IFI44L, SIGLEC1, HERC6, SLC1A2, OLAH, FAM20A | 0,866 | 0,855 | 0,876 | 0,918 | 0,863 | 0,972 | 0,942 | 0,873 | 1 |
| RSAD2, IFI44L, SIGLEC1, ISG15, SLC1A2, IL1R2, OLAH, FAM20A | 0,866 | 0,856 | 0,876 | 0,916 | 0,862 | 0,971 | 0,954 | 0,893 | 1 |
| OAS1, IFI44L, SIGLEC1, SLC1A2, IL1R2, OLAH, FAM20A, RETN | 0,866 | 0,856 | 0,876 | 0,917 | 0,863 | 0,970 | 0,958 | 0,900 | 1 |
| RSAD2, IFI27, OAS1, IFI44L, SIGLEC1, ISG15, SLC1A2, RETN | 0,866 | 0,857 | 0,874 | 0,916 | 0,858 | 0,974 | 0,948 | 0,876 | 1 |
| RSAD2, IFI44L, SIGLEC1, ISG15, HERC6, OLAH, FAM20A, MMP8 | 0,866 | 0,855 | 0,876 | 0,910 | 0,849 | 0,970 | 0,953 | 0,898 | 1 |
| IFIT1, IFI44L, SIGLEC1, ISG15, SLC1A2, FAM20A, RETN, MMP8 | 0,866 | 0,856 | 0,875 | 0,917 | 0,861 | 0,974 | 0,938 | 0,867 | 1 |
| RSAD2, OAS1, IFIT1, IFI44L, SIGLEC1, ISG15, FAM20A, RETN | 0,866 | 0,856 | 0,875 | 0,904 | 0,835 | 0,973 | 0,942 | 0,881 | 1 |
| IFI27, OAS1, IFIT1, IFI44L, ISG15, HERC6, SLC1A2, IL1R2 | 0,866 | 0,856 | 0,875 | 0,923 | 0,877 | 0,969 | 0,936 | 0,875 | 0,997 |
| OAS1, IFI44L, SIGLEC1, ISG15, SLC1A2, IL1R2, FAM20A, RETN | 0,865 | 0,856 | 0,875 | 0,915 | 0,861 | 0,968 | 0,953 | 0,885 | 1 |
| RSAD2, OAS1, IFIT1, IFI44L, SIGLEC1, ISG15, FAM20A, MMP8 | 0,865 | 0,855 | 0,876 | 0,904 | 0,834 | 0,973 | 0,939 | 0,870 | 1 |
| RSAD2, OAS1, IFI44L, SIGLEC1, ISG15, IL1R2, FAM20A, RETN | 0,865 | 0,855 | 0,876 | 0,907 | 0,844 | 0,971 | 0,961 | 0,904 | 1 |
| RSAD2, IFI27, OAS1, IFI44L, ISG15, FAM20A, RETN, MMP8 | 0,865 | 0,854 | 0,877 | 0,914 | 0,851 | 0,976 | 0,946 | 0,872 | 1 |

| Genes | | | | | | | | |
|---|---|---|---|---|---|---|---|---|
| OAS1, IFI44L, SIGLEC1, ISG15, HERC6, FAM20A, RETN, MMP8 | 0,865 | 0,855 | 0,875 | 0,911 | 0,848 | 0,974 | 0,950 | 0,883 | 1 |
| RSAD2, IFI44L, SIGLEC1, ISG15, SLC1A2, IL1R2, FAM20A, RETN | 0,865 | 0,855 | 0,875 | 0,915 | 0,862 | 0,968 | 0,952 | 0,882 | 1 |
| RSAD2, OAS1, SIGLEC1, ISG15, SLC1A2, IL1R2, FAM20A, RETN | 0,865 | 0,855 | 0,875 | 0,915 | 0,861 | 0,968 | 0,954 | 0,888 | 1 |
| RSAD2, IFI44L, SIGLEC1, HERC6, SLC1A2, IL1R2, FAM20A, RETN | 0,865 | 0,855 | 0,876 | 0,919 | 0,868 | 0,970 | 0,947 | 0,874 | 1 |
| IFI27, IFI44L, SIGLEC1, ISG15, HERC6, SLC1A2, RETN, MMP8 | 0,865 | 0,856 | 0,875 | 0,917 | 0,861 | 0,973 | 0,941 | 0,882 | 1 |
| IFI44L, ISG15, HERC6, SLC1A2, IL1R2, OLAH, FAM20A, MMP8 | 0,865 | 0,856 | 0,875 | 0,917 | 0,866 | 0,969 | 0,929 | 0,848 | 1 |
| IFIT1, IFI44L, ISG15, HERC6, SLC1A2, OLAH, FAM20A, MMP8 | 0,865 | 0,855 | 0,875 | 0,917 | 0,863 | 0,971 | 0,922 | 0,840 | 1 |
| RSAD2, IFI27, OAS1, IFIT1, ISG15, HERC6, FAM20A, MMP8 | 0,865 | 0,855 | 0,875 | 0,913 | 0,856 | 0,970 | 0,925 | 0,844 | 1 |
| RSAD2, OAS1, IFI44L, SIGLEC1, ISG15, OLAH, FAM20A, MMP8 | 0,865 | 0,855 | 0,876 | 0,905 | 0,840 | 0,971 | 0,960 | 0,910 | 1 |
| IFI27, OAS1, IFIT1, IFI44L, SIGLEC1, SLC1A2, RETN, MMP8 | 0,865 | 0,856 | 0,874 | 0,915 | 0,858 | 0,972 | 0,950 | 0,885 | 1 |
| OAS1, SIGLEC1, ISG15, HERC6, SLC1A2, OLAH, FAM20A, RETN | 0,865 | 0,854 | 0,876 | 0,915 | 0,859 | 0,970 | 0,943 | 0,874 | 1 |
| OAS1, IFIT1, IFI44L, SIGLEC1, ISG15, SLC1A2, FAM20A, MMP8 | 0,865 | 0,855 | 0,875 | 0,912 | 0,852 | 0,971 | 0,938 | 0,864 | 1 |
| RSAD2, OAS1, IFI44L, SIGLEC1, SLC1A2, IL1R2, OLAH, FAM20A | 0,865 | 0,855 | 0,875 | 0,916 | 0,861 | 0,970 | 0,955 | 0,896 | 1 |
| IFIT1, IFI44L, HERC6, SLC1A2, IL1R2, OLAH, FAM20A, MMP8 | 0,865 | 0,856 | 0,875 | 0,919 | 0,869 | 0,970 | 0,929 | 0,847 | 1 |
| RSAD2, OAS1, IFI44L, HERC6, IL1R2, OLAH, FAM20A, MMP8 | 0,865 | 0,855 | 0,875 | 0,912 | 0,854 | 0,970 | 0,936 | 0,860 | 1 |
| RSAD2, IFI27, OAS1, IFIT1, IFI44L, FAM20A, RETN, MMP8 | 0,865 | 0,854 | 0,876 | 0,915 | 0,853 | 0,977 | 0,947 | 0,873 | 1 |
| RSAD2, OAS1, ISG15, HERC6, IL1R2, OLAH, FAM20A, MMP8 | 0,865 | 0,855 | 0,875 | 0,909 | 0,851 | 0,967 | 0,937 | 0,859 | 1 |
| RSAD2, IFI27, OAS1, IFIT1, SIGLEC1, SLC1A2, RETN, MMP8 | 0,865 | 0,856 | 0,874 | 0,915 | 0,857 | 0,973 | 0,950 | 0,883 | 1 |
| RSAD2, IFI27, OAS1, ISG15, SLC1A2, IL1R2, RETN, MMP8 | 0,865 | 0,856 | 0,874 | 0,923 | 0,872 | 0,973 | 0,934 | 0,863 | 1 |
| RSAD2, IFI27, IFIT1, IFI44L, ISG15, SLC1A2, IL1R2, RETN | 0,865 | 0,856 | 0,874 | 0,920 | 0,868 | 0,972 | 0,934 | 0,868 | 0,999 |
| RSAD2, IFI44L, HERC6, SLC1A2, IL1R2, OLAH, FAM20A, MMP8 | 0,865 | 0,855 | 0,875 | 0,919 | 0,868 | 0,970 | 0,928 | 0,846 | 1 |
| RSAD2, IFI27, OAS1, IFI44L, ISG15, HERC6, SLC1A2, IL1R2 | 0,865 | 0,856 | 0,874 | 0,924 | 0,878 | 0,969 | 0,933 | 0,867 | 0,998 |
| RSAD2, IFI27, OAS1, IFIT1, IFI44L, SLC1A2, IL1R2, MMP8 | 0,865 | 0,856 | 0,873 | 0,921 | 0,874 | 0,969 | 0,940 | 0,879 | 1 |
| RSAD2, IFI27, IFIT1, IFI44L, ISG15, FAM20A, RETN, MMP8 | 0,865 | 0,854 | 0,876 | 0,914 | 0,851 | 0,977 | 0,942 | 0,867 | 1 |
| RSAD2, OAS1, IFIT1, HERC6, IL1R2, OLAH, FAM20A, MMP8 | 0,865 | 0,855 | 0,875 | 0,907 | 0,847 | 0,967 | 0,940 | 0,866 | 1 |
| OAS1, IFIT1, IFI44L, SIGLEC1, HERC6, IL1R2, RETN, MMP8 | 0,865 | 0,855 | 0,874 | 0,903 | 0,841 | 0,966 | 0,963 | 0,924 | 1 |
| RSAD2, OAS1, IFI44L, SIGLEC1, SLC1A2, IL1R2, FAM20A, RETN | 0,865 | 0,855 | 0,875 | 0,915 | 0,861 | 0,969 | 0,953 | 0,888 | 1 |
| RSAD2, OAS1, SIGLEC1, HERC6, SLC1A2, OLAH, FAM20A, RETN | 0,865 | 0,854 | 0,875 | 0,915 | 0,860 | 0,970 | 0,943 | 0,875 | 1 |

| | | | | | | | | |
|---|---|---|---|---|---|---|---|---|
| RSAD2, OAS1, IFIT1, IFI44L, SIGLEC1, SLC1A2, FAM20A, MMP8 | 0,865 | 0,855 | 0,875 | 0,914 | 0,855 | 0,972 | 0,937 | 0,863 | 1 |
| IFI27, OAS1, IFIT1, IFI44L, ISG15, FAM20A, RETN, MMP8 | 0,865 | 0,853 | 0,876 | 0,912 | 0,849 | 0,975 | 0,948 | 0,874 | 1 |
| RSAD2, IFI27, OAS1, IFI44L, ISG15, SLC1A2, IL1R2, RETN | 0,865 | 0,856 | 0,873 | 0,920 | 0,868 | 0,972 | 0,929 | 0,854 | 1 |
| RSAD2, IFI27, OAS1, IFIT1, SIGLEC1, ISG15, RETN, MMP8 | 0,865 | 0,855 | 0,875 | 0,909 | 0,847 | 0,971 | 0,954 | 0,895 | 1 |
| RSAD2, IFIT1, IFI44L, SIGLEC1, ISG15, SLC1A2, FAM20A, MMP8 | 0,865 | 0,855 | 0,874 | 0,912 | 0,853 | 0,970 | 0,937 | 0,863 | 1 |
| RSAD2, OAS1, SIGLEC1, ISG15, HERC6, OLAH, FAM20A, RETN | 0,865 | 0,855 | 0,875 | 0,908 | 0,846 | 0,970 | 0,951 | 0,896 | 1 |
| RSAD2, SIGLEC1, ISG15, HERC6, SLC1A2, OLAH, FAM20A, RETN | 0,865 | 0,854 | 0,875 | 0,915 | 0,860 | 0,970 | 0,941 | 0,871 | 1 |
| RSAD2, IFI27, IFIT1, ISG15, HERC6, SLC1A2, IL1R2, MMP8 | 0,865 | 0,856 | 0,873 | 0,923 | 0,878 | 0,968 | 0,936 | 0,876 | 0,996 |
| OAS1, IFIT1, SIGLEC1, ISG15, HERC6, IL1R2, RETN, MMP8 | 0,865 | 0,855 | 0,874 | 0,908 | 0,849 | 0,968 | 0,963 | 0,925 | 1 |
| RSAD2, IFI27, OAS1, IFI44L, SIGLEC1, ISG15, RETN, MMP8 | 0,865 | 0,854 | 0,875 | 0,910 | 0,848 | 0,972 | 0,953 | 0,890 | 1 |
| RSAD2, IFI27, OAS1, IFIT1, IFI44L, ISG15, SLC1A2, IL1R2 | 0,865 | 0,856 | 0,873 | 0,922 | 0,875 | 0,969 | 0,938 | 0,874 | 1 |
| RSAD2, IFI27, OAS1, IFIT1, SIGLEC1, HERC6, SLC1A2, MMP8 | 0,864 | 0,856 | 0,873 | 0,916 | 0,864 | 0,969 | 0,914 | 0,839 | 0,989 |
| RSAD2, OAS1, IFI44L, SIGLEC1, ISG15, SLC1A2, IL1R2, FAM20A | 0,864 | 0,854 | 0,874 | 0,915 | 0,861 | 0,969 | 0,954 | 0,891 | 1 |
| RSAD2, OAS1, IFIT1, IFI44L, SIGLEC1, ISG15, SLC1A2, FAM20A | 0,864 | 0,855 | 0,874 | 0,912 | 0,852 | 0,971 | 0,935 | 0,860 | 1 |
| RSAD2, IFI27, IFIT1, IFI44L, HERC6, SLC1A2, IL1R2, MMP8 | 0,864 | 0,856 | 0,873 | 0,924 | 0,878 | 0,969 | 0,935 | 0,875 | 0,995 |
| RSAD2, IFIT1, IFI44L, HERC6, SLC1A2, OLAH, FAM20A, MMP8 | 0,864 | 0,854 | 0,875 | 0,917 | 0,864 | 0,970 | 0,916 | 0,833 | 1,000 |
| RSAD2, IFI27, OAS1, IFIT1, ISG15, SLC1A2, IL1R2, MMP8 | 0,864 | 0,856 | 0,873 | 0,921 | 0,873 | 0,968 | 0,936 | 0,871 | 1 |
| RSAD2, IFI27, IFI44L, ISG15, SLC1A2, IL1R2, RETN, MMP8 | 0,864 | 0,856 | 0,873 | 0,923 | 0,873 | 0,973 | 0,930 | 0,860 | 1 |
| IFI44L, ISG15, SLC1A2, IL1R2, OLAH, FAM20A, RETN, MMP8 | 0,864 | 0,855 | 0,874 | 0,917 | 0,864 | 0,970 | 0,929 | 0,851 | 1 |
| OAS1, IFIT1, SIGLEC1, ISG15, SLC1A2, FAM20A, RETN, MMP8 | 0,864 | 0,854 | 0,874 | 0,913 | 0,855 | 0,972 | 0,952 | 0,884 | 1 |
| IFIT1, IFI44L, ISG15, HERC6, IL1R2, OLAH, FAM20A, MMP8 | 0,864 | 0,855 | 0,874 | 0,909 | 0,851 | 0,968 | 0,937 | 0,860 | 1 |
| RSAD2, IFIT1, IFI44L, SIGLEC1, SLC1A2, FAM20A, RETN, MMP8 | 0,864 | 0,854 | 0,874 | 0,917 | 0,861 | 0,973 | 0,937 | 0,865 | 1 |
| OAS1, IFI44L, SIGLEC1, ISG15, HERC6, OLAH, FAM20A, MMP8 | 0,864 | 0,854 | 0,875 | 0,908 | 0,845 | 0,970 | 0,959 | 0,907 | 1 |
| IFIT1, IFI44L, HERC6, SLC1A2, OLAH, FAM20A, RETN, MMP8 | 0,864 | 0,854 | 0,874 | 0,919 | 0,865 | 0,974 | 0,924 | 0,842 | 1 |
| IFI44L, SIGLEC1, ISG15, HERC6, SLC1A2, OLAH, FAM20A, RETN | 0,864 | 0,854 | 0,875 | 0,914 | 0,857 | 0,970 | 0,946 | 0,872 | 1 |
| RSAD2, IFI27, IFIT1, IFI44L, ISG15, HERC6, SLC1A2, IL1R2 | 0,864 | 0,856 | 0,873 | 0,923 | 0,878 | 0,969 | 0,935 | 0,875 | 0,995 |
| RSAD2, OAS1, IFI44L, SIGLEC1, HERC6, FAM20A, RETN, MMP8 | 0,864 | 0,854 | 0,874 | 0,910 | 0,847 | 0,973 | 0,951 | 0,885 | 1 |
| RSAD2, OAS1, SIGLEC1, ISG15, SLC1A2, OLAH, FAM20A, RETN | 0,864 | 0,854 | 0,874 | 0,915 | 0,857 | 0,972 | 0,949 | 0,888 | 1 |

| | | | | | | | | |
|---|---|---|---|---|---|---|---|---|
| RSAD2, OAS1, IFI44L, SIGLEC1, ISG15, SLC1A2, OLAH, FAM20A | 0,864 | 0,854 | 0,874 | 0,911 | 0,852 | 0,970 | 0,945 | 0,881 | 1 |
| IFIT1, IFI44L, ISG15, SLC1A2, IL1R2, OLAH, FAM20A, MMP8 | 0,864 | 0,855 | 0,873 | 0,916 | 0,863 | 0,968 | 0,928 | 0,848 | 1 |
| OAS1, IFI44L, SIGLEC1, ISG15, SLC1A2, OLAH, FAM20A, RETN | 0,864 | 0,854 | 0,874 | 0,914 | 0,857 | 0,972 | 0,948 | 0,888 | 1 |
| IFI27, OAS1, IFI44L, ISG15, HERC6, SLC1A2, IL1R2, MMP8 | 0,864 | 0,855 | 0,873 | 0,921 | 0,875 | 0,967 | 0,928 | 0,861 | 0,995 |
| RSAD2, OAS1, SIGLEC1, ISG15, HERC6, SLC1A2, FAM20A, RETN | 0,864 | 0,853 | 0,874 | 0,912 | 0,855 | 0,969 | 0,941 | 0,869 | 1 |
| OAS1, IFIT1, SIGLEC1, HERC6, SLC1A2, OLAH, RETN, MMP8 | 0,864 | 0,854 | 0,874 | 0,915 | 0,860 | 0,970 | 0,954 | 0,910 | 0,999 |
| RSAD2, OAS1, IFI44L, SIGLEC1, ISG15, HERC6, FAM20A, RETN | 0,864 | 0,854 | 0,874 | 0,911 | 0,851 | 0,972 | 0,946 | 0,879 | 1 |
| RSAD2, IFIT1, IFI44L, SIGLEC1, ISG15, FAM20A, RETN, MMP8 | 0,864 | 0,854 | 0,874 | 0,908 | 0,841 | 0,975 | 0,943 | 0,881 | 1 |
| IFI27, OAS1, IFIT1, IFI44L, HERC6, SLC1A2, FAM20A, MMP8 | 0,864 | 0,853 | 0,874 | 0,917 | 0,863 | 0,971 | 0,910 | 0,820 | 1 |
| OAS1, IFIT1, IFI44L, HERC6, IL1R2, OLAH, FAM20A, MMP8 | 0,864 | 0,854 | 0,873 | 0,908 | 0,849 | 0,967 | 0,938 | 0,862 | 1 |
| IFI27, OAS1, IFIT1, IFI44L, SIGLEC1, ISG15, HERC6, MMP8 | 0,864 | 0,855 | 0,872 | 0,909 | 0,856 | 0,963 | 0,908 | 0,833 | 0,982 |
| OAS1, IFI44L, SIGLEC1, HERC6, SLC1A2, OLAH, FAM20A, RETN | 0,864 | 0,853 | 0,874 | 0,916 | 0,861 | 0,971 | 0,941 | 0,872 | 1 |
| RSAD2, IFI27, IFIT1, IFI44L, HERC6, SLC1A2, FAM20A, MMP8 | 0,864 | 0,853 | 0,874 | 0,916 | 0,861 | 0,971 | 0,914 | 0,826 | 1 |
| IFIT1, IFI44L, ISG15, HERC6, SLC1A2, IL1R2, OLAH, FAM20A | 0,864 | 0,854 | 0,874 | 0,917 | 0,865 | 0,969 | 0,921 | 0,834 | 1 |
| OAS1, IFIT1, IFI44L, HERC6, SLC1A2, OLAH, FAM20A, MMP8 | 0,864 | 0,853 | 0,874 | 0,918 | 0,865 | 0,970 | 0,916 | 0,830 | 1 |
| RSAD2, OAS1, IFIT1, IFI44L, HERC6, IL1R2, FAM20A, MMP8 | 0,864 | 0,854 | 0,873 | 0,912 | 0,853 | 0,971 | 0,938 | 0,859 | 1 |
| RSAD2, OAS1, IFIT1, SIGLEC1, HERC6, IL1R2, RETN, MMP8 | 0,864 | 0,854 | 0,873 | 0,903 | 0,840 | 0,966 | 0,964 | 0,927 | 1 |
| IFI27, OAS1, IFI44L, ISG15, SLC1A2, IL1R2, RETN, MMP8 | 0,864 | 0,855 | 0,872 | 0,923 | 0,874 | 0,973 | 0,934 | 0,865 | 1 |
| IFI44L, HERC6, SLC1A2, IL1R2, OLAH, FAM20A, RETN, MMP8 | 0,864 | 0,854 | 0,873 | 0,917 | 0,864 | 0,971 | 0,933 | 0,852 | 1 |
| OAS1, IFIT1, IFI44L, ISG15, HERC6, SLC1A2, OLAH, FAM20A | 0,864 | 0,853 | 0,874 | 0,916 | 0,863 | 0,970 | 0,915 | 0,830 | 1 |
| RSAD2, OAS1, IFI44L, SIGLEC1, OLAH, FAM20A, RETN, MMP8 | 0,864 | 0,853 | 0,874 | 0,908 | 0,844 | 0,973 | 0,966 | 0,924 | 1 |
| RSAD2, IFI27, OAS1, IFIT1, ISG15, FAM20A, RETN, MMP8 | 0,864 | 0,853 | 0,874 | 0,912 | 0,851 | 0,974 | 0,945 | 0,870 | 1 |
| RSAD2, OAS1, IFI44L, SIGLEC1, ISG15, HERC6, OLAH, FAM20A | 0,864 | 0,853 | 0,874 | 0,908 | 0,846 | 0,970 | 0,950 | 0,893 | 1 |
| RSAD2, IFI27, OAS1, SIGLEC1, ISG15, SLC1A2, RETN, MMP8 | 0,864 | 0,854 | 0,873 | 0,914 | 0,855 | 0,972 | 0,949 | 0,878 | 1 |
| OAS1, IFI44L, SIGLEC1, ISG15, OLAH, FAM20A, RETN, MMP8 | 0,863 | 0,853 | 0,874 | 0,906 | 0,840 | 0,973 | 0,967 | 0,925 | 1 |
| RSAD2, IFI27, OAS1, IFIT1, IFI44L, SIGLEC1, HERC6, MMP8 | 0,863 | 0,855 | 0,872 | 0,907 | 0,849 | 0,964 | 0,903 | 0,825 | 0,982 |
| OAS1, IFIT1, IFI44L, SIGLEC1, ISG15, SLC1A2, FAM20A, RETN | 0,863 | 0,854 | 0,873 | 0,914 | 0,855 | 0,973 | 0,936 | 0,863 | 1 |
| OAS1, IFIT1, SIGLEC1, HERC6, IL1R2, OLAH, RETN, MMP8 | 0,863 | 0,853 | 0,873 | 0,904 | 0,841 | 0,967 | 0,964 | 0,927 | 1 |

| | | | | | | | | |
|---|---|---|---|---|---|---|---|---|
| RSAD2, OAS1, HERC6, IL1R2, OLAH, FAM20A, RETN, MMP8 | 0,863 | 0,853 | 0,874 | 0,909 | 0,848 | 0,969 | 0,941 | 0,867 | 1 |
| IFI27, OAS1, IFI44L, SIGLEC1, ISG15, SLC1A2, RETN, MMP8 | 0,863 | 0,854 | 0,873 | 0,915 | 0,858 | 0,972 | 0,950 | 0,885 | 1 |
| OAS1, IFI44L, HERC6, SLC1A2, IL1R2, OLAH, FAM20A, MMP8 | 0,863 | 0,853 | 0,874 | 0,919 | 0,868 | 0,970 | 0,929 | 0,846 | 1 |
| IFI27, IFIT1, IFI44L, ISG15, HERC6, SLC1A2, IL1R2, MMP8 | 0,863 | 0,855 | 0,872 | 0,923 | 0,877 | 0,969 | 0,934 | 0,873 | 0,994 |
| RSAD2, IFI27, IFIT1, IFI44L, ISG15, HERC6, SLC1A2, RETN | 0,863 | 0,854 | 0,873 | 0,914 | 0,856 | 0,971 | 0,939 | 0,874 | 1 |
| RSAD2, IFI27, IFIT1, IFI44L, ISG15, SLC1A2, IL1R2, MMP8 | 0,863 | 0,855 | 0,872 | 0,923 | 0,876 | 0,969 | 0,934 | 0,873 | 0,994 |
| IFI44L, ISG15, HERC6, IL1R2, OLAH, FAM20A, RETN, MMP8 | 0,863 | 0,853 | 0,873 | 0,912 | 0,852 | 0,972 | 0,946 | 0,874 | 1 |
| IFI27, OAS1, IFIT1, IFI44L, ISG15, SLC1A2, IL1R2, MMP8 | 0,863 | 0,854 | 0,872 | 0,921 | 0,875 | 0,968 | 0,936 | 0,875 | 0,997 |
| RSAD2, OAS1, IFI44L, ISG15, HERC6, IL1R2, OLAH, FAM20A | 0,863 | 0,853 | 0,873 | 0,910 | 0,851 | 0,968 | 0,936 | 0,863 | 1 |
| IFIT1, IFI44L, SIGLEC1, ISG15, HERC6, IL1R2, RETN, MMP8 | 0,863 | 0,854 | 0,873 | 0,904 | 0,842 | 0,966 | 0,964 | 0,926 | 1 |
| RSAD2, IFI27, OAS1, IFI44L, SIGLEC1, SLC1A2, RETN, MMP8 | 0,863 | 0,854 | 0,873 | 0,915 | 0,857 | 0,974 | 0,950 | 0,879 | 1 |
| RSAD2, OAS1, ISG15, HERC6, IL1R2, FAM20A, RETN, MMP8 | 0,863 | 0,853 | 0,873 | 0,910 | 0,851 | 0,969 | 0,935 | 0,853 | 1 |
| IFI27, OAS1, IFIT1, IFI44L, ISG15, SLC1A2, FAM20A, RETN | 0,863 | 0,853 | 0,873 | 0,910 | 0,848 | 0,973 | 0,936 | 0,853 | 1 |
| RSAD2, IFI27, IFI44L, ISG15, HERC6, SLC1A2, IL1R2, MMP8 | 0,863 | 0,854 | 0,872 | 0,920 | 0,874 | 0,967 | 0,926 | 0,859 | 0,993 |
| OAS1, IFI44L, SIGLEC1, HERC6, SLC1A2, FAM20A, RETN, MMP8 | 0,863 | 0,853 | 0,873 | 0,912 | 0,854 | 0,971 | 0,950 | 0,881 | 1 |
| RSAD2, IFI44L, SIGLEC1, HERC6, SLC1A2, OLAH, FAM20A, RETN | 0,863 | 0,852 | 0,874 | 0,917 | 0,862 | 0,972 | 0,939 | 0,868 | 1 |
| RSAD2, IFI27, OAS1, IFIT1, SIGLEC1, ISG15, HERC6, MMP8 | 0,863 | 0,854 | 0,872 | 0,907 | 0,851 | 0,963 | 0,905 | 0,829 | 0,982 |
| IFIT1, SIGLEC1, ISG15, HERC6, IL1R2, OLAH, RETN, MMP8 | 0,863 | 0,853 | 0,873 | 0,906 | 0,845 | 0,968 | 0,966 | 0,930 | 1 |
| IFIT1, IFI44L, ISG15, HERC6, IL1R2, OLAH, FAM20A, RETN | 0,863 | 0,854 | 0,872 | 0,909 | 0,849 | 0,968 | 0,933 | 0,854 | 1 |
| IFIT1, IFI44L, HERC6, IL1R2, OLAH, FAM20A, RETN, MMP8 | 0,863 | 0,853 | 0,872 | 0,911 | 0,851 | 0,971 | 0,942 | 0,869 | 1 |
| RSAD2, IFI27, IFIT1, IFI44L, SIGLEC1, ISG15, SLC1A2, RETN | 0,863 | 0,854 | 0,872 | 0,915 | 0,859 | 0,971 | 0,951 | 0,892 | 1 |
| IFIT1, IFI44L, ISG15, HERC6, IL1R2, FAM20A, RETN, MMP8 | 0,863 | 0,853 | 0,873 | 0,910 | 0,850 | 0,970 | 0,940 | 0,862 | 1 |
| OAS1, ISG15, HERC6, IL1R2, OLAH, FAM20A, RETN, MMP8 | 0,863 | 0,853 | 0,873 | 0,910 | 0,851 | 0,970 | 0,943 | 0,869 | 1 |
| RSAD2, OAS1, IFI44L, SLC1A2, IL1R2, OLAH, FAM20A, MMP8 | 0,863 | 0,853 | 0,873 | 0,917 | 0,865 | 0,969 | 0,928 | 0,848 | 1 |
| OAS1, IFIT1, ISG15, HERC6, IL1R2, FAM20A, RETN, MMP8 | 0,863 | 0,853 | 0,873 | 0,910 | 0,849 | 0,970 | 0,938 | 0,859 | 1 |
| RSAD2, OAS1, IFIT1, SIGLEC1, SLC1A2, FAM20A, RETN, MMP8 | 0,863 | 0,853 | 0,873 | 0,914 | 0,856 | 0,972 | 0,952 | 0,884 | 1 |
| RSAD2, IFI44L, SIGLEC1, HERC6, SLC1A2, FAM20A, RETN, MMP8 | 0,863 | 0,853 | 0,873 | 0,913 | 0,855 | 0,971 | 0,949 | 0,877 | 1 |
| RSAD2, OAS1, IFI44L, ISG15, HERC6, IL1R2, FAM20A, MMP8 | 0,863 | 0,853 | 0,873 | 0,911 | 0,853 | 0,969 | 0,928 | 0,844 | 1 |

561

| | | | | | | | | |
|---|---|---|---|---|---|---|---|---|
| IFIT1, IFI44L, ISG15, HERC6, SLC1A2, OLAH, FAM20A, RETN | 0,863 | 0,853 | 0,873 | 0,918 | 0,864 | 0,971 | 0,918 | 0,833 | 1 |
| RSAD2, OAS1, IFIT1, IFI44L, IL1R2, OLAH, FAM20A, MMP8 | 0,863 | 0,853 | 0,873 | 0,908 | 0,847 | 0,969 | 0,945 | 0,876 | 1 |
| RSAD2, IFI27, IFIT1, IFI44L, SIGLEC1, ISG15, RETN, MMP8 | 0,863 | 0,853 | 0,873 | 0,911 | 0,850 | 0,972 | 0,950 | 0,892 | 1 |
| RSAD2, IFI44L, SIGLEC1, ISG15, SLC1A2, OLAH, FAM20A, RETN | 0,863 | 0,853 | 0,873 | 0,916 | 0,858 | 0,973 | 0,946 | 0,884 | 1 |
| IFI27, OAS1, IFIT1, SIGLEC1, ISG15, HERC6, SLC1A2, MMP8 | 0,863 | 0,854 | 0,872 | 0,913 | 0,860 | 0,965 | 0,915 | 0,843 | 0,987 |
| RSAD2, OAS1, IFIT1, ISG15, HERC6, IL1R2, FAM20A, MMP8 | 0,863 | 0,853 | 0,873 | 0,908 | 0,848 | 0,968 | 0,937 | 0,860 | 1 |
| RSAD2, OAS1, IFIT1, IFI44L, HERC6, IL1R2, OLAH, FAM20A | 0,863 | 0,853 | 0,872 | 0,908 | 0,847 | 0,968 | 0,932 | 0,854 | 1 |
| OAS1, IFIT1, ISG15, HERC6, IL1R2, OLAH, FAM20A, MMP8 | 0,863 | 0,853 | 0,873 | 0,907 | 0,848 | 0,967 | 0,943 | 0,871 | 1 |
| RSAD2, OAS1, IFIT1, IFI44L, HERC6, SLC1A2, OLAH, FAM20A | 0,863 | 0,853 | 0,873 | 0,917 | 0,863 | 0,970 | 0,912 | 0,825 | 0,999 |
| OAS1, IFIT1, IFI44L, SIGLEC1, ISG15, HERC6, IL1R2, RETN | 0,863 | 0,853 | 0,872 | 0,907 | 0,848 | 0,966 | 0,952 | 0,907 | 0,998 |
| RSAD2, IFI44L, SIGLEC1, ISG15, HERC6, OLAH, FAM20A, RETN | 0,863 | 0,853 | 0,873 | 0,908 | 0,847 | 0,970 | 0,949 | 0,892 | 1 |
| IFI27, IFIT1, IFI44L, ISG15, HERC6, SLC1A2, FAM20A, MMP8 | 0,863 | 0,852 | 0,873 | 0,914 | 0,857 | 0,970 | 0,915 | 0,826 | 1 |
| OAS1, IFIT1, HERC6, IL1R2, OLAH, FAM20A, RETN, MMP8 | 0,863 | 0,853 | 0,872 | 0,908 | 0,846 | 0,969 | 0,942 | 0,868 | 1 |
| RSAD2, ISG15, HERC6, IL1R2, OLAH, FAM20A, RETN, MMP8 | 0,863 | 0,852 | 0,873 | 0,909 | 0,849 | 0,969 | 0,938 | 0,861 | 1 |
| RSAD2, IFIT1, IFI44L, SLC1A2, IL1R2, OLAH, FAM20A, MMP8 | 0,863 | 0,853 | 0,872 | 0,917 | 0,865 | 0,969 | 0,932 | 0,854 | 1 |
| OAS1, IFI44L, ISG15, HERC6, IL1R2, OLAH, FAM20A, MMP8 | 0,863 | 0,852 | 0,873 | 0,910 | 0,851 | 0,968 | 0,941 | 0,867 | 1 |
| RSAD2, OAS1, IFI44L, HERC6, SLC1A2, OLAH, FAM20A, MMP8 | 0,862 | 0,852 | 0,873 | 0,916 | 0,862 | 0,970 | 0,920 | 0,840 | 0,999 |
| RSAD2, OAS1, IFIT1, IFI44L, ISG15, HERC6, IL1R2, FAM20A | 0,862 | 0,853 | 0,872 | 0,909 | 0,850 | 0,967 | 0,930 | 0,850 | 1 |
| RSAD2, IFI44L, ISG15, HERC6, IL1R2, OLAH, FAM20A, MMP8 | 0,862 | 0,852 | 0,873 | 0,913 | 0,855 | 0,970 | 0,932 | 0,853 | 1 |
| RSAD2, OAS1, IFI44L, SIGLEC1, SLC1A2, OLAH, FAM20A, RETN | 0,862 | 0,852 | 0,873 | 0,915 | 0,857 | 0,973 | 0,945 | 0,883 | 1 |
| IFIT1, IFI44L, SIGLEC1, HERC6, SLC1A2, OLAH, RETN, MMP8 | 0,862 | 0,852 | 0,872 | 0,913 | 0,858 | 0,969 | 0,950 | 0,904 | 0,996 |
| IFI44L, SIGLEC1, ISG15, HERC6, SLC1A2, FAM20A, RETN, MMP8 | 0,862 | 0,852 | 0,873 | 0,905 | 0,842 | 0,968 | 0,953 | 0,880 | 1 |
| OAS1, IFI44L, SIGLEC1, ISG15, HERC6, OLAH, FAM20A, RETN | 0,862 | 0,852 | 0,873 | 0,907 | 0,844 | 0,970 | 0,954 | 0,899 | 1 |
| RSAD2, OAS1, IFIT1, SIGLEC1, ISG15, SLC1A2, FAM20A, MMP8 | 0,862 | 0,852 | 0,872 | 0,909 | 0,848 | 0,969 | 0,946 | 0,872 | 1 |
| IFIT1, SIGLEC1, HERC6, SLC1A2, IL1R2, OLAH, RETN, MMP8 | 0,862 | 0,853 | 0,872 | 0,916 | 0,862 | 0,970 | 0,962 | 0,924 | 1 |
| RSAD2, IFI27, OAS1, IFI44L, ISG15, SLC1A2, IL1R2, MMP8 | 0,862 | 0,854 | 0,871 | 0,920 | 0,872 | 0,967 | 0,930 | 0,862 | 0,999 |
| RSAD2, OAS1, SIGLEC1, ISG15, HERC6, SLC1A2, FAM20A, MMP8 | 0,862 | 0,852 | 0,873 | 0,909 | 0,852 | 0,966 | 0,940 | 0,864 | 1 |
| OAS1, IFIT1, IFI44L, SIGLEC1, HERC6, OLAH, RETN, MMP8 | 0,862 | 0,852 | 0,872 | 0,901 | 0,836 | 0,967 | 0,952 | 0,908 | 0,997 |

| | | | | | | | | |
|---|---|---|---|---|---|---|---|---|
| OAS1, IFI44L, ISG15, HERC6, SLC1A2, IL1R2, OLAH, FAM20A | 0,862 | 0,851 | 0,873 | 0,912 | 0,858 | 0,966 | 0,924 | 0,839 | 1 |
| RSAD2, OAS1, IFIT1, IFI44L, SIGLEC1, HERC6, IL1R2, RETN | 0,862 | 0,853 | 0,872 | 0,904 | 0,841 | 0,966 | 0,955 | 0,911 | 0,999 |
| IFI27, OAS1, IFIT1, IFI44L, SIGLEC1, HERC6, SLC1A2, MMP8 | 0,862 | 0,854 | 0,871 | 0,912 | 0,859 | 0,965 | 0,917 | 0,847 | 0,988 |
| RSAD2, OAS1, IFI44L, SIGLEC1, HERC6, OLAH, FAM20A, RETN | 0,862 | 0,852 | 0,872 | 0,907 | 0,845 | 0,970 | 0,949 | 0,892 | 1 |
| RSAD2, IFIT1, IFI44L, SIGLEC1, ISG15, SLC1A2, FAM20A, RETN | 0,862 | 0,853 | 0,872 | 0,913 | 0,854 | 0,972 | 0,936 | 0,863 | 1 |
| RSAD2, OAS1, IFIT1, SIGLEC1, ISG15, HERC6, IL1R2, RETN | 0,862 | 0,853 | 0,872 | 0,905 | 0,845 | 0,966 | 0,958 | 0,914 | 1 |
| RSAD2, OAS1, HERC6, SLC1A2, IL1R2, OLAH, FAM20A, MMP8 | 0,862 | 0,853 | 0,872 | 0,914 | 0,862 | 0,966 | 0,927 | 0,840 | 1 |
| OAS1, IFIT1, SIGLEC1, ISG15, HERC6, OLAH, RETN, MMP8 | 0,862 | 0,852 | 0,872 | 0,904 | 0,841 | 0,966 | 0,959 | 0,918 | 0,999 |
| OAS1, IFIT1, IFI44L, SIGLEC1, HERC6, SLC1A2, OLAH, RETN | 0,862 | 0,852 | 0,872 | 0,915 | 0,860 | 0,969 | 0,937 | 0,881 | 0,993 |
| OAS1, IFIT1, HERC6, SLC1A2, IL1R2, OLAH, FAM20A, MMP8 | 0,862 | 0,852 | 0,872 | 0,916 | 0,865 | 0,968 | 0,932 | 0,850 | 1 |
| RSAD2, OAS1, IFIT1, ISG15, HERC6, IL1R2, OLAH, FAM20A | 0,862 | 0,852 | 0,872 | 0,907 | 0,847 | 0,966 | 0,937 | 0,862 | 1 |
| OAS1, IFIT1, SIGLEC1, HERC6, SLC1A2, IL1R2, RETN, MMP8 | 0,862 | 0,853 | 0,872 | 0,911 | 0,856 | 0,966 | 0,963 | 0,925 | 1 |
| RSAD2, IFIT1, IFI44L, ISG15, HERC6, SLC1A2, OLAH, FAM20A | 0,862 | 0,852 | 0,872 | 0,916 | 0,863 | 0,970 | 0,916 | 0,831 | 1 |
| RSAD2, OAS1, IFIT1, SIGLEC1, ISG15, SLC1A2, FAM20A, RETN | 0,862 | 0,852 | 0,872 | 0,910 | 0,849 | 0,970 | 0,945 | 0,873 | 1 |
| IFIT1, ISG15, HERC6, IL1R2, OLAH, FAM20A, RETN, MMP8 | 0,862 | 0,852 | 0,872 | 0,909 | 0,848 | 0,969 | 0,943 | 0,871 | 1 |
| IFI44L, ISG15, HERC6, SLC1A2, OLAH, FAM20A, RETN, MMP8 | 0,862 | 0,852 | 0,872 | 0,914 | 0,857 | 0,971 | 0,935 | 0,858 | 1 |
| IFIT1, IFI44L, ISG15, HERC6, OLAH, FAM20A, RETN, MMP8 | 0,862 | 0,852 | 0,872 | 0,908 | 0,845 | 0,972 | 0,943 | 0,876 | 1 |
| IFIT1, IFI44L, SIGLEC1, ISG15, HERC6, SLC1A2, OLAH, MMP8 | 0,862 | 0,852 | 0,872 | 0,913 | 0,858 | 0,968 | 0,941 | 0,889 | 0,993 |
| RSAD2, IFI27, IFIT1, IFI44L, ISG15, SLC1A2, FAM20A, RETN | 0,862 | 0,852 | 0,872 | 0,910 | 0,848 | 0,973 | 0,939 | 0,859 | 1 |
| RSAD2, OAS1, IFIT1, SLC1A2, IL1R2, OLAH, FAM20A, MMP8 | 0,862 | 0,853 | 0,871 | 0,917 | 0,865 | 0,969 | 0,935 | 0,855 | 1 |
| OAS1, IFIT1, IFI44L, ISG15, HERC6, IL1R2, OLAH, FAM20A | 0,862 | 0,852 | 0,872 | 0,909 | 0,850 | 0,967 | 0,932 | 0,853 | 1 |
| RSAD2, IFIT1, ISG15, HERC6, IL1R2, FAM20A, RETN, MMP8 | 0,862 | 0,852 | 0,872 | 0,911 | 0,851 | 0,970 | 0,937 | 0,859 | 1 |
| OAS1, IFIT1, IFI44L, HERC6, SLC1A2, OLAH, FAM20A, RETN | 0,862 | 0,852 | 0,872 | 0,917 | 0,863 | 0,970 | 0,913 | 0,826 | 1 |
| OAS1, SIGLEC1, HERC6, SLC1A2, IL1R2, OLAH, RETN, MMP8 | 0,862 | 0,852 | 0,872 | 0,915 | 0,860 | 0,969 | 0,962 | 0,923 | 1 |
| RSAD2, OAS1, ISG15, HERC6, IL1R2, OLAH, FAM20A, RETN | 0,862 | 0,851 | 0,872 | 0,906 | 0,845 | 0,966 | 0,937 | 0,862 | 1 |
| RSAD2, OAS1, IFIT1, HERC6, IL1R2, FAM20A, RETN, MMP8 | 0,862 | 0,852 | 0,872 | 0,908 | 0,847 | 0,970 | 0,941 | 0,862 | 1 |
| IFIT1, IFI44L, SIGLEC1, HERC6, IL1R2, OLAH, RETN, MMP8 | 0,862 | 0,852 | 0,872 | 0,904 | 0,841 | 0,967 | 0,964 | 0,926 | 1 |
| RSAD2, IFI27, IFIT1, ISG15, HERC6, SLC1A2, RETN, MMP8 | 0,862 | 0,852 | 0,872 | 0,915 | 0,859 | 0,972 | 0,936 | 0,868 | 1 |

| | | | | | | | | |
|---|---|---|---|---|---|---|---|---|
| IFI27, IFIT1, IFI44L, ISG15, SLC1A2, FAM20A, RETN, MMP8 | 0,862 | 0,851 | 0,873 | 0,913 | 0,852 | 0,975 | 0,940 | 0,861 | 1 |
| RSAD2, IFIT1, HERC6, SLC1A2, IL1R2, OLAH, FAM20A, MMP8 | 0,862 | 0,852 | 0,872 | 0,916 | 0,865 | 0,968 | 0,932 | 0,850 | 1 |
| OAS1, IFIT1, ISG15, HERC6, IL1R2, OLAH, FAM20A, RETN | 0,862 | 0,852 | 0,872 | 0,906 | 0,845 | 0,966 | 0,936 | 0,860 | 1 |
| RSAD2, OAS1, IFIT1, ISG15, HERC6, IL1R2, FAM20A, RETN | 0,862 | 0,852 | 0,872 | 0,907 | 0,848 | 0,967 | 0,934 | 0,851 | 1 |
| RSAD2, IFIT1, IFI44L, HERC6, IL1R2, OLAH, FAM20A, MMP8 | 0,862 | 0,852 | 0,872 | 0,910 | 0,851 | 0,968 | 0,933 | 0,854 | 1 |
| OAS1, IFIT1, SIGLEC1, HERC6, SLC1A2, IL1R2, OLAH, MMP8 | 0,862 | 0,852 | 0,872 | 0,913 | 0,860 | 0,966 | 0,961 | 0,922 | 1 |
| RSAD2, IFI27, OAS1, IFI44L, ISG15, SLC1A2, FAM20A, RETN | 0,862 | 0,851 | 0,872 | 0,911 | 0,850 | 0,973 | 0,940 | 0,861 | 1 |
| IFI27, IFIT1, IFI44L, ISG15, HERC6, SLC1A2, RETN, MMP8 | 0,862 | 0,852 | 0,871 | 0,912 | 0,854 | 0,971 | 0,928 | 0,855 | 1 |
| RSAD2, IFIT1, ISG15, HERC6, IL1R2, OLAH, FAM20A, MMP8 | 0,862 | 0,852 | 0,872 | 0,907 | 0,848 | 0,966 | 0,945 | 0,874 | 1 |
| IFIT1, SIGLEC1, ISG15, HERC6, SLC1A2, IL1R2, OLAH, MMP8 | 0,862 | 0,852 | 0,871 | 0,913 | 0,860 | 0,966 | 0,961 | 0,922 | 1,000 |
| OAS1, IFI44L, SIGLEC1, ISG15, HERC6, IL1R2, RETN, MMP8 | 0,862 | 0,852 | 0,871 | 0,904 | 0,842 | 0,966 | 0,963 | 0,925 | 1 |
| RSAD2, OAS1, SIGLEC1, HERC6, IL1R2, OLAH, RETN, MMP8 | 0,862 | 0,851 | 0,872 | 0,905 | 0,843 | 0,967 | 0,965 | 0,929 | 1 |
| RSAD2, IFIT1, IFI44L, HERC6, SLC1A2, IL1R2, OLAH, FAM20A | 0,862 | 0,852 | 0,872 | 0,918 | 0,866 | 0,969 | 0,916 | 0,828 | 1 |
| RSAD2, IFI27, IFIT1, IFI44L, ISG15, HERC6, SLC1A2, FAM20A | 0,862 | 0,851 | 0,872 | 0,913 | 0,855 | 0,970 | 0,918 | 0,833 | 1 |
| IFIT1, IFI44L, ISG15, SLC1A2, OLAH, FAM20A, RETN, MMP8 | 0,862 | 0,852 | 0,871 | 0,913 | 0,856 | 0,970 | 0,924 | 0,848 | 1,000 |
| OAS1, IFIT1, SIGLEC1, ISG15, HERC6, IL1R2, OLAH, RETN | 0,862 | 0,852 | 0,871 | 0,906 | 0,846 | 0,966 | 0,952 | 0,907 | 0,997 |
| IFI27, IFIT1, IFI44L, SIGLEC1, ISG15, SLC1A2, RETN, MMP8 | 0,862 | 0,852 | 0,871 | 0,914 | 0,858 | 0,971 | 0,950 | 0,891 | 1 |
| RSAD2, OAS1, SIGLEC1, ISG15, HERC6, IL1R2, RETN, MMP8 | 0,862 | 0,851 | 0,872 | 0,907 | 0,846 | 0,967 | 0,970 | 0,936 | 1 |
| RSAD2, IFIT1, IFI44L, ISG15, HERC6, IL1R2, OLAH, FAM20A | 0,861 | 0,852 | 0,871 | 0,910 | 0,851 | 0,968 | 0,930 | 0,853 | 1 |
| RSAD2, IFI27, OAS1, SIGLEC1, ISG15, HERC6, SLC1A2, MMP8 | 0,861 | 0,852 | 0,871 | 0,913 | 0,859 | 0,966 | 0,927 | 0,858 | 0,996 |
| RSAD2, OAS1, IFIT1, IFI44L, SLC1A2, OLAH, FAM20A, MMP8 | 0,861 | 0,852 | 0,871 | 0,915 | 0,860 | 0,971 | 0,923 | 0,847 | 0,999 |
| RSAD2, IFI27, OAS1, IFIT1, IFI44L, SLC1A2, FAM20A, RETN | 0,861 | 0,851 | 0,872 | 0,910 | 0,848 | 0,972 | 0,940 | 0,861 | 1 |
| IFIT1, IFI44L, SIGLEC1, ISG15, HERC6, OLAH, RETN, MMP8 | 0,861 | 0,851 | 0,871 | 0,905 | 0,841 | 0,968 | 0,959 | 0,918 | 0,999 |
| OAS1, SIGLEC1, ISG15, HERC6, SLC1A2, IL1R2, OLAH, MMP8 | 0,861 | 0,852 | 0,871 | 0,912 | 0,859 | 0,965 | 0,961 | 0,922 | 1 |
| OAS1, SIGLEC1, ISG15, HERC6, SLC1A2, OLAH, RETN, MMP8 | 0,861 | 0,851 | 0,872 | 0,915 | 0,859 | 0,970 | 0,962 | 0,921 | 1 |
| RSAD2, OAS1, IFI44L, SIGLEC1, ISG15, HERC6, SLC1A2, FAM20A | 0,861 | 0,851 | 0,871 | 0,910 | 0,854 | 0,967 | 0,940 | 0,866 | 1 |
| RSAD2, IFIT1, SIGLEC1, ISG15, SLC1A2, FAM20A, RETN, MMP8 | 0,861 | 0,851 | 0,871 | 0,913 | 0,854 | 0,971 | 0,950 | 0,881 | 1 |
| OAS1, IFI44L, SIGLEC1, ISG15, HERC6, SLC1A2, FAM20A, RETN | 0,861 | 0,851 | 0,872 | 0,911 | 0,853 | 0,968 | 0,946 | 0,876 | 1 |

| | | | | | | | | | |
|---|---|---|---|---|---|---|---|---|---|
| IFIT1, IFI44L, SLC1A2, IL1R2, OLAH, FAM20A, RETN, MMP8 | 0,861 | 0,852 | 0,871 | 0,916 | 0,862 | 0,969 | 0,934 | 0,857 | 1 |
| RSAD2, IFI27, IFI44L, ISG15, HERC6, SLC1A2, FAM20A, MMP8 | 0,861 | 0,850 | 0,873 | 0,913 | 0,856 | 0,970 | 0,925 | 0,842 | 1 |
| RSAD2, IFI44L, SIGLEC1, ISG15, OLAH, FAM20A, RETN, MMP8 | 0,861 | 0,851 | 0,871 | 0,908 | 0,844 | 0,972 | 0,963 | 0,920 | 1 |
| RSAD2, IFI44L, ISG15, HERC6, SLC1A2, IL1R2, OLAH, FAM20A | 0,861 | 0,850 | 0,872 | 0,912 | 0,858 | 0,967 | 0,925 | 0,841 | 1 |
| IFIT1, ISG15, HERC6, SLC1A2, IL1R2, OLAH, FAM20A, MMP8 | 0,861 | 0,851 | 0,871 | 0,916 | 0,864 | 0,967 | 0,932 | 0,850 | 1 |
| OAS1, IFI44L, ISG15, HERC6, IL1R2, FAM20A, RETN, MMP8 | 0,861 | 0,851 | 0,871 | 0,910 | 0,850 | 0,970 | 0,936 | 0,856 | 1 |
| RSAD2, IFIT1, IFI44L, SIGLEC1, HERC6, IL1R2, RETN, MMP8 | 0,861 | 0,852 | 0,871 | 0,903 | 0,840 | 0,966 | 0,964 | 0,926 | 1 |
| OAS1, IFIT1, IFI44L, SIGLEC1, ISG15, HERC6, OLAH, RETN | 0,861 | 0,852 | 0,871 | 0,905 | 0,844 | 0,966 | 0,942 | 0,891 | 0,994 |
| RSAD2, IFI44L, SIGLEC1, ISG15, HERC6, SLC1A2, FAM20A, RETN | 0,861 | 0,851 | 0,872 | 0,912 | 0,855 | 0,970 | 0,939 | 0,865 | 1 |
| RSAD2, IFI27, IFI44L, ISG15, HERC6, SLC1A2, RETN, MMP8 | 0,861 | 0,852 | 0,871 | 0,915 | 0,859 | 0,972 | 0,937 | 0,870 | 1 |
| OAS1, IFIT1, SIGLEC1, ISG15, HERC6, SLC1A2, OLAH, RETN | 0,861 | 0,851 | 0,871 | 0,915 | 0,860 | 0,969 | 0,942 | 0,890 | 0,995 |
| RSAD2, IFIT1, SIGLEC1, HERC6, SLC1A2, IL1R2, OLAH, MMP8 | 0,861 | 0,852 | 0,871 | 0,912 | 0,859 | 0,966 | 0,962 | 0,924 | 1 |
| OAS1, IFIT1, IFI44L, HERC6, SLC1A2, IL1R2, OLAH, FAM20A | 0,861 | 0,851 | 0,872 | 0,916 | 0,863 | 0,968 | 0,917 | 0,830 | 1 |
| OAS1, IFIT1, IFI44L, SLC1A2, IL1R2, OLAH, FAM20A, MMP8 | 0,861 | 0,851 | 0,871 | 0,917 | 0,866 | 0,969 | 0,929 | 0,850 | 1 |
| OAS1, IFIT1, IFI44L, SIGLEC1, HERC6, SLC1A2, OLAH, MMP8 | 0,861 | 0,851 | 0,871 | 0,913 | 0,858 | 0,967 | 0,940 | 0,888 | 0,993 |
| OAS1, IFI44L, ISG15, SLC1A2, IL1R2, OLAH, FAM20A, MMP8 | 0,861 | 0,851 | 0,871 | 0,916 | 0,863 | 0,968 | 0,926 | 0,843 | 1 |
| RSAD2, OAS1, IFIT1, ISG15, IL1R2, OLAH, FAM20A, MMP8 | 0,861 | 0,851 | 0,871 | 0,908 | 0,846 | 0,970 | 0,945 | 0,873 | 1 |
| RSAD2, IFI27, OAS1, IFI44L, SIGLEC1, HERC6, SLC1A2, MMP8 | 0,861 | 0,852 | 0,870 | 0,914 | 0,861 | 0,967 | 0,928 | 0,858 | 0,998 |
| IFIT1, SIGLEC1, ISG15, HERC6, SLC1A2, OLAH, RETN, MMP8 | 0,861 | 0,851 | 0,871 | 0,914 | 0,859 | 0,969 | 0,952 | 0,907 | 0,997 |
| OAS1, IFI44L, HERC6, SLC1A2, OLAH, FAM20A, RETN, MMP8 | 0,861 | 0,851 | 0,871 | 0,918 | 0,863 | 0,973 | 0,929 | 0,851 | 1 |
| RSAD2, IFI44L, HERC6, SLC1A2, OLAH, FAM20A, RETN, MMP8 | 0,861 | 0,851 | 0,871 | 0,917 | 0,862 | 0,971 | 0,925 | 0,845 | 1 |
| IFI44L, ISG15, HERC6, SLC1A2, IL1R2, OLAH, FAM20A, RETN | 0,861 | 0,851 | 0,871 | 0,914 | 0,860 | 0,968 | 0,929 | 0,846 | 1 |
| RSAD2, OAS1, IFIT1, SIGLEC1, HERC6, SLC1A2, OLAH, MMP8 | 0,861 | 0,851 | 0,871 | 0,912 | 0,857 | 0,967 | 0,951 | 0,905 | 0,998 |
| RSAD2, OAS1, IFI44L, HERC6, SLC1A2, IL1R2, OLAH, FAM20A | 0,861 | 0,850 | 0,872 | 0,915 | 0,862 | 0,968 | 0,923 | 0,838 | 1 |
| IFI27, OAS1, IFIT1, IFI44L, SLC1A2, FAM20A, RETN, MMP8 | 0,861 | 0,850 | 0,872 | 0,913 | 0,852 | 0,974 | 0,939 | 0,859 | 1 |
| RSAD2, IFI44L, ISG15, HERC6, IL1R2, OLAH, FAM20A, RETN | 0,861 | 0,851 | 0,871 | 0,910 | 0,850 | 0,969 | 0,938 | 0,867 | 1 |
| RSAD2, OAS1, ISG15, HERC6, SLC1A2, IL1R2, OLAH, FAM20A | 0,861 | 0,850 | 0,872 | 0,912 | 0,858 | 0,966 | 0,927 | 0,841 | 1 |
| OAS1, IFIT1, IFI44L, ISG15, HERC6, IL1R2, FAM20A, RETN | 0,861 | 0,851 | 0,871 | 0,911 | 0,852 | 0,969 | 0,935 | 0,856 | 1 |

| | | | | | | | | |
|---|---|---|---|---|---|---|---|---|
| OAS1, SIGLEC1, ISG15, HERC6, IL1R2, OLAH, RETN, MMP8 | 0,861 | 0,851 | 0,871 | 0,906 | 0,845 | 0,967 | 0,966 | 0,930 | 1 |
| IFIT1, HERC6, SLC1A2, IL1R2, OLAH, FAM20A, RETN, MMP8 | 0,861 | 0,851 | 0,871 | 0,915 | 0,862 | 0,969 | 0,934 | 0,853 | 1 |
| RSAD2, IFI27, OAS1, IFI44L, HERC6, SLC1A2, FAM20A, MMP8 | 0,861 | 0,850 | 0,872 | 0,912 | 0,856 | 0,969 | 0,925 | 0,842 | 1 |
| OAS1, IFI44L, ISG15, HERC6, SLC1A2, OLAH, FAM20A, MMP8 | 0,861 | 0,850 | 0,872 | 0,914 | 0,858 | 0,969 | 0,927 | 0,850 | 1 |
| OAS1, IFIT1, IFI44L, HERC6, IL1R2, FAM20A, RETN, MMP8 | 0,861 | 0,851 | 0,871 | 0,909 | 0,848 | 0,970 | 0,940 | 0,861 | 1 |
| RSAD2, OAS1, IFIT1, IFI44L, SIGLEC1, SLC1A2, FAM20A, RETN | 0,861 | 0,851 | 0,871 | 0,914 | 0,855 | 0,972 | 0,936 | 0,863 | 1 |
| RSAD2, OAS1, IFIT1, SIGLEC1, HERC6, SLC1A2, IL1R2, OLAH | 0,861 | 0,851 | 0,871 | 0,913 | 0,859 | 0,967 | 0,961 | 0,921 | 1 |
| OAS1, IFIT1, IFI44L, ISG15, HERC6, OLAH, FAM20A, RETN | 0,861 | 0,851 | 0,871 | 0,906 | 0,845 | 0,967 | 0,933 | 0,863 | 1 |
| RSAD2, OAS1, SIGLEC1, HERC6, SLC1A2, OLAH, RETN, MMP8 | 0,861 | 0,851 | 0,871 | 0,912 | 0,855 | 0,970 | 0,962 | 0,921 | 1 |
| RSAD2, ISG15, HERC6, SLC1A2, IL1R2, OLAH, FAM20A, MMP8 | 0,861 | 0,851 | 0,870 | 0,914 | 0,861 | 0,966 | 0,926 | 0,837 | 1 |
| RSAD2, IFI44L, ISG15, HERC6, SLC1A2, OLAH, FAM20A, MMP8 | 0,861 | 0,850 | 0,871 | 0,916 | 0,862 | 0,970 | 0,922 | 0,843 | 1 |
| RSAD2, OAS1, IFIT1, SIGLEC1, HERC6, SLC1A2, OLAH, RETN | 0,861 | 0,851 | 0,871 | 0,912 | 0,856 | 0,969 | 0,942 | 0,887 | 0,998 |
| RSAD2, IFIT1, SIGLEC1, ISG15, HERC6, IL1R2, RETN, MMP8 | 0,861 | 0,851 | 0,870 | 0,906 | 0,844 | 0,967 | 0,963 | 0,925 | 1 |
| RSAD2, OAS1, IFI44L, ISG15, HERC6, IL1R2, FAM20A, RETN | 0,861 | 0,850 | 0,871 | 0,911 | 0,853 | 0,970 | 0,936 | 0,860 | 1 |
| OAS1, ISG15, HERC6, SLC1A2, IL1R2, OLAH, FAM20A, MMP8 | 0,861 | 0,850 | 0,871 | 0,914 | 0,863 | 0,966 | 0,927 | 0,841 | 1 |
| OAS1, IFI44L, HERC6, IL1R2, OLAH, FAM20A, RETN, MMP8 | 0,861 | 0,851 | 0,871 | 0,913 | 0,852 | 0,973 | 0,945 | 0,872 | 1 |
| RSAD2, SIGLEC1, ISG15, HERC6, SLC1A2, IL1R2, OLAH, MMP8 | 0,861 | 0,851 | 0,870 | 0,912 | 0,859 | 0,966 | 0,962 | 0,924 | 1 |
| RSAD2, OAS1, IFI44L, ISG15, IL1R2, OLAH, FAM20A, MMP8 | 0,861 | 0,850 | 0,871 | 0,907 | 0,846 | 0,969 | 0,942 | 0,873 | 1 |
| RSAD2, OAS1, SIGLEC1, HERC6, SLC1A2, IL1R2, OLAH, MMP8 | 0,861 | 0,851 | 0,871 | 0,912 | 0,859 | 0,966 | 0,963 | 0,925 | 1 |
| RSAD2, IFIT1, IFI44L, ISG15, SLC1A2, OLAH, FAM20A, MMP8 | 0,861 | 0,851 | 0,870 | 0,912 | 0,855 | 0,970 | 0,922 | 0,845 | 0,998 |
| IFIT1, IFI44L, HERC6, SLC1A2, IL1R2, OLAH, FAM20A, RETN | 0,861 | 0,850 | 0,871 | 0,917 | 0,864 | 0,969 | 0,920 | 0,833 | 1 |
| RSAD2, IFI27, IFIT1, IFI44L, SLC1A2, FAM20A, RETN, MMP8 | 0,861 | 0,850 | 0,871 | 0,915 | 0,854 | 0,975 | 0,940 | 0,861 | 1 |
| OAS1, IFIT1, SIGLEC1, ISG15, HERC6, SLC1A2, IL1R2, RETN | 0,861 | 0,851 | 0,871 | 0,912 | 0,859 | 0,966 | 0,953 | 0,907 | 0,999 |
| OAS1, SIGLEC1, ISG15, HERC6, SLC1A2, IL1R2, RETN, MMP8 | 0,861 | 0,851 | 0,870 | 0,912 | 0,858 | 0,967 | 0,965 | 0,927 | 1 |
| RSAD2, HERC6, SLC1A2, IL1R2, OLAH, FAM20A, RETN, MMP8 | 0,861 | 0,851 | 0,871 | 0,914 | 0,861 | 0,967 | 0,928 | 0,842 | 1 |
| RSAD2, IFI44L, ISG15, SLC1A2, IL1R2, OLAH, FAM20A, MMP8 | 0,861 | 0,850 | 0,871 | 0,917 | 0,865 | 0,969 | 0,925 | 0,841 | 1 |
| RSAD2, IFI27, IFIT1, SIGLEC1, ISG15, SLC1A2, RETN, MMP8 | 0,861 | 0,851 | 0,870 | 0,915 | 0,859 | 0,971 | 0,953 | 0,896 | 1 |
| OAS1, IFI44L, SIGLEC1, HERC6, IL1R2, OLAH, RETN, MMP8 | 0,861 | 0,850 | 0,871 | 0,903 | 0,840 | 0,966 | 0,964 | 0,926 | 1 |

| | | | | | | | | |
|---|---|---|---|---|---|---|---|---|
| OAS1, IFIT1, IFI44L, SIGLEC1, ISG15, HERC6, SLC1A2, OLAH | 0,861 | 0,850 | 0,871 | 0,913 | 0,858 | 0,968 | 0,941 | 0,889 | 0,993 |
| RSAD2, OAS1, IFI44L, ISG15, HERC6, SLC1A2, OLAH, FAM20A | 0,861 | 0,850 | 0,871 | 0,913 | 0,857 | 0,968 | 0,927 | 0,848 | 1 |
| RSAD2, IFI27, OAS1, IFI44L, ISG15, HERC6, SLC1A2, FAM20A | 0,861 | 0,849 | 0,872 | 0,913 | 0,855 | 0,971 | 0,926 | 0,844 | 1 |
| RSAD2, SIGLEC1, HERC6, SLC1A2, IL1R2, OLAH, RETN, MMP8 | 0,861 | 0,851 | 0,870 | 0,916 | 0,862 | 0,970 | 0,963 | 0,925 | 1 |
| OAS1, IFIT1, IFI44L, ISG15, SLC1A2, OLAH, FAM20A, MMP8 | 0,860 | 0,850 | 0,871 | 0,913 | 0,856 | 0,970 | 0,922 | 0,844 | 0,999 |
| RSAD2, OAS1, IFIT1, IFI44L, HERC6, OLAH, FAM20A, MMP8 | 0,860 | 0,850 | 0,871 | 0,905 | 0,843 | 0,966 | 0,933 | 0,863 | 1 |
| RSAD2, IFIT1, SIGLEC1, HERC6, IL1R2, OLAH, RETN, MMP8 | 0,860 | 0,850 | 0,870 | 0,904 | 0,842 | 0,967 | 0,963 | 0,925 | 1 |
| RSAD2, OAS1, IFI44L, SIGLEC1, HERC6, IL1R2, RETN, MMP8 | 0,860 | 0,850 | 0,871 | 0,903 | 0,839 | 0,966 | 0,966 | 0,930 | 1 |
| RSAD2, OAS1, SIGLEC1, ISG15, HERC6, SLC1A2, IL1R2, OLAH | 0,860 | 0,850 | 0,870 | 0,912 | 0,858 | 0,966 | 0,963 | 0,924 | 1 |
| OAS1, IFIT1, SIGLEC1, ISG15, HERC6, SLC1A2, IL1R2, OLAH | 0,860 | 0,850 | 0,870 | 0,912 | 0,858 | 0,966 | 0,954 | 0,911 | 0,998 |
| RSAD2, OAS1, IFIT1, IFI44L, SIGLEC1, HERC6, SLC1A2, OLAH | 0,860 | 0,850 | 0,870 | 0,912 | 0,857 | 0,967 | 0,951 | 0,905 | 0,998 |
| RSAD2, IFI44L, ISG15, HERC6, IL1R2, FAM20A, RETN, MMP8 | 0,860 | 0,850 | 0,871 | 0,914 | 0,856 | 0,972 | 0,932 | 0,852 | 1 |
| RSAD2, IFIT1, IFI44L, SIGLEC1, HERC6, SLC1A2, OLAH, MMP8 | 0,860 | 0,851 | 0,870 | 0,913 | 0,858 | 0,968 | 0,940 | 0,888 | 0,993 |
| OAS1, IFIT1, SIGLEC1, HERC6, SLC1A2, IL1R2, OLAH, RETN | 0,860 | 0,850 | 0,871 | 0,916 | 0,863 | 0,969 | 0,943 | 0,893 | 0,994 |
| OAS1, IFIT1, IFI44L, ISG15, HERC6, IL1R2, FAM20A, MMP8 | 0,860 | 0,850 | 0,870 | 0,910 | 0,850 | 0,969 | 0,933 | 0,850 | 1 |
| OAS1, IFI44L, ISG15, HERC6, IL1R2, OLAH, FAM20A, RETN | 0,860 | 0,850 | 0,871 | 0,906 | 0,846 | 0,967 | 0,939 | 0,866 | 1 |
| RSAD2, IFI27, IFI44L, ISG15, SLC1A2, FAM20A, RETN, MMP8 | 0,860 | 0,849 | 0,871 | 0,911 | 0,849 | 0,974 | 0,939 | 0,860 | 1 |
| OAS1, IFIT1, ISG15, HERC6, SLC1A2, IL1R2, OLAH, FAM20A | 0,860 | 0,850 | 0,871 | 0,912 | 0,858 | 0,967 | 0,928 | 0,844 | 1 |
| RSAD2, OAS1, IFIT1, SIGLEC1, HERC6, IL1R2, OLAH, RETN | 0,860 | 0,850 | 0,870 | 0,904 | 0,841 | 0,967 | 0,951 | 0,905 | 0,997 |
| RSAD2, OAS1, IFIT1, IFI44L, SIGLEC1, HERC6, IL1R2, MMP8 | 0,860 | 0,851 | 0,870 | 0,904 | 0,845 | 0,963 | 0,961 | 0,921 | 1 |
| RSAD2, IFIT1, IFI44L, ISG15, HERC6, IL1R2, FAM20A, MMP8 | 0,860 | 0,850 | 0,870 | 0,911 | 0,854 | 0,969 | 0,932 | 0,850 | 1 |
| IFIT1, IFI44L, SIGLEC1, ISG15, HERC6, SLC1A2, OLAH, RETN | 0,860 | 0,851 | 0,870 | 0,915 | 0,860 | 0,970 | 0,941 | 0,890 | 0,993 |
| IFI27, OAS1, IFI44L, ISG15, HERC6, SLC1A2, FAM20A, MMP8 | 0,860 | 0,849 | 0,871 | 0,911 | 0,854 | 0,968 | 0,927 | 0,845 | 1 |
| RSAD2, OAS1, IFI44L, HERC6, IL1R2, FAM20A, RETN, MMP8 | 0,860 | 0,850 | 0,871 | 0,911 | 0,851 | 0,972 | 0,936 | 0,856 | 1 |
| IFI27, OAS1, IFI44L, ISG15, SLC1A2, FAM20A, RETN, MMP8 | 0,860 | 0,849 | 0,872 | 0,913 | 0,852 | 0,974 | 0,939 | 0,859 | 1 |
| OAS1, IFIT1, ISG15, SLC1A2, IL1R2, OLAH, FAM20A, MMP8 | 0,860 | 0,851 | 0,870 | 0,917 | 0,865 | 0,969 | 0,936 | 0,858 | 1 |
| RSAD2, OAS1, IFIT1, SIGLEC1, HERC6, SLC1A2, IL1R2, RETN | 0,860 | 0,851 | 0,870 | 0,912 | 0,857 | 0,967 | 0,957 | 0,911 | 1 |
| RSAD2, OAS1, IFIT1, HERC6, SLC1A2, IL1R2, FAM20A, MMP8 | 0,860 | 0,850 | 0,870 | 0,916 | 0,864 | 0,969 | 0,929 | 0,842 | 1 |

| | | | | | | | | |
|---|---|---|---|---|---|---|---|---|
| RSAD2, IFIT1, ISG15, HERC6, IL1R2, OLAH, FAM20A, RETN | 0,860 | 0,850 | 0,870 | 0,906 | 0,846 | 0,966 | 0,937 | 0,862 | 1 |
| RSAD2, IFI27, IFIT1, IFI44L, HERC6, SLC1A2, RETN, MMP8 | 0,860 | 0,851 | 0,870 | 0,912 | 0,853 | 0,971 | 0,930 | 0,860 | 1 |
| OAS1, IFIT1, SIGLEC1, ISG15, HERC6, SLC1A2, OLAH, MMP8 | 0,860 | 0,850 | 0,870 | 0,914 | 0,859 | 0,968 | 0,947 | 0,898 | 0,995 |
| IFI44L, SIGLEC1, ISG15, HERC6, IL1R2, OLAH, RETN, MMP8 | 0,860 | 0,850 | 0,870 | 0,902 | 0,839 | 0,965 | 0,964 | 0,926 | 1 |
| OAS1, IFIT1, IFI44L, SIGLEC1, HERC6, IL1R2, OLAH, MMP8 | 0,860 | 0,851 | 0,869 | 0,907 | 0,849 | 0,964 | 0,962 | 0,922 | 1 |
| RSAD2, IFIT1, SIGLEC1, HERC6, SLC1A2, OLAH, RETN, MMP8 | 0,860 | 0,850 | 0,870 | 0,913 | 0,858 | 0,968 | 0,950 | 0,904 | 0,996 |
| RSAD2, OAS1, IFI44L, SIGLEC1, HERC6, SLC1A2, FAM20A, MMP8 | 0,860 | 0,850 | 0,870 | 0,908 | 0,851 | 0,966 | 0,937 | 0,860 | 1 |
| RSAD2, SIGLEC1, ISG15, HERC6, IL1R2, OLAH, RETN, MMP8 | 0,860 | 0,850 | 0,870 | 0,907 | 0,846 | 0,969 | 0,961 | 0,921 | 1 |
| OAS1, IFIT1, IFI44L, HERC6, OLAH, FAM20A, RETN, MMP8 | 0,860 | 0,850 | 0,870 | 0,905 | 0,841 | 0,969 | 0,941 | 0,874 | 1 |
| RSAD2, OAS1, IFI44L, ISG15, SLC1A2, OLAH, FAM20A, MMP8 | 0,860 | 0,849 | 0,871 | 0,915 | 0,859 | 0,971 | 0,923 | 0,846 | 1,000 |
| OAS1, SIGLEC1, ISG15, HERC6, SLC1A2, IL1R2, OLAH, RETN | 0,860 | 0,849 | 0,871 | 0,914 | 0,860 | 0,968 | 0,957 | 0,912 | 1 |
| OAS1, ISG15, HERC6, SLC1A2, IL1R2, FAM20A, RETN, MMP8 | 0,860 | 0,850 | 0,870 | 0,918 | 0,865 | 0,970 | 0,927 | 0,838 | 1 |
| RSAD2, IFI44L, SIGLEC1, ISG15, HERC6, SLC1A2, FAM20A, MMP8 | 0,860 | 0,850 | 0,870 | 0,911 | 0,854 | 0,968 | 0,937 | 0,860 | 1 |
| RSAD2, IFIT1, HERC6, IL1R2, OLAH, FAM20A, RETN, MMP8 | 0,860 | 0,850 | 0,870 | 0,908 | 0,846 | 0,969 | 0,945 | 0,873 | 1 |
| RSAD2, OAS1, IFI44L, HERC6, SLC1A2, IL1R2, FAM20A, MMP8 | 0,860 | 0,850 | 0,870 | 0,919 | 0,869 | 0,968 | 0,924 | 0,830 | 1 |
| RSAD2, IFI27, OAS1, IFIT1, ISG15, SLC1A2, FAM20A, RETN | 0,860 | 0,850 | 0,870 | 0,908 | 0,847 | 0,970 | 0,939 | 0,858 | 1 |
| RSAD2, IFIT1, SIGLEC1, ISG15, HERC6, SLC1A2, IL1R2, OLAH | 0,860 | 0,851 | 0,869 | 0,913 | 0,859 | 0,967 | 0,953 | 0,909 | 0,997 |
| RSAD2, IFI27, OAS1, IFI44L, SLC1A2, FAM20A, RETN, MMP8 | 0,860 | 0,849 | 0,871 | 0,912 | 0,851 | 0,974 | 0,940 | 0,861 | 1 |
| ISG15, HERC6, SLC1A2, IL1R2, OLAH, FAM20A, RETN, MMP8 | 0,860 | 0,850 | 0,870 | 0,916 | 0,865 | 0,968 | 0,928 | 0,843 | 1 |
| OAS1, HERC6, SLC1A2, IL1R2, OLAH, FAM20A, RETN, MMP8 | 0,860 | 0,850 | 0,870 | 0,915 | 0,862 | 0,968 | 0,929 | 0,844 | 1 |
| RSAD2, OAS1, IFIT1, SIGLEC1, HERC6, OLAH, RETN, MMP8 | 0,860 | 0,850 | 0,870 | 0,901 | 0,836 | 0,966 | 0,953 | 0,909 | 0,997 |
| RSAD2, IFI27, IFIT1, IFI44L, SIGLEC1, SLC1A2, RETN, MMP8 | 0,860 | 0,850 | 0,869 | 0,915 | 0,859 | 0,971 | 0,952 | 0,895 | 1 |
| RSAD2, IFIT1, IFI44L, ISG15, HERC6, IL1R2, FAM20A, RETN | 0,860 | 0,850 | 0,870 | 0,912 | 0,855 | 0,970 | 0,934 | 0,855 | 1 |
| OAS1, ISG15, HERC6, SLC1A2, IL1R2, OLAH, FAM20A, RETN | 0,860 | 0,849 | 0,871 | 0,913 | 0,858 | 0,968 | 0,927 | 0,842 | 1 |
| IFIT1, IFI44L, ISG15, SLC1A2, IL1R2, OLAH, FAM20A, RETN | 0,860 | 0,850 | 0,870 | 0,913 | 0,858 | 0,968 | 0,926 | 0,846 | 1 |
| IFI44L, SIGLEC1, ISG15, SLC1A2, IL1R2, OLAH, RETN, MMP8 | 0,860 | 0,851 | 0,869 | 0,912 | 0,857 | 0,967 | 0,966 | 0,931 | 1 |
| RSAD2, IFI27, OAS1, IFI44L, SIGLEC1, ISG15, HERC6, MMP8 | 0,860 | 0,851 | 0,869 | 0,907 | 0,850 | 0,963 | 0,913 | 0,838 | 0,988 |
| IFI44L, ISG15, HERC6, SLC1A2, IL1R2, FAM20A, RETN, MMP8 | 0,860 | 0,850 | 0,869 | 0,916 | 0,863 | 0,969 | 0,928 | 0,837 | 1 |

| | | | | | | | | | |
|---|---|---|---|---|---|---|---|---|---|
| IFIT1, IFI44L, SIGLEC1, ISG15, HERC6, IL1R2, OLAH, RETN | 0,860 | 0,850 | 0,869 | 0,906 | 0,845 | 0,968 | 0,950 | 0,904 | 0,996 |
| IFIT1, IFI44L, SIGLEC1, ISG15, SLC1A2, OLAH. RETN, MMP8 | 0,860 | 0,850 | 0,869 | 0,913 | 0,857 | 0,970 | 0,955 | 0,911 | 1 |
| IFIT1, IFI44L, SIGLEC1, ISG15, HERC6, IL1R2, OLAH, MMP8 | 0,860 | 0,850 | 0,869 | 0,906 | 0,849 | 0,964 | 0,963 | 0,924 | 1 |
| RSAD2, OAS1, IFIT1, SIGLEC1, ISG15, HERC6, SLC1A2, OLAH | 0,860 | 0,850 | 0,870 | 0,912 | 0,857 | 0,967 | 0,951 | 0,905 | 0,998 |
| OAS1, IFIT1, IFI44L, HERC6, SLC1A2, IL1R2, FAM20A, MMP8 | 0,860 | 0,850 | 0,870 | 0,916 | 0,865 | 0,967 | 0,925 | 0,832 | 1 |
| RSAD2, OAS1, SIGLEC1, HERC6, SLC1A2, IL1R2, RETN, MMP8 | 0,860 | 0,850 | 0,869 | 0,912 | 0,856 | 0,967 | 0,970 | 0,935 | 1 |
| SIGLEC1, ISG15, HERC6, SLC1A2, IL1R2, OLAH, RETN, MMP8 | 0,860 | 0,850 | 0,869 | 0,912 | 0,856 | 0,968 | 0,965 | 0,929 | 1 |
| RSAD2, OAS1, IFIT1, IFI44L, ISG15, HERC6, OLAH, FAM20A | 0,860 | 0,849 | 0,870 | 0,907 | 0,846 | 0,967 | 0,929 | 0,858 | 1 |
| RSAD2, OAS1, SIGLEC1, ISG15, SLC1A2, FAM20A, RETN, MMP8 | 0,860 | 0,849 | 0,870 | 0,909 | 0,849 | 0,969 | 0,955 | 0,888 | 1 |
| RSAD2, IFI27, OAS1, IFIT1, IFI44L, HERC6, SLC1A2, FAM20A | 0,860 | 0,849 | 0,871 | 0,914 | 0,857 | 0,970 | 0,922 | 0,837 | 1 |
| RSAD2, IFI44L, HERC6, IL1R2, OLAH, FAM20A, RETN, MMP8 | 0,860 | 0,849 | 0,870 | 0,914 | 0,854 | 0,973 | 0,941 | 0,868 | 1 |
| IFIT1, IFI44L, SIGLEC1, HERC6, SLC1A2, IL1R2, OLAH, MMP8 | 0,860 | 0,850 | 0,869 | 0,912 | 0,858 | 0,965 | 0,962 | 0,923 | 1 |
| RSAD2, OAS1, IFIT1, IFI44L, SIGLEC1, HERC6, IL1R2, OLAH | 0,860 | 0,850 | 0,869 | 0,903 | 0,842 | 0,964 | 0,957 | 0,914 | 0,999 |
| IFIT1, IFI44L, SIGLEC1, ISG15, SLC1A2, IL1R2, OLAH, MMP8 | 0,860 | 0,851 | 0,868 | 0,912 | 0,858 | 0,965 | 0,966 | 0,931 | 1 |
| RSAD2, OAS1, IFI44L, ISG15, HERC6, SLC1A2, IL1R2, FAM20A | 0,860 | 0,849 | 0,870 | 0,915 | 0,863 | 0,966 | 0,925 | 0,835 | 1 |
| RSAD2, OAS1, SIGLEC1, ISG15, HERC6, SLC1A2, OLAH, MMP8 | 0,860 | 0,849 | 0,870 | 0,911 | 0,855 | 0,967 | 0,955 | 0,912 | 0,999 |
| IFIT1, SIGLEC1, ISG15, HERC6, SLC1A2, IL1R2, OLAH, RETN | 0,860 | 0,850 | 0,870 | 0,914 | 0,859 | 0,968 | 0,949 | 0,902 | 0,996 |
| IFIT1, IFI44L, HERC6, SLC1A2, IL1R2, FAM20A, RETN, MMP8 | 0,860 | 0,850 | 0,869 | 0,918 | 0,865 | 0,970 | 0,929 | 0,839 | 1 |
| RSAD2, OAS1, IFIT1, IFI44L, SIGLEC1, ISG15, HERC6, IL1R2 | 0,860 | 0,850 | 0,869 | 0,903 | 0,843 | 0,962 | 0,959 | 0,917 | 1 |
| IFIT1, SIGLEC1, ISG15, HERC6, SLC1A2, IL1R2, RETN, MMP8 | 0,860 | 0,850 | 0,869 | 0,912 | 0,858 | 0,966 | 0,965 | 0,929 | 1 |
| RSAD2, IFIT1, SIGLEC1, HERC6, SLC1A2, IL1R2, RETN, MMP8 | 0,860 | 0,850 | 0,869 | 0,911 | 0,857 | 0,966 | 0,961 | 0,922 | 1 |
| IFI44L, SIGLEC1, HERC6, SLC1A2, IL1R2, OLAH, RETN, MMP8 | 0,860 | 0,850 | 0,869 | 0,913 | 0,858 | 0,968 | 0,967 | 0,933 | 1 |
| OAS1, IFIT1, HERC6, SLC1A2, IL1R2, FAM20A, RETN, MMP8 | 0,860 | 0,850 | 0,869 | 0,915 | 0,860 | 0,969 | 0,930 | 0,845 | 1 |
| RSAD2, IFIT1, IFI44L, SIGLEC1, ISG15, HERC6, IL1R2, RETN | 0,859 | 0,850 | 0,869 | 0,904 | 0,842 | 0,966 | 0,955 | 0,912 | 0,999 |
| OAS1, IFI44L, SIGLEC1, HERC6, SLC1A2, OLAH, RETN, MMP8 | 0,859 | 0,849 | 0,870 | 0,911 | 0,853 | 0,968 | 0,963 | 0,922 | 1 |
| OAS1, IFIT1, IFI44L, SIGLEC1, ISG15, HERC6, IL1R2, MMP8 | 0,859 | 0,850 | 0,869 | 0,903 | 0,844 | 0,961 | 0,961 | 0,920 | 1 |
| OAS1, IFI44L, ISG15, HERC6, SLC1A2, OLAH, FAM20A, RETN | 0,859 | 0,849 | 0,870 | 0,913 | 0,857 | 0,969 | 0,924 | 0,842 | 1 |
| IFI27, OAS1, IFIT1, IFI44L, ISG15, HERC6, SLC1A2, FAM20A | 0,859 | 0,849 | 0,870 | 0,913 | 0,856 | 0,969 | 0,920 | 0,831 | 1 |

| | | | | | | | | |
|---|---|---|---|---|---|---|---|---|
| RSAD2, IFIT1, IFI44L, HERC6, SLC1A2, IL1R2, FAM20A, MMP8 | 0,859 | 0,850 | 0,869 | 0,917 | 0,867 | 0,968 | 0,925 | 0,832 | 1 |
| IFI27, OAS1, IFI44L, SIGLEC1, ISG15, HERC6, SLC1A2, MMP8 | 0,859 | 0,850 | 0,868 | 0,911 | 0,858 | 0,965 | 0,921 | 0,850 | 0,991 |
| RSAD2, IFIT1, ISG15, SLC1A2, IL1R2, OLAH, FAM20A, MMP8 | 0,859 | 0,850 | 0,869 | 0,914 | 0,862 | 0,967 | 0,935 | 0,856 | 1 |
| RSAD2, OAS1, ISG15, HERC6, OLAH, FAM20A, RETN, MMP8 | 0,859 | 0,849 | 0,870 | 0,905 | 0,843 | 0,967 | 0,942 | 0,874 | 1 |
| IFIT1, ISG15, HERC6, SLC1A2, IL1R2, OLAH, FAM20A, RETN | 0,859 | 0,849 | 0,870 | 0,912 | 0,857 | 0,967 | 0,928 | 0,844 | 1 |
| RSAD2, IFIT1, IFI44L, HERC6, SLC1A2, OLAH, FAM20A, RETN | 0,859 | 0,849 | 0,870 | 0,918 | 0,865 | 0,970 | 0,913 | 0,826 | 1 |
| RSAD2, OAS1, IFIT1, IL1R2, OLAH, FAM20A, RETN, MMP8 | 0,859 | 0,849 | 0,869 | 0,907 | 0,845 | 0,969 | 0,947 | 0,878 | 1 |
| RSAD2, OAS1, SIGLEC1, ISG15, HERC6, SLC1A2, IL1R2, RETN | 0,859 | 0,849 | 0,869 | 0,910 | 0,855 | 0,965 | 0,961 | 0,916 | 1 |
| IFIT1, ISG15, HERC6, SLC1A2, IL1R2, FAM20A, RETN, MMP8 | 0,859 | 0,850 | 0,869 | 0,915 | 0,860 | 0,969 | 0,930 | 0,844 | 1 |
| RSAD2, OAS1, HERC6, SLC1A2, IL1R2, OLAH, FAM20A, RETN | 0,859 | 0,849 | 0,870 | 0,912 | 0,858 | 0,967 | 0,926 | 0,840 | 1 |
| OAS1, IFIT1, ISG15, HERC6, SLC1A2, IL1R2, FAM20A, RETN | 0,859 | 0,849 | 0,870 | 0,911 | 0,856 | 0,966 | 0,929 | 0,843 | 1 |
| OAS1, IFIT1, IFI44L, SIGLEC1, HERC6, IL1R2, OLAH, RETN | 0,859 | 0,850 | 0,869 | 0,905 | 0,843 | 0,967 | 0,947 | 0,899 | 0,995 |
| RSAD2, OAS1, ISG15, HERC6, SLC1A2, IL1R2, FAM20A, MMP8 | 0,859 | 0,849 | 0,869 | 0,915 | 0,864 | 0,966 | 0,924 | 0,831 | 1 |
| RSAD2, OAS1, ISG15, SLC1A2, IL1R2, OLAH, FAM20A, MMP8 | 0,859 | 0,850 | 0,869 | 0,914 | 0,862 | 0,967 | 0,932 | 0,849 | 1 |
| RSAD2, IFIT1, IFI44L, SIGLEC1, HERC6, SLC1A2, OLAH, RETN | 0,859 | 0,850 | 0,869 | 0,914 | 0,859 | 0,969 | 0,937 | 0,883 | 0,991 |
| RSAD2, IFIT1, IFI44L, ISG15, HERC6, OLAH, FAM20A, RETN | 0,859 | 0,850 | 0,869 | 0,908 | 0,848 | 0,968 | 0,934 | 0,865 | 1 |
| RSAD2, IFIT1, IFI44L, SIGLEC1, HERC6, IL1R2, OLAH, MMP8 | 0,859 | 0,850 | 0,869 | 0,904 | 0,845 | 0,964 | 0,962 | 0,922 | 1 |
| RSAD2, OAS1, SIGLEC1, HERC6, SLC1A2, IL1R2, OLAH, RETN | 0,859 | 0,849 | 0,870 | 0,912 | 0,856 | 0,967 | 0,958 | 0,912 | 1 |
| OAS1, IFI44L, ISG15, HERC6, SLC1A2, IL1R2, FAM20A, MMP8 | 0,859 | 0,849 | 0,870 | 0,917 | 0,867 | 0,967 | 0,924 | 0,830 | 1 |
| OAS1, ISG15, HERC6, SLC1A2, OLAH, FAM20A, RETN, MMP8 | 0,859 | 0,849 | 0,870 | 0,912 | 0,855 | 0,968 | 0,935 | 0,856 | 1 |
| RSAD2, IFIT1, SIGLEC1, HERC6, SLC1A2, IL1R2, OLAH, RETN | 0,859 | 0,849 | 0,869 | 0,915 | 0,861 | 0,970 | 0,951 | 0,905 | 0,997 |
| OAS1, IFIT1, IFI44L, SIGLEC1, SLC1A2, OLAH, RETN, MMP8 | 0,859 | 0,849 | 0,869 | 0,912 | 0,856 | 0,969 | 0,957 | 0,912 | 1 |
| RSAD2, IFIT1, SIGLEC1, ISG15, HERC6, SLC1A2, OLAH, MMP8 | 0,859 | 0,849 | 0,869 | 0,912 | 0,857 | 0,967 | 0,947 | 0,898 | 0,996 |
| OAS1, IFIT1, ISG15, HERC6, SLC1A2, IL1R2, FAM20A, MMP8 | 0,859 | 0,849 | 0,869 | 0,915 | 0,863 | 0,967 | 0,928 | 0,839 | 1 |
| RSAD2, OAS1, IFIT1, IFI44L, SIGLEC1, ISG15, HERC6, RETN | 0,859 | 0,849 | 0,869 | 0,904 | 0,840 | 0,967 | 0,933 | 0,875 | 0,990 |
| RSAD2, IFI44L, SIGLEC1, ISG15, HERC6, IL1R2, RETN, MMP8 | 0,859 | 0,849 | 0,869 | 0,902 | 0,838 | 0,966 | 0,964 | 0,927 | 1 |
| IFIT1, IFI44L, ISG15, HERC6, SLC1A2, IL1R2, FAM20A, MMP8 | 0,859 | 0,849 | 0,869 | 0,916 | 0,866 | 0,967 | 0,924 | 0,830 | 1 |
| RSAD2, IFI44L, SIGLEC1, HERC6, SLC1A2, IL1R2, OLAH, MMP8 | 0,859 | 0,849 | 0,869 | 0,912 | 0,858 | 0,966 | 0,965 | 0,929 | 1 |

| | | | | | | | | | |
|---|---|---|---|---|---|---|---|---|---|
| RSAD2, OAS1, IFI44L, SIGLEC1, ISG15, OLAH, FAM20A, RETN | 0,859 | 0,849 | 0,869 | 0,897 | 0,824 | 0,969 | 0,960 | 0,915 | 1 |
| RSAD2, OAS1, IFI44L, SIGLEC1, HERC6, IL1R2, OLAH, MMP8 | 0,859 | 0,850 | 0,869 | 0,904 | 0,846 | 0,963 | 0,962 | 0,922 | 1 |
| RSAD2, OAS1, IFI44L, SIGLEC1, ISG15, HERC6, FAM20A, MMP8 | 0,859 | 0,849 | 0,869 | 0,902 | 0,837 | 0,967 | 0,940 | 0,869 | 1 |
| IFI27, OAS1, IFIT1, ISG15, HERC6, SLC1A2, FAM20A, MMP8 | 0,859 | 0,848 | 0,870 | 0,915 | 0,860 | 0,969 | 0,925 | 0,840 | 1 |
| RSAD2, ISG15, HERC6, SLC1A2, IL1R2, FAM20A, RETN, MMP8 | 0,859 | 0,849 | 0,869 | 0,916 | 0,864 | 0,969 | 0,928 | 0,839 | 1 |
| OAS1, IFIT1, IFI44L, HERC6, IL1R2, OLAH, FAM20A, RETN | 0,859 | 0,850 | 0,869 | 0,905 | 0,844 | 0,967 | 0,934 | 0,857 | 1 |
| IFIT1, IFI44L, SIGLEC1, HERC6, SLC1A2, IL1R2, RETN, MMP8 | 0,859 | 0,850 | 0,868 | 0,911 | 0,856 | 0,965 | 0,964 | 0,927 | 1 |
| RSAD2, IFI44L, HERC6, SLC1A2, IL1R2, OLAH, FAM20A, RETN | 0,859 | 0,848 | 0,870 | 0,915 | 0,861 | 0,968 | 0,924 | 0,840 | 1 |
| OAS1, IFI44L, SIGLEC1, HERC6, SLC1A2, IL1R2, OLAH, MMP8 | 0,859 | 0,849 | 0,869 | 0,912 | 0,859 | 0,966 | 0,961 | 0,922 | 1 |
| OAS1, IFIT1, ISG15, HERC6, OLAH, FAM20A, RETN, MMP8 | 0,859 | 0,849 | 0,869 | 0,905 | 0,842 | 0,967 | 0,947 | 0,882 | 1 |
| RSAD2, OAS1, IFIT1, SIGLEC1, HERC6, IL1R2, OLAH, MMP8 | 0,859 | 0,849 | 0,869 | 0,906 | 0,848 | 0,963 | 0,960 | 0,919 | 1 |
| RSAD2, ISG15, HERC6, SLC1A2, IL1R2, OLAH, FAM20A, RETN | 0,859 | 0,849 | 0,869 | 0,913 | 0,858 | 0,968 | 0,927 | 0,842 | 1 |
| RSAD2, OAS1, SIGLEC1, ISG15, HERC6, IL1R2, OLAH, MMP8 | 0,859 | 0,849 | 0,869 | 0,904 | 0,847 | 0,962 | 0,961 | 0,920 | 1 |
| OAS1, IFIT1, IFI44L, ISG15, HERC6, SLC1A2, IL1R2, FAM20A | 0,859 | 0,849 | 0,869 | 0,915 | 0,865 | 0,966 | 0,926 | 0,835 | 1 |
| OAS1, IFIT1, SIGLEC1, ISG15, SLC1A2, IL1R2, OLAH, MMP8 | 0,859 | 0,850 | 0,868 | 0,913 | 0,859 | 0,966 | 0,965 | 0,929 | 1 |
| IFIT1, SIGLEC1, ISG15, SLC1A2, IL1R2, OLAH, RETN, MMP8 | 0,859 | 0,850 | 0,868 | 0,913 | 0,858 | 0,967 | 0,966 | 0,931 | 1 |
| RSAD2, IFIT1, IFI44L, ISG15, HERC6, SLC1A2, IL1R2, FAM20A | 0,859 | 0,849 | 0,869 | 0,915 | 0,864 | 0,966 | 0,923 | 0,830 | 1 |
| IFI27, OAS1, IFIT1, ISG15, SLC1A2, FAM20A, RETN, MMP8 | 0,859 | 0,848 | 0,870 | 0,909 | 0,846 | 0,972 | 0,941 | 0,861 | 1 |
| OAS1, IFI44L, SIGLEC1, HERC6, SLC1A2, IL1R2, RETN, MMP8 | 0,859 | 0,849 | 0,868 | 0,911 | 0,856 | 0,966 | 0,965 | 0,928 | 1 |
| RSAD2, OAS1, IFIT1, HERC6, SLC1A2, OLAH, FAM20A, MMP8 | 0,859 | 0,849 | 0,869 | 0,909 | 0,853 | 0,966 | 0,929 | 0,849 | 1 |
| IFI44L, SIGLEC1, ISG15, HERC6, SLC1A2, IL1R2, OLAH, MMP8 | 0,859 | 0,849 | 0,868 | 0,913 | 0,859 | 0,966 | 0,964 | 0,927 | 1 |
| OAS1, IFIT1, IFI44L, SIGLEC1, ISG15, SLC1A2, OLAH, MMP8 | 0,859 | 0,849 | 0,869 | 0,913 | 0,857 | 0,968 | 0,950 | 0,903 | 0,997 |
| RSAD2, OAS1, IFIT1, SIGLEC1, ISG15, HERC6, OLAH, RETN | 0,859 | 0,849 | 0,869 | 0,905 | 0,842 | 0,967 | 0,948 | 0,899 | 0,997 |
| RSAD2, OAS1, IFIT1, HERC6, SLC1A2, IL1R2, OLAH, FAM20A | 0,859 | 0,849 | 0,869 | 0,912 | 0,858 | 0,966 | 0,928 | 0,844 | 1 |
| RSAD2, OAS1, IFI44L, SIGLEC1, ISG15, HERC6, IL1R2, RETN | 0,859 | 0,849 | 0,869 | 0,902 | 0,839 | 0,964 | 0,957 | 0,913 | 1 |
| RSAD2, OAS1, IFI44L, HERC6, IL1R2, OLAH, FAM20A, RETN | 0,859 | 0,848 | 0,869 | 0,907 | 0,847 | 0,968 | 0,935 | 0,860 | 1 |
| RSAD2, IFI27, IFI44L, SIGLEC1, ISG15, SLC1A2, RETN, MMP8 | 0,859 | 0,849 | 0,868 | 0,915 | 0,859 | 0,971 | 0,950 | 0,891 | 1 |
| OAS1, IFIT1, IFI44L, SIGLEC1, HERC6, SLC1A2, IL1R2, RETN | 0,859 | 0,849 | 0,868 | 0,913 | 0,859 | 0,966 | 0,948 | 0,900 | 0,996 |

571

| | | | | | | | | | |
|---|---|---|---|---|---|---|---|---|---|
| RSAD2, OAS1, IFIT1, HERC6, IL1R2, OLAH, FAM20A, RETN | 0,859 | 0,849 | 0,869 | 0,906 | 0,844 | 0,967 | 0,937 | 0,863 | 1 |
| RSAD2, IFIT1, ISG15, HERC6, SLC1A2, IL1R2, OLAH, FAM20A | 0,859 | 0,849 | 0,869 | 0,912 | 0,858 | 0,966 | 0,928 | 0,844 | 1 |
| RSAD2, OAS1, IFIT1, IFI44L, HERC6, OLAH, FAM20A, RETN | 0,859 | 0,849 | 0,868 | 0,905 | 0,843 | 0,968 | 0,933 | 0,863 | 1 |
| OAS1, IFIT1, SLC1A2, IL1R2, OLAH, FAM20A, RETN, MMP8 | 0,859 | 0,849 | 0,869 | 0,913 | 0,858 | 0,967 | 0,938 | 0,862 | 1 |
| RSAD2, OAS1, ISG15, IL1R2, OLAH, FAM20A, RETN, MMP8 | 0,859 | 0,849 | 0,869 | 0,907 | 0,845 | 0,968 | 0,938 | 0,861 | 1 |
| RSAD2, IFI44L, SLC1A2, IL1R2, OLAH, FAM20A, RETN, MMP8 | 0,859 | 0,849 | 0,869 | 0,918 | 0,865 | 0,970 | 0,934 | 0,858 | 1 |
| RSAD2, OAS1, IFIT1, IFI44L, SIGLEC1, HERC6, OLAH, RETN | 0,859 | 0,849 | 0,868 | 0,902 | 0,838 | 0,966 | 0,939 | 0,886 | 0,992 |
| RSAD2, OAS1, IFI44L, SIGLEC1, HERC6, SLC1A2, FAM20A, RETN | 0,859 | 0,848 | 0,869 | 0,911 | 0,855 | 0,968 | 0,942 | 0,870 | 1 |
| IFIT1, IFI44L, SIGLEC1, ISG15, HERC6, SLC1A2, IL1R2, OLAH | 0,859 | 0,849 | 0,868 | 0,913 | 0,859 | 0,967 | 0,953 | 0,909 | 0,997 |
| RSAD2, IFIT1, IFI44L, SIGLEC1, ISG15, HERC6, IL1R2, MMP8 | 0,859 | 0,850 | 0,868 | 0,903 | 0,844 | 0,963 | 0,959 | 0,917 | 1 |
| OAS1, IFIT1, IFI44L, SIGLEC1, ISG15, HERC6, IL1R2, OLAH | 0,859 | 0,849 | 0,868 | 0,907 | 0,849 | 0,965 | 0,957 | 0,914 | 0,999 |
| IFIT1, ISG15, HERC6, SLC1A2, OLAH, FAM20A, RETN, MMP8 | 0,859 | 0,848 | 0,869 | 0,909 | 0,851 | 0,967 | 0,934 | 0,855 | 1 |
| OAS1, IFIT1, IFI44L, SIGLEC1, HERC6, SLC1A2, IL1R2, OLAH | 0,858 | 0,848 | 0,869 | 0,913 | 0,859 | 0,967 | 0,952 | 0,907 | 0,997 |
| RSAD2, IFIT1, SIGLEC1, ISG15, HERC6, IL1R2, OLAH, RETN | 0,858 | 0,849 | 0,868 | 0,905 | 0,843 | 0,967 | 0,950 | 0,904 | 0,996 |
| RSAD2, OAS1, IFIT1, ISG15, HERC6, SLC1A2, IL1R2, FAM20A | 0,858 | 0,849 | 0,868 | 0,911 | 0,857 | 0,965 | 0,928 | 0,842 | 1 |
| OAS1, IFIT1, SIGLEC1, ISG15, HERC6, IL1R2, OLAH, MMP8 | 0,858 | 0,849 | 0,868 | 0,905 | 0,847 | 0,962 | 0,961 | 0,921 | 1 |
| OAS1, IFIT1, SIGLEC1, SLC1A2, IL1R2, OLAH, RETN, MMP8 | 0,858 | 0,849 | 0,868 | 0,914 | 0,860 | 0,967 | 0,966 | 0,931 | 1 |
| OAS1, IFIT1, IFI44L, ISG15, IL1R2, OLAH, FAM20A, MMP8 | 0,858 | 0,849 | 0,868 | 0,907 | 0,845 | 0,968 | 0,945 | 0,874 | 1 |
| RSAD2, OAS1, IFIT1, SIGLEC1, ISG15, SLC1A2, OLAH, MMP8 | 0,858 | 0,849 | 0,868 | 0,911 | 0,855 | 0,967 | 0,952 | 0,907 | 0,998 |
| RSAD2, IFIT1, ISG15, HERC6, SLC1A2, IL1R2, FAM20A, MMP8 | 0,858 | 0,849 | 0,868 | 0,915 | 0,863 | 0,967 | 0,929 | 0,842 | 1 |
| RSAD2, OAS1, IFIT1, SIGLEC1, ISG15, HERC6, IL1R2, OLAH | 0,858 | 0,849 | 0,868 | 0,907 | 0,849 | 0,964 | 0,954 | 0,910 | 0,998 |
| RSAD2, OAS1, SIGLEC1, SLC1A2, IL1R2, OLAH, RETN, MMP8 | 0,858 | 0,848 | 0,868 | 0,914 | 0,859 | 0,968 | 0,972 | 0,940 | 1 |
| OAS1, IFIT1, SIGLEC1, ISG15, SLC1A2, IL1R2, OLAH, RETN | 0,858 | 0,849 | 0,868 | 0,913 | 0,858 | 0,968 | 0,957 | 0,913 | 1,000 |
| RSAD2, IFIT1, SIGLEC1, ISG15, HERC6, OLAH, RETN, MMP8 | 0,858 | 0,848 | 0,868 | 0,904 | 0,841 | 0,968 | 0,959 | 0,918 | 0,999 |
| RSAD2, IFIT1, SIGLEC1, ISG15, SLC1A2, IL1R2, OLAH, MMP8 | 0,858 | 0,849 | 0,867 | 0,913 | 0,859 | 0,966 | 0,966 | 0,931 | 1 |
| RSAD2, OAS1, IFI44L, SIGLEC1, ISG15, HERC6, IL1R2, OLAH | 0,858 | 0,849 | 0,868 | 0,903 | 0,843 | 0,963 | 0,958 | 0,915 | 1,000 |
| RSAD2, OAS1, IFIT1, IFI44L, SIGLEC1, SLC1A2, OLAH, MMP8 | 0,858 | 0,849 | 0,868 | 0,910 | 0,854 | 0,966 | 0,954 | 0,910 | 0,999 |
| RSAD2, OAS1, ISG15, HERC6, SLC1A2, IL1R2, FAM20A, RETN | 0,858 | 0,848 | 0,869 | 0,912 | 0,858 | 0,966 | 0,925 | 0,836 | 1 |

| | | | | | | | | |
|---|---|---|---|---|---|---|---|---|
| OAS1, IFIT1, SIGLEC1, ISG15, SLC1A2, OLAH, RETN, MMP8 | 0,858 | 0,848 | 0,868 | 0,911 | 0,855 | 0,968 | 0,957 | 0,914 | 0,999 |
| RSAD2, IFIT1, HERC6, SLC1A2, IL1R2, FAM20A, RETN, MMP8 | 0,858 | 0,849 | 0,868 | 0,915 | 0,860 | 0,969 | 0,930 | 0,845 | 1 |
| RSAD2, IFI27, IFIT1, ISG15, SLC1A2, FAM20A, RETN, MMP8 | 0,858 | 0,848 | 0,869 | 0,908 | 0,845 | 0,971 | 0,942 | 0,864 | 1 |
| OAS1, SIGLEC1, ISG15, SLC1A2, IL1R2, OLAH, RETN, MMP8 | 0,858 | 0,849 | 0,868 | 0,913 | 0,859 | 0,967 | 0,966 | 0,931 | 1 |
| RSAD2, IFIT1, IFI44L, SIGLEC1, HERC6, SLC1A2, IL1R2, OLAH | 0,858 | 0,849 | 0,868 | 0,913 | 0,859 | 0,967 | 0,954 | 0,911 | 0,998 |
| RSAD2, IFI44L, SIGLEC1, ISG15, SLC1A2, FAM20A, RETN, MMP8 | 0,858 | 0,848 | 0,868 | 0,910 | 0,849 | 0,970 | 0,950 | 0,883 | 1 |
| RSAD2, OAS1, SIGLEC1, ISG15, HERC6, IL1R2, OLAH, RETN | 0,858 | 0,848 | 0,869 | 0,905 | 0,844 | 0,966 | 0,958 | 0,915 | 1 |
| RSAD2, IFI27, OAS1, IFIT1, SLC1A2, FAM20A, RETN, MMP8 | 0,858 | 0,848 | 0,869 | 0,909 | 0,846 | 0,972 | 0,943 | 0,866 | 1 |
| RSAD2, IFIT1, IFI44L, ISG15, HERC6, OLAH, FAM20A, MMP8 | 0,858 | 0,848 | 0,869 | 0,905 | 0,844 | 0,966 | 0,933 | 0,862 | 1 |
| RSAD2, IFIT1, SIGLEC1, SLC1A2, IL1R2, OLAH, RETN, MMP8 | 0,858 | 0,849 | 0,868 | 0,914 | 0,859 | 0,968 | 0,970 | 0,937 | 1 |
| RSAD2, OAS1, IFI44L, SIGLEC1, ISG15, FAM20A, RETN, MMP8 | 0,858 | 0,848 | 0,869 | 0,904 | 0,836 | 0,971 | 0,953 | 0,889 | 1 |
| RSAD2, OAS1, IFI44L, SIGLEC1, SLC1A2, FAM20A, RETN, MMP8 | 0,858 | 0,848 | 0,869 | 0,910 | 0,851 | 0,970 | 0,949 | 0,878 | 1 |
| IFIT1, IFI44L, SIGLEC1, ISG15, HERC6, SLC1A2, IL1R2, RETN | 0,858 | 0,849 | 0,867 | 0,909 | 0,854 | 0,964 | 0,955 | 0,912 | 0,998 |
| RSAD2, ISG15, HERC6, SLC1A2, OLAH, FAM20A, RETN, MMP8 | 0,858 | 0,848 | 0,868 | 0,911 | 0,854 | 0,968 | 0,935 | 0,856 | 1 |
| RSAD2, OAS1, IFIT1, SIGLEC1, SLC1A2, IL1R2, OLAH, MMP8 | 0,858 | 0,849 | 0,868 | 0,911 | 0,856 | 0,965 | 0,968 | 0,934 | 1 |
| RSAD2, IFI44L, SIGLEC1, HERC6, IL1R2, OLAH, RETN, MMP8 | 0,858 | 0,848 | 0,868 | 0,902 | 0,838 | 0,966 | 0,963 | 0,924 | 1 |
| IFIT1, IFI44L, SIGLEC1, SLC1A2, IL1R2, OLAH, RETN, MMP8 | 0,858 | 0,849 | 0,868 | 0,914 | 0,860 | 0,968 | 0,967 | 0,933 | 1 |
| OAS1, IFIT1, ISG15, HERC6, SLC1A2, OLAH, FAM20A, RETN | 0,858 | 0,848 | 0,869 | 0,911 | 0,855 | 0,968 | 0,934 | 0,855 | 1 |
| RSAD2, OAS1, IFIT1, IFI44L, ISG15, HERC6, FAM20A, RETN | 0,858 | 0,848 | 0,868 | 0,906 | 0,846 | 0,967 | 0,923 | 0,843 | 1 |
| RSAD2, OAS1, IFIT1, SIGLEC1, ISG15, HERC6, SLC1A2, IL1R2 | 0,858 | 0,849 | 0,868 | 0,908 | 0,855 | 0,962 | 0,966 | 0,929 | 1 |
| IFIT1, ISG15, SLC1A2, IL1R2, OLAH, FAM20A, RETN, MMP8 | 0,858 | 0,848 | 0,868 | 0,914 | 0,861 | 0,967 | 0,938 | 0,864 | 1 |
| IFIT1, IFI44L, SIGLEC1, HERC6, SLC1A2, IL1R2, OLAH, RETN | 0,858 | 0,848 | 0,868 | 0,915 | 0,862 | 0,969 | 0,949 | 0,902 | 0,996 |
| RSAD2, SIGLEC1, ISG15, HERC6, SLC1A2, IL1R2, OLAH, RETN | 0,858 | 0,848 | 0,868 | 0,915 | 0,861 | 0,969 | 0,958 | 0,916 | 0,999 |
| RSAD2, SIGLEC1, ISG15, SLC1A2, IL1R2, OLAH, RETN, MMP8 | 0,858 | 0,849 | 0,868 | 0,912 | 0,857 | 0,967 | 0,968 | 0,935 | 1 |
| RSAD2, IFIT1, IFI44L, SIGLEC1, ISG15, HERC6, SLC1A2, OLAH | 0,858 | 0,849 | 0,868 | 0,912 | 0,857 | 0,967 | 0,940 | 0,888 | 0,993 |
| RSAD2, SIGLEC1, ISG15, HERC6, SLC1A2, OLAH, RETN, MMP8 | 0,858 | 0,848 | 0,868 | 0,911 | 0,854 | 0,968 | 0,951 | 0,906 | 0,997 |
| RSAD2, IFI27, OAS1, IFIT1, IFI44L, ISG15, FAM20A, MMP8 | 0,858 | 0,847 | 0,869 | 0,900 | 0,834 | 0,967 | 0,933 | 0,854 | 1 |
| RSAD2, IFIT1, IFI44L, SIGLEC1, HERC6, IL1R2, OLAH, RETN | 0,858 | 0,849 | 0,867 | 0,903 | 0,840 | 0,967 | 0,949 | 0,902 | 0,996 |

| Genes | | | | | | | | |
|---|---|---|---|---|---|---|---|---|
| RSAD2, OAS1, IFIT1, IFI44L, HERC6, IL1R2, FAM20A, RETN | 0,858 | 0,848 | 0,868 | 0,908 | 0,847 | 0,969 | 0,934 | 0,853 | 1 |
| RSAD2, OAS1, ISG15, HERC6, SLC1A2, OLAH, FAM20A, MMP8 | 0,858 | 0,848 | 0,868 | 0,912 | 0,857 | 0,967 | 0,932 | 0,852 | 1 |
| IFIT1, IFI44L, ISG15, HERC6, SLC1A2, IL1R2, FAM20A, RETN | 0,858 | 0,848 | 0,868 | 0,915 | 0,862 | 0,968 | 0,922 | 0,829 | 1 |
| RSAD2, IFIT1, SIGLEC1, ISG15, HERC6, SLC1A2, OLAH, RETN | 0,858 | 0,848 | 0,868 | 0,912 | 0,856 | 0,968 | 0,945 | 0,895 | 0,994 |
| OAS1, IFIT1, IFI44L, SIGLEC1, ISG15, HERC6, RETN, MMP8 | 0,858 | 0,848 | 0,868 | 0,904 | 0,841 | 0,967 | 0,933 | 0,875 | 0,990 |
| OAS1, IFI44L, HERC6, SLC1A2, IL1R2, OLAH, FAM20A, RETN | 0,858 | 0,847 | 0,869 | 0,913 | 0,858 | 0,967 | 0,923 | 0,838 | 1 |
| RSAD2, OAS1, IFIT1, IFI44L, HERC6, SLC1A2, IL1R2, FAM20A | 0,858 | 0,848 | 0,868 | 0,914 | 0,862 | 0,965 | 0,924 | 0,833 | 1 |
| OAS1, IFI44L, SIGLEC1, ISG15, HERC6, SLC1A2, FAM20A, MMP8 | 0,858 | 0,847 | 0,869 | 0,908 | 0,851 | 0,966 | 0,942 | 0,868 | 1 |
| OAS1, IFI44L, SIGLEC1, ISG15, HERC6, SLC1A2, IL1R2, RETN | 0,858 | 0,848 | 0,868 | 0,910 | 0,854 | 0,965 | 0,958 | 0,912 | 1 |
| RSAD2, OAS1, SIGLEC1, ISG15, SLC1A2, IL1R2, OLAH, MMP8 | 0,858 | 0,848 | 0,868 | 0,911 | 0,857 | 0,966 | 0,967 | 0,933 | 1 |
| RSAD2, OAS1, IFI44L, HERC6, SLC1A2, OLAH, FAM20A, RETN | 0,858 | 0,847 | 0,869 | 0,914 | 0,859 | 0,969 | 0,923 | 0,842 | 1 |
| RSAD2, IFI27, OAS1, ISG15, HERC6, SLC1A2, FAM20A, MMP8 | 0,858 | 0,847 | 0,869 | 0,915 | 0,860 | 0,969 | 0,924 | 0,839 | 1 |
| RSAD2, OAS1, SIGLEC1, ISG15, HERC6, SLC1A2, OLAH, RETN | 0,858 | 0,848 | 0,868 | 0,912 | 0,856 | 0,969 | 0,953 | 0,904 | 1 |
| OAS1, IFIT1, HERC6, SLC1A2, OLAH, FAM20A, RETN, MMP8 | 0,858 | 0,848 | 0,868 | 0,909 | 0,851 | 0,967 | 0,932 | 0,852 | 1 |
| OAS1, IFI44L, HERC6, SLC1A2, IL1R2, FAM20A, RETN, MMP8 | 0,858 | 0,848 | 0,868 | 0,915 | 0,862 | 0,969 | 0,928 | 0,838 | 1 |
| OAS1, IFIT1, IFI44L, SIGLEC1, IL1R2, OLAH, RETN, MMP8 | 0,858 | 0,848 | 0,868 | 0,903 | 0,841 | 0,965 | 0,963 | 0,925 | 1 |
| RSAD2, IFIT1, IFI44L, SIGLEC1, SLC1A2, IL1R2, OLAH, MMP8 | 0,858 | 0,849 | 0,867 | 0,913 | 0,859 | 0,967 | 0,968 | 0,935 | 1 |
| RSAD2, OAS1, IFI44L, HERC6, OLAH, FAM20A, RETN, MMP8 | 0,858 | 0,848 | 0,868 | 0,908 | 0,846 | 0,970 | 0,937 | 0,868 | 1 |
| RSAD2, IFI44L, ISG15, HERC6, SLC1A2, IL1R2, FAM20A, MMP8 | 0,858 | 0,847 | 0,868 | 0,917 | 0,867 | 0,967 | 0,924 | 0,830 | 1 |
| RSAD2, IFIT1, IFI44L, HERC6, IL1R2, FAM20A, RETN, MMP8 | 0,858 | 0,848 | 0,868 | 0,909 | 0,847 | 0,970 | 0,939 | 0,859 | 1 |
| OAS1, IFIT1, IFI44L, SIGLEC1, ISG15, SLC1A2, OLAH, RETN | 0,858 | 0,848 | 0,868 | 0,915 | 0,860 | 0,971 | 0,940 | 0,885 | 0,995 |
| RSAD2, OAS1, IFI44L, ISG15, SLC1A2, IL1R2, OLAH, FAM20A | 0,858 | 0,847 | 0,869 | 0,913 | 0,858 | 0,969 | 0,928 | 0,848 | 1 |
| RSAD2, OAS1, IFIT1, SIGLEC1, ISG15, SLC1A2, IL1R2, OLAH | 0,858 | 0,848 | 0,868 | 0,913 | 0,858 | 0,968 | 0,967 | 0,931 | 1 |
| RSAD2, OAS1, IFIT1, IFI44L, SLC1A2, IL1R2, FAM20A, MMP8 | 0,858 | 0,848 | 0,868 | 0,916 | 0,864 | 0,967 | 0,930 | 0,844 | 1 |
| RSAD2, IFI44L, ISG15, HERC6, SLC1A2, OLAH, FAM20A, RETN | 0,858 | 0,847 | 0,869 | 0,914 | 0,858 | 0,970 | 0,924 | 0,843 | 1 |
| RSAD2, IFI27, IFIT1, IFI44L, SIGLEC1, ISG15, HERC6, MMP8 | 0,858 | 0,849 | 0,867 | 0,907 | 0,850 | 0,963 | 0,907 | 0,832 | 0,981 |
| OAS1, IFI44L, ISG15, HERC6, SLC1A2, IL1R2, FAM20A, RETN | 0,858 | 0,847 | 0,869 | 0,916 | 0,865 | 0,967 | 0,926 | 0,837 | 1 |
| OAS1, IFIT1, IFI44L, SIGLEC1, HERC6, SLC1A2, IL1R2, MMP8 | 0,858 | 0,848 | 0,867 | 0,909 | 0,856 | 0,962 | 0,963 | 0,924 | 1 |

574

| | | | | | | | | |
|---|---|---|---|---|---|---|---|---|
| OAS1, IFIT1, IFI44L, SIGLEC1, SLC1A2, IL1R2, OLAH, MMP8 | 0,858 | 0,848 | 0,867 | 0,912 | 0,858 | 0,966 | 0,966 | 0,931 | 1 |
| RSAD2, OAS1, IFIT1, IFI44L, SLC1A2, IL1R2, OLAH, FAM20A | 0,858 | 0,848 | 0,868 | 0,914 | 0,859 | 0,969 | 0,928 | 0,848 | 1 |
| RSAD2, IFIT1, IFI44L, SIGLEC1, HERC6, OLAH, RETN, MMP8 | 0,858 | 0,848 | 0,868 | 0,902 | 0,837 | 0,967 | 0,953 | 0,910 | 0,997 |
| OAS1, IFI44L, SIGLEC1, ISG15, HERC6, SLC1A2, OLAH, RETN | 0,858 | 0,847 | 0,868 | 0,913 | 0,857 | 0,970 | 0,951 | 0,901 | 1 |
| RSAD2, IFIT1, SIGLEC1, ISG15, HERC6, SLC1A2, IL1R2, RETN | 0,858 | 0,849 | 0,867 | 0,909 | 0,854 | 0,965 | 0,957 | 0,914 | 0,999 |
| RSAD2, OAS1, IFIT1, SIGLEC1, SLC1A2, IL1R2, OLAH, RETN | 0,858 | 0,848 | 0,867 | 0,913 | 0,857 | 0,968 | 0,966 | 0,928 | 1 |
| RSAD2, OAS1, HERC6, SLC1A2, OLAH, FAM20A, RETN, MMP8 | 0,858 | 0,848 | 0,868 | 0,910 | 0,853 | 0,967 | 0,934 | 0,854 | 1 |
| OAS1, IFI44L, SIGLEC1, ISG15, HERC6, IL1R2, OLAH, MMP8 | 0,858 | 0,848 | 0,867 | 0,903 | 0,844 | 0,962 | 0,963 | 0,924 | 1 |
| RSAD2, OAS1, ISG15, HERC6, SLC1A2, OLAH, FAM20A, RETN | 0,858 | 0,847 | 0,868 | 0,913 | 0,857 | 0,968 | 0,932 | 0,851 | 1 |
| RSAD2, IFIT1, IFI44L, HERC6, OLAH, FAM20A, RETN, MMP8 | 0,858 | 0,848 | 0,868 | 0,908 | 0,846 | 0,970 | 0,936 | 0,867 | 1 |
| OAS1, IFIT1, ISG15, IL1R2, OLAH, FAM20A, RETN, MMP8 | 0,858 | 0,848 | 0,867 | 0,905 | 0,842 | 0,969 | 0,945 | 0,873 | 1 |
| IFI44L, SIGLEC1, ISG15, HERC6, SLC1A2, OLAH, RETN, MMP8 | 0,858 | 0,848 | 0,868 | 0,910 | 0,852 | 0,968 | 0,961 | 0,922 | 1 |
| RSAD2, IFIT1, IFI44L, SLC1A2, OLAH, FAM20A, RETN, MMP8 | 0,858 | 0,848 | 0,867 | 0,913 | 0,855 | 0,970 | 0,927 | 0,854 | 1 |
| RSAD2, OAS1, IFIT1, SIGLEC1, ISG15, HERC6, IL1R2, MMP8 | 0,858 | 0,848 | 0,867 | 0,905 | 0,848 | 0,962 | 0,960 | 0,917 | 1 |
| IFI44L, SIGLEC1, ISG15, HERC6, SLC1A2, IL1R2, OLAH, RETN | 0,858 | 0,848 | 0,868 | 0,914 | 0,859 | 0,969 | 0,958 | 0,916 | 1,000 |
| OAS1, IFI44L, SIGLEC1, ISG15, HERC6, IL1R2, OLAH, RETN | 0,858 | 0,847 | 0,868 | 0,902 | 0,840 | 0,964 | 0,957 | 0,913 | 1 |
| OAS1, IFIT1, IFI44L, ISG15, HERC6, OLAH, FAM20A, MMP8 | 0,858 | 0,847 | 0,868 | 0,903 | 0,842 | 0,965 | 0,935 | 0,865 | 1 |
| OAS1, IFI44L, SIGLEC1, ISG15, SLC1A2, FAM20A, RETN, MMP8 | 0,858 | 0,847 | 0,868 | 0,905 | 0,843 | 0,967 | 0,950 | 0,880 | 1 |
| RSAD2, OAS1, IFIT1, IFI44L, ISG15, IL1R2, FAM20A, MMP8 | 0,858 | 0,847 | 0,868 | 0,909 | 0,848 | 0,970 | 0,942 | 0,867 | 1 |
| RSAD2, OAS1, SIGLEC1, ISG15, HERC6, SLC1A2, IL1R2, MMP8 | 0,858 | 0,848 | 0,867 | 0,911 | 0,859 | 0,964 | 0,967 | 0,931 | 1 |
| RSAD2, OAS1, IFI44L, SIGLEC1, HERC6, SLC1A2, IL1R2, OLAH | 0,858 | 0,847 | 0,868 | 0,912 | 0,858 | 0,966 | 0,962 | 0,922 | 1 |
| RSAD2, OAS1, SLC1A2, IL1R2, OLAH, FAM20A, RETN, MMP8 | 0,858 | 0,848 | 0,867 | 0,915 | 0,861 | 0,968 | 0,934 | 0,852 | 1 |
| RSAD2, OAS1, IFIT1, ISG15, HERC6, OLAH, FAM20A, RETN | 0,858 | 0,847 | 0,868 | 0,904 | 0,842 | 0,966 | 0,943 | 0,877 | 1 |
| RSAD2, IFIT1, IFI44L, SIGLEC1, ISG15, HERC6, IL1R2, OLAH | 0,857 | 0,848 | 0,867 | 0,904 | 0,844 | 0,965 | 0,954 | 0,910 | 0,998 |
| RSAD2, IFIT1, IFI44L, SIGLEC1, ISG15, SLC1A2, OLAH, MMP8 | 0,857 | 0,848 | 0,867 | 0,912 | 0,857 | 0,968 | 0,952 | 0,907 | 0,998 |
| RSAD2, IFIT1, SLC1A2, IL1R2, OLAH, FAM20A, RETN, MMP8 | 0,857 | 0,848 | 0,867 | 0,912 | 0,857 | 0,966 | 0,939 | 0,864 | 1 |
| OAS1, IFIT1, ISG15, HERC6, SLC1A2, OLAH, FAM20A, MMP8 | 0,857 | 0,847 | 0,868 | 0,909 | 0,853 | 0,965 | 0,932 | 0,853 | 1 |
| RSAD2, IFI27, IFIT1, ISG15, HERC6, SLC1A2, FAM20A, MMP8 | 0,857 | 0,847 | 0,868 | 0,914 | 0,858 | 0,969 | 0,927 | 0,843 | 1 |

| | | | | | | | | |
|---|---|---|---|---|---|---|---|---|
| RSAD2, OAS1, IFIT1, HERC6, OLAH, FAM20A, RETN, MMP8 | 0,857 | 0,847 | 0,868 | 0,903 | 0,840 | 0,966 | 0,946 | 0,879 | 1 |
| RSAD2, OAS1, HERC6, SLC1A2, IL1R2, FAM20A, RETN, MMP8 | 0,857 | 0,848 | 0,867 | 0,914 | 0,861 | 0,968 | 0,929 | 0,840 | 1 |
| RSAD2, IFI27, OAS1, ISG15, SLC1A2, FAM20A, RETN, MMP8 | 0,857 | 0,847 | 0,868 | 0,911 | 0,849 | 0,972 | 0,941 | 0,861 | 1 |
| IFI44L, SIGLEC1, ISG15, HERC6, SLC1A2, IL1R2, RETN, MMP8 | 0,857 | 0,848 | 0,866 | 0,910 | 0,854 | 0,965 | 0,966 | 0,931 | 1 |
| RSAD2, SIGLEC1, ISG15, HERC6, SLC1A2, IL1R2, RETN, MMP8 | 0,857 | 0,848 | 0,867 | 0,911 | 0,855 | 0,966 | 0,963 | 0,925 | 1 |
| IFI27, IFIT1, IFI44L, SIGLEC1, ISG15, HERC6, SLC1A2, MMP8 | 0,857 | 0,849 | 0,866 | 0,908 | 0,854 | 0,963 | 0,918 | 0,848 | 0,989 |
| RSAD2, OAS1, IFI44L, SIGLEC1, SLC1A2, IL1R2, OLAH, MMP8 | 0,857 | 0,848 | 0,867 | 0,912 | 0,858 | 0,966 | 0,968 | 0,934 | 1 |
| OAS1, IFIT1, SIGLEC1, ISG15, HERC6, SLC1A2, IL1R2, MMP8 | 0,857 | 0,848 | 0,867 | 0,911 | 0,859 | 0,963 | 0,964 | 0,926 | 1 |
| RSAD2, OAS1, IFI44L, ISG15, HERC6, OLAH, FAM20A, RETN | 0,857 | 0,847 | 0,868 | 0,905 | 0,844 | 0,967 | 0,942 | 0,878 | 1 |
| RSAD2, OAS1, IFIT1, IFI44L, SIGLEC1, SLC1A2, IL1R2, OLAH | 0,857 | 0,848 | 0,867 | 0,911 | 0,856 | 0,967 | 0,968 | 0,933 | 1 |
| OAS1, IFI44L, ISG15, SLC1A2, OLAH, FAM20A, RETN, MMP8 | 0,857 | 0,847 | 0,868 | 0,915 | 0,858 | 0,971 | 0,928 | 0,855 | 1 |
| RSAD2, OAS1, IFI44L, SIGLEC1, ISG15, HERC6, IL1R2, MMP8 | 0,857 | 0,848 | 0,867 | 0,901 | 0,841 | 0,961 | 0,963 | 0,924 | 1 |
| OAS1, IFIT1, IFI44L, SIGLEC1, ISG15, HERC6, SLC1A2, RETN | 0,857 | 0,848 | 0,867 | 0,909 | 0,852 | 0,966 | 0,930 | 0,871 | 0,990 |
| RSAD2, IFI44L, ISG15, SLC1A2, IL1R2, OLAH, FAM20A, RETN | 0,857 | 0,847 | 0,868 | 0,912 | 0,856 | 0,968 | 0,927 | 0,848 | 1 |
| OAS1, IFI44L, SLC1A2, IL1R2, OLAH, FAM20A, RETN, MMP8 | 0,857 | 0,847 | 0,868 | 0,917 | 0,864 | 0,969 | 0,932 | 0,853 | 1 |
| RSAD2, IFI44L, ISG15, HERC6, OLAH, FAM20A, RETN, MMP8 | 0,857 | 0,847 | 0,868 | 0,912 | 0,852 | 0,973 | 0,941 | 0,876 | 1 |
| RSAD2, IFIT1, ISG15, HERC6, SLC1A2, IL1R2, FAM20A, RETN | 0,857 | 0,847 | 0,868 | 0,911 | 0,855 | 0,966 | 0,930 | 0,845 | 1 |
| RSAD2, IFIT1, HERC6, SLC1A2, OLAH, FAM20A, RETN, MMP8 | 0,857 | 0,847 | 0,867 | 0,909 | 0,851 | 0,967 | 0,932 | 0,852 | 1 |
| OAS1, IFIT1, IFI44L, SLC1A2, OLAH, FAM20A, RETN, MMP8 | 0,857 | 0,847 | 0,868 | 0,913 | 0,856 | 0,970 | 0,926 | 0,851 | 1 |
| OAS1, IFI44L, SIGLEC1, ISG15, HERC6, SLC1A2, OLAH, MMP8 | 0,857 | 0,847 | 0,868 | 0,911 | 0,855 | 0,967 | 0,953 | 0,908 | 0,998 |
| RSAD2, IFIT1, IFI44L, SIGLEC1, SLC1A2, OLAH, RETN, MMP8 | 0,857 | 0,848 | 0,867 | 0,914 | 0,857 | 0,970 | 0,955 | 0,911 | 1 |
| RSAD2, IFIT1, IFI44L, ISG15, SLC1A2, IL1R2, OLAH, FAM20A | 0,857 | 0,848 | 0,867 | 0,914 | 0,858 | 0,969 | 0,928 | 0,850 | 1 |
| OAS1, IFI44L, SIGLEC1, ISG15, HERC6, SLC1A2, IL1R2, OLAH | 0,857 | 0,847 | 0,867 | 0,911 | 0,857 | 0,966 | 0,959 | 0,917 | 1 |
| RSAD2, IFIT1, IFI44L, HERC6, IL1R2, OLAH, FAM20A, RETN | 0,857 | 0,847 | 0,867 | 0,907 | 0,847 | 0,967 | 0,929 | 0,852 | 1 |
| OAS1, IFIT1, IFI44L, SIGLEC1, SLC1A2, IL1R2, OLAH, RETN | 0,857 | 0,848 | 0,867 | 0,915 | 0,860 | 0,970 | 0,955 | 0,912 | 0,999 |
| RSAD2, IFIT1, ISG15, HERC6, OLAH, FAM20A, RETN, MMP8 | 0,857 | 0,847 | 0,867 | 0,904 | 0,842 | 0,967 | 0,947 | 0,882 | 1 |
| RSAD2, IFIT1, SIGLEC1, ISG15, HERC6, IL1R2, OLAH, MMP8 | 0,857 | 0,848 | 0,867 | 0,907 | 0,850 | 0,964 | 0,960 | 0,919 | 1 |
| OAS1, IFIT1, IFI44L, SIGLEC1, ISG15, SLC1A2, IL1R2, OLAH | 0,857 | 0,848 | 0,867 | 0,912 | 0,857 | 0,968 | 0,961 | 0,922 | 1 |

| | | | | | | | | |
|---|---|---|---|---|---|---|---|---|
| RSAD2, OAS1, IFI44L, SIGLEC1, HERC6, SLC1A2, OLAH, MMP8 | 0,857 | 0,847 | 0,867 | 0,911 | 0,856 | 0,967 | 0,954 | 0,910 | 0,999 |
| RSAD2, OAS1, IFIT1, SIGLEC1, ISG15, HERC6, RETN, MMP8 | 0,857 | 0,847 | 0,867 | 0,903 | 0,839 | 0,966 | 0,933 | 0,875 | 0,990 |
| IFIT1, IFI44L, SIGLEC1, ISG15, SLC1A2, IL1R2, OLAH, RETN | 0,857 | 0,848 | 0,866 | 0,915 | 0,861 | 0,970 | 0,959 | 0,917 | 1 |
| RSAD2, IFIT1, SIGLEC1, ISG15, SLC1A2, IL1R2, OLAH, RETN | 0,857 | 0,848 | 0,867 | 0,912 | 0,856 | 0,968 | 0,960 | 0,918 | 1 |
| RSAD2, OAS1, IFI44L, SIGLEC1, ISG15, HERC6, SLC1A2, OLAH | 0,857 | 0,847 | 0,867 | 0,912 | 0,856 | 0,967 | 0,951 | 0,904 | 0,998 |
| RSAD2, OAS1, IFIT1, IFI44L, SIGLEC1, ISG15, SLC1A2, OLAH | 0,857 | 0,847 | 0,867 | 0,912 | 0,856 | 0,968 | 0,949 | 0,899 | 0,999 |
| OAS1, IFIT1, IFI44L, ISG15, SLC1A2, IL1R2, OLAH, FAM20A | 0,857 | 0,847 | 0,867 | 0,914 | 0,859 | 0,969 | 0,927 | 0,847 | 1 |
| OAS1, IFI44L, ISG15, HERC6, OLAH, FAM20A, RETN, MMP8 | 0,857 | 0,847 | 0,867 | 0,906 | 0,843 | 0,969 | 0,950 | 0,891 | 1 |
| RSAD2, OAS1, IFIT1, SIGLEC1, ISG15, HERC6, SLC1A2, RETN | 0,857 | 0,847 | 0,867 | 0,909 | 0,852 | 0,967 | 0,933 | 0,873 | 0,992 |
| OAS1, IFIT1, HERC6, SLC1A2, IL1R2, OLAH, FAM20A, RETN | 0,857 | 0,846 | 0,868 | 0,912 | 0,858 | 0,967 | 0,927 | 0,843 | 1 |
| RSAD2, IFI44L, SIGLEC1, ISG15, HERC6, IL1R2, OLAH, MMP8 | 0,857 | 0,847 | 0,866 | 0,902 | 0,841 | 0,962 | 0,962 | 0,922 | 1 |
| OAS1, IFI44L, SIGLEC1, HERC6, SLC1A2, IL1R2, OLAH, RETN | 0,857 | 0,846 | 0,868 | 0,915 | 0,862 | 0,969 | 0,957 | 0,913 | 1,000 |
| RSAD2, IFI27, IFIT1, IFI44L, SIGLEC1, HERC6, SLC1A2, MMP8 | 0,857 | 0,848 | 0,866 | 0,909 | 0,855 | 0,963 | 0,920 | 0,850 | 0,989 |
| RSAD2, IFI27, IFIT1, SIGLEC1, ISG15, HERC6, SLC1A2, MMP8 | 0,857 | 0,848 | 0,866 | 0,909 | 0,854 | 0,964 | 0,917 | 0,848 | 0,987 |
| RSAD2, OAS1, IFIT1, IFI44L, ISG15, IL1R2, OLAH, FAM20A | 0,857 | 0,847 | 0,867 | 0,901 | 0,836 | 0,966 | 0,948 | 0,882 | 1 |
| RSAD2, IFI44L, SIGLEC1, SLC1A2, IL1R2, OLAH, RETN, MMP8 | 0,857 | 0,847 | 0,866 | 0,914 | 0,859 | 0,968 | 0,971 | 0,939 | 1 |
| RSAD2, IFI44L, SIGLEC1, ISG15, HERC6, SLC1A2, IL1R2, OLAH | 0,857 | 0,847 | 0,867 | 0,911 | 0,856 | 0,966 | 0,955 | 0,912 | 0,998 |
| RSAD2, OAS1, SIGLEC1, ISG15, SLC1A2, IL1R2, OLAH, RETN | 0,857 | 0,847 | 0,867 | 0,912 | 0,856 | 0,968 | 0,968 | 0,931 | 1 |
| OAS1, IFI44L, ISG15, SLC1A2, IL1R2, OLAH, FAM20A, RETN | 0,857 | 0,846 | 0,868 | 0,911 | 0,856 | 0,967 | 0,928 | 0,848 | 1 |
| OAS1, IFIT1, IFI44L, SIGLEC1, ISG15, HERC6, SLC1A2, IL1R2 | 0,857 | 0,847 | 0,866 | 0,910 | 0,857 | 0,963 | 0,959 | 0,918 | 1,000 |
| OAS1, IFI44L, SIGLEC1, ISG15, SLC1A2, OLAH, RETN, MMP8 | 0,857 | 0,847 | 0,867 | 0,911 | 0,853 | 0,969 | 0,959 | 0,915 | 1 |
| RSAD2, IFI44L, ISG15, SLC1A2, OLAH, FAM20A, RETN, MMP8 | 0,857 | 0,847 | 0,867 | 0,913 | 0,856 | 0,970 | 0,926 | 0,852 | 1,000 |
| RSAD2, OAS1, IFI44L, IL1R2, OLAH, FAM20A, RETN, MMP8 | 0,857 | 0,846 | 0,867 | 0,909 | 0,848 | 0,970 | 0,945 | 0,876 | 1 |
| RSAD2, IFI44L, HERC6, SLC1A2, IL1R2, FAM20A, RETN, MMP8 | 0,857 | 0,847 | 0,867 | 0,918 | 0,865 | 0,970 | 0,928 | 0,838 | 1 |
| RSAD2, IFI44L, ISG15, HERC6, SLC1A2, IL1R2, FAM20A, RETN | 0,857 | 0,846 | 0,868 | 0,913 | 0,860 | 0,967 | 0,927 | 0,840 | 1 |
| RSAD2, OAS1, IFI44L, ISG15, HERC6, OLAH, FAM20A, MMP8 | 0,857 | 0,846 | 0,868 | 0,905 | 0,843 | 0,966 | 0,942 | 0,879 | 1 |
| RSAD2, IFI27, OAS1, IFIT1, HERC6, SLC1A2, FAM20A, MMP8 | 0,857 | 0,846 | 0,867 | 0,913 | 0,858 | 0,968 | 0,924 | 0,839 | 1 |
| RSAD2, OAS1, IFIT1, SIGLEC1, SLC1A2, OLAH, RETN, MMP8 | 0,857 | 0,847 | 0,867 | 0,913 | 0,857 | 0,970 | 0,961 | 0,918 | 1 |

| Gene set | | | | | | | | |
|---|---|---|---|---|---|---|---|---|
| RSAD2, OAS1, IFI44L, SIGLEC1, HERC6, IL1R2, OLAH, RETN | 0,857 | 0,847 | 0,867 | 0,900 | 0,836 | 0,965 | 0,958 | 0,914 | 1 |
| RSAD2, IFIT1, ISG15, HERC6, SLC1A2, OLAH, FAM20A, MMP8 | 0,857 | 0,846 | 0,867 | 0,909 | 0,853 | 0,965 | 0,929 | 0,849 | 1 |
| RSAD2, OAS1, IFIT1, SIGLEC1, HERC6, SLC1A2, IL1R2, MMP8 | 0,857 | 0,847 | 0,866 | 0,908 | 0,855 | 0,962 | 0,966 | 0,929 | 1 |
| OAS1, IFIT1, IFI44L, IL1R2, OLAH, FAM20A, RETN, MMP8 | 0,857 | 0,847 | 0,867 | 0,906 | 0,843 | 0,969 | 0,947 | 0,878 | 1 |
| RSAD2, OAS1, SIGLEC1, ISG15, HERC6, OLAH, RETN, MMP8 | 0,857 | 0,846 | 0,867 | 0,900 | 0,834 | 0,965 | 0,964 | 0,927 | 1 |
| OAS1, IFI44L, SIGLEC1, SLC1A2, IL1R2, OLAH, RETN, MMP8 | 0,857 | 0,847 | 0,867 | 0,913 | 0,859 | 0,967 | 0,967 | 0,932 | 1 |
| RSAD2, IFI44L, SIGLEC1, HERC6, SLC1A2, IL1R2, OLAH, RETN | 0,857 | 0,846 | 0,867 | 0,914 | 0,860 | 0,968 | 0,957 | 0,914 | 0,999 |
| RSAD2, OAS1, IFI44L, SIGLEC1, HERC6, SLC1A2, OLAH, RETN | 0,857 | 0,847 | 0,867 | 0,914 | 0,857 | 0,970 | 0,952 | 0,903 | 1 |
| IFIT1, IFI44L, ISG15, SLC1A2, IL1R2, FAM20A, RETN, MMP8 | 0,857 | 0,847 | 0,866 | 0,912 | 0,858 | 0,966 | 0,932 | 0,848 | 1 |
| IFIT1, IFI44L, SIGLEC1, ISG15, HERC6, SLC1A2, IL1R2, MMP8 | 0,857 | 0,847 | 0,866 | 0,908 | 0,855 | 0,962 | 0,964 | 0,927 | 1 |
| RSAD2, OAS1, IFI44L, SIGLEC1, HERC6, SLC1A2, IL1R2, RETN | 0,856 | 0,847 | 0,866 | 0,911 | 0,856 | 0,966 | 0,957 | 0,909 | 1 |
| RSAD2, OAS1, IFI44L, SLC1A2, OLAH, FAM20A, RETN, MMP8 | 0,856 | 0,846 | 0,867 | 0,914 | 0,857 | 0,971 | 0,926 | 0,851 | 1 |
| RSAD2, IFI44L, SIGLEC1, ISG15, HERC6, SLC1A2, OLAH, MMP8 | 0,856 | 0,847 | 0,866 | 0,911 | 0,856 | 0,967 | 0,947 | 0,898 | 0,995 |
| RSAD2, IFIT1, SIGLEC1, ISG15, SLC1A2, OLAH, RETN, MMP8 | 0,856 | 0,847 | 0,866 | 0,911 | 0,855 | 0,968 | 0,958 | 0,915 | 1 |
| RSAD2, OAS1, IFI44L, SIGLEC1, ISG15, SLC1A2, FAM20A, MMP8 | 0,856 | 0,846 | 0,867 | 0,903 | 0,841 | 0,966 | 0,943 | 0,870 | 1 |
| RSAD2, OAS1, IFIT1, IFI44L, SIGLEC1, SLC1A2, OLAH, RETN | 0,856 | 0,847 | 0,866 | 0,912 | 0,855 | 0,968 | 0,949 | 0,896 | 1 |
| OAS1, IFIT1, SIGLEC1, ISG15, HERC6, SLC1A2, RETN, MMP8 | 0,856 | 0,846 | 0,866 | 0,910 | 0,854 | 0,967 | 0,935 | 0,878 | 0,992 |
| RSAD2, OAS1, IFIT1, ISG15, SLC1A2, IL1R2, OLAH, FAM20A | 0,856 | 0,846 | 0,866 | 0,909 | 0,851 | 0,967 | 0,933 | 0,853 | 1 |
| RSAD2, IFIT1, IFI44L, SLC1A2, IL1R2, OLAH, FAM20A, RETN | 0,856 | 0,846 | 0,866 | 0,911 | 0,856 | 0,967 | 0,926 | 0,845 | 1 |
| RSAD2, IFIT1, IFI44L, SIGLEC1, HERC6, SLC1A2, IL1R2, RETN | 0,856 | 0,847 | 0,865 | 0,907 | 0,851 | 0,964 | 0,952 | 0,907 | 0,997 |
| RSAD2, IFIT1, IFI44L, SIGLEC1, ISG15, HERC6, OLAH, RETN | 0,856 | 0,847 | 0,866 | 0,903 | 0,840 | 0,966 | 0,943 | 0,894 | 0,993 |
| RSAD2, OAS1, IFIT1, ISG15, HERC6, SLC1A2, OLAH, FAM20A | 0,856 | 0,846 | 0,867 | 0,910 | 0,854 | 0,966 | 0,930 | 0,851 | 1 |
| RSAD2, IFIT1, HERC6, SLC1A2, IL1R2, OLAH, FAM20A, RETN | 0,856 | 0,846 | 0,866 | 0,912 | 0,857 | 0,967 | 0,928 | 0,844 | 1 |
| RSAD2, IFI44L, SIGLEC1, ISG15, SLC1A2, IL1R2, OLAH, MMP8 | 0,856 | 0,847 | 0,865 | 0,911 | 0,856 | 0,965 | 0,970 | 0,937 | 1 |
| RSAD2, IFIT1, SIGLEC1, ISG15, HERC6, SLC1A2, IL1R2, MMP8 | 0,856 | 0,847 | 0,865 | 0,908 | 0,854 | 0,963 | 0,965 | 0,929 | 1 |
| OAS1, ISG15, SLC1A2, IL1R2, OLAH, FAM20A, RETN, MMP8 | 0,856 | 0,846 | 0,866 | 0,915 | 0,861 | 0,968 | 0,930 | 0,848 | 1 |
| OAS1, IFI44L, SIGLEC1, ISG15, SLC1A2, IL1R2, OLAH, MMP8 | 0,856 | 0,847 | 0,866 | 0,914 | 0,860 | 0,967 | 0,966 | 0,931 | 1 |
| RSAD2, OAS1, IFI44L, SLC1A2, IL1R2, OLAH, FAM20A, RETN | 0,856 | 0,845 | 0,867 | 0,913 | 0,858 | 0,968 | 0,928 | 0,848 | 1 |

| | | | | | | | | |
|---|---|---|---|---|---|---|---|---|
| RSAD2, OAS1, SIGLEC1, ISG15, SLC1A2, OLAH, RETN, MMP8 | 0,856 | 0,846 | 0,866 | 0,910 | 0,853 | 0,968 | 0,961 | 0,919 | 1 |
| OAS1, IFIT1, IFI44L, SLC1A2, IL1R2, OLAH, FAM20A, RETN | 0,856 | 0,846 | 0,866 | 0,913 | 0,858 | 0,968 | 0,928 | 0,849 | 1 |
| RSAD2, OAS1, ISG15, SLC1A2, IL1R2, OLAH, FAM20A, RETN | 0,856 | 0,846 | 0,866 | 0,910 | 0,854 | 0,967 | 0,928 | 0,844 | 1 |
| RSAD2, IFIT1, IFI44L, SIGLEC1, ISG15, SLC1A2, IL1R2, OLAH | 0,856 | 0,847 | 0,865 | 0,910 | 0,854 | 0,966 | 0,964 | 0,927 | 1 |
| RSAD2, OAS1, IFIT1, ISG15, IL1R2, FAM20A, RETN, MMP8 | 0,856 | 0,846 | 0,866 | 0,908 | 0,846 | 0,970 | 0,947 | 0,875 | 1 |
| RSAD2, OAS1, IFIT1, IFI44L, SIGLEC1, IL1R2, OLAH, MMP8 | 0,856 | 0,847 | 0,865 | 0,901 | 0,841 | 0,962 | 0,960 | 0,919 | 1 |
| RSAD2, IFI44L, SIGLEC1, HERC6, SLC1A2, IL1R2, RETN, MMP8 | 0,856 | 0,847 | 0,865 | 0,910 | 0,854 | 0,965 | 0,964 | 0,927 | 1 |
| RSAD2, IFI44L, SIGLEC1, HERC6, SLC1A2, OLAH, RETN, MMP8 | 0,856 | 0,846 | 0,866 | 0,911 | 0,853 | 0,969 | 0,953 | 0,909 | 0,997 |
| RSAD2, ISG15, SLC1A2, IL1R2, OLAH, FAM20A, RETN, MMP8 | 0,856 | 0,846 | 0,866 | 0,914 | 0,861 | 0,967 | 0,932 | 0,849 | 1 |
| RSAD2, IFIT1, IFI44L, SIGLEC1, HERC6, SLC1A2, IL1R2, MMP8 | 0,856 | 0,847 | 0,865 | 0,908 | 0,853 | 0,962 | 0,963 | 0,925 | 1 |
| RSAD2, IFIT1, IFI44L, SIGLEC1, SLC1A2, IL1R2, OLAH, RETN | 0,856 | 0,847 | 0,865 | 0,913 | 0,857 | 0,969 | 0,962 | 0,923 | 1 |
| RSAD2, OAS1, IFI44L, SIGLEC1, ISG15, SLC1A2, IL1R2, OLAH | 0,856 | 0,846 | 0,866 | 0,912 | 0,856 | 0,967 | 0,966 | 0,928 | 1 |
| RSAD2, OAS1, IFIT1, SIGLEC1, IL1R2, OLAH, RETN, MMP8 | 0,856 | 0,846 | 0,866 | 0,901 | 0,839 | 0,964 | 0,965 | 0,929 | 1 |
| OAS1, IFI44L, SIGLEC1, ISG15, SLC1A2, IL1R2, OLAH, RETN | 0,856 | 0,846 | 0,866 | 0,914 | 0,858 | 0,969 | 0,962 | 0,922 | 1 |
| OAS1, IFIT1, ISG15, SLC1A2, IL1R2, OLAH, FAM20A, RETN | 0,856 | 0,845 | 0,866 | 0,912 | 0,855 | 0,968 | 0,935 | 0,856 | 1 |
| OAS1, IFIT1, IFI44L, HERC6, SLC1A2, IL1R2, FAM20A, RETN | 0,856 | 0,846 | 0,866 | 0,916 | 0,864 | 0,967 | 0,925 | 0,836 | 1 |
| OAS1, IFIT1, IFI44L, SIGLEC1, HERC6, SLC1A2, RETN, MMP8 | 0,856 | 0,847 | 0,865 | 0,908 | 0,849 | 0,967 | 0,927 | 0,868 | 0,987 |
| RSAD2, OAS1, IFIT1, IFI44L, SIGLEC1, HERC6, SLC1A2, IL1R2 | 0,856 | 0,846 | 0,865 | 0,908 | 0,852 | 0,963 | 0,964 | 0,925 | 1 |
| RSAD2, OAS1, IFI44L, SIGLEC1, ISG15, HERC6, SLC1A2, IL1R2 | 0,856 | 0,846 | 0,866 | 0,908 | 0,853 | 0,963 | 0,962 | 0,921 | 1 |
| RSAD2, OAS1, IFI44L, HERC6, SLC1A2, IL1R2, FAM20A, RETN | 0,856 | 0,845 | 0,866 | 0,915 | 0,863 | 0,967 | 0,926 | 0,838 | 1 |
| OAS1, IFIT1, SIGLEC1, ISG15, IL1R2, OLAH, RETN, MMP8 | 0,856 | 0,846 | 0,866 | 0,902 | 0,840 | 0,964 | 0,964 | 0,928 | 1 |
| OAS1, IFIT1, IFI44L, ISG15, SLC1A2, OLAH, FAM20A, RETN | 0,856 | 0,845 | 0,866 | 0,911 | 0,853 | 0,969 | 0,922 | 0,844 | 1,000 |
| RSAD2, OAS1, IFIT1, ISG15, SLC1A2, IL1R2, FAM20A, MMP8 | 0,856 | 0,846 | 0,865 | 0,915 | 0,862 | 0,968 | 0,936 | 0,855 | 1 |
| RSAD2, OAS1, IFIT1, SIGLEC1, ISG15, SLC1A2, OLAH, RETN | 0,856 | 0,846 | 0,865 | 0,911 | 0,855 | 0,968 | 0,958 | 0,910 | 1 |
| RSAD2, OAS1, IFIT1, IFI44L, ISG15, SLC1A2, OLAH, FAM20A | 0,856 | 0,846 | 0,866 | 0,911 | 0,853 | 0,969 | 0,921 | 0,842 | 0,999 |
| RSAD2, IFIT1, ISG15, HERC6, SLC1A2, OLAH, FAM20A, RETN | 0,856 | 0,845 | 0,866 | 0,911 | 0,854 | 0,968 | 0,930 | 0,849 | 1 |
| RSAD2, IFI44L, SIGLEC1, ISG15, HERC6, SLC1A2, OLAH, RETN | 0,856 | 0,846 | 0,865 | 0,914 | 0,857 | 0,970 | 0,947 | 0,898 | 0,995 |
| RSAD2, IFIT1, IFI44L, ISG15, HERC6, FAM20A, RETN, MMP8 | 0,856 | 0,846 | 0,866 | 0,908 | 0,848 | 0,969 | 0,925 | 0,845 | 1 |

| | | | | | | | | |
|---|---|---|---|---|---|---|---|---|
| OAS1, IFIT1, IFI44L, SIGLEC1, SLC1A2, IL1R2, RETN, MMP8 | 0,856 | 0,846 | 0,865 | 0,912 | 0,858 | 0,966 | 0,970 | 0,936 | 1 |
| RSAD2, OAS1, IFIT1, IFI44L, SIGLEC1, HERC6, SLC1A2, RETN | 0,856 | 0,846 | 0,865 | 0,910 | 0,853 | 0,968 | 0,928 | 0,868 | 0,989 |
| RSAD2, OAS1, IFIT1, SLC1A2, IL1R2, OLAH, FAM20A, RETN | 0,856 | 0,846 | 0,866 | 0,911 | 0,854 | 0,967 | 0,935 | 0,855 | 1 |
| OAS1, IFI44L, SIGLEC1, ISG15, HERC6, SLC1A2, IL1R2, MMP8 | 0,856 | 0,846 | 0,865 | 0,910 | 0,858 | 0,963 | 0,963 | 0,924 | 1 |
| RSAD2, OAS1, IFIT1, IFI44L, SIGLEC1, ISG15, IL1R2, OLAH | 0,855 | 0,846 | 0,865 | 0,897 | 0,832 | 0,963 | 0,958 | 0,916 | 0,999 |
| OAS1, IFIT1, SIGLEC1, ISG15, SLC1A2, IL1R2, RETN, MMP8 | 0,855 | 0,846 | 0,865 | 0,912 | 0,858 | 0,966 | 0,971 | 0,938 | 1 |
| RSAD2, IFI27, OAS1, IFIT1, SIGLEC1, ISG15, SLC1A2, MMP8 | 0,855 | 0,846 | 0,865 | 0,905 | 0,846 | 0,964 | 0,932 | 0,861 | 1 |
| RSAD2, OAS1, IFI44L, SIGLEC1, SLC1A2, IL1R2, OLAH, RETN | 0,855 | 0,846 | 0,865 | 0,911 | 0,855 | 0,967 | 0,968 | 0,931 | 1 |
| RSAD2, IFIT1, IFI44L, ISG15, SLC1A2, IL1R2, FAM20A, MMP8 | 0,855 | 0,846 | 0,865 | 0,911 | 0,859 | 0,964 | 0,928 | 0,842 | 1 |
| RSAD2, OAS1, IFI44L, SIGLEC1, ISG15, SLC1A2, OLAH, MMP8 | 0,855 | 0,845 | 0,865 | 0,911 | 0,856 | 0,967 | 0,952 | 0,906 | 0,998 |
| RSAD2, IFIT1, IFI44L, SIGLEC1, ISG15, SLC1A2, OLAH, RETN | 0,855 | 0,846 | 0,865 | 0,914 | 0,858 | 0,970 | 0,946 | 0,894 | 0,997 |
| OAS1, IFIT1, IFI44L, ISG15, SLC1A2, IL1R2, FAM20A, MMP8 | 0,855 | 0,845 | 0,865 | 0,914 | 0,861 | 0,966 | 0,929 | 0,840 | 1 |
| RSAD2, OAS1, IFIT1, SLC1A2, IL1R2, FAM20A, RETN, MMP8 | 0,855 | 0,846 | 0,865 | 0,914 | 0,859 | 0,968 | 0,936 | 0,856 | 1 |
| RSAD2, OAS1, IFI44L, SIGLEC1, ISG15, SLC1A2, FAM20A, RETN | 0,855 | 0,845 | 0,866 | 0,905 | 0,843 | 0,967 | 0,945 | 0,873 | 1 |
| RSAD2, OAS1, IFIT1, ISG15, HERC6, OLAH, FAM20A, MMP8 | 0,855 | 0,845 | 0,866 | 0,901 | 0,839 | 0,963 | 0,941 | 0,872 | 1 |
| RSAD2, IFI44L, SIGLEC1, ISG15, HERC6, SLC1A2, IL1R2, RETN | 0,855 | 0,846 | 0,865 | 0,908 | 0,852 | 0,964 | 0,958 | 0,915 | 1 |
| RSAD2, IFIT1, IFI44L, SIGLEC1, ISG15, HERC6, SLC1A2, IL1R2 | 0,855 | 0,846 | 0,864 | 0,906 | 0,851 | 0,962 | 0,960 | 0,920 | 1,000 |
| RSAD2, OAS1, IFIT1, HERC6, SLC1A2, IL1R2, FAM20A, RETN | 0,855 | 0,845 | 0,865 | 0,912 | 0,857 | 0,966 | 0,930 | 0,845 | 1 |
| OAS1, IFIT1, IFI44L, SIGLEC1, ISG15, IL1R2, OLAH, MMP8 | 0,855 | 0,846 | 0,865 | 0,903 | 0,843 | 0,963 | 0,958 | 0,916 | 1,000 |
| OAS1, IFIT1, ISG15, SLC1A2, IL1R2, FAM20A, RETN, MMP8 | 0,855 | 0,845 | 0,865 | 0,911 | 0,855 | 0,967 | 0,937 | 0,858 | 1 |
| RSAD2, OAS1, IFI44L, ISG15, SLC1A2, IL1R2, FAM20A, MMP8 | 0,855 | 0,845 | 0,866 | 0,914 | 0,863 | 0,965 | 0,928 | 0,838 | 1 |
| OAS1, IFIT1, IFI44L, SIGLEC1, ISG15, IL1R2, RETN, MMP8 | 0,855 | 0,845 | 0,865 | 0,901 | 0,837 | 0,965 | 0,962 | 0,923 | 1 |
| RSAD2, IFI27, OAS1, IFIT1, IFI44L, SIGLEC1, SLC1A2, MMP8 | 0,855 | 0,846 | 0,864 | 0,904 | 0,845 | 0,963 | 0,930 | 0,859 | 1 |
| OAS1, IFIT1, IFI44L, ISG15, HERC6, SLC1A2, FAM20A, RETN | 0,855 | 0,845 | 0,866 | 0,908 | 0,850 | 0,967 | 0,920 | 0,830 | 1 |
| RSAD2, IFIT1, IFI44L, ISG15, IL1R2, OLAH, FAM20A, MMP8 | 0,855 | 0,845 | 0,865 | 0,905 | 0,844 | 0,966 | 0,952 | 0,892 | 1 |
| RSAD2, IFI44L, SIGLEC1, ISG15, HERC6, IL1R2, OLAH, RETN | 0,855 | 0,845 | 0,865 | 0,903 | 0,840 | 0,966 | 0,959 | 0,917 | 1 |
| OAS1, IFIT1, IFI44L, SIGLEC1, ISG15, HERC6, OLAH, MMP8 | 0,855 | 0,845 | 0,865 | 0,900 | 0,839 | 0,962 | 0,943 | 0,893 | 0,994 |
| IFI27, OAS1, IFIT1, IFI44L, SIGLEC1, ISG15, SLC1A2, MMP8 | 0,855 | 0,846 | 0,864 | 0,904 | 0,846 | 0,963 | 0,929 | 0,861 | 0,998 |

| | | | | | | | | |
|---|---|---|---|---|---|---|---|---|
| IFIT1, IFI44L, SIGLEC1, ISG15, IL1R2, OLAH, RETN, MMP8 | 0,855 | 0,845 | 0,865 | 0,902 | 0,840 | 0,964 | 0,964 | 0,927 | 1 |
| IFIT1, IFI44L, ISG15, IL1R2, OLAH, FAM20A, RETN, MMP8 | 0,855 | 0,845 | 0,865 | 0,906 | 0,844 | 0,968 | 0,951 | 0,889 | 1 |
| RSAD2, IFIT1, IFI44L, SIGLEC1, IL1R2, OLAH, RETN, MMP8 | 0,855 | 0,845 | 0,865 | 0,902 | 0,839 | 0,965 | 0,968 | 0,934 | 1 |
| RSAD2, OAS1, IFI44L, ISG15, HERC6, FAM20A, RETN, MMP8 | 0,855 | 0,844 | 0,866 | 0,908 | 0,847 | 0,969 | 0,935 | 0,859 | 1 |
| RSAD2, OAS1, IFI44L, SIGLEC1, HERC6, SLC1A2, IL1R2, MMP8 | 0,855 | 0,845 | 0,865 | 0,907 | 0,853 | 0,961 | 0,967 | 0,931 | 1 |
| RSAD2, OAS1, IFIT1, ISG15, IL1R2, OLAH, FAM20A, RETN | 0,855 | 0,845 | 0,865 | 0,901 | 0,836 | 0,966 | 0,940 | 0,867 | 1 |
| RSAD2, IFI27, OAS1, IFIT1, IFI44L, ISG15, HERC6, MMP8 | 0,855 | 0,846 | 0,864 | 0,901 | 0,844 | 0,958 | 0,896 | 0,816 | 0,976 |
| RSAD2, IFI27, IFIT1, IFI44L, ISG15, SLC1A2, FAM20A, MMP8 | 0,855 | 0,844 | 0,866 | 0,904 | 0,840 | 0,968 | 0,936 | 0,855 | 1 |
| OAS1, IFIT1, IFI44L, SLC1A2, IL1R2, FAM20A, RETN, MMP8 | 0,855 | 0,845 | 0,865 | 0,912 | 0,858 | 0,966 | 0,932 | 0,846 | 1 |
| RSAD2, IFIT1, IFI44L, HERC6, SLC1A2, IL1R2, FAM20A, RETN | 0,855 | 0,845 | 0,865 | 0,914 | 0,862 | 0,966 | 0,923 | 0,834 | 1 |
| RSAD2, OAS1, IFI44L, SIGLEC1, SLC1A2, OLAH, RETN, MMP8 | 0,855 | 0,845 | 0,865 | 0,911 | 0,854 | 0,969 | 0,959 | 0,915 | 1 |
| RSAD2, IFI44L, SIGLEC1, ISG15, SLC1A2, IL1R2, OLAH, RETN | 0,855 | 0,845 | 0,864 | 0,912 | 0,856 | 0,968 | 0,962 | 0,923 | 1 |
| OAS1, IFI44L, SIGLEC1, ISG15, HERC6, OLAH, RETN, MMP8 | 0,855 | 0,844 | 0,865 | 0,899 | 0,833 | 0,965 | 0,964 | 0,927 | 1 |
| RSAD2, OAS1, IFIT1, IFI44L, SIGLEC1, IL1R2, OLAH, RETN | 0,855 | 0,845 | 0,865 | 0,897 | 0,831 | 0,964 | 0,961 | 0,919 | 1 |
| RSAD2, OAS1, IFIT1, IFI44L, SIGLEC1, IL1R2, RETN, MMP8 | 0,855 | 0,845 | 0,865 | 0,900 | 0,836 | 0,964 | 0,970 | 0,937 | 1 |
| RSAD2, OAS1, IFIT1, HERC6, SLC1A2, OLAH, FAM20A, RETN | 0,855 | 0,844 | 0,865 | 0,911 | 0,854 | 0,967 | 0,934 | 0,854 | 1 |
| RSAD2, OAS1, IFIT1, ISG15, SLC1A2, OLAH, FAM20A, MMP8 | 0,855 | 0,845 | 0,865 | 0,908 | 0,850 | 0,967 | 0,940 | 0,869 | 1 |
| RSAD2, OAS1, IFI44L, ISG15, SLC1A2, OLAH, FAM20A, RETN | 0,855 | 0,844 | 0,865 | 0,911 | 0,852 | 0,970 | 0,923 | 0,847 | 0,999 |
| IFI27, OAS1, IFIT1, IFI44L, ISG15, SLC1A2, FAM20A, MMP8 | 0,855 | 0,843 | 0,866 | 0,903 | 0,838 | 0,967 | 0,932 | 0,850 | 1 |
| RSAD2, OAS1, IFIT1, IFI44L, SIGLEC1, HERC6, OLAH, MMP8 | 0,855 | 0,845 | 0,864 | 0,899 | 0,837 | 0,961 | 0,941 | 0,889 | 0,993 |
| RSAD2, IFI27, OAS1, IFI44L, SIGLEC1, ISG15, SLC1A2, MMP8 | 0,855 | 0,845 | 0,864 | 0,905 | 0,846 | 0,964 | 0,933 | 0,862 | 1 |
| RSAD2, IFIT1, IFI44L, ISG15, HERC6, SLC1A2, FAM20A, RETN | 0,855 | 0,844 | 0,865 | 0,910 | 0,852 | 0,967 | 0,922 | 0,837 | 1 |
| RSAD2, OAS1, IFI44L, SIGLEC1, IL1R2, OLAH, RETN, MMP8 | 0,855 | 0,844 | 0,865 | 0,901 | 0,838 | 0,965 | 0,967 | 0,932 | 1 |
| RSAD2, IFI27, OAS1, IFI44L, ISG15, SLC1A2, FAM20A, MMP8 | 0,855 | 0,843 | 0,866 | 0,904 | 0,840 | 0,968 | 0,934 | 0,853 | 1 |
| RSAD2, OAS1, IFIT1, IFI44L, IL1R2, FAM20A, RETN, MMP8 | 0,855 | 0,844 | 0,865 | 0,908 | 0,846 | 0,970 | 0,946 | 0,871 | 1 |
| OAS1, IFI44L, ISG15, IL1R2, OLAH, FAM20A, RETN, MMP8 | 0,855 | 0,844 | 0,865 | 0,906 | 0,844 | 0,968 | 0,947 | 0,878 | 1 |
| OAS1, IFIT1, IFI44L, ISG15, HERC6, FAM20A, RETN, MMP8 | 0,855 | 0,845 | 0,865 | 0,907 | 0,844 | 0,969 | 0,927 | 0,848 | 1 |
| RSAD2, IFI44L, SIGLEC1, ISG15, HERC6, SLC1A2, IL1R2, MMP8 | 0,855 | 0,846 | 0,864 | 0,907 | 0,852 | 0,962 | 0,963 | 0,925 | 1 |

| | | | | | | | | |
|---|---|---|---|---|---|---|---|---|
| RSAD2, OAS1, IFIT1, IFI44L, SIGLEC1, HERC6, RETN, MMP8 | 0,855 | 0,845 | 0,864 | 0,900 | 0,833 | 0,967 | 0,925 | 0,865 | 0,985 |
| RSAD2, IFIT1, IFI44L, SIGLEC1, ISG15, IL1R2, OLAH, MMP8 | 0,855 | 0,845 | 0,864 | 0,901 | 0,840 | 0,962 | 0,959 | 0,917 | 1 |
| RSAD2, IFIT1, ISG15, SLC1A2, IL1R2, OLAH, FAM20A, RETN | 0,854 | 0,845 | 0,864 | 0,908 | 0,852 | 0,965 | 0,936 | 0,860 | 1 |
| RSAD2, OAS1, IFI44L, SIGLEC1, ISG15, SLC1A2, OLAH, RETN | 0,854 | 0,845 | 0,864 | 0,911 | 0,854 | 0,968 | 0,954 | 0,905 | 1 |
| RSAD2, OAS1, SIGLEC1, ISG15, IL1R2, OLAH, RETN, MMP8 | 0,854 | 0,844 | 0,865 | 0,901 | 0,838 | 0,964 | 0,970 | 0,936 | 1 |
| RSAD2, IFI27, IFI44L, SIGLEC1, ISG15, HERC6, SLC1A2, MMP8 | 0,854 | 0,845 | 0,863 | 0,906 | 0,851 | 0,961 | 0,923 | 0,854 | 0,992 |
| OAS1, IFIT1, IFI44L, SIGLEC1, ISG15, SLC1A2, IL1R2, RETN | 0,854 | 0,845 | 0,864 | 0,909 | 0,854 | 0,964 | 0,965 | 0,927 | 1 |
| RSAD2, IFIT1, IFI44L, SLC1A2, IL1R2, FAM20A, RETN, MMP8 | 0,854 | 0,845 | 0,864 | 0,911 | 0,857 | 0,966 | 0,932 | 0,848 | 1 |
| RSAD2, IFIT1, ISG15, SLC1A2, IL1R2, FAM20A, RETN, MMP8 | 0,854 | 0,845 | 0,864 | 0,911 | 0,855 | 0,966 | 0,937 | 0,859 | 1 |
| RSAD2, IFIT1, IFI44L, ISG15, SLC1A2, OLAH, FAM20A, RETN | 0,854 | 0,844 | 0,864 | 0,909 | 0,850 | 0,968 | 0,921 | 0,844 | 0,997 |
| IFIT1, IFI44L, SIGLEC1, ISG15, SLC1A2, IL1R2, RETN, MMP8 | 0,854 | 0,845 | 0,863 | 0,912 | 0,858 | 0,966 | 0,967 | 0,932 | 1 |
| RSAD2, IFI27, OAS1, IFIT1, IFI44L, SLC1A2, FAM20A, MMP8 | 0,854 | 0,843 | 0,865 | 0,903 | 0,839 | 0,967 | 0,934 | 0,853 | 1 |
| RSAD2, IFI44L, SIGLEC1, ISG15, SLC1A2, OLAH, RETN, MMP8 | 0,854 | 0,844 | 0,864 | 0,911 | 0,853 | 0,968 | 0,958 | 0,916 | 0,999 |
| RSAD2, OAS1, IFI44L, ISG15, HERC6, SLC1A2, FAM20A, RETN | 0,854 | 0,843 | 0,865 | 0,905 | 0,844 | 0,966 | 0,929 | 0,850 | 1 |
| RSAD2, OAS1, SIGLEC1, ISG15, HERC6, SLC1A2, RETN, MMP8 | 0,854 | 0,844 | 0,865 | 0,905 | 0,845 | 0,965 | 0,951 | 0,901 | 1 |
| RSAD2, OAS1, IFIT1, SIGLEC1, SLC1A2, IL1R2, RETN, MMP8 | 0,854 | 0,845 | 0,864 | 0,911 | 0,857 | 0,965 | 0,974 | 0,944 | 1 |
| RSAD2, OAS1, IFI44L, SIGLEC1, ISG15, SLC1A2, IL1R2, RETN | 0,854 | 0,845 | 0,863 | 0,906 | 0,849 | 0,963 | 0,967 | 0,929 | 1 |
| RSAD2, OAS1, IFIT1, SIGLEC1, HERC6, SLC1A2, RETN, MMP8 | 0,854 | 0,844 | 0,864 | 0,909 | 0,850 | 0,967 | 0,932 | 0,872 | 0,991 |
| RSAD2, OAS1, IFIT1, IFI44L, SIGLEC1, SLC1A2, IL1R2, RETN | 0,854 | 0,845 | 0,863 | 0,907 | 0,850 | 0,964 | 0,970 | 0,932 | 1 |
| RSAD2, OAS1, IFI44L, SIGLEC1, HERC6, OLAH, RETN, MMP8 | 0,854 | 0,844 | 0,864 | 0,898 | 0,831 | 0,965 | 0,959 | 0,918 | 0,999 |
| RSAD2, OAS1, IFI44L, SIGLEC1, ISG15, IL1R2, OLAH, MMP8 | 0,854 | 0,844 | 0,864 | 0,901 | 0,839 | 0,962 | 0,959 | 0,917 | 1 |
| OAS1, IFIT1, IFI44L, SIGLEC1, ISG15, SLC1A2, IL1R2, MMP8 | 0,854 | 0,845 | 0,863 | 0,907 | 0,853 | 0,962 | 0,964 | 0,926 | 1 |
| RSAD2, OAS1, IFIT1, IFI44L, SIGLEC1, ISG15, SLC1A2, IL1R2 | 0,854 | 0,845 | 0,863 | 0,905 | 0,849 | 0,962 | 0,970 | 0,934 | 1 |
| RSAD2, OAS1, IFIT1, IFI44L, SLC1A2, OLAH, FAM20A, RETN | 0,854 | 0,844 | 0,864 | 0,911 | 0,853 | 0,969 | 0,922 | 0,845 | 0,999 |
| RSAD2, IFIT1, SIGLEC1, ISG15, SLC1A2, IL1R2, RETN, MMP8 | 0,854 | 0,845 | 0,863 | 0,912 | 0,858 | 0,966 | 0,971 | 0,939 | 1 |
| RSAD2, IFIT1, IFI44L, SIGLEC1, SLC1A2, IL1R2, RETN, MMP8 | 0,854 | 0,845 | 0,863 | 0,911 | 0,856 | 0,966 | 0,970 | 0,937 | 1 |
| RSAD2, OAS1, IFIT1, SIGLEC1, ISG15, SLC1A2, IL1R2, RETN | 0,854 | 0,844 | 0,863 | 0,908 | 0,853 | 0,964 | 0,970 | 0,933 | 1 |
| RSAD2, OAS1, IFIT1, IFI44L, SIGLEC1, ISG15, IL1R2, RETN | 0,854 | 0,844 | 0,863 | 0,896 | 0,829 | 0,963 | 0,961 | 0,921 | 1 |

| | | | | | | | | |
|---|---|---|---|---|---|---|---|---|
| RSAD2, OAS1, IFIT1, SIGLEC1, ISG15, HERC6, OLAH, MMP8 | 0,854 | 0,844 | 0,864 | 0,900 | 0,838 | 0,962 | 0,946 | 0,897 | 0,995 |
| RSAD2, OAS1, IFIT1, ISG15, SLC1A2, IL1R2, FAM20A, RETN | 0,854 | 0,844 | 0,864 | 0,907 | 0,851 | 0,964 | 0,936 | 0,855 | 1 |
| RSAD2, IFI27, OAS1, IFIT1, IFI44L, ISG15, RETN, MMP8 | 0,854 | 0,843 | 0,864 | 0,904 | 0,841 | 0,968 | 0,952 | 0,889 | 1 |
| OAS1, IFIT1, IFI44L, SIGLEC1, ISG15, IL1R2, OLAH, RETN | 0,854 | 0,844 | 0,863 | 0,901 | 0,836 | 0,965 | 0,957 | 0,914 | 0,999 |
| RSAD2, IFIT1, IFI44L, SIGLEC1, ISG15, SLC1A2, IL1R2, RETN | 0,854 | 0,845 | 0,862 | 0,906 | 0,849 | 0,963 | 0,963 | 0,925 | 1 |
| RSAD2, IFIT1, SIGLEC1, ISG15, IL1R2, OLAH, RETN, MMP8 | 0,854 | 0,844 | 0,864 | 0,902 | 0,840 | 0,964 | 0,968 | 0,935 | 1 |
| RSAD2, IFIT1, IFI44L, SIGLEC1, ISG15, IL1R2, RETN, MMP8 | 0,854 | 0,844 | 0,863 | 0,899 | 0,835 | 0,964 | 0,967 | 0,933 | 1 |
| RSAD2, OAS1, IFI44L, ISG15, IL1R2, OLAH, FAM20A, RETN | 0,854 | 0,843 | 0,864 | 0,901 | 0,836 | 0,965 | 0,941 | 0,870 | 1 |
| RSAD2, IFI27, OAS1, IFIT1, ISG15, HERC6, SLC1A2, FAM20A | 0,854 | 0,843 | 0,864 | 0,907 | 0,849 | 0,965 | 0,932 | 0,849 | 1 |
| RSAD2, IFI44L, ISG15, HERC6, SLC1A2, FAM20A, RETN, MMP8 | 0,853 | 0,843 | 0,864 | 0,910 | 0,849 | 0,970 | 0,927 | 0,847 | 1 |
| RSAD2, OAS1, IFIT1, IFI44L, IL1R2, OLAH, FAM20A, RETN | 0,853 | 0,843 | 0,864 | 0,900 | 0,834 | 0,965 | 0,941 | 0,870 | 1 |
| RSAD2, IFI27, OAS1, IFIT1, IFI44L, ISG15, SLC1A2, FAM20A | 0,853 | 0,842 | 0,864 | 0,902 | 0,837 | 0,966 | 0,936 | 0,857 | 1 |
| RSAD2, OAS1, IFIT1, IFI44L, SIGLEC1, ISG15, IL1R2, MMP8 | 0,853 | 0,844 | 0,863 | 0,900 | 0,838 | 0,961 | 0,960 | 0,919 | 1 |
| RSAD2, OAS1, IFIT1, IFI44L, ISG15, SLC1A2, IL1R2, FAM20A | 0,853 | 0,843 | 0,863 | 0,907 | 0,851 | 0,964 | 0,926 | 0,839 | 1 |
| RSAD2, OAS1, IFIT1, IFI44L, SIGLEC1, SLC1A2, IL1R2, MMP8 | 0,853 | 0,844 | 0,862 | 0,907 | 0,851 | 0,962 | 0,971 | 0,938 | 1 |
| RSAD2, IFI27, OAS1, IFIT1, ISG15, HERC6, SLC1A2, MMP8 | 0,853 | 0,844 | 0,863 | 0,907 | 0,852 | 0,962 | 0,900 | 0,819 | 0,981 |
| OAS1, IFIT1, IFI44L, ISG15, SLC1A2, IL1R2, FAM20A, RETN | 0,853 | 0,843 | 0,863 | 0,908 | 0,852 | 0,963 | 0,925 | 0,836 | 1 |
| RSAD2, OAS1, IFIT1, SIGLEC1, ISG15, SLC1A2, IL1R2, MMP8 | 0,853 | 0,844 | 0,862 | 0,908 | 0,854 | 0,962 | 0,971 | 0,938 | 1 |
| IFI27, OAS1, IFIT1, IFI44L, ISG15, HERC6, SLC1A2, MMP8 | 0,853 | 0,844 | 0,862 | 0,907 | 0,853 | 0,961 | 0,897 | 0,817 | 0,976 |
| OAS1, IFI44L, SIGLEC1, ISG15, IL1R2, OLAH, RETN, MMP8 | 0,853 | 0,843 | 0,863 | 0,903 | 0,841 | 0,965 | 0,963 | 0,925 | 1 |
| RSAD2, IFIT1, IFI44L, SIGLEC1, ISG15, SLC1A2, IL1R2, MMP8 | 0,853 | 0,845 | 0,861 | 0,906 | 0,851 | 0,962 | 0,968 | 0,935 | 1 |
| RSAD2, IFI27, OAS1, IFIT1, IFI44L, HERC6, SLC1A2, MMP8 | 0,853 | 0,844 | 0,862 | 0,907 | 0,852 | 0,963 | 0,897 | 0,814 | 0,980 |
| OAS1, IFI44L, SIGLEC1, ISG15, SLC1A2, IL1R2, RETN, MMP8 | 0,853 | 0,844 | 0,862 | 0,912 | 0,858 | 0,966 | 0,971 | 0,938 | 1 |
| RSAD2, IFI27, OAS1, IFIT1, IFI44L, ISG15, SLC1A2, RETN | 0,853 | 0,843 | 0,863 | 0,904 | 0,840 | 0,968 | 0,945 | 0,874 | 1 |
| RSAD2, OAS1, IFIT1, SIGLEC1, ISG15, IL1R2, OLAH, MMP8 | 0,853 | 0,843 | 0,863 | 0,905 | 0,846 | 0,963 | 0,963 | 0,926 | 1 |
| RSAD2, IFIT1, IFI44L, SIGLEC1, ISG15, IL1R2, OLAH, RETN | 0,853 | 0,844 | 0,862 | 0,898 | 0,832 | 0,964 | 0,961 | 0,921 | 1 |
| RSAD2, OAS1, SIGLEC1, ISG15, SLC1A2, IL1R2, RETN, MMP8 | 0,853 | 0,843 | 0,862 | 0,912 | 0,858 | 0,965 | 0,973 | 0,941 | 1 |
| RSAD2, IFI27, OAS1, IFI44L, ISG15, HERC6, SLC1A2, MMP8 | 0,853 | 0,843 | 0,862 | 0,906 | 0,851 | 0,961 | 0,901 | 0,821 | 0,981 |

583

| | | | | | | | | | |
|---|---|---|---|---|---|---|---|---|---|
| RSAD2, OAS1, IFIT1, SIGLEC1, ISG15, IL1R2, OLAH, RETN | 0,853 | 0,843 | 0,863 | 0,898 | 0,831 | 0,964 | 0,961 | 0,920 | 1 |
| RSAD2, OAS1, IFI44L, SIGLEC1, ISG15, HERC6, OLAH, RETN | 0,853 | 0,843 | 0,863 | 0,898 | 0,832 | 0,964 | 0,952 | 0,906 | 0,998 |
| OAS1, IFIT1, IFI44L, ISG15, IL1R2, OLAH, FAM20A, RETN | 0,853 | 0,843 | 0,863 | 0,900 | 0,835 | 0,966 | 0,942 | 0,872 | 1 |
| RSAD2, OAS1, IFIT1, SIGLEC1, ISG15, IL1R2, RETN, MMP8 | 0,853 | 0,843 | 0,863 | 0,901 | 0,838 | 0,965 | 0,971 | 0,938 | 1 |
| OAS1, IFI44L, ISG15, SLC1A2, IL1R2, FAM20A, RETN, MMP8 | 0,853 | 0,842 | 0,863 | 0,912 | 0,859 | 0,965 | 0,925 | 0,832 | 1 |
| OAS1, IFIT1, IFI44L, HERC6, SLC1A2, OLAH, RETN, MMP8 | 0,853 | 0,842 | 0,863 | 0,907 | 0,848 | 0,966 | 0,936 | 0,878 | 0,993 |
| IFI27, OAS1, IFIT1, IFI44L, ISG15, SLC1A2, RETN, MMP8 | 0,853 | 0,842 | 0,863 | 0,903 | 0,840 | 0,966 | 0,941 | 0,869 | 1 |
| RSAD2, OAS1, IFI44L, SLC1A2, IL1R2, FAM20A, RETN, MMP8 | 0,853 | 0,842 | 0,863 | 0,912 | 0,859 | 0,965 | 0,926 | 0,835 | 1 |
| RSAD2, OAS1, IFIT1, SLC1A2, OLAH, FAM20A, RETN, MMP8 | 0,853 | 0,842 | 0,863 | 0,906 | 0,846 | 0,967 | 0,942 | 0,872 | 1 |
| RSAD2, OAS1, IFI44L, SIGLEC1, ISG15, IL1R2, RETN, MMP8 | 0,853 | 0,842 | 0,863 | 0,900 | 0,836 | 0,964 | 0,970 | 0,937 | 1 |
| RSAD2, IFI27, IFIT1, IFI44L, SIGLEC1, ISG15, SLC1A2, MMP8 | 0,853 | 0,843 | 0,862 | 0,903 | 0,844 | 0,962 | 0,925 | 0,855 | 0,995 |
| RSAD2, OAS1, IFI44L, SIGLEC1, ISG15, IL1R2, OLAH, RETN | 0,853 | 0,843 | 0,862 | 0,896 | 0,829 | 0,962 | 0,962 | 0,922 | 1 |
| OAS1, IFIT1, IFI44L, ISG15, IL1R2, FAM20A, RETN, MMP8 | 0,852 | 0,842 | 0,863 | 0,907 | 0,845 | 0,970 | 0,946 | 0,871 | 1 |
| RSAD2, OAS1, ISG15, SLC1A2, IL1R2, FAM20A, RETN, MMP8 | 0,852 | 0,843 | 0,862 | 0,913 | 0,860 | 0,967 | 0,928 | 0,837 | 1 |
| RSAD2, OAS1, ISG15, SLC1A2, OLAH, FAM20A, RETN, MMP8 | 0,852 | 0,842 | 0,863 | 0,909 | 0,850 | 0,967 | 0,938 | 0,864 | 1 |
| OAS1, IFIT1, ISG15, SLC1A2, OLAH, FAM20A, RETN, MMP8 | 0,852 | 0,842 | 0,863 | 0,905 | 0,845 | 0,966 | 0,939 | 0,866 | 1 |
| OAS1, IFI44L, SIGLEC1, ISG15, HERC6, SLC1A2, RETN, MMP8 | 0,852 | 0,842 | 0,863 | 0,905 | 0,844 | 0,965 | 0,952 | 0,903 | 1 |
| RSAD2, IFI44L, SIGLEC1, ISG15, IL1R2, OLAH, RETN, MMP8 | 0,852 | 0,842 | 0,862 | 0,902 | 0,838 | 0,965 | 0,967 | 0,932 | 1 |
| RSAD2, IFIT1, ISG15, IL1R2, OLAH, FAM20A, RETN, MMP8 | 0,852 | 0,842 | 0,862 | 0,905 | 0,842 | 0,967 | 0,949 | 0,882 | 1 |
| RSAD2, OAS1, IFI44L, ISG15, IL1R2, FAM20A, RETN, MMP8 | 0,852 | 0,842 | 0,863 | 0,908 | 0,848 | 0,969 | 0,941 | 0,865 | 1 |
| RSAD2, OAS1, IFIT1, IFI44L, ISG15, IL1R2, FAM20A, RETN | 0,852 | 0,842 | 0,863 | 0,902 | 0,839 | 0,965 | 0,940 | 0,865 | 1 |
| RSAD2, OAS1, IFIT1, IFI44L, SLC1A2, IL1R2, FAM20A, RETN | 0,852 | 0,842 | 0,862 | 0,907 | 0,851 | 0,963 | 0,927 | 0,841 | 1 |
| RSAD2, IFI44L, ISG15, SLC1A2, IL1R2, FAM20A, RETN, MMP8 | 0,852 | 0,842 | 0,862 | 0,912 | 0,860 | 0,965 | 0,926 | 0,835 | 1 |
| OAS1, IFIT1, IFI44L, ISG15, HERC6, SLC1A2, OLAH, RETN | 0,852 | 0,842 | 0,863 | 0,910 | 0,854 | 0,967 | 0,934 | 0,872 | 0,996 |
| RSAD2, IFIT1, IFI44L, ISG15, SLC1A2, IL1R2, FAM20A, RETN | 0,852 | 0,842 | 0,862 | 0,907 | 0,851 | 0,962 | 0,926 | 0,839 | 1 |
| RSAD2, OAS1, IFI44L, SIGLEC1, SLC1A2, IL1R2, RETN, MMP8 | 0,852 | 0,843 | 0,861 | 0,911 | 0,857 | 0,966 | 0,974 | 0,944 | 1 |
| RSAD2, IFI27, OAS1, IFI44L, ISG15, SLC1A2, RETN, MMP8 | 0,852 | 0,842 | 0,862 | 0,905 | 0,842 | 0,967 | 0,939 | 0,868 | 1 |
| RSAD2, IFIT1, ISG15, SLC1A2, OLAH, FAM20A, RETN, MMP8 | 0,852 | 0,842 | 0,862 | 0,905 | 0,845 | 0,966 | 0,941 | 0,871 | 1 |

| | | | | | | | | |
|---|---|---|---|---|---|---|---|---|
| RSAD2, OAS1, IFIT1, IFI44L, SIGLEC1, ISG15, HERC6, OLAH | 0,852 | 0,842 | 0,862 | 0,900 | 0,838 | 0,961 | 0,942 | 0,892 | 0,993 |
| RSAD2, IFIT1, IFI44L, SIGLEC1, ISG15, HERC6, OLAH, MMP8 | 0,852 | 0,842 | 0,862 | 0,900 | 0,838 | 0,962 | 0,942 | 0,892 | 0,993 |
| RSAD2, OAS1, IFI44L, SIGLEC1, ISG15, HERC6, SLC1A2, RETN | 0,852 | 0,842 | 0,862 | 0,905 | 0,844 | 0,965 | 0,952 | 0,902 | 1 |
| RSAD2, IFI44L, SIGLEC1, ISG15, SLC1A2, IL1R2, RETN, MMP8 | 0,852 | 0,843 | 0,861 | 0,911 | 0,856 | 0,966 | 0,970 | 0,937 | 1 |
| RSAD2, OAS1, IFIT1, ISG15, HERC6, FAM20A, RETN, MMP8 | 0,852 | 0,841 | 0,862 | 0,904 | 0,841 | 0,966 | 0,935 | 0,858 | 1 |
| RSAD2, IFI27, OAS1, IFIT1, ISG15, SLC1A2, RETN, MMP8 | 0,852 | 0,841 | 0,862 | 0,902 | 0,839 | 0,966 | 0,940 | 0,866 | 1 |
| RSAD2, IFI27, OAS1, IFIT1, IFI44L, SLC1A2, RETN, MMP8 | 0,852 | 0,842 | 0,862 | 0,904 | 0,841 | 0,968 | 0,940 | 0,867 | 1 |
| RSAD2, OAS1, IFIT1, IFI44L, SIGLEC1, OLAH, RETN, MMP8 | 0,852 | 0,841 | 0,862 | 0,897 | 0,829 | 0,964 | 0,955 | 0,912 | 0,999 |
| RSAD2, OAS1, ISG15, HERC6, IL1R2, OLAH, RETN, MMP8 | 0,852 | 0,841 | 0,862 | 0,901 | 0,840 | 0,963 | 0,959 | 0,918 | 1,000 |
| RSAD2, IFI44L, SIGLEC1, ISG15, HERC6, OLAH, RETN, MMP8 | 0,852 | 0,841 | 0,862 | 0,900 | 0,834 | 0,966 | 0,958 | 0,916 | 0,999 |
| RSAD2, IFIT1, IFI44L, IL1R2, OLAH, FAM20A, RETN, MMP8 | 0,852 | 0,841 | 0,862 | 0,905 | 0,843 | 0,968 | 0,951 | 0,889 | 1 |
| OAS1, IFI44L, ISG15, HERC6, SLC1A2, FAM20A, RETN, MMP8 | 0,852 | 0,840 | 0,863 | 0,906 | 0,844 | 0,968 | 0,932 | 0,850 | 1 |
| RSAD2, IFIT1, IFI44L, SIGLEC1, ISG15, HERC6, RETN, MMP8 | 0,852 | 0,842 | 0,861 | 0,899 | 0,834 | 0,965 | 0,929 | 0,872 | 0,987 |
| RSAD2, OAS1, IFI44L, SIGLEC1, ISG15, SLC1A2, IL1R2, MMP8 | 0,851 | 0,842 | 0,861 | 0,907 | 0,851 | 0,962 | 0,973 | 0,943 | 1 |
| RSAD2, IFIT1, IFI44L, SIGLEC1, ISG15, HERC6, SLC1A2, RETN | 0,851 | 0,842 | 0,860 | 0,907 | 0,849 | 0,965 | 0,927 | 0,868 | 0,987 |
| OAS1, IFIT1, IFI44L, SIGLEC1, ISG15, OLAH, RETN, MMP8 | 0,851 | 0,841 | 0,862 | 0,898 | 0,831 | 0,965 | 0,957 | 0,913 | 1,000 |
| OAS1, IFI44L, ISG15, HERC6, SLC1A2, OLAH, RETN, MMP8 | 0,851 | 0,841 | 0,862 | 0,910 | 0,852 | 0,967 | 0,947 | 0,894 | 0,999 |
| RSAD2, OAS1, IFIT1, ISG15, HERC6, SLC1A2, FAM20A, RETN | 0,851 | 0,841 | 0,862 | 0,902 | 0,841 | 0,964 | 0,936 | 0,855 | 1 |
| OAS1, IFIT1, HERC6, SLC1A2, IL1R2, OLAH, RETN, MMP8 | 0,851 | 0,841 | 0,861 | 0,905 | 0,848 | 0,963 | 0,959 | 0,917 | 1 |
| RSAD2, OAS1, IFI44L, ISG15, SLC1A2, IL1R2, FAM20A, RETN | 0,851 | 0,840 | 0,862 | 0,908 | 0,852 | 0,964 | 0,924 | 0,835 | 1 |
| RSAD2, OAS1, IFIT1, ISG15, SLC1A2, OLAH, FAM20A, RETN | 0,851 | 0,840 | 0,861 | 0,908 | 0,850 | 0,967 | 0,935 | 0,861 | 1 |
| RSAD2, OAS1, IFIT1, IFI44L, ISG15, OLAH, FAM20A, MMP8 | 0,851 | 0,840 | 0,862 | 0,898 | 0,830 | 0,966 | 0,945 | 0,885 | 1 |
| RSAD2, OAS1, IFIT1, ISG15, HERC6, IL1R2, RETN, MMP8 | 0,851 | 0,841 | 0,861 | 0,897 | 0,832 | 0,961 | 0,960 | 0,918 | 1 |
| OAS1, IFI44L, HERC6, SLC1A2, IL1R2, OLAH, RETN, MMP8 | 0,851 | 0,840 | 0,861 | 0,909 | 0,852 | 0,967 | 0,945 | 0,893 | 0,996 |
| RSAD2, OAS1, IFIT1, IFI44L, HERC6, SLC1A2, OLAH, RETN | 0,851 | 0,840 | 0,861 | 0,908 | 0,850 | 0,966 | 0,926 | 0,859 | 0,994 |
| RSAD2, OAS1, ISG15, HERC6, SLC1A2, FAM20A, RETN, MMP8 | 0,851 | 0,840 | 0,861 | 0,904 | 0,842 | 0,965 | 0,935 | 0,855 | 1 |
| IFIT1, IFI44L, ISG15, HERC6, SLC1A2, FAM20A, RETN, MMP8 | 0,851 | 0,840 | 0,861 | 0,907 | 0,846 | 0,967 | 0,935 | 0,854 | 1 |
| IFIT1, IFI44L, HERC6, SLC1A2, IL1R2, OLAH, RETN, MMP8 | 0,850 | 0,840 | 0,861 | 0,908 | 0,849 | 0,966 | 0,948 | 0,899 | 0,997 |

| Modèle | | | | | | | | |
|---|---|---|---|---|---|---|---|---|
| RSAD2, IFIT1, SIGLEC1, ISG15, HERC6, SLC1A2, RETN, MMP8 | 0,850 | 0,841 | 0,860 | 0,905 | 0,846 | 0,964 | 0,934 | 0,878 | 0,990 |
| OAS1, IFIT1, ISG15, HERC6, IL1R2, OLAH, RETN, MMP8 | 0,850 | 0,840 | 0,861 | 0,900 | 0,838 | 0,963 | 0,962 | 0,923 | 1 |
| RSAD2, OAS1, HERC6, SLC1A2, IL1R2, OLAH, RETN, MMP8 | 0,850 | 0,840 | 0,860 | 0,903 | 0,844 | 0,962 | 0,942 | 0,885 | 1 |
| OAS1, IFI44L, ISG15, HERC6, SLC1A2, IL1R2, OLAH, RETN | 0,850 | 0,839 | 0,861 | 0,908 | 0,851 | 0,965 | 0,942 | 0,884 | 1 |
| IFIT1, IFI44L, ISG15, HERC6, SLC1A2, OLAH, RETN, MMP8 | 0,850 | 0,840 | 0,860 | 0,908 | 0,850 | 0,966 | 0,941 | 0,889 | 0,993 |
| RSAD2, OAS1, IFIT1, HERC6, SLC1A2, IL1R2, OLAH, MMP8 | 0,850 | 0,840 | 0,860 | 0,904 | 0,846 | 0,961 | 0,953 | 0,908 | 0,999 |
| IFI44L, ISG15, HERC6, SLC1A2, IL1R2, OLAH, RETN, MMP8 | 0,850 | 0,840 | 0,860 | 0,909 | 0,851 | 0,967 | 0,951 | 0,904 | 0,998 |
| RSAD2, OAS1, IFI44L, HERC6, SLC1A2, IL1R2, OLAH, RETN | 0,850 | 0,839 | 0,861 | 0,905 | 0,848 | 0,963 | 0,936 | 0,874 | 0,998 |
| RSAD2, IFI44L, HERC6, SLC1A2, IL1R2, OLAH, RETN, MMP8 | 0,850 | 0,840 | 0,860 | 0,910 | 0,853 | 0,967 | 0,941 | 0,889 | 0,994 |

Performances obtenues pour l'identification de la nature virale ou bactérienne de l'infection sur la base des combinaisons à 9 gènes :

[Tableau 13]

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, HERC6, IL1R2, OLAH, FAM20A, MMP8 | 0,895 | 0,886 | 0,904 | 0,938 | 0,889 | 0,987 | 0,951 | 0,888 | 1 |
| IFI27, IFIT1, IFI44L, SIGLEC1, HERC6, IL1R2, OLAH, FAM20A, MMP8 | 0,894 | 0,885 | 0,902 | 0,937 | 0,888 | 0,986 | 0,955 | 0,894 | 1 |
| IFI27, IFIT1, SIGLEC1, ISG15, HERC6, IL1R2, OLAH, FAM20A, MMP8 | 0,894 | 0,885 | 0,903 | 0,937 | 0,887 | 0,986 | 0,953 | 0,891 | 1 |
| IFI27, IFI44L, SIGLEC1, ISG15, HERC6, IL1R2, OLAH, FAM20A, MMP8 | 0,894 | 0,885 | 0,903 | 0,936 | 0,886 | 0,986 | 0,948 | 0,884 | 1 |
| RSAD2, IFI27, IFIT1, SIGLEC1, HERC6, IL1R2, OLAH, FAM20A, MMP8 | 0,893 | 0,885 | 0,902 | 0,937 | 0,888 | 0,987 | 0,951 | 0,890 | 1 |
| IFI27, OAS1, IFIT1, SIGLEC1, HERC6, OLAH, FAM20A, RETN, MMP8 | 0,893 | 0,884 | 0,903 | 0,937 | 0,884 | 0,990 | 0,949 | 0,887 | 1 |
| RSAD2, IFI27, OAS1, SIGLEC1, HERC6, IL1R2, OLAH, FAM20A, MMP8 | 0,893 | 0,883 | 0,902 | 0,937 | 0,887 | 0,987 | 0,951 | 0,891 | 1 |
| IFI27, OAS1, SIGLEC1, ISG15, HERC6, IL1R2, OLAH, FAM20A, MMP8 | 0,893 | 0,883 | 0,902 | 0,935 | 0,884 | 0,987 | 0,950 | 0,886 | 1 |
| IFI27, IFIT1, IFI44L, SIGLEC1, ISG15, IL1R2, OLAH, FAM20A, MMP8 | 0,893 | 0,884 | 0,901 | 0,937 | 0,888 | 0,986 | 0,951 | 0,890 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, HERC6, OLAH, FAM20A, MMP8 | 0,892 | 0,883 | 0,902 | 0,935 | 0,883 | 0,986 | 0,946 | 0,884 | 1 |
| IFI27, OAS1, IFIT1, SIGLEC1, HERC6, IL1R2, FAM20A, RETN, MMP8 | 0,892 | 0,883 | 0,901 | 0,937 | 0,887 | 0,988 | 0,951 | 0,883 | 1 |
| IFI27, OAS1, IFIT1, IFI44L, SIGLEC1, HERC6, OLAH, FAM20A, MMP8 | 0,892 | 0,883 | 0,902 | 0,934 | 0,882 | 0,986 | 0,946 | 0,883 | 1 |
| IFI27, OAS1, IFIT1, SIGLEC1, ISG15, IL1R2, OLAH, FAM20A, MMP8 | 0,892 | 0,883 | 0,901 | 0,936 | 0,886 | 0,987 | 0,951 | 0,888 | 1 |
| IFI27, OAS1, IFIT1, IFI44L, SIGLEC1, HERC6, IL1R2, FAM20A, MMP8 | 0,892 | 0,883 | 0,901 | 0,934 | 0,884 | 0,983 | 0,948 | 0,881 | 1 |
| RSAD2, IFI27, OAS1, IFIT1, SIGLEC1, IL1R2, OLAH, FAM20A, MMP8 | 0,892 | 0,883 | 0,901 | 0,935 | 0,884 | 0,986 | 0,954 | 0,897 | 1 |
| IFI27, OAS1, IFIT1, IFI44L, SIGLEC1, IL1R2, OLAH, FAM20A, MMP8 | 0,892 | 0,883 | 0,901 | 0,935 | 0,885 | 0,985 | 0,955 | 0,898 | 1 |
| IFI27, OAS1, IFIT1, SIGLEC1, ISG15, HERC6, OLAH, FAM20A, MMP8 | 0,892 | 0,882 | 0,901 | 0,931 | 0,877 | 0,986 | 0,946 | 0,884 | 1 |
| RSAD2, IFI27, SIGLEC1, ISG15, HERC6, IL1R2, OLAH, FAM20A, MMP8 | 0,892 | 0,882 | 0,901 | 0,937 | 0,886 | 0,987 | 0,951 | 0,891 | 1 |
| IFI27, OAS1, IFI44L, SIGLEC1, HERC6, IL1R2, OLAH, FAM20A, MMP8 | 0,891 | 0,882 | 0,901 | 0,937 | 0,888 | 0,986 | 0,950 | 0,889 | 1 |
| RSAD2, IFI27, IFI44L, SIGLEC1, HERC6, IL1R2, OLAH, FAM20A, MMP8 | 0,891 | 0,882 | 0,900 | 0,936 | 0,887 | 0,985 | 0,951 | 0,890 | 1 |
| IFI27, IFIT1, IFI44L, SIGLEC1, ISG15, HERC6, OLAH, FAM20A, MMP8 | 0,891 | 0,882 | 0,901 | 0,932 | 0,878 | 0,986 | 0,948 | 0,886 | 1 |
| IFI27, OAS1, SIGLEC1, HERC6, IL1R2, OLAH, FAM20A, RETN, MMP8 | 0,891 | 0,881 | 0,901 | 0,935 | 0,881 | 0,988 | 0,949 | 0,885 | 1 |
| RSAD2, IFI27, IFIT1, IFI44L, SIGLEC1, HERC6, OLAH, FAM20A, MMP8 | 0,891 | 0,882 | 0,900 | 0,934 | 0,882 | 0,985 | 0,948 | 0,887 | 1 |
| IFI27, OAS1, IFI44L, SIGLEC1, ISG15, IL1R2, OLAH, FAM20A, MMP8 | 0,891 | 0,881 | 0,900 | 0,935 | 0,885 | 0,985 | 0,952 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, HERC6, OLAH, FAM20A, RETN, MMP8 | 0,891 | 0,881 | 0,900 | 0,935 | 0,880 | 0,989 | 0,948 | 0,888 | 1 |
| RSAD2, IFI27, IFIT1, SIGLEC1, ISG15, IL1R2, OLAH, FAM20A, MMP8 | 0,891 | 0,882 | 0,899 | 0,937 | 0,886 | 0,987 | 0,952 | 0,891 | 1 |
| IFI27, IFIT1, IFI44L, SIGLEC1, ISG15, HERC6, IL1R2, FAM20A, MMP8 | 0,891 | 0,882 | 0,899 | 0,932 | 0,882 | 0,983 | 0,953 | 0,886 | 1 |
| IFI27, IFIT1, IFI44L, SIGLEC1, HERC6, IL1R2, FAM20A, RETN, MMP8 | 0,890 | 0,882 | 0,899 | 0,937 | 0,887 | 0,987 | 0,951 | 0,882 | 1 |
| RSAD2, IFI27, IFIT1, IFI44L, SIGLEC1, IL1R2, OLAH, FAM20A, MMP8 | 0,890 | 0,882 | 0,899 | 0,936 | 0,886 | 0,986 | 0,954 | 0,896 | 1 |
| RSAD2, IFI27, SIGLEC1, HERC6, IL1R2, OLAH, FAM20A, RETN, MMP8 | 0,890 | 0,881 | 0,900 | 0,936 | 0,883 | 0,988 | 0,950 | 0,887 | 1 |
| IFI27, OAS1, SIGLEC1, ISG15, HERC6, OLAH, FAM20A, RETN, MMP8 | 0,890 | 0,880 | 0,900 | 0,934 | 0,879 | 0,989 | 0,948 | 0,886 | 1 |
| RSAD2, IFI27, IFIT1, SIGLEC1, ISG15, HERC6, OLAH, FAM20A, MMP8 | 0,890 | 0,881 | 0,900 | 0,934 | 0,882 | 0,987 | 0,945 | 0,883 | 1 |
| IFI27, IFIT1, SIGLEC1, HERC6, IL1R2, OLAH, FAM20A, RETN, MMP8 | 0,890 | 0,881 | 0,900 | 0,935 | 0,883 | 0,988 | 0,957 | 0,897 | 1 |
| IFI27, OAS1, IFIT1, IFI44L, SIGLEC1, HERC6, IL1R2, OLAH, FAM20A | 0,890 | 0,882 | 0,899 | 0,935 | 0,885 | 0,985 | 0,942 | 0,877 | 1 |
| RSAD2, IFI27, OAS1, IFIT1, SIGLEC1, HERC6, IL1R2, FAM20A, MMP8 | 0,890 | 0,881 | 0,899 | 0,934 | 0,884 | 0,983 | 0,951 | 0,885 | 1 |
| IFI27, IFIT1, IFI44L, SIGLEC1, ISG15, HERC6, IL1R2, OLAH, FAM20A | 0,890 | 0,881 | 0,899 | 0,932 | 0,880 | 0,985 | 0,943 | 0,879 | 1 |
| IFI27, OAS1, IFIT1, IFI44L, SIGLEC1, HERC6, OLAH, FAM20A, RETN | 0,890 | 0,881 | 0,899 | 0,931 | 0,878 | 0,985 | 0,938 | 0,873 | 1 |
| RSAD2, IFI27, IFIT1, IFI44L, SIGLEC1, HERC6, IL1R2, FAM20A, MMP8 | 0,890 | 0,882 | 0,898 | 0,934 | 0,885 | 0,984 | 0,949 | 0,880 | 1 |
| IFI27, IFIT1, SIGLEC1, ISG15, IL1R2, OLAH, FAM20A, RETN, MMP8 | 0,890 | 0,880 | 0,899 | 0,934 | 0,881 | 0,987 | 0,957 | 0,896 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, HERC6, OLAH, FAM20A, MMP8 | 0,890 | 0,880 | 0,900 | 0,934 | 0,881 | 0,988 | 0,949 | 0,889 | 1 |
| IFI27, IFIT1, IFI44L, SIGLEC1, HERC6, OLAH, FAM20A, RETN, MMP8 | 0,890 | 0,880 | 0,899 | 0,934 | 0,880 | 0,987 | 0,955 | 0,895 | 1 |
| RSAD2, IFI27, IFIT1, SIGLEC1, HERC6, IL1R2, FAM20A, RETN, MMP8 | 0,890 | 0,881 | 0,898 | 0,937 | 0,887 | 0,988 | 0,950 | 0,881 | 1 |
| RSAD2, IFI27, OAS1, IFIT1, IFI44L, SIGLEC1, HERC6, OLAH, FAM20A | 0,890 | 0,881 | 0,898 | 0,931 | 0,879 | 0,984 | 0,940 | 0,876 | 1 |
| RSAD2, IFI27, OAS1, IFIT1, SIGLEC1, HERC6, IL1R2, OLAH, FAM20A | 0,890 | 0,881 | 0,898 | 0,931 | 0,880 | 0,983 | 0,941 | 0,878 | 1 |
| IFI27, OAS1, IFIT1, SIGLEC1, ISG15, HERC6, OLAH, FAM20A, RETN | 0,890 | 0,880 | 0,899 | 0,932 | 0,879 | 0,985 | 0,937 | 0,872 | 1 |
| IFI27, IFIT1, IFI44L, SIGLEC1, ISG15, HERC6, OLAH, FAM20A, RETN | 0,889 | 0,881 | 0,898 | 0,931 | 0,876 | 0,986 | 0,941 | 0,878 | 1 |
| RSAD2, IFI27, OAS1, SIGLEC1, ISG15, IL1R2, OLAH, FAM20A, MMP8 | 0,889 | 0,880 | 0,899 | 0,936 | 0,885 | 0,987 | 0,952 | 0,892 | 1 |
| IFI27, OAS1, SIGLEC1, ISG15, IL1R2, OLAH, FAM20A, RETN, MMP8 | 0,889 | 0,880 | 0,899 | 0,933 | 0,880 | 0,987 | 0,951 | 0,889 | 1 |
| IFI27, OAS1, IFIT1, SIGLEC1, ISG15, HERC6, IL1R2, FAM20A, MMP8 | 0,889 | 0,880 | 0,898 | 0,934 | 0,885 | 0,983 | 0,952 | 0,885 | 1 |
| IFI27, IFIT1, SIGLEC1, ISG15, HERC6, OLAH, FAM20A, RETN, MMP8 | 0,889 | 0,879 | 0,900 | 0,933 | 0,879 | 0,988 | 0,954 | 0,894 | 1 |
| IFI27, IFI44L, SIGLEC1, ISG15, IL1R2, OLAH, FAM20A, RETN, MMP8 | 0,889 | 0,880 | 0,899 | 0,936 | 0,884 | 0,988 | 0,950 | 0,885 | 1 |
| RSAD2, IFI27, IFIT1, SIGLEC1, ISG15, HERC6, IL1R2, FAM20A, MMP8 | 0,889 | 0,881 | 0,898 | 0,934 | 0,884 | 0,984 | 0,953 | 0,886 | 1 |
| IFI27, IFIT1, SIGLEC1, ISG15, HERC6, IL1R2, FAM20A, RETN, MMP8 | 0,889 | 0,880 | 0,898 | 0,935 | 0,883 | 0,987 | 0,953 | 0,885 | 1 |
| RSAD2, IFI27, IFIT1, SIGLEC1, HERC6, OLAH, FAM20A, RETN, MMP8 | 0,889 | 0,879 | 0,899 | 0,937 | 0,884 | 0,990 | 0,947 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, HERC6, OLAH, FAM20A | 0,889 | 0,880 | 0,899 | 0,932 | 0,880 | 0,985 | 0,939 | 0,875 | 1 |
| IFI27, OAS1, IFIT1, SIGLEC1, ISG15, HERC6, IL1R2, OLAH, FAM20A | 0,889 | 0,880 | 0,898 | 0,934 | 0,883 | 0,985 | 0,937 | 0,871 | 1 |
| RSAD2, IFI27, SIGLEC1, ISG15, HERC6, OLAH, FAM20A, RETN, MMP8 | 0,889 | 0,879 | 0,899 | 0,936 | 0,882 | 0,990 | 0,948 | 0,887 | 1 |
| IFI27, OAS1, IFIT1, IFI44L, SIGLEC1, ISG15, OLAH, FAM20A, MMP8 | 0,889 | 0,879 | 0,899 | 0,929 | 0,872 | 0,986 | 0,949 | 0,891 | 1 |
| IFI27, OAS1, IFIT1, SIGLEC1, HERC6, IL1R2, OLAH, FAM20A, RETN | 0,889 | 0,880 | 0,898 | 0,932 | 0,879 | 0,985 | 0,939 | 0,874 | 1 |
| IFI27, OAS1, IFI44L, SIGLEC1, HERC6, IL1R2, FAM20A, RETN, MMP8 | 0,889 | 0,879 | 0,899 | 0,937 | 0,887 | 0,988 | 0,948 | 0,878 | 1 |
| RSAD2, IFI27, OAS1, IFI44L, SIGLEC1, HERC6, OLAH, FAM20A, MMP8 | 0,889 | 0,879 | 0,898 | 0,933 | 0,881 | 0,985 | 0,948 | 0,887 | 1 |
| RSAD2, IFI27, IFIT1, IFI44L, SIGLEC1, HERC6, IL1R2, OLAH, FAM20A | 0,889 | 0,880 | 0,897 | 0,934 | 0,882 | 0,986 | 0,937 | 0,871 | 1 |
| IFI27, OAS1, IFIT1, SIGLEC1, IL1R2, OLAH, FAM20A, RETN, MMP8 | 0,889 | 0,879 | 0,898 | 0,934 | 0,879 | 0,988 | 0,955 | 0,896 | 1 |
| RSAD2, IFI27, OAS1, IFI44L, SIGLEC1, IL1R2, OLAH, FAM20A, MMP8 | 0,889 | 0,879 | 0,898 | 0,935 | 0,884 | 0,985 | 0,953 | 0,895 | 1 |
| RSAD2, IFI27, IFI44L, SIGLEC1, ISG15, IL1R2, OLAH, FAM20A, MMP8 | 0,888 | 0,879 | 0,898 | 0,936 | 0,886 | 0,985 | 0,952 | 0,893 | 1 |
| IFI27, OAS1, SIGLEC1, ISG15, HERC6, IL1R2, FAM20A, RETN, MMP8 | 0,888 | 0,879 | 0,898 | 0,934 | 0,882 | 0,986 | 0,951 | 0,883 | 1 |
| IFI27, OAS1, IFI44L, SIGLEC1, ISG15, HERC6, IL1R2, FAM20A, MMP8 | 0,888 | 0,879 | 0,897 | 0,935 | 0,886 | 0,984 | 0,949 | 0,880 | 1 |
| RSAD2, IFI27, OAS1, IFIT1, SIGLEC1, ISG15, HERC6, OLAH, FAM20A | 0,888 | 0,879 | 0,897 | 0,933 | 0,880 | 0,985 | 0,939 | 0,875 | 1 |
| IFI27, IFI44L, SIGLEC1, ISG15, SLC1A2, IL1R2, OLAH, FAM20A, MMP8 | 0,888 | 0,880 | 0,897 | 0,942 | 0,899 | 0,985 | 0,946 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, HERC6, IL1R2, FAM20A, MMP8 | 0,888 | 0,879 | 0,897 | 0,934 | 0,885 | 0,984 | 0,948 | 0,881 | 1 |
| IFI27, IFIT1, SIGLEC1, ISG15, SLC1A2, IL1R2, OLAH, FAM20A, MMP8 | 0,888 | 0,879 | 0,897 | 0,944 | 0,901 | 0,986 | 0,947 | 0,879 | 1 |
| IFI27, OAS1, IFIT1, SIGLEC1, ISG15, HERC6, FAM20A, RETN, MMP8 | 0,888 | 0,879 | 0,898 | 0,932 | 0,879 | 0,986 | 0,942 | 0,873 | 1 |
| RSAD2, IFI27, OAS1, SIGLEC1, IL1R2, OLAH, FAM20A, RETN, MMP8 | 0,888 | 0,878 | 0,898 | 0,934 | 0,881 | 0,988 | 0,951 | 0,890 | 1 |
| RSAD2, IFI27, OAS1, IFIT1, IFI44L, SIGLEC1, OLAH, FAM20A, MMP8 | 0,888 | 0,879 | 0,897 | 0,928 | 0,871 | 0,985 | 0,954 | 0,899 | 1 |
| IFI27, OAS1, IFIT1, IFI44L, SIGLEC1, ISG15, HERC6, FAM20A, RETN | 0,888 | 0,879 | 0,897 | 0,931 | 0,878 | 0,984 | 0,936 | 0,864 | 1 |
| RSAD2, IFI27, OAS1, IFIT1, SIGLEC1, ISG15, OLAH, FAM20A, MMP8 | 0,888 | 0,879 | 0,897 | 0,930 | 0,873 | 0,987 | 0,949 | 0,891 | 1 |
| IFI27, OAS1, IFIT1, IFI44L, SIGLEC1, IL1R2, FAM20A, RETN, MMP8 | 0,888 | 0,879 | 0,897 | 0,934 | 0,882 | 0,985 | 0,954 | 0,893 | 1 |
| RSAD2, IFI27, OAS1, SIGLEC1, HERC6, IL1R2, FAM20A, RETN, MMP8 | 0,888 | 0,878 | 0,898 | 0,934 | 0,882 | 0,986 | 0,950 | 0,882 | 1 |
| IFI27, OAS1, IFI44L, SIGLEC1, ISG15, HERC6, OLAH, FAM20A, MMP8 | 0,888 | 0,878 | 0,898 | 0,930 | 0,875 | 0,985 | 0,946 | 0,883 | 1 |
| IFI27, IFIT1, IFI44L, SIGLEC1, IL1R2, OLAH, FAM20A, RETN, MMP8 | 0,888 | 0,878 | 0,898 | 0,935 | 0,882 | 0,988 | 0,955 | 0,895 | 1 |
| IFI27, OAS1, IFIT1, IFI44L, SIGLEC1, HERC6, IL1R2, FAM20A, RETN | 0,888 | 0,879 | 0,897 | 0,934 | 0,884 | 0,984 | 0,945 | 0,878 | 1 |
| RSAD2, IFI27, SIGLEC1, ISG15, HERC6, IL1R2, FAM20A, RETN, MMP8 | 0,888 | 0,878 | 0,897 | 0,936 | 0,884 | 0,988 | 0,949 | 0,879 | 1 |
| IFI27, IFIT1, IFI44L, SIGLEC1, ISG15, IL1R2, FAM20A, RETN, MMP8 | 0,888 | 0,879 | 0,897 | 0,935 | 0,884 | 0,986 | 0,951 | 0,882 | 1 |
| RSAD2, IFI27, SIGLEC1, ISG15, IL1R2, OLAH, FAM20A, RETN, MMP8 | 0,888 | 0,878 | 0,898 | 0,934 | 0,881 | 0,987 | 0,951 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, SIGLEC1, ISG15, HERC6, IL1R2, OLAH, FAM20A, RETN, MMP8 | 0,888 | 0,878 | 0,898 | 0,933 | 0,879 | 0,987 | 0,953 | 0,891 | 1 |
| IFI27, IFI44L, SIGLEC1, ISG15, HERC6, IL1R2, FAM20A, RETN, MMP8 | 0,888 | 0,878 | 0,897 | 0,935 | 0,884 | 0,987 | 0,950 | 0,880 | 1 |
| RSAD2, IFI27, OAS1, IFIT1, SIGLEC1, HERC6, OLAH, FAM20A, RETN | 0,888 | 0,879 | 0,897 | 0,932 | 0,879 | 0,985 | 0,939 | 0,874 | 1 |
| IFI27, IFIT1, IFI44L, SIGLEC1, HERC6, IL1R2, OLAH, FAM20A, RETN | 0,888 | 0,879 | 0,897 | 0,932 | 0,878 | 0,986 | 0,945 | 0,881 | 1 |
| RSAD2, IFI27, IFIT1, SIGLEC1, ISG15, HERC6, IL1R2, OLAH, FAM20A | 0,888 | 0,879 | 0,896 | 0,934 | 0,882 | 0,986 | 0,940 | 0,875 | 1 |
| IFI27, IFIT1, SIGLEC1, ISG15, HERC6, IL1R2, OLAH, FAM20A, RETN | 0,888 | 0,878 | 0,897 | 0,933 | 0,879 | 0,986 | 0,940 | 0,875 | 1 |
| RSAD2, IFI27, OAS1, IFI44L, HERC6, IL1R2, OLAH, FAM20A, MMP8 | 0,888 | 0,878 | 0,897 | 0,933 | 0,882 | 0,984 | 0,929 | 0,856 | 1 |
| IFI27, OAS1, IFI44L, ISG15, HERC6, IL1R2, OLAH, FAM20A, MMP8 | 0,888 | 0,878 | 0,898 | 0,931 | 0,879 | 0,983 | 0,927 | 0,851 | 1 |
| RSAD2, IFI27, OAS1, SIGLEC1, ISG15, HERC6, IL1R2, OLAH, FAM20A | 0,887 | 0,878 | 0,897 | 0,931 | 0,878 | 0,985 | 0,940 | 0,874 | 1 |
| RSAD2, IFI27, OAS1, IFIT1, IFI44L, SIGLEC1, IL1R2, FAM20A, MMP8 | 0,887 | 0,879 | 0,896 | 0,931 | 0,880 | 0,982 | 0,957 | 0,896 | 1 |
| IFI27, OAS1, IFIT1, IFI44L, HERC6, IL1R2, OLAH, FAM20A, MMP8 | 0,887 | 0,878 | 0,897 | 0,932 | 0,880 | 0,983 | 0,929 | 0,855 | 1 |
| RSAD2, IFI27, OAS1, IFI44L, SIGLEC1, HERC6, IL1R2, OLAH, FAM20A | 0,887 | 0,878 | 0,896 | 0,933 | 0,881 | 0,984 | 0,943 | 0,877 | 1 |
| IFI27, OAS1, IFIT1, SIGLEC1, HERC6, SLC1A2, OLAH, FAM20A, MMP8 | 0,887 | 0,877 | 0,897 | 0,939 | 0,892 | 0,986 | 0,941 | 0,876 | 1 |
| IFI27, IFI44L, SIGLEC1, HERC6, IL1R2, OLAH, FAM20A, RETN, MMP8 | 0,887 | 0,878 | 0,897 | 0,935 | 0,884 | 0,987 | 0,951 | 0,886 | 1 |
| RSAD2, IFI27, OAS1, IFIT1, SIGLEC1, HERC6, FAM20A, RETN, MMP8 | 0,887 | 0,878 | 0,897 | 0,933 | 0,879 | 0,987 | 0,942 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, HERC6, OLAH, FAM20A | 0,887 | 0,878 | 0,897 | 0,932 | 0,878 | 0,986 | 0,941 | 0,878 | 1 |
| IFI27, OAS1, IFIT1, SIGLEC1, ISG15, IL1R2, FAM20A, RETN, MMP8 | 0,887 | 0,878 | 0,896 | 0,934 | 0,883 | 0,986 | 0,954 | 0,891 | 1 |
| IFI27, OAS1, IFI44L, SIGLEC1, ISG15, HERC6, IL1R2, OLAH, FAM20A | 0,887 | 0,878 | 0,896 | 0,931 | 0,879 | 0,984 | 0,940 | 0,873 | 1 |
| RSAD2, IFI27, OAS1, IFIT1, HERC6, IL1R2, OLAH, FAM20A, MMP8 | 0,887 | 0,877 | 0,897 | 0,932 | 0,881 | 0,983 | 0,933 | 0,862 | 1 |
| IFI27, OAS1, SIGLEC1, ISG15, HERC6, SLC1A2, OLAH, FAM20A, MMP8 | 0,887 | 0,877 | 0,897 | 0,937 | 0,888 | 0,985 | 0,939 | 0,873 | 1 |
| IFI27, IFIT1, IFI44L, SIGLEC1, SLC1A2, IL1R2, OLAH, FAM20A, MMP8 | 0,887 | 0,878 | 0,896 | 0,939 | 0,894 | 0,985 | 0,954 | 0,893 | 1 |
| RSAD2, IFI27, OAS1, SIGLEC1, ISG15, HERC6, IL1R2, FAM20A, MMP8 | 0,887 | 0,877 | 0,896 | 0,935 | 0,885 | 0,985 | 0,950 | 0,882 | 1 |
| IFI27, IFIT1, IFI44L, SIGLEC1, ISG15, SLC1A2, OLAH, FAM20A, MMP8 | 0,887 | 0,877 | 0,896 | 0,939 | 0,891 | 0,987 | 0,947 | 0,883 | 1 |
| IFI27, OAS1, IFIT1, IFI44L, SIGLEC1, HERC6, FAM20A, RETN, MMP8 | 0,887 | 0,877 | 0,897 | 0,933 | 0,879 | 0,987 | 0,942 | 0,873 | 1 |
| IFI27, IFI44L, SIGLEC1, ISG15, HERC6, OLAH, FAM20A, RETN, MMP8 | 0,887 | 0,877 | 0,897 | 0,931 | 0,875 | 0,987 | 0,947 | 0,881 | 1 |
| RSAD2, IFI27, IFIT1, SIGLEC1, IL1R2, OLAH, FAM20A, RETN, MMP8 | 0,887 | 0,877 | 0,897 | 0,934 | 0,880 | 0,987 | 0,954 | 0,895 | 1 |
| IFI27, IFIT1, IFI44L, SIGLEC1, ISG15, HERC6, FAM20A, RETN, MMP8 | 0,887 | 0,877 | 0,896 | 0,927 | 0,871 | 0,984 | 0,947 | 0,875 | 1 |
| RSAD2, IFI27, IFI44L, SIGLEC1, HERC6, IL1R2, FAM20A, RETN, MMP8 | 0,887 | 0,877 | 0,897 | 0,937 | 0,887 | 0,987 | 0,949 | 0,879 | 1 |
| RSAD2, IFI27, IFIT1, IFI44L, SIGLEC1, HERC6, OLAH, FAM20A, RETN | 0,887 | 0,878 | 0,896 | 0,931 | 0,877 | 0,986 | 0,939 | 0,875 | 1 |
| IFI27, SIGLEC1, ISG15, HERC6, SLC1A2, IL1R2, OLAH, FAM20A, MMP8 | 0,887 | 0,877 | 0,896 | 0,940 | 0,894 | 0,985 | 0,950 | 0,885 | 1 |

593

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, HERC6, IL1R2, FAM20A | 0,887 | 0,878 | 0,895 | 0,933 | 0,883 | 0,983 | 0,947 | 0,881 | 1 |
| IFI27, OAS1, IFIT1, SIGLEC1, ISG15, OLAH, FAM20A, RETN, MMP8 | 0,887 | 0,877 | 0,897 | 0,931 | 0,874 | 0,988 | 0,954 | 0,900 | 1 |
| IFI27, OAS1, IFI44L, SIGLEC1, HERC6, OLAH, FAM20A, RETN, MMP8 | 0,887 | 0,876 | 0,897 | 0,935 | 0,882 | 0,988 | 0,948 | 0,884 | 1 |
| RSAD2, IFI27, IFI44L, SIGLEC1, ISG15, HERC6, IL1R2, FAM20A, MMP8 | 0,887 | 0,878 | 0,896 | 0,934 | 0,883 | 0,984 | 0,951 | 0,883 | 1 |
| RSAD2, IFI27, IFIT1, IFI44L, HERC6, IL1R2, OLAH, FAM20A, MMP8 | 0,887 | 0,877 | 0,896 | 0,933 | 0,881 | 0,984 | 0,930 | 0,857 | 1 |
| IFI27, IFIT1, IFI44L, SIGLEC1, ISG15, OLAH, FAM20A, RETN, MMP8 | 0,887 | 0,877 | 0,896 | 0,930 | 0,874 | 0,986 | 0,955 | 0,900 | 1 |
| RSAD2, IFI27, OAS1, IFIT1, IFI44L, SIGLEC1, HERC6, IL1R2, FAM20A | 0,887 | 0,878 | 0,895 | 0,935 | 0,886 | 0,983 | 0,946 | 0,879 | 1 |
| IFI27, OAS1, IFIT1, SIGLEC1, ISG15, SLC1A2, OLAH, FAM20A, MMP8 | 0,886 | 0,876 | 0,896 | 0,939 | 0,891 | 0,987 | 0,943 | 0,879 | 1 |
| RSAD2, IFI27, IFI44L, SIGLEC1, HERC6, OLAH, FAM20A, RETN, MMP8 | 0,886 | 0,877 | 0,896 | 0,934 | 0,881 | 0,988 | 0,950 | 0,890 | 1 |
| RSAD2, IFI27, IFIT1, SIGLEC1, HERC6, IL1R2, OLAH, FAM20A, RETN | 0,886 | 0,877 | 0,895 | 0,932 | 0,879 | 0,986 | 0,939 | 0,874 | 1 |
| RSAD2, IFI27, IFIT1, SIGLEC1, ISG15, HERC6, FAM20A, RETN, MMP8 | 0,886 | 0,877 | 0,895 | 0,931 | 0,878 | 0,985 | 0,941 | 0,869 | 1 |
| RSAD2, IFI27, SIGLEC1, ISG15, SLC1A2, IL1R2, OLAH, FAM20A, MMP8 | 0,886 | 0,877 | 0,896 | 0,942 | 0,898 | 0,985 | 0,949 | 0,884 | 1 |
| IFI27, OAS1, IFIT1, IFI44L, SIGLEC1, HERC6, SLC1A2, OLAH, FAM20A | 0,886 | 0,877 | 0,896 | 0,937 | 0,889 | 0,984 | 0,935 | 0,867 | 1 |
| RSAD2, IFI27, IFIT1, SIGLEC1, SLC1A2, IL1R2, OLAH, FAM20A, MMP8 | 0,886 | 0,877 | 0,895 | 0,939 | 0,893 | 0,985 | 0,951 | 0,889 | 1 |
| IFI27, IFIT1, IFI44L, SIGLEC1, ISG15, HERC6, IL1R2, FAM20A, RETN | 0,886 | 0,877 | 0,895 | 0,932 | 0,879 | 0,984 | 0,946 | 0,878 | 1 |

594

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, OLAH, FAM20A, MMP8 | 0,886 | 0,876 | 0,896 | 0,934 | 0,881 | 0,987 | 0,946 | 0,884 | 1 |
| RSAD2, IFI27, IFIT1, SIGLEC1, ISG15, HERC6, OLAH, FAM20A, RETN | 0,886 | 0,877 | 0,895 | 0,931 | 0,877 | 0,985 | 0,938 | 0,874 | 1 |
| RSAD2, IFI27, IFI44L, ISG15, HERC6, IL1R2, OLAH, FAM20A, MMP8 | 0,886 | 0,876 | 0,896 | 0,931 | 0,878 | 0,983 | 0,928 | 0,854 | 1 |
| RSAD2, IFI27, IFIT1, SIGLEC1, ISG15, SLC1A2, OLAH, FAM20A, MMP8 | 0,886 | 0,877 | 0,896 | 0,939 | 0,890 | 0,987 | 0,942 | 0,876 | 1 |
| IFI27, OAS1, IFIT1, SIGLEC1, SLC1A2, IL1R2, OLAH, FAM20A, MMP8 | 0,886 | 0,877 | 0,895 | 0,939 | 0,893 | 0,986 | 0,947 | 0,881 | 1 |
| IFI27, OAS1, SIGLEC1, ISG15, SLC1A2, IL1R2, OLAH, FAM20A, MMP8 | 0,886 | 0,877 | 0,896 | 0,940 | 0,895 | 0,985 | 0,947 | 0,879 | 1 |
| RSAD2, IFI27, SIGLEC1, HERC6, SLC1A2, IL1R2, OLAH, FAM20A, MMP8 | 0,886 | 0,877 | 0,896 | 0,938 | 0,892 | 0,984 | 0,948 | 0,884 | 1 |
| IFI27, IFI44L, SIGLEC1, ISG15, HERC6, IL1R2, OLAH, FAM20A, RETN | 0,886 | 0,877 | 0,895 | 0,930 | 0,877 | 0,983 | 0,942 | 0,877 | 1 |
| RSAD2, IFI27, OAS1, ISG15, HERC6, IL1R2, OLAH, FAM20A, MMP8 | 0,886 | 0,876 | 0,896 | 0,931 | 0,879 | 0,982 | 0,924 | 0,847 | 1 |
| IFI27, OAS1, IFIT1, IFI44L, SIGLEC1, OLAH, FAM20A, RETN, MMP8 | 0,886 | 0,876 | 0,896 | 0,928 | 0,870 | 0,986 | 0,959 | 0,907 | 1 |
| IFI27, IFIT1, SIGLEC1, ISG15, HERC6, SLC1A2, OLAH, FAM20A, MMP8 | 0,886 | 0,876 | 0,896 | 0,939 | 0,893 | 0,986 | 0,950 | 0,887 | 1 |
| IFI27, IFIT1, IFI44L, SIGLEC1, ISG15, IL1R2, OLAH, FAM20A, RETN | 0,886 | 0,878 | 0,895 | 0,929 | 0,873 | 0,985 | 0,947 | 0,886 | 1 |
| RSAD2, IFI27, IFIT1, IFI44L, SIGLEC1, ISG15, OLAH, FAM20A, MMP8 | 0,886 | 0,876 | 0,896 | 0,930 | 0,873 | 0,986 | 0,949 | 0,891 | 1 |
| IFI27, OAS1, IFIT1, ISG15, HERC6, IL1R2, OLAH, FAM20A, MMP8 | 0,886 | 0,876 | 0,896 | 0,930 | 0,878 | 0,982 | 0,933 | 0,862 | 1 |
| IFI27, OAS1, IFIT1, IFI44L, SIGLEC1, SLC1A2, OLAH, FAM20A, MMP8 | 0,886 | 0,876 | 0,896 | 0,938 | 0,888 | 0,988 | 0,947 | 0,887 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, HERC6, OLAH, FAM20A, MMP8 | 0,886 | 0,876 | 0,896 | 0,931 | 0,877 | 0,984 | 0,934 | 0,866 | 1 |
| IFI27, OAS1, IFIT1, IFI44L, SIGLEC1, IL1R2, OLAH, FAM20A, RETN | 0,886 | 0,877 | 0,895 | 0,926 | 0,870 | 0,983 | 0,948 | 0,885 | 1 |
| RSAD2, IFI27, OAS1, IFIT1, SIGLEC1, ISG15, HERC6, FAM20A, RETN | 0,886 | 0,877 | 0,895 | 0,930 | 0,877 | 0,984 | 0,934 | 0,861 | 1 |
| IFI27, OAS1, IFIT1, SIGLEC1, ISG15, HERC6, IL1R2, FAM20A, RETN | 0,886 | 0,877 | 0,895 | 0,934 | 0,884 | 0,985 | 0,943 | 0,876 | 1 |
| IFI27, OAS1, ISG15, HERC6, IL1R2, OLAH, FAM20A, RETN, MMP8 | 0,886 | 0,876 | 0,895 | 0,934 | 0,881 | 0,986 | 0,932 | 0,858 | 1 |
| IFI27, OAS1, SIGLEC1, HERC6, SLC1A2, IL1R2, OLAH, FAM20A, MMP8 | 0,886 | 0,876 | 0,896 | 0,938 | 0,892 | 0,985 | 0,940 | 0,871 | 1 |
| IFI27, OAS1, IFIT1, HERC6, IL1R2, OLAH, FAM20A, RETN, MMP8 | 0,886 | 0,876 | 0,895 | 0,934 | 0,882 | 0,986 | 0,929 | 0,855 | 1 |
| IFI27, OAS1, IFIT1, IFI44L, SIGLEC1, ISG15, IL1R2, FAM20A, MMP8 | 0,886 | 0,876 | 0,895 | 0,933 | 0,882 | 0,983 | 0,952 | 0,885 | 1 |
| RSAD2, IFI27, IFI44L, SIGLEC1, ISG15, HERC6, IL1R2, OLAH, FAM20A | 0,886 | 0,877 | 0,895 | 0,931 | 0,878 | 0,984 | 0,939 | 0,872 | 1 |
| RSAD2, IFI27, OAS1, IFI44L, SIGLEC1, ISG15, HERC6, OLAH, FAM20A | 0,886 | 0,876 | 0,895 | 0,930 | 0,876 | 0,985 | 0,940 | 0,874 | 1 |
| RSAD2, IFI27, OAS1, IFIT1, SIGLEC1, HERC6, IL1R2, FAM20A, RETN | 0,886 | 0,877 | 0,894 | 0,936 | 0,886 | 0,985 | 0,942 | 0,874 | 1 |
| IFI27, OAS1, SIGLEC1, ISG15, HERC6, IL1R2, OLAH, FAM20A, RETN | 0,886 | 0,876 | 0,895 | 0,931 | 0,878 | 0,984 | 0,939 | 0,871 | 1 |
| RSAD2, IFI27, IFIT1, SIGLEC1, HERC6, SLC1A2, OLAH, FAM20A, MMP8 | 0,886 | 0,876 | 0,896 | 0,938 | 0,891 | 0,985 | 0,948 | 0,885 | 1 |
| RSAD2, IFI27, OAS1, HERC6, IL1R2, OLAH, FAM20A, RETN, MMP8 | 0,886 | 0,876 | 0,895 | 0,934 | 0,883 | 0,986 | 0,932 | 0,858 | 1 |
| RSAD2, IFI27, IFIT1, SIGLEC1, ISG15, IL1R2, FAM20A, RETN, MMP8 | 0,886 | 0,877 | 0,895 | 0,936 | 0,884 | 0,987 | 0,952 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, IL1R2, FAM20A, RETN, MMP8 | 0,885 | 0,876 | 0,895 | 0,934 | 0,882 | 0,986 | 0,955 | 0,892 | 1 |
| RSAD2, IFI27, OAS1, IFIT1, SIGLEC1, OLAH, FAM20A, RETN, MMP8 | 0,885 | 0,876 | 0,895 | 0,929 | 0,872 | 0,986 | 0,954 | 0,900 | 1 |
| IFI27, OAS1, IFIT1, SIGLEC1, ISG15, IL1R2, OLAH, FAM20A, RETN | 0,885 | 0,876 | 0,894 | 0,929 | 0,872 | 0,985 | 0,945 | 0,883 | 1 |
| IFI27, OAS1, IFI44L, SIGLEC1, HERC6, IL1R2, OLAH, FAM20A, RETN | 0,885 | 0,876 | 0,895 | 0,932 | 0,880 | 0,984 | 0,943 | 0,878 | 1 |
| RSAD2, IFI27, IFIT1, IFI44L, SIGLEC1, ISG15, HERC6, FAM20A, RETN | 0,885 | 0,876 | 0,894 | 0,929 | 0,876 | 0,983 | 0,936 | 0,862 | 1 |
| IFI27, OAS1, IFIT1, IFI44L, SIGLEC1, ISG15, IL1R2, OLAH, FAM20A | 0,885 | 0,877 | 0,894 | 0,928 | 0,873 | 0,984 | 0,946 | 0,883 | 1 |
| IFI27, IFIT1, SIGLEC1, HERC6, SLC1A2, IL1R2, OLAH, FAM20A, MMP8 | 0,885 | 0,876 | 0,895 | 0,939 | 0,894 | 0,985 | 0,951 | 0,887 | 1 |
| RSAD2, IFI27, OAS1, SIGLEC1, HERC6, SLC1A2, OLAH, FAM20A, MMP8 | 0,885 | 0,875 | 0,895 | 0,935 | 0,886 | 0,985 | 0,940 | 0,875 | 1 |
| IFI27, OAS1, SIGLEC1, HERC6, SLC1A2, OLAH, FAM20A, RETN, MMP8 | 0,885 | 0,875 | 0,896 | 0,937 | 0,888 | 0,986 | 0,943 | 0,879 | 1 |
| RSAD2, IFI27, OAS1, IFIT1, SIGLEC1, ISG15, HERC6, IL1R2, FAM20A | 0,885 | 0,877 | 0,894 | 0,935 | 0,886 | 0,984 | 0,948 | 0,882 | 1 |
| RSAD2, IFI27, SIGLEC1, ISG15, HERC6, IL1R2, OLAH, FAM20A, RETN | 0,885 | 0,876 | 0,895 | 0,931 | 0,877 | 0,985 | 0,941 | 0,877 | 1 |
| IFI27, IFIT1, IFI44L, ISG15, HERC6, IL1R2, OLAH, FAM20A, MMP8 | 0,885 | 0,876 | 0,895 | 0,930 | 0,877 | 0,982 | 0,929 | 0,857 | 1 |
| IFI27, IFIT1, IFI44L, SIGLEC1, HERC6, SLC1A2, OLAH, FAM20A, MMP8 | 0,885 | 0,876 | 0,895 | 0,935 | 0,886 | 0,985 | 0,954 | 0,894 | 1 |
| IFI27, OAS1, IFI44L, SIGLEC1, IL1R2, OLAH, FAM20A, RETN, MMP8 | 0,885 | 0,876 | 0,895 | 0,934 | 0,881 | 0,988 | 0,953 | 0,896 | 1 |
| RSAD2, IFI27, OAS1, SIGLEC1, ISG15, HERC6, OLAH, FAM20A, RETN | 0,885 | 0,876 | 0,895 | 0,930 | 0,875 | 0,984 | 0,940 | 0,874 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, HERC6, IL1R2, OLAH, FAM20A, RETN, MMP8 | 0,885 | 0,876 | 0,895 | 0,933 | 0,880 | 0,985 | 0,932 | 0,860 | 1 |
| RSAD2, IFI27, OAS1, SIGLEC1, SLC1A2, IL1R2, OLAH, FAM20A, MMP8 | 0,885 | 0,876 | 0,895 | 0,938 | 0,891 | 0,985 | 0,947 | 0,883 | 1 |
| RSAD2, IFI27, OAS1, SIGLEC1, ISG15, OLAH, FAM20A, RETN, MMP8 | 0,885 | 0,875 | 0,896 | 0,931 | 0,874 | 0,988 | 0,950 | 0,893 | 1 |
| RSAD2, IFI27, OAS1, IFIT1, IFI44L, SIGLEC1, HERC6, FAM20A, RETN | 0,885 | 0,876 | 0,894 | 0,930 | 0,877 | 0,984 | 0,934 | 0,861 | 1 |
| RSAD2, IFI27, OAS1, IFIT1, SIGLEC1, ISG15, IL1R2, FAM20A, MMP8 | 0,885 | 0,876 | 0,894 | 0,933 | 0,882 | 0,983 | 0,954 | 0,893 | 1 |
| IFI27, OAS1, IFIT1, SIGLEC1, HERC6, SLC1A2, IL1R2, FAM20A, MMP8 | 0,885 | 0,875 | 0,895 | 0,937 | 0,893 | 0,980 | 0,951 | 0,884 | 1 |
| RSAD2, IFI27, OAS1, SIGLEC1, HERC6, IL1R2, OLAH, FAM20A, RETN | 0,885 | 0,875 | 0,895 | 0,931 | 0,878 | 0,984 | 0,941 | 0,875 | 1 |
| RSAD2, IFI27, SIGLEC1, ISG15, HERC6, SLC1A2, OLAH, FAM20A, MMP8 | 0,885 | 0,875 | 0,895 | 0,938 | 0,891 | 0,985 | 0,942 | 0,876 | 1 |
| IFI27, OAS1, IFI44L, SIGLEC1, ISG15, IL1R2, FAM20A, RETN, MMP8 | 0,885 | 0,875 | 0,894 | 0,934 | 0,883 | 0,986 | 0,949 | 0,879 | 1 |
| IFI27, OAS1, IFIT1, IFI44L, SIGLEC1, ISG15, OLAH, FAM20A, RETN | 0,885 | 0,876 | 0,894 | 0,927 | 0,870 | 0,985 | 0,941 | 0,878 | 1 |
| RSAD2, IFI27, OAS1, SIGLEC1, ISG15, SLC1A2, OLAH, FAM20A, MMP8 | 0,885 | 0,875 | 0,895 | 0,937 | 0,887 | 0,986 | 0,940 | 0,874 | 1 |
| IFI27, IFIT1, IFI44L, SIGLEC1, ISG15, HERC6, SLC1A2, OLAH, FAM20A | 0,885 | 0,875 | 0,894 | 0,938 | 0,892 | 0,984 | 0,937 | 0,870 | 1 |
| RSAD2, IFI27, OAS1, IFIT1, HERC6, OLAH, FAM20A, RETN, MMP8 | 0,885 | 0,875 | 0,895 | 0,931 | 0,877 | 0,986 | 0,930 | 0,858 | 1 |
| RSAD2, IFI27, OAS1, IFIT1, IFI44L, SIGLEC1, ISG15, OLAH, FAM20A | 0,885 | 0,876 | 0,894 | 0,926 | 0,868 | 0,984 | 0,943 | 0,883 | 1 |
| RSAD2, IFI27, IFI44L, SIGLEC1, IL1R2, OLAH, FAM20A, RETN, MMP8 | 0,885 | 0,875 | 0,895 | 0,935 | 0,882 | 0,988 | 0,951 | 0,890 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, SLC1A2, OLAH, FAM20A, MMP8 | 0,885 | 0,875 | 0,894 | 0,937 | 0,889 | 0,985 | 0,947 | 0,882 | 1 |
| RSAD2, IFI27, IFIT1, IFI44L, SIGLEC1, HERC6, FAM20A, RETN, MMP8 | 0,885 | 0,875 | 0,894 | 0,931 | 0,877 | 0,985 | 0,942 | 0,870 | 1 |
| IFI27, SIGLEC1, ISG15, SLC1A2, IL1R2, OLAH, FAM20A, RETN, MMP8 | 0,885 | 0,875 | 0,894 | 0,942 | 0,898 | 0,986 | 0,946 | 0,875 | 1 |
| RSAD2, IFI27, IFIT1, IFI44L, SIGLEC1, ISG15, IL1R2, FAM20A, MMP8 | 0,885 | 0,876 | 0,894 | 0,932 | 0,882 | 0,983 | 0,952 | 0,885 | 1 |
| RSAD2, IFI27, IFIT1, SIGLEC1, ISG15, OLAH, FAM20A, RETN, MMP8 | 0,885 | 0,875 | 0,895 | 0,931 | 0,875 | 0,988 | 0,954 | 0,900 | 1 |
| IFI27, OAS1, IFI44L, SIGLEC1, ISG15, OLAH, FAM20A, RETN, MMP8 | 0,885 | 0,875 | 0,895 | 0,929 | 0,872 | 0,986 | 0,950 | 0,891 | 1 |
| RSAD2, IFI27, OAS1, IFI44L, SIGLEC1, ISG15, OLAH, FAM20A, MMP8 | 0,885 | 0,874 | 0,895 | 0,930 | 0,873 | 0,986 | 0,949 | 0,890 | 1 |
| IFI27, OAS1, IFI44L, SIGLEC1, ISG15, HERC6, OLAH, FAM20A, RETN | 0,885 | 0,875 | 0,894 | 0,930 | 0,877 | 0,984 | 0,943 | 0,878 | 1 |
| RSAD2, IFI27, OAS1, IFIT1, IFI44L, HERC6, IL1R2, OLAH, FAM20A | 0,885 | 0,875 | 0,894 | 0,931 | 0,878 | 0,983 | 0,928 | 0,854 | 1 |
| IFI27, IFIT1, IFI44L, HERC6, IL1R2, OLAH, FAM20A, RETN, MMP8 | 0,885 | 0,875 | 0,894 | 0,931 | 0,878 | 0,985 | 0,934 | 0,861 | 1 |
| RSAD2, IFI27, IFIT1, IFI44L, SIGLEC1, SLC1A2, OLAH, FAM20A, MMP8 | 0,885 | 0,875 | 0,894 | 0,935 | 0,885 | 0,985 | 0,945 | 0,881 | 1 |
| RSAD2, IFI27, IFIT1, ISG15, HERC6, IL1R2, OLAH, FAM20A, MMP8 | 0,885 | 0,875 | 0,894 | 0,930 | 0,878 | 0,982 | 0,932 | 0,862 | 1 |
| RSAD2, IFI27, OAS1, IFIT1, SIGLEC1, SLC1A2, OLAH, FAM20A, MMP8 | 0,885 | 0,875 | 0,894 | 0,937 | 0,887 | 0,986 | 0,946 | 0,883 | 1 |
| RSAD2, IFI27, OAS1, SIGLEC1, ISG15, IL1R2, FAM20A, RETN, MMP8 | 0,885 | 0,875 | 0,894 | 0,935 | 0,883 | 0,987 | 0,950 | 0,883 | 1 |
| RSAD2, IFI27, OAS1, IFIT1, IFI44L, SIGLEC1, IL1R2, OLAH, FAM20A | 0,885 | 0,876 | 0,893 | 0,926 | 0,870 | 0,983 | 0,948 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, ISG15, HERC6, IL1R2, OLAH, FAM20A, RETN, MMP8 | 0,885 | 0,875 | 0,894 | 0,933 | 0,881 | 0,986 | 0,930 | 0,857 | 1 |
| IFI27, OAS1, IFIT1, SIGLEC1, ISG15, HERC6, SLC1A2, OLAH, FAM20A | 0,884 | 0,874 | 0,895 | 0,939 | 0,894 | 0,985 | 0,930 | 0,860 | 1 |
| RSAD2, IFI27, IFIT1, IFI44L, SIGLEC1, ISG15, HERC6, IL1R2, FAM20A | 0,884 | 0,876 | 0,893 | 0,933 | 0,882 | 0,983 | 0,943 | 0,875 | 1 |
| IFI27, OAS1, IFI44L, HERC6, IL1R2, OLAH, FAM20A, RETN, MMP8 | 0,884 | 0,874 | 0,895 | 0,933 | 0,881 | 0,986 | 0,930 | 0,857 | 1 |
| RSAD2, IFI27, IFI44L, HERC6, IL1R2, OLAH, FAM20A, RETN, MMP8 | 0,884 | 0,874 | 0,895 | 0,933 | 0,881 | 0,985 | 0,934 | 0,863 | 1 |
| RSAD2, IFI27, OAS1, ISG15, HERC6, OLAH, FAM20A, RETN, MMP8 | 0,884 | 0,874 | 0,894 | 0,930 | 0,876 | 0,985 | 0,929 | 0,856 | 1 |
| IFI27, OAS1, IFIT1, IFI44L, HERC6, OLAH, FAM20A, RETN, MMP8 | 0,884 | 0,874 | 0,895 | 0,932 | 0,877 | 0,986 | 0,932 | 0,859 | 1 |
| RSAD2, IFI27, IFIT1, IFI44L, SIGLEC1, HERC6, IL1R2, FAM20A, RETN | 0,884 | 0,875 | 0,894 | 0,933 | 0,881 | 0,985 | 0,948 | 0,880 | 1 |
| RSAD2, IFI27, OAS1, IFIT1, SIGLEC1, ISG15, IL1R2, OLAH, FAM20A | 0,884 | 0,876 | 0,893 | 0,929 | 0,873 | 0,984 | 0,945 | 0,881 | 1 |
| RSAD2, IFI27, OAS1, IFI44L, SIGLEC1, ISG15, IL1R2, FAM20A, MMP8 | 0,884 | 0,875 | 0,894 | 0,933 | 0,883 | 0,983 | 0,950 | 0,882 | 1 |
| IFI27, OAS1, IFI44L, SIGLEC1, ISG15, SLC1A2, OLAH, FAM20A, MMP8 | 0,884 | 0,874 | 0,894 | 0,937 | 0,888 | 0,986 | 0,940 | 0,873 | 1 |
| IFI27, IFI44L, ISG15, HERC6, IL1R2, OLAH, FAM20A, RETN, MMP8 | 0,884 | 0,874 | 0,894 | 0,932 | 0,879 | 0,985 | 0,934 | 0,862 | 1 |
| RSAD2, IFI27, OAS1, SIGLEC1, ISG15, HERC6, FAM20A, RETN, MMP8 | 0,884 | 0,874 | 0,894 | 0,932 | 0,877 | 0,987 | 0,942 | 0,872 | 1 |
| IFI27, OAS1, IFI44L, SIGLEC1, ISG15, IL1R2, OLAH, FAM20A, RETN | 0,884 | 0,875 | 0,893 | 0,928 | 0,872 | 0,983 | 0,949 | 0,889 | 1 |
| RSAD2, IFI27, IFIT1, IFI44L, SIGLEC1, OLAH, FAM20A, RETN, MMP8 | 0,884 | 0,874 | 0,894 | 0,929 | 0,872 | 0,986 | 0,955 | 0,901 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, SIGLEC1, HERC6, SLC1A2, IL1R2, FAM20A, RETN, MMP8 | 0,884 | 0,874 | 0,894 | 0,938 | 0,892 | 0,983 | 0,949 | 0,880 | 1 |
| RSAD2, IFI27, OAS1, IFIT1, SIGLEC1, HERC6, SLC1A2, OLAH, FAM20A | 0,884 | 0,874 | 0,894 | 0,937 | 0,890 | 0,984 | 0,939 | 0,874 | 1 |
| RSAD2, IFI27, OAS1, IFI44L, ISG15, HERC6, OLAH, FAM20A, MMP8 | 0,884 | 0,873 | 0,895 | 0,930 | 0,876 | 0,984 | 0,933 | 0,865 | 1 |
| RSAD2, IFI27, OAS1, IFI44L, ISG15, HERC6, IL1R2, OLAH, FAM20A | 0,884 | 0,874 | 0,893 | 0,930 | 0,877 | 0,983 | 0,928 | 0,855 | 1 |
| IFI27, OAS1, IFIT1, IFI44L, SIGLEC1, SLC1A2, IL1R2, FAM20A, MMP8 | 0,884 | 0,874 | 0,893 | 0,935 | 0,889 | 0,980 | 0,951 | 0,884 | 1 |
| RSAD2, IFI27, IFIT1, IFI44L, SIGLEC1, ISG15, IL1R2, OLAH, FAM20A | 0,884 | 0,875 | 0,892 | 0,930 | 0,876 | 0,984 | 0,946 | 0,884 | 1 |
| RSAD2, IFI27, OAS1, IFI44L, SIGLEC1, ISG15, IL1R2, OLAH, FAM20A | 0,884 | 0,875 | 0,893 | 0,927 | 0,872 | 0,983 | 0,949 | 0,889 | 1 |
| RSAD2, IFI27, OAS1, IFI44L, SIGLEC1, HERC6, OLAH, FAM20A, RETN | 0,884 | 0,874 | 0,893 | 0,932 | 0,879 | 0,985 | 0,939 | 0,871 | 1 |
| IFI27, IFIT1, SIGLEC1, ISG15, SLC1A2, OLAH, FAM20A, RETN, MMP8 | 0,884 | 0,874 | 0,894 | 0,937 | 0,889 | 0,986 | 0,948 | 0,883 | 1 |
| IFI27, IFIT1, ISG15, HERC6, IL1R2, OLAH, FAM20A, RETN, MMP8 | 0,884 | 0,874 | 0,893 | 0,932 | 0,879 | 0,985 | 0,934 | 0,862 | 1 |
| IFI27, OAS1, IFIT1, IFI44L, ISG15, HERC6, OLAH, FAM20A, RETN | 0,884 | 0,874 | 0,893 | 0,931 | 0,879 | 0,983 | 0,928 | 0,855 | 1 |
| IFI27, OAS1, IFIT1, SIGLEC1, HERC6, SLC1A2, IL1R2, OLAH, FAM20A | 0,884 | 0,874 | 0,893 | 0,938 | 0,893 | 0,984 | 0,938 | 0,871 | 1 |
| RSAD2, IFI27, OAS1, IFIT1, IFI44L, IL1R2, OLAH, FAM20A, MMP8 | 0,884 | 0,874 | 0,893 | 0,929 | 0,875 | 0,983 | 0,934 | 0,864 | 1 |
| IFI27, OAS1, IFIT1, ISG15, HERC6, OLAH, FAM20A, RETN, MMP8 | 0,884 | 0,873 | 0,894 | 0,933 | 0,878 | 0,987 | 0,935 | 0,865 | 1 |
| IFI27, OAS1, IFIT1, IFI44L, ISG15, HERC6, OLAH, FAM20A, MMP8 | 0,883 | 0,873 | 0,894 | 0,928 | 0,873 | 0,983 | 0,932 | 0,862 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, IL1R2, OLAH, FAM20A | 0,883 | 0,874 | 0,893 | 0,929 | 0,876 | 0,982 | 0,928 | 0,855 | 1 |
| RSAD2, IFI27, OAS1, IFI44L, HERC6, OLAH, FAM20A, RETN, MMP8 | 0,883 | 0,873 | 0,894 | 0,932 | 0,877 | 0,986 | 0,929 | 0,856 | 1 |
| RSAD2, IFI27, IFIT1, IFI44L, SIGLEC1, IL1R2, FAM20A, RETN, MMP8 | 0,883 | 0,873 | 0,893 | 0,935 | 0,884 | 0,987 | 0,951 | 0,885 | 1 |
| RSAD2, IFI27, IFIT1, IFI44L, HERC6, OLAH, FAM20A, RETN, MMP8 | 0,883 | 0,873 | 0,894 | 0,931 | 0,876 | 0,985 | 0,929 | 0,857 | 1 |
| RSAD2, IFI27, OAS1, SIGLEC1, ISG15, IL1R2, OLAH, FAM20A, RETN | 0,883 | 0,874 | 0,893 | 0,926 | 0,869 | 0,983 | 0,946 | 0,882 | 1 |
| RSAD2, IFI27, IFI44L, SIGLEC1, ISG15, IL1R2, FAM20A, RETN, MMP8 | 0,883 | 0,873 | 0,893 | 0,935 | 0,884 | 0,987 | 0,949 | 0,879 | 1 |
| RSAD2, IFI27, IFIT1, SIGLEC1, HERC6, SLC1A2, IL1R2, FAM20A, MMP8 | 0,883 | 0,874 | 0,893 | 0,938 | 0,894 | 0,982 | 0,953 | 0,885 | 1 |
| IFI27, OAS1, IFI44L, SIGLEC1, HERC6, SLC1A2, OLAH, FAM20A, MMP8 | 0,883 | 0,873 | 0,894 | 0,936 | 0,887 | 0,985 | 0,942 | 0,878 | 1 |
| RSAD2, IFI27, SIGLEC1, ISG15, HERC6, SLC1A2, IL1R2, FAM20A, MMP8 | 0,883 | 0,874 | 0,893 | 0,937 | 0,894 | 0,981 | 0,951 | 0,882 | 1 |
| IFI27, IFIT1, IFI44L, ISG15, HERC6, OLAH, FAM20A, RETN, MMP8 | 0,883 | 0,873 | 0,893 | 0,927 | 0,870 | 0,984 | 0,936 | 0,867 | 1 |
| RSAD2, IFI27, IFI44L, SIGLEC1, HERC6, IL1R2, OLAH, FAM20A, RETN | 0,883 | 0,873 | 0,893 | 0,932 | 0,879 | 0,985 | 0,945 | 0,880 | 1 |
| RSAD2, IFI27, OAS1, IFI44L, SIGLEC1, IL1R2, FAM20A, RETN, MMP8 | 0,883 | 0,873 | 0,893 | 0,933 | 0,881 | 0,985 | 0,952 | 0,888 | 1 |
| IFI27, OAS1, IFI44L, ISG15, HERC6, OLAH, FAM20A, RETN, MMP8 | 0,883 | 0,873 | 0,894 | 0,932 | 0,878 | 0,986 | 0,932 | 0,859 | 1 |
| RSAD2, IFI27, IFIT1, ISG15, HERC6, OLAH, FAM20A, RETN, MMP8 | 0,883 | 0,873 | 0,893 | 0,929 | 0,874 | 0,984 | 0,933 | 0,860 | 1 |
| RSAD2, IFI27, OAS1, IFIT1, ISG15, HERC6, OLAH, FAM20A, MMP8 | 0,883 | 0,873 | 0,893 | 0,929 | 0,875 | 0,983 | 0,934 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, HERC6, SLC1A2, IL1R2, OLAH, FAM20A, MMP8 | 0,883 | 0,874 | 0,893 | 0,933 | 0,884 | 0,982 | 0,951 | 0,885 | 1 |
| IFI27, OAS1, IFI44L, SIGLEC1, ISG15, HERC6, IL1R2, FAM20A, RETN | 0,883 | 0,874 | 0,892 | 0,935 | 0,885 | 0,984 | 0,943 | 0,876 | 1 |
| IFI27, OAS1, IFI44L, SIGLEC1, HERC6, SLC1A2, IL1R2, FAM20A, MMP8 | 0,883 | 0,873 | 0,893 | 0,936 | 0,892 | 0,981 | 0,948 | 0,879 | 1 |
| IFI27, IFIT1, IFI44L, SIGLEC1, HERC6, SLC1A2, IL1R2, FAM20A, MMP8 | 0,883 | 0,874 | 0,892 | 0,936 | 0,891 | 0,981 | 0,955 | 0,888 | 1 |
| RSAD2, IFI27, IFIT1, SIGLEC1, ISG15, HERC6, IL1R2, FAM20A, RETN | 0,883 | 0,874 | 0,892 | 0,933 | 0,881 | 0,986 | 0,949 | 0,882 | 1 |
| RSAD2, IFI27, OAS1, IFIT1, SIGLEC1, IL1R2, OLAH, FAM20A, RETN | 0,883 | 0,874 | 0,892 | 0,926 | 0,869 | 0,983 | 0,949 | 0,886 | 1 |
| RSAD2, IFI27, IFIT1, SIGLEC1, ISG15, IL1R2, OLAH, FAM20A, RETN | 0,883 | 0,874 | 0,892 | 0,929 | 0,872 | 0,985 | 0,945 | 0,881 | 1 |
| IFI27, IFIT1, IFI44L, SIGLEC1, ISG15, SLC1A2, IL1R2, FAM20A, MMP8 | 0,883 | 0,874 | 0,892 | 0,938 | 0,896 | 0,981 | 0,954 | 0,886 | 1 |
| RSAD2, IFI27, IFIT1, IFI44L, SIGLEC1, HERC6, SLC1A2, OLAH, FAM20A | 0,883 | 0,873 | 0,893 | 0,936 | 0,888 | 0,984 | 0,939 | 0,874 | 1 |
| IFI27, OAS1, IFI44L, SIGLEC1, SLC1A2, IL1R2, OLAH, FAM20A, MMP8 | 0,883 | 0,873 | 0,893 | 0,938 | 0,891 | 0,985 | 0,948 | 0,884 | 1 |
| IFI27, IFIT1, IFI44L, SIGLEC1, HERC6, SLC1A2, IL1R2, OLAH, FAM20A | 0,883 | 0,874 | 0,892 | 0,935 | 0,887 | 0,983 | 0,941 | 0,876 | 1 |
| RSAD2, IFI27, OAS1, IFIT1, ISG15, HERC6, IL1R2, OLAH, FAM20A | 0,883 | 0,874 | 0,892 | 0,929 | 0,877 | 0,982 | 0,926 | 0,851 | 1 |
| IFI27, OAS1, IFIT1, SIGLEC1, SLC1A2, IL1R2, FAM20A, RETN, MMP8 | 0,883 | 0,873 | 0,893 | 0,938 | 0,892 | 0,983 | 0,950 | 0,880 | 1 |
| RSAD2, IFI27, IFIT1, IFI44L, ISG15, HERC6, OLAH, FAM20A, MMP8 | 0,883 | 0,873 | 0,893 | 0,930 | 0,876 | 0,984 | 0,933 | 0,865 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, SLC1A2, OLAH, FAM20A | 0,883 | 0,873 | 0,892 | 0,933 | 0,882 | 0,985 | 0,937 | 0,870 | 1 |
| RSAD2, IFI27, IFI44L, SIGLEC1, SLC1A2, IL1R2, OLAH, FAM20A, MMP8 | 0,883 | 0,873 | 0,893 | 0,937 | 0,890 | 0,984 | 0,949 | 0,886 | 1 |
| RSAD2, IFI27, OAS1, IFI44L, SIGLEC1, ISG15, HERC6, IL1R2, FAM20A | 0,883 | 0,874 | 0,892 | 0,933 | 0,884 | 0,982 | 0,946 | 0,880 | 1 |
| RSAD2, IFI27, SIGLEC1, ISG15, SLC1A2, OLAH, FAM20A, RETN, MMP8 | 0,883 | 0,873 | 0,893 | 0,938 | 0,890 | 0,987 | 0,945 | 0,880 | 1 |
| RSAD2, IFI27, OAS1, IFIT1, IFI44L, SIGLEC1, OLAH, FAM20A, RETN | 0,883 | 0,873 | 0,893 | 0,924 | 0,865 | 0,983 | 0,951 | 0,891 | 1 |
| IFI27, IFIT1, IFI44L, SIGLEC1, ISG15, SLC1A2, IL1R2, OLAH, FAM20A | 0,883 | 0,874 | 0,892 | 0,937 | 0,889 | 0,984 | 0,937 | 0,869 | 1 |
| RSAD2, IFI27, SIGLEC1, SLC1A2, IL1R2, OLAH, FAM20A, RETN, MMP8 | 0,883 | 0,873 | 0,893 | 0,938 | 0,890 | 0,985 | 0,949 | 0,885 | 1 |
| RSAD2, IFI27, OAS1, IFIT1, IFI44L, ISG15, HERC6, OLAH, FAM20A | 0,883 | 0,873 | 0,893 | 0,930 | 0,875 | 0,984 | 0,927 | 0,855 | 0,999 |
| IFI27, OAS1, IFIT1, IFI44L, ISG15, IL1R2, OLAH, FAM20A, MMP8 | 0,883 | 0,873 | 0,893 | 0,929 | 0,876 | 0,981 | 0,935 | 0,867 | 1 |
| RSAD2, IFI27, SIGLEC1, HERC6, SLC1A2, IL1R2, FAM20A, RETN, MMP8 | 0,883 | 0,873 | 0,893 | 0,937 | 0,889 | 0,985 | 0,950 | 0,880 | 1 |
| IFI27, IFIT1, SIGLEC1, HERC6, SLC1A2, OLAH, FAM20A, RETN, MMP8 | 0,883 | 0,873 | 0,893 | 0,938 | 0,889 | 0,986 | 0,951 | 0,887 | 1 |
| IFI27, IFIT1, SIGLEC1, HERC6, SLC1A2, IL1R2, FAM20A, RETN, MMP8 | 0,883 | 0,873 | 0,892 | 0,938 | 0,892 | 0,985 | 0,954 | 0,886 | 1 |
| RSAD2, IFI27, IFI44L, SIGLEC1, ISG15, HERC6, OLAH, FAM20A, RETN | 0,883 | 0,873 | 0,892 | 0,930 | 0,876 | 0,984 | 0,940 | 0,874 | 1 |
| IFI27, IFIT1, SIGLEC1, SLC1A2, IL1R2, OLAH, FAM20A, RETN, MMP8 | 0,883 | 0,873 | 0,892 | 0,939 | 0,893 | 0,985 | 0,950 | 0,883 | 1 |
| IFI27, OAS1, IFIT1, SIGLEC1, HERC6, SLC1A2, FAM20A, RETN, MMP8 | 0,883 | 0,873 | 0,892 | 0,935 | 0,885 | 0,985 | 0,940 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, ISG15, HERC6, FAM20A, RETN, MMP8 | 0,883 | 0,872 | 0,893 | 0,931 | 0,876 | 0,985 | 0,943 | 0,871 | 1 |
| RSAD2, IFI27, OAS1, IFIT1, ISG15, IL1R2, OLAH, FAM20A, MMP8 | 0,882 | 0,873 | 0,892 | 0,929 | 0,875 | 0,982 | 0,935 | 0,867 | 1 |
| IFI27, IFIT1, SIGLEC1, ISG15, HERC6, SLC1A2, IL1R2, FAM20A, MMP8 | 0,882 | 0,873 | 0,892 | 0,937 | 0,894 | 0,980 | 0,954 | 0,886 | 1 |
| IFI27, IFIT1, IFI44L, SIGLEC1, SLC1A2, IL1R2, FAM20A, RETN, MMP8 | 0,882 | 0,873 | 0,892 | 0,938 | 0,892 | 0,984 | 0,953 | 0,885 | 1 |
| IFI27, OAS1, SIGLEC1, ISG15, HERC6, SLC1A2, IL1R2, OLAH, FAM20A | 0,882 | 0,872 | 0,892 | 0,935 | 0,887 | 0,983 | 0,936 | 0,866 | 1 |
| RSAD2, IFI27, OAS1, IFIT1, IFI44L, SIGLEC1, SLC1A2, OLAH, FAM20A | 0,882 | 0,873 | 0,892 | 0,931 | 0,877 | 0,984 | 0,941 | 0,876 | 1 |
| IFI27, OAS1, IFIT1, IFI44L, HERC6, IL1R2, OLAH, FAM20A, RETN | 0,882 | 0,873 | 0,892 | 0,931 | 0,879 | 0,983 | 0,928 | 0,855 | 1 |
| IFI27, IFI44L, SIGLEC1, ISG15, HERC6, SLC1A2, IL1R2, FAM20A, MMP8 | 0,882 | 0,873 | 0,892 | 0,938 | 0,895 | 0,981 | 0,952 | 0,883 | 1 |
| RSAD2, IFI27, OAS1, IFI44L, ISG15, IL1R2, OLAH, FAM20A, MMP8 | 0,882 | 0,872 | 0,893 | 0,930 | 0,877 | 0,983 | 0,928 | 0,856 | 1 |
| RSAD2, IFI27, SIGLEC1, HERC6, SLC1A2, OLAH, FAM20A, RETN, MMP8 | 0,882 | 0,872 | 0,893 | 0,937 | 0,889 | 0,986 | 0,947 | 0,881 | 1 |
| IFI27, IFIT1, IFI44L, SIGLEC1, SLC1A2, OLAH, FAM20A, RETN, MMP8 | 0,882 | 0,872 | 0,892 | 0,935 | 0,885 | 0,986 | 0,952 | 0,891 | 1 |
| IFI27, OAS1, IFI44L, ISG15, HERC6, IL1R2, OLAH, FAM20A, RETN | 0,882 | 0,873 | 0,892 | 0,931 | 0,878 | 0,983 | 0,929 | 0,856 | 1 |
| IFI27, OAS1, IFIT1, SIGLEC1, HERC6, SLC1A2, OLAH, FAM20A, RETN | 0,882 | 0,872 | 0,892 | 0,939 | 0,895 | 0,984 | 0,936 | 0,869 | 1 |
| RSAD2, IFI27, OAS1, IFIT1, SIGLEC1, ISG15, OLAH, FAM20A, RETN | 0,882 | 0,873 | 0,892 | 0,926 | 0,868 | 0,984 | 0,942 | 0,879 | 1 |
| RSAD2, IFI27, OAS1, SIGLEC1, ISG15, HERC6, IL1R2, FAM20A, RETN | 0,882 | 0,872 | 0,892 | 0,931 | 0,880 | 0,983 | 0,943 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, HERC6, IL1R2, FAM20A, RETN | 0,882 | 0,873 | 0,892 | 0,934 | 0,883 | 0,984 | 0,942 | 0,872 | 1 |
| RSAD2, IFI27, OAS1, IFI44L, SIGLEC1, OLAH, FAM20A, RETN, MMP8 | 0,882 | 0,872 | 0,892 | 0,929 | 0,871 | 0,986 | 0,955 | 0,900 | 1 |
| IFI27, IFIT1, SIGLEC1, ISG15, HERC6, SLC1A2, IL1R2, OLAH, FAM20A | 0,882 | 0,873 | 0,892 | 0,939 | 0,894 | 0,984 | 0,940 | 0,875 | 1 |
| IFI27, OAS1, SIGLEC1, ISG15, SLC1A2, OLAH, FAM20A, RETN, MMP8 | 0,882 | 0,872 | 0,893 | 0,936 | 0,886 | 0,986 | 0,945 | 0,879 | 1 |
| RSAD2, IFI27, IFI44L, SIGLEC1, HERC6, SLC1A2, OLAH, FAM20A, MMP8 | 0,882 | 0,872 | 0,892 | 0,937 | 0,888 | 0,985 | 0,947 | 0,883 | 1 |
| IFI27, IFIT1, SIGLEC1, ISG15, SLC1A2, IL1R2, FAM20A, RETN, MMP8 | 0,882 | 0,873 | 0,891 | 0,939 | 0,895 | 0,984 | 0,953 | 0,885 | 1 |
| RSAD2, IFI27, OAS1, IFIT1, IFI44L, HERC6, OLAH, FAM20A, RETN | 0,882 | 0,873 | 0,892 | 0,931 | 0,879 | 0,983 | 0,928 | 0,855 | 1 |
| IFI27, IFI44L, SIGLEC1, SLC1A2, IL1R2, OLAH, FAM20A, RETN, MMP8 | 0,882 | 0,873 | 0,891 | 0,938 | 0,891 | 0,985 | 0,952 | 0,888 | 1 |
| IFI27, OAS1, SIGLEC1, SLC1A2, IL1R2, OLAH, FAM20A, RETN, MMP8 | 0,882 | 0,872 | 0,892 | 0,935 | 0,887 | 0,984 | 0,950 | 0,886 | 1 |
| IFI27, IFIT1, IFI44L, ISG15, IL1R2, OLAH, FAM20A, RETN, MMP8 | 0,882 | 0,872 | 0,892 | 0,927 | 0,872 | 0,983 | 0,934 | 0,864 | 1 |
| IFI27, IFI44L, SIGLEC1, ISG15, SLC1A2, OLAH, FAM20A, RETN, MMP8 | 0,882 | 0,872 | 0,892 | 0,935 | 0,886 | 0,985 | 0,946 | 0,878 | 1 |
| RSAD2, IFI27, OAS1, SIGLEC1, HERC6, SLC1A2, IL1R2, FAM20A, MMP8 | 0,882 | 0,872 | 0,892 | 0,937 | 0,891 | 0,982 | 0,948 | 0,879 | 1 |
| RSAD2, IFI27, OAS1, IFI44L, SIGLEC1, SLC1A2, OLAH, FAM20A, MMP8 | 0,882 | 0,872 | 0,892 | 0,935 | 0,883 | 0,986 | 0,943 | 0,880 | 1 |
| RSAD2, IFI27, IFIT1, SIGLEC1, ISG15, SLC1A2, IL1R2, FAM20A, MMP8 | 0,882 | 0,873 | 0,891 | 0,939 | 0,896 | 0,981 | 0,953 | 0,885 | 1 |
| IFI27, OAS1, IFIT1, IFI44L, HERC6, IL1R2, FAM20A, RETN, MMP8 | 0,882 | 0,872 | 0,892 | 0,930 | 0,878 | 0,983 | 0,929 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, SLC1A2, OLAH, FAM20A, MMP8 | 0,882 | 0,871 | 0,892 | 0,939 | 0,891 | 0,987 | 0,941 | 0,875 | 1 |
| RSAD2, IFI27, IFIT1, SIGLEC1, ISG15, HERC6, SLC1A2, OLAH, FAM20A | 0,882 | 0,872 | 0,892 | 0,937 | 0,891 | 0,984 | 0,933 | 0,864 | 1 |
| IFI27, OAS1, SIGLEC1, ISG15, HERC6, SLC1A2, IL1R2, FAM20A, MMP8 | 0,882 | 0,872 | 0,892 | 0,936 | 0,892 | 0,980 | 0,948 | 0,879 | 1 |
| RSAD2, IFI27, IFIT1, SIGLEC1, HERC6, SLC1A2, IL1R2, OLAH, FAM20A | 0,882 | 0,872 | 0,892 | 0,937 | 0,891 | 0,984 | 0,938 | 0,872 | 1 |
| RSAD2, IFI27, IFIT1, IFI44L, SIGLEC1, SLC1A2, IL1R2, FAM20A, MMP8 | 0,882 | 0,873 | 0,891 | 0,936 | 0,891 | 0,981 | 0,953 | 0,885 | 1 |
| IFI27, OAS1, IFIT1, SIGLEC1, SLC1A2, OLAH, FAM20A, RETN, MMP8 | 0,882 | 0,871 | 0,892 | 0,933 | 0,881 | 0,985 | 0,952 | 0,893 | 1 |
| RSAD2, IFI27, IFIT1, IFI44L, SIGLEC1, ISG15, OLAH, FAM20A, RETN | 0,882 | 0,872 | 0,891 | 0,927 | 0,870 | 0,984 | 0,946 | 0,887 | 1 |
| IFI27, OAS1, IFI44L, SIGLEC1, ISG15, HERC6, SLC1A2, OLAH, FAM20A | 0,882 | 0,872 | 0,892 | 0,935 | 0,887 | 0,984 | 0,936 | 0,866 | 1 |
| IFI27, IFIT1, IFI44L, SIGLEC1, ISG15, SLC1A2, OLAH, FAM20A, RETN | 0,882 | 0,873 | 0,891 | 0,935 | 0,885 | 0,985 | 0,939 | 0,873 | 1 |
| IFI27, OAS1, IFIT1, SIGLEC1, HERC6, IL1R2, OLAH, RETN, MMP8 | 0,882 | 0,873 | 0,891 | 0,927 | 0,874 | 0,981 | 0,958 | 0,909 | 1 |
| IFI27, OAS1, IFIT1, IFI44L, SIGLEC1, HERC6, SLC1A2, IL1R2, FAM20A | 0,882 | 0,873 | 0,891 | 0,937 | 0,893 | 0,981 | 0,941 | 0,872 | 1 |
| RSAD2, IFI27, IFI44L, SIGLEC1, HERC6, SLC1A2, IL1R2, FAM20A, MMP8 | 0,882 | 0,872 | 0,892 | 0,935 | 0,890 | 0,981 | 0,954 | 0,886 | 1 |
| RSAD2, IFI27, IFI44L, SIGLEC1, ISG15, IL1R2, OLAH, FAM20A, RETN | 0,882 | 0,872 | 0,891 | 0,928 | 0,872 | 0,983 | 0,945 | 0,881 | 1 |
| RSAD2, IFI27, IFIT1, IFI44L, ISG15, IL1R2, OLAH, FAM20A, MMP8 | 0,882 | 0,872 | 0,892 | 0,929 | 0,877 | 0,982 | 0,935 | 0,866 | 1 |
| IFI27, OAS1, IFIT1, SIGLEC1, ISG15, SLC1A2, IL1R2, FAM20A, MMP8 | 0,882 | 0,872 | 0,891 | 0,938 | 0,895 | 0,981 | 0,951 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, SLC1A2, IL1R2, OLAH, FAM20A | 0,882 | 0,872 | 0,891 | 0,937 | 0,890 | 0,983 | 0,940 | 0,871 | 1 |
| RSAD2, IFI27, IFIT1, IFI44L, ISG15, HERC6, IL1R2, OLAH, FAM20A | 0,882 | 0,872 | 0,891 | 0,929 | 0,875 | 0,983 | 0,929 | 0,857 | 1 |
| RSAD2, IFI27, IFIT1, SIGLEC1, SLC1A2, OLAH, FAM20A, RETN, MMP8 | 0,882 | 0,872 | 0,892 | 0,936 | 0,886 | 0,986 | 0,949 | 0,886 | 1 |
| IFI27, OAS1, IFIT1, ISG15, HERC6, IL1R2, OLAH, FAM20A, RETN | 0,882 | 0,872 | 0,891 | 0,930 | 0,878 | 0,982 | 0,928 | 0,855 | 1 |
| IFI27, OAS1, IFIT1, IFI44L, SIGLEC1, ISG15, IL1R2, FAM20A, RETN | 0,882 | 0,873 | 0,891 | 0,929 | 0,875 | 0,983 | 0,949 | 0,886 | 1 |
| RSAD2, IFI27, IFI44L, SIGLEC1, ISG15, OLAH, FAM20A, RETN, MMP8 | 0,882 | 0,871 | 0,892 | 0,931 | 0,875 | 0,987 | 0,950 | 0,891 | 1 |
| RSAD2, IFI27, IFIT1, IFI44L, SIGLEC1, IL1R2, OLAH, FAM20A, RETN | 0,882 | 0,872 | 0,892 | 0,927 | 0,871 | 0,984 | 0,947 | 0,884 | 1 |
| IFI27, IFI44L, ISG15, HERC6, SLC1A2, IL1R2, OLAH, FAM20A, MMP8 | 0,882 | 0,872 | 0,891 | 0,935 | 0,888 | 0,982 | 0,922 | 0,841 | 1 |
| RSAD2, IFI27, OAS1, IFI44L, SIGLEC1, IL1R2, OLAH, FAM20A, RETN | 0,882 | 0,872 | 0,891 | 0,926 | 0,869 | 0,982 | 0,952 | 0,892 | 1 |
| IFI27, SIGLEC1, ISG15, HERC6, SLC1A2, OLAH, FAM20A, RETN, MMP8 | 0,882 | 0,871 | 0,892 | 0,934 | 0,884 | 0,984 | 0,948 | 0,880 | 1 |
| IFI27, IFIT1, IFI44L, ISG15, HERC6, IL1R2, OLAH, FAM20A, RETN | 0,881 | 0,872 | 0,891 | 0,929 | 0,875 | 0,982 | 0,929 | 0,856 | 1 |
| RSAD2, IFI27, OAS1, IFIT1, SIGLEC1, SLC1A2, IL1R2, FAM20A, MMP8 | 0,881 | 0,872 | 0,891 | 0,934 | 0,887 | 0,980 | 0,952 | 0,885 | 1 |
| RSAD2, IFI27, IFIT1, SIGLEC1, SLC1A2, IL1R2, FAM20A, RETN, MMP8 | 0,881 | 0,872 | 0,891 | 0,938 | 0,892 | 0,985 | 0,952 | 0,883 | 1 |
| RSAD2, IFI27, IFIT1, IFI44L, SIGLEC1, ISG15, SLC1A2, OLAH, FAM20A | 0,881 | 0,872 | 0,891 | 0,935 | 0,885 | 0,985 | 0,939 | 0,873 | 1 |
| IFI27, IFIT1, IFI44L, SIGLEC1, HERC6, SLC1A2, OLAH, FAM20A, RETN | 0,881 | 0,872 | 0,891 | 0,936 | 0,887 | 0,985 | 0,941 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, HERC6, SLC1A2, IL1R2, OLAH, FAM20A | 0,881 | 0,871 | 0,891 | 0,932 | 0,882 | 0,982 | 0,936 | 0,867 | 1 |
| IFI27, OAS1, IFIT1, IFI44L, SIGLEC1, SLC1A2, IL1R2, OLAH, FAM20A | 0,881 | 0,872 | 0,891 | 0,932 | 0,881 | 0,984 | 0,939 | 0,873 | 1 |
| IFI27, IFI44L, ISG15, SLC1A2, IL1R2, OLAH, FAM20A, RETN, MMP8 | 0,881 | 0,872 | 0,891 | 0,935 | 0,887 | 0,983 | 0,922 | 0,841 | 1 |
| IFI27, OAS1, SIGLEC1, ISG15, SLC1A2, IL1R2, FAM20A, RETN, MMP8 | 0,881 | 0,872 | 0,891 | 0,937 | 0,892 | 0,982 | 0,948 | 0,877 | 1 |
| IFI27, IFIT1, SIGLEC1, ISG15, SLC1A2, IL1R2, OLAH, FAM20A, RETN | 0,881 | 0,872 | 0,891 | 0,936 | 0,888 | 0,984 | 0,936 | 0,868 | 1 |
| IFI27, OAS1, IFIT1, IFI44L, SIGLEC1, SLC1A2, OLAH, FAM20A, RETN | 0,881 | 0,872 | 0,891 | 0,930 | 0,877 | 0,984 | 0,940 | 0,875 | 1 |
| RSAD2, IFI27, SIGLEC1, ISG15, SLC1A2, IL1R2, FAM20A, RETN, MMP8 | 0,881 | 0,871 | 0,891 | 0,937 | 0,891 | 0,983 | 0,949 | 0,879 | 1 |
| RSAD2, IFI27, IFI44L, ISG15, HERC6, OLAH, FAM20A, RETN, MMP8 | 0,881 | 0,871 | 0,892 | 0,929 | 0,874 | 0,984 | 0,930 | 0,859 | 1 |
| IFI27, IFI44L, SIGLEC1, ISG15, SLC1A2, IL1R2, FAM20A, RETN, MMP8 | 0,881 | 0,871 | 0,891 | 0,938 | 0,892 | 0,984 | 0,950 | 0,879 | 1 |
| RSAD2, IFI27, OAS1, IFIT1, ISG15, HERC6, OLAH, FAM20A, RETN | 0,881 | 0,872 | 0,891 | 0,930 | 0,878 | 0,983 | 0,934 | 0,864 | 1 |
| IFI27, OAS1, IFIT1, IFI44L, SIGLEC1, HERC6, SLC1A2, FAM20A, RETN | 0,881 | 0,872 | 0,890 | 0,933 | 0,884 | 0,981 | 0,936 | 0,865 | 1 |
| RSAD2, IFI27, OAS1, SIGLEC1, ISG15, HERC6, SLC1A2, OLAH, FAM20A | 0,881 | 0,871 | 0,891 | 0,935 | 0,887 | 0,984 | 0,935 | 0,864 | 1 |
| RSAD2, IFI27, OAS1, HERC6, SLC1A2, IL1R2, OLAH, FAM20A, MMP8 | 0,881 | 0,871 | 0,891 | 0,935 | 0,889 | 0,982 | 0,918 | 0,835 | 1 |
| RSAD2, IFI27, SIGLEC1, ISG15, HERC6, SLC1A2, IL1R2, OLAH, FAM20A | 0,881 | 0,871 | 0,891 | 0,935 | 0,888 | 0,983 | 0,938 | 0,870 | 1 |
| RSAD2, IFI27, OAS1, IFI44L, SIGLEC1, HERC6, FAM20A, RETN, MMP8 | 0,881 | 0,871 | 0,891 | 0,931 | 0,877 | 0,986 | 0,942 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, HERC6, OLAH, RETN, MMP8 | 0,881 | 0,872 | 0,890 | 0,926 | 0,872 | 0,980 | 0,950 | 0,897 | 1 |
| IFI27, IFIT1, IFI44L, ISG15, SLC1A2, IL1R2, OLAH, FAM20A, MMP8 | 0,881 | 0,872 | 0,890 | 0,934 | 0,886 | 0,982 | 0,924 | 0,846 | 1 |
| RSAD2, IFI27, OAS1, IFI44L, SIGLEC1, ISG15, OLAH, FAM20A, RETN | 0,881 | 0,871 | 0,890 | 0,924 | 0,865 | 0,982 | 0,948 | 0,888 | 1 |
| IFI27, OAS1, IFIT1, ISG15, IL1R2, OLAH, FAM20A, RETN, MMP8 | 0,881 | 0,871 | 0,891 | 0,927 | 0,872 | 0,983 | 0,934 | 0,864 | 1 |
| RSAD2, IFI27, OAS1, IFIT1, SIGLEC1, ISG15, SLC1A2, OLAH, FAM20A | 0,881 | 0,871 | 0,890 | 0,933 | 0,882 | 0,984 | 0,941 | 0,877 | 1 |
| RSAD2, IFI27, OAS1, IFI44L, SIGLEC1, ISG15, HERC6, FAM20A, RETN | 0,881 | 0,871 | 0,891 | 0,929 | 0,875 | 0,983 | 0,939 | 0,866 | 1 |
| RSAD2, IFI27, OAS1, ISG15, HERC6, IL1R2, OLAH, FAM20A, RETN | 0,881 | 0,871 | 0,890 | 0,931 | 0,879 | 0,982 | 0,928 | 0,855 | 1 |
| RSAD2, IFI27, OAS1, IFI44L, HERC6, IL1R2, OLAH, FAM20A, RETN | 0,881 | 0,871 | 0,891 | 0,931 | 0,879 | 0,983 | 0,929 | 0,856 | 1 |
| IFI27, IFI44L, SIGLEC1, ISG15, SLC1A2, IL1R2, OLAH, FAM20A, RETN | 0,881 | 0,871 | 0,890 | 0,936 | 0,889 | 0,983 | 0,941 | 0,874 | 1 |
| IFI27, OAS1, IFI44L, SIGLEC1, ISG15, SLC1A2, IL1R2, FAM20A, MMP8 | 0,881 | 0,871 | 0,891 | 0,939 | 0,896 | 0,981 | 0,950 | 0,880 | 1 |
| RSAD2, IFI27, IFIT1, ISG15, HERC6, IL1R2, OLAH, FAM20A, RETN | 0,881 | 0,871 | 0,890 | 0,929 | 0,876 | 0,981 | 0,926 | 0,851 | 1 |
| RSAD2, IFI27, OAS1, IFI44L, SIGLEC1, ISG15, SLC1A2, OLAH, FAM20A | 0,881 | 0,871 | 0,890 | 0,933 | 0,882 | 0,984 | 0,941 | 0,874 | 1 |
| RSAD2, IFI27, IFI44L, SIGLEC1, SLC1A2, IL1R2, FAM20A, RETN, MMP8 | 0,881 | 0,871 | 0,891 | 0,937 | 0,889 | 0,984 | 0,949 | 0,879 | 1 |
| IFI27, OAS1, IFIT1, IFI44L, HERC6, SLC1A2, OLAH, FAM20A, MMP8 | 0,881 | 0,870 | 0,891 | 0,931 | 0,880 | 0,982 | 0,923 | 0,842 | 1 |
| RSAD2, IFI27, OAS1, IFIT1, HERC6, IL1R2, OLAH, FAM20A, RETN | 0,881 | 0,871 | 0,890 | 0,931 | 0,879 | 0,982 | 0,928 | 0,853 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, SLC1A2, IL1R2, FAM20A, RETN, MMP8 | 0,881 | 0,871 | 0,890 | 0,937 | 0,892 | 0,983 | 0,948 | 0,877 | 1 |
| RSAD2, IFI27, OAS1, SIGLEC1, SLC1A2, OLAH, FAM20A, RETN, MMP8 | 0,881 | 0,870 | 0,891 | 0,933 | 0,881 | 0,985 | 0,950 | 0,889 | 1 |
| RSAD2, IFI27, IFIT1, IFI44L, ISG15, HERC6, OLAH, FAM20A, RETN | 0,881 | 0,871 | 0,890 | 0,931 | 0,879 | 0,983 | 0,928 | 0,857 | 0,999 |
| RSAD2, IFI27, IFIT1, IFI44L, HERC6, IL1R2, OLAH, FAM20A, RETN | 0,881 | 0,871 | 0,890 | 0,931 | 0,879 | 0,983 | 0,928 | 0,856 | 1 |
| RSAD2, IFI27, OAS1, IFIT1, IL1R2, OLAH, FAM20A, RETN, MMP8 | 0,881 | 0,871 | 0,890 | 0,929 | 0,875 | 0,983 | 0,933 | 0,863 | 1 |
| IFI27, OAS1, SIGLEC1, ISG15, HERC6, SLC1A2, OLAH, FAM20A, RETN | 0,881 | 0,870 | 0,891 | 0,935 | 0,887 | 0,983 | 0,935 | 0,864 | 1 |
| IFI27, OAS1, IFI44L, ISG15, HERC6, IL1R2, FAM20A, RETN, MMP8 | 0,881 | 0,870 | 0,891 | 0,932 | 0,880 | 0,984 | 0,930 | 0,849 | 1 |
| IFI27, OAS1, IFIT1, SIGLEC1, ISG15, HERC6, OLAH, RETN, MMP8 | 0,881 | 0,871 | 0,890 | 0,926 | 0,872 | 0,980 | 0,955 | 0,905 | 1 |
| IFI27, OAS1, IFIT1, SIGLEC1, HERC6, SLC1A2, IL1R2, FAM20A, RETN | 0,880 | 0,871 | 0,890 | 0,935 | 0,889 | 0,980 | 0,938 | 0,866 | 1 |
| IFI27, OAS1, IFIT1, SIGLEC1, ISG15, SLC1A2, OLAH, FAM20A, RETN | 0,880 | 0,871 | 0,890 | 0,934 | 0,884 | 0,985 | 0,939 | 0,871 | 1 |
| RSAD2, IFI27, IFI44L, ISG15, HERC6, IL1R2, OLAH, FAM20A, RETN | 0,880 | 0,870 | 0,891 | 0,930 | 0,878 | 0,983 | 0,930 | 0,860 | 1 |
| IFI27, OAS1, IFIT1, SIGLEC1, ISG15, HERC6, SLC1A2, IL1R2, FAM20A | 0,880 | 0,871 | 0,890 | 0,937 | 0,893 | 0,981 | 0,943 | 0,874 | 1 |
| IFI27, IFIT1, IFI44L, SIGLEC1, ISG15, HERC6, SLC1A2, IL1R2, FAM20A | 0,880 | 0,871 | 0,890 | 0,935 | 0,889 | 0,981 | 0,949 | 0,880 | 1 |
| IFI27, OAS1, IFIT1, SIGLEC1, ISG15, SLC1A2, IL1R2, OLAH, FAM20A | 0,880 | 0,871 | 0,890 | 0,936 | 0,888 | 0,984 | 0,937 | 0,869 | 1 |
| RSAD2, IFI27, IFIT1, SIGLEC1, ISG15, SLC1A2, IL1R2, OLAH, FAM20A | 0,880 | 0,871 | 0,889 | 0,936 | 0,888 | 0,984 | 0,937 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, HERC6, SLC1A2, IL1R2, OLAH, FAM20A, MMP8 | 0,880 | 0,870 | 0,891 | 0,935 | 0,888 | 0,983 | 0,928 | 0,853 | 1 |
| RSAD2, IFI27, IFI44L, SIGLEC1, ISG15, HERC6, IL1R2, FAM20A, RETN | 0,880 | 0,870 | 0,890 | 0,933 | 0,882 | 0,985 | 0,943 | 0,875 | 1 |
| RSAD2, IFI27, OAS1, IFIT1, IFI44L, SIGLEC1, ISG15, IL1R2, FAM20A | 0,880 | 0,871 | 0,889 | 0,929 | 0,875 | 0,982 | 0,949 | 0,886 | 1 |
| RSAD2, IFI27, OAS1, IFIT1, SIGLEC1, SLC1A2, IL1R2, OLAH, FAM20A | 0,880 | 0,871 | 0,889 | 0,933 | 0,883 | 0,984 | 0,943 | 0,878 | 1 |
| IFI27, OAS1, IFIT1, HERC6, SLC1A2, IL1R2, OLAH, FAM20A, MMP8 | 0,880 | 0,870 | 0,890 | 0,933 | 0,885 | 0,981 | 0,921 | 0,838 | 1 |
| IFI27, IFIT1, IFI44L, SIGLEC1, SLC1A2, IL1R2, OLAH, FAM20A, RETN | 0,880 | 0,871 | 0,890 | 0,932 | 0,881 | 0,983 | 0,942 | 0,878 | 1 |
| IFI27, OAS1, IFI44L, SIGLEC1, HERC6, IL1R2, OLAH, RETN, MMP8 | 0,880 | 0,871 | 0,889 | 0,927 | 0,875 | 0,980 | 0,951 | 0,900 | 1 |
| RSAD2, IFI27, OAS1, IFI44L, ISG15, HERC6, OLAH, FAM20A, RETN | 0,880 | 0,870 | 0,890 | 0,930 | 0,877 | 0,982 | 0,933 | 0,862 | 1 |
| RSAD2, IFI27, IFI44L, SIGLEC1, ISG15, HERC6, FAM20A, RETN, MMP8 | 0,880 | 0,870 | 0,890 | 0,930 | 0,875 | 0,984 | 0,946 | 0,872 | 1 |
| RSAD2, IFI27, OAS1, IFIT1, IFI44L, HERC6, IL1R2, FAM20A, MMP8 | 0,880 | 0,871 | 0,890 | 0,928 | 0,877 | 0,979 | 0,930 | 0,849 | 1 |
| IFI27, IFIT1, SIGLEC1, ISG15, HERC6, SLC1A2, OLAH, FAM20A, RETN | 0,880 | 0,870 | 0,890 | 0,938 | 0,893 | 0,984 | 0,936 | 0,868 | 1 |
| RSAD2, IFI27, IFIT1, IFI44L, SIGLEC1, SLC1A2, IL1R2, OLAH, FAM20A | 0,880 | 0,871 | 0,890 | 0,931 | 0,879 | 0,983 | 0,942 | 0,878 | 1 |
| IFI27, OAS1, IFIT1, IFI44L, IL1R2, OLAH, FAM20A, RETN, MMP8 | 0,880 | 0,870 | 0,890 | 0,928 | 0,874 | 0,983 | 0,933 | 0,863 | 1 |
| IFI27, OAS1, IFI44L, SIGLEC1, HERC6, SLC1A2, IL1R2, OLAH, FAM20A | 0,880 | 0,870 | 0,890 | 0,933 | 0,883 | 0,982 | 0,937 | 0,868 | 1 |
| RSAD2, IFI27, IFIT1, HERC6, SLC1A2, IL1R2, OLAH, FAM20A, MMP8 | 0,880 | 0,870 | 0,890 | 0,933 | 0,884 | 0,981 | 0,927 | 0,851 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, ISG15, HERC6, SLC1A2, OLAH, FAM20A, MMP8 | 0,880 | 0,870 | 0,890 | 0,931 | 0,881 | 0,982 | 0,922 | 0,841 | 1 |
| RSAD2, IFI27, OAS1, ISG15, IL1R2, OLAH, FAM20A, RETN, MMP8 | 0,880 | 0,870 | 0,890 | 0,928 | 0,874 | 0,983 | 0,930 | 0,860 | 1 |
| RSAD2, IFI27, OAS1, SIGLEC1, ISG15, SLC1A2, IL1R2, OLAH, FAM20A | 0,880 | 0,870 | 0,890 | 0,933 | 0,883 | 0,983 | 0,939 | 0,869 | 1 |
| RSAD2, IFI27, IFI44L, SIGLEC1, ISG15, SLC1A2, IL1R2, FAM20A, MMP8 | 0,880 | 0,870 | 0,890 | 0,939 | 0,897 | 0,982 | 0,951 | 0,882 | 1 |
| RSAD2, IFI27, IFI44L, ISG15, SLC1A2, IL1R2, OLAH, FAM20A, MMP8 | 0,880 | 0,870 | 0,890 | 0,935 | 0,887 | 0,983 | 0,924 | 0,847 | 1 |
| RSAD2, IFI27, OAS1, IFI44L, SIGLEC1, HERC6, SLC1A2, OLAH, FAM20A | 0,880 | 0,870 | 0,890 | 0,932 | 0,881 | 0,982 | 0,937 | 0,869 | 1 |
| RSAD2, IFI27, OAS1, SIGLEC1, SLC1A2, IL1R2, FAM20A, RETN, MMP8 | 0,880 | 0,870 | 0,890 | 0,936 | 0,890 | 0,982 | 0,950 | 0,882 | 1 |
| IFI27, OAS1, IFI44L, ISG15, IL1R2, OLAH, FAM20A, RETN, MMP8 | 0,880 | 0,869 | 0,890 | 0,928 | 0,873 | 0,983 | 0,933 | 0,862 | 1 |
| RSAD2, IFI27, HERC6, SLC1A2, IL1R2, OLAH, FAM20A, RETN, MMP8 | 0,880 | 0,870 | 0,890 | 0,935 | 0,887 | 0,983 | 0,925 | 0,845 | 1 |
| IFI27, OAS1, IFIT1, SIGLEC1, ISG15, HERC6, IL1R2, OLAH, RETN | 0,880 | 0,871 | 0,889 | 0,924 | 0,871 | 0,978 | 0,947 | 0,892 | 1 |
| IFI27, IFI44L, SIGLEC1, HERC6, SLC1A2, IL1R2, FAM20A, RETN, MMP8 | 0,880 | 0,870 | 0,890 | 0,934 | 0,886 | 0,983 | 0,955 | 0,887 | 1 |
| RSAD2, IFI27, OAS1, SIGLEC1, ISG15, SLC1A2, IL1R2, FAM20A, MMP8 | 0,880 | 0,870 | 0,890 | 0,937 | 0,893 | 0,980 | 0,949 | 0,880 | 1 |
| IFI27, OAS1, IFI44L, SIGLEC1, ISG15, SLC1A2, IL1R2, OLAH, FAM20A | 0,880 | 0,870 | 0,890 | 0,934 | 0,886 | 0,983 | 0,941 | 0,874 | 1 |
| RSAD2, IFI27, OAS1, IFIT1, SIGLEC1, HERC6, OLAH, RETN, MMP8 | 0,880 | 0,870 | 0,889 | 0,927 | 0,873 | 0,981 | 0,954 | 0,903 | 1 |
| IFI27, OAS1, IFI44L, HERC6, SLC1A2, IL1R2, OLAH, FAM20A, MMP8 | 0,880 | 0,869 | 0,890 | 0,934 | 0,886 | 0,983 | 0,923 | 0,842 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, SLC1A2, IL1R2, FAM20A, MMP8 | 0,880 | 0,870 | 0,890 | 0,936 | 0,891 | 0,982 | 0,949 | 0,882 | 1 |
| RSAD2, IFI27, OAS1, SIGLEC1, HERC6, IL1R2, OLAH, RETN, MMP8 | 0,880 | 0,870 | 0,889 | 0,926 | 0,872 | 0,981 | 0,954 | 0,903 | 1 |
| IFI27, OAS1, IFIT1, ISG15, HERC6, IL1R2, FAM20A, RETN, MMP8 | 0,880 | 0,870 | 0,890 | 0,931 | 0,879 | 0,984 | 0,928 | 0,847 | 1 |
| IFI27, OAS1, IFI44L, SIGLEC1, ISG15, SLC1A2, OLAH, FAM20A, RETN | 0,880 | 0,870 | 0,889 | 0,932 | 0,881 | 0,983 | 0,938 | 0,870 | 1 |
| RSAD2, IFI27, ISG15, HERC6, SLC1A2, IL1R2, OLAH, FAM20A, MMP8 | 0,880 | 0,870 | 0,889 | 0,933 | 0,886 | 0,980 | 0,920 | 0,838 | 1 |
| RSAD2, IFI27, IFIT1, ISG15, IL1R2, OLAH, FAM20A, RETN, MMP8 | 0,880 | 0,870 | 0,890 | 0,928 | 0,872 | 0,983 | 0,932 | 0,860 | 1 |
| IFI27, IFIT1, IFI44L, HERC6, SLC1A2, IL1R2, OLAH, FAM20A, MMP8 | 0,880 | 0,870 | 0,889 | 0,934 | 0,885 | 0,982 | 0,925 | 0,845 | 1 |
| RSAD2, IFI27, OAS1, IFIT1, IFI44L, SIGLEC1, ISG15, HERC6, RETN | 0,880 | 0,871 | 0,888 | 0,921 | 0,864 | 0,978 | 0,942 | 0,881 | 1 |
| RSAD2, IFI27, OAS1, IFIT1, IFI44L, HERC6, SLC1A2, OLAH, FAM20A | 0,880 | 0,870 | 0,890 | 0,932 | 0,882 | 0,982 | 0,921 | 0,838 | 1 |
| RSAD2, IFI27, OAS1, IFIT1, HERC6, SLC1A2, OLAH, FAM20A, MMP8 | 0,880 | 0,869 | 0,890 | 0,929 | 0,877 | 0,981 | 0,923 | 0,843 | 1 |
| RSAD2, IFI27, IFIT1, SIGLEC1, ISG15, SLC1A2, OLAH, FAM20A, RETN | 0,880 | 0,870 | 0,889 | 0,934 | 0,884 | 0,985 | 0,938 | 0,871 | 1 |
| RSAD2, IFI27, IFI44L, SIGLEC1, ISG15, HERC6, SLC1A2, OLAH, FAM20A | 0,880 | 0,869 | 0,890 | 0,933 | 0,883 | 0,982 | 0,934 | 0,864 | 1 |
| IFI27, OAS1, IFI44L, ISG15, HERC6, SLC1A2, OLAH, FAM20A, MMP8 | 0,880 | 0,869 | 0,890 | 0,931 | 0,881 | 0,982 | 0,922 | 0,841 | 1 |
| RSAD2, IFI27, OAS1, IFI44L, SIGLEC1, HERC6, OLAH, RETN, MMP8 | 0,880 | 0,870 | 0,889 | 0,928 | 0,874 | 0,981 | 0,948 | 0,892 | 1 |
| IFI27, OAS1, IFIT1, IFI44L, ISG15, SLC1A2, OLAH, FAM20A, MMP8 | 0,880 | 0,869 | 0,890 | 0,930 | 0,877 | 0,982 | 0,923 | 0,846 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, HERC6, SLC1A2, IL1R2, FAM20A | 0,880 | 0,870 | 0,889 | 0,937 | 0,893 | 0,980 | 0,941 | 0,872 | 1 |
| IFI27, OAS1, SIGLEC1, ISG15, SLC1A2, IL1R2, OLAH, FAM20A, RETN | 0,879 | 0,870 | 0,889 | 0,934 | 0,884 | 0,984 | 0,938 | 0,870 | 1 |
| IFI27, OAS1, IFIT1, SIGLEC1, SLC1A2, IL1R2, OLAH, FAM20A, RETN | 0,879 | 0,870 | 0,889 | 0,933 | 0,882 | 0,983 | 0,941 | 0,875 | 1 |
| RSAD2, IFI27, IFIT1, IFI44L, IL1R2, OLAH, FAM20A, RETN, MMP8 | 0,879 | 0,869 | 0,890 | 0,929 | 0,874 | 0,984 | 0,930 | 0,859 | 1 |
| IFI27, OAS1, SIGLEC1, ISG15, HERC6, IL1R2, OLAH, RETN, MMP8 | 0,879 | 0,870 | 0,889 | 0,927 | 0,873 | 0,981 | 0,955 | 0,905 | 1 |
| RSAD2, IFI27, IFIT1, IFI44L, HERC6, SLC1A2, OLAH, FAM20A, MMP8 | 0,879 | 0,869 | 0,890 | 0,934 | 0,884 | 0,983 | 0,925 | 0,848 | 1 |
| IFI27, OAS1, IFIT1, IFI44L, ISG15, HERC6, IL1R2, FAM20A, RETN | 0,879 | 0,870 | 0,889 | 0,932 | 0,881 | 0,982 | 0,927 | 0,844 | 1 |
| IFI27, OAS1, IFIT1, HERC6, SLC1A2, OLAH, FAM20A, RETN, MMP8 | 0,879 | 0,869 | 0,890 | 0,933 | 0,883 | 0,983 | 0,922 | 0,840 | 1 |
| IFI27, OAS1, IFI44L, SIGLEC1, HERC6, SLC1A2, OLAH, FAM20A, RETN | 0,879 | 0,869 | 0,889 | 0,934 | 0,886 | 0,983 | 0,936 | 0,866 | 1 |
| RSAD2, IFI27, IFIT1, SIGLEC1, HERC6, SLC1A2, OLAH, FAM20A, RETN | 0,879 | 0,869 | 0,889 | 0,938 | 0,892 | 0,985 | 0,938 | 0,872 | 1 |
| RSAD2, IFI27, OAS1, IFIT1, SIGLEC1, ISG15, IL1R2, FAM20A, RETN | 0,879 | 0,870 | 0,888 | 0,928 | 0,873 | 0,982 | 0,950 | 0,889 | 1 |
| RSAD2, IFI27, OAS1, IFI44L, HERC6, SLC1A2, OLAH, FAM20A, MMP8 | 0,879 | 0,869 | 0,890 | 0,931 | 0,881 | 0,982 | 0,921 | 0,841 | 1 |
| IFI27, SIGLEC1, ISG15, HERC6, SLC1A2, IL1R2, FAM20A, RETN, MMP8 | 0,879 | 0,869 | 0,889 | 0,937 | 0,891 | 0,982 | 0,951 | 0,880 | 1 |
| RSAD2, IFI27, OAS1, HERC6, SLC1A2, OLAH, FAM20A, RETN, MMP8 | 0,879 | 0,869 | 0,889 | 0,934 | 0,884 | 0,983 | 0,922 | 0,838 | 1 |
| IFI27, OAS1, IFI44L, SIGLEC1, ISG15, HERC6, SLC1A2, IL1R2, FAM20A | 0,879 | 0,870 | 0,889 | 0,935 | 0,889 | 0,980 | 0,945 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, HERC6, SLC1A2, OLAH, FAM20A, MMP8 | 0,879 | 0,869 | 0,889 | 0,931 | 0,882 | 0,980 | 0,918 | 0,835 | 1 |
| RSAD2, IFI27, IFIT1, SIGLEC1, HERC6, SLC1A2, FAM20A, RETN, MMP8 | 0,879 | 0,870 | 0,889 | 0,930 | 0,878 | 0,983 | 0,942 | 0,869 | 1 |
| RSAD2, IFI27, OAS1, IFIT1, IFI44L, SIGLEC1, IL1R2, FAM20A, RETN | 0,879 | 0,869 | 0,889 | 0,928 | 0,873 | 0,982 | 0,950 | 0,888 | 1 |
| IFI27, OAS1, ISG15, HERC6, SLC1A2, IL1R2, OLAH, FAM20A, MMP8 | 0,879 | 0,869 | 0,889 | 0,935 | 0,889 | 0,981 | 0,922 | 0,841 | 1 |
| RSAD2, IFI27, OAS1, IFIT1, HERC6, IL1R2, FAM20A, RETN, MMP8 | 0,879 | 0,869 | 0,889 | 0,930 | 0,877 | 0,983 | 0,928 | 0,847 | 1 |
| RSAD2, IFI27, IFIT1, IFI44L, SIGLEC1, HERC6, OLAH, RETN, MMP8 | 0,879 | 0,870 | 0,888 | 0,926 | 0,871 | 0,980 | 0,951 | 0,900 | 1 |
| IFI27, IFI44L, SIGLEC1, ISG15, HERC6, SLC1A2, OLAH, FAM20A, RETN | 0,879 | 0,869 | 0,889 | 0,933 | 0,882 | 0,983 | 0,938 | 0,869 | 1 |
| RSAD2, IFI27, IFI44L, SIGLEC1, HERC6, IL1R2, OLAH, RETN, MMP8 | 0,879 | 0,870 | 0,888 | 0,927 | 0,874 | 0,980 | 0,952 | 0,903 | 1 |
| RSAD2, IFI27, OAS1, IFIT1, IFI44L, ISG15, OLAH, FAM20A, MMP8 | 0,879 | 0,869 | 0,890 | 0,924 | 0,866 | 0,983 | 0,937 | 0,873 | 1 |
| RSAD2, IFI27, IFIT1, ISG15, SLC1A2, IL1R2, OLAH, FAM20A, MMP8 | 0,879 | 0,869 | 0,889 | 0,933 | 0,885 | 0,981 | 0,925 | 0,847 | 1 |
| IFI27, OAS1, IFI44L, SIGLEC1, SLC1A2, OLAH, FAM20A, RETN, MMP8 | 0,879 | 0,868 | 0,890 | 0,933 | 0,880 | 0,985 | 0,952 | 0,893 | 1 |
| RSAD2, IFI27, OAS1, ISG15, HERC6, IL1R2, FAM20A, RETN, MMP8 | 0,879 | 0,869 | 0,889 | 0,931 | 0,879 | 0,983 | 0,927 | 0,844 | 1 |
| RSAD2, IFI27, OAS1, IFI44L, ISG15, HERC6, IL1R2, FAM20A, MMP8 | 0,879 | 0,869 | 0,889 | 0,929 | 0,878 | 0,980 | 0,926 | 0,840 | 1 |
| RSAD2, IFI27, IFI44L, ISG15, HERC6, SLC1A2, OLAH, FAM20A, MMP8 | 0,879 | 0,868 | 0,890 | 0,934 | 0,884 | 0,983 | 0,923 | 0,845 | 1 |
| RSAD2, IFI27, IFI44L, SIGLEC1, SLC1A2, OLAH, FAM20A, RETN, MMP8 | 0,879 | 0,869 | 0,889 | 0,935 | 0,884 | 0,986 | 0,948 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SLC1A2, IL1R2, OLAH, FAM20A, MMP8 | 0,879 | 0,869 | 0,889 | 0,933 | 0,882 | 0,983 | 0,927 | 0,851 | 1 |
| RSAD2, IFI27, IFIT1, IFI44L, ISG15, SLC1A2, OLAH, FAM20A, MMP8 | 0,879 | 0,869 | 0,889 | 0,930 | 0,878 | 0,983 | 0,923 | 0,846 | 1 |
| RSAD2, IFI27, OAS1, IFIT1, IFI44L, SIGLEC1, HERC6, OLAH, RETN | 0,879 | 0,870 | 0,888 | 0,924 | 0,870 | 0,979 | 0,942 | 0,884 | 1 |
| RSAD2, IFI27, OAS1, IFI44L, IL1R2, OLAH, FAM20A, RETN, MMP8 | 0,879 | 0,868 | 0,890 | 0,930 | 0,877 | 0,984 | 0,934 | 0,865 | 1 |
| RSAD2, IFI27, IFI44L, SIGLEC1, HERC6, SLC1A2, IL1R2, OLAH, FAM20A | 0,879 | 0,868 | 0,889 | 0,933 | 0,884 | 0,983 | 0,945 | 0,878 | 1 |
| RSAD2, IFI27, IFIT1, SIGLEC1, HERC6, IL1R2, OLAH, RETN, MMP8 | 0,879 | 0,870 | 0,888 | 0,926 | 0,872 | 0,980 | 0,951 | 0,900 | 1 |
| OAS1, IFIT1, IFI44L, SIGLEC1, HERC6, IL1R2, OLAH, FAM20A, MMP8 | 0,879 | 0,870 | 0,888 | 0,924 | 0,870 | 0,977 | 0,963 | 0,909 | 1 |
| RSAD2, IFI27, OAS1, IFIT1, SLC1A2, IL1R2, OLAH, FAM20A, MMP8 | 0,879 | 0,869 | 0,888 | 0,931 | 0,880 | 0,981 | 0,924 | 0,845 | 1 |
| IFI27, OAS1, IFI44L, SIGLEC1, ISG15, HERC6, OLAH, RETN, MMP8 | 0,879 | 0,870 | 0,888 | 0,926 | 0,872 | 0,980 | 0,951 | 0,900 | 1 |
| RSAD2, IFI27, IFIT1, IFI44L, SIGLEC1, HERC6, SLC1A2, IL1R2, FAM20A | 0,879 | 0,869 | 0,888 | 0,936 | 0,890 | 0,981 | 0,946 | 0,876 | 1 |
| RSAD2, IFI27, IFIT1, HERC6, SLC1A2, OLAH, FAM20A, RETN, MMP8 | 0,879 | 0,869 | 0,889 | 0,935 | 0,886 | 0,984 | 0,923 | 0,843 | 1 |
| RSAD2, IFI27, OAS1, IFI44L, SIGLEC1, ISG15, IL1R2, FAM20A, RETN | 0,879 | 0,869 | 0,889 | 0,927 | 0,873 | 0,981 | 0,949 | 0,884 | 1 |
| IFI27, OAS1, IFI44L, ISG15, HERC6, SLC1A2, IL1R2, OLAH, FAM20A | 0,879 | 0,868 | 0,889 | 0,934 | 0,885 | 0,982 | 0,918 | 0,836 | 1 |
| IFI27, IFI44L, SIGLEC1, HERC6, SLC1A2, OLAH, FAM20A, RETN, MMP8 | 0,879 | 0,869 | 0,889 | 0,930 | 0,877 | 0,984 | 0,947 | 0,878 | 1 |
| RSAD2, IFI27, SIGLEC1, ISG15, SLC1A2, IL1R2, OLAH, FAM20A, RETN | 0,879 | 0,869 | 0,889 | 0,933 | 0,883 | 0,983 | 0,938 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, SLC1A2, OLAH, FAM20A | 0,879 | 0,868 | 0,889 | 0,934 | 0,886 | 0,983 | 0,918 | 0,836 | 1 |
| RSAD2, IFI27, OAS1, SIGLEC1, ISG15, HERC6, OLAH, RETN, MMP8 | 0,879 | 0,869 | 0,888 | 0,925 | 0,870 | 0,980 | 0,955 | 0,905 | 1 |
| IFI27, IFIT1, SIGLEC1, HERC6, SLC1A2, IL1R2, OLAH, FAM20A, RETN | 0,879 | 0,869 | 0,889 | 0,936 | 0,888 | 0,984 | 0,941 | 0,875 | 1 |
| RSAD2, IFI27, IFIT1, IFI44L, HERC6, IL1R2, FAM20A, RETN, MMP8 | 0,879 | 0,869 | 0,889 | 0,931 | 0,879 | 0,983 | 0,935 | 0,857 | 1 |
| RSAD2, IFI27, OAS1, IFIT1, ISG15, SLC1A2, OLAH, FAM20A, MMP8 | 0,879 | 0,869 | 0,888 | 0,927 | 0,873 | 0,981 | 0,925 | 0,849 | 1 |
| IFI27, IFIT1, IFI44L, SIGLEC1, HERC6, IL1R2, OLAH, RETN, MMP8 | 0,879 | 0,870 | 0,888 | 0,925 | 0,871 | 0,979 | 0,953 | 0,904 | 1 |
| IFI27, OAS1, SIGLEC1, HERC6, SLC1A2, IL1R2, OLAH, FAM20A, RETN | 0,879 | 0,868 | 0,889 | 0,932 | 0,882 | 0,982 | 0,936 | 0,866 | 1 |
| IFI27, OAS1, IFI44L, ISG15, SLC1A2, IL1R2, OLAH, FAM20A, MMP8 | 0,879 | 0,868 | 0,889 | 0,935 | 0,887 | 0,983 | 0,924 | 0,847 | 1 |
| IFI27, IFIT1, IFI44L, SIGLEC1, HERC6, SLC1A2, IL1R2, FAM20A, RETN | 0,879 | 0,869 | 0,888 | 0,937 | 0,891 | 0,982 | 0,946 | 0,876 | 1 |
| IFI27, OAS1, IFIT1, IFI44L, SIGLEC1, ISG15, FAM20A, RETN, MMP8 | 0,879 | 0,868 | 0,889 | 0,923 | 0,863 | 0,982 | 0,946 | 0,875 | 1 |
| IFI27, SIGLEC1, HERC6, SLC1A2, IL1R2, OLAH, FAM20A, RETN, MMP8 | 0,879 | 0,868 | 0,889 | 0,936 | 0,886 | 0,985 | 0,948 | 0,880 | 1 |
| RSAD2, IFI27, OAS1, IFI44L, HERC6, IL1R2, FAM20A, RETN, MMP8 | 0,879 | 0,868 | 0,889 | 0,930 | 0,878 | 0,983 | 0,932 | 0,850 | 1 |
| IFI27, OAS1, ISG15, HERC6, SLC1A2, OLAH, FAM20A, RETN, MMP8 | 0,879 | 0,868 | 0,889 | 0,933 | 0,884 | 0,982 | 0,920 | 0,836 | 1 |
| IFI27, OAS1, HERC6, SLC1A2, IL1R2, OLAH, FAM20A, RETN, MMP8 | 0,879 | 0,869 | 0,889 | 0,934 | 0,885 | 0,983 | 0,921 | 0,838 | 1 |
| RSAD2, IFI27, OAS1, ISG15, HERC6, SLC1A2, IL1R2, OLAH, FAM20A | 0,879 | 0,869 | 0,888 | 0,935 | 0,888 | 0,982 | 0,915 | 0,828 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, SLC1A2, OLAH, FAM20A, RETN | 0,879 | 0,869 | 0,888 | 0,931 | 0,879 | 0,984 | 0,940 | 0,873 | 1 |
| RSAD2, IFI27, OAS1, SIGLEC1, HERC6, SLC1A2, OLAH, FAM20A, RETN | 0,879 | 0,869 | 0,888 | 0,933 | 0,883 | 0,983 | 0,935 | 0,864 | 1 |
| RSAD2, OAS1, IFIT1, SIGLEC1, HERC6, IL1R2, OLAH, FAM20A, MMP8 | 0,879 | 0,869 | 0,888 | 0,924 | 0,870 | 0,978 | 0,962 | 0,906 | 1 |
| RSAD2, IFI27, IFI44L, SIGLEC1, ISG15, SLC1A2, IL1R2, OLAH, FAM20A | 0,879 | 0,868 | 0,889 | 0,936 | 0,888 | 0,983 | 0,940 | 0,872 | 1 |
| RSAD2, IFI27, OAS1, IFIT1, IFI44L, SLC1A2, OLAH, FAM20A, MMP8 | 0,879 | 0,869 | 0,888 | 0,931 | 0,878 | 0,984 | 0,926 | 0,850 | 1 |
| IFI27, IFI44L, SIGLEC1, ISG15, HERC6, IL1R2, OLAH, RETN, MMP8 | 0,879 | 0,870 | 0,887 | 0,927 | 0,874 | 0,980 | 0,952 | 0,902 | 1 |
| RSAD2, IFI27, IFIT1, SIGLEC1, ISG15, HERC6, SLC1A2, IL1R2, FAM20A | 0,878 | 0,869 | 0,888 | 0,936 | 0,891 | 0,981 | 0,948 | 0,879 | 1 |
| IFI27, IFIT1, IFI44L, SIGLEC1, HERC6, SLC1A2, FAM20A, RETN, MMP8 | 0,878 | 0,869 | 0,888 | 0,929 | 0,875 | 0,982 | 0,950 | 0,878 | 1 |
| RSAD2, IFI27, IFIT1, IFI44L, SIGLEC1, ISG15, IL1R2, FAM20A, RETN | 0,878 | 0,869 | 0,888 | 0,928 | 0,873 | 0,983 | 0,948 | 0,883 | 1 |
| IFI27, OAS1, IFIT1, ISG15, HERC6, SLC1A2, OLAH, FAM20A, MMP8 | 0,878 | 0,868 | 0,889 | 0,931 | 0,881 | 0,981 | 0,920 | 0,839 | 1 |
| IFI27, OAS1, IFIT1, IFI44L, SIGLEC1, HERC6, IL1R2, OLAH, RETN | 0,878 | 0,870 | 0,887 | 0,924 | 0,872 | 0,976 | 0,941 | 0,884 | 0,999 |
| IFI27, OAS1, IFIT1, IFI44L, SIGLEC1, ISG15, HERC6, RETN, MMP8 | 0,878 | 0,869 | 0,887 | 0,920 | 0,863 | 0,978 | 0,943 | 0,886 | 1 |
| IFI27, OAS1, IFIT1, IFI44L, SIGLEC1, ISG15, HERC6, OLAH, RETN | 0,878 | 0,870 | 0,887 | 0,924 | 0,871 | 0,976 | 0,941 | 0,884 | 0,999 |
| IFI27, OAS1, IFIT1, IFI44L, SIGLEC1, SLC1A2, IL1R2, FAM20A, RETN | 0,878 | 0,869 | 0,888 | 0,932 | 0,884 | 0,981 | 0,942 | 0,873 | 1 |
| RSAD2, IFI27, SIGLEC1, ISG15, HERC6, SLC1A2, OLAH, FAM20A, RETN | 0,878 | 0,868 | 0,888 | 0,931 | 0,881 | 0,982 | 0,936 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, ISG15, IL1R2, OLAH, FAM20A, RETN, MMP8 | 0,878 | 0,867 | 0,889 | 0,930 | 0,876 | 0,984 | 0,933 | 0,863 | 1 |
| IFI27, OAS1, IFIT1, IFI44L, HERC6, SLC1A2, IL1R2, OLAH, FAM20A | 0,878 | 0,868 | 0,888 | 0,934 | 0,885 | 0,982 | 0,920 | 0,837 | 1 |
| RSAD2, IFI27, IFI44L, HERC6, SLC1A2, OLAH, FAM20A, RETN, MMP8 | 0,878 | 0,868 | 0,888 | 0,935 | 0,886 | 0,984 | 0,924 | 0,845 | 1 |
| RSAD2, IFI27, IFIT1, SIGLEC1, SLC1A2, IL1R2, OLAH, FAM20A, RETN | 0,878 | 0,869 | 0,888 | 0,933 | 0,882 | 0,983 | 0,943 | 0,879 | 1 |
| IFI27, OAS1, IFIT1, IFI44L, ISG15, HERC6, IL1R2, FAM20A, MMP8 | 0,878 | 0,868 | 0,888 | 0,929 | 0,879 | 0,980 | 0,929 | 0,846 | 1 |
| RSAD2, IFI27, OAS1, IFIT1, IFI44L, ISG15, HERC6, ILIR2, FAM20A | 0,878 | 0,869 | 0,888 | 0,928 | 0,877 | 0,979 | 0,925 | 0,841 | 1 |
| RSAD2, IFI27, OAS1, IFI44L, SIGLEC1, HERC6, IL1R2, OLAH, RETN | 0,878 | 0,869 | 0,887 | 0,924 | 0,871 | 0,977 | 0,943 | 0,885 | 1 |
| IFI27, IFIT1, IFI44L, ISG15, HERC6, IL1R2, FAM20A, RETN, MMP8 | 0,878 | 0,868 | 0,888 | 0,931 | 0,878 | 0,983 | 0,935 | 0,856 | 1 |
| IFI27, IFIT1, IFI44L, SIGLEC1, ISG15, HERC6, OLAH, RETN, MMP8 | 0,878 | 0,869 | 0,887 | 0,925 | 0,870 | 0,980 | 0,949 | 0,896 | 1 |
| IFI27, OAS1, IFIT1, SIGLEC1, ISG15, HERC6, SLC1A2, FAM20A, RETN | 0,878 | 0,868 | 0,888 | 0,931 | 0,881 | 0,980 | 0,936 | 0,865 | 1 |
| IFI27, IFIT1, IFI44L, SIGLEC1, ISG15, SLC1A2, IL1R2, FAM20A, RETN | 0,878 | 0,869 | 0,887 | 0,934 | 0,887 | 0,980 | 0,943 | 0,874 | 1 |
| RSAD2, IFI27, OAS1, IFIT1, IFI44L, SIGLEC1, ISG15, FAM20A, RETN | 0,878 | 0,869 | 0,888 | 0,920 | 0,860 | 0,980 | 0,942 | 0,872 | 1 |
| IFI27, OAS1, IFIT1, IFI44L, SIGLEC1, ISG15, SLC1A2, IL1R2, FAM20A | 0,878 | 0,869 | 0,887 | 0,933 | 0,886 | 0,980 | 0,943 | 0,875 | 1 |
| RSAD2, IFI27, OAS1, IFIT1, IFI44L, ISG15, HERC6, FAM20A, RETN | 0,878 | 0,868 | 0,888 | 0,924 | 0,867 | 0,980 | 0,930 | 0,851 | 1 |
| RSAD2, IFI27, IFIT1, ISG15, HERC6, SLC1A2, OLAH, FAM20A, MMP8 | 0,878 | 0,868 | 0,889 | 0,931 | 0,881 | 0,981 | 0,922 | 0,844 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, ISG15, HERC6, SLC1A2, OLAH, FAM20A, RETN, MMP8 | 0,878 | 0,868 | 0,888 | 0,932 | 0,882 | 0,982 | 0,920 | 0,836 | 1 |
| RSAD2, IFI27, OAS1, IFI44L, ISG15, HERC6, SLC1A2, OLAH, FAM20A | 0,878 | 0,868 | 0,889 | 0,932 | 0,883 | 0,982 | 0,920 | 0,837 | 1 |
| RSAD2, IFI27, OAS1, IFIT1, IFI44L, HERC6, IL1R2, FAM20A, RETN | 0,878 | 0,868 | 0,888 | 0,930 | 0,879 | 0,981 | 0,929 | 0,848 | 1 |
| IFI27, OAS1, IFI44L, ISG15, HERC6, SLC1A2, OLAH, FAM20A, RETN | 0,878 | 0,868 | 0,888 | 0,935 | 0,887 | 0,983 | 0,920 | 0,835 | 1 |
| IFI27, IFIT1, ISG15, HERC6, SLC1A2, IL1R2, OLAH, FAM20A, MMP8 | 0,878 | 0,868 | 0,888 | 0,934 | 0,886 | 0,981 | 0,924 | 0,845 | 1 |
| RSAD2, IFI27, IFI44L, SIGLEC1, ISG15, SLC1A2, OLAH, FAM20A, RETN | 0,878 | 0,868 | 0,888 | 0,930 | 0,878 | 0,982 | 0,939 | 0,871 | 1 |
| IFI27, OAS1, IFIT1, IFI44L, HERC6, SLC1A2, OLAH, FAM20A, RETN | 0,878 | 0,868 | 0,888 | 0,934 | 0,886 | 0,982 | 0,923 | 0,842 | 1 |
| RSAD2, IFI27, OAS1, IFIT1, SIGLEC1, HERC6, IL1R2, OLAH, RETN | 0,878 | 0,869 | 0,887 | 0,926 | 0,873 | 0,980 | 0,947 | 0,891 | 1 |
| RSAD2, IFI27, IFIT1, ISG15, HERC6, IL1R2, FAM20A, RETN, MMP8 | 0,878 | 0,868 | 0,888 | 0,931 | 0,879 | 0,984 | 0,935 | 0,857 | 1 |
| OAS1, IFIT1, SIGLEC1, ISG15, HERC6, IL1R2, OLAH, FAM20A, MMP8 | 0,878 | 0,869 | 0,887 | 0,922 | 0,867 | 0,977 | 0,962 | 0,906 | 1 |
| RSAD2, IFI27, IFIT1, SIGLEC1, ISG15, HERC6, OLAH, RETN, MMP8 | 0,878 | 0,869 | 0,887 | 0,924 | 0,869 | 0,979 | 0,951 | 0,900 | 1 |
| RSAD2, IFI27, OAS1, ISG15, SLC1A2, IL1R2, OLAH, FAM20A, MMP8 | 0,878 | 0,868 | 0,888 | 0,936 | 0,890 | 0,982 | 0,917 | 0,834 | 1 |
| IFI27, OAS1, IFIT1, ISG15, SLC1A2, IL1R2, OLAH, FAM20A, MMP8 | 0,878 | 0,868 | 0,888 | 0,934 | 0,886 | 0,981 | 0,925 | 0,847 | 1 |
| RSAD2, IFI27, OAS1, SIGLEC1, ISG15, HERC6, SLC1A2, IL1R2, FAM20A | 0,878 | 0,868 | 0,888 | 0,935 | 0,890 | 0,980 | 0,943 | 0,874 | 1 |
| RSAD2, IFI27, SIGLEC1, HERC6, SLC1A2, IL1R2, OLAH, FAM20A, RETN | 0,878 | 0,868 | 0,888 | 0,933 | 0,883 | 0,983 | 0,940 | 0,872 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, ISG15, SLC1A2, OLAH, FAM20A, MMP8 | 0,878 | 0,867 | 0,888 | 0,931 | 0,880 | 0,983 | 0,924 | 0,847 | 1 |
| IFI27, OAS1, IFIT1, IFI44L, SLC1A2, IL1R2, OLAH, FAM20A, MMP8 | 0,878 | 0,868 | 0,888 | 0,933 | 0,883 | 0,983 | 0,926 | 0,849 | 1 |
| RSAD2, IFI27, OAS1, SIGLEC1, ISG15, HERC6, IL1R2, OLAH, RETN | 0,878 | 0,869 | 0,887 | 0,923 | 0,868 | 0,978 | 0,946 | 0,889 | 1 |
| IFIT1, IFI44L, SIGLEC1, ISG15, HERC6, IL1R2, OLAH, FAM20A, MMP8 | 0,878 | 0,868 | 0,887 | 0,923 | 0,869 | 0,977 | 0,963 | 0,909 | 1 |
| RSAD2, IFI27, OAS1, IFI44L, HERC6, SLC1A2, IL1R2, OLAH, FAM20A | 0,878 | 0,867 | 0,888 | 0,933 | 0,883 | 0,982 | 0,921 | 0,839 | 1 |
| IFI27, OAS1, IFI44L, HERC6, SLC1A2, OLAH, FAM20A, RETN, MMP8 | 0,878 | 0,867 | 0,889 | 0,936 | 0,887 | 0,985 | 0,923 | 0,842 | 1 |
| RSAD2, IFI27, IFIT1, IFI44L, SIGLEC1, SLC1A2, OLAH, FAM20A, RETN | 0,878 | 0,868 | 0,888 | 0,931 | 0,878 | 0,984 | 0,941 | 0,876 | 1 |
| RSAD2, OAS1, IFIT1, IFI44L, SIGLEC1, HERC6, IL1R2, FAM20A, MMP8 | 0,878 | 0,869 | 0,887 | 0,923 | 0,869 | 0,978 | 0,962 | 0,906 | 1 |
| IFI27, IFIT1, IFI44L, SIGLEC1, ISG15, HERC6, SLC1A2, FAM20A, RETN | 0,878 | 0,868 | 0,887 | 0,930 | 0,879 | 0,981 | 0,941 | 0,869 | 1 |
| IFI27, IFI44L, ISG15, HERC6, SLC1A2, OLAH, FAM20A, RETN, MMP8 | 0,878 | 0,868 | 0,888 | 0,931 | 0,879 | 0,983 | 0,924 | 0,844 | 1 |
| RSAD2, IFI27, OAS1, IFIT1, IFI44L, SIGLEC1, ISG15, HERC6, FAM20A | 0,878 | 0,869 | 0,887 | 0,924 | 0,866 | 0,981 | 0,939 | 0,868 | 1 |
| IFI27, OAS1, IFI44L, SIGLEC1, HERC6, SLC1A2, IL1R2, FAM20A, RETN | 0,878 | 0,868 | 0,888 | 0,936 | 0,891 | 0,981 | 0,942 | 0,872 | 1 |
| IFI27, OAS1, IFIT1, IFI44L, ISG15, OLAH, FAM20A, RETN, MMP8 | 0,878 | 0,867 | 0,888 | 0,922 | 0,862 | 0,981 | 0,942 | 0,877 | 1 |
| IFIT1, IFI44L, SIGLEC1, ISG15, HERC6, IL1R2, FAM20A, RETN, MMP8 | 0,878 | 0,869 | 0,887 | 0,923 | 0,867 | 0,979 | 0,961 | 0,903 | 1 |
| IFI27, ISG15, HERC6, SLC1A2, IL1R2, OLAH, FAM20A, RETN, MMP8 | 0,878 | 0,868 | 0,887 | 0,935 | 0,888 | 0,982 | 0,923 | 0,841 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, HERC6, IL1R2, RETN, MMP8 | 0,878 | 0,869 | 0,887 | 0,928 | 0,877 | 0,978 | 0,954 | 0,907 | 1 |
| RSAD2, IFI27, OAS1, IFIT1, HERC6, IL1R2, OLAH, RETN, MMP8 | 0,878 | 0,868 | 0,887 | 0,924 | 0,868 | 0,980 | 0,945 | 0,885 | 1 |
| RSAD2, IFI27, OAS1, IFIT1, SIGLEC1, HERC6, IL1R2, RETN, MMP8 | 0,878 | 0,869 | 0,887 | 0,925 | 0,872 | 0,979 | 0,957 | 0,906 | 1 |
| RSAD2, IFI27, OAS1, IFIT1, IFI44L, ISG15, IL1R2, OLAH, FAM20A | 0,878 | 0,868 | 0,887 | 0,924 | 0,867 | 0,981 | 0,929 | 0,859 | 1,000 |
| IFI27, OAS1, IFIT1, ISG15, HERC6, SLC1A2, OLAH, FAM20A, RETN | 0,878 | 0,868 | 0,888 | 0,933 | 0,884 | 0,983 | 0,915 | 0,828 | 1 |
| RSAD2, IFI27, OAS1, IFIT1, SIGLEC1, SLC1A2, OLAH, FAM20A, RETN | 0,878 | 0,868 | 0,887 | 0,931 | 0,878 | 0,984 | 0,942 | 0,876 | 1 |
| IFI27, OAS1, IFIT1, IFI44L, SIGLEC1, ISG15, HERC6, FAM20A, MMP8 | 0,878 | 0,868 | 0,887 | 0,923 | 0,866 | 0,980 | 0,937 | 0,865 | 1 |
| OAS1, IFIT1, IFI44L, SIGLEC1, HERC6, IL1R2, FAM20A, RETN, MMP8 | 0,878 | 0,868 | 0,887 | 0,924 | 0,869 | 0,979 | 0,963 | 0,908 | 1 |
| RSAD2, IFI27, OAS1, IFIT1, ISG15, HERC6, OLAH, RETN, MMP8 | 0,878 | 0,868 | 0,887 | 0,924 | 0,869 | 0,980 | 0,945 | 0,886 | 1 |
| RSAD2, IFI27, IFI44L, ISG15, HERC6, IL1R2, FAM20A, RETN, MMP8 | 0,878 | 0,867 | 0,888 | 0,931 | 0,880 | 0,983 | 0,934 | 0,854 | 1 |
| RSAD2, IFI27, OAS1, IFI44L, SIGLEC1, HERC6, IL1R2, OLAH, MMP8 | 0,878 | 0,869 | 0,886 | 0,927 | 0,879 | 0,975 | 0,949 | 0,900 | 0,997 |
| IFI27, OAS1, IFI44L, SIGLEC1, ISG15, HERC6, IL1R2, OLAH, RETN | 0,878 | 0,869 | 0,886 | 0,924 | 0,872 | 0,976 | 0,942 | 0,885 | 1,000 |
| RSAD2, IFI27, SIGLEC1, ISG15, HERC6, IL1R2, OLAH, RETN, MMP8 | 0,877 | 0,868 | 0,887 | 0,925 | 0,871 | 0,979 | 0,953 | 0,903 | 1 |
| IFI27, OAS1, IFIT1, SIGLEC1, ISG15, HERC6, IL1R2, RETN, MMP8 | 0,877 | 0,869 | 0,886 | 0,925 | 0,872 | 0,978 | 0,957 | 0,907 | 1 |
| RSAD2, IFI27, OAS1, IFI44L, SLC1A2, IL1R2, OLAH, FAM20A, MMP8 | 0,877 | 0,867 | 0,888 | 0,934 | 0,884 | 0,983 | 0,926 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, HERC6, IL1R2, FAM20A, MMP8 | 0,877 | 0,868 | 0,887 | 0,928 | 0,877 | 0,979 | 0,929 | 0,848 | 1 |
| RSAD2, IFI27, OAS1, IFIT1, SIGLEC1, HERC6, SLC1A2, FAM20A, RETN | 0,877 | 0,868 | 0,887 | 0,933 | 0,884 | 0,981 | 0,933 | 0,859 | 1 |
| IFI27, IFIT1, HERC6, SLC1A2, IL1R2, OLAH, FAM20A, RETN, MMP8 | 0,877 | 0,867 | 0,887 | 0,932 | 0,882 | 0,982 | 0,929 | 0,853 | 1 |
| IFI27, OAS1, IFIT1, IFI44L, ISG15, HERC6, FAM20A, RETN, MMP8 | 0,877 | 0,867 | 0,888 | 0,926 | 0,869 | 0,983 | 0,934 | 0,855 | 1 |
| IFI27, OAS1, IFI44L, SIGLEC1, SLC1A2, IL1R2, OLAH, FAM20A, RETN | 0,877 | 0,867 | 0,887 | 0,930 | 0,877 | 0,982 | 0,943 | 0,879 | 1 |
| RSAD2, IFI27, OAS1, IFIT1, SIGLEC1, ISG15, HERC6, OLAH, RETN | 0,877 | 0,869 | 0,886 | 0,926 | 0,873 | 0,979 | 0,947 | 0,891 | 1 |
| IFI27, OAS1, IFIT1, ISG15, HERC6, IL1R2, OLAH, RETN, MMP8 | 0,877 | 0,868 | 0,887 | 0,924 | 0,868 | 0,979 | 0,945 | 0,885 | 1 |
| IFI27, OAS1, IFI44L, ISG15, HERC6, IL1R2, OLAH, RETN, MMP8 | 0,877 | 0,868 | 0,887 | 0,924 | 0,868 | 0,980 | 0,945 | 0,887 | 1 |
| RSAD2, IFI27, IFIT1, IFI44L, HERC6, SLC1A2, IL1R2, OLAH, FAM20A | 0,877 | 0,867 | 0,887 | 0,933 | 0,883 | 0,982 | 0,923 | 0,843 | 1 |
| RSAD2, IFI27, OAS1, IFI44L, SIGLEC1, ISG15, HERC6, OLAH, RETN | 0,877 | 0,869 | 0,886 | 0,924 | 0,870 | 0,979 | 0,942 | 0,883 | 1 |
| IFI27, SIGLEC1, ISG15, HERC6, SLC1A2, IL1R2, OLAH, FAM20A, RETN | 0,877 | 0,867 | 0,888 | 0,933 | 0,884 | 0,982 | 0,938 | 0,870 | 1 |
| RSAD2, IFI27, OAS1, IFIT1, HERC6, SLC1A2, IL1R2, OLAH, FAM20A | 0,877 | 0,867 | 0,887 | 0,932 | 0,883 | 0,981 | 0,921 | 0,838 | 1 |
| RSAD2, IFIT1, SIGLEC1, ISG15, HERC6, IL1R2, OLAH, FAM20A, MMP8 | 0,877 | 0,868 | 0,887 | 0,922 | 0,867 | 0,976 | 0,962 | 0,906 | 1 |
| IFI27, IFIT1, IFI44L, HERC6, SLC1A2, OLAH, FAM20A, RETN, MMP8 | 0,877 | 0,867 | 0,887 | 0,933 | 0,882 | 0,985 | 0,928 | 0,848 | 1 |
| IFI27, OAS1, IFIT1, IFI44L, ISG15, IL1R2, OLAH, FAM20A, RETN | 0,877 | 0,868 | 0,887 | 0,924 | 0,867 | 0,981 | 0,932 | 0,861 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, ISG15, HERC6, IL1R2, OLAH, MMP8 | 0,877 | 0,869 | 0,886 | 0,926 | 0,877 | 0,975 | 0,941 | 0,888 | 0,995 |
| RSAD2, IFI27, OAS1, IFIT1, ISG15, OLAH, FAM20A, RETN, MMP8 | 0,877 | 0,867 | 0,888 | 0,923 | 0,863 | 0,982 | 0,943 | 0,880 | 1 |
| RSAD2, IFI27, OAS1, IFIT1, ISG15, HERC6, SLC1A2, OLAH, FAM20A | 0,877 | 0,867 | 0,887 | 0,930 | 0,880 | 0,981 | 0,920 | 0,837 | 1 |
| RSAD2, IFI27, OAS1, IFIT1, IFI44L, OLAH, FAM20A, RETN, MMP8 | 0,877 | 0,867 | 0,888 | 0,923 | 0,863 | 0,983 | 0,941 | 0,877 | 1 |
| IFI27, IFIT1, IFI44L, SIGLEC1, ISG15, HERC6, IL1R2, OLAH, RETN | 0,877 | 0,869 | 0,886 | 0,926 | 0,874 | 0,979 | 0,946 | 0,893 | 0,998 |
| RSAD2, IFI27, OAS1, IFIT1, IFI44L, HERC6, OLAH, RETN, MMP8 | 0,877 | 0,868 | 0,887 | 0,924 | 0,867 | 0,981 | 0,945 | 0,887 | 1 |
| RSAD2, IFI27, OAS1, IFIT1, ISG15, IL1R2, OLAH, FAM20A, RETN | 0,877 | 0,868 | 0,887 | 0,924 | 0,868 | 0,981 | 0,929 | 0,859 | 1,000 |
| IFI27, OAS1, IFIT1, SIGLEC1, HERC6, SLC1A2, OLAH, RETN, MMP8 | 0,877 | 0,867 | 0,887 | 0,932 | 0,884 | 0,980 | 0,949 | 0,890 | 1 |
| IFI27, IFIT1, SIGLEC1, ISG15, HERC6, IL1R2, OLAH, RETN, MMP8 | 0,877 | 0,868 | 0,886 | 0,926 | 0,872 | 0,980 | 0,954 | 0,906 | 1 |
| RSAD2, IFIT1, IFI44L, SIGLEC1, HERC6, IL1R2, OLAH, FAM20A, MMP8 | 0,877 | 0,868 | 0,886 | 0,924 | 0,870 | 0,977 | 0,963 | 0,909 | 1 |
| RSAD2, IFI27, OAS1, SIGLEC1, SLC1A2, IL1R2, OLAH, FAM20A, RETN | 0,877 | 0,867 | 0,887 | 0,930 | 0,878 | 0,983 | 0,940 | 0,873 | 1 |
| RSAD2, IFI27, OAS1, IFIT1, IFI44L, SIGLEC1, HERC6, FAM20A, MMP8 | 0,877 | 0,868 | 0,886 | 0,923 | 0,865 | 0,980 | 0,939 | 0,868 | 1 |
| IFI27, OAS1, IFIT1, IFI44L, SIGLEC1, HERC6, IL1R2, OLAH, MMP8 | 0,877 | 0,869 | 0,886 | 0,926 | 0,877 | 0,975 | 0,947 | 0,896 | 0,997 |
| RSAD2, IFI27, OAS1, IFI44L, SIGLEC1, SLC1A2, IL1R2, OLAH, FAM20A | 0,877 | 0,867 | 0,887 | 0,930 | 0,878 | 0,982 | 0,943 | 0,878 | 1 |
| OAS1, IFIT1, SIGLEC1, HERC6, IL1R2, OLAH, FAM20A, RETN, MMP8 | 0,877 | 0,868 | 0,886 | 0,925 | 0,870 | 0,980 | 0,962 | 0,907 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, HERC6, IL1R2, OLAH, RETN | 0,877 | 0,868 | 0,886 | 0,926 | 0,874 | 0,979 | 0,946 | 0,893 | 0,998 |
| IFI27, OAS1, IFIT1, IFI44L, SIGLEC1, ISG15, HERC6, IL1R2, RETN | 0,877 | 0,869 | 0,886 | 0,927 | 0,877 | 0,977 | 0,941 | 0,886 | 0,996 |
| IFI27, IFIT1, ISG15, SLC1A2, IL1R2, OLAH, FAM20A, RETN, MMP8 | 0,877 | 0,867 | 0,887 | 0,933 | 0,884 | 0,981 | 0,924 | 0,845 | 1 |
| IFI27, OAS1, IFIT1, IFI44L, HERC6, IL1R2, OLAH, RETN, MMP8 | 0,877 | 0,868 | 0,886 | 0,924 | 0,868 | 0,979 | 0,949 | 0,894 | 1 |
| IFI27, IFIT1, IFI44L, ISG15, SLC1A2, OLAH, FAM20A, RETN, MMP8 | 0,877 | 0,867 | 0,887 | 0,930 | 0,877 | 0,983 | 0,925 | 0,847 | 1 |
| RSAD2, IFI27, IFIT1, SIGLEC1, HERC6, SLC1A2, IL1R2, FAM20A, RETN | 0,877 | 0,868 | 0,887 | 0,935 | 0,889 | 0,981 | 0,943 | 0,873 | 1 |
| RSAD2, IFI27, IFIT1, SLC1A2, IL1R2, OLAH, FAM20A, RETN, MMP8 | 0,877 | 0,867 | 0,887 | 0,933 | 0,882 | 0,983 | 0,924 | 0,846 | 1 |
| IFI27, OAS1, IFIT1, IFI44L, ISG15, HERC6, OLAH, RETN, MMP8 | 0,877 | 0,868 | 0,886 | 0,924 | 0,867 | 0,981 | 0,945 | 0,887 | 1 |
| RSAD2, IFI27, OAS1, ISG15, HERC6, IL1R2, OLAH, RETN, MMP8 | 0,877 | 0,867 | 0,887 | 0,924 | 0,869 | 0,980 | 0,945 | 0,885 | 1 |
| RSAD2, IFI27, OAS1, IFIT1, IFI44L, SIGLEC1, HERC6, IL1R2, RETN | 0,877 | 0,869 | 0,885 | 0,924 | 0,871 | 0,977 | 0,953 | 0,902 | 1 |
| IFI27, IFIT1, SIGLEC1, ISG15, HERC6, SLC1A2, IL1R2, FAM20A, RETN | 0,877 | 0,867 | 0,887 | 0,935 | 0,889 | 0,981 | 0,947 | 0,875 | 1 |
| IFI27, OAS1, SIGLEC1, ISG15, HERC6, SLC1A2, IL1R2, FAM20A, RETN | 0,877 | 0,867 | 0,887 | 0,935 | 0,890 | 0,980 | 0,941 | 0,871 | 1 |
| IFI27, OAS1, IFIT1, ISG15, HERC6, SLC1A2, IL1R2, OLAH, FAM20A | 0,877 | 0,867 | 0,887 | 0,933 | 0,885 | 0,981 | 0,916 | 0,832 | 1 |
| RSAD2, IFI27, IFIT1, IFI44L, ISG15, HERC6, IL1R2, FAM20A, MMP8 | 0,877 | 0,867 | 0,887 | 0,928 | 0,877 | 0,979 | 0,932 | 0,850 | 1 |
| RSAD2, IFI27, IFIT1, IFI44L, ISG15, HERC6, IL1R2, FAM20A, RETN | 0,877 | 0,867 | 0,887 | 0,932 | 0,881 | 0,982 | 0,929 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, ISG15, HERC6, SLC1A2, IL1R2, OLAH, FAM20A | 0,877 | 0,867 | 0,887 | 0,934 | 0,885 | 0,983 | 0,920 | 0,839 | 1 |
| RSAD2, IFI27, OAS1, IFI44L, ISG15, IL1R2, OLAH, FAM20A, RETN | 0,877 | 0,867 | 0,887 | 0,924 | 0,867 | 0,981 | 0,928 | 0,856 | 1 |
| RSAD2, IFI27, IFIT1, IFI44L, ISG15, HERC6, SLC1A2, OLAH, FAM20A | 0,877 | 0,867 | 0,887 | 0,933 | 0,883 | 0,983 | 0,918 | 0,837 | 1,000 |
| IFI27, OAS1, IFIT1, IFI44L, SIGLEC1, ISG15, HERC6, IL1R2, OLAH | 0,877 | 0,868 | 0,885 | 0,927 | 0,879 | 0,976 | 0,942 | 0,889 | 0,996 |
| RSAD2, IFI27, OAS1, IFI44L, SIGLEC1, ISG15, HERC6, IL1R2, OLAH | 0,877 | 0,868 | 0,886 | 0,926 | 0,877 | 0,976 | 0,946 | 0,894 | 0,997 |
| IFI27, OAS1, IFIT1, SIGLEC1, ISG15, SLC1A2, IL1R2, FAM20A, RETN | 0,877 | 0,867 | 0,887 | 0,934 | 0,887 | 0,980 | 0,943 | 0,875 | 1 |
| RSAD2, IFI27, OAS1, IFIT1, IFI44L, SIGLEC1, HERC6, IL1R2, OLAH | 0,877 | 0,868 | 0,885 | 0,926 | 0,877 | 0,975 | 0,948 | 0,897 | 0,998 |
| IFI27, IFIT1, ISG15, HERC6, SLC1A2, OLAH, FAM20A, RETN, MMP8 | 0,877 | 0,867 | 0,887 | 0,931 | 0,880 | 0,982 | 0,921 | 0,838 | 1 |
| IFI27, OAS1, IFI44L, SIGLEC1, ISG15, HERC6, IL1R2, RETN, MMP8 | 0,877 | 0,868 | 0,885 | 0,928 | 0,877 | 0,979 | 0,952 | 0,902 | 1 |
| IFI27, IFI44L, HERC6, SLC1A2, IL1R2, OLAH, FAM20A, RETN, MMP8 | 0,877 | 0,867 | 0,887 | 0,932 | 0,882 | 0,982 | 0,932 | 0,856 | 1 |
| RSAD2, IFI27, IFI44L, SIGLEC1, HERC6, SLC1A2, OLAH, FAM20A, RETN | 0,877 | 0,867 | 0,887 | 0,933 | 0,883 | 0,983 | 0,941 | 0,874 | 1 |
| IFIT1, IFI44L, SIGLEC1, HERC6, IL1R2, OLAH, FAM20A, RETN, MMP8 | 0,877 | 0,867 | 0,886 | 0,925 | 0,870 | 0,980 | 0,961 | 0,904 | 1 |
| IFI27, IFIT1, SIGLEC1, ISG15, HERC6, SLC1A2, FAM20A, RETN, MMP8 | 0,877 | 0,867 | 0,887 | 0,930 | 0,877 | 0,983 | 0,951 | 0,879 | 1 |
| IFI27, IFIT1, IFI44L, SLC1A2, IL1R2, OLAH, FAM20A, RETN, MMP8 | 0,877 | 0,867 | 0,886 | 0,934 | 0,884 | 0,983 | 0,926 | 0,848 | 1 |
| RSAD2, IFI27, OAS1, IFIT1, SIGLEC1, ISG15, HERC6, RETN, MMP8 | 0,877 | 0,868 | 0,886 | 0,920 | 0,862 | 0,978 | 0,945 | 0,887 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, HERC6, FAM20A, MMP8 | 0,877 | 0,867 | 0,886 | 0,922 | 0,862 | 0,981 | 0,939 | 0,868 | 1 |
| RSAD2, IFI27, OAS1, IFIT1, IFI44L, SIGLEC1, FAM20A, RETN, MMP8 | 0,877 | 0,866 | 0,887 | 0,923 | 0,864 | 0,982 | 0,946 | 0,876 | 1 |
| RSAD2, IFI27, IFI44L, SIGLEC1, ISG15, HERC6, OLAH, RETN, MMP8 | 0,877 | 0,868 | 0,886 | 0,925 | 0,871 | 0,980 | 0,951 | 0,899 | 1 |
| OAS1, IFIT1, SIGLEC1, ISG15, HERC6, IL1R2, FAM20A, RETN, MMP8 | 0,877 | 0,868 | 0,886 | 0,923 | 0,867 | 0,979 | 0,961 | 0,903 | 1 |
| IFI27, IFIT1, IFI44L, ISG15, HERC6, SLC1A2, IL1R2, OLAH, FAM20A | 0,877 | 0,867 | 0,886 | 0,934 | 0,886 | 0,982 | 0,920 | 0,838 | 1 |
| RSAD2, OAS1, IFIT1, SIGLEC1, ISG15, HERC6, IL1R2, FAM20A, MMP8 | 0,876 | 0,867 | 0,886 | 0,922 | 0,868 | 0,977 | 0,963 | 0,907 | 1 |
| RSAD2, IFI27, OAS1, IFI44L, HERC6, IL1R2, OLAH, RETN, MMP8 | 0,876 | 0,867 | 0,886 | 0,923 | 0,867 | 0,979 | 0,943 | 0,885 | 1 |
| IFI27, OAS1, SIGLEC1, ISG15, HERC6, SLC1A2, FAM20A, RETN, MMP8 | 0,876 | 0,866 | 0,887 | 0,930 | 0,877 | 0,984 | 0,943 | 0,872 | 1 |
| RSAD2, IFI27, OAS1, IFIT1, SIGLEC1, ISG15, FAM20A, RETN, MMP8 | 0,876 | 0,866 | 0,887 | 0,923 | 0,863 | 0,982 | 0,946 | 0,874 | 1 |
| IFI27, OAS1, SIGLEC1, HERC6, SLC1A2, IL1R2, OLAH, RETN, MMP8 | 0,876 | 0,867 | 0,886 | 0,931 | 0,883 | 0,980 | 0,948 | 0,888 | 1 |
| RSAD2, IFI27, OAS1, IFI44L, SIGLEC1, HERC6, IL1R2, RETN, MMP8 | 0,876 | 0,867 | 0,885 | 0,927 | 0,874 | 0,980 | 0,954 | 0,903 | 1 |
| RSAD2, IFI27, ISG15, HERC6, SLC1A2, IL1R2, OLAH, FAM20A, RETN | 0,876 | 0,867 | 0,886 | 0,931 | 0,881 | 0,981 | 0,916 | 0,832 | 1 |
| RSAD2, IFI27, IFIT1, IFI44L, SIGLEC1, ISG15, FAM20A, RETN, MMP8 | 0,876 | 0,866 | 0,887 | 0,923 | 0,864 | 0,982 | 0,946 | 0,874 | 1 |
| IFI27, OAS1, IFIT1, SLC1A2, IL1R2, OLAH, FAM20A, RETN, MMP8 | 0,876 | 0,866 | 0,886 | 0,933 | 0,883 | 0,983 | 0,923 | 0,844 | 1 |
| RSAD2, IFI27, OAS1, IFI44L, ISG15, OLAH, FAM20A, RETN, MMP8 | 0,876 | 0,865 | 0,887 | 0,923 | 0,864 | 0,983 | 0,938 | 0,871 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, SLC1A2, IL1R2, FAM20A | 0,876 | 0,866 | 0,886 | 0,932 | 0,885 | 0,979 | 0,948 | 0,879 | 1 |
| RSAD2, IFI27, OAS1, IFI44L, ISG15, HERC6, IL1R2, FAM20A, RETN | 0,876 | 0,866 | 0,887 | 0,931 | 0,880 | 0,982 | 0,927 | 0,844 | 1 |
| IFIT1, SIGLEC1, ISG15, HERC6, IL1R2, OLAH, FAM20A, RETN, MMP8 | 0,876 | 0,867 | 0,886 | 0,923 | 0,867 | 0,979 | 0,962 | 0,906 | 1 |
| RSAD2, IFI27, IFIT1, IFI44L, SIGLEC1, ISG15, HERC6, OLAH, RETN | 0,876 | 0,868 | 0,885 | 0,925 | 0,872 | 0,978 | 0,946 | 0,893 | 0,998 |
| RSAD2, IFI27, IFIT1, IFI44L, HERC6, IL1R2, OLAH, RETN, MMP8 | 0,876 | 0,867 | 0,886 | 0,924 | 0,868 | 0,979 | 0,948 | 0,894 | 1 |
| RSAD2, IFI27, IFI44L, SIGLEC1, ISG15, HERC6, IL1R2, OLAH, RETN | 0,876 | 0,868 | 0,885 | 0,925 | 0,873 | 0,978 | 0,946 | 0,893 | 0,998 |
| IFI27, IFI44L, SIGLEC1, HERC6, SLC1A2, IL1R2, OLAH, FAM20A, RETN | 0,876 | 0,866 | 0,886 | 0,931 | 0,879 | 0,982 | 0,946 | 0,879 | 1 |
| OAS1, IFIT1, IFI44L, SIGLEC1, ISG15, HERC6, IL1R2, FAM20A, MMP8 | 0,876 | 0,866 | 0,886 | 0,923 | 0,869 | 0,978 | 0,963 | 0,907 | 1 |
| RSAD2, IFI27, OAS1, ISG15, HERC6, SLC1A2, OLAH, FAM20A, RETN | 0,876 | 0,866 | 0,886 | 0,934 | 0,886 | 0,982 | 0,917 | 0,831 | 1 |
| RSAD2, IFI27, OAS1, SIGLEC1, HERC6, SLC1A2, FAM20A, RETN, MMP8 | 0,876 | 0,866 | 0,887 | 0,930 | 0,876 | 0,984 | 0,941 | 0,867 | 1 |
| IFI27, OAS1, IFIT1, HERC6, SLC1A2, IL1R2, OLAH, FAM20A, RETN | 0,876 | 0,866 | 0,886 | 0,932 | 0,882 | 0,981 | 0,915 | 0,828 | 1 |
| RSAD2, IFI27, OAS1, IFI44L, ISG15, HERC6, OLAH, RETN, MMP8 | 0,876 | 0,867 | 0,886 | 0,924 | 0,868 | 0,980 | 0,942 | 0,883 | 1 |
| IFI27, OAS1, IFI44L, SIGLEC1, ISG15, SLC1A2, IL1R2, FAM20A, RETN | 0,876 | 0,866 | 0,886 | 0,933 | 0,886 | 0,981 | 0,941 | 0,871 | 1 |
| RSAD2, IFI27, OAS1, HERC6, SLC1A2, IL1R2, OLAH, FAM20A, RETN | 0,876 | 0,866 | 0,886 | 0,933 | 0,885 | 0,982 | 0,917 | 0,831 | 1 |
| RSAD2, IFI27, OAS1, IFIT1, ISG15, HERC6, IL1R2, FAM20A, RETN | 0,876 | 0,867 | 0,885 | 0,930 | 0,879 | 0,981 | 0,928 | 0,846 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, IL1R2, OLAH, MMP8 | 0,876 | 0,868 | 0,885 | 0,927 | 0,877 | 0,976 | 0,941 | 0,888 | 0,995 |
| IFI27, OAS1, IFIT1, IFI44L, ISG15, IL1R2, FAM20A, RETN, MMP8 | 0,876 | 0,866 | 0,886 | 0,929 | 0,876 | 0,982 | 0,935 | 0,858 | 1 |
| RSAD2, IFI27, OAS1, IFIT1, ISG15, HERC6, IL1R2, OLAH, RETN | 0,876 | 0,867 | 0,885 | 0,923 | 0,867 | 0,978 | 0,941 | 0,882 | 1 |
| RSAD2, IFI27, OAS1, IFI44L, SIGLEC1, ISG15, HERC6, IL1R2, RETN | 0,876 | 0,868 | 0,884 | 0,924 | 0,872 | 0,977 | 0,954 | 0,904 | 1 |
| RSAD2, IFI27, IFIT1, IFI44L, ISG15, OLAH, FAM20A, RETN, MMP8 | 0,876 | 0,865 | 0,887 | 0,922 | 0,861 | 0,982 | 0,938 | 0,872 | 1 |
| RSAD2, IFI27, OAS1, IFI44L, ISG15, HERC6, IL1R2, OLAH, RETN | 0,876 | 0,867 | 0,885 | 0,922 | 0,866 | 0,978 | 0,940 | 0,880 | 1 |
| IFI27, IFIT1, IFI44L, ISG15, HERC6, SLC1A2, OLAH, FAM20A, RETN | 0,876 | 0,866 | 0,886 | 0,932 | 0,882 | 0,982 | 0,918 | 0,836 | 1 |
| RSAD2, IFI27, IFIT1, SIGLEC1, ISG15, HERC6, IL1R2, OLAH, RETN | 0,876 | 0,867 | 0,885 | 0,926 | 0,873 | 0,978 | 0,942 | 0,887 | 0,998 |
| IFI27, OAS1, IFIT1, IFI44L, SIGLEC1, ISG15, HERC6, OLAH, MMP8 | 0,876 | 0,867 | 0,885 | 0,927 | 0,876 | 0,978 | 0,942 | 0,888 | 0,997 |
| IFI27, OAS1, IFI44L, SIGLEC1, HERC6, SLC1A2, FAM20A, RETN, MMP8 | 0,876 | 0,866 | 0,886 | 0,931 | 0,879 | 0,984 | 0,942 | 0,869 | 1 |
| RSAD2, IFI27, IFIT1, ISG15, HERC6, SLC1A2, IL1R2, OLAH, FAM20A | 0,876 | 0,866 | 0,886 | 0,932 | 0,883 | 0,982 | 0,915 | 0,831 | 0,999 |
| RSAD2, IFI27, IFIT1, IFI44L, SIGLEC1, HERC6, IL1R2, OLAH, MMP8 | 0,876 | 0,867 | 0,884 | 0,927 | 0,878 | 0,975 | 0,943 | 0,892 | 0,995 |
| RSAD2, IFI27, OAS1, IFIT1, IFI44L, IL1R2, OLAH, FAM20A, RETN | 0,876 | 0,866 | 0,886 | 0,925 | 0,868 | 0,982 | 0,932 | 0,860 | 1 |
| RSAD2, IFI27, OAS1, IFIT1, SIGLEC1, ISG15, SLC1A2, IL1R2, FAM20A | 0,876 | 0,867 | 0,885 | 0,935 | 0,889 | 0,981 | 0,943 | 0,875 | 1 |
| RSAD2, IFI27, IFIT1, IFI44L, SIGLEC1, HERC6, SLC1A2, FAM20A, RETN | 0,876 | 0,866 | 0,885 | 0,931 | 0,882 | 0,981 | 0,939 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, HERC6, RETN, MMP8 | 0,876 | 0,867 | 0,885 | 0,920 | 0,862 | 0,978 | 0,941 | 0,879 | 1 |
| IFI27, OAS1, ISG15, HERC6, SLC1A2, IL1R2, OLAH, FAM20A, RETN | 0,876 | 0,866 | 0,886 | 0,933 | 0,884 | 0,982 | 0,915 | 0,829 | 1 |
| RSAD2, IFI27, IFI44L, ISG15, HERC6, SLC1A2, OLAH, FAM20A, RETN | 0,876 | 0,866 | 0,886 | 0,935 | 0,886 | 0,983 | 0,917 | 0,836 | 0,999 |
| RSAD2, IFI27, OAS1, SIGLEC1, HERC6, SLC1A2, IL1R2, FAM20A, RETN | 0,876 | 0,866 | 0,886 | 0,934 | 0,888 | 0,980 | 0,940 | 0,868 | 1 |
| IFI27, IFI44L, ISG15, HERC6, SLC1A2, IL1R2, OLAH, FAM20A, RETN | 0,876 | 0,866 | 0,886 | 0,931 | 0,881 | 0,982 | 0,917 | 0,834 | 1 |
| RSAD2, IFI27, ISG15, SLC1A2, IL1R2, OLAH, FAM20A, RETN, MMP8 | 0,876 | 0,866 | 0,886 | 0,935 | 0,887 | 0,984 | 0,922 | 0,841 | 1 |
| IFI27, OAS1, SIGLEC1, ISG15, HERC6, SLC1A2, OLAH, RETN, MMP8 | 0,876 | 0,866 | 0,885 | 0,932 | 0,884 | 0,981 | 0,947 | 0,886 | 1 |
| RSAD2, IFI27, OAS1, IFIT1, SIGLEC1, ISG15, HERC6, FAM20A, MMP8 | 0,876 | 0,866 | 0,885 | 0,923 | 0,866 | 0,980 | 0,939 | 0,868 | 1 |
| RSAD2, IFI27, OAS1, IFIT1, SIGLEC1, ISG15, HERC6, IL1R2, RETN | 0,876 | 0,867 | 0,884 | 0,925 | 0,873 | 0,977 | 0,950 | 0,896 | 1 |
| IFI27, IFI44L, SIGLEC1, ISG15, HERC6, SLC1A2, IL1R2, FAM20A, RETN | 0,876 | 0,866 | 0,886 | 0,933 | 0,886 | 0,980 | 0,947 | 0,875 | 1 |
| RSAD2, IFI27, OAS1, IFI44L, SIGLEC1, HERC6, SLC1A2, IL1R2, FAM20A | 0,876 | 0,865 | 0,886 | 0,935 | 0,890 | 0,979 | 0,943 | 0,872 | 1 |
| RSAD2, IFI27, IFIT1, IFI44L, ISG15, IL1R2, OLAH, FAM20A, RETN | 0,876 | 0,866 | 0,885 | 0,924 | 0,867 | 0,981 | 0,932 | 0,861 | 1 |
| RSAD2, IFI27, OAS1, IFIT1, IFI44L, SIGLEC1, HERC6, OLAH, MMP8 | 0,876 | 0,867 | 0,884 | 0,926 | 0,875 | 0,977 | 0,945 | 0,892 | 0,997 |
| RSAD2, IFI27, OAS1, IFIT1, IFI44L, ISG15, OLAH, FAM20A, RETN | 0,876 | 0,866 | 0,885 | 0,921 | 0,861 | 0,981 | 0,936 | 0,868 | 1 |
| RSAD2, IFI27, IFIT1, IFI44L, SIGLEC1, ISG15, SLC1A2, IL1R2, FAM20A | 0,875 | 0,867 | 0,884 | 0,934 | 0,889 | 0,980 | 0,945 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, HERC6, IL1R2, OLAH, MMP8 | 0,875 | 0,866 | 0,885 | 0,924 | 0,875 | 0,974 | 0,943 | 0,891 | 0,996 |
| RSAD2, IFI27, OAS1, IFI44L, SIGLEC1, SLC1A2, OLAH, FAM20A, RETN | 0,875 | 0,865 | 0,886 | 0,927 | 0,871 | 0,983 | 0,939 | 0,872 | 1 |
| RSAD2, IFI27, IFI44L, SLC1A2, IL1R2, OLAH, FAM20A, RETN, MMP8 | 0,875 | 0,865 | 0,886 | 0,937 | 0,889 | 0,985 | 0,927 | 0,852 | 1 |
| RSAD2, IFI27, OAS1, IFIT1, IFI44L, SIGLEC1, SLC1A2, IL1R2, FAM20A | 0,875 | 0,866 | 0,885 | 0,933 | 0,885 | 0,981 | 0,945 | 0,878 | 1 |
| IFI27, OAS1, IFIT1, SIGLEC1, ISG15, HERC6, IL1R2, OLAH, MMP8 | 0,875 | 0,867 | 0,884 | 0,924 | 0,875 | 0,974 | 0,943 | 0,891 | 0,996 |
| IFI27, OAS1, IFIT1, SIGLEC1, HERC6, SLC1A2, IL1R2, OLAH, RETN | 0,875 | 0,866 | 0,885 | 0,930 | 0,880 | 0,980 | 0,939 | 0,876 | 1 |
| RSAD2, IFI27, IFIT1, SIGLEC1, ISG15, SLC1A2, IL1R2, FAM20A, RETN | 0,875 | 0,866 | 0,885 | 0,934 | 0,888 | 0,981 | 0,945 | 0,875 | 1 |
| IFI27, IFIT1, IFI44L, SIGLEC1, ISG15, HERC6, IL1R2, OLAH, MMP8 | 0,875 | 0,867 | 0,884 | 0,927 | 0,878 | 0,976 | 0,940 | 0,886 | 0,994 |
| RSAD2, IFI27, OAS1, IFIT1, IFI44L, ISG15, HERC6, OLAH, RETN | 0,875 | 0,866 | 0,884 | 0,922 | 0,865 | 0,978 | 0,941 | 0,882 | 1 |
| IFI27, OAS1, IFIT1, ISG15, SLC1A2, IL1R2, OLAH, FAM20A, RETN | 0,875 | 0,866 | 0,885 | 0,929 | 0,877 | 0,980 | 0,922 | 0,842 | 1 |
| RSAD2, IFI27, OAS1, IFI44L, HERC6, SLC1A2, OLAH, FAM20A, RETN | 0,875 | 0,865 | 0,885 | 0,932 | 0,882 | 0,982 | 0,918 | 0,835 | 1 |
| RSAD2, IFI27, OAS1, IFI44L, ISG15, HERC6, FAM20A, RETN, MMP8 | 0,875 | 0,864 | 0,886 | 0,927 | 0,870 | 0,984 | 0,938 | 0,861 | 1 |
| RSAD2, IFI27, OAS1, IFIT1, SIGLEC1, ISG15, HERC6, IL1R2, OLAH | 0,875 | 0,866 | 0,884 | 0,924 | 0,874 | 0,974 | 0,943 | 0,891 | 0,996 |
| RSAD2, IFI27, OAS1, IFIT1, IFI44L, ISG15, SLC1A2, OLAH, FAM20A | 0,875 | 0,865 | 0,885 | 0,930 | 0,877 | 0,983 | 0,920 | 0,839 | 1 |
| RSAD2, IFI27, IFIT1, IFI44L, HERC6, SLC1A2, OLAH, FAM20A, RETN | 0,875 | 0,865 | 0,885 | 0,933 | 0,883 | 0,982 | 0,921 | 0,839 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, SIGLEC1, HERC6, IL1R2, FAM20A, RETN, MMP8 | 0,875 | 0,866 | 0,884 | 0,923 | 0,867 | 0,979 | 0,962 | 0,905 | 1 |
| RSAD2, IFIT1, IFI44L, SIGLEC1, ISG15, HERC6, IL1R2, FAM20A, MMP8 | 0,875 | 0,866 | 0,884 | 0,922 | 0,867 | 0,977 | 0,963 | 0,907 | 1 |
| IFI27, OAS1, IFIT1, IFI44L, SIGLEC1, HERC6, SLC1A2, FAM20A, MMP8 | 0,875 | 0,866 | 0,884 | 0,927 | 0,876 | 0,977 | 0,935 | 0,859 | 1 |
| IFI27, OAS1, ISG15, SLC1A2, IL1R2, OLAH, FAM20A, RETN, MMP8 | 0,875 | 0,865 | 0,885 | 0,933 | 0,884 | 0,981 | 0,923 | 0,843 | 1 |
| IFI27, OAS1, IFI44L, SIGLEC1, HERC6, SLC1A2, OLAH, RETN, MMP8 | 0,875 | 0,865 | 0,885 | 0,931 | 0,883 | 0,980 | 0,947 | 0,887 | 1 |
| RSAD2, IFI27, OAS1, SLC1A2, IL1R2, OLAH, FAM20A, RETN, MMP8 | 0,875 | 0,865 | 0,885 | 0,933 | 0,883 | 0,982 | 0,923 | 0,842 | 1 |
| IFI27, IFIT1, IFI44L, ISG15, SLC1A2, IL1R2, OLAH, FAM20A, RETN | 0,875 | 0,866 | 0,884 | 0,931 | 0,880 | 0,982 | 0,917 | 0,836 | 0,999 |
| RSAD2, IFI27, IFI44L, ISG15, HERC6, IL1R2, OLAH, RETN, MMP8 | 0,875 | 0,866 | 0,884 | 0,924 | 0,869 | 0,980 | 0,945 | 0,889 | 1 |
| RSAD2, IFI27, IFI44L, SIGLEC1, SLC1A2, IL1R2, OLAH, FAM20A, RETN | 0,875 | 0,864 | 0,886 | 0,930 | 0,877 | 0,983 | 0,946 | 0,881 | 1 |
| RSAD2, IFI27, OAS1, IFI44L, ISG15, SLC1A2, IL1R2, OLAH, FAM20A | 0,875 | 0,865 | 0,885 | 0,931 | 0,880 | 0,982 | 0,920 | 0,837 | 1 |
| IFI27, OAS1, IFI44L, HERC6, SLC1A2, IL1R2, OLAH, FAM20A, RETN | 0,875 | 0,865 | 0,885 | 0,934 | 0,885 | 0,982 | 0,918 | 0,835 | 1 |
| IFI27, OAS1, IFIT1, IFI44L, SIGLEC1, IL1R2, OLAH, RETN, MMP8 | 0,875 | 0,866 | 0,884 | 0,924 | 0,870 | 0,978 | 0,958 | 0,904 | 1 |
| RSAD2, IFI27, OAS1, IFIT1, HERC6, SLC1A2, OLAH, FAM20A, RETN | 0,875 | 0,865 | 0,885 | 0,933 | 0,883 | 0,982 | 0,917 | 0,831 | 1 |
| RSAD2, OAS1, IFIT1, IFI44L, SIGLEC1, IL1R2, OLAH, FAM20A, MMP8 | 0,875 | 0,865 | 0,884 | 0,921 | 0,865 | 0,978 | 0,965 | 0,913 | 1 |
| RSAD2, IFI27, OAS1, SIGLEC1, HERC6, SLC1A2, OLAH, RETN, MMP8 | 0,875 | 0,865 | 0,885 | 0,930 | 0,880 | 0,980 | 0,948 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, SLC1A2, IL1R2, FAM20A | 0,875 | 0,865 | 0,885 | 0,934 | 0,888 | 0,980 | 0,941 | 0,869 | 1 |
| RSAD2, OAS1, IFIT1, SIGLEC1, ISG15, IL1R2, OLAH, FAM20A, MMP8 | 0,875 | 0,866 | 0,884 | 0,920 | 0,863 | 0,977 | 0,966 | 0,913 | 1 |
| RSAD2, IFI27, OAS1, IFI44L, SIGLEC1, ISG15, HERC6, OLAH, MMP8 | 0,875 | 0,866 | 0,884 | 0,925 | 0,873 | 0,977 | 0,943 | 0,890 | 0,997 |
| RSAD2,IFI27,OAS1,IFIT1, IFI44L, HERC6, IL1R2, OLAH, RETN | 0,875 | 0,866 | 0,884 | 0,922 | 0,867 | 0,978 | 0,939 | 0,879 | 0,999 |
| RSAD2, IFI27, OAS1, SIGLEC1, ISG15, HERC6, IL1R2, RETN, MMP8 | 0,875 | 0,865 | 0,884 | 0,925 | 0,872 | 0,978 | 0,954 | 0,902 | 1 |
| RSAD2,IFI27,OAS1,IFIT1, SLC1A2, OLAH, FAM20A, RETN, MMP8 | 0,875 | 0,865 | 0,885 | 0,926 | 0,870 | 0,981 | 0,928 | 0,854 | 1 |
| RSAD2, IFI27, IFIT1, SIGLEC1, ISG15, HERC6, SLC1A2, FAM20A, RETN | 0,875 | 0,865 | 0,884 | 0,930 | 0,879 | 0,981 | 0,938 | 0,865 | 1 |
| IFIT1, IFI44L, SIGLEC1, ISG15, HERC6, SLC1A2, OLAH, FAM20A, MMP8 | 0,875 | 0,865 | 0,885 | 0,930 | 0,881 | 0,978 | 0,941 | 0,871 | 1 |
| RSAD2, IFI27, OAS1, IFI44L, SIGLEC1, ISG15, HERC6, RETN, MMP8 | 0,875 | 0,865 | 0,884 | 0,921 | 0,863 | 0,979 | 0,945 | 0,881 | 1 |
| RSAD2, IFI27, IFIT1, IFI44L, SIGLEC1, ISG15, HERC6, IL1R2, OLAH | 0,875 | 0,866 | 0,883 | 0,926 | 0,876 | 0,976 | 0,941 | 0,887 | 0,995 |
| IFI27, OAS1, IFIT1, SIGLEC1, ISG15, HERC6, SLC1A2, OLAH, RETN | 0,875 | 0,865 | 0,885 | 0,930 | 0,881 | 0,978 | 0,940 | 0,878 | 1 |
| IFI27, OAS1, SIGLEC1, ISG15, HERC6, SLC1A2, IL1R2, OLAH, RETN | 0,875 | 0,865 | 0,884 | 0,927 | 0,877 | 0,978 | 0,939 | 0,876 | 1 |
| RSAD2,IFI27,OAS1,IFIT1, ISG15, SLC1A2, IL1R2, OLAH, FAM20A | 0,875 | 0,865 | 0,884 | 0,930 | 0,879 | 0,980 | 0,921 | 0,838 | 1 |
| IFI27, OAS1, IFIT1, ISG15, SLC1A2, OLAH, FAM20A, RETN, MMP8 | 0,875 | 0,864 | 0,885 | 0,929 | 0,876 | 0,982 | 0,926 | 0,850 | 1 |
| RSAD2,IFI27,OAS1,IFIT1, SIGLEC1, HERC6, IL1R2, OLAH, MMP8 | 0,875 | 0,866 | 0,884 | 0,924 | 0,875 | 0,974 | 0,942 | 0,890 | 0,995 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SLC1A2, OLAH, FAM20A, RETN, MMP8 | 0,875 | 0,864 | 0,885 | 0,929 | 0,873 | 0,984 | 0,929 | 0,854 | 1 |
| RSAD2, IFI27, OAS1, IFIT1, SIGLEC1, SLC1A2, IL1R2, FAM20A, RETN | 0,875 | 0,865 | 0,884 | 0,934 | 0,886 | 0,981 | 0,943 | 0,877 | 1 |
| RSAD2, IFI27, IFIT1, ISG15, SLC1A2, IL1R2, OLAH, FAM20A, RETN | 0,875 | 0,865 | 0,884 | 0,928 | 0,877 | 0,980 | 0,918 | 0,837 | 1,000 |
| RSAD2, IFI27, OAS1, IFIT1, IFI44L, SIGLEC1, ISG15, HERC6, OLAH | 0,875 | 0,866 | 0,883 | 0,926 | 0,874 | 0,978 | 0,943 | 0,890 | 0,997 |
| IFI27, OAS1, IFIT1, HERC6, SLC1A2, IL1R2, OLAH, RETN, MMP8 | 0,875 | 0,865 | 0,884 | 0,929 | 0,879 | 0,979 | 0,939 | 0,874 | 1 |
| IFI27, OAS1, IFIT1, HERC6, SLC1A2, IL1R2, FAM20A, RETN, MMP8 | 0,875 | 0,865 | 0,885 | 0,932 | 0,883 | 0,981 | 0,925 | 0,842 | 1 |
| RSAD2, IFI27, IFIT1, IFI44L, ISG15, SLC1A2, IL1R2, OLAH, FAM20A | 0,875 | 0,865 | 0,884 | 0,931 | 0,880 | 0,982 | 0,917 | 0,836 | 0,999 |
| RSAD2, IFI27, IFIT1, ISG15, HERC6, SLC1A2, OLAH, FAM20A, RETN | 0,875 | 0,865 | 0,885 | 0,930 | 0,879 | 0,982 | 0,915 | 0,830 | 1 |
| IFI27, OAS1, IFIT1, IFI44L, ISG15, SLC1A2, IL1R2, OLAH, FAM20A | 0,875 | 0,865 | 0,884 | 0,930 | 0,877 | 0,982 | 0,921 | 0,840 | 1 |
| IFI27, OAS1, IFIT1, IFI44L, ISG15, HERC6, IL1R2, OLAH, RETN | 0,875 | 0,866 | 0,884 | 0,923 | 0,869 | 0,977 | 0,937 | 0,875 | 0,998 |
| IFI27, OAS1, IFI44L, ISG15, SLC1A2, OLAH, FAM20A, RETN, MMP8 | 0,875 | 0,863 | 0,886 | 0,931 | 0,878 | 0,983 | 0,928 | 0,852 | 1 |
| RSAD2, IFI27, IFIT1, IFI44L, ISG15, HERC6, OLAH, RETN, MMP8 | 0,875 | 0,865 | 0,884 | 0,923 | 0,866 | 0,979 | 0,947 | 0,893 | 1 |
| RSAD2, IFI27, IFIT1, ISG15, HERC6, IL1R2, OLAH, RETN, MMP8 | 0,875 | 0,865 | 0,884 | 0,924 | 0,869 | 0,980 | 0,946 | 0,891 | 1 |
| RSAD2, IFI27, OAS1, IFIT1, SIGLEC1, HERC6, SLC1A2, OLAH, RETN | 0,875 | 0,865 | 0,884 | 0,932 | 0,883 | 0,981 | 0,946 | 0,885 | 1 |
| IFI27, IFIT1, IFI44L, HERC6, SLC1A2, IL1R2, OLAH, FAM20A, RETN | 0,875 | 0,865 | 0,884 | 0,931 | 0,879 | 0,983 | 0,925 | 0,845 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, ISG15, HERC6, SLC1A2, FAM20A, RETN, MMP8 | 0,874 | 0,864 | 0,885 | 0,929 | 0,876 | 0,983 | 0,945 | 0,871 | 1 |
| RSAD2, IFIT1, SIGLEC1, ISG15, HERC6, IL1R2, FAM20A, RETN, MMP8 | 0,874 | 0,865 | 0,883 | 0,923 | 0,867 | 0,979 | 0,961 | 0,901 | 1 |
| OAS1, IFIT1, SIGLEC1, HERC6, SLC1A2, IL1R2, OLAH, FAM20A, MMP8 | 0,874 | 0,865 | 0,884 | 0,929 | 0,882 | 0,975 | 0,961 | 0,903 | 1 |
| IFI27, OAS1, IFIT1, IFI44L, SIGLEC1, HERC6, SLC1A2, OLAH, RETN | 0,874 | 0,865 | 0,884 | 0,931 | 0,884 | 0,979 | 0,938 | 0,875 | 1 |
| RSAD2, IFI27, IFIT1, HERC6, SLC1A2, IL1R2, OLAH, FAM20A, RETN | 0,874 | 0,865 | 0,884 | 0,931 | 0,881 | 0,981 | 0,918 | 0,834 | 1 |
| RSAD2, IFI27, IFIT1, ISG15, SLC1A2, OLAH, FAM20A, RETN, MMP8 | 0,874 | 0,864 | 0,885 | 0,929 | 0,875 | 0,982 | 0,926 | 0,850 | 1 |
| IFI27, OAS1, IFIT1, IFI44L, ISG15, SLC1A2, OLAH, FAM20A, RETN | 0,874 | 0,865 | 0,884 | 0,929 | 0,875 | 0,983 | 0,917 | 0,835 | 1,000 |
| RSAD2, IFI27, OAS1, IFIT1, ISG15, HERC6, FAM20A, RETN, MMP8 | 0,874 | 0,864 | 0,885 | 0,925 | 0,868 | 0,982 | 0,935 | 0,857 | 1 |
| RSAD2, IFIT1, SIGLEC1, HERC6, IL1R2, OLAH, FAM20A, RETN, MMP8 | 0,874 | 0,865 | 0,884 | 0,926 | 0,871 | 0,980 | 0,961 | 0,904 | 1 |
| IFIT1, IFI44L, SIGLEC1, ISG15, HERC6, OLAH, FAM20A, RETN, MMP8 | 0,874 | 0,864 | 0,884 | 0,922 | 0,864 | 0,980 | 0,952 | 0,895 | 1 |
| RSAD2, IFI27, IFIT1, IFI44L, ISG15, HERC6, FAM20A, RETN, MMP8 | 0,874 | 0,864 | 0,885 | 0,926 | 0,869 | 0,982 | 0,937 | 0,859 | 1 |
| IFI27, OAS1, IFIT1, IFI44L, SIGLEC1, SLC1A2, FAM20A, RETN, MMP8 | 0,874 | 0,864 | 0,884 | 0,927 | 0,872 | 0,982 | 0,942 | 0,869 | 1 |
| IFI27, OAS1, IFI44L, SIGLEC1, ISG15, HERC6, SLC1A2, FAM20A, RETN | 0,874 | 0,864 | 0,884 | 0,927 | 0,874 | 0,980 | 0,939 | 0,865 | 1 |
| RSAD2, IFI27, IFI44L, SIGLEC1, HERC6, SLC1A2, IL1R2, FAM20A, RETN | 0,874 | 0,864 | 0,885 | 0,934 | 0,886 | 0,981 | 0,947 | 0,877 | 1 |
| RSAD2, IFI27, IFI44L, ISG15, SLC1A2, IL1R2, OLAH, FAM20A, RETN | 0,874 | 0,864 | 0,884 | 0,931 | 0,880 | 0,982 | 0,918 | 0,837 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, SLC1A2, IL1R2, FAM20A, RETN | 0,874 | 0,864 | 0,884 | 0,933 | 0,885 | 0,980 | 0,943 | 0,873 | 1 |
| OAS1, IFIT1, IFI44L, SIGLEC1, ISG15, IL1R2, OLAH, FAM20A, MMP8 | 0,874 | 0,864 | 0,884 | 0,920 | 0,863 | 0,977 | 0,966 | 0,913 | 1 |
| RSAD2, IFI27, IFIT1, IFI44L, SLC1A2, OLAH, FAM20A, RETN, MMP8 | 0,874 | 0,864 | 0,884 | 0,928 | 0,874 | 0,983 | 0,929 | 0,854 | 1 |
| OAS1, IFIT1, IFI44L, SIGLEC1, HERC6, OLAH, FAM20A, RETN, MMP8 | 0,874 | 0,864 | 0,884 | 0,920 | 0,862 | 0,979 | 0,951 | 0,894 | 1 |
| IFI27, OAS1, IFI44L, ISG15, SLC1A2, IL1R2, OLAH, FAM20A, RETN | 0,874 | 0,864 | 0,884 | 0,931 | 0,880 | 0,983 | 0,916 | 0,833 | 1,000 |
| IFI27, OAS1, IFIT1, IFI44L, SIGLEC1, ISG15, SLC1A2, FAM20A, RETN | 0,874 | 0,865 | 0,884 | 0,925 | 0,870 | 0,980 | 0,938 | 0,865 | 1 |
| RSAD2, IFI27, IFIT1, IFI44L, SIGLEC1, HERC6, IL1R2, RETN, MMP8 | 0,874 | 0,865 | 0,883 | 0,924 | 0,872 | 0,977 | 0,953 | 0,908 | 0,999 |
| RSAD2, IFI27, OAS1, IFIT1, IFI44L, IL1R2, FAM20A, RETN, MMP8 | 0,874 | 0,864 | 0,885 | 0,928 | 0,875 | 0,981 | 0,934 | 0,856 | 1 |
| IFIT1, IFI44L, SIGLEC1, HERC6, SLC1A2, IL1R2, OLAH, FAM20A, MMP8 | 0,874 | 0,864 | 0,884 | 0,929 | 0,882 | 0,976 | 0,960 | 0,897 | 1 |
| RSAD2, IFI27, OAS1, IFI44L, SIGLEC1, HERC6, SLC1A2, OLAH, RETN | 0,874 | 0,865 | 0,883 | 0,931 | 0,882 | 0,980 | 0,943 | 0,881 | 1 |
| RSAD2, IFI27, SIGLEC1, HERC6, SLC1A2, IL1R2, OLAH, RETN, MMP8 | 0,874 | 0,865 | 0,884 | 0,931 | 0,883 | 0,980 | 0,951 | 0,898 | 1 |
| RSAD2, IFI27, OAS1, ISG15, SLC1A2, IL1R2, OLAH, FAM20A, RETN | 0,874 | 0,864 | 0,884 | 0,932 | 0,882 | 0,981 | 0,915 | 0,830 | 1 |
| IFI27, IFIT1, IFI44L, ISG15, HERC6, IL1R2, OLAH, RETN, MMP8 | 0,874 | 0,865 | 0,883 | 0,922 | 0,865 | 0,978 | 0,942 | 0,884 | 1 |
| OAS1, IFIT1, IFI44L, SIGLEC1, HERC6, SLC1A2, IL1R2, FAM20A, MMP8 | 0,874 | 0,864 | 0,884 | 0,927 | 0,881 | 0,973 | 0,961 | 0,901 | 1 |
| RSAD2, IFI27, IFI44L, ISG15, SLC1A2, OLAH, FAM20A, RETN, MMP8 | 0,874 | 0,863 | 0,885 | 0,931 | 0,879 | 0,984 | 0,925 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SLC1A2, IL1R2, OLAH, FAM20A, RETN, MMP8 | 0,874 | 0,863 | 0,885 | 0,936 | 0,888 | 0,985 | 0,927 | 0,851 | 1 |
| RSAD2, OAS1, IFI44L, SIGLEC1, HERC6, IL1R2, OLAH, FAM20A, MMP8 | 0,874 | 0,864 | 0,884 | 0,921 | 0,865 | 0,976 | 0,961 | 0,903 | 1 |
| RSAD2, IFI27, IFI44L, HERC6, SLC1A2, IL1R2, OLAH, FAM20A, RETN | 0,874 | 0,864 | 0,884 | 0,932 | 0,882 | 0,982 | 0,923 | 0,842 | 1 |
| IFI27, OAS1, IFIT1, IFI44L, HERC6, SLC1A2, IL1R2, FAM20A, MMP8 | 0,874 | 0,864 | 0,884 | 0,931 | 0,885 | 0,977 | 0,925 | 0,838 | 1 |
| IFI27, IFIT1, IFI44L, SIGLEC1, ISG15, IL1R2, OLAH, RETN, MMP8 | 0,874 | 0,865 | 0,883 | 0,925 | 0,871 | 0,979 | 0,953 | 0,900 | 1 |
| IFI27, OAS1, IFIT1, SIGLEC1, HERC6, SLC1A2, IL1R2, RETN, MMP8 | 0,874 | 0,865 | 0,883 | 0,928 | 0,881 | 0,975 | 0,958 | 0,907 | 1 |
| RSAD2, IFI27, OAS1, SIGLEC1, HERC6, SLC1A2, IL1R2, OLAH, RETN | 0,874 | 0,864 | 0,884 | 0,929 | 0,878 | 0,980 | 0,945 | 0,883 | 1 |
| RSAD2, IFI27, OAS1, IFI44L, ISG15, HERC6, IL1R2, OLAH, MMP8 | 0,874 | 0,865 | 0,883 | 0,927 | 0,878 | 0,976 | 0,937 | 0,879 | 0,995 |
| RSAD2, IFI27, OAS1, IFIT1, SIGLEC1, ISG15, HERC6, OLAH, MMP8 | 0,874 | 0,865 | 0,883 | 0,923 | 0,871 | 0,975 | 0,936 | 0,878 | 0,994 |
| RSAD2, IFI27, OAS1, IFIT1, IFI44L, ISG15, IL1R2, FAM20A, MMP8 | 0,874 | 0,864 | 0,884 | 0,926 | 0,873 | 0,978 | 0,934 | 0,856 | 1 |
| IFI27, OAS1, IFIT1, SIGLEC1, ISG15, IL1R2, OLAH, RETN, MMP8 | 0,874 | 0,864 | 0,883 | 0,923 | 0,868 | 0,978 | 0,960 | 0,911 | 1 |
| IFIT1, IFI44L, SIGLEC1, ISG15, SLC1A2, IL1R2, OLAH, FAM20A, MMP8 | 0,874 | 0,865 | 0,883 | 0,927 | 0,880 | 0,975 | 0,961 | 0,903 | 1 |
| RSAD2, IFI27, OAS1, IFIT1, IFI44L, SLC1A2, IL1R2, OLAH, FAM20A | 0,874 | 0,864 | 0,883 | 0,930 | 0,876 | 0,983 | 0,920 | 0,837 | 1 |
| OAS1, IFIT1, SIGLEC1, ISG15, HERC6, OLAH, FAM20A, RETN, MMP8 | 0,874 | 0,864 | 0,884 | 0,921 | 0,864 | 0,979 | 0,952 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, HERC6, SLC1A2, OLAH, RETN, MMP8 | 0,874 | 0,864 | 0,883 | 0,931 | 0,882 | 0,980 | 0,951 | 0,899 | 1 |
| IFI27, OAS1, IFIT1, SIGLEC1, ISG15, HERC6, SLC1A2, OLAH, MMP8 | 0,874 | 0,864 | 0,884 | 0,930 | 0,880 | 0,979 | 0,937 | 0,877 | 0,997 |
| IFI27, IFIT1, ISG15, HERC6, SLC1A2, IL1R2, OLAH, FAM20A, RETN | 0,874 | 0,864 | 0,884 | 0,931 | 0,881 | 0,981 | 0,917 | 0,834 | 1 |
| RSAD2, OAS1, SIGLEC1, ISG15, HERC6, IL1R2, OLAH, FAM20A, MMP8 | 0,874 | 0,864 | 0,883 | 0,917 | 0,860 | 0,975 | 0,962 | 0,905 | 1 |
| RSAD2, OAS1, IFIT1, IFI44L, SIGLEC1, HERC6, IL1R2, OLAH, FAM20A | 0,874 | 0,864 | 0,883 | 0,918 | 0,860 | 0,975 | 0,953 | 0,898 | 1 |
| IFI27, OAS1, IFIT1, SIGLEC1, ISG15, SLC1A2, FAM20A, RETN, MMP8 | 0,874 | 0,864 | 0,884 | 0,926 | 0,870 | 0,982 | 0,943 | 0,868 | 1 |
| RSAD2, IFI27, OAS1, IFIT1, IFI44L, SIGLEC1, OLAH, RETN, MMP8 | 0,874 | 0,864 | 0,883 | 0,921 | 0,863 | 0,978 | 0,957 | 0,901 | 1 |
| RSAD2, IFI27, OAS1, IFI44L, SIGLEC1, ISG15, FAM20A, RETN, MMP8 | 0,874 | 0,863 | 0,885 | 0,924 | 0,866 | 0,983 | 0,947 | 0,875 | 1 |
| RSAD2, IFI27, IFI44L, SIGLEC1, ISG15, SLC1A2, IL1R2, FAM20A, RETN | 0,874 | 0,864 | 0,883 | 0,933 | 0,885 | 0,980 | 0,943 | 0,872 | 1 |
| RSAD2, IFI27, IFIT1, IFI44L, ISG15, SLC1A2, OLAH, FAM20A, RETN | 0,874 | 0,864 | 0,883 | 0,928 | 0,874 | 0,982 | 0,916 | 0,834 | 0,999 |
| RSAD2, IFI27, IFIT1, IFI44L, SIGLEC1, SLC1A2, IL1R2, FAM20A, RETN | 0,874 | 0,863 | 0,884 | 0,934 | 0,886 | 0,981 | 0,943 | 0,874 | 1 |
| IFI27, IFIT1, IFI44L, SIGLEC1, ISG15, HERC6, IL1R2, RETN, MMP8 | 0,874 | 0,865 | 0,883 | 0,923 | 0,870 | 0,976 | 0,952 | 0,906 | 0,999 |
| IFI27, OAS1, IFIT1, SIGLEC1, ISG15, HERC6, SLC1A2, IL1R2, OLAH | 0,874 | 0,864 | 0,883 | 0,928 | 0,879 | 0,978 | 0,939 | 0,880 | 0,998 |
| OAS1, IFIT1, IFI44L, SIGLEC1, HERC6, SLC1A2, OLAH, FAM20A, MMP8 | 0,874 | 0,863 | 0,884 | 0,927 | 0,878 | 0,977 | 0,938 | 0,869 | 1 |
| IFI27, OAS1, SIGLEC1, ISG15, HERC6, SLC1A2, IL1R2, OLAH, MMP8 | 0,874 | 0,864 | 0,883 | 0,929 | 0,880 | 0,978 | 0,940 | 0,882 | 0,998 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, HERC6, IL1R2, OLAH, MMP8 | 0,874 | 0,864 | 0,883 | 0,925 | 0,875 | 0,975 | 0,941 | 0,887 | 0,996 |
| IFIT1, SIGLEC1, ISG15, HERC6, SLC1A2, IL1R2, OLAH, FAM20A, MMP8 | 0,873 | 0,864 | 0,883 | 0,928 | 0,882 | 0,975 | 0,960 | 0,896 | 1 |
| RSAD2, IFI27, OAS1, IFIT1, ISG15, IL1R2, FAM20A, RETN, MMP8 | 0,873 | 0,863 | 0,884 | 0,930 | 0,877 | 0,982 | 0,934 | 0,856 | 1 |
| IFI27, OAS1, IFI44L, SIGLEC1, ISG15, IL1R2, OLAH, RETN, MMP8 | 0,873 | 0,864 | 0,883 | 0,926 | 0,873 | 0,980 | 0,953 | 0,901 | 1 |
| RSAD2, IFIT1, IFI44L, SIGLEC1, HERC6, SLC1A2, OLAH, FAM20A, MMP8 | 0,873 | 0,863 | 0,884 | 0,929 | 0,880 | 0,977 | 0,934 | 0,862 | 1 |
| IFI27, OAS1, IFIT1, IFI44L, SIGLEC1, ISG15, IL1R2, OLAH, MMP8 | 0,873 | 0,865 | 0,882 | 0,925 | 0,875 | 0,975 | 0,941 | 0,888 | 0,994 |
| IFI27, IFIT1, SIGLEC1, HERC6, SLC1A2, IL1R2, OLAH, RETN, MMP8 | 0,873 | 0,864 | 0,883 | 0,930 | 0,881 | 0,980 | 0,957 | 0,908 | 1 |
| IFI27, OAS1, ISG15, HERC6, SLC1A2, IL1R2, FAM20A, RETN, MMP8 | 0,873 | 0,864 | 0,883 | 0,934 | 0,887 | 0,982 | 0,925 | 0,838 | 1 |
| RSAD2, IFI27, IFIT1, SIGLEC1, ISG15, HERC6, IL1R2, OLAH, MMP8 | 0,873 | 0,864 | 0,882 | 0,925 | 0,876 | 0,974 | 0,942 | 0,890 | 0,995 |
| RSAD2, IFI27, OAS1, IFIT1, IFI44L, HERC6, FAM20A, RETN, MMP8 | 0,873 | 0,863 | 0,884 | 0,923 | 0,865 | 0,982 | 0,935 | 0,854 | 1 |
| RSAD2, IFI27, OAS1, IFIT1, IFI44L, SIGLEC1, HERC6, SLC1A2, FAM20A | 0,873 | 0,864 | 0,883 | 0,930 | 0,881 | 0,979 | 0,936 | 0,861 | 1 |
| IFI27, OAS1, IFIT1, IFI44L, SLC1A2, IL1R2, OLAH, FAM20A, RETN | 0,873 | 0,863 | 0,883 | 0,929 | 0,875 | 0,983 | 0,921 | 0,839 | 1 |
| RSAD2, IFI27, SIGLEC1, ISG15, HERC6, SLC1A2, IL1R2, FAM20A, RETN | 0,873 | 0,863 | 0,884 | 0,933 | 0,886 | 0,981 | 0,943 | 0,873 | 1 |
| IFI27, IFIT1, IFI44L, SIGLEC1, ISG15, HERC6, SLC1A2, FAM20A, MMP8 | 0,873 | 0,864 | 0,883 | 0,927 | 0,876 | 0,977 | 0,938 | 0,860 | 1 |
| RSAD2, IFI27, SIGLEC1, ISG15, HERC6, SLC1A2, OLAH, RETN, MMP8 | 0,873 | 0,864 | 0,883 | 0,930 | 0,881 | 0,980 | 0,950 | 0,897 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, IL1R2, OLAH, MMP8 | 0,873 | 0,864 | 0,882 | 0,923 | 0,873 | 0,974 | 0,949 | 0,899 | 0,999 |
| RSAD2, IFIT1, IFI44L, SIGLEC1, HERC6, IL1R2, FAM20A, RETN, MMP8 | 0,873 | 0,864 | 0,883 | 0,925 | 0,870 | 0,980 | 0,962 | 0,905 | 1 |
| IFI27, OAS1, IFIT1, IFI44L, SIGLEC1, ISG15, HERC6, SLC1A2, FAM20A | 0,873 | 0,864 | 0,883 | 0,930 | 0,881 | 0,979 | 0,937 | 0,862 | 1 |
| IFI27, OAS1, IFI44L, HERC6, SLC1A2, IL1R2, FAM20A, RETN, MMP8 | 0,873 | 0,863 | 0,883 | 0,933 | 0,885 | 0,982 | 0,924 | 0,836 | 1 |
| IFI27, IFIT1, IFI44L, HERC6, SLC1A2, IL1R2, FAM20A, RETN, MMP8 | 0,873 | 0,864 | 0,883 | 0,932 | 0,883 | 0,981 | 0,932 | 0,849 | 1 |
| OAS1, IFIT1, SIGLEC1, ISG15, IL1R2, OLAH, FAM20A, RETN, MMP8 | 0,873 | 0,864 | 0,882 | 0,920 | 0,863 | 0,978 | 0,968 | 0,918 | 1 |
| RSAD2, IFI27, OAS1, HERC6, SLC1A2, IL1R2, OLAH, RETN, MMP8 | 0,873 | 0,863 | 0,883 | 0,930 | 0,879 | 0,981 | 0,941 | 0,877 | 1 |
| RSAD2, IFI27, OAS1, SIGLEC1, ISG15, HERC6, SLC1A2, OLAH, RETN | 0,873 | 0,864 | 0,883 | 0,930 | 0,880 | 0,980 | 0,945 | 0,883 | 1 |
| RSAD2, OAS1, IFIT1, SIGLEC1, HERC6, OLAH, FAM20A, RETN, MMP8 | 0,873 | 0,864 | 0,883 | 0,921 | 0,864 | 0,978 | 0,950 | 0,892 | 1 |
| RSAD2, IFI27, OAS1, IFIT1, HERC6, SLC1A2, OLAH, RETN, MMP8 | 0,873 | 0,863 | 0,883 | 0,927 | 0,876 | 0,979 | 0,940 | 0,875 | 1 |
| RSAD2, IFI27, OAS1, IFI44L, ISG15, SLC1A2, OLAH, FAM20A, RETN | 0,873 | 0,863 | 0,883 | 0,928 | 0,874 | 0,983 | 0,918 | 0,836 | 1 |
| RSAD2, IFI27, IFIT1, HERC6, SLC1A2, IL1R2, FAM20A, RETN, MMP8 | 0,873 | 0,863 | 0,883 | 0,933 | 0,884 | 0,982 | 0,933 | 0,852 | 1 |
| IFI27, OAS1, IFIT1, IFI44L, SIGLEC1, ISG15, OLAH, RETN, MMP8 | 0,873 | 0,864 | 0,882 | 0,920 | 0,862 | 0,978 | 0,952 | 0,896 | 1 |
| RSAD2, IFI27, OAS1, IFI44L, SIGLEC1, IL1R2, OLAH, RETN, MMP8 | 0,873 | 0,864 | 0,883 | 0,924 | 0,870 | 0,979 | 0,959 | 0,907 | 1 |
| RSAD2, IFI27, OAS1, IFIT1, SLC1A2, IL1R2, OLAH, FAM20A, RETN | 0,873 | 0,864 | 0,882 | 0,927 | 0,873 | 0,981 | 0,920 | 0,836 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, IL1R2, OLAH, RETN, MMP8 | 0,873 | 0,864 | 0,882 | 0,925 | 0,871 | 0,979 | 0,955 | 0,904 | 1 |
| RSAD2, IFI27, OAS1, IFIT1, SIGLEC1, ISG15, HERC6, SLC1A2, OLAH | 0,873 | 0,864 | 0,882 | 0,930 | 0,880 | 0,980 | 0,940 | 0,881 | 0,999 |
| IFI27, IFIT1, IFI44L, SIGLEC1, ISG15, SLC1A2, FAM20A, RETN, MMP8 | 0,873 | 0,862 | 0,884 | 0,927 | 0,872 | 0,982 | 0,942 | 0,865 | 1 |
| IFI27, IFI44L, SIGLEC1, ISG15, HERC6, SLC1A2, OLAH, RETN, MMP8 | 0,873 | 0,864 | 0,882 | 0,930 | 0,879 | 0,980 | 0,950 | 0,898 | 1 |
| RSAD2, IFI27, IFIT1, IFI44L, ISG15, IL1R2, FAM20A, RETN, MMP8 | 0,873 | 0,862 | 0,884 | 0,929 | 0,876 | 0,982 | 0,933 | 0,854 | 1 |
| RSAD2, IFI27, IFI44L, SIGLEC1, HERC6, SLC1A2, OLAH, RETN, MMP8 | 0,873 | 0,864 | 0,882 | 0,930 | 0,880 | 0,980 | 0,951 | 0,899 | 1 |
| RSAD2, IFI27, OAS1, ISG15, SLC1A2, OLAH, FAM20A, RETN, MMP8 | 0,873 | 0,862 | 0,884 | 0,928 | 0,874 | 0,982 | 0,922 | 0,841 | 1 |
| RSAD2, IFIT1, IFI44L, SIGLEC1, ISG15, IL1R2, OLAH, FAM20A, MMP8 | 0,873 | 0,864 | 0,882 | 0,919 | 0,862 | 0,976 | 0,967 | 0,916 | 1 |
| IFI27, OAS1, ISG15, HERC6, SLC1A2, IL1R2, OLAH, RETN, MMP8 | 0,873 | 0,863 | 0,883 | 0,929 | 0,878 | 0,979 | 0,938 | 0,874 | 1 |
| RSAD2, IFIT1, SIGLEC1, HERC6, SLC1A2, IL1R2, OLAH, FAM20A, MMP8 | 0,873 | 0,863 | 0,883 | 0,927 | 0,879 | 0,974 | 0,958 | 0,894 | 1 |
| RSAD2, IFI27, OAS1, IFIT1, SIGLEC1, IL1R2, OLAH, RETN, MMP8 | 0,873 | 0,863 | 0,883 | 0,922 | 0,865 | 0,978 | 0,962 | 0,913 | 1 |
| IFI27, OAS1, IFIT1, IFI44L, HERC6, SLC1A2, OLAH, RETN, MMP8 | 0,873 | 0,863 | 0,883 | 0,929 | 0,877 | 0,980 | 0,939 | 0,874 | 1 |
| RSAD2, IFI27, IFIT1, IFI44L, SIGLEC1, ISG15, HERC6, OLAH, MMP8 | 0,873 | 0,864 | 0,882 | 0,925 | 0,873 | 0,977 | 0,945 | 0,892 | 0,997 |
| IFI27, OAS1, IFI44L, SIGLEC1, ISG15, HERC6, SLC1A2, OLAH, RETN | 0,873 | 0,863 | 0,882 | 0,931 | 0,882 | 0,979 | 0,937 | 0,873 | 1 |
| RSAD2, IFI27, OAS1, IFIT1, IFI44L, SIGLEC1, ISG15, IL1R2, OLAH | 0,873 | 0,864 | 0,882 | 0,924 | 0,874 | 0,975 | 0,943 | 0,890 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, SIGLEC1, ISG15, HERC6, IL1R2, OLAH, FAM20A | 0,873 | 0,863 | 0,882 | 0,919 | 0,861 | 0,977 | 0,954 | 0,900 | 1 |
| IFI27, OAS1, IFIT1, SIGLEC1, HERC6, SLC1A2, IL1R2, OLAH, MMP8 | 0,873 | 0,863 | 0,883 | 0,929 | 0,881 | 0,977 | 0,942 | 0,885 | 0,999 |
| RSAD2, IFI27, IFI44L, SIGLEC1, HERC6, SLC1A2, FAM20A, RETN, MMP8 | 0,873 | 0,863 | 0,883 | 0,929 | 0,876 | 0,982 | 0,948 | 0,874 | 1 |
| IFI27, OAS1, IFIT1, ISG15, HERC6, SLC1A2, OLAH, RETN, MMP8 | 0,873 | 0,863 | 0,883 | 0,930 | 0,880 | 0,980 | 0,940 | 0,876 | 1 |
| OAS1, IFIT1, IFI44L, SIGLEC1, ISG15, HERC6, IL1R2, OLAH, FAM20A | 0,873 | 0,863 | 0,883 | 0,918 | 0,861 | 0,976 | 0,951 | 0,894 | 1 |
| IFIT1, IFI44L, SIGLEC1, ISG15, IL1R2, OLAH, FAM20A, RETN, MMP8 | 0,873 | 0,863 | 0,882 | 0,920 | 0,863 | 0,978 | 0,967 | 0,916 | 1 |
| IFIT1, IFI44L, SIGLEC1, ISG15, HERC6, SLC1A2, IL1R2, FAM20A, MMP8 | 0,873 | 0,863 | 0,882 | 0,927 | 0,881 | 0,973 | 0,959 | 0,891 | 1 |
| IFI27, IFI44L, SIGLEC1, HERC6, SLC1A2, IL1R2, OLAH, RETN, MMP8 | 0,873 | 0,863 | 0,882 | 0,929 | 0,879 | 0,979 | 0,955 | 0,906 | 1 |
| RSAD2, IFI27, IFIT1, IFI44L, ISG15, HERC6, IL1R2, OLAH, RETN | 0,873 | 0,864 | 0,882 | 0,923 | 0,870 | 0,977 | 0,937 | 0,878 | 0,996 |
| RSAD2, IFI27, OAS1, IFIT1, SIGLEC1, HERC6, SLC1A2, IL1R2, OLAH | 0,873 | 0,863 | 0,882 | 0,929 | 0,880 | 0,977 | 0,946 | 0,889 | 1 |
| RSAD2, IFI27, OAS1, IFI44L, SIGLEC1, ISG15, IL1R2, OLAH, MMP8 | 0,873 | 0,864 | 0,881 | 0,927 | 0,877 | 0,976 | 0,943 | 0,891 | 0,996 |
| IFIT1, IFI44L, SIGLEC1, HERC6, SLC1A2, OLAH, FAM20A, RETN, MMP8 | 0,873 | 0,862 | 0,883 | 0,930 | 0,881 | 0,979 | 0,948 | 0,883 | 1 |
| IFI27, OAS1, IFIT1, SIGLEC1, ISG15, HERC6, SLC1A2, RETN, MMP8 | 0,873 | 0,863 | 0,882 | 0,927 | 0,876 | 0,979 | 0,943 | 0,886 | 1 |
| RSAD2, OAS1, IFIT1, IFI44L, SIGLEC1, HERC6, OLAH, FAM20A, MMP8 | 0,873 | 0,863 | 0,883 | 0,917 | 0,859 | 0,975 | 0,949 | 0,891 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, SIGLEC1, HERC6, SLC1A2, OLAH, FAM20A, MMP8 | 0,873 | 0,862 | 0,883 | 0,926 | 0,876 | 0,975 | 0,943 | 0,874 | 1 |
| OAS1, IFIT1, IFI44L, SIGLEC1, IL1R2, OLAH, FAM20A, RETN, MMP8 | 0,873 | 0,863 | 0,882 | 0,922 | 0,865 | 0,978 | 0,967 | 0,916 | 1 |
| RSAD2, IFI27, OAS1, SIGLEC1, ISG15, HERC6, SLC1A2, OLAH, MMP8 | 0,873 | 0,863 | 0,882 | 0,931 | 0,881 | 0,981 | 0,940 | 0,881 | 0,999 |
| RSAD2, IFI27, IFIT1, IFI44L, HERC6, SLC1A2, IL1R2, FAM20A, MMP8 | 0,873 | 0,863 | 0,882 | 0,930 | 0,884 | 0,977 | 0,928 | 0,842 | 1 |
| RSAD2, IFI27, IFI44L, SIGLEC1, ISG15, IL1R2, OLAH, RETN, MMP8 | 0,873 | 0,864 | 0,882 | 0,927 | 0,874 | 0,980 | 0,954 | 0,904 | 1 |
| RSAD2, IFI27, OAS1, SIGLEC1, ISG15, HERC6, SLC1A2, IL1R2, OLAH | 0,873 | 0,863 | 0,882 | 0,929 | 0,880 | 0,978 | 0,942 | 0,884 | 1 |
| RSAD2, IFI27, OAS1, IFIT1, ISG15, HERC6, IL1R2, OLAH, MMP8 | 0,873 | 0,863 | 0,882 | 0,923 | 0,873 | 0,973 | 0,939 | 0,882 | 0,996 |
| RSAD2, OAS1, IFIT1, IFI44L, SIGLEC1, ISG15, HERC6, IL1R2, FAM20A | 0,873 | 0,864 | 0,882 | 0,919 | 0,862 | 0,976 | 0,955 | 0,901 | 1 |
| IFI27, OAS1, IFIT1, IFI44L, SIGLEC1, ISG15, IL1R2, OLAH, RETN | 0,873 | 0,864 | 0,881 | 0,920 | 0,865 | 0,975 | 0,950 | 0,898 | 1 |
| IFI27, IFIT1, IFI44L, SIGLEC1, HERC6, SLC1A2, OLAH, RETN, MMP8 | 0,873 | 0,863 | 0,882 | 0,929 | 0,878 | 0,980 | 0,953 | 0,904 | 1 |
| RSAD2, IFI27, IFIT1, IFI44L, SIGLEC1, HERC6, SLC1A2, FAM20A, MMP8 | 0,873 | 0,863 | 0,882 | 0,928 | 0,877 | 0,979 | 0,937 | 0,860 | 1 |
| IFI27, IFI44L, SIGLEC1, ISG15, SLC1A2, IL1R2, OLAH, RETN, MMP8 | 0,872 | 0,863 | 0,881 | 0,931 | 0,883 | 0,979 | 0,953 | 0,902 | 1 |
| RSAD2, IFI27, OAS1, IFI44L, SIGLEC1, ISG15, IL1R2, OLAH, RETN | 0,872 | 0,864 | 0,881 | 0,922 | 0,868 | 0,977 | 0,949 | 0,893 | 1 |
| RSAD2, IFI27, OAS1, IFIT1, IFI44L, ISG15, HERC6, OLAH, MMP8 | 0,872 | 0,863 | 0,882 | 0,925 | 0,874 | 0,977 | 0,936 | 0,877 | 0,994 |
| RSAD2, IFIT1, IFI44L, SIGLEC1, SLC1A2, IL1R2, OLAH, FAM20A, MMP8 | 0,872 | 0,863 | 0,882 | 0,927 | 0,878 | 0,975 | 0,959 | 0,904 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, HERC6, SLC1A2, IL1R2, FAM20A, MMP8 | 0,872 | 0,863 | 0,882 | 0,929 | 0,884 | 0,974 | 0,959 | 0,893 | 1 |
| OAS1, IFIT1, IFI44L, SIGLEC1, ISG15, SLC1A2, OLAH, FAM20A, MMP8 | 0,872 | 0,862 | 0,883 | 0,924 | 0,872 | 0,977 | 0,941 | 0,877 | 1 |
| RSAD2, IFI27, OAS1, SIGLEC1, ISG15, HERC6, SLC1A2, FAM20A, RETN | 0,872 | 0,862 | 0,883 | 0,926 | 0,872 | 0,979 | 0,937 | 0,863 | 1 |
| IFI27, OAS1, IFIT1, ISG15, HERC6, SLC1A2, IL1R2, OLAH, RETN | 0,872 | 0,863 | 0,882 | 0,927 | 0,876 | 0,978 | 0,932 | 0,864 | 0,999 |
| RSAD2, IFI27, IFIT1, IFI44L, SIGLEC1, HERC6, SLC1A2, OLAH, RETN | 0,872 | 0,863 | 0,881 | 0,929 | 0,879 | 0,978 | 0,943 | 0,888 | 0,999 |
| RSAD2, IFI27, OAS1, IFI44L, SLC1A2, OLAH, FAM20A, RETN, MMP8 | 0,872 | 0,861 | 0,883 | 0,929 | 0,874 | 0,983 | 0,927 | 0,851 | 1 |
| RSAD2, IFI27, OAS1, IFIT1, IFI44L, HERC6, IL1R2, RETN, MMP8 | 0,872 | 0,863 | 0,881 | 0,924 | 0,871 | 0,978 | 0,939 | 0,877 | 1 |
| RSAD2, IFI27, IFIT1, HERC6, SLC1A2, IL1R2, OLAH, RETN, MMP8 | 0,872 | 0,863 | 0,882 | 0,929 | 0,878 | 0,979 | 0,941 | 0,881 | 1 |
| IFI27, OAS1, IFIT1, IFI44L, SIGLEC1, HERC6, SLC1A2, OLAH, MMP8 | 0,872 | 0,862 | 0,882 | 0,927 | 0,877 | 0,977 | 0,941 | 0,884 | 0,999 |
| IFI27, IFIT1, SIGLEC1, ISG15, HERC6, SLC1A2, IL1R2, OLAH, RETN | 0,872 | 0,863 | 0,882 | 0,928 | 0,877 | 0,979 | 0,940 | 0,882 | 0,998 |
| IFI27, OAS1, SIGLEC1, ISG15, SLC1A2, IL1R2, OLAH, RETN, MMP8 | 0,872 | 0,863 | 0,882 | 0,930 | 0,881 | 0,979 | 0,953 | 0,898 | 1 |
| RSAD2, IFI27, IFIT1, IFI44L, SIGLEC1, ISG15, HERC6, IL1R2, RETN | 0,872 | 0,864 | 0,881 | 0,922 | 0,869 | 0,976 | 0,945 | 0,893 | 0,996 |
| RSAD2, IFI27, OAS1, IFIT1, IFI44L, SIGLEC1, HERC6, SLC1A2, RETN | 0,872 | 0,864 | 0,881 | 0,926 | 0,873 | 0,979 | 0,941 | 0,876 | 1 |
| RSAD2, IFI27, OAS1, IFIT1, SIGLEC1, SLC1A2, FAM20A, RETN, MMP8 | 0,872 | 0,862 | 0,882 | 0,927 | 0,872 | 0,982 | 0,943 | 0,869 | 1 |
| RSAD2, IFI27, OAS1, ISG15, HERC6, SLC1A2, IL1R2, OLAH, RETN | 0,872 | 0,863 | 0,882 | 0,928 | 0,877 | 0,979 | 0,932 | 0,860 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, ISG15, HERC6, FAM20A, RETN, MMP8 | 0,872 | 0,862 | 0,882 | 0,920 | 0,861 | 0,978 | 0,945 | 0,881 | 1 |
| RSAD2, IFI27, IFIT1, IFI44L, SLC1A2, IL1R2, OLAH, FAM20A, RETN | 0,872 | 0,862 | 0,882 | 0,927 | 0,872 | 0,981 | 0,922 | 0,841 | 1 |
| RSAD2, IFI27, IFI44L, SIGLEC1, ISG15, HERC6, IL1R2, RETN, MMP8 | 0,872 | 0,863 | 0,881 | 0,924 | 0,871 | 0,977 | 0,951 | 0,905 | 0,998 |
| RSAD2, IFI27, OAS1, IFI44L, SIGLEC1, ISG15, HERC6, SLC1A2, OLAH | 0,872 | 0,863 | 0,882 | 0,930 | 0,881 | 0,980 | 0,943 | 0,886 | 1 |
| RSAD2, IFI27, OAS1, IFI44L, SIGLEC1, SLC1A2, IL1R2, FAM20A, RETN | 0,872 | 0,862 | 0,883 | 0,930 | 0,881 | 0,979 | 0,943 | 0,873 | 1 |
| IFI27, OAS1, IFIT1, IFI44L, SIGLEC1, ISG15, HERC6, SLC1A2, OLAH | 0,872 | 0,862 | 0,882 | 0,929 | 0,879 | 0,979 | 0,939 | 0,880 | 0,998 |
| RSAD2, IFI27, SIGLEC1, ISG15, HERC6, SLC1A2, IL1R2, OLAH, MMP8 | 0,872 | 0,863 | 0,881 | 0,929 | 0,880 | 0,978 | 0,941 | 0,885 | 0,997 |
| RSAD2, IFI27, OAS1, IFIT1, IFI44L, ISG15, HERC6, IL1R2, RETN | 0,872 | 0,863 | 0,881 | 0,923 | 0,870 | 0,976 | 0,938 | 0,875 | 1 |
| RSAD2, IFIT1, IFI44L, SIGLEC1, ISG15, HERC6, IL1R2, OLAH, FAM20A | 0,872 | 0,863 | 0,882 | 0,918 | 0,860 | 0,976 | 0,951 | 0,894 | 1 |
| RSAD2, IFI27, OAS1, HERC6, SLC1A2, IL1R2, FAM20A, RETN, MMP8 | 0,872 | 0,862 | 0,882 | 0,935 | 0,888 | 0,982 | 0,925 | 0,839 | 1 |
| RSAD2, IFI27, IFIT1, SIGLEC1, ISG15, SLC1A2, FAM20A, RETN, MMP8 | 0,872 | 0,862 | 0,882 | 0,926 | 0,871 | 0,982 | 0,943 | 0,868 | 1 |
| RSAD2, IFI27, IFIT1, IFI44L, SIGLEC1, ISG15, IL1R2, OLAH, MMP8 | 0,872 | 0,864 | 0,881 | 0,925 | 0,875 | 0,975 | 0,943 | 0,891 | 0,996 |
| OAS1, IFIT1, IFI44L, SIGLEC1, SLC1A2, IL1R2, OLAH, FAM20A, MMP8 | 0,872 | 0,862 | 0,882 | 0,927 | 0,879 | 0,975 | 0,959 | 0,904 | 1 |
| RSAD2, IFI27, IFIT1, IFI44L, SIGLEC1, SLC1A2, FAM20A, RETN, MMP8 | 0,872 | 0,862 | 0,882 | 0,926 | 0,871 | 0,982 | 0,943 | 0,870 | 1 |
| IFI27, IFIT1, SIGLEC1, ISG15, HERC6, SLC1A2, OLAH, RETN, MMP8 | 0,872 | 0,862 | 0,882 | 0,930 | 0,880 | 0,981 | 0,952 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, SLC1A2, IL1R2, OLAH, RETN | 0,872 | 0,863 | 0,881 | 0,928 | 0,878 | 0,978 | 0,943 | 0,887 | 1 |
| RSAD2, OAS1, IFIT1, SIGLEC1, IL1R2, OLAH, FAM20A, RETN, MMP8 | 0,872 | 0,863 | 0,882 | 0,921 | 0,864 | 0,978 | 0,966 | 0,915 | 1 |
| RSAD2, IFI27, OAS1, IFIT1, ISG15, HERC6, IL1R2, RETN, MMP8 | 0,872 | 0,863 | 0,881 | 0,925 | 0,871 | 0,978 | 0,945 | 0,885 | 1 |
| RSAD2, IFI27, OAS1, IFIT1, IFI44L, ISG15, HERC6, IL1R2, OLAH | 0,872 | 0,863 | 0,881 | 0,923 | 0,873 | 0,974 | 0,936 | 0,878 | 0,994 |
| IFI27, OAS1, IFIT1, SIGLEC1, ISG15, SLC1A2, IL1R2, OLAH, RETN | 0,872 | 0,863 | 0,881 | 0,928 | 0,877 | 0,979 | 0,942 | 0,881 | 1 |
| IFIT1, IFI44L, SIGLEC1, ISG15, HERC6, IL1R2, OLAH, FAM20A, RETN | 0,872 | 0,863 | 0,881 | 0,918 | 0,860 | 0,976 | 0,951 | 0,894 | 1 |
| RSAD2, OAS1, SIGLEC1, HERC6, SLC1A2, IL1R2, OLAH, FAM20A, MMP8 | 0,872 | 0,862 | 0,882 | 0,924 | 0,876 | 0,972 | 0,960 | 0,900 | 1 |
| IFI27, IFIT1, IFI44L, SIGLEC1, ISG15, SLC1A2, IL1R2, OLAH, MMP8 | 0,872 | 0,863 | 0,881 | 0,928 | 0,879 | 0,977 | 0,943 | 0,890 | 0,997 |
| RSAD2, IFI27, OAS1, ISG15, HERC6, SLC1A2, OLAH, RETN, MMP8 | 0,872 | 0,862 | 0,882 | 0,930 | 0,879 | 0,981 | 0,940 | 0,875 | 1 |
| RSAD2, IFI27, OAS1, SIGLEC1, HERC6, SLC1A2, IL1R2, OLAH, MMP8 | 0,872 | 0,862 | 0,882 | 0,928 | 0,879 | 0,977 | 0,946 | 0,889 | 1 |
| RSAD2, IFI27, OAS1, IFIT1, SIGLEC1, HERC6, SLC1A2, OLAH, MMP8 | 0,872 | 0,862 | 0,882 | 0,928 | 0,878 | 0,978 | 0,940 | 0,881 | 0,999 |
| RSAD2, IFI27, IFIT1, SIGLEC1, ISG15, HERC6, IL1R2, RETN, MMP8 | 0,872 | 0,863 | 0,881 | 0,925 | 0,873 | 0,977 | 0,961 | 0,920 | 1 |
| IFI27, SIGLEC1, ISG15, HERC6, SLC1A2, IL1R2, OLAH, RETN, MMP8 | 0,872 | 0,862 | 0,881 | 0,929 | 0,878 | 0,979 | 0,951 | 0,899 | 1 |
| IFI27, OAS1, IFIT1, IFI44L, SIGLEC1, ISG15, HERC6, SLC1A2, RETN | 0,872 | 0,863 | 0,881 | 0,926 | 0,876 | 0,977 | 0,942 | 0,884 | 1 |
| IFIT1, SIGLEC1, HERC6, SLC1A2, IL1R2, OLAH, FAM20A, RETN, MMP8 | 0,872 | 0,862 | 0,882 | 0,928 | 0,881 | 0,976 | 0,959 | 0,893 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, IL1R2, OLAH, RETN | 0,872 | 0,863 | 0,881 | 0,920 | 0,864 | 0,975 | 0,953 | 0,897 | 1 |
| RSAD2, IFI27, OAS1, IFIT1, SIGLEC1, ISG15, OLAH, RETN, MMP8 | 0,872 | 0,862 | 0,881 | 0,920 | 0,861 | 0,979 | 0,959 | 0,907 | 1 |
| IFI27, OAS1, IFIT1, SIGLEC1, SLC1A2, IL1R2, OLAH, RETN, MMP8 | 0,872 | 0,862 | 0,882 | 0,928 | 0,878 | 0,978 | 0,955 | 0,900 | 1 |
| IFI27, OAS1, IFIT1, IFI44L, ISG15, HERC6, IL1R2, OLAH, MMP8 | 0,872 | 0,863 | 0,881 | 0,924 | 0,874 | 0,975 | 0,939 | 0,884 | 0,995 |
| RSAD2, OAS1, SIGLEC1, ISG15, HERC6, IL1R2, FAM20A, RETN, MMP8 | 0,872 | 0,862 | 0,881 | 0,920 | 0,863 | 0,977 | 0,958 | 0,896 | 1 |
| RSAD2, IFI27, OAS1, IFIT1, SIGLEC1, HERC6, SLC1A2, FAM20A, MMP8 | 0,872 | 0,862 | 0,881 | 0,929 | 0,880 | 0,978 | 0,937 | 0,862 | 1 |
| IFIT1, IFI44L, SIGLEC1, HERC6, SLC1A2, IL1R2, FAM20A, RETN, MMP8 | 0,872 | 0,862 | 0,881 | 0,930 | 0,883 | 0,976 | 0,960 | 0,895 | 1 |
| IFI27, OAS1, IFIT1, SIGLEC1, ISG15, SLC1A2, IL1R2, OLAH, MMP8 | 0,872 | 0,863 | 0,881 | 0,929 | 0,880 | 0,978 | 0,939 | 0,880 | 0,999 |
| OAS1, IFIT1, SIGLEC1, HERC6, SLC1A2, OLAH, FAM20A, RETN, MMP8 | 0,872 | 0,862 | 0,882 | 0,926 | 0,876 | 0,976 | 0,947 | 0,880 | 1 |
| RSAD2, OAS1, IFIT1, IFI44L, SIGLEC1, ISG15, IL1R2, FAM20A, MMP8 | 0,872 | 0,862 | 0,882 | 0,920 | 0,862 | 0,977 | 0,967 | 0,914 | 1 |
| RSAD2, IFI27, OAS1, SIGLEC1, ISG15, IL1R2, OLAH, RETN, MMP8 | 0,872 | 0,862 | 0,882 | 0,924 | 0,869 | 0,979 | 0,959 | 0,910 | 1 |
| RSAD2, IFIT1, IFI44L, SIGLEC1, ISG15, SLC1A2, OLAH, FAM20A, MMP8 | 0,872 | 0,862 | 0,882 | 0,925 | 0,873 | 0,977 | 0,940 | 0,875 | 1 |
| RSAD2, IFI27, IFI44L, HERC6, SLC1A2, IL1R2, FAM20A, RETN, MMP8 | 0,872 | 0,862 | 0,882 | 0,933 | 0,884 | 0,981 | 0,926 | 0,841 | 1 |
| IFI27, IFIT1, SIGLEC1, ISG15, HERC6, SLC1A2, IL1R2, OLAH, MMP8 | 0,872 | 0,862 | 0,881 | 0,929 | 0,880 | 0,978 | 0,940 | 0,884 | 0,997 |
| IFI27, OAS1, IFI44L, SIGLEC1, HERC6, SLC1A2, IL1R2, OLAH, RETN | 0,872 | 0,862 | 0,882 | 0,930 | 0,882 | 0,979 | 0,939 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, SIGLEC1, ISG15, HERC6, IL1R2, OLAH, FAM20A, RETN, MMP8 | 0,872 | 0,862 | 0,881 | 0,917 | 0,859 | 0,975 | 0,963 | 0,909 | 1 |
| OAS1, IFIT1, SIGLEC1, HERC6, SLC1A2, IL1R2, FAM20A, RETN, MMP8 | 0,872 | 0,862 | 0,882 | 0,929 | 0,882 | 0,975 | 0,955 | 0,890 | 1 |
| RSAD2, IFI27, OAS1, IFI44L, SIGLEC1, ISG15, OLAH, RETN, MMP8 | 0,872 | 0,863 | 0,881 | 0,920 | 0,862 | 0,978 | 0,959 | 0,908 | 1 |
| OAS1, IFI44L, SIGLEC1, ISG15, HERC6, IL1R2, OLAH, FAM20A, MMP8 | 0,872 | 0,862 | 0,882 | 0,918 | 0,861 | 0,975 | 0,962 | 0,906 | 1 |
| OAS1, IFIT1, SIGLEC1, ISG15, HERC6, SLC1A2, IL1R2, FAM20A, MMP8 | 0,872 | 0,862 | 0,882 | 0,929 | 0,883 | 0,974 | 0,959 | 0,893 | 1 |
| IFI27, IFIT1, IFI44L, SIGLEC1, ISG15, HERC6, SLC1A2, OLAH, RETN | 0,872 | 0,863 | 0,881 | 0,927 | 0,877 | 0,978 | 0,941 | 0,884 | 0,998 |
| IFI27, OAS1, IFIT1, SIGLEC1, ISG15, HERC6, SLC1A2, FAM20A, MMP8 | 0,872 | 0,862 | 0,882 | 0,930 | 0,881 | 0,978 | 0,936 | 0,860 | 1 |
| OAS1, IFIT1, IFI44L, SIGLEC1, ISG15, IL1R2, FAM20A, RETN, MMP8 | 0,872 | 0,862 | 0,881 | 0,923 | 0,866 | 0,979 | 0,967 | 0,914 | 1 |
| RSAD2, OAS1, IFIT1, SIGLEC1, HERC6, SLC1A2, IL1R2, FAM20A, MMP8 | 0,872 | 0,862 | 0,881 | 0,927 | 0,881 | 0,973 | 0,959 | 0,895 | 1 |
| OAS1, IFIT1, SIGLEC1, ISG15, HERC6, IL1R2, OLAH, FAM20A, RETN | 0,872 | 0,862 | 0,881 | 0,919 | 0,862 | 0,977 | 0,953 | 0,898 | 1 |
| IFI27, OAS1, IFIT1, IFI44L, SIGLEC1, HERC6, SLC1A2, IL1R2, RETN | 0,872 | 0,863 | 0,881 | 0,929 | 0,882 | 0,975 | 0,941 | 0,884 | 0,999 |
| RSAD2, OAS1, IFI44L, SIGLEC1, ISG15, HERC6, IL1R2, FAM20A, MMP8 | 0,872 | 0,862 | 0,881 | 0,918 | 0,860 | 0,975 | 0,960 | 0,900 | 1 |
| IFI27, OAS1, IFI44L, ISG15, HERC6, SLC1A2, IL1R2, FAM20A, MMP8 | 0,872 | 0,861 | 0,882 | 0,930 | 0,884 | 0,976 | 0,923 | 0,834 | 1 |
| RSAD2, IFI44L, SIGLEC1, ISG15, HERC6, IL1R2, OLAH, FAM20A, MMP8 | 0,872 | 0,862 | 0,881 | 0,918 | 0,862 | 0,975 | 0,961 | 0,903 | 1 |
| RSAD2, IFI27, IFIT1, IFI44L, HERC6, SLC1A2, OLAH, RETN, MMP8 | 0,872 | 0,862 | 0,881 | 0,929 | 0,877 | 0,980 | 0,941 | 0,881 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, HERC6, SLC1A2, IL1R2, OLAH, RETN | 0,872 | 0,862 | 0,881 | 0,926 | 0,877 | 0,976 | 0,943 | 0,887 | 1 |
| IFI27, IFI44L, ISG15, HERC6, SLC1A2, IL1R2, FAM20A, RETN, MMP8 | 0,872 | 0,862 | 0,882 | 0,931 | 0,882 | 0,980 | 0,929 | 0,843 | 1 |
| RSAD2, IFI27, IFIT1, IFI44L, SIGLEC1, ISG15, HERC6, SLC1A2, FAM20A | 0,872 | 0,862 | 0,881 | 0,927 | 0,875 | 0,978 | 0,937 | 0,860 | 1 |
| RSAD2, OAS1, IFIT1, IFI44L, SIGLEC1, SLC1A2, OLAH, FAM20A, MMP8 | 0,872 | 0,861 | 0,882 | 0,926 | 0,874 | 0,979 | 0,943 | 0,880 | 1 |
| RSAD2, IFI27, OAS1, IFI44L, HERC6, SLC1A2, IL1R2, FAM20A, MMP8 | 0,872 | 0,861 | 0,882 | 0,930 | 0,884 | 0,976 | 0,923 | 0,834 | 1 |
| IFI27, OAS1, IFI44L, ISG15, HERC6, SLC1A2, OLAH, RETN, MMP8 | 0,872 | 0,862 | 0,881 | 0,930 | 0,881 | 0,980 | 0,937 | 0,871 | 1 |
| IFI27, IFIT1, SIGLEC1, ISG15, SLC1A2, IL1R2, OLAH, RETN, MMP8 | 0,872 | 0,862 | 0,881 | 0,931 | 0,882 | 0,980 | 0,957 | 0,906 | 1 |
| RSAD2, IFI27, OAS1, IFIT1, IFI44L, SIGLEC1, HERC6, IL1R2, MMP8 | 0,872 | 0,863 | 0,880 | 0,924 | 0,876 | 0,971 | 0,945 | 0,894 | 0,995 |
| RSAD2, OAS1, IFIT1, SIGLEC1, SLC1A2, IL1R2, OLAH, FAM20A, MMP8 | 0,872 | 0,862 | 0,881 | 0,926 | 0,878 | 0,975 | 0,963 | 0,907 | 1 |
| IFI44L, SIGLEC1, ISG15, HERC6, SLC1A2, IL1R2, OLAH, FAM20A, MMP8 | 0,872 | 0,862 | 0,881 | 0,924 | 0,876 | 0,973 | 0,959 | 0,895 | 1 |
| RSAD2, IFI27, IFIT1, SIGLEC1, ISG15, IL1R2, OLAH, RETN, MMP8 | 0,872 | 0,862 | 0,881 | 0,922 | 0,866 | 0,977 | 0,958 | 0,908 | 1 |
| RSAD2, IFI27, OAS1, IFI44L, SIGLEC1, HERC6, SLC1A2, FAM20A, RETN | 0,872 | 0,861 | 0,882 | 0,928 | 0,875 | 0,980 | 0,937 | 0,861 | 1 |
| RSAD2, IFI27, IFIT1, SIGLEC1, HERC6, SLC1A2, IL1R2, OLAH, RETN | 0,872 | 0,862 | 0,881 | 0,927 | 0,877 | 0,978 | 0,945 | 0,889 | 1 |
| RSAD2, IFI27, OAS1, IFIT1, IFI44L, SLC1A2, OLAH, FAM20A, RETN | 0,872 | 0,862 | 0,881 | 0,927 | 0,871 | 0,984 | 0,924 | 0,845 | 1 |
| RSAD2, IFI27, OAS1, IFIT1, SIGLEC1, HERC6, SLC1A2, IL1R2, RETN | 0,872 | 0,863 | 0,880 | 0,927 | 0,878 | 0,976 | 0,947 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, SIGLEC1, ISG15, HERC6, SLC1A2, IL1R2, OLAH, RETN | 0,872 | 0,862 | 0,881 | 0,928 | 0,878 | 0,978 | 0,940 | 0,882 | 0,998 |
| RSAD2, OAS1, SIGLEC1, HERC6, IL1R2, OLAH, FAM20A, RETN, MMP8 | 0,872 | 0,862 | 0,882 | 0,920 | 0,864 | 0,977 | 0,960 | 0,902 | 1 |
| RSAD2, IFI27, OAS1, IFI44L, HERC6, SLC1A2, OLAH, RETN, MMP8 | 0,872 | 0,862 | 0,882 | 0,929 | 0,879 | 0,980 | 0,941 | 0,877 | 1 |
| RSAD2, IFI27, OAS1, IFIT1, IFI44L, SIGLEC1, ISG15, OLAH, RETN | 0,872 | 0,863 | 0,880 | 0,918 | 0,860 | 0,977 | 0,945 | 0,883 | 1 |
| RSAD2, IFI27, OAS1, IFIT1, HERC6, SLC1A2, IL1R2, OLAH, RETN | 0,871 | 0,862 | 0,881 | 0,927 | 0,876 | 0,979 | 0,932 | 0,860 | 1 |
| IFI27, OAS1, IFIT1, IFI44L, SIGLEC1, ISG15, SLC1A2, OLAH, MMP8 | 0,871 | 0,862 | 0,881 | 0,930 | 0,880 | 0,980 | 0,943 | 0,886 | 1 |
| IFI27, OAS1, SIGLEC1, ISG15, HERC6, SLC1A2, IL1R2, RETN, MMP8 | 0,871 | 0,862 | 0,880 | 0,928 | 0,881 | 0,976 | 0,957 | 0,905 | 1 |
| RSAD2, IFI27, OAS1, IFIT1, SIGLEC1, ISG15, HERC6, SLC1A2, RETN | 0,871 | 0,863 | 0,880 | 0,924 | 0,870 | 0,978 | 0,939 | 0,876 | 1 |
| IFI27, OAS1, IFIT1, ISG15, HERC6, SLC1A2, IL1R2, FAM20A, RETN | 0,871 | 0,861 | 0,882 | 0,934 | 0,887 | 0,981 | 0,926 | 0,842 | 1 |
| OAS1, IFIT1, SIGLEC1, ISG15, HERC6, SLC1A2, OLAH, FAM20A, MMP8 | 0,871 | 0,861 | 0,882 | 0,926 | 0,877 | 0,975 | 0,943 | 0,873 | 1 |
| IFI27, OAS1, IFI44L, SIGLEC1, ISG15, HERC6, SLC1A2, OLAH, MMP8 | 0,871 | 0,861 | 0,881 | 0,930 | 0,880 | 0,979 | 0,939 | 0,881 | 0,997 |
| RSAD2, IFI27, IFIT1, IFI44L, SIGLEC1, ISG15, OLAH, RETN, MMP8 | 0,871 | 0,862 | 0,881 | 0,920 | 0,862 | 0,978 | 0,953 | 0,898 | 1 |
| RSAD2, IFI27, OAS1, IFIT1, SIGLEC1, ISG15, SLC1A2, OLAH, MMP8 | 0,871 | 0,862 | 0,881 | 0,929 | 0,878 | 0,980 | 0,943 | 0,886 | 1 |
| RSAD2, IFI27, IFIT1, IFI44L, SIGLEC1, ISG15, SLC1A2, FAM20A, RETN | 0,871 | 0,862 | 0,881 | 0,926 | 0,871 | 0,981 | 0,937 | 0,861 | 1 |
| RSAD2, IFI27, OAS1, IFIT1, HERC6, SLC1A2, IL1R2, FAM20A, MMP8 | 0,871 | 0,861 | 0,882 | 0,930 | 0,884 | 0,976 | 0,927 | 0,842 | 1 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, ISG15, HERC6, IL1R2, OLAH, FAM20A, RETN, MMP8 | 0,871 | 0,861 | 0,881 | 0,917 | 0,859 | 0,975 | 0,960 | 0,900 | 1 |
| RSAD2, IFI27, OAS1, IFIT1, ISG15, SLC1A2, OLAH, FAM20A, RETN | 0,871 | 0,861 | 0,882 | 0,925 | 0,870 | 0,980 | 0,923 | 0,843 | 1 |
| IFI27, IFI44L, SIGLEC1, ISG15, HERC6, SLC1A2, IL1R2, OLAH, MMP8 | 0,871 | 0,862 | 0,881 | 0,930 | 0,881 | 0,978 | 0,943 | 0,890 | 0,997 |
| IFI27, OAS1, IFI44L, ISG15, HERC6, SLC1A2, IL1R2, FAM20A, RETN | 0,871 | 0,861 | 0,882 | 0,932 | 0,885 | 0,980 | 0,925 | 0,840 | 1 |
| IFI27, OAS1, IFIT1, IFI44L, HERC6, SLC1A2, IL1R2, FAM20A, RETN | 0,871 | 0,861 | 0,881 | 0,932 | 0,885 | 0,980 | 0,925 | 0,840 | 1 |
| RSAD2, IFI27, OAS1, IFIT1, IFI44L, IL1R2, OLAH, RETN, MMP8 | 0,871 | 0,862 | 0,881 | 0,922 | 0,867 | 0,977 | 0,950 | 0,895 | 1 |
| RSAD2, IFI27, ISG15, HERC6, SLC1A2, IL1R2, FAM20A, RETN, MMP8 | 0,871 | 0,862 | 0,881 | 0,933 | 0,885 | 0,981 | 0,929 | 0,844 | 1 |
| IFI27, OAS1, IFIT1, SIGLEC1, ISG15, HERC6, SLC1A2, IL1R2, RETN | 0,871 | 0,862 | 0,881 | 0,928 | 0,881 | 0,975 | 0,948 | 0,892 | 1 |
| RSAD2, OAS1, IFIT1, SIGLEC1, ISG15, HERC6, OLAH, FAM20A, MMP8 | 0,871 | 0,861 | 0,882 | 0,916 | 0,858 | 0,975 | 0,952 | 0,896 | 1 |
| IFIT1, SIGLEC1, ISG15, HERC6, SLC1A2, IL1R2, FAM20A, RETN, MMP8 | 0,871 | 0,862 | 0,881 | 0,926 | 0,878 | 0,974 | 0,957 | 0,889 | 1 |
| RSAD2, IFI27, OAS1, IFI44L, SIGLEC1, HERC6, SLC1A2, OLAH, MMP8 | 0,871 | 0,862 | 0,881 | 0,929 | 0,879 | 0,979 | 0,947 | 0,892 | 1 |
| IFI27, OAS1, IFI44L, SIGLEC1, HERC6, SLC1A2, IL1R2, OLAH, MMP8 | 0,871 | 0,862 | 0,881 | 0,927 | 0,879 | 0,975 | 0,943 | 0,888 | 0,999 |
| RSAD2, IFI27, OAS1, IFI44L, SIGLEC1, HERC6, SLC1A2, IL1R2, RETN | 0,871 | 0,863 | 0,880 | 0,927 | 0,879 | 0,976 | 0,946 | 0,884 | 1 |
| RSAD2, IFI27, IFIT1, SIGLEC1, ISG15, HERC6, SLC1A2, OLAH, RETN | 0,871 | 0,862 | 0,881 | 0,929 | 0,879 | 0,978 | 0,941 | 0,884 | 0,999 |
| RSAD2, IFI27, OAS1, IFI44L, SIGLEC1, HERC6, SLC1A2, IL1R2, OLAH | 0,871 | 0,862 | 0,881 | 0,928 | 0,880 | 0,976 | 0,950 | 0,897 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, HERC6, SLC1A2, IL1R2, OLAH, RETN, MMP8 | 0,871 | 0,862 | 0,881 | 0,928 | 0,877 | 0,979 | 0,941 | 0,881 | 1 |
| RSAD2, IFI27, IFI44L, SIGLEC1, ISG15, HERC6, SLC1A2, OLAH, RETN | 0,871 | 0,862 | 0,880 | 0,927 | 0,876 | 0,978 | 0,943 | 0,887 | 1 |
| RSAD2, IFIT1, IFI44L, SIGLEC1, HERC6, OLAH, FAM20A, RETN, MMP8 | 0,871 | 0,862 | 0,881 | 0,921 | 0,863 | 0,979 | 0,949 | 0,890 | 1 |
| IFI27, IFIT1, IFI44L, SIGLEC1, ISG15, HERC6, SLC1A2, OLAH, MMP8 | 0,871 | 0,862 | 0,881 | 0,930 | 0,880 | 0,979 | 0,940 | 0,882 | 0,998 |
| IFI27, OAS1, IFIT1, IFI44L, ISG15, HERC6, SLC1A2, IL1R2, FAM20A | 0,871 | 0,861 | 0,881 | 0,931 | 0,886 | 0,977 | 0,926 | 0,840 | 1 |
| RSAD2, IFI27, OAS1, IFIT1, IFI44L, SIGLEC1, HERC6, SLC1A2, OLAH | 0,871 | 0,862 | 0,881 | 0,929 | 0,878 | 0,979 | 0,947 | 0,892 | 1 |
| RSAD2, IFI27, IFIT1, IFI44L, ISG15, HERC6, IL1R2, OLAH, MMP8 | 0,871 | 0,862 | 0,880 | 0,925 | 0,875 | 0,975 | 0,937 | 0,880 | 0,993 |
| RSAD2, IFI27, ISG15, HERC6, SLC1A2, IL1R2, OLAH, RETN, MMP8 | 0,871 | 0,862 | 0,881 | 0,928 | 0,878 | 0,979 | 0,940 | 0,879 | 1 |
| RSAD2, IFI27, OAS1, IFIT1, SIGLEC1, HERC6, SLC1A2, RETN, MMP8 | 0,871 | 0,862 | 0,880 | 0,924 | 0,870 | 0,979 | 0,942 | 0,880 | 1 |
| RSAD2, IFI27, OAS1, SIGLEC1, HERC6, SLC1A2, IL1R2, RETN, MMP8 | 0,871 | 0,862 | 0,881 | 0,927 | 0,879 | 0,976 | 0,960 | 0,909 | 1 |
| RSAD2, IFI27, OAS1, IFIT1, SIGLEC1, ISG15, SLC1A2, IL1R2, OLAH | 0,871 | 0,862 | 0,880 | 0,929 | 0,879 | 0,979 | 0,942 | 0,884 | 1 |
| RSAD2, IFIT1, SIGLEC1, ISG15, HERC6, SLC1A2, IL1R2, FAM20A, MMP8 | 0,871 | 0,862 | 0,881 | 0,927 | 0,881 | 0,973 | 0,958 | 0,890 | 1 |
| IFI27, OAS1, IFI44L, SIGLEC1, HERC6, SLC1A2, IL1R2, RETN, MMP8 | 0,871 | 0,862 | 0,880 | 0,928 | 0,881 | 0,975 | 0,955 | 0,906 | 1 |
| RSAD2, IFI27, IFI44L, SIGLEC1, HERC6, SLC1A2, IL1R2, OLAH, MMP8 | 0,871 | 0,862 | 0,880 | 0,928 | 0,879 | 0,976 | 0,949 | 0,898 | 0,999 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, IL1R2, RETN, MMP8 | 0,871 | 0,862 | 0,880 | 0,924 | 0,871 | 0,977 | 0,940 | 0,880 | 1 |
| RSAD2, IFIT1, SIGLEC1, ISG15, HERC6, OLAH, FAM20A, RETN, MMP8 | 0,871 | 0,862 | 0,881 | 0,921 | 0,863 | 0,978 | 0,952 | 0,896 | 1 |
| RSAD2, IFI27, OAS1, IFI44L, SLC1A2, IL1R2, OLAH, FAM20A, RETN | 0,871 | 0,861 | 0,881 | 0,929 | 0,875 | 0,984 | 0,921 | 0,838 | 1 |
| IFIT1, SIGLEC1, ISG15, HERC6, SLC1A2, OLAH, FAM20A, RETN, MMP8 | 0,871 | 0,861 | 0,881 | 0,928 | 0,878 | 0,977 | 0,950 | 0,883 | 1 |
| IFI27, OAS1, IFIT1, ISG15, HERC6, SLC1A2, IL1R2, OLAH, MMP8 | 0,871 | 0,861 | 0,881 | 0,928 | 0,879 | 0,977 | 0,929 | 0,862 | 0,996 |
| IFI27, IFIT1, IFI44L, SIGLEC1, ISG15, SLC1A2, OLAH, RETN, MMP8 | 0,871 | 0,862 | 0,880 | 0,930 | 0,879 | 0,980 | 0,952 | 0,897 | 1 |
| IFI27, IFIT1, IFI44L, SIGLEC1, HERC6, SLC1A2, IL1R2, OLAH, RETN | 0,871 | 0,862 | 0,881 | 0,927 | 0,878 | 0,977 | 0,945 | 0,889 | 1 |
| RSAD2, IFI27, IFIT1, IFI44L, SIGLEC1, ISG15, IL1R2, OLAH, RETN | 0,871 | 0,863 | 0,880 | 0,922 | 0,867 | 0,977 | 0,950 | 0,898 | 1 |
| IFI27, IFIT1, IFI44L, ISG15, SLC1A2, IL1R2, FAM20A, RETN, MMP8 | 0,871 | 0,861 | 0,881 | 0,931 | 0,881 | 0,980 | 0,933 | 0,851 | 1 |
| RSAD2, IFI27, IFIT1, SIGLEC1, SLC1A2, IL1R2, OLAH, RETN, MMP8 | 0,871 | 0,862 | 0,881 | 0,930 | 0,881 | 0,979 | 0,959 | 0,908 | 1 |
| RSAD2, IFI27, OAS1, IFIT1, IFI44L, SIGLEC1, ISG15, FAM20A, MMP8 | 0,871 | 0,861 | 0,881 | 0,915 | 0,851 | 0,980 | 0,943 | 0,873 | 1 |
| OAS1, IFIT1, IFI44L, SIGLEC1, HERC6, IL1R2, OLAH, FAM20A, RETN | 0,871 | 0,862 | 0,880 | 0,916 | 0,858 | 0,975 | 0,951 | 0,894 | 1 |
| RSAD2, IFI27, OAS1, IFIT1, IFI44L, SIGLEC1, ISG15, OLAH, MMP8 | 0,871 | 0,862 | 0,880 | 0,920 | 0,863 | 0,976 | 0,939 | 0,882 | 0,997 |
| RSAD2, SIGLEC1, ISG15, HERC6, IL1R2, OLAH, FAM20A, RETN, MMP8 | 0,871 | 0,861 | 0,881 | 0,919 | 0,862 | 0,976 | 0,961 | 0,905 | 1 |
| IFI27, OAS1, IFIT1, IFI44L, SIGLEC1, HERC6, SLC1A2, RETN, MMP8 | 0,871 | 0,862 | 0,880 | 0,927 | 0,876 | 0,978 | 0,945 | 0,887 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, SLC1A2, IL1R2, OLAH, FAM20A, MMP8 | 0,871 | 0,862 | 0,880 | 0,926 | 0,878 | 0,974 | 0,962 | 0,900 | 1 |
| RSAD2, IFI27, OAS1, IFI44L, ISG15, IL1R2, FAM20A, RETN, MMP8 | 0,871 | 0,860 | 0,882 | 0,930 | 0,878 | 0,982 | 0,935 | 0,856 | 1 |
| RSAD2, OAS1, IFIT1, SIGLEC1, ISG15, IL1R2, FAM20A, RETN, MMP8 | 0,871 | 0,862 | 0,880 | 0,922 | 0,865 | 0,979 | 0,967 | 0,914 | 1 |
| RSAD2, IFI27, OAS1, IFIT1, IFI44L, ISG15, IL1R2, FAM20A, RETN | 0,871 | 0,861 | 0,881 | 0,924 | 0,871 | 0,977 | 0,930 | 0,852 | 1 |
| RSAD2, IFI27, OAS1, IFIT1, SIGLEC1, ISG15, HERC6, SLC1A2, FAM20A | 0,871 | 0,862 | 0,880 | 0,928 | 0,879 | 0,977 | 0,936 | 0,859 | 1 |
| IFI27, OAS1, IFIT1, IFI44L, SIGLEC1, SLC1A2, OLAH, RETN, MMP8 | 0,871 | 0,861 | 0,881 | 0,927 | 0,875 | 0,979 | 0,957 | 0,901 | 1 |
| RSAD2, IFI27, OAS1, IFIT1, SIGLEC1, ISG15, SLC1A2, FAM20A, RETN | 0,871 | 0,861 | 0,880 | 0,925 | 0,869 | 0,980 | 0,938 | 0,863 | 1 |
| RSAD2, SIGLEC1, ISG15, HERC6, SLC1A2, IL1R2, OLAH, FAM20A, MMP8 | 0,871 | 0,861 | 0,881 | 0,925 | 0,877 | 0,973 | 0,961 | 0,901 | 1 |
| RSAD2, IFI27, IFIT1, ISG15, HERC6, SLC1A2, OLAH, RETN, MMP8 | 0,871 | 0,861 | 0,881 | 0,929 | 0,879 | 0,979 | 0,941 | 0,881 | 1 |
| RSAD2, IFI27, OAS1, IFI44L, ISG15, HERC6, IL1R2, RETN, MMP8 | 0,871 | 0,862 | 0,880 | 0,923 | 0,869 | 0,976 | 0,940 | 0,877 | 1 |
| OAS1, IFIT1, SIGLEC1, ISG15, SLC1A2, IL1R2, OLAH, FAM20A, MMP8 | 0,871 | 0,861 | 0,880 | 0,926 | 0,879 | 0,974 | 0,961 | 0,899 | 1 |
| RSAD2, IFI27, OAS1, SIGLEC1, ISG15, SLC1A2, IL1R2, OLAH, RETN | 0,871 | 0,862 | 0,880 | 0,930 | 0,879 | 0,981 | 0,948 | 0,885 | 1 |
| RSAD2, IFI27, IFIT1, SIGLEC1, ISG15, HERC6, SLC1A2, IL1R2, OLAH | 0,871 | 0,862 | 0,880 | 0,929 | 0,880 | 0,978 | 0,940 | 0,883 | 0,998 |
| RSAD2, IFI27, OAS1, IFIT1, IFI44L, HERC6, SLC1A2, IL1R2, FAM20A | 0,871 | 0,861 | 0,880 | 0,930 | 0,884 | 0,976 | 0,926 | 0,840 | 1 |
| IFI27, IFI44L, ISG15, HERC6, SLC1A2, IL1R2, OLAH, RETN, MMP8 | 0,871 | 0,861 | 0,880 | 0,929 | 0,879 | 0,979 | 0,938 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, SLC1A2, IL1R2, OLAH, MMP8 | 0,871 | 0,862 | 0,879 | 0,929 | 0,879 | 0,978 | 0,945 | 0,891 | 0,998 |
| RSAD2, IFI27, OAS1, IFIT1, IFI44L, SIGLEC1, SLC1A2, FAM20A, RETN | 0,871 | 0,861 | 0,881 | 0,927 | 0,872 | 0,981 | 0,940 | 0,868 | 1 |
| RSAD2, IFI27, OAS1, IFIT1, IFI44L, ISG15, HERC6, RETN, MMP8 | 0,871 | 0,861 | 0,880 | 0,918 | 0,859 | 0,978 | 0,930 | 0,861 | 1 |
| RSAD2, IFI27, OAS1, IFIT1, SIGLEC1, SLC1A2, IL1R2, OLAH, RETN | 0,871 | 0,862 | 0,880 | 0,929 | 0,878 | 0,979 | 0,949 | 0,887 | 1 |
| RSAD2, IFI27, IFIT1, SIGLEC1, HERC6, SLC1A2, IL1R2, OLAH, MMP8 | 0,871 | 0,861 | 0,880 | 0,928 | 0,879 | 0,977 | 0,943 | 0,889 | 0,998 |
| RSAD2, IFI27, OAS1, IFIT1, IFI44L, HERC6, SLC1A2, OLAH, RETN | 0,871 | 0,861 | 0,880 | 0,927 | 0,875 | 0,978 | 0,934 | 0,862 | 1 |
| IFI27, OAS1, IFIT1, IFI44L, ISG15, IL1R2, OLAH, RETN, MMP8 | 0,871 | 0,861 | 0,880 | 0,918 | 0,861 | 0,976 | 0,948 | 0,894 | 1 |
| RSAD2, IFI27, OAS1, IFI44L, SIGLEC1, ISG15, SLC1A2, IL1R2, OLAH | 0,871 | 0,862 | 0,880 | 0,929 | 0,879 | 0,980 | 0,947 | 0,891 | 1 |
| RSAD2, IFI27, IFI44L, ISG15, HERC6, SLC1A2, OLAH, RETN, MMP8 | 0,871 | 0,861 | 0,880 | 0,928 | 0,878 | 0,979 | 0,941 | 0,881 | 1 |
| RSAD2, IFIT1, IFI44L, SIGLEC1, ISG15, HERC6, OLAH, FAM20A, MMP8 | 0,871 | 0,860 | 0,881 | 0,917 | 0,859 | 0,975 | 0,949 | 0,891 | 1 |
| IFI27, OAS1, IFIT1, SIGLEC1, ISG15, SLC1A2, OLAH, RETN, MMP8 | 0,871 | 0,860 | 0,881 | 0,929 | 0,878 | 0,981 | 0,954 | 0,897 | 1 |
| RSAD2, IFI27, OAS1, IFIT1, IFI44L, SIGLEC1, SLC1A2, OLAH, RETN | 0,871 | 0,862 | 0,879 | 0,926 | 0,873 | 0,979 | 0,950 | 0,887 | 1 |
| RSAD2, IFI27, IFI44L, SIGLEC1, SLC1A2, IL1R2, OLAH, RETN, MMP8 | 0,871 | 0,861 | 0,880 | 0,930 | 0,881 | 0,980 | 0,959 | 0,910 | 1 |
| IFI27, OAS1, IFI44L, SIGLEC1, ISG15, HERC6, SLC1A2, IL1R2, OLAH | 0,871 | 0,861 | 0,881 | 0,929 | 0,881 | 0,978 | 0,939 | 0,882 | 0,996 |
| RSAD2, IFI27, OAS1, IFI44L, SIGLEC1, ISG15, SLC1A2, OLAH, MMP8 | 0,871 | 0,862 | 0,880 | 0,930 | 0,880 | 0,981 | 0,950 | 0,896 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, HERC6, SLC1A2, OLAH, FAM20A, MMP8 | 0,871 | 0,860 | 0,881 | 0,926 | 0,877 | 0,975 | 0,945 | 0,874 | 1 |
| OAS1, SIGLEC1, ISG15, HERC6, SLC1A2, IL1R2, OLAH, FAM20A, MMP8 | 0,871 | 0,860 | 0,881 | 0,922 | 0,873 | 0,971 | 0,961 | 0,901 | 1 |
| RSAD2, IFI27, OAS1, IFIT1, ISG15, HERC6, SLC1A2, OLAH, RETN | 0,871 | 0,861 | 0,880 | 0,928 | 0,877 | 0,979 | 0,930 | 0,858 | 1 |
| RSAD2, IFI27, IFI44L, SIGLEC1, ISG15, HERC6, SLC1A2, OLAH, MMP8 | 0,871 | 0,861 | 0,880 | 0,931 | 0,881 | 0,980 | 0,940 | 0,883 | 0,998 |
| IFI27, OAS1, IFI44L, HERC6, SLC1A2, IL1R2, OLAH, RETN, MMP8 | 0,871 | 0,860 | 0,881 | 0,930 | 0,881 | 0,980 | 0,938 | 0,872 | 1 |
| RSAD2, IFI27, SIGLEC1, ISG15, SLC1A2, IL1R2, OLAH, RETN, MMP8 | 0,871 | 0,861 | 0,880 | 0,930 | 0,880 | 0,979 | 0,953 | 0,900 | 1 |
| IFI27, OAS1, IFI44L, SIGLEC1, ISG15, HERC6, SLC1A2, IL1R2, RETN | 0,871 | 0,862 | 0,879 | 0,928 | 0,881 | 0,975 | 0,940 | 0,881 | 0,999 |
| RSAD2, IFI27, OAS1, IFI44L, ISG15, HERC6, SLC1A2, OLAH, RETN | 0,871 | 0,861 | 0,880 | 0,929 | 0,879 | 0,979 | 0,933 | 0,861 | 1 |
| IFIT1, IFI44L, SIGLEC1, ISG15, HERC6, SLC1A2, IL1R2, OLAH, FAM20A | 0,871 | 0,861 | 0,880 | 0,925 | 0,876 | 0,975 | 0,937 | 0,867 | 1 |
| RSAD2, IFI27, OAS1, IFI44L, ISG15, HERC6, SLC1A2, IL1R2, FAM20A | 0,871 | 0,860 | 0,881 | 0,930 | 0,884 | 0,975 | 0,926 | 0,839 | 1 |
| IFI27, OAS1, IFIT1, IFI44L, SIGLEC1, ISG15, SLC1A2, OLAH, RETN | 0,871 | 0,862 | 0,880 | 0,930 | 0,879 | 0,980 | 0,946 | 0,885 | 1 |
| IFI27, OAS1, IFIT1, ISG15, HERC6, SLC1A2, IL1R2, FAM20A, MMP8 | 0,871 | 0,860 | 0,881 | 0,930 | 0,884 | 0,976 | 0,927 | 0,842 | 1 |
| IFI27, OAS1, IFIT1, IFI44L, SIGLEC1, HERC6, SLC1A2, IL1R2, OLAH | 0,871 | 0,861 | 0,880 | 0,928 | 0,880 | 0,976 | 0,946 | 0,890 | 1 |
| RSAD2, IFI27, OAS1, IFIT1, SIGLEC1, ISG15, IL1R2, OLAH, MMP8 | 0,871 | 0,861 | 0,880 | 0,923 | 0,872 | 0,974 | 0,945 | 0,894 | 0,995 |
| OAS1, IFIT1, IFI44L, SIGLEC1, ISG15, HERC6, IL1R2, FAM20A, RETN | 0,871 | 0,861 | 0,880 | 0,919 | 0,862 | 0,976 | 0,955 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, SLC1A2, IL1R2, OLAH, MMP8 | 0,871 | 0,861 | 0,880 | 0,926 | 0,875 | 0,976 | 0,951 | 0,897 | 1 |
| IFI27, OAS1, IFIT1, IFI44L, SLC1A2, IL1R2, FAM20A, RETN, MMP8 | 0,870 | 0,861 | 0,880 | 0,930 | 0,880 | 0,979 | 0,927 | 0,844 | 1 |
| RSAD2, IFI27, IFI44L, SIGLEC1, ISG15, HERC6, SLC1A2, IL1R2, OLAH | 0,870 | 0,861 | 0,880 | 0,928 | 0,878 | 0,978 | 0,943 | 0,890 | 0,997 |
| RSAD2, IFI27, OAS1, SIGLEC1, SLC1A2, IL1R2, OLAH, RETN, MMP8 | 0,870 | 0,861 | 0,880 | 0,929 | 0,879 | 0,979 | 0,954 | 0,898 | 1 |
| RSAD2, IFI27, IFIT1, SIGLEC1, ISG15, SLC1A2, IL1R2, OLAH, MMP8 | 0,870 | 0,861 | 0,879 | 0,929 | 0,880 | 0,978 | 0,942 | 0,887 | 0,998 |
| IFI27, OAS1, IFIT1, IFI44L, SIGLEC1, ISG15, IL1R2, RETN, MMP8 | 0,870 | 0,861 | 0,880 | 0,925 | 0,873 | 0,977 | 0,958 | 0,913 | 1 |
| IFIT1, IFI44L, SIGLEC1, ISG15, HERC6, SLC1A2, OLAH, FAM20A, RETN | 0,870 | 0,861 | 0,880 | 0,924 | 0,873 | 0,975 | 0,930 | 0,858 | 1 |
| RSAD2, IFI27, OAS1, IFIT1, IFI44L, SIGLEC1, ISG15, SLC1A2, OLAH | 0,870 | 0,862 | 0,879 | 0,930 | 0,880 | 0,981 | 0,948 | 0,892 | 1 |
| RSAD2, IFI27, OAS1, IFIT1, SIGLEC1, SLC1A2, OLAH, RETN, MMP8 | 0,870 | 0,861 | 0,880 | 0,928 | 0,875 | 0,981 | 0,954 | 0,897 | 1 |
| RSAD2, IFI27, OAS1, ISG15, HERC6, SLC1A2, IL1R2, OLAH, MMP8 | 0,870 | 0,861 | 0,880 | 0,929 | 0,880 | 0,977 | 0,930 | 0,862 | 0,999 |
| OAS1, IFI44L, SIGLEC1, ISG15, HERC6, IL1R2, FAM20A, RETN, MMP8 | 0,870 | 0,861 | 0,880 | 0,918 | 0,860 | 0,976 | 0,958 | 0,894 | 1 |
| RSAD2, IFI27, OAS1, IFI44L, SIGLEC1, ISG15, SLC1A2, OLAH, RETN | 0,870 | 0,862 | 0,879 | 0,929 | 0,877 | 0,981 | 0,948 | 0,885 | 1 |
| RSAD2, IFI27, IFIT1, SIGLEC1, ISG15, HERC6, SLC1A2, FAM20A, MMP8 | 0,870 | 0,861 | 0,880 | 0,929 | 0,880 | 0,979 | 0,936 | 0,857 | 1 |
| IFIT1, IFI44L, SIGLEC1, ISG15, SLC1A2, OLAH, FAM20A, RETN, MMP8 | 0,870 | 0,861 | 0,880 | 0,922 | 0,869 | 0,975 | 0,949 | 0,888 | 1 |
| RSAD2, IFI27, OAS1, SIGLEC1, ISG15, HERC6, SLC1A2, IL1R2, RETN | 0,870 | 0,862 | 0,879 | 0,926 | 0,878 | 0,975 | 0,948 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, IL1R2, OLAH, FAM20A, MMP8 | 0,870 | 0,860 | 0,880 | 0,919 | 0,862 | 0,975 | 0,962 | 0,905 | 1 |
| OAS1, IFIT1, IFI44L, SIGLEC1, ISG15, HERC6, SLC1A2, OLAH, FAM20A | 0,870 | 0,860 | 0,881 | 0,925 | 0,875 | 0,975 | 0,929 | 0,859 | 1 |
| RSAD2, IFI27, OAS1, IFI44L, SIGLEC1, SLC1A2, IL1R2, OLAH, RETN | 0,870 | 0,861 | 0,879 | 0,925 | 0,874 | 0,977 | 0,949 | 0,886 | 1 |
| IFI27, IFIT1, IFI44L, SIGLEC1, SLC1A2, IL1R2, OLAH, RETN, MMP8 | 0,870 | 0,861 | 0,880 | 0,930 | 0,880 | 0,980 | 0,958 | 0,908 | 1 |
| IFI27, IFIT1, IFI44L, SIGLEC1, ISG15, SLC1A2, IL1R2, OLAH, RETN | 0,870 | 0,861 | 0,879 | 0,929 | 0,879 | 0,979 | 0,946 | 0,887 | 1 |
| RSAD2, IFI27, IFI44L, SIGLEC1, ISG15, SLC1A2, IL1R2, OLAH, RETN | 0,870 | 0,862 | 0,879 | 0,929 | 0,878 | 0,979 | 0,943 | 0,884 | 1 |
| RSAD2, IFI27, OAS1, IFIT1, IFI44L, SLC1A2, IL1R2, FAM20A, MMP8 | 0,870 | 0,861 | 0,880 | 0,928 | 0,880 | 0,976 | 0,926 | 0,840 | 1 |
| IFI27, OAS1, IFI44L, SIGLEC1, ISG15, SLC1A2, OLAH, RETN, MMP8 | 0,870 | 0,861 | 0,880 | 0,929 | 0,878 | 0,980 | 0,952 | 0,895 | 1 |
| RSAD2, IFI27, IFIT1, SIGLEC1, ISG15, SLC1A2, IL1R2, OLAH, RETN | 0,870 | 0,861 | 0,879 | 0,929 | 0,879 | 0,980 | 0,940 | 0,879 | 1 |
| RSAD2, OAS1, IFIT1, IFI44L, SIGLEC1, HERC6, SLC1A2, OLAH, FAM20A | 0,870 | 0,860 | 0,880 | 0,926 | 0,876 | 0,976 | 0,933 | 0,863 | 1 |
| RSAD2, IFI27, OAS1, SIGLEC1, ISG15, SLC1A2, IL1R2, OLAH, MMP8 | 0,870 | 0,861 | 0,879 | 0,929 | 0,879 | 0,979 | 0,945 | 0,887 | 1 |
| IFI27, IFIT1, ISG15, HERC6, SLC1A2, IL1R2, OLAH, RETN, MMP8 | 0,870 | 0,860 | 0,880 | 0,929 | 0,879 | 0,979 | 0,941 | 0,881 | 1 |
| RSAD2, IFI27, IFIT1, IFI44L, SIGLEC1, HERC6, SLC1A2, OLAH, MMP8 | 0,870 | 0,861 | 0,879 | 0,926 | 0,876 | 0,977 | 0,941 | 0,885 | 0,997 |
| RSAD2, IFI27, OAS1, IFIT1, ISG15, HERC6, SLC1A2, IL1R2, OLAH | 0,870 | 0,861 | 0,880 | 0,929 | 0,879 | 0,978 | 0,933 | 0,866 | 0,999 |
| RSAD2, IFIT1, SIGLEC1, ISG15, IL1R2, OLAH, FAM20A, RETN, MMP8 | 0,870 | 0,861 | 0,879 | 0,920 | 0,863 | 0,978 | 0,967 | 0,916 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, IL1R2, OLAH, RETN, MMP8 | 0,870 | 0,860 | 0,880 | 0,921 | 0,865 | 0,977 | 0,950 | 0,897 | 1 |
| OAS1, IFIT1, IFI44L, SIGLEC1, ISG15, HERC6, OLAH, FAM20A, MMP8 | 0,870 | 0,859 | 0,881 | 0,917 | 0,858 | 0,975 | 0,948 | 0,890 | 1 |
| IFI27, OAS1, IFIT1, IFI44L, SIGLEC1, SLC1A2, IL1R2, OLAH, RETN | 0,870 | 0,861 | 0,879 | 0,927 | 0,875 | 0,978 | 0,950 | 0,891 | 1 |
| IFI27, OAS1, IFIT1, IFI44L, SIGLEC1, ISG15, HERC6, IL1R2, MMP8 | 0,870 | 0,862 | 0,879 | 0,924 | 0,878 | 0,971 | 0,942 | 0,891 | 0,994 |
| IFI27, OAS1, IFIT1, IFI44L, ISG15, HERC6, SLC1A2, OLAH, RETN | 0,870 | 0,860 | 0,880 | 0,928 | 0,878 | 0,978 | 0,934 | 0,867 | 1 |
| RSAD2, IFI27, OAS1, IFIT1, IFI44L, SIGLEC1, IL1R2, RETN, MMP8 | 0,870 | 0,861 | 0,879 | 0,922 | 0,868 | 0,976 | 0,959 | 0,911 | 1 |
| RSAD2, IFI27, OAS1, ISG15, HERC6, SLC1A2, IL1R2, FAM20A, RETN | 0,870 | 0,860 | 0,880 | 0,932 | 0,885 | 0,980 | 0,924 | 0,836 | 1 |
| RSAD2, IFI27, OAS1, SIGLEC1, ISG15, SLC1A2, FAM20A, RETN, MMP8 | 0,870 | 0,859 | 0,881 | 0,925 | 0,869 | 0,982 | 0,942 | 0,868 | 1 |
| RSAD2, IFI27, IFIT1, IFI44L, SLC1A2, IL1R2, FAM20A, RETN, MMP8 | 0,870 | 0,860 | 0,880 | 0,932 | 0,883 | 0,981 | 0,928 | 0,845 | 1 |
| OAS1, IFIT1, IFI44L, SIGLEC1, ISG15, HERC6, OLAH, FAM20A, RETN | 0,870 | 0,860 | 0,880 | 0,916 | 0,858 | 0,975 | 0,940 | 0,880 | 1 |
| RSAD2, OAS1, IFIT1, SIGLEC1, ISG15, HERC6, FAM20A, RETN, MMP8 | 0,870 | 0,861 | 0,879 | 0,921 | 0,863 | 0,979 | 0,947 | 0,882 | 1 |
| IFI27, IFIT1, ISG15, HERC6, SLC1A2, IL1R2, FAM20A, RETN, MMP8 | 0,870 | 0,860 | 0,880 | 0,934 | 0,886 | 0,983 | 0,930 | 0,847 | 1 |
| IFI27, OAS1, IFIT1, IFI44L, SIGLEC1, SLC1A2, IL1R2, OLAH, MMP8 | 0,870 | 0,861 | 0,879 | 0,928 | 0,879 | 0,977 | 0,951 | 0,899 | 1 |
| RSAD2, IFI27, OAS1, IFIT1, SIGLEC1, ISG15, IL1R2, OLAH, RETN | 0,870 | 0,861 | 0,879 | 0,920 | 0,865 | 0,976 | 0,949 | 0,892 | 1 |
| IFI27, OAS1, IFIT1, IFI44L, HERC6, SLC1A2, IL1R2, OLAH, RETN | 0,870 | 0,860 | 0,880 | 0,927 | 0,876 | 0,978 | 0,934 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, SLC1A2, FAM20A, RETN | 0,870 | 0,860 | 0,880 | 0,924 | 0,869 | 0,979 | 0,929 | 0,848 | 1 |
| RSAD2, IFI27, IFIT1, SIGLEC1, ISG15, HERC6, SLC1A2, OLAH, MMP8 | 0,870 | 0,860 | 0,880 | 0,930 | 0,880 | 0,980 | 0,940 | 0,883 | 0,997 |
| RSAD2, IFI27, OAS1, IFIT1, IFI44L, SIGLEC1, ISG15, HERC6, IL1R2 | 0,870 | 0,861 | 0,878 | 0,922 | 0,874 | 0,971 | 0,948 | 0,900 | 0,996 |
| RSAD2, IFI27, OAS1, IFIT1, HERC6, SLC1A2, IL1R2, OLAH, MMP8 | 0,870 | 0,860 | 0,880 | 0,929 | 0,880 | 0,977 | 0,935 | 0,869 | 1 |
| RSAD2, IFI27, OAS1, IFI44L, HERC6, SLC1A2, IL1R2, OLAH, RETN | 0,870 | 0,860 | 0,880 | 0,927 | 0,875 | 0,978 | 0,932 | 0,860 | 1 |
| RSAD2, OAS1, IFIT1, SIGLEC1, ISG15, HERC6, IL1R2, FAM20A, RETN | 0,870 | 0,861 | 0,879 | 0,920 | 0,863 | 0,977 | 0,952 | 0,895 | 1 |
| RSAD2, IFI27, OAS1, IFIT1, IFI44L, SIGLEC1, SLC1A2, OLAH, MMP8 | 0,870 | 0,861 | 0,879 | 0,926 | 0,874 | 0,978 | 0,954 | 0,902 | 1 |
| RSAD2, IFI27, OAS1, IFI44L, ISG15, IL1R2, OLAH, RETN, MMP8 | 0,870 | 0,860 | 0,879 | 0,923 | 0,867 | 0,978 | 0,949 | 0,894 | 1 |
| IFI27, OAS1, IFI44L, SIGLEC1, ISG15, SLC1A2, IL1R2, OLAH, MMP8 | 0,870 | 0,861 | 0,879 | 0,930 | 0,882 | 0,978 | 0,946 | 0,892 | 0,999 |
| IFI27, IFIT1, IFI44L, SIGLEC1, ISG15, HERC6, SLC1A2, IL1R2, OLAH | 0,870 | 0,861 | 0,879 | 0,929 | 0,881 | 0,978 | 0,941 | 0,886 | 0,997 |
| RSAD2, IFI27, OAS1, ISG15, HERC6, SLC1A2, IL1R2, FAM20A, MMP8 | 0,870 | 0,860 | 0,880 | 0,931 | 0,886 | 0,976 | 0,921 | 0,829 | 1 |
| RSAD2, IFI27, OAS1, IFIT1, IFI44L, SIGLEC1, SLC1A2, IL1R2, OLAH | 0,870 | 0,861 | 0,879 | 0,928 | 0,878 | 0,978 | 0,949 | 0,892 | 1 |
| RSAD2, IFI27, OAS1, SIGLEC1, ISG15, SLC1A2, OLAH, RETN, MMP8 | 0,870 | 0,860 | 0,879 | 0,928 | 0,876 | 0,981 | 0,953 | 0,894 | 1 |
| IFI27, IFIT1, IFI44L, ISG15, HERC6, SLC1A2, IL1R2, FAM20A, MMP8 | 0,870 | 0,860 | 0,880 | 0,929 | 0,881 | 0,976 | 0,928 | 0,841 | 1 |
| RSAD2, IFI27, IFIT1, IFI44L, ISG15, IL1R2, OLAH, RETN, MMP8 | 0,870 | 0,860 | 0,879 | 0,919 | 0,861 | 0,976 | 0,949 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, ISG15, SLC1A2, OLAH, RETN, MMP8 | 0,870 | 0,860 | 0,879 | 0,928 | 0,876 | 0,980 | 0,953 | 0,898 | 1 |
| RSAD2, OAS1, IFIT1, IFI44L, SIGLEC1, IL1R2, FAM20A, RETN, MMP8 | 0,870 | 0,860 | 0,880 | 0,923 | 0,867 | 0,979 | 0,968 | 0,918 | 1 |
| RSAD2, IFIT1, SIGLEC1, ISG15, HERC6, IL1R2, OLAH, FAM20A, RETN | 0,870 | 0,861 | 0,879 | 0,918 | 0,860 | 0,977 | 0,952 | 0,897 | 1 |
| RSAD2, IFI44L, SIGLEC1, HERC6, SLC1A2, IL1R2, OLAH, FAM20A, MMP8 | 0,870 | 0,859 | 0,880 | 0,924 | 0,876 | 0,973 | 0,959 | 0,897 | 1 |
| RSAD2, IFI27, IFIT1, IFI44L, SIGLEC1, ISG15, HERC6, SLC1A2, OLAH | 0,870 | 0,861 | 0,879 | 0,930 | 0,881 | 0,980 | 0,940 | 0,883 | 0,998 |
| RSAD2, OAS1, IFIT1, SIGLEC1, HERC6, IL1R2, OLAH, FAM20A, RETN | 0,870 | 0,860 | 0,879 | 0,918 | 0,860 | 0,977 | 0,955 | 0,901 | 1 |
| IFI27, OAS1, IFIT1, ISG15, SLC1A2, IL1R2, FAM20A, RETN, MMP8 | 0,870 | 0,860 | 0,880 | 0,931 | 0,882 | 0,980 | 0,927 | 0,844 | 1 |
| RSAD2, IFI27, OAS1, IFI44L, SIGLEC1, ISG15, HERC6, IL1R2, MMP8 | 0,870 | 0,861 | 0,878 | 0,924 | 0,876 | 0,972 | 0,940 | 0,888 | 0,993 |
| RSAD2, IFI27, IFIT1, IFI44L, HERC6, SLC1A2, IL1R2, FAM20A, RETN | 0,870 | 0,860 | 0,880 | 0,930 | 0,882 | 0,979 | 0,926 | 0,842 | 1 |
| OAS1, IFIT1, IFI44L, SIGLEC1, HERC6, SLC1A2, IL1R2, OLAH, FAM20A | 0,870 | 0,860 | 0,880 | 0,927 | 0,879 | 0,975 | 0,934 | 0,865 | 1 |
| RSAD2, IFI27, OAS1, IFIT1, ISG15, HERC6, SLC1A2, OLAH, MMP8 | 0,870 | 0,860 | 0,880 | 0,929 | 0,879 | 0,979 | 0,929 | 0,861 | 0,998 |
| RSAD2, IFI27, IFIT1, IFI44L, SIGLEC1, SLC1A2, OLAH, RETN, MMP8 | 0,870 | 0,860 | 0,879 | 0,929 | 0,877 | 0,981 | 0,958 | 0,905 | 1 |
| RSAD2, OAS1, SIGLEC1, ISG15, IL1R2, OLAH, FAM20A, RETN, MMP8 | 0,870 | 0,860 | 0,880 | 0,918 | 0,859 | 0,976 | 0,963 | 0,908 | 1 |
| RSAD2, IFIT1, SIGLEC1, HERC6, SLC1A2, IL1R2, FAM20A, RETN, MMP8 | 0,870 | 0,860 | 0,879 | 0,928 | 0,881 | 0,975 | 0,957 | 0,889 | 1 |
| OAS1, IFI44L, SIGLEC1, HERC6, SLC1A2, IL1R2, OLAH, FAM20A, MMP8 | 0,870 | 0,859 | 0,880 | 0,924 | 0,875 | 0,972 | 0,960 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, HERC6, SLC1A2, IL1R2, FAM20A, MMP8 | 0,870 | 0,860 | 0,880 | 0,924 | 0,876 | 0,972 | 0,958 | 0,892 | 1 |
| RSAD2, IFI27, OAS1, IFIT1, SLC1A2, IL1R2, FAM20A, RETN, MMP8 | 0,870 | 0,860 | 0,879 | 0,932 | 0,883 | 0,981 | 0,929 | 0,848 | 1 |
| IFIT1, IFI44L, SIGLEC1, SLC1A2, IL1R2, OLAH, FAM20A, RETN, MMP8 | 0,870 | 0,860 | 0,879 | 0,928 | 0,880 | 0,976 | 0,961 | 0,903 | 1 |
| RSAD2, OAS1, IFI44L, SIGLEC1, HERC6, IL1R2, FAM20A, RETN, MMP8 | 0,870 | 0,860 | 0,880 | 0,921 | 0,865 | 0,977 | 0,960 | 0,899 | 1 |
| RSAD2, IFI27, IFIT1, ISG15, SLC1A2, IL1R2, FAM20A, RETN, MMP8 | 0,870 | 0,860 | 0,879 | 0,933 | 0,884 | 0,981 | 0,929 | 0,847 | 1 |
| IFI27, OAS1, IFI44L, SIGLEC1, ISG15, SLC1A2, IL1R2, OLAH, RETN | 0,870 | 0,861 | 0,879 | 0,929 | 0,878 | 0,979 | 0,947 | 0,888 | 1 |
| RSAD2, IFIT1, SIGLEC1, HERC6, SLC1A2, OLAH, FAM20A, RETN, MMP8 | 0,870 | 0,859 | 0,880 | 0,928 | 0,879 | 0,977 | 0,947 | 0,879 | 1 |
| RSAD2, OAS1, SIGLEC1, ISG15, SLC1A2, IL1R2, OLAH, FAM20A, MMP8 | 0,870 | 0,860 | 0,879 | 0,923 | 0,874 | 0,972 | 0,961 | 0,901 | 1 |
| IFI27, IFIT1, IFI44L, ISG15, HERC6, SLC1A2, IL1R2, FAM20A, RETN | 0,870 | 0,860 | 0,879 | 0,930 | 0,882 | 0,979 | 0,926 | 0,842 | 1 |
| RSAD2, IFI27, OAS1, IFIT1, IFI44L, ISG15, IL1R2, OLAH, MMP8 | 0,870 | 0,860 | 0,879 | 0,919 | 0,866 | 0,972 | 0,942 | 0,889 | 0,995 |
| IFI27, IFIT1, IFI44L, HERC6, SLC1A2, IL1R2, OLAH, RETN, MMP8 | 0,869 | 0,860 | 0,879 | 0,926 | 0,874 | 0,978 | 0,941 | 0,880 | 1 |
| RSAD2, IFI27, OAS1, IFIT1, SIGLEC1, ISG15, SLC1A2, OLAH, RETN | 0,869 | 0,861 | 0,878 | 0,930 | 0,879 | 0,982 | 0,946 | 0,882 | 1 |
| RSAD2, IFI27, IFI44L, SIGLEC1, ISG15, HERC6, SLC1A2, FAM20A, RETN | 0,869 | 0,859 | 0,880 | 0,927 | 0,875 | 0,980 | 0,943 | 0,870 | 1 |
| RSAD2, IFI27, OAS1, IFI44L, SIGLEC1, SLC1A2, IL1R2, OLAH, MMP8 | 0,869 | 0,860 | 0,879 | 0,927 | 0,877 | 0,977 | 0,951 | 0,897 | 1 |
| RSAD2, IFI27, OAS1, IFI44L, SIGLEC1, ISG15, HERC6, FAM20A, MMP8 | 0,869 | 0,859 | 0,880 | 0,916 | 0,857 | 0,975 | 0,943 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, ISG15, OLAH, RETN, MMP8 | 0,869 | 0,860 | 0,879 | 0,919 | 0,859 | 0,978 | 0,950 | 0,893 | 1 |
| RSAD2, OAS1, IFIT1, IFI44L, SIGLEC1, ISG15, HERC6, FAM20A, RETN | 0,869 | 0,860 | 0,879 | 0,919 | 0,861 | 0,976 | 0,940 | 0,876 | 1 |
| RSAD2, OAS1, IFIT1, IFI44L, SIGLEC1, HERC6, OLAH, FAM20A, RETN | 0,869 | 0,860 | 0,879 | 0,915 | 0,855 | 0,976 | 0,942 | 0,882 | 1 |
| IFI27, OAS1, IFIT1, ISG15, SLC1A2, IL1R2, OLAH, RETN, MMP8 | 0,869 | 0,860 | 0,879 | 0,927 | 0,876 | 0,978 | 0,942 | 0,880 | 1 |
| IFI44L, SIGLEC1, ISG15, SLC1A2, IL1R2, OLAH, FAM20A, RETN, MMP8 | 0,869 | 0,860 | 0,879 | 0,923 | 0,873 | 0,972 | 0,957 | 0,890 | 1 |
| RSAD2, IFI27, OAS1, IFIT1, IFI44L, HERC6, SLC1A2, OLAH, MMP8 | 0,869 | 0,860 | 0,879 | 0,928 | 0,878 | 0,978 | 0,935 | 0,868 | 1 |
| RSAD2, IFI27, OAS1, IFI44L, SIGLEC1, ISG15, HERC6, SLC1A2, RETN | 0,869 | 0,861 | 0,878 | 0,922 | 0,867 | 0,977 | 0,940 | 0,873 | 1 |
| RSAD2, IFIT1, IFI44L, SIGLEC1, ISG15, HERC6, FAM20A, RETN, MMP8 | 0,869 | 0,860 | 0,879 | 0,921 | 0,864 | 0,979 | 0,945 | 0,878 | 1 |
| RSAD2, IFI27, IFIT1, ISG15, HERC6, SLC1A2, IL1R2, OLAH, RETN | 0,869 | 0,860 | 0,879 | 0,927 | 0,876 | 0,978 | 0,934 | 0,869 | 0,999 |
| IFI27, OAS1, IFI44L, ISG15, HERC6, SLC1A2, IL1R2, OLAH, RETN | 0,869 | 0,859 | 0,879 | 0,929 | 0,878 | 0,979 | 0,932 | 0,864 | 0,999 |
| RSAD2, IFI27, OAS1, IFIT1, IFI44L, SIGLEC1, ISG15, IL1R2, RETN | 0,869 | 0,861 | 0,878 | 0,918 | 0,863 | 0,974 | 0,954 | 0,903 | 1 |
| IFI27, OAS1, IFI44L, SIGLEC1, SLC1A2, IL1R2, OLAH, RETN, MMP8 | 0,869 | 0,859 | 0,879 | 0,929 | 0,879 | 0,979 | 0,952 | 0,896 | 1 |
| OAS1, IFIT1, IFI44L, SIGLEC1, HERC6, SLC1A2, FAM20A, RETN, MMP8 | 0,869 | 0,860 | 0,879 | 0,925 | 0,874 | 0,976 | 0,940 | 0,868 | 1 |
| RSAD2, IFI27, IFIT1, IFI44L, ISG15, SLC1A2, IL1R2, FAM20A, MMP8 | 0,869 | 0,859 | 0,879 | 0,928 | 0,880 | 0,976 | 0,926 | 0,840 | 1 |
| RSAD2, IFI27, IFI44L, ISG15, HERC6, SLC1A2, IL1R2, FAM20A, MMP8 | 0,869 | 0,858 | 0,880 | 0,930 | 0,885 | 0,976 | 0,927 | 0,840 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, SIGLEC1, ISG15, SLC1A2, IL1R2, OLAH, FAM20A, RETN, MMP8 | 0,869 | 0,860 | 0,879 | 0,928 | 0,881 | 0,975 | 0,962 | 0,906 | 1 |
| IFI27, OAS1, IFI44L, SIGLEC1, ISG15, HERC6, SLC1A2, RETN, MMP8 | 0,869 | 0,860 | 0,878 | 0,923 | 0,870 | 0,977 | 0,940 | 0,877 | 1 |
| RSAD2, IFI27, OAS1, IFIT1, ISG15, HERC6, SLC1A2, IL1R2, FAM20A | 0,869 | 0,859 | 0,879 | 0,932 | 0,887 | 0,977 | 0,925 | 0,839 | 1 |
| RSAD2, IFI27, IFIT1, IFI44L, ISG15, HERC6, SLC1A2, IL1R2, FAM20A | 0,869 | 0,860 | 0,879 | 0,928 | 0,880 | 0,976 | 0,925 | 0,838 | 1 |
| RSAD2, IFI27, IFIT1, IFI44L, SIGLEC1, HERC6, SLC1A2, IL1R2, OLAH | 0,869 | 0,860 | 0,878 | 0,927 | 0,878 | 0,977 | 0,946 | 0,893 | 0,998 |
| RSAD2, IFI27, IFI44L, ISG15, HERC6, SLC1A2, IL1R2, OLAH, RETN | 0,869 | 0,860 | 0,878 | 0,926 | 0,876 | 0,977 | 0,935 | 0,870 | 0,999 |
| IFI27, OAS1, IFIT1, IFI44L, SIGLEC1, ISG15, SLC1A2, IL1R2, OLAH | 0,869 | 0,860 | 0,878 | 0,927 | 0,877 | 0,977 | 0,942 | 0,885 | 1,000 |
| RSAD2, IFI27, IFIT1, ISG15, HERC6, SLC1A2, IL1R2, FAM20A, MMP8 | 0,869 | 0,859 | 0,879 | 0,929 | 0,882 | 0,976 | 0,929 | 0,845 | 1 |
| RSAD2, IFI27, OAS1, IFI44L, SIGLEC1, SLC1A2, OLAH, RETN, MMP8 | 0,869 | 0,860 | 0,879 | 0,927 | 0,873 | 0,980 | 0,957 | 0,899 | 1 |
| RSAD2, OAS1, IFIT1, SIGLEC1, ISG15, SLC1A2, OLAH, FAM20A, MMP8 | 0,869 | 0,859 | 0,879 | 0,922 | 0,868 | 0,975 | 0,952 | 0,891 | 1 |
| RSAD2, IFI27, IFIT1, ISG15, HERC6, SLC1A2, IL1R2, OLAH, MMP8 | 0,869 | 0,860 | 0,878 | 0,928 | 0,879 | 0,977 | 0,928 | 0,862 | 0,994 |
| RSAD2, IFI27, OAS1, IFIT1, SLC1A2, IL1R2, OLAH, RETN, MMP8 | 0,869 | 0,860 | 0,879 | 0,925 | 0,873 | 0,978 | 0,942 | 0,880 | 1 |
| RSAD2, OAS1, IFIT1, IFI44L, SIGLEC1, ISG15, HERC6, OLAH, FAM20A | 0,869 | 0,859 | 0,879 | 0,915 | 0,856 | 0,974 | 0,946 | 0,888 | 1 |
| RSAD2, OAS1, IFIT1, IFI44L, SIGLEC1, ISG15, IL1R2, OLAH, FAM20A | 0,869 | 0,860 | 0,878 | 0,912 | 0,849 | 0,975 | 0,963 | 0,914 | 1 |
| RSAD2, IFI27, IFIT1, IFI44L, SIGLEC1, SLC1A2, IL1R2, OLAH, RETN | 0,869 | 0,860 | 0,878 | 0,926 | 0,874 | 0,977 | 0,950 | 0,894 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, SLC1A2, OLAH, RETN, MMP8 | 0,869 | 0,859 | 0,879 | 0,930 | 0,879 | 0,981 | 0,952 | 0,897 | 1 |
| RSAD2, IFI27, IFIT1, IFI44L, SIGLEC1, ISG15, HERC6, RETN, MMP8 | 0,869 | 0,860 | 0,878 | 0,916 | 0,858 | 0,975 | 0,943 | 0,891 | 0,996 |
| IFI27, OAS1, IFIT1, IFI44L, SIGLEC1, ISG15, SLC1A2, FAM20A, MMP8 | 0,869 | 0,859 | 0,879 | 0,921 | 0,865 | 0,978 | 0,939 | 0,866 | 1 |
| RSAD2, IFI27, IFIT1, IFI44L, SIGLEC1, ISG15, SLC1A2, OLAH, RETN | 0,869 | 0,861 | 0,877 | 0,929 | 0,878 | 0,980 | 0,946 | 0,887 | 1 |
| RSAD2, IFIT1, IFI44L, SIGLEC1, IL1R2, OLAH, FAM20A, RETN, MMP8 | 0,869 | 0,859 | 0,879 | 0,920 | 0,863 | 0,977 | 0,968 | 0,918 | 1 |
| RSAD2, IFI27, IFIT1, IFI44L, SIGLEC1, ISG15, SLC1A2, OLAH, MMP8 | 0,869 | 0,860 | 0,878 | 0,931 | 0,882 | 0,981 | 0,941 | 0,883 | 1,000 |
| RSAD2, OAS1, IFIT1, IFI44L, SIGLEC1, HERC6, FAM20A, RETN, MMP8 | 0,869 | 0,859 | 0,879 | 0,919 | 0,860 | 0,979 | 0,945 | 0,879 | 1 |
| RSAD2, IFI27, IFIT1, SIGLEC1, HERC6, SLC1A2, IL1R2, RETN, MMP8 | 0,869 | 0,860 | 0,878 | 0,926 | 0,879 | 0,974 | 0,962 | 0,921 | 1 |
| OAS1, IFI44L, SIGLEC1, HERC6, IL1R2, OLAH, FAM20A, RETN, MMP8 | 0,869 | 0,859 | 0,879 | 0,920 | 0,864 | 0,976 | 0,962 | 0,905 | 1 |
| RSAD2, OAS1, IFIT1, IFI44L, SIGLEC1, ISG15, OLAH, FAM20A, MMP8 | 0,869 | 0,858 | 0,880 | 0,912 | 0,848 | 0,976 | 0,951 | 0,898 | 1 |
| RSAD2, OAS1, IFIT1, IFI44L, SIGLEC1, SLC1A2, IL1R2, FAM20A, MMP8 | 0,869 | 0,859 | 0,879 | 0,927 | 0,880 | 0,974 | 0,963 | 0,904 | 1 |
| OAS1, IFIT1, IFI44L, SIGLEC1, SLC1A2, OLAH, FAM20A, RETN, MMP8 | 0,869 | 0,859 | 0,879 | 0,924 | 0,872 | 0,977 | 0,948 | 0,888 | 1 |
| RSAD2, IFI27, IFIT1, IFI44L, SIGLEC1, SLC1A2, IL1R2, OLAH, MMP8 | 0,869 | 0,860 | 0,878 | 0,926 | 0,877 | 0,976 | 0,950 | 0,899 | 1 |
| RSAD2, IFI27, IFIT1, ISG15, HERC6, SLC1A2, IL1R2, FAM20A, RETN | 0,869 | 0,859 | 0,879 | 0,933 | 0,886 | 0,981 | 0,927 | 0,845 | 1 |
| RSAD2, IFIT1, IFI44L, SIGLEC1, ISG15, HERC6, SLC1A2, OLAH, FAM20A | 0,869 | 0,859 | 0,879 | 0,925 | 0,875 | 0,975 | 0,927 | 0,853 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, HERC6, SLC1A2, OLAH, FAM20A, RETN | 0,869 | 0,859 | 0,879 | 0,925 | 0,875 | 0,975 | 0,928 | 0,856 | 1 |
| RSAD2, OAS1, IFIT1, IFI44L, SIGLEC1, HERC6, IL1R2, FAM20A, RETN | 0,869 | 0,859 | 0,878 | 0,920 | 0,863 | 0,977 | 0,953 | 0,898 | 1 |
| RSAD2, IFI27, OAS1, IFIT1, HERC6, SLC1A2, IL1R2, FAM20A, RETN | 0,869 | 0,859 | 0,879 | 0,931 | 0,883 | 0,979 | 0,924 | 0,837 | 1 |
| RSAD2, IFI27, IFIT1, IFI44L, ISG15, HERC6, SLC1A2, OLAH, RETN | 0,869 | 0,860 | 0,878 | 0,926 | 0,875 | 0,976 | 0,934 | 0,869 | 0,999 |
| RSAD2, IFIT1, IFI44L, SIGLEC1, HERC6, SLC1A2, IL1R2, OLAH, FAM20A | 0,869 | 0,859 | 0,879 | 0,926 | 0,877 | 0,975 | 0,937 | 0,867 | 1 |
| IFIT1, IFI44L, SIGLEC1, ISG15, SLC1A2, IL1R2, FAM20A, RETN, MMP8 | 0,869 | 0,859 | 0,878 | 0,928 | 0,882 | 0,974 | 0,959 | 0,893 | 1 |
| RSAD2, OAS1, IFI44L, SIGLEC1, HERC6, SLC1A2, IL1R2, FAM20A, MMP8 | 0,869 | 0,859 | 0,879 | 0,924 | 0,877 | 0,972 | 0,959 | 0,895 | 1 |
| OAS1, SIGLEC1, ISG15, HERC6, SLC1A2, IL1R2, FAM20A, RETN, MMP8 | 0,869 | 0,859 | 0,879 | 0,926 | 0,878 | 0,974 | 0,959 | 0,895 | 1 |
| OAS1, IFIT1, IFI44L, SIGLEC1, SLC1A2, IL1R2, FAM20A, RETN, MMP8 | 0,869 | 0,859 | 0,879 | 0,927 | 0,881 | 0,974 | 0,963 | 0,906 | 1 |
| IFI27, IFIT1, IFI44L, ISG15, HERC6, SLC1A2, OLAH, RETN, MMP8 | 0,869 | 0,859 | 0,879 | 0,926 | 0,874 | 0,979 | 0,939 | 0,878 | 1 |
| RSAD2, IFI27, OAS1, IFIT1, IFI44L, SIGLEC1, ISG15, SLC1A2, FAM20A | 0,869 | 0,859 | 0,879 | 0,921 | 0,863 | 0,978 | 0,939 | 0,864 | 1 |
| RSAD2, IFI27, OAS1, IFI44L, HERC6, SLC1A2, IL1R2, FAM20A, RETN | 0,869 | 0,859 | 0,879 | 0,932 | 0,884 | 0,979 | 0,925 | 0,838 | 1 |
| RSAD2, IFI27, IFIT1, IFI44L, HERC6, SLC1A2, IL1R2, OLAH, RETN | 0,869 | 0,859 | 0,878 | 0,926 | 0,874 | 0,977 | 0,935 | 0,870 | 0,999 |
| OAS1, IFIT1, SIGLEC1, SLC1A2, IL1R2, OLAH, FAM20A, RETN, MMP8 | 0,869 | 0,859 | 0,879 | 0,927 | 0,878 | 0,975 | 0,961 | 0,903 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, HERC6, SLC1A2, RETN, MMP8 | 0,869 | 0,859 | 0,878 | 0,922 | 0,866 | 0,977 | 0,948 | 0,885 | 1 |
| OAS1, IFIT1, SIGLEC1, ISG15, HERC6, SLC1A2, IL1R2, OLAH, FAM20A | 0,869 | 0,859 | 0,879 | 0,924 | 0,875 | 0,973 | 0,943 | 0,875 | 1 |
| RSAD2, OAS1, IFI44L, SIGLEC1, SLC1A2, IL1R2, OLAH, FAM20A, MMP8 | 0,869 | 0,859 | 0,879 | 0,924 | 0,875 | 0,973 | 0,958 | 0,897 | 1 |
| OAS1, SIGLEC1, ISG15, SLC1A2, IL1R2, OLAH, FAM20A, RETN, MMP8 | 0,869 | 0,859 | 0,879 | 0,923 | 0,873 | 0,972 | 0,961 | 0,903 | 1 |
| IFI27, IFIT1, IFI44L, SIGLEC1, HERC6, SLC1A2, IL1R2, OLAH, MMP8 | 0,869 | 0,859 | 0,878 | 0,927 | 0,878 | 0,976 | 0,952 | 0,904 | 1 |
| OAS1, IFIT1, IFI44L, SIGLEC1, ISG15, SLC1A2, IL1R2, FAM20A, MMP8 | 0,869 | 0,858 | 0,879 | 0,926 | 0,880 | 0,973 | 0,961 | 0,897 | 1 |
| RSAD2, IFIT1, IFI44L, SIGLEC1, HERC6, IL1R2, OLAH, FAM20A, RETN | 0,869 | 0,859 | 0,878 | 0,918 | 0,860 | 0,976 | 0,952 | 0,895 | 1 |
| IFI27, IFI44L, SIGLEC1, ISG15, HERC6, SLC1A2, FAM20A, RETN, MMP8 | 0,869 | 0,858 | 0,880 | 0,927 | 0,872 | 0,981 | 0,950 | 0,876 | 1 |
| RSAD2, IFIT1, SIGLEC1, ISG15, HERC6, SLC1A2, IL1R2, OLAH, FAM20A | 0,869 | 0,859 | 0,878 | 0,924 | 0,874 | 0,974 | 0,945 | 0,875 | 1 |
| IFI27, IFIT1, IFI44L, SIGLEC1, HERC6, SLC1A2, IL1R2, RETN, MMP8 | 0,869 | 0,860 | 0,878 | 0,927 | 0,878 | 0,975 | 0,955 | 0,909 | 1 |
| RSAD2, IFI27, OAS1, IFIT1, IFI44L, ISG15, IL1R2, OLAH, RETN | 0,869 | 0,859 | 0,878 | 0,917 | 0,860 | 0,974 | 0,940 | 0,880 | 1 |
| SIGLEC1, ISG15, HERC6, SLC1A2, IL1R2, OLAH, FAM20A, RETN, MMP8 | 0,869 | 0,859 | 0,879 | 0,923 | 0,873 | 0,972 | 0,957 | 0,890 | 1 |
| IFI27, OAS1, IFIT1, IFI44L, ISG15, HERC6, SLC1A2, OLAH, MMP8 | 0,869 | 0,858 | 0,879 | 0,928 | 0,878 | 0,978 | 0,928 | 0,862 | 0,995 |
| OAS1, SIGLEC1, HERC6, SLC1A2, IL1R2, OLAH, FAM20A, RETN, MMP8 | 0,869 | 0,858 | 0,879 | 0,926 | 0,878 | 0,975 | 0,959 | 0,897 | 1 |
| RSAD2, IFI27, OAS1, IFI44L, ISG15, HERC6, SLC1A2, OLAH, MMP8 | 0,869 | 0,859 | 0,878 | 0,929 | 0,879 | 0,979 | 0,932 | 0,864 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, ISG15, IL1R2, FAM20A, RETN, MMP8 | 0,869 | 0,859 | 0,878 | 0,922 | 0,865 | 0,979 | 0,967 | 0,916 | 1 |
| IFI27, OAS1, IFIT1, IFI44L, ISG15, SLC1A2, IL1R2, FAM20A, MMP8 | 0,869 | 0,858 | 0,879 | 0,927 | 0,879 | 0,975 | 0,926 | 0,840 | 1 |
| RSAD2, SIGLEC1, ISG15, HERC6, SLC1A2, IL1R2, FAM20A, RETN, MMP8 | 0,869 | 0,859 | 0,878 | 0,925 | 0,877 | 0,973 | 0,955 | 0,887 | 1 |
| RSAD2, IFI27, IFI44L, ISG15, SLC1A2, IL1R2, FAM20A, RETN, MMP8 | 0,868 | 0,858 | 0,879 | 0,930 | 0,881 | 0,979 | 0,925 | 0,837 | 1 |
| RSAD2, IFI27, IFI44L, ISG15, HERC6, SLC1A2, IL1R2, FAM20A, RETN | 0,868 | 0,858 | 0,879 | 0,930 | 0,882 | 0,979 | 0,926 | 0,842 | 1 |
| OAS1, IFIT1, IFI44L, SIGLEC1, ISG15, HERC6, SLC1A2, IL1R2, FAM20A | 0,868 | 0,859 | 0,878 | 0,926 | 0,879 | 0,974 | 0,941 | 0,872 | 1 |
| OAS1, IFI44L, SIGLEC1, ISG15, HERC6, SLC1A2, IL1R2, FAM20A, MMP8 | 0,868 | 0,858 | 0,879 | 0,924 | 0,876 | 0,972 | 0,958 | 0,892 | 1 |
| RSAD2, IFI27, IFIT1, IFI44L, SIGLEC1, ISG15, SLC1A2, IL1R2, OLAH | 0,868 | 0,860 | 0,877 | 0,928 | 0,878 | 0,978 | 0,943 | 0,889 | 0,998 |
| RSAD2, IFIT1, IFI44L, SIGLEC1, ISG15, HERC6, IL1R2, FAM20A, RETN | 0,868 | 0,859 | 0,878 | 0,919 | 0,861 | 0,976 | 0,953 | 0,897 | 1 |
| OAS1, IFI44L, SIGLEC1, ISG15, SLC1A2, IL1R2, OLAH, FAM20A, MMP8 | 0,868 | 0,858 | 0,878 | 0,922 | 0,871 | 0,972 | 0,961 | 0,901 | 1 |
| RSAD2, IFIT1, IFI44L, SIGLEC1, ISG15, SLC1A2, IL1R2, FAM20A, MMP8 | 0,868 | 0,859 | 0,878 | 0,927 | 0,881 | 0,973 | 0,961 | 0,897 | 1 |
| RSAD2, OAS1, IFIT1, SIGLEC1, ISG15, HERC6, OLAH, FAM20A, RETN | 0,868 | 0,859 | 0,878 | 0,915 | 0,854 | 0,975 | 0,949 | 0,892 | 1 |
| RSAD2, IFI27, OAS1, IFI44L, SIGLEC1, HERC6, SLC1A2, RETN, MMP8 | 0,868 | 0,859 | 0,877 | 0,923 | 0,868 | 0,978 | 0,942 | 0,875 | 1 |
| IFI27, OAS1, IFIT1, IFI44L, HERC6, SLC1A2, IL1R2, OLAH, MMP8 | 0,868 | 0,858 | 0,878 | 0,929 | 0,880 | 0,977 | 0,936 | 0,873 | 0,999 |
| RSAD2, IFI27, OAS1, IFIT1, ISG15, SLC1A2, IL1R2, OLAH, MMP8 | 0,868 | 0,859 | 0,878 | 0,929 | 0,880 | 0,978 | 0,933 | 0,867 | 0,998 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, ISG15, HERC6, SLC1A2, IL1R2, FAM20A, RETN, MMP8 | 0,868 | 0,858 | 0,878 | 0,924 | 0,876 | 0,973 | 0,957 | 0,889 | 1 |
| IFI27, OAS1, IFI44L, SIGLEC1, ISG15, SLC1A2, FAM20A, RETN, MMP8 | 0,868 | 0,857 | 0,880 | 0,922 | 0,864 | 0,979 | 0,938 | 0,859 | 1 |
| OAS1, IFIT1, SIGLEC1, ISG15, SLC1A2, IL1R2, FAM20A, RETN, MMP8 | 0,868 | 0,859 | 0,878 | 0,927 | 0,881 | 0,974 | 0,959 | 0,893 | 1 |
| IFI27, OAS1, IFI44L, ISG15, SLC1A2, IL1R2, FAM20A, RETN, MMP8 | 0,868 | 0,858 | 0,878 | 0,931 | 0,883 | 0,979 | 0,923 | 0,835 | 1 |
| RSAD2, IFIT1, SIGLEC1, SLC1A2, IL1R2, OLAH, FAM20A, RETN, MMP8 | 0,868 | 0,859 | 0,878 | 0,927 | 0,879 | 0,975 | 0,961 | 0,903 | 1 |
| RSAD2, IFI27, IFI44L, ISG15, SLC1A2, IL1R2, OLAH, RETN, MMP8 | 0,868 | 0,859 | 0,878 | 0,927 | 0,875 | 0,979 | 0,939 | 0,876 | 1 |
| RSAD2, IFI27, IFIT1, ISG15, SLC1A2, IL1R2, OLAH, RETN, MMP8 | 0,868 | 0,858 | 0,878 | 0,927 | 0,876 | 0,979 | 0,941 | 0,880 | 1 |
| RSAD2, IFI44L, SIGLEC1, ISG15, HERC6, IL1R2, FAM20A, RETN, MMP8 | 0,868 | 0,858 | 0,878 | 0,918 | 0,861 | 0,976 | 0,959 | 0,897 | 1 |
| RSAD2, OAS1, IFIT1, SIGLEC1, HERC6, SLC1A2, IL1R2, OLAH, FAM20A | 0,868 | 0,858 | 0,878 | 0,926 | 0,877 | 0,974 | 0,947 | 0,882 | 1 |
| IFI27, OAS1, IFI44L, ISG15, HERC6, SLC1A2, IL1R2, OLAH, MMP8 | 0,868 | 0,858 | 0,878 | 0,927 | 0,878 | 0,976 | 0,928 | 0,861 | 0,995 |
| RSAD2, SIGLEC1, HERC6, SLC1A2, IL1R2, OLAH, FAM20A, RETN, MMP8 | 0,868 | 0,858 | 0,878 | 0,926 | 0,878 | 0,974 | 0,958 | 0,895 | 1 |
| RSAD2, IFI27, OAS1, IFI44L, HERC6, SLC1A2, IL1R2, OLAH, MMP8 | 0,868 | 0,858 | 0,878 | 0,929 | 0,880 | 0,977 | 0,935 | 0,869 | 1 |
| RSAD2, IFI27, IFIT1, IFI44L, SIGLEC1, ISG15, IL1R2, RETN, MMP8 | 0,868 | 0,859 | 0,877 | 0,923 | 0,870 | 0,976 | 0,958 | 0,914 | 1 |
| IFI27, OAS1, IFIT1, IFI44L, SIGLEC1, SLC1A2, IL1R2, RETN, MMP8 | 0,868 | 0,859 | 0,877 | 0,926 | 0,878 | 0,974 | 0,958 | 0,908 | 1 |
| RSAD2, IFI27, IFIT1, IFI44L, HERC6, SLC1A2, IL1R2, OLAH, MMP8 | 0,868 | 0,859 | 0,878 | 0,926 | 0,876 | 0,975 | 0,933 | 0,870 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, ISG15, SLC1A2, IL1R2, FAM20A, MMP8 | 0,868 | 0,858 | 0,878 | 0,930 | 0,884 | 0,977 | 0,927 | 0,840 | 1 |
| RSAD2, IFI44L, SIGLEC1, HERC6, IL1R2, OLAH, FAM20A, RETN, MMP8 | 0,868 | 0,858 | 0,878 | 0,920 | 0,864 | 0,976 | 0,961 | 0,903 | 1 |
| OAS1, IFI44L, SIGLEC1, ISG15, IL1R2, OLAH, FAM20A, RETN, MMP8 | 0,868 | 0,858 | 0,878 | 0,915 | 0,856 | 0,975 | 0,964 | 0,909 | 1 |
| IFI27, IFIT1, IFI44L, ISG15, SLC1A2, IL1R2, OLAH, RETN, MMP8 | 0,868 | 0,858 | 0,878 | 0,926 | 0,874 | 0,978 | 0,942 | 0,883 | 1 |
| RSAD2, IFI27, IFIT1, IFI44L, SIGLEC1, ISG15, SLC1A2, FAM20A, MMP8 | 0,868 | 0,858 | 0,878 | 0,922 | 0,866 | 0,977 | 0,938 | 0,863 | 1 |
| RSAD2, IFI27, OAS1, IFIT1, IFI44L, SIGLEC1, SLC1A2, FAM20A, MMP8 | 0,868 | 0,858 | 0,878 | 0,923 | 0,866 | 0,979 | 0,939 | 0,865 | 1 |
| RSAD2, IFI44L, SIGLEC1, ISG15, SLC1A2, IL1R2, OLAH, FAM20A, MMP8 | 0,868 | 0,858 | 0,878 | 0,922 | 0,873 | 0,972 | 0,959 | 0,897 | 1 |
| RSAD2, IFI27, OAS1, IFI44L, ISG15, HERC6, SLC1A2, IL1R2, OLAH | 0,868 | 0,858 | 0,878 | 0,929 | 0,881 | 0,977 | 0,927 | 0,857 | 0,997 |
| RSAD2, OAS1, IFIT1, SIGLEC1, ISG15, SLC1A2, IL1R2, FAM20A, MMP8 | 0,868 | 0,858 | 0,878 | 0,927 | 0,881 | 0,974 | 0,959 | 0,893 | 1 |
| IFI27, OAS1, IFIT1, IFI44L, HERC6, SLC1A2, FAM20A, RETN, MMP8 | 0,868 | 0,858 | 0,878 | 0,922 | 0,865 | 0,979 | 0,936 | 0,854 | 1 |
| RSAD2, OAS1, SIGLEC1, HERC6, SLC1A2, IL1R2, FAM20A, RETN, MMP8 | 0,868 | 0,858 | 0,878 | 0,926 | 0,878 | 0,974 | 0,957 | 0,891 | 1 |
| RSAD2, SIGLEC1, ISG15, SLC1A2, IL1R2, OLAH, FAM20A, RETN, MMP8 | 0,868 | 0,858 | 0,878 | 0,924 | 0,876 | 0,973 | 0,959 | 0,897 | 1 |
| RSAD2, IFI27, OAS1, IFI44L, SIGLEC1, ISG15, SLC1A2, FAM20A, RETN | 0,868 | 0,857 | 0,878 | 0,923 | 0,867 | 0,980 | 0,940 | 0,864 | 1 |
| RSAD2, IFI27, IFI44L, ISG15, HERC6, SLC1A2, IL1R2, OLAH, MMP8 | 0,868 | 0,859 | 0,877 | 0,929 | 0,881 | 0,978 | 0,927 | 0,859 | 0,995 |
| RSAD2, IFI27, OAS1, IFIT1, ISG15, SLC1A2, IL1R2, OLAH, RETN | 0,868 | 0,858 | 0,877 | 0,927 | 0,876 | 0,979 | 0,936 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, SLC1A2, IL1R2, RETN, MMP8 | 0,868 | 0,859 | 0,876 | 0,928 | 0,880 | 0,975 | 0,954 | 0,908 | 1 |
| RSAD2, IFI27, IFIT1, IFI44L, SIGLEC1, ISG15, HERC6, IL1R2, MMP8 | 0,868 | 0,859 | 0,876 | 0,920 | 0,871 | 0,970 | 0,939 | 0,886 | 0,992 |
| RSAD2, IFI27, OAS1, IFI44L, SIGLEC1, ISG15, IL1R2, RETN, MMP8 | 0,868 | 0,858 | 0,877 | 0,920 | 0,866 | 0,975 | 0,960 | 0,913 | 1 |
| RSAD2, IFIT1, IFI44L, SIGLEC1, ISG15, HERC6, SLC1A2, IL1R2, FAM20A | 0,868 | 0,858 | 0,877 | 0,926 | 0,878 | 0,974 | 0,941 | 0,869 | 1 |
| RSAD2, IFI27, OAS1, IFIT1, IFI44L, HERC6, SLC1A2, IL1R2, OLAH | 0,868 | 0,858 | 0,877 | 0,926 | 0,877 | 0,975 | 0,937 | 0,873 | 1 |
| RSAD2, IFI27, OAS1, IFIT1, IFI44L, SLC1A2, OLAH, RETN, MMP8 | 0,868 | 0,858 | 0,877 | 0,923 | 0,867 | 0,978 | 0,941 | 0,877 | 1 |
| RSAD2, IFI27, OAS1, IFIT1, IFI44L, HERC6, SLC1A2, FAM20A, RETN | 0,868 | 0,858 | 0,878 | 0,922 | 0,865 | 0,978 | 0,934 | 0,852 | 1 |
| RSAD2, OAS1, SIGLEC1, SLC1A2, IL1R2, OLAH, FAM20A, RETN, MMP8 | 0,868 | 0,858 | 0,878 | 0,924 | 0,875 | 0,973 | 0,961 | 0,903 | 1 |
| RSAD2, IFI27, IFI44L, SIGLEC1, ISG15, SLC1A2, FAM20A, RETN, MMP8 | 0,868 | 0,857 | 0,879 | 0,926 | 0,870 | 0,982 | 0,943 | 0,868 | 1 |
| RSAD2, IFI27, OAS1, ISG15, SLC1A2, IL1R2, OLAH, RETN, MMP8 | 0,868 | 0,858 | 0,877 | 0,929 | 0,877 | 0,980 | 0,937 | 0,870 | 1 |
| RSAD2, IFI27, OAS1, IFIT1, IFI44L, ISG15, SLC1A2, OLAH, MMP8 | 0,868 | 0,858 | 0,877 | 0,925 | 0,872 | 0,978 | 0,933 | 0,865 | 1,000 |
| RSAD2, IFI27, OAS1, IFIT1, SIGLEC1, ISG15, HERC6, IL1R2, MMP8 | 0,868 | 0,859 | 0,876 | 0,922 | 0,875 | 0,969 | 0,950 | 0,903 | 0,997 |
| RSAD2, IFI27, OAS1, IFI44L, SIGLEC1, SLC1A2, FAM20A, RETN, MMP8 | 0,868 | 0,857 | 0,878 | 0,924 | 0,867 | 0,982 | 0,946 | 0,874 | 1 |
| RSAD2, IFI27, IFIT1, IFI44L, ISG15, HERC6, IL1R2, RETN, MMP8 | 0,868 | 0,858 | 0,877 | 0,922 | 0,868 | 0,975 | 0,942 | 0,885 | 1,000 |
| RSAD2, OAS1, IFIT1, SIGLEC1, ISG15, HERC6, SLC1A2, OLAH, FAM20A | 0,868 | 0,857 | 0,878 | 0,922 | 0,870 | 0,973 | 0,940 | 0,874 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, ISG15, HERC6, SLC1A2, OLAH | 0,868 | 0,858 | 0,877 | 0,928 | 0,877 | 0,978 | 0,930 | 0,863 | 0,998 |
| RSAD2, IFI27, IFIT1, IFI44L, ISG15, HERC6, SLC1A2, OLAH, MMP8 | 0,868 | 0,858 | 0,877 | 0,927 | 0,877 | 0,978 | 0,929 | 0,865 | 0,994 |
| RSAD2, IFI44L, SIGLEC1, ISG15, HERC6, SLC1A2, IL1R2, FAM20A, MMP8 | 0,867 | 0,857 | 0,878 | 0,926 | 0,879 | 0,973 | 0,959 | 0,893 | 1 |
| RSAD2, IFIT1, IFI44L, SIGLEC1, ISG15, HERC6, OLAH, FAM20A, RETN | 0,867 | 0,858 | 0,877 | 0,918 | 0,859 | 0,977 | 0,942 | 0,882 | 1 |
| IFIT1, IFI44L, SIGLEC1, ISG15, HERC6, SLC1A2, IL1R2, FAM20A, RETN | 0,867 | 0,858 | 0,877 | 0,925 | 0,877 | 0,974 | 0,941 | 0,869 | 1 |
| IFI27, OAS1, IFIT1, IFI44L, ISG15, SLC1A2, IL1R2, FAM20A, RETN | 0,867 | 0,858 | 0,877 | 0,924 | 0,873 | 0,976 | 0,924 | 0,837 | 1 |
| RSAD2, IFI27, IFIT1, IFI44L, SLC1A2, IL1R2, OLAH, RETN, MMP8 | 0,867 | 0,858 | 0,877 | 0,924 | 0,872 | 0,977 | 0,946 | 0,888 | 1 |
| IFIT1, IFI44L, SIGLEC1, HERC6, SLC1A2, IL1R2, OLAH, FAM20A, RETN | 0,867 | 0,857 | 0,877 | 0,925 | 0,876 | 0,975 | 0,938 | 0,868 | 1 |
| RSAD2, IFI27, OAS1, IFIT1, ISG15, SLC1A2, IL1R2, FAM20A, MMP8 | 0,867 | 0,857 | 0,877 | 0,927 | 0,878 | 0,975 | 0,926 | 0,841 | 1 |
| OAS1, IFIT1, IFI44L, SIGLEC1, ISG15, IL1R2, OLAH, FAM20A, RETN | 0,867 | 0,858 | 0,877 | 0,910 | 0,847 | 0,973 | 0,961 | 0,910 | 1 |
| RSAD2, IFI27, OAS1, IFIT1, ISG15, SLC1A2, OLAH, RETN, MMP8 | 0,867 | 0,857 | 0,877 | 0,925 | 0,870 | 0,979 | 0,940 | 0,875 | 1 |
| RSAD2, IFI27, IFIT1, IFI44L, SIGLEC1, HERC6, SLC1A2, IL1R2, RETN | 0,867 | 0,859 | 0,876 | 0,927 | 0,878 | 0,976 | 0,946 | 0,895 | 0,997 |
| RSAD2, IFI27, IFI44L, SIGLEC1, HERC6, SLC1A2, IL1R2, RETN, MMP8 | 0,867 | 0,858 | 0,876 | 0,926 | 0,878 | 0,974 | 0,955 | 0,909 | 1 |
| RSAD2, IFI27, OAS1, IFI44L, SLC1A2, IL1R2, FAM20A, RETN, MMP8 | 0,867 | 0,857 | 0,877 | 0,930 | 0,881 | 0,979 | 0,923 | 0,835 | 1 |
| IFI27, IFIT1, IFI44L, ISG15, HERC6, SLC1A2, IL1R2, OLAH, RETN | 0,867 | 0,858 | 0,877 | 0,926 | 0,874 | 0,977 | 0,933 | 0,867 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, SLC1A2, OLAH, FAM20A, RETN, MMP8 | 0,867 | 0,857 | 0,877 | 0,923 | 0,871 | 0,976 | 0,948 | 0,888 | 1 |
| RSAD2, OAS1, IFIT1, SIGLEC1, ISG15, HERC6, SLC1A2, IL1R2, FAM20A | 0,867 | 0,857 | 0,877 | 0,926 | 0,877 | 0,974 | 0,948 | 0,882 | 1 |
| RSAD2, IFI27, OAS1, IFIT1, SIGLEC1, ISG15, IL1R2, RETN, MMP8 | 0,867 | 0,858 | 0,876 | 0,921 | 0,866 | 0,975 | 0,965 | 0,920 | 1 |
| RSAD2, IFIT1, SIGLEC1, ISG15, SLC1A2, IL1R2, FAM20A, RETN, MMP8 | 0,867 | 0,858 | 0,876 | 0,928 | 0,882 | 0,975 | 0,960 | 0,894 | 1 |
| IFI27, IFIT1, IFI44L, SIGLEC1, ISG15, HERC6, SLC1A2, IL1R2, RETN | 0,867 | 0,858 | 0,876 | 0,928 | 0,880 | 0,976 | 0,945 | 0,892 | 0,997 |
| RSAD2, OAS1, IFIT1, SIGLEC1, ISG15, IL1R2, OLAH, FAM20A, RETN | 0,867 | 0,858 | 0,876 | 0,912 | 0,850 | 0,974 | 0,964 | 0,914 | 1 |
| RSAD2, IFI27, OAS1, IFIT1, ISG15, HERC6, SLC1A2, FAM20A, RETN | 0,867 | 0,857 | 0,877 | 0,923 | 0,867 | 0,979 | 0,932 | 0,850 | 1 |
| RSAD2, OAS1, IFIT1, IFI44L, SIGLEC1, HERC6, SLC1A2, IL1R2, FAM20A | 0,867 | 0,857 | 0,877 | 0,927 | 0,879 | 0,974 | 0,946 | 0,879 | 1 |
| IFI27, OAS1, IFIT1, IFI44L, SLC1A2, IL1R2, OLAH, RETN, MMP8 | 0,867 | 0,857 | 0,877 | 0,924 | 0,872 | 0,977 | 0,942 | 0,881 | 1 |
| OAS1, IFI44L, SIGLEC1, HERC6, SLC1A2, IL1R2, FAM20A, RETN, MMP8 | 0,867 | 0,857 | 0,877 | 0,926 | 0,878 | 0,975 | 0,959 | 0,895 | 1 |
| RSAD2, IFIT1, SIGLEC1, HERC6, SLC1A2, IL1R2, OLAH, FAM20A, RETN | 0,867 | 0,857 | 0,877 | 0,924 | 0,874 | 0,974 | 0,947 | 0,876 | 1 |
| OAS1, IFIT1, SIGLEC1, ISG15, HERC6, SLC1A2, FAM20A, RETN, MMP8 | 0,867 | 0,857 | 0,877 | 0,921 | 0,868 | 0,975 | 0,943 | 0,873 | 1 |
| IFI27, OAS1, IFIT1, IFI44L, ISG15, HERC6, SLC1A2, IL1R2, OLAH | 0,867 | 0,857 | 0,877 | 0,926 | 0,877 | 0,976 | 0,927 | 0,859 | 0,995 |
| OAS1, IFIT1, SIGLEC1, ISG15, HERC6, SLC1A2, OLAH, FAM20A, RETN | 0,867 | 0,857 | 0,877 | 0,921 | 0,869 | 0,973 | 0,936 | 0,865 | 1 |
| IFI27, IFIT1, IFI44L, ISG15, HERC6, SLC1A2, IL1R2, OLAH, MMP8 | 0,867 | 0,857 | 0,876 | 0,928 | 0,880 | 0,977 | 0,930 | 0,866 | 0,995 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, IL1R2, OLAH, FAM20A, RETN, MMP8 | 0,867 | 0,856 | 0,877 | 0,919 | 0,861 | 0,976 | 0,962 | 0,906 | 1 |
| RSAD2, IFI27, OAS1, IFI44L, ISG15, SLC1A2, IL1R2, OLAH, MMP8 | 0,867 | 0,858 | 0,876 | 0,929 | 0,880 | 0,978 | 0,930 | 0,862 | 0,998 |
| IFI27, OAS1, IFIT1, IFI44L, SIGLEC1, ISG15, SLC1A2, IL1R2, RETN | 0,867 | 0,858 | 0,876 | 0,924 | 0,875 | 0,973 | 0,950 | 0,896 | 1 |
| IFIT1, IFI44L, SIGLEC1, ISG15, SLC1A2, IL1R2, OLAH, FAM20A, RETN | 0,867 | 0,857 | 0,876 | 0,921 | 0,868 | 0,974 | 0,943 | 0,882 | 1 |
| RSAD2, IFIT1, IFI44L, SIGLEC1, HERC6, SLC1A2, FAM20A, RETN, MMP8 | 0,867 | 0,857 | 0,876 | 0,922 | 0,869 | 0,975 | 0,940 | 0,867 | 1 |
| OAS1, IFIT1, IFI44L, SIGLEC1, ISG15, OLAH, FAM20A, RETN, MMP8 | 0,867 | 0,856 | 0,878 | 0,914 | 0,849 | 0,978 | 0,961 | 0,913 | 1 |
| RSAD2, IFI27, IFIT1, IFI44L, ISG15, HERC6, SLC1A2, FAM20A, RETN | 0,867 | 0,857 | 0,877 | 0,922 | 0,867 | 0,977 | 0,928 | 0,846 | 1 |
| RSAD2, IFI27, OAS1, IFI44L, ISG15, SLC1A2, IL1R2, OLAH, RETN | 0,867 | 0,858 | 0,876 | 0,927 | 0,876 | 0,979 | 0,932 | 0,861 | 1 |
| IFI27, IFIT1, SIGLEC1, ISG15, HERC6, SLC1A2, IL1R2, RETN, MMP8 | 0,867 | 0,858 | 0,876 | 0,928 | 0,881 | 0,974 | 0,963 | 0,923 | 1 |
| RSAD2, OAS1, SIGLEC1, HERC6, SLC1A2, OLAH, FAM20A, RETN, MMP8 | 0,867 | 0,857 | 0,877 | 0,921 | 0,867 | 0,974 | 0,948 | 0,882 | 1 |
| RSAD2, IFIT1, IFI44L, SIGLEC1, SLC1A2, IL1R2, FAM20A, RETN, MMP8 | 0,867 | 0,857 | 0,876 | 0,929 | 0,882 | 0,975 | 0,961 | 0,900 | 1 |
| IFI44L, SIGLEC1, HERC6, SLC1A2, IL1R2, OLAH, FAM20A, RETN, MMP8 | 0,867 | 0,856 | 0,877 | 0,923 | 0,873 | 0,973 | 0,955 | 0,889 | 1 |
| RSAD2, IFI27, OAS1, IFIT1, IFI44L, SLC1A2, IL1R2, OLAH, MMP8 | 0,867 | 0,857 | 0,876 | 0,927 | 0,877 | 0,977 | 0,939 | 0,875 | 1 |
| IFIT1, SIGLEC1, ISG15, HERC6, SLC1A2, IL1R2, OLAH, FAM20A, RETN | 0,867 | 0,857 | 0,877 | 0,924 | 0,875 | 0,974 | 0,942 | 0,871 | 1 |
| RSAD2, OAS1, IFI44L, SIGLEC1, ISG15, IL1R2, FAM20A, RETN, MMP8 | 0,867 | 0,857 | 0,877 | 0,918 | 0,860 | 0,976 | 0,961 | 0,902 | 1 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, ISG15, HERC6, SLC1A2, FAM20A, RETN, MMP8 | 0,867 | 0,857 | 0,876 | 0,924 | 0,871 | 0,976 | 0,945 | 0,873 | 1 |
| OAS1, IFIT1, SIGLEC1, HERC6, SLC1A2, IL1R2, OLAH, FAM20A, RETN | 0,867 | 0,857 | 0,877 | 0,924 | 0,875 | 0,973 | 0,942 | 0,872 | 1 |
| RSAD2, IFIT1, IFI44L, SIGLEC1, HERC6, SLC1A2, OLAH, FAM20A, RETN | 0,867 | 0,856 | 0,877 | 0,925 | 0,875 | 0,975 | 0,928 | 0,855 | 1 |
| RSAD2, OAS1, SIGLEC1, ISG15, HERC6, SLC1A2, OLAH, FAM20A, MMP8 | 0,867 | 0,856 | 0,877 | 0,919 | 0,867 | 0,972 | 0,945 | 0,876 | 1 |
| OAS1, IFIT1, IFI44L, SIGLEC1, HERC6, SLC1A2, IL1R2, FAM20A, RETN | 0,867 | 0,857 | 0,876 | 0,927 | 0,881 | 0,974 | 0,941 | 0,872 | 1 |
| RSAD2, IFI27, IFIT1, IFI44L, ISG15, SLC1A2, OLAH, RETN, MMP8 | 0,867 | 0,857 | 0,877 | 0,924 | 0,869 | 0,979 | 0,942 | 0,880 | 1 |
| IFI27, IFIT1, IFI44L, SIGLEC1, ISG15, SLC1A2, IL1R2, RETN, MMP8 | 0,867 | 0,858 | 0,875 | 0,925 | 0,877 | 0,973 | 0,958 | 0,912 | 1 |
| RSAD2, OAS1, IFIT1, SIGLEC1, SLC1A2, IL1R2, FAM20A, RETN, MMP8 | 0,867 | 0,857 | 0,876 | 0,927 | 0,879 | 0,974 | 0,961 | 0,900 | 1 |
| OAS1, IFIT1, SIGLEC1, ISG15, SLC1A2, OLAH, FAM20A, RETN, MMP8 | 0,867 | 0,856 | 0,877 | 0,922 | 0,869 | 0,975 | 0,951 | 0,891 | 1 |
| IFI27, OAS1, IFIT1, IFI44L, ISG15, SLC1A2, OLAH, RETN, MMP8 | 0,867 | 0,856 | 0,877 | 0,925 | 0,871 | 0,979 | 0,941 | 0,877 | 1 |
| IFI27, OAS1, IFI44L, ISG15, HERC6, SLC1A2, FAM20A, RETN, MMP8 | 0,867 | 0,856 | 0,877 | 0,925 | 0,869 | 0,981 | 0,935 | 0,854 | 1 |
| IFI27, OAS1, IFIT1, IFI44L, ISG15, SLC1A2, IL1R2, OLAH, MMP8 | 0,867 | 0,857 | 0,876 | 0,928 | 0,880 | 0,977 | 0,933 | 0,869 | 0,996 |
| RSAD2, IFI27, IFIT1, IFI44L, ISG15, SLC1A2, IL1R2, OLAH, RETN | 0,866 | 0,857 | 0,875 | 0,927 | 0,876 | 0,978 | 0,935 | 0,870 | 1,000 |
| OAS1, IFIT1, SIGLEC1, ISG15, HERC6, SLC1A2, IL1R2, FAM20A, RETN | 0,866 | 0,856 | 0,877 | 0,924 | 0,876 | 0,973 | 0,945 | 0,873 | 1 |
| RSAD2, IFI27, OAS1, IFIT1, HERC6, SLC1A2, IL1R2, RETN, MMP8 | 0,866 | 0,857 | 0,876 | 0,923 | 0,872 | 0,974 | 0,940 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, ISG15, SLC1A2, IL1R2, RETN, MMP8 | 0,866 | 0,857 | 0,876 | 0,926 | 0,879 | 0,974 | 0,963 | 0,916 | 1 |
| RSAD2, OAS1, IFIT1, SIGLEC1, HERC6, SLC1A2, FAM20A, RETN, MMP8 | 0,866 | 0,857 | 0,876 | 0,922 | 0,868 | 0,975 | 0,943 | 0,873 | 1 |
| RSAD2, IFI27, IFIT1, IFI44L, ISG15, HERC6, SLC1A2, IL1R2, OLAH | 0,866 | 0,857 | 0,876 | 0,928 | 0,879 | 0,977 | 0,929 | 0,864 | 0,995 |
| RSAD2, IFI27, IFIT1, IFI44L, ISG15, SLC1A2, IL1R2, OLAH, MMP8 | 0,866 | 0,857 | 0,875 | 0,929 | 0,880 | 0,977 | 0,934 | 0,872 | 0,995 |
| RSAD2, IFI27, IFIT1, SIGLEC1, ISG15, HERC6, SLC1A2, IL1R2, RETN | 0,866 | 0,858 | 0,875 | 0,927 | 0,881 | 0,974 | 0,952 | 0,905 | 1,000 |
| RSAD2, IFI27, SIGLEC1, ISG15, HERC6, SLC1A2, IL1R2, RETN, MMP8 | 0,866 | 0,857 | 0,875 | 0,927 | 0,879 | 0,974 | 0,962 | 0,921 | 1 |
| RSAD2, IFI27, OAS1, IFI44L, SIGLEC1, ISG15, SLC1A2, IL1R2, RETN | 0,866 | 0,858 | 0,875 | 0,925 | 0,876 | 0,975 | 0,950 | 0,890 | 1 |
| OAS1, SIGLEC1, ISG15, HERC6, SLC1A2, OLAH, FAM20A, RETN, MMP8 | 0,866 | 0,856 | 0,877 | 0,919 | 0,865 | 0,973 | 0,947 | 0,881 | 1 |
| IFI27, OAS1, IFIT1, ISG15, HERC6, SLC1A2, IL1R2, RETN, MMP8 | 0,866 | 0,857 | 0,876 | 0,923 | 0,872 | 0,974 | 0,940 | 0,877 | 1 |
| RSAD2, OAS1, IFIT1, SIGLEC1, HERC6, SLC1A2, OLAH, FAM20A, RETN | 0,866 | 0,856 | 0,876 | 0,924 | 0,874 | 0,974 | 0,941 | 0,875 | 1 |
| IFI27, OAS1, IFIT1, ISG15, HERC6, SLC1A2, FAM20A, RETN, MMP8 | 0,866 | 0,855 | 0,877 | 0,922 | 0,866 | 0,979 | 0,933 | 0,851 | 1 |
| OAS1, IFIT1, IFI44L, SIGLEC1, ISG15, HERC6, SLC1A2, FAM20A, RETN | 0,866 | 0,856 | 0,876 | 0,924 | 0,874 | 0,974 | 0,935 | 0,865 | 1 |
| RSAD2, IFI27, OAS1, IFI44L, SLC1A2, IL1R2, OLAH, RETN, MMP8 | 0,866 | 0,856 | 0,876 | 0,928 | 0,876 | 0,980 | 0,940 | 0,875 | 1 |
| RSAD2, IFI44L, SIGLEC1, HERC6, SLC1A2, IL1R2, FAM20A, RETN, MMP8 | 0,866 | 0,856 | 0,876 | 0,926 | 0,878 | 0,975 | 0,958 | 0,892 | 1 |
| RSAD2, OAS1, SIGLEC1, ISG15, SLC1A2, IL1R2, FAM20A, RETN, MMP8 | 0,866 | 0,856 | 0,876 | 0,925 | 0,877 | 0,973 | 0,959 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, HERC6, OLAH, FAM20A, RETN, MMP8 | 0,866 | 0,856 | 0,876 | 0,916 | 0,856 | 0,975 | 0,951 | 0,893 | 1 |
| IFI27, OAS1, IFI44L, ISG15, SLC1A2, IL1R2, OLAH, RETN, MMP8 | 0,866 | 0,856 | 0,876 | 0,927 | 0,876 | 0,979 | 0,936 | 0,870 | 1 |
| RSAD2, IFI27, OAS1, ISG15, HERC6, SLC1A2, IL1R2, RETN, MMP8 | 0,866 | 0,857 | 0,875 | 0,925 | 0,874 | 0,975 | 0,934 | 0,863 | 1 |
| RSAD2, IFI27, OAS1, IFIT1, IFI44L, SLC1A2, IL1R2, OLAH, RETN | 0,866 | 0,857 | 0,875 | 0,926 | 0,874 | 0,977 | 0,939 | 0,871 | 1 |
| RSAD2, IFI27, OAS1, IFIT1, SIGLEC1, ISG15, SLC1A2, FAM20A, MMP8 | 0,866 | 0,856 | 0,876 | 0,922 | 0,866 | 0,977 | 0,943 | 0,871 | 1 |
| RSAD2, IFI27, OAS1, IFIT1, IFI44L, SIGLEC1, SLC1A2, IL1R2, RETN | 0,866 | 0,857 | 0,875 | 0,925 | 0,876 | 0,974 | 0,950 | 0,889 | 1 |
| RSAD2, IFI44L, SIGLEC1, ISG15, IL1R2, OLAH, FAM20A, RETN, MMP8 | 0,866 | 0,856 | 0,876 | 0,918 | 0,859 | 0,976 | 0,964 | 0,911 | 1 |
| RSAD2, OAS1, IFIT1, IFI44L, SIGLEC1, IL1R2, OLAH, FAM20A, RETN | 0,866 | 0,856 | 0,876 | 0,910 | 0,847 | 0,974 | 0,961 | 0,908 | 1 |
| RSAD2, IFIT1, SIGLEC1, ISG15, HERC6, SLC1A2, OLAH, FAM20A, RETN | 0,866 | 0,856 | 0,876 | 0,920 | 0,867 | 0,974 | 0,938 | 0,865 | 1 |
| RSAD2, IFI27, OAS1, ISG15, SLC1A2, IL1R2, FAM20A, RETN, MMP8 | 0,866 | 0,856 | 0,876 | 0,930 | 0,881 | 0,978 | 0,928 | 0,842 | 1 |
| RSAD2, IFI27, OAS1, IFIT1, IFI44L, ISG15, SLC1A2, IL1R2, FAM20A | 0,866 | 0,856 | 0,876 | 0,925 | 0,876 | 0,974 | 0,925 | 0,839 | 1 |
| RSAD2, IFI27, OAS1, SIGLEC1, ISG15, HERC6, SLC1A2, FAM20A, MMP8 | 0,866 | 0,855 | 0,877 | 0,919 | 0,865 | 0,974 | 0,940 | 0,864 | 1 |
| RSAD2, IFI27, OAS1, IFI44L, ISG15, SLC1A2, OLAH, RETN, MMP8 | 0,866 | 0,856 | 0,876 | 0,927 | 0,874 | 0,981 | 0,939 | 0,874 | 1 |
| RSAD2, IFI27, OAS1, IFI44L, ISG15, HERC6, SLC1A2, FAM20A, RETN | 0,866 | 0,855 | 0,877 | 0,923 | 0,867 | 0,978 | 0,935 | 0,854 | 1 |
| RSAD2, IFI27, OAS1, IFIT1, IFI44L, ISG15, SLC1A2, IL1R2, OLAH | 0,866 | 0,856 | 0,875 | 0,927 | 0,877 | 0,978 | 0,932 | 0,865 | 0,998 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, ISG15, SLC1A2, IL1R2, FAM20A, RETN | 0,866 | 0,856 | 0,876 | 0,927 | 0,877 | 0,977 | 0,924 | 0,837 | 1 |
| RSAD2, IFIT1, SIGLEC1, ISG15, HERC6, SLC1A2, IL1R2, FAM20A, RETN | 0,866 | 0,856 | 0,876 | 0,926 | 0,877 | 0,974 | 0,946 | 0,874 | 1 |
| IFI27, OAS1, IFIT1, IFI44L, HERC6, SLC1A2, IL1R2, RETN, MMP8 | 0,866 | 0,856 | 0,875 | 0,923 | 0,872 | 0,974 | 0,937 | 0,871 | 1 |
| RSAD2, IFI27, OAS1, IFIT1, ISG15, SLC1A2, IL1R2, FAM20A, RETN | 0,866 | 0,856 | 0,875 | 0,927 | 0,878 | 0,976 | 0,921 | 0,833 | 1 |
| IFI27, OAS1, IFIT1, IFI44L, SIGLEC1, HERC6, SLC1A2, IL1R2, MMP8 | 0,866 | 0,857 | 0,875 | 0,926 | 0,881 | 0,970 | 0,950 | 0,902 | 0,998 |
| RSAD2, IFI27, OAS1, IFIT1, ISG15, HERC6, SLC1A2, IL1R2, RETN | 0,866 | 0,857 | 0,875 | 0,924 | 0,873 | 0,975 | 0,935 | 0,865 | 1 |
| RSAD2, IFI27, IFI44L, SIGLEC1, ISG15, HERC6, SLC1A2, IL1R2, RETN | 0,866 | 0,857 | 0,874 | 0,928 | 0,879 | 0,976 | 0,943 | 0,890 | 0,997 |
| RSAD2, IFI27, OAS1, IFIT1, IFI44L, HERC6, SLC1A2, IL1R2, RETN | 0,866 | 0,857 | 0,874 | 0,923 | 0,872 | 0,974 | 0,930 | 0,857 | 1 |
| RSAD2, IFI27, OAS1, IFIT1, IFI44L, SLC1A2, IL1R2, FAM20A, RETN | 0,866 | 0,856 | 0,876 | 0,924 | 0,873 | 0,976 | 0,922 | 0,834 | 1 |
| RSAD2, IFI27, OAS1, IFI44L, SIGLEC1, SLC1A2, IL1R2, RETN, MMP8 | 0,866 | 0,857 | 0,875 | 0,927 | 0,879 | 0,975 | 0,957 | 0,902 | 1 |
| OAS1, IFIT1, IFI44L, SIGLEC1, SLC1A2, IL1R2, OLAH, FAM20A, RETN | 0,866 | 0,856 | 0,875 | 0,922 | 0,870 | 0,975 | 0,946 | 0,885 | 1 |
| RSAD2, OAS1, IFIT1, IFI44L, SIGLEC1, SLC1A2, IL1R2, OLAH, FAM20A | 0,866 | 0,856 | 0,875 | 0,921 | 0,868 | 0,974 | 0,950 | 0,892 | 1 |
| RSAD2, OAS1, IFI44L, SIGLEC1, ISG15, HERC6, IL1R2, OLAH, FAM20A | 0,866 | 0,856 | 0,875 | 0,915 | 0,856 | 0,973 | 0,959 | 0,905 | 1 |
| RSAD2, OAS1, IFIT1, IFI44L, SIGLEC1, OLAH, FAM20A, RETN, MMP8 | 0,866 | 0,855 | 0,876 | 0,915 | 0,853 | 0,978 | 0,959 | 0,910 | 1 |
| RSAD2, IFI27, OAS1, IFI44L, ISG15, HERC6, SLC1A2, IL1R2, RETN | 0,865 | 0,857 | 0,874 | 0,923 | 0,872 | 0,974 | 0,932 | 0,858 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, SLC1A2, IL1R2, RETN, MMP8 | 0,865 | 0,857 | 0,874 | 0,925 | 0,877 | 0,974 | 0,961 | 0,908 | 1 |
| IFI27, OAS1, IFI44L, SIGLEC1, ISG15, SLC1A2, IL1R2, RETN, MMP8 | 0,865 | 0,857 | 0,874 | 0,927 | 0,881 | 0,974 | 0,959 | 0,910 | 1 |
| RSAD2, IFI27, OAS1, IFIT1, IFI44L, SIGLEC1, HERC6, SLC1A2, IL1R2 | 0,865 | 0,857 | 0,874 | 0,926 | 0,880 | 0,972 | 0,954 | 0,906 | 1 |
| OAS1, IFI44L, SIGLEC1, ISG15, SLC1A2, IL1R2, FAM20A, RETN, MMP8 | 0,865 | 0,856 | 0,875 | 0,925 | 0,877 | 0,974 | 0,958 | 0,894 | 1 |
| RSAD2, IFI27, OAS1, IFI44L, SIGLEC1, ISG15, HERC6, SLC1A2, IL1R2 | 0,865 | 0,857 | 0,874 | 0,926 | 0,880 | 0,972 | 0,957 | 0,911 | 1 |
| RSAD2, OAS1, IFIT1, SIGLEC1, ISG15, OLAH, FAM20A, RETN, MMP8 | 0,865 | 0,855 | 0,875 | 0,915 | 0,852 | 0,978 | 0,961 | 0,912 | 1 |
| RSAD2, IFI27, OAS1, IFIT1, IFI44L, SIGLEC1, ISG15, IL1R2, MMP8 | 0,865 | 0,857 | 0,874 | 0,919 | 0,869 | 0,970 | 0,951 | 0,905 | 0,997 |
| RSAD2, IFIT1, IFI44L, SIGLEC1, ISG15, IL1R2, OLAH, FAM20A, RETN | 0,865 | 0,856 | 0,875 | 0,911 | 0,849 | 0,974 | 0,963 | 0,914 | 1 |
| OAS1, IFIT1, IFI44L, SIGLEC1, ISG15, SLC1A2, OLAH, FAM20A, RETN | 0,865 | 0,856 | 0,875 | 0,920 | 0,864 | 0,975 | 0,934 | 0,867 | 1 |
| RSAD2, IFIT1, SIGLEC1, ISG15, HERC6, SLC1A2, FAM20A, RETN, MMP8 | 0,865 | 0,856 | 0,875 | 0,921 | 0,868 | 0,975 | 0,943 | 0,871 | 1 |
| RSAD2, OAS1, IFI44L, SIGLEC1, HERC6, SLC1A2, OLAH, FAM20A, MMP8 | 0,865 | 0,855 | 0,876 | 0,921 | 0,868 | 0,973 | 0,942 | 0,875 | 1 |
| RSAD2, IFI27, OAS1, IFIT1, IFI44L, ISG15, SLC1A2, OLAH, RETN | 0,865 | 0,856 | 0,875 | 0,927 | 0,874 | 0,979 | 0,935 | 0,865 | 1 |
| IFI27, OAS1, IFI44L, ISG15, HERC6, SLC1A2, IL1R2, RETN, MMP8 | 0,865 | 0,856 | 0,875 | 0,924 | 0,875 | 0,974 | 0,938 | 0,872 | 1 |
| RSAD2, SIGLEC1, ISG15, HERC6, SLC1A2, OLAH, FAM20A, RETN, MMP8 | 0,865 | 0,855 | 0,875 | 0,920 | 0,866 | 0,974 | 0,943 | 0,873 | 1 |
| RSAD2, IFI27, OAS1, ISG15, HERC6, SLC1A2, FAM20A, RETN, MMP8 | 0,865 | 0,854 | 0,876 | 0,922 | 0,865 | 0,978 | 0,933 | 0,851 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, HERC6, SLC1A2, IL1R2, FAM20A, RETN | 0,865 | 0,856 | 0,875 | 0,926 | 0,877 | 0,974 | 0,941 | 0,869 | 1 |
| IFI27, OAS1, IFIT1, IFI44L, ISG15, HERC6, SLC1A2, IL1R2, RETN | 0,865 | 0,856 | 0,874 | 0,924 | 0,875 | 0,974 | 0,938 | 0,874 | 1 |
| OAS1, IFIT1, IFI44L, SIGLEC1, ISG15, SLC1A2, IL1R2, OLAH, FAM20A | 0,865 | 0,855 | 0,875 | 0,919 | 0,866 | 0,973 | 0,942 | 0,879 | 1 |
| RSAD2, OAS1, IFIT1, IFI44L, SIGLEC1, ISG15, SLC1A2, OLAH, FAM20A | 0,865 | 0,856 | 0,875 | 0,920 | 0,865 | 0,975 | 0,939 | 0,876 | 1 |
| IFI27, OAS1, IFIT1, IFI44L, SIGLEC1, ISG15, HERC6, SLC1A2, IL1R2 | 0,865 | 0,856 | 0,874 | 0,926 | 0,881 | 0,970 | 0,949 | 0,901 | 0,997 |
| RSAD2, IFI27, OAS1, IFI44L, HERC6, SLC1A2, IL1R2, RETN, MMP8 | 0,865 | 0,856 | 0,875 | 0,923 | 0,872 | 0,974 | 0,933 | 0,860 | 1 |
| RSAD2, IFI27, OAS1, IFIT1, SIGLEC1, ISG15, SLC1A2, IL1R2, RETN | 0,865 | 0,857 | 0,874 | 0,924 | 0,876 | 0,973 | 0,957 | 0,899 | 1 |
| RSAD2, IFI27, OAS1, IFI44L, SIGLEC1, HERC6, SLC1A2, FAM20A, MMP8 | 0,865 | 0,855 | 0,875 | 0,920 | 0,867 | 0,974 | 0,939 | 0,863 | 1 |
| RSAD2, IFI27, OAS1, IFI44L, SIGLEC1, ISG15, HERC6, SLC1A2, FAM20A | 0,865 | 0,855 | 0,875 | 0,920 | 0,866 | 0,974 | 0,940 | 0,864 | 1 |
| RSAD2, OAS1, IFIT1, SIGLEC1, SLC1A2, OLAH, FAM20A, RETN, MMP8 | 0,865 | 0,855 | 0,875 | 0,923 | 0,870 | 0,976 | 0,953 | 0,896 | 1 |
| RSAD2, IFI27, OAS1, IFIT1, SIGLEC1, HERC6, SLC1A2, IL1R2, MMP8 | 0,865 | 0,856 | 0,874 | 0,926 | 0,881 | 0,972 | 0,951 | 0,901 | 1 |
| RSAD2, OAS1, IFI44L, SIGLEC1, ISG15, SLC1A2, OLAH, FAM20A, MMP8 | 0,865 | 0,855 | 0,875 | 0,918 | 0,863 | 0,973 | 0,945 | 0,881 | 1 |
| RSAD2, IFI27, IFIT1, IFI44L, SIGLEC1, SLC1A2, IL1R2, RETN, MMP8 | 0,865 | 0,856 | 0,874 | 0,926 | 0,878 | 0,975 | 0,958 | 0,912 | 1 |
| RSAD2, IFIT1, IFI44L, SIGLEC1, ISG15, SLC1A2, IL1R2, OLAH, FAM20A | 0,865 | 0,856 | 0,874 | 0,920 | 0,867 | 0,974 | 0,948 | 0,888 | 1 |
| IFI27, OAS1, IFIT1, IFI44L, ISG15, SLC1A2, IL1R2, OLAH, RETN | 0,865 | 0,855 | 0,875 | 0,927 | 0,877 | 0,978 | 0,934 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, SLC1A2, RETN, MMP8 | 0,865 | 0,855 | 0,875 | 0,915 | 0,858 | 0,973 | 0,936 | 0,866 | 1 |
| OAS1, IFIT1, SIGLEC1, ISG15, SLC1A2, IL1R2, OLAH, FAM20A, RETN | 0,865 | 0,855 | 0,875 | 0,920 | 0,867 | 0,972 | 0,953 | 0,892 | 1 |
| RSAD2, OAS1, IFIT1, SIGLEC1, HERC6, SLC1A2, IL1R2, FAM20A, RETN | 0,865 | 0,855 | 0,875 | 0,926 | 0,878 | 0,974 | 0,948 | 0,882 | 1 |
| RSAD2, OAS1, IFIT1, SIGLEC1, ISG15, SLC1A2, IL1R2, OLAH, FAM20A | 0,865 | 0,855 | 0,875 | 0,919 | 0,864 | 0,973 | 0,957 | 0,896 | 1 |
| RSAD2, OAS1, IFI44L, SIGLEC1, ISG15, SLC1A2, IL1R2, FAM20A, MMP8 | 0,865 | 0,855 | 0,875 | 0,924 | 0,876 | 0,972 | 0,959 | 0,893 | 1 |
| RSAD2, IFI27, OAS1, IFI44L, SIGLEC1, HERC6, SLC1A2, IL1R2, MMP8 | 0,865 | 0,856 | 0,874 | 0,927 | 0,881 | 0,973 | 0,953 | 0,904 | 1 |
| OAS1, IFI44L, SIGLEC1, ISG15, HERC6, SLC1A2, OLAH, FAM20A, MMP8 | 0,865 | 0,854 | 0,876 | 0,919 | 0,866 | 0,972 | 0,942 | 0,874 | 1 |
| IFI27, IFIT1, IFI44L, ISG15, HERC6, SLC1A2, FAM20A, RETN, MMP8 | 0,865 | 0,854 | 0,875 | 0,922 | 0,864 | 0,979 | 0,936 | 0,854 | 1 |
| RSAD2, OAS1, IFIT1, SIGLEC1, SLC1A2, IL1R2, OLAH, FAM20A, RETN | 0,865 | 0,855 | 0,874 | 0,922 | 0,869 | 0,974 | 0,957 | 0,898 | 1 |
| RSAD2, IFI27, OAS1, IFIT1, IFI44L, ISG15, HERC6, SLC1A2, RETN | 0,865 | 0,855 | 0,874 | 0,917 | 0,859 | 0,976 | 0,930 | 0,855 | 1 |
| RSAD2, OAS1, IFIT1, IFI44L, SIGLEC1, ISG15, HERC6, FAM20A, MMP8 | 0,865 | 0,855 | 0,875 | 0,911 | 0,850 | 0,972 | 0,935 | 0,866 | 1 |
| RSAD2, IFI27, OAS1, IFI44L, ISG15, SLC1A2, IL1R2, FAM20A, RETN | 0,865 | 0,854 | 0,875 | 0,924 | 0,873 | 0,975 | 0,923 | 0,835 | 1 |
| RSAD2, IFIT1, SIGLEC1, ISG15, SLC1A2, OLAH, FAM20A, RETN, MMP8 | 0,865 | 0,855 | 0,874 | 0,921 | 0,868 | 0,974 | 0,952 | 0,893 | 1 |
| RSAD2, IFI44L, SIGLEC1, SLC1A2, IL1R2, OLAH, FAM20A, RETN, MMP8 | 0,865 | 0,854 | 0,875 | 0,924 | 0,875 | 0,973 | 0,960 | 0,900 | 1 |
| RSAD2, OAS1, IFIT1, IFI44L, SIGLEC1, ISG15, IL1R2, FAM20A, RETN | 0,865 | 0,855 | 0,874 | 0,911 | 0,848 | 0,973 | 0,963 | 0,911 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, ISG15, HERC6, SLC1A2, IL1R2 | 0,865 | 0,856 | 0,873 | 0,926 | 0,881 | 0,971 | 0,953 | 0,905 | 1 |
| IFI27, OAS1, IFIT1, SIGLEC1, ISG15, HERC6, SLC1A2, IL1R2, MMP8 | 0,865 | 0,855 | 0,874 | 0,927 | 0,882 | 0,971 | 0,949 | 0,899 | 0,999 |
| RSAD2, IFIT1, SIGLEC1, ISG15, SLC1A2, IL1R2, OLAH, FAM20A, RETN | 0,865 | 0,855 | 0,874 | 0,922 | 0,871 | 0,974 | 0,958 | 0,899 | 1 |
| RSAD2, IFI27, IFIT1, SIGLEC1, ISG15, SLC1A2, IL1R2, RETN, MMP8 | 0,864 | 0,856 | 0,873 | 0,928 | 0,881 | 0,974 | 0,964 | 0,922 | 1 |
| RSAD2, IFIT1, IFI44L, SIGLEC1, SLC1A2, IL1R2, OLAH, FAM20A, RETN | 0,864 | 0,855 | 0,874 | 0,922 | 0,870 | 0,975 | 0,950 | 0,890 | 1 |
| RSAD2, IFI27, OAS1, IFIT1, IFI44L, ISG15, HERC6, FAM20A, MMP8 | 0,864 | 0,854 | 0,875 | 0,915 | 0,856 | 0,973 | 0,917 | 0,837 | 0,998 |
| RSAD2, IFI27, IFIT1, ISG15, HERC6, SLC1A2, FAM20A, RETN, MMP8 | 0,864 | 0,854 | 0,875 | 0,924 | 0,869 | 0,980 | 0,935 | 0,853 | 1 |
| IFI27, OAS1, IFI44L, SIGLEC1, ISG15, HERC6, SLC1A2, FAM20A, MMP8 | 0,864 | 0,854 | 0,875 | 0,923 | 0,872 | 0,975 | 0,942 | 0,866 | 1 |
| OAS1, IFI44L, SIGLEC1, SLC1A2, IL1R2, OLAH, FAM20A, RETN, MMP8 | 0,864 | 0,854 | 0,875 | 0,922 | 0,872 | 0,973 | 0,958 | 0,899 | 1 |
| RSAD2, OAS1, IFIT1, IFI44L, SIGLEC1, ISG15, HERC6, SLC1A2, FAM20A | 0,864 | 0,855 | 0,874 | 0,919 | 0,867 | 0,972 | 0,929 | 0,852 | 1 |
| RSAD2, OAS1, IFIT1, IFI44L, SIGLEC1, HERC6, SLC1A2, FAM20A, RETN | 0,864 | 0,855 | 0,874 | 0,924 | 0,874 | 0,975 | 0,935 | 0,862 | 1 |
| RSAD2, IFI27, IFI44L, SIGLEC1, ISG15, HERC6, SLC1A2, FAM20A, MMP8 | 0,864 | 0,854 | 0,875 | 0,921 | 0,867 | 0,975 | 0,938 | 0,860 | 1 |
| RSAD2, IFI27, IFIT1, IFI44L, HERC6, SLC1A2, FAM20A, RETN, MMP8 | 0,864 | 0,854 | 0,874 | 0,923 | 0,868 | 0,979 | 0,938 | 0,856 | 1 |
| RSAD2, OAS1, IFIT1, IFI44L, SIGLEC1, HERC6, SLC1A2, FAM20A, MMP8 | 0,864 | 0,854 | 0,874 | 0,920 | 0,869 | 0,972 | 0,935 | 0,859 | 1 |
| RSAD2, IFI27, OAS1, IFIT1, IFI44L, ISG15, IL1R2, RETN, MMP8 | 0,864 | 0,854 | 0,874 | 0,919 | 0,863 | 0,974 | 0,942 | 0,883 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, HERC6, IL1R2, OLAH, FAM20A, RETN | 0,864 | 0,854 | 0,874 | 0,914 | 0,855 | 0,972 | 0,958 | 0,902 | 1 |
| RSAD2, IFI44L, SIGLEC1, ISG15, HERC6, SLC1A2, OLAH, FAM20A, MMP8 | 0,864 | 0,854 | 0,874 | 0,920 | 0,868 | 0,973 | 0,945 | 0,878 | 1 |
| RSAD2, IFI27, OAS1, IFIT1, IFI44L, ISG15, HERC6, IL1R2, MMP8 | 0,864 | 0,855 | 0,873 | 0,919 | 0,869 | 0,969 | 0,938 | 0,882 | 0,994 |
| RSAD2, OAS1, SIGLEC1, ISG15, HERC6, SLC1A2, IL1R2, OLAH, FAM20A | 0,864 | 0,853 | 0,875 | 0,919 | 0,867 | 0,970 | 0,946 | 0,878 | 1 |
| RSAD2, IFI27, IFIT1, IFI44L, SIGLEC1, ISG15, HERC6, SLC1A2, RETN | 0,864 | 0,855 | 0,873 | 0,923 | 0,871 | 0,975 | 0,940 | 0,883 | 0,998 |
| RSAD2, IFIT1, IFI44L, SIGLEC1, ISG15, HERC6, SLC1A2, FAM20A, MMP8 | 0,864 | 0,854 | 0,874 | 0,921 | 0,870 | 0,973 | 0,929 | 0,852 | 1 |
| OAS1, IFI44L, SIGLEC1, HERC6, SLC1A2, OLAH, FAM20A, RETN, MMP8 | 0,864 | 0,854 | 0,874 | 0,920 | 0,866 | 0,974 | 0,947 | 0,881 | 1 |
| RSAD2, IFI27, OAS1, SIGLEC1, ISG15, HERC6, SLC1A2, IL1R2, MMP8 | 0,864 | 0,855 | 0,873 | 0,926 | 0,880 | 0,971 | 0,952 | 0,902 | 1 |
| RSAD2, IFI44L, SIGLEC1, ISG15, SLC1A2, IL1R2, FAM20A, RETN, MMP8 | 0,864 | 0,854 | 0,874 | 0,925 | 0,877 | 0,973 | 0,958 | 0,892 | 1 |
| RSAD2, OAS1, IFI44L, SIGLEC1, SLC1A2, IL1R2, FAM20A, RETN, MMP8 | 0,864 | 0,854 | 0,874 | 0,923 | 0,873 | 0,973 | 0,958 | 0,894 | 1 |
| OAS1, IFIT1, IFI44L, SIGLEC1, ISG15, HERC6, SLC1A2, FAM20A, MMP8 | 0,864 | 0,853 | 0,874 | 0,922 | 0,870 | 0,973 | 0,930 | 0,854 | 1 |
| RSAD2, IFI27, OAS1, SIGLEC1, ISG15, SLC1A2, IL1R2, RETN, MMP8 | 0,864 | 0,855 | 0,873 | 0,926 | 0,878 | 0,974 | 0,960 | 0,906 | 1 |
| RSAD2, OAS1, IFIT1, SIGLEC1, ISG15, HERC6, SLC1A2, FAM20A, RETN | 0,864 | 0,854 | 0,874 | 0,922 | 0,870 | 0,973 | 0,942 | 0,875 | 1 |
| RSAD2, IFI27, IFI44L, ISG15, HERC6, SLC1A2, FAM20A, RETN, MMP8 | 0,864 | 0,853 | 0,875 | 0,925 | 0,869 | 0,980 | 0,938 | 0,860 | 1 |
| RSAD2, IFIT1, IFI44L, SIGLEC1, ISG15, SLC1A2, OLAH, FAM20A, RETN | 0,864 | 0,854 | 0,873 | 0,921 | 0,865 | 0,976 | 0,937 | 0,872 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, HERC6, SLC1A2, FAM20A, RETN, MMP8 | 0,864 | 0,853 | 0,875 | 0,923 | 0,866 | 0,981 | 0,936 | 0,855 | 1 |
| RSAD2, IFI27, OAS1, IFIT1, HERC6, SLC1A2, FAM20A, RETN, MMP8 | 0,864 | 0,853 | 0,874 | 0,923 | 0,866 | 0,980 | 0,932 | 0,848 | 1 |
| RSAD2, IFIT1, IFI44L, SIGLEC1, ISG15, HERC6, SLC1A2, FAM20A, RETN | 0,864 | 0,854 | 0,873 | 0,924 | 0,874 | 0,974 | 0,934 | 0,858 | 1 |
| OAS1, IFIT1, IFI44L, SIGLEC1, ISG15, SLC1A2, IL1R2, FAM20A, RETN | 0,864 | 0,854 | 0,873 | 0,919 | 0,867 | 0,971 | 0,952 | 0,893 | 1 |
| RSAD2, IFI27, IFI44L, SIGLEC1, ISG15, SLC1A2, IL1R2, RETN, MMP8 | 0,864 | 0,855 | 0,872 | 0,928 | 0,881 | 0,975 | 0,959 | 0,914 | 1 |
| RSAD2, IFI27, IFIT1, IFI44L, SIGLEC1, ISG15, SLC1A2, IL1R2, RETN | 0,864 | 0,855 | 0,872 | 0,924 | 0,875 | 0,974 | 0,947 | 0,893 | 1 |
| RSAD2, IFI27, OAS1, IFIT1, IFI44L, SIGLEC1, ISG15, SLC1A2, IL1R2 | 0,864 | 0,855 | 0,872 | 0,926 | 0,880 | 0,972 | 0,955 | 0,909 | 1 |
| IFI27, OAS1, IFI44L, SIGLEC1, ISG15, HERC6, SLC1A2, IL1R2, MMP8 | 0,864 | 0,855 | 0,872 | 0,926 | 0,881 | 0,970 | 0,950 | 0,902 | 0,998 |
| RSAD2, OAS1, IFIT1, IFI44L, SIGLEC1, SLC1A2, OLAH, FAM20A, RETN | 0,863 | 0,853 | 0,874 | 0,919 | 0,863 | 0,975 | 0,938 | 0,875 | 1 |
| IFI27, OAS1, IFIT1, IFI44L, SIGLEC1, ISG15, SLC1A2, IL1R2, MMP8 | 0,863 | 0,855 | 0,872 | 0,925 | 0,880 | 0,971 | 0,947 | 0,896 | 0,998 |
| RSAD2, IFI27, OAS1, IFIT1, ISG15, HERC6, SLC1A2, RETN, MMP8 | 0,863 | 0,853 | 0,873 | 0,916 | 0,857 | 0,975 | 0,926 | 0,849 | 1 |
| IFI27, IFIT1, IFI44L, SIGLEC1, ISG15, HERC6, SLC1A2, IL1R2, MMP8 | 0,863 | 0,854 | 0,872 | 0,925 | 0,880 | 0,970 | 0,947 | 0,897 | 0,997 |
| RSAD2, OAS1, IFIT1, IFI44L, SIGLEC1, ISG15, OLAH, FAM20A, RETN | 0,863 | 0,853 | 0,873 | 0,905 | 0,835 | 0,975 | 0,947 | 0,891 | 1 |
| RSAD2, IFIT1, IFI44L, SIGLEC1, ISG15, OLAH, FAM20A, RETN, MMP8 | 0,863 | 0,853 | 0,873 | 0,914 | 0,850 | 0,977 | 0,957 | 0,907 | 1 |
| RSAD2, IFI27, IFIT1, IFI44L, SIGLEC1, HERC6, SLC1A2, IL1R2, MMP8 | 0,863 | 0,854 | 0,872 | 0,925 | 0,879 | 0,971 | 0,949 | 0,901 | 0,996 |

(suite)

| Modèle | AUC modèle | IC 95% | | AUC Train | IC 95% Train | | AUC Test | IC 95% Test | |
|---|---|---|---|---|---|---|---|---|---|
| IFI44L, SIGLEC1, ISG15, HERC6, SLC1A2, OLAH, FAM20A, RETN, MMP8 | 0,863 | 0,853 | 0,873 | 0,918 | 0,861 | 0,974 | 0,950 | 0,878 | 1 |
| RSAD2, IFI27, OAS1, IFI44L, SIGLEC1, ISG15, SLC1A2, IL1R2, MMP8 | 0,863 | 0,855 | 0,871 | 0,926 | 0,880 | 0,973 | 0,953 | 0,906 | 1 |
| RSAD2, IFI27, OAS1, IFI44L, ISG15, HERC6, SLC1A2, RETN, MMP8 | 0,863 | 0,853 | 0,873 | 0,916 | 0,857 | 0,974 | 0,928 | 0,851 | 1 |
| RSAD2, IFI27, OAS1, IFIT1, IFI44L, SIGLEC1, SLC1A2, IL1R2, MMP8 | 0,863 | 0,854 | 0,872 | 0,925 | 0,879 | 0,972 | 0,951 | 0,900 | 1 |
| RSAD2, OAS1, IFIT1, IFI44L, SIGLEC1, ISG15, SLC1A2, IL1R2, FAM20A | 0,863 | 0,853 | 0,872 | 0,921 | 0,869 | 0,972 | 0,954 | 0,895 | 1 |
| OAS1, IFI44L, SIGLEC1, ISG15, HERC6, SLC1A2, IL1R2, OLAH, FAM20A | 0,863 | 0,852 | 0,873 | 0,919 | 0,868 | 0,971 | 0,946 | 0,878 | 1 |
| OAS1, IFI44L, SIGLEC1, ISG15, SLC1A2, OLAH, FAM20A, RETN, MMP8 | 0,863 | 0,852 | 0,873 | 0,912 | 0,856 | 0,969 | 0,946 | 0,880 | 1 |
| RSAD2, OAS1, SIGLEC1, ISG15, SLC1A2, OLAH, FAM20A, RETN, MMP8 | 0,863 | 0,852 | 0,873 | 0,917 | 0,862 | 0,972 | 0,950 | 0,886 | 1 |
| RSAD2, OAS1, SIGLEC1, ISG15, HERC6, SLC1A2, FAM20A, RETN, MMP8 | 0,863 | 0,852 | 0,873 | 0,915 | 0,858 | 0,972 | 0,948 | 0,877 | 1 |
| RSAD2, IFI44L, SIGLEC1, ISG15, HERC6, SLC1A2, IL1R2, OLAH, FAM20A | 0,863 | 0,852 | 0,873 | 0,919 | 0,866 | 0,971 | 0,946 | 0,873 | 1 |
| RSAD2, OAS1, IFIT1, SIGLEC1, ISG15, SLC1A2, OLAH, FAM20A, RETN | 0,862 | 0,853 | 0,872 | 0,919 | 0,865 | 0,974 | 0,946 | 0,882 | 1 |
| RSAD2, OAS1, IFIT1, SIGLEC1, ISG15, SLC1A2, IL1R2, FAM20A, RETN | 0,862 | 0,853 | 0,872 | 0,922 | 0,870 | 0,973 | 0,957 | 0,895 | 1 |
| RSAD2, IFIT1, IFI44L, SIGLEC1, ISG15, SLC1A2, IL1R2, FAM20A, RETN | 0,862 | 0,853 | 0,872 | 0,920 | 0,869 | 0,971 | 0,954 | 0,892 | 1 |
| RSAD2, IFI27, IFI44L, SIGLEC1, ISG15, HERC6, SLC1A2, IL1R2, MMP8 | 0,862 | 0,854 | 0,871 | 0,926 | 0,880 | 0,971 | 0,950 | 0,903 | 0,997 |
| RSAD2, OAS1, IFI44L, SIGLEC1, HERC6, IL1R2, OLAH, FAM20A, RETN | 0,862 | 0,852 | 0,873 | 0,913 | 0,853 | 0,972 | 0,960 | 0,906 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, HERC6, SLC1A2, OLAH, FAM20A, RETN, MMP8 | 0,862 | 0,852 | 0,873 | 0,919 | 0,865 | 0,974 | 0,943 | 0,875 | 1 |
| RSAD2, OAS1, IFIT1, SIGLEC1, ISG15, HERC6, SLC1A2, FAM20A, MMP8 | 0,862 | 0,852 | 0,872 | 0,918 | 0,864 | 0,971 | 0,937 | 0,860 | 1 |
| RSAD2, IFI27, IFIT1, ISG15, HERC6, SLC1A2, IL1R2, RETN, MMP8 | 0,862 | 0,853 | 0,871 | 0,924 | 0,874 | 0,974 | 0,943 | 0,885 | 1 |
| OAS1, IFI44L, SIGLEC1, ISG15, HERC6, IL1R2, OLAH, FAM20A, RETN | 0,862 | 0,852 | 0,872 | 0,914 | 0,854 | 0,973 | 0,961 | 0,908 | 1 |
| RSAD2, IFI27, IFIT1, IFI44L, SIGLEC1, ISG15, HERC6, SLC1A2, IL1R2 | 0,862 | 0,854 | 0,871 | 0,925 | 0,879 | 0,971 | 0,947 | 0,897 | 0,996 |
| RSAD2, IFI27, IFIT1, IFI44L, SIGLEC1, HERC6, SLC1A2, RETN, MMP8 | 0,862 | 0,853 | 0,871 | 0,923 | 0,871 | 0,976 | 0,941 | 0,885 | 0,998 |
| RSAD2, OAS1, IFI44L, SIGLEC1, ISG15, HERC6, SLC1A2, IL1R2, FAM20A | 0,862 | 0,851 | 0,872 | 0,920 | 0,870 | 0,970 | 0,952 | 0,884 | 1 |
| RSAD2, IFI27, OAS1, IFIT1, IFI44L, HERC6, SLC1A2, RETN, MMP8 | 0,862 | 0,852 | 0,872 | 0,914 | 0,855 | 0,974 | 0,926 | 0,847 | 1 |
| RSAD2, IFI44L, SIGLEC1, ISG15, HERC6, IL1R2, OLAH, FAM20A, RETN | 0,862 | 0,852 | 0,872 | 0,914 | 0,854 | 0,973 | 0,958 | 0,901 | 1 |
| RSAD2, IFI27, IFIT1, IFI44L, HERC6, SLC1A2, IL1R2, RETN, MMP8 | 0,862 | 0,853 | 0,871 | 0,923 | 0,872 | 0,974 | 0,941 | 0,880 | 1 |
| RSAD2, IFI27, IFIT1, SIGLEC1, ISG15, HERC6, SLC1A2, RETN, MMP8 | 0,862 | 0,853 | 0,871 | 0,923 | 0,870 | 0,976 | 0,943 | 0,888 | 0,999 |
| RSAD2, IFI27, IFIT1, SIGLEC1, ISG15, HERC6, SLC1A2, IL1R2, MMP8 | 0,862 | 0,853 | 0,870 | 0,924 | 0,879 | 0,970 | 0,949 | 0,900 | 0,998 |
| OAS1, IFI44L, SIGLEC1, ISG15, HERC6, OLAH, FAM20A, RETN, MMP8 | 0,862 | 0,851 | 0,872 | 0,914 | 0,853 | 0,975 | 0,958 | 0,904 | 1 |
| RSAD2, IFI27, OAS1, IFIT1, SIGLEC1, ISG15, SLC1A2, IL1R2, MMP8 | 0,862 | 0,853 | 0,870 | 0,925 | 0,879 | 0,971 | 0,954 | 0,905 | 1 |
| RSAD2, OAS1, IFIT1, IFI44L, SIGLEC1, SLC1A2, IL1R2, FAM20A, RETN | 0,861 | 0,852 | 0,871 | 0,921 | 0,869 | 0,972 | 0,953 | 0,894 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, HERC6, FAM20A, RETN, MMP8 | 0,861 | 0,852 | 0,871 | 0,913 | 0,852 | 0,974 | 0,950 | 0,887 | 1 |
| RSAD2, IFI27, OAS1, IFI44L, SIGLEC1, ISG15, SLC1A2, FAM20A, MMP8 | 0,861 | 0,850 | 0,872 | 0,916 | 0,857 | 0,975 | 0,948 | 0,877 | 1 |
| RSAD2, OAS1, IFI44L, SIGLEC1, HERC6, OLAH, FAM20A, RETN, MMP8 | 0,861 | 0,851 | 0,872 | 0,915 | 0,855 | 0,975 | 0,951 | 0,893 | 1 |
| RSAD2, IFI44L, SIGLEC1, ISG15, SLC1A2, OLAH, FAM20A, RETN, MMP8 | 0,861 | 0,851 | 0,872 | 0,914 | 0,858 | 0,971 | 0,945 | 0,881 | 1 |
| IFI44L, SIGLEC1, ISG15, HERC6, SLC1A2, IL1R2, OLAH, FAM20A, RETN | 0,861 | 0,851 | 0,872 | 0,917 | 0,864 | 0,970 | 0,951 | 0,879 | 1 |
| IFI27, IFIT1, IFI44L, SIGLEC1, ISG15, HERC6, SLC1A2, RETN, MMP8 | 0,861 | 0,852 | 0,870 | 0,920 | 0,866 | 0,974 | 0,942 | 0,886 | 0,999 |
| RSAD2, OAS1, IFI44L, SIGLEC1, HERC6, SLC1A2, IL1R2, OLAH, FAM20A | 0,861 | 0,850 | 0,872 | 0,919 | 0,867 | 0,970 | 0,946 | 0,878 | 1 |
| IFI27, IFIT1, IFI44L, ISG15, HERC6, SLC1A2, IL1R2, RETN, MMP8 | 0,861 | 0,852 | 0,870 | 0,923 | 0,871 | 0,974 | 0,940 | 0,878 | 1 |
| RSAD2, IFI27, IFIT1, IFI44L, SIGLEC1, ISG15, SLC1A2, IL1R2, MMP8 | 0,861 | 0,853 | 0,869 | 0,925 | 0,879 | 0,971 | 0,950 | 0,902 | 0,998 |
| RSAD2, OAS1, SIGLEC1, ISG15, SLC1A2, IL1R2, OLAH, FAM20A, RETN | 0,861 | 0,851 | 0,871 | 0,918 | 0,865 | 0,970 | 0,958 | 0,900 | 1 |
| RSAD2, OAS1, IFI44L, SIGLEC1, ISG15, HERC6, OLAH, FAM20A, MMP8 | 0,861 | 0,851 | 0,872 | 0,910 | 0,850 | 0,970 | 0,953 | 0,898 | 1 |
| RSAD2, OAS1, SIGLEC1, HERC6, SLC1A2, IL1R2, OLAH, FAM20A, RETN | 0,861 | 0,850 | 0,872 | 0,919 | 0,868 | 0,970 | 0,949 | 0,883 | 1 |
| RSAD2, IFI27, IFIT1, IFI44L, ISG15, HERC6, SLC1A2, IL1R2, RETN | 0,861 | 0,852 | 0,870 | 0,923 | 0,872 | 0,973 | 0,934 | 0,869 | 0,999 |
| RSAD2, SIGLEC1, ISG15, HERC6, SLC1A2, IL1R2, OLAH, FAM20A, RETN | 0,861 | 0,851 | 0,872 | 0,919 | 0,868 | 0,970 | 0,950 | 0,879 | 1 |
| OAS1, SIGLEC1, ISG15, HERC6, SLC1A2, IL1R2, OLAH, FAM20A, RETN | 0,861 | 0,850 | 0,872 | 0,920 | 0,870 | 0,971 | 0,949 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, HERC6, IL1R2, FAM20A, RETN | 0,861 | 0,851 | 0,871 | 0,914 | 0,854 | 0,973 | 0,959 | 0,904 | 1 |
| RSAD2, IFI27, IFI44L, ISG15, HERC6, SLC1A2, IL1R2, RETN, MMP8 | 0,861 | 0,852 | 0,870 | 0,924 | 0,874 | 0,974 | 0,935 | 0,868 | 1 |
| RSAD2, OAS1, IFI44L, SIGLEC1, ISG15, IL1R2, OLAH, FAM20A, RETN | 0,861 | 0,850 | 0,871 | 0,905 | 0,841 | 0,969 | 0,962 | 0,907 | 1 |
| RSAD2, IFI44L, SIGLEC1, ISG15, HERC6, OLAH, FAM20A, RETN, MMP8 | 0,861 | 0,851 | 0,871 | 0,916 | 0,857 | 0,976 | 0,952 | 0,895 | 1 |
| RSAD2, OAS1, IFI44L, SIGLEC1, ISG15, HERC6, SLC1A2, OLAH, FAM20A | 0,861 | 0,850 | 0,871 | 0,918 | 0,863 | 0,972 | 0,942 | 0,873 | 1 |
| RSAD2, OAS1, IFI44L, SIGLEC1, SLC1A2, OLAH, FAM20A, RETN, MMP8 | 0,861 | 0,850 | 0,871 | 0,915 | 0,858 | 0,971 | 0,947 | 0,884 | 1 |
| RSAD2, IFI27, OAS1, IFIT1, IFI44L, SLC1A2, IL1R2, RETN, MMP8 | 0,860 | 0,851 | 0,870 | 0,919 | 0,867 | 0,972 | 0,938 | 0,872 | 1 |
| RSAD2, IFI27, OAS1, IFIT1, IFI44L, ISG15, HERC6, SLC1A2, IL1R2 | 0,860 | 0,851 | 0,869 | 0,922 | 0,875 | 0,968 | 0,936 | 0,873 | 0,999 |
| RSAD2, OAS1, SIGLEC1, ISG15, HERC6, SLC1A2, IL1R2, FAM20A, RETN | 0,860 | 0,850 | 0,871 | 0,918 | 0,868 | 0,969 | 0,952 | 0,884 | 1 |
| RSAD2, IFI44L, SIGLEC1, ISG15, SLC1A2, IL1R2, OLAH, FAM20A, RETN | 0,860 | 0,850 | 0,870 | 0,917 | 0,864 | 0,969 | 0,954 | 0,891 | 1 |
| RSAD2, IFI27, OAS1, IFIT1, ISG15, SLC1A2, IL1R2, RETN, MMP8 | 0,860 | 0,851 | 0,869 | 0,919 | 0,867 | 0,972 | 0,942 | 0,879 | 1 |
| OAS1, IFI44L, SIGLEC1, ISG15, HERC6, SLC1A2, IL1R2, FAM20A, RETN | 0,860 | 0,849 | 0,871 | 0,920 | 0,869 | 0,970 | 0,949 | 0,880 | 1 |
| RSAD2, IFI27, OAS1, IFIT1, IFI44L, HERC6, SLC1A2, IL1R2, MMP8 | 0,860 | 0,851 | 0,869 | 0,922 | 0,875 | 0,969 | 0,935 | 0,870 | 1,000 |
| RSAD2, IFI27, OAS1, IFIT1, IFI44L, ISG15, SLC1A2, IL1R2, RETN | 0,860 | 0,851 | 0,869 | 0,920 | 0,868 | 0,972 | 0,934 | 0,862 | 1 |
| IFI27, OAS1, IFIT1, IFI44L, ISG15, SLC1A2, IL1R2, RETN, MMP8 | 0,860 | 0,850 | 0,870 | 0,921 | 0,870 | 0,972 | 0,940 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, HERC6, SLC1A2, IL1R2, MMP8 | 0,860 | 0,851 | 0,869 | 0,923 | 0,876 | 0,969 | 0,937 | 0,873 | 1 |
| OAS1, IFI44L, SIGLEC1, ISG15, SLC1A2, IL1R2, OLAH, FAM20A, RETN | 0,860 | 0,849 | 0,870 | 0,918 | 0,866 | 0,970 | 0,955 | 0,894 | 1 |
| RSAD2, IFI27, OAS1, IFIT1, IFI44L, SIGLEC1, ISG15, RETN, MMP8 | 0,860 | 0,849 | 0,870 | 0,909 | 0,847 | 0,971 | 0,952 | 0,889 | 1 |
| RSAD2, IFI27, OAS1, IFIT1, IFI44L, SIGLEC1, ISG15, SLC1A2, RETN | 0,860 | 0,851 | 0,868 | 0,915 | 0,857 | 0,973 | 0,946 | 0,876 | 1 |
| RSAD2, OAS1, SIGLEC1, ISG15, HERC6, SLC1A2, OLAH, FAM20A, RETN | 0,859 | 0,849 | 0,870 | 0,915 | 0,860 | 0,970 | 0,943 | 0,875 | 1 |
| RSAD2, OAS1, IFIT1, IFI44L, SIGLEC1, ISG15, FAM20A, RETN, MMP8 | 0,859 | 0,849 | 0,870 | 0,910 | 0,845 | 0,976 | 0,941 | 0,874 | 1 |
| RSAD2, IFI27, IFI44L, SIGLEC1, ISG15, HERC6, SLC1A2, RETN, MMP8 | 0,859 | 0,850 | 0,869 | 0,921 | 0,867 | 0,974 | 0,943 | 0,887 | 1 |
| OAS1, IFI44L, SIGLEC1, HERC6, SLC1A2, IL1R2, OLAH, FAM20A, RETN | 0,859 | 0,848 | 0,870 | 0,920 | 0,870 | 0,971 | 0,948 | 0,880 | 1 |
| OAS1, IFIT1, IFI44L, SIGLEC1, ISG15, SLC1A2, FAM20A, RETN, MMP8 | 0,859 | 0,849 | 0,870 | 0,918 | 0,862 | 0,974 | 0,938 | 0,865 | 1 |
| RSAD2, OAS1, IFI44L, SIGLEC1, ISG15, SLC1A2, IL1R2, OLAH, FAM20A | 0,859 | 0,849 | 0,869 | 0,917 | 0,863 | 0,971 | 0,955 | 0,896 | 1 |
| RSAD2, OAS1, IFIT1, IFI44L, HERC6, IL1R2, OLAH, FAM20A, MMP8 | 0,859 | 0,849 | 0,869 | 0,912 | 0,854 | 0,970 | 0,936 | 0,860 | 1 |
| RSAD2, IFI27, IFIT1, IFI44L, ISG15, SLC1A2, IL1R2, RETN, MMP8 | 0,859 | 0,850 | 0,868 | 0,922 | 0,871 | 0,973 | 0,940 | 0,880 | 1 |
| IFIT1, IFI44L, ISG15, HERC6, SLC1A2, IL1R2, OLAH, FAM20A, MMP8 | 0,859 | 0,850 | 0,869 | 0,917 | 0,866 | 0,968 | 0,929 | 0,847 | 1 |
| RSAD2, IFI27, OAS1, IFIT1, IFI44L, ISG15, FAM20A, RETN, MMP8 | 0,859 | 0,848 | 0,871 | 0,916 | 0,854 | 0,978 | 0,947 | 0,873 | 1 |
| RSAD2, OAS1, IFI44L, SIGLEC1, HERC6, SLC1A2, IL1R2, FAM20A, RETN | 0,859 | 0,849 | 0,869 | 0,920 | 0,869 | 0,970 | 0,952 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, HERC6, SLC1A2, IL1R2, OLAH, FAM20A, MMP8 | 0,859 | 0,848 | 0,870 | 0,920 | 0,870 | 0,970 | 0,927 | 0,845 | 1 |
| RSAD2, IFI44L, SIGLEC1, ISG15, HERC6, SLC1A2, IL1R2, FAM20A, RETN | 0,859 | 0,848 | 0,869 | 0,918 | 0,866 | 0,969 | 0,948 | 0,875 | 1 |
| OAS1, IFI44L, SIGLEC1, ISG15, HERC6, SLC1A2, OLAH, FAM20A, RETN | 0,859 | 0,848 | 0,869 | 0,916 | 0,862 | 0,971 | 0,941 | 0,872 | 1 |
| RSAD2, OAS1, IFI44L, SIGLEC1, SLC1A2, IL1R2, OLAH, FAM20A, RETN | 0,859 | 0,848 | 0,869 | 0,917 | 0,863 | 0,970 | 0,958 | 0,900 | 1 |
| IFI27, OAS1, IFIT1, IFI44L, SIGLEC1, ISG15, SLC1A2, RETN, MMP8 | 0,859 | 0,849 | 0,868 | 0,916 | 0,859 | 0,972 | 0,950 | 0,885 | 1 |
| RSAD2, IFI44L, SIGLEC1, HERC6, SLC1A2, IL1R2, OLAH, FAM20A, RETN | 0,859 | 0,847 | 0,870 | 0,920 | 0,869 | 0,971 | 0,947 | 0,877 | 1 |
| RSAD2, OAS1, IFIT1, IFI44L, SIGLEC1, ISG15, SLC1A2, FAM20A, MMP8 | 0,859 | 0,848 | 0,869 | 0,913 | 0,855 | 0,971 | 0,936 | 0,861 | 1 |
| RSAD2, OAS1, IFI44L, SIGLEC1, ISG15, OLAH, FAM20A, RETN, MMP8 | 0,859 | 0,848 | 0,869 | 0,908 | 0,843 | 0,973 | 0,965 | 0,922 | 1 |
| IFI27, OAS1, IFIT1, IFI44L, ISG15, HERC6, SLC1A2, FAM20A, MMP8 | 0,859 | 0,848 | 0,870 | 0,915 | 0,861 | 0,970 | 0,910 | 0,820 | 1,000 |
| RSAD2, IFI27, OAS1, IFI44L, ISG15, HERC6, SLC1A2, IL1R2, MMP8 | 0,859 | 0,849 | 0,868 | 0,923 | 0,877 | 0,970 | 0,928 | 0,858 | 0,999 |
| OAS1, IFI44L, SIGLEC1, ISG15, HERC6, SLC1A2, FAM20A, RETN, MMP8 | 0,859 | 0,848 | 0,869 | 0,914 | 0,856 | 0,972 | 0,950 | 0,883 | 1 |
| RSAD2, IFI44L, SIGLEC1, ISG15, HERC6, SLC1A2, FAM20A, RETN, MMP8 | 0,859 | 0,848 | 0,869 | 0,915 | 0,858 | 0,973 | 0,948 | 0,875 | 1 |
| RSAD2, OAS1, IFIT1, IFI44L, HERC6, SLC1A2, OLAH, FAM20A, MMP8 | 0,858 | 0,848 | 0,869 | 0,918 | 0,866 | 0,970 | 0,918 | 0,836 | 1 |
| OAS1, IFIT1, IFI44L, HERC6, SLC1A2, OLAH, FAM20A, RETN, MMP8 | 0,858 | 0,848 | 0,869 | 0,920 | 0,867 | 0,974 | 0,917 | 0,830 | 1 |
| RSAD2, IFIT1, IFI44L, SIGLEC1, ISG15, SLC1A2, FAM20A, RETN, MMP8 | 0,858 | 0,848 | 0,868 | 0,917 | 0,860 | 0,973 | 0,937 | 0,865 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, SLC1A2, IL1R2, MMP8 | 0,858 | 0,849 | 0,868 | 0,923 | 0,878 | 0,969 | 0,936 | 0,875 | 0,997 |
| RSAD2, OAS1, ISG15, HERC6, IL1R2, OLAH, FAM20A, RETN, MMP8 | 0,858 | 0,848 | 0,869 | 0,911 | 0,852 | 0,970 | 0,942 | 0,867 | 1 |
| RSAD2, OAS1, IFI44L, ISG15, HERC6, IL1R2, OLAH, FAM20A, MMP8 | 0,858 | 0,847 | 0,869 | 0,912 | 0,855 | 0,970 | 0,933 | 0,855 | 1 |
| RSAD2, OAS1, IFI44L, SIGLEC1, ISG15, SLC1A2, IL1R2, FAM20A, RETN | 0,858 | 0,848 | 0,869 | 0,915 | 0,861 | 0,968 | 0,954 | 0,888 | 1 |
| RSAD2, IFIT1, IFI44L, HERC6, SLC1A2, IL1R2, OLAH, FAM20A, MMP8 | 0,858 | 0,848 | 0,868 | 0,919 | 0,869 | 0,970 | 0,926 | 0,844 | 1 |
| OAS1, IFIT1, IFI44L, ISG15, HERC6, SLC1A2, OLAH, FAM20A, MMP8 | 0,858 | 0,847 | 0,869 | 0,918 | 0,865 | 0,970 | 0,916 | 0,830 | 1 |
| IFIT1, IFI44L, HERC6, SLC1A2, IL1R2, OLAH, FAM20A, RETN, MMP8 | 0,858 | 0,848 | 0,868 | 0,920 | 0,869 | 0,972 | 0,929 | 0,845 | 1 |
| IFI44L, ISG15, HERC6, SLC1A2, IL1R2, OLAH, FAM20A, RETN, MMP8 | 0,858 | 0,848 | 0,868 | 0,919 | 0,867 | 0,971 | 0,930 | 0,847 | 1 |
| RSAD2, IFI27, IFIT1, IFI44L, ISG15, HERC6, SLC1A2, FAM20A, MMP8 | 0,858 | 0,847 | 0,869 | 0,916 | 0,861 | 0,972 | 0,912 | 0,823 | 1 |
| IFIT1, IFI44L, ISG15, HERC6, SLC1A2, OLAH, FAM20A, RETN, MMP8 | 0,858 | 0,847 | 0,868 | 0,919 | 0,864 | 0,973 | 0,924 | 0,842 | 1 |
| RSAD2, IFI27, OAS1, IFI44L, ISG15, SLC1A2, IL1R2, RETN, MMP8 | 0,858 | 0,849 | 0,866 | 0,924 | 0,874 | 0,974 | 0,934 | 0,863 | 1 |
| OAS1, IFIT1, IFI44L, ISG15, HERC6, SLC1A2, OLAH, FAM20A, RETN | 0,858 | 0,847 | 0,868 | 0,917 | 0,864 | 0,970 | 0,917 | 0,831 | 1 |
| RSAD2, OAS1, IFI44L, SIGLEC1, HERC6, SLC1A2, FAM20A, RETN, MMP8 | 0,858 | 0,847 | 0,868 | 0,913 | 0,855 | 0,971 | 0,949 | 0,878 | 1 |
| RSAD2, OAS1, IFI44L, SIGLEC1, HERC6, SLC1A2, OLAH, FAM20A, RETN | 0,857 | 0,847 | 0,868 | 0,917 | 0,863 | 0,972 | 0,941 | 0,872 | 1 |
| RSAD2, IFI27, OAS1, IFIT1, IFI44L, SIGLEC1, SLC1A2, RETN, MMP8 | 0,857 | 0,848 | 0,867 | 0,916 | 0,859 | 0,974 | 0,948 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, ISG15, SLC1A2, RETN, MMP8 | 0,857 | 0,848 | 0,867 | 0,915 | 0,857 | 0,973 | 0,949 | 0,880 | 1 |
| OAS1, IFI44L, ISG15, HERC6, SLC1A2, IL1R2, OLAH, FAM20A, MMP8 | 0,857 | 0,846 | 0,868 | 0,917 | 0,865 | 0,969 | 0,929 | 0,847 | 1 |
| RSAD2, IFI27, OAS1, IFIT1, IFI44L, HERC6, SLC1A2, FAM20A, MMP8 | 0,857 | 0,847 | 0,868 | 0,917 | 0,864 | 0,971 | 0,908 | 0,817 | 0,998 |
| RSAD2, OAS1, IFI44L, SIGLEC1, ISG15, HERC6, OLAH, FAM20A, RETN | 0,857 | 0,847 | 0,867 | 0,908 | 0,846 | 0,970 | 0,951 | 0,896 | 1 |
| OAS1, IFIT1, IFI44L, HERC6, SLC1A2, IL1R2, OLAH, FAM20A, MMP8 | 0,857 | 0,847 | 0,868 | 0,918 | 0,866 | 0,969 | 0,926 | 0,840 | 1 |
| RSAD2, IFIT1, IFI44L, HERC6, SLC1A2, OLAH, FAM20A, RETN, MMP8 | 0,857 | 0,847 | 0,867 | 0,919 | 0,866 | 0,972 | 0,922 | 0,838 | 1 |
| RSAD2, IFI27, OAS1, IFIT1, IFI44L, SIGLEC1, HERC6, SLC1A2, MMP8 | 0,857 | 0,848 | 0,866 | 0,916 | 0,863 | 0,969 | 0,921 | 0,848 | 0,994 |
| OAS1, IFIT1, SIGLEC1, ISG15, HERC6, SLC1A2, OLAH, RETN, MMP8 | 0,857 | 0,847 | 0,867 | 0,915 | 0,861 | 0,970 | 0,955 | 0,911 | 1 |
| RSAD2, IFI27, OAS1, IFIT1, IFI44L, ISG15, SLC1A2, IL1R2, MMP8 | 0,857 | 0,848 | 0,866 | 0,922 | 0,875 | 0,969 | 0,938 | 0,874 | 1 |
| RSAD2, OAS1, IFIT1, IFI44L, SIGLEC1, HERC6, IL1R2, RETN, MMP8 | 0,857 | 0,847 | 0,867 | 0,904 | 0,842 | 0,966 | 0,964 | 0,927 | 1 |
| OAS1, IFIT1, SIGLEC1, ISG15, HERC6, IL1R2, OLAH, RETN, MMP8 | 0,857 | 0,847 | 0,867 | 0,907 | 0,847 | 0,967 | 0,963 | 0,925 | 1 |
| RSAD2, OAS1, IFI44L, SIGLEC1, ISG15, SLC1A2, OLAH, FAM20A, RETN | 0,857 | 0,846 | 0,867 | 0,915 | 0,858 | 0,973 | 0,947 | 0,885 | 1 |
| RSAD2, OAS1, IFIT1, SIGLEC1, ISG15, HERC6, IL1R2, RETN, MMP8 | 0,857 | 0,847 | 0,867 | 0,906 | 0,845 | 0,966 | 0,967 | 0,932 | 1 |
| RSAD2, IFI27, OAS1, IFIT1, SIGLEC1, ISG15, HERC6, SLC1A2, MMP8 | 0,857 | 0,848 | 0,866 | 0,916 | 0,864 | 0,968 | 0,912 | 0,837 | 0,987 |
| RSAD2, IFIT1, IFI44L, ISG15, HERC6, SLC1A2, OLAH, FAM20A, MMP8 | 0,857 | 0,846 | 0,867 | 0,918 | 0,865 | 0,970 | 0,917 | 0,834 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, ISG15, HERC6, IL1R2, FAM20A, RETN, MMP8 | 0,857 | 0,847 | 0,867 | 0,913 | 0,854 | 0,971 | 0,939 | 0,862 | 1 |
| RSAD2, OAS1, IFIT1, IFI44L, SIGLEC1, SLC1A2, FAM20A, RETN, MMP8 | 0,857 | 0,846 | 0,867 | 0,919 | 0,863 | 0,975 | 0,938 | 0,867 | 1 |
| RSAD2, OAS1, IFI44L, HERC6, IL1R2, OLAH, FAM20A, RETN, MMP8 | 0,857 | 0,846 | 0,867 | 0,914 | 0,855 | 0,972 | 0,938 | 0,864 | 1 |
| OAS1, IFIT1, ISG15, HERC6, IL1R2, OLAH, FAM20A, RETN, MMP8 | 0,857 | 0,847 | 0,867 | 0,909 | 0,848 | 0,969 | 0,943 | 0,871 | 1 |
| RSAD2, IFI27, OAS1, IFI44L, SIGLEC1, ISG15, SLC1A2, RETN, MMP8 | 0,857 | 0,847 | 0,866 | 0,916 | 0,858 | 0,974 | 0,948 | 0,876 | 1 |
| RSAD2, IFI44L, SIGLEC1, ISG15, HERC6, SLC1A2, OLAH, FAM20A, RETN | 0,857 | 0,846 | 0,867 | 0,916 | 0,861 | 0,971 | 0,938 | 0,867 | 1 |
| OAS1, IFIT1, IFI44L, SIGLEC1, ISG15, HERC6, IL1R2, RETN, MMP8 | 0,857 | 0,847 | 0,867 | 0,907 | 0,847 | 0,967 | 0,962 | 0,923 | 1 |
| OAS1, IFIT1, IFI44L, ISG15, HERC6, SLC1A2, IL1R2, OLAH, FAM20A | 0,857 | 0,846 | 0,867 | 0,918 | 0,866 | 0,969 | 0,921 | 0,834 | 1 |
| RSAD2, IFI44L, HERC6, SLC1A2, IL1R2, OLAH, FAM20A, RETN, MMP8 | 0,856 | 0,846 | 0,867 | 0,919 | 0,867 | 0,971 | 0,929 | 0,847 | 1 |
| OAS1, IFIT1, IFI44L, SIGLEC1, HERC6, SLC1A2, OLAH, RETN, MMP8 | 0,856 | 0,846 | 0,867 | 0,916 | 0,862 | 0,971 | 0,951 | 0,904 | 0,999 |
| RSAD2, OAS1, IFIT1, ISG15, HERC6, IL1R2, OLAH, FAM20A, MMP8 | 0,856 | 0,846 | 0,867 | 0,910 | 0,850 | 0,969 | 0,941 | 0,867 | 1 |
| OAS1, IFIT1, IFI44L, SIGLEC1, HERC6, IL1R2, OLAH, RETN, MMP8 | 0,856 | 0,846 | 0,866 | 0,904 | 0,841 | 0,967 | 0,964 | 0,926 | 1 |
| OAS1, IFIT1, SIGLEC1, HERC6, SLC1A2, IL1R2, OLAH, RETN, MMP8 | 0,856 | 0,846 | 0,867 | 0,915 | 0,861 | 0,969 | 0,958 | 0,916 | 0,999 |
| RSAD2, OAS1, IFIT1, IFI44L, ISG15, HERC6, IL1R2, OLAH, FAM20A | 0,856 | 0,846 | 0,867 | 0,911 | 0,853 | 0,968 | 0,932 | 0,855 | 1 |
| RSAD2, OAS1, IFIT1, IFI44L, SLC1A2, IL1R2, OLAH, FAM20A, MMP8 | 0,856 | 0,846 | 0,866 | 0,917 | 0,865 | 0,969 | 0,933 | 0,852 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, ISG15, HERC6, IL1R2, OLAH, FAM20A, MMP8 | 0,856 | 0,846 | 0,867 | 0,909 | 0,850 | 0,968 | 0,937 | 0,860 | 1 |
| OAS1, IFI44L, ISG15, HERC6, SLC1A2, OLAH, FAM20A, RETN, MMP8 | 0,856 | 0,846 | 0,867 | 0,919 | 0,864 | 0,974 | 0,930 | 0,852 | 1 |
| RSAD2, IFI27, IFIT1, IFI44L, ISG15, HERC6, SLC1A2, IL1R2, MMP8 | 0,856 | 0,847 | 0,865 | 0,923 | 0,878 | 0,969 | 0,935 | 0,875 | 0,995 |
| RSAD2, OAS1, IFIT1, HERC6, IL1R2, OLAH, FAM20A, RETN, MMP8 | 0,856 | 0,846 | 0,866 | 0,910 | 0,850 | 0,970 | 0,943 | 0,870 | 1 |
| OAS1, IFIT1, IFI44L, HERC6, IL1R2, OLAH, FAM20A, RETN, MMP8 | 0,856 | 0,846 | 0,866 | 0,911 | 0,851 | 0,971 | 0,942 | 0,869 | 1 |
| IFIT1, IFI44L, ISG15, HERC6, IL1R2, OLAH, FAM20A, RETN, MMP8 | 0,856 | 0,846 | 0,866 | 0,911 | 0,851 | 0,971 | 0,941 | 0,867 | 1 |
| RSAD2, OAS1, IFIT1, HERC6, SLC1A2, IL1R2, OLAH, FAM20A, MMP8 | 0,856 | 0,846 | 0,866 | 0,917 | 0,866 | 0,968 | 0,933 | 0,852 | 1 |
| RSAD2, IFI27, OAS1, IFIT1, IFI44L, SIGLEC1, ISG15, HERC6, MMP8 | 0,856 | 0,847 | 0,865 | 0,907 | 0,850 | 0,964 | 0,908 | 0,832 | 0,983 |
| RSAD2, OAS1, IFIT1, IFI44L, ISG15, HERC6, SLC1A2, OLAH, FAM20A | 0,856 | 0,845 | 0,867 | 0,916 | 0,862 | 0,969 | 0,915 | 0,831 | 0,999 |
| RSAD2, IFI44L, ISG15, HERC6, SLC1A2, IL1R2, OLAH, FAM20A, MMP8 | 0,856 | 0,845 | 0,867 | 0,918 | 0,867 | 0,969 | 0,925 | 0,842 | 1 |
| RSAD2, OAS1, IFIT1, IFI44L, ISG15, HERC6, IL1R2, FAM20A, MMP8 | 0,856 | 0,846 | 0,866 | 0,912 | 0,854 | 0,970 | 0,932 | 0,849 | 1 |
| RSAD2, OAS1, IFIT1, SIGLEC1, HERC6, IL1R2, OLAH, RETN, MMP8 | 0,856 | 0,846 | 0,866 | 0,905 | 0,843 | 0,967 | 0,964 | 0,927 | 1 |
| RSAD2, IFI27, IFIT1, IFI44L, ISG15, HERC6, SLC1A2, RETN, MMP8 | 0,856 | 0,846 | 0,865 | 0,916 | 0,860 | 0,973 | 0,938 | 0,872 | 1 |
| OAS1, IFI44L, ISG15, HERC6, IL1R2, OLAH, FAM20A, RETN, MMP8 | 0,856 | 0,845 | 0,866 | 0,912 | 0,852 | 0,972 | 0,943 | 0,871 | 1 |
| RSAD2, OAS1, IFI44L, HERC6, SLC1A2, OLAH, FAM20A, RETN, MMP8 | 0,856 | 0,845 | 0,866 | 0,916 | 0,862 | 0,971 | 0,928 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, SIGLEC1, ISG15, SLC1A2, FAM20A, RETN, MMP8 | 0,856 | 0,846 | 0,866 | 0,914 | 0,856 | 0,972 | 0,952 | 0,884 | 1 |
| IFIT1, IFI44L, SIGLEC1, ISG15, HERC6, IL1R2, OLAH, RETN, MMP8 | 0,856 | 0,846 | 0,865 | 0,904 | 0,842 | 0,966 | 0,964 | 0,926 | 1 |
| RSAD2, OAS1, IFI44L, ISG15, HERC6, SLC1A2, IL1R2, OLAH, FAM20A | 0,856 | 0,844 | 0,867 | 0,913 | 0,860 | 0,967 | 0,923 | 0,837 | 1 |
| RSAD2, OAS1, IFIT1, IFI44L, SIGLEC1, ISG15, SLC1A2, FAM20A, RETN | 0,856 | 0,846 | 0,866 | 0,914 | 0,856 | 0,973 | 0,936 | 0,863 | 1 |
| RSAD2, IFI27, OAS1, IFIT1, IFI44L, ISG15, HERC6, SLC1A2, FAM20A | 0,856 | 0,845 | 0,867 | 0,913 | 0,855 | 0,971 | 0,918 | 0,833 | 1 |
| RSAD2, IFI27, OAS1, IFIT1, IFI44L, ISG15, SLC1A2, FAM20A, RETN | 0,856 | 0,845 | 0,866 | 0,911 | 0,850 | 0,973 | 0,940 | 0,861 | 1 |
| RSAD2, OAS1, IFI44L, ISG15, HERC6, IL1R2, FAM20A, RETN, MMP8 | 0,856 | 0,845 | 0,866 | 0,914 | 0,855 | 0,972 | 0,934 | 0,854 | 1 |
| RSAD2, OAS1, IFIT1, IFI44L, HERC6, SLC1A2, IL1R2, OLAH, FAM20A | 0,856 | 0,845 | 0,866 | 0,916 | 0,864 | 0,969 | 0,917 | 0,830 | 1 |
| RSAD2, OAS1, IFI44L, SIGLEC1, ISG15, HERC6, SLC1A2, FAM20A, RETN | 0,856 | 0,845 | 0,866 | 0,913 | 0,856 | 0,970 | 0,941 | 0,870 | 1 |
| OAS1, IFIT1, HERC6, SLC1A2, IL1R2, OLAH, FAM20A, RETN, MMP8 | 0,855 | 0,845 | 0,866 | 0,915 | 0,862 | 0,968 | 0,933 | 0,851 | 1 |
| OAS1, IFIT1, IFI44L, ISG15, HERC6, OLAH, FAM20A, RETN, MMP8 | 0,855 | 0,845 | 0,865 | 0,909 | 0,847 | 0,971 | 0,940 | 0,874 | 1 |
| OAS1, SIGLEC1, ISG15, HERC6, SLC1A2, IL1R2, OLAH, RETN, MMP8 | 0,855 | 0,845 | 0,865 | 0,916 | 0,862 | 0,969 | 0,963 | 0,925 | 1 |
| OAS1, IFIT1, IFI44L, SIGLEC1, ISG15, HERC6, SLC1A2, OLAH, RETN | 0,855 | 0,845 | 0,865 | 0,916 | 0,863 | 0,970 | 0,938 | 0,883 | 0,993 |
| RSAD2, OAS1, IFIT1, IFI44L, SIGLEC1, ISG15, HERC6, IL1R2, RETN | 0,855 | 0,846 | 0,865 | 0,905 | 0,843 | 0,966 | 0,958 | 0,915 | 1 |
| RSAD2, OAS1, IFIT1, SIGLEC1, HERC6, SLC1A2, OLAH, RETN, MMP8 | 0,855 | 0,845 | 0,865 | 0,914 | 0,859 | 0,969 | 0,952 | 0,905 | 1,000 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, ISG15, HERC6, OLAH, RETN, MMP8 | 0,855 | 0,845 | 0,865 | 0,904 | 0,842 | 0,967 | 0,955 | 0,912 | 0,999 |
| IFIT1, SIGLEC1, ISG15, HERC6, SLC1A2, IL1R2, OLAH, RETN, MMP8 | 0,855 | 0,845 | 0,865 | 0,916 | 0,862 | 0,970 | 0,962 | 0,924 | 1 |
| RSAD2, OAS1, IFIT1, IFI44L, HERC6, OLAH, FAM20A, RETN, MMP8 | 0,855 | 0,845 | 0,865 | 0,908 | 0,846 | 0,971 | 0,936 | 0,866 | 1 |
| RSAD2, IFIT1, IFI44L, ISG15, HERC6, SLC1A2, IL1R2, OLAH, FAM20A | 0,855 | 0,845 | 0,866 | 0,917 | 0,865 | 0,969 | 0,920 | 0,834 | 1 |
| RSAD2, IFI44L, ISG15, HERC6, IL1R2, OLAH, FAM20A, RETN, MMP8 | 0,855 | 0,845 | 0,866 | 0,915 | 0,856 | 0,973 | 0,935 | 0,860 | 1 |
| RSAD2, OAS1, IFI44L, ISG15, HERC6, SLC1A2, OLAH, FAM20A, MMP8 | 0,855 | 0,844 | 0,866 | 0,916 | 0,862 | 0,970 | 0,920 | 0,840 | 0,999 |
| RSAD2, IFI27, OAS1, IFI44L, ISG15, HERC6, SLC1A2, FAM20A, MMP8 | 0,855 | 0,844 | 0,867 | 0,913 | 0,856 | 0,970 | 0,922 | 0,838 | 1 |
| RSAD2, IFIT1, ISG15, HERC6, IL1R2, OLAH, FAM20A, RETN, MMP8 | 0,855 | 0,845 | 0,865 | 0,910 | 0,850 | 0,970 | 0,943 | 0,873 | 1 |
| OAS1, IFIT1, IFI44L, ISG15, HERC6, IL1R2, FAM20A, RETN, MMP8 | 0,855 | 0,845 | 0,865 | 0,911 | 0,851 | 0,970 | 0,936 | 0,856 | 1 |
| OAS1, IFIT1, SIGLEC1, ISG15, HERC6, SLC1A2, IL1R2, RETN, MMP8 | 0,855 | 0,845 | 0,865 | 0,912 | 0,858 | 0,966 | 0,963 | 0,924 | 1 |
| RSAD2, OAS1, IFI44L, ISG15, SLC1A2, IL1R2, OLAH, FAM20A, MMP8 | 0,855 | 0,845 | 0,866 | 0,918 | 0,867 | 0,969 | 0,924 | 0,841 | 1 |
| RSAD2, IFIT1, IFI44L, ISG15, HERC6, IL1R2, OLAH, FAM20A, MMP8 | 0,855 | 0,844 | 0,866 | 0,913 | 0,855 | 0,970 | 0,932 | 0,853 | 1 |
| IFIT1, IFI44L, ISG15, HERC6, SLC1A2, IL1R2, OLAH, FAM20A, RETN | 0,855 | 0,845 | 0,865 | 0,915 | 0,862 | 0,968 | 0,921 | 0,835 | 1 |
| IFIT1, IFI44L, ISG15, SLC1A2, IL1R2, OLAH, FAM20A, RETN, MMP8 | 0,855 | 0,845 | 0,865 | 0,915 | 0,862 | 0,969 | 0,932 | 0,853 | 1 |
| OAS1, IFI44L, HERC6, SLC1A2, IL1R2, OLAH, FAM20A, RETN, MMP8 | 0,855 | 0,844 | 0,866 | 0,919 | 0,867 | 0,971 | 0,929 | 0,846 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, HERC6, SLC1A2, IL1R2, OLAH, FAM20A, RETN, MMP8 | 0,855 | 0,845 | 0,865 | 0,914 | 0,861 | 0,967 | 0,929 | 0,843 | 1 |
| RSAD2, OAS1, SIGLEC1, HERC6, SLC1A2, IL1R2, OLAH, RETN, MMP8 | 0,855 | 0,845 | 0,865 | 0,914 | 0,859 | 0,969 | 0,966 | 0,930 | 1 |
| IFI27, OAS1, IFIT1, IFI44L, ISG15, SLC1A2, FAM20A, RETN, MMP8 | 0,855 | 0,844 | 0,866 | 0,913 | 0,852 | 0,974 | 0,938 | 0,858 | 1 |
| RSAD2, OAS1, IFIT1, IFI44L, HERC6, IL1R2, FAM20A, RETN, MMP8 | 0,855 | 0,845 | 0,865 | 0,911 | 0,851 | 0,971 | 0,940 | 0,863 | 1 |
| IFIT1, ISG15, HERC6, SLC1A2, IL1R2, OLAH, FAM20A, RETN, MMP8 | 0,855 | 0,845 | 0,865 | 0,914 | 0,861 | 0,968 | 0,934 | 0,853 | 1 |
| RSAD2, OAS1, SIGLEC1, ISG15, HERC6, IL1R2, OLAH, RETN, MMP8 | 0,855 | 0,844 | 0,866 | 0,907 | 0,846 | 0,968 | 0,968 | 0,934 | 1 |
| RSAD2, IFIT1, IFI44L, ISG15, HERC6, OLAH, FAM20A, RETN, MMP8 | 0,855 | 0,845 | 0,865 | 0,912 | 0,852 | 0,971 | 0,936 | 0,867 | 1 |
| IFIT1, IFI44L, SIGLEC1, ISG15, HERC6, SLC1A2, OLAH, RETN, MMP8 | 0,855 | 0,845 | 0,865 | 0,914 | 0,859 | 0,969 | 0,950 | 0,904 | 0,996 |
| IFI27, OAS1, IFIT1, IFI44L, SIGLEC1, ISG15, HERC6, SLC1A2, MMP8 | 0,855 | 0,846 | 0,864 | 0,913 | 0,861 | 0,965 | 0,915 | 0,843 | 0,987 |
| RSAD2, IFIT1, ISG15, HERC6, SLC1A2, IL1R2, OLAH, FAM20A, MMP8 | 0,855 | 0,845 | 0,865 | 0,916 | 0,864 | 0,967 | 0,932 | 0,850 | 1 |
| OAS1, IFIT1, ISG15, HERC6, SLC1A2, IL1R2, OLAH, FAM20A, MMP8 | 0,855 | 0,844 | 0,865 | 0,916 | 0,865 | 0,968 | 0,933 | 0,852 | 1 |
| RSAD2, IFI27, IFIT1, IFI44L, ISG15, SLC1A2, FAM20A, RETN, MMP8 | 0,855 | 0,844 | 0,866 | 0,913 | 0,851 | 0,974 | 0,939 | 0,860 | 1 |
| RSAD2, OAS1, IFIT1, IFI44L, SIGLEC1, HERC6, SLC1A2, OLAH, RETN | 0,855 | 0,845 | 0,865 | 0,916 | 0,861 | 0,971 | 0,941 | 0,886 | 0,996 |
| RSAD2, IFIT1, IFI44L, ISG15, HERC6, IL1R2, FAM20A, RETN, MMP8 | 0,855 | 0,845 | 0,865 | 0,914 | 0,855 | 0,972 | 0,932 | 0,852 | 1 |
| RSAD2, IFIT1, IFI44L, ISG15, SLC1A2, IL1R2, OLAH, FAM20A, MMP8 | 0,855 | 0,845 | 0,864 | 0,915 | 0,863 | 0,968 | 0,929 | 0,849 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, HERC6, SLC1A2, FAM20A, MMP8 | 0,855 | 0,844 | 0,865 | 0,911 | 0,854 | 0,968 | 0,938 | 0,863 | 1 |
| RSAD2, IFIT1, SIGLEC1, ISG15, HERC6, IL1R2, OLAH, RETN, MMP8 | 0,855 | 0,845 | 0,865 | 0,906 | 0,845 | 0,967 | 0,964 | 0,926 | 1 |
| RSAD2, OAS1, ISG15, HERC6, SLC1A2, IL1R2, OLAH, FAM20A, MMP8 | 0,855 | 0,844 | 0,865 | 0,914 | 0,861 | 0,966 | 0,927 | 0,840 | 1 |
| RSAD2, OAS1, IFIT1, ISG15, HERC6, IL1R2, OLAH, FAM20A, RETN | 0,855 | 0,844 | 0,865 | 0,906 | 0,845 | 0,966 | 0,937 | 0,862 | 1 |
| OAS1, IFIT1, IFI44L, ISG15, HERC6, IL1R2, OLAH, FAM20A, RETN | 0,855 | 0,845 | 0,864 | 0,907 | 0,848 | 0,967 | 0,932 | 0,854 | 1 |
| OAS1, ISG15, HERC6, SLC1A2, IL1R2, OLAH, FAM20A, RETN, MMP8 | 0,854 | 0,844 | 0,865 | 0,916 | 0,863 | 0,969 | 0,929 | 0,844 | 1 |
| RSAD2, OAS1, IFI44L, ISG15, HERC6, IL1R2, OLAH, FAM20A, RETN | 0,854 | 0,844 | 0,865 | 0,910 | 0,851 | 0,969 | 0,937 | 0,864 | 1 |
| OAS1, IFIT1, SIGLEC1, ISG15, HERC6, SLC1A2, IL1R2, OLAH, RETN | 0,854 | 0,844 | 0,865 | 0,914 | 0,860 | 0,968 | 0,950 | 0,903 | 0,997 |
| RSAD2, OAS1, IFIT1, IFI44L, ISG15, IL1R2, OLAH, FAM20A, MMP8 | 0,854 | 0,844 | 0,865 | 0,908 | 0,847 | 0,969 | 0,945 | 0,876 | 1 |
| RSAD2, OAS1, IFIT1, SIGLEC1, ISG15, HERC6, IL1R2, OLAH, RETN | 0,854 | 0,844 | 0,864 | 0,907 | 0,846 | 0,967 | 0,958 | 0,915 | 1 |
| RSAD2, OAS1, IFIT1, IFI44L, ISG15, SLC1A2, OLAH, FAM20A, MMP8 | 0,854 | 0,844 | 0,865 | 0,915 | 0,859 | 0,971 | 0,922 | 0,844 | 0,999 |
| OAS1, IFIT1, IFI44L, ISG15, SLC1A2, IL1R2, OLAH, FAM20A, MMP8 | 0,854 | 0,844 | 0,864 | 0,916 | 0,864 | 0,968 | 0,927 | 0,845 | 1 |
| RSAD2, OAS1, IFIT1, SIGLEC1, HERC6, SLC1A2, IL1R2, OLAH, RETN | 0,854 | 0,844 | 0,865 | 0,915 | 0,860 | 0,969 | 0,950 | 0,900 | 1,000 |
| RSAD2, IFIT1, IFI44L, ISG15, HERC6, SLC1A2, OLAH, FAM20A, RETN | 0,854 | 0,844 | 0,864 | 0,917 | 0,864 | 0,970 | 0,916 | 0,831 | 1 |
| RSAD2, IFIT1, SIGLEC1, HERC6, SLC1A2, IL1R2, OLAH, RETN, MMP8 | 0,854 | 0,844 | 0,864 | 0,916 | 0,862 | 0,970 | 0,961 | 0,922 | 1,000 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, HERC6, SLC1A2, IL1R2, FAM20A, MMP8 | 0,854 | 0,844 | 0,864 | 0,918 | 0,868 | 0,969 | 0,925 | 0,832 | 1 |
| RSAD2, OAS1, IFIT1, SIGLEC1, ISG15, HERC6, SLC1A2, IL1R2, OLAH | 0,854 | 0,844 | 0,864 | 0,912 | 0,858 | 0,966 | 0,962 | 0,923 | 1 |
| RSAD2, IFIT1, IFI44L, SIGLEC1, ISG15, HERC6, IL1R2, RETN, MMP8 | 0,854 | 0,844 | 0,864 | 0,904 | 0,842 | 0,966 | 0,964 | 0,926 | 1 |
| RSAD2, IFI44L, ISG15, HERC6, SLC1A2, OLAH, FAM20A, RETN, MMP8 | 0,854 | 0,843 | 0,865 | 0,918 | 0,863 | 0,972 | 0,927 | 0,848 | 1 |
| RSAD2, ISG15, HERC6, SLC1A2, IL1R2, OLAH, FAM20A, RETN, MMP8 | 0,854 | 0,844 | 0,864 | 0,916 | 0,864 | 0,968 | 0,929 | 0,843 | 1 |
| OAS1, IFIT1, SIGLEC1, ISG15, HERC6, SLC1A2, IL1R2, OLAH, MMP8 | 0,854 | 0,844 | 0,864 | 0,913 | 0,860 | 0,966 | 0,962 | 0,924 | 1 |
| RSAD2, OAS1, IFIT1, IFI44L, ISG15, HERC6, IL1R2, FAM20A, RETN | 0,854 | 0,844 | 0,864 | 0,913 | 0,855 | 0,970 | 0,934 | 0,855 | 1 |
| RSAD2, OAS1, IFIT1, IFI44L, ISG15, HERC6, OLAH, FAM20A, MMP8 | 0,854 | 0,843 | 0,865 | 0,907 | 0,846 | 0,967 | 0,933 | 0,863 | 1 |
| RSAD2, IFIT1, IFI44L, SIGLEC1, HERC6, IL1R2, OLAH, RETN, MMP8 | 0,854 | 0,844 | 0,864 | 0,904 | 0,841 | 0,966 | 0,964 | 0,926 | 1 |
| RSAD2, OAS1, IFIT1, SIGLEC1, ISG15, HERC6, SLC1A2, IL1R2, RETN | 0,854 | 0,844 | 0,864 | 0,911 | 0,856 | 0,965 | 0,957 | 0,909 | 1 |
| RSAD2, OAS1, IFIT1, IFI44L, ISG15, HERC6, OLAH, FAM20A, RETN | 0,854 | 0,844 | 0,864 | 0,908 | 0,849 | 0,968 | 0,933 | 0,863 | 1 |
| OAS1, IFIT1, ISG15, HERC6, SLC1A2, IL1R2, FAM20A, RETN, MMP8 | 0,854 | 0,844 | 0,864 | 0,914 | 0,860 | 0,968 | 0,930 | 0,844 | 1 |
| OAS1, IFIT1, IFI44L, SIGLEC1, ISG15, HERC6, IL1R2, OLAH, RETN | 0,854 | 0,844 | 0,864 | 0,907 | 0,847 | 0,967 | 0,952 | 0,907 | 0,998 |
| RSAD2, IFIT1, IFI44L, HERC6, IL1R2, OLAH, FAM20A, RETN, MMP8 | 0,854 | 0,844 | 0,864 | 0,912 | 0,853 | 0,972 | 0,941 | 0,868 | 1 |
| OAS1, IFIT1, IFI44L, SIGLEC1, HERC6, SLC1A2, IL1R2, RETN, MMP8 | 0,854 | 0,844 | 0,864 | 0,911 | 0,856 | 0,966 | 0,961 | 0,921 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, SIGLEC1, HERC6, SLC1A2, IL1R2, RETN, MMP8 | 0,854 | 0,844 | 0,864 | 0,913 | 0,859 | 0,968 | 0,965 | 0,927 | 1 |
| RSAD2, IFI27, OAS1, IFI44L, ISG15, SLC1A2, FAM20A, RETN, MMP8 | 0,854 | 0,842 | 0,865 | 0,913 | 0,851 | 0,974 | 0,940 | 0,862 | 1 |
| IFIT1, IFI44L, SIGLEC1, HERC6, SLC1A2, IL1R2, OLAH, RETN, MMP8 | 0,854 | 0,844 | 0,864 | 0,915 | 0,861 | 0,969 | 0,959 | 0,918 | 0,999 |
| RSAD2, OAS1, IFIT1, IFI44L, HERC6, SLC1A2, OLAH, FAM20A, RETN | 0,854 | 0,844 | 0,864 | 0,917 | 0,864 | 0,970 | 0,913 | 0,826 | 1 |
| RSAD2, IFI27, OAS1, IFIT1, IFI44L, SLC1A2, FAM20A, RETN, MMP8 | 0,854 | 0,843 | 0,865 | 0,912 | 0,851 | 0,974 | 0,940 | 0,861 | 1 |
| OAS1, IFI44L, ISG15, HERC6, SLC1A2, IL1R2, OLAH, FAM20A, RETN | 0,854 | 0,842 | 0,865 | 0,911 | 0,856 | 0,966 | 0,924 | 0,839 | 1 |
| RSAD2, OAS1, IFIT1, SIGLEC1, ISG15, HERC6, SLC1A2, OLAH, RETN | 0,854 | 0,844 | 0,864 | 0,915 | 0,860 | 0,970 | 0,950 | 0,897 | 1 |
| RSAD2, IFI27, IFIT1, IFI44L, SIGLEC1, ISG15, SLC1A2, RETN, MMP8 | 0,854 | 0,844 | 0,863 | 0,915 | 0,859 | 0,971 | 0,951 | 0,892 | 1 |
| RSAD2, OAS1, ISG15, HERC6, SLC1A2, IL1R2, FAM20A, RETN, MMP8 | 0,854 | 0,844 | 0,864 | 0,917 | 0,864 | 0,969 | 0,928 | 0,839 | 1 |
| RSAD2, OAS1, IFIT1, SIGLEC1, HERC6, SLC1A2, IL1R2, OLAH, MMP8 | 0,854 | 0,843 | 0,864 | 0,912 | 0,858 | 0,966 | 0,963 | 0,925 | 1 |
| RSAD2, OAS1, ISG15, HERC6, SLC1A2, IL1R2, OLAH, FAM20A, RETN | 0,854 | 0,843 | 0,864 | 0,912 | 0,858 | 0,967 | 0,928 | 0,843 | 1 |
| RSAD2, OAS1, IFIT1, IFI44L, SIGLEC1, HERC6, OLAH, RETN, MMP8 | 0,854 | 0,843 | 0,864 | 0,901 | 0,836 | 0,966 | 0,953 | 0,909 | 0,997 |
| RSAD2, OAS1, IFIT1, IFI44L, SIGLEC1, HERC6, IL1R2, OLAH, RETN | 0,853 | 0,844 | 0,863 | 0,905 | 0,842 | 0,968 | 0,955 | 0,911 | 0,999 |
| OAS1, IFIT1, ISG15, HERC6, SLC1A2, OLAH, FAM20A, RETN, MMP8 | 0,853 | 0,843 | 0,864 | 0,910 | 0,852 | 0,968 | 0,936 | 0,859 | 1 |
| OAS1, IFIT1, IFI44L, HERC6, SLC1A2, IL1R2, OLAH, FAM20A, RETN | 0,853 | 0,843 | 0,864 | 0,918 | 0,866 | 0,969 | 0,917 | 0,831 | 1 |

EP 4 365 308 A1

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SIGLEC1, HERC6, SLC1A2, OLAH, MMP8 | 0,853 | 0,843 | 0,864 | 0,912 | 0,857 | 0,967 | 0,951 | 0,905 | 0,998 |
| RSAD2, IFI27, OAS1, IFI44L, SIGLEC1, ISG15, HERC6, SLC1A2, MMP8 | 0,853 | 0,844 | 0,863 | 0,913 | 0,860 | 0,967 | 0,925 | 0,854 | 0,996 |
| RSAD2, OAS1, IFIT1, SIGLEC1, ISG15, HERC6, OLAH, RETN, MMP8 | 0,853 | 0,843 | 0,864 | 0,905 | 0,843 | 0,967 | 0,958 | 0,916 | 1,000 |
| RSAD2, IFIT1, SIGLEC1, ISG15, HERC6, SLC1A2, IL1R2, OLAH, MMP8 | 0,853 | 0,844 | 0,863 | 0,914 | 0,860 | 0,967 | 0,962 | 0,924 | 1 |
| OAS1, IFI44L, SIGLEC1, ISG15, HERC6, IL1R2, OLAH, RETN, MMP8 | 0,853 | 0,843 | 0,864 | 0,906 | 0,845 | 0,967 | 0,967 | 0,932 | 1 |
| OAS1, IFIT1, IFI44L, SIGLEC1, ISG15, HERC6, SLC1A2, OLAH, MMP8 | 0,853 | 0,843 | 0,864 | 0,913 | 0,858 | 0,968 | 0,945 | 0,894 | 0,995 |
| IFIT1, IFI44L, ISG15, HERC6, SLC1A2, IL1R2, FAM20A, RETN, MMP8 | 0,853 | 0,844 | 0,863 | 0,916 | 0,863 | 0,969 | 0,928 | 0,837 | 1 |
| OAS1, IFIT1, IFI44L, HERC6, SLC1A2, IL1R2, FAM20A, RETN, MMP8 | 0,853 | 0,843 | 0,863 | 0,915 | 0,862 | 0,969 | 0,927 | 0,836 | 1 |
| RSAD2, OAS1, SIGLEC1, ISG15, HERC6, SLC1A2, IL1R2, OLAH, MMP8 | 0,853 | 0,843 | 0,863 | 0,911 | 0,857 | 0,964 | 0,962 | 0,923 | 1 |
| OAS1, IFIT1, IFI44L, SIGLEC1, ISG15, HERC6, SLC1A2, IL1R2, RETN | 0,853 | 0,843 | 0,863 | 0,913 | 0,860 | 0,966 | 0,952 | 0,904 | 1,000 |
| RSAD2, OAS1, IFIT1, HERC6, SLC1A2, IL1R2, FAM20A, RETN, MMP8 | 0,853 | 0,843 | 0,863 | 0,914 | 0,859 | 0,969 | 0,934 | 0,850 | 1 |
| RSAD2, IFIT1, IFI44L, ISG15, HERC6, IL1R2, OLAH, FAM20A, RETN | 0,853 | 0,843 | 0,863 | 0,909 | 0,850 | 0,968 | 0,932 | 0,855 | 1 |
| OAS1, IFI44L, SIGLEC1, ISG15, HERC6, SLC1A2, OLAH, RETN, MMP8 | 0,853 | 0,843 | 0,863 | 0,912 | 0,855 | 0,969 | 0,962 | 0,921 | 1 |
| RSAD2, OAS1, IFIT1, ISG15, SLC1A2, IL1R2, OLAH, FAM20A, MMP8 | 0,853 | 0,843 | 0,863 | 0,917 | 0,865 | 0,969 | 0,936 | 0,859 | 1 |
| RSAD2, OAS1, IFIT1, IFI44L, ISG15, HERC6, SLC1A2, IL1R2, FAM20A | 0,853 | 0,843 | 0,863 | 0,915 | 0,865 | 0,966 | 0,925 | 0,834 | 1 |

702

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SIGLEC1, HERC6, IL1R2, OLAH, MMP8 | 0,853 | 0,843 | 0,863 | 0,904 | 0,845 | 0,963 | 0,963 | 0,924 | 1 |
| RSAD2, OAS1, IFI44L, SIGLEC1, HERC6, IL1R2, OLAH, RETN, MMP8 | 0,853 | 0,842 | 0,864 | 0,901 | 0,838 | 0,965 | 0,965 | 0,927 | 1 |
| RSAD2, OAS1, IFI44L, ISG15, HERC6, OLAH, FAM20A, RETN, MMP8 | 0,853 | 0,842 | 0,864 | 0,912 | 0,851 | 0,972 | 0,938 | 0,872 | 1 |
| OAS1, IFIT1, ISG15, HERC6, SLC1A2, IL1R2, OLAH, FAM20A, RETN | 0,853 | 0,842 | 0,864 | 0,912 | 0,857 | 0,966 | 0,927 | 0,842 | 1 |
| IFIT1, IFI44L, SIGLEC1, ISG15, HERC6, SLC1A2, IL1R2, OLAH, MMP8 | 0,853 | 0,843 | 0,863 | 0,913 | 0,860 | 0,967 | 0,961 | 0,922 | 1,000 |
| RSAD2, OAS1, IFI44L, ISG15, HERC6, SLC1A2, IL1R2, FAM20A, MMP8 | 0,853 | 0,842 | 0,864 | 0,918 | 0,867 | 0,968 | 0,924 | 0,830 | 1 |
| RSAD2, OAS1, SIGLEC1, ISG15, HERC6, SLC1A2, OLAH, RETN, MMP8 | 0,853 | 0,843 | 0,863 | 0,913 | 0,857 | 0,970 | 0,961 | 0,919 | 1 |
| RSAD2, OAS1, IFIT1, IFI44L, SIGLEC1, ISG15, HERC6, IL1R2, OLAH | 0,853 | 0,843 | 0,862 | 0,905 | 0,847 | 0,964 | 0,958 | 0,915 | 1 |
| RSAD2, OAS1, IFIT1, ISG15, HERC6, SLC1A2, IL1R2, OLAH, FAM20A | 0,853 | 0,842 | 0,863 | 0,912 | 0,858 | 0,966 | 0,928 | 0,844 | 1 |
| RSAD2, IFIT1, IFI44L, HERC6, SLC1A2, IL1R2, OLAH, FAM20A, RETN | 0,853 | 0,843 | 0,863 | 0,917 | 0,865 | 0,969 | 0,917 | 0,831 | 1 |
| RSAD2, IFIT1, HERC6, SLC1A2, IL1R2, OLAH, FAM20A, RETN, MMP8 | 0,853 | 0,842 | 0,863 | 0,915 | 0,863 | 0,968 | 0,933 | 0,851 | 1 |
| OAS1, IFI44L, SIGLEC1, HERC6, SLC1A2, IL1R2, OLAH, RETN, MMP8 | 0,853 | 0,843 | 0,863 | 0,915 | 0,860 | 0,969 | 0,962 | 0,923 | 1 |
| RSAD2, OAS1, ISG15, HERC6, SLC1A2, OLAH, FAM20A, RETN, MMP8 | 0,853 | 0,843 | 0,863 | 0,911 | 0,855 | 0,968 | 0,935 | 0,856 | 1 |
| RSAD2, OAS1, IFIT1, IFI44L, HERC6, IL1R2, OLAH, FAM20A, RETN | 0,853 | 0,843 | 0,863 | 0,907 | 0,846 | 0,968 | 0,934 | 0,858 | 1 |
| RSAD2, OAS1, IFIT1, SIGLEC1, ISG15, HERC6, SLC1A2, OLAH, MMP8 | 0,853 | 0,842 | 0,863 | 0,912 | 0,857 | 0,967 | 0,951 | 0,905 | 0,998 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, HERC6, IL1R2, RETN, MMP8 | 0,853 | 0,843 | 0,863 | 0,903 | 0,841 | 0,965 | 0,967 | 0,932 | 1 |
| RSAD2, SIGLEC1, ISG15, HERC6, SLC1A2, IL1R2, OLAH, RETN, MMP8 | 0,853 | 0,843 | 0,862 | 0,916 | 0,862 | 0,969 | 0,962 | 0,923 | 1 |
| RSAD2, IFI44L, ISG15, SLC1A2, IL1R2, OLAH, FAM20A, RETN, MMP8 | 0,853 | 0,842 | 0,863 | 0,917 | 0,864 | 0,969 | 0,929 | 0,851 | 1 |
| RSAD2, OAS1, IFI44L, SLC1A2, IL1R2, OLAH, FAM20A, RETN, MMP8 | 0,853 | 0,842 | 0,863 | 0,919 | 0,867 | 0,970 | 0,930 | 0,852 | 1 |
| RSAD2, OAS1, SIGLEC1, ISG15, HERC6, SLC1A2, IL1R2, RETN, MMP8 | 0,853 | 0,843 | 0,863 | 0,912 | 0,857 | 0,967 | 0,966 | 0,929 | 1 |
| RSAD2, IFIT1, SIGLEC1, ISG15, HERC6, SLC1A2, OLAH, RETN, MMP8 | 0,853 | 0,843 | 0,862 | 0,914 | 0,859 | 0,969 | 0,952 | 0,907 | 0,997 |
| RSAD2, IFIT1, IFI44L, SIGLEC1, HERC6, SLC1A2, IL1R2, OLAH, MMP8 | 0,853 | 0,843 | 0,862 | 0,912 | 0,858 | 0,965 | 0,962 | 0,923 | 1 |
| RSAD2, IFIT1, ISG15, HERC6, SLC1A2, IL1R2, FAM20A, RETN, MMP8 | 0,853 | 0,843 | 0,862 | 0,915 | 0,861 | 0,969 | 0,930 | 0,844 | 1 |
| RSAD2, IFI44L, ISG15, HERC6, SLC1A2, IL1R2, OLAH, FAM20A, RETN | 0,853 | 0,841 | 0,864 | 0,913 | 0,858 | 0,967 | 0,925 | 0,841 | 1 |
| RSAD2, OAS1, IFIT1, ISG15, HERC6, OLAH, FAM20A, RETN, MMP8 | 0,853 | 0,842 | 0,863 | 0,905 | 0,843 | 0,967 | 0,943 | 0,876 | 1 |
| RSAD2, OAS1, IFIT1, SLC1A2, IL1R2, OLAH, FAM20A, RETN, MMP8 | 0,853 | 0,843 | 0,862 | 0,913 | 0,858 | 0,968 | 0,937 | 0,861 | 1 |
| OAS1, IFIT1, IFI44L, ISG15, HERC6, SLC1A2, IL1R2, FAM20A, MMP8 | 0,853 | 0,842 | 0,863 | 0,917 | 0,866 | 0,967 | 0,925 | 0,832 | 1 |
| RSAD2, IFI27, OAS1, IFIT1, ISG15, SLC1A2, FAM20A, RETN, MMP8 | 0,853 | 0,842 | 0,863 | 0,910 | 0,847 | 0,972 | 0,942 | 0,864 | 1 |
| OAS1, IFI44L, ISG15, SLC1A2, IL1R2, OLAH, FAM20A, RETN, MMP8 | 0,853 | 0,842 | 0,863 | 0,917 | 0,865 | 0,970 | 0,928 | 0,847 | 1 |
| RSAD2, OAS1, IFIT1, IFI44L, SIGLEC1, HERC6, SLC1A2, IL1R2, OLAH | 0,853 | 0,843 | 0,862 | 0,914 | 0,860 | 0,967 | 0,962 | 0,923 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, HERC6, SLC1A2, IL1R2, OLAH, RETN | 0,852 | 0,842 | 0,863 | 0,911 | 0,856 | 0,967 | 0,959 | 0,915 | 1 |
| OAS1, IFI44L, ISG15, HERC6, SLC1A2, IL1R2, FAM20A, RETN, MMP8 | 0,852 | 0,842 | 0,863 | 0,915 | 0,862 | 0,968 | 0,926 | 0,836 | 1 |
| RSAD2, OAS1, IFIT1, ISG15, HERC6, SLC1A2, IL1R2, FAM20A, MMP8 | 0,852 | 0,843 | 0,862 | 0,916 | 0,864 | 0,968 | 0,929 | 0,842 | 1 |
| OAS1, IFIT1, IFI44L, SIGLEC1, HERC6, SLC1A2, IL1R2, OLAH, MMP8 | 0,852 | 0,842 | 0,863 | 0,913 | 0,860 | 0,966 | 0,962 | 0,924 | 1 |
| RSAD2, OAS1, IFIT1, IFI44L, SIGLEC1, ISG15, HERC6, SLC1A2, OLAH | 0,852 | 0,842 | 0,863 | 0,912 | 0,857 | 0,967 | 0,953 | 0,908 | 0,999 |
| RSAD2, OAS1, IFIT1, ISG15, IL1R2, OLAH, FAM20A, RETN, MMP8 | 0,852 | 0,842 | 0,863 | 0,906 | 0,844 | 0,968 | 0,947 | 0,878 | 1 |
| RSAD2, IFIT1, IFI44L, SIGLEC1, HERC6, SLC1A2, OLAH, RETN, MMP8 | 0,852 | 0,843 | 0,862 | 0,914 | 0,859 | 0,969 | 0,945 | 0,895 | 0,994 |
| RSAD2, IFIT1, IFI44L, SIGLEC1, ISG15, HERC6, SLC1A2, OLAH, MMP8 | 0,852 | 0,842 | 0,862 | 0,912 | 0,858 | 0,967 | 0,941 | 0,889 | 0,993 |
| OAS1, IFIT1, ISG15, SLC1A2, IL1R2, OLAH, FAM20A, RETN, MMP8 | 0,852 | 0,842 | 0,862 | 0,915 | 0,861 | 0,969 | 0,937 | 0,861 | 1 |
| OAS1, IFIT1, IFI44L, SIGLEC1, ISG15, HERC6, SLC1A2, IL1R2, OLAH | 0,852 | 0,842 | 0,862 | 0,913 | 0,859 | 0,967 | 0,954 | 0,911 | 0,998 |
| RSAD2, OAS1, IFI44L, HERC6, SLC1A2, IL1R2, OLAH, FAM20A, RETN | 0,852 | 0,841 | 0,863 | 0,914 | 0,860 | 0,967 | 0,923 | 0,838 | 1 |
| RSAD2, OAS1, IFI44L, SIGLEC1, ISG15, SLC1A2, FAM20A, RETN, MMP8 | 0,852 | 0,842 | 0,863 | 0,911 | 0,851 | 0,970 | 0,949 | 0,878 | 1 |
| OAS1, IFIT1, IFI44L, ISG15, SLC1A2, OLAH, FAM20A, RETN, MMP8 | 0,852 | 0,842 | 0,863 | 0,914 | 0,857 | 0,970 | 0,922 | 0,845 | 0,999 |
| RSAD2, IFIT1, IFI44L, SLC1A2, IL1R2, OLAH, FAM20A, RETN, MMP8 | 0,852 | 0,842 | 0,862 | 0,916 | 0,862 | 0,969 | 0,934 | 0,858 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, HERC6, SLC1A2, IL1R2, OLAH, RETN | 0,852 | 0,842 | 0,863 | 0,915 | 0,862 | 0,968 | 0,945 | 0,894 | 0,995 |
| RSAD2, OAS1, IFIT1, IFI44L, SIGLEC1, ISG15, HERC6, OLAH, RETN | 0,852 | 0,842 | 0,862 | 0,905 | 0,843 | 0,968 | 0,947 | 0,897 | 0,997 |
| RSAD2, IFI44L, ISG15, HERC6, SLC1A2, IL1R2, FAM20A, RETN, MMP8 | 0,852 | 0,842 | 0,862 | 0,917 | 0,864 | 0,970 | 0,927 | 0,838 | 1 |
| OAS1, IFIT1, IFI44L, SIGLEC1, ISG15, HERC6, IL1R2, OLAH, MMP8 | 0,852 | 0,842 | 0,862 | 0,906 | 0,849 | 0,964 | 0,963 | 0,924 | 1 |
| IFIT1, IFI44L, SIGLEC1, ISG15, HERC6, SLC1A2, IL1R2, RETN, MMP8 | 0,852 | 0,843 | 0,861 | 0,911 | 0,857 | 0,966 | 0,966 | 0,931 | 1 |
| RSAD2, OAS1, IFI44L, ISG15, HERC6, SLC1A2, OLAH, FAM20A, RETN | 0,852 | 0,841 | 0,863 | 0,914 | 0,857 | 0,970 | 0,925 | 0,846 | 1 |
| RSAD2, OAS1, IFIT1, IFI44L, SIGLEC1, ISG15, HERC6, IL1R2, MMP8 | 0,852 | 0,842 | 0,861 | 0,904 | 0,845 | 0,962 | 0,960 | 0,919 | 1 |
| OAS1, IFIT1, SIGLEC1, ISG15, SLC1A2, IL1R2, OLAH, RETN, MMP8 | 0,852 | 0,842 | 0,862 | 0,913 | 0,859 | 0,967 | 0,966 | 0,931 | 1 |
| OAS1, IFIT1, IFI44L, SLC1A2, IL1R2, OLAH, FAM20A, RETN, MMP8 | 0,852 | 0,842 | 0,862 | 0,916 | 0,863 | 0,969 | 0,934 | 0,857 | 1 |
| RSAD2, IFIT1, SIGLEC1, ISG15, HERC6, SLC1A2, IL1R2, OLAH, RETN | 0,852 | 0,842 | 0,862 | 0,914 | 0,859 | 0,969 | 0,951 | 0,905 | 0,997 |
| RSAD2, OAS1, IFI44L, SIGLEC1, HERC6, SLC1A2, IL1R2, OLAH, MMP8 | 0,852 | 0,842 | 0,862 | 0,912 | 0,858 | 0,966 | 0,962 | 0,923 | 1 |
| IFIT1, IFI44L, SIGLEC1, ISG15, HERC6, SLC1A2, IL1R2, OLAH, RETN | 0,852 | 0,842 | 0,862 | 0,913 | 0,858 | 0,968 | 0,949 | 0,902 | 0,996 |
| IFI44L, SIGLEC1, ISG15, HERC6, SLC1A2, IL1R2, OLAH, RETN, MMP8 | 0,852 | 0,842 | 0,861 | 0,913 | 0,858 | 0,968 | 0,965 | 0,929 | 1 |
| OAS1, IFI44L, SIGLEC1, ISG15, HERC6, SLC1A2, IL1R2, RETN, MMP8 | 0,852 | 0,842 | 0,861 | 0,911 | 0,857 | 0,966 | 0,967 | 0,931 | 1 |
| RSAD2, IFIT1, IFI44L, SIGLEC1, ISG15, HERC6, SLC1A2, OLAH, RETN | 0,852 | 0,842 | 0,861 | 0,915 | 0,860 | 0,969 | 0,941 | 0,890 | 0,993 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, HERC6, SLC1A2, IL1R2, RETN, MMP8 | 0,852 | 0,843 | 0,861 | 0,913 | 0,859 | 0,966 | 0,963 | 0,925 | 1 |
| RSAD2, IFIT1, IFI44L, ISG15, HERC6, SLC1A2, IL1R2, FAM20A, MMP8 | 0,852 | 0,841 | 0,862 | 0,918 | 0,868 | 0,968 | 0,924 | 0,830 | 1 |
| RSAD2, OAS1, IFIT1, IFI44L, SIGLEC1, HERC6, SLC1A2, IL1R2, RETN | 0,852 | 0,842 | 0,861 | 0,911 | 0,855 | 0,967 | 0,957 | 0,910 | 1 |
| RSAD2, IFIT1, IFI44L, SIGLEC1, HERC6, SLC1A2, IL1R2, OLAH, RETN | 0,852 | 0,842 | 0,861 | 0,916 | 0,862 | 0,970 | 0,949 | 0,902 | 0,996 |
| RSAD2, OAS1, IFIT1, HERC6, SLC1A2, OLAH, FAM20A, RETN, MMP8 | 0,852 | 0,842 | 0,862 | 0,909 | 0,851 | 0,967 | 0,932 | 0,852 | 1 |
| OAS1, IFIT1, IFI44L, SIGLEC1, ISG15, SLC1A2, OLAH, RETN, MMP8 | 0,852 | 0,842 | 0,862 | 0,912 | 0,856 | 0,969 | 0,957 | 0,912 | 1 |
| OAS1, IFIT1, IFI44L, ISG15, HERC6, SLC1A2, IL1R2. FAM20A, RETN | 0,852 | 0,841 | 0,862 | 0,915 | 0,864 | 0,967 | 0,924 | 0,833 | 1 |
| RSAD2, IFIT1, IFI44L, HERC6, SLC1A2, IL1R2, FAM20A, RETN, MMP8 | 0,852 | 0,842 | 0,861 | 0,916 | 0,863 | 0,970 | 0,927 | 0,839 | 1 |
| RSAD2, OAS1, IFIT1, IFI44L, IL1R2, OLAH, FAM20A, RETN, MMP8 | 0,852 | 0,841 | 0,862 | 0,910 | 0,849 | 0,971 | 0,945 | 0,877 | 1 |
| RSAD2, OAS1, IFIT1, IFI44L, SLC1A2, OLAH, FAM20A, RETN, MMP8 | 0,851 | 0,841 | 0,862 | 0,914 | 0,858 | 0,971 | 0,924 | 0,848 | 1,000 |
| RSAD2, IFI27, OAS1, IFIT1, ISG15, HERC6, SLC1A2, FAM20A, MMP8 | 0,851 | 0,841 | 0,862 | 0,915 | 0,860 | 0,969 | 0,923 | 0,837 | 1 |
| RSAD2, IFIT1, IFI44L, ISG15, SLC1A2, OLAH, FAM20A, RETN, MMP8 | 0,851 | 0,841 | 0,861 | 0,912 | 0,855 | 0,970 | 0,925 | 0,851 | 0,999 |
| RSAD2, OAS1, IFIT1, IFI44L, SIGLEC1, ISG15, SLC1A2, OLAH, MMP8 | 0,851 | 0,841 | 0,861 | 0,912 | 0,857 | 0,968 | 0,949 | 0,900 | 0,997 |
| RSAD2, IFIT1, ISG15, HERC6, SLC1A2, IL1R2. OLAH, FAM20A, RETN | 0,851 | 0,841 | 0,862 | 0,911 | 0,856 | 0,967 | 0,928 | 0,844 | 1 |
| RSAD2, IFIT1, IFI44L, SIGLEC1, ISG15, HERC6, SLC1A2, IL1R2, OLAH | 0,851 | 0,842 | 0,861 | 0,913 | 0,859 | 0,967 | 0,953 | 0,909 | 0,997 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, ISG15, HERC6, IL1R2, OLAH, MMP8 | 0,851 | 0,842 | 0,861 | 0,904 | 0,845 | 0,964 | 0,962 | 0,922 | 1 |
| RSAD2, OAS1, IFI44L, SIGLEC1, ISG15, HERC6, SLC1A2, IL1R2, OLAH | 0,851 | 0,841 | 0,861 | 0,912 | 0,858 | 0,966 | 0,962 | 0,922 | 1 |
| OAS1, IFI44L, SIGLEC1, ISG15, HERC6, SLC1A2, IL1R2, OLAH, RETN | 0,851 | 0,841 | 0,862 | 0,914 | 0,860 | 0,968 | 0,957 | 0,912 | 1 |
| RSAD2, OAS1, IFIT1, SIGLEC1, ISG15, HERC6, IL1R2, OLAH, MMP8 | 0,851 | 0,841 | 0,861 | 0,906 | 0,849 | 0,963 | 0,961 | 0,921 | 1 |
| RSAD2, OAS1, IFI44L, SIGLEC1, HERC6, SLC1A2, OLAH, RETN, MMP8 | 0,851 | 0,841 | 0,862 | 0,911 | 0,853 | 0,968 | 0,963 | 0,922 | 1 |
| RSAD2, IFI44L, SIGLEC1, HERC6, SLC1A2, IL1R2, OLAH, RETN, MMP8 | 0,851 | 0,841 | 0,861 | 0,915 | 0,861 | 0,969 | 0,962 | 0,923 | 1 |
| RSAD2, IFIT1, IFI44L, SIGLEC1, ISG15, HERC6, IL1R2, OLAH, RETN, | 0,851 | 0,842 | 0,861 | 0,905 | 0,844 | 0,967 | 0,950 | 0,904 | 0,996 |
| OAS1, IFI44L, SIGLEC1, ISG15, HERC6, SLC1A2, IL1R2, OLAH, MMP8 | 0,851 | 0,841 | 0,861 | 0,912 | 0,859 | 0,966 | 0,961 | 0,922 | 1 |
| RSAD2, IFI44L, SIGLEC1, ISG15, HERC6, IL1R2, OLAH, RETN, MMP8 | 0,851 | 0,841 | 0,861 | 0,903 | 0,839 | 0,966 | 0,963 | 0,924 | 1 |
| RSAD2, OAS1, IFI44L, SIGLEC1, ISG15, HERC6, IL1R2 OLAH, MMP8 | 0,851 | 0,841 | 0,861 | 0,904 | 0,846 | 0,963 | 0,962 | 0,922 | 1 |
| RSAD2, OAS1, IFI44L, SIGLEC1, ISG15, HERC6, IL1R2. OLAH, RETN | 0,851 | 0,841 | 0,861 | 0,902 | 0,840 | 0,965 | 0,957 | 0,912 | 1 |
| RSAD2, OAS1, IFI44L, HERC6, SLC1A2, IL1R2, FAM20A, RETN, MMP8 | 0,851 | 0,841 | 0,861 | 0,918 | 0,865 | 0,970 | 0,926 | 0,834 | 1 |
| RSAD2, OAS1, IFI44L, ISG15, SLC1A2, OLAH, FAM20A, RETN, MMP8 | 0,851 | 0,840 | 0,862 | 0,915 | 0,858 | 0,972 | 0,924 | 0,848 | 0,999 |
| RSAD2, IFI44L, SIGLEC1, ISG15, HERC6, SLC1A2, IL1R2, OLAH, MMP8 | 0,851 | 0,842 | 0,860 | 0,913 | 0,859 | 0,966 | 0,965 | 0,929 | 1 |
| RSAD2, IFIT1, ISG15, HERC6, SLC1A2, OLAH, FAM20A, RETN, MMP8 | 0,851 | 0,841 | 0,861 | 0,911 | 0,853 | 0,968 | 0,934 | 0,855 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, ISG15, HERC6, SLC1A2, OLAH, FAM20A, MMP8 | 0,851 | 0,840 | 0,861 | 0,910 | 0,853 | 0,966 | 0,929 | 0,849 | 1 |
| OAS1, IFIT1, IFI44L, SIGLEC1, SLC1A2, IL1R2, OLAH, RETN, MMP8 | 0,851 | 0,841 | 0,861 | 0,914 | 0,861 | 0,968 | 0,967 | 0,932 | 1 |
| RSAD2, OAS1, IFIT1, ISG15, HERC6, SLC1A2, IL1R2, FAM20A, RETN | 0,851 | 0,840 | 0,861 | 0,911 | 0,856 | 0,966 | 0,928 | 0,842 | 1 |
| RSAD2, OAS1, IFIT1, SIGLEC1, SLC1A2, IL1R2, OLAH, RETN, MMP8 | 0,851 | 0,841 | 0,861 | 0,913 | 0,859 | 0,967 | 0,972 | 0,940 | 1 |
| IFIT1, IFI44L, SIGLEC1, ISG15, SLC1A2, IL1R2, OLAH, RETN, MMP8 | 0,851 | 0,841 | 0,860 | 0,913 | 0,859 | 0,968 | 0,967 | 0,933 | 1 |
| RSAD2, OAS1, IFIT1, HERC6, SLC1A2, IL1R2, OLAH, FAM20A, RETN | 0,851 | 0,840 | 0,861 | 0,912 | 0,857 | 0,966 | 0,928 | 0,844 | 1 |
| RSAD2, IFIT1, IFI44L, SIGLEC1, HERC6, SLC1A2, IL1R2, RETN, MMP8 | 0,851 | 0,842 | 0,860 | 0,912 | 0,857 | 0,966 | 0,964 | 0,927 | 1 |
| RSAD2, OAS1, IFIT1, IFI44L, ISG15, SLC1A2, IL1R2, OLAH, FAM20A | 0,850 | 0,840 | 0,861 | 0,914 | 0,859 | 0,969 | 0,927 | 0,847 | 1 |
| RSAD2, OAS1, IFI44L, ISG15, IL1R2, OLAH, FAM20A, RETN, MMP8 | 0,850 | 0,840 | 0,861 | 0,910 | 0,848 | 0,971 | 0,945 | 0,876 | 1 |
| RSAD2, OAS1, IFI44L, ISG15, HERC6, SLC1A2, IL1R2, FAM20A, RETN | 0,850 | 0,839 | 0,862 | 0,914 | 0,861 | 0,967 | 0,925 | 0,835 | 1 |
| RSAD2, IFIT1, IFI44L, SIGLEC1, ISG15, HERC6, OLAH, RETN, MMP8 | 0,850 | 0,840 | 0,860 | 0,905 | 0,841 | 0,968 | 0,957 | 0,915 | 0,998 |
| RSAD2, OAS1, IFIT1, IFI44L, SIGLEC1, SLC1A2, IL1R2, OLAH, MMP8 | 0,850 | 0,841 | 0,860 | 0,912 | 0,858 | 0,966 | 0,968 | 0,934 | 1 |
| RSAD2, IFIT1, SIGLEC1, ISG15, SLC1A2, IL1R2, OLAH, RETN, MMP8 | 0,850 | 0,841 | 0,860 | 0,913 | 0,858 | 0,967 | 0,968 | 0,935 | 1 |
| OAS1, IFIT1, IFI44L, SIGLEC1, ISG15, SLC1A2, IL1R2, OLAH, MMP8 | 0,850 | 0,841 | 0,860 | 0,913 | 0,859 | 0,966 | 0,967 | 0,933 | 1 |
| RSAD2, OAS1, IFI44L, SIGLEC1, ISG15, HERC6, SLC1A2, IL1R2, RETN | 0,850 | 0,840 | 0,860 | 0,911 | 0,856 | 0,965 | 0,958 | 0,911 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, SLC1A2, IL1R2, OLAH, RETN, MMP8 | 0,850 | 0,840 | 0,860 | 0,912 | 0,858 | 0,967 | 0,971 | 0,938 | 1 |
| RSAD2, OAS1, IFI44L, SIGLEC1, HERC6, SLC1A2, IL1R2, OLAH, RETN | 0,850 | 0,840 | 0,861 | 0,912 | 0,857 | 0,967 | 0,959 | 0,914 | 1 |
| RSAD2, OAS1, IFIT1, IFI44L, ISG15, HERC6, FAM20A, RETN, MMP8 | 0,850 | 0,840 | 0,861 | 0,908 | 0,847 | 0,969 | 0,927 | 0,849 | 1 |
| RSAD2, OAS1, IFIT1, SIGLEC1, ISG15, SLC1A2, IL1R2, OLAH, MMP8 | 0,850 | 0,841 | 0,860 | 0,912 | 0,858 | 0,966 | 0,968 | 0,935 | 1 |
| RSAD2, OAS1, IFI44L, SIGLEC1, HERC6, SLC1A2, IL1R2, RETN, MMP8 | 0,850 | 0,841 | 0,860 | 0,911 | 0,855 | 0,966 | 0,968 | 0,933 | 1 |
| RSAD2, IFIT1, IFI44L, ISG15, HERC6, SLC1A2, IL1R2, FAM20A, RETN | 0,850 | 0,840 | 0,861 | 0,915 | 0,863 | 0,967 | 0,923 | 0,831 | 1 |
| OAS1, IFIT1, IFI44L, SIGLEC1, ISG15, SLC1A2, IL1R2, OLAH, RETN | 0,850 | 0,840 | 0,860 | 0,915 | 0,860 | 0,970 | 0,955 | 0,912 | 0,999 |
| RSAD2, OAS1, IFIT1, IFI44L, SIGLEC1, SLC1A2, IL1R2, OLAH, RETN | 0,850 | 0,840 | 0,860 | 0,912 | 0,857 | 0,968 | 0,966 | 0,928 | 1 |
| RSAD2, OAS1, IFI44L, SIGLEC1, ISG15, HERC6, SLC1A2, OLAH, RETN | 0,850 | 0,840 | 0,860 | 0,912 | 0,856 | 0,969 | 0,953 | 0,904 | 1 |
| RSAD2, OAS1, IFIT1, IFI44L, SIGLEC1, ISG15, SLC1A2, IL1R2, OLAH | 0,850 | 0,840 | 0,860 | 0,911 | 0,856 | 0,967 | 0,967 | 0,931 | 1 |
| RSAD2, IFIT1, IFI44L, SIGLEC1, SLC1A2, IL1R2, OLAH, RETN, MMP8 | 0,850 | 0,841 | 0,860 | 0,914 | 0,860 | 0,968 | 0,970 | 0,937 | 1 |
| RSAD2, OAS1, IFIT1, ISG15, HERC6, SLC1A2, OLAH, FAM20A, RETN | 0,850 | 0,840 | 0,861 | 0,912 | 0,857 | 0,968 | 0,932 | 0,851 | 1 |
| RSAD2, OAS1, ISG15, SLC1A2, IL1R2, OLAH, FAM20A, RETN, MMP8 | 0,850 | 0,840 | 0,860 | 0,914 | 0,861 | 0,967 | 0,934 | 0,854 | 1 |
| RSAD2, IFIT1, ISG15, SLC1A2, IL1R2, OLAH, FAM20A, RETN, MMP8 | 0,850 | 0,840 | 0,860 | 0,913 | 0,859 | 0,967 | 0,938 | 0,862 | 1 |

Performances obtenues pour l'identification de la nature virale ou bactérienne de l'infection sur la base des combinaisons à 10 gènes cibles :

[Tableau 14]

| Modèle | AUC modèle | IC 95% | | AUC Train | IC 95% Train | | AUC Test | IC 95% Test | |
|---|---|---|---|---|---|---|---|---|---|
| RSAD2,IFI27,OAS1,IFIT1, SIGLEC1, HERC6, IL1R2, OLAH, FAM20A, MMP8 | 0,888 | 0,878 | 0,897 | 0,938 | 0,890 | 0,987 | 0,951 | 0,890 | 1 |
| IFI27, OAS1, IFIT1, IFI44L, SIGLEC1, HERC6, IL1R2, OLAH, FAM20A, MMP8 | 0,888 | 0,878 | 0,897 | 0,938 | 0,889 | 0,986 | 0,952 | 0,891 | 1 |
| IFI27, IFIT1, IFI44L, SIGLEC1, ISG15, HERC6, IL1R2, OLAH, FAM20A, MMP8 | 0,887 | 0,878 | 0,896 | 0,938 | 0,889 | 0,986 | 0,946 | 0,879 | 1 |
| IFI27, OAS1, IFIT1, SIGLEC1, HERC6, IL1R2, OLAH, FAM20A, RETN, MMP8 | 0,887 | 0,877 | 0,897 | 0,938 | 0,886 | 0,989 | 0,952 | 0,891 | 1 |
| RSAD2, IFI27, IFIT1, IFI44L, SIGLEC1, HERC6, IL1R2, OLAH, FAM20A, MMP8 | 0,887 | 0,878 | 0,895 | 0,938 | 0,890 | 0,987 | 0,951 | 0,889 | 1 |
| IFI27, OAS1, IFIT1, SIGLEC1, ISG15, HERC6, IL1R2, OLAH, FAM20A, MMP8 | 0,887 | 0,877 | 0,897 | 0,938 | 0,889 | 0,987 | 0,953 | 0,891 | 1 |
| RSAD2, IFI27, IFIT1, SIGLEC1, ISG15, HERC6, IL1R2, OLAH, FAM20A, MMP8 | 0,886 | 0,877 | 0,896 | 0,938 | 0,890 | 0,987 | 0,951 | 0,889 | 1 |
| IFI27, OAS1, IFI44L, SIGLEC1, ISG15, HERC6, IL1R2, OLAH, FAM20A, MMP8 | 0,886 | 0,876 | 0,895 | 0,937 | 0,888 | 0,985 | 0,948 | 0,885 | 1 |
| RSAD2,IFI27,OAS1,IFIT1, IFI44L, SIGLEC1, HERC6, OLAH, FAM20A, MMP8 | 0,885 | 0,876 | 0,895 | 0,934 | 0,883 | 0,986 | 0,947 | 0,885 | 1 |
| RSAD2, IFI27, OAS1, SIGLEC1, ISG15, HERC6, IL1R2, OLAH, FAM20A, MMP8 | 0,885 | 0,875 | 0,895 | 0,937 | 0,886 | 0,987 | 0,951 | 0,891 | 1 |
| RSAD2, IFI27, OAS1, IFI44L, SIGLEC1, HERC6, IL1R2, OLAH, FAM20A, MMP8 | 0,885 | 0,876 | 0,895 | 0,936 | 0,887 | 0,985 | 0,951 | 0,890 | 1 |
| IFI27, OAS1, SIGLEC1, ISG15, HERC6, IL1R2, OLAH, FAM20A, RETN, MMP8 | 0,885 | 0,875 | 0,895 | 0,934 | 0,881 | 0,987 | 0,949 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, IL1R2, OLAH, FAM20A, MMP8 | 0,885 | 0,875 | 0,895 | 0,936 | 0,887 | 0,986 | 0,951 | 0,890 | 1 |
| IFI27, OAS1, IFIT1, SIGLEC1, ISG15, HERC6, OLAH, FAM20A, RETN, MMP8 | 0,885 | 0,875 | 0,895 | 0,936 | 0,883 | 0,990 | 0,949 | 0,887 | 1 |
| IFI27, OAS1, IFIT1, IFI44L, SIGLEC1, HERC6, IL1R2, FAM20A, RETN, MMP8 | 0,885 | 0,875 | 0,894 | 0,938 | 0,889 | 0,988 | 0,950 | 0,880 | 1 |
| RSAD2, IFI27, IFI44L, SIGLEC1, ISG15, HERC6, IL1R2, OLAH, FAM20A, MMP8 | 0,885 | 0,875 | 0,894 | 0,938 | 0,889 | 0,986 | 0,948 | 0,885 | 1 |
| RSAD2, IFI27, OAS1, SIGLEC1, HERC6, IL1R2, OLAH, FAM20A, RETN, MMP8 | 0,885 | 0,874 | 0,895 | 0,937 | 0,884 | 0,989 | 0,949 | 0,886 | 1 |
| RSAD2,IFI27,OAS1,IFIT1, SIGLEC1, ISG15, IL1R2, OLAH, FAM20A, MMP8 | 0,885 | 0,875 | 0,894 | 0,937 | 0,887 | 0,987 | 0,952 | 0,891 | 1 |
| RSAD2,IFI27,OAS1,IFIT1, SIGLEC1, HERC6, OLAH, FAM20A, RETN, MMP8 | 0,885 | 0,874 | 0,895 | 0,937 | 0,885 | 0,989 | 0,949 | 0,887 | 1 |
| RSAD2,IFI27,OAS1,IFIT1, SIGLEC1, ISG15, HERC6, OLAH, FAM20A, MMP8 | 0,885 | 0,875 | 0,894 | 0,934 | 0,881 | 0,986 | 0,945 | 0,883 | 1 |
| RSAD2,IFI27,OAS1,IFIT1, IFI44L, SIGLEC1, IL1R2, OLAH, FAM20A, MMP8 | 0,884 | 0,875 | 0,894 | 0,935 | 0,884 | 0,985 | 0,954 | 0,896 | 1 |
| IFI27, OAS1, IFIT1, IFI44L, SIGLEC1, HERC6, OLAH, FAM20A, RETN, MMP8 | 0,884 | 0,874 | 0,895 | 0,938 | 0,886 | 0,990 | 0,947 | 0,884 | 1 |
| IFI27, OAS1, IFIT1, SIGLEC1, ISG15, HERC6, IL1R2, FAM20A, RETN, MMP8 | 0,884 | 0,875 | 0,894 | 0,937 | 0,887 | 0,988 | 0,952 | 0,885 | 1 |
| RSAD2,IFI27,OAS1,IFIT1, IFI44L, SIGLEC1, HERC6, IL1R2, FAM20A, MMP8 | 0,884 | 0,875 | 0,893 | 0,934 | 0,884 | 0,983 | 0,948 | 0,881 | 1 |
| IFI27, IFIT1, IFI44L, SIGLEC1, HERC6, IL1R2, OLAH, FAM20A, RETN, MMP8 | 0,884 | 0,874 | 0,894 | 0,937 | 0,884 | 0,989 | 0,952 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, HERC6, OLAH, FAM20A, RETN, MMP8 | 0,884 | 0,874 | 0,894 | 0,935 | 0,880 | 0,989 | 0,948 | 0,888 | 1 |
| RSAD2, IFI27, SIGLEC1, ISG15, HERC6, IL1R2, OLAH, FAM20A, RETN, MMP8 | 0,884 | 0,873 | 0,894 | 0,936 | 0,884 | 0,988 | 0,950 | 0,887 | 1 |
| IFI27, OAS1, IFIT1, IFI44L, SIGLEC1, ISG15, HERC6, OLAH, FAM20A, MMP8 | 0,883 | 0,873 | 0,894 | 0,933 | 0,879 | 0,986 | 0,946 | 0,884 | 1 |
| RSAD2, IFI27, OAS1, IFIT1, SIGLEC1, HERC6, IL1R2, FAM20A, RETN, MMP8 | 0,883 | 0,874 | 0,893 | 0,937 | 0,887 | 0,988 | 0,951 | 0,883 | 1 |
| RSAD2, IFI27, IFIT1, SIGLEC1, HERC6, IL1R2, OLAH, FAM20A, RETN, MMP8 | 0,883 | 0,873 | 0,893 | 0,938 | 0,887 | 0,989 | 0,951 | 0,888 | 1 |
| RSAD2, IFI27, OAS1, IFIT1, IFI44L, SIGLEC1, HERC6, IL1R2, OLAH, FAM20A | 0,883 | 0,875 | 0,892 | 0,934 | 0,884 | 0,984 | 0,943 | 0,880 | 1 |
| IFI27, IFIT1, SIGLEC1, ISG15, HERC6, IL1R2, OLAH, FAM20A, RETN, MMP8 | 0,883 | 0,873 | 0,894 | 0,937 | 0,885 | 0,988 | 0,954 | 0,891 | 1 |
| IFI27, IFI44L, SIGLEC1, ISG15, HERC6, IL1R2, OLAH, FAM20A, RETN, MMP8 | 0,883 | 0,873 | 0,893 | 0,935 | 0,884 | 0,987 | 0,948 | 0,882 | 1 |
| RSAD2, IFI27, OAS1, IFI44L, SIGLEC1, ISG15, IL1R2, OLAH, FAM20A, MMP8 | 0,883 | 0,873 | 0,893 | 0,936 | 0,886 | 0,985 | 0,952 | 0,893 | 1 |
| RSAD2, IFI27, IFIT1, IFI44L, SIGLEC1, ISG15, IL1R2, OLAH, FAM20A, MMP8 | 0,883 | 0,873 | 0,892 | 0,936 | 0,887 | 0,986 | 0,951 | 0,890 | 1 |
| IFI27, IFIT1, IFI44L, SIGLEC1, ISG15, HERC6, IL1R2, FAM20A, RETN, MMP8 | 0,883 | 0,873 | 0,892 | 0,936 | 0,886 | 0,987 | 0,952 | 0,883 | 1 |
| IFI27, IFIT1, IFI44L, SIGLEC1, ISG15, HERC6, OLAH, FAM20A, RETN, MMP8 | 0,883 | 0,872 | 0,893 | 0,935 | 0,881 | 0,988 | 0,952 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, HERC6, IL1R2, OLAH, FAM20A, RETN, MMP8 | 0,882 | 0,872 | 0,893 | 0,935 | 0,883 | 0,988 | 0,949 | 0,883 | 1 |
| IFI27, OAS1, IFIT1, IFI44L, SIGLEC1, HERC6, IL1R2, OLAH, FAM20A, RETN | 0,882 | 0,873 | 0,891 | 0,933 | 0,881 | 0,985 | 0,940 | 0,876 | 1 |
| RSAD2, IFI27, IFIT1, SIGLEC1, ISG15, HERC6, OLAH, FAM20A, RETN, MMP8 | 0,882 | 0,872 | 0,893 | 0,936 | 0,883 | 0,990 | 0,949 | 0,887 | 1 |
| IFI27, IFIT1, IFI44L, SIGLEC1, ISG15, IL1R2, OLAH, FAM20A, RETN, MMP8 | 0,882 | 0,872 | 0,892 | 0,937 | 0,886 | 0,989 | 0,952 | 0,889 | 1 |
| IFI27, OAS1, IFIT1, SIGLEC1, ISG15, IL1R2, OLAH, FAM20A, RETN, MMP8 | 0,882 | 0,872 | 0,893 | 0,933 | 0,880 | 0,987 | 0,955 | 0,896 | 1 |
| IFI27, IFIT1, IFI44L, SIGLEC1, ISG15, HERC6, IL1R2, OLAH, FAM20A, RETN | 0,882 | 0,873 | 0,891 | 0,932 | 0,879 | 0,985 | 0,945 | 0,881 | 1 |
| RSAD2,IFI27,OAS1,IFIT1, SIGLEC1, ISG15, HERC6, IL1R2, OLAH, FAM20A | 0,882 | 0,873 | 0,891 | 0,933 | 0,881 | 0,984 | 0,939 | 0,874 | 1 |
| IFI27, OAS1, IFIT1, IFI44L, SIGLEC1, ISG15, HERC6, OLAH, FAM20A, RETN | 0,882 | 0,873 | 0,891 | 0,932 | 0,878 | 0,985 | 0,939 | 0,876 | 1 |
| RSAD2, IFI27, IFIT1, IFI44L, SIGLEC1, ISG15, HERC6, OLAH, FAM20A, MMP8 | 0,882 | 0,871 | 0,892 | 0,935 | 0,883 | 0,987 | 0,947 | 0,886 | 1 |
| IFI27, OAS1, IFIT1, SIGLEC1, ISG15, HERC6, IL1R2, OLAH, FAM20A, RETN | 0,882 | 0,872 | 0,891 | 0,932 | 0,879 | 0,985 | 0,937 | 0,872 | 1 |
| RSAD2, IFI27, IFIT1, SIGLEC1, ISG15, HERC6, IL1R2, FAM20A, RETN, MMP8 | 0,882 | 0,872 | 0,891 | 0,938 | 0,887 | 0,988 | 0,950 | 0,881 | 1 |
| IFI27, OAS1, IFIT1, IFI44L, SIGLEC1, ISG15, HERC6, IL1R2, OLAH, FAM20A | 0,882 | 0,872 | 0,891 | 0,934 | 0,882 | 0,985 | 0,940 | 0,875 | 1 |
| IFI27, OAS1, IFIT1, IFI44L, SIGLEC1, ISG15, HERC6, IL1R2, FAM20A, MMP8 | 0,881 | 0,872 | 0,891 | 0,934 | 0,885 | 0,983 | 0,950 | 0,882 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, HERC6, OLAH, FAM20A, RETN | 0,881 | 0,872 | 0,891 | 0,931 | 0,877 | 0,984 | 0,938 | 0,873 | 1 |
| RSAD2, IFI27, OAS1, IFIT1, SIGLEC1, ISG15, HERC6, IL1R2, FAM20A, MMP8 | 0,881 | 0,872 | 0,891 | 0,934 | 0,885 | 0,983 | 0,952 | 0,885 | 1 |
| RSAD2, IFI27, OAS1, IFIT1, SIGLEC1, IL1R2, OLAH, FAM20A, RETN, MMP8 | 0,881 | 0,871 | 0,891 | 0,934 | 0,880 | 0,987 | 0,954 | 0,895 | 1 |
| RSAD2, IFI27, IFIT1, IFI44L, SIGLEC1, HERC6, IL1R2, FAM20A, RETN, MMP8 | 0,881 | 0,871 | 0,891 | 0,938 | 0,888 | 0,988 | 0,950 | 0,880 | 1 |
| RSAD2, IFI27, OAS1, SIGLEC1, ISG15, IL1R2, OLAH, FAM20A, RETN, MMP8 | 0,881 | 0,871 | 0,892 | 0,934 | 0,881 | 0,987 | 0,951 | 0,889 | 1 |
| RSAD2, IFI27, OAS1, IFIT1, SIGLEC1, ISG15, HERC6, OLAH, FAM20A, RETN | 0,881 | 0,872 | 0,890 | 0,932 | 0,879 | 0,985 | 0,937 | 0,872 | 1 |
| RSAD2, IFI27, OAS1, IFIT1, IFI44L, SIGLEC1, ISG15, HERC6, OLAH, FAM20A | 0,881 | 0,871 | 0,891 | 0,932 | 0,879 | 0,985 | 0,939 | 0,875 | 1 |
| RSAD2, IFI27, OAS1, IFIT1, SIGLEC1, HERC6, IL1R2, OLAH, FAM20A, RETN | 0,881 | 0,872 | 0,890 | 0,931 | 0,877 | 0,984 | 0,939 | 0,874 | 1 |
| RSAD2, IFI27, OAS1, SIGLEC1, ISG15, HERC6, IL1R2, FAM20A, RETN, MMP8 | 0,881 | 0,871 | 0,891 | 0,936 | 0,884 | 0,987 | 0,949 | 0,880 | 1 |
| IFI27, OAS1, SIGLEC1, ISG15, HERC6, SLC1A2, IL1R2, OLAH, FAM20A, MMP8 | 0,881 | 0,871 | 0,891 | 0,939 | 0,895 | 0,984 | 0,947 | 0,879 | 1 |
| IFI27, IFIT1, IFI44L, SIGLEC1, ISG15, SLC1A2, IL1R2, OLAH, FAM20A, MMP8 | 0,881 | 0,872 | 0,890 | 0,943 | 0,901 | 0,986 | 0,946 | 0,878 | 1 |
| RSAD2, IFI27, IFIT1, IFI44L, SIGLEC1, ISG15, HERC6, IL1R2, OLAH, FAM20A | 0,881 | 0,872 | 0,890 | 0,933 | 0,881 | 0,985 | 0,943 | 0,879 | 1 |
| IFI27, OAS1, IFIT1, SIGLEC1, HERC6, SLC1A2, IL1R2, OLAH, FAM20A, MMP8 | 0,881 | 0,871 | 0,891 | 0,941 | 0,897 | 0,985 | 0,946 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, ISG15, HERC6, SLC1A2, IL1R2, OLAH, FAM20A, MMP8 | 0,881 | 0,871 | 0,890 | 0,941 | 0,897 | 0,985 | 0,949 | 0,882 | 1 |
| RSAD2, IFI27, IFI44L, SIGLEC1, HERC6, IL1R2, OLAH, FAM20A, RETN, MMP8 | 0,881 | 0,870 | 0,891 | 0,936 | 0,884 | 0,988 | 0,952 | 0,890 | 1 |
| IFI27, OAS1, IFIT1, IFI44L, SIGLEC1, IL1R2, OLAH, FAM20A, RETN, MMP8 | 0,881 | 0,870 | 0,891 | 0,934 | 0,880 | 0,987 | 0,957 | 0,901 | 1 |
| IFI27, OAS1, IFI44L, SIGLEC1, ISG15, HERC6, OLAH, FAM20A, RETN, MMP8 | 0,881 | 0,870 | 0,891 | 0,934 | 0,881 | 0,988 | 0,947 | 0,881 | 1 |
| RSAD2, IFI27, IFIT1, IFI44L, SIGLEC1, HERC6, OLAH, FAM20A, RETN, MMP8 | 0,881 | 0,870 | 0,892 | 0,937 | 0,884 | 0,990 | 0,949 | 0,887 | 1 |
| IFI27, OAS1, IFI44L, SIGLEC1, ISG15, IL1R2, OLAH, FAM20A, RETN, MMP8 | 0,881 | 0,870 | 0,891 | 0,935 | 0,883 | 0,988 | 0,951 | 0,889 | 1 |
| RSAD2, IFI27, OAS1, IFIT1, IFI44L, HERC6, IL1R2, OLAH, FAM20A, MMP8 | 0,881 | 0,871 | 0,891 | 0,933 | 0,881 | 0,984 | 0,932 | 0,858 | 1 |
| IFI27, IFIT1, SIGLEC1, ISG15, HERC6, SLC1A2, IL1R2, OLAH, FAM20A, MMP8 | 0,881 | 0,871 | 0,891 | 0,942 | 0,899 | 0,986 | 0,949 | 0,883 | 1 |
| RSAD2, IFI27, IFIT1, IFI44L, SIGLEC1, ISG15, HERC6, IL1R2, FAM20A, MMP8 | 0,881 | 0,872 | 0,890 | 0,932 | 0,881 | 0,983 | 0,953 | 0,886 | 1 |
| RSAD2, IFI27, OAS1, IFIT1, SIGLEC1, HERC6, SLC1A2, OLAH, FAM20A, MMP8 | 0,881 | 0,870 | 0,891 | 0,939 | 0,893 | 0,986 | 0,941 | 0,876 | 1 |
| IFI27, OAS1, IFIT1, SIGLEC1, HERC6, SLC1A2, OLAH, FAM20A, RETN, MMP8 | 0,881 | 0,870 | 0,891 | 0,940 | 0,894 | 0,986 | 0,943 | 0,877 | 1 |
| IFI27, OAS1, IFIT1, IFI44L, SIGLEC1, HERC6, SLC1A2, OLAH, FAM20A, MMP8 | 0,881 | 0,870 | 0,891 | 0,939 | 0,892 | 0,986 | 0,942 | 0,879 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, HERC6, FAM20A, RETN, MMP8 | 0,880 | 0,870 | 0,891 | 0,932 | 0,879 | 0,986 | 0,942 | 0,873 | 1 |
| RSAD2, IFI27, SIGLEC1, ISG15, HERC6, SLC1A2, IL1R2, OLAH, FAM20A, MMP8 | 0,880 | 0,870 | 0,890 | 0,941 | 0,897 | 0,985 | 0,950 | 0,885 | 1 |
| IFI27, OAS1, IFIT1, SIGLEC1, ISG15, SLC1A2, IL1R2, OLAH, FAM20A, MMP8 | 0,880 | 0,870 | 0,890 | 0,943 | 0,899 | 0,986 | 0,947 | 0,880 | 1 |
| RSAD2, IFI27, IFIT1, SIGLEC1, ISG15, HERC6, IL1R2, OLAH, FAM20A, RETN | 0,880 | 0,871 | 0,889 | 0,933 | 0,881 | 0,986 | 0,940 | 0,875 | 1 |
| RSAD2, IFI27, OAS1, IFI44L, SIGLEC1, ISG15, HERC6, OLAH, FAM20A, MMP8 | 0,880 | 0,870 | 0,891 | 0,933 | 0,879 | 0,986 | 0,946 | 0,884 | 1 |
| RSAD2, IFI27, IFIT1, SIGLEC1, ISG15, IL1R2, OLAH, FAM20A, RETN, MMP8 | 0,880 | 0,870 | 0,890 | 0,935 | 0,883 | 0,988 | 0,953 | 0,892 | 1 |
| IFI27, OAS1, IFI44L, SIGLEC1, ISG15, HERC6, IL1R2, FAM20A, RETN, MMP8 | 0,880 | 0,870 | 0,891 | 0,938 | 0,888 | 0,988 | 0,948 | 0,877 | 1 |
| RSAD2, IFI27, IFIT1, SIGLEC1, ISG15, SLC1A2, IL1R2, OLAH, FAM20A, MMP8 | 0,880 | 0,870 | 0,890 | 0,943 | 0,900 | 0,986 | 0,950 | 0,886 | 1 |
| RSAD2, IFI27, IFIT1, SIGLEC1, HERC6, SLC1A2, IL1R2, OLAH, FAM20A, MMP8 | 0,880 | 0,870 | 0,890 | 0,940 | 0,895 | 0,985 | 0,952 | 0,891 | 1 |
| IFI27, OAS1, IFIT1, SIGLEC1, ISG15, HERC6, SLC1A2, OLAH, FAM20A, MMP8 | 0,880 | 0,869 | 0,891 | 0,942 | 0,897 | 0,987 | 0,942 | 0,877 | 1 |
| RSAD2, IFI27, OAS1, IFIT1, SIGLEC1, ISG15, HERC6, FAM20A, RETN, MMP8 | 0,880 | 0,870 | 0,890 | 0,933 | 0,879 | 0,986 | 0,942 | 0,873 | 1 |
| RSAD2, IFI27, OAS1, IFI44L, SIGLEC1, HERC6, OLAH, FAM20A, RETN, MMP8 | 0,880 | 0,869 | 0,890 | 0,936 | 0,883 | 0,989 | 0,948 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, SLC1A2, IL1R2, OLAH, FAM20A, MMP8 | 0,880 | 0,870 | 0,890 | 0,940 | 0,896 | 0,985 | 0,947 | 0,879 | 1 |
| RSAD2, IFI27, OAS1, IFIT1, IFI44L, SIGLEC1, ISG15, OLAH, FAM20A, MMP8 | 0,880 | 0,869 | 0,890 | 0,930 | 0,874 | 0,987 | 0,949 | 0,891 | 1 |
| RSAD2, IFI27, OAS1, IFIT1, SIGLEC1, SLC1A2, IL1R2, OLAH, FAM20A, MMP8 | 0,880 | 0,870 | 0,889 | 0,939 | 0,892 | 0,985 | 0,947 | 0,881 | 1 |
| IFI27, OAS1, IFI44L, SIGLEC1, ISG15, HERC6, IL1R2, OLAH, FAM20A, RETN | 0,880 | 0,870 | 0,889 | 0,932 | 0,880 | 0,984 | 0,942 | 0,876 | 1 |
| IFI27, IFIT1, IFI44L, SIGLEC1, HERC6, SLC1A2, IL1R2, OLAH, FAM20A, MMP8 | 0,880 | 0,870 | 0,889 | 0,939 | 0,894 | 0,985 | 0,952 | 0,888 | 1 |
| RSAD2, IFI27, OAS1, IFI44L, SIGLEC1, ISG15, HERC6, IL1R2, OLAH, FAM20A | 0,880 | 0,870 | 0,889 | 0,932 | 0,880 | 0,984 | 0,941 | 0,875 | 1 |
| RSAD2, IFI27, OAS1, SIGLEC1, ISG15, HERC6, SLC1A2, OLAH, FAM20A, MMP8 | 0,880 | 0,869 | 0,890 | 0,937 | 0,888 | 0,985 | 0,940 | 0,875 | 1 |
| RSAD2, IFI27, OAS1, IFI44L, ISG15, HERC6, IL1R2, OLAH, FAM20A, MMP8 | 0,880 | 0,869 | 0,890 | 0,931 | 0,879 | 0,983 | 0,928 | 0,854 | 1 |
| IFI27, IFIT1, IFI44L, SIGLEC1, ISG15, HERC6, SLC1A2, OLAH, FAM20A, MMP8 | 0,879 | 0,869 | 0,890 | 0,939 | 0,893 | 0,986 | 0,949 | 0,886 | 1 |
| RSAD2, IFI27, OAS1, IFIT1, IFI44L, SIGLEC1, ISG15, HERC6, FAM20A, RETN | 0,879 | 0,870 | 0,889 | 0,930 | 0,877 | 0,984 | 0,934 | 0,861 | 1 |
| IFI27, OAS1, IFIT1, SIGLEC1, HERC6, SLC1A2, IL1R2, FAM20A, RETN, MMP8 | 0,879 | 0,869 | 0,890 | 0,940 | 0,896 | 0,984 | 0,947 | 0,875 | 1 |
| RSAD2, IFI27, OAS1, IFIT1, SIGLEC1, ISG15, SLC1A2, OLAH, FAM20A, MMP8 | 0,879 | 0,869 | 0,889 | 0,939 | 0,891 | 0,987 | 0,945 | 0,880 | 1 |
| RSAD2, IFI27, OAS1, IFIT1, HERC6, IL1R2, OLAH, FAM20A, RETN, MMP8 | 0,879 | 0,869 | 0,889 | 0,934 | 0,882 | 0,986 | 0,933 | 0,861 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, ISG15, OLAH, FAM20A, RETN, MMP8 | 0,879 | 0,869 | 0,890 | 0,931 | 0,874 | 0,988 | 0,954 | 0,900 | 1 |
| IFI27, OAS1, IFIT1, IFI44L, HERC6, IL1R2, OLAH, FAM20A, RETN, MMP8 | 0,879 | 0,869 | 0,889 | 0,934 | 0,881 | 0,986 | 0,932 | 0,859 | 1 |
| IFI27, OAS1, SIGLEC1, ISG15, HERC6, SLC1A2, OLAH, FAM20A, RETN, MMP8 | 0,879 | 0,868 | 0,890 | 0,940 | 0,894 | 0,987 | 0,942 | 0,876 | 1 |
| IFI27, OAS1, IFIT1, IFI44L, SIGLEC1, ISG15, IL1R2, FAM20A, RETN, MMP8 | 0,879 | 0,869 | 0,889 | 0,935 | 0,884 | 0,986 | 0,952 | 0,885 | 1 |
| RSAD2, IFI27, OAS1, IFI44L, SIGLEC1, HERC6, IL1R2, FAM20A, RETN, MMP8 | 0,879 | 0,868 | 0,890 | 0,937 | 0,886 | 0,987 | 0,948 | 0,878 | 1 |
| RSAD2, IFI27, OAS1, IFIT1, IFI44L, SIGLEC1, SLC1A2, OLAH, FAM20A, MMP8 | 0,879 | 0,869 | 0,889 | 0,937 | 0,887 | 0,988 | 0,945 | 0,882 | 1 |
| IFI27, OAS1, IFIT1, IFI44L, SIGLEC1, ISG15, SLC1A2, OLAH, FAM20A, MMP8 | 0,879 | 0,868 | 0,890 | 0,939 | 0,890 | 0,987 | 0,945 | 0,880 | 1 |
| RSAD2, IFI27, OAS1, SIGLEC1, HERC6, SLC1A2, IL1R2, OLAH, FAM20A, MMP8 | 0,879 | 0,869 | 0,889 | 0,938 | 0,892 | 0,984 | 0,941 | 0,874 | 1 |
| RSAD2, IFI27, OAS1, IFIT1, IFI44L, SIGLEC1, OLAH, FAM20A, RETN, MMP8 | 0,879 | 0,868 | 0,889 | 0,929 | 0,872 | 0,986 | 0,955 | 0,901 | 1 |
| RSAD2, IFI27, IFIT1, IFI44L, SIGLEC1, HERC6, IL1R2, OLAH, FAM20A, RETN | 0,879 | 0,869 | 0,889 | 0,932 | 0,878 | 0,986 | 0,938 | 0,872 | 1 |
| IFI27, OAS1, IFIT1, IFI44L, SIGLEC1, HERC6, SLC1A2, IL1R2, FAM20A, MMP8 | 0,879 | 0,869 | 0,889 | 0,937 | 0,893 | 0,980 | 0,951 | 0,884 | 1 |
| RSAD2, IFI27, IFIT1, IFI44L, SIGLEC1, ISG15, HERC6, OLAH, FAM20A, RETN | 0,879 | 0,869 | 0,889 | 0,933 | 0,879 | 0,986 | 0,940 | 0,876 | 1 |
| IFI27, OAS1, IFIT1, IFI44L, SIGLEC1, ISG15, HERC6, IL1R2, FAM20A, RETN | 0,879 | 0,869 | 0,888 | 0,934 | 0,884 | 0,985 | 0,945 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, ISG15, HERC6, IL1R2, OLAH, FAM20A, RETN, MMP8 | 0,879 | 0,868 | 0,889 | 0,932 | 0,879 | 0,985 | 0,932 | 0,859 | 1 |
| RSAD2, IFI27, IFIT1, IFI44L, SIGLEC1, HERC6, SLC1A2, OLAH, FAM20A, MMP8 | 0,879 | 0,869 | 0,889 | 0,938 | 0,891 | 0,986 | 0,945 | 0,882 | 1 |
| IFI27, OAS1, SIGLEC1, ISG15, SLC1A2, IL1R2, OLAH, FAM20A, RETN, MMP8 | 0,879 | 0,869 | 0,889 | 0,941 | 0,897 | 0,986 | 0,946 | 0,877 | 1 |
| IFI27, OAS1, IFIT1, ISG15, HERC6, IL1R2, OLAH, FAM20A, RETN, MMP8 | 0,879 | 0,868 | 0,889 | 0,932 | 0,879 | 0,985 | 0,928 | 0,853 | 1 |
| IFI27, OAS1, IFIT1, IFI44L, ISG15, HERC6, IL1R2, OLAH, FAM20A, MMP8 | 0,878 | 0,868 | 0,889 | 0,932 | 0,880 | 0,983 | 0,930 | 0,858 | 1 |
| RSAD2, IFI27, OAS1, SIGLEC1, ISG15, HERC6, IL1R2, OLAH, FAM20A, RETN | 0,878 | 0,868 | 0,888 | 0,931 | 0,878 | 0,984 | 0,941 | 0,875 | 1 |
| RSAD2, IFI27, OAS1, IFIT1, IFI44L, SIGLEC1, HERC6, SLC1A2, OLAH, FAM20A | 0,878 | 0,869 | 0,888 | 0,936 | 0,888 | 0,984 | 0,940 | 0,876 | 1 |
| RSAD2, IFI27, OAS1, IFI44L, SIGLEC1, ISG15, HERC6, IL1R2, FAM20A, MMP8 | 0,878 | 0,868 | 0,888 | 0,934 | 0,885 | 0,984 | 0,949 | 0,880 | 1 |
| RSAD2, IFI27, OAS1, IFIT1, ISG15, HERC6, IL1R2, OLAH, FAM20A, MMP8 | 0,878 | 0,868 | 0,889 | 0,930 | 0,878 | 0,982 | 0,932 | 0,862 | 1 |
| IFI27, OAS1, SIGLEC1, HERC6, SLC1A2, IL1R2, OLAH, FAM20A, RETN, MMP8 | 0,878 | 0,868 | 0,889 | 0,938 | 0,891 | 0,984 | 0,945 | 0,876 | 1 |
| RSAD2, IFI27, OAS1, IFIT1, IFI44L, SIGLEC1, ISG15, IL1R2, OLAH, FAM20A | 0,878 | 0,869 | 0,887 | 0,930 | 0,875 | 0,984 | 0,947 | 0,886 | 1 |
| RSAD2, IFI27, OAS1, ISG15, HERC6, IL1R2, OLAH, FAM20A, RETN, MMP8 | 0,878 | 0,868 | 0,889 | 0,934 | 0,882 | 0,986 | 0,932 | 0,858 | 1 |
| RSAD2, IFI27, IFIT1, IFI44L, SIGLEC1, ISG15, HERC6, FAM20A, RETN, MMP8 | 0,878 | 0,869 | 0,888 | 0,931 | 0,877 | 0,985 | 0,941 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, HERC6, IL1R2, OLAH, FAM20A, RETN, MMP8 | 0,878 | 0,867 | 0,889 | 0,933 | 0,881 | 0,986 | 0,929 | 0,855 | 1 |
| IFI27, OAS1, IFIT1, IFI44L, SIGLEC1, ISG15, IL1R2, OLAH, FAM20A, RETN | 0,878 | 0,869 | 0,888 | 0,929 | 0,873 | 0,985 | 0,946 | 0,884 | 1 |
| RSAD2, IFI27, OAS1, IFI44L, SIGLEC1, IL1R2, OLAH, FAM20A, RETN, MMP8 | 0,878 | 0,868 | 0,889 | 0,935 | 0,882 | 0,988 | 0,952 | 0,893 | 1 |
| RSAD2, IFI27, OAS1, IFIT1, IFI44L, SIGLEC1, HERC6, IL1R2, FAM20A, RETN | 0,878 | 0,868 | 0,888 | 0,935 | 0,885 | 0,984 | 0,943 | 0,876 | 1 |
| RSAD2, IFI27, OAS1, IFIT1, IFI44L, SIGLEC1, HERC6, FAM20A, RETN, MMP8 | 0,878 | 0,868 | 0,888 | 0,933 | 0,879 | 0,987 | 0,942 | 0,873 | 1 |
| IFI27, OAS1, IFIT1, IFI44L, SIGLEC1, ISG15, HERC6, SLC1A2, OLAH, FAM20A | 0,878 | 0,868 | 0,888 | 0,939 | 0,893 | 0,985 | 0,933 | 0,863 | 1 |
| RSAD2, IFI27, IFIT1, IFI44L, SIGLEC1, SLC1A2, IL1R2, OLAH, FAM20A, MMP8 | 0,878 | 0,868 | 0,888 | 0,938 | 0,892 | 0,984 | 0,952 | 0,891 | 1 |
| IFI27, IFI44L, SIGLEC1, ISG15, SLC1A2, IL1R2, OLAH, FAM20A, RETN, MMP8 | 0,878 | 0,868 | 0,888 | 0,941 | 0,897 | 0,985 | 0,948 | 0,879 | 1 |
| RSAD2, IFI27, OAS1, IFIT1, IFI44L, HERC6, OLAH, FAM20A, RETN, MMP8 | 0,878 | 0,867 | 0,889 | 0,932 | 0,877 | 0,986 | 0,930 | 0,857 | 1 |
| RSAD2, IFI27, SIGLEC1, ISG15, SLC1A2, IL1R2, OLAH, FAM20A, RETN, MMP8 | 0,878 | 0,868 | 0,888 | 0,944 | 0,902 | 0,987 | 0,948 | 0,881 | 1 |
| RSAD2, IFI27, OAS1, IFIT1, SIGLEC1, ISG15, HERC6, IL1R2, FAM20A, RETN | 0,878 | 0,869 | 0,887 | 0,936 | 0,886 | 0,986 | 0,943 | 0,876 | 1 |
| IFI27, OAS1, IFI44L, SIGLEC1, ISG15, SLC1A2, IL1R2, OLAH, FAM20A, MMP8 | 0,878 | 0,868 | 0,888 | 0,941 | 0,897 | 0,984 | 0,947 | 0,879 | 1 |
| RSAD2, IFI27, IFIT1, SIGLEC1, ISG15, HERC6, SLC1A2, OLAH, FAM20A, MMP8 | 0,878 | 0,867 | 0,889 | 0,941 | 0,895 | 0,987 | 0,941 | 0,875 | 1 |

721

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, HERC6, IL1R2, OLAH, FAM20A, RETN, MMP8 | 0,878 | 0,867 | 0,888 | 0,931 | 0,878 | 0,985 | 0,933 | 0,861 | 1 |
| RSAD2, IFI27, IFI44L, SIGLEC1, ISG15, IL1R2, OLAH, FAM20A, RETN, MMP8 | 0,878 | 0,867 | 0,889 | 0,936 | 0,884 | 0,988 | 0,950 | 0,887 | 1 |
| RSAD2, IFI27, OAS1, IFIT1, SIGLEC1, ISG15, IL1R2, FAM20A, RETN, MMP8 | 0,878 | 0,868 | 0,888 | 0,935 | 0,884 | 0,987 | 0,954 | 0,891 | 1 |
| RSAD2, IFI27, IFIT1, IFI44L, SIGLEC1, IL1R2, OLAH, FAM20A, RETN, MMP8 | 0,878 | 0,867 | 0,889 | 0,935 | 0,882 | 0,988 | 0,953 | 0,895 | 1 |
| IFI27, OAS1, IFIT1, IFI44L, SIGLEC1, SLC1A2, IL1R2, OLAH, FAM20A, MMP8 | 0,878 | 0,867 | 0,888 | 0,939 | 0,893 | 0,985 | 0,946 | 0,879 | 1 |
| RSAD2, IFI27, IFI44L, SIGLEC1, ISG15, HERC6, IL1R2, FAM20A, RETN, MMP8 | 0,878 | 0,868 | 0,888 | 0,938 | 0,887 | 0,988 | 0,950 | 0,880 | 1 |
| RSAD2, IFI27, OAS1, IFIT1, ISG15, HERC6, OLAH, FAM20A, RETN, MMP8 | 0,878 | 0,867 | 0,888 | 0,930 | 0,875 | 0,985 | 0,934 | 0,863 | 1 |
| RSAD2, IFI27, OAS1, IFIT1, IFI44L, SIGLEC1, ISG15, IL1R2, FAM20A, MMP8 | 0,877 | 0,868 | 0,887 | 0,933 | 0,883 | 0,983 | 0,952 | 0,885 | 1 |
| IFI27, OAS1, IFIT1, IFI44L, ISG15, HERC6, OLAH, FAM20A, RETN, MMP8 | 0,877 | 0,867 | 0,888 | 0,932 | 0,878 | 0,986 | 0,932 | 0,859 | 1 |
| IFI27, OAS1, IFIT1, IFI44L, SIGLEC1, HERC6, SLC1A2, OLAH, FAM20A, RETN | 0,877 | 0,867 | 0,888 | 0,937 | 0,891 | 0,984 | 0,937 | 0,871 | 1 |
| RSAD2, IFI27, IFIT1, IFI44L, SIGLEC1, ISG15, SLC1A2, OLAH, FAM20A, MMP8 | 0,877 | 0,867 | 0,888 | 0,939 | 0,891 | 0,987 | 0,943 | 0,878 | 1 |
| RSAD2, IFI27, OAS1, IFIT1, IFI44L, SIGLEC1, ISG15, HERC6, IL1R2, FAM20A | 0,877 | 0,868 | 0,887 | 0,933 | 0,883 | 0,983 | 0,950 | 0,886 | 1 |
| RSAD2, IFI27, OAS1, IFIT1, SIGLEC1, ISG15, IL1R2, OLAH, FAM20A, RETN | 0,877 | 0,868 | 0,887 | 0,928 | 0,872 | 0,985 | 0,947 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, SLC1A2, IL1R2, OLAH, FAM20A, MMP8 | 0,877 | 0,867 | 0,888 | 0,942 | 0,899 | 0,985 | 0,949 | 0,884 | 1 |
| IFI27, IFIT1, IFI44L, SIGLEC1, HERC6, SLC1A2, IL1R2, FAM20A, RETN, MMP8 | 0,877 | 0,868 | 0,887 | 0,939 | 0,893 | 0,985 | 0,954 | 0,886 | 1 |
| RSAD2, IFI27, OAS1, SIGLEC1, HERC6, SLC1A2, OLAH, FAM20A, RETN, MMP8 | 0,877 | 0,867 | 0,888 | 0,939 | 0,891 | 0,987 | 0,945 | 0,881 | 1 |
| IFI27, OAS1, IFI44L, SIGLEC1, ISG15, HERC6, SLC1A2, OLAH, FAM20A, MMP8 | 0,877 | 0,867 | 0,888 | 0,937 | 0,889 | 0,985 | 0,943 | 0,879 | 1 |
| IFI27, OAS1, IFIT1, SIGLEC1, ISG15, HERC6, SLC1A2, IL1R2, OLAH, FAM20A | 0,877 | 0,867 | 0,888 | 0,940 | 0,895 | 0,984 | 0,930 | 0,860 | 1 |
| RSAD2, IFI27, IFI44L, SIGLEC1, HERC6, SLC1A2, IL1R2, OLAH, FAM20A, MMP8 | 0,877 | 0,867 | 0,888 | 0,938 | 0,892 | 0,984 | 0,949 | 0,885 | 1 |
| IFI27, OAS1, IFIT1, IFI44L, SIGLEC1, ISG15, OLAH, FAM20A, RETN, MMP8 | 0,877 | 0,866 | 0,889 | 0,929 | 0,873 | 0,986 | 0,954 | 0,898 | 1 |
| IFI27, IFIT1, SIGLEC1, ISG15, SLC1A2, IL1R2, OLAH, FAM20A, RETN, MMP8 | 0,877 | 0,867 | 0,887 | 0,944 | 0,901 | 0,987 | 0,948 | 0,879 | 1 |
| RSAD2, IFI27, IFIT1, SIGLEC1, HERC6, SLC1A2, IL1R2, FAM20A, RETN, MMP8 | 0,877 | 0,868 | 0,887 | 0,940 | 0,894 | 0,986 | 0,950 | 0,881 | 1 |
| RSAD2, IFI27, IFIT1, ISG15, HERC6, IL1R2, OLAH, FAM20A, RETN, MMP8 | 0,877 | 0,867 | 0,887 | 0,933 | 0,880 | 0,985 | 0,932 | 0,860 | 1 |
| RSAD2, IFI27, OAS1, IFIT1, IFI44L, SIGLEC1, IL1R2, FAM20A, RETN, MMP8 | 0,877 | 0,867 | 0,888 | 0,934 | 0,882 | 0,985 | 0,954 | 0,893 | 1 |
| RSAD2, IFI27, IFIT1, SIGLEC1, ISG15, SLC1A2, OLAH, FAM20A, RETN, MMP8 | 0,877 | 0,867 | 0,887 | 0,938 | 0,889 | 0,986 | 0,948 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, HERC6, IL1R2, OLAH, FAM20A, RETN | 0,877 | 0,867 | 0,887 | 0,932 | 0,880 | 0,984 | 0,943 | 0,878 | 1 |
| IFI27, SIGLEC1, ISG15, HERC6, SLC1A2, IL1R2, OLAH, FAM20A, RETN, MMP8 | 0,877 | 0,867 | 0,887 | 0,940 | 0,895 | 0,984 | 0,947 | 0,877 | 1 |
| IFI27, OAS1, IFI44L, SIGLEC1, HERC6, SLC1A2, IL1R2, OLAH, FAM20A, MMP8 | 0,877 | 0,866 | 0,888 | 0,936 | 0,889 | 0,983 | 0,945 | 0,878 | 1 |
| RSAD2, IFI27, SIGLEC1, HERC6, SLC1A2, IL1R2, OLAH, FAM20A, RETN, MMP8 | 0,877 | 0,866 | 0,888 | 0,939 | 0,892 | 0,986 | 0,947 | 0,881 | 1 |
| RSAD2, IFI27, OAS1, IFIT1, SIGLEC1, HERC6, SLC1A2, IL1R2, OLAH, FAM20A | 0,877 | 0,867 | 0,887 | 0,936 | 0,890 | 0,983 | 0,940 | 0,874 | 1 |
| RSAD2, IFI27, IFIT1, IFI44L, ISG15, HERC6, IL1R2, OLAH, FAM20A, MMP8 | 0,877 | 0,866 | 0,888 | 0,932 | 0,881 | 0,983 | 0,932 | 0,861 | 1 |
| IFI27, OAS1, IFIT1, SIGLEC1, ISG15, SLC1A2, OLAH, FAM20A, RETN, MMP8 | 0,877 | 0,866 | 0,887 | 0,937 | 0,887 | 0,986 | 0,947 | 0,883 | 1 |
| RSAD2, IFI27, IFI44L, SIGLEC1, ISG15, HERC6, IL1R2, OLAH, FAM20A, RETN | 0,877 | 0,867 | 0,887 | 0,931 | 0,878 | 0,983 | 0,940 | 0,874 | 1 |
| RSAD2, IFI27, OAS1, IFI44L, SIGLEC1, ISG15, HERC6, OLAH, FAM20A, RETN | 0,877 | 0,867 | 0,887 | 0,930 | 0,876 | 0,984 | 0,943 | 0,878 | 1 |
| RSAD2, IFI27, IFIT1, SIGLEC1, HERC6, SLC1A2, OLAH, FAM20A, RETN, MMP8 | 0,877 | 0,866 | 0,888 | 0,940 | 0,893 | 0,987 | 0,950 | 0,889 | 1 |
| RSAD2, IFI27, IFIT1, IFI44L, SIGLEC1, ISG15, IL1R2, FAM20A, RETN, MMP8 | 0,877 | 0,867 | 0,887 | 0,937 | 0,886 | 0,987 | 0,951 | 0,882 | 1 |
| IFI27, IFIT1, IFI44L, SIGLEC1, ISG15, HERC6, SLC1A2, IL1R2, FAM20A, MMP8 | 0,877 | 0,867 | 0,886 | 0,938 | 0,895 | 0,981 | 0,953 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, SIGLEC1, ISG15, HERC6, SLC1A2, IL1R2, OLAH, FAM20A | 0,877 | 0,867 | 0,886 | 0,940 | 0,895 | 0,984 | 0,939 | 0,873 | 1 |
| RSAD2, IFI27, OAS1, IFI44L, SIGLEC1, ISG15, IL1R2, OLAH, FAM20A, RETN | 0,877 | 0,867 | 0,886 | 0,927 | 0,872 | 0,982 | 0,948 | 0,886 | 1 |
| IFI27, IFIT1, SIGLEC1, ISG15, HERC6, SLC1A2, OLAH, FAM20A, RETN, MMP8 | 0,877 | 0,866 | 0,887 | 0,941 | 0,895 | 0,986 | 0,951 | 0,889 | 1 |
| RSAD2, IFI27, OAS1, IFI44L, SIGLEC1, HERC6, SLC1A2, OLAH, FAM20A, MMP8 | 0,877 | 0,866 | 0,887 | 0,935 | 0,886 | 0,985 | 0,940 | 0,874 | 1 |
| IFI27, OAS1, IFIT1, IFI44L, SIGLEC1, SLC1A2, IL1R2, FAM20A, RETN, MMP8 | 0,877 | 0,867 | 0,886 | 0,938 | 0,893 | 0,983 | 0,950 | 0,880 | 1 |
| RSAD2, IFI27, IFIT1, IFI44L, SIGLEC1, ISG15, OLAH, FAM20A, RETN, MMP8 | 0,877 | 0,866 | 0,887 | 0,931 | 0,875 | 0,987 | 0,954 | 0,900 | 1 |
| RSAD2, IFI27, OAS1, IFIT1, IFI44L, ISG15, HERC6, OLAH, FAM20A, MMP8 | 0,877 | 0,866 | 0,887 | 0,930 | 0,876 | 0,984 | 0,933 | 0,865 | 1 |
| RSAD2, IFI27, OAS1, IFIT1, SIGLEC1, ISG15, HERC6, SLC1A2, OLAH, FAM20A | 0,876 | 0,866 | 0,887 | 0,938 | 0,891 | 0,984 | 0,936 | 0,867 | 1 |
| IFI27, OAS1, IFIT1, IFI44L, SIGLEC1, HERC6, SLC1A2, IL1R2, OLAH, FAM20A | 0,876 | 0,866 | 0,887 | 0,937 | 0,891 | 0,984 | 0,936 | 0,868 | 1 |
| RSAD2, IFI27, OAS1, IFIT1, IFI44L, SIGLEC1, ISG15, OLAH, FAM20A, RETN | 0,876 | 0,867 | 0,886 | 0,926 | 0,869 | 0,984 | 0,943 | 0,882 | 1 |
| IFI27, OAS1, IFIT1, SIGLEC1, ISG15, SLC1A2, IL1R2, FAM20A, RETN, MMP8 | 0,876 | 0,867 | 0,886 | 0,939 | 0,896 | 0,982 | 0,949 | 0,879 | 1 |
| IFI27, IFIT1, SIGLEC1, HERC6, SLC1A2, IL1R2, OLAH, FAM20A, RETN, MMP8 | 0,876 | 0,866 | 0,886 | 0,940 | 0,894 | 0,986 | 0,952 | 0,886 | 1 |
| IFI27, OAS1, SIGLEC1, ISG15, HERC6, SLC1A2, IL1R2, FAM20A, RETN, MMP8 | 0,876 | 0,866 | 0,886 | 0,938 | 0,894 | 0,983 | 0,949 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, ISG15, HERC6, SLC1A2, IL1R2, FAM20A, MMP8 | 0,876 | 0,867 | 0,886 | 0,938 | 0,896 | 0,981 | 0,952 | 0,883 | 1 |
| RSAD2, IFI27, OAS1, IFIT1, IFI44L, SIGLEC1, IL1R2, OLAH, FAM20A, RETN | 0,876 | 0,866 | 0,886 | 0,926 | 0,870 | 0,983 | 0,948 | 0,885 | 1 |
| RSAD2, IFI27, IFIT1, IFI44L, SIGLEC1, HERC6, SLC1A2, IL1R2, FAM20A, MMP8 | 0,876 | 0,866 | 0,886 | 0,938 | 0,894 | 0,982 | 0,953 | 0,885 | 1 |
| IFI27, OAS1, IFIT1, SIGLEC1, ISG15, HERC6, SLC1A2, OLAH, FAM20A, RETN | 0,876 | 0,866 | 0,886 | 0,939 | 0,895 | 0,984 | 0,930 | 0,859 | 1 |
| RSAD2, IFI27, OAS1, IFIT1, SIGLEC1, HERC6, SLC1A2, IL1R2, FAM20A, MMP8 | 0,876 | 0,866 | 0,886 | 0,936 | 0,892 | 0,980 | 0,950 | 0,882 | 1 |
| IFI27, IFIT1, IFI44L, SIGLEC1, ISG15, SLC1A2, OLAH, FAM20A, RETN, MMP8 | 0,876 | 0,865 | 0,887 | 0,937 | 0,889 | 0,986 | 0,949 | 0,885 | 1 |
| IFI27, OAS1, IFI44L, SIGLEC1, HERC6, SLC1A2, IL1R2, FAM20A, RETN, MMP8 | 0,876 | 0,865 | 0,886 | 0,939 | 0,894 | 0,984 | 0,947 | 0,876 | 1 |
| RSAD2, IFI27, IFIT1, IFI44L, SIGLEC1, ISG15, HERC6, IL1R2, FAM20A, RETN | 0,876 | 0,866 | 0,886 | 0,931 | 0,879 | 0,984 | 0,946 | 0,878 | 1 |
| IFI27, IFIT1, IFI44L, ISG15, HERC6, IL1R2, OLAH, FAM20A, RETN, MMP8 | 0,876 | 0,865 | 0,887 | 0,932 | 0,879 | 0,985 | 0,934 | 0,862 | 1 |
| RSAD2, IFI27, OAS1, IFIT1, IFI44L, ISG15, HERC6, IL1R2, OLAH, FAM20A | 0,876 | 0,866 | 0,886 | 0,930 | 0,877 | 0,983 | 0,928 | 0,855 | 1 |
| IFI27, OAS1, IFI44L, SIGLEC1, ISG15, HERC6, SLC1A2, IL1R2, FAM20A, MMP8 | 0,876 | 0,865 | 0,886 | 0,939 | 0,896 | 0,981 | 0,950 | 0,880 | 1 |
| IFI27, IFIT1, IFI44L, SIGLEC1, ISG15, SLC1A2, IL1R2, FAM20A, RETN, MMP8 | 0,876 | 0,866 | 0,885 | 0,939 | 0,894 | 0,984 | 0,950 | 0,881 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, SLC1A2, IL1R2, OLAH, FAM20A, RETN, MMP8 | 0,876 | 0,865 | 0,887 | 0,936 | 0,889 | 0,984 | 0,952 | 0,890 | 1 |
| IFI27, OAS1, IFIT1, SIGLEC1, ISG15, HERC6, SLC1A2, IL1R2, FAM20A, MMP8 | 0,876 | 0,864 | 0,887 | 0,939 | 0,896 | 0,981 | 0,952 | 0,883 | 1 |
| RSAD2, IFI27, OAS1, SIGLEC1, ISG15, HERC6, SLC1A2, IL1R2, OLAH, FAM20A | 0,876 | 0,866 | 0,886 | 0,937 | 0,890 | 0,983 | 0,935 | 0,864 | 1 |
| IFI27, IFIT1, IFI44L, SIGLEC1, HERC6, SLC1A2, OLAH, FAM20A, RETN, MMP8 | 0,876 | 0,865 | 0,886 | 0,938 | 0,889 | 0,986 | 0,951 | 0,887 | 1 |
| RSAD2, IFI27, IFIT1, SIGLEC1, ISG15, HERC6, SLC1A2, IL1R2, OLAH, FAM20A | 0,876 | 0,866 | 0,885 | 0,939 | 0,895 | 0,984 | 0,934 | 0,865 | 1 |
| IFI27, OAS1, IFIT1, SIGLEC1, HERC6, SLC1A2, IL1R2, OLAH, FAM20A, RETN | 0,876 | 0,865 | 0,886 | 0,939 | 0,894 | 0,984 | 0,936 | 0,869 | 1 |
| RSAD2, IFI27, IFIT1, IFI44L, SIGLEC1, ISG15, HERC6, SLC1A2, OLAH, FAM20A | 0,875 | 0,865 | 0,886 | 0,937 | 0,891 | 0,983 | 0,933 | 0,864 | 1 |
| RSAD2, IFI27, IFIT1, IFI44L, SIGLEC1, HERC6, SLC1A2, IL1R2, OLAH, FAM20A | 0,875 | 0,865 | 0,885 | 0,936 | 0,889 | 0,983 | 0,940 | 0,876 | 1 |
| RSAD2, IFI27, SIGLEC1, ISG15, HERC6, SLC1A2, OLAH, FAM20A, RETN, MMP8 | 0,875 | 0,865 | 0,886 | 0,941 | 0,895 | 0,987 | 0,942 | 0,876 | 1 |
| RSAD2, IFI27, OAS1, IFI44L, SIGLEC1, ISG15, SLC1A2, OLAH, FAM20A, MMP8 | 0,875 | 0,864 | 0,886 | 0,937 | 0,888 | 0,986 | 0,940 | 0,873 | 1 |
| RSAD2, IFI27, IFI44L, SIGLEC1, ISG15, HERC6, OLAH, FAM20A, RETN, MMP8 | 0,875 | 0,864 | 0,887 | 0,936 | 0,883 | 0,989 | 0,948 | 0,886 | 1 |
| IFI27, OAS1, IFIT1, IFI44L, SIGLEC1, SLC1A2, OLAH, FAM20A, RETN, MMP8 | 0,875 | 0,864 | 0,887 | 0,934 | 0,883 | 0,985 | 0,950 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, SLC1A2, IL1R2, OLAH, FAM20A, MMP8 | 0,875 | 0,865 | 0,886 | 0,937 | 0,890 | 0,984 | 0,948 | 0,884 | 1 |
| IFI27, IFIT1, IFI44L, SIGLEC1, ISG15, HERC6, SLC1A2, OLAH, FAM20A, RETN | 0,875 | 0,866 | 0,885 | 0,938 | 0,892 | 0,985 | 0,936 | 0,868 | 1 |
| RSAD2, IFI27, IFIT1, SIGLEC1, ISG15, SLC1A2, IL1R2, FAM20A, RETN, MMP8 | 0,875 | 0,866 | 0,885 | 0,939 | 0,895 | 0,984 | 0,950 | 0,880 | 1 |
| RSAD2, IFI27, IFIT1, IFI44L, SIGLEC1, ISG15, IL1R2, OLAH, FAM20A, RETN | 0,875 | 0,865 | 0,885 | 0,930 | 0,875 | 0,985 | 0,943 | 0,882 | 1 |
| RSAD2, IFI27, OAS1, IFI44L, SIGLEC1, ISG15, IL1R2, FAM20A, RETN, MMP8 | 0,875 | 0,865 | 0,886 | 0,934 | 0,883 | 0,986 | 0,949 | 0,879 | 1 |
| IFI27, OAS1, IFI44L, SIGLEC1, HERC6, SLC1A2, OLAH, FAM20A, RETN, MMP8 | 0,875 | 0,864 | 0,886 | 0,937 | 0,889 | 0,986 | 0,948 | 0,885 | 1 |
| IFI27, IFIT1, IFI44L, SIGLEC1, SLC1A2, IL1R2, OLAH, FAM20A, RETN, MMP8 | 0,875 | 0,865 | 0,886 | 0,939 | 0,893 | 0,985 | 0,950 | 0,883 | 1 |
| RSAD2,IFI27,OAS1,IFIT1, IFI44L, SIGLEC1, ISG15, SLC1A2, OLAH, FAM20A | 0,875 | 0,865 | 0,885 | 0,933 | 0,881 | 0,984 | 0,940 | 0,875 | 1 |
| RSAD2, IFI27, IFI44L, SIGLEC1, ISG15, HERC6, SLC1A2, OLAH, FAM20A, MMP8 | 0,875 | 0,864 | 0,886 | 0,939 | 0,892 | 0,986 | 0,943 | 0,879 | 1 |
| RSAD2, IFI27, OAS1, SIGLEC1, HERC6, SLC1A2, IL1R2, FAM20A, RETN, MMP8 | 0,875 | 0,865 | 0,886 | 0,937 | 0,891 | 0,983 | 0,949 | 0,880 | 1 |
| RSAD2, IFI27, IFIT1, SIGLEC1, SLC1A2, IL1R2, OLAH, FAM20A, RETN, MMP8 | 0,875 | 0,865 | 0,885 | 0,939 | 0,892 | 0,985 | 0,950 | 0,886 | 1 |
| RSAD2,IFI27,OAS1,IFIT1, IFI44L, ISG15, IL1R2, OLAH, FAM20A, MMP8 | 0,875 | 0,864 | 0,886 | 0,929 | 0,876 | 0,982 | 0,935 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, SLC1A2, OLAH, FAM20A, RETN | 0,875 | 0,865 | 0,885 | 0,933 | 0,882 | 0,985 | 0,937 | 0,870 | 1 |
| IFI27, OAS1, IFIT1, IFI44L, SIGLEC1, HERC6, SLC1A2, FAM20A, RETN, MMP8 | 0,875 | 0,865 | 0,885 | 0,935 | 0,885 | 0,985 | 0,941 | 0,872 | 1 |
| IFI27, OAS1, IFI44L, SIGLEC1, ISG15, HERC6, SLC1A2, IL1R2, OLAH, FAM20A | 0,875 | 0,865 | 0,885 | 0,938 | 0,892 | 0,983 | 0,935 | 0,865 | 1 |
| RSAD2, IFI27, IFI44L, ISG15, HERC6, IL1R2, OLAH, FAM20A, RETN, MMP8 | 0,875 | 0,863 | 0,887 | 0,933 | 0,880 | 0,985 | 0,933 | 0,862 | 1 |
| RSAD2, IFI27, OAS1, IFIT1, IFI44L, ISG15, HERC6, OLAH, FAM20A, RETN | 0,875 | 0,865 | 0,885 | 0,931 | 0,879 | 0,984 | 0,926 | 0,852 | 1 |
| IFI27, IFIT1, SIGLEC1, ISG15, HERC6, SLC1A2, IL1R2, FAM20A, RETN, MMP8 | 0,875 | 0,865 | 0,885 | 0,940 | 0,896 | 0,984 | 0,953 | 0,885 | 1 |
| RSAD2, IFI27, OAS1, SIGLEC1, SLC1A2, IL1R2, OLAH, FAM20A, RETN, MMP8 | 0,875 | 0,864 | 0,885 | 0,937 | 0,889 | 0,985 | 0,948 | 0,883 | 1 |
| RSAD2, IFI27, IFI44L, SIGLEC1, HERC6, SLC1A2, OLAH, FAM20A, RETN, MMP8 | 0,875 | 0,864 | 0,885 | 0,938 | 0,890 | 0,986 | 0,948 | 0,883 | 1 |
| RSAD2, IFI27, SIGLEC1, ISG15, HERC6, SLC1A2, IL1R2, FAM20A, RETN, MMP8 | 0,875 | 0,865 | 0,885 | 0,937 | 0,890 | 0,983 | 0,950 | 0,880 | 1 |
| RSAD2, IFI27, OAS1, IFIT1, IFI44L, SIGLEC1, SLC1A2, IL1R2, FAM20A, MMP8 | 0,875 | 0,865 | 0,885 | 0,934 | 0,888 | 0,980 | 0,952 | 0,885 | 1 |
| IFI27, OAS1, IFIT1, IFI44L, ISG15, HERC6, IL1R2, OLAH, FAM20A, RETN | 0,875 | 0,865 | 0,885 | 0,930 | 0,877 | 0,982 | 0,927 | 0,853 | 1 |
| IFI27, OAS1, IFIT1, IFI44L, SIGLEC1, HERC6, SLC1A2, IL1R2, FAM20A, RETN | 0,875 | 0,865 | 0,884 | 0,935 | 0,890 | 0,981 | 0,936 | 0,863 | 1 |
| RSAD2, IFI27, OAS1, IFIT1, SIGLEC1, HERC6, SLC1A2, OLAH, FAM20A, RETN | 0,875 | 0,865 | 0,885 | 0,938 | 0,892 | 0,984 | 0,936 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, HERC6, SLC1A2, OLAH, FAM20A | 0,875 | 0,864 | 0,885 | 0,936 | 0,888 | 0,984 | 0,937 | 0,867 | 1 |
| IFI27, OAS1, IFIT1, IFI44L, SIGLEC1, ISG15, HERC6, SLC1A2, IL1R2, FAM20A | 0,875 | 0,865 | 0,884 | 0,937 | 0,893 | 0,981 | 0,942 | 0,873 | 1 |
| RSAD2, IFI27, OAS1, IFI44L, SIGLEC1, ISG15, OLAH, FAM20A, RETN, MMP8 | 0,875 | 0,863 | 0,886 | 0,931 | 0,876 | 0,987 | 0,950 | 0,891 | 1 |
| RSAD2, IFI27, OAS1, SIGLEC1, ISG15, HERC6, SLC1A2, IL1R2, FAM20A, MMP8 | 0,875 | 0,864 | 0,885 | 0,937 | 0,893 | 0,981 | 0,948 | 0,879 | 1 |
| IFI27, OAS1, IFIT1, IFI44L, SIGLEC1, ISG15, SLC1A2, IL1R2, OLAH, FAM20A | 0,874 | 0,865 | 0,884 | 0,935 | 0,886 | 0,985 | 0,937 | 0,870 | 1 |
| RSAD2, IFI27, IFI44L, ISG15, HERC6, SLC1A2, IL1R2, OLAH, FAM20A, MMP8 | 0,874 | 0,864 | 0,885 | 0,937 | 0,891 | 0,983 | 0,925 | 0,849 | 1 |
| IFI27, IFI44L, SIGLEC1, ISG15, HERC6, SLC1A2, OLAH, FAM20A, RETN, MMP8 | 0,874 | 0,864 | 0,885 | 0,937 | 0,888 | 0,985 | 0,942 | 0,872 | 1 |
| IFI27, OAS1, IFIT1, SIGLEC1, ISG15, SLC1A2, IL1R2, OLAH, FAM20A, RETN | 0,874 | 0,864 | 0,884 | 0,936 | 0,887 | 0,985 | 0,937 | 0,869 | 1 |
| IFI27, OAS1, IFIT1, IFI44L, HERC6, SLC1A2, IL1R2, OLAH, FAM20A, MMP8 | 0,874 | 0,864 | 0,885 | 0,934 | 0,886 | 0,983 | 0,923 | 0,842 | 1 |
| IFI27, OAS1, IFIT1, SIGLEC1, ISG15, HERC6, SLC1A2, FAM20A, RETN, MMP8 | 0,874 | 0,864 | 0,885 | 0,935 | 0,885 | 0,985 | 0,940 | 0,869 | 1 |
| RSAD2, IFI27, IFI44L, SIGLEC1, ISG15, HERC6, SLC1A2, IL1R2, FAM20A, MMP8 | 0,874 | 0,864 | 0,884 | 0,938 | 0,895 | 0,981 | 0,952 | 0,883 | 1 |
| IFI27, OAS1, IFI44L, ISG15, HERC6, SLC1A2, IL1R2, OLAH, FAM20A, MMP8 | 0,874 | 0,863 | 0,886 | 0,937 | 0,890 | 0,983 | 0,922 | 0,841 | 1 |
| IFI27, OAS1, IFI44L, SIGLEC1, ISG15, SLC1A2, OLAH, FAM20A, RETN, MMP8 | 0,874 | 0,863 | 0,886 | 0,935 | 0,885 | 0,985 | 0,943 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, HERC6, IL1R2, OLAH, FAM20A, RETN | 0,874 | 0,865 | 0,884 | 0,930 | 0,879 | 0,982 | 0,928 | 0,855 | 1 |
| RSAD2, IFI27, OAS1, IFIT1, IFI44L, HERC6, IL1R2, OLAH, FAM20A, RETN | 0,874 | 0,864 | 0,884 | 0,931 | 0,879 | 0,983 | 0,927 | 0,853 | 1 |
| IFI27, OAS1, IFIT1, IFI44L, SIGLEC1, HERC6, IL1R2, OLAH, RETN, MMP8 | 0,874 | 0,865 | 0,883 | 0,928 | 0,875 | 0,980 | 0,951 | 0,900 | 1 |
| IFI27, IFIT1, IFI44L, SIGLEC1, HERC6, SLC1A2, IL1R2, OLAH, FAM20A, RETN | 0,874 | 0,864 | 0,884 | 0,937 | 0,889 | 0,984 | 0,941 | 0,875 | 1 |
| IFI27, OAS1, IFIT1, IFI44L, SIGLEC1, ISG15, SLC1A2, IL1R2, FAM20A, MMP8 | 0,874 | 0,864 | 0,884 | 0,937 | 0,894 | 0,981 | 0,951 | 0,882 | 1 |
| RSAD2, IFI27, OAS1, IFIT1, SIGLEC1, HERC6, SLC1A2, FAM20A, RETN, MMP8 | 0,874 | 0,864 | 0,884 | 0,935 | 0,885 | 0,985 | 0,940 | 0,869 | 1 |
| IFI27, OAS1, IFIT1, SIGLEC1, ISG15, HERC6, IL1R2, OLAH, RETN, MMP8 | 0,874 | 0,865 | 0,883 | 0,927 | 0,874 | 0,981 | 0,958 | 0,909 | 1 |
| RSAD2, IFI27, OAS1, IFI44L, ISG15, HERC6, OLAH, FAM20A, RETN, MMP8 | 0,874 | 0,862 | 0,886 | 0,931 | 0,877 | 0,986 | 0,928 | 0,855 | 1 |
| IFI27, OAS1, IFIT1, IFI44L, ISG15, IL1R2, OLAH, FAM20A, RETN, MMP8 | 0,874 | 0,863 | 0,885 | 0,928 | 0,873 | 0,983 | 0,934 | 0,864 | 1 |
| RSAD2, IFI27, OAS1, IFI44L, ISG15, HERC6, IL1R2, OLAH, FAM20A, RETN | 0,874 | 0,863 | 0,885 | 0,932 | 0,880 | 0,983 | 0,929 | 0,856 | 1 |
| IFI27, IFIT1, IFI44L, SIGLEC1, ISG15, SLC1A2, IL1R2, OLAH, FAM20A, RETN | 0,874 | 0,864 | 0,884 | 0,936 | 0,888 | 0,984 | 0,936 | 0,868 | 1 |
| IFI27, OAS1, IFIT1, IFI44L, HERC6, SLC1A2, OLAH, FAM20A, RETN, MMP8 | 0,874 | 0,863 | 0,885 | 0,937 | 0,888 | 0,985 | 0,921 | 0,837 | 1 |
| RSAD2, IFI27, OAS1, IFIT1, SIGLEC1, ISG15, SLC1A2, IL1R2, OLAH, FAM20A | 0,874 | 0,864 | 0,884 | 0,935 | 0,887 | 0,983 | 0,941 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFI44L, SIGLEC1, HERC6, SLC1A2, IL1R2, OLAH, FAM20A, RETN, MMP8 | 0,874 | 0,864 | 0,884 | 0,939 | 0,892 | 0,986 | 0,952 | 0,888 | 1 |
| RSAD2, IFI27, IFI44L, SIGLEC1, ISG15, HERC6, SLC1A2, IL1R2, OLAH, FAM20A | 0,874 | 0,864 | 0,884 | 0,937 | 0,891 | 0,983 | 0,936 | 0,867 | 1 |
| RSAD2, IFI27, OAS1, IFIT1, IFI44L, SIGLEC1, HERC6, OLAH, RETN, MMP8 | 0,874 | 0,865 | 0,883 | 0,927 | 0,874 | 0,981 | 0,947 | 0,889 | 1 |
| RSAD2, IFI27, IFIT1, IFI44L, SIGLEC1, SLC1A2, OLAH, FAM20A, RETN, MMP8 | 0,874 | 0,863 | 0,885 | 0,935 | 0,886 | 0,985 | 0,950 | 0,889 | 1 |
| RSAD2, IFI27, IFI44L, SIGLEC1, HERC6, SLC1A2, IL1R2, FAM20A, RETN, MMP8 | 0,874 | 0,863 | 0,885 | 0,938 | 0,890 | 0,985 | 0,948 | 0,877 | 1 |
| RSAD2, IFI27, OAS1, IFIT1, SIGLEC1, ISG15, SLC1A2, IL1R2, FAM20A, MMP8 | 0,874 | 0,864 | 0,884 | 0,937 | 0,894 | 0,980 | 0,951 | 0,882 | 1 |
| RSAD2, IFI27, OAS1, IFIT1, IFI44L, HERC6, SLC1A2, OLAH, FAM20A, MMP8 | 0,874 | 0,863 | 0,885 | 0,931 | 0,881 | 0,982 | 0,922 | 0,840 | 1 |
| RSAD2, IFI27, OAS1, IFIT1, HERC6, SLC1A2, IL1R2, OLAH, FAM20A, MMP8 | 0,874 | 0,864 | 0,884 | 0,933 | 0,885 | 0,981 | 0,923 | 0,842 | 1 |
| RSAD2, IFI27, OAS1, IFIT1, ISG15, IL1R2, OLAH, FAM20A, RETN, MMP8 | 0,874 | 0,863 | 0,884 | 0,927 | 0,872 | 0,983 | 0,933 | 0,862 | 1 |
| RSAD2, IFI27, OAS1, SIGLEC1, ISG15, SLC1A2, OLAH, FAM20A, RETN, MMP8 | 0,874 | 0,863 | 0,885 | 0,936 | 0,887 | 0,986 | 0,946 | 0,882 | 1 |
| RSAD2, IFI27, IFIT1, IFI44L, SIGLEC1, ISG15, SLC1A2, IL1R2, OLAH, FAM20A | 0,874 | 0,864 | 0,883 | 0,937 | 0,889 | 0,984 | 0,937 | 0,869 | 1 |
| RSAD2, IFI27, OAS1, IFI44L, SIGLEC1, ISG15, HERC6, IL1R2, FAM20A, RETN | 0,874 | 0,863 | 0,884 | 0,934 | 0,884 | 0,984 | 0,943 | 0,875 | 1 |
| IFI27, OAS1, IFI44L, SIGLEC1, ISG15, HERC6, SLC1A2, OLAH, FAM20A, RETN | 0,874 | 0,863 | 0,884 | 0,936 | 0,889 | 0,984 | 0,937 | 0,867 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, SLC1A2, IL1R2, FAM20A, MMP8 | 0,874 | 0,864 | 0,883 | 0,938 | 0,896 | 0,981 | 0,952 | 0,883 | 1 |
| RSAD2, IFI27, OAS1, IFIT1, SIGLEC1, HERC6, IL1R2, OLAH, RETN, MMP8 | 0,874 | 0,864 | 0,883 | 0,927 | 0,874 | 0,981 | 0,958 | 0,909 | 1 |
| RSAD2, IFI27, OAS1, IFIT1, SIGLEC1, SLC1A2, OLAH, FAM20A, RETN, MMP8 | 0,874 | 0,863 | 0,885 | 0,933 | 0,882 | 0,985 | 0,952 | 0,894 | 1 |
| RSAD2, IFI27, IFIT1, HERC6, SLC1A2, IL1R2, OLAH, FAM20A, RETN, MMP8 | 0,874 | 0,864 | 0,884 | 0,933 | 0,884 | 0,983 | 0,923 | 0,843 | 1 |
| IFI27, OAS1, IFIT1, IFI44L, ISG15, HERC6, IL1R2, FAM20A, RETN, MMP8 | 0,874 | 0,863 | 0,884 | 0,932 | 0,880 | 0,984 | 0,929 | 0,848 | 1 |
| IFI27, OAS1, IFIT1, SIGLEC1, ISG15, HERC6, SLC1A2, IL1R2, FAM20A, RETN | 0,874 | 0,863 | 0,884 | 0,936 | 0,891 | 0,981 | 0,938 | 0,866 | 1 |
| IFI27, IFI44L, SIGLEC1, ISG15, HERC6, SLC1A2, IL1R2, FAM20A, RETN, MMP8 | 0,874 | 0,863 | 0,884 | 0,938 | 0,893 | 0,983 | 0,951 | 0,882 | 1 |
| RSAD2, IFI27, IFIT1, IFI44L, SIGLEC1, HERC6, SLC1A2, OLAH, FAM20A, RETN | 0,873 | 0,863 | 0,884 | 0,938 | 0,892 | 0,985 | 0,938 | 0,872 | 1 |
| RSAD2, IFI27, IFIT1, IFI44L, ISG15, HERC6, OLAH, FAM20A, RETN, MMP8 | 0,873 | 0,862 | 0,885 | 0,929 | 0,874 | 0,984 | 0,932 | 0,861 | 1 |
| RSAD2, IFI27, OAS1, IFIT1, IFI44L, IL1R2, OLAH, FAM20A, RETN, MMP8 | 0,873 | 0,863 | 0,884 | 0,929 | 0,874 | 0,983 | 0,932 | 0,861 | 1 |
| IFI27, OAS1, IFIT1, IFI44L, ISG15, HERC6, SLC1A2, OLAH, FAM20A, MMP8 | 0,873 | 0,862 | 0,885 | 0,931 | 0,881 | 0,982 | 0,922 | 0,841 | 1 |
| IFI27, OAS1, SIGLEC1, ISG15, HERC6, SLC1A2, IL1R2, OLAH, FAM20A, RETN | 0,873 | 0,863 | 0,884 | 0,935 | 0,888 | 0,983 | 0,935 | 0,864 | 1 |
| IFI27, OAS1, IFI44L, SIGLEC1, SLC1A2, IL1R2, OLAH, FAM20A, RETN, MMP8 | 0,873 | 0,863 | 0,884 | 0,935 | 0,887 | 0,984 | 0,949 | 0,885 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, HERC6, SLC1A2, IL1R2, OLAH, FAM20A, RETN, MMP8 | 0,873 | 0,863 | 0,884 | 0,936 | 0,888 | 0,984 | 0,920 | 0,836 | 1 |
| RSAD2, IFI27, IFI44L, SIGLEC1, SLC1A2, IL1R2, OLAH, FAM20A, RETN, MMP8 | 0,873 | 0,862 | 0,884 | 0,939 | 0,892 | 0,985 | 0,949 | 0,885 | 1 |
| IFI27, OAS1, IFIT1, IFI44L, SIGLEC1, SLC1A2, IL1R2, OLAH, FAM20A, RETN | 0,873 | 0,863 | 0,883 | 0,932 | 0,881 | 0,984 | 0,939 | 0,873 | 1 |
| RSAD2, IFI27, OAS1, IFI44L, ISG15, HERC6, SLC1A2, OLAH, FAM20A, MMP8 | 0,873 | 0,862 | 0,884 | 0,932 | 0,881 | 0,982 | 0,923 | 0,844 | 1 |
| RSAD2, IFI27, OAS1, SIGLEC1, ISG15, HERC6, SLC1A2, OLAH, FAM20A, RETN | 0,873 | 0,863 | 0,884 | 0,936 | 0,888 | 0,984 | 0,934 | 0,862 | 1 |
| IFI27, IFI44L, SIGLEC1, ISG15, HERC6, SLC1A2, IL1R2, OLAH, FAM20A, RETN | 0,873 | 0,863 | 0,883 | 0,937 | 0,891 | 0,983 | 0,940 | 0,871 | 1 |
| IFI27, OAS1, IFIT1, ISG15, HERC6, SLC1A2, OLAH, FAM20A, RETN, MMP8 | 0,873 | 0,863 | 0,884 | 0,933 | 0,883 | 0,983 | 0,922 | 0,840 | 1 |
| RSAD2, IFI27, OAS1, IFI44L, SIGLEC1, HERC6, IL1R2, OLAH, RETN, MMP8 | 0,873 | 0,864 | 0,883 | 0,927 | 0,875 | 0,980 | 0,953 | 0,903 | 1 |
| IFI27, OAS1, IFIT1, IFI44L, SIGLEC1, ISG15, HERC6, SLC1A2, FAM20A, RETN | 0,873 | 0,863 | 0,883 | 0,932 | 0,884 | 0,981 | 0,938 | 0,868 | 1 |
| RSAD2, IFI27, OAS1, IFI44L, SIGLEC1, ISG15, HERC6, FAM20A, RETN, MMP8 | 0,873 | 0,862 | 0,884 | 0,932 | 0,878 | 0,986 | 0,942 | 0,869 | 1 |
| RSAD2, IFI27, IFIT1, SIGLEC1, ISG15, SLC1A2, IL1R2, OLAH, FAM20A, RETN | 0,873 | 0,863 | 0,883 | 0,937 | 0,889 | 0,985 | 0,937 | 0,870 | 1 |
| RSAD2, IFI27, OAS1, IFIT1, SIGLEC1, ISG15, HERC6, SLC1A2, IL1R2, FAM20A | 0,873 | 0,863 | 0,883 | 0,937 | 0,894 | 0,980 | 0,941 | 0,871 | 1 |
| IFI27, IFI44L, ISG15, HERC6, SLC1A2, IL1R2, OLAH, FAM20A, RETN, MMP8 | 0,873 | 0,863 | 0,883 | 0,935 | 0,888 | 0,982 | 0,922 | 0,841 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, ISG15, HERC6, SLC1A2, IL1R2, OLAH, FAM20A, RETN | 0,873 | 0,862 | 0,884 | 0,940 | 0,895 | 0,985 | 0,937 | 0,870 | 1 |
| IFI27, OAS1, IFI44L, SIGLEC1, ISG15, SLC1A2, IL1R2, FAM20A, RETN, MMP8 | 0,873 | 0,863 | 0,883 | 0,938 | 0,894 | 0,983 | 0,947 | 0,876 | 1 |
| RSAD2, IFI27, IFIT1, IFI44L, HERC6, SLC1A2, IL1R2, OLAH, FAM20A, MMP8 | 0,873 | 0,862 | 0,884 | 0,934 | 0,886 | 0,982 | 0,926 | 0,850 | 1 |
| RSAD2, IFI27, IFIT1, IFI44L, ISG15, HERC6, IL1R2, OLAH, FAM20A, RETN | 0,873 | 0,863 | 0,883 | 0,930 | 0,878 | 0,983 | 0,928 | 0,856 | 1 |
| IFI27, OAS1, IFIT1, IFI44L, SIGLEC1, ISG15, HERC6, OLAH, RETN, MMP8 | 0,873 | 0,863 | 0,882 | 0,926 | 0,871 | 0,980 | 0,952 | 0,900 | 1 |
| RSAD2, IFI27, OAS1, SIGLEC1, ISG15, SLC1A2, IL1R2, OLAH, FAM20A, RETN | 0,873 | 0,862 | 0,883 | 0,933 | 0,884 | 0,983 | 0,938 | 0,868 | 1 |
| RSAD2, IFI27, IFIT1, ISG15, HERC6, SLC1A2, IL1R2, OLAH, FAM20A, MMP8 | 0,873 | 0,863 | 0,883 | 0,933 | 0,886 | 0,981 | 0,926 | 0,849 | 1 |
| IFI27, OAS1, IFI44L, SIGLEC1, ISG15, SLC1A2, IL1R2, OLAH, FAM20A, RETN | 0,873 | 0,863 | 0,883 | 0,934 | 0,885 | 0,984 | 0,940 | 0,873 | 1 |
| RSAD2, IFI27, IFIT1, SIGLEC1, ISG15, HERC6, SLC1A2, OLAH, FAM20A, RETN | 0,873 | 0,862 | 0,883 | 0,938 | 0,893 | 0,983 | 0,934 | 0,866 | 1 |
| RSAD2, IFI27, OAS1, IFIT1, IFI44L, SIGLEC1, SLC1A2, IL1R2, OLAH, FAM20A | 0,873 | 0,863 | 0,883 | 0,933 | 0,882 | 0,984 | 0,940 | 0,874 | 1 |
| RSAD2, IFI27, IFI44L, SIGLEC1, ISG15, HERC6, SLC1A2, OLAH, FAM20A, RETN | 0,873 | 0,863 | 0,883 | 0,934 | 0,886 | 0,982 | 0,935 | 0,865 | 1 |
| RSAD2, IFI27, OAS1, IFI44L, ISG15, IL1R2, OLAH, FAM20A, RETN, MMP8 | 0,873 | 0,861 | 0,884 | 0,930 | 0,875 | 0,984 | 0,930 | 0,860 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, ISG15, HERC6, IL1R2, OLAH, RETN, MMP8 | 0,873 | 0,863 | 0,882 | 0,927 | 0,875 | 0,980 | 0,951 | 0,900 | 1 |
| RSAD2, IFI27, IFIT1, SIGLEC1, HERC6, SLC1A2, IL1R2, OLAH, FAM20A, RETN | 0,873 | 0,863 | 0,883 | 0,938 | 0,893 | 0,984 | 0,939 | 0,873 | 1 |
| RSAD2, IFI27, OAS1, IFIT1, HERC6, SLC1A2, OLAH, FAM20A, RETN, MMP8 | 0,873 | 0,862 | 0,883 | 0,933 | 0,883 | 0,983 | 0,922 | 0,840 | 1 |
| RSAD2, IFI27, IFIT1, IFI44L, ISG15, IL1R2, OLAH, FAM20A, RETN, MMP8 | 0,873 | 0,862 | 0,884 | 0,928 | 0,872 | 0,983 | 0,934 | 0,865 | 1 |
| IFI27, IFIT1, IFI44L, ISG15, HERC6, SLC1A2, IL1R2, OLAH, FAM20A, MMP8 | 0,873 | 0,863 | 0,883 | 0,935 | 0,887 | 0,982 | 0,924 | 0,846 | 1 |
| RSAD2, IFI27, OAS1, IFI44L, SIGLEC1, HERC6, SLC1A2, IL1R2, OLAH, FAM20A | 0,873 | 0,862 | 0,883 | 0,933 | 0,884 | 0,982 | 0,938 | 0,870 | 1 |
| RSAD2, IFI27, OAS1, IFI44L, SIGLEC1, HERC6, SLC1A2, IL1R2, FAM20A, MMP8 | 0,873 | 0,861 | 0,884 | 0,937 | 0,892 | 0,981 | 0,947 | 0,878 | 1 |
| RSAD2, IFI27, IFIT1, IFI44L, SIGLEC1, HERC6, SLC1A2, FAM20A, RETN, MMP8 | 0,873 | 0,863 | 0,882 | 0,931 | 0,879 | 0,984 | 0,942 | 0,868 | 1 |
| RSAD2, IFI27, OAS1, IFIT1, SIGLEC1, ISG15, HERC6, OLAH, RETN, MMP8 | 0,873 | 0,863 | 0,882 | 0,927 | 0,873 | 0,981 | 0,955 | 0,905 | 1 |
| RSAD2, IFI27, OAS1, IFIT1, IFI44L, SIGLEC1, HERC6, SLC1A2, IL1R2, FAM20A | 0,873 | 0,862 | 0,883 | 0,936 | 0,893 | 0,980 | 0,941 | 0,872 | 1 |
| RSAD2, IFI27, OAS1, HERC6, SLC1A2, IL1R2, OLAH, FAM20A, RETN, MMP8 | 0,872 | 0,862 | 0,883 | 0,934 | 0,885 | 0,983 | 0,922 | 0,839 | 1 |
| IFI27, OAS1, IFI44L, ISG15, HERC6, SLC1A2, OLAH, FAM20A, RETN, MMP8 | 0,872 | 0,862 | 0,883 | 0,934 | 0,885 | 0,984 | 0,922 | 0,840 | 1 |
| RSAD2, IFI27, OAS1, IFIT1, SIGLEC1, ISG15, SLC1A2, OLAH, FAM20A, RETN | 0,872 | 0,863 | 0,882 | 0,933 | 0,883 | 0,984 | 0,940 | 0,874 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, SLC1A2, IL1R2, OLAH, FAM20A | 0,872 | 0,862 | 0,883 | 0,936 | 0,889 | 0,983 | 0,943 | 0,875 | 1 |
| RSAD2, IFI27, OAS1, IFIT1, IFI44L, ISG15, SLC1A2, OLAH, FAM20A, MMP8 | 0,872 | 0,862 | 0,883 | 0,930 | 0,877 | 0,983 | 0,923 | 0,846 | 1,000 |
| RSAD2, IFI27, OAS1, SIGLEC1, ISG15, SLC1A2, IL1R2, FAM20A, RETN, MMP8 | 0,872 | 0,862 | 0,883 | 0,937 | 0,892 | 0,982 | 0,948 | 0,877 | 1 |
| RSAD2, IFI27, OAS1, IFIT1, IFI44L, SIGLEC1, SLC1A2, OLAH, FAM20A, RETN | 0,872 | 0,862 | 0,883 | 0,931 | 0,878 | 0,984 | 0,940 | 0,873 | 1 |
| RSAD2, IFI27, OAS1, IFIT1, IFI44L, SIGLEC1, ISG15, IL1R2, FAM20A, RETN | 0,872 | 0,862 | 0,883 | 0,929 | 0,875 | 0,982 | 0,949 | 0,886 | 1 |
| RSAD2, IFI27, OAS1, IFI44L, ISG15, HERC6, SLC1A2, IL1R2, OLAH, FAM20A | 0,872 | 0,862 | 0,883 | 0,935 | 0,887 | 0,982 | 0,917 | 0,833 | 1 |
| RSAD2, IFI27, OAS1, IFI44L, HERC6, SLC1A2, IL1R2, OLAH, FAM20A, MMP8 | 0,872 | 0,861 | 0,884 | 0,935 | 0,888 | 0,983 | 0,923 | 0,843 | 1 |
| RSAD2, IFI27, OAS1, IFIT1, SIGLEC1, SLC1A2, IL1R2, FAM20A, RETN, MMP8 | 0,872 | 0,862 | 0,883 | 0,939 | 0,894 | 0,983 | 0,948 | 0,876 | 1 |
| RSAD2, IFI27, OAS1, IFIT1, IFI44L, SIGLEC1, HERC6, IL1R2, OLAH, RETN | 0,872 | 0,863 | 0,881 | 0,926 | 0,873 | 0,979 | 0,942 | 0,884 | 1 |
| RSAD2, IFI27, OAS1, IFIT1, IFI44L, HERC6, IL1R2, FAM20A, RETN, MMP8 | 0,872 | 0,862 | 0,883 | 0,932 | 0,880 | 0,984 | 0,930 | 0,852 | 1 |
| RSAD2, IFI27, OAS1, IFIT1, IFI44L, SIGLEC1, ISG15, HERC6, OLAH, RETN | 0,872 | 0,864 | 0,881 | 0,925 | 0,871 | 0,979 | 0,941 | 0,883 | 1,000 |
| RSAD2, IFI27, OAS1, IFI44L, SIGLEC1, ISG15, SLC1A2, IL1R2, FAM20A, MMP8 | 0,872 | 0,862 | 0,883 | 0,938 | 0,895 | 0,981 | 0,949 | 0,879 | 1 |
| RSAD2, IFI27, OAS1, SIGLEC1, ISG15, HERC6, IL1R2, OLAH, RETN, MMP8 | 0,872 | 0,862 | 0,882 | 0,927 | 0,872 | 0,981 | 0,955 | 0,905 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, SLC1A2, IL1R2, FAM20A, RETN, MMP8 | 0,872 | 0,861 | 0,883 | 0,938 | 0,891 | 0,985 | 0,952 | 0,883 | 1 |
| RSAD2, IFI27, IFIT1, IFI44L, ISG15, SLC1A2, IL1R2, OLAH, FAM20A, MMP8 | 0,872 | 0,862 | 0,883 | 0,935 | 0,887 | 0,982 | 0,925 | 0,848 | 1 |
| IFI27, OAS1, IFI44L, SIGLEC1, HERC6, SLC1A2, IL1R2, OLAH, FAM20A, RETN | 0,872 | 0,862 | 0,883 | 0,934 | 0,886 | 0,983 | 0,936 | 0,865 | 1 |
| RSAD2, IFI27, OAS1, IFIT1, SIGLEC1, HERC6, SLC1A2, IL1R2, FAM20A, RETN | 0,872 | 0,863 | 0,882 | 0,936 | 0,891 | 0,981 | 0,937 | 0,864 | 1 |
| IFI27, OAS1, IFI44L, HERC6, SLC1A2, IL1R2, OLAH, FAM20A, RETN, MMP8 | 0,872 | 0,861 | 0,883 | 0,937 | 0,889 | 0,984 | 0,923 | 0,841 | 1 |
| RSAD2, IFI27, OAS1, ISG15, HERC6, SLC1A2, IL1R2, OLAH, FAM20A, MMP8 | 0,872 | 0,861 | 0,883 | 0,934 | 0,886 | 0,981 | 0,920 | 0,838 | 1 |
| RSAD2, IFI27, SIGLEC1, ISG15, HERC6, SLC1A2, IL1R2, OLAH, FAM20A, RETN | 0,872 | 0,861 | 0,883 | 0,935 | 0,888 | 0,983 | 0,938 | 0,870 | 1 |
| IFI27, OAS1, IFIT1, ISG15, HERC6, SLC1A2, IL1R2, OLAH, FAM20A, MMP8 | 0,872 | 0,861 | 0,883 | 0,934 | 0,886 | 0,981 | 0,922 | 0,840 | 1 |
| RSAD2, IFI27, OAS1, IFIT1, SIGLEC1, ISG15, HERC6, IL1R2, OLAH, RETN | 0,872 | 0,863 | 0,881 | 0,926 | 0,873 | 0,979 | 0,947 | 0,891 | 1 |
| RSAD2, IFI27, IFI44L, HERC6, SLC1A2, IL1R2, OLAH, FAM20A, RETN, MMP8 | 0,872 | 0,861 | 0,883 | 0,936 | 0,888 | 0,984 | 0,924 | 0,844 | 1 |
| RSAD2, IFI27, OAS1, IFIT1, IFI44L, HERC6, SLC1A2, IL1R2, OLAH, FAM20A | 0,872 | 0,862 | 0,882 | 0,933 | 0,885 | 0,982 | 0,920 | 0,837 | 1 |
| RSAD2, IFI27, IFIT1, IFI44L, SIGLEC1, ISG15, HERC6, SLC1A2, IL1R2, FAM20A | 0,872 | 0,862 | 0,882 | 0,935 | 0,889 | 0,981 | 0,949 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, SLC1A2, OLAH, FAM20A, RETN | 0,872 | 0,862 | 0,882 | 0,935 | 0,885 | 0,985 | 0,939 | 0,873 | 1 |
| IFI27, IFIT1, IFI44L, ISG15, SLC1A2, IL1R2, OLAH, FAM20A, RETN, MMP8 | 0,872 | 0,862 | 0,882 | 0,933 | 0,885 | 0,982 | 0,923 | 0,843 | 1 |
| RSAD2, IFI27, IFIT1, SIGLEC1, ISG15, HERC6, SLC1A2, FAM20A, RETN, MMP8 | 0,872 | 0,862 | 0,882 | 0,930 | 0,878 | 0,983 | 0,943 | 0,870 | 1 |
| RSAD2, IFI27, IFIT1, IFI44L, HERC6, SLC1A2, OLAH, FAM20A, RETN, MMP8 | 0,872 | 0,861 | 0,882 | 0,936 | 0,887 | 0,984 | 0,922 | 0,842 | 1 |
| RSAD2, IFI27, OAS1, IFIT1, SIGLEC1, SLC1A2, IL1R2, OLAH, FAM20A, RETN | 0,872 | 0,862 | 0,882 | 0,933 | 0,883 | 0,983 | 0,943 | 0,878 | 1 |
| RSAD2, IFI27, IFIT1, IFI44L, SIGLEC1, HERC6, IL1R2, OLAH, RETN, MMP8 | 0,872 | 0,863 | 0,881 | 0,926 | 0,873 | 0,980 | 0,952 | 0,903 | 1 |
| IFI27, IFIT1, IFI44L, HERC6, SLC1A2, IL1R2, OLAH, FAM20A, RETN, MMP8 | 0,872 | 0,862 | 0,882 | 0,934 | 0,886 | 0,983 | 0,928 | 0,850 | 1 |
| RSAD2, IFI27, OAS1, IFI44L, ISG15, SLC1A2, IL1R2, OLAH, FAM20A, MMP8 | 0,872 | 0,861 | 0,883 | 0,935 | 0,887 | 0,983 | 0,924 | 0,847 | 1 |
| RSAD2, IFI27, OAS1, IFIT1, ISG15, SLC1A2, IL1R2, OLAH, FAM20A, MMP8 | 0,872 | 0,861 | 0,882 | 0,934 | 0,886 | 0,981 | 0,924 | 0,845 | 1 |
| RSAD2, OAS1, IFIT1, IFI44L, SIGLEC1, HERC6, IL1R2, OLAH, FAM20A, MMP8 | 0,872 | 0,862 | 0,881 | 0,924 | 0,870 | 0,977 | 0,963 | 0,909 | 1 |
| IFI27, IFIT1, IFI44L, SIGLEC1, ISG15, HERC6, SLC1A2, IL1R2, FAM20A, RETN | 0,872 | 0,862 | 0,882 | 0,934 | 0,887 | 0,980 | 0,947 | 0,875 | 1 |
| RSAD2, IFI27, IFIT1, IFI44L, ISG15, HERC6, SLC1A2, OLAH, FAM20A, MMP8 | 0,872 | 0,861 | 0,883 | 0,933 | 0,884 | 0,983 | 0,923 | 0,845 | 1 |
| RSAD2, IFI27, OAS1, IFIT1, IFI44L, SLC1A2, IL1R2, OLAH, FAM20A, MMP8 | 0,872 | 0,862 | 0,882 | 0,934 | 0,884 | 0,983 | 0,926 | 0,850 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, ISG15, HERC6, SLC1A2, IL1R2, OLAH, FAM20A, RETN, MMP8 | 0,872 | 0,861 | 0,882 | 0,935 | 0,888 | 0,982 | 0,924 | 0,844 | 1 |
| RSAD2, IFI27, IFIT1, ISG15, HERC6, SLC1A2, OLAH, FAM20A, RETN, MMP8 | 0,872 | 0,861 | 0,882 | 0,933 | 0,884 | 0,983 | 0,923 | 0,844 | 1 |
| IFI27, OAS1, ISG15, HERC6, SLC1A2, IL1R2, OLAH, FAM20A, RETN, MMP8 | 0,872 | 0,861 | 0,882 | 0,935 | 0,888 | 0,982 | 0,920 | 0,836 | 1 |
| RSAD2, IFI27, OAS1, IFI44L, SIGLEC1, HERC6, SLC1A2, OLAH, FAM20A, RETN | 0,872 | 0,861 | 0,882 | 0,934 | 0,885 | 0,982 | 0,936 | 0,866 | 1 |
| RSAD2, IFI27, OAS1, IFIT1, IFI44L, ISG15, HERC6, SLC1A2, OLAH, FAM20A | 0,872 | 0,861 | 0,882 | 0,933 | 0,884 | 0,982 | 0,918 | 0,836 | 1 |
| IFI27, OAS1, IFIT1, IFI44L, ISG15, HERC6, SLC1A2, OLAH, FAM20A, RETN | 0,871 | 0,861 | 0,882 | 0,935 | 0,887 | 0,982 | 0,918 | 0,835 | 1 |
| RSAD2, IFI27, OAS1, IFI44L, SIGLEC1, ISG15, HERC6, OLAH, RETN, MMP8 | 0,871 | 0,862 | 0,881 | 0,927 | 0,874 | 0,981 | 0,950 | 0,895 | 1 |
| IFI27, OAS1, IFIT1, ISG15, SLC1A2, IL1R2, OLAH, FAM20A, RETN, MMP8 | 0,871 | 0,861 | 0,882 | 0,933 | 0,884 | 0,982 | 0,925 | 0,847 | 1 |
| RSAD2, IFI27, IFI44L, SIGLEC1, ISG15, SLC1A2, IL1R2, FAM20A, RETN, MMP8 | 0,871 | 0,861 | 0,882 | 0,938 | 0,892 | 0,983 | 0,948 | 0,877 | 1 |
| RSAD2, IFI27, IFI44L, SIGLEC1, ISG15, SLC1A2, IL1R2, OLAH, FAM20A, RETN | 0,871 | 0,861 | 0,882 | 0,936 | 0,888 | 0,983 | 0,940 | 0,872 | 1 |
| RSAD2, IFI27, OAS1, IFIT1, ISG15, HERC6, SLC1A2, OLAH, FAM20A, MMP8 | 0,871 | 0,860 | 0,883 | 0,930 | 0,879 | 0,980 | 0,921 | 0,840 | 1 |
| RSAD2, IFI27, OAS1, IFIT1, IFI44L, SIGLEC1, ISG15, HERC6, RETN, MMP8 | 0,871 | 0,862 | 0,881 | 0,920 | 0,863 | 0,978 | 0,943 | 0,883 | 1 |
| IFI27, OAS1, IFIT1, IFI44L, SIGLEC1, ISG15, HERC6, IL1R2, OLAH, RETN | 0,871 | 0,863 | 0,880 | 0,924 | 0,871 | 0,976 | 0,941 | 0,884 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, SLC1A2, IL1R2, FAM20A, RETN, MMP8 | 0,871 | 0,861 | 0,882 | 0,937 | 0,892 | 0,983 | 0,948 | 0,877 | 1 |
| RSAD2, IFI27, OAS1, ISG15, HERC6, SLC1A2, OLAH, FAM20A, RETN, MMP8 | 0,871 | 0,861 | 0,882 | 0,934 | 0,885 | 0,983 | 0,922 | 0,838 | 1 |
| RSAD2, IFI27, OAS1, IFI44L, SIGLEC1, ISG15, SLC1A2, OLAH, FAM20A, RETN | 0,871 | 0,861 | 0,881 | 0,933 | 0,882 | 0,984 | 0,940 | 0,872 | 1 |
| RSAD2, IFI27, OAS1, SIGLEC1, HERC6, SLC1A2, IL1R2, OLAH, FAM20A, RETN | 0,871 | 0,861 | 0,882 | 0,933 | 0,883 | 0,982 | 0,936 | 0,866 | 1 |
| IFI27, OAS1, IFI44L, SIGLEC1, ISG15, HERC6, SLC1A2, IL1R2, FAM20A, RETN | 0,871 | 0,861 | 0,882 | 0,935 | 0,891 | 0,980 | 0,941 | 0,869 | 1 |
| OAS1, IFIT1, SIGLEC1, ISG15, HERC6, IL1R2, OLAH, FAM20A, RETN, MMP8 | 0,871 | 0,862 | 0,880 | 0,923 | 0,867 | 0,979 | 0,963 | 0,909 | 1 |
| RSAD2, IFI27, OAS1, IFI44L, SIGLEC1, ISG15, HERC6, IL1R2, OLAH, RETN | 0,871 | 0,862 | 0,880 | 0,924 | 0,871 | 0,978 | 0,942 | 0,883 | 1 |
| RSAD2, IFI27, OAS1, IFIT1, IFI44L, SIGLEC1, HERC6, SLC1A2, FAM20A, RETN | 0,871 | 0,861 | 0,881 | 0,934 | 0,885 | 0,982 | 0,933 | 0,859 | 1 |
| RSAD2, IFI27, OAS1, IFIT1, ISG15, HERC6, SLC1A2, IL1R2, OLAH, FAM20A | 0,871 | 0,861 | 0,881 | 0,933 | 0,886 | 0,981 | 0,918 | 0,834 | 1 |
| IFI27, OAS1, IFIT1, IFI44L, ISG15, SLC1A2, IL1R2, OLAH, FAM20A, MMP8 | 0,871 | 0,860 | 0,882 | 0,934 | 0,885 | 0,982 | 0,924 | 0,846 | 1 |
| RSAD2, IFI27, IFI44L, SIGLEC1, ISG15, HERC6, IL1R2, OLAH, RETN, MMP8 | 0,871 | 0,862 | 0,880 | 0,928 | 0,876 | 0,980 | 0,952 | 0,902 | 1 |
| RSAD2, IFI27, IFI44L, SIGLEC1, ISG15, SLC1A2, OLAH, FAM20A, RETN, MMP8 | 0,871 | 0,859 | 0,883 | 0,938 | 0,890 | 0,986 | 0,945 | 0,879 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, SLC1A2, OLAH, FAM20A, RETN, MMP8 | 0,871 | 0,860 | 0,882 | 0,933 | 0,881 | 0,985 | 0,950 | 0,889 | 1 |
| RSAD2, IFI27, IFIT1, IFI44L, ISG15, HERC6, IL1R2, FAM20A, RETN, MMP8 | 0,871 | 0,860 | 0,881 | 0,932 | 0,880 | 0,983 | 0,936 | 0,858 | 1 |
| RSAD2, IFI27, IFIT1, IFI44L, SIGLEC1, SLC1A2, IL1R2, OLAH, FAM20A, RETN | 0,871 | 0,860 | 0,882 | 0,931 | 0,879 | 0,983 | 0,942 | 0,878 | 1 |
| RSAD2, OAS1, IFIT1, SIGLEC1, ISG15, HERC6, IL1R2, OLAH, FAM20A, MMP8 | 0,871 | 0,861 | 0,881 | 0,921 | 0,866 | 0,976 | 0,962 | 0,906 | 1 |
| RSAD2, IFI27, OAS1, IFIT1, SIGLEC1, ISG15, HERC6, SLC1A2, FAM20A, RETN | 0,871 | 0,861 | 0,880 | 0,932 | 0,883 | 0,981 | 0,934 | 0,860 | 1 |
| RSAD2, IFI27, OAS1, IFIT1, ISG15, HERC6, IL1R2, FAM20A, RETN, MMP8 | 0,871 | 0,861 | 0,881 | 0,931 | 0,879 | 0,984 | 0,928 | 0,847 | 1 |
| RSAD2, IFI27, IFIT1, SIGLEC1, ISG15, HERC6, IL1R2, OLAH, RETN, MMP8 | 0,871 | 0,862 | 0,880 | 0,926 | 0,872 | 0,979 | 0,952 | 0,901 | 1 |
| RSAD2, IFI27, OAS1, IFI44L, HERC6, SLC1A2, OLAH, FAM20A, RETN, MMP8 | 0,871 | 0,859 | 0,882 | 0,935 | 0,885 | 0,984 | 0,924 | 0,844 | 1 |
| RSAD2, IFI27, OAS1, IFIT1, IFI44L, ISG15, OLAH, FAM20A, RETN, MMP8 | 0,871 | 0,860 | 0,882 | 0,923 | 0,863 | 0,983 | 0,940 | 0,876 | 1 |
| IFI27, OAS1, IFIT1, IFI44L, SIGLEC1, ISG15, SLC1A2, IL1R2, FAM20A, RETN | 0,871 | 0,861 | 0,881 | 0,934 | 0,887 | 0,980 | 0,943 | 0,875 | 1 |
| IFI27, IFIT1, IFI44L, ISG15, HERC6, SLC1A2, OLAH, FAM20A, RETN, MMP8 | 0,871 | 0,860 | 0,881 | 0,932 | 0,881 | 0,983 | 0,924 | 0,843 | 1 |
| IFI27, IFIT1, IFI44L, SIGLEC1, ISG15, HERC6, IL1R2, OLAH, RETN, MMP8 | 0,871 | 0,861 | 0,880 | 0,926 | 0,873 | 0,979 | 0,953 | 0,904 | 1 |
| IFI27, OAS1, IFIT1, IFI44L, ISG15, HERC6, SLC1A2, IL1R2, OLAH, FAM20A | 0,871 | 0,860 | 0,881 | 0,935 | 0,887 | 0,983 | 0,917 | 0,834 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, HERC6, SLC1A2, IL1R2, FAM20A | 0,870 | 0,860 | 0,881 | 0,935 | 0,890 | 0,979 | 0,941 | 0,869 | 1 |
| RSAD2, IFI27, IFIT1, ISG15, SLC1A2, IL1R2, OLAH, FAM20A, RETN, MMP8 | 0,870 | 0,860 | 0,881 | 0,933 | 0,884 | 0,982 | 0,925 | 0,847 | 1 |
| RSAD2, IFI27, OAS1, IFIT1, IFI44L, SIGLEC1, HERC6, IL1R2, RETN, MMP8 | 0,870 | 0,861 | 0,880 | 0,927 | 0,874 | 0,979 | 0,953 | 0,901 | 1 |
| IFI27, OAS1, IFIT1, IFI44L, HERC6, SLC1A2, IL1R2, OLAH, FAM20A, RETN | 0,870 | 0,860 | 0,881 | 0,934 | 0,885 | 0,982 | 0,923 | 0,842 | 1 |
| IFI27, IFIT1, ISG15, HERC6, SLC1A2, IL1R2, OLAH, FAM20A, RETN, MMP8 | 0,870 | 0,860 | 0,881 | 0,935 | 0,887 | 0,982 | 0,925 | 0,846 | 1 |
| RSAD2, IFI27, OAS1, IFIT1, IFI44L, HERC6, IL1R2, OLAH, RETN, MMP8 | 0,870 | 0,861 | 0,880 | 0,923 | 0,867 | 0,979 | 0,949 | 0,895 | 1 |
| RSAD2, IFI27, OAS1, IFIT1, IFI44L, ISG15, HERC6, OLAH, RETN, MMP8 | 0,870 | 0,861 | 0,880 | 0,924 | 0,867 | 0,981 | 0,945 | 0,887 | 1 |
| RSAD2, IFI27, OAS1, IFIT1, IFI44L, HERC6, SLC1A2, OLAH, FAM20A, RETN | 0,870 | 0,860 | 0,880 | 0,934 | 0,886 | 0,982 | 0,922 | 0,839 | 1 |
| RSAD2, IFI27, OAS1, IFIT1, IFI44L, ISG15, HERC6, IL1R2, FAM20A, MMP8 | 0,870 | 0,860 | 0,881 | 0,928 | 0,877 | 0,979 | 0,930 | 0,849 | 1 |
| RSAD2, IFI27, IFIT1, IFI44L, SIGLEC1, ISG15, HERC6, OLAH, RETN, MMP8 | 0,870 | 0,861 | 0,879 | 0,925 | 0,871 | 0,980 | 0,951 | 0,899 | 1 |
| RSAD2, IFI27, OAS1, IFI44L, ISG15, HERC6, IL1R2, FAM20A, RETN, MMP8 | 0,870 | 0,859 | 0,882 | 0,932 | 0,880 | 0,984 | 0,929 | 0,846 | 1 |
| OAS1, IFIT1, IFI44L, SIGLEC1, HERC6, IL1R2, OLAH, FAM20A, RETN, MMP8 | 0,870 | 0,861 | 0,880 | 0,926 | 0,871 | 0,980 | 0,964 | 0,911 | 1 |
| RSAD2, IFI27, IFI44L, ISG15, SLC1A2, IL1R2, OLAH, FAM20A, RETN, MMP8 | 0,870 | 0,859 | 0,881 | 0,935 | 0,887 | 0,984 | 0,924 | 0,845 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, SIGLEC1, HERC6, SLC1A2, IL1R2, OLAH, RETN, MMP8 | 0,870 | 0,860 | 0,880 | 0,932 | 0,884 | 0,980 | 0,949 | 0,890 | 1 |
| RSAD2, IFI27, OAS1, IFIT1, ISG15, HERC6, IL1R2, OLAH, RETN, MMP8 | 0,870 | 0,860 | 0,880 | 0,924 | 0,868 | 0,980 | 0,945 | 0,885 | 1 |
| IFI27, IFIT1, IFI44L, SIGLEC1, ISG15, HERC6, SLC1A2, FAM20A, RETN, MMP8 | 0,870 | 0,860 | 0,881 | 0,930 | 0,877 | 0,983 | 0,951 | 0,879 | 1 |
| RSAD2, IFI27, OAS1, IFIT1, IFI44L, SIGLEC1, ISG15, FAM20A, RETN, MMP8 | 0,870 | 0,859 | 0,881 | 0,923 | 0,864 | 0,982 | 0,947 | 0,877 | 1 |
| RSAD2, IFI27, OAS1, IFIT1, IFI44L, SIGLEC1, ISG15, HERC6, IL1R2, RETN | 0,870 | 0,862 | 0,879 | 0,924 | 0,871 | 0,977 | 0,954 | 0,904 | 1 |
| RSAD2, IFI27, OAS1, IFIT1, IFI44L, ISG15, HERC6, IL1R2, FAM20A, RETN | 0,870 | 0,860 | 0,880 | 0,931 | 0,880 | 0,982 | 0,927 | 0,844 | 1 |
| IFI27, OAS1, IFIT1, IFI44L, ISG15, SLC1A2, OLAH, FAM20A, RETN, MMP8 | 0,870 | 0,859 | 0,881 | 0,931 | 0,878 | 0,984 | 0,924 | 0,846 | 1 |
| RSAD2, IFI27, OAS1, IFIT1, IFI44L, SIGLEC1, ISG15, HERC6, IL1R2, OLAH | 0,870 | 0,861 | 0,879 | 0,927 | 0,878 | 0,976 | 0,947 | 0,896 | 0,998 |
| OAS1, IFIT1, IFI44L, SIGLEC1, ISG15, HERC6, IL1R2, FAM20A, RETN, MMP8 | 0,870 | 0,860 | 0,879 | 0,923 | 0,868 | 0,979 | 0,964 | 0,909 | 1 |
| IFI27, OAS1, IFI44L, ISG15, HERC6, SLC1A2, IL1R2, OLAH, FAM20A, RETN | 0,870 | 0,859 | 0,881 | 0,935 | 0,887 | 0,983 | 0,917 | 0,833 | 1 |
| RSAD2, IFI27, IFIT1, IFI44L, SIGLEC1, HERC6, SLC1A2, IL1R2, FAM20A, RETN | 0,870 | 0,859 | 0,881 | 0,935 | 0,888 | 0,981 | 0,943 | 0,873 | 1 |
| OAS1, IFIT1, IFI44L, SIGLEC1, ISG15, HERC6, IL1R2, OLAH, FAM20A, MMP8 | 0,870 | 0,859 | 0,880 | 0,923 | 0,869 | 0,977 | 0,963 | 0,909 | 1 |
| RSAD2, IFI27, IFI44L, ISG15, HERC6, SLC1A2, OLAH, FAM20A, RETN, MMP8 | 0,870 | 0,858 | 0,881 | 0,936 | 0,888 | 0,985 | 0,923 | 0,843 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, ISG15, HERC6, SLC1A2, OLAH, FAM20A, RETN | 0,870 | 0,859 | 0,880 | 0,934 | 0,885 | 0,983 | 0,918 | 0,834 | 1 |
| IFI27, OAS1, IFIT1, IFI44L, SIGLEC1, ISG15, HERC6, IL1R2, RETN, MMP8 | 0,870 | 0,860 | 0,879 | 0,926 | 0,875 | 0,978 | 0,954 | 0,906 | 1 |
| RSAD2, IFI27, OAS1, IFI44L, ISG15, HERC6, IL1R2, OLAH, RETN, MMP8 | 0,870 | 0,860 | 0,879 | 0,924 | 0,868 | 0,980 | 0,943 | 0,885 | 1 |
| RSAD2, IFI27, OAS1, IFIT1, IFI44L, SIGLEC1, ISG15, SLC1A2, IL1R2, FAM20A | 0,870 | 0,860 | 0,879 | 0,934 | 0,888 | 0,980 | 0,942 | 0,873 | 1 |
| RSAD2, IFI27, OAS1, IFI44L, SIGLEC1, ISG15, HERC6, IL1R2, OLAH, MMP8 | 0,870 | 0,861 | 0,879 | 0,926 | 0,876 | 0,976 | 0,947 | 0,896 | 0,997 |
| RSAD2, IFI27, OAS1, IFIT1, SLC1A2, IL1R2, OLAH, FAM20A, RETN, MMP8 | 0,869 | 0,859 | 0,880 | 0,932 | 0,881 | 0,983 | 0,924 | 0,846 | 1 |
| RSAD2, IFI27, OAS1, ISG15, HERC6, SLC1A2, IL1R2, OLAH, FAM20A, RETN | 0,869 | 0,859 | 0,880 | 0,935 | 0,888 | 0,982 | 0,918 | 0,833 | 1 |
| IFI27, OAS1, IFIT1, IFI44L, ISG15, HERC6, IL1R2, OLAH, RETN, MMP8 | 0,869 | 0,860 | 0,879 | 0,924 | 0,868 | 0,979 | 0,949 | 0,894 | 1 |
| RSAD2, IFI27, OAS1, IFIT1, IFI44L, SIGLEC1, HERC6, IL1R2, OLAH, MMP8 | 0,869 | 0,860 | 0,878 | 0,926 | 0,878 | 0,975 | 0,949 | 0,900 | 0,998 |
| RSAD2, IFI27, IFIT1, IFI44L, SIGLEC1, ISG15, HERC6, SLC1A2, FAM20A, RETN | 0,869 | 0,860 | 0,879 | 0,932 | 0,882 | 0,982 | 0,939 | 0,866 | 1 |
| IFIT1, IFI44L, SIGLEC1, ISG15, HERC6, IL1R2, OLAH, FAM20A, RETN, MMP8 | 0,869 | 0,859 | 0,879 | 0,924 | 0,869 | 0,980 | 0,963 | 0,907 | 1 |
| RSAD2, IFI27, IFIT1, IFI44L, SIGLEC1, ISG15, HERC6, IL1R2, OLAH, RETN | 0,869 | 0,861 | 0,878 | 0,926 | 0,874 | 0,979 | 0,946 | 0,893 | 0,998 |
| RSAD2, IFI27, OAS1, IFIT1, SIGLEC1, ISG15, SLC1A2, IL1R2, FAM20A, RETN | 0,869 | 0,860 | 0,879 | 0,933 | 0,887 | 0,980 | 0,942 | 0,873 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, ISG15, HERC6, SLC1A2, IL1R2, OLAH, FAM20A, RETN | 0,869 | 0,859 | 0,880 | 0,934 | 0,886 | 0,982 | 0,916 | 0,830 | 1 |
| RSAD2, OAS1, IFIT1, IFI44L, SIGLEC1, ISG15, HERC6, IL1R2, FAM20A, MMP8 | 0,869 | 0,859 | 0,879 | 0,922 | 0,867 | 0,977 | 0,963 | 0,907 | 1 |
| RSAD2, IFI27, IFI44L, SIGLEC1, HERC6, SLC1A2, IL1R2, OLAH, FAM20A, RETN | 0,869 | 0,858 | 0,880 | 0,934 | 0,885 | 0,983 | 0,943 | 0,876 | 1 |
| IFI27, OAS1, IFIT1, IFI44L, SIGLEC1, ISG15, HERC6, IL1R2, OLAH, MMP8 | 0,869 | 0,860 | 0,878 | 0,928 | 0,879 | 0,976 | 0,941 | 0,888 | 0,995 |
| RSAD2, OAS1, IFIT1, SIGLEC1, HERC6, IL1R2, OLAH, FAM20A, RETN, MMP8 | 0,869 | 0,860 | 0,879 | 0,924 | 0,869 | 0,980 | 0,962 | 0,907 | 1 |
| RSAD2, OAS1, IFIT1, SIGLEC1, ISG15, HERC6, IL1R2, FAM20A, RETN, MMP8 | 0,869 | 0,860 | 0,878 | 0,923 | 0,867 | 0,979 | 0,961 | 0,903 | 1 |
| IFI27, OAS1, IFI44L, ISG15, SLC1A2, IL1R2, OLAH, FAM20A, RETN, MMP8 | 0,869 | 0,858 | 0,880 | 0,935 | 0,887 | 0,984 | 0,924 | 0,845 | 1 |
| RSAD2, IFI27, OAS1, IFIT1, IFI44L, SIGLEC1, ISG15, HERC6, FAM20A, MMP8 | 0,869 | 0,859 | 0,879 | 0,923 | 0,866 | 0,981 | 0,937 | 0,865 | 1 |
| RSAD2, IFI27, IFIT1, SIGLEC1, ISG15, HERC6, SLC1A2, IL1R2, FAM20A, RETN | 0,869 | 0,859 | 0,879 | 0,934 | 0,887 | 0,981 | 0,945 | 0,873 | 1 |
| RSAD2, IFI27, OAS1, IFIT1, IFI44L, ISG15, HERC6, FAM20A, RETN, MMP8 | 0,869 | 0,858 | 0,880 | 0,925 | 0,868 | 0,983 | 0,932 | 0,853 | 1 |
| RSAD2, IFI27, OAS1, SIGLEC1, ISG15, HERC6, SLC1A2, FAM20A, RETN, MMP8 | 0,869 | 0,858 | 0,880 | 0,930 | 0,876 | 0,984 | 0,941 | 0,867 | 1 |
| RSAD2, IFI27, OAS1, IFIT1, IFI44L, ISG15, IL1R2, OLAH, FAM20A, RETN | 0,869 | 0,858 | 0,880 | 0,924 | 0,867 | 0,981 | 0,929 | 0,859 | 1,000 |
| RSAD2, IFI27, IFIT1, IFI44L, ISG15, HERC6, SLC1A2, IL1R2, OLAH, FAM20A | 0,869 | 0,859 | 0,879 | 0,935 | 0,888 | 0,983 | 0,917 | 0,836 | 0,999 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SLC1A2, IL1R2, OLAH, FAM20A, RETN, MMP8 | 0,869 | 0,859 | 0,879 | 0,934 | 0,885 | 0,984 | 0,926 | 0,850 | 1 |
| IFI27, OAS1, IFIT1, SIGLEC1, ISG15, HERC6, SLC1A2, IL1R2, OLAH, RETN | 0,869 | 0,859 | 0,879 | 0,931 | 0,882 | 0,981 | 0,939 | 0,876 | 1 |
| RSAD2,IFI27,OAS1,IFIT1, SIGLEC1, ISG15, HERC6, IL1R2, RETN, MMP8 | 0,869 | 0,860 | 0,878 | 0,925 | 0,872 | 0,978 | 0,953 | 0,900 | 1 |
| RSAD2,IFI27,OAS1,IFIT1, ISG15, SLC1A2, OLAH, FAM20A, RETN, MMP8 | 0,869 | 0,858 | 0,880 | 0,929 | 0,876 | 0,982 | 0,927 | 0,852 | 1 |
| RSAD2, IFI27, IFIT1, IFI44L, HERC6, SLC1A2, IL1R2, OLAH, FAM20A, RETN | 0,869 | 0,859 | 0,879 | 0,934 | 0,885 | 0,983 | 0,920 | 0,838 | 1 |
| IFI27, OAS1, SIGLEC1, ISG15, HERC6, SLC1A2, IL1R2, OLAH, RETN, MMP8 | 0,869 | 0,859 | 0,879 | 0,932 | 0,883 | 0,981 | 0,947 | 0,886 | 1 |
| RSAD2, IFIT1, IFI44L, SIGLEC1, ISG15, HERC6, IL1R2, OLAH, FAM20A, MMP8 | 0,869 | 0,858 | 0,879 | 0,923 | 0,869 | 0,977 | 0,963 | 0,909 | 1 |
| RSAD2,IFI27,OAS1,IFIT1, IFI44L, ISG15, HERC6, IL1R2, OLAH, RETN | 0,869 | 0,860 | 0,878 | 0,923 | 0,867 | 0,978 | 0,939 | 0,879 | 0,999 |
| RSAD2, IFI27, OAS1, IFI44L, SIGLEC1, SLC1A2, IL1R2, OLAH, FAM20A, RETN | 0,869 | 0,858 | 0,880 | 0,930 | 0,878 | 0,983 | 0,943 | 0,878 | 1 |
| RSAD2, IFI27, IFIT1, IFI44L, ISG15, SLC1A2, OLAH, FAM20A, RETN, MMP8 | 0,869 | 0,858 | 0,880 | 0,931 | 0,879 | 0,984 | 0,925 | 0,848 | 1 |
| RSAD2, IFI27, OAS1, IFI44L, SIGLEC1, HERC6, SLC1A2, IL1R2, FAM20A, RETN | 0,869 | 0,858 | 0,879 | 0,935 | 0,890 | 0,980 | 0,939 | 0,865 | 1 |
| IFI27, OAS1, IFIT1, IFI44L, SIGLEC1, HERC6, SLC1A2, OLAH, RETN, MMP8 | 0,869 | 0,859 | 0,879 | 0,932 | 0,883 | 0,980 | 0,947 | 0,887 | 1 |
| IFI27, OAS1, IFIT1, IFI44L, SLC1A2, IL1R2, OLAH, FAM20A, RETN, MMP8 | 0,869 | 0,858 | 0,879 | 0,934 | 0,885 | 0,984 | 0,925 | 0,848 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, HERC6, SLC1A2, IL1R2, OLAH, FAM20A, RETN | 0,869 | 0,859 | 0,879 | 0,933 | 0,884 | 0,981 | 0,917 | 0,831 | 1 |
| RSAD2, IFI27, IFIT1, IFI44L, ISG15, HERC6, SLC1A2, OLAH, FAM20A, RETN | 0,869 | 0,858 | 0,879 | 0,936 | 0,888 | 0,984 | 0,918 | 0,837 | 1 |
| RSAD2, IFI27, OAS1, IFIT1, SIGLEC1, HERC6, SLC1A2, IL1R2, OLAH, RETN | 0,869 | 0,859 | 0,878 | 0,931 | 0,881 | 0,982 | 0,946 | 0,885 | 1 |
| IFI27, OAS1, IFIT1, SIGLEC1, ISG15, HERC6, SLC1A2, OLAH, RETN, MMP8 | 0,869 | 0,858 | 0,879 | 0,931 | 0,882 | 0,980 | 0,948 | 0,888 | 1 |
| IFI27, OAS1, IFIT1, IFI44L, HERC6, SLC1A2, IL1R2, FAM20A, RETN, MMP8 | 0,868 | 0,858 | 0,878 | 0,933 | 0,884 | 0,982 | 0,927 | 0,843 | 1 |
| RSAD2, IFI27, OAS1, IFIT1, SIGLEC1, HERC6, SLC1A2, OLAH, RETN, MMP8 | 0,868 | 0,858 | 0,879 | 0,932 | 0,883 | 0,982 | 0,950 | 0,892 | 1 |
| RSAD2, IFI27, OAS1, IFIT1, IFI44L, SIGLEC1, HERC6, SLC1A2, OLAH, RETN | 0,868 | 0,859 | 0,877 | 0,931 | 0,882 | 0,980 | 0,945 | 0,882 | 1 |
| RSAD2, IFI27, OAS1, IFIT1, IFI44L, SIGLEC1, ISG15, HERC6, OLAH, MMP8 | 0,868 | 0,859 | 0,877 | 0,925 | 0,873 | 0,977 | 0,941 | 0,886 | 0,997 |
| RSAD2, IFIT1, SIGLEC1, ISG15, HERC6, IL1R2, OLAH, FAM20A, RETN, MMP8 | 0,868 | 0,859 | 0,878 | 0,923 | 0,867 | 0,979 | 0,962 | 0,906 | 1 |
| RSAD2, IFI27, IFI44L, ISG15, HERC6, SLC1A2, IL1R2, OLAH, FAM20A, RETN | 0,868 | 0,858 | 0,879 | 0,935 | 0,888 | 0,983 | 0,916 | 0,832 | 1 |
| RSAD2, IFI27, OAS1, ISG15, SLC1A2, IL1R2, OLAH, FAM20A, RETN, MMP8 | 0,868 | 0,858 | 0,879 | 0,935 | 0,887 | 0,983 | 0,922 | 0,841 | 1 |
| RSAD2, IFI27, OAS1, SIGLEC1, ISG15, HERC6, SLC1A2, IL1R2, FAM20A, RETN | 0,868 | 0,857 | 0,879 | 0,934 | 0,888 | 0,980 | 0,941 | 0,870 | 1 |
| RSAD2, IFI27, OAS1, IFI44L, SIGLEC1, ISG15, HERC6, IL1R2, RETN, MMP8 | 0,868 | 0,859 | 0,877 | 0,926 | 0,874 | 0,979 | 0,955 | 0,905 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, SIGLEC1, HERC6, SLC1A2, IL1R2, OLAH, RETN, MMP8 | 0,868 | 0,858 | 0,878 | 0,932 | 0,883 | 0,980 | 0,951 | 0,899 | 1 |
| RSAD2, IFIT1, IFI44L, SIGLEC1, HERC6, SLC1A2, IL1R2, OLAH, FAM20A, MMP8 | 0,868 | 0,858 | 0,878 | 0,929 | 0,883 | 0,976 | 0,955 | 0,891 | 1 |
| RSAD2, OAS1, IFIT1, IFI44L, SIGLEC1, HERC6, IL1R2, FAM20A, RETN, MMP8 | 0,868 | 0,858 | 0,878 | 0,924 | 0,869 | 0,979 | 0,963 | 0,908 | 1 |
| RSAD2, IFI27, OAS1, IFIT1, ISG15, HERC6, SLC1A2, OLAH, FAM20A, RETN | 0,868 | 0,857 | 0,878 | 0,934 | 0,885 | 0,982 | 0,916 | 0,829 | 1 |
| IFI27, OAS1, IFIT1, IFI44L, SIGLEC1, ISG15, IL1R2, OLAH, RETN, MMP8 | 0,868 | 0,858 | 0,877 | 0,925 | 0,871 | 0,979 | 0,954 | 0,901 | 1 |
| RSAD2, IFI27, IFIT1, ISG15, HERC6, SLC1A2, IL1R2, OLAH, FAM20A, RETN | 0,868 | 0,858 | 0,878 | 0,930 | 0,880 | 0,981 | 0,915 | 0,830 | 1 |
| RSAD2, IFI27, IFIT1, IFI44L, ISG15, HERC6, IL1R2, OLAH, RETN, MMP8 | 0,868 | 0,858 | 0,877 | 0,924 | 0,869 | 0,979 | 0,946 | 0,891 | 1 |
| IFIT1, IFI44L, SIGLEC1, ISG15, HERC6, SLC1A2, IL1R2, OLAH, FAM20A, MMP8 | 0,868 | 0,858 | 0,877 | 0,928 | 0,880 | 0,975 | 0,960 | 0,897 | 1 |
| RSAD2, IFI27, IFIT1, IFI44L, SIGLEC1, ISG15, SLC1A2, IL1R2, FAM20A, RETN | 0,868 | 0,858 | 0,877 | 0,934 | 0,888 | 0,980 | 0,943 | 0,874 | 1 |
| RSAD2, IFI27, OAS1, IFI44L, HERC6, SLC1A2, IL1R2, OLAH, FAM20A, RETN | 0,868 | 0,857 | 0,878 | 0,932 | 0,883 | 0,982 | 0,918 | 0,835 | 1 |
| IFI27, IFIT1, IFI44L, ISG15, HERC6, SLC1A2, IL1R2, OLAH, FAM20A, RETN | 0,868 | 0,857 | 0,878 | 0,933 | 0,883 | 0,982 | 0,916 | 0,833 | 1,000 |
| RSAD2, IFI27, OAS1, SIGLEC1, HERC6, SLC1A2, IL1R2, OLAH, RETN, MMP8 | 0,867 | 0,857 | 0,878 | 0,931 | 0,882 | 0,980 | 0,948 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, HERC6, IL1R2, OLAH, MMP8 | 0,867 | 0,859 | 0,876 | 0,928 | 0,879 | 0,977 | 0,940 | 0,886 | 0,995 |
| IFI27, OAS1, IFIT1, IFI44L, ISG15, SLC1A2, IL1R2, OLAH, FAM20A, RETN | 0,867 | 0,857 | 0,878 | 0,930 | 0,877 | 0,982 | 0,921 | 0,840 | 1 |
| IFI27, OAS1, IFI44L, SIGLEC1, ISG15, HERC6, SLC1A2, FAM20A, RETN, MMP8 | 0,867 | 0,856 | 0,879 | 0,932 | 0,879 | 0,984 | 0,942 | 0,869 | 1 |
| OAS1, IFIT1, IFI44L, SIGLEC1, HERC6, SLC1A2, IL1R2, OLAH, FAM20A, MMP8 | 0,867 | 0,857 | 0,878 | 0,929 | 0,882 | 0,976 | 0,954 | 0,892 | 1 |
| RSAD2, IFI27, OAS1, IFIT1, SIGLEC1, ISG15, HERC6, IL1R2, OLAH, MMP8 | 0,867 | 0,858 | 0,877 | 0,924 | 0,875 | 0,974 | 0,943 | 0,891 | 0,996 |
| IFI27, OAS1, IFI44L, SIGLEC1, HERC6, SLC1A2, IL1R2, OLAH, RETN, MMP8 | 0,867 | 0,857 | 0,877 | 0,931 | 0,882 | 0,980 | 0,949 | 0,891 | 1 |
| RSAD2, IFI27, OAS1, IFIT1, IFI44L, ISG15, SLC1A2, IL1R2, OLAH, FAM20A | 0,867 | 0,857 | 0,877 | 0,931 | 0,879 | 0,982 | 0,921 | 0,839 | 1 |
| RSAD2, IFI27, OAS1, IFI44L, SIGLEC1, HERC6, SLC1A2, OLAH, RETN, MMP8 | 0,867 | 0,857 | 0,877 | 0,931 | 0,882 | 0,981 | 0,949 | 0,890 | 1 |
| RSAD2, IFI27, OAS1, IFI44L, SLC1A2, IL1R2, OLAH, FAM20A, RETN, MMP8 | 0,867 | 0,856 | 0,878 | 0,937 | 0,889 | 0,985 | 0,927 | 0,851 | 1 |
| RSAD2, IFI27, OAS1, IFIT1, IFI44L, SIGLEC1, IL1R2, OLAH, RETN, MMP8 | 0,867 | 0,858 | 0,877 | 0,925 | 0,872 | 0,979 | 0,957 | 0,903 | 1 |
| RSAD2, IFI27, OAS1, IFIT1, IFI44L, SIGLEC1, SLC1A2, IL1R2, FAM20A, RETN | 0,867 | 0,857 | 0,878 | 0,934 | 0,886 | 0,981 | 0,943 | 0,877 | 1 |
| RSAD2, OAS1, IFIT1, SIGLEC1, HERC6, SLC1A2, IL1R2, OLAH, FAM20A, MMP8 | 0,867 | 0,857 | 0,877 | 0,928 | 0,882 | 0,975 | 0,962 | 0,904 | 1 |
| RSAD2, IFI27, OAS1, IFI44L, SIGLEC1, ISG15, SLC1A2, IL1R2, FAM20A, RETN | 0,867 | 0,857 | 0,878 | 0,934 | 0,888 | 0,979 | 0,941 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, SIGLEC1, ISG15, HERC6, SLC1A2, IL1R2, OLAH, RETN | 0,867 | 0,858 | 0,877 | 0,929 | 0,879 | 0,980 | 0,946 | 0,885 | 1 |
| IFI27, OAS1, IFIT1, IFI44L, SIGLEC1, ISG15, HERC6, SLC1A2, FAM20A, MMP8 | 0,867 | 0,857 | 0,877 | 0,927 | 0,876 | 0,978 | 0,935 | 0,859 | 1 |
| RSAD2, IFI27, IFIT1, IFI44L, ISG15, SLC1A2, IL1R2, OLAH, FAM20A, RETN | 0,867 | 0,857 | 0,877 | 0,931 | 0,880 | 0,982 | 0,917 | 0,836 | 0,999 |
| IFI27, OAS1, IFIT1, IFI44L, SIGLEC1, ISG15, SLC1A2, FAM20A, RETN, MMP8 | 0,867 | 0,856 | 0,878 | 0,927 | 0,871 | 0,982 | 0,941 | 0,867 | 1 |
| RSAD2, IFI27, IFI44L, SIGLEC1, HERC6, SLC1A2, IL1R2, OLAH, RETN, MMP8 | 0,867 | 0,858 | 0,876 | 0,930 | 0,880 | 0,979 | 0,952 | 0,901 | 1 |
| RSAD2, IFI27, OAS1, IFIT1, IFI44L, SLC1A2, OLAH, FAM20A, RETN, MMP8 | 0,867 | 0,856 | 0,878 | 0,929 | 0,874 | 0,983 | 0,928 | 0,853 | 1 |
| RSAD2, OAS1, IFIT1, IFI44L, SIGLEC1, ISG15, IL1R2, OLAH, FAM20A, MMP8 | 0,867 | 0,857 | 0,877 | 0,920 | 0,863 | 0,976 | 0,966 | 0,913 | 1 |
| RSAD2, IFI27, IFIT1, IFI44L, SIGLEC1, ISG15, IL1R2, OLAH, RETN, MMP8 | 0,867 | 0,858 | 0,876 | 0,924 | 0,870 | 0,979 | 0,954 | 0,902 | 1 |
| RSAD2, IFI27, OAS1, IFI44L, SIGLEC1, HERC6, SLC1A2, IL1R2, OLAH, RETN | 0,867 | 0,857 | 0,876 | 0,931 | 0,882 | 0,980 | 0,945 | 0,883 | 1 |
| RSAD2, IFI27, OAS1, IFI44L, ISG15, SLC1A2, OLAH, FAM20A, RETN, MMP8 | 0,867 | 0,855 | 0,879 | 0,930 | 0,877 | 0,983 | 0,927 | 0,851 | 1 |
| RSAD2, OAS1, IFIT1, IFI44L, SIGLEC1, HERC6, SLC1A2, OLAH, FAM20A, MMP8 | 0,867 | 0,856 | 0,878 | 0,928 | 0,879 | 0,977 | 0,936 | 0,864 | 1 |
| IFI27, OAS1, IFIT1, IFI44L, SIGLEC1, HERC6, SLC1A2, IL1R2, OLAH, RETN | 0,867 | 0,857 | 0,876 | 0,929 | 0,880 | 0,979 | 0,939 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, ISG15, HERC6, IL1R2, FAM20A, RETN, MMP8 | 0,867 | 0,857 | 0,876 | 0,924 | 0,868 | 0,980 | 0,961 | 0,903 | 1 |
| RSAD2, IFI27, OAS1, IFIT1, ISG15, SLC1A2, IL1R2, OLAH, FAM20A, RETN | 0,867 | 0,857 | 0,877 | 0,930 | 0,880 | 0,981 | 0,921 | 0,838 | 1 |
| IFI27, OAS1, IFIT1, IFI44L, SIGLEC1, ISG15, HERC6, SLC1A2, OLAH, RETN | 0,867 | 0,857 | 0,876 | 0,930 | 0,882 | 0,979 | 0,937 | 0,873 | 1 |
| OAS1, IFIT1, IFI44L, SIGLEC1, ISG15, HERC6, OLAH, FAM20A, RETN, MMP8 | 0,867 | 0,856 | 0,877 | 0,922 | 0,864 | 0,979 | 0,952 | 0,896 | 1 |
| RSAD2, OAS1, IFIT1, IFI44L, SIGLEC1, ISG15, HERC6, IL1R2, OLAH, FAM20A | 0,867 | 0,857 | 0,876 | 0,919 | 0,861 | 0,976 | 0,953 | 0,898 | 1 |
| RSAD2, IFI27, OAS1, IFI44L, SIGLEC1, HERC6, SLC1A2, FAM20A, RETN, MMP8 | 0,867 | 0,856 | 0,878 | 0,930 | 0,877 | 0,984 | 0,943 | 0,871 | 1 |
| RSAD2, IFI27, OAS1, IFI44L, ISG15, SLC1A2, IL1R2, OLAH, FAM20A, RETN | 0,867 | 0,856 | 0,877 | 0,931 | 0,880 | 0,982 | 0,920 | 0,837 | 1 |
| IFI27, OAS1, IFI44L, SIGLEC1, ISG15, HERC6, SLC1A2, OLAH, RETN, MMP8 | 0,867 | 0,856 | 0,877 | 0,932 | 0,883 | 0,981 | 0,946 | 0,884 | 1 |
| RSAD2, IFI27, OAS1, IFIT1, SIGLEC1, ISG15, HERC6, SLC1A2, OLAH, RETN | 0,867 | 0,857 | 0,876 | 0,932 | 0,883 | 0,981 | 0,946 | 0,885 | 1 |
| RSAD2, IFI27, OAS1, SIGLEC1, ISG15, HERC6, SLC1A2, OLAH, RETN, MMP8 | 0,866 | 0,856 | 0,877 | 0,931 | 0,881 | 0,980 | 0,948 | 0,888 | 1 |
| RSAD2, IFI27, OAS1, IFIT1, SIGLEC1, ISG15, HERC6, SLC1A2, IL1R2, OLAH | 0,866 | 0,857 | 0,876 | 0,929 | 0,879 | 0,978 | 0,942 | 0,884 | 1 |
| RSAD2, IFI27, OAS1, IFIT1, IFI44L, SIGLEC1, ISG15, OLAH, RETN, MMP8 | 0,866 | 0,857 | 0,876 | 0,919 | 0,861 | 0,978 | 0,953 | 0,898 | 1 |
| RSAD2, IFI27, IFI44L, SIGLEC1, ISG15, HERC6, SLC1A2, IL1R2, FAM20A, RETN | 0,866 | 0,856 | 0,877 | 0,933 | 0,887 | 0,980 | 0,947 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, HERC6, SLC1A2, OLAH, RETN | 0,866 | 0,857 | 0,876 | 0,929 | 0,878 | 0,980 | 0,943 | 0,881 | 1 |
| OAS1, IFIT1, SIGLEC1, ISG15, HERC6, SLC1A2, IL1R2, OLAH, FAM20A, MMP8 | 0,866 | 0,856 | 0,877 | 0,929 | 0,883 | 0,975 | 0,962 | 0,902 | 1 |
| IFI27, OAS1, IFIT1, ISG15, HERC6, SLC1A2, IL1R2, OLAH, RETN, MMP8 | 0,866 | 0,856 | 0,877 | 0,930 | 0,880 | 0,979 | 0,940 | 0,876 | 1 |
| IFIT1, IFI44L, SIGLEC1, ISG15, HERC6, SLC1A2, OLAH, FAM20A, RETN, MMP8 | 0,866 | 0,856 | 0,877 | 0,931 | 0,883 | 0,979 | 0,946 | 0,878 | 1 |
| IFI27, IFIT1, SIGLEC1, ISG15, HERC6, SLC1A2, IL1R2, OLAH, RETN, MMP8 | 0,866 | 0,856 | 0,876 | 0,931 | 0,881 | 0,980 | 0,953 | 0,902 | 1 |
| RSAD2, IFI27, OAS1, IFIT1, IFI44L, SIGLEC1, ISG15, HERC6, SLC1A2, FAM20A | 0,866 | 0,857 | 0,876 | 0,930 | 0,881 | 0,979 | 0,936 | 0,861 | 1 |
| RSAD2, IFIT1, SIGLEC1, ISG15, HERC6, SLC1A2, IL1R2, OLAH, FAM20A, MMP8 | 0,866 | 0,857 | 0,876 | 0,928 | 0,881 | 0,975 | 0,961 | 0,897 | 1 |
| RSAD2, OAS1, IFIT1, IFI44L, SIGLEC1, HERC6, OLAH, FAM20A, RETN, MMP8 | 0,866 | 0,856 | 0,876 | 0,921 | 0,863 | 0,979 | 0,949 | 0,890 | 1 |
| RSAD2, IFIT1, IFI44L, SIGLEC1, HERC6, IL1R2, OLAH, FAM20A, RETN, MMP8 | 0,866 | 0,856 | 0,877 | 0,926 | 0,871 | 0,980 | 0,962 | 0,907 | 1 |
| IFI27, OAS1, IFI44L, ISG15, HERC6, SLC1A2, IL1R2, FAM20A, RETN, MMP8 | 0,866 | 0,855 | 0,877 | 0,933 | 0,885 | 0,982 | 0,923 | 0,834 | 1 |
| RSAD2, IFI27, IFIT1, IFI44L, SIGLEC1, HERC6, SLC1A2, OLAH, RETN, MMP8 | 0,866 | 0,857 | 0,875 | 0,931 | 0,881 | 0,981 | 0,952 | 0,901 | 1 |
| RSAD2, IFI27, OAS1, IFI44L, SIGLEC1, ISG15, IL1R2, OLAH, RETN, MMP8 | 0,866 | 0,857 | 0,876 | 0,925 | 0,871 | 0,979 | 0,954 | 0,901 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFI44L, SIGLEC1, ISG15, HERC6, SLC1A2, IL1R2, OLAH, RETN | 0,866 | 0,856 | 0,876 | 0,930 | 0,880 | 0,979 | 0,937 | 0,873 | 1 |
| RSAD2, IFI27, OAS1, IFIT1, IFI44L, HERC6, SLC1A2, IL1R2, FAM20A, MMP8 | 0,866 | 0,856 | 0,877 | 0,931 | 0,885 | 0,977 | 0,925 | 0,838 | 1 |
| RSAD2, IFI27, OAS1, IFIT1, IFI44L, SIGLEC1, ISG15, IL1R2, OLAH, MMP8 | 0,866 | 0,857 | 0,875 | 0,926 | 0,876 | 0,976 | 0,943 | 0,891 | 0,996 |
| IFI27, OAS1, IFIT1, ISG15, HERC6, SLC1A2, IL1R2, FAM20A, RETN, MMP8 | 0,866 | 0,856 | 0,876 | 0,933 | 0,884 | 0,981 | 0,925 | 0,842 | 1 |
| OAS1, IFIT1, SIGLEC1, HERC6, SLC1A2, IL1R2, OLAH, FAM20A, RETN, MMP8 | 0,866 | 0,856 | 0,876 | 0,930 | 0,883 | 0,977 | 0,958 | 0,894 | 1 |
| RSAD2, OAS1, IFIT1, SIGLEC1, ISG15, HERC6, OLAH, FAM20A, RETN, MMP8 | 0,866 | 0,856 | 0,876 | 0,921 | 0,864 | 0,979 | 0,953 | 0,898 | 1 |
| OAS1, IFIT1, IFI44L, SIGLEC1, HERC6, SLC1A2, OLAH, FAM20A, RETN, MMP8 | 0,866 | 0,855 | 0,877 | 0,930 | 0,881 | 0,979 | 0,940 | 0,873 | 1 |
| RSAD2, IFI27, OAS1, IFIT1, IFI44L, ISG15, IL1R2, FAM20A, RETN, MMP8 | 0,866 | 0,855 | 0,877 | 0,929 | 0,876 | 0,982 | 0,933 | 0,854 | 1 |
| RSAD2, IFI27, OAS1, IFIT1, HERC6, SLC1A2, IL1R2, OLAH, RETN, MMP8 | 0,866 | 0,856 | 0,876 | 0,928 | 0,877 | 0,979 | 0,940 | 0,875 | 1 |
| RSAD2, IFIT1, IFI44L, SIGLEC1, ISG15, HERC6, SLC1A2, OLAH, FAM20A, MMP8 | 0,866 | 0,855 | 0,877 | 0,929 | 0,880 | 0,978 | 0,937 | 0,866 | 1 |
| RSAD2, IFI27, OAS1, IFIT1, SIGLEC1, ISG15, IL1R2, OLAH, RETN, MMP8 | 0,866 | 0,856 | 0,876 | 0,923 | 0,868 | 0,978 | 0,959 | 0,910 | 1 |
| RSAD2, IFI27, OAS1, IFIT1, IFI44L, SIGLEC1, HERC6, SLC1A2, FAM20A, MMP8 | 0,866 | 0,855 | 0,876 | 0,927 | 0,876 | 0,978 | 0,935 | 0,859 | 1 |
| OAS1, IFIT1, IFI44L, SIGLEC1, ISG15, HERC6, SLC1A2, OLAH, FAM20A, MMP8 | 0,866 | 0,855 | 0,877 | 0,929 | 0,880 | 0,978 | 0,938 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, HERC6, SLC1A2, IL1R2, FAM20A, RETN, MMP8 | 0,866 | 0,856 | 0,876 | 0,929 | 0,882 | 0,975 | 0,958 | 0,892 | 1 |
| RSAD2, IFI27, SIGLEC1, ISG15, HERC6, SLC1A2, IL1R2, OLAH, RETN, MMP8 | 0,866 | 0,856 | 0,876 | 0,931 | 0,884 | 0,979 | 0,950 | 0,897 | 1 |
| IFI27, IFI44L, SIGLEC1, ISG15, HERC6, SLC1A2, IL1R2, OLAH, RETN, MMP8 | 0,866 | 0,856 | 0,875 | 0,930 | 0,882 | 0,979 | 0,949 | 0,896 | 1 |
| RSAD2, IFI27, OAS1, IFIT1, IFI44L, SIGLEC1, ISG15, HERC6, SLC1A2, RETN | 0,866 | 0,857 | 0,875 | 0,924 | 0,870 | 0,978 | 0,940 | 0,876 | 1 |
| RSAD2, OAS1, SIGLEC1, ISG15, HERC6, IL1R2, OLAH, FAM20A, RETN, MMP8 | 0,866 | 0,856 | 0,876 | 0,918 | 0,861 | 0,975 | 0,961 | 0,903 | 1 |
| RSAD2, IFI27, OAS1, IFIT1, IFI44L, ISG15, HERC6, IL1R2, OLAH, MMP8 | 0,866 | 0,856 | 0,875 | 0,924 | 0,873 | 0,974 | 0,939 | 0,883 | 0,995 |
| RSAD2, OAS1, IFI44L, SIGLEC1, ISG15, HERC6, IL1R2, OLAH, FAM20A, MMP8 | 0,866 | 0,856 | 0,876 | 0,919 | 0,863 | 0,976 | 0,962 | 0,905 | 1 |
| RSAD2, IFI27, OAS1, IFIT1, IFI44L, SIGLEC1, ISG15, HERC6, SLC1A2, OLAH | 0,866 | 0,856 | 0,875 | 0,931 | 0,882 | 0,980 | 0,943 | 0,886 | 1 |
| RSAD2, IFI27, OAS1, IFIT1, IFI44L, SIGLEC1, ISG15, IL1R2, OLAH, RETN | 0,866 | 0,857 | 0,875 | 0,922 | 0,867 | 0,977 | 0,949 | 0,893 | 1 |
| RSAD2, IFI27, OAS1, IFI44L, SIGLEC1, ISG15, HERC6, SLC1A2, FAM20A, RETN | 0,866 | 0,855 | 0,876 | 0,928 | 0,875 | 0,981 | 0,937 | 0,861 | 1 |
| IFI27, IFIT1, IFI44L, SIGLEC1, HERC6, SLC1A2, IL1R2, OLAH, RETN, MMP8 | 0,866 | 0,856 | 0,875 | 0,929 | 0,880 | 0,979 | 0,955 | 0,907 | 1 |
| OAS1, IFIT1, IFI44L, SIGLEC1, ISG15, IL1R2, OLAH, FAM20A, RETN, MMP8 | 0,866 | 0,855 | 0,876 | 0,920 | 0,863 | 0,978 | 0,968 | 0,918 | 1 |
| IFI27, OAS1, IFIT1, SIGLEC1, ISG15, HERC6, SLC1A2, IL1R2, OLAH, MMP8 | 0,866 | 0,855 | 0,876 | 0,929 | 0,880 | 0,977 | 0,941 | 0,884 | 0,999 |

(suite)

| Modèle | AUC modèle | IC 95% | | AUC Train | IC 95% Train | | AUC Test | IC 95% Test | |
|---|---|---|---|---|---|---|---|---|---|
| IFIT1, IFI44L, SIGLEC1, ISG15, HERC6, SLC1A2, IL1R2, FAM20A, RETN, MMP8 | 0,866 | 0,856 | 0,875 | 0,929 | 0,883 | 0,976 | 0,958 | 0,890 | 1 |
| IFI27, OAS1, IFIT1, IFI44L, SIGLEC1, HERC6, SLC1A2, IL1R2, RETN, MMP8 | 0,866 | 0,856 | 0,875 | 0,927 | 0,880 | 0,974 | 0,954 | 0,905 | 1 |
| RSAD2, IFI27, IFIT1, IFI44L, HERC6, SLC1A2, IL1R2, FAM20A, RETN, MMP8 | 0,865 | 0,856 | 0,875 | 0,933 | 0,885 | 0,981 | 0,929 | 0,847 | 1 |
| IFI27, OAS1, IFIT1, IFI44L, SIGLEC1, ISG15, HERC6, SLC1A2, OLAH, MMP8 | 0,865 | 0,855 | 0,876 | 0,930 | 0,880 | 0,979 | 0,941 | 0,884 | 0,999 |
| RSAD2, IFI27, OAS1, IFIT1, HERC6, SLC1A2, IL1R2, FAM20A, RETN, MMP8 | 0,865 | 0,855 | 0,876 | 0,933 | 0,884 | 0,981 | 0,926 | 0,843 | 1 |
| RSAD2, OAS1, IFIT1, SIGLEC1, ISG15, IL1R2, OLAH, FAM20A, RETN, MMP8 | 0,865 | 0,856 | 0,875 | 0,920 | 0,863 | 0,978 | 0,968 | 0,918 | 1 |
| RSAD2, IFI27, OAS1, ISG15, HERC6, SLC1A2, IL1R2, OLAH, RETN, MMP8 | 0,865 | 0,855 | 0,875 | 0,930 | 0,879 | 0,981 | 0,940 | 0,875 | 1 |
| RSAD2, IFI27, IFIT1, IFI44L, SIGLEC1, HERC6, SLC1A2, IL1R2, OLAH, RETN | 0,865 | 0,856 | 0,875 | 0,928 | 0,879 | 0,977 | 0,943 | 0,888 | 0,999 |
| IFIT1, SIGLEC1, ISG15, HERC6, SLC1A2, IL1R2, OLAH, FAM20A, RETN, MMP8 | 0,865 | 0,855 | 0,875 | 0,929 | 0,881 | 0,976 | 0,959 | 0,893 | 1 |
| IFIT1, IFI44L, SIGLEC1, HERC6, SLC1A2, IL1R2, OLAH, FAM20A, RETN, MMP8 | 0,865 | 0,855 | 0,876 | 0,931 | 0,884 | 0,978 | 0,960 | 0,899 | 1 |
| RSAD2, IFI27, OAS1, IFIT1, IFI44L, SLC1A2, IL1R2, OLAH, FAM20A, RETN | 0,865 | 0,855 | 0,875 | 0,929 | 0,875 | 0,983 | 0,921 | 0,839 | 1 |
| IFI27, OAS1, IFIT1, SIGLEC1, ISG15, SLC1A2, IL1R2, OLAH, RETN, MMP8 | 0,865 | 0,855 | 0,875 | 0,930 | 0,881 | 0,980 | 0,953 | 0,898 | 1 |
| RSAD2, IFI27, OAS1, IFIT1, IFI44L, ISG15, SLC1A2, OLAH, FAM20A, RETN | 0,865 | 0,855 | 0,876 | 0,928 | 0,874 | 0,983 | 0,920 | 0,838 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, SLC1A2, IL1R2, OLAH, RETN | 0,865 | 0,856 | 0,874 | 0,928 | 0,878 | 0,978 | 0,943 | 0,887 | 1 |
| RSAD2, IFI27, OAS1, IFIT1, SIGLEC1, HERC6, SLC1A2, IL1R2, OLAH, MMP8 | 0,865 | 0,855 | 0,875 | 0,929 | 0,880 | 0,977 | 0,945 | 0,888 | 1 |
| IFI27, OAS1, IFIT1, SIGLEC1, ISG15, HERC6, SLC1A2, IL1R2, RETN, MMP8 | 0,865 | 0,856 | 0,875 | 0,928 | 0,881 | 0,975 | 0,955 | 0,903 | 1 |
| RSAD2, IFI27, OAS1, IFIT1, IFI44L, SIGLEC1, ISG15, SLC1A2, FAM20A, RETN | 0,865 | 0,855 | 0,875 | 0,926 | 0,872 | 0,981 | 0,938 | 0,864 | 1 |
| RSAD2, IFI27, OAS1, IFI44L, SIGLEC1, ISG15, HERC6, SLC1A2, IL1R2, OLAH | 0,865 | 0,856 | 0,875 | 0,930 | 0,881 | 0,979 | 0,945 | 0,890 | 1,000 |
| RSAD2, IFI27, OAS1, IFIT1, IFI44L, HERC6, SLC1A2, OLAH, RETN, MMP8 | 0,865 | 0,855 | 0,875 | 0,928 | 0,877 | 0,980 | 0,940 | 0,875 | 1 |
| RSAD2, OAS1, IFIT1, IFI44L, SIGLEC1, ISG15, SLC1A2, OLAH, FAM20A, MMP8 | 0,865 | 0,855 | 0,875 | 0,926 | 0,874 | 0,977 | 0,941 | 0,877 | 1 |
| RSAD2, IFI27, IFIT1, SIGLEC1, ISG15, HERC6, SLC1A2, OLAH, RETN, MMP8 | 0,865 | 0,855 | 0,875 | 0,931 | 0,882 | 0,980 | 0,949 | 0,895 | 1 |
| RSAD2, IFI27, OAS1, IFIT1, IFI44L, SIGLEC1, HERC6, SLC1A2, OLAH, MMP8 | 0,865 | 0,855 | 0,875 | 0,929 | 0,878 | 0,979 | 0,945 | 0,889 | 1 |
| RSAD2, OAS1, IFIT1, SIGLEC1, ISG15, HERC6, SLC1A2, OLAH, FAM20A, MMP8 | 0,865 | 0,854 | 0,876 | 0,926 | 0,877 | 0,975 | 0,943 | 0,874 | 1 |
| IFI27, OAS1, IFIT1, IFI44L, HERC6, SLC1A2, IL1R2, OLAH, RETN, MMP8 | 0,865 | 0,855 | 0,875 | 0,929 | 0,878 | 0,979 | 0,939 | 0,874 | 1 |
| RSAD2, IFI27, IFIT1, IFI44L, HERC6, SLC1A2, IL1R2, OLAH, RETN, MMP8 | 0,865 | 0,855 | 0,875 | 0,928 | 0,877 | 0,979 | 0,941 | 0,881 | 1 |
| OAS1, IFIT1, SIGLEC1, ISG15, HERC6, SLC1A2, IL1R2, FAM20A, RETN, MMP8 | 0,865 | 0,855 | 0,875 | 0,929 | 0,882 | 0,975 | 0,957 | 0,889 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2,IFI27,OAS1,IFIT1, SIGLEC1, ISG15, HERC6, SLC1A2, OLAH, MMP8 | 0,865 | 0,855 | 0,875 | 0,930 | 0,880 | 0,980 | 0,940 | 0,881 | 0,999 |
| RSAD2, IFI27, IFIT1, IFI44L, SIGLEC1, ISG15, HERC6, IL1R2, RETN, MMP8 | 0,865 | 0,856 | 0,874 | 0,923 | 0,870 | 0,976 | 0,953 | 0,908 | 0,999 |
| OAS1, IFIT1, SIGLEC1, ISG15, HERC6, SLC1A2, OLAH, FAM20A, RETN, MMP8 | 0,865 | 0,855 | 0,875 | 0,926 | 0,876 | 0,976 | 0,947 | 0,881 | 1 |
| RSAD2, IFIT1, IFI44L, SIGLEC1, ISG15, SLC1A2, IL1R2, OLAH, FAM20A, MMP8 | 0,865 | 0,855 | 0,875 | 0,927 | 0,879 | 0,975 | 0,960 | 0,900 | 1 |
| RSAD2, IFI27, IFIT1, IFI44L, SIGLEC1, ISG15, SLC1A2, FAM20A, RETN, MMP8 | 0,865 | 0,854 | 0,876 | 0,927 | 0,872 | 0,983 | 0,942 | 0,869 | 1 |
| RSAD2, IFIT1, IFI44L, SIGLEC1, ISG15, HERC6, OLAH, FAM20A, RETN, MMP8 | 0,865 | 0,855 | 0,875 | 0,922 | 0,865 | 0,979 | 0,953 | 0,896 | 1 |
| RSAD2, OAS1, IFIT1, IFI44L, SIGLEC1, SLC1A2, IL1R2, OLAH, FAM20A, MMP8 | 0,865 | 0,855 | 0,875 | 0,926 | 0,878 | 0,975 | 0,959 | 0,904 | 1 |
| RSAD2, OAS1, IFIT1, SIGLEC1, ISG15, SLC1A2, IL1R2, OLAH, FAM20A, MMP8 | 0,865 | 0,855 | 0,874 | 0,926 | 0,877 | 0,974 | 0,963 | 0,904 | 1 |
| RSAD2,IFI27,OAS1,IFIT1, SIGLEC1, ISG15, HERC6, SLC1A2, FAM20A, MMP8 | 0,865 | 0,855 | 0,875 | 0,929 | 0,879 | 0,978 | 0,937 | 0,862 | 1 |
| RSAD2,IFI27,OAS1,IFIT1, SIGLEC1, ISG15, SLC1A2, FAM20A, RETN, MMP8 | 0,865 | 0,854 | 0,875 | 0,926 | 0,870 | 0,982 | 0,943 | 0,868 | 1 |
| RSAD2, IFI27, IFIT1, ISG15, HERC6, SLC1A2, IL1R2, FAM20A, RETN, MMP8 | 0,865 | 0,855 | 0,874 | 0,933 | 0,885 | 0,982 | 0,932 | 0,849 | 1 |
| IFI27, OAS1, IFIT1, IFI44L, SIGLEC1, ISG15, HERC6, SLC1A2, RETN, MMP8 | 0,865 | 0,856 | 0,874 | 0,929 | 0,879 | 0,979 | 0,943 | 0,886 | 1 |
| RSAD2, IFI27, IFI44L, SIGLEC1, ISG15, HERC6, SLC1A2, FAM20A, RETN, MMP8 | 0,865 | 0,854 | 0,876 | 0,930 | 0,877 | 0,983 | 0,946 | 0,872 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, ISG15, HERC6, SLC1A2, IL1R2, FAM20A, RETN, MMP8 | 0,865 | 0,855 | 0,875 | 0,934 | 0,887 | 0,982 | 0,924 | 0,836 | 1 |
| RSAD2, IFI27, OAS1, IFI44L, SIGLEC1, ISG15, HERC6, SLC1A2, OLAH, MMP8 | 0,865 | 0,855 | 0,874 | 0,931 | 0,881 | 0,981 | 0,942 | 0,885 | 1 |
| IFI27, IFIT1, IFI44L, SIGLEC1, ISG15, HERC6, SLC1A2, OLAH, RETN, MMP8 | 0,865 | 0,855 | 0,875 | 0,930 | 0,880 | 0,980 | 0,951 | 0,899 | 1 |
| RSAD2, OAS1, IFIT1, IFI44L, SIGLEC1, IL1R2, OLAH, FAM20A, RETN, MMP8 | 0,865 | 0,854 | 0,875 | 0,922 | 0,865 | 0,978 | 0,967 | 0,916 | 1 |
| RSAD2, IFI27, OAS1, IFIT1, IFI44L, SIGLEC1, SLC1A2, FAM20A, RETN, MMP8 | 0,865 | 0,854 | 0,875 | 0,927 | 0,872 | 0,982 | 0,943 | 0,871 | 1 |
| RSAD2, OAS1, IFIT1, IFI44L, SIGLEC1, ISG15, HERC6, OLAH, FAM20A, MMP8 | 0,865 | 0,854 | 0,876 | 0,917 | 0,859 | 0,975 | 0,949 | 0,891 | 1 |
| RSAD2, IFI27, OAS1, SIGLEC1, ISG15, HERC6, SLC1A2, IL1R2, OLAH, MMP8 | 0,865 | 0,854 | 0,875 | 0,929 | 0,880 | 0,978 | 0,942 | 0,884 | 1 |
| IFI27, OAS1, IFIT1, IFI44L, SIGLEC1, ISG15, HERC6, SLC1A2, IL1R2, OLAH | 0,865 | 0,855 | 0,875 | 0,930 | 0,882 | 0,978 | 0,940 | 0,883 | 0,997 |
| RSAD2, IFI27, IFIT1, IFI44L, SIGLEC1, ISG15, HERC6, SLC1A2, OLAH, RETN | 0,865 | 0,856 | 0,873 | 0,927 | 0,877 | 0,978 | 0,941 | 0,884 | 0,998 |
| OAS1, IFIT1, IFI44L, SIGLEC1, ISG15, HERC6, SLC1A2, IL1R2, FAM20A, MMP8 | 0,865 | 0,854 | 0,875 | 0,928 | 0,883 | 0,974 | 0,961 | 0,899 | 1 |
| RSAD2, IFI27, IFIT1, SIGLEC1, ISG15, HERC6, SLC1A2, IL1R2, OLAH, RETN | 0,865 | 0,855 | 0,874 | 0,929 | 0,878 | 0,979 | 0,940 | 0,882 | 0,998 |
| RSAD2, IFI27, IFIT1, ISG15, HERC6, SLC1A2, IL1R2, OLAH, RETN, MMP8 | 0,865 | 0,855 | 0,874 | 0,930 | 0,880 | 0,979 | 0,939 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, SIGLEC1, ISG15, HERC6, SLC1A2, IL1R2, OLAH, FAM20A, MMP8 | 0,865 | 0,854 | 0,875 | 0,924 | 0,876 | 0,972 | 0,961 | 0,901 | 1 |
| RSAD2, IFI27, OAS1, IFI44L, HERC6, SLC1A2, IL1R2, FAM20A, RETN, MMP8 | 0,865 | 0,854 | 0,875 | 0,933 | 0,884 | 0,981 | 0,923 | 0,834 | 1 |
| RSAD2, OAS1, IFIT1, SIGLEC1, HERC6, SLC1A2, OLAH, FAM20A, RETN, MMP8 | 0,865 | 0,854 | 0,875 | 0,927 | 0,877 | 0,977 | 0,948 | 0,881 | 1 |
| IFI27, OAS1, IFIT1, IFI44L, ISG15, HERC6, SLC1A2, IL1R2, FAM20A, RETN | 0,864 | 0,854 | 0,875 | 0,933 | 0,885 | 0,980 | 0,925 | 0,840 | 1 |
| RSAD2, OAS1, IFIT1, IFI44L, SIGLEC1, HERC6, SLC1A2, IL1R2, FAM20A, MMP8 | 0,864 | 0,854 | 0,875 | 0,928 | 0,882 | 0,974 | 0,963 | 0,904 | 1 |
| RSAD2, IFI27, OAS1, IFI44L, SIGLEC1, HERC6, SLC1A2, IL1R2, OLAH, MMP8 | 0,864 | 0,855 | 0,874 | 0,928 | 0,880 | 0,976 | 0,950 | 0,897 | 1 |
| RSAD2, IFI27, OAS1, IFIT1, IFI44L, SIGLEC1, SLC1A2, IL1R2, OLAH, RETN | 0,864 | 0,855 | 0,873 | 0,925 | 0,873 | 0,977 | 0,950 | 0,887 | 1 |
| RSAD2, IFI27, OAS1, IFIT1, IFI44L, SIGLEC1, HERC6, SLC1A2, IL1R2, RETN | 0,864 | 0,856 | 0,873 | 0,928 | 0,879 | 0,976 | 0,945 | 0,882 | 1 |
| RSAD2, IFI27, OAS1, IFIT1, IFI44L, ISG15, HERC6, IL1R2, RETN, MMP8 | 0,864 | 0,855 | 0,874 | 0,924 | 0,870 | 0,978 | 0,940 | 0,879 | 1 |
| RSAD2, IFI27, OAS1, IFIT1, SIGLEC1, SLC1A2, IL1R2, OLAH, RETN, MMP8 | 0,864 | 0,855 | 0,874 | 0,929 | 0,879 | 0,979 | 0,955 | 0,900 | 1 |
| RSAD2, IFI27, OAS1, IFIT1, IFI44L, SIGLEC1, HERC6, SLC1A2, IL1R2, OLAH | 0,864 | 0,855 | 0,874 | 0,929 | 0,880 | 0,977 | 0,950 | 0,897 | 1 |
| RSAD2, IFI27, OAS1, IFI44L, SIGLEC1, ISG15, SLC1A2, IL1R2, OLAH, RETN | 0,864 | 0,855 | 0,873 | 0,930 | 0,880 | 0,980 | 0,947 | 0,883 | 1 |
| RSAD2, IFI27, IFI44L, SIGLEC1, ISG15, HERC6, SLC1A2, OLAH, RETN, MMP8 | 0,864 | 0,854 | 0,874 | 0,931 | 0,881 | 0,980 | 0,951 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, HERC6, SLC1A2, IL1R2, OLAH, RETN | 0,864 | 0,854 | 0,874 | 0,928 | 0,876 | 0,979 | 0,932 | 0,860 | 1 |
| RSAD2, IFI27, IFIT1, IFI44L, SIGLEC1, ISG15, HERC6, SLC1A2, FAM20A, MMP8 | 0,864 | 0,854 | 0,874 | 0,927 | 0,877 | 0,978 | 0,937 | 0,860 | 1 |
| IFI27, OAS1, IFIT1, IFI44L, ISG15, HERC6, SLC1A2, OLAH, RETN, MMP8 | 0,864 | 0,854 | 0,875 | 0,930 | 0,879 | 0,980 | 0,939 | 0,874 | 1 |
| IFI27, OAS1, IFIT1, IFI44L, SIGLEC1, ISG15, HERC6, SLC1A2, IL1R2, RETN | 0,864 | 0,855 | 0,873 | 0,928 | 0,881 | 0,975 | 0,941 | 0,883 | 0,999 |
| OAS1, IFIT1, IFI44L, SIGLEC1, ISG15, SLC1A2, IL1R2, OLAH, FAM20A, MMP8 | 0,864 | 0,854 | 0,874 | 0,928 | 0,880 | 0,975 | 0,961 | 0,903 | 1 |
| IFI27, OAS1, IFIT1, IFI44L, SIGLEC1, HERC6, SLC1A2, IL1R2, OLAH, MMP8 | 0,864 | 0,854 | 0,874 | 0,927 | 0,878 | 0,975 | 0,946 | 0,891 | 1 |
| RSAD2, IFIT1, SIGLEC1, HERC6, SLC1A2, IL1R2, OLAH, FAM20A, RETN, MMP8 | 0,864 | 0,854 | 0,874 | 0,929 | 0,881 | 0,976 | 0,955 | 0,889 | 1 |
| RSAD2, IFI27, OAS1, IFIT1, SIGLEC1, ISG15, HERC6, SLC1A2, RETN, MMP8 | 0,864 | 0,855 | 0,873 | 0,924 | 0,870 | 0,978 | 0,940 | 0,878 | 1 |
| RSAD2, IFI27, OAS1, IFIT1, SIGLEC1, HERC6, SLC1A2, IL1R2, RETN, MMP8 | 0,864 | 0,855 | 0,874 | 0,929 | 0,882 | 0,977 | 0,957 | 0,903 | 1 |
| OAS1, IFIT1, IFI44L, SIGLEC1, ISG15, HERC6, IL1R2, OLAH, FAM20A, RETN | 0,864 | 0,854 | 0,874 | 0,918 | 0,860 | 0,976 | 0,952 | 0,897 | 1 |
| IFI27, OAS1, IFI44L, SIGLEC1, ISG15, HERC6, SLC1A2, IL1R2, OLAH, MMP8 | 0,864 | 0,854 | 0,874 | 0,930 | 0,882 | 0,978 | 0,941 | 0,886 | 0,997 |
| IFIT1, IFI44L, SIGLEC1, ISG15, SLC1A2, IL1R2, OLAH, FAM20A, RETN, MMP8 | 0,864 | 0,855 | 0,874 | 0,926 | 0,878 | 0,975 | 0,960 | 0,900 | 1 |
| RSAD2, OAS1, IFIT1, SIGLEC1, ISG15, HERC6, IL1R2, OLAH, FAM20A, RETN | 0,864 | 0,855 | 0,874 | 0,919 | 0,861 | 0,977 | 0,954 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, ISG15, HERC6, SLC1A2, IL1R2, FAM20A, RETN, MMP8 | 0,864 | 0,854 | 0,874 | 0,931 | 0,882 | 0,980 | 0,930 | 0,847 | 1 |
| IFI27, OAS1, IFIT1, IFI44L, ISG15, HERC6, SLC1A2, IL1R2, FAM20A, MMP8 | 0,864 | 0,853 | 0,875 | 0,931 | 0,885 | 0,978 | 0,926 | 0,840 | 1 |
| RSAD2, OAS1, IFIT1, SIGLEC1, ISG15, HERC6, SLC1A2, IL1R2, FAM20A, MMP8 | 0,864 | 0,854 | 0,874 | 0,927 | 0,881 | 0,974 | 0,960 | 0,896 | 1 |
| RSAD2, OAS1, IFI44L, SIGLEC1, HERC6, SLC1A2, IL1R2, OLAH, FAM20A, MMP8 | 0,864 | 0,853 | 0,875 | 0,923 | 0,875 | 0,972 | 0,959 | 0,897 | 1 |
| RSAD2, IFI27, OAS1, IFI44L, HERC6, SLC1A2, IL1R2, OLAH, RETN, MMP8 | 0,864 | 0,854 | 0,874 | 0,929 | 0,879 | 0,980 | 0,941 | 0,877 | 1 |
| RSAD2, IFI27, OAS1, IFIT1, IFI44L, SIGLEC1, ISG15, SLC1A2, IL1R2, OLAH | 0,864 | 0,855 | 0,873 | 0,929 | 0,878 | 0,979 | 0,947 | 0,890 | 1 |
| RSAD2, IFI27, OAS1, IFIT1, SIGLEC1, ISG15, HERC6, SLC1A2, IL1R2, RETN | 0,864 | 0,855 | 0,873 | 0,926 | 0,877 | 0,975 | 0,947 | 0,886 | 1 |
| RSAD2, IFI27, OAS1, IFIT1, ISG15, HERC6, SLC1A2, OLAH, RETN, MMP8 | 0,864 | 0,853 | 0,875 | 0,928 | 0,876 | 0,980 | 0,940 | 0,875 | 1 |
| RSAD2, IFI27, OAS1, IFIT1, SIGLEC1, ISG15, SLC1A2, IL1R2, OLAH, MMP8 | 0,864 | 0,855 | 0,873 | 0,929 | 0,880 | 0,979 | 0,945 | 0,887 | 1 |
| RSAD2, IFI27, OAS1, IFIT1, IFI44L, ISG15, IL1R2, OLAH, RETN, MMP8 | 0,864 | 0,854 | 0,874 | 0,919 | 0,861 | 0,976 | 0,949 | 0,895 | 1 |
| RSAD2, IFI27, OAS1, IFI44L, ISG15, HERC6, SLC1A2, IL1R2, OLAH, RETN | 0,864 | 0,854 | 0,874 | 0,928 | 0,877 | 0,979 | 0,932 | 0,860 | 1 |
| RSAD2, IFI27, IFIT1, SIGLEC1, ISG15, HERC6, SLC1A2, IL1R2, OLAH, MMP8 | 0,864 | 0,854 | 0,873 | 0,929 | 0,880 | 0,978 | 0,941 | 0,885 | 0,997 |
| RSAD2, IFI27, OAS1, IFI44L, SIGLEC1, ISG15, HERC6, SLC1A2, IL1R2, RETN | 0,864 | 0,855 | 0,872 | 0,927 | 0,879 | 0,976 | 0,946 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, SLC1A2, IL1R2, OLAH, RETN, MMP8 | 0,864 | 0,854 | 0,874 | 0,927 | 0,876 | 0,978 | 0,952 | 0,896 | 1 |
| RSAD2, IFI27, OAS1, IFI44L, ISG15, HERC6, SLC1A2, IL1R2, FAM20A, MMP8 | 0,864 | 0,853 | 0,875 | 0,930 | 0,884 | 0,976 | 0,923 | 0,834 | 1 |
| RSAD2, IFI27, OAS1, IFIT1, IFI44L, SIGLEC1, ISG15, SLC1A2, OLAH, MMP8 | 0,864 | 0,855 | 0,873 | 0,930 | 0,880 | 0,981 | 0,948 | 0,893 | 1 |
| RSAD2, IFI27, OAS1, IFI44L, ISG15, HERC6, SLC1A2, OLAH, RETN, MMP8 | 0,864 | 0,854 | 0,874 | 0,930 | 0,880 | 0,981 | 0,940 | 0,875 | 1 |
| RSAD2, OAS1, IFIT1, IFI44L, SIGLEC1, ISG15, HERC6, FAM20A, RETN, MMP8 | 0,864 | 0,854 | 0,874 | 0,921 | 0,863 | 0,979 | 0,946 | 0,881 | 1 |
| OAS1, IFI44L, SIGLEC1, ISG15, HERC6, IL1R2, OLAH, FAM20A, RETN, MMP8 | 0,864 | 0,854 | 0,874 | 0,918 | 0,861 | 0,975 | 0,961 | 0,903 | 1 |
| RSAD2, IFI27, OAS1, IFIT1, IFI44L, HERC6, SLC1A2, IL1R2, OLAH, RETN | 0,864 | 0,854 | 0,874 | 0,927 | 0,875 | 0,978 | 0,932 | 0,860 | 1 |
| RSAD2, IFI27, OAS1, IFIT1, IFI44L, SIGLEC1, SLC1A2, IL1R2, OLAH, MMP8 | 0,864 | 0,855 | 0,873 | 0,929 | 0,880 | 0,977 | 0,951 | 0,896 | 1 |
| OAS1, IFI44L, SIGLEC1, ISG15, HERC6, SLC1A2, IL1R2, OLAH, FAM20A, MMP8 | 0,864 | 0,853 | 0,874 | 0,922 | 0,872 | 0,972 | 0,961 | 0,901 | 1 |
| IFI27, OAS1, IFI44L, ISG15, HERC6, SLC1A2, IL1R2, OLAH, RETN, MMP8 | 0,864 | 0,853 | 0,874 | 0,930 | 0,881 | 0,979 | 0,937 | 0,871 | 1 |
| RSAD2, IFIT1, IFI44L, SIGLEC1, HERC6, SLC1A2, OLAH, FAM20A, RETN, MMP8 | 0,864 | 0,853 | 0,874 | 0,931 | 0,883 | 0,979 | 0,940 | 0,872 | 1 |
| RSAD2, IFI27, OAS1, IFI44L, SIGLEC1, ISG15, SLC1A2, IL1R2, OLAH, MMP8 | 0,864 | 0,854 | 0,873 | 0,930 | 0,881 | 0,979 | 0,949 | 0,895 | 1 |
| RSAD2, IFI27, OAS1, IFIT1, IFI44L, SIGLEC1, HERC6, SLC1A2, RETN, MMP8 | 0,864 | 0,855 | 0,873 | 0,926 | 0,873 | 0,979 | 0,941 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, SIGLEC1, ISG15, HERC6, SLC1A2, OLAH, FAM20A, RETN, MMP8 | 0,864 | 0,854 | 0,873 | 0,927 | 0,878 | 0,977 | 0,946 | 0,878 | 1 |
| RSAD2, IFIT1, IFI44L, SIGLEC1, ISG15, HERC6, SLC1A2, IL1R2, FAM20A, MMP8 | 0,864 | 0,854 | 0,874 | 0,928 | 0,882 | 0,973 | 0,959 | 0,891 | 1 |
| RSAD2, IFI27, IFIT1, SIGLEC1, ISG15, SLC1A2, IL1R2, OLAH, RETN, MMP8 | 0,864 | 0,854 | 0,873 | 0,930 | 0,881 | 0,979 | 0,953 | 0,900 | 1 |
| RSAD2, IFIT1, IFI44L, SIGLEC1, HERC6, SLC1A2, IL1R2, FAM20A, RETN, MMP8 | 0,864 | 0,854 | 0,873 | 0,930 | 0,883 | 0,976 | 0,957 | 0,891 | 1 |
| IFI27, IFIT1, IFI44L, SIGLEC1, ISG15, SLC1A2, IL1R2, OLAH, RETN, MMP8 | 0,864 | 0,854 | 0,873 | 0,930 | 0,882 | 0,979 | 0,953 | 0,902 | 1 |
| OAS1, IFIT1, SIGLEC1, ISG15, SLC1A2, IL1R2, OLAH, FAM20A, RETN, MMP8 | 0,864 | 0,854 | 0,873 | 0,929 | 0,883 | 0,975 | 0,961 | 0,901 | 1 |
| RSAD2, IFI27, IFI44L, SIGLEC1, ISG15, HERC6, SLC1A2, IL1R2, OLAH, MMP8 | 0,863 | 0,854 | 0,873 | 0,929 | 0,880 | 0,978 | 0,945 | 0,891 | 0,998 |
| RSAD2, IFI27, IFI44L, ISG15, HERC6, SLC1A2, IL1R2, OLAH, RETN, MMP8 | 0,863 | 0,854 | 0,873 | 0,928 | 0,878 | 0,979 | 0,941 | 0,881 | 1 |
| OAS1, SIGLEC1, ISG15, HERC6, SLC1A2, IL1R2, OLAH, FAM20A, RETN, MMP8 | 0,863 | 0,853 | 0,874 | 0,924 | 0,876 | 0,973 | 0,959 | 0,897 | 1 |
| RSAD2, OAS1, IFIT1, SIGLEC1, HERC6, SLC1A2, IL1R2, FAM20A, RETN, MMP8 | 0,863 | 0,854 | 0,873 | 0,928 | 0,881 | 0,975 | 0,960 | 0,897 | 1 |
| IFI27, IFIT1, IFI44L, SIGLEC1, ISG15, HERC6, SLC1A2, IL1R2, OLAH, MMP8 | 0,863 | 0,854 | 0,873 | 0,928 | 0,879 | 0,977 | 0,943 | 0,890 | 0,997 |
| RSAD2, OAS1, IFIT1, IFI44L, SIGLEC1, ISG15, HERC6, IL1R2, FAM20A, RETN | 0,863 | 0,854 | 0,873 | 0,920 | 0,864 | 0,977 | 0,954 | 0,900 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, SLC1A2, IL1R2, FAM20A, RETN, MMP8 | 0,863 | 0,853 | 0,874 | 0,931 | 0,882 | 0,979 | 0,927 | 0,844 | 1 |
| RSAD2, OAS1, IFIT1, IFI44L, SIGLEC1, HERC6, IL1R2, OLAH, FAM20A, RETN | 0,863 | 0,853 | 0,873 | 0,918 | 0,860 | 0,976 | 0,953 | 0,898 | 1 |
| RSAD2, IFIT1, IFI44L, SIGLEC1, ISG15, IL1R2, OLAH, FAM20A, RETN, MMP8 | 0,863 | 0,853 | 0,873 | 0,920 | 0,863 | 0,978 | 0,967 | 0,916 | 1 |
| IFI27, IFIT1, IFI44L, SIGLEC1, ISG15, HERC6, SLC1A2, IL1R2, OLAH, RETN | 0,863 | 0,854 | 0,873 | 0,928 | 0,878 | 0,978 | 0,941 | 0,884 | 0,998 |
| IFI27, OAS1, IFIT1, IFI44L, SIGLEC1, ISG15, SLC1A2, OLAH, RETN, MMP8 | 0,863 | 0,853 | 0,874 | 0,929 | 0,877 | 0,980 | 0,950 | 0,892 | 1 |
| RSAD2, IFIT1, SIGLEC1, ISG15, HERC6, SLC1A2, IL1R2, FAM20A, RETN, MMP8 | 0,863 | 0,854 | 0,873 | 0,926 | 0,878 | 0,974 | 0,957 | 0,889 | 1 |
| RSAD2, IFI27, OAS1, IFIT1, ISG15, HERC6, SLC1A2, IL1R2, OLAH, MMP8 | 0,863 | 0,853 | 0,873 | 0,929 | 0,880 | 0,977 | 0,933 | 0,866 | 0,999 |
| RSAD2, IFI27, OAS1, IFIT1, IFI44L, SIGLEC1, SLC1A2, OLAH, RETN, MMP8 | 0,863 | 0,854 | 0,873 | 0,927 | 0,873 | 0,980 | 0,958 | 0,901 | 1 |
| RSAD2, IFI27, OAS1, IFIT1, SIGLEC1, ISG15, SLC1A2, IL1R2, OLAH, RETN | 0,863 | 0,854 | 0,873 | 0,930 | 0,880 | 0,981 | 0,947 | 0,885 | 1 |
| OAS1, IFIT1, IFI44L, SIGLEC1, ISG15, HERC6, SLC1A2, IL1R2, OLAH, FAM20A | 0,863 | 0,853 | 0,873 | 0,927 | 0,879 | 0,975 | 0,935 | 0,866 | 1 |
| IFI27, OAS1, IFI44L, SIGLEC1, ISG15, HERC6, SLC1A2, IL1R2, RETN, MMP8 | 0,863 | 0,854 | 0,872 | 0,927 | 0,880 | 0,974 | 0,955 | 0,907 | 1 |
| IFI27, OAS1, IFIT1, IFI44L, SIGLEC1, ISG15, SLC1A2, IL1R2, OLAH, MMP8 | 0,863 | 0,853 | 0,873 | 0,928 | 0,879 | 0,977 | 0,948 | 0,895 | 1 |
| RSAD2, IFI27, IFIT1, IFI44L, SIGLEC1, SLC1A2, IL1R2, OLAH, RETN, MMP8 | 0,863 | 0,854 | 0,873 | 0,930 | 0,880 | 0,979 | 0,958 | 0,908 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, SLC1A2, IL1R2, OLAH, RETN | 0,863 | 0,853 | 0,872 | 0,929 | 0,878 | 0,979 | 0,948 | 0,890 | 1 |
| RSAD2, OAS1, IFIT1, IFI44L, SIGLEC1, ISG15, HERC6, SLC1A2, OLAH, FAM20A | 0,863 | 0,852 | 0,873 | 0,926 | 0,877 | 0,976 | 0,933 | 0,863 | 1 |
| RSAD2, OAS1, IFI44L, SIGLEC1, HERC6, IL1R2, OLAH, FAM20A, RETN, MMP8 | 0,863 | 0,852 | 0,874 | 0,920 | 0,864 | 0,976 | 0,961 | 0,903 | 1 |
| RSAD2, IFI27, OAS1, SIGLEC1, ISG15, HERC6, SLC1A2, IL1R2, RETN, MMP8 | 0,863 | 0,854 | 0,872 | 0,926 | 0,878 | 0,975 | 0,958 | 0,906 | 1 |
| RSAD2, IFI27, IFIT1, IFI44L, SIGLEC1, HERC6, SLC1A2, IL1R2, OLAH, MMP8 | 0,863 | 0,854 | 0,872 | 0,928 | 0,879 | 0,976 | 0,948 | 0,897 | 0,999 |
| RSAD2, IFI27, OAS1, IFIT1, IFI44L, ISG15, HERC6, SLC1A2, IL1R2, FAM20A | 0,863 | 0,852 | 0,873 | 0,930 | 0,884 | 0,976 | 0,926 | 0,840 | 1 |
| RSAD2, OAS1, SIGLEC1, HERC6, SLC1A2, IL1R2, OLAH, FAM20A, RETN, MMP8 | 0,863 | 0,852 | 0,873 | 0,927 | 0,879 | 0,975 | 0,959 | 0,897 | 1 |
| RSAD2, OAS1, IFI44L, SIGLEC1, ISG15, HERC6, IL1R2, FAM20A, RETN, MMP8 | 0,863 | 0,853 | 0,873 | 0,918 | 0,861 | 0,976 | 0,959 | 0,897 | 1 |
| RSAD2, OAS1, IFIT1, IFI44L, SIGLEC1, HERC6, SLC1A2, IL1R2, OLAH, FAM20A | 0,863 | 0,853 | 0,873 | 0,926 | 0,878 | 0,974 | 0,938 | 0,871 | 1 |
| RSAD2, IFI27, OAS1, IFIT1, IFI44L, SIGLEC1, ISG15, SLC1A2, OLAH, RETN | 0,863 | 0,854 | 0,872 | 0,929 | 0,877 | 0,981 | 0,948 | 0,885 | 1 |
| RSAD2, IFI27, OAS1, IFI44L, SIGLEC1, HERC6, SLC1A2, IL1R2, RETN, MMP8 | 0,863 | 0,853 | 0,872 | 0,929 | 0,881 | 0,977 | 0,950 | 0,892 | 1 |
| RSAD2, IFI27, IFI44L, ISG15, HERC6, SLC1A2, IL1R2, FAM20A, RETN, MMP8 | 0,863 | 0,852 | 0,874 | 0,933 | 0,884 | 0,981 | 0,926 | 0,841 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, ISG15, HERC6, SLC1A2, OLAH, RETN, MMP8 | 0,863 | 0,853 | 0,873 | 0,928 | 0,876 | 0,979 | 0,941 | 0,882 | 1 |
| RSAD2, IFI27, OAS1, IFIT1, ISG15, HERC6, SLC1A2, IL1R2, FAM20A, MMP8 | 0,863 | 0,852 | 0,873 | 0,930 | 0,883 | 0,977 | 0,927 | 0,842 | 1 |
| RSAD2, IFI27, IFIT1, IFI44L, SIGLEC1, ISG15, HERC6, SLC1A2, OLAH, MMP8 | 0,863 | 0,853 | 0,872 | 0,930 | 0,880 | 0,980 | 0,942 | 0,886 | 0,999 |
| RSAD2, OAS1, IFIT1, IFI44L, SIGLEC1, ISG15, IL1R2, FAM20A, RETN, MMP8 | 0,863 | 0,852 | 0,873 | 0,922 | 0,865 | 0,979 | 0,967 | 0,914 | 1 |
| RSAD2, IFI27, IFIT1, IFI44L, SIGLEC1, ISG15, SLC1A2, IL1R2, OLAH, MMP8 | 0,863 | 0,854 | 0,872 | 0,928 | 0,879 | 0,978 | 0,943 | 0,890 | 0,997 |
| RSAD2, SIGLEC1, ISG15, HERC6, SLC1A2, IL1R2, OLAH, FAM20A, RETN, MMP8 | 0,863 | 0,853 | 0,872 | 0,927 | 0,879 | 0,974 | 0,958 | 0,893 | 1 |
| RSAD2, IFI27, IFIT1, IFI44L, SIGLEC1, ISG15, HERC6, SLC1A2, ILIR2, OLAH | 0,863 | 0,853 | 0,872 | 0,929 | 0,880 | 0,978 | 0,942 | 0,888 | 0,997 |
| RSAD2, IFI27, OAS1, IFIT1, IFI44L, ISG15, HERC6, SLC1A2, OLAH, RETN | 0,863 | 0,853 | 0,873 | 0,928 | 0,877 | 0,979 | 0,933 | 0,861 | 1 |
| RSAD2, OAS1, IFIT1, SIGLEC1, ISG15, HERC6, SLC1A2, IL1R2, OLAH, FAM20A | 0,863 | 0,853 | 0,873 | 0,926 | 0,877 | 0,974 | 0,948 | 0,884 | 1 |
| RSAD2, IFI27, OAS1, IFIT1, ISG15, HERC6, SLC1A2, IL1R2, FAM20A, RETN | 0,863 | 0,853 | 0,872 | 0,932 | 0,884 | 0,980 | 0,925 | 0,838 | 1 |
| OAS1, IFIT1, IFI44L, SIGLEC1, ISG15, HERC6, SLC1A2, OLAH, FAM20A, RETN | 0,863 | 0,853 | 0,873 | 0,926 | 0,876 | 0,975 | 0,929 | 0,859 | 1 |
| OAS1, IFIT1, IFI44L, SIGLEC1, ISG15, SLC1A2, OLAH, FAM20A, RETN, MMP8 | 0,863 | 0,852 | 0,873 | 0,922 | 0,869 | 0,975 | 0,949 | 0,888 | 1 |
| IFI27, OAS1, IFIT1, IFI44L, ISG15, HERC6, SLC1A2, FAM20A, RETN, MMP8 | 0,862 | 0,852 | 0,873 | 0,923 | 0,867 | 0,979 | 0,932 | 0,850 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, IFI44L, ISG15, HERC6, SLC1A2, IL1R2, OLAH, RETN, MMP8 | 0,862 | 0,853 | 0,872 | 0,927 | 0,875 | 0,978 | 0,936 | 0,873 | 0,999 |
| RSAD2, IFIT1, IFI44L, SIGLEC1, ISG15, HERC6, SLC1A2, IL1R2, OLAH, FAM20A | 0,862 | 0,852 | 0,872 | 0,925 | 0,876 | 0,975 | 0,937 | 0,867 | 1 |
| RSAD2, IFI27, OAS1, IFI44L, SIGLEC1, SLC1A2, IL1R2, OLAH, RETN, MMP8 | 0,862 | 0,852 | 0,872 | 0,927 | 0,875 | 0,978 | 0,954 | 0,897 | 1 |
| IFI27, OAS1, IFI44L, SIGLEC1, ISG15, SLC1A2, IL1R2, OLAH, RETN, MMP8 | 0,862 | 0,852 | 0,872 | 0,930 | 0,881 | 0,979 | 0,952 | 0,898 | 1 |
| RSAD2, IFI27, OAS1, IFIT1, IFI44L, HERC6, SLC1A2, IL1R2, FAM20A, RETN | 0,862 | 0,852 | 0,872 | 0,932 | 0,884 | 0,979 | 0,925 | 0,838 | 1 |
| RSAD2, IFI27, IFIT1, IFI44L, ISG15, SLC1A2, IL1R2, FAM20A, RETN, MMP8 | 0,862 | 0,852 | 0,873 | 0,930 | 0,881 | 0,980 | 0,928 | 0,845 | 1 |
| RSAD2, OAS1, SIGLEC1, ISG15, HERC6, SLC1A2, IL1R2, FAM20A, RETN, MMP8 | 0,862 | 0,852 | 0,872 | 0,926 | 0,878 | 0,974 | 0,958 | 0,892 | 1 |
| RSAD2, OAS1, IFIT1, IFI44L, SIGLEC1, ISG15, HERC6, OLAH, FAM20A, RETN | 0,862 | 0,852 | 0,872 | 0,916 | 0,857 | 0,976 | 0,940 | 0,880 | 1 |
| RSAD2, IFI27, OAS1, SIGLEC1, ISG15, SLC1A2, IL1R2, OLAH, RETN, MMP8 | 0,862 | 0,852 | 0,873 | 0,930 | 0,880 | 0,980 | 0,951 | 0,892 | 1 |
| OAS1, IFIT1, IFI44L, SIGLEC1, ISG15, SLC1A2, IL1R2, FAM20A, RETN, MMP8 | 0,862 | 0,852 | 0,872 | 0,928 | 0,881 | 0,974 | 0,959 | 0,893 | 1 |
| RSAD2, IFIT1, IFI44L, SIGLEC1, ISG15, HERC6, IL1R2, OLAH, FAM20A, RETN | 0,862 | 0,853 | 0,872 | 0,918 | 0,860 | 0,976 | 0,951 | 0,894 | 1 |
| RSAD2, IFI27, IFIT1, IFI44L, SIGLEC1, ISG15, SLC1A2, IL1R2, OLAH, RETN | 0,862 | 0,853 | 0,871 | 0,929 | 0,879 | 0,978 | 0,943 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, SLC1A2, IL1R2, OLAH, FAM20A, RETN, MMP8 | 0,862 | 0,852 | 0,872 | 0,927 | 0,879 | 0,975 | 0,959 | 0,904 | 1 |
| RSAD2, IFI44L, SIGLEC1, ISG15, HERC6, SLC1A2, IL1R2, OLAH, FAM20A, MMP8 | 0,862 | 0,851 | 0,873 | 0,922 | 0,872 | 0,971 | 0,959 | 0,897 | 1 |
| IFIT1, IFI44L, SIGLEC1, ISG15, HERC6, SLC1A2, IL1R2, OLAH, FAM20A, RETN | 0,862 | 0,852 | 0,872 | 0,926 | 0,876 | 0,975 | 0,938 | 0,868 | 1 |
| RSAD2, IFI27, OAS1, IFIT1, IFI44L, SLC1A2, IL1R2, FAM20A, RETN, MMP8 | 0,862 | 0,852 | 0,873 | 0,930 | 0,881 | 0,980 | 0,927 | 0,844 | 1 |
| IFI44L, SIGLEC1, ISG15, HERC6, SLC1A2, IL1R2, OLAH, FAM20A, RETN, MMP8 | 0,862 | 0,852 | 0,872 | 0,923 | 0,873 | 0,972 | 0,957 | 0,890 | 1 |
| RSAD2, IFI27, IFIT1, IFI44L, ISG15, HERC6, SLC1A2, IL1R2, FAM20A, RETN | 0,862 | 0,852 | 0,872 | 0,930 | 0,882 | 0,979 | 0,926 | 0,842 | 1 |
| RSAD2, IFI27, IFIT1, IFI44L, ISG15, HERC6, SLC1A2, IL1R2, FAM20A, MMP8 | 0,862 | 0,852 | 0,872 | 0,930 | 0,883 | 0,977 | 0,927 | 0,841 | 1 |
| RSAD2, OAS1, IFIT1, SIGLEC1, SLC1A2, IL1R2, OLAH, FAM20A, RETN, MMP8 | 0,862 | 0,852 | 0,872 | 0,926 | 0,878 | 0,975 | 0,961 | 0,903 | 1 |
| RSAD2, IFI27, IFI44L, SIGLEC1, ISG15, SLC1A2, IL1R2, OLAH, RETN, MMP8 | 0,862 | 0,852 | 0,872 | 0,931 | 0,883 | 0,979 | 0,954 | 0,903 | 1 |
| RSAD2, IFI44L, SIGLEC1, ISG15, HERC6, IL1R2, OLAH, FAM20A, RETN, MMP8 | 0,862 | 0,852 | 0,872 | 0,918 | 0,860 | 0,975 | 0,961 | 0,903 | 1 |
| RSAD2, IFI27, OAS1, IFIT1, IFI44L, SIGLEC1, ISG15, HERC6, IL1R2, MMP8 | 0,862 | 0,853 | 0,871 | 0,922 | 0,874 | 0,971 | 0,948 | 0,900 | 0,996 |
| RSAD2, IFI27, OAS1, IFIT1, IFI44L, ISG15, HERC6, SLC1A2, FAM20A, RETN | 0,862 | 0,851 | 0,872 | 0,924 | 0,869 | 0,979 | 0,929 | 0,848 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, ISG15, HERC6, SLC1A2, FAM20A, RETN, MMP8 | 0,862 | 0,852 | 0,872 | 0,925 | 0,875 | 0,976 | 0,939 | 0,868 | 1 |
| RSAD2, IFI27, OAS1, IFIT1, IFI44L, HERC6, SLC1A2, IL1R2, OLAH, MMP8 | 0,862 | 0,852 | 0,872 | 0,926 | 0,877 | 0,975 | 0,937 | 0,873 | 1 |
| RSAD2, IFI27, OAS1, IFI44L, ISG15, HERC6, SLC1A2, IL1R2, FAM20A, RETN | 0,862 | 0,851 | 0,873 | 0,933 | 0,886 | 0,980 | 0,926 | 0,840 | 1 |
| RSAD2, IFI27, OAS1, IFI44L, SIGLEC1, ISG15, HERC6, SLC1A2, RETN, MMP8 | 0,862 | 0,852 | 0,871 | 0,923 | 0,868 | 0,978 | 0,942 | 0,877 | 1 |
| RSAD2, IFI27, OAS1, IFIT1, IFI44L, ISG15, HERC6, SLC1A2, OLAH, MMP8 | 0,862 | 0,852 | 0,872 | 0,927 | 0,877 | 0,978 | 0,929 | 0,861 | 0,998 |
| RSAD2, IFI27, OAS1, IFIT1, IFI44L, ISG15, SLC1A2, IL1R2, FAM20A, MMP8 | 0,862 | 0,851 | 0,872 | 0,927 | 0,879 | 0,975 | 0,926 | 0,840 | 1 |
| RSAD2, IFI27, OAS1, IFIT1, IFI44L, SIGLEC1, ISG15, IL1R2, RETN, MMP8 | 0,862 | 0,852 | 0,871 | 0,921 | 0,866 | 0,975 | 0,961 | 0,915 | 1 |
| IFI27, OAS1, IFIT1, IFI44L, ISG15, HERC6, SLC1A2, IL1R2, OLAH, RETN | 0,862 | 0,851 | 0,872 | 0,927 | 0,876 | 0,978 | 0,932 | 0,864 | 0,999 |
| RSAD2, IFI27, OAS1, IFIT1, ISG15, SLC1A2, IL1R2, OLAH, RETN, MMP8 | 0,862 | 0,852 | 0,872 | 0,928 | 0,877 | 0,979 | 0,941 | 0,878 | 1 |
| RSAD2, IFIT1, IFI44L, SIGLEC1, ISG15, SLC1A2, OLAH, FAM20A, RETN, MMP8 | 0,862 | 0,852 | 0,871 | 0,922 | 0,869 | 0,975 | 0,946 | 0,884 | 1 |
| RSAD2, OAS1, IFI44L, SIGLEC1, ISG15, SLC1A2, IL1R2, OLAH, FAM20A, MMP8 | 0,862 | 0,851 | 0,872 | 0,922 | 0,872 | 0,972 | 0,960 | 0,900 | 1 |
| RSAD2, OAS1, SIGLEC1, ISG15, SLC1A2, IL1R2, OLAH, FAM20A, RETN, MMP8 | 0,861 | 0,851 | 0,872 | 0,923 | 0,874 | 0,972 | 0,960 | 0,900 | 1 |
| RSAD2, IFI27, OAS1, IFIT1, IFI44L, SIGLEC1, ISG15, SLC1A2, FAM20A, MMP8 | 0,861 | 0,851 | 0,872 | 0,921 | 0,863 | 0,978 | 0,939 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, ISG15, HERC6, SLC1A2, IL1R2, OLAH | 0,861 | 0,852 | 0,871 | 0,928 | 0,880 | 0,977 | 0,933 | 0,867 | 0,998 |
| RSAD2, IFIT1, SIGLEC1, ISG15, SLC1A2, IL1R2, OLAH, FAM20A, RETN, MMP8 | 0,861 | 0,852 | 0,871 | 0,928 | 0,881 | 0,975 | 0,964 | 0,907 | 1 |
| RSAD2, IFI27, IFIT1, IFI44L, SIGLEC1, HERC6, SLC1A2, IL1R2, RETN, MMP8 | 0,861 | 0,852 | 0,870 | 0,926 | 0,878 | 0,975 | 0,957 | 0,911 | 1 |
| RSAD2, OAS1, IFIT1, IFI44L, SIGLEC1, ISG15, SLC1A2, IL1R2, FAM20A, MMP8 | 0,861 | 0,851 | 0,871 | 0,926 | 0,880 | 0,973 | 0,961 | 0,897 | 1 |
| RSAD2, OAS1, IFI44L, SIGLEC1, ISG15, IL1R2, OLAH, FAM20A, RETN, MMP8 | 0,861 | 0,851 | 0,872 | 0,916 | 0,858 | 0,975 | 0,963 | 0,908 | 1 |
| RSAD2, IFI27, OAS1, IFI44L, ISG15, HERC6, SLC1A2, IL1R2, OLAH, MMP8 | 0,861 | 0,851 | 0,871 | 0,930 | 0,881 | 0,978 | 0,930 | 0,863 | 0,998 |
| RSAD2, OAS1, IFIT1, IFI44L, SIGLEC1, HERC6, SLC1A2, OLAH, FAM20A, RETN | 0,861 | 0,851 | 0,871 | 0,926 | 0,876 | 0,976 | 0,933 | 0,863 | 1 |
| OAS1, IFIT1, SIGLEC1, ISG15, HERC6, SLC1A2, IL1R2, OLAH, FAM20A, RETN | 0,861 | 0,851 | 0,871 | 0,924 | 0,875 | 0,973 | 0,942 | 0,872 | 1 |
| RSAD2, IFI27, OAS1, IFIT1, SIGLEC1, ISG15, SLC1A2, OLAH, RETN, MMP8 | 0,861 | 0,850 | 0,872 | 0,929 | 0,876 | 0,981 | 0,952 | 0,893 | 1 |
| RSAD2, OAS1, SIGLEC1, ISG15, HERC6, SLC1A2, OLAH, FAM20A, RETN, MMP8 | 0,861 | 0,851 | 0,871 | 0,920 | 0,867 | 0,974 | 0,947 | 0,879 | 1 |
| OAS1, IFIT1, IFI44L, SIGLEC1, HERC6, SLC1A2, IL1R2, OLAH, FAM20A, RETN | 0,861 | 0,851 | 0,871 | 0,927 | 0,879 | 0,975 | 0,934 | 0,865 | 1 |
| OAS1, IFI44L, SIGLEC1, ISG15, HERC6, SLC1A2, IL1R2, FAM20A, RETN, MMP8 | 0,861 | 0,851 | 0,871 | 0,926 | 0,878 | 0,974 | 0,959 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, SLC1A2, IL1R2, FAM20A, RETN, MMP8 | 0,861 | 0,851 | 0,871 | 0,931 | 0,883 | 0,980 | 0,927 | 0,844 | 1 |
| RSAD2, IFI27, OAS1, IFI44L, SIGLEC1, ISG15, SLC1A2, OLAH, RETN, MMP8 | 0,861 | 0,851 | 0,871 | 0,929 | 0,877 | 0,981 | 0,954 | 0,896 | 1 |
| RSAD2, OAS1, IFIT1, IFI44L, SIGLEC1, ISG15, HERC6, SLC1A2, IL1R2, FAM20A | 0,861 | 0,851 | 0,871 | 0,926 | 0,878 | 0,973 | 0,946 | 0,879 | 1 |
| RSAD2, IFI27, OAS1, IFI44L, SIGLEC1, ISG15, SLC1A2, FAM20A, RETN, MMP8 | 0,861 | 0,849 | 0,872 | 0,924 | 0,868 | 0,981 | 0,945 | 0,871 | 1 |
| RSAD2, IFI27, IFIT1, IFI44L, ISG15, HERC6, SLC1A2, IL1R2, OLAH, RETN | 0,861 | 0,851 | 0,870 | 0,926 | 0,875 | 0,976 | 0,934 | 0,869 | 0,999 |
| RSAD2, IFI27, IFIT1, IFI44L, ISG15, SLC1A2, IL1R2, OLAH, RETN, MMP8 | 0,861 | 0,851 | 0,870 | 0,926 | 0,874 | 0,978 | 0,943 | 0,885 | 1 |
| RSAD2, IFIT1, IFI44L, SIGLEC1, SLC1A2, IL1R2, OLAH, FAM20A, RETN, MMP8 | 0,861 | 0,851 | 0,871 | 0,927 | 0,879 | 0,975 | 0,959 | 0,902 | 1 |
| RSAD2, IFIT1, SIGLEC1, ISG15, HERC6, SLC1A2, IL1R2, OLAH, FAM20A, RETN | 0,861 | 0,851 | 0,871 | 0,924 | 0,875 | 0,974 | 0,943 | 0,872 | 1 |
| RSAD2, IFIT1, IFI44L, SIGLEC1, ISG15, HERC6, SLC1A2, OLAH, FAM20A, RETN | 0,861 | 0,851 | 0,871 | 0,925 | 0,876 | 0,975 | 0,928 | 0,855 | 1 |
| RSAD2, IFI27, IFIT1, IFI44L, SIGLEC1, ISG15, SLC1A2, OLAH, RETN, MMP8 | 0,861 | 0,850 | 0,871 | 0,930 | 0,879 | 0,980 | 0,952 | 0,897 | 1 |
| RSAD2, IFI27, OAS1, IFIT1, IFI44L, ISG15, SLC1A2, IL1R2, OLAH, MMP8 | 0,861 | 0,851 | 0,870 | 0,926 | 0,877 | 0,976 | 0,934 | 0,869 | 0,998 |
| IFI27, OAS1, IFIT1, IFI44L, ISG15, HERC6, SLC1A2, IL1R2, OLAH, MMP8 | 0,861 | 0,850 | 0,871 | 0,927 | 0,878 | 0,976 | 0,933 | 0,869 | 0,996 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, HERC6, SLC1A2, IL1R2, FAM20A, MMP8 | 0,861 | 0,850 | 0,871 | 0,924 | 0,876 | 0,972 | 0,959 | 0,893 | 1 |
| RSAD2, IFI27, OAS1, IFIT1, IFI44L, SLC1A2, IL1R2, OLAH, RETN, MMP8 | 0,861 | 0,851 | 0,871 | 0,926 | 0,873 | 0,978 | 0,942 | 0,881 | 1 |
| OAS1, IFI44L, SIGLEC1, HERC6, SLC1A2, IL1R2, OLAH, FAM20A, RETN, MMP8 | 0,860 | 0,850 | 0,871 | 0,925 | 0,876 | 0,974 | 0,958 | 0,896 | 1 |
| IFI27, IFIT1, IFI44L, SIGLEC1, ISG15, HERC6, SLC1A2, IL1R2, RETN, MMP8 | 0,860 | 0,851 | 0,870 | 0,926 | 0,878 | 0,974 | 0,954 | 0,908 | 1 |
| RSAD2, OAS1, IFIT1, SIGLEC1, HERC6, SLC1A2, IL1R2, OLAH, FAM20A, RETN | 0,860 | 0,850 | 0,870 | 0,926 | 0,877 | 0,974 | 0,947 | 0,882 | 1 |
| RSAD2, IFIT1, IFI44L, SIGLEC1, ISG15, HERC6, SLC1A2, FAM20A, RETN, MMP8 | 0,860 | 0,851 | 0,870 | 0,925 | 0,874 | 0,976 | 0,939 | 0,866 | 1 |
| IFI27, OAS1, IFIT1, IFI44L, SIGLEC1, ISG15, SLC1A2, IL1R2, RETN, MMP8 | 0,860 | 0,851 | 0,870 | 0,926 | 0,879 | 0,974 | 0,958 | 0,909 | 1 |
| RSAD2, IFIT1, IFI44L, SIGLEC1, HERC6, SLC1A2, IL1R2, OLAH, FAM20A, RETN | 0,860 | 0,850 | 0,870 | 0,926 | 0,877 | 0,974 | 0,937 | 0,867 | 1 |
| OAS1, IFI44L, SIGLEC1, ISG15, SLC1A2, IL1R2, OLAH, FAM20A, RETN, MMP8 | 0,860 | 0,850 | 0,870 | 0,922 | 0,872 | 0,972 | 0,960 | 0,900 | 1 |
| RSAD2, IFIT1, IFI44L, SIGLEC1, ISG15, SLC1A2, IL1R2, FAM20A, RETN, MMP8 | 0,860 | 0,851 | 0,870 | 0,927 | 0,880 | 0,974 | 0,959 | 0,893 | 1 |
| OAS1, IFIT1, IFI44L, SIGLEC1, ISG15, HERC6, SLC1A2, IL1R2, FAM20A, RETN | 0,860 | 0,850 | 0,870 | 0,926 | 0,879 | 0,973 | 0,941 | 0,872 | 1 |
| RSAD2, OAS1, IFIT1, IFI44L, SIGLEC1, ISG15, IL1R2, OLAH, FAM20A, RETN | 0,860 | 0,850 | 0,870 | 0,911 | 0,849 | 0,973 | 0,963 | 0,913 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, SIGLEC1, ISG15, HERC6, SLC1A2, OLAH, FAM20A, RETN | 0,860 | 0,850 | 0,870 | 0,924 | 0,874 | 0,974 | 0,941 | 0,875 | 1 |
| RSAD2, IFI27, IFIT1, SIGLEC1, ISG15, HERC6, SLC1A2, IL1R2, RETN, MMP8 | 0,860 | 0,851 | 0,869 | 0,926 | 0,878 | 0,973 | 0,962 | 0,921 | 1 |
| IFI27, OAS1, IFIT1, IFI44L, ISG15, SLC1A2, IL1R2, OLAH, RETN, MMP8 | 0,860 | 0,850 | 0,870 | 0,926 | 0,874 | 0,978 | 0,941 | 0,879 | 1 |
| RSAD2, OAS1, IFIT1, IFI44L, SIGLEC1, SLC1A2, OLAH, FAM20A, RETN, MMP8 | 0,860 | 0,849 | 0,871 | 0,925 | 0,873 | 0,977 | 0,948 | 0,888 | 1 |
| RSAD2, OAS1, IFIT1, IFI44L, SIGLEC1, HERC6, SLC1A2, FAM20A, RETN, MMP8 | 0,860 | 0,850 | 0,870 | 0,924 | 0,872 | 0,975 | 0,939 | 0,867 | 1 |
| RSAD2, IFI27, OAS1, IFIT1, IFI44L, HERC6, SLC1A2, FAM20A, RETN, MMP8 | 0,860 | 0,849 | 0,871 | 0,922 | 0,866 | 0,979 | 0,936 | 0,854 | 1 |
| RSAD2, OAS1, IFIT1, SIGLEC1, ISG15, SLC1A2, IL1R2, FAM20A, RETN, MMP8 | 0,860 | 0,850 | 0,869 | 0,928 | 0,881 | 0,975 | 0,959 | 0,893 | 1 |
| RSAD2, IFI27, IFIT1, IFI44L, ISG15, HERC6, SLC1A2, IL1R2, OLAH, MMP8 | 0,860 | 0,850 | 0,869 | 0,929 | 0,880 | 0,977 | 0,932 | 0,868 | 0,995 |
| RSAD2, IFI27, OAS1, IFI44L, ISG15, SLC1A2, IL1R2, FAM20A, RETN, MMP8 | 0,860 | 0,849 | 0,871 | 0,930 | 0,882 | 0,978 | 0,923 | 0,835 | 1 |
| RSAD2, OAS1, IFIT1, SIGLEC1, ISG15, HERC6, SLC1A2, FAM20A, RETN, MMP8 | 0,860 | 0,850 | 0,870 | 0,922 | 0,869 | 0,975 | 0,943 | 0,873 | 1 |
| RSAD2, IFI27, OAS1, IFI44L, ISG15, SLC1A2, IL1R2, OLAH, RETN, MMP8 | 0,859 | 0,849 | 0,870 | 0,929 | 0,877 | 0,980 | 0,939 | 0,874 | 1 |
| RSAD2, IFI44L, SIGLEC1, HERC6, SLC1A2, IL1R2, OLAH, FAM20A, RETN, MMP8 | 0,859 | 0,849 | 0,870 | 0,926 | 0,878 | 0,974 | 0,959 | 0,898 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, ISG15, HERC6, SLC1A2, FAM20A, RETN, MMP8 | 0,859 | 0,849 | 0,870 | 0,927 | 0,872 | 0,981 | 0,936 | 0,857 | 1 |
| RSAD2, IFI27, OAS1, IFIT1, IFI44L, ISG15, HERC6, SLC1A2, IL1R2, RETN | 0,859 | 0,850 | 0,868 | 0,922 | 0,871 | 0,974 | 0,932 | 0,859 | 1 |
| RSAD2, IFI27, OAS1, IFIT1, IFI44L, ISG15, SLC1A2, IL1R2, OLAH, RETN | 0,859 | 0,849 | 0,869 | 0,928 | 0,877 | 0,979 | 0,935 | 0,865 | 1 |
| RSAD2, OAS1, IFI44L, SIGLEC1, ISG15, HERC6, SLC1A2, OLAH, FAM20A, MMP8 | 0,859 | 0,849 | 0,870 | 0,921 | 0,869 | 0,973 | 0,943 | 0,876 | 1 |
| RSAD2, IFI27, OAS1, IFIT1, IFI44L, ISG15, SLC1A2, OLAH, RETN, MMP8 | 0,859 | 0,849 | 0,869 | 0,924 | 0,869 | 0,979 | 0,941 | 0,877 | 1 |
| RSAD2, OAS1, IFIT1, IFI44L, SIGLEC1, SLC1A2, IL1R2, FAM20A, RETN, MMP8 | 0,859 | 0,849 | 0,869 | 0,927 | 0,881 | 0,974 | 0,963 | 0,906 | 1 |
| RSAD2, IFI27, OAS1, IFIT1, IFI44L, HERC6, SLC1A2, IL1R2, RETN, MMP8 | 0,859 | 0,850 | 0,869 | 0,923 | 0,872 | 0,974 | 0,935 | 0,866 | 1 |
| RSAD2, IFI44L, SIGLEC1, ISG15, SLC1A2, IL1R2, OLAH, FAM20A, RETN, MMP8 | 0,859 | 0,849 | 0,869 | 0,921 | 0,871 | 0,971 | 0,959 | 0,897 | 1 |
| RSAD2, IFI27, OAS1, IFIT1, ISG15, HERC6, SLC1A2, FAM20A, RETN, MMP8 | 0,859 | 0,848 | 0,870 | 0,922 | 0,866 | 0,979 | 0,933 | 0,851 | 1 |
| RSAD2, OAS1, IFIT1, IFI44L, SIGLEC1, ISG15, OLAH, FAM20A, RETN, MMP8 | 0,859 | 0,848 | 0,870 | 0,913 | 0,849 | 0,978 | 0,959 | 0,910 | 1 |
| RSAD2, IFI27, OAS1, IFI44L, ISG15, HERC6, SLC1A2, FAM20A, RETN, MMP8 | 0,859 | 0,847 | 0,871 | 0,925 | 0,869 | 0,980 | 0,935 | 0,854 | 1 |
| RSAD2, OAS1, IFI44L, SIGLEC1, HERC6, SLC1A2, IL1R2, FAM20A, RETN, MMP8 | 0,859 | 0,849 | 0,869 | 0,926 | 0,878 | 0,974 | 0,957 | 0,891 | 1 |
| OAS1, IFI44L, SIGLEC1, ISG15, HERC6, SLC1A2, OLAH, FAM20A, RETN, MMP8 | 0,859 | 0,849 | 0,869 | 0,918 | 0,864 | 0,972 | 0,946 | 0,880 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, SLC1A2, IL1R2, RETN | 0,859 | 0,850 | 0,868 | 0,926 | 0,877 | 0,975 | 0,949 | 0,888 | 1 |
| RSAD2, IFI44L, SIGLEC1, ISG15, HERC6, SLC1A2, IL1R2, FAM20A, RETN, MMP8 | 0,859 | 0,849 | 0,869 | 0,925 | 0,877 | 0,973 | 0,955 | 0,887 | 1 |
| RSAD2, OAS1, IFIT1, SIGLEC1, ISG15, HERC6, SLC1A2, IL1R2, FAM20A, RETN | 0,859 | 0,849 | 0,869 | 0,925 | 0,877 | 0,973 | 0,946 | 0,879 | 1 |
| RSAD2, IFI27, OAS1, IFIT1, IFI44L, SIGLEC1, SLC1A2, IL1R2, RETN, MMP8 | 0,859 | 0,850 | 0,868 | 0,925 | 0,876 | 0,974 | 0,957 | 0,902 | 1 |
| RSAD2, IFI27, OAS1, IFIT1, ISG15, HERC6, SLC1A2, IL1R2, RETN, MMP8 | 0,859 | 0,850 | 0,868 | 0,925 | 0,875 | 0,975 | 0,940 | 0,876 | 1 |
| RSAD2, IFI27, IFIT1, IFI44L, SIGLEC1, ISG15, HERC6, SLC1A2, IL1R2, RETN | 0,859 | 0,850 | 0,867 | 0,928 | 0,880 | 0,976 | 0,946 | 0,894 | 0,997 |
| RSAD2, IFI27, OAS1, IFI44L, SIGLEC1, ISG15, HERC6, SLC1A2, FAM20A, MMP8 | 0,859 | 0,848 | 0,869 | 0,921 | 0,868 | 0,974 | 0,939 | 0,863 | 1 |
| RSAD2, OAS1, IFIT1, IFI44L, SIGLEC1, ISG15, SLC1A2, IL1R2, OLAH, FAM20A | 0,859 | 0,849 | 0,868 | 0,921 | 0,869 | 0,974 | 0,950 | 0,892 | 1 |
| RSAD2, IFI27, OAS1, IFIT1, IFI44L, SIGLEC1, ISG15, HERC6, SLC1A2, IL1R2 | 0,859 | 0,850 | 0,867 | 0,926 | 0,880 | 0,971 | 0,954 | 0,908 | 1 |
| RSAD2, IFI27, IFI44L, SIGLEC1, ISG15, HERC6, SLC1A2, IL1R2, RETN, MMP8 | 0,858 | 0,850 | 0,867 | 0,928 | 0,880 | 0,975 | 0,954 | 0,908 | 1 |
| RSAD2, IFIT1, IFI44L, SIGLEC1, ISG15, HERC6, SLC1A2, IL1R2, FAM20A, RETN | 0,858 | 0,849 | 0,868 | 0,926 | 0,878 | 0,974 | 0,941 | 0,869 | 1 |
| IFI27, OAS1, IFIT1, IFI44L, ISG15, HERC6, SLC1A2, IL1R2, RETN, MMP8 | 0,858 | 0,849 | 0,868 | 0,924 | 0,874 | 0,974 | 0,936 | 0,870 | 1 |
| OAS1, IFIT1, IFI44L, SIGLEC1, ISG15, SLC1A2, IL1R2, OLAH, FAM20A, RETN | 0,858 | 0,848 | 0,868 | 0,920 | 0,867 | 0,973 | 0,945 | 0,884 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, HERC6, SLC1A2, IL1R2, MMP8 | 0,858 | 0,849 | 0,868 | 0,927 | 0,881 | 0,973 | 0,954 | 0,906 | 1 |
| RSAD2, OAS1, IFIT1, SIGLEC1, ISG15, SLC1A2, OLAH, FAM20A, RETN, MMP8 | 0,858 | 0,848 | 0,869 | 0,922 | 0,869 | 0,975 | 0,951 | 0,891 | 1 |
| RSAD2, IFI27, OAS1, IFI44L, ISG15, HERC6, SLC1A2, IL1R2, RETN, MMP8 | 0,858 | 0,849 | 0,868 | 0,925 | 0,874 | 0,975 | 0,933 | 0,860 | 1 |
| RSAD2, OAS1, IFIT1, IFI44L, SIGLEC1, ISG15, HERC6, SLC1A2, FAM20A, RETN | 0,858 | 0,848 | 0,868 | 0,924 | 0,875 | 0,974 | 0,934 | 0,864 | 1 |
| RSAD2, OAS1, IFIT1, IFI44L, SIGLEC1, HERC6, SLC1A2, IL1R2, FAM20A, RETN | 0,858 | 0,848 | 0,868 | 0,926 | 0,879 | 0,974 | 0,945 | 0,878 | 1 |
| RSAD2, OAS1, IFIT1, IFI44L, SIGLEC1, SLC1A2, IL1R2, OLAH, FAM20A, RETN | 0,858 | 0,848 | 0,868 | 0,921 | 0,868 | 0,974 | 0,949 | 0,890 | 1 |
| RSAD2, OAS1, IFIT1, SIGLEC1, ISG15, SLC1A2, IL1R2, OLAH, FAM20A, RETN | 0,858 | 0,848 | 0,868 | 0,922 | 0,870 | 0,973 | 0,959 | 0,901 | 1 |
| RSAD2, IFI27, OAS1, IFIT1, IFI44L, ISG15, SLC1A2, IL1R2, FAM20A, RETN | 0,858 | 0,847 | 0,869 | 0,926 | 0,877 | 0,976 | 0,922 | 0,834 | 1 |
| RSAD2, OAS1, IFI44L, SIGLEC1, ISG15, SLC1A2, IL1R2, FAM20A, RETN, MMP8 | 0,858 | 0,848 | 0,868 | 0,926 | 0,878 | 0,974 | 0,959 | 0,895 | 1 |
| RSAD2, IFI27, OAS1, IFIT1, IFI44L, ISG15, HERC6, SLC1A2, RETN, MMP8 | 0,858 | 0,848 | 0,868 | 0,918 | 0,860 | 0,975 | 0,927 | 0,851 | 1 |
| RSAD2, IFI27, OAS1, IFI44L, SIGLEC1, ISG15, SLC1A2, IL1R2, RETN, MMP8 | 0,858 | 0,849 | 0,867 | 0,927 | 0,879 | 0,975 | 0,959 | 0,906 | 1 |
| RSAD2, IFIT1, IFI44L, SIGLEC1, ISG15, SLC1A2, IL1R2, OLAH, FAM20A, RETN | 0,858 | 0,848 | 0,867 | 0,922 | 0,869 | 0,974 | 0,950 | 0,890 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SIGLEC1, ISG15, HERC6, SLC1A2, FAM20A, MMP8 | 0,858 | 0,847 | 0,868 | 0,920 | 0,868 | 0,972 | 0,929 | 0,852 | 1 |
| RSAD2, OAS1, IFI44L, SIGLEC1, SLC1A2, IL1R2, OLAH, FAM20A, RETN, MMP8 | 0,858 | 0,847 | 0,868 | 0,923 | 0,874 | 0,973 | 0,959 | 0,899 | 1 |
| RSAD2, IFI27, OAS1, IFI44L, SIGLEC1, ISG15, HERC6, SLC1A2, IL1R2, MMP8 | 0,858 | 0,849 | 0,866 | 0,926 | 0,880 | 0,972 | 0,953 | 0,905 | 1 |
| RSAD2, OAS1, IFIT1, IFI44L, SIGLEC1, ISG15, SLC1A2, OLAH, FAM20A, RETN | 0,857 | 0,848 | 0,867 | 0,920 | 0,865 | 0,976 | 0,937 | 0,873 | 1 |
| RSAD2, OAS1, IFI44L, SIGLEC1, HERC6, SLC1A2, OLAH, FAM20A, RETN, MMP8 | 0,857 | 0,847 | 0,868 | 0,921 | 0,867 | 0,974 | 0,941 | 0,872 | 1 |
| RSAD2, IFI27, OAS1, IFIT1, SIGLEC1, ISG15, SLC1A2, IL1R2, RETN, MMP8 | 0,857 | 0,848 | 0,866 | 0,926 | 0,877 | 0,974 | 0,961 | 0,908 | 1 |
| RSAD2, IFI27, IFIT1, IFI44L, SIGLEC1, ISG15, SLC1A2, IL1R2, RETN, MMP8 | 0,857 | 0,848 | 0,866 | 0,926 | 0,878 | 0,974 | 0,958 | 0,912 | 1 |
| IFI27, OAS1, IFIT1, IFI44L, SIGLEC1, ISG15, HERC6, SLC1A2, IL1R2, MMP8 | 0,857 | 0,848 | 0,867 | 0,925 | 0,880 | 0,970 | 0,950 | 0,902 | 0,998 |
| RSAD2, IFI44L, SIGLEC1, ISG15, HERC6, SLC1A2, OLAH, FAM20A, RETN, MMP8 | 0,857 | 0,847 | 0,867 | 0,920 | 0,866 | 0,974 | 0,941 | 0,870 | 1 |
| RSAD2, OAS1, IFI44L, SIGLEC1, ISG15, HERC6, OLAH, FAM20A, RETN, MMP8 | 0,856 | 0,846 | 0,866 | 0,916 | 0,856 | 0,976 | 0,951 | 0,893 | 1 |
| RSAD2, OAS1, SIGLEC1, ISG15, HERC6, SLC1A2, IL1R2, OLAH, FAM20A, RETN | 0,856 | 0,845 | 0,867 | 0,919 | 0,867 | 0,970 | 0,950 | 0,885 | 1 |
| RSAD2, OAS1, IFI44L, SIGLEC1, ISG15, HERC6, SLC1A2, IL1R2, OLAH, FAM20A | 0,856 | 0,845 | 0,867 | 0,919 | 0,867 | 0,970 | 0,946 | 0,878 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFI44L, SIGLEC1, ISG15, HERC6, IL1R2, OLAH, FAM20A, RETN | 0,856 | 0,845 | 0,866 | 0,913 | 0,854 | 0,972 | 0,959 | 0,905 | 1 |
| RSAD2, IFI27, OAS1, IFIT1, IFI44L, SIGLEC1, ISG15, SLC1A2, IL1R2, MMP8 | 0,856 | 0,847 | 0,864 | 0,927 | 0,881 | 0,972 | 0,954 | 0,907 | 1 |
| RSAD2, IFI27, OAS1, IFIT1, SIGLEC1, ISG15, HERC6, SLC1A2, IL1R2, MMP8 | 0,856 | 0,846 | 0,865 | 0,926 | 0,881 | 0,972 | 0,952 | 0,903 | 1 |
| RSAD2, OAS1, IFI44L, SIGLEC1, ISG15, SLC1A2, OLAH, FAM20A, RETN, MMP8 | 0,856 | 0,845 | 0,866 | 0,915 | 0,858 | 0,971 | 0,945 | 0,881 | 1 |
| RSAD2, OAS1, IFIT1, IFI44L, SIGLEC1, ISG15, SLC1A2, IL1R2, FAM20A, RETN | 0,855 | 0,846 | 0,865 | 0,921 | 0,870 | 0,972 | 0,954 | 0,894 | 1 |
| OAS1, IFI44L, SIGLEC1, ISG15, HERC6, SLC1A2, IL1R2, OLAH, FAM20A, RETN | 0,854 | 0,843 | 0,865 | 0,919 | 0,869 | 0,970 | 0,948 | 0,880 | 1 |
| RSAD2, IFI27, IFIT1, IFI44L, SIGLEC1, ISG15, HERC6, SLC1A2, RETN, MMP8 | 0,854 | 0,845 | 0,863 | 0,923 | 0,871 | 0,975 | 0,945 | 0,890 | 1,000 |
| RSAD2, IFI27, IFIT1, IFI44L, SIGLEC1, ISG15, HERC6, SLC1A2, IL1R2, MMP8 | 0,854 | 0,845 | 0,863 | 0,925 | 0,879 | 0,971 | 0,950 | 0,902 | 0,998 |
| RSAD2, IFI27, IFIT1, IFI44L, ISG15, HERC6, SLC1A2, IL1R2, RETN, MMP8 | 0,854 | 0,844 | 0,863 | 0,923 | 0,872 | 0,974 | 0,941 | 0,880 | 1 |
| RSAD2, OAS1, IFI44L, SIGLEC1, ISG15, HERC6, SLC1A2, FAM20A, RETN, MMP8 | 0,853 | 0,843 | 0,864 | 0,916 | 0,859 | 0,974 | 0,946 | 0,874 | 1 |
| RSAD2, OAS1, IFI44L, SIGLEC1, HERC6, SLC1A2, IL1R2, OLAH, FAM20A, RETN | 0,853 | 0,842 | 0,864 | 0,919 | 0,868 | 0,970 | 0,948 | 0,880 | 1 |
| RSAD2, IFI27, OAS1, IFIT1, IFI44L, ISG15, SLC1A2, IL1R2, RETN, MMP8 | 0,853 | 0,844 | 0,862 | 0,920 | 0,868 | 0,973 | 0,938 | 0,872 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI44L, SIGLEC1, ISG15, HERC6, SLC1A2, IL1R2, OLAH, FAM20A, RETN | 0,853 | 0,842 | 0,864 | 0,919 | 0,867 | 0,970 | 0,948 | 0,877 | 1 |
| OAS1, IFIT1, IFI44L, ISG15, HERC6, SLC1A2, OLAH, FAM20A, RETN, MMP8 | 0,852 | 0,842 | 0,863 | 0,922 | 0,870 | 0,974 | 0,921 | 0,834 | 1 |
| RSAD2, IFI27, OAS1, IFIT1, IFI44L, ISG15, HERC6, SLC1A2, IL1R2, MMP8 | 0,852 | 0,843 | 0,862 | 0,922 | 0,875 | 0,969 | 0,936 | 0,872 | 1 |
| RSAD2, OAS1, IFI44L, SIGLEC1, ISG15, SLC1A2, IL1R2, OLAH, FAM20A, RETN | 0,852 | 0,842 | 0,863 | 0,917 | 0,865 | 0,970 | 0,958 | 0,900 | 1 |
| RSAD2, OAS1, IFIT1, IFI44L, HERC6, SLC1A2, IL1R2, OLAH, FAM20A, MMP8 | 0,852 | 0,842 | 0,863 | 0,921 | 0,871 | 0,971 | 0,927 | 0,845 | 1 |
| RSAD2, OAS1, IFI44L, SIGLEC1, ISG15, HERC6, SLC1A2, IL1R2, FAM20A, RETN | 0,852 | 0,841 | 0,863 | 0,919 | 0,869 | 0,970 | 0,951 | 0,883 | 1 |
| RSAD2, OAS1, IFIT1, IFI44L, HERC6, SLC1A2, OLAH, FAM20A, RETN, MMP8 | 0,852 | 0,842 | 0,862 | 0,920 | 0,867 | 0,972 | 0,922 | 0,838 | 1 |
| RSAD2, OAS1, IFI44L, SIGLEC1, ISG15, HERC6, SLC1A2, OLAH, FAM20A, RETN | 0,852 | 0,841 | 0,863 | 0,917 | 0,863 | 0,972 | 0,941 | 0,872 | 1 |
| RSAD2, IFI27, OAS1, IFIT1, IFI44L, ISG15, HERC6, SLC1A2, FAM20A, MMP8 | 0,852 | 0,840 | 0,863 | 0,918 | 0,863 | 0,972 | 0,911 | 0,822 | 1 |
| RSAD2, OAS1, IFIT1, IFI44L, ISG15, HERC6, SLC1A2, OLAH, FAM20A, MMP8 | 0,852 | 0,841 | 0,863 | 0,919 | 0,867 | 0,971 | 0,918 | 0,836 | 1 |
| RSAD2, OAS1, IFI44L, ISG15, HERC6, IL1R2, OLAH, FAM20A, RETN, MMP8 | 0,851 | 0,840 | 0,862 | 0,916 | 0,858 | 0,974 | 0,939 | 0,865 | 1 |
| RSAD2, OAS1, IFI44L, ISG15, HERC6, SLC1A2, IL1R2, OLAH, FAM20A, MMP8 | 0,851 | 0,840 | 0,862 | 0,919 | 0,868 | 0,970 | 0,927 | 0,845 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, SLC1A2, RETN, MMP8 | 0,851 | 0,841 | 0,861 | 0,915 | 0,858 | 0,973 | 0,947 | 0,877 | 1 |
| RSAD2, OAS1, IFIT1, IFI44L, ISG15, HERC6, IL1R2, OLAH, FAM20A, MMP8 | 0,851 | 0,840 | 0,862 | 0,912 | 0,854 | 0,970 | 0,932 | 0,853 | 1 |
| OAS1, IFIT1, IFI44L, ISG15, HERC6, SLC1A2, IL1R2, OLAH, FAM20A, MMP8 | 0,851 | 0,840 | 0,861 | 0,918 | 0,866 | 0,969 | 0,927 | 0,844 | 1 |
| IFIT1, IFI44L, ISG15, HERC6, SLC1A2, IL1R2, OLAH, FAM20A, RETN, MMP8 | 0,850 | 0,840 | 0,861 | 0,920 | 0,869 | 0,972 | 0,927 | 0,842 | 1 |
| RSAD2, OAS1, IFI44L, HERC6, SLC1A2, IL1R2, OLAH, FAM20A, RETN, MMP8 | 0,850 | 0,840 | 0,861 | 0,919 | 0,867 | 0,971 | 0,929 | 0,847 | 1 |
| RSAD2, OAS1, IFIT1, IFI44L, HERC6, IL1R2, OLAH, FAM20A, RETN, MMP8 | 0,850 | 0,840 | 0,861 | 0,913 | 0,855 | 0,972 | 0,937 | 0,861 | 1 |
| RSAD2, OAS1, IFIT1, IFI44L, SIGLEC1, ISG15, SLC1A2, FAM20A, RETN, MMP8 | 0,850 | 0,840 | 0,861 | 0,918 | 0,862 | 0,974 | 0,938 | 0,867 | 1 |
| RSAD2, IFIT1, IFI44L, HERC6, SLC1A2, IL1R2, OLAH, FAM20A, RETN, MMP8 | 0,850 | 0,840 | 0,860 | 0,921 | 0,869 | 0,972 | 0,925 | 0,841 | 1 |
| RSAD2, OAS1, IFIT1, ISG15, HERC6, IL1R2, OLAH, FAM20A, RETN, MMP8 | 0,850 | 0,840 | 0,860 | 0,911 | 0,850 | 0,971 | 0,942 | 0,869 | 1 |

Performances obtenues pour l'identification de la nature virale ou bactérienne de l'infection sur la base des combinaisons à 11 gènes cibles :

[Tableau 15]

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, HERC6, IL1R2, OLAH, FAM20A, MMP8 | 0,880 | 0,870 | 0,889 | 0,938 | 0,889 | 0,987 | 0,951 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, ISG15, HERC6, IL1R2, OLAH, FAM20A, MMP8 | 0,879 | 0,869 | 0,889 | 0,938 | 0,889 | 0,987 | 0,952 | 0,891 | 1 |
| IFI27, OAS1, IFIT1, IFI44L, SIGLEC1, HERC6, IL1R2, OLAH, FAM20A, RETN, MMP8 | 0,879 | 0,869 | 0,889 | 0,939 | 0,888 | 0,990 | 0,951 | 0,889 | 1 |
| IFI27, OAS1, IFIT1, SIGLEC1, ISG15, HERC6, IL1R2, OLAH, FAM20A, RETN, MMP8 | 0,879 | 0,868 | 0,889 | 0,939 | 0,888 | 0,990 | 0,953 | 0,893 | 1 |
| IFI27, OAS1, IFIT1, IFI44L, SIGLEC1, ISG15, HERC6, IL1R2, OLAH, FAM20A, MMP8 | 0,878 | 0,868 | 0,889 | 0,938 | 0,889 | 0,986 | 0,947 | 0,882 | 1 |
| RSAD2, IFI27, OAS1, IFIT1, SIGLEC1, HERC6, IL1R2, OLAH, FAM20A, RETN, MMP8 | 0,878 | 0,868 | 0,889 | 0,938 | 0,886 | 0,989 | 0,949 | 0,885 | 1 |
| RSAD2, IFI27, OAS1, IFI44L, SIGLEC1, ISG15, HERC6, IL1R2, OLAH, FAM20A, MMP8 | 0,877 | 0,866 | 0,888 | 0,938 | 0,889 | 0,986 | 0,947 | 0,883 | 1 |
| RSAD2, IFI27, OAS1, IFIT1, IFI44L, SIGLEC1, HERC6, OLAH, FAM20A, RETN, MMP8 | 0,877 | 0,866 | 0,887 | 0,936 | 0,883 | 0,989 | 0,946 | 0,882 | 1 |
| RSAD2, IFI27, IFIT1, IFI44L, SIGLEC1, ISG15, HERC6, IL1R2, OLAH, FAM20A, MMP8 | 0,877 | 0,866 | 0,887 | 0,938 | 0,890 | 0,986 | 0,947 | 0,883 | 1 |
| RSAD2, IFI27, OAS1, IFIT1, SIGLEC1, ISG15, HERC6, OLAH, FAM20A, RETN, MMP8 | 0,876 | 0,865 | 0,887 | 0,937 | 0,884 | 0,989 | 0,949 | 0,887 | 1 |
| RSAD2, IFI27, OAS1, SIGLEC1, ISG15, HERC6, IL1R2, OLAH, FAM20A, RETN, MMP8 | 0,876 | 0,865 | 0,887 | 0,937 | 0,885 | 0,988 | 0,949 | 0,885 | 1 |
| IFI27, IFIT1, IFI44L, SIGLEC1, ISG15, HERC6, IL1R2, OLAH, FAM20A, RETN, MMP8 | 0,876 | 0,866 | 0,887 | 0,938 | 0,886 | 0,989 | 0,949 | 0,884 | 1 |
| IFI27, OAS1, IFI44L, SIGLEC1, ISG15, HERC6, IL1R2, OLAH, FAM20A, RETN, MMP8 | 0,876 | 0,866 | 0,887 | 0,937 | 0,886 | 0,989 | 0,948 | 0,883 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, IL1R2, OLAH, FAM20A, MMP8 | 0,876 | 0,866 | 0,887 | 0,936 | 0,887 | 0,986 | 0,951 | 0,890 | 1 |
| IFI27, OAS1, IFIT1, IFI44L, SIGLEC1, ISG15, HERC6, OLAH, FAM20A, RETN, MMP8 | 0,875 | 0,864 | 0,887 | 0,936 | 0,884 | 0,989 | 0,946 | 0,881 | 1 |
| RSAD2, IFI27, IFIT1, SIGLEC1, ISG15, HERC6, IL1R2, OLAH, FAM20A, RETN, MMP8 | 0,875 | 0,864 | 0,886 | 0,937 | 0,886 | 0,988 | 0,950 | 0,888 | 1 |
| RSAD2, IFI27, OAS1, IFI44L, SIGLEC1, HERC6, IL1R2, OLAH, FAM20A, RETN, MMP8 | 0,875 | 0,864 | 0,886 | 0,937 | 0,885 | 0,989 | 0,949 | 0,884 | 1 |
| IFI27, OAS1, IFIT1, IFI44L, SIGLEC1, ISG15, HERC6, IL1R2, FAM20A, RETN, MMP8 | 0,875 | 0,864 | 0,886 | 0,938 | 0,888 | 0,988 | 0,950 | 0,881 | 1 |
| RSAD2, IFI27, OAS1, IFIT1, SIGLEC1, ISG15, HERC6, IL1R2, FAM20A, RETN, MMP8 | 0,875 | 0,865 | 0,885 | 0,937 | 0,887 | 0,988 | 0,952 | 0,885 | 1 |
| RSAD2, IFI27, IFIT1, IFI44L, SIGLEC1, HERC6, IL1R2, OLAH, FAM20A, RETN, MMP8 | 0,875 | 0,864 | 0,886 | 0,937 | 0,886 | 0,989 | 0,951 | 0,888 | 1 |
| RSAD2, IFI27, OAS1, IFIT1, SIGLEC1, ISG15, HERC6, IL1R2, OLAH, FAM20A, RETN | 0,874 | 0,865 | 0,884 | 0,933 | 0,880 | 0,986 | 0,939 | 0,875 | 1 |
| RSAD2, IFI27, OAS1, IFIT1, IFI44L, SIGLEC1, ISG15, HERC6, IL1R2, OLAH, FAM20A | 0,874 | 0,865 | 0,884 | 0,933 | 0,881 | 0,984 | 0,941 | 0,876 | 1 |
| RSAD2, IFI27, OAS1, IFIT1, IFI44L, SIGLEC1, HERC6, IL1R2, FAM20A, RETN, MMP8 | 0,874 | 0,863 | 0,885 | 0,938 | 0,889 | 0,988 | 0,951 | 0,882 | 1 |
| RSAD2, IFI27, IFIT1, SIGLEC1, ISG15, HERC6, SLC1A2, IL1R2, OLAH, FAM20A, MMP8 | 0,874 | 0,864 | 0,884 | 0,944 | 0,901 | 0,986 | 0,950 | 0,886 | 1 |
| RSAD2, IFI27, OAS1, IFIT1, IFI44L, SIGLEC1, HERC6, IL1R2, OLAH, FAM20A, RETN | 0,874 | 0,864 | 0,884 | 0,930 | 0,877 | 0,984 | 0,940 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, HERC6, SLC1A2, IL1R2, OLAH, FAM20A, MMP8 | 0,874 | 0,863 | 0,884 | 0,941 | 0,897 | 0,985 | 0,947 | 0,882 | 1 |
| RSAD2, IFI27, OAS1, IFIT1, SIGLEC1, ISG15, IL1R2, OLAH, FAM20A, RETN, MMP8 | 0,874 | 0,863 | 0,885 | 0,935 | 0,883 | 0,988 | 0,953 | 0,892 | 1 |
| RSAD2, IFI27, OAS1, IFIT1, IFI44L, SIGLEC1, ISG15, HERC6, OLAH, FAM20A, MMP8 | 0,873 | 0,862 | 0,885 | 0,934 | 0,881 | 0,986 | 0,945 | 0,883 | 1 |
| IFI27, IFIT1, IFI44L, SIGLEC1, ISG15, HERC6, SLC1A2, IL1R2, OLAH, FAM20A, MMP8 | 0,873 | 0,863 | 0,883 | 0,943 | 0,900 | 0,986 | 0,949 | 0,883 | 1 |
| IFI27, OAS1, IFIT1, IFI44L, SIGLEC1, ISG15, HERC6, IL1R2, OLAH, FAM20A, RETN | 0,873 | 0,863 | 0,883 | 0,931 | 0,878 | 0,985 | 0,938 | 0,872 | 1 |
| IFI27, OAS1, IFIT1, SIGLEC1, ISG15, HERC6, SLC1A2, IL1R2, OLAH, FAM20A, MMP8 | 0,873 | 0,862 | 0,885 | 0,942 | 0,899 | 0,986 | 0,947 | 0,879 | 1 |
| RSAD2, IFI27, OAS1, IFIT1, SIGLEC1, ISG15, SLC1A2, IL1R2, OLAH, FAM20A, MMP8 | 0,873 | 0,863 | 0,883 | 0,943 | 0,900 | 0,986 | 0,947 | 0,880 | 1 |
| RSAD2, IFI27, OAS1, IFIT1, IFI44L, SIGLEC1, ISG15, HERC6, OLAH, FAM20A, RETN | 0,873 | 0,863 | 0,883 | 0,931 | 0,878 | 0,985 | 0,940 | 0,876 | 1 |
| IFI27, OAS1, IFIT1, IFI44L, SIGLEC1, ISG15, IL1R2, OLAH, FAM20A, RETN, MMP8 | 0,873 | 0,861 | 0,885 | 0,936 | 0,884 | 0,988 | 0,950 | 0,887 | 1 |
| IFI27, OAS1, IFIT1, IFI44L, SIGLEC1, HERC6, SLC1A2, IL1R2, OLAH, FAM20A, MMP8 | 0,873 | 0,862 | 0,884 | 0,941 | 0,896 | 0,985 | 0,946 | 0,880 | 1 |
| IFI27, OAS1, IFIT1, IFI44L, SIGLEC1, HERC6, SLC1A2, IL1R2, FAM20A, RETN, MMP8 | 0,873 | 0,863 | 0,883 | 0,940 | 0,896 | 0,984 | 0,946 | 0,874 | 1 |
| IFI27, OAS1, SIGLEC1, ISG15, HERC6, SLC1A2, IL1R2, OLAH, FAM20A, RETN, MMP8 | 0,873 | 0,862 | 0,883 | 0,941 | 0,897 | 0,986 | 0,946 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, SIGLEC1, ISG15, HERC6, SLC1A2, OLAH, FAM20A, MMP8 | 0,873 | 0,862 | 0,884 | 0,941 | 0,896 | 0,986 | 0,941 | 0,876 | 1 |
| RSAD2, IFI27, OAS1, IFIT1, IFI44L, SIGLEC1, HERC6, SLC1A2, OLAH, FAM20A, MMP8 | 0,873 | 0,861 | 0,884 | 0,939 | 0,892 | 0,986 | 0,942 | 0,879 | 1 |
| IFI27, OAS1, IFIT1, SIGLEC1, ISG15, HERC6, SLC1A2, OLAH, FAM20A, RETN, MMP8 | 0,873 | 0,862 | 0,884 | 0,942 | 0,898 | 0,987 | 0,943 | 0,878 | 1 |
| IFI27, OAS1, IFIT1, SIGLEC1, HERC6, SLC1A2, IL1R2, OLAH, FAM20A, RETN, MMP8 | 0,872 | 0,861 | 0,884 | 0,942 | 0,898 | 0,986 | 0,942 | 0,874 | 1 |
| IFI27, OAS1, IFI44L, SIGLEC1, ISG15, HERC6, SLC1A2, IL1R2, OLAH, FAM20A, MMP8 | 0,872 | 0,861 | 0,883 | 0,940 | 0,897 | 0,984 | 0,947 | 0,879 | 1 |
| RSAD2, IFI27, OAS1, IFIT1, IFI44L, SIGLEC1, ISG15, HERC6, IL1R2, FAM20A, MMP8 | 0,872 | 0,862 | 0,882 | 0,933 | 0,884 | 0,982 | 0,950 | 0,882 | 1 |
| RSAD2, IFI27, IFIT1, IFI44L, SIGLEC1, HERC6, SLC1A2, IL1R2, OLAH, FAM20A, MMP8 | 0,872 | 0,862 | 0,883 | 0,940 | 0,895 | 0,985 | 0,949 | 0,885 | 1 |
| RSAD2, IFI27, OAS1, SIGLEC1, ISG15, HERC6, SLC1A2, IL1R2, OLAH, FAM20A, MMP8 | 0,872 | 0,861 | 0,884 | 0,940 | 0,896 | 0,985 | 0,947 | 0,879 | 1 |
| IFI27, OAS1, IFIT1, IFI44L, SIGLEC1, ISG15, SLC1A2, IL1R2, OLAH, FAM20A, MMP8 | 0,872 | 0,861 | 0,883 | 0,942 | 0,898 | 0,985 | 0,948 | 0,882 | 1 |
| RSAD2, IFI27, IFIT1, IFI44L, SIGLEC1, ISG15, HERC6, IL1R2, OLAH, FAM20A, RETN | 0,872 | 0,862 | 0,882 | 0,933 | 0,880 | 0,986 | 0,943 | 0,879 | 1 |
| RSAD2, IFI27, OAS1, IFIT1, IFI44L, SIGLEC1, ISG15, SLC1A2, OLAH, FAM20A, MMP8 | 0,872 | 0,861 | 0,882 | 0,939 | 0,891 | 0,987 | 0,945 | 0,880 | 1 |
| RSAD2, IFI27, OAS1, IFIT1, IFI44L, HERC6, IL1R2, OLAH, FAM20A, RETN, MMP8 | 0,871 | 0,860 | 0,882 | 0,933 | 0,880 | 0,986 | 0,930 | 0,857 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, HERC6, SLC1A2, OLAH, FAM20A, MMP8 | 0,871 | 0,860 | 0,883 | 0,942 | 0,897 | 0,987 | 0,938 | 0,871 | 1 |
| IFI27, OAS1, IFIT1, IFI44L, SIGLEC1, HERC6, SLC1A2, OLAH, FAM20A, RETN, MMP8 | 0,871 | 0,859 | 0,883 | 0,940 | 0,894 | 0,986 | 0,943 | 0,878 | 1 |
| RSAD2, IFI27, OAS1, IFI44L, SIGLEC1, ISG15, IL1R2, OLAH, FAM20A, RETN, MMP8 | 0,871 | 0,860 | 0,882 | 0,936 | 0,884 | 0,989 | 0,950 | 0,887 | 1 |
| RSAD2, IFI27, OAS1, IFIT1, IFI44L, SIGLEC1, SLC1A2, IL1R2, OLAH, FAM20A, MMP8 | 0,871 | 0,861 | 0,882 | 0,939 | 0,893 | 0,985 | 0,946 | 0,879 | 1 |
| IFI27, OAS1, IFIT1, SIGLEC1, ISG15, SLC1A2, IL1R2, OLAH, FAM20A, RETN, MMP8 | 0,871 | 0,860 | 0,882 | 0,942 | 0,897 | 0,986 | 0,947 | 0,880 | 1 |
| RSAD2, IFI27, IFIT1, IFI44L, SIGLEC1, ISG15, HERC6, IL1R2, FAM20A, RETN, MMP8 | 0,871 | 0,860 | 0,882 | 0,938 | 0,888 | 0,988 | 0,950 | 0,880 | 1 |
| RSAD2, IFI27, IFIT1, IFI44L, SIGLEC1, ISG15, SLC1A2, IL1R2, OLAH, FAM20A, MMP8 | 0,871 | 0,860 | 0,881 | 0,943 | 0,900 | 0,986 | 0,949 | 0,883 | 1 |
| RSAD2, IFI27, OAS1, IFIT1, SIGLEC1, HERC6, SLC1A2, OLAH, FAM20A, RETN, MMP8 | 0,871 | 0,859 | 0,882 | 0,940 | 0,894 | 0,986 | 0,942 | 0,875 | 1 |
| RSAD2, IFI27, IFIT1, IFI44L, SIGLEC1, ISG15, IL1R2, OLAH, FAM20A, RETN, MMP8 | 0,871 | 0,859 | 0,882 | 0,936 | 0,884 | 0,988 | 0,951 | 0,889 | 1 |
| RSAD2, IFI27, OAS1, SIGLEC1, HERC6, SLC1A2, IL1R2, OLAH, FAM20A, RETN, MMP8 | 0,871 | 0,859 | 0,882 | 0,940 | 0,894 | 0,986 | 0,945 | 0,878 | 1 |
| RSAD2, IFI27, OAS1, IFIT1, IFI44L, SIGLEC1, IL1R2, OLAH, FAM20A, RETN, MMP8 | 0,870 | 0,858 | 0,882 | 0,935 | 0,882 | 0,988 | 0,952 | 0,893 | 1 |
| RSAD2, IFI27, OAS1, IFIT1, SIGLEC1, ISG15, HERC6, SLC1A2, IL1R2, OLAH, FAM20A | 0,870 | 0,860 | 0,881 | 0,939 | 0,893 | 0,984 | 0,934 | 0,864 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFI44L, SIGLEC1, ISG15, HERC6, IL1R2, OLAH, FAM20A, RETN, MMP8 | 0,870 | 0,858 | 0,882 | 0,938 | 0,888 | 0,989 | 0,949 | 0,885 | 1 |
| RSAD2, IFI27, IFI44L, SIGLEC1, ISG15, HERC6, SLC1A2, IL1R2, OLAH, FAM20A, MMP8 | 0,870 | 0,859 | 0,882 | 0,942 | 0,898 | 0,985 | 0,950 | 0,885 | 1 |
| RSAD2, IFI27, OAS1, IFIT1, IFI44L, ISG15, HERC6, IL1R2, OLAH, FAM20A, MMP8 | 0,870 | 0,859 | 0,882 | 0,932 | 0,881 | 0,983 | 0,932 | 0,861 | 1 |
| RSAD2, IFI27, IFIT1, IFI44L, SIGLEC1, ISG15, HERC6, OLAH, FAM20A, RETN, MMP8 | 0,870 | 0,859 | 0,882 | 0,935 | 0,882 | 0,988 | 0,950 | 0,888 | 1 |
| IFI27, OAS1, IFIT1, SIGLEC1, ISG15, HERC6, SLC1A2, IL1R2, FAM20A, RETN, MMP8 | 0,870 | 0,859 | 0,881 | 0,940 | 0,897 | 0,983 | 0,949 | 0,877 | 1 |
| IFI27, OAS1, IFIT1, IFI44L, ISG15, HERC6, IL1R2, OLAH, FAM20A, RETN, MMP8 | 0,870 | 0,859 | 0,881 | 0,932 | 0,878 | 0,985 | 0,928 | 0,853 | 1 |
| RSAD2, IFI27, OAS1, IFI44L, SIGLEC1, ISG15, HERC6, IL1R2, OLAH, FAM20A, RETN | 0,870 | 0,859 | 0,881 | 0,933 | 0,880 | 0,985 | 0,942 | 0,876 | 1 |
| IFI27, OAS1, IFIT1, IFI44L, SIGLEC1, ISG15, HERC6, SLC1A2, IL1R2, OLAH, FAM20A | 0,870 | 0,860 | 0,880 | 0,940 | 0,895 | 0,984 | 0,932 | 0,861 | 1 |
| IFI27, IFIT1, IFI44L, SIGLEC1, ISG15, SLC1A2, IL1R2, OLAH, FAM20A, RETN, MMP8 | 0,870 | 0,859 | 0,881 | 0,944 | 0,901 | 0,987 | 0,948 | 0,880 | 1 |
| RSAD2, IFI27, OAS1, IFIT1, ISG15, HERC6, IL1R2, OLAH, FAM20A, RETN, MMP8 | 0,870 | 0,859 | 0,881 | 0,933 | 0,880 | 0,986 | 0,930 | 0,858 | 1 |
| IFI27, OAS1, IFI44L, SIGLEC1, HERC6, SLC1A2, IL1R2, OLAH, FAM20A, RETN, MMP8 | 0,870 | 0,859 | 0,881 | 0,939 | 0,893 | 0,985 | 0,948 | 0,882 | 1 |
| RSAD2, IFI27, IFIT1, SIGLEC1, ISG15, SLC1A2, IL1R2, OLAH, FAM20A, RETN, MMP8 | 0,870 | 0,859 | 0,881 | 0,943 | 0,900 | 0,986 | 0,947 | 0,879 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, HERC6, FAM20A, RETN, MMP8 | 0,870 | 0,859 | 0,881 | 0,933 | 0,879 | 0,986 | 0,942 | 0,873 | 1 |
| RSAD2, IFI27, OAS1, IFIT1, IFI44L, SIGLEC1, ISG15, HERC6, SLC1A2, OLAH, FAM20A | 0,870 | 0,859 | 0,880 | 0,937 | 0,890 | 0,984 | 0,934 | 0,864 | 1 |
| IFI27, IFI44L, SIGLEC1, ISG15, HERC6, SLC1A2, IL1R2, OLAH, FAM20A, RETN, MMP8 | 0,870 | 0,859 | 0,880 | 0,941 | 0,897 | 0,985 | 0,947 | 0,877 | 1 |
| RSAD2, IFI27, OAS1, IFI44L, SIGLEC1, ISG15, HERC6, OLAH, FAM20A, RETN, MMP8 | 0,870 | 0,858 | 0,881 | 0,935 | 0,882 | 0,989 | 0,947 | 0,883 | 1 |
| RSAD2, IFI27, IFIT1, SIGLEC1, ISG15, HERC6, SLC1A2, OLAH, FAM20A, RETN, MMP8 | 0,870 | 0,859 | 0,880 | 0,943 | 0,899 | 0,987 | 0,948 | 0,884 | 1 |
| RSAD2, IFI27, IFIT1, IFI44L, SIGLEC1, ISG15, HERC6, SLC1A2, OLAH, FAM20A, MMP8 | 0,869 | 0,858 | 0,881 | 0,941 | 0,895 | 0,987 | 0,943 | 0,880 | 1 |
| RSAD2, IFI27, SIGLEC1, ISG15, HERC6, SLC1A2, IL1R2, OLAH, FAM20A, RETN, MMP8 | 0,869 | 0,858 | 0,881 | 0,944 | 0,902 | 0,987 | 0,948 | 0,881 | 1 |
| RSAD2, IFI27, OAS1, IFI44L, SIGLEC1, ISG15, SLC1A2, IL1R2, OLAH, FAM20A, MMP8 | 0,869 | 0,858 | 0,881 | 0,940 | 0,897 | 0,984 | 0,947 | 0,879 | 1 |
| IFI27, OAS1, IFI44L, SIGLEC1, ISG15, HERC6, SLC1A2, OLAH, FAM20A, RETN, MMP8 | 0,869 | 0,858 | 0,881 | 0,940 | 0,894 | 0,986 | 0,943 | 0,877 | 1 |
| RSAD2, IFI27, OAS1, IFIT1, IFI44L, SIGLEC1, ISG15, HERC6, IL1R2, FAM20A, RETN | 0,869 | 0,859 | 0,879 | 0,935 | 0,886 | 0,984 | 0,946 | 0,879 | 1 |
| IFI27, OAS1, IFIT1, IFI44L, SIGLEC1, HERC6, SLC1A2, IL1R2, OLAH, FAM20A, RETN | 0,869 | 0,859 | 0,880 | 0,937 | 0,891 | 0,984 | 0,936 | 0,869 | 1 |
| RSAD2, IFI27, OAS1, IFI44L, SIGLEC1, ISG15, HERC6, IL1R2, FAM20A, RETN, MMP8 | 0,869 | 0,858 | 0,881 | 0,938 | 0,887 | 0,988 | 0,948 | 0,877 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, IFIT1, SIGLEC1, ISG15, HERC6, SLC1A2, IL1R2, OLAH, FAM20A, RETN, MMP8 | 0,869 | 0,858 | 0,880 | 0,943 | 0,899 | 0,986 | 0,949 | 0,881 | 1 |
| IFI27, OAS1, IFI44L, SIGLEC1, ISG15, SLC1A2, IL1R2, OLAH, FAM20A, RETN, MMP8 | 0,869 | 0,858 | 0,880 | 0,941 | 0,898 | 0,985 | 0,945 | 0,875 | 1 |
| RSAD2, IFI27, OAS1, IFIT1, IFI44L, ISG15, HERC6, OLAH, FAM20A, RETN, MMP8 | 0,869 | 0,857 | 0,881 | 0,931 | 0,877 | 0,986 | 0,928 | 0,855 | 1 |
| RSAD2, IFI27, IFIT1, SIGLEC1, HERC6, SLC1A2, IL1R2, OLAH, FAM20A, RETN, MMP8 | 0,869 | 0,858 | 0,880 | 0,940 | 0,895 | 0,986 | 0,950 | 0,886 | 1 |
| RSAD2, IFI27, OAS1, IFI44L, ISG15, HERC6, IL1R2, OLAH, FAM20A, RETN, MMP8 | 0,869 | 0,857 | 0,881 | 0,933 | 0,881 | 0,985 | 0,933 | 0,861 | 1 |
| IFI27, OAS1, IFIT1, IFI44L, SIGLEC1, ISG15, HERC6, SLC1A2, OLAH, FAM20A, RETN | 0,869 | 0,858 | 0,879 | 0,939 | 0,895 | 0,984 | 0,932 | 0,861 | 1 |
| RSAD2, IFI27, OAS1, IFI44L, SIGLEC1, HERC6, SLC1A2, IL1R2, OLAH, FAM20A, MMP8 | 0,869 | 0,857 | 0,881 | 0,937 | 0,891 | 0,984 | 0,945 | 0,878 | 1 |
| RSAD2, IFI27, OAS1, IFI44L, SIGLEC1, ISG15, HERC6, SLC1A2, OLAH, FAM20A, MMP8 | 0,869 | 0,857 | 0,880 | 0,937 | 0,889 | 0,985 | 0,942 | 0,877 | 1 |
| IFI27, OAS1, IFIT1, SIGLEC1, ISG15, HERC6, SLC1A2, IL1R2, OLAH, FAM20A, RETN | 0,869 | 0,858 | 0,880 | 0,940 | 0,895 | 0,984 | 0,932 | 0,861 | 1 |
| IFI27, IFIT1, IFI44L, SIGLEC1, ISG15, HERC6, SLC1A2, OLAH, FAM20A, RETN, MMP8 | 0,869 | 0,857 | 0,880 | 0,941 | 0,895 | 0,986 | 0,950 | 0,886 | 1 |
| RSAD2, IFI27, OAS1, IFIT1, IFI44L, SIGLEC1, HERC6, SLC1A2, IL1R2, FAM20A, MMP8 | 0,869 | 0,858 | 0,880 | 0,936 | 0,892 | 0,980 | 0,950 | 0,882 | 1 |
| RSAD2, IFI27, OAS1, IFIT1, IFI44L, SIGLEC1, ISG15, IL1R2, OLAH, FAM20A, RETN | 0,869 | 0,858 | 0,879 | 0,929 | 0,874 | 0,984 | 0,947 | 0,886 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, HERC6, SLC1A2, IL1R2, OLAH, FAM20A | 0,869 | 0,858 | 0,879 | 0,936 | 0,890 | 0,983 | 0,938 | 0,871 | 1 |
| RSAD2, IFI27, IFIT1, IFI44L, SIGLEC1, HERC6, SLC1A2, IL1R2, FAM20A, RETN, MMP8 | 0,868 | 0,858 | 0,879 | 0,940 | 0,894 | 0,986 | 0,950 | 0,881 | 1 |
| RSAD2, IFI27, OAS1, SIGLEC1, ISG15, SLC1A2, IL1R2, OLAH, FAM20A, RETN, MMP8 | 0,868 | 0,857 | 0,880 | 0,942 | 0,898 | 0,986 | 0,945 | 0,875 | 1 |
| RSAD2, IFI27, OAS1, IFIT1, SIGLEC1, ISG15, HERC6, SLC1A2, IL1R2, FAM20A, MMP8 | 0,868 | 0,858 | 0,879 | 0,937 | 0,894 | 0,980 | 0,951 | 0,882 | 1 |
| RSAD2, IFI27, IFIT1, SIGLEC1, ISG15, HERC6, SLC1A2, IL1R2, FAM20A, RETN, MMP8 | 0,868 | 0,858 | 0,879 | 0,939 | 0,894 | 0,985 | 0,951 | 0,882 | 1 |
| IFI27, IFIT1, IFI44L, SIGLEC1, ISG15, HERC6, SLC1A2, IL1R2, FAM20A, RETN, MMP8 | 0,868 | 0,858 | 0,879 | 0,939 | 0,895 | 0,984 | 0,952 | 0,883 | 1 |
| IFI27, OAS1, IFI44L, SIGLEC1, ISG15, HERC6, SLC1A2, IL1R2, FAM20A, RETN, MMP8 | 0,868 | 0,857 | 0,879 | 0,938 | 0,893 | 0,984 | 0,947 | 0,874 | 1 |
| RSAD2, IFI27, OAS1, IFIT1, IFI44L, SIGLEC1, ISG15, OLAH, FAM20A, RETN, MMP8 | 0,868 | 0,856 | 0,880 | 0,931 | 0,875 | 0,987 | 0,952 | 0,895 | 1 |
| RSAD2, IFI27, OAS1, IFIT1, SIGLEC1, HERC6, SLC1A2, IL1R2, FAM20A, RETN, MMP8 | 0,868 | 0,857 | 0,879 | 0,941 | 0,897 | 0,984 | 0,946 | 0,872 | 1 |
| RSAD2, IFI27, IFIT1, IFI44L, SIGLEC1, ISG15, HERC6, SLC1A2, IL1R2, OLAH, FAM20A | 0,868 | 0,858 | 0,878 | 0,939 | 0,895 | 0,984 | 0,934 | 0,865 | 1 |
| IFI27, OAS1, IFIT1, IFI44L, SIGLEC1, ISG15, HERC6, SLC1A2, IL1R2, FAM20A, MMP8 | 0,868 | 0,856 | 0,880 | 0,938 | 0,895 | 0,981 | 0,951 | 0,882 | 1 |
| IFI27, IFIT1, IFI44L, SIGLEC1, HERC6, SLC1A2, IL1R2, OLAH, FAM20A, RETN, MMP8 | 0,868 | 0,857 | 0,879 | 0,940 | 0,894 | 0,986 | 0,953 | 0,888 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, HERC6, SLC1A2, OLAH, FAM20A, RETN, MMP8 | 0,868 | 0,857 | 0,879 | 0,938 | 0,890 | 0,986 | 0,947 | 0,883 | 1 |
| RSAD2, IFI27, OAS1, SIGLEC1, ISG15, HERC6, SLC1A2, OLAH, FAM20A, RETN, MMP8 | 0,868 | 0,856 | 0,880 | 0,942 | 0,896 | 0,987 | 0,942 | 0,876 | 1 |
| RSAD2, IFI27, IFIT1, IFI44L, SIGLEC1, ISG15, HERC6, SLC1A2, IL1R2, FAM20A, MMP8 | 0,868 | 0,858 | 0,878 | 0,938 | 0,895 | 0,981 | 0,952 | 0,883 | 1 |
| IFI27, OAS1, IFIT1, IFI44L, SIGLEC1, ISG15, SLC1A2, IL1R2, FAM20A, RETN, MMP8 | 0,868 | 0,857 | 0,879 | 0,939 | 0,895 | 0,982 | 0,949 | 0,879 | 1 |
| RSAD2, IFI27, OAS1, IFIT1, IFI44L, SIGLEC1, HERC6, SLC1A2, OLAH, FAM20A, RETN | 0,868 | 0,857 | 0,879 | 0,936 | 0,889 | 0,983 | 0,937 | 0,870 | 1 |
| RSAD2, IFI27, OAS1, IFIT1, SIGLEC1, HERC6, SLC1A2, IL1R2, OLAH, FAM20A, RETN | 0,868 | 0,857 | 0,878 | 0,938 | 0,892 | 0,984 | 0,935 | 0,866 | 1 |
| IFI27, OAS1, IFIT1, IFI44L, SIGLEC1, ISG15, SLC1A2, OLAH, FAM20A, RETN, MMP8 | 0,868 | 0,856 | 0,880 | 0,937 | 0,888 | 0,986 | 0,947 | 0,883 | 1 |
| RSAD2, IFI27, OAS1, IFIT1, SIGLEC1, ISG15, HERC6, SLC1A2, OLAH, FAM20A, RETN | 0,868 | 0,857 | 0,878 | 0,938 | 0,892 | 0,984 | 0,936 | 0,867 | 1 |
| RSAD2, IFI27, OAS1, IFI44L, SIGLEC1, ISG15, HERC6, SLC1A2, IL1R2, OLAH, FAM20A | 0,868 | 0,857 | 0,878 | 0,938 | 0,892 | 0,983 | 0,938 | 0,869 | 1 |
| RSAD2, IFI27, OAS1, IFIT1, SIGLEC1, SLC1A2, IL1R2, OLAH, FAM20A, RETN, MMP8 | 0,867 | 0,856 | 0,879 | 0,937 | 0,889 | 0,984 | 0,951 | 0,888 | 1 |
| RSAD2, IFI27, OAS1, IFIT1, SIGLEC1, ISG15, SLC1A2, OLAH, FAM20A, RETN, MMP8 | 0,867 | 0,856 | 0,879 | 0,937 | 0,887 | 0,986 | 0,947 | 0,883 | 1 |
| RSAD2, IFI27, OAS1, IFIT1, IFI44L, SIGLEC1, ISG15, SLC1A2, IL1R2, OLAH, FAM20A | 0,867 | 0,857 | 0,877 | 0,936 | 0,888 | 0,983 | 0,941 | 0,875 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, HERC6, SLC1A2, OLAH, FAM20A, RETN, MMP8 | 0,867 | 0,855 | 0,879 | 0,941 | 0,894 | 0,987 | 0,949 | 0,887 | 1 |
| RSAD2, IFI27, OAS1, IFI44L, SIGLEC1, ISG15, HERC6, SLC1A2, IL1R2, FAM20A, MMP8 | 0,867 | 0,857 | 0,878 | 0,938 | 0,895 | 0,981 | 0,949 | 0,879 | 1 |
| RSAD2, IFI27, IFIT1, IFI44L, ISG15, HERC6, IL1R2, OLAH, FAM20A, RETN, MMP8 | 0,867 | 0,855 | 0,879 | 0,933 | 0,880 | 0,986 | 0,932 | 0,859 | 1 |
| RSAD2, IFI27, IFI44L, SIGLEC1, HERC6, SLC1A2, IL1R2, OLAH, FAM20A, RETN, MMP8 | 0,867 | 0,855 | 0,879 | 0,939 | 0,893 | 0,986 | 0,948 | 0,882 | 1 |
| RSAD2, IFI27, OAS1, IFIT1, IFI44L, SIGLEC1, HERC6, IL1R2, OLAH, RETN, MMP8 | 0,867 | 0,857 | 0,876 | 0,927 | 0,873 | 0,980 | 0,953 | 0,903 | 1 |
| RSAD2, IFI27, OAS1, IFIT1, HERC6, SLC1A2, IL1R2, OLAH, FAM20A, RETN, MMP8 | 0,867 | 0,856 | 0,877 | 0,935 | 0,887 | 0,983 | 0,921 | 0,839 | 1 |
| IFI27, OAS1, IFIT1, IFI44L, SIGLEC1, ISG15, HERC6, SLC1A2, FAM20A, RETN, MMP8 | 0,867 | 0,856 | 0,878 | 0,935 | 0,885 | 0,984 | 0,941 | 0,872 | 1 |
| RSAD2, IFI27, IFIT1, IFI44L, SIGLEC1, HERC6, SLC1A2, IL1R2, OLAH, FAM20A, RETN | 0,867 | 0,856 | 0,878 | 0,938 | 0,892 | 0,985 | 0,939 | 0,873 | 1 |
| IFI27, OAS1, IFIT1, IFI44L, SIGLEC1, ISG15, HERC6, SLC1A2, IL1R2, FAM20A, RETN | 0,867 | 0,856 | 0,877 | 0,936 | 0,891 | 0,981 | 0,937 | 0,865 | 1 |
| RSAD2, IFI27, OAS1, SIGLEC1, ISG15, HERC6, SLC1A2, IL1R2, FAM20A, RETN, MMP8 | 0,867 | 0,856 | 0,878 | 0,939 | 0,894 | 0,983 | 0,949 | 0,878 | 1 |
| RSAD2, IFI27, OAS1, IFIT1, SIGLEC1, ISG15, SLC1A2, IL1R2, OLAH, FAM20A, RETN | 0,867 | 0,856 | 0,877 | 0,935 | 0,887 | 0,984 | 0,941 | 0,875 | 1 |
| IFI27, OAS1, IFI44L, SIGLEC1, ISG15, HERC6, SLC1A2, IL1R2, OLAH, FAM20A, RETN | 0,867 | 0,856 | 0,877 | 0,936 | 0,890 | 0,982 | 0,936 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, IL1R2, FAM20A, RETN, MMP8 | 0,867 | 0,855 | 0,878 | 0,935 | 0,884 | 0,986 | 0,952 | 0,885 | 1 |
| IFI27, IFIT1, IFI44L, SIGLEC1, ISG15, HERC6, SLC1A2, IL1R2, OLAH, FAM20A, RETN | 0,867 | 0,856 | 0,877 | 0,939 | 0,895 | 0,984 | 0,938 | 0,872 | 1 |
| RSAD2, IFI27, IFIT1, IFI44L, SIGLEC1, ISG15, SLC1A2, OLAH, FAM20A, RETN, MMP8 | 0,866 | 0,854 | 0,878 | 0,938 | 0,889 | 0,986 | 0,948 | 0,885 | 1 |
| RSAD2, IFI27, OAS1, IFIT1, IFI44L, SIGLEC1, ISG15, HERC6, SLC1A2, IL1R2, FAM20A | 0,866 | 0,857 | 0,876 | 0,938 | 0,895 | 0,980 | 0,943 | 0,874 | 1 |
| RSAD2, IFI27, OAS1, IFIT1, IFI44L, SIGLEC1, ISG15, SLC1A2, IL1R2, FAM20A, MMP8 | 0,866 | 0,856 | 0,877 | 0,937 | 0,895 | 0,980 | 0,951 | 0,882 | 1 |
| RSAD2, IFI27, IFIT1, ISG15, HERC6, SLC1A2, IL1R2, OLAH, FAM20A, RETN, MMP8 | 0,866 | 0,856 | 0,876 | 0,933 | 0,885 | 0,982 | 0,923 | 0,843 | 1 |
| IFI27, OAS1, IFI44L, ISG15, HERC6, SLC1A2, IL1R2, OLAH, FAM20A, RETN, MMP8 | 0,866 | 0,855 | 0,877 | 0,936 | 0,889 | 0,984 | 0,924 | 0,843 | 1 |
| RSAD2, IFI27, OAS1, IFIT1, IFI44L, ISG15, HERC6, IL1R2, OLAH, FAM20A, RETN | 0,866 | 0,855 | 0,877 | 0,931 | 0,879 | 0,983 | 0,926 | 0,852 | 1 |
| IFI27, OAS1, IFIT1, IFI44L, ISG15, HERC6, SLC1A2, OLAH, FAM20A, RETN, MMP8 | 0,866 | 0,855 | 0,878 | 0,934 | 0,885 | 0,984 | 0,922 | 0,840 | 1 |
| RSAD2, IFI27, OAS1, IFIT1, IFI44L, HERC6, SLC1A2, IL1R2, OLAH, FAM20A, MMP8 | 0,866 | 0,855 | 0,878 | 0,934 | 0,886 | 0,983 | 0,924 | 0,844 | 1 |
| IFI27, OAS1, IFIT1, IFI44L, SIGLEC1, ISG15, SLC1A2, IL1R2, OLAH, FAM20A, RETN | 0,866 | 0,855 | 0,877 | 0,935 | 0,886 | 0,985 | 0,936 | 0,867 | 1 |
| RSAD2, IFI27, OAS1, IFI44L, SIGLEC1, HERC6, SLC1A2, IL1R2, FAM20A, RETN, MMP8 | 0,866 | 0,855 | 0,877 | 0,939 | 0,894 | 0,984 | 0,947 | 0,876 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, ISG15, HERC6, SLC1A2, IL1R2, OLAH, FAM20A, MMP8 | 0,866 | 0,855 | 0,877 | 0,934 | 0,886 | 0,981 | 0,922 | 0,840 | 1 |
| RSAD2, IFI27, IFIT1, IFI44L, SIGLEC1, ISG15, HERC6, SLC1A2, OLAH, FAM20A, RETN | 0,866 | 0,855 | 0,877 | 0,938 | 0,893 | 0,984 | 0,935 | 0,867 | 1 |
| RSAD2, IFI27, IFIT1, IFI44L, SIGLEC1, ISG15, SLC1A2, IL1R2, FAM20A, RETN, MMP8 | 0,866 | 0,856 | 0,876 | 0,939 | 0,894 | 0,984 | 0,949 | 0,879 | 1 |
| IFI27, OAS1, IFIT1, ISG15, HERC6, SLC1A2, IL1R2, OLAH, FAM20A, RETN, MMP8 | 0,866 | 0,855 | 0,877 | 0,933 | 0,885 | 0,982 | 0,921 | 0,839 | 1 |
| RSAD2, IFI27, OAS1, IFIT1, IFI44L, SIGLEC1, SLC1A2, OLAH, FAM20A, RETN, MMP8 | 0,866 | 0,854 | 0,878 | 0,934 | 0,883 | 0,985 | 0,950 | 0,889 | 1 |
| RSAD2, IFI27, OAS1, IFIT1, IFI44L, ISG15, IL1R2, OLAH, FAM20A, RETN, MMP8 | 0,866 | 0,854 | 0,878 | 0,927 | 0,871 | 0,982 | 0,934 | 0,865 | 1 |
| RSAD2, IFI27, IFIT1, SIGLEC1, ISG15, HERC6, SLC1A2, IL1R2, OLAH, FAM20A, RETN | 0,866 | 0,855 | 0,877 | 0,939 | 0,894 | 0,984 | 0,934 | 0,866 | 1 |
| RSAD2, IFI27, OAS1, IFI44L, ISG15, HERC6, SLC1A2, IL1R2, OLAH, FAM20A, MMP8 | 0,866 | 0,854 | 0,878 | 0,937 | 0,891 | 0,984 | 0,922 | 0,841 | 1 |
| RSAD2, IFI27, OAS1, IFIT1, SIGLEC1, ISG15, HERC6, IL1R2, OLAH, RETN, MMP8 | 0,866 | 0,856 | 0,876 | 0,927 | 0,874 | 0,981 | 0,958 | 0,909 | 1 |
| RSAD2, IFI27, OAS1, IFIT1, IFI44L, ISG15, HERC6, SLC1A2, OLAH, FAM20A, MMP8 | 0,866 | 0,854 | 0,878 | 0,931 | 0,881 | 0,982 | 0,923 | 0,844 | 1 |
| RSAD2, IFI27, OAS1, IFIT1, IFI44L, SIGLEC1, ISG15, HERC6, OLAH, RETN, MMP8 | 0,866 | 0,856 | 0,875 | 0,927 | 0,874 | 0,981 | 0,950 | 0,895 | 1 |
| IFI27, OAS1, IFIT1, IFI44L, HERC6, SLC1A2, IL1R2, OLAH, FAM20A, RETN, MMP8 | 0,866 | 0,855 | 0,877 | 0,937 | 0,889 | 0,984 | 0,923 | 0,841 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, SLC1A2, OLAH, FAM20A, RETN | 0,866 | 0,855 | 0,876 | 0,933 | 0,881 | 0,984 | 0,938 | 0,872 | 1 |
| RSAD2, IFI27, OAS1, IFIT1, SIGLEC1, ISG15, HERC6, SLC1A2, FAM20A, RETN, MMP8 | 0,866 | 0,856 | 0,876 | 0,935 | 0,885 | 0,985 | 0,940 | 0,869 | 1 |
| RSAD2, IFI27, IFI44L, SIGLEC1, ISG15, SLC1A2, IL1R2, OLAH, FAM20A, RETN, MMP8 | 0,866 | 0,853 | 0,878 | 0,944 | 0,901 | 0,986 | 0,946 | 0,878 | 1 |
| RSAD2, IFI27, OAS1, IFIT1, ISG15, HERC6, SLC1A2, OLAH, FAM20A, RETN, MMP8 | 0,866 | 0,855 | 0,876 | 0,933 | 0,883 | 0,983 | 0,923 | 0,842 | 1 |
| IFI27, OAS1, IFIT1, IFI44L, SIGLEC1, ISG15, HERC6, IL1R2, OLAH, RETN, MMP8 | 0,866 | 0,856 | 0,875 | 0,927 | 0,875 | 0,980 | 0,952 | 0,901 | 1 |
| RSAD2, IFI27, OAS1, IFI44L, SIGLEC1, ISG15, HERC6, SLC1A2, OLAH, FAM20A, RETN | 0,866 | 0,855 | 0,876 | 0,936 | 0,890 | 0,983 | 0,937 | 0,866 | 1 |
| RSAD2, IFI27, OAS1, IFIT1, SIGLEC1, ISG15, SLC1A2, IL1R2, FAM20A, RETN, MMP8 | 0,865 | 0,855 | 0,876 | 0,938 | 0,894 | 0,982 | 0,949 | 0,879 | 1 |
| RSAD2, IFI27, OAS1, SIGLEC1, ISG15, HERC6, SLC1A2, IL1R2, OLAH, FAM20A, RETN | 0,865 | 0,854 | 0,876 | 0,936 | 0,889 | 0,983 | 0,935 | 0,864 | 1 |
| RSAD2, IFI27, IFI44L, SIGLEC1, ISG15, HERC6, SLC1A2, IL1R2, OLAH, FAM20A, RETN | 0,865 | 0,855 | 0,876 | 0,937 | 0,891 | 0,983 | 0,936 | 0,867 | 1 |
| RSAD2, IFI27, IFI44L, SIGLEC1, ISG15, HERC6, SLC1A2, OLAH, FAM20A, RETN, MMP8 | 0,865 | 0,853 | 0,877 | 0,942 | 0,896 | 0,988 | 0,943 | 0,876 | 1 |
| RSAD2, IFI27, OAS1, IFIT1, IFI44L, SIGLEC1, HERC6, SLC1A2, FAM20A, RETN, MMP8 | 0,865 | 0,854 | 0,876 | 0,934 | 0,885 | 0,984 | 0,941 | 0,872 | 1 |
| RSAD2, IFI27, IFIT1, IFI44L, SIGLEC1, ISG15, SLC1A2, IL1R2, OLAH, FAM20A, RETN | 0,865 | 0,855 | 0,875 | 0,937 | 0,889 | 0,985 | 0,936 | 0,868 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, ISG15, HERC6, SLC1A2, IL1R2, OLAH, FAM20A, MMP8 | 0,865 | 0,854 | 0,876 | 0,937 | 0,891 | 0,983 | 0,924 | 0,847 | 1 |
| RSAD2, IFI27, OAS1, IFIT1, IFI44L, SIGLEC1, ISG15, HERC6, IL1R2, OLAH, RETN | 0,865 | 0,856 | 0,874 | 0,926 | 0,872 | 0,979 | 0,941 | 0,882 | 1 |
| RSAD2, IFI27, OAS1, IFI44L, SIGLEC1, ISG15, HERC6, IL1R2, OLAH, RETN, MMP8 | 0,865 | 0,856 | 0,875 | 0,926 | 0,873 | 0,980 | 0,952 | 0,901 | 1 |
| RSAD2, IFI27, IFI44L, SIGLEC1, ISG15, HERC6, SLC1A2, IL1R2, FAM20A, RETN, MMP8 | 0,865 | 0,854 | 0,876 | 0,938 | 0,892 | 0,984 | 0,949 | 0,879 | 1 |
| RSAD2, IFI27, OAS1, IFIT1, SIGLEC1, ISG15, HERC6, SLC1A2, IL1R2, FAM20A, RETN | 0,865 | 0,854 | 0,875 | 0,937 | 0,893 | 0,981 | 0,937 | 0,864 | 1 |
| RSAD2, IFI27, OAS1, IFIT1, IFI44L, SIGLEC1, ISG15, HERC6, SLC1A2, FAM20A, RETN | 0,865 | 0,855 | 0,875 | 0,933 | 0,884 | 0,982 | 0,934 | 0,860 | 1 |
| RSAD2, IFI27, OAS1, IFI44L, SIGLEC1, SLC1A2, IL1R2, OLAH, FAM20A, RETN, MMP8 | 0,865 | 0,853 | 0,876 | 0,936 | 0,888 | 0,984 | 0,948 | 0,883 | 1 |
| RSAD2, IFI27, OAS1, IFIT1, IFI44L, ISG15, SLC1A2, IL1R2, OLAH, FAM20A, MMP8 | 0,865 | 0,854 | 0,876 | 0,934 | 0,885 | 0,982 | 0,925 | 0,848 | 1 |
| RSAD2, IFI27, OAS1, IFIT1, IFI44L, ISG15, HERC6, SLC1A2, IL1R2, OLAH, FAM20A | 0,865 | 0,854 | 0,875 | 0,935 | 0,887 | 0,982 | 0,920 | 0,837 | 1 |
| RSAD2, IFI27, OAS1, IFI44L, SIGLEC1, ISG15, SLC1A2, IL1R2, OLAH, FAM20A, RETN | 0,865 | 0,854 | 0,875 | 0,935 | 0,888 | 0,983 | 0,943 | 0,875 | 1 |
| RSAD2, IFI27, IFIT1, IFI44L, SIGLEC1, SLC1A2, IL1R2, OLAH, FAM20A, RETN, MMP8 | 0,865 | 0,853 | 0,877 | 0,938 | 0,892 | 0,985 | 0,950 | 0,886 | 1 |
| RSAD2, IFI27, OAS1, IFIT1, IFI44L, SIGLEC1, HERC6, SLC1A2, IL1R2, FAM20A, RETN | 0,865 | 0,854 | 0,876 | 0,936 | 0,891 | 0,981 | 0,939 | 0,866 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, ISG15, HERC6, SLC1A2, IL1R2, OLAH, FAM20A, MMP8 | 0,865 | 0,853 | 0,876 | 0,935 | 0,887 | 0,983 | 0,924 | 0,845 | 1 |
| RSAD2, IFI27, OAS1, IFIT1, IFI44L, SIGLEC1, SLC1A2, IL1R2, FAM20A, RETN, MMP8 | 0,865 | 0,853 | 0,876 | 0,938 | 0,894 | 0,983 | 0,948 | 0,876 | 1 |
| RSAD2, IFI27, OAS1, IFIT1, ISG15, SLC1A2, IL1R2, OLAH, FAM20A, RETN, MMP8 | 0,864 | 0,854 | 0,875 | 0,933 | 0,884 | 0,982 | 0,925 | 0,847 | 1 |
| RSAD2, IFI27, IFIT1, IFI44L, ISG15, SLC1A2, IL1R2, OLAH, FAM20A, RETN, MMP8 | 0,864 | 0,854 | 0,875 | 0,935 | 0,887 | 0,983 | 0,924 | 0,845 | 1 |
| IFI27, OAS1, IFIT1, IFI44L, SIGLEC1, SLC1A2, IL1R2, OLAH, FAM20A, RETN, MMP8 | 0,864 | 0,852 | 0,877 | 0,937 | 0,889 | 0,984 | 0,951 | 0,887 | 1 |
| RSAD2, IFI27, OAS1, IFIT1, IFI44L, HERC6, SLC1A2, OLAH, FAM20A, RETN, MMP8 | 0,864 | 0,852 | 0,876 | 0,936 | 0,888 | 0,985 | 0,923 | 0,842 | 1 |
| RSAD2, IFI27, OAS1, IFI44L, SIGLEC1, ISG15, SLC1A2, IL1R2, FAM20A, RETN, MMP8 | 0,864 | 0,853 | 0,875 | 0,939 | 0,894 | 0,983 | 0,947 | 0,876 | 1 |
| RSAD2, IFI27, IFI44L, ISG15, HERC6, SLC1A2, IL1R2, OLAH, FAM20A, RETN, MMP8 | 0,864 | 0,852 | 0,875 | 0,937 | 0,889 | 0,984 | 0,922 | 0,841 | 1 |
| RSAD2, IFI27, IFIT1, IFI44L, HERC6, SLC1A2, IL1R2, OLAH, FAM20A, RETN, MMP8 | 0,864 | 0,853 | 0,875 | 0,937 | 0,889 | 0,984 | 0,924 | 0,844 | 1 |
| RSAD2, IFI27, OAS1, IFIT1, IFI44L, SIGLEC1, SLC1A2, IL1R2, OLAH, FAM20A, RETN | 0,864 | 0,852 | 0,875 | 0,933 | 0,882 | 0,984 | 0,941 | 0,876 | 1 |
| RSAD2, IFI27, OAS1, ISG15, HERC6, SLC1A2, IL1R2, OLAH, FAM20A, RETN, MMP8 | 0,864 | 0,852 | 0,875 | 0,935 | 0,888 | 0,983 | 0,923 | 0,840 | 1 |
| RSAD2, IFI27, OAS1, IFI44L, ISG15, HERC6, SLC1A2, OLAH, FAM20A, RETN, MMP8 | 0,864 | 0,852 | 0,875 | 0,934 | 0,885 | 0,984 | 0,923 | 0,842 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, ISG15, HERC6, IL1R2, FAM20A, RETN, MMP8 | 0,863 | 0,852 | 0,875 | 0,932 | 0,880 | 0,984 | 0,928 | 0,847 | 1 |
| IFI27, OAS1, IFIT1, IFI44L, ISG15, SLC1A2, IL1R2, OLAH, FAM20A, RETN, MMP8 | 0,863 | 0,852 | 0,874 | 0,933 | 0,884 | 0,982 | 0,923 | 0,843 | 1 |
| RSAD2, IFI27, IFIT1, IFI44L, SIGLEC1, ISG15, HERC6, IL1R2, OLAH, RETN, MMP8 | 0,863 | 0,854 | 0,873 | 0,927 | 0,875 | 0,980 | 0,950 | 0,898 | 1 |
| RSAD2, IFI27, IFIT1, IFI44L, SIGLEC1, ISG15, HERC6, SLC1A2, FAM20A, RETN, MMP8 | 0,863 | 0,853 | 0,874 | 0,931 | 0,879 | 0,984 | 0,942 | 0,868 | 1 |
| IFI27, IFIT1, IFI44L, ISG15, HERC6, SLC1A2, IL1R2, OLAH, FAM20A, RETN, MMP8 | 0,863 | 0,852 | 0,874 | 0,934 | 0,887 | 0,982 | 0,923 | 0,843 | 1 |
| RSAD2, IFI27, OAS1, IFI44L, HERC6, SLC1A2, IL1R2, OLAH, FAM20A, RETN, MMP8 | 0,863 | 0,851 | 0,876 | 0,938 | 0,891 | 0,985 | 0,924 | 0,844 | 1 |
| RSAD2, IFI27, IFIT1, IFI44L, ISG15, HERC6, SLC1A2, OLAH, FAM20A, RETN, MMP8 | 0,863 | 0,852 | 0,875 | 0,936 | 0,888 | 0,985 | 0,923 | 0,843 | 1 |
| RSAD2, IFI27, OAS1, IFI44L, SIGLEC1, HERC6, SLC1A2, IL1R2, OLAH, FAM20A, RETN | 0,863 | 0,852 | 0,875 | 0,935 | 0,888 | 0,983 | 0,938 | 0,869 | 1 |
| RSAD2, IFI27, OAS1, IFI44L, SIGLEC1, ISG15, SLC1A2, OLAH, FAM20A, RETN, MMP8 | 0,863 | 0,851 | 0,876 | 0,936 | 0,886 | 0,985 | 0,947 | 0,885 | 1 |
| RSAD2, IFI27, IFIT1, IFI44L, SIGLEC1, ISG15, HERC6, SLC1A2, IL1R2, FAM20A, RETN | 0,863 | 0,853 | 0,873 | 0,934 | 0,888 | 0,980 | 0,946 | 0,874 | 1 |
| OAS1, IFIT1, IFI44L, SIGLEC1, ISG15, HERC6, IL1R2, OLAH, FAM20A, RETN, MMP8 | 0,863 | 0,853 | 0,873 | 0,924 | 0,869 | 0,980 | 0,963 | 0,909 | 1 |
| RSAD2, IFI27, OAS1, IFIT1, IFI44L, ISG15, HERC6, SLC1A2, OLAH, FAM20A, RETN | 0,863 | 0,852 | 0,874 | 0,934 | 0,886 | 0,983 | 0,920 | 0,835 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, HERC6, SLC1A2, IL1R2, OLAH, FAM20A, RETN | 0,863 | 0,852 | 0,873 | 0,934 | 0,886 | 0,982 | 0,922 | 0,839 | 1 |
| RSAD2, OAS1, IFIT1, SIGLEC1, ISG15, HERC6, IL1R2, OLAH, FAM20A, RETN, MMP8 | 0,863 | 0,853 | 0,872 | 0,923 | 0,867 | 0,979 | 0,963 | 0,909 | 1 |
| RSAD2, OAS1, IFIT1, IFI44L, SIGLEC1, ISG15, HERC6, IL1R2, OLAH, FAM20A, MMP8 | 0,862 | 0,852 | 0,873 | 0,923 | 0,868 | 0,977 | 0,963 | 0,909 | 1 |
| RSAD2, IFI27, OAS1, IFIT1, IFI44L, ISG15, HERC6, IL1R2, OLAH, RETN, MMP8 | 0,862 | 0,852 | 0,872 | 0,923 | 0,867 | 0,979 | 0,949 | 0,894 | 1 |
| RSAD2, IFI27, OAS1, IFIT1, SIGLEC1, HERC6, SLC1A2, IL1R2, OLAH, RETN, MMP8 | 0,862 | 0,852 | 0,872 | 0,932 | 0,883 | 0,982 | 0,950 | 0,892 | 1 |
| IFI27, OAS1, IFIT1, IFI44L, SIGLEC1, HERC6, SLC1A2, IL1R2, OLAH, RETN, MMP8 | 0,862 | 0,852 | 0,872 | 0,931 | 0,883 | 0,980 | 0,949 | 0,891 | 1 |
| RSAD2, IFI27, OAS1, IFIT1, IFI44L, SIGLEC1, ISG15, HERC6, IL1R2, OLAH, MMP8 | 0,862 | 0,853 | 0,871 | 0,927 | 0,879 | 0,976 | 0,948 | 0,898 | 0,998 |
| RSAD2, IFI27, OAS1, IFIT1, IFI44L, ISG15, SLC1A2, OLAH, FAM20A, RETN, MMP8 | 0,862 | 0,850 | 0,873 | 0,930 | 0,877 | 0,983 | 0,923 | 0,845 | 1 |
| IFI27, OAS1, IFIT1, IFI44L, ISG15, HERC6, SLC1A2, IL1R2, OLAH, FAM20A, RETN | 0,862 | 0,851 | 0,873 | 0,934 | 0,887 | 0,982 | 0,918 | 0,835 | 1 |
| RSAD2, IFI27, OAS1, IFI44L, SIGLEC1, ISG15, HERC6, SLC1A2, IL1R2, FAM20A, RETN | 0,862 | 0,851 | 0,873 | 0,935 | 0,890 | 0,980 | 0,939 | 0,864 | 1 |
| RSAD2, OAS1, IFIT1, IFI44L, SIGLEC1, HERC6, IL1R2, OLAH, FAM20A, RETN, MMP8 | 0,862 | 0,851 | 0,872 | 0,925 | 0,871 | 0,980 | 0,962 | 0,907 | 1 |
| RSAD2, IFI27, OAS1, IFI44L, ISG15, HERC6, SLC1A2, IL1R2, OLAH, FAM20A, RETN | 0,862 | 0,851 | 0,873 | 0,934 | 0,886 | 0,982 | 0,917 | 0,833 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, HERC6, IL1R2, RETN, MMP8 | 0,862 | 0,852 | 0,871 | 0,926 | 0,874 | 0,979 | 0,955 | 0,905 | 1 |
| RSAD2, IFI27, OAS1, IFIT1, IFI44L, SIGLEC1, HERC6, SLC1A2, IL1R2, OLAH, RETN | 0,862 | 0,852 | 0,871 | 0,931 | 0,882 | 0,981 | 0,945 | 0,882 | 1 |
| RSAD2, IFI27, OAS1, IFI44L, ISG15, SLC1A2, IL1R2, OLAH, FAM20A, RETN, MMP8 | 0,861 | 0,850 | 0,873 | 0,935 | 0,887 | 0,984 | 0,924 | 0,845 | 1 |
| RSAD2, IFI27, OAS1, IFIT1, IFI44L, SIGLEC1, ISG15, SLC1A2, IL1R2, FAM20A, RETN | 0,861 | 0,851 | 0,872 | 0,933 | 0,887 | 0,980 | 0,942 | 0,873 | 1 |
| IFI27, OAS1, IFIT1, SIGLEC1, ISG15, HERC6, SLC1A2, IL1R2, OLAH, RETN, MMP8 | 0,861 | 0,850 | 0,872 | 0,931 | 0,882 | 0,980 | 0,947 | 0,886 | 1 |
| RSAD2, IFI27, OAS1, IFIT1, ISG15, HERC6, SLC1A2, IL1R2, OLAH, FAM20A, RETN | 0,861 | 0,851 | 0,872 | 0,934 | 0,886 | 0,981 | 0,916 | 0,829 | 1 |
| RSAD2, IFI27, OAS1, IFIT1, IFI44L, SIGLEC1, HERC6, SLC1A2, OLAH, RETN, MMP8 | 0,861 | 0,851 | 0,871 | 0,932 | 0,883 | 0,981 | 0,949 | 0,890 | 1 |
| RSAD2, OAS1, IFIT1, IFI44L, SIGLEC1, ISG15, HERC6, IL1R2, FAM20A, RETN, MMP8 | 0,861 | 0,850 | 0,871 | 0,923 | 0,868 | 0,979 | 0,964 | 0,909 | 1 |
| RSAD2, IFI27, OAS1, IFIT1, SIGLEC1, ISG15, HERC6, SLC1A2, IL1R2, OLAH, RETN | 0,861 | 0,851 | 0,870 | 0,931 | 0,882 | 0,981 | 0,946 | 0,885 | 1 |
| RSAD2, IFI27, OAS1, IFIT1, IFI44L, SLC1A2, IL1R2, OLAH, FAM20A, RETN, MMP8 | 0,860 | 0,849 | 0,872 | 0,934 | 0,884 | 0,983 | 0,926 | 0,850 | 1 |
| RSAD2, IFI27, OAS1, IFI44L, SIGLEC1, ISG15, HERC6, SLC1A2, FAM20A, RETN, MMP8 | 0,860 | 0,849 | 0,872 | 0,931 | 0,877 | 0,984 | 0,942 | 0,868 | 1 |
| RSAD2, IFIT1, IFI44L, SIGLEC1, ISG15, HERC6, IL1R2, OLAH, FAM20A, RETN, MMP8 | 0,860 | 0,850 | 0,870 | 0,924 | 0,869 | 0,980 | 0,962 | 0,907 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, ISG15, HERC6, SLC1A2, IL1R2, OLAH, FAM20A, RETN | 0,860 | 0,849 | 0,871 | 0,936 | 0,888 | 0,983 | 0,918 | 0,837 | 1 |
| RSAD2, IFI27, OAS1, IFI44L, SIGLEC1, HERC6, SLC1A2, IL1R2, OLAH, RETN, MMP8 | 0,860 | 0,849 | 0,870 | 0,931 | 0,882 | 0,980 | 0,950 | 0,892 | 1 |
| RSAD2, OAS1, IFIT1, IFI44L, SIGLEC1, HERC6, SLC1A2, IL1R2, OLAH, FAM20A, MMP8 | 0,860 | 0,849 | 0,871 | 0,930 | 0,883 | 0,976 | 0,954 | 0,892 | 1 |
| RSAD2, IFI27, IFIT1, SIGLEC1, ISG15, HERC6, SLC1A2, IL1R2, OLAH, RETN, MMP8 | 0,860 | 0,850 | 0,870 | 0,931 | 0,883 | 0,980 | 0,950 | 0,897 | 1 |
| RSAD2, IFI27, OAS1, IFIT1, IFI44L, SIGLEC1, ISG15, IL1R2, OLAH, RETN, MMP8 | 0,860 | 0,850 | 0,869 | 0,924 | 0,870 | 0,978 | 0,952 | 0,897 | 1 |
| RSAD2, IFI27, OAS1, IFI44L, SIGLEC1, ISG15, HERC6, SLC1A2, IL1R2, OLAH, RETN | 0,860 | 0,850 | 0,869 | 0,929 | 0,879 | 0,979 | 0,943 | 0,881 | 1 |
| IFI27, OAS1, IFIT1, IFI44L, ISG15, HERC6, SLC1A2, IL1R2, FAM20A, RETN, MMP8 | 0,860 | 0,849 | 0,870 | 0,933 | 0,884 | 0,982 | 0,927 | 0,843 | 1 |
| IFI27, OAS1, IFIT1, IFI44L, SIGLEC1, ISG15, HERC6, SLC1A2, OLAH, RETN, MMP8 | 0,860 | 0,849 | 0,870 | 0,931 | 0,882 | 0,981 | 0,946 | 0,884 | 1 |
| IFI27, OAS1, IFI44L, SIGLEC1, ISG15, HERC6, SLC1A2, IL1R2, OLAH, RETN, MMP8 | 0,860 | 0,850 | 0,870 | 0,933 | 0,884 | 0,981 | 0,947 | 0,887 | 1 |
| RSAD2, IFIT1, IFI44L, SIGLEC1, ISG15, HERC6, SLC1A2, IL1R2, OLAH, FAM20A, MMP8 | 0,860 | 0,849 | 0,870 | 0,929 | 0,883 | 0,976 | 0,957 | 0,893 | 1 |
| OAS1, IFIT1, SIGLEC1, ISG15, HERC6, SLC1A2, IL1R2, OLAH, FAM20A, RETN, MMP8 | 0,859 | 0,849 | 0,869 | 0,929 | 0,882 | 0,976 | 0,957 | 0,891 | 1 |
| RSAD2, OAS1, IFIT1, SIGLEC1, ISG15, HERC6, SLC1A2, IL1R2, OLAH, FAM20A, MMP8 | 0,859 | 0,849 | 0,870 | 0,928 | 0,881 | 0,975 | 0,961 | 0,899 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, ISG15, SLC1A2, IL1R2, OLAH, FAM20A, RETN | 0,859 | 0,849 | 0,870 | 0,931 | 0,880 | 0,982 | 0,921 | 0,839 | 1 |
| IFI27, OAS1, IFIT1, IFI44L, SIGLEC1, ISG15, HERC6, SLC1A2, IL1R2, OLAH, RETN | 0,859 | 0,849 | 0,869 | 0,930 | 0,880 | 0,979 | 0,937 | 0,873 | 1 |
| RSAD2, IFI27, IFIT1, IFI44L, SIGLEC1, HERC6, SLC1A2, IL1R2, OLAH, RETN, MMP8 | 0,859 | 0,849 | 0,869 | 0,930 | 0,881 | 0,980 | 0,952 | 0,901 | 1 |
| OAS1, IFIT1, IFI44L, SIGLEC1, ISG15, HERC6, SLC1A2, IL1R2, OLAH, FAM20A, MMP8 | 0,859 | 0,848 | 0,870 | 0,929 | 0,882 | 0,976 | 0,954 | 0,890 | 1 |
| IFIT1, IFI44L, SIGLEC1, ISG15, HERC6, SLC1A2, IL1R2, OLAH, FAM20A, RETN, MMP8 | 0,859 | 0,849 | 0,869 | 0,930 | 0,884 | 0,977 | 0,959 | 0,899 | 1 |
| RSAD2, OAS1, IFIT1, IFI44L, SIGLEC1, ISG15, HERC6, OLAH, FAM20A, RETN, MMP8 | 0,859 | 0,849 | 0,869 | 0,922 | 0,865 | 0,979 | 0,951 | 0,893 | 1 |
| RSAD2, OAS1, IFIT1, IFI44L, SIGLEC1, ISG15, HERC6, SLC1A2, OLAH, FAM20A, MMP8 | 0,859 | 0,848 | 0,870 | 0,929 | 0,880 | 0,978 | 0,935 | 0,863 | 1 |
| OAS1, IFIT1, IFI44L, SIGLEC1, ISG15, HERC6, SLC1A2, OLAH, FAM20A, RETN, MMP8 | 0,859 | 0,849 | 0,869 | 0,930 | 0,881 | 0,979 | 0,940 | 0,873 | 1 |
| RSAD2, IFI27, OAS1, IFIT1, IFI44L, SIGLEC1, ISG15, HERC6, SLC1A2, FAM20A, MMP8 | 0,859 | 0,849 | 0,869 | 0,927 | 0,876 | 0,978 | 0,935 | 0,859 | 1 |
| RSAD2, IFI27, OAS1, IFIT1, IFI44L, HERC6, SLC1A2, IL1R2, OLAH, RETN, MMP8 | 0,859 | 0,849 | 0,869 | 0,927 | 0,876 | 0,979 | 0,938 | 0,872 | 1 |
| RSAD2, IFI27, OAS1, IFIT1, IFI44L, SIGLEC1, ISG15, HERC6, SLC1A2, OLAH, RETN | 0,859 | 0,849 | 0,869 | 0,931 | 0,881 | 0,981 | 0,945 | 0,883 | 1 |
| OAS1, IFIT1, IFI44L, SIGLEC1, ISG15, HERC6, SLC1A2, IL1R2, FAM20A, RETN, MMP8 | 0,859 | 0,849 | 0,869 | 0,929 | 0,882 | 0,975 | 0,958 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| OAS1, IFIT1, IFI44L, SIGLEC1, HERC6, SLC1A2, IL1R2, OLAH, FAM20A, RETN, MMP8 | 0,858 | 0,848 | 0,869 | 0,929 | 0,882 | 0,977 | 0,950 | 0,886 | 1 |
| RSAD2, IFI27, OAS1, SIGLEC1, ISG15, HERC6, SLC1A2, IL1R2, OLAH, RETN, MMP8 | 0,858 | 0,847 | 0,870 | 0,931 | 0,881 | 0,980 | 0,948 | 0,888 | 1 |
| RSAD2, IFI27, OAS1, IFIT1, ISG15, HERC6, SLC1A2, IL1R2, OLAH, RETN, MMP8 | 0,858 | 0,848 | 0,869 | 0,928 | 0,877 | 0,979 | 0,939 | 0,874 | 1 |
| IFI27, IFIT1, IFI44L, SIGLEC1, ISG15, HERC6, SLC1A2, IL1R2, OLAH, RETN, MMP8 | 0,858 | 0,848 | 0,868 | 0,930 | 0,882 | 0,979 | 0,949 | 0,896 | 1 |
| RSAD2, IFI27, OAS1, IFIT1, IFI44L, HERC6, SLC1A2, IL1R2, FAM20A, RETN, MMP8 | 0,858 | 0,848 | 0,869 | 0,932 | 0,883 | 0,981 | 0,927 | 0,843 | 1 |
| RSAD2, OAS1, IFIT1, SIGLEC1, HERC6, SLC1A2, IL1R2, OLAH, FAM20A, RETN, MMP8 | 0,858 | 0,848 | 0,869 | 0,929 | 0,882 | 0,976 | 0,959 | 0,895 | 1 |
| RSAD2, IFI27, OAS1, IFIT1, SIGLEC1, ISG15, HERC6, SLC1A2, OLAH, RETN, MMP8 | 0,858 | 0,847 | 0,869 | 0,932 | 0,882 | 0,981 | 0,949 | 0,890 | 1 |
| RSAD2, OAS1, IFIT1, IFI44L, SIGLEC1, ISG15, SLC1A2, IL1R2, OLAH, FAM20A, MMP8 | 0,858 | 0,848 | 0,868 | 0,927 | 0,879 | 0,975 | 0,960 | 0,902 | 1 |
| RSAD2, IFI27, OAS1, IFIT1, IFI44L, SIGLEC1, ISG15, HERC6, SLC1A2, IL1R2, OLAH | 0,858 | 0,848 | 0,868 | 0,929 | 0,881 | 0,978 | 0,946 | 0,890 | 1 |
| RSAD2, OAS1, IFIT1, SIGLEC1, ISG15, HERC6, SLC1A2, OLAH, FAM20A, RETN, MMP8 | 0,858 | 0,848 | 0,868 | 0,926 | 0,876 | 0,977 | 0,948 | 0,881 | 1 |
| RSAD2, OAS1, IFIT1, IFI44L, SIGLEC1, HERC6, SLC1A2, OLAH, FAM20A, RETN, MMP8 | 0,858 | 0,847 | 0,869 | 0,929 | 0,880 | 0,978 | 0,941 | 0,875 | 1 |
| RSAD2, IFI27, OAS1, IFI44L, SIGLEC1, ISG15, HERC6, SLC1A2, OLAH, RETN, MMP8 | 0,858 | 0,847 | 0,869 | 0,931 | 0,882 | 0,981 | 0,950 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFIT1, IFI44L, SIGLEC1, ISG15, HERC6, SLC1A2, OLAH, FAM20A, RETN, MMP8 | 0,858 | 0,848 | 0,868 | 0,930 | 0,881 | 0,978 | 0,938 | 0,868 | 1 |
| RSAD2, IFI27, OAS1, IFIT1, IFI44L, SIGLEC1, ISG15, SLC1A2, FAM20A, RETN, MMP8 | 0,858 | 0,847 | 0,869 | 0,927 | 0,872 | 0,982 | 0,941 | 0,867 | 1 |
| RSAD2, IFI27, OAS1, IFIT1, IFI44L, SIGLEC1, ISG15, HERC6, SLC1A2, RETN, MMP8 | 0,858 | 0,849 | 0,867 | 0,924 | 0,870 | 0,978 | 0,941 | 0,878 | 1 |
| RSAD2, IFIT1, SIGLEC1, ISG15, HERC6, SLC1A2, IL1R2, OLAH, FAM20A, RETN, MMP8 | 0,858 | 0,848 | 0,868 | 0,929 | 0,881 | 0,976 | 0,959 | 0,893 | 1 |
| RSAD2, IFI27, OAS1, IFIT1, IFI44L, SIGLEC1, HERC6, SLC1A2, IL1R2, OLAH, MMP8 | 0,858 | 0,848 | 0,868 | 0,928 | 0,880 | 0,976 | 0,950 | 0,897 | 1 |
| RSAD2, IFI27, OAS1, IFIT1, IFI44L, SIGLEC1, ISG15, SLC1A2, IL1R2, OLAH, MMP8 | 0,857 | 0,848 | 0,867 | 0,929 | 0,880 | 0,978 | 0,948 | 0,893 | 1 |
| RSAD2, IFIT1, IFI44L, SIGLEC1, HERC6, SLC1A2, IL1R2, OLAH, FAM20A, RETN, MMP8 | 0,857 | 0,847 | 0,868 | 0,930 | 0,883 | 0,977 | 0,953 | 0,892 | 1 |
| RSAD2, OAS1, IFIT1, IFI44L, SIGLEC1, ISG15, IL1R2, OLAH, FAM20A, RETN, MMP8 | 0,857 | 0,847 | 0,868 | 0,920 | 0,863 | 0,978 | 0,968 | 0,918 | 1 |
| IFI27, OAS1, IFIT1, IFI44L, SIGLEC1, ISG15, HERC6, SLC1A2, IL1R2, RETN, MMP8 | 0,857 | 0,848 | 0,867 | 0,926 | 0,879 | 0,974 | 0,954 | 0,905 | 1 |
| RSAD2, IFI27, OAS1, IFIT1, IFI44L, SIGLEC1, ISG15, HERC6, SLC1A2, IL1R2, RETN | 0,857 | 0,848 | 0,866 | 0,928 | 0,880 | 0,976 | 0,945 | 0,882 | 1 |
| RSAD2, OAS1, IFI44L, SIGLEC1, ISG15, HERC6, IL1R2, OLAH, FAM20A, RETN, MMP8 | 0,857 | 0,846 | 0,868 | 0,917 | 0,859 | 0,975 | 0,960 | 0,900 | 1 |
| RSAD2, IFI27, OAS1, IFIT1, IFI44L, SIGLEC1, SLC1A2, IL1R2, OLAH, RETN, MMP8 | 0,857 | 0,847 | 0,867 | 0,927 | 0,876 | 0,978 | 0,952 | 0,895 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, ISG15, HERC6, SLC1A2, IL1R2, OLAH, RETN, MMP8 | 0,857 | 0,847 | 0,867 | 0,931 | 0,881 | 0,981 | 0,940 | 0,875 | 1 |
| RSAD2, IFI27, IFI44L, SIGLEC1, ISG15, HERC6, SLC1A2, IL1R2, OLAH, RETN, MMP8 | 0,857 | 0,847 | 0,867 | 0,931 | 0,883 | 0,980 | 0,949 | 0,896 | 1 |
| RSAD2, OAS1, SIGLEC1, ISG15, HERC6, SLC1A2, IL1R2, OLAH, FAM20A, RETN, MMP8 | 0,857 | 0,847 | 0,867 | 0,925 | 0,877 | 0,974 | 0,957 | 0,892 | 1 |
| RSAD2, IFI27, OAS1, IFIT1, ISG15, HERC6, SLC1A2, IL1R2, FAM20A, RETN, MMP8 | 0,857 | 0,846 | 0,867 | 0,933 | 0,884 | 0,981 | 0,926 | 0,843 | 1 |
| IFI27, OAS1, IFIT1, IFI44L, SIGLEC1, ISG15, HERC6, SLC1A2, IL1R2, OLAH, MMP8 | 0,857 | 0,846 | 0,867 | 0,929 | 0,881 | 0,977 | 0,941 | 0,885 | 0,998 |
| RSAD2, OAS1, IFIT1, IFI44L, SIGLEC1, ISG15, HERC6, SLC1A2, IL1R2, OLAH, FAM20A | 0,857 | 0,847 | 0,867 | 0,927 | 0,879 | 0,975 | 0,938 | 0,871 | 1 |
| RSAD2, IFI27, OAS1, IFIT1, SIGLEC1, ISG15, HERC6, SLC1A2, IL1R2, OLAH, MMP8 | 0,857 | 0,846 | 0,868 | 0,929 | 0,880 | 0,978 | 0,942 | 0,884 | 1 |
| OAS1, IFIT1, IFI44L, SIGLEC1, ISG15, SLC1A2, IL1R2, OLAH, FAM20A, RETN, MMP8 | 0,857 | 0,846 | 0,867 | 0,927 | 0,878 | 0,975 | 0,958 | 0,898 | 1 |
| RSAD2, OAS1, IFI44L, SIGLEC1, ISG15, HERC6, SLC1A2, IL1R2, OLAH, FAM20A, MMP8 | 0,857 | 0,846 | 0,867 | 0,922 | 0,872 | 0,972 | 0,959 | 0,897 | 1 |
| IFI27, OAS1, IFIT1, IFI44L, ISG15, HERC6, SLC1A2, IL1R2, OLAH, RETN, MMP8 | 0,857 | 0,846 | 0,867 | 0,929 | 0,879 | 0,980 | 0,939 | 0,874 | 1 |
| RSAD2, IFIT1, IFI44L, SIGLEC1, ISG15, HERC6, SLC1A2, IL1R2, FAM20A, RETN, MMP8 | 0,857 | 0,847 | 0,866 | 0,929 | 0,882 | 0,975 | 0,957 | 0,889 | 1 |
| RSAD2, IFI27, OAS1, IFIT1, IFI44L, SIGLEC1, HERC6, SLC1A2, IL1R2, RETN, MMP8 | 0,857 | 0,847 | 0,866 | 0,929 | 0,881 | 0,977 | 0,950 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| IFI27, OAS1, IFIT1, IFI44L, SIGLEC1, ISG15, SLC1A2, IL1R2, OLAH, RETN, MMP8 | 0,856 | 0,847 | 0,866 | 0,928 | 0,878 | 0,978 | 0,950 | 0,894 | 1 |
| RSAD2, IFI27, OAS1, IFIT1, IFI44L, SIGLEC1, ISG15, HERC6, SLC1A2, OLAH, MMP8 | 0,856 | 0,845 | 0,868 | 0,931 | 0,881 | 0,980 | 0,942 | 0,885 | 1 |
| RSAD2, IFI27, OAS1, IFIT1, IFI44L, ISG15, HERC6, SLC1A2, OLAH, RETN, MMP8 | 0,856 | 0,846 | 0,867 | 0,929 | 0,877 | 0,980 | 0,940 | 0,876 | 1 |
| RSAD2, IFI27, IFIT1, IFI44L, SIGLEC1, ISG15, HERC6, SLC1A2, OLAH, RETN, MMP8 | 0,856 | 0,846 | 0,867 | 0,931 | 0,881 | 0,980 | 0,951 | 0,899 | 1 |
| RSAD2, OAS1, IFIT1, IFI44L, SIGLEC1, ISG15, HERC6, IL1R2, OLAH, FAM20A, RETN | 0,856 | 0,846 | 0,866 | 0,918 | 0,861 | 0,976 | 0,953 | 0,898 | 1 |
| RSAD2, IFI27, OAS1, IFIT1, SIGLEC1, ISG15, SLC1A2, IL1R2, OLAH, RETN, MMP8 | 0,856 | 0,846 | 0,866 | 0,931 | 0,881 | 0,980 | 0,951 | 0,892 | 1 |
| RSAD2, IFI27, IFIT1, IFI44L, ISG15, HERC6, SLC1A2, IL1R2, FAM20A, RETN, MMP8 | 0,856 | 0,846 | 0,867 | 0,934 | 0,886 | 0,982 | 0,930 | 0,849 | 1 |
| RSAD2, IFI27, OAS1, IFIT1, SIGLEC1, ISG15, HERC6, SLC1A2, IL1R2, RETN, MMP8 | 0,856 | 0,847 | 0,866 | 0,930 | 0,882 | 0,977 | 0,953 | 0,899 | 1 |
| RSAD2, IFI27, OAS1, IFI44L, SIGLEC1, ISG15, HERC6, SLC1A2, IL1R2, OLAH, MMP8 | 0,856 | 0,845 | 0,867 | 0,930 | 0,881 | 0,978 | 0,945 | 0,890 | 1,000 |
| RSAD2, OAS1, IFIT1, SIGLEC1, ISG15, HERC6, SLC1A2, IL1R2, FAM20A, RETN, MMP8 | 0,856 | 0,846 | 0,866 | 0,928 | 0,881 | 0,975 | 0,960 | 0,897 | 1 |
| RSAD2, IFI27, IFIT1, IFI44L, SIGLEC1, ISG15, HERC6, SLC1A2, IL1R2, OLAH, RETN | 0,856 | 0,846 | 0,866 | 0,928 | 0,878 | 0,978 | 0,941 | 0,884 | 0,998 |
| RSAD2, OAS1, IFIT1, IFI44L, SIGLEC1, HERC6, SLC1A2, IL1R2, FAM20A, RETN, MMP8 | 0,856 | 0,845 | 0,866 | 0,928 | 0,881 | 0,975 | 0,958 | 0,892 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, OAS1, IFIT1, IFI44L, SIGLEC1, ISG15, HERC6, SLC1A2, IL1R2, FAM20A, MMP8 | 0,856 | 0,845 | 0,867 | 0,927 | 0,882 | 0,973 | 0,961 | 0,899 | 1 |
| RSAD2, IFI27, OAS1, IFI44L, ISG15, HERC6, SLC1A2, IL1R2, FAM20A, RETN, MMP8 | 0,856 | 0,844 | 0,867 | 0,933 | 0,885 | 0,982 | 0,923 | 0,834 | 1 |
| RSAD2, IFI27, IFIT1, IFI44L, ISG15, HERC6, SLC1A2, IL1R2, OLAH, RETN, MMP8 | 0,856 | 0,846 | 0,866 | 0,929 | 0,878 | 0,979 | 0,937 | 0,875 | 0,999 |
| RSAD2, IFI27, OAS1, IFIT1, IFI44L, SIGLEC1, ISG15, SLC1A2, IL1R2, OLAH, RETN | 0,856 | 0,846 | 0,865 | 0,930 | 0,880 | 0,980 | 0,947 | 0,883 | 1 |
| RSAD2, IFI27, OAS1, IFIT1, IFI44L, ISG15, HERC6, SLC1A2, IL1R2, OLAH, RETN | 0,856 | 0,845 | 0,866 | 0,929 | 0,878 | 0,979 | 0,933 | 0,861 | 1 |
| RSAD2, IFI27, OAS1, IFIT1, IFI44L, ISG15, HERC6, SLC1A2, IL1R2, FAM20A, MMP8 | 0,856 | 0,844 | 0,867 | 0,931 | 0,885 | 0,977 | 0,926 | 0,840 | 1 |
| RSAD2, IFI27, OAS1, IFI44L, SIGLEC1, ISG15, HERC6, SLC1A2, IL1R2, RETN, MMP8 | 0,855 | 0,846 | 0,865 | 0,929 | 0,881 | 0,977 | 0,949 | 0,890 | 1 |
| RSAD2, IFI27, OAS1, IFIT1, IFI44L, ISG15, HERC6, SLC1A2, IL1R2, FAM20A, RETN | 0,855 | 0,845 | 0,866 | 0,933 | 0,886 | 0,980 | 0,926 | 0,840 | 1 |
| RSAD2, IFIT1, IFI44L, SIGLEC1, ISG15, SLC1A2, IL1R2, OLAH, FAM20A, RETN, MMP8 | 0,855 | 0,845 | 0,865 | 0,926 | 0,878 | 0,975 | 0,959 | 0,899 | 1 |
| RSAD2, OAS1, IFIT1, IFI44L, SIGLEC1, ISG15, HERC6, SLC1A2, OLAH, FAM20A, RETN | 0,855 | 0,845 | 0,865 | 0,926 | 0,876 | 0,975 | 0,933 | 0,863 | 1 |
| RSAD2, OAS1, IFIT1, SIGLEC1, ISG15, SLC1A2, IL1R2, OLAH, FAM20A, RETN, MMP8 | 0,855 | 0,845 | 0,865 | 0,927 | 0,879 | 0,975 | 0,963 | 0,906 | 1 |
| RSAD2, OAS1, IFIT1, IFI44L, SIGLEC1, ISG15, SLC1A2, OLAH, FAM20A, RETN, MMP8 | 0,855 | 0,845 | 0,865 | 0,922 | 0,870 | 0,975 | 0,945 | 0,883 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, IFIT1, IFI44L, SIGLEC1, ISG15, SLC1A2, IL1R2, OLAH, RETN, MMP8 | 0,855 | 0,844 | 0,865 | 0,930 | 0,882 | 0,979 | 0,953 | 0,902 | 1 |
| RSAD2, OAS1, IFIT1, SIGLEC1, ISG15, HERC6, SLC1A2, IL1R2, OLAH, FAM20A, RETN | 0,855 | 0,845 | 0,865 | 0,926 | 0,877 | 0,975 | 0,948 | 0,884 | 1 |
| OAS1, IFIT1, IFI44L, SIGLEC1, ISG15, HERC6, SLC1A2, IL1R2, OLAH, FAM20A, RETN | 0,855 | 0,844 | 0,865 | 0,927 | 0,878 | 0,975 | 0,934 | 0,865 | 1 |
| OAS1, IFI44L, SIGLEC1, ISG15, HERC6, SLC1A2, IL1R2, OLAH, FAM20A, RETN, MMP8 | 0,855 | 0,844 | 0,865 | 0,923 | 0,874 | 0,973 | 0,959 | 0,897 | 1 |
| RSAD2, IFI27, IFIT1, IFI44L, SIGLEC1, ISG15, HERC6, SLC1A2, IL1R2, OLAH, MMP8 | 0,855 | 0,845 | 0,864 | 0,929 | 0,879 | 0,978 | 0,945 | 0,892 | 0,997 |
| RSAD2, IFI27, OAS1, IFIT1, IFI44L, ISG15, HERC6, SLC1A2, FAM20A, RETN, MMP8 | 0,854 | 0,843 | 0,866 | 0,924 | 0,869 | 0,980 | 0,930 | 0,849 | 1 |
| RSAD2, IFI27, OAS1, IFIT1, IFI44L, ISG15, SLC1A2, IL1R2, FAM20A, RETN, MMP8 | 0,854 | 0,843 | 0,865 | 0,931 | 0,882 | 0,979 | 0,927 | 0,844 | 1 |
| RSAD2, IFI27, OAS1, IFIT1, IFI44L, ISG15, SLC1A2, IL1R2, OLAH, RETN, MMP8 | 0,854 | 0,844 | 0,864 | 0,926 | 0,875 | 0,978 | 0,941 | 0,878 | 1 |
| RSAD2, IFI27, OAS1, IFIT1, IFI44L, SIGLEC1, ISG15, SLC1A2, OLAH, RETN, MMP8 | 0,854 | 0,843 | 0,864 | 0,928 | 0,875 | 0,981 | 0,953 | 0,895 | 1 |
| RSAD2, OAS1, IFIT1, IFI44L, SIGLEC1, SLC1A2, IL1R2, OLAH, FAM20A, RETN, MMP8 | 0,854 | 0,843 | 0,864 | 0,927 | 0,878 | 0,975 | 0,959 | 0,904 | 1 |
| RSAD2, IFI44L, SIGLEC1, ISG15, HERC6, SLC1A2, IL1R2, OLAH, FAM20A, RETN, MMP8 | 0,854 | 0,843 | 0,864 | 0,924 | 0,876 | 0,973 | 0,959 | 0,897 | 1 |
| RSAD2, OAS1, IFIT1, IFI44L, SIGLEC1, HERC6, SLC1A2, IL1R2, OLAH, FAM20A, RETN | 0,854 | 0,843 | 0,864 | 0,927 | 0,879 | 0,975 | 0,937 | 0,869 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFI44L, SIGLEC1, ISG15, SLC1A2, IL1R2, OLAH, RETN, MMP8 | 0,853 | 0,843 | 0,864 | 0,931 | 0,882 | 0,980 | 0,953 | 0,896 | 1 |
| RSAD2, OAS1, IFIT1, IFI44L, SIGLEC1, ISG15, HERC6, SLC1A2, FAM20A, RETN, MMP8 | 0,853 | 0,843 | 0,864 | 0,926 | 0,876 | 0,977 | 0,939 | 0,868 | 1 |
| RSAD2, IFI27, OAS1, IFIT1, IFI44L, ISG15, HERC6, SLC1A2, IL1R2, OLAH, MMP8 | 0,853 | 0,843 | 0,864 | 0,927 | 0,879 | 0,976 | 0,935 | 0,871 | 0,998 |
| RSAD2, OAS1, IFI44L, SIGLEC1, HERC6, SLC1A2, IL1R2, OLAH, FAM20A, RETN, MMP8 | 0,853 | 0,842 | 0,864 | 0,924 | 0,875 | 0,973 | 0,958 | 0,897 | 1 |
| RSAD2, OAS1, IFI44L, SIGLEC1, ISG15, SLC1A2, IL1R2, OLAH, FAM20A, RETN, MMP8 | 0,853 | 0,843 | 0,863 | 0,922 | 0,872 | 0,971 | 0,959 | 0,897 | 1 |
| RSAD2, OAS1, IFIT1, IFI44L, SIGLEC1, ISG15, SLC1A2, IL1R2, FAM20A, RETN, MMP8 | 0,853 | 0,843 | 0,863 | 0,927 | 0,880 | 0,974 | 0,960 | 0,895 | 1 |
| RSAD2, IFIT1, IFI44L, SIGLEC1, ISG15, HERC6, SLC1A2, IL1R2, OLAH, FAM20A, RETN | 0,853 | 0,843 | 0,863 | 0,926 | 0,878 | 0,975 | 0,937 | 0,867 | 1 |
| RSAD2, OAS1, IFI44L, SIGLEC1, ISG15, HERC6, SLC1A2, IL1R2, FAM20A, RETN, MMP8 | 0,853 | 0,842 | 0,863 | 0,925 | 0,877 | 0,973 | 0,958 | 0,892 | 1 |
| RSAD2, OAS1, IFI44L, SIGLEC1, ISG15, HERC6, SLC1A2, OLAH, FAM20A, RETN, MMP8 | 0,852 | 0,842 | 0,863 | 0,920 | 0,867 | 0,974 | 0,941 | 0,872 | 1 |
| RSAD2, OAS1, IFIT1, IFI44L, SIGLEC1, ISG15, HERC6, SLC1A2, IL1R2, FAM20A, RETN | 0,852 | 0,842 | 0,862 | 0,926 | 0,879 | 0,973 | 0,946 | 0,879 | 1 |
| RSAD2, IFI27, OAS1, IFIT1, IFI44L, ISG15, HERC6, SLC1A2, IL1R2, RETN, MMP8 | 0,852 | 0,842 | 0,861 | 0,923 | 0,872 | 0,974 | 0,935 | 0,866 | 1 |
| RSAD2, IFI27, IFIT1, IFI44L, SIGLEC1, ISG15, HERC6, SLC1A2, IL1R2, RETN, MMP8 | 0,851 | 0,842 | 0,861 | 0,927 | 0,880 | 0,975 | 0,954 | 0,908 | 1 |

(suite)

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, SLC1A2, IL1R2, RETN, MMP8 | 0,851 | 0,842 | 0,860 | 0,928 | 0,880 | 0,976 | 0,958 | 0,905 | 1 |
| RSAD2, OAS1, IFIT1, IFI44L, SIGLEC1, ISG15, SLC1A2, IL1R2, OLAH, FAM20A, RETN | 0,851 | 0,841 | 0,861 | 0,921 | 0,869 | 0,973 | 0,949 | 0,888 | 1 |

Performances obtenues pour l'identification de la nature virale ou bactérienne de l'infection sur la base des combinaisons à 12 gènes cibles :

[Tableau 16]

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2, IFI27, OAS1, IFIT1, SIGLEC1, ISG15, HERC6, IL1R2, OLAH, FAM20A, RETN, MMP8 | 0,870 | 0,858 | 0,881 | 0,938 | 0,886 | 0,989 | 0,951 | 0,888 | 1 |
| IFI27, OAS1, IFIT1, IFI44L, SIGLEC1, ISG15, HERC6, IL1R2, OLAH, FAM20A, RETN, MMP8 | 0,870 | 0,859 | 0,881 | 0,939 | 0,888 | 0,990 | 0,952 | 0,890 | 1 |
| RSAD2, IFI27, OAS1, IFIT1, IFI44L, SIGLEC1, ISG15, HERC6, IL1R2, OLAH, FAM20A, MMP8 | 0,869 | 0,857 | 0,880 | 0,938 | 0,890 | 0,986 | 0,947 | 0,882 | 1 |
| RSAD2, IFI27, OAS1, IFIT1, IFI44L, SIGLEC1, HERC6, IL1R2, OLAH, FAM20A, RETN, MMP8 | 0,868 | 0,856 | 0,880 | 0,938 | 0,887 | 0,989 | 0,951 | 0,888 | 1 |
| RSAD2, IFI27, OAS1, IFIT1, SIGLEC1, ISG15, HERC6, SLC1A2, IL1R2, OLAH, FAM20A, MMP8 | 0,867 | 0,855 | 0,878 | 0,943 | 0,900 | 0,986 | 0,948 | 0,881 | 1 |
| RSAD2, IFI27, OAS1, IFIT1, IFI44L, SIGLEC1, ISG15, HERC6, IL1R2, OLAH, FAM20A, RETN | 0,865 | 0,855 | 0,876 | 0,932 | 0,879 | 0,985 | 0,940 | 0,876 | 1 |
| IFI27, OAS1, IFIT1, IFI44L, SIGLEC1, ISG15, HERC6, SLC1A2, IL1R2, OLAH, FAM20A, MMP8 | 0,865 | 0,853 | 0,876 | 0,942 | 0,898 | 0,985 | 0,947 | 0,879 | 1 |
| RSAD2, IFI27, OAS1, IFIT1, IFI44L, SIGLEC1, HERC6, SLC1A2, IL1R2, OLAH, FAM20A, MMP8 | 0,865 | 0,853 | 0,876 | 0,941 | 0,896 | 0,985 | 0,946 | 0,880 | 1 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2, IFI27, OAS1, IFI44L, SIGLEC1, ISG15, HERC6, IL1R2, OLAH, FAM20A, RETN, MMP8 | 0,864 | 0,852 | 0,877 | 0,938 | 0,886 | 0,989 | 0,948 | 0,883 | 1 |
| RSAD2, IFI27, OAS1, IFIT1, IFI44L, SIGLEC1, ISG15, SLC1A2, IL1R2, OLAH, FAM20A, MMP8 | 0,864 | 0,853 | 0,875 | 0,942 | 0,898 | 0,986 | 0,946 | 0,877 | 1 |
| IFI27, OAS1, IFIT1, SIGLEC1, ISG15, HERC6, SLC1A2, IL1R2, OLAH, FAM20A, RETN, MMP8 | 0,864 | 0,853 | 0,876 | 0,944 | 0,900 | 0,987 | 0,945 | 0,876 | 1 |
| RSAD2, IFI27, IFIT1, IFI44L, SIGLEC1, ISG15, HERC6, | 0,864 | 0,852 | 0,875 | 0,943 | 0,900 | 0,986 | 0,949 | 0,883 | 1 |
| SLC1A2, IL1R2, OLAH, FAM20A, MMP8 | | | | | | | | | |
| RSAD2, IFI27, OAS1, IFIT1, IFI44L, SIGLEC1, ISG15, HERC6, OLAH, FAM20A, RETN, MMP8 | 0,864 | 0,851 | 0,876 | 0,936 | 0,884 | 0,989 | 0,945 | 0,879 | 1 |
| RSAD2, IFI27, IFIT1, IFI44L, SIGLEC1, ISG15, HERC6, IL1R2, OLAH, FAM20A, RETN, MMP8 | 0,863 | 0,851 | 0,876 | 0,939 | 0,888 | 0,989 | 0,949 | 0,885 | 1 |
| RSAD2, IFI27, OAS1, IFIT1, SIGLEC1, HERC6, SLC1A2, IL1R2, OLAH, FAM20A, RETN, MMP8 | 0,863 | 0,852 | 0,875 | 0,942 | 0,898 | 0,986 | 0,942 | 0,874 | 1 |
| RSAD2, IFI27, OAS1, IFI44L, SIGLEC1, ISG15, HERC6, SLC1A2, IL1R2, OLAH, FAM20A, MMP8 | 0,863 | 0,851 | 0,875 | 0,940 | 0,897 | 0,984 | 0,947 | 0,879 | 1 |
| RSAD2, IFI27, OAS1, IFIT1, IFI44L, SIGLEC1, ISG15, HERC6, SLC1A2, IL1R2, OLAH, FAM20A | 0,863 | 0,853 | 0,874 | 0,938 | 0,893 | 0,984 | 0,934 | 0,864 | 1 |
| IFI27, OAS1, IFI44L, SIGLEC1, ISG15, HERC6, SLC1A2, IL1R2, OLAH, FAM20A, RETN, MMP8 | 0,863 | 0,852 | 0,874 | 0,943 | 0,900 | 0,986 | 0,945 | 0,876 | 1 |
| RSAD2, IFI27, OAS1, IFIT1, IFI44L, SIGLEC1, ISG15, HERC6, SLC1A2, OLAH, FAM20A, MMP8 | 0,863 | 0,851 | 0,875 | 0,941 | 0,896 | 0,986 | 0,938 | 0,871 | 1 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| IFI27, OAS1, IFIT1, IFI44L, SIGLEC1, HERC6, SLC1A2, IL1R2, OLAH, FAM20A, RETN, MMP8 | 0,863 | 0,850 | 0,875 | 0,942 | 0,897 | 0,986 | 0,942 | 0,874 | 1 |
| RSAD2, IFI27, IFIT1, SIGLEC1, ISG15, HERC6, SLC1A2, IL1R2, OLAH, FAM20A, RETN, MMP8 | 0,862 | 0,851 | 0,874 | 0,944 | 0,902 | 0,986 | 0,946 | 0,877 | 1 |
| RSAD2, IFI27, OAS1, IFIT1, IFI44L, SIGLEC1, ISG15, IL1R2, OLAH, FAM20A, RETN, MMP8 | 0,862 | 0,850 | 0,875 | 0,937 | 0,884 | 0,989 | 0,952 | 0,891 | 1 |
| RSAD2, IFI27, OAS1, IFIT1, SIGLEC1, ISG15, HERC6, SLC1A2, OLAH, FAM20A, RETN, MMP8 | 0,862 | 0,850 | 0,874 | 0,942 | 0,898 | 0,987 | 0,943 | 0,878 | 1 |
| RSAD2, IFI27, OAS1, IFIT1, IFI44L, SIGLEC1, ISG15, HERC6, IL1R2, FAM20A, RETN, MMP8 | 0,862 | 0,849 | 0,874 | 0,938 | 0,888 | 0,988 | 0,950 | 0,881 | 1 |
| IFI27, OAS1, IFIT1, IFI44L, SIGLEC1, ISG15, HERC6, SLC1A2, IL1R2, FAM20A, RETN, MMP8 | 0,861 | 0,849 | 0,873 | 0,942 | 0,899 | 0,984 | 0,948 | 0,876 | 1 |
| RSAD2, IFI27, OAS1, IFIT1, SIGLEC1, ISG15, HERC6, SLC1A2, IL1R2, OLAH, FAM20A, RETN | 0,861 | 0,850 | 0,872 | 0,939 | 0,894 | 0,983 | 0,936 | 0,867 | 1 |
| IFI27, OAS1, IFIT1, IFI44L, SIGLEC1, ISG15, HERC6, SLC1A2, OLAH, FAM20A, RETN, MMP8 | 0,861 | 0,848 | 0,874 | 0,943 | 0,898 | 0,987 | 0,943 | 0,878 | 1 |
| RSAD2, IFI27, OAS1, IFIT1, IFI44L, SIGLEC1, HERC6, SLC1A2, OLAH, FAM20A, RETN, MMP8 | 0,861 | 0,848 | 0,874 | 0,940 | 0,894 | 0,986 | 0,941 | 0,874 | 1 |
| RSAD2, IFI27, OAS1, SIGLEC1, ISG15, HERC6, SLC1A2, IL1R2, OLAH, FAM20A, RETN, MMP8 | 0,861 | 0,848 | 0,873 | 0,943 | 0,899 | 0,986 | 0,946 | 0,877 | 1 |
| RSAD2, IFI27, OAS1, IFI44L, SIGLEC1, HERC6, SLC1A2, IL1R2, OLAH, FAM20A, RETN, MMP8 | 0,861 | 0,849 | 0,873 | 0,940 | 0,894 | 0,986 | 0,947 | 0,881 | 1 |
| RSAD2, IFI27, IFIT1, IFI44L, SIGLEC1, ISG15, HERC6, SLC1A2, IL1R2, FAM20A, RETN, MMP8 | 0,860 | 0,850 | 0,871 | 0,940 | 0,895 | 0,985 | 0,950 | 0,881 | 1 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2, IFI27, OAS1, IFIT1, IFI44L, SIGLEC1, HERC6, SLC1A2, IL1R2, OLAH, FAM20A, RETN | 0,860 | 0,849 | 0,872 | 0,937 | 0,891 | 0,983 | 0,937 | 0,870 | 1 |
| IFI27, OAS1, IFIT1, IFI44L, SIGLEC1, ISG15, HERC6, SLC1A2, IL1R2, OLAH, FAM20A, RETN | 0,860 | 0,849 | 0,872 | 0,939 | 0,895 | 0,984 | 0,932 | 0,861 | 1 |
| RSAD2, IFI27, OAS1, IFIT1, IFI44L, SIGLEC1, ISG15, HERC6, SLC1A2, IL1R2, FAM20A, MMP8 | 0,860 | 0,849 | 0,871 | 0,938 | 0,896 | 0,981 | 0,951 | 0,882 | 1 |
| RSAD2, IFI27, OAS1, IFIT1, SIGLEC1, ISG15, SLC1A2, IL1R2, OLAH, FAM20A, RETN, MMP8 | 0,860 | 0,848 | 0,872 | 0,942 | 0,897 | 0,986 | 0,947 | 0,880 | 1 |
| RSAD2, IFI27, OAS1, IFIT1, IFI44L, ISG15, HERC6, IL1R2, OLAH, FAM20A, RETN, MMP8 | 0,860 | 0,848 | 0,873 | 0,933 | 0,880 | 0,986 | 0,933 | 0,861 | 1 |
| RSAD2, IFI27, OAS1, IFIT1, SIGLEC1, ISG15, HERC6, SLC1A2, IL1R2, FAM20A, RETN, MMP8 | 0,860 | 0,849 | 0,871 | 0,939 | 0,895 | 0,983 | 0,949 | 0,877 | 1 |
| IFI27, IFIT1, IFI44L, SIGLEC1, ISG15, HERC6, SLC1A2, IL1R2, OLAH, FAM20A, RETN, MMP8 | 0,860 | 0,848 | 0,872 | 0,944 | 0,901 | 0,987 | 0,948 | 0,879 | 1 |
| RSAD2, IFI27, OAS1, IFIT1, IFI44L, SIGLEC1, HERC6, SLC1A2, IL1R2, FAM20A, RETN, MMP8 | 0,860 | 0,848 | 0,872 | 0,941 | 0,898 | 0,984 | 0,946 | 0,872 | 1 |
| RSAD2, IFI27, OAS1, IFIT1, IFI44L, SIGLEC1, ISG15, HERC6, SLC1A2, OLAH, FAM20A, RETN | 0,859 | 0,848 | 0,871 | 0,938 | 0,893 | 0,984 | 0,937 | 0,870 | 1 |
| RSAD2, IFI27, IFIT1, IFI44L, SIGLEC1, HERC6, SLC1A2, IL1R2, OLAH, FAM20A, RETN, MMP8 | 0,859 | 0,846 | 0,872 | 0,940 | 0,895 | 0,986 | 0,949 | 0,884 | 1 |
| RSAD2, IFI27, OAS1, IFI44L, SIGLEC1, ISG15, HERC6, SLC1A2, IL1R2, FAM20A, RETN, MMP8 | 0,859 | 0,848 | 0,870 | 0,939 | 0,894 | 0,983 | 0,947 | 0,874 | 1 |
| RSAD2, IFI27, IFIT1, IFI44L, SIGLEC1, ISG15, SLC1A2, IL1R2, OLAH, FAM20A, RETN, MMP8 | 0,859 | 0,846 | 0,871 | 0,944 | 0,902 | 0,986 | 0,948 | 0,882 | 1 |

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2, IFI27, IFIT1, IFI44L, SIGLEC1, ISG15, HERC6, SLC1A2, IL1R2, OLAH, FAM20A, RETN | 0,859 | 0,848 | 0,870 | 0,939 | 0,893 | 0,984 | 0,934 | 0,866 | 1 |
| RSAD2, IFI27, OAS1, IFI44L, SIGLEC1, ISG15, HERC6, SLC1A2, IL1R2, OLAH, FAM20A, RETN | 0,858 | 0,847 | 0,869 | 0,938 | 0,893 | 0,983 | 0,937 | 0,866 | 1 |
| RSAD2, IFI27, IFIT1, IFI44L, SIGLEC1, ISG15, HERC6, SLC1A2, OLAH, FAM20A, RETN, MMP8 | 0,858 | 0,846 | 0,871 | 0,943 | 0,899 | 0,987 | 0,948 | 0,884 | 1 |
| RSAD2, IFI27, IFI44L, SIGLEC1, ISG15, HERC6, SLC1A2, IL1R2, OLAH, FAM20A, RETN, MMP8 | 0,858 | 0,845 | 0,871 | 0,943 | 0,900 | 0,986 | 0,947 | 0,880 | 1 |
| IFI27, OAS1, IFIT1, IFI44L, SIGLEC1, ISG15, SLC1A2, IL1R2, OLAH, FAM20A, RETN, MMP8 | 0,858 | 0,845 | 0,872 | 0,942 | 0,899 | 0,986 | 0,947 | 0,880 | 1 |
| RSAD2, IFI27, OAS1, IFIT1, IFI44L, SIGLEC1, ISG15, SLC1A2, IL1R2, OLAH, FAM20A, RETN | 0,858 | 0,847 | 0,869 | 0,935 | 0,887 | 0,984 | 0,940 | 0,874 | 1 |
| RSAD2, IFI27, OAS1, IFIT1, IFI44L, SIGLEC1, ISG15, HERC6, IL1R2, OLAH, RETN, MMP8 | 0,858 | 0,848 | 0,868 | 0,927 | 0,874 | 0,979 | 0,952 | 0,901 | 1 |
| RSAD2, IFI27, OAS1, IFI44L, SIGLEC1, ISG15, SLC1A2, IL1R2, OLAH, FAM20A, RETN, MMP8 | 0,858 | 0,845 | 0,870 | 0,941 | 0,897 | 0,985 | 0,945 | 0,875 | 1 |
| RSAD2, IFI27, OAS1, IFIT1, ISG15, HERC6, SLC1A2, IL1R2, OLAH, FAM20A, RETN, MMP8 | 0,858 | 0,847 | 0,869 | 0,933 | 0,885 | 0,982 | 0,921 | 0,839 | 1 |
| IFI27, OAS1, IFIT1, IFI44L, ISG15, HERC6, SLC1A2, IL1R2, OLAH, FAM20A, RETN, MMP8 | 0,858 | 0,846 | 0,869 | 0,935 | 0,886 | 0,983 | 0,922 | 0,840 | 1 |
| RSAD2, IFI27, OAS1, IFIT1, IFI44L, ISG15, HERC6, SLC1A2, IL1R2, OLAH, FAM20A, MMP8 | 0,858 | 0,845 | 0,870 | 0,934 | 0,886 | 0,983 | 0,925 | 0,848 | 1 |
| RSAD2, IFI27, OAS1, IFI44L, SIGLEC1, ISG15, HERC6, SLC1A2, OLAH, FAM20A, RETN, MMP8 | 0,858 | 0,845 | 0,871 | 0,941 | 0,896 | 0,987 | 0,943 | 0,877 | 1 |

**EP 4 365 308 A1**

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2, IFI27, OAS1, IFIT1, IFI44L, SIGLEC1, ISG15, SLC1A2, OLAH, FAM20A, RETN, MMP8 | 0,857 | 0,845 | 0,870 | 0,937 | 0,888 | 0,986 | 0,947 | 0,883 | 1 |
| RSAD2, IFI27, OAS1, IFIT1, IFI44L, SIGLEC1, ISG15, HERC6, SLC1A2, IL1R2, FAM20A, RETN | 0,857 | 0,847 | 0,868 | 0,937 | 0,892 | 0,981 | 0,938 | 0,866 | 1 |
| RSAD2, IFI27, OAS1, IFIT1, IFI44L, SIGLEC1, ISG15, | 0,857 | 0,846 | 0,868 | 0,934 | 0,884 | 0,984 | 0,941 | 0,872 | 1 |
| HERC6, SLC1A2, FAM20A, RETN, MMP8 | | | | | | | | | |
| RSAD2, IFI27, OAS1, IFIT1, IFI44L, ISG15, HERC6, SLC1A2, OLAH, FAM20A, RETN, MMP8 | 0,857 | 0,845 | 0,869 | 0,934 | 0,885 | 0,984 | 0,923 | 0,842 | 1 |
| RSAD2, IFI27, OAS1, IFI44L, ISG15, HERC6, SLC1A2, IL1R2, OLAH, FAM20A, RETN, MMP8 | 0,856 | 0,844 | 0,869 | 0,936 | 0,889 | 0,984 | 0,924 | 0,843 | 1 |
| RSAD2, IFI27, OAS1, IFIT1, IFI44L, SIGLEC1, ISG15, SLC1A2, IL1R2, FAM20A, RETN, MMP8 | 0,856 | 0,845 | 0,867 | 0,938 | 0,894 | 0,982 | 0,949 | 0,879 | 1 |
| RSAD2, IFI27, OAS1, IFIT1, IFI44L, HERC6, SLC1A2, IL1R2, OLAH, FAM20A, RETN, MMP8 | 0,856 | 0,844 | 0,868 | 0,937 | 0,890 | 0,985 | 0,924 | 0,844 | 1 |
| RSAD2, IFI27, OAS1, IFIT1, IFI44L, SIGLEC1, SLC1A2, IL1R2, OLAH, FAM20A, RETN, MMP8 | 0,856 | 0,843 | 0,869 | 0,937 | 0,889 | 0,984 | 0,950 | 0,886 | 1 |
| RSAD2, IFI27, OAS1, IFIT1, IFI44L, ISG15, SLC1A2, IL1R2, OLAH, FAM20A, RETN, MMP8 | 0,856 | 0,844 | 0,867 | 0,934 | 0,884 | 0,983 | 0,923 | 0,843 | 1 |
| RSAD2, IFI27, IFIT1, IFI44L, ISG15, HERC6, SLC1A2, IL1R2, OLAH, FAM20A, RETN, MMP8 | 0,855 | 0,843 | 0,867 | 0,935 | 0,888 | 0,983 | 0,923 | 0,844 | 1 |
| RSAD2, OAS1, IFIT1, IFI44L, SIGLEC1, ISG15, HERC6, IL1R2, OLAH, FAM20A, RETN, MMP8 | 0,854 | 0,844 | 0,865 | 0,924 | 0,869 | 0,980 | 0,962 | 0,907 | 1 |

**815**

(suite)

| Modèle | AUC (modèle) | IC 95% | | AUC (Train) | IC 95% (Train) | | AUC (Test) | IC 95% (Test) | |
|---|---|---|---|---|---|---|---|---|---|
| RSAD2, IFI27, OAS1, IFIT1, IFI44L, ISG15, HERC6, SLC1A2, IL1R2, OLAH, FAM20A, RETN | 0,854 | 0,842 | 0,865 | 0,934 | 0,886 | 0,982 | 0,920 | 0,835 | 1 |
| RSAD2, IFI27, OAS1, IFIT1, IFI44L, SIGLEC1, HERC6, SLC1A2, IL1R2, OLAH, RETN, MMP8 | 0,853 | 0,843 | 0,864 | 0,932 | 0,883 | 0,981 | 0,949 | 0,890 | 1 |
| RSAD2, OAS1, IFIT1, IFI44L, SIGLEC1, ISG15, HERC6, SLC1A2, IL1R2, OLAH, FAM20A, MMP8 | 0,853 | 0,842 | 0,863 | 0,929 | 0,882 | 0,976 | 0,954 | 0,890 | 1 |
| RSAD2, IFI27, OAS1, IFIT1, IFI44L, SIGLEC1, ISG15, HERC6, SLC1A2, IL1R2, OLAH, RETN | 0,852 | 0,842 | 0,862 | 0,931 | 0,881 | 0,981 | 0,943 | 0,881 | 1 |
| OAS1, IFIT1, IFI44L, SIGLEC1, ISG15, HERC6, SLC1A2, IL1R2, OLAH, FAM20A, RETN, MMP8 | 0,852 | 0,841 | 0,862 | 0,931 | 0,884 | 0,977 | 0,950 | 0,886 | 1 |
| IFI27, OAS1, IFIT1, IFI44L, SIGLEC1, ISG15, HERC6, SLC1A2, IL1R2, OLAH, RETN, MMP8 | 0,851 | 0,840 | 0,863 | 0,932 | 0,883 | 0,980 | 0,947 | 0,887 | 1 |
| RSAD2, OAS1, IFIT1, IFI44L, SIGLEC1, ISG15, HERC6, SLC1A2, OLAH, FAM20A, RETN, MMP8 | 0,851 | 0,841 | 0,862 | 0,929 | 0,880 | 0,978 | 0,942 | 0,877 | 1 |
| RSAD2, OAS1, IFIT1, SIGLEC1, ISG15, HERC6, SLC1A2, IL1R2, OLAH, FAM20A, RETN, MMP8 | 0,851 | 0,841 | 0,861 | 0,930 | 0,883 | 0,976 | 0,958 | 0,894 | 1 |
| RSAD2, IFI27, OAS1, IFIT1, SIGLEC1, ISG15, HERC6, SLC1A2, IL1R2, OLAH, RETN, MMP8 | 0,851 | 0,839 | 0,863 | 0,932 | 0,882 | 0,981 | 0,949 | 0,890 | 1 |
| RSAD2, IFIT1, IFI44L, SIGLEC1, ISG15, HERC6, SLC1A2, IL1R2, OLAH, FAM20A, RETN, MMP8 | 0,850 | 0,840 | 0,860 | 0,931 | 0,885 | 0,978 | 0,954 | 0,892 | 1 |
| RSAD2, IFI27, OAS1, IFIT1, IFI44L, SIGLEC1, ISG15, HERC6, SLC1A2, OLAH, RETN, MMP8 | 0,850 | 0,839 | 0,861 | 0,932 | 0,883 | 0,981 | 0,950 | 0,892 | 1 |

Performances obtenues pour l'identification de la nature virale ou bactérienne de l'infection sur la base des combinaisons à 13 gènes cibles :

[Tableau 17]

| Modèle | AUC modèle | IC 95% | | AUC Train | IC 95% Train | | AUC Test | IC 95% Test | |
|---|---|---|---|---|---|---|---|---|---|
| RSAD2, IFI27, OAS1, IFIT1, IFI44L, SIGLEC1, ISG15, HERC6, IL1R2, OLAH, FAM20A, RETN, MMP8 | 0,857 | 0,845 | 0,870 | 0,938 | 0,887 | 0,989 | 0,950 | 0,886 | 1 |
| RSAD2, IFI27, OAS1, IFIT1, IFI44L, SIGLEC1, ISG15, HERC6, SLC1A2, IL1R2, OLAH, FAM20A, MMP8 | 0,856 | 0,844 | 0,868 | 0,943 | 0,900 | 0,986 | 0,946 | 0,877 | 1 |
| RSAD2, IFI27, OAS1, IFIT1, SIGLEC1, ISG15, HERC6, SLC1A2, IL1R2, OLAH, FAM20A, RETN, MMP8 | 0,853 | 0,840 | 0,866 | 0,944 | 0,900 | 0,987 | 0,945 | 0,876 | 1 |
| IFI27, OAS1, IFIT1, IFI44L, SIGLEC1, ISG15, HERC6, SLC1A2, IL1R2, OLAH, FAM20A, RETN, MMP8 | 0,853 | 0,839 | 0,866 | 0,942 | 0,899 | 0,986 | 0,943 | 0,873 | 1 |
| RSAD2, IFI27, OAS1, IFIT1, IFI44L, SIGLEC1, HERC6, SLC1A2, IL1R2, OLAH, FAM20A, RETN, MMP8 | 0,852 | 0,838 | 0,865 | 0,942 | 0,898 | 0,986 | 0,943 | 0,877 | 1 |
| RSAD2, IFI27, OAS1, IFIT1, IFI44L, SIGLEC1, ISG15, HERC6, SLC1A2, IL1R2, FAM20A, RETN, MMP8 | 0,851 | 0,840 | 0,863 | 0,940 | 0,897 | 0,983 | 0,949 | 0,877 | 1 |
| RSAD2, IFI27, IFIT1, IFI44L, SIGLEC1, ISG15, HERC6, SLC1A2, IL1R2, OLAH, FAM20A, RETN, MMP8 | 0,851 | 0,838 | 0,864 | 0,945 | 0,903 | 0,986 | 0,943 | 0,874 | 1 |
| RSAD2, IFI27, OAS1, IFIT1, IFI44L, SIGLEC1, ISG15, HERC6, SLC1A2, IL1R2, OLAH, FAM20A, RETN | 0,851 | 0,839 | 0,863 | 0,939 | 0,894 | 0,983 | 0,936 | 0,867 | 1 |

Performances obtenues pour l'identification de la nature virale ou bactérienne de l'infection sur la base des combinaisons des 14 gènes cibles :

[Tableau 18]

| Modèle | AUC (modèle) | IC 95% | | AUC (Train) | IC 95% (Train) | | AUC (Test) | IC 95% (Test) | |
|---|---|---|---|---|---|---|---|---|---|
| SIGLEC1 ISG15 HERC6 SLC1A2 IL1R2 FAM20A OLAH RETN MMP8 RSAD2, IFI27, OAS1, IFIT1, IFI44L | 0,84 | 0,83 | 0,86 | 0,94 | 0,90 | 0,99 | 0,94 | 0,87 | 1,00 |

Performances obtenues pour l'identification de la nature virale ou bactérienne de l'infection sur la base des combinaisons des 14 gènes cibles et des 4 gènes additionnels:

[Tableau 19]

| | Modèle | AUC (modèle) | IC 95% | | AUC (Train) | IC 95% (Train) | | AUC (Test) | IC 95% (Test) | |
|---|---|---|---|---|---|---|---|---|---|---|
| | SIGLEC1 ISG15 HERC6 SLC1A2 IL1R2 FAM20A OLAH RETN MMP8 RSAD2, IFI27, OAS1, IFIT1, IFI44L, PI3, EBI3, ADGRE1 et S100P | 0,814 | 0,797 | 0,831 | 0,959 | 0,925 | 0,993 | 0,928 | 0,861 | 0,995 |

Ainsi, les signatures transcriptomiques selon l'invention permettent de déterminer la nature virale ou bactérienne d'une infection chez un patient infecté avec un niveau robuste de performance car les AUC obtenues sur le set TEST sont généralement égales ou supérieures aux AUC obtenues sur le set TRAIN.

**REFERENCES BIBLIOGRAPHIQUES**

Takeuchi O, Akira S (2010), "Pattern recognition receptors and inflammation"; Cell 140 (6):805-820.

Ramilo O, Mejías A (2009), "Shifting the paradigm: Host gene signatures for diagnosis of infectious diseases"; Cell Host Microbe 6(3):199-200.

Fauci, A. S et al. (2014), "The perpetual challenge of antimicrobial resistance"; JAMA 311, 1853-1854.

Hu et al. (2013), "Gene expression profiles in febrile children with defined viral and bacterial infection"; PNAS 12792-12797, vol.110, no. 31.

Eric D. Brown et al. (2016), « Antibacterial drug discovery in the resistance era » - Review - doi :10.1038/nature17042.

Ephraim L. Tsalik et al. (2021), « Discriminating bacterial and viral infection using a rapid host gene expression test"; Critical Care Medecine - Vol 49, no. 10.

Mark A. Poritz et al. (2011), « FilmArray, an automated Nested Multiplex PCR System for Multi-Pathogen Detection: development and application to respiratory tract infection", PLoS ONE 6(10):e26047.

William M. Schneider et al. (2014), "Interferon-stimulated genes: a complex web of host response" Annu Rev Immunol. 2014 ; 32: 513-545. doi:10.1146/annurev-immunol-032713-120231.

Emily Yang et al. (2020), « All about the RNA : interferon-stimulated genes that interfere with viral RNA processes"; Front. Immunol. 11:605024.

Trine H. Mogensen et al. (2001), « Molecular pathways in virus-induced cytokine production"; Microbiology and molecular biology review, Mar. 2001, p.131-150.

Kawasaki et al. (2014), « Toll-like receptor signaling pathway » ; Front. Immunol., Sec. Cancer Immunity and Immunotherapy.

Hochrainer et al. (2005), « The human HERC family of ubiquitin ligases: novel members, genomic organization, expression profiling, and evolutionary aspects » ; Genomics 85 153-164

**Revendications**

**1.** Méthode *in vitro* ou *ex vivo* pour déterminer la nature virale ou bactérienne d'une infection à partir d'un échantillon biologique d'un sujet infecté, ou susceptible d'être infecté, comprenant les étapes suivantes :

(a) mesure de l'expression d'au moins un gène cible viral et d'au moins un gène cible bactérien choisi parmi les gènes cibles bactériens OLAH, FAM20A, IL1R2, MMP8, RETN et SLC1A2.
(b) comparaison des expressions mesurées à l'étape (a) avec des valeurs d'expression de références prédéterminées desdits gènes cibles, et
(c) conclusion quant à la nature virale ou bactérienne de l'infection sur la base des résultats de comparaison.

2. Méthode selon la revendication 1, **caractérisée en ce que** le gène cible viral est choisi parmi les gènes cibles viraux de la voie de l'interféron, de la voie des cytokines pro-inflammatoires, de la voie de signalisation des récepteurs Toll-like, de la voie de signalisation des récepteurs RIG-I-like et de la voie de la présentation antigénique médiée par le CMH de classe II.

3. Méthode selon la revendication 1 ou 2, **caractérisée en ce que** le gène cible viral est choisi parmi les gènes cibles viraux impliqués dans la voie de l'interféron et de la présentation antigénique médiée par le CMH de classe II.

4. Méthode selon la revendication 3, **caractérisée en ce que** les gènes cibles viraux sont choisis parmi IFI27, SIGLEC1, IFN-α, IFN-β, IFN-ε, IFN-κ, IFN-ω, IFN-γ, IFNL1, IFNL2, IFNL3, ADAR1, IFIT1, IFIT2, IFIT3, IFIT5, IFI44L, ISG15, ISG20, MDA5, OAS1, OAS2, OAS3, OASL, PARP12, PKR, RIG-I, RNaseL, RSAD2, RBBP6, SHFL, TRIM22, TRIM25, TRIM32, TRIM69, Viperin, ZAP, ZCCHC3, ZNFX1, HERC1, HERC2, HERC3, HERC4, HERC5, HERC6 et leurs combinaisons.

5. Méthode selon l'une des revendications 1 à 4, **caractérisée en ce que** l'étape (a) comprend ou consiste en la mesure d'au moins un gène cible viral choisi parmi IFI27, SIGLEC1, ISG15, HERC6, RSAD2, OAS1, IFIT1, IFI44L et d'au moins un gène cible bactérien choisi parmi OLAH, FAM20A, IL1R2, MMP8, RETN et SLC1A2.

6. Méthode selon la revendication 5, **caractérisée en ce que** l'étape (a) comprend la mesure de l'expression du gène cible viral IFI27 et du gène cible bactérien OLAH.

7. Méthode selon la revendication 6, **caractérisée en ce que** l'étape (a) comprend également la mesure de l'expression du gène cible bactérien FAM20A.

8. Méthode selon l'une des revendications 1 à 5, **caractérisée en ce que** l'étape (a) de mesure comprend ou consiste en la mesure de l'expression de deux gènes cibles viraux choisis parmi IFI27, SIGLEC1, ISG15, HERC6, RSAD2, OAS1, IFIT1 et IFI44L et de deux gènes cibles bactériens choisis parmi OLAH, FAM20A, IL1R2, MMP8, RETN et SLC1A2, et de préférence choisis parmi OLAH, FAM20A, IL1R2 et MMP8.

9. Méthode selon l'une des revendications 1 à 5, **caractérisée en ce que** l'étape (a) de mesure comprend ou consiste en la mesure de l'expression de deux à cinq gènes cibles viraux choisis parmi SIGLEC1, ISG15, HERC6, RSAD2, IFI27, OAS1, IFIT1 et IFI44L et de trois ou quatre gènes cibles bactériens choisis parmi OLAH, FAM20A, IL1R2, MMP8, RETN et SLC1A2, et de préférence choisis parmi OLAH, FAM20A, IL1R2 et MMP8.

10. Méthode selon la revendication 5, **caractérisée en ce que** l'étape (a) de mesure comprend ou consiste en la mesure de l'expression du gène cible viral IFI27, et éventuellement d'au moins un autre gène viral choisi parmi SIGLEC1, ISG15, HERC6, RSAD2, OAS1, IFIT1, IFI44L, et d'au moins deux gènes cibles bactériens choisis parmi OLAH, FAM20A, IL1R2, MMP8, RETN et SLC1A2, et de préférence choisis parmi OLAH, FAM20A, IL1R2 et MMP8.

11. Méthode selon la revendication 5, **caractérisée en ce que** l'étape (a) comprend ou consiste en la mesure d'au moins un gène cible viral choisi parmi SIGLEC1, ISG15 et HERC6, et d'au moins un gène cible bactérien choisi parmi OLAH, FAM20A, IL1R2, MMP8, RETN et SLC1A2, de préférence parmi IL1R2, OLAH et FAM20A.

12. Méthode selon la revendication 5, **caractérisée en ce que** l'étape (a) comprend ou consiste en la mesure d'au moins un gène cible viral choisi parmi IFI27, SIGLEC1, ISG15, HERC6, RSAD2, OAS1, IFIT1 et IFI44L, du gène cible bactérien OLAH, et d'au moins un autre gène cible bactérien choisi parmi FAM20A, IL1R2, MMP8, RETN et SLC1A2, de préférence parmi FAM20A, IL1R2 et MMP8.

13. Méthode selon la revendication 5, **caractérisée en ce que** l'étape (a) comprend ou consiste en la mesure d'au moins un gène cible viral choisi parmi IFI27, SIGLEC1, ISG15, HERC6, RSAD2, OAS1, IFIT1 et IFI44L, du gène cible bactérien FAM20A, et d'au moins un autre gène cible bactérien choisi parmi OLAH, IL1R2, MMP8, RETN et SLC1A2, de préférence parmi OLAH, IL1R2 et MMP8.

14. Méthode selon la revendication 5, **caractérisée en ce que** l'étape (a) de mesure comprend ou consiste en la mesure de l'expression du gène cible viral IFI27, d'un autre gène cible viral choisi parmi HERC6, SIGLEC1, IFI44L et ISG15, du gène cible bactérien FAM20A et d'un autre gène cible bactérien choisi parmi OLAH et IL1R2.

15. Méthode selon la revendication 9, **caractérisée en ce que** l'étape (a) comprend ou consiste en la mesure de l'expression du gène cible viral IFI27, de deux autres gènes cibles viraux choisis parmi SIGLEC1, HERC6, OAS1 et IFIT1, du gène cible bactérien FAM20A et d'un autre gène cible bactérien choisi parmi OLAH, IL1R2 et MMP8.

16. Méthode selon la revendication 5, **caractérisée en ce que** l'étape (a) de mesure comprend ou consiste en la mesure de l'expression des gènes cibles viraux IFI27 et SIGLEC1, d'un autre gène cible viral choisi parmi IFIT1, HERC6, ISG15 et ISG15, des gènes cibles bactériens FAM20A et MMP8, et d'un autre gène cible bactérien choisi parmi OLAH et IL1R2.

17. Méthode selon la revendication 5, **caractérisée en ce que** l'étape (a) de mesure comprend ou consiste en la mesure de l'expression des gènes cibles viraux IFI27 et SIGLEC1, et de deux autres gènes cibles viraux choisis parmi IFIT1, OAS1, HERC6 et ISG15, des gènes cibles bactériens FAM20A et MMP8, et d'un autre gène cible bactérien choisi parmi OLAH et IL1R2.

18. Méthode selon la revendication 1, **caractérisé en ce que** l'étape (a) consiste en la mesure d'une combinaison de gène cibles viraux et bactériens choisie parmi les combinaisons de gènes cibles listés dans les tableaux 6 à 12 et qui présentent une AUC du modèle pour la détermination de la nature virale ou bactérienne de l'infection d'au moins 0,90.

19. Méthode selon l'une des revendications 5 à 18, **caractérisée en ce que** la valeur d'expression de référence prédéterminée des gènes cibles correspond à l'expression respectives desdits gènes cibles dans un échantillon biologique de référence obtenu chez un sujet ayant une infection bactérienne et **en ce qu'**il est conclu à une infection de nature virale lorsque les résultats de comparaison d'expression des gènes cibles mettent en évidence au moins une variation d'expression choisie parmi :

   - une surexpression de SIGLEC1,
   - une surexpression de ISG15,
   - une surexpression de HERC6,
   - une surexpression de RSAD2,
   - une surexpression de IFI27,
   - une surexpression de OAS1,
   - une surexpression de IFIT1, et
   - une surexpression de IFI44L,

   et au moins une autre variation d'expression choisie parmi :

   - une sous expression de SLC1A2,
   - une sous expression de IL1R2,
   - une sous expression de FAM20A,
   - une sous expression de OLAH,
   - une sous expression de RETN, et
   - une sous expression de MMP8.

20. Méthode selon l'une des revendications 5 à 18, **caractérisée en ce que** la valeur d'expression de référence prédéterminée desdits gènes cibles correspond à l'expression respectives desdits gènes cibles dans un échantillon biologique de référence obtenu chez un sujet ayant une infection virale et **en ce qu'**il est conclu à une infection de nature bactérienne lorsque les résultats de comparaison d'expression des gènes cibles mettent en en évidence au moins une variation d'expression choisie parmi :

   - une sous expression de SIGLEC1,
   - une sous expression de ISG15,

- une sous expression de HERC6,
- une sous expression de RSAD2,
- une sous expression de IFI27,
- une sous expression de OAS1,
- une sous expression de IFIT1, et
- une sous expression de IFI44L,

et au moins une autre variation d'expression choisie parmi :

- une surexpression de SLC1A2,
- une surexpression de IL1R2,
- une surexpression de FAM20A,
- une surexpression de OLAH,
- une surexpression de RETN, et
- une surexpression de MMP8

21. Méthode selon l'une des revendications 1 à 20, **caractérisée en ce qu'**elle comprend en outre une étape (a') de mesure de l'expression d'au moins un gène cible additionnel choisi parmi PI3, EBI3, ADGRE1 et S100P, une étape (b') de comparaison des expressions mesurées à l'étape (a') avec des valeurs d'expression de références desdits gènes cibles additionnels.

22. Méthode selon l'une des revendications 1 à 21, **caractérisée en ce que** la mesure de l'expression des gènes cibles est réalisée au niveau ARNm.

23. Méthode selon l'une des revendications 1 à 22, **caractérisée en ce que** la mesure de la variation d'expression est réalisée par amplification via une RT-PCR, de préférence une RT-PCR quantitative, ou une PCR nichée.

24. Méthode selon l'une des revendications 1 à 23, **caractérisée en ce que** l'expression est normalisée par rapport à l'expression d'un ou plusieurs gènes de ménage, de préférence choisis parmi DECR1, HPRT1, PPIB, GAPDH, et ACTB.

25. Méthode selon l'une des revendications 1 à 24, **caractérisée en ce que** l'échantillon biologique est issu d'un sujet qui est un enfant, de préférence un enfant de moins de 4 ans, et de préférence encore un enfant de moins de 2 ans.

26. Méthode selon l'une des revendications 1 à 25, **caractérisée en ce que** l'échantillon biologique est un échantillon de sang, de préférence un échantillon de sang total.

27. Kit pour la mesure *in vitro* ou *ex vivo* dans un échantillon biologique de l'expression génique, ledit kit comprenant des moyens de détermination de la variation du niveau d'expression d'au moins un gène cible viral choisi parmi IFI27, SIGLEC1, ISG15, HERC6, RSAD2, OAS1, IFIT1, IFI44L, et d'au moins un gène cible bactérien choisi parmi OLAH, FAM20A, IL1R2, MMP8, RETN et SLC1A2, lesdits moyens étant de préférence des amorces ou des sondes.

28. Utilisation du kit selon la revendication 27 pour déterminer la nature virale ou bactérienne d'une infection à partir d'un échantillon biologique d'un sujet.

**FIGURE 1**

**FIGURE 2**

Europäisches
Patentamt

European
Patent Office

Office européen
des brevets

# RAPPORT DE RECHERCHE EUROPEENNE

Numéro de la demande

EP 22 20 5313

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2022/008829 A1 (BIOMERIEUX SA [FR]; BIOASTER [FR]; HOSPICES CIVILS LYON [FR]) 13 janvier 2022 (2022-01-13) * claims 1-3, 12-23, p. 78 l. 15-20 * | 1 | INV. C12Q1/6883 |
| X | US 2017/022568 A1 (GHAZAL PETER [GB] ET AL) 26 janvier 2017 (2017-01-26) * [0008],[0174]-[0181] * | 1 | |
| X | WO 2021/205461 A1 (MEMED DIAGNOSTICS LTD [IL]) 14 octobre 2021 (2021-10-14) | 1-5, 8-17, 19-28 | |
| Y | * p. 7 l. 15-21, p. 4 l. 21-29, p. 67 l. 6-12, p. 67 l. 20-26, Tab. 12, 14, 15 * | 7 | |
| A | US 2022/112542 A1 (KHATRI PURVESH [US] ET AL) 14 avril 2022 (2022-04-14) * claims 1-7 * | 1-28 | |
| X | RAJIT K BASU ET AL: "Identification of candidate serum biomarkers for severe septic shock-associated kidney injury via microarray", CRITICAL CARE, BIOMED CENTRAL LTD LONDON, GB, vol. 15, no. 6, 18 novembre 2011 (2011-11-18), page R273, XP021112690, ISSN: 1364-8535, DOI: 10.1186/CC10554 * abstract and Tab. 2 * | 1,6,18 | DOMAINES TECHNIQUES RECHERCHES (IPC) C12Q |
| X | US 2020/131574 A1 (HALL JUDITH [GB] ET AL) 30 avril 2020 (2020-04-30) | 1,27,28 | |
| Y | * [0010], [0085], [0086], [0022], [1021] * | 7 | |

Le présent rapport a été établi pour toutes les revendications

| Lieu de la recherche | Date d'achèvement de la recherche | Examinateur |
|---|---|---|
| Munich | 22 mars 2023 | Lapopin, Laurence |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 22 20 5313

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-03-2023

| Document brevet cité au rapport de recherche | | Date de publication | Membre(s) de la famille de brevet(s) | | Date de publication |
|---|---|---|---|---|---|
| WO 2022008829 | A1 | 13-01-2022 | FR | 3112208 A1 | 07-01-2022 |
| | | | WO | 2022008829 A1 | 13-01-2022 |
| US 2017022568 | A1 | 26-01-2017 | EP | 3129496 A1 | 15-02-2017 |
| | | | ES | 2781703 T3 | 04-09-2020 |
| | | | PT | 3129496 T | 07-04-2020 |
| | | | US | 2017022568 A1 | 26-01-2017 |
| | | | WO | 2015155517 A1 | 15-10-2015 |
| WO 2021205461 | A1 | 14-10-2021 | AUCUN | | |
| US 2022112542 | A1 | 14-04-2022 | AU | 2020247700 A1 | 14-10-2021 |
| | | | CA | 3134617 A1 | 01-10-2020 |
| | | | CN | 113785074 A | 10-12-2021 |
| | | | EP | 3947735 A1 | 09-02-2022 |
| | | | IL | 286559 A | 31-10-2021 |
| | | | JP | 2022528343 A | 10-06-2022 |
| | | | KR | 20210143854 A | 29-11-2021 |
| | | | US | 2022112542 A1 | 14-04-2022 |
| | | | WO | 2020197659 A1 | 01-10-2020 |
| US 2020131574 | A1 | 30-04-2020 | AU | 2017334293 A1 | 04-04-2019 |
| | | | CA | 3036411 A1 | 05-04-2018 |
| | | | EP | 3519594 A2 | 07-08-2019 |
| | | | EP | 4137584 A1 | 22-02-2023 |
| | | | US | 2020131574 A1 | 30-04-2020 |
| | | | WO | 2018060739 A2 | 05-04-2018 |

EPO FORM P0460

Pour tout renseignement concernant cette annexe : voir Journal Officiel de l'Office européen des brevets, No.12/82

**EP 4 365 308 A1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018011316 A
- WO 2008140568 A
- WO 2017093672 A

**Littérature non-brevet citée dans la description**

- **TAKEUCHI O ; AKIRA S.** Pattern recognition receptors and inflammation. *Cell,* 2010, vol. 140 (6), 805-820
- **RAMILO O ; MEJÍAS A.** Shifting the paradigm: Host gene signatures for diagnosis of infectious diseases. *Cell Host Microbe,* 2009, vol. 6 (3), 199-200
- **FAUCI, A. S et al.** The perpetual challenge of antimicrobial resistance. *JAMA,* 2014, vol. 311, 1853-1854
- **HU et al.** Gene expression profiles in febrile children with defined viral and bacterial infection. *PNAS,* 2013, vol. 110 (31), 12792-12797
- **ERIC D. BROWN et al.** *Antibacterial drug discovery in the resistance era,* 2016
- **EPHRAIM L. TSALIK et al.** Discriminating bacterial and viral infection using a rapid host gene expression test. *Critical Care Medecine,* 2021, vol. 49 (10
- **MARK A. PORITZ et al.** FilmArray, an automated Nested Multiplex PCR System for Multi-Pathogen Detection: development and application to respiratory tract infection. *PLoS ONE,* 2011, vol. 6 (10), e26047
- **WILLIAM M. SCHNEIDER et al.** Interferon-stimulated genes: a complex web of host response. *Annu Rev Immunol.,* 2014, vol. 32, 513-545
- **EMILY YANG et al.** All about the RNA : interferon-stimulated genes that interfere with viral RNA processes. *Front. Immunol.,* 2020, vol. 11, 605024
- **TRINE H. MOGENSEN et al.** Molecular pathways in virus-induced cytokine production. *Microbiology and molecular biology,* Mars 2001, 131-150
- **KAWASAKI et al.** Toll-like receptor signaling pathway. *Front. Immunol., Sec. Cancer Immunity and Immunotherapy,* 2014
- **HOCHRAINER et al.** The human HERC family of ubiquitin ligases: novel members, genomic organization, expression profiling, and evolutionary aspects. *Genomics,* 2005, vol. 85, 153-164